# EUROPEAN PATENT APPLICATION

(11) **EP 1 340 818 A1**
(43) Date of publication of application: **03.09.2003**
(21) Application number: 02004551.4
(22) Date of filing: 27.02.2002
(51) Int. Cl.: C12Q 1/68

(54) **Method and nucleic acids for the analysis of a colon cell proliferative disorder**

(71) Applicant: Epigenomics AG, 10435 Berlin (DE)
(72) Inventor: Adorjan, Peter, 10437 Berlin (DE); Burger, Matthias, 10967 Berlin (DE); Maier, Sabine, 12163 Berlin (DE); Nimmrich, Inko, 10407 Berlin (DE); Becker, Evelyne, 10437 Berlin (DE); Lesche, Ralf, 10439 Berlin (DE); Rujan, Tamas, 13189 Berlin (DE); Schmitt, Armin, 96149 Breitengüssbach (DE)
(74) Representative: Schohe, Stefan

(57) **Abstract**

The present invention relates to modified and genomic sequences, to oligonucleotides and/or PNA-oligomers for detecting the cytosine methylation state of genomic DNA, as well as to a method for ascertaining genetic and/or epigenetic parameters of genes for use in the differentiation, diagnosis, treatment and/or monitoring of colon cell proliferative disorders, or the predisposition to colon cell proliferative disorders.

## Description

### Field of the Invention

The levels of observation that have been studied by the methodological developments of recent years in molecular biology, are the genes themselves, the translation of these genes into RNA, and the resulting proteins. The question of which gene is switched on at which point in the course of the development of an individual, and how the activation and inhibition of specific genes in specific cells and tissues are controlled is correlatable to the degree and character of the methylation of the genes or of the genome. In this respect, pathogenic conditions may manifest themselves in a changed methylation pattern of individual genes or of the genome.

Colorectal cancer is the fourth leading cause of cancer mortality in men and women, although ranking third in frequency in men and second in women. The 5-year survival rate is 61% over all stages with early detection being a prerequisite for curative therapy of the disease. Up to 95% of all colorectal cancers are adenocarcinomas of varying differentiation grades .

Sporadic colon cancer develops in a multistep process starting with the pathologic transformation of normal colonic epithelium to an adenoma which consecutively progresses to invasive cancer. The progression rate of benign colonic adenomas depends strongly on their histologic appearance: whereas tubular-type adenomas tend to progress to malignant tumors very rarely, villous adenomas, particularly if larger than 2 cm in diameter, have a significant malignant potential.
During progression from benign proliferative lesions to malignant neoplasms several genetic and epigenetic alterations occur. Somatic mutation of the APC gene seems to be one of the earliest events in 75 to 80% of colorectal adenomas and carcinomas. Activation of K-RAS is thought to be a critical step in the progression towards a malignant phenotype. Consecutively, mutations in other oncogenes as well as alterations leading to inactivation of tumor suppressor genes accumulate.
Aberrant DNA methylation within CpG islands is among the earliest and most common alterations in human malignancies leading to abrogation or overexpression of a broad spectrum of genes. In addition, abnormal methylation has been shown to occur in CpG rich regulatory elements in intronic and coding parts of genes for certain tumors. In contrast to the specific hypermethylation of tumor suppressor genes, an overall hypomethylation of DNA can be observed in tumor cells. This decrease in global methylation can be detected early, far before the development of frank tumor formation. Also, correlation between hypomethylation and increased gene expression was reported for many oncogenes. In colon cancer, aberrant DNA methylation constitutes one of the most prominent alterations and inactivates many tumor suppressor genes such as p1 4ARF, p16INK4a, THBS1, MINT2, and MINT31 and DNA mismatch repair genes such as hMLH1.
In the molecular evolution of colorectal cancer, DNA methylation errors have been suggested to play two distinct roles. In normal colonic mucosa cells, methylation errors accumulate as a function of age or as time-dependent events predisposing these cells to neoplastic transformation. For example, hypermethylation of several loci could be shown to be already present in adenomas, particularly in the tubulovillous and villous subtype. At later stages, increased DNA methylation of CpG islands plays an important role in a subset of tumors affected by the so called CpG island methylator phenotype (CIMP). Most CIMP+ tumors, which constitute about 15% of all sporadic colorectal cancers, are characterized by microsatellite instability (MIN) due to hypermethylation of the hMLH1 promoter and other DNA mismatch repair genes. By contrast, CIMP- colon cancers evolve along a more classic genetic instability pathway (CIN), with a high rate of p53 mutations and chromosomal changes.
However, the molecular subtypes do not only show varying frequencies regarding molecular alterations. According to the presence of either micro satellite instability or chromosomal aberrations, colon cancer can be subclassified into two classes, which also exhibit significant clinical differences. Almost all MIN tumors originate in the proximal colon (ascending and transversum), whereas 70% of CIN tumors are located in the distal colon and rectum. This has been attributed to the varying prevalence of different carcinogens in different sections of the colon. Methylating carcinogens, which constitute the prevailing carcinogen in the proximal colon have been suggested to play a role in the pathogenesis of MIN cancers, whereas CIN tumors are thought to be more frequently caused by adduct-forming carcinogens, which occur more frequently in distal parts of the colon and rectum. Moreover, MIN tumors have a better prognosis than do tumors with a CIN phenotype and respond better to adjuvant chemotherapy.
The identification of markers for the differentiation of colon carcinoma as well as for early detection are main goals of current research.

5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing since 5-methylcytosine has the same base pairing behavior as cytosine. Moreover, the epigenetic information carried by 5-methylcytosine is completely lost during PCR amplification.

A relatively new and currently the most frequently used method for analyzing DNA for 5-methylcytosine is based upon the specific reaction of bisulfite with cytosine which, upon subsequent alkaline hydrolysis, is converted to uracil which corresponds to thymidine in its base pairing behavior. However, 5-methylcytosine remains unmodified under these conditions. Consequently, the original DNA is converted in such a manner that methylcytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited. In terms of sensitivity, the prior art is defined by a method which encloses the DNA to be analyzed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyze individual cells, which illustrates the potential of the method. However, currently only individual regions of a length of up to approximately 3000 base pairs are analyzed, a global analysis of cells for thousands of possible methylation events is not possible. However; this method cannot reliably analyze very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res. 1998, 26, 2255.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridization has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705 and WO 95/15373.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionization of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionization Mass Spectrometry. Current Innovations and Future Trends. 1995, 1; 147-57). The sensitivity to nucleic acids is approximately 100 times worse than to peptides and decreases disproportionally with increasing fragment size. For nucleic acids having a multiply negatively charged backbone, the ionization process via the matrix is considerably less efficient. In MALDI-TOF spectrometry, the selection of the matrix plays an eminently important role. For the desorption of peptides, several very efficient matrixes have been found which produce a very fine crystallization. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8):1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA is obtained from DNA of cell, tissue or other test samples using standard methods. This standard methodology is found in references such as Sambrook, Fritsch and Maniatis eds., Molecular Cloning: A Laboratory Manual, 1989.

### Description

The invention provide a method for the analysis of biological samples for features associated with the development of colon cell proliferative disorders, characterised in that the nucleic acid of at least one member of the group comprising MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 is/are contacted with a reagent or series of reagents capable of distinguishing between methylated and non methylated CpG dinucleotides within the genomic sequence of interest.

The present invention makes available a method for ascertaining genetic and/or epigenetic parameters of genomic DNA. The method is for use in the improved diagnosis, treatment and monitoring of colon cell proliferative disorders, more specifically by enabling the improved identification of and differentiation between subclasses of said disorder and the genetic predisposition to said disorders. The invention presents improvements over the state of the art in that it enables a highly specific classification of colon carcinomas, thereby allowing for improved and informed treatment of patients.

In a particularly preferred embodiment the present invention makes available methods and nucleic acids that allow the differentiation between colon carcinoma, colon adenoma and normal colon tissue.
Furthermore, the method enables the analysis of cytosine methylations and single nucleotide polymorphisms.

The genes that form the basis of the present invention can be used to form a "gene panel", i.e. a collection comprising the particular genetic sequences of the present invention and/or their respective informative methylation sites. The formation of gene panels allow for a quick and specific analysis of the disorders they are related with. The gene panels described in this invention can be used with surprisingly high efficiency for the diagnosis, treatment and monitoring of and the analysis of colon cell proliferative disorders as described herein. The use of multiple CpG sites from a diverse array of genes, allows for a relatively high degree of sensitivity and specificity in comparison to single gene diagnostic and detection tools. Furthermore, the panel as described herein may be adapted for use in the analysis of many aspects of colon cell proliferative disorders.

### In a preferred embodiment, the method comprises the following steps:

In the first step of the method the genomic DNA sample must be isolated from tissue or cellular sources. Such sources may include colon tissue samples, cell lines, histological slides, body fluids, or tissue embedded in paraffin. Extraction may be by means that are standard to one skilled in the art, these include the use of detergent lysates, sonification and vortexing with glass beads. Once the nucleic acids have been extracted the genomic double stranded DNA is used in the analysis.

In a preferred embodiment the DNA may be cleaved prior to the next step of the method, this may be by any means standard in the state of the art, in particular, but not limited to, with restriction endonucleases.

In the second step of the method, the genomic DNA sample is treated in such a manner that cytosine bases which are unmethylated at the 5'-position are converted to uracil, thymine, or another base which is dissimilar to cytosine in terms of hybridization behaviour. This will be understood as 'pretreatment' hereinafter.

The above described treatment of genomic DNA is preferably carried out with bisulfite (sulfite, disulfite) and subsequent alkaline hydrolysis which results in a conversion of non-methylated cytosine nucleobases to uracil or to another base which is dissimilar to cytosine in terms of base pairing behaviour. If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA is then used for the detection of methylated cytosines.

Fragments of the pretreated DNA are amplified, using sets of primer oligonucleotides according to SEQ ID NO: 389 to SEQ ID NO: 518, and a, preferably heat-stable, polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

The method may also be enabled by the use of alternative primers, the design of such primers is obvious to one skilled in the art. These should include at least two oligonucleotides whose sequences are each reverse complementary or identical to an at least 18 base-pair long segment of the base sequences specified in the appendix (SEQ ID NO: 133 through SEQ ID NO: 388). Said primer oligonucleotides are preferably characterized in that they do not contain any CpG dinucleotides. In a particularly preferred embodiment of the method, the sequence of said primer oligonucleotides are designed so as to selectively anneal to and amplify, only the colon tissue specific DNA of interest, thereby minimizing the amplification of background or non relevant DNA. In the context of the present invention, background DNA is taken to mean genomic DNA which does not have a relevant tissue specific methylation pattern, in this case, the relevant tissue being colon, both healthy and diseased.

According to the present invention, it is preferred that at least one primer oligonucleotide is bound to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminum, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualized by means of matrix assisted laser desorption/ionization mass spectrometry (MALDI) or using electron spray mass spectrometry (ESI).

The amplificates obtained in the second step of the method are subsequently hybridized to an array or a set of oligonucleotides and/or PNA probes. In this context, the hybridization preferably takes place in the manner described as follows. The set of probes used during the hybridization is preferably composed of at least 10 oligonucleotides or PNA-oligomers. However, it is understood and as well claimed, that the process can be conducted using only one Oligonucleotide or PNA probe. In the process, the amplificates hybridize to oligonucleotides previously bonded to a solid phase. In a particularly preferred embodiment, the oligonucleotides are taken from the group comprising SEQ ID NO: 519 to SEQ ID NO: 1030. In a further preferred embodiment the oligonucleotides are taken from the group comprising SEQ ID NO: 895 to SEQ ID NO: 1030. The non-hybridized fragments are subsequently removed. Said oligonucleotides contain at least one base sequence having a length of 10 nucleotides which is reverse complementary or identical to a segment of the base sequences specified in the appendix, the segment containing at least one CpG or TpG dinucleotide. In a further preferred embodiment the cytosine of the CpG dinucleotide, or in the case of TpG, the thiamine, is the 5^{th} to 9^{th} nucleotide from the 5'-end of the 10-mer. One oligonucleotide exists for each CpG or TpG dinucleotide.

In the fifth step of the method, the non-hybridized amplificates are removed.

In the final step of the method, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualized by means of matrix assisted laser desorption/ionization mass spectrometry (MALDI) or using electron spray mass spectrometry (ESI). The produced fragments may have a single positive or negative net charge for better detectability in the mass spectrometer.

The aforementioned method is preferably used for ascertaining genetic and/or epigenetic parameters of genomic DNA.

In order to enable this method, the invention further provides the modified DNA of genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 as well as oligonucleotides and/or PNA-oligomers for detecting cytosine methylations within said genes. The present invention is based on the discovery that genetic and epigenetic parameters and, in particular, the cytosine methylation patterns of genomic DNA are particularly suitable for improved diagnosis, treatment and monitoring of colon cell proliferative disorders. Furthermore, the invention enables the differentiation between different subclasses of colon cell proliferative disorders or detection of a predisposition to colon cell proliferative disorders.

The nucleic acids according to the present invention can be used for the analysis of genetic and/or epigenetic parameters of genomic DNA.

This objective is achieved according to the present invention using a nucleic acid containing a sequence of at least 18 bases in length of the pretreated genomic DNA according to one of SEQ ID NO: 133 through SEQ ID NO: 388 and sequences complementary thereto.

The-modified nucleic acid could heretofore not be connected with the ascertainment of disease relevant genetic and epigenetic parameters.

The object of the present invention is further achieved by an oligonucleotide or oligomer for the analysis of pretreated DNA, for detecting the genomic cytosine methylation state, said oligonucleotide containing at least one base sequence having a length of at least 10 nucleotides which hybridizes to a pretreated genomic DNA according to SEQ ID NO: 133 through to SEQ ID NO: 388. The oligomer probes according to the present invention constitute important and effective tools which, for the first time, make it possible to ascertain specific genetic and epigenetic parameters during the analysis of biological samples for features associated with the development of colon cell proliferative disorders. Said oligonucleotides allow the improved diagnosis, treatment and monitoring of colon cell proliferative disorders and detection of the predisposition to said disorders. Furthermore, they allow the differentiation of different subclasses of colon cell proliferative disorders. The base sequence of the oligomers preferably contains at least one CpG or TpG dinucleotide. The probes may also exist in the form of a PNA (peptide nucleic acid) which has particularly preferred pairing properties. Particularly preferred are oligonucleotides according to the present invention in which the cytosine of the CpG dinucleotide is the 5^{th} - 9^{th} nucleotide from the 5'-end of the 13-mer; in the case of PNA-oligomers, it is preferred for the cytosine of the CpG dinucleotide to be the 4^{th} - 6^{th} nucleotide from the 5'-end of the 9-mer.

The oligomers according to the present invention are normally used in so called "sets" which contain at least one oligomer for each of the CpG dinucleotides within SEQ ID NO: 133 through SEQ ID NO: 388. Preferred is a set which contains at least one oligomer for each of the CpG dinucleotides, from SEQ ID NO: 519 through SEQ ID NO: 1030. Further preferred is a set comprising SEQ ID NO: 895 to SEQ ID NO: 1030.

In the case of the sets of oligonucleotides according to the present invention, it is preferred that at least one oligonucleotide is bound to a solid phase. It is further preferred that all the oligonucleotides of one set are bound to a solid phase.

The present invention moreover relates to a set of preferably at least 10 n (oligonucleotides and/or PNA-oligomers) used for detecting the cytosine methylation state of genomic DNA using treated versions of said genomic DNA (according to SEQ ID NO: 133 through SEQ ID NO: 388 and sequences complementary thereto). However, it is understood and as well claimed, that the process can be conducted using only one Oligonucleotide or PNA oligomer. These probes enable improved diagnosis, treatment and monitoring of colon cell proliferative disorders. In particular they enable the differentiation between different sub classes of colon cell proliferative disorders and the detection of a predisposition to said disorders. In a particularly preferred embodiment the set comprises SEQ ID NO: 519 to SEQ ID NO: 1030.

The set of oligomers may also be used for detecting single nucleotide polymorphisms (SNPs) using pretreated genomic DNA according to one of SEQ ID NO: 133 through SEQ ID NO: 388.

According to the present invention, it is preferred that an arrangement of different oligonucleotides and/or PNA-oligomers (a so-called "array") made available by the present invention is present in a manner that it is likewise bound to a solid phase. This array of different oligonucleotide- and/or PNA-oligomer sequences can be characterized in that it is arranged on the solid phase in the form of a rectangular or hexagonal lattice. The solid phase surface is preferably composed of silicon, glass, polystyrene, aluminum, steel, iron, copper, nickel, silver, or gold. However, nitrocellulose as well as plastics such as nylon which can exist in the form of pellets or also as resin matrices are suitable alternatives.

Therefore, a further subject matter of the present invention is a method for manufacturing an array fixed to a carrier material for the improved diagnosis, treatment and monitoring of colon cell proliferative disorders, the differentiation between different subclasses of colon cell proliferative disorders and/or detection of the predisposition to colon cell proliferative disorders. In said method at least one oligomer according to the present invention is coupled to a solid phase. Methods for manufacturing such arrays are known, for example, from Patent US 5,744,305 by means of solid-phase chemistry and photolabile protecting groups.

A further subject matter of the present invention relates to a DNA chip for the improved diagnosis, treatment and monitoring of colon cell proliferative disorders. Furthermore the DNA chip enables detection of the predisposition to colon cell proliferative disorders and the differentiation between different subclasses of colon cell proliferative disorders. The DNA chip contains at least one nucleic acid according to the present invention. DNA chips are known, for example, in Patent US 5,837,832.

Moreover, a subject matter of the present invention is a kit which may be composed, for example, of a bisulfite-containing reagent, a set of primer oligonucleotides containing at least two oligonucleotides whose sequences in each case correspond or are complementary to a 18 base long segment of the base sequences specified in the appendix (SEQ ID NO: 133 through SEQ ID NO: 388), oligonucleotides and/or PNA-oligomers as well as instructions for carrying out and evaluating the described method. However, a kit along the lines of the present invention can also contain only part of the aforementioned components.

The oligomers according to the present invention or arrays thereof as well as a kit according to the present invention are intended to be used for the improved diagnosis, treatment and monitoring of colon cell proliferative disorders. Furthermore the use of said inventions extends to the differentiation between different subclasses of colon cell proliferative disorders and detection of the predisposition to colon cell proliferative disorders. According to the present invention, the method is preferably used for the analysis of important genetic and/or epigenetic parameters within genomic DNA, in particular for use in improved diagnosis, treatment and monitoring of colon cell proliferative disorders, detection of the predisposition to said disorders and the differentiation between subclasses of said disorders.

The methods according to the present invention are used, for example, for improved diagnosis, treatment and monitoring of colon cell proliferative disorders progression, detection of the predisposition to said disorders and the differentiation between subclasses of said disorders.

A further embodiment of the invention is a method for the analysis of the methylation status of genomic DNA without the need for pretreatment. In the first step of the method the genomic DNA sample must be isolated from tissue or cellular sources. Such sources may include cell lines, histological slides, body fluids, or tissue embedded in paraffin. Extraction may be by means that are standard to one skilled in the art, these include the use of detergent lysates, sonification and vortexing with glass beads. Once the nucleic acids have been extracted the genomic double stranded DNA is used in the analysis.

In a preferred embodiment the DNA may be cleaved prior to the treatment, this may be any means standard in the state of the art, in particular with restriction endonucleases. In the second step, the DNA is then digested with one or more methylation sensitive restriction enzymes. The digestion is carried out such that hydrolysis of the DNA at the restriction site is informative of the methylation status of a specific CpG dinucleotide.

In the third step the restriction fragments are amplified. In a preferred embodiment this is carried out using a polymerase chain reaction.

In the final step the amplificates are detected. The detection may be by any means standard in the art, for example, but not limited to, gel electrophoresis analysis, hybridisation analysis, incorporation of detectable tags within the PCR products, DNA array analysis, MALDI or ESI analysis.

The present invention moreover relates to the diagnosis and/or prognosis of events which are disadvantageous or relevant to patients or individuals in which important genetic and/or epigenetic parameters within genomic DNA, said parameters obtained by means of the present invention may be compared to another set of genetic and/or epigenetic parameters, the differences serving as the basis for the diagnosis and/or prognosis of events which are disadvantageous or relevant to patients or individuals.

In the context of the present invention the term "hybridization" is to be understood as a bond of an oligonucleotide to a completely complementary sequence along the lines of the Watson-Crick base pairings in the sample DNA, forming a duplex structure.

In the context of the present invention, "genetic parameters" are mutations and polymorphisms of genomic DNA and sequences further required for their regulation. To be designated as mutations are, in particular, insertions, deletions, point mutations, inversions and polymorphisms and, particularly preferred, SNPs (single nucleotide polymorphisms).

In the context of the present invention "methylation state analysis" is taken to mean the analysis of cytosines within a nucleic acid in order to ascertain whether they are methylated or not.

In the context of the present invention, "epigenetic parameters" are, in particular, cytosine methylations and further modifications of DNA bases of genomic DNA and sequences further required for their regulation. Further epigenetic parameters include, for example, the acetylation of histones which, cannot be directly analyzed using the described method but which, in turn, correlates with the DNA methylation.

In the following, the present invention will be explained in greater detail on the basis of the sequences and examples without being limited thereto.

SEQ ID NO: 1 to SEQ ID NO: 64 represent 5' and/or regulatory regions of the genomic DNA of genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1. EGFR , ERBB2, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSHS. These sequences are derived from the ensembl database (date 01.10.2001) (http://www.ensembl.org) and will be taken to include all minor variations of the sequence material which are currently unforeseen, for example, but not limited to, minor deletions and SNPs.

SEQ ID 133 to 388 exhibit the pretreated sequences of DNA derived from genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1. MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1. CEA, MB, PCNA, CDC2 , ESR1, CASP8, RASSF1, MSH4, MSH5. These sequences will be taken to include all minor variations of the sequence material which are currently unforeseen, for example, but not limited to, minor deletions and SNPs.

SEQ ID NO: 389 to SEQ ID NO: 518 exhibit the sequences of primer oligonucleotides for the amplification of pretreated DNA according to Sequence ID NO: 133 to SEQ ID NO: 388.

SEQ ID NO: 65 to SEQ ID NO: 132 exhibit the sequences of oligomers which are useful for the analysis of CpG positions within genomic DNA according to SEQ ID NO: 1 to SEQ ID NO: 64.

SEQ ID NO: 519 to SEQ ID NO: 1030 exhibit the sequences of oligomers which are useful for the analysis of the methylation status of CpG positions within genomic DNA according to SEQ ID NO: 1 to SEQ ID NO: 64 after treatment of said genomic DNA with bisulfite.

SEQ ID NO: 895 to SEQ ID NO: 1030 exhibit the sequences of oligomers which are particularly useful for the analysis of CpG positions within genomic DNA according to SEQ ID NO: 1 to SEQ ID NO: 64, after treatment of said with bisulfite and are subject to a preferred embodiment of this invention.

### Examples 1 and 2: Digital Phenotype

In the following examples, multiplex PCR was carried out upon tissue samples originating from colon adenomas or colon carcinoma. Multiplex PCR was also carried out upon healthy colon tissue. Each sample was treated in the manner described below in Example 1 in order to deduce the methylation status of CpG positions, the CpG methylation information for each sample was collated and then used in an analysis, as detailed in Example 2. An alternative method for the analysis of CpG methylation status is described in Example 3.

### Example 1

In the first step the genomic DNA was isolated from the cell samples using the Wizzard kit from (Promega).

The isolated genomic DNA from the samples are treated using a bisulfite solution (hydrogen sulfite, disulfite). The treatment is such that all non methylated cytosines within the sample are converted to thiamidine, conversely 5-methylated cytosines within the sample remain unmodified.

The treated nucleic acids were then amplified using multiplex PCRs, amplifying 8 fragments per reaction with Cy5 fluorescently labelled primers. PCR primers used are described in Table 1. PCR conditions were as follows.

### Reaction solution:

10 ng bisulfite treated DNA
3,5 mM MgC12
400 µM dNTPs
2 pmol each primer
1 U Hot Star Taq (Qiagen)

Forty cycles were carried out as follows. Denaturation at 95°C for 15 min, followed by annealirig at 55°C for 45 sec., primer elongation at 65°C for 2 min. A final elongation at 65°C was carried out for 10 min.

All PCR products from each individual sample were then hybridised to glass slides carrying a pair of immobilised oligonucleotides for each CpG position under analysis. Each of these detection oligonucleotides was designed to hybridise to the bisulphite converted sequence around one CpG site which was either originally unmethylated (TG) or methylated (CG). See Table 2 for further details of all hybridisation oligonucleotides used (both informative and non-informative) Hybridisation conditions were selected to allow the detection of the single nucleotide differences between the TG and CG variants.

5 µl volume of each multiplex PCR product was diluted in 10 x Ssarc buffer (10 x Ssarc:230 ml 20 x SSC, 180 ml sodium lauroyl sarcosinate solution 20%, dilute to 1000 ml with dH20). The reaction mixture was then hybridised to the detection oligonucleotides as follows. Denaturation at 95°C, cooling down to 10 °C, hybridisation at 42°C overnight followed by washing with 10 x Ssarc and dH2O at 42°C.

Fluorescent signals from each hybridised oligonucleotide were detected using genepix scanner and software. Ratios for the two signals (from the CG oligonucleotide and the TG oligonucleotide used to analyse each CpG position) were calculated based on comparison of intensity of the fluorescent signals.

### Example 2

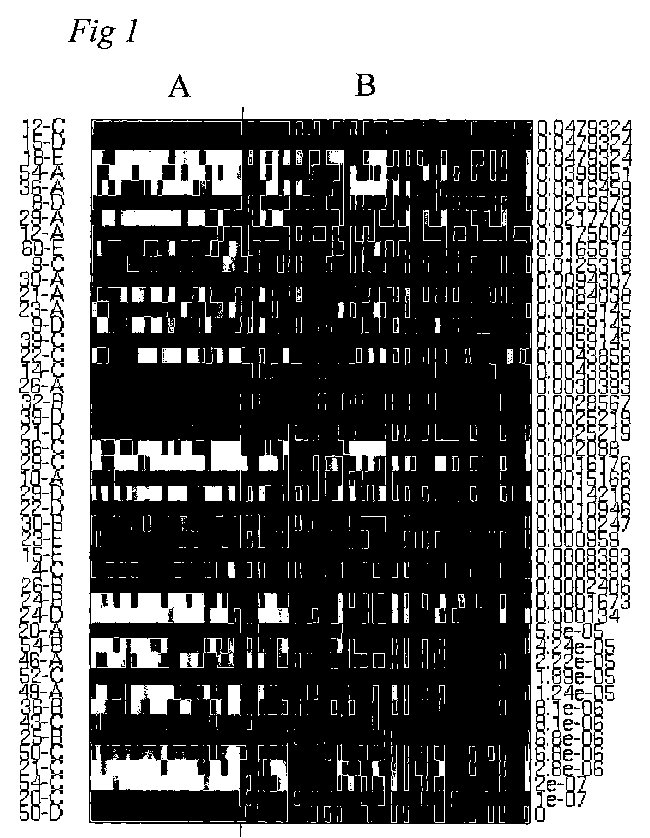

The data obtained according to Example 1 is then sorted into a ranked matrix (as shown in Figures 1 to 4) according to CpG methylation differences between the two classes of tissues, using an algorithm. The most significant CpG positions are at the bottom of the matrix with significance decreasing towards the top. Black indicates total methylation at a given CpG position, white represents no methylation at the particular position, with degrees of methylation represented in gray, from light (low proportion of methylation) to dark (high proportion of methylation). Each row represents one specific CpG position within a gene and each column shows the methylation profile for the different CpGs for one sample. On the left side a CpG and gene identifier is shown this may be cross referenced with the accompanying tables (Table 1 and 7) in order to ascertain the gene in question and the detection oligomer used. On the right side p values for the individual CpG positions are shown. The p values are the probabi-1ities that the observed distribution occurred by chance in the data set.

For selected distinctions, we trained a learning algorithm (support vector machine, SVM). The SVM (as discussed by F. Model,P. Adorjan,A. Olek,C. Piepenbrock, Feature selection for DNA methylation based cancer classification. Bioinformatics. 2001 Jun;17 Suppl 1:8157-64) constructs an optimal discriminant between two classes of given training samples. In this case each sample is described by the methylation patterns (CG/TG ratios) at the investigated CpG sites. The SVM was trained on a subset of samples of each class, which were presented with the diagnosis attached. Independent test samples, which were not shown to the SVM before were then presented to evaluate, if the diagnosis can be predicted correctly based on the predictor created in the training round. This procedure was repeated several times using different partitions of the samples, a method called crossvalidation. Please note that all rounds are performed without using any knowledge obtained in the previous runs. The number of correct classifications was averaged over all runs, which gives a good estimate of our test accuracy (percent of correct classified samples over all rounds).

### Healthy colon tissue compared to non healthy colon tissue (colon adenoma and colon carcinoma) (Figure 1)

Figure 1 shows the differentiation of healthy tissue from non healthy tissue wherein the non healthy specimens are obtained from either colon adenoma or colon carcinoma tissue. The evaluation is carried out using informative CpG positions from 27 genes. Informative CpG positions are further described in Table 3.

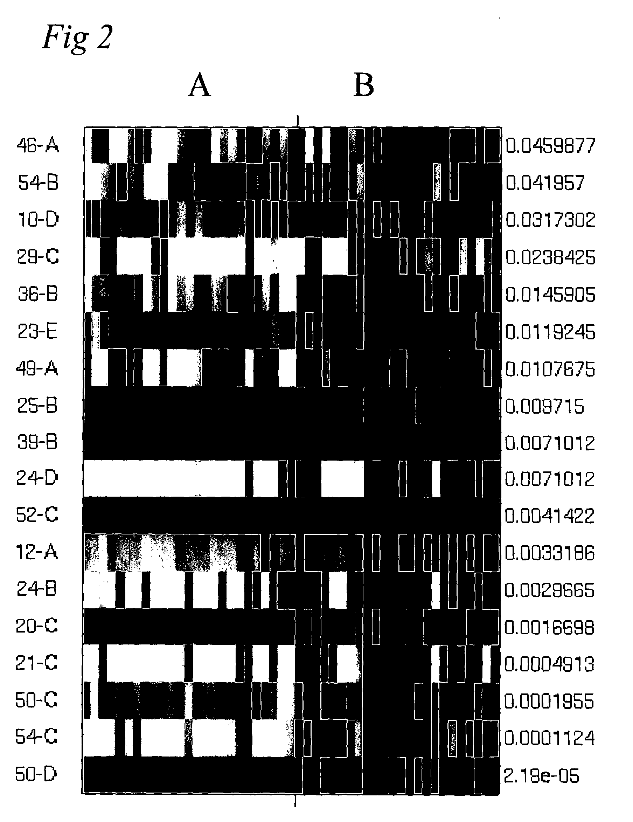

### Healthy colon tissue compared to colon carcinoma tissue (Figure 2)

Figure 2 shows the differentiation of healthy tissue from carcinoma tissue using informative CpG positions from 15 genes. Informative CpG positions are further described in Table 4.

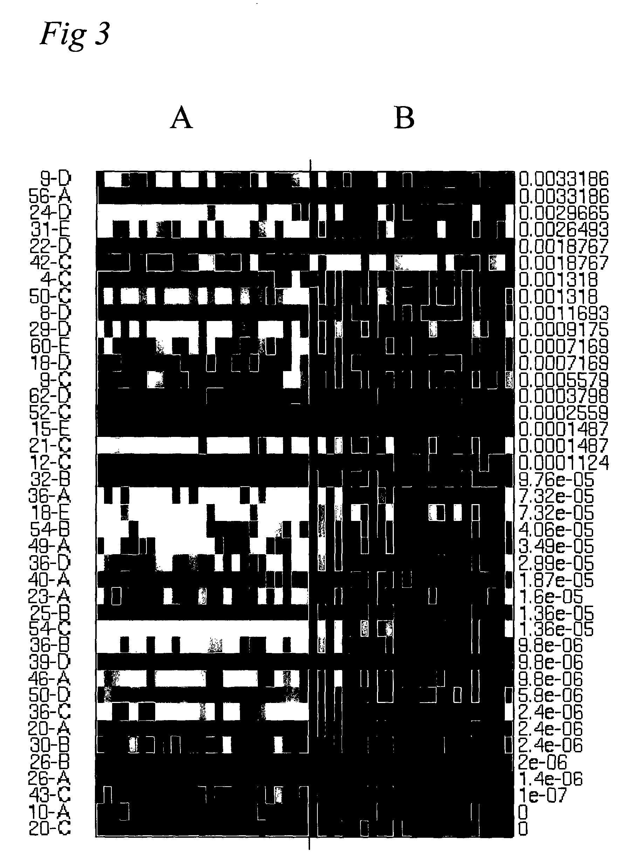

### Healthy colon tissue compared to colon adenoma tissue (Figure 3)

Figure 3 shows the differentiation of healthy tissue from adenoma tissue using informative CpG positions from 40 genes. Informative CpG positions are further described in Table 5.

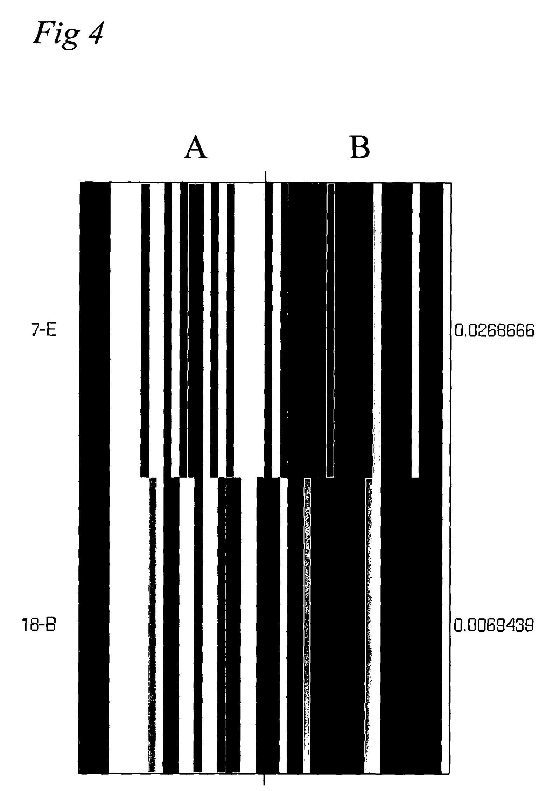

### Colon carcinoma tissue compared to colon adenoma tissue (Figure 4)

Figure 4 shows the differentiation of carcinoma tissue from adenoma tissue using informative CpG positions from 2 genes. Informative CpG positions are further described in Table 6.

### Example 3: Identification of the methylation status of a CpG site within the gene CD44.

A fragment of the bisulfite treated DNA of the gene CD44 (Seq ID NO: 20) was PCR amplified using primers GAAAGGAGAGGTTAAAGGTTG (Seq ID NO 429) and AACTCACTTAACTCCAATCCC (Seq ID NO 430). The resultant fragment (696 bp in length) contained an informative CpG at position 235. The amplificate DNA was digested with the restriction endonuclease *Apa I,* recogniton site GGGCC. Hydrolysis by said endonuclease is blocked by methylation of the CpG at position 235 of the amplificate. The digest was used as a control.

Genomic DNA was isolated from sample using the DNA wizzard DNA isolation kit (Promega). Each sample was digested using *Apa I* according to manufacturer's recommendations (New England Biolabs).

10 ng of each genomic digest was then amplified using PCR primers GAAAGGAGAGGTTAAAGGTTG and AACTCACTTAACTCCAATCCC. The PCR reactions were performed using a thermocycler (Eppendorf GmbH) using 10 ng of DNA, 6 pmol of each primer, 200 µM of each dNTP, 1.5 mM MgC12 and 1 U of HotstartTaq (Qiagen AG). The other conditions were as recommended by the Taq polymerase manufacturer. Using the above mentioned primers, gene fragments were amplified by PCR performing a first denaturation step for 14 min at 96 °C, followed by 30 - 45 cycles (step 2: 60 sec at 96°C, step 3: 45 sec at 52°C , step 4: 75 sec at 72°C) and a subsequent final elongation of 10 min at 72°C. The presence of PCR products was analysed by agrarose gel electrophoresis.

PCR products were detectable with *Apa I* hydrolyzed DNA isolated wherein the CpG position in question was upmethylated, when step 2 to step 4 of the cycle program were repeated 34, 37, 39, 42 and 45 fold. In contrast PCR products were only detectable with *Apa I* hydrolyzed DNA isolated from downmethylated DNA (and control DNA) when step 2 to step 4 of the cycle program were repeated 42 and 45 fold. These results were incorporated into a CpG methylation matrix analysis as described in Example 2.

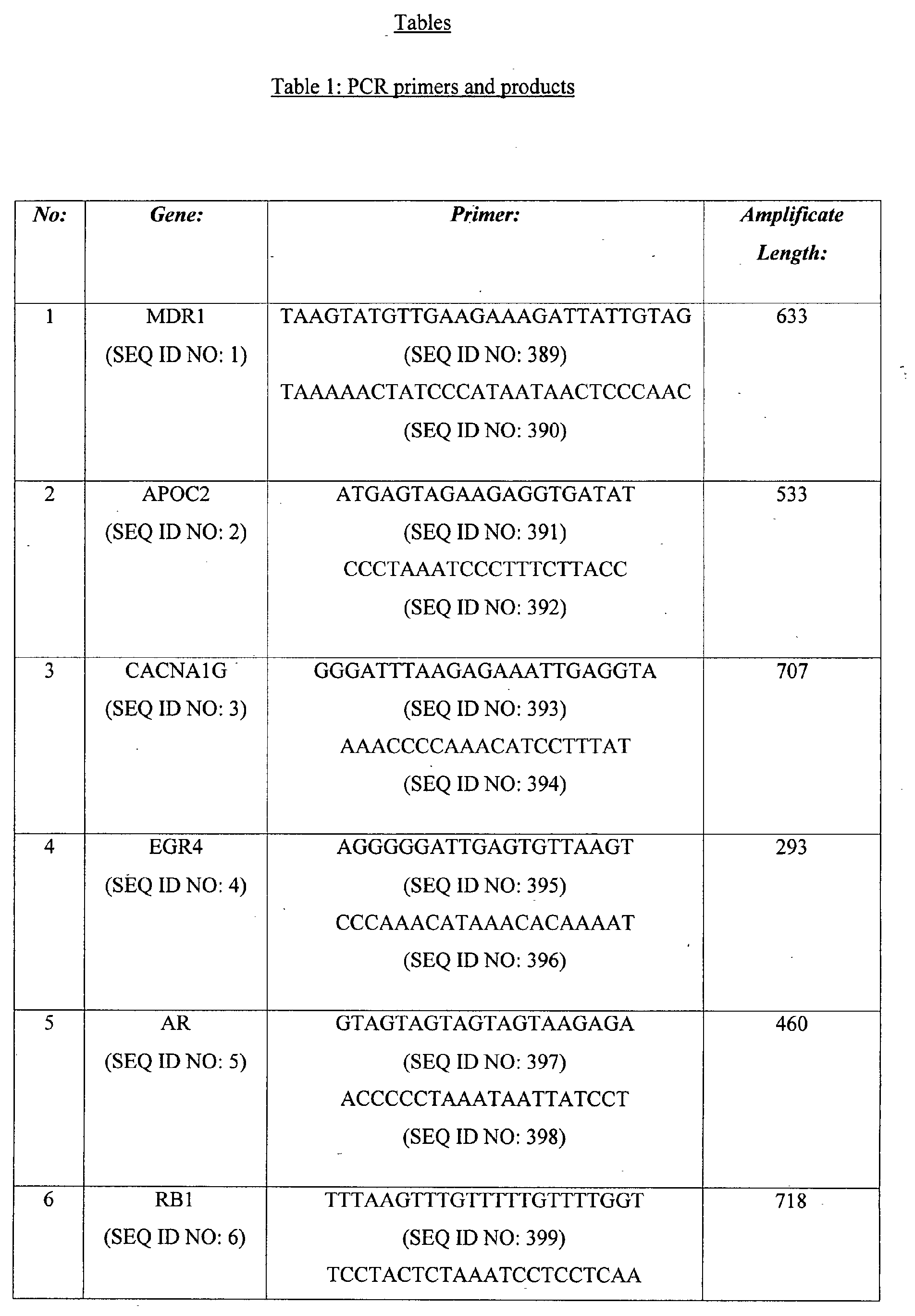

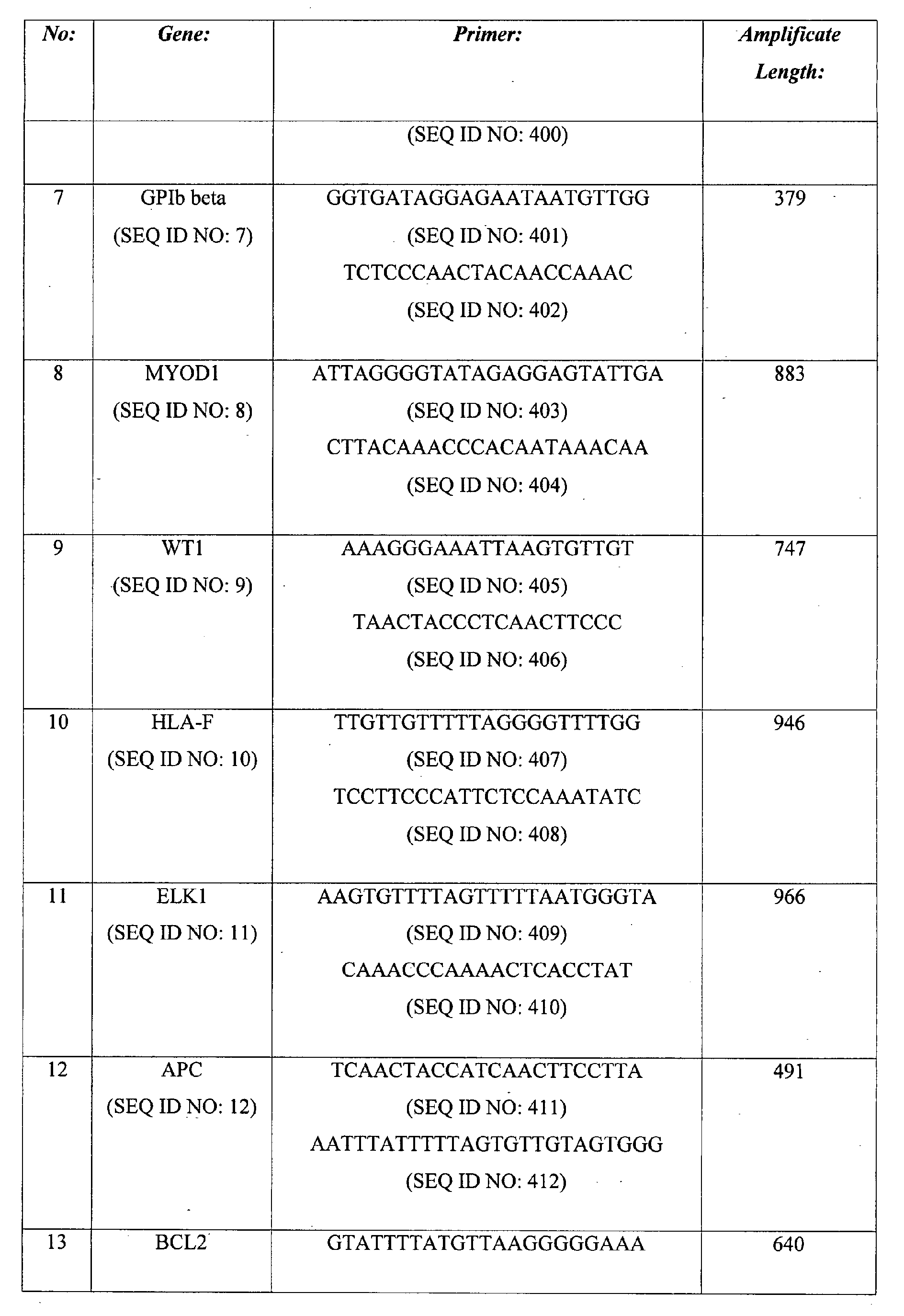

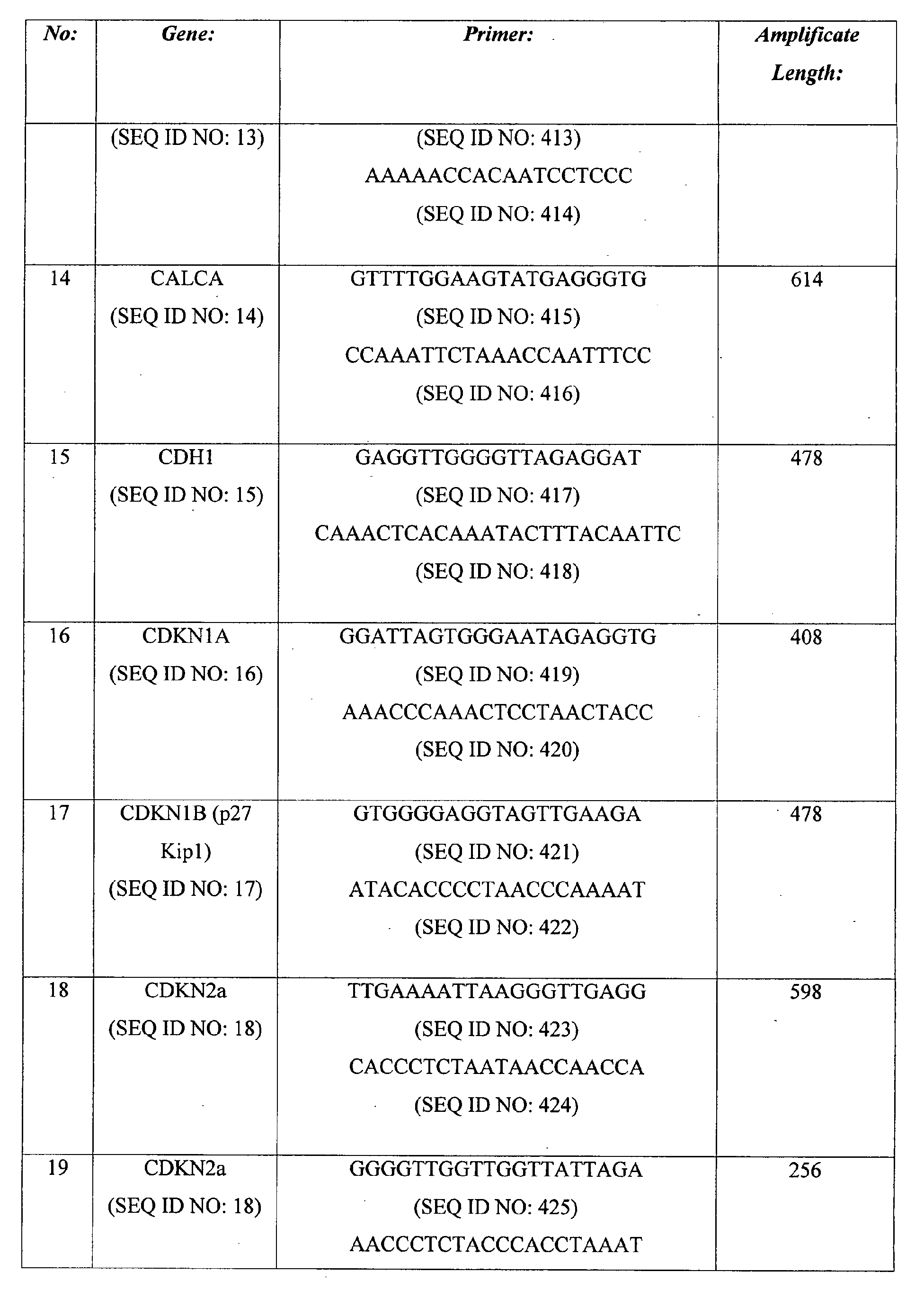

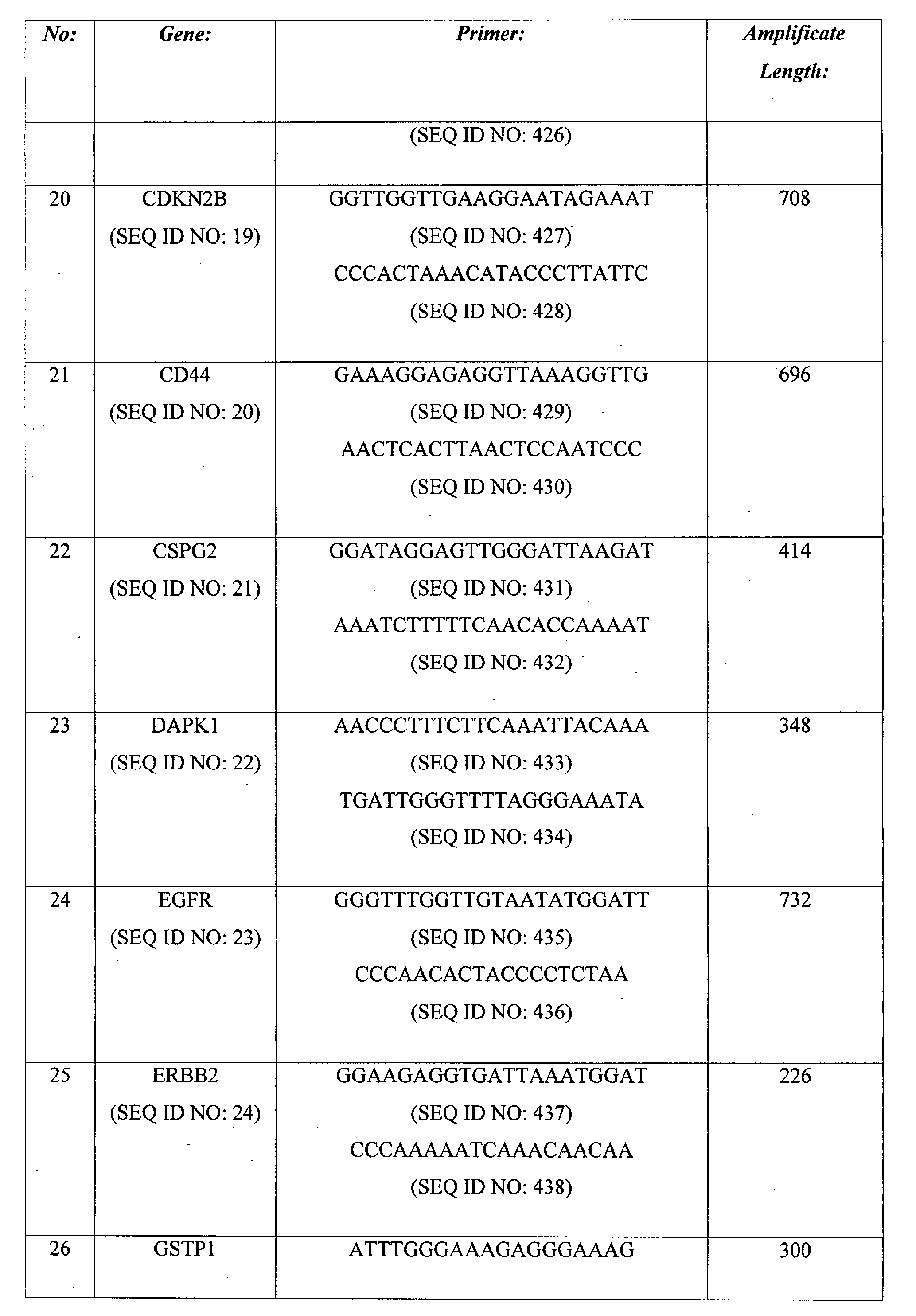

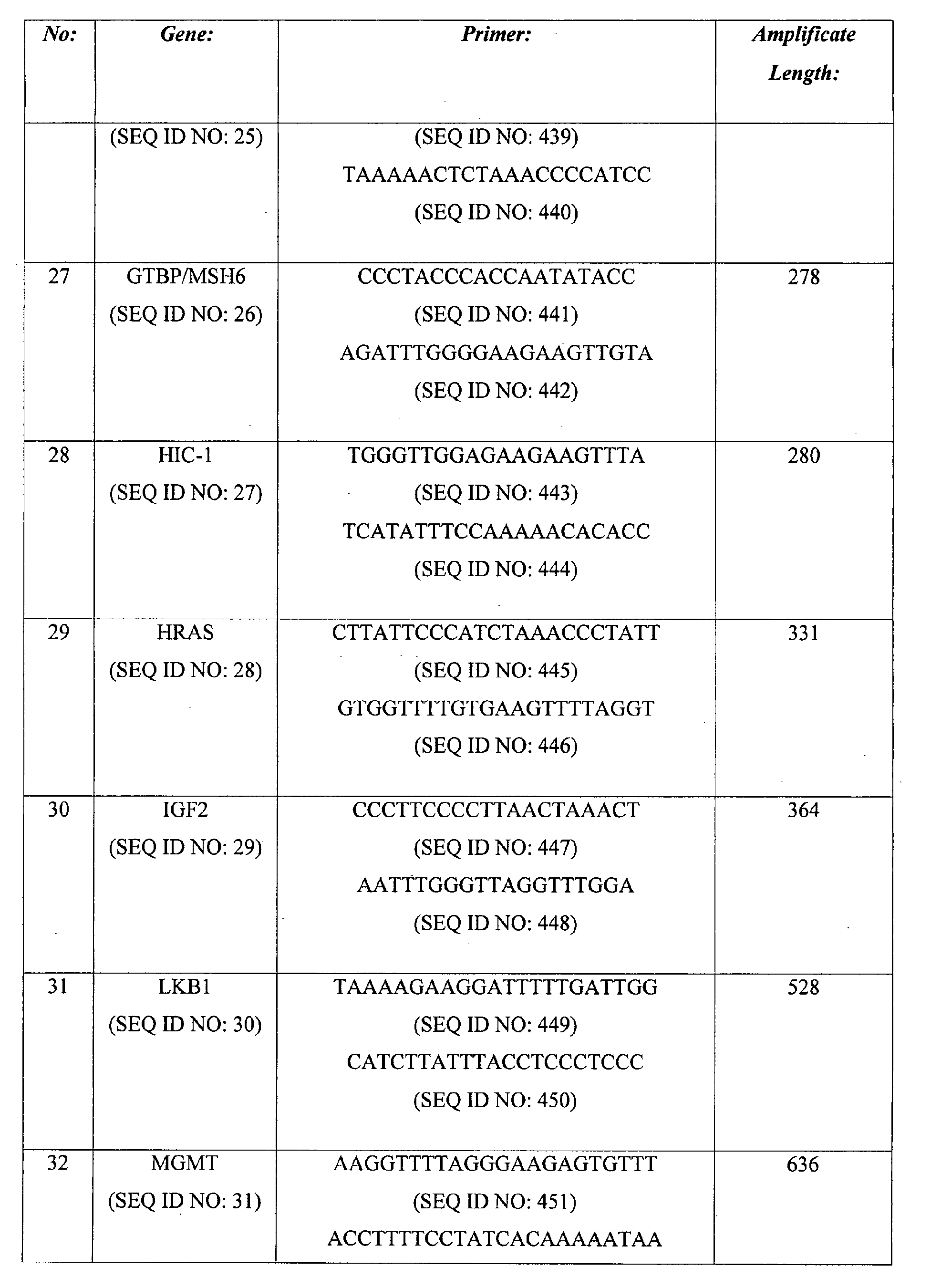

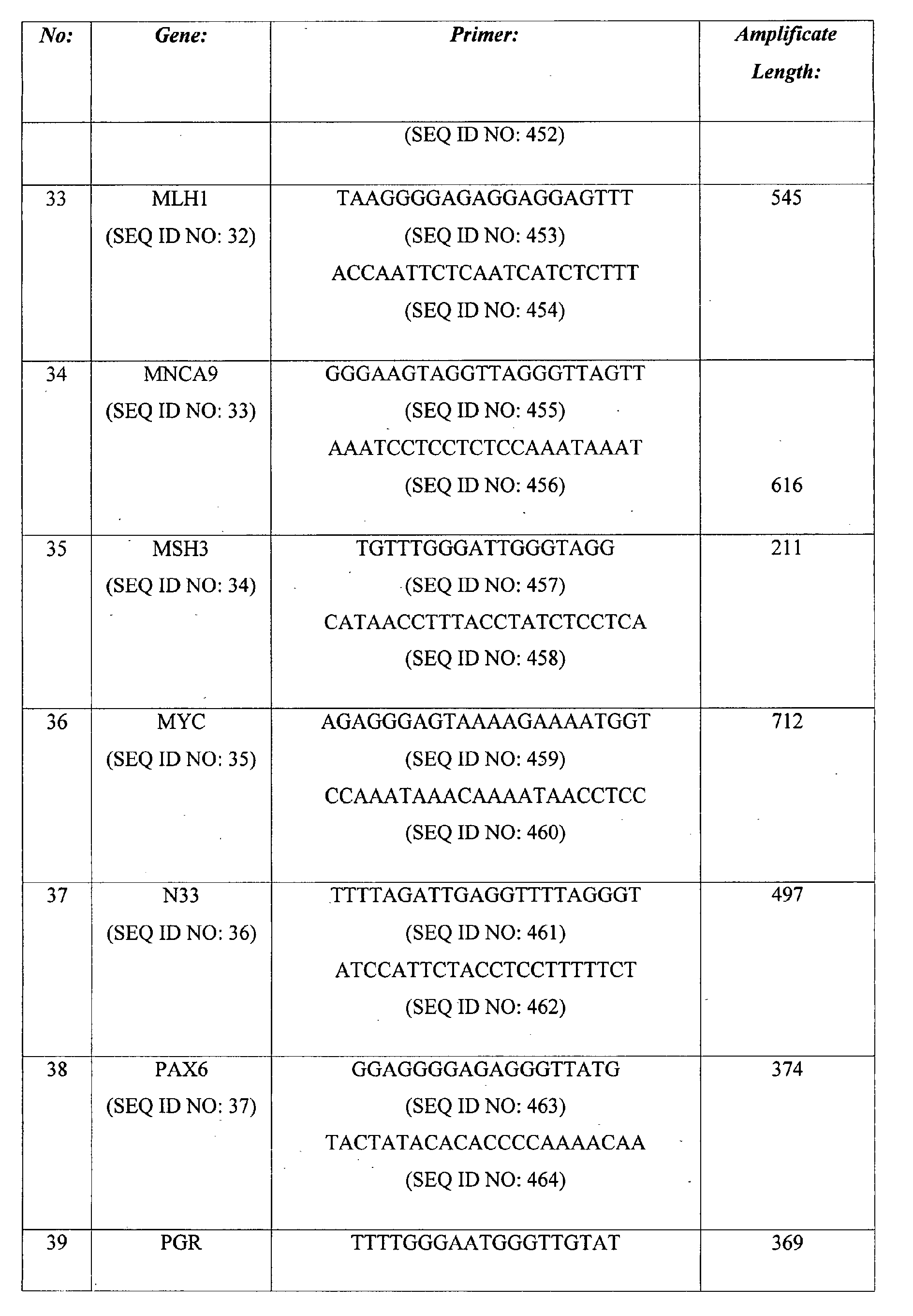

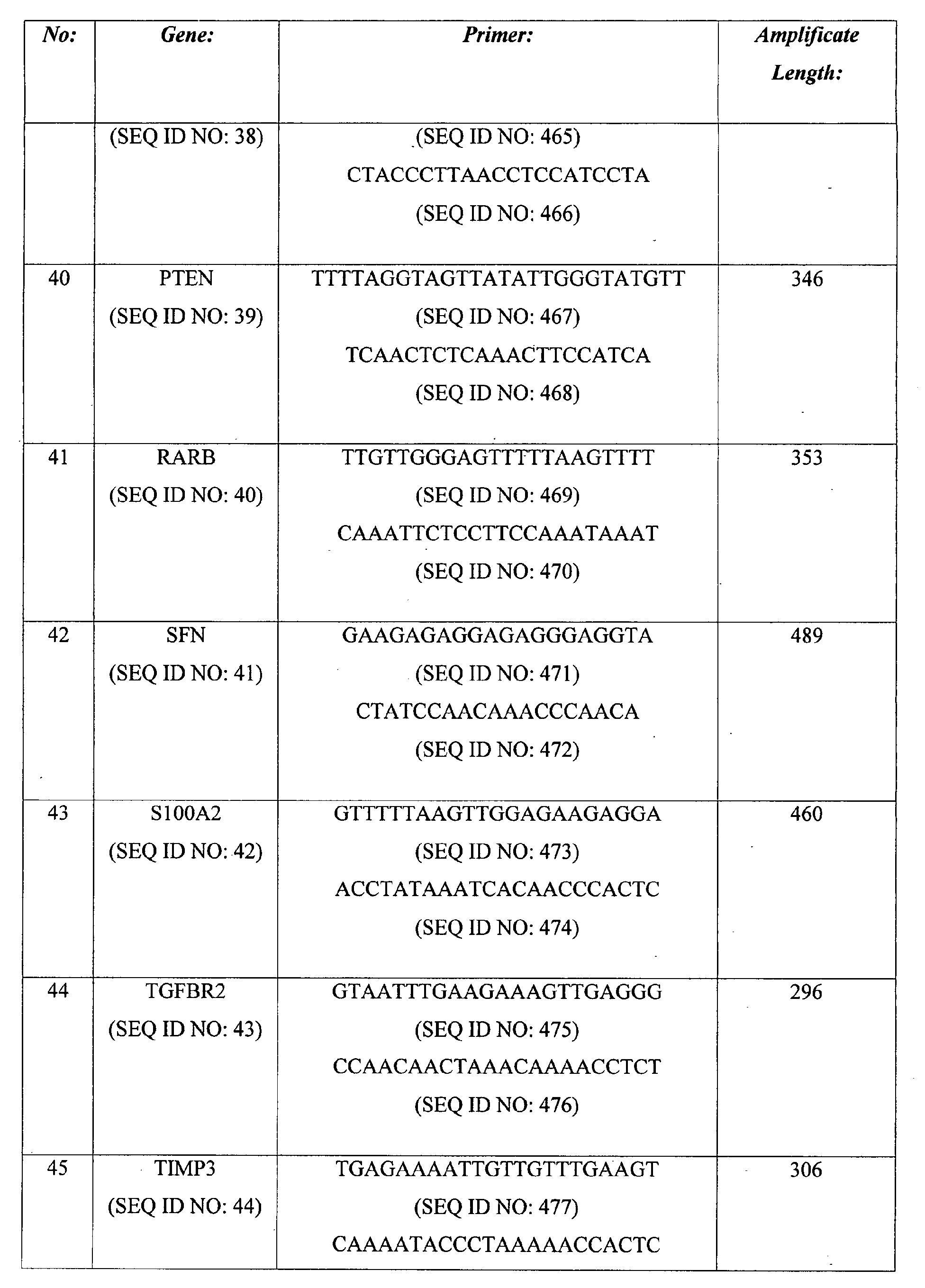

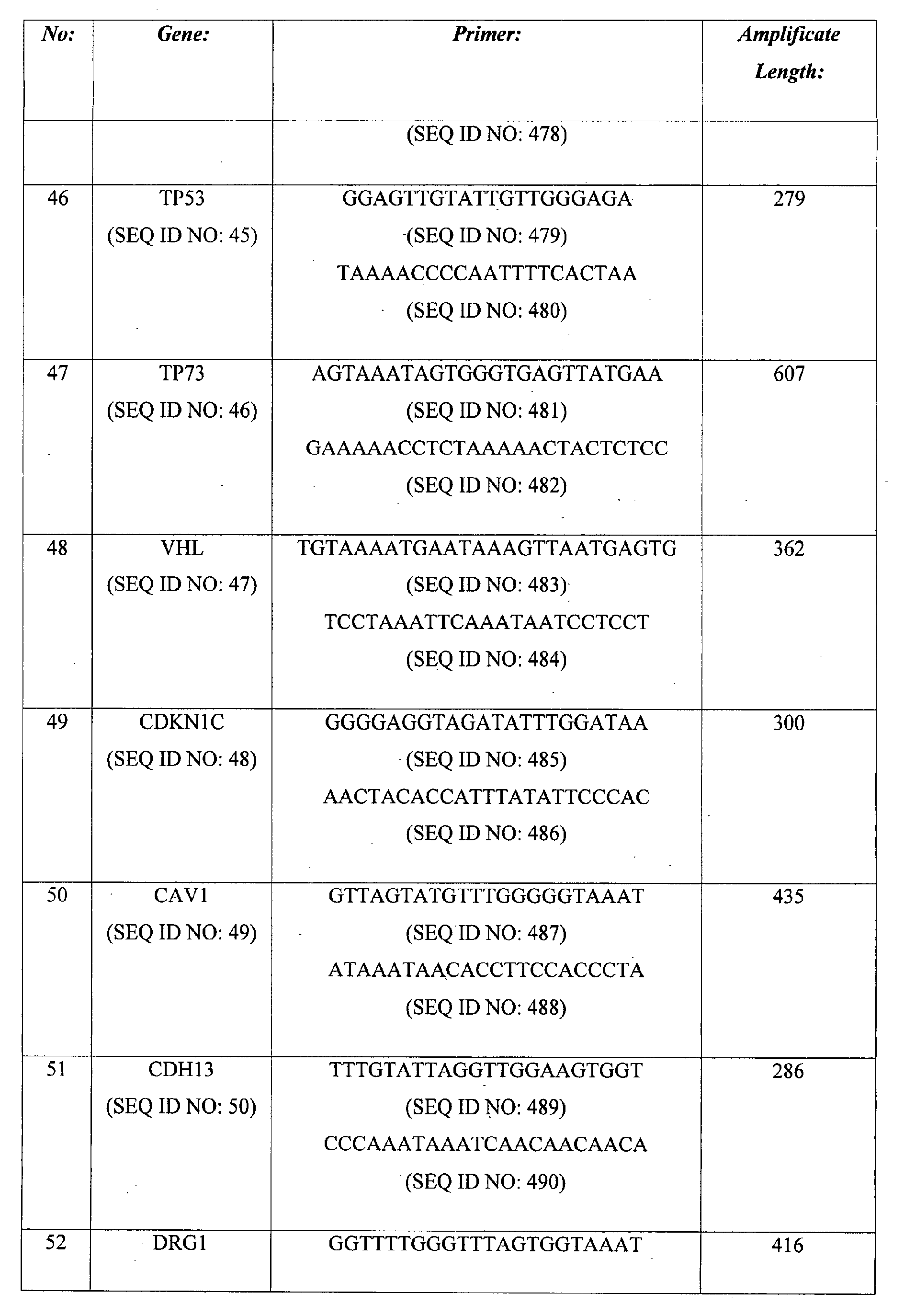

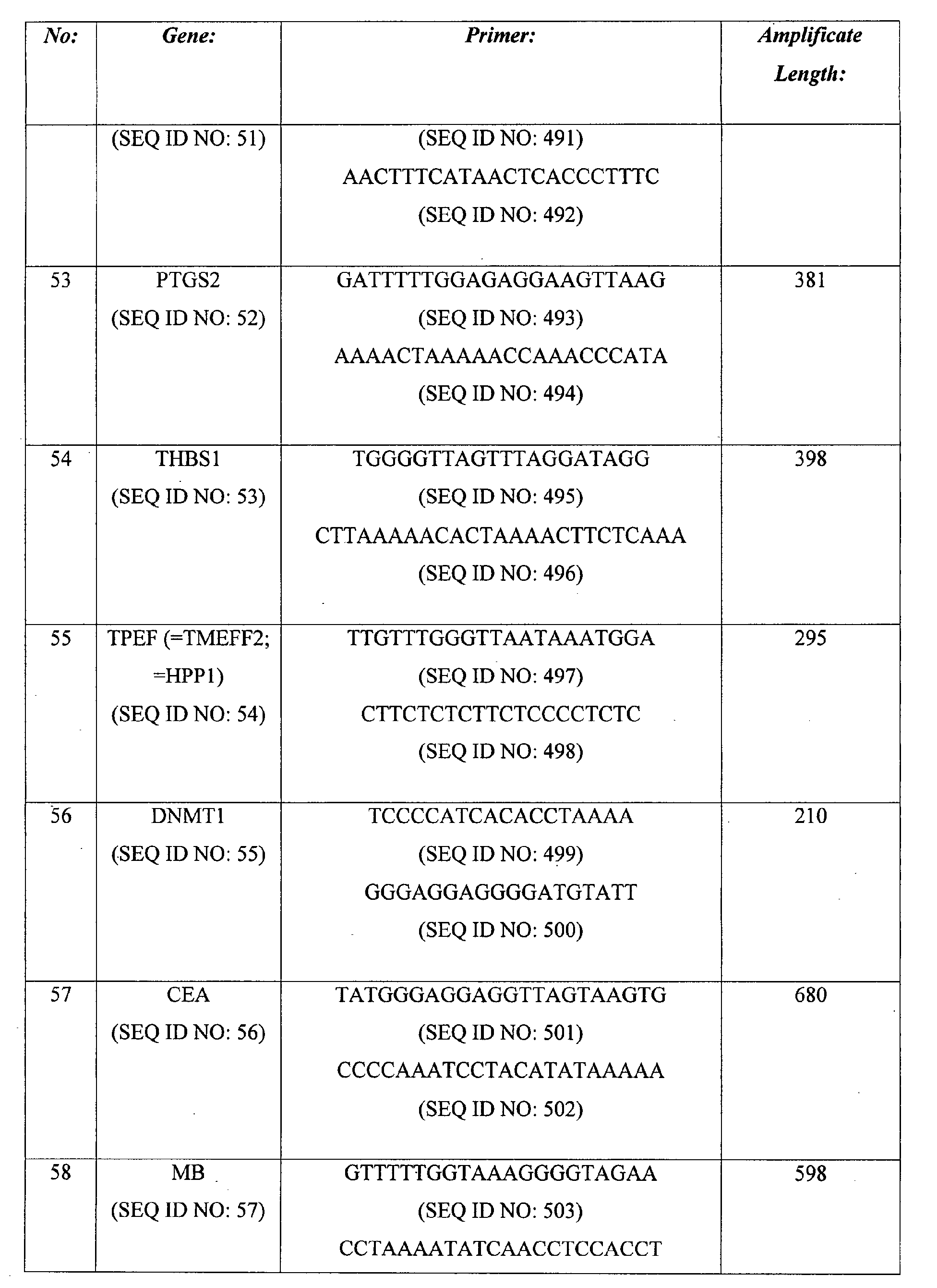

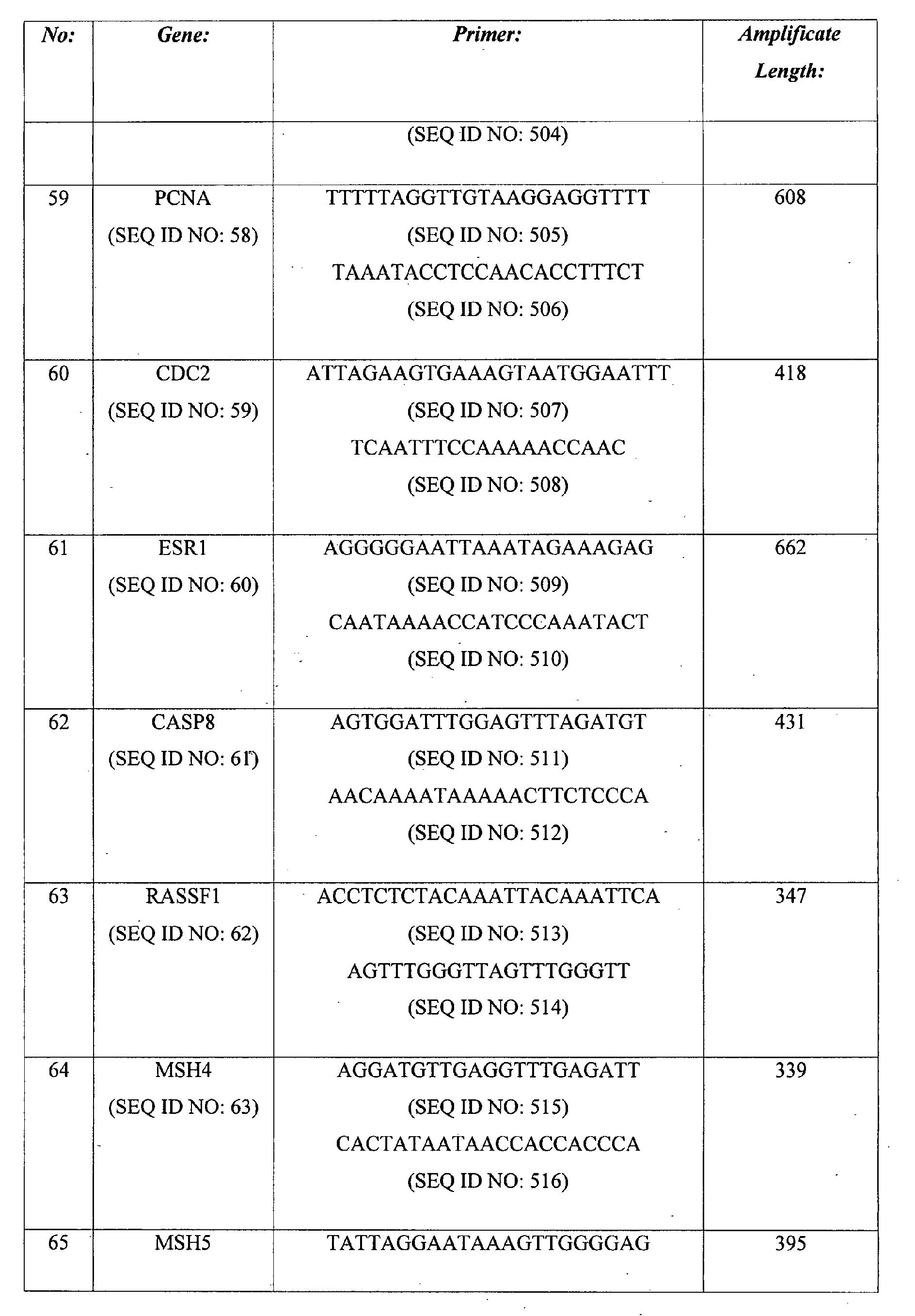

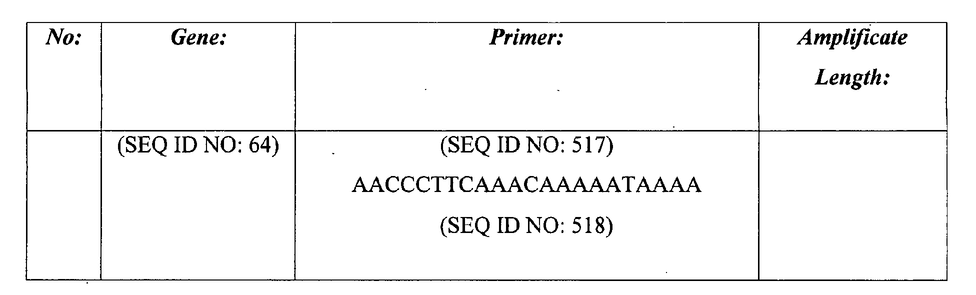

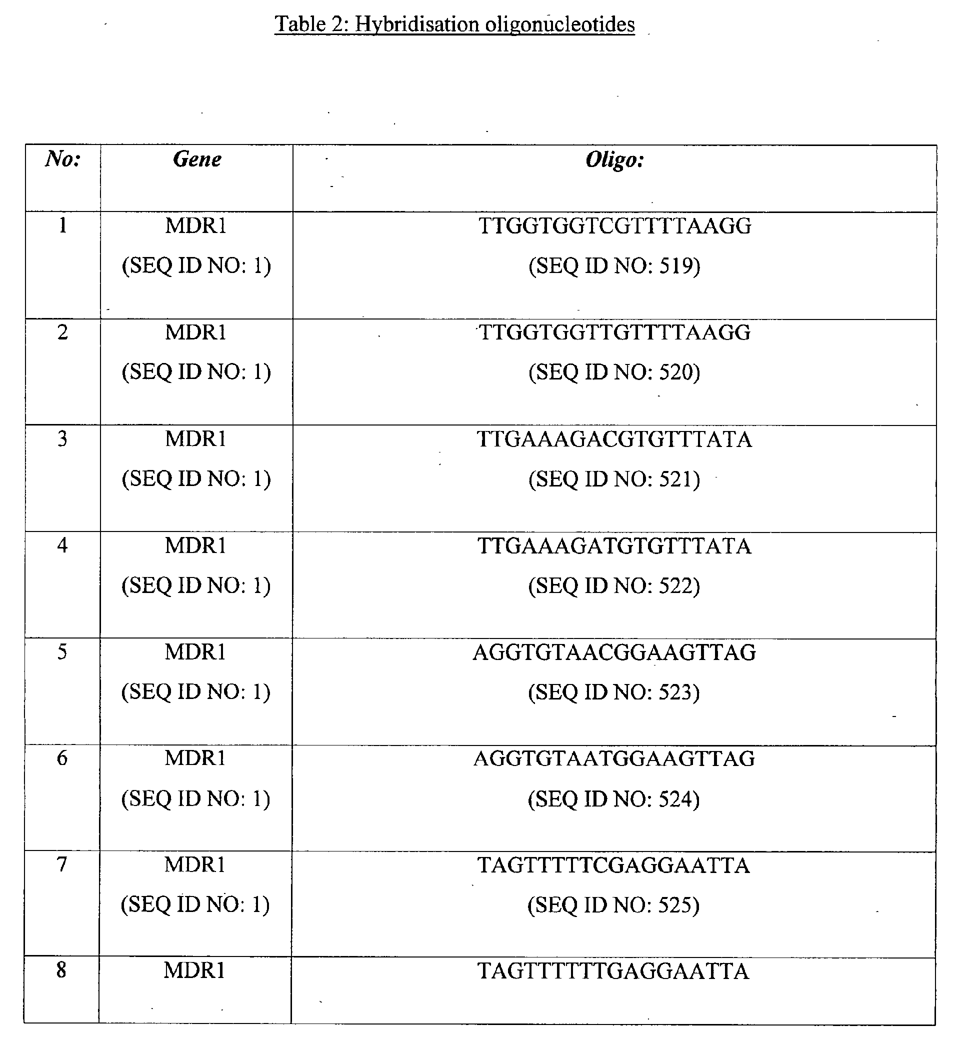

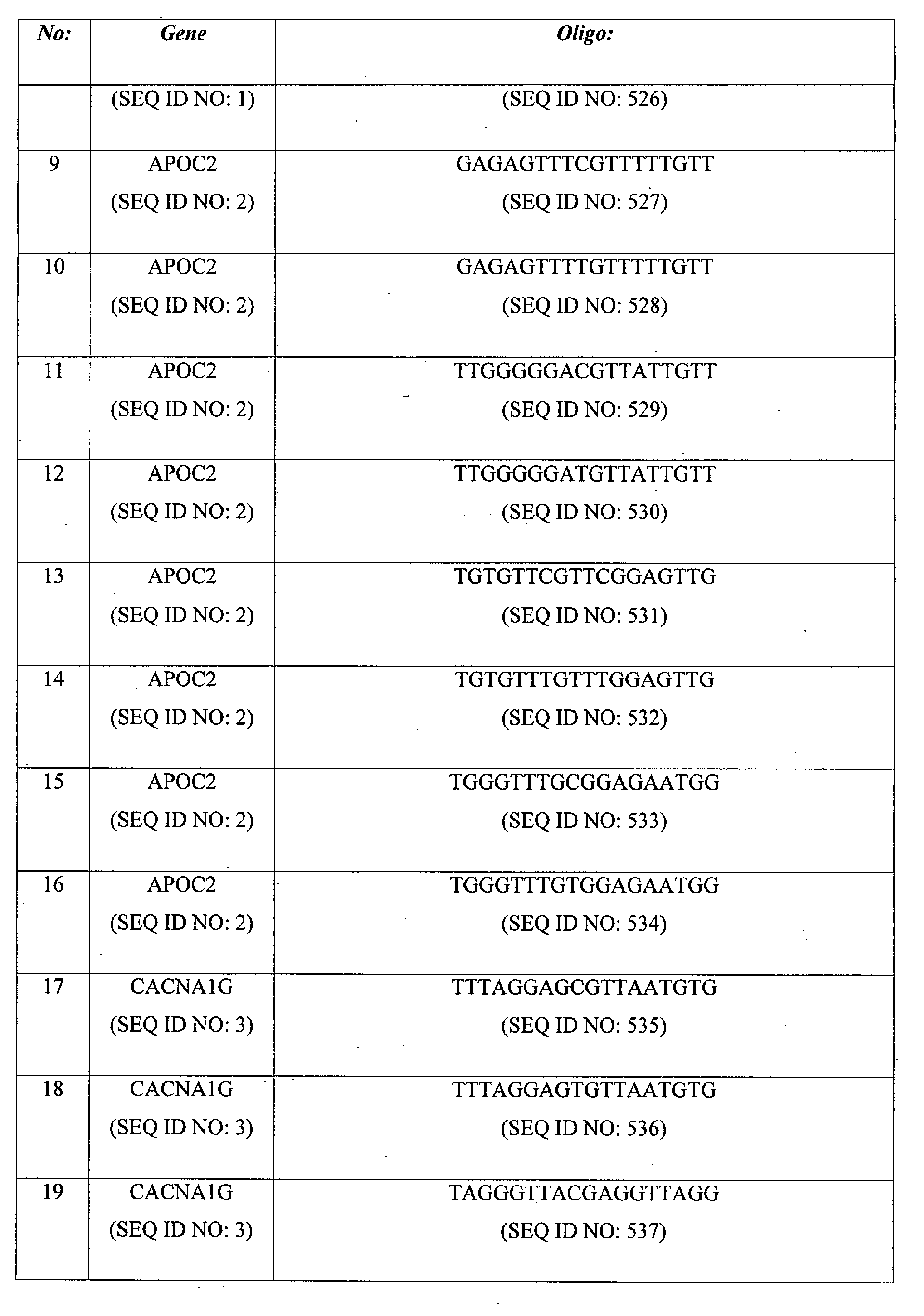

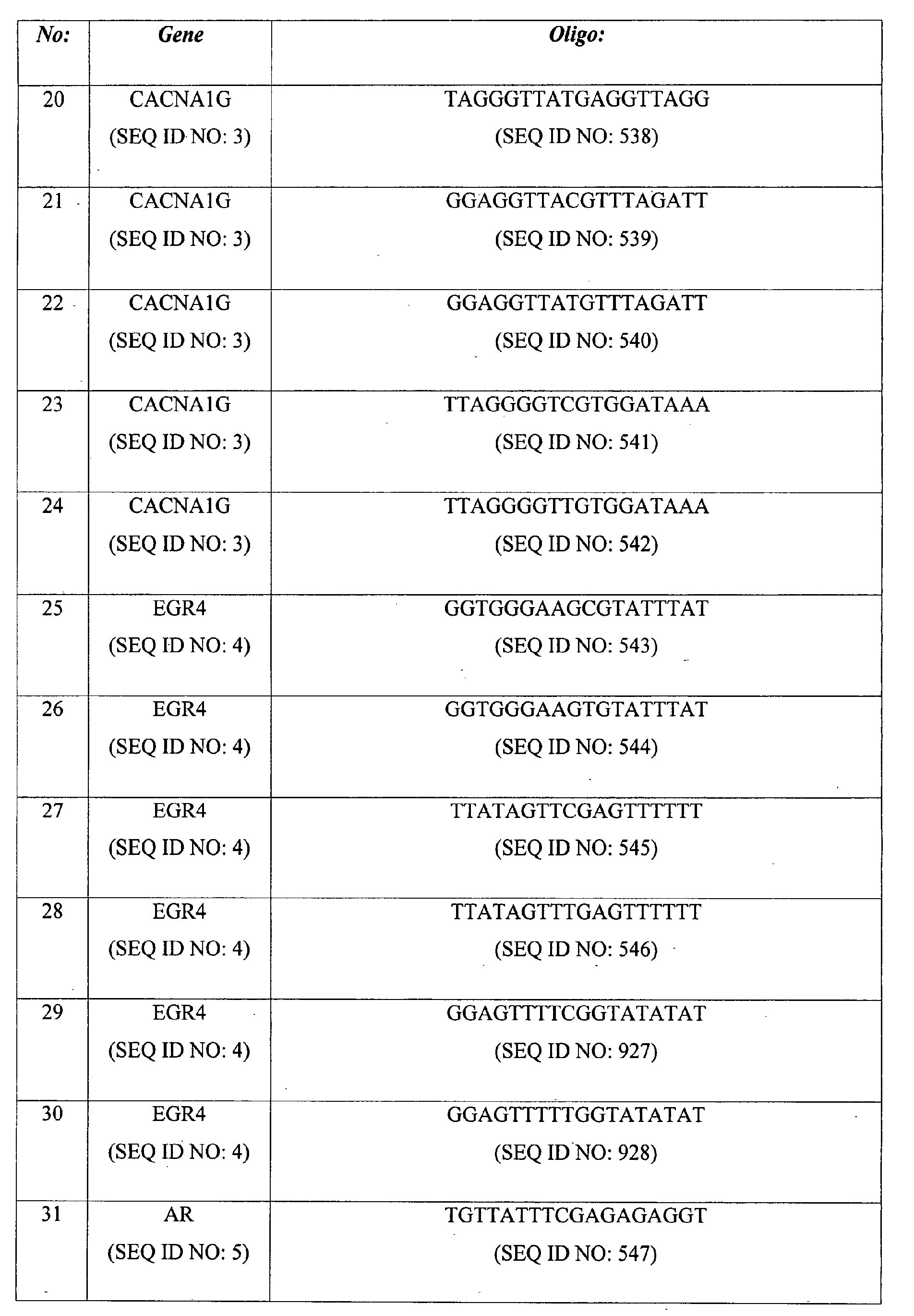

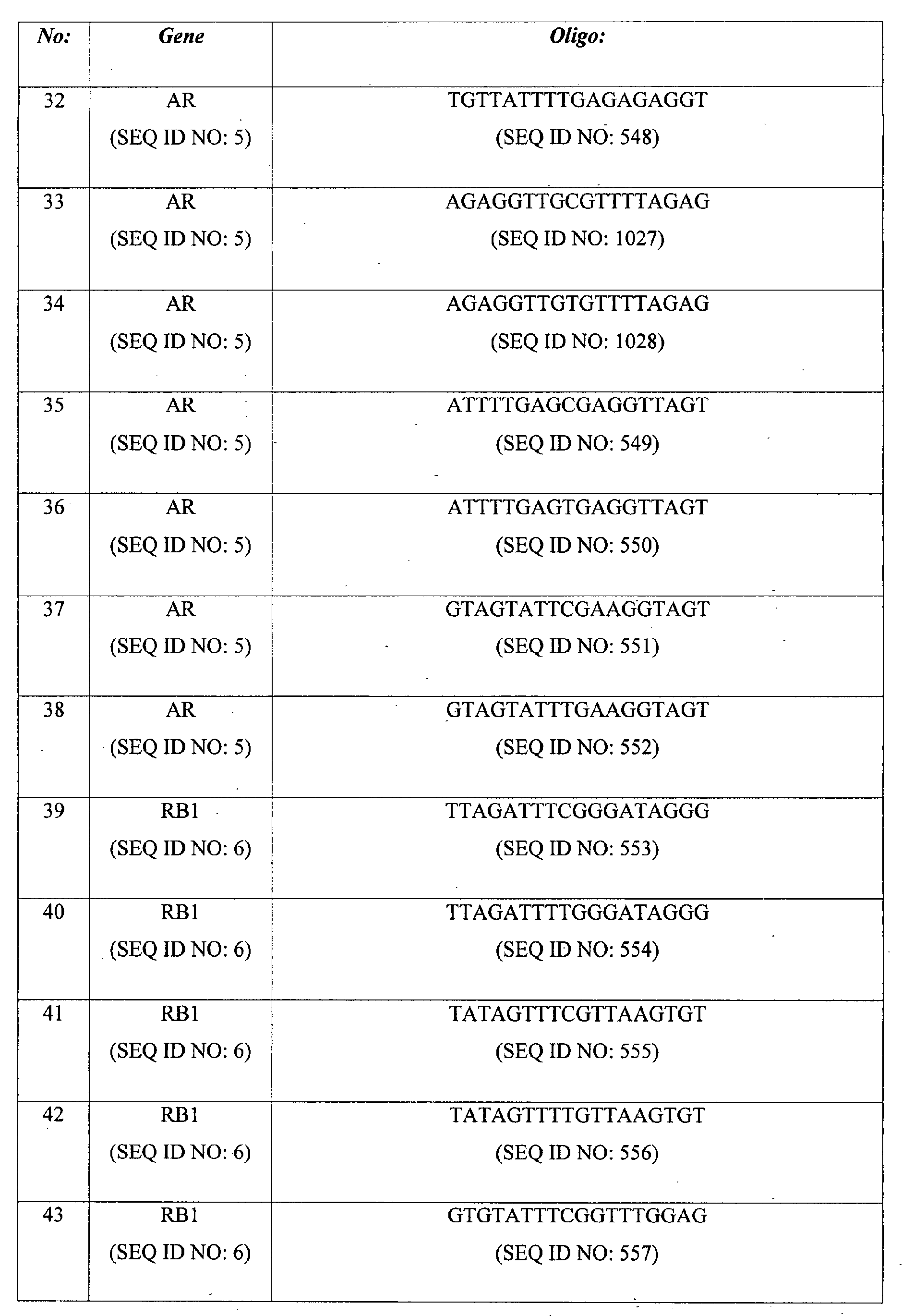

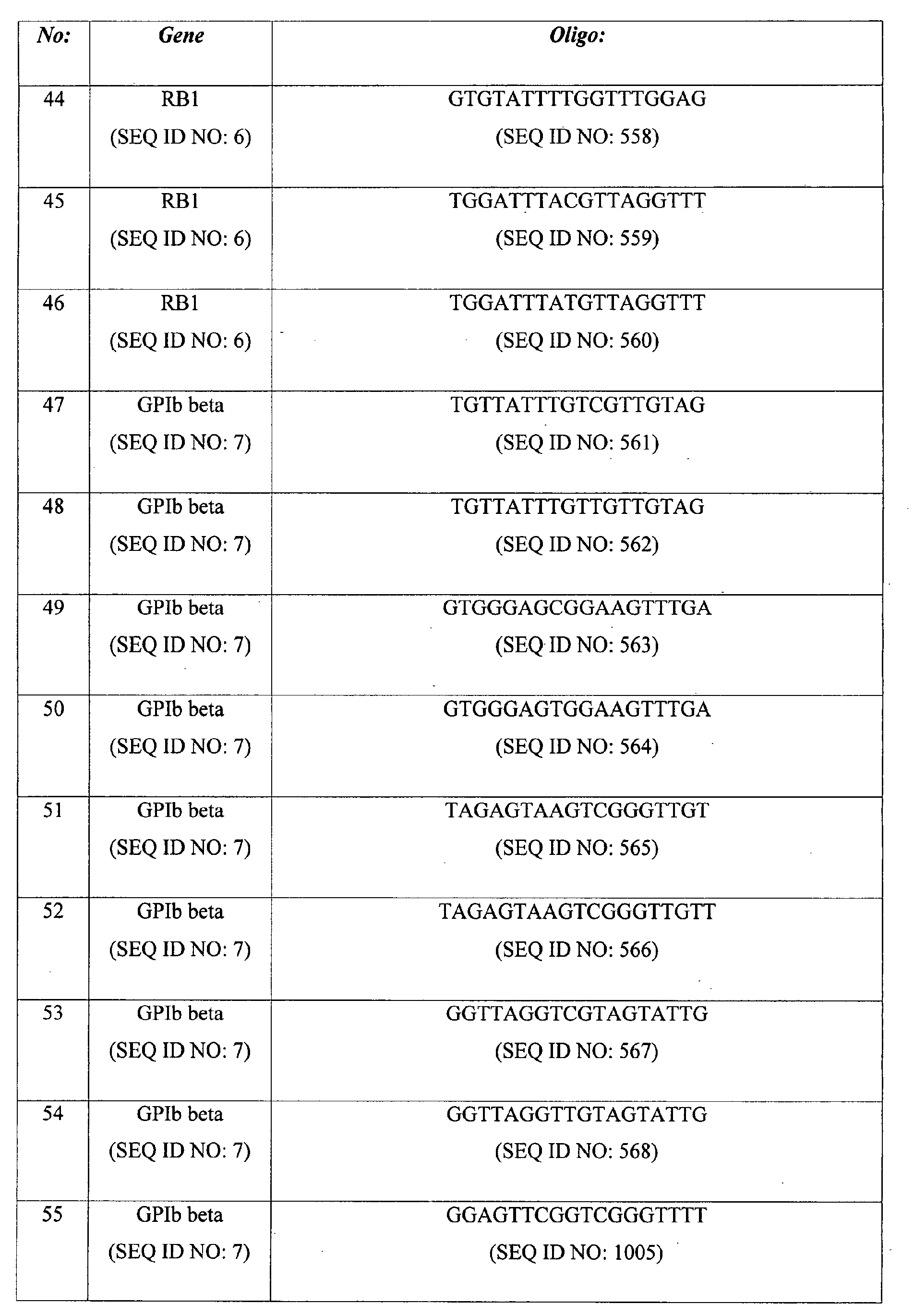

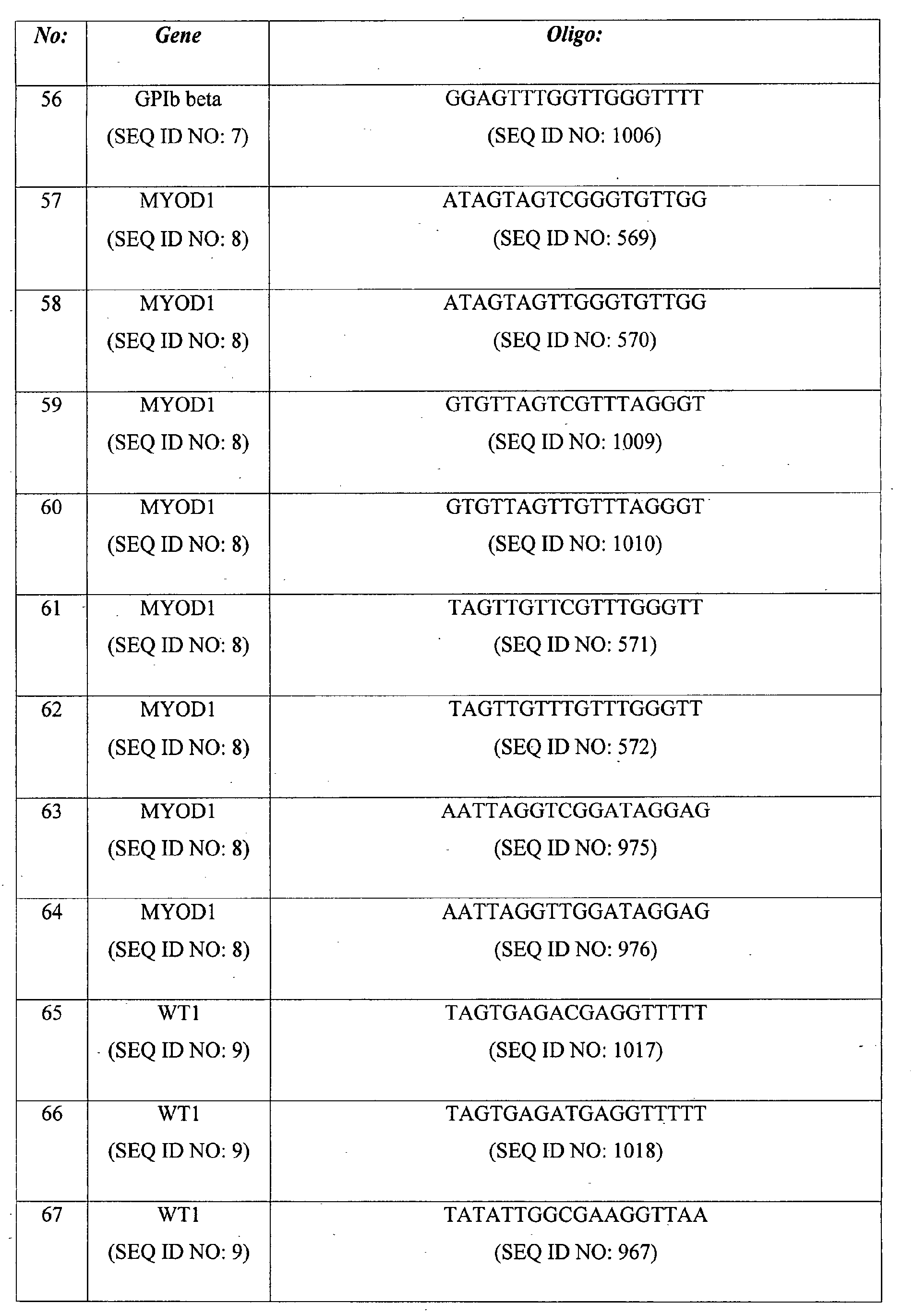

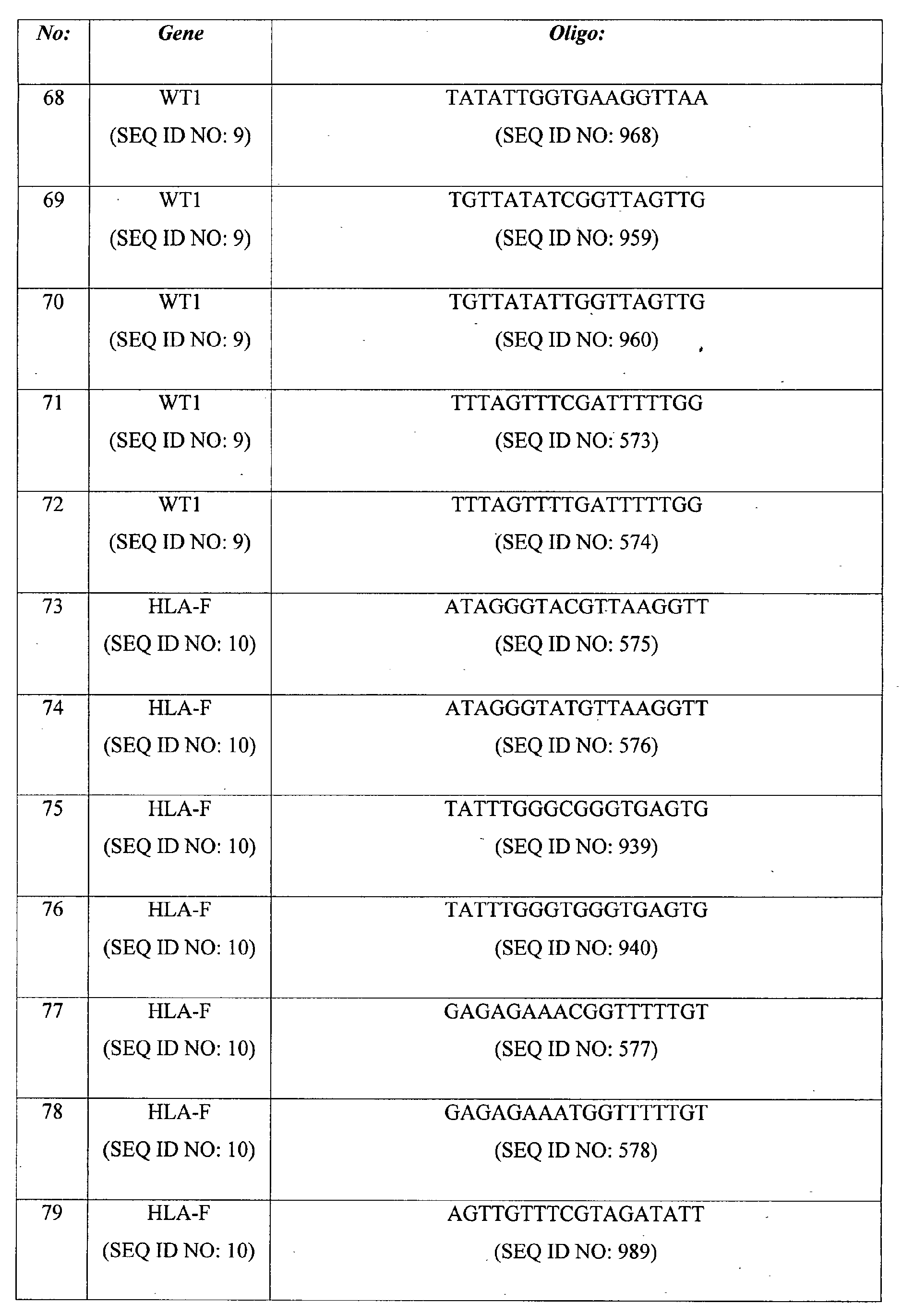

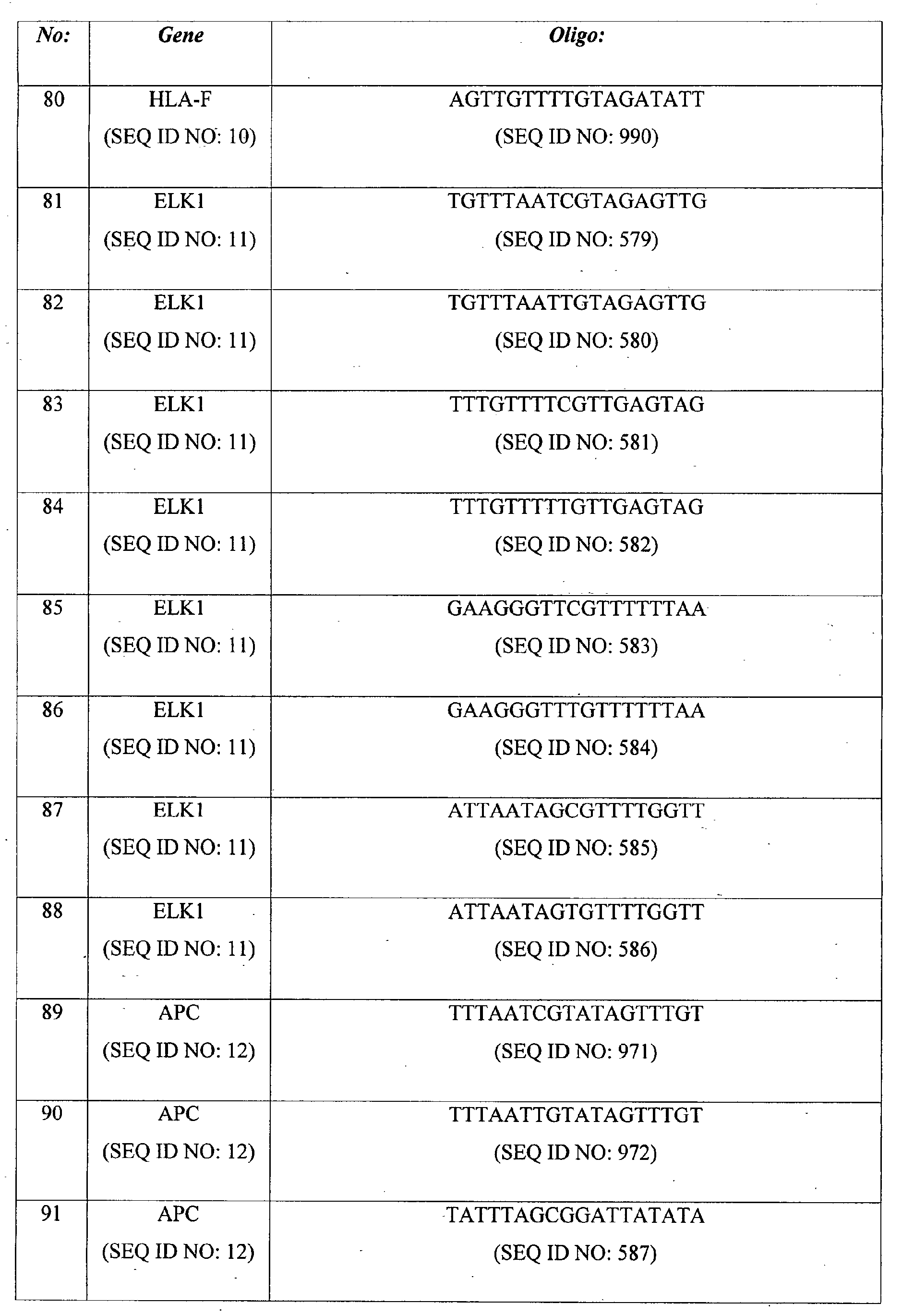

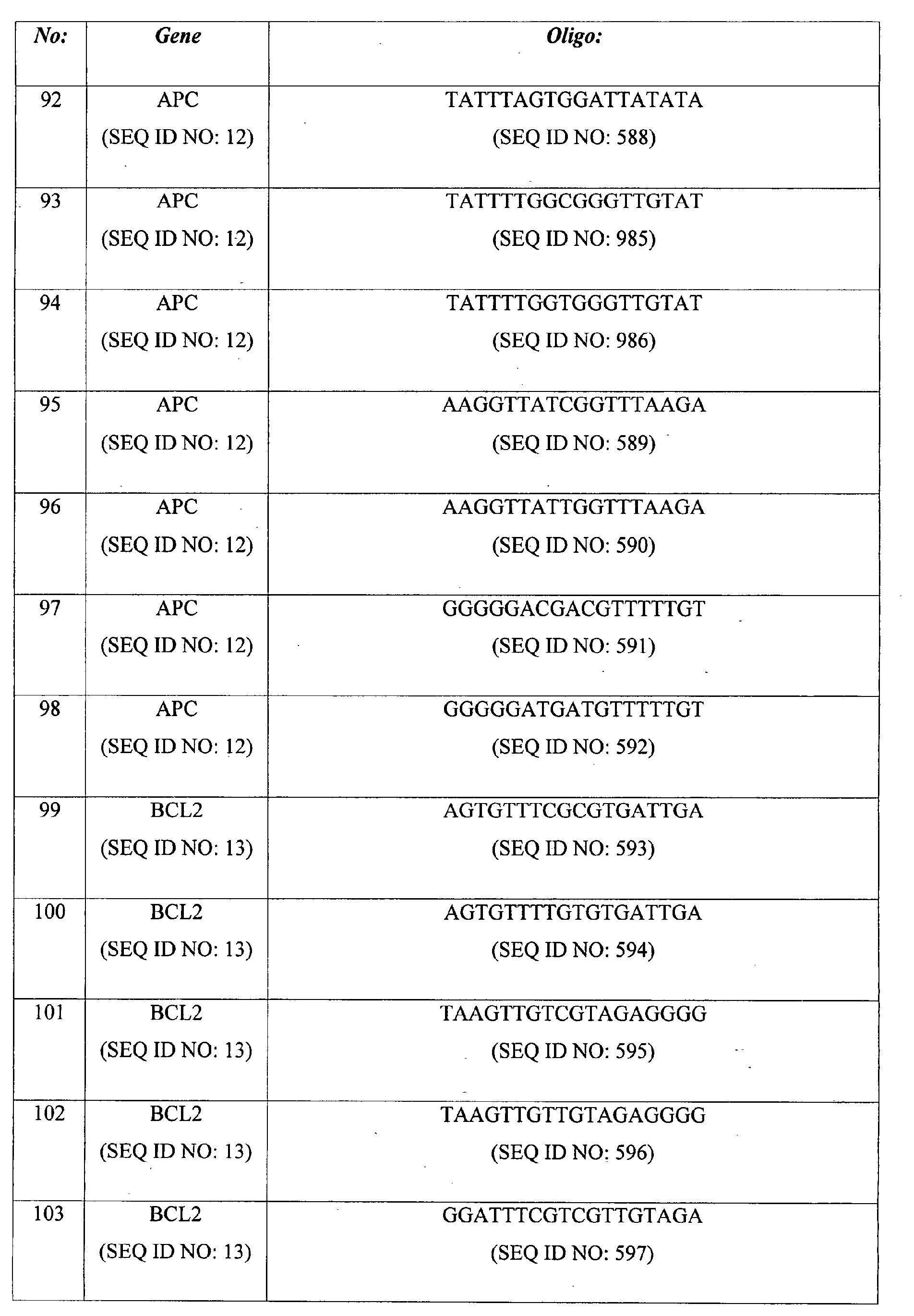

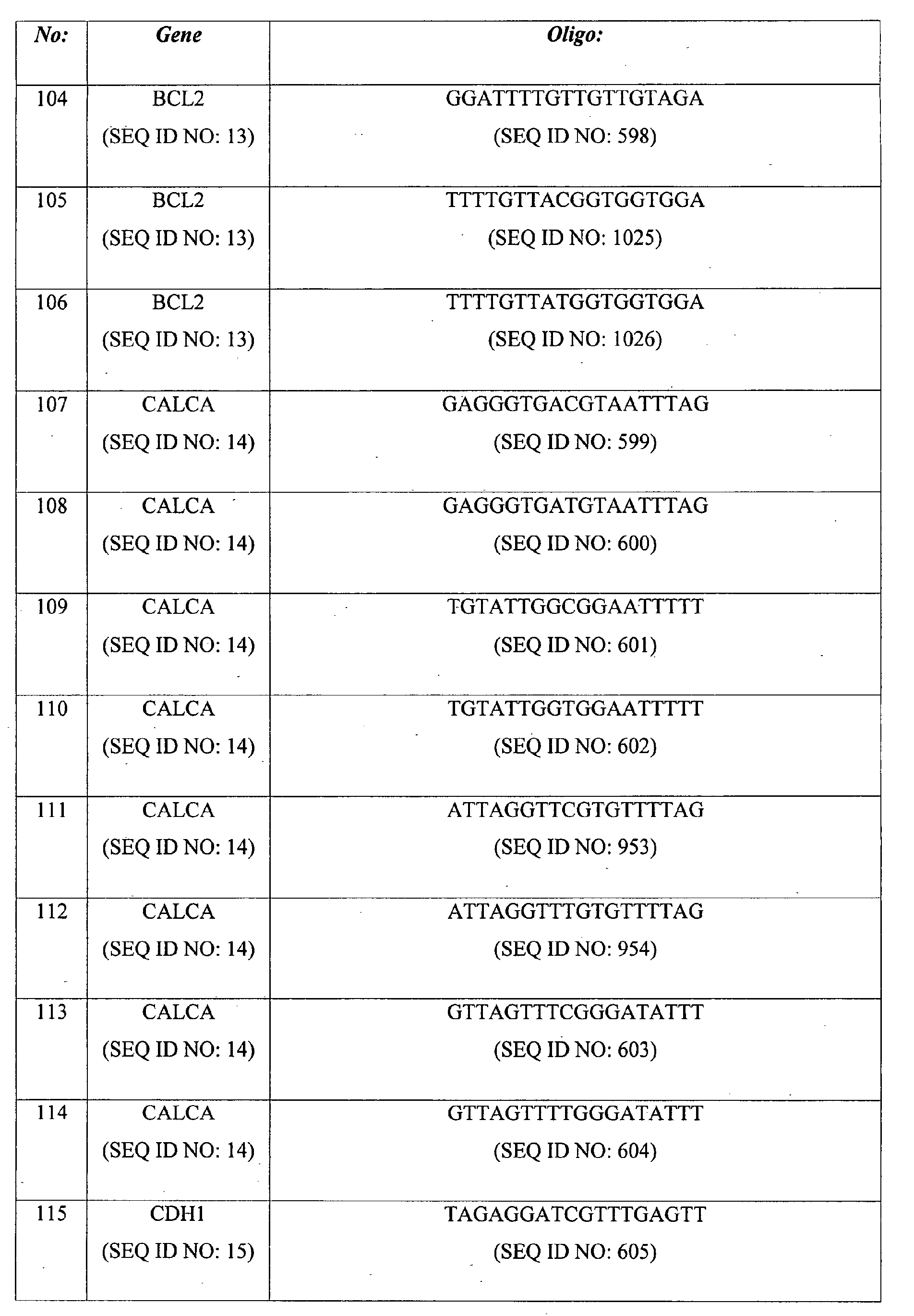

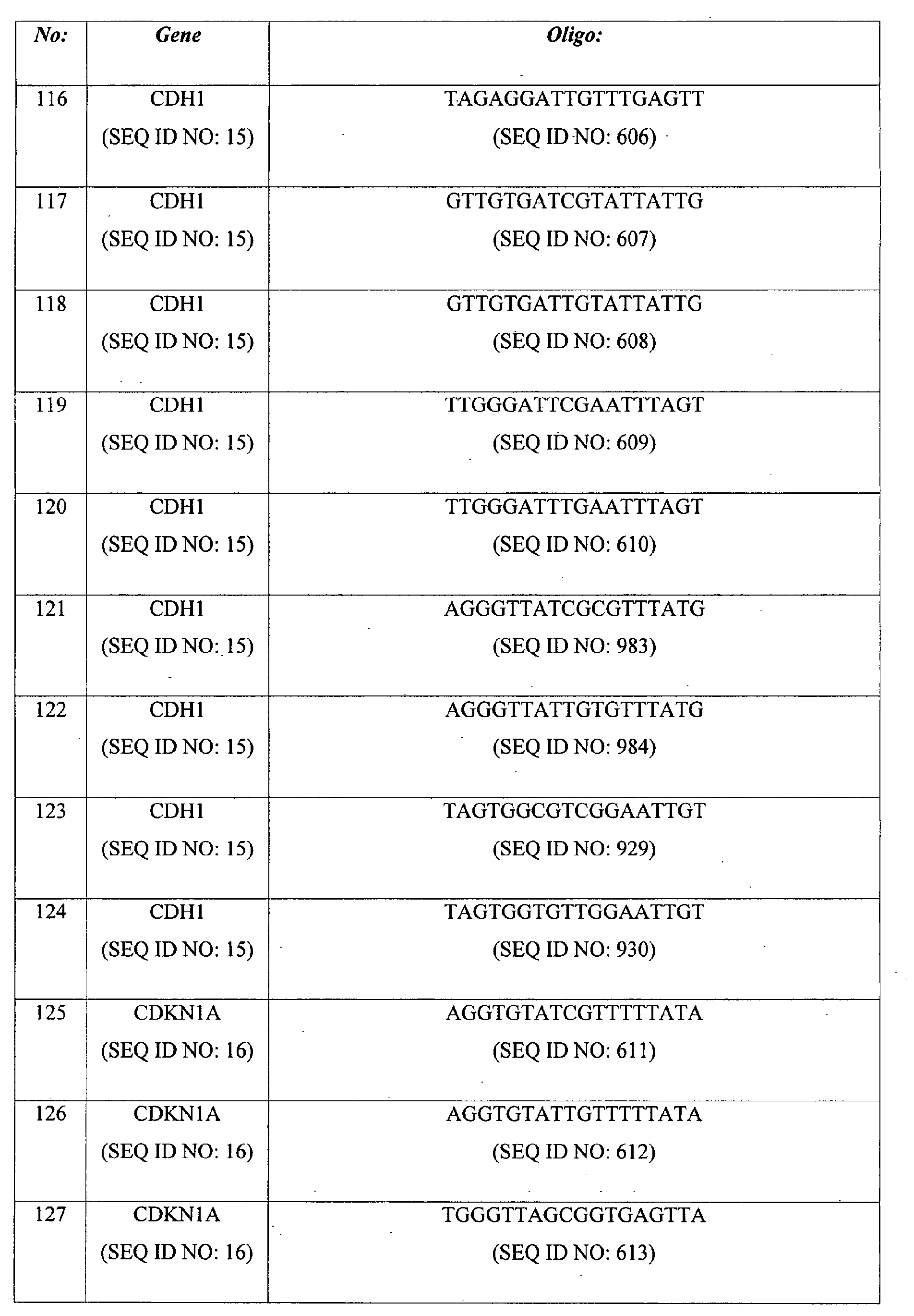

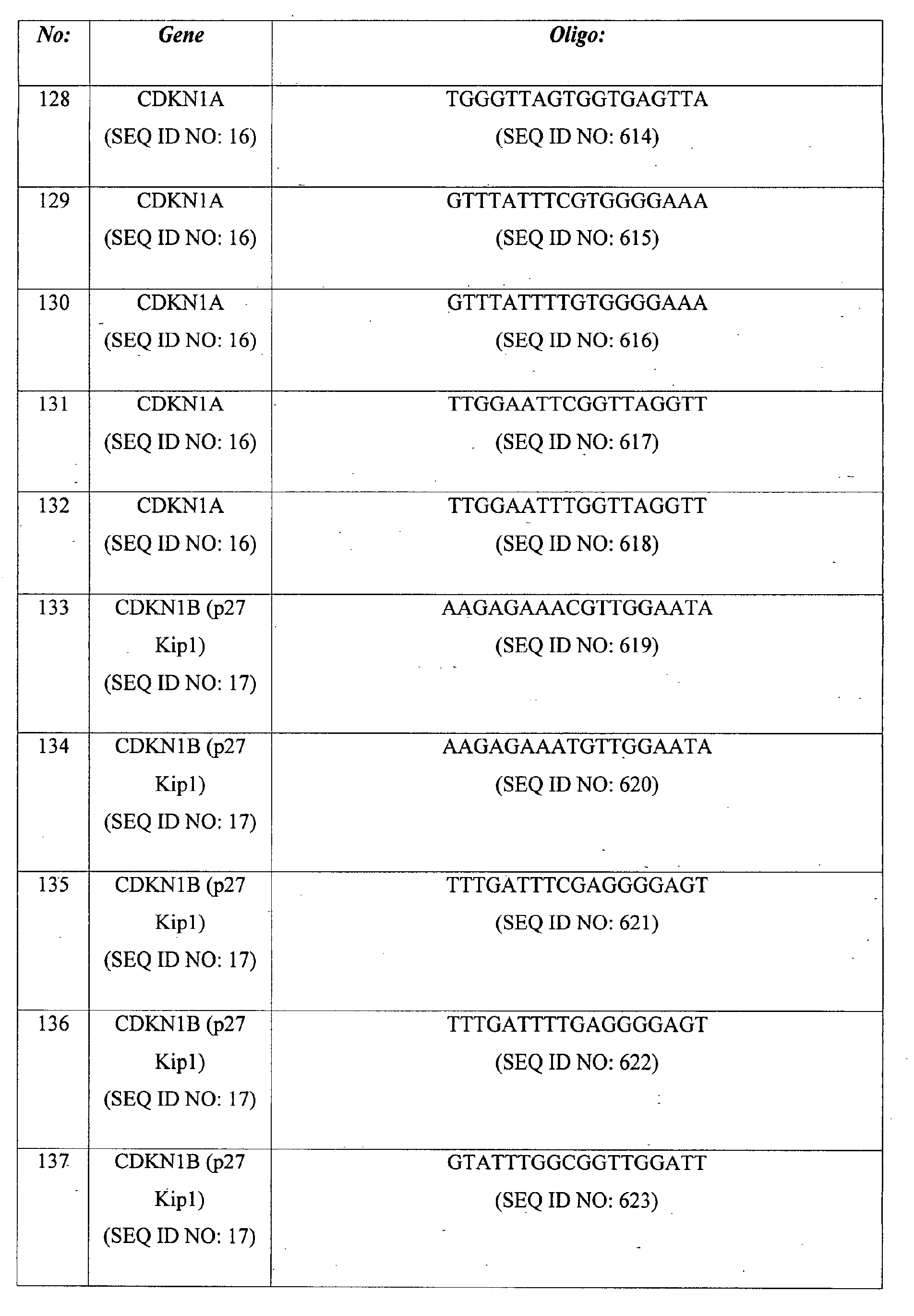

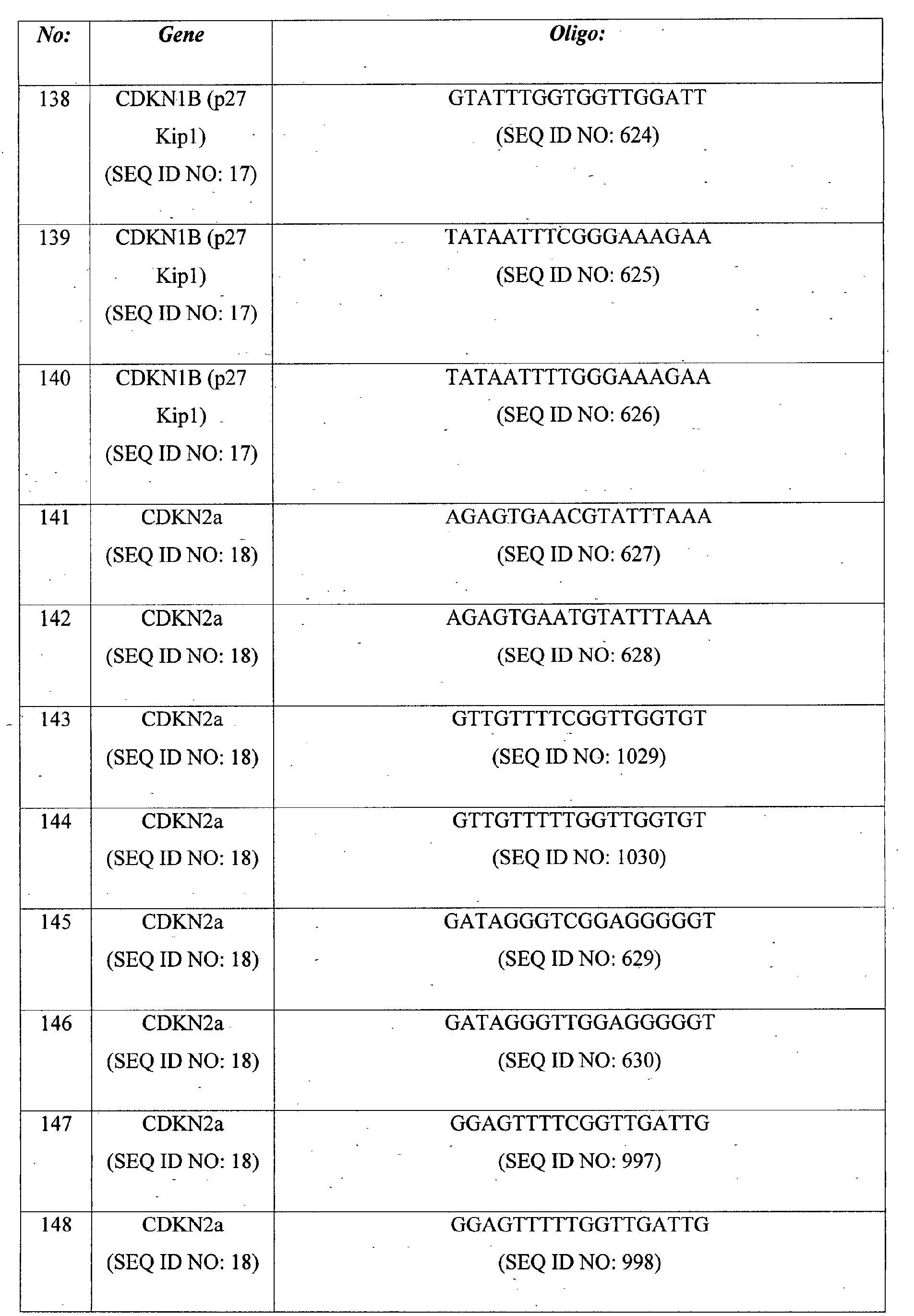

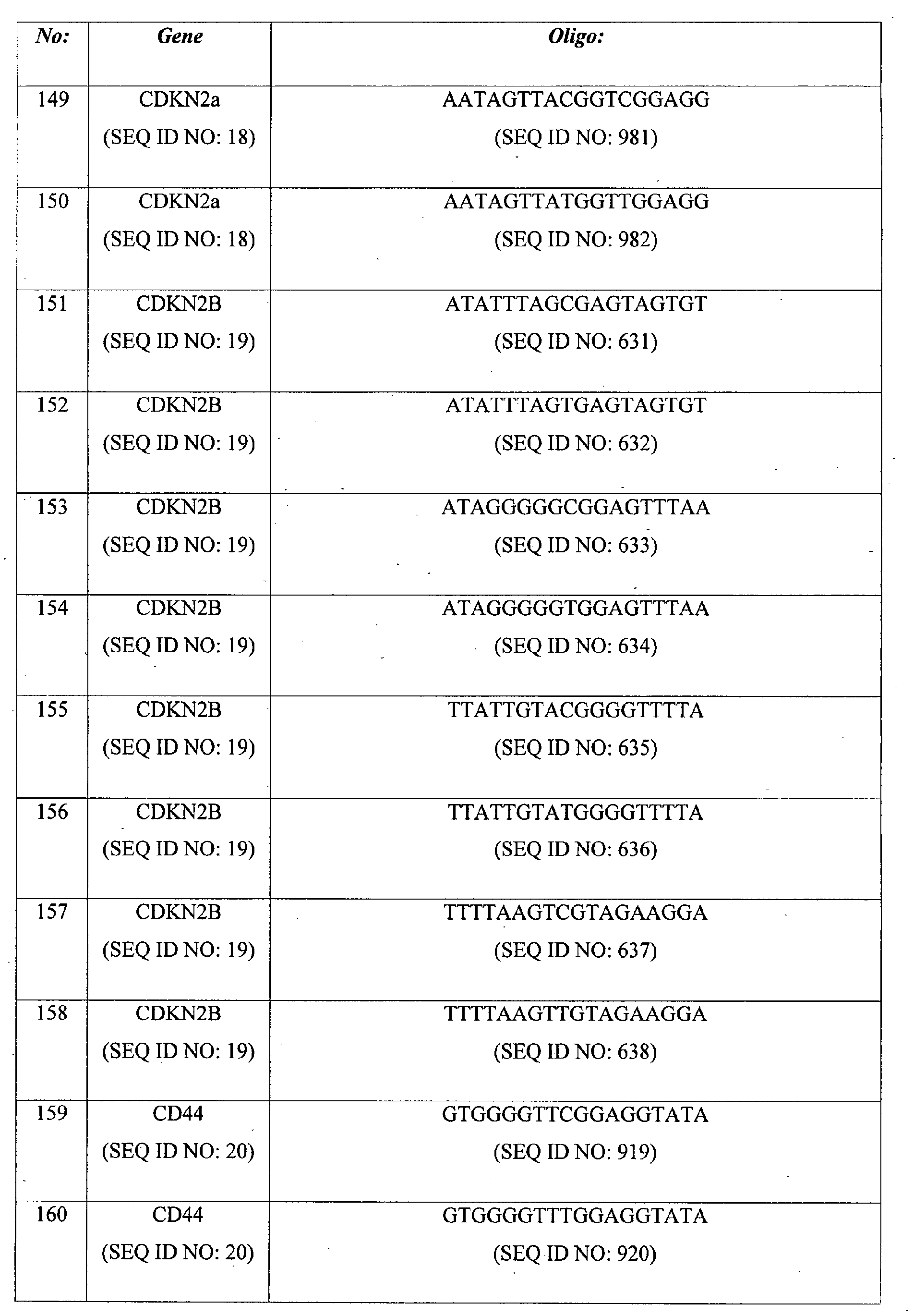

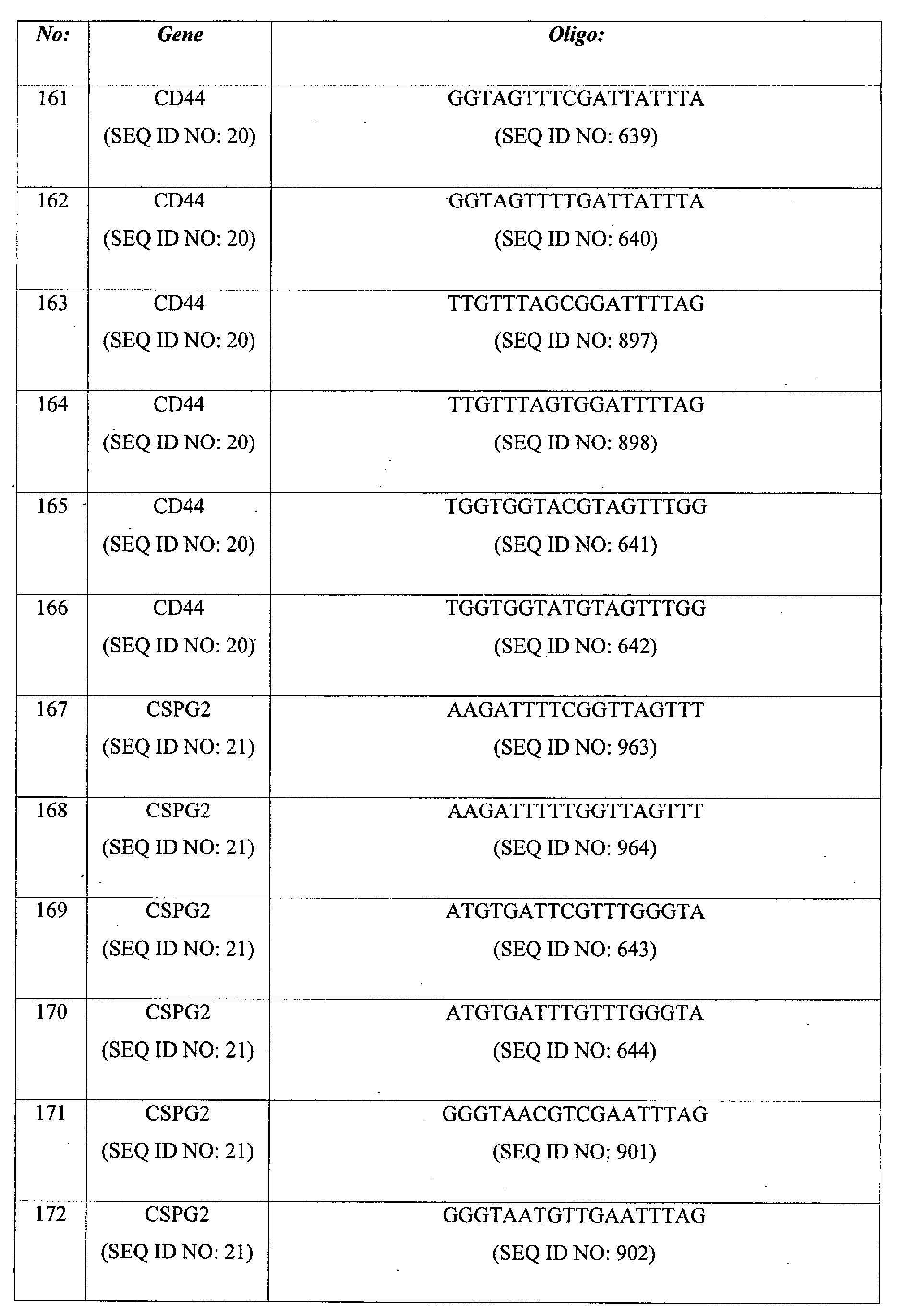

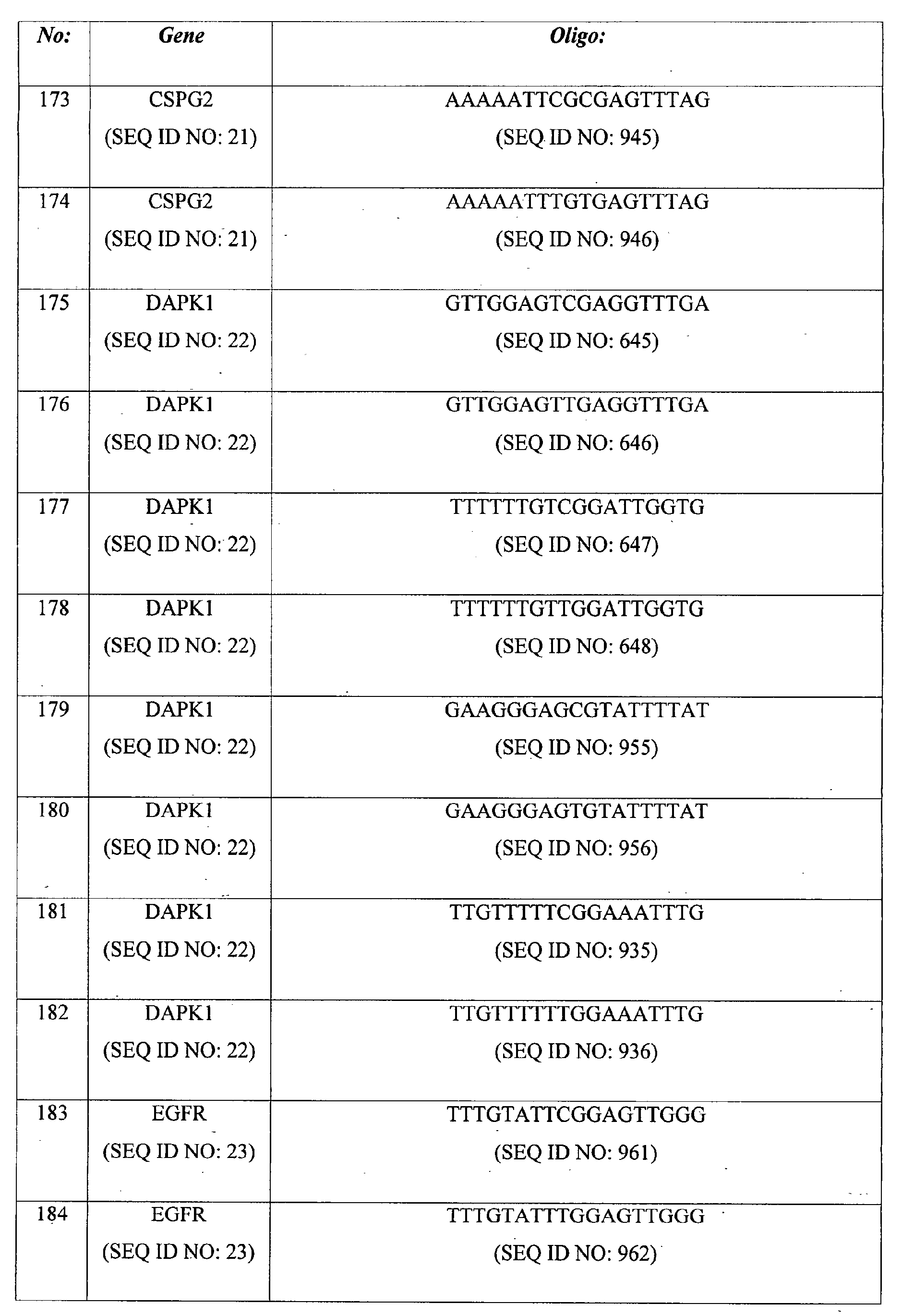

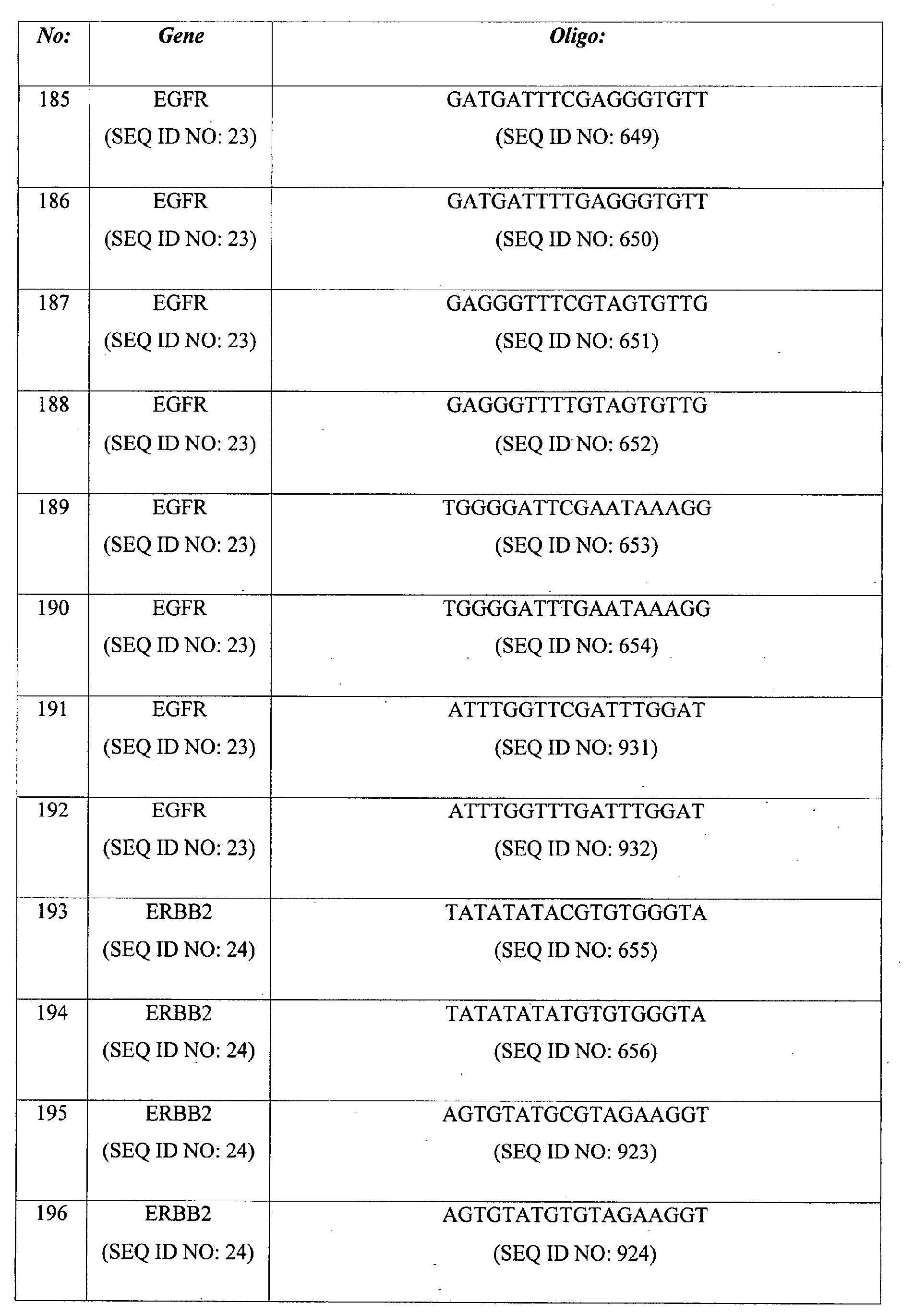

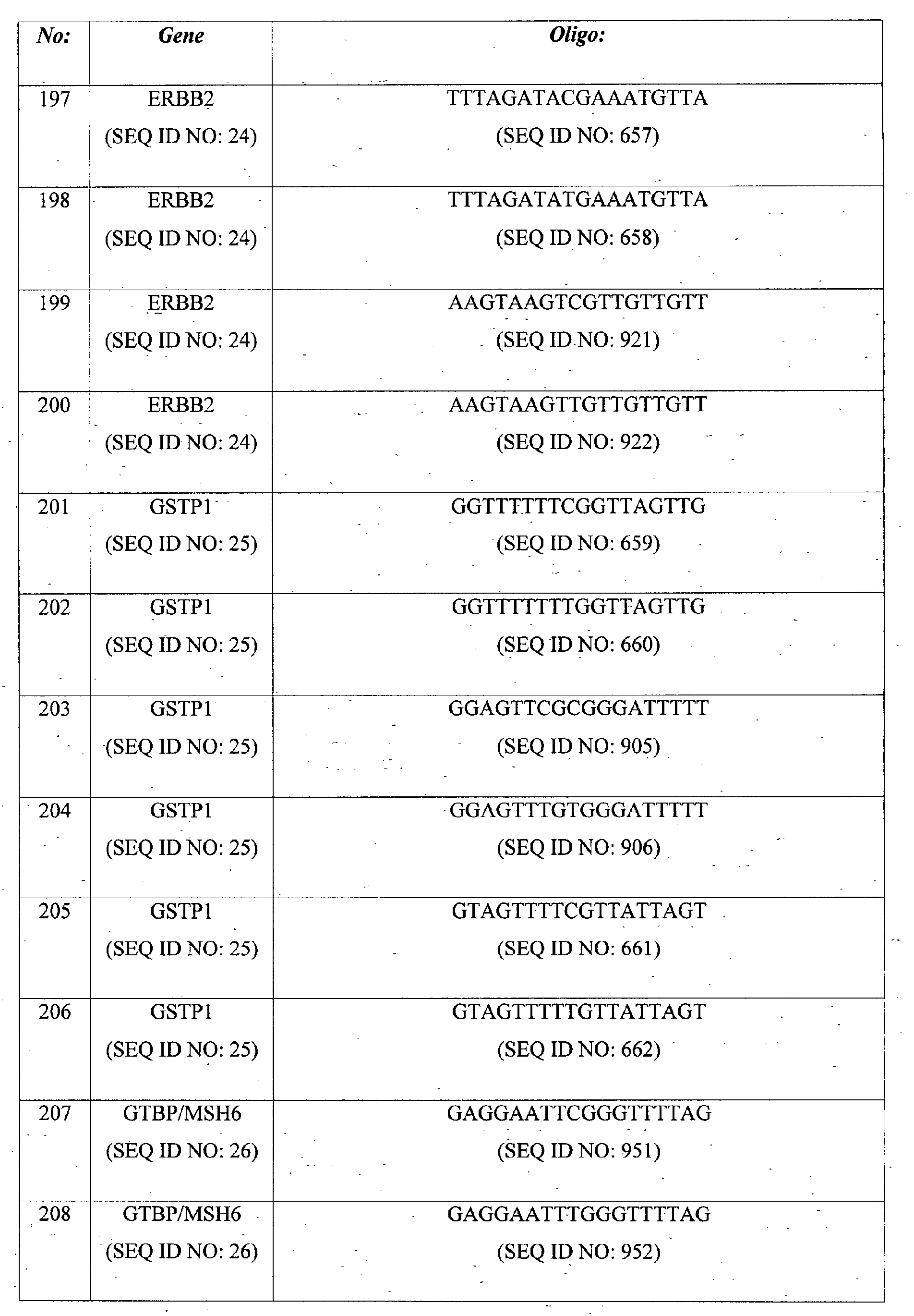

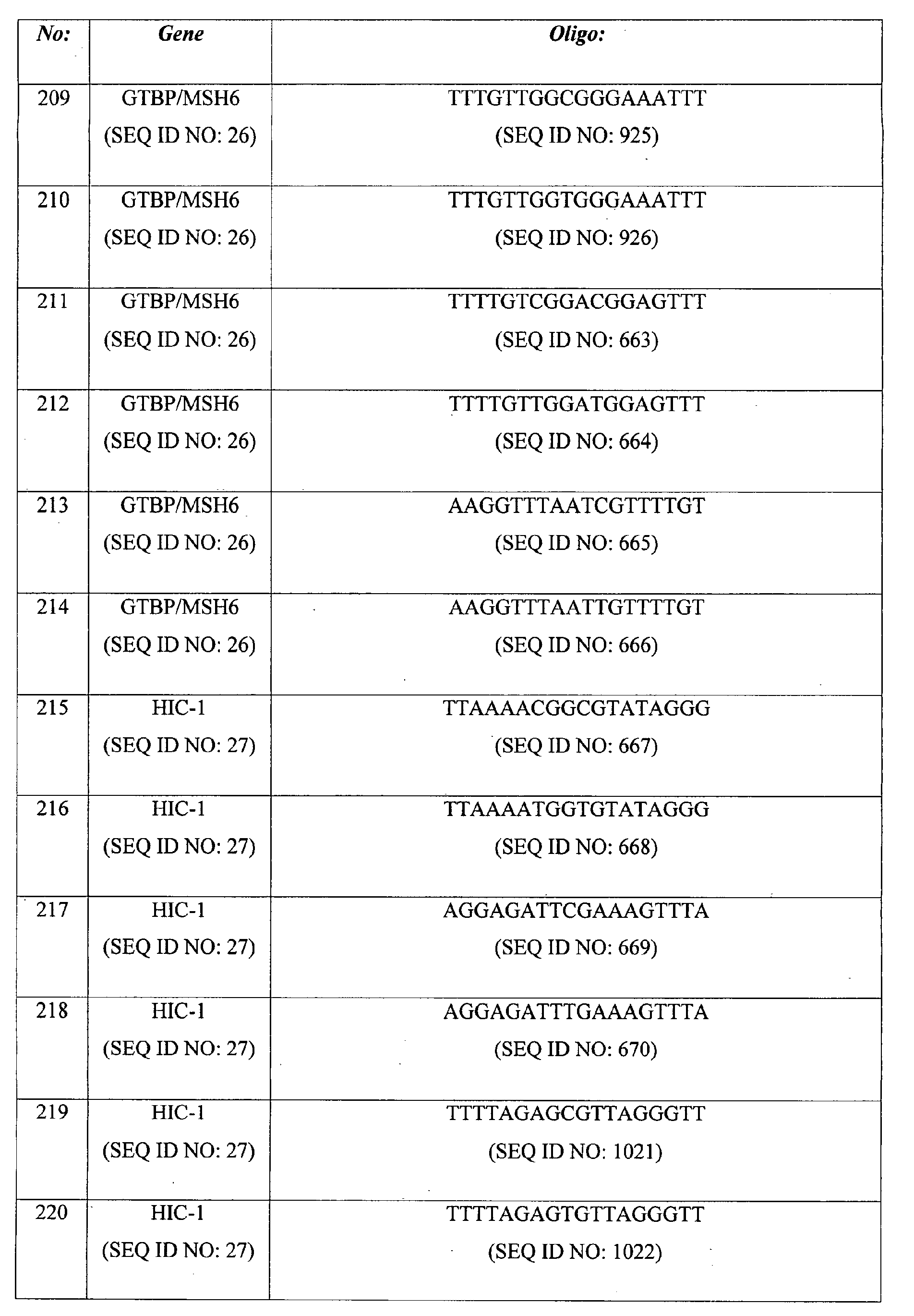

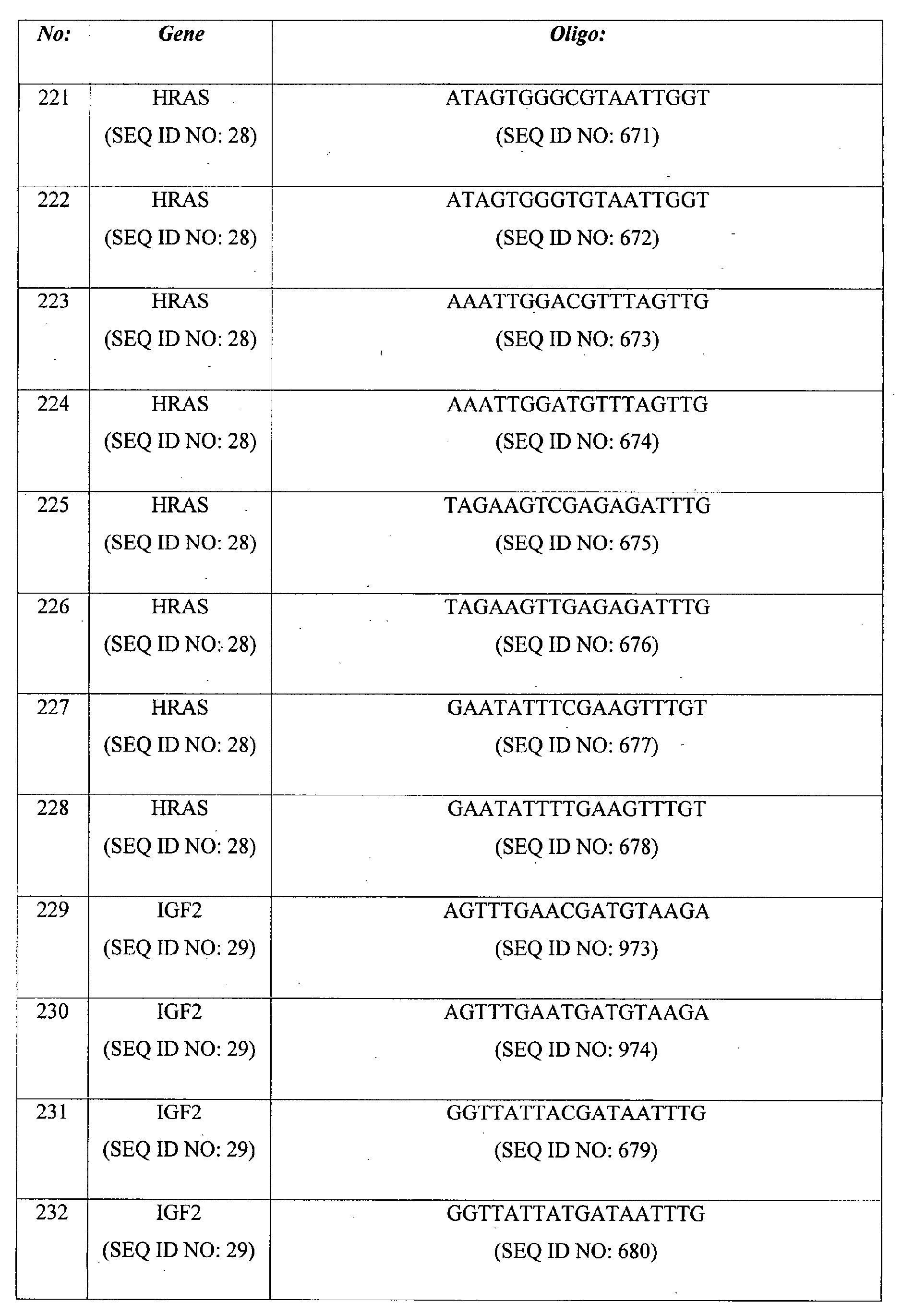

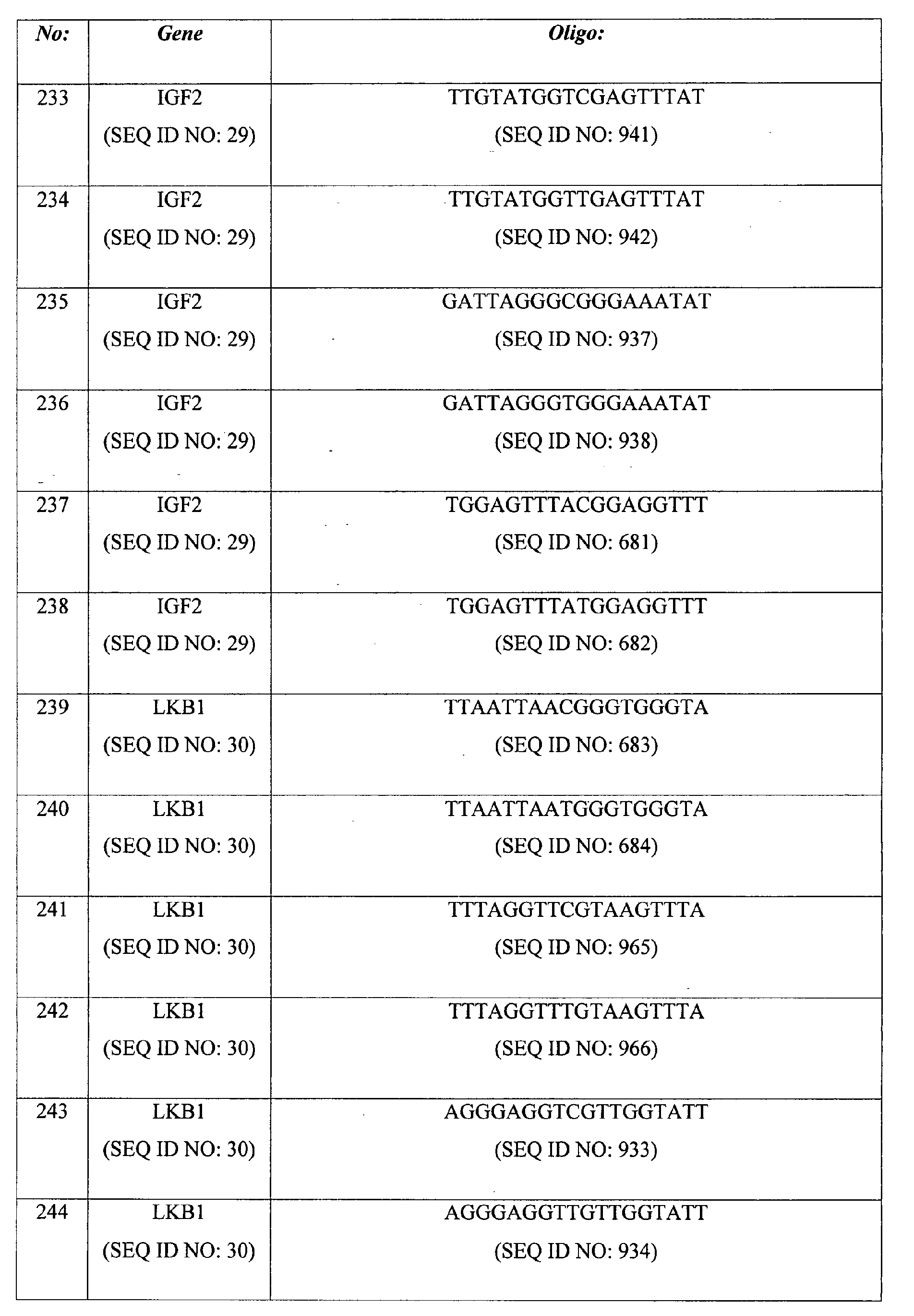

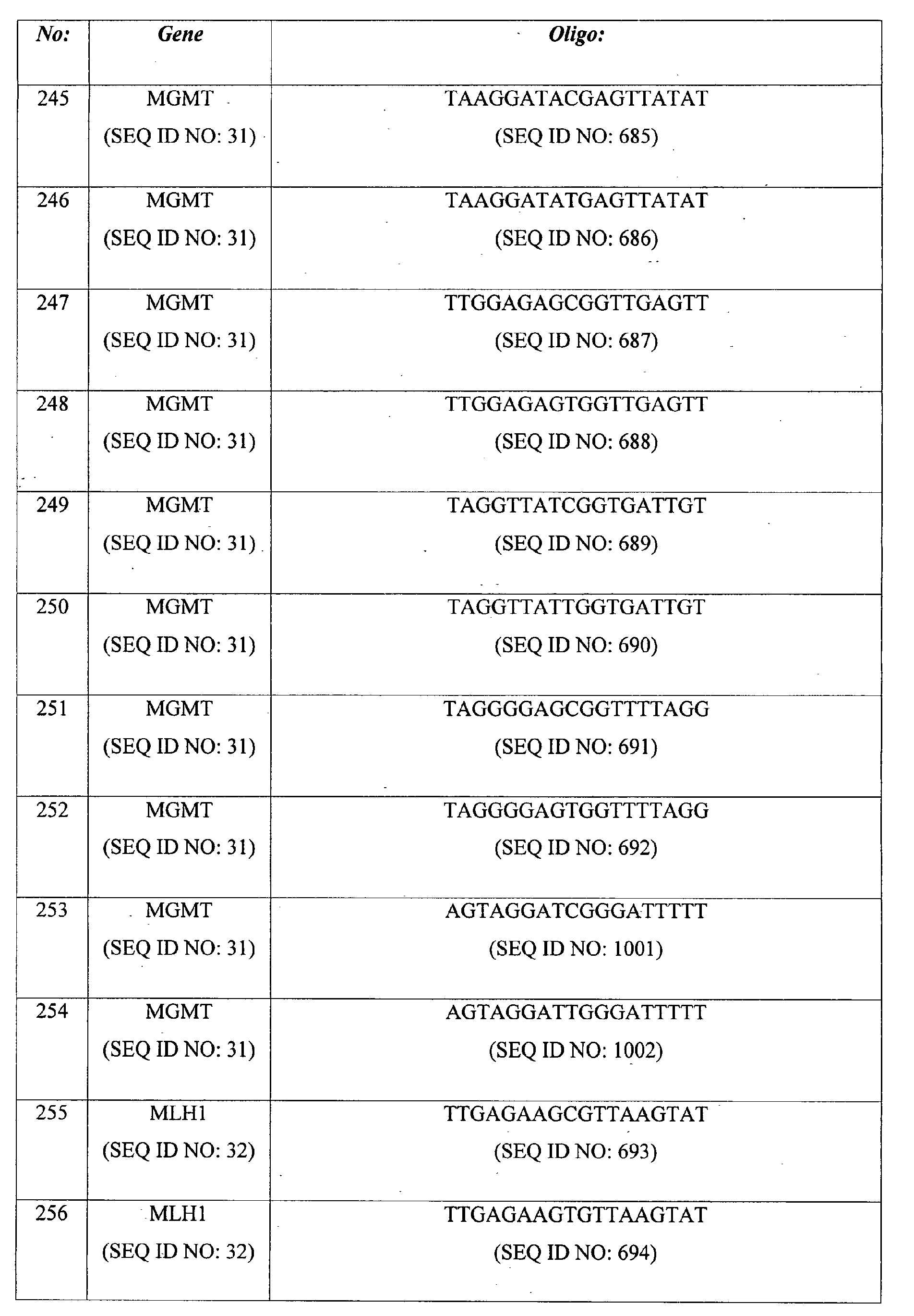

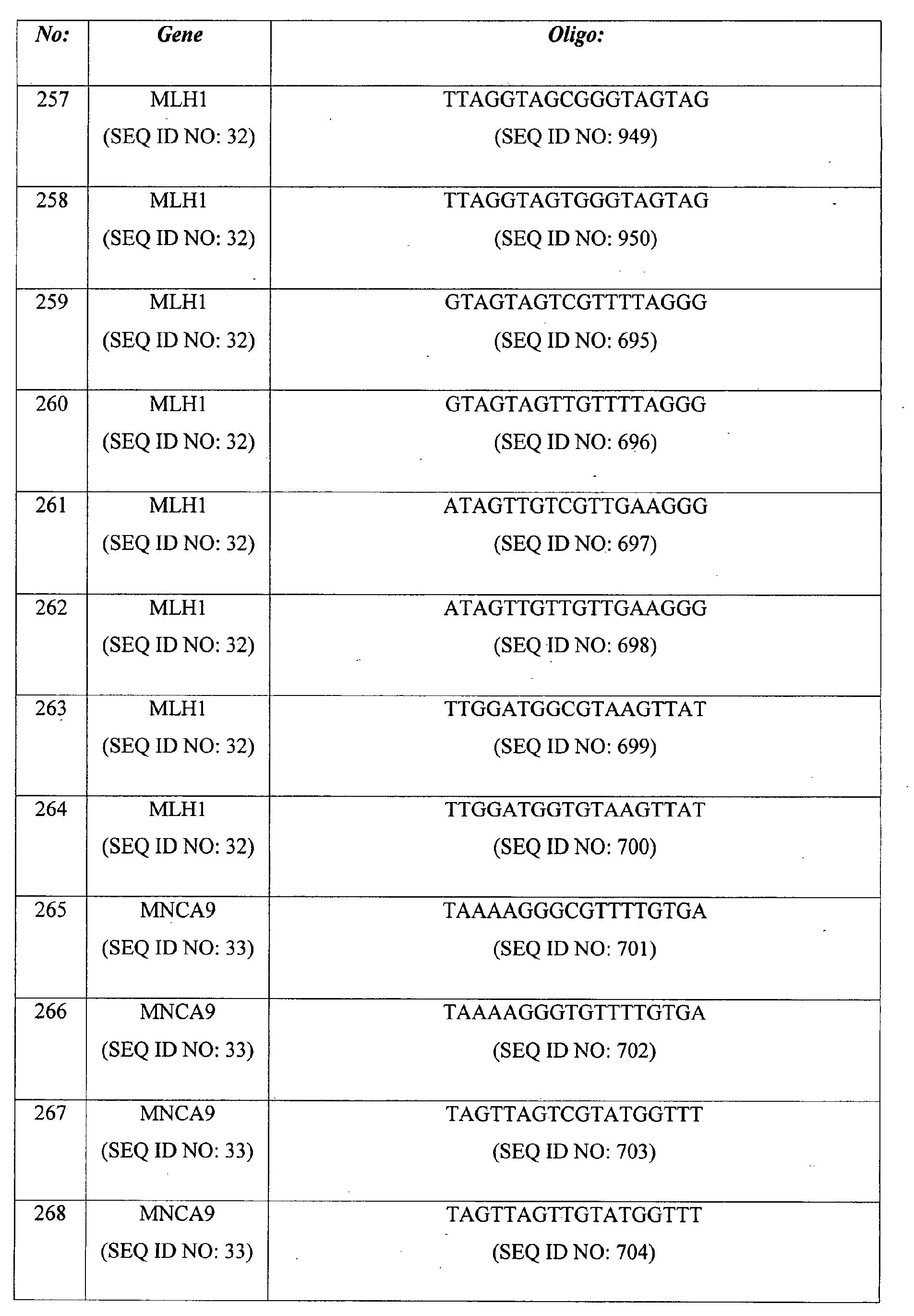

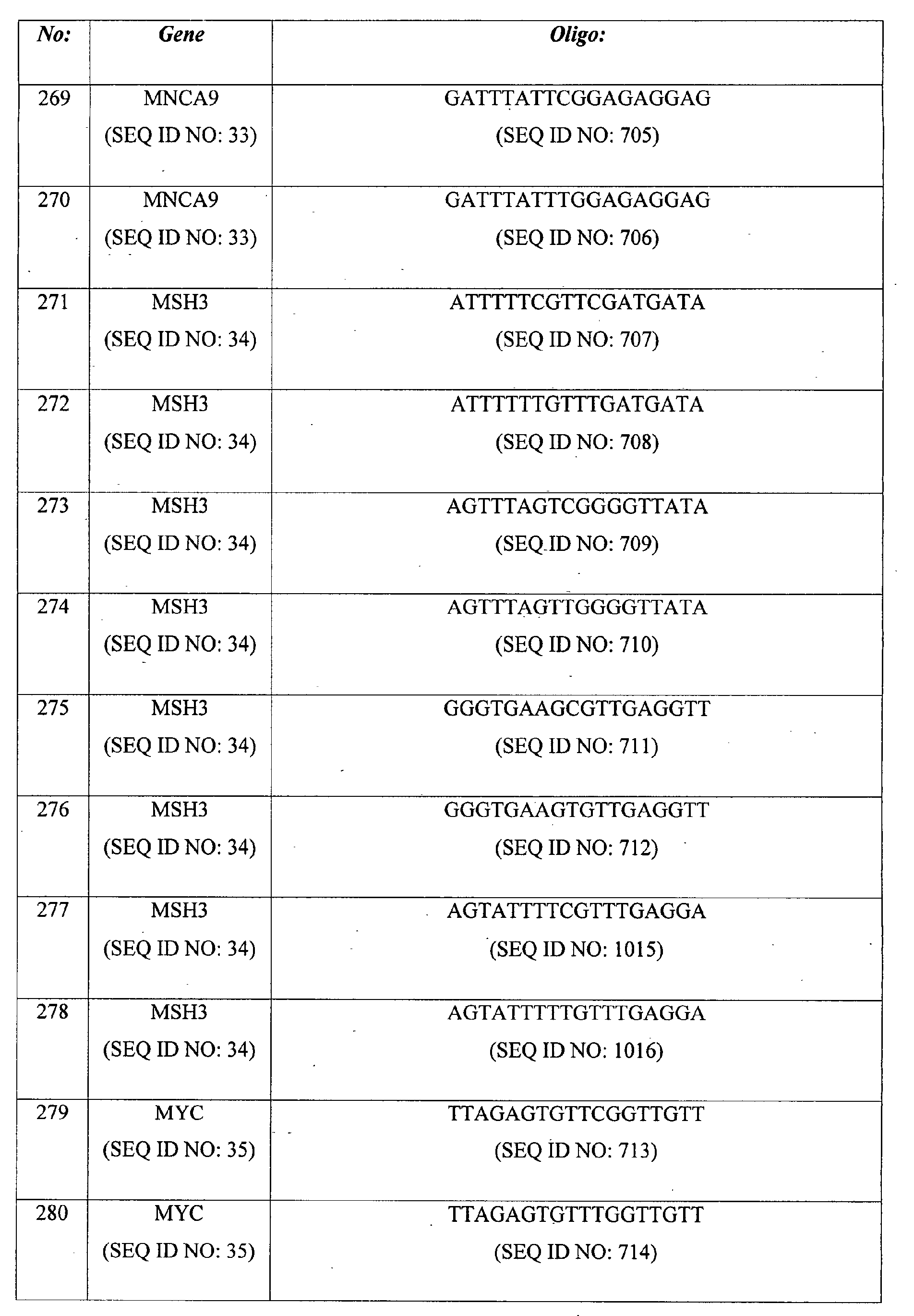

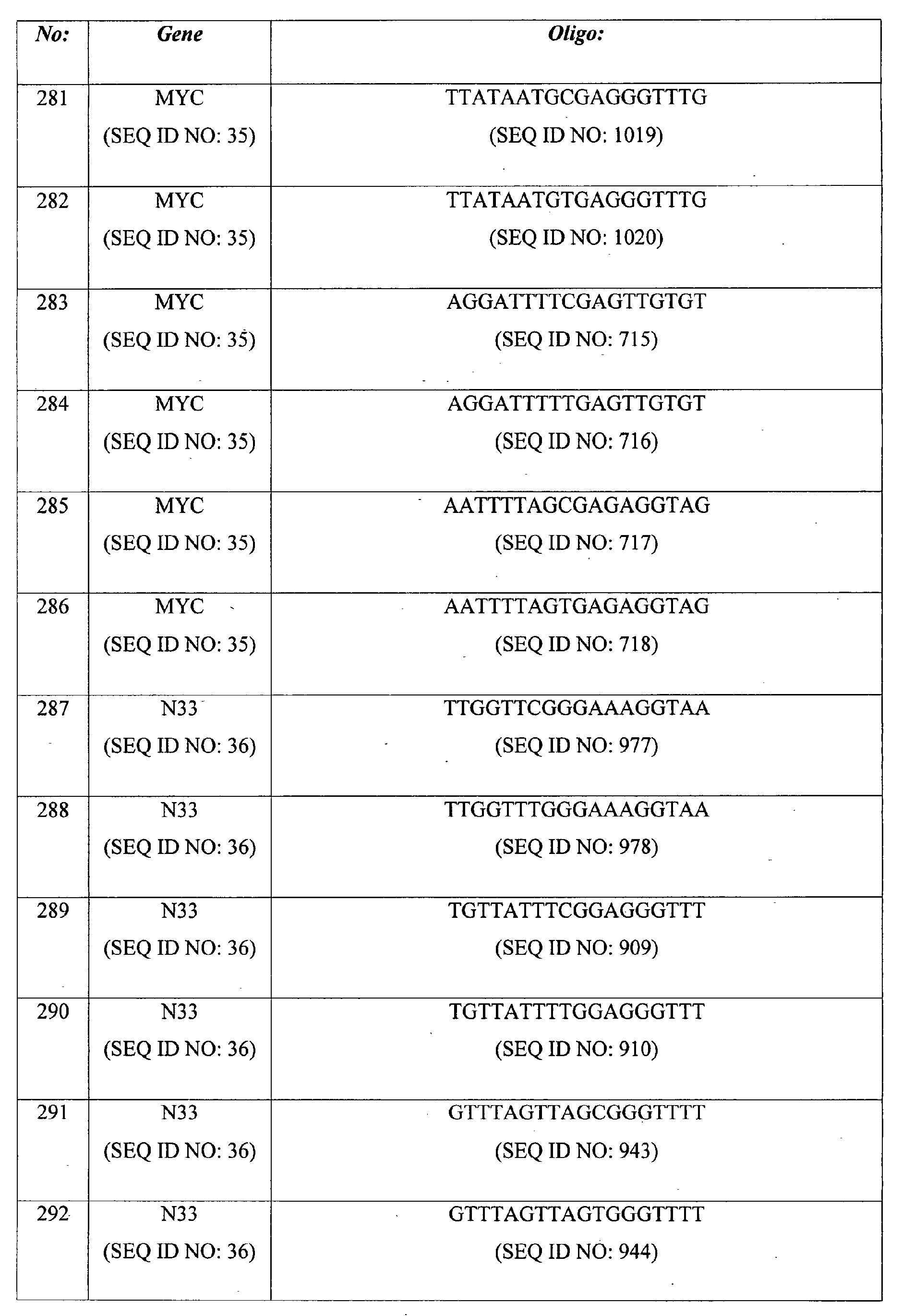

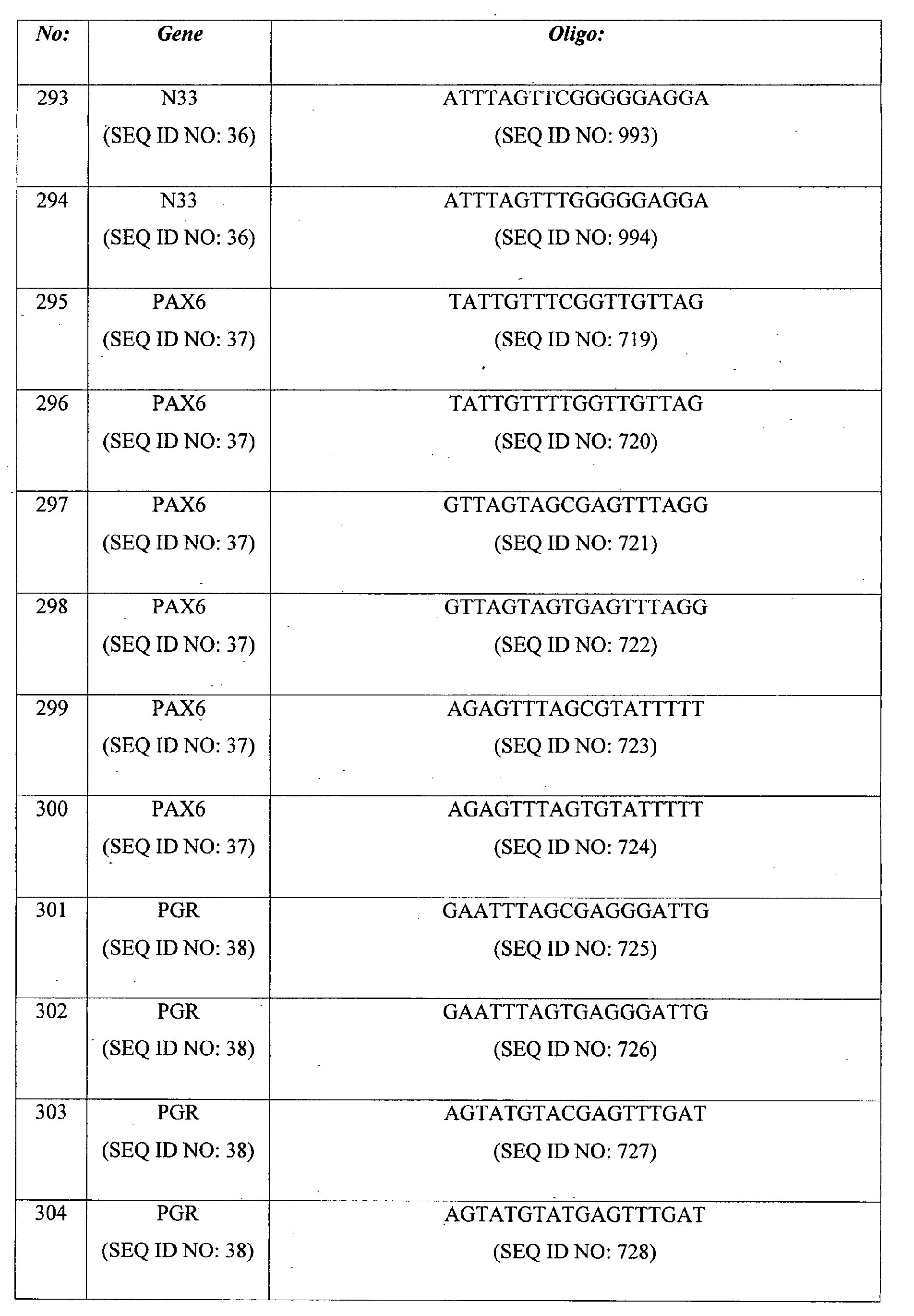

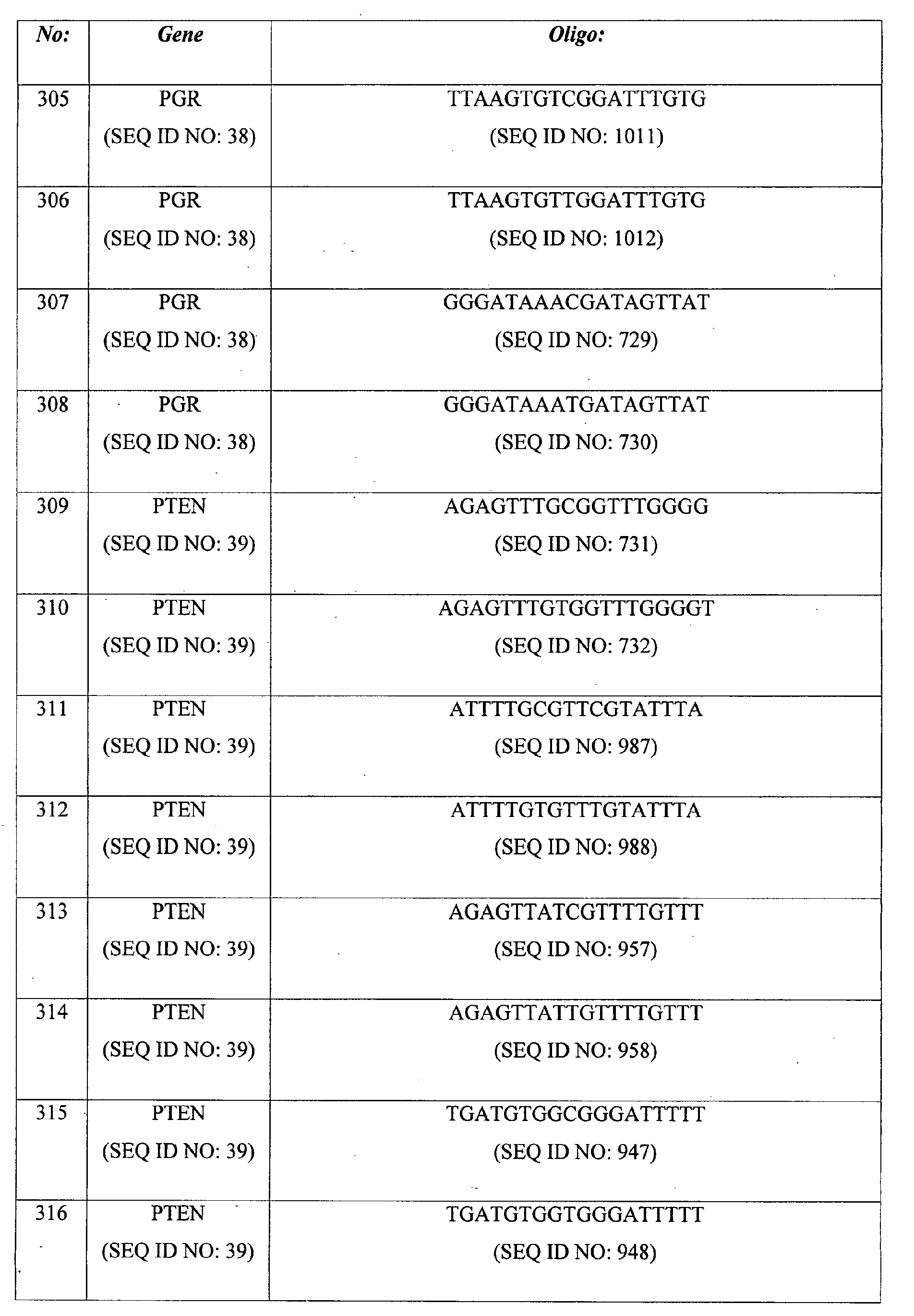

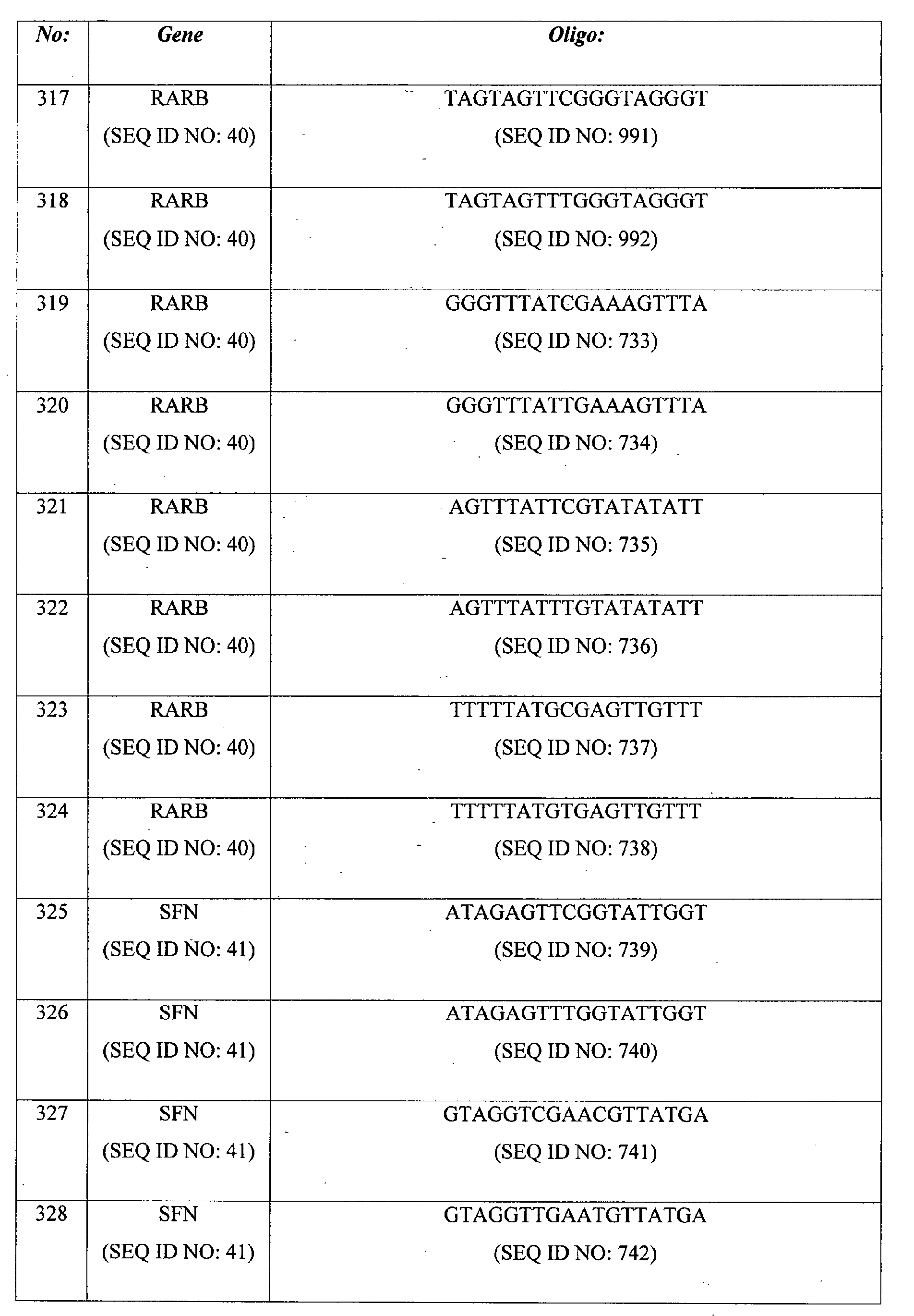

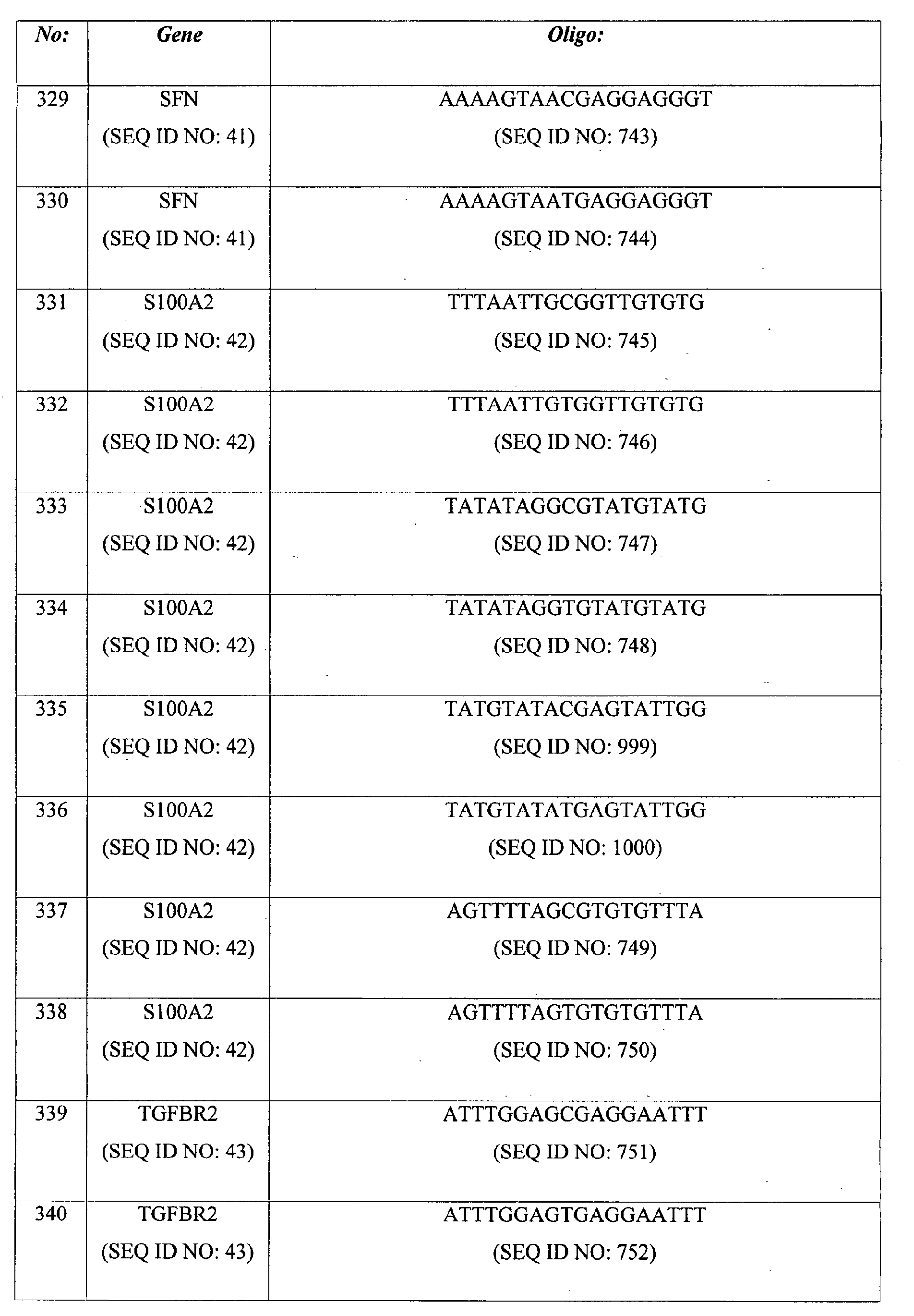

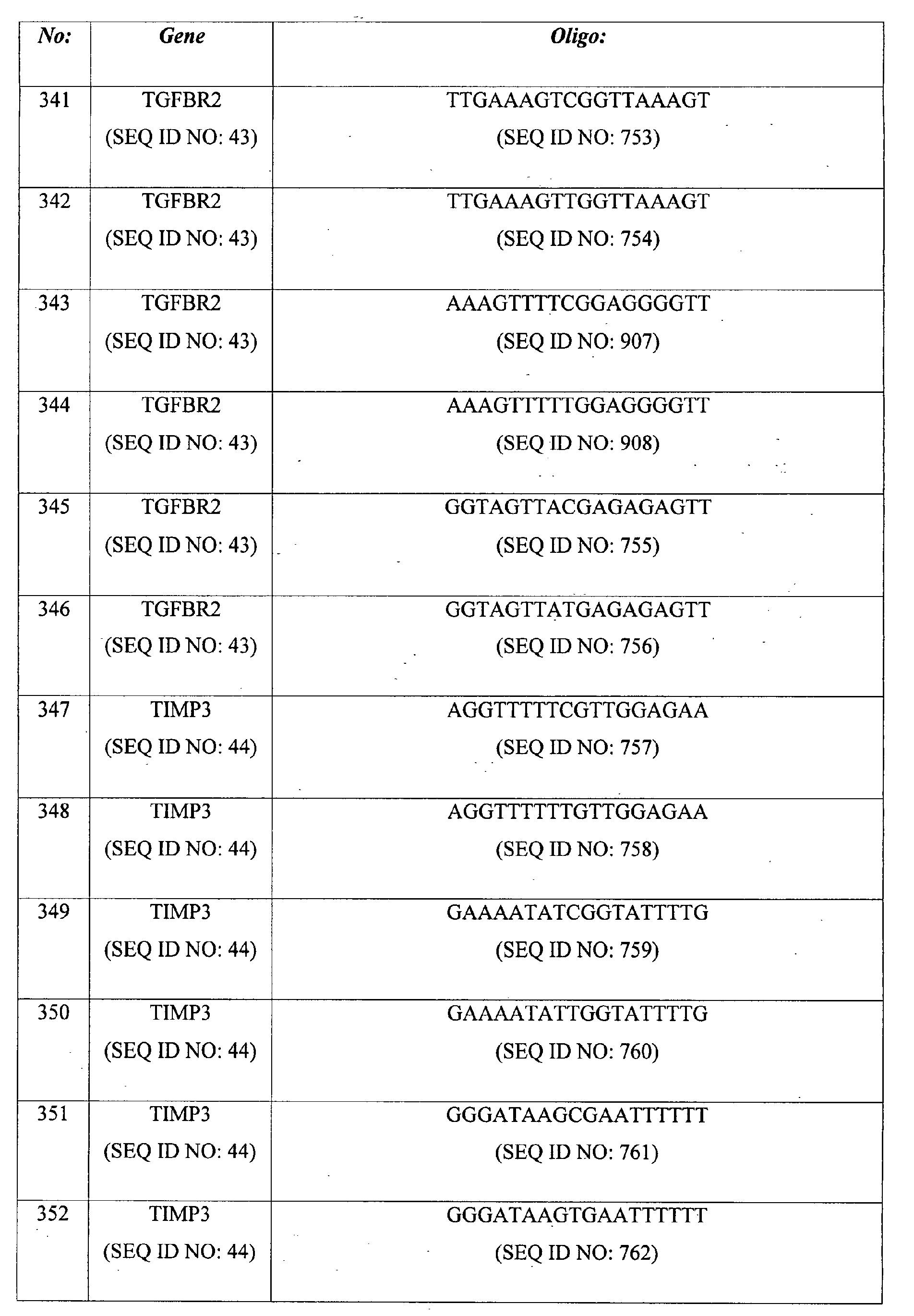

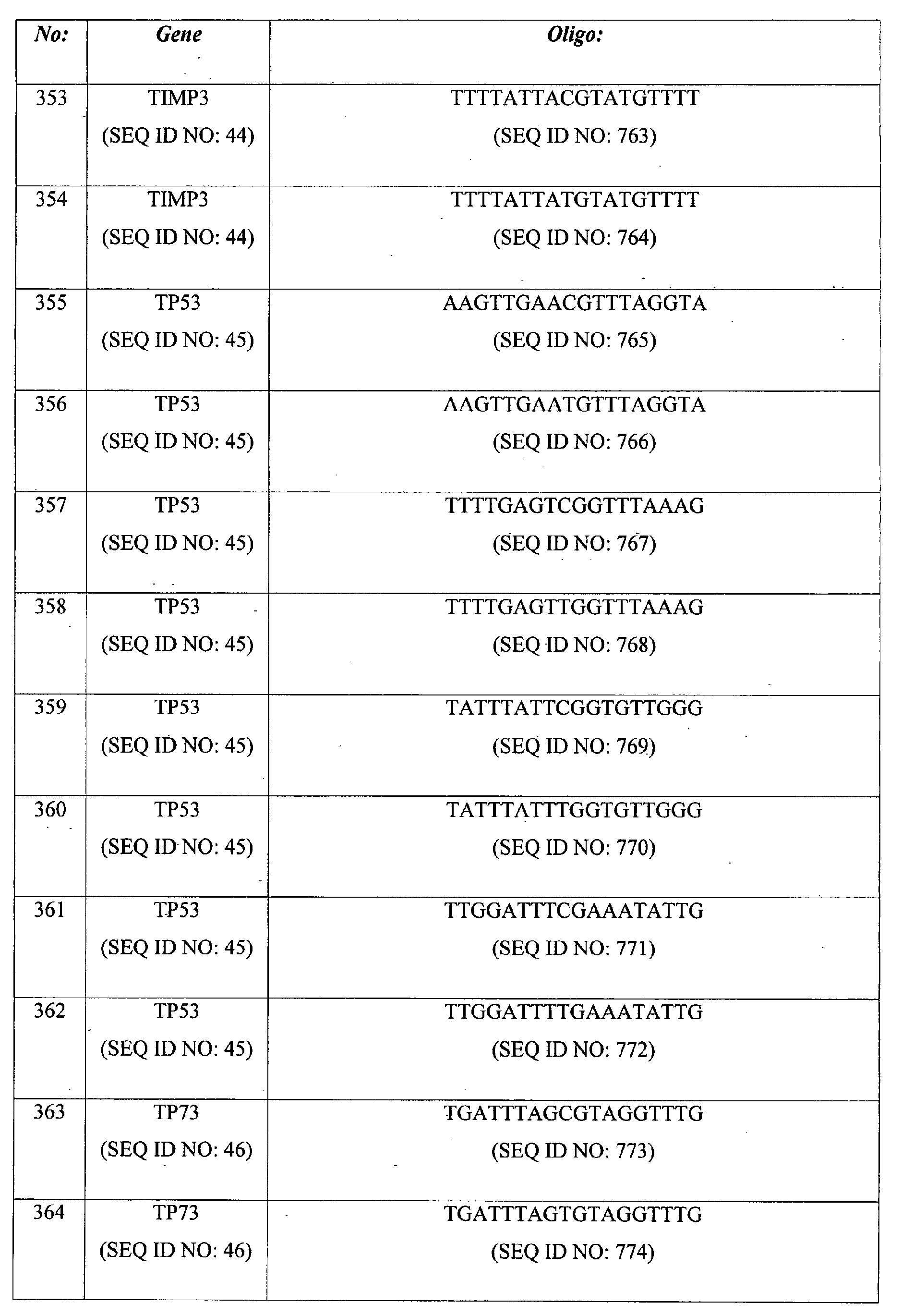

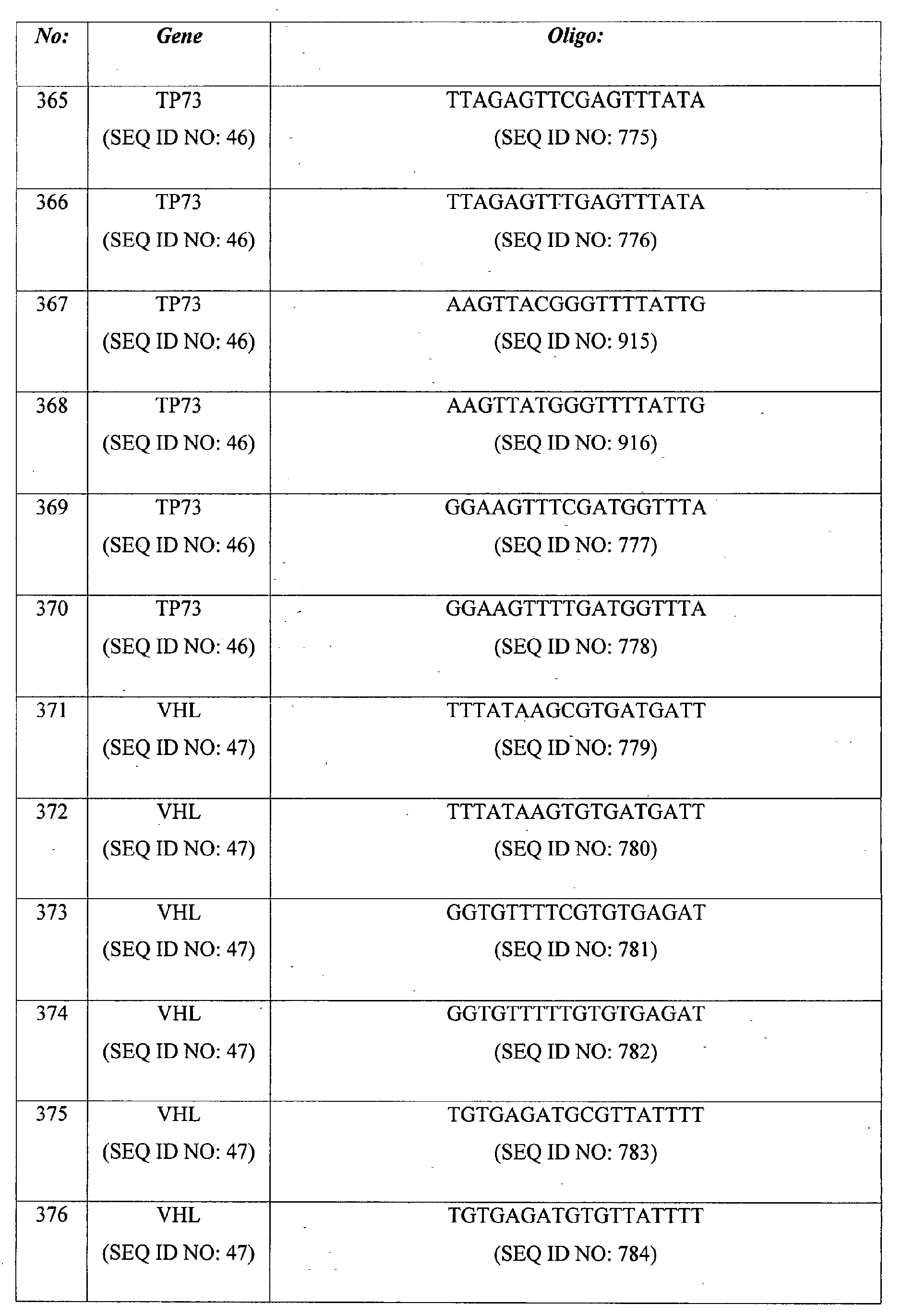

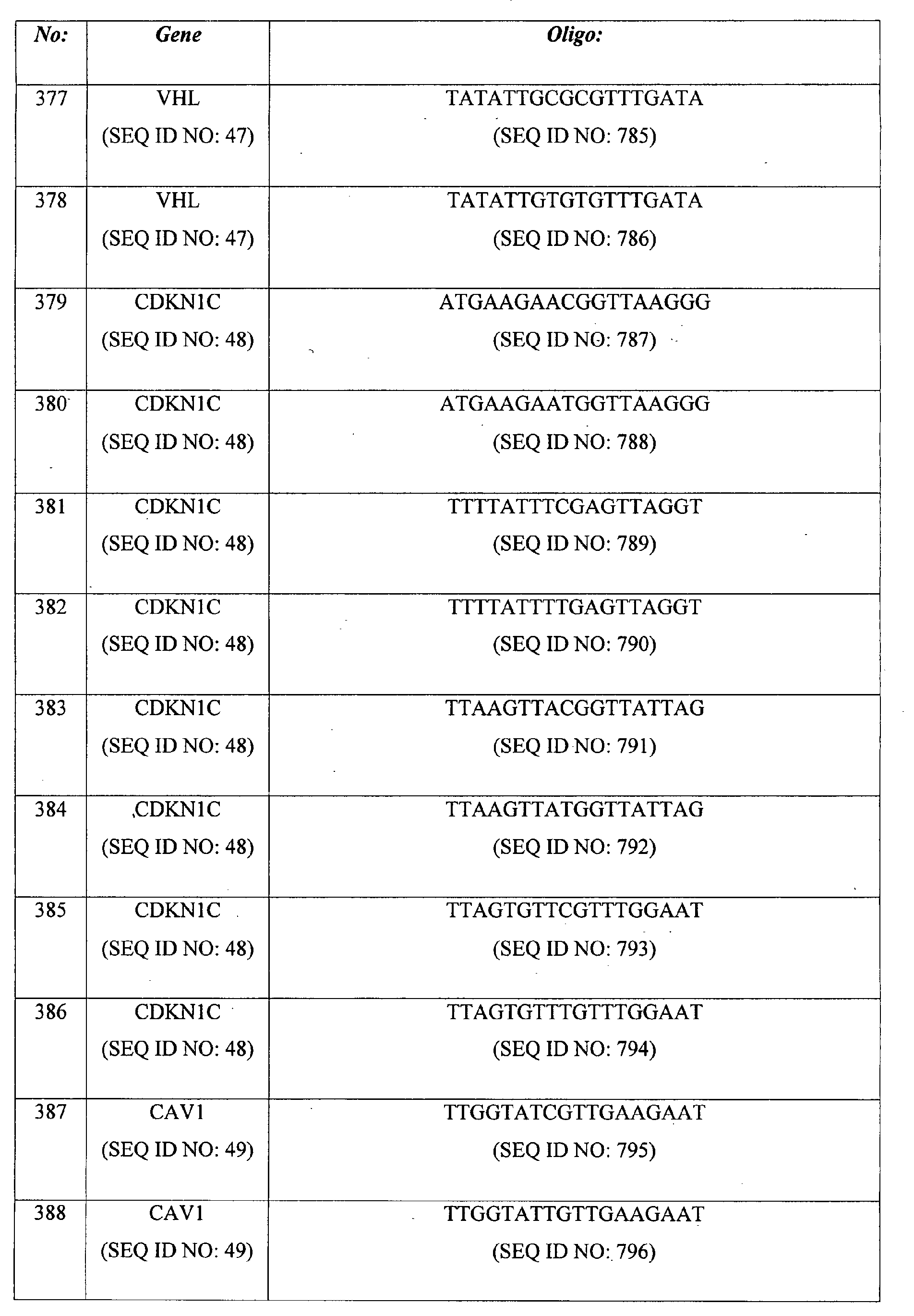

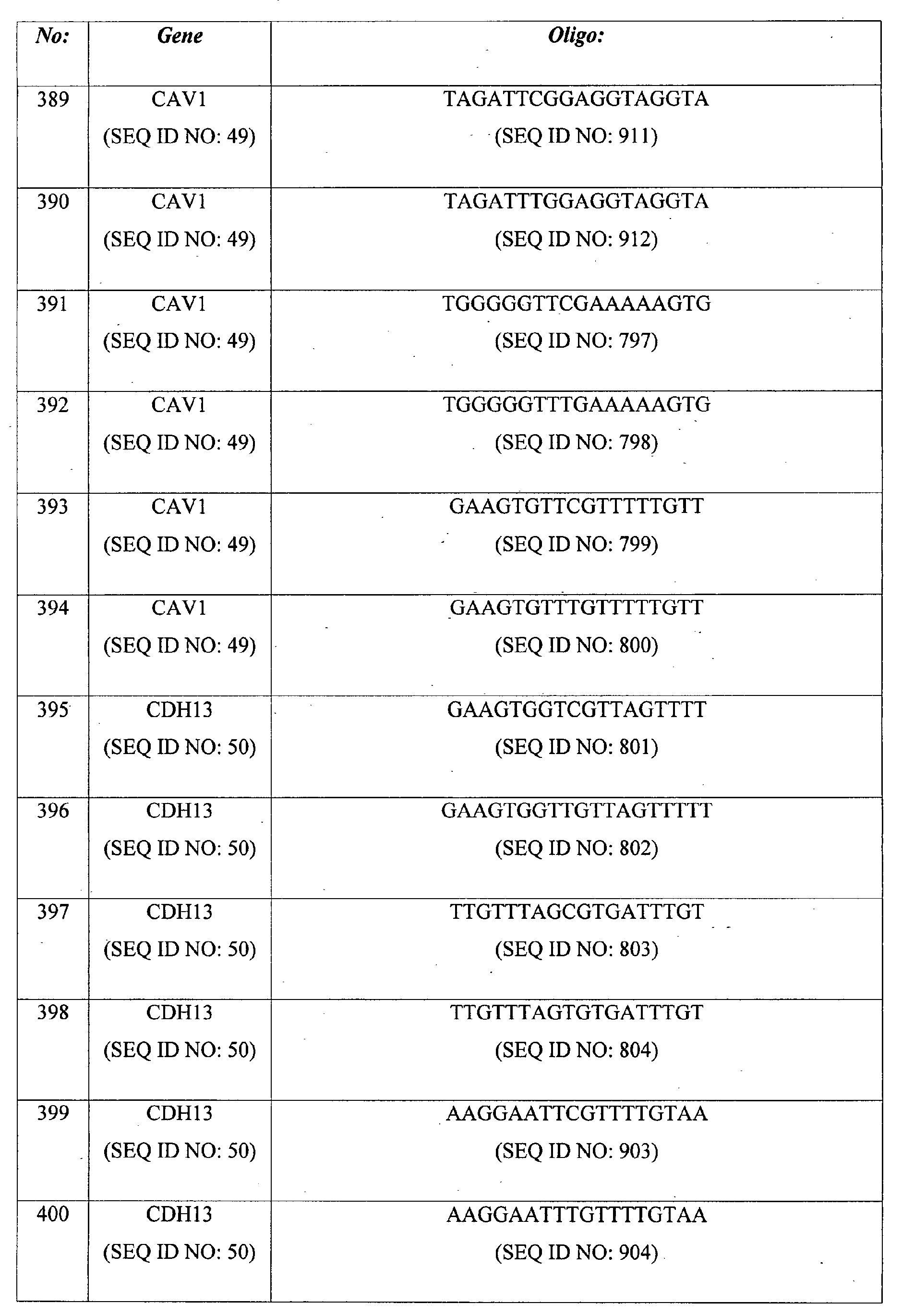

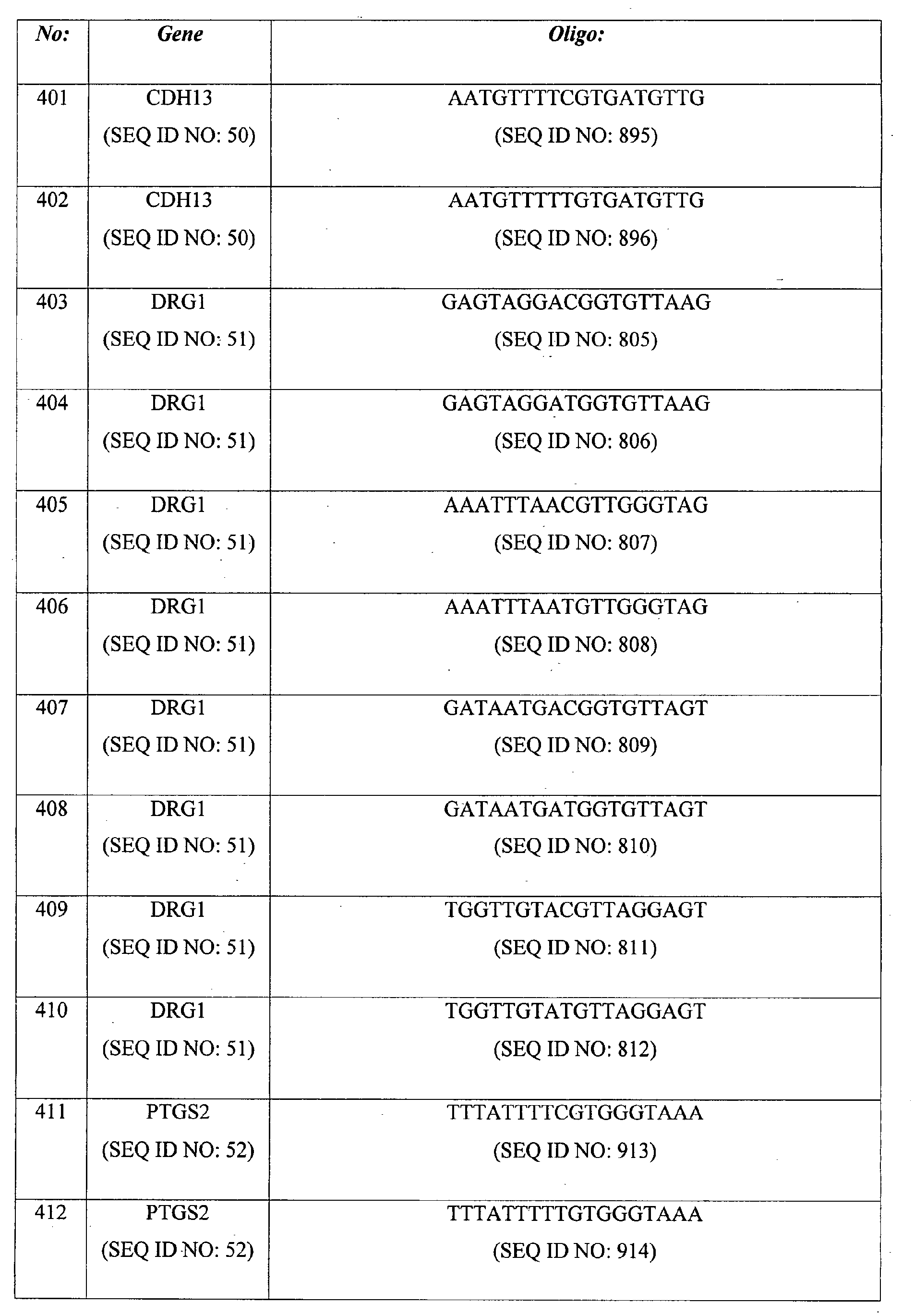

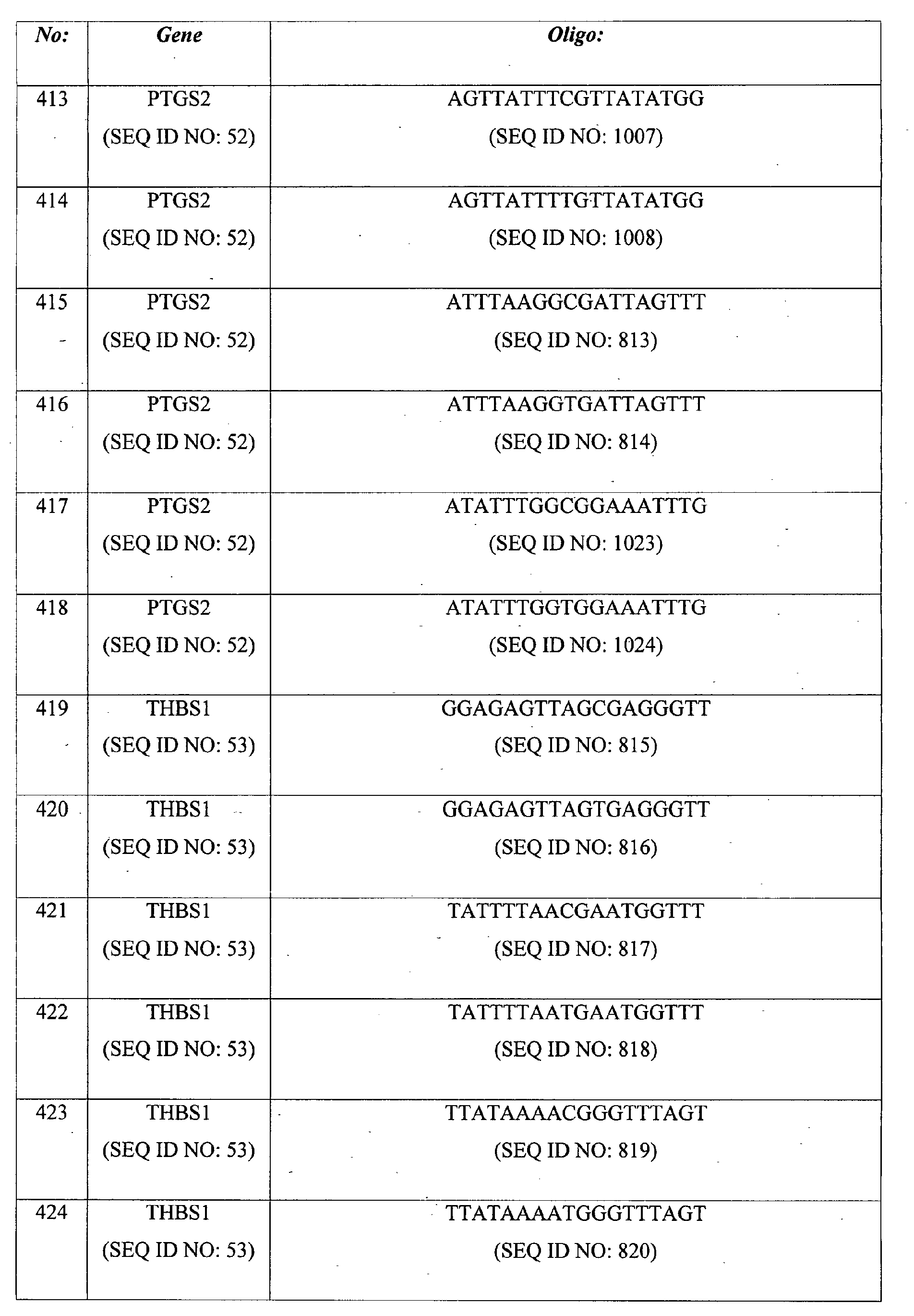

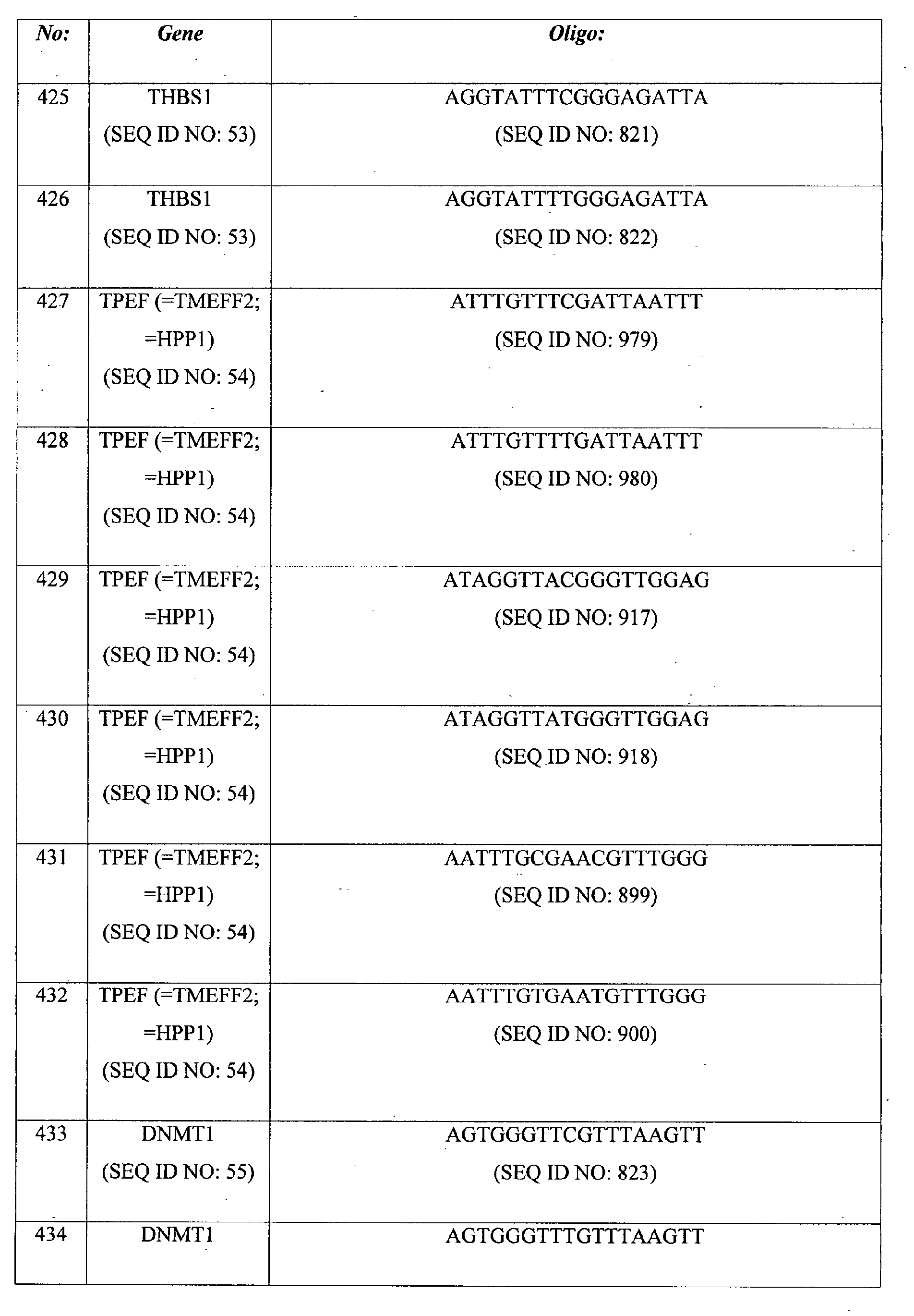

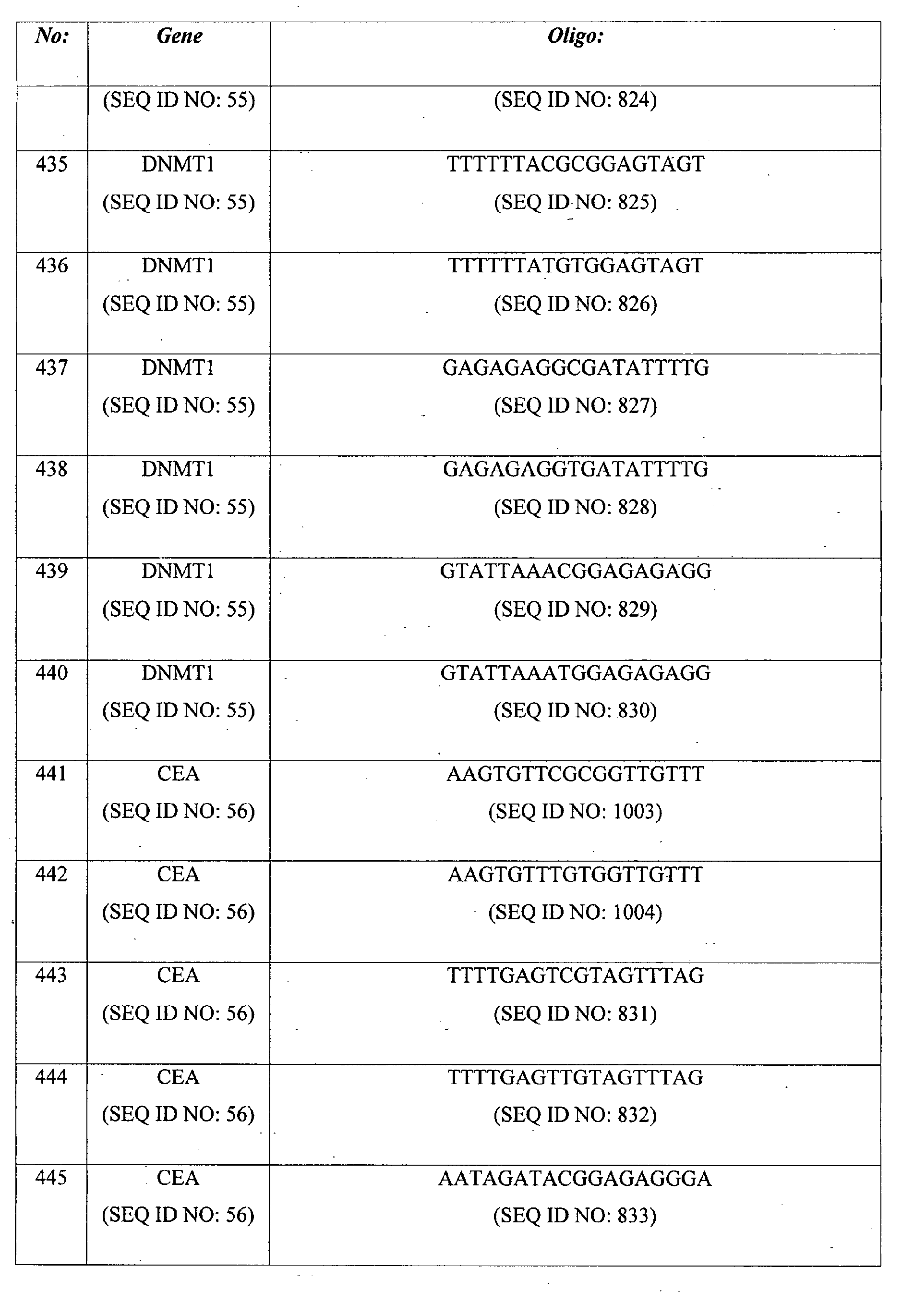

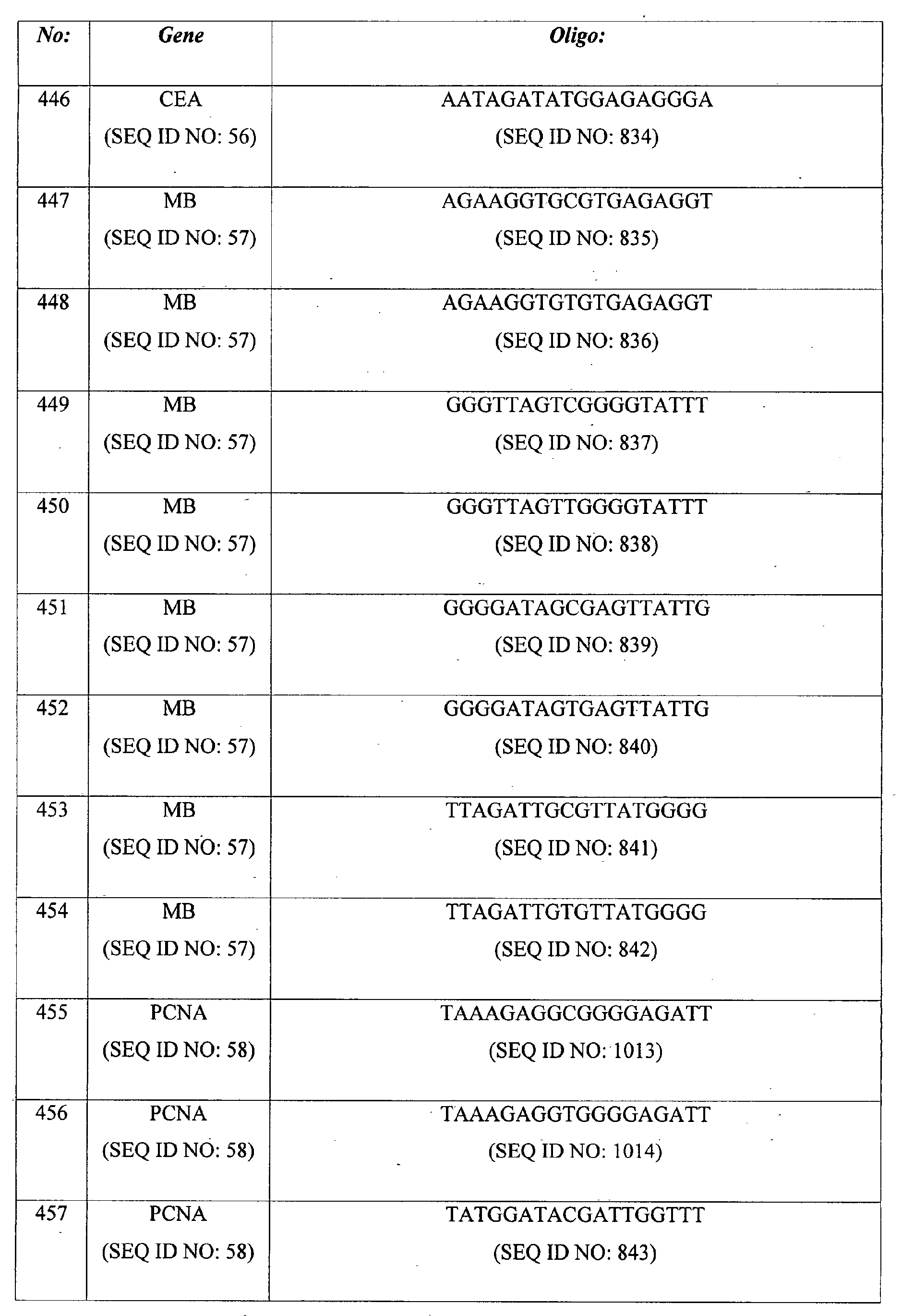

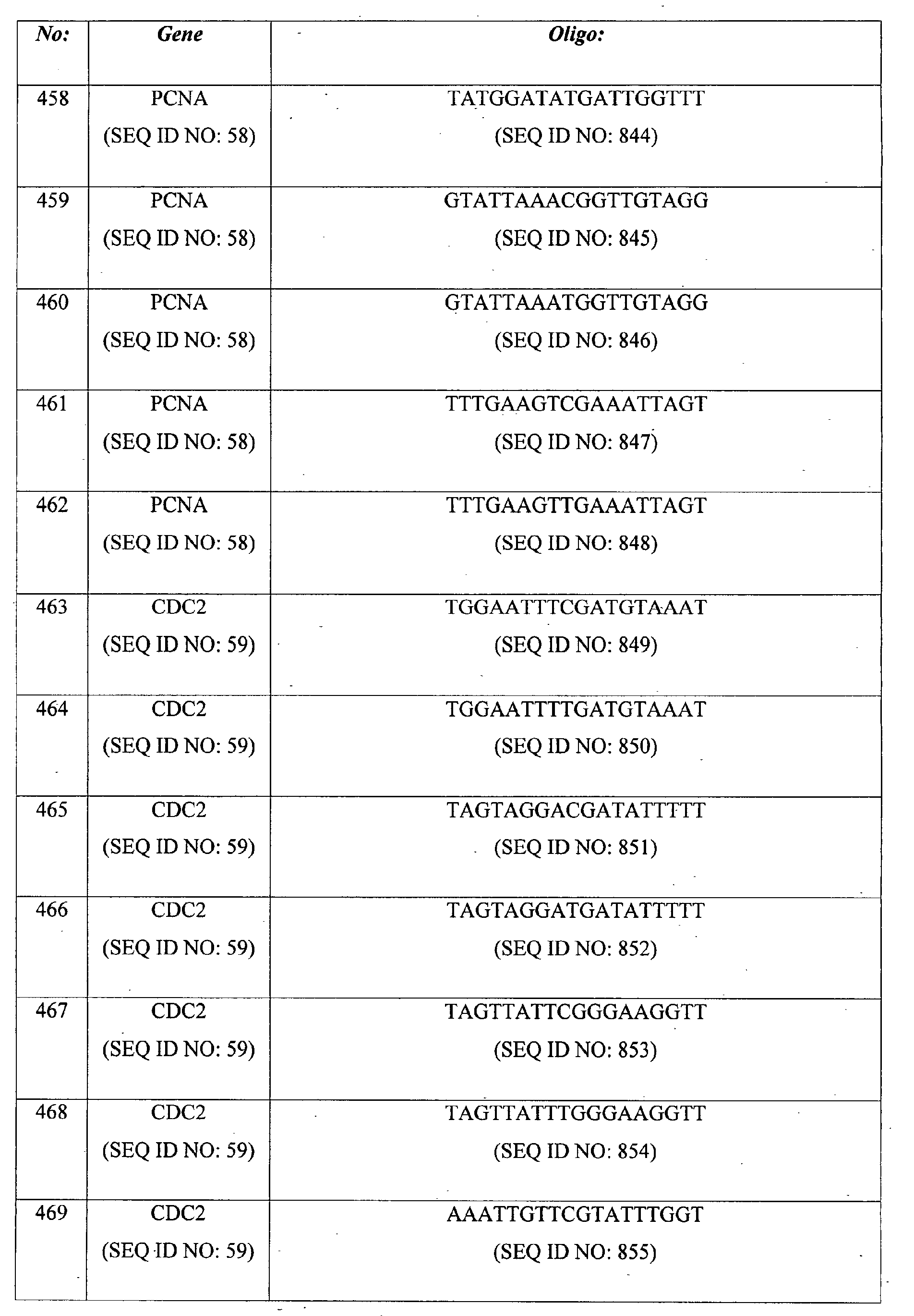

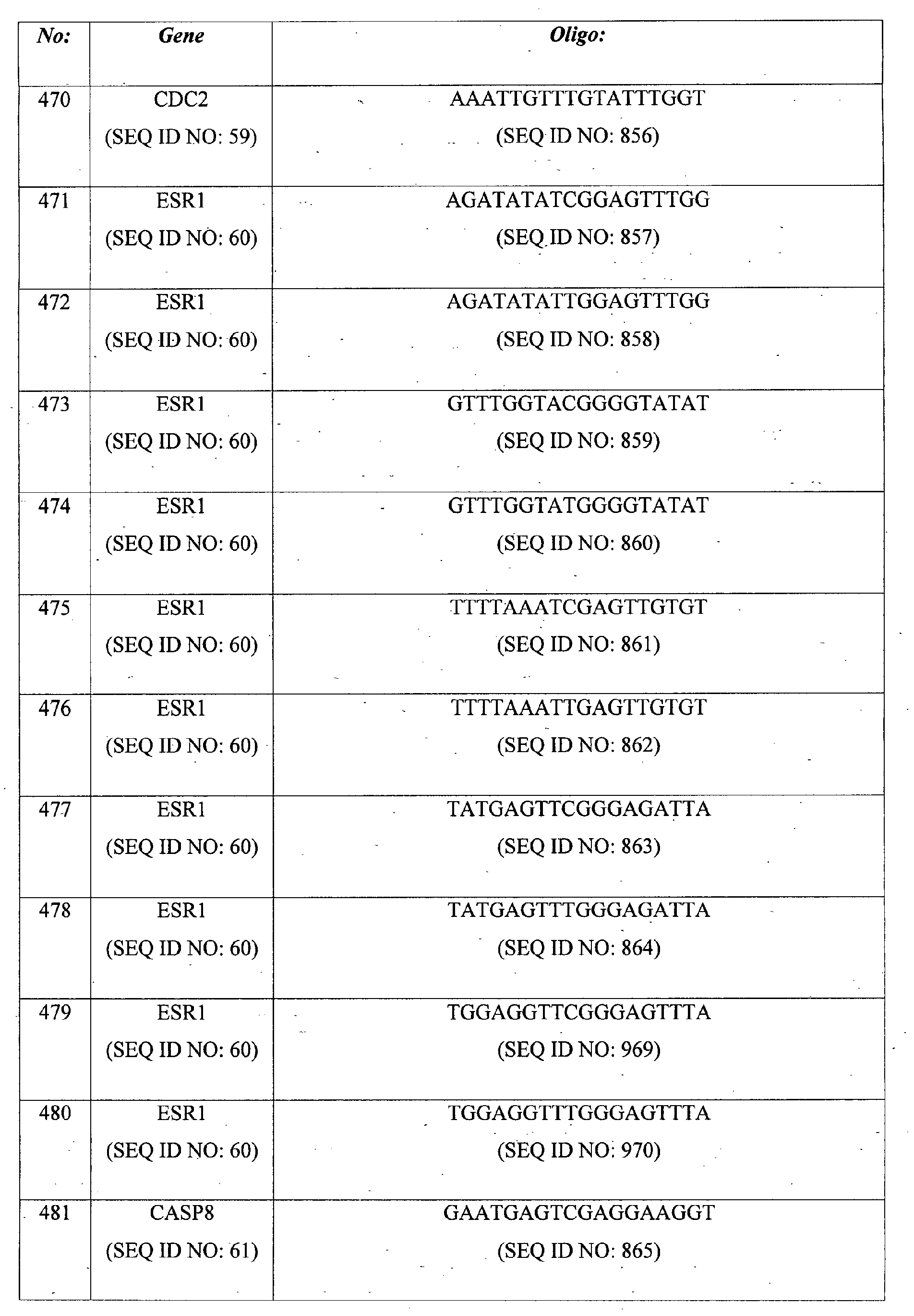

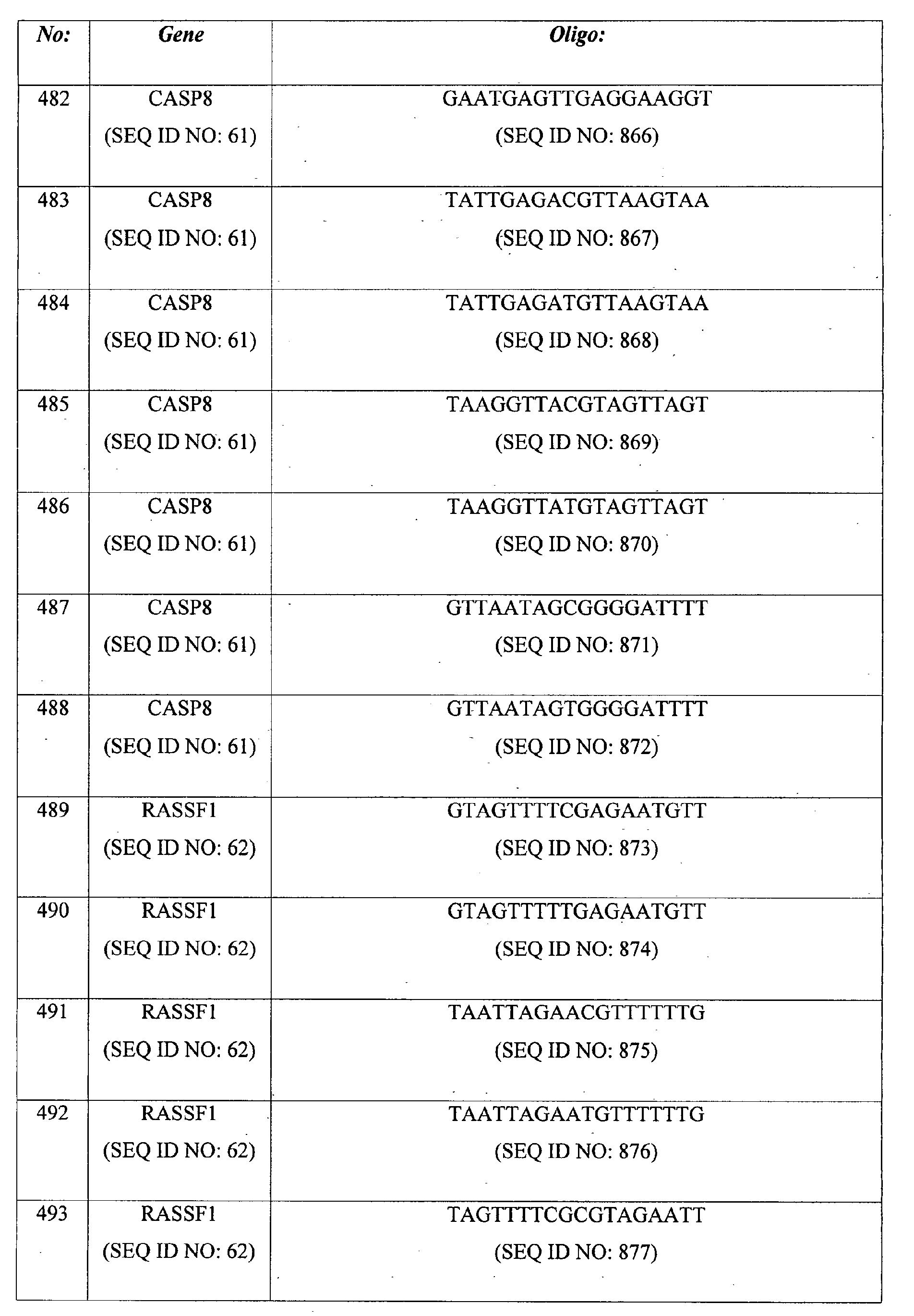

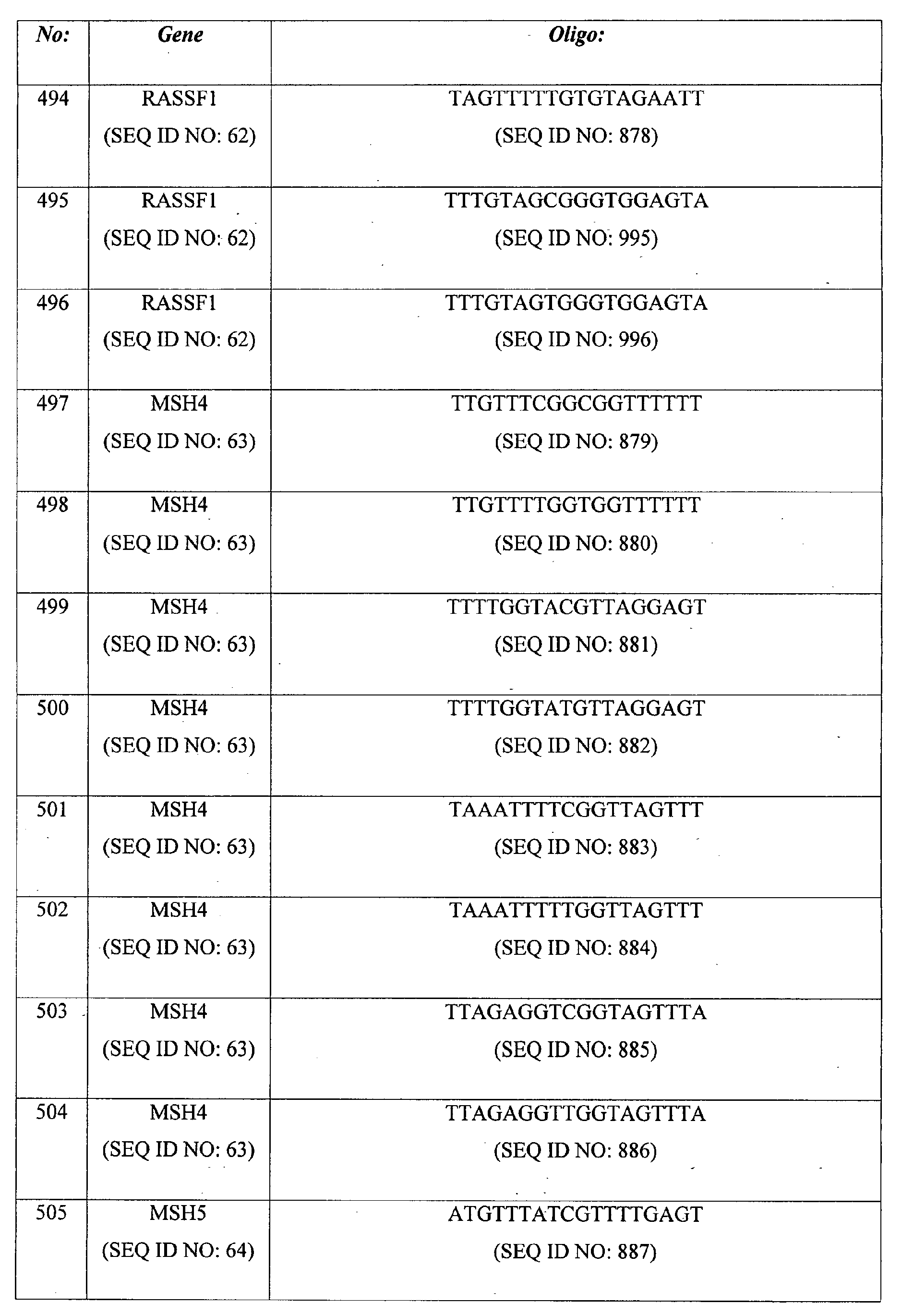

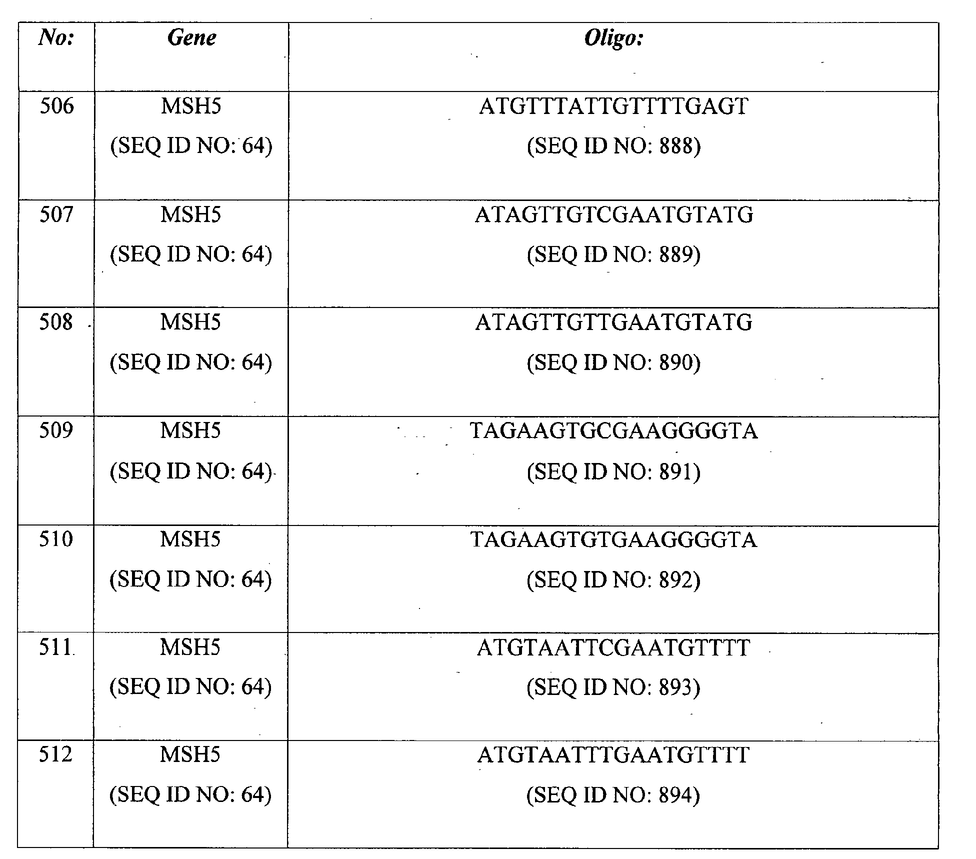

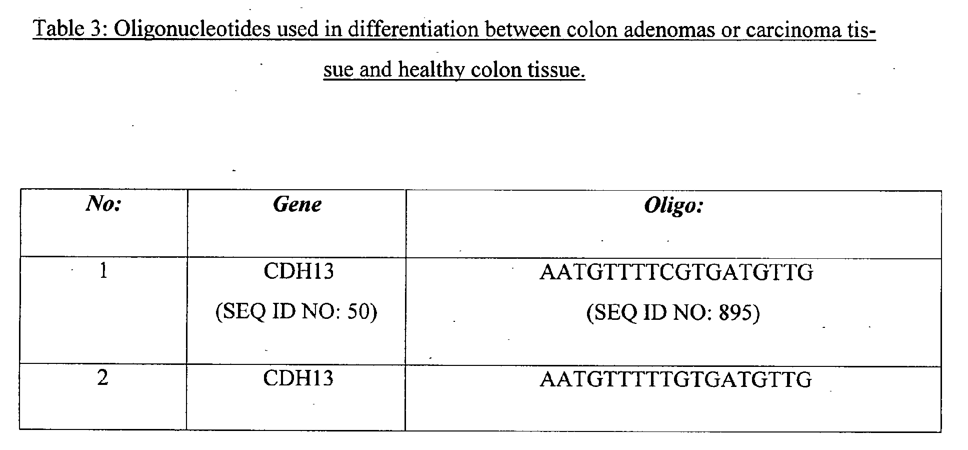

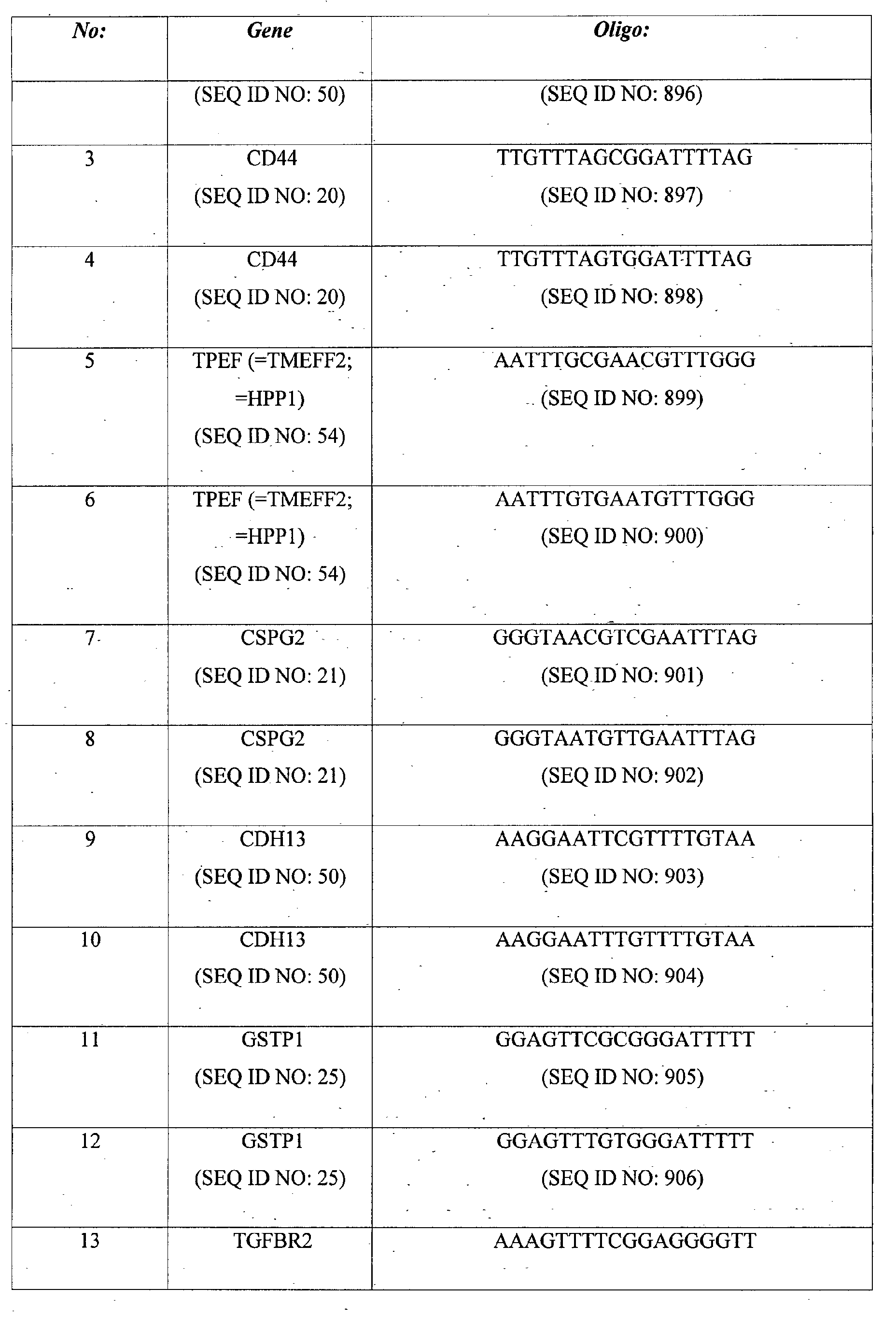

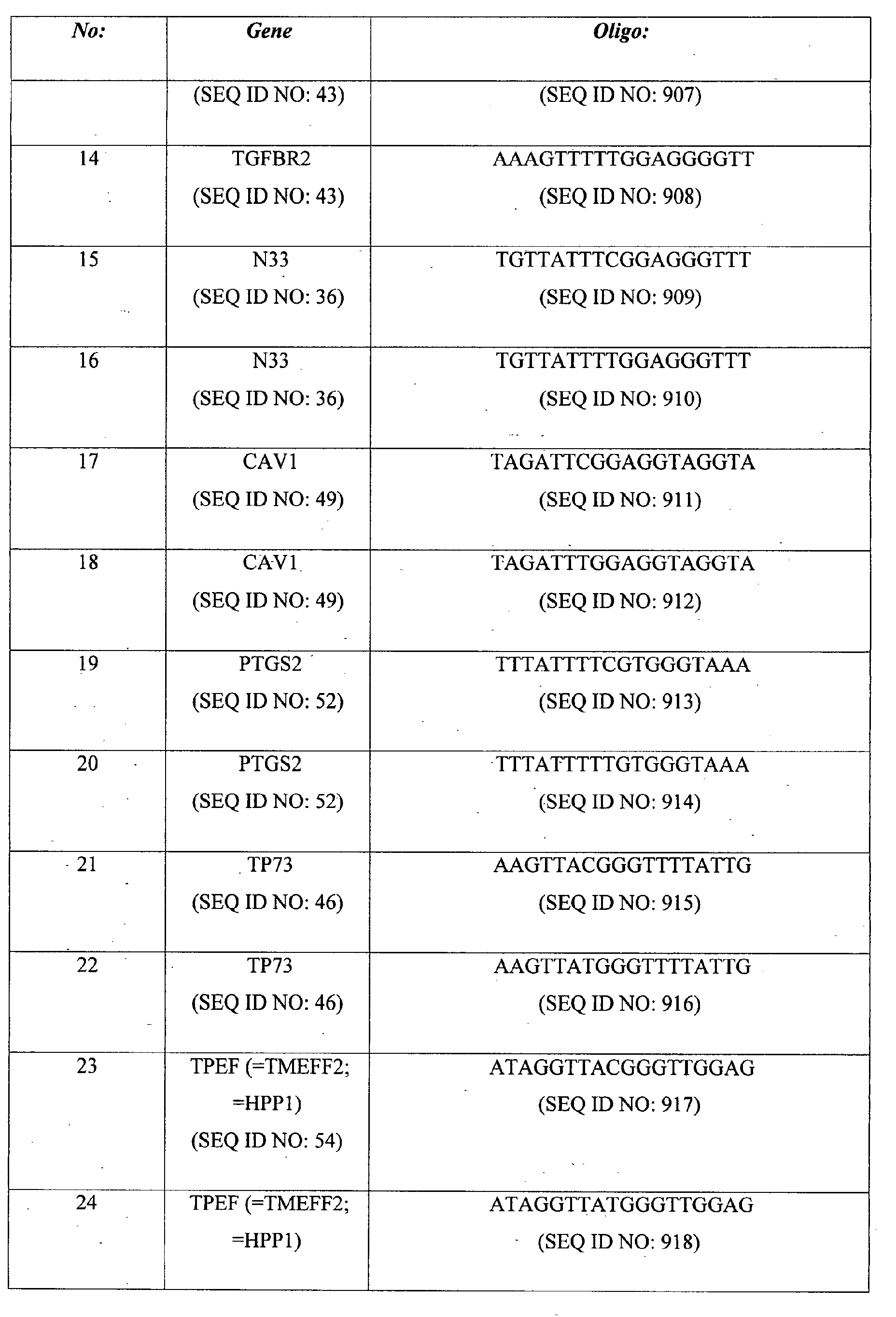

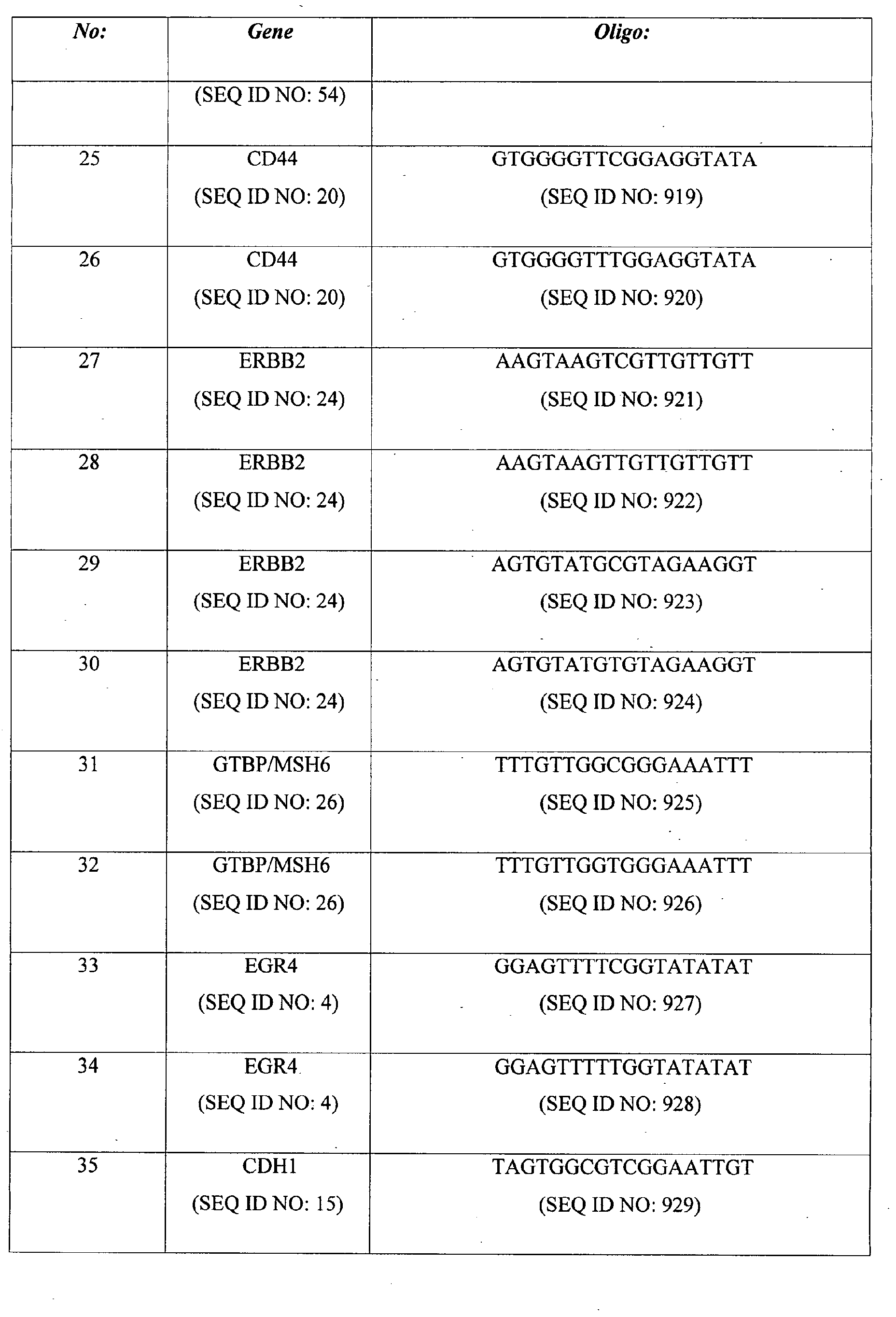

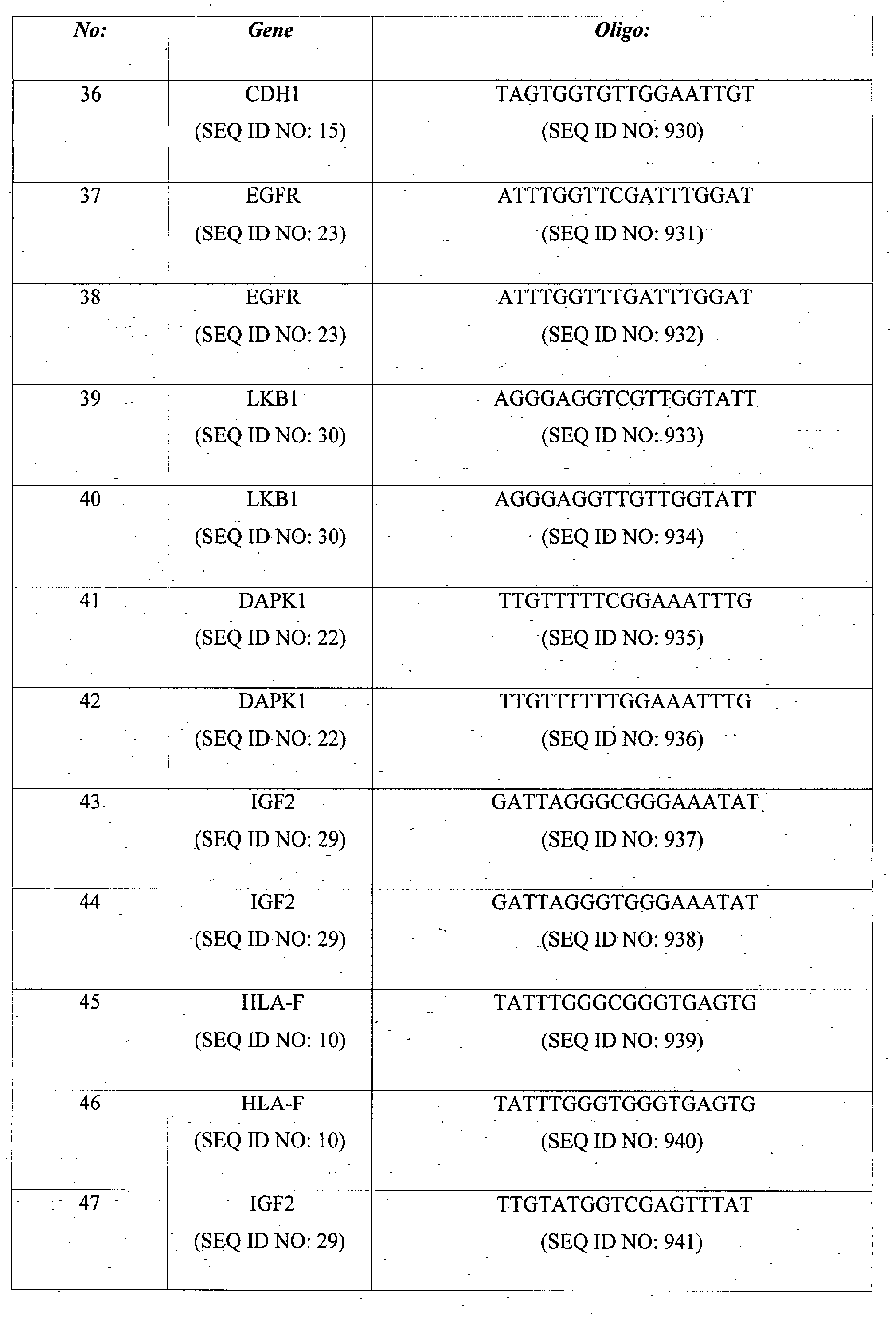

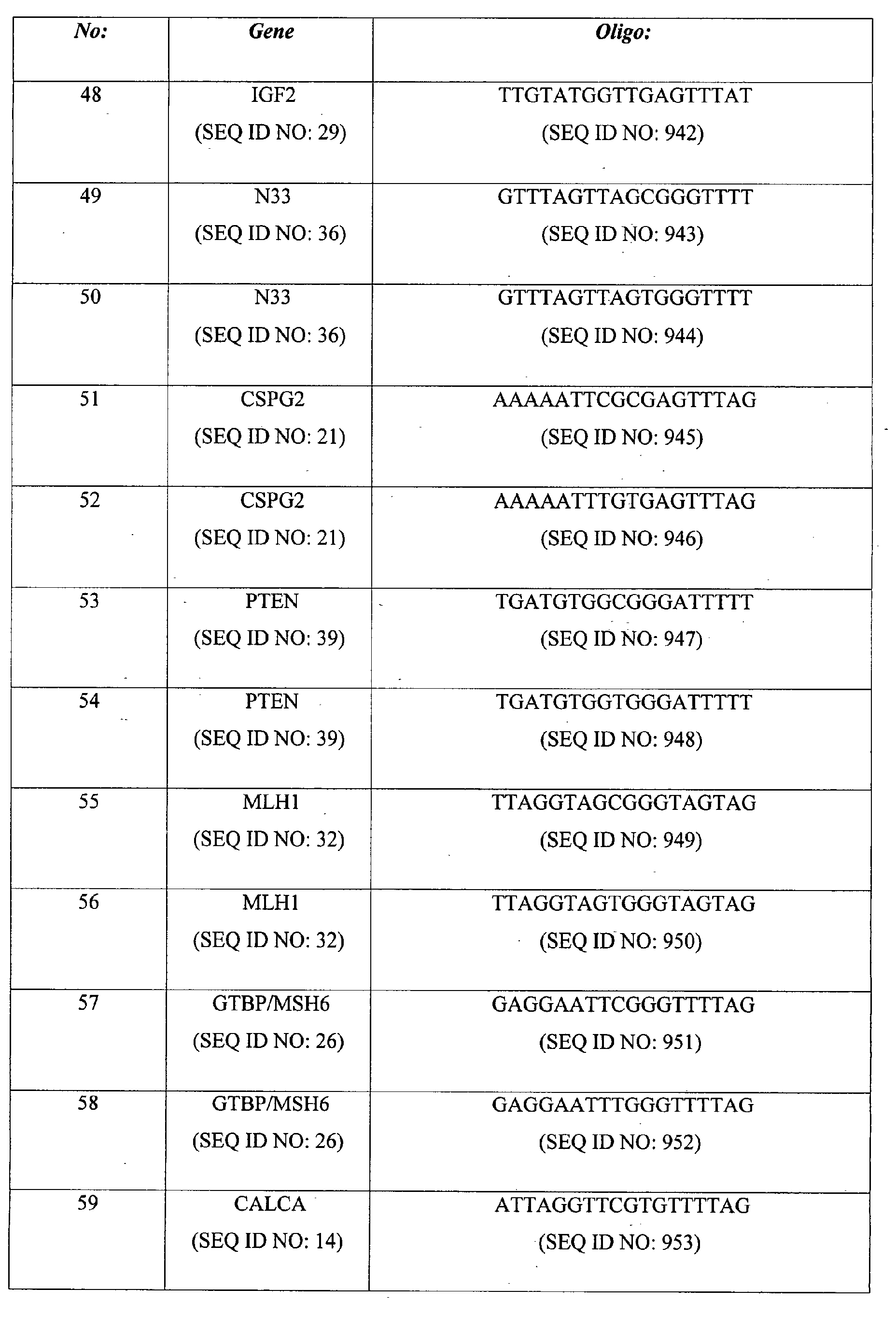

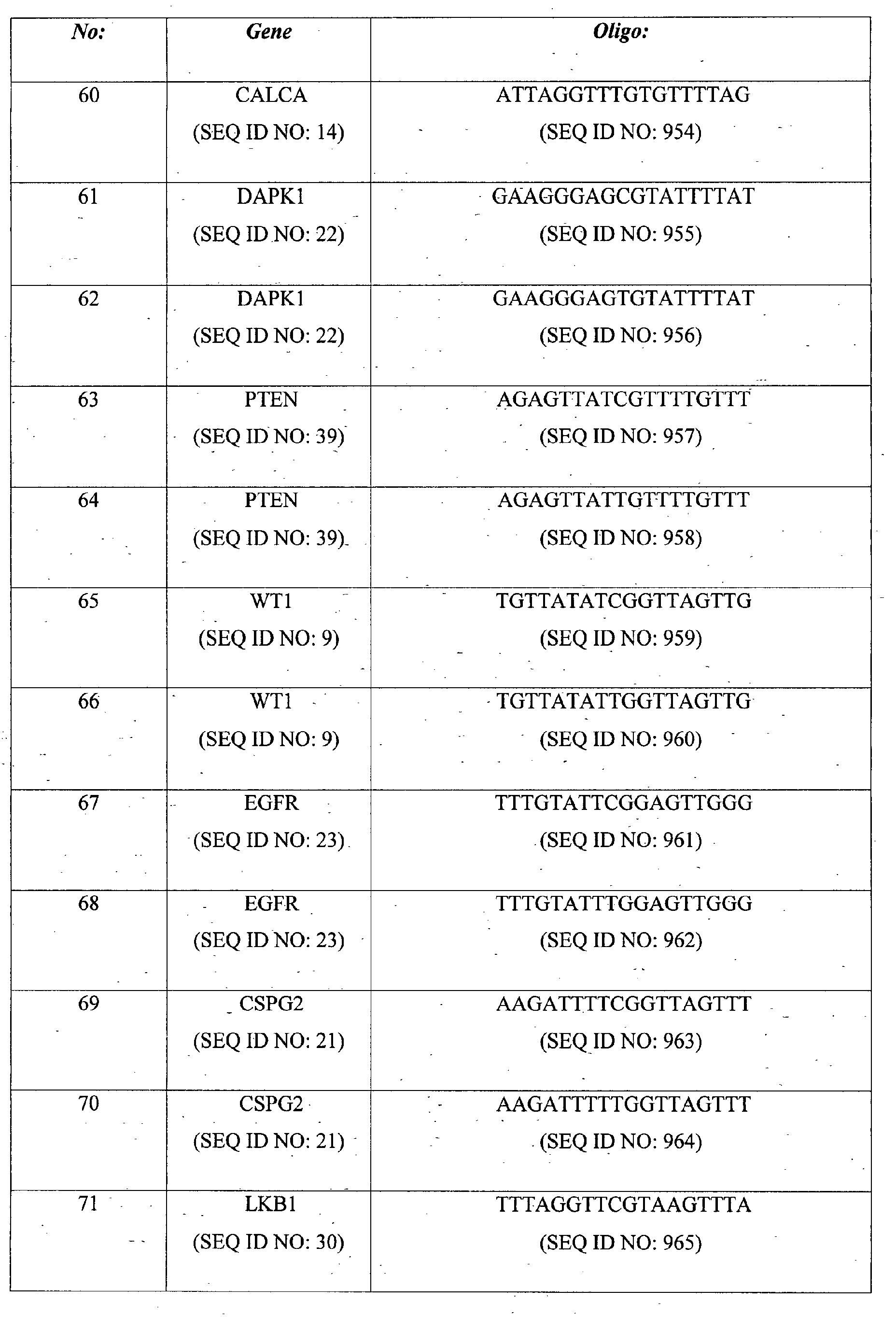

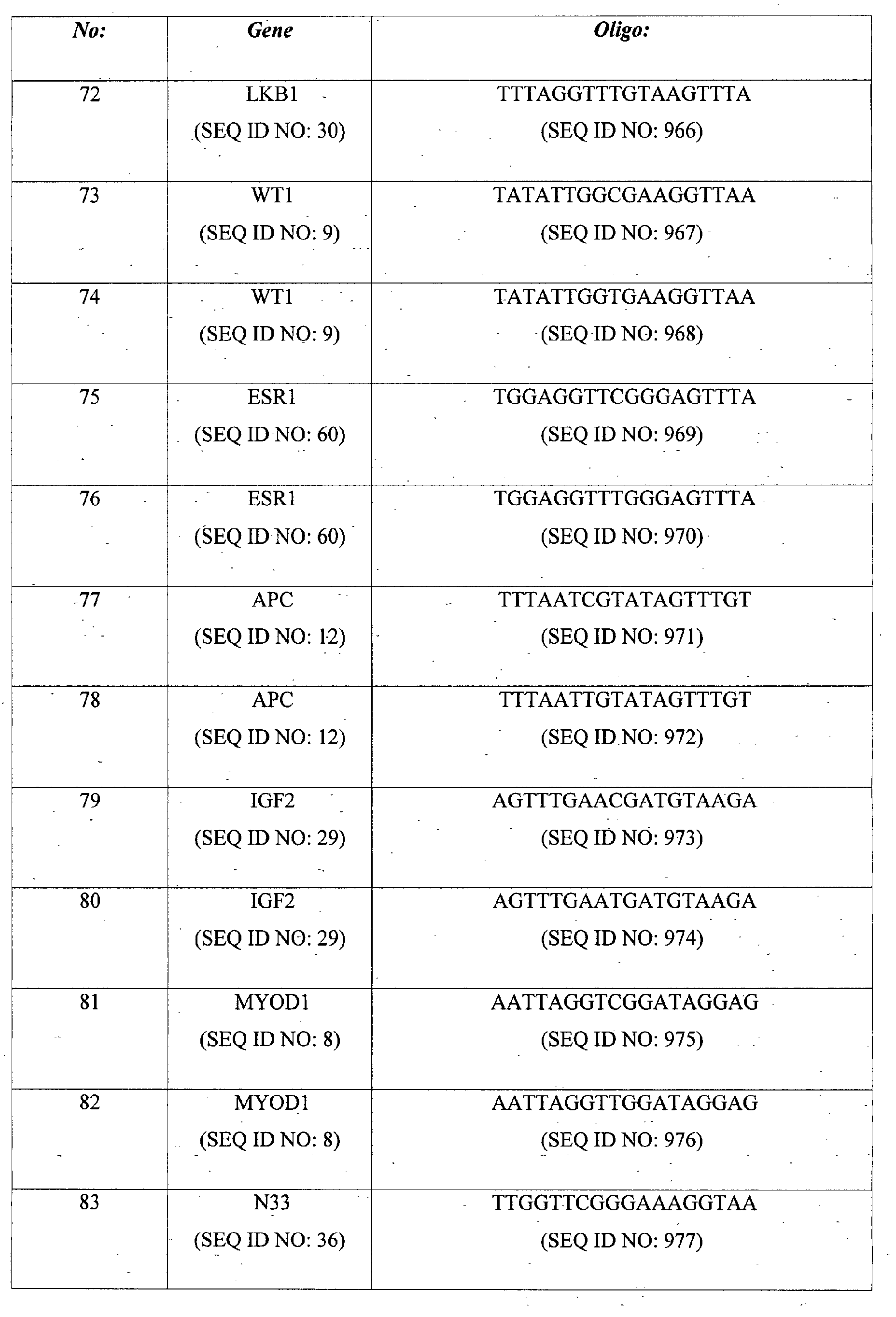

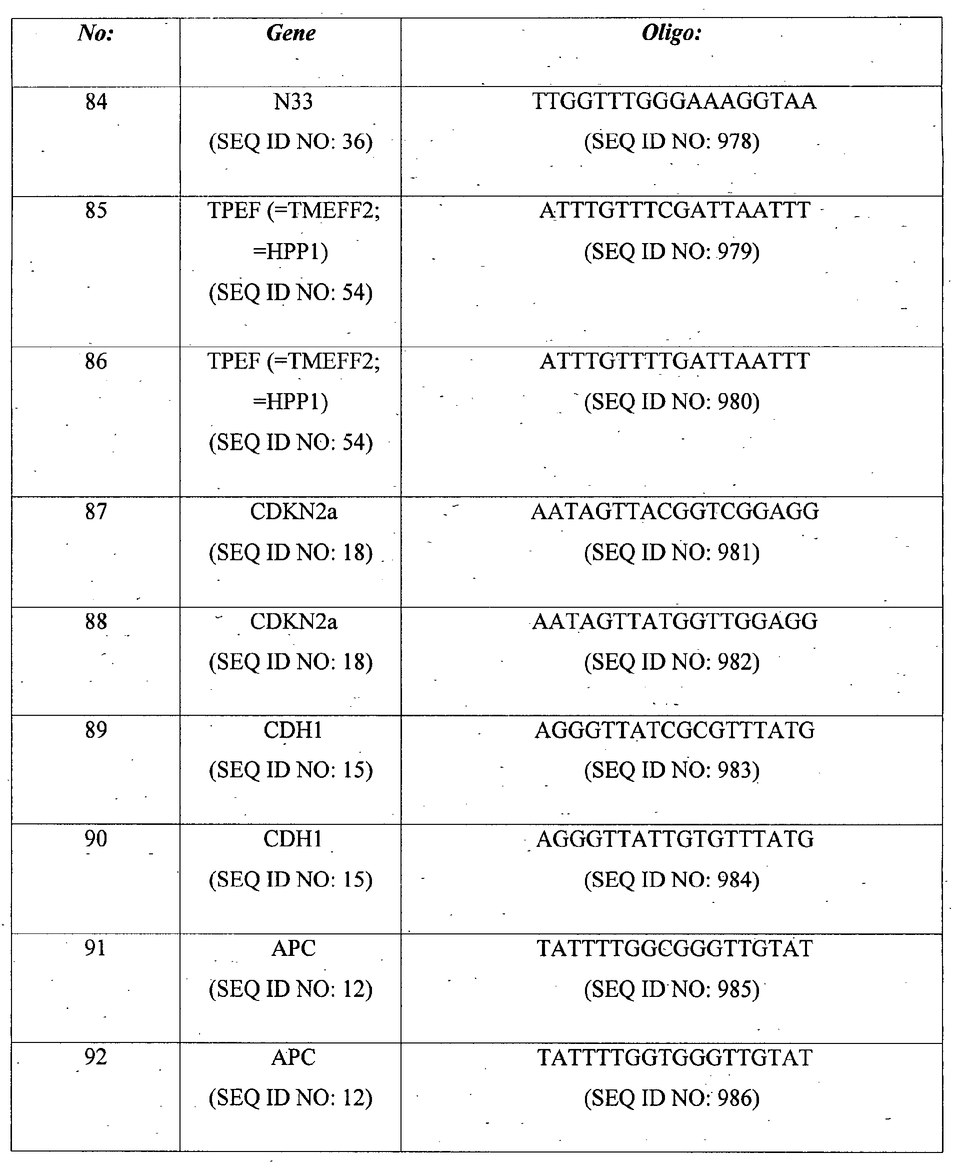

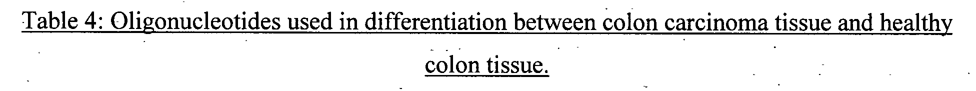

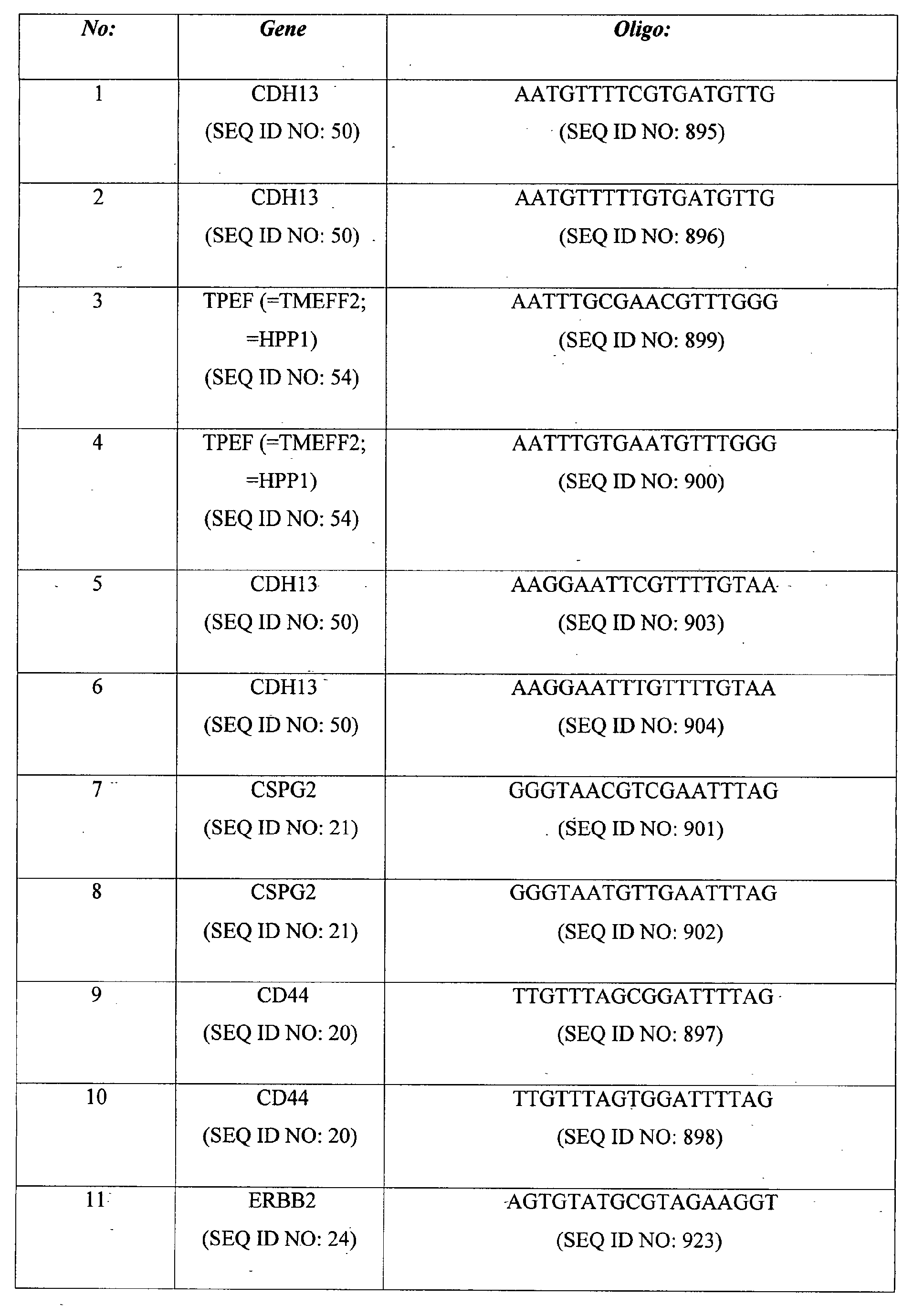

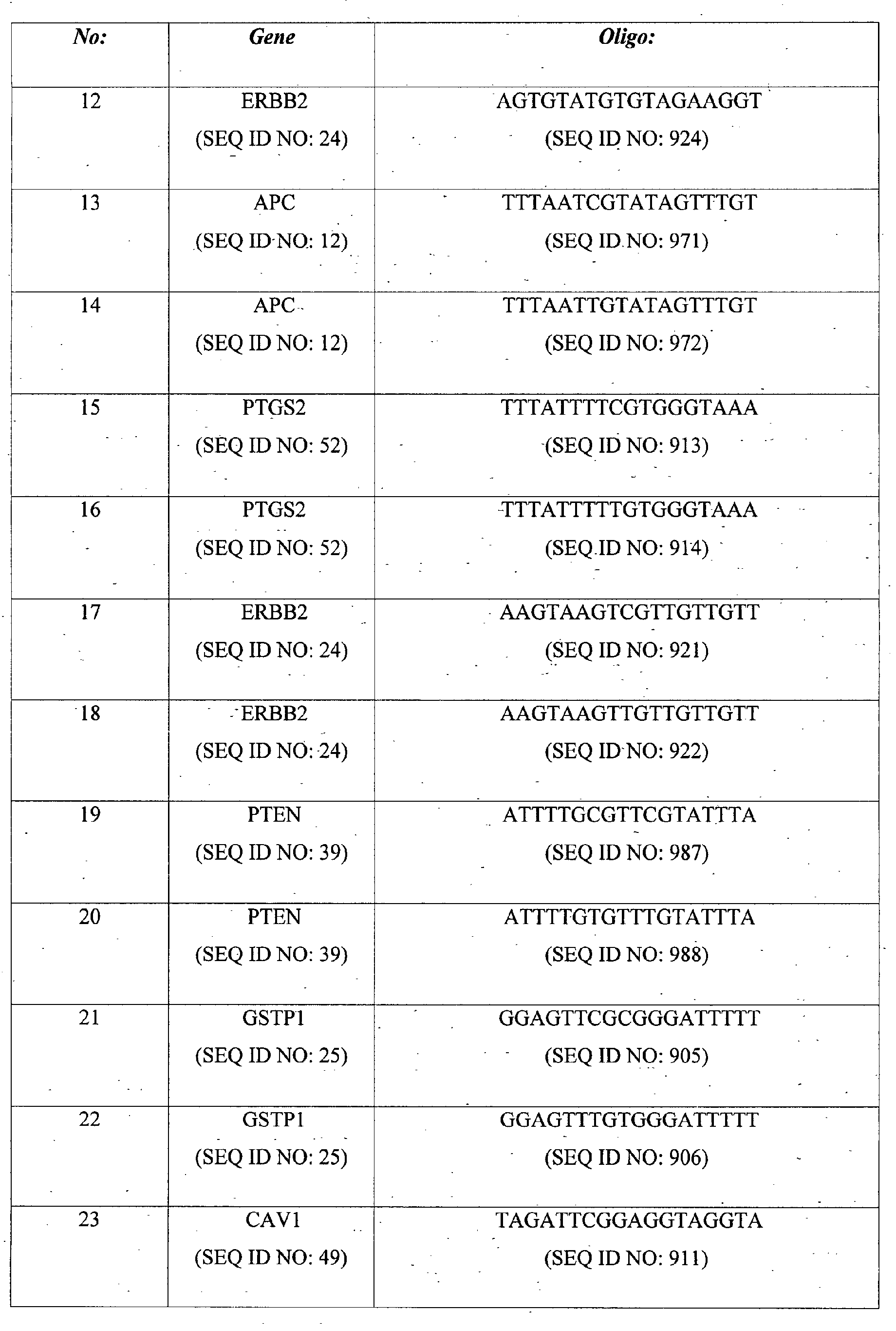

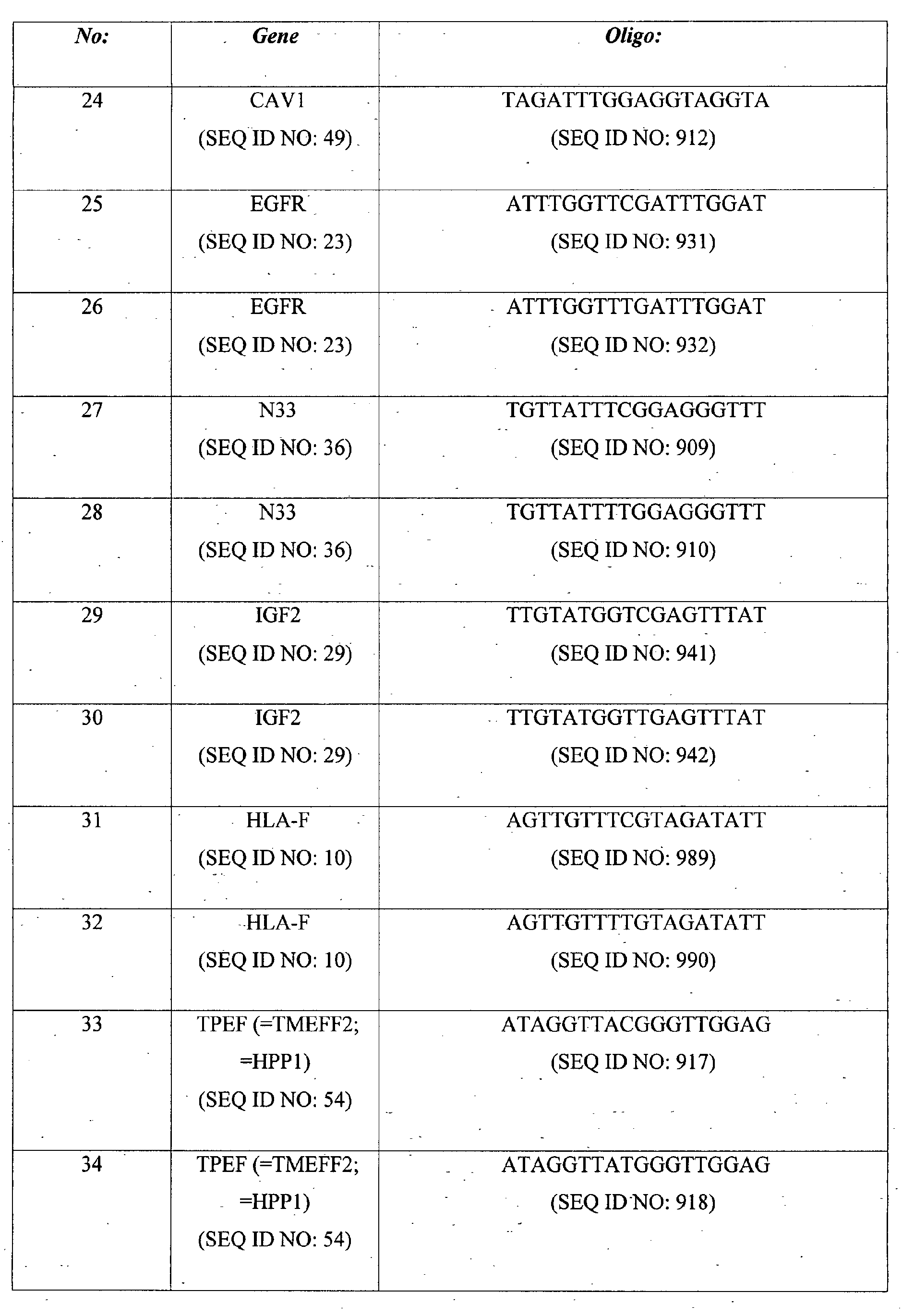

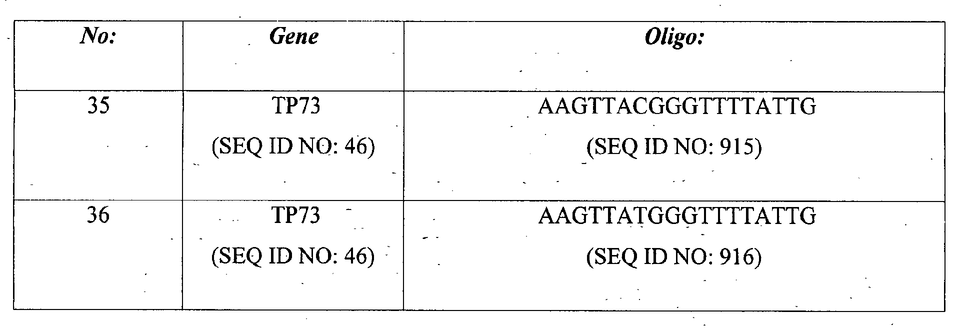

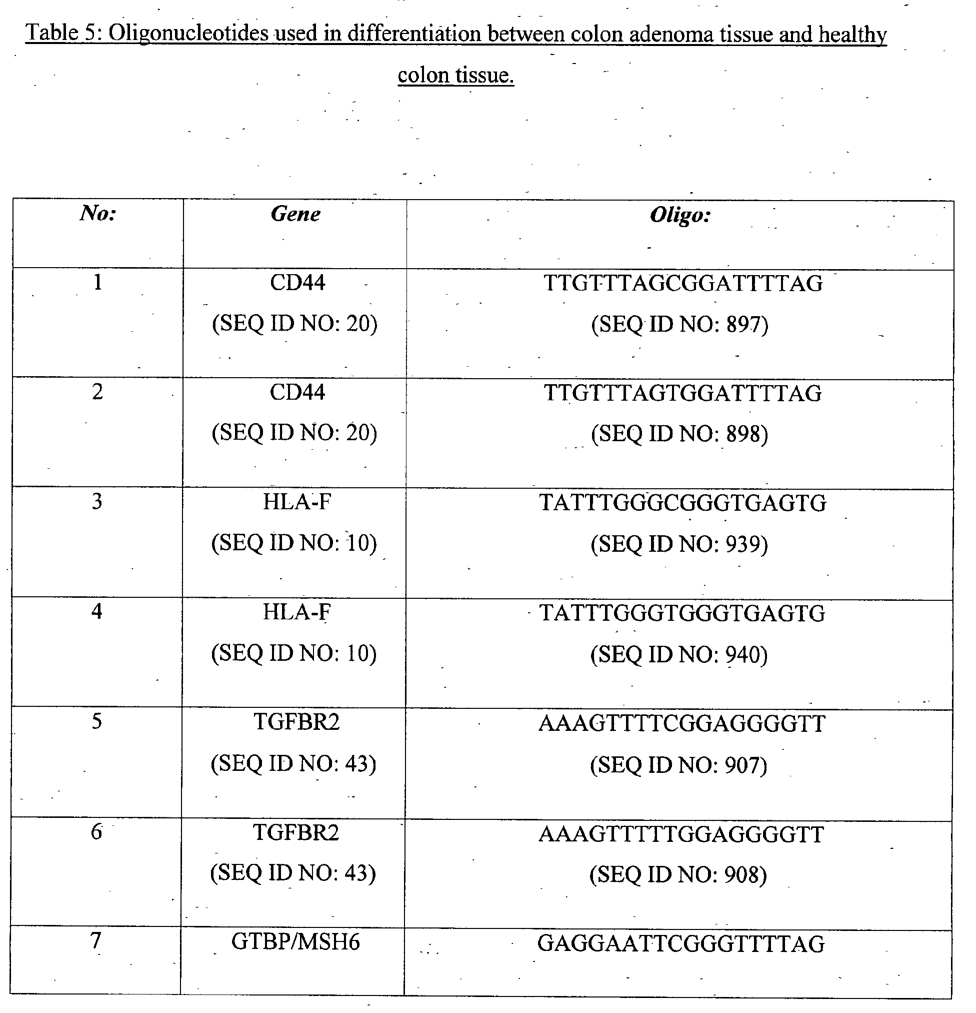

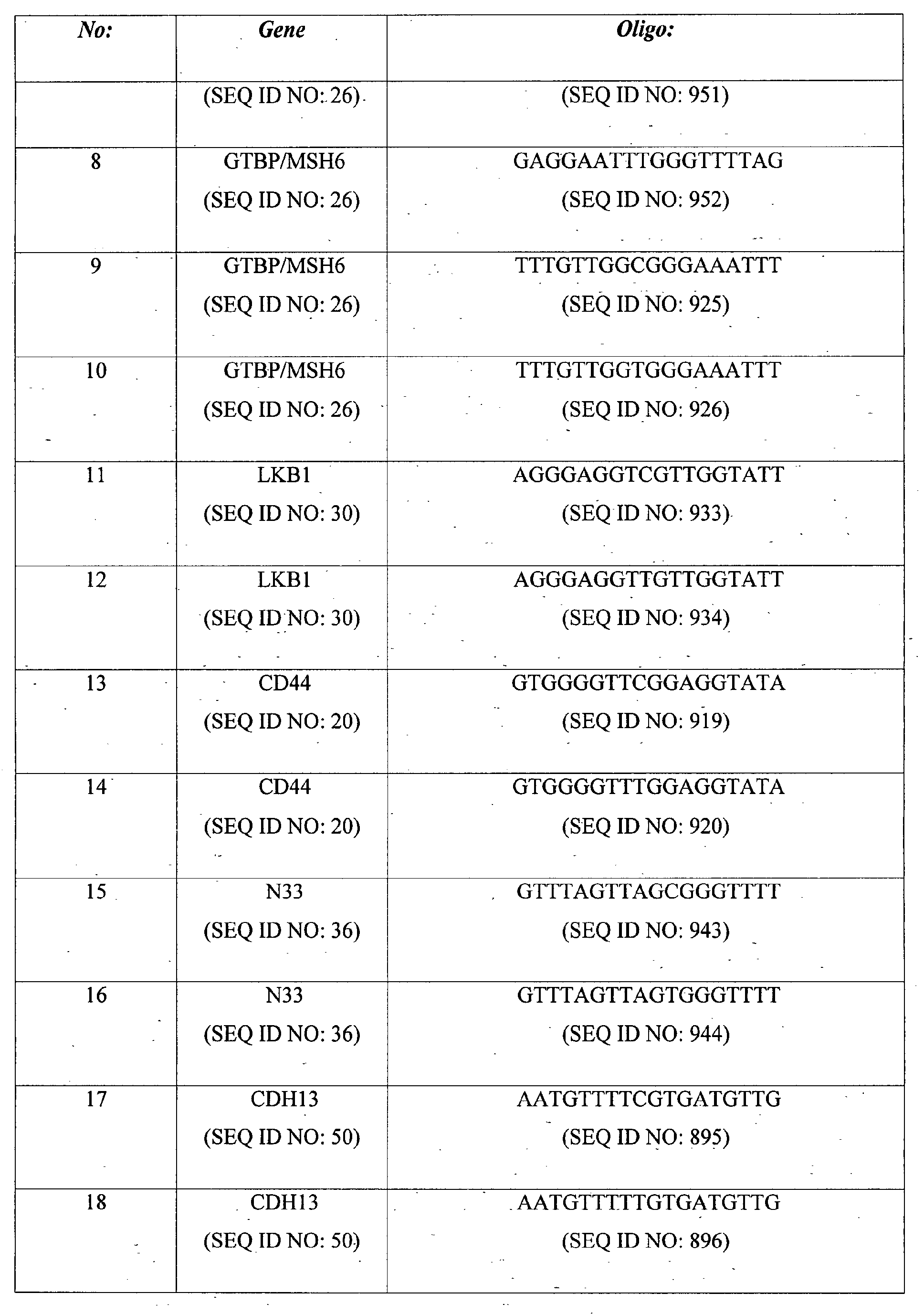

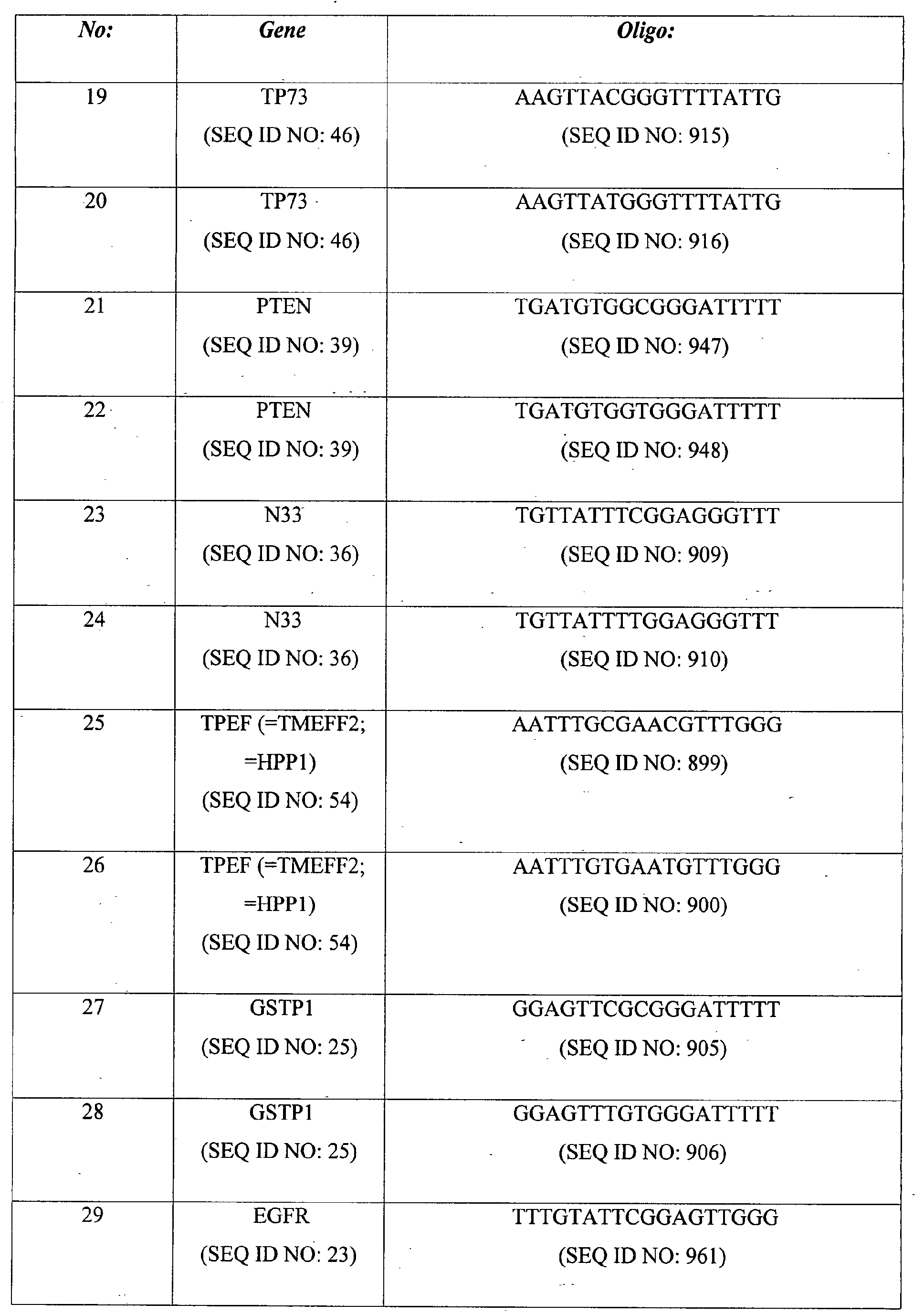

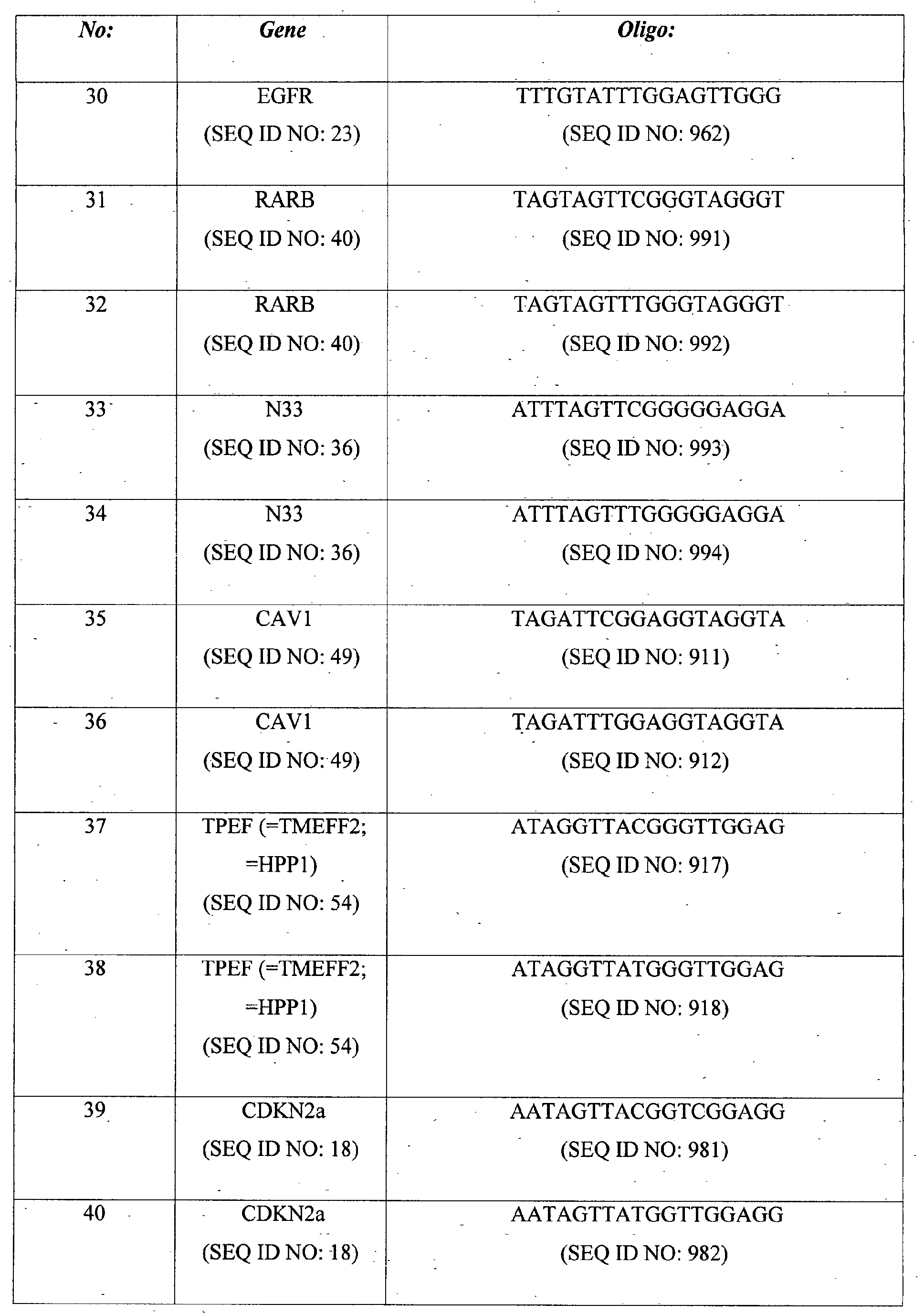

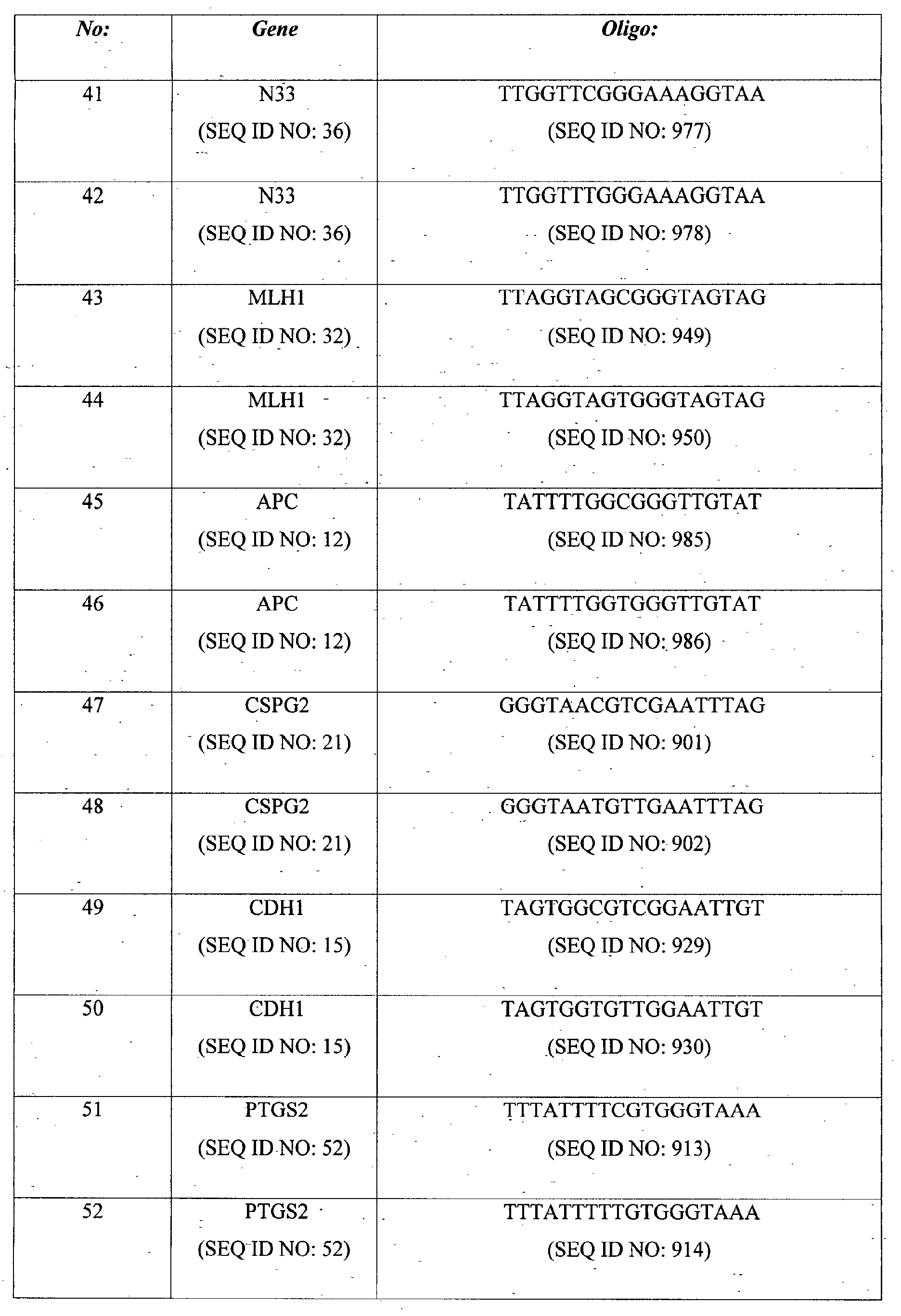

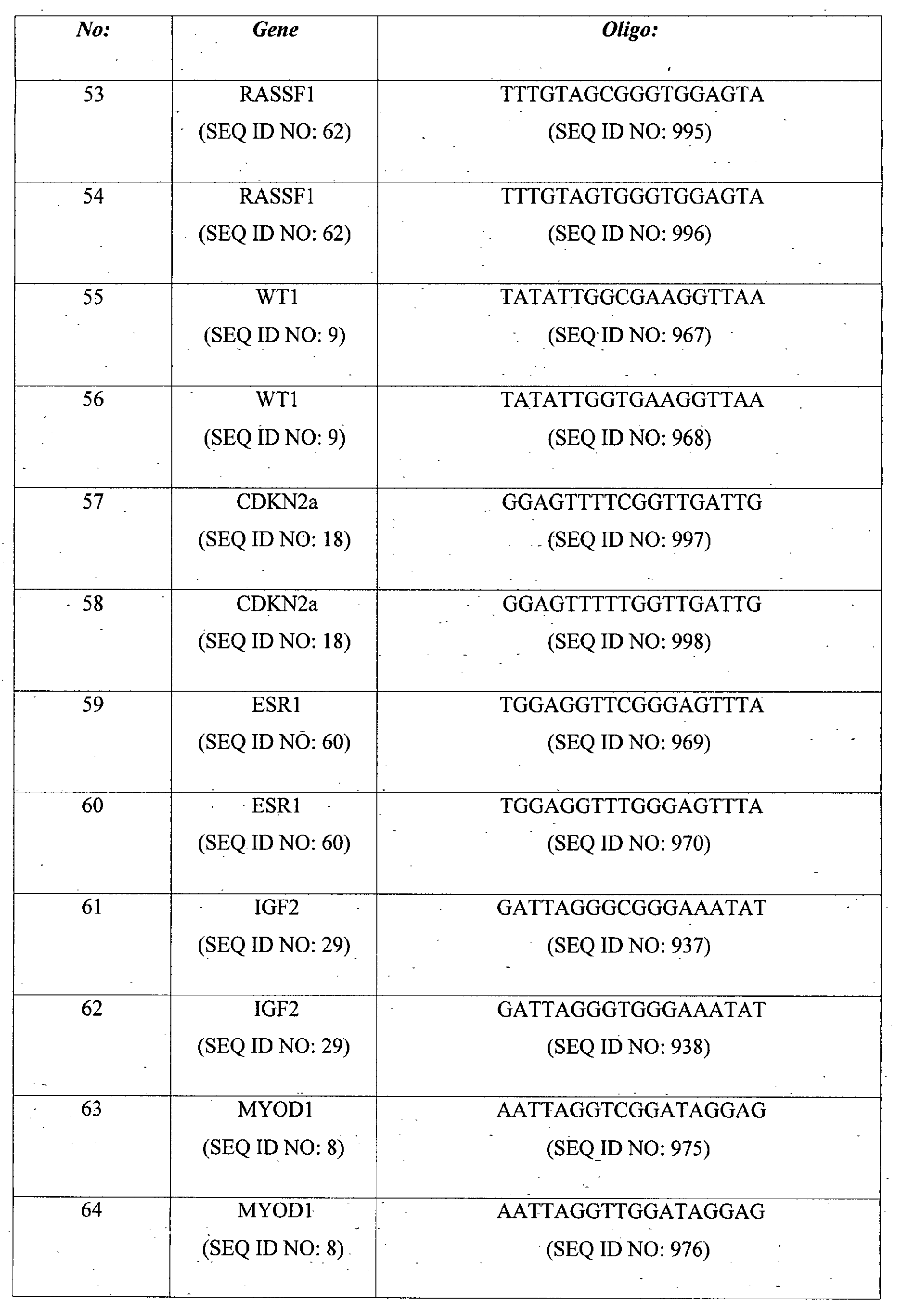

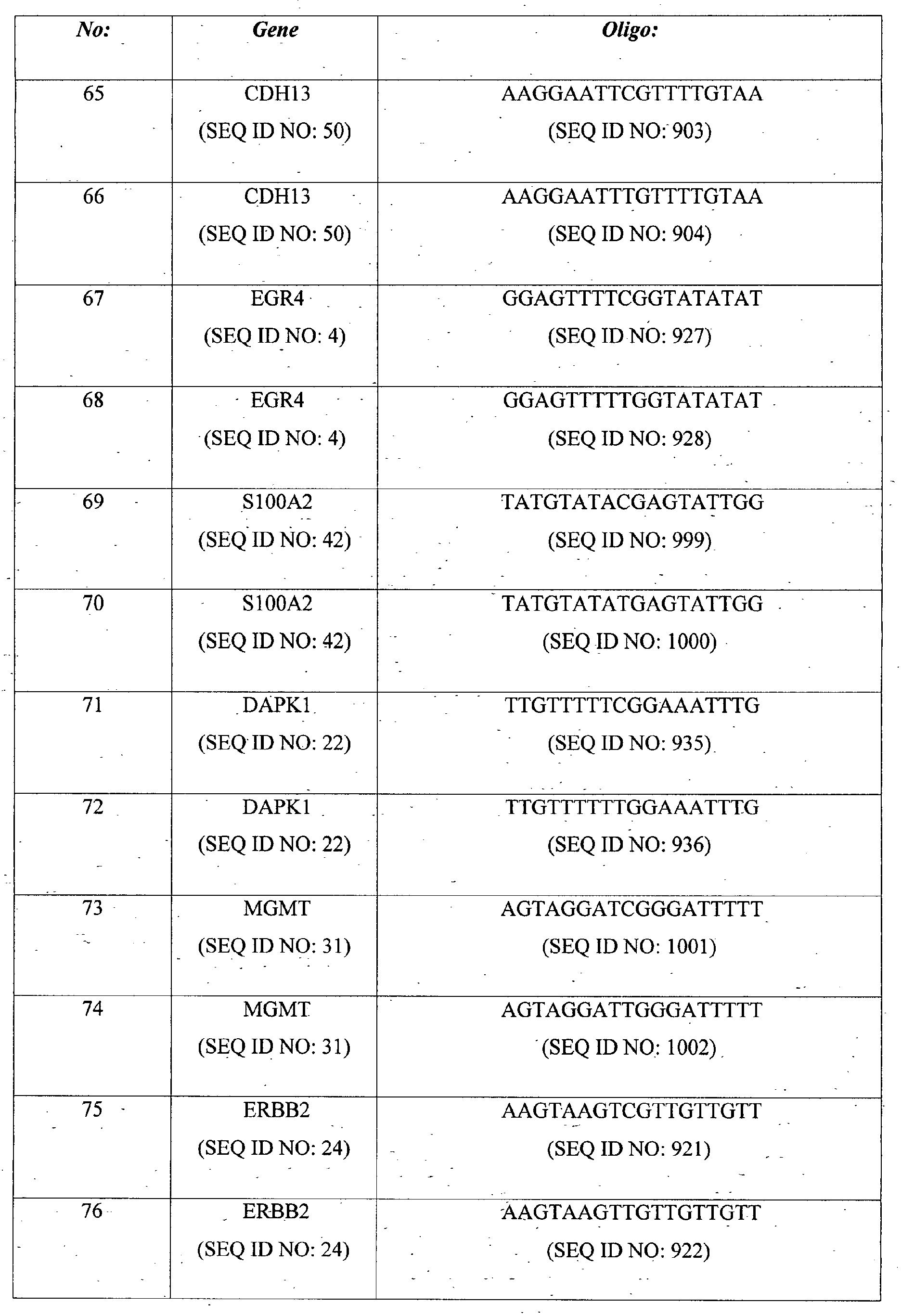

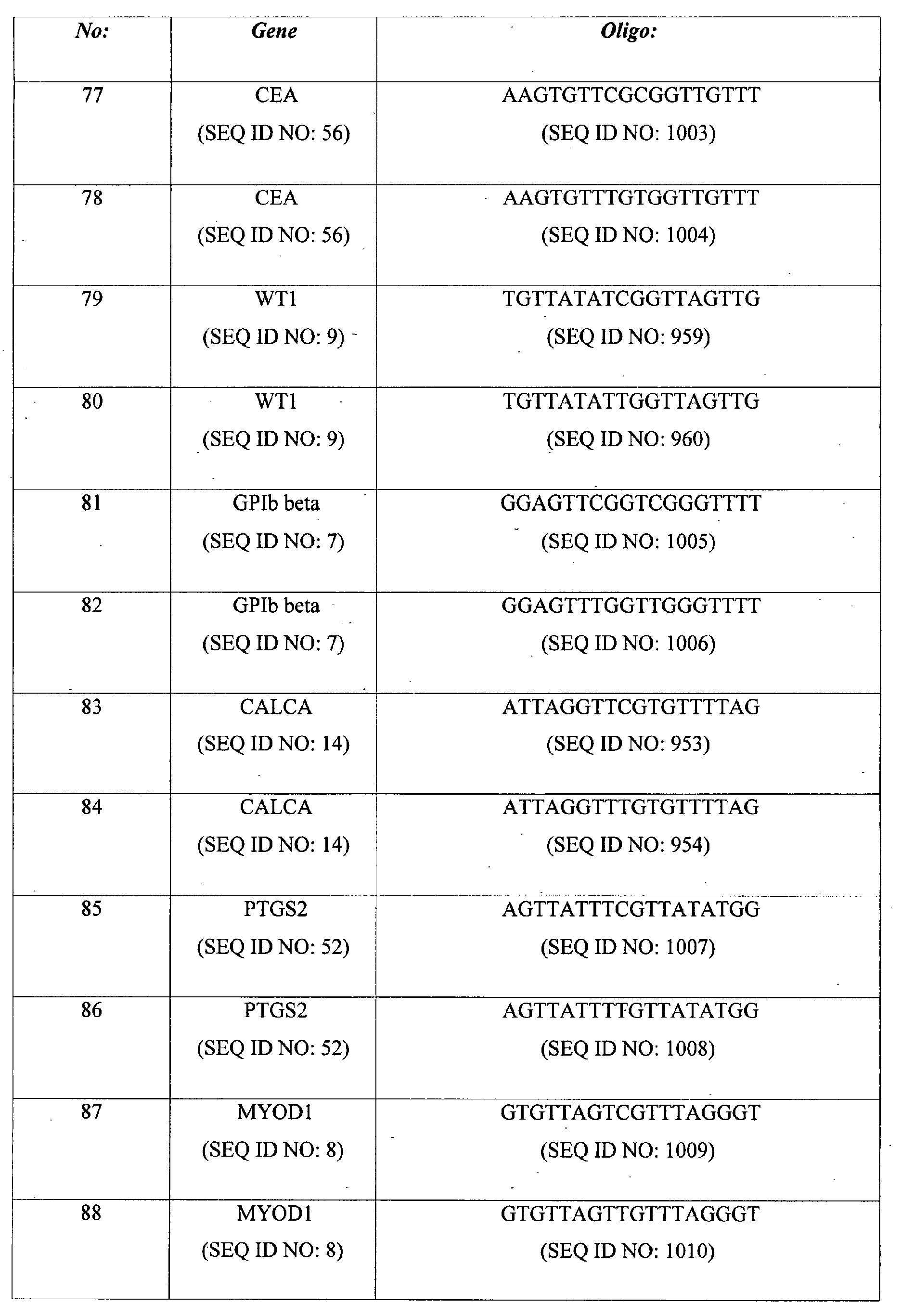

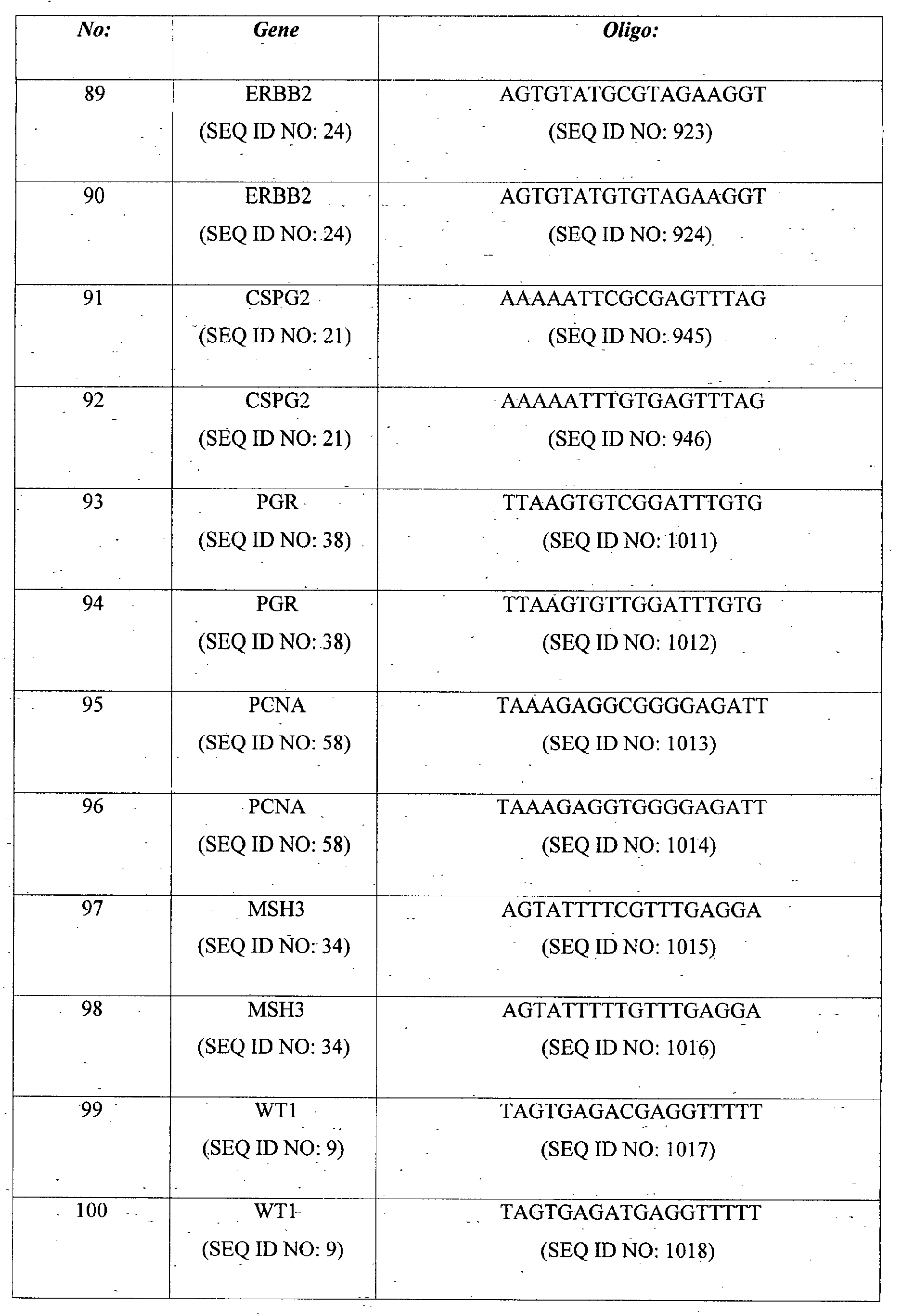

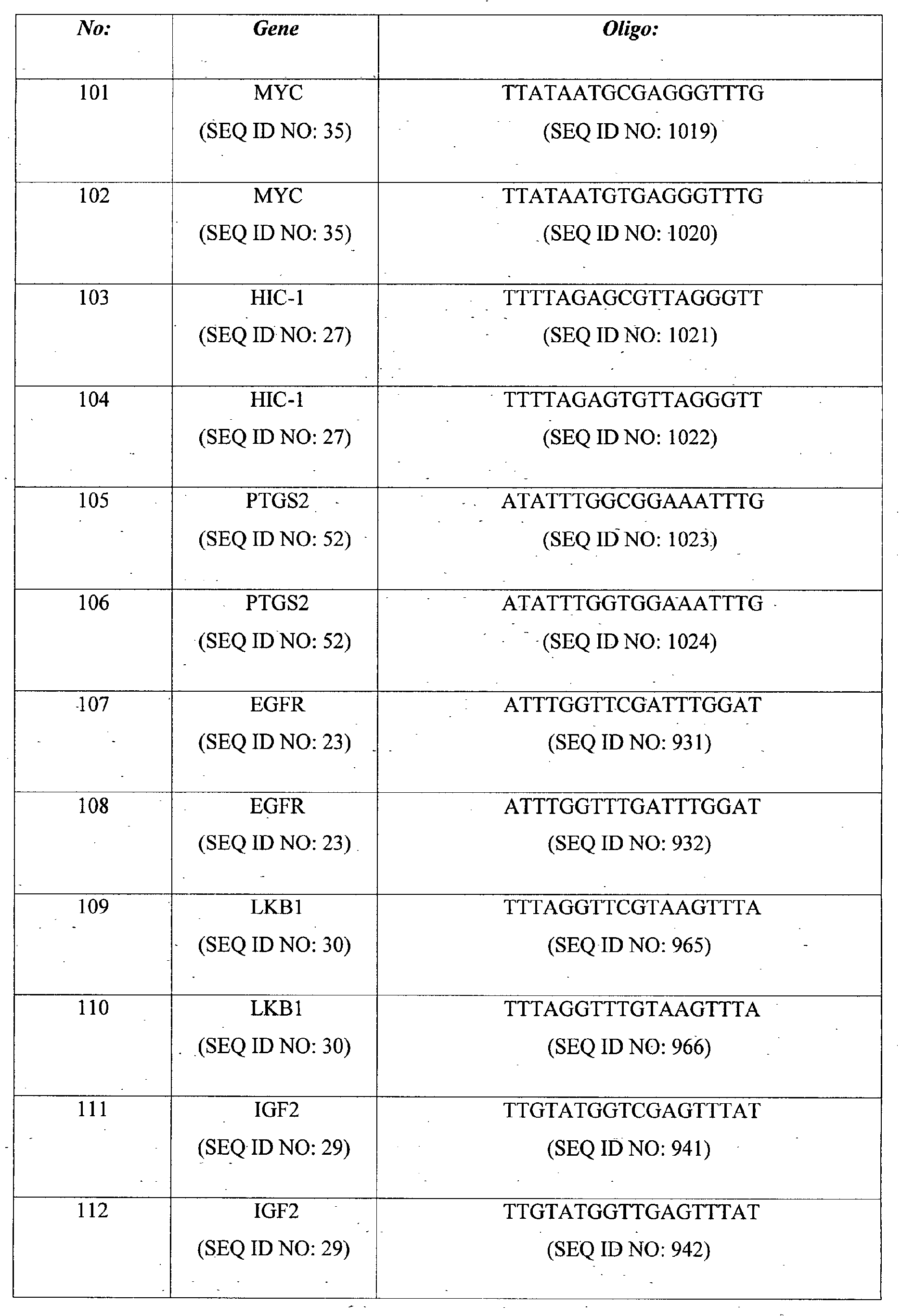

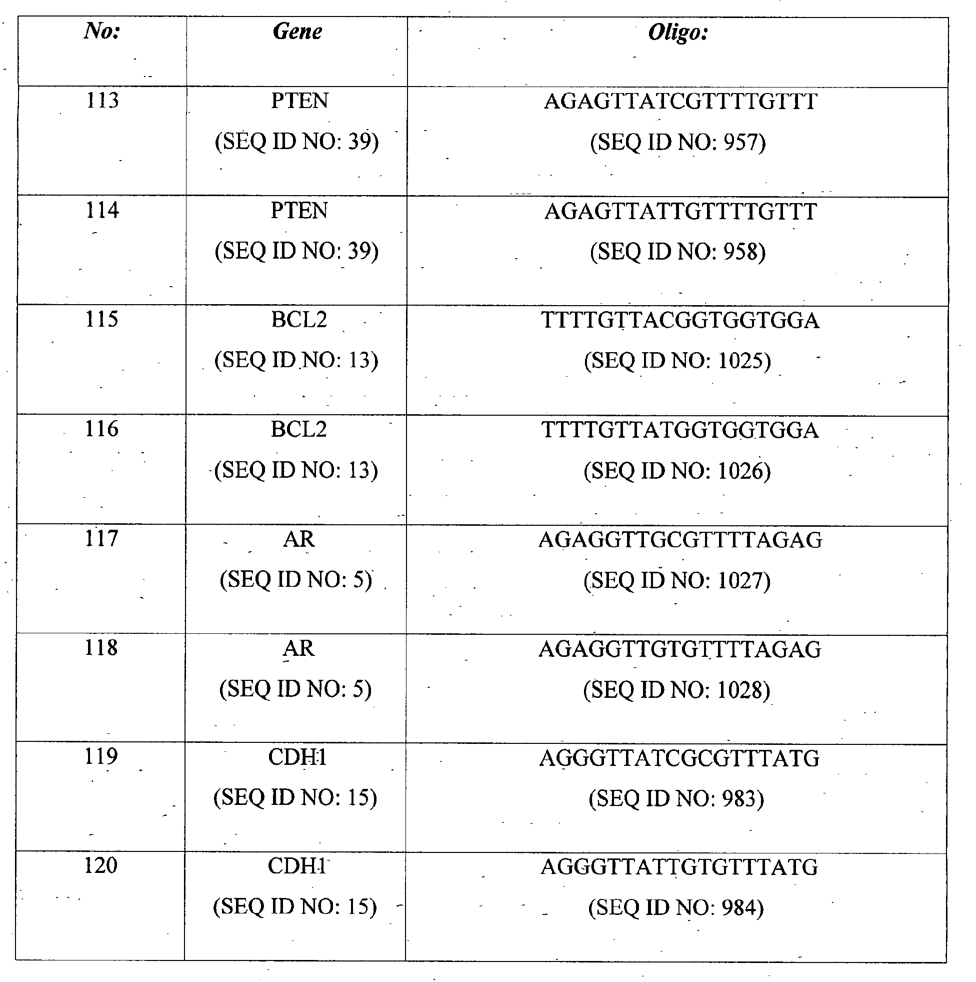

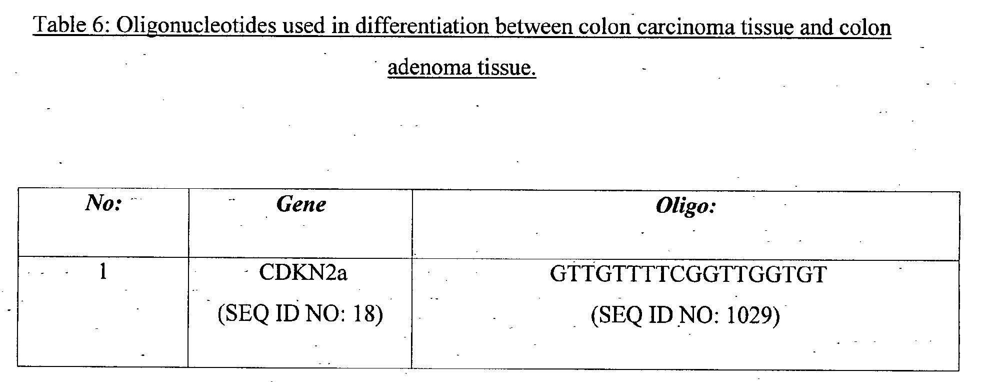

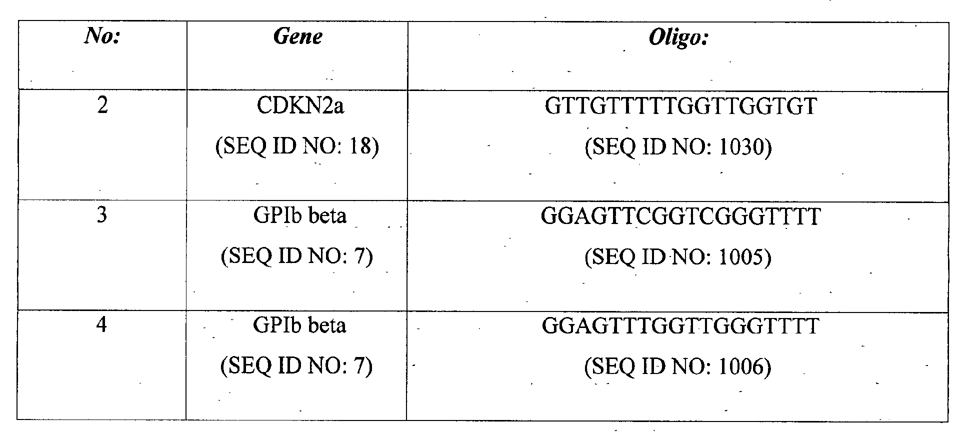

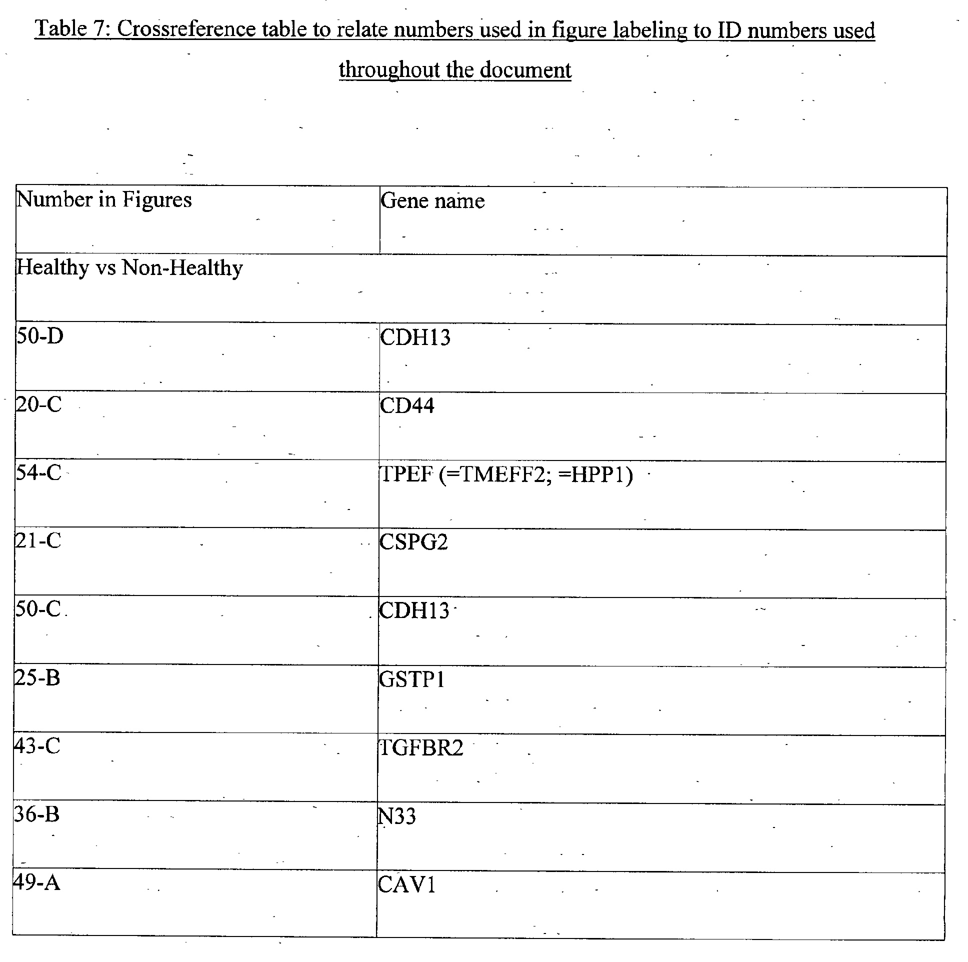

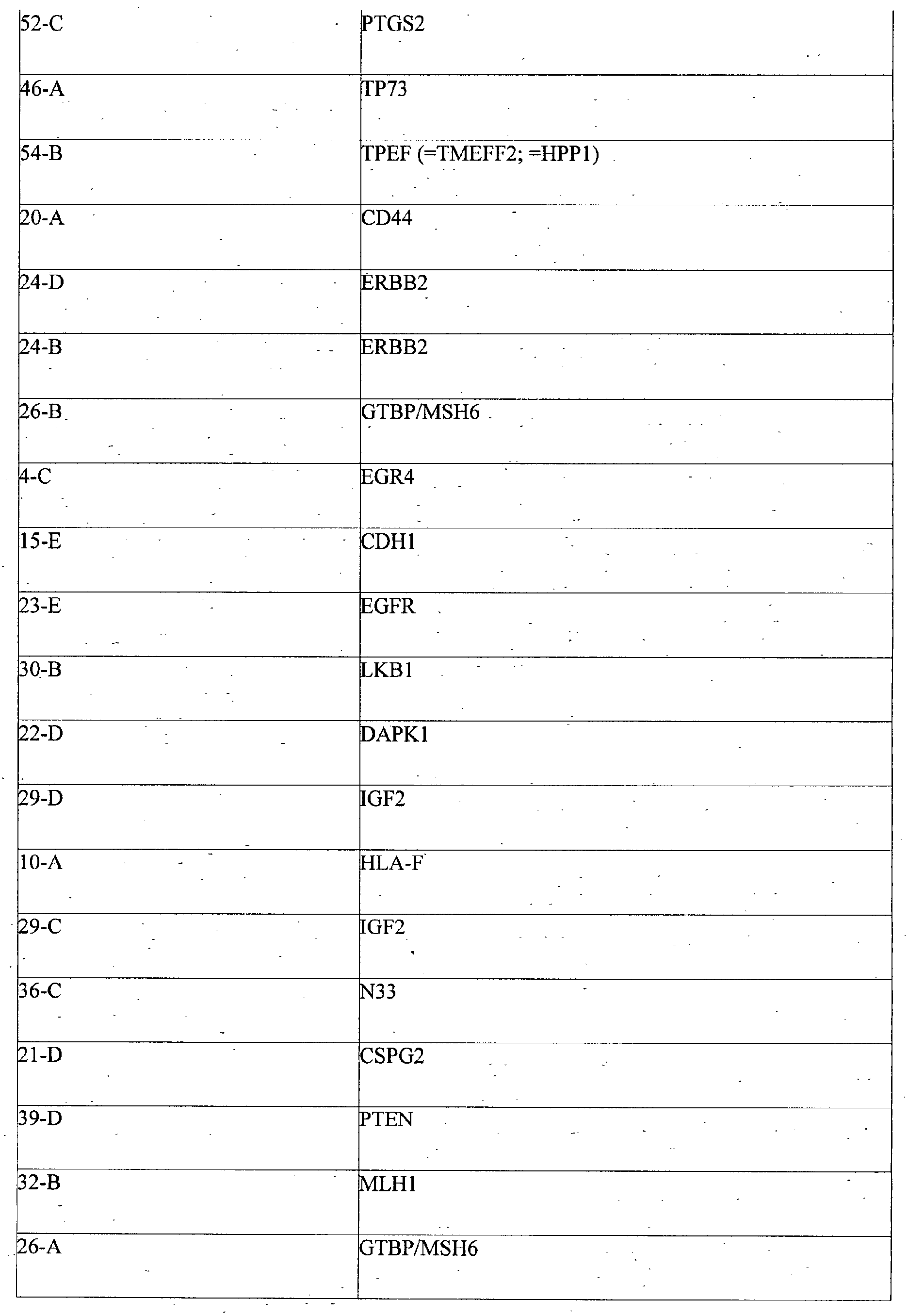

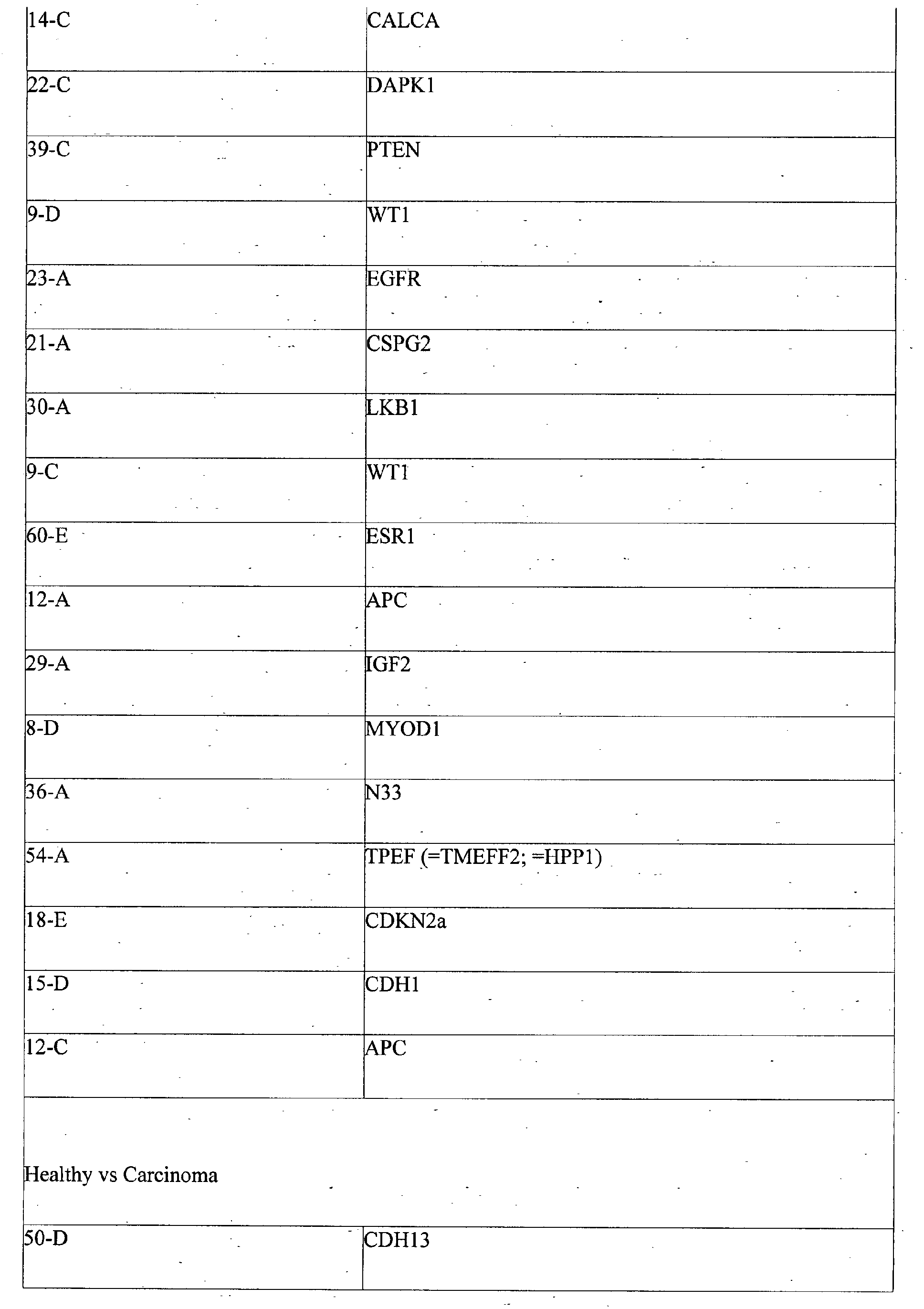

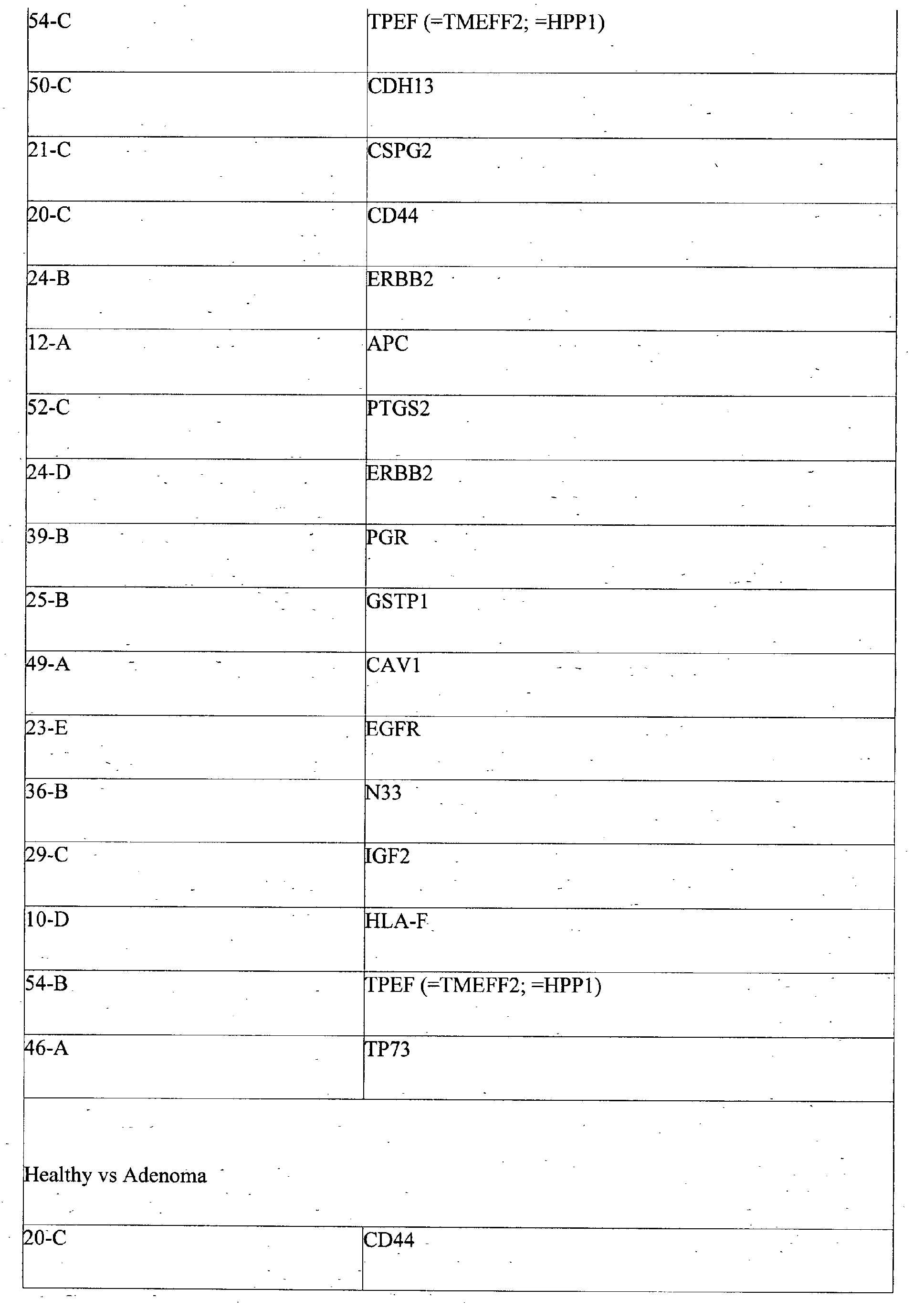

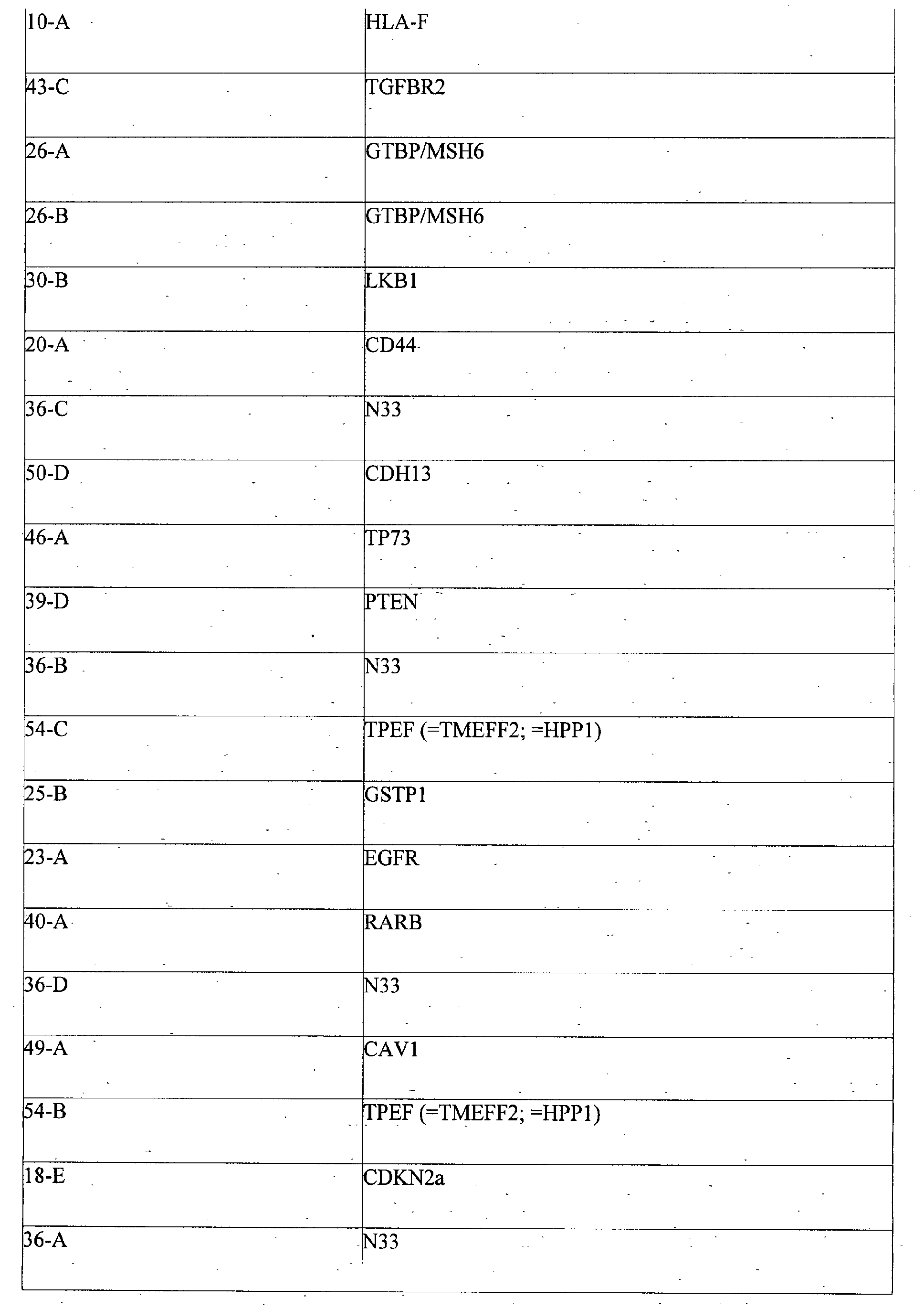

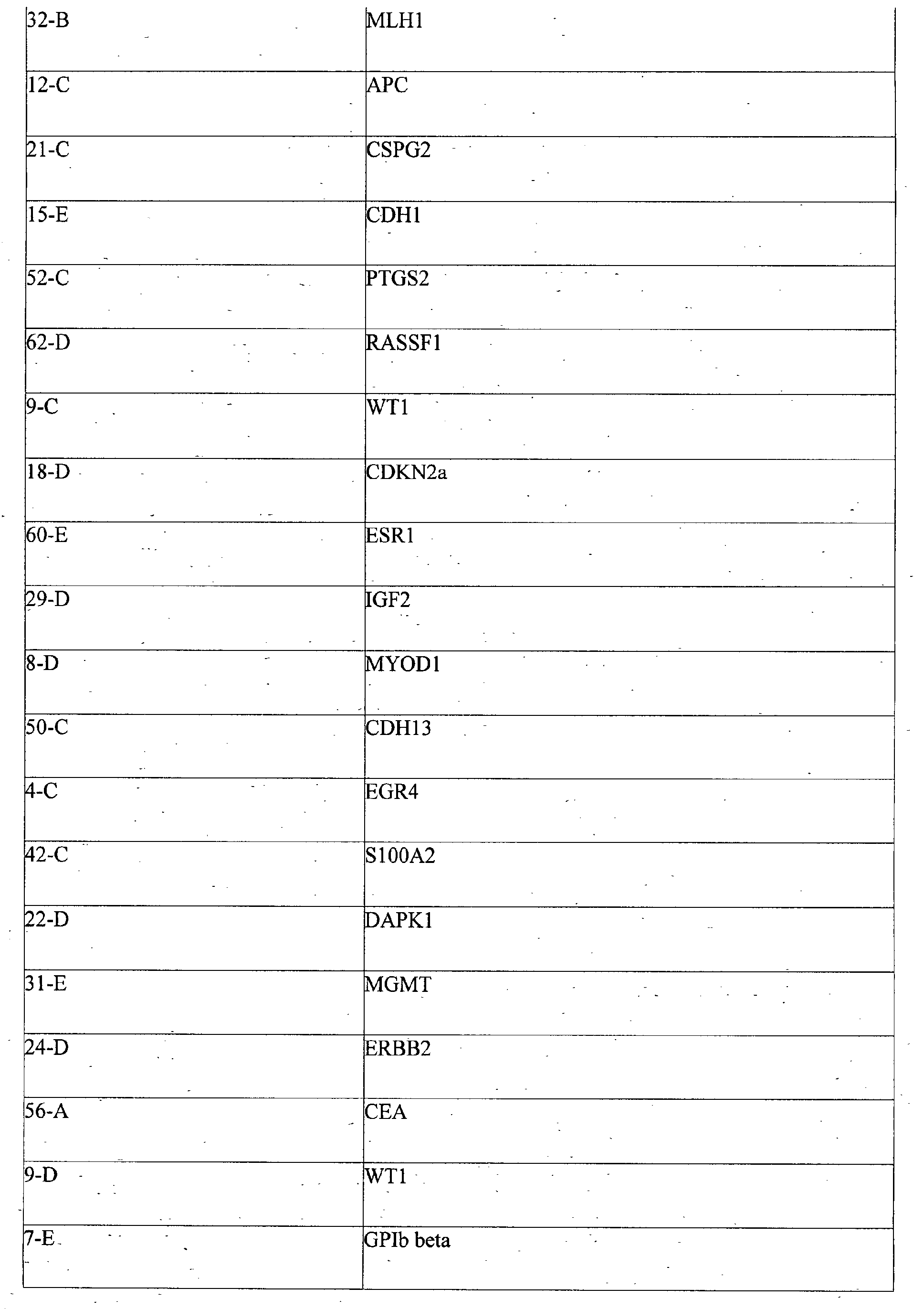

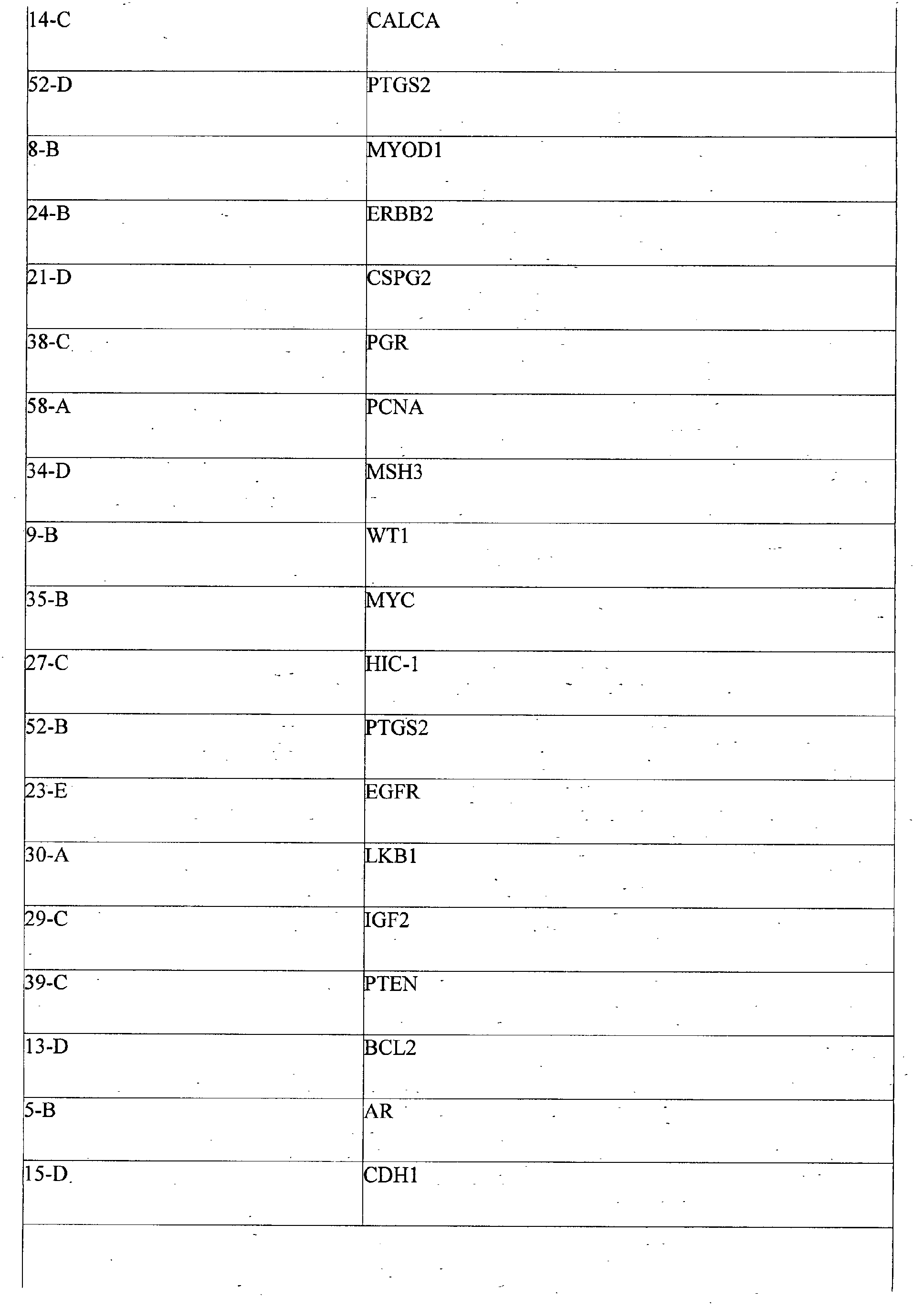

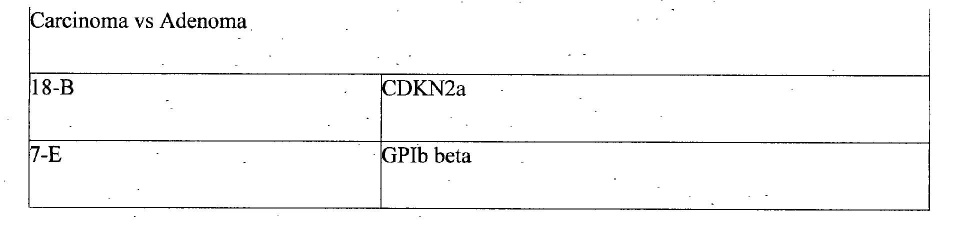

### Description of figures

### Figure 1

Differentiation between healthy colon tissue and adenoma or carcinoma colon tissue according to Example 2.

The labels on the left side of the plot are gene and CpG identifiers, these can be cross referenced using Table 3 and Table 7. The labels on the right side of the figure give the significance (p-value, T-test) of the difference between the means of the two groups. Each row corresponds to a single CpG and each column to the methylation levels of one sample. CpGs are ordered according to their contribution to the differentiation between the two tissue types (A = healthy, B = non healthy) with increasing contribution from top to bottom. Black indicates total methylation at a given CpG position, white represents no methylation at the particular position, with degrees of methylation represented in gray, from light (low proportion of methylation) to dark (high proportion of methylation).

### Figure 2

Differentiation between healthy colon tissue and carcinoma colon tissue according to Example 2.

The labels on the left side of the plot are gene and CpG- identifiers, these can be cross referenced using Table 4 and Table 7. The labels on the right side of the figure give the significance (p-value, T-test) of the difference between the means of the two groups. Each row corresponds to a single CpG and each column to the methylation levels of one sample. CpGs are ordered according to their contribution to the differentiation between the two tissue types (A = healthy, B = carcinoma) with increasing contribution from top to bottom. Black indicates total methylation at a given CpG position, white represents no methylation at the particular position, with degrees of methylation represented in gray, from light (low proportion of methylation) to dark (high proportion of methylation).

### Figure 3

Differentiation between healthy colon tissue and adenoma colon tissue according to Example 2.

The labels on the left side of the plot are gene and CpG identifiers, these can be cross referenced in Table 5 and Table 7. The labels on the right side give the significance (p-value, T-test) of the difference between the means of the two groups. Each row corresponds to a single CpG and each column to the methylation levels of one sample. CpGs are ordered according to their contribution to the distinction to the differential diagnosis between the two tissue types (A = healthy, B = adenoma) with increasing contribution from top to bottom. Black indicates total methylation at a given CpG position, white represents no methylation at the particular position, with degrees of methylation represented in gray, from light (low proportion of methylation) to dark (high proportion of methylation). Due to formating of the page only 40 CpGs are shown in this figure.

### Figure 4

Differentiation between carcinoma colon tissue and adenoma colon tissue according to Example 2.

The labels on the left side of the plot are gene and CpG identifiers, these can be cross referenced in Table 6 and Table 7. The labels on the right side give the significance (p-value, T-test) of the difference between the means of the two groups. Each row corresponds to a single CpG and each column to the methylation levels of one sample. CpGs are ordered according to their contribution to the distinction to the differential diagnosis between the two tissue types (A = carcinoma, B = adenoma) with increasing contribution from top to bottom. Black indicates total methylation at a given CpG position, white represents no methylation at the particular position, with degrees of methylation represented in gray, from light (low proportion of methylation) to dark (high proportion of methylation).

## Claims

1. A method for detecting and differentiating between colon cell proliferative disorders associated with at least one gene and/or their regulatory regions from the group comprising MDR1, APOC2, CACNA1G, EGR4, AR, RB1, GPIb beta, MYOD1, WT1, HLA-F, ELK1, -APC,. BCL2, CALCA, CDH1, CDKN1-A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1. MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 in a subject, said method comprising contacting a target nucleic acid in a biological sample obtained from said subject with at least one reagent or a series of reagents, wherein said reagent or series of reagents, distinguishes between methylated and non methylated CpG dinucleotides within the target nucleic acid.

2. A method according to claim 1 wherein, said method differentiates between normal colon tissue and colon cell proliferative disorder tissue.

3. A method according to claim 2 wherein, the method is carried out by means of methylation state analysis of genes from the group comprising APC, CALCA, CAV1, CD44, CDH1, CDH13, CDKN2a, CSPG2, DAPK1, EGFR, EGR4, ESR1, GSTP1, GTBP/MSH6, HLA-F, IGF2, LKB1, MLH1, MYOD1, N33, PTEN, PTGS2, TGFBR2, TP73, TPEF (=TMEFF2; =HPP1), WT1, ERBB2.

4. A method according to claim 1 wherein, said method differentiates between colon adenoma tissue and normal colon tissue.

5. A method according to claim 4 wherein, the method is carried out by means of methylation state analysis of genes from the group comprising APC, AR, BCL2, CALCA, CAV1, CD44, CDH1, CDH13, CDKN2a, CEA, CSPG2, DAPK1, EGFR, EGR4, ESR1, GPIb beta, GSTP1, GTBP/MSH6, HIC-1, HLA-F, IGF2, LKB1, MGMT, MLH1, MSH3, MYC, MYOD1, N33, PCNA, PGR, PTEN, PTGS2, RARB, RASSF1, S-100A2, TGFBR2, TP-73, TPEF (=TMEFF2; =HPP1), WT1, ERBB2

6. A method according to claim 1 wherein, said method differentiates between colon carcinoma tissue and normal colon tissue.

7. A method according to claim 6 wherein, the method is carried out by means of methylation state analysis of genes from the group comprising APC, CAV1, CD44, CDH13, CSPG2, EGFR, GSTP1, HLA-F, IGF2, N33, PTEN, PTGS2, TP73, TPEF (=TMEFF2; =HPP1), ERBB2

8. Use of methods according to claim 1 wherein, said methods are used to differentiate between colon adenoma tissue and colon carcinoma tissue.

9. A method according to claim 8 wherein, the method is carried out by means of methylation state analysis of genes from the group comprising GPIb beta and CDKN2a.

10. A method according to any one of Claims 1 to 9 comprising the following steps:
- obtaining a biological sample containing genomic DNA
- extracting the genomic DNA
- converting cytosine bases in the genomic DNA sample which are unmethylated at the 5-position, by treatment, to uracil or another base which is dissimilar to cytosine in terms of base pairing behaviour;
- fragments of the pretreated genomic DNA are amplified
- identification of the methylation status of one or more cytosine positions

11. The method according to claim 10, **characterized in that** the reagent is a solution of bisulfite, hydrogen sulfite or disulfite.

12. The method as recited in Claims 10 and 11, **characterized in that** the amplification is carried out by means of the polymerase chain reaction (PCR).

13. The method as recited in one of the Claims 10 through 12, **characterized in that** the amplification is carried out by means of a heat-resistant DNA polymerase.

14. The method as recited in one of the Claims 10 through 13, **characterized in that** more than ten different fragments having a length of 100 - 2000 base pairs are amplified.

15. The method as recited in one of the Claims 10 through 14, wherein the amplification step is carried out using a set of primer oligonucleotides comprising SEQ ID NO: 389 through SEQ ID NO: 518.

16. The method as recited in one of the Claims 10 through 15, **characterized in that** the amplification of several DNA segments is carried out in one reaction vessel.

17. The method as recited in one of Claims 10 through 16, **characterized in that** the amplification step preferentially amplifies DNA which is of particular interest in healthy and/or diseased colon tissues, based on the specific genomic methylation status of colon tissue, as opposed to background DNA.

18. The method according to one of Claims 10 through 17, **characterized in that** the methylation status within at least one gene and/or their regulatory regions from the group comprising MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 is detected by hybridization of each amplificate to an oligonucleotide or peptide nucleic acid (PNA)-oligomer.

19. A method according to claim 18, **characterized in that** the olignonucleotide or peptide nucleic acid (PNA)-oligomer is taken from the group comprising SEQ ID NO: 519 to SEQ ID NO: 1030.

20. The method according to Claims 10 through 19, **characterized in that** the amplificates are labelled.

21. The method as recited in Claim 20, **characterized in that** the labels of the amplificates are fluorescence labels.

22. The method as recited in Claim 20, **characterized in that** the labels of the amplificates are radionuclides.

23. The method as recited in Claims 20, **characterized in that** the labels of the amplificates are detachable molecule fragments having a typical mass which are detected in a mass spectrometer.

24. The method as recited in one of the Claims 10 through 23, **characterized in that** the amplificates or fragments of the amplificates are detected in the mass spectrometer.

25. The method as recited in one of the Claims 23 and 24, **characterized in that** the produced fragments have a single positive or negative net charge.

26. The method as recited in one of the Claims 23 through 25, **characterized in that** detection is carried out and visualized by means of matrix assisted laser desorption/ionization mass spectrometry (MALDI) or using electron spray mass spectrometry (ESI).

27. A method according to Claims 1 through 9 comprising the following steps;
a) obtaining a biological sample containing genomic DNA
b) extracting the genomic DNA
c) digesting the genomic DNA comprising at least one or more CpGs of the genes MDR1, APOC2, CACNA1G, EGR4, AR, RB1, GPIb beta, MYOD1, WT1, HLA-F, ELK1, APC, BCL2, CALCA, CDH1, CDKN1A, CDKN1B (p27 Kip1), CDKN2a, CDKN2B, CD44, CSPG2, DAPK1, EGFR , ERBB2, GSTP1, GTBP/MSH6, HIC-1, HRAS, IGF2, LKB1, MGMT, MLH1, MNCA9, MSH3, MYC, N33, PAX6, PGR, PTEN, RARB, SFN, S100A2, TGFBR2, TIMP3, TP53, TP73, VHL, CDKN1C, CAV1, CDH13, DRG1, PTGS2, THBS1, TPEF (=TMEFF2; =HPP1), DNMT1, CEA, MB, PCNA, CDC2 , ESR1, CASP8, RASSF1, MSH4, MSH5 with one or more methylation sensitive restriction enzymes
d) detection of the DNA fragments generated in the digest of step c)

28. A method according to Claim 27, wherein the DNA digest is amplified prior to Step d).

29. The method as recited in Claim 28, **characterized in that** the amplification is carried out by means of the polymerase chain reaction (PCR).

30. The method as recited in one of the Claims 28 and/or 29, **characterized in that** the amplification of more than one DNA fragments is carried out in one reaction vessel.

31. The method as recited in one of the Claims 28 through 30, **characterized in that** the polymerase is a heat-resistant DNA polymerase.

32. An isolated nucleic acid of a pretreated genomic DNA according to one of the sequences taken from the group comprising SEQ ID NO: 133 to SEQ ID NO: 388 and sequences complementary thereto.

33. An oligomer, in particular an oligonucleotide or peptide nucleic acid (PNA)-oligomer, said oligomer comprising at least one base sequence of at least 10 nucleotides which hybridizes to or is identical to a pretreated genomic DNA according to one of the SEQ ID NO: 133 to SEQ ID NO: 388 according to Claim 32.

34. The oligonucleotide as recited in Claim 33; wherein the base sequence includes at least one CpG or TpG dinucleotide sequence.

35. The oligonucleotide as recited in Claim 34; **characterized in that** the cytosine of the at least one CpG or TpG dinucleotide is/are located approximately in the middle third of the oligomer.

36. An oligomer, in particular an oligonucleotide or peptide nucleic acid (PNA)-oligomer, according to one of the sequences taken from the group comprising SEQ ID NO: 519 to SEQ ID NO: 1030.

37. A set of oligonucleotides, comprising at least two oligonucleotides according to any of Claims 33 through 36.

38. A set of oligonucleotides, comprising at least two oligonucleotides according to SEQ ID NO: 895-986.

39. One or more isolated nucleic acid(s) taken from the group according to SEQ ID NO: 65 - 110.

40. A set of oligonucleotides, comprising at least two oligonucleotides according to SEQ ID NO: 895 - 906, 909 - 918, 921 - 924, 931, 932, 941, 942, 971, 972, 987 - 990.

41. One or more isolated nucleic acids) taken from the group according to SEQ ID NO: 65 - 70, 72- 76, 78, 79, 83, 88, 103, 111, 112.

42. A set of oligonucleotides, comprising at least two oligonucleotides according to SEQ ID NO: 895 - 954, 957 - 962, 965 - 970, 975- 978, 981- 986, 991 - 1028.

43. One or more isolated nucleic acid(s) taken from the group according to SEQ ID NO: 65 - 94, 96 - 98, 100 - 102, 105, 106, 108 - 110, 113 - 131.

44. A set of oligonucleotides, comprising at least two oligonucleotides according to SEQ ID NO: 1005, 1006, 1029, 1030.

45. One or more isolated nucleic acid(s) taken from the group according to SEQ ID NO: 120, 132.

46. A set of oligomers, peptide nucleic acid (PNA)-oligomers and/or isolated mucleic acids as recited in Claims 37 through 45, comprising oligomers for detecting the methylation state of all CpG dinucleotides within one or more of the sequences according to SEQ ID NO: 1 to SEQ ID NO: 64 and sequences complementary thereto.

47. Use of a set of oligomers or peptide nucleic acid (PNA)-oligomers according to any of Claims 33 through 38, 40, 42 and 44 as probes for determining the cytosine methylation state and/or single nucleotide polymorphisms (SNPs) of sequences according to SEQ ID NO: 1 to SEQ ID NO: 64 and sequences complementary thereto.

48. Use of a set of oligonucleotides according to Claim 38 or nucleic acid(s) according to Claim 39 for the differentiation between colon adenoma or colon carcinoma tissue and normal colon tissue.

49. Use of a set of oligonucleotides according to Claim 40 or nucleic acid(s) according to Claim 41 for the differentiation between colon carcinoma tissue and normal colon tissue.

50. Use of a set of oligonucleotides according to Claim 42 or nucleic acid(s) according to Claim 43 for the differentiation between colon adenoma tissue and normal colon carcinoma.

51. Use of a set of oligonucleotides according to Claim 44 or nucleic acid(s) according to Claim 45 for the differentiation between colon carcinoma tissue and colon adenoma tissue.

52. A set of at least two oligonucleotides or peptide nucleic acid (PNA)-oligomers as recited in Claim 33, as primer oligonucleotides for the amplification of DNA sequences of one of SEQ ID NO: 133 to SEQ ID NO: 388 according to Claim 32 and/or sequences complementary thereto and segments thereof.

53. Use of a pretreated genomic DNA according to Claim 32 for the determination of the methylation status of a corresponding genomic DNA and/or detection of single nucleotide polymorphisms (SNPs).

54. A set of oligonucleotides or peptide nucleic acid (PNA)-oligomers as recited in Claims 37, 38, 40, 42 or 44, **characterized in that** at least one oligonucleotide is bound to a solid phase.

55. A set of oligonucleotides or peptide nucleic acid (PNA)-oligomers as recited in Claims 37, 38, 40, 42 or 44, **characterized in that** all members of the set are bound to a solid phase.

56. A method for manufacturing an arrangement of different oligomers or peptide nucleic acid (PNA)-oligomers (array) for analyzing diseases associated with the corresponding genomic methylation status of the CpG dinucleotides within one of the SEQ ID NO: 1 to SEQ ID NO: 64 and sequences complementary thereto, wherein at least one oligomer according to any of the Claims 37, 38, 40, 42 or 44 is coupled to a solid phase.

57. An arrangement of different oligomers or peptide nucleic acid (PNA)-oligomers (array) obtainable according to claims 54 and 55.

58. An array of different oligonucleotide- and/or PNA-oligomer sequences as recited in Claim 57,
**characterized in that** these are arranged on a plane solid phase in the form of a rectangular or hexagonal lattice.

59. A nucleic acid or peptide nucleic acid array for the analysis of colon cell proliferative disorders-associated with the methylation state of genes comprising at least one nucleic acid according to one of the preceeding claims.

60. The array as recited in any of the Claims 57 through 59, **characterized in that** the solid phase surface is composed of silicon, glass, polystyrene, aluminium, steel, iron, copper, nickel, silver, or gold.

61. A kit comprising a bisulfite (= disulfite, hydrogen sulfite) reagent as well as oligonucleotides and/or PNA-oligomers according to one of the Claims 33 through 46.

62. The use of oligonucleotides or peptide nucleic acid (PNA)-oligorners -according to the groups of SEQ ID NO: 65 to SEQ ID NO: 132 and SEQ ID NO: 519 to SEQ ID NO: 1030 for the detection of a predisposition to, differentiation between subclasses, diagnosis, prognosis, treatment, and/or monitoring of colon cell proliferative disorders.

63. A DNA sequence according to one of the sequences taken from the group comprising SEQ ID NO: 133 to SEQ ID NO: 388 and sequences complementary-thereto for use in the analysis of cytosine methylation within said nucleic acid for the detection of a predisposition to, differentiation between subclasses, diagnosis, prognosis, treatment and/or monitoring of colon cell proliferative disorders.


Sequence listing

<110> Epigenomics AG

<120> test

<160> 1030

<210> 1
<211> 2932
<212> DNA
<213> Homo Sapiens

<400> 1

gccaagaaca aaatatatca agataaggaa aatttgtagt caagaataga aaaaaattat 60
ggctttgaag tatgagttat ttaaagaaag tggaaacatc ctcagactat gcagtaaaaa 120
acaaagtgat tttcttcttc taaacttatg caataaactg ataggtaata tgtgaaagtc 180
atagaatgta gactagagga tacaacaaac ctatttcctc tatgttcata agaagtaaga 240
aaagctctga tgtgagttag cattgcttta caattttgaa ttgtgcagat tgcacgtact 300
tttcctcagt ttgaagtaaa tagtggacag gaaaaaatat taaatgttgg cagtaaatat 360
ggaaggaaat tacaactaat gtaatatgct aaaacatgct atgtttattt tactaatttg 420
aattaaaatg taagaattta aaatgccctg gaaaaacacg ggcattgatc tgacgtctga 480
agttttaaaa tattacacac tttgaaatag catttgtacc ttgaaatacc tgtctctata 540
tattttttaa aacttccttt ttctttcatt ccatttatca tcaaataaag gatgaacaga 600
tgtaactcag aaactgtcaa gcatgctgaa gaaagaccac tgcagaaaaa tttctcctag 660
ccttttcaaa ggtgttagga agcagaaagg tgatacagaa ttggagaggt cggagttttt 720
gtattaactg tattaaatgc gaatcccgag aaaatttccc ttaactacgt cctgtagtta 780
tatggatatg aagacttatg tgaactttga aagacgtgtc tacataagtt gaaatgtccc 840
caatgattca gctgatgcgc gtttctctac ttgccctttc tagagaggtg caacggaagc 900
cagaacattc ctcctggaaa ttcaacctgt ttcgcagttt ctcgaggaat cagcattcag 960
tcaatccggg ccgggagcag tcatctgtgg tgaggctgat tggctgggca ggaacagcgc 1020
cggggcgtgg gctgagcaca gccgcttcgc tctctttgcc acaggaagcc tgagctcatt 1080
cgagtagcgg ctcttccaag ctcaaagaag cagaggccgc tgttcgtttc ctttaggtct 1140
ttccactaaa gtcggagtat cttcttccaa aatttcacgt cttggtggcc gttccaagga 1200
gcgcgaggta ggggcacgca aagctgggag ctactatggg acagttccca agtgtcaggc 1260
tttcagattt cctgaacttg gtcttcacgg gagaagggct tcttgaggcg tggatagtgt 1320
gaagtcctct ggcaagtcca tggggaccaa gtggggttag atctagactc aggagctcct 1380
ggagcagcgc ccaaaccgta gtggcactgg accatgttgc ccggagcgcg cacagcccgc 1440
gcggtgcggg gacctgctct ctgagcccgc gggcggtggg tgggaggaag catcgtccgc 1500
ggcgactgga accgggaggg agaatcgcac tggcggcggg caaagtccag aacgcgctgc 1560
cagaccccca actctgcctt cgtggagatg ctggagaccc cgcgcacagg aaagcccctg 1620
cagtgcccat cgcggccaga gcagctgggg catcaacggc gggcgctccc tcttactgct 1680
ctctggcttc gacgggggac tagaggttag tctcacctcc agcgcgcctg aggctcatgc 1740
atttggctaa tgagctgcgg tttctcttca ggtcgggatg gatcttgaag gggaccgcaa 1800
tggaggagca aagaagaaga acttttttaa actgaacaat aaaaggtaac tagcttgttt 1860
cattttcata gtttacatag ttgcgagatt tgagtaattt atttctagcc tccagctctg 1920
aaataaatga catgttgttg tttttaatta tttttaagaa acgcaagcta gcctttggaa 1980
tcaatatccc tgcttagagc agaagtttgt tggctgagtg gagcacagca tatgcatttt 2040
ccctgtcttt tttgttcttt cttttaatga tacataatat tttacatatt tatgaaatgg 2100
ggtacatgga agcgtttttt acatgcccgg aatgtgtaat gatcaagtcc gggtatttga 2160
aggatacatc accttaggta tatttcattt ctatgtgttg ataacatttt aagtcttcta 2220
gctactttga aatatacaat atattgctaa ctgtagtcac cctcgtctgc tatcgaacat 2280
tggaacttat ttgtcctatc caaccgttct tagtcattca ccaacctctt ttcatttcac 2340
ctttttaccc ttcccggcct ctttccctta gtcttggtgt gcctctttct cagctttcct 2400
gccccagaca ggcggatgct catatgtgtt tctgtcttat gaacttctgc ttttcaagtg 2460
gtgttggtcg cccacacgtg agccatatgc tgctggtgat ctgctctgtg gtccaggctc 2520
ttgcttccgg taaatggcta tgtaaacatc gcgtttgtgg cctggctgat gagacagaag 2580
gtcaaaagta catttaggtt gttaactggc aataaatatc tgtatataat attggtaatg 2640
taatcatata gggaaaataa ttatttaaag taaattttga tcatggtgct ctgcctttat 2700
agaatattta aaacttcact aaatagattc attgttagta gtaaattgta aaatagacta 2760
gtaagtttaa taatattaga aactgtaatg taaattataa gataaattag ctaaacacat 2820
taatattata agaaaccaag cttttcagtg taagagaaaa aatacaaatg tggaaatcaa 2880

atacattttt aaaaataatg ttaagtttga attagaaatt tcaatatgaa tt          2932

<210> 2
<211> 7432
<212> DNA
<213> Homo Sapiens

<400> 2

atctgaagcc agagtcaccc agaggagaaa gagttggaat tgagaactca aggaatgctt      60
ggaagtgatc gggctcgagc ccacctagga agaaacagag gctggagaca tgagactgtg     120
ttgctatttc ctctcatcaa cccttgggcc ctattgaggc cctaccacaa gcctggccct     180
gcagcccagt gactaggaga aattagacac aagataataa taacagcaat gatctttttt     240
tttttctgga gacggagtct tgctctttcg cccaggctgg actgcagtgg cgcgatctcg     300
gctcaatgca agctccacct cccaggttca cgccattctc ctgcctcagc ctcccgagta     360
gctaggacta caggcgcctg ccaccacgcc tggctaattt ttcatatttt tagtagagat     420
ggggtttcac cgtgttagcc aagatggtct caatctcctg acctcgtgat ccgcctgcct     480
cggcctccca aagtgttggg gttacaggca tgagccaccg cgcctggcca acagcaatga     540
tctttgagca cctatattgc cagtctccac ggtaagagct ttcttcattt tttgttttgt     600
tttgtttcaa gacagagtct tgctctgtca cccaggctgg agtgcagtgg tgtgatcgcg     660
gctcactgca gccttcactt cccgggttca agccattctc ctgcctcagc ctcccaagta     720
gctgggatta caggcacgca tcactacttc tggctaattt ttgtattttt agtagggaca     780
gggtttttca ccatgttggc caggttggtc tcaaactcct ggcctcatat gatctgccca     840
cctcggcctc ccaaagtgct gggattacag gcgtgagcca ctgcgccttt ctttgtattt     900
gttcaagtaa tatactgaaa tatgtactgt gcctcccact ttatggagga ggaaactgag     960
gccagcaaat gaggctgtca tgggaggtgg agacaggatt tgaacctgcc tcagtgcagg    1020
aggctcaaga gcctctgtct tctctcaggg cactgtgtgg gagggtgaga aggagggagg    1080
cccacagagg catgacctct gattgccact gtcacctggg ccctgctctc tgaagtctct    1140
gccaagcggg gaggtggccg gggagggccc ctgctctgtg cagcctcccc tcccccggcc    1200
cgcagagttg agcacagagg gacagaggca cggaaccccc agaaatgtcc ctcctcagaa    1260
acaggctcca ggccctgcct gccctgtgcc tctgcgtgct ggtcctggcc tgcattgggg    1320
gtgagaagaa gtgggtggag ggatgtgggg cccacacctg gtgggtgtga gtgtggctgt    1380
gtgtcctgtg gctctgtagc cacgtgagac atgagtacgg agtgtgtgcg tttcatggcg    1440
tgcgtatgca tgtgcgtgtc ggggagtgtg tgtgtcggtg gctgagagtg aagtgtgaat    1500
gtcacattgg tacaaactgg gatcatctgt gtgtgtgcac gtgcgtgcgt ggaagtggga    1560
gtatgcagtc gtggtaaaaa agtgcatgtc tgtgtgcata tgtgtatttg tgtgcacctg    1620
tctctctgtg gggtatgtgt gtgcaaaata tttgagtgtg tggacatgtg tgagggggtg    1680
agtgtgtgct ggtgtgtacg tctgtgtttt gcatatgcat tttttttttt ttttttgaga    1740
cggagtctca ctctgtcacc caggctggag tgcagtggta gcagtggtgc gatcttggct    1800
cactgcatca tccgcctacc cgtttcaagg gattctcctg cctcagtctt cagagtattt    1860
gggactacag acacacgcca ccatgcctgg ctattttttt tttttgagac ggagtctcgc    1920
tctgttaccc aggctggagt gcagtggcgt gatcttggct cactgcaacg tccgcctccc    1980
gggttcacgc cattctcctg cctcagcctc ccgagtagct gggactacag gagcccacca    2040
ccacgcctgg ctaatttttt gtatttttac tagagacggg gtttcgccgt gttagccagg    2100
atggtctcca tatcctgacc tcgtgatccg cctgcctcgg ccttccaaag tgctaggatt    2160
ataggcgtga gccactgcgc ctggccaatg cctggctaat ttttttatat ttttggtaga    2220
gacagggttt tgccatgttg cccaggctgg tcttgaaatc ctgacctcag gtgatccgcc    2280
cgccttggcc tcccaaagtg ctgggattac aggcatgagc caccacgccc ggccatgtac    2340
tttatgttaa aatgggatca tattctagat cagcattatc cagtagaaat ttaaattttt    2400
aatacagggc caggcacggt ggctcatgcc tgtaatccca gcactttcgg aggccgaggc    2460
gggtggatcg caaggtcagg agatttgaga tcatcctggc taacagatgg gtaaaaaccc    2520
atctctacta aaaatacaaa aaattagcca tgcatggtgg catgcgcctg tagtcccagc    2580
tactcgggag gctgaggccg gagaatcact tgaacccggg aggcagaggt tgcagtgagc    2640
cgagatcgcg ccactgcatt ccaacctggg tgacagagcg agactccgtc tgaaaaaaaa    2700
aaaaaattta acacgtatgt agacaatgtg caaggcacca ttccatgtgc atcgtatgta    2760
gtaactctta attctcacga taaccctgag gtagatatta ttaccccgtt ctacaaaagg    2820
agaaacagtc ctggggagac aggataagtc accggccaag gcacacagct agctacatgt    2880
ggcccccgcg tgacggctgg tctctgtagg cgaggctttg tccagatgcg tgggtagaag    2940
gtctggcccg gaaagaggaa ctgacagcaa ggctaagcca atgtctgccc ctgggggcag    3000
aaagtcacct cctgctctcc ctccactgtc cacagaggta gctcagacag ggtgggggtc    3060
acaggagaac gaagggagaa gggggtagtt cctgggcagc aaaatcaggt ggtgaaggga    3120
ggcatcagag gatggcaatt agagaggcca ttagagggga accacaggca gacagggtga    3180
caggagggac tactgacaca aggtgaagag atggcccagc cggacggggt ggctcacatc    3240

tgtaatccca gcattttggg agcccgaggt gggtggatca cttgaggtca ggagttcgag 3300
gccccaacat ggcaaaaccc catctcttct aaaaatacaa aaattagccg ggcatgatgg 3360
cagatgcctg taatccctgc tactcgggag gctgaggcag gaaaattgcc tgaatccagg 3420
aggtggaggt tgcaatgaga cgagatcatg acactgcact ccaccctggg caacagagca 3480
agagactgac tctgtctcat aaaaaaaaag aaaaaagaaa aaaaaaaaga gatggctgat 3540
ggttaaagag gggttagcgg tcaggggaca cataagggta aaggcaggag gcaagaggac 3600
tggcaggggg ctgcccctgg gccaccggga gcgacacagg atgagcatgg agggaaaggg 3660
agaaggggat tctagggtcc cagcctaccc aagttgccct ctggttccac ctagcatgcc 3720
agccagaggc ccaggaagga accctgagcc ccccaccaaa gctaaagatg agtcgctgga 3780
gcctggtgag gggcaggatg aaggagctgc tggagacagt ggtgaacagg accagagacg 3840
ggtggcaatg gttctggtga gggtgtgctg ggctgggtgg tgggagggga ctcctgggtc 3900
tgagggagga ggggctgggg cctggacccc tgagtctcag ggaggaggaa agggtgggag 3960
tggggctgtg acccctaggt ctgggaggag tggagggtta gagctgagag caggaactcc 4020
taggtcacag agaggagcgg ataaatgggg cagagaacac ctggggagag ctggggcctc 4080
cactgtgatg tcctctctcc tgtaggagcc cgagcacctt ccggggcttc atgcagacct 4140
actatgacga ccacctgagg gacctgggtc cgctcaccaa ggcctggttc ctcgaatcca 4200
aagacagcct cttgaagaag acccacagcc tgtgccccag gcttgtctgt ggggacaagg 4260
accagggtta aaatgttcat aaaagccagg tgtggttgtg gcgggtgcct gtagtcccag 4320
ctactcagga ggctgaggta ggatgatggc ttgagcccag gagttcgaga ccagcctggg 4380
caacacagcg agatctcttg ggggtaaaac aaaaagaaaa aaaaaagttc atacttctcc 4440
aataaataaa gtctcacctg tgtccctgtc tggatccttc cccagtgtgg ccagaaaaaa 4500
acccacccca ctgcctccca ggaatcaatg agtagaagag gtgacacctg atggggaagg 4560
aagagtaggg aggtcgggaa gggtatcaag gaataacacc ctattgtggg cttgcggaga 4620
atgggggact tcaaggcgtg tcagtttcag gagggtgagg gcaggagcgt gggtggagtc 4680
agcaggtccc catgatggcc ctcactgaga gcttcgccct tgtctcctac aagctctgac 4740
tccattccca gtgggcaccc agcacctcca acccctccac agcccccaac ccagcctctg 4800
tcggaggcga attctcagag tgagggttcc ctgtcacttg agagaaggtt ccctgtgacg 4860
tgaccttggg ggacgtcatt gccctttctg tccccaccca ccccctccgc agttctgttg 4920
gccaggactt tggcctagac aaaggatggg ggttgtggct gtggagcgga agtgggtctc 4980
aaccactata aatcctctct gtgcccgtcc ggagctggtg aggacagcct gccagagtct 5040
ggtaagaaag ggactcaggg tgcggggaca ggggggcgtc agcagggaga gggcaaagat 5100
cgataaagca ggaattttaa gaggcacaat attagaagcc cgtgttggaa ccatgactgt 5160
gtgtgtgtgt gtgtgtgtgt gtgtgtgtgt gtgtgtgtgt gagagagaga gagagggaga 5220
tggagtctcg ctatgtagcc caggctagac tcaaactcct gggctcaagc aatcctcctg 5280
cctcagcctc cccagtagct gggactacag gtgcaccacc acactccaca aatcacagaa 5340
tttagaactg tagactattt gagcttctgc ttagagttag ggtggctgag gtggggagga 5400
tcccttgagc ccaggagttt gaggatgcag tgagctgtga tcttgccacc gtgttccagc 5460
ctgggtgaca gagaaacccc atttctaaaa aagagaaaga aaaagggata ggtacaatgg 5520
ctcatgcctg taatcccagc actctgggag gccgaggcgg gtggatcact tgaggtcagg 5580
agttcgacac cagccttacc agcatggtga aaccgcatct atactaaaaa tacaaaaatt 5640
ggccgggtgt ggtagcatat gcctgtaatc ccagctattc cagaggctga gacaggagaa 5700
ttgcttgaac ccaggaagcg gaggttgcag tgagcccaga tcgtgccact gtactctagc 5760
ctgggtgaca gagcaagact cagtcttggc ggaaaaaaag aatgaaaaaa tttaaaaaac 5820
taaaaaagaa ctgtaggctg ggcgtggtgg cttacacttg taatccaaac gctttgggag 5880
gccaaggcaa acggatcact tgatgtcagg agttggagac cagcctggcc aacatggtga 5940
aaccccgtct ctactaaaaa tacaaaaatt agacaggcat ggtggtgcat gcttgtattt 6000
ccagttactc aggaggctga ggcaggagaa tcgctcgaac ccggaagaca gaggttgcgg 6060
tgagccaaaa ttgcgccatc gcactccagc ctgggcgaga gaacaagacc ttgtcttgga 6120
aaaaaaaaaa gaattgtaga ccatttgctt gtgttctttc tccggggatc agatctcacc 6180
ctctttctgc cgtacttcct catctcctac gtgtggatga tgatattgtg ccctgtgcat 6240
gttcttcgtc accaaaagtg cctctctcat agagcaggtg agaactcagt gaggagatgc 6300
agggacatga ggtctgactt agggcagagc cctaaggtaa cacatttgat ctactgtagg 6360
tccttaatgg tgtctgcaga gcacctccct gcactgactc agccttagca aagggcagag 6420
gctttgctgt gttccctgct gggcccagaa ctgtttaggt gctcaagaaa gccttctagg 6480
ctgggctcag tggctcacac ctgtactccc agcaccctgg ggaggccgag atgggaggat 6540
cgcttgagcc caggagttcc agaccagcct gggcaacaaa acaagtctcc catctctaca 6600
aaagaataaa aattagcagc tgggcatggt ggctcatgcc tgtaattcca gcactttggg 6660
aggccaaggc aggcaaatca cttgaggtta ggagttcaag accagcctgg ccaacatggt 6720
gaaaccccat ctctactaaa aatacaaaaa tcaggtgggg cacagtggct caagcctgta 6780
atcctagcac tttgggaggc caaggtgggc ggatcacgag gtcagaagtt cgagaccagc 6840
ctggccagca tggtgaaacc ccatctctac taaaaataca aaatattagc cgggcatggt 6900
ggcaggtgct tgtgattcca gctccttggg aggctgaggc agaagaattg ctagaaccct 6960
ggaggcagag gttgcagtga gccgagaaca cgccactgca ctccagcctg ggtgacagag 7020

cgagactcca tctcaaaaaa tacgaaaaca aaaatcagcc gggtggtggc gggtgcctgt 7080
aatcccagct actggggagg ctgaggcagg agaattgctt gaacctggga ggtgggggtt 7140
gcagtgagcc aagattgcac cactgcactc cagcctgggc aacagagtga gattccatct 7200
caaaaaagaa aaaaataata attaaaatgt taaaatcagg agtagaatca cagaatgttg 7260
gaaagtgagg cccaagaagg gggctgtgtc caagtccatg catgggaaac ttgactggga 7320
caccgagctc acacagagca ggatctcagt cccccccacc agagtggggc gtgaccacag 7380
gaacagccgc ctccagtcag cctgccacat gacaccccct caatgttcca gg 7432

<210> 3
<211> 12423
<212> DNA
<213> Homo Sapiens

<400> 3
   tctatctgag tccttcttgc tgcagagaaa gccttcctgt gacctcatcc tttcccctaa 60
   atgttgggaa atgctcctga gtcctgtcct tgacacatac atcttccttg ggttgaggga 120
   tggggactgg gacacatgct ttgggaaggg caggtgcaga gtgatggggc tgacggggtg 180
   ggggaagcag ggggaggagc tgtccctctg aggcctttgg gaatcggctg tgccaggcat 240
   cctcagccct ccagctaagc aacacagaga gccaaaagag gctactagag acaagaaggc 300
   cttcatgggg ccactgcttg tgggggatgt cagcagtggg gaggagtgaa gcaggaagga 360
   accgggagac tggatggaat tgctcccttc acacggtcac acatagccac acatggtcac 420
   tgggaccaga gtcagctctg tctgggcagc caggccctca gtgcaagcca ccagcccatc 480
   tggaggggat tagtgctccg gccaacaagg tcagctggcc ccttcctagc tggagcctac 540
   tcaaccttgc caggaagtca gagagctgga caagtggagc ccagcctggg gaggtggaag 600
   agagggagga tggagcatgg tgaagcacag gtggcctttt tggcagcccc agccctggct 660
   ttggaacagt ctgggcagtg tgccaacccc tcttgccact gtcgtcccat tgaccctcat 720
   gaatgagttg cgaggcagtt accttcagcc tcctatggat aaatattcga ggcccagaga 780
   gggtaagaga cctgcctgcg acccctcagc acttctgttt ctctctgggg tcttgagggt 840
   acaataaaga cccctaaggc ttcctcttct cgcaggaggt ccaggcgcag ctgtggggga 900
   gggtgccctt ggtgtcttct gtccctgcag ccagtctgct ttctactcgg cagctcctct 960
   ctccctcctg ggatgagatg tgcacgcgat gatgggattc tgtaaatggg ttgggaagaa 1020
   ggagttacct ccttaaaggg ttagttctcc caggagtgct cgaatttcta aagggggcag 1080
   ggtcccacat aaatctcact cagaagcagg attataagag ttcctgcagc atcccttccc 1140
   ctttgctggt ccctcttgag tgtgcaccct gttctaccag cggaacacct gcgggccagg 1200
   gtcactggag acagggccat accgtctggg cggtcactta agtaacttca tcattctgag 1260
   cacccccctt ccttatctga aaggtggggg gccatccacc cagctctcag ggctgttaag 1320
   gtgactaaat gggataatgg ttgtatcatc cctgagcaaa gggtatcaca gatgggaaga 1380
   ttatcttagc tccgcaggga gccagatggg ctgtgaggga ggggtaaagg cagggctgga 1440
   gggggctgga ggggctggct gagcagggta tatatggggg gggttcttcc tcaaacccag 1500
   aaaaactctt agttaggctt agtgtctttg ttgagaaact gaggcccaaa gaaaggaatt 1560
   tcttgtccct ttgtttccct ttccctgcct gaggtcattc attcacctaa caactttgtg 1620
   cccgacacta gacaaggccc tagtgagcaa aatgaacata gcccctctgg gaacacagag 1680
   cctagatagg gagatgatca tttcattact gcaaacacaa acatataatc ccagcctggg 1740
   ctaagtgctg tgaagttcca gacttgtgtc tggctcggtg ctgtatccta agcaccttgg 1800
   cacttaagtg gcattgaaat acttgctgga aagaaggcca ctctgaggag gtgatgtcag 1860
   agctgaggct tggagggctg gagttaaata aagatggttt caggtcttct ttgccttccc 1920
   tgtaggttct gcctctgcct cctctctggc actcaggcta agggtaagca tctccgactg 1980
   gccatcagcc ccaacatgcc gagtacaggg atcagatcct gtcttgcgtg gccggtctcc 2040
   cctaggtcct gaacccaggg aagtgcatgt ggatctcaat ttatctagat ggtgcgtttg 2100
   tgcctgccct cctttcgggc atgttctaga acagagttca gtccagagcc caggggtggc 2160
   cgggatgatg gatgagggtg tggggcctgc tgaccaccag gggacctgat cattgggctg 2220
   ggccagcctc tctgtcctca ttttcccctc tgcaaagaag gggtgttggg cgtccctact 2280
   tctcgcgcca gccccggggc ctctatcctg gcgggaaggg caggccgacc cggcagactg 2340
   cggcctctcg ggagggaaga aggtgtcaga cgcgcggagc aaccataaat agcccccctt 2400
   tcccagaaga cggcacgggg ttcaagactc aggcgccgca tactcagaat gagagcagag 2460
   actcccgcca ggaaaaaaag gcacttaggg gatctgctca ttagcatgaa atgcaaatga 2520
   gcccgcccgg cctcatttac acaactctgt gcatggattc ggcgaaaggg caaccaggga 2580
   gacgacggcg cagcagccac tctgccactt cccccatccc ctccccccat cggccggggc 2640
   gggaactgag acgaccccaa ccctctgcgg tggcgggagg tgcgcggggg ctgcgtgggt 2700
   ggtgcagcct taggagagtg aacaacgccc aggggtgatg gcctcagcaa agtgaggggt 2760
   ggtgatggag gtcatccgac ccatcccgcc gcctctccgc agtggcgcaa gcgccccaaa 2820
   atctccggag agggaactga ctgacccact aggttccgcc gtgtctacct ctcgcagatg 2880
   ttggggaagt gcttcccggc gtctaatcct cgctgttccc ccctccaccg gcgcccagca 2940
   cacccgcggc gctccgctcc cgggtaccca cagctttctg gagtggcgcc ggcggcagga 3000
   gagagctaga gccgactgag ccccagaact cggggaggta ggcggggcaa gggccggggt 3060
   ctgcgcgtgc gtgtgcacgc gtggaaagca caaacccacg agggcccgct gccggtaggg 3120
   ttaaggtgag tgagtgaagg cggcagaccc gcgaagttcc agttccgcgg acactccttc 3180
   cctaagccac atagggactt aagagaaact gaggcacgaa gttggggggt ggcggtggtg 3240
   gggactttga aggtcgagtc gataaagccc agctctgtgc ttccgccccc tcccacacac 3300
   tcgagacccc cacaggttcc ttcttagggg tcctcgctct gctccgcagc ccctcctggg 3360
   gatccgggct ctgcggtcca gcgcgacctg cctggggcca cgtgttcaag cacgaagccc 3420
   ctgcgtggag tccacgccct tcaaaaggtc cctaggggca aagggatgaa gcccaggagc 3480
   gtcaatgtga ggtcaggtcc agcgggttag ggttacgagg tcagggtcga agtctcagaa 3540
   ttgactcggg agtatgacca ataagcccaa gagatttgtg gaggccacgc ccagaccatg 3600
   cctaccctag cctttctcga gctccgcccg tttctccaag actgggcccc tccagcctga 3660
   agccccaccc ccaggacatc cagctccgct ccttcccttc cccagtcccg cagccaagct 3720
   caactccagg gtgggagatt aaccaggtgg agacctggga tttcgctctg ggaccccctg 3780
   gtgacaaccc tcacccccgt gccgcctgtc tcctttcttc cccaggcccg cccagagctg 3840
   agctccgtcc tccggctgct gcccaaatca ggggtcgtgg acaaaggatg cctggggcct 3900
   gcggccctac gccaggaccc cgcgccgaat actctgattc ttcgggctcc ctccaaggga 3960
   gtcccaaaga ccccaatggc caataggaaa gtgggttcgg tctgggcagc agtctgattg 4020
   gctccagcct tcgggagcgg acccaggggc aaggggaggg gagaggggcg gtcctgggtt 4080
   ttgggggtgg aatcggattc cagctgtggt tctctccctg cgctcccgcc cgcactgcca 4140
   cggcggacgg ccaatgggcg cgcggctcgg ggccggcggc gtccggcgat tggctgcggg 4200
   gctgtctggg ggcggggccg aggcttgaag ttgaagtgag ggatccagct gtggtgtgcg 4260
   cggggctcct cgccgccgct ttcgctcgct cgctccgcgt ctcggccgga ggaggaggct 4320
   gtggcgccgg cgacagctac ggcagcggca gccaccgcgg cggctgcggc ggcggcatct 4380
   ccgcctccac tcccgcccgg gactgccccc cactgtctcc ccgcccctcc cggacagtga 4440
   gcccgcggcg gggcggggga aggagccgcc cccaccccct ccaagcccac ccctaaagag 4500
   atccctcctc ccctcccccg ccgcctggcg cggagccggg acgatgctga ccccttagat 4560
   ccggctccag ctgcgccgcg ggaagagggg gcgcccctcc ccggaccccc gccctccgcc 4620
   gctgcccccc ttttcgttcg ccctctcggg gcggcttcgc cgaaggtagc gccgaatccg 4680
   gcaaccggag cctgggcgcg aagcgaagaa gccggaacaa agtgaggggg agccggccgg 4740
   ctggcccggg aagccccagg ggcgcagggg aagcgggact cgcgccgggc ggggtttccc 4800
   tgcgccccgg cgccccgcgg gcagcatgcc cctgcgggca gggggagctg ggctgaactg 4860
   gccctcccgg gggctcagct tgcgccctag agcccaccag atgtgccccc gccggggccc 4920
   ccgggttgcg tgaggacacc tcctctgagg ggcgccgctt gcccctctcc ggatcgcccg 4980
   gggccccggc tggccagagg atggacgagg aggaggatgg agcgggcgcc gaggagtcgg 5040
   gacagccccg gagcttcatg cggctcaacg acctgtcggg ggccgggggc ggccggggcc 5100
   ggggtcagca gaaaaggacc cgggcagcgc ggactccgag gcggaggggc tgccgtaccc 5160
   ggcgctggcc ccggtggttt tcttctactt gagccaggac agccgcccgc ggagctggtg 5220
   tctccgcacg gtctgtaacc catatccttc ggggcacgac ggccaggcgc ggggtcagaa 5280
   gggggacggg ccgcaccgcc gggggtcggg ggggaagaag acccaccgcc aggtgagtcg 5340
   aagtgagccc ggagggtagg cggatggggg gggggctgcc agggagggga gggggcacca 5400
   gagtgggagc ggagacgcga gcaggtctcg tcggtaaccc gggcttaccc cacctgcgta 5460
   cacacacctc agtcttcctg ggttgggggg gtggggatcc aggccaggag aagagagctg 5520
   tgccccgctg gctcgcagct ggacgccctc cagatgtggt caggggaggg tcgtcatcct 5580
   ccagatgtgg gaagcttcgg gagcctggga gctgtactct gcccgcgccg gttagcgagc 5640
   tgggtttggt ttccgagttt ggtggggggt gggtgggggc ggtggggagg aagctgcggg 5700
   gacggaggag gggggaccgc aatctcctgg gtttccctcc ttcccccgcc ccaaagtttg 5760
   cggcggattc tagggtctga tcccccaacc tggtccttga ggctgaggtt tttactgacc 5820
   tagaaggaga tgttgggggg cggggaccag gtcctggccc acctcacccc ccacctcgcg 5880
   ggttggaggc acaacaagga gattccggcg gcggctgatg tcaggggcgc agaatgagaa 5940
   caagatgtgg tggaggggag ctgtctgccc ccggagctgg gagtggagcc cctttccgct 6000
   agagcccagt gccgcgggtg cctcctaccc gatctccatt cgatgcaggg tgcttaagat 6060
   tgggttgggg agttttatgc tggatttttc ttgtggaggg aaagactgtg tcaagagctt 6120
   ttttagcaag tgggggatgg ggttaagctg gtccaggtta ctcctcccct ttcacaaata 6180
   aagagtgctc ctgcagacca ggtatggggg actgcaggcc cgcgaaagtc ttgaggggac 6240
   agtaatactg tcagtgtgtt cactaaggag gactcgggtc caccacccca gcgtagactc 6300
   cgcagatagg tttggggaat gcaagcccca cattccaggg agatggattt agcactgctc 6360
   cctgtctgcc caggctggag tggtagggga gaccccagcc cagtcagacc ctagctcaaa 6420
   gcgccacttg acagacgcct ggccccaagc tccctggcgg gtcctgccac cgggggaagg 6480
   aaaggtggag gggaggtgtt gctgccatct caggtctgga cagcttctct gggctccttc 6540
   cttccagctc agaggttctt atagacccag ggtgggagac cccaggtctc ccttgtagcc 6600
   cctgtgagcc tagcagggac tctgctttgg ggaagggggg tggaaggttg gtgccgccct 6660
   cactctctgc agctctgctg cctctagtgc gcaaagcagg gatgagcttg tggccccccc 6720
   acccccatcc tctacacctt gtgggtcttt aggaagctcc cctcatcaca tcctcttctc 6780
   atccactgcc ccttcttcct ttctctctga gctgcagaaa gagaggtgca ggcctcagag 6840
   gtctgtgttc ttgctgaata ggcacaatct tgaggtttga gcccccagcc ccaggcccct 6900
   ttctcccagg caggatggga gatggagaac tgatatggct ggaaaggatg ggttccgagc 6960
   aactctcctc tctgccccta atcatttacc ccttccacga atgggaaaaa gcatcaacct 7020
   ctatgtcctc cttctcaagg gtcccagggg aggtagagag agctcttctc tcccctctcc 7080
   ctgaaaacag acctctaggc tagcatctct tctcgcctgc cctatctcca ggtgctagtg 7140
   gagggtctgg aggcagactc cttcactgag tggtctggac gggggcaagt gttgaggcct 7200
   ggcatggggg aggagaggtg ggaggacagc actcaccttg ggagttgggg agcagcttcc 7260
   cccaggggag tggtagttgg gtgtgtggca tcagcattac taggtctgga gaagcctggg 7320
   ttggctgagg atgggttgag aacaattttt tggcagggga ctgcctaagc tgtgcccctc 7380
   accactgcca tcctacccaa gtaccaagag acacctgtat ttttgctgcc agaggggctc 7440
   tgaactgcct ccagggcctc cctctttctc tttgtggtat ctgtgactcc ccacaggctt 7500
   caggagggtc cctgtacgcc tctgagccgg agggacataa ggacactccc ctccaccccc 7560
   aactaactga agctagttag gcttttcttg gtacattctg cttcttggcc tctccttttt 7620
   aagggaccag gtttcctaga gaggagatct agacccaggt aggaagtgtg tgtgtgtgtg 7680
   tgtgtgtgtg tgtgtgtgtg tgtgtgtgtg tgtgttgcaa gggaggacag caaaaggctc 7740
   agaagtgcaa gctttggtgt ggagagcagg ggtactggca gacagtcacc cctgcctcag 7800
   atcagagggg agcatgctgg cctttccagt atgtgttcct gcaagtgtga gggagcgcgc 7860
   tcccctctcg gggaggagtt cctccgcatt ccctctctgg ctgttgtgtc acatctggaa 7920
   gtttgtgtag gagtttctcc aagctcagag cccagcacag ctttttctca tttggaaatc 7980
   acaggctggc atttgggatg ccgagagagg gggagtggga ggggggccag agggggaaga 8040
   ggggagcctg ccaggagtcg ggggaggaga ggggggggag gcctgaacgg caggagccct 8100
   aatggcttcc agaagtgagt gtgagtgagt gactgtgcgt cactctgtgt gtctgagtgt 8160
   gtccctctag aggggtgggg gcacaggagt ttctggagcc ttccttgcca cactgtccca 8220
   atgccccaag tctgcctatt tctcttacct agggagaatt ctccatttct gtcctcagag 8280
   gaaatggtgg ggggagtgtt cttcctgtct cttcatcaat tccctcctcc aatttaattt 8340
   ccaaacccta agctcaccag aggagattct ggggagaagg gctggggctg gagaattgaa 8400
   tgggattgag ggggggccag atcacccctt ggcatgcttt ctgccccagg cccactttcc 8460
   ttctgggtca cacacacatt ccttcccacc cgctctgctc caccgctctg ctgggggact 8520
   ccccatcacc cgtagtgcca gttggagcca ttcctgggat tacccccacc acacagcccc 8580
   ccccaccctc gcctgctccc gcccagcact caccccgccc tgcctgcctc agccaggatg 8640
   gcgacagaca caactgctca cacgcagaca tgcgcacgcg cgcgcacaca cacacgcaca 8700
   cacacacaca gagtcacaga cgtttgaatc agatttctgg cctatttccc cttccaaaga 8760
   ttccagcggg ccgtctctct tgtgtctctg tgtctcatgt ctctgtctct gggtggagcc 8820
   ggctgccttt ctgtgagctt cagttttcaa ttcagctgga agcctctctc cccctacacc 8880
   catgcactga agcgtggtga ggggggaacc acagggaaat aggcttgtgg ggtgaggggg 8940
   gggggttctg ctgggggaat ccccctttga gctttggagc agggcggagc ccctacatcc 9000
   catgttaccc taggggttgc cgaatccgat agacaccagg agcctctctg tgggatgggt 9060
   gaggcaagga cctatccccc tacctcctca tttactagaa ggccaggact cctgggtcca 9120
   ggcccatttt ctcctcaacg ttccttagtc tcacatttcc aggctcctag gtgggaatgg 9180
   ggccgctgga gggcctgctt gccttctcag cccatgccca gagcccctgt gcccctagag 9240
   gtgtcccagc cacccccctc ctctccctac tttcctcacc cttgcggctg ccaggctaag 9300
   caagggctct atctagataa agccccacac agaggactca tttgtggaga taacatgctc 9360
   tagttctctc tcccaggagg gaggaaagga ggagggatgg aagtggggtt cctctctctg 9420
   ccgtttcctt tttctctgct tttgatggtg ggttgtgggg agaagaaaga tcctcccctc 9480
   tcctaccccc tctttgggat ccactccagc ccggacagtc accatagcaa cagtagacat 9540
   gtgactgcac aggtctctca tagtcactat ggcaaccagt gtcccccgtt cccttggtta 9600
   ccatggggat ctgtcacatg gttttccccc aacctgataa cctgtgggtc tccctgattc 9660
   tcctcccctg cataaggaca agacaacagc ctcagtcagg ggagatcatg tgagaggggt 9720
   ctttttaatt accatgacaa caaatccaca ccacctggtg acacatcctc ctgtcgttat 9780
   ggcaacagaa gaacatcaca cagtgacata ctgccatgtt cccaaggaaa caagccctac 9840
   tctgagaagg gtgaaagacc ccccccccca ttaattattc ctctattcag agtgtcccaa 9900
   attgtcgtgt gccttggtct cttccctgtc cgtttatgct tacgtgtcgt ggaaggagag 9960
   ctgtacaggg tgggcaggat gttacttgga agcaggctga attgccaagg gacccttaag 10020
   aaggggctga catgatcaag agctgtgcat gaggttattc gggggcaggc tttgcctgta 10080
   agaggcctcc agtctgagcc tgggtaaggt ggggacagaa agaggggcaa ctcctctgtg 10140
   aggtcacttc tgtaccttcc cccagttata agcagttcac ctctgtcaag ggaaacaagg 10200
   caggaagacc tgaatgggga cacacatctg gcttctaggc agggagactt attgattggt 10260
   tgattcgttc attcattcat tcagtcttcc caggtattga tcatctgctc cgtgccaggc 10320
   actacagcaa gctccgtagg ggatctaaag acgtatcaga atgcccctgc ctgtcaggag 10380
   ccccagctca gcaggggagg tcgacaagtg cacagattac taacacggtg gaactctgcc 10440
   attagagggg tccaagcaga tgtcctggga gcccctcttc ctcagagatc tactgcctcc 10500
   acacagagac attcactcac tggggctcct tccagacccc cctttccctg cagaggagag 10560
   gaggggacct gcatggggaa agcagaccag ttcttgaggg taactgtgtc tttccctttc 10620
   caaatgatgg ctcccttcct gtcccccttt ttccagggtg acccctggct gaaccccatt 10680
   ctggggagag atccactgca ctccggctct actcagaaaa acaattccat ggaaacagag 10740
   cactttttat ttcctccctg tcatctgcct acccccaaaa acctagaact tttttcttaa 10800
   accctcttat tcttgtgagg gggtgggggt tagggaaact gcagtgcatt aggactctcg 10860
   gggtaccacg gacaggtggc gagcaggcat gtgagtctag gggagaaggg cctgggtggt 10920
   ggagaagaga caggggaggg ctgtcagggg tttgagggat tcatgggcta tgggggaggg 10980
   catgtggctt gggagtcaga acagagatgg gctatggctg ggaccagctg ggaggaaggt 11040
   ccataggttc ctgaacattc ctgctggcct cttcatgcct ggccaaagcc ttctcattcc 11100
   agaagcaaga ttccacctgc tctgggccct acagagtctg gcttggtggg tcatgccagg 11160
   agctgccaca atacctcccc catccccatt ccccaggggg tctctggtct cctttcccca 11220
   atctcctgct ctctgatctt ttctagcctc tttactcttc cccacccttc tcatattttc 11280
   cttaaagaaa ataggatact tggtcattta accgtgagtc cagtgcctgg aaccttctaa 11340
   ggtccggagg ccgttttgta cattctcctg ctgatgggct ggaaatgagt cactccatag 11400
   aatccactgt atggaaagac ccagggttta gaaagaaccc ctctctcctg cttgctgttt 11460
   tggctggtct gggctggaag ccacaagcag gtgtgatggc ggcaatggcc tcttggggga 11520
   gggggtagac attgtccact ctgcacggct tgggagctat gcccagcatg gtgcctgaca 11580
   cataatgggt cctcaatatt taaatgtgtg aatatgccta gtggtcattt gagggtgttg 11640
   agaggttggg aggctggtgg tggaagaggg agccccgggc tctggtgctg tagcaggggg 11700
   gactgcagct gtggacgtct gccttacacc tagtgccagg gcggtcatga caggcagttc 11760
   tgcctgcctg agcagctatg gtgtgcagag gaaactatca tatatcagga gcttggaaac 11820
   caccgtctca tcctgccttc tcgggtgacc ttcagcacat gacttcggca tttctgggcc 11880
   tcaacttcct cgagttctgg ggaagaaaat gatctacccc agatatttca cagggctgtt 11940
   gtgagcatca actgtggcat tgtgtgaaag tgcttggtaa actgtaaggc aggcataatc 12000
   cgactttagg tttgtataac acattcacat acagtatctc atttgcttct cacatctacc 12060
   ctgtgaggta gggagaatag gattcagatt ctgcagcaaa ctcatggtgt ctggcatttc 12120
   cacaggcatg atcgatttat tccccaaatg gcttgtgcaa atagtcaggg tatttgtggc 12180
   ttgtcttcct ttacagatga ggaagctggg tccagagagg tgagatgacc aaggtgggac 12240
   tccccttctc tgatgaggag tctggggctg ggggctggtc tgcgtgtatg tcagggccct 12300
   ggcaccacac ctgcttagcc tcagatggag ccaggaggta aacgaggagg aggtgttagg 12360
   gcggggtcgg ggggccggcc tcagctccag ccttggccag ctgtttcctt gactgccagt 12420
   acc 12423

<210> 4
<211> 3720
<212> DNA
<213> Homo Sapiens

<400> 4

tgcagttgtc tatcaaaatt ttacatatac tttggcacac aattacactc gttggaattt 60
atctcacaga cacattcaca tttatatagt caaggatgtt gactccaaca acattggtat 120
aagtaaaaag ttggaaactg cctacatgct catggatagg ggataaatcg actaactcaa 180
ggtcctccct tacaatggag tactatacag ccttgaaaaa gaatgacaag atctttatgt 240
caggacatgg cacaatctct aagttatttc aagtgaaaaa ataataataa taataatatt 300
gcagaacaat gtgtatggtt ggatatacat aaattcttct gaaagaatac ccaagaaact 360
taaatgtggc tacctcagga gctaggaatg aggtaggtgg agaggaggga cttttacttt 420
tcaatttaga atataccctg ttgtgctagt tgactttcct cctactgtta acaagtatta 480
cagtcctttt taatttaaat tatttttcct agcacaaatt taaaaaaaaa aaacttaaaa 540
tggaaaaaca cacaggcgtg agcaaaagaa atgaatggca ataatctctg tggtttactg 600
agtgcgcctg gacatttatg tgctctcagt ttctctccac aggaaatgca caggtgagaa 660
actgacgtta agggggactg agtgtcaagc tagttagtgg cagagggcag attcaaaccc 720
aacacggtcc tcccctgctg cccctcggcc tctgcctcca ggtgggaagc gcatctaccg 780
gacggtcggc ccggtgaggc gcagcgcccc agactggcgc atccgcggcc ccagcgctcc 840
acgcctgggg agcgcgcgcg cacgcagcgg cgcgagcctg gcggcggcgg cgacaacaac 900
aacgtcacag ctcgagcttt ccttttcgga gtccccggca cacatcctgt gtccatgttt 960
gggcatttac gtcacggcgg cagggccggg gcctcccaaa atggcagtgg cccggggagt 1020
cggaagcccg gagccagcgc cgccgcagct atataagtgg gggggctgtg ggctggggga 1080
gcccggcagc gctttggaga ggcgaggagc cgccgcccga ggccggtgcg ggcgagcgag 1140
ggcgccgcgg ctccccgact cctttcccag aggtgagtgc ccgaagccag gagcccggcg 1200
cccataggtc tgtgcgctgc ggggaacccc taccgccagc ctccccgcca cccgcgcgcc 1260

cccaagccca gcgggcgagg ccccgggcgc cccacagccg gcgccgcgcc atgctccacc 1320
ttagcgagtt ttccgaaccc gacgcgctcc tcgtcaagtc cactgaaggc tgttgcgccg 1380
aacccagcgc tgaattgccc cggctgcctg ccagggacgc tcccgcggcc accggctacc 1440
ctggaggtaa ggagggcgag caggggtgct cagacgacga cggcgcagcg cgggggcgca 1500
ccatacctga gaaccaggag ggactgggac attggagcta tgagaatagg ggcgatggga 1560
agcttaggag tcctggggtg cgcaccccca ttcccaccca cactgggccg ccagcgcctc 1620
cgcaggaacc tgtgcggtta tcggagcgcc cttttgcctg tgcacgtgtg ttttgcgtgt 1680
gcatgtttct atgtgctcct tgggacgtat gcgggcccct gctgaatcag aatgtgcaaa 1740
aggcactttg tgtatatccg tgggcaccaa gagttttgta ggtaggggct gtggactcag 1800
gtgcaccctt tgatgtgccc agagctgatt cctgctccct cctcagcagg cgacttcttg 1860
agctgggctt tgaacagctg cggcgcaagt ggggacttag ccgactcctg cttcctggag 1920
gggcctgcgc ccacaccccc tcccggcctc agctacagcg gtagcttctt cattcaggca 1980
gtgcccgaac acccgcacga cccggaggca ctcttcaacc tcatgtcggg cgtcttaggc 2040
ctggcaccct tccccggtcc agaggcagca gcgtccagat ccccgctgga tgcccctttt 2100
cctgcggggt ccgatgcctt gctgccgggt ccgccggacc tttactcccc ggatctgggc 2160
gctgcccctt tcccagaggc gttctgggag gcctcgcctt gcgcgggtgc cccctcgcag 2220
tgcctgtatg agcctcagct ctccccgccc gacgtcaagc ccggcctccg gcggcctccc 2280
gcctcgccag cgctggacgc tgtctctgcc ttcaagggtc cctacgcgcc ctgggagctg 2340
ctttctgtgg gggccccagg gaactgtggg tcacagggag actaccaggc cgccccggag 2400
gctcgttttc ccgtaatagg gaccaagatt gaggacttgc tgtccatcag ctgccctgcg 2460
gaactgccgg ccgtcccagc caacagactc tatcccagcg gggcctatga cgctttcccg 2520
ctggccccgg gtgacttagg ggagggggct gagggcctcc ctgggctcct gacccctcct 2580
agtggggagg gagggagtag cggcgacggc ggagagtttc tggccagtac gcagcctcag 2640
ctttccccgc tgggccttcg cagcgccgcc gcggcggact tccctaaacc tctggtggcg 2700
gacatccctg gaagcagtgg cgtggctgca ccacccgtgc cgccgccgcc gcccacccct 2760
ttcccccagg ccaaggcgcg acgcaagggg cgccgcggcg gcaaatgcag cacgcgctgc 2820
ttctgcccgc ggccgcacgc caaggccttc gcttgcccgg tggagagttg tgtgcggagc 2880
tttgcgcgct ccgacgagct caatcgccac ctgcgcatcc acacgggcca caaacccttc 2940
cagtgccgca tctgcctccg caacttcagc cgcagcgacc acctcaccag ccacgtgcgc 3000
acccacaccg gcgagaagcc ttttgcttgc gacgtgtgcg gccgccgctt cgcgcgcagc 3060
gatgagaaga aacggcacag caaggtgcac ctcaagcaga aggcgcgcgc cgaggagcgg 3120
ctcaagggcc tcggctttta ctcgctgggc ctctccttcg cttctctctg agcaagagat 3180
gggtttatgg gttgggcgcc cgccgttcgg cgcgcacgag ttccgggccg ttcccctccc 3240
cgctcttctt ccaactcctc ctcgcacgcc cgagggccgg cctccgtccc gcttccagtt 3300
tccttgaagc gcccgccgca cacgccctat tcagcaccag ctcgccggac agttcccgcg 3360
gtccaggcgc tgtcaccctt gtcagccgcg ctttggggga agtcttctga gaccacccag 3420
tgaataggca ctaccctggg attcaagaca gtcttttgta actgcacacg ccccacgcct 3480
tcctctataa cccccagaga caggctgggg caggccaagg cggtctcgcg cgggactttg 3540
tacagcagtg tcttatccag cagccgattg gatgtaacgt tttgctttgg gttttttttc 3600
cttttgttgt tgttaatttt tgtaaagcag acgctactct caagcagttg acaaaactgt 3660
ttatttttgc aattaaaatt attgtgctaa aagcttactg aatctgccat gtaagctcct 3720

<210> 5
<211> 8759
<212> DNA
<213> Homo Sapiens

<400> 5

tagcctggaa gagggcagcc aggggagaag ttagggctgg agctatgaga aaggataaga 60
tgagatgatg gcttcaacat tgaggacaga aagaatattg agatgagaaa gtagtccatt 120
ataagcatct atgcaaagga aatagcagat gtccacaaat cagcagaggc aacaactctg 180
aaagtttatt cataagcccc tcttttcatc tccaatccag ttcaaatgta attatttaaa 240
ttgttcttca ctctccttcc tggatcatga atgagctcct taaatgcagg gtccacagtg 300
tcctattcat cagtgaattc caagtgccta gcacagagcc tggcaaatag taaatgctta 360
acaaatattc gttcagtgca tgaattggag tgattctcta ctttgctcat aagttgaaaa 420
aaggtttatt acatacctaa atatgctgaa atcacagggc atttggcaac cccccaaaac 480
caaaactccc agtttggaaa cagaatttta attctgtgaa aataaaatcc attcatttat 540
tcaaaaaata tttattaaac aatgaccatg tcccacacca ggctgagtcc taaggattca 600
atgatgaaca aaaaccaaca tgattcctgc tcttaggaaa catacagttc agtgaggaaa 660
acagattgtg agaagtcctc caacaaatac tgggtgctat taaaatatat taaaaggtga 720
gtgggtgagg gacttgagct agcctaggtg gttcaggaag tcttcctgga tgtgctgata 780
tgcataggca ttaactagat aaatagagag aaggatgaac caacattgca ggtagaggga 840

acagaatatg caaaggcagg aaggattatg gagtcgttgg aggacctgaa taaaggccag 900
tgtaagtgga tctcagaaaa caggaggaaa ggtgtatgag atgagatcag agaggcagat 960
catgtggggt atggttaatg ttttggactt ttctattaag agcaatgggg agacagtgac 1020
aggacttaaa cggggaaata atatgaccag attaaacttt ctaaaaaacc ctctatgcaa 1080
atatatattg agagttaatt attgacaaag attcaaaggc aacaaagtgg agagagaata 1140
gtattttcaa aaaatggtgc caaaacaata ggacatctat attaaaagtt gggtatctgt 1200
ctacaaaact taattcaaaa tggatcacag acctaaatgt aaaactgaaa gctatacaac 1260
ttctggaaga aaacacagat gggaatctgt gtgatcttga gtttgaaaat gatttattat 1320
atctgacacc ataatccgta agttaacata attcataagt gaacaaagtg atgaactgga 1380
cttcatcaga atttaaaatg tttgtgcttc aaaagacact ggtatgataa tgaagacaaa 1440
ctacagataa gatattgttg aatcatattt ctgataaagg aattgtgctc agaatacata 1500
actctaaaac ccccataata aattacaagt agcccaatta aaaaaaaaaa aagagaaaaa 1560
atttacagtc ttcatcaaag aaagtataca attgtaaaat aagcacatga aaaatgctct 1620
gcatctttat tcatgggaga aataaaaatt aaatgggaaa gacacctcta attagaatac 1680
taaaattaaa aagactgacc ataccaagta ttggtgaagt ggaaatgtaa aatgatacaa 1740
tcacttagga agatgatttg gaagtttctt acaaaagtag gtgtatacct accctgtgac 1800
tcacccattc catggctaag tatttacctg agagaaatga aagaatacat ccatacaaag 1860
atgtttatac aaatatttat agcagtttta tttgtagtag ccccaaactg aaaagaaccc 1920
aaatgtccat caaaagtgaa tggataaaca aagcgtggta cagcaatgca atagaatact 1980
acttagcaat aaagaagaat gagctagtga tatacataac agcttaaatg tacatcaaag 2040
gcattgtgct cagtgaaaga tgcaagtaaa aaaaaaaaag agtacatgct gtatagttcc 2100
attgacataa aactctggaa agtgaaaaac agtctatact gacagaaagc agatcattgg 2160
ttgcctgagg aggaggagta taggagaggt ggagggaaaa tgtacaaagt ggcacaataa 2220
aaacttttgg aatcatagat atattcacta tcttgattga gtgatgattt catgagtgca 2280
cgcgtgtgtc aaaaatgatc aatttatgca actttaaata tgtgcagttt attgtatata 2340
tcaattatac ctcagtacgg ctattaaaaa gaaaccctct ggctgcacaa tgcagaactg 2400
attctaggaa agagtggagg gaggatgacc atttacagtg ctccaggtgg aagagaacgg 2460
tgccttctgg aagtgaacta ggttggcaac aacagagatg aaataaatgg gcagatgtgt 2520
gagatactta ggaaataaaa cccgatggtc accattttcc aaaggtcagc tcatcctggc 2580
tttccagagc aaagagctag ggaagacttt attaataaat ccctcttgaa gttgcagagg 2640
aagcttatag cagaaactta ctctcaacct gactaatctg agagaacacc tctggttcca 2700
tttgattact aaaaaactgc aaagaacagg aggagaaaga agaagaaagc tggtacaaac 2760
agtgaactta tataatatta atcaataatt gtctcttgtt cttaaaagca atgggaagaa 2820
aatgagattt gagctggaag atcagagttc aaaatccaaa taaagtatat ggccctaata 2880
tgcttatagt agttaacctt tcctgataat gatataattg ttgacagcac catctttaaa 2940
aataaaaata acatagtaat ccttcagatt tgtagaatgc tttcctgttt acaagtttgt 3000
tctatacaca ttatgtcttt taaatgacac actagccttc tgagggtaac ttatattggc 3060
aacagttttc agatgtggaa actgtgaaga caatgttggt gatgtggaag caacataaac 3120
tttggagtct ttcagaccca ggtttgaatg tcagactgct ttttattcag agtaacttca 3180
gagcattatt tctcacctta attttttttc aggcctcttt gtgtctatgt gtcctcttca 3240
ctcctgtcca ttgttcattc agtgattttt gcaccttcct tcactgttag tgtgtagaca 3300
catagttctc ctggctctga gacctatgtt aattccattc taccatcctg ccagcccact 3360
caattcctat tgagcaatgc tagttgaaag ttgtggtggg attaaatgtt gcaatgagta 3420
ttcaaatgag gttgaagtat ctacgcattc tacttacata tggtgaggta tattcaagga 3480
aggctgtagc cattaaaatc tcaggaaata atttttcacc tcctcaggtg aaagggtctt 3540
caggcctttg tgttctggaa ggttcattta tagccatttc ccaaatgaca atgcgattga 3600
tgagtctaga gtctagctca aatagcaatg gactggaaga ctagtttagg ttttactaat 3660
gtggaacata gaacaaatta tgtccttgtt tcagcctgtt catctgtgaa atagagccta 3720
tcatatccag tcttccttgc ctttaggttt gagttacctt ctttggtcaa ggtaagtaaa 3780
tgcctatgat gtttggctgt gcacaagata aagctacaac aaagctacaa cccatctttt 3840
ctctgtagaa gactgcaaaa agcaaaagag acccaggcaa aaatctcgga atgacttttg 3900
gaacagagag cctccccaga atcagaagtc aaaggaattt aaaacatagg gaggcccagg 3960
gtctctactg acataaagga aagatgtttt ccttataggt ttacgtttac attttctctc 4020
tctttccatt cccacttgca tctccacctt tacacagggc ttatgggacc tcctccacaa 4080
aagagcagtt gcagtaaccc acatcatcct ctacgcctgg ctgtccatca agaggcgaaa 4140
agcagcccta tataggttct atccttggat agttccagtt gtaaagttta aatatgcgaa 4200
aggcaacttg gaaaagcaag cggctgcata caaagcaaac gtttacagag ctctggacaa 4260
aattgagcgc ctatgtgtac atggcaagtg tttttagtgt ttgtgtgttt acctgcttgt 4320
ctgggtgatt ttgcctttga gagtctggat gagaaatgca tggttaaagg caattccaga 4380
caggaagaaa ggcagagaag agggtagaaa tgacctctga ttcttggggc tgagggttcc 4440
tagagcaaat ggcacaatgc cacgaggccc gatctatccc tatgacggaa tctaaggttt 4500
cagcaagtat ctgctggctt ggtcatggct tgctcctcag tttgtaggag actctcccac 4560
tctcccatct gcgcgctctt atcagtcctg aaaagaaccc ctggcagcca ggagcaggta 4620

ttcctatcgt ccttttcctc cctccctcgc ctccaccctg ttggtttttt agattgggct 4680
ttggaaccaa atttggtgag tgctggcctc caggaaatct ggagccctgg cgcctaaacc 4740
ttggtttagg aaagcaggag ctattcagga agcaggggtc ctccagggct agagctagcc 4800
tctcctgccc tcgcccacgc tgcgccagca cttgtttctc caaagccact aggcaggcgt 4860
tagcgcgcgg tgaggggagg ggagaaaagg aaaggggagg ggagggaaaa ggaggtggga 4920
aggcaaggag gccggcccgg tggggcgggg acccgactcg caaactgttg catttgctct 4980
ccacctccca gcgccccctc cgagatcccg gggagccagc ttgctgggag agcgggacgg 5040
tccggagcaa gcccagaggc agaggaggcg acagagggaa aaagggccga gctagccgct 5100
ccagtgctgt acaggagccg aagggacgca ccacgccagc cccagcccgg ctccagcgac 5160
agccaacgcc tcttgcagcg cggcggcttc gaagccgccg cccggagctg ccctttcctc 5220
ttcggtgaag tttttaaaag ctgctaaaga ctcggaggaa gcaaggaaag tgcctggtag 5280
gactgacggc tgcctttgtc ctcctcctct ccaccccgcc tccccccacc ctgccttccc 5340
cccctccccc gtcttctctc ccgcagctgc ctcagtcggc tactctcagc caacccccct 5400
caccaccctt ctccccaccc gcccccccgc ccccgtcggc ccagcgctgc cagcccgagt 5460
ttgcagagag gtaactccct ttggctgcga gcgggcgagc tagctgcaca ttgcaaagaa 5520
ggctcttagg agccaggcga ctggggagcg gcttcagcac tgcagccacg acccgcctgg 5580
ttaggctgca cgcggagaga accctctgtt ttcccccact ctctctccac ctcctcctgc 5640
cttccccacc ccgagtgcgg agccagagat caaaagatga aaaggcagtc aggtcttcag 5700
tagccaaaaa acaaaacaaa caaaaacaaa aaagccgaaa taaaagaaaa agataataac 5760
tcagttctta tttgcaccta cttcagtgga cactgaattt ggaaggtgga ggattttgtt 5820
tttttctttt aagatctggg catcttttga atctaccctt caagtattaa gagacagact 5880
gtgagcctag cagggcagat cttgtccacc gtgtgtcttc ttctgcacga gactttgagg 5940
ctgtcagagc gctttttgcg tggttgctcc cgcaagtttc cttctctgga gcttcccgca 6000
ggtgggcagc tagctgcagc gactaccgca tcatcacagc ctgttgaact cttctgagca 6060
agagaagggg aggcggggta agggaagtag gtggaagatt cagccaagct caaggatgga 6120
agtgcagtta gggctgggaa gggtctaccc tcggccgccg tccaagacct accgaggagc 6180
tttccagaat ctgttccaga gcgtgcgcga agtgatccag aacccgggcc ccaggcaccc 6240
agaggccgcg agcgcagcac ctcccggcgc cagtttgctg ctgctgcagc agcagcagca 6300
gcagcagcag cagcagcagc agcagcagca gcagcagcag cagcagcagc agcaagagac 6360
tagccccagg cagcagcagc agcagcaggg tgaggatggt tctccccaag cccatcgtag 6420
aggccccaca ggctacctgg tcctggatga ggaacagcaa ccttcacagc cgcagtcggc 6480
cctggagtgc caccccgaga gaggttgcgt cccagagcct ggagccgccg tggccgccag 6540
caaggggctg ccgcagcagc tgccagcacc tccggacgag gatgactcag ctgccccatc 6600
cacgttgtcc ctgctgggcc ccactttccc cggcttaagc agctgctccg ctgaccttaa 6660
agacatcctg agcgaggcca gcaccatgca actccttcag caacagcagc aggaagcagt 6720
atccgaaggc agcagcagcg ggagagcgag ggaggcctcg gggctcccca cttcctccaa 6780
ggacaattac ttagggggca cttcgaccat ttctgacaac gccaaggagt tgtgtaaggc 6840
agtgtcggtg tccatgggcc tgggtgtgga ggcgttggag catctgagtc caggggaaca 6900
gcttcggggg gattgcatgt acgccccact tttgggagtt ccacccgctg tgcgtcccac 6960
tccttgtgcc ccattggccg aatgcaaagg ttctctgcta gacgacagcg caggcaagag 7020
cactgaagat actgctgagt attccccttt caagggaggt tacaccaaag ggctagaagg 7080
cgagagccta ggctgctctg gcagcgctgc agcagggagc tccgggacac ttgaactgcc 7140
gtctaccctg tctctctaca agtccggagc actggacgag gcagctgcgt accagagtcg 7200
cgactactac aactttccac tggctctggc cggaccgccg ccccctccgc cgcctcccca 7260
tccccacgct cgcatcaagc tggagaaccc gctggactac ggcagcgcct gggcggctgc 7320
ggcggcgcag tgccgctatg gggacctggc gagcctgcat ggcgcgggtg cagcgggacc 7380
cggttctggg tcaccctcag ccgccgcttc ctcatcctgg cacactctct tcacagccga 7440
agaaggccag ttgtatggac cgtgtggtgg tggtgggggt ggtggcggcg gcggcggcgg 7500
cggcggcggc ggcggcggcg gcggcggcgg cggcgaggcg ggagctgtag ccccctacgg 7560
ctacactcgg ccccctcagg ggctggcggg ccaggaaagc gacttcaccg cacctgatgt 7620
gtggtaccct ggcggcatgg tgagcagagt gccctatccc agtcccactt gtgtcaaaag 7680
cgaaatgggc ccctggatgg atagctactc cggaccttac ggggacatgc ggtaagtttt 7740
tccttccaga aatgtcgcct ttcggcccag ggcagagtca ctctgtgttc tggggtatct 7800
agcggctcct acctgcgcga acactcagat tgcccctggg agagctcagc agggtaaacc 7860
tagagctctc ccgtggactc ccggcctgcc agaggtttaa cctgagctct cctaatttct 7920
gctgcgtgcc ctgggtgctg attcctgccc tcccagattc ttcaactccc ccaaccgccc 7980
caaattctca ctacctcctg gtactcgagg tcccaaacag aaatcctatt gcacgggcca 8040
ccttcagaga taaagctccc aagccctcca ctcttccttt cctcctgtcc tcaaagtctg 8100
agaacctcaa caggaatttg ggcaatttct cctcttcagg tctgttagga tttcactttc 8160
agcctgcgca gattagagtc aaaaagaccg gcccaatagc ttctcagcgg gtatcctcca 8220
gagaggtaaa gtgaaattct cggttaggga aagaaagtgg tctctgggtg ctgaggtctg 8280
ctgtgtgaaa gggtgaactt ctttctcctg aagcaactgg ggacttgctc cagggctgga 8340
ggtcagtaga gataatccaa accgtcatgt ttagagtagg cagaggggca actttcttgg 8400

taaagacttc acaggatttg cactcacagt ttctcaacgt tggttgacta tgttgaaagt 8460
agttgcttgg gtcggttttc tcttgtaaag tgtttatttt ctctgtggat tataacagat 8520
ccacagcccc ctacttcagg tttgcatcag atctataaag aggagaatat tcttttaatg 8580
tacaatttaa ttaggcttga ctctgactta caaaactgtt ggaaacattt tttttgtaaa 8640
gcatttcctg ctatttcagt gtgctccaaa atctccactg gggagggtgg agtgaggttt 8700
tttattatat tcctttattt ttaggacatg tttgcatttt agaatatgtg cagttagct 8759

<210> 6
<211> 8404
<212> DNA
<213> Homo Sapiens

<400> 6

aggaagctaa aaaccttgaa aaaaggttag aggaattgct acctaaaata agcagtttag 60
agaagaacat aaatgacctg atggagctga aaaacacagc acaagaactt cgtgaagcat 120
acacaagtat caacagccaa agcagaagaa aggatatcag agattgaaga tcacctttat 180
gaaataaagt gtgaagacaa gattagagga aaaagaatga aaagaaacga acaaagcctc 240
caagaaatag gggactatgt gaaaagacca aacctatgtt tgattgtccc tgtacctgaa 300
agtgacaggg agaattgaac caagttggaa aacactcttc aggatattat ccaggagaat 360
ttccccaacc tagcacgaca ggccaacatt ccaattcagg aaatacagag aacaccacaa 420
agatactcct caagaagagc aaccccaaga cacataatcg tcagattcac caaggttgaa 480
atgaagccaa aaatgttaaa agcagccaga gagaaagtcg ggttacctac aaagggaagc 540
ccatcagact aatagtggat ctctctgcaa aaaccctaca ggccataaaa gatgggggcc 600
aatattcaac attcttaaag aaaggaattt tcaacccaga attttgtatc cagccaaact 660
aagctttata agtgaatgat aaataaaatc cttcacagat aagcaaatgt tgagagattt 720
tgtcaccacc aggcctgcat tacaagagct cctgaaggaa gcactaaata tggaaaggaa 780
aaattggtcc cagccactgc aaaaacatac caaattgtaa agaccattga cactatgaag 840
aaactaatgg gcatcaacta atgggcaaac taactggcta gcatcataat gacaggatca 900
gattcacata taacaatatt aaccttaaaa gtaaacgggc taagtgccac aattaaaaga 960
cacagactgg caagttggat aaagagtcaa gacccatcag tgtgctgtat tcagcagact 1020
catctcacat gcaaagacac gtgctctaaa taagggaatg gaggaagatt taccacagca 1080
atggaaagca aaaaaaaaaa aaagcagggg atgcaatcct agtctctgat aaaacagact 1140
ttaaaccaac gtagatcaga aaaagacaag aagggcatta catgatggta tagggatcaa 1200
tgcaacatga agagctacct atcctaagta tgtatgcacc caatacagga gctaccagat 1260
tcataaagcg agtccttgga gaggaagaaa gagacttaga ctcccacgca ataatagtgg 1320
gagactttaa caccccgctg tcaatatttg acagatcaat gagacagagt aattaacaag 1380
gataatcagg acttcaagtc agttttgcac cattcagact aaatagacat attcaatata 1440
tatatatata tatatatata tatatatatt ttttgagatg gggtcttgct cttgtcgcat 1500
aggctgaagt gcagtggcat gatctcagct cactgcaacc tctgcctcct gggttcaagt 1560
gattctcctg cctcagcctc cagagtagct gggattacag gcacctgcca ccacactcag 1620
ctaatttcgt atttttggta gagatgtggt ttcaccatgt ttgccagcct ggtctcaaac 1680
tcctgacctc aggtgatcca cccgccttgg cctcccaaac tgctgggatt acaggcgtga 1740
gccaccgtac ctggtctaga ctctgccgaa attctaatac ttctttggta atgcaacaga 1800
aaactaaaga ttttgatggt gaactcaatt gcttctgagt tgtgtggatg aaaatatatt 1860
ttattatttt ttccttcaac ttttaagctc ccaggtatat gtgtaggatg tgcaggtttg 1920
ttacacaggt aaatgtgtgc tatggtggtg tgctgcacag atcaacccat caccatggta 1980
ttaagcccag catccattag ctctcctgat gctctccctc tccccaccct tccccgctac 2040
aggccccagt gtgtgttgtt ccccttcctg ggtctatttt atggtatgta tgtgtgtatg 2100
tgtgtgtgtg tgtgtgtgtg tgtgtgtgtg tgtgtatcta tttatgtgta tatattatga 2160
tatatacacc acactttttt caaactatta ttttaggttt agggatacat gtgcaagttt 2220
gttatatagg taaactgtgt gtctctgggg tttggtgtgt acagattttt tcaccaccca 2280
ggtaacaagt gtagtacctg ataggtagtt ttttgatctt ctgtcctctc accctacacc 2340
ctcaagtaat cccccagtgt gtgttgttcc cctcttcatg tccatgtgtt ctcattgttt 2400
agctcccact tataagtgag aacatgcagt gtttggtttt ctgttcctgc gttagtttgc 2460
tgaggataaa ggcttctggc tccatccatg ttcctgcaaa ggacatgatc tcattccttt 2520
ttatggctgc aggaaaatat attttacata ctagtgaaca tagttaaatt aatccatggt 2580
caagacatat attcttttgt aaattgagaa atctctttat cataattgta atatggtaat 2640
aaataaatct agattacata aatgtaatct agatgttgat aaactggttt taaataaagc 2700
aagtgatatc tacgtatgaa gagagtttac ttcagttcct agaactttcc ttcactttcc 2760
tgccatccct acttttagta agcaagcatt tactgtgtgt gtaacactgt gctaggtact 2820
aaggcaatga tgatacccaa gatttggtct ctatcctgaa gataagcaca ggagaaacat 2880
tatcagtcat gatgctttct gttgcaaaca agtgaaaacc tcagctcagc ttaggtcagc 2940

tccagggctg gccaaatcag aggctcattt ataacactga ggatccagat tcttttgaaa 3000
ttcctcctgc accaatatca gcatttctac cttctctgta gggtggcttg cctcacgtta 3060
caatggctgc agcaatcaga agtgtcacat cctcagtaat acttaatatt attactatta 3120
tttaataata tttagaactg tgccatccct gtttcaattt atcaggctcc cagcagacta 3180
ctccttatct ttcaaatgtc aaaactgcat cctgagctct tgcctaaact aatctggggt 3240
gaggtgaatg gaagtagcac tttaattgta ttcattcttt gtagctggac ctgggcctgg 3300
gctatctcct gacatttgcc cacaagaaag atttctgaag ttaggtagga atggctgttg 3360
agtaggccag tgcttgccaa acctttacac atcctcacat atgtcataat atgcagataa 3420
aaagataatc ccttatacaa cttgctggga taaactcagg aggcttacag catgacctgc 3480
ttgaaggttc ttcctgcctt agaccttgct cagctgctcc aggatgaggg gatttacatc 3540
acagcacaac tgtattttat tcacagcata aaccatctct ttccttctca gttgacgagt 3600
tcagatgggc aataacagtg tctgccaaag agaaaaaaaa atgtattcaa actagataat 3660
ctattggtac aaataccgag acacagaagt gataacagct ttaagccaat gtttgatggt 3720
ggtagtccca gcaagctctt ttctgatgtc tttgtgcctt tgcacatgct ccttctctgt 3780
cactgttttc ttcatcaaac ataatataat ggacaagtgg aatcaaatag aattgagttc 3840
aaattctctg ctacccatcg gccctggtat tggacaaatt aactcctctg agcctgtttc 3900
ctcatctgca acgtagacta gctaatacta cccattggaa agcgttgttt cttagctaat 3960
gcatgcaagg cttaaaacct agatgacggg ttgataggtg cagcaaacct ccatggcata 4020
cgtatgccta tgtaacaaac ctacacgttc tgcacttgta tcccggaact taaagtaaaa 4080
aaaaaaaaaa aaaaaaaaag aaagaaagaa aaagaaaaaa aaggctgttt ctggggatta 4140
aataagacaa ttatgtaagg tggccagcac agttcctggt acatagtaaa tgtcaggcct 4200
gcctgacaga cttctattca gcagctactg ctcccctgaa aatcttcctc agacgtttcc 4260
acggtgcttc ccgttcttac accactacaa tcctttatta cactactatc cgttcattcc 4320
ccacagctcc ctcccttcct ttccctaacc agtgatccca aaaggccagc aagtgtctaa 4380
cattttctat cttctaagtg actggtaaag ttccgcacct atcagcgctc caagtttgtt 4440
tttgttttgg ccgactttgc aaaacggatt gggcgggatg agaggtgggg ggcgccgcca 4500
aggagggaga gtggcgctcc cgccgagggt gcactagcca gatattccct gcggggcccg 4560
agagtcttcc ctatcagacc ccgggatagg gatgaggccc acagtcaccc accagactct 4620
ttgtatagcc ccgttaagtg caccccggcc tggagggggt ggttctgggt agaagcacgt 4680
ccgggccgcg ccggatgcct cctggaaggc gcctggaccc acgccaggtt tcccagttta 4740
attcctcatg acttagcgtc ccagcccgcg caccgaccag cgccccagtt ccccacagac 4800
gccggcgggc ccgggagcct cgcggacgtg acgccgcggg cggaagtgac gttttcccgc 4860
ggttggacgc ggcgctcagt tgccgggcgg gggagggcgc gtccggtttt tctcagggga 4920
cgttgaaatt atttttgtaa cgggagtcgg gagaggacgg ggcgtgcccc gacgtgcgcg 4980
cgcgtcgtcc tccccggcgc tcctccacag ctcgctggct cccgccgcgg aaaggcgtca 5040
tgccgcccaa aaccccccga aaaacggccg ccaccgccgc cgctgccgcc gcggaacccc 5100
cggcaccgcc gccgccgccc cctcctgagg aggacccaga gcaggacagc ggcccggagg 5160
acctgcctct cgtcaggtga gcgagcagag ccgccgtcgc ctcacgcggg aagggcgccc 5220
cgggtgtgcg tagggcgggc gcaaggcggc tcggcgggga cccgtcctcg ccaggggccg 5280
ggtcccggcg ggaggaggcg ccctccctgc cccccgccac ggcggagcgt ctgcagaatg 5340
gtgacaggat tctgggttct tgggcgaggg gtctcggctt caacttgaca ggtgtcgggc 5400
gggtggggct agggtcctga gcgaagtgac aggtgcagtt ccctcttgtg aggctcggag 5460
gcagagggtc gttgcgagcg tccatcagac gcaaaaaatg aaaaataaaa atacaaaaat 5520
ggtgtctgtg ggagagtttt tcaccggaga attggagtac tccggtggtc gtctgacttt 5580
ctgttttggt tcacgcgatg caacagttgg gaagtatttt cttccgggcg tgcactgcat 5640
ctgaagtcca tttgtgggag aggccgacca gaaagccttg gacaagaagc gcagggtcct 5700
gagtgtccat tgcccacagg atactcggct caggagcttt gcggcgtttc cttagaacaa 5760
taatgcatcg aggccttggg gactcaaagc catctgtagt gattgatgga gcgtaactct 5820
ttagaggaac tgaaacatgg gcaaaacttt catgagacat ttaccagaag tgcttgaaag 5880
tttctaaact tttttttttc ctgtttgatg aactcttctt gcgtgttagt cggcttcggc 5940
ttgtctcatt atttcttcca ttttgccttt tgactttgaa ccagcaagga tcttggtgtc 6000
ccctcttttt gcctttgttt ttggcacaaa attagtggtt ctgtgcgcaa atggaaattt 6060
tcgttttccc ttattaagtg gaatctaaat ttaagcaagt ccatacgaat gcactagatc 6120
ttgaagggaa gtatttattg tattacaaca tcttactttt cttgattttt ctactttatg 6180
gttaaatagc tatgattgaa agagtgtaat tgtcattatt gtcagcactg gttctacttt 6240
gagacaagtt cattgcagag ggaatgggac ttgtttctgt tttcactatt tttctctccc 6300
attcttgtct atcaccaaat cctttcaccc tcacccattt ctttccactc ggtatacact 6360
aacaattcac ggcagaaaag attgaagtgg gatttaggaa atggcccctg gaaggctatt 6420
aaaaatttat atatttaaat ggactgtctt ataggtcagt taaaaaccat attcgttaaa 6480
aaaccaaaat aacaacaaca aaaaattaaa aaccacgtct ggggcatgtt ctgggaaaag 6540
acatggcttt agtttctgat taaattctga tgtatccaat tcttgcaaat ttcccttggg 6600
aaaatgcagt aatggctact ctaaagaatt ccatgttatg cacacagctt tggaagcata 6660
ctctaatgta gggtaaccag gaatattgac gttgtggcca ctgcttggaa aaaagaggac 6720

tgtttctttc attttttaac tcattttata tattttaagt aatggaacta taaaaaattc 6780
tcttataagt caaaaccata caagatacag tctgttcttg attatccata cccacaaagg 6840
gtttcattaa catagacagt tgaactctat aaattaacca gcaagggtag aaccactgcc 6900
gtgcagtctg atactgagca tcttgcctgg agatggaatc aaggctcagt ccaactgcac 6960
ctccagtgac agattccatt cctgatttga gagtttagat cttatttctg ttgccttatc 7020
tctggcctaa gtaatgtagc tggaagggaa actgtcacta ggaacagcat accacattca 7080
ttgttgaaat aatcaaaggt taatcatgtt tcgagctggg tgtgatggcg cgtacctgta 7140
gtccatgcta ctcaggaggc tgaggcagga ggatcacatg agcttgggag ttggaggcca 7200
gcctgggcaa caacgcaaga ccccagtctt taaaaaaaca aaaaagctga ccaggcatgt 7260
tggctcacgc tgtaatgcca gcactttggg aggccaagat gggaagattg cttgaggcca 7320
ggagtttgag aggagcctgg gcaacaaaac aagaccctgt ctctaaaaca aattttaaaa 7380
aattagccag tggctgtggc acacctgtag gctcatctac tcaggaggct gaggtgggag 7440
gatccttgag cccaggagtt tgagactgca gtgagccatg atttcaccac tgcaccccag 7500
cctggatgac agagtgaaac cctatttcaa aacaagaaaa aaaaacaaaa actaattatg 7560
ttttgaagga ggaattggca gtctagaata gtggtcaagg gcatggatta tagagttaga 7620
ctgttgggct cacatagtgg tttacctctt accggctgag acctcaggta agttctttaa 7680
cttctccaat ttgacagaca tctgtaaatg tctactttta cagatgagaa tacttatcta 7740
aaagggctga tgtcgggatt cagtgaaacc atatgtttaa ggtactttct acaggatcat 7800
agtaaatact caaaaattat taattattct tcatactatt cttattagta gataacacat 7860
agaaattaga tgaatgaaaa tgaattagcc aacaagaatt tattaaatgc ttgtctttgt 7920
taaggggaaa ggacaaaaat aagcagtcgc atcatgtaag atgtgctagt aggtagagag 7980
atatatgaaa cattgataag agaagactac agtttaacaa agtcactggc gttgaattgt 8040
ataatattat ctatggtttt ttatcttttt gttgttatct ttatcctatt ttcccaaaca 8100
gctttagcta ttacatttac tttccttcac agaagtgttt tgctgctttg aagtatattt 8160
gacttaccat gcaagcaaat atttttcact gtgtggtatc cttattttgg aatgaccatg 8220
aaaaagataa tcatatgttt aaatttgaag tgtaatgttt ttctaagata aaataagatc 8280
ttaaagtatt taataatgtt ctttttcaca gtagtgttat gtgcaaacta ttgaaacaag 8340
tatgtactga atcaatttga tttataagta tatgccaatt atatgattat tttcatttgg 8400
tagg 8404

<210> 7
<211> 5312
<212> DNA
<213> Homo Sapiens

<400> 7

gtcggcctcg ggggtcgacg atcccccggg taggcgacgt gccctgtcca ggcctcactt 60
cccgcgtccg caaaacgggg tggacaacgc agcctaaggc agagccgcgc caaggtccct 120
cgctgtcgcc gggctctggc ggcctgaccg ggcctggggt ccgagcgtgc ccccgggcct 180
gggggggtcg ccgcgatgga ctcgctggca gcgccccagg accgcctggt ggagcagctg 240
ctgtcgccgc ggacccaggc ccagaggcgg ctcaaggtgc gtgtgtggag agggcggaac 300
gtgggtctgt gaccccgcgg gctccgggac tcggcatggg gtccccgtcc ccgcgcgcca 360
cggcccgcca gcgcctaggc tcagcccttc cctccgcagc ggcctcgcag gtccgcggcc 420
ccacggtggg gcgacgtgcc ctgtccaggc ctcacctcac gcgactccaa aacgcggcgg 480
agaatgcggc cgtgggaccc ctccaggggc cccaagggtc tgcgggggct gggcgcctgg 540
gcgcgcgggc gggggaggct cggcgccgcg gtctataaat aggcgctgct ccgccaccgc 600
cgctgccgcc gcccccgcgg ctggatccgg gccaggtggg gcctcccgcc ccctggcact 660
ctccggaggg gtgagtgtcg aggcccccag cttctccggc ttatggaggg ccttgagtgt 720
gtcccagggg tcccaatcct gctgccctct gcccgaggag ggaggacatc gcctgtgcct 780
gctgcggcct gacatctccc cacggaataa atacagtgtc ggtccctgca gagccctccc 840
tgggattggg gccaggggtg ccttctcctg ccaccacctc gcctgccttc ttatctccag 900
gcttttgccc acgggccctc cccttatttt tgccaacgaa atgccccacc ctgggaaccg 960
taccctctgc tgtctcctca taccgcattt ggtaagagac agggtattgg gcttctggag 1020
aaacctttgt gtccctaccc tgttccatcc tgtccccagg acattgacaa gcagtacgtg 1080
ggcttcgcca cactgcccaa ccaggtgcac cgcaagtcgg tgaagaaagg ctttgacttc 1140
acactcatgg tggctggtga gtgggccagg ctcctcgggg gagtggctgg ggtcactggc 1200
cagccaagct ctgtcgttgg agccccagac ctaacgcagc tccttctctg tacctgtgtg 1260
caggtgagtc aggcctgggg aagtccacac tggtccacag cctcttcctg acagacttgt 1320
acaaggaccg gaagctgctc agtgctgagg gtgagtggcc cccaggaggc cctggcactg 1380
atccccagtc cccttccatg ggacctctcc aaggactccc tttcaggtcc agctcccact 1440
gttctgttct cgcggtgtgg gtcccctggg ggtagggcca aggcaccaag atggatgagg 1500
acgagggtcc tggctgccaa gggtgagggg ctgaggttgg gagaggccct tccagtggcc 1560

ccttccccgt agagcgcatc agccagacgg tagagattct aaaacacacg gtggacattg 1620
aggagaaggg agtcaagctg aagctcacca tcgtggacac gccgggattc ggggacgctg 1680
tcaacaacac cgagtggtga gtgaggcctg ctgagaaagg ccttgcctag gcggccacag 1740
cactcgaggc ctggcctcac ctccctcctg cccacagctg gaagcccatc accgactatg 1800
tggaccagca gtttgagcag tacttccgtg atgagagcgg cctcaaccga aagaacatcc 1860
aagacaaccg agtgcactgc tgcctatact tcatctcccc cttcgggcat gggtgtgtgg 1920
ctgtcctggg gccaggctcg ggagtgcagc ccctacaata tggccccctg gctgtgccta 1980
tgcccaccct tggctgctct cggcaggctg cggccagtgg atgtgggttt catgaaggca 2040
ttgcatgaga aggtcaacat cgtgcctctc atcgccaaag ctgactgtct tgtccccagt 2100
gagatccgga agctgaagga gcgggtgagc ctgccgtcgc acaggggcct ggccagggcc 2160
ctggggctga gagtaccagg gggacttgtc tggcctcaaa tctgatggtc cttgccccac 2220
cacagatccg ggaggagatt gacaagtttg ggatccatgt ataccagttc cctgagtgtg 2280
actcggacga ggatgaggac ttcaagcagc aggaccggga actgaaggtg aacatgcaga 2340
ctggtggggc aggggggatg gagctggtga ggggcagaac cagaggcctt tgtctccttc 2400
acattgagcc tgctggagga gggccaggtc agcccagttg ggtgcaagag tcatttgttc 2460
taggagtaga ggacctgtac ccccttcatc cagggctaga aggtaaaggt caccatgtct 2520
ttgcctggct ggggtgggtc atgtgggccc cgtgtggtgc ttggcaggta tggagcactt 2580
gcccagatcc agaggtgcag cagtgggcca ggcccccaac cccaaccctc ttcccatggg 2640
tcaccagagg aaggggctgc cccagtggca caccctggct cccagatttc tgagctccag 2700
ggttggtggc tttgggtgcc ccggcagcta gagtgatgat ggagaccatg ccaggggcag 2760
gtggccacca gggcaggggc atcagcatgg ggagacatag gctcagccct ggggagggaa 2820
ggggcagctg gagggggtgg ttcttgatga gggaggagat gaggagggag catgcctggt 2880
tgggcatagc ctggaaaggc cctggaatgt gagccacatg ggctctggct ccagccaggg 2940
gcagagaggc cagaaagggg caacgccagg atctctttga ggagagatag ttgatggtga 3000
tgctggaggg gtgccccggg agttacatgc ccgtttccca tcagtaaccg tgcaattacg 3060
ctcaccgggt gtgagccttt ctccctcctt cccccaggag agcgcgccct tcgccgttat 3120
aggcagcaac acggtggtgg aggccaaggg gcagcgggtc cggggccgac tgtacccctg 3180
ggggatcgtg gagggtgagt agagtcttgg ggtaccaggt ctggtggggg aaggctgtcc 3240
tgggccggcg ccagcccact acccaccccc accccgcagt ggagaaccag gcgcattgcg 3300
acttcgtgaa gctgcgcaac atgctcatcc gcacgcatat gcacgacctc aaggacgtga 3360
cgtgcgacgt gcactacgag aactaccgcg cgcactgcat ccagcagatg accaggtgcg 3420
cgccccagcc gcgagccaga cctcgcccct ctggccccgc ccacgtctcc ataactgagg 3480
gccggtcctg tcagcccacc cagacttgaa ctttgcacca ttccctaagc cccccccctc 3540
ccccagagcc tggtctccct agaaccaagt ccagggctgt gagggctccg gagggcaggg 3600
cctcagcagt ggcggggatg ggccaggcat cgccagccca cgctgagcct cccggtggcg 3660
ccgccccgcc catcctcccc cccgccccgc gcagcaaact gacccaggac agccgcatgg 3720
agagccccat cccgatcctg ccgctgccca ccccggacgc cgagactgag aagcttatca 3780
ggatgaagga tgaggaagta tgtggggcgg cgggggcggc ggaggcgggc gtcagggatg 3840
ctcctccgcg gtgctgctca cccgccgggt tgtctccgcc cgcagctgag gcgcatgcag 3900
gagatgctgc agaggatgaa gcagcagatg caggaccagt gacgctcgcc gcggacacac 3960
cgtccgtctc cgggacgccc tcgcacccct ggacaccaga ccggactgtt cccgacccgg 4020
agacgcgggg ccacagcccc cagctgaccc taatttattc tcagcaccac cccctcccag 4080
gtcattgtgt ctgtttccga ggggcctgga ccgtagcccc cgcccagctg gccctctctg 4140
accttggggg atcaggagcg aagttgggcg ggacttcaga gatccgcctc ccttgccctt 4200
cccccgcccc cggacggtca cagcacccaa accgcaggcc ctgctctggc aggcaggcaa 4260
agctaggcag aagaggattc ccaggatcct gggtctgttc cctgccccag tgctgcagaa 4320
cggacttggg agccctcctt tgcctgctcc cgcgggtcac ccagcgagtg ctgagacccc 4380
attttctgtc gaggcgggcc gagtcttccc ttatccccag acgcctagcg ggcagggttg 4440
ggctgaatca aatgggagcc ctccagacat aaggaggcca gaggctgcaa ggagcggggt 4500
cgtgaccgct tacacccctt ctccacagcc cggcccgacc tggagggccc ccggggcact 4560
gggcggtgag ccacctcctg gcaactctcg gtgccgtccc ctgccctcgc tcgaggcctc 4620
ttctccccag caccgctgtg gtgtgccggg atcctgagcc taggcctccc gatgttccca 4680
cccgcatgat cccttcccgc cacacgatgc tccgttttct tccgttgtga atgccgcgtc 4740
ctgtcctggt gacaggagaa caatgttggt gaacgtcgca gcgggtgtcc gagtgctccg 4800
tgtgcccctg agagcgggtg ggagcggaag cctgagcggc ctgcggcctc cggcgatagt 4860
gtgctatctg ccgctgcagc gcgcgtccgc gcggcctctg ggctatttct ggccaggccg 4920
cagcactgtg gtcggtgcgg gcgtggcagg ggcggggcgg ccttatcgct cggctctccc 4980
gcctacgcct cccgctgcag agtaagccgg gctgccgtct tctcgccatg ggctccggtg 5040
agtctggagt ccggtcgggc ccccggctgc tccctaggcc gacccgggtt gagaggagct 5100
ctggtcgttt ggctgcagct gggagagact tgggtcagac ttagagggga cttccagccg 5160
gcgtgcgggg tggtcagggt ggagaggctg gcgggctacc gggacgccgg gcatcagggg 5220
ctggatggag ccgggccggc agtctgggta ctcagagatg tcgcccaggt gcccgccgac 5280
cgctcggctt actgcggcgc ttcccttgca gg 5312

<210> 8
<211> 3025
<212> DNA
<213> Homo Sapiens

<400> 8

ctctcacgac ctgatttcta cagccgctct acccatgggt cccccacaaa tcaggggtac 60
agaggagtat tgaaagtcag ctcagaggtg agcgcgcgca gccagcgttt cccgcggata 120
cagcagtcgg gtgttggaga ggtttggaaa gggcgtgccg gagagccaag tgtcagccgc 180
ctagggcttg ccggtcgctc cctccctccc tgcccggtag gggacctagc gcgcacgcca 240
gtgtggaggg gcgggctggc tggccagtct cgggcccctc ggccaccccg gggacccccc 300
ccaagccccg cccccgagtg ttcctattgg cctcggactc cccctccccc agctgcccgc 360
ctgggctccg gggcgtttag gctactacgg ataaatagcc cagggcgcct ggccgagaag 420
ctaggggtga ggaagccctg gggcgctgcc gccgctttcc ttaaccacaa atcaggccgg 480
acaggagagg gaggggtggg ggacagtggg tggggattca gactgccagc actttgctat 540
ctacagccgg ggctcccgag cggcagaaag ttccggccac tctctgccgc ttgggttggg 600
cgaaagccag gaccgtgccg cgccaccgcc aggatatgga gctactgtcg ccaccgctcc 660
gcgacgtaga cctgacggcc cccgacggct ctctctgctc ctttgccaca acggacgact 720
tctatgacga cccgtgtttc gactccccgg acctgcgctt cttcgaagac ctggacccgc 780
gcctgatgca cgtgggcgcg ctcctgaaac ccgaagagca ctcgcacttc cccgcggcgg 840
tgcacccggc cccgggcgca cgtgaggacg agcatgtgcg cgcgcccagc gggcaccacc 900
aggcgggccg ctgcctactg tgggcctgca aggcgtgcaa gcgcaagacc accaacgccg 960
accgccgcaa ggccgccacc atgcgcgagc ggcgccgcct gagcaaagta aatgaggcct 1020
ttgagacact caagcgctgc acgtcgagca atccaaacca gcggttgccc aaggtggaga 1080
tcctgcgcaa cgccatccgc tatatcgagg gcctgcaggc tctgctgcgc gaccaggacg 1140
ccgcgccccc tggcgccgca gccgccttct atgcgccggg cccgctgccc ccgggccgcg 1200
gcggcgagca ctacagcggc gactccgacg cgtccagccc gcgctccaac tgctccgacg 1260
gcatggtaag gccgggaccc caggaagtga ggaagttagg gcggcgctcg ggatatcagg 1320
gacgcgtttc cgagggcggg gagctggcct tgcgggaggt ttgggccagg atccttcccg 1380
agagagagga cccccttgtc ctgggcagct gtcactgggg tagcctgttt tggaagtgtg 1440
cgggcaagcg ttcgagctgc cccattgggg gcgctattag aacactgcag cgcgaacgtg 1500
aagatctttt tctctactta tccctacttc caaaatgtaa atttgcgccc cttggtgact 1560
gtccgccctt ggtttggccc tgcatgttgc agacctcatc tcctacccac ccgtaattac 1620
ccccccaacc aggacaggtc tgggcccgga actagagcct taggctagag ttagggaggg 1680
ggcggctaca ggaattggtg ttcgggcctc gagccgtccc gcgggcctga ctcagtcgcc 1740
cttgctgttt gcagatggac tacagcggcc ccccgagcgg cgcccggcgg cggaactgct 1800
acgaaggcgc ctactacaac gaggcgccca gcggtgggta ttccgggcct ctccctgctc 1860
gctcctcctc cttcatggag ctgtcctggc ctctatctag gacgctccca cccccactca 1920
cacacgccta tgtcctggga agtggtgcag gagatgaaat actaagcaag tagctccctg 1980
tcttttcgat tgtcccggac tctaactaaa gtcctcagtt tccaatctgt ctcaaagtac 2040
tgggcccggg ggtgggaggc ttgtcgcggc cccacccctg cttactaacc gagccctccc 2100
cgcgcagaac ccaggcccgg gaagagtgcg gcggtgtcga gcctagactg cctgtccagc 2160
atcgtggagc gcatctccac cgagagccct gcggcgcccg ccctcctgct ggcggacgtg 2220
ccttctgagt cgcctccgcg caggcaagag gctgccgccc ccagcgaggg agagagcagc 2280
ggcgacccca cccagtcacc ggacgccgcc ccgcagtgcc ctgcgggtgc gaaccccaac 2340
ccgatatacc aggtgctctg aggggatggt ggccgcccac ccgcccgagg gatggtgccc 2400
ctagggtccc tcgcgcccaa aagattgaac ttaaatgccc ccctcccaac agcgctttaa 2460
aagcgacttc tcttgaggta ggagaggcgg gagaactgaa gtttccgccc ccgccccaca 2520
gggcaaggac acagcgcggt tttttccacg cagcaccctt ctcggagacc cattgcgatg 2580
gccgctccgt gttcctcggt gggccagagc tgaaccttga ggggctaggt tcagctttct 2640
cgcgccctcc cccatggggg tgagaccctc gcagacctaa ccctgccccg ggatgcaccg 2700
gttatttggg ggggcgtgag acccagtgca ctccggtccc aaatgtagca ggtgtaaccg 2760
taacccaccc ccaacccgtt tcccggttca ggaccacttt ttgtaatact tttgtaatct 2820
attcctgtaa ataagagttg ctttgccaga gcaggagccc ctggggctgt atttatctct 2880
gaggcatggt gtgtggtgct acagggaatt tgtacgttta taccgcaggc gggcgagccg 2940
cgggcgctcg ctcaggtgat caaaataaag gcgctaattt ataccgccgt ggctccggct 3000
ttccctggac atgggtgtgg gatcc 3025

<210> 9
<211> 11913
<212> DNA

<213> Homo Sapiens

<400> 9

taaagcaggg tgttactaaa gaaagaagga agaaattggc taaacgaatt tagaacacaa 60
ataaaattac tgcacaaata aaatctctgc ttacgattaa gctggaatgg cggtcaaaaa 120
agaaaaggga aaaaaaattc ctagatgcag gtagatttct ataattaagc catttctcaa 180
aggggaagta aatgtgacag aatatttagc aagcaaattt actgaggaat attttgttcc 240
tatataatta aattcaacac tgaggtaaaa ctgacaacta ttaaactgag ggcagttggg 300
agagggaaag gggatgagat aaaatccagt ctataaaaca ttccattatc ttttataggt 360
taaaaaacac aaaagaaata cttggaaaga attgtctaag acggaaagat tttaagattg 420
tgtgactttt tttttttttt tttttttttt ttttttttta caggtgcaga ataatttttt 480
ttccctctct cgtggttttt cttttcccca tttttttccc ctgttgtttg caaagttgaa 540
tctggggtca ggagttctaa ttgtaacctc agactggaat tccagatggt gcgctcagga 600
ctggaagcaa ctctcagttc atcaatttca tgggcctacg tatctgctct ggccacggcc 660
tggcctccat ccccactgtg gcattaatgc agaacaaatt gagacatatc ctctatataa 720
ttagttccag aatttaaaaa gatttacggt ataataatag aggcagtgcc gcggaatcct 780
ttcactgagg actctcgggg agctcgggga ccatctcgcg gtggcatcag aacagacgag 840
taaccccagc ggctccaggc actgcgaact gggggagagg gggaaatttt gcaccgcatt 900
cggcagtacc taacggttcc gggctcccag atagggtcag cgaggagagt taggacagta 960
agtggggact ggttaaaaaa aatcgactta attttgaaat gatcagaaga agctgggagt 1020
agttatcaca aagcccctcc cttgcccccc ccttttttta attgaaggaa tgacgttgaa 1080
attttcaccg ccagcgcggg gctgggaacc atcctaaaca cttgccctgc agccccgagt 1140
gggcagattc cgaagagttg gagtcggcgg ggaggggagg aacagctgtt gggctgtctt 1200
gcgcgtgggg ctcggggcgc ggccccgggg gagaggcgct agtcgaaagc ttgttcctct 1260
cccgcgacag gcagcagcga ggtcgagcca ctctttatta cggcctgcgg gccccgcgcg 1320
cagtgtggct gtccccgccc ttgaccgacc gcagcgcttt ggggtgttta ttcagttgtc 1380
cgcggccgct gggtgactcc cggagggcgg cccaggcgtg cgcactggtc cctggggttg 1440
cggctgtagc agcccagctc cgcgctctgt cagatgcagt ggacagcgcc ggggtgaaag 1500
taagggttga gaaatcctca ctgccctgct cctacccagc ctcgattttt tcatattgca 1560
ataattatgc accttcgagg ccgggatgcc tgcgctttgc cagtgtatca acgccacatg 1620
gttttccagg ggctgtcctc gcctctgcat cccattagct ctgaagaggt agaggggtgg 1680
tgggtaaaac ctccaactgg acgttgagag acccgggttt cagctcggcg ttgccactaa 1740
cttgtttcct gtccttaaac agtagtagta acaaaaactg taaagttgat tgagtcaaag 1800
gcccacactg ctctcctatc taatcctcag aacaactcta tgaggtgggc gctagtatcc 1860
cattatcccg tttgaccagt aaggaaactg aggctcagag agactgcgga actcgtctag 1920
ggtcacacag ctgttggtta cctggcagag ctgaggttcc attctggggc caccaacccc 1980
ttgctcttta ccgctgcgct ccatagactg cctctccttc ggctgtctgg gcctcagttt 2040
ccttctctgt aaaacgagaa gtttaatctg cagctttctt tgccaatcgg tggttcagac 2100
caggaacctc tgctacagat ctcggcgctg acaggggtaa gaaggtgggt gaaagaaggg 2160
ggcgcccagt tttccacagc ctgcttttct ctgggacctc gcgaagggcg ggcctcgcga 2220
gctaaaggag gtacaggaga gcgcctatcg tccgcggcgg gtgaaggtgc tacctgcctt 2280
cgtgctaggc tgtgagtcct ggtgcttagc tcagggcgcc aaggccagtg tagctggcat 2340
gtcccccttg gaaaacctca ggtctcccgc agagaacgtt acccacaaca aagaagagga 2400
cagagaggca tggagcgccc tgcgactgca ggagtacgtc agttccccag cgctggctta 2460
gtgtcgcctg ggcttccggg catgtggatc cgttggggtc gtacggagac ttcctgtcgg 2520
gtccctgggg tcctccgact gcggctcctc agcttagcac tttcttcttg gccccgcagg 2580
ctgcagggaa ctcctcccac ctctttagtc ggagaagtcc aagtcggcgg aggggggcacc 2640
ccggggttcg caccggtgct cttcccctcc ccgcccccac aaggattctg agaaaataaa 2700
tggcagagga gagaggagtt ctacatttgc ttggctctcc tttcctccta tccaccccta 2760
catccctcac cccggggcaa aaacttattt ttgaaaaatg ttggcagaga tttacgtgtc 2820
tttgccttac ctgggtttca caaacacaac gactcacatt caagccagcc tcccttcaga 2880
taacctcctc tccccccgct aaaagtgcca aggatggtaa aagaagaaac aatctcaatc 2940
ttttcgtttg gaaatgaaag tccccggctt ttcataaagg gctcctcgcc cctcacagtt 3000
gagtcctagt taagaaaaac gacttccaag tagaaataat aggcggggag aaggaaggga 3060
gatacaggga tctggggggt tcttagggca actggcagtg aattttgtct cgagagtcct 3120
ttctccactc aaaaaaccaa acgcgcgagc cccgcgaaag gtttagggat agatcgtgtg 3180
ggagaggact gagcagagag cgtgggggca gtgtcttgta gaatctttct tttcttaata 3240
ataattttaa aagcttctga gtggagacga cgcaaagtca agcagcaaag gtggcctggg 3300
aggcaagcgg agggctcaag tgccgcatct ttaccctcag ggtctcctgc gcctacggga 3360
tgcgcattcc caagaagtgc gcccttcgag taagtcctgg gcccgcacac acttcgggtc 3420
cgcagccaga atttaatggc gacaacgttt atgcaatgca agctaaaaac caaagcgtaa 3480
aaaattacta tgtcatttat tgaaacgcca ttctttgtca aactgcaact actttgcttc 3540

acataagttt ggctggaaag cttgcagccc cagcccgggc cagccaggta caggaggccg 3600
gactgcaacc ggttgcttcc ctcccgtcgc gcctggccgt cccacgctgc gccgtcgctg 3660
ctgcctcctg gcgcccctgg gattttatac gcacctctga aacacgctcc gctccggccc 3720
ccggttcttc tccttgccta ggggttgttt cccaatagat actgactcct ttagaagatc 3780
caaaaaccaa accaaaacac cccctacccg ccccaaacac ctgctctggg gcgcgggggc 3840
tgccaaacag agactagacg aagggagtca gatttagcga agctcttcga gctcccaaag 3900
attcgaacac taactcgcgc ccgtgggccg atggaggttc tccctactcc actccttggt 3960
ccccttaact ggcttccgcc tcctggtcaa tcactgagca accagaatgg tatcctcgac 4020
cagggccaca ggcagtgctc ggcggagtgg ctccaggagt tacccgctcc ctgccgggct 4080
tcgtatccaa accctcccct tcacccctcc tccccaaact gggcgccagg atgctccggc 4140
cggaatatac gcaggctttg ggcgtttgcc caaggttttt cttccctcct aaactagccg 4200
ctgttttccc ggcttaaccg tagaagaatt agatattcct cactggaaag ggaaactaag 4260
tgctgctgac tccaatttta ggtaggcggc aaccgccttc cgcctggcgc aaacctcacc 4320
aagtaaacaa ctactagccg atcgaaatac gcccggctta taactggtgc aactcccggc 4380
cacccaactg agggacgttc gctttcagtc ccgacctctg gaacccacaa agggccacct 4440
ctttccccag tgaccccaag atcatggcca ctcccctacc cgacagttct agaagcaaga 4500
gccagactca agggtgcaaa gcaagggtat acgcttcttt gaagcttgac tgagttcttt 4560
ctgcgctttc ctgaagttcc cgccctcttg gagcctacct gcccctccct ccaaaccact 4620
cttttagatt aacaacccca tctctactcc caccgcattc gaccctgccc ggactcactg 4680
cttacctgaa cggactctcc agtgagacga ggctcccaca ctggcgaagg ccaagaaggg 4740
gaggtggggg gagggttgtg ccacaccggc cagctgagag cgcgtgttgg gttgaagagg 4800
agggtgtctc cgagagggac gctccctcgg acccgccctc accccagctg cgaggcgccc 4860
cccaaggagc agcgcgcgct gcctggccgg gcttgggctg ctgagtgaat ggagcggccg 4920
agcctcctgg ctcctcctct tccccgcgcc gccggcccct cttatttgag ctttgggaag 4980
ctgagggcag ccaggcagct ggggtaagga gttcaaggca gcgcccacac ccgggggctc 5040
tccgcaaccc gaccgcctgt ccgctccccc acttcccgcc ctccctccca cctactcatt 5100
cacccaccca cccacccaga gccgggacgg cagcccaggc gcccgggccc cgccgtctcc 5160
tcgccgcgat cctggacttc ctcttgctgc aggacccggc ttccacgtgt gtcccggagc 5220
cggcgtctca gcacacgctc cgctccgggc ctgggtgcct acagcagcca gagcagcagg 5280
gagtccggga cccgggcggc atctggccaa gttaggcgcc gccgaggcca gcgctgaac 5340
gtctccaggg ccggaggagc cgcggggcgt ccgggtctga gccgcagcaa atgggctccg 5400
acgtgcggga cctgaacgcg ctgctgcccg ccgtcccctc cctgggtggc ggcggcggct 5460
gtgccctgcc tgtgagcggc gcggcgcagt gggcgccggt gctggacttt gcgcccccgg 5520
gcgcttcggc ttacgggtcg ttgggcggcc ccgcgccgcc accggctccg ccgccacccc 5580
cgccgccgcc gcctcactcc ttcatcaaac aggagccgag ctggggcggc gcggagccgc 5640
acgaggagca gtgcctgagc gccttcactg tccacttttc cggccagttc actggcacag 5700
ccggagcctg tcgctacggg cccttcggtc ctcctccgcc cagccaggcg tcatccggcc 5760
aggccaggat gtttcctaac gcgccctacc tgcccagctg cctcgagagc cagcccgcta 5820
ttcgcaatca gggtaagtag gccggggagc gccccctacg cgcggggcag tggcgccagg 5880
gactctccgc tctaggacac ccccctctcc tacccctttt gaccgcagct cttacccagc 5940
tgcttcccaa gggccgtgag gatagcggaa gcggcggctg gggaggaggc cggagagtgg 6000
gagtgcacgc aggcactggc ccccgacatc ctccaaagcc aggcagagct aggagcctga 6060
ctgttcgcaa gagccgggag ggcgtctggg gccccttaga gtccgagaga ttccgggact 6120
gctagtcccc acgggagtgg aaccaggaga ccggcgcagt gtctggctct gccttgggga 6180
gagttttgtt aattaaggaa acgccaaatt gtcaccaagc gtagcgcttt tcagacgtct 6240
ggactctgga ttcggagtgg gaccagcgcg ggggtgagcg cctgtgctgt gctgcctccg 6300
cagcgctaaa atgtccagga gcacctccag cccttataat acggtgttgg cacacactta 6360
ggtgcacatt aagtatcgta gagaactaga atgagcacat gcacctccag taactaaatg 6420
gctttctgga acagcgtcgt gtccgtggat agctcccgcg tatggtaata cagaagtgga 6480
tcttcactgg gcttcgctca ggcggcggcc acgcaggttc tggggatgga agagaacttg 6540
acttatccga gtccaagcta tgcgggacca ggttctaccg caggcagtgg aaggaggcac 6600
gagctttggc ctttctttca gcgcatctgg ggcgcctctt catcttgtaa acgcggggct 6660
cagctgggac agtatttctt caagacctgg ggttagaaca aggacagggt tattcgctgt 6720
gcttagtcag gatacaagga gggaactcgg cacacgataa gtatcaggtt ggtttctcat 6780
cccgcgccga tgcccgccca cctctctttc gcctgggcgg cgcggacccg aacctgcgga 6840
gcccctgtag tttgccctct tcatttattt tcagtggatt tccacgctat tgagaaccta 6900
agccagttcg gtttgattcg gtaatttagt aaacgcttta ctaatacaat ggcggatgct 6960
tttcctggag caaaattact cgagggccta gggaccggga tgtttttgga atgcgtggga 7020
agcagagtgt atcttttgca ggaggtacct tcgcccaagg ctcgggagaa gggctctgcc 7080
cttgttgccc gccttcaccc gcgccttcta aacgcgcgtc ttcaggccgc tcctacctta 7140
gcctaaccag gagcacccgg cccccgcgaa gccttggaga aatcgaaacc gcccttttct 7200
tcgggagaat ctgggatcac tgtcctgtgg aggaaccgca agcagaccca agggttgcct 7260
ccactctatg cttttagtta gtgaaattag tgttttgtac tcaaggggcg ttgggacaac 7320

ttactggtcg ctgaggcttg tgttctttga tcagatcgcg ccacctttgt ttagcaggct 7380
ggtgttactt ttggggagtt tctaggtctc agtgtccccg tctataagag gacagctgag 7440
atgggcaacc tggagattgg gtccggcttg aagttatact ccatagatgc agaagcgcta 7500
cccaccaccc tcttggggct ttaaatatgg aactcggcaa cccatctaaa catctgtgac 7560
tgagaaacaa tgaatccgag agcactgtct gcatgtgtgc tatttggagg cggttgagaa 7620
accatcgaat ccatgcccct ggggcacaaa atactactac gccattatct ttttagtact 7680
tctaggggat ttttgaaacc tgggttctgt cgcttcgttt gggtcccttg tccagccctt 7740
ttcagggcag gtctggtgcc tgggatctta tcacatgtcg tggggggtgg gactgtgggt 7800
tttgcagcca gcaggcatta cacttctgga aaatatctgg attcttctgt aatatacagg 7860
cgtggctgcc atggagaaaa agttaaaagg ggaccaggac tagtaggtga atgtatcctt 7920
cttctccacc ctggagaaaa agcccaatgg agagagggcc ctagattcaa gtcatatagc 7980
ctggaagcac catctccagg tttaagtttt gtcttgcagt gaaaacagat gggtccatct 8040
tacccagccc ttgaggctca ttgggcagct tggatgtgag aagcgctccg gctgctatcc 8100
tggagtgggc aagggtggcc ctcgagattc tgggtttatt ctttggattt ccagggttcc 8160
tgctgcttcc ccaggaatgc agggcctggc ccaagacttg ggctgtgaga ggcaagggca 8220
tcaactctca aaaatgtgga aatggccatc agagcgccaa ggccaaaccc caggggagag 8280
gaatcaaggc cgtctctgga tccctagggc agtctagcca tggtgcacag gcaggggcaa 8340
ttgttggtac ccactcacaa ccgcccaaaa cttcacaagc atgttcatat tttaaattct 8400
taaatattat atcttgcatc ctaatcttgt aaacattaat ttagcaaaca tcaatcgcct 8460
acttggtcag gcactgggtt ctgaatatta attaagacaa tggcctcgag aacctttgca 8520
agaagctcac cagctaatcc tggagaggcc ccttgttttg gatgaggaaa tccaggttct 8580
atgagattag gtggcagagg tgggatttga acccagacca tgctagggtg tttctactcc 8640
ttttccaggg atcttggccc caccgttgag atggatttct tctggcctgc gggaggggca 8700
ccaaactctt agtccatgtg tgggggttga cgggtttgtg agggcagatg tggggtgagg 8760
acttgagtag ccccagacta gatgcaaggc ttaaggagga ggggaaaaaa gcttgcttgg 8820
gccggacgcg gtggctcgag gcggttggat cacctgaggt caggagtttg agaccagcct 8880
ggccaacacg gtgaaaccct gtctctactg aaaatacaaa aaaaaaaatt ggccgggctt 8940
ggtgacgggc gcctataatc ccagctactc gggaggctga ggcacaagaa tcgtttgaac 9000
ccgggaggct gaggttgaag tgagctgaga tcgcgccact gcactgcagc ctgggcgaca 9060
gagcgaaact ccgtctcgga aaaaaaaaaa aaaaaaagag gttgcctgct agtccactga 9120
tcccgcagcc ccgcaggctc gcagaaccct aaggcatgat taggcctggg atcatcaggc 9180
caccctaggg tcaggtgcgg aggcctgcga cgcccgaggg ccaaaggctc cctcttccgg 9240
tcacacagga gagggtattt tgttggggga caggttttcc ccaatcacag tgcagctgca 9300
gtcatcgttt tcagccttct gagcaccttc ctggctgagc ctgctgtcca tttcacccct 9360
cagctctcac ggcccggcca ggccaggcgg ttcccggctt ctctgcagcc gccggcgggg 9420
gaggagggca cagagcgcgt ctcgcctctt agcgcacggt caaggttgcg ctcccctccc 9480
gggccgggac taaccgtggg aaaggcgcta ccttggccac tcgattctct ccctccaagg 9540
cctgtacaag gagcctttga acacgctgct ccttccagcc ggcagcttta cctcccctgg 9600
cccctgcccc cgcactctcc gcgccaggcc aagcgctggg cccagggaca gcgcgctcta 9660
atcaaacccg tgtccgataa gcttctaatt aaaagagacc tccctccgct ccgcaggcgc 9720
tttcaccact gcccctcccg gggggacctg aaggagaggg tttgaggccg gtctttgccc 9780
gccgaggtct gcgtgtccgg tctgggagga ggcctaggag ggctcgcggg ccacgggcat 9840
ccttgggccc gagttctggg gtgcggacgg acgtctcgag agtgggtgcc gcgactcggg 9900
acccacggcc ctcgccgggc acggacagtt gcggagcagg gctctgagga ttgtgcagtg 9960
ccctgggtcc ctgcctactc ctgggctcag gaatggagaa gggttcgtct agagaaaact 10020
tcttcgtagc ttctatttcg tctctctctc tctctctctc tctctctctc tctctccctc 10080
cctctctctc tctctctctg tgtctgtctc tctttctctc tctctctttt tagtgtcctt 10140
tcagaaattc ggccttcact ccgggcaata gtgggtgccc tgagccaaat ttcgtttgta 10200
agctcatcta taggcctcaa agaaggccag gcctcaggtt tcaaaaggaa gtctgtggaa 10260
gcccaaacgt gctcgcccat gcaggagagc taacggggcc gggggttatc tttgcccact 10320
tagcaggaga gaaatggagg ccctcgaggt catatgataa aagctagtgg cttctcaaat 10380
ccccagtgtg tggtgctgag cagtgtgtga gacttaagcc tggacctgta ctatccggga 10440
gttaggattt gccccctcct tccctccctt gcactctgcc acaccaccct ccaggctcat 10500
gcctctgttg ccccagagaa gctttttttt tttctttttc tttttttttt tttctgggcg 10560
gggggtggtg gtgtgtgtgc atgagcgtcc gtgtgtgttt cgaaaatgga ggtaacgatg 10620
ccctgggtcc tgctcatcgg ctgagtcctg cttaagtctt tggaggcact ttggtcaaat 10680
agtgcaagag tgggagaatg ggtgctgttt gacccaattt actcctgaga attttgtgaa 10740
gtttggagac ccagggcagt cctgggccag gaggatctga ggctgctggg gtggccctgc 10800
caggatccca taggagcagc tctccttagc cattgtgtct ggacttcagg ccacagactc 10860
cggaacttgt agaggaatga gaccccatcc ttgggttccg gggccaatga cgtatcttta 10920
gccgaaggat ccttttcccc agaggcagca acagctttcc agaagcttgt ggggtataag 10980
tttaaagcaa tttaataaca aaaattataa aagactatgt tttccaactg aggctccacg 11040
ctctttttct cactttgggg ggggggggga atgctaggat ttccctaggg ggaaactgag 11100

gcaaaggacc gaagcactac catggaggga aagggagata cagtgtcagg cacactctaa 11160
aggaaattgg ttgtccagcc tggggagtga ggaagttggc tcctacagca gtggtcaggg 11220
tcggacccct gggcgcaagg tgggtcccat agggggaggg aggaggagag cgcagggtcg 11280
ccatctgaag gcccaactgg aaggggccct tgttttcccc ctcaccagct ttcccttggg 11340
tgtcatggtg tcaggtcggg tagccccaga cagataacat tactcggtaa ttactcttcc 11400
catttttaag accaggccat ttaactggct ccgagtaatt aatgaaagtt ggtgcgcggg 11460
cccctgattt acagctcgga gtcaacgcgg ggttagccca gcagattcat ttcgcttgcc 11520
cgcgctgctc ctgcggaaga gctgcagttt atttttgggg ggtgaacaac cttctctgtc 11580
gatcaggcaa cgagttttat acttaatttg ccaggggttc gctgcagaag cggcagagac 11640
cgtagaagac tgcggcataa ataaattggg aaaaagcgag gtgggggttt tagtcagaaa 11700
aggatggaga agcctgtccc aaggtcacat ccagggactt gggccgtctt cctgccgaaa 11760
gtcctggagg cttgtggggc tggattctcc tccgctgggg gtccgaatgc ggggttcagc 11820
gcacaccccc cccgcccgtg gctggttcag acccactgcc ccgtcttgcg agagcaccgc 11880
tgacactgtg cttctctccg ccgctcccgc agg 11913

<210> 10
<211> 4316
<212> DNA
<213> Homo Sapiens

<400> 10

ctgcagctaa taaaaaaaaa aaaagaaaga aagaaactgg tctctgtcct atttcatatg 60
ctcaggtaca acttttccag agaagaagag gagggggcgg gggaggagca ggaggaggag 120
gaaagaagga ggagaaggag aaggagaaga agaggaagag gaagaggaag aagaagaaga 180
agaagaagag gaagaggaag aggaagaaga agaagaagaa gaagaagaag aagaagaaga 240
agaagaagaa gaagaagaag aagaagaaga ggaagaagag gaagaagaag aaactgtctc 300
tagaccttca ttctcaggac aagttcattg tctggcacca agctccttgg ggtgaatttt 360
cttccaaaag agtccgggga gtccaggtat ggaatgggag gcagaaagtt caatcaaggg 420
actgggattt cggaatgaat aatgaaggga gatggactgg gtccatgccg aaggtttctc 480
cctggtttct cagcccccgg gcgaagactc agggagacat tgagacacac cctgcacagg 540
agggggaggg ggagggggag ggcaaagtcc caggccccca ggagtggctc tcaagggctc 600
aggccccgag gcggtgtctg gggttggaag gctcagtatt gagaattccc catctcccca 660
gagtttctct ttctctccca acccgtgtca ggtccttcat cctggatact cataacgcgg 720
ccccatttct cactcccatt gggcgtcgcg tttctagaga agccaatcag tgtcgccgca 780
gttcccaggt tctaaagtcc cacgcacccc gcgggactca tatttttccc agacgcggag 840
gttggggtca tggcgccccg aagcctcctc ctgctgctct caggggccct ggccctgacc 900
gatacttggg cgggtgagtg cggggtccag agagaaacgg cctctgtggg gaggagtgag 960
gggcccgccc ggtgggggcg caggactcag ggagccgcgc ccggaggagg gtctggcggg 1020
tctcaccccc tcctcgcccc caggctccca ctccttgagg tatttcagca ccgctgtgtc 1080
gcggcccggc cgcggggagc cccgctacat cgccgtggag tacgtagacg acacgcaatt 1140
cctgcggttc gacagcgacg ccgcgattcc gaggatggag ccgcgggagc cgtgggtgga 1200
gcaagagggg ccgcagtatt gggagtggac cacagggtac gccaaggcca acgcacagac 1260
tgaccgagtg gccctgagga acctgctccg ccgctacaac cagagcgagg ctggtgagtg 1320
aacccggccg gggcgcaggt cacgaccacc cccccatccg ccacggaccg cccgggtccc 1380
cccgagtctc cggatccgaa atctaccccg aggcagcgga cccgcccaga ccctccaccc 1440
gggagagtcc caggcgcctt taccgaggtt cattttcagt ttaggccaaa atccccgcgg 1500
gttgggcggg gagggggcgg ggctagctgg gcggggctga ctgcggggac cggctagggt 1560
ctcacaccct ccagggaatg aatggctgcg acatggggcc cgacggacgc ctcctccgcg 1620
ggtatcacca gcacgcgtac gacggcaagg attacatctc cctgaacgag gacctgcgct 1680
cctggaccgc ggcggacacc gtggctcaga tcacccagcg cttctatgag gcagaggaat 1740
atgcagagga gttcaggacc tacctggagg gcgagtgcct ggagttgctc cgcagatact 1800
tggagaatgg gaaggagacg ctacagcgcg caggtaccag gggccatggg cgccttccct 1860
atctcctgta gatctcttgg gatggcctcg cacaaggttg ggaggaaagt gggcccaatg 1920
ctaggatatc gccctccctc tagtcctgag taggaagaat cttcctggct ttcgagatcc 1980
ggtaccagag agtgactgtg agagtccgcc ctgctctctg ggacaattaa gggatgaaat 2040
ctctgaggga atggagggaa gacagtccct ggaataccga tccgcggtcc cctttgagcc 2100
ctccaacagc cttgggcccc gtgacttttc tctcaagttt tgttctctgc ctcacactca 2160
atgtgtttgg ggctctgatt ccagtccctc ggcctccact taggtcaggg ccagaagtcc 2220
ctgctcccca ctcagagact cgaactttcc aaggaatagg agattttccc aggtgtctgt 2280
gtccaggctg gtgtctgggt tctgtgctcc cttccccacc ccaggtgtcc tgtccattct 2340
caggttggtc acatgggtgc tgctggggtt tcccatgagg agtgcaaagt gcctgaattt 2400
tctgactctt ctcagatcct ccaaaggcac acgttgccca ccaccccatc tctgaccatg 2460

aggccaccct gaggtgctgg gccctgggct tctaccctgc ggagatcacg ctgacctggc 2520
agcgggatgg ggaggaacag acccaggaca cagagcttgt ggagaccagg cctgcagggg 2580
atggaacctt ccagaagtgg gccgctgtgg tggtgccttc tggagaggaa cagagataca 2640
catgccatgt gcagcacgag gggctgcccc agcccctcat cctgagatgg ggtaaggagg 2700
gagatgggta aagaggggaa cgaggggtca tgtcttttct cagggaaagc aggagccctt 2760
ctggagctct tcagcagggt cagggctgag gcctggagat cagggcccct caccttccct 2820
tcctttccca gagcagtctc cccagcccac catccccatc gtgggcatcg ttgctggcct 2880
tgttgtcctt ggagctgtgg tcactggagc tgtggtcgct gctgtgatgt ggaggaagaa 2940
gagctcaggt aggaaggggt gaggagtgga gtctgagttt tcttgtccca ctgggggttg 3000
caagccccaa gtagaagtgt gccctgcctc attactggga agcaccatcc acactcatgg 3060
gtctacccag cctgggccct gtgtgccagc acctactcat ttgtaaagct cctgtgaaaa 3120
tgaaggacag attcttcact tcgatgatta tggtggtgat gggacctgat cccagcagtc 3180
acaaatcaca ggggaaggtc cctgctgatg acagacctca ggagggcagt tggtccagga 3240
cccacatctg ctttcttcat atttcttgat cctgccctgg atctacagtt acacttttct 3300
ggaaacttct ctgggatcaa agactagggg tttgctctag gaccttatgg ccctgcctcc 3360
tttctggcct ctcacaggac attttcttcc catagataga aacagaggga gctactctca 3420
ggctgcaggt aagatgaagg aggctgatcc ctgagattgt tgggatattg tggtcaggag 3480
cctatgaggg agctcaccca ccccacagtt cctctagcca catctgtggg ctctgaccag 3540
gtcctgtttt tgttctaccc caatcactga cagtgcccag ggctctgggg tgtctctcac 3600
agctaataaa ggtgacactc cagggcaggg gccctgatgt gagtggggtg ttggggggga 3660
acagagggga ctcagctgtg ctattgggtt tctttgactt ggatgtcttg agcatgaaat 3720
gggctattta gagtgttacc tctcactgtg actgatacga atttgttcat gaatattttc 3780
tctatagtgt gagacagctt ccttgtgtgg gactgagaag caagatatca atgtagcaga 3840
attgcacttg tgcctcacga acatacataa attttaaaaa taaagaataa aaatatatct 3900
ttttatagat acaggtagat atgtttttat agcatgcacg taaatgtgtg tgtgtgtgtg 3960
tgtgtgtgaa gagaaagagt gaatagagag attaagattc ttttaatggt gaaaagatat 4020
acatatattt ggaattagcc agcttgactc agtttaggtg atcccaattt tggtggcaac 4080
aaccaaagca tcgtagtcag gagccagtcg aacatatgcc ttcctctctc catcagactg 4140
aatcagagtg ttgactttgg ccacatcaat gtcacaaact tcttcacagc ctgtttgatc 4200
tggtgcttgt tggctttaac atccacagtg aacacaagta ggctgttgtt ttctatcttc 4260
ttcacagcct actcagtggt cagcggaaac ttgatgataa catggtggtc aagctt 4316

<210> 11
<211> 14147
<212> DNA
<213> Homo Sapiens

<400> 11

gggggcactg agcaatacac ttagattaac aacatctcac aagtgtgagt accattgatt 60
taaacttctt tgttggttgt gttttgtgtt tatgtctgtt ctacattcat tttttccaga 120
gttgcctccc ttagagtttc agtaattttt atcttttcaa acctttccag ccatgggaag 180
gactagcagg tgctagtgga cccaaaagaa cctcagacct gctgagattt ctgattcttg 240
gtgttcagac caattagcaa atagtttaat taaacggcct gctttcatgg tcaacacaat 300
tctgaactgg tggtcattta gaccaagtcc actttgtttt tgagacggaa ttttgctctt 360
gtcgcccagg ctggagtgca gtggtgcgat cttggctcac tgcaacctct gcctcctggg 420
cttaagggat tctcctgcct cagcctccca agtagctggg actacaggca cccaccacca 480
ctcccgacta atttttgtat ttttagtaga gacagggttt tgccatgttg gccaggctgg 540
tctcaaactc cgacctcaga tgatctgccc acctcagcct ccctaagtgc tgggattgca 600
ggtgtgagct accgcgccca gctaaagtct agtttgtata agaaggcttt ccttgttatg 660
tcacaggatt cctgagccta tcaaatagat tttaatgttt tgagactcca cgtacttccc 720
caaaatgaag tatttaaatt gtcagtgcct gcccccttgc ctggtctttt tgatgtactg 780
acctggtctg ttagtgtgat gaaaaatatg gttgagtctt actgctttat tgtaattagt 840
aggactgtct agaagtttaa tttttctaag agatgaagat ttgagtctat gtagctggct 900
gtcctttaaa aatatttggt tacttgggat atactttgaa ctaactttct ggtcatttta 960
tgtgatcaaa agtgtcagtt cttcctgttc ttagtatatt tctttgtttg ggaagtggaa 1020
gggatgaaat ctctatattc tttcctgatg acacagaaag gagtacacat ccacatctca 1080
tgaatagaca tgtaattata gaagaaacag cctaacggcc gggcgcggtg gctcacacct 1140
gtaatcccag cactttggga ggccgaggtg ggtggatcac gaggtcagga gttcaagacc 1200
agcctgtcca agatagtgaa accccgtctc tactaaaaat acaaaaaatt agccgggaac 1260
ggtggcaggc gcctgtaatc ccagctactt aggaggctga ggcaggagaa tcgcttgaac 1320
tcggagggtg gaggttgcag tgagccgaga tcacgccact gcactccagc ctgggcgaca 1380
cagtgagact ccatctcaaa aaaaaaaaaa aaagcataac catatgacta ttcacactgc 1440

cttacatact aaatcagtag taatttgctt ctcacatagc aaataaaaat aaatcagcag 1500
taataaacat atcccttttg tgttacagct agcatttggt gaactctgta tttttctccc 1560
ttttaaagtg ttgaactcat aggttttcat aaggtaagct tacaccatta actaattttg 1620
ttctcttttt tgatgttaaa attgtccctt aggagctgat caggttcatt cttctgttct 1680
ttaagcatta cccccagaat attcttgaaa gtattcttgc tctctggcaa caacacccat 1740
acttcattta tttcctgtcc ctaaacatac actcagccaa cctcaggttc cttttaatgg 1800
agtagtcact tattcattca acatacgttg ggtgcctcct ctgtgccaca cactgttctg 1860
gtccctgggg atagagcagg aaacaaaaaa aaacaaaatc tgccctcata cagctgacat 1920
tctagtcagg ggagacaaac aataaacaaa tgaatgatgt agtatgtcag atgttgacag 1980
tgctatggag aaagcatatc atggagatca gaagcattac ctgcaatgga acagtcatga 2040
ttttaagaag aatggctgac tggatgtgaa gtgtgagaga gaggagccca ggtttttggc 2100
tgagcaactg gagggatgga gttgccatta tctgagatgg agaagttagg ggaggagtag 2160
atttggaggg gaaggtcagg aatttagtct ggaacctgtt aagtatgagg cacttattaa 2220
acactgaagt ggatttttag aagatgtcat taaaactagt caagttcagg agagagttct 2280
gggctagaaa taaaaatgtg ggagatattg gtatatacat gggttcatac tacttttttt 2340
tccaatttaa catcctatct tattgctctc tgcttttcca acagtattta gtttcattta 2400
cccctcaaac tgactcctac attgacagca caataaaatc cacattcctt attatattaa 2460
gaccctgtgt gatctaacct cttcctatct cttggacctt gtcttaggcc actttccctc 2520
cccactcaat ttctaacact ctgacctcac tgcttctcaa acagctcttt gttgcctctt 2580
gtcctttttg tggaaaaggc cttcctactg cttaaccatt aggcattctt cgattccttc 2640
tcattcttca ttcttgagaa aggccttcct tgacccttta tatcaattag gattcttttg 2700
ggcgagatct gatccaaact ggcttagaga aaaggagtca actgactggt tcatgtaatt 2760
caacctcctt cctctgctgc aaccttccct tggctcccat ctcacttaag gtcaaagcca 2820
gggtccttac catggccttg taggcttcac acagtttgtc tccctcatta cctctctgac 2880
ctcacctctt acattccccc ttcacttatt cttctccagc cagagtggcc tctttgctgc 2940
acctcaaaca tgccaggcac actcccacct cagtgccttt gtgtttgctg ccccttgtgt 3000
gtggaacaca tcctccatat agccacacta ctcaaaattc actgtcttta agtctttact 3060
caaatgcttc tccgtgaagc catccctgag ccctctttaa aattgcagcc ccccactcct 3120
gtactcctta ttgtctctca tttttaattt ttgttattgt agttagaacc atctgacaca 3180
tagcttttat tccattggtt ttttgttatg tctttcttta caagaatttg aagtccatca 3240
ggccgggagt tttgtttgtt gtgtttgctg ctatctccca gtgcctaaaa ttgcctggca 3300
tacagtaggc atttaataat ctttgaatca gtgaaaacca gatggtggct tggcatttcc 3360
acataggaat gagccaggtg gaaatcatcc aggatataag tagatcttga agtgataagg 3420
aagggtcatc ataatcatgt ggggcccatt ttgccctttc ttgtttcttt tctctaggct 3480
cagcaacagc ctcaccaagg actccatgaa tatcaaagcc catatccaca tgttgctaga 3540
ggtgagagca gctcacccca ctaccagact ctgtgtttag ggtggtgacc tgaagaagga 3600
agagagcgaa agaagggaag gaccatcttt ccctctaaac tggagtcaag ggagggaggt 3660
cagagcaagc ctgggggcgt aacccagacc cagtctttgt tcaatctctt ctgtcctctt 3720
tttcaggggc ttagagaact acaaggcctg cagaatttcc cagagaagcc tcaccattga 3780
cttcttcccc ccatcctcag acattaaaga gcctgaatgc ctttgagtca catggccctt 3840
ctttttcccc ataaaccctg ctagttgcca cgggggcctg ttcctagggc acaaagttac 3900
tgagagaccc agagatccag tctctctgtg gaacctccaa aatgccccag cagtcctgcc 3960
tccagcctgt tgcctggcag tatttgccag ttgaccttat gcactgccct ctcttctgtc 4020
atcttggcaa cctgggcccc ccacctcacc ctttcttcca ttccttttcc tagacgccac 4080
cctttgaatt gccatagaga atgggccaat tcatggtgga ggtttgttca ttcccacaac 4140
aatcaaaggg ccctaaggtt tacgctttca cacacccaat cattccccag gtaccccaaa 4200
ttacacccaa accctaactc agccctacct tgtcttagcc cctgcttgta agtgttccag 4260
cttccaatgg gcacagacct ggatcccgct ctccctagtc gggcccctat tctaggttga 4320
gtccagccac cgccaatcaa tgcagagcca ggttcctccc ctttttaact ctggccgcag 4380
ttcaaccctg ccctctgaaa gcatttcgtt tctgccactc aatgctcttt cgtgtgcctg 4440
acagccatcc tgccctccta cctccgctgt gttctaaatt cgtctttacc cagctctatg 4500
gtcttgtcta accgtagagc tgccctgccc gcctacgcgg agcccagtcc gacccactcc 4560
cgccggctgc tacgcccatt tctatacaag ccctgcttcc gctgagcagc atggcgtgcg 4620
acaccgcccc cttggtgttt tggcaggggt ctagaagtcc ctcgtccgcc aatcagagaa 4680
aaacagggcc acccaccccc gtccttgggg gctgtcttcc atcaatccca tgtaagccaa 4740
tcagtgtgag gcagaccccc gcccccccga cacaggcccc gagccttttc agttgctcac 4800
agtccgtcag tccttgagcc aatcggcgtg gagcaccgtg aaggccgaac gcgcctcctc 4860
gggactccag gggccgtgag cgttccatca tttcccctta cgccaatcac ggcacagctc 4920
tgtagggaag ggcccgtccc ccaacccctc gaggccttgc ggccgattaa tagcgctttg 4980
gccaatcagc gagcggcggg acattgggct cctcctcctc gggcccacgt gagctgtagg 5040
gaaacgcagg ggcggcttct aggtgctgcc gccgccaccg ccaccaccac ctccaccgcc 5100
gcctcggaac ccaggcctgg ggggcggtgg ggccgcgtat ggagcccccg ccccccggag 5160
ctgccaacat tgccaacgcc accgccacgc tacacacagg tgagctctgg gcctggaggg 5220

tggagggccc agtccgtgac cccacgtatc cttcccgccc ccgcgcagag gatgtggctt 5280
ggccggtggc ctgctggtgt tcgactcccc gccgccacca ccacggctgg tggacctgcg 5340
tgtggcattg ctcaagccct tcgtccctta tagtggatct gaccgtggcc taacctcccc 5400
ctcccgttgt ataatggatc ggtctgcgtg cttatgtttt tccccacgcc aacttagggt 5460
ggactcgtcc ataggctttc cccctacccc tacccccacc cgccccccac ccacccccac 5520
ttatagggag ctagcctgtg acagtgttca gcccccttaa tagtaggtgt atctgagtgt 5580
ttggatttct cctagcctca actttcagga gacccgtccg tggccttatt tattccaccc 5640
ttcctgtaca tcgtagcgaa tcaatccgtg gcgccgcact cctccgcatc cctctttaac 5700
agtgagtcta cctgagtttg tattcctgcc tcctccagtc ccccaatgca tcagtccatg 5760
gcttttttca aaaccacccc ccccccccac caccaccacc accattttat agtggatcag 5820
tcttcatgtt tggattcacg tccctttagc tatcagtcca ttgccctgtc tcctcttttt 5880
cgtagactgt cgctctagcg cttggggttc tctccttttt aatagtgggt tagtttagtg 5940
ctttacatct ccctattttc agtagctcag tccatagcct tttccctctc tgctttgaat 6000
cagtctgtgt gatggctttt ctctcctctc actgtggtgc aggagtctgt aaggttttct 6060
gctccccttc ccctcagacg atgagcctca tcacccctcc ctttgcagtg gatctgttca 6120
ttggccttcc tcccccacac ccctgtatgc ttgcacagtc ccccgcatac cctcccgtga 6180
gtccagtgct ttgtaattgg gggaagatcg gccgtgtact ttcaatttcc ttctcttttt 6240
ttttcccttc ccagaagaga acgtgctgat cctcttttcc ttgtgatgga gcactgtacg 6300
gccttccctg tcctccacct cttaatagtg ggtcagcctg gtcacactcg taaccacaaa 6360
gacgttctgc ctcactacaa taagtgaata cattagcgcc agtgatgata gtaatgccaa 6420
taggagctag cgtttattga gcacctgccg tatatcaggc agagtgtttc atatgaatga 6480
gttcctttaa tcctcaccac aaccccatga gataggtagt gagggaattg ggccataggg 6540
aggttaaatt ccttactcaa aggtggccca ggaagtactc aggacacagg ataatttttg 6600
aggctcccct cttagatctc atcccacccc acgtgaggcc tcagtcctta aaaaccaaat 6660
tagttctcac ctccaagctt ttgttcttgc tgttccctct gcctgcagag cagtcttcat 6720
tcctcacctt ctctcagcac ccacccaccc cccaatccca gcagctgtgt gaccttagat 6780
gagtgattta accacttcct ctctgtgcct ttttcttcat ttgtgaaatg ggggatcaga 6840
actagaatcc cttccagtgt aaataaatat taactgtgaa ttaatggtaa aacaagtaac 6900
ctttctagca ttaccgccta taccttccca cccccttact gtgctccaac cacactggtt 6960
ccaccacaca gctcctcaaa catagcaggc atgggccagc ctcagggcct ttgcattggc 7020
tgttctttgt gccttgaaca ccacccccag atctctgcat ggttcctttc agcactctgt 7080
tcaaatacca acccccaaag gatgtttacc acactgtggt gtgtggaatg cagtataaaa 7140
agatacatgt atgaaagatg tttctaagaa tgccagttta tatatgagta agggttttcc 7200
tccttttcaa aaggatgtgt tcacaagact gaggtacgta atatgctctg taaaggcata 7260
ggtataaaag ctgtttctaa gagtgctagt tgtatatgtg aataaggact gtgttgttgc 7320
attgaaagaa tgtgttcaca aaactgtggt gtgtagctgg gcacaggggt gtgcacttat 7380
agtcccagct actcgggagg ctgaggtggg aggatagctt gagcccagga gttccaggcc 7440
agcctgggca atatagcgag atcccttttt ctgaaaaaaa aaaacccaaa aaacaaccac 7500
tgtggtgtgt gtaatacact ctcttaaagg cacaggcgta aaagatgttt ctaagagtga 7560
tttgattttg ttgtacctgg gtagtgttat ttcattaaag gttgattgtc acaccatggt 7620
acttagaata tactatacaa aggcacatgt ataaaaaaga agttcctaac aatgctactt 7680
gtaaaaacaa aaaaagtcaa cccctccctg accatctgaa gctgcacacc cagcagggct 7740
caagccaggt ccctgatttg tcccgttgcc ctttatcact gtctgacaca ctgtgtgggt 7800
tacttgtttg ttgtctgtct ctcctcatta gaatgtgagt cccatgagga ctcagttttt 7860
gtctgctcat tcaccgctat gtctccagca actgaaaatg taccaggtaa acagcagatg 7920
ctcaataaga atttatccca tgaataaatg ggaaaaagat aatttgagac accagtgtag 7980
gggtcacatg aggaagaaga agggatatga gagctaatta gttgggactg ttcctgcaac 8040
caacctttac tgagcccctt gtgagtacca ggcactcttc taggtgctgg aatatggcaa 8100
ggaacgaaac agggcgaaat atttgtgctt gtggagctga catttattaa aagttgggga 8160
ggctgggcac ggtggctaac accagtaatc ctagcacttt gggaggctga ggtgggtgga 8220
ttgcctgagc tcaggagttc aagaccagcc tgggcatcac ggtgaaaccc catctctact 8280
aaaatacaaa aaaattagct gggtgtggcg gcgtgcgcct gtaatcccag ttacttggga 8340
ggctgaggca ggagaattgc ttgaacccag gaggcggagg ttgcagtgag ccaagattgc 8400
accatgcact ccagcatggg tgacagagcg agactctgtc tcaaaaaaaa aaaaaaaaaa 8460
acccaagctg gggagacaga ctatgtgaac aaaataaata agtaaaatta gcataataga 8520
caaatgctga gaagaaataa tgaaataggg caggtaggaa tcgagtattg ggggtggttg 8580
aaatcttagg catagggaag gtgactcttg aattagggcc tcacggtaga gacggagaga 8640
gccttaatgt ctacgggaag agcataccag gcagaaggaa gtgccagttc agaggccctg 8700
aggtgggatg gtgcctgcca tgttcaagga acatcaaggc ggccagtgtg gctggagcag 8760
agtgcgggga agggtagaag gtgaggtcac acaggtgatg ggagccagat agtgcaggtg 8820
atacatagga taggattgca ggggcagggg gagaaggatg acacactagc taatagctaa 8880
tatctgtgga gtatctgcca cacatcagag gcacttttct gttcaaatac caacccccaa 8940
aggatgttta ccacacggtg gtgcctggaa tgcactacaa aaagatacat gtgtcaaaga 9000

tatttctaag aatgccagtt tatatatgaa taaggatttt cctccttttc aaaaggatat 9060
gttcacaaaa ctgaggtatg taatatgctc tatggaggca caggtataaa agctgtttct 9120
aagagtgcta gttgtatatg tgaataagga ctgtgttgtt gcattgaaag aatgtgttca 9180
caaaactgtg gtgtatagct gggcacagag gctgaggtgg gaggatcact tgagcccagg 9240
agttccaggc cagcctgtgc aacatagtga gatccccgtc tccgaaaaaa aacccaaaaa 9300
ataaccactg tggtgtgcat aatacactct cctaaaggca caggcataaa agatgtttct 9360
aagagtgatt tgattttctg gcacctgggt agtgttattt cagtaaatat tcattgttaa 9420
acactatggt gcttggaata tactatacaa aggcacatgt ataaaaaaga agttcctaac 9480
aatgctactt gtaaaaacaa aaaaaaaagt caacccctca agtcttttac agaagtattt 9540
tacagaattg acacagttaa tcctcacaga agccttggag ggtaggtgta cttttgagaa 9600
tatttgactt tgagagtaaa atatccccat tttacagatg aggaaacaag caaagagagg 9660
ctgagtaact tgctcagggt aaccttgagc cagaatagcc agaacagggg cagaggaaca 9720
taaggctgaa gactcagaaa gaagaacaga tttgcaaggg caggcaggtt atttggcacc 9780
tttgatcacg cagaggaaga atgaattctt cctggaatgc gatgattgtg taattgagtc 9840
catgaagtga gggaagagct ctccctgcgg ggagagcagc ccgtgcttac cctcagaggt 9900
tggaactgtc tcgggaacgg tagatagttc agggtggctg cctcagagcc tttgtacacg 9960
ccgttcgctc cgcctgaagg ccctccccca cagagggcag cctggcttgc tacctcccct 10020
ccttcaggcc aggtgtcact tccttgggga agaactcctt gtacaccctg tgtaaaaaca 10080
gcattcctgt cactctctag gatcttaccc ttctctgttt ctcttcatgg agaactggtg 10140
gtgtaaggcg attgctcact agagccaaac cacttaggtt ttgaattccg gctctgccaa 10200
cttccagctg tgtgacttgg ggcaggttac ttctctgtac ctccattgcc tcatgtggaa 10260
aatgggggaa atgataatcc ccacccttta acggtggttt gaggattcaa tgagttaaat 10320
gattcaaaga gtgtgcagcc cacggtaagg atcgtatgcc tggcttgcat ttttaaatta 10380
ttaaaatgta ttcatagcat acccgtagat gtctgacagg cctctgaaac tgaccatctc 10440
caaaactggt gtgacccccc tcaaaactta ctctcctgca aggctcccac ctcagctgac 10500
cacaatgctg tctttgtggg tgattaggcc acaaacctag actcaccctg gacccttctc 10560
tcacctcccc cgggcatgct atctgtcagc aagtcctatt ctgtgttcag gctttctcca 10620
gaccccagct acttctcgcc agccccactg ctaccaccat catctaccgc agtggcttct 10680
tcagccccaa cagtctgttc ttctcacaac cgccaggggg atcctgttga aatctcagtc 10740
acagcctgtc atccctctgt tcacaaccct cttgtggctc ctgtctcact caacatcctg 10800
acagcacccg aaaaagcctg gctcagtctg gcccccgtca ctctgttctc ctctcccgct 10860
gcctccctcc tctcccccga acccatgtca gcctcctctc cccatgtcac ctgccaggca 10920
ctctccagcc ttgggccctt cacaccggct gccgcttcca cctggcttgc tgctctcaga 10980
tactcacgag ctcagttcct tcacctcctt ttggtctctg cccagttgtc ccctccttgg 11040
cgactactct cccctgactc tccatgtaaa agtaacagct ctcccctgct tgattttttt 11100
cccataaccc ttaccaccat ctgacgcact cttgtacatg tttgcctttt ttctcatctg 11160
cctttcctgc taagatgtga gctcctctgg agcagggatt gttgtctgtt gcgttctcga 11220
ctgtatccgc agcacctggc ccacagtatg cactcaccaa atgcttgcta agtgaaggag 11280
atggaaagaa gtaaacatat ctaagcaagg cagattacta gaactcttat gaggggtcaa 11340
gtgggagaaa taacaaaata caaagccctg tgtgctgttg aagctggaag cccaccgtca 11400
cctttcactc agctgatagg ttctgttgca gctgaaagaa gaactggtaa aattgcctgt 11460
ctttgccacc aggagtcact ctgaactctt tggacttctg aaacaagcct gcctgcaaaa 11520
caaattcagt gaggtctctg gtgacaggac tctctaatcg gacacttcat ggctttgtga 11580
cttgtataat tgaacaggaa gctcatgttt atttccagtt tgctctcagg aaagaaagca 11640
gtaatggggt gacagggagg atcctgttct ttgaaggaca tgctgagagg gaatagtcgg 11700
tgtgaggggc cgtttagcct aatggtgaaa tgcttagtca actggaacct gactgcctgg 11760
ctccaaatgc tggctttgcc acatgtggcc tgtggaaggt acgtagacat ttgtgcctcc 11820
gctgcctcat ttataagtta atgacaggaa gactacctac ttctcagagg catgatcagg 11880
gataaataag tcaatgccca ttaaaaaaaa aaataacagt acaggtgtac atgcccttac 11940
ccaaaacccc tggggccaga tgtgtttcag aatttccatt tcttcacatt tcagaaaggt 12000
aacatgatgc atatacgatg ttacataaca ccccttagta agctctgtaa tcggctgtgt 12060
tcatatttct gcagcaaaat gtatgaacac tcgcaataag tgagattcaa taaagaccag 12120
aaagggcctt gtttcatttc aaatcaggtt ttgccaccaa aagagttaaa agaagctttc 12180
tgcccttaga gcattttgga tgttggaatg gcagataagg gactatgaac aaatcagaaa 12240
ctaatattta gagcttatca tacacctcag gcatataata tttaatctgt acaatatctc 12300
tgtgaactag gtattgttat ccccatttta ctaatgggca tatcattatc cctgttttac 12360
acatgagaaa attgaggtac aaagaggtta aagaatatca gccaagcgtg gtgcctcatg 12420
cctgtaatcc tagcacttta ggaggccagg gagagaggat tgcttgagcc caggagttca 12480
agaacagcct gggcaacata gtgagacccc atttcttaaa aaaagaaaaa gaatatgccc 12540
agggtcacac agctaataag tggcagaacc aggattcaaa cactggcaat ctggccctag 12600
tgcctgggca gttgtccagc aggccatatt gcctctctgc ttagaagagt gcctgatgtg 12660
tattaagctc tcaatagatg ttaattattg ctgtcatcaa gaaagagaat attcagggcc 12720
gagcacggta tctcacacct gtaatcctag cactttggga agccaaggtg ggaggtgggt 12780

ggatcacttg aggtcaggag ttcaagacca gtctggccaa catggtgaaa ccatgtctct 12840
actaaaaata caaaaattag ccgggcgtgg tcgtgggtac ctgtaatccc agctatgcgg 12900
gaggctgagg catgagaatc gcttgaacct gggaggcaga ggttgcagtg agtcaagatt 12960
gtgccactat actgcgctcc agcctgggca acagagagag actccatctc aaaaaaaaag 13020
aaaaaaaaaa aaagaacgag aacattcagg gacagtaaca ggacagagag atcaggcatt 13080
cattcagttt taggacggct tcataaagga aaggaccttt gggctgggac ttgaaagata 13140
agaaagactg ctaggcgaaa gaaggaagcc ccacgtggag tgagcagcct ttgcaagggt 13200
ctggcaaaaa aaaaacctcc tgaaaaactt gacattgata tggtagaaga tacagataat 13260
attcaaatga ggaggatgga actagttttc tattcttgct gtaacaaata atcacaaatt 13320
tagctgctta aaacaacaca tttgttatct tacagttctg taggtcagaa gtcccacact 13380
ggtctcagct gaaatcaagg tattggcagg gttgcgttcc ttctggaggc tctaggggaa 13440
aatccatttc ctgctcattc aagttgttgg cagaatccaa ttccttgaga ttgtaggact 13500
gaagtccctg tttccttact gtctgtcagc tgacagccat tcccagcttt aagaggctgc 13560
ccacagtcct tgattctgag cctccttcct ccatcttcaa agccaacaat ggcaggttga 13620
gtccctctca cattttgaat ctcccctgcc tcttctgtca tcacatctct gagctacctt 13680
tctttctcca tcatctgctt ttcagagctc atatgattag attgaatttc atcaaataat 13740
ccagaaataa tctccctgtc ttagggtttg taaccttaat ttcatctgca aagttccttt 13800
ctgctatgta gtgtaaataa catacaggtt ccgaggatta ggatgcggag cagggttggc 13860
atcatttggc ctaccacagt ctgccctctg acccccacag attcacattt gtttcacatt 13920
caaaatacat tcacaccatc ctaagatacc catgagttgt atcccatcac agggagcatc 13980
ccaagcaaga ctggggagct agaagttagt ctgggaaggt ttagggaatg atggtagggt 14040
ttgaagacag agatggagaa aagatgacaa aaaaaggaat cctcctcccg ctgacctctg 14100
cccctcctat gtccacagcc tctcaactca gctgtttgct ctccagg 14147

<210> 12
<211> 3120
<212> DNA
<213> Homo Sapiens

<400> 12

aactttaagt ttaggccggg cacagtggct cacgcatgta atcccagtac ttggagggac 60
cgaggtgggc ggatcacaag gtcaggagat tgagaccatc ctggccagca tggtgaaacc 120
ccgtctctac taaaaataca aaacttagct gggcatgatg gcacgtgcct gtagtcccag 180
ctacttggga ggctgaggca ggagaatcgc ttgaacccgg gaggtggagg ttgcagtgag 240
cggagattgc accactgcac tcgcctggtg acagagcaag actctatctc aaaaaacaaa 300
caaagaaaac ttgaagtata gtatcctttt aaattttaaa tagataatag aaactggttt 360
ccccccattt aaaccagaat ttaagtttaa ctttatatat tcttgacagt ttggattttg 420
tccttcaacc tcataaaatt gggaatttaa gcatcacctg gttcgattta aatgcaatgt 480
agaatttgca ttaaaatact acattaaagc ctcagatttg tagtagctaa cagcacttct 540
atgtatgtgt cagggactgc tctaaatact tcatatatat taactcctct attctgtact 600
tctgttcccg ttttatacag caggaaattg aaacactgag aggttaagta actaaagtta 660
cagagctaga gtgacaggag taaagcttca actcaggcaa cccagacttc cagagttctg 720
atctccacta ctaagctgct agcatagctt ttctggtaac tatttttaat tcaaatataa 780
ttcgagtgat ctatctaaca agtcatcact ctgacaactc agtgacttgt aatgtaaaat 840
tattcattgt aattcattta atattattgt ttctctgtgc tgcaaaaatc atagcaatcg 900
agatgtaatt tattactctc cctcccacct ccggcatctt gtgctaatcc ttctgccctg 960
cggacctccc ccgactcttt actatgcgtg tcaactgcca tcaacttcct tgcttgctgg 1020
ggactggggc cgcgagggca tacccccgag gggtacgggg ctagggctag gcaggctgtg 1080
cggttgggcg gggccctgtg ccccactgcg gagtgcgggt cgggaagcgg agagagaagc 1140
agctgtgtaa tccgctggat gcggaccagg gcgctcccca ttcccgtcgg gagcccgccg 1200
attggctggg tgtgggcgca cgtgaccgac atgtggctgt attggtgcag cccgccaggg 1260
tgtcactgga gacagaatgg aggtgctgcc ggactcggaa atggggtagg tgctggagcc 1320
accatggcca ggcttgctgc ggggggaggg gggaaggtgg ttttccctcg cactgtctta 1380
aaccgatggc ctttccttgg cacaggtccc actgcagcat gccaaacgag gaggcagggg 1440
cgtcgtcccc ccgcccccca ctgcagcact ggagatggat ttcctgtact tcggatccag 1500
ggtttttgac agaagaggaa gaagggggag gggtagaagt gttaagggga gtctgctgag 1560
aaaagctgtt tttgaagcca gaaggggttt ttgtttttat aatgccattt gacagagtgg 1620
aataacagta tctaaggaaa cgggtagagg acaacaaaga atggagcata ttcatggcga 1680
ggagcaaaag ctctacccca ttgaaaggct tcttttcctc cctggcgaca aggacacatg 1740
cattggtggc caaaagagag aggagacaaa accgctgcag atggctgatg tgaatctagt 1800
ggaaagagct actggggatg agagaaagag gaggaggcag gtactgcaga gcgtgagtgg 1860
tggtgttggt tggtgaaata ctggtcacca gtagtgtgcc tgcttttgta aaacatctaa 1920

gtaaactccc tgtgaacagg gtggcaaaca gataccagtg tctttgttag ttacaaaatg 1980
cagtggtagt ggctttttgc ggacgactgc agcagtgctt tttctccctc tgttaggccg 2040
aaaagacaac tgcagaggaa taagaaacct tgcagcaaat gctggggtag aagcccattt 2100
acaagaagcc atagtttata aatgcagcct gaacagcaga aaaaaaatta ctgtttttta 2160
aagtaggaat aatgtcaggc tatgaatgtt ttgtcattgg aatgtattgg acactttgat 2220
tctacatcac gaaagtgatg ctcaaattct ttgatttaac ataaatccta tacgaaatct 2280
taataaatta tgtatgaaac agtggatctt ttcttttgtt agtgaagctt ttatgccatt 2340
aattaggtca ttcaagagca aaccacttta caacgtaaat tactttgtca aaaattatgg 2400
tgaacaaatt tttgtaggcc taatatttaa gacctatagt taagtaattt tatatttctc 2460
tttggttgct tttagataac actgaataaa tatttaagat attaattcag tgtgcaaata 2520
ttttaaatta aagcaacatg gcttttcttc tagatgtatt tctgtttagt gagtactcat 2580
gagatatact cttgatataa agtgtttttc attgagcttt tttttccttt acctaaatgt 2640
aaaagcctat ctttatgcat acttatagta gccagcctgc cacacctccc cactccctga 2700
acaaggatgg cagtggcttt gtaaagccct ccagggtcag tgacagtgtc tcttaaataa 2760
tgtttatgta atagaaagtc ttaagatgac ctattatatt gtttcagtaa tattttcaag 2820
ataatgcgga attgggctgg cttagataaa taagactaca taagtttttc actgataaat 2880
ttaaatagtt ctttaaaaaa ttatttctgc tttaagaatt acttgatcat ggatatcagg 2940
gttaatagta cttaggagcc gggcgtggtg gcccatgcct gtaatacaag ctaaacagga 3000
ggctgaggga agctgaggca ggaggatcac ttgagccttg gcgttcaaga ccagcctggg 3060
caaccaagca agaccctatc tcaaaaaaaa gtacttagag tctctttttt aaaatctgat 3120

<210> 13
<211> 2501
<212> DNA
<213> Homo Sapiens

<400> 13

tcacacggcc agaaagggtc caggcggttc ccggcgcttt tccagccctt gttttcatgg 60
cgcaccctcc cgccagccgc ccccctccgc actccgtcgt ccgcccggcc cggccgcgtg 120
cggttccccg ggagccccca ccccgtcgcg gaccccagcg accaccaagt ccgcacgcgg 180
cctgccgcag gcctgagcag aaggccccgc gcacacccac cgcgccgcgg ccgcgcggga 240
ggcctgtgcc gcccgcgcca cccactggcc gggccccgcg ggcgcagcgg agcgggcggg 300
tggccggccc ggacgcgccc tccccggccg cggccccgcg cgccatgtgc ccccggcggg 360
acgcgccact cccgggcctg ccgcggcgcc tttaacccgg gccagggagc ggggcggagg 420
gggcggtcgg gtggctcaga ggagggctct ttctttcttc tttttttgaa tgaaccgtgt 480
gacgttacgc acaggaaacc ggtcgggctg tgcagagaat gaagtaagag gacaggcacc 540
acagccccgc tcccgccccc ttcctcccgc gcccgcccct ccgcgccgcc tgcccgcccg 600
cccgccgcgc tcccgcccgc cgctctccgt ggccccgccg cgctgccgcc gccgccgctg 660
ccagcagaag gtgccggggc tccgggccct ccctgccggc ggccgtcagc gctcggagcg 720
ggctgcgcgg cgggagctcc gggaggcggc cgtagccagc gccgccgcgc aggaccagga 780
ggaggagaaa gggtgcgcag cccggaggcg gggtgcgccg gtggggtgca gcggaagagg 840
gggtccaggg gggagaactt cgtagcagtc atccttttta ggaaaagagg gaaaaaataa 900
aaccctcccc caccacctcc ttctccccac ccctcgccgc accacacaca gcgcgggctt 960
ctagcgctcg gcaccggcgg gccaggcgcg tcctgccttc atttatccag cagcttttcg 1020
gaaaatgcat ttgctgttcg gagtttaatc agaagaggat tcctgcctcc gtccccggct 1080
ccttcatcgt cccctctccc ctgtctctct cctggggagg cgtgaagcgg tcccgtggat 1140
agagattcat gcctgtgccc gcgcgtgtgt gcgcgcgtgt aaattgccga gaaggggaaa 1200
acatcacagg acttctgcga ataccggact gaaaattgta attcatctgc cgccgccgct 1260
gccttttttt tttctcgagc tcttgagatc tccggttggg attcctgcgg attgacattt 1320
ctgtgaagca gaagtctggg aatcgatctg gaaatcctcc taatttttac tccctctccc 1380
cgcgactcct gattcattgg gaagtttcaa atcagctata actggagagt gctgaagatt 1440
gatgggatcg ttgccttatg catttgtttt ggttttacaa aaaggaaact tgacagagga 1500
tcatgctgta cttaaaaaat acaagtaagt tctctgcaca ggaaattggt ttaatgtaac 1560
tttcaatgga aacctttgag attttttact taaagtgcat tcgagtaaat ttaatttcca 1620
ggcagcttaa tacattcttt ttagccgtgt tacttgtagt gtgtatgccc tgctttcact 1680
cagtgtgtac agggaaacgc acctgatttt ttacttatta gtttgttttt tctttaacct 1740
ttcagcatca cagaggaagt agactgatat taacaatact tactaataat aacgtgcctc 1800
atgaaataaa gatccgaaag gaattggaat aaaaatttcc tgcatctcat gccaaggggg 1860
aaacaccaga atcaagtgtt ccgcgtgatt gaagacaccc cctcgtccaa gaatgcaaag 1920
cacatccaat aaaatagctg gattataact cctcttcttt ctctgggggc cgtggggtgg 1980
gagctggggc gagaggtgcc gttggccccc gttgcttttc ctctgggaag gatggcgcac 2040
gctgggagaa caggtacga taaccgggag atagtgatga agtacatcca ttataagctg 2100

tcgcagaggg gctacgagtg ggatgcggga gatgtgggcg ccgcgccccc gggggccgcc 2160
cccgcaccgg gcatcttctc ctcccagccc gggcacacgc cccatccagc cgcatcccgg 2220
gacccggtcg ccaggacctc gccgctgcag accccggctg cccccggcgc cgccgcgggg 2280
cctgcgctca gcccggtgcc acctgtggtc cacctgaccc tccgccaggc cggcgacgac 2340
ttctcccgcc gctaccgccg cgacttcgcc gagatgtcca gccagctgca cctgacgccc 2400
ttcaccgcgc ggggacgctt tgccacggtg gtggaggagc tcttcaggga cggggtgaac 2460
tgggggagga ttgtggcctt ctttgagttc ggtggggtca t 2501

<210> 14
<211> 6104
<212> DNA
<213> Homo Sapiens

<400> 14

tagccctgaa gcaggcagac atgcccatat cttcctctga gtcagtgcct ggatccaggg 60
gaccagacct cttacctgac atggcagaga gataagctat gtttcaggtc ctcaaaagga 120
gcctggaaaa tataattaat tcaagccaaa tactcttact attcccttgc atatacaaca 180
aagataaaaa tatctatctt attaaactgt tgagagtaag aaagataata tacacacagt 240
gcctggcaca cggatggaac ttagttgatg gtagttataa tatttataat atcttttctt 300
tgtttcaatt taatatgatt tatatgactt ttcatactta ctgcacatgc aacatctttt 360
agataattct atatctaggt tattagtaaa acattgatat cctcttcagt atttatacag 420
atgcccctct tctaaagtct tcagcaaaat tcccactggg gaatttcagc tgtctctcac 480
tctttttttt tttcactttt atttccatat taaaggtttt tatttttaag gtatacaata 540
tgatattttg atacatgtat actttgtgaa atgattgcaa caatcaagtt aattaacata 600
tcattacctc acatagtttc ctgtgtgtgt gtgtgtgtgt gtgcgcttag agctcttgag 660
atctactctc tttgcaaatt tcaagcatat gttaactata gtcaccaggc tgtatattag 720
gtctccagaa cttactcatc ttataactga aaggttgtat cctttaacta gtatttctct 780
tttaccccca accgcagccc caaataatca ccattctaac ctttgttact gagttcgact 840
tgttttttga gattctacat ataagtgaga tcatgtatta cgtgtctttc tgtgcctcac 900
ttattttgtg tgtcataata ttccccaggt tcagccatgt tggcacaaat gacaatattt 960
ccttcttctg aataatactg ctgactaata ttccattgtg tgtgtgtgat aaacacacac 1020
cacattttct ttaataattc attcatcaat ggacacttag gctgtttctg tatcttggtt 1080
attgtgaatc tcgctgtctc tttggcagcc atttctgtac tcccttcccc agaagcagct 1140
ttactgcagt ctaaaagtgt cttaggaaaa tgattcagaa gcaactactt ctcttggcat 1200
gttcttcagt aaacacgatg atggaaagct ttgatttctg cccttcttta aacagtgcaa 1260
tcgattttca gttgggattt tttccagtta atttgatagc tccacgtttc taccaggctg 1320
aagaaaatgt atgtgttgtg ggctgttgta ttactgttgc tggtggtgct gttgctactg 1380
ctgagatttt gtggttgagt ccatattata aaggaattgt agagcagtaa taaatttaac 1440
ttgagctctg aactcaagcc ttcaggtgtg acttttttaa tcacgtggaa attcacgtaa 1500
gaattttcaa ccagacttca tgtttatttt tatttcttaa gcaaagggta caggaatctg 1560
gtctaaacga atgggaacaa acattacagt tagactttat tggagtgttg taaaaatgtt 1620
tttttccaaa atcagattgt ttcaaaactg accctggttt ccagattttt aagttaggaa 1680
gtaagacagt aaaaatcaag ggtcaaatag tcaagcgact atttttttaa aatcagcatt 1740
ttccttggcg acttccgtta gaccatgctc tgtttagtac tgaaactcag agaagttcca 1800
ggaatagcgg gatgaaatct aagtgatccg ctcacgggaa gctctaccct tccttcgctc 1860
gctcagtaaa tccgggatgg gctcagagaa tgacttcgca ttttgcacca gaagatctca 1920
gtctttagag agaaccctag acgaaatcct tacttccctg cctcattcct gaaaacttct 1980
acctgggaac cgacttgctg tgacaaataa aatgggagga gtctggctga atagtttgtg 2040
gatttcggaa agcaaagcga aatgccgctc taactaggtc cgaattttct gcaagctacc 2100
ttgtgaaact ggtgaatttg ccagaatgaa ttttcattca tttaactttg gcggatcgtc 2160
tactagggtg cctggcatgg tgctaattgc tggagaaaat gattttcaaa caaaaacgaa 2220
aaaaactttc ggcccccagc aaggagccag gcaattccta caaactcaag gagtgcttga 2280
gaggtgagtt aatgaccgtc cctcctgtcc taaagagaca gacttttaca gtcagtcttg 2340
aaactgctcg gggctacact agcgccctct ggacgcagac tctggttagc gccccacggc 2400
accagccttt gatttgtagc tgccaaagat ttgatagtgg ggagtacgtg tctattctga 2460
aggctgaaga cacacaccac agtcaggagt agaattccga cccctcaaag tgacatggcc 2520
acaaaatctg cgtctactgg ctttcatttt gcggtcaccg tggcacgggc gaaagctatg 2580
aactctctct ttaggaaaac acacatcaca gacacacaca cttacaaacg gtttcaggag 2640
gttcttatgt tagaaacctc tggtcttgga aaaaaatcat ttccgctgaa gtatgtggta 2700
gctgagcgca aatacccttc tgctacagcc ccagtggcag aggcccaagc actacgcccc 2760
actcagtgcc cgcactgcgg ccttcgcaac tcttcaagca gctctgcacc gaaaacccag 2820
gccggagatc cgagcctaaa ctgctgaaaa gctccaaggc tccggacagg gtctcagcag 2880

gggttatctt ccttcaggat ccggggctca ttgtgcccaa gatccgccat ccaagccctg 2940
ctctgcgcgc acgttgcctg tttcacgctc tgcgctgaca cgcgccggtg tcctcccggg 3000
ccagttccag tcccgggtcc tgtggccgcc ctgccggcga ccctgcggag agcgagtctt 3060
agatacccag tccccagccc cgagttgtta ttccctcgct gtagttaaga aggaggagat 3120
caattaaggg catcttagaa gttaggcgtt cccgctgcct cctttgagca cggaggccac 3180
caacccccta gggggaagag atgtagcgcg aggcaggggt gtcgtgctaa gaaatttcga 3240
cgcttctggg gactgaggac aaaggtgcgg acacgacccc ggggtacctg gagttccgtg 3300
actcgcgcca cggacggcac acctaggggc taatttctgc tctgcctcaa agaacctcaa 3360
gctagagtcc ttgcctccgc ccacagcccc gggatgccgc tgctgcgctc accgcacagg 3420
cagcgcccgg accggctgca gcagatcgcg cgctgcgcgt tccaccggga gatggtggag 3480
acgctgaaaa gcttctttct tgccactctg gacgctgtgg gcggcaagcg ccttagtccc 3540
tacctctgct gagctgaacg ctcaggcaca gtggaactga aacccggttc tgcgggatgt 3600
gagagctgtt gaggtcacgc gtaattgggt gtgatggagg gcgcctgttc gtgatgtgtg 3660
caggtttgat gcaagcaggt catcgtcgtg cgagtgtgtg gatgcgaccg cccgagagac 3720
tcggaggcag gcttgggaca cgtttgagtg aacacctcag gatactcttc tggccagtat 3780
ctgtttttta gtgtctgtga ttcagagtgg gcacatgttg ggagacagta atgggtttgg 3840
gtgtgtgtaa atgagtgtga ccggaagcga gtgtgagctt gatctaggca gggaccacac 3900
agcactgtca cacctgcctg ctctttagta gaggactgaa gtgcgggggt gggggtacgg 3960
ggccggaata gaatgtctct gggacatctt ggcaaacagc agccggaagc aaaggggcag 4020
ctgtgcaaac ggctcaggca ggtgatggat ggcagggtag gaagggggag gtccagaggt 4080
ctggatggag gcttccgcat ctgtaccttg caactcaccc ctcaggccca gcaggtcatc 4140
ggccccctcc tcacacatgt aatggatctg aagagtaccc cgggacagtc cggggagatg 4200
gagattcgga aagtatccat ggagatctta cagaatcccc tgtgcggacc aggaaactct 4260
tgtagatccc tgcctatctg aggcccaggc gctgggctgt ttctcacaat attccttcaa 4320
gatgagattg tggtccccat ttcaaagatg agtacactga gcctctgtga agttacttgc 4380
ccatgatcac acaaccagga attgggccaa ctgtaattga actcctgtct aacaaagttc 4440
ttgctcccag ctccgtctct tgtttcccac gagccctggc cctctgtggg taataccagc 4500
tactggagtc agatttcttg ggcccagaac ccacccttag gggcattaac ctttaaaatc 4560
tcacttgggc aggggtctgg gatcagagtt ggaagagtcc ctacaatcct ggaccctttc 4620
cgccaaatcg tgaaaccagg ggtggagtgg ggcgagggtt caaaaccagg ccggactgag 4680
aggtgaaatt caccatgacg tcaaactgcc ctcaaattcc cgctcacttt aagggcgtta 4740
cttgttggtg cccccaccat cccccaccat ttccatcaat gacctcaatg caaatacaag 4800
tgggacggtc ctgctggatc ctccaggttc tggaagcatg agggtgacgc aacccagggg 4860
caaaggaccc ctccgcccat tggttgctgt gcactggcgg aactttcccg acccacagcg 4920
gcgggaataa gagcagtcgc tggcgctggg aggcatcaga gacactgccc agcccaagtg 4980
tcgccgccgc ttccacaggg ctctgctgga cgccgccgcc gccgctgcca ccgcctctga 5040
tccaagccac ctcccgccag gtgagccccg agatcctggc tcaggtatat gtctctccct 5100
ccctctccct ccattcgtca ttttctcact ccctttcctc ctctccctct ctctccgtta 5160
gtctcttcat cagatagtct ctgttagtcc gcgatttata ccaggctcgt gccctaggtt 5220
ggatcggaca gtctcaatcc cccggctcgc tcttcctgct cggctgcgga ctccagtctt 5280
actctctcgc actgcacaca ggcttaggcc agtctcggga cactcaggct ccccagggac 5340
cgcgcacaga gcctgaggca agagaaactt tccgcagacg gtgcgatcag ggacggcgtc 5400
tggagcccag cagtcccagg gaaattggtt cagaacctgg aacagagcgg atgggtggca 5460
aataggcacg acgactgagg gacaagcagc cctaaactgc aagccccagt cacaggctct 5520
gggagcaaag agagaaagtc ttgggtccca atttaagagt aaaacttggt tcctgcaggg 5580
gactgaggtg ccagcaggcg gagcttaggc tgagcactaa aattgattct tttatttctc 5640
agattccaat tgcagagata atctctgtgg atagatccca cccctcccac caaggtgtat 5700
gcaaaagccc cagaagagga ggacagctct gggtttcctt atgggatcag cttctgtgac 5760
aggggcctgg gagcatgtcc tccacagaga cctggctaac aggtttatat gtatctgtgt 5820
ggctttgggg agaagggtag gactgagacc tcccccactc agctctccct ctaggatcgc 5880
tcctccagct ctatcagact gtgcatgcta caggcccact gaacctccca gaagtccact 5940
gtgctgatga ggaaacaaaa aagaagagaa atataatgac cagtctgaag ccacaaagcc 6000
agagcgttag aagcaagaca gaaaaccagg ataggagcca aggtctgtgg gctcctagaa 6060
cttcgaactg gcgcaggtgg tttatctcat tcttcccttg caga 6104

<210> 15
<211> 5976
<212> DNA
<213> Homo Sapiens

<400> 15

agaggggcac ctcccacccc aatctgaacc ctatgactgc accccttgcc aggagatgaa 60

agggacaaga ctgcctgaag gattaatctt tagcctccag gcaggccagt ggccaaaaca 120
cccaggaagt gaaagtctgt cctctgccat aaggaaacct ggagtctgtg atcccatcat 180
gcggggtctg ttatctgcca ggtgacaggg tctcttatct atctgcggtg atctgaaggg 240
caaggcctct ggccaaacat ttcttttccc cagtgatgcc tgaaattcca ctgttagcgc 300
tggatctcag caagatctca gagaaagctc aactcctgga gtttgggttt tccccagttg 360
agatgttagg aaagcaatgg gccttatcac ctgcacggac aggtttgtgt acagtctcag 420
gggattcagg gaccccacca gagcccatcc acagacacca ggttaaaagc cctggacagc 480
atttacaact cctttgaaag aagaaaagtc tttctctaga gccctcttta aagtgcaaat 540
ggccctggga ggttcgaaat gatgagctgg ccggcatgaa agaattttct ccagtagatg 600
caagagataa cctgaggagc tttagtggcg gggtgggggg aaggagagcc tgggggaggg 660
gaggctccag gcagctcaca atttttccta gaactggaaa gggaatgtat tgggccaaga 720
gggctaatca ctcacctttg cagagtgttt acaagaacct tatgaattaa gtgctatcaa 780
cattcccatt ttacagatga ggaaaccaag atggtggaga taaaatgact tatgcttctc 840
aactaatggc agagccagaa gtgaatccag gctggagtga agtggctcca gtgctcaaat 900
gatcctccta cctcagcctc cagagtagct gggactacag gcatgcactg ccatgtccag 960
ctaattcaaa ataatatttg ttggggagat ggggtttcac catgttgccc agactggtct 1020
caaactcctg ggctcaagta atcttctcac ctcccaacta gcctcccaaa gtgctgcgat 1080
tacagttgtg agccaccagg cctgcttagg tatccctgtt tgatacaaga gaaaacagag 1140
ggcaggcaca gtggctcaca cctgtaatcc cagcactttg ggaggctgag gtaggtggat 1200
cacttgaggt caggagtttg agaccagcct ggccaacatg gcgaaatccc gtctctacta 1260
aaatacaaaa attagccaga catggtgacg tgtgtctgta atcacagcta ctcaggaggc 1320
tgaggcacaa gaatctcttg aacctgggag gcagagagtg cagtgagccg agatcgcgcc 1380
attgcactcc agccggggca acagagcaag accttgtcac taaataaata aataggaaac 1440
agagaagcaa ttcagtgtgt tgcgaaagtc acacagcacc tgtcttccac aaggagcccc 1500
tctgtgttcc cgcctcaccc tggacacctg atctactgtc tttggagctt ggagaaggac 1560
ttgccgagaa gtctgaagaa tgcatcaggc ctgacctgac tttgctgccc ctaattgttt 1620
tctaaaatcc tttttattga attatcagag tgtaatggaa gagtcgatga gagtcgctgc 1680
agagttacga ggccaaggct tgctgggtag aagaaagtaa agccctaaag gctcccaggg 1740
cccagcgatg gtataaaata tgtattttta cataactgag aggaaaaaaa taagcagaca 1800
gtacagcccc atgggggaaa cgcctctttc tgtatttcca ttatcttgtt caaggcacga 1860
tcctagtggg aaaggaaata ccggagcctt ctccctctga gcctcctggt gggttaagag 1920
cagagggagc caaaatacga catccaaaag acctgattgg aaactcatta aaattgaggg 1980
gacaggttgt tggggggggc ggtgctgggc tgcccagaaa tcgtctgcat ttgagaaatt 2040
acttgatgga ctcagaggca cctttagctc tgggtgaaat attccaaaca tggctttccc 2100
caggaggaag cgaaataggg gctgggcctt ttgaaagagt aacccctact ccaaatattt 2160
attgagcagt tgctatgatg agccaagaac tctgcttagc gattgttttt gtattagctc 2220
atgccaattc acttatacaa aggagaacac tgacttcccc aaggtcacag ggctagtgag 2280
tggctgactc cacttaatgc tgtgtagttc taaccatgct tgaaggataa agggaaagga 2340
ggtgcccttg tcggggtaga caagccaccc agacgtggct ggccacaggg agtcagacag 2400
ggaagttgtc taacctaaca tttgcctgtc tattctgttt ttgtcttttg atggaggacg 2460
gcgggtgctg gagggccact ggcccaggac ctgagacctt tggcccctat ctattctatt 2520
tttgatccta aaattatttc actggggaca gaattctctg gagaaggtga tggtggccat 2580
ggtgtggctg tggcattaaa agccccactc tgggggtcag aagatcctgg gttttggtct 2640
ttggctttga cattgacctg cctatgtgac attgaacaag ttatttgcaa atcataggtt 2700
tgggtgaact ctaaaggtct cttccaggtc aacatcaaaa aacagactga aaactgcagc 2760
tccagctgga agactggacc cacatgagat gaagctgatt gttagaagaa aggccctgga 2820
atcagagagg ttggttccag tcctgcgcca cctctcattg gcctcaatct ctctaagctt 2880
cagttgccca tctatcatat gggaataata atacagctct tgggttgttt tgaaatgagc 2940
ttaaatgagc tggtcagtgg ctgggcacgg tggctcatgc ctgtaatccc agcactttgg 3000
gaggctgagg caggtggatc atctgaggac aggagttcaa gaccagcctg gccaacatga 3060
tgaaaccctg tctctactaa aaatacaaaa attagccagt cgtggtggcg catgcctgta 3120
atcccagcta ctcaggaggc tgatagagga gaaccgcttg aacccaagag gcgaaggttg 3180
cagtgagcca agaacacacc attgcactcc agcctgggcg acagagcagg actccgtctc 3240
aaaaaaaaaa gagctggtca gtgtcaaatg cttagcacag agactggcac agtaatcttc 3300
aatgtccagc acctattgtt actatttttt tttttttttt ttttttgaga cagagtcttg 3360
ctctgtcgcc caggctggag tacagtggcg cgatctcggc tcactgcaag ctccacctcc 3420
caggttcatg ccattctcct gcctcagcct cccgagtagc tgggactaca ggcgcccacc 3480
accacgcctg gctaattttt tgtattttta gtagagacgg ggtttcactg cgttagccag 3540
gatggtctca atctcctgac ctcgtgatct gcccgcctcg gcctcccaaa gtgctgggat 3600
tacaggcgtg agccaccatg cctggcccta ttgttactat ttttacccct cacttctgta 3660
cagagcattt atggctcaag aaacatttgt cattttaatt gtatgggagt cccacaacag 3720
catagggaga catttctgat cattattccc attaggaggg tggagaaact gaggctttgg 3780
gaggtggtcc tgacctaggg aatcaatttg ctgactcact aacccatgaa gctctacagt 3840

taaaaaagac tagattaaaa aatgagaact cagtaaaggg gctgaggcag gaggatcgcc 3900
tgagttcaga aatttgagat cagcctcggc aacatagtga gatcccctct ctagaaaaat 3960
tttttaaaaa attaggccgc tcgaggcaga gtgcagtggc tcacgcctgt aatccaacac 4020
ttcaggaggc tgaagagggt ggatcacctg aggtcaggag ttccagacca gcctggccaa 4080
catggtgaaa ccccgtctgt actaaaaata caaaattagc cggtgtggtg gcacacgcct 4140
gtagtcccag ctactcaata ggctgagaca ggagagtctc ttgaacccgg caggcggagg 4200
ttgcagtgag ccgagatcgt gccactgcac tccagcctgg gcaagacaga gcgagactcc 4260
gtctcaaaaa atacaaacaa aacaaacaaa caaaaaatta ggctgctagc tcagtggctc 4320
atggctcaca cctgaaatcc tagcactttg ggaggccaag gcaggaggat cgcttcagcc 4380
caggagttcg agaccaggct gggcaataca gggagacaca gcgcccccac tgcccctgtc 4440
cgccccgact tgtctctcta caaaaaggca aaagaaaaaa aaattagcct ggcgtggtgg 4500
tgtgcacctg tactcccagc tactagagag gctgggccca gaggaccgct tgagcccagg 4560
agttcgaggc tgcagtgagc tgtgatcgca ccactgcact ccagcttggg tgaaagagtg 4620
agaccccatc tccaaaacga acaaacaaaa aatcccaaaa aacaaaagaa ctcagccaag 4680
tgtaaaagcc ctttctgatc ccaggtctta gtgagccacc ggcggggctg ggattcgaac 4740
ccagtggaat cagaaccgtg caggtcccat aacccaccta gaccctagca actccaggct 4800
agagggtcac cgcgtctatg cgaggccggg tggcgggccc gtcagctccg ccctggggag 4860
gggtccgcgc tgctgattgg ctgtggccgg caggtgaacc ctcagccaat cagcggtacg 4920
gggggcggtg cctccggggc tcacctggct gcagccacgc accccctctc agtggcgtcg 4980
gaactgcaaa gcacctgtga gcttgcggaa gtcagttcag actccagccc gctccagccc 5040
ggcccgaccc gaccgcaccc ggcgcctgcc ctcgctcggc gtccccggcc agccatgggc 5100
ccttggagcc gcagcctctc ggcgctgctg ctgctgctgc aggtaccccg gatcccctga 5160
cttgcgaggg acgcattcgg gccgcaagct ccgcgcccca gccctgcgcc ccttcctctc 5220
ccgtcgtcac cgcttccctt cttccaagaa agttcgggtc ctgaggagcg gagcggcctg 5280
gaagcctcgc gcgctccgga ccccccagtg atggagtggg gggtgggtgg gtgaggggcg 5340
agcgcggctt tcctgccccc tccagcgcag accgaggcgg gggcgtctgg ccgcggagtc 5400
cgcggggtgg gctcgcgcgg gcggtggggg cgtgaagcgg ggtgtagggg gtggggtgtg 5460
gagaaggggt gccctggtgc aagtcgaggg ggagccagga gtcgtgggga cgatcttcga 5520
gggaaggaga ggggcatccg tagaaataaa ggcacctgcc atgccaagaa aggtcgtaaa 5580
taggagtgag ggtcccgggg ataagaaagt gaggtcggag gaggtgggag cgcccctcgc 5640
tctgaggagt ggtgcattcc cggtctaagg aaagtggggt actggagaat aaagacatct 5700
ccaataaaat gagaaaggag actgaaaggg aacggtgggc taggtcttga gggggtgact 5760
cggcggcccc ctcccgggag ttcctggggg ctcggcggcc gtaggtttcg ggtgggggga 5820
gggtgacgtc gctgcccgcc cgtcccgggg ctgcgggctg ggtcctcccc ccaatcccga 5880
cgccgggagc gagggagggg cggcgctgtt ggtttcggtg agcaggaggg aaccctccga 5940
gtcacccggt tccatctacc tttcccccac cccagg 5976

<210> 16
<211> 2501
<212> DNA
<213> Homo Sapiens

<400> 16

cagaagtcct cccttagagt gtgtctgggt acacattcaa gtgcatggtt gcaaactttt 60
ttttttaaag cactgaatag tactagacac ttagtaggta cttaagaaat attgaatgtc 120
gtggtggtgg tgagctagaa gttataaaaa aaattctttc ccaaaaacaa caacaaaaag 180
aattatttca ttgtgaagct cagtaccaca aaaatttaaa taattcatta caagccttta 240
ttaaaaaaaa ttttctcccc aaagtaaaca gacagacaat gtctagtcta tttgaaatgc 300
ctgaaagcag aggggcttca aggcagtggg agaaggtgcc tgtcctctgc tggacatttg 360
acaaccagcc ctttggatgg tttggatgta taggagcgaa ggtgcagaca gcagtggggc 420
ttagagtggg gtcctgaggc tgtgccgtgg cctttctggg gtttagccac aatcctggcc 480
tgactccagg gcgaggcagg ccaagggggt ctgctactgt gtcctcccac ccctacctgg 540
gctcccatcc ccacagcaga ggagaaagaa gcctgtcctc cccgaggtca gctgcgttag 600
aggaagaaga ctgggcatgt ctgggcagag atttccagac tctgagcagc ctgagatgtc 660
agtaattgta gctgctccaa gcctgggttc tgttttttag tgggatttct gttcagatga 720
acaatccatc ctctgcaatt ttttaaaagc aaaactgcaa atgtttcagg cacagaaagg 780
aggcaaaggt gaagtccagg ggaggtcagg ggtgtgaggt agatgggagc ggatagacac 840
atcactcatt tctgtgtctg tcagaagaac cagtagacac ttccagaatt gtcctttatt 900
tatgtcatct ccataaacca tctgcaaatg agggttattt ggcatttttg tcattttgga 960
gccacagaaa taaaggatga caagcagaga gccccgggca ggaggcaaaa gtcctgtgtt 1020
ccaactatag tcatttcttt gctgcatgat ctgagttagg tcaccagact tctctgagcc 1080
ccagtttccc cagcagtgta tacgggctat gtggggagta ttcaggagac agacaactca 1140

ctcgtcaaat cctccccttc ctggccaaca aagctgctgc aaccacaggg atttcttctg 1200
ttcaggtgag tgtagggtgt agggagattg gttcaatgtc caattcttct gtttccctgg 1260
agatcaggtt gccctttttt ggtagtctct ccaattccct ccttcccgga agcatgtgac 1320
aatcaacaac tttgtatact taagttcagt ggacctcaat ttcctcatct gtgaaataaa 1380
cgggactgaa aaatcattct ggcctcaaga tgctttgttg gggtgtctag gtgctccagg 1440
tgcttctggg agaggtgacc tagtgaggga tcagtgggaa tagaggtgat attgtggggc 1500
ttttctggaa attgcagaga ggtgcatcgt ttttataatt tatgaatttt tatgtattaa 1560
tgtcatcctc ctgatctttt cagctgcatt gggtaaatcc ttgcctgcca gagtgggtca 1620
gcggtgagcc agaaaggggg ctcattctaa cagtgctgtg tcctcctgga gagtgccaac 1680
tcattctcca agtaaaaaaa gccagatttg tggctcactt cgtggggaaa tgtgtccagc 1740
gcaccaacgc aggcgaggga ctgggggagg agggaagtgc cctcctgcag cacgcgaggt 1800
tccgggaccg gctggcctgc tggaactcgg ccaggctcag ctggctcggc gctgggcagc 1860
caggagcctg ggccccgggg agggcggtcc cgggcggcgc ggtgggccga gcgcgggtcc 1920
cgcctccttg aggcgggccc gggcggggcg gttgtatatc agggccgcgc tgagctgcgc 1980
cagctgaggt gtgagcagct gccgaagtca gttccttgtg gagccggagc tgggcgcgga 2040
ttcgccgagg caccgaggca ctcagaggag gtgagagagc ggcggcagac aacaggggac 2100
cccgggccgg cggcccagag ccgagccaag cgtgcccgcg tgtgtccctg cgtgtccgcg 2160
aggatgcgtg ttcgcgggtg tgtgctgcgt tcacaggtgt ttctgcggca ggtgaatgac 2220
gggcgtgggt cggtgcgcgc tcggcttgcg cacacggtgt ctctaagtgc gcgggtgacg 2280
agagtcggga tgtgccggag accccggggc ggagagcggg attacaagta caggaatccc 2340
tggtcacgct ccccgcccct ggaaacccag ctggggcgag ggagggcgtg gacgggaccg 2400
ttctgggagc tcgcctttgg ctgcggttgg ctccaggccc caggcgcagt ttgctcgcgg 2460
cgtggggatg aagtccgtgt ccctggaggg gcccaggaag g 2501

<210> 17
<211> 3486
<212> DNA
<213> Homo Sapiens

<400> 17

aactaaagag gatgagacct cttgcttgat cctaggtgaa ttcttttctg tcagttaggt 60
aggaagtcct gacttgaaaa ctagttctgg gcactgcccc ctttactgtt ctctgggtat 120
caacccctgt ccttcaattt tagttgaact agtggatggt gataccacag gctcaagaca 180
gctgcattta aatatcagtg accacaggcc acatcaagga aacatctgca ggcaacccag 240
ggcctgggaa ggagccattt tcagtcactt gtaagacagc aggacctgca gactacagca 300
caatcaaact cagacaaaac cctgaaccag tgagaaccat taggaaggaa aggaacagaa 360
aatgaaccaa cctgagtgtt aggagacttg catctagtcc tgactccggt accaaccgaa 420
tgcatgtccc tggacaggaa acctctctga gtctcgattt cctccgtggt aaaaaggaga 480
gggttaaacc acagggtccc gagggtccct tccagctgtc acattctgga gcgtatgaga 540
tgaggtaggc acacaaagtg gacaagatgt ggctaagaaa acaagctaca catcaagctc 600
atctgtagca taggtgctta agaaaacttt gctgctgtgt aatattagaa cggaaggttg 660
gtttccagta aaatgcatta actttggctc aaaccaagat gatgggtacc gggcatgggg 720
gtggggaggc agttgaagat ccactgagct ttgtctcagg gcagccctgc tcatcgtcct 780
actttacctt ccaccacggt gctcaagccc acactgagag agaaatttcc agctgcaaaa 840
gggagaagag aaacgctgga atactagtat cggacgttag gacatggttg tggtgtttta 900
aaaatcattt catcatctgg agtttgaccc cgaggggagt attttcaccc ttcagccctc 960
tgaaagcatt cactagcatc tgaatattgt tctgagtttg ttggagcagt gaaatctggt 1020
gagagagaag ggtggaggaa ggaaggagct gttgtatttg gcggctggac tcaggtagag 1080
gaaactgcta caatcccggg aaagaacaga aaagtagaaa gggacgagtt cccacacgca 1140
gccaatgtcc atggccttaa ctgtgcttgg gaaggaagat cctgggccag gggtgtaccc 1200
tcgtttttca aaactaaacg tgtctgagac agctacaaag tttattaagg gacttgagag 1260
actagagttt tttgtttttt ttttttaatc ttgagttcct ttcttatttt cattgaggga 1320
gagcttgagt tcatgataag tgccgcgtct actcctggct aatttctaaa agaaagacgt 1380
tcgctttggc ttcttcccta ggcccccagc ctccccaggg atggcagaaa cttctgggtt 1440
aaggctgagc gaaccattgc ccactgcctc caccagcccc cagcaaaggc acgccggcgg 1500
gggggcgccc agccccccca gcaaacgctc cgcggcctcc cccgcagacc acgaggtggg 1560
ggccgctggg gagggccgag ctgggggcag ctcgccaccc cggctcctag cgagctgccg 1620
gcgaccttcg cggtcctctg gtccaggtcc cggcttcccg ggcgaggagc gggagggagg 1680
tcggggctta ggcgccgcgg cgaacccgcc aacgcagcgc cgggccccga acctcaggcc 1740
ccgccccagg ttcccggccg tttggctagt ttgtttgtct taattttaat ttctccgagg 1800
ccagccagag caggtttgtt ggcagcagta cccctccagc agtcacgcga ccagccaatc 1860
tcccggcggc gctcggggag gcggcgcgct cgggaacgag gggaggtggc ggaaccgcgc 1920

cggggccacc ttaaggccgc gctcgccagc ctcggcgggg cggctcccgc cgccgcaacc 1980
aatggatctc ctcctctgtt taaatagact cgccgtgtca atcattttct tcttcgtcag 2040
cctcccttcc accgccatat tgggccacta aaaaaagggg gctcgtcttt tcggggtgtt 2100
tttctccccc tcccctgtcc ccgcttgctc acggctctgc gactccgacg ccggcaaggt 2160
ttggagagcg gctgggttcg cgggacccgc gggcttgcac ccgcccagac tcggacgggc 2220
tttgccaccc tctccgcttg cctggtcccc tctcctctcc gccctcccgc tcgccagtcc 2280
atttgatcag cggagactcg gcggccgggc cggggcttcc ccgcagcccc tgcgcgctcc 2340
tagagctcgg gccgtggctc gtcggggtct gtgtcttttg gctccgaggg cagtcgctgg 2400
gcttccgaga ggggttcggg ctgcgtaggg gcgctttgtt ttgttcggtt ttgttttttt 2460
gagagtgcga gagaggcggt cgtgcagacc cgggagaaag atgtcaaacg tgcgagtgtc 2520
taacgggagc cctagcctgg agcggatgga cgccaggcag gcggagcacc ccaagccctc 2580
ggcctgcagg aacctcttcg gcccggtgga ccacgaagag ttaacccggg acttggagaa 2640
gcactgcaga gacatggaag aggcgagcca gcgcaagtgg aatttcgatt ttcagaatca 2700
caaaccccta gagggcaagt acgagtggca agaggtggag aagggcagct tgcccgagtt 2760
ctactacaga cccccgcggc cccccaaagg tgcctgcaag gtgccggcgc aggagagcca 2820
ggatgtcagc gggagccgcc cggcggcgcc tttaattggg gctccggcta actctgagga 2880
cacgcatttg gtggacccaa agactgatcc gtcggacagc cagacggggt tagcggagca 2940
atgcgcagga ataaggaagc gacctgcaac cgacggtaat gaccctttcc caaccataga 3000
atgtgtttgg ggccccgctt tgcctgctgg agggtgttaa ccttagcttg cttttcggcg 3060
tattctgatt tagctttggg agagctaact ttattggtct taggtgttca gtgctacctg 3120
gcccactgct tgtctgtttg tgacttttaa gtcagaaact ggagatggta agatccgata 3180
atttccctaa cttaatacat cgcggtccct ctcactagca actcctaggt atgtgacaaa 3240
gttgggatgt ttatcaacgg tccgcctcct ggctagggaa agagctctgg ggcggagaat 3300
gcactttctg ttttttgaaa acaacctcat tttgtgccct taaaagccac tggggatgac 3360
ggatccagga ttgtgggtgg aggtagtggg tttttcatcc cctgactatg ggccaacttt 3420
ctgccagcca ttgttttttc taataaagat tgtgtgttct ttttaaaaat ttcccctgcg 3480
cttaga 3486

<210> 18
<211> 8666
<212> DNA
<213> Homo Sapiens

<400> 18

ctgcagggct ttctttagtt taaagaaagt aatcaggtgt ccctactgtg tcatgatctc 60
caccctcagc tgggttctcc agtctggttt taaagaacaa aacaaaaggc ttctctgtct 120
gagtcttact caacccatcc tctctactca taagaggtat tccaaacctt tacgattctc 180
aaacttccta accgaccatc ttattttcac tctgcaaaca agctaacctc ctcattcata 240
gaaggaagtg cctcaacttc ctccccgttc tgaccttttc tccctcccaa atctatgtat 300
ctcttgtgac aaaatctata accaccgctg tactttgagt tctatttctt cattattttt 360
gagggacctc aagtcctcaa aaatatccta tcttgcctgt gtacttaact tttcttttat 420
tcttttctaa ctttcccttc tcttcacttg gcacttgccc ttccaggtat atgtgtgctc 480
aggtctcctc caccttccat ctgcctcact tcatggcata gggccttgaa ctatcacaac 540
caagctatga aagagtagtc aacgcagtgt ccccacttcc ttgccatccc attatcctag 600
tttttctttt ggctctctga ggagtccttc acaggctggt tttcaggaat aagtctaaat 660
gaattacttt cagttttcct aaacttctat gcctttgcac atcctcttac ctctgcctag 720
aatatctttc tccttctttt ccatctttaa actctcacat cattcttcaa gactgggatc 780
agctctcagc atccggaagc ctttgcctac tagagacaaa tgagaatgag tttggtcacc 840
ttttcatttt cttgtatcat tctgtgcttt attttgctct tctaagagca ttacatgctt 900
catttaatcc ctaaacaact gtttgaggca agtacagtta ttatcctaat catgcaaatg 960
agaaaacaga ggcccagaca tgttgagtaa ctttgataaa agttaaagaa ccaataagtg 1020
gaacagttga ggtttgaacc ctggcagtct gactgtagag atactatgtt tgacctactc 1080
ccctctgccc ccaccccatg tctgccctta gtttctgagc ttgttgaatg aatgaacagg 1140
tggtagtctt tttttgttat aagactgatc agaattaaga caggtttaaa tttcacgtgt 1200
agaattttca aaactgcaaa ggcagtgcaa atctaaaaaa agaatggcat tctcaggaaa 1260
gaggaaaagt aagtgtgaga ataataataa caataaccaa caaactttag taaatttagt 1320
aaatgtagta aatttttaca ttaaaagctt ttggacatac attatcatat tttatggcca 1380
catgaaatat attataatcc cattttgcac ataggaaatc tgagactggc ataaggagca 1440
cagagatcca ggactttata ttttcattct tctaggattt tgcacctcag gtcgatatgt 1500
atgagtaaac tgggagtata atgggctctt taacagaaaa actaggaaag ttttcccact 1560
attattaatt atttacataa tattttttta attttattat tatttatact ttaagtttta 1620
gagtacatgt gcacaatgtg caggtttgtt acatatgtat acatgtgcca tgttggtgtg 1680

ctgcacccat caactcatca tttagcatta ggtatatctc ctaatgctat ccctcccccc 1740
tcccccctac ataagattta taatggataa tggacttcaa tttctagagc aaaatggccc 1800
cacccaagga tgccataatc cttccagagc tctactgcaa gatatgagat atacatatct 1860
aaaacttgtt cttggtattt ccaaagcagt caacttttac acctgtttat aatgcatcca 1920
aatgttgttt ttatatggtt gcatctccca tcttcttcac caatagctat atatattttt 1980
cacaagagct gaaagagttc ttgatgtagg aatccatggt agagtttcag agaaatccct 2040
gaattcactg aaagttttat ctagaaatac atgtgcaagt gaacacatct tttttaaaaa 2100
aaatcattac ctactttctt ttttgagaag aaggtattta tttcaacaga ctcttgaagg 2160
agcctactct tcccactctc ccacccccat taagaaccac tgtaggccgg gcacgatggc 2220
tcatgcctgt aatcccagca ctttgggagg ctaaggtggg tggatcacct gaggtcagga 2280
gttcgagaca agcctagcca acatagtgaa accccgtctc tactaataat acaaaaatta 2340
gctgggtatg gcagcatgtg cctgtaatcc cagctactcg ggaggctgag gcaggagaat 2400
tgctcgaacc cgggaggcgg aggttgcagt gaaccgagag agatcgtgcg gtgccatttc 2460
actccagcct gggcaacaga gcgaaactcc atctcaaaaa aacacacaaa acaaacaaac 2520
aaaaagaaag aaccattgta ttagtgatgg aaatgtgttc cctccctccc atcctggcaa 2580
ccactttctt cctcctccat cataaaatat cttaaactaa actaaaataa ttttatttat 2640
cgatagtttg aattttccct atcattgcta cacagctaat tgagaggtac cccgaggaaa 2700
atataaatgg tacagtaatg cattgtagat tttaataaca tacttgacat cccaaattgt 2760
tttcattggc ttcattttaa aaactacatg ttttaaaatc aagcagacac taaaagtaca 2820
agatatactg ggtctacaag gtttaagtca accagggatt gaaatataac ttttaaacag 2880
agctggatta tccagtaggc agattaagca tgtgcttaag gcatcagcaa agtctgagca 2940
atccattttt taaaacgtag tacatgtttt tgataagctt aaaaagtagt agtcacagga 3000
aaaattagaa cttttacctc cttgcgcttg ttatactctt tagtgctgtt taacttttct 3060
ttgtaagtga gggtggtgga gggtgcccat aatcttttca gggagtaagt tcttcttggt 3120
ctttctttct ttctttcttt ctttttttct tgagaccaag tttcgctctt gtctcccagg 3180
ctggagtgca atggcgcgat ctcggctcac tgcaacctcc gccttctcct gggttcaagc 3240
gattctccta catcagcctc cgagtagctg ggattacagg catgcgccac caagccccgc 3300
taattttgta ttttttagta gagacagggt ttcgccatgt tggtcaggct tgtctcgaac 3360
tcctggcctc aggtgatccg cctgtctcgg cctcccagaa tgctgggatt atagacgtga 3420
gccaccgcat ccggactttc cttttatgta atagtgataa ttctatccaa agcatttttt 3480
tttttttttg agtcggagtc tcattctgtc acccaggctg gagggtggtg gcgcgatctc 3540
ggcttactgc aacctctgcc tcccgggttc aagcgattct cctgcctcag cctcctgagt 3600
agctggaatt acacacgtgc gccaccatgg ccagctaatt tttgtatttt tagtagagac 3660
ggggtgtcac cattttggcc aagctggcct cgaactcctg acctcaggtg atctgcccgc 3720
ctcggcttcc caaagtgctg ggattacagg tgtgagccac cgcgtcctgc tccaaagcat 3780
tttctttcta tgcctcaaaa caagattgca agccagtcct caaagcggat aattcaagag 3840
ctaacaggta ttagcttagg atgtgtggca ctgttcttaa ggcttatatg tattaataca 3900
tcatttaaac tcacaacaac ccctataaag cagggggcac tcatattccc ttcccccttt 3960
ataattacga aaaatgcaag gtattttcag taggaaagag aaatgtgaga agtgtgaagg 4020
agacaggaca gtatttgaag ctggtctttg gatcactgtg caactctgct tctagaacac 4080
tgagcacttt ttctggtcta ggaattatga ctttgagaat ggagtccgtc cttccaatga 4140
ctccctcccc attttcctat ctgcctacag gcagaattct cccccgtccg tattaaataa 4200
acctcatctt ttcagagtct gctcttatac caggcaatgt acacgtctga gaaacccttg 4260
ccccagacag ccgttttaca cgcaggaggg gaaggggagg ggaaggagag agcagtccga 4320
ctctccaaaa ggaatccttt gaactagggt ttctgactta gtgaaccccg cgctcctgaa 4380
aatcaagggt tgagggggta gggggacact ttctagtcgt acaggtgatt tcgattctcg 4440
gtggggctct cacaactagg aaagaatagt tttgcttttt cttatgatta aaagaagaag 4500
ccatactttc cctatgacac caaacacccc gattcaattt ggcagttagg aaggttgtat 4560
cgcggaggaa ggaaacgggg cgggggcgga tttcttttta acagagtgaa cgcactcaaa 4620
cacgcctttg ctggcaggcg ggggagcgcg gctgggagca gggaggccgg agggcggtgt 4680
ggggggcagg tggggaggag cccagtcctc cttccttgcc aacgctggct ctggcgaggg 4740
ctgcttccgg ctggtgcccc cgggggagac ccaacctggg gcgacttcag gggtgccaca 4800
ttcgctaagt gctcggagtt aatagcacct cctccgagca ctcgctcacg gcgtcccctt 4860
gcctggaaag ataccgcggt ccctccagag gatttgaggg acagggtcgg agggggctct 4920
tccgccagca ccggaggaag aaagaggagg ggctggctgg tcaccagagg gtggggcgga 4980
ccgcgtgcgc tcggcggctg cggagagggg gagagcaggc agcgggcggc ggggagcagc 5040
atggagccgg cggcggggag cagcatggag ccttcggctg actggctggc cacggccgcg 5100
gcccggggtc gggtagagga ggtgcgggcg ctgctggagg cgggggcgct gcccaacgca 5160
ccgaatagtt acggtcggag gccgatccag gtgggtagag ggtctgcagc gggagcaggg 5220
gatggcgggc gactctggag gacgaagttt gcaggggaat tggaatcagg tagcgcttcg 5280
attctccgga aaaaggggag gcttcctggg gagttttcag aaggggtttg taatcacaga 5340
cctcctcctg gcgacgccct gggggcttgg gaagccaagg aagaggaatg aggagccacg 5400
cgcgtacaga tctctcgaat gctgagaaga tctgaagggg ggaacatatt tgtattagat 5460

ggaagtatgc tctttatcag atacaaaatt tacgaacgtt tgggataaaa agggagtctt 5520
aaagaaatgt aagatgtgct gggactactt agcctccaat tcacagatac ctggatggag 5580
cttatctttc ttactaggag ggattatcag tggaaatctg tggtgtatgt tggaataaat 5640
atcgaatata aattttgatc gaaattattc agaagcggcc gggcgcggtg cctcacgcct 5700
tgtaatccct tcactttggg agatcaaggc ggggggaatc acctgaggtc gggagttcga 5760
gaccagcctg gccaacaggt gaaacctcgc ctctactaaa aatacaaaaa gtagccgggg 5820
gtggtggcag gcgcctgtaa tcccagctac tcgggaggtt gaggcaggag aatcgcttga 5880
acccgggagg ctgaggttgt agtgaacagc gagatggagc cacttcactc cagcctgggt 5940
gacagagtga gactttgtcg aaagaaagaa agagagaaag agagagagaa aaattattca 6000
gaagcaacta catattgtgt ttatttttaa ctgagtaggg caaataaata tatgtttgct 6060
gtaggaactt aggaaataat gagccacatt catgtgatca ttccagaggt aatatgtagt 6120
taccattttg ggaatatctg ctaacatttt tgctctttta ctatctttag cttacttgat 6180
atagtttatt tgtgataaga gttttcaatt cctcattttt gaacagaggt gtttctcctc 6240
tccctactcc tgttttgtga gggagttagg ggaggattta aaagtaatta atacatgggt 6300
aacttagcat ctctaaaatt ttgccaacag cttgaacccg ggagtttggc tttgtagtcc 6360
tacaatatct tagaagagac cttatttgtt taaaaacaaa aaggaaaaag aaaagtggat 6420
agttttgaca atttttaatg gagaagggag aagaacatgt agaaaagggg aaatgatgtt 6480
ggcttagaat cctaactaca ttggtgttta atataggaac atttatttat ataacatttt 6540
aaagtactaa attcatatta gtatattatc aaatggatat attatcaaat gggtttaagc 6600
atcctacaca ttttaattca attgattcat tttctttttg ctttggattt ctatcatgat 6660
ttaaatattt acatatgggt tactttttag atttttcata ctatgaaata taagaaaaac 6720
ctttaaggct agttttatga ccaagacgaa ggacttcatt gaatacacaa aacaataaat 6780
atactgcaac attttgtctt tctttttgta gctgcaattt ggtttgctta tactttctct 6840
ttgtctcttt gaaaactgag tcagtttcac tttctcagga caggatttaa taaccataat 6900
ataatttagt ataattcctt gatttaggca aattatgcaa tttgtgttta gtatgaaatg 6960
tacctaaaaa taagtaactc ctctttaaca ccaccatcct caaactaata taacaaataa 7020
cagttatcct aaaataaatt gtctacttcc accatgcagc actcaaattt taaggttgct 7080
atgactgcag acagtatttt aaaattcctc tctggaaatg gctttgtttc caagatgatt 7140
taggaaccaa agaggtgacc atctcttgtt taatgaactc tcaaatcata aacctgggaa 7200
gtgttttagt ttcctactgc tgctgttaca aattatcaca aatgtgttag ctaaaacaaa 7260
cacaaaatta ttattttaca gttctagaga tcagaagtca aaaatgggtc cacaaggttt 7320
cattcctttt ggaaactcta aggggcaatc tgtttccttg tcttttccag cttctagtga 7380
ccatcaaatt ccttggctca tggtctctgt attttctctg tggcctgtgc ttccattctt 7440
gtatcttctc tctgactgtg accctctaat aaaaacactt ggggttatgt tgggcccacc 7500
ctgaaaattc tggataatct ccctcaagac cattaattaa atcacatctg caaagcctct 7560
tttgccacat aagttaatgt attaaaagtt tttgaggatt aggacataga cattgggggt 7620
gggggggcat tattcagcct accacaggaa ggaattttag ggttaattaa actagccttc 7680
ttattttata cttgaagaaa ttgaagtttt ggaattggag agcattatgc taaatgaaat 7740
aagccaaaca cagaaagaca aatatcacat gttctcactt atctgtgaaa tataaaacaa 7800
ttacattctt agcagtaaag agtagaatgg tggttactag agctgggggg tgggaggaat 7860
ggggagatgg taatcaagat ataaagcctc agttaagatg ggaggaataa gtttgattgt 7920
tttttttgag atgtgtttca tagcatgatg aatatagcta aatagtaaat cccaaatgct 7980
ctcatttgac aaaaatgtca aatatttgag atgatggata ggttacttag cttgacttaa 8040
taattcccca ttgtgttcaa agatcataac ttcatattgt accacataaa tatatacaac 8100
tgtactatcc caatatataa ttttaaaact aatataatga aaaagaaatt gaagttcaac 8160
attcccagaa gctaagtgta acttaaaagt tttgtgagaa tttgttttaa caaacaaaca 8220
agttttctct ttttaacaat taccacattc tgcgcttgga tatacagcag tgaacaaaaa 8280
aaaaaaaaaa aaaaaaaatc tccaggccta acataatttc aggaagaaat ttcagtagtt 8340
gtatctcagg ggaaatacag gaagttagcc tggagtaaaa gtcagtctgt ccctgcccct 8400
ttgctatttt gcccgtgcct cacagtgctc tctgcctgtg acgacagctc cgcagaagtt 8460
cggaggatat aatggaattc attgtgtact gaagaatgga tagagaactc aagaaggaaa 8520
ttggaaactg gaagcaaatg taggggtaat tagacacctg gggcttgtgt gggggtctgc 8580
ttggcggtga gggggctcta cacaagcttc ctttccgtca tgccggcccc caccctggct 8640
ctgaccattc tgttctctct ggcagg 8666

<210> 19
<211> 2501
<212> DNA
<213> Homo Sapiens

<400> 19

gaaaaccagg ttgagcaggt tggtttttca aagaaattca ttgagcttcc ataatgtcct 60

ttctatttga cgggagaaaa ggggctttgg aagatcaagt gtgaagcttt agaattctgt 120
ctggagctgg ttaggaccac ccaggcctga gtataagcaa agcgctgtcc gtggtgctag 180
taagcgcgaa tgcccccggc aacgttttcc ctacccaaag ttgtttttca ttcagtcaac 240
ttgcttcgcg aagctcacac atctgcctcg tgcaagattc tcagtcattt tacttagtca 300
ttggttcttt ccctatcacc attctttatg tccccctcaa agaaaaacat tatcttccat 360
ttccttatca actccaaaca gctttcattt ttctgacata tttactacct aagaaaatgg 420
ctcaagaatt gggtcagact atcttgtcct aacttttctg ataagtttca gagaaactca 480
aaggtcaaaa caagagcata agagtaaaag gtagagaaat taagaaactg aagactagga 540
aatgggggtt ggatgggaaa gaaaaagaaa ttgttattaa tgctacccgg ttcccttccc 600
tgtccaggtg gatttcagct ctgttgaggc tctgtcagta gattttcagc cctaaccagc 660
acttccatgg tggtggcact tccactgcct ttaaaagaaa gagctttttt taattctaca 720
gggatttggg ggatgaggag tcagagctaa ggtatcctaa aaaaaacatg tgaagactct 780
cattttgcaa tacacaagca attgccctcc tgttaagact ttgtcttcct cagcactccg 840
aaccaaaatg attctgtaaa caaaaactgt tcacttttag gagaggtcca cttatgcagt 900
tcctcaccaa agtttttagg caacaaatcc ataacttgcg gttctcttcc tatccaatgt 960
agcatccgct gaaatgtttt aaatatttta agtaataaat gttgattcaa actcacctag 1020
gaagattagg aaggggaaaa aaagcacttg gcatttaaat cttcagaaga gaatttaatg 1080
acaggttcag cctgtttaat gacaagccca gcaccacacc cctctcttat gatgtttcat 1140
tattactgca taaatttcct ttattactca tgataaataa aaataagata cctgacaaag 1200
tgggtttaaa taggtaagag tgcaaacaaa gatttactgt acaaatatga tgaaactggg 1260
atctcagatt cttaaagtat aatttttttt tgtcttatgt gtgccaggtt gccactctca 1320
atctcgaact agtttttttc tcttttaagg gttgtatcca taatgcaaaa atggaaagaa 1380
ttaaaaagca cacgcaaaac atgattctcg ggatttttct ctatttttat ggttgactaa 1440
ttcaaacaga aagacacatc caagagaaaa ttgctaagtt tgatacaagt tatgaaactt 1500
gtgaagccca agtactgcct ggggatgaat ttaacttgta tgacaggtgc agagctgtcg 1560
ctttcagaca tcttaagaaa cacggagtta ttttgaatga ctttctctcg gtcacaaggg 1620
agccaccaac gtctccacag tgaaaccaac tggctggctg aaggaacaga aatcctctgc 1680
tccgcctact ggggattagg agctgagggc agtggtgaac attcccaaaa tattagcctt 1740
ggctttactg gacatccagc gagcagtgca gccagcattc ctggcggctc cctggcccag 1800
tctctggcgc atgcgtccta gcatctttgg gcaggcttcc ccgccctcgt gacgcgtcgg 1860
cccgggcctg gcctcccggc gatcacagcg gacagggggc ggagcctaag ggggtgggga 1920
gacgccggcc ccttggccca gctgaaaacg gaattctttg ccggctggct ccccactctg 1980
ccagagcgag gcggggcagt gaggactccg cgacgcgtcc gcaccctgcg gccagagcgg 2040
ctttgagctc ggctgcgtcc gcgctaggcg ctttttccca gaagcaatcc aggcgcgccc 2100
gctggttctt gagcgccagg aaaagcccgg agctaacgac cggccgctcg gccactgcac 2160
ggggccccaa gccgcagaag gacgacggga gggtaatgaa gctgagccca ggtctcctag 2220
gaaggagaga gtgcgccgga gcagcgtggg aaagaaggga agagtgtcgt taagtttacg 2280
gccaacggtg gattatccgg gccgctgcgc gtctgggggc tgcggaatgc gcgaggagaa 2340
caagggcatg cccagtgggg gcggcagcga tgagggtctg gccagcgccg cggcgcgggg 2400
actagtggag aaggtgcgac agctcctgga agccggcgcg gatcccaacg gagtcaaccg 2460
tttcgggagg cgcgcgatcc aggtagctgg ggccccaggg c 2501

<210> 20
<211> 2501
<212> DNA
<213> Homo Sapiens

<400> 20

gtgttctagc tcctgaatcc atgctgttcg tcactacact gtactgcctg tggatgactt 60
acttgtccct gtagtttcat ctgaagaatt cctcctcctt ttcctttgag gcctgcctca 120
aatatcactt cccctgtgaa gactgcctgg tgttctccag gagagagtgt gactcccttc 180
tctaggaatg gtagcacccc aaacacacac attttgcagc atatttcacc ttgcatggta 240
atggcctgct tgtgagtttt attccgtacc agagggtgag ggctctgaag atagcgccag 300
gtcttattta cctcgatacc ccacaacact cattacatgt ctgatgaatg aatgcatagg 360
gggatggctg ggtgcatttc tctcaacttt tcaatttctt gaaataaata acaaaatctt 420
accttccctc agaagtcctg gcatggttcc tttcatcttg ccacagccac tgataatcac 480
tttcattttc tgtgtaactc accaggcaag aagtccatgc agatttactt ttagtagttc 540
acatgacaaa taaatactgc gtttgatttc caaacattaa accatagtat attatagata 600
gatatagagt tatcattcaa agtatgatat ttcaatctca aaaggcttcc cctgaagaat 660
attacaaact cttcctctct ttaagatctg ctgggtagga aagatgggag aaaatgaatt 720
aatgtttaca cagaaaggag gataatgggg gcaaaaataa tagatgaacg tatgggtgga 780
tgagagaatg gataaaatga taggtggata tgttgatctt ggacagatgg gaaatgagtg 840

gatatatcaa taaacagata tgtgggtgga tgggtggaga agaggatggt ggatggttgt 900
ggttttatga agagatgtga aaaaggaagt gtggaatgat ggatgagaag ttgtatggga 960
agatgaatag aagaataggt ggttgaataa attaaaaggt gtgtggttgg atgaatgaat 1020
gagtgggatg atagatggac ctaagtggtt agtggatgga caggaggatg gatggatgtg 1080
agagccccag aaggacataa ggaaagatgg gtggatagat ggatgggcgg atggaaggat 1140
atttaggagg atgaatgagc atgtgtgtgg agagaggtgc ccattcacac tggcttgaac 1200
acatgggtta gctgagccaa atgccagccc tatgacaggc catcagtagc tttccctgag 1260
ctgttctgcc aagaagctaa aattcattca agccatgtgg acttgttatt gaggggaaaa 1320
agaatgagct ctccctcttt ccacttggaa gattcaccaa ctccccaccc ctcactcccc 1380
actgtgggca cggaggcact gcgccaccca gggcaagacc tcgccctctc tccagctcct 1440
ctcccaggat atccaacatc ctgtgaaacc cagagatctt gctccagccg gattcagaga 1500
aatttagcgg gaaaggagag gccaaaggct gaacccaatg gtgcaaggtt ttacggttcg 1560
gtcatcctct gtcctgacgc cgcggggcca gcgggagaag aaagccagtg cgtctctggg 1620
cgcaggggcc agtggggctc ggaggcacag gcaccccgcg acactccagg ttccccgacc 1680
cacgtccctg gcagccccga ttatttacag cctcagcaga gcacggggcg ggggcagagg 1740
ggcccgcccg ggagggctgc tacttcttaa aacctctgcg ggctgcttag tcacagcccc 1800
ccttgcttgg gtgtgtcctt cgctcgctcc ctccctccgt cttaggtcac tgttttcaac 1860
ctcgaataaa aactgcagcc aacttccgag gcagcctcat tgcccagcgg accccagcct 1920
ctgccaggtt cggtccgcca tcctcgtccc gtcctccgcc ggcccctgcc ccgcgcccag 1980
ggatcctcca gctcctttcg cccgcgccct ccgttcgctc cggacaccat ggacaagttt 2040
tggtggcacg cagcctgggg actctgcctc gtgccgctga gcctggcgca gatcggtgag 2100
tgcccgccgc agcctgggca gcaagatggg tgcggggtgc tcagcgcgga cccggcggca 2160
gcccctccgg ctgagtcggc cctgggggac tggagtcaag tgagctgtct gcgaagtgca 2220
ttgggctccg gaaagcaggg ctgggatttg cgctaaaccg ttggagaatg tgtctgtgga 2280
agcaccattt ggttgaaaga aaaagagaaa gagaagaaag tttgttgggc aggctgccgg 2340
cgcgcagttt tgggcgaggt cgctagagct gcagcacatg gcagaaagta accgttctcc 2400
cggatgcgca cagtcgttgt ctggactaac aggctcctgt gcccaagggc tcgccaagcc 2460
ccaccgggct gtgtctaggc agggcagagc tgggcggggc a 2501

<210> 21
<211> 16579
<212> DNA
<213> Homo Sapiens

<400> 21

tgttttttaa aatgatgtct agctgaacca gccaatggag acaaaaatat ttatatataa 60
ttttcctcta agttcactag tcattttata gatcagttat aactttggct actccaatga 120
gttaatttct ttctagagac attataaact ccttaaagat ggaaaataat tgttactttt 180
ttgaataatg cctactatct aaaacaatgc acataaaagc tgctcaataa acttttgttg 240
aattgagaga aaatataaaa aattgaaagc agtctgtttg taataagtgt ttgctaagtc 300
ttacagatct tctctttttt ttggaattat tgcaacaaat atttgacctt tgatttgtag 360
gattaaaatg tgatttctca tattttaata ggagcaagca ttagtcctgc aattcttttt 420
gtcaatagaa taaaaatgga atgtatgctt gctgcttcta catgccggtt gatacccatg 480
ctgaacactt ttcttacaac gcagaggttt ccttgttttc tgtcttgcgt tctacctttg 540
ctcaccacag agacattaat gatgccatgt gtctttttgc accatcagtg cagcagcttt 600
cattatgctc cttgtctcgg tcccactgta gtctaattaa ataaatggaa acgggtagtc 660
actgaaaata aacagcaact gcaaaagttt tgcctcttaa gaaaccttct gcattgtatt 720
ccgccctgcc tcattgattc taagatctct accgcttcct tgtgttcaga aaccagactc 780
ttcagtttgt cttgcttcag tcagacaaat gctttataaa aatgacctat atttttcaga 840
ctgtggggag agaagaaaaa gactttgaga aagtgactaa ggagggagta agagtgatac 900
aatctttgat tcaatttagt tggactgtgt tctttatgcc ttcatctttt tctttataaa 960
aaagatagcg tttttgatgc tctgctccag gcagcctccc agggtgagtg cagatgtgct 1020
gagccatttg gctgccttga ggaccggctc agagagcctc tcttctcact aatgaagtca 1080
tctcctctcg gcagcccggc ctctcccatc actccatgtt ggaacttggc tttggcttgc 1140
atagttctgt cgtgatgtag atggtctttc agttaagttc tagagaaatc tgctcaacca 1200
tgaacaggac atttacagca caggagaaaa aaaaatttat taaaacaaat attttactgt 1260
aacattttga tttgggggtt ggagagcaaa aactcaatat ctgatgtctg ggaaaaatct 1320
aatgttttat ggctttctaa ggaaatttgt ctgtgtttaa tttatcagag caatttatat 1380
acttcgggtt aaagcacaat gagattgcca ccatcacttt gccttttgca agatttcttt 1440
tttaagaata aaaagaaggc aataaagacc ttatatttct gcctttccta gaaagccaaa 1500
gagaattcaa agtgctggat gaaatgtctg tccctaattt gcagtgtttt atttaaaaaa 1560
aaaattatga cacaatctta gaggataggc ttcctttttc cagggtcttt gaaagttaaa 1620

agtacatttt taatctgtag cattactgta tcatccagtg aggataattt gcttagttgt 1680
gcctaggacc agtcagatca aggagttgaa actaagctct ttctgaattt gaatgggact 1740
ggaatcttgt catccctaga gcagcaatag atgatgccat gtctaagaac acttcacctg 1800
atcaataaaa aaggagaagt ctgaaatgta atcctaaaga acctcagaag gaggtttaga 1860
tgaagataat aaaattccct ggagacacat ctgccatcta cacctttcct ctccaagtca 1920
ttggcttgct gcccctgagc tcagccaaat tcccaaagac aagatcaaag caaggaagca 1980
aggcacatag cttctggctt atggtttctt ttagacacaa gcattgtgaa tgaagatctt 2040
gctttattta tacctgggat cttggagttt tcacaaagaa tatccccagt acaaggatgt 2100
gatttaaagg gggactcaca gaataatatt ttaattactg tttgattcat acttgagtgt 2160
aatggacaaa taaatacaat tttagttata aaacctgaaa aatatatatt ttcccattca 2220
aaaacttctg atagttttgt caggcattgg agaatcagaa ataaatgctc tctcattttc 2280
tttctcttga tgaaggaaag tcattaaaaa cagttacata gaaataataa tacaactgta 2340
gactctattc taaatatagc tggtatgttt tccttttctt ttttatccaa ttctccattt 2400
tagacagcta gaggggaaag tctgccctag agatagaaag aagatatcca tggggtataa 2460
tttcatttta gttatctgat agcctgaaga agtatttaat tttctgaata ataatgttac 2520
aagtcaaaga agagaaaaga caggcagttt aaagcttttc aacattgtgc atattctaat 2580
tccttaagtc agccctgaga aagtgccttt gtcctgtaga agtatttggt caagtgttgt 2640
gaagtctatt ggcattaaaa gaaactaaaa attttggagg attagagaat caaatttggc 2700
aacataaatg aaaacatctc agaatgtaaa aagtatctaa cccatcactt tcaatcatgt 2760
tccattatat acagatggca ttatttatag tgaatgttaa ctattaacat ttccctaaaa 2820
atgggcatta atctgctatt agttgcttgt ttctgatcta gttctccttt gttgaaaggt 2880
tttacttaac tgtatcataa agggacaaaa gggtaaaaga taataagctt ttcagactgg 2940
cagatctgtc acttacaaaa tggccttggg caggttactt gaactctttg aaactcaact 3000
agatttactc aaatttaaga atgagaatac aaatccacat cttgaagtgt ttcacagaaa 3060
ggtctatctt aatgtctgga gtatatattt caatgaacat tcattttatt ttatttctct 3120
ccattcctga atcaagcaat cttgaatcta aagttgctat gattagcact gaaaagacca 3180
ctggactatt aattgtgtga ctttgggaca gtaactttct gcaccttagt ttgtttacat 3240
gttatacatg aaggttgaag tctgattctg ctctgtgact atcattctaa acatctgatg 3300
aaatcaaatt tcagtgtttg gaatggtagt acaataaatt tactaagaat aaataattca 3360
ctgcaaaaac acattgattt ccaaatgatg taactgacag ttatattact gcagagggct 3420
gataaataac aaaagaaatg aaagatgcac atggtgagaa ctgaaattat cctgacaagt 3480
cttctacctg tttatcactt aaaatcaatg accatgctga atgcctacaa attacaaaat 3540
ataaaagaaa tcttataaat gcgcatgtac aggagtctaa gttactaaaa gttttaaagc 3600
ataagtttaa accaaactaa tcaaagaagt tgagaggaaa aattggcttt catctttaat 3660
cactactgtt ttgaggtcct atgtttaata taattttcta agtagaggct tcagagagaa 3720
gagttgtgag gatactttca tatttgtgta gaaggaaaag tttgccatcc attctagtat 3780
ccctagtgtt atactgatgt gcaccttgga tttattttgt tcctattgta taaactcata 3840
cttgacttca aagaaaagga aaatccaaag tccctctttt ctaaggggac agaaatcctt 3900
tgtgtcaact gtttgaccct tttctctgta aggtcctatt ggaaatcttt tgtaacacaa 3960
tgcaggggac tcttccatgt gttgatgctg tttacacagt ggggtgggcc tgactgaaga 4020
aaaaaaatcg catatacgca tgaaagatta tggtcttatt tccggaaagc atgaaaggtg 4080
attgatactt ccaagaagtc cctgttactc aggaaaatta tcaaatattc tactcagaga 4140
tacttggaaa gactgaagga aaggaagaac gaagaaagca gaatctagac ttatgtgggg 4200
agagatttgt ggcagaggaa aagtattctc tttgaatccg acaagggatt tgcctggggg 4260
aatttcctgt ccagcctttt attaccaggg tcttttgaag ccgggctccc cattgggcag 4320
ttccctggga gtgcagtggg gaattcttac actttccctc taggtccccg aaggatctcg 4380
ttttctcagt gtctctttca ggttggcagg agccttgagc ctgacacttc cctttgatgg 4440
gacaggcaag ctctgtgggc gcgtaaacac gctgtaacca agttctttgc tgattttaca 4500
gttttgtgtg ctcccgagaa gaagtgatcg tactcaattg tctattgctg gcctgccccc 4560
taagagcctg gggctccttt tcccctaacc cagaactagc tgcacggggg gcggggaagt 4620
gggggtgggg aaggagtggg agggcagtgg tttccgcgag cagagcgatg ttactgagtg 4680
agtccctgaa tggggagcgc tgctgtcccc aagccgattg gtacttcttg tcaggaagaa 4740
acgccaagag gtgggagtgc ctggggaggg aggcaggcgg tccctaccgc aggcgcgggg 4800
agctgccttt ccgcccctcc gcctgctttc caagcctgga ctcttaggag tggctgaagc 4860
tgcggagcgc ttttggagcc tgtgaatgaa ccctcctcct ctccctcctc cttcttctcg 4920
ctgagtctcc tcctcggctc tgacggtaca gtgatataat gatgatgggt gtcacaaccc 4980
gcatttgaac ttgcaggcga gctgccccga gcctttctgg ggaagaactc caggcgtgcg 5040
gacgcaacag ccgagaacat taggtgttgt ggacaggagc tgggaccaag atcttcggcc 5100
agccccgcat cctcccgcat cttccagcac cgtcccgcac cctccgcatc cttccccggg 5160
ccaccacgct tcctatgtga cccgcctggg caacgccgaa cccagtcgcg cagcgctgca 5220
gtgaattttc cccccaaact gcaataagcc gccttccaag gtaatcacgt ttcttttgtt 5280
ccccccttaa aaaacaaaaa caaaaaactt atagaaaaaa acccgcgagc ttagaaaaaa 5340
gaagcaattg gtagaaggct ttaattaagg caaagagctg taaggcgaag ttaagaaaat 5400

gtaggcactt aaaaaatgca ggtaactttc ataaaggctt ttggggagag gcatacagag 5460
ggaccttggt gttgaaaaag attcagacaa aagaaaccca gggtggggtg ggggtaaaat 5520
gactaacgga attgggggaa gggagggaat aaattgtaaa gaaatcatag aaaagtggtg 5580
ggttcttgag ctggagagaa gagagggacc tttggcactt tgattttttt gttgttgttg 5640
ttcttaacac gctcgaggca aaagtttgaa tggggactac caagacttgc cacagacaag 5700
tccccgaagc cgccttggtg caggccacct ggtttcccag cccctggtgt gtggtcagtg 5760
cctggtgttc ctggaaagcc actcccgggc agctcctgac agtgcgaccc ggcgcccaag 5820
cagcctggga ccttgcgcgg acctgacccc ttcagaccgc aggcagtctg ggaggaggtc 5880
cggccggggg aggtgcagga tccccgccgt gtctctttga cgacttgggg actgtcacgg 5940
ttctctcccg gcgcccctgg gttcttttgt cctgcacgcg gtgcgaaggg gccagcaggg 6000
aaggagcaga ggatgggggg tggggttgtt ggagccccgc ggaggtctgg gaggcccctg 6060
ggcgggaaaa gcctgttctg aatcggcagg gatgtgcaac aatttttctc accctgaaga 6120
gtgaaatagg gtctgtcgct cccatctcaa caagcaaacc ggcacccaga gcgcactgca 6180
gacaaaggtg gctcggggac ccgaatcagg ggcctctggg tcagtcctct cgcccagact 6240
acaggagtcc tccgtttcct tacatattcc ccaccctctc ctagttctcc gctctagttg 6300
agcaacttta ctggcaggtc tagcggcagc ggcgcgcgcg tgtgccggga gccccggggg 6360
acgtcctcgg ctggagcgcc ccaccgtcct gcagaggcgt gggcgctgta gggcgacatc 6420
cctggtgcgt gcagacctag ggcatccggg ttgttctggc ccgcggtctg tgtcactggg 6480
cgtggagcgg tctgggtgtt aggcagagga gagcggggta gaaaaatagt gtcatagcct 6540
aaactatttg ctctccaatc caactgcgtc tcggcaagtc ctgcatgtcc ctgggagatg 6600
ctgcgggaag ggggagaaat ctacacggcg cctggagagt tgtcctgccg cgcgcacacc 6660
cgcggcagag tctctctggg tcgcgttcct catctttgtg tctccctctg tctccatctt 6720
ctctgcctcc cttcccctct cccagcctct gttctctctc tctagcttct gcgtcccctc 6780
ccccacagct gacaaatgaa tggctagtgt gaaatccctg cctttcccgt gctccaaggc 6840
agcagggagg gaggagcgag ggaggcggtg cgctccttcg gatctgcccc cagttcagct 6900
cacaagactt gcagaaccta gatgtctagg aattgggagt tttgcggcgg gtgtgggcgc 6960
cccctgatgg agaagtcccg cacaggcgga gaaaaacaag ccccccagac caagcgtgca 7020
tctttcacaa ccttgtgttc aaggatggaa ggccctagtt ttcccttaag tcattcattt 7080
tgcactccac aatctgcggc gtacatctag gagtttggag gaccctgaaa aaaggtcttg 7140
gtctgtgcaa agtgcagaga tgccttctcg cgagtcggct ggaacgcacg cggcgccctg 7200
ggtccagttc gccgcttagt ggacgaacgc acatggccgg ggtggagcca ggtttcggag 7260
gtctagacgc gccaccctgg gcgtctttaa gaaagataat acacataccg tgctctcaaa 7320
acccatgacc acttgaaccc aacggtaggg ccccgcccca ggatattgca aaagaagggc 7380
tcctaatcca aaacttaaac ttcctcaact tcagggcggg cgtcggacgg gaaagtggag 7440
agaaggcggg cagtgggagg aaaaaagaaa gggaaggaag ggaagtggaa ggaaagagga 7500
ccgtggaggg gaggaaaagg agaaaggaac ggagatgggg cagatggcat tcaaacagcc 7560
aggaacggac cggaagggtt cgtttcgggg tttgggtctg aatttcgaga ctcgttttag 7620
gatactcttt tccctttccc agcggcaaca aatgtgactg cagaggcgtg ccgggatgga 7680
agggtgggaa agaactagac aagcggaggt ggtccagccc cgcccaaagg ggctgggtct 7740
ggggcccgcc cccactcgct cacctcgcgc caagggagga cccgcaacgg gcgctccccg 7800
cagcggctgg gttagccagc cgccacgtgg gttggcgctt cggctcccag ccgagacggg 7860
tgtgacttcc tgggctcaga gactgcagta cagccgcaca ggcggccggc gcagccgagg 7920
cccccaccca ccgaggccaa tgggggcggg gcggggaagg cagggcccag cttaaccgag 7980
agagccgagt ggcctcgccc ccgggattcg gggccggctg cttctggagc tgacccgagt 8040
tccatccaga cagcgctgaa cactcttatt aaaaggctat ttaagtttta ttttatttta 8100
ttttattttt gcttgcttct tgggagtggt aggaggggca tcgtgctttt gttttgaggc 8160
aggggagagg ttaaaattta ctccaagcaa actttttgtc agaaaaatga gactttatac 8220
cgtattccat ttgttgactt ttaaatacaa ttcaggtcac tctaaagccc attcttagtc 8280
aattaacaga ttaatttcgt tccagactgt cataaagagt tcgcctggaa tggcaaatac 8340
tgccattcga caagactaat agaatttcat gggggcaaac atggaagggc tgcgagtgta 8400
actgaggttc ctgtgcctca atgaggaacc ttagcgggga cctggcgggg actggaagct 8460
cagcacctat ttagggagtg actgagctgg aaacctggag aagcttggtg cagacgagac 8520
ggtgcttctt agagctgcat tttaagtcct cccattttgc tgcatattgg aagaactgga 8580
ttttaaagat cttttccatc acctcagatt ctgtagctaa cagccacgtc tttttctttt 8640
tgtctgctga aagctgtgac ctaattgttt taagcacttc ttgacaattt cgccaaaccc 8700
atcctgaaaa tattcacata aagtcctaaa ataggatgtt gtgagtgtaa tataaaaata 8760
ggaaggtacc aatggaagca acaattagtg ttctgagata gaagcggttc ccagtttgta 8820
acttggaaat gttaagagca gtacatcagg gagaaggtct ccaagagtct gacttcttgt 8880
tctgagctct gagtaaagaa agaaatatgt tagaattgta ttgtctgccc gcagtaatat 8940
tgtaaagggt tccaatggat tccaatgtga ttgagcaatt acttcctcca gcaccaagtc 9000
cacagtcctt aatcttcttg tgagaagatt aggtaggaag cgggatgaga ttcaagacaa 9060
tcatttagga tttatgtcag aattcaaagt tttgattctt ttggtagata aaaacagagt 9120
tggttttaaa ttgtatttcc tgctttcaaa aaaacgaggt ttgaaaggaa tccccctaga 9180

tttacatagg tttacagatg taagtttctt ctttttccct gtgtttgcaa actggaggga 9240
gatgcctttg atattccaga atacctgtat ctatacactt gtaaattctg ctaggaaagg 9300
acctaaacag ctaaaccaaa ggtgaccatg attaatgtta aagatactac ccattgttct 9360
ctcttttgtt gtattattag gcttaaatac tttggagtta atgattggtc acttaattga 9420
gtaacagagc ctttgagagc gcaaagatca actaactctg tctcccaaag tctggcaact 9480
aagccctcct ttcctggtga gatttaaaca tttttgttga cgtcattcat ggacaaacat 9540
cggtaaacac tgtatttaaa atgggatatt gccctgtgat ggagcagaac ttctcaagac 9600
acatggttct tttgaaagaa agaattaatg actgtagtaa ggtatttttt caccacccac 9660
gtagagaaac cagttaggac tgacaaacac tcattgttgg gagttacttg ctctggtctg 9720
agaaaatatg ctgattgtta cataggtctg tattttacca gaaaacacaa agcatgtact 9780
ctccctgatt tggaatcaga aaagggggat tagatatgga tgtcttggcc aagctccatt 9840
tcaattagtg aatttttgcc agtttcctct tgtagcctag acttggtaaa ttacatatat 9900
ataaagtctg tgtgtgtgtg tggggtgcgt gtgtgtgtgt atgtatacag ggtttttaaa 9960
cactgctaag ttcttaccat tgaatgtgtt agaggtggtt gtatttcaga ggtcagtaca 10020
aagaatcttt gtttaatgtg tcagttctgt ttcctgaaac acacctctgg gtagatttac 10080
caactataaa aggatacaaa tgtatatact ttccacatct gtatacatcc tacctcattc 10140
ttctaaggat tttaagaggt gtttatgtct ataaaatgag attacatatt ttacaataaa 10200
catatttgat aaattataat ctttttatta ttaaattatt gatttgatat tacttgaggt 10260
atagtggaat gagcactaga cttggaatca gaaagtttga gttggactca ctagctgaga 10320
aaaccaagca aagatattac ttttggacct ttagtgtcat aatctattaa atgaatatgc 10380
agttataaag atcaaaagag taagtacttt gaaaaactat acaaaaaagg gatgataata 10440
tattatgtct tgggattcat attgatctaa ttctataatt ttaataataa ctgcattacc 10500
caagccatgt agtttctact gaatctataa aataaatcag agaggggcat attgttttaa 10560
aaattgttta gtttcattaa tcagaagaca gattacagaa attgactcca atgaagttgg 10620
acattcagtg gaaaggaaga attattcaat aaatggtttc aggacaattg atgggtccat 10680
gcaaaataag aatcagtctc tacctgattt ctcatcctaa aagaaatata agatgaatca 10740
aatatgaaat gtagggaata actttttaca aaatgataaa agaaaatcat gggattaaaa 10800
aaaataagtt aggaatagag aaacctgttc taagcaaaac ataaaaccca gtgccattct 10860
aggaaatact gataggtctg actaagtaaa aattacacaa aagcaaataa tatatttgga 10920
aaacatctac gacatttgag agacaaaaag ctaataactc ttgatattcc atggactttt 10980
atgaataact ttgaaaagtc cacatctatc aaaatatgag caaaaattat gtacaggtag 11040
tggcagaaag agacatagag ttttaaaatt atagaaaaaa tcgctcagcc tcatctgtaa 11100
ttaaataaat agtaattaaa acaccaatga tatgctacac cattttttca ccaatcagag 11160
gagtaacaaa aaaaaataat ttttttcttt tttttttttt ttgagatgga gtctgttact 11220
caggttggag tgcagtggca cgatctcagc tcactgcaac ctccgccttc caggttcaag 11280
caattctcct gtgtcagcct ccctagtagc tgggattatg ggcacccacc accatgcttg 11340
gctaattttc gtgtttttag tagagtgggg tttcaccatg ttggccatgg cagttgatct 11400
tgaactcctg acctcaagtg attcacccac ctcagcctcc caaagtgctg ggattacagg 11460
catgagccac tgtgcctggc aaaaatgaaa ttaaaaagtt tgatgcaagt ttgtgttggc 11520
caggttgtgg agaaactggg tctctcatat actgttgtat gaaagtaaat aggttcctct 11580
acttcaaaaa ataatttggc aatcactttt ttttgattct ttatcaaaag tttttaaaag 11640
atgtatatac tctttgattc agcaattcca cttctcgaca ttattatatg gatggatatt 11700
ggtaccaata tcacccaaga caaatggaca aggatgttct ttgcagcatt gcttctaaga 11760
gcaaatatct ggaaacagtg taacaggctc atcataggga accatttaaa taaaagttgc 11820
tgtatccacc taatggaaaa ctatgcagct gtcaaaaata acatggaaaa attgtatttg 11880
ctgatttgga acattctcca aaatatattg tattctaagt gaaaaaattg atagataaaa 11940
ctgagtatat attatacatc taattatgta aaatgggtgt atattcattc ttttaacaaa 12000
tatttactga gtgttctagg cattgtggat acaggagtga acaaaacatc aaagactgct 12060
ctgttggaac ttttactatg gtaggtagag aaaggcaatt cccaatcaca tatgcaaaat 12120
ggtgatatgt gctaggatga aaatagagca tgggaaaggg aatagagtac agtatgtctt 12180
aggtagagta atgagaaagg agttcaccag gcagagaaca gaggagtgct aaggggcgac 12240
aatgtgaaaa agcttagcca agtccaggtt caggaattca catgatgcta ttacttgtat 12300
gaagtaaatt catgaaattt cgcacctaaa tcacgtgatt tctctctgct gtacaatgat 12360
tctttctgtg agaaagaacc agtgagcaat atgatatgtg atgctcatta catccacttt 12420
gtagctgaag tatcctagaa agttggttgt ctagggtacc taattagcag aatggtggac 12480
aaaaccaccg tgtctctgtc cttaagtcac cccttgtcag ctgagcttcc ccaaaatcaa 12540
gggagaatac taaatatcta tttaatagaa tcttggcttt ctctcggagg ctgcccatcc 12600
ctggtgtgtc ccttatcctc cctgcagaca tttctcatgg ataggagttc cattgacctt 12660
gtcttttatg tctcccaggc aattaaacag tctcagtacc ctgtacctgg attgttcaat 12720
gactttcttc ccttctcatt gacttcactg gagtagaagg ctaaatatag gcattcactc 12780
tcatatctct caggtctttc catgcagaca gagttgttat cactagtaga cggtgcttat 12840
aagagagaca tggaaaagtg gaagaatgga aatagggatt tggagtggtg ctaaaaacaa 12900
agaaagaggc ttctgaaagc tttccattta atatgatcaa acatagaaga caaaagatgt 12960

aacttaatga ccaaggatag ggacatagcc ttgagaaaat tacagaagtg gaatgaacgt 13020
cttggactcc aaagaagagg gaatggaaat gcagttctga ggcagctaaa gtcaataaag 13080
ccctctgata tatttactgt agaaataaat gatcatcttt taattcaagg tttgcaaact 13140
taggtgacta caggggcgag gcatggagtg gaaatgggta agagactaag tgggggcagt 13200
aatggccttg agcacacaga cttaagggga gagactgctg cttggttaat gctcatgatc 13260
agccagctga aatatagact tagtgttgct agaccttctg attttttgaa agaagttaga 13320
gattttatat ttagtgtaaa cagtctaatt tttaaattga gtaacttatt caaacatatt 13380
tatgcaactt attcaaatgt atttagagca tgtctgatct cctccttgta ttcttaccta 13440
gtcttagggg taatcttaag gaaagtgatg cgtatacttg ttgtacctat gtagatggag 13500
gaccattctc ctctccccaa caaaagacac ttttataagg attgagtgtg gcacaaagag 13560
ttcgattttt agtatttaaa ttgagtagga aactcaaata cagaagatcc tttcctgggt 13620
cacacctttg gtttttttaa atgagatttg actctctaga tattgacaaa catcatatta 13680
ttgggtcatg gtaaatcttc aagtggactg attcaaaagt gttcaaaagt atttcaggtg 13740
ttaaggaatt gttggcacgg agtttcaaag gtgtttctgc ataagaaata gaggcgttga 13800
tcatgtattt gatctacaaa cattcctcaa ggatataccc tgaacctggc aatgaagaaa 13860
tattgtggga attattaagt tgaataaagt actagctctg ccttccagaa gcctactgtt 13920
ctacagagga gattagacat gtttacagat aatttgaagt acatagcaat aaattatagg 13980
atgatttatt ccaagcataa agtgtatatt gctactatag tggtaaccta ggttataatt 14040
ttcaaatgta agagccccaa atactattct ttttactctt agtgatatga aactatgtta 14100
gaacatctgc agaaagtctg aattagtaat tttaataata aatattgagt actgatatat 14160
tgtatatcac tgagatttat tttatataca ttattttatc aattcctatg atcacttcgg 14220
taagtactaa ttttcacaac taagtagtga cagaaaatga ggtttagaga gatatagtat 14280
tttacccaaa gtcacagaat ataagtagag aatgcagaac ttgcattctg ggctatttga 14340
cagtggagcc cagactttta aaattaggct tataaagatt tcctggaaaa aaagaaaaat 14400
aaaaaataga gaacaaattt tcctgaagaa aagtaggaaa ttattattta taagtataat 14460
taaagagaga tctttaattt cttttttttt tttttttttg agacggagtc tcgctctgtc 14520
gcccaggcgc aatctcagct cactgcaacc tccacctccc gggttcatgc cataaagaaa 14580
gtttctattt gattggtccg acctgattgt ctactagaac ataggctttc cagtagggtt 14640
aacaagtcat aacagttcta gtgatataag ataaatacaa actaataata aggttaattt 14700
agaattatca acatatgttt ttcattgtga attatcagtt ggtctataga tggaaaatat 14760
aagatatata atgtattagc ttaaaaaatg ctcttcagag agtgtctaga attatagaat 14820
ttaaaaattt tgttttgaaa aggtctaatt cttatttcaa aatatataaa gaaatcaaat 14880
gcaaaaaaat taagagaaaa ggtcttaatt aattaaatgc tgtacaactc caagccctat 14940
ttatgttcaa gatcagtttt gtaatttatg actaatttca gcattagctt gttttcaatc 15000
taggtttgct aaataatata gaatttcttt tccaatgaag tttttcaggg tcccagctga 15060
aaaatatata ccagttagga tgctttcaac tgtaaggaac aaaaagtcac gattttattg 15120
ggtttaaaca ataatgacat ttataatctc atataaaatg aagttcaaag gcatggtcca 15180
ctctaggtct aatccttaag gacttacgcc ttgcctttgc tgattctttc tcagcttcat 15240
ttccagaggt atctcactta ctcatggatg caaaatccct acaacagttc cagcagaaag 15300
attccagcat gataatgtcc agggaagaaa actgaccacc tcttcctttg ttctcttctc 15360
aggatgaatc tatctttccc agggaccctt cactggagga agagatcccc tcacatctca 15420
ctggccaaaa ctatattata tactgttcca aatccaatca ttggaaaagg gaatggtacc 15480
tctataattg acttaggata tcaggatcat cccttgggct ggggtttgac ccaggattct 15540
ccaaagtatg tgactgaggg taggcactag aatggaacca gagttgtgtt agaaaggaga 15600
aacgcatggc tattatagaa tagttctaaa tgctaccgag gagggcacaa ctgtaaaaac 15660
caaataatct tttgccattc ttttgaagtt ggcactttga tttctagatg gttccccaac 15720
acaggttctt ctcctcctca tcattatagc tgccttgaaa tttgagctgg aagggaacat 15780
tctgagaccc agattgttaa atgtcttttc caaagtcatg caataaatta aatggcaaag 15840
ccagggcagt ttcttgactc agtacagggt attttctttc attctttact cttgagactt 15900
tagaactgtt ggtactgctt taaaattcat ggcaagaact ggtcactttt gtaattaaca 15960
cctccttata atacatttgt tttgtttgct tagccagcta gaaactacat ggagtctgtg 16020
ctttaaaaag cctgccgaag tccttattct ctgttttggt attatgtgca tgaaccacca 16080
attggttcct tctcacctta cacttgatga agatgccttt ctttcaacat ctttctctat 16140
tgctccccat cttctcttgc tctatttatg atcagctgtc tgtttctaaa tagactttgt 16200
ggtcacccat ttctttttgt gccagctcct atccactagt tacttgaact gtggtctcta 16260
ttgttcttca tacagcctac tcacttgtgg ttgtcacaca catccacatt gttatatgtt 16320
cccaaactgt attctggaat gattttggta atggctgcat tggatgagat tcaaactaat 16380
aattaaagca ttgagatagc ttctgctatt acaagtttac tcctgttttt atagtccaag 16440
aggagctact tcttctactc ttattactta atgcttaata ctacccttat tacatacaat 16500
gcacaaaaag catgtgattt atgacccact ttaaacctga atgcttgtga tttactttgt 16560
gtttttcttc atttctagg 16579

<210> 22

<211> 6090
<212> DNA
<213> Homo Sapiens

<400> 22

cagagatgga atgctcaaat taggacaatt cagggaggtg tgggagaggt gcagggggac 60
cataagggat ggagcaggaa cccaaggtag taatagcaga gctggcacca accctaggct 120
caaaaagaca aaggagagag agtggttacc aaaacccaga aagagaaaac ccggtaaaag 180
acgggacctg gtgaggagca gcaacctgtg tttggctgtg gactgtggca atcttgcagg 240
gaggagagca gaggaatgac tatcttggct tcactctcct tccttctcct tcatctcatg 300
tcaggtgccc cattggctaa acctaccagc aaccagagga caaggaatgc attaaaataa 360
tcttacggct cagagagtag attagatgta gagtgacaaa tggattatat ttagcacaat 420
cgcaggtcta tttgttgatc cgtctccctc atcagactga gttacctgag gacagagaat 480
gtttctctta tatctgtatc cctaacattt ggtggacact agatactcaa gaaatgctta 540
tggacgggat cagagaagat ggtaaattgg gcattagatg gaggtttgtg ctttctgagc 600
ttgccgcatg ctttctggcg aatccaaggc aaaatgagaa acagaatgag ctctgtactt 660
ttgtctggta aaagaaaata tgatttggtg agaagaaatg aatactttta gcactcagtg 720
ctgggaggaa ctggaatcag tcatgtgact gcctctatca gaggctctca actgcgtaac 780
ggacagaatc tttaatggtg aaaccacaat gcagaaacat tctttttttt tttttttttt 840
tttttttgga gacggagtct tgctctgtca cccaggctgg agtgcagtgg caccatctcg 900
gctcactgca agctccgcct cccgggttca ccccattctc ctgcctcagc ctcccaagta 960
gctgggacta caggcacctg ccaccacacc tggctaattt tttgtatttt tagtagaaat 1020
ggggtttcac cgtgttagct aggatgctct tgatctcctg acctcgtgat ctgcccgcct 1080
cggcctccca aagtgggaaa cattcttaaa aatattagcc cttgataaaa ccaaagccat 1140
gttacaaaaa agcaaccaaa aggtggaaat gagataatat ggtccacttt gagctgagaa 1200
catcttgcca gaaaatgatt gtctcagact tcaaatctga cctcctacat aaaccttcag 1260
tgcaatcctc cctgtattat ctcttctctc atcagccaaa agatggaaag gataaactga 1320
ggaacttacg ctggaggagc atggattcat tagatggcag aaacctgggt ttctgaatga 1380
ctactgtgga gctgatctct gcttatgccc tgcaaacctt tgtgctgaga gcaagtaaca 1440
catttttaat gtgttaaacc tctggatttt gttgggtctt atagcaatgg ttattttacc 1500
ctaatacgac tgtaaaataa tgttctaaaa tctacagtcg ggtttgttta aaatattatc 1560
gccatcatta ttataatcca tagtccatat cataattatt tttatagaag tccttctcaa 1620
ctggcatctc ataatttgtg gcttcttata ttgttgcctt ctatgtgtga gatgcctctc 1680
tgagtccttt ttgctaggga catgatagaa aataaaatat ccaacattct ggtgtatttt 1740
ataatgtaca aagtcctgtt tcatgcatta tctctttaga ttctcccaac aaacccaagg 1800
aatatgtaag ggtctataat catcttcatt tcacaggtga tgaaactgag acgtttacat 1860
gtaaagtgga aataaccact tatttattta tttatttatt aaataaaaac cccgctaatt 1920
tatttaaaga ttaattgaga gaattatttc atctgtctgg aacctggtat gtgttaagag 1980
aaaagttttc ttaatttttc tttttaagaa gataatctat tgctacataa caaaccactc 2040
tgaaataatg acttaaaaca ttaaaagtca ttgattttgc tcacagaact tgaactggaa 2100
gatataaaca actgggagtg agggtggggc tggacagctg gcttctctgg gcatctctcg 2160
ttctttctgt ggttttgtca catgtctcta catggcagcc ctagggtaat catgacagct 2220
gaaggcttta gagtgggtgg aggggagagg ggagggaggg aaagagagag agagagagag 2280
agagagaaag agaagcaagc aggcagaagc tctaatgcct tatgacctaa ccttggaaat 2340
aaattacact gtgccactgc cactaccttt tcttagaagc aagcagatag ggttggtgaa 2400
tatttattga gaggggaatt agactccacc tcttgattgg gggagtgtca aagaatttgt 2460
gaatatgttt ttaaatgacc acatttcccc tttctaaaat gcttgtctaa attggtgtac 2520
acaaaaagct cgggggttag tgactcatcc aaggttatta agctgttaaa tggttccacc 2580
aatactagat ctaattttgt gactccaaat ttcatgctct ttttcactac tccagataat 2640
ctccggtgaa catcattgtc cattagggta gttaagacat tctagctgcc aaatagattc 2700
agtcctttta attgcctcgg acaatatatt ttttattttg cagaaaataa atattaccca 2760
tatggcagtc atccataaac accttccaag tttatcccaa acatctgtgt tttaaatttt 2820
ttctcccaac agatgtattc atttgcattt tcccagatga atcactcttt ttttgttgtt 2880
gttgttattt ttgactgtat ttctcacctc tcaagaccct tttgtattat caagcccact 2940
gcttaagcta ataccatctc ccaatttggg gacttcccct tgattcctgt gtgcccttcc 3000
tgtgggtcat taatgcaatg ttatttaaga ctaggatttg gctgggcgca gtggctcacg 3060
cctgtaatcc cagcactgtg ggaggccgag ccgggaggat cacctgaggt caggagttca 3120
agaccagcct gaccaacatg gtgaaaccac gtctctacta aaaatacaaa attagccggg 3180
catagtcaca tgcctgtaat cccagctact gggtagcctg aggcaggaga atcgcttgaa 3240
cccgggaggc ggaggcggag tttgcagtga gccaagattt cacaactgca ctccagtctg 3300
ggccacaaga gcgaaaaccc gtctcaaaaa aaaaaaaaag actaggattt gacataaggc 3360
ctgaggggta ttcttttgtt ttgttttgcc ttgttttcaa gaggccaaaa tcttcacagt 3420

tgaaaatttc tgttgaacca cagagatttg aaccaactca gtttagaaag cctggggatt 3480
tgaacaacgg tatggatcgg aaatctcttc atctgtcagt tttcatcatt ctaggcagta 3540
aaatagattt ccctttagga gcttttcacc gtttggggtt ctccagcagt gggatgtggg 3600
gaatcaaccc ttcttcgtct ccacccaaac attaggtggg agcaaggggt gggaagtaga 3660
gaaagtggat agaggtctcc agtggatatg ggatctttgt gtagaccagc acagtcctca 3720
gaaatctcat gcaagcaaca taggtactgt tatattttct agtggccacc ttttaaaaag 3780
taaacaggtg aggccgggcg cggtcgtcac gcctgtaatc ccagcacttt gggaggccca 3840
ggcgggcgga tcacgaggtc aagagatgga gaccatcctg gtcgacacgg tgaaaccccg 3900
tctctactaa aaatacaaaa attagctggg catggtgacg cgcgactgta gtcctagcta 3960
ctggggaggc cgaggcagga gaatcacttg aaccctggag gtggaggttg ccacgctcca 4020
ctacactcca gcctggcgac agagtgagac tccgtctcaa aaaaaagaaa gtaaacaggt 4080
gaaattaatt ttaataatat attttgttta acccaacgta tccaaaatac tatcatttga 4140
aagtgtaatg aatataaaaa tattcatgag atatttttca ttctcatatc catactgtct 4200
tggactctaa tgtgtatttt acacttacag cacaattaat ttgggactag ctacatttca 4260
gctcaacaat agccaatagc atatggatag cgcaaataaa actctgcgtc tctgttgctt 4320
ctttgggtct cggagacctc aaccctttct tcagattgca aaccttcttg ccttcaagcc 4380
tcggctccaa caccagtccg gcagaggaac ccagtctaat gaggtacgct cccttcctgc 4440
cattctctat tccattaacc tgtttcgtgg taaacgtagg actgatcctc caaaattacc 4500
ttattaatta gcttacatat ttattatcta tctgtcccac cagaatgcag gtttccggaa 4560
ggcagggatt taaaaaaatc tgttttgttc tatgtgattt tcccatacca agcaccgtgc 4620
ccggcacaag ctgggatccc agtacacatc tcggacggaa agaaccgtgt ttccctagaa 4680
cccagtcaga gggcagctta gcaatgtgtc acaggtgggg cgcccgcgtt ccgggcggac 4740
gcactggctc cccggccggc gtgggtgtgg ggcgagtggg tgtgtgcggg gtgtgcgcgg 4800
tagagcgcgc cagcgagccc ggagcgcgga gctgggagga gcagcgagcg ccgcgcagaa 4860
cccgcagcgc cggcctggca gggcagctcg gaggtgggtg ggccgcgccg ccagcccgct 4920
tgcagggtcc ccattggccg cctgccggcc gccctccgcc caaaaggcgg caaggagccg 4980
agaggctgct tcggagtgtg aggaggacag ccggaccgag ccaacgccgg ggactttgtt 5040
ccctccgcgg aggggactcg gcaactcgca gcggcagggt ctggggccgg cgcctgggag 5100
ggatctgcgc cccccactca ctccctagct gtgttcccgc cgcgccccgg ctagtctccg 5160
gcgctggcgc ctatggtcgg cctccgacag cgctccggag ggaccggggg agctcccagg 5220
cgcccgggtg agtagccagg cgcggctccc cggtcccccc gacccccggc gccagctttt 5280
gctttcccag ccagggcgcg gtggggtttg tccgggcagt gcctcgagca actgggaagg 5340
ccaaggcgga gggaaacttg gcttcgggga gaagtgcgat cgcagccggg aggcttcccc 5400
agccccgcgg gccgggtgag aacaggtggc gccggcccga ccaggcgctt tgtgtcgggg 5460
cgcgaggatc tggagcgaac tgctgcgcct cggtgggccg ctcccttccc tcccttgctc 5520
ccccgggcgg ccgcacgccg ggtcggccgg gtaacggaga gggagtcgcc aggaatgtgg 5580
ctctggggac tgcctcgctc ggggaagggg agagggtggc cacggtgtta ggagaggcgc 5640
gggagccgag aggtggcgcg ggggtgccac cgttgccgca ggctggagag agattgctcc 5700
cagtgaggcg cgtaccgtct gggcgagggc ttcattcttc cgcggcgtcc ctggaggtgg 5760
gaaagctggg tgggcatgtg tgcagagaaa ggggaggcgg ggaggccagt cacttccgga 5820
gccggttctg atcccaacag accgcccagc gtttggggac gccgacctcg gggtgccgtg 5880
gtgcccggcc ccacgcgcgc gcggggctga ggggtcgggg gcgtccctgg ccgcccagct 5940
ttaacaaagg gtgctcctct ccaccccgcg aggaggggca gctccggaga cccggtcttc 6000
agcgagcggg gtcttagcgc cggggaggtc tacttccttt tggggttgcc attttactat 6060
tattattgcc tttttttttt cttcaaaagg 6090

<210> 23
<211> 6274
<212> DNA
<213> Homo Sapiens

<400> 23

ggatcacctg agcctgggaa gttgaggttg cagtgagcca aggtcacgcc actgcactct 60
ggattgggca acagagccag accctgtctc aaaaaaaaga aaaattccat ggctctgctt 120
acattatcca tctgatctta catgttgcct attttttcca ttaaaactcc tagcctatta 180
atcatagttt ttttataatt aatactccga tgtgataatg tcttagtcca attactgtgg 240
ttataacaga atgccacaaa ctgggtgatt tataaacaaa agaagctgat ttaggctgat 300
ttagaggctg gggagtccaa gagcttggtg ctagcatctg atgagtgtct tcttgcttca 360
tcataacatg ggagagggca tcacgtgtga agagagctta ctcttataac atagccactc 420
ccacaagaat taacccaccg ccatgagagc catgtgaatt cattcatgag gacagcgggt 480
taagtttcca atatatggac ttttcgggga cacattcaaa ccacagcagt tagttgtaac 540
gttcgtgtca tgtctcattc tggttctgat gcttgtgcag tctcttcaaa ctgcgtcttt 600

gccttttagt gtgccttgca atgtggaaat gatatactgg gtaagaggag ctgtagtaaa 660
gaggcttcta gtgacgtagt gacaagctgt ggggagaggg agtgttgcac agtcctgccg 720
catgtcacag tcttccagtg agcctgtgtc cctggactgt gaacttcatg cttgcttctc 780
agcttcccca gccccttaga tggtacagaa ctgttggagg ggggtggagt tgtatatttc 840
ccttgctctg ggtaggtcac cctctgataa aacaccaggt taggcctctg gtgaaataat 900
ttctcctgag ggcagacctt ctattaataa tagaatgttc caacctattt caaaatggtt 960
cctcttctcc ttccactgcc agaagcataa tgagattttc cccctaatat tcgtggtaag 1020
gacctagcag agctccagga ggtaacactc tcaagtgtct catactaccc tgcaccatga 1080
ctgggctctg ctggagttct taatttgcag aactgcccac actgagcctc ccgcaatttc 1140
tcaattacag ggcaaacttt cccagccggc actgggtcct tggaggtttc tgtctgctgg 1200
tttcttcctc tggaggttgt gcttctgtgt ttgcctgtct ctccaatttg gggggcagtg 1260
gtttgcccaa tgacctcaat tctctgaaag agctaagaag aggtgttaat ttttcggttt 1320
gctcagcttt ctacttgttg ctagaatgga gcgccaatag tgcctcctat agtgacatgt 1380
aaccctcaac tctagagatg atgaagcata ctaatgacaa aggagaaatg cttcagcagt 1440
tttctgtcag cacattaccc cttgaaaaag ctgcttcttc cacattctgc aagagatggg 1500
tctcaactca gagctcaagg caaatgactt ccttcaagga gaaggaataa acagtctcag 1560
aaaccatgaa agcctgcccc caggagtgtc cctgaacctc agcaggggcc acacttacct 1620
tgcagaaata ggtgaggcat gctcctggta caaaatccca atggtacaga aggacaaatt 1680
gaaaaacaag tctccctcta aacccctgac cccgagctac ctagttctcc tccctagagg 1740
caaagctgtt accagattct tgtatctcct taacatatat ccttagaaga gctgtcaagt 1800
gaacacatgt ttaagtgaaa acctatttta gaagtgcatt ttcttaagga actttagggt 1860
tggaaggaac ctgtgtcagt ccttaattca caacctccat tagtacttat tgttcttgca 1920
caaaaatctt tctcaaaaaa gccctttcca ctctgacata gcttattcta cttttactta 1980
gctccaataa cttataaaac atatttttga aagtctaaaa tctgccacta tgtttttttt 2040
tcctaatcaa tctttacttt gacctctaag ccagagaaaa caggtggtca aatgcctttt 2100
gcctaagatg gaacttagaa tatttgaaga cctcagatct tcaccctgcc aaataacgtg 2160
tttctcctcc cctttcacag agcatttggt tttaggaaat tcagagccac attccttata 2220
gacaagacta aactcttatt caacatactc agaaacttct tctaagagga taaccactca 2280
tcagaggaaa aaagtttctc atgtacagct ggcaaaggga tggaaccatc tgtgttatta 2340
aaattgacag acgcttatga gatttattaa gggaaatact agagtcttag tacatacttg 2400
ctaatatagc atacatgaag gctttatcta taattttttt ggccaagcag aaattttggt 2460
attactcacc ctaacaaatt tccaagacat tatgaaatag aattttaggt cctgacatca 2520
ccatttgtct caggttttga agcgttgctg gacaagaggg gtaaaacacg gctctgcctt 2580
ggattcaaag ttggcctctc atactagcaa gtataccttg gtatcctggt cacttctccc 2640
ggccacagca tcacattgct ataaaaggca gatacaagta ttaaccagct cacaggttat 2700
cagataagct tagtctgacc aatgcttaac acagcaactg ggccactatt gtcattcctg 2760
tggtggtggc acacacaccc agcctctgtc cgggccatgg tctaggacca ccctccacag 2820
aggctgtgag ctagagccct aactgtgcag ggccctaact atgccaggct acttatctct 2880
cttaagagga cttcattagt gcctgctcgg ccatacagtt ttttacttac caagtaacac 2940
agttatcagc acactccagg tactagccaa ggactacaaa atcaacgtga atgtcagctt 3000
ttgtatcaaa agctcaaagg agaaactcaa actttacata gatgtcccat gaagatgttc 3060
agcaaaccca ttcttctctg ttccctggaa tccatcccag tattgtgcta tgtgtgtgtc 3120
tagtaattct ttacaaaaag ctctgtttct tgtgatgcta tcagatcaca ttgaagaata 3180
tacaagccgt actatgaagg ctgttgtctc atatagtcct aacgtagtga gaactgatgt 3240
tcttacatgc tgtctttttg ggcactcaaa gaaattcctg tacagtctta caaatcagtt 3300
gtagcttaaa ttgatttgtg ttgtgacttg tacacacagg tcacattccc ttgacagaaa 3360
atatagttta aaaccaaatt tgcagccctt gttaagtgaa tgcacaggac tttattgtat 3420
tcaggtcttt tattgtaaga ctcactcctg tcttcatttt atgttccact gttgtgcttc 3480
ccatttgcct ttctctagtt ttgttttctg tgtttctacg gactgctctc agcccaggtg 3540
tgcaggaagc acacacatgc ctgcagagcc ttcatggcct ctgcattcag ggcatgactt 3600
caacgcacag tggctgtact gatttgttaa aacaaaggaa cagattactt ctcctaattc 3660
acagggaagt tccaggttgt gcgggcagtg agcagacctg tgtctgtctg cgcttgccct 3720
ggtgaaaaac cccaccgttc aggctgcagg gtgcgagacc caggcacaaa cattttgctg 3780
gatgaggagg aaagatgtaa ggttgctccc cttcagagac agcaaagggc aggtctgtag 3840
cttcacttac ttcaggattg tgatttttga cagagccgag agatcagggt tgttgaacca 3900
ggcctgaagg tcctagtgaa tctcgtgaag agaggagggg tctggctgta acatggacct 3960
agaggacatt tttactgcag gagaaggaac agtggggatg gggtggactt gccaaaggaa 4020
tatagctcaa gttcctgcag cccaaaaaag ctcagtttct tttggccaaa gcttccgcga 4080
gtttccctgg catttctcct gcgggagcta caggggcagt gggacactta gcctctctaa 4140
aagcacctcc acggctgttt gtgtcaagcc tttattccaa gagcttcact tttgcgaagt 4200
aatgtgcttc acacattggc ttcaaagtac ccatggctgg ttgcaataaa cattaaggag 4260
gcctgtctct gcacccggag ttgggtgccc tcatttcaga tgatttcgag ggtgcttgac 4320
aagatctgaa ggaccctcgg actttagagc accacctcgg acgcctggca cccctgccgc 4380

gcgggcacgg cgacctcctc agctgccagg ccagcctctg atccccgaga gggtcccgta 4440
gtgctgcagg ggaggtgggg acccgaataa aggagcagtt tccccgtcgg tgccattatc 4500
cgacgctggc tctaaggctc ggccagtctg tctaaagctg gtacaagttt gctttgtaaa 4560
acaaaagaag ggaaaggggg aaggggaccc tggcacagat ttggctcgac ctggacatag 4620
gctgggcctg caagtccgcg gggaccgggt ccagaggggc agtgctggga acgcccctct 4680
cggaaattaa ctcctcaggg cacccgctcc cctcccatgc gccgccccac tcccgccgga 4740
gactaggtcc cgcgggggcc accgctgtcc accgcctccg gcggccgctg gccttgggtc 4800
cccgctgctg gttctcctcc ctcctcctcg cattctcctc ctcctctgct cctcccgatc 4860
cctcctccgc cgcctggtcc ctcctcctcc cgccctgcct ccccgcgcct cggcccgcgc 4920
gagctagacg tccgggcagc ccccggcgca gcgcggccgc agcagcctcc gccccccgca 4980
cggtgtgagc gcccgacgcg gccgaggcgg ccggagtccc gagctagccc cggcggccgc 5040
cgccgcccag accggacgac aggccacctc gtcggcgtcc gcccgagtcc ccgcctcgcc 5100
gccaacgcca caaccaccgc gcacggcccc ctgactccgt ccagtattga tcgggagagc 5160
cggagcgagc tcttcgggga gcagcgatgc gaccctccgg gacggccggg gcagcgctcc 5220
tggcgctgct ggctgcgctc tgcccggcga gtcgggctct ggaggaaaag aaaggtaagg 5280
gcgtgtctcg ccggctcccg cgccgccccc ggatcgcgcc ccggaccccg cagcccgccc 5340
aaccgcgcac cggcgcaccg gctcggcgcc cgcgcccccg cccgtccttt cctgtttcct 5400
tgagatcagc tgcgccgccg accgggaccg cgggaggaac gggacgtttc gttcttcggc 5460
cgggagagtc tggggcgggc ggaggaggag acgcgtggga caccgggctg caggccaggc 5520
ggggaacggc cgccgggacc tccggcgccc cgaaccgctc ccaactttct tccctcactt 5580
tccccgccca gctgcgcagg atcggcgtca gtgggcgaaa gccgggtgct ggtgggcgcc 5640
tggggccggg gtcccgcacg tgcgccccgc gctgtcttcc cagggcgcga cggggtcctg 5700
gcgcgcaccc gagggcgggg cgctgcccac ccgccgagac tgcactgttt agggaagctg 5760
aggaaggaac ccaaaaatac agcctcccct cggaccccgc gggacaggcg gctttctgag 5820
aggacctccc cgcctccgcc ctccgcgcag gtctcaaact gaagccggcg cccgccagcc 5880
tggccccggc ccctctccag gtccccgcga tcctcgttcc ccagtgtgga gtcgcagcct 5940
cgacctggga gctgggagaa ctcgtctacc accacctgcg gctcccgggg aggggtggtg 6000
ctggcggcgg ttagtttcct cgttggcaaa aggcaggtgg ggtccgaccc gccccttggg 6060
cgcagacccc ggccgctcgc ctcgcccggt gcgccctcgt cttgcctatc caagagtgcc 6120
ccccacctcc cggggacccc agctccctcc tgggcgcccg cgccgaaagc cccaggctct 6180
ccttcgatgg ccgcctcgcg gagacgtccg ggtctgctcc acctgcagcc cttcggtcgc 6240
gcctgggctt cgcggtggag cgggacgcgg ctgt 6274

<210> 24
<211> 3002
<212> DNA
<213> Homo Sapiens

<400> 24

acatttaaat tgcatatttg gcttgtaata tattacatat ttaaaagaat tactattttt 60
ctgaatacca ttaatactaa taatagcatc acagtgacta agagtggact taaataaagg 120
ctggcttgaa actcaggtct gccatttatt agcaagcttc taaaaattct gagcccttag 180
ttttctcaca tgtgaaatgg agaaaataaa tctgcaaaat taattaaatc tgtccattca 240
tatttttctc tccagtgtat attaactggc attcctcgtt aggccagaat gtgctctcaa 300
ccatgctcca aatccgcttt gtgccaaccc cactgccaga accctttcta ccttgagaac 360
cagaaaagga aacattatgc ctggcaatgc ctacaccctc caaaataaat ctgcaggaaa 420
gaacacccag taagtgatga gagcagcaac gactgccttt atcattttaa atttacaaca 480
ccaccttttc tagagcctct taagcattgt agataattcc ccactcatta aaaaataaat 540
tgtaaccata agtattcagg gttgatactg cttttgaatt agacagtgct catatcagtt 600
gcataagacc aacctaaagt agaggatgaa atcttttttc tgaacctttt tcagaacgta 660
acttagtgaa tatattaaaa ctaaactttc tttgaatggg agtaatttct acggattaat 720
ctgtaatctc ttagaccaca cctaaggtaa tgtagaggtt gttgtatcat aggctttgtg 780
attagagacc actggatttg tctttggaaa agtcactcat gattctttgg gctttggttt 840
cctcatcttt tataccggct taataatgac caccgtagag ttgtcatgga agttaaatga 900
actcatgtac cataagtgac caatacaatg attgacactc agtgatttat caataaatta 960
aaacatttat tatcaatatg acagagaagg tgccgctaaa atagacaata ggtttttgga 1020
agaggtgatt aaatggatgc aaaatttatg gattgtttat tccgtctacc tttgctgtgt 1080
cccctggttg tggcatacac acgtgtgggt ataaaatcgt aaatcctatg tagtcgcgta 1140
gtgcatgcgc agaaggctta gacacgaaat gtcatttcag caatgtgcct agagaagctc 1200
tgacgccgcc ttggaagtaa gtcgttgctg cctgaccttt gggcgtctgg gacggatgcc 1260
tatacctgca cccagcagca ctggaagggg cccaggccct tcgcagcaca gcctatcccc 1320
agaccgctta gtccttcata acatatatct ccacggaaaa gggtatttcc tcccgtcaga 1380

aaaagcgccc cagtctggtc tgggttggtt tttatttcac gttgttgcaa gtaggcgaag 1440
tcccttctgt ctcctccctt ggggtaagtg gaaaggagtc cggcaggggg cccgcagtgg 1500
cctgcacagg ggaactgggt agcgagagag ttccaggcaa ttccgggggc tgccccacag 1560
aagcaggtgg ggatcgacag tggctctccg gcccagggag gagagcgcgg tcgcgggtcc 1620
ctcccctcag cctggaggct gcagccgctc gagtcggccc gggtgggggc ggggtggggg 1680
cggcgcggag ggcacggaga ttacggcggc gccacccggg acatccaggg ccccgaggcc 1740
ctgggcggtc cccacgcgag atcgcaaacc atgacaatag gcagtcaccc gaggtcaaat 1800
aaaaacggag tgggtccccc gcgcgccgcc gccccccgcg tccctggcgg cctcccccga 1860
ggcccccggc ggcctcacga gcccgcagta gccggtggcg acgtcgcccc cgccccacct 1920
ccctgcgcaa gtgcgaggct gccggcagcg cggcgcacgc tccggccgtt cccggcttcc 1980
gcgcaaaact tccatcctgt ccacgtgaag ttgtcgctgc cttagagagg gggaaagagc 2040
tgcgggaaaa gccggggagt gacgactgcg gcggctgggc gcgctctctc atttcttttt 2100
cttctccttt cccccctgtc gcagtccgga gttttggctc ctctcctttc ctcctccccc 2160
tcggagccgg cttctccctc cgccccgctt ctcccccgct tgtgtacgct atttgttgtg 2220
gggtggccga aggggatgtc ctgttttcac cagaggcaca gcgcgaaggg gaaacttcga 2280
cactggaagg aacgagaata aatacttaat tacggacgca ctgaaccgcg gctgggacag 2340
acacttcggg aacccgaggc ggaccgggcg acgaggtgag tgaccccttc ttccaacccc 2400
cgccccaggg ctcccggggg agcctgagtt gagagaaccc ccaaactttc cgggaaagtg 2460
cgcgaggctc cgccggggac gccgagcgct gggtactgag gacgcgcagc tggacggtgc 2520
gtgggcgcct gcgtccccgg ggggcgcttg gaggccgggt gccccacgcc tgagggcccg 2580
ggccgctcgg accgcagcgg tgctctctgc cctagaagac gtccccaagc cccaagggtc 2640
ccttccgagc ctgcctgtcc cttccggggt cggcgcggag cctgcgcgta acggagttca 2700
tccagcagtc cagcgcgcgg cttctacctg caccccgcct ccacctggca gaggcgcgag 2760
catcggggtc tcccccacat ctttcttatg acgtgtatta ctttctgatg accccctaga 2820
tggtccaggc gcgaggatgc tgacccagag tccttcggag ggtcacaggc gcctgggctt 2880
tcccggtgcc gggtgcgtgt gtactttaaa ggctcgcgtt ctaatctcca ggcactgatc 2940
gggcttttca actgcggcga tcccacttta atagttttta tgtggcgtgg actgaatgtc 3000
-- 3002

<210> 25
<211> 5649
<212> DNA
<213> Homo Sapiens

<400> 25

cggtggctca cacctgtaat cccagcactt tgggaggctg aggcaggcgg atcacaaggt 60
caggagatcg agaccatcct ggctaacaca gtgaaacccc gtctctacta aaaatacaaa 120
aaattagccg ggtgtggtgg cgggcgcctg tagtcccagc tactctggag gctgaggaag 180
gagaatggcg tgaacccggg aggtggagct tgcagtgagc caagatcgtg ccactgcact 240
ccagcctggg cgacagagcc agactccatc tcaaaaaaaa aaaaagaaaa aagaaaatat 300
cttaagcttt gaggaccaca tagtctctgt tgcaatcact catttgggcc actgtagcac 360
aaaaacaaac atagataaca cacacatgag tgagcatggc tggggcccaa taaaacttta 420
tttacacaag caggtgatga accacggtcc atagtgtccc aatccttgat ttagatggtg 480
aactatcatg agctttcgtt gccctctccg gtagaaatat gggagaaacc acagagagaa 540
acagagattt caggaaaaca gtaaaattaa acaaataaag tgagacgtcg cccctcctgg 600
aggctgcagg ctgtgttgaa cctgtgtgtg tctgggtgta cagggatgtg agtagatgcg 660
cgtggagatg tgtgtgtggg tggagactga gcgtggctgg aggtggctgt ggtaggatgc 720
cagaaggaag gagtttgcat tcagttcctt tattagtact ctctggagtt gttgagggga 780
tgtcactgct gaacactgac cctggtgcag gctaaaccga agttagcctc tgctaccctc 840
tgctgggtga ggcactgggg agaacagtgg gactgaatgg ggcgggcttt ctccctccca 900
cacttcccct ggaaacaccc agcaccagca cattagaaca tggggagcca ccttccccag 960
cgctgcttcc caaggttcgg aaggggaaac tctgacctca agctctgttt gaagagcgat 1020
agcaaagggg agaggttggc tgtggtttgg tggtgcttat ctccttcctt tgctttgaaa 1080
ccacaggctg gcatactacc cgagagaatg accagtgcca ccaagggaag ggcacaagct 1140
cactggttcc aggggagaca tctcagggga caggaacatc ccctgacgct gaatgagtgc 1200
agcacacaga cggagaatac acacaagaga aatcatgagg acatgagaga aggcactggc 1260
aataggaagt tacaaaaagc aacaaaaaga aagagtagat ataaaaacaa taatcgaaga 1320
aagccaacaa cagatgggat gaatcatacg aggtatgcga ctcatggatg aagaggtgag 1380
tgatgagatc agctggggaa ctctctccca ggaaagacaa accagaagaa acacaaatga 1440
catcgaagtg gccaggcgca gtggctcacg cctgtaatcc cagcactttg ggagcccgag 1500
gcaggtggat cacttgaggt caggagtttg agaccagcct ggccaacatg atgaaaccct 1560
gtctctacta aaaatacaaa aattagccag gtgtggtggc aggcacctgt gatcccagct 1620

actcaggagg ctgaggcagg agaatcactt gaaccaggaa gcagagattg cagtgagctg 1680
agatggtgcc agtgcactcc agcctgggca gtagagtgac agcctgtcta aaaaaaaaaa 1740
aaggcagtaa agtgatagga ggatagatta gaaggccatt tttccagata gtgcaaggaa 1800
gaaagaagaa gaagaagagg aggaggagga ggaagaggaa gaagacggag aagagggcat 1860
ctcagaagaa acaatggaga tacatttctc aaaactgaag aaagatgacc aacctcagac 1920
cgaaggccaa gaggcaaacc agagagacag cttggcaagc caggaagagc accaaagcta 1980
gacaattaga gtgaaattaa agaaaaccca aggtgacaaa aatttaaaaa gcttccttgt 2040
gacctcagtc cattggtgaa aatttaaaaa aaaaaaaaaa aaaaaaagga cagcctgggc 2100
aacatggtga aaccccatct ctacaaaaaa tacaaaaatt agctgggtgt ggtggcacat 2160
gcctgtagtc ccagctattc gagaggctaa gtaaggcagg aggatcactt ggggccaaga 2220
gttccagacc agctgcggca acatacatgt tgtgggaagt caggcacccc gaacggaggg 2280
actggctgaa gccatggcag aagaacataa attgtgaaga tttcatggac atttatcact 2340
tccccaatca atactcttgt gatttcctat gcctgtcttt aatctcttaa tcctgtcatc 2400
ttcgtaaact gaggatgtat gtcgccttag gaccctgtga tgactgcgtt aactgcacaa 2460
attgtttgta gaggacaatt gttgtgtgtt tgagcaatgt gaaatctggg catcttaaga 2520
acaggataac agcaattttc agggaacaag ggagataacc ttaaagtctg gctgcctgtg 2580
ggctgggcag gacagagcca tacttctctt attaccgcaa acaggtaaga gaaatatcgc 2640
tgaattcttt ccccagtaag gaatattaat aattaacagc cctgagaaaa gaatgcattc 2700
ccagggccgg gccactgaaa tggcctccct gagagtgcct gcctttattc agaagtagaa 2760
agggatgaaa cacgccctag tctcctgcag cacccccaga cttgatagga ttaggaaatt 2820
ccagcctggc aaattctagt cagaccggtt ctctgctctt gaaccctgac agtgcgtgcc 2880
agtgggacct ggaagttcat tagtgattct agtttcgccg tgaccttctg ccctgtgatc 2940
ttttgtcgcc cttgaagtat gtgatctctg tgacccacac cctgttcgtg cactccctcc 3000
tctttgaaaa ttgctaataa aaactcgctg gttttacggc tcagggggca tcacggaacc 3060
tgcggacatg tgatgtctcc cctggacacc cggctttaaa atgtctctct tttgtactct 3120
ttccctttat ttctcaggcc agccgacact tagggaaaat aggaaaggac ccacgtgaaa 3180
tattgggggc tgaatttccc ccgataataa gtctcttaaa aaagactttt aaatgaaatt 3240
tctttgtttt ttaactttta tttctgtttt gggggtacat gtgaaggttt gttacataag 3300
taaactcgtg tcttcggtgt ttgttgtaca gaatatttca tcacccaggt attatgccga 3360
gtacccaata gttctctttt ctgctcctct ccttcctccc atcctgcacc ctggagtcaa 3420
ccacagtgtc tgttgtttcc ttgtttgtgt tataagttct catcatttag ctcccactta 3480
caagtgagaa catccagtat ttggatttct gttcctgcat tagtttgcta aggataatag 3540
cctctagctc catccatgtc ccacaaaaga catgatctag ttctttttaa tggctgcatt 3600
aaatgaagtt ttaaagatac aacataaaca ccaacctctt ccccaccaca aaaatccctt 3660
gctgaatttg attacactta aattaacgag ttttgtttca tgaaagactc cttggacaaa 3720
cttgacagtt gatggaatag gagaagctgt ctgtcatgtc taaagccaac aagagatcaa 3780
tatctagaat aaatggagat ctgcaaatca acagaaagta ggcagcaaag ccaaagaaaa 3840
tagcctaagg cacagccact aaaaggaacg tgatcatgtc ctttgcaggg acatgggtgg 3900
agctggaagc cgttagcctc agcaaactca cacaggaaca gaaaaccagc gagaccgcat 3960
ggtctcactt ataagtggga gctgaacaat gagaacacat ggtcacatgg cggcgatcaa 4020
cacacactgg tgcctgttga gcggggtgct ggggagggag agtaccagga agaatagcta 4080
agggatactg ggcttaatac ctgggtgatg ggatgatctg tacagcaaac catcatggcg 4140
cacacaccta tgtaacaaac ctgcacatcc tctacatgta ccccagaact tcaaataaaa 4200
gttggacggc caggcgtggt ggctcacgcc tgtaatccca gcactcttgg gaagccgagg 4260
cgtgcagatc acctaaggtc aggagttcga gaccagcccg gccaacatgg tgaaaccccg 4320
tctctactaa aaatacaaaa atcagccaga tgtggcacgc cacctataat tccacctact 4380
cgggaggctg aagcagaatt gcttgaaccc gagagcggag gttgcagtga gccgccgaga 4440
tcgcgccact gcactccagc ctgggccaca gcgtgagact acgtcataaa ataaaataaa 4500
ataacacaaa ataaaataaa ataaaataaa ataaaataaa ataaaataaa ataaaataaa 4560
aaaataaaat aaaataaaat aaaataaaat aaagcaattt cctttcctct aagcggcctc 4620
cacccctctc ccctgccctg tgaagcgggt gtgcaagctc cgggatcgca gcggtcttag 4680
ggaatttccc cccgcgatgt cccggcgcgc cagttcgctg cgcacacttc gctgcggtcc 4740
tcttcctgct gtctgtttac tccctaggcc ccgctgggac ctgggaaaga gggaaaggct 4800
tccccggcca gctgcgcggc gactccgggg actccagggc gcccctctgc ggccgacgcc 4860
cggggtgcag cggccgccgg ggctggggcc ggcgggagtc cgcgggaccc tccagaagag 4920
cggccggcgc cgtgactcag cactggggcg gagcggggcg ggaccaccct tataaggctc 4980
ggaggccgcg aggcttcgct ggagtttcgc cgccgcagtc ttcgccacca gtgagtacgc 5040
gcggcccgcg tccccgggga tggggctcag agctcccagc atggggccaa cccgcagcat 5100
caggcccggg ctcccggcag gctcctcgcc cacctcgaga cccgggacgg gggcctaggg 5160
gacccaggac gtccccagtg ccgttagcgg ctttcagggg gcccggagcg cctcggggag 5220
ggatgggacc ccgggggcgg ggaggggggg cagactgcgc tcaccgcgcc ttggcatcct 5280
cccccgggct ccagcaaact tttctttgtt cgctgcagtg ccgccctaca ccgtggtcta 5340
tttcccagtt cgaggtagga gcatgtgtct ggcagggaag ggaggcaggg gctggggctg 5400

cagcccacag cccctcgccc acccggagag atccgaaccc ccttatccct ccgtcgtgtg 5460
gcttttaccc cgggcctcct tcctgttccc cgcctctccc gccatgcctg ctccccgccc 5520
cagtgttgtg tgaaatcttc ggaggaacct gtttccctgt tccctccctg cactcctgac 5580
ccctccccgg gttgctgcga ggcggagtcg gcccggtccc cacatctcgt acttctccct 5640
ccccgcagc 5649

<210> 26
<211> 12781
<212> DNA
<213> Homo Sapiens

<400> 26

cactatgttg ctagctggtc ttgaactcct ggcctcaagt catccttctg tcttggcctc 60
ccaaagtgtt gggattgtaa gtgtgagcca ctgtccctgg ccagttggtg atttatttgt 120
ataactgtcc aatttattga atacgtatgg cgtgccaagc actgggctga gggcttcata 180
atgccctttc actcaatgct tagcacaacc catgaagaag gtagtgttaa tatcatccct 240
gttttacaga tgtagaaact gaggcacagg ctaaataact tgcccaacaa gctcgtgcag 300
tttagtaagc agctcagctg ggatgtgaac acaaactttg aatacagagc tcttaaccag 360
taggccagag gttcccaaac acctagtata tttactacct tagtactttc ctgccacatc 420
tcctaggcca aaacaactcc attgattgtt atgcaattta ctaagtgtag ccatttgaaa 480
aaaaaataca tataaaagaa aatatttttt attcagtttt caaaataacc catatatagt 540
catggaatgt atgtgttggt tgggcactgc agactaaaga cagtttaggg ccgggcacgg 600
tggctcaggc ctgtaatcct agcactttgg gaggccaagt agagagaagg gcttgagccc 660
aagagttgga gaccggcctg ggcaacatag caagacccag tctctacaga aaataaaatt 720
atcagggtgt gaggacgcac acctgcagtc ttagctgctt gggaggctga ggctggagga 780
tcagcctggg caacagagtg agaccctgtc tcagaaaaaa aaaaaaaaaa agacagactt 840
ttattcagat atgcatgcag gagttcacag aaaaaaaaag tgagtccagg aggctgttat 900
ttggcattta tacaactttt tttttcttga atctcgaaat ctactttata tataccattt 960
aataggggaa gaggagggag aaaaagcctt ccatgggaag aacaaatagg tttctggggg 1020
aacaaaaggg agataagaat gtttgttttt gcaggtgcaa gtggtctttg tctttttttc 1080
tggccactaa aactccccta gagaggagat ttacggcagc ttcactccca gaaatttctg 1140
ctgttagtcg cataagggaa gctttgaaac ggcatctttc tgcatctgtt ggctctcaaa 1200
tgtcttcagt tccaagtaac attcatgcca attctggggg tctgagtgtc cccacataat 1260
acatgtgttc tcttgtcttt taatgaagtt tgtgggaggc atctaactgt agcctccaaa 1320
atttggccca taggtactac tgtccttatc aaagacgagg aaacaagttc agaaaagtat 1380
taattgctcc gagttatctg cttggctagc taggatcaga gctcagttct ccatttaacc 1440
caaagcccag gctcttaacc tcttacaact ggcgcatccc ctctgaacct ccatttcctc 1500
cctgtaaaag aataacatcg gccgggcgca gtggctcaca tctataatcc cagcactttg 1560
ggaggcagag atgggcggat cacgaggtca ggagtttgag accagcctgg ccaacatggt 1620
gaaaccccat ctctactaaa aatacaaaac ttagctgggt gtgttggtgc ctgtaacccc 1680
agctactcag gagactgagg caggagaatt gccttaacct gggaggcgga ggttgtggtg 1740
agccaagatc gtgccattgc actccagcct cggtgacaga gcaagactcc atccccaaaa 1800
aaacaaacaa caacaacaaa aagagaataa cgttatattc agttgaacca aaatgaatta 1860
aatattaata tttgtacttc aaaaacggtc cagcttggct gggcgcagtg gctcccgcct 1920
gtaatcccaa cattttggga ggccgaggca ggaggatcat ttgaggtcag gagtttgaga 1980
ccagcctggc caacatggtg aaatcctgtc tctactaaaa atacaaaaat tagctgggca 2040
gtagtagcgc gtgccggtaa tcccagctat tcaggaggct gaggaaggag aattgcttga 2100
gcttgggagg tgaaagttgt ggtgagctga gactgcacta ctgcacacca gtctgggaga 2160
cagagtaaga ccctgtctca aaacaaaaca accaaaaaac caaaaaggtc cagcttgggc 2220
aacatagtga aacttcgtct ctacagaaaa tttttaaaat actagcaggg caccgggcac 2280
agtggctcat acctgtaatc ccagcacttt gggaggctga ggcaggcggg tcacttgtgg 2340
tcaggagttt gggatcaggc aggccaacat ggtgaaaccg tgtctctact aaaaaacaaa 2400
aattagctgg gcatggtggt aggcaccagt aatcctagca ctcaggaggc tgaggcatga 2460
gaattgcctg aacccgcaaa gcaggggttg cagtgaacca agatggcgtc actgtactcc 2520
agcctgggtg acagaataag actcctcaat taaaaaaaaa aaaaattagc tgggcatggt 2580
gttgcgggcc tgtggtccca ggtactcagg aggctgaggt gagaggatta cttaagcctg 2640
ggaggttgag gctacagtaa gccaagatca cgccactata ctccagcctc tgtgacagag 2700
ccagaccctg tctcaaaaaa attttaaaaa gggcaaattt tggcaatttc acatagttca 2760
acctagtata aggtggttgt aataactaaa tgagataaaa tggtgttaaa ttggaagtat 2820
tatagtattt ctgttaacaa catagggctc cagaaccagc ttccttgagt ttaaatccag 2880
gctccaccac ttcctagcta tgcagtcatg ggcaagttac ttgacccaac tgtgcctcag 2940
cttcatccat gatatggaga tacaggataa ccagcctctt acgtgcaatt ctgaaatcca 3000

aaaagctctg taaaccaaaa gtttgggggt aaactcattt ggtagcaaat tttgacctga 3060
ggctatttat agtctatatt ctgtattctt tctacttagt atgaataagc atgtaagttt 3120
tactgcatgt ttgatttcag catgttcccc cagactctct gggggtgttt acgtatgccg 3180
gtgggggaaa gagaccaact ctcaaatatt atctcaaaca gttggtttca ctgtgcttgc 3240
ttgggtagca catataccaa aattggaatg acccctgcac agggatgaaa tgcaaattcg 3300
tgaagcatac tgtatttttc ttagcacata ccacctttgg caatattctt tttttttttt 3360
tgagagggag tcttgctctg tcgcccaggc tggagtgcag aggcgcgatc tcggctcact 3420
gcaagctccg cctcccgggt tcacaccatt ctcctacctc agcctcccca gtagctggga 3480
ctacaggcgt gtgctaccac gccaggctaa ttttttgtat ttttagtaga ggcggggttt 3540
cactgtgtta gccaggatgg tctcgatctc ctgacctcgt gatccgccca cctccgcccc 3600
ccccccgaag tgccgagtgc tgggactaca ggcgtgagcc actgcgcccg gcccccgcct 3660
ttttttttta gattgatttt attacttgcc tagcaaagga gaaccttctg gcagaacagt 3720
ctccaagaac aaggcaaaca actaatttta cataggtttt taccaatgta cagctgttga 3780
ttgtgactgg tttccggcaa tctggatttc acaatctgga taaggggaca aacaattgtc 3840
tgtcttccac tatctttctt gaatttgaat agaacctttt tattctcata gcctcttagc 3900
tttctttctt ttttttttga gacggagttt cgctcttgtc gcccaggctg gagtgcagtg 3960
gcgcgacctt ggctcactgc aaacgctgcc tcccaggttc aagttattct cctgcctcag 4020
cctcccaagt agctgggatt acaggcgcat gccaccacgc ccggctaatt tttggatttt 4080
tagtagagac gggggtttca ccatgttgac taggctggtc ttcaacgcct gacctcaggt 4140
gatccgcccg cctcggcatc ccaaagtgct gggattacag gcgtgagcca ctgcgcccgg 4200
cctctcatag tctcttagct ttctaaaatt tgaaaaatcc tgtaaagaca cacctgggtc 4260
aaagggctca gataacggac tgtggccctt aagtacttac gtcacaggtt attgagagga 4320
tcgatttagt taccagatgt aaaatgctgg gatcagtgcc tggcaaagga aaactttgta 4380
cagctgcagg ctttcaccat acacaacagc atcgctaacg aatgctatta caatattcat 4440
ttagcgttta ccaagtgcct actctataca aatcttgaga atacaacgtg aaggtgaact 4500
gctgactaaa gtttggtccc tttcgctccg tctccttgcg aaaatgctct aacggcagga 4560
ggtcacgcga gcgctggacg cgtttctccc cgcgagcccc tttccgaggc ctttcgggtc 4620
cccccggtta tccccgcccg ggcggtgcgc gcccccgctg ttcccgcttc cgctccagag 4680
aggcagggct ttccgagcct gctagccccg cggccgcaac taaccccggg tcggagtgtt 4740
ccggcccggc cagccccgcg gcgtgaggga aggggagctc agcagttccc cgcgcggggc 4800
ccaggcgtcg gcggcagggc gggcccctca ccgccagcgt gccagccccg cccctaccca 4860
ccagtgtgcc agccccgccc ttccccacgt cgccgcgcgc ccgggggcgg ggcctggcgc 4920
gcaccgcccg cgcacggcga ggcgcctgtt gattggccac tggggcccgg gttcctccgg 4980
cggagcgcgc ctccccccag atttcccgcc agcaggagcc gcgcggtaga tgcggtgctt 5040
ttaggagctc cgtccgacag aacggttggg ccttgccggc tgtcggtatg tcgcgacaga 5100
gcaccctgta cagcttcttc cccaagtctc cggcgctgag tgatgccaac aaggcctcgg 5160
ccagggcctc acgcgaaggc ggccgtgccg ccgctgcccc cggggcctct ccttccccag 5220
gcggggatgc ggcctggagc gaggctgggc ctgggcccag gcccttggcg cgctccgcgt 5280
caccgcccaa ggcgaagaac ctcaacggag ggctgcggag atcggtagcg cctgctgccc 5340
ccaccaggta gcggggtggg ggtggggtcg aaggcggggg catagcggcg gggcgcttgg 5400
aacccggcga ggggaggctc gcacaggggg ttgggggggt gcacggcctg gccctgggct 5460
cggaggaggc ggggccgcag agttggcttg aatgagtgca ggggtcgagt ctggagcatt 5520
tgggggtgta gcttgtaaac agggtcggag gagagaggct gtgcaggaag agggctgcag 5580
gggagacgcg gagagttcgg gccttttgga gggaggagac gcgtcccgcc aggtgggggt 5640
gctgggctaa ggaaggggcg acgcgcgcag ctccgggtgg ggagggggcc tgggaggtgg 5700
gagcactggg ggtggggcga gaaggggaag gcgcccggcc cacttggtgg gcggggcggg 5760
gggcggggtg gcgggaagga ggaatgcctg cgggaggccg aacggggaga gtccggtggt 5820
gtggggtgcg aaaggaggtt cctcggccgg cgcggagata gtgagttggg gctccagtag 5880
tcgatcgagg tagacactta gaggtagtta agagccgcgg tcgccgagac gccttgggga 5940
cggtgggcct tcggcctagg tgaggggccg ccgagggggt gggccacgag ctgcgagcgc 6000
gggggggtgt gtcaccatgg ggaccgcggg gcctaattgg gcggggcggg gccgtgggga 6060
gccgaagtgc tgggatccgg ctgggtcctt cggtaggtag gctgcacgtg caccgagacg 6120
aagatagaat attttgacgt atgtggaaat tcgtgtcgag tggaaaatat tttattttat 6180
gaaatagtgt aatttttatg gggcaccact gggcttttag aggccttaat cgggcgctgg 6240
acaaagatgt gtggacgtga gtgactccgg ggaagcctgt cgggagttgt cctcacttta 6300
tgggcagtta agtgcttttt tttttttttc ctttttgaga gagagtttcg ctcaagtcca 6360
ggctggagtg caatggcgcg atctcagctc accgcaatct ccgcgtcccg gcttcaagcg 6420
attccccagc ttcagcctcc cgagtagtcg ggattacagg aatgcgcccc cacaccccgc 6480
caattttgta tttttagtag agacggggtt tctccatgtt ggtcaggcta gtctcggaat 6540
tcccgacctc aggtgatcca cccgcctcgg cctcaaagtg ctgggattac aggcgctagc 6600
caccgcgccc ggtctgttta gggcttttta tccgggcagc tggcgacatt ttgaaaagct 6660
tgcttttgct gtttgccaga tacatatata tgtattttga gacagagtct tgctcttttg 6720
tccaggctag agtgcagtgg cgcgttcttg gctcaccaca acctctgtct ctggatcaag 6780

agattatcct gcctcagcct cccaagtagc tgggactaca ggtgcgcccc accacgcctg 6840
gctaattttt gtatttttag tagagacggg tttcactatg ttggccaggc tggtatcgaa 6900
ctcctgacct cttgatcggc ccgcattggc ctaccaaagt gctgggatta caggcatgaa 6960
ccaccgagcc cggccgtttg tcagatacta aacacaaagt ttaatggtcg ctatttgaac 7020
aaacgaagaa ataaaggctc agaaaaaata actcattcaa gataagagcc agttcgtgtt 7080
ttttgtttgg ttttgttttg aaatggagtc tcgctctgtc gcccaggctg gagtgctgtg 7140
gcgctttctc ggctcactgc aacctctgcc cgccgggttc aagtgattct cctgcctcag 7200
cttcccgagt agctgggatt acgggtgtgc ccaccgcggt ccggctgatt tttcaccatg 7260
gagtttcacc atgttggcca ggctggtctt gaaactgctg acctcaagtg gtccacccac 7320
ttcagcctcc caaagtgctg ggattacagg tgtgagccac cgtgcccggc cgctagttag 7380
tggttttgag taatggattt caaatccatt taaatccagt ttaaagtgtc ctaaaggaat 7440
tctgagattt ttctaagtgt aattatagtg ttacccttgt ttaagcgacc ctttcccgca 7500
gtttaaatat atatagttgt gcattagtag aatatgcttg tggggaacag agccagcatc 7560
cgcaataaca aactcctggt tagaaaagca tgacgtattg tttacttgag catgaattga 7620
ttgttgaatc caaaccaaac gggtgtattt attgtaagga tgtactttac attcatattg 7680
aatagcgtat gttatttgtt tcttgaggtt gagtttaaga gacttgtaaa aataaaacgt 7740
atacatttca cctcccgtta tggagaggat tccagggtat tcaagaaaga tgggcatttg 7800
atactaggtt tctaaagaaa ctgcagtgtc tagatcactc tgccgagcac agcattaggc 7860
attatggatc ctggatacaa ccatgaacag gacaaagcaa agaggcaatt gtagactcca 7920
agtggaaagg ggacggagag gatgcgggtc aggctaggct ctcagctctg taaaccgaaa 7980
ccagaaggac aaataagctt agacagatta tagtgagagt gggaagctgg ttcaggaaga 8040
ggaaggtctg caaattgtgg gtaggatgaa aggaggagga gggagcattg gagaagttaa 8100
gcagagatcc aatcatgaac agtctgatga gctacagaga cattcggact tactccatga 8160
atcatttaag ccttaaaaca tgttgagcgt attttttttt tttttgagac ggaatttcac 8220
tcttgttgcc caagctggag tgcagtggtg tggtctcagc tcactgcaac ctccgcctcc 8280
tgggttccag cgattctcct gcctcatcct ctcaagtacc tggtattaca ggtgcctgcc 8340
accacgccca gctaattttt gtgtttatag tagagacggg tttcaccatg ttggtcaggc 8400
cagtcgtgaa ctcctgacct caggtgatcc acccacctca gcctcccaaa gtgttgggat 8460
tacaggcgtg aaccaccgca cctggccgtg agccaccgtg tctgtccgag catcttttaa 8520
tgtttgtcat ttagatttct tcttgtgctg aagtgtttgt cttttgctgt ttcttttttt 8580
tttcctagtt ctttgtcatt tgtgtgtgat ataaatgtct tctttcacaa tgagttcttt 8640
catttagttt atggctttgt tgttgttgtt gaataataga ggtctcactt tgttgcccag 8700
gctggtgttg aactcttgct ctcaagcgat cctcccactt cagcctccca acctgttggg 8760
attacaagtg tgagccacca cacccagcct tatggcatct ttcgatgaac aaattattga 8820
ttataatgtg gaatttgtcc ttttattttc tctgtggtta gtgtttctat aggttttatt 8880
taagaaatcc acagggaggc tgggtgcagt ggctcatgcc tgtaaaccca acactttggg 8940
aggccaaggc aggccaacat ggctagaccc tgtctctcca aaaaataaga aaattagcca 9000
ggcatggtgg cgtgtgcctg tagtcccagc ttcttgggag actgagatgg gaggatcgct 9060
tgagtccagg aggttgaggc tgcagtaagc caagagatca tgccatgcac tccagcctgg 9120
gtggcagagt cagaccctgt ctgccaaaaa ataaaataaa agttggtgaa aatgttgatt 9180
atatatttta ggaacaacta gtaattgaca tcaaaattat gggctaaaga gaaagcaaaa 9240
ataatgtgat tttaaaccag aattcaaaag atctgtttag cgtatgttta gacaaagcca 9300
ttacttatta tatcaaagtt ttaacattta ttttgtgagc tgtcagcttt tcctcttaac 9360
atttttcccc accgtcttaa aaaaccccaa gaataccgga catttaagac tcacttaaag 9420
ctttaaaagc acttgcaaaa tcctaaaatc ataatttaag gtgtttttgg agggcaggag 9480
caatggtggc aggcagtgtt ttgctttgtt gcccaggctg aagtacagtg gcagatctcg 9540
gttcactgca ccctcgacct attcggctca agtgatcctc ccacctcagc tttctgagta 9600
gctgggaccc caagtgcaca ccaccccatg cctggctaat ttttaaattt ttttgtagaa 9660
acaaggtctc actgtgtagc ccagatggtc tcgaattcct gggctcttaa gagatcctcc 9720
caaagtgctg ggatcatagg tgtgagccac cacacctggc ctattttggc attcttgaaa 9780
accgcaggat taccacggat aaaattttaa aattaccttt aaagaattca ggtttacaca 9840
caaaaaaaat ttggtttgtt agcagtgagt gaagaaaaat tttgagaaat gtttaaaatt 9900
tttagttttg ttacacaata cattttacta cctgtttaat tatctttttt gactcagaaa 9960
ccagtttcct gggtccagga tgtttagtgg tactcttttt cttcaagctt tttagcattg 10020
gaggaactgc atattagtaa aatttttagt cttagcattt tatagcttac tgctatttct 10080
tttctttcat tctttctttc tttttttttt tttttttttt ttttttgaga tggagtctcg 10140
ccctgtcacc caggctggag tgcagtggca cgatctcggc ttactgcaac ctctgccttc 10200
caggttcaaa tgattctcct gcttcagcct cccgagtagc tgggattaca gatgcccgcc 10260
accatgccca gctaattttt atttttttag tagagatggg gtttcaccat gttggccagg 10320
ccagtctcga actcctgacc tcgtgatcaa cccgccttgg cctttcaaag tgctgggatt 10380
acaggcgtga gccaccgtgc ccagcctttt tctttttctt tttctttttt tttttttttt 10440
tgagacggag tcttgctctg ttacccaggc tggagtgtag tggcatgatc tgggctcact 10500
gcaacctcca cctcccgggt tcaagggagt ctcctgcttc agcctcccga gtagctggga 10560

ttacaggcgc ctgccaccat gcccagctaa tttttgtatt tttttagtag agatggggtt 10620
tcgccatgtt ggccaggctg gtcttgaact cctgacctca ggtgatctgc ctgcctcgtc 10680
ctcccaaaat gctgggatta taggagtgag ccactgcgcc cggcccagca tactgctatt 10740
tctttctttc tttcttcttc cttttttttt tttttgtttt tttttttttt tttttttttt 10800
tgtgagacgg agtctgtcgt ccaggctgga atgcagtggc gtttcttggc ctcactgcaa 10860
cctctgctgc ccgggttcaa gtgattctcc tgcttcaggc tcccaagtag ctgggattat 10920
aggcctctgc cactgcactt ggctaatttt tgtatttttg gtagagacgg ggtttcacca 10980
tcttggccag gctggtcttg aactcctgac ctcgtgatcc acctgccttg gcctcccaaa 11040
gtgctgggat tacagacctg agccaccgca cccggcccat actgctattt cttaacagca 11100
gagaaattat gtgtcagatt ctgtaagtgt aatggtatat aaaggataaa atgatgttga 11160
aaaacaaaat tttttgttta aatgcttatg tttctaatat tttatttcag aaaggaattt 11220
atttcaaaac tgataatggt tggatccagc ttttcacaca aacttttttt tcctagtgag 11280
gatgcacatt tatcctgtaa acaaatggaa gacattattt ttttaattgc ttgcttagaa 11340
atgaaataat tcttttctaa tgatctttta aagcatgaga cctcatacat catttaaaac 11400
aatttatact gtattttaca catgacaaag ttctaaggta acagcccttt tctaagacta 11460
aagttacagt cctccctttg tatctgaggg ggattggttg caggaccccc ctgtgaatac 11520
ccaaatcctt ggatgtccaa gtcccttatg agatgtagta tttgcatata acctatacac 11580
atcttcccct gtactttatc tctagattac gtacaatacc taatagaatg taaatgcttt 11640
gaaattagtt gttcagctgt atttaaattt tgtatttttt ttcctttttt ttttgagaca 11700
gagtcttgct ctgttgccca ggctggagta cagtacagtg atcacagctc actgcacctt 11760
taacctccca ggctcaagct gtcctgcctc ggcctcccca agtgttggga ttacaggtgt 11820
gagccatcat acctggtcac tgttttttat tggttttaaa tttttgattt aaaattttta 11880
atctaggttg gttgaatctg gactggaacc caaggatatg tttgttgagc atactgtatt 11940
tactttggaa tacaactaga atgcttaact tgtatgttaa aaatacttta tttggccagg 12000
cgcggtggct cacgcctgta atcccagcac tttgagaggc caaggcgggt gaatcatttg 12060
aggtcaggag tttaagacga gcctggccaa catggcaaaa ccctgactct acaaaaaaaa 12120
ggtaaaaata agccaggtgt gatggcgtgt gcctgtagtc ttggctattc aggaggctga 12180
gacacaagaa tcgcttgaac cggggaggca cgttacgccc tcagttgttg acttgagttt 12240
ttccgtagtt tgtaggggga gggtaataga gtattaggta gcttttggaa tacataggag 12300
tgtaactgga aaaagattcc aagcaagtct aatgaattag ataatttacc taattagtaa 12360
attatgtaat cagtatgctt tataataata ttgtgagtta gatcctgttt ctgatatgta 12420
cataccatat tgtataggtg ctactaattt ggagagcata tacagtgagt ccatgccttt 12480
ttcctgccat cagcattata ccaaaattct gccatggttt ttaaactttg attctgagaa 12540
agtttctcac cctaataaca taactatatt tgtgtttgtc ttcatagtta aatatgcatt 12600
atgatatcag cttgcataca ttttttaaat gacttgaata tctgacttta aaaattattc 12660
tagaatttct gtgcttcaat attaatgcca gaagacttgg aattgtttat ttgtaggtaa 12720
ctgcctttaa ggaaacttga ccaaatatta actaagttat gtatttcctt ttggcaacag 12780
t 12781

<210> 27
<211> 1631
<212> DNA
<213> Homo Sapiens

<400> 27

ggatccagaa catggggcgg actcaggcag cggcgggaat caggcggact cgtttggacc 60
gaagcctccg ccaggcaccg aagccgtgaa gtcgcctgcg cagcaaaggg agcctccggg 120
gggcgcccga gaccttggtg tcgctcgggg gccgctggta gccgcgcccg ctgcccctcg 180
cccggtgact cagccggtac ctctggcggg gccatggggg ccagaggaca gcgcgggggg 240
cggcgagcgc ggttgctaag ctctccaagg cctcggaggg acaggatctg ggcgcacctc 300
accggttgcc atggtaacgc agcgccccag cccctcgcgc tccgcggtgg agggaggcgc 360
agccaatcgg aagcggcgga gttctcgggg accccccccc ccccgctcca cgccgtgtgt 420
gggcgggggt caggaggtgc ggggcggagg cggagcgtgg gccgcggaga tccggcgttc 480
gcaggaggct tggtgcgcgg cggggctgca cggggccact tccggagtag tactgcgagc 540
agcggcgcga cagtgcgggg tcccctttct cccagaagag acgtcaccca cacaaacctg 600
accttcacgt ggggcgcggg accttgcggg gtcccagccg caggcgcccc tgttgtttcc 660
ttcgggcggg tgggttggag aagaagtcca cgcgggattc ttcaaaacgg cgtacagggg 720
gattctaggg ccccatggtt acttctttgg acccccggga ggcgctgtcc agccacttcc 780
agtcgccctg atgactcgtc gtgggttcct ttaggagacc cgaaagttca gggcgggctg 840
tgtgggtaac acctctgccc aggttcccgg agggccctac gtggctgccc ctggagtatc 900
ccagagcgct agggctgcgg gaaggggcgg gtgtgcttct ggaaacatga gcgcctgtta 960
gtatcagtgc ctggataggg ctggggacaa atcagtttat gccgcgcaca ccgaatccat 1020

gcgcctgagt gagggtgggt gtgttgggga gtgcgtccaa gtggacagtg ccgtacagta 1080
atgtctacgg ggagttccag gagagctcgg ctactcctgc gcaggataac ctctccccca 1140
ccacccgagt cccgtgctcg cgggcaggac ttttccgaac tggggctgtg tgcctagaaa 1200
tacgtacatg ggagcgctca gctcaaagcc ccagggtttc tgggactcgc gtgtccgggg 1260
tcggggtccc aggtgggtac aggtgggagg gcgaacctgc gggtagggtg ggcccctccc 1320
cgcgggctca gcatctgtgc gcctccagct caggtgcgcg ggaggaaggc agcggcctgc 1380
cgcgcagagc cctgcgcgcc cgcgaggtgg cgccatagcc gcagcagcgc ccgccggccc 1440
gggccgctcc agataagagt gtgcggaaag cgcggcgggg ctgagacgcg accaggacgc 1500
ggggaggacg gaccagcagg acagaccgac cgggggcccg gcgggcggag ggcagcgcag 1560
ccacgtcccc cctggatccg ccgtcagccg ggcccggggc tttcgacatg ccccccaggt 1620
gggtcctcga g 1631

<210> 28
<211> 9254
<212> DNA
<213> Homo Sapiens

<400> 28

tgggggaaac tgaccccatg attcaattat ctccacctga ccccaccttt gacatgtgag 60
gattattaca attcaaggtg agatttgagt ggggacacaa ccaaaccata tgagtggaca 120
tgcaatatga gtgagaaaca attctgtgat tgtaacccag taagattttt ggatggctaa 180
cacagcataa cctagagtat atggaggtgt gcatttccag ctctttgact tccgggtacc 240
tagtaggatt gcactttcct gcacccttta agtaaggcat ggccatgtga ctaattttag 300
ccaataatat gtaaatacaa taaaaagtgt cattcctagg agggagcttt taaagccaat 360
gtgagttcac catgctttct ttcccctgcc aaactgatgc gatgattgtt gaagcatgta 420
tcaagatgaa accttcttca gactaggttt ctaaatgagc acattaagca gagccccgtg 480
aagttgaaca tgtagtatga gcaaaatata aaatttattt ttaagccact gaaattttag 540
gattgtttgt tatggcagca taacctaaac catcctgact catataaaaa ttggctccta 600
gatatgggtt agtgtaataa caacaataag cataacaaca ataaaactaa acaatcattt 660
agagggcgat aactaagaac actgctatca gaagctgaag ggatggtgat ctgtttggta 720
aacatatcac ctgtgataat gtagaaggca gatcatgaac ctgatgaact aaagaaaagg 780
attgtaaaac agagagtgag tagtgtgtgt tggtggctgt tggatgcatt ctgaaagtta 840
ctacaagcta ttcaaagatg aactcagaaa aatttggcct gtttgcaatc aggaatgaaa 900
tggaatagag aaaacctata aatccaggga tttacatcgt tagaagaggc aattgtttcc 960
cagtcccgac tggtaaaata taaaatcaag aaatactttt agcaagaaag gacctggaag 1020
aaactaagct ttgaagtaag gatcaagtca caaagtatga acctactatt aaaacctcag 1080
gataggaggc cgaggaggac ggatcacctg aggtcgggag ttcgagacaa gcctgaccaa 1140
tatggagaaa tcctgtcgct cctaaaaata caaaattagc cgggcgtggt ggcgcatgcc 1200
tgtaatccca gctactcagg aggctgaggc aggagaatcg cttgaacccg ggaggtagag 1260
gttgcagtga gccgagactg tgccattgca ctccaaccag ggcaacaaga gtgaaactcc 1320
gtctcagaaa aaaaaaaaaa aggatcaatt aagatcttac ccagtatata ctttcaactg 1380
gacaaaatgg ctctgggaaa aggaagaaag gtctaactct cccatcaaaa cccaataagc 1440
ccaaagtgct caaaatcaag tgaagagcaa gagagtgaga cggcaaaaga gagacagagg 1500
agtcaacagg caggagagca ggaaagcaaa gagatatggc aaaccaaaga agtatgtctg 1560
gaaaagaact ttgtggctat tgtcacatgg aacacctcat atcagataga taagaagcct 1620
acctacacat attcaagact taatgtcact ctgcatctcc ccaactttta aaagcttatt 1680
taaaagcata ttttccaatg cctgctttag ctgtggaaaa ggaagactct ccgagggcaa 1740
atccaggagt catggaaaac aatgggcagg gcagcttggt gctgtgactg gatgggtctt 1800
taaggttgtt tcccttgaat aaagaatgag ggaattccac caggggaagg agagtagacc 1860
aaacatttgg tgaacagaaa gggagacaga gtctttagtg ttattcccca aatattcctg 1920
gagagccctt tgagacacct ggtaggatta tagtgatcta aagaggcatg gccatgtgac 1980
tgctttggcc aatgatgtat gggcagaagt gatgtgtgcc ataagtggat gctctgacag 2040
ccatgatgtt ccgagttcct tccccacagt caggatagct gtgaaaaggg gtatcaacac 2100
gaggcctcct tcagcctgag ccctttagtg actacaatga gcagagctac cctgccaacc 2160
tacaacggtc atgtagcaga aatgaccaac tttcactgga ataagccact gatatgttag 2220
ggttgtttgt tagggcagca taacactgcc tttcctgaca gaactgggag tgagagataa 2280
ggctggagag agaaggagtt ggattgtgaa aagccccttc tccaaggcat aggaatcgga 2340
actctcctga ttgtaaggaa aagccaatga aggcttccaa gcaggggcgt taacatgaac 2400
agatttgtta tttttttgga tgaaatatgc tatttctcaa cataaggaga agatcagcct 2460
actaaaccac atgaggcaga gaggaacaca cagaacctta ggcaaaagat atatatttat 2520
gcaaagtcct aagtcttgaa atttctaaat acaagagctc tgaacggagt tacagtcaga 2580
ggatcaggca agaaaatttc ttctatgcca caagcgctat ttcctctgca gataactaat 2640

actccacaag aaagtttgcc agagtagaga aaatacaaag gaatgtaaca cagacctgag 2700
gtcaatttcc agctgcctgt gtgacctcaa caaagatact taacctctct gggcctattt 2760
cctcacatgt taaatctgga taataatata tgtctctcaa gacagttgtg gggagaaaac 2820
agcgtattta aattgcttag gagaatgcct ggcagataat aagtgtttaa tacatgaaac 2880
tgatttttat tactgttatt aaaaaatatt tagaacacca actccctgaa tacaacagaa 2940
aatgattcag ggcaacagac agaggagaat gttctctcct tgaggaagca actggatctt 3000
gtcatcactg tatacctacc taccccaccc cctccccagc tcagtgcctg gctcacagta 3060
ggctttcagt taccctctgc agatcagtga aagctaggtg agtgcccgga gtgaagaaaa 3120
gttggcaggt ttcccactga taccagctgc tgttggtttc tgaacactca aagccgcaaa 3180
taccttaggg ctgggggcaa tgaacccaag gctgaattcc aagttcagaa gcagcgaagt 3240
ctgaatttag aacctaggaa cttaaactgc tgcaggtcca acttcaagcc ccagttttag 3300
acagaggctt gggaaagatc tgacttctaa cccggttccc cctcccctcc tcccctcgat 3360
gcttctcaca ggaaagtaca cctggtcctg ccaaatcgca ctcttatatc ctggcatcct 3420
atccaggctc tgcgaggatg gaaactgcga ggcaggggag ggaagcgggc tgtttggcca 3480
ccacctccct agtgctgcag gcgaccctgt cacactaact cctggcagcc cagtgaggtg 3540
gacggcaccg gccccacctg cagatgaggg aaatgaagct cggaggagtt ccgtgatttg 3600
cttgcttcac actgtggtag cctggccacg aaagaaccag gattcccgac ttcgggattc 3660
tttccaccac acactttcgt ccctaagggg tgggggcggg ggggagaata aaataaccgc 3720
ggaaaaggaa ccacttacat gtgtctagcg cttcctagag gctacccagg atatgcgccc 3780
accacccggc cgggagtgca gagatttgaa gtccaggttc taccccgggc tccgagtact 3840
actgcgtgac tttatgcgag tgtccgccgc cttctgggct tgttttcccg gaagcaactc 3900
ggcgcggatg gagtgtgtgt gtgcgcgcgc gcgcgcgtta tgttgtgcgt gttgtgttaa 3960
gcgtgtgcgt gttgtccggg ggcgggaggg ggagtagact aacaccgggg ttccccgagt 4020
ttcggatcgc ctacacgctt gttcccatct ggaccctgtt acccaccaat tgcgcccact 4080
ataaaaactg cccctccgag gcaaagctgt gaacccccgc gccctttccc ccacggtccc 4140
ggaggatgaa gtggggtgca acggagactc agctgagcgt ccagtttcgg gcaatacaaa 4200
tctctcggct tctacgagca gccagacgac cccgcggacc gtcgctcctg aacttgaccg 4260
agatgcaaac ttcggagtgt tctcaacgtg gggggccgac tctcgggaga ccgcccctaa 4320
acttaagtcc ccttaggctc gcccccacct gggacttcac agagccacct taagggcggt 4380
attcccgccc ccccggaagt gcgggggggt ggcagcgtac ttggattctc agcctccagc 4440
cccgcgcggt ggcggccgcc ggtggatgac ttcgggcccc acaagtgggg aaacaacaac 4500
cacccctcgc ccgcacccct ggcccaaaac aactggccag gttccctggc ctcccgggtc 4560
cctgcatccc ccgcatcccc gtccgcagcc gtgaacttga gcccccctcc atcagaggtt 4620
gcgagcgtcc gcccgctcgc ggcagccacc gtcactagac agtcaaaccc caagacgtca 4680
gcccacaatg caccgggcgg gccgggaaaa acggcccggg gaggggaccg gggaagagag 4740
ggccgagagg cgtgcggcag gggggagggt aggagaaaga agggcccgac tgtaggaggg 4800
cagcggagca ttacctcatc ccgtgagcct ccgcgggccc agagaagaat cttctagggt 4860
ggagtctcca tggtgacggg cgggcccgcc cccctgagag cgacgcgagc caatgggaag 4920
gccttggggt gacatcatgg gctattttta ggggttgact ggtagcagat aagtgttgag 4980
ctcgggctgg ataagggctc agagttgcac tgagtgtggc tgaagcagcg aggcgggagt 5040
ggaggtgcgc ggagtcaggc agacagacag acacagccag ccagccaggt cggcagtata 5100
gtccgaactg caaatcttat tttcttttca ccttctctct aactgcccag agctagcgcc 5160
tgtggctccc gggctggtgt ttcgggagtg tccagagagc ctggtctcca gccgcccccg 5220
ggaggagagc cctgctgccc aggcgctgtt gacagcggcg gaaagcagcg gtacccacgc 5280
gcccgccggg ggaagtcggc gagcggctgc agcagcaaag aactttcccg gctgggagga 5340
ccggagacaa gtggcagagt cccggagcca acttttgcaa gcctttcctg cgtcttaggc 5400
ttctccacgg cggtaaagac cagaaggcgg cggagagcca cgcaagagaa gaaggacgtg 5460
cgctcagctt cgctcgcacc ggttgttgaa cttgggcgag cgcgagccgc ggctgccggg 5520
cgccccctcc ccctagcagc ggaggagggg acaagtcgtc ggagtccggg cggccaagac 5580
ccgccgccgg ccggccactg cagggtccgc actgatccgc tccgcgggga gagccgctgc 5640
tctgggaagt gagttcgcct gcggactccg aggaaccgct gcgcacgaag agcgctcagt 5700
gagtgaccgc gacttttcaa agccgggtag cgcgcgcgag tcgacaagta agagtgcggg 5760
aggcatctta attaaccctg cgctccctgg agcgagctgg tgaggagggc gcagcgggga 5820
cgacagccag cgggtgcgtg cgctcttaga gaaactttcc ctgtcaaagg ctccgggggg 5880
cgcgggtgtc ccccgcttgc cacagccctg ttgcggcccc gaaacttgtg cgcgcagccc 5940
aaactaacct cacgtgaagt gacggactgt tctatgactg caaagatgga aacgaccttc 6000
tatgacgatg ccctcaacgc ctcgttcctc ccgtccgaga gcggacctta tggctacagt 6060
aaccccaaga tcctgaaaca gagcatgacc ctgaacctgg ccgacccagt ggggagcctg 6120
aagccgcacc tccgcgccaa gaactcggac ctcctcacct cgcccgacgt ggggctgctc 6180
aagctggcgt cgcccgagct ggagcgcctg ataatccagt ccagcaacgg gcacatcacc 6240
accacgccga cccccaccca gttcctgtgc cccaagaacg tgacagatga gcaggagggc 6300
ttcgccgagg gcttcgtgcg cgccctggcc gaactgcaca gccagaacac gctgcccagc 6360
gtcacgtcgg cggcgcagcc ggtcaacggg gcaggcatgg tggctcccgc ggtagcctcg 6420

gtggcagggg gcagcggcag cggcggcttc agcgccagcc tgcacagcga gccgccggtc 6480
tacgcaaacc tcagcaactt caacccaggc gcgctgagca gcggcggcgg ggcgccctcc 6540
tacggcgcgg ccggcctggc ctttcccgcg caaccccagc agcagcagca gccgccgcac 6600
cacctgcccc agcagatgcc cgtgcagcac ccgcggctgc aggccctgaa ggaggagcct 6660
cagacagtgc ccgagatgcc cggcgagaca ccgcccctgt cccccatcga catggagtcc 6720
caggagcgga tcaaggcgga gaggaagcgc atgaggaacc gcatcgctgc ctccaagtgc 6780
cgaaaaagga agctggagag aatcgcccgg ctggaggaaa aagtgaaaac cttgaaagct 6840
cagaactcgg agctggcgtc cacggccaac atgctcaggg aacaggtggc acagcttaaa 6900
cagaaagtca tgaaccacgt taacagtggg tgccaactca tgctaacgca gcagttgcaa 6960
acattttgaa gagagaccgt cgggggctga ggggcaacga agaaaaaaaa taacacagag 7020
agacagactt gagaacttga caagttgcga cggagagaaa aaagaagtgt ccgagaacta 7080
aagccaaggg tatccaagtt ggactgggtt gcgtcctgac ggcgccccca gtgtgcacga 7140
gtgggaagga cttggcgcgc cctcccttgg cgtggagcca gggagcggcc gcctgcgggc 7200
tgccccgctt tgcggacggg ctgtccccgc gcgaacggaa cgttggactt ttcgttaaca 7260
ttgaccaaga actgcatgga cctaacattc gatctcattc agtattaaag gggggagggg 7320
gagggggtta caaactgcaa tagagactgt agattgcttc tgtagtactc cttaagaaca 7380
caaagcgggg ggagggttgg ggaggggcgg caggagggag gtttgtgaga gcgaggctga 7440
gcctacagat gaactctttc tggcctgcct tcgttaactg tgtatgtaca tatatatatt 7500
ttttaatttg atgaaagctg attactgtca ataaacagct tcatgccttt gtaagttatt 7560
tcttgtttgt ttgtttgggt atcctgccca gtgttgtttg taaataagag atttggagca 7620
ctctgagttt accatttgta ataaagtata taattttttt atgttttgtt tctgaaaatt 7680
ccagaaagga tatttaagaa aatacaataa actattggaa agtactcccc taacctcttt 7740
tctgcatcat ctgtagatac tagctatcta ggtggagttg aaagagttaa gaatgtcgat 7800
taaaatcact ctcagtgctt cttactatta agcagtaaaa actgttctct attagacttt 7860
agaaataaat gtacctgatg tacctgatgc tatggtcagg ttatactcct cctcccccag 7920
ctatctatat ggaattgctt accaaaggat agtgcgatgt ttcaggaggc tggaggaagg 7980
ggggttgcag tggagaggga cagcccactg agaagtcaaa catttcaaag tttggattgt 8040
atcaagtggc atgtgctgtg accatttata atgttagtag aaattttaca ataggtgctt 8100
attctcaaag caggaattgg tggcagattt tacaaaagat gtatccttcc aatttggaat 8160
cttctctttg acaattccta gataaaaaga tggcctttgc ttatgaatat ttataacagc 8220
attcttgtca caataaatgt attcaaatac caataacaga tcttgaattg cttcccttta 8280
ctactttttt cttcccaagt tatatactga agtttttatt tttagttgct gaggttaaca 8340
ttgctgcaat ctgaagtgat ttgaaaatta agaagcaaag aatttaggaa gagtcaaact 8400
agaagttgtg atctgaatga aacagtctcc caatttcatt aattctttcc ctctttctcc 8460
atctctatct ctctctctac tacctcaccc ccaaataaac ccctatgcct atggaaacta 8520
acagtttagc attttgggtc tgtctgtagc ttatttctta atttgtttct aaaataatca 8580
accttgttta aatatagaat aagtctcaaa agatagtaat ctaatactat ggaaaagatt 8640
cacaagagaa aacatgaact agtactaact caatgattac atgcgtaaat caaacctcag 8700
ctctggggaa atgggaaact tgaattctac ctacagatat aacttaggag atcaacaagg 8760
cagtgcttaa caagggaaca attttttgct tggttgcttt tcataccaag agccagtgtg 8820
attatcaggc agcaaaagca atttgatatc caggaatatt tcacaaacca ctaattgagt 8880
cagggctgtt tgctcattag cagtgttcta gtacccagta ggtctgggag tgctaatatc 8940
ctgcctgtgg ctgtaaatcc aaatacacaa agagtttctc cttaggagta tatctaggag 9000
tggaagagat tggacaggct tgttagcttc ggatcctttg taataacact tcttgaacaa 9060
agggttactg ggagcagagc ccagtttgcc aaactgaatt tgccgatgtc agcaacccgg 9120
aaggtgagat gattttgacc atatgtagaa atagcagagg gacaataaag cattttgggg 9180
tggggctctg tggaagctaa acaagagcca actagaaaaa ataagcaggc tggggaatta 9240
attacctgca aagg 9254

<210> 29
<211> 8244
<212> DNA
<213> Homo Sapiens

<400> 29

ttctaaatat attcaccatt tcgacgggtc acaataattt tcttggacgt taatttccgg 60
ggacgtcaaa acacatcagt cccggcgggc ttttccagac ttacactatg tggcctgggg 120
ccccagatgt gctttctccc aggctctgga cagttttata caccccctcc aggtcccaca 180
gatttacagg ccactaaccc gggttcccta attttaaaga cgaagccctt ggtccgtggt 240
ggtcgcgctg accaatttgc ctggctcccc aggatgtgga cagtgccttt tccacattta 300
ggcattgttt ccaaaacaag tggaactttc ccgcataatt ttgaatatta actccagggt 360
ctcctaagct tactgtttcc cgcacacgtc cgccccattc cgcgcccccc caccccaccc 420

ccgcgcccct tcccgttcac ctcagcatgg gacatttgct gttgggtccc gcaaatctat 480
tcacactaac ctgggttccc taaactttac acgttgaatc ccaagtccct catgacactc 540
agcagggctg gaaggttgga accctcagag tatgaaaatt gcttcccata ccttccccca 600
aattcggtca ttacacccag agcatgttga atcctttctt gaatcataaa cgacctgccc 660
tctgattgtc tgagtttcat aaaatggagg gatttcccca gtgattcccc aaagctattg 720
agaatgttgt gggtgtgtga gtcacaaagc tttggggcat taacgtctat ggctctatat 780
gctgcctggc cacgaatcag gtcccttaag atgtagacag tgccacccag gtccctggag 840
cacactgagc agttaccagg aggtgctcaa gtgtgaccca ggattctcca ggtcccccca 900
aatcacacag ggtctcccgg gtccctctgg gctacaccaa gcacaaaagg accccttggg 960
cagagcctac ttttatttct gttatgccag gtgttgctaa cggccccagt tcccaaacat 1020
tccgagcact ttctctgcat cacaacatat tgactattaa acaattctct gggtcccagg 1080
agcttcataa caagaatctt gcttttctaa aattcaggca ttggtctgaa accccaacgg 1140
ccaggatcac actggaccct tttcctggtc ccccatactt ggatgttctg gacgctgccc 1200
tccaggccct ctaggaacat tcagcattgc cctcggatca caggacagca ctgatttctt 1260
gggctccaaa caacccactg agtcatctca aagttaagca atatttcctt caaacacact 1320
gcacactccc tatcacaaaa tctgaaaatt cctaagtcct aagacctagg aattctgaat 1380
cccctttctt taaaatgtac atatggaccc ccaagtcctc caaggactct gagcaacttc 1440
cctagatctt tagattcaaa aacgattttc ctggagcccc caaattgcgg tattgtctcc 1500
cagccttcca aagcaaattg agattttttt cccttcacaa aacaattgag gttttttttt 1560
tttttaatac tgatttatga gtctcctgac tttatggtcc ctgccctggg tccccctaca 1620
tttagaaaat gttccatgga cccccaaagc acactaaaaa atgtccctgg gtcccaagaa 1680
atcccaggca tggaaaaacc tgcgacctat aagtttccta gctactaact aggtttccag 1740
aaatttagat atcaaatctc cattgggtaa tttccatgtg tcccaaaaac ttgaaatgtg 1800
tttcactggg gctcccccaa atgcagacga catccaggaa aatatatagt ctttttctta 1860
tttaccaaaa ataagctaat ggaaatcatt taaaaattag catagaaaaa taatactgat 1920
tttatatttt tttatttttt attttgcttt ccccaaatgt actgatcaca ctccaggctc 1980
ccccaaaatc tagacagtgc tttcttccat ctctgaaggg tgttaaaacc tttccctgaa 2040
gccacagtaa ttatgaaggt tattttttcc ccggctgctg ccagcgtcca ggccactaac 2100
ttatattctt aagatgtgaa aattaatctc agcttccccc taacacacca agaatgtgtt 2160
tggatcccca aaatgtgttc cttgctttca tctgccaatt ttacgtaata tggctctacg 2220
gcaaaattcc caatttcata tggagaattt tctttaacta cccctcctca caaattggtc 2280
ccccaagcta gctggcccct atttgagacc tctttctcta tgttcccaat tgcatggagc 2340
aacttctctc atcccccaaa cctgtaatct atttttctgg agtctcgagt ttagtcatta 2400
atcacggttc ccacattaac ggagtccccg gggtcccctc ctccaggaca cccattcgct 2460
aagcccgcaa ggcagaaaga actctgcctt gcgttcccca aaatttgggc attgttcccg 2520
gctcgccggc cacccactgc agcttcccca accccgcgca cagcgggcac tggtttcggg 2580
cctctctgtc tcctacgaag tccccagagc aactcggatt tgggaaattt ctctctagcg 2640
ttgcccaaac acacttgggt cggccgcgcg ccctcaggac gtggacaggg agggcttccc 2700
cgtgtccagg aaagcgaccg ggcattgccc ccagtctccc ccaaatttgg gcattgtccc 2760
cgggtcttcc aacggactgg gcgttgctcc cggacactga ggactggccc cggggtctcg 2820
ctcaccttca gcagcgtcca ccgcctgcca cagagcgttc gatcgctcgc tgcctgagct 2880
cctggtgcgc ccgcggacgc agcctccagc ttcgcggtga gctccccgcc gcgccgatcc 2940
cctccgcctc tgcgcccctg accggctctc ggcccgcatc tgctgctgtc ccgccggtgc 3000
tggcgctcgt ctccggctgc cgccggggag gccggcgtgg ggcgcgggac acggctgcgg 3060
acttgcggct ggcggctgcg ctcgctcctg ctgggcgccc cgaaatccgc gccactttcg 3120
tttgctcatt gcaaagatct cattcgtggg gaaagcggct ggagggtccc aaagtggggc 3180
gggcaggggg ctggggcgag ggacgcggag gagaggcgct cccgccgggc ggtaaagtgc 3240
ctctagcccg cgggcctagg actccgccgg gaggcgcgcg cggagcgcgg gcgaagtgat 3300
tgatggcgga gcgagggggg cgaggggggc caggggggcg cgagattccg ccggcggccc 3360
cttccccttg gctaggctta ggcggcgggg ggctggcggg gtgcgggatt ttgtgcgtgg 3420
tttttgactt ggtaaaaatc acagtgcttt cttacatcgt tcaaactctc caggagatgg 3480
tttccccaga cccccaaatt atcgtggtgg cccccgagac cgaactcgcg tctatgcaag 3540
tccaacgcac tgaggacggg gtaaccatta tccagatatt ttgggtgggc cgcaaaggcg 3600
agctacttag acgcaccccg gtgagctcgg ccatgcaggt aggatttgag ctgtgtttcc 3660
cgccctgatc ctctctcctc tggcggccgg agcctccgta ggctccaagc ctggcccaga 3720
ttcggcccgg cgcagccggc cttccgcgcg tcccgcacct ggcgggggct ccggggctcc 3780
ggcgcggcac cggggggcgc tcgggatctg gctgaggctc caagcgccgc gtggccggct 3840
ccttctgctg gggcaggtgg cggctgcgcg ccccgcccga gcccaggggc cccctcagcc 3900
gcaacaacca gcaaggaccc cccgactcag ccccaagcca cctgcatctg cactcagacg 3960
gggcgcaccc gcagtgcagc ctcctggtgg ggcgctggga gcccgcctgc ccctgcctgc 4020
ccggagaccc cagctcacga gcacaggccg cccgggcacc ccagaaaccc gggatggggc 4080
ccctgaattc tctagaacgg gcattcagca tggccttggc gctctgcggc tccctgcccc 4140
ccacccagcc tcgcccccgc gcacccccca gcccctgcga ccgccgcccc cccccccggg 4200

gccccagggc ccccagcccg caccccccgc cccgctcttg gctcgggttg cgggggcggg 4260
ccgggggcgg ggcgagggct ccgcgggcgc ccattggcgc gggcgcgagg ccagcgaggc 4320
ccgcgcgggc cctgggccgc gggctggcgc gactataaga gccgggcgtg ggcgcccgca 4380
gttcgcctgc tctccggcgg agctgcgtga ggcccggccg gccccggccc cccccttccg 4440
gccgcccccg cctcctggcc cacgcctgcc cgcgctctgc ccaccagcgc ctccatcggg 4500
caaggcggcc ccgcgtcgac gccgcccgct gcctcgctgc tgactcccgt cccgggcgcc 4560
gtccgcgggg tcgcgctccg ccgggcctgc ggattccccg ccgcctcctc ttcatctacc 4620
tcaactcccc ccatccccgc ttcgcccgag gaggcggttc cccccgcagg cagtccggct 4680
cgcaggccgc cggcgttgtc accccccccg cgctccccct ccagccctcc ccccggcgcg 4740
cagcctcggg ccgctcccct ttccgcgctg cgtcccggag cggccccggt gccgccaccg 4800
cctgtccccc tcccgaggcc cgggctcgcg acggcagagg gctccgtcgg cccaaaccga 4860
gctgggcgcc cgcggtccgg gtgcagcctc cactccgccc cccagtcacc gcctcccccg 4920
gcccctcgac gtggcgccct tccctccgct tctctgtgct ccccgcgccc ctcttggcgt 4980
ctggccccgg cccccgctct ttctcccgca accttccctt cgctccctcc cgtccccccc 5040
agctcctagc ctccgactcc ctccccccct cacgcccgcc ctctcgcctt cgccgaacca 5100
aagtggatta attacacgct ttctgtttct ctccgtgctg ttctctcccg ctgtgcgcct 5160
gcccgcctct cgctgtcctc tctccccctc gccctctctt cggccccccc ctttcacgtt 5220
cactctgtct ctcccactat ctctgccccc ctctatcctt gatacaacag ctgacctcat 5280
ttcccgatac cttttccccc ccgaaaagta caacatctgg cccgccccag cccgaagaca 5340
gcccgtcctc cctggacaat cagacgaatt ctcccccccc ccccaaaaaa aagccatccc 5400
cccgctctgc cccgtcgcac attcggcccc cgcgactcgg ccagagcggc gctggcagag 5460
gagtgtccgg caggagggcc aacgcccgct gttcggtttg cgacacgcag cagggaggtg 5520
ggcggcagcg tcgccggctt ccaggtaagc ggcgtgtgcg ggccgggccg gggccggggc 5580
tggggcggcg cgggcttgcg ccggacgccc ggcccttcct ccgcccgctc ccggcccggg 5640
gcctgcgggg ctcggcgggg cggctgagcc cgggggggag gaggaggagg aggaggagga 5700
cggacggctg cgggtcccgt tccctgcgcg gagccccgcg ctcaccctgg cggcggagct 5760
gggggtgggg tgggggcgtc gggaagggcc gagggaggtg tgaggtgtct gcaggggcga 5820
cttcccggtc ggtctgtggg tgcagggggt gccgcctcac atgtgtgatt cgtgccttgc 5880
gggccctggc ctccggggtg ctgggtaacg aggagggcgc cggagccgca gaagcccacc 5940
ctggtatgtt gacgcggtgc cagcgagacc gcgagaggaa gacgggggtg ggcggggcca 6000
ggatggagag gggccgagtt ggcaggagtc atggcagacg ccacattcgc gacatctccc 6060
ccacaccccc tctggctctg tccgcaacat ttccaaacag gagtcccggg agagggggag 6120
aggggctgct ggtctgaggc taagaagggc agagccttcg acccggagag aggccgcggc 6180
ccctgcccag tgggcagcgt ggaagtttcc atacaaggag gtgggaagga gacccccccc 6240
cccttcactg ccctgtgcag agatgagccg ggggtgcagg atgggagccc atggcacttc 6300
gctacgggat ggtccagggc tcccggttgg gggtgcagga gagaagagac tggctgggag 6360
gagggagagg gcgggagcaa aggcgcgggg gagtggtcag cagggagagg ggtggggggt 6420
agggtggagc ccgggctggg aggagtcggc tcacacataa aagctgaggc actgaccagc 6480
ctgcaaactg gacattagct tctcctgtga aagagacttc cagcttcctc ctcctcctct 6540
tcctcctcct cctcctgccc cagcgagcct tctgctgagc tgtaggtaac cagggctgtg 6600
gagtgaggac ccccgctgcc atcccactcc agcctgaggc agggcagcag ggggcacggc 6660
ccacgcctgg gcctcgggcc ctgcagccgc cagcccgctg cctctcggac agcacccccc 6720
tcccctcttt tcctctgccc ctgcccccac ctggtctctg ctccctcacc tgctccttcc 6780
ctttctgttc cttcccttcg gccccctcct tgcccagctc agggcttttc ctgggccctc 6840
acctgctccg caccgctgca tgcttcctgt cctgctttct gccggtcccc tgacccggac 6900
ctccaagttc agagtggtgg ggcttgttgc ggaagcgcgg cgagggctag agtggccagc 6960
tggcggagtg tgctcttaga atttggaagg gggtggcaga gggggcggtg agaggactgg 7020
ccagggtcca gtcaaggaga tgaccaagga ggctttcaga tcctcggcgc agctgcccac 7080
tagtctttag agagggcatg caaagttgtg cttctgtccc actgcctgct cagtcgctca 7140
cataatttat tgcatcaaaa actcccctgg gtctgcggag cgaaggctgg ggctgcccgc 7200
ctggagggtt ccaccttctg caggggcagg gccaacttgc tgtggtggct cccggcctcc 7260
cacccccgag tgggttaacc cggccctgtg gccctgcagc ctgtggaggg ggtgtgtcct 7320
aagactggcc tccccttcca gattgtagtc tggggaacct ggtgtcggac ttcccaggtg 7380
gcctgagctg gtctctccag ctcccacggg gagagtttgg tagcgcaaat agggagatgt 7440
tctggaggcc cctggcctta ctggttcgat ttgaggcctg gaaaggaggc tctgggcgtg 7500
tgtgtgtgtg tttgggggta cccaaggcag actggagttg gagaactggg tgactgggaa 7560
aacaaggttt ctagagcatg ggtggcgtgg ttgtgttaac cattggagtc cttgacccag 7620
gcctggctca gctgcagact ggaaaggtgg aaaagccagg gggagggcgg gggctggccc 7680
agcaggactg gcctgctgct ttgaggcgga tggtcctcct ggaccccccc tgctcagctg 7740
ggggttgtgg ggaggaaggg actggtcctc ctggatgcac atgctctgta ggggtggggc 7800
tgtctgccat cttggctggc gctggaggcc tgagaagtgg cgatgtgacg ctgggctggc 7860
cctgccccca tggtgtcata ggacggaggc caggtcgggt gtccagcctg ggcccctgca 7920
gctgtggatg ccgctgagct cctgcaataa tgaccgtgga gatggtcacc cctcgtgtaa 7980

aattactagt gcttcttgca aatggaagga actgggcctt ttctgtgtgc ttctggacgc 8040
ttcattctgc acatggccct gcgccctcac ctcggcatta tgacctgtgt gttacttttg 8100
taataaaaat aatgtttata ggaaagccgt gctttcaatt ttcaactgaa tttgtaggtt 8160
ggcaaatttg gtttgggagg ggcacctctg gcctggggct tggcctggct gccccgctca 8220
cgccacttct ctcccgcccc caga 8244

<210> 30
<211> 16842
<212> DNA
<213> Homo Sapiens

<400> 30

cctcccaaag tgctgggatt acaggcgtga gccactgcac ctggcctgtt atttatgtat 60
ttattttttt ttgagacaag ttcttgctat gttgcccagg ctggcctcaa actcctaggc 120
tcaagagatc ctcccaaagt tctgggatta caggtgtgag ccactgtgca taaccctgaa 180
gtggttaatt ttaataatac atttacttta gcctaacata tccaaaatta catcatttca 240
acatgcaatc attattaaaa ttattaatga gatattttat tttattttta tttatttatt 300
tttttgagac ggagtctcgc tctgtctccc aggctggagt gcggtggcgc gatcttggtt 360
cactgcaagc tccgcctcct gggttcacgc ctttctcctg tctcagcctc ccgagtagct 420
gggattacag gtgcccgcca ccacgcccag ctaatttttg tatttttagt agagaccagg 480
tttcaccacg ttggccagga tggtctcgaa ctcctgacct cgtgatcagc ccaccttggc 540
ctcctaaagt gctgggatta caggcgtgag ccactgtgtc tggccagtga gatattttat 600
ttgtgccctt tttttttttt tttgagaccg agtctcactg tgttgcccag gctgcagtgc 660
agtggcacaa tcttggctca ctgcaacctc tgcctcccag gttcaagcga ttctcttacc 720
tcagcctcct gagtagctgg gattacaggc gggcgccacg acacctggct agtttttgta 780
tttttagtag agacgggggt tttaccatgt tgcccaggct ggtcttgaac tcttgacctc 840
aggtgatcac ctgccttggc cccccaaagt gctgggatta caggcaaaag ccgccatacc 900
aggcccattt ttttttcttt cgagacggag tctcattgtt gcccaaggtg gagtgcaatg 960
gcaagatctc agctccctgc aacctctgcc tcctgggttc aagcgatcct cctgcctcag 1020
cctcccaagt agctgagatt acgggcgccc gccaccacgc ctggctaatt tttgtatttt 1080
tagtagagac agggttttac cacgttggcc agactggtct caaactcctg acctcgggtg 1140
atccatccgc ctcggcctcc caaagtgctg ggattacagg tgtgaactac cgtgcctggc 1200
aggcccattt ttaatttttc taactttgga aattcagtgt gtagggcagg catggtgcct 1260
catgcctgta atcccagaac tttgggaggc cgaggcaggt ggatcacctg aggttaggag 1320
ttcgggacca gcctgaacaa tatggtgaaa ccctgtctct attgaaaatc caaaaattat 1380
ccagccatgg tggtgtatgc ctgtagtccc agctactcca gaggctgaga caggagaatt 1440
gcttgaatct gggaagcaga ggttgcagtg aaccaagatc atgccattgc actccagccc 1500
gggcaacaga gcgagactcc gtctcaaaaa aaaaaaaaga gagtcagtgt ggagggaagt 1560
gtctctgttg ggtccgggtg ctctgtggga ctctgaggaa aagctcgcac caggtagatg 1620
ctgttctgtc cccatgggta aaggacaccc caacaaaccg aggagcaaaa tgtcctcgta 1680
tgccctcttc gcgcagacct gccaggaaga gcacaagaag aaacacccgg actcttctgt 1740
caatttcgtg gaattctcca agaagtgttt ggagagatgg aagaccacgt ctgcaaagga 1800
gaagtgaagt ttgaagagaa ggcaaaaagt gacaaagctc gctgtgacag ggagattaaa 1860
aattacattc ctccgaaatg taagaaaggg taagaaagga aagaaaaagg atcgcaatgc 1920
tcctagaagg ccaccatctg ccttcttcct gttttgctct gaacatcgcc caaagatcaa 1980
aagtggacac ccaggcctat ttgtcgtgga aactgcaaag aaactgggtg aaatgtggtc 2040
tgggcagtca gccaaagata aacaaccata tgagcagaaa gcagttaagc tacaggagag 2100
atatgaaaag ggtattgctg catatcgtgc taagggcaaa agtgaagcag gaaagaaggg 2160
ctcaaagaag aacaaaccag aagatgagga ggaggaggag gagaaagaag atgaagatga 2220
ggaggaagag ggtgaagatg aagaataaat ggctatcctt taatgatgcc tgtgcagtgg 2280
gcttgttttg ctaagaatgt gaattctagt acagctcagt attagcttca gtataaaact 2340
gtacaaattt tcgtatagct cataagattc tctgtacaga aatacttttt cttttctttt 2400
cttttttttg agacagagtt tcgctcttgt tgcctaggct ggagtgcaat ggcgtgatct 2460
cggctcaccg caacctccgc ctcccgggtc ctggttcaag cagttctcct gcctcagcct 2520
cctgagtagc tgggattaca ggcacatgcc accacgctca gctaattttt gtatttttag 2580
tagagatggg gtttcaccat gttggccagg ctgctttcaa actcctgacc tcgtgatccg 2640
cctgcttcgg cctcccaaaa tactgggatt acaggtgtga gccaccgcac cctgcctaat 2700
gtccctaaat atttaatggt ttttaaaaaa tttattgtgt atggcagcac agcacacttg 2760
taggaattag tatcaacagt acatcttgcg ttttttaaga tgctgcattt tttaacattt 2820
tgtaataaaa ttatgcgtat caaaaaaaca aagaaattcc gtgtgtagtt cacactcaca 2880
gcacatctcc gtccaggcac ttgagagaat gactaggagg ggttcttgga ggaggtggtc 2940
tttgaacgga gaatccatct tcaaggattc tgtctgtaat ggtcaccaag tatttcctga 3000

gtcacttcca tgtgtcctgc agttctctga aggggcgtgg gacctaccga tgccaattat 3060
ccagcattat ctccagattc caagaagttg gggtgtgagc cagcaatcag tacagaaaag 3120
agataccaaa ataagtttga gttggggagt gttccttcaa cttcagtttt ctggaagaga 3180
tctttttttt ttttttgaga cagagtttcg ctcttattgc ccaagctgga gtgcagtggc 3240
acgatctcgg ctcaccgcaa cctcctcctc ccgggttcaa gcgattctcc tccctcagcc 3300
ttctgagtag ctgggattac agacatgcac ctgtaatttc tactaaaaat acaaaaatta 3360
gccgggcgtg gtggcgcacg cctgtaatct cagctactgg ggaggctgag gcaggagaat 3420
cgcttgaaac caggaggcgg agattgtacc aagatagttt gttccagcta aacaacctgg 3480
cgctagtgca ggaaaaggtg gaaggcacgg ggctagcaca ggagggttca atattttcaa 3540
ccttatcaag ccatattttg gcaactcttg tttttcacga gaagcccccg ctgggcttgt 3600
cccagcgctg tcctgaggct tcccccatga gttccgatag ggcagaggcc gccctgagcg 3660
tttctctttc ccctggtcca agagtggctc aaaagaagga tttttgactg gaattggcca 3720
ctttgtgtta ctttttgacc cttgacctcg ccccaaaggg ggatgcgggg gaggggctct 3780
ggtaggggtg gccccgctcc ttccaggtcc gcaagcccag gttcccgccc accgggctca 3840
gcccaccctg cggccgttca gggaggccgt tggcacccgt gacctacgac ccccttcccg 3900
agccccaccg aggtcacagc cgtggcctcg tctccccatg cctgcttccc gccccctgcc 3960
cgtgacgggc gtctccgagg accaatgagc gcgctgtatc cacccctcgg gcggggccaa 4020
gcgccgacca atcgccgctc gggcgcccgg ccgggtccaa acgctccaat cgtcagcggc 4080
ggcggggcgg gcagagggcc ggggatggca ggttcaacca acgggtgggc acgtcgtcct 4140
cgcgaggagg cgtgccctgc ggccgggcgt gcggtgtccg cggcggcgca gggaggggga 4200
gggaggtaaa caagatggcg gcggcgtgtc gggcgcggaa gggggaggcg gcccggggcg 4260
cccgcgagtg aggcgcgggg cggcgaaggg agcgcgggtg gcggcacttg ctgccgcggc 4320
cttggatggg ctgggccccc ctcgccgctc cgcctcctcc acacgcgcgg cggccgcggc 4380
gagggggacg cgccgcccgg ggcccggcac cttcgggaac cccccggccc ggagcctgcg 4440
gcctgcgccg cctcggccgc cgggagcccc gtggagcccc cgccgccgcg ccgccccgcg 4500
gaccggacgc tgagggcact cggggcgggg cgcgcgctcg ggcagacgtt tgcggggagg 4560
ggggcgcctg ccgggccccg gcgaccacct tgggggtcgc gggccggctc ggggggcgcc 4620
cagtgcgggc cctcgcgggc gccgggcagc gaccagccct gagcggagct gttggccgcg 4680
gcgggaggcc tcccggacgc ccccagcccc ccgaacgctc gcccgggccg gcgggagtcg 4740
gcgccccccg ggaggtccgc tcggtcgtcc gcggcggagc gtttgctcct gggacaggcg 4800
gtgggaccgg ggcgtcgccg gagacgcccc cagcgaagtt gggctctcca ggtgtggggg 4860
tcccgggggg tagcgacgtc gcggacccgg cctgtgggat gggcggcccg gagaagactg 4920
cgctcggccg tgttcatact tgtccgtggg cctgaggtcc ccggaggatg acctagcact 4980
gaaaagcccc ggccggcctc cccagggtcc ccgaggacga agttgaccct gaccgggccg 5040
tctcccagtt ctgaggcccg ggtcccactg gaactcgcgt ctgagccgcc gtcccggacc 5100
cccggtgccc gccggtccgc agaccctgca ccgggcttgg actcgcagcc gggactgacg 5160
tgtagaacaa tcgtttctgt tggaagaagg gtttttccct tccttttggg gtttttgttg 5220
cctttttttt ttcttttttc tttgtaaaat tttggagaag ggaagtcgga acacaaggaa 5280
ggaccgctca cccgcggact cagggctggc ggcgggactc caggaccctg ggtccagcat 5340
ggaggtggtg gacccgcagc agctgggcat gttcacggag ggcgagctga tgtcggtggg 5400
tatggacacg ttcatccacc gcatcgactc caccgaggtc atctaccagc cgcgccgcaa 5460
gcgggccaag ctcatcggca agtacctgat gggggacctg ctgggggaag gctcttacgg 5520
caaggtgaag gaggtgctgg actcggagac gctgtgcagg agggccgtca agatcctcaa 5580
gaagaagaag ttgcgaagga tccccaacgg ggaggccaac gtgaagaagt aagtatggct 5640
tgctggggtc ggggccgggc cgggccagtc acggtgctga tggttctgtc ttccttcctt 5700
ctctcctccc tccctccctt acttcctctt aacaccctga gctggacccg tctggcgcct 5760
gtgtcctccg tgccagggag agcgtggttg ggggcctgcg ttacggactt tcactcaggc 5820
aaggccagtt gtcgcagcgg ggcgtgcgtt tgcatgggct cttggactcc agttaaaatg 5880
ccctggtagc gaaaccctcc tgagaaggga gcggccccca atcccctaag actagcccct 5940
tggctccccc agctgtccaa ggagcagagg cgcccagtgg aatcagcctg tgtttgtttg 6000
ggccccgaga gtttgtgtgc ggccgccaac acgttttctg cgcagtgtgt ggccgttacc 6060
ggggccaggc gaaatgtgat ttgtttatcc tgtcagaggg gaaccctggg ctgccaaaaa 6120
taactgtttg caccggctta tcagtcagca ggagggaaac gtagcctttc ctcatttgcc 6180
agggatgtga cgctggaagc atccctggcc cccggggctg gaagccctgc ccgaggggga 6240
ctgtgcctcc ctcccgaatt gcatccggaa gaccttactt ttccaactga cttcttcagg 6300
cacggggctg ccgctgggca tcccggacgc ctctgcatct gtgcgcggag aagctcctac 6360
ctagggcagc actggccggc ctgagcctct cccagctggt gggggtggcc gggggtgtcc 6420
ctgccttatc gcagccagac acgctgcacc tgccgccgcc tggcgggccc tgcccaggcc 6480
ctgctccttt cccagccttc ttaactttca aaactttgcc tcctgtttcc aagaaaggac 6540
cttatctgct gggctaggcc tcgagggact ggcaaggaca ccctgcgcag ggcaccatga 6600
ccttggaagg aggtccgggg aggggctgct gcggtgtctg ctctcacctt cctcctgctc 6660
aggggccctg gggctcctgc cgtgtctgct ctcacctctc tcctgctcag gggccctggg 6720
gctcacgtac attttttttt tttttttttt gagacggagt ctcgctctgt tccccaggct 6780

ggagtgcagt ggcgcgaact gggctcactg caagctccgc ctcccgggtt catgccattc 6840
tcctgcctca gcctcccgag tagctgggac tacaggcacc cgccaccacg cccggccaat 6900
tttttgtatt tttcagtaga gacggggttt caccgtgtta gccaggatgg tctcaatctc 6960
ctgacctcgt gatccgcccg cctcggcctc ccaaagtgct ggtactacag gcgtgagtca 7020
acgcgtgcgg cctttttttt tttttttttt tttttttttg agacggagtt tcactcttgt 7080
tgctcaggct ggagtgcagt ggagtggtct tggctcactg caatctccgc cccctgggtt 7140
caagtgattc tcgtgcctca gcctcccgag tagctgggat tacaggtgcc tgccacagtg 7200
cccagctaat tttttttttt tttttttagt agagacgggg tttcaccatc ttggccaggt 7260
tggtcttgaa cttctgacct cgtgatccac ctgcctcagc ctcccaaagt gctgggatta 7320
aaggcgtgag ccaccgcgcc cggccagcac gcttcttaat cagcatgcag gctgtgacat 7380
gcggaaacca ggggcacagg ctctgaagtt tgtactctgg agctggagca gtcagggtta 7440
aatgggagcg gctgggaagg gtgtgtttcc acgaggcctt tctgatgtct cagtgcccag 7500
tctggacccg ggtagaaggt gtggccagtg ccttgagtca gtggagcagt tgcctgtgcc 7560
tggtctctgt gagatgggtt ggtcaggggc taggcagagc ctgtgcctgg tctctgtgag 7620
atgggttggt caggggctag gcagagcctg tgctgggggt agcaggtgcc ctctaaaggg 7680
gccagtgaca gtctctctcc cagcagtgag aaacccacgc ttaggggccg ggcgcggtgg 7740
ctcacgcctg taatcccagc actttgggag gctgaggtgg gtggatcgcc tgaggtcagg 7800
agttcgagac cagcctggcc aacatggtga aaccccatct ctactaaaaa tacaaaagtt 7860
agccgagtgt ggtggcgtgt gcctgtaatc ccagctactc gggaggctga ggcagaacat 7920
tgctggagcc tgggaggcgg agcttgcggt gacccgagat cgcaccattg cgctccagcc 7980
tgggcgacac agcgagactc tgtctcaaaa ataaataaat aaataaataa ataaataaat 8040
aaataaataa atctggcttg agttttgaca aatgtatatg caggtcaatg ggaagacag 8100
acgctggagg gtggaagcct gactgtgaga gtgagccccc tgctttcctg aacagtcagt 8160
ggaggaggtc ctgggagctc aggtgagcac ctgaacggta gggcagagcc tgagggtctt 8220
ggtggagaca ggcaagaagg ttgcaggaac ctagtctggc ccagtgcctg ggagaacggg 8280
agggaacacg gaggcaggga ggcagagggg ttggcaggac ctggactgga ctgtgcgtcg 8340
cagctaccca cagcttgacc ccactgcctt gaagtgagtg atgcaccctc actagtgttc 8400
agcgtccaat ttcatcctgg ccctgagtgt gtaacggctt tgcccttgcg gggccccctg 8460
ggatgagggg tgagccctgt cccttcctgg tcactgtctg ccatcaggag ctgccccgct 8520
tacagctgtg attcaggagc cactgcattt cctccagccc cctgggtgct gccagggggt 8580
cctcctattc tgggagctgg tgtgttctga ctggaggctg ccatgtgcag agtgctgtca 8640
aggagaaggg gtgtccactg cccagccctg aggcccaagg ccttggccag ccttgctgcc 8700
agcgctgcag ggaaaaagcc tcctttgtgt gtgggaagtt taataaactc cgctcagatt 8760
gtgtctcgca gcgagtgtct ggaaccttcc agacaagcct caggcgtccg gtcctccagt 8820
tggtgtggaa agcgtgggcg atcaccaagg ggggtgggtt ggggcagatg gagccggcgt 8880
gagtcccgtc tcttcccttc cttcccagaa aggcagccct ggagtccatg ccttgtcccg 8940
ctctcaccgg caaaaagtat aatcttatta gaaataggaa agttccaaaa agcatcaatg 9000
agttaaaaag agggctgggc atgttcggtc atgcctgtaa tcccagcact ttgggaggcc 9060
aaggcgggtg gatcacccga ggttgggagt tagcctgacc aacatggtga aaccctgtct 9120
ctactaaaaa tacaaaatta gctgagtgtg gtggcgcacg cctgtaatcc cagctactct 9180
ggaggctgag gcaggagaat cacttgaacc cgggaagcag aggttgcggt gagccaagac 9240
catgccattg cactccagcc tgggtaacaa gagtgaaact ccgtctcgaa aaaaaaaagg 9300
gctggggccg ggcgtggtgg ctcacgcctg taatcccagc actttgggag gccgaggcgg 9360
tttgatcaca aggtcagaag tttgagacca gcgtggccaa catggtgaaa ccctctctct 9420
actaaaaata gaaaaactag ccaggtgtgg tggcacatgc caataatccc agctacttca 9480
tctgaggtca ggagttcaag accagcctgg ccaatatggc gaaactccgt ctctactaca 9540
aatacaaaaa ttagccaggc gtggtggcgc gcgcctgtag tccagctact ggggaagctg 9600
aagcaaggag aatcgcttga acctgggagg cggaggttgc ggtgagccga gatcgcatca 9660
ttgcattcca ggctgggcaa gcctaggaga gaagagcgaa attgtgtctc aaaaaaacaa 9720
ttaattaaaa taaaatcagt gccataggct gtttctggta ggcagtgggt tcctagggtg 9780
gagcagggaa gggctggaaa gggtcgcctg tgcccagccc gactccccgt gcttctcggg 9840
ggacagcacg gctagctgct gctgcggagc ccacagtggg gttctggggg ggggggtgca 9900
ggcagaaggc cgccatgcct cccctcccct ttccacccca gtgagagtgg gctcgaaggc 9960
agggagtctg cccgccttgt ggagcctccg cagaggcacc cagggctgcc gtgagcgcac 10020
agcctggcgg tgtcccacgg ccctgccctt tcctgtcact tccacctgtt gcaaaacact 10080
gtgctttcgt atcttttctt tctacctgtt tggaaatgtg aaaatcactg ctgccgtagg 10140
aaacgggccc agatccgggc tgtggctctc ccatccgcac cgcccccgcg gtgtccagcc 10200
aggaggtggg aggtgggcgg cactcagtgt gcctggttgc ggcgatgatt ggcagatgct 10260
tggacctatg gtaaagagga ttttcgcgag ctgggagtca gtcctggact ttgtaggtct 10320
ttacatccca ggcggccttt atggggttcc agcatctgct gaggcccctg cgccagccat 10380
gtgaggcctg ggtgtcctcg aggcctctgt ttgtgcccct ctctggcagc gggagggag 10440
agctctgaga cccagaggga gctcaggggc tggcagccgc agaacagcgg gggtaatgg 10500
gtccactggg ggttctgggc tgtgcacaca gtgggcttcc ggtgcccagc cagctggggc 10560

aggcctggaa tgttggttcc tctctcagga tctggggcgc tgctgtagct gtcggggaat 10620
cagtgaggcg gctggacggc ccgtatatcc cacaaacgcc accgtattgc tcaaactgga 10680
ctcaattttc ttaacacctt tttttttttt tttttttttt tttttggaga cagggtctca 10740
ctgtatcgcc gaggctggag tgcagtggcg caatcatagc tctgggctca ggtgaccctc 10800
ctgcccccaa ggtagctggg actgcagatg tgcaccacca cacccgacta gttttttgta 10860
gagatggggt ttcgccatgt tgtccaggct ggtctcaaac tcctagactc aagctatccg 10920
cctgcctggg cctcccagag tgctgggatt acaggtatga gccactgctt cgccacgttc 10980
acctttattt acttatttat ttatttttga gacggagtct cgctctgtcc cccaggctgg 11040
agtgctgtgg cacgatcttg gctcactgca agctccgctt cagcctccca agtaagctgg 11100
aactacaggt gcctgccacc aagcctggct aattttgttt ttgtattttt agtagagacg 11160
gggtttcacc gtgttagcca gtatggtctc gatctcctga ccttgtgatc cgcccgcctc 11220
ggcctcccaa agtcctggga ttacaggctt gagtcactgt gcccggccca cctttttatt 11280
ttttatttta tagagacaag ggcttgctgt gttgcccagg ctggtcttga acccctgacc 11340
ttaagtaatc ctctctggcc tcccaaagtg ctgggattac aggtgtaagc catagtactg 11400
ggcctaaagt tcaccttttt tttttttttt tttttttttg agatggagtc tcgctctgtc 11460
acccaggctg gagtgcagtg gcgtgatctt ggctcactgc aagctctgcc tcccgggttc 11520
acaccattct cctgcctcag cctcccgaat agctgggact acaggcgcct gccaccatgc 11580
ccggctaatt tttttgtatt tttagtagag atggggtttc actgtgttag ccaggatggt 11640
ctcgatctcc tgacctcgtg atctgcctgc ctcggcctcc caaagtgctg ggttacaggt 11700
tgtgagccac cgcacccggc aaagttcacc tttttaaagt gtgtaattca gtggcatata 11760
gtacattgac agagttgcac aactatcacc tctatcagct tccagaacat tcttgttacc 11820
ccagaaggaa accccgtctc tgtcagccat cactccccat tccctcccca gtcccggcac 11880
ccacgcattc ccttcctgcc tctggattgg cctgtcctga acatttcaca cgagtggtgt 11940
cacacaccgc gtggcctttt gcgtccggct tgtctcacgg agtgtgctgt cctcaagggg 12000
catccgcgcc gtggcctggg tccgaacctc tctgctgttc acggctgcgt cactttccag 12060
tgtgtggagg gccacgctgc gtttgactgc tcatctgtgg ctggacactt gggctgctcc 12120
cgcctctgcc tgtgaattgt ggctgctttg gtgcacgtgt gctcagccgg cgtccctcta 12180
gaatccttga gggccttgtt tcagccacat caggagggat tcagggacca gggcacgcgg 12240
gtctggagct gggtgttcct gggtctccat gtgcctccca gtcctgctct gagccgtggg 12300
aggccggggc gagggccagg cccatctctt ccctgtatga tctgtttcta ggcagacgga 12360
ggctcctagc cccacctctc tgtgagcttg agtggcgttt atgactctcc ttcctatttc 12420
ctgtcctgct aatcaacaag tcagctaaac agtccatgct gcctgggcaa gcccagtagg 12480
cccctaacgt ggcacctcgg agcggctggc gatgccccaa gagccgggct tccccctcct 12540
ggaggctctt cctgttcctc gccaagctct taggtttcgc tgggcctcct cttctgctct 12600
ttttctggct ggagacgtca gaggaagcct ggcgatctag agaagctgat gtgcacccat 12660
catcccggag gtgactgcca ccaggcgggg gtggctccac ccccactcct cccctggcat 12720
aaatcggggg acgcacgtgc ctgcgcagaa tgccttggtc ccttttaatt aaacagacaa 12780
cccagtgtcc cttggtctat gctgacctgc tcaggagtgc ctgggctctt ccacgtttca 12840
gctgctgtgt gtcctgctgc tgtgaagtgt ccccgttgag gtggaggcag ggcagggcgc 12900
cttcggtgag ggcgagtcct cctgtgcccg tttccccatc tgtagaatga tggctcatgg 12960
aggagacatg gtgtcggcag gtggtggccg gcgcagggag gtttccagga gggagtcctc 13020
caccctcatt tgcttttagg gccgtgcagc ctcccgagcg gcccccacga ggctctgagg 13080
ctgcatgcag gttgagctcc tggcctcact gcccctcctc agggttagga ctgtgctgga 13140
gcctgggtgg gtgtggggtg gtccctgcac aggcagcagg aggtgggcca gagcggtgct 13200
ggacctgaag gcccgtggct ccctcagctc cactgaggag gctggaatag tgtcctgggc 13260
caccttccct gccggtagcc ggctcctgct gagggagggg gtgctttcgc ctcctggtgg 13320
agcatcaggc tcttggggcc tgtgcgtccg agcatctggc ctctgctgtc tgaaaacagc 13380
accttccccg ccccccggcc accgcatctc ctctgaatag aaggcaagtc ttgcctccca 13440
ggaatggcct gtgaccttca tcagcggggg aggttagggg cacacagagc cctggggaac 13500
aaggggatct ggggctgagc cgggcctctc gggcccaccc cgagctctcc tgagcagcct 13560
ggcaggcagg aaggcgctgg gccccggggc ctttgccgag cccagcctca ccctcagcgt 13620
ccccctcaat gccctgggtg cctgccaggc tgtggacaca ggccctggcc agttctataa 13680
ataaccttgc cgggccctgc tgggaattgg tgccaccacc acatagccca gagagaacgg 13740
aaacacaggg cttctcagcg agcagaccag gcctgcggtg accagtggaa gtgggggacg 13800
ctttcagggc tgggcagccc gacagggcac cgggcacagc caggggccgg aaccagttgc 13860
tcactggctc cgcttctggg ggactgtgtc cctctgtgcc ccgtccccat cacaccttgc 13920
tttcccttca tcctgctctc ctagcccctc cccagactgg cggccccttg tccaggccca 13980
gccccagcac agcctgcagc cctggcagag ctcaggcctt ggccttctga ggttgccctg 14040
agggcagtga gcaatgagcc ctgggactgg tgcactggga gggaaaggtg agccaaggct 14100
tcctgcaaga ggcagccctg agctgggggt ggggaaggct gggtcgtggg atacccctga 14160
caccccaagt tttttttttg agatggagtc ttgctctgtc acccaggctg gagtgcagtg 14220
gtgagatctc gactctctgc aacctccgcc tcccaggttc aagcgattct cctgcctcag 14280
cctcctgagt agctgggatt acaggcgccc atcaccacgc ccggctaatt tttgtatttt 14340

tagtagagac aaggtttcac catgttggcc aggctggttt cgaactcctg acctcaggtg 14400
atccacctcg gcctcccata gtgtcgggat tacaggcgtg agtcaccgcg cctggccccc 14460
cagctgtttt ttatgctcca tgcttgggct tcctgttaag aattaaaaaa gaaaaaaaaa 14520
actatggaga tggaatttac atactgtaca actcattcgt tcacagtgta caattcagtg 14580
gcattaagta cattcacacg gctgtgcaac cactacttct aattccagaa cgttcggtca 14640
ccccaaaagc aagccccatt cccattgtgt cactctccat cccctccacc agctcctggc 14700
cactctgaat ccacttcctg gctctggatt ggcttgtcct ggatattgca tagaaatggg 14760
atcctgtcgg ccgggcgcgg tgactcacga gagcaagaga tccagaccat cctggccaac 14820
atggtgaaac cccatctcta ctaaaaatac aaaaattagc tgggcgtggt tgcgggcacc 14880
tgtagtccca gcgactcagg ggctgagtca ggagaaatcg cttgaacccg ggaggcagag 14940
gttgcagtga gctgagatca tgccattgca ctctggcctg gcgacagagt gagactccgt 15000
ctccaaaaaa aaaaaaacaa gaaatgggat cctgtcatcc cagcgttttc cgaggccgag 15060
gtgggcagat gacttgagcc tagtagttca aacgagacca gcctgggcag cacggtgaaa 15120
ctgtctctac taaaatacaa aaattagctg ggcatggtag cacacgtctg tagtcccagc 15180
tacttgcggg gctgagatgg gaggatcgct tgagcctggg aggtgcactc cagcctgggc 15240
gacagggaaa ccctgtctca aaaaaaaaaa aaaaagtgca tcacacactg tgtgtccttt 15300
agtgtctcac tgtgacatcc tcaaggtgca gctaggctgt ggctcgagtc agagcctcac 15360
tggtttttgt ggctgagtct tgttgagtgc gtggatgggc tgtgctgatc ctcgtctgtg 15420
gaagggcccc tgggtggttc cacatctcgg ctgctgtgcg tcctgctgct gtgagcatct 15480
cgtgctccgt ttctgcgtgg cgtgtactgt tcctcagggt gtgtacctgg gagtggagct 15540
gctggctcac agggtaacag acctttggag aagctgcaga cgcttccacg cccacccact 15600
gggtgtgagg accttgctcc tcgtcatttt tgtttttgtt taattttttc ttttatgaga 15660
cagggtctta ttctgttgtc caggctggag tgcagtagca cgatcacagg taactacagg 15720
ctcaagtgat cctcccacct gagcctcccg agtagctagg acttgaggca cgtacctcca 15780
cacctggcta atttttgtat ttttagtaga gacagggttt cgccgtgttg ctcaggctgg 15840
tctcgaactc ctgagctcag gtgatccacc cgcctcggcc tcctatagtg ctgggagaaa 15900
tgaagtgtct gcgacagggc ggcagctgca gagggggctg tgctctctgg cctgttgtgc 15960
cccacccttg tgacaggcag gtgggcgtgg ccaactgggc ggcagctgca gagggggcag 16020
tgacaggcgg gtgggtgtgg caggacccca caatgtctct ttctgtggcg tctccttctt 16080
cgcctgcccc ttcctatggg ccatccttcc agctcacctg cggcccaccc tccaaggagt 16140
gggaccctcg gagttgggag cccttggctg cgtgggactg tactgtgtga cggagccatc 16200
accagggctc caactcaccc tggctcctgc cacagtctgg gctccatctg tgctaccatt 16260
gcagtagtct ggtttctagg aagcaaaact gttactgagt tacaaagctc aattaagagt 16320
gtcctttggg aggccgaggc gggcggatca caaggtcagg agatcgagac catcctggcc 16380
aacatggtga aaccctgtct gtactaaaat acaaaaaaat tagccggctg tggtgacacg 16440
tgcctgtagt cccagctact cgggaggctg aggcagggaa atcacttgaa cctgggaggc 16500
agaggttgca gtgagctgag atcgggccac cgtgctccag cctagggaca gagcaagact 16560
ctgtctcaga aaaaaaaaaa agtgtcctaa ctgtgtcctc caaagccctc gccggccgat 16620
gacagactag agggcgctgt gctcccaccc cctaccgccc tgagcctgga cgcgtggccc 16680
ctgcagggcc ctttcccaca gcactgtgaa ctcacagctt ctctctaggg aagggaggag 16740
gtacgccact tccacaggga gatggggagg ccgactccag ggatccaggc catcatcctg 16800
acgttgggtc ggctgataca cccctgtcct ctctgtccca gg 16842

<210> 31
<211> 1157
<212> DNA
<213> Homo Sapiens

<400> 31

ggatcctgct ccctctgaag gctccaggga agagtgtcct ctgctccctc cgaaggctcc 60
agggaagggt ctgtcctctt aggcttctgg tggcttgcag gtgcagccct ccaatcctcc 120
tccccaagcg gcctgctgcc tataaggaca cgagtcatac tggatgaggg gcccactaat 180
tgatggcttc tgtaaagtcc ccatctccaa ataaggtcac attgtgaggt actgggagtt 240
aggactccaa catagcttct ctggtggaca caattcaact cctaataacg tccacacaac 300
cccaagcagg gcctggcacc ctgtgtgctc tctggagagc ggctgagtca ggctctggca 360
gtgtctaggc catcggtgac tgcagcccct ggacggcatc gcccaccaca ggccctggag 420
gctgccccca cggccccctg acagggtctc tgctggtctg ggggtccctg actaggggag 480
cggccccagg aggggagaga ctcgcgctcc gggctcagcg tagccgcccc gagcaggacc 540
gggattctca ctaagcgggc gccgtcctac gacccccgcg cgctttcagg accactcggg 600
cacgtggcag gtcgcttgca cgcccgcgga ctatccctgt gacaggaaaa ggtacgggcc 660
atttggcaaa ctaaggcaca gagcctcagg cggaagctgg gaaggcgccg cccggcttgt 720
accggccgaa gggccatccg ggtcaggcgc acagggcagc ggcgctgccg gaggaccagg 780

gccggcgtgc cggcgtccag cgaggatgcg cagactgcct caggcccggc gccgccgcac 840
tgggcatgcg ccgacccggt cgggcgggaa caccccgccc cgcccgggct ccgccccagc 900
tccgcccccg cgcgccccgg ccccgccccc gcgcgctctc ttgcttttct caggtcctcg 960
gctccgcccc gctctagacc ccgccccacg ccgccatccc cgtgcccctc ggccccgccc 1020
ccgcgccccg gatatgctgg gacagcccgc gcccctagaa cgctttgcgt cccgacgccc 1080
gcaggtcctc gcggtgcgca ccgtttgcga cttggtgagt gtctgggtcg cctcgctccc 1140
ggaagagtgc ggagctc 1157

<210> 32
<211> 8093
<212> DNA
<213> Homo Sapiens

<400> 32

ccatggaagg aaatgagatc agctagcgag ctgacacaga acaaggcagt ctaaaacaaa 60
tttttttaaa aatacgaaga acagatattg aagggaagag gtgcctgcaa agactaagaa 120
agcacacctg gagatggtat ctcctcaaag ctaaagtcat caagtgttca agtgtttcaa 180
ggagggtaag actattaaca aggacttagc atagtagagc aatttgagtg gcaatacggg 240
acactgggaa tacaaatctg tcaagaaaac tagtaggaat gagctatagg acagtaactg 300
gtaaggacct aataattttt tttttaatgt acgtatttta actatattca ctgctacaac 360
aggaccagta acaactatat ttatttaaaa aaaaaaagac tgccatgcag ttacagaatt 420
acttaataca gaaaacagta aaatacactt ttttcttttt cttttttttt tttttttttt 480
acaaacaaga ctagcttata gcaaattctc tatagctaag ggtcaattta aaatccttgg 540
cttatatctc cccctcactc aatgactaca tgatgcaaac taattttatt aacaccttaa 600
gcaaaacata ctggaatttc acaaaatgta caagatttca atatttaagg aactggggtt 660
agaaagcaga agtggctttc aggtcttcca gtctttctct caagtaataa agctctgctg 720
tgaatattca aagctattgg gaaattaccg gtagattttt ctgttttttt ttttcggttt 780
tccactatgt tgtttctcta gatatgtaag cttactctat taaccaaaat ctcagcttga 840
ccattcttga taagtaccta atcgacatgt aacttttttt ctgccttaaa tatgtataac 900
aggacagagc ccttaaatct gattcaatta ttaattcctg atttacaagt gctatggtga 960
gctaacagaa cttatcaatg cctttattgc actttactag ccaaatttag aaggttggaa 1020
ttagtctctc ctatctagta ttctgtcagt ttgcccagct tgtactttta attttgcttc 1080
taatggtaat ctgccctatc ccttgaaata aaataatcta cattttggga gggctaattc 1140
ttcattgtgc caggctgtcc catgcactgc aggggtgagt gtctttaggc ttaaatgcca 1200
acagaagccc ctagtaaata tgacaaccaa aaaagtgccc ctacacattt ctcagcatcc 1260
tctggaatga caggttactg cctctagttg aaagccactg gcacaacttt ggtttttaag 1320
ctcttatgcc atttatttta attgcccaga catcaattcc acctaaattc ttagtcatag 1380
cctggttcct tgaatttgct ggattagtaa ccacagatta aggtgtttca atagttaaga 1440
caggactttg gaacaagagt ttttaaattg tataatactt gagaggatct atgaatataa 1500
attgggtcct gtttataatt agttttacat aatgaacttt aagattgcct tttcatggtg 1560
aacagaagtt tggaaattac tgttttggca caaagcagat tatcttagta gaaatacaga 1620
attactgcaa tctgtgaata agactgcttt taaatatttc tacttgtgtg ctatcttaca 1680
tatagaatgt gtacgacagt tccaaatttt agaataaatc catttctagc atctaacaaa 1740
atctgatact gtatcatttt aaaacaaagt gtttacttta ggcaggattt tttaaaataa 1800
agcagcaata cccacgcaga taagacaaaa aagctaaaat atctcacacc tcctaatcct 1860
ggagtgcaat cttttttcct catcgttttt gatagggcca aacttgtgtc tacagtaaaa 1920
aaaaaaaaaa aaagaattac taactggcaa ccattaagat tctatactta ccatagtcct 1980
ttaataggca agctgataaa atagccccca gttattaaaa aaaaaatcca aggaaaaccc 2040
ccaataatta gtcttatctc caaattgcat gaagtctcct atatctgaaa cttaaaaatg 2100
attctaatga cttcctctat cagtaatgtg ttatcactga ggtgggtgat ggggagggaa 2160
gagggaagaa atctgtcagt attaccttcg aactcagaaa tgtttaaaaa aaagtctcaa 2220
acattttgat ggttagacaa aacacctcca ctgttatgta tgggcttcct ttttggaaac 2280
ttatgaactt gctatgtgag cttctgcaaa ttggttcaaa agcacattta aggagttgat 2340
aatttaagac tatatgaatc agaattttaa cactccatta aaataagagc tgaaattttt 2400
ggcatttatc ttcagaacac ctaaaaaaca gactgcaaat tcaactcaca ttaatactaa 2460
atctctttaa aattaactat atcataaaag acaatgactt tgtcactaaa ctaagtttta 2520
aaaaaggtgg cattctcatg tttcagtccc atgctgccat ttgagatgaa aaaaaaggca 2580
actgtcagaa ttttaattgt gatcagtttg gacggctggt acttggtact ttctcacaat 2640
ccaaattagt aaacttgaaa actcaaacca atttttgctt ttagaattaa attcttgttg 2700
agtttttcaa tcttgtacta caaagcctta tggattagat tcatttcttt ctctcacaag 2760
gtcatcccaa ccgggctcca tttcagttgt ggcaacccga aacagggctt ggagatgctc 2820
atctgttaat ggctgactca aagtgtgttg gtttcgagtc aaatatgaaa aagccttttc 2880

acagatttgg ttactatcaa acaaggatgc caccttacag gcaacccctt tgataattgg 2940
gtaggattca gcagacaatc cagcataaaa ctgccccaag tctttgattc tgtattcatt 3000
ccaaagatta gtgtttgcct gaagttttgt tagctccacc ctcactgaaa taggtgcata 3060
ttcaggttta aagttaaatg gatttgaaaa aagttctaag tcctttttaa tgaacctgag 3120
gtccttaaaa tgtctctcaa attctttttg gagacgacag atcaccattt gatacctatc 3180
aggatcaaat attttttcat cttccttatt ttgctgtttt agctcatcaa caacttctct 3240
gagggcagga aagtctgtta gatttttttc ctcaatatgt ctttgaaata aattcagctt 3300
aacttcgaaa gtacaaatat ggtcaaaggc agcagcagca aagactttac taactcgtaa 3360
ttcttcactg agttctcgaa ggtgttccat aatgtccacc aagaagccaa agtcacaaag 3420
ccattgtttg tctgagaagt ggactgttgt tgcccctact gaaaccaaga acgcttccat 3480
ttcttttctt agagagaata ttagttttaa agttttccct ctcctaagcc aattgttcag 3540
acatcgtcca ttaacccttt caccatgctc tgattcagat tccgttagta aagtctgaaa 3600
ttcaggtcgc ctaacgcctc tggtcttaat caaaactatc cattcggata tggtatttat 3660
gatctgatta acatctacat cataggagct caacagttcc aagtgaagaa atcctgaata 3720
atgaatgaca ttccaacagt tggggcttac ggccttttct ctcatgtatg agacgagtcc 3780
tgagttctca ccaatcatcc tcaaagtatg ggtcgtggtc agtccaacca ttctctgcaa 3840
gctaagccct gctgtctgca gggactctag gattgccgac atgagcgcac caacactgaa 3900
atgatgagtc aggttgatta tggtcagaag atcttcttgc acctccaact cagggcctac 3960
accgcggata aagaccagga ggtagttctc ataggccaca aaagcctggt cgtccaaggc 4020
aagagaatag gctttaaagt ccctggctcg gttaaaaagc tggttgcgta gattcctgtc 4080
aatgctcagg atcctctgcc ttgtgatatc tggagataag tcaacgcctt gcaggacgct 4140
tacatgctcg ggcagtacct ctctcagcaa cacctccatg cactggtata caaagtcccc 4200
ctcaccccag ccgcgaccct tcaaggccaa gaggcggcag agcccgaggc ctgcacgagc 4260
agctctctct tcaggagtga aggaggccac gggcaagtcg ccctgacgca gacgctccac 4320
cagggccgcg cgctcgccgt ccgccacata ccgctcgtag tattcgtgct cagcctcgta 4380
gtggcgcctg acgtcgcgtt cgcgggtagc tacgatgagg cggcgacaga ccaggcacag 4440
ggccccatcg ccctccggag gctccaccac caaataacgc tgggtccact cgggccggaa 4500
aactagagcc tcgtcgactt ccatcttgct tcttttgggc gtcatccaca ttctgcggga 4560
ggccacaaga gcagggccaa cgttagaaag gccgcaaggg gagaggagga gcctgagaag 4620
cgccaagcac ctcctccgct ctgcgccaga tcacctcagc agaggcacac aagcccggtt 4680
ccggcatctc tgctcctatt ggctggatat ttcgtattcc ccgagctcct aaaaacgaac 4740
caataggaag agcggacagc gatctctaac gcgcaagcgc atatccttct aggtagcggg 4800
cagtagccgc ttcagggagg gacgaagaga cccagcaacc cacagagttg agaaatttga 4860
ctggcattca agctgtccaa tcaatagctg ccgctgaagg gtggggctgg atggcgtaag 4920
ctacagctga aggaagaacg tgagcacgag gcactgaggt gattggctga aggcacttcc 4980
gttgagcatc tagacgtttc cttggctctt ctggcgccaa aatgtcgttc gtggcagggg 5040
ttattcggcg gctggacgag acagtggtga accgcatcgc ggcgggggaa gttatccagc 5100
ggccagctaa tgctatcaaa gagatgattg agaactggta cggagggagt cgagccgggc 5160
tcacttaagg gctacgactt aacgggccgc gtcactcaat ggcgcggaca cgcctctttg 5220
cccgggcaga ggcatgtaca gcgcatgccc acaacggcgg aggccgccgg gttccctgac 5280
gtgccagtca ggccttctcc ttttccgcag accgtgtgtt tctttaccgc tctcccccga 5340
gaccttttaa gggttgtttg gagtgtaagt ggaggaatat acgtagtgtt gtcttaatgg 5400
taccgttaac taagtaagga agccacttaa tttaaaatta tgtatgcaga acatgcgaag 5460
ttaaaagatg tataaaagct taagatgggg agaaaaacct tttttcagag ggtactgtgt 5520
tactgttttc ttgcttttca ttcattccag aaatcatctg ttcacatcca aaggcacaat 5580
tcattttgag tttctttcaa aacaaatcgt ttgtagtttt aggacaggct gatgcacttt 5640
gggcttgact tctgattacc ctattgttaa attagtgacc cctcttagtg ttttcctgtc 5700
ctttatttcg gaggacgcac ttcgaagata ccagatttta tgggtcatcc ttggattttg 5760
aagcttataa ctgtgacaaa aaatgtgaag ggaagagatt tgaaacatgt ggaaggaaaa 5820
gtgagtgcag actataaact tccaaaaaga caagcccaaa atacacctaa acgttatgtc 5880
agattatttt gttaaaatca gttgttagtg acgtccgtac gttaatagaa aaaagaatgc 5940
ttcagtttgg agtggtaggt ttctagaggg atttattgtg aaagtataaa ctattcaggg 6000
caatgggact gagagaacag tgggtagaaa ggaccactga aggaaaggaa gagaattgga 6060
aggtagatga aagaaggagc aagaacctgg ggatgttttt tccttttcac ttgtaatagt 6120
agtaacagaa gcaatggcag actggctttt gtttctactg tgttagaatg aattgacagg 6180
acaactgggc ctattattgt actgtgccag aatactgtaa aacaaaacta aacatactag 6240
cttggtggct tgtaattaat tacttaagtg gagattttta tttttttttt attttttttt 6300
tagacggagt ctcactttgt cacccaggct ggagtgcagt ggcgcgatct cagctgactg 6360
caacctcctc ctcacaggtt caagggagat tctcctgcct cagcctcccg agtagctagg 6420
actataggca tgtgccacca cacctggcta attttgtatt tttagtagag atgggatttc 6480
tccatgttgg tcaggctggt gtcaaaactc tcgatctcag gtgaaccgcc tgcctcagcc 6540
ttccaaagtg ctgggattac aggcgtgagc caccgcgccc tgcagttttt tgtattttta 6600
atagagacag ggtttcacca tgttagccag gatggtctcg atttcctgac ctcaggtgat 6660

ctgcccgctt tggcctccca aagtgctggg attacaagca tgagccaccg cgcccggctc 6720
aagtggagat ttttatatgg agtccagtta tactcttttt aatatataag ttgagatgac 6780
taatacaact tcaatacagg ggctcatgag aaatgtctgt aatatttaag taacttattg 6840
tcttctttct ttttttttta agatgaagtc ttactctgtt gcccaggcgg aagtgcagtg 6900
gcgtgatctt ggctcagggc aacctctgcc tcctggtttc aagcgatctt cctgcctcag 6960
cctcccgagt agctgggagt acaggcgtgc atgaccacac ccggctaatt tttttatttt 7020
tagtagagac ggggtttctc catgttggcc gggctggtct tgaactcctg acctcaggtg 7080
atccgcccac ctcagcctcc ccaagtgttg ggattacagg tgtgagcccc cgtgcccagc 7140
ctattatctt atttctgaat aaagaattgt ctgtgtgggg aatagataac tctttctcat 7200
gcagcccctg ctagaaaatt tgttttctct agcagttggt ctgtgcttat aggctactct 7260
ttgaaagcac aaaaaattta ttgacttctt ttttttgggt tttttttttt ttttgagaca 7320
gagttttgcc cttgttgccc aggttggagt gcaatggcgc gatctcagct caccgcaacc 7380
tccacctcct gggttcaagt gattctcctg ccttagcctc ctgagtagct gggattacag 7440
gcatgcgtca ccatgcctgg ctaattttgt atttttagta caaatggggt ttctccatgt 7500
tggtcaggct ggtctcaaac tcctgacctc aggtgatcca cccgccttgg cctcccaaag 7560
tgctgggatt atgggtgtga gccattgcgc ctggccagaa aattcattga cttcctaaag 7620
atttattaac tttctgcatt actttttttt ttcccctcca tcgtaaatat aaaaaggaat 7680
agtagagaaa atcattcaga attttatttt ttagtgacat tatttagtga cattttatta 7740
gagtcactta ggaacctgag gctgaataaa gttcaggtaa aagtaaaatt agttgagaag 7800
agacatctgc caaaagaaat ctatttttaa cttcacttgc tgtctttcct agaggaacag 7860
aaatagtgct gaatgtccta ttagaaatga tggttgctct gcccgtctct tccctctctc 7920
tcacacaata tgtaaactca tacagtgtat gagcctgtaa gacaaaggaa aaacacgtta 7980
atgaggcact attgtttgta tttggagttt gttatcattg cttggctcat attaaaatat 8040
gtacattaga gtagttgcag actgataaat tattttctgt ttgatttgcc agt 8093

<210> 33
<211> 6521
<212> DNA
<213> Homo Sapiens

<400> 33

acaaaattat gcatgggtaa aagatccaat caggtgcagt tcagaaaaac ggattttttt 60
tttttttttg agacagagtc ttgctctgtc acccaagctg gagtgcagtg gtgcaatctc 120
ggctaactgc aaccttcgcc tcgcaggttc aagtgattcc cctgcctcag cctcccgagt 180
ggctgggatt acaggcgtgt gccaccacca cacctgacta atttttgtag ttttagtaga 240
gacgggaggg tttcaccatg ttggccaggc tgctctcaaa ctcctgacct caggtgatcc 300
acccacctca gcatcccaaa gtgctgggat tacaggcgtg agccaccatg cccagaccca 360
aatttttctt gttttctttc tttttttggg ggtggggggc agggtctcac tctgttgccc 420
aggctggagt gcagaggctt gatcttggct cactgaaacc tctacctcct aggctcaagc 480
aatcctccca cctcagcctc tcgagtagct gggaccacag gcgtgcacca tcacacctgg 540
ctaatttttt gtatttttgg taaagacggg gttttgccat gttgcccaag ctgttcttga 600
actcctgggc tcaagcgatc ctcttgcctc accctcccaa cgtgctggaa ttgcaggcat 660
gagccaccat gcctggaaca aagttttatt tttcaagtgg atcatttaaa aaaatgcttt 720
atggccaggc acgccacttc tctccagcct gggcaagtga gccgagattg tgccactgcg 780
gtctggtctg agcgacagag ggagactcca tctcagataa ataaataaat aaaattaaaa 840
caaatttaaa cattctttat atgacaaaat tattctcaat ggcaattata aaatgaataa 900
taagggccaa gaaataaact cagtgaatat tttctttcct tgaactttgt aaatataatc 960
atgtcagcat aaaaacagcc atgcattgct taacaacagg gatatgttct gagaaatgag 1020
tcactaggca attttgtcat tgtgcaaaca tcatagagtg tattttcaca aacctagatg 1080
gtgtagccta ttacccatct aggctacatg gtatagcctg gtgctcctag gccccaatgc 1140
ttacagcatg ttaccatact gaatactgta ggcagttgta agacaatggt atttgtgcat 1200
ctactgtaga gacagggtct ctccgtctgt cgcccaggct ggagtgcagt ggcataatca 1260
tagctccctg cagcctccaa ctcctgggct caagtgatcc tcccacctca gtctcccaag 1320
tagctaggac tacacgcttg tgtcatcagg cctgtctact gtttttgttt gttttttggt 1380
agagatggag ggtctcacta tattgctcag actggttttg aactcctggc ctcaagcaat 1440
cctcctgctg ccttggcctc caaagtgctg gtattacagg aatgagccaa aagctcaata 1500
tggctgttga ataaaagact gactcaatca agtacggacc ctgcccttat ggagctgaag 1560
gccgttagag cagataaaat gagttatcac ccttatatga gttagacggt ggacaaagtt 1620
attattgagg gcaggcaaag aactgggttg gctcaaagat gccagggatc ttttccagac 1680
tagtggtaga gggttggtta aggagtttcc tgaaggaggt ggcctttcat ctgagtttca 1740
aagaacagaa gagattagac agggaggaca gtaaaatttt tttttttttt tgaaacagtc 1800
tcgctttgtc gcccaggctg gagtgcagtg gcgcgatctt ggcttactac aagctttgcc 1860

tcctgggttc acgccattct cctgcctcag cctcctgagt agctgggact acaggcggcc 1920
gccaccacgc ccggctaatt tttttttttt ttttttttca gtagagacgg ggtttcacta 1980
tgttagcgac gatttttgtc tgtttgtttg tttttgagac agagtctcac tctgtcgatc 2040
aggctggagt gcagtggcgc gatctcggct cactgcaacc tctgcctccc aggttcaagt 2100
gattctcctg cctcagcctc ctgagtagct gagattacag gtgcctgcca ccatgcccag 2160
ctaatttttg tatttttagt agagacgggg tttcaccatg ttggccaggc tggtctcgaa 2220
ctcctgacct caggtgatct gcacacctcg gcctcccaaa gtgctggtat tagaggcatg 2280
agccactgca cctggccagt aaaactattt gtgacacagc agcaagtgca caagcactga 2340
cctatcagag ttgaaggtgg ctgaggcact caagtgggct cctggggact ctgttatgcc 2400
agggaatacc tctttagata agggatcagg aggtctggag ggtccctaag gaagagctag 2460
ggtagcaggg gtaccctgga gctaggggcc atggctatat gccaacagga ttagaaatat 2520
cttaatgtat ttaattaacc tacacaacca tattggtggg tgcctactga atattagccc 2580
tagatatgga cctgaaatgg ccttattctc ttttagtttg tttaagccaa tggagcctta 2640
ctagaatgtg agctacaaaa cagtagagct attctctatt caatgctgca tctgtagggc 2700
ctagaacagc acctgaagga ctggcggagg cttaataaat tgttaaaagg gcagactagc 2760
caggtgtgat ggcgtgtgcc tgtagtccca gctacttggg aggctgaggc aggaggatca 2820
cttgatccca ggatttctgg gctgtagtgt agtataccag tcaggtgtct gcactaagtt 2880
ctgcatcaac ctggttgcct aaggatggtt gaactgaccc aggtggaaat tggagcaggt 2940
caaaactcaa tgctgatcag taacagggtc gcacctgtaa atagccaccg ccctccagcc 3000
tgggcaacac agtgagatcc catctctaaa ataaatttta aaaataatta attagaaaaa 3060
aaaaccagcc tgtaatccca gcactttggg aggccaaggc gggcagatca cctgaggtca 3120
ggagttcaag acaagcctgg ccaacatggc aaaaccccaa tctctaccaa aaatacaaaa 3180
attagctggg cgtggtggca ggtgcctgta gtcccagcta cccaggaggc tgaggcaaga 3240
gaatcgcttg aacctgggag gtggagattg cagtgggccg agatcgcgcc acttcactcc 3300
agcctgggta gcagagtgcg actctgtctc aaaaaaaaaa aaaaagaaag aaaagaaaag 3360
aaaaagaaaa gaaaagaaaa aaactgggag gcctaagccc attcttggtg tcctcacact 3420
cttctgcccc atctccttgc acccagtctt cccctgcaat ctgtgctcca tactagccct 3480
caagccctca acgtgaccta gtatgagaac tggattctgt cactctcctg ctcataatat 3540
tttatgcctt ccctttgccc agaatatcat tctccctatt gtttttacca atggaactgg 3600
tatttcttca aggacatgat caaatttgcc tacttctata ttatcttcta aagcagaatt 3660
catctctctt ccctcaatat gatgatattg acagggtttg ccctcactca ctagattgtg 3720
agctgctcag ggcaggtagc gttttttgtt tttgtttttg tttttctttt ttgagacagg 3780
gtcttgctct gtcacccagg ccagagtgca atggtacagt ctcagctcac tgcagcctca 3840
accgcctcgg ctcaaaccat catcccattt cagcctcctg agtagctggg actacaggca 3900
catgccatta cacctggcta atttttttgt atttctagta gagacagggt ttggccatgt 3960
tgcccgggct ggtctcgaac tcctggactc aagcaatcca cccacctcag cctcccaaaa 4020
tgagggaccg tgtcttattc atttccatgt ccctagtcca tagcccagtg ctggacctat 4080
ggtagtacta aataaatatt tgttgaatgc aatagtaaat agcatttcag ggagcaagaa 4140
ctagattaac aaaggtggta aaaggtttgg agaaaaaaat aatagtttaa tttggctaga 4200
gtatgaggga gagtagtagg agacaagatg gaaaggtctc ttgggcaagg ttttgaagga 4260
agttggaagt cagaagtaca caatgtgcat atcgtggcag gcagtgggga gccaatgaag 4320
gcttttgagc aggagagtaa tgtgttgaaa aataaatata ggttaaacct atcagagccc 4380
ctctgacaca tacacttgct tttcattcaa gctcaagttt gtctcccaca tacccattac 4440
ttaactcacc ctcgggctcc cctagcagcc tgccctacct ctttacctgc ttcctggtgg 4500
agtcagggat gtatacatga gctgctttcc ctctcagcca gaggacatgg gggccccagc 4560
tcccctgcct ttccccttct gtgcctggag ctgggaagca ggccagggtt agctgaggct 4620
ggctggcaag cagctgggtg gtgccaggga gagcctgcat agtgccaggt ggtgccttgg 4680
gttccaagct gagtccatgg ccccgataac cttctgcctg tgcacacacc tgcccctcac 4740
tccaccccca tcctagcttt ggtatggggg agagggcaca gggccagaca aacctgtgag 4800
actttggctc catctctgca aaagggcgct ctgtgagtca gcctgctccc ctccaggctt 4860
gctcctcccc cacccagctc tcgtttccaa tgcacgtaca gcccgtacac accgtgtgct 4920
gggacacccc acagtcagcc gcatggctcc cctgtgcccc agcccctggc tccctctgtt 4980
gatcccggcc cctgctccag gcctcactgt gcaactgctg ctgtcactgc tgcttctggt 5040
gcctgtccat ccccagaggt tgccccggat gcaggaggat tcccccttgg gaggaggctc 5100
ttctggggaa gatgacccac tgggcgagga ggatctgccc agtgaagagg attcacccag 5160
agaggaggat ccacccggag aggaggatct acctggagag gaggatctac ctggagagga 5220
ggatctacct gaagttaagc ctaaatcaga agaagagggc tccctgaagt tagaggatct 5280
acctactgtt gaggctcctg gagatcctca agaaccccag aataatgccc acaggacaaa 5340
agaaggtaag tggtcatcaa tctccaaatc caggttccag gaggttcatg actcccctcc 5400
cataccccag cctaggctct gttcactcag ggaaggaggg gagactgtac tccccacaga 5460
agcccttcca gaggtcccat accaatatcc ccatccccac tctcggaggt agaaagggac 5520
agatgtggag agaaaataaa aagggtgcaa aaggagagag gtgagctgga tgagatggga 5580
gagaaggggg aggctggaga agagaaaggg atgagaactg cagatgagag aaaaaatgtg 5640

cagacagagg aaaaaaatag gtggagaagg agagtcagag agtttgaggg gaagagaaaa 5700
ggaaagcttg ggaggtgaag tgggtaccag agacaagcaa gaagagctgg tagaagtcat 5760
ctcatcttag gctacaatga ggaaattgag acctaggaag aagggacaca gcaggtagag 5820
aaacgtggct tcttgactcc caagccagga atttggggaa aggggttgga gaccatacaa 5880
ggcagaggga tgagtgggga gaagaaagaa gggagaaagg aaagatggtg tactcactca 5940
tttgggactc aggactgaag tgcccactca cttttttttt tttttttttt gagacaaact 6000
ttcacttttg ttgcccaggc tggagtgcaa tggcgcgatc tcggctcact gcaacctcca 6060
cctcccgggt tcaagtgatt ctcctgcctc agcctctagc caagtagctg cgattacagg 6120
catgcgccac cacgcccggc taatttttgt atttttagta gagacggggt ttcgccatgt 6180
tggtcaggct ggtctcgaac tcctgatctc aggtgatcca accaccctgg cctcccaaag 6240
tgctgggatt ataggcgtga gccacagcgc ctggcctgaa gcagccactc acttttacag 6300
accctaagac aatgattgca agctggtagg attgctgttt ggcccaccca gctgcggtgt 6360
tgagtttggg tgcggtctcc tgtgctttgc acctggcccg cttaaggcat ttgttacccg 6420
taatgctcct gtaaggcatc tgcgtttgtg acatcgtttt ggtcgccagg aagggattgg 6480
ggctctaagc ttgagcggtt catccttttc atttatacag g 6521

<210> 34
<211> 6565
<212> DNA
<213> Homo Sapiens

<400> 34
   cgacccttaa atttcataag ggttatcgta gttttctgaa gtagaaaatg tagcccattt 60
   cttgccagct catgggctac accttgacct aagcatcttt acggtgggta cttgcgctta 120
   ctttgcagcc ttcgtcagag attatgctag aagatggcgg tatataggct gagcaagagg 180
   tggtgaggtt gatcggggtt tatcgattac agaacaggct cctctagagg gatatgaagc 240
   accgccaggt cctttgagtt ttaagctgtg gctcgtagtg ttctggcgag cagttttgtt 300
   aatttaactg ttgaggttta gggctaagca tagtggggta tctaatccca gtttgggtct 360
   tagctattgt gtgttcagat gcgttaaagc cactttcgta gtttattttg tatcaactgg 420
   agttttttac aactcaggtg aattttagct ttattgaggg gaattgatct aaaacactct 480
   ttacgccggt ttctattgac ttgggttaat cgtgtgaccg cggtggctgg cacgaacttg 540
   acgaacccta gggttagtat agcttagtta aactttcgtt tattgctaaa ggtttatcac 600
   tgctgtctcc cgtgggggtg tggctaggct aagcgttttg agctgcgttg ctgcgtgctt 660
   gatacttgtt ccttttgatc gtggtgattt agagggtgac tcaccggggc aggatgcttt 720
   gcatgtgtaa tcttactaag agctaatagg ctacgaccaa acctatttgt ttatggggtg 780
   gtatgagcct gtcgaaacat tttcagtgta ttgctttgag gaagtaagct acataaaccg 840
   tatggggtgt ctttggggtt tggttggttc ggggtatggg gctagcagcg gtgtatatgt 900
   tgagtaaggt gggcagggag ttgcattgat ggggttagta gaatgtgagt tgagggagga 960
   tgatgtgtta gttgaggggt gactgttaaa aatgcacttt ccttcctgcc accatgtgaa 1020
   gaaggatgcg tttgcttccc cttccaccat gattttaaat ttcctgtggc ctccccagcc 1080
   atgaggaact gtgagtcaat taaacctctt tcctgtacaa attgcccagt cttgggcagt 1140
   tctttataag aacatgagaa tggattaaca caggtccaat tctggccacc cctcaacctc 1200
   ccaagccatt ggagttgact tcctgggcag tcttaatatg attacccctc ccactctcca 1260
   gtcaccaggg catatcaggc ttcatgaact actgactcca acccctgggt tggaggacag 1320
   gaaaattctt agcagaggct aactggagat acccgcttct ctccaggatc tctagaacca 1380
   tttcaatact ttgaaatgtt ttaccttcag acactgggta atgaatgtta agtacaaaat 1440
   attattgggt attgtggtta actaggaata acagagtaga agattatgac ccatttcagc 1500
   ccatcctaaa attcacattt tattgcattt agggttgggt ccagaaaggg gatcatcact 1560
   taattttagc aagtcccaaa aaatagttgt tttgacttta tgtgaaacta caggttattg 1620
   attacactgt ataatttata tttgggccta ctgaatgatg gttcaagata acttgcaaat 1680
   ctgataccac agtgagctat ttaatgttta atgtaacttt tcaaggtgat gaaatcaatt 1740
   actcaggtag tagaataagt tattgtcatg tagtataaat aactgcttat taattttcta 1800
   taatcacctt tgtgatatta agcagcttca tcaatagctc cttttataca atttcatttt 1860
   atcatatact gatctccact atgagacatg attttcttgt ggtgaattta tgttaaggta 1920
   cttacttgag ttctctgctg agaactaaat taattctacc ctttaaaggt cgattcttct 1980
   caggaatgga gaacccaggt cttcttaccc ataatcacca gattctgtta cctataaaat 2040
   cacaaagaaa gcctaagtat attttgggaa tatacatata ttttcatgta ttatatacaa 2100
   catgtccttg aataacatcg ttttcttcta acgttgatga gaaaaataat caattcctgg 2160
   ccagggccac cgtctgtgtg gagtctgcat gcttttccca tgtctgcatg gggtttctcc 2220
   aggtattcca gtttccttcc acatcccaaa gacatgcaca ttattcttct taaatgtatg 2280
   tatagctcat atttacttca gtgttttcta ttagaagtgt ttttggcctt catttagaag 2340
   tttggtgatg tttttgtgac cagcaatatg ccctaggaac ttaactcttg tgtacatcca 2400
   gtagcctatg gtaaaattgg ttttgctcta cattgtttca cttaaagtca cagtttccaa 2460
   gaactcacag atgacattga ggactatcta tgtgcataga tatgatggac atcctgacat 2520
   ttagtggatc tcctttaaaa aactgaactt aaaagtctta ccaaaattca tacttatcac 2580
   tcattaccta aatctaacta tttttccctt tggcattatc caatttatat ccaatattta 2640
   gttttaatca tttgcaattg tgtatgacat agtaaagatt ctggggaggc caagatgggc 2700
   agatcacgag gtcaggagat cgagaccatc ctggctaaca cggtgaaacc ccatctctac 2760
   taaaaataca aaaaattagc caggtgtggt ggcaggcgtc tgtagtccca gctactcagg 2820
   aggctgaggc agaagaatgg tgtgaacccg ggaggcggag cttgcagtga gcgtagatcg 2880
   caccactgca ctccagcctg ggcgacagag caagactccc tcttaaaaaa aaaaaaaaaa 2940
   aaagattctg agtcaaagtg ctcaagttga atgcattttg tcatccacaa gacaaatcgt 3000
   gttaacccct tgtggtttac tttatctata aaatagagat aacaatagtt cctgcttcta 3060
   gggttgttgt gggaattaaa gacttagaat aatgttcagc ctctaatcag tgctgtcaca 3120
   actgtctgat acaattgtat tatatttgtg tactttgtag attgatatta aatcatactt 3180
   ttaaaaatag gtgcttaatg ttccactcaa ttaccttaaa acatgtttaa ttatgtctct 3240
   atcctactct tataacactt ctataaaaac tttttacata tagcgtccac ttttggttca 3300
   gtttcttagg aaaataactt tgagagtcag ctatctgaac caaagaaaca ttaacattac 3360
   cagactatat tgggattttt gagactggct tttatcaatt ctttagctac gggctcttgt 3420
   catcatctct accagtgacc taagtgtcaa acccaaatgc cttgtatctg tcccattaaa 3480
   gagatgcagc atctgctcct ttcttactgt ttccatttcc tctgccatgc ctcctcttac 3540
   aaccataaat atccaggtct cttaggtttt aaacggggca tctctcaacc cccacattct 3600
   tttccttggt tattcccttc cctccaacag ttcaattcac ctagatcccc acgcctgaaa 3660
   ttatcctaga tgtcctagag gcgcctcatc attacaatgg tacattattc tccactcctt 3720
   tacatgtcac gccagctttc aaactgaaaa tctgagcgtt catccctggt gcatcacctt 3780
   taaattccag atctccaaaa tccaggtcca tgtaacctta aaaaattttt accctctctt 3840
   ctccactgcc cttgttcagg ccttatctct tccagcagct gttccaaagg cctactctgt 3900
   tttcctttcg gagtgctaac ctccaccgaa gcctccaccc agttgccaat tctgccccat 3960
   gcctgataat ttgctcgtgc gttgacatac ataaaatttc taagacaaaa attttttaat 4020
   aatggtaaat gaaccttggg aactgcatac agatcataca gatccataat aagagaaaag 4080
   gtcccagatt aacacggaaa actttccatt taactaacat ttgcactggt aaacttcatc 4140
   aagcaagacc ctacttaatc ccacattacc ttctactgaa gaggttgtgg tcattctctg 4200
   gaaatatctg aattcattcc tacaagttag agaaacagcg ttactcgaaa cattatccct 4260
   tgggctcgag ctctaaggca cctgacaaac ggagcgctgt gggtaggggt gaggtgtttt 4320
   ctccagggct gggactttgc cctgggcgag ggcgccgcag ggcaaagacc tcaccgggca 4380
   gcagaatccg ggcagaaatc agcaactggg cctcccgcgc agcagaaaag gggaatccag 4440
   tcggggccca cccttcctgc cagcgcagac cgcaagtctg gccccatcct ctcgccggga 4500
   gtcggcctgg cgcgtcccgc ccaggtaccc cgaccgtggg cagcctgcgc ccgtttgggt 4560
   cccatcgccc cggcccggca gatacctgag cggtggccag ggcaggtccc cgttcttgcc 4620
   gatgcccatg ttctgggaca cagcgacgat gcagtttagc gaaccaacca tgacagcagc 4680
   gggaggacct ccgagcccgc tcgttacagc agaacgcgcg gtcaagtttg gcgcgaaatt 4740
   gtggccgccc cgccccctcg tccctatttg tgcaggcgag gccccgcccc cccgccccgg 4800
   cgcacgcagg gtcgcggcgt gctcgcgccc gcagacgcct gggaactgcg gccgcgggtt 4860
   cgcgctcctc gccgggccct gccgccgggc tgccatcctt gccctgccat gtctcgccgg 4920
   aagcctgcgt cgggcggcct cgctgcctcc agctcagccc ctgcgaggca agcggttttg 4980
   agccgattct tccagtctac gggaagcctg aaatccacct cctcctccac aggtgcagcc 5040
   gaccaggtgg accctggcgc tgcagcggcc gcagcgcccc cagcgcccgc cttcccgccc 5100
   cagctgccgc cgcacgtagt aggttctgtc tgggactggg cagggccatc ggggctgggg 5160
   gggcggggct tgtgggtaag gcgggcggag gcgtggaccc tccgcccgat gatagggctg 5220
   gaggaggaag gggcgggctg aagaagggga aggtgggaag agcccagccg gggctacaaa 5280
   ttgggtgaag cgctgaggtt ttagtacttc cgtttgagga gataggcaaa ggttatgcag 5340
   gtttttaatg gcaggcctga gacaggaact caggtctcct gactcccatt ctgatgaggg 5400
   ggcttgtggg caaggggtgc gtcttttaac ctccatcctt tttttttttc cttggtggtc 5460
   gaagagtttt actgattttt taaaaaagtg ctggattggg tgactagaag aaagctgccc 5520
   ttaggtctcc agtttttaag actgcaacag catggctcaa tatttgaaat cactacggtt 5580
   aaaagcagtc atattgtgca gttccccagt gaacactttt tgtagtttag attataaagt 5640
   ctgaaataca gtttgggtgg ataaagtttc ttaggatggt atagatgtga ctaatcatgg 5700
   agtccttcct gttgccgtcc ttcaacgcaa taagtacgtt tgtttgtttt taaaccagag 5760
   atactgccac aggaaaagcc caagggtgac ccctcacttg ggtcacctgc acagtatttt 5820
   tcagactgag gctgtggaag ggcattgaag actggtgttt agccacctac cgtgttttaa 5880
   tgtaggtggc agcttgggtg cttcaaaatc ttgttccttt cgtatatatt tctcccttaa 5940
   aatgattact cctcttctgt tttttttgag gacgagctac gttttcatga attcaccaaa 6000
   attttgcacc caccatttaa agggtagctg gtgaaggact tatgtatgct tttggcaagt 6060
   tttctgccaa gaaaaaattc ataatcttgt cacatcttta gtgatgaaca taaacctttt 6120
   aaacaggaaa cacttaaatt gcaatgaaca tgtcaaaaat gatactgttc ttttattacc 6180
   tatcttttcc cctctgtgcc tttgctacat aactgtttat ttcttagagg tattttattt 6240
   agcatatgta agtagggtga ttcaagtttt ataattagag aaagctgaag gctcattgaa 6300
   tcaagaccgg tagcagcctt ggtttcaatt aattctaaac acggcttgag ttgctgaggc 6360
   tcctgactat cagactctac cttgtctttt atggtgttaa aaacagttca cccatagggt 6420
   tttccagagc taaaagtaga ggattctttg aagagtggat ggcaaggaac cttgtcattc 6480
   agttgtaaag tttaaattgg ttcacatacg tcaggccttc tcagagtaga gataacacat 6540
   cattttctaa ccttcccgat atagg 6565

<210> 35
<211> 2891
<212> DNA
<213> Homo Sapiens

<220>
<221> unsure
<222> (223, 366)
<223> unknown base

<400> 35

cccctttccc ccgaattgtt ttctcttttg gaggtggtgg agggagagaa aagtttactt 60
aaaatgcctt tgggtgaggg accaaggatg agaagaatgt tttttgtttt tcatgccgtg 120
gaataacaca aaataaaaaa tcccgaggga atatacatta tatattaaat atagatcatt 180
tcagggagca aacaaatcat gtgtggggct gggcaactag ctnaagtcga agcgtaaata 240
aaatgtgaat acacgtttgc gggttacata cagtgcactt tcactagtat tcagaaaaaa 300
ttgtgagtca gtgaactagg aaattaatgc ctggaaggca gccaaatttt aattagctca 360
agactncccc ccccccccaa aaaaaggcac ggaagtaata ctcctctcct cttctttgat 420
cagaatcgat gcattttttg tgcatgaccg catttccaat aataaaaggg gaaagaggac 480
ctggaaagga attaaacgtc cggtttgtcc ggggaggaaa gagttaacgg tttttttcac 540
aagggtctct gctgactccc ccggctcggt ccacaagctc tccacttgcc ccttttagga 600
agtccggtcc cgcggttcgg gtaccccctg cccctcccat attctcccgt ctagcacctt 660
tgatttctcc caaacccggc agcccgagac tgttgcaaac cggcgccaca gggcgcaaag 720
gggatttgtc tcttctgaaa cctggctgag aaattgggaa ctccgtgtgg gaggcgtggg 780
ggtgggacgg tggggtacag actggcagag agcaggcaac ctccctctcg ccctagccca 840
gctctggaac aggcagacac atctcagggc taaacagacg cctcccgcac ggggccccac 900
ggaagcctga gcaggcgggg caggagggcc ggtatctgct gctttggcag caaattgggg 960
gactcagtct gggtggaagg tatccaatcc agatagctgt gcatacataa tgcataatac 1020
atgactcccc ccaacaaatg caatgggagt ttattcataa cgcgctctcc aagtatacgt 1080
ggcaatgcgt tgctgggtta ttttaatcat tctaggcatc gttttcctcc ttatgcctct 1140
atcattcctc cctatctaca ctaacatccc acgctctgaa cgcgcgccca ttaataccct 1200
tctttcctcc actctccctg ggactcttga tcaaagcgcg gccctttccc cagccttagc 1260
gaggcgccct gcagcctggt acgcgcgtgg cgtggcggtg ggcgcgcagt gcgttctcgg 1320
tgtggagggc agctgttccg cctgcgatga tttatactca caggacaagg atgcggtttg 1380
tcaaacagta ctgctacgga ggagcagcag agaaagggag agggtttgag agggagcaaa 1440
agaaaatggt aggcgcgcgt agttaattca tgcggctctc ttactctgtt tacatcctag 1500
agctagagtg ctcggctgcc cggctgagtc tcctccccac cttccccacc ctccccaccc 1560
tccccataag cgcccctccc gggttcccaa agcagagggc gtgggggaaa agaaaaaaga 1620
tcctctctcg ctaatctccg cccaccggcc ctttataatg cgagggtctg gacggctgag 1680
gacccccgag ctgtgctgct cgcggccgcc accgccgggc cccggccgtc cctggctccc 1740
ctcctgcctc gagaagggca gggcttctca gaggcttggc gggaaaaaga acggagggag 1800
ggatcgcgct gagtataaaa gccggttttc ggggctttat ctaactcgct gtagtaattc 1860
cagcgagagg cagagggagc gagcgggcgg ccggctaggg tggaagagcc gggcgagcag 1920
agctgcgctg tgggcgtcct gggaagggag atccggagcg aatagggggc ttcgcctctg 1980
gcccagccct cccgctgatc ccccaaccag cggtccgcaa cccttgtcgc atccacgaaa 2040
ctttgtccat agcagcgggc ggacactttg cactggaact tacaacaccc gagcaaagac 2100
gcgactctcc cgacgcgggg aggctattct gcccatttgg ggacacttcc ccgccgctgc 2160
caggacccgc ttctctgaaa ggctctcctt gcagctgctt agacgctgga tttttttcgg 2220
gtggaaaacc aggtaagcac cgaagtccac ttgcctttta atttattttt ttatcacttt 2280
aatgctgaga tgagtcgaat gcctaaatag ggtgtctttt ctcccattcc tgcgctattg 2340
acacttttct cagagtagtt atggtaattg gggctggggt ggggggtaat ccagaactgg 2400
atcggggtaa agtgacttgt caagatggca gaggagaagg cagagggaaa acgggaatgg 2460
tttttaagac taccctttcg agatttctgc cttatgaata tattcacgct gactcccggc 2520
cggtcggaca ttcctgcttt attgtgttaa ttgctctctg cgttttgggg ggctgggggt 2580

tgctttgcgg tgggcagaaa gccccttgca tcctgagctc cttggagtag ggaccgcata 2640
tcgcctgtgt gagccagatc gctccgcagc cgctgacttg tccccgtctc cgggagggca 2700
tttaaatttc ggctcaccgc atttctgaca gccggagacg gacactgcgg cgcgtcccgc 2760
ccgcctgtcc ccgcggcgat tgcaacccgc cctgatcctt ttaagaagtt ggcatttggc 2820
tttttaaaaa acaataatac aatttaaaac ctgggtctct agaggtgtta ggacgtggtg 2880
ttgggtaggc g 2891

<210> 36
<211> 1092
<212> DNA
<213> Homo Sapiens

<400> 36

aagcttcccc ttcatcatcc aagaaggcat tcaggtcttt ctgtgctagg ccccaggtaa 60
agtgctggac tacccagtaa ttgggttcag tagcaggatg gcctcagatt gaggtcccag 120
ggccaaagga ccactcctct cctcagcgct ggtccgggaa aggcaagctc cgggcgggag 180
cgcacgccgc gcccccgaag cctggctccc tcgccacgcc cacttcctgc ccccatcccg 240
cgcctttcca ggtcttctcc cggtgaaccg gatgctctgt cagtctccta ctctgcgtcc 300
tcggccgcgg cccgggtccc tcgcaaagcc gctgccatcc cggagggccc agccagcggg 360
ctcccggagg ctggccgggc aggcgtggtg cgcggtagga gctgggcgcg cacggctacc 420
gcgcgtggag gagacactgc cctgccgcga tgggggcccg gggcgctcct tcacgccgta 480
ggcaagcggg gcggcggctg cggtacctgc ccaccgggag ctttcccttc cttctcctgc 540
tgctgctgct ctgcatccag ctcgggggag gacagaagaa aaaggaggta gaatggatcc 600
ccttggcctt cccctgtggt cgggggcggg ccagggtggg ccgcgttgcc caggcagccc 660
tgccgtgttg ctaggcagcc tggtcgccgg cgtgggcgat gccggcgctg gggcgggagc 720
cgcgagggtg ggaggccctg gggcgtttcc gggacgtgga gttagcaggg ttctgacttg 780
aaaaacgacg gcaaagcgtg ttcttgactg cttctgagca cctcacacct ttcagaccca 840
gggcgccttt attcccagct ggaagcccag cttagagcaa tggtgccact aaaaggggtg 900
tgttggatgt gaaaataccc tttggaagta tttataagcc tgcaggaaat atgttttcct 960
tattttctta ctctgctccc ttcattaccc atttcaagaa gcaacagaac ctgtgcagag 1020
tgtgttttaa gttacactgt atgtttattt ttgtttatgt tgaactcggt gtatacttgt 1080
gagaataagc tt 1092

<210> 37
<211> 5203
<212> DNA
<213> Homo Sapiens

<400> 37

agggaaccca acgcccctcc ggggcagaag ggggcagcgc gcgcgaggcg gaaggacagt 60
cactctcgct gcacaaactt tcccgcgggc cgcgccgcgc gtgcaggggc tggacgccac 120
ccggcgccag agccgggcct gaggagcggg gtctggccgg gaacgagggc tgccagcctg 180
ggtggtgtca tttgttgctt catttttcat tccctttcct ctcgtgagga atcagaggca 240
tcgggccgcg cgggctgaga attcttaaat tctccccggc cgcattcggc tgagccaacc 300
cgcagttcct gtcagcccct ctgtcccccg ggcagcggtt ttccccgcta gccaggtttg 360
gaagtcaccc tctgtgagac tgggttagga agtgacgaaa agcgccgaat tgttttcaaa 420
ttgaaaatac tttttttttt ttttttggag atagcgctga caaatatatg ggatcccggc 480
ttttgatccc tggctgccgc ctctgttctc ctgtcgctaa taaaactcgc attgagcccc 540
ttagtgcctt gattaacagg cagattaact cttactgggg ttgggaacag cttgtcctaa 600
tcaatgtcat tttaaaagcc cgtcaggatg cgcttagctt cggttccctg gatgcttgag 660
cgagccagcc atgacatttg ctccctcccc ctccccagcc tcaaatcccc cagtaaagcc 720
cccagttccc gcctgcctgt tcccaggtct acaacgggac aaatcgcagg accgggtcgt 780
tggcctttag aataggcagc ggtttagaga ggggatatcc agggagaagg gactgaggtg 840
gccttgactt gcttgctttt cctttgaagc tgaactcttg cctcttgggg gcagaggtta 900
cagggaaaag agagatgggg ggatcgtcac agggtgatga aatggaaagc aaacttttct 960
aggaacccta tttgcaaaaa tcggagagtg ccgctgattt cagttgtcct acatgtcagg 1020
agttttaaac ctaactccta tctggacaac agcatggagg gactgggagg caaagccttg 1080
ggagtctgac ccgctgggct gctaggaatc cctttgctgg gagactccac tggacaatgt 1140
tattttaaag gtattttggg ggtgggcagg gagaagacag agagagaaag agagagagag 1200
agagagagag agagagagag agagagacag gctggcctcc cagagcgaac ggaagaggac 1260
tgaggactga acccagggag cccctagcac tggccatttg gcaggaggcg aggaagggcg 1320

agggcaccgc ggcttccccc caagccgagc agtggccaca gcctgaaggt gccacggggc 1380
ggctctggga tcttcttggc ttttctaggc tctgggctcg gcgaggctgc cgggttagcg 1440
ggaaccggca ggttgctgga gaaaggagag ggacaaaggc ctggaagtgg gggaggcacc 1500
agggactggg cggccgggat gggagccttt gcctttaggg ctcactggac ccgtttgcca 1560
gctccgcgga caagggatcc caccttccca gggtctgcaa aaagagagac gtttggggca 1620
ctacaaagtg acgcaggtgt ctgccaagac tttccttctc caaaaaatgc cagtcttcac 1680
cctttccagc gcgccggcgc tgcgcctctg cttgaccggg gaaagagaga gtttggaggt 1740
gtcagaactg tcagaactcc ggatgggagg ctcttggttc tgaagctggg agctggaggc 1800
acttcccccc agaagtgggc acttctttgc ctccccgaag gcctgatggg gtgggcacag 1860
gggaatgcca gggatcggtg cgggtgagat gcttgcctca cctcatgttg acgcatcaat 1920
actggctaga agctctgggc aggctctggc gagccacggt cccccagcgg gactccctcc 1980
acgcagacag ccccgctacc gggtgggacg accccggcgg cccccgcggg aacgcacccg 2040
ccggcctgag aggtcctccc tctggccaca gggggcggag ggtagggggg aacaggggcc 2100
tgtggggagg cggggaaagg agacagaacc taaagcgcaa tccccgccgc ccccccgcgt 2160
cttggagggc agcaagtggg cctcgggcgt gttggtagcc gcaattaaaa tggcgacctg 2220
cgagttgtct cggttttgta cacacctctg gcaaccgcgc ccgcacctca gtctcggccc 2280
ccacacccct aggccgcttc gcccccagag ccggtacttg gcagagggag tgaggctccc 2340
cacgaggtag aacagcaccc ccaccttcct tgagaccagt ccagagagaa cgaggaaatc 2400
tcactctcac ccctctctcc ttaaactttg aaaactcggc ctcattctgg ggttgaactc 2460
ccctccccaa atattgacac atgggaagca gccaagtcct tttgtttttt tgtttgtgcg 2520
gaggagtttc caggagtctt tcccggagcc cccttcccca cccaatgacg tcagtggcat 2580
tcactcctcg ctccttgcct tttctgattt tttgttaaaa cttttctccg cagcgagatg 2640
ggggttggag gggtgggaga aggagctgat tcaaagagcc agtgccctgg ggggaaatag 2700
caacaagatt acggctgctt ctttccgagg caaagcttaa gaaggtgcta atccactcgg 2760
tcgctaattg agaggttgta aaaatgatac tcgcctccag ttcggacaga atcacttccc 2820
ccccccccgc ccccccgcaa gtccctcgct catgcacagc ggacttgagt cttgagtcca 2880
agcttcttta atgctcgctt ggaaagtaaa aggagagaga aagagaaagc cgactttcgc 2940
tggtctggga ctcagggccg cggccccact tgccggcatc ctccgaaagt cgcgctcctg 3000
cgaaatgaaa cctcgcccag gaggccgcgg acctggacac ccggcgccac ctccttcacc 3060
tctgacccag gtttcctccc ggcgctgcga gctcccgggg aagggttaga gccggcagcc 3120
ctccccagcc cggggagggg agagggttat gcgaccccac ctctggctag ggccggggag 3180
gcctttgctt cccgggagcc ctgcccgggc tccttggtcg cagggctgct gggtcccagg 3240
caggaacgag agggtgaggc ccacatgtgg cccggcggcc cagggcggct tgcagcgtcc 3300
tcactgtccc ggctgccagg ggctgcggcg acgcggccag tcagcagcga gttcaggtcg 3360
cgcagatttt attgatgagc tctgactttc agcactttcc ctaagtcaag aagagtctag 3420
cgtacccttc ggctgcttca tttcagcctc cctgcctcag ctcttcagcc ctattccccc 3480
tcgccctgtc ctggggtgtg tacagcagcc caggccttcc ttctccttcc cggctccgtg 3540
gcccgaagcc gccgagagag ctcgggacag cgcaggacca ggcagccgct cgctctcctg 3600
tcaccttaac tgcaggctcc gaggggcgcc tttggagtgt actgaggtgt gtcctaatcg 3660
tgcggcattc aacaaatgga cttctggtgt gtggtcagaa gagaaaagcc atttacttac 3720
tttcctcccc ggttttctgg caacagctga aggggagctg cctccgtgga ctgagcagac 3780
ccaggagagg gagtcgtggt gcggagacac acgcaccaca cacagatgac cggtggcaca 3840
cacgacacac gctgacatac cgacatcgcc agtgggacac acacacacac acacacacac 3900
acacacacac acacagagag agagagagaa tccctcccag cattggtcat ccgccccccc 3960
acccaggctt ccactccccc tcccctctta tctcccctgg cttcccctcc tctcgggcgc 4020
tgcgaaaagc agccgcactt agtcaacaaa tggcacgtgg gagaagttgg tgagtgtttg 4080
gtgaggactc ttcagggctt ttcacaagaa ccctctgtac acaaagtaag tggcgtgttt 4140
actcgggcct ctccagccag agctgtgcct ctgctccgct gcgcaccgcg gcttccgaaa 4200
ggagaaagga gagaagaaag ggcggggaga gcggggtgga ggatttggac aggccctgga 4260
ggcttgggct ggggaggcct ctggcctcgt ttagttctcg gcccggcaac ctcctctcgg 4320
cctaggcttc gccgcggcct ccgcagctgg aatggagctg ccaggaccca gtgacgctcc 4380
cgcccctttc ctcttcttcc aaggggccag gtgggctggg gtgcggccgc cgctgtgctc 4440
tgtgtcttgg ggccccggct gggatggggt gggggcgggc gggggcgggg cggcaggcca 4500
cgctgtcctg gagttggcaa gaaaggacag cacagaaact tgcaccctcc gaggactggg 4560
agtcccgagt ccagcttagg gggagtgggg gcgcgacccc caacccagaa accttcactt 4620
gaccgctcaa gttcgcggca gcagggcggg ccgcgccgaa tctcggcgtg cgcggagcgg 4680
ggagatgcag gcgagcgcca gagcccgggc tcgggggccc tgcgccgggg agaggagccg 4740
ggacccaccg gcggagccga aaacaagtgt attcatattc aaacaaacgg accaattgca 4800
ccaggcgggg agagggagca tccaatcggc tggcgcgagg ccccggcgct gctttgcata 4860
aagcaatatt ttgtgtgaga gcgagcggtg catttgcatg ttgcggagtg attagtgggt 4920
ttgaaaaggg aaccgtggct cggcctcatt tcccgctctg gttcaggcgc aggaggaagt 4980
gttttgctgg aggatgatga cagaggtcag gcttcgctaa tgggccagtg aggagcggtg 5040
gaggcgaggc cgggcgccgg cacacacaca ttaacacact tgagccatca ccaatcagca 5100

taggtgtgct ggctgcagcc acttccctca cccacactct ttatctctca ctctccagcc 5160
gctgacagcc cattttattg tcaatctctg tctccttccc agg 5203

<210> 38
<211> 8085
<212> DNA
<213> Homo Sapiens

<220>
<221> unsure
<222> (2545)
<223> unknown base

<400> 38

gctccatgac ctaatcactc cccaacacct cctcctacta agactatcac attggtgatt 60
gggttttgac acacagattt ggggagaaca caaacactca tatcacagca cctactaatt 120
tatccagcca tttcactgct aggtatttac ccagaaaaaa tgaaaacaca tgttcataca 180
aagacttgta caacattttc atagtagctc tatttataat accaaaaaaa ttggaaacaa 240
ctcagatatt tatccacagt taaatggata aacaaactat agtatataaa taaaaagaat 300
aaaaagaatt aactattgat acatactata atttagctga atctcaaaat aattatgctg 360
agtgagagaa accaaatgaa aaaaagatac actatattat atacaattct aagaaataaa 420
acctaattta tttggcaccc agatcttcat ttctatacca gtttcaaata aaaagtctca 480
aacttccttg gataaatggc tgattctacg actggggcag gaaatataca agatgagtct 540
gatgagtctg tagcatcttg taataccaga aagtaggaaa gtccacacat acacacacat 600
acacacaata cagagagtgg gtcaaggagc acaagagtca actgaaaaag cccccagtgc 660
caaaaactgg aaaaatttga gcaacaaagt attcatgaat ccatacaaag aaaagactga 720
ataaagaagt aaatggggga gaagaagcaa atcttcctca cagaattcca aataaattat 780
gtagatactc cactccaaag gagggctagc ataactccct actcccccac acttccttaa 840
gtgactcata atgatttcct tccaaagagt acagagtatg caaagaggga aaaaagagta 900
actatgcaat ggagaaacct gacaaacact acttcagcca ggtgatggag aacaacatta 960
acagtcataa gtcatgttga caatatgtac ccttcatatg atgtaataaa tacagtacat 1020
tacctctttg atttccaccc cgccaaaacc caaaacccta gtctactcat gaggatacat 1080
caaatcccaa tagaagggca tcttgcaata tacctcacca gtactcctaa ggactgtcaa 1140
ggtcatcaaa tacaaggaaa atctgagaaa ctctcacagc caagaggagc acaagacatc 1200
ataacaatta aatgtggtgt cctggatgga atcctggaaa ggaaaaagga cattaagtga 1260
aaatgaagaa aatctaaata aactatgaat attagttaat aacaatttat cagtattagt 1320
tcattaattg taacaaatgt accctattaa tttaaaatgc taacagagaa aattgtacaa 1380
atgatatatg agcattctct atattatctg ctcaattttt ctgtaaatcc aaaatcgttt 1440
cccaaaagta aaatctatta ataaaacatc taaagcaaac agacctaaaa ggcatatgca 1500
aatgtataat ttaatgttta ataaatcaga tataaatatt tttaaaaacc taatatttgg 1560
tgacagaaag cagattagtg gcttgccaga aagtggtgat tagtactagg ggaaggggat 1620
gggacagaac agtatgaagg gatttaaaaa atggcatgag gaaacttttg gaggcaaagg 1680
gtatgtttat cattttagtt tcacaggtct aacaacaaat tatacaattt aaatatatgc 1740
agcttattat atgacaacta tatcttgata aagtggtttt tttccccaaa ctatagaaaa 1800
aatacttcca catttaaatc ctcttctcca aaaacagtct tcaggaatat ttgaagttag 1860
ccccatcaca cctgaattta atcagtgaat tttgttcatt caaagattca tacatttggg 1920
aagttcctgt gccaaaaatg ctaatcaccc agcatcaaaa tttatggaat aatggtgatt 1980
taggacatgc tggacatctt tattgttgtt ttcccacttc aagattttca atcttttgga 2040
aacaacctcc ccagacattc tgacaaggga attattataa ccaaccaaaa tcaagccaaa 2100
tatagtttgt cagatttaaa atgaggccaa gtgccaattt tacagcagcg accggaaaat 2160
caaattatat ggcatctgac atttaaaatc acactcctaa gaaggctgtc atttgatgtc 2220
ttgggtttgg gttacagtac ttaactttat aatactgggt aaccttgtgc ctcagttttc 2280
ccaactatga aattgggata acagtaccta ttttgtgaga ctgttgaagg attacagtta 2340
acacaggtaa aaggcttaca agactacctg gcaatgctgt cctgatttta caaattctat 2400
acactgtgtg gcattagcca tcaggaaaat ataaataaat cagtgagata ccacttcaca 2460
cacttggggg gctataacaa aaaagacaga taaaaaatgt tgtcaaagat gtgaagaaac 2520
taggaccctt attcactgct ggtgnaaatg taagaagtac aatcatttga accatagtct 2580
ggcagttact ccaaagacca cacctagagt tgccatatga accagcaatt ccactcctat 2640
gcatatatcc aggagaaagg aaaatatacg ctgcacaaaa acttgtacat gaatgttcat 2700
tagcaggatt tttcatggta gccaaaaggt gtaaacaacc caaatgtcca acaactgata 2760
gagatggata aataaaatgt ggtttatcca tacaatgaaa tattatttgt caataaaaag 2820
gagtgaagga gtgaagtact gattcatgtg gaacatggat gaaccttgat ccaaaatagc 2880

caaatcacag gtacaaaaag catattagtg gctgctggga gatttaggcg gaaatgtgga 2940
ataactgcta gtgggtattg agattttaga gtcatactca tgttacaaaa ttaatagtgc 3000
tgatggttgc acaactctga gtacatgaaa aatcaatgaa ctgatacttt gagtgagctg 3060
tatgatactg gaattacacc tcaataaagc atggtaactg ttttaagata ggctggaaag 3120
agaaagcctg aaaacaacaa taatgatatt aataaattag tttacttctc tagtctcata 3180
tacttctgtg cccacacttg ctcctgttct attcataatg gtccccttgc agttgccata 3240
ttatatcctg ccatttgatg cccggtgaac attctatacc tgcttcccag aattctcttt 3300
acctttcctc tatctgccta acttccacat atctaaaatt aatcagagta aactatttac 3360
tagaacaacc aactccaaat cctagtaacc taacatgata aaggtttgtt tctcactcat 3420
atagcccctc cccagatgat cgaggggtcc aggctcctta cctctagtgg ctcccccacc 3480
ttctggagtc ttctgcattc tttatacatg gttgagataa actatgagtc attagcacag 3540
ctagaccttg aggtcctaca agaaaatttg caaatcattc actctgtttt gaacaaggta 3600
tatttaagat gatgttaaaa tacccaatgg tcttgggtca aatacagttt atgactgtgt 3660
atctaaaata tatattgcaa tattcttccc tttttctact gacttcatga atttagcggg 3720
gatccatttt ataagctcaa agataattac ttttcagact aagaatattt agggtaaaaa 3780
gtactgttca acatctctac tgaggatgtt atgatgtagc acactgtata agctggagct 3840
aaaggaaact ttccttaaag tgctatttac taaaaattgg aacacattcc ttaagacaaa 3900
tcgaagtgtg gcacacaaca tccaaacttc catcatagat acagaggtgt taccatctcc 3960
cactcccaaa tttctttgtc acgctgagga tactcaagag gagcaggaca tgttggtcgc 4020
agcaggagaa acttgaaagc attcactttt atggaactca taagggagag aatttcttat 4080
tttagtatcg tccttgatac atttattatt ttaaaagata atgtagccaa atgtcttcct 4140
ctgtgttaaa tctttacaaa actgaaatct taaaatggtg acaaaaattc tacttctgat 4200
agaatctatt catttttcca attagatagg gcataattct taatttgcaa aacaaaacgt 4260
aatatgctta tgaggttcca tcccaaagaa cctgctattg agagtagcat tcagaataac 4320
gggtggaaat gccaactcca gagtttcaga tcctaccggt aattggggta gggaggggct 4380
ttgggcgggg cctccctaga ggaggaggcg ttgttagaaa gctgtctggc cagtccacag 4440
ctgtcactaa tcggggtaag ccttgttgta tttgtgcgtg tgggtggcat tctcaatgag 4500
aactagcttc acttgtcatt tgagtgaaat ctacaacccg aggcggctag tgctcccgca 4560
ctactgggat ctgagatctt cggagatgac tgtcgcccgc agtacggagc cagcagaagt 4620
ccgacccttc ctgggaatgg gctgtaccga gaggtccgac tagccccagg gttttagtga 4680
gggggcagtg gaactcagcg agggactgag agcttcacag catgcacgag tttgatgcca 4740
gagaaaaagt cgggagataa aggagccgcg tgtcactaaa ttgccgtcgc agccgcagcc 4800
actcaagtgc cggacttgtg agtactctgc gtctccagtc ctcggacaga agttggagaa 4860
ctctcttgga gaactccccg agttaggaga cgagatctcc taacaattac tactttttct 4920
tgcgctcccc acttgccgct cgctgggaca aacgacagcc acagttcccc tgacgacagg 4980
atggaggcca agggcaggag ctgaccagcg ccgccctccc ccgcccccga cccaggaggt 5040
ggagatccct ccggtccagc cacattcaac acccactttc tcctccctct gcccctatat 5100
tcccgaaacc ccctcctcct tcccttttcc ctcctccctg gagacggggg aggagaaaag 5160
gggagtccag tcgtcatgac tgagctgaag gcaaagggtc cccgggctcc ccacgtggcg 5220
ggcggcccgc cctcccccga ggtcggatcc ccactgctgt gtcgcccagc cgcaggtccg 5280
ttcccgggga gccagacctc ggacaccttg cctgaagttt cggccatacc tatctccctg 5340
gacgggctac tcttccctcg gccctgccag ggacaggacc cctccgacga aaagacgcag 5400
gaccagcagt cgctgtcgga cgtggagggc gcatattcca gagctgaagc tacaaggggt 5460
gctggaggca gcagttctag tcccccagaa aaggacagcg gactgctgga cagtgtcttg 5520
gacactctgt tggcgccctc aggtcccggg cagagccaac ccagccctcc cgcctgcgag 5580
gtcaccagct cttggtgcct gtttggcccc gaacttcccg aagatccacc ggctgccccc 5640
gccacccagc gggtgttgtc cccgctcatg agccggtccg ggtgcaaggt tggagacagc 5700
tccgggacgg cagctgccca taaagtgctg ccccggggcc tgtcaccagc ccggcagctg 5760
ctgctcccgg cctctgagag ccctcactgg tccggggccc cagtgaagcc gtctccgcag 5820
gccgctgcgg tggaggttga ggaggaggat ggctctgagt ccgaggagtc tgcgggtccg 5880
cttctgaagg gcaaacctcg ggctctgggt ggcgcggcgg ctggaggagg agccgcggct 5940
gtcccgccgg ggcggcagca ggaggcgtcg ccctggtccc caaggaagat tcccgcttct 6000
cagcgcccag ggtcgccctg gtggagcagg acgcgccgat cgtccgggac gtccccgctg 6060
gccaccacgg tgatggattt catccacgtg cctatcctgc ctctcaatca cgccttattg 6120
gcagcccgca ctcggcagct gctggaagac gaaagttacg acggcggggc cggggctgcc 6180
agcgcctttg ccccgccgcg gagttcaccc tgtgcctcgt ccaccccggt cgctgtaggc 6240
gacttccccg actgcgcgta cccgcccgac gccgagccca aggacgacgc gtaccctctc 6300
tatagcgact tccagccgcc cgctctaaag ataaaggagg aggaggaagg cgcggaggcc 6360
tccgcgcgct ccccgcgttc ctaccttgtg gccggtgcca accccgcagc cttcccggat 6420
ttcccgttgg ggccaccgcc cccgctgccg ccgcgagcga ccccatccag acccggggaa 6480
gcggcggtga cggccgcacc cgccagtgcc tcagtctcgt ctgcgtcctc ctcggggtcg 6540
accctggagt gcatcctgta caaagcggag ggcgcgccgc cccagcaggg cccgttcgcg 6600
ccgccgccct gcaaggcgcc gggcgcgagc ggctgcctgc tcccgcggga cggcctgccc 6660

tccacctccg cctctgccgc cgccgccggg gcggcccccg cgctctaccc tgcactcggc 6720
ctcaacgggc tcccgcagct cggctaccag gccgccgtgc tcaaggaggg cctgccgcag 6780
gtctacccgc cctatctcaa ctacctgagg tgaggcccgg ggacggggca cgcccagcgc 6840
gtccgggagt agcggttccg ttggcggcgg cggccgccaa ccctcagccc cagccccagc 6900
gcaccgctgc gctccccggg gcggccggag agggtgggca gcgggacaca gcacaggggc 6960
agttgcctcc cttcttcttc cctcctctcc tcactcttgg ggacacgaag gtgggcgcag 7020
aatatactat ttttggggcg tgcctccctg aaagctgttt ttttgtttgt tttttaactt 7080
tccgaatctt ccagattccg aagcagaacc aaccccgatt taaaacgtgc agcgtcacac 7140
taggtccgct gtagcccagt ggggcagaaa gtgcgcggcg agttgggggc tttatgaaat 7200
gcttctttct tagaagaagg acgtttacca ggagtgcttg tcttggagag gagttaaggc 7260
accgttcccc cgggaggggt gggacttgag aggtggccgg ccagaaccga aagcagcacc 7320
atcttaggga tttgaacact tcagtggctc agttttctta agaatctcaa gattaaaatt 7380
aagttcacgt gggaaatgtt taaactgtgg atttaaacgc ctgtcactgc attgcaccgt 7440
tttcttatta ttgcttgcta ttcactacaa ttttttttat atacaggttt aaaaaacact 7500
actttgcata ctgaagtaat ggaatgtaaa aaaagaatgc tctgtttgga atcttatgtt 7560
gtgaataggc aaaacagtgt cagtgtattg gacaatactt taaaatgaca aacatatact 7620
tgcttaagta agcaatgatt acagggttgt gttttaaaaa ctcaaaacca aaacattgca 7680
aagtaccatc gaacttttaa agccaaacca tatttgtttt gacccagcat acagacagga 7740
aggacataac atttcatttg tcaaagacta aattgtttct atataaagag ttttgtagaa 7800
agatttcctt ttaaccgact ttaactttct aggacataat attatacact aattattgtt 7860
cttttatatt ggtgctactg atgaatggct aatcatttgc aagtatggtg aatccagtta 7920
cggatagtct attaccaagt ttagtttgca tgtctttcaa gtgtatatat acagttctgt 7980
ttttaaaatc tcctttcacc ctgttaatac tggtttaaga aacctttagt attagatagt 8040
ggtgcactta aaaataaatg gagtactttg ttttgcattt caagg 8085

<210> 39
<211> 7110
<212> DNA
<213> Homo Sapiens

<400> 39

tgttttttgt gatgccttag aggccagaag gatgttttga aggggaaaat tgtaggagtt 60
aggcagagca gggttttggc tccttctcaa tgtccctcta cctagacaaa ttcgggctat 120
tctgcagagg ttgcagtgag ccatgatcac gtcactgcac tccagcctga ggaacagagg 180
gagactccgt ctcaaaaaaa aaaaaaaaaa aaaaaaaaaa aaaaaaaaaa atttcggcct 240
actctatgaa gcacctggta ggtgtcccaa tattttctgg tatatgggaa ttaccttttt 300
ctattattgg aaggtctaaa aagcaaacaa tgtgctccat atggctagag ttagtattaa 360
ggggaccaag tagaataatt ggtagctagt gttagcctgg agactaacat agtcaaagct 420
gtcttgtcat gttatgtctt ttttatttgt attgctccac ggtccaagac aaaatttctg 480
aacgccactt ggacacagtg agtacctggt ctgctctatt gttctcagga gcaaccaact 540
caacccttat ttctctgaga atgatgattt catacagcac atctctctac caagatgtga 600
aagatgacac catggcatct gaaatagctt caggagagat ttgggacatg ggaagcttgt 660
agacaataat ggaaaattct cttttagaat atagttactt gtatgaccca cagtagcgcc 720
ttttggagaa tgtcttaaaa ttatctttat tgaaagaaca ataatgtttg ttattaggat 780
aaatgaaata aggggaaaac ctcagaccct tggaacaatg ggtttacatt caatccaatg 840
attattatgt ttttatattc tgtattattt agaaaacagt agttaaacag acagaaatac 900
agaagattgt ttaaaaatta aagctattga gttagatccc tttttgaaag gtcagcgtat 960
gggagatgag aaaggcacta tagagatcag agtgtttaca caaaagacat cttagcagat 1020
gacctacaaa gagcacatca agtatttata tcatccacat caagttgctg gtcatttcgc 1080
ttatcaaaga aaataagaaa gaaattttct ttcgacatta ttttggtgta gtaacaatag 1140
agttttggaa tcagctgtta gaagcgataa ttaaagctag tttaccatgc ttactaatca 1200
atctacatag tcaccctgaa gctttatata attgtccctt tgttacaagc ttctacttct 1260
ccctatggta ttctggttct gaatccagac aggtaaagaa ctaagtatgg ccgggcgcgg 1320
tggctcacgc ctgtaatccc agcactttgg gaggccgtgg cgggcagatc acgaggtcag 1380
gagatcgaga ccatcctgac caacatggtg aaaccctgtc tttactaaac tacaaaaaat 1440
tagcccggca tgatggtgtg cgcctgtagt cccagctact cgggaggctg aggcagggga 1500
attgcttgaa cctgggaggc agaggttgca gtgaaccgag atcatgccac tgcactccag 1560
cctgtgagac tccgtctcaa aaaaaaaaaa aaaaaaagaa aaaaaaaaaa agaactgaag 1620
tgcttggaga ccagtgtatt gtaatatcta tacagccaag caggtatttt caactgaact 1680
ttaaaaaagt actatttttg aaactttctt tagtttagat agggccagcc atttgggtag 1740
aaaggaaaag aaaaacaaaa aattggaaaa ggatgcatca gtatttcttt cagaaacaac 1800
ttaacactag gaacagaagg aggattttat tcaccaactc ctaataacca cctgatgttg 1860

ctgttgggct ggccaaatat ctgccaacat tgatgatcct ttcattcacc actagagaga 1920
agtagaggga cctgatgttt agagaagcag taaaacccat ttcccatttt ctttcttcag 1980
ccggatggaa gtacttggag gctggtctca tattctagca ctttagtgct ggtgatgcag 2040
attatctctg ctccagagta actctgtgcc atggaatcca agaggattct gtcgcaccag 2100
gacaggaatg cacccttgtt tcatttgctt ccatgaaagc agatttcaga gattctcaga 2160
tgctatgaga ctaattgtca cttagggtag gctccagggg agggacagag aagggcagtg 2220
ctaataacag ggactctgga gacaagatca gcaatctaag aaacaaatgt gagacatttt 2280
acccctccct aatctactcc ccttttaaaa gttcatcttc attttccctt tttctaccac 2340
gcagtaattg ataattaaac acagctctgg tttttaccat gcaatctagt agaacaccta 2400
gacatcagga actccttatc tttggttatt tatacaataa tgaaatctgt agttcacgct 2460
aggctgtgtg gacagtatct gcatctctgg aaatcaactg gaggcctttc tcttactctt 2520
ttccggttgc tttccatttc cttttccact gtggtccttg cctttcccaa gctgaccaga 2580
actatgcaca aaaccagtct tcaataaata tcaacactga agcttcagca ggttgttaac 2640
tcctgcacag ctatcttaaa tgctgtatat aagtgcaaag ttttatggag cctcagaggt 2700
ctctaagaac actgtaaata tcccaggttt aattagtagt cccggagtta ggtaatggcc 2760
tgtggctttc tgcgctaggt ctcttgaggt ttctagtctt cagaggttgc ctgcaactat 2820
gatgtgcaca tatacagtac tctttctcaa atacagcata aaccctcttt agactttgct 2880
aggcacttac aattattgaa cacacatgca gattgattct cattctctca aagtttgaaa 2940
aacaactcag tgcttcaacc taggtctcgt tagatatttt ttgactcaaa ttgtcgtctg 3000
tagttctact tcctaaggga aatgaaaaaa caataaattc ccagactggt gttgatgctc 3060
attctcttta agcgggtcga ctacttgctt tgtagatcct tgacgggtgg ggtgcggggt 3120
aggagtgcga tccaactctc agcatttccg aatcagctct ctcacggtga caggtcagcc 3180
caatcggggc tgtaaacaga cttgacaggt ttgttctggg ctgacggcca ttgactaggt 3240
tctcagacca gataagtcac ttggctgagt ccacagtagg tggggcgcgc tcaccagctc 3300
aggggtagtg actggacgtt tgttgcaaca tcggagaatg cacgctctgg gctgcagcag 3360
gagataccct caagcacaga accaaaaggg ttcaccctaa gcggcagggc atcagcgatg 3420
gagaggcccg agagccctag cgcccagctc cttttcccac gtttgggaag gcgcagaata 3480
ggtcgatgta gagcaaggag tgagtctcag gtctcagtcc tttggcttgc tcttagggta 3540
gcaggcgagg agtggcacca gtttggggac tctctccccg cgttctgtaa gaatcggcgg 3600
cagccagcag gcggggaggc gggggcacgt gtttggatgt gggtgcttgt gtaaccagtt 3660
ccccaagcgc cagccccgac agcgctcctt cgggaggctg gtccgagccc ctgtttccgc 3720
cgcggcgcag gaagggttgg ggttccgctg cctgcaccag gcaagagcac cccgagcaaa 3780
ggaagaagac gacttgcctc cggagctatc actggggagt gggaatttgg aaagttcccc 3840
aactagggac acacgtgacc tccttcggaa agtagttccg actgtggccc gtgtatcctt 3900
ccacctcctt ttgaaccctc ctaggtctcc tcgccccgcc cactcgctgg gctgcagctt 3960
cctaccgttc cgtactttcc actcaacccg gtaaccccaa acgtgcacgg tccggccggg 4020
gcgcgcggag cctggccccg ggcgatccat cctgccgggt tttcacggcg gccaaggggg 4080
ggcggggcta ggtggtctct gagaaccgag cttgactccg acgccgcgaa ccgacctgga 4140
gcccgagggg aaagatgctc gactctcttg ggggcaccgg agcgggcgca ggagaggcct 4200
gcggggtgcg tcccactcac agggatcctc tttcagttca tttagatagg tgccctttgg 4260
gcccttgaaa ttcaacggct atgtgttcac gttcagcacg ctcggctgag agctttcatt 4320
tttagggcaa acgagccgag ttaccgggga agcgagaggt ggggcgctgc aagggagccg 4380
gatgaggtga tacacgctgg cgacacaata gcaggttgct ctttgtgcta agactgacac 4440
catgaggaca cagatttggg ggaaggggga atctctaggc aaaggctgtt acagtcaaat 4500
ctctgcgaac gattgtgatc cgacagcggt gcaaaaggaa agagcgaatg cagtccacgc 4560
cgcggaaatc taggggtaga ggcaaggggg gagggtattc cccttgcagg gaccgtccct 4620
gcatttccct ctacactgag cagcgtggtc acctggtcct tttcacctgt gcacaggtaa 4680
cctcagactc gagtcagtga cactgctcaa cgcacccatc tcagctttca tcatcagtcc 4740
tccacccccg ccccacaaca gcctaccctg cctccggctg ggtttctggg cagaggccga 4800
ggcttagctc gttatcctcg cctcgcgttg ctgcaaaagc cgcagcaagt gcagctgcag 4860
gctggcggct gggaaccggc ccgagcaagc cccaggcagc tacactgggc atgctcagta 4920
gagcctgcgg cttggggact ctgcgctcgc acccagagct accgctctgc cccctcctac 4980
cgccccctgc cctgccctgc cctcccctcg cccggcgcgg tcccgtccgc ctctcgctcg 5040
cctcccgcct cccctcggtc ttccgaggcg cccgggctcc cggcgcggcg gcggaggggg 5100
cgggcaggcc ggcgggcggt gatgtggcgg gactctttat gcgctgcggc aggatacgcg 5160
ctcggcgctg ggacgcgact gcgctcagtt ctctcctctc ggaagctgca gccatgatgg 5220
aagtttgaga gttgagccgc tgtgaggcga ggccgggctc aggcgaggga gatgagagac 5280
ggcggcggcc gcggcccgga gcccctctca gcgcctgtga gcagccgcgg gggcagcgcc 5340
ctcggggagc cggccggcct gcggcggcgg cagcggcggc gtttctcgcc tcctcttcgt 5400
cttttctaac cgtgcagcct cttcctcggc ttctcctgaa agggaaggtg gaagccgtgg 5460
gctcgggcgg gagccggctg aggcgcggcg gcggcggcgg cacctcccgc tcctggagcg 5520
ggggggagaa gcggcggcgg cggcggccgc ggcggctgca gctccaggga gggggtctga 5580
gtcgcctgtc accatttcca gggctgggaa cgccggagag ttggtctctc cccttctact 5640

gcctccaaca cggcggcggc ggcggcggca catccaggga cccgggccgg ttttaaacct 5700
cccgtccgcc gccgccgcac cccccgtggc ccgggctccg gaggccgccg gcggaggcag 5760
ccgttcggag gattattcgt cttctcccca ttccgctgcc gccgctgcca ggcctctggc 5820
tgctgaggag aagcaggccc agtcgctgca accatccagc agccgccgca gcagccatta 5880
cccggctgcg gtccagagcc aagcggcggc agagcgaggg gcatcagcta ccgccaagtc 5940
cagagccatt tccatcctgc agaagaagcc ccgccaccag cagcttctgc catctctctc 6000
ctcctttttc ttcagccaca ggctcccaga catgacagcc atcatcaaag agatcgttag 6060
cagaaacaaa aggagatatc aagaggatgg attcgactta gacttgacct gtatccattt 6120
ctgcggctgc tcctctttac ctttctgtca ctctcttaga acgtgggagt agacggatgc 6180
gaaaatgtcc gtagtttggg tgactataac atttaaccct ggtcaggttg ctaggtcata 6240
tattttgtgt ttcctttctg tgtattcaac ctagggtgtg tttggctaga cggaactctt 6300
gcctggttgc aagtgtcaag ccaccgattg ctttcttagg ctatctatat ggtctcttcc 6360
tgagggctat tgtccgttaa tacagaatac agtacactgt tagtggatta gcgagctcgg 6420
taatccggtc tcctaaatga acaaaaaagt agacgctttt tgaggttgag catatttcga 6480
ttaaatcttg gcttaggccc tagatcaagg gtttagatca gaataaaatg aaaattagtg 6540
ttgcacgtac gcatattgca tcagaatctt gcagtgattg ttttagtttc ctgagttgca 6600
ttgatagatt cttttaaaat atgactgatt tgcataactt tagaagcaga atcattttca 6660
gtatatatgg tgcacattga gggcaaaaag tagttttgtt aatgtttaaa cttaagttac 6720
ctacaacttt gaactgtatg tagaagtttt gtagtttgaa gtcaatagtg ccataatata 6780
ccttataagg cgttcttact agatctttgt tatatttacc tttttctctc cctatggggt 6840
gatgtaggat agtgcttgaa atttgcactt cagtagcatt taatgttcag tgctcttgtc 6900
ataaacatag aatggatatt gagtagtttc tgatcccaga tggtaatgtg taggttcaag 6960
ggtattgtgt gtagcaagtg aagattgcag aaataaaact tcagttcatg cttgaaattt 7020
aagtattgtt gtgatgccag aattgctgct caccgttttt aggtttcagg tcctctgaca 7080
ccttttggta tcgttaattt tactgatttg 7110

<210> 40
<211> 6478
<212> DNA
<213> Homo Sapiens

<400> 40

tttattttga agggatgttc aagaaatatt gctaaattta aaagtgttgc agagcaatat 60
gtatcataag accatactgg tataatagca acaaacagca cccccccaca caatcccaaa 120
aaaatataca gatctatgcc aagcaccagt taagatgcct ggcacatggt aggtattcaa 180
catttaatga atacatgtag aggactttta cttttcactt tatatctttt ggacaagact 240
ttgaattttt atgagcatgt attacttaaa tctagattta tagggagtga tggaaaatgt 300
gaaaataaaa ttttaaaaag gaaaagaaag agtaaagatc tagatttaaa aaaggaaatg 360
taaacaagat gaaatagcca aagataggtc tccccaaaac taatgttgag acaatgccac 420
cagcagtgac agtcagctaa gagatggttc gcagctcact gctcagtact gaggcattca 480
gcaggggttg tatttaactt tcaagttgga accaaccgaa actgttgggc ttgaagagct 540
gtctttatga gtcatcgacg gcagaaagca cagaagcact aaagaaaata attcagaaat 600
gtggaaagag aaaagtatgc ccttgtcttg ggagaacaat atttgtgtgt aaccaagtag 660
gaagactttg tggaggaaag tctgaaaaag cagagagctg aagagaaaag agccaaatga 720
aggacacggt ggccaacact caaagtaaaa agacattttt gaagaagatg tgtttctagg 780
gtttctaaga agtcgctata ctcatgccca gagccacctt gcaagttcat ttggaaagtg 840
cattggtgtc ttttggtata agatccacga acatcaacag tcctgttatg tgagcaggtg 900
ttcatactga agggccacaa gcaaagtttc catgtgttat tactgttaga acttcccccg 960
ctcccttcca acacaaaagc agctctgcct gctagtatgg aagccctggg aagagcctca 1020
tacttgcctc gtgctgccaa catctctgcc ttggcttccc tgctggaaca cacaagtcac 1080
tcagtactag ccatttattc ttctggcaga actgtggcaa gaggggatta acagggaaaa 1140
aaaattaaaa aacaaacaaa aagccaaaac aacatgaaag agctgggcat gtctttaaaa 1200
caatcagtgt ccacctcccc cagccctgtg cctgtgtctg cctgcacttt gcatgtctgc 1260
ccctgtggtg gggggggata tgtctgcgcc tctgaaaatc ttcttattta gacatcacaa 1320
ggtattagcc caattttgaa cacagccggc tgcagtacgg tgcacagaca cgactgctct 1380
ttcttcagaa aactctccag ttcaaacatg tttgcagagc atgattcatg gtgtttcttt 1440
ctggattgtc cagtgggtaa ttgctgtacg aaaaccagag aaacagatgt agaccctgaa 1500
ccctggctat aaatttttgt tttatgatta tgggtttcaa atgtgctgtg tcacactgac 1560
aaatatacag ttactctgaa acaaaatgga ttttactctc aaagttttgc tttttgtttt 1620
tcactttttg tttgcttgtt ggttggaaat acatttatta gatcattgat caaacacaaa 1680
gaaaatagag atgacacata tcagaatgtt attttatgcc ataacaagaa gatatttggg 1740
aaagtttctt ccaaccctgt ggctaatgtt taaaatgggt gtcagtagtt tacatttcat 1800

tacggacaaa tcggtctatt tttcactttc taccctactc attaatcttt atagtgcacg 1860
gatctgtatt taaagaatta tggcttattt atgtatcttt ccttcatttt acttaaaatg 1920
aagaatttgg agacagaatc aagttttcaa gaagtgtaag catttactga tgaagaaata 1980
attagcatgt ggctaaatca tgggtctgtt ttataaatta atacttggat taagtaatag 2040
ctctaattat tgctatcagc ataattaatt catgatgagg aatgattaat tgactagcga 2100
atattgtcat attataagaa acccatacta ttcctttcat ggggatttaa atactgatta 2160
ttcatttcaa ggcccaagtt catttctcag ttgagaactt caaggtaatt atatatttta 2220
cattttcttg cacaaagaaa gaaaacatcg agtcgcccaa cctttaccac acacagagag 2280
agacacacta atgttgttat ttttgtgcaa tgatacagta tagaggtaag aaattcggaa 2340
gcagtggtct cgctcaatga ggctgattcc atagcttaag aaatgagaat gaacagaatt 2400
ttgaggacac ccatccccat cttcacttcc ctgggaccag tgcatctcta ctctttggag 2460
agatgttaag caagaatgtg cagcagcctc ccctggataa cagatccctg tacacacata 2520
cctgtcagtg atgccatgtt tctcatctct aacttaagtt tctcctgctc tacttccaat 2580
atagctgttt cctcttctct tgtctaacaa cttttcatcc acctccactg tccggtaaaa 2640
atgaactcag tttccaagga actctaagga ccttaaataa gcatttggag ccataagcct 2700
catggaccaa gtagaaattg gcacataatg tagtgctttg tgtatcttta caactacctt 2760
aatgacagtg tgttaaatgt tttgtaaatt cctctgtgtt ctcttgctta aaatgtgtgt 2820
tttctttgta gttaaagggt ttcttacaat aagtctctgt tacaaaatga tggccatata 2880
aaagagagaa gagaggcgca aaaataacaa acacagattc agcaaaaatc tcaccccttt 2940
attgccctga acttttcaat agcaagaact gagcttgctg ggctgaaaat tgttttaatg 3000
taccttcata aaaggatctt tgacttggta agtgtgtgcg atgcatactt ttcatgttac 3060
accacaagtg ccacttagca actccactag acagggcagt gtttcagcat ggggtggggt 3120
gccccctgac aggcttttaa aaggcccgga tgccaatgca cattccaaca ctatccacaa 3180
aaaggagact ggagcagtgc tcttccctgc attgggcaag gagactctcc ctccctgcct 3240
aaccacttgc ctgccctgtt ttgtgggaga attacaagta aatgctacag aggcagtgga 3300
gaaaaaaggg tgttttaatt cctctccaga gtttccttta tttgatgtat gttgcatcct 3360
ttaaacaagt tgtgcaaaat ggctgcaggg tagattggct ctccctttta aagctctcca 3420
tccggctggg tttatttgta aatactgcat ctatccttct tagtgtttta ggactggctg 3480
gaaagactct tcttcctgta ggttgggtca gtgtgagaga tctaaaaaat cattttccct 3540
taaaattact gtattttaat aaaaggattg ggcaggggct ggaatgagag aaaactggtc 3600
cttcaaaatg taaaactgtc atacttaaac cagtttacaa aatatgcgtt taattatgtg 3660
gtgggatgtg tgtaggtgta tgatgagaga ggcaaccaac atggctattt ggggtgcaag 3720
gatgtgggaa caggcaagta attttcacat tggactttca tcctagggag ctgggttcta 3780
gtcacagctc tgagctgtgt gaccttgggt aggtctcatc tccccggggt tttgtttcac 3840
cagttgaaca gtatgaggat gagtcacagc taacatttgt tccatgatat ttacccagca 3900
ccatacaagt gttatttctg tcctcccagt taacactgac gtgggtagta ttatatgccc 3960
attttacaga tgaggaaact gaagcctgaa gaagttaaat acttatccca gaacacacag 4020
ctggtaagtg gcagacctgg aattggaatc tagttcagtt tgattcccca acccatgctc 4080
ttgaccacta tactgttttt tcaagtccag atctgaaatc tcattttctg tgtggctgtg 4140
tgtttgggac aggggtaacc aattcctgac tactctatat gctgcataga acctggagag 4200
gatttttcaa agtaaatgaa tctcgaaagc tggattgcag agcaaacgag tgcagtcaat 4260
tcagccaggg gcttgcaaga gggagaaaga gaaaaagact gtggaatgga aagtttccca 4320
acccaagcct ttcccaaggg gtagccattc tctgttctac agtttagggc ttgcatgtgc 4380
tttttctgga gtggaaaaat acataagtta taaggaattt aacagacaga aaggcgcaca 4440
gaggaattta aagtgtgggc tgggggcgga ggcggtgggc gggaggcgag cgggcgcagg 4500
cggaacaccg ttttccaagc taagccgccg caaataaaaa ggcgtaaagg gagagaagtt 4560
ggtgctcaac gtgagccagg agcagcgtcc cggctcctcc cctgctcatt ttaaaagcac 4620
ttcttgtatt gtttttaagg tgagaaatag gaaagaaaac gccggcttgt gcgctcgctg 4680
cctgcctctc tggctgtctg cttttgcagg gctgctggga gtttttaagc tctgtgagaa 4740
tcctgggagt tggtgatgtc agactagttg ggtcatttga aggttagcag cccgggtagg 4800
gttcaccgaa agttcactcg catatattag gcaattcaat ctttcattct gtgtgacaga 4860
agtagtagga agtgagctgt tcagaggcag gagggtctat tctttgccaa aggggggacc 4920
agaattcccc catgcgagct gtttgaggac tgggatgccg agaacgcgag cgatccgagc 4980
agggtttgtc tgggcaccgt cggggtagga tccggaacgc attcggaagg ctttttgcaa 5040
gcatttactt ggaaggagaa cttgggatct ttctgggaac cccccgcccc ggctggattg 5100
gccgagcaag cctggaaaat ggtaaatgat catttggatc aattacaggc ttttagctgg 5160
cttgtctgtc ataattcatg attcggggct gggaaaaaga ccaacagcct acgtgccaaa 5220
aaaggggcag agtttgatgg agttgggtgg acttttctat gccatttgcc tccacaccta 5280
gaggataagc acttttgcag acattcagtg caagggagat catgtttgac tgtatggatg 5340
ttctgtcagt gagtcctggg caaatcctgg atttctacac tgcgagtccg tcttcctgca 5400
tgctccagga gaaagctctc aaagcatgct tcagtggatt gacccaaacc gaatggcagc 5460
atcggcacac tgctcaatgt aggtttattt ttttcccttc ttctaccaag aaaaaaaaaa 5520
ttgtctctct tgcatgcaat aaagacgttg gaaataaact gcattggtag caagacaaag 5580

gatttaatga tttaatgctg aaggggtgtg atatgctggc atgcatattg attccctttg 5640
ctgaaaattt cctgttagat gttttcttcc caaataaccc ctcccgcccc catgctcctc 5700
tccttaccaa ccttatagtc tcttgaaaac agtttttaaa atatgcataa aaatgtgggg 5760
ggtggggagt agagggtgac cacaagactt taaaacattc ttcaagtagg gcaatttgac 5820
acattttgca gtttttacta aaatgtatgc agagtggaat ttaactttgc tgttctttgg 5880
attagctact agttgtatag gtccccccac cccttgtgta ttccaacagt ccaaagtaat 5940
taaaatatga cattttcctt gaaaggaatt tagtttaatt aaggtggctg ctgctcccga 6000
agttcataaa ccaccagttc aagctgccgc ttccaatgtt ttccttgcta aggaaaggca 6060
atatttctta gcattgaccc tgctgccacc tttccaagtc actttgttgg tctaagaagt 6120
tgctgttttg ctagaaaact actggcaatg aacacccctt ggactgagcc atccattatt 6180
tccatatctt gcagttcagc ccggaacgtg gacatcagtt aaaggaaagg aaatataagt 6240
ggggtagccc aatttaaaaa aaaaatattg actattttgt tatctgatgc tgacacagtg 6300
ccgaggtacg cacctggtca acagtagcct ggctgcaggc ttgagaaatg atcaggagtc 6360
ccagaaaatc ccaccttcgg gtggctgtag tgggagcctg caggatgctt cgccgccctg 6420
acagtgattt agataaatgg ctcagctatc tatcaaaatg tttttagtac tttatttg 6478

<210> 41
<211> 2534
<212> DNA
<213> Homo Sapiens

<400> 41

aggcaggaaa gtgggacagc cggggagctg gaccccaccc tctgtgagcc ccgctggtac 60
ctgatggcat gtggcttgga gagggcaggt gacctggcgt ggagggccag agggtaaatc 120
ctcaaacaag tggcaacagg ccaccaactt gaaagggaaa attgtgtagt gatgggaaat 180
gtgtccaaca aacctactgg gtgactaatt acaaaggctg ggctggagct tcagaggctg 240
cttgttaaac acttcattaa gcggcactct gaaagctgcc acctgcgcat tctgggagct 300
cagaggggac cctgaggggg aatgaggcct ggaggatgga accatcttca ggtagactga 360
gaaggagcct ggatctcact tccaaacaca gtctggagct cataggtcag aggcctcaat 420
gggagaaaag ctaaaggaag agggtgcaga aaggagtttc agggaattgg tggctatgtg 480
actttgagca aatctcaccc ctctctgaga cttagtgttc ccatctctat ggtcctgtgt 540
gtgtcacaga gacatggtgg ggattaaatt cgatcgtgat atgaaagtgc ttgggaaact 600
ccatggccct acctaaacat gagttatcct cacctgaacc aaggggggaa gttacctggc 660
aggattagga accccatcct cctgaacctt tatgggctct gtcgaggctg aagcagccag 720
gggctaaagc cagtccttag cccctggaag ggcactgtga aagtggatct gatttgagaa 780
gccgtttcct gatgtgggca gccatgtgat gccagccccg aacaagaggg ggcagcctgg 840
agcctggaaa ggtgccagtg caggtggggc ccacgcccag atttctcctg ctgactgttc 900
tgatgattca cccccacatc ccagcctttt tacctttact gcagagccgg aaagggtgtg 960
gggaagagag gagagggagg caggtcttgg gccctggtcc cgccccctgc tcctccccac 1020
ccttctctgg gcctggccac ccagccaaaa ggcaggccaa gagcaggaga gacacagagt 1080
ccggcattgg tcccaggcag cagttagccc gccgcccgcc tgtgtgtccc cagagccatg 1140
gagagagcca gtctgatcca gaaggccaag ctggcagagc aggccgaacg ctatgaggac 1200
atggcagcct tcatgaaagg cgccgtggag aagggcgagg agctctcctg cgaagagcga 1260
aacctgctct cagtagccta taagaacgtg gtgggcggcc agagggctgc ctggagggtg 1320
ctgtccagta ttgagcagaa aagcaacgag gagggctcgg aggagaaggg gcccgaggtg 1380
cgtgagtacc gggagaaggt ggagactgag ctccagggcg tgtgcgacac cgtgctgggc 1440
ctgctggaca gccacctcat caaggaggcc ggggacgccg agagccgggt cttctacctg 1500
aagatgaagg gtgactacta ccgctacctg gccgaggtgg ccaccggtga cgacaagaag 1560
cgcatcattg actcagcccg gtcagcctac caggaggcca tggacatcag caagaaggag 1620
atgccgccca ccaaccccat ccgcctgggc ctggccctga acttttccgt cttccactac 1680
gagatcgcca acagccccga ggaggccatc tctctggcca agaccacttt cgacgaggcc 1740
atggctgatc tgcacaccct cagcgaggac tcctacaaag acagcaccct catcatgcag 1800
ctgctgcgag acaacctgac actgtggacg gccgacaacg ccggggaaga ggggggcgag 1860
gctccccagg agccccagag ctgagtgttg cccgccaccg ccccgccctg ccccctccag 1920
tcccccaccc tgccgagagg actagtatgg ggtgggaggc cccacccttc tcccctaggc 1980
gctgttcttg ctccaaaggg ctccgtggag agggactggc agagctgagg ccacctgggg 2040
ctggggatcc cactcttctt gcagctgttg agcgcaccta accactggtc atgcccccac 2100
ccctgctctc cgcacccgct tcctcccgac cccaggacca ggctacttct cccctcctct 2160
tgcctccctc ctgcccctgc tgcctctgat cgtaggaatt gaggagtgtc ccgccttgtg 2220
gctgagaact ggacagtggc aggggctgga gatgggtgtg tgtgtgtgtg tgtgtgtgtg 2280
tgtgtgcgcg cgcgccagtg caagaccgag actgagggaa agcatgtctg ctgggtgtga 2340
ccatgtttcc tctcaataaa gttcccctgt gacactcctc ctgtctctct tccagttctt 2400

ggcgatgggc tgggagtggg actggaatct gacttagaga ccctgacttt ggacctctga 2460
gttagggccc tgaactccct aggtggctca gtggcccgca cgcaagactt tgagtccagg 2520
tgaggccggg gtcc 2534

<210> 42
<211> 6558
<212> DNA
<213> Homo Sapiens

<400> 42

caagatcata aattgcttct catcagtggg gaaggctcca aacattatgc atctagcata 60
agtaggtgta accaaccgta acggcctgtc ctgttactcc cgattaacac acaattatca 120
ggttagaact gcattccaca agtctcttgc gttcagttat cctgcatata ttaattctcc 180
caagctttgt gctttaaaag aactgtacag tgctgggaag ttctttaccc attccaatcc 240
ttagggtacc gagctcgaat tcgaatcagg tatcaggcat gctccatcac cacaaaagat 300
gcaaaaagat gcgcacagtg gcgcacacct atagccccag ttactgagga ggttaatgtg 360
ggaggatcac atgaggctgc agtgagctgt gatggtgcca ctgtactcca gccttggcga 420
cagtgagtct atgtctcaaa taagtaagta aacaaaaatt aaaaagaatc cagtccacag 480
ggcatttgaa ggcaagagga aaagatgcca gaatcagaga tggggagaag atgggcttct 540
ggcgcctgct gaggttgaga aatgagacag ataggctgag tgtggggtgg agagaggatg 600
ggcagagaga ctgaggctgg tctgaatgga aatgaaatgt tagggctctc agggttatcg 660
gggaataatt ggagcttcta ggaaaggttt aacgttgtga ccacctgtgt tcgtcatgcc 720
tccccacccc ttactaattg tgtgaatttg gcagactttg agtctcagtg ttctcctctg 780
tgaagtgggg tcatcttatt ccaactcctg ggattgttgt gtgaattaaa tggggtaatg 840
tacggagagc acctgacgca cagcgagtgc ttcaaaattt cagtctgcac cccccagcaa 900
aggatatgca cacgcccatt gtgagtgaca aatccaggat gacctgaacc caatgtgata 960
acgtgggtcc tcgcatgctg gtcatgctgc cgggagacac ttatggatcc aattagtaca 1020
acaggggaaa taaattattt aatgcatttt gctaagacag aatacctcag aacttatttt 1080
gtggggcggg gcataataaa gggggtcctt ctgctgaaaa cgtttaagct caggttcgtg 1140
gcaccactca accaaggtcg acagtcacac agtaagccag aggcaatgtc aggacttaaa 1200
ctaaacctgt ggcccccaca atgaggccat ttctctttcc cctgaacggc ctggggaaag 1260
ggggtgggtg ggcagaactt ggcagtggcc aatccctcac ttctgtcccc tggttttctc 1320
ctgcccttat ctctaggctt gcattgattg attgattgag acagggtctt gctctgtcgt 1380
ccaggctgga gtgcagtggc acgatcatgg ctcactgcag cctcaaactc ctaggctcaa 1440
gtggtctttc cgcctcctat ctcccgagta cccatatccc taggctttta aaatggcttc 1500
caggtatctg gctgccgtct cagacatcca cctgggcttc tgggcaggga ctgtccggga 1560
aacctcatct atgtgaagca ggtgtgggtg taggaaggcc gcttggaaat gaatcagcac 1620
tgtctcctgt ttgagtcgta agcagggcgc cagagggtct ggcggacaag aaaggaggga 1680
tgacaggagg ccggcactgc aatgacacgc cttagccacc agagggcacg aagcagctgg 1740
gcaaaatccc gcggggccgc tggtggaaaa tttctggcac ctggagcccg gagatggggt 1800
ggacggaatg tgaggaccca gcttcctgag gctgggccgg ggcagagtca ctgctttgga 1860
tgtccgcagg gcctgcttgt gtcttgacta ctctgccttt gtagacagct ggagaatgtg 1920
agagtgggat tgggatcgga ctctaggggc cattccgtac aactctcctg ccctgccgtg 1980
ggggagggag ttgcccaagg ttacgcagca agttagtggc aaatgaatac gattatcacc 2040
agtctcaggt atatggccat ttgatgggcg cagtcgcagc ctcagttcct gagacagaga 2100
cacctgatta aggacaggcc ttcaggagct gaccctagtg acccgcggct ctgctgctgt 2160
ctctgttttt ctccctggct tttccatctg actgactctt tgtcttcttc gtctgcctgc 2220
ctgtctccgt ctctgcccgc tggggggttt gctcaactcc ctcactgggt cctgggagcc 2280
gcagtttcct gctgtcactc ctcagggatt tgtagctctc tgaagctctt ttccgacccg 2340
ttgtctcggt tccactcttg ggatccagag gagaggtgat tatttcgtag catagtcagt 2400
ggtgtgattt cacggggtga gaaggactcc cttgctccta agcactcctc cagtgacccc 2460
tgttgccatg tggtagccgt aagcactggt tggcacctgg tgtgggcgag acccttacct 2520
catgcagaaa tgagtaagac cggtgagctc accatgtggg ggtgaggctg agagaaaaca 2580
agtacacagg tgattcagtc aaaatcagaa ttctctaagt acacacgaaa agggcaaaag 2640
gggcgctttg tacaggacag aacaggtaga cactgaatcc ggttgggccc tgggaaggct 2700
ccctgcagtg gcctttgaag ggggggttgg atttcagcag gatagagggc atgggcatgt 2760
gtgggcacgt tctgaacaga ggggtcagcg caagccgagg gtcttggcca cactagttgc 2820
atgtgccggt gtgtttaagg gacacgcagc agcaggccga gtctggagcg cctcactgcc 2880
aggcttttta aaaattttta attttaattt aattttattt tatttttact ttaagttctg 2940
gcatacatgt gcagaatgtg gtttgttaca taggtataca tgtgccatgg tggtttgctg 3000
cacctatcaa cccatcatct aggttttaag ccccgcatgc atcaggtatt agtcctaatg 3060
ctctccctcc ccttgccccc atccttctcc ccgcaactgc ccacaggccc tggtatgtgg 3120

tgttcccttc cctgtgtcca tatgttctca ttgttcaact cccacttatg agtgagaaca 3180
taccgcctgg ctttaaggga cagccatggg gatgcactgc agtttctgag cagggaaggc 3240
cctgtggagg cccttagtta aaaggaaaga atggctgtga aaatcgatgc attgcgctcc 3300
cttgtccctc accctcagtg tgaagggttt ttattccgag ttctacttga agtaggcctc 3360
gatgggaaga caagtagcat gaggggttca agtactgagg ggagcaaggg acactcggtg 3420
gctgtgccaa ggtgtagaag aggacactgg gggccccaag gcctgacttc atgtacactg 3480
ctcaggctgg cccccaagtc acacggtgac cgctaggaag ggaccagcct gttctcagtc 3540
tgatcctcct gccatgtcat tatccaaagc tcctcctggc agggcctgtt tggggtctct 3600
gtgccagtgc tttccctgcc aggctgggct ggggcttcca cctactgctc tgggactgct 3660
gctgccctgg ccctggggga ggagggtgtg ccgctgagtc actgcctggg catctgggcc 3720
tggaacctcg ggtgagtcac ttagggctga ggtagagggg ctgggggagg ggaagaagct 3780
actcgacagc tggagcaggg aggggagctg ggccacaggg aagggcggtg ccctgatgcc 3840
cagacgggcc gggatagaca aagggccaag gaggaagggg ccctgggagg gggcagccct 3900
cccttgggct gggctctgaa tggccacagt gtttgccttt ctccgggtct ggggaggaca 3960
tgtgtgtggg gggcagtgag agagggctgt ggctgagggc tgtgcttcag gcctggattc 4020
tggcttggga agctgtccag ctggtgtttt cagccttggg tagggatgta cccctaccca 4080
cccacccagc cctcaagctg gagaagagga ggccaaagtt ttcctgttca gcctttaact 4140
actcgggact tccttatgct ccccacagac tgtggcccag cccaactgcg gctgtgtgta 4200
gagcaacccc atttctcact gcttccccat ccttccagac accttcctac acagagggac 4260
cttcccaggt atttctaagc acacttagtt acctcattac ctcattaaga ggtattctgg 4320
tgctggccat taaaagtcac tccacttcat ccatgccctg aagtcagtcc tgtccttctc 4380
ctcctgatgt cccccagctg cctcctctgg cccccagctt cctaagggtg gccccaggtt 4440
gcttctctct cacacacaca ggcgcatgta tgtacacgag cactggacca tgaagtctca 4500
gcgtgtgctc acagcctctc acacaggagt gggctgtgac tcacaggcat gtcatgagaa 4560
tgaggcctgg caccagtctc caggccccag agcaggggtt gcctcccctc accccggtcc 4620
aggatgccca gtccccacga cacctcccac ttcccactgt ggcctgggtg ggctcagggg 4680
ctgcccttga cctggcctag agccctcccc cagctggtgg tggagctggc actctctggg 4740
agggaggggg ctgggaggga atgagtggga atggcaagag gccagggttt ggtgggatca 4800
ggttgaggca ggtttggttt ccttaaaatg ccaagttggg ggccagtggg gcccacatat 4860
aaatcctcac cctgggagcc tggctgcctt gctctccttc ctgggtctgt ctctgccacc 4920
tggtctggtg agtacctctg tcctgctgag ggcagggtgg ggaggatccc cgtgggtctc 4980
tgtctttgtc tccacagttc tctcattcca gcttccctgg tgggatcaac ctgggcctct 5040
ctgggccttc ccccttggaa gaactctctg tgaagtgctg aagtgttgac tgaagggttt 5100
tttttttttt tttttttttt tgagatggag tctcgctctg tcgcccaggc tggagtacag 5160
tggtgtgatc tcagctcact gcaaactccc cctcccaggt tcacgccatt ctcctgcctc 5220
agcctcccga gtagctggga ctgcaggcgc ccaccaccat gcccggctaa tttttttgta 5280
tttttagtag agatggggtt tcaccatgtt agccaggatg gtctcgatct cctgatctcg 5340
tgatccaccc atctcggcct cccaaagtgc tgggattaca ggagtaagcc accgcgcccg 5400
gccgactgaa gggtttttct ccaggttcct ctgtgaggtc tcagtgcagg ggttgctctg 5460
aggccctccc ctggatatct cagtctaggg gcccttcttt gggggtctag gcctaggagc 5520
aggaggtgtg catgtgggcg ttgctgcaaa aagaatcctg agattttttt tttttttttt 5580
tttttttttt gcaaagtcct ggattctagc aggactaagg tgcaagaggc aggggtctca 5640
agactctgcc tgggtcatgg ccccaagcag caaagctctg ccccctgcct cggtgaaggc 5700
agggctggca tgatgggccc agggcatgcc ctgcctctgg catagctcct ctggcctcac 5760
cctgaaacct gcctaacctt tccaggctgg tctgagtatt ctcagaggcc ttgccgctga 5820
ggtctgtccc atcctgatcc caaggcaatg aacatttcat atctttaatt ctaattccaa 5880
caggatcctt cctggtggag agaatgttaa gttgccccca ccctatccat gcccctgtct 5940
gcctagaggc tcaggggcct tcagggtgag gggagacaca ttccccaccc tctgggagct 6000
cctagtctga gagaggaaac actcctgccc aagggagctt ccagttagat ggcagagaga 6060
gatgcctctg gcttcaggag tcccgagtct aaggagggaa acgactcctt cagggagctt 6120
cctgctccta ggctgtagcc atggctcctg ccagactgca caggagcccc catctgccag 6180
ccggtgcatg tggccctgct ccccagagcc tgcgcagatg ccatcaaaat gggactctgg 6240
tcaccctgtc atttcccttc tggcagacac taaaatgggg agccctgccc tcaggggggt 6300
gtcccaagtg ccatcagagg aggcttggtg actcccagac acaagggaag ctttagcgtc 6360
tgccctcagg gtgagatgga ggtatcgcct ccggcctcag ggaaccacag tctgaggggg 6420
agatgcagcc cctgccttcc cattcagaga ggggttttgt gaggtggctt gggggcatag 6480
ggcagaagtg gatcctacag gctgagctaa ggccccaaga gcctcagcag tgtacccatc 6540
acctggcacc tctgcagc 6558

<210> 43
<211> 6432
<212> DNA
<213> Homo Sapiens

<400> 43

caccacgctc agctaatttt cgtattttta gtagagacag ggtttcacaa tgttggtcag 60
gctggtctcg aaatcctgac ctcatgatct gcctgcctcg gcctcccaaa gtgctggaat 120
tacaggcata agccagcatg cccggcccat attctttaac tgtatgtaca atttaatttg 180
tagatgatcc taaaagtcta aattggagtc ctactgtctt cctcatcttc agcccccata 240
acaaacctca tgatagtttt tgtctataag aacccctttg tagttttacc acctgcaaat 300
tatttaacct tgcttttccg tgattcctca cccttcacat cccttatatc tcttagctat 360
tgcctcaaaa attctggata acaaaccaac ccaaattttt ccttaaacgg catttactta 420
ttcttactca gactcatatg gattggctag aggtaggctt gagattggct gatggaggtc 480
tggcttagat tcaatcagga ggttggtcca catctgttcc acatgtctct cctcctccct 540
gcatttgctg gtctttaagt gccttcaact gaaaataatc agtataccaa agcagcatat 600
tttggagtgg catttcctga aatccttcac tggtcaggtg gcgaagatca accacctgat 660
caatttgttc tagagtgtag aacaagtctg gaactagaca ggactaagtt caaatctggg 720
ctctgtcttt tactagctat gtgacctttg tcaaatactt aatctcttta atcctcaaat 780
tcttcatttg taaagggaag attagaatag tactgcaagg acatgctcca aaatttaaca 840
aggattatct ttggataata gggttaagaa tccttttaaa gtctgccttt aaaaaaaaaa 900
tctaaatttc ctacctataa ttaacacgta ttacccttgt aattttcaaa aagttatttt 960
tttcaaaaca tactattgat gctttctgta aacatgtggc ataattgcag gttctattaa 1020
tatctgtgaa gaactgcaaa caagagaatg gattaggaaa actgatattc ttatttttct 1080
ctctattgaa gacaagaaac acacactgaa tactgattat ctgtagagca cggagtgtgg 1140
ttctacattg aaaaaggaat attttgtgct tttaagggct ttacaatcat tagggtagaa 1200
ttctaaaaag caaataaata acaataatgt actccatgtt agaatggggc aaacattttt 1260
ttttaaaaaa aggctaaata aacgaaatgt tccctgggtt atccctggga aaggagtgat 1320
cacagtaggt gaagcatcac aaaatatttc ctggaggagg tagcatcatg gatgacccac 1380
aaagggtgga tgaccctcac aaattaacta cagtctcagt gcttattggt ttgcttcaca 1440
ataaaaggaa aattggtcaa gagataagag ctgagaatga ggctggttaa tttataagga 1500
gaggaatctc cctgttaact gctctagctg acagtaacct caacagctgc atgtcacaaa 1560
gctcagggga gtaggaagtt cttctctcat cacaactgag aaacccaacg tttaaataaa 1620
ccacaatttg attcagttca attgtgtgtg tttgtgtgtg cgtgcagagt ttaaaaagct 1680
ggtaaaatag attgtcagct tttaaaattt ctgttcaagt tgatatttat tgtttttcaa 1740
ctcattaagg gcttaaacgt taccttcctt gttttttaaa ccctgagtcg ggctattatt 1800
tttttgttcc catttgcaaa tggcccagtt ttttacatgt atatattaac tgcttatgtg 1860
aatacacaaa atattatact aagccaaata tacaactatg tgtgaaaatg atgtcttgat 1920
taagggtatc agacatccct ttcaacaaga atttcaaaag gaagggcatc atgacaattt 1980
ttttttcttt ttcttttatt tattttttgt ttttgagaca gggtctcatt ctgtcttcta 2040
ggctgaagtg cagtggcaca atcatggctc actgcagcgt tcgtctcctg ggctcaagtg 2100
attctcccac ctcagcctcc tgagtagttg gcactataga tacatgccaa catgcctggc 2160
tttttttttt tttttttttt ttttagtaaa gatgaaatct cactatgttg cttaggctgg 2220
tctcgaactc ctgggctcaa gtaatcctcc catctagacc tcccaaagtg ctgggattac 2280
aggtgtggac cactgcactc agcctgatga atttttaaaa ttatcaaatt gcaatatgca 2340
tgagtttaca catctattat ttttattatg atgattttca aaaggctagc ttgcacttgg 2400
caatgctgaa ttctgattct tctcaatttc acaggagact ggagattaaa tcagaaatgt 2460
taagggcaaa caaaattaaa atcaacatta atgatgtatc atggcaaaga ccaccagtta 2520
aacattccac catagattta aatatctagc ctccaacaat gtaaaacatc tttgtgataa 2580
ttaaggaaaa caaagctttt ggaatctatt ggacatttat ttgccacatt gtgttttcag 2640
gagaaagaaa gtacatgttt gctatctgga ggaacttggg caaatgtagg aaaaaaaaaa 2700
gaagaagaaa gaaacccaca tacttctcca ttcttaagaa aaggaagtag tggtgacatc 2760
attctcggct aaaatgtagt ctgtttatat ctcaaatatt cggtaacaat gttttctcca 2820
gagttatgaa gcaactagaa caatcaaata caattttctt ctaaatcttt attgcctgat 2880
tcattcattc atccaacaaa tatttattaa acatcgatta tgtgtttgat gcttagggac 2940
acaatagtaa gtggagggaa agatacataa tacctgccct caagaaattt ggagttgagt 3000
ggaggataga aatataaatt aaagaatgac acaaataatt ataaagttac agctgttaaa 3060
agaaaagcat atggtgccaa gagaacgtgt aatacaagat ctactcatgg aggtgaggga 3120
aagcttgccc atcaaagaag ttatgattca atccacgaag accaggagtt ggctgggtga 3180
agaaaaaaag gtcagaggaa ggaagtccac actggggaag gctctaagca taaagggtag 3240
gaggattaca gaggcatatt cacgaaattt ggagaaggct ttcagtaagc aaggagaagc 3300
caaatgaaag tttacgggag agttggaggc ttgaagacac gttcaaggat ctggttttta 3360
tcttctcttt atctcaagag cagtgggaag ccattaaatg attttaatca gagggttggt 3420
ataactagtt ttgtattttg aaaagctgaa ttcagctctc gtttgagaaa ctgagtgaaa 3480
gagcccagaa cggccgtggc tgagggtgac tcgtgggaga ctcctacaca agccatggca 3540
gtggcatggg ctggtggcag aagagggaat agggagaaga tttggaactc aatcttcctc 3600

cattgacaaa gtcactccag ctttggcaag gcaattaatt ggtgggaaag aagatgccta 3660
gccctcctga tttcactgca ctttctgcat cttcaacatg agtactggga agtggcaaaa 3720
catccagagg cagcttgggt gctaggtgga gcatgagtta aaattccagg atgaagcaaa 3780
tgaacactta gaatgacagg aaagatttgg gagttgggtt tgggggaggg ctatttacct 3840
ttattccctg gagaccctgg cacaaaccct tgcctctgca atcttcctct caggtaaagg 3900
aattcattaa atgaattgct agaagatcta ctgaccagag ggctgtacag aatcatatct 3960
ttgagagtgg gaagtaggtt gatcacatag tttattatcc aatcaggaca tatctgaaag 4020
agaaaggggg ttctattaat atttaaacta caaaacatgt acaccaggaa tgtcttgggc 4080
aaatctggtt gccctagcaa gaaaggaaat ttgaaagttt atactgttct gctcccatgt 4140
taccccgttt gcacatgaga gggtaagtat tctctttctt cacctgcatt aagggaataa 4200
aagcacaagc attcaggtga ctcccaaccc acttttaatt ttacagtttc tgctatactc 4260
tatacattct gaaaattaca tttcccacca ctatcacttc gtgataggtg atcatttaca 4320
attactcact gactcagtcc cgggaagagg cggtgcaaaa tgggacgctc tatccaggtg 4380
ctcattagaa atgcagaatc tctgcctgcc tcctagacct actgaattag aatctgcatt 4440
tttaaataag atttccaggt gatcaatatg tacattaaaa cttgagaaaa acctctagac 4500
ttcgacctaa agaaaaacat tttacaactt gacagtgtat gcacatacat acatgcatat 4560
agacacaact gaagcacaaa tttaatgaag tagaatttac cgttactatt ttatttggga 4620
aagaaatgtg ctcgcgactc aatagattgg agtattcact cctggatctc aacttgcaat 4680
ttgaaaacgc atctctaaag cacctaggag caatctgaag aaagctgagg ggaggcggca 4740
gatgttctga tctactaggg aaaacgtgga cgttttctgt tgttactttg tgaactgtgt 4800
gcacttagtc attcttgagt aaatacttgg agcgaggaac tcctgagtgg tgtgggaggg 4860
cggtgagggg cagctgaaag tcggccaaag ctctcggagg ggctggtcta ggaaacatga 4920
ttggcagcta cgagagagct aggggctgga cgtcgaggag agggagaagg ctctcgggcg 4980
gagagaggtc ctgcccagct gttggcgagg agtttcctgt ttcccccgca gcgctgagtt 5040
gaagttgagt gagtcactcg cgcgcacgga gcgacgacac ccccgcgcgt gcacccgctc 5100
gggacaggag ccggactcct gtgcagcttc cctcggccgc cgggggcctc cccgcgcctc 5160
gccggcctcc aggccccctc ctggctggcg agcgggcgcc acatctggcc cgcacatctg 5220
cgctgccggc ccggcgcggg gtccggagag ggcgcggcgc ggaggcgcag ccaggggtcc 5280
gggaaggcgc cgtccgctgc gctgggggct cggtctatga cgagcagcgg ggtctgccat 5340
gggtcggggg ctgctcaggg gcctgtggcc gctgcacatc gtcctgtgga cgcgtatcgc 5400
cagcacgatc ccaccgcacg ttcagaagtc gggtgagtgg tccccagccc gggctcggcg 5460
gggcgccggg ggtcttcctg gggtccccgc ctctccgctg cgcttgacag tcgggcccgg 5520
caacccggcc cccgggcgga aacgaggaaa gtttcccccg cgacactcac gcagcccgac 5580
tcccgtagct gcagggattg tgagtttttc ttgaaaaaga gaaggaaagt tcagttgcaa 5640
ggggcgcggg gcacgtttgg tcccctttgt gcgagcagga aaggcgttgt gttggccgcg 5700
ttcgaggcga gcccccaccc ccggaaaggg aagtttgaga agttggttat ctgaaggcgg 5760
ccggggagca gcggcccgga gcggcagcct gagctgccaa agcagccagc gcacctgggc 5820
cactccgccc atggcgatgc cgcgtgccca cccagctctt tttgagccac ccgttcaaaa 5880
ggccagctcc tcagtcctta gctcctggag acggccacgc tttccctcag gccggcttct 5940
tggcccggag tttttggaaa caagtttcaa gaaaataatc gatttccaaa agaaagttag 6000
ctggctccac tgacgccttg gcatggatgg atagggagtg gagatgctca ggtgaaaccg 6060
agaatccctc actgaatgcc tactgggtgc tagaggctgt agattttgcc tggaacaaga 6120
tagtcctgtc ttcagggagt tgatggttct atgcaattat cgtttgttgg aaaccgaagg 6180
gttaaaatcc taactaggct acccacctga tcgctgtctc tgaaggtttt taaggaaaaa 6240
ataaaaataa aaaaatatat atatatatta tatatatata aatatatata taattatata 6300
tataaatata tataatatta atatatatat gcatgcagga atgggggttc ttaactattt 6360
gttatggaaa gtgtaaaacc tctgagactt caaaagttag attttttttt tttggagacg 6420
gagtctcgct ct 6432

<210> 44
<211> 7309
<212> DNA
<213> Homo Sapiens

<400> 44

atgcgatcaa tggtgttaac tgctctttgg actgtcccca cagcacagga caagaccata 60
tcagttccag caggttccac tgaagaaagc tcatgtgcct tgcagaaaca agacctccag 120
cccagtctaa caggagacca accacatgtc catcccattc cctcccaagg catgacagca 180
cctggctgag atgcttgtgt gtattctctg tgtgaagctg agcatgcaac cttcaaagaa 240
accacaaagg agctactcgt ttttaaagga aaaacacatg aactccacgg gcataaggaa 300
gacattaaaa cagggttggg gagaagttct tctccttcca tttcatagta cccttcacag 360
aaataattga ctgcttgaat atatggtcat tgaaacatac atacacacac atacatgcac 420

atgaaaacaa gagctggaat atttgactct gctttccatt tcccaccacc ctagataaca 480
caggatacaa catggcttta ctgcacacat gtgttttctg gcttaaaaac actgagagta 540
gatccctgtg gaaagaaata tgaaaacctg aggattcatg cgaatgtagc cagaggcgaa 600
tgtagccaga ggggtcttca atttgctggc agagctgggg gagagctggg gcaattttac 660
tgccatgaaa ttggctccag atggaggcaa ggaaaggtgt ttaccccctg ctggtgccag 720
acacagagag atcctgcagt acctcgcatc ttaggctcag aattgcccag tgctgggcag 780
cagccccact gagggtctgg ccagagctca tggggatggg tggaccaggc tgtctgttgc 840
ttggaaaagg ccattttgaa tataatgact ttcacatgga cggtggggct tgggggaagg 900
catcgtggct agggtctgga ggggaagagg gaggaaagca aagaggagct tccctatgag 960
gcaacagaaa tgacagtacc ttctccaggt tgaatttcca aaacccagct ctgccctgga 1020
agactgggac tgtggcatcc tctgcagtgt tcccagaccg ccctaaggcc cccaaaccaa 1080
tgattcacaa ctatgttgct tccccagaga gcatctcccc ctttggttga catgtggcca 1140
gctcaggggt ggaggagctc cggcagccac gaacccagac gggatgttga ctgagagctt 1200
tgtggccatg gcagatggga ggggtgagct tcacccactc aaaggcaccc gcaccccacc 1260
gatgagtgtg gaattgtgct ctcccgccct cgcagtgaga cgaccgacca ggactgcagg 1320
ctgagcaaat gggtgttctc tggcttatag cgccccctag caccagccag cgggatagag 1380
ctccgtgaac tctccaagga gccaccacct gcctgtctga atgctggaaa ggggtcggtg 1440
gtgctgatca ttggacccat tcaaccaata cttattaaac tcgttgttac caacacttaa 1500
tccctgtgtt ctaggggctt agactccagc aagggaaaca gatgggtaaa taatcacatt 1560
agagtcagct aagtattgtc aaagaagttg atacagggtg ttgtggggat acatggaggg 1620
ggaatcagag gaggtttccc ggaggaggtg acatctaagc tgaggtctga aggatgtgga 1680
agagttcagt aactcctgaa gaccaaaaaa atggtgataa aaaagacacc ttcctaactt 1740
tttagtgcta cgatccaaga gacaaacaag caggtgaacg cataactaca ctgggctcca 1800
ccataagcca gagcctgtct ctcctaggac tttggcatca caatcttctc tgaaattctg 1860
ttttcggcat tgattcttgg gggaggtagc aatataggaa ctcagtgttc gttgcgaaac 1920
tgatggatgg tcttcagtct tgggtacgtt cccatgtgtc aaacctgttt aaatggcagg 1980
acccatctcc ccttcagaag cattcacttg tcctacagga ttccccatgg aatcttggag 2040
tttttgaggc gagagggatc ctggatacca ctgagttcta tctttcatcc aataaacaca 2100
gaagtggacg cctggacagg caaagtgact tgaccaaggc aggtgcacag ctattctgca 2160
acattgggaa caaatctcag gtcttttgat tttttgtttc cactttactc tcttttcatt 2220
tcccagaaac aaagttttca tgtgcttttt tttatagtga tatgtttgga atgcattagc 2280
tagtaattta ggaagggaaa aaaataaaca cacaagagat aaacctgtca ggaggacaaa 2340
cctgtattgc ttctgattgg ctcagagggt gattattatc atggtagaga attatttaat 2400
cagtgtaagt aaaatttctc tgtgggctgg gcactgtaca aagactcaaa cgaatctgtc 2460
tacagatctg aaaagcagat acgagatctg tgaatggctg gggtttccaa gcccacagta 2520
caagcatggg ccacacctta cagcttggag gactgagccc tgaaaatggg caagttcctt 2580
cacttctctg aaccttattt ttcccacatt taaaacaagg atgagtagtt tctgaggtcc 2640
tttttacgac ttctcttcct acagactcta gcatcctata acttgataca aagagggtgg 2700
atatgaactc acctttccta gaaaagttcc aggaaagaga ataccaggtc atcctagtag 2760
gtgtgtagac aggccagata gatcttgaaa cttactcagt tcttcccaga tgtataactc 2820
tatcattgtt cttagctgtc aagagaaagc aggagagcct gcatcttcat tctttttttt 2880
tttttttttt tttttggaga cggagtctca ctccatcacc taggctagag tgcagtggca 2940
tgatctcagc tcactgcaag ctccgcctcc caggttcacg ccattctcct gcctcagcct 3000
cccaagtaac tgggactaca ggcgcccacc accacacctg gctaattttt tgtgttgtta 3060
gtacagacgg ggtttcacca tgttagccag gatggtctcg atctcctgac ctcgtgatcc 3120
gcccaccttg gcctctcaaa gtgctgggat tacaggcgtg agccaccgca cccagcctgc 3180
atcttcattc ttactgttag cctcaggttc accccaccta gcttattaag tgatgttgaa 3240
taaccaattc ttacatatta ttaggctcat ggacaccatg acatccagac tgatgggtgc 3300
ctgctgaagg gggtgaccct agcaggagga ctcccctacg caaggattca tggagtttgc 3360
tgtttctttt ccttagggtg agaaccaaac tgccttcaca cggtgggcag aggggaactg 3420
actcaggttt ggaataagag agaacatccc aactgaaaag ctcttggaat tcgctgaact 3480
tcaagacact gtgtggacca gcttaggata gggagtgaga agaaattaac caaaaggtaa 3540
tttcgttact tttcagctgg aaaaaagatc agattatact tgtgctttca taattaagta 3600
gctgctggaa aaaaacgctt cagatgcttt ctatgagaaa actgctgctt gaagttcagc 3660
agaagttatc tacttgatac ttatattcca ggcaaggcct tccgttggag aaaatatcgg 3720
cactttggac aaaactgaaa tgtgaaaaga aagggaagag agggcctcta tcatgtaaga 3780
tgcttatcca aagtggattt ggtctggaaa gtcttctaaa accttccaca tgactgtgga 3840
ataagtcatg tggggcgcgg ggataagcga atctctcaaa ttccaccacg tatgccctca 3900
ttcaacctgg atccttagag tggcctccag ggcactctgc tcaggactca gtcagctgtt 3960
ggccacaccc atgctctcca gtctcctgag accctatttg gttctgagag ggctaaaaag 4020
cagtgtggct aaatatccca ggcctcaaag tattcctact gtggttgggg aagcaataga 4080
atcatacccc ataaaacaat gaaaacagtg ctagaaaaac atcgagagac agaaacatct 4140
ctacgagtta ggccacagtt agagtgaagg cagggaaggt ttttaaagct gggtggaggg 4200

gacaagtcaa aaagatgtgg aaactggttt ccctttccta tggctaaagt gctcaaaggg 4260
gaaaaaggag tttcaaaaat gttcttggaa ataccatctc tcacgaattc ttcggcctct 4320
gctgtcccaa tgtcacttgt ctgagatgta aacagaggag ttctgagaaa gaagctgaac 4380
ttgcatttct ccctgtttct atttgttcca aacttgtggc atttctaaca ggatgaagcg 4440
gaagagaaag ggaaagagac aaaagtgtag aaagatggaa gatcccagct gcaaatggcc 4500
atttgcagtt agatggaaca gctgctgacg ttcagggaaa tgcatgtctc tcttcagatg 4560
ggaaggagca gtggaaaggg gtgacgagtt cctggctggc caccaatcat cccatctttc 4620
tgtgccggtt cctcatctgg aaagtgggag tgatacttgt gcttgctttt cctacccaca 4680
aagattattg tgagagctat aatacggtga gatacagaat cctgctttta aaaatacaaa 4740
gcagaatcaa gatgtcaata ataaggatag taattgtgtt agttatctgc aatcatctat 4800
tatagctagt cgtctaggat cctggatcgt tctcctggtt ttactacagt tttggatcag 4860
ctcaccccca aatcccttgc tgaagggtgg agctctgtca gccatgggca gggaaccact 4920
tcctcttgcc tttctacttt ctgtctttca aacatgccca gggtctttgc acttgctgtt 4980
ccccctgcct ggtacctctc tcctgtggct tgccccagag ctgatccttg tctttgtcca 5040
cttctcagcg aggatggcac ttcagggagc ccttccctta ctatcgcaga gagagcaggc 5100
cctccccagt catgtccaac ccagaactct gttttgtttt cttcatagcc ctagcatcac 5160
agaaaatcac cctgtgcatt catggatgtc cacgggggca agggctttgt gttgcttaac 5220
ccagcatcct gaaccgtgtt tgttgaatga atacagaacc ccgtttgctc tgggagagca 5280
cagaaaacag tcttctatca tatatcatag ccagctgcaa acagcagatg gcttcccata 5340
tcccagagag taagaaccag agagagagag aaagagagag agtttgggtc tttctcctct 5400
gtgcctgctc tctccagaga aactggaggg gtagcagtta gcattccccc gctggttcca 5460
ccaagcacag tcaaggtctc taggacatgg ccacccctca cctgtggaag cggtcctgct 5520
ggggtgggtg ggtgttagtt ggttctggtt tgggtcagag acacccagtg gcccaggtgg 5580
gcgtggggcc agggcgcaga cgagaagggg cacgagggct ccgctccgag gacccagcgg 5640
caagcaccgg tcccgggcgc gccccagccc acccactcgc gtgcccacgg cggcattatt 5700
ccctataagg atctgaacga tccgggggcg gccccgcccc gttacccctt gcccccggcc 5760
ccgccccctt tttggagggc cgatgaggta atgcggctct gccattggtc tgagggggcg 5820
ggccccaaca gcccgaggcg gggtccccgg gggcccagcg ctatatcact cggccgccca 5880
ggcagcggcg cagagcgggc agcaggcagg cggcgggcgc tcagacggct tctcctcctc 5940
ctcttgctcc tccagctcct gctccttcgc cgggaggccg cccgccgagt cctgcgccag 6000
cgccgaggca gcctcgctgc gccccatccc gtcccgccgg gcactcggag ggcagcgcgc 6060
cggaggccaa ggttgccccg cacggcccgg cgggcgagcg agctcgggct gcagcagccc 6120
cgccggcggc gcgcacggca actttggaga ggcgagcagc agccccggca gcggcggcag 6180
cagcggcaat gaccccttgg ctcgggctca tcgtgctcct gggcagctgg agcctggggg 6240
actggggcgc cgaggcgtgc acatgctcgc ccagccaccc ccaggacgcc ttctgcaact 6300
ccgacatcgg taagcgctcc tggtgccccg cccgagcccc acgctgcagc caggactgca 6360
gcgctgctta gggaggcagg gcgagcccca ctcctttcct ctgccccagg agaggggcag 6420
acggggttgg ggcggagtgg agaaactcga tgtccttggg cgggggcgct ggcatagctg 6480
agaggggaag atgccctgca gaggaaactc acagtggctg agggagcccc tggccgcctt 6540
tgctttccta acttaggtcg tgaggttcct accggtcctt ttgacatctg gaaaatgtcc 6600
ccattcacta ctaacggagg aagggctaga agagaagggt ggggaaaggg ttcccaaaac 6660
ttggaatgct aactaaagtg ctgggaaatt gaatagttaa aaaaaaaaaa aaaagttgcc 6720
cgccatgtgc acggaaagtt gcaggagaaa cttggagtac tccgagtctg catcatctcc 6780
tgcgcctatg aaattcattc ttttcactga gacccaaccc agctgcaggc ttcccaggca 6840
agcgctaacc gagcttctgg gactgccaag tttgggtgga tttaaggggg aaaatgagga 6900
gaaagctgtg gcattgaccc acgccctgga ggcactgtgt ttgagagtgt tgatagccag 6960
cagaaggtgc gaggggtcac tcaccacata gctgtgaaaa ttcacaggaa ggctattttt 7020
ttccccccttg aatgtgtgtg ggagggggga gaatgggtag tggaaaacag atttgtcacc 7080
aaaagaacaa acctgccaag ggtcacatca cggtgacagt tccagactgg acttagtgtc 7140
ttgagtagag agaccctgta aagagacctc ctactcctta gtgtgtatgg agggccccga 7200
gagcatcctg ggaggcttgt ctgggagtgt ttgggacact tgtgtttgga tggctaaagg 7260
tggagtggca tctgggaaac atctctctcc tcctttccag cccataaag 7309

<210> 45
<211> 15863
<212> DNA
<213> Homo Sapiens

<400> 45

agcctcaacc tcgcaggctc aagcgatcct cccacctcag cctcccaagt agctgggacc 60
ataggcccaa cttttttgat agagacagga tctggctgtg ttaccaaggc tggtctcaaa 120
ctcctgggct caagcatcct cccaccttgg tctcccaaag tgctgggatt ataggcatga 180

gccaccacac cagccacaaa aatcttttga acacaaactc actggccagg agcagtgctc 240
acgcctgtaa tcccagcact ttaggaggcg caaatgggtg gatcacttga ggtcgggagt 300
ttgagaccag cctggccaac ttggcgaaac cccatctcta ctccaaatac cccaattagc 360
cgggtgcggt ggtgcgtgcc tgtaatccca gctactcagg aagctgaggc agcagaatcg 420
cttgaaccgg gacacagagg ttgcagtgag gcaagatcac accactgctc tccagcctgg 480
gccacagagc gagactctgt ctcaaaaccc ccccccccca aagaaagcca aaaagaacac 540
aaattgacta tctgtattgc aagatatatc ttttaaaatg tcctaatact ataatcatta 600
atatcataat cacgacctcg atgaaccact ctcgagagtt gaaaatttga cagtcgttat 660
caccgtctca gaaagcagta caaaaaagaa agagtgctga tggaggagga gaaggcccag 720
cagagccctg agtgcctgag tgctccagcg cagggaggtg gagacagtga ctcatgctgc 780
taagagatgc agtgaggcga atttagagaa tgacccaaga ggggctgggc gcggggctca 840
tgcttgtaat cccagcactt tgggaggccg aggcaggagg atcacgaggt caggagatcg 900
agaccatggt gaaaccccgt ctctactaaa aatacaaaaa attagccagg cgtggtggcg 960
ggcgcctgta gtcccagcta ctcgggagag gctgaggcag gagaatggcg tgatcccggg 1020
aggcagagct tgcagtgagc tgagatggcg ccactgcact ccagctgggt gacagagcga 1080
gactctgtct caaaaaaaaa aaaaaaacac ctctctcatg acttcccaaa taaactccaa 1140
atgccttacc cataagaacc aacacgacgt ggctgctctt ctctgtcctc acccctctgt 1200
ccccctcact tgcctcagtc tggctatacc agcattctgg gttttttgtt ttgtgtttgt 1260
ttgtttgttt gtttgtttgt ttgtttgttt tgggatggag tctcactctg tcacccaggc 1320
tggagtgcag tgacatgatc ttggctcact gcaacctcca tctcctgggt tcaaatgatt 1380
cttctgcttc agcctctcaa gtagctggga ttacaggcac ccaccatcac acccagctaa 1440
tttttgtatt tttgtagaga tgaggttttg ccatgttggc caggctggtc tcaaactctt 1500
gacctcaggt gatctgccca cctcagcctc ccaaagtgct gggattacag gtgtgagcca 1560
ctgagcccag tctgttttgt tgttttttga gatggagtct cactctgtcg cccaggctgg 1620
agtgcagtgg tacaattttg tctgactgca gcctccacct cctgagttta agagattctc 1680
ctgcctcagc cacacaagta gctgggatta caggcgcgtg ccaccacatg cgtctaattt 1740
tggtattttt agtagcgatg gggttttgcc atattggcca ggctggtctc gaactcctgg 1800
cctcaagtga tctgcacacc tcggcctcct gagtagctgg gattacaggc gtgagccacc 1860
atgcagggcc tttctggtct tttaacgcac aaagctattt cccagttctg ggtgtttata 1920
gtatcattgc cagttcctgg aaagctcttt ccagaagcct tcacatgact gatcccttat 1980
cctccttcag gtcttagctc aaatgcctcc ttttcagaga gctccttcct gaccatttta 2040
tgcaatgtgc tttcctctag ttagtctctc ttcattctgt gtatttcctt cagagcattt 2100
atcatcatct tggtcctgat gcttgctggt ttacatgttt gttgtctgtc tcccacacag 2160
aaagcaaacc agcccttcat ctgtcttgtc caccacttta tcccagcaca gtgactaaca 2220
tatatcagta gtcaatcaat aaatagctat aagccaggca cggtgactca cacctgtgat 2280
ccccgcactt tgggaggcca aggtgggcgg atcctctgag gtcaggaatt tgatatcagc 2340
ctgcccaaca tggcgaaacc ccgtctctac taaaactata aaaattagtc gagtgtgatg 2400
gcgtgcgcct gtaataccag ctacttgtga ggctgaggca ggagaatcgc ttgaacccgg 2460
gaggcagagg ttgcagtgag ccgagatcac accacagcac tccagcctgg gtgacagagc 2520
aagaccttgt ctcaaaaaaa taagaaatag aaatatatat atgctgccac aagaaattca 2580
ctacttttta gcaaaaaaca cagattccta attaaagaaa ggggaaacct ctctctaaat 2640
aacttcagaa ttcggggcag aaaacgctac atgtggaaac tcgtcttgaa aaacagtccc 2700
gtttgtgaac aggaaacact tggactacac tttttccatc tccacgaagg acttaaatgt 2760
gcagcattga tgaagaggca gttagcccca gtccttacca tttctgcata ttccacctgc 2820
tccccctcat ggtacagctc tgggggcagg ttataaattc gcaagatgtt atcagcacta 2880
ttggtcaaga tgcaggaacc gtcaggagcc ctagaaacag gggagagtta gaaagctggc 2940
cagacctatg cttttcaagt gtagggctag ggctgagcct gcctctgggg taggtaagcc 3000
cccctgaatc cttgagggaa gtagaagaca caaactgcta gataaaatgt aagctcagtc 3060
taaaagggct acgtgccgct tctcccagct ctggggcatc cctctcctag aaaactggac 3120
tgttttacag tgaaaatctc gggggtggtc agctccctgc cccgttgtta tccttaccac 3180
ttacagcctt tcaagaagtt ctcaggttgg gtgctgaact ctgaccagga accactgaga 3240
aatcgaggca gctgggagaa gctgtagttc caagcgctga aaggaagatg ggggacaata 3300
aacctgggtc gccaagcaaa gggggcagag gcctggagaa gtgggtctca ggaccagagg 3360
acagatcgac ctcacacttc atctccccag actccacact ccactgccat caccacttac 3420
gtgtctccct cgtcctctgc agcgggttcc ccagaggtat cttccatggc ttttccagac 3480
cccaactctg gcccgttcgc ttcttcttca gaaaggctcc cgtttgcttc ttctgcagga 3540
aggcttgtat tttcagaaag ttcttgctcc tcgattcgag gactcaactc actaggggaa 3600
ccaaactctg tttccagggg agtggagaga gaaactgggt ccccctcccg tagctcctgg 3660
gacacagctg agccagccac aggatctggg gacaaccggg gcggatcccc cctttcggga 3720
ggcggtggca tcagttcaga gtccgcattt ttattcatcg gggaagcgtg gggagaagga 3780
tgggctggag ctgggtcctg gtctgaagga cagcagtccg gagctaacgg ttgagtctcc 3840
aaagtcttca tactgcagag gaagcacagc ggagattagc ctcagccagg atggcttcga 3900
agttctcagg gatccgacgc agagctaaag aaacccacct gtgcttccct cctcttctgg 3960

gagtaggcag aagactcccg ggaggagagg cgaacagcgg acgccaattc ttttgaaagc 4020
actgtgttcc ttagcaccgc gggtcgctac gggcctcttg ctgtcgcggg atttcggtcc 4080
accttccgat tgggccgccg catcccggat cagatttcgc gggcgaccca cggaacccgc 4140
ggagccggga cgtgaaaggt tagaaggttt cccgttccca tcaagcccta gggctcctcg 4200
tggctgctgg gagttgtagt ctgaacgctt ctatcttggc gagaagcgcc tacgctcccc 4260
ctaccgagtc ccgcggtaat tcttaaagca cctgcaccgc ccccccgccg cctgcagagg 4320
gcgcagcagg tcttgcacct cttctgcatc tcattctcca ggcttcagac ctgtctccct 4380
cattcaaaaa atatttatta tcgagctctt acttgctacc cagcactgat ataggcactc 4440
aggaatacaa caatgaataa gatagtagaa aaattctata tcctcataag gcttacgttt 4500
ccatgtactg aaagcaatga acaaataaat cttatcagag tgataagggt tgtgaaggag 4560
attaaataag atggtgtgat ataaagtatc tgggagaaaa cgttagggtg tgatattacg 4620
gaaagccttc ctaaaaaatg acattttaac tgatgagaag aaaggatcca gctgagagca 4680
aacgcaaaag ctttcttcct tccacccttc atatttgaca caatgcagga ttcctccaaa 4740
atgatttcca ccaattctgc cctcacagct ctggcttgca gaattttcca ccccaaaatg 4800
ttagtatcta cggcaccagg tcggcgagaa tcctgactct gcaccctcct ccccaactcc 4860
atttcctttg cttcctccgg caggcggatt acttgccctt acttgtcatg gcgactgtcc 4920
agctttgtgc caggagcctc gcaggggttg atgggattgg ggttttcccc tcccatgtgc 4980
tcaagactgg cgctaaaagt tttgagcttc tcaaaagtct agagccaccg tccagggagc 5040
aggtagctgc tgggctccgg ggacactttg cgttcgggct gggagcgtgc tttccacgac 5100
ggtgacacgc ttccctggat tgggtaagct cctgactgaa cttgatgagt cctctctgag 5160
tcacgggctc tcggctccgt gtattttcag ctcgggaaaa tcgctggggc tgggggtggg 5220
gcagtgggga cttagcgagt ttgggggtga gtgggatgga agcttggcta gagggatcat 5280
cataggagtt gcattgttgg gagacctggg tgtagatgat ggggatgtta ggaccatccg 5340
aactcaaagt tgaacgccta ggcagaggag tggagctttg gggaaccttg agccggccta 5400
aagcgtactt ctttgcacat ccacccggtg ctgggcgtag ggaatccctg aaataaaaga 5460
tgcacaaagc attgaggtct gagacttttg gatctcgaaa cattgagaac tcatagctgt 5520
atattttaga gcccatggca tcctagtgaa aactggggct ccattccgaa atgatcattt 5580
gggggtgatc cggggagccc aagctgctaa ggtcccacaa cttccggacc tttgtccttc 5640
ctggagcgat ctttccaggc agcccccggc tccgctagat ggagaaaatc caattgaagg 5700
ctgtcagtcg tggaagtgag aagtgctaaa ccaggggttt gcccgccagg ccgaggagga 5760
ccgtcgcaat ctgagaggcc cggcagccct gttattgttt ggctccacat ttacatttct 5820
gcctcttgca gcagcatttc cggtttcttt ttgccggagc agctcactat tcacccgatg 5880
agaggggagg agagagagag aaaatgtcct ttaggccggt tcctcttact tggcagaggg 5940
aggctgctat tctccgcctg catttctttt tctggattac ttagttatgg cctttgcaaa 6000
ggcaggggta tttgttttga tgcaaacctc aatccctccc cttctttgaa tggtgtgccc 6060
caccccccgg gtcgcctgca acctaggcgg acgctaccat ggcgtagaca gggagggaaa 6120
gaagtgtgca gaaggcaagc ccggaggcac tttcaagaat gagcatatct catcttcccg 6180
gagaaaaaaa aaaaagaatg gtacgtctga gaatgaaatt ttgaaagagt gcaatgatgg 6240
gtcgtttgat aatttgtcgg gaaaaacaat ctacctgtta tctagctttg ggctaggcca 6300
ttccagttcc agacgcaggc tgaacgtcgt gaagcggaag ggcgggcccc gcaggcgtcc 6360
gtgtggtcct ccgtgcagcc ctcggcccga gccggttctt cctggtagga ggcggaactc 6420
gaattcattt ctcccgctgc cccatctctt agctcgcggt tgtttcattc cgcagtttct 6480
tcccatgcac ctgccgcgta ccggccactt tgtgccgtac ttacgtcatc tttttcctaa 6540
atcgaggtgg catttacaca cagcgccagt gcacacagca agtgcacagg aagatgagtt 6600
ttggccccta accgctccgt gatgcctacc aagtcacaga cccttttcat cgtcccagaa 6660
acgtttcatc acgtctcttc ccagtcgatt cccgacccca cctttatttt gatctccata 6720
accattttgc ctgttggaga acttcatata gaatggaatc aggatgggcg ctgtggctca 6780
cgcctgcact ttggctcacg cctgcacttt gggaggccga ggcgggcgga ttacttgagg 6840
ataggagttc cagaccagcg tggccaacgt ggtgaatccc cgtctctact aaaaaataca 6900
aaaattagct gggcgtggtg ggtgcctgta atcccagcta ttcgggaggg tgaggcagga 6960
gaatcgcttg aacccgggag gcagaggttg cagtgagcca agatcgtgcc actacactcc 7020
agcctgggcg acaagaacga aactccgtct caaaaaaaag gggggaatca tacattatgt 7080
gctcattttt gtcgggcttc tgtccttcaa tgtactgtct gacattcgtt catgttgtat 7140
atatcagtat tttgctcctt ttcatttagt atagtccatc gattgtatat ccgtcctttt 7200
gatggccttt tgagttgttt cccatttgcg gttatgaaat aaagctgcta taaacattct 7260
tgtacaattc tttttgtgat catatgtttt cgtgtttctt ggagaaatac ttaggagggg 7320
aattgtggag gaagtaaaaa gtagctgtat tttgaacttt ttcagaagct ctgagttttc 7380
cagagcggtt gtaccatttt acactccaac tagcaaggta tgggagttat tatggttgtg 7440
ccacagcctt ccggacatta ggtatgtcag tctttctaat gtggtatatc cttgtggttg 7500
taatttacag ttctctattg actaaggatg ttcagcattt tttcatgtgc ctattggcca 7560
ttcgtatttt gtttgtaaag tagctcttcg agtcttttac ctgttatttt ggtttttttg 7620
tttgttttta ttgttcagtt gtgggactgc tttatacttt ctggatacaa gtccttatca 7680
gatccatgag tcgtgaatgt tttcttctga tctgttgcgg gcctatttgt ttgctttaca 7740

gagtttacag aatcttaaga ggagtggatt aatctttttt atgttcagta tttgccttgt 7800
cctgtttagg acatcttttt tttttttttt aaccccaggg tcatgaagat ataatcttac 7860
attttctttt aggaccttta tggtggtaag ttttacagta aggtccttaa gccattaatt 7920
aattcttaaa attaattgtt tatggtgtga ggtgtaggag tcagtctctg gtatctttcc 7980
tgtatggaaa tccagttatt ctgtctccac ttgttgaaat aggcttcctt tctctactga 8040
atgcttttaa ttttaattat tttacagttg gagtataggg ctaccatttt agtgctattt 8100
tctttttttc tttgttaatt tttgagacag ggactcacac tgttgcccag gctagagtac 8160
aatggcacaa tcaaggctta ctgcagcctc gaacccctgg gctcaagcag tcctctagca 8220
gcctcacgag tagctgggat tactccacca cacccagcta actattttat ttttttgtat 8280
tgacaggatc tcactatgtt gcccaggctg gtctcaaact gctggcctca agctttcatc 8340
ccatctcggc ctcccaaagt gctgggatta caggtgtgag ccaccatgcc tgacctctta 8400
gtgctatttt ctatttatct cctctgttct ctgctctctt taaacgttgg aggaagaaac 8460
agtacccatc ttacacaaac tcttcagaaa acagaggaac agactgggcg cggtggctca 8520
tacctgtaat ctcagcactt tggtacgctg aggcagggga tcatttgagg tcgggagttc 8580
gagaccagcc tggccaacac ggcgaaaccc catctctact aaaatacaaa agtagctagg 8640
cgtgcaccat acctgtaatg ccagttactc aggaggctga ggcacaagaa tcccttgaac 8700
ctgggaagcg gaggttgcag tgagccgaga ttgcgccact gcactccagc ctgggcaaca 8760
gagtgagacc ctgtctcaga aaaaaaaaga aagaaagaaa aaatagagga atatttccca 8820
acttgttttc gaagccagga taatcctggt accaaaacca aacaaggaca ttataagaaa 8880
agaaaatata gaccaatatt cctgttagca tagacatgca acagctaacc aattttagca 8940
aaccaaacct ggtaatatag aaaaaaggat aaataggcca gtcgcggtgg ctcacgcctg 9000
taatcccagc actttgggag gctgaggcag gcagatcact tgaggtcagg agtttgagac 9060
cagcctgacc aacatggtga aaccccgttt ctaataaaaa tacaaaaatc aggctgggca 9120
cggtggctca cgcctgtaat cccagcactt tgggaggccg aggtgggcag atcacgaggt 9180
caggagttca agaccagcct gaccaatgtg gtgaaacgcc atctctacta aaaatacgaa 9240
aatcagccgg tgtggtggca cctgcctgta atcccagcta ctcaggaggc tgaggcagaa 9300
ttgcttgaac ccgggaggca gaggttgcag tgagccaaga tcgtgccact gcactccagc 9360
ctgggcgaca gagcaagact tcatctcaaa aaaaaaaaaa attagctggg catggtggtg 9420
ggcacctgaa atcccagcta ctcgggagtc tgaggcagga gaatcgcttg aacccaggag 9480
gcagaagttg cactgagctg ggatcacacc attgcactcc agcctgggca acagagtgag 9540
actccatctc aaaaaaagaa aaagaaaaag gataaataca ttctaaccaa ataatgttta 9600
tctcatgatt gtagctgatt caacattcaa aaattggcct ggtgcagtag ctcaggcctg 9660
taatcccaac attttaggag gctgaggcag gaagatctct tgagcccagg atttcaagac 9720
cagcctgggc aacatagtca gactggtctt tactgggggg aaaaaaatca gtctgtgtaa 9780
ttcaccacat taacaaaggg aaacataaaa accctatgat catttcaaca gatgtagcaa 9840
aagcagttaa tgatatcaac acatatgcat gattacaaac caaccaacct cctagcaaac 9900
tagggaaagg aaacttaact agtttgataa cagggcgtcc acagtcggag ttccactagc 9960
agcatacata atggtagaaa actcagtgct gctgggggcg gtggctcacg cctgtaatgc 10020
cagcgctttg ggaggcctag gcgggcggat cacgaggtca ggagatcgag actgtcctga 10080
ctagcatgct gaaaccccgt ctctactaaa aatacaaaaa caaaaaatta gccgggcatg 10140
gtggcgggcg cctatagtgc cagctactcg ggaggctgag gcgagagaat ggcgtgaacc 10200
cgggaggcgg agcttgcaga gcctagatcg tgccactgca ctccagcctg ggtgacagag 10260
tgagacttcg tctcaaaaaa aaaaaaaaaa aaaaaagaaa agaaaactca acgctttttc 10320
ctctaagatc aggaactaga aaaggatttg actctcacaa cgttgatacc atactggagg 10380
ttttaaccag gcaagaaaaa gaaataatga gggccgggtg cggtggctca ggcctgtaat 10440
cccagcactt tgggaagccg agacgggtgg atcacgaggt caggagatcg agccatcctg 10500
gctaacacgg tgaaaccctg tctctactaa atatacaaaa aattagccgg gcgtggtggc 10560
gggcgcctgt agtcccagct actcgggagg ctgaggcagg agaatggcgt gaactcaggg 10620
ggcggagctt gcagtgagct gagatcgagc cactgcactc cagcctgggc gacagagcaa 10680
gactgtgtct caaaaaaaaa aaaagaaaaa gaaataatga ttagtggccc gatgtctcac 10740
gccagtaatc ccagcacttt gggaggccga ggtgggcaga tcacctgagg tctggagttg 10800
gagaccagcc tgacaaagat ggtgaaacct cgtctctatt aaaatattaa aaaaatagcc 10860
aggcgttggc cgggtacagt ggctcatgcc tgtaacccca gcactttggg aggccgaggt 10920
gggtggatca cctgaggtca ggagttcaac accagcctgg ccaacatggt gaaaccccat 10980
ctctactaaa aatacaaaat tagccgggcg tagtggcggg cgcctgtaat cccagctact 11040
tgggaggctt aggcaggaga atcgcttgaa cctgggaggc ggaggttgta gtgagccgag 11100
attgcaccat tgcactccag cctgggtgac aaaagcaaaa actccgtctc aaaaaaaaaa 11160
gaattagcca ggggtagtgg tgaacgcctg tagtcccagc tactcaggag gcagaggcag 11220
gagaatcact tgaaccccgg aggcagaggt tgcagtgagc cgagattgtc ccattgcact 11280
ccagcctagg cgagaagagc aaaattccat gtcaaaaaaa aaaaaaaaaa aggaaagaaa 11340
aaaaataacg attagaaagg aagaaatcaa acacattcac agccagtatg attctataca 11400
taccatggtc ctaatggggc caggcgtggt ggctcatgct gtaatcctag cacttttagg 11460
aggctgaggc aggtggcttc cctgggacca gctggccaac atggtgaaac cccaactcta 11520

ataaaaatac aaaaaatcag ccaggcgtgg tgagggcacc tctaatccca gctactcagg 11580
aggctgaggc aggagaattg cttggacctg ggaggcagag gttgcagtga gccgagatcg 11640
cgctattgca ctccagcctg ggcaacaaga gtgaaactcc ggcagggtgt ggtcttacgc 11700
ctgtaatccc agcacttcgg gaggctgagc caggccgatc acctgaggtc aggagtttga 11760
gaccaaccta acatggtgaa accccgtctc tactaaaaat acaagaatta gctgggtgta 11820
gtggtgggcg cctgtaatcc cagctacttg ggaggctgag acagaagaat tgcttgaacc 11880
caggaggtgg aggttgcagt gagctgagat catgccattg cacaccacgc cgggcaacag 11940
agcgagattc cgtctcaaaa aaaaaaaaaa gatgaaactc tatctcaaaa aaaaaaaaaa 12000
gtcctaatgg aaaatccata aaaagctacc aaaactaata aataaatata gcagggttgc 12060
aggttacagg gcaatatagt tatccctcta tctgtagggg cttggttctg ggactcctca 12120
cacaccaaac ccacagatgt ctaagtccca tatataagac ggaatagtat ttaacctaca 12180
catatcctcc catatagttt aaattatcta gattacttac attaccccca tacaatgaaa 12240
atgctaatgt acatgcaagt atgtatgtaa gtacttgtac tatattgttt agggaatcac 12300
tggacagata ggccttcaag actgatacca gcagccactg ttaagattct ggtcaggcct 12360
gcccctgttt ggggtctcag ttgatctcat tgccttccca cccagccaag ggcacctgca 12420
tttctcttgg ctccctggcc atttggaagg cctagttcag cctggcacat ttgtatcctg 12480
gcccactgat gctggtaccc ctgggaaggt cctgctctga aaaacacgga gattttagtt 12540
gctactgaag atttgagaga taaagacagg gagacctgtc tgtagacctg tgtccctcca 12600
agtgggattg agactttggg ccccccattt caggacagca cctcctggcc tgttgactga 12660
atagatccct gaaggaggtg tagttgcatt ttaggagtgg gggtgggagc agtaccactg 12720
atccgcacta acaatcacac agttctctct agaataataa tatagaacaa gtgaaataga 12780
acaattgcag aaagagctaa cctttgttga gctcttactg tgtgcccagc actttcctca 12840
actctacatt tcccataata catagagtac taggtaggcg gggcttgggg gctcacgcct 12900
gtaatcccag cactttagga ggccaagggg ggtggatcac ctgaggtcgg gagttcaaga 12960
ccagcctgac taacatggtg aaaccccgtc tctactagaa gtacaaaatt agccaggtgt 13020
ggtggcacat gcttgtagtc ctagctactc agcaggctga ggcaggagaa tcatttgaat 13080
ccgggaggag gttgcagtaa gcggagatag tgccactgta ctccagcctg ggcaataaga 13140
gctgagactc cgtctcaaaa taaaataaaa taaaataaaa taaaataaaa taaaataaaa 13200
aaagaaaaga gcctgccatt aaaggagctg tttggtaggg gatgttttgt cagtgcaaac 13260
aacagaaaag tgggctgggc acagtggttc atgcctgtaa tcccagcact ttgggaggcc 13320
aaggcgggcg gatcacctga agttgggagt tcaagaccag cctgaccaat atggagaaac 13380
cccgtctcta ctaaaaatac aaaattagcc gggcgcagtg gccgatgcct gtaatcccag 13440
ctactcggga ggctgaggca ggagaatcgc ttgaacctgg gaggcagagg ttgcggtgag 13500
ccgagatcgc accattgcac tccagcctgg acgagagcaa aactctgtct caaaaaaaaa 13560
aaaaaacaga aaagtgtaac aaacacttac agtaggcatg tttcttagca aatctgatga 13620
caaatttggc ataaagaaag agagcatccc tgaaaaaaaa aaaaagaaaa agaaagagag 13680
catcctgcct gggcaacata gtgaaaccct gcctctacaa aaaaactcaa aaattggccg 13740
ggtgcagtgg ctcacacctg taatcccagc actttgggag tcggaggcgg gaggatcacc 13800
tgaggtcagg agttcgaaac cagcctggcc aacatggcaa aaccccatct ctactaaaaa 13860
tacaaaaaat taatcaggcg cattggtggg cgcctgtaat cccagctact caggaagttg 13920
aggcaagagg atcgcttgat actgggaggt ggaggttaca gtgagtcgag atcacaccac 13980
tgcactctag cctgggtgac agggcgagac tccgtctcca aaaaaaaaaa gaaaaagaaa 14040
aagactaaaa aattagccag gcaggcctct gtggtcccag ctacttggga ggctgaggca 14100
ggagaatcac tgagcccagg agtggcaggc tgtagtgagc catgattgca ccactgtacc 14160
ctagcttggg cttcaaagca agaccctgcc tcaaaagaaa aaagaaagaa agaaagaaca 14220
tggcgggcca ggcacagtgg ctcacacctg taatcccagc gctttgagag gccgaggcag 14280
gtggatcaca aggtcaggag ttccacacca gcctggccaa catggtgaaa ccctgtctct 14340
actaaaaata caaaaaatca gcaggcaggg tggtaggggc ctgtaatccc agctactcgg 14400
gaggctgagg caggagaatt gcttgaaacc agaaggcaga ggttgcagtg agcctagact 14460
gcaccactgc actccagcct gggcgaaaag agccaaactc catctcaaaa aacaaacaaa 14520
aaaacaaaac aaaaaaaaac atggcagcct ttgaaagctt gtctgggaga aggtgcgatg 14580
atggttgcat aacttcgtgc aagatgctgg tccacacagg ggctgcccct tgctctttct 14640
cgctctctta acctctcata taacaggctt gtgtgttatg cacatttatt gagcccaagc 14700
aggtgcaagg cattgtgatc taatactttg gtcagcaaga caacaagata gatcactgcc 14760
ctgcccttag gaagtgtata tgctattaga ggaaacagat aaaataaaca aggaaaagta 14820
tcagacaatg taagtgctat gagaatgcaa atgaggtgat gtgaattaaa ataggatgac 14880
ttaagtctgc acggaaggcc cctaccccca tgttcctggc tagccaagga accaccagtt 14940
gattagcaga gaagggcagc ccgtctagct agagcttttg ggaagagggg agtggttgtt 15000
aagagatgag attaaagaag ccgagacggg cccttcgtga gggggggttg taatgcaggg 15060
ctgaggagtg tccgaagaga atgggcaggt gagcggtgag acagttgttc ttccagaagc 15120
tttgcagtga aaggaatcaa agaaatggag ccgtgtatca ggtggggaag ggtgggggcc 15180
aagggggtgt ccttccccat acagagattg caggctgaga atgactatat ccttgttaac 15240
aggaggtggg agcagggcac ggtagctcac acctgtaatc ttggcacttt aggaggcgga 15300

ggcgggccga tcacctgaag taaggagttc gagaccagcc tggccaacat gcaaagccct 15360
gtctctacta aaaatacaaa aattagctgg gtgtggtggt actcgcctgt aatcccagct 15420
actcgggaga ctgaggcagg agaatggctt gaacccggaa ggtagaggtt gcagtgagct 15480
gagatcatgc cactgtgctc cagcctaggt gacagagaga gactccatct caaaaaaaaa 15540
aaaaaataca ggaagggagt tgggaatagg gtgcacattt aggaagtctt ggggatttag 15600
tggtgggaag gttggaagtc cctctctgat tgtcttttcc tcaaagaagt gcatggctgg 15660
tgtggggtgg ggcaggagtg cttgggttgt ggtgaaacat tggaagagag aatgtgaagc 15720
agccattctt ttcctgctcc acaggaagcc gagctgtctc agacactggc atggtgttgg 15780
gggagggggt tccttctctg caggcccagg tgacccaggg ttggaagtgt ctcatgctgg 15840
atccccactt ttcctcttgc agc 15863

<210> 46
<211> 6077
<212> DNA
<213> Homo Sapiens

<400> 46

aaaatatgag aaagcaagca cctgacattc tccactccaa aagagggggg ccttcggaat 60
gctactgccc tggccttcag tgactgtggc cttctctcct gccagaggtt tcatgtctag 120
atcaccagtg acatccccag cccctaaaga tcagttcagt tcatacaagg gtaatgttga 180
aatatacaag ccaggtttta atattgctat actctattgt aaagtataaa agtatgtttt 240
aactcgctac ttaaagcttt aagacatttt taagtccctt cttatctgta tgttaaaaac 300
taaacttaaa tgggaagtag cattgggaga aagctcttct gtgaactaat aaccaaaatc 360
ctctgctagt tttataaaca cttttggaca atttcctcaa atttataaag ggtaagaatt 420
gaggcttcac agatgacaac gaaaatggca aggaatgcat caatcttctt atgcagtgcc 480
gaactcagag ccaaaacaac agaagatcaa aagacaggct ttcacttctg cacacataaa 540
gatgcttcgc aaatgctcat ttatctccat taacctattc ctaacacgaa acgggaaatg 600
tttaagtgtt agtactattt tcttaaagat agtttcttct tgtccatatt tgttcattat 660
gtccctatcc aagcaaacaa tttcaagtaa aaataacgcc tgagtactca cctgctgaat 720
gtatatttta ctacagtttc aagatcaaga actgatgttt aaatattaac tgcttcgtta 780
gttttactta tcaaggtcaa agtagtttgt gttttatgaa ccgatatcaa aaaagaaagc 840
cacgttagtt ttccgtggga gaaaagagag acaggcgggt gacggcagat tggaacaagg 900
agctaccaca gcacttactt tctcacactg tagctttgaa aactgtgatc agttacagtg 960
aattccttat gaaatggaat atttttacca acaccttgag gaaccacata aactctactg 1020
caagatatga aaactagaaa catggacctg ctctccccag tcctcagggc tttcaagaag 1080
atgattccag taacagatca ttaccaagtg tttcagggta ttttgaccag agtgtacaag 1140
ctggaaaaaa acacccaagt taagcaagca gatgtaatct ttctccttat aattcactga 1200
aattttcata aatggtaaaa acaaaaaaca aacctctcat ttaattgagg aggggggcac 1260
attgaaaata taccaaggct gagatgccat ttaaactaat gaggttttct gttttgtagc 1320
tgatgctatt ttatgtgatt tggaataaaa cctccctgaa gaggtaaaaa acgtaatttg 1380
agagttaaac tctcaaaagc tagacagagc acaggaatgt caggagtggc aaaacctgaa 1440
aaagcatcat gatacatcag tcagttagta caggtggtgg ataaggaaaa cgaagcccac 1500
tttttacgag gcatgtttct tcttagtggc tttccaatgc caattgtccc cctcttctga 1560
ggacatacat actaggcatc cgggttatcc ctctcttctg aggatataca ctactaggca 1620
tccaggtaca tacactatat gttttgaagc ttaaagttat agctctgtga tttaagcacc 1680
cttctgccac ccatggaacg aagacctcaa atcccagcca tgaggacaac tacttcctta 1740
cctggggata gaatactagt atttaaatca tttattcggc atgtggtaga ggagaagaga 1800
attagaggag aagtagagat gacaaagtag ccacaccact taccagttta caggcaacag 1860
aatcatcaat ttgccttttg tgacaaagta acaacaaaga gccgacatct cctataccct 1920
cacctgtgtg cagtcggcac tgccgatacc cacctttcca aggcacctcc cccagacccc 1980
ccacctgtct acggtatctt cctgggtccc gcacctgccc gggcacctgc cggggtcctg 2040
cacctgtccg gcccccatat ctgcttgggg tacctgcctg ggccccgcac ctgcttgggg 2100
tacctgcctg ggccccgcac ctgcttgggg cacctccccg ggccctccac ctgtctaggg 2160
tatcttcctg ggtcccgcac ctgtctgggg cacttgctca gacactgcac ctgccccggg 2220
tacctgcccg ggccccgcac ctgtccgggc accgcacctg caggatcccc aagctgcctc 2280
cacccacgcg gccgcccccg gccctgcccg ccggggacgc tggcaccgag gatgtcctgc 2340
ccgtggccca ggtccccgcc gctcaccagg tacttgccgt ccggggagaa cttgcagagt 2400
aagctggaga gcttgaatac ctcggagaag ttcatggccg ccgcctgccg cgggcgccac 2460
cctgcgcccg aaaacccgcg ggacccctgg gcgcgcagca ggctgcaaca gccgacgccg 2520
gcctccgagg ccggaagtca gaaggcggaa gtgaactgca gcctatcagc gccgccggct 2580
tccgcgcggc attgtggggc ttgtagttct tgtgccgcag ggctttaaag gaaacgccca 2640
cgtttcttcc gaccagggat ttccgacccg agaaccttac ctcaaaggcc gggaggcctt 2700

tgagcacctc cagctagggc tgctgataaa aatgtagaaa gcacagtaaa atttgaattt 2760
cagattcaca acaaatctag ttataagtat gttcccaaat attgcacggg acatgctaat 2820
acggaaaaat tactcgctag tctgaaattc aaatttaatt gagcgacctg tgtgtctgcg 2880
tgtgtgtaca catgcatata tatatattta tatttatatg taaatgtatg tttacatgta 2940
aatatatgtt tacctacaaa tatatcttta ataagtaata cggtgtctgt cgcacatata 3000
ttatatcgtg tatgtaatgt ataagtattt atttcgtttg cttggggttt tgtttgcttt 3060
tgctgagtcc gacccctcta cctgccgcct ggcccttgcc tcacgctcca gtgccactga 3120
gatcaaggag agaacgaatt tgccgctgac tgggcagagc gagcgcgtgg atcgcggcca 3180
ccgcccgttc atcacccgcg cgcatctggg ctggcaccgg gcgaagaatc gtgcgggtct 3240
gggacctggg ggcccagagg gagcgagctc ctgcgcgggc gctcggtccg caggtttcgc 3300
aggctcaggg gcgtgcctcg ttctcacccc cactccggac cccggtcctc ttccctagac 3360
agcggccccc tccacccctg gctcccgcag gccgctagta gtccgcgcca ggccccgccg 3420
gcgcctctag ggccccccag atcgcgcaga ccctgacatc cccgcctggc cctgggttct 3480
gggagctgag agccggccag ggtcctgctc gtacctccgg gcgcccagcc tcgggtctgc 3540
tccccgcgga cgccccaacc tccccggccg aatggatggt ggtgcgcgcg cgtcctactc 3600
cggcggtgcc ggccttttct gttgccaaaa ctagacccaa acctctgcat gggattcgtc 3660
tttgggtccc caccccgtgc gcccagcaaa cagtgggtga gccatgaaga tgtgcgagtc 3720
agccggaccc tccccgtcag gcgcggaccc gctgcggcca gagaacccag tctgcgccag 3780
cccggctcgc tcgcgaagcc acgggcttca ctgacgcgac tttccaagac gtgggggtca 3840
ccatgggcag aggacatcgg ttcggagcca gatcacgggc cccataagca tcagaccata 3900
agcagcgccg ccactgagag ccgctcggaa ctcgcccagc atgtcgggtc ccctagccag 3960
ggcctggtgt acgtggtcga gggccctgga agccccgatg gcctaggagg agcaggcggg 4020
cggggcggcg ggtgtcgctg gccggtagag agcttcggcc tgacctagcg caggtctggt 4080
gcgcgcagag aacaactcca agcgcaccga cgcccgcgag ctccttccaa acaccgaacg 4140
ggatccagag cccgagccca caggcggcgg ccgggggagg gagcagggtg ctggccgccg 4200
cccgggagtg ttcgcgtcct gggtgacccc tggaaggacg tggggcccaa actccggctg 4260
gggttgggag agcagccccc agaggctctc cgcgggatcc tctgccgggc gggaccgtgg 4320
ctccacagga gaagtgggtg gcaagccctg cttggcggaa agcagccgtt cccctcctcc 4380
tgggcctggg gcggcgcccc tcacccctgt tccccgcccc tcacccctgt tccccgccgg 4440
ccacatcccc tgccccttgg attccaagcg ccccgcgcgc cgaggagccc agcgctagtg 4500
gcggcggcca ggagagaccc gggtgtcagg aaagatgggc cgtctggggg acagcaggga 4560
gtccggggga aacgcaggcg tcgggcacag agtcggcacc ggcgtcccca gctctgccga 4620
agatcgcggt cgggtctggc ccgcgggagg ggccctggcg ccggacctgc ttcggccctg 4680
cgtgggcggc ctcgccgggc tctgcaggag cgacgcgcgc caaaaggcgg cgggaaggag 4740
gcggggcaga gcgcgcccgg gaccccgact tggacgcggc cagctggaga ggcggagcgc 4800
cgggaggaga ccttggcccc gccgcgactc ggtggcccgc gctgccttcc cgcgcgccgg 4860
gctaaaaagg cgctaacgcc cgcggccgcc tactccccgc ggcgcctccc ctccccgcgc 4920
ccatataacc cgcctagggg ccgggcagcc cgccctgcct ccccgcccgc gcacccgccc 4980
ggaggctcgc gcgcccgcga aggggacgca gcgaaaccgg ggcccgcgcc aggccagccg 5040
ggacggacgc cgatgcccgg ggctgcgacg gctgcaggta ggaggcccag ggccgggggg 5100
cggttcggct ccgcgggcgg gggctggagc gcagcgctgg gcaggcacct gggctcgcag 5160
ctccgaagct gggaggtgag gggagagcga tcggggacga gctgggacaa ggcgacacag 5220
gggctccctc ggagttggat cggcccctgg gacttggcgc tcgcgagagg ctggagcggc 5280
cagagtctag cctgcgagga gacgcgggtc ctgccctcag cgccggccgc ctttggcgcc 5340
aaagacagcc ccgcaggggt tccgggaggg ccctcctcct gctgtcccct ctccaccccg 5400
ggctccgagg gccgttggga gggtaacccc gggaagaggc cggggtgcgg ggcgcgggtg 5460
caggtggaaa tcgccagcaa gctcctcccc gcccgcgcgc tccctccgac ctgcagggct 5520
gtgccaatcc cgaggcctca gcttccctga ggagccaggg ccaggccccc ctctggacag 5580
ggagaaggat ctgggcgggg gccttgaccc atggagttgg ttactaagcg gtttcgatgg 5640
tttcccgagg gacagctccc tgtggctctg agtttgtctg tcgagggctc ctggcctgtc 5700
tccggagcgg tcccaggtag agaaagcccg tgaagaaatg gcccgggccg gcctggaggg 5760
agacacctca cgccccctta gctcctgggc cgcctcctcc tgcagcccct gcctttcccg 5820
gggcttggac ttggggagcg atgattacct ttgctcagct tgtattttgg cctggacgct 5880
aggagataag cccatgtagt atgcacacgt ctgctacata aacaggggac agatagacga 5940
tcttcaacca gcaagggtgc agggaaaagc aatgcacccc aaacttctga ccagaggtca 6000
tttgcttcca aagatgctgc catctgttta ttcactgtct ggacatttgg aaatggctca 6060
ggctcattaa cacaatg 6077

<210> 47
<211> 2501
<212> DNA
<213> Homo Sapiens

<400> 47

agctgggact acaggtgcac gccactatgc ctggctaatt ctttcatttt ttatagagac 60
aggacctcgc tatgttgccc aggctggttc caactcctga actcaagtga tccacctgcc 120
ttgggctccc aaaatctcgg gattacaggc gtatagtgct aggccttcac attcttatat 180
acccttctgt ttccttagct gtgaaatgga gataatgaca tagccttaat tcacgtggga 240
tgcttagcac agtgtctggc acttgataga tttttttctt tctacttgaa atttattttt 300
gagctgggca cggtggctca cacctgtaat cccagcactt gggaaaacca aggcaggcag 360
attacctgag gtgaggagtt caagaccagc ctggcaaaca tggtgaaacc ccgtctctac 420
taaaagtaca aaaattacct gggtgtggtg gcacacacct gtaatcccag ctactccaga 480
ggctgaggca cgataatcac ttgaactcgg cagatggagg ttgcagctga gctgagactg 540
agccactgca ctccagcctg ggtgataaag cgagactgtc taaaaaaaaa ttatttttaa 600
cctaatataa aactctctga acccttcttt ttctgagctg cttctccact tgagtccatc 660
ccacatcatt gaacagttaa tcacagaatt gtgtaccatc ttccttgacc cccagatctt 720
tagccttcta tagtaccctg tctctttttg tcgtactagt cacagttgca cttttttttt 780
tttttttttt gagacagagt ctcattctgt cacccaggct gcagtgcagt agcacaatct 840
cagctcactg caacctccgc ctcccgggtt caagcaattc tcctgcctca gcctcctaag 900
tagctgggat tacatgcgcg caccaccacg ccaggctaat ttttgtattt ttagtagaga 960
cgaggtttca ccatgttgtc aggctggtct agaactcctg accttgtgat cagcccactt 1020
cagcctccca aagtactggg attacaggca tgagccactg cacccggcca tagttgctat 1080
tttatttgtg tgattgatta ccgtatgacg cttttattga agtgcagtga aggcaggaaa 1140
catgaccggt ttttacaata ttttcaacac ttatcaccat aggtggtaca tagtagggcc 1200
ttaataaata tttgtaaaat gaacaaagtt aatgagtgtt tatgtttgta gttttaattg 1260
ctctgaagta aatatctgat tttccaattt ccaccagagt gctctgcaca tagtaggtct 1320
aattattttt ccctctttac taatcaccca tgccttgtaa gaattcagtt agttgacttt 1380
ttgtacttta taagcgtgat gattgggtgt tcccgtgtga gatgcgccac cctcgaacct 1440
tgttacgacg tcggcacatt gcgcgtctga catgaagaaa aaaaaaattc agttagtcca 1500
ccaggcacag tggctaaggc ctgtaatccc tgcactttga gaggccaagg caggaggatc 1560
acttgaaccc aggagttcga gaccagccta ggcaacatag cgagactccg tttcaaacaa 1620
caaataaaaa taattagtcg ggcatggtgg tgcgcgccta cagtaccaac tactcgggag 1680
gctgaggcga gacgatcgct tgagccaggg aggtcaaggc tgcagtgagc caagctcgcg 1740
ccactgcact ccagcccggg cgacagagtg agaccctgtc tcaaaaaaaa aaaaaacacc 1800
aaaccttaga ggggcgaaaa aaaattttat agtggaaata cagtaacgag ttggcctagc 1860
ctcgcctccg ttacaacggc ctacggtgct ggaggatcct tctgcgcacg cgcacagcct 1920
ccggccggct atttccgcga gcgcgttcca tcctctaccg agcgcgcgcg aagactacgg 1980
aggtcgactc gggagcgcgc acgcagctcc gccccgcgtc cgacccgcgg atcccgcggc 2040
gtccggcccg ggtggtctgg atcgcggagg gaatgccccg gagggcggag aactgggacg 2100
aggccgaggt aggcgcggag gaggcaggcg tcgaagagta cggccctgaa gaagacggcg 2160
gggaggagtc gggcgccgag gagtccggcc cggaagagtc cggcccggag gaactgggcg 2220
ccgaggagga gatggaggcc gggcggccgc ggcccgtgct gcgctcggtg aactcgcgcg 2280
agccctccca ggtcatcttc tgcaatcgca gtccgcgcgt cgtgctgccc gtatggctca 2340
acttcgacgg cgagccgcag ccctacccaa cgctgccgcc tggcacgggc cgccgcatcc 2400
acagctaccg aggtacgggc ccggcgctta ggcccgaccc agcagggacg atagcacggt 2460
ctgaagcccc tctaccgccc cggggtccat tttgcagacg g 2501

<210> 48
<211> 2401
<212> DNA
<213> Homo Sapiens

<400> 48

tcatcaaaga aaaaaaagat gacttactag acagttgtta aggcactcac ataaaactac 60
acgatagcag gatagatggt aaccaagagg gaactcagga cccgtcacta ccagctggaa 120
acacctgacc tccagcctgc aaatgtgaca agctagacca acaccagcag gacactgaac 180
acagcttgca gggatgagtc tctgcgtcat ttctggacat gtgtccaagt aaccagaaca 240
actcaaaatg ccaccaggcc cttgctgtca ttcccataaa cctcgaacag tgagttgggt 300
cttcaaggaa cctgtctctg tcagcttacc ttatgctgca taacaaacag gcccaaagct 360
cttggcagcg tacatcaagc atggaggatc tctcactcac cacgacagtc ccttcgctgg 420
agggctgggg ttgtactcca ttttctcttt tggaaggggc ggcccctgtc tgaggtctca 480
tttgtctcac agcagaggga tgaaacggca ggaccctggc tttgacgggc tccatttggc 540
aatggtacat gccaagtggg tcacgtttca ctgcccagag caagttacat ggccacgtct 600
gatattgacg tgcgggaaat gcagaatctt cctgcatttg ggaggcagat tatttggaca 660

acatgcaatt cattacaggg ccagctggat gaagaacggc caagggccca ggctcttcac 720
cccgagccag gccgaaccag ccggggccgt gagatcaagc cacggtcatc agtgttcgtt 780
tggaattatg ccttgtcctt gccgtgtcca cattgtagta gggtttctgt tttttaattc 840
atctgaaatt ggtggtgggt gtggtacaca gatgagaaac aggaactctc cacgctgggc 900
tttaacagga gctgcggtgg gaacacaaat ggtgcagcca ctttggaaga tagtttggtg 960
gctccttaca aaactaaatg tacccttcac atatgatccg ccacacacca tccagcaatt 1020
gtaccccttg gtatttgccc agatgagtca aaaacttgca tccacacaaa aacttgcacg 1080
tggatgttga tagcagcttt attcataatt gcccaaacct ggaagccttc aggaggtgag 1140
tgggtaaaca aactgtggtg cctccggtca gcggaatatt attcagtgct aaaaagaaat 1200
gagctcaagt catggaaaga catgaaagaa ccttaagtat tattaagtca aagaagccaa 1260
tctgaaaagg ctgatgctgt atgagttcac atctatgaca ttatagaaaa ggcaaaacta 1320
caaagacagt aaaaggatca gtggttgcag gagctaggga ggagggaggg atgaataggt 1380
ggggcacaga ggatttttag ggcagtgaaa ctaccttgta tgatactgta atggtggaca 1440
caggacagta agcatttgtc ctttatcccg tagaatgtac ggcactgaga acggaccctc 1500
ctgtaacata tggactttca gttataatat tgaatccctt tcggggttcc gtaccgcccg 1560
cagcgccctt taactcccgc ccccgccccg ctcaccgccc ccttcccctg ctacagcgcg 1620
ctcgccccgc ggccgccaat cagccgcgcg ccccggccgc gcgccccgcc ccgcccccgg 1680
tgggtgtgcg cgcggccaat gggcggtgcg cgggggccgg gccgcggcgg ggcggggcag 1740
cggggcggcc gccaatcgcc gtggtgttgt tgaaactgaa aatactacat tatgctaatc 1800
gcggccgggc ccgcgcgcac gggggtgggg cccgcgcgta taaagggggc gcaggcgggc 1860
tgggcgttcc acaggccaag tgcgctgtgc tcgaggggtg ccggccaggc ctgagcgagc 1920
gagctagcca gcaggcatcg agggggcgcg gctgccgtcc ggacgagaca ggcgaacccg 1980
acgcagaaga gtccaccacc ggacagccag gtagccgccg cgtccctcgc acacgcagag 2040
tcgggcggcg cggggtctcc cttgcgcccg gcctccgccc tctcctcctc tcctttcccc 2100
ttcttctcgc tgtcctctcc tctctcgctg cccgcgtttg cgcagccccg ggccatgtcc 2160
gacgcgtccc tccgcagcac atccacgatg gagcgtcttg tcgcccgtgg gaccttccca 2220
gtactagtgc gcaccagcgc ctgccgcagc ctcttcgggc cggtggacca cgaggagctg 2280
agccgcgagc tgcaggcccg cctggccgag ctgaacgccg aggaccagaa ccgctgggat 2340
tacgacttcc agcaggacat gccgctgcgg ggccctggac gcctgcagtg gaccgaagtg 2400
g 2401

<210> 49
<211> 1508
<212> DNA
<213> Homo Sapiens

<400> 49
   attctacaat tataaacatt tagtgtattt tgcaaatatg gcataacctg ttggcataaa 60
   attccattgt tccagaaaat atcggtaata aaattataga aaagttaaag atcttcattt 120
   cttatttcga agcgtttggg agacatttca gaaacggatg ggaaatgtta aattctgcat 180
   gcctgcttat ttttctttcc acaccgacta gatgtaaacg agtgtcacca aaagtacacc 240
   acaggcaccc acacagattc cttccataag ggatccacaa agtttagatg tgaaatgtac 300
   cctaaagggt tcctagccgt ctctcatccc tccctctgtg aaacagggaa gacacatgtg 360
   ttttcaaggg cagagatgga acttggccca atggccgggg gggtgggggg aagttggaag 420
   ggactgcctt aggacatggc aggatagtgg attgtttctg ccgccttgtt gcccataccg 480
   gggctctctg caggcgcgtc ggctccctcc ccccctgctg agaagaagcc ctgccaaaac 540
   atccgcctcc cccgggaacc cccccgtggg ttcaaagcgg gaaaatgttg cctcaggttt 600
   aaaataatct gtccaagccc cccagcgcgg gagaaacgtt cttactcgct ctctgccccc 660
   gggggtgctc cccgccctct gctgccagaa ccttggggag tgcctagacc cggggcagca 720
   cacgtccggg ccaaccgcga gcagaacaaa cctttggtgg gcggccagga agctccctcc 780
   cagccaccgc ccccctccag tgcctttttt tccccccata caatacaaga tcttccttcc 840
   tcagttccct taaagcacag cccaaggaaa cctcctcaca gttttcatcc agccacgggc 900
   cagcatgtct gggggcaaat acgtagactc ggaggtaggc atccgtgggg gggcgccggc 960
   tcgggcgtgc ggggaagtgt ccgcttctgc tatctgcctc tccaaatatc ccgactgctg 1020
   atctggcccc ccagccctct ttccacttcg gagcactcct ctggcgttgg caccgctgaa 1080
   gaatgggcct gggcggggag gtgaagagaa gccaggaatg ttttatgttt tcctaatgga 1140
   gagggggcct cgggagcccc tgagctagga ggacacggaa aaggggattg gggtcctgag 1200
   attgggtctg ttgggcccaa gacgcgtttt ctggatgggt ctaggatgct cccctgtcgc 1260
   gggaaccccg cggtccggcc ctgcctgctg ggggttcgaa aaagtggatt gcaggtggga 1320
   aggtgttatt tacccgagtc ctggggacag tccccgggac tctccgccaa gcgcccaaaa 1380
   cggcaggtcc ccaagcggcg cgcggtgttt ttgcactttc caaaattctt gaaacatctc 1440
   aaaaatcctt ctgcatcttg gcgtctggca gggttttcca aaaaaagtaa acctccctcc 1500
   caaatgca                                                              1508

<210> 50
<211> 1984
<212> DNA
<213> Homo Sapiens

<220>
<221> unsure
<222> (9, 17, 18, 33, 41, 49, 50, 52, 54, 62, 80, 84, 127, 157, 159)
<223> unknown base

<220>
<221> unsure
<222> (175, 207..209, 214, 1181)
<223> unknown base

<400> 50

agcttcagnc atcactnnca tctttatcaa tgnccttggg nccacaggnn gngnccttct      60
gnccaaacag cagccaatgn aggnatctta aaaggcacat cactttatat ttattcagtg     120
gttatgncat cacttaatcc tcaccacaac cttgtgngnt cagtacaatt tgtgntttca     180
ttttatctgt gaacaaatgg aggcagnnnt ttgngtaatt tacaagtaag tggcagatcc     240
gggggctgtg cctagacaat gtggctccag actccttgcc cctccctgcc ttctgctgca     300
tcttgattga gtctagccat ctatttcttt aacacacact aattgctgtc tccatgttcc     360
aggccctgtg ccagatgcta gaaatacatc cccatccact ggatcttggc atggtccatt     420
gctcagtgtc ctgctatgtt acgtgttaca taatgcattt attccggtat ttagcccatg     480
gaaaataatg ctgaaagaca ttgtatgcat gtttctacaa caaaactatg taacttatgt     540
ttcttttatt gctttggcta tcaggaagct cttatccaaa tcagagcaaa tacattagaa     600
tttgggcttg tcatttcagt ttgctgaact tttccttctg gcccagattt tctattttgg     660
ttcataaatt ctattgcaca aatgtccttt attgtaaaac accttaaatt ctttctaagg     720
gaaggctgca tggaaatgat acagtaaggt cttccctgca ttttcttaga ttcctattag     780
ggaaggcaga cctagatgtc cctcttaccc tcagtcccaa agccccccat ttataaaatc     840
ccttaagcag tgactactgc tgttctgagt acctggaggt agttcagagc ttctgaaagg     900
taaaatccat atacaaaaag aagttccttc cgatccagat cccagctttg gtgccagatg     960
cacatttgag gagtaggtgg ctagtcagac tctcacctga gcagttaata aaatctattg    1020
ccccttaatg gaattttttc tgcaagctcg aattgatctg tcatctttgt gatttgtgag    1080
atggcaggga agcaccaaac accatcatga cttgggccac agtggtggga aaaaaggaaa    1140
aaagaaaaaa aaaatccact gccaagcctt gccaggcgta naaagggctg gaactgctgg    1200
ggccatttta tctgatttat tggaaataga gtggatctta ttaacatttt aataaagaga    1260
atctttttgc actaggctgg aagtggccgc cagtcccccg tgcaattcca ttctctggaa    1320
aagtggaatc agctggcatt gcccagcgtg atttgtgagg ctgagcccca acagtccaaa    1380
gaagcaaatg ggatgccacc tccgcggggc tcgctcctcg cgaggtgctc accccgtatc    1440
tgccatgcaa aacgagggag cgttaggaag gaatccgtct tgtaaagcca ttggtcctgg    1500
tcatcagcct ctacccaatg ctttcgtgat gctgctgctg atctatttgg gaagttggct    1560
ggctggcgag gcagagcctc tcctcaaagc ctggctccca cggaaaatat gctcagtgca    1620
gccgcgtgca tgaatgaaaa cgccgccggg cgcttctagt cggacaaaat gcagccgaga    1680
actccgctcg ttctgtgcgt tctcctgtcc caggtaggga agaggggctg ccgggcgcgc    1740
tctgcgcccc gtttctgcat tcggatcgcc cggcacgggc agggtgaggg ggctttcggg    1800
gggtcggggc ctccggtcgc ggcggcgaag acagatcggg gctcggtagg gaggtcattc    1860
cgagcccaga gatcctaggc accccccaca cacaggctcc cactctggcg tgcgtgcgtg    1920
tgtgtgtgtg tgtgtgtgtg tgtgtgtgtg tgtgtgtgtg tgtgtacgtt cgttaacggg    1980
agga                                                                  1984

<210> 51
<211> 17897
<212> DNA
<213> Homo Sapiens

<400> 51

acattgcaca actcttcatt tatgttcaaa gtaaaggatt tattacacat cctgaacata      60
gcctaccctt caccctcatt tggaattgtt ttcctttcat ggtttttttt ttttttaatt     120

ttcattgctc acaacatgat attacaggcc aggcatgctt gcagataact gggataattt 180
acttttactt aagtaaaaat cccagatggc agtccctcca cctgggatca tgaagcaggt 240
aggatcccct tttccctcct aacagagtcc aagcagtaag accgaataag atgggatgaa 300
ttcaaagaga aacatctgca tttgctcagt tcttcaacca atatttactg aacttctcta 360
cggtcaggtg tgcaagatgc caggagggat gagacaagat gatgatgatg atgatgatga 420
tgatgatgat ggtgatgatg acagtgagga tgagggtgaa cactttaatg agagccttct 480
atatgccagg gactgttcta ggcactttac atatattaac acatatattt atcacaatga 540
ccctgtattg ttatgtgtat cttatacatg aggaggcaga ggaaggaaat gaaataactt 600
gcccaagagc atagagtctg caagtggcaa agataagata gaaataattc tccaagagca 660
gagagtggtg acttctgggg atagggaagt ggaaaagtgg ggagacaaag gctgttgttt 720
ttcctaacaa gttttgaaga cttatttgac caggttcaag aaaaacttga taatcactac 780
aaagtaaaag gaagaggaga tgaggtgggc ctttgggtac agatgcttgt tttggaaagt 840
ttagggagga aggggtgaga ggaactgggc tgcagtacag gaatggggga cttgggaatc 900
agaggggatt ggttagggtg ggtcattgat tgcagtgaat gtttacttgt tgattgggct 960
gcagtacagg agtggggggc ttggaattaa gaggggattg gtttagggtg ggtcattgat 1020
tgcagtgaat atttacttgt tgattagttt gatccagaag aaagggagag atggatgctg 1080
gaggagagag ggggtcccga gggagcccag gactggaggt gggaagacac ttttacggac 1140
actttttcat tttgacagac agaaaagatg atggtctgca gcagtagacc cataaattgt 1200
gattgaaagg tctgatggtt tctctcactc gtttcctcca tgcacagaga ggtggagtaa 1260
tttgccccag gtactacagc tcataagtgg tgaagttgga gctgggggcc acagaccacc 1320
gggttcttct ccatgaatcc ctgctgcatc ttggtctctc ccaaccccga gtacccatga 1380
gttttaagaa agacagttgg gaacaggtga ttcctatggt catttttgga acatttggtt 1440
agaatattct agtataaaat ctaagttgtt gttaagtaga aatttccagt ggacattagt 1500
ccagaaaatc caatctcatc ctgtgacgga tttgcagcgc gtgtgcattt actaagcaaa 1560
tctgattcta ctgctgacca gcgagaaaga ggaataatgt aaacagtcag tgcaattgca 1620
cccacattca gctccgtgag cttctgccac ctcagctggc tgcggtgggg gatgtggagg 1680
tgcatgcagc tcccaggtcc cagtgagggg cctctgtata gggtgggcgt cctctatgct 1740
ttatgtgcat ccaggcctgt actaaagaca gtctttctcc tgcagcgtga cacttagcta 1800
agaaaactac ttcatctggt gctcagcgga agaaaacatc cttttcaaca gggaaggaaa 1860
attggctgtg ttgggctgag ggaggggagt gtggaatcca aatggtgtct tcctcaggcc 1920
ttctccaagg tgatgtggac tctgatttta gaatgccatc acttggaaaa ttgtgttcca 1980
ttgctcaaat tcttgcctcc cttctctgct cttttctctt cactccactc tccttcctct 2040
gtcttctgac ctattcaatg ctaatggtct tccagaaggt taggcttccc acacaagccc 2100
agcctcctgc caggcaacct cctcggggtt tcaagctgca aatctgggct gctcttcgca 2160
ttctgtcctg aggacctcct caaagagaca ggcaaggccc tcccctggca aaatgccctt 2220
ctaattccat gcactagttc catttgttac tgaagaagtg ggccgccttc cctgcgcctt 2280
tgaacttgac attccttatc ttgacactcc gcacttttga acttgacact cttgtatctt 2340
tagcaggtaa aaaaccaaag gtgctcactc aataaatcgc cgcaatgggg agcctatggg 2400
aaaatgggta tgtgtaagct ggctaaagga taacaggcac acaggctgct tgatctggga 2460
gggtctttag acagcaggat ggctcccaga gatgcacttt tgttgttgct gttgttcatg 2520
atcatgtccc cagggtctag aatggtgcct gggcatagta ggtccttaat atagttaggg 2580
aatgaatgag ttcatctcaa ccacgctgta tggatggaga aactgcgtct tggggctgta 2640
ccatgctatg cacctgtaga aaatgttttt gccttttcag tcctttccca gtggcggagt 2700
acaggaccca gctcataaga gcagctcaat tctgtcattt gtggaatgac agaataaata 2760
agtttcaggg cctagggagg caaggctcct gccttagcct cccaagtagc tggaactaca 2820
gctgcatgcc actaagccca cctgattttt tattttcttg tagagatgat gtctcattat 2880
gttgctcaga cttttctgga actcctgggc tcaagcaatc ctcccacctc tgcctctgaa 2940
gtagctggga ctacaggtac ttgccaccag gcctggctag ctttcatctt ttttgtagag 3000
atgggacatc actatgttgt gaaggctggt ctcaaactcc tggactcaag agaggcttct 3060
gcattggcct cccaaagtgc tgggattaca ggtgtaagcc actctacctg gacacaacat 3120
tttatttgaa ataagaaaaa ttttccctca ctgggaatgc tgcagttgaa ctagttcaaa 3180
tccataaaga atggaagact gcccagtggt cattcattca cttattaatt ctagatgtta 3240
aattttctag ggtgggggaa tgtgctccta cattcaaggc attcttgaat gtagccaaac 3300
tttgtagttt agccaaactt catagctaat agccaaactt tgtagtttag ccaaactctt 3360
ctagcagcaa aacccatgac actggaactc tgttcttcac actggtcatt tgtaatagaa 3420
taacagagct ttagcactag ataggttctg aaagatcatt gaatctaatc ctctcccttt 3480
gccagtgaga attctgaagc ccagatttgt tgatttgtga tcgatagtgt caaagacagg 3540
cctgaaacac agatgtcctg ggtcctagag gtgctgtttg cccctctcca tatttctttt 3600
gttccagaaa acccttctcc aaaactggcc ctaataatca gaggggaaag ccatggcccc 3660
tgccttgggg acagcatggg ttggcacaga aaagaggttt acaattcagc aggaagtgtt 3720
gtgcgtgcgc gcgtgtgtgt ctgtggaggc gcagggaggg tcacctgagc ttgccctggg 3780
tctggtcctg ggctcagtgg caaattcaac gctgggcagg tggcctgagg acaatgacgg 3840
tgccagctgt gagtcagact tcctttattc ataaaatcat gttcctccat gcagtgcttc 3900

tcaacctggc tgcacgtcag gagccacctg ggtagctttg taaaaacccc aatgcccagt 3960
tggtttgcac cccctgagat tctgattaaa ctgttctgga gtccatctct ctctttttat 4020
tatctatcta tttatttatt ttttaatttg gcggctcctc tgggacttct catatgcagt 4080
aggggttgag aattgctggt ttctttggcc agatgggaac atgcaggcca atagttacgc 4140
acaaacagag cagcagagca ggacggtgct aaggttggaa agggtgagtc atgaaagttc 4200
aggaaatcag aataggaagg ggtgagaatc ctctcatctg ggcttgtggg gcaggagaga 4260
agagcccagt gggcaaggtc cagtgagtgt gcttagaact gggaagatga ggggaagggt 4320
aagcgctggg cggcggtggg caagatggac gagattaaac gggagcgagg atttcagcga 4380
gagctttggc gacacggaag gctgagaatc ggagggagcc tgtcgggcga aagcgaaggc 4440
ggctgctggg caggcagccg aatccggctg gagagccgag ctggtgagac ctacaggaag 4500
tgaagggagt cgctcagggc gtggcgcaac gagactctta gaagaaactc tgaggcagag 4560
atggggggcc tccgcccata cggagacaca aggaagtcca catgcacacg cacgagcgcg 4620
cacatgaaca cgcacaagca cacaaacgcc tcctccgggc agggcacacg cgcccgctgc 4680
acaggccgag gccctggact cggaggggac tgcagagccg acccacaacc cgggccccga 4740
tgcccctccc ggccgcgccc ctacgactgc ttgcgcaaca ggcggcggct ccagtgggcg 4800
cccgccgcgc gctgccggag cccagcccag cccggcgcgc ccgggaggag ggagcaggga 4860
gcggggaagg ggtgtgtccc ggctgcgtgc tgggactgcg agggtctggg aggggcgagg 4920
cgcgggggcg gggccgcggc gcctataaag tcgccctccg cccggacgta aacaaacctc 4980
gcctggctcc cagctggtgc tgaagctcgt cagttcacca tccgccctcg gcttccgcgg 5040
ggcgctgggc cgccagcctc ggcaccgtcc tttcctttct ccctcgcgtt aggtaagccc 5100
gcgccctctt ctcccaggcg cgacgcggag cagaaagggg ccgcccgtgc ggtgccgctg 5160
gagcgccagg accgcccttt gtagccccgc gtactccagg agtaagcgcc ccggagctgc 5220
aagtgcgcgg tcaccccggg gcgcgggcgc gagggggctt tgggtgcaga caccgctgaa 5280
gtggggggtt caattacgga aagggaggaa aaaaagacag ggtcccattc atctcccccc 5340
tagctgggga ttggggttct ggaggtcggg aagggggcga tgagacggtc cgaaaaaata 5400
ccaggatagc gtttcctggc gtcgtctccg aatcccagct ggcgccgctg gcaggggtgc 5460
gcgctggacg tgggtgcaga agggactagg tgagtgtgtg tgtgtgtgtg tgtgtgtgtg 5520
tgtgtgtgtg tctgtgtctg ggaacggaga gattgcgcgc gagtgtgtag atgaggggtt 5580
ggtagaggtg agtggcgctg gcaaggaggc tggagagggg ggcgtgggtg tgtctggctt 5640
gcagactggg tcgggaacgg gtgtggatcc gagaggctgg tggcgcttca gtcccgtagc 5700
acacacaggg cggtgtgcat gtgtgagtgt aagcgtgtgt gtggggggag ggtccagggt 5760
gtgtcacagt gcaacacgcg agccggagca agtgtctgcg agcgccactg ggcagggtgc 5820
gcccccggag gaacccggga ttggcgcatc ggcggcgtgg gagaagtgac ctgggcgaga 5880
ggggtctcgc ggacatttgg cggccccttg agctggctgg agctaggagt ggcgtcattt 5940
tcgcctccgc ccaccctccc ccgctttcct cccctcccct gcctctggtg ctggccgctc 6000
ctgccctcca cgggggaaaa cagctggaat tacttcgacg ttcctggggg atccgccgcg 6060
gtctctgatt cagcctcggg aggagatttc gggctgggct tggggaatgc ggctggttct 6120
gggggaaaca cgctgtcacg cctctgtcaa attagtagct ggagctggga tttagtgggt 6180
cggttcctta cccacccctc ccccatatcc cccgcctgtt gtccctgctg tccctggggg 6240
tcctgaacag cacgcctctg tggcttggaa gtgggagatg cctggcagtg ggtgggggag 6300
gcagggccct tggttatctt atgcccatcc aagaacaccc cttcctcacg gacgctgggg 6360
gcacttcagt ttgtttggtt tctttaagct actgggaaat atctaatatt cagaagtcag 6420
ccagtggcca ggcaggaggg cggtagacat ccttataata cccatcagct gcacgggacc 6480
ttctcctaag ctgcctcaaa agaacatatc taaatacaaa ccccctgttc ttgctgcaga 6540
aatgtagttt caaggcgaat ttttcagtct agactcttcc tgttgagaaa ttctggcgtt 6600
cttattgagt ttcacaaatg tgattgaaat aagcaaggta cagagggggc aaaccccagc 6660
gcctttcttg gaagggctgc cttctggtgg ggaagacaca cggctggaga cgcagaccgg 6720
ttctgtgtgg tgtgtgtggc ggcagagcca gccccaggct ggggtggcac agtgtcgggg 6780
atgaaggaag gtgtaaggga gagagagggt tttggtgtgg atggaaaact gggtgttccc 6840
ctgacctgtg acttcttagc tttgtgggac cgtgtctccc actgcatctg ggcaaggagg 6900
aggatttgcc tgccctgccc tgtaacctgg cccctggccc agcgcctggc aggtgggagg 6960
agttgaaata tgcacatggg atgaagtgga tggctctggg tcttctgccc cattctcctg 7020
tgaaccagca ggttgagttt cgggcgaggt aatagactgt gcatgggtgg aggtgttggc 7080
ttttgctttt gagggatcct ggagtgtctg tttgggtctc ttgtgaggaa gttcttttgg 7140
agcaacaaag tttgatcatt tggaatggaa taaaaaactc acacaggacc tacctagtcc 7200
aacttctttc cattttacag atggaaaatg aggcacaggg atggggaggg atgtgcccag 7260
gccgaactga gctggagctc agctttgtga gaggctcctg ggtgggactg aaggcagtgg 7320
aaagggttcc tggcaggatt ttgcctgcct gctatggagg ggcgaaagga aattgcatct 7380
tcattgaatt tctggtgagg aaacttttga ggccaaaggg gcagagcgga atgatttttt 7440
ttaagtgtaa atcaggtgtt cttgagagat tagcaaagcc agcctgaagg gcaggggctt 7500
agaggctgca gccccctgag ttggcctttg actgaatggg gctgcccccc ccccatgaca 7560
gaagacagat ctcaactgag ctgaggcccc ccctgggtta cgttgaaatc cgctggcctg 7620
aacttgcctc ctcatggacc tgaaagaaag atagcattgt gtttttcggg tggcccaaag 7680

ctgcagttct tccttgggac ccaaccccag agaatcccat tccagatgtg actcagaggc 7740
cttgtcctgg gagagaaagc gtgatgactc agcatctgag actcaagcag ggttcaggtt 7800
tgactcagcc tggaaggaag gccagggagg gacacgacat ctctctccct ccagagcagc 7860
cactgtctcc ctccatctgc cttcatcctc gcaagaacgt tgcctggatc gtattgctcc 7920
attgaatgga tgaggcaact gaggccagga gagtttcagg atttgcctgt tgccatatgg 7980
ctcaggtggg agagccagac tttgaacccg gacagttgga cttcagagcc tttgtttttt 8040
ttccatgaca acatgttggg gcgcctgaag aaatcaggct caaatgaaaa ggcagtcagg 8100
agctccatgc ctcagtttgc ttttctgtaa aatgagagta acagacatga tgccatcaga 8160
atgtgtaaat attaatctgt gtaacctgag aacagcactg agcatgtgga aaacagcgtg 8220
taggtgtttg ccgtcagtag cacccgcaga agacattctc aaggcatctt cttggcaggc 8280
agagtctggg accctttgtc ctaaacctac ccaaggctgt gaaagtgcct gcaaatgctt 8340
gacctaggta ttgctttttg ctaagcttat gtaggttgca taatagtgtc atgaaaatct 8400
tgtctgaaat gcacatttcc aggcccggtc tcctgcgatt ctctctccat caggggatat 8460
gctgttgttt caaagctcct ttggctgggt tgggaatggg gtatcctgca ggcagacaga 8520
gttgagaacc tctgctctgg aatgacaggg gagagaagaa ggaaggtgta gtcatacatg 8580
ccgctgtacc ttcaggccac cctgtgtcca cagtgccagg gaattagtga gtgctcagta 8640
aatagctctt gtttgctata gtgccttgga ggttcaatgc atcacatcag ctaactgagg 8700
tagaagacat tctttccacc tctcaggtca gaaaattgag gctcaggctg gtgactcttc 8760
tgtccagagt ccagtgagtg gggttctgcc caaggtgagc ctcctgagcc ctttccaccc 8820
tccaacattg cgcttggagg ccacccacac cctctgggcc caccttttga gaaggagctt 8880
gtttcctgga tccaccccag gctgttgttc ctgcctccct tgatggctgt gtgatggaag 8940
taccgcgtgt ccacttgctt tttatctttc aacttggaga tagaggcagg ctagatagcg 9000
gtcttggctg agaacttagc ccaggcaggt aggcagaagg gctggggaca ctgaggcccc 9060
acaggaaggc ggctgcagag gtagagggcc atgagtaggt gccaacctgg agccgtgttc 9120
tcgggtgggg gccatgtgaa aagaaacctg ggctccctcg ggtgggggcg taagccccag 9180
cactacagaa tgggaatgga ttacacaatc ggggccataa gcaggtctct ttgacccaga 9240
tctttgactt tcccacctaa cattgtttgc agacctgcct atgccctacc tgcctgggct 9300
ccatctctga ccttttgagt ccagcgggtt ttcttgcaac tgagctggta gaaagtgagg 9360
ctccttaaca gtctgggggc cttgagtgcc ctccccagca gtttaatctt tcctttcttt 9420
aaatctgtgg ggagactttc tgcagctata cattttaaat ctgcatagag ttctgtttga 9480
agagaggtgg gtgggggccc aggtctccac cttgttctcc tgccttcacc ctctgcccca 9540
cccccacgga acctaagaag attccaagta accctcttca aatatggatg tgagagggcc 9600
atttaagagg tgggtctatg gcctctctca gctgggaatg cttaccatct ctccttcctc 9660
ctttttaatc atggaactct cctcctttct ccagaaaggc ctccttggct gtcctctgct 9720
ggaggggagg ctgggttggg tccttcctgg gacctgtcca tgtctcccat gaaacctgag 9780
agcctgtcct tagtaggtgt tgtgttagtg gtggtggaaa ggacatctcc actgattaag 9840
gaagtaactt ggaccatctt gcatagacaa gatcggaggc ccattccgga gggtggaaac 9900
tgcaagacca ttctggggaa ggatcctgtt agcgtggcta gcatcccatc tcgggacaaa 9960
gggttctgta tgaggaagcc atttcctcgt cctctgctaa cactggctcc gtttgtgtga 10020
tttccacaaa ttatctcaga ccatccagcg cagctcatgc ctgtcttgca atttcacaaa 10080
ataatcctct aaggagacca gtgtcctcct ttggacttcc agttattcac ctcgtcccct 10140
cagacggtcg tcttctgtct gccaaacaaa ttactcgtgc tgccaaaact ccactgagaa 10200
tctccaggaa gatctcggtt gtattgcaga aaagaggtgg gccctgacga gattcctgac 10260
acctccctct tcctgctgtt gtgtttggga cactcttgct gacctccctg gtgctgtgct 10320
caagttgaac acacagcagt tcagtttcag ctccagcccc tctggaccca cacccaggga 10380
gtgaatgcgg ctttggcttg tttgagaggg agacagtggg gttcaccttc ttagtgatcc 10440
ctttttcatt ccttgaatat caaacatcac tgatgcctcc tgggtttcag gctccactgc 10500
aaagtacagg ggccctcgag gagttcctac tgtggttttg ttctctgaca tttgtactgt 10560
ttccttgatt ctgattgctc tgatacagtt ccctaacctc taaactaggg aactttcttg 10620
aaggtccagt gaatccaccc atattgtttg ctgagttgat tagttaagaa agacagttcg 10680
tgaagactgc caacttcctc accctcccca gggccttgag tttcagaagg gccagaaaac 10740
accaagagag cccatgaact gctttgtagc ttgacttcaa aatctctttg ggcaaagaaa 10800
tgacaaagga ttgtatccac agtaaggcag ggctatccag ggcctatctc aaggtggttt 10860
tccttgaggt tcaggcagaa atggtgctgt tctcggcaat tttctgggga agttgctaca 10920
gcaaacagcc acagcacatg ttgatttttt ccctcctttt aaaaatctgt tctataaaga 10980
gcacaatgtg ctggagagaa cactggcttt catgtggcct taaaaccagg catgttacag 11040
agggtggaat ctgggccagt tgctgttggt gacaatcgat gctacccttg tcagcaattc 11100
ccgcctgctc tccctttatc tgcttcactg aaggcctcca ggggactgtt gtccgccctc 11160
ccccgaggga atgagtgcca cacctgcacc cacaacaaac cagtgagttt ttcacctttc 11220
cctcagtagg gaggaatcta gacatgtggc ctttggaagt caagtgggcc tttgtgtttg 11280
tttttgagcc tcaggttacc catttgtgaa ctgggtataa tacccagcct ctcccggggc 11340
aacctccttg aggagcaagt gaaacagtgt gctgagtgaa tgcttttcca aagcattgag 11400
ttggccgtct taggaaggta ctatggggga gctgaaaaca gagaaccttc tgggtgttgt 11460

gaagggagaa aagttcaaga tgctggtttt gccaaaagct caagcctgct catgatggct 11520
cagtggtgac tgcagggtgg aggagaggca tgtgactgtc ccagctgacc tccacacgct 11580
gaaagcctca tggtcacgtg cagcccacct tacaacgttg tatctgggcc acgggtggtg 11640
gtgaggactg tggatatatt tagggcaatg tgagatcatt gtcaacattc cctttaaggt 11700
tgctttaacc ttaaagcagc cagcttgatg cttgctgact tggggcacac atcttaacct 11760
cttgatgcct cagtttcccc gtctgttaaa tggtgacatg ctggcacctc cctcctaggg 11820
tttctgtgag gcttaaagga agtaccgggt ttagagtcca gttagtgcca cagatactgt 11880
tcctggtgct gtttttgtgg tgtgtctgtg tagggttaca ttgttgcctt cctgtggctg 11940
ctcttcctgg tgggcggaac ctggaacttc tagaacagat catggatgtt tctctgctgg 12000
ataccccttc agcctcaccc tgtgggagtt gtggcggggc ccaggagagt ttgctcaaga 12060
gggtgtgcag gtgggtggca gagctggagc aaggcactag tcttaacaga caggagtctg 12120
aggctggcag aagtgggggc cttggtccaa ggtgcggggc taatctggcc agagctgagc 12180
ctggcagggc cttggtaaga ccctgtggcc cttttgtctt ggtgaagcac cttcctgcct 12240
gggtttccat ttgcctgtaa gtttgttgtg gacagcgtga tctccataga cgtacactgt 12300
gcctacttta cagatggaga aaccaaggcc cagagaggaa gtgacttgct cagagtcatg 12360
gaataggaaa gtggcagggt ccccatgccc tgattcctag gccagcgttc ctgctgcccc 12420
actgaggttt ttgggaatca caggctctgt gtgtccccct cagtccccca ggggctgttg 12480
aacagttctc aagtggtgtc ggattactga agatgcctct gcccactgcc ctcccaggtc 12540
ccagggtcac gggtcacgtt cttttcttcc tcgggcagat cctggccttg gctttgcctg 12600
gcacctgtga tccagctctc ctctgactct cctgggtctg ctggactgga agggtatttg 12660
tgtagcccct cttgggccct cattactcat tgacagacag tgctggtgga gagaagggcc 12720
ccagtggtga cgcactgggc ctgataatca gaaaccttgg tggcatttgc tgagcatttt 12780
ccatgtgtcc ctctcctcgc tgggactcta ggactgaaac ccaggccaca tcctcacaga 12840
tgtcctggca tctccaaggc aaccctgaaa ggaaagtatg ttgatgaatt aagcaactgt 12900
ttgcttaatt ctccatgcca ttcttgaatt taagccccac tgctgatcat tgggagcagg 12960
agttagcatg cccattgaca gacaagcaaa ctgaggcaaa gaggttagat agccaggcat 13020
ggtggcatat gcctgtaatc tcagctactc aggaggccgg agtaggagga tcacttgaac 13080
ccaggagttt gagaccagtc tgggcaacat agcaagaccc tgtctctcta aaaaacaaga 13140
tgaataatac atttttaaaa agagattcga taaattatgc aggatcacag agctaataaa 13200
tggtgacacc atggcctgca agccttcccc cagctcctgg ctacctttcg cttctctggc 13260
attggtcttc tcggaattcc tcccaggcag gtggatggga aaagaagtga agatacaggc 13320
tggcagctgg tgctgtgacc tggagccctg tttttggtgc taagctgggt tttagctgaa 13380
ggagacctgc tggaggggtt tcttctgtgc ttcttgggga gcaaagatgg ccttggagat 13440
ggagacacgg tttgtcagtc ccaagccact ccaaaggtgg ggctggtggg ctaggagtca 13500
gccctggttc ccccgccatg ctgtgtgtgg ccctgccttg ccaccccacc tgctggagga 13560
cctctttgat gctttctagt gggtcccaac attctgtgat taggactttg catgaggcag 13620
agggagggca ggagctggag tccagcaagg acctcaagcc cagggacagg ctccacctgc 13680
cacacttgct aagccgtgag ggcctcaggt gcccgggagg agggagcagg agtggaagca 13740
ccagctcgag ccgccaaacc aggtcacgta tcacctgcca tttcctgctg gcagaaggag 13800
atcacagaca ccagaagaca gcagcttctg ccagaactgg gtccttccat gattctcagg 13860
tggtgacttt gaatcccaga aaaagtgttt tggggagagt ggagtgaagg aagagtgtga 13920
acattggatc tgactccact ccagcccctg tgctgcagcc agcagggtga gctcaggaca 13980
gctggcagcc gcttgggccc agccccctcc tccgtgaatg aaggtggtgg ggagtgcacg 14040
tgtgcgtgtg agtgcgtgtg agtataaatg actgtggatg tgagagtcca tgaaagtgag 14100
aatgtatcag tgtgtaagaa agtgtgagta tgtgagaatg agtgtgtttg tgagaatgta 14160
tgtatgtgtg agtataagtg tgtgaatgtg agaatgtctg agtgtgagaa tgaaagtttg 14220
caagtgtgtc agtgtgaggg aatgtgtgag tgcatttgag agtgtgtgtg tgtgtgtgtg 14280
cgtgtgtgca cgcatgcact agattaaata agaatgcatc caactgacat tttctggata 14340
ctgttaccta ctaggaatta ctaaaatcag ctattccaga ggagggtgga tgtcgcacca 14400
ggggacattt ggcaacgttt gcagattctt ttgctggtcg cagcgtgggg tgtatgctgt 14460
gggcatgtgg tgggcagagg ccagggatgc tgctgagcct cctcccgtgc tcagggcagc 14520
ccctaaggca gaggctgctt gtggggccca aatgtcagca gtgctgagct gaggatctct 14580
gtccaggagg cacagacgct gtcctggatg ctgtccagac agcatctgct ctgccctcca 14640
gggggacaga cagacagtgg acacagccca gatgcagcct gatgagtgag cccaagggag 14700
tgcctgtccc ctcgggactg tgagctccgt gggggcagga actgggtctg tttgctcatg 14760
atccccagat gagtcccata gccagtctca tcaccatagc tggtgttatt tttgttctga 14820
gctctgtgtt gggcacatag aacctagtgg gtagaaacct ctgaataaat cctgagtttt 14880
ccatcctgct tccgtgaact cagatgttct ttgagggcca gcctggtggc gtgggggctg 14940
ggagtgggca gattgtaggg agccaagtcc tggggtggag tggggttcag gcccctttcc 15000
ctggcttgag ccgaagcacc ttggcttttt aaattttaaa atttttattt tttaacattt 15060
ttatatgttg aacttcctta catgatttcc tttggataaa agctcctgca aaaaaagttt 15120
gaaaagtttt ttggttgagg catcagggac aaggtaaggg ctgcttgatg ggcccaactc 15180
gggagaactg aggtgaggga gccgctctcc tcccacccta caggctggcc agatggtgga 15240

ccgccagcag ctggctgggt caacctgcag agagcacctt ctgcagtcct tggttcatgc 15300
ggccttagta ttgcaggacg cgaccaacat gcaggctacc ccgaggcctg cagcccccac 15360
agtcccccca gactagtgca aagctttatc cagtctcccg gcagacttca gagtctagga 15420
cacttcctaa atggttcaaa atcaatggaa tcatttgggg gacaataaat aatgtcacta 15480
gacattgcca aatgtcccct gggagagaaa atccccccta gtttagatat accccgtgat 15540
catcctcagc agttttatct ctggacttcc caaaggcctt tagcagtgag ggggctgggt 15600
cagagaaggc cccaccagct cccaaaagca gatgctcaag aacttgatat ttctaggaca 15660
cacagcgatt tttttaaccc attgcctcat gtggtctggg aagctaagct gtaggaataa 15720
aaaaggtgcc gggtagggta aagaggtttc accagataga ggaaacgggg aaggtggaca 15780
tttctgtctt ttctgttctg gagaacgaga gagctggtgg agactgacag acactatgtc 15840
ctaatgtgat cacggtttct ggaagccaaa gagaactgac caaactgggc tgaaccacag 15900
catttctcga gtccttttaa atgtagaatt tgttttccag acattgcact cttggggttt 15960
agcaatgatt taaaagtgaa ttttactgaa agcatgctgt tgagctacat tgctcattta 16020
ttcagagagg gagttgcagc ctgactttct gcctaggaga gtctctcgtg ctgggagcta 16080
gaggtagaga aaagaacaag ctacctgctt gatgaagctc acagatagga ctgcttgaac 16140
ccacctggtg ggacagtgag agccccctga cttcaccctg gcgcgctttg ctgagcaggg 16200
tatgctaaga agctcctgtg aaatgagaag tcctttcact cccagaatga ggggtggcgt 16260
tggcagggcc ctgggtttct ggaaggctcc ctaggccatg taggcgaggt gtcccacagg 16320
ccagggtctt gggaccacga acctgctgaa ttagcaaggc taacagacgc gggcctttca 16380
tgcctcctgc ctccaagctg gcagctgccc aggaatgctc acaaatataa aaccgacttt 16440
gttttcagaa tgttctggag catagactcc aaagtctgta taaagccatt cttgtggggc 16500
tggttatcta ctggggaaaa tcatgctggg gccccagacc agagcacccc agtggagagc 16560
aggatctgcc cacggaatgc catgtgttgg cacaagggat ggacccgggg tggattcggg 16620
gtggcccgca cttgccggct gacctctaag aggtgtttca ccaggttctt gggagttcct 16680
gggtcttcag aggagcccta ggcaggaatt tcagaggctt gcattctctc gaccattgca 16740
tccgaggggc cttgggcagt gcggggccca ggttcccact ctgaagctgg ctgtgtgagg 16800
gctttggtca ggcgttttcc attcagaaag tctggatctg tgaactgggt gggtggcctg 16860
ggctcttccc tcttgtcact tacttcttct ccgccggtcc tctgctgctg tctgtaagac 16920
ttgaccttat tctgcatttt ttatgcttca ccccagggat tgatgggcat ttagtttttc 16980
taacttgact actgatctag aagaatacat gcttatactt ctggtttcta gattcagctc 17040
ttggaggcat gcttaataga tagcaccacc tgcccaagcc ttgcacccca gggagtcagt 17100
ggcagcattc caagtcttcc tgggctgtgc ttcagtctgg ttttgcagcc cacacttctg 17160
cacctgccct gtgttagagc acaggtaacc cctctggtcc atcccctgct ttgatccagg 17220
aagtgctttc tgccttggag gtcttttttc ttacaaaatt gtactgtgcg ttcaaatgcc 17280
tgccaaactc cttgacttaa ggttttcctc tgcccctctt cccttttagc aggcagccga 17340
gagtcctgca ccgtggggtg tgtatgtgtg ttcacgcgca tgcgtgtgcc tgtgtgtctg 17400
tgcgtctgca tcaccaacct tctgttaggg ctaactgtga ggctgaagct tgcactatgc 17460
ctatacgtgg taagcatcgt gtgagcttgt gttccccttt cattgtcaca cgggggctgc 17520
ctgactttgg gatcagcaat gcaggtcccc ttctagaatg cagtgagctg catcctgggt 17580
atggtgggtg ggcctgaact tggtatgggg acctgtttgt gaggctgatc tatccatttc 17640
attgggaggg tggtgtctgt gagcctcagt atcctctccc aggaaatcca ctgcgctggc 17700
cccaatggga agactgggtt gggtccatgg gctgtgtgta caagggacct cctcatcccc 17760
tgcagtgcat cacgagggag gtgaagctgc tgggcagagc gcaggcctgg ccttcttgcc 17820
attggtcttg gatttgcttc ctttaggaaa cctctcagca ggaagtgttg accttttggc 17880
ctttgtctcc ttgcagg 17897

<210> 52
<211> 5987
<212> DNA
<213> Homo Sapiens

<400> 52

tttctaacct gtatcatttt tcttatctca ataacttctt ttaacatttc tcacaaagca 60
gatctactgg ccacagaatg cctcaatttt catttgtctg agaaaacctt atttctcctt 120
cacttttgaa agataatttt gtagggtaca gaattctagg ttgtaggttt tttcccctca 180
aagtgaaata tttcattcca ctcttttctt ctttgtatgg tatctgagaa gaagtcagat 240
gtaattctta tcattattac ttaaaagatt gcttctgttc ctttctctct tctccttccc 300
ttctttcctt ctctgtatat tacacctttt atagttgccc catatttctt agatattatg 360
ttttggtttt cttctgtgtt tttttctttg attctcagtt ttagaagtct ctatttatat 420
atctgcaatc gcagggattc tttcctctgc catgtccagt ctactaataa gcccttacag 480
acattgttga cttctgttcc agtgtttttg atctctagca tttctctgat tatttcttgg 540
aattgccatc tgtctactta cattaccaac ctattcttgt gtgttgtctt atcatagtaa 600

ttgcagttgt tttaatttca taggtattgt aatttcaaca tctctaccat atttgacatt 660
gattctgatg cttgctctgt cttatcaagc tatgtttttg tcttttagtg tgacttctaa 720
ttttttgttg aaagccaggc atgatgtact gagtgaaaga aactcaatac attgtaatgt 780
gacgataaga gttcagggga agtgaagcat tctatagtcc tatagcaggt ctcggccttt 840
tagtgagcct gtgcctatga acggtgactt tcaacaagtg cttttcattc cactcttttc 900
ctgtccttaa gtgggacaag atcactgggg gggggctaga attgggtatt tcccttctcc 960
aatgtagaag ctaaagagag ggctggagtt gggtattttt cttcccctgt atggaaagct 1020
agaggcagtt aaatttggat attttccttc ttctaattca gttaggctgc gacaaaaatc 1080
ccgacagttt aggctctaat attataaaat aatttctctt gagtataggc cttattaaga 1140
acactatact ctgatggagc tgagggggag ttttctctga tattcactgc gagaacctcg 1200
tagagctcca ggaagcaaaa ctcacaaaag tgtgggagtc ttccagaatt tttcctttgc 1260
agacttatct gcactgaacc tccagaaatt catcaattac agttcaggtt ttcctaccca 1320
ggtactggtt ttcatggagg tttctgcctg tgcatttctg ctccagtaag ttgttcttct 1380
tgtatggtct gtctttcaaa ttttttaagt agggttatga cctgtcgcct cacttctctg 1440
acagttctga gagtgttgat ttttcagttt gcttagattt ttacttgttt ttaggatgaa 1500
gtgacaattt ccaagctcct ccctgacatg ccagatcaga aactgaaagt cctaagcctc 1560
atattctgtg cgtgggtatg ttcacatcct gcctgctcca gtgcccccac ctcacactct 1620
ctttcccttc cttgtcccct tgtgagattt ctaggtccaa tacaaagact gtgttcaact 1680
cattcaacta ctttggctca tctgagtatt ataatgaaca atcacaaaaa aaaatgaagt 1740
aaaagaaaaa tccatcaaag aattgagata tttgagaaaa agaaaggaga tcagtgtttt 1800
ataaaactta gaaatagatt ttttaagtgt ttcttcattg acttatgtga aaggactttt 1860
cttaatttaa caaattatgt gctttcgttt atagcctcaa aacttcttgt gtagctaaga 1920
atgggtaaat aatcaggctt tactaaagga ctaacgtaaa gatcttctgt aagtaacatt 1980
tctgctactc aaggaagaga taaacttcat ggcataacct tgccaaagta tactaagaat 2040
aaccctgaca caaagctctt ttttcagcca acatgccatg aaagaaagaa gacaaggggt 2100
gatctccact ctctaagtga accactaaac ccaccaaaga agaaacgagg gaaatagaaa 2160
gaggaccctt gcctgagata atggatctgt atgtatgagt agtagaaccc tgctcaaagt 2220
acaaggaagg gaaaaaaaag ttagtttatt tggaattttg gacattaaga gtctttattg 2280
ttcattttct tttaactcac atgaatggct tatcacttca attaataaat atttcatttc 2340
ttttcaacat attcatgaaa caaatctgaa atgaacagtg caacatgtga atgtttagaa 2400
cattataaaa ttaaacacaa aatctgtctg gcaatcttcc tagcatctta ggaaaaaagt 2460
tgacaaaatt tcaagcagca gaagggggca gtaaaactca acagaaagct ctggaagatt 2520
tttaagattc ttccttattt tcttttcatg tagattattt cccaacaaat ttcagacgct 2580
aatagaaatt ttgtacaaca gatccatata tttgcctaaa atagacacag aaacattgaa 2640
tatatgcaaa catgagagct ataagtttta catgatcaaa cctttttttt atggtacaca 2700
atagtcacag tacttttcca tataaaacag gtttagtggt cttaatttag tttggcacat 2760
ttaatacact cccatgacca gcatcccaaa tgtacctatc cgttttattt tattgtctca 2820
gaattgtcag ttatttaata aattatgtaa cttttttcct tatgctcaga tttgcacttc 2880
tttctaaaac tctgcccatc cttaaagtcc cagattctcc ttgaactttt ttttttgact 2940
ttccaagtac atggaactct tcactctatc ctgctatata agtgacagaa tttccactat 3000
gggatagatg gagttcaatt cctttgagtt taaaataatc taaatataat tattccttat 3060
gccctgtttt tccctcactt ttgtatccaa atctcttttc agacaacaga acaattaatg 3120
tctgataagg aagacaatga tgatgatcac ttcaaaatga attcaggatt gtaatgtaaa 3180
attttagtac tctctcacag tatggattct aacatggctt ctaacccaaa ctaacattag 3240
tagctctaac tataaacttc aaatttcagt agatgcaacc tactccttta aaatgaaaca 3300
gaagattgaa attattaaat tatcaaaaag aaaatgatcc acgctcttag ttgaaatttc 3360
atgtaagatt ccatgcaata aataggagtg ccataaatgg aatgatgaaa tatgactaga 3420
ggaggagaaa ggcttcctag atgagatgga attttagtca tccgtgtctc atgaagaatc 3480
agatgtgtac actaagcaaa acagttaaaa aaaaaacctc caagtgagtc tcttatttat 3540
ttttttctta taagacttct acaaattgag gtacctggtg tagttttatt tcaggtttta 3600
tgctgtcatt ttcctgtaat gctaaggact taggacataa ctgaattttc tattttccac 3660
ttcttttctg gtgtgtgtgt atatatatat gtatatatac acacacacat atacatatat 3720
atatttttta gtatctcacc ctcacatgct cctccctgag cactacccat gatagatgtt 3780
aaacaaaagc aaagatgaaa ttccaactgt caaaatctcc cttccatcta attaattcct 3840
catccaacta tgttccaaaa cgagaataga aaattagccc caataagccc aggcaactga 3900
aaagtaaatg ctatgttgta ctttgatcca tggtcacaac tcataatctt ggaaaagtgg 3960
acagaaaaga caaaagagtg aactttaaaa ctcgaattta ttttaccagt atctcctatg 4020
aagggctagt aaccaaaata atccacgcat cagggagaga aatgccttaa ggcatacgtt 4080
ttggacattt agcgtccctg caaattctgg ccatcgccgc ttcctttgtc catcagaagg 4140
caggaaactt tatattggtg acccgtggag ctcacattaa ctatttacag ggtaactgct 4200
taggaccagt attatgagga gaatttacct ttcccgcctc tctttccaag aaacaaggag 4260
ggggtgaagg tacggagaac agtatttctt ctgttgaaag caacttagct acaaagataa 4320
attacagcta tgtacactga aggtagctat ttcattccac aaaataagag ttttttaaaa 4380

agctatgtat gtatgtgctg catatagagc agatatacag cctattaagc gtcgtcacta 4440
aaacataaaa catgtcagcc tttcttaacc ttactcgccc cagtctgtcc cgacgtgact 4500
tcctcgaccc tctaaagacg tacagaccag acacggcggc ggcggcggga gaggggattc 4560
cctgcgcccc cggacctcag ggccgctcag attcctggag aggaagccaa gtgtccttct 4620
gccctccccc ggtatcccat ccaaggcgat cagtccagaa ctggctctcg gaagcgctcg 4680
ggcaaagact gcgaagaaga aaagacatct ggcggaaacc tgtgcgcctg gggcggtgga 4740
actcggggag gagagggagg gatcagacag gagagtgggg actaccccct ctgctcccaa 4800
attggggcag cttcctgggt ttccgatttt ctcatttccg tgggtaaaaa accctgcccc 4860
caccgggctt acgcaatttt tttaagggga gaggagggaa aaatttgtgg ggggtacgaa 4920
aaggcggaaa gaaacagtca tttcgtcaca tgggcttggt tttcagtctt ataaaaagga 4980
aggttctctc ggttagcgac caattgtcat acgacttgca gtgagcgtca ggagcacgtc 5040
caggaactcc tcagcagcgc ctccttcagc tccacagcca gacgccctca gacagcaaag 5100
cctacccccg cgccgcgccc tgcccgccgc tgcgatgctc gcccgcgccc tgctgctgtg 5160
cgcggtcctg gcgctcagcc atacaggtga gtacctggcg ccgcgcaccg gggactccgg 5220
ttccacgcac ccgggcagag tttccgctct gacctcctgg gtctatccca gtactccgac 5280
ttctctccga atagagaagc tacgtgactt gggaaagagc ttggaccgct agagttcgaa 5340
agaactccgt ggatattcca gctttcccac aagcactgat cattatgagc cagttactta 5400
accgatctga gacactctca cctcctaaat agggatagat gatactaatt tgcaggttgt 5460
cattatgata agacaggatc tgatcaatat atgtgaattg tttatatttg gaaccttttt 5520
attgagtgga agaagttgtt ttaaatattc tagtcagttc tttcctgctc ccaggaaagc 5580
ccggattatg ttttaagata agcaaaatgt cttaaaagta agctgtttta ctttgaattt 5640
ttccctaaat gttgattagt gtactagatc cattttaatt tggaaagtga agtgctactt 5700
atttgaactt cttaaaaatg ctaattttaa catctaaaga gttaactaag aaaagcttag 5760
taacatgatg taccaagttg aatatgctgt tatccttatt tagaatagaa aattggtatt 5820
tctacgtttt atccattcta aggcaggtta aaaaattgta tttccatgac tacctatata 5880
tttcttgaat ttattattgt aaagttgatt catagtcaaa caattaaatg tttaaattaa 5940
gattaagaca ctagagaatg atttatttgc tgtcctttaa ttgcagc 5987

<210> 53
<211> 5683
<212> DNA
<213> Homo Sapiens

<400> 53

atggcaaaca cttgaaaaag cttccacgct gaaacccaaa ccagaccata tttaggatgc 60
cccataaggt ggcatttttt actaagatga taaaggataa acattggtgg agccagagag 120
gctgctccca aatacagtcc tccccatcag caacactgca gtagcctgca cctatcgtcc 180
agagtatgaa aaagactttt gccaaagacg gcttttgggg atttatcatt attacatgtt 240
aagagcctgt tctccatgtc ctcaccttcc taccttgcta atcttcccca tttccatctt 300
tcggcccagt gtgagacctc accatctcct tttcccatct tctttcctcc atacccttgg 360
tttcctagca ggtcaatgcc cctgcccctt cactggaatc catccatcct ggttattctt 420
ttctttatga acctgctgga ctacatctca aagttaccca aaagagggat acaaagacac 480
aataaaatcg gaaataacgc tcacccttac cagcctgcag ttaagagtgt gtggaactag 540
ggtggctgca ggcaaagaga tagggggaag ggctgcctca tgccatgggc aggacagtaa 600
atggtgcaaa ttttctggaa agcatcttag taatataggt gaagagcctt tacaatgtgt 660
ttatcctatg acctatcctt acttctctta ttctaaagga ataagaagag gtttaatcaa 720
agcatgagtg aaaaggccat ccaaacacag ggaaaggggc cgttatgatg tctacggttc 780
taagaaatag aaaacaacaa cttggcttaa ataataagca cataaccaaa gaaatctaga 840
agtaggattc ccttaagagt tgagagattt agcagctcaa aaatgtgacc tgacatacga 900
gttctttcta tgcttttgtt cttcctcctc caacactggt ttcacactaa acttggctcc 960
ccagtggcca atagcaccaa acagcactta tcggcattct tgttggtatc caaaagagat 1020
ggagaaagcc tgatttcaca tggctcttcc ttaaaagcaa caaaggactt ttcccggaag 1080
ccttcagcaa acctcgtcag atttgaatca catgccaatt cctaaatcag tcactggcag 1140
ggaagtggga aaaaggggag gaggtgttta cccttaacca atatgcctac ccctgcccac 1200
tgagatcaat tccataaaag tattgcttca gttctttgaa gaggagtaca atgtttgtga 1260
gtcactcacc acatttaccc accaagagct aagggataac tgagtaaaat ggtagagctg 1320
gtaggaaaat ggtgagtagt cccgtccttc tgtgccataa ttgacttgta cacaaagaga 1380
tttggaaatt taaacataag aactctctct tcccaagccc agggctttgg gcacttcaag 1440
tctggtcctt gcctgggcac agtggctcac gcctgtaatc tcagcaattt gggaagccaa 1500
ggagggtgga tcacctgagg tgatgagttt gagaccagcc tggccaacat ggcgaaaccc 1560
caactctact aaaaaataca aaaattagtt ggatgtagtg gcacacgcct ggaatcccag 1620
ctactcagga ggctgaggca ggaaaatcgc ttgaatctgg gaggcaaaag ttgcagtgag 1680

ctgagattgt accactgcac tccagcctgg gagacagagc aagactccat cccacccccc 1740
accaaaaaaa aattctggtc tttacatgtg aaaaaataaa agctaatttt taaaaactaa 1800
ataataaaga gtgttaggtg ataagtttta tgatagagat aatgtgtgct ttaaaacaca 1860
cagcataaaa cagatggaaa acacctcatg gagaagggag agcttgaatt ggtgtttgat 1920
tgaaggacaa gaaaaatctg ttggtaactt cctcctggct ctgacctctg atgcagttaa 1980
agtaaagcaa cttagaatta tgatctaggg aacatattca gagagaaatg aatttatgat 2040
gtcttcaatt tgaagatgct atgattttag actattctag aatacaatga tttttcattg 2100
cttcacactt gtaagaaatg ttccactact gaaaatttgc aacacttaaa aaattcctac 2160
acaaaatcac agcccacctt gctgttctat gctaaataaa caaaatattt tttctcaatt 2220
atgttgctct ccttaattac acgcaatttt aaaactagct gtaactgaca gccctttcct 2280
cacacttact gccaatgtat ttaggtcaat cccacctcca attagagaaa cttagaaaaa 2340
ggaaatccta cggtgaaatg ccatatgcct tacaattttc agtaactaat cctcaaggat 2400
gacaacttgg caaaaaagag aactggacca gctccctgaa aggtccatca cttcggccca 2460
tgttaattga ttgccttaaa atcacagtgc ttccaggttg tagagacaca ctcaaaatgg 2520
actattgccc tcacattaaa ggggataggt tttcttttcc cttttcccat ccctgcttct 2580
ctcaaagctg cacagaggta tatgaggttt ggggaatatt tgcagcacat gttggctggc 2640
aagaatgtgg gtgaattcct gttaaggggc gcagacactc cctcttctat gacctttcct 2700
ttccacccag aacaactgaa gtatcatgat aagaggaagg gctttgtgtt tgagataagc 2760
agctcctata cctgtcagct ttctctaata agaaagctct ttcctgccac cgtataggtc 2820
accttcttat tggtaaagca tgatgaagtt tggaaactgt tgtccttgtt ttgttgaaag 2880
aagccaagtt ccaagaaatc tgcagaggca ttcctacaat ccctcagcca tggcagcatg 2940
aatcctgaag gggcagtgag tgctggtttc tactctgtct gcacagctaa gcctagggga 3000
gataatcttt tcatacaaga tgcattctgc ttttgtgggc ctcttgcagc cctcaagccc 3060
ccatctgatt tgtacacaat gatccagtgg gccagaggag cccagagcca tgagcggccc 3120
atccctccaa gaactatttc tgactgtcca gtatcatgga gcaagtggaa agaagaaaaa 3180
aaaaacccaa ttacttttcg aagagcaaga tgaatgctgt agaaggagaa ggaaggggag 3240
ggagatggat gggtgccgat tccagaatct tcagatctgc ttggatgaat cattacctat 3300
gatttgcggg acaagaatct gattttattc atcaaccagt agaaactttt ctttctgcct 3360
cccaacatct gaaatccaac aaacatgtgc cttaggaaca taccggtcat cttttagagg 3420
cattttatat acatattgag taactagaaa acactctttc cgtaatacac acacacacac 3480
acacacacac acaccatctt gtcatacaac actcccacgc aagaaaagcg aaactgctgt 3540
ttgatgaatg taaacacttg gctgtttgca gcagtcggga gtcctgccag gtttaagtgc 3600
taagatggga ggtgaacccc aggggtttcc ccctgcccgt gctgagatcc ttatttggtc 3660
aagcttctac ctatgccctg gcctcggagc gagcccgata gcgctggatc acagcagagg 3720
gagcgaggcg gctgacgtcc catcccgaag agatgaatgg aattccagga agctagagtc 3780
atgctggctt gggacagtgg cttggagacc agacttcaat gacagaagca ctaggcagcg 3840
gcactcatgg caatgtgtgc acccacagaa atgtaaccca cacctcgggt tcaggagccg 3900
aaaaatgaaa agaacgttta gggaggaaaa agggaaatac aataataggc agagagtaat 3960
ttattactct atgggtctgc tctgtaaata gctgaagact ctggagccag atggttctgc 4020
aaattctcca aacaggagtc acgttaagaa gcacgagtgg gcacaaaaac tgtttttcaa 4080
gacacaattt caatttggct tgtggaaact ggatacgagt aagtttcctt aaaattcgag 4140
tagaaagcag ctgtcctccc cggccccctt gatgagaata cgcacaccgc ccccaagcgg 4200
ccggccgagg gagcgccgcg gcagcgggag aggcgtctct gtggcccccc tggcagccgc 4260
ggcaggaaag ggcccgaagg cagcgaaggc gaacgcggcg caccaacctg ccggccccgc 4320
cgacgccgcg ctcacctccc tccggggcgg gcgtggggcc agctcaggac aggcgctcgg 4380
gggacgcgtg tcctcacccc acggggacgg tggaggagag tcagcgaggg cccgaggggc 4440
aggtacttta acgaatggct ctcttggtgt cccctgcgcc ccgtcggccc atttttcttt 4500
ttacaaaacg ggcccagtct ctagtatcca cctctcgcca tcaaccaggc attccgggag 4560
atcagctcgc ccgaaagccc ctgcgccacc ccgcgggccc tcctaggtgg tctccccagc 4620
cccgtccctt ttcgggatgc ttgctgatca ccccgagccc gcgtggcgca agagtacgag 4680
cgccgagccc gtgcgcgcca aggctgcgtg ggcgggcacc gacttttctg agaagttcta 4740
gtgctcccaa gccccgaccc ccgccccctt cactttctag ctggaaagtt gcgcgccagg 4800
cagcgggggg cggagagagg agcccagact ggcccccacc tcccgcttcc tgcccggccg 4860
ccgcccattg gccggaggaa tccccaggaa tgcgagcgcc cctttaaaag cgcgcggctc 4920
ctccgccttg ccagccgctg cgcccgagct ggcctgcgag ttcagggctc ctgtcgctct 4980
ccaggagcaa cctctactcc ggacgcacag gcattccccg cgcccctcca gccctcgccg 5040
ccctcgccac cgctcccggc cgccgcgctc cggtacacac aggtaagtcg cccccggcgg 5100
ccgccgagga ccaaagctgc ccgggacatc cacctggagc gctgaggctt cagtccctct 5160
ggtggacccc ggaacctaca ctctccccgc tcgcctaccc cagcccgctc ctctcagccg 5220
ctggaggact cttcagggca aggctccaga gccatcctct ccagccttga ggttcacaaa 5280
ccaactcatc aggacacccc aagatttcct tactctctga agtcctcctt aagcctttgt 5340
atcagcactc cagggaagag tctgtacttc ccctgccctc cctgcaaccc caaactacag 5400
ttcctgatct tgctcacctt cgacttccca aaagccccca aattgttggt cttgcgcccc 5460

ccacacttta aaaccagcat ctctttcctc cacctctctc tctctctctc tctctctctc 5520
tcatgctctc tggaaagtaa tcctgttgtc cttcatttca gcaaacgcgg ggtcaaagag 5580
ggatggtccc ggccctgtcc ccatgcccag ccccgtttct gcgcaccgtc cctccctgcc 5640
tagctgatct ctgaccctcg gctcttgtgc ttcctgctac agg 5683

<210> 54
<211> 7833
<212> DNA
<213> Homo Sapiens

<400> 54

gtctttggtg agatatgtgt tttacaagtt ttaatggaga aaaatgtaag tattttacct 60
cctgaaactt ggctatttga gtaatgagaa aatagtcact ttccccagga cagtggttct 120
caatcatggc tatgtgtttc tccaggaaaa ctttaaaaat atatatatac caatgcttct 180
gtgtcacttc tagggattcc aagtctttga atacgaactc tgcatcagta ttctttaatt 240
atccaggtga ttgtgatgtg aaatcatgac tgagccccac tgctctaaga tgaaataaac 300
tttcctcagc actgaaatca caaacttaaa ctaccaaaat taattaaggg catgggaatc 360
aataaggcat agggaagctt ttacattata aaattatttc tttaaatcac agctcattgt 420
ttatatgtta tttgccattg tagaaaaggg tgaaaaaata gcaaatttaa ttactctcag 480
tttgaaaaat tatccagaaa tgaagatgac gactctgaaa cattgtcaat atcatttgac 540
ctataaataa tgttctaata catttactac acactgatag atactttttc atatgaatat 600
tatacattaa aactaaggca ataatgcatt tagaacattc tatctatatc tatgtatctt 660
aagtaggcta gaaattaaga tatgagttat taagtatgag atgttaaggt gtggggttag 720
aaattatact gtacttcatt atcaataatc aacatatact tcaatatcac atacatttaa 780
ctttaatttg tacatcttta actattttta attatgtgta taaatataag tacacacatc 840
tttatgtatt tatttattca tacctccatt cacttattta tataggggat ccccccaaat 900
ccactaccat taaaccatac atttttattt taatctttag aacaagccca ggaggcaggt 960
attgttatta ctcacatttt acaaatgagg aaattgtcta cagtcacaaa gttactgtgt 1020
cagacatatt agaagcttaa tacatatttg gtgaacatat gcataaaaac agagagacag 1080
acatgtacaa cagctcatct ttacactgag taaaagcttt taacctgtct cagaaacctc 1140
tctgtgaaaa ctgagcaaaa atcgaggtat cctttcattt gtcatatagg tataggtggt 1200
accttacttc tccaacaagg atgaatattg aaatgtggat cccaaggccc aactccagat 1260
tttctgaatc cctgatagtg ggacttggaa tttgtctatt gtttcaaagt ttctcaagga 1320
attcatatga tcaaccaggt tcagaaatca ctggatctta ttgccgaagt ttgagaatta 1380
aagtttgggc cttactgcgg ctccacagaa agggcaaatg aagtatcatg gacagaactg 1440
atacgttccc agttagtttc ccctctcaga agctaacagg cagcaataca gcagaaatta 1500
gtgacttatg tcttgtgctc tgaagtcagg cagaatttca cagagtccca gcagtgtcac 1560
tgacgagatt tgtttcttgg ggcaagttgc ctgatgcttt caaagccata ttccttttat 1620
ataaaatgag ataatattct ttgtctcata ggggtgtttt aaagattaaa taaaaataac 1680
atgttctatc ctacatggca caatgcctga cacctaagaa gcaaaggata catcttacct 1740
ttattgaagc aatcagaaag tatgaaatca tgaaggagat aagagttctg attggcagtg 1800
tatcttattt tcccaggttc atttatttat cttaaactat tcttgttgga gaataactcc 1860
caagccccct acttaagctg tgagtaatct cacactttat aatgatgttc tttccatgag 1920
aaaaaaaaat gttcttaagt tttctggaga aaatatatct gcactatttc tactgaaaaa 1980
tctaacaact ggactctgct cctctgcatc aattctagag tgtatatgcc acaaataaag 2040
tgttctagct caagaagatt gaaagtaaat atggtatagt attttaaaat aagaattttg 2100
caaatacatg gtatgattgt gtcatattac tagcaatcat atgatacgca atgcaaagta 2160
cagttcatag acttaaattt aattctaata agtaaactga ttttgccttg ctggggaaaa 2220
gttaaagcac taatccaatt gctaatgcag tcttgtctac ttctttggta cctagtgaca 2280
agtctaaata atgtatatat ttttatttac atattcagta atacaattct ctgctcaatg 2340
agtgatgttc ttctgccact tggtggtgct tgccagtttc agaatttgtt tcttggtggc 2400
actataacac taagtacaga gtaagtgcaa caaaattgca gcattcccat tgaaaaggct 2460
ttgcttcaaa ctgtttaata atttaaagga cctctgtgga agcaaccgca tttgttaacc 2520
agttacaacc agtaattaac tcctttggag ttttaactta cttttggcaa aacgtcttag 2580
gaagagcata tattattaga aagtatgcca aaaatttact tagcagaaaa ttcaaaaaca 2640
gttttcctct gctaagaggt tctctaaaat tctacttaca tagccaaact ctgaaatcct 2700
agcaggtcct gtttcattat cataattact gcataaacac ttttaaggac tttgccttta 2760
gtttcaagca tgacttattt tcataagcct gattagttac cacaccagcc ttgctatgga 2820
aaatgacatg ttctcattct ctgctgtaga gttgttaaat cttgatctat atttatgttg 2880
ccttctctgc tgaaagcctg tagcgaaaga aatttctaat tccttgtttt gcaatattag 2940
ttggcagctc tatctaatgg gtattctgtt tccttaaaga atttagctgc tctgtctaga 3000
agccgatttt ctgatgcctc caacgtctgg tctaattgat ctgttttaat ggagtcttcg 3060

tcggtgagga gcgagatgcc accgactaga atgctgggat ctgctgctta attgccagga 3120
gtgagagaca ctgagattca gaaatctttg gaggtgggag gggagaggga cagtctcgga 3180
cggaggcgga gatgtaagat aaagggatgg atttcacaca ggaaaaaaaa aaagatttcg 3240
ttgaggcact gaggtgctgc acgatcacat ctctcaaagg agaagttaaa aagcaaggaa 3300
gtgggaggag gttggaggtt aaagtactta aaaggattac tcgggtacaa tttgtttttc 3360
tgctggtgtc tgcaaaggat agatagtccc gttttcaaag tatatgaatg cctcttttaa 3420
gtgattggga atggacacta attgcctgtt aaatgttatc aaatgctctc ctaaattcag 3480
gggacacaga aagaggggca caaaaggaga atttaaatag aaaaagggag gatccggagg 3540
cttttgaaag cggggggaga agaaggagga gggataacag agaggaatag agaaggagag 3600
cggagagaag ataaacaaaa acaaaaacag gaatcactga ataatcacac accaaaaaga 3660
aagctcttcc ctatggggca tccaaaacac tgagactgca atagtgaccc cggtcatgga 3720
agaaagatgt tcctctccac ccttgtcccc gaaagctctt ggtcccgtta ctggcgacta 3780
aaattccatt aggctaaaga gtgtgtctaa ctgcctgaag aatgcagcag acggaaggcg 3840
ggtcccgcta tgccgtttgc ccttcccgct ggagagaatg aaagaaacgc gcagagccag 3900
agactcctgc cgagttagac cttctctcgt cgccccaggt caccggccat ccggcaaaga 3960
cccgagtaag gaacgcaggg tcactgcctg ggccaacaaa tggagcccgc tctccccttc 4020
ccggacgccg ctgcccggcc gatgctcccg gcaacccacc cgcggcgtat gcagaggagc 4080
ctttctcttt ctctcagacc acttgtcccg accaatctga ccttccaaac acatctgacc 4140
gcacctccca ggtggacaca ctaataggct acgggctgga gaggagcggg tgatgaggag 4200
agggattcaa acctgcgaac gcttgggctg ggtcggagct gcggggggcc tgggaggaga 4260
gaggggagaa gagagaagga aggagagcgc ctgccgggat ggctgagctg cctcggcgag 4320
cagccttggg gttgcacgct cttgtgggag atgctgctgt tgcttccagg tcggcaagag 4380
cggttctaac accatcgcct ctcaccctct ttcctgtaaa tccctagaga aacgtccctg 4440
gcctctccgc cgcgacattc ccagcctgca tccccctaca gcctaggcgg cgcgctcccg 4500
cacgctggag cgccggtcgc cagcaggacg ccctctcccg cgccgactcg cccctctctg 4560
ccctgctgct gctgctcctc tgacacctcc gcccccacca tctccagctc ggagagacgc 4620
cacccagccg cggcccgcac tcgcggcccg gggtcacgcg cggaagaggg gcgctagtcc 4680
ggaccccgcc ttcggtaggg ggcgtcctgg agcggagagt gaggcgaatg gtatatgagt 4740
gtgcgggtag cccaccctga agcccgagct tctcatttga gccatgcccc gcctagcccc 4800
actcggccca gcgcctggcg agcgagccca tctgtggctt ccgcggccgc ctcctccttg 4860
catccttgca cctactcgtc gacccctccc tcccgggacc tgcatcctgc tccaccaatc 4920
agagcccgac tgcctcttcc cacgtgaccc cgggcgggct gaggacctgc tgcttcccaa 4980
acgccagagg gatgcgggcg gcagagctcg agaggcggct gccgggctgc ggggcgcctt 5040
gactctccct ccaccctgcc tcctcgggct ccactcgtct gcccctggac tcccgtctcc 5100
tcctgtcctc cggcttccca gagctccctc cttatggcag cagcttcccg cgtctccggc 5160
gcagcttctc agcggacgac cctctcgctc cggggctgag cccagtccct ggatgttgct 5220
gaaactctcg agatcatgcg cgggtttggc tgctgcttcc ccgccgggtg ccactgccac 5280
cgccgccgcc tctgctgccg ccgtccgcgg gatgctcagt agcccgctgc ccggcccccg 5340
cgatcctgtg ttcctcggaa gccgtttgct gctgcagagt tgcacgaact agtcatggtg 5400
ctgtgggagt ccccgcggca gtgcagcagc tggacacttt gcgagggctt ttgctggctg 5460
ctgctgctgc ccgtcatgct actcatcgta gcccgcccgg tgaagctcgc tgctttccct 5520
acctccttaa gtgactgcca aacgcccacc ggctggaatt gctctggtaa gtccagaacc 5580
cccgtccccg accctttaac tccgcagaag aacacgcgta tccagcacag accagcctac 5640
cctagcgcgc ctcctcagcc cctcacctcc tactgcccta gacccctaat accacccacc 5700
tctatccaga gaaacaaggg gaactgttgc aggcccgggg gtgaggggtg gttctgggat 5760
gggcagaaag tgcaggtgta gcaggaaacc tttgcatgct tgcgcttaca ttggagctgc 5820
gaggattttg agaaatatta aacgggatgg ttttctgggt tcactgtttt gaaagagcac 5880
caatcctagg ggaaacactg aaacagaagc tttgtcatca ttaaagaaaa aagtcttact 5940
aggatgagga agaaataact ttatgagaaa gaatgagcga gaaagcaata aatcaaatgg 6000
tgactgcagg ggaatcgctg attcctggca aaggtgccat gaggtcgcac tggtctcccg 6060
ttgaagacca ggtcacacag attctagagg agctgggttt caatagaatt tctctctctc 6120
tctctctctc tctctctctc tctctctctc tctctctatc tatctatctc tctctctctc 6180
tcattccctt ctctcctagg cggcaaaaga cattggtttt gcagtccaga tatgcccctc 6240
tctttgcttc cctaagcttc aaggtagtac aggggagttg agaaaaagaa cactttgcgg 6300
gtctcccagg ccggagtggg catgactgag gctggtcagg ctccatgtag gcgagccgag 6360
ggcggaaccg acttcagtgg gcgctgactc ctccatttct ggacaggctt ctgtggagtg 6420
ggtcaggcac tcttcttgct cgctcgggtt ccttcagatt ctgacggcga acgcttggca 6480
ggcttcgctc tgctgaagct tcctaattaa atagggccag aggatgggag ttgctgcact 6540
cctagctggc atagcattcg gtttgacagc ctgtagtata gggtgtatgt aatttttcat 6600
cttctgtgaa tataattttg ctgtagttaa atctggctct gaataaagtg tctttcaaag 6660
atgtatataa gctgaagtgt atgtaacttt agagaggagg gaatgaccaa ctgtaactca 6720
gggtgaaagc ctgtatagtt cctagttatt actgatgtaa atgccaaaag gaaaattatt 6780
atgcatcatt ctaatttatc ctttacaaag acaagttgag atatgcaacc ctattagatt 6840

tgggtcaata gattgttctc ttttttggca gtttctaaat ttggcatttt aataaaactc 6900
aacatgtttc tataacttct tgattcatgc gtacatgtgt gttgtttttg aaagaataag 6960
tttcactttg ctattgccta atcacttttt agatgcttta ttatggtaat aattatgagc 7020
ctgcaaaaac aatttttgga aatgttgatg gctttgtagt ccaacacaga ctggtttgct 7080
tcattcctag cccttgcatt gttttaggaa ataactaact taaatgtgaa gttgacattt 7140
gcaatcaaga aattacatat ttaccagata ttttaaaggg gactgcataa actaaagaga 7200
ataaactggt tttgcagata ggttgtcaag aacttggcac cccgcttcca cccctgttaa 7260
cttagaggtg atcaatcttc atttgagcca aacagaccat cacagaaaac actgtgcctg 7320
tttatcttta ttattgaggc tttgtttcct ctttgtctgg atacatttca aataaggggt 7380
tgtttcagtc gttgaagcaa aagaacaatt aaagatgggg aaatggtaaa agggtattca 7440
gagatcatca ctagctcttt tccaaaatgt ggagttttgt ggtcataaat attgtccacc 7500
taatgagcaa aaaataaaaa taaaaaaaaa acaggaagca aatgttaagc tttcattcac 7560
cactgtcagt attaacgcaa gctttaaaaa atagcactat cagaaaagga tactaaagga 7620
gaattgacta gaaaagaatt gtggaaaatg gaaacgaata ttgatcactt aactagattt 7680
tgaggttatc agtagacagt gaccttgcag tacagctata gttgttggat ttaaaattta 7740
ggacaagtat tttaaagctt caaagtagtg cttttttttg ttaaaaatct gtaagatgtt 7800
ttaatgactg gagtgttctc tttgaatttg agg 7833

<210> 55
<211> 2501
<212> DNA
<213> Homo Sapiens

<400> 55
   gcaaattttt atattttgag gagagacagg gtttcaccat gttggccagg ctggtctcaa 60
   actcttgacc tcaggtgatc cacccgcctt ggcctctcaa agtgctggga ttacaggcgt 120
   gagccaccaa gcctgacggg tagtaattta ttttttatat aatttttttc ttcttttttt 180
   ggtagagacg aggttttgcc atgttgctca ggctggtcta aaactaccgg gctcaagtga 240
   tctgcctacc cgggcctccc aaagtgcagg gatcacaagc atgaaccacc atgcctggct 300
   gtaatttttc atatattccg gatattaacc ctttaccaga taatacagtt tgcaaacttt 360
   ttttttcccc attctgtagg ttgccttttc actgttggtc atttcttttg ctgtatagct 420
   atccttaagt ttgttgcaaa cccatttgtc tatcttttgt cttgttgcct gtgttttgct 480
   gtcatatcca agaaatcatt gccaaatcca ttgtcatgaa gcttttcccc atgttttctt 540
   ctaggagcac tatagtttca ggtcttatgt ttaatcttta ataagttttg tgtttttgta 600
   tatggtgtaa ggtaagggtc caacttcatt cttttgtatg tggttataca gttttctcag 660
   caccatttgt taaagacaca atctttcccc catgttctgg tgctttaaaa aaaaaaaaaa 720
   ttctggctgg ttacggtggc ttaggcctat aatcccagca ctttgggagg ctgaggcaag 780
   tggactgctt gaggctagga gtcccagact agcctggcca acatggtgaa accctgtctc 840
   taccaccgaa gatacaaaaa ttagccaggc gtggtggagt acgcctgtaa tcccagccta 900
   ctagggaggc tgaggcatga gaatcgcttg aacctgggag gcagaggttg cagtgagcca 960
   agatctcacc actgcacgcc agccggggtg acagagtgag gcagggtctt accctgtcgc 1020
   ccaggcagga gtccagtggc ccaatcatgg ctcattgcag cctacactgc cagggttcaa 1080
   gccatcctcc cacctcagcc tcccaagtag ctaggattac aggtgtgtgt caccatgccc 1140
   agcaaatctt tgtatttttt gtagagatgg ggtatcccta tgttgctcag gctggtcttg 1200
   aactcctaac ctcaagcgat cctcccacct gggcctctca aagcactggg attacaggcg 1260
   tgagccactg cgcctgacat ggtgcttctt aatttattct tactttttat ttttattttt 1320
   ttgagacaag gtcttgctct gtctcccagg ctggaatgta gtggtacaat catggctcac 1380
   tgcaacctct gcctctccgg ttcaagtgat cttcctgcct caacctctgg agtagtttgg 1440
   actatgggca catgccacaa cgactagcta atttttgttt ttcttttttt ctttctttct 1500
   ttctttcttt tttttttttt ttgagatgca gtttctctat gttacctagg ctggtctaaa 1560
   actcctgggc tcaagcgatc ctcccaccct ggcctcccaa agtgctggga tgacaggcgt 1620
   gagccacgtg gtgcttaaaa aaggcaacaa aaaacccccc acacactggg tatagaagtg 1680
   gcatgggcct ctatacactg tgagattctt ggtactagct acaaattctg tgtatactca 1740
   agattttcta gagtaggtgc aattaccccg ttttacagat gaggacacag aggctgagcc 1800
   gtagtgaccc acctaaggtc gtatagccag caaatagatg gaggttggat tggaactgag 1860
   gactttactc aagggctctc acaaaccctt gggggcttct cgctgcttta tccccatcac 1920
   acctgaaaga atgaatgaat gaatgcctcg ggcaccgtgc ccacctccca gcaaaccgtg 1980
   gagcttggac gagcccactg ctccgcgtgg ggggggtgtg tgcccgcctt gcgcatgcgt 2040
   gttccctggg catggccggc tccgttccat ccttctgcac agggtatcgc ctctctccgt 2100
   ttggtacatc ccctcctccc ccacgcccgg actggggtgg tagacgccgc ctccgctcat 2160
   cgcccctccc catcggtttc cgcgcgaaaa gccggggcgc ctgcgctgcc gccgccgcgt 2220
   ctgctgaagc ctccgagatg ccggcgcgta ccgccccagc ccgggtgccc acactggccg 2280
   tcccggccat ctcgctgccc gacgatgtcc gcaggcggta ggtaccatgg gggggaacac 2340
   ggactcaggg ggacaggcag ggcgctgggt ggggggtcgc ttcccctcgg ggtggccggt 2400
   ggccgctgct gacagacggg cgcgcatgcg tggggtggtg cggcgcgcag cggcagttgg 2460
   cgcgggcagg gtggcacttc cggtcgcgcg tgcccgggct g 2501

<210> 56
<211> 4138
<212> DNA
<213> Homo Sapiens

<400> 56

gaattccgga tccggatcta tgtccccgcc caaatctcat gtcaaattgt aaaccccaat 60
gttggaggtg gggccttgtg agaagtgatt ggataatgcg ggtggatttt ctgctttgat 120
gctgtttctg tgatagagat ctcacatgat ctggttgttt aaaagtgtgt agcacctctc 180
ccctctctct ctctctctct tgctcatgct ctgccatgta agacgttcct cgtttcccct 240
tcaccgtcca gaatgattgt aagttttctg aggcctcccc aggagcagaa gccactatgc 300
ttcctgtaca actgcagaat gatgagcgaa ttaaacctct tttctttata aattacccag 360
tctcaggtat ttctttatag caatgcgagg acagactaat acaatcttct actcccagat 420
ccccgcacac gcttagcccc agacatcact gcccctggga gcatgcacag cgcagcctcc 480
tgccgacaaa agcaaagtca caaaggtgac aaaaatctgc atttggggac atctgattgt 540
gaaagaggat ggacagtaca cttgtagcca cagagactgg ggctcaccga gctgaaacct 600
ggtagcactt tggcataaca tgtgcatgac ccgtgttcaa tgtctagaga tcagtgttga 660
gtaaaacagc ctggtctggg gccgctgctg tccccacttc cctcctgtcc accagagggc 720
ggcagagttc ctccctccca ccctggagcc tccccagggg ctgctgacct cctcagccgg 780
gcccacagcc tagcagggtc caccctcacc cgggtcacct cggcccacgt cctcctcgcc 840
ctccgagctc ctcacacgga ctctgtcagc tcctccctgc agcctatcgg ccgcccacct 900
gaggcttgtc ggccgcccac ttgaggcctg tcggctgccc tctgcaggca gctcctgtcc 960
cctacacccc ctccttcccc gggctcagct gaaagggcgt ctcccagggc agctccctgt 1020
gatctccagt acagctcagt ctctcacagg ctccgacgcc ccctatgctg tcacctcaca 1080
gccctgtcat taccattaac tcctcagtcc catgaagttc actgagcgcc tgtctcccgg 1140
ttacaggaaa actctgtgac agggaccacg tctgtaatgc tctctgtgga atcccaaggc 1200
ccagcccagt gcctgacacg gaacagatgc tccataaata ctggttaaat gtgtgggaga 1260
tctctaaaaa cagcatatca cctccgtgtg gcccccagca gtcagagtct gttccatgtg 1320
gacacagggg cactggcacc agcatgggag gaggccagca agtgcccgcg gctgccccag 1380
gaatgaggcc tcaaccccca gagcttcaga agggaggaca gaggcctgca gggaatagat 1440
cctccggcct gaccctgcag cctaatccag agttcagggt cagctcacac cacgtcgacc 1500
ctggtcagca tccctagggc agttccagac aaggccggag gtctccttct ttgccctcca 1560
gggggtgaca ttgcacacag acatcactca ggaaacggat tcccctggac aggaacctgg 1620
ctttgctaag gaagtggagg tggagcctgg tttccatccc ttgctccaac agacccttct 1680
gatctctccc acatacctgc tctgttcctt tctgggtcct atgaggaccc tgttctgcca 1740
ggggtccctg tgcaactcca gactccctcc tggtaccacc atggggaagg tggggtgatc 1800
acaggacagt cagcctcgca gagacagaga ccacccagga cagtcaggga gaacatggac 1860
aggccctgag ccgcagcttc agccaacaga cacggagagg gagggtcccc ctggagcctt 1920
ccccaaggac agcagagccc agagtcaccc acctccctcc accacagtcc tctctttcca 1980
ggacacacaa gacacctccc cctctacatg caggatctgg ggactcctga gacctctggg 2040
cctgggtctc catccctggg tcagtggcgg ggttggtggt actggagaca gagggctggt 2100
ccctccccag ccaccaccca gtgagccttt ttctagcccc cagagccacc tctgtcacct 2160
tcctgttggg catcatccca ccttcccaga gccctggaga gcatggggag acccgggacc 2220
ctgctgggtt tctctgtcac aaaggaaaat aatccccctg gtgtgacaga cccaaggaca 2280
gaacacagca gaggtcagca ctggggaaga caggttgtcc tcccagggga tgggggtgga 2340
tccaccttgc cgaaaagatt tgtctgagga actgaaaata gaagggaaaa aagaggaggg 2400
acaaaagagg cagaaatgag aggggagggg acagaggaca cctgaataaa gaccacaccc 2460
tgacccacgt gatgctgaga agtactcctg ccctaggaag agactcaggg cagagggagg 2520
aaggacagca gaccagacag tcacagcagc cttgacaaaa cgttcctgga actcaagctc 2580
ttctccacag aggaggacag agcagacagc agagaccatg gagtctccct cggcccctcc 2640
ccacagatgg tgcatcccct ggcagaggct cctgctcaca ggtgaaggga ggacaacctg 2700
ggagagggtg ggaggaggga gcacagagac tggctggggt ctcctgggta ggacagggct 2760
gtgagacgga cagaggctcc tgttggagcc tgaataggga agaggacatc agagagggac 2820
aggagtcaca ccagaaaaat caaattgaac tggaattgga aaggggcagg aaaacctcaa 2880
gagttctatt ttcctagtta attgtcactg gccactacgt ttttaaaaat cataataact 2940
gcatcacatg acactttaaa taaaaacata ataactgcat cagatgacac tttaaataaa 3000
aacataacca gggcatgaaa cactgtcctc atccgcctac cgcggacatt ggaaaataag 3060

ccccaggctg tggagggccc tgggaaccct catgaactca tccacaggaa tctgcagcct 3120
gtcccaggca ctggggtgca accaagatca cacaaatccc tgccctcatg aagctcatgc 3180
tctcatgggg aggaagacag acatacaaag agatctagaa tgtgaggtca ggtgttgaca 3240
agagccctgg aggaatagag cagggaaagg tcagaaaagg aagacccagg gtctctagag 3300
gaggtgtcag ggaagggatc tcccaagaat gccctgatgt gagcaggacc tgaaggcaat 3360
ggggagggag ccgtgaagac ccctggaaaa gcagattcca cacagggaaa tgccaaggtc 3420
ggaggtgcta aggaaatagg agacacactg ctgaccttga cctagtagga cacacacaca 3480
cacacacaca cactcactca ctccagggct gggggatgaa gagacctgct caggacccag 3540
gaccccattt ttccacccta atgcataggt cccaatattg accgatgctc tctgctctct 3600
cctagcctca cttctaacct tctggaaccc gcccaccact gccaagctca ctattgaatc 3660
cacgccgttc aatgtcgcag aggggaagga ggtgcttcta cttgtccaca atctgcccca 3720
gcatcttttt ggctacagct ggtacaaagg tgaaagagtg gatggcaacc gtcaaattat 3780
aggatatgta ataggaactc aacaagctac cccagggccc gcatacagtg gtcgagagat 3840
aatatacccc aatgcatccc tgctgatcca gaacatcatc cagaatgaca caggattcta 3900
caccctacac gtcataaagt cagatcttgt gaatgaagaa gcaactggcc agttccgggt 3960
ataccgtgag tgattccccc atgacctctg ggtgttgggg gtcagttcta cttcccacac 4020
acaggattat caggcctggg ctgtgctgtg gccccctctg cattacgcac catgttaggg 4080
tttgggcatt tagtgcagga tacacacaga agagacaaac ttcaacagat cagaattc 4138

<210> 57
<211> 11222
<212> DNA
<213> Homo Sapiens

<400> 57

ggcctggcgc tggtccagga gggtagactc tgccagggag gcacaagcta agctgggggt 60
ggggtgggag ggcctgctgg caggagggag gcactgccca gactggcctc caaaggctgg 120
ttcccagaag gaggaggaag agagtagtga agatcgtggg gagtcctggc tttgaggagc 180
cagagttctc cttcttgcca catctttgat ctcccttggt ggtgtcttaa gcccagattt 240
accaaaggga attgtcagct gtccaagggc tagcaaattc ctaggtcacc tagattggat 300
tttctgacca taaaaactgt gggccaggtg cacagctgcc tgaggggctc aaacctgtgc 360
aggtgggtgc tggaatcccc tttgctatag cgttctcttt gtgtgcatgt gaccagaggt 420
gagagtggct ggagaccgaa gatggcagag cccgtctttc tccaccagac tgaggccccc 480
gacttgcctt gcctggggag ggtgctcagt ggaagtcctg ggatgttcag tctggacaag 540
aaaagtcttg ggtgagcctg ctccttctct tgtgaggcag actcagctgg gcctctatat 600
ccccaaggag caggcggagc tgccgcaagg aagtgcacgt gacatggagg catcttttgg 660
ttcgacataa aaaaggattt ttaaagcttt aggtagtgag ctcaccactc ccaggagtat 720
tcaagcagat gccagatgat gatctgtagg gaaaactatg tttagtggga aattggacaa 780
aataccgttg aaggcctctt ttagtcacaa gatactatgg ccctataatt ctagatttct 840
ctgcttctag ctctctataa gtctggaatc tagaacccaa gtttctccgg tgctgaaaag 900
ttgaagactt tgacactcta gaattttaat tctgggatct caagattcca gaattgggag 960
ccttgaggat gattctacaa gtctcagttt tggattcaaa ggatcctgtg atatgaaagg 1020
gaaaaagagg aggtctgact tggtgggatc cagttttgcc ccactgggta tcagactaat 1080
gtgccctgca ttcgtgcgtg tgtgtgcgtg tgtgtagcat gagcgtgtaa ggctgtagaa 1140
agttttgcac acaaaggcag cctggctttc ctatcatgtg tctgcagctt ctctgccctg 1200
gccataatga aggctgtcac agggataata atagccccca aacagagctg gtgactccag 1260
gtagccatag caactgtgag gtaacgctgc tctgttgtag agatgtgtga gtacaaacag 1320
atgtcggtgc cttcccatta gcttaaatgg taaaacttaa aaggtccatc aacttcaaag 1380
ctacaggttt ccatgagcat gccagttaat gggggcgctt gccaagcggg tgatagcttc 1440
atattaaaca tttcagcatc agtaatgggt tgatgagatt ttaaatgctc aggccatccc 1500
cagacaactc aggaggccag agacgggaga agagctggga ttcgggtagt aatcagccct 1560
gccctgcccc gcaccctcag gggtgttctg acactctcgg gagggcacag gccagagagc 1620
atttgaaaac cgcacattgc gtctcaatct ccaggagtct cagatctgcc tcttggcctt 1680
ccccagcgca ccaggcaggg ggccccaggt cagatgtggc acagctcaat gccatctcct 1740
tctttctgcc ctcctctctc acaccctgga ctcacctcca gcgagtgacc agcagtaagg 1800
gtcaaactgc tgagagtctg agaccggggc agaccctctc agagttgtct ttccttctga 1860
ggctgggcct ggcacagact ggggcctcag taatgggctt tcaaatgaat gaatgaacaa 1920
aggaacaaac agatgactga ctgactgaat gaatgaatga gatccccttt ggcatcccca 1980
tggcttcttt ttttcttttt gagatggagg ctcgctctgt tgccaggctg gagtacagcg 2040
atctcggctc actgcaacct ctgcctcccg ggttcaagtg attctcctgc ctcagcctcc 2100
caagtagctg ggactacagg ttcgtgccac catgcccagc taatttttgt atttttagta 2160
gagacagggc ttgaacatgt tagccaggat agtctcgatc tcttgacctt gtgatccacc 2220

cacctcagcc tcccaaattg ctgggattac aggcgtgagc caccacgccc ggccccccat 2280
ggcattttag ctacttattt tgttactgct tcttccactg ctaaagagat gggcctttgg 2340
aggtcggctg gcctagcatt tggggttgac ttctggaatg gtcactaggg aaggggcgcc 2400
agaggtgcag ccggtgcctc tgaccctttt ggtcgctagg agtcagccga ctcagtacac 2460
aggactcact gaatggagac acaaggctcc tccagggagt ggcggctcat ggcaatccta 2520
gaatggtcac cagccaggct ttagagaccc acacagaggg cgttctgacc caaagttgca 2580
ctggggaact ccaagtttgg ggattctttg aatttaactc tttttctagc tacatttcct 2640
attatttgtc caattcttac caaacatctc tgttcacatt ctgaagctgg gatctgactg 2700
gcagagctag tagatgctga ctattcagat ggagccctga cattggcttt ctcagcttgg 2760
ctgtgactgg cagcaggttt gcgggagaac tgtgtgtccc agaacatgac tggctacacc 2820
tgcacctcag caagattggg gcagggcagt tatcttcaaa aagctgtgta ggtggggcag 2880
tcattactga caaatccagt gcagacccag gatggcccaa acactggctt atcctttctg 2940
aatctcatct cccacagctg taaagcgggg tggtgctcgc tacctcacag aggtgttgta 3000
aagattagat gtaatcttgc caagcagcca ctttgtaaac tgtatagtct tatgcagatg 3060
gaaggaaggg cctgtgccta ccttgatcat agcactaaac aaactgtact gtattttcat 3120
tcctcttagt tatctcccta aaaagactct gagttccttg aacacaggaa ggtgttttat 3180
ttgattttgt tatcctcagc atgtagcagt gtctgacaca cagtaggtgc tctatcactg 3240
tgagagggat ggatggatgg gtggagttac agatggatag aaggatagat ggagggatgg 3300
gtggatgatg gatggataga tggatggagg ggggatgatg aatggaggga taatgagtgg 3360
atgaatgagg gaatgggtgg atggatggat ggagggatgg aggaacagat agatagatgg 3420
agggatgggt gggtgatgga tggatagatg gatggaggga gggatgatga atggagggat 3480
aatgaatgga tgaatgaggg gatgggtgga tggatgaatg gagggatgat gggtggatga 3540
atgaattgag ggatggatgg atgaacacat ggatggatgg atagatggat agatggagga 3600
actggtggat tttggatgga tgggtggatg gatagatgaa tgaatgcctg gatagacaaa 3660
gagatgatgg atagatgaat agatgaatta agggatgtcg gatagatgga gggattgata 3720
gatgttggat ggatgggtgg tggatggata gatgagtgaa tgcatggata gacaaagaga 3780
tgatggatgg atgaattaag ggatgacaga tggatggatg gatgagtaac tggatggaca 3840
agtggataaa tggatagatg gttgaatacc tgaatggatt gaaggaggat gcatggatgt 3900
aagataaggc taatcatcct ccactctctt tctttgcaaa accatccacc catttactca 3960
ataaacattt attcagttca aacttggcac aaagcaccat gtgaggccca agagatacgt 4020
gggttaataa aacagagctc ctgccctcct gaaaactgca aagaaagggg cgtggcttcc 4080
tgagttcaaa tcccaactct gccagcgact agttgtacat cagtgatgtt tccctacttt 4140
ctctcaatta aatagggata atgtcagtac ctatcacatt gggaggtctt gcggggatta 4200
aatgagttac caaatgccaa gtgtttggga cagggcctgg cacccagcaa agtctcttgt 4260
gagtgctggc tgctattatc ctaatggaga agatggcatg aaaaccagga aataggatgc 4320
cctttgggaa gcaatgcaac aggaacttac acaaagaaag gaaaggagga agcaattagt 4380
ggtgtctcaa aggagtatgt caagaaaaac ttttcagagg gaaacctttg agcagggtca 4440
tgaaaacagg agttctctaa gagattgtgg acttgcctgg gaccacctgg ctataagcac 4500
aaaaccatcc ggttcctttc tgtcacttct ggctgggtga ggggtctctg gcaaaggggc 4560
agaaggtgcg tgagaggttg cgaatggcca ggactgtcct ggggccagcc ggggcacctg 4620
gtggccaagc ttagaaacat gacaggtcct cttgggaggg ctgaccgcag ggagcgttgg 4680
gtttcaggct gctggcgtcg gcttctgtgg tgccctttct gtcggctatg agagtccaga 4740
cagtgcccaa cctcctcccc ttctttccac acgcacaacc accccacccc ctgtggcctg 4800
agctgtcctg cctcgccaca atggcacctg ccctaaaata gcttcccatg tgagggctag 4860
agaaaggaaa agattagacc ctccctggat gagagagaga aagtgaagga gggcagggga 4920
gggggacagc gagccattga gcgatctttg tcaagcatcc cagaaggtat aaaaacgccc 4980
ttgggaccag gcagcctcaa accccagctg ttggggccag gacacccagt gagcccatac 5040
ttgctctttt tgtcttcttc agactgcgcc atggggctca gcgacgggga atggcagttg 5100
gtgctgaacg tctgggggaa ggtggaggct gacatcccag gccatgggca ggaagtcctc 5160
atcaggtaaa aggaagagat tccattgccc ctgccaccca caccctaaga tcaagggtgt 5220
tcagctgcaa ggtggaaagt ttgcacgtgg ggtaggtcag ttggctgcat tagttaaggg 5280
tgttagaacg gtcacttgct ttttctttgc ttttaagtgt cagggattgg actcaggaga 5340
gggaaaggag ccatttcagg ctgatgtcag cagctggagg aagcatgaga atcaaaccta 5400
ggatgctcag agtccaccag gaagaatttt agaattatag acagtcagag ttaacaaggg 5460
tcctgagaga ttttgtacag ccacctctct tacaggatga ggacaaaaag ggactgagaa 5520
ggggaggaca tttccagagt cacagctcat taaatgctct taaagtgtca aggttaagac 5580
atgctcttca aggggagaca gatctggttc tagacttggc tctgccactg agccactggg 5640
tgacctttgg gaaagtactc aacctctcgg agcctcaatt tcctctcctg tacagtgagg 5700
ggatatccta atatctatat cctagaggag atgtgagaat taaataaaat aatgcatgca 5760
agaggcctgg catggttcct ggcatatact gagtcctaga aatgttagta gctattactg 5820
atgaagccca ggctagggac ctttcaaagc attgcaatta gagaacagaa gatagaggct 5880
cattagtgac cttcgatgtt gagtatgtct ctagtttgag aggtctgaat gatgtggtct 5940
gcaagtatat cctgccttct accacaaggg attccagaat acaccaaaga aaacaaaatt 6000

ctgaggtttg taaatagagg gtggctgtgg tttgtacata gaagctcatc tcctcgttgc 6060
cttctatccc aaaggtgata cactcttctc ttggcccctt ccctcaccat tctgagctgg 6120
ttccctcaga agtctaatag gttaagaatc aacgtttctg ccaacgggag gaaggaagtg 6180
ggcgccgggg ttgccacaag tgaatggatt gtctttggca ctttaatttg cagattaatt 6240
tctccttatt tgtgagccag agccaatttt cctcttccac tccgtcgtcc tttgggtctc 6300
ccttgggctt tacccatttc cagcttccgt ctacctcggg agcatggctg gtcctggaag 6360
ctccgttatc taaaatttga gccgttggtg atccgtgcta gaaacacttg aggaagaaga 6420
gttgagtttt ctgttgactg taatggcatc gtagtgtgtt tatgtctttc ttctcctgtc 6480
cctgtcatct ggctgcaaca ggccctgttg gattgcacaa agtgctcccc cgaccaacac 6540
acacacacaa attgacatca actcctgggc cacagatatg ccatgtgacc ttggacaagt 6600
ctctccacct ctgggcctta tttctctgct ggttgagcga agggattgtc ttccatggtc 6660
tccgagatcc cgtcccacgc tcatgcccta gaattctctg agtccttgat gcacttttgc 6720
ctttggcgag gaggcaggac agtcaggcgt ggaggtagga gaccagagtt tgcagagcag 6780
aggtctttcc aaggtgattt gggagctgcc agacctaagg cagtgacttg tacgtcttga 6840
aagagactca gggtgttgca tcatcaggga ggatcacgga ggtttgccgg gggccgggag 6900
ccagggataa gacataatgc ctagggttgc tggtccctga aaggtcccag aggcctggtg 6960
ccctgtcacg tcttaggttg tgggacatgc agagactgtg ccagctggtt ctgccctctc 7020
agtatttggg gtacagggag gatggcctcc ctgggggcag ggtggaggag gctcatgccc 7080
aggactgcaa atgccacccg tgagagttgg catcttgact ttgttctaga tactgactct 7140
ggatttctaa actgaccttg aaaggttatg tctccaatct tctgattgta aaagagggca 7200
aattccatga agcccagtct gtaaaacact gaggccttca ctgcacgtac acaaacataa 7260
aattctagaa cacagaagct ggaaggtcac ttggagattc tggctccagg agtggggctt 7320
cggaagggag ggggctgctg agtgggctcc acacccctgt ttttcctttc tttactcaaa 7380
ccttttttgt tttatttact ttggctgttt tggggagcgg ggggaacaaa ggatttcaag 7440
gtcataaaat gtttgaaaat caccagtggt ggtcactact tctcattaca gggaggaact 7500
gggcctgaga gtctccaccg gttaatggca gccacaggca ggaccaaaaa ttcctgggtg 7560
atggtgatga taatgataat gataatgata atggtagaga tgatgaggag gagaaagaca 7620
gcaaccattt attcagcaca tactgattgc tgggctctgt ttaagtgttt cactcactga 7680
agcctgacaa caatcttatg aggtagatat gattatgatc accacattta acagagaggt 7740
taagtaactt acgccaagtc acacagcaag taagaaagga gctggaattc aaattcaggc 7800
agcttggatc cagaatcctt gctccttttt tttttttttg agatggagtc ttgctgggat 7860
tacaggtgca tgccaccatg cctggctaat ttttgtattt ttagtagaga ctaggtttcg 7920
ccatgttggc caggctggtc tcgaactcct gacctcaggt gatctgcccg ccttggcctc 7980
ccaaagtgct gggattacaa gccaatgagc ccggcccaga atccttgctc ttgaccacta 8040
tgctatactg ccccatgacc ttgccacacg caagacgggc tgccacccac gctctggttc 8100
ttttattcta gtagagccag aggggtgttc atggatctga caatgtgctc catgttccat 8160
gtgagtgtcc aattgggcct cagttcagag aacagcagga aaatggcctc ccttctctct 8220
gacttggact gaagatctgt ctgatgaacc aggcccactg acagcagggt tgaagctgga 8280
aacacaggcc agcggggctt tctgaaatac tagagcaaac gccctccgtc catttccaca 8340
ggctgagggg ctttcatctg ttcattttcc ggatcaggca aatccaggag aatggccttc 8400
tgtggctgcc tggggggctc ctagctgcaa ggcctgggcc cacaggaggc aggcagctgt 8460
gtggtgccgg aatgatcttg cggggagaca aattagcccg attctgctct gctgtttaaa 8520
gtaaatatgg ggtggggtgg ggtggctcag gcctataatc ccagcacttt gggaggcagg 8580
atcggggaat cgcttgagtc caggagttcg agaccagcct gagccacata gtgagacccc 8640
ccttctctac aaaaaataaa caaaattagc tgggcgtggt ggtgtgtgcc tgtagtccca 8700
gctacgcggg aggctgaggt aggaggatca cttgggccca ggagattgag gctccagtga 8760
gccaagatca caccactgca ctccagcctg ggcaacagag caataccgtg tctcaaaaaa 8820
atattaataa agtaaatatg agctctctcc ctggctgtct ggtgttgagt ggaaagaaag 8880
gcattggcgg gtctagaccc ttttggttta catatggatg agcaatgatc cagagaggct 8940
aagtgacttc aacatcacag agcacattgg cgccagaact gggaccggaa ctcagcttcc 9000
gtaactgtca gtcccatgtt ctcttcccta gatatcagag ggcaggggca ggccccaaaa 9060
tgtctgagac ccccagctgc cttgtggccc aaattcttgg ccctgctctg cctcagacag 9120
tctgctttca gcgtcctgac ctctgaccat cgcctgtgca tgccaggtct tgcatctgtc 9180
aggcgggagt tcaaggatca ttgtcgccag ataataatag tcaggagctc agggaccctt 9240
ggatgaaagg tgtcagagac aagccgtcag tgacgcacga ggatttggat aaggtcctgg 9300
agcgaggtgg caatgttggg cacggcggca gaccatgccc aacatggggg aagggagtgt 9360
atgtggttca ggcttgtgcg tgtctctgtg tgtgtgtgca ggtgagtatg gcgtgtgctg 9420
ggatatattt atgtgtacct ctgtgtgagt gtgcaggccc atatgtgagt gtgcacacac 9480
atctgtgaga gcctgcatgc atgtacacgt gtgaggtgtt gatacatgtc catgtaggta 9540
tcagtttgcc tgtaaatatc tctgtgtaac aataacaaag tccgaatagt catcaagagt 9600
atagacatga agccagactg cctgggttaa atccccagct agttagtctt tcggtgcctc 9660
tgtttcttcc tctgcaaaat ggcgtctacc ccatcaggat tttaaaatca gttaatatgg 9720
ctgggcgcct gtaatcccag cactttggga ggctgaggca ggcggatcac aaggtcagga 9780

gatcgagacc atcctgccta acatggagaa ttctgagaat tgctcaaacc caggaggtgg 9840
aggttgcggt gagcagagat tgcaccactg cactccagcc tgggcaacag agccagattc 9900
catctcaaaa aagaaaaaca aaaaacaaaa aaaccatgaa ctcattttca ggttgaggag 9960
ctcagcatcc tggttgtgaa ataccctcct cataaaaccc tgggatggag actacgggga 10020
tcaggtgctt ccttgtgaca acttctgggc atggtggctc agggcgcaaa ctggagtgtg 10080
gccacaatac atactgtgta cttttacaag gatgtcacag agcctgggta tcataaaaga 10140
ggagcttttc aaggaactga aaccattaga caggagagag agccctgggc agacagggtt 10200
gcccgtgcca aacatttcag ctgtggcaca agggaaaggg tgggagttat gaaactgttc 10260
cattttgggt ttaggtctgg gctctgccgc tagctagcca agtgaccttg gccacttatc 10320
tctgtggtct tccatgagta aaaggcggaa actcactcct acccagaggg caggtctgac 10380
tccctttaac cagcacccac ctgctcacag caggaaggac tgaggtctaa agctggaggt 10440
gggcaggaag gactgaggtc taaagctgga ggtgggcagg aaggaccaag gtctaaagtt 10500
ggaggtgggc aggaaggacc gaggtctaaa gctggaggtg gctgctcaga gtcccagcag 10560
aggcctctgg ggcacctcac tgagtgcctg gcaggagtgg gtgcctgtct cagggctggg 10620
ttgagttgct cccaccagga cccttcgtca tctgcacagt gaggggactg ggaggttcag 10680
agagtcacag cttgggctca aaacaagcaa gaggtttctg agtgtgagga ttgctctgga 10740
gtggaatggc cctcacaggt aggagtgagc ctcctgtagc tagaggtatt taagcagctg 10800
aaggacaatc cctgggcagg aagctgcaga gatggtcgca gcgtggacta gaactgctgt 10860
tttggtcact cagacctcat tccagcctgg cttctctgga cagcacccct gcaatagtga 10920
gctggtgact ttacgcctca gaacctcggt ttccacatct gtaaaatggg aattatatga 10980
cactcactat gtgccagaca ccctgttggt acatagcaca cactatctca cttaatcctt 11040
caagtaggga caagttatcc ccatccctta tatgaggaag ctgaggcaca gagaggtgaa 11100
gtgaatggcc caaggtcaca cagctgggaa gacagggagc taaacttgaa ctctagtctg 11160
gctgccccca gacctcacac cgcacctccc atgccgactc cagccttccc tgtgcccaca 11220
gg 11222

<210> 58
<211> 5952
<212> DNA
<213> Homo Sapiens

<400> 58

atattcctga tttctatatg agtatatgcc cgagtagctt ggactacagg tgtgcagcac 60
cacacccagc taacttttaa atttttgtgt agcgatgaag tctcacttta ttgcttaggc 120
tggtcttaaa ctcctgggct cgagcaatcc tcccacatag gcctcctaaa gttctgggat 180
tacaggcatg agccacagca ctcgagcaag atgatcttct aagacaggtg aagatgttag 240
tgtctagaac ttcaccttca ggggcacgta ggtatgaaag gtcatatcta agtgagtgtc 300
atgtcagtcc ttctagaaac tcagctgctc cttttcaaat ccagcctaat aaaaggaggc 360
ttggagaatc aatgcacata agtcagaatt atagaatgca cagtatgatt actaggatgg 420
ataacatgtg gtctgaacaa acttctcatc cattacactc agccctctca ggttaatgga 480
actcagatca agtatattcc tgaagtcagt actgtgtgac gaacaggggt ctggtggaag 540
tgatgctaat agggctttag gccagcttta gagcccttct ctctctctct tttcgttttt 600
tgttttcttt ttgtttgttt tttgtttttt ttttttagac agtttccctc ttgttgctca 660
ggctggagtg caatggtgtg atctcggctc actgcaacct ccgcctccca ggttcaagca 720
attctcctgt ctcagcctcc cgagtagctg ggactacagg cgcccgccac cacagctggc 780
taatttttgt atttttagta gagacagggg ttttaggtca ggctggtttt gaattcttga 840
cctcaggtga tccgcccacc tgggcctccc aaagtgctgg gattacaggc atcagccact 900
gcacctggct caagaaacat tcttaagcat gcttgatctt ggtaaatgcc cctgatttct 960
tctgtaggaa aaaaccctag agatcgaatt tctgtgtcaa agaataagta aaattctaag 1020
gcttttgata cacatttcta agtttccacc agaaaaatta taccaatgta tactcccact 1080
tgcaatgtat ttaagtgcct acttccttga atccttccta acacaccagg tgttaacatg 1140
atttttatta atttggtagg ttaaaattct gtctccattg aatttacatt tctttgctta 1200
ttaatgtggc taacaaactt cacgtttatg aagactagga ctggacagtg gaggccagat 1260
acctctggct ttttgtttag atgaataaat tttctatttc tcttaatcca ttcaagttga 1320
gttctctgtc actgaaacct aaactatact aaccaatata atcctttgag gcatactgaa 1380
gtcttaaaac ttgtatggtt attcattatg ttttatgggt ttttttctta tgtttggaag 1440
cccataggaa gatctcataa atatttacca aaatattttt ccatttctta ttgggtcaag 1500
atttgaattt gaattcttta tctagaattt agtgaataat tattaggaaa agtctttttt 1560
tttttttttg agatggaggt ttgctctgtc acccaggctg gagtacagtg gcgcaatctt 1620
ggttcactgc aacctctgcc tcccgggttc aagcaattct cctgcgtcag cctccctagt 1680
atctggaatt acaggtgtgc accaccacgc ccagctaatt tttgtatttt tagtagagac 1740
ggggtttcac catgttggcc tggctggtct cgaactcctg acctcagatg atccacctac 1800

ctcagcctcc caaagttctg ggattacagg catgagccac tgcgcccatc cagaaaagtc 1860
tacagctttt attcttttct aatagtttaa ccaattatct gatcaaaatt tacttcattc 1920
tttctcccac tgatccaata gtacctttac tagatgttag agtagccact taaaatgtaa 1980
attggatcac atcaaagatc tgctgttgac cacattccct tgcactttta gagagcctac 2040
aaagtcctgc aagagctcac cccagcctct ctgcaacctc accttgcacc actcttgctt 2100
cagacacatc agtctgagtt ctacatcaaa cttgtctgaa tatattccaa gtatgctgac 2160
ctttataatc atgctattct ctcagcctag aatgcttttt tccaactctc caagcagctg 2220
tcttctctga taagatgcta cctcctcaga gaggccttct ctgaccacag tcataggcat 2280
gtttatgtcc tcatttcagt ttaacataaa atttacgtaa ctgtacacct ctttatcatt 2340
tgacactttt aggaggacat cacttctgta atagagcaga aataaacaga ttgatgtaca 2400
tggtggatcc cccatggata gagtttgtca tttaaaatgt cttcaaatct attacatgta 2460
atttggcatt aaaccaaaat gttattatgt aagcgaatgt ttttaaagtc taaaagttct 2520
tttaaaacaa aatagacccg ggcgcggtgg ctcatgcctg taatcccagc actttgggag 2580
gccaaggcag gcagatcaca tgaggtcagg agttcaagac caacctggcc aacatggcaa 2640
aaccccgtct ctactaaaaa tacaaaaatt agccaggtgt gatggcgcat gcctgtaatc 2700
ctagctattc aggaggctga ggcatgagaa ttgcttgaac ccaggaggcg gagattgcgg 2760
ttagccaaga tcacaccact gcactccagc ctgggtgata gagtgagact ctgtctcaga 2820
aaaaaaaaaa agaaaaagaa aaagaaaaaa aaaaagaaat tctaagtgat aaagcattgt 2880
gtaacagttt gaggctgggc acagtggctc atgcctgtaa tctcacactt tgggagaccg 2940
attgggaaga ttgcttgagc ccaggagttg gagaccagcc agagcgacat agtgggacct 3000
tctctctaca aaaacattta aaaattagcc aggtacagtg gtgcacccct gtagtcccag 3060
ctatttggga ggtggcaaga ggatctctcg agcctagaag ttggaggtgg cagtgagtag 3120
tcatcttgcc actgcactcc agcctgggtg tcaaagggag actcccctct ctagaaaata 3180
ataaaaaaaa aagtttgaca gttttcttgc ttgtgctaca taaaataatg gtacatatta 3240
caattgaagg catcttataa tagaaatgcc ctttcctgtt cccttttaag gctgacccag 3300
tgctttaaga ggctgataca gaagggtaaa gttaagtctc cacaaaatcc agggaagaga 3360
ctgtgaaact catcttagaa ccttgtatgg agtcacagct gaactaggaa aaaaaaaaaa 3420
aaaaaaaaaa ggaaagaaag gcattccaga gataaagaat agactaagga gaggagatat 3480
ttgaagccat aatgattgag aatatgtcag acttgaaatg aatcttcagt tccctcaaca 3540
agacaaataa aactaactcc atacctagat acatggtaga aaaactaaag aattctgctg 3600
accaaggtat taaaagtaac taaagagaag tggtgtgaag aaagcaagag agaaacaaca 3660
aatcctgtcc atcctgtaac aattgaaaat ttctggctgg gcgtggtggc tcacgcctgt 3720
aatcccagca ctttgagagg ccgaggcagg tggatcacct caggtcaggt gttcaagacc 3780
agcctggcca acatggtgaa accccgtctc tactaaaaaa aaataataat aataatacaa 3840
aaattagccg ggggtggtgg taggcacctg taatcccaga tactcgggag gctgaggcag 3900
gagactcact tgaacctggg aggcggaggt tgcaatgagc tgagatcgcg ccactgtact 3960
ccagcctgga tgacagagca ggactccatc tcaaaaagaa aggaaaagaa aagaaaaaat 4020
attaaatgtg tacgctcttt gactcagctg tattacttca aggagttgat atcaccaaaa 4080
ttgcctaagt gctcaaaggt gtttgtagtt aaacaacagg agattgataa attatgttat 4140
atacatgtga tgctatgttt taaagaggta ctgatatgat aaaagatgta cgtggcataa 4200
aattaaatgt actttattaa gtacttttcc aagtgtttac ggaatgagtg catttttgaa 4260
aaaaaaaaag tgtattcgaa cttttaaaaa agctttaaaa gctttataca atgaacgatt 4320
gagtgattat aagagctggc gggggaatgt taagaggatg atagggagct aagtttaaca 4380
gaacaattca cctctttatc ttgtgacacc tacgagcgca tcaattctgt aattgaaaaa 4440
taaagtgcat atttgcagca gctgtactct cttcaggctg caaggaggct tttcctcccg 4500
gtaggcttga tttgcatttc actttcactt tcgtggctgg aaactttcta cccacgtagt 4560
gggaggctga ggagccacca taaagctggg gcttgacgag ccgggaccgg gacccgatct 4620
ccacatatgc ccggacttgt tctgcggccg ggttcaggag tcaaagaggc ggggagacct 4680
gcgcgacgct gccccgccct gcgcccgctt cctccaatgt atgctctagg gggcgggcct 4740
cgcggggagc atggacacga ttggccctaa agtcttcccc gcaaggccgt gggctggaca 4800
gcgtggtgac gtcgcaacgc ggcgcagggt gagagcgcgc gcttgcggac gcggcggcat 4860
taaacggttg caggcgtagc agagtggtcg ttgtctttct aggtctcagc cggtcgtcgc 4920
gacgttcgcc cgctcgctct gaggctcctg aagccgaaac cagctagact ttcctccttc 4980
ccgcctgcct gtagcggcgt tgttgccact ccgccaccat gttcgaggcg cgcctggtcc 5040
agggctccat cctcaagaaa ggtgttggag gcactcaagg acctcatcaa cgaggcctgc 5100
tgggatatta gctccagcgg tgtaaacctg cagagcatgg actcgtccca cgtctctttg 5160
gtgcagctca ccctgcggtc tgagggcttc gacacctacc gctgcgaccg caacctggcc 5220
atgggcgtga acctcaccag gtgagcctcg cggccccggg aagccggccc cggcccgcct 5280
gcacctccgg tgcttggcgg gagcgctttc gagcctagcc ctcattggct ggcgtgggcc 5340
atccgcgcct tctcattggc ctgccacgca gtggggtggg gcccagctga gcgcgcggtt 5400
cggaaaagcc cgcgctggct gctgcgcgaa cctgcttttt cgcgccaaag tcacaaagcg 5460
ggtggtggcg ggaaaatcaa gggtttttcc gcagtgccag gaacactgtt ccaggctctt 5520
ttgctcacta aatcctgttg gccttgaatg gacgctttag ctgtggcttt cttgtttctg 5580

agacggtctc ggtgtgttgc ccgggctggt ctccaacttc tgggctcaag cgatcctccc 5640
ggctcagtcg cggttgactt taaatgcttt ataatgccct tgcgagaaat gtggcagcct 5700
gtcatcctac ttagtggcag gagattgttt ctatccagaa gggacactgc tggtggtatt 5760
ttagtataaa tactgccaga tgcgtcccaa aacgtctgca ttaataatgg catcctccag 5820
cagtccgctt accctccacc agttctgaga cggcctgatg ggtgagagtg gtaacccctt 5880
ctaaccgcgt tcgaaataca gcccttcagc agacggcgtt gattttaaag catgtgtctc 5940
ctgtcttcta gt 5952

<210> 59
<211> 6664
<212> DNA
<213> Homo Sapiens

<400> 59
   tattataagg gcattaatcc cattaatgag ggaaaagccc tcgaggttta ataatcacct 60
   cccgaaggcc ctacctctta ataccatcac cttggggttt aagttccaac atatgcattt 120
   aggaggggca cataccttca aattatagca atccgcaagt gatatacact tcaggcccta 180
   tggaacctac acttaccctt ggccatatcc agccacgtgg atgtcccaca gtacttcact 240
   atcaatatgc ccaaagtgat gctccatgcc ccatagctgc cccttagcat ctctctagaa 300
   cagcaggacc ctcagcctgt tgcaaagcca cacacccaag tcatccctga tgactccttc 360
   tccctttttt aacccagtcc cagctcctaa aaatatctca ttgtcatttc ttcctcttga 420
   aactgacatc tccccaagtt cagcacctgc tgggactgtt gcagagccct ccattgtctg 480
   ctggctctga tgctgcatca ccagccctct tcatgacttt tcccccctaa ctcccactta 540
   tgaccatgtc actgccttgt tcacattttt ttgttcaagt ccaacgtcta atccaaaact 600
   tttagcatga ccttgaacat taccttcaag gtatagatat tatttaacta cctagcctaa 660
   tgtttatgac ccttgctgag cacccagatt ctgtttatgt tcagcgtact gaattaattt 720
   cagtctacaa acttgcattg ttctttcttg tcaatggatc aagctctcca ccttcatttg 780
   tcttcttggc aaactctttt tattccttaa gactcagttc agtcaacttt gctatgaagc 840
   cttgcctgat gccctctcca ctatgttctg cacacagcag tcttcaccat attgcattgt 900
   aaacatttgc accccaccta ggtggaagat ccacagctac tcacctgtta tccttgatcc 960
   tcattagtcc caaggttgac atatgaatct cttctcccta caccatgccc agtttctaaa 1020
   attccaatga gagattaagg tgggttgaca tgtatgcatt taaagtacaa ctaccaacag 1080
   gtttaaagtg atctctttct ctttttgtgg ggcgagaatg aagatagggg gcatggaggt 1140
   tgtatatgac aaacttttat tatctgtatt ttacactttg gattatttct taaaatttac 1200
   agaaaaatca cattactttt ataatcagaa aaacacccaa ctctgtcata gcaacgatag 1260
   ttcagtattt tcacttgtgg tgaaaagacc tgaatctata tatcttgtat tttctgtcac 1320
   tttcctttct gaattaacat gaaaaaggat aaatcttctt cttttgcata cattttccgg 1380
   caacttttaa attttttttt tctttttata tatttgtctg tatagagaca gggtcttact 1440
   atgttgccaa gctggcctca aactcctggc ctcaagggat cctccacctt ggcctcccaa 1500
   agtgctggga ttacaggtgt gagccactgt gcctggccaa tttttaccat ttttatccag 1560
   tagcaatgga ggccttggca agactgattc attcattcat ttatttattc gttcattttt 1620
   gggctagggt agtacattca cgtggctcaa tattcaaaag atacaaaact ctcctttcca 1680
   cctctgtacc aggtcactct tgtattccac agagggcaca tttttattag tttcttccac 1740
   ctccttcccg aagtgtattt tcttttcttt tctttttttt ttagaaggag tctcgctcta 1800
   tcactaggct ggagtgcagt ggcgccatct cggctcactg caacattcgc ctccagggtt 1860
   caagcgattc tcctgactca gcctcccgag tagctgggat tacaggcatg tgccaccacg 1920
   cccagctaat ttttgtattt ttagtagaga tgggttttca ccacgttggc caggatggtc 1980
   tcgatctcct gacaacgtga tccacccgcc ttggcctccc agagtgctga gattataggc 2040
   gtgagacacc gcgcccggcc cggaggtgta ttttcaagca aatgttctca catttttctt 2100
   atctaaaaaa aacacttgtc ttgacctggg ctgccataac aaaatgccat agactacgtg 2160
   ccttaaacaa caaaatttta ttttcactca gttctggagg cttggtgtgc aagttcagag 2220
   ggcgggcaac atcaggttct ggcaagggct gacttcctca ctgacaggtg ccttctcatt 2280
   ctaacctcac atgagagcaa ccaagctctt cagcgtctct tcttataaga gcactaattc 2340
   catcacgagg gccccaccct catgtcctca tctaaaccta attacctccc aaagcctcat 2400
   ctccaaacac catcccagtg gggtgaggcc ttcaacgtat gaattagcgg ggtgggggga 2460
   caacaaacat tcagaccata gaaatacgta aatataagaa acaaaaccaa aaagacttat 2520
   gtgcaatacc aactgatctt ttcttttcct tccaaaatgg agtttaccaa aatacaaata 2580
   tgaagttttt cagagcacat accttcccct gctctcctct gcagtttttt tttttttaat 2640
   accacctcta gggtagtttt cctaaaatat ggttccaagg ctgtgtgtac ctctcctgct 2700
   cagatccttt ggaacagtgt tcagcgctca tcaggattct gactgtaaac tcccttctct 2760
   gactttaaca tcacaggatg cagtactccc tacattctac catggggggc accctgaatt 2820
   acctgggttg actttctggc ctctgtgctc tacttataca atttcctctg tatggaaggc 2880
   ccactccaga atcctttccc cagcctttgt tcaaatgtca cttctgctag cattagcagt 2940
   ttcctcctct gggttctcac attgccctgt aacataagtt acaaggtaag taccagaaga 3000
   gaattcccaa ttatcatcct gctcccctac cctcaacacc tccagcggta actgtatctt 3060
   attcattttt gtgtctatga agcacagtgt tgcactttgg agatactaca tagaagttgg 3120
   cccaaagggt aataaactgt atttgccaag atgatagcat ggatttacaa taacctctga 3180
   aactttataa agaaagatga ttgagttaaa ttgatacacc tagcatcaca gctgtggaag 3240
   cttgtggtct tctttatgtt aaaggtaaaa tggatataat gaattagttg ccatttttag 3300
   atgatataga agttgaattc ctcttaagcc tctcaaagag aaagacatat taaaattcat 3360
   taaaaccagt accataaact gcacagaaca gcctggttcc ctagagcact tctctggggc 3420
   agtgagtttt catttaaaaa atgaagtttg gattttaaac ataagtttta aaagtgatta 3480
   ggctccagca ggagttctta aattcctacc tacaccatga ctcccaaatg ctctgcctct 3540
   gttgcctagg aacaaaaatt gtcaaggttg agccagatgc aatgtataac accagatgat 3600
   agatgcagct cattgtgtta aaaggtgtta aacacttaag tgcatgtgat atactctata 3660
   tcacaagggt aaggaagttc cattccatat cctgatttgg gtcttctaat acatacttgt 3720
   agtggatgtg gtaatcagtc tttgttctat gtcccattag gatcaatgca accagaaatc 3780
   cttaaaatgg atttttatta tgtctagctc tgttttactg ttctatttgt ttattttaaa 3840
   aagattttag aatggtgttt aggttgggct ggtggctcac gcctgtaatc tcagcacttt 3900
   gggaggccaa ggcaggagga ttgctggagc ctaggagttg gagaccagct agggcaacat 3960
   aaggagacat tgtctccgca aacaaacaga aaaaaaaaaa gtaagaaaga aagagagaaa 4020
   gaaggaaaga acaagaaaaa ggaaaaaaaa attaactgca gtaagtgcag aaatctctct 4080
   caaggatatg atggatggca atgaagacag agtttttgac actggattat ttaaagcata 4140
   atttaagcct ctttctctcc cctcatagaa ttgtgaattt ttgtgtttta attatattta 4200
   agccaggcgt ggtggcgagt gcctgtagtc gcagctgcgc tggaggctga ggccgattgc 4260
   ttgagcccag gatttggagg ccagcatgcg caacataatg agacccagtc tctaaatgca 4320
   tgcctctctc tatatattta aaattctgat gtgaaaatat tttaaaattt aatacatttc 4380
   aaatgttttt aattgtataa taaacaaaat gtaaataata aaataattta atattaaatt 4440
   caaaaatgag gtagaaacaa agcacagcga tataaataat aaattttcct ttacattttt 4500
   gaggcggtct tttgagtttt ccatttcctt cttaaggtca ctgaaatgtg ctccttggag 4560
   ccagcccgca aatcacgcat ttagaaaaac ataactatac actcctaacc ctaagtatta 4620
   gaagtgaaag taatggaatc tcgatgtaaa cacaatatca cttttttgat gagctatttt 4680
   gagtataata aatttgaact gtgccaatgc tgggagaaaa aatttaaaag aagaacggag 4740
   cgaacagtag cttcctgctc cgctgactag aaacagtagg acgacactct cccgactgga 4800
   ggagagcgct tgcgctcgca ctcagttggc gcccgccctc ctgctttttc tctagccgcc 4860
   ctttcctctt tctttcgcgc tctagccacc cgggaaggcc tgcccagcgt agctgggctc 4920
   tgattggctg ctttgaaagt ctacgggcta cccgattggt gaatccgggg ccctttagcg 4980
   cggtgagttt gaaactgctc gcacttggct tcaaagctgg ctcttggaaa ttgagcggag 5040
   agcgacgcgg ttgttgtagc tgccgctgcg gccgccgcgg aataataagc cgggtacagt 5100
   ggctggggtc agggtcgtgt ctaggggacg gccgagggcc tcggagggcg agtattgagg 5160
   aacggggtcc tctaagaagg ccggactgga ggtcagggat ctgcgcgggg cccggctggg 5220
   cctcgggggg cggtgggcag ggccttcgcc tgggtctggc cgtgtgagcc acactgggct 5280
   ggctttagag ggaggctatg ggagcccagc ctggcggggt taggcggcgt gggggtgggg 5340
   gttcggggct gcagtcatga gtcgaggtcg gctgttctca ggaactgcct gaagaggctt 5400
   tcgggtcttg attgaagcaa aatccgctcc cccacgctga gcgcctgcgg agtagcaggc 5460
   tctttcctgg gcccggcccc tgcgcgccct gccatcccgc ctcgggggtg ggaaagtggg 5520
   accgcacctg ggccaggtac aggcgcgggt ccctagagcc aggccgccct gagaaaccga 5580
   tcctggaact cgtgggcctt cttgggcttg ctctgcccac ggccccggcc cacctggagc 5640
   tgttaagaac agtcagccca ggcacttaac tctcatgagc agtgtagtgc agtttttttc 5700
   atttaaaaag atacatctct gcttctttct ggattgattt ttctttgaag atgaatgtga 5760
   gaaatagaac ttaaaggtct attctgagtg tgctttataa atgattttat catcagtgat 5820
   gggtgttaga aaaatctagg taattatgcc tggagttctg acgtttcatt ggcaaacttc 5880
   agaggaagtc ttgaacttct ggttgtcagc tttttttttt aacccattca ttgtgattca 5940
   agtataccgt ttttctgctt agtccttgac attgtttagg ttataattag aattttatct 6000
   gtggaattgc acttttcatt cttttgtttt acggtgctta agtgatattt cctactcttg 6060
   gaaaaagtac cttgaaagtc ttgagcatgt ttgtctgtga cctcagcaag cagaggaaaa 6120
   agattaaaat tgtcacgtgg gcaactgtaa acacttaaaa tgtttttgtc gttttaaaca 6180
   aaattttcct aatgaacagt ctgaacacat tttagttctg tcctcataac tttaaaaaat 6240
   aatgttttga ataaaattag gagtatcata aagcggtata aatttgttga aatatttggg 6300
   tataactttg gcacacgcag gtactacttt aaagtgtcac aaaaagggaa cataattctg 6360
   gtttttgttt ggaaagaata gacaagttga tacctgaatt tgagggttat tttgattgga 6420
   agaagctaaa tactacacta gtatagaata tacacgtttc caatgtctca ggagtcaaga 6480
   aggatggaaa gttattgtag gagtccttac ccttgtaaat attgtttgaa tgtaattaat 6540
   atttatggaa tacttatgct gcagtaggca ctgtggaaaa tgctttacat atagtgtttc 6600
   attaatgctt aaaactactc gagttagtgg catgtaatta agtcagtttt tccttcactt 6660
   tagg                                                              6664

<210> 60
<211> 6815
<212> DNA
<213> Homo Sapiens

<400> 60

ctgcttgcga actaaaaatt cagagctctc ttgtgaatca tgctgccaag aattgcagac      60
ttaagcctca agattgtacg aatttaaatt ttgactttat ggcctcagtt ggcatgaaga     120
tacaaagata atcttctagc aaaaacagtt ttgaaagacc gggcgtgatg gctcatgcct     180
ataatcccag cactttggga ggttgaggcg ggtggatcac ctgtggtcag gagttcagga     240
ccagcctggc caacatggtg aaatcctgtc tcaactaaac atacaaaaaa attagctggg     300
tgtggtggcg gacacctgta atcccagcta cttagggagg ctgaggcagg agaatcgctt     360
gaacccagga ggcagaggtt gcagtgagcc gagatcacac cagtgtactc cagcctgggt     420
gacaaaagtg aaactctgtc tcaaaaaaaa aaaaaaatgg tagaatgtca catggaaagt     480
taacttttag agatagtaat ccagacacct ataatattac attgtccttt taagtatcta     540
tatcaataaa ttaaccaaca tttgttgact taacatggcc agggacagtg ctgggttctc     600
ataattccca agtaattcta gccagacctt gtcctcaagg agcttatgag cagcatgagg     660
agataaaaaa cagataaata actctaattc aggaagaatt tgataaatgc atgataacta     720
ctactagaat tcagaggaga ggaaattttc tttgaataga gactaaagga aggaataatg     780
aatgtggtag catttgggct ggacctatta caagaggcat gctttcccct gacaagccaa     840
gaaagtgggg atttaaggta gaagaaaaga aaagccttga gttcttggag tattaaaatt     900
ttgcttggct gaagcatagg ttacagtcgg aggagatgga ctaggaagga tcgttatggg     960
cagagaggaa gccctgaact catgggggaa gctatgattt gggttaaaga cagagctggg    1020
tcatgtcaag atggatcctg aagcagtaaa aaaaaatcca agaaagtaaa caggttgcag    1080
ggtttaggat gaagtccggg aggaaagggc agagtggtcc ttaggaggcc gttaggacag    1140
gtaaggtaat gggtctcaaa gggagtggcc gaaatgcaat ggaaaaagag agattgtaaa    1200
gctagaaggc ttaggaattg cctcttgatt aggtgtggaa ggcaagggaa aatcagccct    1260
cgaagaagac agtgagattt taatctgggt ggctggagag acagtgatgc tgggcacaga    1320
cacggggaag ttgagaggaa caccatgttt gagaatggtg actcatattt gaacaagcct    1380
gcaatgccca gcagaccgct ggaaaagtgg ggctggagac acattcaacg gaggagccag    1440
atcaatcttt acccttcttc acctgagaga gccagtaagt cacggctgga acgtgtgtgt    1500
ccagcaggag agggtaggga gggaagccaa gagagctggg agcccgagtg aagtttttgc    1560
caaaggcaga agaggaaagt cggcgtagca cagtatactt tcccacccat gctcaccaag    1620
cccagggaca aggctcacca agatgagttt ggaagagaat gctggagaga aagtggttaa    1680
gaaaactgcc tttactgaac ttcttgggct aactttgatt gtaagtctct gaacaatcaa    1740
agcctgtgag gagacagcta accttcttat tcttcctatg tcaatagtga acaattgcag    1800
atcccctttc ctttccttct cctttcccct gttcctctct cctccctccc tgaatactct    1860
tgcttttttc tgggactggt ctagagcatg ggtggccatt gttgacctac aggaggcacc    1920
actgtcacca acaaagggta acagtctttc ttttcaatat ttatttatat ccagtattta    1980
ttttcaatac tgactatgga gagagctctc ctgtgctcaa acactgcaat actgggggtc    2040
tttcaaagca caaaaacata tatttgcatg atggcatcat taacattttt atggctttct    2100
atttcttttt tgtactggtc tcaagagcca ctcataaatc tctcagtaac tgcatagtgt    2160
cccagggcca gagaccggcc actcctggca ttgtgattag agtcatttaa tatccaaggt    2220
ggtgactaat gtctggcaac aaagcctcca ttgggtgtca tgtgtcctgg gaccctgagc    2280
gtgggcactc taggagcacc tcagtattgc gtgttagtac tatggccgag agaatagttg    2340
agaaagtggt caagaggtgg atccatgtga acgccactgg gaaatgagag acctcgttcc    2400
caatcacggt cagtgcaact cgaaagccta aaatcagttt aaaacaaagg tatctacctt    2460
tatcttatgt tcatatccta ggcttttaat aatacgtatt tttcacatgt ttacagaaag    2520
cagtcaactg agctattcat ggaaaggttt gtgggtttgg ttaacgaagt ggaggagtat    2580
tacatttcag ctggaaacac atccctagaa tgccaaaaca tttattccaa agtctggttt    2640
cctggtgcaa tcggaggcat ggcaatgcct ctgttcagag actgggggct agggccagta    2700
aggcatttga tccacatgta tcccagaagg cttttattgt taaattatat tctttcggaa    2760
aaaccaccca tgtcctattt tgtaaacttg atatccatac acttttgact ggcattctat    2820
tttagccgta agactatgat tcacagcaag cctgtttttc ctcttgcttg gggtggcagc     2880
agaaagcata gggtactttc cagcctccaa gggtaggggc aaaggggctg gggtttctcc    2940
tccccagtac agctttctct ggctgtgcca cactgctccc tgtgagcaga cagcaagtct    3000
cccctcactc cccactgcca ttcatccagc gctgtgcagt agcccagctg cgtgtctgcc    3060
gggaggggct gccaagtgcc ctgcctactg gctgcttccc gaatccctgc cattccacgc    3120
acaaacacat ccacacactc tctctgccta gttcacacac tgagccactc gcacatgcga    3180
gcacattcct tccttccttc tcactctctc ggcccttgac ttctacaagc ccatggaaca    3240

tttctggaaa gacgttcttg atccagcagg gtaggcttgt tttgatttct ctctctgtag 3300
ctttagcatt ttgagaaagc aacttacctt tctggctagt gtctgtatcc tagcagggag 3360
atgaggattg ctgttctcca tgggggtatg tgtgtgtctc ctttttcttt caggacttgt 3420
aggattcttt gtgccatttg catataattt ggcaggttca cattttttaa gagccctatg 3480
aagtgctttt tgcatgtgtt ttaaaaaggc atttgaaaat tgaaagtgtg atttatggaa 3540
attaaatcat ctgtaaaaaa ttgctttgga aagtaatgat tgctggccat aaagggaaat 3600
atctgcgatg cacctaatgt gtttttaacc ctttatttgc tgacaatcta tagtcattaa 3660
tgctaaactc gattttggct tcagctacat ttgcatattg tccaacaatg gtctattttt 3720
gtaagaatta gataaaatgt atacttgata taaaatagtc aaaaatgtaa ctcttagtaa 3780
cagtaagctt ggcatttaga tagaccatga acacttcgtc agatactctg ttgggtgttt 3840
gggatagcaa ttaaaacaaa gtattgatag ttgtatcaga gtctattagg ctgcagcaaa 3900
ggaagtttat tcaaaagtat aaactatcca agattataga cgcatgatat acttcaccta 3960
ttttttgtct ccttaatatg tatatatata tatatatata tatatataca catatatgtg 4020
tgtgtgtatg tgcgtgtgca tgtttaactt ttaattcagt taaaaacttt tttctatttg 4080
tttttcatct ggatatttga ttctgcatat cctagcccaa gtgaaccgag aagatcgagt 4140
tgtaggacta aaggatagac atgcagaaat gcattttaaa aatctgttag ctggaccaga 4200
ccgacaatgt aacataattg ccaaagcttt ggttcgtgac ctgaggttat gtttggtatg 4260
aaaaggtcac attttatatt cagttttctg aagttttggt tgcataacca acctgtggaa 4320
ggcatgaaca cccatgtgcg ccctaaccaa aggtttttct gaatcatcct tcacatgaga 4380
attcctaatg ggaccaagta cagtactgtg gtccaacata aacacacaag tcaggctgag 4440
agaatctcag aaggttgtgg aagggtctat ctactttggg agcattttgc agaggaagaa 4500
actgaggtcc tggcaggttg cattctcctg atggcaaaat gcagctcttc ctatatgtat 4560
accctgaatc tccgccccct tcccctcaga tgccccctgt cagttccccc agctgctaaa 4620
tatagctgtc tgtggctggc tgcgtatgca accgcacacc ccattctatc tgccctatct 4680
cggttacagt gtagtcctcc ccagggtcat cctatgtaca cactacgtat ttctagccaa 4740
cgaggagggg gaatcaaaca gaaagagaga caaacagaga tatatcggag tctggcacgg 4800
ggcacataag gcagcacatt agagaaagcc ggcccctgga tccgtctttc gcgtttattt 4860
taagcccagt cttccctggg ccacctttag cagatcctcg tgcgcccccg ccccctggcc 4920
gtgaaactca gcctctatcc agcagcgacg acaagtaaag taaagttcag ggaagctgct 4980
ctttgggatc gctccaaatc gagttgtgcc tggagtgatg tttaagccaa tgtcagggca 5040
aggcaacagt ccctggccgt cctccagcac ctttgtaatg catatgagct cgggagacca 5100
gtacttaaag ttggaggccc gggagcccag gagctggcgg agggcgttcg tcctgggact 5160
gcacttgctc ccgtcgggtc gcccggcttc accggacccg caggctcccg gggcagggcc 5220
ggggccagag ctcgcgtgtc ggcgggacat gcgctgcgtc gcctctaacc tcgggctgtg 5280
ctctttttcc aggtggcccg ccggtttctg agccttctgc cctgcgggga cacggtctgc 5340
accctgcccg cggccacgga ccatgaccat gaccctccac accaaagcat ctgggatggc 5400
cctactgcat cagatccaag ggaacgagct ggagcccctg aaccgtccgc agctcaagat 5460
ccccctggag cggcccctgg gcgaggtgta cctggacagc agcaagcccg ccgtgtacaa 5520
ctaccccgag ggcgccgcct acgagttcaa cgccgcggcc gccgccaacg cgcaggtcta 5580
cggtcagacc ggcctcccct acggccccgg gtctgaggct gcggcgttcg gctccaacgg 5640
cctggggggt ttccccccac tcaacagcgt gtctccgagc ccgctgatgc tactgcaccc 5700
gccgccgcag ctgtcgcctt tcctgcagcc ccacggccag caggtgccct actacctgga 5760
gaacgagccc agcggctaca cggtgcgcga ggccggcccg ccggcattct acaggtaccc 5820
gcgcccgcgc cgcccgtcgg ggtggccgcc gcgcccggca ggagggaggg agggagggag 5880
ggagaaggga gagcctaggg agctgcggga gccgcgggac gcgcgacccg agggtgcgcg 5940
cagggagccc ggggcgcgcg gcccagcccg ggggttctgc gtgcagcccg cgctgcgttc 6000
agagtcaagt tctctcgccg ggcagctgaa aaaaacgtac tctccaccca cttaccgtcc 6060
gtgcgagagg cagacccgaa agcccgggct tcctaacaaa acacacgttg gaaaaccaga 6120
caaagcagca gttatttgtg ggggaaaaca cctccaggca aataaacacg gggcgctttg 6180
agtcacttgg gaaggtctcg ctcttggcat ttaaagttgg gggtgtttgg agttagcaga 6240
gctcagcaga gttttattta tccttttaat gtttttgttt aatgtgctcc ccaaatttcc 6300
tttcatctag actatttgat tggaaatatg tcagctatga tgatgacttt ctgggaagcg 6360
attcctgtca cccgctttcc cctcctcccc accccacgtc ctggggcttt agagagcgat 6420
tgggagttga atgggtctga tttcggagtt agctggctga gtccgcgctg gagcggattg 6480
ctggcatgtg acttctgaca gccggaaatt tgtaggtgtc ccgcgagttt aaaacaagcc 6540
atatggaagc acaagtgctt aaaaataatc tcctgccagc ccagtgacaa gcctgtccca 6600
cccggggaga atgccccgga gtggcgtgcg ggtcagccag ggtctgcgcc tcgcagccac 6660
tgtggaagga gcgcggccgg tccaggacac aggagaccac tttgtgactt caatggcgaa 6720
ggttgtgtgt cctcatttta atttttttcc ctacaagaat tgttctttct ccctctcctc 6780
tccctcccat tttctcttgc ccagtttctc ctttt 6815

<210> 61
<211> 5527

<212> DNA
<213> Homo Sapiens

<400> 61

ggcttcttgc tgctgccaaa ttaacaactc cagcccttgg gtttagccac ctgctgctgc 60
taaaaatgtt gctgagagtg atggagaggt gttttagttc ccattttaca gatcagaaaa 120
tcaaggccca gagaaactaa gtagtcttgg agataggagc catggcctct ccacttgagt 180
tatcatcttg aaacatcaag gacaagccaa ctttatcaac agttttaggc ctgtgagaaa 240
tgggcttggt ttatcttgaa gattgtaaac aaataaaaaa atagactgct ccattactga 300
tgagaaatat gagtcattct gccaggaatt gaaagtacag ctgcccataa aaaagctatg 360
ccggggtcca gcctctccag gtccttgtgt gtgaaaaacc actcagtcaa agttcccctt 420
ttatttctct ttgtgctcag taggatgctg aaatttctgg aaaagacaat ggaaatcagg 480
ggcaggaaga gaacagtgtg gggtgctaaa cagatctcag caacctccct gcccacggcc 540
atctctgcgc agacacctcg accccccatg cgcaggtgga gcagcgtttc ctagttcttt 600
ccagaggctt ccttctgcct gccttccagc cacatcgcct gagattgaca acgccctaca 660
gcaagacgga aacctccctt tacagcacca ccttgcgatt ctgcagccac aaagttgaga 720
cttctgaacg tggcactctt ctgttccctt actgtttcac gtgtacctgt gtcatctttc 780
ttgtttcatc gtaaacatac ttctaaaatt cccattttct ttatttagaa atagaactac 840
aagcggatgg ttaaacaatt taaacaaatg gtccatgggg aaaagtgaat ttcacactgt 900
cccccaaact ttcagtgcct tttatggaga taattattgt ttctaatgtt ttgaatattc 960
tttcagagat attttattct tatataagca tatgtgtatt tgtgtccttc cctcattttc 1020
cccccacaaa cactagcaga ctgttttgca ccttggttag ctcatttaac atatcttgga 1080
gccaggtgcg gtggctcatg cctgtaatcc cagcactttg ggaggccgag gtgggcggat 1140
cacctgaggt cgggagttcg agaccagcct gaccaacgtg gagaaacccc gtctctacta 1200
aaaatacaaa attagccagg cgtggtggca catgcctgta atctcagcta ctagggaggc 1260
tgaggcagga gaatcgcttg aacctgggag gcggaggttg cggtgagccg agatcgtgcc 1320
attgcactcc agcctgggca acagaacata tagatctgct tgatcctttt catgactgct 1380
acagctccat tgaatgtatg gatcatcagt gctccagcta gcaccttagt gatgggcatt 1440
tagggatcac aaacttcata agggcagaaa caatatctta cccatcctct gtttctgata 1500
tggtttggat gtgtgtccct gcccaaatca cacgtcaagg taatccccca tgtcagaggt 1560
ggggcctggt ggaggtgatt ggatcatggg gatggatcct tcatgaatgg gttagcacca 1620
tccctttggt gctgttcttg tgatagagct ctcatgagat ctggttgttt aaatgtgtgt 1680
ggcacctccc cactctcttg gtcaagttcc tgccatgtaa gatgcttgcc cttgctttgc 1740
cttctgccat gaataaaagc tctctgaggc ttccccagaa gcagatgcca ctatgcttcc 1800
tgtacagcct gtggaactgt gagccaatta agcctctttt ccttatgaat tatccagtct 1860
taggtatttc tttgtaacag tgtgaggatg agctaataca gtttcctaca ctgtaaccta 1920
aggcaatgct ttgcacaaag ggatgaacca gattgcttag taattaaaac acaaatacaa 1980
accacaagca tatccattca tgaattgggg gacagctttt atttgattct gagttctgat 2040
acctgctgct ttgctttgtt aaaaatgtgc tagttctcat tcagcttcct atcagtcata 2100
tcaccaacca acatgtataa catgtacaat ctgccaggtc aggaggaaac ttatggatac 2160
aagaaatgtc cctcccttct aggaaatgat aatctgcttt gggagagaag gcaggtactt 2220
tttggcaaaa gtgacataat aaaataatca aaaaaagatg gatctttttt tcttttttga 2280
gatggagtca cgctctgtca cccaggctgg agtgcagtgg cgcgatctcg gctcactgca 2340
acatccgcct cccaggttca agcgattctc ctgtctcagg ctcccgagta gctgggacta 2400
caggcaccca ccaccacgcc cggctaatat tttgtatttt tagtagagac ggggtttcac 2460
tgtgttagcc aggatggtct cgatctcctg accttgtgat ccgcctgctt cggcctccca 2520
aagtgctggg attacaaaca tgagccactg caccaggcga aaagatggat tttttaaaaa 2580
aggtgttagg ataatcaggt ccccacctgc aagaaagtaa agttagaagc ccacctcaga 2640
ccatgcatac cagcacaaaa atgtcaaatg gcttaaagat aaaatgttaa aacaagtcta 2700
gaaaggtatt aaaagaaaat atagaaaata cagcaccagc aaaaatttcc ctacacaaga 2760
tgtaaaagcc agaagtcata aaggaaaagg ttgataaatt agaatagata aaaatgtaaa 2820
acttttgtat aatataagac acaaagaatg aatttaaaga catacaatga aggttaggaa 2880
gatgaagatg ttttggcgat gagtggtggt tacacatcag tattaatgta cttaatgtta 2940
ctgaactgta cacttaaaat gatgaaaatg gtaattttta tgttatgtgt atttcatcac 3000
ataaaaaaag acatgaaatg aatcggggaa aatatttgct acataactaa gaatgaaggc 3060
ccttaataaa atctgtaaaa ctatacacac ttttaggaat gaatcaacaa ataatttcta 3120
tgaattagaa aaaagtgaca atccaactaa aaaatgaata agggatataa gcaatgtgtt 3180
tcacagaaaa aataaaaatt gacaatgaag ttatgaaaaa atgttcagtc tccttagtaa 3240
ttgcacaaaa caaactaaaa caatgagaca ttacccctaa gattagtaaa tgttaaagaa 3300
aaataataat tggtgagggt gtggggaagt gggcacttac acctatgttt ggaaatataa 3360
attggtgcaa ccttataggg agagcaatct cacaacattt tccaaagact tacatgcaca 3420
accctatggc agagaaattt attcctcttc caggattttt tttccttcaa aaacagtgat 3480

gtggatgaaa aacacatgtt cactactgca cagggtataa cagctgaaaa ctggaaacga 3540
taatactcac attcccttca gtaggggaat ggttaaataa attttacaag ccatctggta 3600
gataccaggc atgagctaaa agttagggtc cagttagaga tggaaagcac accagtaatt 3660
tgaaagggaa aatgtaatat gaagaattat taactagtaa aagaaggcta actgctaaag 3720
gtacaagagc actcaagctg tctgcagtca gcaggccccg gctggtgagc aggaagctgc 3780
ccgctgggag gctgccaaag ttccctgaag gtgagcacca ctggttctac aagctgctgg 3840
cagtcatggc gttaagagca ggaagagaag caccagaacc cggaagagaa atccagtcct 3900
ctgctaggcc ttgcaccgtc cctctggcgc cctctactga caaagccagt aaaattgtgc 3960
cgctagcaaa ggagatcttt ttatgggatg tagcttggtg tcaccaaaga gaacagagtg 4020
gacttggagc tcagatgcaa cacaatgatt gatactggca cagtatactt accctgcttt 4080
tgtaaacaaa atggtatatg tgatgtctct ctttgtctct ctgtatataa aacaatattt 4140
gtttctactt attatgtatt tatgtcttta ctctgcatgc caggagctaa gtattttgca 4200
tgtattaact cattttgttc tcataataac cttcacatgc aggaatcatt atagctactt 4260
tatgaatgag ccgaggaagg cactgagacg ttaagtaact tgcccaaggt cacgcagcta 4320
gtaagtggca gagcaagaat tactatggct ttataagcct aggaaaaagt ctgaaagaat 4380
caaaatgtta acagcgggga cctcaaggaa gcattgaaga ggccatggga gaagttttca 4440
ctttgttaaa aaatcagtcc ttcaaataaa taaatacagt gaggcttccc cagaagcaga 4500
tgtcactatg cttcctgtac agcctgtgga actgtgagcc agttaaacct cttttcttta 4560
taaattatcc agtcttaggt atttctttat aacagtgcta ggatgagctg atacagtttc 4620
ctacactgta acctaaggca atgctttgca caaagggatg agccagattg cttagtaatt 4680
aaaacgcaaa tacaaaccac aagcatatcc attcatgaat tggggggctg ctttgtgtgc 4740
atagataagg tatatttttt aaaaaaatta tttttccaag aagaaaataa accagttaat 4800
aaacgacaac tcacagtgcc aggaagtgag aaacaagtgt gtgataaacg gtggagaatg 4860
ggagcactct ccgcagtggg cgggaggaga cgaggagggc gttccctggg gagtggcagt 4920
ggttggagca aaggtttgga ggaggtaagt catgtgctct gagtttttgg tttctgtttc 4980
accttgtgtc tgagctggtc tgaaggctgg ttgttcagac tgagcttcct gcctgcctgt 5040
accccgccaa cagcttcaga agaaggtgac tggtggctgc ctgaggaata ccagtgggca 5100
agagaattag catttctgga gcatctgctg tctgtgagat taagcactat gtatattgct 5160
ttattcactc cccacagcaa ccttaccaag cagttctttt ccacgtgaaa agatggaggc 5220
tgggtggagc aaaaggaggt atttagagtc ctcagcaagt gagaggcaga gctgggattt 5280
gaatccagat ctgcctgata ctgaagtcta ggctggttcc acctctccgg actgctttcc 5340
agggagtaga agacagatat tttaccttag ctggctgctt ctagaagtct gaccctgctg 5400
gctcaaaacg actttagttc cttgcccaga ggctgcgggc tgcgggtcaa gacatcagta 5460
gaaggagggc ccagccagag aggctgacat gggcttctac tcaccgtcat ctctccttcc 5520
ttccagg 5527

<210> 62
<211> 10224
<212> DNA
<213> Homo Sapiens

<400> 62
   agcccccaca gccccagtac cgccctcccc atcctacccc gactgcgtcc ctgctgttta 60
   tctttgccca cccacctcaa ccccagtgct cttttcagtc cttgggcctc aggtgacaca 120
   ccagctagtg ggacatgggc ccccacaggc attctcagcc caacccagcc ccttcctttt 180
   ccttggcccc ctggccagca cctgcatcac actggcctcc actggacacc cttgcagctt 240
   cgggccttcc tcacagacac actgctggac cagctgccca acctggccca cttgcagagt 300
   ttcctggccc atctgaccct aactgaaacc cagcctccta agaaggacct ggtgttggaa 360
   caggtaggca ctggaaagtt agctgctcag gaccactgtc ccactttacc agcaccttcc 420
   tgccactctc cacttctctc tcctagatcc cagaaatctg ggagcggctg gagcgagaaa 480
   acagaggcaa gtggcaggca attgccaagc accagctcca gcatgtgttc agcccctcag 540
   agcaggacct gcggctgcag gcgcgaaggt aaggcctgtg gaaatggcag ggagggtgga 600
   ggggatgcag gaggcatgga tgtgggtggg gtgcccccac cttccagggc cagtcagacc 660
   ttcctgactt tcccccaggt gggctgagac ctacaggctg gatgtgctag aggcagtggc 720
   tccagagcgg ccccgctgtg cttactgcag tgcagaggct tctaagcgct gctcacgatg 780
   ccagaatgag tggtattgct gcaggtgagg gtatcctaga accttggacc tctaagccct 840
   actcccacat cccccacatg cattgccatc ctcaataccc acctgcctgc agggagtgcc 900
   aagtcaagca ctgggaaaag catggaaaga cttgtgtcct ggcagcccag ggtgacagag 960
   ccaaatgagg gctgcagttg ctgagggccg accacccatg ccaagggaat ccacccagaa 1020
   tgcacccctg aacctcaaga tcacggtcca gcctctgccg gagccccagt ctccgcagtg 1080
   gagagcagag cgggcggtaa agctgctgac cgatctccct cctcctcacc ccaagtgaag 1140
   gctcgagact tcctgcccca cccagtgggt aggccaagtg tgttgcttca gcaaaccgga 1200
   ccaggagggc cagggccgga tgtggggacc ctcttcctct agcacagtaa agctggcctc 1260
   cagaaacacg ggtatctccg cgtggtgctt tgcggtcgcc gtcgttgtgg ccgtccgggg 1320
   tggggtgtga ggaggggacg aaggagggaa ggaagggcaa ggcggggggg gctctgcgag 1380
   agcgcgccca gccccgcctt cgggccccac agtccctgca cccaggtttc cattgcgcgg 1440
   ctctcctcag ctccttcccg ccgcccagtc tggatcctgg gggaggcgct gaagtcgggg 1500
   cccgccctgt ggccccgccc ggcccgcgct tgctagcgcc caaagccagc gaagcacggg 1560
   cccaaccggg ccatgtcggg ggagcctgag ctcattgagc tgcgggagct ggcacccgct 1620
   gggcgcgctg ggaagggccg cacccggctg gagcgtgcca acgcgctgcg catcgcgcgg 1680
   ggcaccgcgt gcaaccccac acggcagctg gtccctggcc gtggccaccg cttccagccc 1740
   gcggggcccg ccacgcacac gtggtgcgac ctctgtggcg acttcatctg gggcgtcgtg 1800
   cgcaaaggcc tgcagtgcgc gcgtgagtag tggccccgcg cgcctacgag agcggaaggg 1860
   gcagccaagg ggcagcgcag tcgccgcggg tcaagtcgcg gcagaggggg tcggcgggga 1920
   cagctcccga ggactaggtc cgttactttc gccccatcgc tgaagagtgc gcgaaaatgg 1980
   tttatccctt gtcgcactcc actcgtatct gggccacaga tgagcagagg tggctgctta 2040
   tatgtaaaaa tacgctgatt ttaagtttct tatctttaaa atgccttggc ccttcttgag 2100
   aaagggtttg tgcctactgt cctcggagtc catcttccca ggcttgcctc ttctcaaaca 2160
   ctcatgaccc cctccagaac ctttagggtg aaggaaaatt accacctatg ggagggagcc 2220
   tggaaaaatt tagaaccttt ggtgggcccc ctgcaagcag gagttttgtt gagtctttat 2280
   ttagcaaaca cccttttctg acccagtgaa tcagatgcta aaatatgcac gcagccacac 2340
   acccagcagt ccttctgcac ccctgggaat cgccagcaag caaaggttgc tctcccctgg 2400
   gtagacacca gctggaatca ccaggggtgc ttttacagtc ctccccgcta gcctggatcc 2460
   caccgcagac ctgttgaatc aactgctggg agtggaccct aggcatcagt aaattttaaa 2520
   aactccccaa attattgtaa catggagtct gggttgagca tcactgctct ggcctattta 2580
   ggaacttgtg gatggatagt gtcccaggtc tgtgtgtgca tggagaccct ctcatccggt 2640
   acaagaggac atcacaaatt cagctggggg gagcacaaag ttgtgacaga atgcaaagaa 2700
   tgaacaaggg gccgagcgcg gtggctcatg cctgtaatcc cagcacttcg gaaggcggag 2760
   gcgggtggat cacctgaggt caggagttca agaccagcct ggccaacatg gtgaaacctc 2820
   atgtctacta aaaaataaaa aaaaatgagc caggcgtagt ggcgggtgcc tgtaatccca 2880
   gctactcggg aggctgaggt gggagaattg cttgaacaca ggaggcggag gttgcagtga 2940
   gccgagatcg tgccactgcc ctccagcctt ggcgacagag tgagactctg tctcaaaaaa 3000
   aaaaaaaaaa aaaaaaagaa caaggctggg acattgcagc gttctcaaag agaaataaag 3060
   tagccatgga gataagaagc aggatgattt gggcatgttt atcagaggta gagacaaggg 3120
   agaaatcaaa gataagtttg ggcttttgtc tccagtaact gggagcctag tggccatttt 3180
   tgctgcaaag aggaagctgg gcaagtgtag cagtgaggct gaagaaaagg gaattaaatt 3240
   ttggccatgt tcacttgaaa cgtcttttag acatcctagt gaaggtactg gcacggagga 3300
   tctagtctga gggtttaggt cagtgtttca gccgtggatc tggggcagat gaatgtagac 3360
   agaccaggcc agtgatcagg actgagccca gacttcatcg tgagatatgg aagttgagtc 3420
   agaatctgca aaggagctga gcaggagctg cagggggtag gaggaaaact gggagagtgt 3480
   agcccctggg agtcaaaggg agcaagcttc aaatgatgct gagggggtga gaatggagaa 3540
   tggaacactg gattccattt ggtagtacac agatcgctga ggaccctgtc ccgggcagtt 3600
   tcctggagga agaggcaagc ctggctggag tgggtagagg ggagagtgaa ggcgaaggat 3660
   tagagtgtat agagaccagt gtcttggtct gaggggagta gagacaggtg acaaccacag 3720
   ggcagacgta ggttaaaggt gtttagtttt tccttcaagt aaatgggcag atgtattcca 3780
   tatacgttcc cagtgaaggg ccgggtgcgg tggctcaagc ctgtagtccc agcactttgg 3840
   aaggccgagg cgggtggatc acctgagatc aggagtttga gaccagcctg gctaacatgg 3900
   tgaaaccccg tctctactaa aaatacaaaa attagctggg catggtggcg ggcgcctgta 3960
   atcctaggta ctcaggaggc tgaggcagaa gaatcgcttg aacccaggag gcggaggttg 4020
   cggtgagccg aaatcgcgcc attgcactcc agcctgggtg acaaaagcaa gacgcagttt 4080
   tttgttgttg tttttttaat tgccaatgag gaaaggggaa gttctgtgct aggcgataga 4140
   gatccaactg ttgagcaggc ctctctgcct gtggccttcc ggccggtttc cagacgccca 4200
   ggtggccaac attagagtcc gcgtagcagt gtgaggtaac ccactgagat aggtcgggcc 4260
   tgcggagcct ggcgagcagc ggccctctcc ctggggcttc ccttcaatct ccgggacatt 4320
   tccccgacct ggagctcctc cgcctcaccg ccaggcctct ctgcagattg caagttcacc 4380
   tgccactacc gctgccgcgc gctcgtctgc ctggactgtt gcgggccccg ggacctgggc 4440
   tgggaacccg cggtggagcg ggacacgaac gtggtgagcg cggggccgag ggcgtatggg 4500
   aagggcgagg atgggcaggc cacagtgcag gcattctcga gggctgcctg ggtgccgcgc 4560
   gcaaggagcg ttctaattgc cgatttcccg gcggcacaga gaggctaatt ctgcgcgggg 4620
   gctgggaggg gagcctggat tgccggctcc gcaagtactc cacccgctgc aagcggaccc 4680
   gggcccaggc tgacccaggc tccgcgcacg cgcacttccc gcaccttccc gccctcgcct 4740
   ccggccagag gccactcttg tgcgcttgcc cggacgctgg cacccgcccc cgttccctgt 4800
   ggtaggtggg gtctgtgagt ggagctccgg agcgatgagg tcattcctgg gggcgaagcg 4860
   tgcgtgtccc cgccccggcg ttcctgcccc aatgagacaa gagctagatc ccggcgatct 4920
   acgtttcagt cttaacggtt gcggcgcggc tctggcccgg gcgcacgcgc acactgacac 4980
   gcgtacacgc acgcacgcga ccggggcggt ggttggcggc tacggacgcg caggactggg 5040
   ggacgggcgg gtacggctat gggcgaggcg gaggcgcctt ctttcgaaat gacctggagc 5100
   agcacgacga gcagtggcta ctgcagccaa gaggactcgg actcggagct cgagcagtac 5160
   ttcaccgcgc gaacctcgct agctcgcagg ccgcgccggg accaggtggg agccaggggg 5220
   tgccggcggg cgggagggga agcggtcgct ggagctccgc cctccccggt ccgttgccgc 5280
   gtcctgggtc ggtgggcagc cccaccctcc tggctacgtg gctccccgcg ggtcctggcc 5340
   ggggacctgc ccgcggaacc gtgcgtaaga ccccgattcc accgcctaga tgctgggtgc 5400
   cggggccccc ttggtttctg tcacagacag gttgaacacg gaaaaagcag ctgtatggct 5460
   tgtggtagac ctgagccggg cattatccag ctatgactaa agccgaccga gcagtttgga 5520
   ctagcacctc gatttccgcg ttcgaatgct cctgctccct ccttggggag actaggggag 5580
   gatgtggaga gggaagagtc ctcgccagga attgagaagt atgtttagga aaacttgaga 5640
   ggcagagaga gatcctgctc ctccatctgc actcctgtat ggagccagct gagccctcac 5700
   ctcttccctg ttctggcctg tcaccagctg ctggaatgtg gaagattctg ttcccttcct 5760
   ctagggtgga tctggagaaa gatttgggaa tagataggaa agaagtcttg ttttggacca 5820
   taagcattca ggagcacttt acccacagga agggggaaag ctagattata aaatgcctaa 5880
   agaggtggaa aaagagatcc aggttactaa cccaggactg taaggtgtct cggaacctcc 5940
   taggtatccc cattatcgga gaactgtgtg ccagatgcca ttggtgtgac caccaggctc 6000
   agagaaccag gcctaggcac caggaaaaag aaacagggac tgtgaagctc agtatgcctg 6060
   gcagaaatgg ggcggaaatc cttatttaag taaagaaagt ggagttgtga gtgatgcttc 6120
   agataaaatt ttacaaaatt ccttacaaaa tgggtggtgc tcagcacgcc aaaatcttag 6180
   cccagagctt gggtgcaagg gttgagttga gtgtagaccc ctgggcttgt cttcatgtca 6240
   gtcagtcctg agccattttc cactgtggaa aggtgggaaa accacaagac actaaccaat 6300
   tgaaaaggag ggctagccac ggaggtgcac acctgtaatc ccagctactt gggagggtga 6360
   ggcagaagga tcacttgaac ctgggaggca gaggttgcag tgagccaaga tcgtgccact 6420
   gcactccagc ctgagtgaca gagtgagact ctgtctcaaa aatagaaaag gaagccaagt 6480
   acggtggctc acacctctaa tgccaatgct ttgggaggcc aaggcaggtg gatcatttgc 6540
   aatcaggaat tcgaggtcag cctggccaac atggtgaaac cctatctcta ctaaacatac 6600
   aaaaattagc cgggcatggt ggtgtgtgac tgtagtccca gctacttggg agactgaatc 6660
   acttcaaccg ggaggcaaag gttgcagtga gccaagatcg tgccactgca ctccaacctg 6720
   ggtgacaggg tgaggctctg tctcaaaaaa aagaaagaag gctgggcttg gtgactcatg 6780
   cctgtaatct cagcattttg ggaggccaag gcaggcagat cacttgaggc caagagttcg 6840
   agacctgcca ggccaacata gcaaaacccc gtctgtactg aaaatacaaa aaaattatct 6900
   ggccatggtg gtgtgtgcct gtaatcccag ctactgggga ggctgaggca ggagtatcac 6960
   ttgaacccag aagacagagg ttgcagtgag tcgagactgg gccactgcat tccagcctgg 7020
   atgagagagc aagactctgt ctcaaaaaaa aaaaaaaaaa aaaagaaaga ataggaggct 7080
   gagaagtccc aagttatatg ttaaaaaaaa agaaaaaaac atcagtttta ggccaggtgc 7140
   agtggctcac acctttaatc ccagcacttt ggaaagccga ggtgggtgga tcatgaggtc 7200
   aggagttcaa gaccagcctg gccaaaatgg tgaaaccccg tctcgactaa aaatacaaaa 7260
   aattagccag ttgtggtggc aggcacctgt aatcccagct acttgggagg ctgaagcaga 7320
   gaattgcttg aacccaggag gcagagattg caatgagcca agatcgcacc actgcactcc 7380
   agcctggaaa acagagcgag actctgtctc aaaaaaaaaa ccatcagttt ttatggacag 7440
   tggtagagtg gagggtgggt ccctatggtg cagaaggaaa attccatggt cctgctgtgc 7500
   atccgactgg gatggctgtt gaaatcctct tccagcaggc agctttggaa acagaaaaag 7560
   aaactcttcc tcctttagaa tcctggaagg gctgtgcagt gcctctaatc caagtctgtt 7620
   ttctgagtga agatagggag gttcatcacc agaagggaag gggctggaaa tgaggtcact 7680
   gcatcccagc ccagggctcc tgggtcatcc aggaagggaa gaaggagcaa gctttctcat 7740
   tgttaggtag gagctcagag ccatcacaag aacaagttag caccatccct gtgccctccc 7800
   tgttctgcaa acaaaatgat cttccttctt gccctggcac tagagtctgt ctggcatttc 7860
   tcctgcccct agtactcctc ccatctgggt acttcttccc gttggtgtac tgaacaaaca 7920
   catccactgc tttattcaca gcctccagcc ctcattttcc agggcccaca ccatttgttt 7980
   ttactaaccc gacaaggttg cccactgtcc ccagtaaggt ttgtactggg gtttttactc 8040
   cagtgctctt ctccatccag gagacctttg gatacttggg gaagaaaatg agcttaaatt 8100
   cccacccctc cccctttacc tttttcctgt aaggccctgg ccttagttct tagccccaca 8160
   tccttgctgg ctgcagaata gcagcgggtt ctgggtaagg agcattctgc taaaacgctc 8220
   caccctgctc cctcatctgt cctctccatt tgtccccatc agatggttta agtgcttaag 8280
   gggactccag ggcggagtca gggagaaccc tggctctcct gggctaggca caagatcatt 8340
   ctacaggaaa ccttgtggga attcttctgg gacaaagtat tggtcagcgc tgagcttagc 8400
   tgtgtctgtg acactcgcat tctaactagg gcctatctga cgtcaacagg aagtaaggct 8460
   gatgcagtgg ggccaaggga gtctgggaga agaaagtcgg ttcagagccc tggctgccct 8520
   gtcccacact ccacccttcc ggcaagaatc cagtccctag atgaggtggg gagtgagtgg 8580
   tcgagttaaa aatctctggg tcgggtacga tggttcacgc ctgtaatccc agcactttgg 8640
   gaggtgaagg caggcggatc acttgaggtc aggagttcaa gaccaacctg gccaatgtgg 8700
   tgaaatccca tctctactaa aaatacaaaa attagccggg tgttgttgtg gcacgcgcct 8760
   gtagtcccag ctactcggga gtctgaggca ggagaatcgc ttgaacccag gaggcagaac 8820
   ttgcagtgag ccaagatcca gccactgcac tacagcctgg gcgacagagt gaggcttcgt 8880
   ctcaaaaaaa aaaaaaatct ttgggccaaa tctccagaca gcacaggcag gtgcagaaac 8940
   ccaccaggaa gctgcctgtg tacctctggc agattggagc ctggcctaaa gctgcctttt 9000
   atgcagcttg ggtcaaggtt aaacatcatg tcacagtgat ttttctcact atgtgtgaga 9060
   catggagaac tggctccaag tactactctg tccactggtg gctggactac tgatgtgcac 9120
   cactctccac tcctctcacc ctgcagtggg tcatggcccc gtgccggggc agaggagaaa 9180
   aatgggctgc cttctccagg acaaaccctc actccaactc aactagggtg ctgtgatcag 9240
   aatgtgcaat tgaggtgtga ttttactgat tttttttttt tttgagaccg agtttcgctc 9300
   ttgttgccca ggctggagtg cgatggcacg atctcagttc actgcaacct ccacctcccg 9360
   agtttgagca attctcctgc ctcagcctcc taagtagctg ggattacagg catgtgccac 9420
   cacgcctggc taattttgta tttttagtag agacggggtt tctccatgtt ggtcaggctg 9480
   gtctcaaact cctgacctca ggtgatccac ccgcctcggc ctcccaaagt gctagaatta 9540
   caggcgtgag ccaacgtgcc cagcctgttt ttgttttttg tgttttgaag cagggtctca 9600
   ctcagttccc caggctggag tgcagtgaca cgataatagc ttactgtagc tgcaatctcc 9660
   cgggctcaaa cgatcctccc acctcagcct cctgaacagt tgggactaca ggcacaccac 9720
   cacacctggc taattttttt ttttcttttt ttagtagaga tgaggtcttg ctatgttgcc 9780
   caagctggtc tcaaactcct gaggatcaag tgatcctcct accttagcct cccaaaatgc 9840
   tgggattgca gatgtgagcc accacaccca gcctgatttt actttaaatg agagtccctc 9900
   ttcagagtcc ctcagctgtt cctggcccct ggccatgtgc cttcagttgc ccctgcttct 9960
   gtggtatcct taaggctaca ttcagtgctg aggccctagg caggcagcag agagaagcca 10020
   aatgattctg tctttccctt atccacccag agcatgcaaa accaggagca gtggtgggtt 10080
   cagggtgggc accagctatg tatatgtaca tcagggacag ggggccaaag gcagtcagtt 10140
   tccaaagact gccccagagg ccatttttca gagaagccct gggttcctca agggccctgt 10200
   gtccatgctg gcccatcttg cagg 10224

<210> 63
<211> 11787
<212> DNA
<213> Homo Sapiens

<400> 63

tgtgaaatac tagccatgaa gaaattttga caataatatt tcagtgcaga attgcatact 60
agtaatagta tgcttggatt ttttcagata tgtggtatta atacatgttt ggaattttaa 120
gtgtcagatc aagaagaaaa acttaagata taaagtggtg ttttaataat ttgagtttta 180
tcacttttaa tgtcaagaaa ttattataca cataaacttt atgtctgtaa ttttagatct 240
attgttgcac aggatttctg gcaattacaa gtcagttgca tcaagtaaat tctgatttac 300
ttggctggtg gctttgtgaa cgacaattac cctcaggcgg gctcaatgga aggccggaga 360
aggtattgtt tgataagcca tattctgcta gctttagaac cttgagtgaa tgctgctgct 420
ttgtcttgcc tgtttctata ttgtaaattg gccatgagct tatttattac ctttccctgt 480
tgtgaatttt cagtaatata tcctactaaa agactcccct tgacaaagta gacccccatg 540
agttgaaata ctcattgatc ctaatgtcca tttccagaca gtcttgtaca ggaatctcca 600
tgtttaagga cagttgattt tggcaaattg gattttttaa aaattagact acagaatttt 660
tgtttgcaat cttcttattt aaggaatacc cagataattt ggtttggaga gaagattatt 720
aaattttcct ccacataata ggtctctctt atcaggtctg taagggacaa atagtgaata 780
ctcatttgca actatgtaat tgtcctgtag agcaagtgca gccataagtg gtaagaaatg 840
aatgagtatg gctgtgttcc aattacaatt attatggatg gatatacttg tatttcctat 900
gtcagatgga atcttgctct gttgcccagg ctggagtgca gtgacgccat cttgattcac 960
tgcaacctcc gcctcccagg ttcaagcagt tctgcctcag cctcccaagt agctgggact 1020
acaggcgcac ggctaatttt tgtattttta ttagagacgg ggtttcacca tattggccag 1080
gctggtctca aactcctgac ctcgtgatct gcctgcatca acctcccaaa gtgctgggat 1140
tgtaggcgtg agccaccgtg cctgacccag gactatattt aaatgattta taaatgtaaa 1200
aactattcct acactataga tttgactcaa ggattgtagc ttgctgtgcc cctgccctct 1260
gaatgggacg tggtggtaca gtagctggga ttacagtcat acaccaccac gcccatccaa 1320
ttttgtattt ttagtagaga tggggtatca ccatgttcat caggctgatc ttgaactcct 1380
gacctcaggt gatctacccg cctcaacctc ccaaagtgct gggattacag gcgtgagcca 1440
ctgtgctcgg cctgtatttt tatttttaaa ccttagtatt tctgaataac aacgggtaga 1500
gaaacacccg cagtagctgt ctacaataga gtgaaataaa aagcctgagg aactgtcaac 1560
agaaaaatgg gtggccgtgg ggagcaccgg gacctgcact tgcacacaat ctagggcagc 1620
ttctaatgag tctatgagtt actcagggat ctcattaaaa tgagatcagc cttgagtggg 1680
gttttcttta tagacttctg tcaccatatt ttgaagagca agggtttagt tgattttaag 1740
caggtgtgta cccccctgga cagtgcttgt agaaaaacag aactataccc atggatttca 1800

tgattatact gctagcttct tggttaaggg gctgagttag gcattcattg cagatagaaa 1860
caaagcagat gcatgcttat cagtgagtac tgagatattt agcatggtta cctatgaatt 1920
gaagtttggt ctcttcttac ccattaattg attatctttc attctaagca agtatttcat 1980
accctgcata tcagtatatc cctggatttc gtccttccac atggtttgac actttgaaca 2040
tcagattacc taagagtgag acttaaccca cttttaaatt gttctcaaaa ttagggaaat 2100
aaaagagaat gaaattgtgg caactttttt ccctcctagt taccagatgt atgctactca 2160
tggtgggtcc tggcttccct aaagataatt ggaagacttc attggattga tagagagaaa 2220
ctgcgtaatt tcattttagc atgtcaagat gaagaaacgg ggggatttgc agacaggcca 2280
ggagatatgg caagtaatat ttctgacata tttctataaa ttatttcagc gtttgcatta 2340
cttcatggtt ccatggattt ccagggtggc attccgagtg ttgctttgaa agcagttttt 2400
ttttctattt attgtagagt gtatgaagga tatagaacaa tcttacagaa atttcttgtc 2460
gcttgttgct aacaaaatta gtatatggta aaggtcaggt attcataatt tgaagttaaa 2520
gaagttttca ttttgtgatc tgtatataaa ttctttttta aataggtgga tccttttcat 2580
accttatttg gaattgctgg attgtcactt ttgggagaag aacagattaa acctgttaat 2640
cctgtctttt gcatgcctga agaagtgctt cagagagtga atgttcagcc tgagctagtg 2700
agctagattc attgaattga aagttgcata gtatagtttt gccattttaa catttctgta 2760
tttgaagtgc ttatcgaatc taaaagtgac tactgttaat attttgtata ttgtgttaaa 2820
ttaattttaa taaattatat aattatacat attgtaaaat aaagaccggt attttatttt 2880
ctgcttttta ttctgaagtc ctgttattct gactacagtt ctttgtgtat acttctgtgt 2940
ctgttatgtt caataactga gctaacataa aataactcta ggtttctact tgatttttcc 3000
cccatgtata cctttcatct gttctatagc aagttgatgt aaattggttt gtcaacaaga 3060
atgttaactg atgaaagtgg atagaaccca tacatgaatt aaatgatgca caaaataaat 3120
ggctgttgaa atttggaaat gattgatagg tttgaatatg gttattctga attggtcaca 3180
gacttaggtt aaatggcaat ctaccatctt aaaatggggt tgtgattcgg actgtccttt 3240
tttttttttt tttttttttt ttttttagca ttttatatat cagaattacc caaagaagga 3300
gttgtcttca aataaggaaa cactaacggg taagttgatg gtcaccacag tatttttttg 3360
cacctgtctt tttcttaaaa agctaaaatg ggtacaatca ctctttttta tttttttgtg 3420
acagaatctc tgttgcccag gctggagcgc agcagcacga tctcagctta actgcaacct 3480
ccgtctgccg ggtttaagca attcagccgc agcctctcga gtagctggga ctacagccgc 3540
acaccaccat gcccggctaa tttttatgtt actttagtac agacagggtt tcaccatgct 3600
gaggagctgg agaccagcct gaccttgtga tccacccacc tcggcctccc aaagtgctgg 3660
gattacaggc gtgagccacc acgcctggcc taaaatgagt acaatcactc taggggttta 3720
cagttttggt gaaagtgagg tattctctat ttttaaattt tattttttag agacagggtc 3780
ttcctttctt ccccaggctg gggcatagta gtgcaatcac agctcactgt aaccttaaac 3840
tcctgggctc aagcagtcct tccacctcag cctcctgagt accagggtct acaggtacct 3900
gccaccatgc ccactatgcc cagctgattt cttaattttg gggacaggca ctgtcttccc 3960
caggctggtc tccggaggtg aagtaatcct ctggcctcag tctcccaaag tactgggatt 4020
acagggagga gccatcgtgc ccgatctgaa aataaggttt tttgatggaa attgagattt 4080
ttctcaagta tgtttggaat gccctgtgca ataatcattc caagaacaga gatgttctta 4140
aggactgttg tcactgcatg tgggtaaacc gctgtgcata actggtttat tgagatactc 4200
aacagtggca ggatttcagt agtggtgtaa attaatgtct tctaatagct tctgtatggc 4260
agtatccaga taatttcgtc atgtttaatc catggatctc tggtaagttg gtactattgg 4320
ccttatttaa ggaattttct aaaggtcacc actgcaaagc tggagtttga acttctagtg 4380
tgccattaac tgatttatat gttagaaacc acagtactag aaattggtag atggggaatt 4440
gttaactgag agatcttacc cagtcagtct ttccctgttt cctactctac tatgcacaca 4500
gccatgtgca tactatcttt tgataatata actatctttc ctatatttgg tcacaagatc 4560
aaccttaggt gggttatgct gtgtgagcat tacacgttct cattgtgtca aaggcactgg 4620
gtgtactgtg ttgggtggtt tctacattta aagataagct agtgtgcttg gacgtgtggg 4680
aaatgcatgt aacatttgca ataagggaga ttgggtgctg tgagaataat ggggctaaca 4740
aatcgggtgg tcattgatgg gccttgcaga gttctggtca gtctgatacc tgcaggctga 4800
ggatgttcca gtgaggttca tgtggaaagt gatgggtttg ggaatggctg gaagcagggt 4860
catagacctg aagtacgtga atccatgtgg aaagctgagc ctgcctgcgc ctgcgcagtg 4920
cagcttagtg cgtcggcgcg cagttctccc gcccgtttca gcggcgcagc ttctgtagtt 4980
gggctactgg aggggtcgct cagaaacctc atacttctcg ggtcagggaa ggtttgggag 5040
gatgctgagg cctgagatct catcaacctc gccttctgcc ccggcggttt ccccgtcgtc 5100
gggagaaacc cgctcacctc agggtccccg ctacaatttc ggactccagg agactccaca 5160
gagccgccct tcggtccagg tggtctctgc atccacctgt cctggcacgt caggagctgc 5220
gggcgaccgg agcagcagca gcagcagcct tccctgcccc gcgccaaact cccggccagc 5280
tcaaggcaag gagtgattgg gtggaggagt ctccttgagg ctagaggccg gcagttcatg 5340
gaggctcggg ggcacgtggg tggtggttac cacagtgaag ttgtgatttg gttggggcgt 5400
gagatctgag tgccctggga atttggtgag tctgggtgat tagaagcctt gacttttcaa 5460
attggttcta ttgcaagacc ttgactgtgg atggctcagt tctgagaaag gctgaagtat 5520
cccatataac gtccctcaca gtgtctgaca aatgacagtt tgtgcgtgag tttgctgata 5580

gaagttgtga gctgatcttt ggctgctgaa tttaattctc caaatcctgc taaggtggga 5640
taaaatagat taaattgaac atttcatcta attcagggaa aataatatgt ttagtattaa 5700
aagagtgggc tgggcggtgg ctgacgcctg taatcccagc actttgggaa accagggtgg 5760
gcggatcact tgaggccggg agttccacac cagcctggcc aacatggtga acctcccacc 5820
cccatcccct atctctacta aaagtacaaa aattatctgg gtgtggtggc acgtgactgt 5880
ggtcccagct actcaggagg ttgaggtggg aggatggcga gtccgggagg tcgaggctgc 5940
cgtgagctga gattgcgcca ctgcatttca gcctgggtga cacagtgaga ccctgactca 6000
aaacaaacaa acaaaaaaga tgcatgtcct tcaaatcatt tattattttg ttagtctcta 6060
ttcatgacaa agcatcacca tttttcacac acgattgcaa cacaatggta aagtaacaaa 6120
taccaaatcc aatggtcatt tttcagttca tctcatgtga cctttctgct gtatttaatg 6180
ttcttaattt ctttattttt agaaacagag tctcgctatg ctgcccatgc tggtctcaaa 6240
ctcctggtct caagtgctcc tgcctcggtc tgcccaagca ttgggattac aggcatgagc 6300
cactgctgcc tggcctatgt ttaatgttct tgactggtct tctcttaaac tcttttaatt 6360
tggcttcctt gattccactt gcccctgttt ttctgctacc tcccagagaa ttgctgtgcc 6420
cacctttgtt cccaaattat gggcatgtca ttaaagcttt ttttcctagg ccacttctac 6480
ctagatgtga ctatctccac agctcagatc tcatctacac tccagactta cttcagactg 6540
tcttctgaag tttttattga tggactaatg gatacctcag gctgactata tgattaatta 6600
aattcctctc tgcacttttt tttttttttt gagacagagt cttgctctgt tgcccaggct 6660
ggagtgcagt ggcatggtca tgactcactg cagcctcaaa ttcctgggct caagcgatcc 6720
tcccacctca gcctcccgag tagctgggac caaggcatgt gcccctacag atggctaatt 6780
taaaaaattt ttttgtagag acagtgtttt cctacgttgc ccagcctggt ctcgaacttc 6840
taggctcaag cgatcctcct acctcagcct cccaaaatgc tgggattaca ggtgtgagcc 6900
actgcaccta gccctcaaac tcttaagtgt tcttccatcc ttgtagctta cttactgtac 6960
caccagagtt atgtggaaaa tgatcttatc atgtcccttg ccacttaaat cagtagacct 7020
gtatctgcag gtaaaagatc aaactctcta gaaggatagc caaagcaatt gggatcggtt 7080
tctaatcttt acagatttat ccctcatcat ttaatattta ccctgctttc taacaaaact 7140
gagctacttg caattccttt aaagcaacat gaatgttcac attgtcagtc taatccctct 7200
cttcatcaga tgcttggtca cccacttatt tatcagtaat gataacattc actaatttat 7260
atgcacctac tttgttctgg cttcttacta ggcagacggt atctttgatc ctcacaataa 7320
ttttgcagag tagatttttt ttttgttcct gttttacaga tagaaccaag gcccaaatag 7380
gcgaacaaag gttcaaatta tgttagcagc tggggataat aatctgtgct ctcaatgaac 7440
ttaaggttca gtagagaaga cagacttaaa aatcaacaaa tacacattta agaaataact 7500
gtatttgagg tatttataat ttttgaggta ggtataatta aagtatgggg acaaaagtgg 7560
gaaaagacat ttcacagagt aagtgcacct tgaactggtc ttcaagaata agtaggaaga 7620
agaatgccag ccaaagggaa acagctggag cgcattcttg agaaaaacac gtgcaaaggc 7680
acagaagaat catgtaaaac tgctaataat tttgcttggc tagagcagga gagtgatcta 7740
ggtagataaa cttgaatgta gatatagttg tgagaattag atctgggaga caaatttaac 7800
ccagagaccc cccatattca acaccaggtc tctggttttg gtgactggat agatggtagt 7860
agtattttct gattctggga attttgtagg caaagcaaat tttagaggga agatgatgag 7920
ctctgttatg gacatgctga gtgtgaaaag cctgtgggat atccatatat ttatattaag 7980
ataattgaat ataggaaact agagctaaga agagagaaac ttgagggaaa tatggatttg 8040
aaagttacca gtatgttggg accacagcag agaatatgaa gactatatag caaaatagag 8100
gtcaaggaag taggaggaga attaagacaa gtgaatgagt ttcaagaaag ggtatatagt 8160
ctaaaagtag tctgagaaca agaaacagcc ctctccatct ccagtttccg catctccagt 8220
ttctggcaac cactctcttc cttagctccc taaagcctag aaatgctaat ggagcacatt 8280
ttatgctggt tctacacacg tctttatgat agcaccccta caccctgtcc acacctttgt 8340
aaatattctc tttatcaaat tatcccagtt tgagtatatc attgattcct gctggaaccc 8400
atataatgaa gttatatttt accctatggt gagtatgggg agacacatag gaattgtaag 8460
aaagagagat actgatcaga tttgcttttc agaaagatta ttggtgatga ttcgaagaat 8520
agattagaga ggtgtaatac tggaagcaag gagaagaaga agtaggaaac ttgcacaagg 8580
accaaagaag atacaggctt aaaatgggct gggaggagta gagagggatt taaattttag 8640
tgatgttaaa aaaaaagaat tctagcactc agtcactgag tgttagggtt gatgaaggaa 8700
gaattgagaa tgtctcctaa gcttatgact tgagtgacaa gggacaagct ggtacaatta 8760
accatactgg taataaagaa ttaattttga gggaaagata gagttcactt ttggacattt 8820
agaatgctgg gaagatacct aaaggtttat gcccaagaga tagatatagt gacctggagc 8880
tacattgtct gatagaatat tctgtgataa tagaaacgtt tcatacttat gctgtccgat 8940
gtggtaacta gtagccgtat gtggcttttg agcatttgaa aatgttgcta gtgtgactga 9000
ggaacagaat ttaaattgta tttaattttg atttagttaa atactcacat atgctgcttt 9060
attgaacaat gcaggtatag agctgagaac ggtttttgca cttttgaagg attaaagaat 9120
caagaatatt ttgtgatatt aaaattatat gaaatttaga attcaatatg tagaaataaa 9180
gccattttta attgaaacat agccattctc atttatttat gtattatcta tgactgcttt 9240
tctatgatag tgtcagagct gagtaattgt gacagtgatt atatggccca caaaagccta 9300
aaatatttgc catctggtcc tttatttaaa agtttatggg ctgggcatga tggctcatgc 9360

ctataatccc agcactttgg gaggctgagg tgggcagatc acctgaggcc aggagttcat 9420
gaccagtctg gccaacatga caaaaccctg tctctattaa aaatacaaaa aaaaaatagc 9480
caggtgtggt gggcatgcct ataatgccag ctgcttggga ggctgaggca ggaggattgc 9540
ttgaacctgg gaggcagagg ctgcagtgag ctgagattgc gccattgcac tctagcctgg 9600
gcaacaagag tgaaactcca tctcaaaaaa aaaaaaaaaa agtttatgct gaggcatggt 9660
ggcttatgct tgtaatctca gtgctttggg aggctgaagt gggaggattg cttgaggcca 9720
ggtgtttgag atcagcctgg gcaatgtagc aagaccctgt ctctataaaa aaaaaattag 9780
ctgtgtgtgg tgaggtgcac ctatagtcca tagttactca agaggctgaa gcaggaggtt 9840
acttgagccc atgagcttga ggctgcagtg agctgtgatc acactactgc actccagtct 9900
gggcgacaaa gagagacctt gtctttataa gacagtaaaa tagactgggc atggtggctc 9960
ccgattataa tcccagcatt ttgggaggct gagatgggaa gatcacttga gcccaggagt 10020
ttgagaccag cctgggcaac atggcaaaac cccatctcta caaaaataca aaaaattagc 10080
ctggcatggt ggcatgagcc tatagtccca gctactcggg agggtgaggt aggaggattg 10140
cttgagccca ggagttcaag gctgcattta gccaagatca caccactgaa ttccagccta 10200
agctacagaa cgagagtctc acatacagac aaaaaggaaa aattaaaagt ttagcaaccc 10260
ctggtttaca gtttaaggag tcgttacagc tatatacgaa ggcttgagag ttgtcagaaa 10320
atagatgtta atagaaacaa agaggagaga tgagatgcct ggagatgcat aatgttgatt 10380
tgtgagggaa agcttaaaag tatgaagccc tggaggccaa cagttaagaa gcagaggaaa 10440
aaggagatgg caaaggaaac taaaagtggt ttaaagagaa ttcaagatag agagttgtgg 10500
aaatcaaaga gaataggtga acagagccca aacattttaa aattttttta gaacttctat 10560
gtgccaagag gctatgctag gttgtttatg tacattattt aaaaatttta tgacaatcct 10620
gtaaaatagg taatagctta catcagtaga taagtaccta aataaggaac tgaagccaat 10680
tgagtctaaa atgccatttg cataggggag agaaattcaa tgaagaaatt aacagaagaa 10740
ggttaaaaga ggtctaataa ttattataca aatgaaacat atgtcttagt ctgtattctg 10800
ttgcttttaa aagaatactt gaaactgggt gattatcaag aaagaaaaca tatttcttgg 10860
aagtctggag gctgggaagt ccaaggtcaa aggaggcaca tttagtgagg gtctttgtgc 10920
tggtggagtc tctgtagagt cctgtagtgg tacagggcat cacatggaga gggagctgag 10980
catggtagct caggtctctc atcctcttct tataaaacca ccagtcctac tctcgtgata 11040
acccattaac ctattaaatc atgaatggat taattcattc atgaaggcag agccctcatg 11100
ccccatcacc tcttaaaggc cccacctctg tcctgccaca cttcactggg gattaaattt 11160
caatgtaagt ttcggagggg acagatattc ataccttagc aacacatctg gtatatttga 11220
agattaaagg gtaatttatg attttagcca gagtagttgc agtgaacttg tagaggcaaa 11280
agctatatta gcatattttt gtatatttat caataatgtc attaattttg ttataatctg 11340
tttattttca aaaatcattt tcacatgaaa ttttatagaa agtgaacata gtggttgggt 11400
gcggtgactc aggcctctaa tcccagcact ttgggaggcc gaggcaggca gatcatgagg 11460
tcaggaattc gagaccagcc cagccaacat ggtgaaaccc catctctact aaaaatacaa 11520
aaattcacca ggcgtggtgg cgggtgcctg taatcccagc tactcgggag gctgaggcag 11580
gaggatcact tgaacttggg aggtggacgt tgcagtgagc cgagatcatg ccactacgct 11640
ccagcctggg cgacaagagc aagactctgt ctcaaaaaag aaaaaaagaa aaaaaaggaa 11700
agtgaacatg gtataaatta taatgactaa tacatcattg cataattcaa atctttaaga 11760
attgactgtt aatttttcaa atttagg 11787

<210> 64
<211> 2455
<212> DNA
<213> Homo Sapiens

<400> 64

ggagtgcaag aacacagaac taaaacagag cttgaaactt aaagaaaggg agagacttgg 60
gggaggagtg gggtggagtg acgtgatgtg ctgctggaaa ccagcagttg gtggtttcct 120
cttgtgcttc ctcttctgtg ggttttctcc tgcttgtggg agggcctttt tctctcctcc 180
cgacagaaag gctatctttg gtgttcgttc ccttgaactg taacatcctg taagggtatg 240
attccatgcc tctgtgtggg tgtgaattcc ctcatggtga ccctcaaaat ctgcacacag 300
gaccccttcc cattgagggg aggggatcaa aacaactcta cttctcaggg tcctctcctg 360
ttccaactgg tctgtgtcca agagaagcct taggtaaatg gggccagctt gaagatcaaa 420
caggtttggc agcctctccc ggcctctctt ttctctccta cagctttata gctacagctg 480
ccttgatatc aatattgact ttggctggct ggcatgacta cccacagggt atcgtgcctt 540
aatttaccag gtgacaggca acgctgccct ctcctggaac catccagcag agccagggct 600
gtacccccaa atcctgcaac agaggtttcc ctccatctca cctccctgtc cctgcatttc 660
tcctatctca gtagctcctc tttccctctc tgggcttctc tttccactcc ctccccttcc 720
tgggcttggt aaactagtcc ctaatctctt cacaccccag attggaaggt gggtccctcc 780
ctgacactcc ccagagctgt caccaacctc ctccaagttt ctatagctcc attgctcaac 840

agatttgcca ggggtaacca ttaacccagc ccttaactct gttcccccac ctttcttgct 900
ggaggggatt ttccaattac tggttagcac agctaggtca tctcaccccc accatctttc 960
ctaacttctt gggttggggg gctggggagg aatctcccca tctcagggta ctaggaacaa 1020
agctggggag gatggtgcat ttaaagggat tatatatata tatatatata ttttttttct 1080
ttctccctca taaccccacc cccgcaacac acacacacac acacacacac acacacacac 1140
acacacacac agacgcacaa ataagcttta tggagcagtg acttcattat gttcaccgct 1200
ttgagtccaa cccctggccc aaaataggca ctaaatagtt gccgaatgca tgaatgatag 1260
atacctctct gtcttcaggg gtgtgtagaa gtgcgaaggg gtatgggcat gtcccagtag 1320
gggtgtgagt gttctgatca gaactacttc tctctgccag aatttgatgt aattcgaatg 1380
cttccacctc tgcttgaagg gtttaaataa taaattaggc cctgtcgtgc cattatgggg 1440
gtggtcatac cctgtaccca ggaaacaggc acggtagggc tgagacagaa gtcctgcttg 1500
tttccgctta tttatttgaa acaccgctca tttaggtctt actttgtttg ccaggcactg 1560
ttctaagctc tgtataaata ttaactcaga gggtacaaat attaacttaa gagttgttgc 1620
aggaaaaaaa ataagcgcct ctggctcttt aagtttggcc tccccctcaa aacccccgca 1680
acggtcccaa accccttcca gggactggga ctacggaccc tggtccgacc ttctcgcggg 1740
cttcccactg cgccaatcaa atcccagaaa cagtgagtgc tagaggcccg gctgctaagc 1800
aacggcagag ggcgggaagt ttgaacgttc tggacccgcc ccgaaggcaa ataggccaat 1860
cagcgtccag actcttcagc tacggcagtc cgcttctcct cctcgccctg tcggatctct 1920
aggctggatc cgggcctctc caatcaacag cggctaggag ggcggggcgc gtgcgcgcgc 1980
acctcgctca cgcgccggcg cgctcctttt gcaggctcgt ggcggtcggt cagcggggcg 2040
ttctcccacc tgtagcgact caggttactg aaaaggcggg aaaacgctgc gatggcggca 2100
gctgggggag gaggaagata agcgcgtgag gctggggtcc tggcgcgtgg ttggcagagg 2160
cagagacata agacgtgcac gactcgcccc acagggccct cagacccctt ccttccaaag 2220
ggtaacctcc gcgtgacagg aatgagggtg gggcgcgtgg agtttcccac aatctgtact 2280
ttagttaaat acccgagaat tcacctcctg tgtccacagc tctccacgcc cctcagccct 2340
gccccgcagc cctgtagcag aagtacttag tgctttgcat tctgcgcgcc accctacccc 2400
ggcctcctct gtgaatcgtt gcttccgaac cgccctcact ttttgcatcc gcaga 2455

<210> 65
<211> 18
<212> DNA
<213> Homo Sapiens

<400> 65

aatgctttcg tgatgctg 18

<210> 66
<211> 18
<212> DNA
<213> Homo Sapiens

<400> 66

ttgcccagcg gaccccag 18

<210> 67
<211> 18
<212> DNA
<213> Homo Sapiens

<400> 67

aacctgcgaa cgcttggg 18

<210> 68
<211> 18
<212> DNA
<213> Homo Sapiens

<400> 68

gggcaacgcc gaacccag 18

<210> 69
<211> 18
<212> DNA
<213> Homo Sapiens

<400> 69

aaggaatccg tcttgtaa                                                    18

<210> 70
<211> 18
<212> DNA
<213> Homo Sapiens

<400> 70

ggagtccgcg ggaccctc                                                    18

<210> 71
<211> 18
<212> DNA
<213> Homo Sapiens

<400> 71

aaagctctcg gaggggct                                                    18

<210> 72
<211> 18
<212> DNA
<213> Homo Sapiens

<400> 72

tgccatcccg gagggccc                                                    18

<210> 73
<211> 18
<212> DNA
<213> Homo Sapiens

<400> 73

tagactcgga ggtaggca                                                    18

<210> 74
<211> 18
<212> DNA
<213> Homo Sapiens

<400> 74

ctcatttccg tgggtaaa                                                    18

<210> 75
<211> 18
<212> DNA
<213> Homo Sapiens

<400> 75

aagccacggg cttcactg                                                    18

<210> 76
<211> 18
<212> DNA
<213> Homo Sapiens

<400> 76

ataggctacg ggctggag                                                    18

<210> 77
<211> 18
<212> DNA
<213> Homo Sapiens

<400> 77

gtggggctcg gaggcaca                                                    18

<210> 78
<211> 18
<212> DNA
<213> Homo Sapiens

<400> 78

aagtaagtcg ttgctgcc                                                    18

<210> 79
<211> 18
<212> DNA
<213> Homo Sapiens

<400> 79

agtgcatgcg cagaaggc                                                    18

<210> 80
<211> 18
<212> DNA
<213> Homo Sapiens

<400> 80

aggattccag ggtattca                                                    18

<210> 81
<211> 18
<212> DNA
<213> Homo Sapiens

<400> 81

ggagtccccg gcacacat                                                    18

<210> 82
<211> 18
<212> DNA
<213> Homo Sapiens

<400> 82

cagtggcgtc ggaactgc                                                    18

<210> 83
<211> 18
<212> DNA
<213> Homo Sapiens

<400> 83

atttggctcg acctggac                                                    18

<210> 84
<211> 18
<212> DNA
<213> Homo Sapiens

<400> 84

agggaggccg ttggcacc                                                    18

<210> 85
<211> 18
<212> DNA
<213> Homo Sapiens

<400> 85

taaaatctac agtcgggt                                                    18

<210> 86
<211> 18
<212> DNA
<213> Homo Sapiens

<400> 86

gctccgccgg gcctgcgg                                                    18

<210> 87
<211> 18
<212> DNA
<213> Homo Sapiens

<400> 87

tacttgggcg ggtgagtg                                                    18

<210> 88
<211> 18
<212> DNA
<213> Homo Sapiens

<400> 88

cctcttcatc tacctcaa                                                    18

<210> 89
<211> 18
<212> DNA
<213> Homo Sapiens

<400> 89

gcccagccag cgggctcc                                                    18

<210> 90
<211> 18
<212> DNA
<213> Homo Sapiens

<400> 90

aaaaacccgc gagcttag                                                    18

<210> 91
<211> 18
<212> DNA
<213> Homo Sapiens

<400> 91

tgatgtggcg ggactctt                                                    18

<210> 92
<211> 18
<212> DNA
<213> Homo Sapiens

<400> 92

ctaggtagcg ggcagtag                                                    18

<210> 93
<211> 18
<212> DNA
<213> Homo Sapiens

<400> 93

taggtttcta aagaaact                                                    18

<210> 94
<211> 18
<212> DNA
<213> Homo Sapiens

<400> 94

accaggctcg tgccctag                                                    18

<210> 95
<211> 18
<212> DNA
<213> Homo Sapiens

<400> 95

tgccttctat gtgtgaga                                                    18

<210> 96
<211> 18
<212> DNA
<213> Homo Sapiens

<400> 96

agagctaccg ctctgccc                                                    18

<210> 97
<211> 18
<212> DNA
<213> Homo Sapiens

<400> 97

tgccacaccg gccagctg                                                    18

<210> 98
<211> 18
<212> DNA
<213> Homo Sapiens

<400> 98

tctgcacccg gagttggg                                                    18

<210> 99
<211> 18
<212> DNA
<213> Homo Sapiens

<400> 99

aagatcttcg gccagccc                                                    18

<210> 100
<211> 18
<212> DNA
<213> Homo Sapiens

<400> 100

tccaggtccg caagccca                                                    18

<210> 101
<211> 18
<212> DNA
<213> Homo Sapiens

<400> 101

cacactggcg aaggccaa                                                    18

<210> 102
<211> 18
<212> DNA
<213> Homo Sapiens

<400> 102

tggaggcccg ggagccca                                                    18

<210> 103
<211> 18
<212> DNA
<213> Homo Sapiens

<400> 103

ttaggccgaa aagacaac                                                    18

<210> 104
<211> 18
<212> DNA
<213> Homo Sapiens

<400> 104

gagcggcccc ggtgccgc                                                    18

<210> 105
<211> 18
<212> DNA
<213> Homo Sapiens

<400> 105

aatcaggccg gacaggag                                                    18

<210> 106
<211> 18
<212> DNA
<213> Homo Sapiens

<400> 106

ctggtccggg aaaggcaa                                                    18

<210> 107
<211> 18
<212> DNA
<213> Homo Sapiens

<400> 107

acttgtcccg accaatct                                                    18

<210> 108
<211> 18
<212> DNA
<213> Homo Sapiens

<400> 108

aatagttacg gtcggagg                                                    18

<210> 109
<211> 18
<212> DNA
<213> Homo Sapiens

<400> 109

agggtcaccg cgtctatg                                                    18

<210> 110
<211> 18
<212> DNA
<213> Homo Sapiens

<400> 110

ggtggtgttg gttggtga                                                    18

<210> 111
<211> 18
<212> DNA
<213> Homo Sapiens

<400> 111

actctgcgct cgcaccca                                                    18

<210> 112
<211> 18
<212> DNA
<213> Homo Sapiens

<400> 112

agttgctccg cagatact                                                    18

<210> 113
<211> 18
<212> DNA
<213> Homo Sapiens

<400> 113

tagcagcccg ggtagggt                                                    18

<210> 114
<211> 18
<212> DNA
<213> Homo Sapiens

<400> 114
   atccagctcg ggggagga                                                     18

<210> 115
<211> 18
<212> DNA
<213> Homo Sapiens

<400> 115

ctgctccctc cttgggga                                                    18

<210> 116
<211> 18
<212> DNA
<213> Homo Sapiens

<400> 116

ggagccttcg gctgactg                                                    18

<210> 117
<211> 18
<212> DNA
<213> Homo Sapiens

<400> 117

tatgtacacg agcactgg                                                    18

<210> 118
<211> 18
<212> DNA
<213> Homo Sapiens

<400> 118

agcaggaccg ggattctc                                                    18

<210> 119
<211> 18
<212> DNA
<213> Homo Sapiens

<400> 119

aagtgcccgc ggctgccc                                                    18

<210> 120
<211> 18
<212> DNA
<213> Homo Sapiens

<400> 120

ggagtccggt cgggcccc                                                    18

<210> 121
<211> 18
<212> DNA
<213> Homo Sapiens

<400> 121

agtcatttcg tcacatgg                                                    18

<210> 122
<211> 18
<212> DNA
<213> Homo Sapiens

<400> 122

gtgtcagccg cctagggc                                                    18

<210> 123
<211> 18
<212> DNA
<213> Homo Sapiens

<400> 123

tcaagtgccg gacttgtg                                                    18

<210> 124
<211> 18
<212> DNA
<213> Homo Sapiens

<400> 124
   caaagaggcg gggagacc                                                     18

<210> 125
<211> 18
<212> DNA
<213> Homo Sapiens

<400>, 125

agtacttccg tttgagga                                                    18

<210> 126
<211> 18
<212> DNA
<213> Homo Sapiens

<400> 126

cagtgagacg aggctccc                                                    18

<210> 127
<211> 18
<212> DNA
<213> Homo Sapiens

<400> 127

ttataatgcg agggtctg                                                    18

<210> 128
<211> 18
<212> DNA
<213> Homo Sapiens

<400> 128

tcccagagcg ctagggct                                                    18

<210> 129
<211> 18
<212> DNA
<213> Homo Sapiens

<400> 129

acatctggcg gaaacctg                                                    18

<210> 130
<211> 18
<212> DNA
<213> Homo Sapiens

<400> 130

ctttgccacg gtggtgga                                                    18

<210> 131
<211> 18
<212> DNA
<213> Homo Sapiens

<400> 131

agaggttgcg tcccagag                                                    18

<210> 132
<211> 18
<212> DNA
<213> Homo Sapiens

<400> 132

gctgcttccg gctggtgc                                                    18

<210> 133
<211> 2932
<212> DNA
<213> Artificial Sequence

<220>
<223> chemically treated genomic DNA (Homo sapiens)

<400> 133

gttaagaata aaatatatta agataaggaa aatttgtagt taagaataga aaaaaattat      60
ggttttgaag tatgagttat ttaaagaaag tggaaatatt tttagattat gtagtaaaaa     120
ataaagtgat tttttttttt taaatttatg taataaattg ataggtaata tgtgaaagtt     180
atagaatgta gattagagga tataataaat ttattttttt tatgtttata agaagtaaga     240
aaagttttga tgtgagttag tattgtttta taattttgaa ttgtgtagat tgtacgtatt     300
tttttttagt ttgaagtaaa tagtggatag gaaaaaatat taaatgttgg tagtaaatat     360
ggaaggaaat tataattaat gtaatatgtt aaaatatgtt atgtttattt tattaatttg     420
aattaaaatg taagaattta aaatgttttg gaaaaatacg ggtattgatt tgacgtttga     480
agttttaaaa tattatatat tttgaaatag tatttgtatt ttgaaatatt tgtttttata     540
tattttttaa aatttttttt tttttttatt ttatttatta ttaaataaag gatgaataga     600
tgtaatttag aaattgttaa gtatgttgaa gaaagattat tgtagaaaaa ttttttttag     660
tttttttaaa ggtgttagga agtagaaagg tgatatagaa ttggagaggt cggagttttt     720
gtattaattg tattaaatgc gaatttcgag aaaatttttt ttaattacgt tttgtagtta     780
tatggatatg aagatttatg tgaattttga aagacgtgtt tatataagtt gaaatgtttt     840
taatgattta gttgatgcgc gtttttttat ttgttttttt tagagaggtg taacggaagt     900
tagaatattt tttttggaaa tttaatttgt ttcgtagttt ttcgaggaat tagtatttag     960
ttaattcggg tcgggagtag ttatttgtgg tgaggttgat tggttgggta ggaatagcgt    1020
cggggcgtgg gttgagtata gtcgtttcgt tttttttgtt ataggaagtt tgagtttatt    1080
cgagtagcgg tttttttaag tttaaagaag tagaggtcgt tgttcgtttt ttttaggttt    1140
ttttattaaa gtcggagtat ttttttttaa aattttacgt tttggtggtc gttttaagga    1200
gcgcgaggta ggggtacgta aagttgggag ttattatggg atagttttta agtgttaggt    1260
ttttagattt tttgaatttg gtttttacgg gagaagggtt ttttgaggcg tggatagtgt    1320
gaagtttttt ggtaagttta tggggattaa gtggggttag atttagattt aggagttttt    1380
ggagtagcgt ttaaatcgta gtggtattgg attatgttgt tcggagcgcg tatagttcgc    1440
gcggtgcggg gatttgtttt ttgagttcgc gggcggtggg tgggaggaag tatcgttcgc    1500
ggcgattgga atcgggaggg agaatcgtat tggcggcggg taaagtttag aacgcgttgt    1560
tagattttta attttgtttt cgtggagatg ttggagattt cgcgtatagg aaagtttttg    1620
tagtgtttat cgcggttaga gtagttgggg tattaacggc gggcgttttt ttttattgtt    1680
ttttggtttc gacgggggat tagaggttag ttttattttt agcgcgtttg aggtttatgt    1740
atttggttaa tgagttgcgg ttttttttta ggtcgggatg gattttgaag gggatcgtaa    1800
tggaggagta aagaagaaga atttttttaa attgaataat aaaaggtaat tagtttgttt    1860
tatttttata gtttatatag ttgcgagatt tgagtaattt atttttagtt tttagttttg    1920
aaataaatga tatgttgttg tttttaatta tttttaagaa acgtaagtta gtttttggaa    1980
ttaatatttt tgtttagagt agaagtttgt tggttgagtg gagtatagta tatgtatttt    2040
ttttgttttt tttgtttttt tttttaatga tatataatat tttatatatt tatgaaatgg    2100
ggtatatgga agcgtttttt atatgttcgg aatgtgtaat gattaagttc gggtatttga    2160
aggatatatt attttaggta tattttattt ttatgtgttg ataatatttt aagtttttta    2220
gttattttga aatatataat atattgttaa ttgtagttat tttcgtttgt tatcgaatat    2280
tggaatttat ttgttttatt taatcgtttt tagttattta ttaatttttt tttattttat    2340
ttttttattt ttttcggttt ttttttttta gttttggtgt gttttttttt tagttttttt    2400
gttttagata ggcggatgtt tatatgtgtt tttgttttat gaatttttgt tttttaagtg    2460
gtgttggtcg tttatacgtg agttatatgt tgttggtgat ttgttttgtg gtttaggttt    2520
ttgttttcgg taaatggtta tgtaaatatc gcgtttgtgg tttggttgat gagatagaag    2580

gttaaaagta tatttaggtt gttaattggt aataaatatt tgtatataat attggtaatg 2640
taattatata gggaaaataa ttatttaaag taaattttga ttatggtgtt ttgtttttat 2700
agaatattta aaattttatt aaatagattt attgttagta gtaaattgta aaatagatta 2760
gtaagtttaa taatattaga aattgtaatg taaattataa gataaattag ttaaatatat 2820
taatattata agaaattaag ttttttagtg taagagaaaa aatataaatg tggaaattaa 2880
atatattttt aaaaataatg ttaagtttga attagaaatt ttaatatgaa tt 2932

<210> 134
<211> 2932
<212> DNA
<213> Artificial Sequence

<220>
<223> chemically treated genomic DNA (Homo sapiens)

<400> 134

aatttatatt gaaattttta atttaaattt aatattattt ttaaaaatgt atttgatttt 60
tatatttgta tttttttttt tatattgaaa agtttggttt tttataatat taatgtgttt 120
agttaattta ttttataatt tatattatag tttttaatat tattaaattt attagtttat 180
tttataattt attattaata atgaatttat ttagtgaagt tttaaatatt ttataaaggt 240
agagtattat gattaaaatt tattttaaat aattattttt tttatatgat tatattatta 300
atattatata tagatattta ttgttagtta ataatttaaa tgtatttttg attttttgtt 360
ttattagtta ggttataaac gcgatgttta tatagttatt tatcggaagt aagagtttgg 420
attatagagt agattattag tagtatatgg tttacgtgtg ggcgattaat attatttgaa 480
aagtagaagt ttataagata gaaatatata tgagtattcg tttgtttggg gtaggaaagt 540
tgagaaagag gtatattaag attaagggaa agaggtcggg aagggtaaaa aggtgaaatg 600
aaaagaggtt ggtgaatgat taagaacggt tggataggat aaataagttt taatgttcga 660
tagtagacga gggtgattat agttagtaat atattgtata ttttaaagta gttagaagat 720
ttaaaatgtt attaatatat agaaatgaaa tatatttaag gtgatgtatt ttttaaatat 780
tcggatttga ttattatata tttcgggtat gtaaaaaacg tttttatgta ttttatttta 840
taaatatgta aaatattatg tattattaaa agaaagaata aaaaagatag ggaaaatgta 900
tatgttgtgt tttatttagt taataaattt ttgttttaag tagggatatt gattttaaag 960
gttagtttgc gttttttaaa aataattaaa aataataata tgttatttat tttagagttg 1020
gaggttagaa ataaattatt taaatttcgt aattatgtaa attatgaaaa tgaaataagt 1080
tagttatttt ttattgttta gtttaaaaaa gttttttttt tttgtttttt tattgcggtt 1140
ttttttaaga tttatttcga tttgaagaga aatcgtagtt tattagttaa atgtatgagt 1200
tttaggcgcg ttggaggtga gattaatttt tagtttttcg tcgaagttag agagtagtaa 1260
gagggagcgt tcgtcgttga tgttttagtt gttttggtcg cgatgggtat tgtaggggtt 1320
tttttgtgcg cggggttttt agtattttta cgaaggtaga gttgggggtt tggtagcgcg 1380
ttttggattt tgttcgtcgt tagtgcgatt ttttttttcg gttttagtcg tcgcggacga 1440
tgtttttttt tatttatcgt tcgcgggttt agagagtagg ttttcgtatc gcgcgggttg 1500
tgcgcgtttc gggtaatatg gtttagtgtt attacggttt gggcgttgtt ttaggagttt 1560
ttgagtttag atttaatttt atttggtttt tatggatttg ttagaggatt ttatattatt 1620
tacgttttaa gaagtttttt tttcgtgaag attaagttta ggaaattgaa aagtttgata 1680
tttgggaatt gttttatagt agtttttagt tttgcgtgtt tttatttcgc gttttttgga 1740
acggttatta agacgtgaaa ttttggaaga agatatttcg attttagtgg aaagatttaa 1800
aggaaacgaa tagcggtttt tgtttttttg agtttggaag agtcgttatt cgaatgagtt 1860
taggtttttt gtggtaaaga gagcgaagcg gttgtgttta gtttacgttt cggcgttgtt 1920
tttgtttagt taattagttt tattatagat gattgttttc ggttcggatt gattgaatgt 1980
tgatttttcg agaaattgcg aaataggttg aatttttagg aggaatgttt tggttttcgt 2040
tgtatttttt tagaaagggt aagtagagaa acgcgtatta gttgaattat tggggatatt 2100
ttaatttatg tagatacgtt ttttaaagtt tatataagtt tttatattta tataattata 2160
ggacgtagtt aagggaaatt ttttcgggat tcgtatttaa tatagttaat ataaaaattt 2220
cgattttttt aattttgtat tatttttttg ttttttaata tttttgaaaa ggttaggaga 2280
aatttttttg tagtggtttt tttttagtat gtttgatagt ttttgagtta tatttgttta 2340
ttttttattt gatgataaat ggaatgaaag aaaaaggaag ttttaaaaaa tatatagaga 2400
taggtatttt aaggtataaa tgttatttta aagtgtgtaa tattttaaaa ttttagacgt 2460
tagattaatg ttcgtgtttt tttagggtat tttaaatttt tatattttaa tttaaattag 2520
taaaataaat atagtatgtt ttagtatatt atattagttg taattttttt ttatatttat 2580
tgttaatatt taatattttt ttttgtttat tatttatttt aaattgagga aaagtacgtg 2640
taatttgtat aatttaaaat tgtaaagtaa tgttaattta tattagagtt ttttttattt 2700
tttatgaata tagaggaaat aggtttgttg tattttttag tttatatttt atgattttta 2760

tatattattt attagtttat tgtataagtt tagaagaaga aaattatttt gttttttatt 2820
gtatagtttg aggatgtttt tatttttttt aaataattta tattttaaag ttataatttt 2880
tttttatttt tgattataaa ttttttttat tttgatatat tttgtttttg gt 2932

<210> 135
<211> 7432
<212> DNA
<213> Artificial Sequence

<220>
<223> chemically treated genomic DNA (Homo sapiens)

<400> 135

atttgaagtt agagttattt agaggagaaa gagttggaat tgagaattta aggaatgttt 60
ggaagtgatc gggttcgagt ttatttagga agaaatagag gttggagata tgagattgtg 120
ttgttatttt tttttattaa tttttgggtt ttattgaggt tttattataa gtttggtttt 180
gtagtttagt gattaggaga aattagatat aagataataa taatagtaat gatttttttt 240
ttttttttga gacggagttt tgttttttcg tttaggttgg attgtagtgg cgcgatttcg 300
gtttaatgta agttttattt tttaggttta cgttattttt ttgttttagt ttttcgagta 360
gttaggatta taggcgtttg ttattacgtt tggttaattt tttatatttt tagtagagat 420
ggggttttat cgtgttagtt aagatggttt taattttttg atttcgtgat tcgtttgttt 480
cggtttttta aagtgttggg gttataggta tgagttatcg cgtttggtta atagtaatga 540
tttttgagta tttatattgt tagtttttac ggtaagagtt ttttttattt tttgttttgt 600
tttgttttaa gatagagttt tgttttgtta tttaggttgg agtgtagtgg tgtgatcgcg 660
gtttattgta gtttttattt ttcgggttta agttattttt ttgttttagt tttttaagta 720
gttgggatta taggtacgta ttattatttt tggttaattt ttgtattttt agtagggata 780
gggtttttta ttatgttggt taggttggtt ttaaattttt ggttttatat gatttgttta 840
tttcggtttt ttaaagtgtt gggattatag gcgtgagtta ttgcgttttt ttttgtattt 900
gtttaagtaa tatattgaaa tatgtattgt gttttttatt ttatggagga ggaaattgag 960
gttagtaaat gaggttgtta tgggaggtgg agataggatt tgaatttgtt ttagtgtagg 1020
aggtttaaga gtttttgttt ttttttaggg tattgtgtgg gagggtgaga aggagggagg 1080
tttatagagg tatgattttt gattgttatt gttatttggg ttttgttttt tgaagttttt 1140
gttaagcggg gaggtggtcg ggggagggtt ttgttttgtg tagttttttt tttttcggtt 1200
cgtagagttg agtatagagg gatagaggta cggaattttt agaaatgttt ttttttagaa 1260
ataggtttta ggttttgttt gttttgtgtt tttgcgtgtt ggttttggtt tgtattgggg 1320
gtgagaagaa gtgggtggag ggatgtgggg tttatatttg gtgggtgtga gtgtggttgt 1380
gtgttttgtg gttttgtagt tacgtgagat atgagtacgg agtgtgtgcg ttttatggcg 1440
tgcgtatgta tgtgcgtgtc ggggagtgtg tgtgtcggtg gttgagagtg aagtgtgaat 1500
gttatattgg tataaattgg gattatttgt gtgtgtgtac gtgcgtgcgt ggaagtggga 1560
gtatgtagtc gtggtaaaaa agtgtatgtt tgtgtgtata tgtgtatttg tgtgtatttg 1620
tttttttgtg gggtatgtgt gtgtaaaata tttgagtgtg tggatatgtg tgagggggtg 1680
agtgtgtgtt ggtgtgtacg tttgtgtttt gtatatgtat tttttttttt ttttttgaga 1740
cggagtttta ttttgttatt taggttggag tgtagtggta gtagtggtgc gattttggtt 1800
tattgtatta ttcgtttatt cgttttaagg gatttttttg ttttagtttt tagagtattt 1860
gggattatag atatacgtta ttatgtttgg ttattttttt tttttgagac ggagtttcgt 1920
tttgttattt aggttggagt gtagtggcgt gattttggtt tattgtaacg ttcgtttttc 1980
gggtttacgt tatttttttg ttttagtttt tcgagtagtt gggattatag gagtttatta 2040
ttacgtttgg ttaatttttt gtatttttat tagagacggg gtttcgtcgt gttagttagg 2100
atggttttta tattttgatt tcgtgattcg tttgtttcgg ttttttaaag tgttaggatt 2160
ataggcgtga gttattgcgt ttggttaatg tttggttaat ttttttatat ttttggtaga 2220
gatagggttt tgttatgttg tttaggttgg ttttgaaatt ttgattttag gtgattcgtt 2280
cgttttggtt ttttaaagtg ttgggattat aggtatgagt tattacgttc ggttatgtat 2340
tttatgttaa aatgggatta tattttagat tagtattatt tagtagaaat ttaaattttt 2400
aatatagggt taggtacggt ggtttatgtt tgtaatttta gtattttcgg aggtcgaggc 2460
gggtggatcg taaggttagg agatttgaga ttattttggt taatagatgg gtaaaaattt 2520
atttttatta aaaatataaa aaattagtta tgtatggtgg tatgcgtttg tagttttagt 2580
tattcgggag gttgaggtcg gagaattatt tgaattcggg aggtagaggt tgtagtgagt 2640
cgagatcgcg ttattgtatt ttaatttggg tgatagagcg agatttcgtt tgaaaaaaaa 2700
aaaaaattta atacgtatgt agataatgtg taaggtatta ttttatgtgt atcgtatgta 2760
gtaattttta atttttacga taattttgag gtagatatta ttatttcgtt ttataaaagg 2820
agaaatagtt ttggggagat aggataagtt atcggttaag gtatatagtt agttatatgt 2880
ggttttcgcg tgacggttgg tttttgtagg cgaggttttg tttagatgcg tgggtagaag 2940

gtttggttcg gaaagaggaa ttgatagtaa ggttaagtta atgtttgttt ttgggggtag 3000
aaagttattt tttgtttttt ttttattgtt tatagaggta gtttagatag ggtgggggtt 3060
ataggagaac gaagggagaa gggggtagtt tttgggtagt aaaattaggt ggtgaaggga 3120
ggtattagag gatggtaatt agagaggtta ttagagggga attataggta gatagggtga 3180
taggagggat tattgatata aggtgaagag atggtttagt cggacggggt ggtttatatt 3240
tgtaatttta gtattttggg agttcgaggt gggtggatta tttgaggtta ggagttcgag 3300
gttttaatat ggtaaaattt tatttttttt aaaaatataa aaattagtcg ggtatgatgg 3360
tagatgtttg taatttttgt tattcgggag gttgaggtag gaaaattgtt tgaatttagg 3420
aggtggaggt tgtaatgaga cgagattatg atattgtatt ttattttggg taatagagta 3480
agagattgat tttgttttat aaaaaaaaag aaaaaagaaa aaaaaaaaga gatggttgat 3540
ggttaaagag gggttagcgg ttaggggata tataaggtta aaggtaggag gtaagaggat 3600
tggtaggggg ttgtttttgg ggttatcgga gcgatatagg atgagtatgg agggaaaggg 3660
agaaggggat tttagggttt tagtttattt aagttgtttt ttggttttat ttagtatgtt 3720
agttagaggt ttaggaagga attttgagtt ttttattaaa gttaaagatg agtcgttgga 3780
gtttggtgag gggtaggatg aaggagttgt tggagatagt ggtgaatagg attagagacg 3840
ggtggtaatg gttttggtga gggtgtgttg ggttgggtgg tgggagggga tttttgggtt 3900
tgagggagga ggggttgggg tttggatttt tgagttttag ggaggaggaa agggtgggag 3960
tggggttgtg atttttaggt ttgggaggag tggagggtta gagttgagag taggaatttt 4020
taggttatag agaggagcgg ataaatgggg tagagaatat ttggggagag ttggggtttt 4080
tattgtgatg tttttttttt tgtaggagtt cgagtatttt tcggggtttt atgtagattt 4140
attatgacga ttatttgagg gatttgggtt cgtttattaa ggtttggttt ttcgaattta 4200
aagatagttt tttgaagaag atttatagtt tgtgttttag gtttgtttgt ggggataagg 4260
attagggtta aaatgtttat aaaagttagg tgtggttgtg gcgggtgttt gtagttttag 4320
ttatttagga ggttgaggta ggatgatggt ttgagtttag gagttcgaga ttagtttggg 4380
taatatagcg agattttttg ggggtaaaat aaaaagaaaa aaaaaagttt atattttttt 4440
aataaataaa gttttatttg tgtttttgtt tggatttttt tttagtgtgg ttagaaaaaa 4500
atttatttta ttgtttttta ggaattaatg agtagaagag gtgatatttg atggggaagg 4560
aagagtaggg aggtcgggaa gggtattaag gaataatatt ttattgtggg tttgcggaga 4620
atgggggatt ttaaggcgtg ttagttttag gagggtgagg gtaggagcgt gggtggagtt 4680
agtaggtttt tatgatggtt tttattgaga gtttcgtttt tgttttttat aagttttgat 4740
tttattttta gtgggtattt agtattttta atttttttat agtttttaat ttagtttttg 4800
tcggaggcga atttttagag tgagggtttt ttgttatttg agagaaggtt ttttgtgacg 4860
tgattttggg ggacgttatt gttttttttg ttttatttta tttttttcgt agttttgttg 4920
gttaggattt tggtttagat aaaggatggg ggttgtggtt gtggagcgga agtgggtttt 4980
aattattata aatttttttt gtgttcgttc ggagttggtg aggatagttt gttagagttt 5040
ggtaagaaag ggatttaggg tgcggggata ggggggcgtt agtagggaga gggtaaagat 5100
cgataaagta ggaattttaa gaggtataat attagaagtt cgtgttggaa ttatgattgt 5160
gtgtgtgtgt gtgtgtgtgt gtgtgtgtgt gtgtgtgtgt gagagagaga gagagggaga 5220
tggagtttcg ttatgtagtt taggttagat ttaaattttt gggtttaagt aatttttttg 5280
ttttagtttt tttagtagtt gggattatag gtgtattatt atattttata aattatagaa 5340
tttagaattg tagattattt gagtttttgt ttagagttag ggtggttgag gtggggagga 5400
ttttttgagt ttaggagttt gaggatgtag tgagttgtga ttttgttatc gtgttttagt 5460
ttgggtgata gagaaatttt atttttaaaa aagagaaaga aaaagggata ggtataatgg 5520
tttatgtttg taattttagt attttgggag gtcgaggcgg gtggattatt tgaggttagg 5580
agttcgatat tagttttatt agtatggtga aatcgtattt atattaaaaa tataaaaatt 5640
ggtcgggtgt ggtagtatat gtttgtaatt ttagttattt tagaggttga gataggagaa 5700
ttgtttgaat ttaggaagcg gaggttgtag tgagtttaga tcgtgttatt gtattttagt 5760
ttgggtgata gagtaagatt tagttttggc ggaaaaaaag aatgaaaaaa tttaaaaaat 5820
taaaaaagaa ttgtaggttg ggcgtggtgg tttatatttg taatttaaac gttttgggag 5880
gttaaggtaa acggattatt tgatgttagg agttggagat tagtttggtt aatatggtga 5940
aatttcgttt ttattaaaaa tataaaaatt agataggtat ggtggtgtat gtttgtattt 6000
ttagttattt aggaggttga ggtaggagaa tcgttcgaat tcggaagata gaggttgcgg 6060
tgagttaaaa ttgcgttatc gtattttagt ttgggcgaga gaataagatt ttgttttgga 6120
aaaaaaaaaa gaattgtaga ttatttgttt gtgttttttt ttcggggatt agattttatt 6180
ttttttttgt cgtatttttt tattttttac gtgtggatga tgatattgtg ttttgtgtat 6240
gtttttcgtt attaaaagtg ttttttttat agagtaggtg agaatttagt gaggagatgt 6300
agggatatga ggtttgattt agggtagagt tttaaggtaa tatatttgat ttattgtagg 6360
tttttaatgg tgtttgtaga gtattttttt gtattgattt agttttagta aagggtagag 6420
gttttgttgt gttttttgtt gggtttagaa ttgtttaggt gtttaagaaa gttttttagg 6480
ttgggtttag tggtttatat ttgtattttt agtattttgg ggaggtcgag atgggaggat 6540
cgtttgagtt taggagtttt agattagttt gggtaataaa ataagttttt tatttttata 6600
aaagaataaa aattagtagt tgggtatggt ggtttatgtt tgtaatttta gtattttggg 6660
aggttaaggt aggtaaatta tttgaggtta ggagtttaag attagtttgg ttaatatggt 6720

gaaattttat ttttattaaa aatataaaaa ttaggtgggg tatagtggtt taagtttgta 6780
attttagtat tttgggaggt taaggtgggc ggattacgag gttagaagtt cgagattagt 6840
ttggttagta tggtgaaatt ttatttttat taaaaatata aaatattagt cgggtatggt 6900
ggtaggtgtt tgtgatttta gttttttggg aggttgaggt agaagaattg ttagaatttt 6960
ggaggtagag gttgtagtga gtcgagaata cgttattgta ttttagtttg ggtgatagag 7020
cgagatttta ttttaaaaaa tacgaaaata aaaattagtc gggtggtggc gggtgtttgt 7080
aattttagtt attggggagg ttgaggtagg agaattgttt gaatttggga ggtgggggtt 7140
gtagtgagtt aagattgtat tattgtattt tagtttgggt aatagagtga gattttattt 7200
taaaaaagaa aaaaataata attaaaatgt taaaattagg agtagaatta tagaatgttg 7260
gaaagtgagg tttaagaagg gggttgtgtt taagtttatg tatgggaaat ttgattggga 7320
tatcgagttt atatagagta ggattttagt tttttttatt agagtggggc gtgattatag 7380
gaatagtcgt ttttagttag tttgttatat gatatttttt taatgtttta gg 7432

<210> 136
<211> 7432
<212> DNA
<213> Artificial Sequence

<220>
<223> chemically treated genomic DNA (Homo sapiens)

<400> 136
   tttggaatat tgagggggtg ttatgtggta ggttgattgg aggcggttgt ttttgtggtt 60
   acgttttatt ttggtggggg ggattgagat tttgttttgt gtgagttcgg tgttttagtt 120
   aagtttttta tgtatggatt tggatatagt tttttttttg ggttttattt tttaatattt 180
   tgtgatttta tttttgattt taatatttta attattattt tttttttttt tgagatggaa 240
   ttttattttg ttgtttaggt tggagtgtag tggtgtaatt ttggtttatt gtaattttta 300
   ttttttaggt ttaagtaatt tttttgtttt agttttttta gtagttggga ttataggtat 360
   tcgttattat tcggttgatt tttgttttcg tattttttga gatggagttt cgttttgtta 420
   tttaggttgg agtgtagtgg cgtgttttcg gtttattgta atttttgttt ttagggtttt 480
   agtaattttt ttgttttagt tttttaagga gttggaatta taagtatttg ttattatgtt 540
   cggttaatat tttgtatttt tagtagagat ggggttttat tatgttggtt aggttggttt 600
   cgaatttttg atttcgtgat tcgtttattt tggtttttta aagtgttagg attataggtt 660
   tgagttattg tgttttattt gatttttgta tttttagtag agatggggtt ttattatgtt 720
   ggttaggttg gttttgaatt tttaatttta agtgatttgt ttgttttggt tttttaaagt 780
   gttggaatta taggtatgag ttattatgtt tagttgttaa tttttatttt tttgtagaga 840
   tgggagattt gttttgttgt ttaggttggt ttggaatttt tgggtttaag cgattttttt 900
   atttcggttt ttttagggtg ttgggagtat aggtgtgagt tattgagttt agtttagaag 960
   gtttttttga gtatttaaat agttttgggt ttagtaggga atatagtaaa gtttttgttt 1020
   tttgttaagg ttgagttagt gtagggaggt gttttgtaga tattattaag gatttatagt 1080
   agattaaatg tgttatttta gggttttgtt ttaagttaga ttttatgttt ttgtattttt 1140
   ttattgagtt tttatttgtt ttatgagaga ggtatttttg gtgacgaaga atatgtatag 1200
   ggtataatat tattatttat acgtaggaga tgaggaagta cggtagaaag agggtgagat 1260
   ttgattttcg gagaaagaat ataagtaaat ggtttataat tttttttttt tttttaagat 1320
   aaggttttgt tttttcgttt aggttggagt gcgatggcgt aattttggtt tatcgtaatt 1380
   tttgtttttc gggttcgagc gatttttttg ttttagtttt ttgagtaatt ggaaatataa 1440
   gtatgtatta ttatgtttgt ttaatttttg tatttttagt agagacgggg ttttattatg 1500
   ttggttaggt tggtttttaa tttttgatat taagtgattc gtttgttttg gttttttaaa 1560
   gcgtttggat tataagtgta agttattacg tttagtttat agtttttttt tagtttttta 1620
   aattttttta tttttttttt tcgttaagat tgagttttgt tttgttattt aggttagagt 1680
   atagtggtac gatttgggtt tattgtaatt ttcgtttttt gggtttaagt aatttttttg 1740
   ttttagtttt tggaatagtt gggattatag gtatatgtta ttatattcgg ttaatttttg 1800
   tatttttagt atagatgcgg ttttattatg ttggtaaggt tggtgtcgaa tttttgattt 1860
   taagtgattt attcgtttcg gttttttaga gtgttgggat tataggtatg agttattgta 1920
   tttatttttt tttttttttt ttttttagaa atggggtttt tttgttattt aggttggaat 1980
   acggtggtaa gattatagtt tattgtattt ttaaattttt gggtttaagg gatttttttt 2040
   attttagtta ttttaatttt aagtagaagt ttaaatagtt tatagtttta aattttgtga 2100
   tttgtggagt gtggtggtgt atttgtagtt ttagttattg gggaggttga ggtaggagga 2160
   ttgtttgagt ttaggagttt gagtttagtt tgggttatat agcgagattt tatttttttt 2220
   tttttttttt ttatatatat atatatatat atatatatat atatatatat atatagttat 2280
   ggttttaata cgggttttta atattgtgtt ttttaaaatt tttgttttat cgatttttgt 2340
   tttttttttg ttgacgtttt tttgttttcg tattttgagt ttttttttta ttagattttg 2400
   gtaggttgtt tttattagtt tcggacgggt atagagagga tttatagtgg ttgagattta 2460
   ttttcgtttt atagttataa tttttatttt ttgtttaggt taaagttttg gttaatagaa 2520
   ttgcggaggg ggtgggtggg gatagaaagg gtaatgacgt tttttaaggt tacgttatag 2580
   ggaatttttt tttaagtgat agggaatttt tattttgaga attcgttttc gatagaggtt 2640
   gggttggggg ttgtggaggg gttggaggtg ttgggtgttt attgggaatg gagttagagt 2700
   ttgtaggaga taagggcgaa gtttttagtg agggttatta tggggatttg ttgattttat 2760
   ttacgttttt gtttttattt ttttgaaatt gatacgtttt gaagtttttt atttttcgta 2820
   agtttataat agggtgttat tttttgatat ttttttcgat ttttttattt tttttttttt 2880
   attaggtgtt atttttttta tttattgatt tttgggaggt agtggggtgg gttttttttt 2940
   ggttatattg gggaaggatt tagataggga tataggtgag attttattta ttggagaagt 3000
   atgaattttt tttttttttt ttgttttatt tttaagagat ttcgttgtgt tgtttaggtt 3060
   ggtttcgaat ttttgggttt aagttattat ttttatttag ttttttgagt agttgggatt 3120
   ataggtattc gttataatta tatttggttt ttatgaatat ttttaatttg gtttttgttt 3180
   ttatagataa gtttggggta taggttgtgg gtttttttta agaggttgtt tttggattcg 3240
   aggaattagg ttttggtgag cggatttagg ttttttaggt ggtcgttata gtaggtttgt 3300
   atgaagtttc ggaaggtgtt cgggttttta taggagagag gatattatag tggaggtttt 3360
   agtttttttt aggtgttttt tgttttattt attcgttttt ttttgtgatt taggagtttt 3420
   tgtttttagt tttaattttt tatttttttt agatttaggg gttatagttt tatttttatt 3480
   tttttttttt ttttgagatt taggggttta ggttttagtt tttttttttt tagatttagg 3540
   agtttttttt tattatttag tttagtatat ttttattaga attattgtta ttcgtttttg 3600
   gttttgttta ttattgtttt tagtagtttt tttattttgt tttttattag gttttagcga 3660
   tttattttta gttttggtgg ggggtttagg gttttttttt gggtttttgg ttggtatgtt 3720
   aggtggaatt agagggtaat ttgggtaggt tgggatttta gaattttttt tttttttttt 3780
   ttttatgttt attttgtgtc gttttcggtg gtttaggggt agttttttgt tagttttttt 3840
   gttttttgtt tttattttta tgtgtttttt gatcgttaat ttttttttaa ttattagtta 3900
   tttttttttt tttttttttt tttttttttt ttatgagata gagttagttt tttgttttgt 3960
   tgtttagggt ggagtgtagt gttatgattt cgttttattg taatttttat tttttggatt 4020
   taggtaattt ttttgtttta gtttttcgag tagtagggat tataggtatt tgttattatg 4080
   ttcggttaat ttttgtattt ttagaagaga tggggttttg ttatgttggg gtttcgaatt 4140
   tttgatttta agtgatttat ttatttcggg tttttaaaat gttgggatta tagatgtgag 4200
   ttatttcgtt cggttgggtt atttttttat tttgtgttag tagttttttt tgttattttg 4260
   tttgtttgtg gttttttttt aatggttttt ttaattgtta ttttttgatg ttttttttta 4320
   ttatttgatt ttgttgttta ggaattattt tttttttttt tcgttttttt gtgattttta 4380
   ttttgtttga gttatttttg tggatagtgg agggagagta ggaggtgatt ttttgttttt 4440
   aggggtagat attggtttag ttttgttgtt agtttttttt ttcgggttag attttttatt 4500
   tacgtatttg gataaagttt cgtttataga gattagtcgt tacgcggggg ttatatgtag 4560
   ttagttgtgt gttttggtcg gtgatttatt ttgttttttt aggattgttt ttttttttgt 4620
   agaacggggt aataatattt attttagggt tatcgtgaga attaagagtt attatatacg 4680
   atgtatatgg aatggtgttt tgtatattgt ttatatacgt gttaaatttt tttttttttt 4740
   tagacggagt ttcgttttgt tatttaggtt ggaatgtagt ggcgcgattt cggtttattg 4800
   taatttttgt ttttcgggtt taagtgattt ttcggtttta gtttttcgag tagttgggat 4860
   tataggcgta tgttattatg tatggttaat tttttgtatt tttagtagag atgggttttt 4920
   atttatttgt tagttaggat gattttaaat tttttgattt tgcgatttat tcgtttcggt 4980
   tttcgaaagt gttgggatta taggtatgag ttatcgtgtt tggttttgta ttaaaaattt 5040
   aaatttttat tggataatgt tgatttagaa tatgatttta ttttaatata aagtatatgg 5100
   tcgggcgtgg tggtttatgt ttgtaatttt agtattttgg gaggttaagg cgggcggatt 5160
   atttgaggtt aggattttaa gattagtttg ggtaatatgg taaaattttg tttttattaa 5220
   aaatataaaa aaattagtta ggtattggtt aggcgtagtg gtttacgttt ataattttag 5280
   tattttggaa ggtcgaggta ggcggattac gaggttagga tatggagatt attttggtta 5340
   atacggcgaa atttcgtttt tagtaaaaat ataaaaaatt agttaggcgt ggtggtgggt 5400
   ttttgtagtt ttagttattc gggaggttga ggtaggagaa tggcgtgaat tcgggaggcg 5460
   gacgttgtag tgagttaaga ttacgttatt gtattttagt ttgggtaata gagcgagatt 5520
   tcgttttaaa aaaaaaaaat agttaggtat ggtggcgtgt gtttgtagtt ttaaatattt 5580
   tgaagattga ggtaggagaa ttttttgaaa cgggtaggcg gatgatgtag tgagttaaga 5640
   tcgtattatt gttattattg tattttagtt tgggtgatag agtgagattt cgttttaaaa 5700
   aaaaaaaaaa aaatgtatat gtaaaatata gacgtatata ttagtatata tttatttttt 5760
   tatatatgtt tatatattta aatattttgt atatatatat tttatagaga gataggtgta 5820
   tataaatata tatatgtata tagatatgta tttttttatt acgattgtat atttttattt 5880
   ttacgtacgt acgtgtatat atatagatga ttttagtttg tattaatgtg atatttatat 5940
   tttattttta gttatcgata tatatatttt tcgatacgta tatgtatacg tacgttatga 6000
   aacgtatata tttcgtattt atgttttacg tggttataga gttataggat atatagttat 6060
   atttatattt attaggtgtg ggttttatat ttttttattt attttttttt atttttaatg 6120
   taggttagga ttagtacgta gaggtatagg gtaggtaggg tttggagttt gtttttgagg 6180
   agggatattt ttgggggttt cgtgtttttg tttttttgtg tttaattttg cgggtcgggg 6240
   gaggggaggt tgtatagagt agggtttttt ttcggttatt ttttcgtttg gtagagattt 6300
   tagagagtag ggtttaggtg atagtggtaa ttagaggtta tgtttttgtg ggtttttttt 6360
   ttttttattt ttttatatag tgttttgaga gaagatagag gtttttgagt tttttgtatt 6420
   gaggtaggtt taaattttgt ttttattttt tatgatagtt ttatttgttg gttttagttt 6480
   ttttttttat aaagtgggag gtatagtata tattttagta tattatttga ataaatataa 6540
   agaaaggcgt agtggtttac gtttgtaatt ttagtatttt gggaggtcga ggtgggtaga 6600
   ttatatgagg ttaggagttt gagattaatt tggttaatat ggtgaaaaat tttgttttta 6660
   ttaaaaatat aaaaattagt tagaagtagt gatgcgtgtt tgtaatttta gttatttggg 6720
   aggttgaggt aggagaatgg tttgaattcg ggaagtgaag gttgtagtga gtcgcgatta 6780
   tattattgta ttttagtttg ggtgatagag taagatttgg ttttgaaata aaataaaata 6840
   aaaaatgaag aaagttttta tcgtggagat tggtaatata ggtgtttaaa gattattgtt 6900
   gttggttagg cgcggtggtt tatgtttgta attttaatat tttgggaggt cgaggtaggc 6960
   ggattacgag gttaggagat tgagattatt ttggttaata cggtgaaatt ttatttttat 7020
   taaaaatatg aaaaattagt taggcgtggt ggtaggcgtt tgtagtttta gttattcggg 7080
   aggttgaggt aggagaatgg cgtgaatttg ggaggtggag tttgtattga gtcgagatcg 7140
   cgttattgta gtttagtttg ggcgaaagag taagatttcg ttttagaaaa aaaaaaaaga 7200
   ttattgttgt tattattatt ttgtgtttaa ttttttttag ttattgggtt gtagggttag 7260
   gtttgtggta gggttttaat agggtttaag ggttgatgag aggaaatagt aatatagttt 7320
   tatgttttta gtttttgttt ttttttaggt gggttcgagt tcgattattt ttaagtattt 7380
   tttgagtttt taattttaat tttttttttt ttgggtgatt ttggttttag at 7432

<210> 137
<211> 12423
<212> DNA
<213> Artificial Sequence

<220>
<223> chemically treated genomic DNA (Homo sapiens)

<400> 137

tttatttgag ttttttttgt tgtagagaaa gtttttttgt gattttattt ttttttttaa 60
atgttgggaa atgtttttga gttttgtttt tgatatatat attttttttg ggttgaggga 120
tggggattgg gatatatgtt ttgggaaggg taggtgtaga gtgatggggt tgacggggtg 180
ggggaagtag ggggaggagt tgtttttttg aggttttggg gaatcggttg tgttaggtat 240
ttttagtttt ttagttaagt aatatagaga gttaaaagag gttattagag ataagaaggt 300
ttttatgggg ttattgtttg tgggggatgt tagtagtggg gaggagtgaa gtaggaagga 360
atcgggagat tggatggaat tgtttttttt atacggttat atatagttat atatggttat 420
tgggattaga gttagttttg tttgggtagt taggttttta gtgtaagtta ttagtttatt 480
tggaggggat tagtgtttcg gttaataagg ttagttggtt tttttttagt tggagtttat 540
ttaattttgt taggaagtta gagagttgga taagtggagt ttagtttggg gaggtggaag 600
agagggagga tggagtatgg tgaagtatag gtggtttttt tggtagtttt agttttggtt 660
ttggaatagt ttgggtagtg tgttaatttt ttttgttatt gtcgttttat tgatttttat 720
gaatgagttg cgaggtagtt atttttagtt ttttatggat aaatattcga ggtttagaga 780
gggtaagaga tttgtttgcg attttttagt atttttgttt ttttttgggg ttttgagggt 840
ataataaaga tttttaaggt tttttttttt cgtaggaggt ttaggcgtag ttgtggggga 900
gggtgttttt ggtgtttttt gtttttgtag ttagtttgtt ttttattcgg tagttttttt 960
tttttttttg ggatgagatg tgtacgcgat gatgggattt tgtaaatggg ttgggaagaa 1020
ggagttattt ttttaaaggg ttagtttttt taggagtgtt cgaattttta aagggggtag 1080
ggttttatat aaattttatt tagaagtagg attataagag tttttgtagt attttttttt 1140
ttttgttggt tttttttgag tgtgtatttt gttttattag cggaatattt gcgggttagg 1200
gttattggag atagggttat atcgtttggg cggttattta agtaatttta ttattttgag 1260
tatttttttt ttttatttga aaggtggggg gttatttatt tagtttttag ggttgttaag 1320
gtgattaaat gggataatgg ttgtattatt tttgagtaaa gggtattata gatgggaaga 1380
ttattttagt ttcgtaggga gttagatggg ttgtgaggga ggggtaaagg tagggttgga 1440
gggggttgga ggggttggtt gagtagggta tatatggggg gggttttttt ttaaatttag 1500
aaaaattttt agttaggttt agtgtttttg ttgagaaatt gaggtttaaa gaaaggaatt 1560
ttttgttttt ttgttttttt ttttttgttt gaggttattt atttatttaa taattttgtg 1620
ttcgatatta gataaggttt tagtgagtaa aatgaatata gtttttttgg gaatatagag 1680
tttagatagg gagatgatta ttttattatt gtaaatataa atatataatt ttagtttggg 1740
ttaagtgttg tgaagtttta gatttgtgtt tggttcggtg ttgtatttta agtattttgg 1800
tatttaagtg gtattgaaat atttgttgga aagaaggtta ttttgaggag gtgatgttag 1860

agttgaggtt tggagggttg gagttaaata aagatggttt taggtttttt ttgttttttt 1920
tgtaggtttt gttttgtttt ttttttggt atttaggtta agggtaagta ttttcgattg 1980
gttattagtt ttaatatgtc gagtataggg attagatttt gttttgcgtg gtcggttttt 2040
tttaggtttt gaatttaggg aagtgtatgt ggattttaat ttatttagat ggtgcgtttg 2100
tgtttgtttt tttttcgggt atgttttaga atagagttta gtttagagtt taggggtggt 2160
cgggatgatg gatgagggtg tggggtttgt tgattattag gggatttgat tattgggttg 2220
ggttagtttt tttgttttta tttttttttt tgtaaagaag ggtgttgggg cgttttttatt 2280
tttcgcgtta gtttcggggt ttttattttg gcgggaaggg taggtcgatt cggtagattg 2340
cggtttttcg ggagggaaga aggtgttaga cgcgcggagt aattataaat agtttttttt 2400
ttttagaaga cggtacgggg tttaagattt aggcgtcgta tatttagaat gagagtagag 2460
attttcgtta ggaaaaaaag gtatttaggg gatttgttta ttagtatgaa atgtaaatga 2520
gttcgttcgg ttttatttat ataattttgt gtatggattc ggcgaaaggg taattaggga 2580
gacgacggcg tagtagttat tttgttattt tttttatttt ttttttttat cggtcggggc 2640
gggaattgag acgattttaa ttttttgcgg tggcgggagg tgcgcggggg ttgcgtgggt 2700
ggtgtagttt taggagagtg aataacgttt aggggtgatg gttttagtaa agtgaggggt 2760
ggtgatggag gttattcgat ttatttcgtc gttttttcgt agtggcgtaa gcgttttaaa 2820
attttcggag agggaattga ttgatttatt aggtttcgtc gtgtttattt ttcgtagatg 2880
ttggggaagt gtttttcggc gtttaatttt cgttgttttt ttttttatcg gcgtttagta 2940
tattcgcggc gtttcgtttt cgggtattta tagttttttg gagtggcgtc ggcggtagga 3000
gagagttaga gtcgattgag ttttagaatt cggggaggta ggcggggtaa gggtcggggt 3060
ttgcgcgtgc gtgtgtacgc gtggaaagta taaatttacg agggttcgtt gtcggtaggg 3120
ttaaggtgag tgagtgaagg cggtagattc gcgaagtttt agtttcgcgg atattttttt 3180
tttaagttat atagggattt aagagaaatt gaggtacgaa gttggggggt ggcggtggtg 3240
gggatttgaa aggtcgagtc gataaagttt agttttgtgt tttcgttttt ttttatatat 3300
tcgagatttt tataggtttt tttttagggg ttttcgtttt gtttcgtagt tttttttggg 3360
gattcgggtt ttgcggttta gcgcgatttg tttggggtta cgtgtttaag tacgaagttt 3420
ttgcgtggag tttacgtttt ttaaaaggtt tttaggggta aagggatgaa gtttaggagc 3480
gttaatgtga ggttaggttt agcgggttag ggttacgagg ttagggtcga agttttagaa 3540
ttgattcggg agtatgatta ataagtttaa gagatttgtg gaggttacgt ttagattatg 3600
tttattttag tttttttcga gtttcgttcg tttttttaag attgggtttt tttagtttga 3660
agtttatttt ttaggatatt tagtttcgtt tttttttttt tttagtttcg tagttaagtt 3720
taattttagg gtgggagatt aattaggtgg agatttggga tttcgttttg ggattttttg 3780
gtgataattt ttattttcgt gtcgtttgtt tttttttttt tttaggttcg tttagagttg 3840
agtttcgttt ttcggttgtt gtttaaatta ggggtcgtgg ataaaggatg tttggggttt 3900
gcggttttac gttaggattt cgcgtcgaat atttgatttt ttcgggtttt ttttaaggga 3960
gttttaaaga ttttaatggt taataggaaa gtgggttcgg tttgggtagt agtttgattg 4020
gttttagttt tcgggagcgg atttaggggt aaggggaggg gagaggggcg gttttgggtt 4080
ttggggtggg aatcggattt tagttgtggt tttttttttg cgttttcgtt cgtattgtta 4140
cggcggacgg ttaatgggcg cgcggttcgg ggtcggcggc gttcggcgat tggttgcggg 4200
gttgtttggg ggcggggtcg aggtttgaag ttgaagtgag ggatttagtt gtggtgtgcg 4260
cggggttttt cgtcgtcgtt ttcgttcgtt cgtttcgcgt ttcggtcgga ggaggaggtt 4320
gtggcgtcgg cgatagttac ggtagcggta gttatcgcgg cggttgcggc ggcggtattt 4380
tcgtttttat tttcgttcgg gattgttttt tattgttttt tcgttttttt cggatagtga 4440
gttcgcggcg gggcggggga aggagtcgtt tttatttttt ttaagtttat ttttaaagag 4500
attttttttt ttttttttcg tcgtttggcg cggagtcggg acgatgttga ttttttagat 4560
tcggttttag ttgcgtcgcg ggaagagggg gcgttttttt tcggattttc gtttttcgtc 4620
gttgtttttt ttttcgttcg ttttttcggg gcggtttcgt cgaaggtagc gtcgaattcg 4680
gtaatcggag tttgggcgcg aagcgaagaa gtcggaataa agtgaggggg agtcggtcgg 4740
ttggttcggg aagttttagg ggcgtagggg aagcgggatt cgcgtcgggc ggggtttttt 4800
tgcgtttcgg cgtttcgcgg gtagtatgtt tttgcgggta gggggagttg ggttgaattg 4860
gttttttcgg gggtttagtt tgcgttttag agtttattag atgtgttttc gtcggggttt 4920
tcgggttgcg tgaggatatt ttttttgagg ggcgtcgttt gttttttttc ggatcgttcg 4980
gggtttcggt tggttagagg atggacgagg aggaggatgg agcgggcgtc gaggagtcgg 5040
gatagtttcg gagttttatg cggtttaacg atttgtcggg ggtcgggggc ggtcggggtc 5100
ggggttagta gaaaaggatt cgggtagcgc ggatttcgag gcggaggggt tgtcgtattc 5160
ggcgttggtt tcggtggttt ttttttattt gagttaggat agtcgttcgc ggagttggtg 5220
ttttcgtacg gtttgtaatt tatatttttc ggggtacgac ggttaggcgc ggggttagaa 5280
gggggacggg tcgtatcgtc gggggtcggg ggggaagaag atttatcgtt aggtgagtcg 5340
aagtgagttc ggagggtagg cggatggggg gggggttgtt agggagggga gggggtatta 5400
gagtgggagc ggagacgcga gtaggtttcg tcggtaattc gggtttattt tatttgcgta 5460
tatatatttt agtttttttg ggttgggggg gtggggattt aggttaggag aagagagttg 5520
tgtttcgttg gttcgtagtt ggacgttttt tagatgtggt taggggaggg tcgttatttt 5580
ttagatgtgg gaagtttcgg gagtttggga gttgtatttt gttcgcgtcg gttagcgagt 5640

tgggtttggt tttcgagttt ggtggggggt gggtgggggc ggtggggagg aagttgcggg 5700
gacggaggag gggggatcgt aattttttgg gttttttttt ttttttcgtt ttaaagtttg 5760
cggcggattt tagggtttga ttttttaatt tggtttttga ggttgaggtt tttattgatt 5820
tagaaggaga tgttgggggg cggggattag gttttggttt attttatttt ttatttcgcg 5880
ggttggaggt ataataagga gatttcggcg gcggttgatg ttaggggcgt agaatgagaa 5940
taagatgtgg tggaggggag ttgtttgttt tcggagttgg gagtggagtt ttttttcgtt 6000
agagtttagt gtcgcgggtg ttttttattc gatttttatt cgatgtaggg tgtttaagat 6060
tgggttgggg agttttatgt tggatttttt ttgtggaggg aaagattgtg ttaagagttt 6120
ttttagtaag tgggggatgg ggttaagttg gtttaggtta tttttttttt tttataaata 6180
aagagtgttt ttgtagatta ggtatggggg attgtaggtt cgcgaaagtt ttgaggggat 6240
agtaatattg ttagtgtgtt tattaaggag gattcgggtt tattatttta gcgtagattt 6300
cgtagatagg tttggggaat gtaagtttta tattttaggg agatggattt agtattgttt 6360
tttgtttgtt taggttggag tggtagggga gattttagtt tagttagatt ttagtttaaa 6420
gcgttatttg atagacgttt ggttttaagt tttttggcgg gttttgttat cggggaaggg 6480
aaaggtggag gggaggtgtt gttgttattt taggtttgga tagttttttt gggttttttt 6540
tttttagttt agaggttttt atagatttag ggtgggagat tttaggtttt ttttgtagtt 6600
tttgtgagtt tagtagggat tttgttttgg ggaagggggg tggaaggttg gtgtcgtttt 6660
tattttttgt agttttgttg tttttagtgc gtaaagtagg gatgagtttg tggttttttt 6720
atttttattt tttatatttt gtgggttttt aggaagtttt ttttattata tttttttttt 6780
atttattgtt tttttttttt ttttttttga gttgtagaaa gagaggtgta ggttttagag 6840
gtttgtgttt ttgttgaata ggtataattt tgaggtttga gtttttagtt ttaggttttt 6900
tttttttagg tagggatgga gatggagaat tgatatggtt ggaaaggatg ggtttcgagt 6960
aatttttttt tttgttttta attatttatt ttttttacga atgggaaaaa gtattaattt 7020
ttatgttttt ttttttaagg gtttaggggg aggtagagag agtttttttt tttttttttt 7080
ttgaaaatag atttttaggt tagtattttt tttcgtttgt tttattttta ggtgttagtg 7140
gagggtttgg aggtagattt ttttattgag tggtttggac gggggtaagt gttgaggttt 7200
ggtatggggg aggagaggtg ggaggatagt atttattttg ggagttgggg agtagttttt 7260
tttaggggag tggtagttgg gtgtgtggta ttagtattat taggtttgga gaagtttggg 7320
ttggttgagg atgggttgag aataattttt tggtagggga ttgtttaagt tgtgtttttt 7380
attattgtta ttttatttaa gtattaagag atatttgtat ttttgttgtt agaggggttt 7440
tgaattgttt ttagggtttt tttttttttt tttgtggtat ttgtgatttt ttataggttt 7500
taggagggtt tttgtacgtt tttgagtcgg agggatataa ggatattttt ttttattttt 7560
aattaattga agttagttag gtttttttg gtatatttg tttttggtt ttttttttt 7620
aagggattag gttttttaga gaggagattt agatttaggt aggaagtgtg tgtgtgtgtg 7680
tgtgtgtgtg tgtgtgtgtg tgtgtgtgtg tgtgttgtaa gggaggatag taaaaggttt 7740
agaagtgtaa gttttggtgt ggagagtagg ggtattggta gatagttatt tttgttttag 7800
attagagggg agtatgttgg tttttttagt atgtgttttt gtaagtgtga gggagcgcgt 7860
ttttttttcg gggaggagtt ttttcgtatt ttttttttgg ttgttgtgtt atatttggaa 7920
gtttgtgtag gagttttttt aagtttagag tttagtatag ttttttttta tttggaaatt 7980
ataggttggt atttgggatg tcgagagagg gggagtggga ggggggttag agggggaaga 8040
ggggagtttg ttaggagtcg ggggaggaga ggggggggag gtttgaacgg taggagtttt 8100
aatggttttt agaagtgagt gtgagtgagt gattgtgcgt tattttgtgt gtttgagtgt 8160
gtttttttag aggggtgggg gtataggagt ttttggagtt ttttttgtta tattgtttta 8220
atgttttaag tttgtttatt ttttttattt agggagaatt ttttattttt gtttttagag 8280
gaaatggtgg ggggagtgtt ttttttgttt ttttattaat tttttttttt aatttaattt 8340
ttaaatttta agtttattag aggagatttt ggggagaagg gttggggttg gagaattgaa 8400
tgggattgag ggggggttag attatttttt ggtatgtttt ttgttttagg tttatttttt 8460
ttttgggtta tatatatatt tttttttatt cgttttgttt tatcgttttg ttgggggatt 8520
ttttattatt cgtagtgtta gttggagtta tttttgggat tatttttatt atatagtttt 8580
ttttattttc gtttgttttc gtttagtatt tatttcgttt tgtttgtttt agttaggatg 8640
gcgatagata taattgttta tacgtagata tgcgtacgcg cgcgtatata tatacgtata 8700
tatatatata gagttataga cgtttgaatt agatttttgg tttatttttt tttttaaaga 8760
ttttagcggg tcgttttttt tgtgtttttg tgttttatgt ttttgttttt gggtggagtc 8820
ggttgttttt ttgtgagttt tagtttttaa tttagttgga agtttttttt tttttatatt 8880
tatgtattga agcgtggtga ggggggaatt atagggaaat aggtttgtgg ggtgaggggg 8940
gggggttttg ttgggggaat ttttttttga gttttggagt agggcggagt ttttatattt 9000
tatgttattt taggggttgt cgaattcgat agatattagg agtttttttg tgggatgggt 9060
gaggtaagga tttatttttt tattttttta tttattagaa ggttaggatt tttgggttta 9120
ggtttatttt ttttttaacg ttttttagtt ttatattttt aggtttttag gtgggaatgg 9180
ggtcgttgga gggtttgttt gtttttttag tttatgttta gagtttttgt gtttttagag 9240
gtgttttagt tatttttttt ttttttttat tttttttatt tttgcggttg ttaggttaag 9300
taagggtttt atttagataa agttttatat agaggattta tttgtggaga taatatgttt 9360
tagttttttt ttttaggagg gaggaaagga ggagggatgg aagtggggtt tttttttttg 9420

tcgttttttt tttttttgtt tttgatggtg ggttgtgggg agaagaaaga tttttttttt 9480
ttttattttt tttttgggat ttattttagt tcggatagtt attatagtaa tagtagatat 9540
gtgattgtat aggtttttta tagttattat ggtaattagt gtttttcgtt tttttggtta 9600
ttatggggat ttgttatatg gttttttttt aatttgataa tttgtgggtt tttttgattt 9660
tttttttttg tataaggata agataatagt tttagttagg ggagattatg tgagaggggt 9720
ttttttaatt attatgataa taaatttata ttatttggtg atatattttt ttgtcgttat 9780
ggtaatagaa gaatattata tagtgatata ttgttatgtt tttaaggaaa taagttttat 9840
tttgagaagg gtgaaagatt ttttttttta ttaattattt ttttatttag agtgttttaa 9900
attgtcgtgt gttttggttt tttttttgtt cgtttatgtt tacgtgtcgt ggaaggagag 9960
ttgtataggg tgggtaggat gttatttgga agtaggttga attgttaagg gatttttaag 10020
aaggggttga tatgattaag agttgtgtat gaggttattc gggggtaggt tttgtttgta 10080
agaggttttt agtttgagtt tgggtaaggt ggggatagaa agaggggtaa tttttttgtg 10140
aggttatttt tgtatttttt tttagttata agtagtttat ttttgttaag ggaaataagg 10200
taggaagatt tgaatgggga tatatatttg gtttttaggt agggagattt attgattggt 10260
tgattcgttt atttatttat ttagtttttt taggtattga ttatttgttt cgtgttaggt 10320
attatagtaa gtttcgtagg ggatttaaag acgtattaga atgtttttgt ttgttaggag 10380
ttttagttta gtaggggagg tcgataagtg tatagattat taatacggtg gaattttgtt 10440
attagagggg tttaagtaga tgttttggga gttttttttt tttagagatt tattgttttt 10500
atatagagat atttatttat tggggttttt tttagatttt tttttttttg tagaggagag 10560
gaggggattt gtatggggaa agtagattag tttttgaggg taattgtgtt tttttttttt 10620
taaatgatgg tttttttttt gttttttttt ttttagggtg atttttggtt gaattttatt 10680
ttggggagag atttattgta tttcggtttt atttagaaaa ataattttat ggaaatagag 10740
tattttttat tttttttttg ttatttgttt atttttaaaa atttagaatt ttttttttaa 10800
atttttttat ttttgtgagg gggtgggggt tagggaaatt gtagtgtatt aggattttcg 10860
gggtattacg gataggtggc gagtaggtat gtgagtttag gggagaaggg tttgggtggt 10920
ggagaagaga taggggaggg ttgttagggg tttgagggat ttatgggtta tgggggaggg 10980
tatgtggttt gggagttaga atagagatgg gttatggttg ggattagttg ggaggaaggt 11040
ttataggttt ttgaatattt ttgttggttt ttttatgttt ggttaaagtt tttttatttt 11100
agaagtaaga ttttatttgt tttgggtttt atagagtttg gtttggtggg ttatgttagg 11160
agttgttata atattttttt tattttattt ttttaggggg tttttggttt tttttttttta 11220
attttttgtt ttttgatttt ttttagtttt tttatttttt tttatttttt ttatattttt 11280
tttaaagaaa ataggatatt tggttattta atcgtgagtt tagtgtttgg aattttttaa 11340
ggttcggagg tcgttttgta tatttttttg ttgatgggtt ggaaatgagt tattttatag 11400
aatttattgt atggaaagat ttagggttta gaaagaattt tttttttttg tttgttgttt 11460
tggttggttt gggttggaag ttataagtag gtgtgatggc ggtaatggtt ttttggggga 11520
gggggtagat attgtttatt ttgtacggtt tgggagttat gtttagtatg gtgtttgata 11580
tataatgggt ttttaatatt taaatgtgtg aatatgttta gtggttattt gagggtgttg 11640
agaggttggg aggttggtgg tggaagaggg agtttcgggt tttggtgttg tagtaggggg 11700
gattgtagtt gtggacgttt gttttatatt tagtgttagg gcggttatga taggtagttt 11760
tgtttgtttg agtagttatg gtgtgtagag gaaattatta tatattagga gtttggaaat 11820
tatcgtttta ttttgttttt tcgggtgatt tttagtatat gatttcggta tttttgggtt 11880
ttaatttttt cgagttttgg ggaagaaaat gatttatttt agatatttta tagggttgtt 11940
gtgagtatta attgtggtat tgtgtgaaag tgtttggtaa attgtaaggt aggtataatt 12000
cgattttagg tttgtataat atatttatat atagtatttt atttgttttt tatatttatt 12060
ttgtgaggta gggagaatag gatttagatt ttgtagtaaa tttatggtgt ttggtatttt 12120
tataggtatg atcgatttat tttttaaatg gtttgtgtaa atagttaggg tatttgtggt 12180
ttgttttttt ttatagatga ggaagttggg tttagagagg tgagatgatt aaggtgggat 12240
tttttttttt tgatgaggag tttggggttg ggggttggtt tgcgtgtatg ttagggtttt 12300
ggtattatat ttgtttagtt ttagatggag ttaggaggta aacgaggagg aggtgttagg 12360
gcggggtcgg ggggtcggtt ttagttttag ttttggttag ttgttttttt gattgttagt 12420
att 12423

<210> 138
<211> 12423
<212> DNA
<213> Artificial Sequence

<220>
<223> chemically treated genomic DNA (Homo sapiens)

<400> 138

ggtattggta gttaaggaaa tagttggtta aggttggagt tgaggtcggt ttttcgattt 60

cgttttaata tttttttttc gtttattttt tggttttatt tgaggttaag taggtgtggt 120
gttagggttt tgatatatac gtagattagt ttttagtttt agatttttta ttagagaagg 180
ggagttttat tttggttatt ttattttttt ggatttagtt tttttatttg taaaggaaga 240
taagttataa atattttgat tatttgtata agttatttgg ggaataaatc gattatgttt 300
gtggaaatgt tagatattat gagtttgttg tagaatttga attttatttt ttttatttta 360
tagggtagat gtgagaagta aatgagatat tgtatgtgaa tgtgttatat aaatttaaag 420
tcggattatg tttgttttat agtttattaa gtatttttat ataatgttat agttgatgtt 480
tataatagtt ttgtgaaata tttggggtag attatttttt tttttagaat tcgaggaagt 540
tgaggtttag aaatgtcgaa gttatgtgtt gaaggttatt cgagaaggta ggatgagacg 600
gtggttttta agtttttgat atatgatagt tttttttgta tattatagtt gtttaggtag 660
gtagaattgt ttgttatgat cgttttggta ttaggtgtaa ggtagacgtt tatagttgta 720
gttttttttg ttatagtatt agagttcggg gttttttttt ttattattag ttttttaatt 780
ttttaatatt tttaaatgat tattaggtat atttatatat ttaaatattg aggatttatt 840
atgtgttagg tattatgttg ggtatagttt ttaagtcgtg tagagtggat aatgtttatt 900
ttttttttta agaggttatt gtcgttatta tatttgtttg tggtttttag tttagattag 960
ttaaaatagt aagtaggaga gaggggtttt ttttaaattt tgggtttttt tatatagtgg 1020
attttatgga gtgatttatt tttagtttat tagtaggaga atgtataaaa cggttttcgg 1080
attttagaag gttttaggta ttggatttac ggttaaatga ttaagtattt tatttttttt 1140
aaggaaaata tgagaagggt ggggaagagt aaagaggtta gaaaagatta gagagtagga 1200
gattggggaa aggagattag agattttttg gggaatgggg atgggggagg tattgtggta 1260
gtttttggta tgatttatta agttagattt tgtagggttt agagtaggtg gaattttgtt 1320
tttggaatga gaaggttttg gttaggtatg aagaggttag taggaatgtt taggaattta 1380
tggatttttt ttttagttgg ttttagttat agtttatttt tgttttgatt tttaagttat 1440
atgtttttt ttatagttta tgaatttttt aaattttga tagtttttt ttgtttttt 1500
tttattattt aggttttttt tttttagatt tatatgtttg ttcgttattt gttcgtggta 1560
tttcgagagt tttaatgtat tgtagttttt ttaattttta tttttttata agaataagag 1620
ggtttaagaa aaaagtttta ggtttttggg ggtaggtaga tgatagggag gaaataaaaa 1680
gtgttttgtt tttatggaat tgtttttttg agtagagtcg gagtgtagtg gatttttttt 1740
tagaatgggg tttagttagg ggttattttg gaaaaagggg gataggaagg gagttattat 1800
ttggaaaggg aaagatatag ttatttttaa gaattggttt gtttttttta tgtaggtttt 1860
tttttttttt ttgtagggaa agggggtttt ggaaggagtt ttagtgagtg aatgtttttg 1920
tgtggaggta gtagattttt gaggaagagg ggtttttagg atatttgttt ggattttttt 1980
aatggtagag ttttatcgtg ttagtaattt gtgtatttgt cgattttttt tgttgagttg 2040
gggtttttga taggtagggg tattttgata cgtttttaga ttttttacgg agtttgttgt 2100
agtgtttggt acggagtaga tgattaatat ttgggaagat tgaatgaatg aatgaacgaa 2160
ttaattaatt aataagtttt tttgtttaga agttagatgt gtgtttttat ttaggttttt 2220
ttgttttgtt ttttttgata gaggtgaatt gtttataatt gggggaaggt atagaagtga 2280
ttttatagag gagttgtttt tttttttgtt tttattttat ttaggtttag attggaggtt 2340
ttttataggt aaagtttgtt ttcgaataat tttatgtata gtttttgatt atgttagttt 2400
ttttttaagg gttttttggt aatttagttt gtttttaagt aatattttgt ttattttgta 2460
tagttttttt tttacgatac gtaagtataa acggataggg aagagattaa ggtatacgat 2520
aatttgggat attttgaata gaggaataat taatgggggg gggggttttt tatttttttt 2580
agagtagggt ttgttttttt gggaatatgg tagtatgtta ttgtgtgatg tttttttgtt 2640
gttataacga taggaggatg tgttattagg tggtgtggat ttgttgttat ggtaattaaa 2700
aagatttttt ttatatgatt ttttttgatt gaggttgttg ttttgttttt atgtagggga 2760
ggagaattag ggagatttat aggttattag gttgggggaa aattatgtga tagattttta 2820
tggtaattaa gggaacgggg gatattggtt gttatagtga ttatgagaga tttgtgtagt 2880
tatatgttta ttgttgttat ggtgattgtt cgggttggag tggattttaa agagggggta 2940
ggagagggga ggattttttt tttttttata atttattatt aaaagtagag aaaaaggaaa 3000
cggtagagag aggaatttta tttttatttt tttttttttt tttttttttg ggagagagaa 3060
ttagagtatg ttatttttat aaatgagttt tttgtgtggg gttttattta gatagagttt 3120
ttgtttagtt tggtagtcgt aagggtgagg aaagtaggga gaggaggggg gtggttggga 3180
tatttttagg ggtatagggg ttttgggtat gggttgagaa ggtaagtagg ttttttagcg 3240
gttttatttt tatttaggag tttggaaatg tgagattaag gaacgttgag gagaaaatgg 3300
gtttggattt aggagttttg gttttttagt aaatgaggag gtagggggat aggtttttgt 3360
tttatttatt ttatagagag gtttttggtg tttatcggat tcggtaattt ttagggtaat 3420
atgggatgta ggggtttcgt tttgttttaa agtttaaagg gggatttttt tagtagaatt 3480
tttttttttt attttataag tttatttttt tgtggttttt tttttattac gttttagtgt 3540
atgggtgtag ggggagagag gtttttagtt gaattgaaaa ttgaagttta tagaaaggta 3600
gtcggtttta tttagagata gagatatgag atatagagat ataagagaga cggttcgttg 3660
gaatttttgg aaggggaaat aggttagaaa tttgatttaa acgtttgtga ttttgtgtgt 3720
gtgtgtgcgt gtgtgtgtgc gcgcgcgtgc gtatgtttgc gtgtgagtag ttgtgtttgt 3780
cgttattttg gttgaggtag gtagggcggg gtgagtgttg ggcgggagta ggcgagggtg 3840

gggggggttg tgtggtgggg gtaattttag gaatggtttt aattggtatt acgggtgatg 3900
gggagttttt tagtagagcg gtggagtaga gcgggtggga aggaatgtgt gtgtgattta 3960
gaaggaaagt gggtttgggg tagaaagtat gttaaggggt gatttggttt ttttttaatt 4020
ttatttaatt ttttagtttt agtttttttt tttagaattt tttttggtga gtttagggtt 4080
tggaaattaa attggaggag ggaattgatg aagagatagg aagaatattt tttttattat 4140
tttttttgag gatagaaatg gagaattttt tttaggtaag agaaataggt agatttgggg 4200
tattgggata gtgtggtaag gaaggtttta gaaatttttg tgtttttatt tttttagagg 4260
gatatattta gatatataga gtgacgtata gttatttatt tatatttatt tttggaagtt 4320
attagggttt ttgtcgttta ggtttttttt tttttttttt tttcgatttt tggtaggttt 4380
tttttttttt tttttggttt tttttttatt tttttttttt cggtatttta aatgttagtt 4440
tgtgattttt aaatgagaaa aagttgtgtt gggttttgag tttggagaaa tttttatata 4500
aatttttaga tgtgatataa tagttagaga gggaatgcgg aggaattttt tttcgagagg 4560
ggagcgcgtt tttttatatt tgtaggaata tatattggaa aggttagtat gttttttttt 4620
gatttgaggt aggggtgatt gtttgttagt atttttgttt tttatattaa agtttgtatt 4680
tttgagtttt ttgttgtttt tttttgtaat atatatatat atatatatat atatatatat 4740
atatatatat atatatattt tttatttggg tttagatttt ttttttagga aatttggttt 4800
tttaaaaagg agaggttaag aagtagaatg tattaagaaa agtttaatta gttttagtta 4860
gttgggggtg gaggggagtg tttttatgtt ttttcggttt agaggcgtat agggattttt 4920
ttgaagtttg tggggagtta tagatattat aaagagaaag agggaggttt tggaggtagt 4980
ttagagtttt tttggtagta aaaatatagg tgttttttgg tatttgggta ggatggtagt 5040
ggtgaggggt atagtttagg tagttttttg ttaaaaaatt gtttttaatt tatttttagt 5100
taatttaggt ttttttagat ttagtaatgt tgatgttata tatttaatta ttattttttt 5160
gggggaagtt gttttttaat ttttaaggtg agtgttgttt ttttattttt ttttttttat 5220
gttaggtttt aatatttgtt ttcgtttaga ttatttagtg aaggagtttg tttttagatt 5280
ttttattagt atttggagat agggtaggcg agaagagatg ttagtttaga ggtttgtttt 5340
tagggagagg ggagagaaga gtttttttta ttttttttgg gatttttgag aaggaggata 5400
tagaggttga tgtttttttt tattcgtgga aggggtaaat gattaggggt agagaggaga 5460
gttgttcgga atttattttt tttagttata ttagtttttt atttttattt ttgtttggga 5520
gaaaggggtt tggggttggg ggtttaaatt ttaagattgt gtttatttag taagaatata 5580
gatttttgag gtttgtattt ttttttttgt agtttagaga gaaaggaaga aggggtagtg 5640
gatgagaaga ggatgtgatg aggggagttt tttaaagatt tataaggtgt agaggatggg 5700
ggtggggggg ttataagttt atttttgttt tgcgtattag aggtagtaga gttgtagaga 5760
gtgagggcgg tattaatttt ttattttttt tttttaaagt agagtttttg ttaggtttat 5820
aggggttata agggagattt ggggtttttt attttgggtt tataagaatt tttgagttgg 5880
aaggaaggag tttagagaag ttgtttagat ttgagatggt agtaatattt tttttttatt 5940
tttttttttt tcggtggtag gattcgttag ggagtttggg gttaggcgtt tgttaagtgg 6000
cgttttgagt tagggtttga ttgggttggg gtttttttta ttattttagt ttgggtagat 6060
agggagtagt gttaaattta tttttttgga atgtggggtt tgtatttttt aaatttattt 6120
gcggagttta cgttggggtg gtggattcga gtttttttta gtgaatatat tgatagtatt 6180
attgtttttt taagattttc gcgggtttgt agttttttat atttggtttg taggagtatt 6240
ttttatttgt gaaaggggag gagtaatttg gattagttta attttatttt ttatttgtta 6300
aaaaagtttt tgatatagtt ttttttttta taagaaaaat ttagtataaa attttttaat 6360
ttaattttaa gtattttgta tcgaatggag atcgggtagg aggtattcgc ggtattgggt 6420
tttagcggaa aggggtttta tttttagttt cgggggtaga tagttttttt ttattatatt 6480
ttgtttttat tttgcgtttt tgatattagt cgtcgtcgga atttttttgt tgtgttttta 6540
attcgcgagg tggggggtga ggtgggttag gatttggttt tcgtttttta atattttttt 6600
ttaggttagt aaaaatttta gttttaagga ttaggttggg ggattagatt ttagaattcg 6660
tcgtaaattt tggggcgggg gaaggaggga aatttaggag attgcggttt tttttttttc 6720
gttttcgtag tttttttttt atcgttttta tttatttttt attaaattcg gaaattaaat 6780
ttagttcgtt aatcggcgcg ggtagagtat agtttttagg ttttcgaagt tttttatatt 6840
tggaggatga cgattttttt ttgattatat ttggagggcg tttagttgcg agttagcggg 6900
gtatagtttt ttttttttgg tttggatttt tattttttta atttaggaag attgaggtgt 6960
gtgtacgtag gtggggtaag ttcgggttat cgacgagatt tgttcgcgtt ttcgttttta 7020
ttttggtgtt tttttttttt tttggtagtt ttttttttat tcgtttattt ttcgggttta 7080
tttcgattta tttggcggtg ggtttttttt tttttcgatt ttcggcggtg cggttcgttt 7140
tttttttgat ttcgcgtttg gtcgtcgtgt ttcgaaggat atgggttata gatcgtgcgg 7200
agatattagt ttcgcgggcg gttgttttgg tttaagtaga agaaaattat cggggttagc 7260
gtcgggtacg gtagtttttt cgtttcggag ttcgcgttgt tcgggttttt ttttgttgat 7320
ttcggtttcg gtcgttttcg gttttcgata ggtcgttgag tcgtatgaag tttcggggtt 7380
gtttcgattt ttcggcgttc gttttatttt ttttttcgtt tattttttgg ttagtcgggg 7440
tttcgggcga ttcggagagg ggtaagcggc gttttttaga ggaggtgttt ttacgtaatt 7500
cgggggtttc ggcgggggta tatttggtgg gttttagggc gtaagttgag ttttcgggag 7560
ggttagttta gtttagtttt ttttgttcgt aggggtatgt tgttcgcggg gcgtcggggc 7620

gtagggaaat ttcgttcggc gcgagtttcg ttttttttgc gtttttgggg tttttcgggt 7680
tagtcggtcg gttttttttt attttgtttc ggttttttcg tttcgcgttt aggtttcggt 7740
tgtcggattc ggcgttattt tcggcgaagt cgtttcgaga gggcgaacga aaaggggggt 7800
agcggcggag ggcgggggtt cggggagggg cgtttttttt tttcgcggcg tagttggagt 7860
cggatttaag gggttagtat cgtttcggtt tcgcgttagg cggcggggga ggggaggagg 7920
gattttttta ggggtgggtt tggagggggt gggggcggtt ttttttttcg tttcgtcgcg 7980
ggtttattgt tcgggagggg cggggagata gtggggggta gtttcgggcg ggagtggagg 8040
cggagatgtc gtcgtcgtag tcgtcgcggt ggttgtcgtt gtcgtagttg tcgtcggcgt 8100
tatagttttt tttttcggtc gagacgcgga gcgagcgagc gaaagcggcg gcgaggagtt 8160
tcgcgtatat tatagttgga ttttttattt taattttaag tttcggtttc gtttttagat 8220
agtttcgtag ttaatcgtcg gacgtcgtcg gtttcgagtc gcgcgtttat tggtcgttcg 8280
tcgtggtagt gcgggcggga gcgtagggag agaattatag ttggaattcg atttttattt 8340
taaaatttag gatcgttttt tttttttttt tttgtttttg ggttcgtttt cgaaggttgg 8400
agttaattag attgttgttt agatcgaatt tattttttta ttggttattg gggtttttgg 8460
gatttttttg gagggagttc gaagaattag agtattcggc gcggggtttt ggcgtagggt 8520
cgtaggtttt aggtattttt tgtttacgat ttttgatttg ggtagtagtc ggaggacgga 8580
gtttagtttt gggcgggttt ggggaagaaa ggagataggc ggtacggggg tgagggttgt 8640
tattaggggg ttttagagcg aaattttagg tttttatttg gttaattttt tattttggag 8700
ttgagtttgg ttgcgggatt ggggaaggga aggagcggag ttggatgttt tgggggtggg 8760
gttttaggtt ggaggggttt agttttggag aaacgggcgg agttcgagaa aggttagggt 8820
aggtatggtt tgggcgtggt ttttataaat tttttgggtt tattggttat attttcgagt 8880
taattttgag atttcgattt tgatttcgta attttaattc gttggatttg attttatatt 8940
gacgtttttg ggttttattt ttttgttttt agggattttt tgaagggcgt ggattttacg 9000
taggggtttc gtgtttgaat acgtggtttt aggtaggtcg cgttggatcg tagagttcgg 9060
atttttagga ggggttgcgg agtagagcga ggatttttaa gaaggaattt gtgggggttt 9120
cgagtgtgtg ggagggggcg gaagtataga gttgggtttt atcgattcga tttttaaagt 9180
ttttattatc gttatttttt aatttcgtgt tttagttttt tttaagtttt tatgtggttt 9240
agggaaggag tgttcgcgga attggaattt cgcgggtttg tcgtttttat ttatttattt 9300
taattttatc ggtagcgggt tttcgtgggt ttgtgttttt tacgcgtgta tacgtacgcg 9360
tagatttcgg tttttgtttc gtttattttt tcgagttttg gggtttagtc ggttttagtt 9420
tttttttgtc gtcggcgtta ttttagaaag ttgtgggtat tcgggagcgg agcgtcgcgg 9480
gtgtgttggg cgtcggtgga ggggggaata gcgaggatta gacgtcggga agtatttttt 9540
taatatttgc gagaggtaga tacggcggaa tttagtgggt tagttagttt ttttttcgga 9600
gattttgggg cgtttgcgtt attgcggaga ggcggcggga tgggtcggat gatttttatt 9660
attatttttt attttgttga ggttattatt tttgggcgtt gtttattttt ttaaggttgt 9720
attatttacg tagttttcgc gtatttttcg ttatcgtaga gggttggggt cgttttagtt 9780
ttcgtttcgg tcgatggggg gaggggatgg gggaagtggt agagtggttg ttgcgtcgtc 9840
gtttttttgg ttgttttttc gtcgaattta tgtatagagt tgtgtaaatg aggtcgggcg 9900
ggtttatttg tattttatgt taatgagtag attttttaag tgtttttttt ttttggcggg 9960
agtttttgtt tttattttga gtatgcggcg tttgagtttt gaatttcgtg tcgttttttg 10020
ggaaaggggg gttatttatg gttgtttcgc gcgtttgata tttttttttt tttcgagagg 10080
tcgtagtttg tcgggtcggt ttgttttttt cgttaggata gaggtttcgg ggttggcgcg 10140
agaagtaggg acgtttaata tttttttttt gtagagggga aaatgaggat agagaggttg 10200
gtttagttta atgattaggt tttttggtgg ttagtaggtt ttatattttt atttattatt 10260
tcggttattt ttgggttttg gattgaattt tgttttagaa tatgttcgaa aggagggtag 10320
gtataaacgt attatttaga taaattgaga tttatatgta tttttttggg tttaggattt 10380
aggggagatc ggttacgtaa gataggattt gatttttgta ttcggtatgt tggggttgat 10440
ggttagtcgg agatgtttat ttttagtttg agtgttagag aggaggtaga ggtagaattt 10500
atagggaagg taaagaagat ttgaaattat ttttatttaa ttttagtttt ttaagtttta 10560
gttttgatat tattttttta gagtggtttt ttttttagta agtattttaa tgttatttaa 10620
gtgttaaggt gtttaggata tagtatcgag ttagatataa gtttggaatt ttatagtatt 10680
tagtttaggt tgggattata tgtttgtgtt tgtagtaatg aaatgattat ttttttattt 10740
aggttttgtg tttttagagg ggttatgttt attttgttta ttagggtttt gtttagtgtc 10800
gggtataaag ttgttaggtg aatgaatgat tttaggtagg gaaagggaaa taaagggata 10860
agaaattttt ttttttgggt tttagttttt taataaagat attaagttta attaagagtt 10920
tttttgggtt tgaggaagaa ttttttttat atatattttg tttagttagt ttttttagtt 10980
ttttttagtt ttgtttttat ttttttttta tagtttattt ggttttttgc ggagttaaga 11040
taattttttt atttgtgata ttttttgttt agggatgata taattattat tttatttagt 11100
tattttaata gttttgagag ttgggtggat ggttttttat tttttagata aggaaggggg 11160
gtgtttagaa tgatgaagtt atttaagtga tcgtttagac ggtatggttt tgtttttagt 11220
gattttggtt cgtaggtgtt tcgttggtag aatagggtgt atatttaaga gggattagta 11280
aaggggaagg gatgttgtag gaatttttat aattttgttt ttgagtgaga tttatgtggg 11340
attttgtttt ttttagaaat tcgagtattt ttgggagaat taatttttta aggaggtaat 11400

tttttttttt taatttattt atagaatttt attatcgcgt gtatatttta ttttaggagg 11460
gagagaggag ttgtcgagta gaaagtagat tggttgtagg gatagaagat attaagggta 11520
ttttttttta tagttgcgtt tggatttttt gcgagaagag gaagttttag gggtttttat 11580
tgtattttta agattttaga gagaaataga agtgttgagg ggtcgtaggt aggtttttta 11640
ttttttttgg gtttcgaata tttatttata ggaggttgaa ggtaattgtt tcgtaattta 11700
tttatgaggg ttaatgggac gatagtggta agaggggttg gtatattgtt tagattgttt 11760
taaagttagg gttggggttg ttaaaaaggt tatttgtgtt ttattatgtt ttattttttt 11820
ttttttttat ttttttaggt tgggttttat ttgtttagtt ttttgatttt ttggtaaggt 11880
tgagtaggtt ttagttagga aggggttagt tgattttgtt ggtcggagta ttaatttttt 11940
ttagatgggt tggtggtttg tattgagggt ttggttgttt agatagagtt gattttggtt 12000
ttagtgatta tgtgtggtta tgtgtgatcg tgtgaaggga gtaattttat ttagtttttc 12060
ggtttttttt tgttttattt ttttttattg ttgatatttt ttataagtag tggttttatg 12120
aaggtttttt tgtttttagt agtttttttt ggttttttgt gttgtttagt tggagggttg 12180
aggatgtttg gtatagtcga tttttaaagg ttttagaggg atagtttttt tttttgtttt 12240
ttttatttcg ttagttttat tattttgtat ttgttttttt taaagtatgt gttttagttt 12300
ttatttttta atttaaggaa gatgtatgtg ttaaggatag gatttaggag tattttttaa 12360
tatttagggg aaaggatgag gttataggaa ggtttttttt gtagtaagaa ggatttagat 12420
aga 12423

<210> 139
<211> 3720
<212> DNA
<213> Artificial Sequence

<220>
<223> chemically treated genomic DNA (Homo sapiens)

<400> 139

tgtagttgtt tattaaaatt ttatatatat tttggtatat aattatattc gttggaattt 60
attttataga tatatttata tttatatagt taaggatgtt gattttaata atattggtat 120
aagtaaaaag ttggaaattg tttatatgtt tatggatagg ggataaatcg attaatttaa 180
ggtttttttt tataatggag tattatatag ttttgaaaaa gaatgataag atttttatgt 240
taggatatgg tataattttt aagttatttt aagtgaaaaa ataataataa taataatatt 300
gtagaataat gtgtatggtt ggatatatat aaattttttt gaaagaatat ttaagaaatt 360
taaatgtggt tattttagga gttaggaatg aggtaggtgg agaggaggga tttttatttt 420
ttaatttaga atatattttg ttgtgttagt tgattttttt tttattgtta ataagtatta 480
tagttttttt taatttaaat tatttttttt agtataaatt taaaaaaaaa aaatttaaaa 540
tggaaaaata tataggcgtg agtaaaagaa atgaatggta ataatttttg tggtttattg 600
agtgcgtttg gatatttatg tgtttttagt ttttttttat aggaaatgta taggtgagaa 660
attgacgtta agggggattg agtgttaagt tagttagtgg tagagggtag atttaaattt 720
aatacggttt ttttttgttg tttttcggtt tttgttttta ggtgggaagc gtatttatcg 780
gacggtcggt tcggtgaggc gtagcgtttt agattggcgt attcgcggtt ttagcgtttt 840
acgtttgggg agcgcgcgcg tacgtagcgg cgcgagtttg gcggcggcgg cgataataat 900
aacgttatag ttcgagtttt ttttttcgga gttttcggta tatattttgt gtttatgttt 960
gggtatttac gttacggcgg tagggtcggg gttttttaaa atggtagtgg ttcggggagt 1020
cggaagttcg gagttagcgt cgtcgtagtt atataagtgg gggggttgtg ggttggggga 1080
gttcggtagc gttttggaga ggcgaggagt cgtcgttcga ggtcggtgcg ggcgagcgag 1140
ggcgtcgcgg tttttcgatt ttttttttag aggtgagtgt tcgaagttag gagttcggcg 1200
tttataggtt tgtgcgttgc ggggaatttt tatcgttagt tttttcgtta ttcgcgcgtt 1260
tttaagttta gcgggcgagg tttcgggcgt tttatagtcg gcgtcgcgtt atgttttatt 1320
ttagcgagtt tttcgaattc gacgcgtttt tcgttaagtt tattgaaggt tgttgcgtcg 1380
aatttagcgt tgaattgttt cggttgtttg ttagggacgt tttcgcggtt atcggttatt 1440
ttggaggtaa ggagggcgag taggggtgtt tagacgacga cggcgtagcg cgggggcgta 1500
ttatatttga gaattaggag ggattgggat attggagtta tgagaatagg ggcgatggga 1560
agtttaggag ttttggggtg cgtattttta tttttattta tattgggtcg ttagcgtttt 1620
cgtaggaatt tgtgcggtta tcggagcgtt tttttgtttg tgtacgtgtg ttttgcgtgt 1680
gtatgttttt atgtgttttt tgggacgtat gcgggttttt gttgaattag aatgtgtaaa 1740
aggtattttg tgtatattcg tgggtattaa gagttttgta ggtaggggtt gtggatttag 1800
gtgtattttt tgatgtgttt agagttgatt tttgtttttt ttttagtagg cgattttttg 1860
agttgggttt tgaatagttg cggcgtaagt ggggatttag tcgatttttg ttttttggag 1920
gggtttgcgt ttatattttt tttcggtttt agttatagcg gtagtttttt tatttaggta 1980
gtgttcgaat attcgtacga ttcggaggta ttttttaatt ttatgtcggg cgttttaggt 2040

ttggtatttt ttttcggttt agaggtagta gcgtttagat tttcgttgga tgtttttttt 2100
tttgcggggt tcgatgtttt gttgtcgggt tcgtcggatt tttatttttc ggatttgggc 2160
gttgtttttt ttttagaggc gttttgggag gtttcgtttt gcgcgggtgt tttttcgtag 2220
tgtttgtatg agttttagtt tttttcgttc gacgttaagt tcggttttcg gcggtttttc 2280
gtttcgttag cgttggacgt tgtttttgtt tttaagggtt tttacgcgtt ttgggagttg 2340
ttttttgtgg gggttttagg gaattgtggg ttatagggag attattaggt cgtttcggag 2400
gttcgttttt tcgtaatagg gattaagatt gaggatttgt tgtttattag ttgttttgcg 2460
gaattgtcgg tcgttttagt taatagattt tattttagcg gggtttatga cgttttttcg 2520
ttggtttcgg gtgatttagg ggagggggtt gagggttttt ttgggttttt gatttttttt 2580
agtggggagg gagggagtag cggcgacggc ggagagtttt tggttagtac gtagttttag 2640
tttttttcgt tgggttttcg tagcgtcgtc gcggcggatt tttttaaatt tttggtggcg 2700
gatatttttg gaagtagtgg cgtggttgta ttattcgtgt cgtcgtcgtc gtttattttt 2760
tttttttagg ttaaggcgcg acgtaagggg cgtcgcggcg gtaaatgtag tacgcgttgt 2820
ttttgttcgc ggtcgtacgt taaggttttc gtttgttcgg tggagagttg tgtgcggagt 2880
tttgcgcgtt tcgacgagtt taatcgttat ttgcgtattt atacgggtta taaatttttt 2940
tagtgtcgta tttgttttcg taattttagt cgtagcgatt attttattag ttacgtgcgt 3000
atttatatcg gcgagaagtt ttttgtttgc gacgtgtgcg gtcgtcgttt cgcgcgtagc 3060
gatgagaaga aacggtatag taaggtgtat tttaagtaga aggcgcgcgt cgaggagcgg 3120
tttaagggtt tcggttttta ttcgttgggt ttttttttcg tttttttttg agtaagagat 3180
gggtttatgg gttggggcgt cgtcgttcgg cgcgtacgag tttcgggtcg tttttttttt 3240
cgtttttttt ttaatttttt ttcgtacgtt cgagggtcgg ttttcgtttc gtttttagtt 3300
tttttgaagc gttcgtcgta tacgttttat ttagtattag ttcgtcggat agttttcgcg 3360
gtttaggcgt tgttattttt gttagtcgcg ttttggggga agttttttga gattatttag 3420
tgaataggta ttattttggg atttaagata gttttttgta attgtatacg ttttacgttt 3480
ttttttataa tttttagaga taggttgggg taggttaagg cggtttcgcg cgggattttg 3540
tatagtagtg ttttatttag tagtcgattg gatgtaacgt tttgttttgg gttttttttt 3600
tttttgttgt tgttaatttt tgtaaagtag acgttatttt taagtagttg ataaaattgt 3660
ttatttttgt aattaaaatt attgtgttaa aagtttattg aatttgttat gtaagttttt 3720

<210> 140
<211> 3720
<212> DNA
<213> Artificial Sequence

<220>
<223> chemically treated genomic DNA (Homo sapiens)

<400> 140

aggagtttat atggtagatt tagtaagttt ttagtataat aattttaatt gtaaaaataa 60
atagttttgt taattgtttg agagtagcgt ttgttttata aaaattaata ataataaaag 120
gaaaaaaaat ttaaagtaaa acgttatatt taatcggttg ttggataaga tattgttgta 180
taaagtttcg cgcgagatcg ttttggtttg ttttagtttg tttttggggg ttatagagga 240
aggcgtgggg cgtgtgtagt tataaaagat tgttttgaat tttagggtag tgtttattta 300
ttgggtggtt ttagaagatt ttttttaaag cgcggttgat aagggtgata gcgtttggat 360
cgcgggaatt gttcggcgag ttggtgttga atagggcgtg tgcggcgggc gttttaagga 420
aattggaagc gggacggagg tcggttttcg ggcgtgcgag gaggagttgg aagaagagcg 480
gggaggggaa cggttcggaa ttcgtgcgcg tcgaacggcg gcgttttaat ttataaattt 540
attttttgtt tagagagaag cgaaggagag gtttagcgag taaaagtcga ggtttttgag 600
tcgtttttcg gcgcgcgttt tttgtttgag gtgtattttg ttgtgtcgtt tttttttatc 660
gttgcgcgcg aagcggcggt cgtatacgtc gtaagtaaaa ggtttttcgt cggtgtgggt 720
gcgtacgtgg ttggtgaggt ggtcgttgcg gttgaagttg cggaggtaga tgcggtattg 780
gaagggtttg tggttcgtgt ggatgcgtag gtggcgattg agttcgtcgg agcgcgtaaa 840
gtttcgtata taatttttta tcgggtaagc gaaggttttg gcgtgcggtc gcgggtagaa 900
gtagcgcgtg ttgtatttgt cgtcgcggcg ttttttgcgt cgcgttttgg tttgggggaa 960
aggggtgggc ggcggcggcg gtacgggtgg tgtagttacg ttattgtttt tagggatgtt 1020
cgttattaga ggtttaggga agttcgtcgc ggcggcgttg cgaaggttta gcggggaaag 1080
ttgaggttgc gtattggtta gaaatttttc gtcgtcgtcg ttattttttt tttttttatt 1140
aggaggggtt aggagtttag ggaggttttt agtttttttt tttaagttat tcggggttag 1200
cgggaaagcg ttataggttt cgttgggata gagtttgttg gttgggacgg tcggtagttt 1260
cgtagggtag ttgatggata gtaagttttt aattttggtt tttattacgg gaaaacgagt 1320
tttcggggcg gtttggtagt ttttttgtga tttatagttt tttggggttt ttatagaaag 1380
tagtttttag ggcgcgtagg gatttttgaa ggtagagata gcgtttagcg ttggcgaggc 1440

gggaggtcgt cggaggtcgg gtttgacgtc gggcggggag agttgaggtt tatataggta 1500
ttgcgagggg gtattcgcgt aaggcgaggt tttttagaac gtttttggga aaggggtagc 1560
gtttagattc ggggagtaaa ggttcggcgg attcggtagt aaggtatcgg atttcgtagg 1620
aaaaggggta tttagcgggg atttggacgt tgttgttttt ggatcgggga agggtgttag 1680
gtttaagacg ttcgatatga ggttgaagag tgttttcggg tcgtgcgggt gttcgggtat 1740
tgtttgaatg aagaagttat cgttgtagtt gaggtcggga gggggtgtgg gcgtaggttt 1800
ttttaggaag taggagtcgg ttaagttttt atttgcgtcg tagttgttta aagtttagtt 1860
taagaagtcg tttgttgagg agggagtagg aattagtttt gggtatatta aagggtgtat 1920
ttgagtttat agtttttatt tataaaattt ttggtgttta cggatatata taaagtgttt 1980
tttgtatatt ttgatttagt aggggttcgt atacgtttta aggagtatat agaaatatgt 2040
atacgtaaaa tatacgtgta taggtaaaag ggcgtttcga taatcgtata ggtttttgcg 2100
gaggcgttgg cggtttagtg tgggtgggaa tgggggtgcg tattttagga tttttaagtt 2160
ttttatcgtt tttattttta tagttttaat gttttagttt tttttggttt ttaggtatgg 2220
tgcgttttcg cgttgcgtcg tcgtcgtttg agtatttttg ttcgtttttt ttatttttag 2280
ggtagtcggt ggtcgcggga gcgtttttgg taggtagtcg gggtaattta gcgttgggtt 2340
cggcgtaata gtttttagtg gatttgacga ggagcgcgtc gggttcggaa aattcgttaa 2400
ggtggagtat ggcgcggcgt cggttgtggg gcgttcgggg tttcgttcgt tgggtttggg 2460
ggcgcgcggg tggcggggag gttggcggta ggggtttttc gtagcgtata gatttatggg 2520
cgtcgggttt ttggtttcgg gtatttattt ttgggaaagg agtcggggag tcgcggcgtt 2580
ttcgttcgtt cgtatcggtt tcgggcggcg gtttttcgtt tttttaaagc gttgtcgggt 2640
ttttttagtt tatagttttt ttatttatat agttgcggcg gcgttggttt cgggttttcg 2700
atttttcggg ttattgttat tttgggaggt ttcggttttg tcgtcgtgac gtaaatgttt 2760
aaatatggat ataggatgtg tgtcggggat ttcgaaaagg aaagttcgag ttgtgacgtt 2820
gttgttgtcg tcgtcgtcgt taggttcgcg tcgttgcgtg cgcgcgcgtt ttttaggcgt 2880
ggagcgttgg ggtcgcggat gcgttagttt ggggcgttgc gttttatcgg gtcgatcgtt 2940
cggtagatgc gttttttatt tggaggtaga ggtcgagggg tagtagggga ggatcgtgtt 3000
gggtttgaat ttgttttttg ttattaatta gtttgatatt tagttttttt taacgttagt 3060
tttttatttg tgtatttttt gtggagagaa attgagagta tataaatgtt taggcgtatt 3120
tagtaaatta tagagattat tgttatttat tttttttgtt tacgtttgtg tgttttttta 3180
ttttaagttt ttttttttta aatttgtgtt aggaaaaata atttaaatta aaaaggattg 3240
taatatttgt taatagtagg aggaaagtta attagtataa tagggtatat tttaaattga 3300
aaagtaaaag tttttttttt ttatttattt tatttttagt ttttgaggta gttatattta 3360
agttttttgg gtattttttt agaagaattt atgtatattt aattatatat attgttttgt 3420
aatattatta ttattattat ttttttattt gaaataattt agagattgtg ttatgttttg 3480
atataaagat tttgttattt ttttttaagg ttgtatagta ttttattgta agggaggatt 3540
ttgagttagt cgatttattt tttatttatg agtatgtagg tagtttttaa ttttttattt 3600
atattaatgt tgttggagtt aatatttttg attatataaa tgtgaatgtg tttgtgagat 3660
aaattttaac gagtgtaatt gtgtgttaaa gtatatgtaa aattttgata gataattgta 3720

<210> 141
<211> 8759
<212> DNA
<213> Artificial Sequence

<220>
<223> chemically treated genomic DNA (Homo sapiens)

<400> 141

tagtttggaa gagggtagtt aggggagaag ttagggttgg agttatgaga aaggataaga 60
tgagatgatg gttttaatat tgaggataga aagaatattg agatgagaaa gtagtttatt 120
ataagtattt atgtaaagga aatagtagat gtttataaat tagtagaggt aataattttg 180
aaagtttatt tataagtttt tttttttatt tttaatttag tttaaatgta attatttaaa 240
ttgtttttta tttttttttt tggattatga atgagttttt taaatgtagg gtttatagtg 300
ttttatttat tagtgaattt taagtgttta gtatagagtt tggtaaatag taaatgttta 360
ataaatattc gtttagtgta tgaattggag tgatttttta ttttgtttat aagttgaaaa 420
aaggtttatt atatatttaa atatgttgaa attatagggt atttggtaat tttttaaaat 480
taaaattttt agtttggaaa tagaatttta attttgtgaa aataaaattt atttatttat 540
ttaaaaaata tttattaaat aatgattatg ttttatatta ggttgagttt taaggattta 600
atgatgaata aaaattaata tgatttttgt ttttaggaaa tatatagttt agtgaggaaa 660
atagattgtg agaagttttt taataaatat tgggtgttat taaaatatat taaaaggtga 720
gtgggtgagg gatttgagtt agtttaggtg gtttaggaag tttttttgga tgtgttgata 780
tgtataggta ttaattagat aaatagagag aaggatgaat taatattgta ggtagaggga 840

atagaatatg taaaggtagg aaggattatg gagtcgttgg aggatttgaa taaaggttag 900
tgtaagtgga ttttagaaaa taggaggaaa ggtgtatgag atgagattag agaggtagat 960
tatgtggggt atggttaatg ttttggattt ttttattaag agtaatgggg agatagtgat 1020
aggatttaaa cggggaaata atatgattag attaaatttt ttaaaaaatt ttttatgtaa 1080
atatatattg agagttaatt attgataaag atttaaaggt aataaagtgg agagagaata 1140
gtatttttaa aaaatggtgt taaaataata ggatatttat attaaaagtt gggtatttgt 1200
ttataaaatt taatttaaaa tggattatag atttaaatgt aaaattgaaa gttatataat 1260
ttttggaaga aaatatagat gggaatttgt gtgattttga gtttgaaaat gatttattat 1320
atttgatatt ataattcgta agttaatata atttataagt gaataaagtg atgaattgga 1380
ttttattaga atttaaaatg tttgtgtttt aaaagatatt ggtatgataa tgaagataaa 1440
ttatagataa gatattgttg aattatattt ttgataaagg aattgtgttt agaatatata 1500
attttaaaat ttttataata aattataagt agtttaatta aaaaaaaaaa aagagaaaaa 1560
atttatagtt tttattaaag aaagtatata attgtaaaat aagtatatga aaaatgtttt 1620
gtatttttat ttatgggaga aataaaaatt aaatggaaaa gatattttta attagaatat 1680
taaaattaaa aagattgatt atattaagta ttggtgaagt ggaaatgtaa aatgatataa 1740
ttatttagga agatgatttg gaagtttttt ataaaagtag gtgtatattt attttgtgat 1800
ttatttattt tatggttaag tatttatttg agagaaatga aagaatatat ttatataaag 1860
atgtttatat aaatatttat agtagtttta tttgtagtag ttttaaattg aaaagaattt 1920
aaatgtttat taaaagtgaa tggataaata aagcgtggta tagtaatgta atagaatatt 1980
atttagtaat aaagaagaat gagttagtga tatatataat agtttaaatg tatattaaag 2040
gtattgtgtt tagtgaaaga tgtaagtaaa aaaaaaaaag agtatatgtt gtatagtttt 2100
attgatataa aattttggaa agtgaaaaat agtttatatt gatagaaagt agattattgg 2160
ttgtttgagg aggaggagta taggagaggt ggagggaaaa tgtataaagt ggtataataa 2220
aaatttttgg aattatagat atatttatta ttttgattga gtgatgattt tatgagtgta 2280
cgcgtgtgtt aaaaatgatt aatttatgta attttaaata tgtgtagttt attgtatata 2340
ttaattatat tttagtacgg ttattaaaaa gaaatttttt ggttgtataa tgtagaattg 2400
attttaggaa agagtggagg gaggatgatt atttatagtg ttttaggtgg aagagaacgg 2460
tgttttttgg aagtgaatta ggttggtaat aatagagatg aaataaatgg gtagatgtgt 2520
gagatattta ggaaataaaa ttcgatggtt attatttttt aaaggttagt ttattttggt 2580
tttttagagt aaagagttag ggaagatttt attaataaat tttttttgaa gttgtagagg 2640
aagtttatag tagaaattta tttttaattt gattaatttg agagaatatt tttggtttta 2700
tttgattatt aaaaaattgt aaagaatagg aggagaaaga agaagaaagt tggtataaat 2760
agtgaattta tataatatta attaataatt gttttttgtt tttaaaagta atgggaagaa 2820
aatgagattt gagttggaag attagagttt aaaatttaaa taaagtatat ggttttaata 2880
tgtttatagt agttaatttt ttttgataat gatataattg ttgatagtat tatttttaaa 2940
aataaaaata atatagtaat tttttagatt tgtagaatgt ttttttgttt ataagtttgt 3000
tttatatata ttatgttttt taaatgatat attagttttt tgagggtaat ttatattggt 3060
aatagttttt agatgtggaa attgtgaaga taatgttggt gatgtggaag taatataaat 3120
tttggagttt tttagattta ggtttgaatg ttagattgtt ttttatttag agtaatttta 3180
gagtattatt ttttatttta attttttttt aggttttttt gtgtttatgt gtttttttta 3240
tttttgttta ttgtttattt agtgattttt gtattttttt ttattgttag tgtgtagata 3300
tatagttttt ttggttttga gatttatgtt aattttattt tattattttg ttagtttatt 3360
taatttttat tgagtaatgt tagttgaaag ttgtggtggg attaaatgtt gtaatgagta 3420
tttaaatgag gttgaagtat ttacgtattt tatttatata tggtgaggta tatttaagga 3480
aggttgtagt tattaaaatt ttaggaaata attttttatt tttttaggtg aaagggtttt 3540
taggtttttg tgttttggaa ggtttattta tagttatttt ttaaatgata atgcgattga 3600
tgagtttaga gtttagttta aatagtaatg gattggaaga ttagtttagg ttttattaat 3660
gtggaatata gaataaatta tgtttttgtt ttagtttgtt tatttgtgaa atagagttta 3720
ttatatttag ttttttttgt ttttaggttt gagttatttt ttttggttaa ggtaagtaaa 3780
tgtttatgat gtttggttgt gtataagata aagttataat aaagttataa tttatttttt 3840
ttttgtagaa gattgtaaaa agtaaaagag atttaggtaa aaatttcgga atgatttttg 3900
gaatagagag tttttttaga attagaagtt aaaggaattt aaaatatagg gaggtttagg 3960
gtttttattg atataaagga aagatgtttt ttttataggt ttacgtttat attttttttt 4020
tttttttatt tttatttgta tttttatttt tatatagggt ttatgggatt ttttttataa 4080
aagagtagtt gtagtaattt atattatttt ttacgtttgg ttgtttatta agaggcgaaa 4140
agtagtttta tataggtttt atttttggat agttttagtt gtaaagttta aaatatgcga 4200
aggtaatttg gaaaagtaag cggttgtata taaagtaaac gtttatagag ttttggataa 4260
aattgagcgt ttatgtgtat atggtaagtg tttttagtgt ttgtgtgttt atttgtttgt 4320
ttgggtgatt ttgtttttga gagtttggat gagaaatgta tggttaaagg taattttaga 4380
taggaagaaa ggtagagaag agggtagaaa tgatttttga tttttggggt tgagggtttt 4440
tagagtaaat ggtataatgt tacgaggttc gatttatttt tatgacggaa tttaaggttt 4500
tagtaagtat ttgttggttt ggttatggtt tgttttttag tttgtaggag atttttttat 4560
ttttttattt gcgcgttttt attagttttg aaaagaattt ttggtagtta ggagtaggta 4620

tttttatcgt tttttttttt tttttttcgt ttttattttg ttggtttttt agattgggtt 4680
ttggaattaa atttggtgag tgttggtttt taggaaattt ggagttttgg cgtttaaatt 4740
ttggtttagg aaagtaggag ttatttagga agtaggggtt ttttagggtt agagttagtt 4800
ttttttgttt tcgtttacgt tgcgttagta tttgtttttt taaagttatt aggtaggcgt 4860
tagcgcgcgg tgaggggagg ggagaaaagg aaaggggagg ggagggaaaa ggaggtggga 4920
aggtaaggag gtcggttcgg tgggggcggg attcgattcg taaattgttg tatttgtttt 4980
ttatttttta gcgttttttt cgagatttcg gggagttagt ttgttgggag agcgggacgg 5040
ttcggagtaa gtttagaggt agaggaggcg atagagggaa aaagggtcga gttagtcgtt 5100
ttagtgttgt ataggagtcg aagggacgta ttacgttagt tttagttcgg ttttagcgat 5160
agttaacgtt ttttgtagcg cggcggtttc gaagtcgtcg ttcggagttg tttttttttt 5220
ttcggtgaag tttttaaaag ttgttaaaga ttcggaggaa gtaaggaaag tgtttggtag 5280
gattgacggt tgtttttgtt tttttttttt ttatttcgtt tttttttatt ttgttttttt 5340
tttttttttc gttttttttt tcgtagttgt tttagtcggt tatttttagt taattttttt 5400
tattattttt ttttttattc gttttttcgt tttcgtcggt ttagcgttgt tagttcgagt 5460
ttgtagagag gtaatttttt ttggttgcga gcgggcgagt tagttgtata ttgtaaagaa 5520
ggtttttagg agttaggcga ttggggagcg gttttagtat tgtagttacg attcgtttgg 5580
ttaggttgta cgcggagaga attttttgtt tttttttatt ttttttttat ttttttttgt 5640
tttttttatt tcgagtgcgg agttagagat taaaagatga aaaggtagtt aggtttttag 5700
tagttaaaaa ataaaataaa taaaaataaa aaagtcgaaa taaaagaaaa agataataat 5760
ttagttttta tttgtattta ttttagtgga tattgaattt ggaaggtgga ggattttgtt 5820
tttttttttt aagatttggg tattttttga atttattttt taagtattaa gagatagatt 5880
gtgagtttag tagggtagat tttgtttatc gtgtgttttt ttttgtacga gatttgaggg 5940
ttgttagagc gttttttgcg tggttgtttt cgtaagtttt tttttttgga gtttttcgta 6000
ggtgggtagt tagttgtagc gattatcgta ttattatagt ttgttgaatt tttttgagta 6060
agagaagggg aggcggggta agggaagtag gtggaagatt tagttaagtt taaggatgga 6120
agtgtagtta gggttgggaa gggtttattt tcggtcgtcg tttaagattt atcgaggagt 6180
tttttagaat ttgttttaga gcgtgcgcga agtgatttag aattcgggtt ttaggtattt 6240
agaggtcgcg agcgtagtat ttttcggcgt tagtttgttg ttgttgtagt agtagtagta 6300
gtagtagtag tagtagtagt agtagtagta gtagtagtag tagtagtagt agtaagagat 6360
tagttttagg tagtagtagt agtagtaggg tgaggatggt tttttttaag tttatcgtag 6420
aggttttata ggttatttgg ttttggatga ggaatagtaa tttttatagt cgtagtcggt 6480
tttggagtgt tatttcgaga gaggttgcgt tttagagttt ggagtcgtcg tggtcgttag 6540
taaggggttg tcgtagtagt tgttagtatt ttcggacgag gatgatttag ttgttttatt 6600
tacgttgttt ttgttgggtt ttattttttt cggtttaagt agttgtttcg ttgattttaa 6660
agatattttg agcgaggtta gtattatgta atttttttag taatagtagt aggaagtagt 6720
attcgaaggt agtagtagcg ggagagcgag ggaggtttcg gggcttttta ttttttttaa 6780
ggataattat ttagggggta tttcgattat ttttgataac gttaaggagt tgtgtaaggt 6840
agtgtcggtg tttatgggtt tgggtgtgga ggcgttggag tatttgagtt taggggaata 6900
gtttcggggg gattgtatgt acgttttatt tttgggagtt ttattcgttg tgcgttttat 6960
tttttgtgtt ttattggtcg aatgtaaagg ttttttgtta gacgatagcg taggtaagag 7020
tattgaagat attgttgagt attttttttt taagggaggt tatattaaag ggttagaagg 7080
cgagagttta ggttgttttg gtagcgttgt agtagggagt ttcgggatat ttgaattgtc 7140
gtttattttg tttttttata agttcggagt attggacgag gtagttgcgt attagagtcg 7200
cgattattat aattttttat tggttttggt cggatcgtcg tttttttcgt cgttttttta 7260
tttttacgtt cgtattaagt tggagaattc gttggattac ggtagcgttt gggcggttgc 7320
ggcggcgtag tgtcgttatg gggatttggc gagtttgtat ggcgcgggtg tagcgggatt 7380
cggttttggg ttatttttag tcgtcgtttt tttattttgg tatatttttt ttatagtcga 7440
agaaggttag ttgtatggat cgtgtggtgg tggtgggggt ggtggcggcg gcggcggcgg 7500
cggcggcggc ggcggcggcg gcggcggcgg cggcgaggcg ggagttgtag ttttttacgg 7560
ttatattcgg tttttttagg ggttggcggg ttaggaaagc gattttatcg tatttgatgt 7620
gtggtatttt ggcggtatgg tgagtagagt gttttatttt agttttattt gtgttaaaag 7680
cgaaatgggt ttttggatgg atagttattt cggattttac ggggatatgc ggtaagtttt 7740
tttttttaga aatgtcgttt ttcggtttag ggtagagtta ttttgtgttt tggggtattt 7800
agcggttttt atttgcgcga atatttagat tgtttttggg agagtttagt agggtaaatt 7860
tagagttttt tcgtggattt tcggtttgtt agaggtttaa tttgagtttt tttaattttt 7920
gttgcgtgtt ttgggtgttg atttttgttt ttttagattt tttaattttt ttaatcgttt 7980
taaattttta ttattttttg gtattcgagg ttttaaatag aaattttatt gtacgggtta 8040
tttttagaga taaagttttt aagtttttta tttttttttt ttttttgttt ttaaagtttg 8100
agaattttaa taggaatttg ggtaattttt tttttttagg tttgttagga ttttattttt 8160
agtttgcgta gattagagtt aaaaagatcg gtttaatagt tttttagcgg gtatttttta 8220
gagaggtaaa gtgaaatttt cggttaggga aagaaagtgg tttttgggtg ttgaggtttg 8280
ttgtgtgaaa gggtgaattt tttttttttg aagtaattgg ggatttgttt tagggttgga 8340
ggttagtaga gataatttaa atcgttatgt ttagagtagg tagaggggta atttttttgg 8400

taaagatttt ataggatttg tatttatagt tttttaacgt tggttgatta tgttgaaagt 8460
agttgtttgg gtcggttttt ttttgtaaag tgtttatttt ttttgtggat tataatagat 8520
ttatagtttt ttattttagg tttgtattag atttataaag aggagaatat ttttttaatg 8580
tataatttaa ttaggtttga ttttgattta taaaattgtt ggaaaatatt tttttgtaaa 8640
gtattttttg ttattttagt gtgttttaaa atttttattg gggagggtgg agtgaggttt 8700
tttattatat ttttttattt ttaggatatg tttgtatttt agaatatgtg tagttagtt 8759

<210> 142
<211> 8759
<212> DNA
<213> Artificial Sequence

<220>
<223> chemically treated genomic DNA (Homo sapiens)

<400> 142

agttaattgt atatatttta aaatgtaaat atgttttaaa aataaaggaa tataataaaa 60
aattttattt tatttttttt agtggagatt ttggagtata ttgaaatagt aggaaatgtt 120
ttataaaaaa atgtttttta atagttttgt aagttagagt taagtttaat taaattgtat 180
attaaaagaa tatttttttt tttatagatt tgatgtaaat ttgaagtagg gggttgtgga 240
tttgttataa tttatagaga aaataaatat tttataagag aaaatcgatt taagtaatta 300
tttttaatat agttaattaa cgttgagaaa ttgtgagtgt aaattttgtg aagtttttat 360
taagaaagtt gtttttttgt ttattttaaa tatgacggtt tggattattt ttattgattt 420
ttagttttgg agtaagtttt tagttgtttt aggagaaaga agtttatttt tttatatagt 480
agattttagt atttagagat tatttttttt ttttaatcga gaattttatt ttattttttt 540
ggaggatatt cgttgagaag ttattgggtc ggtttttttg attttaattt gcgtaggttg 600
aaagtgaaat tttaatagat ttgaagagga gaaattgttt aaatttttgt tgaggttttt 660
agattttgag gataggagga aaggaagagt ggagggtttg ggagttttat ttttgaaggt 720
ggttcgtgta ataggatttt tgtttgggat ttcgagtatt aggaggtagt gagaatttgg 780
ggcggttggg ggagttgaag aatttgggag ggtaggaatt agtatttagg gtacgtagta 840
gaaattagga gagtttaggt taaatttttg gtaggtcggg agtttacggg agagttttag 900
gtttattttg ttgagttttt ttaggggtaa tttgagtgtt cgcgtaggta ggagtcgtta 960
gatattttag aatatagagt gattttgttt tgggtcgaaa ggcgatattt ttggaaggaa 1020
aaatttatcg tatgttttcg taaggttcgg agtagttatt tatttagggg tttatttcgt 1080
ttttgatata agtgggattg ggatagggta ttttgtttat tatgtcgtta gggtattata 1140
tattaggtgc ggtgaagtcg tttttttggt tcgttagttt ttgagggggt cgagtgtagt 1200
cgtagggggt tatagttttc gtttcgtcgt cgtcgtcgtc gtcgtcgtcg tcgtcgtcgt 1260
cgtcgtcgtc gtcgttatta tttttattat tattatacgg tttatataat tggttttttt 1320
cggttgtgaa gagagtgtgt taggatgagg aagcggcggt tgagggtgat ttagaatcgg 1380
gtttcgttgt attcgcgtta tgtaggttcg ttaggttttt atagcggtat tgcgtcgtcg 1440
tagtcgttta ggcgttgtcg tagtttagcg ggttttttag tttgatgcga gcgtggggat 1500
ggggaggcgg cggagggggc ggcggttcgg ttagagttag tggaaagttg tagtagtcgc 1560
gattttggta cgtagttgtt tcgtttagtg tttcggattt gtagagagat agggtagacg 1620
gtagtttaag tgtttcggag ttttttgttg tagcgttgtt agagtagttt aggttttcgt 1680
tttttagttt tttggtgtaa ttttttttga aaggggaata tttagtagta tttttagtgt 1740
ttttgtttgc gttgtcgttt agtagagaat ttttgtattc ggttaatggg gtataaggag 1800
tgggacgtat agcgggtgga atttttaaaa gtggggcgta tatgtaattt tttcgaagtt 1860
gtttttttgg atttagatgt tttaacgttt ttatatttag gtttatggat atcgatattg 1920
ttttatataa ttttttggcg ttgttagaaa tggtcgaagt gttttttaag taattgtttt 1980
tggaggaagt gggagttttc gaggtttttt tcgttttttc gttgttgttg ttttcggata 2040
ttgttttttg ttgttgttgt tgaaggagtt gtatggtgtt ggtttcgttt aggatgtttt 2100
taaggttagc ggagtagttg tttaagtcgg ggaaagtggg gtttagtagg gataacgtgg 2160
atggggtagt tgagttattt tcgttcggag gtgttggtag ttgttgcggt agttttttgt 2220
tggcggttac ggcggtttta ggttttggga cgtaattttt ttcggggtgg tatttagggg 2280
tcgattgcgg ttgtgaaggt tgttgttttt tatttaggat taggtagttt gtggggtttt 2340
tacgatgggt ttggggagaa ttatttttat tttgttgttg ttgttgttgt ttggggttag 2400
ttttttgttg ttgttgttgt tgttgttgtt gttgttgttg ttgttgttgt tgttgttgtt 2460
gttgttgttg ttgtagtagt agtaaattgg cgtcgggagg tgttgcgttc gcggtttttg 2520
ggtgtttggg gttcgggttt tggattattt cgcgtacgtt ttggaataga ttttggaaag 2580
tttttcggta ggttttggac ggcggtcgag ggtagatttt ttttagtttt aattgtattt 2640
ttattttga gtttggttga atttttattt tattttttt atttcgtttt ttttttttt 2700
gtttagaaga gtttaatagg ttgtgatgat gcggtagtcg ttgtagttag ttgtttattt 2760

gcgggaagtt ttagagaagg aaatttgcgg gagtaattac gtaaaaagcg ttttgatagt 2820
tttaaagttt cgtgtagaag aagatatacg gtggataaga tttgttttgt taggtttata 2880
gtttgttttt taatatttga agggtagatt taaaagatgt ttagatttta aaagaaaaaa 2940
ataaaatttt ttatttttta aatttagtgt ttattgaagt aggtgtaaat aagaattgag 3000
ttattatttt ttttttttat ttcggttttt ttgtttttgt ttgttttgtt ttttggttat 3060
tgaagatttg attgtttttt tattttttga tttttggttt cgtattcggg gtggggaagg 3120
taggaggagg tggagagaga gtgggggaaa atagaggttt tttttcgcgt gtagtttaat 3180
taggcgggtc gtggttgtag tgttgaagtc gttttttagt cgtttggttt ttaagagttt 3240
tttttgtaat gtgtagttag ttcgttcgtt cgtagttaaa gggagttatt tttttgtaaa 3300
ttcgggttgg tagcgttggg tcgacggggg cgggggggcg ggtggggaga agggtggtga 3360
gggggggttgg ttgagagtag tcgattgagg tagttgcggg agagaagacg gggggagggg 3420
ggaaggtagg gtggggggag gcggggtgga gaggaggagg ataaaggtag tcgttagttt 3480
tattaggtat tttttttgtt tttttcgagt ttttagtagt ttttaaaaat tttatcgaag 3540
aggaaagggt agtttcgggc ggcggtttcg aagtcgtcgc gttgtaagag gcgttggttg 3600
tcgttggagt cgggttgggg ttggcgtggt gcgttttttc ggtttttgta tagtattgga 3660
gcggttagtt cggttttttt tttttttgtc gttttttttg tttttgggtt tgtttcggat 3720
cgtttcgttt ttttagtaag ttggtttttc gggatttcgg agggggcgtt gggaggtgga 3780
gagtaaatgt aatagtttgc gagtcgggtt tcgtttttat cgggtcggtt tttttgtttt 3840
tttatttttt tttttttttt tttttttttt tttttttttt ttttttttat cgcgcgttaa 3900
cgtttgttta gtggttttgg agaaataagt gttggcgtag cgtgggcgag ggtaggagag 3960
gttagtttta gttttggagg atttttgttt tttgaatagt ttttgttttt ttaaattaag 4020
gtttaggcgt tagggtttta gattttttgg aggttagtat ttattaaatt tggttttaaa 4080
gtttaattta aaaaattaat agggtggagg cgagggaggg aggaaaagga cgataggaat 4140
atttgttttt ggttgttagg ggtttttttt aggattgata agagcgcgta gatgggagag 4200
tgggagagtt ttttataaat tgaggagtaa gttatgatta agttagtaga tatttgttga 4260
aattttagat ttcgttatag ggatagatcg ggtttcgtgg tattgtgtta tttgttttag 4320
gaatttttag ttttaagaat tagaggttat ttttattttt ttttttgttt ttttttttgt 4380
ttggaattgt ttttaattat gtatttttta tttagatttt taaaggtaaa attatttaga 4440
taagtaggta aatatataaa tattaaaaat atttgttatg tatatatagg cgtttaattt 4500
tgtttagagt tttgtaaacg tttgttttgt atgtagtcgt ttgttttttt aagttgtttt 4560
cgtatatttt aaattttata attggaatta tttaaggata gaatttatat agggttgttt 4620
ttcgtttttt gatggatagt taggcgtaga ggatgatgtg ggttattgta attgtttttt 4680
tgtggaggag gttttataag ttttgtgtaa aggtggagat gtaagtggga atggaaagag 4740
agagaaaatg taaacgtaaa tttataagga aaatattttt tttttatgtt agtagagatt 4800
ttgggttttt ttatgtttta aatttttttg atttttgatt ttggggaggt tttttgtttt 4860
aaaagttatt tcgagatttt tgtttgggtt ttttttgttt tttgtagttt tttatagaga 4920
aaagatgggt tgtagttttg ttgtagtttt attttgtgta tagttaaata ttataggtat 4980
ttatttattt tgattaaaga aggtaattta aatttaaagg taaggaagat tggatatgat 5040
aggttttatt ttatagatga ataggttgaa ataaggatat aatttgtttt atgttttata 5100
ttagtaaaat ttaaattagt tttttagttt attgttattt gagttagatt ttagatttat 5160
taatcgtatt gttatttggg aaatggttat aaatgaattt tttagaatat aaaggtttga 5220
agattttttt atttgaggag gtgaaaaatt attttttgag attttaatgg ttatagtttt 5280
ttttgaatat attttattat atgtaagtag aatgcgtaga tattttaatt ttatttgaat 5340
atttattgta atatttaatt ttattataat ttttaattag tattgtttaa taggaattga 5400
gtgggttggt aggatggtag aatggaatta atataggttt tagagttagg agaattatgt 5460
gtttatatat taatagtgaa ggaaggtgta aaaattattg aatgaataat ggataggagt 5520
gaagaggata tatagatata aagaggtttg aaaaaaaatt aaggtgagaa ataatgtttt 5580
gaagttattt tgaataaaaa gtagtttgat atttaaattt gggtttgaaa gattttaaag 5640
tttatgttgt ttttatatta ttaatattgt ttttatagtt tttatatttg aaaattgttg 5700
ttaatataag ttatttttag aaggttagtg tgttatttaa aagatataat gtgtatagaa 5760
taaatttgta aataggaaag tattttataa atttgaagga ttattatgtt atttttattt 5820
ttaaagatgg tgttgttaat aattatatta ttattaggaa aggttaatta ttataagtat 5880
attagggtta tatattttat ttggattttg aattttgatt ttttagttta aattttattt 5940
tttttttatt gtttttaaga ataagagata attattgatt aatattatat aagtttattg 6000
tttgtattag tttttttttt tttttttttt ttgttttttg tagtttttta gtaattaaat 6060
ggaattagag gtgttttttt agattagtta ggttgagagt aagtttttgt tataagtttt 6120
ttttgtaatt ttaagaggga tttattaata aagttttttt tagttttttg ttttggaaag 6180
ttaggatgag ttgatttttg gaaaatggtg attatcgggt tttatttttt aagtatttta 6240
tatatttgtt tatttatttt atttttgttg ttgttaattt agtttatttt tagaaggtat 6300
cgtttttttt tatttggagt attgtaaatg gttatttttt ttttattttt ttttagaatt 6360
agttttgtat tgtgtagtta gagggttttt ttttaatagt cgtattgagg tataattgat 6420
atatataata aattgtatat atttaaagtt gtataaattg attatttttg atatacgcgt 6480
gtatttatga aattattatt taattaagat agtgaatata tttatgattt taaaagtttt 6540

tattgtgtta ttttgtatat ttttttttta ttttttttat attttttttt tttaggtaat 6600
taatgatttg ttttttgtta gtatagattg ttttttattt tttagagttt tatgttaatg 6660
gaattatata gtatgtattt tttttttttt ttatttgtat tttttattga gtataatgtt 6720
tttgatgtat atttaagttg ttatgtatat tattagttta ttttttttta ttgttaagta 6780
gtattttatt gtattgttgt attacgtttt gtttatttat ttatttttga tggatatttg 6840
ggtttttttt agtttggggt tattataaat aaaattgtta taaatatttg tataaatatt 6900
tttgtatgga tgtatttttt tatttttttt aggtaaatat ttagttatgg aatgggtgag 6960
ttatagggta ggtatatatt tatttttgta agaaattttt aaattatttt tttaagtgat 7020
tgtattattt tatattttta ttttattaat atttggtatg gttagttttt ttaattttag 7080
tattttaatt agaggtgttt tttttattta attttatttt tttttatgaa taaagatgta 7140
gagtattttt tatgtgttta ttttataatt gtatattttt tttgatgaag attgtaaatt 7200
tttttttttt tttttttttt aattgggtta tttgtaattt attatggggg ttttagagtt 7260
atgtattttg agtataattt ttttattaga aatatgattt aataatattt tatttgtagt 7320
ttgtttttat tattatatta gtgttttttg aagtataaat attttaaatt ttgatgaagt 7380
ttagtttatt attttgttta tttatgaatt atgttaattt acggattatg gtgttagata 7440
taataaatta tttttaaatt taagattata tagattttta tttgtgtttt tttttagaag 7500
ttgtatagtt tttagtttta tatttaggtt tgtgatttat tttgaattaa gttttgtaga 7560
tagatattta atttttaata tagatgtttt attgttttgg tattattttt tgaaaatatt 7620
attttttttt tattttgttg tttttgaatt tttgttaata attaattttt aatatatatt 7680
tgtatagagg gttttttaga aagtttaatt tggttatatt attttttcgt ttaagttttg 7740
ttattgtttt tttattgttt ttaatagaaa agtttaaaat attaattata ttttatatga 7800
tttgtttttt tgattttatt ttatatattt ttttttttgt tttttgagat ttatttatat 7860
tggtttttat ttaggttttt taacgatttt ataatttttt ttgtttttgt atattttgtt 7920
ttttttattt gtaatgttgg tttatttttt tttttattta tttagttaat gtttatgtat 7980
attagtatat ttaggaagat tttttgaatt atttaggtta gtttaagttt tttatttatt 8040
tattttttaa tatattttaa tagtatttag tatttgttgg aggatttttt ataatttgtt 8100
ttttttattg aattgtatgt tttttaagag taggaattat gttggttttt gtttattatt 8160
gaatttttag gatttagttt ggtgtgggat atggttattg tttaataaat attttttgaa 8220
taaatgaatg gattttattt ttatagaatt aaaattttgt ttttaaattg ggagttttgg 8280
ttttgggggg ttgttaaatg ttttgtgatt ttagtatatt taggtatgta ataaattttt 8340
ttttaattta tgagtaaagt agagaattat tttaatttat gtattgaacg aatatttgtt 8400
aagtatttat tatttgttag gttttgtgtt aggtatttgg aatttattga tgaataggat 8460
attgtggatt ttgtatttaa ggagtttatt tatgatttag gaaggagagt gaagaataat 8520
ttaaataatt atatttgaat tggattggag atgaaaagag gggtttatga ataaattttt 8580
agagttgttg tttttgttga tttgtggata tttgttattt tttttgtata gatgtttata 8640
atggattatt tttttatttt aatatttttt ttgtttttaa tgttgaagtt attattttat 8700
tttatttttt tttatagttt tagttttaat tttttttttg gttgtttttt tttaggtta 8759

<210> 143
<211> 8404
<212> DNA
<213> Artificial Sequence

<220>
<223> chemically treated genomic DNA (Homo sapiens)

<400> 143

aggaagttaa aaattttgaa aaaaggttag aggaattgtt atttaaaata agtagtttag 60
agaagaatat aaatgatttg atggagttga aaaatatagt ataagaattt cgtgaagtat 120
atataagtat taatagttaa agtagaagaa aggatattag agattgaaga ttatttttat 180
gaaataaagt gtgaagataa gattagagga aaaagaatga aaagaaacga ataaagtttt 240
taagaaatag gggattatgt gaaaagatta aatttatgtt tgattgtttt tgtatttgaa 300
agtgataggg agaattgaat taagttggaa aatatttttt aggatattat ttaggagaat 360
ttttttaatt tagtacgata ggttaatatt ttaatttagg aaatatagag aatattataa 420
agatattttt taagaagagt aattttaaga tatataatcg ttagatttat taaggttgaa 480
atgaagttaa aaatgttaaa agtagttaga gagaaagtcg ggttatttat aaagggaagt 540
ttattagatt aatagtggat ttttttgtaa aaattttata ggttataaaa gatgggggtt 600
aatatttaat atttttaaag aaaggaattt ttaatttaga attttgtatt tagttaaatt 660
aagttttata agtgaatgat aaataaaatt ttttatagat aagtaaatgt tgagagattt 720
tgttattatt aggtttgtat tataagagtt tttgaaggaa gtattaaata tggaaaggaa 780
aaattggttt tagttattgt aaaaatatat taaattgtaa agattattga tattatgaag 840
aaattaatgg gtattaatta atgggtaaat taattggtta gtattataat gataggatta 900

gatttatata taataatatt aattttaaaa gtaaacgggt taagtgttat aattaaaaga 960
tatagattgg taagttggat aaagagttaa gatttattag tgtgttgtat ttagtagatt 1020
tattttatat gtaaagatac gtgttttaaa taagggaatg gaggaagatt tattatagta 1080
atggaaagta aaaaaaaaaa aaagtagggg atgtaatttt agtttttgat aaaatagatt 1140
ttaaattaac gtagattaga aaaagataag aagggtatta tatgatggta tagggattaa 1200
tgtaatatga agagttattt attttaagta tgtatgtatt taatatagga gttattagat 1260
ttataaagcg agtttttgga gaggaagaaa gagatttaga tttttacgta ataatagtgg 1320
gagattttaa tatttcgttg ttaatatttg atagattaat gagatagagt aattaataag 1380
gataattagg attttaagtt agttttgtat tatttagatt aaatagatat atttaatata 1440
tatatatata tatatatata tatatatatt ttttgagatg gggttttgtt tttgtcgtat 1500
aggttgaagt gtagtggtat gattttagtt tattgtaatt tttgtttttt gggtttaagt 1560
gatttttttg ttttagtttt tagagtagtt gggattatag gtatttgtta ttatatttag 1620
ttaatttcgt atttttggta gagatgtggt tttattatgt ttgttagttt ggttttaaat 1680
ttttgatttt aggtgattta ttcgttttgg ttttttaaat tgttgggatt ataggcgtga 1740
gttatcgtat ttggtttaga ttttgtcgaa attttaatat ttttttggta atgtaataga 1800
aaattaaaga ttttgatggt gaatttaatt gtttttgagt tgtgtggatg aaaatatatt 1860
ttattatttt tttttttaat ttttaagttt ttaggtatat gtgtaggatg tgtaggtttg 1920
ttatataggt aaatgtgtgt tatggtggtg tgttgtatag attaatttat tattatggta 1980
ttaagtttag tatttattag tttttttgat gttttttttt tttttatttt ttttcgttat 2040
aggttttagt gtgtgttgtt tttttttttg ggtttatttt atggtatgta tgtgtgtatg 2100
tgtgtgtgtg tgtgtgtgtg tgtgtgtgtg tgtgtattta tttatgtgta tatattatga 2160
tatatatatt atattttttt taaattatta ttttaggttt agggatatat gtgtaagttt 2220
gttatatagg taaattgtgt gtttttgggg tttggtgtgt atagattttt ttattattta 2280
ggtaataagt gtagtatttg ataggtagtt ttttgatttt ttgttttttt attttatatt 2340
tttaagtaat tttttagtgt gtgttgtttt tttttttatg tttatgtgtt tttattgttt 2400
agtttttatt tataagtgag aatatgtagt gtttggtttt ttgtttttgc gttagtttgt 2460
tgaggataaa ggtttttggt tttatttatg tttttgtaaa ggatatgatt ttattttttt 2520
ttatggttgt aggaaaatat atttatatat ttagtgaata tagttaaatt aatttatggt 2580
taagatatat atttttttgt aaattgagaa atttttttat tataattgta atatggtaat 2640
aaataaattt agattatata aatgtaattt agatgttgat aaattggttt taaataaagt 2700
aagtgatatt tacgtatgaa gagagtttat tttagttttt agaatttttt tttatttttt 2760
tgttattttt atttttagta agtaagtatt tattgtgtgt gtaatattgt gttaggtatt 2820
aaggtaatga tgatatttaa gatttggttt ttattttgaa gataagtata ggagaaatat 2880
tattagttat gatgtttttt gttgtaaata agtgaaaatt ttagtttagt ttaggttagt 2940
tttagggttg gttaaattag aggtttattt ataatattga ggatttagat ttttttgaaa 3000
ttttttttgt attaatatta gtatttttat tttttttgta gggtggtttg ttttacgtta 3060
taatggttgt agtaattaga agtgttatat ttttagtaat atttaatatt attattatta 3120
tttaataata tttagaattg tgttattttt gttttaattt attaggtttt tagtagatta 3180
ttttttattt tttaaatgtt aaaattgtat tttgagtttt tgtttaaatt aatttggggt 3240
gaggtgaatg gaagtagtat tttaattgta tttatttttt gtagttggat ttgggtttgg 3300
gttatttttt gatatttgtt tataagaaag atttttgaag ttaggtagga atggttgttg 3360
agtaggttag tgtttgttaa atttttatat atttttatat atgttataat atgtagataa 3420
aaagataatt ttttatataa tttgttggga taaatttagg aggtttatag tatgatttgt 3480
ttgaaggttt tttttgtttt agattttgtt tagttgtttt aggatgaggg gatttatatt 3540
atagtataat tgtattttat ttatagtata aattattttt ttttttttta gttgacgagt 3600
ttagatgggt aataatagtg tttgttaaag agaaaaaaaa atgtatttaa attagataat 3660
ttattggtat aaatatcgag atatagaagt gataatagtt ttaagttaat gtttgatggt 3720
ggtagtttta gtaagttttt ttttgatgtt tttgtgtttt tgtatatgtt ttttttttgt 3780
tattgttttt tttattaaat ataatataat ggataagtgg aattaaatag aattgagttt 3840
aaattttttg ttatttatcg gttttggtat tggataaatt aatttttttg agtttgtttt 3900
tttatttgta acgtagatta gttaatatta tttattggaa agcgttgttt tttagttaat 3960
gtatgtaagg tttaaaattt agatgacggg ttgataggtg tagtaaattt ttatggtata 4020
cgtatgttta tgtaataaat ttatacgttt tgtatttgta tttcggaatt taaagtaaaa 4080
aaaaaaaaaa aaaaaaaaag aaagaaagaa aaagaaaaaa aaggttgttt ttggggatta 4140
aataagataa ttatgtaagg tggttagtat agtttttggt atatagtaaa tgttaggttt 4200
gtttgataga tttttattta gtagttattg tttttttgaa aatttttttt agacgttttt 4260
acggtgtttt tcgtttttat attattataa ttttttatta tattattatt cgtttatttt 4320
ttatagtttt tttttttttt ttttttaatt agtgatttta aaaggttagt aagtgtttaa 4380
tattttttat tttttaagtg attggtaaag tttcgtattt attagcgttt taagtttgtt 4440
tttgttttgg tcgattttgt aaaacggatt gggcgggatg agaggtgggg ggcgtcgtta 4500
aggagggaga gtggcgtttt cgtcgagggt gtattagtta gatatttttt gcggggttcg 4560
agagtttttt ttattagatt tcgggatagg gatgaggttt atagttattt attagatttt 4620
ttgtatagtt tcgttaagtg tatttcggtt tggagggggt ggttttgggt agaagtacgt 4680

tcgggtcgcg tcggatgttt tttggaaggc gtttggattt acgttaggtt ttttagttta 4740
attttttatg atttagcgtt ttagttcgcg tatcgattag cgttttagtt ttttatagac 4800
gtcggcgggt tcgggagttt cgcggacgtg acgtcgcggg cggaagtgac gttttttcgc 4860
ggttggacgc ggcgtttagt tgtcgggcgg gggagggcgc gttcggtttt ttttagggga 4920
cgttgaaatt atttttgtaa cgggagtcgg gagaggacgg ggcgtgtttc gacgtgcgcg 4980
cgcgtcgttt ttttcggcgt ttttttatag ttcgttggtt ttcgtcgcgg aaaggcgtta 5040
tgtcgtttaa aatttttcga aaaacggtcg ttatcgtcgt cgttgtcgtc gcggaatttt 5100
cggtatcgtc gtcgtcgttt ttttttgagg aggatttaga gtaggatagc ggttcggagg 5160
atttgttttt cgttaggtga gcgagtagag tcgtcgtcgt tttacgcggg aagggcgttt 5220
cgggtgtgcg tagggcgggc gtaaggcggt tcggcgggga ttcgttttcg ttaggggtcg 5280
ggtttcggcg ggaggaggcg ttttttttgt ttttcgttac ggcggagcgt ttgtagaatg 5340
gtgataggat tttgggtttt tgggcgaggg gtttcggttt taatttgata ggtgtcgggc 5400
gggtggggtt agggttttga gcgaagtgat aggtgtagtt tttttttgtg aggttcggag 5460
gtagagggtc gttgcgagcg tttattagac gtaaaaaatg aaaaataaaa atataaaaat 5520
ggtgtttgtg ggagagtttt ttatcggaga attggagtat ttcggtggtc gtttgatttt 5580
ttgttttggt ttacgcgatg taatagttgg gaagtatttt ttttcgggcg tgtattgtat 5640
ttgaagttta tttgtgggag aggtcgatta gaaagttttg gataagaagc gtagggtttt 5700
gagtgtttat tgtttatagg atattcggtt taggagtttt gcggcgtttt tttagaataa 5760
taatgtatcg aggttttggg gatttaaagt tatttgtagt gattgatgga gcgtaatttt 5820
ttagaggaat tgaaatatgg gtaaaatttt tatgagatat ttattagaag tgtttgaaag 5880
tttttaaatt tttttttttt ttgtttgatg aatttttttt gcgtgttagt cggtttcggt 5940
ttgttttatt atttttttta ttttgttttt tgattttgaa ttagtaagga ttttggtgtt 6000
tttttttttt gtttttgttt ttggtataaa attagtggtt ttgtgcgtaa atggaaattt 6060
tcgttttttt ttattaagtg gaatttaaat ttaagtaagt ttatacgaat gtattagatt 6120
ttgaagggaa gtatttattg tattataata ttttattttt tttgattttt ttattttatg 6180
gttaaatagt tatgattgaa agagtgtaat tgttattatt gttagtattg gttttatttt 6240
gagataagtt tattgtagag ggaatgggat ttgtttttgt tttttattat tttttttttt 6300
atttttgttt attattaaat ttttttattt ttatttattt ttttttattc ggtatatatt 6360
aataatttac ggtagaaaag attgaagtgg gatttaggaa atggtttttg gaaggttatt 6420
aaaaatttat atatttaaat ggattgtttt ataggttagt taaaaattat attcgttaaa 6480
aaattaaaat aataataata aaaaattaaa aattacgttt ggggtatgtt ttgggaaaag 6540
atatggtttt agtttttgat taaattttga tgtatttaat ttttgtaaat tttttttggg 6600
aaaatgtagt aatggttatt ttaaagaatt ttatgttatg tatatagttt tggaagtata 6660
ttttaatgta gggtaattag gaatattgac gttgtggtta ttgtttggaa aaaagaggat 6720
tgtttttttt attttttaat ttattttata tattttaagt aatggaatta taaaaaattt 6780
ttttataagt taaaattata taagatatag tttgtttttg attatttata tttataaagg 6840
gttttattaa tatagatagt tgaattttat aaattaatta gtaagggtag aattattgtc 6900
gtgtagtttg atattgagta ttttgtttgg agatggaatt aaggtttagt ttaattgtat 6960
ttttagtgat agattttatt tttgatttga gagtttagat tttatttttg ttgttttatt 7020
tttggtttaa gtaatgtagt tggaagggaa attgttatta ggaatagtat attatattta 7080
ttgttgaaat aattaaaggt taattatgtt tcgagttggg tgtgatggcg cgtatttgta 7140
gtttatgtta tttaggaggt tgaggtagga ggattatatg agtttgggag ttggaggtta 7200
gtttgggtaa taacgtaaga ttttagtttt taaaaaaata aaaaagttga ttaggtatgt 7260
tggtttacgt tgtaatgtta gtattttggg aggttaagat gggaagattg tttgaggtta 7320
ggagtttgag aggagtttgg gtaataaaat aagattttgt ttttaaaata aattttaaaa 7380
aattagttag tggttgtggt atatttgtag gtttatttat ttaggaggtt gaggtgggag 7440
gatttttgag tttaggagtt tgagattgta gtgagttatg attttattat tgtattttag 7500
tttggatgat agagtgaaat tttattttaa aataagaaaa aaaaataaaa attaattatg 7560
ttttgaagga ggaattggta gtttagaata gtggttaagg gtatggatta tagagttaga 7620
ttgttgggtt tatatagtgg tttatttttt atcggttgag attttaggta agttttttaa 7680
tttttttaat ttgatagata tttgtaaatg tttattttta tagatgagaa tatttattta 7740
aaagggttga tgtcgggatt tagtgaaatt atatgtttaa ggtatttttt ataggattat 7800
agtaaatatt taaaaattat taattatttt ttatattatt tttattagta gataatatat 7860
agaaattaga tgaatgaaaa tgaattagtt aataagaatt tattaaatgt ttgtttttgt 7920
taaggggaaa ggataaaaat aagtagtcgt attatgtaag atgtgttagt aggtagagag 7980
atatatgaaa tattgataag agaagattat agtttaataa agttattggc gttgaattgt 8040
ataatattat ttatgggttt ttattttttt gttgttattt ttattttatt tttttaaata 8100
gttttagtta ttatatttat ttttttttat agaagtgttt tgttgttttg aagtatattt 8160
gatttattat gtaagtaaat attttttatt gtgtggtatt tttattttgg aatgattatg 8220
aaaaagataa ttatatgttt aaatttgaag tgtaatgttt ttttaagata aaataagatt 8280
ttaaagtatt taataatgtt tttttttata gtagtgttat gtgtaaatta ttgaaataag 8340
tatgtattga attaatttga tttataagta tatgttaatt atatgattat ttttatttgg 8400
tagg 8404

<210> 144
<211> 8404
<212> DNA
<213> Artificial Sequence

<220>
<223> chemically treated genomic DNA (Homo sapiens)

<400> 144
   tttattaaat gaaaataatt atataattgg tatatattta taaattaaat tgatttagta 60
   tatatttgtt ttaatagttt gtatataata ttattgtgaa aaagaatatt attaaatatt 120
   ttaagatttt attttatttt agaaaaatat tatattttaa atttaaatat atgattattt 180
   tttttatggt tattttaaaa taaggatatt atatagtgaa aaatatttgt ttgtatggta 240
   agttaaatat attttaaagt agtaaaatat ttttgtgaag gaaagtaaat gtaatagtta 300
   aagttgtttg ggaaaatagg ataaagataa taataaaaag ataaaaattt atagataata 360
   ttatataatt taacgttagt gattttgtta aattgtagtt tttttttatt aatgttttat 420
   atattttttt atttattagt atattttata tgatgcgatt gtttattttt gttttttttt 480
   tttaataaag ataagtattt aataaatttt tgttggttaa tttattttta tttatttaat 540
   ttttatgtgt tatttattaa taagaatagt atgaagaata attaataatt tttgagtatt 600
   tattatgatt ttgtagaaag tattttaaat atatggtttt attgaatttc gatattagtt 660
   tttttagata agtattttta tttgtaaaag tagatattta tagatgtttg ttaaattgga 720
   gaagttaaag aatttatttg aggttttagt cggtaagagg taaattatta tgtgagttta 780
   atagtttaat tttataattt atgtttttga ttattatttt agattgttaa tttttttttt 840
   aaaatataat tagtttttgt tttttttttt tgttttgaaa tagggtttta ttttgttatt 900
   taggttgggg tgtagtggtg aaattatggt ttattgtagt tttaaatttt tgggtttaag 960
   gattttttta ttttagtttt ttgagtagat gagtttatag gtgtgttata gttattggtt 1020
   aattttttaa aatttgtttt agagataggg ttttgttttg ttgtttaggt tttttttaaa 1080
   tttttggttt taagtaattt ttttattttg gttttttaaa gtgttggtat tatagcgtga 1140
   gttaatatgt ttggttagtt tttttgtttt tttaaagatt ggggttttgc gttgttgttt 1200
   aggttggttt ttaattttta agtttatgtg atttttttgt tttagttttt tgagtagtat 1260
   ggattatagg tacgcgttat tatatttagt tcgaaatatg attaattttt gattatttta 1320
   ataatgaatg tggtatgttg tttttagtga tagttttttt tttagttata ttatttaggt 1380
   tagagataag gtaatagaaa taagatttaa atttttaaat taggaatgga atttgttatt 1440
   ggaggtgtag ttggattgag ttttgatttt atttttaggt aagatgttta gtattagatt 1500
   gtacggtagt ggttttattt ttgttggtta atttatagag tttaattgtt tatgttaatg 1560
   aaattttttg tgggtatgga taattaagaa tagattgtat tttgtatggt tttgatttat 1620
   aagagaattt tttatagttt tattatttaa aatatataaa atgagttaaa aaatgaaaga 1680
   aatagttttt ttttttttaa gtagtggtta taacgttaat atttttggtt attttatatt 1740
   agagtatgtt tttaaagttg tgtgtataat atggaatttt ttagagtagt tattattgta 1800
   tttttttaag ggaaatttgt aagaattgga tatattagaa tttaattaga aattaaagtt 1860
   atgttttttt ttagaatatg ttttagacgt ggtttttaat tttttgttgt tgttattttg 1920
   gttttttaac gaatatggtt tttaattgat ttataagata gtttatttaa atatataaat 1980
   ttttaatagt tttttagggg ttatttttta aattttattt taattttttt tgtcgtgaat 2040
   tgttagtgta tatcgagtgg aaagaaatgg gtgagggtga aaggatttgg tgatagataa 2100
   gaatgggaga gaaaatagtg aaaaatagaa ataagtttta ttttttttgt aatgaatttg 2160
   ttttaaagta gaattagtgt tgataataat gataattata tttttttaat tatagttatt 2220
   taattataaa gtagaaaaat taagaaaagt aagatgttgt aatataataa atattttttt 2280
   ttaagattta gtgtattcgt atggatttgt ttaaatttag attttattta ataaggaaaa 2340
   acgaaaattt ttatttgcgt atagaattat taattttgtg ttaaaaataa aggtaaaaag 2400
   aggggatatt aagatttttg ttggtttaaa gttaaaaggt aaaatggaag aaataatgag 2460
   ataagtcgaa gtcgattaat acgtaagaag agtttattaa ataggaaaaa aaaaagttta 2520
   gaaattttta agtatttttg gtaaatgttt tatgaaagtt ttgtttatgt tttagttttt 2580
   ttaaagagtt acgttttatt aattattata gatggttttg agtttttaag gtttcgatgt 2640
   attattgttt taaggaaacg tcgtaaagtt tttgagtcga gtattttgtg ggtaatggat 2700
   atttaggatt ttgcgttttt tgtttaaggt tttttggtcg gtttttttta taaatggatt 2760
   ttagatgtag tgtacgttcg gaagaaaata ttttttaatt gttgtatcgc gtgaattaaa 2820
   atagaaagtt agacgattat cggagtattt taatttttcg gtgaaaaatt tttttataga 2880
   tattattttt gtatttttat tttttatttt ttgcgtttga tggacgttcg taacgatttt 2940
   ttgttttcga gttttataag agggaattgt atttgttatt tcgtttagga ttttagtttt 3000
   attcgttcga tatttgttaa gttgaagtcg agatttttcg tttaagaatt tagaattttg 3060
   ttattatttt gtagacgttt cgtcgtggcg gggggtaggg agggcgtttt ttttcgtcgg 3120
   gattcggttt ttggcgagga cgggttttcg tcgagtcgtt ttgcgttcgt tttacgtata 3180
   ttcggggcgt ttttttcgcg tgaggcgacg gcggttttgt tcgtttattt gacgagaggt 3240
   aggtttttcg ggtcgttgtt ttgttttggg ttttttttag gagggggcgg cggcggcggt 3300
   gtcgggggtt tcgcggcggt agcggcggcg gtggcggtcg tttttcgggg ggttttgggc 3360
   ggtatgacgt tttttcgcgg cgggagttag cgagttgtgg aggagcgtcg gggaggacga 3420
   cgcgcgcgta cgtcggggta cgtttcgttt tttttcgatt ttcgttataa aaataatttt 3480
   aacgtttttt gagaaaaatc ggacgcgttt tttttcgttc ggtaattgag cgtcgcgttt 3540
   aatcgcggga aaacgttatt ttcgttcgcg gcgttacgtt cgcgaggttt tcgggttcgt 3600
   cggcgtttgt ggggaattgg ggcgttggtc ggtgcgcggg ttgggacgtt aagttatgag 3660
   gaattaaatt gggaaatttg gcgtgggttt aggcgttttt taggaggtat tcggcgcggt 3720
   tcggacgtgt ttttatttag aattattttt tttaggtcgg ggtgtattta acggggttat 3780
   ataaagagtt tggtgggtga ttgtgggttt tatttttatt tcggggtttg atagggaaga 3840
   ttttcgggtt tcgtagggaa tatttggtta gtgtattttc ggcgggagcg ttattttttt 3900
   tttttggcgg cgttttttat tttttatttc gtttaattcg ttttgtaaag tcggttaaaa 3960
   taaaaataaa tttggagcgt tgataggtgc ggaattttat tagttattta gaagatagaa 4020
   aatgttagat atttgttggt tttttgggat tattggttag ggaaaggaag ggagggagtt 4080
   gtggggaatg aacggatagt agtgtaataa aggattgtag tggtgtaaga acgggaagta 4140
   tcgtggaaac gtttgaggaa gatttttagg ggagtagtag ttgttgaata gaagtttgtt 4200
   aggtaggttt gatatttatt atgtattagg aattgtgttg gttattttat ataattgttt 4260
   tatttaattt ttagaaatag tttttttttt tttttttttt tttttttttt tttttttttt 4320
   ttttttttat tttaagtttc gggatataag tgtagaacgt gtaggtttgt tatataggta 4380
   tacgtatgtt atggaggttt gttgtattta ttaattcgtt atttaggttt taagttttgt 4440
   atgtattagt taagaaataa cgttttttaa tgggtagtat tagttagttt acgttgtaga 4500
   tgaggaaata ggtttagagg agttaatttg tttaatatta gggtcgatgg gtagtagaga 4560
   atttgaattt aattttattt gattttattt gtttattata ttatgtttga tgaagaaaat 4620
   agtgatagag aaggagtatg tgtaaaggta taaagatatt agaaaagagt ttgttgggat 4680
   tattattatt aaatattggt ttaaagttgt tattattttt gtgtttcggt atttgtatta 4740
   atagattatt tagtttgaat atattttttt tttttttggg tagatattgt tattgtttat 4800
   ttgaattcgt taattgagaa ggaaagagat ggtttatgtt gtgaataaaa tatagttgtg 4860
   ttgtgatgta aattttttta ttttggagta gttgagtaag gtttaaggta ggaagaattt 4920
   ttaagtaggt tatgttgtaa gttttttgag tttattttag taagttgtat aagggattat 4980
   ttttttattt gtatattatg atatatgtga ggatgtgtaa aggtttggta agtattggtt 5040
   tatttaatag ttatttttat ttaattttag aaattttttt tgtgggtaaa tgttaggaga 5100
   tagtttaggt ttaggtttag ttataaagaa tgaatataat taaagtgtta tttttattta 5160
   ttttatttta gattagttta ggtaagagtt taggatgtag ttttgatatt tgaaagataa 5220
   ggagtagttt gttgggagtt tgataaattg aaatagggat ggtatagttt taaatattat 5280
   taaataatag taataatatt aagtattatt gaggatgtga tatttttgat tgttgtagtt 5340
   attgtaacgt gaggtaagtt attttataga gaaggtagaa atgttgatat tggtgtagga 5400
   ggaattttaa aagaatttgg atttttagtg ttataaatga gtttttgatt tggttagttt 5460
   tggagttgat ttaagttgag ttgaggtttt tatttgtttg taatagaaag tattatgatt 5520
   gataatgttt tttttgtgtt tatttttagg atagagatta aattttgggt attattattg 5580
   ttttagtatt tagtatagtg ttatatatat agtaaatgtt tgtttattaa aagtagggat 5640
   ggtaggaaag tgaaggaaag ttttaggaat tgaagtaaat tttttttata cgtagatatt 5700
   atttgtttta tttaaaatta gtttattaat atttagatta tatttatgta atttagattt 5760
   atttattatt atattataat tatgataaag agatttttta atttataaaa gaatatatgt 5820
   tttgattatg gattaattta attatgttta ttagtatgta aaatatattt ttttgtagtt 5880
   ataaaaagga atgagattat gttttttgta ggaatatgga tggagttaga agtttttatt 5940
   tttagtaaat taacgtagga atagaaaatt aaatattgta tgtttttatt tataagtggg 6000
   agttaaataa tgagaatata tggatatgaa gaggggaata atatatattg ggggattatt 6060
   tgagggtgta gggtgagagg atagaagatt aaaaaattat ttattaggta ttatatttgt 6120
   tatttgggtg gtgaaaaaat ttgtatatat taaattttag agatatatag tttatttata 6180
   taataaattt gtatatgtat ttttaaattt aaaataatag tttgaaaaaa gtgtggtgta 6240
   tatattataa tatatatata taaatagata tatatatata tatatatata tatatatata 6300
   tatatatata tatatatata ttataaaata gatttaggaa ggggaataat atatattggg 6360
   gtttgtagcg gggaagggtg gggagaggga gagtattagg agagttaatg gatgttgggt 6420
   ttaatattat ggtgatgggt tgatttgtgt agtatattat tatagtatat atttatttgt 6480
   gtaataaatt tgtatatttt atatatatat ttgggagttt aaaagttgaa ggaaaaaata 6540
   ataaaatata tttttattta tataatttag aagtaattga gtttattatt aaaattttta 6600
   gttttttgtt gtattattaa agaagtatta gaatttcggt agagtttaga ttaggtacgg 6660
   tggtttacgt ttgtaatttt agtagtttgg gaggttaagg cgggtggatt atttgaggtt 6720
   aggagtttga gattaggttg gtaaatatgg tgaaattata tttttattaa aaatacgaaa 6780
   ttagttgagt gtggtggtag gtgtttgtaa ttttagttat tttggaggtt gaggtaggag 6840
   aattatttga atttaggagg tagaggttgt agtgagttga gattatgtta ttgtatttta 6900
   gtttatgcga taagagtaag attttatttt aaaaaatata tatatatata tatatatata 6960
   tatatatatt gaatatgttt atttagtttg aatggtgtaa aattgatttg aagttttgat 7020
   tatttttgtt aattattttg ttttattgat ttgttaaata ttgatagcgg ggtgttaaag 7080
   ttttttatta ttattgcgtg ggagtttaag tttttttttt tttttttaag gattcgtttt 7140
   atgaatttgg tagtttttgt attgggtgta tatatattta ggataggtag ttttttatgt 7200
   tgtattgatt tttatattat tatgtaatgt tttttttgtt tttttttgat ttacgttggt 7260
   ttaaagtttg ttttattaga gattaggatt gtattttttg tttttttttt tttttgtttt 7320
   ttattgttgt ggtaaatttt tttttatttt tttatttaga gtacgtgttt ttgtatgtga 7380
   gatgagtttg ttgaatatag tatattgatg ggttttgatt ttttatttaa tttgttagtt 7440
   tgtgtttttt aattgtggta tttagttcgt ttatttttaa ggttaatatt gttatatgtg 7500
   aatttgattt tgttattatg atgttagtta gttagtttgt ttattagttg atgtttatta 7560
   gtttttttat agtgttaatg gtttttataa tttggtatgt ttttgtagtg gttgggatta 7620
   attttttttt tttatattta gtgttttttt taggagtttt tgtaatgtag gtttggtggt 7680
   gataaaattt tttaatattt gtttatttgt gaaggatttt atttattatt tatttataaa 7740
   gtttagtttg gttggatata aaattttggg ttgaaaattt ttttttttaa gaatgttgaa 7800
   tattggtttt tattttttat ggtttgtagg gtttttgtag agagatttat tattagtttg 7860
   atgggttttt ttttgtaggt aattcgattt tttttttggt tgtttttaat atttttggtt 7920
   ttattttaat tttggtgaat ttgacgatta tgtgttttgg ggttgttttt tttgaggagt 7980
   atttttgtgg tgttttttgt attttttgaa ttggaatgtt ggtttgtcgt gttaggttgg 8040
   ggaaattttt ttggataata ttttgaagag tgttttttaa tttggtttaa ttttttttgt 8100
   tatttttagg tatagggata attaaatata ggtttggttt ttttatatag ttttttattt 8160
   tttggaggtt ttgttcgttt ttttttattt tttttttttt aatttgtttt ttatatttta 8220
   ttttataaag gtgattttta atttttgata tttttttttt tgttttggtt gttgatattt 8280
   gtgtatgttt tacgaagttt ttgtgttgtg ttttttagtt ttattaggtt atttatgttt 8340
   ttttttaaat tgtttatttt aggtagtaat ttttttaatt tttttttaag gtttttagtt 8400
   tttt 8404

<210> 145
<211> 5312
<212> DNA
<213> Artificial Sequence

<220>
<223> chemically treated genomic DNA (Homo sapiens)

<400> 145

gtcggtttcg ggggtcgacg atttttcggg taggcgacgt gttttgttta ggttttattt 60
ttcgcgttcg taaaacgggg tggataacgt agtttaaggt agagtcgcgt taaggttttt 120
cgttgtcgtc gggttttggc ggtttgatcg ggtttggggt tcgagcgtgt tttcgggttt 180
gggggggtcg tcgcgatgga ttcgttggta gcgttttagg atcgtttggt ggagtagttg 240
ttgtcgtcgc ggatttaggt ttagaggcgg tttaaggtgc gtgtgtggag agggcggaac 300
gtgggtttgt gatttcgcgg gtttcgggat tcggtatggg gttttcgttt tcgcgcgtta 360
cggttcgtta gcgtttaggt ttagtttttt ttttcgtagc ggtttcgtag gttcgcggtt 420
ttacggtggg gcgacgtgtt ttgtttaggt tttattttac gcgattttaa aacgcggcgg 480
agaatgcggt cgtgggattt ttttaggggt tttaagggtt tgcgggggtt gggcgtttgg 540
gcgcgcgggc gggggaggtt cggcgtcgcg gtttataaat aggcgttgtt tcgttatcgt 600
cgttgtcgtc gttttcgcgg ttggattcgg gttaggtggg gtttttcgtt ttttggtatt 660
tttcggaggg gtgagtgtcg aggtttttag ttttttcggt ttatggaggg ttttgagtgt 720
gttttagggg ttttaatttt gttgtttttt gttcgaggag ggaggatatc gtttgtgttt 780
gttgcggttt gatatttttt tacggaataa atatagtgtc ggtttttgta gagttttttt 840
tgggattggg gttaggggtg tttttttttg ttattatttc gtttgttttt ttatttttag 900
gtttttgttt acgggttttt tttttatttt tgttaacgaa atgttttatt ttgggaatcg 960
tattttttgt tgttttttta tatcgtattt ggtaagagat agggtattgg gtttttggag 1020
aaatttttgt gtttttattt tgttttattt tgtttttagg atattgataa gtagtacgtg 1080
ggtttcgtta tattgtttaa ttaggtgtat cgtaagtcgg tgaagaaagg ttttgatttt 1140
atatttatgg tggttggtga gtgggttagg tttttcgggg gagtggttgg ggttattggt 1200
tagttaagtt ttgtcgttgg agttttagat ttaacgtagt tttttttttg tatttgtgtg 1260
taggtgagtt aggtttgggg aagtttatat tggtttatag tttttttttg atagatttgt 1320
ataaggatcg gaagttgttt agtgttgagg gtgagtggtt tttaggaggt tttggtattg 1380
atttttagtt tttttttatg ggattttttt aaggattttt ttttaggttt agtttttatt 1440
gttttgtttt cgcggtgtgg gtttttgggg ggtagggtta aggtattaag atggatgagg 1500
acgagggttt tggttgttaa gggtgagggg ttgagggttg gagaggtttt tttagtggtt 1560

tttttttcgt agagcgtatt agttagacgg tagagatttt aaaatatacg gtggatattg 1620
aggagaaggg agttaagttg aagtttatta tcgtggatac gtcgggattc ggggacgttg 1680
ttaataatat cgagtggtga gtgaggtttg ttgagaaagg ttttgtttag gcggttatag 1740
tattcgaggt ttggttttat tttttttttg tttatagttg gaagtttatt atcgattatg 1800
tggattagta gtttgagtag tattttcgtg atgagagcgg ttttaatcga aagaatattt 1860
aagataatcg agtgtattgt tgtttatatt ttattttttt tttcgggtat gggtgtgtgg 1920
ttgttttggg gttaggttcg ggagtgtagt ttttataata tggttttttg gttgtgttta 1980
tgtttatttt tggttgtttt cggtaggttg cggttagtgg atgtgggttt tatgaaggta 2040
ttgtatgaga aggttaatat cgtgtttttt atcgttaaag ttgattgttt tgtttttagt 2100
gagattcgga agttgaagga gcgggtgagt ttgtcgtcgt ataggggttt ggttagggtt 2160
ttggggttga gagtattagg gggatttgtt tggttttaaa tttgatggtt tttgttttat 2220
tatagattcg gaggagattt gataagtttg ggatttatgt atattagttt tttgagtgtg 2280
attcggacga ggatgaggat tttaagtagt aggatcggga attgaaggtg aatatgtaga 2340
ttggtggggt aggggggatg gagttggtga ggggtagaat tagagggttt tgtttttttt 2400
atattgagtt tgttggagga gggttaggtt agtttagttg ggtgtaagag ttatttgttt 2460
taggagtaga ggatttgtat tttttttatt tagggttaga aggtaaaggt tattatgttt 2520
ttgtttggtt ggggtgggtt atgtgggttt cgtgtggtgt ttggtaggta tggagtattt 2580
gtttagattt agaggtgtag tagtgggtta ggtttttaat tttaattttt tttttatggg 2640
ttattagagg aaggggttgt tttagtggta tattttggtt tttagatttt tgagttttag 2700
ggttggtggt tttgggtgtt tcggtagtta gagtgatgat ggagattatg ttaggggtag 2760
gtggttatta gggtaggggt attagtatgg ggagatatag gtttagtttt ggggagggaa 2820
ggggtagttg gagggggtgg tttttgatga gggaggagat gaggagggag tatgtttggt 2880
tgggtatagt ttggaaaggt tttggaatgt gagttatatg ggttttggtt ttagttaggg 2940
gtagagaggt tagaaagggg taacgttagg atttttttga ggagagatag ttgatggtga 3000
tgttggaggg gtgtttcggg agttatatgt tcgtttttta ttagtaatcg tgtaattacg 3060
tttatcgggt gtgagttttt tttttttttt tttttaggag agcgcgtttt tcgtcgttat 3120
aggtagtaat acggtggtgg aggttaaggg gtagcgggtt cggggtcgat tgtatttttg 3180
ggggatcgtg gagggtgagt agagttttgg ggtattaggt ttggtggggg aaggttgttt 3240
tgggtcggcg ttagtttatt atttattttt atttcgtagt ggagaattag gcgtattgcg 3300
atttcgtgaa gttgcgtaat atgtttattc gtacgtatat gtacgatttt aaggacgtga 3360
cgtgcgacgt gtattacgag aattatcgcg cgtattgtat ttagtagatg attaggtgcg 3420
cgttttagtc gcgagttaga tttcgttttt ttggtttcgt ttacgttttt ataattgagg 3480
gtcggttttg ttagtttatt tagatttgaa ttttgtatta ttttttaagt tttttttttt 3540
ttttagagtt tggttttttt agaattaagt ttagggttgt gagggtttcg gagggtaggg 3600
ttttagtagt ggcggggatg ggttaggtat cgttagttta cgttgagttt ttcggtggcg 3660
tcgtttcgtt tatttttttt ttcgtttcgc gtagtaaatt gatttaggat agtcgtatgg 3720
agagttttat ttcgattttg tcgttgttta tttcggacgt cgagattgag aagtttatta 3780
ggatgaagga tgaggaagta tgtggggcgg cgggggcggc ggaggcgggc gttagggatg 3840
ttttttcgcg gtgttgttta ttcgtcgggt tgttttcgtt cgtagttgag gcgtatgtag 3900
gagatgttgt agaggatgaa gtagtagatg taggattagt gacgttcgtc gcggatatat 3960
cgttcgtttt cgggacgttt tcgtattttt ggatattaga tcggattgtt ttcgattcgg 4020
agacgcgggg ttatagtttt tagttgattt taatttattt ttagtattat ttttttttag 4080
gttattgtgt ttgttttcga ggggtttgga tcgtagtttt cgtttagttg gttttttttg 4140
attttggggg attaggagcg aagttgggcg ggatttagaa gattcgtttt ttttgttttt 4200
ttttcgtttt cggacggtta tagtatttaa atcgtaggtt ttgttttggt aggtaggtaa 4260
agttaggtag aagaggattt ttaggatttt gggtttgttt tttgttttag tgttgtagaa 4320
cggatttggg agtttttttt tgtttgtttt cgcgggttat ttagcgagtg ttgagatttt 4380
attttttgtc gaggcgggtc gagttttttt ttatttttag acgtttagcg ggtagggttg 4440
ggttgaatta aatgggagtt ttttagatat aaggaggtta gaggttgtaa ggagcggggt 4500
cgtgatcgtt tatatttttt ttttatagtt cggttcgatt tggagggttt tcggggtatt 4560
gggcggtgag ttattttttg gtaattttcg gtgtcgtttt ttgttttcgt tcgaggtttt 4620
ttttttttag tatcgttgtg gtgtgtcggg attttgagtt taggtttttc gatgttttta 4680
ttcgtatgat tttttttcgt tatacgatgt ttcgtttttt ttcgttgtga atgtcgcgtt 4740
ttgttttggt gataggagaa taatgttggt gaacgtcgta gcgggtgttc gagtgtttcg 4800
tgtgtttttg agagcgggtg ggagcggaag tttgagcggt ttgcggtttt cggcgatagt 4860
gtgttatttg tcgttgtagc gcgcgttcgc gcggtttttg ggttattttt ggttaggtcg 4920
tagtattgtg gtcggtgcgg gcgtggtagg ggcggggcgg ttttatcgtt cggttttttc 4980
gtttacgttt ttcgttgtag agtaagtcgg gttgtcgttt tttcgttatg ggtttcggtg 5040
agtttggagt tcggtcgggt tttcggttgt tttttaggtc gattcgggtt gagaggagtt 5100
ttggtcgttt ggttgtagtt gggagagatt tgggttagat ttagagggga tttttagtcg 5160
gcgtgcgggg tggttagggt ggagaggttg gcgggttatc gggacgtcgg gtattagggg 5220
ttggatggag tcgggtcggt agtttgggta tttagagatg tcgtttaggt gttcgtcgat 5280
cgttcggttt attgcggcgt tttttttgta gg 5312

<210> 146
<211> 5312
<212> DNA
<213> Artificial Sequence

<220>
<223> chemically treated genomic DNA (Homo sapiens)

<400> 146

tttgtaaggg aagcgtcgta gtaagtcgag cggtcggcgg gtatttgggc gatatttttg 60
agtatttaga ttgtcggttc ggttttattt agtttttgat gttcggcgtt tcggtagttc 120
gttagttttt ttattttgat tatttcgtac gtcggttgga agtttttttt aagtttgatt 180
taagtttttt ttagttgtag ttaaacgatt agagtttttt ttaattcggg tcggtttagg 240
gagtagtcgg gggttcgatc ggattttaga tttatcggag tttatggcga gaagacggta 300
gttcggttta ttttgtagcg ggaggcgtag gcgggagagt cgagcgataa ggtcgtttcg 360
tttttgttac gttcgtatcg attatagtgt tgcggtttgg ttagaaatag tttagaggtc 420
gcgcggacgc gcgttgtagc ggtagatagt atattatcgt cggaggtcgt aggtcgttta 480
ggttttcgtt tttattcgtt tttaggggta tacggagtat tcggatattc gttgcgacgt 540
ttattaatat tgtttttttg ttattaggat aggacgcggt atttataacg gaagaaaacg 600
gagtatcgtg tggcgggaag ggattatgcg ggtgggaata tcgggaggtt taggtttagg 660
atttcggtat attatagcgg tgttggggag aagaggtttc gagcgagggt aggggacggt 720
atcgagagtt gttaggaggt ggtttatcgt ttagtgtttc ggggggtttt taggtcgggt 780
cgggttgtgg agaaggggtg taagcggtta cgatttcgtt ttttgtagtt tttggttttt 840
ttatgtttgg agggttttta tttgatttag tttaattttg ttcgttaggc gtttggggat 900
aagggaagat tcggttcgtt tcgatagaaa atggggtttt agtattcgtt gggtgattcg 960
cgggagtagg taaaggaggg tttttaagtt cgttttgtag tattggggta gggaatagat 1020
ttaggatttt gggaattttt ttttgtttag ttttgtttgt ttgttagagt agggtttgcg 1080
gtttgggtgt tgtgatcgtt cgggggcggg ggaagggtaa gggaggcgga tttttgaagt 1140
ttcgtttaat ttcgtttttg attttttaag gttagagagg gttagttggg cgggggttac 1200
ggtttaggtt tttcggaaat agatataatg atttgggagg gggtggtgtt gagaataaat 1260
tagggttagt tgggggttgt ggtttcgcgt tttcgggtcg ggaatagttc ggtttggtgt 1320
ttaggggtgc gagggcgttt cggagacgga cggtgtgttc gcggcgagcg ttattggttt 1380
tgtatttgtt gttttatttt ttgtagtatt ttttgtatgc gttttagttg cgggcggaga 1440
taattcggcg ggtgagtagt atcgcggagg agtatttttg acgttcgttt tcgtcgtttt 1500
cgtcgtttta tatatttttt tattttttat tttgataagt tttttagttt cggcgttcgg 1560
ggtgggtagc ggtaggatcg ggatggggtt ttttatgcgg ttgttttggg ttagtttgtt 1620
gcgcggggcg ggggggagga tgggcggggc ggcgttatcg ggaggtttag cgtgggttgg 1680
cgatgtttgg tttattttcg ttattgttga ggttttgttt ttcggagttt ttatagtttt 1740
ggatttggtt ttagggagat taggttttgg gggagggggg gggtttaggg aatggtgtaa 1800
agtttaagtt tgggtgggtt gataggatcg gtttttagtt atggagacgt gggcggggtt 1860
agaggggcga ggtttggttc gcggttgggg cgcgtatttg gttatttgtt ggatgtagtg 1920
cgcgcggtag ttttcgtagt gtacgtcgta cgttacgttt ttgaggtcgt gtatatgcgt 1980
gcggatgagt atgttgcgta gttttacgaa gtcgtaatgc gtttggtttt ttattgcggg 2040
gtgggggtgg gtagtgggtt ggcgtcggtt taggatagtt tttttttatt agatttggta 2100
ttttaagatt ttatttattt tttacgattt tttaggggta tagtcggttt cggattcgtt 2160
gttttttggt ttttattatc gtgttgttgt ttataacggc gaagggcgcg tttttttggg 2220
ggaaggaggg agaaaggttt atattcggtg agcgtaattg tacggttatt gatgggaaac 2280
gggtatgtaa ttttcggggt atttttttag tattattatt aattattttt ttttaaagag 2340
attttggcgt tgtttttttt tggttttttt gtttttggtt ggagttagag tttatgtggt 2400
ttatatttta gggttttttt aggttatgtt taattaggta tgtttttttt ttattttttt 2460
ttttattaag aattattttt tttagttgtt tttttttttt ttagggttga gtttatgttt 2520
ttttatgttg atgtttttgt tttggtggtt atttgttttt ggtatggttt ttattattat 2580
tttagttgtc ggggtattta aagttattaa ttttggagtt tagaaatttg ggagttaggg 2640
tgtgttattg ggtagttttt ttttttttggt gatttatggg aagagggttg gggttggggg 2700
tttggtttat tgttgtattt ttggatttgg gtaagtgttt tatatttgtt aagtattata 2760
cggggtttat atgatttatt ttagttaggt aaagatatgg tgatttttat tttttagttt 2820
tggatgaagg gggtataggt tttttatttt tagaataaat gatttttgta tttaattggg 2880
ttgatttggt ttttttttag taggtttaat gtgaaggaga taaagttttt tggttttgtt 2940
ttttattagt tttatttttt ttgttttatt agtttgtatg tttattttta gttttcggtt 3000
ttgttgtttg aagtttttat tttcgttcga gttatattta gggaattggt atatatggat 3060
tttaaatttg ttaatttttt ttcggatttg tggtggggta aggattatta gatttgaggt 3120

tagataagtt tttttggtat ttttagtttt agggttttgg ttaggttttt gtgcgacggt 3180
aggtttattc gtttttttag ttttcggatt ttattgggga taagatagtt agttttggcg 3240
atgagaggta cgatgttgat ttttttatgt aatgttttta tgaaatttat atttattggt 3300
cgtagtttgt cgagagtagt taagggtggg tataggtata gttagggggt tatattgtag 3360
gggttgtatt ttcgagtttg gttttaggat agttatatat ttatgttcga agggggagat 3420
gaagtatagg tagtagtgta ttcggttgtt ttggatgttt tttcggttga ggtcgttttt 3480
attacggaag tattgtttaa attgttggtt tatatagtcg gtgatgggtt tttagttgtg 3540
ggtaggaggg aggtgaggtt aggtttcgag tgttgtggtc gtttaggtaa ggtttttttt 3600
agtaggtttt atttattatt cggtgttgtt gatagcgttt tcgaatttcg gcgtgtttac 3660
gatggtgagt tttagtttga tttttttttt tttaatgttt atcgtgtgtt ttagaatttt 3720
tatcgtttgg ttgatgcgtt ttacggggaa ggggttattg gaagggtttt tttaattttt 3780
agttttttat ttttggtagt taggattttc gtttttattt attttggtgt tttggtttta 3840
tttttagggg atttatatcg cgagaataga atagtgggag ttggatttga aagggagttt 3900
ttggagaggt tttatggaag gggattgggg attagtgtta gggttttttg ggggttattt 3960
atttttagta ttgagtagtt ttcggttttt gtataagttt gttaggaaga ggttgtggat 4020
tagtgtggat ttttttaggt ttgatttatt tgtatatagg tatagagaag gagttgcgtt 4080
aggtttgggg ttttaacgat agagtttggt tggttagtga ttttagttat tttttcgagg 4140
agtttggttt atttattagt tattatgagt gtgaagttaa agtttttttt tatcgatttg 4200
cggtgtattt ggttgggtag tgtggcgaag tttacgtatt gtttgttaat gttttgggga 4260
taggatggaa tagggtaggg atataaaggt ttttttagaa gtttaatatt ttgtttttta 4320
ttaaatgcgg tatgaggaga tagtagaggg tacggttttt agggtggggt atttcgttgg 4380
taaaaataag gggagggttc gtgggtaaaa gtttggagat aagaaggtag gcgaggtggt 4440
ggtaggagaa ggtatttttg gttttaattt tagggagggt tttgtaggga tcgatattgt 4500
atttatttcg tggggagatg ttaggtcgta gtaggtatag gcgatgtttt tttttttcgg 4560
gtagagggta gtaggattgg gatttttggg atatatttaa ggttttttat aagtcggaga 4620
agttgggggt ttcgatattt attttttcgg agagtgttag ggggcgggag gttttatttg 4680
gttcggattt agtcgcgggg gcggcggtag cggcggtggc ggagtagcgt ttatttatag 4740
atcgcggcgt cgagtttttt tcgttcgcgc gtttaggcgt ttagttttcg tagatttttg 4800
gggtttttgg aggggtttta cggtcgtatt tttcgtcgcg ttttggagtc gcgtgaggtg 4860
aggtttggat agggtacgtc gttttatcgt ggggtcgcgg atttgcgagg tcgttgcgga 4920
gggaagggtt gagtttaggc gttggcgggt cgtggcgcgc ggggacgggg attttatgtc 4980
gagtttcgga gttcgcgggg ttatagattt acgtttcgtt ttttttatat acgtattttg 5040
agtcgttttt gggtttgggt tcgcggcgat agtagttgtt ttattaggcg gttttggggc 5100
gttgttagcg agtttatcgc ggcgattttt ttaggttcgg gggtacgttc ggattttagg 5160
ttcggttagg tcgttagagt tcggcgatag cgagggattt tggcgcggtt ttgttttagg 5220
ttgcgttgtt tatttcgttt tgcggacgcg ggaagtgagg tttggatagg gtacgtcgtt 5280
tattcggggg atcgtcgatt ttcgaggtcg at 5312

<210> 147
<211> 3025
<212> DNA
<213> Artificial Sequence

<220>
<223> chemically treated genomic DNA (Homo sapiens)

<400> 147
   tttttacgat ttgattttta tagtcgtttt atttatgggt tttttataaa ttaggggtat 60
   agaggagtat tgaaagttag tttagaggtg agcgcgcgta gttagcgttt ttcgcggata 120
   tagtagtcgg gtgttggaga ggtttggaaa gggcgtgtcg gagagttaag tgttagtcgt 180
   ttagggtttg tcggtcgttt tttttttttt tgttcggtag gggatttagc gcgtacgtta 240
   gtgtggaggg gcgggttggt tggttagttt cgggtttttc ggttatttcg ggattttttt 300
   ttaagtttcg ttttcgagtg tttttattgg tttcggattt tttttttttt agttgttcgt 360
   ttgggtttcg gggcgtttag gttattacgg ataaatagtt tagggcgttt ggtcgagaag 420
   ttaggggtga ggaagttttg gggcgttgtc gtcgtttttt ttaattataa attaggtcgg 480
   ataggagagg gaggggtggg ggatagtggg tggggattta gattgttagt attttgttat 540
   ttatagtcgg ggttttcgag cggtagaaag tttcggttat tttttgtcgt ttgggttggg 600
   cgaaagttag gatcgtgtcg cgttatcgtt aggatatgga gttattgtcg ttatcgtttc 660
   gcgacgtaga tttgacggtt ttcgacggtt ttttttgttt ttttgttata acggacgatt 720
   tttatgacga ttcgtgtttc gatttttcgg atttgcgttt tttcgaagat ttggattcgc 780
   gtttgatgta cgtgggcgcg tttttgaaat tcgaagagta ttcgtatttt ttcgcggcgg 840
   tgtattcggt ttcgggcgta cgtgaggacg agtatgtgcg cgcgtttagc gggtattatt 900
   aggcgggtcg ttgtttattg tgggtttgta aggcgtgtaa gcgtaagatt attaacgtcg 960
   atcgtcgtaa ggtcgttatt atgcgcgagc ggcgtcgttt gagtaaagta aatgaggttt 1020
   ttgagatatt taagcgttgt acgtcgagta atttaaatta gcggttgttt aaggtggaga 1080
   ttttgcgtaa cgttattcgt tatatcgagg gtttgtaggt tttgttgcgc gattaggacg 1140
   tcgcgttttt tggcgtcgta gtcgtttttt atgcgtcggg ttcgttgttt tcgggtcgcg 1200
   gcggcgagta ttatagcggc gatttcgacg cgtttagttc gcgttttaat tgtttcgacg 1260
   gtatggtaag gtcgggattt taggaagtga ggaagttagg gcggcgttcg ggatattagg 1320
   gacgcgtttt cgagggcggg gagttggttt tgcgggaggt ttgggttagg atttttttcg 1380
   agagagagga tttttttgtt ttgggtagtt gttattgggg tagtttgttt tggaagtgtg 1440
   cgggtaagcg ttcgagttgt tttattgggg gcgttattag aatattgtag cgcgaacgtg 1500
   aagatttttt tttttattta tttttatttt taaaatgtaa atttgcgttt tttggtgatt 1560
   gttcgttttt ggtttggttt tgtatgttgt agattttatt ttttatttat tcgtaattat 1620
   ttttttaatt aggataggtt tgggttcgga attagagttt taggttagag ttagggaggg 1680
   ggcggttata ggaattggtg ttcgggtttc gagtcgtttc gcgggtttga tttagtcgtt 1740
   tttgttgttt gtagatggat tatagcggtt tttcgagcgg cgttcggcgg cggaattgtt 1800
   acgaaggcgt ttattataac gaggcgttta gcggtgggta tttcgggttt ttttttgttc 1860
   gttttttttt ttttatggag ttgttttggt ttttatttag gacgttttta tttttattta 1920
   tatacgttta tgttttggga agtggtgtag gagatgaaat attaagtaag tagttttttg 1980
   ttttttcgat tgtttcggat tttaattaaa gtttttagtt tttaatttgt tttaaagtat 2040
   tgggttcggg ggtgggaggt ttgtcgcggt tttatttttg tttattaatc gagttttttt 2100
   cgcgtagaat ttaggttcgg gaagagtgcg gcggtgtcga gtttagattg tttgtttagt 2160
   atcgtggagc gtatttttat cgagagtttt gcggcgttcg tttttttgtt ggcggacgtg 2220
   ttttttgagt cgttttcgcg taggtaagag gttgtcgttt ttagcgaggg agagagtagc 2280
   ggcgatttta tttagttatc ggacgtcgtt tcgtagtgtt ttgcgggtgc gaattttaat 2340
   tcgatatatt aggtgttttg aggggatggt ggtcgtttat tcgttcgagg gatggtgttt 2400
   ttagggtttt tcgcgtttaa aagattgaat ttaaatgttt tttttttaat agcgttttaa 2460
   aagcgatttt ttttgaggta ggagaggcgg gagaattgaa gttttcgttt tcgttttata 2520
   gggtaaggat atagcgcggt tttttttacg tagtattttt ttcggagatt tattgcgatg 2580
   gtcgtttcgt gtttttcggt gggttagagt tgaattttga ggggttaggt ttagtttttt 2640
   cgcgtttttt tttatggggg tgagattttc gtagatttaa ttttgtttcg ggatgtatcg 2700
   gttatttggg ggggcgtgag atttagtgta tttcggtttt aaatgtagta ggtgtaatcg 2760
   taatttattt ttaattcgtt tttcggttta ggattatttt ttgtaatatt tttgtaattt 2820
   atttttgtaa ataagagttg ttttgttaga gtaggagttt ttggggttgt atttattttt 2880
   gaggtatggt gtgtggtgtt atagggaatt tgtacgttta tatcgtaggc gggcgagtcg 2940
   cgggcgttcg tttaggtgat taaaataaag gcgttaattt atatcgtcgt ggtttcggtt 3000
   ttttttggat atgggtgtgg gattt 3025

<210> 148
<211> 3025
<212> DNA
<213> Artificial Sequence

<220>
<223> chemically treated genomic DNA (Homo sapiens)

<400> 148

ggattttata tttatgttta gggaaagtcg gagttacggc ggtataaatt agcgttttta 60
ttttgattat ttgagcgagc gttcgcggtt cgttcgtttg cggtataaac gtataaattt 120
tttgtagtat tatatattat gttttagaga taaatatagt tttaggggtt tttgttttgg 180
taaagtaatt tttatttata ggaatagatt ataaaagtat tataaaaagt ggttttgaat 240
cgggaaacgg gttgggggtg ggttacggtt atatttgtta tatttgggat cggagtgtat 300
tgggttttac gtttttttaa ataatcggtg tatttcgggg tagggttagg tttgcgaggg 360
ttttattttt atgggggagg gcgcgagaaa gttgaattta gttttttaag gtttagtttt 420
ggtttatcga ggaatacgga gcggttatcg taatgggttt tcgagaaggg tgttgcgtgg 480
aaaaaatcgc gttgtgtttt tgttttgtgg ggcgggggcg gaaattttag ttttttcgtt 540
ttttttattt taagagaagt cgtttttaaa gcgttgttgg gaggggggta tttaagttta 600
attttttggg cgcgagggat tttaggggta ttatttttcg ggcgggtggg cggttattat 660
ttttttagag tatttggtat atcgggttgg ggttcgtatt cgtagggtat tgcggggcgg 720
cgttcggtga ttgggtgggg tcgtcgttgt tttttttttc gttggggcgg gtagtttttt 780
gtttgcgcgg aggcgattta gaaggtacgt tcgttagtag gagggcgggc gtcgtagggt 840
tttcggtgga gatgcgtttt acgatgttgg ataggtagtt taggttcgat atcgtcgtat 900
ttttttcggg tttgggtttt gcgcggggag ggttcggtta gtaagtaggg gtggggtcgc 960

gataagtttt ttattttcgg gtttagtatt ttgagataga ttggaaattg aggattttag 1020
ttagagttcg ggataatcga aaagataggg agttatttgt ttagtatttt attttttgta 1080
ttatttttta ggatataggc gtgtgtgagt gggggtggga gcgttttaga tagaggttag 1140
gatagtttta tgaaggagga ggagcgagta gggagaggtt cggaatattt atcgttgggc 1200
gtttcgttgt agtaggcgtt ttcgtagtag tttcgtcgtc gggcgtcgtt cggggggtcg 1260
ttgtagttta tttgtaaata gtaagggcga ttgagttagg ttcgcgggac ggttcgaggt 1320
tcgaatatta atttttgtag tcgttttttt tttaatttta gtttaaggtt ttagtttcgg 1380
gtttagattt gttttggttg ggggggtaat tacgggtggg taggagatga ggtttgtaat 1440
atgtagggtt aaattaaggg cggatagtta ttaaggggcg taaatttata ttttggaagt 1500
agggataagt agagaaaaag atttttacgt tcgcgttgta gtgttttaat agcgttttta 1560
atggggtagt tcgaacgttt gttcgtatat ttttaaaata ggttatttta gtgatagttg 1620
tttaggataa gggggttttt tttttcggga aggattttgg tttaaatttt tcgtaaggtt 1680
agtttttcgt tttcggaaac gcgtttttga tatttcgagc gtcgttttaa tttttttatt 1740
ttttggggtt tcggttttat tatgtcgtcg gagtagttgg agcgcgggtt ggacgcgtcg 1800
gagtcgtcgt tgtagtgttc gtcgtcgcgg ttcgggggta gcgggttcgg cgtatagaag 1860
gcggttgcgg cgttaggggg cgcggcgttt tggtcgcgta gtagagtttg taggttttcg 1920
atatagcgga tggcgttgcg taggattttt attttgggta atcgttggtt tggattgttc 1980
gacgtgtagc gtttgagtgt tttaaaggtt ttatttattt tgtttaggcg gcgtcgttcg 2040
cgtatggtgg cggttttgcg gcggtcggcg ttggtggttt tgcgtttgta cgttttgtag 2100
gtttatagta ggtagcggtt cgtttggtgg tgttcgttgg gcgcgcgtat atgttcgttt 2160
ttacgtgcgt tcggggtcgg gtgtatcgtc gcggggaagt gcgagtgttt ttcgggtttt 2220
aggagcgcgt ttacgtgtat taggcgcggg tttaggtttt cgaagaagcg taggttcggg 2280
gagtcgaaat acgggtcgtt atagaagtcg ttcgttgtgg taaaggagta gagagagtcg 2340
tcgggggtcg ttaggtttac gtcgcggagc ggtggcgata gtagttttat attttggcgg 2400
tggcgcggta cggttttggt tttcgtttaa tttaagcggt agagagtggt cggaattttt 2460
tgtcgttcgg gagtttcggt tgtagatagt aaagtgttgg tagtttgaat ttttatttat 2520
tgttttttat tttttttttt tttgttcggt ttgatttgtg gttaaggaaa gcggcggtag 2580
cgttttaggg tttttttatt tttagttttt cggttaggcg ttttgggtta tttattcgta 2640
gtagtttaaa cgtttcggag tttaggcggg tagttggggg agggggagtt cgaggttaat 2700
aggaatattc gggggcgggg tttggggggg gttttcgggg tggtcgaggg gttcgagatt 2760
ggttagttag ttcgtttttt tatattggcg tgcgcgttag gttttttatc gggtagggag 2820
ggagggagcg atcggtaagt tttaggcggt tgatatttgg tttttcggta cgtttttttt 2880
aaattttttt aatattcgat tgttgtattc gcgggaaacg ttggttgcgc gcgtttattt 2940
ttgagttgat ttttaatatt tttttgtatt tttgatttgt gggggattta tgggtagagc 3000
ggttgtagaa attaggtcgt gagag 3025

<210> 149
<211> 11913
<212> DNA
<213> Artificial Sequence

<220>
<223> chemically treated genomic DNA (Homo sapiens)

<400> 149

taaagtaggg tgttattaaa gaaagaagga agaaattggt taaacgaatt tagaatataa 60
ataaaattat tgtataaata aaatttttgt ttacgattaa gttggaatgg cggttaaaaa 120
agaaaaggga aaaaaaattt ttagatgtag gtagattttt ataattaagt tattttttaa 180
aggggaagta aatgtgatag aatatttagt aagtaaattt attgaggaat attttgtttt 240
tatataatta aatttaatat tgaggtaaaa ttgataatta ttaaattgag ggtagttggg 300
agagggaaag gggatgagat aaaatttagt ttataaaata ttttattatt ttttataggt 360
taaaaaatat aaaagaaata tttggaaaga attgtttaag acggaaagat tttaagattg 420
tgtgattttt tttttttttt tttttttttt ttttttttta taggtgtaga ataatttttt 480
tttttttttt cgtggttttt ttttttttta tttttttttt ttgttgtttg taaagttgaa 540
tttggggtta ggagttttaa ttgtaatttt agattggaat tttagatggt gcgtttagga 600
ttggaagtaa tttttagttt attaatttta tgggtttacg tatttgtttt ggttacggtt 660
tggtttttat ttttattgtg gtattaatgt agaataaatt gagatatatt ttttatataa 720
ttagttttag aatttaaaaa gatttacggt ataataatag aggtagtgtc gcggaatttt 780
tttattgagg attttcgggg agttcgggga ttatttcgcg gtggtattag aatagacgag 840
taattttagc ggttttaggt attgcgaatt gggggagagg gggaaatttt gtatcgtatt 900
cggtagtatt taacggtttc gggtttttag atagggttag cgaggagagt taggatagta 960
agtggggatt ggttaaaaaa aatcgattta attttgaaat gattagaaga agttgggagt 1020

agttattata aagttttttt tttgtttttt ttttttttta attgaaggaa tgacgttgaa 1080
atttttatcg ttagcgcggg gttgggaatt attttaaata tttgttttgt agtttcgagt 1140
gggtagattt cgaagagttg gagtcggcgg ggaggggagg aatagttgtt gggttgtttt 1200
gcgcgtgggg ttcggggcgc gggtttcggg gagaggcgtt agtcgaaagt ttgttttttt 1260
ttcgcgatag gtagtagcga ggtcgagtta ttttttatta cggtttgcgg gtttcgcgcg 1320
tagtgtggtt gttttcgttt ttgatcgatc gtagcgtttt ggggtgttta tttagttgtt 1380
cgcggtcgtt gggtgatttt cggagggcgg tttaggcgtg cgtattggtt tttggggttg 1440
cggttgtagt agtttagttt cgcgttttgt tagatgtagt ggatagcgtc ggggtgaaag 1500
taagggttga gaaattttta ttgttttgtt tttatttagt ttcgattttt ttatattgta 1560
ataattatgt attttcgagg tcgggatgtt tgcgttttgt tagtgtatta acgttatatg 1620
gttttttagg ggttgttttc gtttttgtat tttattagtt ttgaagaggt agaggggtgg 1680
tgggtaaaat ttttaattgg acgttgagag attcgggttt tagttcggcg ttgttattaa 1740
tttgtttttt gtttttaaat agtagtagta ataaaaattg taaagttgat tgagttaaag 1800
gtttatattg tttttttatt taatttttag aataatttta tgaggtgggc gttagtattt 1860
tattatttcg tttgattagt aaggaaattg aggtttagag agattgcgga attcgtttag 1920
ggttatatag ttgttggtta tttggtagag ttgaggtttt attttggggt tattaatttt 1980
ttgtttttta tcgttgcgtt ttatagattg tttttttttc ggttgtttgg gtttagtttt 2040
ttttttttgt aaaacgagaa gtttaatttg tagttttttt tgttaatcgg tggtttagat 2100
taggaatttt tgttatagat ttcggcgttg ataggggtaa gaaggtgggt gaaagaaggg 2160
ggcgtttagt tttttatagt ttgttttttt ttgggatttc gcgaagggcg ggtttcgcga 2220
gttaaaggag gtataggaga gcgtttatcg ttcgcggcgg gtgaaggtgt tatttgtttt 2280
cgtgttaggt tgtgagtttt ggtgtttagt ttagggcgtt aaggttagtg tagttggtat 2340
gttttttttg gaaaatttta ggtttttcgt agagaacgtt atttataata aagaagagga 2400
tagagaggta tggagcgttt tgcgattgta ggagtacgtt agttttttag cgttggttta 2460
gtgtcgtttg ggttttcggg tatgtggatt cgttggggtc gtacggagat tttttgtcgg 2520
gtttttgggg tttttcgatt gcggtttttt agtttagtat tttttttttg gtttcgtagg 2580
ttgtagggaa ttttttttat ttttttagtc ggagaagttt aagtcgggcg agggggtatt 2640
tcggggttcg tatcggtgtt tttttttttt tcgtttttat aaggattttg agaaaataaa 2700
tggtagagga gagaggagtt ttatatttgt ttggtttttt ttttttttta tttattttta 2760
tattttttat ttcggggtaa aaatttattt ttgaaaaatg ttggtagaga tttacgtgtt 2820
tttgttttat ttgggtttta taaatataac gatttatatt taagttagtt tttttttaga 2880
taattttttt ttttttcgtt aaaagtgtta aggatggtaa aagaagaaat aattttaatt 2940
ttttcgtttg gaaatgaaag ttttcggttt tttataaagg gtttttcgtt ttttatagtt 3000
gagttttagt taagaaaaac gatttttaag tagaaataat aggcggggag aaggaaggga 3060
gatataggga tttggggggt ttttagggta attggtagtg aattttgttt cgagagtttt 3120
ttttttattt aaaaaattaa acgcgcgagt ttcgcgaaag gtttagggat agatcgtgtg 3180
ggagaggatt gagtagagag cgtgggggta gtgttttgta gaattttttt ttttttaata 3240
ataattttaa aagtttttga gtggagacga cgtaaagtta agtagtaaag gtggtttggg 3300
aggtaagcgg agggtttaag tgtcgtattt ttatttttag ggttttttgc gtttacggga 3360
tgcgtatttt taagaagtgc gtttttcgag taagttttgg gttcgtatat atttcgggtt 3420
cgtagttaga atttaatggc gataacgttt atgtaatgta agttaaaaat taaagcgtaa 3480
aaaattatta tgttatttat tgaaacgtta ttttttgtta aattgtaatt attttgtttt 3540
atataagttt ggttggaaag tttgtagttt tagttcgggt tagttaggta taggaggtcg 3600
gattgtaatc ggttgttttt ttttcgtcgc gtttggtcgt tttacgttgc gtcgtcgttg 3660
ttgtttttg gcgttttgg gatttatac gtattttga aatacgtttc gtttcggttt 3720
tcggtttttt tttttgttta ggggttgttt tttaatagat attgattttt ttagaagatt 3780
taaaaattaa attaaaatat tttttattcg ttttaaatat ttgttttggg gcgcgggggt 3840
tgttaaatag agattagacg aagggagtta gatttagcga agtttttcga gtttttaaag 3900
attcgaatat taattcgcgt tcgtgggtcg atggaggttt tttttatttt attttttggt 3960
ttttttaatt ggttttcgtt ttttggttaa ttattgagta attagaatgg tattttcgat 4020
tagggttata ggtagtgttc ggcggagtgg ttttaggagt tattcgtttt ttgtcgggtt 4080
tcgtatttaa attttttttt ttattttttt tttttaaatt gggcgttagg atgtttcggt 4140
cggaatatac gtaggttttg ggcgtttgtt taagggtttt tttttttttt aaattagtcg 4200
ttgttttttc ggtttaatcg tagaagaatt agatattttt tattggaaag ggaaattaag 4260
tgttgttgat tttaatttta ggtaggcggt aatcgttttt cgtttggcgt aaattttatt 4320
aagtaaataa ttattagtcg atcgaaatac gttcggttta taattggtgt aattttcggt 4380
tatttaattg agggacgttc gtttttagtt tcgatttttg gaatttataa agggttattt 4440
ttttttttag tgattttaag attatggtta tttttttatt cgatagtttt agaagtaaga 4500
gttagattta agggtgtaaa gtaagggtat acgttttttt gaagtttgat tgagtttttt 4560
ttgcgttttt ttgaagtttt cgtttttttg gagtttattt gttttttttt ttaaattatt 4620
tttttagatt aataatttta tttttatttt tatcgtattc gattttgttc ggatttattg 4680
tttatttgaa cggatttttt agtgagacga ggtttttata ttggcgaagg ttaagaaggg 4740
gaggtggggg gagggttgtg ttatatcggt tagttgagag cgcgtgttgg gttgaagagg 4800

agggtgtttt cgagagggac gttttttcgg attcgttttt attttagttg cgagggcgtt 4860
tttaaggagt agcgcgcgtt gtttggtcgg gtttgggttg ttgagtgaat ggagcggtcg 4920
agttttttgg tttttttttt ttttcgcgtc gtcggttttt tttatttgag ttttgggaag 4980
ttgagggtag ttaggtagtt ggggtaagga gtttaaggta gcgtttatat tcgggggttt 5040
ttcgtaattc gatcgtttgt tcgttttttt atttttcgtt ttttttttta tttatttatt 5100
tatttattta tttatttaga gtcgggacgg tagtttaggc gttcgggttt cgtcgttttt 5160
tcgtcgcgat tttggatttt tttttgttgt aggattcggt ttttacgtgt gtttcggagt 5220
cggcgtttta gtatacgttt cgtttcgggt ttgggtgttt atagtagtta gagtagtagg 5280
gagttcggga ttcgggcggt atttgggtta agttaggcgt cgtcgaggtt agcgttgaac 5340
gtttttaggg tcggaggagt cgcggggcgt tcgggtttga gtcgtagtaa atgggtttcg 5400
acgtgcggga tttgaacgcg ttgttgttcg tcgttttttt tttgggtggc ggcggcggtt 5460
gtgttttgtt tgtgagcggc gcggcgtagt gggcgtcggt gttggatttt gcgttttcgg 5520
gcgtttcggt ttacgggtcg ttgggcggtt tcgcgtcgtt atcggtttcg tcgttatttt 5580
cgtcgtcgtc gttttatttt tttattaaat aggagtcgag ttggggcggc gcggagtcgt 5640
acgaggagta gtgtttgagc gtttttattg tttatttttt cggttagttt attggtatag 5700
tcggagtttg tcgttacggg tttttcggtt ttttttcgtt tagttaggcg ttattcggtt 5760
aggttaggat gttttttaac gcgttttatt tgtttagttg tttcgagagt tagttcgtta 5820
ttcgtaatta gggtaagtag gtcggggagc gttttttacg cgcggggtag tggcgttagg 5880
gatttttcgt tttaggatat tttttttttt tatttttttt gatcgtagtt tttatttagt 5940
tgttttttaa gggtcgtgag gatagcggaa gcggcggttg gggaggaggt cggagagtgg 6000
gagtgtacgt aggtattggt tttcgatatt ttttaaagtt aggtagagtt aggagtttga 6060
ttgttcgtaa gagtcgggag ggcgtttggg gttttttaga gttcgagaga tttcgggatt 6120
gttagttttt acgggagtgg aattaggaga tcggcgtagt gtttggtttt gttttgggga 6180
gagttttgtt aattaaggaa acgttaaatt gttattaagc gtagcgtttt ttagacgttt 6240
ggattttgga ttcggagtgg gattagcgcg ggggtgagcg tttgtgttgt gttgttttcg 6300
tagcgttaaa atgtttagga gtatttttag tttttataat acggtgttgg tatatattta 6360
ggtgtatatt aagtatcgta gagaattaga atgagtatat gtatttttag taattaaatg 6420
gttttttgga atagcgtcgt gttcgtggat agttttcgcg tatggtaata tagaagtgga 6480
tttttattgg gtttcgttta ggcggcggtt acgtaggttt tggggatgga agagaatttg 6540
atttattcga gtttaagtta tgcgggatta ggttttatcg taggtagtgg aaggaggtac 6600
gagttttggt ttttttttta gcgtatttgg ggcgtttttt tattttgtaa acgcggggtt 6660
tagttgggat agtatttttt taagatttgg ggttagaata aggatagggt tattcgttgt 6720
gtttagttag gatataagga gggaattcgg tatacgataa gtattaggtt ggttttttat 6780
ttcgcgtcga tgttcgttta tttttttttc gtttgggcgg cgcggattcg aatttgcgga 6840
gtttttgtag tttgtttttt ttatttattt ttagtggatt tttacgttat tgagaattta 6900
agttagttcg gtttgattcg gtaatttagt aaacgtttta ttaatataat ggcggatgtt 6960
ttttttggag taaaattatt cgagggttta gggatcggga tgtttttgga atgcgtggga 7020
agtagagtgt attttttgta ggaggtattt tcgtttaagg ttcgggagaa gggttttgtt 7080
tttgttgttc gtttttattc gcgtttttta aacgcgcgtt tttaggtcgt ttttatttta 7140
gtttaattag gagtattcgg ttttcgcgaa gttttggaga aatcgaaatc gttttttttt 7200
tcgggagaat ttgggattat tgttttgtgg aggaatcgta agtagattta agggttgttt 7260
ttattttatg tttttagtta gtgaaattag tgttttgtat ttaaggggcg ttgggataat 7320
ttattggtcg ttgaggtttg tgttttttga ttagatcgcg ttatttttgt ttagtaggtt 7380
ggtgttattt ttggggagtt tttaggtttt agtgttttcg tttataagag gatagttgag 7440
atgggtaatt tggagattgg gttcggtttg aagttatatt ttatagatgt agaagcgtta 7500
tttattattt ttttggggtt ttaaatatgg aattcggtaa tttatttaaa tatttgtgat 7560
tgagaaataa tgaattcgag agtattgttt gtatgtgtgt tatttggagg cggttgagaa 7620
attatcgaat ttatgttttt ggggtataaa atattattac gttattattt ttttagtatt 7680
tttaggggat ttttgaaatt tgggttttgt cgtttcgttt gggttttttg tttagttttt 7740
tttagggtag gtttggtgtt tgggatttta ttatatgtcg tggggggtgg gattgtgggt 7800
tttgtagtta gtaggtatta tatttttgga aaatatttgg atttttttgt aatatatagg 7860
cgtggttgtt atggagaaaa agttaaaagg ggattaggat tagtaggtga atgtattttt 7920
tttttttatt ttggagaaaa agtttaatgg agagagggtt ttagatttaa gttatatagt 7980
ttggaagtat tatttttagg tttaagtttt gttttgtagt gaaaatagat gggtttattt 8040
tatttagttt ttgaggttta ttgggtagtt tggatgtgag aagcgtttcg gttgttattt 8100
tggagtgggt aagggtggtt ttcgagattt tgggtttatt ttttggattt ttagggtttt 8160
tgttgttttt ttaggaatgt agggtttggt ttaagatttg ggttgtgaga ggtaagggta 8220
ttaattttta aaaatgtgga aatggttatt agagcgttaa ggttaaattt taggggagag 8280
gaattaaggt cgtttttgga tttttagggt agtttagtta tggtgtatag gtaggggtaa 8340
ttgttggtat ttatttataa tcgtttaaaa ttttataagt atgtttatat tttaaatttt 8400
taaatattat attttgtatt ttaattttgt aaatattaat ttagtaaata ttaatcgttt 8460
atttggttag gtattgggtt ttgaatatta attaagataa tggtttcgag aatttttgta 8520
agaagtttat tagttaattt tggagaggtt ttttgttttg gatgaggaaa tttaggtttt 8580

atgagattag gtggtagagg tgggatttga atttagatta tgttagggtg tttttatttt 8640
ttttttaggg attttggttt tatcgttgag atggattttt tttggtttgc gggaggggta 8700
ttaaattttt agtttatgtg tgggggttga cgggtttgtg agggtagatg tggggtgagg 8760
atttgagtag ttttagatta gatgtaaggt ttaaggagga ggggaaaaaa gtttgtttgg 8820
gtcggacgcg gtggttcgag gcggttggat tatttgaggt taggagtttg agattagttt 8880
ggttaatacg gtgaaatttt gtttttattg aaaatataaa aaaaaaaatt ggtcgggttt 8940
ggtgacgggc gtttataatt ttagttattc gggaggttga ggtataagaa tcgtttgaat 9000
tcgggaggtt gaggttgaag tgagttgaga tcgcgttatt gtattgtagt ttgggcgata 9060
gagcgaaatt tcgtttcgga aaaaaaaaaa aaaaaaagag gttgtttgtt agtttattga 9120
tttcgtagtt tcgtaggttc gtagaatttt aaggtatgat taggtttggg attattaggt 9180
tattttaggg ttaggtgcgg aggtttgcga cgttcgaggg ttaaaggttt tttttttcgg 9240
ttatatagga gagggtattt tgttggggga taggtttttt ttaattatag tgtagttgta 9300
gttatcgttt ttagtttttt gagtattttt ttggttgagt ttgttgttta ttttattttt 9360
tagtttttac ggttcggtta ggttaggcgg ttttcggttt ttttgtagtc gtcggcgggg 9420
gaggagggta tagagcgcgt ttcgtttttt agcgtacggt taaggttgcg tttttttttc 9480
gggtcgggat taatcgtggg aaaggcgtta ttttggttat tcgatttttt ttttttaagg 9540
tttgtataag gagtttttga atacgttgtt ttttttagtc ggtagtttta ttttttttgg 9600
tttttgtttt cgtatttttc gcgttaggtt aagcgttggg tttagggata gcgcgtttta 9660
attaaattcg tgttcgataa gtttttaatt aaaagagatt ttttttcgtt tcgtaggcgt 9720
ttttattatt gtttttttcg gggggatttg aaggagaggg tttgaggtcg gtttttgttc 9780
gtcgaggttt gcgtgttcgg tttgggagga ggtttaggag ggttcgcggg ttacgggtat 9840
ttttgggttc gagttttggg gtgcggacgg acgtttcgag agtgggtgtc gcgattcggg 9900
atttacggtt ttcgtcgggt acggatagtt gcggagtagg gttttgagga ttgtgtagtg 9960
ttttgggttt ttgtttattt ttgggtttag gaatggagaa gggttcgttt agagaaaatt 10020
ttttcgtagt ttttatttcg tttttttttt tttttttttt tttttttttt tttttttttt 10080
tttttttttt tttttttttg tgtttgtttt tttttttttt tttttttttt tagtgttttt 10140
ttagaaattc ggtttttatt tcgggtaata gtgggtgttt tgagttaaat ttcgtttgta 10200
agtttattta taggttttaa agaaggttag gttttaggtt ttaaaaggaa gtttgtggaa 10260
gtttaaacgt gttcgtttat gtaggagagt taacggggtc gggggttatt tttgtttatt 10320
tagtaggaga gaaatggagg ttttcgaggt tatatgataa aagttagtgg ttttttaaat 10380
ttttagtgtg tggtgttgag tagtgtgtga gatttaagtt tggatttgta ttattcggga 10440
gttaggattt gttttttttt tttttttttt gtattttgtt atattatttt ttaggtttat 10500
gtttttgttg ttttagagaa gttttttttt tttttttttt tttttttttt tttttgggcg 10560
gggggtggtg gtgtgtgtgt atgagcgttc gtgtgtgttt cgaaaatgga ggtaacgatg 10620
ttttgggttt tgtttatcgg ttgagttttg tttaagtttt tggaggtatt ttggttaaat 10680
agtgtaagag tgggagaatg ggtgttgttt gatttaattt atttttgaga attttgtgaa 10740
gtttggagat ttagggtagt tttgggttag gaggatttga ggttgttggg gtggttttgt 10800
taggatttta taggagtagt tttttttagt tattgtgttt ggattttagg ttatagattt 10860
cggaatttgt agaggaatga gattttattt ttgggtttcg gggttaatga cgtattttta 10920
gtcgaaggat tttttttttt agaggtagta atagtttttt agaagtttgt ggggtataag 10980
tttaaagtaa tttaataata aaaattataa aagattatgt tttttaattg aggttttacg 11040
tttttttttt tattttgggg ggggggggga atgttaggat ttttttaggg ggaaattgag 11100
gtaaaggatc gaagtattat tatggaggga aagggagata tagtgttagg tatattttaa 11160
aggaaattgg ttgtttagtt tggggagtga ggaagttggt ttttatagta gtggttaggg 11220
tcggattttt gggcgtaagg tgggttttat agggggaggg aggaggagag cgtagggtcg 11280
ttatttgaag gtttaattgg aaggggtttt tgtttttttt tttattagtt tttttttggg 11340
tgttatggtg ttaggtcggg tagttttaga tagataatat tattcggtaa ttattttttt 11400
tatttttaag attaggttat ttaattggtt tcgagtaatt aatgaaagtt ggtgcgcggg 11460
tttttgattt atagttcgga gttaacgcgg ggttagttta gtagatttat ttcgtttgtt 11520
cgcgttgttt ttgcggaaga gttgtagttt atttttgggg ggtgaataat tttttttgtc 11580
gattaggtaa cgagttttat atttaatttg ttaggggttc gttgtagaag cggtagagat 11640
cgtagaagat tgcggtataa ataaattggg aaaaagcgag gtgggggttt tagttagaaa 11700
aggatggaga agtttgtttt aaggttatat ttagggattt gggtcgtttt tttgtcgaaa 11760
gttttggagg tttgtggggt tggatttttt ttcgttgggg gttcgaatgc ggggtttagc 11820
gtatattttt ttcgttcgtg gttggtttag atttattgtt tcgttttgcg agagtatcgt 11880
tgatattgtg ttttttttcg tcgttttcgt agg 11913

<210> 150
<211> 11913
<212> DNA
<213> Artificial Sequence

<220>

<223> chemically treated genomic DNA (Homo sapiens)

<400> 150

tttgcgggag cggcggagag aagtatagtg ttagcggtgt tttcgtaaga cggggtagtg 60
ggtttgaatt agttacgggc ggggggggtg tgcgttgaat ttcgtattcg gatttttagc 120
ggaggagaat ttagttttat aagtttttag gattttcggt aggaagacgg tttaagtttt 180
tggatgtgat tttgggatag gtttttttat ttttttttga ttaaaatttt tatttcgttt 240
ttttttaatt tatttatgtc gtagtttttt acggtttttg tcgtttttgt agcgaatttt 300
tggtaaatta agtataaaat tcgttgtttg atcgatagag aaggttgttt attttttaaa 360
aataaattgt agttttttcg taggagtagc gcgggtaagc gaaatgaatt tgttgggtta 420
atttcgcgtt gatttcgagt tgtaaattag gggttcgcgt attaattttt attaattatt 480
cggagttagt taaatggttt ggttttaaaa atgggaagag taattatcga gtaatgttat 540
ttgtttgggg ttattcgatt tgatattatg atatttaagg gaaagttggt gagggggaaa 600
ataagggttt tttttagttg ggtttttaga tggcgatttt gcgttttttt tttttttttt 660
tttatgggat ttattttgcg tttaggggtt cgattttgat tattgttgta ggagttaatt 720
tttttatttt ttaggttgga taattaattt tttttagagt gtgtttgata ttgtattttt 780
tttttttttt atggtagtgt ttcggttttt tgttttagtt tttttttagg gaaattttag 840
tatttttttt tttttttaaa gtgagaaaaa gagcgtggag ttttagttgg aaaatatagt 900
tttttataat ttttgttatt aaattgtttt aaatttatat tttataagtt tttggaaagt 960
tgttgttgtt tttggggaaa aggatttttc ggttaaagat acgttattgg tttcggaatt 1020
taaggatggg gttttatttt tttataagtt tcggagtttg tggtttgaag tttagatata 1080
atggttaagg agagttgttt ttatgggatt ttggtagggt tattttagta gttttagatt 1140
tttttggttt aggattgttt tgggttttta aattttataa aatttttagg agtaaattgg 1200
gttaaatagt atttattttt ttatttttgt attatttgat taaagtgttt ttaaagattt 1260
aagtaggatt tagtcgatga gtaggattta gggtatcgtt atttttattt tcgaaatata 1320
tacggacgtt tatgtatata tattattatt tttcgtttag aaaaaaaaaa aaagaaaaag 1380
aaaaaaaaaa agtttttttg gggtaataga ggtatgagtt tggagggtgg tgtggtagag 1440
tgtaagggag ggaaggaggg ggtaaatttt aattttcgga tagtataggt ttaggtttaa 1500
gttttatata ttgtttagta ttatatattg gggatttgag aagttattag tttttattat 1560
atgatttcga gggtttttat tttttttttg ttaagtgggt aaagataatt ttcggtttcg 1620
ttagtttttt tgtatgggcg agtacgtttg ggtttttata gatttttttt tgaaatttga 1680
ggtttggttt tttttgaggt ttatagatga gtttataaac gaaatttggt ttagggtatt 1740
tattattgtt cggagtgaag gtcgaatttt tgaaaggata ttaaaaagag agagagagaa 1800
agagagatag atatagagag agagagagag agggagggag agagagagag agagagagag 1860
agagagagag agacgaaata gaagttacga agaagttttt tttagacgaa ttttttttta 1920
tttttgagtt taggagtagg tagggattta gggtattgta taatttttag agttttgttt 1980
cgtaattgtt cgtgttcggc gagggtcgtg ggtttcgagt cgcggtattt attttcgaga 2040
cgttcgttcg tattttagaa ttcgggttta aggatgttcg tggttcgcga gtttttttag 2100
gttttttttt agatcggata cgtagatttc ggcgggtaaa gatcggtttt aaattttttt 2160
ttttaggttt tttcgggagg ggtagtggtg aaagcgtttg cggagcggag ggaggttttt 2220
tttaattaga agtttatcgg atacgggttt gattagagcg cgttgttttt gggtttagcg 2280
tttggtttgg cgcggagagt gcgggggtag gggttagggg aggtaaagtt gtcggttgga 2340
aggagtagcg tgtttaaagg ttttttgtat aggttttgga gggagagaat cgagtggtta 2400
aggtagcgtt ttttttacgg ttagtttcgg ttcgggaggg gagcgtaatt ttgatcgtgc 2460
gttaagaggc gagacgcgtt ttgtgttttt ttttttcgtc ggcggttgta gagaagtcgg 2520
gaatcgtttg gtttggtcgg gtcgtgagag ttgaggggtg aaatggatag taggtttagt 2580
taggaaggtg tttagaaggt tgaaaacgat gattgtagtt gtattgtgat tggggaaaat 2640
ttgtttttta ataaaatatt tttttttgtg tgatcggaag agggagtttt tggttttcgg 2700
gcgtcgtagg ttttcgtatt tgattttagg gtggtttgat gattttaggt ttaattatgt 2760
tttagggttt tgcgagtttg cggggttgcg ggattagtgg attagtaggt aatttttttt 2820
tttttttttt tttttcgaga cggagtttcg ttttgtcgtt taggttgtag tgtagtggcg 2880
cgattttagt ttattttaat tttagttttt cgggtttaaa cgatttttgt gttttagttt 2940
ttcgagtagt tgggattata ggcgttcgtt attaagttcg gttaattttt ttttttgtat 3000
ttttagtaga gatagggttt tatcgtgttg gttaggttgg ttttaaattt ttgattttag 3060
gtgatttaat cgtttcgagt tatcgcgttc ggtttaagta agtttttttt tttttttttt 3120
taagttttgt atttagtttg gggttattta agtttttatt ttatatttgt ttttataaat 3180
tcgttaattt ttatatatgg attaagagtt tggtgttttt ttcgtaggtt agaagaaatt 3240
tattttaacg gtggggttaa gatttttgga aaaggagtag aaatatttta gtatggtttg 3300
ggtttaaatt ttatttttgt tatttaattt tatagaattt ggattttttt atttaaaata 3360
aggggttttt ttaggattag ttggtgagtt ttttgtaaag gttttcgagg ttattgtttt 3420
aattaatatt tagaatttag tgtttgatta agtaggcgat tgatgtttgt taaattaatg 3480
tttataagat taggatgtaa gatataatat ttaagaattt aaaatatgaa tatgtttgtg 3540

aagttttggg cggttgtgag tgggtattaa taattgtttt tgtttgtgta ttatggttag 3600
attgttttag ggatttagag acggttttga tttttttttt ttggggtttg gttttggcgt 3660
tttgatggtt atttttatat ttttgagagt tgatgttttt gttttttata gtttaagttt 3720
tgggttaggt tttgtatttt tggggaagta gtaggaattt tggaaattta aagaataaat 3780
ttagaatttc gagggttatt tttgtttatt ttaggatagt agtcggagcg ttttttatat 3840
ttaagttgtt taatgagttt taagggttgg gtaagatgga tttatttgtt tttattgtaa 3900
gataaaattt aaatttggag atggtgtttt taggttatat gatttgaatt tagggttttt 3960
tttttattgg gttttttttt tagggtggag aagaaggata tatttattta ttagttttgg 4020
ttttttttta attttttttt tatggtagtt acgtttgtat attatagaag aatttagata 4080
ttttttagaa gtgtaatgtt tgttggttgt aaaatttata gttttatttt ttacgatatg 4140
tgataagatt ttaggtatta gatttgtttt gaaaagggtt ggataaggga tttaaacgaa 4200
gcgatagaat ttaggtttta aaaatttttt agaagtatta aaaagataat ggcgtagtag 4260
tattttgtgt tttaggggta tggattcgat ggttttttaa tcgtttttaa atagtatata 4320
tgtagatagt gttttcggat ttattgtttt ttagttatag atgtttagat gggttgtcga 4380
gttttatatt taaagtttta agagggtggt gggtagcgtt tttgtattta tggagtataa 4440
ttttaagtcg atttaatttt ttaggttgtt tattttagtt gtttttttat agacggggat 4500
attgagattt agaaattttt taaaagtaat attagtttgt taaataaagg tggcgcgatt 4560
tgattaaaga atataagttt tagcgattag taagttgttt taacgttttt tgagtataaa 4620
atattaattt tattaattaa aagtatagag tggaggtaat ttttgggttt gtttgcggtt 4680
tttttatagg atagtgattt tagatttttt cgaagaaaag ggcggtttcg atttttttaa 4740
ggtttcgcgg gggtcgggtg tttttggtta ggttaaggta ggagcggttt gaagacgcgc 4800
gtttagaagg cgcgggtgaa ggcgggtaat aagggtagag tttttttttc gagttttggg 4860
cgaaggtatt ttttgtaaaa gatatatttt gttttttacg tattttaaaa atatttcggt 4920
ttttaggttt tcgagtaatt ttgttttagg aaaagtattc gttattgtat tagtaaagcg 4980
tttattaaat tatcgaatta aatcgaattg gtttaggttt ttaatagcgt ggaaatttat 5040
tgaaaataaa tgaagagggt aaattatagg ggtttcgtag gttcgggttc gcgtcgttta 5100
ggcgaaagag aggtgggcgg gtatcggcgc gggatgagaa attaatttga tatttatcgt 5160
gtgtcgagtt ttttttttgt attttgatta agtatagcga ataattttgt ttttgtttta 5220
attttaggtt ttgaagaaat attgttttag ttgagtttcg cgtttataag atgaagaggc 5280
gttttagatg cgttgaaaga aaggttaaag ttcgtgtttt tttttattgt ttgcggtaga 5340
atttggtttc gtatagtttg gattcggata agttaagttt ttttttattt ttagaatttg 5400
cgtggtcgtc gtttgagcga agtttagtga agatttattt ttgtattatt atacgcggga 5460
gttatttacg gatacgacgt tgttttagaa agttatttag ttattggagg tgtatgtgtt 5520
tattttagtt ttttacgata tttaatgtgt atttaagtgt gtgttaatat cgtattataa 5580
gggttggagg tgtttttgga tattttagcg ttgcggaggt agtatagtat aggcgtttat 5640
tttcgcgttg gttttatttc gaatttagag tttagacgtt tgaaaagcgt tacgtttggt 5700
gataatttgg cgttttttta attaataaaa ttttttttaa ggtagagtta gatattgcgt 5760
cggttttttg gttttatttt cgtggggatt agtagtttcg gaatttttcg gattttaagg 5820
ggttttagac gttttttcgg tttttgcgaa tagttaggtt tttagttttg tttggttttg 5880
gaggatgtcg gggttagtg tttgcgtgta tttttatttt tcggtttttt ttttagtcgt 5940
cgttttcgtt atttttacgg tttttgggaa gtagttgggt aagagttgcg gttaaaaggg 6000
gtaggagagg ggggtgtttt agagcggaga gtttttggcg ttattgtttc gcgcgtaggg 6060
ggcgtttttc ggtttattta ttttgattgc gaatagcggg ttggttttcg aggtagttgg 6120
gtaggtaggg cgcgttagga aatattttgg tttggtcgga tgacgtttgg ttgggcggag 6180
gaggatcgaa gggttcgtag cgataggttt cggttgtgtt agtgaattgg tcggaaaagt 6240
ggatagtgaa ggcgtttagg tattgttttt cgtgcggttt cgcgtcgttt tagttcggtt 6300
tttgtttgat gaaggagtga ggcggcggcg gcgggggtgg cggcggagtc ggtggcggcg 6360
cggggtcgtt taacgattcg taagtcgaag cgttcggggg cgtaaagttt agtatcggcg 6420
tttattgcgt cgcgtcgttt ataggtaggg tatagtcgtc gtcgttattt agggagggga 6480
cggcgggtag tagcgcgttt aggtttcgta cgtcggagtt tatttgttgc ggtttagatt 6540
cggacgtttc gcggtttttt cggttttgga gacgtttagc gttggtttcg gcggcgttta 6600
atttggttta gatgtcgttc gggtttcgga ttttttgttg ttttggttgt tgtaggtatt 6660
taggttcgga gcggagcgtg tgttgagacg tcggtttcgg gatatacgtg gaagtcgggt 6720
tttgtagtaa gaggaagttt aggatcgcgg cgaggagacg gcggggttcg ggcgtttggg 6780
ttgtcgtttc ggttttgggt gggtgggtgg gtgaatgagt aggtgggagg gagggcggga 6840
agtgggggag cggataggcg gtcgggttgc ggagagtttt cgggtgtggg cgttgttttg 6900
aattttttat tttagttgtt tggttgtttt tagtttttta aagtttaaat aagaggggtc 6960
ggcggcgcgg ggaagaggag gagttaggag gttcggtcgt tttatttatt tagtagttta 7020
agttcggtta ggtagcgcgc gttgtttttt gggggcgttt tcgtagttgg ggtgagggcg 7080
ggttcgaggg agcgtttttt tcggagatat tttttttttt aatttaatac gcgtttttag 7140
ttggtcggtg tggtataatt ttttttttat tttttttttt tggttttcgt tagtgtggga 7200
gtttcgtttt attggagagt tcgtttaggt aagtagtgag ttcgggtagg gtcgaatgcg 7260
gtgggagtag agatggggtt gttaatttaa aagagtggtt tggagggagg ggtaggtagg 7320

ttttaagagg gcgggaattt taggaaagcg tagaaagaat ttagttaagt tttaaagaag 7380
cgtatatttt tgttttgtat ttttgagttt ggtttttgtt tttagaattg tcgggtaggg 7440
gagtggttat gattttgggg ttattgggga aagaggtggt tttttgtggg ttttagaggt 7500
cgggattgaa agcgaacgtt ttttagttgg gtggtcggga gttgtattag ttataagtcg 7560
ggcgtatttc gatcggttag tagttgttta tttggtgagg tttgcgttag gcggaaggcg 7620
gttgtcgttt atttaaaatt ggagttagta gtatttagtt ttttttttta gtgaggaata 7680
tttaattttt ttacggttaa gtcgggaaaa tagcggttag tttaggaggg aagaaaattt 7740
ttgggtaaac gtttaaagtt tgcgtatatt tcggtcggag tattttggcg tttagtttgg 7800
ggaggagggg tgaaggggag ggtttggata cgaagttcgg tagggagcgg gtaatttttg 7860
gagttatttc gtcgagtatt gtttgtggtt ttggtcgagg atattatttt ggttgtttag 7920
tgattgatta ggaggcggaa gttagttaag gggattaagg agtggagtag ggagaatttt 7980
tatcggttta cgggcgcgag ttagtgttcg aatttttggg agttcgaaga gtttcgttaa 8040
atttgatttt tttcgtttag tttttgtttg gtagttttcg cgttttagag taggtgtttg 8100
gggcgggtag ggggtgtttt ggtttggttt ttggattttt taaaggagtt agtatttatt 8160
gggaaataat ttttaggtaa ggagaagaat cgggggtcgg agcggagcgt gttttagagg 8220
tgcgtataaa attttagggg cgttaggagg tagtagcgac ggcgtagcgt gggacggtta 8280
ggcgcgacgg gagggaagta atcggttgta gttcggtttt ttgtatttgg ttggttcggg 8340
ttggggttgt aagtttttta gttaaattta tgtgaagtaa agtagttgta gtttgataaa 8400
gaatggcgtt ttaataaatg atatagtaat tttttacgtt ttggttttta gtttgtattg 8460
tataaacgtt gtcgttatta aattttggtt gcggattcga agtgtgtgcg ggtttaggat 8520
ttattcgaag ggcgtatttt ttgggaatgc gtatttcgta ggcgtaggag attttgaggg 8580
taaagatgcg gtatttgagt ttttcgtttg ttttttaggt tatttttgtt gtttgatttt 8640
gcgtcgtttt tatttagaag tttttaaaat tattattaag aaaagaaaga ttttataaga 8700
tattgttttt acgttttttg tttagttttt ttttatacga tttattttta aatttttcgc 8760
ggggttcgcg cgtttggttt tttgagtgga gaaaggattt tcgagataaa atttattgtt 8820
agttgtttta agaatttttt agatttttgt attttttttt ttttttttcg tttattattt 8880
ttatttggaa gtcgtttttt ttaattagga tttaattgtg aggggcgagg agttttttat 8940
gaaaagtcgg ggatttttat ttttaaacga aaagattgag attgtttttt tttttattat 9000
ttttggtatt tttagcgggg ggagaggagg ttatttgaag ggaggttggt ttgaatgtga 9060
gtcgttgtgt ttgtgaaatt taggtaaggt aaagatacgt aaatttttgt taatattttt 9120
taaaaataag tttttgtttc ggggtgaggg atgtaggggt ggataggagg aaaggagagt 9180
taagtaaatg tagaattttt tttttttttg ttatttattt ttttagaatt tttgtggggg 9240
cggggagggg aagagtatcg gtgcgaattt cggggtgttt tttcgttcga tttggatttt 9300
ttcgattaaa gaggtgggag gagttttttg tagtttgcgg ggttaagaag aaagtgttaa 9360
gttgaggagt cgtagtcgga ggattttagg gattcgatag gaagttttcg tacgatttta 9420
acggatttat atgttcggaa gtttaggcga tattaagtta gcgttgggga attgacgtat 9480
ttttgtagtc gtagggcgtt ttatgttttt ttgttttttt ttttgttgtg ggtaacgttt 9540
tttgcgggag atttgaggtt ttttaagggg gatatgttag ttatattggt tttggcgttt 9600
tgagttaagt attaggattt atagtttagt acgaaggtag gtagtatttt tattcgtcgc 9660
ggacgatagg cgtttttttg tatttttttt agttcgcgag gttcgttttt cgcgaggttt 9720
tagagaaaag taggttgtgg aaaattgggc gttttttttt tttatttatt tttttatttt 9780
tgttagcgtc gagatttgta gtagaggttt ttggtttgaa ttatcgattg gtaaagaaag 9840
ttgtagatta aatttttcgt tttatagaga aggaaattga ggtttagata gtcgaaggag 9900
aggtagttta tggagcgtag cggtaaagag taaggggttg gtggttttag aatggaattt 9960
tagttttgtt aggtaattaa tagttgtgtg attttagacg agtttcgtag tttttttgag 10020
ttttagtttt tttattggtt aaacgggata atgggatatt agcgtttatt ttatagagtt 10080
gttttgagga ttagatagga gagtagtgtg ggtttttgat ttaattaatt ttatagtttt 10140
tgttattatt attgtttaag gataggaaat aagttagtgg taacgtcgag ttgaaattcg 10200
ggttttttaa cgtttagttg gaggttttat ttattatttt tttatttttt tagagttaat 10260
gggatgtaga ggcgaggata gtttttggaa aattatgtgg cgttgatata ttggtaaagc 10320
gtaggtattt cggtttcgaa ggtgtataat tattgtaata tgaaaaaatc gaggttgggt 10380
aggagtaggg tagtgaggat tttttaattt ttatttttat ttcggcgttg tttattgtat 10440
ttgatagagc gcggagttgg gttgttatag tcgtaatttt agggattagt gcgtacgttt 10500
gggtcgtttt tcgggagtta tttagcggtc gcggataatt gaataaatat tttaaagcgt 10560
tgcggtcggt taagggcggg gatagttata ttgcgcgcgg ggttcgtagg tcgtaataaa 10620
gagtggttcg atttcgttgt tgtttgtcgc gggagaggaa taagttttcg attagcgttt 10680
tttttcgggg ttcgcgtttc gagttttacg cgtaagatag tttaatagtt gttttttttt 10740
ttttcgtcga ttttaatttt tcggaatttg tttattcggg gttgtagggt aagtgtttag 10800
gatggttttt agtttcgcgt tggcggtgaa aattttaacg ttattttttt aattaaaaaa 10860
aggggggggt aagggagggg ttttgtgata attattttta gttttttttg attattttaa 10920
aattaagtcg atttttttta attagttttt atttattgtt ttaatttttt tcgttgattt 10980
tatttgggag ttcggaatcg ttaggtattg tcgaatgcgg tgtaaaattt tttttttttt 11040
tttagttcgt agtgtttgga gtcgttgggg ttattcgttt gttttgatgt tatcgcgaga 11100

tggttttcga gtttttcgag agtttttagt gaaaggattt cgcggtattg tttttattat 11160
tatatcgtaa atttttttaa attttggaat taattatata gaggatatgt tttaatttgt 11220
tttgtattaa tgttatagtg gggatggagg ttaggtcgtg gttagagtag atacgtaggt 11280
ttatgaaatt gatgaattga gagttgtttt tagttttgag cgtattattt ggaattttag 11340
tttgaggtta taattagaat ttttgatttt agatttaatt ttgtaaataa taggggaaaa 11400
aaatggggaa aagaaaaatt acgagagagg gaaaaaaaat tattttgtat ttgtaaaaaa 11460
aaaaaaaaaa aaaaaaaaaa aaaaaaagtt atataatttt aaaatttttt cgttttagat 11520
aatttttttt aagtattttt tttgtgtttt ttaatttata aaagataatg gaatgtttta 11580
tagattggat tttattttat tttttttttt ttttttaatt gtttttagtt taatagttgt 11640
tagttttatt ttagtgttga atttaattat ataggaataa aatatttttt agtaaatttg 11700
tttgttaaat attttgttat atttattttt tttttgagaa atggtttaat tatagaaatt 11760
tatttgtatt taggaatttt tttttttttt ttttttttga tcgttatttt agtttaatcg 11820
taagtagaga ttttatttgt gtagtaattt tatttgtgtt ttaaattcgt ttagttaatt 11880
tttttttttt ttttttagta atattttgtt tta 11913

<210> 151
<211> 4316
<212> DNA
<213> Artificial Sequence

<220>
<223> chemically treated genomic DNA (Homo sapiens)

<400> 151

ttgtagttaa taaaaaaaaa aaaagaaaga aagaaattgg tttttgtttt attttatatg 60
tttaggtata atttttttag agaagaagag gaggggggcg gggaggagta ggaggaggag 120
gaaagaagga ggagaaggag aaggagaaga agaggaagag gaagaggaag aagaagaaga 180
agaagaagag gaagaggaag aggaagaaga agaagaagaa gaagaagaag aagaagaaga 240
agaagaagaa gaagaagaag aagaagaaga ggaagaagag gaagaagaag aaattgtttt 300
tagattttta tttttaggat aagtttattg tttggtatta agttttttgg ggtgaatttt 360
tttttaaaag agttcgggga gtttaggtat ggaatgggag gtagaaagtt taattaaggg 420
attgggattt cggaatgaat aatgaaggga gatggattgg gtttatgtcg aaggtttttt 480
tttggttttt tagttttcgg gcgaagattt agggagatat tgagatatat tttgtatagg 540
agggggaggg ggagggggag ggtaaagttt tagggtttta ggagtggttt ttaagggttt 600
aggtttcgag gcggtgtttg gggttggaag gtttagtatt gagaattttt tattttttta 660
gagttttttt ttttttttta attcgtgtta ggttttttat tttggatatt tataacgcgg 720
ttttattttt tatttttatt gggcgtcgcg tttttagaga agttaattag tgtcgtcgta 780
gtttttaggt tttaaagttt tacgtatttc gcgggattta tatttttttt agacgcggag 840
gttggggtta tggcgtttcg aagttttttt ttgttgtttt taggggtttt ggttttgatc 900
gatatttggg cgggtgagtg cggggtttag agagaaacgg tttttgtggg gaggagtgag 960
gggttcgttc ggtgggggcg taggatttag ggagtcgcgt tcggaggagg gtttggcggg 1020
ttttattttt ttttcgtttt taggttttta ttttttgagg tattttagta tcgttgtgtc 1080
gcggttcggt cgcggggagt ttcgttatat cgtcgtggag tacgtagacg atacgtaatt 1140
tttgcggttc gatagcgacg tcgcgatttc gaggatggag tcgcgggagt cgtgggtgga 1200
gtaagagggg tcgtagtatt gggagtggat tatagggtac gttaaggtta acgtatagat 1260
tgatcgagtg gttttgagga atttgtttcg tcgttataat tagagcgagg ttggtgagtg 1320
aattcggtcg gggcgtaggg ttacgattat tttttattcg ttacggatcg ttcgggtttt 1380
ttcgagtttt cggattcgaa atttatttcg aggtagcgga ttcgtttaga ttttttattc 1440
gggagagttt taggcgtttt tatcgaggtt tatttttagt ttaggttaaa attttcgcgg 1500
gttgggcggg gagggggcgg ggttagttgg gcggggttga ttgcggggat cggttagggt 1560
tttatatttt ttagggaatg aatggttgcg atatggggtt cgacggacgt ttttttcgcg 1620
ggtattatta gtacgcgtac gacggtaagg attatatttt tttgaacgag gatttgcgtt 1680
tttggatcgc ggcggatatc gtggtttaga ttatttagcg tttttatgag gtagaggaat 1740
atgtagagga gtttaggatt tatttggagg gcgagtgttt ggagttgttt cgtagatatt 1800
tggagaatgg gaaggagacg ttatagcgcg taggtattag gggttatggg cgtttttttt 1860
attttttgta gattttttgg gatggtttcg tataaggttg ggaggaaagt gggtttaatg 1920
ttaggatatc gttttttttt tagttttgag taggaagaat ttttttggtt ttcgagattc 1980
ggtattagag agtgattgtg agagttcgtt ttgttttttg ggataattaa gggatgaaat 2040
ttttgaggga atggagggaa gatagttttt ggaatatcga ttcgcggttt tttttgagtt 2100
ttttaatagt tttgggtttc gtgatttttt ttttaagttt tgttttttgt tttatattta 2160
atgtgtttgg ggttttgatt ttagtttttc ggtttttatt taggttaggg ttagaagttt 2220
ttgtttttta tttagagatt cgaatttttt aaggaatagg agattttttt aggtgtttgt 2280

gtttaggttg gtgtttgggt tttgtgtttt tttttttatt ttaggtgttt tgtttatttt 2340
taggttggtt atatgggtgt tgttggggtt ttttatgagg agtgtaaagt gtttgaattt 2400
tttgattttt tttagatttt ttaaaggtat acgttgttta ttattttatt tttgattatg 2460
aggttatttt gaggtgttgg gttttgggtt tttattttgc ggagattacg ttgatttggt 2520
agcgggatgg ggaggaatag atttaggata tagagtttgt ggagattagg tttgtagggg 2580
atggaatttt ttagaagtgg gtcgttgtgg tggtgttttt tggagaggaa tagagatata 2640
tatgttatgt gtagtacgag gggttgtttt agttttttat tttgagatgg ggtaaggagg 2700
gagatgggta aagaggggaa cgagggttta tgtttttttt tagggaaagt aggagttttt 2760
ttggagtttt ttagtagggt tagggttgag gtttggagat tagggttttt tatttttttt 2820
ttttttttta gagtagtttt tttagtttat tatttttatc gtgggtatcg ttgttggttt 2880
tgttgttttt ggagttgtgg ttattggagt tgtggtcgtt gttgtgatgt ggaggaagaa 2940
gagtttaggt aggaaggggt gaggagtgga gtttgagttt ttttgtttta ttgggggttg 3000
taagttttaa gtagaagtgt gttttgtttt attattggga agtattattt atatttatgg 3060
gtttatttag tttgggtttt gtgtgttagt atttatttat ttgtaaagtt tttgtgaaaa 3120
tgaaggatag attttttatt tcgatgatta tggtggtgat gggatttgat tttagtagtt 3180
ataaattata ggggaaggtt tttgttgatg atagatttta ggagggtagt tggtttagga 3240
tttatatttg ttttttttat attttttgat tttgttttgg atttatagtt atattttttt 3300
ggaaattttt ttgggattaa agattagggg tttgttttag gattttatgg ttttgttttt 3360
tttttggttt tttataggat attttttttt tatagataga aatagaggga gttattttta 3420
ggttgtaggt aagatgaagg aggttgattt ttgagattgt tgggatattg tggttaggag 3480
tttatgaggg agtttattta ttttatagtt tttttagtta tatttgtggg ttttgattag 3540
gttttgtttt tgttttattt taattattga tagtgtttag ggttttgggg tgttttttat 3600
agttaataaa ggtgatattt tagggtaggg gttttgatgt gagtggggtg ttggggggga 3660
atagagggga tttagttgtg ttattgggtt tttttgattt ggatgttttg agtatgaaat 3720
gggttattta gagtgttatt ttttattgtg attgatacga atttgtttat gaatattttt 3780
tttatagtgt gagatagttt ttttgtgtgg gattgagaag taagatatta atgtagtaga 3840
attgtatttg tgttttacga atatatataa attttaaaaa taaagaataa aaatatattt 3900
ttttatagat ataggtagat atgtttttat agtatgtacg taaatgtgtg tgtgtgtgtg 3960
tgtgtgtgaa gagaaagagt gaatagagag attaagattt ttttaatggt gaaaagatat 4020
atatatattt ggaattagtt agtttgattt agtttaggtg attttaattt tggtggtaat 4080
aattaaagta tcgtagttag gagttagtcg aatatatgtt tttttttttt tattagattg 4140
aattagagtg ttgattttgg ttatattaat gttataaatt tttttatagt ttgtttgatt 4200
tggtgtttgt tggttttaat atttatagtg aatataagta ggttgttgtt ttttattttt 4260
tttatagttt atttagtggt tagcggaaat ttgatgataa tatggtggtt aagttt 4316

<210> 152
<211> 4316
<212> DNA
<213> Artificial Sequence

<220>
<223> chemically treated genomic DNA (Homo sapiens)

<400> 152

aagtttgatt attatgttat tattaagttt tcgttgatta ttgagtaggt tgtgaagaag 60
atagaaaata atagtttatt tgtgtttatt gtggatgtta aagttaataa gtattagatt 120
aaataggttg tgaagaagtt tgtgatattg atgtggttaa agttaatatt ttgatttagt 180
ttgatggaga gaggaaggta tatgttcgat tggtttttga ttacgatgtt ttggttgttg 240
ttattaaaat tgggattatt taaattgagt taagttggtt aattttaaat atatgtatat 300
ttttttatta ttaaaagaat tttaattttt ttatttattt ttttttttta tatatatata 360
tatatatata tatttacgtg tatgttataa aaatatattt atttgtattt ataaaaagat 420
atatttttat tttttatttt taaaatttat gtatgttcgt gaggtataag tgtaattttg 480
ttatattgat attttgtttt ttagttttat ataaggaagt tgttttatat tatagagaaa 540
atatttatga ataaattcgt attagttata gtgagaggta atattttaaa tagtttattt 600
tatgtttaag atatttaagt taaagaaatt taatagtata gttgagtttt ttttgttttt 660
ttttaatatt ttatttatat tagggttttt gttttggagt gttattttta ttagttgtga 720
gagatatttt agagttttgg gtattgttag tgattggggt agaataaaaa taggatttgg 780
ttagagttta tagatgtggt tagaggaatt gtggggtggg tgagtttttt tataggtttt 840
tgattataat attttaataa ttttagggat tagttttttt tattttattt gtagtttgag 900
agtagttttt tttgttttta tttatgggaa gaaaatgttt tgtgagaggt tagaaaggag 960
gtagggttat aaggttttag agtaaatttt tagtttttga ttttagagaa gtttttagaa 1020
aagtgtaatt gtagatttag ggtaggatta agaaatatga agaaagtaga tgtgggtttt 1080

ggattaattg tttttttgag gtttgttatt agtagggatt tttttttgtg atttgtgatt 1140
gttgggatta ggttttatta ttattataat tatcgaagtg aagaatttgt tttttatttt 1200
tataggagtt ttataaatga gtaggtgttg gtatataggg tttaggttgg gtagatttat 1260
gagtgtggat ggtgtttttt agtaatgagg tagggtatat ttttatttgg ggtttgtaat 1320
ttttagtggg ataagaaaat ttagatttta ttttttattt ttttttattt gagttttttt 1380
ttttttatat tatagtagcg attatagttt tagtgattat agttttaagg ataataaggt 1440
tagtaacgat gtttacgatg gggatggtgg gttggggaga ttgttttggg aaaggaaggg 1500
aaggtgaggg gttttgattt ttaggtttta gttttgattt tgttgaagag ttttagaagg 1560
gtttttgttt tttttgagaa aagatatgat ttttcgtttt tttttttatt tatttttttt 1620
tttattttat tttaggatga ggggttgggg tagtttttcg tgttgtatat ggtatgtgta 1680
tttttgtttt tttttagaag gtattattat agcggtttat ttttggaagg ttttattttt 1740
tgtaggtttg gtttttataa gttttgtgtt ttgggtttgt ttttttttat ttcgttgtta 1800
ggttagcgtg attttcgtag ggtagaagtt tagggtttag tattttaggg tggttttatg 1860
gttagagatg gggtggtggg taacgtgtgt ttttggagga tttgagaaga gttagaaaat 1920
ttaggtattt tgtatttttt atgggaaatt ttagtagtat ttatgtgatt aatttgagaa 1980
tggataggat atttggggtg gggaagggag tatagaattt agatattagt ttggatatag 2040
atatttggga aaatttttta ttttttggaa agttcgagtt tttgagtggg gagtagggat 2100
ttttggtttt gatttaagtg gaggtcgagg gattggaatt agagttttaa atatattgag 2160
tgtgaggtag agaataaaat ttgagagaaa agttacgggg tttaaggttg ttggagggtt 2220
taaaggggat cgcggatcgg tattttaggg attgtttttt ttttattttt ttagagattt 2280
tattttttaa ttgttttaga gagtagggcg gatttttata gttatttttt ggtatcggat 2340
ttcgaaagtt aggaagattt tttttattta ggattagagg gagggcgata ttttagtatt 2400
gggtttattt tttttttaat tttgtgcgag gttattttaa gagatttata ggagataggg 2460
aaggcgttta tggtttttgg tatttgcgcg ttgtagcgtt ttttttttat tttttaagta 2520
tttgcggagt aattttaggt attcgttttt taggtaggtt ttgaattttt ttgtatattt 2580
ttttgtttta tagaagcgtt gggtgatttg agttacggtg ttcgtcgcgg tttaggagcg 2640
taggttttcg tttagggaga tgtaattttt gtcgtcgtac gcgtgttggt gatattcgcg 2700
gaggaggcgt tcgtcgggtt ttatgtcgta gttatttatt ttttggaggg tgtgagattt 2760
tagtcggttt tcgtagttag tttcgtttag ttagtttcgt ttttttttcg tttaattcgc 2820
ggggattttg gtttaaattg aaaatgaatt tcggtaaagg cgtttgggat tttttcgggt 2880
ggagggtttg ggcgggttcg ttgtttcggg gtagatttcg gattcggaga ttcgggggga 2940
ttcgggcggt tcgtggcgga tggggggtgg tcgtgatttg cgttttcggt cgggtttatt 3000
tattagtttc gttttggttg tagcggcgga gtaggttttt tagggttatt cggttagttt 3060
gtgcgttggt tttggcgtat tttgtggttt atttttaata ttgcggtttt ttttgtttta 3120
tttacggttt tcgcggtttt attttcggaa tcgcggcgtc gttgtcgaat cgtaggaatt 3180
gcgtgtcgtt tacgtatttt acggcgatgt agcggggttt ttcgcggtcg ggtcgcgata 3240
tagcggtgtt gaaatatttt aaggagtggg agtttggggg cgaggagggg gtgagattcg 3300
ttagattttt tttcgggcgc ggttttttga gttttgcgtt tttatcgggc gggtttttta 3360
ttttttttta tagaggtcgt ttttttttgg atttcgtatt tattcgttta agtatcggtt 3420
agggttaggg tttttgagag tagtaggagg aggtttcggg gcgttatgat tttaattttc 3480
gcgtttggga aaaatatgag tttcgcgggg tgcgtgggat tttagaattt gggaattgcg 3540
gcgatattga ttggtttttt tagaaacgcg acgtttaatg ggagtgagaa atggggtcgc 3600
gttatgagta tttaggatga aggatttgat acgggttggg agagaaagag aaattttggg 3660
gagatgggga atttttaata ttgagttttt taattttaga tatcgtttcg gggtttgagt 3720
ttttgagagt tatttttggg gttttgggat tttgtttttt tttttttttt tttttttttg 3780
tgtagggtgt gttttaatgt ttttttgagt tttcgttcgg gggttgagaa attagggaga 3840
aattttcggt atggatttag tttatttttt tttattattt atttcgaaat tttagttttt 3900
tgattgaatt ttttgttttt tattttatat ttggattttt cggatttttt tggaagaaaa 3960
tttattttaa ggagtttggt gttagataat gaatttgttt tgagaatgaa ggtttagaga 4020
tagttttttt tttttttttt tttttttttt tttttttttt tttttttttt tttttttttt 4080
tttttttttt tttttttttt tttttttttt tttttttttt tttttttttt tttttttttt 4140
tttttttttt tttttttttt tttttttttt tttttttttt tttttttttt tttttttttt 4200
tttttttgtt ttttttcgtt tttttttttt ttttttttgg aaaagttgta tttgagtata 4260
tgaaatagga tagagattag tttttttttt tttttttttt tttttattag ttgtag 4316

<210> 153
<211> 14147
<212> DNA
<213> Artificial Sequence

<220>
<223> chemically treated genomic DNA (Homo sapiens)

<400> 153

gggggtattg agtaatatat ttagattaat aatattttat aagtgtgagt attattgatt 60
taaatttttt tgttggttgt gttttgtgtt tatgtttgtt ttatatttat tttttttaga 120
gttgtttttt ttagagtttt agtaattttt atttttttaa atttttttag ttatgggaag 180
gattagtagg tgttagtgga tttaaaagaa ttttagattt gttgagattt ttgatttttg 240
gtgtttagat taattagtaa atagtttaat taaacggttt gtttttatgg ttaatataat 300
tttgaattgg tggttattta gattaagttt attttgtttt tgagacggaa ttttgttttt 360
gtcgtttagg ttggagtgta gtggtgcgat tttggtttat tgtaattttt gttttttggg 420
tttaagggat ttttttgttt tagtttttta agtagttggg attataggta tttattatta 480
ttttcgatta atttttgtat ttttagtaga gatagggttt tgttatgttg gttaggttgg 540
ttttaaattt cgattttaga tgatttgttt attttagttt ttttaagtgt tgggattgta 600
ggtgtgagtt atcgcgttta gttaaagttt agtttgtata agaaggtttt ttttgttatg 660
ttataggatt tttgagttta ttaaatagat tttaatgttt tgagatttta cgtatttttt 720
taaaatgaag tatttaaatt gttagtgttt gtttttttgt ttggtttttt tgatgtattg 780
atttggtttg ttagtgtgat gaaaaatatg gttgagtttt attgttttat tgtaattagt 840
aggattgttt agaagtttaa tttttttaag agatgaagat ttgagtttat gtagttggtt 900
gttttttaaa aatatttggt tatttgggat atattttgaa ttaatttttt ggttatttta 960
tgtgattaaa agtgttagtt ttttttgttt ttagtatatt tttttgtttg ggaagtggaa 1020
gggatgaaat ttttatattt ttttttgatg atatagaaag gagtatatat ttatatttta 1080
tgaatagata tgtaattata gaagaaatag tttaacggtc gggcgcggtg gtttatattt 1140
gtaattttag tattttggga ggtcgaggtg ggtggattac gaggttagga gtttaagatt 1200
agtttgttta agatagtgaa atttcgtttt tattaaaaat ataaaaaatt agtcgggaac 1260
ggtggtaggc gtttgtaatt ttagttattt aggaggttga ggtaggagaa tcgtttgaat 1320
tcggagggtg gaggttgtag tgagtcgaga ttacgttatt gtattttagt ttgggcgata 1380
tagtgagatt ttattttaaa aaaaaaaaaa aaagtataat tatatgatta tttatattgt 1440
tttatatatt aaattagtag taatttgttt tttatatagt aaataaaaat aaattagtag 1500
taataaatat attttttttg tgttatagtt agtatttggt gaattttgta tttttttttt 1560
ttttaaagtg ttgaatttat aggtttttat aaggtaagtt tatattatta attaattttg 1620
tttttttttt tgatgttaaa attgtttttt aggagttgat taggtttatt tttttgtttt 1680
ttaagtatta tttttagaat atttttgaaa gtatttttgt tttttggtaa taatatttat 1740
attttattta ttttttgttt ttaaatatat atttagttaa ttttaggttt tttttaatgg 1800
agtagttatt tatttattta atatacgttg ggtgtttttt ttgtgttata tattgttttg 1860
gtttttgggg atagagtagg aaataaaaaa aaataaaatt tgtttttata tagttgatat 1920
tttagttagg ggagataaat aataaataaa tgaatgatgt agtatgttag atgttgatag 1980
tgttatggag aaagtatatt atggagatta gaagtattat ttgtaatgga atagttatga 2040
ttttaagaag aatggttgat tggatgtgaa gtgtgagaga gaggagttta ggtttttggt 2100
tgagtaattg gagggatgga gttgttatta tttgagatgg agaagttagg ggaggagtag 2160
atttggaggg gaaggttagg aatttagttt ggaatttgtt aagtatgagg tatttattaa 2220
atattgaagt ggatttttag aagatgttat taaaattagt taagtttagg agagagtttt 2280
gggttagaaa taaaaatgtg ggagatattg gtatatatat gggtttatat tatttttttt 2340
tttaatttaa tattttattt tattgttttt tgttttttta atagtattta gttttattta 2400
ttttttaaat tgatttttat attgatagta taataaaatt tatatttttt attatattaa 2460
gattttgtgt gatttaattt ttttttattt tttggatttt gttttaggtt attttttttt 2520
tttatttaat ttttaatatt ttgattttat tgttttttaa atagtttttt gttgtttttt 2580
gttttttttg tggaaaaggt ttttttattg tttaattatt aggtattttt cgattttttt 2640
ttatttttta tttttgagaa aggttttttt tgatttttta tattaattag gatttttttg 2700
ggcgagattt gatttaaatt ggtttagaga aaaggagtta attgattggt ttatgtaatt 2760
taattttttt tttttgttgt aatttttttt tggtttttat tttatttaag gttaaagtta 2820
gggtttttat tatggttttg taggttttat atagtttgtt ttttttatta tttttttgat 2880
tttatttttt atattttttt tttatttatt tttttttagt tagagtggtt tttttgttgt 2940
attttaaata tgttaggtat atttttattt tagtgttttt gtgtttgttg ttttttgtgt 3000
gtggaatata ttttttatat agttatatta tttaaaattt attgttttta agtttttatt 3060
taaatgtttt ttcgtgaagt tatttttgag ttttttttaa aattgtagtt ttttattttt 3120
gtatttttta ttgtttttta tttttaattt ttgttattgt agttagaatt atttgatata 3180
tagtttttat tttattggtt ttttgttatg ttttttttta taagaatttg aagtttatta 3240
ggtcgggagt tttgtttgtt gtgtttgttg ttatttttta gtgtttaaaa ttgtttggta 3300
tatagtaggt atttaataat ttttgaatta gtgaaaatta gatggtggtt tggtattttt 3360
atataggaat gagttaggtg gaaattattt aggatataag tagattttga agtgataagg 3420
aagggttatt ataattatgt ggggtttatt ttgttttttt ttgttttttt tttttaggtt 3480
tagtaatagt tttattaagg attttatgaa tattaaagtt tatatttata tgttgttaga 3540
ggtgagagta gtttatttta ttattagatt ttgtgtttag ggtggtgatt tgaagaagga 3600
agagagcgaa agaagggaag gattattttt ttttttaaat tggagttaag ggagggaggt 3660

tagagtaagt ttgggggcgt aatttagatt tagtttttgt ttaatttttt ttgttttttt 3720
ttttaggggt ttagagaatt ataaggtttg tagaattttt tagagaagtt ttattattga 3780
tttttttttt ttatttttag atattaaaga gtttgaatgt tttgagttta tatggttttt 3840
tttttttttt ataaattttg ttagttgtta cgggggtttg tttttagggt ataaagttat 3900
tgagagattt agagatttag tttttttgtg gaatttttaa aatgttttag tagttttgtt 3960
tttagtttgt tgtttggtag tatttgttag ttgattttat gtattgtttt tttttttgtt 4020
attttggtaa tttgggtttt ttattttatt ttttttttta tttttttttt tagacgttat 4080
tttttgaatt gttatagaga atgggttaat ttatggtgga ggtttgttta tttttataat 4140
aattaaaggg ttttaaggtt tacgttttta tatatttaat tattttttag gtattttaaa 4200
ttatatttaa attttaattt agttttattt tgttttagtt tttgtttgta agtgttttag 4260
tttttaatgg gtatagattt ggatttcgtt ttttttagtc gggtttttat tttaggttga 4320
gtttagttat cgttaattaa tgtagagtta ggtttttttt ttttttaatt ttggtcgtag 4380
tttaattttg ttttttgaaa gtatttcgtt tttgttattt aatgtttttt cgtgtgtttg 4440
atagttattt tgttttttta ttttcgttgt gttttaaatt cgtttttatt tagttttatg 4500
gttttgttta atcgtagagt tgttttgttc gtttacgcgg agtttagttc gatttatttt 4560
cgtcggttgt tacgtttatt tttatataag ttttgttttc gttgagtagt atggcgtgcg 4620
atatcgtttt tttggtgttt tggtaggggt ttagaagttt ttcgttcgtt aattagagaa 4680
aaatagggtt atttattttc gtttttgggg gttgtttttt attaatttta tgtaagttaa 4740
ttagtgtgag gtagattttc gttttttcga tataggtttc gagttttttt agttgtttat 4800
agttcgttag tttttgagtt aatcggcgtg gagtatcgtg aaggtcgaac gcgttttttc 4860
gggattttag gggtcgtgag cgttttatta ttttttttta cgttaattac ggtatagttt 4920
tgtagggaag ggttcgtttt ttaatttttc gaggttttgc ggtcgattaa tagcgttttg 4980
gttaattagc gagcggcggg atattgggtt tttttttttc gggtttacgt gagttgtagg 5040
gaaacgtagg ggcggttttt aggtgttgtc gtcgttatcg ttattattat ttttatcgtc 5100
gtttcggaat ttaggtttgg ggggcggtgg ggtcgcgtat ggagttttcg tttttcggag 5160
ttgttaatat tgttaacgtt atcgttacgt tatatatagg tgagttttgg gtttggaggg 5220
tggagggttt agttcgtgat tttacgtatt tttttcgttt tcgcgtagag gatgtggttt 5280
ggtcggtggt ttgttggtgt tcgatttttc gtcgttatta ttacggttgg tggatttgcg 5340
tgtggtattg tttaagtttt tcgtttttta tagtggattt gatcgtggtt taattttttt 5400
ttttcgttgt ataatggatc ggtttgcgtg tttatgtttt tttttacgtt aatttagggt 5460
ggattcgttt ataggttttt tttttatttt tatttttatt cgttttttat ttatttttat 5520
ttatagggag ttagtttgtg atagtgttta gtttttttaa tagtaggtgt atttgagtgt 5580
ttggattttt tttagtttta atttttagga gattcgttcg tggttttatt tattttattt 5640
tttttgtata tcgtagcgaa ttaattcgtg gcgtcgtatt ttttcgtatt tttttttaat 5700
agtgagttta tttgagtttg tatttttgtt ttttttagtt ttttaatgta ttagtttatg 5760
gtttttttta aaattatttt ttttttttat tattattatt attattttat agtggattag 5820
tttttatgtt tggatttacg tttttttagt tattagttta ttgttttgtt tttttttttt 5880
cgtagattgt cgttttagcg tttggggttt tttttttttt aatagtgggt tagtttagtg 5940
ttttatattt ttttattttt agtagtttag tttatagttt tttttttttt tgttttgaat 6000
tagtttgtgt gatggttttt tttttttttt attgtggtgt aggagtttgt aaggtttttt 6060
gttttttttt tttttagacg atgagtttta ttattttttt ttttgtagtg gatttgttta 6120
ttggtttttt ttttttatat ttttgtatgt ttgtatagtt tttcgtatat tttttcgtga 6180
gtttagtgtt ttgtaattgg gggaagatcg gtcgtgtatt ttttaatttt tttttttttt 6240
tttttttttt ttagaagaga acgtgttgat tttttttttt ttgtgatgga gtattgtacg 6300
gttttttttg ttttttattt tttaatagtg ggttagtttg gttatattcg taattataaa 6360
gacgttttgt tttattataa taagtgaata tattagcgtt agtgatgata gtaatgttaa 6420
taggagttag cgtttattga gtatttgtcg tatattaggt agagtgtttt atatgaatga 6480
gtttttttaa tttttattat aattttatga gataggtagt gagggaattg ggttataggg 6540
aggttaaatt ttttatttaa aggtggttta ggaagtattt aggatatagg ataatttttg 6600
aggttttttt tttagatttt attttatttt acgtgaggtt ttagttttta aaaattaaat 6660
tagtttttat ttttaagttt ttgtttttgt tgtttttttt gtttgtagag tagtttttat 6720
tttttatttt tttttagtat ttatttattt tttaatttta gtagttgtgt gattttagat 6780
gagtgattta attatttttt ttttgtgttt ttttttttat ttgtgaaatg ggggattaga 6840
attagaattt tttttagtgt aaataaatat taattgtgaa ttaatggtaa aataagtaat 6900
ttttttagta ttatcgttta tattttttta tttttttatt gtgttttaat tatattggtt 6960
ttattatata gttttttaaa tatagtaggt atgggttagt tttagggttt ttgtattggt 7020
tgttttttgt gttttgaata ttatttttag atttttgtat ggtttttttt agtattttgt 7080
ttaaatatta atttttaaag gatgtttatt atattgtggt gtgtggaatg tagtataaaa 7140
agatatatgt atgaaagatg tttttaagaa tgttagttta tatatgagta aggttttttt 7200
ttttttttaa aaggatgtgt ttataagatt gaggtacgta atatgttttg taaaggtata 7260
ggtataaaag ttgtttttaa gagtgttagt tgtatatgtg aataaggatt gtgttgttgt 7320
attgaaagaa tgtgtttata aaattgtggt gtgtagttgg gtataggggt gtgtatttat 7380
agttttagtt attcgggagg ttgaggtggg aggatagttt gagtttagga gttttaggtt 7440

agtttgggta atatagcgag attttttttt ttgaaaaaaa aaaatttaaa aaataattat 7500
tgtggtgtgt gtaatatatt tttttaaagg tataggcgta aaagatgttt ttaagagtga 7560
tttgattttg ttgtatttgg gtagtgttat tttattaaag gttgattgtt atattatggt 7620
atttagaata tattatataa aggtatatgt ataaaaaaga agtttttaat aatgttattt 7680
gtaaaaataa aaaaagttaa tttttttttg attatttgaa gttgtatatt tagtagggtt 7740
taagttaggt ttttgatttg tttcgttgtt ttttattatt gtttgatata ttgtgtgggt 7800
tatttgtttg ttgtttgttt ttttttatta gaatgtgagt tttatgagga tttagttttt 7860
gtttgtttat ttatcgttat gtttttagta attgaaaatg tattaggtaa atagtagatg 7920
tttaataaga atttatttta tgaataaatg ggaaaaagat aatttgagat attagtgtag 7980
gggttatatg aggaagaaga agggatatga gagttaatta gttgggattg tttttgtaat 8040
taatttttat tgagtttttt gtgagtatta ggtatttttt taggtgttgg aatatggtaa 8100
ggaacgaaat agggcgaaat atttgtgttt gtggagttga tatttattaa aagttgggga 8160
ggttgggtac ggtggttaat attagtaatt ttagtatttt gggaggttga ggtgggtgga 8220
ttgtttgagt ttaggagttt aagattagtt tgggtattac ggtgaaattt tatttttatt 8280
aaaatataaa aaaattagtt gggtgtggcg gcgtgcgttt gtaattttag ttatttggga 8340
ggttgaggta ggagaattgt ttgaatttag gaggcggagg ttgtagtgag ttaagattgt 8400
attatgtatt ttagtatggg tgatagagcg agatttgttt ttaaaaaaaa aaaaaaaaaa 8460
atttaagttg ggagatagaa ttatgtgaat aaaataaata agtaaaatta gtataataga 8520
taaatgttga gaagaaataa tgaaataggg taggtaggaa tcgagtattg ggggtggttg 8580
aaatttaggg tatagggaag gtgatttttg aattagggtt ttacggtaga gacggagaga 8640
gttttaatgt ttacgggaag agtatattag gtagaaggaa gtgttagttt agaggttttg 8700
aggtgggatg gtgtttgtta tgtttaagga atattaaggc ggttagtgtg gttggagtag 8760
agtgcgggga agggtagaag gtgaggttat ataggtgatg ggagttagat agtgtaggtg 8820
atatatagga taggattgta ggggtagggg gagaaggatg atatattagt taatagttaa 8880
tatttgtgga gtatttgtta tatattagag gtattttttt gtttaaatat taatttttaa 8940
aggatgttta ttatacggtg gtgtttggaa tgtattataa aaagatatat gtgttaaaga 9000
tatttttaag aatgttagtt tatatatgaa taaggatttt tttttttttt aaaaggatat 9060
gtttataaaa ttgaggtatg taatatgttt tatggaggta taggtataaa agttgttttt 9120
aagagtgtta gttgtatatg tgaataagga ttgtgttgtt gtattgaaag aatgtgttta 9180
taaaattgtg gtgtatagtt gggtatagag gttgaggtgg gaggattatt tgagtttagg 9240
agttttaggt tagtttgtgt aatatagtga gattttcgtt ttcgaaaaaa aatttaaaaa 9300
ataattattg tggtgtgtat aatatatttt tttaaaggta taggtataaa agatgttttt 9360
aagagtgatt tgattttttg gtatttgggt agtgttattt tagtaaatat ttattgttaa 9420
atattatggt gtttggaata tattatataa aggtatatgt ataaaaaaga agtttttaat 9480
aatgttattt gtaaaaataa aaaaaaaagt taatttttta agttttttat agaagtattt 9540
tatagaattg atatagttaa tttttataga agttttggag ggtaggtgta tttttgagaa 9600
tatttgattt tgagagtaaa atatttttat tttatagatg aggaaataag taaagagagg 9660
ttgagtaatt tgtttagggt aattttgagt tagaatagtt agaatagggg tagaggaata 9720
taaggttgaa gatttagaaa gaagaataga tttgtaaggg taggtaggtt atttggtatt 9780
tttgattacg tagaggaaga atgaattttt tttggaatgc gatgattgtg taattgagtt 9840
tatgaagtga gggaagagtt ttttttgcgg ggagagtagt tcgtgtttat ttttagaggt 9900
tggaattgtt tcgggaacgg tagatagttt agggtggttg ttttagagtt tttgtatacg 9960
tcgttcgttt cgtttgaagg ttttttttta tagagggtag tttggtttgt tatttttttt 10020
tttttaggtt aggtgttatt tttttgggga agaatttttt gtatattttg tgtaaaaata 10080
gtatttttgt tatttttttag gatttttattt tttttgttt tttttatgg agaattggtg 10140
gtgtaaggcg attgtttatt agagttaaat tatttaggtt ttgaatttcg gttttgttaa 10200
tttttagttg tgtgatttgg ggtaggttat ttttttgtat ttttattgtt ttatgtggaa 10260
aatgggggaa atgataattt ttatttttta acggtggttt gaggatttaa tgagttaaat 10320
gatttaaaga gtgtgtagtt tacggtaagg atcgtatgtt tggtttgtat ttttaaatta 10380
ttaaaatgta tttatagtat attcgtagat gtttgatagg tttttgaaat tgattatttt 10440
taaaattggt gtgatttttt ttaaaattta tttttttgta aggtttttat tttagttgat 10500
tataatgttg tttttgtggg tgattaggtt ataaatttag atttattttg gatttttttt 10560
ttattttttt cgggtatgtt atttgttagt aagttttatt ttgtgtttag gtttttttta 10620
gattttagtt atttttcgtt agttttattg ttattattat tatttatcgt agtggttttt 10680
ttagttttaa tagtttgttt tttttataat cgttaggggg atttgttgaa aattttagtt 10740
atagtttgtt atttttttgt ttataatttt tttgtggttt ttgttttatt taatattttg 10800
atagtattcg aaaaagtttg gtttagtttg gttttcgtta ttttgttttt ttttttcgtt 10860
gttttttttt tttttttcga atttatgtta gttttttttt tttatgttat ttgttaggta 10920
ttttttagtt ttgggttttt tatatcggtt gtcgttttta tttggtttgt tgtttttaga 10980
tatttacgag tttagttttt ttattttttt ttggtttttg tttagttgtt ttttttttgg 11040
cgattatttt tttttgattt tttatgtaaa agtaatagtt tttttttgtt tgattttttt 11100
tttataattt ttattattat ttgacgtatt tttgtatatg tttgtttttt tttttatttg 11160
tttttttttgt taagatgtga gtttttttgg agtaggattt gttgtttgtt gcgttttcga 11220

ttgtattcgt agtatttggt ttatagtatg tatttattaa atgtttgtta agtgaaggag 11280
atggaaagaa gtaaatatat ttaagtaagg tagattatta gaatttttat gaggggttaa 11340
gtgggagaaa taataaaata taaagttttg tgtgttgttg aagttggaag tttatcgtta 11400
ttttttattt agttgatagg ttttgttgta gttgaaagaa gaattggtaa aattgtttgt 11460
ttttgttatt aggagttatt ttgaattttt tggatttttg aaataagttt gtttgtaaaa 11520
taaatttagt gaggtttttg gtgataggat tttttaatcg gatattttat ggttttgtga 11580
tttgtataat tgaataggaa gtttatgttt atttttagtt tgtttttagg aaagaaagta 11640
gtaatggggt gatagggagg attttgtttt ttgaaggata tgttgagagg gaatagtcgg 11700
tgtgaggggt cgtttagttt aatggtgaaa tgtttagtta attggaattt gattgtttgg 11760
ttttaaatgt tggttttgtt atatgtggtt tgtggaaggt acgtagatat ttgtgttttc 11820
gttgttttat ttataagtta atgataggaa gattatttat tttttagagg tatgattagg 11880
gataaataag ttaatgttta ttaaaaaaaa aaataatagt ataggtgtat atgtttttat 11940
ttaaaatttt tggggttaga tgtgttttag aatttttatt tttttatatt ttagaaaggt 12000
aatatgatgt atatacgatg ttatataata ttttttagta agttttgtaa tcggttgtgt 12060
ttatattttt gtagtaaaat gtatgaatat tcgtaataag tgagatttaa taaagattag 12120
aaagggtttt gttttatttt aaattaggtt ttgttattaa aagagttaaa agaagttttt 12180
tgtttttaga gtattttgga tgttggaatg gtagataagg gattatgaat aaattagaaa 12240
ttaatattta gagtttatta tatattttag gtatataata tttaatttgt ataatatttt 12300
tgtgaattag gtattgttat ttttatttta ttaatgggta tattattatt tttgttttat 12360
atatgagaaa attgaggtat aaagaggtta aagaatatta gttaagcgtg gtgttttatg 12420
tttgtaattt tagtatttta ggaggttagg gagagaggat tgtttgagtt taggagttta 12480
agaatagttt gggtaatata gtgagatttt attttttaaa aaaagaaaaa gaatatgttt 12540
agggttatat agttaataag tggtagaatt aggatttaaa tattggtaat ttggttttag 12600
tgtttgggta gttgtttagt aggttatatt gtttttttgt ttagaagagt gtttgatgtg 12660
tattaagttt ttaatagatg ttaattattg ttgttattaa gaaagagaat atttagggtc 12720
gagtacggta ttttatattt gtaattttag tattttggga agttaaggtg ggaggtgggt 12780
ggattatttg aggttaggag tttaagatta gtttggttaa tatggtgaaa ttatgttttt 12840
attaaaaata taaaaattag tcgggcgtgg tcgtgggtat ttgtaatttt agttatgcgg 12900
gaggttgagg tatgagaatc gtttgaattt gggaggtaga ggttgtagtg agttaagatt 12960
gtgttattat attgcgtttt agtttgggta atagagagag attttatttt aaaaaaaaag 13020
aaaaaaaaaa aaagaacgag aatatttagg gatagtaata ggatagagag attaggtatt 13080
tatttagttt taggacggtt ttataaagga aaggattttt gggttgggat ttgaaagata 13140
agaaagattg ttaggcgaaa gaaggaagtt ttacgtggag tgagtagttt ttgtaagggt 13200
ttggtaaaaa aaaaattttt tgaaaaattt gatattgata tggtagaaga tatagataat 13260
atttaaatga ggaggatgga attagttttt tatttttgtt gtaataaata attataaatt 13320
tagttgttta aaataatata tttgttattt tatagttttg taggttagaa gtttatattt 13380
ggttttagtt gaaattaagg tattggtagg gttgcgtttt ttttggaggt tttaggggaa 13440
aatttatttt ttgtttattt aagttgttgg tagaatttaa ttttttgaga ttgtaggatt 13500
gaagtttttg tttttttatt gtttgttagt tgatagttat ttttagtttt aagaggttgt 13560
ttatagtttt tgattttgag tttttttttt ttatttttaa agttaataat ggtaggttga 13620
gtttttttta tattttgaat tttttttgtt ttttttgtta ttatattttt gagttatttt 13680
ttttttttta ttatttgttt tttagagttt atatgattag attgaatttt attaaataat 13740
ttagaaataa tttttttgtt ttagggtttg taattttaat tttatttgta aagttttttt 13800
ttgttatgta gtgtaaataa tatataggtt tcgaggatta ggatgcggag tagggttggt 13860
attatttggt ttattatagt ttgttttttg atttttatag atttatattt gttttatatt 13920
taaaatatat ttatattatt ttaagatatt tatgagttgt attttattat agggagtatt 13980
ttaagtaaga ttggggagtt agaagttagt ttgggaaggt ttagggaatg atggtagggt 14040
ttgaagatag agatggagaa aagatgataa aaaaaggaat ttttttttcg ttgatttttg 14100
ttttttttat gtttatagtt ttttaattta gttgtttgtt ttttagg 14147

<210> 154
<211> 14147
<212> DNA
<213> Artificial Sequence

<220>
<223> chemically treated genomic DNA (Homo sapiens)

<400> 154

tttggagagt aaatagttga gttgagaggt tgtggatata ggaggggtag aggttagcgg 60
gaggaggatt tttttttttg ttattttttt tttatttttg tttttaaatt ttattattat 120
tttttaaatt tttttagatt aatttttagt tttttagttt tgtttgggat gttttttgtg 180

atgggatata atttatgggt attttaggat ggtgtgaatg tattttgaat gtgaaataaa 240
tgtgaatttg tgggggttag agggtagatt gtggtaggtt aaatgatgtt aattttgttt 300
cgtattttaa ttttcggaat ttgtatgtta tttatattat atagtagaaa ggaattttgt 360
agatgaaatt aaggttataa attttaagat agggagatta tttttggatt atttgatgaa 420
atttaattta attatatgag ttttgaaaag tagatgatgg agaaagaaag gtagtttaga 480
gatgtgatga tagaagaggt aggggagatt taaaatgtga gagggattta atttgttatt 540
gttggttttg aagatggagg aaggaggttt agaattaagg attgtgggta gttttttaaa 600
gttgggaatg gttgttagtt gatagatagt aaggaaatag ggattttagt tttataattt 660
taaggaattg gattttgtta ataatttgaa tgagtaggaa atggattttt ttttagagtt 720
tttagaagga acgtaatttt gttaatattt tgattttagt tgagattagt gtgggatttt 780
tgatttatag aattgtaaga taataaatgt gttgttttaa gtagttaaat ttgtgattat 840
ttgttatagt aagaatagaa aattagtttt atttttttta tttgaatatt atttgtattt 900
tttattatat taatgttaag ttttttagga ggtttttttt ttgttagatt tttgtaaagg 960
ttgtttattt tacgtggggt tttttttttt cgtttagtag ttttttttat tttttaagtt 1020
ttagtttaaa ggtttttttt tttatgaagt cgttttaaaa ttgaatgaat gtttgatttt 1080
tttgttttgt tattgttttt gaatgttttc gttttttttt tttttttttt tttttttgag 1140
atggagtttt tttttgttgt ttaggttgga gcgtagtata gtggtataat tttgatttat 1200
tgtaattttt gttttttagg tttaagcgat ttttatgttt tagtttttcg tatagttggg 1260
attataggta tttacgatta cgttcggtta atttttgtat ttttagtaga gatatggttt 1320
tattatgttg gttagattgg ttttgaattt ttgattttaa gtgatttatt tattttttat 1380
tttggttttt taaagtgtta ggattatagg tgtgagatat cgtgttcggt tttgaatatt 1440
tttttttttg atgatagtaa taattaatat ttattgagag tttaatatat attaggtatt 1500
tttttaagta gagaggtaat atggtttgtt ggataattgt ttaggtatta gggttagatt 1560
gttagtgttt gaattttggt tttgttattt attagttgtg tgattttggg tatatttttt 1620
tttttttttt aagaaatggg gtttattat gttgttaggg ttgttttga atttttgggt 1680
ttaagtaatt tttttttttt ggttttttaa agtgttagga ttataggtat gaggtattac 1740
gtttggttga tattttttaa tttttttgta ttttaatttt tttatgtgta aaatagggat 1800
aatgatatgt ttattagtaa aatggggata ataatattta gtttatagag atattgtata 1860
gattaaatat tatatgtttg aggtgtatga taagttttaa atattagttt ttgatttgtt 1920
tatagttttt tatttgttat tttaatattt aaaatgtttt aagggtagaa agtttttttt 1980
aatttttttg gtggtaaaat ttgatttgaa atgaaataag gttttttttg gtttttattg 2040
aattttattt attgcgagtg tttatatatt ttgttgtaga aatatgaata tagtcgatta 2100
tagagtttat taaggggtgt tatgtaatat cgtatatgta ttatgttatt tttttgaaat 2160
gtgaagaaat ggaaattttg aaatatattt ggttttaggg gttttgggta agggtatgta 2220
tatttgtatt gttatttttt tttttaatgg gtattgattt atttattttt gattatgttt 2280
ttgagaagta ggtagttttt ttgttattaa tttataaatg aggtagcgga ggtataaatg 2340
tttacgtatt ttttataggt tatatgtggt aaagttagta tttggagtta ggtagttagg 2400
ttttagttga ttaagtattt tattattagg ttaaacggtt ttttatatcg attatttttt 2460
tttagtatgt tttttaaaga ataggatttt ttttgttatt ttattattgt tttttttttt 2520
gagagtaaat tggaaataaa tatgagtttt ttgtttaatt atataagtta taaagttatg 2580
aagtgttcga ttagagagtt ttgttattag agattttatt gaatttgttt tgtaggtagg 2640
tttgttttag aagtttaaag agtttagagt gatttttggt ggtaaagata ggtaatttta 2700
ttagtttttt ttttagttgt aatagaattt attagttgag tgaaaggtga cggtgggttt 2760
ttagttttaa tagtatatag ggttttgtat tttgttattt tttttatttg attttttata 2820
agagttttag taatttgttt tgtttagata tgtttatttt tttttatttt ttttatttag 2880
taagtatttg gtgagtgtat attgtgggtt aggtgttgcg gatatagtcg agaacgtaat 2940
agataataat ttttgtttta gaggagttta tattttagta ggaaaggtag atgagaaaaa 3000
aggtaaatat gtataagagt gcgttagatg gtggtaaggg ttatgggaaa aaaattaagt 3060
aggggagagt tgttattttt atatggagag ttaggggaga gtagtcgtta aggaggggat 3120
aattgggtag agattaaaag gaggtgaagg aattgagttc gtgagtattt gagagtagta 3180
agttaggtgg aagcggtagt cggtgtgaag ggtttaaggt tggagagtgt ttggtaggtg 3240
atatggggag aggaggttga tatgggttcg ggggagagga gggaggtagc gggagaggag 3300
aatagagtga cgggggttag attgagttag gttttttcgg gtgttgttag gatgttgagt 3360
gagataggag ttataagagg gttgtgaata gagggatgat aggttgtgat tgagatttta 3420
ataggatttt tttggcggtt gtgagaagaa tagattgttg gggttgaaga agttattgcg 3480
gtagatgatg gtggtagtag tggggttggc gagaagtagt tggggtttgg agaaagtttg 3540
aatatagaat aggatttgtt gatagatagt atgttcgggg gaggtgagag aagggtttag 3600
ggtgagttta ggtttgtggt ttaattattt ataaagatag tattgtggtt agttgaggtg 3660
ggagttttgt aggagagtaa gttttgaggg gggttatatt agttttggag atggttagtt 3720
ttagaggttt gttagatatt tacgggtatg ttatgaatat attttaataa tttaaaaatg 3780
taagttaggt atacgatttt tatcgtgggt tgtatatttt ttgaattatt taatttattg 3840
aatttttaaa ttatcgttaa agggtgggga ttattatttt ttttattttt tatatgaggt 3900
aatggaggta tagagaagta atttgtttta agttatatag ttggaagttg gtagagtcgg 3960

aatttaaaat ttaagtggtt tggttttagt gagtaatcgt tttatattat tagtttttta 4020
tgaagagaaa tagagaaggg taagatttta gagagtgata ggaatgttgt ttttatatag 4080
ggtgtataag gagttttttt ttaaggaagt gatatttggt ttgaaggagg ggaggtagta 4140
agttaggttg ttttttgtgg gggagggttt ttaggcggag cgaacggcgt gtataaaggt 4200
tttgaggtag ttattttgaa ttatttatcg ttttcgagat agttttaatt tttgagggta 4260
agtacgggtt gtttttttcg tagggagagt ttttttttta ttttatggat ttaattatat 4320
aattatcgta ttttaggaag aatttatttt ttttttgcgt gattaaaggt gttaaataat 4380
ttgtttgttt ttgtaaattt gttttttttt ttgagttttt agttttatgt ttttttgttt 4440
ttgttttggt tattttggtt taaggttatt ttgagtaagt tatttagttt ttttttgttt 4500
gtttttttat ttgtaaaatg gggatatttt attttaaaag ttaaatattt ttaaaagtat 4560
atttattttt taaggttttt gtgaggatta attgtgttaa ttttgtaaaa tatttttgta 4620
aaagatttga ggggttgatt tttttttttg tttttataag tagtattgtt aggaattttt 4680
tttttatata tgtgtttttg tatagtatat tttaagtatt atagtgttta ataatgaata 4740
tttattgaaa taatattatt taggtgttag aaaattaaat tatttttaga aatatttttt 4800
atgtttgtgt ttttaggaga gtgtattatg tatattatag tggttatttt ttgggttttt 4860
tttcggagac ggggatttta ttatgttgta taggttggtt tggaattttt gggtttaagt 4920
gattttttta ttttagtttt tgtgtttagt tatatattat agttttgtga atatattttt 4980
ttaatgtaat aatatagttt ttatttatat atataattag tatttttaga aatagttttt 5040
atatttgtgt ttttatagag tatattatat attttagttt tgtgaatata tttttttgaa 5100
aaggaggaaa atttttattt atatataaat tggtattttt agaaatattt ttgatatatg 5160
tatttttttg tagtgtattt taggtattat cgtgtggtaa atattttttg ggggttggta 5220
tttgaataga aaagtgtttt tgatgtgtgg tagatatttt atagatatta gttattagtt 5280
agtgtgttat tttttttttt ttgtttttgt aattttattt tatgtattat ttgtattatt 5340
tggtttttat tatttgtgtg attttatttt ttattttttt tcgtattttg ttttagttat 5400
attggtcgtt ttgatgtttt ttgaatatgg taggtattat tttattttag ggtttttgaa 5460
ttggtatttt tttttgtttg gtatgttttt ttcgtagata ttaaggtttt tttcgttttt 5520
atcgtgaggt tttaatttaa gagttatttt ttttatgttt aagattttaa ttatttttaa 5580
tattcgattt ttatttgttt tattttatta ttttttttta gtatttgttt attatgttaa 5640
ttttatttat ttattttgtt tatatagttt gtttttttag tttgggtttt tttttttttt 5700
tttttgagat agagtttcgt tttgttattt atgttggagt gtatggtgta attttggttt 5760
attgtaattt tcgttttttg ggtttaagta atttttttgt tttagttttt taagtaattg 5820
ggattatagg cgtacgtcgt tatatttagt taattttttt gtattttagt agagatgggg 5880
ttttatcgtg atgtttaggt tggttttgaa tttttgagtt taggtaattt atttatttta 5940
gttttttaaa gtgttaggat tattggtgtt agttatcgtg tttagttttt ttaattttta 6000
ataaatgtta gttttataag tataaatatt tcgttttgtt tcgttttttg ttatatttta 6060
gtatttagaa gagtgtttgg tatttataag gggtttagta aaggttggtt gtaggaatag 6120
ttttaattaa ttagttttta tatttttttt ttttttttat gtgattttta tattggtgtt 6180
ttaaattatt ttttttttat ttatttatgg gataaatttt tattgagtat ttgttgttta 6240
tttggtatat ttttagttgt tggagatata gcggtgaatg agtagataaa aattgagttt 6300
ttatgggatt tatattttaa tgaggagaga tagataataa ataagtaatt tatatagtgt 6360
gttagatagt gataaagggt aacgggataa attagggatt tggtttgagt tttgttgggt 6420
gtgtagtttt agatggttag ggaggggttg attttttttg tttttataag tagtattgtt 6480
aggaattttt tttttatata tgtgtttttg tatagtatat tttaagtatt atggtgtgat 6540
aattaatttt taatgaaata atattattta ggtataataa aattaaatta tttttagaaa 6600
tattttttac gtttgtgttt ttaagagagt gtattatata tattatagtg gttgtttttt 6660
gggttttttt tttttagaaa aagggatttc gttatattgt ttaggttggt ttggaatttt 6720
tgggtttaag ttattttttt atttagtttt ttcgagtagt tgggattata agtgtatatt 6780
tttgtgttta gttatatatt atagttttgt gaatatattt ttttaatgta ataatatagt 6840
ttttatttat atatataatt agtattttta gaaatagttt ttatatttat gtttttatag 6900
agtatattac gtattttagt tttgtgaata tatttttttg aaaaggagga aaatttttat 6960
ttatatataa attggtattt ttagaaatat tttttatata tgtatttttt tatattgtat 7020
tttatatatt atagtgtggt aaatattttt tgggggttgg tatttgaata gagtgttgaa 7080
aggaattatg tagagatttg ggggtggtgt ttaaggtata aagaatagtt aatgtaaagg 7140
ttttgaggtt ggtttatgtt tgttatgttt gaggagttgt gtggtggaat tagtgtggtt 7200
ggagtatagt aagggggtgg gaaggtatag gcggtaatgt tagaaaggtt atttgtttta 7260
ttattaattt atagttaata tttatttata ttggaaggga ttttagtttt gattttttat 7320
tttataaatg aagaaaaagg tatagagagg aagtggttaa attatttatt taaggttata 7380
tagttgttgg gattgggggg tgggtgggtg ttgagagaag gtgaggaatg aagattgttt 7440
tgtaggtaga gggaatagta agaataaaag tttggaggtg agaattaatt tggtttttaa 7500
ggattgaggt tttacgtggg gtgggatgag atttaagagg ggagttttaa aaattatttt 7560
gtgttttgag tattttttgg gttatttttg agtaaggaat ttaatttttt tatggtttaa 7620
tttttttatt atttatttta tggggttgtg gtgaggatta aaggaattta tttatatgaa 7680
atattttgtt tgatatacgg taggtgttta ataaacgtta gtttttattg gtattattat 7740

tattattggc gttaatgtat ttatttattg tagtgaggta gaacgttttt gtggttacga 7800
gtgtgattag gttgatttat tattaagagg tggaggatag ggaaggtcgt atagtgtttt 7860
attataagga aaagaggatt agtacgtttt tttttgggaa gggaaaaaaa aagagaagga 7920
aattgaaagt atacggtcga ttttttttta attataaagt attggattta cgggagggta 7980
tgcgggggat tgtgtaagta tataggggtg tgggggagga aggttaatga atagatttat 8040
tgtaaaggga ggggtgatga ggtttatcgt ttgaggggaa ggggagtaga aaattttata 8100
gatttttgta ttatagtgag aggagagaaa agttattata tagattgatt taaagtagag 8160
agggaaaagg ttatggattg agttattgaa aatagggaga tgtaaagtat taaattaatt 8220
tattattaaa aaggagagaa ttttaagcgt tagagcgata gtttacgaaa aagaggagat 8280
agggtaatgg attgatagtt aaagggacgt gaatttaaat atgaagattg atttattata 8340
aaatggtggt ggtggtggtg gggggggggg gtggttttga aaaaagttat ggattgatgt 8400
attgggggat tggaggaggt aggaatataa atttaggtag atttattgtt aaagagggat 8460
gcggaggagt gcggcgttac ggattgattc gttacgatgt ataggaaggg tggaataaat 8520
aaggttacgg acgggttttt tgaaagttga ggttaggaga aatttaaata tttagatata 8580
tttattatta agggggttga atattgttat aggttagttt tttataagtg ggggtgggtg 8640
gggggcgggt gggggtaggg gtagggggaa agtttatgga cgagtttatt ttaagttggc 8700
gtggggaaaa atataagtac gtagatcgat ttattatata acgggagggg gaggttaggt 8760
tacggttaga tttattataa gggacgaagg gtttgagtaa tgttatacgt aggtttatta 8820
gtcgtggtgg tggcggcggg gagtcgaata ttagtaggtt atcggttaag ttatattttt 8880
tgcgcggggg cgggaaggat acgtggggtt acggattggg ttttttattt tttaggttta 8940
gagtttattt gtgtgtagcg tggcggtggc gttggtaatg ttggtagttt cggggggcgg 9000
gggttttata cgcggtttta tcgtttttta ggtttgggtt tcgaggcggc ggtggaggtg 9060
gtggtggcgg tggcggcggt agtatttaga agtcgttttt gcgttttttt atagtttacg 9120
tgggttcgag gaggaggagt ttaatgtttc gtcgttcgtt gattggttaa agcgttatta 9180
atcggtcgta aggtttcgag gggttggggg acgggttttt ttttatagag ttgtgtcgtg 9240
attggcgtaa ggggaaatga tggaacgttt acggtttttg gagtttcgag gaggcgcgtt 9300
cggtttttac ggtgttttac gtcgattggt ttaaggattg acggattgtg agtaattgaa 9360
aaggttcggg gtttgtgtcg ggggggcggg ggtttgtttt atattgattg gtttatatgg 9420
gattgatgga agatagtttt taaggacggg ggtgggtggt tttgtttttt tttgattggc 9480
ggacgaggga tttttagatt tttgttaaaa tattaagggg gcggtgtcgt acgttatgtt 9540
gtttagcgga agtagggttt gtatagaaat gggcgtagta gtcggcggga gtgggtcgga 9600
ttgggtttcg cgtaggcggg tagggtagtt ttacggttag ataagattat agagttgggt 9660
aaagacgaat ttagaatata gcggaggtag gagggtagga tggttgttag gtatacgaaa 9720
gagtattgag tggtagaaac gaaatgtttt tagagggtag ggttgaattg cggttagagt 9780
taaaaagggg aggaatttgg ttttgtattg attggcggtg gttggattta atttagaata 9840
ggggttcgat tagggagagc gggatttagg tttgtgttta ttggaagttg gaatatttat 9900
aagtaggggt taagataagg tagggttgag ttagggtttg ggtgtaattt ggggtatttg 9960
gggaatgatt gggtgtgtga aagcgtaaat tttagggttt tttgattgtt gtgggaatga 10020
ataaattttt attatgaatt ggtttatttt ttatggtaat ttaaagggtg gcgtttagga 10080
aaaggaatgg aagaaagggt gaggtggggg gtttaggttg ttaagatgat agaagagagg 10140
gtagtgtata aggttaattg gtaaatattg ttaggtaata ggttggaggt aggattgttg 10200
gggtattttg gaggttttat agagagattg gatttttggg ttttttagta attttgtgtt 10260
ttaggaatag gttttcgtgg taattagtag ggtttatggg gaaaaagaag ggttatgtga 10320
tttaaaggta tttaggtttt ttaatgtttg aggatggggg gaagaagtta atggtgaggt 10380
tttttgggga aattttgtag gttttgtagt tttttaagtt tttgaaaaag aggatagaag 10440
agattgaata aagattgggt ttgggttacg tttttaggtt tgttttgatt tttttttttt 10500
gattttagtt tagagggaaa gatggttttt tttttttttc gttttttttt ttttttaggt 10560
tattatttta aatatagagt ttggtagtgg ggtgagttgt ttttattttt agtaatatgt 10620
ggatatgggt tttgatattt atggagtttt tggtgaggtt gttgttgagt ttagagaaaa 10680
gaaataagaa agggtaaaat gggttttata tgattatgat gatttttttt tattatttta 10740
agatttattt atattttgga tgatttttat ttggtttatt tttatgtgga aatgttaagt 10800
tattatttgg tttttattga tttaaagatt attaaatgtt tattgtatgt taggtaattt 10860
taggtattgg gagatagtag taaatataat aaataaaatt ttcggtttga tggattttaa 10920
atttttgtaa agaaagatat aataaaaaat taatggaata aaagttatgt gttagatggt 10980
tttaattata ataataaaaa ttaaaaatga gagataataa ggagtatagg agtggggggt 11040
tgtaatttta aagagggttt agggatggtt ttacggagaa gtatttgagt aaagatttaa 11100
agatagtgaa ttttgagtag tgtggttata tggaggatgt gttttatata taaggggtag 11160
taaatataaa ggtattgagg tgggagtgtg tttggtatgt ttgaggtgta gtaaagaggt 11220
tattttggtt ggagaagaat aagtgaaggg ggaatgtaag aggtgaggtt agagaggtaa 11280
tgagggagat aaattgtgtg aagtttataa ggttatggta aggattttgg ttttgatttt 11340
aagtgagatg ggagttaagg gaaggttgta gtagaggaag gaggttgaat tatatgaatt 11400
agttagttga tttttttttt ttaagttagt ttggattaga tttcgtttaa aagaatttta 11460
attgatataa agggttaagg aaggtttttt ttaagaatga agaatgagaa ggaatcgaag 11520

aatgtttaat ggttaagtag taggaaggtt ttttttataa aaaggataag aggtaataaa 11580
gagttgtttg agaagtagtg aggttagagt gttagaaatt gagtggggag ggaaagtggt 11640
ttaagataag gtttaagaga taggaagagg ttagattata tagggtttta atataataag 11700
gaatgtggat tttattgtgt tgttaatgta ggagttagtt tgaggggtaa atgaaattaa 11760
atattgttgg aaaagtagag agtaataaga taggatgtta aattggaaaa aaaagtagta 11820
tgaatttatg tatatattaa tattttttat atttttattt ttagtttaga attttttttt 11880
gaatttgatt agttttaatg atatttttta aaaatttatt ttagtgttta ataagtgttt 11940
tatatttaat aggttttaga ttaaattttt gatttttttt tttaaattta tttttttttt 12000
aattttttta ttttagataa tggtaatttt atttttttag ttgtttagtt aaaaatttgg 12060
gttttttttt tttatatttt atatttagtt agttattttt tttaaaatta tgattgtttt 12120
attgtaggta atgtttttga tttttatgat atgttttttt tatagtattg ttaatatttg 12180
atatattata ttatttattt gttattgtt tgtttttttt gattagaatg ttagttgtat 12240
gagggtagat tttgtttttt tttgtttttt gttttatttt tagggattag aatagtgtgt 12300
ggtatagagg aggtatttaa cgtatgttga atgaataagt gattatttta ttaaaaggaa 12360
tttgaggttg gttgagtgta tgtttaggga taggaaataa atgaagtatg ggtgttgttg 12420
ttagagagta agaatatttt taagaatatt ttgggggtaa tgtttaaaga atagaagaat 12480
gaatttgatt agtttttaag ggataatttt aatattaaaa aagagaataa aattagttaa 12540
tggtgtaagt ttattttatg aaaatttatg agtttaatat tttaaaaggg agaaaaatat 12600
agagtttatt aaatgttagt tgtaatataa aagggatatg tttattattg ttgatttatt 12660
tttatttgtt atgtgagaag taaattatta ttgatttagt atgtaaggta gtgtgaatag 12720
ttatatggtt atgttttttt tttttttttt gagatggagt tttattgtgt cgtttaggtt 12780
ggagtgtagt ggcgtgattt cggtttattg taatttttat ttttcgagtt taagcgattt 12840
ttttgtttta gttttttaag tagttgggat tataggcgtt tgttatcgtt ttcggttaat 12900
tttttgtatt tttagtagag acggggtttt attattttgg ataggttggt tttgaatttt 12960
tgatttcgtg atttatttat ttcggttttt taaagtgttg ggattatagg tgtgagttat 13020
cgcgttcggt cgttaggttg ttttttttat aattatatgt ttatttatga gatgtggatg 13080
tgtatttttt tttgtgttat taggaaagaa tatagagatt ttattttttt tattttttaa 13140
ataaagaaat atattaagaa taggaagaat tgatattttt gattatataa aatgattaga 13200
aagttagttt aaagtatatt ttaagtaatt aaatattttt aaaggatagt tagttatata 13260
gatttaaatt tttatttttt agaaaaatta aatttttaga tagttttatt aattataata 13320
aagtagtaag atttaattat attttttatt atattaatag attaggttag tatattaaaa 13380
agattaggta agggggtagg tattgataat ttaaatattt tattttgggg aagtacgtgg 13440
agttttaaaa tattaaaatt tatttgatag gtttaggaat tttgtgatat aataaggaaa 13500
gtttttttat ataaattaga ttttagttgg gcgcggtagt ttatatttgt aattttagta 13560
tttagggagg ttgaggtggg tagattattt gaggtcggag tttgagatta gtttggttaa 13620
tatggtaaaa ttttgttttt attaaaaata taaaaattag tcgggagtgg tggtgggtgt 13680
ttgtagtttt agttatttgg gaggttgagg taggagaatt ttttaagttt aggaggtaga 13740
ggttgtagtg agttaagatc gtattattgt attttagttt gggcgataag agtaaaattt 13800
cgttttaaaa ataaagtgga tttggtttaa atgattatta gtttagaatt gtgttgatta 13860
tgaaagtagg tcgtttaatt aaattatttg ttaattggtt tgaatattaa gaattagaaa 13920
ttttagtagg tttgaggttt ttttgggttt attagtattt gttagttttt tttatggttg 13980
gaaaggtttg aaaagataaa aattattgaa attttaaggg aggtaatttt ggaaaaaatg 14040
aatgtagaat agatataaat ataaaatata attaataaag aagtttaaat taatggtatt 14100
tatatttgtg agatgttgtt aatttaagtg tattgtttag tgttttt 14147

<210> 155
<211> 3120
<212> DNA
<213> Artificial Sequence

<220>
<223> chemically treated genomic DNA (Homo sapiens)

<400> 155

aattttaagt ttaggtcggg tatagtggtt tacgtatgta attttagtat ttggagggat 60
cgaggtgggc ggattataag gttaggagat tgagattatt ttggttagta tggtgaaatt 120
tcgtttttat taaaaatata aaatttagtt gggtatgatg gtacgtgttt gtagttttag 180
ttatttggga ggttgaggta ggagaatcgt ttgaattcgg gaggtggagg ttgtagtgag 240
cggagattgt attattgtat tcgtttggtg atagagtaag attttatttt aaaaaataaa 300
taaagaaaat ttgaagtata gtattttttt aaattttaaa tagataatag aaattggttt 360
ttttttattt aaattagaat ttaagtttaa ttttatatat ttttgatagt ttggattttg 420
ttttttaatt ttataaaatt gggaatttaa gtattatttg gttcgattta aatgtaatgt 480

agaatttgta ttaaaatatt atattaaagt tttagatttg tagtagttaa tagtattttt 540
atgtatgtgt tagggattgt tttaaatatt ttatatatat taattttttt attttgtatt 600
tttgttttcg ttttatatag taggaaattg aaatattgag aggttaagta attaaagtta 660
tagagttaga gtgataggag taaagtttta atttaggtaa tttagatttt tagagttttg 720
atttttatta ttaagttgtt agtatagttt ttttggtaat tatttttaat ttaaatataa 780
ttcgagtgat ttatttaata agttattatt ttgataattt agtgatttgt aatgtaaaat 840
tatttattgt aatttattta atattattgt ttttttgtgt tgtaaaaatt atagtaatcg 900
agatgtaatt tattattttt ttttttattt tcggtatttt gtgttaattt ttttgttttg 960
cggatttttt tcgatttttt attatgcgtg ttaattgtta ttaatttttt tgtttgttgg 1020
ggattggggt cgcgagggta tattttcgag gggtacgggg ttagggttag gtaggttgtg 1080
cggttgggcg gggttttgtg ttttattgcg gagtgcgggt cgggaagcgg agagagaagt 1140
agttgtgtaa ttcgttggat gcggattagg gcgtttttta ttttcgtcgg gagttcgtcg 1200
attggttggg tgtgggcgta cgtgatcgat atgtggttgt attggtgtag ttcgttaggg 1260
tgttattgga gatagaatgg aggtgttgtc ggattcggaa atggggtagg tgttggagtt 1320
attatggtta ggtttgttgc gggggggaggg gggaaggtgg tttttttttcg tattgtttta 1380
aatcgatggt ttttttttgg tatagggttt attgtagtat gttaaacgag gaggtagggg 1440
cgtcgttttt tcgtttttta ttgtagtatt ggagatggat tttttgtatt tcggatttag 1500
ggtttttgat agaagaggaa gaagggggag gggtagaagt gttaagggga gtttgttgag 1560
aaaagttgtt tttgaagtta gaaggggttt ttgtttttat aatgttattt gatagagtgg 1620
aataatagta tttaaggaaa cgggtagagg ataataaaga atggagtata tttatggcga 1680
ggagtaaaag ttttatttta ttgaaaggtt tttttttttt tttggcgata aggatatatg 1740
tattggtggt taaaagagag aggagataaa atcgttgtag atggttgatg tgaatttagt 1800
ggaaagagtt attggggatg agagaaagag gaggaggtag gtattgtaga gcgtgagtgg 1860
tggtgttggt tggtgaaata ttggttatta gtagtgtgtt tgtttttgta aaatatttaa 1920
gtaaattttt tgtgaatagg gtggtaaata gatattagtg tttttgttag ttataaaatg 1980
tagtggtagt ggttttttgc ggacgattgt agtagtgttt tttttttttt tgttaggtcg 2040
aaaagataat tgtagaggaa taagaaattt tgtagtaaat gttggggtag aagtttattt 2100
ataagaagtt atagtttata aatgtagttt gaatagtaga aaaaaaatta ttgtttttta 2160
aagtaggaat aatgttaggt tatgaatgtt ttgttattgg aatgtattgg atattttgat 2220
tttatattac gaaagtgatg tttaaatttt ttgatttaat ataaatttta tacgaaattt 2280
taataaatta tgtatgaaat agtggatttt tttttttgtt agtgaagttt ttatgttatt 2340
aattaggtta tttaagagta aattatttta taacgtaaat tattttgtta aaaattatgg 2400
tgaataaatt tttgtaggtt taatatttaa gatttatagt taagtaattt tatatttttt 2460
tttggttgtt tttagataat attgaataaa tatttaagat attaatttag tgtgtaaata 2520
ttttaaatta aagtaatatg gttttttttt tagatgtatt tttgtttagt gagtatttat 2580
gagatatatt tttgatataa agtgtttttt attgagtttt tttttttttt atttaaatgt 2640
aaaagtttat ttttatgtat atttatagta gttagtttgt tatatttttt tattttttga 2700
ataaggatgg tagtggtttt gtaaagtttt ttagggttag tgatagtgtt ttttaaataa 2760
tgtttatgta atagaaagtt ttaagatgat ttattatatt gttttagtaa tatttttaag 2820
ataatgcgga attgggttgg tttagataaa taagattata taagtttttt attgataaat 2880
ttaaatagtt ttttaaaaaa ttatttttgt tttaagaatt atttgattat ggatattagg 2940
gttaatagta tttaggagtc gggcgtggtg gtttatgttt gtaatataag ttaaatagga 3000
ggttgaggga agttgaggta ggaggattat ttgagttttg gcgtttaaga ttagtttggg 3060
taattaagta agattttatt ttaaaaaaaa gtatttagag tttttttttt aaaatttgat 3120

<210> 156
<211> 3120
<212> DNA
<213> Artificial Sequence

<220>
<223> chemically treated genomic DNA (Homo sapiens)

<400> 156
   attagatttt aaaaaagaga ttttaagtat ttttttttga gatagggttt tgtttggttg 60
   tttaggttgg ttttgaacgt taaggtttaa gtgatttttt tgttttagtt ttttttagtt 120
   ttttgtttag tttgtattat aggtatgggt tattacgttc ggtttttaag tattattaat 180
   tttgatattt atgattaagt aatttttaaa gtagaaataa ttttttaaag aattatttaa 240
   atttattagt gaaaaattta tgtagtttta tttatttaag ttagtttaat ttcgtattat 300
   tttgaaaata ttattgaaat aatataatag gttattttaa gattttttat tatataaata 360
   ttatttaaga gatattgtta ttgattttgg agggttttat aaagttattg ttatttttgt 420
   ttagggagtg gggaggtgtg gtaggttggt tattataagt atgtataaag ataggttttt 480
   atatttaggt aaaggaaaaa aaagtttaat gaaaaatatt ttatattaag agtatatttt 540
   atgagtattt attaaataga aatatattta gaagaaaagt tatgttgttt taatttaaaa 600
   tatttgtata ttgaattaat attttaaata tttatttagt gttatttaaa agtaattaaa 660
   gagaaatata aaattattta attataggtt ttaaatatta ggtttataaa aatttgttta 720
   ttataatttt tgataaagta atttacgttg taaagtggtt tgtttttgaa tgatttaatt 780
   aatggtataa aagttttatt aataaaagaa aagatttatt gttttatata taatttatta 840
   agatttcgta taggatttat gttaaattaa agaatttgag tattattttc gtgatgtaga 900
   attaaagtgt ttaatatatt ttaatgataa aatatttata gtttgatatt atttttattt 960
   taaaaaatag taattttttt tttgttgttt aggttgtatt tataaattat ggttttttgt 1020
   aaatgggttt ttattttagt atttgttgta aggtttttta tttttttgta gttgtttttt 1080
   cggtttaata gagggagaaa aagtattgtt gtagtcgttc gtaaaaagtt attattattg 1140
   tattttgtaa ttaataaaga tattggtatt tgtttgttat tttgtttata gggagtttat 1200
   ttagatgttt tataaaagta ggtatattat tggtgattag tattttatta attaatatta 1260
   ttatttacgt tttgtagtat ttgttttttt tttttttttt tatttttagt agtttttttt 1320
   attagattta tattagttat ttgtagcggt tttgtttttt tttttttttg gttattaatg 1380
   tatgtgtttt tgtcgttagg gaggaaaaga agttttttaa tggggtagag tttttgtttt 1440
   tcgttatgaa tatgttttat tttttgttgt tttttattcg tttttttaga tattgttatt 1500
   ttattttgtt aaatggtatt ataaaaataa aaattttttt tggttttaaa aatagttttt 1560
   tttagtagat tttttttaat atttttattt tttttttttt tttttttttt gttaaaaatt 1620
   ttggattcga agtataggaa atttattttt agtgttgtag tggggggcgg ggggacgacg 1680
   tttttgtttt ttcgtttggt atgttgtagt ggattttgtg ttaaggaaag gttatcggtt 1740
   taagatagtg cgagggaaaa ttattttttt tttttttttc gtagtaagtt tggttatggt 1800
   ggttttagta tttattttat tttcgagttc ggtagtattt ttattttgtt tttagtgata 1860
   ttttggcggg ttgtattaat atagttatat gtcggttacg tgcgtttata tttagttaat 1920
   cggcgggttt tcgacgggaa tggggagcgt tttggttcgt atttagcgga ttatatagtt 1980
   gttttttttt tcgtttttcg attcgtattt cgtagtgggg tatagggttt cgtttaatcg 2040
   tatagtttgt ttagttttag tttcgtattt ttcgggggta tgttttcgcg gttttagttt 2100
   ttagtaagta aggaagttga tggtagttga tacgtatagt aaagagtcgg gggaggttcg 2160
   tagggtagaa ggattagtat aagatgtcgg aggtgggagg gagagtaata aattatattt 2220
   cgattgttat gatttttgta gtatagagaa ataataatat taaatgaatt ataatgaata 2280
   attttatatt ataagttatt gagttgttag agtgatgatt tgttagatag attattcgaa 2340
   ttatatttga attaaaaata gttattagaa aagttatgtt agtagtttag tagtggagat 2400
   tagaattttg gaagtttggg ttgtttgagt tgaagtttta tttttgttat tttagttttg 2460
   taattttagt tatttaattt tttagtgttt taattttttg ttgtataaaa cgggaataga 2520
   agtatagaat agaggagtta atatatatga agtatttaga gtagtttttg atatatatat 2580
   agaagtgttg ttagttatta taaatttgag gttttaatgt agtattttaa tgtaaatttt 2640
   atattgtatt taaatcgaat taggtgatgt ttaaattttt aattttatga ggttgaagga 2700
   taaaatttaa attgttaaga atatataaag ttaaatttaa attttggttt aaatgggggg 2760
   aaattagttt ttattattta tttaaaattt aaaaggatat tatattttaa gttttttttg 2820
   tttgtttttt gagatagagt tttgttttgt tattaggcga gtgtagtggt gtaattttcg 2880
   tttattgtaa tttttatttt tcgggtttaa gcgatttttt tgttttagtt ttttaagtag 2940
   ttgggattat aggtacgtgt tattatgttt agttaagttt tgtattttta gtagagacgg 3000
   ggttttatta tgttggttag gatggtttta attttttgat tttgtgattc gtttatttcg 3060
   gtttttttaa gtattgggat tatatgcgtg agttattgtg ttcggtttaa atttaaagtt 3120

<210> 157
<211> 2501
<212> DNA
<213> Artificial Sequence

<220>
<223> chemically treated genomic DNA (Homo sapiens)

<400> 157

ttatacggtt agaaagggtt taggcggttt tcggcgtttt tttagttttt gtttttatgg 60
cgtatttttt cgttagtcgt tttttttcgt atttcgtcgt tcgttcggtt cggtcgcgtg 120
cggtttttcg ggagttttta tttcgtcgcg gattttagcg attattaagt tcgtacgcgg 180
tttgtcgtag gtttgagtag aaggtttcgc gtatatttat cgcgtcgcgg tcgcgcggga 240
ggtttgtgtc gttcgcgtta tttattggtc gggtttcgcg ggcgtagcgg agcgggcggg 300
tggtcggttc ggacgcgttt ttttcggtcg cggtttcgcg cgttatgtgt tttcggcggg 360
acgcgttatt ttcgggtttg tcgcggcgtt tttaattcgg gttagggagc ggggcggagg 420
gggcggtcgg gtggtttaga ggagggtttt tttttttttt tttttttgaa tgaatcgtgt 480

gacgttacgt ataggaaatc ggtcgggttg tgtagagaat gaagtaagag gataggtatt 540
atagtttcgt tttcgttttt tttttttcgc gttcgttttt tcgcgtcgtt tgttcgttcg 600
ttcgtcgcgt tttcgttcgt cgtttttcgt ggtttcgtcg cgttgtcgtc gtcgtcgttg 660
ttagtagaag gtgtcggggt ttcgggtttt ttttgtcggc ggtcgttagc gttcggagcg 720
ggttgcgcgg cgggagtttc gggaggcggt cgtagttagc gtcgtcgcgt aggattagga 780
ggaggagaaa gggtgcgtag ttcggaggcg gggtgcgtcg gtggggtgta gcggaagagg 840
gggtttaggg gggagaattt cgtagtagtt atttttttta ggaaaagagg gaaaaaataa 900
aatttttttt tattattttt ttttttttat ttttcgtcgt attatatata gcgcgggttt 960
ttagcgttcg gtatcggcgg gttaggcgcg ttttgttttt atttatttag tagtttttcg 1020
gaaaatgtat ttgttgttcg gagtttaatt agaagaggat ttttgttttc gttttcggtt 1080
tttttatcgt tttttttttt ttgttttttt tttggggagg cgtgaagcgg tttcgtggat 1140
agagatttat gtttgtgttc gcgcgtgtgt gcgcgcgtgt aaattgtcga gaaggggaaa 1200
atattatagg atttttgcga atatcggatt gaaaattgta atttatttgt cgtcgtcgtt 1260
gttttttttt tttttcgagt ttttgagatt ttcggttggg atttttgcgg attgatattt 1320
ttgtgaagta gaagtttggg aatcgatttg gaaatttttt taatttttat tttttttttt 1380
cgcgattttt gatttattgg gaagttttaa attagttata attggagagt gttgaagatt 1440
gatgggatcg ttgttttatg tatttgtttt ggttttataa aaaggaaatt tgatagagga 1500
ttatgttgta tttaaaaaat ataagtaagt tttttgtata ggaaattggt ttaatgtaat 1560
ttttaatgga aatttttgag attttttatt taaagtgtat tcgagtaaat ttaattttta 1620
ggtagtttaa tatatttttt ttagtcgtgt tatttgtagt gtgtatgttt tgtttttatt 1680
tagtgtgtat agggaaacgt atttgatttt ttatttatta gtttgttttt tttttaattt 1740
tttagtatta tagaggaagt agattgatat taataatatt tattaataat aacgtgtttt 1800
atgaaataaa gattcgaaag gaattggaat aaaaattttt tgtattttat gttaaggggg 1860
aaatattaga attaagtgtt tcgcgtgatt gaagatattt tttcgtttaa gaatgtaaag 1920
tatatttaat aaaatagttg gattataatt tttttttttt ttttgggggt cgtggggtgg 1980
gagttggggc gagaggtgtc gttggttttc gttgtttttt ttttgggaag gatggcgtac 2040
gttgggagaa tagggtacga taatcgggag atagtgatga agtatattta ttataagttg 2100
tcgtagaggg gttacgagtg ggatgcggga gatgtgggcg tcgcgttttc gggggtcgtt 2160
ttcgtatcgg gtattttttt tttttagttc gggtatacgt tttatttagt cgtatttcgg 2220
gattcggtcg ttaggatttc gtcgttgtag atttcggttg ttttcggcgt cgtcgcgggg 2280
tttgcgttta gttcggtgtt atttgtggtt tatttgattt ttcgttaggt cggcgacgat 2340
ttttttcgtc gttatcgtcg cgatttcgtc gagatgttta gttagttgta tttgacgttt 2400
tttatcgcgc ggggacgttt tgttacggtg gtggaggagt tttttaggga cggggtgaat 2460
tgggggagga ttgtggtttt ttttgagttc ggtggggtta t 2501

<210> 158
<211> 2501
<212> DNA
<213> Artificial Sequence

<220>
<223> chemically treated genomic DNA (Homo sapiens)

<400> 158

atgattttat cgaatttaaa gaaggttata attttttttt agtttatttc gtttttgaag 60
agttttttta ttatcgtggt aaagcgtttt cgcgcggtga agggcgttag gtgtagttgg 120
ttggatattt cggcgaagtc gcggcggtag cggcgggaga agtcgtcgtc ggtttggcgg 180
agggttaggt ggattatagg tggtatcggg ttgagcgtag gtttcgcggc ggcgtcgggg 240
gtagtcgggg tttgtagcgg cgaggttttg gcgatcgggt ttcgggatgc ggttggatgg 300
ggcgtgtgtt cgggttggga ggagaagatg ttcggtgcgg gggcggtttt cgggggcgcg 360
gcgtttatat ttttcgtatt ttattcgtag tttttttgcg atagtttata atggatgtat 420
tttattatta tttttcggtt atcgtatttt gtttttttag cgtgcgttat tttttttaga 480
ggaaaagtaa cgggggttaa cggtattttt cgttttagtt tttattttac ggtttttaga 540
gaaagaagag gagttataat ttagttattt tattggatgt gttttgtatt tttggacgag 600
ggggtgtttt taattacgcg gaatatttga ttttggtgtt ttttttttgg tatgagatgt 660
aggaaatttt tattttaatt tttttcggat ttttatttta tgaggtacgt tattattagt 720
aagtattgtt aatattagtt tatttttttt gtgatgttga aaggttaaag aaaaaaataa 780
ttaataagta aaaaattagg tgcgtttttt tgtatatatt gagtgaaagt agggtatata 840
tattataagt aatacggtta aaaagaatgt attaagttgt ttggaaatta aatttattcg 900
aatgtatttt aagtaaaaaa ttttaaaggt ttttattgaa agttatatta aattaatttt 960
ttgtgtagag aatttatttg tattttttaa gtatagtatg attttttgtt aagttttttt 1020
tttgtaaaat taaaataaat gtataaggta acgattttat taatttttag tattttttag 1080

ttatagttga tttgaaattt tttaatgaat taggagtcgc ggggagaggg agtaaaaatt 1140
aggaggattt ttagatcgat ttttagattt ttgttttata gaaatgttaa ttcgtaggaa 1200
ttttaatcgg agattttaag agttcgagaa aaaaaaaagg tagcggcggc ggtagatgaa 1260
ttataatttt tagttcggta ttcgtagaag ttttgtgatg tttttttttt ttcggtaatt 1320
tatacgcgcg tatatacgcg cgggtatagg tatgaatttt tatttacggg atcgttttac 1380
gtttttttag gagagagata ggggagaggg gacgatgaag gagtcgggga cggaggtagg 1440
aatttttttt tgattaaatt tcgaatagta aatgtatttt tcgaaaagtt gttggataaa 1500
tgaaggtagg acgcgtttgg ttcgtcggtg tcgagcgtta gaagttcgcg ttgtgtgtgg 1560
tgcggcgagg ggtggggaga aggaggtggt gggggagggt tttatttttt tttttttttt 1620
ttaaaaagga tgattgttac gaagtttttt tttttggatt ttttttttcg ttgtatttta 1680
tcggcgtatt tcgttttcgg gttgcgtatt tttttttttt tttttggttt tgcgcggcgg 1740
cgttggttac ggtcgttttt cggagttttc gtcgcgtagt tcgtttcgag cgttgacggt 1800
cgtcggtagg gagggttcgg agtttcggta ttttttgttg gtagcggcgg cggcggtagc 1860
gcggcggggt tacggagagc ggcgggcggg agcgcggcgg gcgggcgggt aggcggcgcg 1920
gaggggcggg cgcgggagga agggggcggg agcggggttg tggtgtttgt ttttttattt 1980
tattttttgt atagttcgat cggttttttg tgcgtaacgt tatacggttt atttaaaaaa 2040
agaagaaaga aagagttttt ttttgagtta ttcgatcgtt tttttcgttt cgttttttgg 2100
ttcgggttaa aggcgtcgcg gtaggttcgg gagtggcgcg tttcgtcggg ggtatatggc 2160
gcgcggggtc gcggtcgggg agggcgcgtt cgggtcggtt attcgttcgt ttcgttgcgt 2220
tcgcggggtt cggttagtgg gtggcgcggg cggtataggt ttttcgcgcg gtcgcggcgc 2280
ggtgggtgtg cgcggggttt tttgtttagg tttgcggtag gtcgcgtgcg gatttggtgg 2340
tcgttggggt tcgcgacggg gtgggggttt tcggggaatc gtacgcggtc gggtcggcgg 2400
gacgacggag tgcggagggg ggcggttggc gggagggtgc gttatgaaaa taagggttgg 2460
aaaagcgtcg ggaatcgttt ggattttttt tggtcgtgtg a 2501

<210> 159
<211> 6104
<212> DNA
<213> Artificial Sequence

<220>
<223> chemically treated genomic DNA (Homo sapiens)

<400> 159

tagttttgaa gtaggtagat atgtttatat ttttttttga gttagtgttt ggatttaggg 60
gattagattt tttatttgat atggtagaga gataagttat gtttaggttt tttaaaagga 120
gtttggaaaa tataattaat ttaagttaaa tatttttatt atttttttgt atatataata 180
aagataaaaa tatttatttt attaaattgt tgagagtaag aaagataata tatatatagt 240
gtttggtata cggatggaat ttagttgatg gtagttataa tatttataat attttttttt 300
tgttttaatt taatatgatt tatatgattt tttatattta ttgtatatgt aatatttttt 360
agataatttt atatttaggt tattagtaaa atattgatat tttttttagt atttatatag 420
atgttttttt tttaaagttt ttagtaaaat ttttattggg gaattttagt tgttttttat 480
tttttttttt ttttattttt atttttatat taaaggtttt tatttttaag gtatataata 540
tgatattttg atatatgtat attttgtgaa atgattgtaa taattaagtt aattaatata 600
ttattatttt atatagtttt ttgtgtgtgt gtgtgtgtgt gtgcgtttag agtttttgag 660
atttattttt tttgtaaatt ttaagtatat gttaattata gttattaggt tgtatattag 720
gtttttagaa tttatttatt ttataattga aaggttgtat tttttaatta gtattttttt 780
tttattttta atcgtagttt taaataatta ttattttaat ttttgttatt gagttcgatt 840
tgttttttga gattttatat ataagtgaga ttatgtatta cgtgtttttt tgtgttttat 900
ttattttgtg tgttataata ttttttaggt ttagttatgt tggtataaat gataatattt 960
tttttttttg aataatattg ttgattaata ttttattgtg tgtgtgtgat aaatatatat 1020
tatatttttt ttaataattt atttattaat ggatatttag gttgtttttg tattttggtt 1080
attgtgaatt tcgttgtttt tttggtagtt atttttgtat tttttttttt agaagtagtt 1140
ttattgtagt ttaaaagtgt tttaggaaaa tgatttagaa gtaattattt tttttggtat 1200
gttttttagt aaatacgatg atggaaagtt ttgatttttg ttttttttta aatagtgtaa 1260
tcgattttta gttgggattt tttttagtta atttgatagt tttacgtttt tattaggttg 1320
aagaaaatgt atgtgttgtg ggttgttgta ttattgttgt tggtggtgtt gttgttattg 1380
ttgagatttt gtggttgagt ttatattata aaggaattgt agagtagtaa taaatttaat 1440
ttgagttttg aatttaagtt tttaggtgtg atttttttaa ttacgtggaa atttacgtaa 1500
gaatttttaa ttagatttta tgtttatttt tattttttaa gtaaaggtta taggaatttg 1560
gtttaaacga atgggaataa atattatagt tagattttat tggagtgttg taaaaatgtt 1620
tttttttaaa attagattgt tttaaaattg attttggttt ttagattttt aagttaggaa 1680

gtaagatagt aaaaattaag ggttaaatag ttaagcgatt atttttttaa aattagtatt 1740
ttttttggcg attttcgtta gattatgttt tgtttagtat tgaaatttag agaagtttta 1800
ggaatagcgg gatgaaattt aagtgattcg tttacgggaa gttttatttt tttttcgttc 1860
gtttagtaaa ttcgggatgg gtttagagaa tgatttcgta ttttgtatta gaagatttta 1920
gtttttagag agaattttag acgaaatttt tatttttttg ttttattttt gaaaattttt 1980
atttgggaat cgatttgttg tgataaataa aatgggagga gtttggttga atagtttgtg 2040
gatttcggaa agtaaagcga aatgtcgttt taattaggtt cgaatttttt gtaagttatt 2100
ttgtgaaatt ggtgaatttg ttagaatgaa tttttattta tttaattttg gcggatcgtt 2160
tattagggtg tttggtatgg tgttaattgt tggagaaaat gatttttaaa taaaaacgaa 2220
aaaaattttc ggtttttagt aaggagttag gtaattttta taaatttaag gagtgtttga 2280
gaggtgagtt aatgatcgtt ttttttgttt taaagagata gatttttata gttagttttg 2340
aaattgttcg gggttatatt agcgtttttt ggacgtagat tttggttagc gttttacggt 2400
attagttttt gatttgtagt tgttaaagat ttgatagtgg ggagtacgtg tttattttga 2460
aggttgaaga tatatattat agttaggagt agaatttcga ttttttaaag tgatatggtt 2520
ataaaatttg cgtttattgg tttttatttt gcggttatcg tggtacgggc gaaagttatg 2580
aatttttttt ttaggaaaat atatattata gatatatata tttataaacg gttttaggag 2640
gtttttatgt tagaaatttt tggttttgga aaaaaattat tttcgttgaa gtatgtggta 2700
gttgagcgta aatatttttt tgttatagtt ttagtggtag aggtttaagt attacgtttt 2760
atttagtgtt cgtattgcgg ttttcgtaat tttttaagta gttttgtatc gaaaatttag 2820
gtcggagatt cgagtttaaa ttgttgaaaa gttttaaggt ttcggatagg gttttagtag 2880
gggttatttt tttttaggat tcggggttta ttgtgtttaa gattcgttat ttaagttttg 2940
ttttgcgcgt acgttgtttg ttttacgttt tgcgttgata cgcgtcggtg ttttttcggg 3000
ttagttttag tttcgggttt tgtggtcgtt ttgtcggcga ttttgcggag agcgagtttt 3060
agatatttag tttttagttt cgagttgtta ttttttcgtt gtagttaaga aggaggagat 3120
taattaaggg tattttagaa gttaggcgtt ttcgttgttt tttttgagta cggaggttat 3180
taatttttta gggggaagag atgtagcgcg aggtaggggt gtcgtgttaa gaaatttcga 3240
cgtttttggg gattgaggat aaaggtgcgg atacgatttc ggggtatttg gagtttcgtg 3300
attcgcgtta cggacggtat atttaggggt taatttttgt tttgttttaa agaattttaa 3360
gttagagttt ttgttttcgt ttatagtttc gggatgtcgt tgttgcgttt atcgtatagg 3420
tagcgttcgg atcggttgta gtagatcgcg cgttgcgcgt tttatcggga gatggtggag 3480
acgttgaaaa gttttttttt tgttattttg gacgttgtgg gcggtaagcg ttttagtttt 3540
tatttttgtt gagttgaacg tttaggtata gtggaattga aattcggttt tgcgggatgt 3600
gagagttgtt gaggttacgc gtaattgggt gtgatggagg gcgtttgttc gtgatgtgtg 3660
taggtttgat gtaagtaggt tatcgtcgtg cgagtgtgtg gatgcgatcg ttcgagagat 3720
tcggaggtag gtttgggata cgtttgagtg aatattttag gatatttttt tggttagtat 3780
ttgtttttta gtgtttgtga tttagagtgg gtatatgttg ggagatagta atgggtttgg 3840
gtgtgtgtaa atgagtgtga tcggaagcga gtgtgagttt gatttaggta gggattatat 3900
agtattgtta tatttgtttg ttttttagta gaggattgaa gtgcgggggt gggggtacgg 3960
ggtcggaata gaatgttttt gggatatttt ggtaaatagt agtcggaagt aaaggggtag 4020
ttgtgtaaac ggtttaggta ggtgatggat ggtagggtag gaagggggag gtttagaggt 4080
ttggatggag gttttcgtat ttgtattttg taatttattt tttaggttta gtaggttatc 4140
ggtttttttt ttatatatgt aatggatttg aagagtattt cgggatagtt cggggagatg 4200
gagattcgga aagtatttat ggagatttta tagaattttt tgtgcggatt aggaaatttt 4260
tgtagatttt tgtttatttg aggtttaggc gttgggttgt tttttataat atttttttaa 4320
gatgagattg tggtttttat tttaaagatg agtatattga gtttttgtga agttatttgt 4380
ttatgattat ataattagga attgggttaa ttgtaattga atttttgttt aataaagttt 4440
ttgtttttag tttcgttttt tgttttttac gagttttggt tttttgtggg taatattagt 4500
tattggagtt agattttttg ggtttagaat ttatttttag gggtattaat ttttaaaatt 4560
ttatttgggt aggggtttgg gattagagtt ggaagagttt ttataatttt ggattttttt 4620
cgttaaatcg tgaaattagg ggtggagtgg ggcgagggtt taaaattagg tcggattgag 4680
aggtgaaatt tattatgacg ttaaattgtt tttaaatttt cgtttatttt aagggcgtta 4740
tttgttggtg tttttattat tttttattat ttttattaat gattttaatg taaatataag 4800
tgggacggtt ttgttggatt ttttaggttt tggaagtatg agggtgacgt aatttagggg 4860
taaaggattt tttcgtttat tggttgttgt gtattggcgg aattttttcg atttatagcg 4920
gcgggaataa gagtagtcgt tggcgttggg aggtattaga gatattgttt agtttaagtg 4980
tcgtcgtcgt ttttataggg ttttgttgga cgtcgtcgtc gtcgttgtta tcgtttttga 5040
tttaagttat ttttcgttag gtgagtttcg agattttggt ttaggtatat gttttttttt 5100
tttttttttt ttattcgtta tttttttatt tttttttttt tttttttttt tttttcgtta 5160
gtttttttat tagatagttt ttgttagttc gcgatttata ttaggttcgt gttttaggtt 5220
ggatcggata gttttaattt ttcggttcgt tttttttgtt cggttgcgga ttttagtttt 5280
attttttcgt attgtatata ggtttaggtt agtttcggga tatttaggtt ttttagggat 5340
cgcgtataga gtttgaggta agagaaattt ttcgtagacg gtgcgattag ggacggcgtt 5400
tggagtttag tagttttagg gaaattggtt tagaatttgg aatagagcgg atgggtggta 5460

aataggtacg acgattgagg gataagtagt tttaaattgt aagttttagt tataggtttt 5520
gggagtaaag agagaaagtt ttgggtttta atttaagagt aaaatttggt ttttgtaggg 5580
gattgaggtg ttagtaggcg gagtttaggt tgagtattaa aattgatttt tttatttttt 5640
agattttaat tgtagagata atttttgtgg atagatttta ttttttttat taaggtgtat 5700
gtaaaagttt tagaagagga ggatagtttt gggttttttt atgggattag tttttgtgat 5760
aggggtttgg gagtatgttt tttatagaga tttggttaat aggtttatat gtatttgtgt 5820
ggttttgggg agaagggtag gattgagatt ttttttattt agtttttttt ttaggatcgt 5880
ttttttagtt ttattagatt gtgtatgtta taggtttatt gaatttttta gaagtttatt 5940
gtgttgatga ggaaataaaa aagaagagaa atataatgat tagtttgaag ttataaagtt 6000
agagcgttag aagtaagata gaaaattagg ataggagtta aggtttgtgg gtttttagaa 6060
tttcgaattg gcgtaggtgg tttattttat tttttttttg taga 6104

<210> 160
<211> 6104
<212> DNA
<213> Artificial Sequence

<220>
<223> chemically treated genomic DNA (Homo sapiens)

<400> 160

tttgtaaggg aagaatgaga taaattattt gcgttagttc gaagttttag gagtttatag 60
attttggttt ttattttggt tttttgtttt gtttttaacg ttttggtttt gtggttttag 120
attggttatt atattttttt ttttttttgt ttttttatta gtatagtgga tttttgggag 180
gtttagtggg tttgtagtat gtatagtttg atagagttgg aggagcgatt ttagagggag 240
agttgagtgg gggaggtttt agttttattt ttttttttaa agttatatag atatatataa 300
atttgttagt taggtttttg tggaggatat gtttttaggt ttttgttata gaagttgatt 360
ttataaggaa atttagagtt gttttttttt tttgggtttt ttgtatatat tttggtggga 420
ggggtgggat ttatttatag agattatttt tgtaattgga atttgagaaa taaaagaatt 480
aattttagtg tttagtttaa gtttcgtttg ttggtatttt agttttttgt aggaattaag 540
ttttattttt aaattgggat ttaagatttt ttttttttgt ttttagagtt tgtgattggg 600
gtttgtagtt tagggttgtt tgttttttag tcgtcgtgtt tatttgttat ttattcgttt 660
tgttttaggt tttgaattaa tttttttggg attgttgggt tttagacgtc gtttttgatc 720
gtatcgtttg cggaaagttt tttttgtttt aggttttgtg cgcggttttt ggggagtttg 780
agtgtttcga gattggttta agtttgtgtg tagtgcgaga gagtaagatt ggagttcgta 840
gtcgagtagg aagagcgagt cgggggattg agattgttcg atttaattta gggtacgagt 900
ttggtataaa tcgcggatta atagagatta tttgatgaag agattaacgg agagagaggg 960
agaggaggaa agggagtgag aaaatgacga atggagggag agggagggag agatatatat 1020
ttgagttagg atttcggggt ttatttggcg ggaggtggtt tggattagag gcggtggtag 1080
cggcggcggc ggcgtttagt agagttttgt ggaagcggcg gcgatatttg ggttgggtag 1140
tgtttttgat gttttttagc gttagcgatt gtttttattt tcgtcgttgt gggtcgggaa 1200
agtttcgtta gtgtatagta attaatgggc ggaggggttt tttgtttttg ggttgcgtta 1260
tttttatgtt tttagaattt ggaggattta gtaggatcgt tttatttgta tttgtattga 1320
ggttattgat ggaaatggtg ggggatggtg ggggtattaa taagtaacgt ttttaaagtg 1380
agcgggaatt tgagggtagt ttgacgttat ggtgaatttt attttttagt tcggtttggt 1440
tttgaatttt cgttttattt tatttttggt tttacgattt ggcggaaagg gtttaggatt 1500
gtagggattt ttttaatttt gattttagat ttttgtttaa gtgagatttt aaaggttaat 1560
gtttttaagg gtgggttttg ggtttaagaa atttgatttt agtagttggt attatttata 1620
gagggttagg gttcgtggga aataagagac ggagttggga gtaagaattt tgttagatag 1680
gagtttaatt atagttggtt taatttttgg ttgtgtgatt atgggtaagt aattttatag 1740
aggtttagtg tatttatttt tgaaatgggg attataattt tattttgaag gaatattgtg 1800
agaaatagtt tagcgtttgg gttttagata ggtagggatt tataagagtt ttttggttcg 1860
tataggggat tttgtaagat ttttatggat atttttcgaa tttttatttt ttcggattgt 1920
ttcggggtat tttttagatt tattatatgt gtgaggaggg ggtcgatgat ttgttgggtt 1980
tgaggggtga gttgtaaggt atagatgcgg aagtttttat ttagattttt ggattttttt 2040
ttttttattt tgttatttat tatttgtttg agtcgtttgt atagttgttt ttttgttttc 2100
ggttgttgtt tgttaagatg ttttagagat attttatttc ggtttcgtat ttttattttc 2160
gtattttagt tttttattaa agagtaggta ggtgtgatag tgttgtgtgg tttttgttta 2220
gattaagttt atattcgttt tcggttatat ttatttatat atatttaaat ttattattgt 2280
tttttaatat gtgtttattt tgaattatag atattaaaaa atagatattg gttagaagag 2340
tattttgagg tgtttattta aacgtgtttt aagtttgttt tcgagttttt cgggcggtcg 2400
tatttatata ttcgtacgac gatgatttgt ttgtattaaa tttgtatata ttacgaatag 2460

gcgtttttta ttatatttaa ttacgcgtga ttttaatagt ttttatattt cgtagaatcg 2520
ggttttagtt ttattgtgtt tgagcgttta gtttagtaga ggtagggatt aaggcgtttg 2580
tcgtttatag cgtttagagt ggtaagaaag aagtttttta gcgtttttat tatttttcgg 2640
tggaacgcgt agcgcgcgat ttgttgtagt cggttcgggc gttgtttgtg cggtgagcgt 2700
agtagcggta tttcggggtt gtgggcggag gtaaggattt tagtttgagg ttttttgagg 2760
tagagtagaa attagttttt aggtgtgtcg ttcgtggcgc gagttacgga attttaggta 2820
tttcggggtc gtgttcgtat tttgtttttt agttttagaa agcgtcgaaa tttttttagta 2880
cgatattttt gtttcgcgtt atattttttt tttttagggg gttggtggtt ttcgtgttta 2940
aaggaggtag cgggaacgtt taatttttaa gatgttttta attgattttt ttttttttaa 3000
ttatagcgag ggaataataa ttcggggttg gggattgggt atttaagatt cgtttttcgt 3060
agggtcgtcg gtagggcggt tataggattc gggattggaa ttggttcggg aggatatcgg 3120
cgcgtgttag cgtagagcgt gaaataggta acgtgcgcgt agagtagggt ttggatggcg 3180
gattttgggt ataatgagtt tcggattttg aaggaagata atttttgttg agattttgtt 3240
cggagttttg gagtttttta gtagtttagg ttcggatttt cggtttgggt tttcggtgta 3300
gagttgtttg aagagttgcg aaggtcgtag tgcgggtatt gagtggggcg tagtgtttgg 3360
gtttttgtta ttggggttgt agtagaaggg tatttgcgtt tagttattat atattttagc 3420
ggaaatgatt tttttttaag attagaggtt tttaatataa gaattttttg aaatcgtttg 3480
taagtgtgtg tgtttgtgat gtgtgttttt ttaaagagag agtttatagt tttcgttcgt 3540
gttacggtga tcgtaaaatg aaagttagta gacgtagatt ttgtggttat gttattttga 3600
ggggtcggaa ttttattttt gattgtggtg tgtgttttta gtttttagaa tagatacgta 3660
ttttttatta ttaaattttt ggtagttata aattaaaggt tggtgtcgtg gggcgttaat 3720
tagagtttgc gtttagaggg cgttagtgta gtttcgagta gttttaagat tgattgtaaa 3780
agtttgtttt tttaggatag gagggacggt tattaattta ttttttaagt attttttgag 3840
tttgtaggaa ttgtttggtt ttttgttggg ggtcgaaagt ttttttcgtt tttgtttgaa 3900
aattattttt tttagtaatt agtattatgt taggtatttt agtagacgat tcgttaaagt 3960
taaatgaatg aaaatttatt ttggtaaatt tattagtttt ataaggtagt ttgtagaaaa 4020
ttcggattta gttagagcgg tatttcgttt tgtttttcga aatttataaa ttatttagtt 4080
agattttttt tattttattt gttatagtaa gtcggttttt aggtagaagt ttttaggaat 4140
gaggtaggga agtaaggatt tcgtttaggg ttttttttaa agattgagat tttttggtgt 4200
aaaatgcgaa gttatttttt gagtttattt cggatttatt gagcgagcga aggaagggta 4260
gagtttttcg tgagcggatt atttagattt tatttcgtta tttttggaat ttttttgagt 4320
tttagtatta aatagagtat ggtttaacgg aagtcgttaa ggaaaatgtt gattttaaaa 4380
aaatagtcgt ttgattattt gatttttgat ttttattgtt ttatttttta atttaaaaat 4440
ttggaaatta gggttagttt tgaaataatt tgattttgga aaaaaatatt tttataatat 4500
tttaataaag tttaattgta atgtttgttt ttattcgttt agattagatt tttgtatttt 4560
ttgtttaaga aataaaaata aatatgaagt ttggttgaaa atttttacgt gaatttttac 4620
gtgattaaaa aagttatatt tgaaggtttg agtttagagt ttaagttaaa tttattattg 4680
ttttataatt tttttataat atggatttaa ttataaaatt ttagtagtag taatagtatt 4740
attagtaata gtaatataat agtttataat atatatattt tttttagttt ggtagaaacg 4800
tggagttatt aaattaattg gaaaaaattt taattgaaaa tcgattgtat tgtttaaaga 4860
agggtagaaa ttaaagtttt ttattatcgt gtttattgaa gaatatgtta agagaagtag 4920
ttgtttttga attatttttt taagatattt ttagattgta gtaaagttgt ttttggggaa 4980
gggagtatag aaatggttgt taaagagata gcgagattta taataattaa gatatagaaa 5040
tagtttaagt gtttattgat gaatgaatta ttaaagaaaa tgtggtgtgt gtttattata 5100
tatatataat ggaatattag ttagtagtat tatttagaag aaggaaatat tgttatttgt 5160
gttaatatgg ttgaatttgg ggaatattat gatatataaa ataagtgagg tatagaaaga 5220
tacgtaatat atgattttat ttatatgtag aattttaaaa aataagtcga atttagtaat 5280
aaaggttaga atggtgatta tttggggttg cggttggggg taaaagagaa atattagtta 5340
aaggatataa ttttttagtt ataagatgag taagttttgg agatttaata tatagtttgg 5400
tgattatagt taatatatgt ttgaaatttg taaagagagt agattttaag agttttaagc 5460
gtatatatat atatatatat ataggaaatt atgtgaggta atgatatgtt aattaatttg 5520
attgttgtaa ttattttata aagtatatat gtattaaaat attatattgt atattttaaa 5580
aataaaaatt tttaatatgg aaataaaagt gaaaaaaaaa aagagtgaga gatagttgaa 5640
attttttagt gggaattttg ttgaagattt tagaagaggg gtatttgtat aaatattgaa 5700
gaggatatta atgttttatt aataatttag atatagaatt atttaaaaga tgttgtatgt 5760
gtagtaagta tgaaaagtta tataaattat attaaattga aataaagaaa agatattata 5820
aatattataa ttattattaa ttaagtttta ttcgtgtgtt aggtattgtg tgtatattat 5880
tttttttatt tttaatagtt taataagata gatattttta tttttgttgt atatgtaagg 5940
gaatagtaag agtatttggt ttgaattaat tatatttttt aggttttttt tgaggatttg 6000
aaatatagtt tatttttttg ttatgttagg taagaggttt ggtttttttgg atttaggtat 6060
tgatttagag gaagatatgg gtatgtttgt ttgttttagg gtta 6104

<210> 161

<211> 5976
<212> DNA
<213> Artificial Sequence

<220>
<223> chemically treated genomic DNA (Homo sapiens)

<400> 161

agaggggtat tttttatttt aatttgaatt ttatgattgt attttttgtt aggagatgaa 60
agggataaga ttgtttgaag gattaatttt tagtttttag gtaggttagt ggttaaaata 120
tttaggaagt gaaagtttgt tttttgttat aaggaaattt ggagtttgtg attttattat 180
gcggggtttg ttatttgtta ggtgataggg ttttttattt atttgcggtg atttgaaggg 240
taaggttttt ggttaaatat tttttttttt tagtgatgtt tgaaatttta ttgttagcgt 300
tggattttag taagatttta gagaaagttt aatttttgga gtttgggttt tttttagttg 360
agatgttagg aaagtaatgg gttttattat ttgtacggat aggtttgtgt atagttttag 420
gggatttagg gattttatta gagtttattt atagatatta ggttaaaagt tttggatagt 480
atttataatt tttttgaaag aagaaaagtt tttttttaga gtttttttta aagtgtaaat 540
ggttttggga ggttcgaaat gatgagttgg tcggtatgaa agaatttttt ttagtagatg 600
taagagataa tttgaggagt tttagtggcg gggtgggggg aaggagagtt tgggggaggg 660
gaggttttag gtagtttata atttttttta gaattggaaa gggaatgtat tgggttaaga 720
gggttaatta tttatttttg tagagtgttt ataagaattt tatgaattaa gtgttattaa 780
tatttttatt ttatagatga ggaaattaag atggtggaga taaaatgatt tatgtttttt 840
aattaatggt agagttagaa gtgaatttag gttggagtga agtggtttta gtgtttaaat 900
gattttttta ttttagtttt tagagtagtt gggattatag gtatgtattg ttatgtttag 960
ttaatttaaa ataatatttg ttggggagat ggggttttat tatgttgttt agattggttt 1020
taaatttttg ggtttaagta atttttttat tttttaatta gttttttaaa gtgttgcgat 1080
tatagttgtg agttattagg tttgtttagg tatttttgtt tgatataaga gaaaatagag 1140
ggtaggtata gtggtttata tttgtaattt tagtattttg ggaggttgag gtaggtggat 1200
tatttgaggt taggagtttg agattagttt ggttaatatg gcgaaatttc gtttttatta 1260
aaatataaaa attagttaga tatggtgacg tgtgtttgta attatagtta tttaggaggt 1320
tgaggtataa gaattttttg aatttgggag gtagagagtg tagtgagtcg agatcgcgtt 1380
attgtatttt agtcggggta atagagtaag attttgttat taaataaata aataggaaat 1440
agagaagtaa tttagtgtgt tgcgaaagtt atatagtatt tgttttttat aaggagtttt 1500
tttgtgtttt cgttttattt tggatatttg atttattgtt tttggagttt ggagaaggat 1560
ttgtcgagaa gtttgaagaa tgtattaggt ttgatttgat tttgttgttt ttaattgttt 1620
tttaaaattt tttttattga attattagag tgtaatggaa gagtcgatga gagtcgttgt 1680
agagttacga ggttaaggtt tgttgggtag aagaaagtaa agttttaaag gtttttaggg 1740
tttagcgatg gtataaaata tgtattttta tataattgag aggaaaaaaa taagtagata 1800
gtatagtttt atgggggaaa cgtttttttt tgtattttta ttattttgtt taaggtacga 1860
ttttagtggg aaaggaaata tcggagtttt ttttttttga gttttttggt gggttaagag 1920
tagagggagt taaaatacga tatttaaaag atttgattgg aaatttatta aaattgaggg 1980
gataggttgt tggggggggc ggtgttgggt tgtttagaaa tcgtttgtat ttgagaaatt 2040
atttgatgga tttagaggta tttttagttt tgggtgaaat attttaaata tggttttttt 2100
taggaggaag cgaaataggg gttgggtttt ttgaaagagt aatttttatt ttaaatattt 2160
attgagtagt tgttatgatg agttaagaat tttgtttagc gattgttttt gtattagttt 2220
atgttaattt atttatataa aggagaatat tgattttttt aaggttatag ggttagtgag 2280
tggttgattt tatttaatgt tgtgtagttt taattatgtt tgaaggataa agggaaagga 2340
ggtgtttttg tcggggtaga taagttattt agacgtggtt ggttataggg agttagatag 2400
ggaagttgtt taatttaata tttgtttgtt tattttgttt ttgttttttg atggaggacg 2460
gcgggtgttg gagggttatt ggtttaggat ttgagatttt tggtttttat ttattttatt 2520
tttgatttta aaattatttt attggggata gaattttttg gagaaggtga tggtggttat 2580
ggtgtggttg tggtattaaa agttttattt tgggggttag aagattttgg gttttggttt 2640
ttggttttga tattgatttg tttatgtgat attgaataag ttatttgtaa attataggtt 2700
tgggtgaatt ttaaaggttt tttttaggtt aatattaaaa aatagattga aaattgtagt 2760
tttagttgga agattgggat tatatgagat gaagttgatt gttagaagaa aggttttgga 2820
attagagagg ttggttttag ttttgcgtta ttttttattg gttttaattt ttttaagttt 2880
tagttgttta tttattatat gggaataata atatagtttt tgggttgttt tgaaatgagt 2940
ttaaatgagt tggttagtgg ttgggtacgg tggtttatgt ttgtaatttt agtattttgg 3000
gaggttgagg taggtggatt atttgaggat aggagtttaa gattagtttg gttaatatga 3060
tgaaattttg tttttattaa aaatataaaa attagttagt cgtggtggcg tatgtttgta 3120
attttagtta tttaggaggt tgatagagga gaatcgtttg aatttaagag gcgaaggttg 3180
tagtgagtta agaatatatt attgtatttt agtttgggcg atagagtagg atttcgtttt 3240

aaaaaaaaaa gagttggtta gtgttaaatg tttagtatag agattggtat agtaattttt 3300
aatgtttagt atttattgtt attatttttt tttttttttt ttttttgaga tagagttttg 3360
ttttgtcgtt taggttggag tatagtggcg cgatttcggt ttattgtaag ttttattttt 3420
taggtttatg ttattttttt gttttagttt ttcgagtagt tgggattata ggcgtttatt 3480
attacgtttg gttaattttt tgtattttta gtagagacgg ggttttattg cgttagttag 3540
gatggtttta attttttgat ttcgtgattt gttcgtttcg gttttttaaa gtgttgggat 3600
tataggcgtg agttattatg tttggtttta ttgttattat ttttattttt tatttttgta 3660
tagagtattt atggtttaag aaatatttgt tattttaatt gtatgggagt tttataatag 3720
tatagggaga tatttttgat tattattttt attaggaggg tggagaaatt gaggttttgg 3780
gaggtggttt tgatttaggg aattaatttg ttgatttatt aatttatgaa gttttatagt 3840
taaaaaagat tagattaaaa aatgagaatt tagtaaaggg gttgaggtag gaggatcgtt 3900
tgagtttaga aatttgagat tagtttcggt aatatagtga gatttttttt ttagaaaaat 3960
tttttaaaaa attaggtcgt tcgaggtaga gtgtagtggt ttacgtttgt aatttaatat 4020
tttaggaggt tgaagagggt ggattatttg aggttaggag ttttagatta gtttggttaa 4080
tatggtgaaa tttcgtttgt attaaaaata taaaattagt cggtgtggtg gtatacgttt 4140
gtagttttag ttatttaata ggttgagata ggagagtttt ttgaattcgg taggcggagg 4200
ttgtagtgag tcgagatcgt gttattgtat tttagtttgg gtaagataga gcgagatttc 4260
gtttttaaaa atataaataa aataaataaa taaaaaatta ggttgttagt ttagtggttt 4320
atggtttata tttgaaattt tagtattttg ggaggttaag gtaggaggat cgttttagtt 4380
taggagttcg agattaggtt gggtaatata gggagatata gcgtttttat tgtttttgtt 4440
cgtttcgatt tgttttttta taaaaaggta aaagaaaaaa aaattagttt ggcgtggtgg 4500
tgtgtatttg tatttttagt tattagagag gttggggtta gaggatcgtt tgagtttagg 4560
agttcgaggt tgtagtgagt tgtgatcgta ttattgtatt ttagtttggg tgaaagagtg 4620
agattttatt tttaaaacga ataaataaaa aattttaaaa aataaaagaa tttagttaag 4680
tgtaaaagtt ttttttgatt ttaggtttta gtgagttatc ggcggggttg ggattcgaat 4740
ttagtggaat tagaatcgtg taggttttat aatttattta gattttagta attttaggtt 4800
agagggttat cgcgtttatg cgaggtcggg tgggcgggtc gttagtttcg ttttggggag 4860
gggttcgcgt tgttgattgg ttgtggtcgg taggtgaatt tttagttaat tagcggtacg 4920
gggggcggtg ttttcggggt ttatttggtt gtagttacgt attttttttt agtggcgtcg 4980
gaattgtaaa gtatttgtga gtttgcggaa gttagtttag attttagttc gttttagttc 5040
ggttcgattc gatcgtattc ggcgtttgtt ttcgttcggc gttttcggtt agttatgggt 5100
ttttggagtc gtagtttttc ggcgttgttg ttgttgttgt aggtatttcg gattttttga 5160
tttgcgaggg acgtattcgg gtcgtaagtt tcgcgtttta gttttgcgtt tttttttttt 5220
tcgtcgttat cgtttttttt tttttaagaa agttcgggtt ttgaggagcg gagcggtttg 5280
gaagtttcgc gcgtttcgga ttttttagtg atgggagtgg ggggtgggtg gtgaggggcg 5340
agcgcggttt ttttgttttt tttagcgtag atcgaggcgg gggcgtttgg tcgcggagtt 5400
cgcggggtgg gttcgcgcgg gcggtggggg cgtgaagcgg ggtgtagggg gtggggtgtg 5460
gagaaggggt gttttggtgt aagtcgaggg ggagttagga gtcgtgggga cgattttcga 5520
gggaaggaga ggggtattcg tagaaataaa ggtatttgtt atgttaagaa aggtcgtaaa 5580
taggagtgag ggtttcgggg ataagaaagt gaggtcggag gaggtgggag cgtttttcgt 5640
tttgaggagt ggtgtatttt cggtttaagg aaagtggggt attggagaat aaagatattt 5700
ttaataaaat gagaaaggag attgaaaggg aacggtgggt taggttttga gggggtgatt 5760
cggcggtttt ttttcgggag tttttggggg ttcggcggtc gtaggtttcg ggtgggggga 5820
gggtgacgtc gttgttcgtt cgtttcgggg ttgcgggttg gggttttttt ttaatttcga 5880
cgtcgggagc gagggagggg cggcgttgtt ggtttcggtg agtaggaggg aatttttcga 5940
gttattcggt tttatttatt ttttttttat tttagg 5976

<210> 162
<211> 5976
<212> DNA
<213> Artificial Sequence

<220>
<223> chemically treated genomic DNA (Homo sapiens)

<400> 162

tttggggtgg gggaaaggta gatggaatcg ggtgattcgg agggtttttt tttgtttatc 60
gaaattaata gcgtcgtttt tttttcgttt tcggcgtcgg gattggggga ggattttagt 120
tcgtagtttc gggacgggcg ggtagcgacg ttattttttt ttatttcgaa atttacggtc 180
gtcgagtttt taggaatttt cgggaggggg tcgtcgagtt atttttttaa gatttagttt 240
atcgtttttt tttagttttt ttttttattt tattggagat gtttttattt tttagtattt 300
tatttttttt agatcgggaa tgtattattt tttagagcga ggggcgtttt tatttttttc 360

gattttattt ttttattttc gggattttta tttttattta cgattttttt tggtatggta 420
ggtgttttta tttttacgga tgtttttttt tttttttcga agatcgtttt tacgattttt 480
ggtttttttt cgatttgtat tagggtattt tttttttata ttttattttt tatatttcgt 540
tttacgtttt tatcgttcgc gcgagtttat ttcgcggatt tcgcggttag acgttttcgt 600
ttcggtttgc gttggagggg gtaggaaagt cgcgttcgtt ttttattatt tattttttat 660
ttttattatt ggggggttcg gagcgcgcga ggtttttagg tcgtttcgtt ttttaggatt 720
cgaatttttt tggaagaagg gaagcggtga cgacgggaga ggaaggggcg tagggttggg 780
gcgcggagtt tgcggttcga atgcgttttt cgtaagttag gggattcggg gtatttgtag 840
tagtagtagt agcgtcgaga ggttgcggtt ttaagggttt atggttggtc ggggacgtcg 900
agcgagggta ggcgtcgggt gcggtcgggt cgggtcgggt tggagcgggt tggagtttga 960
attgattttc gtaagtttat aggtgttttg tagtttcgac gttattgaga gggggtgcgt 1020
ggttgtagtt aggtgagttt cggaggtatc gtttttcgta tcgttgattg gttgagggtt 1080
tatttgtcgg ttatagttaa ttagtagcgc ggattttttt ttagggcgga gttgacggtt 1140
cgtttattcg gtttcgtata gacgcggtga ttttttagtt tggagttgtt agggtttagg 1200
tgggttatgg gatttgtacg gttttgattt tattgggttc gaattttagt ttcgtcggtg 1260
gtttattaag atttgggatt agaaagggtt tttatatttg gttgagtttt tttgtttttt 1320
gggatttttt gtttgttcgt tttggagatg gggttttatt tttttattta agttggagtg 1380
tagtggtgcg attatagttt attgtagttt cgaatttttg ggtttaagcg gttttttggt 1440
tttagttttt ttagtagttg ggagtatagg tgtatattat tacgttaggt taattttttt 1500
tttttttgtt tttttgtaga gagataagtc ggggcggata ggggtagtgg gggcgttgtg 1560
tttttttgta ttgtttagtt tggtttcgaa tttttgggtt gaagcgattt ttttgttttg 1620
gttttttaaa gtgttaggat tttaggtgtg agttatgagt tattgagtta gtagtttaat 1680
tttttgtttg tttgttttgt ttgtattttt tgagacggag tttcgttttg ttttgtttag 1740
gttggagtgt agtggtacga tttcggttta ttgtaatttt cgtttgtcgg gtttaagaga 1800
tttttttgtt ttagtttatt gagtagttgg gattataggc gtgtgttatt atatcggtta 1860
attttgtatt tttagtatag acggggtttt attatgttgg ttaggttggt ttggaatttt 1920
tgattttagg tgatttattt tttttagttt tttgaagtgt tggattatag gcgtgagtta 1980
ttgtattttg tttcgagcgg tttaattttt taaaaaattt ttttagagag gggattttat 2040
tatgttgtcg aggttgattt taaatttttg aatttaggcg atttttttgt tttagttttt 2100
ttattgagtt tttatttttt aatttagttt tttttaattg tagagtttta tgggttagtg 2160
agttagtaaa ttgatttttt aggttaggat tattttttaa agttttagtt tttttatttt 2220
tttaatggga ataatgatta gaaatgtttt tttatgttgt tgtgggattt ttatataatt 2280
aaaatgataa atgttttttg agttataaat gttttgtata gaagtgaggg gtaaaaatag 2340
taataatagg gttaggtatg gtggtttacg tttgtaattt tagtattttg ggaggtcgag 2400
gcgggtagat tacgaggtta ggagattgag attattttgg ttaacgtagt gaaatttcgt 2460
ttttattaaa aatataaaaa attagttagg cgtggtggtg ggcgtttgta gttttagtta 2520
ttcgggaggt tgaggtagga gaatggtatg aatttgggag gtggagtttg tagtgagtcg 2580
agatcgcgtt attgtatttt agtttgggcg atagagtaag attttgtttt aaaaaaaaaa 2640
aaaaaaaaaa aatagtaata ataggtgttg gatattgaag attattgtgt tagtttttgt 2700
gttaagtatt tgatattgat tagttttttt ttttttgaga cggagttttg ttttgtcgtt 2760
taggttggag tgtaatggtg tgtttttggt ttattgtaat tttcgttttt tgggtttaag 2820
cggttttttt ttattagttt tttgagtagt tgggattata ggtatgcgtt attacgattg 2880
gttaattttt gtatttttag tagagatagg gttttattat gttggttagg ttggttttga 2940
atttttgttt ttagatgatt tatttgtttt agttttttaa agtgttggga ttataggtat 3000
gagttatcgt gtttagttat tgattagttt atttaagttt attttaaaat aatttaagag 3060
ttgtattatt atttttatat gatagatggg taattgaagt ttagagagat tgaggttaat 3120
gagaggtggc gtaggattgg aattaatttt tttgatttta gggttttttt tttaataatt 3180
agttttattt tatgtggttt tagtttttta gttggagttg tagtttttag tttgtttttt 3240
gatgttgatt tggaagagat ttttagagtt tatttaaatt tatgatttgt aaataatttg 3300
tttaatgtta tataggtagg ttaatgttaa agttaaagat taaaatttag gattttttga 3360
tttttagagt ggggttttta atgttatagt tatattatgg ttattattat tttttttaga 3420
gaattttgtt tttagtgaaa taattttagg attaaaaata gaatagatag gggttaaagg 3480
ttttaggttt tgggttagtg gttttttagt attcgtcgtt ttttattaaa agataaaaat 3540
agaatagata ggtaaatgtt aggttagata atttttttgt ttgatttttt gtggttagtt 3600
acgtttgggt ggtttgttta tttcgataag ggtatttttt ttttttttat tttttaagta 3660
tggttagaat tatatagtat taagtggagt tagttattta ttagttttgt gattttgggg 3720
aagttagtgt ttttttttgt ataagtgaat tggtatgagt taatataaaa ataatcgtta 3780
agtagagttt ttggtttatt atagtaattg tttaataaat atttggagta ggggttattt 3840
ttttaaaagg tttagttttt atttcgtttt tttttgggga aagttatgtt tggaatattt 3900
tatttagagt taaaggtgtt tttgagttta ttaagtaatt ttttaaatgt agacgatttt 3960
tgggtagttt agtatcgttt tttttaataa tttgtttttt taattttaat gagtttttaa 4020
ttaggttttt tggatgtcgt attttggttt tttttgtttt taatttatta ggaggtttag 4080
agggagaagg tttcggtatt ttttttttta ttaggatcgt gttttgaata agataatgga 4140

aatatagaaa gaggcgtttt ttttatgggg ttgtattgtt tgtttatttt ttttttttta 4200
gttatgtaaa aatatatatt ttatattatc gttgggtttt gggagttttt agggttttat 4260
ttttttttat ttagtaagtt ttggtttcgt aattttgtag cgatttttat cgattttttt 4320
attatatttt gataatttaa taaaaaggat tttagaaaat aattaggggt agtaaagtta 4380
ggttaggttt gatgtatttt ttagattttt cggtaagttt ttttttaagt tttaaagata 4440
gtagattagg tgtttagggt gaggcgggaa tatagagggg ttttttgtgg aagataggtg 4500
ttgtgtgatt ttcgtaatat attgaattgt ttttttgttt tttatttatt tatttagtga 4560
taaggttttg ttttgttgtt tcggttggag tgtaatggcg cgatttcggt ttattgtatt 4620
ttttgttttt taggtttaag agatttttgt gttttagttt tttgagtagt tgtgattata 4680
gatatacgtt attatgtttg gttaattttt gtattttagt agagacggga tttcgttatg 4740
ttggttaggt tggttttaaa tttttgattt taagtgattt atttatttta gttttttaaa 4800
gtgttgggat tataggtgtg agttattgtg tttgtttttt gttttttttt gtattaaata 4860
gggatattta agtaggtttg gtggtttata attgtaatcg tagtattttg ggaggttagt 4920
tgggaggtga gaagattatt tgagtttagg agtttgagat tagtttgggt aatatggtga 4980
aattttattt ttttaataaa tattattttg aattagttgg atatggtagt gtatgtttgt 5040
agttttagtt attttggagg ttgaggtagg aggattattt gagtattgga gttattttat 5100
tttagtttgg atttattttt ggttttgtta ttagttgaga agtataagtt attttatttt 5160
tattattttg gtttttttat ttgtaaaatg ggaatgttga tagtatttaa tttataaggt 5220
ttttgtaaat attttgtaaa ggtgagtgat tagttttttt ggtttaatat attttttttt 5280
tagttttagg aaaaattgtg agttgtttgg agtttttttt tttttaggtt tttttttttt 5340
tattttcgtt attaaagttt tttaggttat tttttgtatt tattggagaa aattttttta 5400
tgtcggttag tttattattt cgaatttttt agggttattt gtattttaaa gagggtttta 5460
gagaaagatt tttttttttt taaaggagtt gtaaatgttg tttagggttt ttaatttggt 5520
gtttgtggat gggttttggt ggggtttttg aattttttga gattgtatat aaatttgttc 5580
gtgtaggtga taaggtttat tgttttttta atattttaat tggggaaaat ttaaatttta 5640
ggagttgagt tttttttgag attttgttga gatttagcgt taatagtgga attttaggta 5700
ttattgggga aaagaaatgt ttggttagag gttttgtttt ttagattatc gtagatagat 5760
aagagatttt gttatttggt agataataga tttcgtatga tgggattata gattttaggt 5820
tttttttatgg tagaggatag attttatttt tttgggtgtt ttggttattg gtttgtttgg 5880
aggttaaaga ttaatttttt aggtagtttt gttttttttta tttttggtta aggggtgtag 5940
ttatagggtt tagattgggg tgggaggtgt tttttt 5976

<210> 163
<211> 2501
<212> DNA
<213> Artificial Sequence

<220>
<223> chemically treated genomic DNA (Homo sapiens)

<400> 163
   tagaagtttt tttttagagt gtgtttgggt atatatttaa gtgtatggtt gtaaattttt 60
   ttttttaaag tattgaatag tattagatat ttagtaggta tttaagaaat attgaatgtc 120
   gtggtggtgg tgagttagaa gttataaaaa aaattttttt ttaaaaataa taataaaaag 180
   aattatttta ttgtgaagtt tagtattata aaaatttaaa taatttatta taagttttta 240
   ttaaaaaaaa tttttttttt aaagtaaata gatagataat gtttagttta tttgaaatgt 300
   ttgaaagtag aggggtttta aggtagtggg agaaggtgtt tgttttttgt tggatatttg 360
   ataattagtt ttttggatgg tttggatgta taggagcgaa ggtgtagata gtagtggggt 420
   ttagagtggg gttttgaggt tgtgtcgtgg tttttttggg gtttagttat aattttggtt 480
   tgattttagg gcgaggtagg ttaagggggt ttgttattgt gtttttttat ttttatttgg 540
   gtttttattt ttatagtaga ggagaaagaa gtttgttttt ttcgaggtta gttgcgttag 600
   aggaagaaga ttgggtatgt ttgggtagag atttttagat tttgagtagt ttgagatgtt 660
   agtaattgta gttgttttaa gtttgggttt tgttttttag tgggattttt gtttagatga 720
   ataatttatt ttttgtaatt ttttaaaagt aaaattgtaa atgttttagg tatagaaagg 780
   aggtaaaggt gaagtttagg ggaggttagg ggtgtgaggt agatgggagc ggatagatat 840
   attatttatt tttgtgtttg ttagaagaat tagtagatat ttttagaatt gttttttatt 900
   tatgttattt ttataaatta tttgtaaatg agggttattt ggtatttttg ttattttgga 960
   gttatagaaa taaaggatga taagtagaga gtttcgggta ggaggtaaaa gttttgtgtt 1020
   ttaattatag ttattttttt gttgtatgat ttgagttagg ttattagatt tttttgagtt 1080
   ttagtttttt tagtagtgta tacgggttat gtggggagta tttaggagat agataattta 1140
   ttcgttaaat tttttttttt ttggttaata aagttgttgt aattataggg attttttttg 1200
   tttaggtgag tgtagggtgt agggagattg gtttaatgtt taattttttt gtttttttgg 1260
   agattaggtt gttttttttt ggtagttttt ttaatttttt ttttttcgga agtatgtgat 1320
   aattaataat tttgtatatt taagtttagt ggattttaat ttttttattt gtgaaataaa 1380
   cgggattgaa aaattatttt ggttttaaga tgttttgttg gggtgtttag gtgttttagg 1440
   tgtttttggg agaggtgatt tagtgaggga ttagtgggaa tagaggtgat attgtggggt 1500
   ttttttggaa attgtagaga ggtgtatcgt ttttataatt tatgaatttt tatgtattaa 1560
   tgttattttt ttgatttttt tagttgtatt gggtaaattt ttgtttgtta gagtgggtta 1620
   gcggtgagtt agaaaggggg tttattttaa tagtgttgtg tttttttgga gagtgttaat 1680
   ttatttttta agtaaaaaaa gttagatttg tggtttattt cgtggggaaa tgtgtttagc 1740
   gtattaacgt aggcgaggga ttgggggagg agggaagtgt ttttttgtag tacgcgaggt 1800
   ttcgggatcg gttggtttgt tggaattcgg ttaggtttag ttggttcggc gttgggtagt 1860
   taggagtttg ggtttcgggg agggcggttt cgggcggcgc ggtgggtcga gcgcgggttt 1920
   cgtttttttg aggcgggttc gggcggggcg gttgtatatt agggtcgcgt tgagttgcgt 1980
   tagttgaggt gtgagtagtt gtcgaagtta gttttttgtg gagtcggagt tgggcgcgga 2040
   ttcgtcgagg tatcgaggta tttagaggag gtgagagagc ggcggtagat aataggggat 2100
   ttcgggtcgg cggtttagag tcgagttaag cgtgttcgcg tgtgtttttg cgtgttcgcg 2160
   aggatgcgtg ttcgcgggtg tgtgttgcgt ttataggtgt ttttgcggta ggtgaatgac 2220
   gggcgtgggt cggtgcgcgt tcggtttgcg tatacggtgt ttttaagtgc gcgggtgacg 2280
   agagtcggga tgtgtcggag atttcggggc ggagagcggg attataagta taggaatttt 2340
   tggttacgtt tttcgttttt ggaaatttag ttggggcgag ggagggcgtg gacgggatcg 2400
   ttttgggagt tcgtttttgg ttgcggttgg ttttaggttt taggcgtagt ttgttcgcgg 2460
   cgtggggatg aagttcgtgt ttttggaggg gtttaggaag g 2501

<210> 164
<211> 2501
<212> DNA
<213> Artificial Sequence

<220>
<223> chemically treated genomic DNA (Homo sapiens)

<400> 164
   ttttttgggg tttttttagg gatacggatt ttatttttac gtcgcgagta aattgcgttt 60
   ggggtttgga gttaatcgta gttaaaggcg agtttttaga acggtttcgt ttacgttttt 120
   tttcgtttta gttgggtttt taggggcggg gagcgtgatt agggattttt gtatttgtaa 180
   tttcgttttt cgtttcgggg ttttcggtat atttcgattt tcgttattcg cgtatttaga 240
   gatatcgtgt gcgtaagtcg agcgcgtatc gatttacgtt cgttatttat ttgtcgtaga 300
   aatatttgtg aacgtagtat atattcgcga atacgtattt tcgcggatac gtagggatat 360
   acgcgggtac gtttggttcg gttttgggtc gtcggttcgg ggttttttgt tgtttgtcgt 420
   cgttttttta ttttttttga gtgtttcggt gtttcggcga attcgcgttt agtttcggtt 480
   ttataaggaa ttgatttcgg tagttgttta tattttagtt ggcgtagttt agcgcggttt 540
   tgatatataa tcgtttcgtt cgggttcgtt ttaaggaggc gggattcgcg ttcggtttat 600
   cgcgtcgttc gggatcgttt ttttcggggt ttaggttttt ggttgtttag cgtcgagtta 660
   gttgagtttg gtcgagtttt agtaggttag tcggtttcgg aatttcgcgt gttgtaggag 720
   ggtatttttt ttttttttta gtttttcgtt tgcgttggtg cgttggatat atttttttac 780
   gaagtgagtt ataaatttgg ttttttttat ttggagaatg agttggtatt ttttaggagg 840
   atatagtatt gttagaatga gttttttttt tggtttatcg ttgatttatt ttggtaggta 900
   aggatttatt taatgtagtt gaaaagatta ggaggatgat attaatatat aaaaatttat 960
   aaattataaa aacgatgtat ttttttgtaa tttttagaaa agttttataa tattattttt 1020
   atttttattg attttttatt aggttatttt ttttagaagt atttggagta tttagatatt 1080
   ttaataaagt attttgaggt tagaatgatt ttttagtttc gtttatttta tagatgagga 1140
   aattgaggtt tattgaattt aagtatataa agttgttgat tgttatatgt tttcgggaag 1200
   gagggaattg gagagattat taaaaaaggg taatttgatt tttagggaaa tagaagaatt 1260
   ggatattgaa ttaatttttt tatattttat atttatttga atagaagaaa tttttgtggt 1320
   tgtagtagtt ttgttggtta ggaaggggag gatttgacga gtgagttgtt tgttttttga 1380
   atatttttta tatagttcgt atatattgtt ggggaaattg gggtttagag aagtttggtg 1440
   atttaattta gattatgtag taaagaaatg attatagttg gaatatagga tttttgtttt 1500
   ttgttcgggg ttttttgttt gttatttttt atttttgtgg ttttaaaatg ataaaaatgt 1560
   taaataattt ttatttgtag atggtttatg gagatgatat aaataaagga taattttgga 1620
   agtgtttatt ggtttttttg atagatatag aaatgagtga tgtgtttatt cgtttttatt 1680
   tattttatat ttttgatttt ttttggattt tatttttgtt ttttttttgt gtttgaaata 1740
   tttgtagttt tgtttttaaa aaattgtaga ggatggattg tttatttgaa tagaaatttt 1800
   attaaaaaat agaatttagg tttggagtag ttataattat tgatatttta ggttgtttag 1860
   agtttggaaa tttttgttta gatatgttta gttttttttt tttaacgtag ttgatttcgg 1920
   ggaggatagg tttttttttt ttttgttgtg gggatgggag tttaggtagg ggtgggagga 1980
   tatagtagta gatttttttg gtttgtttcg ttttggagtt aggttaggat tgtggttaaa 2040
   ttttagaaag gttacggtat agttttagga ttttatttta agttttattg ttgtttgtat 2100
   tttcgttttt atatatttaa attatttaaa gggttggttg ttaaatgttt agtagaggat 2160
   aggtattttt ttttattgtt ttgaagtttt tttgttttta ggtattttaa atagattaga 2220
   tattgtttgt ttgtttattt tggggagaaa atttttttta ataaaggttt gtaatgaatt 2280
   atttaaattt ttgtggtatt gagttttata atgaaataat tttttttgtt gttgtttttg 2340
   ggaaagaatt ttttttataa tttttagttt attattatta cgatatttaa tattttttaa 2400
   gtatttatta agtgtttagt attatttagt gttttaaaaa aaaaagtttg taattatgta 2460
   tttgaatgtg tatttagata tattttaagg gaggattttt g 2501

<210> 165
<211> 3486
<212> DNA
<213> Artificial Sequence

<220>
<223> chemically treated genomic DNA (Homo sapiens)

<400> 165

aattaaagag gatgagattt tttgtttgat tttaggtgaa tttttttttg ttagttaggt 60
aggaagtttt gatttgaaaa ttagttttgg gtattgtttt ttttattgtt ttttgggtat 120
taatttttgt tttttaattt tagttgaatt agtggatggt gatattatag gtttaagata 180
gttgtattta aatattagtg attataggtt atattaagga aatatttgta ggtaatttag 240
ggtttgggaa ggagttattt ttagttattt gtaagatagt aggatttgta gattatagta 300
taattaaatt tagataaaat tttgaattag tgagaattat taggaaggaa aggaatagaa 360
aatgaattaa tttgagtgtt aggagatttg tatttagttt tgatttcggt attaatcgaa 420
tgtatgtttt tggataggaa atttttttga gtttcgattt ttttcgtggt aaaaaggaga 480
gggttaaatt atagggtttc gagggttttt tttagttgtt atatttggga gcgtatgaga 540
tgaggtaggt atataaagtg gataagatgt ggttaagaaa ataagttata tattaagttt 600
atttgtagta taggtgttta agaaaatttt gttgttgtgt aatattagaa cggaaggttg 660
gtttttagta aaatgtatta attttggttt aaattaagat gatgggtatc ggtatggggg 720
gtggggaggt agttgaagat ttattgagtt ttgttttagg gtagttttgt ttatcgtttt 780
attttatttt ttattacggt gtttaagttt atattgagag agaaattttt agttgtaaaa 840
gggagaagag aaacgttgga atattagtat cggacgttag gatatggttg tggtgtttta 900
aaaattattt tattatttgg agtttgattt cgaggggagt atttttattt tttagttttt 960
tgaaagtatt tattagtatt tgaatattgt tttgagtttg ttggagtagt gaaatttggt 1020
gagagagaag ggtggaggaa ggaaggagtt gttgtatttg gcggttggat ttaggtagag 1080
gaaattgtta taatttcggg aaagaataga aaagtagaaa gggacgagtt tttatacgta 1140
gttaatgttt atggttttaa ttgtgtttgg gaaggaagat tttgggttag gggtgtattt 1200
tcgtttttta aaattaaacg tgtttgagat agttataaag tttattaagg gatttgagag 1260
attagagttt tttgtttttt ttttttaatt ttgagttttt tttttatttt tattgaggga 1320
gagtttgagt ttatgataag tgtcgcgttt atttttggtt aatttttaaa agaaagacgt 1380
tcgttttggt ttttttttta ggtttttagt ttttttaggg atggtagaaa tttttgggtt 1440
aaggttgagc gaattattgt ttattgtttt tattagtttt tagtaaaggt acgtcggcgg 1500
gggggcgttt agttttttta gtaaacgttt cgcggttttt ttcgtagatt acgaggtggg 1560
ggtcgttggg gagggtcgag ttgggggtag ttcgttattt cggtttttag cgagttgtcg 1620
gcgattttcg cggttttttg gtttaggttt cggtttttcg ggcgaggagc gggagggagg 1680
tcggggttta ggcgtcgcgg cgaattcgtt aacgtagcgt cgggtttcga attttaggtt 1740
tcgttttagg ttttcggtcg tttggttagt ttgtttgttt taattttaat tttttcgagg 1800
ttagttagag taggtttgtt ggtagtagta tttttttagt agttacgcga ttagttaatt 1860
tttcggcggc gttcggggag gcggcgcgtt cgggaacgag gggaggtggc ggaatcgcgt 1920
cggggttatt ttaaggtcgc gttcgttagt ttcggcgggg cggttttcgt cgtcgtaatt 1980
aatggatttt tttttttgtt taaatagatt cgtcgtgtta attatttttt ttttcgttag 2040
tttttttttt atcgttatat tgggttatta aaaaaagggg gttcgttttt tcggggtgtt 2100
tttttttttt ttttttgttt tcgtttgttt acggttttgc gatttcgacg tcggtaaggt 2160
ttggagagcg gttgggttcg cgggattcgc gggtttgtat tcgtttagat tcggacgggt 2220
tttgttattt ttttcgtttg tttggttttt tttttttttc gttttttcgt tcgttagttt 2280
atttgattag cggagattcg gcggtcgggt cggggttttt tcgtagtttt tgcgcgtttt 2340
tagagttcgg gtcgtggttc gtcggggttt gtgttttttg gtttcgaggg tagtcgttgg 2400
gttttcgaga ggggttcggg ttgcgtaggg gcgttttgtt ttgttcggtt ttgttttttt 2460

gagagtgcga gagaggcggt cgtgtagatt cgggagaaag atgttaaacg tgcgagtgtt 2520
taacgggagt tttagtttgg agcggatgga cgttaggtag gcggagtatt ttaagttttc 2580
ggtttgtagg aattttttcg gttcggtgga ttacgaagag ttaattcggg atttggagaa 2640
gtattgtaga gatatggaag aggcgagtta gcgtaagtgg aatttcgatt tttagaatta 2700
taaattttta gagggtaagt acgagtggta agaggtggag aagggtagtt tgttcgagtt 2760
ttattataga ttttcgcggt tttttaaagg tgtttgtaag gtgtcggcgt aggagagtta 2820
ggatgttagc gggagtcgtt cggcggcgtt tttaattggg gtttcggtta attttgagga 2880
tacgtatttg gtggatttaa agattgattc gtcggatagt tagacggggt tagcggagta 2940
atgcgtagga ataaggaagc gatttgtaat cgacggtaat gatttttttt taattataga 3000
atgtgtttgg ggtttcgttt tgtttgttgg agggtgttaa ttttagtttg tttttcggcg 3060
tattttgatt tagttttggg agagttaatt ttattggttt taggtgttta gtgttatttg 3120
gtttattgtt tgtttgtttg tgatttttaa gttagaaatt ggagatggta agattcgata 3180
atttttttaa tttaatatat cgcggttttt tttattagta atttttaggt atgtgataaa 3240
gttgggatgt ttattaacgg ttcgtttttt ggttagggaa agagttttgg ggcggagaat 3300
gtattttttg ttttttgaaa ataattttat tttgtgtttt taaaagttat tggggatgac 3360
ggatttagga ttgtgggtgg aggtagtggg ttttttattt tttgattatg gggttaattt 3420
ttgttagtta ttgttttttt taataaagat tgtgtgtttt ttttaaaaat tttttttgcg 3480
tttaga 3486

<210> 166
<211> 3486
<212> DNA
<213> Artificial Sequence

<220>
<223> chemically treated genomic DNA (Homo sapiens)

<400> 166
   tttaagcgta ggggaaattt ttaaaaagaa tatataattt ttattagaaa aaataatggt 60
   tggtagaagt tggttttata gttaggggat gaaaaattta ttatttttat ttataatttt 120
   ggattcgtta tttttagtgg tttttaaggg tataaaatga ggttgttttt aaaaaataga 180
   aagtgtattt ttcgttttag agtttttttt ttagttagga ggcggatcgt tgataaatat 240
   tttaattttg ttatatattt aggagttgtt agtgagaggg atcgcgatgt attaagttag 300
   ggaaattatc ggattttatt atttttagtt tttgatttaa aagttataaa tagataagta 360
   gtgggttagg tagtattgaa tatttaagat taataaagtt agttttttta aagttaaatt 420
   agaatacgtc gaaaagtaag ttaaggttaa tattttttag taggtaaagc ggggttttaa 480
   atatatttta tggttgggaa agggttatta tcgtcggttg taggtcgttt ttttattttt 540
   gcgtattgtt tcgttaattt cgtttggttg ttcgacggat tagtttttgg gtttattaaa 600
   tgcgtgtttt tagagttagt cggagtttta attaaaggcg tcgtcgggcg gttttcgttg 660
   atattttggt ttttttgcgt cggtattttg taggtatttt tggggggtcg cgggggtttg 720
   tagtagaatt cgggtaagtt gttttttttt attttttgtt attcgtattt gttttttagg 780
   ggtttgtgat tttgaaaatc gaaattttat ttgcgttggt tcgttttttt tatgtttttg 840
   tagtgttttt ttaagtttcg ggttaatttt tcgtggttta tcgggtcgaa gaggtttttg 900
   taggtcgagg gtttggggtg tttcgtttgt ttggcgttta ttcgttttag gttagggttt 960
   tcgttagata ttcgtacgtt tgatattttt ttttcgggtt tgtacgatcg tttttttcgt 1020
   atttttaaaa aaataaaatc gaataaaata aagcgttttt acgtagttcg aatttttttc 1080
   ggaagtttag cgattgtttt cggagttaaa agatatagat ttcgacgagt tacggttcga 1140
   gttttaggag cgcgtagggg ttgcggggaa gtttcggttc ggtcgtcgag ttttcgttga 1200
   ttaaatggat tggcgagcgg gagggcggag aggagagggg attaggtaag cggagagggt 1260
   ggtaaagttc gttcgagttt gggcgggtgt aagttcgcgg gtttcgcgaa tttagtcgtt 1320
   ttttaaattt tgtcggcgtc ggagtcgtag agtcgtgagt aagcggggat aggggagggg 1380
   gagaaaaata tttcgaaaag acgagttttt tttttttagt ggtttaatat ggcggtggaa 1440
   gggaggttga cgaagaagaa aatgattgat acggcgagtt tatttaaata gaggaggaga 1500
   tttattggtt gcggcggcgg gagtcgtttc gtcgaggttg gcgagcgcgg ttttaaggtg 1560
   gtttcggcgc ggtttcgtta ttttttttcg ttttcgagcg cgtcgttttt tcgagcgtcg 1620
   tcgggagatt ggttggtcgc gtgattgttg gaggggtatt gttgttaata aatttgtttt 1680
   ggttggtttc ggagaaatta aaattaagat aaataaatta gttaaacggt cgggaatttg 1740
   gggcggggtt tgaggttcgg ggttcggcgt tgcgttggcg ggttcgtcgc ggcgtttaag 1800
   tttcgatttt tttttcgttt ttcgttcggg aagtcgggat ttggattaga ggatcgcgaa 1860
   ggtcgtcggt agttcgttag gagtcggggt ggcgagttgt ttttagttcg gtttttttta 1920
   gcggttttta tttcgtggtt tgcgggggag gtcgcggagc gtttgttggg ggggttgggc 1980
   gttttttcgt cggcgtgttt ttgttggggg ttggtggagg tagtgggtaa tggttcgttt 2040
   agttttaatt tagaagtttt tgttattttt ggggaggttg ggggtttagg gaagaagtta 2100
   aagcgaacgt ttttttttta gaaattagtt aggagtagac gcggtattta ttatgaattt 2160
   aagttttttt ttaatgaaaa taagaaagga atttaagatt aaaaaaaaaa aataaaaaat 2220
   tttagttttt taagtttttt aataaatttt gtagttgttt tagatacgtt tagttttgaa 2280
   aaacgagggt atatttttgg tttaggattt ttttttttaa gtatagttaa ggttatggat 2340
   attggttgcg tgtgggaatt cgtttttttt tatttttttg ttttttttcg ggattgtagt 2400
   agtttttttt atttgagttt agtcgttaaa tataatagtt tttttttttt tttatttttt 2460
   ttttttatta gattttattg ttttaataaa tttagaataa tatttagatg ttagtgaatg 2520
   tttttagagg gttgaagggt gaaaatattt ttttcggggt taaattttag atgatgaaat 2580
   gatttttaaa atattataat tatgttttaa cgttcgatat tagtatttta gcgttttttt 2640
   tttttttttt gtagttggaa attttttttt tagtgtgggt ttgagtatcg tggtggaagg 2700
   taaagtagga cgatgagtag ggttgttttg agataaagtt tagtggattt ttaattgttt 2760
   ttttattttt atgttcggta tttattattt tggtttgagt taaagttaat gtattttatt 2820
   ggaaattaat ttttcgtttt aatattatat agtagtaaag tttttttaag tatttatgtt 2880
   atagatgagt ttgatgtgta gtttgttttt ttagttatat tttgtttatt ttgtgtgttt 2940
   attttatttt atacgtttta gaatgtgata gttggaaggg attttcggga ttttgtggtt 3000
   taattttttt ttttttatta cggaggaaat cgagatttag agaggttttt tgtttaggga 3060
   tatgtattcg gttggtatcg gagttaggat tagatgtaag ttttttaata tttaggttgg 3120
   tttatttttt gttttttttt tttttaatgg tttttattgg tttagggttt tgtttgagtt 3180
   tgattgtgtt gtagtttgta ggttttgttg ttttataagt gattgaaaat ggtttttttt 3240
   taggttttgg gttgtttgta gatgtttttt tgatgtggtt tgtggttatt gatatttaaa 3300
   tgtagttgtt ttgagtttgt ggtattatta tttattagtt taattaaaat tgaaggatag 3360
   gggttgatat ttagagaata gtaaaggggg tagtgtttag aattagtttt taagttagga 3420
   tttttatttt aattgataga aaagaattta tttaggatta agtaagaggt tttatttttt 3480
   ttagtt 3486

<210> 167
<211> 8666
<212> DNA
<213> Artificial Sequence

<220>
<223> chemically treated genomic DNA (Homo sapiens)

<400> 167

ttgtagggtt ttttttagtt taaagaaagt aattaggtgt ttttattgtg ttatgatttt 60
tatttttagt tgggtttttt agtttggttt taaagaataa aataaaaggt ttttttgttt 120
gagttttatt taatttattt tttttattta taagaggtat tttaaatttt tacgattttt 180
aaatttttta atcgattatt ttatttttat tttgtaaata agttaatttt tttatttata 240
gaaggaagtg ttttaatttt tttttcgttt tgattttttt ttttttttaa atttatgtat 300
tttttgtgat aaaatttata attatcgttg tattttgagt tttatttttt tattattttt 360
gagggatttt aagtttttaa aaatatttta ttttgtttgt gtatttaatt ttttttttat 420
ttttttttaa tttttttttt tttttatttg gtatttgttt ttttaggtat atgtgtgttt 480
aggttttttt tattttttat ttgttttatt ttatggtata gggttttgaa ttattataat 540
taagttatga aagagtagtt aacgtagtgt ttttattttt ttgttatttt attattttag 600
tttttttttt ggttttttga ggagtttttt ataggttggt ttttaggaat aagtttaaat 660
gaattatttt tagttttttt aaatttttat gtttttgtat atttttttat ttttgtttag 720
aatatttttt tttttttttt ttatttttaa atttttatat tattttttaa gattgggatt 780
agtttttagt attcggaagt ttttgtttat tagagataaa tgagaatgag tttggttatt 840
tttttatttt tttgtattat tttgtgtttt attttgtttt tttaagagta ttatatgttt 900
tatttaattt ttaaataatt gtttgaggta agtatagtta ttattttaat tatgtaaatg 960
agaaaataga ggtttagata tgttgagtaa ttttgataaa agttaaagaa ttaataagtg 1020
gaatagttga ggtttgaatt ttggtagttt gattgtagag atattatgtt tgatttattt 1080
ttttttgttt ttattttatg tttgttttta gtttttgagt ttgttgaatg aatgaatagg 1140
tggtagtttt tttttgttat aagattgatt agaattaaga taggtttaaa ttttacgtgt 1200
agaattttta aaattgtaaa ggtagtgtaa atttaaaaaa agaatggtat ttttaggaaa 1260
gaggaaaagt aagtgtgaga ataataataa taataattaa taaattttag taaatttagt 1320
aaatgtagta aatttttata ttaaaagttt ttggatatat attattatat tttatggtta 1380
tatgaaatat attataattt tattttgtat ataggaaatt tgagattggt ataaggagta 1440
tagagattta ggattttata tttttatttt tttaggattt tgtattttag gtcgatatgt 1500
atgagtaaat tgggagtata atgggttttt taatagaaaa attaggaaag tttttttatt 1560
attattaatt atttatataa tattttttta attttattat tatttatatt ttaagtttta 1620

gagtatatgt gtataatgtg taggtttgtt atatatgtat atatgtgtta tgttggtgtg 1680
ttgtatttat taatttatta tttagtatta ggtatatttt ttaatgttat tttttttttt 1740
ttttttttat ataagattta taatggataa tggattttaa tttttagagt aaaatggttt 1800
tatttaagga tgttataatt tttttagagt tttattgtaa gatatgagat atatatattt 1860
aaaatttgtt tttggtattt ttaaagtagt taatttttat atttgtttat aatgtattta 1920
aatgttgttt ttatatggtt gtatttttta ttttttttat taatagttat atatattttt 1980
tataagagtt gaaagagttt ttgatgtagg aatttatggt agagttttag agaaattttt 2040
gaatttattg aaagttttat ttagaaatat atgtgtaagt gaatatattt tttttaaaaa 2100
aaattattat ttattttttt ttttgagaag aaggtattta ttttaataga tttttgaagg 2160
agtttatttt ttttattttt ttatttttat taagaattat tgtaggtcgg gtacgatggt 2220
ttatgtttgt aattttagta ttttgggagg ttaaggtggg tggattattt gaggttagga 2280
gttcgagata agtttagtta atatagtgaa atttcgtttt tattaataat ataaaaatta 2340
gttgggtatg gtagtatgtg tttgtaattt tagttattcg ggaggttgag gtaggagaat 2400
tgttcgaatt cgggaggcgg aggttgtagt gaatcgagag agatcgtgcg gtgttatttt 2460
attttagttt gggtaataga gcgaaatttt attttaaaaa aatatataaa ataaataaat 2520
aaaaagaaag aattattgta ttagtgatgg aaatgtgttt tttttttttt attttggtaa 2580
ttattttttt ttttttttat tataaaatat tttaaattaa attaaaataa ttttatttat 2640
cgatagtttg aatttttttt attattgtta tatagttaat tgagaggtat ttcgaggaaa 2700
atataaatgg tatagtaatg tattgtagat tttaataata tatttgatat tttaaattgt 2760
ttttattggt tttattttaa aaattatatg ttttaaaatt aagtagatat taaaagtata 2820
agatatattg ggtttataag gtttaagtta attagggatt gaaatataat ttttaaatag 2880
agttggatta tttagtaggt agattaagta tgtgtttaag gtattagtaa agtttgagta 2940
atttattttt taaaacgtag tatatgtttt tgataagttt aaaaagtagt agttatagga 3000
aaaattagaa tttttatttt tttgcgtttg ttatattttt tagtgttgtt taattttttt 3060
ttgtaagtga gggtggtgga gggtgtttat aattttttta gggagtaagt tttttttggt 3120
tttttttttt tttttttttt tttttttttt tgagattaag tttcgttttt gttttttagg 3180
ttggagtgta atggcgcgat ttcggtttat tgtaattttc gttttttttt gggtttaagc 3240
gattttttta tattagtttt cgagtagttg ggattatagg tatgcgttat taagtttcgt 3300
taattttgta ttttttagta gagatagggt ttcgttatgt tggttaggtt tgtttcgaat 3360
ttttggtttt aggtgattcg tttgtttcgg ttttttagaa tgttgggatt atagacgtga 3420
gttatcgtat tcggattttt tttttatgta atagtgataa ttttatttaa agtatttttt 3480
tttttttttg agtcggagtt ttattttgtt atttaggttg gagggtggtg gcgcgatttc 3540
ggtttattgt aatttttgtt tttcgggttt aagcgatttt tttgttttag ttttttgagt 3600
agttggaatt atatacgtgc gttattatgg ttagttaatt tttgtatttt tagtagagac 3660
ggggtgttat tattttggtt aagttggttt cgaatttttg attttaggtg atttgttcgt 3720
ttcggttttt taaagtgttg ggattatagg tgtgagttat cgcgttttgt tttaaagtat 3780
ttttttttta tgttttaaaa taagattgta agttagtttt taaagcggat aatttaagag 3840
ttaataggta ttagtttagg atgtgtggta ttgtttttaa ggtttatatg tattaatata 3900
ttatttaaat ttataataat ttttataaag tagggggtat ttatattttt tttttttttt 3960
ataattacga aaaatgtaag gtatttttag taggaaagag aaatgtgaga agtgtgaagg 4020
agataggata gtatttgaag ttggtttttg gattattgtg taattttgtt tttagaatat 4080
tgagtatttt ttttggttta ggaattatga ttttgagaat ggagttcgtt tttttaatga 4140
tttttttttt atttttttat ttgtttatag gtagaatttt ttttcgttcg tattaaataa 4200
attttatttt tttagagttt gtttttatat taggtaatgt atacgtttga gaaatttttg 4260
ttttagatag tcgttttata cgtaggaggg gaaggggagg ggaaggagag agtagttcga 4320
ttttttaaaa ggaatttttt gaattagggt ttttgattta gtgaatttcg cgtttttgaa 4380
aattaagggt tgagggggta gggggatatt ttttagtcgt ataggtgatt tcgattttcg 4440
gtggggtttt tataattagg aaagaatagt tttgtttttt tttatgatta aaagaagaag 4500
ttatattttt tttatgatat taaatatttc gatttaattt ggtagttagg aaggttgtat 4560
cgcggaggaa ggaaacgggg cgggggcgga ttttttttta atagagtgaa cgtatttaaa 4620
tacgtttttg ttggtaggcg ggggagcgcg gttgggagta gggaggtcgg agggcggtgt 4680
ggggggtagg tggggaggag tttagttttt tttttttgtt aacgttggtt ttggcgaggg 4740
ttgttttcgg ttggtgtttt cgggggagat ttaatttggg gcgattttag gggtgttata 4800
ttcgttaagt gttcggagtt aatagtattt ttttcgagta ttcgtttacg gcgttttttt 4860
gtttggaaag atatcgcggt ttttttagag gatttgaggg atagggtcgg agggggtttt 4920
ttcgttagta tcggaggaag aaagaggagg ggttggttgg ttattagagg gtggggcgga 4980
tcgcgtgcgt tcggcggttg cggagagggg gagagtaggt agcgggcggc ggggagtagt 5040
atggagtcgg cggcggggag tagtatggag ttttcggttg attggttggt tacggtcgcg 5100
gttcggggtc gggtagagga ggtgcgggcg ttgttggagg cgggggcgtt gtttaacgta 5160
tcgaatagtt acggtcggag gtcgatttag gtgggtagag ggtttgtagc gggagtaggg 5220
gatggcgggc gattttggag gacgaagttt gtaggggaat tggaattagg tagcgtttcg 5280
atttttcgga aaaaggggag gtttttgggg gagtttttag aaggggtttg taattataga 5340
tttttttttg gcgacgtttt gggggtttgg gaagttaagg aagaggaatg aggagttacg 5400

cgcgtataga tttttcgaat gttgagaaga tttgaagggg ggaatatatt tgtattagat 5460
ggaagtatgt tttttattag atataaaatt tacgaacgtt tgggataaaa agggagtttt 5520
aaagaaatgt aagatgtgtt gggattattt agtttttaat ttatagatat ttggatggag 5580
tttatttttt ttattaggag ggattattag tggaaatttg tggtgtatgt tggaataaat 5640
atcgaatata aattttgatc gaaattattt agaagcggtc gggcgcggtg ttttacgttt 5700
tgtaattttt ttattttggg agattaaggc ggggggaatt atttgaggtc gggagttcga 5760
gattagtttg gttaataggt gaaatttcgt ttttattaaa aatataaaaa gtagtcgggg 5820
gtggtggtag gcgtttgtaa ttttagttat tcgggaggtt gaggtaggag aatcgtttga 5880
attcgggagg ttgaggttgt agtgaatagc gagatggagt tattttattt tagtttgggt 5940
gatagagtga gattttgtcg aaagaaagaa agagagaaag agagagagaa aaattattta 6000
gaagtaatta tatattgtgt ttatttttaa ttgagtaggg taaataaata tatgtttgtt 6060
gtaggaattt aggaaataat gagttatatt tatgtgatta ttttagaggt aatatgtagt 6120
tattattttg ggaatatttg ttaatatttt tgttttttta ttatttttag tttatttgat 6180
atagtttatt tgtgataaga gtttttaatt ttttattttt gaatagaggt gttttttttt 6240
tttttatttt tgttttgtga gggagttagg ggaggattta aaagtaatta atatatgggt 6300
aatttagtat ttttaaaatt ttgttaatag tttgaattcg ggagtttggt tttgtagttt 6360
tataatattt tagaagagat tttatttgtt taaaaataaa aaggaaaaag aaaagtggat 6420
agttttgata atttttaatg gagaagggag aagaatatgt agaaaagggg aaatgatgtt 6480
ggtttagaat tttaattata ttggtgttta atataggaat atttatttat ataatatttt 6540
aaagtattaa atttatatta gtatattatt aaatggatat attattaaat gggtttaagt 6600
attttatata ttttaattta attgatttat tttttttttg ttttggattt ttattatgat 6660
ttaaatattt atatatgggt tattttttag attttttata ttatgaaata taagaaaaat 6720
ttttaaggtt agttttatga ttaagacgaa ggattttatt gaatatataa aataataaat 6780
atattgtaat attttgtttt tttttttgta gttgtaattt ggtttgttta tatttttttt 6840
ttgttttttt gaaaattgag ttagttttat ttttttagga taggatttaa taattataat 6900
ataatttagt ataatttttt gatttaggta aattatgtaa tttgtgttta gtatgaaatg 6960
tatttaaaaa taagtaattt ttttttaata ttattatttt taaattaata taataaataa 7020
tagttatttt aaaataaatt gtttattttt attatgtagt atttaaattt taaggttgtt 7080
atgattgtag atagtatttt aaaatttttt tttggaaatg gttttgtttt taagatgatt 7140
taggaattaa agaggtgatt attttttgtt taatgaattt ttaaattata aatttgggaa 7200
gtgttttagt tttttattgt tgttgttata aattattata aatgtgttag ttaaaataaa 7260
tataaaatta ttattttata gttttagaga ttagaagtta aaaatgggtt tataaggttt 7320
tatttttttt ggaaatttta aggggtaatt tgtttttttg ttttttttag tttttagtga 7380
ttattaaatt ttttggttta tggtttttgt attttttttg tggtttgtgt ttttattttt 7440
gtattttttt tttgattgtg attttttaat aaaaatattt ggggttatgt tgggtttatt 7500
ttgaaaattt tggataattt tttttaagat tattaattaa attatatttg taaagttttt 7560
tttgttatat aagttaatgt attaaaagtt tttgaggatt aggatataga tattgggggt 7620
gggggggtat tatttagttt attataggaa ggaattttag ggttaattaa attagttttt 7680
ttattttata tttgaagaaa ttgaagtttt ggaattggag agtattatgt taaatgaaat 7740
aagttaaata tagaaagata aatattatat gtttttattt atttgtgaaa tataaaataa 7800
ttatattttt agtagtaaag agtagaatgg tggttattag agttgggggg tgggaggaat 7860
ggggagatgg taattaagat ataaagtttt agttaagatg ggaggaataa gtttgattgt 7920
tttttttgag atgtgtttta tagtatgatg aatatagtta aatagtaaat tttaaatgtt 7980
tttatttgat aaaaatgtta aatatttgag atgatggata ggttatttag tttgatttaa 8040
taatttttta ttgtgtttaa agattataat tttatattgt attatataaa tatatataat 8100
tgtattattt taatatataa ttttaaaatt aatataatga aaaagaaatt gaagtttaat 8160
atttttagaa gttaagtgta atttaaaagt tttgtgagaa tttgttttaa taaataaata 8220
agtttttttt ttttaataat tattatattt tgcgtttgga tatatagtag tgaataaaaa 8280
aaaaaaaaaa aaaaaaaatt tttaggttta atataatttt aggaagaaat tttagtagtt 8340
gtattttagg ggaaatatag gaagttagtt tggagtaaaa gttagtttgt ttttgttttt 8400
ttgttatttt gttcgtgttt tatagtgttt tttgtttgtg acgatagttt cgtagaagtt 8460
cggaggatat aatggaattt attgtgtatt gaagaatgga tagagaattt aagaaggaaa 8520
ttggaaattg gaagtaaatg taggggtaat tagatatttg gggtttgtgt gggggtttgt 8580
ttggcggtga gggggtttta tataagtttt tttttcgtta tgtcggtttt tattttggtt 8640
ttgattattt tgtttttttt ggtagg 8666

<210> 168
<211> 8666
<212> DNA
<213> Artificial Sequence

<220>
<223> chemically treated genomic DNA (Homo sapiens)

<400> 168

tttgttagag agaatagaat ggttagagtt agggtggggg tcggtatgac ggaaaggaag 60
tttgtgtaga gtttttttat cgttaagtag atttttatat aagttttagg tgtttaatta 120
tttttatatt tgtttttagt ttttaatttt ttttttgagt tttttattta ttttttagta 180
tataatgaat tttattatat ttttcgaatt tttgcggagt tgtcgttata ggtagagagt 240
attgtgaggt acgggtaaaa tagtaaaggg gtagggatag attgattttt attttaggtt 300
aattttttgt attttttttg agatataatt attgaaattt ttttttgaaa ttatgttagg 360
tttggagatt tttttttttt tttttttttt tgtttattgt tgtatattta agcgtagaat 420
gtggtaattg ttaaaaagag aaaatttgtt tgtttgttaa aataaatttt tataaaattt 480
ttaagttata tttagttttt gggaatgttg aattttaatt ttttttttat tatattagtt 540
ttaaaattat atattgggat agtatagttg tatatattta tgtggtataa tatgaagtta 600
tgatttttga atataatggg gaattattaa gttaagttaa gtaatttatt tattatttta 660
aatatttgat atttttgtta aatgagagta tttgggattt attatttagt tatatttatt 720
atgttatgaa atatatttta aaaaaaataa ttaaatttat tttttttatt ttaattgagg 780
ttttatattt tgattattat ttttttattt tttttatttt ttagttttag taattattat 840
tttatttttt attgttaaga atgtaattgt tttatatttt atagataagt gagaatatgt 900
gatatttgtt tttttgtgtt tggtttattt tatttagtat aatgtttttt aattttaaaa 960
ttttaatttt tttaagtata aaataagaag gttagtttaa ttaattttaa aatttttttt 1020
tgtggtaggt tgaataatgt ttttttattt ttaatgttta tgttttaatt tttaaaaatt 1080
tttaatatat taatttatgt ggtaaaagag gttttgtaga tgtgatttaa ttaatggttt 1140
tgagggagat tatttagaat ttttagggtg ggtttaatat aattttaagt gtttttatta 1200
gagggttata gttagagaga agatataaga atggaagtat aggttataga gaaaatatag 1260
agattatgag ttaaggaatt tgatggttat tagaagttgg aaaagataag gaaatagatt 1320
gttttttaga gtttttaaaa ggaatgaaat tttgtggatt tatttttgat ttttgatttt 1380
tagaattgta aaataataat tttgtgtttg ttttagttaa tatatttgtg ataatttgta 1440
atagtagtag taggaaatta aaatattttt taggtttatg atttgagagt ttattaaata 1500
agagatggtt atttttttgg tttttaaatt attttggaaa taaagttatt tttagagagg 1560
aattttaaaa tattgtttgt agttatagta attttaaaat ttgagtgttg tatggtggaa 1620
gtagataatt tattttagga taattgttat ttgttatatt agtttgagga tggtggtgtt 1680
aaagaggagt tatttatttt taggtatatt ttatattaaa tataaattgt ataatttgtt 1740
taaattaagg aattatatta aattatatta tggttattaa attttgtttt gagaaagtga 1800
aattgattta gtttttaaag agataaagag aaagtataag taaattaaat tgtagttata 1860
aaaagaaaga taaaatgttg tagtatattt attgttttgt gtatttaatg aagtttttcg 1920
ttttggttat aaaattagtt ttaaaggttt tttttatatt ttatagtatg aaaaatttaa 1980
aaagtaattt atatgtaaat atttaaatta tgatagaaat ttaaagtaaa aagaaaatga 2040
attaattgaa ttaaaatgtg taggatgttt aaatttattt gataatatat ttatttgata 2100
atatattaat atgaatttag tattttaaaa tgttatataa ataaatgttt ttatattaaa 2160
tattaatgta gttaggattt taagttaata ttattttttt ttttttatat gttttttttt 2220
tttttttatt aaaaattgtt aaaattattt attttttttt tttttttttg tttttaaata 2280
aataaggttt tttttaagat attgtaggat tataaagtta aattttcggg tttaagttgt 2340
tggtaaaatt ttagagatgt taagttattt atgtattaat tatttttaaa ttttttttta 2400
atttttttat aaaataggag tagggagagg agaaatattt ttgtttaaaa atgaggaatt 2460
gaaaattttt attataaata aattatatta agtaagttaa agatagtaaa agagtaaaaa 2520
tgttagtaga tatttttaaa atggtaatta tatattattt ttggaatgat tatatgaatg 2580
tggtttatta ttttttaagt ttttatagta aatatatatt tatttgtttt atttagttaa 2640
aaataaatat aatatgtagt tgtttttgaa taattttttt tttttttttt tttttttttt 2700
ttttttcgat aaagttttat tttgttattt aggttggagt gaagtggttt tatttcgttg 2760
tttattataa ttttagtttt tcgggtttaa gcgatttttt tgttttaatt tttcgagtag 2820
ttgggattat aggcgtttgt tattattttc ggttattttt tgtattttta gtagaggcga 2880
ggttttattt gttggttagg ttggtttcga attttcgatt ttaggtgatt tttttcgttt 2940
tgatttttta aagtgaaggg attataaggc gtgaggtatc gcgttcggtc gtttttgaat 3000
aatttcgatt aaaatttata ttcgatattt attttaatat atattataga tttttattga 3060
taattttttt tagtaagaaa gataagtttt atttaggtat ttgtgaattg gaggttaagt 3120
agttttagta tattttatat ttttttaaga tttttttttt attttaaacg ttcgtaaatt 3180
ttgtatttga taaagagtat atttttattt aatataaata tgtttttttt tttagatttt 3240
tttagtattc gagagatttg tacgcgcgtg gttttttatt tttttttttt ggttttttaa 3300
gtttttaggg cgtcgttagg aggaggtttg tgattataaa ttttttttga aaatttttta 3360
ggaagttttt ttttttttcg gagaatcgaa gcgttatttg attttaattt ttttgtaaat 3420
ttcgtttttt agagtcgttc gttatttttt gttttcgttg tagatttttt atttatttgg 3480
atcggttttc gatcgtaatt attcggtgcg ttgggtagcg ttttcgtttt tagtagcgtt 3540
cgtatttttt ttattcgatt tcgggtcgcg gtcgtggtta gttagttagt cgaaggtttt 3600

atgttgtttt tcgtcgtcgg ttttatgttg tttttcgtcg ttcgttgttt gttttttttt 3660
ttttcgtagt cgtcgagcgt acgcggttcg ttttattttt tggtgattag ttagtttttt 3720
tttttttttt tttcggtgtt ggcggaagag tttttttcga ttttgttttt taaatttttt 3780
ggagggatcg cggtattttt ttaggtaagg ggacgtcgtg agcgagtgtt cggaggaggt 3840
gttattaatt tcgagtattt agcgaatgtg gtatttttga agtcgtttta ggttgggttt 3900
ttttcggggg tattagtcgg aagtagtttt cgttagagtt agcgttggta aggaaggagg 3960
attgggtttt tttttatttg ttttttatat cgtttttcgg tttttttgtt tttagtcgcg 4020
ttttttcgtt tgttagtaaa ggcgtgtttg agtgcgttta ttttgttaaa aagaaattcg 4080
ttttcgtttc gttttttttt ttcgcgatat aattttttta attgttaaat tgaatcgggg 4140
tgtttggtgt tatagggaaa gtatggtttt tttttttaat tataagaaaa agtaaaatta 4200
ttttttttta gttgtgagag ttttatcgag aatcgaaatt atttgtacga ttagaaagtg 4260
tttttttatt tttttaattt ttgattttta ggagcgcggg gtttattaag ttagaaattt 4320
tagtttaaag gatttttttt ggagagtcgg attgtttttt tttttttttt tttttttttt 4380
tttgcgtgta aaacggttgt ttggggtaag ggttttttag acgtgtatat tgtttggtat 4440
aagagtagat tttgaaaaga tgaggtttat ttaatacgga cgggggagaa ttttgtttgt 4500
aggtagatag gaaaatgggg agggagttat tggaaggacg gattttattt ttaaagttat 4560
aatttttaga ttagaaaaag tgtttagtgt tttagaagta gagttgtata gtgatttaaa 4620
gattagtttt aaatattgtt ttgttttttt tatatttttt atattttttt tttttattga 4680
aaatattttg tatttttcgt aattataaag ggggaaggga atatgagtgt tttttgtttt 4740
ataggggttg ttgtgagttt aaatgatgta ttaatatata taagttttaa gaatagtgtt 4800
atatatttta agttaatatt tgttagtttt tgaattattc gttttgagga ttggtttgta 4860
attttgtttt gaggtataga aagaaaatgt tttggagtag gacgcggtgg tttatatttg 4920
taattttagt attttgggaa gtcgaggcgg gtagattatt tgaggttagg agttcgaggt 4980
tagtttggtt aaaatggtga tatttcgttt ttattaaaaa tataaaaatt agttggttat 5040
ggtggcgtac gtgtgtaatt ttagttattt aggaggttga ggtaggagaa tcgtttgaat 5100
tcgggaggta gaggttgtag taagtcgaga tcgcgttatt attttttagt ttgggtgata 5160
gaatgagatt tcgatttaaa aaaaaaaaaa aatgttttgg atagaattat tattattata 5220
taaaaggaaa gttcggatgc ggtggtttac gtttataatt ttagtatttt gggaggtcga 5280
gataggcgga ttatttgagg ttaggagttc gagataagtt tgattaatat ggcgaaattt 5340
tgtttttatt aaaaaatata aaattagcgg ggtttggtgg cgtatgtttg taattttagt 5400
tattcggagg ttgatgtagg agaatcgttt gaatttagga gaaggcggag gttgtagtga 5460
gtcgagatcg cgttattgta ttttagtttg ggagataaga gcgaaatttg gttttaagaa 5520
aaaaagaaag aaagaaagaa agaaagatta agaagaattt attttttgaa aagattatgg 5580
gtatttttta ttatttttat ttataaagaa aagttaaata gtattaaaga gtataataag 5640
cgtaaggagg taaaagtttt aatttttttt gtgattatta ttttttaagt ttattaaaaa 5700
tatgtattac gttttaaaaa atggattgtt tagattttgt tgatgtttta agtatatgtt 5760
taatttgttt attggataat ttagttttgt ttaaaagtta tattttaatt tttggttgat 5820
ttaaattttg tagatttagt atattttgta tttttagtgt ttgtttgatt ttaaaatatg 5880
tagtttttaa aatgaagtta atgaaaataa tttgggatgt taagtatgtt attaaaattt 5940
ataatgtatt attgtattat ttatattttt ttcggggtat tttttaatta gttgtgtagt 6000
aatgataggg aaaatttaaa ttatcgataa ataaaattat tttagtttag tttaagatat 6060
tttatgatgg aggaggaaga aagtggttgt taggatggga gggagggaat atatttttat 6120
tattaatata atggtttttt ttttttgttt gtttgttttg tgtgtttttt tgagatggag 6180
tttcgttttg ttgtttaggt tggagtgaaa tggtatcgta cgattttttt cggtttattg 6240
taattttcgt ttttcgggtt cgagtaattt ttttgtttta gtttttcgag tagttgggat 6300
tataggtata tgttgttata tttagttaat ttttgtatta ttagtagaga cggggtttta 6360
ttatgttggt taggtttgtt tcgaattttt gattttaggt gatttattta ttttagtttt 6420
ttaaagtgtt gggattatag gtatgagtta tcgtgttcgg tttatagtgg tttttaatgg 6480
gggtgggaga gtgggaagag taggtttttt taagagtttg ttgaaataaa tatttttttt 6540
ttaaaaaaga aagtaggtaa tgattttttt taaaaaagat gtgtttattt gtatatgtat 6600
ttttagataa aatttttagt gaatttaggg atttttttga aattttatta tggattttta 6660
tattaagaat ttttttagtt tttgtgaaaa atatatatag ttattggtga agaagatggg 6720
agatgtaatt atataaaaat aatatttgga tgtattataa ataggtgtaa aagttgattg 6780
ttttggaaat attaagaata agttttagat atgtatattt tatattttgt agtagagttt 6840
tggaaggatt atggtatttt tgggtggggt tattttgttt tagaaattga agtttattat 6900
ttattataaa ttttatgtag gggggagggg ggagggatag tattaggaga tatatttaat 6960
gttaaatgat gagttgatgg gtgtagtata ttaatatggt atatgtatat atatgtaata 7020
aatttgtata ttgtgtatat gtattttaaa atttaaagta taaataataa taaaattaaa 7080
aaaatattat gtaaataatt aataatagtg ggaaaatttt tttagttttt ttgttaaaga 7140
gtttattata tttttagttt atttatatat atcgatttga ggtgtaaaat tttagaagaa 7200
tgaaaatata aagttttgga tttttgtgtt ttttatgtta gttttagatt ttttatgtgt 7260
aaaatgggat tataatatat tttatgtggt tataaaatat gataatgtat gtttaaaagt 7320
ttttaatgta aaaatttatt atatttatta aatttattaa agtttgttgg ttattgttat 7380

tattattttt atatttattt tttttttttt ttgagaatgt tatttttttt ttagatttgt 7440
attgtttttg tagttttgaa aattttatac gtgaaattta aatttgtttt aattttgatt 7500
agttttataa taaaaaaaga ttattatttg tttatttatt taataagttt agaaattaag 7560
ggtagatatg gggtgggggt agaggggagt aggttaaata tagtattttt atagttagat 7620
tgttagggtt taaattttaa ttgttttatt tattggtttt ttaattttta ttaaagttat 7680
ttaatatgtt tgggtttttg tttttttatt tgtatgatta ggataataat tgtatttgtt 7740
ttaaatagtt gtttagggat taaatgaagt atgtaatgtt tttagaagag taaaataaag 7800
tatagaatga tataagaaaa tgaaaaggtg attaaattta tttttatttg tttttagtag 7860
gtaaaggttt tcggatgttg agagttgatt ttagttttga agaatgatgt gagagtttaa 7920
agatggaaaa gaaggagaaa gatattttag gtagaggtaa gaggatgtgt aaaggtatag 7980
aagtttagga aaattgaaag taatttattt agatttattt ttgaaaatta gtttgtgaag 8040
gattttttag agagttaaaa gaaaaattag gataatggga tggtaaggaa gtggggatat 8100
tgcgttgatt atttttttat agtttggttg tgatagttta aggttttatg ttatgaagtg 8160
aggtagatgg aaggtggagg agatttgagt atatatatat ttggaagggt aagtgttaag 8220
tgaagagaag ggaaagttag aaaagaataa aagaaaagtt aagtatatag gtaagatagg 8280
atatttttga ggatttgagg ttttttaaaa ataatgaaga aatagaattt aaagtatagc 8340
ggtggttata gattttgtta taagagatat atagatttgg gagggagaaa aggttagaac 8400
ggggaggaag ttgaggtatt tttttttatg aatgaggagg ttagtttgtt tgtagagtga 8460
aaataagatg gtcggttagg aagtttgaga atcgtaaagg tttggaatat tttttatgag 8520
tagagaggat gggttgagta agatttagat agagaagttt tttgttttgt tttttaaaat 8580
tagattggag aatttagttg agggtggaga ttatgatata gtagggatat ttgattattt 8640
tttttaaatt aaagaaagtt ttgtag 8666

<210> 169
<211> 2501
<212> DNA
<213> Artificial Sequence

<220>
<223> chemically treated genomic DNA (Homo sapiens)

<400> 169

gaaaattagg ttgagtaggt tggtttttta aagaaattta ttgagttttt ataatgtttt 60
ttttatttga cgggagaaaa ggggttttgg aagattaagt gtgaagtttt agaattttgt 120
ttggagttgg ttaggattat ttaggtttga gtataagtaa agcgttgttc gtggtgttag 180
taagcgcgaa tgttttcggt aacgtttttt ttatttaaag ttgtttttta tttagttaat 240
ttgtttcgcg aagtttatat atttgtttcg tgtaagattt ttagttattt tatttagtta 300
ttggtttttt ttttattatt attttttatg ttttttttaa agaaaaatat tattttttat 360
ttttttatta attttaaata gtttttattt ttttgatata tttattattt aagaaaatgg 420
tttaagaatt gggttagatt attttgtttt aatttttttg ataagtttta gagaaattta 480
aaggttaaaa taagagtata agagtaaaag gtagagaaat taagaaattg aagattagga 540
aatgggggtt ggatgggaaa gaaaaagaaa ttgttattaa tgttattcgg tttttttttt 600
tgtttaggtg gattttagtt ttgttgaggt tttgttagta gatttttagt tttaattagt 660
atttttatgg tggtggtatt tttattgttt ttaaaagaaa gagttttttt taattttata 720
gggatttggg ggatgaggag ttagagttaa ggtattttaa aaaaaatatg tgaagatttt 780
tattttgtaa tatataagta attgtttttt tgttaagatt ttgttttttt tagtatttcg 840
aattaaaatg attttgtaaa taaaaattgt ttatttttag gagaggttta tttatgtagt 900
tttttattaa agtttttagg taataaattt ataatttgcg gttttttttt tatttaatgt 960
agtattcgtt gaaatgtttt aaatatttta agtaataaat gttgatttaa atttatttag 1020
gaagattagg aaggggaaaa aaagtatttg gtatttaaat ttttagaaga gaatttaatg 1080
ataggtttag tttgtttaat gataagttta gtattatatt ttttttttat gatgttttat 1140
tattattgta taaatttttt ttattattta tgataaataa aaataagata tttgataaag 1200
tgggtttaaa taggtaagag tgtaaataaa gatttattgt ataaatatga tgaaattggg 1260
attttagatt tttaaagtat aatttttttt tgttttatgt gtgttaggtt gttattttta 1320
atttcgaatt agtttttttt ttttttaagg gttgtattta taatgtaaaa atggaaagaa 1380
ttaaaaagta tacgtaaaat atgattttcg ggattttttt ttatttttat ggttgattaa 1440
tttaaataga aagatatatt taagagaaaa ttgttaagtt tgatataagt tatgaaattt 1500
gtgaagttta agtattgttt ggggatgaat ttaatttgta tgataggtgt agagttgtcg 1560
tttttagata ttttaagaaa tacggagtta ttttgaatga ttttttttcg gttataaggg 1620
agttattaac gtttttatag tgaaattaat tggttggttg aaggaataga aattttttgt 1680
ttcgtttatt ggggattagg agttgagggt agtggtgaat atttttaaaa tattagtttt 1740
ggttttattg gatatttagc gagtagtgta gttagtattt ttggcggttt tttggtttag 1800

tttttggcgt atgcgtttta gtatttttgg gtaggttttt tcgttttcgt gacgcgtcgg 1860
ttcgggtttg gtttttcggc gattatagcg gatagggggc ggagtttaag ggggtgggga 1920
gacgtcggtt ttttggttta gttgaaaacg gaattttttg tcggttggtt ttttattttg 1980
ttagagcgag gcggggtagt gaggatttcg cgacgcgttc gtattttgcg gttagagcgg 2040
ttttgagttc ggttgcgttc gcgttaggcg ttttttttta gaagtaattt aggcgcgttc 2100
gttggttttt gagcgttagg aaaagttcgg agttaacgat cggtcgttcg gttattgtac 2160
ggggttttaa gtcgtagaag gacgacggga gggtaatgaa gttgagttta ggttttttag 2220
gaaggagaga gtgcgtcgga gtagcgtggg aaagaaggga agagtgtcgt taagtttacg 2280
gttaacggtg gattattcgg gtcgttgcgc gtttgggggt tgcggaatgc gcgaggagaa 2340
taagggtatg tttagtgggg gcggtagcga tgagggtttg gttagcgtcg cggcgcgggg 2400
attagtggag aaggtgcgat agtttttgga agtcggcgcg gattttaacg gagttaatcg 2460
tttcgggagg cgcgcgattt aggtagttgg ggttttaggg t 2501

<210> 170
<211> 2501
<212> DNA
<213> Artificial Sequence

<220>
<223> chemically treated genomic DNA (Homo sapiens)

<400> 170

gttttggggt tttagttatt tggatcgcgc gtttttcgaa acggttgatt tcgttgggat 60
tcgcgtcggt ttttaggagt tgtcgtattt tttttattag ttttcgcgtc gcggcgttgg 120
ttagattttt atcgttgtcg tttttattgg gtatgttttt gtttttttcg cgtatttcgt 180
agtttttaga cgcgtagcgg ttcggataat ttatcgttgg tcgtaaattt aacgatattt 240
tttttttttt ttttacgttg tttcggcgta tttttttttt tttaggagat ttgggtttag 300
ttttattatt ttttcgtcgt ttttttgcgg tttggggttt cgtgtagtgg tcgagcggtc 360
ggtcgttagt ttcgggtttt ttttggcgtt taagaattag cgggcgcgtt tggattgttt 420
ttgggaaaaa gcgtttagcg cggacgtagt cgagtttaaa gtcgttttgg tcgtagggtg 480
cggacgcgtc gcggagtttt tattgtttcg tttcgttttg gtagagtggg gagttagtcg 540
gtaaagaatt tcgtttttag ttgggttaag gggtcggcgt tttttttattt ttttaggttt 600
cgttttttgt tcgttgtgat cgtcgggagg ttaggttcgg gtcgacgcgt tacgagggcg 660
gggaagtttg tttaaagatg ttaggacgta tgcgttagag attgggttag ggagtcgtta 720
ggaatgttgg ttgtattgtt cgttggatgt ttagtaaagt taaggttaat attttgggaa 780
tgtttattat tgtttttagt ttttaatttt tagtaggcgg agtagaggat ttttgttttt 840
ttagttagtt agttggtttt attgtggaga cgttggtggt ttttttgtga tcgagagaaa 900
gttatttaaa ataatttcgt gttttttaag atgtttgaaa gcgatagttt tgtatttgtt 960
atataagtta aatttatttt taggtagtat ttgggtttta taagttttat aatttgtatt 1020
aaatttagta attttttttt ggatgtgttt ttttgtttga attagttaat tataaaaata 1080
gagaaaaatt tcgagaatta tgttttgcgt gtgtttttta atttttttta tttttgtatt 1140
atggatataa tttttaaaag agaaaaaaat tagttcgaga ttgagagtgg taatttggta 1200
tatataagat aaaaaaaaat tatattttaa gaatttgaga ttttagtttt attatatttg 1260
tatagtaaat ttttgtttgt atttttattt atttaaattt attttgttag gtattttatt 1320
tttatttatt atgagtaata aaggaaattt atgtagtaat aatgaaatat tataagagag 1380
gggtgtggtg ttgggtttgt tattaaatag gttgaatttg ttattaaatt tttttttgaa 1440
gatttaaatg ttaagtgttt tttttttttt ttttaatttt tttaggtgag tttgaattaa 1500
tatttattat ttaaaatatt taaaatattt tagcggatgt tatattggat aggaagagaa 1560
tcgtaagtta tggatttgtt gtttaaaaat tttggtgagg aattgtataa gtggattttt 1620
tttaaaagtg aatagttttt gtttatagaa ttattttggt tcggagtgtt gaggaagata 1680
aagttttaat aggagggtaa ttgtttgtgt attgtaaaat gagagttttt atatgttttt 1740
tttaggatat tttagttttg attttttatt ttttaaattt ttgtagaatt aaaaaaagtt 1800
ttttttttta aaggtagtgg aagtgttatt attatggaag tgttggttag ggttgaaaat 1860
ttattgatag agttttaata gagttgaaat ttatttggat agggaaggga atcgggtagt 1920
attaataata attttttttt tttttttatt taatttttat tttttagttt ttagtttttt 1980
aattttttta ttttttattt ttatgttttt gttttgattt ttgagttttt ttgaaattta 2040
ttagaaaagt taggataaga tagtttgatt taatttttga gttatttttt taggtagtaa 2100
atatgttaga aaaatgaaag ttgtttggag ttgataagga aatggaagat aatgtttttt 2160
tttgaggggg atataaagaa tggtgatagg gaaagaatta atgattaagt aaaatgattg 2220
agaattttgt acgaggtaga tgtgtgagtt tcgcgaagta agttgattga atgaaaaata 2280
attttgggta gggaaaacgt tgtcgggggt attcgcgttt attagtatta cggatagcgt 2340
tttgtttata tttaggtttg ggtggtttta attagtttta gatagaattt taaagtttta 2400

tatttgattt tttaaagttt tttttttttc gttaaataga aaggatatta tggaagttta 2460
atgaattttt ttgaaaaatt aatttgttta atttggtttt t 2501

<210> 171
<211> 2501
<212> DNA
<213> Artificial Sequence

<220>
<223> chemically treated genomic DNA (Homo sapiens)

<400> 171

gtgttttagt ttttgaattt atgttgttcg ttattatatt gtattgtttg tggatgattt 60
atttgttttt gtagttttat ttgaagaatt tttttttttt tttttttgag gtttgtttta 120
aatattattt tttttgtgaa gattgtttgg tgttttttag gagagagtgt gatttttttt 180
tttaggaatg gtagtatttt aaatatatat attttgtagt atattttatt ttgtatggta 240
atggtttgtt tgtgagtttt atttcgtatt agagggtgag ggttttgaag atagcgttag 300
gttttattta tttcgatatt ttataatatt tattatatgt ttgatgaatg aatgtatagg 360
gggatggttg ggtgtatttt ttttaatttt ttaatttttt gaaataaata ataaaatttt 420
attttttttt agaagttttg gtatggtttt ttttattttg ttatagttat tgataattat 480
ttttattttt tgtgtaattt attaggtaag aagtttatgt agatttattt ttagtagttt 540
atatgataaa taaatattgc gtttgatttt taaatattaa attatagtat attatagata 600
gatatagagt tattatttaa agtatgatat tttaatttta aaaggttttt tttgaagaat 660
attataaatt tttttttttt ttaagatttg ttgggtagga aagatgggag aaaatgaatt 720
aatgtttata tagaaaggag gataatgggg gtaaaaataa tagatgaacg tatgggtgga 780
tgagagaatg gataaaatga taggtggata tgttgatttt ggatagatgg gaaatgagtg 840
gatatattaa taaatagata tgtgggtgga tgggtggaga agaggatggt ggatggttgt 900
ggttttatga agagatgtga aaaaggaagt gtggaatgat ggatgagaag ttgtatggga 960
agatgaatag aagaataggt ggttgaataa attaaaaggt gtgtggttgg atgaatgaat 1020
gagtgggatg atagatggat ttaagtggtt agtggatgga taggaggatg gatggatgtg 1080
agagttttag aaggatataa ggaaagatgg gtggatagat ggatgggcgg atggaaggat 1140
atttaggagg atgaatgagt atgtgtgtgg agagaggtgt ttatttatat tggtttgaat 1200
atatgggtta gttgagttaa atgttagttt tatgataggt tattagtagt tttttttgag 1260
ttgttttgtt aagaagttaa aatttattta agttatgtgg atttgttatt gaggggaaaa 1320
agaatgagtt tttttttttt ttatttggaa gatttattaa ttttttattt tttatttttt 1380
attgtgggta cggaggtatt gcgttattta gggtaagatt tcgttttttt tttagttttt 1440
tttttaggat atttaatatt ttgtgaaatt tagagatttt gttttagtcg gatttagaga 1500
aatttagcgg gaaaggagag gttaaaggtt gaatttaatg gtgtaaggtt ttacggttcg 1560
gttatttttt gttttgacgt cgcggggtta gcgggagaag aaagttagtg cgtttttggg 1620
cgtaggggtt agtggggttc ggaggtatag gtatttcgcg atattttagg tttttcgatt 1680
tacgtttttg gtagtttcga ttatttatag ttttagtaga gtacggggcg ggggtagagg 1740
ggttcgttcg ggagggttgt tattttttaa aatttttgcg ggttgtttag ttatagtttt 1800
ttttgtttgg gtgtgttttt cgttcgtttt tttttttcgt tttaggttat tgtttttaat 1860
ttcgaataaa aattgtagtt aattttcgag gtagttttat tgtttagcgg atttagtttt 1920
ttgttaggtt cggttcgtta ttttcgtttc gtttttcgtc ggtttttgtt tcgcgtttag 1980
ggatttttta gtttttttcg ttcgcgtttt tcgttcgttt cggatattat ggataagttt 2040
tggtggtacg tagtttgggg attttgtttc gtgtcgttga gtttggcgta gatcggtgag 2100
tgttcgtcgt agtttgggta gtaagatggg tgcggggtgt ttagcgcgga ttcggcggta 2160
gttttttcgg ttgagtcggt tttgggggat tggagttaag tgagttgttt gcgaagtgta 2220
ttgggtttcg gaaagtaggg ttgggatttg cgttaaatcg ttggagaatg tgtttgtgga 2280
agtattattt ggttgaaaga aaaagagaaa gagaagaaag tttgttgggt aggttgtcgg 2340
cgcgtagttt tgggcgaggt cgttagagtt gtagtatatg gtagaaagta atcgtttttt 2400
cggatgcgta tagtcgttgt ttggattaat aggtttttgt gtttaagggt tcgttaagtt 2460
ttatcgggtt gtgtttaggt agggtagagt tgggcggggt a 2501

<210> 172
<211> 2501
<212> DNA
<213> Artificial Sequence

<220>
<223> chemically treated genomic DNA (Homo sapiens)

<400> 172
   tgtttcgttt agttttgttt tgtttagata tagttcggtg gggtttggcg agtttttggg 60
   tataggagtt tgttagttta gataacgatt gtgcgtattc gggagaacgg ttattttttg 120
   ttatgtgttg tagttttagc gatttcgttt aaaattgcgc gtcggtagtt tgtttaataa 180
   attttttttt tttttttttt tttttttaat taaatggtgt ttttatagat atatttttta 240
   acggtttagc gtaaatttta gttttgtttt tcggagttta atgtatttcg tagatagttt 300
   atttgatttt agttttttag ggtcgattta gtcggagggg ttgtcgtcgg gttcgcgttg 360
   agtatttcgt atttattttg ttgtttaggt tgcggcgggt atttatcgat ttgcgttagg 420
   tttagcggta cgaggtagag tttttaggtt gcgtgttatt aaaatttgtt tatggtgttc 480
   ggagcgaacg gagggcgcgg gcgaaaggag ttggaggatt tttgggcgcg gggtaggggt 540
   cggcggagga cgggacgagg atggcggatc gaatttggta gaggttgggg ttcgttgggt 600
   aatgaggttg tttcggaagt tggttgtagt ttttattcga ggttgaaaat agtgatttaa 660
   gacggaggga gggagcgagc gaaggatata tttaagtaag gggggttgtg attaagtagt 720
   tcgtagaggt tttaagaagt agtagttttt tcgggcgggt tttttgtttt tcgtttcgtg 780
   ttttgttgag gttgtaaata atcggggttg ttagggacgt gggtcgggga atttggagtg 840
   tcgcggggtg tttgtgtttt cgagttttat tggtttttgc gtttagagac gtattggttt 900
   ttttttttcg ttggtttcgc ggcgttagga tagaggatga tcgaatcgta aaattttgta 960
   ttattgggtt tagtttttgg tttttttttt ttcgttaaat ttttttgaat tcggttggag 1020
   taagattttt gggttttata ggatgttgga tattttggga gaggagttgg agagagggcg 1080
   aggttttgtt ttgggtggcg tagtgttttc gtgtttatag tggggagtga ggggtgggga 1140
   gttggtgaat tttttaagtg gaaagaggga gagtttattt tttttttttt taataataag 1200
   tttatatggt ttgaatgaat tttagttttt tggtagaata gtttagggaa agttattgat 1260
   ggtttgttat agggttggta tttggtttag ttaatttatg tgtttaagtt agtgtgaatg 1320
   ggtatttttt tttatatata tgtttattta tttttttaaa tattttttta ttcgtttatt 1380
   tatttattta tttatttttt tttatgtttt tttggggttt ttatatttat ttattttttt 1440
   gtttatttat taattattta ggtttattta ttattttatt tatttattta tttaattata 1500
   tattttttaa tttatttaat tatttatttt tttatttatt tttttatata attttttatt 1560
   tattatttta tatttttttt tttatatttt tttataaaat tataattatt tattattttt 1620
   tttttatttt atttatttat atatttgttt attgatatat ttatttattt tttatttgtt 1680
   taagattaat atatttattt attattttat ttattttttt atttatttat acgtttattt 1740
   attatttttg tttttattat tttttttttt gtgtaaatat taatttattt ttttttattt 1800
   tttttattta gtagatttta aagagaggaa gagtttgtaa tattttttag gggaagtttt 1860
   ttgagattga aatattatat tttgaatgat aattttatat ttatttataa tatattatgg 1920
   tttaatgttt ggaaattaaa cgtagtattt atttgttatg tgaattatta aaagtaaatt 1980
   tgtatggatt ttttgtttgg tgagttatat agaaaatgaa agtgattatt agtggttgtg 2040
   gtaagatgaa aggaattatg ttaggatttt tgagggaagg taagattttg ttatttattt 2100
   taagaaattg aaaagttgag agaaatgtat ttagttattt ttttatgtat ttatttatta 2160
   gatatgtaat gagtgttgtg gggtatcgag gtaaataaga tttggcgtta tttttagagt 2220
   ttttattttt tggtacggaa taaaatttat aagtaggtta ttattatgta aggtgaaata 2280
   tgttgtaaaa tgtgtgtgtt tggggtgtta ttatttttag agaagggagt tatatttttt 2340
   tttggagaat attaggtagt ttttataggg gaagtgatat ttgaggtagg ttttaaagga 2400
   aaaggaggag gaatttttta gatgaaatta tagggataag taagttattt ataggtagta 2460
   tagtgtagtg acgaatagta tggatttagg agttagaata t 2501

<210> 173
<211> 16579
<212> DNA
<213> Artificial Sequence

<220>
<223> chemically treated genomic DNA (Homo sapiens)

<400> 173

tgttttttaa aatgatgttt agttgaatta gttaatggag ataaaaatat ttatatataa 60
ttttttttta agtttattag ttattttata gattagttat aattttggtt attttaatga 120
gttaattttt ttttagagat attataaatt ttttaaagat ggaaaataat tgttattttt 180
ttgaataatg tttattattt aaaataatgt atataaaagt tgtttaataa atttttgttg 240
aattgagaga aaatataaaa aattgaaagt agtttgtttg taataagtgt ttgttaagtt 300
ttatagattt tttttttttt ttggaattat tgtaataaat atttgatttt tgatttgtag 360
gattaaaatg tgatttttta tattttaata ggagtaagta ttagttttgt aatttttttt 420

gttaatagaa taaaaatgga atgtatgttt gttgttttta tatgtcggtt gatatttatg 480
ttgaatattt tttttataac gtagaggttt ttttgttttt tgttttgcgt tttatttttg 540
tttattatag agatattaat gatgttatgt gtttttttgt attattagtg tagtagtttt 600
tattatgttt tttgtttcgg ttttattgta gtttaattaa ataaatggaa acgggtagtt 660
attgaaaata aatagtaatt gtaaaagttt tgttttttaa gaaatttttt gtattgtatt 720
tcgttttgtt ttattgattt taagattttt atcgtttttt tgtgtttaga aattagattt 780
tttagtttgt tttgttttag ttagataaat gttttataaa aatgatttat attttttaga 840
ttgtggggag agaagaaaaa gattttgaga aagtgattaa ggagggagta agagtgatat 900
aatttttgat ttaatttagt tggattgtgt tttttatgtt tttatttttt tttttataaa 960
aaagatagcg tttttgatgt tttgttttag gtagtttttt agggtgagtg tagatgtgtt 1020
gagttatttg gttgttttga ggatcggttt agagagtttt tttttttatt aatgaagtta 1080
ttttttttcg gtagttcggt tttttttatt attttatgtt ggaatttggt tttggtttgt 1140
atagttttgt cgtgatgtag atggtttttt agttaagttt tagagaaatt tgtttaatta 1200
tgaataggat atttatagta taggagaaaa aaaaatttat taaaataaat attttattgt 1260
aatattttga tttgggggtt ggagagtaaa aatttaatat ttgatgtttg ggaaaaattt 1320
aatgttttat ggttttttaa ggaaatttgt ttgtgtttaa tttattagag taatttatat 1380
atttcgggtt aaagtataat gagattgtta ttattatttt gttttttgta agattttttt 1440
tttaagaata aaaagaaggt aataaagatt ttatattttt gtttttttta gaaagttaaa 1500
gagaatttaa agtgttggat gaaatgtttg tttttaattt gtagtgtttt atttaaaaaa 1560
aaaattatga tataatttta gaggataggt tttttttttt tagggttttt gaaagttaaa 1620
agtatatttt taatttgtag tattattgta ttatttagtg aggataattt gtttagttgt 1680
gtttaggatt agttagatta aggagttgaa attaagtttt ttttgaattt gaatgggatt 1740
ggaattttgt tatttttaga gtagtaatag atgatgttat gtttaagaat attttatttg 1800
attaataaaa aaggagaagt ttgaaatgta attttaaaga attttagaag gaggtttaga 1860
tgaagataat aaaatttttt ggagatatat ttgttattta tatttttttt ttttaagtta 1920
ttggtttgtt gtttttgagt ttagttaaat ttttaaagat aagattaaag taaggaagta 1980
aggtatatag tttttggttt atggtttttt ttagatataa gtattgtgaa tgaagatttt 2040
gttttattta tatttgggat tttggagttt ttataaagaa tatttttagt ataaggatgt 2100
gatttaaagg gggatttata gaataatatt ttaattattg tttgatttat atttgagtgt 2160
aatggataaa taaatataat tttagttata aaatttgaaa aatatatatt tttttattta 2220
aaaatttttg atagttttgt taggtattgg agaattagaa ataaatgttt ttttattttt 2280
ttttttttga tgaaggaaag ttattaaaaa tagttatata gaaataataa tataattgta 2340
gatttatttt taaatatagt tggtatgttt tttttttttt tttttattta tttttttattt 2400
tagatagtta gaggggaaag tttgttttag agatagaaag aagatattta tggggtataa 2460
ttttatttta gttatttgat agtttgaaga agtatttaat tttttgaata ataatgttat 2520
aagttaaaga agagaaaaga taggtagttt aaagtttttt aatattgtgt atattttaat 2580
tttttaagtt agttttgaga aagtgttttt gttttgtaga agtatttggt taagtgttgt 2640
gaagtttatt ggtattaaaa gaaattaaaa attttggagg attagagaat taaatttggt 2700
aatataaatg aaaatatttt agaatgtaaa aagtatttaa tttattattt ttaattatgt 2760
tttattatat atagatggta ttatttatag tgaatgttaa ttattaatat ttttttaaaa 2820
atgggtatta atttgttatt agttgtttgt ttttgattta gttttttttt gttgaaaggt 2880
tttatttaat tgtattataa agggataaaa gggtaaaaga taataagttt tttagattgg 2940
tagatttgtt atttataaaa tggttttggg taggttattt gaattttttg aaatttaatt 3000
agatttattt aaatttaaga atgagaatat aaatttatat tttgaagtgt tttatagaaa 3060
ggtttatttt aatgtttgga gtatatattt taatgaatat ttattttatt ttattttttt 3120
ttatttttga attaagtaat tttgaattta aagttgttat gattagtatt gaaaagatta 3180
ttggattatt aattgtgtga ttttgggata gtaatttttt gtattttagt ttgtttatat 3240
gttatatatg aaggttgaag tttgattttg ttttgtgatt attattttaa atatttgatg 3300
aaattaaatt ttagtgtttg gaatggtagt ataataaatt tattaagaat aaataattta 3360
ttgtaaaaat atattgattt ttaaatgatg taattgatag ttatattatt gtagagggtt 3420
gataaataat aaaagaaatg aaagatgtat atggtgagaa ttgaaattat tttgataagt 3480
tttttatttg tttattattt aaaattaatg attatgttga atgtttataa attataaaat 3540
ataaaagaaa ttttataaat gcgtatgtat aggagtttaa gttattaaaa gttttaaagt 3600
ataagtttaa attaaattaa ttaaagaagt tgagaggaaa aattggtttt tatttttaat 3660
tattattgtt ttgaggtttt atgtttaata taatttttta agtagaggtt ttagagagaa 3720
gagttgtgag gatattttta tatttgtgta gaaggaaaag tttgttattt attttagtat 3780
ttttagtgtt atattgatgt gtattttgga tttattttgt ttttattgta taaatttata 3840
tttgatttta aagaaaagga aaatttaaag tttttttttt ttaaggggat agaaattttt 3900
tgtgttaatt gtttgatttt tttttttgta aggttttatt ggaaattttt tgtaatataa 3960
tgtaggggat ttttttatgt gttgatgttg tttatatagt ggggtgggtt tgattgaaga 4020
aaaaaaatcg tatatacgta tgaaagatta tggttttatt ttcggaaagt atgaaaggtg 4080
attgatattt ttaagaagtt tttgttattt aggaaaatta ttaaatattt tatttagaga 4140
tatttggaaa gattgaagga aaggaagaac gaagaaagta gaatttagat ttatgtgggg 4200

agagatttgt ggtagaggaa aagtattttt tttgaattcg ataagggatt tgtttggggg 4260
aattttttgt ttagtttttt attattaggg ttttttgaag tcgggttttt tattgggtag 4320
ttttttggga gtgtagtggg gaatttttat attttttttt taggttttcg aaggatttcg 4380
tttttttagt gtttttttta ggttggtagg agttttgagt ttgatatttt tttttgatgg 4440
gataggtaag ttttgtgggc gcgtaaatac gttgtaatta agttttttgt tgattttata 4500
gttttgtgtg ttttcgagaa gaagtgatcg tatttaattg tttattgttg gtttgttttt 4560
taagagtttg gggttttttt ttttttaatt tagaattagt tgtacggggg gcggggaagt 4620
gggggtgggg aaggagtggg agggtagtgg ttttcgcgag tagagcgatg ttattgagtg 4680
agtttttgaa tggggagcgt tgttgttttt aagtcgattg gtattttttg ttaggaagaa 4740
acgttaagag gtgggagtgt ttggggaggg aggtaggcgg tttttatcgt aggcgcgggg 4800
agttgttttt tcgttttttc gtttgttttt taagtttgga tttttaggag tggttgaagt 4860
tgcggagcgt ttttggagtt tgtgaatgaa tttttttttt tttttttttt ttttttttcg 4920
ttgagttttt ttttcggttt tgacggtata gtgatataat gatgatgggt gttataattc 4980
gtatttgaat ttgtaggcga gttgtttcga gtttttttgg ggaagaattt taggcgtgcg 5040
gacgtaatag tcgagaatat taggtgttgt ggataggagt tgggattaag attttcggtt 5100
agtttcgtat tttttcgtat tttttagtat cgtttcgtat ttttcgtatt ttttttcggg 5160
ttattacgtt ttttatgtga ttcgtttggg taacgtcgaa tttagtcgcg tagcgttgta 5220
gtgaattttt tttttaaatt gtaataagtc gttttttaag gtaattacgt tttttttgtt 5280
ttttttttaa aaaataaaaa taaaaaattt atagaaaaaa attcgcgagt ttagaaaaaa 5340
gaagtaattg gtagaaggtt ttaattaagg taaagagttg taaggcgaag ttaagaaaat 5400
gtaggtattt aaaaaatgta ggtaattttt ataaaggttt ttggggagag gtatatagag 5460
ggattttggt gttgaaaaag atttagataa aagaaattta gggtggggtg ggggtaaaat 5520
gattaacgga attgggggaa gggagggaat aaattgtaaa gaaattatag aaaagtggtg 5580
ggtttttgag ttggagagaa gagagggatt tttggtattt tgattttttt gttgttgttg 5640
tttttaatac gttcgaggta aaagtttgaa tggggattat taagatttgt tatagataag 5700
ttttcgaagt cgttttggtg taggttattt ggttttttag tttttggtgt gtggttagtg 5760
tttggtgttt ttggaaagtt attttcgggt agtttttgat agtgcgattc ggcgtttaag 5820
tagtttggga ttttgcgcgg atttgatttt tttagatcgt aggtagtttg ggaggaggtt 5880
cggtcggggg aggtgtagga ttttcgtcgt gtttttttga cgatttgggg attgttacgg 5940
ttttttttcg gcgtttttgg gtttttttgt tttgtacgcg gtgcgaaggg gttagtaggg 6000
aaggagtaga ggatgggggg tggggttgtt ggagtttcgc ggaggtttgg gaggtttttg 6060
ggcgggaaaa gtttgttttg aatcggtagg gatgtgtaat aatttttttt attttgaaga 6120
gtgaaatagg gtttgtcgtt tttattttaa taagtaaatc ggtatttaga gcgtattgta 6180
gataaaggtg gttcggggat tcgaattagg ggtttttggg ttagtttttt cgtttagatt 6240
ataggagttt ttcgtttttt tatatatttt ttattttttt ttagtttttc gttttagttg 6300
agtaatttta ttggtaggtt tagcggtagc ggcgcgcgcg tgtgtcggga gtttcggggg 6360
acgttttcgg ttggagcgtt ttatcgtttt gtagaggcgt gggcgttgta gggcgatatt 6420
tttggtgcgt gtagatttag ggtattcggg ttgttttggt tcgcggtttg tgttattggg 6480
cgtggagcgg tttgggtgtt aggtagagga gagcggggta gaaaaatagt gttatagttt 6540
aaattatttg ttttttaatt taattgcgtt tcggtaagtt ttgtatgttt ttgggagatg 6600
ttgcgggaag ggggagaaat ttatacggcg tttggagagt tgttttgtcg cgcgtatatt 6660
cgcggtagag tttttttggg tcgcgttttt tatttttgtg tttttttttg tttttatttt 6720
ttttgttttt tttttttttt tttagttttt gttttttttt tttagttttt gcgttttttt 6780
ttttatagtt gataaatgaa tggttagtgt gaaatttttg tttttttcgt gttttaaggt 6840
agtagggagg gaggagcgag ggaggcggtg cgttttttcg gatttgtttt tagtttagtt 6900
tataagattt gtagaattta gatgtttagg aattgggagt tttgcggcgg gtgtgggcgt 6960
tttttgatgg agaagtttcg tataggcgga gaaaaataag ttttttagat taagcgtgta 7020
ttttttataa ttttgtgttt aaggatggaa ggttttagtt tttttttaag ttatttattt 7080
tgtattttat aatttgcggc gtatatttag gagtttggag gattttgaaa aaaggttttg 7140
gtttgtgtaa agtgtagaga tgttttttcg cgagtcggtt ggaacgtacg cggcgttttg 7200
ggtttagttc gtcgtttagt ggacgaacgt atatggtcgg ggtggagtta ggtttcggag 7260
gtttagacgc gttattttgg gcgtttttaa gaaagataat atatatatcg tgtttttaaa 7320
atttatgatt atttgaattt aacggtaggg tttcgtttta ggatattgta aaagaagggt 7380
ttttaattta aaatttaaat ttttttaatt ttaggcgggg cgtcggacgg gaaagtggag 7440
agaaggcggg tagtgggagg aaaaaagaaa gggaaggaag ggaagtggaa ggaaagagga 7500
tcgtggaggg gaggaaaagg agaaaggaac ggagatgggg tagatggtat ttaaatagtt 7560
aggaacggat cggaagggtt cgtttcgggg tttgggtttg aatttcgaga ttcgttttag 7620
gatatttttt tttttttttt agcggtaata aatgtgattg tagaggcgtg tcgggatgga 7680
agggtgggaa agaattagat aagcggaggt ggtttagttt cgtttaaagg ggttgggttt 7740
ggggttcgtt tttattcgtt tatttcgcgt taagggagga ttcgtaacgg gcgtttttcg 7800
tagcggttgg gttagttagt cgttacgtgg gttggcgttt cggtttttag tcgagacggg 7860
tgtgattttt tgggtttaga gattgtagta tagtcgtata ggcggtcggc gtagtcgagg 7920
tttttattta tcgaggttaa tgggggcggg gcggggaagg tagggtttag tttaatcgag 7980

agagtcgagt ggtttcgttt tcgggattcg gggtcggttg tttttggagt tgattcgagt 8040
tttatttaga tagcgttgaa tatttttatt aaaaggttat ttaagtttta ttttatttta 8100
ttttattttt gtttgttttt tgggagtggt aggaggggta tcgtgttttt gttttgaggt 8160
aggggagagg ttaaaattta ttttaagtaa attttttgtt agaaaaatga gattttatat 8220
cgtattttat ttgttgattt tttaaatata tttaggttat tttaaagttt atttttagtt 8280
aattaataga ttaatttcgt tttagattgt tataaagagt tcgtttggaa tggtaaatat 8340
tgttattcga taagattaat agaattttat gggggtaaat atggaagggt tgcgagtgta 8400
attgaggttt ttgtgtttta atgaggaatt ttagcgggga tttggcgggg attggaagtt 8460
tagtatttat ttagggagtg attgagttgg aaatttggag aagtttggtg tagacgagac 8520
ggtgtttttt agagttgtat tttaagtttt tttattttgt tgtatattgg aagaattgga 8580
ttttaaagat tttttttatt attttagatt ttgtagttaa tagttacgtt tttttttttt 8640
tgtttgttga aagttgtgat ttaattgttt taagtatttt ttgataattt cgttaaattt 8700
attttgaaaa tatttatata aagttttaaa ataggatgtt gtgagtgtaa tataaaaata 8760
ggaaggtatt aatggaagta ataattagtg ttttgagata gaagcggttt ttagtttgta 8820
atttggaaat gttaagagta gtatattagg gagaaggttt ttaagagttt gattttttgt 8880
tttgagtttt gagtaaagaa agaaatatgt tagaattgta ttgtttgttc gtagtaatat 8940
tgtaaagggt tttaatggat tttaatgtga ttgagtaatt atttttttta gtattaagtt 9000
tatagttttt aatttttttg tgagaagatt aggtaggaag cgggatgaga tttaagataa 9060
ttatttagga tttatgttag aatttaaagt tttgattttt ttggtagata aaaatagagt 9120
tggttttaaa ttgtattttt tgtttttaaa aaaacgaggt ttgaaaggaa tttttttaga 9180
tttatatagg tttatagatg taagtttttt tttttttttt gtgtttgtaa attggaggga 9240
gatgtttttg atattttaga atatttgtat ttatatattt gtaaattttg ttaggaaagg 9300
atttaaatag ttaaattaaa ggtgattatg attaatgtta aagatattat ttattgtttt 9360
tttttttgtt gtattattag gtttaaatat tttggagtta atgattggtt atttaattga 9420
gtaatagagt ttttgagagc gtaaagatta attaattttg ttttttaaag tttggtaatt 9480
aagttttttt tttttggtga gatttaaata tttttgttga cgttatttat ggataaatat 9540
cggtaaatat tgtatttaaa atgggatatt gttttgtgat ggagtagaat tttttaagat 9600
atatggtttt tttgaaagaa agaattaatg attgtagtaa ggtatttttt tattatttac 9660
gtagagaaat tagttaggat tgataaatat ttattgttgg gagttatttg ttttggtttg 9720
agaaaatatg ttgattgtta tataggtttg tattttatta gaaaatataa agtatgtatt 9780
ttttttgatt tggaattaga aaagggggat tagatatgga tgttttggtt aagttttatt 9840
ttaattagtg aatttttgtt agtttttttt tgtagtttag atttggtaaa ttatatatat 9900
ataaagtttg tgtgtgtgtg tggggtgcgt gtgtgtgtgt atgtatatag ggtttttaaa 9960
tattgttaag tttttattat tgaatgtgtt agaggtggtt gtattttaga ggttagtata 10020
aagaattttt gtttaatgtg ttagttttgt tttttgaaat atatttttgg gtagatttat 10080
taattataaa aggatataaa tgtatatatt ttttatattt gtatatattt tattttattt 10140
ttttaaggat tttaagaggt gtttatgttt ataaaatgag attatatatt ttataataaa 10200
tatatttgat aaattataat ttttttatta ttaaattatt gatttgatat tatttgaggt 10260
atagtggaat gagtattaga tttggaatta gaaagtttga gttggattta ttagttgaga 10320
aaattaagta aagatattat ttttggattt ttagtgttat aatttattaa atgaatatgt 10380
agttataaag attaaaagag taagtatttt gaaaaattat ataaaaaagg gatgataata 10440
tattatgttt tgggatttat attgatttaa ttttataatt ttaataataa ttgtattatt 10500
taagttatgt agtttttatt gaatttataa aataaattag agaggggtat attgttttaa 10560
aaattgttta gttttattaa ttagaagata gattatagaa attgatttta atgaagttgg 10620
atatttagtg gaaaggaaga attatttaat aaatggtttt aggataattg atgggtttat 10680
gtaaaataag aattagtttt tatttgattt tttattttaa aagaaatata agatgaatta 10740
aatatgaaat gtagggaata attttttata aaatgataaa agaaaattat gggattaaaa 10800
aaaataagtt aggaatagag aaatttgttt taagtaaaat ataaaattta gtgttatttt 10860
aggaaatatt gataggtttg attaagtaaa aattatataa aagtaaataa tatatttgga 10920
aaatatttac gatatttgag agataaaaag ttaataattt ttgatatttt atggattttt 10980
atgaataatt ttgaaaagtt tatatttatt aaaatatgag taaaaattat gtataggtag 11040
tggtagaaag agatatagag ttttaaaatt atagaaaaaa tcgtttagtt ttatttgtaa 11100
ttaaataaat agtaattaaa atattaatga tatgttatat tattttttta ttaattagag 11160
gagtaataaa aaaaaataat tttttttttt tttttttttt ttgagatgga gtttgttatt 11220
taggttggag tgtagtggta cgattttagt ttattgtaat tttcgttttt taggtttaag 11280
taattttttt gtgttagttt ttttagtagt tgggattatg ggtatttatt attatgtttg 11340
gttaattttc gtgtttttag tagagtgggg ttttattatg ttggttatgg tagttgattt 11400
tgaatttttg attttaagtg atttatttat tttagttttt taaagtgttg ggattatagg 11460
tatgagttat tgtgtttggt aaaaatgaaa ttaaaaagtt tgatgtaagt ttgtgttggt 11520
taggttgtgg agaaattggg ttttttatat attgttgtat gaaagtaaat aggttttttt 11580
attttaaaaa ataatttggt aattattttt ttttgatttt ttattaaaag tttttaaaag 11640
atgtatatat tttttgattt agtaatttta tttttcgata ttattatatg gatggatatt 11700
ggtattaata ttatttaaga taaatggata aggatgtttt ttgtagtatt gtttttaaga 11760

gtaaatattt ggaaatagtg taataggttt attataggga attatttaaa taaaagttgt 11820
tgtatttatt taatggaaaa ttatgtagtt gttaaaaata atatggaaaa attgtatttg 11880
ttgatttgga atatttttta aaatatattg tattttaagt gaaaaaattg atagataaaa 11940
ttgagtatat attatatatt taattatgta aaatgggtgt atatttattt ttttaataaa 12000
tatttattga gtgttttagg tattgtggat ataggagtga ataaaatatt aaagattgtt 12060
ttgttggaat ttttattatg gtaggtagag aaaggtaatt tttaattata tatgtaaaat 12120
ggtgatatgt gttaggatga aaatagagta tgggaaaggg aatagagtat agtatgtttt 12180
aggtagagta atgagaaagg agtttattag gtagagaata gaggagtgtt aaggggcgat 12240
aatgtgaaaa agtttagtta agtttaggtt taggaattta tatgatgtta ttatttgtat 12300
gaagtaaatt tatgaaattt cgtatttaaa ttacgtgatt tttttttgtt gtataatgat 12360
tttttttgtg agaaagaatt agtgagtaat atgatatgtg atgtttatta tatttatttt 12420
gtagttgaag tattttagaa agttggttgt ttagggtatt taattagtag aatggtggat 12480
aaaattatcg tgtttttgtt tttaagttat tttttgttag ttgagttttt ttaaaattaa 12540
gggagaatat taaatattta tttaatagaa ttttggtttt ttttcggagg ttgtttattt 12600
ttggtgtgtt ttttattttt tttgtagata ttttttatgg ataggagttt tattgatttt 12660
gttttttatg ttttttaggt aattaaatag ttttagtatt ttgtatttgg attgtttaat 12720
gatttttttt tttttttatt gattttattg gagtagaagg ttaaatatag gtatttattt 12780
ttatattttt taggtttttt tatgtagata gagttgttat tattagtaga cggtgtttat 12840
aagagagata tggaaaagtg gaagaatgga aatagggatt tggagtggtg ttaaaaataa 12900
agaaagaggt ttttgaaagt tttttattta atatgattaa atatagaaga taaaagatgt 12960
aatttaatga ttaaggatag ggatatagtt ttgagaaaat tatagaagtg gaatgaacgt 13020
tttggatttt aaagaagagg gaatggaaat gtagttttga ggtagttaaa gttaataaag 13080
ttttttgata tatttattgt agaaataaat gattattttt taatttaagg tttgtaaatt 13140
taggtgatta taggggcgag gtatggagtg gaaatgggta agagattaag tgggggtagt 13200
aatggttttg agtatataga tttaagggga gagattgttg tttggttaat gtttatgatt 13260
agttagttga aatatagatt tagtgttgtt agattttttg attttttgaa agaagttaga 13320
gattttatat ttagtgtaaa tagtttaatt tttaaattga gtaatttatt taaatatatt 13380
tatgtaattt atttaaatgt atttagagta tgtttgattt tttttttgta tttttattta 13440
gttttagggg taattttaag gaaagtgatg cgtatatttg ttgtatttat gtagatggag 13500
gattattttt ttttttttaa taaaagatat ttttataagg attgagtgtg gtataaagag 13560
ttcgattttt agtatttaaa ttgagtagga aatttaaata tagaagattt ttttttgggt 13620
tatatttttg gtttttttaa atgagatttg attttttaga tattgataaa tattatatta 13680
ttgggttatg gtaaattttt aagtggattg atttaaaagt gtttaaaagt attttaggtg 13740
ttaaggaatt gttggtacgg agtttaaaag gtgtttttgt ataagaaata gaggcgttga 13800
ttatgtattt gatttataaa tattttttaa ggatatattt tgaatttggt aatgaagaaa 13860
tattgtggga attattaagt tgaataaagt attagttttg ttttttagaa gtttattgtt 13920
ttatagagga gattagatat gtttatagat aatttgaagt atatagtaat aaattatagg 13980
atgatttatt ttaagtataa agtgtatatt gttattatag tggtaattta ggttataatt 14040
tttaaatgta agagttttaa atattatttt ttttattttt agtgatatga aattatgtta 14100
gaatatttgt agaaagtttg aattagtaat tttaataata aatattgagt attgatatat 14160
tgtatattat tgagatttat tttatatata ttattttatt aatttttatg attatttcgg 14220
taagtattaa tttttataat taagtagtga tagaaaatga ggtttagaga gatatagtat 14280
tttatttaaa gttatagaat ataagtagag aatgtagaat ttgtattttg ggttatttga 14340
tagtggagtt tagattttta aaattaggtt tataaagatt ttttggaaaa aaagaaaaat 14400
aaaaaataga gaataaattt ttttgaagaa aagtaggaaa ttattattta taagtataat 14460
taaagagaga tttttaattt tttttttttt tttttttttg agacggagtt tcgttttgtc 14520
gtttaggcgt aattttagtt tattgtaatt tttatttttc gggtttatgt tataaagaaa 14580
gtttttattt gattggttcg atttgattgt ttattagaat ataggttttt tagtagggtt 14640
aataagttat aatagtttta gtgatataag ataaatataa attaataata aggttaattt 14700
agaattatta atatatgttt tttattgtga attattagtt ggtttataga tggaaaatat 14760
aagatatata atgtattagt ttaaaaaatg ttttttagag agtgtttaga attatagaat 14820
ttaaaaattt tgttttgaaa aggtttaatt tttattttaa aatatataaa gaaattaaat 14880
gtaaaaaaat taagagaaaa ggttttaatt aattaaatgt tgtataattt taagttttat 14940
ttatgtttaa gattagtttt gtaatttatg attaatttta gtattagttt gtttttaatt 15000
taggtttgtt aaataatata gaattttttt tttaatgaag ttttttaggg ttttagttga 15060
aaaatatata ttagttagga tgtttttaat tgtaaggaat aaaaagttac gattttattg 15120
ggtttaaata ataatgatat ttataatttt atataaaatg aagtttaaag gtatggttta 15180
ttttaggttt aatttttaag gatttacgtt ttgtttttgt tgattttttt ttagttttat 15240
ttttagaggt attttattta tttatggatg taaaattttt ataatagttt tagtagaaag 15300
attttagtat gataatgttt agggaagaaa attgattatt tttttttttg tttttttttt 15360
aggatgaatt tatttttttt aggatttttt tattggagga agagattttt ttatatttta 15420
ttggttaaaa ttatattata tattgtttta aatttaatta ttggaaaggg gaatggtatt 15480
tttataattg atttaggata ttaggattat tttttgggtt ggggtttgat ttaggatttt 15540

ttaaagtatg tgattgaggg taggtattag aatggaatta gagttgtgtt agaaaggaga 15600
aacgtatggt tattatagaa tagttttaaa tgttatcgag gagggtataa ttgtaaaaat 15660
taaataattt tttgttattt ttttgaagtt ggtattttga tttttagatg gttttttaat 15720
ataggttttt ttttttttta ttattatagt tgttttgaaa tttgagttgg aagggaatat 15780
tttgagattt agattgttaa atgttttttt taaagttatg taataaatta aatggtaaag 15840
ttagggtagt tttttgattt agtatagggt attttttttt atttttattt tttgagattt 15900
tagaattgtt ggtattgttt taaaatttat ggtaagaatt ggttattttt gtaattaata 15960
tttttttata atatatttgt tttgtttgtt tagttagtta gaaattatat ggagtttgtg 16020
ttttaaaaag tttgtcgaag tttttatttt ttgttttggt attatgtgta tgaattatta 16080
attggttttt ttttatttta tatttgatga agatgttttt tttttaatat ttttttttat 16140
tgttttttat ttttttttgt tttatttatg attagttgtt tgtttttaaa tagattttgt 16200
ggttatttat ttttttttgt gttagttttt atttattagt tatttgaatt gtggttttta 16260
ttgtttttta tatagtttat ttatttgtgg ttgttatata tatttatatt gttatatgtt 16320
tttaaattgt attttggaat gattttggta atggttgtat tggatgagat ttaaattaat 16380
aattaaagta ttgagatagt ttttgttatt ataagtttat ttttgttttt atagtttaag 16440
aggagttatt ttttttattt ttattattta atgtttaata ttatttttat tatatataat 16500
gtataaaaag tatgtgattt atgatttatt ttaaatttga atgtttgtga tttattttgt 16560
gttttttttt atttttagg 16579

<210> 174
<211> 16579
<212> DNA
<213> Artificial Sequence

<220>
<223> chemically treated genomic DNA (Homo sapiens)

<400> 174

tttagaaatg aagaaaaata taaagtaaat tataagtatt taggtttaaa gtgggttata 60
aattatatgt tttttgtgta ttgtatgtaa taagggtagt attaagtatt aagtaataag 120
agtagaagaa gtagtttttt ttggattata aaaataggag taaatttgta atagtagaag 180
ttattttaat gttttaatta ttagtttgaa ttttatttaa tgtagttatt attaaaatta 240
ttttagaata tagtttggga atatataata atgtggatgt gtgtgataat tataagtgag 300
taggttgtat gaagaataat agagattata gtttaagtaa ttagtggata ggagttggta 360
taaaaagaaa tgggtgatta taaagtttat ttagaaatag atagttgatt ataaatagag 420
taagagaaga tggggagtaa tagagaaaga tgttgaaaga aaggtatttt tattaagtgt 480
aaggtgagaa ggaattaatt ggtggtttat gtatataata ttaaaataga gaataaggat 540
ttcggtaggt tttttaaagt atagatttta tgtagttttt agttggttaa gtaaataaaa 600
taaatgtatt ataaggaggt gttaattata aaagtgatta gtttttgtta tgaattttaa 660
agtagtatta atagttttaa agttttaaga gtaaagaatg aaagaaaata ttttgtattg 720
agttaagaaa ttgttttggt tttgttattt aatttattgt atgattttgg aaaagatatt 780
taataatttg ggttttagaa tgtttttttt tagtttaaat tttaaggtag ttataatgat 840
gaggaggaga agaatttgtg ttggggaatt atttagaaat taaagtgtta attttaaaag 900
aatggtaaaa gattatttgg tttttatagt tgtgtttttt tcggtagtat ttagaattat 960
tttataatag ttatgcgttt ttttttttta atataatttt ggttttattt tagtgtttat 1020
ttttagttat atattttgga gaattttggg ttaaatttta gtttaaggga tgattttgat 1080
attttaagtt aattatagag gtattatttt tttttttaat gattggattt ggaatagtat 1140
ataatatagt tttggttagt gagatgtgag ggattttttt tttttagtga agggtttttg 1200
ggaaagatag atttattttg agaagagaat aaaggaagag gtggttagtt tttttttttg 1260
gatattatta tgttggaatt tttttgttgg aattgttgta gggattttgt atttatgagt 1320
aagtgagata tttttggaaa tgaagttgag aaagaattag taaaggtaag gcgtaagttt 1380
ttaaggatta gatttagagt ggattatgtt tttgaatttt attttatatg agattataaa 1440
tgttattatt gtttaaattt aataaaatcg tgattttttg ttttttatag ttgaaagtat 1500
tttaattggt atatattttt tagttgggat tttgaaaaat tttattggaa aagaaatttt 1560
atattattta gtaaatttag attgaaaata agttaatgtt gaaattagtt ataaattata 1620
aaattgattt tgaatataaa tagggtttgg agttgtatag tatttaatta attaagattt 1680
ttttttttaa tttttttgta tttgattttt ttatatattt tgaaataaga attagatttt 1740
tttaaaataa aatttttaaa ttttataatt ttagatattt tttgaagagt attttttaag 1800
ttaatatatt atatatttta tattttttat ttatagatta attgataatt tataatgaaa 1860
aatatatgtt gataatttta aattaatttt attattagtt tgtatttatt ttatattatt 1920
agaattgtta tgatttgtta attttattgg aaagtttatg ttttagtaga taattaggtc 1980
ggattaatta aatagaaatt ttttttatgg tatgaattcg ggaggtggag gttgtagtga 2040

gttgagattg cgtttgggcg atagagcgag atttcgtttt aaaaaaaaaa aaaaaaaaga 2100
aattaaagat ttttttttaa ttatatttat aaataataat tttttatttt tttttaggaa 2160
aatttgtttt ttatttttta tttttttttt tttttaggaa atttttataa gtttaatttt 2220
aaaagtttgg gttttattgt taaatagttt agaatgtaag ttttgtattt tttatttata 2280
ttttgtgatt ttgggtaaaa tattatattt ttttaaattt tattttttgt tattatttag 2340
ttgtgaaaat tagtatttat cgaagtgatt ataggaattg ataaaataat gtatataaaa 2400
taaattttag tgatatataa tatattagta tttaatattt attattaaaa ttattaattt 2460
agattttttg tagatgtttt aatatagttt tatattatta agagtaaaaa gaatagtatt 2520
tggggttttt atatttgaaa attataattt aggttattat tatagtagta atatatattt 2580
tatgtttgga ataaattatt ttataattta ttgttatgta ttttaaatta tttgtaaata 2640
tgtttaattt tttttgtaga atagtaggtt tttggaaggt agagttagta ttttatttaa 2700
tttaataatt tttataatat ttttttattg ttaggtttag ggtatatttt tgaggaatgt 2760
ttgtagatta aatatatgat taacgttttt attttttatg tagaaatatt tttgaaattt 2820
cgtgttaata attttttaat atttgaaata tttttgaata tttttgaatt agtttatttg 2880
aagatttatt atgatttaat aatatgatgt ttgttaatat ttagagagtt aaattttatt 2940
taaaaaaatt aaaggtgtga tttaggaaag gattttttgt atttgagttt tttatttaat 3000
ttaaatatta aaaatcgaat tttttgtgtt atatttaatt tttataaaag tgttttttgt 3060
tggggagagg agaatggttt tttatttata taggtataat aagtatacgt attatttttt 3120
ttaagattat ttttaagatt aggtaagaat ataaggagga gattagatat gttttaaata 3180
tatttgaata agttgtataa atatgtttga ataagttatt taatttaaaa attagattgt 3240
ttatattaaa tataaaattt ttaatttttt ttaaaaaatt agaaggttta gtaatattaa 3300
gtttatattt tagttggttg attatgagta ttaattaagt agtagttttt ttttttaagt 3360
ttgtgtgttt aaggttatta ttgtttttat ttagtttttt atttattttt attttatgtt 3420
tcgtttttgt agttatttaa gtttgtaaat tttgaattaa aagatgatta tttattttta 3480
tagtaaatat attagagggt tttattgatt ttagttgttt tagaattgta tttttatttt 3540
tttttttttg gagtttaaga cgtttatttt atttttgtaa tttttttaag gttatgtttt 3600
tatttttggt tattaagtta tattttttgt tttttatgtt tgattatatt aaatggaaag 3660
tttttagaag tttttttttt tgtttttagt attattttaa atttttattt ttattttttt 3720
atttttttat gtttttttta taagtatcgt ttattagtga taataatttt gtttgtatgg 3780
aaagatttga gagatatgag agtgaatgtt tatatttagt tttttatttt agtgaagtta 3840
atgagaaggg aagaaagtta ttgaataatt taggtatagg gtattgagat tgtttaattg 3900
tttgggagat ataaaagata aggttaatgg aatttttatt tatgagaaat gtttgtaggg 3960
aggataaggg atatattagg gatgggtagt tttcgagaga aagttaagat tttattaaat 4020
agatatttag tatttttttt tgattttggg gaagtttagt tgataagggg tgatttaagg 4080
atagagatac ggtggttttg tttattattt tgttaattag gtattttaga taattaattt 4140
tttaggatat tttagttata aagtggatgt aatgagtatt atatattata ttgtttattg 4200
gttttttttt atagaaagaa ttattgtata gtagagagaa attacgtgat ttaggtgcga 4260
aattttatga atttatttta tataagtaat agtattatgt gaatttttga atttggattt 4320
ggttaagttt ttttatattg tcgtttttta gtattttttt gttttttgtt tggtgaattt 4380
tttttttatt attttattta agatatattg tattttattt ttttttttat gttttatttt 4440
tattttagta tatattatta ttttgtatat gtgattggga attgtttttt tttatttatt 4500
atagtaaaag ttttaataga gtagtttttg atgttttgtt tatttttgta tttataatgt 4560
ttagaatatt tagtaaatat ttgttaaaag aatgaatata tatttatttt atataattag 4620
atgtataata tatatttagt tttatttatt aattttttta tttagaatat aatatatttt 4680
ggagaatgtt ttaaattagt aaatataatt tttttatgtt atttttgata gttgtatagt 4740
tttttattag gtggatatag taatttttat ttaaatggtt ttttatgatg agtttgttat 4800
attgttttta gatatttgtt tttagaagta atgttgtaaa gaatattttt gtttatttgt 4860
tttgggtgat attggtatta atatttattt atataataat gtcgagaagt ggaattgttg 4920
aattaaagag tatatatatt ttttaaaaat ttttgataaa gaattaaaaa aaagtgattg 4980
ttaaattatt ttttgaagta gaggaattta tttattttta tataatagta tatgagagat 5040
ttagtttttt tataatttgg ttaatataaa tttgtattaa attttttaat tttatttttg 5100
ttaggtatag tggtttatgt ttgtaatttt agtattttgg gaggttgagg tgggtgaatt 5160
atttgaggtt aggagtttaa gattaattgt tatggttaat atggtgaaat tttattttat 5220
taaaaatacg aaaattagtt aagtatggtg gtgggtgttt ataattttag ttattaggga 5280
ggttgatata ggagaattgt ttgaatttgg aaggcggagg ttgtagtgag ttgagatcgt 5340
gttattgtat tttaatttga gtaatagatt ttattttaaa aaaaaaaaaa aagaaaaaaa 5400
ttattttttt ttgttatttt tttgattggt gaaaaaatgg tgtagtatat tattggtgtt 5460
ttaattatta tttatttaat tatagatgag gttgagcgat tttttttata attttaaaat 5520
tttatgtttt tttttgttat tatttgtata taatttttgt ttatattttg atagatgtgg 5580
attttttaaa gttatttata aaagtttatg gaatattaag agttattagt tttttgtttt 5640
ttaaatgtcg tagatgtttt ttaaatatat tatttgtttt tgtgtaattt ttatttagtt 5700
agatttatta gtatttttta gaatggtatt gggttttatg ttttgtttag aataggtttt 5760
tttattttta atttattttt tttaatttta tgattttttt ttattatttt gtaaaaagtt 5820

attttttata ttttatattt gatttatttt atattttttt taggatgaga aattaggtag 5880
agattgattt ttattttgta tggatttatt aattgttttg aaattattta ttgaataatt 5940
tttttttttt attgaatgtt taattttatt ggagttaatt tttgtaattt gttttttgat 6000
taatgaaatt aaataatttt taaaataata tgtttttttt tgatttattt tatagattta 6060
gtagaaatta tatggtttgg gtaatgtagt tattattaaa attatagaat tagattaata 6120
tgaattttaa gatataatat attattattt tttttttgta tagtttttta aagtatttat 6180
ttttttgatt tttataattg tatatttatt taatagatta tgatattaaa ggtttaaaag 6240
taatattttt gtttggtttt tttagttagt gagtttaatt taaatttttt gattttaagt 6300
ttagtgttta ttttattata ttttaagtaa tattaaatta ataatttaat aataaaaaga 6360
ttataattta ttaaatatgt ttattgtaaa atatgtaatt ttattttata gatataaata 6420
ttttttaaaa tttttagaag aatgaggtag gatgtatata gatgtggaaa gtatatatat 6480
ttgtattttt ttatagttgg taaatttatt tagaggtgtg ttttaggaaa tagaattgat 6540
atattaaata aagatttttt gtattgattt ttgaaatata attattttta atatatttaa 6600
tggtaagaat ttagtagtgt ttaaaaattt tgtatatata tatatatata cgtattttat 6660
atatatatat agattttata tatatgtaat ttattaagtt taggttataa gaggaaattg 6720
gtaaaaattt attaattgaa atggagtttg gttaagatat ttatatttaa tttttttttt 6780
ttgattttaa attagggaga gtatatgttt tgtgtttttt ggtaaaatat agatttatgt 6840
aataattagt atattttttt agattagagt aagtaatttt taataatgag tgtttgttag 6900
ttttaattgg tttttttacg tgggtggtga aaaaatattt tattatagtt attaattttt 6960
ttttttaaaa gaattatgtg ttttgagaag ttttgtttta ttatagggta atattttatt 7020
ttaaatatag tgtttatcga tgtttgttta tgaatgacgt taataaaaat gtttaaattt 7080
tattaggaaa ggagggttta gttgttagat tttgggagat agagttagtt gatttttgcg 7140
tttttaaagg ttttgttatt taattaagtg attaattatt aattttaaag tatttaagtt 7200
taataatata ataaaagaga gaataatggg tagtattttt aatattaatt atggttattt 7260
ttggtttagt tgtttaggtt tttttttagt agaatttata agtgtataga tataggtatt 7320
ttggaatatt aaaggtattt ttttttagtt tgtaaatata gggaaaaaga agaaatttat 7380
atttgtaaat ttatgtaaat ttagggggat tttttttaaa tttcgttttt ttgaaagtag 7440
gaaatataat ttaaaattaa ttttgttttt atttattaaa agaattaaaa ttttgaattt 7500
tgatataaat tttaaatgat tgttttgaat tttatttcgt tttttattta atttttttat 7560
aagaagatta aggattgtgg atttggtgtt ggaggaagta attgtttaat tatattggaa 7620
tttattggaa ttttttataa tattattgcg ggtagataat ataattttaa tatatttttt 7680
tttttattta gagtttagaa taagaagtta gatttttgga gatttttttt ttgatgtatt 7740
gtttttaata tttttaagtt ataaattggg aatcgttttt attttagaat attaattgtt 7800
gtttttattg gtattttttt atttttatat tatatttata atattttatt ttaggatttt 7860
atgtgaatat ttttaggatg ggtttggcga aattgttaag aagtgtttaa aataattagg 7920
ttatagtttt tagtagataa aaagaaaaag acgtggttgt tagttataga atttgaggtg 7980
atggaaaaga tttttaaaat ttagtttttt taatatgtag taaaatggga ggatttaaaa 8040
tgtagtttta agaagtatcg tttcgtttgt attaagtttt tttaggtttt tagtttagtt 8100
attttttaaa taggtgttga gtttttagtt ttcgttaggt tttcgttaag gttttttatt 8160
gaggtatagg aattttagtt atattcgtag tttttttatg tttgttttta tgaaatttta 8220
ttagttttgt cgaatggtag tatttgttat tttaggcgaa ttttttatga tagtttggaa 8280
cgaaattaat ttgttaattg attaagaatg ggttttagag tgatttgaat gtatttaaaa 8340
agttaataaa tggaatacgg tataaagttt tatttttttg ataaaaagtt tgtttggagt 8400
aaattttaat tttttttttg ttttaaaata aaagtacgat gtttttttta ttatttttaa 8460
gaagtaagta aaaataaaat aaaataaaat aaaatttaaa tagtttttta ataagagtgt 8520
ttagcgttgt ttggatggaa ttcgggttag ttttagaagt agtcggtttc gaatttcggg 8580
ggcgaggtta ttcggttttt tcggttaagt tgggttttgt ttttttcgtt tcgtttttat 8640
tggtttcggt gggtgggggt ttcggttgcg tcggtcgttt gtgcggttgt attgtagttt 8700
ttgagtttag gaagttatat tcgtttcggt tgggagtcga agcgttaatt tacgtggcgg 8760
ttggttaatt tagtcgttgc ggggagcgtt cgttgcgggt ttttttttgg cgcgaggtga 8820
gcgagtgggg gcgggtttta gatttagttt ttttgggcgg ggttggatta ttttcgtttg 8880
tttagttttt ttttattttt ttatttcggt acgtttttgt agttatattt gttgtcgttg 8940
ggaaagggaa aagagtattt taaaacgagt ttcgaaattt agatttaaat ttcgaaacga 9000
attttttcgg ttcgtttttg gttgtttgaa tgttatttgt tttattttcg tttttttttt 9060
tttttttttt tttttacggt tttttttttt tttatttttt tttttttttt tttttttttt 9120
tttttattgt tcgttttttt tttatttttt cgttcgacgt tcgttttgaa gttgaggaag 9180
tttaagtttt ggattaggag tttttttttt gtaatatttt ggggcggggt tttatcgttg 9240
ggtttaagtg gttatgggtt ttgagagtac ggtatgtgta ttattttttt taaagacgtt 9300
tagggtggcg cgtttagatt ttcgaaattt ggttttattt cggttatgtg cgttcgttta 9360
ttaagcggcg aattggattt agggcgtcgc gtgcgtttta gtcgattcgc gagaaggtat 9420
ttttgtattt tgtatagatt aagatttttt tttagggttt tttaaatttt tagatgtacg 9480
tcgtagattg tggagtgtaa aatgaatgat ttaagggaaa attagggttt tttatttttg 9540
aatataaggt tgtgaaagat gtacgtttgg tttggggggt ttgttttttt tcgtttgtgc 9600

gggatttttt tattaggggg cgtttatatt cgtcgtaaaa tttttaattt ttagatattt 9660
aggttttgta agttttgtga gttgaattgg gggtagattc gaaggagcgt atcgtttttt 9720
tcgttttttt ttttttgttg ttttggagta cgggaaaggt agggatttta tattagttat 9780
ttatttgtta gttgtggggg aggggacgta gaagttagag agagagaata gaggttggga 9840
gaggggaagg gaggtagaga agatggagat agagggagat ataaagatga ggaacgcgat 9900
ttagagagat tttgtcgcgg gtgtgcgcgc ggtaggataa ttttttaggc gtcgtgtaga 9960
tttttttttt ttttcgtagt attttttagg gatatgtagg atttgtcgag acgtagttgg 10020
attggagagt aaatagttta ggttatgata ttattttttt atttcgtttt tttttgttta 10080
atatttagat cgttttacgt ttagtgatat agatcgcggg ttagaataat tcggatgttt 10140
taggtttgta cgtattaggg atgtcgtttt atagcgttta cgtttttgta ggacggtggg 10200
gcgttttagt cgaggacgtt tttcggggtt ttcggtatac gcgcgcgtcg ttgtcgttag 10260
atttgttagt aaagttgttt aattagagcg gagaattagg agagggtggg gaatatgtaa 10320
ggaaacggag gatttttgta gtttgggcga gaggattgat ttagaggttt ttgattcggg 10380
ttttcgagtt atttttgttt gtagtgcgtt ttgggtgtcg gtttgtttgt tgagatggga 10440
gcgatagatt ttattttatt ttttagggtg agaaaaattg ttgtatattt ttgtcgattt 10500
agaataggtt tttttcgttt aggggttttt tagattttcg cggggtttta ataattttat 10560
tttttatttt ttgttttttt tttgttggtt ttttcgtatc gcgtgtagga taaaagaatt 10620
taggggcgtc gggagagaat cgtgatagtt tttaagtcgt taaagagata cggcggggat 10680
tttgtatttt tttcggtcgg attttttttt agattgtttg cggtttgaag gggttaggtt 10740
cgcgtaaggt tttaggttgt ttgggcgtcg ggtcgtattg ttaggagttg ttcgggagtg 10800
gttttttagg aatattaggt attgattata tattaggggt tgggaaatta ggtggtttgt 10860
attaaggcgg tttcggggat ttgtttgtgg taagttttgg tagtttttat ttaaattttt 10920
gtttcgagcg tgttaagaat aataataata aaaaaattaa agtgttaaag gttttttttt 10980
tttttttagt ttaagaattt attatttttt tatgattttt ttataattta tttttttttt 11040
ttttttaatt tcgttagtta ttttattttt attttatttt gggttttttt tgtttgaatt 11100
ttttttaata ttaaggtttt tttgtatgtt tttttttaaa agtttttatg aaagttattt 11160
gtatttttta agtgtttata tttttttaat ttcgttttat agttttttgt tttaattaaa 11220
gttttttatt aattgttttt tttttttaag ttcgcgggtt tttttttata agttttttgt 11280
ttttgttttt taagggggga ataaaagaaa cgtgattatt ttggaaggcg gtttattgta 11340
gtttgggggg aaaatttatt gtagcgttgc gcgattgggt tcggcgttgt ttaggcgggt 11400
tatataggaa gcgtggtggt tcggggaagg atgcggaggg tgcgggacgg tgttggaaga 11460
tgcgggagga tgcggggttg gtcgaagatt ttggttttag tttttgttta taatatttaa 11520
tgttttcggt tgttgcgttc gtacgtttgg agtttttttt tagaaaggtt cggggtagtt 11580
cgtttgtaag tttaaatgcg ggttgtgata tttattatta ttatattatt gtatcgttag 11640
agtcgaggag gagatttagc gagaagaagg aggagggaga ggaggagggt ttatttatag 11700
gttttaaaag cgtttcgtag ttttagttat ttttaagagt ttaggtttgg aaagtaggcg 11760
gaggggcgga aaggtagttt ttcgcgtttg cggtagggat cgtttgtttt tttttttagg 11820
tatttttatt ttttggcgtt tttttttgat aagaagtatt aatcggtttg gggatagtag 11880
cgttttttat ttagggattt atttagtaat atcgttttgt tcgcggaaat tattgttttt 11940
ttattttttt tttattttta ttttttcgtt tttcgtgtag ttagttttgg gttaggggaa 12000
aggagttttt aggtttttag ggggtaggtt agtaatagat aattgagtac gattattttt 12060
tttcgggagt atataaaatt gtaaaattag taaagaattt ggttatagcg tgtttacgcg 12120
tttatagagt ttgtttgttt tattaaaggg aagtgttagg tttaaggttt ttgttaattt 12180
gaaagagata ttgagaaaac gagatttttc ggggatttag agggaaagtg taagaatttt 12240
ttattgtatt tttagggaat tgtttaatgg ggagttcggt tttaaaagat tttggtaata 12300
aaaggttgga taggaaattt ttttaggtaa attttttgtc ggatttaaag agaatatttt 12360
ttttttgtta taaatttttt tttatataag tttagatttt gtttttttcg tttttttttt 12420
tttttagttt ttttaagtat ttttgagtag aatatttgat aatttttttg agtaataggg 12480
attttttgga agtattaatt attttttatg tttttcggaa ataagattat aattttttat 12540
gcgtatatgc gatttttttt ttttagttag gtttatttta ttgtgtaaat agtattaata 12600
tatggaagag ttttttgtat tgtgttataa aagattttta ataggatttt atagagaaaa 12660
gggttaaata gttgatataa aggatttttg tttttttaga aaagagggat tttggatttt 12720
tttttttttt gaagttaagt atgagtttat ataataggaa taaaataaat ttaaggtgta 12780
tattagtata atattaggga tattagaatg gatggtaaat tttttttttt atataaatat 12840
gaaagtattt ttataatttt tttttttgaa gtttttattt agaaaattat attaaatata 12900
ggattttaaa atagtagtga ttaaagatga aagttaattt ttttttttaa tttttttgat 12960
tagtttggtt taaatttatg ttttaaaatt tttagtaatt tagatttttg tatatgcgta 13020
tttataagat tttttttata ttttgtaatt tgtaggtatt tagtatggtt attgatttta 13080
agtgataaat aggtagaaga tttgttagga taattttagt ttttattatg tgtatttttt 13140
attttttttg ttatttatta gttttttgta gtaatataat tgttagttat attatttgga 13200
aattaatgtg tttttgtagt gaattattta tttttagtaa atttattgta ttattatttt 13260
aaatattgaa atttgatttt attagatgtt tagaatgata gttatagagt agaattagat 13320
tttaattttt atgtataata tgtaaataaa ttaaggtgta gaaagttatt gttttaaagt 13380

tatataatta atagtttagt ggttttttta gtgttaatta tagtaatttt agatttaaga 13440
ttgtttgatt taggaatgga gagaaataaa ataaaatgaa tgtttattga aatatatatt 13500
ttagatatta agatagattt ttttgtgaaa tattttaaga tgtggatttg tatttttatt 13560
tttaaatttg agtaaattta gttgagtttt aaagagttta agtaatttgt ttaaggttat 13620
tttgtaagtg atagatttgt tagtttgaaa agtttattat tttttatttt tttgtttttt 13680
tatgatatag ttaagtaaaa ttttttaata aaggagaatt agattagaaa taagtaatta 13740
atagtagatt aatgtttatt tttagggaaa tgttaatagt taatatttat tataaataat 13800
gttatttgta tataatggaa tatgattgaa agtgatgggt tagatatttt ttatattttg 13860
agatgttttt atttatgttg ttaaatttga ttttttaatt ttttaaaatt tttagttttt 13920
tttaatgtta atagatttta taatatttga ttaaatattt ttataggata aaggtatttt 13980
tttagggttg atttaaggaa ttagaatatg tataatgttg aaaagtttta aattgtttgt 14040
tttttttttt ttttgatttg taatattatt atttagaaaa ttaaatattt ttttaggtta 14100
ttagataatt aaaatgaaat tatattttat ggatattttt tttttatttt tagggtagat 14160
tttttttttt agttgtttaa aatggagaat ggataaaaaa gaaaaaggaa aatatattag 14220
ttatatttag aatagagttt atagttgtat tattattttt atgtaattgt ttttaatgat 14280
ttttttttat taagagaaag aaaatgagag agtatttatt tttgattttt taatgtttga 14340
taaaattatt agaagttttt gaatgggaaa atatatattt tttaggtttt ataattaaaa 14400
ttgtatttat ttgtttatta tatttaagta tgaattaaat agtaattaaa atattatttt 14460
gtgagttttt ttttaaatta tatttttgta ttggggatat tttttgtgaa aattttaaga 14520
ttttaggtat aaataaagta agatttttat ttataatgtt tgtgtttaaa agaaattata 14580
agttagaagt tatgtgtttt gtttttttgt tttgattttg tttttgggaa tttggttgag 14640
tttaggggta gtaagttaat gatttggaga ggaaaggtgt agatggtaga tgtgttttta 14700
gggaatttta ttatttttat ttaaattttt ttttgaggtt ttttaggatt atattttaga 14760
tttttttttt tttattgatt aggtgaagtg tttttagata tggtattatt tattgttgtt 14820
ttagggatga taagatttta gttttattta aatttagaaa gagtttagtt ttaatttttt 14880
gatttgattg gttttaggta taattaagta aattattttt attggatgat atagtaatgt 14940
tatagattaa aaatgtattt ttaattttta aagattttgg aaaaaggaag tttatttttt 15000
aagattgtgt tataattttt tttttaaata aaatattgta aattagggat agatatttta 15060
tttagtattt tgaatttttt ttggtttttt aggaaaggta gaaatataag gtttttattg 15120
tttttttttt atttttaaaa aagaaatttt gtaaaaggta aagtgatggt ggtaatttta 15180
ttgtgtttta attcgaagta tataaattgt tttgataaat taaatataga taaatttttt 15240
tagaaagtta taaaatatta gatttttttt agatattaga tattgagttt ttgtttttta 15300
atttttaaat taaaatgtta tagtaaaata tttgttttaa taaatttttt ttttttttgt 15360
gttgtaaatg ttttgtttat ggttgagtag atttttttag aatttaattg aaagattatt 15420
tatattacga tagaattatg taagttaaag ttaagtttta atatggagtg atgggagagg 15480
tcgggttgtc gagaggagat gattttatta gtgagaagag aggttttttg agtcggtttt 15540
taaggtagtt aaatggttta gtatatttgt atttattttg ggaggttgtt tggagtagag 15600
tattaaaaac gttatttttt ttataaagaa aaagatgaag gtataaagaa tatagtttaa 15660
ttaaattgaa ttaaagattg tattattttt attttttttt tagttatttt tttaaagttt 15720
tttttttttt tttttatagt ttgaaaaata taggttattt ttataaagta tttgtttgat 15780
tgaagtaaga taaattgaag agtttggttt ttgaatataa ggaagcggta gagattttag 15840
aattaatgag gtagggcgga atataatgta gaaggttttt taagaggtaa aatttttgta 15900
gttgttgttt atttttagtg attattcgtt tttatttatt taattagatt atagtgggat 15960
cgagataagg agtataatga aagttgttgt attgatggtg taaaaagata tatggtatta 16020
ttaatgtttt tgtggtgagt aaaggtagaa cgtaagatag aaaataagga aatttttgcg 16080
ttgtaagaaa agtgtttagt atgggtatta atcggtatgt agaagtagta agtatatatt 16140
ttatttttat tttattgata aaaagaattg taggattaat gtttgttttt attaaaatat 16200
gagaaattat attttaattt tataaattaa aggttaaata tttgttgtaa taattttaaa 16260
aaaaagagaa gatttgtaag atttagtaaa tatttattat aaatagattg tttttaattt 16320
tttatatttt tttttaattt aataaaagtt tattgagtag tttttatgtg tattgtttta 16380
gatagtaggt attatttaaa aaagtaataa ttatttttta tttttaagga gtttataatg 16440
tttttagaaa gaaattaatt tattggagta gttaaagtta taattgattt ataaaatgat 16500
tagtgaattt agaggaaaat tatatataaa tatttttgtt tttattggtt ggtttagtta 16560
gatattattt taaaaaata 16579

<210> 175
<211> 6090
<212> DNA
<213> Artificial Sequence

<220>
<223> chemically treated genomic DNA (Homo sapiens)

<400> 175

tagagatgga atgtttaaat taggataatt tagggaggtg tgggagaggt gtagggggat 60
tataagggat ggagtaggaa tttaaggtag taatagtaga gttggtatta attttaggtt 120
taaaaagata aaggagagag agtggttatt aaaatttaga aagagaaaat tcggtaaaag 180
acgggatttg gtgaggagta gtaatttgtg tttggttgtg gattgtggta attttgtagg 240
gaggagagta gaggaatgat tattttggtt ttattttttt tttttttttt ttattttatg 300
ttaggtgttt tattggttaa atttattagt aattagagga taaggaatgt attaaaataa 360
ttttacggtt tagagagtag attagatgta gagtgataaa tggattatat ttagtataat 420
cgtaggttta tttgttgatt cgtttttttt attagattga gttatttgag gatagagaat 480
gtttttttta tatttgtatt tttaatattt ggtggatatt agatatttaa gaaatgttta 540
tggacgggat tagagaagat ggtaaattgg gtattagatg gaggtttgtg ttttttgagt 600
ttgtcgtatg ttttttggcg aatttaaggt aaaatgagaa atagaatgag ttttgtattt 660
ttgtttggta aaagaaaata tgatttggtg agaagaaatg aatattttta gtatttagtg 720
ttgggaggaa ttggaattag ttatgtgatt gtttttatta gaggttttta attgcgtaac 780
ggatagaatt tttaatggtg aaattataat gtagaaatat tttttttttt tttttttttt 840
ttttttttga gacggagttt tgttttgtta tttaggttgg agtgtagtgg tattatttcg 900
gtttattgta agtttcgttt ttcgggttta ttttattttt ttgttttagt tttttaagta 960
gttgggatta taggtatttg ttattatatt tggttaattt tttgtatttt tagtagaaat 1020
ggggttttat cgtgttagtt aggatgtttt tgattttttg atttcgtgat ttgttcgttt 1080
cggtttttta aagtgggaaa tatttttaaa aatattagtt tttgataaaa ttaaagttat 1140
gttataaaaa agtaattaaa aggtggaaat gagataatat ggtttatttt gagttgagaa 1200
tattttgtta gaaaatgatt gttttagatt ttaaatttga ttttttatat aaatttttag 1260
tgtaattttt tttgtattat tttttttttt attagttaaa agatggaaag gataaattga 1320
ggaatttacg ttggaggagt atggatttat tagatggtag aaatttgggt ttttgaatga 1380
ttattgtgga gttgattttt gtttatgttt tgtaaatttt tgtgttgaga gtaagtaata 1440
tatttttaat gtgttaaatt tttgggattt gttgggtttt atagtaatgg ttattttatt 1500
ttaatacgat tgtaaaataa tgttttaaaa tttatagtcg ggtttgttta aaatattatc 1560
gttattatta ttataattta tagtttatat tataattatt tttatagaag ttttttttaa 1620
ttggtatttt ataatttgtg gttttttata ttgttgtttt ttatgtgtga gatgtttttt 1680
tgagtttttt ttgttaggga tatgatagaa aataaaatat ttaatatttt ggtgtatttt 1740
ataatgtata aagttttgtt ttatgtatta tttttttaga tttttttaat aaatttaagg 1800
aatatgtaag ggtttataat tatttttatt ttataggtga tgaaattgag acgtttatat 1860
gtaaagtgga aataattatt tatttattta tttatttatt aaataaaaat ttcgttaatt 1920
tatttaaaga ttaattgaga gaattatttt atttgtttgg aatttggtat gtgttaagag 1980
aaaagttttt ttaatttttt tttttaagaa gataatttat tgttatataa taaattattt 2040
tgaaataatg atttaaaata ttaaaagtta ttgattttgt ttatagaatt tgaattggaa 2100
gatataaata attgggagtg agggtggggt tggatagttg gtttttttgg gtatttttcg 2160
ttttttttgt ggttttgtta tatgttttta tatggtagtt ttagggtaat tatgatagtt 2220
gaaggtttta gagtgggtgg agggggagag ggagggaggg aaagagagag agagagagag 2280
agagagaaag agaagtaagt aggtagaagt tttaatgttt tatgatttaa ttttggaaat 2340
aaattatatt gtgttattgt tattattttt ttttagaagt aagtagatag ggttggtgaa 2400
tatttattga gaggggaatt agattttatt ttttgattgg gggagtgtta aagaatttgt 2460
gaatatgttt ttaaatgatt atattttttt tttttaaaat gtttgtttaa attggtgtat 2520
ataaaaagtt cgggggttag tgatttattt aaggttatta agttgttaaa tggttttatt 2580
aatattagat ttaattttgt gattttaaat tttatgtttt tttttattat tttagataat 2640
tttcggtgaa tattattgtt tattagggta gttaagatat tttagttgtt aaatagattt 2700
agttttttta attgtttcgg ataatatatt ttttattttg tagaaaataa atattattta 2760
tatggtagtt atttataaat attttttaag tttattttaa atatttgtgt tttaaatttt 2820
tttttttaat agatgtattt atttgtattt ttttagatga attatttttt ttttgttgtt 2880
gttgttattt ttgattgtat tttttatttt ttaagatttt tttgtattat taagtttatt 2940
gtttaagtta atattatttt ttaatttggg gatttttttt tgatttttgt gtgttttttt 3000
tgtgggttat taatgtaatg ttatttaaga ttaggatttg gttgggcgta gtggtttacg 3060
tttgtaattt tagtattgtg ggaggtcgag tcgggaggat tatttgaggt taggagttta 3120
agattagttt gattaatatg gtgaaattac gtttttatta aaaatataaa attagtcggg 3180
tatagttata tgtttgtaat tttagttatt gggtagtttg aggtaggaga atcgtttgaa 3240
ttcgggaggc ggaggcggag tttgtagtga gttaagattt tataattgta ttttagtttg 3300
ggttataaga gcgaaaattc gttttaaaaa aaaaaaaaag attaggattt gatataaggt 3360
ttgaggggta tttttttgtt ttgttttgtt ttgtttttaa gaggttaaaa tttttatagt 3420
tgaaaatttt tgttgaatta tagagatttg aattaattta gtttagaaag tttggggatt 3480
tgaataacgg tatggatcgg aaattttttt atttgttagt ttttattatt ttaggtagta 3540
aaatagattt ttttttagga gttttttatc gtttggggtt ttttagtagt gggatgtggg 3600
gaattaattt tttttcgttt ttatttaaat attaggtggg agtaaggggt gggaagtaga 3660

gaaagtggat agaggttttt agtggatatg ggatttttgt gtagattagt atagttttta 3720
gaaattttat gtaagtaata taggtattgt tatatttttt agtggttatt ttttaaaaag 3780
taaataggtg aggtcgggcg cggtcgttac gtttgtaatt ttagtatttt gggaggttta 3840
ggcgggcgga ttacgaggtt aagagatgga gattattttg gtcgatacgg tgaaatttcg 3900
tttttattaa aaatataaaa attagttggg tatggtgacg cgcgattgta gttttagtta 3960
ttggggaggt cgaggtagga gaattatttg aattttggag gtggaggttg ttacgtttta 4020
ttatatttta gtttggcgat agagtgagat ttcgttttaa aaaaaagaaa gtaaataggt 4080
gaaattaatt ttaataatat attttgttta atttaacgta tttaaaatat tattatttga 4140
aagtgtaatg aatataaaaa tatttatgag atatttttta tttttatatt tatattgttt 4200
tggattttaa tgtgtatttt atatttatag tataattaat ttgggattag ttatatttta 4260
gtttaataat agttaatagt atatgggata gcgtaaataa attttgcgtt tttgttgttt 4320
ttttgggttt cggagatttt aatttttttt ttagattgta aatttttttg tttttaagtt 4380
tcggttttaa tattagttcg gtagaggaat ttagtttaat gaggtacgtt ttttttttgt 4440
tattttttat tttattaatt tgtttcgtgg taaacgtagg attgattttt taaaattatt 4500
ttattaatta gtttatatat ttattattta tttgttttat tagaatgtag gttttcggaa 4560
ggtagggatt taaaaaaatt tgttttgttt tatgtgattt ttttatatta agtatcgtgt 4620
tcggtataag ttgggatttt agtatatatt tcgggacgga agaatcgtgt ttttttagaa 4680
tttagttaga gggtagttta gtaatgtgtt ataggtgggg cgttcgcgtt tcgggcggac 4740
gtattggttt ttcggtcggc gtgggtgtgg ggcgagtggg tgtgtgcggg gtgtgcgcgg 4800
tagagcgcgt tagcgagttc ggagcgcgga gttgggagga gtagcgagcg tcgcgtagaa 4860
ttcgtagcgt cggtttggta gggtagttcg gaggtgggtg ggtcgcgtcg ttagttcgtt 4920
tgtagggttt ttattggtcg tttgtcggtc gtttttcgtt taaaaggcgg taaggagtcg 4980
agaggttgtt tcggagtgtg aggaggatag tcggatcgag ttaacgtcgg ggattttgtt 5040
tttttcgcgg aggggattcg gtaattcgta gcggtagggt ttggggtcgg cgtttgggag 5100
ggatttgcgt tttttattta ttttttagtt gtgttttcgt cgcgtttcgg ttagttttcg 5160
gcgttggcgt ttatggtcgg ttttcgatag cgtttcggag ggatcggggg agtttttagg 5220
cgttcgggtg agtagttagg cgcggttttt cggttttttc gattttcggc gttagttttt 5280
gtttttttag ttagggcgcg gtggggtttg ttcgggtagt gtttcgagta attgggaagg 5340
ttaaggcgga gggaaatttg gtttcgggga gaagtgcgat cgtagtcggg aggttttttt 5400
agtttcgcgg gtcgggtgag aataggtggc gtcggttcga ttaggcgttt tgtgtcgggg 5460
cgcgaggatt tggagcgaat tgttgcgttt cggtgggtcg tttttttttt ttttttgttt 5520
tttcgggcgg tcgtacgtcg ggtcggtcgg gtaacggaga gggagtcgtt aggaatgtgg 5580
ttttggggat tgtttcgttc ggggaagggg agagggtggt tacggtgtta ggagaggcgc 5640
gggagtcgag aggtggcgcg ggggtgttat cgttgtcgta ggttggagag agattgtttt 5700
tagtgaggcg cgtatcgttt gggcgagggt tttatttttt cgcggcgttt ttggaggtgg 5760
gaaagttggg tgggtatgtg tgtagagaaa ggggaggcgg ggaggttagt tattttcgga 5820
gtcggttttg attttaatag atcgtttagc gtttggggac gtcgatttcg gggtgtcgtg 5880
gtgttcggtt ttacgcgcgc gcggggttga ggggtcgggg gcgtttttgg tcgtttagtt 5940
ttaataaagg gtgttttttt ttatttcgcg aggaggggta gtttcggaga ttcggttttt 6000
agcgagcggg gttttagcgt cggggaggtt tatttttttt tggggttgtt attttattat 6060
tattattgtt tttttttttt ttttaaaagg 6090

<210> 176
<211> 6090
<212> DNA
<213> Artificial Sequence

<220>
<223> chemically treated genomic DNA (Homo sapiens)

<400> 176

ttttttgaag aaaaaaaaaa ggtaataata atagtaaaat ggtaatttta aaaggaagta 60
gattttttcg gcgttaagat ttcgttcgtt gaagatcggg ttttcggagt tgtttttttt 120
cgcggggtgg agaggagtat tttttgttaa agttgggcgg ttagggacgt tttcgatttt 180
ttagtttcgc gcgcgcgtgg ggtcgggtat tacggtattt cgaggtcggc gtttttaaac 240
gttgggcggt ttgttgggat tagaatcggt ttcggaagtg attggttttt tcgttttttt 300
tttttttgta tatatgttta tttagttttt ttatttttag ggacgtcgcg gaagaatgaa 360
gttttcgttt agacggtacg cgttttattg ggagtaattt ttttttagtt tgcggtaacg 420
gtggtatttt cgcgttattt ttcggttttc gcgttttttt taatatcgtg gttatttttt 480
tttttttttc gagcgaggta gtttttagag ttatattttt ggcgattttt ttttcgttat 540
tcggtcgatt cggcgtgcgg tcgttcgggg gagtaaggga gggaagggag cggtttatcg 600
aggcgtagta gttcgtttta gattttcgcg tttcgatata aagcgtttgg tcgggtcggc 660

gttatttgtt tttattcggt tcgcggggtt ggggaagttt ttcggttgcg atcgtatttt 720
ttttcgaagt taagtttttt ttcgttttgg tttttttagt tgttcgaggt attgttcgga 780
taaattttat cgcgttttgg ttgggaaagt aaaagttggc gtcgggggtc ggggggatcg 840
gggagtcgcg tttggttatt tattcgggcg tttgggagtt ttttcggttt tttcggagcg 900
ttgtcggagg tcgattatag gcgttagcgt cggagattag tcggggcgcg gcgggaatat 960
agttagggag tgagtggggg gcgtagattt tttttaggcg tcggttttag attttgtcgt 1020
tgcgagttgt cgagtttttt tcgcggaggg aataaagttt tcggcgttgg ttcggttcgg 1080
ttgttttttt tatatttcga agtagttttt cggttttttg tcgttttttg ggcggagggc 1140
ggtcggtagg cggttaatgg ggattttgta agcgggttgg cggcgcggtt tatttatttt 1200
cgagttgttt tgttaggtcg gcgttgcggg ttttgcgcgg cgttcgttgt tttttttagt 1260
ttcgcgtttc gggttcgttg gcgcgtttta tcgcgtatat ttcgtatata tttattcgtt 1320
ttatatttac gtcggtcggg gagttagtgc gttcgttcgg aacgcggcgg ttttatttgt 1380
gatatattgt taagttgttt tttgattggg ttttagggaa atacggtttt ttcgtttcga 1440
gatgtgtatt gggattttag tttgtgtcgg gtacggtgtt tggtatggga aaattatata 1500
gaataaaata gattttttta aatttttgtt tttcggaaat ttgtattttg gtgggataga 1560
tagataataa atatgtaagt taattaataa ggtaattttg gaggattagt tttacgttta 1620
ttacgaaata ggttaatgga atagagaatg gtaggaaggg agcgtatttt attagattgg 1680
gtttttttgt cggattggtg ttggagtcga ggtttgaagg taagaaggtt tgtaatttga 1740
agaaagggtt gaggttttcg agatttaaag aagtaataga gacgtagagt ttatttgcgt 1800
tattttatat gttattggtt attgttgagt tgaaatgtag ttagttttaa attaattgtg 1860
ttgtaagtgt aaaatatata ttagagttta agatagtatg gatatgagaa tgaaaaatat 1920
tttatgaata tttttatatt tattatattt ttaaatgata gtattttgga tacgttgggt 1980
taaataaaat atattattaa aattaatttt atttgtttat tttttttttt ttgagacgga 2040
gttttatttt gtcgttaggt tggagtgtag tggagcgtgg taatttttat ttttagggtt 2100
taagtgattt ttttgtttcg gtttttttag tagttaggat tatagtcgcg cgttattatg 2160
tttagttaat ttttgtattt ttagtagaga cggggtttta tcgtgtcgat taggatggtt 2220
tttatttttt gatttcgtga ttcgttcgtt tgggtttttt aaagtgttgg gattataggc 2280
gtgacgatcg cgttcggttt tatttgttta ttttttaaaa ggtggttatt agaaaatata 2340
atagtattta tgttgtttgt atgagatttt tgaggattgt gttggtttat ataaagattt 2400
tatatttatt ggagattttt atttattttt tttatttttt attttttgtt tttatttaat 2460
gtttgggtgg agacgaagaa gggttgattt tttatatttt attgttggag aattttaaac 2520
ggtgaaaagt ttttaaaggg aaatttattt tattgtttag aatgatgaaa attgatagat 2580
gaagagattt tcgatttata tcgttgttta aatttttagg ttttttaaat tgagttggtt 2640
taaatttttg tggtttaata gaaattttta attgtgaaga ttttggtttt ttgaaaataa 2700
ggtaaaataa aataaaagaa tattttttag gttttatgtt aaattttagt tttttttttt 2760
tttttgagac gggttttcgt ttttgtggtt tagattggag tgtagttgtg aaattttggt 2820
ttattgtaaa tttcgttttc gtttttcggg tttaagcgat ttttttgttt taggttattt 2880
agtagttggg attataggta tgtgattatg ttcggttaat tttgtatttt tagtagagac 2940
gtggttttat tatgttggtt aggttggttt tgaatttttg attttaggtg attttttcgg 3000
ttcggttttt tatagtgttg ggattatagg cgtgagttat tgcgtttagt taaattttag 3060
ttttaaataa tattgtatta atgatttata ggaagggtat ataggaatta aggggaagtt 3120
tttaaattgg gagatggtat tagtttaagt agtgggtttg ataaatataa agggttttga 3180
gaggtgagaa atatagttaa aaataataat aataataaaa aaagagtgat ttatttggga 3240
aaatgtaaat gaatatattt gttgggagaa aaaatttaaa atatagatgt ttgggataaa 3300
tttggaaggt gtttatggat gattgttata tgggtaatat ttattttttg taaaataaaa 3360
aatatattgt tcgaggtaat taaaaggatt gaatttattt ggtagttaga atgttttaat 3420
tattttaatg gataatgatg tttatcggag attatttgga gtagtgaaaa agagtatgaa 3480
atttggagtt ataaaattag atttagtatt ggtggaatta tttaatagtt taataatttt 3540
ggatgagtta ttaattttcg agttttttgt gtatattaat ttagataagt attttagaaa 3600
ggggaaatgt ggttatttaa aaatatattt ataaattttt tgatattttt ttaattaaga 3660
ggtggagttt aatttttttt ttaataaata tttattaatt ttatttgttt gtttttaaga 3720
aaaggtagtg gtagtggtat agtgtaattt atttttaagg ttaggttata aggtattaga 3780
gtttttgttt gtttgttttt tttttttttt tttttttttt tttttttttt tttttttttt 3840
tttttttttt ttatttattt taaagttttt agttgttatg attattttag ggttgttatg 3900
tagagatatg tgataaaatt atagaaagaa cgagagatgt ttagagaagt tagttgttta 3960
gttttatttt tatttttagt tgtttatatt ttttagttta agttttgtga gtaaaattaa 4020
tgatttttaa tgttttaagt tattatttta gagtggtttg ttatgtagta atagattatt 4080
tttttaaaaa gaaaaattaa gaaaattttt tttttaatat atattaggtt ttagatagat 4140
gaaataattt ttttaattaa tttttaaata aattagcggg gtttttattt aataaataaa 4200
taaataaata agtggttatt tttattttat atgtaaacgt tttagtttta ttatttgtga 4260
aatgaagatg attatagatt tttatatatt ttttgggttt gttgggagaa tttaaagaga 4320
taatgtatga aataggattt tgtatattat aaaatatatt agaatgttgg atattttatt 4380
ttttattatg tttttagtaa aaaggattta gagaggtatt ttatatatag aaggtaataa 4440

tataagaagt tataaattat gagatgttag ttgagaagga tttttataaa aataattatg 4500
atatggatta tggattataa taatgatggc gataatattt taaataaatt cgattgtaga 4560
ttttagaata ttattttata gtcgtattag ggtaaaataa ttattgttat aagatttaat 4620
aaattttaga ggtttaatat attaaaaatg tgttatttgt ttttagtata aaggtttgta 4680
gggtataagt agagattagt tttatagtag ttatttagaa atttaggttt ttgttattta 4740
atgaatttat gtttttttag cgtaagtttt ttagtttatt tttttatttt tttggttgat 4800
gagagaagag ataatatagg gaggattgta ttgaaggttt atgtaggagg ttagatttga 4860
agtttgagat aattattttt tggtaagatg tttttagttt aaagtggatt atattatttt 4920
atttttattt tttggttgtt tttttgtaat atggttttgg ttttattaag ggttaatatt 4980
tttaagaatg ttttttattt tgggaggtcg aggcgggtag attacgaggt taggagatta 5040
agagtatttt agttaatacg gtgaaatttt atttttatta aaaatataaa aaattagtta 5100
ggtgtggtgg taggtgtttg tagttttagt tatttgggag gttgaggtag gagaatgggg 5160
tgaattcggg aggcggagtt tgtagtgagt cgagatggtg ttattgtatt ttagtttggg 5220
tgatagagta agatttcgtt ttaaaaaaaa aaaaaaaaaa aaaaaaaaga atgtttttgt 5280
attgtggttt tattattaaa gattttgttc gttacgtagt tgagagtttt tgatagaggt 5340
agttatatga ttgattttag ttttttttag tattgagtgt taaaagtatt tatttttttt 5400
tattaaatta tatttttttt tattagataa aagtatagag tttattttgt tttttatttt 5460
gttttggatt cgttagaaag tatgcggtaa gtttagaaag tataaatttt tatttaatgt 5520
ttaatttatt attttttttg atttcgttta taagtatttt ttgagtattt agtgtttatt 5580
aaatgttagg gatatagata taagagaaat attttttgtt tttaggtaat ttagtttgat 5640
gagggagacg gattaataaa tagatttgcg attgtgttaa atataattta tttgttattt 5700
tatatttaat ttattttttg agtcgtaaga ttattttaat gtattttttg ttttttggtt 5760
gttggtaggt ttagttaatg gggtatttga tatgagatga aggagaagga aggagagtga 5820
agttaagata gttatttttt tgtttttttt tttgtaagat tgttatagtt tatagttaaa 5880
tataggttgt tgttttttat taggtttcgt tttttatcgg gttttttttt tttgggtttt 5940
ggtaattatt tttttttttt tgtttttttg agtttagggt tggtgttagt tttgttatta 6000
ttattttggg tttttgtttt attttttatg gtttttttgt atttttttta tatttttttg 6060
aattgtttta atttgagtat tttatttttg 6090

<210> 177
<211> 6274
<212> DNA
<213> Artificial Sequence

<220>
<223> chemically treated genomic DNA (Homo sapiens)

<400> 177

ggattatttg agtttgggaa gttgaggttg tagtgagtta aggttacgtt attgtatttt 60
ggattgggta atagagttag attttgtttt aaaaaaaaga aaaattttat ggttttgttt 120
atattattta tttgatttta tatgttgttt atttttttta ttaaaatttt tagtttatta 180
attatagttt ttttataatt aatatttcga tgtgataatg ttttagttta attattgtgg 240
ttataataga atgttataaa ttgggtgatt tataaataaa agaagttgat ttaggttgat 300
ttagaggttg gggagtttaa gagtttggtg ttagtatttg atgagtgttt ttttgtttta 360
ttataatatg ggagagggta ttacgtgtga agagagttta tttttataat atagttattt 420
ttataagaat taatttatcg ttatgagagt tatgtgaatt tatttatgag gatagcgggt 480
taagttttta atatatggat ttttcgggga tatatttaaa ttatagtagt tagttgtaac 540
gttcgtgtta tgttttattt tggttttgat gtttgtgtag tttttttaaa ttgcgttttt 600
gttttttagt gtgttttgta atgtggaaat gatatattgg gtaagaggag ttgtagtaaa 660
gaggttttta gtgacgtagt gataagttgt ggggagaggg agtgttgtat agttttgtcg 720
tatgttatag ttttttagtg agtttgtgtt tttggattgt gaattttatg tttgtttttt 780
agttttttta gttttttaga tggtatagaa ttgttggagg ggggtggagt tgtatatttt 840
ttttgttttg ggtaggttat tttttgataa aatattaggt taggtttttg gtgaaataat 900
tttttttgag ggtagatttt ttattaataa tagaatgttt taatttattt taaaatggtt 960
tttttttttt ttttattgtt agaagtataa tgagattttt tttttaatat tcgtggtaag 1020
gatttagtag agttttagga ggtaatattt ttaagtgttt tatattattt tgtattatga 1080
ttgggttttg ttggagtttt taatttgtag aattgtttat attgagtttt tcgtaatttt 1140
ttaattatag ggtaaatttt tttagtcggt attgggtttt tggaggtttt tgtttgttgg 1200
tttttttttt tggaggttgt gtttttgtgt ttgtttgttt ttttaatttg gggggtagtg 1260
gtttgtttaa tgattttaat tttttgaaag agttaagaag aggtgttaat ttttcggttt 1320
gtttagtttt ttatttgttg ttagaatgga gcgttaatag tgttttttat agtgatatgt 1380
aatttttaat tttagagatg atgaagtata ttaatgataa aggagaaatg ttttagtagt 1440

tttttgttag tatattattt tttgaaaaag ttgttttttt tatattttgt aagagatggg 1500
ttttaattta gagtttaagg taaatgattt tttttaagga gaaggaataa atagttttag 1560
aaattatgaa agtttgtttt taggagtgtt tttgaatttt agtaggggtt atatttattt 1620
tgtagaaata ggtgaggtat gtttttggta taaaatttta atggtataga aggataaatt 1680
gaaaaataag ttttttttta aatttttgat ttcgagttat ttagtttttt tttttagagg 1740
taaagttgtt attagatttt tgtatttttt taatatatat ttttagaaga gttgttaagt 1800
gaatatatgt ttaagtgaaa atttatttta gaagtgtatt tttttaagga attttagggt 1860
tggaaggaat ttgtgttagt ttttaattta taatttttat tagtatttat tgtttttgta 1920
taaaaatttt ttttaaaaaa gtttttttta ttttgatata gtttatttta tttttattta 1980
gttttaataa tttataaaat atatttttga aagtttaaaa tttgttatta tgtttttttt 2040
ttttaattaa tttttatttt gatttttaag ttagagaaaa taggtggtta aatgtttttt 2100
gtttaagatg gaatttagaa tatttgaaga ttttagattt ttattttgtt aaataacgtg 2160
tttttttttt ttttttatag agtatttggt tttaggaaat ttagagttat attttttata 2220
gataagatta aatttttatt taatatattt agaaattttt tttaagagga taattattta 2280
ttagaggaaa aaagtttttt atgtatagtt ggtaaaggga tggaattatt tgtgttatta 2340
aaattgatag acgtttatga gatttattaa gggaaatatt agagttttag tatatatttg 2400
ttaatatagt atatatgaag gttttattta taattttttt ggttaagtag aaattttggt 2460
attatttatt ttaataaatt tttaagatat tatgaaatag aattttaggt tttgatatta 2520
ttatttgttt taggttttga agcgttgttg gataagaggg gtaaaatacg gttttgtttt 2580
ggatttaaag ttggtttttt atattagtaa gtatattttg gtattttggt tatttttttc 2640
ggttatagta ttatattgtt ataaaaggta gatataagta ttaattagtt tataggttat 2700
tagataagtt tagtttgatt aatgtttaat atagtaattg ggttattatt gttatttttg 2760
tggtggtggt atatatattt agtttttgtt cgggttatgg tttaggatta ttttttatag 2820
aggttgtgag ttagagtttt aattgtgtag ggttttaatt atgttaggtt atttattttt 2880
tttaagagga ttttattagt gtttgttcgg ttatatagtt ttttatttat taagtaatat 2940
agttattagt atattttagg tattagttaa ggattataaa attaacgtga atgttagttt 3000
ttgtattaaa agtttaaagg agaaatttaa attttatata gatgttttat gaagatgttt 3060
agtaaattta tttttttttg ttttttggaa tttattttag tattgtgtta tgtgtgtgtt 3120
tagtaatttt ttataaaaag ttttgttttt tgtgatgtta ttagattata ttgaagaata 3180
tataagtcgt attatgaagg ttgttgtttt atatagtttt aacgtagtga gaattgatgt 3240
ttttatatgt tgtttttttg ggtatttaaa gaaatttttg tatagtttta taaattagtt 3300
gtagtttaaa ttgatttgtg ttgtgatttg tatatatagg ttatattttt ttgatagaaa 3360
atatagttta aaattaaatt tgtagttttt gttaagtgaa tgtataggat tttattgtat 3420
ttaggttttt tattgtaaga tttatttttg tttttatttt atgttttatt gttgtgtttt 3480
ttatttgttt ttttttagtt ttgttttttg tgtttttacg gattgttttt agtttaggtg 3540
tgtaggaagt atatatatgt ttgtagagtt tttatggttt ttgtatttag ggtatgattt 3600
taacgtatag tggttgtatt gatttgttaa aataaaggaa tagattattt tttttaattt 3660
atagggaagt tttaggttgt gcgggtagtg agtagatttg tgtttgtttg cgtttgtttt 3720
ggtgaaaaat tttatcgttt aggttgtagg gtgcgagatt taggtataaa tattttgttg 3780
gatgaggagg aaagatgtaa ggttgttttt ttttagagat agtaaagggt aggtttgtag 3840
ttttatttat tttaggattg tgatttttga tagagtcgag agattagggt tgttgaatta 3900
ggtttgaagg ttttagtgaa tttcgtgaag agaggagggg tttggttgta atatggattt 3960
agaggatatt tttattgtag gagaaggaat agtggggatg gggtggattt gttaaaggaa 4020
tatagtttaa gtttttgtag tttaaaaaag tttagttttt tttggttaaa gttttcgcga 4080
gtttttttgg tatttttttt gcgggagtta taggggtagt gggatattta gtttttttaa 4140
aagtattttt acggttgttt gtgttaagtt tttattttaa gagttttatt tttgcgaagt 4200
aatgtgtttt atatattggt tttaaagtat ttatggttgg ttgtaataaa tattaaggag 4260
gtttgttttt gtattcggag ttgggtgttt ttattttaga tgatttcgag ggtgtttgat 4320
aagatttgaa ggattttcgg attttagagt attatttcgg acgtttggta tttttgtcgc 4380
gcgggtacgg cgattttttt agttgttagg ttagtttttg attttcgaga gggtttcgta 4440
gtgttgtagg ggaggtgggg attcgaataa aggagtagtt ttttcgtcgg tgttattatt 4500
cgacgttggt tttaaggttc ggttagtttg tttaaagttg gtataagttt gttttgtaaa 4560
ataaaagaag ggaaaggggg aaggggattt tggtatagat ttggttcgat ttggatatag 4620
gttgggtttg taagttcgcg gggatcgggt ttagaggggt agtgttggga acgttttttt 4680
cggaaattaa ttttttaggg tattcgtttt ttttttatgc gtcgttttat tttcgtcgga 4740
gattaggttt cgcgggggtt atcgttgttt atcgttttcg gcggtcgttg gttttgggtt 4800
ttcgttgttg gttttttttt ttttttttcg tatttttttt tttttttgtt tttttcgatt 4860
tttttttcgt cgtttggttt tttttttttt cgttttgttt tttcgcgttt cggttcgcgc 4920
gagttagacg ttcgggtagt tttcggcgta gcgcggtcgt agtagttttc gtttttcgta 4980
cggtgtgagc gttcgacgcg gtcgaggcgg tcggagtttc gagttagttt cggcggtcgt 5040
cgtcgtttag atcggacgat aggttatttc gtcggcgttc gttcgagttt tcgtttcgtc 5100
gttaacgtta taattatcgc gtacggtttt ttgatttcgt ttagtattga tcgggagagt 5160
cggagcgagt ttttcgggga gtagcgatgc gatttttcgg gacggtcggg gtagcgtttt 5220

tggcgttgtt ggttgcgttt tgttcggcga gtcgggtttt ggaggaaaag aaaggtaagg 5280
gcgtgtttcg tcggttttcg cgtcgttttc ggatcgcgtt tcggatttcg tagttcgttt 5340
aatcgcgtat cggcgtatcg gttcggcgtt cgcgttttcg ttcgtttttt tttgtttttt 5400
tgagattagt tgcgtcgtcg atcgggatcg cgggaggaac gggacgtttc gtttttcggt 5460
cgggagagtt tggggcgggc ggaggaggag acgcgtggga tatcgggttg taggttaggc 5520
ggggaacggt cgtcgggatt ttcggcgttt cgaatcgttt ttaatttttt ttttttattt 5580
ttttcgttta gttgcgtagg atcggcgtta gtgggcgaaa gtcgggtgtt ggtgggcgtt 5640
tggggtcggg gtttcgtacg tgcgtttcgc gttgtttttt tagggcgcga cggggttttg 5700
gcgcgtattc gaggggcggg cgttgtttat tcgtcgagat tgtattgttt agggaagttg 5760
aggaaggaat ttaaaaatat agtttttttt cggatttcgc gggataggcg gttttttgag 5820
aggatttttt cgttttcgtt tttcgcgtag gttttaaatt gaagtcggcg ttcgttagtt 5880
tggtttcggt ttttttttag gttttcgcga ttttcgtttt ttagtgtgga gtcgtagttt 5940
cgatttggga gttgggagaa ttcgtttatt attatttgcg gttttcgggg aggggtggtg 6000
ttggcggcgg ttagtttttt cgttggtaaa aggtaggtgg ggttcgattc gttttttggg 6060
cgtagatttc ggtcgttcgt ttcgttcggt gcgttttcgt tttgtttatt taagagtgtt 6120
ttttattttt cggggatttt agtttttttt tgggcgttcg cgtcgaaagt tttaggtttt 6180
ttttcgatgg tcgtttcgcg gagacgttcg ggtttgtttt atttgtagtt tttcggtcgc 6240
gtttgggttt cgcggtggag cgggacgcgg ttgt 6274

<210> 178
<211> 6274
<212> DNA
<213> Artificial Sequence

<220>
<223> chemically treated genomic DNA (Homo sapiens)

<400> 178

atagtcgcgt ttcgttttat cgcgaagttt aggcgcgatc gaagggttgt aggtggagta 60
gattcggacg ttttcgcgag gcggttatcg aaggagagtt tggggttttc ggcgcgggcg 120
tttaggaggg agttggggtt ttcgggaggt ggggggtatt tttggatagg taagacgagg 180
gcgtatcggg cgaggcgagc ggtcggggtt tgcgtttaag gggcgggtcg gattttattt 240
gttttttgtt aacgaggaaa ttaatcgtcg ttagtattat tttttttcgg gagtcgtagg 300
tggtggtaga cgagtttttt tagtttttag gtcgaggttg cgattttata ttggggaacg 360
aggatcgcgg ggatttggag aggggtcggg gttaggttgg cgggcgtcgg ttttagtttg 420
agatttgcgc ggagggcgga ggcggggagg tttttttaga aagtcgtttg tttcgcgggg 480
ttcgagggga ggttgtattt ttgggttttt tttttagttt ttttaaatag tgtagtttcg 540
gcgggtgggt agcgttcgtt tttcgggtgc gcgttaggat ttcgtcgcgt tttgggaaga 600
tagcgcgggg cgtacgtgcg ggatttcggt tttaggcgtt tattagtatt cggttttcgt 660
ttattgacgt cgattttgcg tagttgggcg gggaaagtga gggaagaaag ttgggagcgg 720
ttcggggcgt cggaggtttc ggcggtcgtt tttcgtttgg tttgtagttc ggtgttttac 780
gcgttttttt tttcgttcgt tttagatttt ttcggtcgaa gaacgaaacg tttcgttttt 840
ttcgcggttt cggtcggcgg cgtagttgat tttaaggaaa taggaaagga cgggcggggg 900
cgcgggcgtc gagtcggtgc gtcggtgcgc ggttgggcgg gttgcggggt tcggggcgcg 960
attcgggggc ggcgcgggag tcggcgagat acgtttttat tttttttttt ttttagagtt 1020
cgattcgtcg ggtagagcgt agttagtagc gttaggagcg ttgtttcggt cgtttcggag 1080
ggtcgtatcg ttgtttttcg aagagttcgt ttcggttttt tcgattaata ttggacggag 1140
ttagggggtc gtgcgcggtg gttgtggcgt tggcggcgag gcggggattc gggcggacgt 1200
cgacgaggtg gtttgtcgtt cggtttgggc ggcggcggtc gtcggggtta gttcgggatt 1260
tcggtcgttt cggtcgcgtc gggcgtttat atcgtgcggg gggcggaggt tgttgcggtc 1320
gcgttgcgtc gggggttgtt cggacgttta gttcgcgcgg gtcgaggcgc ggggaggtag 1380
ggcgggagga ggagggatta ggcggcggag gagggatcgg gaggagtaga ggaggaggag 1440
aatgcgagga ggagggagga gaattagtag cggggattta aggttagcgg tcgtcggagg 1500
cggtggatag cggtggtttt cgcgggattt agttttcggc gggagtgggg cggcgtatgg 1560
gaggggagcg ggtgttttga ggagttaatt ttcgagaggg gcgtttttag tattgttttt 1620
ttggattcgg ttttcgcgga tttgtaggtt tagtttatgt ttaggtcgag ttaaatttgt 1680
gttagggttt tttttttttt tttttttttt ttgttttata aagtaaattt gtattagttt 1740
tagatagatt ggtcgagttt tagagttagc gtcggataat ggtatcgacg gggaaattgt 1800
ttttttattc gggtttttat tttttttgta gtattacggg attttttcgg ggattagagg 1860
ttggtttggt agttgaggag gtcgtcgtgt tcgcgcggta ggggtgttag gcgttcgagg 1920
tggtgtttta aagttcgagg gttttttaga ttttgttaag tattttcgaa attatttgaa 1980
atgagggtat ttaatttcgg gtgtagagat aggttttttt aatgtttatt gtaattagtt 2040

atgggtattt tgaagttaat gtgtgaagta tattatttcg taaaagtgaa gtttttggaa 2100
taaaggtttg atataaatag tcgtggaggt gtttttagag aggttaagtg ttttattgtt 2160
tttgtagttt tcgtaggaga aatgttaggg aaattcgcgg aagttttggt taaaagaaat 2220
tgagtttttt tgggttgtag gaatttgagt tatatttttt tggtaagttt attttatttt 2280
tattgttttt ttttttgtag taaaaatgtt ttttaggttt atgttatagt tagatttttt 2340
ttttttttac gagatttatt aggattttta ggtttggttt aataattttg atttttcggt 2400
tttgttaaaa attataattt tgaagtaagt gaagttatag atttgttttt tgttgttttt 2460
gaaggggagt aattttatat tttttttttt tatttagtaa aatgtttgtg tttgggtttc 2520
gtattttgta gtttgaacgg tggggttttt tattagggta agcgtagata gatataggtt 2580
tgtttattgt tcgtataatt tggaattttt ttgtgaatta ggagaagtaa tttgtttttt 2640
tgttttaata aattagtata gttattgtgc gttgaagtta tgttttgaat gtagaggtta 2700
tgaaggtttt gtaggtatgt gtgtgttttt tgtatatttg ggttgagagt agttcgtaga 2760
aatatagaaa ataaaattag agaaaggtaa atgggaagta taatagtgga atataaaatg 2820
aagataggag tgagttttat aataaaagat ttgaatataa taaagttttg tgtatttatt 2880
taataagggt tgtaaatttg gttttaaatt atattttttg ttaagggaat gtgatttgtg 2940
tgtataagtt ataatataaa ttaatttaag ttataattga tttgtaagat tgtataggaa 3000
tttttttgag tgtttaaaaa gatagtatgt aagaatatta gtttttatta cgttaggatt 3060
atatgagata atagttttta tagtacggtt tgtatatttt ttaatgtgat ttgatagtat 3120
tataagaaat agagtttttt gtaaagaatt attagatata tatatagtat aatattggga 3180
tggattttag ggaatagaga agaatgggtt tgttgaatat ttttatggga tatttatgta 3240
aagtttgagt tttttttttg agtttttgat ataaaagttg atatttacgt tgattttgta 3300
gtttttggtt agtatttgga gtgtgttgat aattgtgtta tttggtaagt aaaaaattgt 3360
atggtcgagt aggtattaat gaagtttttt taagagagat aagtagtttg gtatagttag 3420
ggttttgtat agttagggtt ttagtttata gtttttgtgg agggtggttt tagattatgg 3480
ttcggataga ggttgggtgt gtgtgttatt attataggaa tgataatagt ggtttagttg 3540
ttgtgttaag tattggttag attaagttta tttgataatt tgtgagttgg ttaatatttg 3600
tatttgtttt ttatagtaat gtgatgttgt ggtcgggaga agtgattagg atattaaggt 3660
atatttgtta gtatgagagg ttaattttga atttaaggta gagtcgtgtt ttattttttt 3720
tgtttagtaa cgttttaaaa tttgagataa atggtgatgt taggatttaa aattttattt 3780
tataatgttt tggaaatttg ttagggtgag taatattaaa atttttgttt ggttaaaaaa 3840
attatagata aagtttttat gtatgttata ttagtaagta tgtattaaga ttttagtatt 3900
ttttttaata aattttataa gcgtttgtta attttaataa tatagatggt tttatttttt 3960
tgttagttgt atatgagaaa tttttttttt ttgatgagtg gttatttttt tagaagaagt 4020
ttttgagtat gttgaataag agtttagttt tgtttataag gaatgtggtt ttgaattttt 4080
taaaattaaa tgttttgtga aaggggagga gaaatacgtt atttggtagg gtgaagattt 4140
gaggttttta aatattttaa gttttatttt aggtaaaagg tatttgatta tttgtttttt 4200
ttggtttaga ggttaaagta aagattgatt aggaaaaaaa aatatagtgg tagattttag 4260
atttttaaaa atatgtttta taagttattg gagttaagta aaagtagaat aagttatgtt 4320
agagtggaaa gggttttttt gagaaagatt tttgtgtaag aataataagt attaatggag 4380
gttgtgaatt aaggattgat ataggttttt tttaatttta aagtttttta agaaaatgta 4440
tttttaaaat aggtttttat ttaaatatgt gtttatttga tagttttttt aaggatatat 4500
gttaaggaga tataagaatt tggtaatagt tttgttttta gggaggagaa ttaggtagtt 4560
cggggttagg ggtttagagg gagatttgtt ttttaatttg tttttttgta ttattgggat 4620
tttgtattag gagtatgttt tatttatttt tgtaaggtaa gtgtggtttt tgttgaggtt 4680
tagggatatt tttgggggta ggtttttatg gtttttgaga ttgtttattt tttttttttg 4740
aaggaagtta tttgttttga gttttgagtt gagatttatt ttttgtagaa tgtggaagaa 4800
gtagtttttt taaggggtaa tgtgttgata gaaaattgtt gaagtatttt ttttttgtta 4860
ttagtatgtt ttattatttt tagagttgag ggttatatgt tattatagga ggtattattg 4920
gcgttttatt ttagtaataa gtagaaagtt gagtaaatcg aaaaattaat attttttttt 4980
agttttttta gagaattgag gttattgggt aaattattgt tttttaaatt ggagagatag 5040
gtaaatatag aagtataatt tttagaggaa gaaattagta gatagaaatt tttaaggatt 5100
tagtgtcggt tgggaaagtt tgttttgtaa ttgagaaatt gcgggaggtt tagtgtgggt 5160
agttttgtaa attaagaatt ttagtagagt ttagttatgg tgtagggtag tatgagatat 5220
ttgagagtgt tattttttgg agttttgtta ggtttttatt acgaatatta gggggaaaat 5280
tttattatgt ttttggtagt ggaaggagaa gaggaattat tttgaaatag gttggaatat 5340
tttattatta atagaaggtt tgtttttagg agaaattatt ttattagagg tttaatttgg 5400
tgttttatta gagggtgatt tatttagagt aagggaaata tataatttta ttttttttta 5460
atagttttgt attatttaag gggttgggga agttgagaag taagtatgaa gtttatagtt 5520
tagggatata ggtttattgg aagattgtga tatgcggtag gattgtgtaa tatttttttt 5580
ttttatagtt tgttattacg ttattagaag tttttttatt atagtttttt ttatttagta 5640
tattattttt atattgtaag gtatattaaa aggtaaagac gtagtttgaa gagattgtat 5700
aagtattaga attagaatga gatatgatac gaacgttata attaattgtt gtggtttgaa 5760
tgtgttttcg aaaagtttat atattggaaa tttaattcgt tgtttttatg aatgaattta 5820

tatggttttt atggcggtgg gttaattttt gtgggagtgg ttatgttata agagtaagtt 5880
ttttttatac gtgatgtttt tttttatgtt atgatgaagt aagaagatat ttattagatg 5940
ttagtattaa gtttttggat tttttagttt ttaaattagt ttaaattagt tttttttgtt 6000
tataaattat ttagtttgtg gtattttgtt ataattatag taattggatt aagatattat 6060
tatatcggag tattaattat aaaaaaatta tgattaatag gttaggagtt ttaatggaaa 6120
aaataggtaa tatgtaagat tagatggata atgtaagtag agttatggaa tttttttttt 6180
ttttgagata gggtttggtt ttgttgttta atttagagtg tagtggcgtg attttggttt 6240
attgtaattt taatttttta ggtttaggtg attt 6274

<210> 179
<211> 3002
<212> DNA
<213> Artificial Sequence

<220>
<223> chemically treated genomic DNA (Homo sapiens)

<400> 179

atatttaaat tgtatatttg gtttgtaata tattatatat ttaaaagaat tattattttt 60
ttgaatatta ttaatattaa taatagtatt atagtgatta agagtggatt taaataaagg 120
ttggtttgaa atttaggttt gttatttatt agtaagtttt taaaaatttt gagtttttag 180
tttttttata tgtgaaatgg agaaaataaa tttgtaaaat taattaaatt tgtttattta 240
tatttttttt tttagtgtat attaattggt atttttcgtt aggttagaat gtgtttttaa 300
ttatgtttta aattcgtttt gtgttaattt tattgttaga atttttttta ttttgagaat 360
tagaaaagga aatattatgt ttggtaatgt ttatattttt taaaataaat ttgtaggaaa 420
gaatatttag taagtgatga gagtagtaac gattgttttt attattttaa atttataata 480
ttattttttt tagagttttt taagtattgt agataatttt ttatttatta aaaaataaat 540
tgtaattata agtatttagg gttgatattg tttttgaatt agatagtgtt tatattagtt 600
gtataagatt aatttaaagt agaggatgaa attttttttt tgaatttttt ttagaacgta 660
atttagtgaa tatattaaaa ttaaattttt tttgaatggg agtaattttt acggattaat 720
ttgtaatttt ttagattata tttaaggtaa tgtagaggtt gttgtattat aggttttgtg 780
attagagatt attggatttg tttttggaaa agttatttat gattttttgg gttttggttt 840
ttttattttt tatatcggtt taataatgat tatcgtagag ttgttatgga agttaaatga 900
atttatgtat tataagtgat taatataatg attgatattt agtgatttat taataaatta 960
aaatatttat tattaatatg atagagaagg tgtcgttaaa atagataata ggtttttgga 1020
agaggtgatt aaatggatgt aaaatttatg gattgtttat ttcgtttatt tttgttgtgt 1080
tttttggttg tggtatatat acgtgtgggt ataaaatcgt aaattttatg tagtcgcgta 1140
gtgtatgcgt agaaggttta gatacgaaat gttattttag taatgtgttt agagaagttt 1200
tgacgtcgtt ttggaagtaa gtcgttgttg tttgattttt gggcgtttgg gacggatgtt 1260
tatatttgta tttagtagta ttggaagggg tttaggtttt tcgtagtata gtttattttt 1320
agatcgttta gttttttata atatatattt ttacggaaaa gggtattttt tttcgttaga 1380
aaaagcgttt tagtttggtt tgggttggtt tttattttac gttgttgtaa gtaggcgaag 1440
ttttttttgt tttttttttt ggggtaagtg gaaaggagtt cggtaggggg ttcgtagtgg 1500
tttgtatagg ggaattgggt agcgagagag ttttaggtaa tttcgggggt tgttttatag 1560
aagtaggtgg ggatcgatag tggtttttcg gtttagggag gagagcgcgg tcgcgggttt 1620
ttttttttag tttggaggtt gtagtcgttc gagtcggttc gggtgggggc ggggtggggg 1680
cggcgcggag ggtacggaga ttacggcggc gttattcggg atatttaggg tttcgaggtt 1740
ttgggcggtt tttacgcgag atcgtaaatt atgataatag gtagttattc gaggttaaat 1800
aaaaacggag tgggtttttc gcgcgtcgtc gtttttcgcg tttttggcgg tttttttcga 1860
ggttttcggc ggttttacga gttcgtagta gtcggtggcg acgtcgtttt cgttttattt 1920
ttttgcgtaa gtgcgaggtt gtcggtagcg cggcgtacgt ttcggtcgtt ttcggttttc 1980
gcgtaaaatt tttattttgt ttacgtgaag ttgtcgttgt tttagagagg gggaaagagt 2040
tgcgggaaaa gtcggggagt gacgattgcg gcggttgggc gcgttttttt attttttttt 2100
tttttttttt tttttttgtc gtagttcgga gttttggttt tttttttttt tttttttttt 2160
tcggagtcgg tttttttttt cgtttcgttt ttttttcgtt tgtgtacgtt atttgttgtg 2220
gggtggtcga aggggatgtt ttgtttttat tagaggtata gcgcgaaggg gaaatttcga 2280
tattggaagg aacgagaata aatatttaat tacggacgta ttgaatcgcg gttgggatag 2340
atatttcggg aattcgaggc ggatcgggcg acgaggtgag tgattttttt ttttaatttt 2400
cgttttaggg ttttcggggg agtttgagtt gagagaattt ttaaattttt cgggaaagtg 2460
cgcgaggttt cgtcggggac gtcgagcgtt gggtattgag gacgcgtagt tggacggtgc 2520
gtgggcgttt gcgttttcgg ggggcgtttg gaggtcgggt gttttacgtt tgagggttcg 2580
ggtcgttcgg atcgtagcgg tgttttttgt tttagaagac gtttttaagt tttaagggtt 2640

tttttcgagt ttgtttgttt ttttcggggt cggcgcggag tttgcgcgta acggagttta 2700
tttagtagtt tagcgcgcgg tttttatttg tatttcgttt ttatttggta gaggcgcgag 2760
tatcggggtt ttttttatat tttttttatg acgtgtatta ttttttgatg attttttaga 2820
tggtttaggc gcgaggatgt tgatttagag tttttcggag ggttataggc gtttgggttt 2880
tttcggtgtc gggtgcgtgt gtattttaaa ggttcgcgtt ttaattttta ggtattgatc 2940
gggtttttta attgcggcga ttttatttta atagttttta tgtggcgtgg attgaatgtt 3000
-- 3002

<210> 180
<211> 3002
<212> DNA
<213> Artificial Sequence

<220>
<223> chemically treated genomic DNA (Homo sapiens)

<400> 180

--gatattta gtttacgtta tataaaaatt attaaagtgg gatcgtcgta gttgaaaagt 60
tcgattagtg tttggagatt agaacgcgag tttttaaagt atatacgtat tcggtatcgg 120
gaaagtttag gcgtttgtga tttttcgaag gattttgggt tagtattttc gcgtttggat 180
tatttagggg gttattagaa agtaatatac gttataagaa agatgtgggg gagatttcga 240
tgttcgcgtt tttgttaggt ggaggcgggg tgtaggtaga agtcgcgcgt tggattgttg 300
gatgaatttc gttacgcgta ggtttcgcgt cgatttcgga agggataggt aggttcggaa 360
gggatttttg gggtttgggg acgtttttta gggtagagag tatcgttgcg gttcgagcgg 420
ttcgggtttt taggcgtggg gtattcggtt tttaagcgtt tttcggggac gtaggcgttt 480
acgtatcgtt tagttgcgcg tttttagtat ttagcgttcg gcgttttcgg cggagtttcg 540
cgtatttttt cggaaagttt gggggttttt ttaatttagg ttttttcggg agtttggggg 600
cgggggttgg aagaaggggt tatttatttc gtcgttcggt tcgtttcggg ttttcgaagt 660
gtttgtttta gtcgcggttt agtgcgttcg taattaagta tttattttcg ttttttttag 720
tgtcgaagtt tttttttcgc gttgtgtttt tggtgaaaat aggatatttt tttcggttat 780
tttataataa atagcgtata taagcggggg agaagcgggg cggagggaga agtcggtttc 840
gagggggagg aggaaaggag aggagttaaa atttcggatt gcgatagggg ggaaaggaga 900
agaaaagaaa atgagagagc gcgtttagtc gtcgtagtcg ttatttttcg gtttttttcg 960
tagttttttt ttttttttta aggtagcgat aattttacgt ggataggatg gaagttttgc 1020
gcggaagtcg ggaacggtcg gagcgtgcgt cgcgttgtcg gtagtttcgt atttgcgtag 1080
ggaggtgggg cgggggcgac gtcgttatcg gttattgcgg gttcgtgagg tcgtcggggg 1140
tttcggggga ggtcgttagg gacgcggggg gcggcggcgc gcgggggatt tatttcgttt 1200
ttatttgatt tcgggtgatt gtttattgtt atggtttgcg atttcgcgtg gggatcgttt 1260
agggtttcgg ggttttggat gtttcgggtg gcgtcgtcgt aattttcgtg tttttcgcgt 1320
cgtttttatt tcgtttttat tcgggtcgat tcgagcggtt gtagttttta ggttgagggg 1380
agggattcgc gatcgcgttt ttttttttgg gtcggagagt tattgtcgat ttttatttgt 1440
ttttgtgggg tagttttcgg aattgtttgg aattttttcg ttatttagtt tttttgtgta 1500
ggttattgcg ggttttttgt cggatttttt tttatttatt ttaagggagg agatagaagg 1560
gatttcgttt atttgtaata acgtgaaata aaaattaatt tagattagat tggggcgttt 1620
tttttgacgg gaggaaatat tttttttcgt ggagatatat gttatgaagg attaagcggt 1680
ttggggatag gttgtgttgc gaagggtttg ggtttttttt agtgttgttg ggtgtaggta 1740
taggtattcg ttttagacgt ttaaaggtta ggtagtaacg atttattttt aaggcggcgt 1800
tagagttttt ttaggtatat tgttgaaatg atatttcgtg tttaagtttt ttgcgtatgt 1860
attacgcgat tatataggat ttacgatttt atatttatac gtgtgtatgt tataattagg 1920
ggatatagta aaggtagacg gaataaataa tttataaatt ttgtatttat ttaattattt 1980
tttttaaaaa tttattgttt attttagcgg tatttttttt gttatattga taataaatgt 2040
tttaatttat tgataaatta ttgagtgtta attattgtat tggttattta tggtatatga 2100
gtttatttaa tttttatgat aattttacgg tggttattat taagtcggta taaaagatga 2160
ggaaattaaa gtttaaagaa ttatgagtga tttttttaaa gataaattta gtggttttta 2220
attataaagt ttatgatata ataattttta tattatttta ggtgtggttt aagagattat 2280
agattaattc gtagaaatta tttttattta aagaaagttt agttttaata tatttattaa 2340
gttacgtttt gaaaaaggtt tagaaaaaag attttatttt ttattttagg ttggttttat 2400
gtaattgata tgagtattgt ttaatttaaa agtagtatta attttgaata tttatggtta 2460
taatttattt tttaatgagt ggggaattat ttataatgtt taagaggttt tagaaaaggt 2520
ggtgttgtaa atttaaaatg ataaaggtag tcgttgttgt ttttattatt tattgggtgt 2580
ttttttttgt agatttattt tggagggtgt aggtattgtt aggtataatg tttttttttt 2640
tggtttttaa ggtagaaagg gttttggtag tggggttggt ataaagcgga tttggagtat 2700

ggttgagagt atattttggt ttaacgagga atgttagtta atatatattg gagagaaaaa 2760
tatgaatgga tagatttaat taattttgta gatttatttt ttttatttta tatgtgagaa 2820
aattaagggt ttagaatttt tagaagtttg ttaataaatg gtagatttga gttttaagtt 2880
agtttttatt taagtttatt tttagttatt gtgatgttat tattagtatt aatggtattt 2940
agaaaaatag taattttttt aaatatgtaa tatattataa gttaaatatg taatttaaat 3000
gt 3002

<210> 181
<211> 5649
<212> DNA
<213> Artificial Sequence

<220>
<223> chemically treated genomic DNA (Homo sapiens)

<400> 181

cggtggttta tatttgtaat tttagtattt tgggaggttg aggtaggcgg attataaggt 60
taggagatcg agattatttt ggttaatata gtgaaatttc gtttttatta aaaatataaa 120
aaattagtcg ggtgtggtgg cgggcgtttg tagttttagt tattttggag gttgaggaag 180
gagaatggcg tgaattcggg aggtggagtt tgtagtgagt taagatcgtg ttattgtatt 240
ttagtttggg cgatagagtt agatttattt ttaaaaaaaa aaaaagaaaa aagaaaatat 300
tttaagtttt gaggattata tagtttttgt tgtaattatt tatttgggtt attgtagtat 360
aaaaataaat atagataata tatatatgag tgagtatggt tggggtttaa taaaatttta 420
tttatataag taggtgatga attacggttt atagtgtttt aatttttgat ttagatggtg 480
aattattatg agttttcgtt gtttttttcg gtagaaatat gggagaaatt atagagagaa 540
atagagattt taggaaaata gtaaaattaa ataaataaag tgagacgtcg ttttttttgg 600
aggttgtagg ttgtgttgaa tttgtgtgtg tttgggtgta tagggatgtg agtagatgcg 660
cgtggagatg tgtgtgtggg tggagattga gcgtggttgg aggtggttgt ggtaggatgt 720
tagaaggaag gagtttgtat ttagtttttt tattagtatt ttttggagtt gttgagggga 780
tgttattgtt gaatattgat tttggtgtag gttaaatcga agttagtttt tgttattttt 840
tgttgggtga ggtattgggg agaatagtgg gattgaatgg ggcgggtttt ttttttttta 900
tatttttttt ggaaatattt agtattagta tattagaata tggggagtta ttttttttag 960
cgttgttttt taaggttcgg aaggggaaat tttgatttta agttttgttt gaagagcgat 1020
agtaaagggg agaggttggt tgtggtttgg tggtgtttat tttttttttt tgttttgaaa 1080
ttataggttg gtatattatt cgagagaatg attagtgtta ttaaggaaag ggtataagtt 1140
tattggtttt aggggagata ttttagggga taggaatatt ttttgacgtt gaatgagtgt 1200
agtatataga cggagaatat atataagaga aattatgagg atatgagaga aggtattggt 1260
aataggaagt tataaaaagt aataaaaaga aagagtagat ataaaaataa taatcgaaga 1320
aagttaataa tagatgggat gaattatacg aggtatgcga tttatggatg aagaggtgag 1380
tgatgagatt agttggggaa ttttttttta ggaaagataa attagaagaa atataaatga 1440
tatcgaagtg gttaggcgta gtggtttacg tttgtaattt tagtattttg ggagttcgag 1500
gtaggtggat tatttgaggt taggagtttg agattagttt ggttaatatg atgaaatttt 1560
gtttttatta aaaatataaa aattagttag gtgtggtggt aggtatttgt gattttagtt 1620
atttaggagg ttgaggtagg agaattattt gaattaggaa gtagagattg tagtgagttg 1680
agatggtgtt agtgtatttt agtttgggta gtagagtgat agtttgttta aaaaaaaaaa 1740
aaggtagtaa agtgatagga ggatagatta gaagggttat tttttagata gtgtaaggaa 1800
gaaagaagaa gaagaagagg aggaggagga ggaagaggaa gaagacggag aagagggtat 1860
tttagaagaa ataatggaga tatatttttt aaaattgaag aaagatgatt aattttagat 1920
cgaaggttaa gaggtaaatt agagagatag tttggtaagt taggaagagt attaaagtta 1980
gataattaga gtgaaattaa agaaaattta aggtgataaa aatttaaaaa gtttttttgt 2040
gattttagtt tattggtgaa aatttaaaaa aaaaaaaaaa aaaaaaagga tagtttgggt 2100
aatatggtga aattttattt ttataaaaaa tataaaaatt agttgggtgt ggtggtatat 2160
gtttgtagtt ttagttattc gagaggttaa gtaaggtagg aggattattt ggggttaaga 2220
gttttagatt agttgcggta atatatatgt tgtgggaagt taggtatttc gaacggaggg 2280
attggttgaa gttatggtag aagaatataa attgtgaaga ttttatggat atttattatt 2340
tttttaatta atatttttgt gattttttat gtttgttttt aattttttaa ttttgttatt 2400
ttcgtaaatt gaggatgtat gtcgttttag gattttgtga tgattgcgtt aattgtataa 2460
attgtttgta gaggataatt gttgtgtgtt tgagtaatgt gaaatttggg tattttaaga 2520
ataggataat agtaattttt agggaataag ggagataatt ttaaagtttg gttgtttgtg 2580
ggttgggtag gatagagtta tatttttttt attatcgtaa ataggtaaga gaaatatcgt 2640
tgaatttttt ttttagtaag gaatattaat aattaatagt tttgagaaaa gaatgtattt 2700
ttagggtcgg gttattgaaa tggttttttt gagagtgttt gtttttattt agaagtagaa 2760

agggatgaaa tacgttttag ttttttgtag tatttttaga tttgatagga ttaggaaatt 2820
ttagtttggt aaattttagt tagatcggtt ttttgttttt gaattttgat agtgcgtgtt 2880
agtgggattt ggaagtttat tagtgatttt agtttcgtcg tgattttttg ttttgtgatt 2940
ttttgtcgtt tttgaagtat gtgatttttg tgatttatat tttgttcgtg tatttttttt 3000
tttttgaaaa ttgttaataa aaattcgttg gttttacggt ttagggggta ttacggaatt 3060
tgcggatatg tgatgttttt tttggatatt cggttttaaa atgttttttt tttgtatttt 3120
ttttttttat tttttaggtt agtcgatatt tagggaaaat aggaaaggat ttacgtgaaa 3180
tattgggggt tgaatttttt tcgataataa gttttttaaa aaagattttt aaatgaaatt 3240
tttttgtttt ttaattttta tttttgtttt gggggtatat gtgaaggttt gttatataag 3300
taaattcgtg ttttcggtgt ttgttgtata gaatatttta ttatttaggt attatgtcga 3360
gtatttaata gttttttttt ttgttttttt tttttttttt attttgtatt ttggagttaa 3420
ttatagtgtt tgttgttttt ttgtttgtgt tataagtttt tattatttag tttttattta 3480
taagtgagaa tatttagtat ttggattttt gtttttgtat tagtttgtta aggataatag 3540
tttttagttt tatttatgtt ttataaaaga tatgatttag ttttttttaa tggttgtatt 3600
aaatgaagtt ttaaagatat aatataaata ttaatttttt ttttattata aaaatttttt 3660
gttgaatttg attatattta aattaacgag ttttgtttta tgaaagattt tttggataaa 3720
tttgatagtt gatggaatag gagaagttgt ttgttatgtt taaagttaat aagagattaa 3780
tatttagaat aaatggagat ttgtaaatta atagaaagta ggtagtaaag ttaaagaaaa 3840
tagtttaagg tatagttatt aaaaggaacg tgattatgtt ttttgtaggg atatgggtgg 3900
agttggaagt cgttagtttt agtaaattta tataggaata gaaaattagc gagatcgtat 3960
ggttttattt ataagtggga gttgaataat gagaatatat ggttatatgg cggcgattaa 4020
tatatattgg tgtttgttga gcggggtgtt ggggagggag agtattagga agaatagtta 4080
agggatattg ggtttaatat ttgggtgatg ggatgatttg tatagtaaat tattatggcg 4140
tatatattta tgtaataaat ttgtatattt tttatatgta ttttagaatt ttaaataaaa 4200
gttggacggt taggcgtggt ggtttacgtt tgtaatttta gtatttttgg gaagtcgagg 4260
cgtgtagatt atttaaggtt aggagttcga gattagttcg gttaatatgg tgaaatttcg 4320
tttttattaa aaatataaaa attagttaga tgtggtacgt tatttataat tttatttatt 4380
cgggaggttg aagtagaatt gtttgaattc gagagcggag gttgtagtga gtcgtcgaga 4440
tcgcgttatt gtattttagt ttgggttata gcgtgagatt acgttataaa ataaaataaa 4500
ataatataaa ataaaataaa ataaaataaa ataaaataaa ataaaataaa ataaaataaa 4560
aaaataaaat aaaataaaat aaaataaaat aaagtaattt tttttttttt aagcggtttt 4620
tatttttttt ttttgttttg tgaagcgggt gtgtaagttt cgggatcgta gcggttttag 4680
ggaatttttt ttcgcgatgt ttcggcgcgt tagttcgttg cgtatatttc gttgcggttt 4740
tttttttgtt gtttgtttat tttttaggtt tcgttgggat ttgggaaaga gggaaaggtt 4800
ttttcggtta gttgcgcggc gatttcgggg attttagggc gtttttttgc ggtcgacgtt 4860
cggggtgtag cggtcgtcgg ggttggggtc ggcgggagtt cgcgggattt tttagaagag 4920
cggtcggcgt cgtgatttag tattggggcg gagcggggcg ggattatttt tataaggttc 4980
ggaggtcgcg aggtttcgtt ggagtttcgt cgtcgtagtt ttcgttatta gtgagtacgc 5040
gcggttcgcg ttttcgggga tggggtttag agtttttagt atggggttaa ttcgtagtat 5100
taggttcggg ttttcggtag gtttttcgtt tatttcgaga ttcgggacgg gggtttaggg 5160
gatttaggac gtttttagtg tcgttagcgg tttttagggg gttcggagcg tttcggggag 5220
ggatgggatt tcgggggcgg ggaggggggg tagattgcgt ttatcgcgtt ttggtatttt 5280
ttttcgggtt ttagtaaatt tttttttgtt cgttgtagtg tcgttttata tcgtggttta 5340
ttttttagtt cgaggtagga gtatgtgttt ggtagggaag ggaggtaggg gttggggttg 5400
tagtttatag tttttcgttt attcggagag attcgaattt ttttattttt tcgtcgtgtg 5460
gtttttattt cgggtttttt ttttgttttt cgtttttttc gttatgtttg tttttcgttt 5520
tagtgttgtg tgaaattttc ggaggaattt gtttttttgt tttttttttg tatttttgat 5580
tttttttcgg gttgttgcga ggcggagtcg gttcggtttt tatatttcgt attttttttt 5640
tttcgtagt 5649

<210> 182
<211> 5649
<212> DNA
<213> Artificial Sequence

<220>
<223> chemically treated genomic DNA (Homo sapiens)

<400> 182
   gttgcgggga gggagaagta cgagatgtgg ggatcgggtc gatttcgttt cgtagtaatt 60
   cggggagggg ttaggagtgt agggagggaa tagggaaata ggttttttcg aagattttat 120
   ataatattgg ggcggggagt aggtatggcg ggagaggcgg ggaataggaa ggaggttcgg 180
   ggtaaaagtt atacgacgga gggataaggg ggttcggatt ttttcgggtg ggcgaggggt 240
   tgtgggttgt agttttagtt tttgtttttt ttttttgtta gatatatgtt tttatttcga 300
   attgggaaat agattacggt gtagggcggt attgtagcga ataaagaaaa gtttgttgga 360
   gttcggggga ggatgttaag gcgcggtgag cgtagtttgt tttttttttt cgttttcggg 420
   gttttatttt ttttcgaggc gtttcgggtt ttttgaaagt cgttaacggt attggggacg 480
   ttttgggttt tttaggtttt cgtttcgggt ttcgaggtgg gcgaggagtt tgtcgggagt 540
   tcgggtttga tgttgcgggt tggttttatg ttgggagttt tgagttttat tttcggggac 600
   gcgggtcgcg cgtatttatt ggtggcgaag attgcggcgg cgaaatttta gcgaagtttc 660
   gcggttttcg agttttataa gggtggtttc gtttcgtttc gttttagtgt tgagttacgg 720
   cgtcggtcgt ttttttggag ggtttcgcgg atttcgtcgg gtttagtttt cggcggtcgt 780
   tgtatttcgg gcgtcggtcg tagaggggcg ttttggagtt ttcggagtcg tcgcgtagtt 840
   ggtcggggaa gttttttttt tttttttagg ttttagcggg gtttagggag taaatagata 900
   gtaggaagag gatcgtagcg aagtgtgcgt agcgaattgg cgcgtcggga tatcgcgggg 960
   ggaaattttt taagatcgtt gcgatttcgg agtttgtata ttcgttttat agggtagggg 1020
   agaggggtgg aggtcgttta gaggaaagga aattgtttta ttttatttta ttttatttta 1080
   ttttattttt ttattttatt ttattttatt ttattttatt ttattttatt ttattttatt 1140
   ttgtgttatt ttattttatt ttatgacgta gttttacgtt gtggtttagg ttggagtgta 1200
   gtggcgcgat ttcggcggtt tattgtaatt ttcgttttcg ggtttaagta attttgtttt 1260
   agtttttcga gtaggtggaa ttataggtgg cgtgttatat ttggttgatt tttgtatttt 1320
   tagtagagac ggggttttat tatgttggtc gggttggttt cgaatttttg attttaggtg 1380
   atttgtacgt ttcggttttt taagagtgtt gggattatag gcgtgagtta ttacgtttgg 1440
   tcgtttaatt tttatttgaa gttttggggt atatgtagag gatgtgtagg tttgttatat 1500
   aggtgtgtgc gttatgatgg tttgttgtat agattatttt attatttagg tattaagttt 1560
   agtatttttt agttattttt tttggtattt ttttttttta gtatttcgtt taataggtat 1620
   tagtgtgtgt tgatcgtcgt tatgtgatta tgtgttttta ttgtttagtt tttatttata 1680
   agtgagatta tgcggtttcg ttggtttttt gtttttgtgt gagtttgttg aggttaacgg 1740
   tttttagttt tatttatgtt tttgtaaagg atatgattac gtttttttta gtggttgtgt 1800
   tttaggttat tttttttggt tttgttgttt attttttgtt gatttgtaga tttttattta 1860
   ttttagatat tgattttttg ttggttttag atatgataga tagttttttt tattttatta 1920
   attgttaagt ttgtttaagg agttttttat gaaataaaat tcgttaattt aagtgtaatt 1980
   aaatttagta agggattttt gtggtgggga agaggttggt gtttatgttg tatttttaaa 2040
   attttattta atgtagttat taaaaagaat tagattatgt tttttgtggg atatggatgg 2100
   agttagaggt tattattttt agtaaattaa tgtaggaata gaaatttaaa tattggatgt 2160
   ttttatttgt aagtgggagt taaatgatga gaatttataa tataaataag gaaataatag 2220
   atattgtggt tgattttagg gtgtaggatg ggaggaagga gaggagtaga aaagagaatt 2280
   attgggtatt cggtataata tttgggtgat gaaatatttt gtataataaa tatcgaagat 2340
   acgagtttat ttatgtaata aatttttata tgtattttta aaatagaaat aaaagttaaa 2400
   aaataaagaa attttattta aaagtttttt ttaagagatt tattatcggg ggaaatttag 2460
   tttttaatat tttacgtggg ttttttttta ttttttttaa gtgtcggttg gtttgagaaa 2520
   taaagggaaa gagtataaaa gagagatatt ttaaagtcgg gtgtttaggg gagatattat 2580
   atgttcgtag gtttcgtgat gttttttgag tcgtaaaatt agcgagtttt tattagtaat 2640
   ttttaaagag gagggagtgt acgaataggg tgtgggttat agagattata tattttaagg 2700
   gcgataaaag attatagggt agaaggttac ggcgaaatta gaattattaa tgaattttta 2760
   ggttttattg gtacgtattg ttagggttta agagtagaga atcggtttga ttagaatttg 2820
   ttaggttgga attttttaat tttattaagt ttgggggtgt tgtaggagat tagggcgtgt 2880
   tttatttttt tttatttttg aataaaggta ggtattttta gggaggttat tttagtggtt 2940
   cggttttggg aatgtatttt ttttttaggg ttgttaatta ttaatatttt ttattgggga 3000
   aagaatttag cgatattttt tttatttgtt tgcggtaata agagaagtat ggttttgttt 3060
   tgtttagttt ataggtagtt agattttaag gttatttttt ttgttttttg aaaattgttg 3120
   ttattttgtt tttaagatgt ttagatttta tattgtttaa atatataata attgtttttt 3180
   ataaataatt tgtgtagtta acgtagttat tatagggttt taaggcgata tatattttta 3240
   gtttacgaag atgataggat taagagatta aagataggta taggaaatta taagagtatt 3300
   gattggggaa gtgataaatg tttatgaaat ttttataatt tatgtttttt tgttatggtt 3360
   ttagttagtt ttttcgttcg ggtgtttgga ttttttataa tatgtatgtt gtcgtagttg 3420
   gtttggaatt tttggtttta agtgattttt ttgttttatt tagtttttcg aatagttggg 3480
   attataggta tgtgttatta tatttagtta atttttgtat tttttgtaga gatggggttt 3540
   tattatgttg tttaggttgt tttttttttt tttttttttt ttttaaattt ttattaatgg 3600
   attgaggtta taaggaagtt ttttaaattt ttgttatttt gggttttttt taattttatt 3660
   ttaattgttt agttttggtg ttttttttgg tttgttaagt tgtttttttg gtttgttttt 3720
   tggttttcgg tttgaggttg gttatttttt tttagttttg agaaatgtat ttttattgtt 3780
   ttttttgaga tgtttttttt ttcgtttttt tttttttttt tttttttttt tttttttttt 3840
   tttttttttt tttttgtatt atttggaaaa tggttttttt aatttatttt tttattattt 3900
   tattgttttt tttttttttt agataggttg ttattttatt gtttaggttg gagtgtattg 3960
   gtattatttt agtttattgt aatttttgtt ttttggttta agtgattttt ttgttttagt 4020
   tttttgagta gttgggatta taggtgtttg ttattatatt tggttaattt ttgtattttt 4080
   agtagagata gggttttatt atgttggtta ggttggtttt aaatttttga ttttaagtga 4140
   tttatttgtt tcgggttttt aaagtgttgg gattataggc gtgagttatt gcgtttggtt 4200
   atttcgatgt tatttgtgtt ttttttggtt tgtttttttt gggagagagt tttttagttg 4260
   attttattat ttattttttt atttatgagt cgtatatttc gtatgattta ttttatttgt 4320
   tgttggtttt tttcgattat tgtttttata tttatttttt ttttttgttg ttttttgtaa 4380
   ttttttattg ttagtgtttt tttttatgtt tttatgattt tttttgtgtg tatttttcgt 4440
   ttgtgtgttg tatttattta gcgttagggg atgtttttgt tttttgagat gttttttttg 4500
   gaattagtga gtttgtgttt tttttttggt ggtattggtt attttttcgg gtagtatgtt 4560
   agtttgtggt tttaaagtaa aggaaggaga taagtattat taaattatag ttaatttttt 4620
   ttttttgtta tcgtttttta aatagagttt gaggttagag tttttttttt cgaattttgg 4680
   gaagtagcgt tggggaaggt ggttttttat gttttaatgt gttggtgttg ggtgttttta 4740
   ggggaagtgt gggagggaga aagttcgttt tatttagttt tattgttttt tttagtgttt 4800
   tatttagtag agggtagtag aggttaattt cggtttagtt tgtattaggg ttagtgttta 4860
   gtagtgatat ttttttaata attttagaga gtattaataa aggaattgaa tgtaaatttt 4920
   ttttttttgg tattttatta tagttatttt tagttacgtt tagtttttat ttatatatat 4980
   atttttacgc gtatttattt atatttttgt atatttagat atatataggt ttaatatagt 5040
   ttgtagtttt taggaggggc gacgttttat tttatttgtt taattttatt gtttttttga 5100
   aatttttgtt tttttttgtg gtttttttta tatttttatc ggagagggta acgaaagttt 5160
   atgatagttt attatttaaa ttaaggattg ggatattatg gatcgtggtt tattatttgt 5220
   ttgtgtaaat aaagttttat tgggttttag ttatgtttat ttatgtgtgt gttatttatg 5280
   tttgtttttg tgttatagtg gtttaaatga gtgattgtaa tagagattat gtggttttta 5340
   aagtttaaga tatttttttt tttttttttt tttttttgag atggagtttg gttttgtcgt 5400
   ttaggttgga gtgtagtggt acgattttgg tttattgtaa gttttatttt tcgggtttac 5460
   gttatttttt ttttttagtt tttagagtag ttgggattat aggcgttcgt tattatattc 5520
   ggttaatttt ttgtattttt agtagagacg gggttttatt gtgttagtta ggatggtttc 5580
   gattttttga ttttgtgatt cgtttgtttt agttttttaa agtgttggga ttataggtgt 5640
   gagttatcg 5649

<210> 183
<211> 12781
<212> DNA
<213> Artificial Sequence

<220>
<223> chemically treated genomic DNA (Homo sapiens)

<400> 183

tattatgttg ttagttggtt ttgaattttt ggttttaagt tatttttttg ttttggtttt 60
ttaaagtgtt gggattgtaa gtgtgagtta ttgtttttgg ttagttggtg atttatttgt 120
ataattgttt aatttattga atacgtatgg cgtgttaagt attgggttga gggttttata 180
atgttttttt atttaatgtt tagtataatt tatgaagaag gtagtgttaa tattattttt 240
gttttataga tgtagaaatt gaggtatagg ttaaataatt tgtttaataa gttcgtgtag 300
tttagtaagt agtttagttg ggatgtgaat ataaattttg aatatagagt ttttaattag 360
taggttagag gtttttaaat atttagtata tttattattt tagtattttt ttgttatatt 420
ttttaggtta aaataatttt attgattgtt atgtaattta ttaagtgtag ttatttgaaa 480
aaaaaatata tataaaagaa aaatattttt atttagtttt taaaataatt tatatatagt 540
tatggaatgt atgtgttggt tgggtattgt agattaaaga tagtttaggg tcgggtacgg 600
tggtttaggt ttgtaatttt agtattttgg gaggttaagt agagagaagg gtttgagttt 660
aagagttgga gatcggtttg ggtaatatag taagatttag tttttataga aaataaaatt 720
attagggtgt gaggacgtat atttgtagtt ttagttgttt gggaggttga ggttggagga 780
ttagtttggg taatagagtg agattttgtt ttagaaaaaa aaaaaaaaaa agatagattt 840
ttatttagat atgtatgtag gagtttatag aaaaaaaaag tgagtttagg aggttgttat 900
ttggtattta tataattttt ttttttttga atttcgaaat ttattttata tatattattt 960
aataggggaa gaggagggag aaaaagtttt ttatgggaag aataaatagg tttttggggg 1020
aataaaaggg agataagaat gtttgttttt gtaggtgtaa gtggtttttg tttttttttt 1080
tggttattaa aattttttta gagaggagat ttacggtagt tttattttta gaaatttttg 1140
ttgttagtcg tataagggaa gttttgaaac ggtatttttt tgtatttgtt ggtttttaaa 1200
tgtttttagt tttaagtaat atttatgtta atttgggggg tttgagtgtt tttatataat 1260
atatgtgttt ttttgttttt taatgaagtt tgtgggaggt atttaattgt agtttttaaa 1320
atttggttta taggtattat tgtttttatt aaagacgagg aaataagttt agaaaagtat 1380

taattgtttc gagttatttg tttggttagt taggattaga gtttagtttt ttatttaatt 1440
taaagtttag gtttttaatt ttttataatt ggcgtatttt ttttgaattt ttattttttt 1500
tttgtaaaag aataatatcg gtcgggcgta gtggtttata tttataattt tagtattttg 1560
ggaggtagag atgggcggat tacgaggtta ggagtttgag attagtttgg ttaatatggt 1620
gaaattttat ttttattaaa aatataaaat ttagttgggt gtgttggtgt ttgtaatttt 1680
agttatttag gagattgagg taggagaatt gttttaattt gggaggcgga ggttgtggtg 1740
agttaagatc gtgttattgt attttagttt cggtgataga gtaagatttt atttttaaaa 1800
aaataaataa taataataaa aagagaataa cgttatattt agttgaatta aaatgaatta 1860
aatattaata tttgtatttt aaaaacggtt tagtttggtt gggcgtagtg gttttcgttt 1920
gtaattttaa tattttggga ggtcgaggta ggaggattat ttgaggttag gagtttgaga 1980
ttagtttggt taatatggtg aaattttgtt tttattaaaa atataaaaat tagttgggta 2040
gtagtagcgc gtgtcggtaa ttttagttat ttaggaggtt gaggaaggag aattgtttga 2100
gtttgggagg tgaaagttgt ggtgagttga gattgtatta ttgtatatta gtttgggaga 2160
tagagtaaga ttttgtttta aaataaaata attaaaaaat taaaaaggtt tagtttgggt 2220
aatatagtga aatttcgttt ttatagaaaa tttttaaaat attagtaggg tatcgggtat 2280
agtggtttat atttgtaatt ttagtatttt gggaggttga ggtaggcggg ttatttgtgg 2340
ttaggagttt gggattaggt aggttaatat ggtgaaatcg tgtttttatt aaaaaataaa 2400
aattagttgg gtatggtggt aggtattagt aattttagta tttaggaggt tgaggtatga 2460
gaattgtttg aattcgtaaa gtaggggttg tagtgaatta agatggcgtt attgtatttt 2520
agtttgggtg atagaataag attttttaat taaaaaaaaa aaaaattagt tgggtatggt 2580
gttgcgggtt tgtggtttta ggtatttagg aggttgaggt gagaggatta tttaagtttg 2640
ggaggttgag gttatagtaa gttaagatta cgttattata ttttagtttt tgtgatagag 2700
ttagattttg ttttaaaaaa attttaaaaa gggtaaattt tggtaatttt atatagttta 2760
atttagtata aggtggttgt aataattaaa tgagataaaa tggtgttaaa ttggaagtat 2820
tatagtattt ttgttaataa tatagggttt tagaattagt ttttttgagt ttaaatttag 2880
gttttattat tttttagtta tgtagttatg ggtaagttat ttgatttaat tgtgttttag 2940
ttttatttat gatatggaga tataggataa ttagtttttt acgtgtaatt ttgaaattta 3000
aaaagttttg taaattaaaa gtttgggggt aaatttattt ggtagtaaat tttgatttga 3060
ggttatttat agtttatatt ttgtattttt tttatttagt atgaataagt atgtaagttt 3120
tattgtatgt ttgattttag tatgtttttt tagatttttt gggggtgttt acgtatgtcg 3180
gtgggggaaa gagattaatt tttaaatatt attttaaata gttggtttta ttgtgtttgt 3240
ttgggtagta tatatattaa aattggaatg atttttgtat agggatgaaa tgtaaattcg 3300
tgaagtatat tgtatttttt ttagtatata ttatttttgg taatattttt tttttttttt 3360
tgagagggag ttttgttttg tcgtttaggt tggagtgtag aggcgcgatt tcggtttatt 3420
gtaagtttcg tttttcgggt ttatattatt tttttatttt agttttttta gtagttggga 3480
ttataggcgt gtgttattac gttaggttaa ttttttgtat ttttagtaga ggcggggttt 3540
tattgtgtta gttaggatgg tttcgatttt ttgatttcgt gattcgttta ttttcgtttt 3600
tttttcgaag tgtcgagtgt tgggattata ggcgtgagtt attgcgttcg gttttcgttt 3660
ttttttttta gattgatttt attatttgtt tagtaaagga gaattttttg gtagaatagt 3720
ttttaagaat aaggtaaata attaatttta tataggtttt tattaatgta tagttgttga 3780
ttgtgattgg ttttcggtaa tttggatttt ataatttgga taaggggata aataattgtt 3840
tgttttttat tatttttttt gaatttgaat agaatttttt tatttttata gttttttagt 3900
tttttttttt tttttttgga gacggagttt cgtttttgtc gtttaggttg gagtgtagtg 3960
gcgcgatttt ggtttattgt aaacgttgtt ttttaggttt aagttatttt tttgttttag 4020
ttttttaagt agttgggatt ataggcgtat gttattacgt tcggttaatt tttggatttt 4080
tagtagagac gggggtttta ttatgttgat taggttggtt tttaacgttt gattttaggt 4140
gattcgttcg tttcggtatt ttaaagtgtt gggattatag gcgtgagtta ttgcgttcgg 4200
ttttttatag ttttttagtt ttttaaaatt tgaaaaattt tgtaaagata tatttgggtt 4260
aaagggttta gataacggat tgtggttttt aagtatttac gttataggtt attgagagga 4320
tcgatttagt tattagatgt aaaatgttgg gattagtgtt tggtaaagga aaattttgta 4380
tagttgtagg tttttattat atataatagt atcgttaacg aatgttatta taatatttat 4440
ttagcgttta ttaagtgttt attttatata aattttgaga atataacgtg aaggtgaatt 4500
gttgattaaa gtttggtttt tttcgtttcg tttttttgcg aaaatgtttt aacggtagga 4560
ggttacgcga gcgttggacg cgtttttttt cgcgagtttt ttttcgaggt ttttcgggtt 4620
ttttcggtta ttttcgttcg ggcggtgcgc gttttcgttg ttttcgtttt cgttttagag 4680
aggtagggtt tttcgagttt gttagtttcg cggtcgtaat taatttcggg tcggagtgtt 4740
tcggttcggt tagtttcgcg gcgtgaggga aggggagttt agtagttttt cgcgcggggt 4800
ttaggcgtcg gcggtagggc gggtttttta tcgttagcgt gttagtttcg tttttattta 4860
ttagtgtgtt agtttcgttt ttttttacgt cgtcgcgcgt tcgggggcgg ggtttggcgc 4920
gtatcgttcg cgtacggcga ggcgtttgtt gattggttat tggggttcgg gttttttcgg 4980
cggagcgcgt ttttttttag atttttcgtt agtaggagtc gcgcggtaga tgcggtgttt 5040
ttaggagttt cgttcgatag aacggttggg ttttgtcggt tgtcggtatg tcgcgataga 5100
gtattttgta tagttttttt tttaagtttt cggcgttgag tgatgttaat aaggtttcgg 5160

ttagggtttt acgcgaaggc ggtcgtgtcg tcgttgtttt cggggttttt ttttttttag 5220
gcggggatgc ggtttggagc gaggttgggt ttgggtttag gtttttggcg cgtttcgcgt 5280
tatcgtttaa ggcgaagaat tttaacggag ggttgcggag atcggtagcg tttgttgttt 5340
ttattaggta gcggggtggg ggtggggtcg aaggcggggg tatagcggcg gggcgtttgg 5400
aattcggcga ggggaggttc gtataggggg ttgggggggt gtacggtttg gttttgggtt 5460
cggaggaggc ggggtcgtag agttggtttg aatgagtgta ggggtcgagt ttggagtatt 5520
tgggggtgta gtttgtaaat agggtcggag gagagaggtt gtgtaggaag agggttgtag 5580
gggagacgcg gagagttcgg gttttttgga gggaggagac gcgtttcgtt aggtgggggt 5640
gttgggttaa ggaaggggcg acgcgcgtag tttcgggtgg ggagggggtt tgggaggtgg 5700
gagtattggg ggtggggcga gaaggggaag gcgttcggtt tatttggtgg gcggggcggg 5760
gggcggggtg gcgggaagga ggaatgtttg cgggaggtcg aacggggaga gttcggtggt 5820
gtggggtgcg aaaggaggtt tttcggtcgg cgcggagata gtgagttggg gttttagtag 5880
tcgatcgagg tagatattta gaggtagtta agagtcgcgg tcgtcgagac gttttgggga 5940
cggtgggttt tcggtttagg tgaggggtcg tcgagggggt gggttacgag ttgcgagcgc 6000
gggggggtgt gttattatgg ggatcgcggg gtttaattgg gcggggcggg gtcgtgggga 6060
gtcgaagtgt tgggattcgg ttgggttttt cggtaggtag gttgtacgtg tatcgagacg 6120
aagatagaat attttgacgt atgtggaaat tcgtgtcgag tggaaaatat tttattttat 6180
gaaatagtgt aatttttatg gggtattatt gggtttttag aggttttaat cgggcgttgg 6240
ataaagatgt gtggacgtga gtgatttcgg ggaagtttgt cgggagttgt ttttatttta 6300
tgggtagtta agtgtttttt tttttttttt ttttttgaga gagagtttcg tttaagttta 6360
ggttggagtg taatggcgcg attttagttt atcgtaattt tcgcgtttcg gttttaagcg 6420
attttttagt tttagttttt cgagtagtcg ggattatagg aatgcgtttt tatatttcgt 6480
taattttgta tttttagtag agacggggtt tttttatgtt ggttaggtta gtttcggaat 6540
tttcgatttt aggtgattta ttcgtttcgg ttttaaagtg ttgggattat aggcgttagt 6600
tatcgcgttc ggtttgttta gggtttttta ttcgggtagt tggcgatatt ttgaaaagtt 6660
tgtttttgtt gtttgttaga tatatatata tgtattttga gatagagttt tgtttttttg 6720
tttaggttag agtgtagtgg cgcgtttttg gtttattata atttttgttt ttggattaag 6780
agattatttt gttttagttt tttaagtagt tgggattata ggtgcgtttt attacgtttg 6840
gttaattttt gtatttttag tagagacggg ttttattatg ttggttaggt tggtatcgaa 6900
tttttgattt tttgatcggt tcgtattggt ttattaaagt gttgggatta taggtatgaa 6960
ttatcgagtt cggtcgtttg ttagatatta aatataaagt ttaatggtcg ttatttgaat 7020
aaacgaagaa ataaaggttt agaaaaaata atttatttaa gataagagtt agttcgtgtt 7080
ttttgtttgg ttttgttttg aaatggagtt tcgttttgtc gtttaggttg gagtgttgtg 7140
gcgttttttc ggtttattgt aatttttgtt cgtcgggttt aagtgatttt tttgttttag 7200
tttttcgagt agttgggatt acgggtgtgt ttatcgcggt tcggttgatt ttttattatg 7260
gagttttatt atgttggtta ggttggtttt gaaattgttg attttaagtg gtttatttat 7320
tttagttttt taaagtgttg ggattatagg tgtgagttat cgtgttcggt cgttagttag 7380
tggttttgag taatggattt taaatttatt taaatttagt ttaaagtgtt ttaaaggaat 7440
tttgagattt ttttaagtgt aattatagtg ttatttttgt ttaagcgatt ttttttcgta 7500
gtttaaatat atatagttgt gtattagtag aatatgtttg tggggaatag agttagtatt 7560
cgtaataata aatttttggt tagaaaagta tgacgtattg tttatttgag tatgaattga 7620
ttgttgaatt taaattaaac gggtgtattt attgtaagga tgtattttat atttatattg 7680
aatagcgtat gttatttgtt ttttgaggtt gagtttaaga gatttgtaaa aataaaacgt 7740
atatatttta tttttcgtta tggagaggat tttagggtat ttaagaaaga tgggtatttg 7800
atattaggtt tttaaagaaa ttgtagtgtt tagattattt tgtcgagtat agtattaggt 7860
attatggatt ttggatataa ttatgaatag gataaagtaa agaggtaatt gtagatttta 7920
agtggaaagg ggacggagag gatgcgggtt aggttaggtt tttagttttg taaatcgaaa 7980
ttagaaggat aaataagttt agatagatta tagtgagagt gggaagttgg tttaggaaga 8040
ggaaggtttg taaattgtgg gtaggatgaa aggaggagga gggagtattg gagaagttaa 8100
gtagagattt aattatgaat agtttgatga gttatagaga tattcggatt tattttatga 8160
attatttaag ttttaaaata tgttgagcgt attttttttt tttttgagac ggaattttat 8220
ttttgttgtt taagttggag tgtagtggtg tggttttagt ttattgtaat tttcgttttt 8280
tgggttttag cgattttttt gttttatttt tttaagtatt tggtattata ggtgtttgtt 8340
attacgttta gttaattttt gtgtttatag tagagacggg ttttattatg ttggttaggt 8400
tagtcgtgaa tttttgattt taggtgattt atttatttta gttttttaaa gtgttgggat 8460
tataggcgtg aattatcgta tttggtcgtg agttatcgtg tttgttcgag tattttttaa 8520
tgtttgttat ttagattttt ttttgtgttg aagtgtttgt tttttgttgt tttttttttt 8580
ttttttagtt ttttgttatt tgtgtgtgat ataaatgttt ttttttataa tgagtttttt 8640
tatttagttt atggttttgt tgttgttgtt gaataataga ggttttattt tgttgtttag 8700
gttggtgttg aatttttgtt tttaagcgat ttttttattt tagtttttta atttgttggg 8760
attataagtg tgagttatta tatttagttt tatggtattt ttcgatgaat aaattattga 8820
ttataatgtg gaatttgttt ttttattttt tttgtggtta gtgtttttat aggttttatt 8880
taagaaattt atagggaggt tgggtgtagt ggtttatgtt tgtaaattta atattttggg 8940

aggttaaggt aggttaatat ggttagattt tgttttttta aaaaataaga aaattagtta 9000
ggtatggtgg cgtgtgtttg tagttttagt tttttgggag attgagatgg gaggatcgtt 9060
tgagtttagg aggttgaggt tgtagtaagt taagagatta tgttatgtat tttagtttgg 9120
gtggtagagt tagattttgt ttgttaaaaa ataaaataaa agttggtgaa aatgttgatt 9180
atatatttta ggaataatta gtaattgata ttaaaattat gggttaaaga gaaagtaaaa 9240
ataatgtgat tttaaattag aatttaaaag atttgtttag cgtatgttta gataaagtta 9300
ttatttatta tattaaagtt ttaatattta ttttgtgagt tgttagtttt tttttttaat 9360
attttttttt atcgttttaa aaaattttaa gaatatcgga tatttaagat ttatttaaag 9420
ttttaaaagt atttgtaaaa ttttaaaatt ataatttaag gtgtttttgg agggtaggag 9480
taatggtggt aggtagtgtt ttgttttgtt gtttaggttg aagtatagtg gtagatttcg 9540
gtttattgta ttttcgattt attcggttta agtgattttt ttattttagt tttttgagta 9600
gttgggattt taagtgtata ttattttatg tttggttaat ttttaaattt ttttgtagaa 9660
ataaggtttt attgtgtagt ttagatggtt tcgaattttt gggtttttaa gagatttttt 9720
taaagtgttg ggattatagg tgtgagttat tatatttggt ttattttggt atttttgaaa 9780
atcgtaggat tattacggat aaaattttaa aattattttt aaagaattta ggtttatata 9840
taaaaaaaat ttggtttgtt agtagtgagt gaagaaaaat tttgagaaat gtttaaaatt 9900
tttagttttg ttatataata tattttatta tttgtttaat tatttttttt gatttagaaa 9960
ttagtttttt gggtttagga tgtttagtgg tatttttttt ttttaagttt tttagtattg 10020
gaggaattgt atattagtaa aatttttagt tttagtattt tatagtttat tgttattttt 10080
ttttttttat tttttttttt tttttttttt tttttttttt ttttttgaga tggagtttcg 10140
ttttgttatt taggttggag tgtagtggta cgatttcggt ttattgtaat ttttgttttt 10200
taggtttaaa tgattttttt gtttagtttt ttcgagtagt tgggattata gatgttcgtt 10260
attatgttta gttaattttt atttttttag tagagatggg gttttattat gttggttagg 10320
ttagtttcga atttttgatt tcgtgattaa ttcgttttgg ttttttaaag tgttgggatt 10380
ataggcgtga gttatcgtgt ttagtttttt tttttttttt tttttttttt tttttttttt 10440
tgagacggag ttttgttttg ttatttaggt tggagtgtag tggtatgatt tgggtttatt 10500
gtaattttta tttttcgggt ttaagggagt tttttgtttt agtttttcga gtagttggga 10560
ttataggcgt ttgttattat gtttagttaa tttttgtatt tttttagtag agatggggtt 10620
tcgttatgtt ggttaggttg gttttgaatt tttgatttta ggtgatttgt ttgtttcgtt 10680
tttttaaaat gttgggatta taggagtgag ttattgcgtt cggtttagta tattgttatt 10740
tttttttttt tttttttttt tttttttttt tttttgtttt tttttttttt tttttttttt 10800
tgtgagacgg agtttgtcgt ttaggttgga atgtagtggc gtttttttgg tttattgtaa 10860
tttttgttgt tcgggtttaa gtgatttttt tgttttaggt ttttaagtag ttgggattat 10920
aggtttttgt tattgtattt ggttaatttt tgtatttttg gtagagacgg ggttttatta 10980
ttttggttag gttggttttg aatttttgat ttcgtgattt atttgttttg gttttttaaa 11040
gtgttgggat tatagatttg agttatcgta ttcggtttat attgttattt tttaatagta 11100
gagaaattat gtgttagatt ttgtaagtgt aatggtatat aaaggataaa atgatgttga 11160
aaaataaaat tttttgttta aatgtttatg tttttaatat tttattttag aaaggaattt 11220
attttaaaat tgataatggt tggatttagt tttttatata aatttttttt ttttagtgag 11280
gatgtatatt tattttgtaa ataaatggaa gatattattt ttttaattgt ttgtttagaa 11340
atgaaataat ttttttttaa tgatttttta aagtatgaga ttttatatat tatttaaaat 11400
aatttatatt gtattttata tatgataaag ttttaaggta atagtttttt tttaagatta 11460
aagttatagt tttttttttg tatttgaggg ggattggttg taggattttt ttgtgaatat 11520
ttaaattttt ggatgtttaa gttttttatg agatgtagta tttgtatata atttatatat 11580
attttttttt gtattttatt tttagattac gtataatatt taatagaatg taaatgtttt 11640
gaaattagtt gtttagttgt attttaaatt ttgtattttt tttttttttt ttttgagata 11700
gagttttgtt ttgttgttta ggttggagta tagtatagtg attatagttt attgtatttt 11760
taatttttta ggtttaagtt gttttgtttc ggttttttta agtgttggga ttataggtgt 11820
gagttattat atttggttat tgttttttat tggttttaaa tttttgattt aaaattttta 11880
atttaggttg gttgaatttg gattggaatt taaggatatg tttgttgagt atattgtatt 11940
tattttggaa tataattaga atgtttaatt tgtatgttaa aaatatttta tttggttagg 12000
cgcggtggtt tacgtttgta attttagtat tttgagaggt taaggcgggt gaattatttg 12060
aggttaggag tttaagacga gtttggttaa tatggtaaaa ttttgatttt ataaaaaaaa 12120
ggtaaaaata agttaggtgt gatggcgtgt gtttgtagtt ttggttattt aggaggttga 12180
gatataagaa tcgtttgaat cggggaggta cgttacgttt ttagttgttg atttgagttt 12240
tttcgtagtt tgtaggggga gggtaataga gtattaggta gtttttggaa tatataggag 12300
tgtaattgga aaaagatttt aagtaagttt aatgaattag ataatttatt taattagtaa 12360
attatgtaat tagtatgttt tataataata ttgtgagtta gattttgttt ttgatatgta 12420
tatattatat tgtataggtg ttattaattt ggagagtata tatagtgagt ttatgttttt 12480
tttttgttat tagtattata ttaaaatttt gttatggttt ttaaattttg attttgagaa 12540
agttttttat tttaataata taattatatt tgtgtttgtt tttatagtta aatatgtatt 12600
atgatattag tttgtatata ttttttaaat gatttgaata tttgatttta aaaattattt 12660
tagaattttt gtgttttaat attaatgtta gaagatttgg aattgtttat ttgtaggtaa 12720

ttgtttttaa ggaaatttga ttaaatatta attaagttat gtattttttt ttggtaatag 12780
t 12781

<210> 184
<211> 12781
<212> DNA
<213> Artificial Sequence

<220>
<223> chemically treated genomic DNA (Homo sapiens)

<400> 184

attgttgtta aaaggaaata tataatttag ttaatatttg gttaagtttt tttaaaggta 60
gttatttata aataaataat tttaagtttt ttggtattaa tattgaagta tagaaatttt 120
agaataattt ttaaagttag atatttaagt tatttaaaaa atgtatgtaa gttgatatta 180
taatgtatat ttaattatga agataaatat aaatatagtt atgttattag ggtgagaaat 240
ttttttagaa ttaaagttta aaaattatgg tagaattttg gtataatgtt gatggtagga 300
aaaaggtatg gatttattgt atatgttttt taaattagta gtatttatat aatatggtat 360
gtatatatta gaaataggat ttaatttata atattattat aaagtatatt gattatataa 420
tttattaatt aggtaaatta tttaatttat tagatttgtt tggaattttt ttttagttat 480
atttttatgt attttaaaag ttatttaata ttttattatt ttttttttat aaattacgga 540
aaaatttaag ttaataattg agggcgtaac gtgttttttc ggtttaagcg atttttgtgt 600
tttagttttt tgaatagtta agattatagg tatacgttat tatatttggt ttatttttat 660
tttttttttg tagagttagg gttttgttat gttggttagg ttcgttttaa atttttgatt 720
ttaaatgatt tattcgtttt ggttttttaa agtgttggga ttataggcgt gagttatcgc 780
gtttggttaa ataaagtatt tttaatatat aagttaagta ttttagttgt attttaaagt 840
aaatatagta tgtttaataa atatattttt gggttttagt ttagatttaa ttaatttaga 900
ttaaaaattt taaattaaaa atttaaaatt aataaaaaat agtgattagg tatgatggtt 960
tatatttgta attttaatat ttggggaggt cgaggtagga tagtttgagt ttgggaggtt 1020
aaaggtgtag tgagttgtga ttattgtatt gtattttagt ttgggtaata gagtaagatt 1080
ttgttttaaa aaaaaaggaa aaaaaatata aaatttaaaa tatagttgaa taattaattt 1140
taaagtattt atattttatt aggtattgta cgtaatttag agataaagta taggggaaga 1200
tgtgtatagg ttatatgtaa atattatatt ttataaggga tttggatatt taaggatttg 1260
ggtatttata ggggggtttt gtaattaatt ttttttagat ataaagggag gattgtaatt 1320
ttagttttag aaaagggttg ttattttaga attttgttat gtgtaaaata tagtataaat 1380
tgttttaaat gatgtatgag gttttatgtt ttaaaagatt attagaaaag aattatttta 1440
tttttaagta agtaattaaa aaaataatgt tttttatttg tttataggat aaatgtgtat 1500
ttttattagg aaaaaaaagt ttgtgtgaaa agttggattt aattattatt agttttgaaa 1560
taaatttttt tttgaaataa aatattagaa atataagtat ttaaataaaa aattttgttt 1620
tttaatatta ttttattttt tatatattat tatatttata gaatttgata tataattttt 1680
ttgttgttaa gaaatagtag tatgggtcgg gtgcggtggt ttaggtttgt aattttagta 1740
ttttgggagg ttaaggtagg tggattacga ggttaggagt ttaagattag tttggttaag 1800
atggtgaaat ttcgttttta ttaaaaatat aaaaattagt taagtgtagt ggtagaggtt 1860
tataatttta gttatttggg agtttgaagt aggagaatta tttgaattcg ggtagtagag 1920
gttgtagtga gttaagaaaa cgttattgta ttttagtttg gacgatagat ttcgttttat 1980
aaaaaaaaaa aaaaaaaaaa aaaaataaaa aaaaaaaaaa ggaagaagaa agaaagaaag 2040
aaatagtagt atgttgggtc gggcgtagtg gtttattttt ataattttag tattttggga 2100
ggacgaggta ggtagattat ttgaggttag gagtttaaga ttagtttggt taatatggcg 2160
aaattttatt tttattaaaa aaatataaaa attagttggg tatggtggta ggcgtttgta 2220
attttagtta ttcgggaggt tgaagtagga gatttttttg aattcgggag gtggaggttg 2280
tagtgagttt agattatgtt attatatttt agtttgggta atagagtaag atttcgtttt 2340
aaaaaaaaaa aaaaaaagaa aaagaaaaag aaaaaggttg ggtacggtgg tttacgtttg 2400
taattttagt attttgaaag gttaaggcgg gttgattacg aggttaggag ttcgagattg 2460
gtttggttaa tatggtgaaa ttttattttt attaaaaaaa taaaaattag ttgggtatgg 2520
tggcgggtat ttgtaatttt agttattcgg gaggttgaag taggagaatt atttgaattt 2580
ggaaggtaga ggttgtagta agtcgagatc gtgttattgt attttagttt gggtgatagg 2640
gcgagatttt attttaaaaa aaaaaaaaaa aaaaaaaaaa agaaagaaag aatgaaagaa 2700
aagaaatagt agtaagttat aaaatgttaa gattaaaaat tttattaata tgtagttttt 2760
ttaatgttaa aaagtttgaa gaaaaagagt attattaaat attttggatt taggaaattg 2820
gtttttgagt taaaaaagat aattaaatag gtagtaaaat gtattgtgta ataaaattaa 2880
aaattttaaa tattttttaa aatttttttt tatttattgt taataaatta aatttttttt 2940
gtgtgtaaat ttgaattttt taaaggtaat tttaaaattt tattcgtggt aattttgcgg 3000

tttttaagaa tgttaaaata ggttaggtgt ggtggtttat atttatgatt ttagtatttt 3060
gggaggattt tttaagagtt taggaattcg agattatttg ggttatatag tgagattttg 3120
tttttataaa aaaatttaaa aattagttag gtatggggtg gtgtgtattt ggggttttag 3180
ttatttagaa agttgaggtg ggaggattat ttgagtcgaa taggtcgagg gtgtagtgaa 3240
tcgagatttg ttattgtatt ttagtttggg taataaagta aaatattgtt tgttattatt 3300
gtttttgttt tttaaaaata ttttaaatta tgattttagg attttgtaag tgtttttaaa 3360
gttttaagtg agttttaaat gttcggtatt tttggggttt tttaagacgg tggggaaaaa 3420
tgttaagagg aaaagttgat agtttataaa ataaatgtta aaattttgat ataataagta 3480
atggttttgt ttaaatatac gttaaataga ttttttgaat tttggtttaa aattatatta 3540
tttttgtttt ttttttagtt tataattttg atgttaatta ttagttgttt ttaaaatata 3600
taattaatat ttttattaat ttttatttta ttttttggta gatagggttt gattttgtta 3660
tttaggttgg agtgtatggt atgatttttt ggtttattgt agttttaatt ttttggattt 3720
aagcgatttt tttattttag ttttttaaga agttgggatt ataggtatac gttattatgt 3780
ttggttaatt tttttatttt ttggagagat agggtttagt tatgttggtt tgttttggtt 3840
ttttaaagtg ttgggtttat aggtatgagt tattgtattt agtttttttg tggatttttt 3900
aaataaaatt tatagaaata ttaattatag agaaaataaa aggataaatt ttatattata 3960
attaataatt tgtttatcga aagatgttat aaggttgggt gtggtggttt atatttgtaa 4020
ttttaatagg ttgggaggtt gaagtgggag gatcgtttga gagtaagagt ttaatattag 4080
tttgggtaat aaagtgagat ttttattatt taataataat aataaagtta taaattaaat 4140
gaaagaattt attgtgaaag aagatattta tattatatat aaatgataaa gaattaggaa 4200
aaaaaaaaga aatagtaaaa gataaatatt ttagtataag aagaaattta aatgataaat 4260
attaaaagat gttcggatag atacggtggt ttacggttag gtgcggtggt ttacgtttgt 4320
aattttaata ttttgggagg ttgaggtggg tggattattt gaggttagga gtttacgatt 4380
ggtttgatta atatggtgaa attcgttttt attataaata taaaaattag ttgggcgtgg 4440
tggtaggtat ttgtaatatt aggtatttga gaggatgagg taggagaatc gttggaattt 4500
aggaggcgga ggttgtagtg agttgagatt atattattgt attttagttt gggtaataag 4560
agtgaaattt cgttttaaaa aaaaaaaaaa tacgtttaat atgttttaag gtttaaatga 4620
tttatggagt aagttcgaat gtttttgtag tttattagat tgtttatgat tggatttttg 4680
tttaattttt ttaatgtttt tttttttttt ttttatttta tttataattt gtagattttt 4740
ttttttttga attagttttt tatttttatt ataatttgtt taagtttatt tgtttttttg 4800
gtttcggttt atagagttga gagtttagtt tgattcgtat ttttttcgtt ttttttttat 4860
ttggagttta taattgtttt tttgttttgt tttgtttatg gttgtattta ggatttataa 4920
tgtttaatgt tgtgttcggt agagtgattt agatattgta gtttttttag aaatttagta 4980
ttaaatgttt attttttttg aatattttgg aatttttttt ataacgggag gtgaaatgta 5040
tacgttttat ttttataagt tttttaaatt taattttaag aaataaataa tatacgttat 5100
ttaatatgaa tgtaaagtat atttttataa taaatatatt cgtttggttt ggatttaata 5160
attaatttat gtttaagtaa ataatacgtt atgttttttt aattaggagt ttgttattgc 5220
ggatgttggt tttgtttttt ataagtatat tttattaatg tataattata tatatttaaa 5280
ttgcgggaaa gggtcgttta aataagggta atattataat tatatttaga aaaattttag 5340
aattttttta ggatatttta aattggattt aaatggattt gaaatttatt atttaaaatt 5400
attaattagc ggtcgggtac ggtggtttat atttgtaatt ttagtatttt gggaggttga 5460
agtgggtgga ttatttgagg ttagtagttt taagattagt ttggttaata tggtgaaatt 5520
ttatggtgaa aaattagtcg gatcgcggtg ggtatattcg taattttagt tattcgggaa 5580
gttgaggtag gagaattatt tgaattcggc gggtagaggt tgtagtgagt cgagaaagcg 5640
ttatagtatt ttagtttggg cgatagagcg agattttatt ttaaaataaa attaaataaa 5700
aaatacgaat tggtttttat tttgaatgag ttattttttt tgagttttta ttttttcgtt 5760
tgtttaaata gcgattatta aattttgtgt ttagtatttg ataaacggtc gggttcggtg 5820
gtttatgttt gtaattttag tattttggta ggttaatgcg ggtcgattaa gaggttagga 5880
gttcgatatt agtttggtta atatagtgaa attcgttttt attaaaaata taaaaattag 5940
ttaggcgtgg tggggcgtat ttgtagtttt agttatttgg gaggttgagg taggataatt 6000
ttttgattta gagatagagg ttgtggtgag ttaagaacgc gttattgtat tttagtttgg 6060
ataaaagagt aagattttgt tttaaaatat atatatatgt atttggtaaa tagtaaaagt 6120
aagtttttta aaatgtcgtt agttgttcgg ataaaaagtt ttaaatagat cgggcgcggt 6180
ggttagcgtt tgtaatttta gtattttgag gtcgaggcgg gtggattatt tgaggtcggg 6240
aatttcgaga ttagtttgat taatatggag aaatttcgtt tttattaaaa atataaaatt 6300
ggcggggtgt gggggcgtat ttttgtaatt tcgattattc gggaggttga agttggggaa 6360
tcgtttgaag tcgggacgcg gagattgcgg tgagttgaga tcgcgttatt gtattttagt 6420
ttggatttga gcgaaatttt tttttaaaaa ggaaaaaaaa aaaaaagtat ttaattgttt 6480
ataaagtgag gataattttc gataggtttt ttcggagtta tttacgttta tatatttttg 6540
tttagcgttc gattaaggtt tttaaaagtt tagtggtgtt ttataaaaat tatattattt 6600
tataaaataa aatatttttt attcgatacg aatttttata tacgttaaaa tattttattt 6660
tcgtttcggt gtacgtgtag tttatttatc gaaggattta gtcggatttt agtatttcgg 6720
ttttttacgg tttcgtttcg tttaattagg tttcgcggtt tttatggtga tatatttttt 6780

cgcgttcgta gttcgtggtt tattttttcg gcggtttttt atttaggtcg aaggtttatc 6840
gtttttaagg cgtttcggcg atcgcggttt ttaattattt ttaagtgttt atttcgatcg 6900
attattggag ttttaattta ttattttcgc gtcggtcgag gaattttttt tcgtatttta 6960
tattatcgga tttttttcgt tcggtttttc gtaggtattt ttttttttcg ttatttcgtt 7020
tttcgtttcg tttattaagt gggtcgggcg tttttttttt ttcgttttat ttttagtgtt 7080
tttatttttt aggttttttt tttattcgga gttgcgcgcg tcgttttttt tttagtttag 7140
tatttttatt tggcgggacg cgtttttttt ttttaaaagg ttcgaatttt tcgcgttttt 7200
tttgtagttt tttttttgta tagttttttt ttttcgattt tgtttataag ttatattttt 7260
aaatgtttta gattcgattt ttgtatttat ttaagttaat tttgcggttt cgtttttttc 7320
gagtttaggg ttaggtcgtg tattttttta attttttgtg cgagtttttt ttcgtcgggt 7380
tttaagcgtt tcgtcgttat gttttcgttt tcgattttat ttttatttcg ttatttggtg 7440
ggggtagtag gcgttatcga ttttcgtagt ttttcgttga ggtttttcgt tttgggcggt 7500
gacgcggagc gcgttaaggg tttgggttta ggtttagttt cgttttaggt cgtattttcg 7560
tttggggaag gagaggtttc gggggtagcg gcggtacggt cgttttcgcg tgaggttttg 7620
gtcgaggttt tgttggtatt atttagcgtc ggagatttgg ggaagaagtt gtatagggtg 7680
ttttgtcgcg atatatcgat agtcggtaag gtttaatcgt tttgtcggac ggagttttta 7740
aaagtatcgt atttatcgcg cggtttttgt tggcgggaaa tttgggggga ggcgcgtttc 7800
gtcggaggaa ttcgggtttt agtggttaat taataggcgt ttcgtcgtgc gcgggcggtg 7860
cgcgttaggt ttcgttttcg ggcgcgcggc gacgtgggga agggcggggt tggtatattg 7920
gtgggtaggg gcggggttgg tacgttggcg gtgaggggtt cgttttgtcg tcgacgtttg 7980
ggtttcgcgc ggggaattgt tgagtttttt tttttttacg tcgcggggtt ggtcgggtcg 8040
gaatatttcg attcggggtt agttgcggtc gcggggttag taggttcgga aagttttgtt 8100
tttttggagc ggaagcggga atagcggggg cgcgtatcgt tcgggcgggg ataatcgggg 8160
ggattcgaaa ggtttcggaa aggggttcgc ggggagaaac gcgtttagcg ttcgcgtgat 8220
tttttgtcgt tagagtattt tcgtaaggag acggagcgaa agggattaaa ttttagttag 8280
tagtttattt ttacgttgta tttttaagat ttgtatagag taggtatttg gtaaacgtta 8340
aatgaatatt gtaatagtat tcgttagcga tgttgttgtg tatggtgaaa gtttgtagtt 8400
gtataaagtt tttttttgtt aggtattgat tttagtattt tatatttggt aattaaatcg 8460
atttttttaa taatttgtga cgtaagtatt taagggttat agttcgttat ttgagttttt 8520
tgatttaggt gtgtttttat aggatttttt aaattttaga aagttaagag attatgagag 8580
gtcgggcgta gtggtttacg tttgtaattt tagtattttg ggatgtcgag gcgggcggat 8640
tatttgaggt taggcgttga agattagttt agttaatatg gtgaaatttt cgtttttatt 8700
aaaaatttaa aaattagtcg ggcgtggtgg tatgcgtttg taattttagt tatttgggag 8760
gttgaggtag gagaataatt tgaatttggg aggtagcgtt tgtagtgagt taaggtcgcg 8820
ttattgtatt ttagtttggg cgataagagc gaaatttcgt tttaaaaaaa aaagaaagaa 8880
agttaagagg ttatgagaat aaaaaggttt tatttaaatt taagaaagat agtggaagat 8940
agataattgt ttgttttttt atttagattg tgaaatttag attgtcggaa attagttata 9000
attaatagtt gtatattggt aaaaatttat gtaaaattag ttgtttgttt tgtttttgga 9060
gattgttttg ttagaaggtt tttttttgtt aggtaagtaa taaaattaat ttaaaaaaaa 9120
aaggcggggg tcgggcgtag tggtttacgt ttgtagtttt agtattcggt atttcggggg 9180
gggggcggag gtgggcggat tacgaggtta ggagatcgag attattttgg ttaatatagt 9240
gaaatttcgt ttttattaaa aatataaaaa attagtttgg cgtggtagta tacgtttgta 9300
gttttagtta ttggggaggt tgaggtagga gaatggtgtg aattcgggag gcggagtttg 9360
tagtgagtcg agatcgcgtt tttgtatttt agtttgggcg atagagtaag attttttttt 9420
aaaaaaaaaa aaagaatatt gttaaaggtg gtatgtgtta agaaaaatat agtatgtttt 9480
acgaatttgt attttatttt tgtgtagggg ttattttaat tttggtatat gtgttattta 9540
agtaagtata gtgaaattaa ttgtttgaga taatatttga gagttggttt ttttttttta 9600
tcggtatacg taaatatttt tagagagttt gggggaatat gttgaaatta aatatgtagt 9660
aaaatttata tgtttattta tattaagtag aaagaatata gaatatagat tataaatagt 9720
tttaggttaa aatttgttat taaatgagtt tatttttaaa tttttggttt atagagtttt 9780
ttggatttta gaattgtacg taagaggttg gttattttgt atttttatat tatggatgaa 9840
gttgaggtat agttgggtta agtaatttgt ttatgattgt atagttagga agtggtggag 9900
tttggattta aatttaagga agttggtttt ggagttttat gttgttaata gaaatattat 9960
aatattttta atttaatatt attttatttt atttagttat tataattatt ttatattagg 10020
ttgaattatg tgaaattgtt aaaatttgtt ttttttaaaa tttttttgag atagggtttg 10080
gttttgttat agaggttgga gtatagtggc gtgattttgg tttattgtag ttttaatttt 10140
ttaggtttaa gtaatttttt tattttagtt ttttgagtat ttgggattat aggttcgtaa 10200
tattatgttt agttaatttt tttttttttt aattgaggag ttttattttg ttatttaggt 10260
tggagtatag tgacgttatt ttggtttatt gtaatttttg ttttgcgggt ttaggtaatt 10320
tttatgtttt agttttttga gtgttaggat tattggtgtt tattattatg tttagttaat 10380
ttttgttttt tagtagagat acggttttat tatgttggtt tgtttgattt taaatttttg 10440
attataagtg attcgtttgt tttagttttt taaagtgttg ggattatagg tatgagttat 10500
tgtgttcggt gttttgttag tattttaaaa attttttgta gagacgaagt tttattatgt 10560

tgtttaagtt ggattttttt ggttttttgg ttgttttgtt ttgagatagg gttttatttt 10620
gttttttaga ttggtgtgta gtagtgtagt tttagtttat tataattttt attttttaag 10680
tttaagtaat tttttttttt tagttttttg aatagttggg attatcggta cgcgttatta 10740
ttgtttagtt aatttttgta tttttagtag agataggatt ttattatgtt ggttaggttg 10800
gttttaaatt tttgatttta aatgattttt ttgtttcggt tttttaaaat gttgggatta 10860
taggcgggag ttattgcgtt tagttaagtt ggatcgtttt tgaagtataa atattaatat 10920
ttaatttatt ttggtttaat tgaatataac gttatttttt ttttgttgtt gttgtttgtt 10980
tttttgggga tggagttttg ttttgttatc gaggttggag tgtaatggta cgattttggt 11040
ttattataat tttcgttttt taggttaagg taattttttt gtttagtttt tttgagtagt 11100
tggggttata ggtattaata tatttagtta agttttgtat ttttagtaga gatggggttt 11160
tattatgttg gttaggttgg ttttaaattt ttgatttcgt gattcgttta tttttgtttt 11220
ttaaagtgtt gggattatag atgtgagtta ttgcgttcgg tcgatgttat ttttttatag 11280
ggaggaaatg gaggtttaga ggggatgcgt tagttgtaag aggttaagag tttgggtttt 11340
gggttaaatg gagaattgag ttttgatttt agttagttaa gtagataatt cggagtaatt 11400
aatatttttt tgaatttgtt ttttcgtttt tgataaggat agtagtattt atgggttaaa 11460
ttttggaggt tatagttaga tgttttttat aaattttatt aaaagataag agaatatatg 11520
tattatgtgg ggatatttag atttttagaa ttggtatgaa tgttatttgg aattgaagat 11580
atttgagagt taatagatgt agaaagatgt cgttttaaag ttttttttat gcgattaata 11640
gtagaaattt ttgggagtga agttgtcgta aatttttttt ttaggggagt tttagtggtt 11700
agaaaaaaag ataaagatta tttgtatttg taaaaataaa tatttttatt ttttttttgt 11760
ttttttagaa atttatttgt tttttttatg gaaggttttt tttttttttt ttttttttat 11820
taaatggtat atataaagta gatttcgaga tttaagaaaa aaaaagttgt ataaatgtta 11880
aataatagtt ttttggattt attttttttt tttgtgaatt tttgtatgta tatttgaata 11940
aaagtttgtt tttttttttt tttttttttg agatagggtt ttattttgtt gtttaggttg 12000
attttttagt tttagttttt taagtagtta agattgtagg tgtgcgtttt tatattttga 12060
taattttatt ttttgtagag attgggtttt gttatgttgt ttaggtcggt ttttaatttt 12120
tgggtttaag tttttttttt tatttggttt tttaaagtgt taggattata ggtttgagtt 12180
atcgtgttcg gttttaaatt gtttttagtt tgtagtgttt aattaatata tatattttat 12240
gattatatat gggttatttt gaaaattgaa taaaaatatt ttttttttat atgtattttt 12300
tttttaaatg gttatattta gtaaattgta taataattaa tggagttgtt ttggtttagg 12360
agatgtggta ggaaagtatt aaggtagtaa atatattagg tgtttgggaa tttttggttt 12420
attggttaag agttttgtat ttaaagtttg tgtttatatt ttagttgagt tgtttattaa 12480
attgtacgag tttgttgggt aagttattta gtttgtgttt tagtttttat atttgtaaaa 12540
tagggatgat attaatatta ttttttttat gggttgtgtt aagtattgag tgaaagggta 12600
ttatgaagtt tttagtttag tgtttggtac gttatacgta tttaataaat tggatagtta 12660
tataaataaa ttattaattg gttagggata gtggtttata tttataattt taatattttg 12720
ggaggttaag atagaaggat gatttgaggt taggagttta agattagtta gtaatatagt 12780
g 12781

<210> 185
<211> 1631
<212> DNA
<213> Artificial Sequence

<220>
<223> chemically treated genomic DNA (Homo sapiens)

<400> 185

ggatttagaa tatggggcgg atttaggtag cggcgggaat taggcggatt cgtttggatc 60
gaagttttcg ttaggtatcg aagtcgtgaa gtcgtttgcg tagtaaaggg agttttcggg 120
gggcgttcga gattttggtg tcgttcgggg gtcgttggta gtcgcgttcg ttgtttttcg 180
ttcggtgatt tagtcggtat ttttggcggg gttatggggg ttagaggata gcgcgggggg 240
cggcgagcgc ggttgttaag ttttttaagg tttcggaggg ataggatttg ggcgtatttt 300
atcggttgtt atggtaacgt agcgttttag tttttcgcgt ttcgcggtgg agggaggcgt 360
agttaatcgg aagcggcgga gttttcgggg attttttttt tttcgtttta cgtcgtgtgt 420
gggcgggggt taggaggtgc ggggcggagg cggagcgtgg gtcgcggaga ttcggcgttc 480
gtaggaggtt tggtgcgcgg cggggttgta cggggttatt ttcggagtag tattgcgagt 540
agcggcgcga tagtgcgggg tttttttttt tttagaagag acgttattta tataaatttg 600
atttttacgt ggggcgcggg attttgcggg gttttagtcg taggcgtttt tgttgttttt 660
ttcgggcggg tgggttggag aagaagttta cgcgggattt tttaaaacgg cgtatagggg 720
gattttaggg ttttatggtt atttttttgg atttttcgga ggcgttgttt agttattttt 780
agtcgttttg atgattcgtc gtgggttttt ttaggagatt cgaaagttta gggcgggttg 840

tgtgggtaat atttttgttt aggttttcgg agggttttac gtggttgttt ttggagtatt 900
ttagagcgtt agggttgcgg gaaggggcgg gtgtgttttt ggaaatatga gcgtttgtta 960
gtattagtgt ttggataggg ttggggataa attagtttat gtcgcgtata tcgaatttat 1020
gcgtttgagt gagggtgggt gtgttgggga gtgcgtttaa gtggatagtg tcgtatagta 1080
atgtttacgg ggagttttag gagagttcgg ttatttttgc gtaggataat ttttttttta 1140
ttattcgagt ttcgtgttcg cgggtaggat tttttcgaat tggggttgtg tgtttagaaa 1200
tacgtatatg ggagcgttta gtttaaagtt ttagggtttt tgggattcgc gtgttcgggg 1260
tcggggtttt aggtgggtat aggtgggagg gcgaatttgc gggtagggtg ggtttttttt 1320
cgcgggttta gtatttgtgc gtttttagtt taggtgcgcg ggaggaaggt agcggtttgt 1380
cgcgtagagt tttgcgcgtt cgcgaggtgg cgttatagtc gtagtagcgt tcgtcggttc 1440
gggtcgtttt agataagagt gtgcggaaag cgcggcgggg ttgagacgcg attaggacgc 1500
ggggaggacg gattagtagg atagatcgat cgggggttcg gcgggcggag ggtagcgtag 1560
ttacgttttt tttggattcg tcgttagtcg ggttcggggt tttcgatatg ttttttaggt 1620
gggttttcga g 1631

<210> 186
<211> 1631
<212> DNA
<213> Artificial Sequence

<220>
<223> chemically treated genomic DNA (Homo sapiens)

<400> 186

ttcgaggatt tatttggggg gtatgtcgaa agtttcgggt tcggttgacg gcggatttag 60
gggggacgtg gttgcgttgt ttttcgttcg tcgggttttc ggtcggtttg ttttgttggt 120
tcgttttttt cgcgttttgg tcgcgtttta gtttcgtcgc gtttttcgta tatttttatt 180
tggagcggtt cgggtcggcg ggcgttgttg cggttatggc gttatttcgc gggcgcgtag 240
ggttttgcgc ggtaggtcgt tgtttttttt tcgcgtattt gagttggagg cgtatagatg 300
ttgagttcgc ggggaggggt ttattttatt cgtaggttcg tttttttatt tgtatttatt 360
tgggatttcg atttcggata cgcgagtttt agaaattttg gggttttgag ttgagcgttt 420
ttatgtacgt atttttaggt atatagtttt agttcggaaa agttttgttc gcgagtacgg 480
gattcgggtg gtgggggaga ggttattttg cgtaggagta gtcgagtttt tttggaattt 540
ttcgtagata ttattgtacg gtattgttta tttggacgta ttttttaata tatttatttt 600
tatttaggcg tatggattcg gtgtgcgcgg tataaattga tttgttttta gttttattta 660
ggtattgata ttaataggcg tttatgtttt tagaagtata ttcgtttttt ttcgtagttt 720
tagcgttttg ggatatttta ggggtagtta cgtagggttt ttcgggaatt tgggtagagg 780
tgttatttat atagttcgtt ttgaattttc gggtttttta aaggaattta cgacgagtta 840
ttagggcgat tggaagtggt tggatagcgt tttcgggggg tttaaagaag taattatggg 900
gttttagaat ttttttgtac gtcgttttga agaatttcgc gtggattttt tttttaattt 960
attcgttcga aggaaataat aggggcgttt gcggttggga tttcgtaagg tttcgcgttt 1020
tacgtgaagg ttaggtttgt gtgggtgacg ttttttttgg gagaaagggg atttcgtatt 1080
gtcgcgtcgt tgttcgtagt attatttcgg aagtggtttc gtgtagtttc gtcgcgtatt 1140
aagttttttg cgaacgtcgg attttcgcgg tttacgtttc gttttcgttt cgtatttttt 1200
gattttcgtt tatatacggc gtggagcggg gggggggggg ttttcgagaa tttcgtcgtt 1260
ttcgattggt tgcgtttttt tttatcgcgg agcgcgaggg gttggggcgt tgcgttatta 1320
tggtaatcgg tgaggtgcgt ttagattttg ttttttcgag gttttggaga gtttagtaat 1380
cgcgttcgtc gtttttcgcg ttgttttttg gtttttatgg tttcgttaga ggtatcggtt 1440
gagttatcgg gcgaggggta gcgggcgcgg ttattagcgg ttttcgagcg atattaaggt 1500
ttcgggcgtt tttcggaggt tttttttgtt gcgtaggcga ttttacggtt tcggtgtttg 1560
gcggaggttt cggtttaaac gagttcgttt gattttcgtc gttgtttgag ttcgttttat 1620
gttttggatt t 1631

<210> 187
<211> 9254
<212> DNA
<213> Artificial Sequence

<220>
<223> chemically treated genomic DNA (Homo sapiens)

<400> 187

tgggggaaat tgattttatg atttaattat ttttatttga ttttattttt gatatgtgag 60
gattattata atttaaggtg agatttgagt ggggatataa ttaaattata tgagtggata 120
tgtaatatga gtgagaaata attttgtgat tgtaatttag taagattttt ggatggttaa 180
tatagtataa tttagagtat atggaggtgt gtatttttag ttttttgatt ttcgggtatt 240
tagtaggatt gtattttttt gtatttttta agtaaggtat ggttatgtga ttaattttag 300
ttaataatat gtaaatataa taaaaagtgt tatttttagg agggagtttt taaagttaat 360
gtgagtttat tatgtttttt tttttttgtt aaattgatgc gatgattgtt gaagtatgta 420
ttaagatgaa atttttttta gattaggttt ttaaatgagt atattaagta gagtttcgtg 480
aagttgaata tgtagtatga gtaaaatata aaatttattt ttaagttatt gaaattttag 540
gattgtttgt tatggtagta taatttaaat tattttgatt tatataaaaa ttggttttta 600
gatatgggtt agtgtaataa taataataag tataataata ataaaattaa ataattattt 660
agagggcgat aattaagaat attgttatta gaagttgaag ggatggtgat ttgtttggta 720
aatatattat ttgtgataat gtagaaggta gattatgaat ttgatgaatt aaagaaaagg 780
attgtaaaat agagagtgag tagtgtgtgt tggtggttgt tggatgtatt ttgaaagtta 840
ttataagtta tttaaagatg aatttagaaa aatttggttt gtttgtaatt aggaatgaaa 900
tggaatagag aaaatttata aatttaggga tttatatcgt tagaagaggt aattgttttt 960
tagtttcgat tggtaaaata taaaattaag aaatattttt agtaagaaag gatttggaag 1020
aaattaagtt ttgaagtaag gattaagtta taaagtatga atttattatt aaaattttag 1080
gataggaggt cgaggaggac ggattatttg aggtcgggag ttcgagataa gtttgattaa 1140
tatggagaaa ttttgtcgtt tttaaaaata taaaattagt cgggcgtggt ggcgtatgtt 1200
tgtaatttta gttatttagg aggttgaggt aggagaatcg tttgaattcg ggaggtagag 1260
gttgtagtga gtcgagattg tgttattgta ttttaattag ggtaataaga gtgaaatttc 1320
gttttagaaa aaaaaaaaaa aggattaatt aagattttat ttagtatata tttttaattg 1380
gataaaatgg ttttgggaaa aggaagaaag gtttaatttt tttattaaaa tttaataagt 1440
ttaaagtgtt taaaattaag tgaagagtaa gagagtgaga cggtaaaaga gagatagagg 1500
agttaatagg taggagagta ggaaagtaaa gagatatggt aaattaaaga agtatgtttg 1560
gaaaagaatt ttgtggttat tgttatatgg aatattttat attagataga taagaagttt 1620
atttatatat atttaagatt taatgttatt ttgtattttt ttaattttta aaagtttatt 1680
taaaagtata ttttttaatg tttgttttag ttgtggaaaa ggaagatttt tcgagggtaa 1740
atttaggagt tatggaaaat aatgggtagg gtagtttggt gttgtgattg gatgggtttt 1800
taaggttgtt ttttttgaat aaagaatgag ggaattttat taggggaagg agagtagatt 1860
aaatatttgg tgaatagaaa gggagataga gtttttagtg ttatttttta aatatttttg 1920
gagagttttt tgagatattt ggtaggatta tagtgattta aagaggtatg gttatgtgat 1980
tgttttggtt aatgatgtat gggtagaagt gatgtgtgtt ataagtggat gttttgatag 2040
ttatgatgtt tcgagttttt tttttatagt taggatagtt gtgaaaaggg gtattaatac 2100
gaggtttttt ttagtttgag ttttttagtg attataatga gtagagttat tttgttaatt 2160
tataacggtt atgtagtaga aatgattaat ttttattgga ataagttatt gatatgttag 2220
ggttgtttgt tagggtagta taatattgtt ttttttgata gaattgggag tgagagataa 2280
ggttggagag agaaggagtt ggattgtgaa aagttttttt tttaaggtat aggaatcgga 2340
atttttttga ttgtaaggaa aagttaatga aggtttttaa gtaggggcgt taatatgaat 2400
agatttgtta tttttttgga tgaaatatgt tattttttaa tataaggaga agattagttt 2460
attaaattat atgaggtaga gaggaatata tagaatttta ggtaaaagat atatatttat 2520
gtaaagtttt aagttttgaa atttttaaat ataagagttt tgaacggagt tatagttaga 2580
ggattaggta agaaaatttt ttttatgtta taagcgttat tttttttgta gataattaat 2640
attttataag aaagtttgtt agagtagaga aaatataaag gaatgtaata tagatttgag 2700
gttaattttt agttgtttgt gtgattttaa taaagatatt taattttttt gggtttattt 2760
ttttatatgt taaatttgga taataatata tgttttttaa gatagttgtg gggagaaaat 2820
agcgtattta aattgtttag gagaatgttt ggtagataat aagtgtttaa tatatgaaat 2880
tgatttttat tattgttatt aaaaaatatt tagaatatta attttttgaa tataatagaa 2940
aatgatttag ggtaatagat agaggagaat gttttttttt tgaggaagta attggatttt 3000
gttattattg tatatttatt tattttattt tttttttagt ttagtgtttg gtttatagta 3060
ggtttttagt tattttttgt agattagtga aagttaggtg agtgttcgga gtgaagaaaa 3120
gttggtaggt tttttattga tattagttgt tgttggtttt tgaatattta aagtcgtaaa 3180
tattttaggg ttgggggtaa tgaatttaag gttgaatttt aagtttagaa gtagcgaagt 3240
ttgaatttag aatttaggaa tttaaattgt tgtaggttta attttaagtt ttagttttag 3300
atagaggttt gggaaagatt tgatttttaa ttcggttttt tttttttttt ttttttcgat 3360
gttttttata ggaaagtata tttggttttg ttaaatcgta tttttatatt ttggtatttt 3420
atttaggttt tgcgaggatg gaaattgcga ggtaggggag ggaagcgggt tgtttggtta 3480
ttattttttt agtgttgtag gcgattttgt tatattaatt tttggtagtt tagtgaggtg 3540
gacggtatcg gttttatttg tagatgaggg aaatgaagtt cggaggagtt tcgtgatttg 3600
tttgttttat attgtggtag tttggttacg aaagaattag gattttcgat ttcgggattt 3660
tttttattat atattttcgt ttttaagggg tggggggcgg ggggagaata aaataatcgc 3720

ggaaaaggaa ttatttatat gtgtttagcg ttttttagag gttatttagg atatgcgttt 3780
attattcggt cgggagtgta gagatttgaa gtttaggttt tatttcgggt ttcgagtatt 3840
attgcgtgat tttatgcgag tgttcgtcgt tttttgggtt tgttttttcg gaagtaattc 3900
ggcgcggatg gagtgtgtgt gtgcgcgcgc gcgcgcgtta tgttgtgcgt gttgtgttaa 3960
gcgtgtgcgt gttgttcggg ggcgggaggg ggagtagatt aatatcgggg tttttcgagt 4020
ttcggatcgt ttatacgttt gtttttattt ggattttgtt atttattaat tgcgtttatt 4080
ataaaaattg ttttttcgag gtaaagttgt gaattttcgc gttttttttt ttacggtttc 4140
ggaggatgaa gtggggtgta acggagattt agttgagcgt ttagtttcgg gtaatataaa 4200
tttttcggtt tttacgagta gttagacgat ttcgcggatc gtcgtttttg aatttgatcg 4260
agatgtaaat ttcggagtgt ttttaacgtg gggggtcgat tttcgggaga tcgtttttaa 4320
atttaagttt ttttaggttc gtttttattt gggattttat agagttattt taagggcggt 4380
attttcgttt tttcggaagt gcgggggggt ggtagcgtat ttggattttt agtttttagt 4440
ttcgcgcggt ggcggtcgtc ggtggatgat ttcgggtttt ataagtgggg aaataataat 4500
tatttttcgt tcgtattttt ggtttaaaat aattggttag gttttttggt ttttcgggtt 4560
tttgtatttt tcgtattttc gttcgtagtc gtgaatttga gttttttttt attagaggtt 4620
gcgagcgttc gttcgttcgc ggtagttatc gttattagat agttaaattt taagacgtta 4680
gtttataatg tatcgggcgg gtcgggaaaa acggttcggg gaggggatcg gggaagagag 4740
ggtcgagagg cgtgcggtag gggggagggt aggagaaaga agggttcgat tgtaggaggg 4800
tagcggagta ttattttatt tcgtgagttt tcgcgggttt agagaagaat tttttagggt 4860
ggagttttta tggtgacggg cgggttcgtt tttttgagag cgacgcgagt taatgggaag 4920
gttttggggt gatattatgg gttattttta ggggttgatt ggtagtagat aagtgttgag 4980
ttcgggttgg ataagggttt agagttgtat tgagtgtggt tgaagtagcg aggcgggagt 5040
ggaggtgcgc ggagttaggt agatagatag atatagttag ttagttaggt cggtagtata 5100
gttcgaattg taaattttat ttttttttta tttttttttt aattgtttag agttagcgtt 5160
tgtggttttc gggttggtgt ttcgggagtg tttagagagt ttggttttta gtcgttttcg 5220
ggaggagagt tttgttgttt aggcgttgtt gatagcggcg gaaagtagcg gtatttacgc 5280
gttcgtcggg ggaagtcggc gagcggttgt agtagtaaag aattttttcg gttgggagga 5340
tcggagataa gtggtagagt ttcggagtta atttttgtaa gttttttttg cgttttaggt 5400
ttttttacgg cggtaaagat tagaaggcgg cggagagtta cgtaagagaa gaaggacgtg 5460
cgtttagttt cgttcgtatc ggttgttgaa tttgggcgag cgcgagtcgc ggttgtcggg 5520
cgtttttttt ttttagtagc ggaggagggg ataagtcgtc ggagttcggg cggttaagat 5580
tcgtcgtcgg tcggttattg tagggttcgt attgattcgt ttcgcgggga gagtcgttgt 5640
tttgggaagt gagttcgttt gcggatttcg aggaatcgtt gcgtacgaag agcgtttagt 5700
gagtgatcgc gattttttaa agtcgggtag cgcgcgcgag tcgataagta agagtgcggg 5760
aggtatttta attaattttg cgttttttgg agcgagttgg tgaggagggc gtagcgggga 5820
cgatagttag cgggtgcgtg cgtttttaga gaaatttttt ttgttaaagg tttcgggggg 5880
cgcgggtgtt tttcgtttgt tatagttttg ttgcggtttc gaaatttgtg cgcgtagttt 5940
aaattaattt tacgtgaagt gacggattgt tttatgattg taaagatgga aacgattttt 6000
tatgacgatg tttttaacgt ttcgtttttt tcgttcgaga gcggatttta tggttatagt 6060
aattttaaga ttttgaaata gagtatgatt ttgaatttgg tcgatttagt ggggagtttg 6120
aagtcgtatt ttcgcgttaa gaattcggat ttttttattt cgttcgacgt ggggttgttt 6180
aagttggcgt cgttcgagtt ggagcgtttg ataatttagt ttagtaacgg gtatattatt 6240
attacgtcga tttttattta gtttttgtgt tttaagaacg tgatagatga gtaggagggt 6300
ttcgtcgagg gtttcgtgcg cgttttggtc gaattgtata gttagaatac gttgtttagc 6360
gttacgtcgg cggcgtagtc ggttaacggg gtaggtatgg tggttttcgc ggtagtttcg 6420
gtggtagggg gtagcggtag cggcggtttt agcgttagtt tgtatagcga gtcgtcggtt 6480
tacgtaaatt ttagtaattt taatttaggc gcgttgagta gcggcggcgg ggcgtttttt 6540
tacggcgcgg tcggtttggt ttttttcgcg taattttagt agtagtagta gtcgtcgtat 6600
tatttgtttt agtagatgtt cgtgtagtat tcgcggttgt aggttttgaa ggaggagttt 6660
tagatagtgt tcgagatgtt cggcgagata tcgtttttgt tttttatcga tatggagttt 6720
taggagcgga ttaaggcgga gaggaagcgt atgaggaatc gtatcgttgt ttttaagtgt 6780
cgaaaaagga agttggagag aatcgttcgg ttggaggaaa aagtgaaaat tttgaaagtt 6840
tagaattcgg agttggcgtt tacggttaat atgtttaggg aataggtggt atagtttaaa 6900
tagaaagtta tgaattacgt taatagtggg tgttaattta tgttaacgta gtagttgtaa 6960
atattttgaa gagagatcgt cgggggttga ggggtaacga agaaaaaaaa taatatagag 7020
agatagattt gagaatttga taagttgcga cggagagaaa aaagaagtgt tcgagaatta 7080
aagttaaggg tatttaagtt ggattgggtt gcgttttgac ggcgttttta gtgtgtacga 7140
gtgggaagga tttggcgcgt ttttttttgg cgtggagtta gggagcggtc gtttgcgggt 7200
tgtttcgttt tgcggacggg ttgttttcgc gcgaacggaa cgttggattt ttcgttaata 7260
ttgattaaga attgtatgga tttaatattc gattttattt agtattaaag gggggagggg 7320
gagggggtta taaattgtaa tagagattgt agattgtttt tgtagtattt tttaagaata 7380
taaagcgggg ggagggttgg ggaggggcgg taggagggag gtttgtgaga gcgaggttga 7440
gtttatagat gaattttttt tggtttgttt tcgttaattg tgtatgtata tatatatatt 7500

ttttaatttg atgaaagttg attattgtta ataaatagtt ttatgttttt gtaagttatt 7560
ttttgtttgt ttgtttgggt attttgttta gtgttgtttg taaataagag atttggagta 7620
ttttgagttt attatttgta ataaagtata taattttttt atgttttgtt tttgaaaatt 7680
ttagaaagga tatttaagaa aatataataa attattggaa agtatttttt taattttttt 7740
tttgtattat ttgtagatat tagttattta ggtggagttg aaagagttaa gaatgtcgat 7800
taaaattatt tttagtgttt tttattatta agtagtaaaa attgtttttt attagatttt 7860
agaaataaat gtatttgatg tatttgatgt tatggttagg ttatattttt ttttttttag 7920
ttatttatat ggaattgttt attaaaggat agtgcgatgt tttaggaggt tggaggaagg 7980
ggggttgtag tggagaggga tagtttattg agaagttaaa tattttaaag tttggattgt 8040
attaagtggt atgtgttgtg attatttata atgttagtag aaattttata ataggtgttt 8100
atttttaaag taggaattgg tggtagattt tataaaagat gtattttttt aatttggaat 8160
tttttttttg ataattttta gataaaaaga tggtttttgt ttatgaatat ttataatagt 8220
atttttgtta taataaatgt atttaaatat taataataga ttttgaattg ttttttttta 8280
ttattttttt ttttttaagt tatatattga agtttttatt tttagttgtt gaggttaata 8340
ttgttgtaat ttgaagtgat ttgaaaatta agaagtaaag aatttaggaa gagttaaatt 8400
agaagttgtg atttgaatga aatagttttt taattttatt aatttttttt tttttttttt 8460
atttttattt ttttttttat tattttattt ttaaataaat ttttatgttt atggaaatta 8520
atagtttagt attttgggtt tgtttgtagt ttatttttta atttgttttt aaaataatta 8580
attttgttta aatatagaat aagttttaaa agatagtaat ttaatattat ggaaaagatt 8640
tataagagaa aatatgaatt agtattaatt taatgattat atgcgtaaat taaattttag 8700
ttttggggaa atgggaaatt tgaattttat ttatagatat aatttaggag attaataagg 8760
tagtgtttaa taagggaata attttttgtt tggttgtttt ttatattaag agttagtgtg 8820
attattaggt agtaaaagta atttgatatt taggaatatt ttataaatta ttaattgagt 8880
tagggttgtt tgtttattag tagtgtttta gtatttagta ggtttgggag tgttaatatt 8940
ttgtttgtgg ttgtaaattt aaatatataa agagtttttt tttaggagta 'tatttaggag 9000
tggaagagat tggataggtt tgttagtttc ggattttttg taataatatt ttttgaataa 9060
agggttattg ggagtagagt ttagtttgtt aaattgaatt tgtcgatgtt agtaattcgg 9120
aaggtgagat gattttgatt atatgtagaa atagtagagg gataataaag tattttgggg 9180
tggggttttg tggaagttaa ataagagtta attagaaaaa ataagtaggt tggggaatta 9240
attatttgta aagg 9254

<210> 188
<211> 9254
<212> DNA
<213> Artificial Sequence

<220>
<223> chemically treated genomic DNA (Homo sapiens)

<400> 188

tttttgtagg taattaattt tttagtttgt ttattttttt tagttggttt ttgtttagtt 60
tttatagagt tttattttaa aatgttttat tgtttttttg ttatttttat atatggttaa 120
aattatttta tttttcgggt tgttgatatc ggtaaattta gtttggtaaa ttgggttttg 180
tttttagtaa ttttttgttt aagaagtgtt attataaagg attcgaagtt aataagtttg 240
tttaattttt tttattttta gatatatttt taaggagaaa ttttttgtgt atttggattt 300
atagttatag gtaggatatt agtattttta gatttattgg gtattagaat attgttaatg 360
agtaaatagt tttgatttaa ttagtggttt gtgaaatatt tttggatatt aaattgtttt 420
tgttgtttga taattatatt ggtttttggt atgaaaagta attaagtaaa aaattgtttt 480
tttgttaagt attgttttgt tgatttttta agttatattt gtaggtagaa tttaagtttt 540
ttattttttt agagttgagg tttgatttac gtatgtaatt attgagttag tattagttta 600
tgtttttttt tgtgaatttt ttttatagta ttagattatt attttttgag atttatttta 660
tatttaaata aggttgatta ttttagaaat aaattaagaa ataagttata gatagattta 720
aaatgttaaa ttgttagttt ttataggtat aggggtttat ttgggggtga ggtagtagag 780
agagagatag agatggagaa agagggaaag aattaatgaa attgggagat tgttttattt 840
agattataat ttttagtttg atttttttta aattttttgt tttttaattt ttaaattatt 900
ttagattgta gtaatgttaa ttttagtaat taaaaataaa aattttagta tataatttgg 960
gaagaaaaaa gtagtaaagg gaagtaattt aagatttgtt attggtattt gaatatattt 1020
attgtgataa gaatgttgtt ataaatattt ataagtaaag gttatttttt tatttaggaa 1080
ttgttaaaga gaagatttta aattggaagg atatattttt tgtaaaattt gttattaatt 1140
tttgttttga gaataagtat ttattgtaaa atttttatta atattataaa tggttatagt 1200
atatgttatt tgatataatt taaattttga aatgtttgat tttttagtgg gttgtttttt 1260
tttattgtaa tttttttttt tttagttttt tgaaatatcg tattattttt tggtaagtaa 1320

ttttatatag atagttgggg gaggaggagt ataatttgat tatagtatta ggtatattag 1380
gtatatttat ttttaaagtt taatagagaa tagtttttat tgtttaatag taagaagtat 1440
tgagagtgat tttaatcgat atttttaatt tttttaattt tatttagata gttagtattt 1500
atagatgatg tagaaaagag gttaggggag tattttttaa tagtttattg tattttttta 1560
aatatttttt ttggaatttt tagaaataaa atataaaaaa attatatatt ttattataaa 1620
tggtaaattt agagtgtttt aaatttttta tttataaata atattgggta ggatatttaa 1680
ataaataaat aagaaataat ttataaaggt atgaagttgt ttattgatag taattagttt 1740
ttattaaatt aaaaaatata tatatgtata tatatagtta acgaaggtag gttagaaaga 1800
gtttatttgt aggtttagtt tcgtttttat aaattttttt tttgtcgttt ttttttaatt 1860
tttttttcgt tttgtgtttt taaggagtat tatagaagta atttatagtt tttattgtag 1920
tttgtaattt tttttttttt ttttttttaa tattgaatga gatcgaatgt taggtttatg 1980
tagtttttgg ttaatgttaa cgaaaagttt aacgtttcgt tcgcgcgggg atagttcgtt 2040
cgtaaagcgg ggtagttcgt aggcggtcgt tttttggttt tacgttaagg gagggcgcgt 2100
taagtttttt ttattcgtgt atattggggg cgtcgttagg acgtaattta gtttaatttg 2160
gatatttttg gttttagttt tcggatattt tttttttttt ttcgtcgtaa tttgttaagt 2220
ttttaagttt gtttttttgt gttatttttt tttttcgttg ttttttagtt ttcgacggtt 2280
tttttttaaa atgtttgtaa ttgttgcgtt agtatgagtt ggtatttatt gttaacgtgg 2340
tttatgattt tttgtttaag ttgtgttatt tgttttttga gtatgttggt cgtggacgtt 2400
agtttcgagt tttgagtttt taaggttttt attttttttt ttagtcgggc gatttttttt 2460
agtttttttt ttcggtattt ggaggtagcg atgcggtttt ttatgcgttt ttttttcgtt 2520
ttgattcgtt tttgggattt tatgtcgatg ggggataggg gcggtgtttc gtcgggtatt 2580
tcgggtattg tttgaggttt tttttttagg gtttgtagtc gcgggtgttg tacgggtatt 2640
tgttggggta ggtggtgcgg cggttgttgt tgttgttggg gttgcgcggg aaaggttagg 2700
tcggtcgcgt cgtaggaggg cgtttcgtcg tcgttgttta gcgcgtttgg gttgaagttg 2760
ttgaggtttg cgtagatcgg cggttcgttg tgtaggttgg cgttgaagtc gtcgttgtcg 2820
ttgttttttg ttatcgaggt tatcgcggga gttattatgt ttgtttcgtt gatcggttgc 2880
gtcgtcgacg tgacgttggg tagcgtgttt tggttgtgta gttcggttag ggcgcgtacg 2940
aagttttcgg cgaagttttt ttgtttattt gttacgtttt tggggtatag gaattgggtg 3000
ggggtcggcg tggtggtgat gtgttcgttg ttggattgga ttattaggcg ttttagttcg 3060
ggcgacgtta gtttgagtag ttttacgtcg ggcgaggtga ggaggttcga gtttttggcg 3120
cggaggtgcg gttttaggtt ttttattggg tcggttaggt ttagggttat gttttgtttt 3180
aggattttgg ggttattgta gttataaggt tcgttttcgg acgggaggaa cgaggcgttg 3240
agggtatcgt tatagaaggt cgtttttatt tttgtagtta tagaatagtt cgttatttta 3300
cgtgaggtta gtttgggttg cgcgtataag tttcggggtc gtaatagggt tgtggtaagc 3360
gggggatatt cgcgtttttc ggagtttttg atagggaaag tttttttaag agcgtacgta 3420
ttcgttggtt gtcgttttcg ttgcgttttt tttattagtt cgttttaggg agcgtagggt 3480
taattaagat gtttttcgta tttttatttg tcgattcgcg cgcgttattc ggttttgaaa 3540
agtcgcggtt atttattgag cgtttttcgt gcgtagcggt ttttcggagt tcgtaggcga 3600
atttattttt tagagtagcg gtttttttcg cggagcggat tagtgcggat tttgtagtgg 3660
tcggtcggcg gcgggttttg gtcgttcgga tttcgacgat ttgttttttt tttcgttgtt 3720
agggggaggg ggcgttcggt agtcgcggtt cgcgttcgtt taagtttaat aatcggtgcg 3780
agcgaagttg agcgtacgtt tttttttttt tgcgtggttt ttcgtcgttt tttggttttt 3840
atcgtcgtgg agaagtttaa gacgtaggaa aggtttgtaa aagttggttt cgggattttg 3900
ttatttgttt tcggtttttt tagtcgggaa agttttttgt tgttgtagtc gttcgtcgat 3960
ttttttcggc gggcgcgtgg gtatcgttgt ttttcgtcgt tgttaatagc gtttgggtag 4020
tagggttttt ttttcggggg cggttggaga ttaggttttt tggatatttt cgaaatatta 4080
gttcgggagt tataggcgtt agttttgggt agttagagag aaggtgaaaa gaaaataaga 4140
tttgtagttc ggattatatt gtcgatttgg ttggttggtt gtgtttgttt gtttgtttga 4200
tttcgcgtat ttttattttc gtttcgttgt tttagttata tttagtgtaa ttttgagttt 4260
ttatttagtt cgagtttaat atttatttgt tattagttaa tttttaaaaa tagtttatga 4320
tgttatttta aggttttttt attggttcgc gtcgttttta ggggggcggg ttcgttcgtt 4380
attatggaga ttttatttta gaagattttt ttttgggttc gcggaggttt acgggatgag 4440
gtaatgtttc gttgtttttt tatagtcggg tttttttttt ttttattttt ttttttgtcg 4500
tacgtttttc ggtttttttt ttttcggttt ttttttcggg tcgttttttt cggttcgttc 4560
ggtgtattgt gggttgacgt tttggggttt gattgtttag tgacggtggt tgtcgcgagc 4620
gggcggacgt tcgtaatttt tgatggaggg gggtttaagt ttacggttgc ggacggggat 4680
gcgggggatg tagggattcg ggaggttagg gaatttggtt agttgttttg ggttaggggt 4740
gcgggcgagg ggtggttgtt gtttttttat ttgtggggtt cgaagttatt tatcggcggt 4800
cgttatcgcg cggggttgga ggttgagaat ttaagtacgt tgttattttt tcgtattttc 4860
gggggggcgg gaatatcgtt tttaaggtgg ttttgtgaag ttttaggtgg gggcgagttt 4920
aaggggattt aagtttaggg gcggtttttc gagagtcggt tttttacgtt gagaatattt 4980
cgaagtttgt atttcggtta agtttaggag cgacggttcg cggggtcgtt tggttgttcg 5040
tagaagtcga gagatttgta ttgttcgaaa ttggacgttt agttgagttt tcgttgtatt 5100

ttattttatt tttcgggatc gtgggggaaa gggcgcgggg gtttatagtt ttgtttcgga 5160
ggggtagttt ttatagtggg cgtaattggt gggtaatagg gtttagatgg gaataagcgt 5220
gtaggcgatt cgaaattcgg ggaatttcgg tgttagttta tttttttttt cgttttcgga 5280
taatacgtat acgtttaata taatacgtat aatataacgc gcgcgcgcgc gtatatatat 5340
attttattcg cgtcgagttg ttttcgggaa aataagttta gaaggcggcg gatattcgta 5400
taaagttacg tagtagtatt cggagttcgg ggtagaattt ggattttaaa tttttgtatt 5460
ttcggtcggg tggtgggcgt atatttgggg tagtttttag gaagcgttag atatatgtaa 5520
gtggtttttt tttcgcggtt attttatttt tttttcgttt tttatttttt agggacgaaa 5580
gtgtgtggtg gaaagaattt cgaagtcggg aatttggttt ttttcgtggt taggttatta 5640
tagtgtgaag taagtaaatt acggaatttt ttcgagtttt atttttttta tttgtaggtg 5700
gggtcggtgt cgtttatttt attgggttgt taggagttag tgtgataggg tcgtttgtag 5760
tattagggag gtggtggtta aatagttcgt tttttttttt tgtttcgtag tttttatttt 5820
cgtagagttt ggataggatg ttaggatata agagtgcgat ttggtaggat taggtgtatt 5880
tttttgtgag aagtatcgag ggaggagggg gaggggggaat cgggttagaa gttagatttt 5940
ttttaagttt ttgtttaaaa ttggggtttg aagttggatt tgtagtagtt taagttttta 6000
ggttttaaat ttagatttcg ttgtttttga atttggaatt tagttttggg tttattgttt 6060
ttagttttaa ggtatttgcg gttttgagtg tttagaaatt aatagtagtt ggtattagtg 6120
ggaaatttgt taattttttt ttatttcggg tatttattta gtttttattg atttgtagag 6180
ggtaattgaa agtttattgt gagttaggta ttgagttggg gagggggtgg ggtaggtagg 6240
tatatagtga tgataagatt tagttgtttt tttaaggaga gaatattttt ttttgtttgt 6300
tgttttgaat tattttttgt tgtatttagg gagttggtgt tttaaatatt ttttaataat 6360
agtaataaaa attagtttta tgtattaaat atttattatt tgttaggtat ttttttaagt 6420
aatttaaata cgttgttttt tttttataat tgttttgaga gatatatatt attatttaga 6480
tttaatatgt gaggaaatag gtttagagag gttaagtatt tttgttgagg ttatataggt 6540
agttggaaat tgattttagg tttgtgttat atttttttgt atttttttta ttttggtaaa 6600
tttttttgtg gagtattagt tatttgtaga ggaaatagcg tttgtggtat agaagaaatt 6660
tttttgtttg attttttgat tgtaatttcg tttagagttt ttgtatttag aaattttaag 6720
atttaggatt ttgtataaat atatattttt tgtttaaggt tttgtgtgtt tttttttgtt 6780
ttatgtggtt tagtaggttg attttttttt tatgttgaga aatagtatat tttatttaaa 6840
aaaataataa atttgtttat gttaacgttt ttgtttggaa gtttttattg gttttttttt 6900
ataattagga gagtttcgat ttttatgttt tggagaaggg gttttttata atttaatttt 6960
tttttttttt agtttatttt tttattttta gttttgttag gaaaggtagt gttatgttgt 7020
tttaataaat aattttaata tattagtggt ttattttagt gaaagttggt tatttttgtt 7080
atatgatcgt tgtaggttgg tagggtagtt ttgtttattg tagttattaa agggtttagg 7140
ttgaaggagg tttcgtgttg atattttttt ttatagttat tttgattgtg gggaaggaat 7200
tcggaatatt atggttgtta gagtatttat ttatggtata tattattttt gtttatatat 7260
tattggttaa agtagttata tggttatgtt tttttagatt attataattt tattaggtgt 7320
tttaaagggt tttttaggaa tatttgggga ataatattaa agatttgttt tttttttttg 7380
tttattaaat gtttggttta tttttttttt tttggtggaa tttttttatt ttttatttaa 7440
gggaaataat tttaaagatt tatttagtta tagtattaag ttgttttgtt tattgttttt 7500
tatgattttt ggatttgttt tcggagagtt tttttttttt atagttaaag taggtattgg 7560
aaaatatgtt tttaaataag tttttaaaag ttggggagat gtagagtgat attaagtttt 7620
gaatatgtgt aggtaggttt tttatttatt tgatatgagg tgttttatgt gataatagtt 7680
ataaagtttt tttttagata tatttttttg gtttgttata tttttttgtt tttttgtttt 7740
tttgtttgtt gatttttttg tttttttttt gtcgttttat ttttttgttt tttatttgat 7800
tttgagtatt ttgggtttat tgggttttga tgggagagtt agattttttt tttttttttt 7860
agagttattt tgtttagttg aaagtatata ttgggtaaga ttttaattga tttttttttt 7920
tttttttttg agacggagtt ttatttttgt tgttttggtt ggagtgtaat ggtatagttt 7980
cggtttattg taatttttat ttttcgggtt taagcgattt ttttgtttta gttttttgag 8040
tagttgggat tataggtatg cgttattacg ttcggttaat tttgtatttt taggagcgat 8100
aggatttttt tatattggtt aggtttgttt cgaattttcg attttaggtg attcgttttt 8160
ttcggttttt tattttgagg ttttaatagt aggtttatat tttgtgattt gatttttatt 8220
ttaaagttta gtttttttta ggtttttttt tgttaaaagt attttttgat tttatatttt 8280
attagtcggg attgggaaat aattgttttt tttaacgatg taaatttttg gatttatagg 8340
ttttttttat tttattttat ttttgattgt aaataggtta aatttttttg agtttatttt 8400
tgaatagttt gtagtaattt ttagaatgta tttaatagtt attaatatat attatttatt 8460
ttttgtttta taattttttt ttttagttta ttaggtttat gatttgtttt ttatattatt 8520
ataggtgata tgtttattaa atagattatt atttttttag tttttgatag tagtgttttt 8580
agttatcgtt ttttaaatga ttgtttagtt ttattgttgt tatgtttatt gttgttatta 8640
tattaattta tatttaggag ttaattttta tatgagttag gatggtttag gttatgttgt 8700
tataataaat aattttaaaa ttttagtggt ttaaaaataa attttatatt ttgtttatat 8760
tatatgttta attttacggg gttttgttta atgtgtttat ttagaaattt agtttgaaga 8820
aggttttatt ttgatatatg ttttaataat tatcgtatta gtttggtagg ggaaagaaag 8880

tatggtgaat ttatattggt tttaaaagtt tttttttagg aatgatattt tttattgtat 8940
ttatatatta ttggttaaaa ttagttatat ggttatgttt tatttaaagg gtgtaggaaa 9000
gtgtaatttt attaggtatt cggaagttaa agagttggaa atgtatattt ttatatattt 9060
taggttatgt tgtgttagtt atttaaaaat tttattgggt tataattata gaattgtttt 9120
ttatttatat tgtatgttta tttatatggt ttggttgtgt ttttatttaa atttatattt 9180
gaattgtaat aatttttata tgttaaaggt ggggttaggt ggagataatt gaattatggg 9240
gttagttttt ttta 9254

<210> 189
<211> 8244
<212> DNA
<213> Artificial Sequence

<220>
<223> chemically treated genomic DNA (Homo sapiens)

<400> 189

ttttaaatat atttattatt tcgacgggtt ataataattt ttttggacgt taattttcgg 60
ggacgttaaa atatattagt ttcggcgggt ttttttagat ttatattatg tggtttgggg 120
ttttagatgt gttttttttt aggttttgga tagttttata tatttttttt aggttttata 180
gatttatagg ttattaattc gggtttttta attttaaaga cgaagttttt ggttcgtggt 240
ggtcgcgttg attaatttgt ttggtttttt aggatgtgga tagtgttttt tttatattta 300
ggtattgttt ttaaaataag tggaattttt tcgtataatt ttgaatatta attttagggt 360
tttttaagtt tattgttttt cgtatacgtt cgttttattt cgcgtttttt tattttattt 420
tcgcgttttt tttcgtttat tttagtatgg gatatttgtt gttgggtttc gtaaatttat 480
ttatattaat ttgggttttt taaattttat acgttgaatt ttaagttttt tatgatattt 540
agtagggttg gaaggttgga atttttagag tatgaaaatt gttttttata ttttttttta 600
aattcggtta ttatatttag agtatgttga attttttttt gaattataaa cgatttgttt 660
tttgattgtt tgagttttat aaaatggagg gattttttta gtgatttttt aaagttattg 720
agaatgttgt gggtgtgtga gttataaagt tttggggtat taacgtttat ggttttatat 780
gttgtttggt tacgaattag gttttttaag atgtagatag tgttatttag gtttttggag 840
tatattgagt agttattagg aggtgtttaa gtgtgattta ggatttttta ggttttttta 900
aattatatag ggtttttcgg gtttttttgg gttatattaa gtataaaagg attttttggg 960
tagagtttat ttttattttt gttatgttag gtgttgttaa cggttttagt ttttaaatat 1020
ttcgagtatt ttttttgtat tataatatat tgattattaa ataatttttt gggttttagg 1080
agttttataa taagaatttt gtttttttaa aatttaggta ttggtttgaa attttaacgg 1140
ttaggattat attggatttt ttttttggtt ttttatattt ggatgttttg gacgttgttt 1200
tttaggtttt ttaggaatat ttagtattgt tttcggatta taggatagta ttgatttttt 1260
gggttttaaa taatttattg agttatttta aagttaagta atattttttt taaatatatt 1320
gtatattttt tattataaaa tttgaaaatt tttaagtttt aagatttagg aattttgaat 1380
tttttttttt taaaatgtat atatggattt ttaagttttt taaggatttt gagtaatttt 1440
tttagatttt tagatttaaa aacgattttt ttggagtttt taaattgcgg tattgttttt 1500
tagtttttta aagtaaattg agattttttt ttttttataa aataattgag gttttttttt 1560
tttttaatat tgatttatga gttttttgat tttatggttt ttgttttggg tttttttata 1620
tttagaaaat gttttatgga tttttaaagt atattaaaaa atgtttttgg gttttaagaa 1680
attttaggta tggaaaaatt tgcgatttat aagtttttta gttattaatt aggtttttag 1740
aaatttagat attaaatttt tattgggtaa tttttatgtg ttttaaaaat ttgaaatgtg 1800
ttttattggg gtttttttaa atgtagacga tatttaggaa aatatatagt ttttttttta 1860
tttattaaaa ataagttaat ggaaattatt taaaaattag tatagaaaaa taatattgat 1920
tttatatttt tttatttttt attttgtttt ttttaaatgt attgattata ttttaggttt 1980
ttttaaaatt tagatagtgt ttttttttat ttttgaaggg tgttaaaatt tttttttgaa 2040
gttatagtaa ttatgaaggt tatttttttt tcggttgttg ttagcgttta ggttattaat 2100
ttatattttt aagatgtgaa aattaatttt agtttttttt taatatatta agaatgtgtt 2160
tggattttta aaatgtgttt tttgttttta tttgttaatt ttacgtaata tggttttacg 2220
gtaaaatttt taattttata tggagaattt tttttaatta ttttttttta taaattggtt 2280
ttttaagtta gttggttttt atttgagatt ttttttttta tgtttttaat tgtatggagt 2340
aatttttttt attttttaaa tttgtaattt atttttttgg agtttcgagt ttagttatta 2400
attacggttt ttatattaac ggagttttcg ggtttttttt ttttaggata tttattcgtt 2460
aagttcgtaa ggtagaaaga attttgtttt gcgtttttta aaatttgggt attgttttcg 2520
gttcgtcggt tatttattgt agttttttta atttcgcgta tagcgggtat tggtttcggg 2580
tttttttgtt ttttacgaag tttttagagt aattcggatt tgggaaattt ttttttagcg 2640
ttgtttaaat atatttgggt cggtcgcgcg tttttaggac gtggataggg agggtttttt 2700

cgtgtttagg aaagcgatcg ggtattgttt ttagtttttt ttaaatttgg gtattgtttt 2760
cgggtttttt aacggattgg gcgttgtttt cggatattga ggattggttt cggggtttcg 2820
tttattttta gtagcgttta tcgtttgtta tagagcgttc gatcgttcgt tgtttgagtt 2880
tttggtgcgt tcgcggacgt agtttttagt ttcgcggtga gtttttcgtc gcgtcgattt 2940
ttttcgtttt tgcgtttttg atcggttttc ggttcgtatt tgttgttgtt tcgtcggtgt 3000
tggcgttcgt tttcggttgt cgtcggggag gtcggcgtgg ggcgcgggat acggttgcgg 3060
atttgcggtt ggcggttgcg ttcgtttttg ttgggcgttt cgaaattcgc gttattttcg 3120
tttgtttatt gtaaagattt tattcgtggg gaaagcggtt ggagggtttt aaagtggggc 3180
gggtaggggg ttggggcgag ggacgcggag gagaggcgtt ttcgtcgggc ggtaaagtgt 3240
ttttagttcg cgggtttagg atttcgtcgg gaggcgcgcg cggagcgcgg gcgaagtgat 3300
tgatggcgga gcgagggggg cgaggggggt taggggggcg cgagatttcg tcggcggttt 3360
tttttttttg gttaggttta ggcggcgggg ggttggcggg gtgcgggatt ttgtgcgtgg 3420
tttttgattt ggtaaaaatt atagtgtttt tttatatcgt ttaaattttt taggagatgg 3480
tttttttaga tttttaaatt atcgtggtgg ttttcgagat cgaattcgcg tttatgtaag 3540
tttaacgtat tgaggacggg gtaattatta tttagatatt ttgggtgggt cgtaaaggcg 3600
agttatttag acgtatttcg gtgagttcgg ttatgtaggt aggatttgag ttgtgttttt 3660
cgttttgatt tttttttttt tggcggtcgg agttttcgta ggttttaagt ttggtttaga 3720
ttcggttcgg cgtagtcggt ttttcgcgcg tttcgtattt ggcgggggtt tcggggtttc 3780
ggcgcggtat cggggggcgt tcgggatttg gttgaggttt taagcgtcgc gtggtcggtt 3840
ttttttgttg gggtaggtgg cggttgcgcg tttcgttcga gtttaggggt ttttttagtc 3900
gtaataatta gtaaggattt ttcgatttag ttttaagtta tttgtatttg tatttagacg 3960
gggcgtattc gtagtgtagt tttttggtgg ggcgttggga gttcgtttgt ttttgtttgt 4020
tcggagattt tagtttacga gtataggtcg ttcgggtatt ttagaaattc gggatggggt 4080
ttttgaattt tttagaacgg gtatttagta tggttttggc gttttgcggt tttttgtttt 4140
ttatttagtt tcgttttcgc gtatttttta gtttttgcga tcgtcgtttt ttttttcggg 4200
gttttagggt ttttagttcg tatttttcgt ttcgtttttg gttcgggttg cgggggcggg 4260
tcgggggcgg ggcgagggtt tcgcgggcgt ttattggcgc gggcgcgagg ttagcgaggt 4320
tcgcgcgggt tttgggtcgc gggttggcgc gattataaga gtcgggcgtg ggcgttcgta 4380
gttcgtttgt ttttcggcgg agttgcgtga ggttcggtcg gtttcggttt ttttttttcg 4440
gtcgttttcg ttttttggtt tacgtttgtt cgcgttttgt ttattagcgt ttttatcggg 4500
taaggcggtt tcgcgtcgac gtcgttcgtt gtttcgttgt tgattttcgt ttcgggcgtc 4560
gttcgcgggg tcgcgtttcg tcgggtttgc ggatttttcg tcgttttttt tttatttatt 4620
ttaatttttt ttattttcgt ttcgttcgag gaggcggttt ttttcgtagg tagttcggtt 4680
cgtaggtcgt cggcgttgtt atttttttcg cgtttttttt ttagtttttt tttcggcgcg 4740
tagtttcggg tcgttttttt tttcgcgttg cgtttcggag cggtttcggt gtcgttatcg 4800
tttgtttttt tttcgaggtt cgggttcgcg acggtagagg gtttcgtcgg tttaaatcga 4860
gttgggcgtt cgcggttcgg gtgtagtttt tatttcgttt tttagttatc gtttttttcg 4920
gtttttcgac gtggcgtttt ttttttcgtt tttttgtgtt tttcgcgttt tttttggcgt 4980
ttggtttcgg ttttcgtttt ttttttcgta attttttttt cgtttttttt cgtttttttt 5040
agtttttagt tttcgatttt tttttttttt tacgttcgtt ttttcgtttt cgtcgaatta 5100
aagtggatta attatacgtt ttttgttttt tttcgtgttg ttttttttcg ttgtgcgttt 5160
gttcgttttt cgttgttttt tttttttttc gttttttttt cggttttttt tttttacgtt 5220
tattttgttt tttttattat ttttgttttt ttttattttt gatataatag ttgattttat 5280
ttttcgatat tttttttttt tcgaaaagta taatatttgg ttcgttttag ttcgaagata 5340
gttcgttttt tttggataat tagacgaatt tttttttttt ttttaaaaaa aagttatttt 5400
ttcgttttgt ttcgtcgtat attcggtttt cgcgattcgg ttagagcggc gttggtagag 5460
gagtgttcgg taggagggtt aacgttcgtt gttcggtttg cgatacgtag tagggaggtg 5520
ggcggtagcg tcgtcggttt ttaggtaagc ggcgtgtgcg ggtcgggtcg gggtcggggt 5580
tggggcggcg cgggtttgcg tcggacgttc ggtttttttt tcgttcgttt tcggttcggg 5640
gtttgcgggg ttcggcgggg cggttgagtt cgggggggag gaggaggagg aggaggagga 5700
cggacggttg cgggtttcgt tttttgcgcg gagtttcgcg tttattttgg cggcggagtt 5760
gggggtgggg tgggggcgtc gggaagggtc gagggaggtg tgaggtgttt gtaggggcga 5820
tttttcggtc ggtttgtggg tgtagggggt gtcgttttat atgtgtgatt cgtgttttgc 5880
gggttttggt tttcggggtg ttgggtaacg aggaggggcg cggagtcgta gaagtttatt 5940
ttggtatgtt gacgcggtgt tagcgagatc gcgagaggaa gacgggggtg ggcggggtta 6000
ggatggagag gggtcgagtt ggtaggagtt atggtagacg ttatattcgc gatatttttt 6060
ttatattttt tttggttttg ttcgtaatat ttttaaatag gagtttcggg agagggggag 6120
aggggttgtt ggtttgaggt taagaagggt agagttttcg attcggagag aggtcgcggt 6180
ttttgtttag tgggtagcgt ggaagttttt atataaggag gtgggaagga gatttttttt 6240
ttttttattg ttttgtgtag agatgagtcg ggggtgtagg atgggagttt atggtatttc 6300
gttacgggat ggtttagggt tttcggttgg gggtgtagga gagaagagat tggttgggag 6360
gagggagagg gcgggagtaa aggcgcgggg gagtggttag tagggagagg ggtggggggt 6420
agggtggagt tcgggttggg aggagtcggt ttatatataa aagttgaggt attgattagt 6480

ttgtaaattg gatattagtt ttttttgtga aagagatttt tagttttttt tttttttttt 6540
tttttttttt ttttttgttt tagcgagttt tttgttgagt tgtaggtaat tagggttgtg 6600
gagtgaggat tttcgttgtt attttatttt agtttgaggt agggtagtag ggggtacggt 6660
ttacgtttgg gtttcgggtt ttgtagtcgt tagttcgttg tttttcggat agtatttttt 6720
tttttttttt ttttttgttt ttgtttttat ttggtttttg tttttttatt tgtttttttt 6780
ttttttgttt ttttttttcg gttttttttt tgtttagttt agggtttttt ttgggttttt 6840
atttgtttcg tatcgttgta tgttttttgt tttgtttttt gtcggttttt tgattcggat 6900
ttttaagttt agagtggtgg ggtttgttgc ggaagcgcgg cgagggttag agtggttagt 6960
tggcggagtg tgtttttaga atttggaagg gggtggtaga gggggcggtg agaggattgg 7020
ttagggttta gttaaggaga tgattaagga ggtttttaga ttttcggcgt agttgtttat 7080
tagtttttag agagggtatg taaagttgtg tttttgtttt attgtttgtt tagtcgttta 7140
tataatttat tgtattaaaa atttttttgg gtttgcggag cgaaggttgg ggttgttcgt 7200
ttggagggtt ttattttttg taggggtagg gttaatttgt tgtggtggtt ttcggttttt 7260
tattttcgag tgggttaatt cggttttgtg gttttgtagt ttgtggaggg ggtgtgtttt 7320
aagattggtt ttttttttta gattgtagtt tggggaattt ggtgtcggat tttttaggtg 7380
gtttgagttg gtttttttag tttttacggg gagagtttgg tagcgtaaat agggagatgt 7440
tttggaggtt tttggtttta ttggttcgat ttgaggtttg gaaaggaggt tttgggcgtg 7500
tgtgtgtgtg tttgggggta tttaaggtag attggagttg gagaattggg tgattgggaa 7560
aataaggttt ttagagtatg ggtggcgtgg ttgtgttaat tattggagtt tttgatttag 7620
gtttggttta gttgtagatt ggaaaggtgg aaaagttagg gggaggggcg gggttggttt 7680
agtaggattg gtttgttgtt ttgagggcga tggttttttt ggattttttt tgtttagttg 7740
ggggttgtgg ggaggaaggg attggttttt ttggatgtat atgttttgta ggggtggggt 7800
tgtttgttat tttggttggc gttggaggtt tgagaagtgg cgatgtgacg ttgggttggt 7860
tttgttttta tggtgttata ggacggaggt taggtcgggt gtttagtttg ggtttttgta 7920
gttgtggatg tcgttgagtt tttgtaataa tgatcgtgga gatggttatt tttcgtgtaa 7980
aattattagt gttttttgta aatggaagga attgggtttt ttttgtgtgt ttttggacgt 8040
tttattttgt atatggtttt gcgtttttat ttcggtatta tgatttgtgt gttatttttg 8100
taataaaaat aatgtttata ggaaagtcgt gtttttaatt tttaattgaa tttgtaggtt 8160
ggtaaatttg gtttgggagg ggtatttttg gtttggggtt tggtttggtt gtttcgttta 8220
cgttattttt ttttcgtttt taga 8244

<210> 190
<211> 8244
<212> DNA
<213> Artificial Sequence

<220>
<223> chemically treated genomic DNA (Homo sapiens)

<400> 190

tttgggggcg ggagagaagt ggcgtgagcg gggtagttag gttaagtttt aggttagagg 60
tgtttttttt aaattaaatt tgttaattta taaatttagt tgaaaattga aagtacggtt 120
tttttataaa tattattttt attataaaag taatatatag gttataatgt cgaggtgagg 180
gcgtagggtt atgtgtagaa tgaagcgttt agaagtatat agaaaaggtt tagttttttt 240
tatttgtaag aagtattagt aattttatac gaggggtgat tatttttacg gttattattg 300
taggagttta gcggtattta tagttgtagg ggtttaggtt ggatattcga tttggttttc 360
gttttatgat attatggggg tagggttagt ttagcgttat atcgttattt tttaggtttt 420
tagcgttagt taagatggta gatagtttta tttttataga gtatgtgtat ttaggaggat 480
tagttttttt ttttttataa tttttagttg agtagggggg gtttaggagg attatcgttt 540
ttaaagtagt aggttagttt tgttgggtta gtttcgtttt tttttttggt ttttttattt 600
ttttagtttg tagttgagtt aggtttgggt taaggatttt aatggttaat ataattacgt 660
tatttatgtt ttagaaattt tgttttttta gttatttagt tttttaattt tagtttgttt 720
tgggtatttt taaatatata tatatacgtt tagagttttt tttttaggtt ttaaatcgaa 780
ttagtaaggt taggggtttt tagaatattt ttttatttgc gttattaaat ttttttcgtg 840
ggagttggag agattagttt aggttatttg ggaagttcga tattaggttt tttagattat 900
aatttggaag gggaggttag ttttaggata tatttttttt ataggttgta gggttatagg 960
gtcgggttaa tttattcggg ggtgggaggt cgggagttat tatagtaagt tggttttgtt 1020
tttgtagaag gtggaatttt ttaggcgggt agtttagttt ttcgtttcgt agatttaggg 1080
gagtttttga tgtaataaat tatgtgagcg attgagtagg tagtgggata gaagtataat 1140
tttgtatgtt ttttttaaag attagtgggt agttgcgtcg aggatttgaa agtttttttg 1200
gttatttttt tgattggatt ttggttagtt tttttatcgt tttttttgtt attttttttt 1260
aaattttaag agtatatttc gttagttggt tattttagtt ttcgtcgcgt tttcgtaata 1320

agttttatta ttttgaattt ggaggttcgg gttaggggat cggtagaaag taggatagga 1380
agtatgtagc ggtgcggagt aggtgagggt ttaggaaaag ttttgagttg ggtaaggagg 1440
gggtcgaagg gaaggaatag aaagggaagg agtaggtgag ggagtagaga ttaggtgggg 1500
gtaggggtag aggaaaagag gggagggggg tgttgttcga gaggtagcgg gttggcggtt 1560
gtagggttcg aggtttaggc gtgggtcgtg ttttttgttg ttttgtttta ggttggagtg 1620
ggatggtagc gggggttttt attttatagt tttggttatt tatagtttag tagaaggttc 1680
gttggggtag gaggaggagg aggaagagga ggaggaggaa gttggaagtt ttttttatag 1740
gagaagttaa tgtttagttt gtaggttggt tagtgtttta gtttttatgt gtgagtcgat 1800
tttttttagt tcgggtttta ttttattttt tatttttttt tttgttgatt attttttcgc 1860
gtttttgttt tcgttttttt ttttttttta gttagttttt ttttttttgt atttttaatc 1920
gggagttttg gattatttcg tagcgaagtg ttatgggttt ttattttgta ttttcggttt 1980
atttttgtat agggtagtga aggggggggg ggtttttttt ttattttttt gtatggaaat 2040
ttttacgttg tttattgggt aggggtcgcg gttttttttc gggtcgaagg ttttgttttt 2100
tttagtttta gattagtagt tttttttttt ttttttcggg atttttgttt ggaaatgttg 2160
cggatagagt tagagggggt gtgggggaga tgtcgcgaat gtggcgtttg ttatgatttt 2220
tgttaattcg gttttttttt attttggttt cgtttatttt cgtttttttt tcgcggtttc 2280
gttggtatcg cgttaatata ttagggtggg tttttgcggt ttcgcgtttt ttttcgttat 2340
ttagtatttc ggaggttagg gttcgtaagg tacgaattat atatgtgagg cggtattttt 2400
tgtatttata gatcgatcgg gaagtcgttt ttgtagatat tttatatttt tttcggtttt 2460
tttcgacgtt tttattttat ttttagtttc gtcgttaggg tgagcgcggg gtttcgcgta 2520
gggaacggga ttcgtagtcg ttcgtttttt tttttttttt tttttttttt ttcgggttta 2580
gtcgtttcgt cgagtttcgt aggtttcggg tcgggagcgg gcggaggaag ggtcgggcgt 2640
tcggcgtaag ttcgcgtcgt tttagtttcg gtttcggttc ggttcgtata cgtcgtttat 2700
ttggaagtcg gcgacgttgt cgtttatttt tttgttgcgt gtcgtaaatc gaatagcggg 2760
cgttggtttt tttgtcggat atttttttgt tagcgtcgtt ttggtcgagt cgcgggggtc 2820
gaatgtgcga cggggtagag cggggggatg gttttttttt gggggggggg ggagaattcg 2880
tttgattgtt tagggaggac gggttgtttt cggttggggg cgggttagat gttgtatttt 2940
tcggggggga aaaggtatcg ggaaatgagg ttagttgttg tattaaggat agaggggggt 3000
agagatagtg ggagagatag agtgaacgtg aaaggggggg gtcgaagaga gggcgagggg 3060
gagagaggat agcgagaggc gggtaggcgt atagcgggag agaatagtac ggagagaaat 3120
agaaagcgtg taattaattt attttggttc ggcgaaggcg agagggcggg cgtgaggggg 3180
ggagggagtc ggaggttagg agttgggggg gacgggaggg agcgaaggga aggttgcggg 3240
agaaagagcg ggggtcgggg ttagacgtta agaggggcgc ggggagtata gagaagcgga 3300
gggaagggcg ttacgtcgag ggtcgggggg aggcggtgat tgggggcgg agtggaggtt 3360
gtattcggat cgcgggcgtt tagttcggtt tgggtcgacg gagttttttg tcgtcgcgag 3420
ttcgggtttc gggaggggga taggcggtgg cggtatcggg gtcgtttcgg gacgtagcgc 3480
ggaaagggga gcggttcgag gttgcgcgtc ggggggaggg ttggaggggg agcgcggggg 3540
gggtgataac gtcggcggtt tgcgagtcgg attgtttgcg gggggaatcg ttttttcggg 3600
cgaagcgggg atggggggag ttgaggtaga tgaagaggag gcggcgggga attcgtaggt 3660
tcggcggagc gcgatttcgc ggacggcgtt cgggacggga gttagtagcg aggtagcggg 3720
cggcgtcgac gcggggtcgt tttgttcgat ggaggcgttg gtgggtagag cgcgggtagg 3780
cgtgggttag gaggcggggg cggtcggaag gggggggtcg gggtcggtcg ggttttacgt 3840
agtttcgtcg gagagtaggc gaattgcggg cgtttacgtt cggtttttat agtcgcgtta 3900
gttcgcggtt tagggttcgc gcgggtttcg ttggtttcgc gttcgcgtta atgggcgttc 3960
gcggagtttt cgtttcgttt tcggttcgtt ttcgtaattc gagttaagag cggggcgggg 4020
ggtgcgggtt gggggttttg ggtttcgggg gggggggcg gcggtcgtag ggttggggg 4080
gtgcgcgggg gcgaggttgg gtggggggta gggagtcgta gagcgttaag gttatgttga 4140
atgttcgttt tagagaattt aggggtttta tttcggtttt ttggggtgtt cggcggtttt 4200
gtgttcgtga gttggggttt tcgggtaggt aggggtaggc gggtttttag cgttttatta 4260
ggaggttgta ttgcgggtgc gtttcgtttg agtgtagatg taggtggttt ggggttgagt 4320
cggggggttt ttgttggttg ttgcggttga gggggttttt gggttcgggc ggggcgcgta 4380
gtcgttattt gttttagtag aaggagtcgg ttacgcggcg tttggagttt tagttagatt 4440
tcgagcgttt ttcggtgtcg cgtcggagtt tcggagtttt cgttaggtgc gggacgcgcg 4500
gaaggtcggt tgcgtcgggt cgaatttggg ttaggtttgg agtttacgga ggtttcggtc 4560
gttagaggag agaggattag ggcgggaaat atagtttaaa ttttatttgt atggtcgagt 4620
ttatcggggt gcgtttaagt agttcgtttt tgcggtttat ttaaaatatt tggataatgg 4680
ttatttcgtt tttagtgcgt tggatttgta tagacgcgag ttcggtttcg ggggttatta 4740
cgataatttg ggggtttggg gaaattattt tttggagagt ttgaacgatg taagaaagta 4800
ttgtgatttt tattaagtta aaaattacgt ataaaatttc gtatttcgtt agtttttcgt 4860
cgtttaagtt tagttaaggg gaaggggtcg tcggcggaat ttcgcgtttt tttggttttt 4920
ttcgtttttt tcgtttcgtt attaattatt tcgttcgcgt ttcgcgcgcg tttttcggcg 4980
gagttttagg ttcgcgggtt agaggtattt tatcgttcgg cgggagcgtt tttttttcgc 5040
gtttttcgtt ttagtttttt gttcgtttta ttttgggatt ttttagtcgt tttttttacg 5100

aatgagattt ttgtaatgag taaacgaaag tggcgcggat ttcggggcgt ttagtaggag 5160
cgagcgtagt cgttagtcgt aagttcgtag tcgtgtttcg cgttttacgt cggttttttc 5220
ggcggtagtc ggagacgagc gttagtatcg gcgggatagt agtagatgcg ggtcgagagt 5280
cggttagggg cgtagaggcg gaggggatcg gcgcggcggg gagtttatcg cgaagttgga 5340
ggttgcgttc gcgggcgtat taggagttta ggtagcgagc gatcgaacgt tttgtggtag 5400
gcggtggacg ttgttgaagg tgagcgagat ttcggggtta gtttttagtg ttcgggagta 5460
acgtttagtt cgttggaaga ttcggggata atgtttaaat ttgggggaga ttgggggtaa 5520
tgttcggtcg tttttttgga tacggggaag ttttttttgt ttacgttttg agggcgcgcg 5580
gtcgatttaa gtgtgtttgg gtaacgttag agagaaattt tttaaattcg agttgttttg 5640
gggatttcgt aggagataga gaggttcgaa attagtgttc gttgtgcgcg gggttgggga 5700
agttgtagtg ggtggtcggc gagtcgggaa taatgtttaa attttgggga acgtaaggta 5760
gagttttttt tgttttgcgg gtttagcgaa tgggtgtttt ggaggagggg atttcgggga 5820
tttcgttaat gtgggaatcg tgattaatga ttaaattcga gattttagaa aaatagatta 5880
taggtttggg ggatgagaga agttgtttta tgtaattggg aatatagaga aagaggtttt 5940
aaataggggt tagttagttt gggggattaa tttgtgagga ggggtagtta aagaaaattt 6000
tttatatgaa attgggaatt ttgtcgtaga gttatattac gtaaaattgg tagatgaaag 6060
taaggaatat attttgggga tttaaatata tttttggtgt gttaggggga agttgagatt 6120
aatttttata ttttaagaat ataagttagt ggtttggacg ttggtagtag tcggggaaaa 6180
aataattttt ataattattg tggttttagg gaaaggtttt aatatttttt agagatggaa 6240
gaaagtattg tttagatttt gggggagttt ggagtgtgat tagtatattt ggggaaagta 6300
aaataaaaaa taaaaaaata taaaattagt attatttttt tatgttaatt tttaaatgat 6360
ttttattagt ttatttttgg taaataagaa aaagattata tatttttttg gatgtcgttt 6420
gtatttgggg gagttttagt gaaatatatt tttaagtttt gggatatatg gaaattattt 6480
aatggagatt tgatatttaa atttttggaa atttagttag tagttaggaa atttataggt 6540
cgtaggtttt tttatgtttg ggattttttg ggatttaggg atatttttta gtgtgttttg 6600
ggggtttatg gaatattttt taaatgtagg gggatttagg gtagggatta taaagttagg 6660
agatttataa attagtatta aaaaaaaaaa aaattttaat tgttttgtga agggaaaaaa 6720
attttaattt gttttggaag gttgggagat aatatcgtaa tttgggggtt ttaggaaaat 6780
cgtttttgaa tttaaagatt tagggaagtt gtttagagtt tttggaggat ttgggggttt 6840
atatgtatat tttaaagaaa ggggatttag aatttttagg ttttaggatt taggaatttt 6900
tagattttgt gatagggagt gtgtagtgtg tttgaaggaa atattgttta attttgagat 6960
gatttagtgg gttgtttgga gtttaagaaa ttagtgttgt tttgtgattc gagggtaatg 7020
ttgaatgttt ttagagggtt tggagggtag cgtttagaat atttaagtat gggggattag 7080
gaaaagggtt tagtgtgatt ttggtcgttg gggttttaga ttaatgtttg aattttagaa 7140
aagtaagatt tttgttatga agtttttggg atttagagaa ttgtttaata gttaatatgt 7200
tgtgatgtag agaaagtgtt cggaatgttt gggaattggg gtcgttagta atatttggta 7260
taatagaaat aaaagtaggt tttgtttaag ggtttttttt gtgtttggtg tagtttagag 7320
ggattcggga gattttgtgt gatttggggg gatttggaga attttgggtt atatttgagt 7380
attttttggt aattgtttag tgtgttttag ggatttgggt ggtattgttt atattttaag 7440
ggatttgatt cgtggttagg tagtatatag agttatagac gttaatgttt taaagttttg 7500
tgatttatat atttataata tttttaatag ttttggggaa ttattgggga aattttttta 7560
ttttatgaaa tttagataat tagagggtag gtcgtttatg atttagaaaa ggatttaata 7620
tgttttgggt gtaatgatcg aatttggggg aaggtatggg aagtaatttt tatattttga 7680
gggttttaat tttttagttt tgttgagtgt tatgagggat ttgggattta acgtgtaaag 7740
tttagggaat ttaggttagt gtgaatagat ttgcgggatt taatagtaaa tgttttatgt 7800
tgaggtgaac gggaaggggc gcgggggtgg ggtggggggg cgcggaatgg ggcggacgtg 7860
tgcgggaaat agtaagttta ggagattttg gagttaatat ttaaaattat gcgggaaagt 7920
tttatttgtt ttggaaataa tgtttaaatg tggaaaaggt attgtttata ttttggggag 7980
ttaggtaaat tggttagcgc gattattacg gattaagggt ttcgttttta aaattaggga 8040
attcggttta gtggtttgta aatttgtggg atttggaggg ggtgtataaa attgtttaga 8100
gtttgggaga aagtatattt ggggttttag gttatatagt gtaagtttgg aaaagttcgt 8160
cgggattgat gtgttttgac gttttcggaa attaacgttt aagaaaatta ttgtgattcg 8220
tcgaaatggt gaatatattt agaa 8244

<210> 191
<211> 16842
<212> DNA
<213> Artificial Sequence

<220>
<223> chemically treated genomic DNA (Homo sapiens)

<400> 191

ttttttaaag tgttgggatt ataggcgtga gttattgtat ttggtttgtt atttatgtat 60
ttattttttt ttgagataag tttttgttat gttgtttagg ttggttttaa atttttaggt 120
ttaagagatt tttttaaagt tttgggatta taggtgtgag ttattgtgta taattttgaa 180
gtggttaatt ttaataatat atttatttta gtttaatata tttaaaatta tattatttta 240
atatgtaatt attattaaaa ttattaatga gatattttat tttattttta tttatttatt 300
tttttgagac ggagtttcgt tttgtttttt aggttggagt gcggtggcgc gattttggtt 360
tattgtaagt ttcgtttttt gggtttacgt tttttttttg ttttagtttt tcgagtagtt 420
gggattatag gtgttcgtta ttacgtttag ttaatttttg tatttttagt agagattagg 480
ttttattacg ttggttagga tggtttcgaa tttttgattt cgtgattagt ttattttggt 540
tttttaaagt gttgggatta taggcgtgag ttattgtgtt tggttagtga gatattttat 600
ttgtgttttt tttttttttt tttgagatcg agttttattg tgttgtttag gttgtagtgt 660
agtggtataa ttttggttta ttgtaatttt tgttttttag gtttaagcga tttttttatt 720
ttagtttttt gagtagttgg gattataggc gggcgttacg atatttggtt agtttttgta 780
tttttagtag agacgggggt tttattatgt tgtttaggtt ggttttgaat ttttgatttt 840
aggtgattat ttgttttggt tttttaaagt gttgggatta taggtaaaag tcgttatatt 900
aggtttattt tttttttttt cgagacggag ttttattgtt gtttaaggtg gagtgtaatg 960
gtaagatttt agttttttgt aatttttgtt ttttgggttt aagcgatttt tttgttttag 1020
ttttttaagt agttgagatt acgggcgttc gttattacgt ttggttaatt tttgtatttt 1080
tagtagagat agggttttat tacgttggtt agattggttt taaatttttg atttcgggtg 1140
atttattcgt ttcggttttt taaagtgttg ggattatagg tgtgaattat cgtgtttggt 1200
aggtttattt ttaatttttt taattttgga aatttagtgt gtagggtagg tatggtgttt 1260
tatgtttgta attttagaat tttgggaggt cgaggtaggt ggattatttg aggttaggag 1320
ttcgggatta gtttgaataa tatggtgaaa ttttgttttt attgaaaatt taaaaattat 1380
ttagttatgg tggtgtatgt ttgtagtttt agttatttta gaggttgaga taggagaatt 1440
gtttgaattt gggaagtaga ggttgtagtg aattaagatt atgttattgt attttagttc 1500
gggtaataga gcgagatttc gttttaaaaa aaaaaaaaga gagttagtgt ggagggaagt 1560
gtttttgttg ggttcgggtg ttttgtggga ttttgaggaa aagttcgtat taggtagatg 1620
ttgttttgtt tttatgggta aaggatattt taataaatcg aggagtaaaa tgttttcgta 1680
tgtttttttc gcgtagattt gttaggaaga gtataagaag aaatattcgg atttttttgt 1740
taatttcgtg gaatttttta agaagtgttt ggagagatgg aagattacgt ttgtaaagga 1800
gaagtgaagt ttgaagagaa ggtaaaaagt gataaagttc gttgtgatag ggagattaaa 1860
aattatattt tttcgaaatg taagaaaggg taagaaagga aagaaaaagg atcgtaatgt 1920
ttttagaagg ttattatttg tttttttttt gttttgtttt gaatatcgtt taaagattaa 1980
aagtggatat ttaggtttat ttgtcgtgga aattgtaaag aaattgggtg aaatgtggtt 2040
tgggtagtta gttaaagata aataattata tgagtagaaa gtagttaagt tataggagag 2100
atatgaaaag ggtattgttg tatatcgtgt taagggtaaa agtgaagtag gaaagaaggg 2160
tttaaagaag aataaattag aagatgagga ggaggaggag gagaaagaag atgaagatga 2220
ggaggaagag ggtgaagatg aagaataaat ggttattttt taatgatgtt tgtgtagtgg 2280
gtttgttttg ttaagaatgt gaattttagt atagtttagt attagtttta gtataaaatt 2340
gtataaattt tcgtatagtt tataagattt tttgtataga aaatattttt tttttttttt 2400
tttttttttg agatagagtt tcgtttttgt tgtttaggtt ggagtgtaat ggcgtgattt 2460
cggtttatcg taattttcgt ttttcgggtt ttggtttaag tagttttttt gttttagttt 2520
tttgagtagt tgggattata ggtatatgtt attacgttta gttaattttt gtatttttag 2580
tagagatggg gttttattat gttggttagg ttgtttttaa atttttgatt tcgtgattcg 2640
tttgtttcgg ttttttaaaa tattgggatt ataggtgtga gttatcgtat tttgtttaat 2700
gtttttaaat atttaatggt ttttaaaaaa tttattgtgt atggtagtat agtatatttg 2760
taggaattag tattaatagt atattttgcg ttttttaaga tgttgtattt tttaatattt 2820
tgtaataaaa ttatgcgtat taaaaaaata aagaaatttc gtgtgtagtt tatatttata 2880
gtatattttc gtttaggtat ttgagagaat gattaggagg ggtttttgga ggaggtggtt 2940
tttgaacgga gaatttattt ttaaggattt tgtttgtaat ggttattaag tattttttga 3000
gttattttta tgtgttttgt agttttttga aggggcgtgg gatttatcga tgttaattat 3060
ttagtattat ttttagattt taagaagttg gggtgtgagt tagtaattag tatagaaaag 3120
agatattaaa ataagtttga gttggggagt gtttttttaa ttttagtttt ttggaagaga 3180
tttttttttt ttttttgaga tagagtttcg tttttattgt ttaagttgga gtgtagtggt 3240
acgatttcgg tttatcgtaa tttttttttt tcgggtttaa gcgatttttt ttttttagtt 3300
ttttgagtag ttgggattat agatatgtat ttgtaatttt tattaaaaat ataaaaatta 3360
gtcgggcgtg gtggcgtacg tttgtaattt tagttattgg ggaggttgag gtaggagaat 3420
cgtttgaaat taggaggcgg agattgtatt aagatagttt gttttagtta aataatttgg 3480
cgttagtgta ggaaaaggtg gaaggtacgg ggttagtata ggagggttta atatttttaa 3540
ttttattaag ttatattttg gtaatttttg ttttttacga gaagttttcg ttgggtttgt 3600
tttagcgttg ttttgaggtt ttttttatga gtttcgatag ggtagaggtc gttttgagcg 3660
tttttttttt ttttggttta agagtggttt aaaagaagga tttttgattg gaattggtta 3720

ttttgtgtta ttttttgatt tttgatttcg ttttaaaggg ggatgcgggg gaggggtttt 3780
ggtaggggtg gtttcgtttt ttttaggttc gtaagtttag gttttcgttt atcgggttta 3840
gtttattttg cggtcgttta gggaggtcgt tggtattcgt gatttacgat ttttttttcg 3900
agttttatcg aggttatagt cgtggtttcg tttttttatg tttgtttttc gttttttgtt 3960
cgtgacgggc gttttcgagg attaatgagc gcgttgtatt tatttttcgg gcggggttaa 4020
gcgtcgatta atcgtcgttc gggcgttcgg tcgggtttaa acgttttaat cgttagcggc 4080
ggcggggcgg gtagagggtc ggggatggta ggtttaatta acgggtgggt acgtcgtttt 4140
cgcgaggagg cgtgttttgc ggtcgggcgt gcggtgttcg cggcggcgta gggaggggga 4200
gggaggtaaa taagatggcg gcggcgtgtc gggcgcggaa gggggaggcg gttcggggcg 4260
ttcgcgagtg aggcgcgggg cggcgaaggg agcgcgggtg gcggtatttg ttgtcgcggt 4320
tttggatggg ttgggttttt ttcgtcgttt cgtttttttt atacgcgcgg cggtcgcggc 4380
gagggggacg cgtcgttcgg ggttcggtat tttcgggaat ttttcggttc ggagtttgcg 4440
gtttgcgtcg tttcggtcgt cgggagtttc gtggagtttt cgtcgtcgcg tcgtttcgcg 4500
gatcggacgt tgagggtatt cggggcgggg cgcgcgttcg ggtagacgtt tgcggggagg 4560
ggggcgtttg tcgggtttcg gcgattattt tgggggtcgc gggtcggttc ggggggcgtt 4620
tagtgcgggt tttcgcgggc gtcgggtagc gattagtttt gagcggagtt gttggtcgcg 4680
gcgggaggtt tttcggacgt ttttagtttt tcgaacgttc gttcgggtcg gcgggagtcg 4740
gcgtttttcg ggaggttcgt tcggtcgttc gcggcggagc gtttgttttt gggataggcg 4800
gtgggatcgg ggcgtcgtcg gagacgtttt tagcgaagtt gggtttttta ggtgtggggg 4860
tttcgggggg tagcgacgtc gcggattcgg tttgtgggat gggcggttcg gagaagattg 4920
cgttcggtcg tgtttatatt tgttcgtggg tttgaggttt tcggaggatg atttagtatt 4980
gaaaagtttc ggtcggtttt tttagggttt tcgaggacga agttgatttt gatcgggtcg 5040
ttttttagtt ttgaggttcg ggttttattg gaattcgcgt ttgagtcgtc gtttcggatt 5100
ttcggtgttc gtcggttcgt agattttgta tcgggtttgg attcgtagtc gggattgacg 5160
tgtagaataa tcgtttttgt tggaagaagg gttttttttt tttttttggg gtttttgttg 5220
tttttttttt tttttttttt tttgtaaaat tttggagaag ggaagtcgga atataaggaa 5280
ggatcgttta ttcgcggatt tagggttggc ggcgggattt taggattttg ggtttagtat 5340
ggaggtggtg gattcgtagt agttgggtat gtttacggag ggcgagttga tgtcggtggg 5400
tatggatacg tttatttatc gtatcgattt tatcgaggtt atttattagt cgcgtcgtaa 5460
gcgggttaag tttatcggta agtatttgat gggggatttg ttgggggaag gtttttacgg 5520
taaggtgaag gaggtgttgg attcggagac gttgtgtagg agggtcgtta agatttttaa 5580
gaagaagaag ttgcgaagga tttttaacgg ggaggttaac gtgaagaagt aagtatggtt 5640
tgttggggtc ggggtcgggt cgggttagtt acggtgttga tggttttgtt tttttttttt 5700
tttttttttt tttttttttt attttttttt aatattttga gttggattcg tttggcgttt 5760
gtgtttttcg tgttagggag agcgtggttg ggggtttgcg ttacggattt ttatttaggt 5820
aaggttagtt gtcgtagcgg ggcgtgcgtt tgtatgggtt tttggatttt agttaaaatg 5880
ttttggtagc gaaatttttt tgagaaggga gcggttttta attttttaag attagttttt 5940
tggttttttt agttgtttaa ggagtagagg cgtttagtgg aattagtttg tgtttgtttg 6000
ggtttcgaga gtttgtgtgc ggtcgttaat acgttttttg cgtagtgtgt ggtcgttatc 6060
ggggttaggc gaaatgtgat ttgtttattt tgttagaggg gaattttggg ttgttaaaaa 6120
taattgtttg tatcggttta ttagttagta ggagggaaac gtagtttttt tttatttgtt 6180
agggatgtga cgttggaagt atttttggtt ttcggggttg gaagttttgt tcgaggggga 6240
ttgtgttttt ttttcgaatt gtattcggaa gattttattt ttttaattga tttttttagg 6300
tacggggttg tcgttgggta tttcggacgt ttttgtattt gtgcgcggag aagtttttat 6360
ttagggtagt attggtcggt ttgagttttt tttagttggt gggggtggtc gggggtgttt 6420
ttgttttatc gtagttagat acgttgtatt tgtcgtcgtt tggcgggttt tgtttaggtt 6480
ttgttttttt tttagttttt ttaattttta aaattttgtt ttttgttttt aagaaaggat 6540
tttatttgtt gggttaggtt tcgagggatt ggtaaggata ttttgcgtag ggtattatga 6600
ttttggaagg aggttcgggg aggggttgtt gcggtgtttg tttttatttt ttttttgttt 6660
aggggttttg gggtttttgt cgtgtttgtt tttatttttt ttttgtttag gggttttggg 6720
gtttacgtat attttttttt tttttttttt gagacggagt ttcgttttgt tttttaggtt 6780
ggagtgtagt ggcgcgaatt gggtttattg taagtttcgt ttttcgggtt tatgttattt 6840
ttttgtttta gtttttcgag tagttgggat tataggtatt cgttattacg ttcggttaat 6900
tttttgtatt ttttagtaga gacggggttt tatcgtgtta gttaggatgg ttttaatttt 6960
ttgatttcgt gattcgttcg tttcggtttt ttaaagtgtt ggtattatag gcgtgagtta 7020
acgcgtgcgg tttttttttt tttttttttt tttttttttg agacggagtt ttatttttgt 7080
tgtttaggtt ggagtgtagt ggagtggttt tggtttattg taattttcgt tttttgggtt 7140
taagtgattt tcgtgtttta gtttttcgag tagttgggat tataggtgtt tgttatagtg 7200
tttagttaat tttttttttt tttttttagt agagacgggg ttttattatt ttggttaggt 7260
tggttttgaa tttttgattt cgtgatttat ttgttttagt tttttaaagt gttgggatta 7320
aaggcgtgag ttatcgcgtt cggttagtac gttttttaat tagtatgtag gttgtgatat 7380
gcggaaatta ggggtatagg ttttgaagtt tgtattttgg agttggagta gttagggtta 7440
aatgggagcg gttgggaagg gtgtgttttt acgaggtttt tttgatgttt tagtgtttag 7500

tttggattcg ggtagaaggt gtggttagtg ttttgagtta gtggagtagt tgtttgtgtt 7560
tggtttttgt gagatgggtt ggttaggggt taggtagagt ttgtgtttgg tttttgtgag 7620
atgggttggt taggggttag gtagagtttg tgttgggggt agtaggtgtt ttttaaaggg 7680
gttagtgata gttttttttt tagtagtgag aaatttacgt ttaggggtcg ggcgcggtgg 7740
tttacgtttg taattttagt attttgggag gttgaggtgg gtggatcgtt tgaggttagg 7800
agttcgagat tagtttggtt aatatggtga aattttattt ttattaaaaa tataaaagtt 7860
agtcgagtgt ggtggcgtgt gtttgtaatt ttagttattc gggaggttga ggtagaatat 7920
tgttggagtt tgggaggcgg agtttgcggt gattcgagat cgtattattg cgttttagtt 7980
tgggcgatat agcgagattt tgttttaaaa ataaataaat aaataaataa ataaataaat 8040
aaataaataa atttggtttg agttttgata aatgtatatg tagggttaat gggaagatag 8100
acgttggagg gtggaagttt gattgtgaga gtgagttttt tgtttttttg aatagttagt 8160
ggaggaggtt ttgggagttt aggtgagtat ttgaacggta gggtagagtt tgagggtttt 8220
ggtggagata ggtaagaagg ttgtaggaat ttagtttggt ttagtgtttg ggagaacggg 8280
agggaatacg gaggtaggga ggtagagggg ttggtaggat ttggattgga ttgtgcgtcg 8340
tagttattta tagtttgatt ttattgtttt gaagtgagtg atgtattttt attagtgttt 8400
agcgtttaat tttattttgg ttttgagtgt gtaacggttt tgtttttgcg ggtttttttg 8460
ggatgagggg tgagttttgt ttttttttgg ttattgtttg ttattaggag ttgtttcgtt 8520
tatagttgtg atttaggagt tattgtattt tttttagttt tttgggtgtt gttagggggt 8580
ttttttattt tgggagttgg tgtgttttga ttggaggttg ttatgtgtag agtgttgtta 8640
aggagaaggg gtgtttattg tttagttttg aggtttaagg ttttggttag ttttgttgtt 8700
agcgttgtag ggaaaaagtt ttttttgtgt gtgggaagtt taataaattt cgtttagatt 8760
gtgtttcgta gcgagtgttt ggaatttttt agataagttt taggcgttcg gttttttagt 8820
tggtgtggaa agcgtgggcg attattaagg ggggtgggtt ggggtagatg gagtcggcgt 8880
gagtttcgtt tttttttttt ttttttagaa aggtagtttt ggagtttatg ttttgtttcg 8940
tttttatcgg taaaaagtat aattttatta gaaataggaa agttttaaaa agtattaatg 9000
agttaaaaag agggttgggt atgttcggtt atgtttgtaa ttttagtatt ttgggaggtt 9060
aaggcgggtg gattattcga ggttgggagt tagtttgatt aatatggtga aattttgttt 9120
ttattaaaaa tataaaatta gttgagtgtg gtggcgtacg tttgtaattt tagttatttt 9180
ggaggttgag gtaggagaat tatttgaatt cgggaagtag aggttgcggt gagttaagat 9240
tatgttattg tattttagtt tgggtaataa gagtgaaatt tcgtttcgaa aaaaaaaagg 9300
gttggggtcg ggcgtggtgg tttacgtttg taattttagt attttgggag gtcgaggcgg 9360
tttgattata aggttagaag tttgagatta gcgtggttaa tatggtgaaa tttttttttt 9420
attaaaaata gaaaaattag ttaggtgtgg tggtatatgt taataatttt agttatttta 9480
tttgaggtta ggagtttaag attagtttgg ttaatatggc gaaatttcgt ttttattata 9540
aatataaaaa ttagttaggc gtggtggcgc gcgtttgtag tttagttatt ggggaagttg 9600
aagtaaggag aatcgtttga atttgggagg cggaggttgc ggtgagtcga gatcgtatta 9660
ttgtatttta ggttgggtaa gtttaggaga gaagagcgaa attgtgtttt aaaaaaataa 9720
ttaattaaaa taaaattagt gttataggtt gtttttggta ggtagtgggt ttttagggtg 9780
gagtagggaa gggttggaaa gggtcgtttg tgtttagttc gatttttcgt gtttttcggg 9840
ggatagtacg gttagttgtt gttgcggagt ttatagtggg gttttggggg ggggggtgta 9900
ggtagaaggt cgttatgttt tttttttttt ttttatttta gtgagagtgg gttcgaaggt 9960
agggagtttg ttcgttttgt ggagttttcg tagaggtatt tagggttgtc gtgagcgtat 10020
agtttggcgg tgttttacgg ttttgttttt ttttgttatt tttatttgtt gtaaaatatt 10080
gtgttttcgt attttttttt tttatttgtt tggaaatgtg aaaattattg ttgtcgtagg 10140
aaacgggttt agattcgggt tgtggttttt ttattcgtat cgttttcgcg gtgtttagtt 10200
aggaggtggg aggtgggcgg tatttagtgt gtttggttgc ggcgatgatt ggtagatgtt 10260
tggatttatg gtaaagagga ttttcgcgag ttgggagtta gttttggatt ttgtaggttt 10320
ttatatttta ggcggttttt atggggtttt agtatttgtt gaggtttttg cgttagttat 10380
gtgaggtttg ggtgttttcg aggtttttgt ttgtgttttt ttttgggtag cgggagggag 10440
agttttgaga tttagaggga gtttaggggt tgggtagtcg tagaatagcg ggggtaatgg 10500
gtttattggg ggttttgggt tgtgtatata gtgggttttc ggtgtttagt tagttggggt 10560
aggtttggaa tgttggtttt ttttttagga tttggggcgt tgttgtagtt gtcggggaat 10620
tagtgaggcg gttggacggt tcgtatattt tataaacgtt atcgtattgt ttaaattgga 10680
tttaattttt ttaatatttt tttttttttt tttttttttt tttttggaga tagggtttta 10740
ttgtatcgtc gaggttggag tgtagtggcg taattatagt tttgggttta ggtgattttt 10800
ttgtttttaa ggtagttggg attgtagatg tgtattatta tattcgatta gttttttgta 10860
gagatggggt ttcgttatgt tgtttaggtt ggttttaaat ttttagattt aagttattcg 10920
tttgtttggg ttttttagag tgttgggatt ataggtatga gttattgttt cgttacgttt 10980
atttttattt atttatttat ttatttttga gacggagttt cgttttgttt tttaggttgg 11040
agtgttgtgg tacgattttg gtttattgta agtttcgttt tagtttttta agtaagttgg 11100
aattataggt gtttgttatt aagtttggtt aattttgttt ttgtattttt agtagagacg 11160
gggttttatc gtgttagtta gtatggtttc gattttttga ttttgtgatt cgttcgtttc 11220
ggttttttaa agttttggga ttataggttt gagttattgt gttcggttta tttttttatt 11280

ttttatttta tagagataag ggtttgttgt gttgtttagg ttggttttga atttttgatt 11340
ttaagtaatt ttttttggtt ttttaaagtg ttgggattat aggtgtaagt tatagtattg 11400
ggtttaaagt ttattttttt tttttttttt tttttttttg agatggagtt tcgttttgtt 11460
atttaggttg gagtgtagtg gcgtgatttt ggtttattgt aagttttgtt tttcgggttt 11520
atattatttt tttgttttag tttttcgaat agttgggatt ataggcgttt gttattatgt 11580
tcggttaatt tttttgtatt tttagtagag atggggtttt attgtgttag ttaggatggt 11640
ttcgattttt tgatttcgtg atttgtttgt ttcggttttt taaagtgttg gggttatagg 11700
tgtgagttat cgtattcggt aaagtttatt tttttaaagt gtgtaattta gtggtatata 11760
gtatattgat agagttgtat aattattatt tttattagtt tttagaatat ttttgttatt 11820
ttagaaggaa atttcgtttt tgttagttat tattttttat ttttttttta gtttcggtat 11880
ttacgtattt tttttttgtt tttggattgg tttgttttga atattttata cgagtggtgt 11940
tatatatcgc gtggtttttt gcgttcggtt tgttttacgg agtgtgttgt ttttaagggg 12000
tattcgcgtc gtggtttggg ttcgaatttt tttgttgttt acggttgcgt tattttttag 12060
tgtgtggagg gttacgttgc gtttgattgt ttatttgtgg ttggatattt gggttgtttt 12120
cgtttttgtt tgtgaattgt ggttgttttg gtgtacgtgt gtttagtcgg cgttttttta 12180
gaatttttga gggttttgtt ttagttatat taggagggat ttagggatta gggtacgcgg 12240
gtttggagtt gggtgttttt gggtttttat gtgtttttta gttttgtttt gagtcgtggg 12300
aggtcggggc gagggttagg tttatttttt ttttgtatga tttgttttta ggtagacgga 12360
ggtttttagt tttatttttt tgtgagtttg agtggcgttt atgatttttt tttttatttt 12420
ttgttttgtt aattaataag ttagttaaat agtttatgtt gtttgggtaa gtttagtagg 12480
tttttaacgt ggtatttcgg agcggttggc gatgttttaa gagtcgggtt tttttttttt 12540
ggaggttttt tttgtttttc gttaagtttt taggtttcgt tgggtttttt tttttgtttt 12600
ttttttggtt ggagacgtta gaggaagttt ggcgatttag agaagttgat gtgtatttat 12660
tatttcggag gtgattgtta ttaggcgggg gtggttttat ttttattttt tttttggtat 12720
aaatcggggg acgtacgtgt ttgcgtagaa tgttttggtt ttttttaatt aaatagataa 12780
tttagtgttt tttggtttat gttgatttgt ttaggagtgt ttgggttttt ttacgtttta 12840
gttgttgtgt gttttgttgt tgtgaagtgt tttcgttgag gtggaggtag ggtagggcgt 12900
tttcggtgag ggcgagtttt tttgtgttcg tttttttatt tgtagaatga tggtttatgg 12960
aggagatatg gtgtcggtag gtggtggtcg gcgtagggag gtttttagga gggagttttt 13020
tatttttatt tgtttttagg gtcgtgtagt ttttcgagcg gtttttacga ggttttgagg 13080
ttgtatgtag gttgagtttt tggttttatt gttttttttt agggttagga ttgtgttgga 13140
gtttgggtgg gtgtggggtg gtttttgtat aggtagtagg aggtgggtta gagcggtgtt 13200
ggatttgaag gttcgtggtt tttttagttt tattgaggag gttggaatag tgttttgggt 13260
tatttttttt gtcggtagtc ggtttttgtt gagggagggg gtgttttcgt tttttggtgg 13320
agtattaggt ttttggggtt tgtgcgttcg agtatttggt ttttgttgtt tgaaaatagt 13380
atttttttcg tttttcggtt atcgtatttt ttttgaatag aaggtaagtt ttgtttttta 13440
ggaatggttt gtgattttta ttagcggggg aggttagggg tatatagagt tttggggaat 13500
aaggggattt ggggttgagt cgggtttttc gggtttattt cgagtttttt tgagtagttt 13560
ggtaggtagg aaggcgttgg gtttcggggt ttttgtcgag tttagtttta tttttagcgt 13620
tttttttaat gttttgggtg tttgttaggt tgtggatata ggttttggtt agttttataa 13680
ataattttgt cgggttttgt tgggaattgg tgttattatt atatagttta gagagaacgg 13740
aaatataggg ttttttagcg agtagattag gtttgcggtg attagtggaa gtgggggacg 13800
tttttagggt tgggtagttc gatagggtat cgggtatagt taggggtcgg aattagttgt 13860
ttattggttt cgtttttggg ggattgtgtt tttttgtgtt tcgtttttat tatattttgt 13920
ttttttttta ttttgttttt ttagtttttt tttagattgg cggttttttg tttaggttta 13980
gttttagtat agtttgtagt tttggtagag tttaggtttt ggttttttga ggttgttttg 14040
agggtagtga gtaatgagtt ttgggattgg tgtattggga gggaaaggtg agttaaggtt 14100
ttttgtaaga ggtagttttg agttgggggt ggggaaggtt gggtcgtggg atatttttga 14160
tattttaagt tttttttttg agatggagtt ttgttttgtt atttaggttg gagtgtagtg 14220
gtgagatttc gattttttgt aattttcgtt ttttaggttt aagcgatttt tttgttttag 14280
ttttttgagt agttgggatt ataggcgttt attattacgt tcggttaatt tttgtatttt 14340
tagtagagat aaggttttat tatgttggtt aggttggttt cgaatttttg attttaggtg 14400
atttatttcg gttttttata gtgtcgggat tataggcgtg agttatcgcg tttggttttt 14460
tagttgtttt ttatgtttta tgtttgggtt ttttgttaag aattaaaaaa gaaaaaaaaa 14520
attatggaga tggaatttat atattgtata atttattcgt ttatagtgta taatttagtg 14580
gtattaagta tatttatacg gttgtgtaat tattattttt aattttagaa cgttcggtta 14640
ttttaaaagt aagttttatt tttattgtgt tattttttat tttttttatt agtttttggt 14700
tattttgaat ttattttttg gttttggatt ggtttgtttt ggatattgta tagaaatggg 14760
attttgtcgg tcgggcgcgg tgatttacga gagtaagaga tttagattat tttggttaat 14820
atggtgaaat tttattttta ttaaaaatat aaaaattagt tgggcgtggt tgcgggtatt 14880
tgtagtttta gcgatttagg ggttgagtta ggagaaatcg tttgaattcg ggaggtagag 14940
gttgtagtga gttgagatta tgttattgta ttttggtttg gcgatagagt gagatttcgt 15000
ttttaaaaaa aaaaaaataa gaaatgggat tttgttattt tagcgttttt cgaggtcgag 15060

gtgggtagat gatttgagtt tagtagttta aacgagatta gtttgggtag tacggtgaaa 15120
ttgtttttat taaaatataa aaattagttg ggtatggtag tatacgtttg tagttttagt 15180
tatttgcggg gttgagatgg gaggatcgtt tgagtttggg aggtgtattt tagtttgggc 15240
gatagggaaa ttttgtttta aaaaaaaaaa aaaaagtgta ttatatattg tgtgtttttt 15300
agtgttttat tgtgatattt ttaaggtgta gttaggttgt ggttcgagtt agagttttat 15360
tggtttttgt ggttgagttt tgttgagtgc gtggatgggt tgtgttgatt ttcgtttgtg 15420
gaagggtttt tgggtggttt tatatttcgg ttgttgtgcg ttttgttgtt gtgagtattt 15480
cgtgtttcgt ttttgcgtgg cgtgtattgt tttttagggt gtgtatttgg gagtggagtt 15540
gttggtttat agggtaatag atttttggag aagttgtaga cgtttttacg tttatttatt 15600
gggtgtgagg attttgtttt tcgttatttt tgtttttgtt taattttttt ttttatgaga 15660
tagggtttta ttttgttgtt taggttggag tgtagtagta cgattatagg taattatagg 15720
tttaagtgat ttttttattt gagtttttcg agtagttagg atttgaggta cgtattttta 15780
tatttggtta atttttgtat ttttagtaga gatagggttt cgtcgtgttg tttaggttgg 15840
tttcgaattt ttgagtttag gtgatttatt cgtttcggtt ttttatagtg ttgggagaaa 15900
tgaagtgttt gcgatagggc ggtagttgta gagggggttg tgttttttgg tttgttgtgt 15960
tttatttttg tgataggtag gtgggcgtgg ttaattgggc ggtagttgta gagggggtag 16020
tgataggcgg gtgggtgtgg taggatttta taatgttttt ttttgtggcg tttttttttt 16080
cgtttgtttt tttttatggg ttattttttt agtttatttg cggtttattt tttaaggagt 16140
gggattttcg gagttgggag tttttggttg cgtgggattg tattgtgtga cggagttatt 16200
attagggttt taatttattt tggtttttgt tatagtttgg gttttatttg tgttattatt 16260
gtagtagttt ggtttttagg aagtaaaatt gttattgagt tataaagttt aattaagagt 16320
gtttttgggg aggtcgaggc gggcggatta taaggttagg agatcgagat tattttggtt 16380
aatatggtga aattttgttt gtattaaaat ataaaaaaat tagtcggttg tggtgatacg 16440
tgtttgtagt tttagttatt cgggaggttg aggtagggaa attatttgaa tttgggaggt 16500
agaggttgta gtgagttgag atcgggttat cgtgttttag tttagggata gagtaagatt 16560
ttgttttaga aaaaaaaaaa agtgttttaa ttgtgttttt taaagttttc gtcggtcgat 16620
gatagattag agggcgttgt gtttttattt tttatcgttt tgagtttgga cgcgtggttt 16680
ttgtagggtt tttttttata gtattgtgaa tttatagttt ttttttaggg aagggaggag 16740
gtacgttatt tttataggga gatggggagg tcgattttag ggatttaggt tattattttg 16800
acgttgggtc ggttgatata tttttgtttt ttttgtttta gg 16842

<210> 192
<211> 16842
<212> DNA
<213> Artificial Sequence

<220>
<223> chemically treated genomic DNA (Homo sapiens)

<400> 192

tttgggatag agaggatagg ggtgtattag tcgatttaac gttaggatga tggtttggat 60
ttttggagtc ggttttttta tttttttgtg gaagtggcgt attttttttt ttttttagag 120
agaagttgtg agtttatagt gttgtgggaa agggttttgt aggggttacg cgtttaggtt 180
tagggcggta gggggtggga gtatagcgtt ttttagtttg ttatcggtcg gcgagggttt 240
tggaggatat agttaggata tttttttttt tttttgagat agagttttgt tttgttttta 300
ggttggagta cggtggttcg attttagttt attgtaattt ttgtttttta ggtttaagtg 360
atttttttgt tttagttttt cgagtagttg ggattatagg tacgtgttat tatagtcggt 420
taattttttt gtattttagt atagataggg ttttattatg ttggttagga tggtttcgat 480
tttttgattt tgtgattcgt tcgtttcggt tttttaaagg atatttttaa ttgagttttg 540
taatttagta atagttttgt tttttagaaa ttagattatt gtaatggtag tatagatgga 600
gtttagattg tggtaggagt tagggtgagt tggagttttg gtgatggttt cgttatatag 660
tatagtttta cgtagttaag ggtttttaat ttcgagggtt ttattttttg gagggtgggt 720
cgtaggtgag ttggaaggat ggtttatagg aaggggtagg cgaagaagga gacgttatag 780
aaagagatat tgtggggttt tgttatattt attcgtttgt tattgttttt tttgtagttg 840
tcgtttagtt ggttacgttt atttgtttgt tataagggtg gggtataata ggttagagag 900
tatagttttt tttgtagttg tcgttttgtc gtagatattt tatttttttt agtattatag 960
gaggtcgagg cgggtggatt atttgagttt aggagttcga gattagtttg agtaatacgg 1020
cgaaattttg tttttattaa aaatataaaa attagttagg tgtggaggta cgtgttttaa 1080
gttttagtta ttcgggaggt ttaggtggga ggattatttg agtttgtagt tatttgtgat 1140
cgtgttattg tattttagtt tggataatag aataagattt tgttttataa aagaaaaaat 1200
taaataaaaa taaaaatgac gaggagtaag gtttttatat ttagtgggtg ggcgtggaag 1260
cgtttgtagt ttttttaaag gtttgttatt ttgtgagtta gtagttttat ttttaggtat 1320

atattttgag gaatagtata cgttacgtag aaacggagta cgagatgttt atagtagtag 1380
gacgtatagt agtcgagatg tggaattatt taggggtttt tttatagacg aggattagta 1440
tagtttattt acgtatttaa taagatttag ttataaaaat tagtgaggtt ttgattcgag 1500
ttatagttta gttgtatttt gaggatgtta tagtgagata ttaaaggata tatagtgtgt 1560
gatgtatttt tttttttttt tttgagatag ggtttttttg tcgtttaggt tggagtgtat 1620
tttttaggtt taagcgattt ttttatttta gtttcgtaag tagttgggat tatagacgtg 1680
tgttattatg tttagttaat ttttgtattt tagtagagat agttttatcg tgttgtttag 1740
gttggtttcg tttgaattat taggtttaag ttatttgttt atttcggttt cggaaaacgt 1800
tgggatgata ggattttatt ttttgttttt ttttttttgg agacggagtt ttattttgtc 1860
gttaggttag agtgtaatgg tatgatttta gtttattgta atttttgttt ttcgggttta 1920
agcgattttt tttgatttag tttttgagtc gttgggatta taggtgttcg taattacgtt 1980
tagttaattt ttgtattttt agtagagatg gggttttatt atgttggtta ggatggtttg 2040
gattttttgt tttcgtgagt tatcgcgttc ggtcgatagg attttatttt tatgtaatat 2100
ttaggataag ttaatttaga gttaggaagt ggatttagag tggttaggag ttggtggagg 2160
ggatggagag tgatataatg ggaatggggt ttgtttttgg ggtgatcgaa cgttttggaa 2220
ttagaagtag tggttgtata gtcgtgtgaa tgtatttaat gttattgaat tgtatattgt 2280
gaacgaatga gttgtatagt atgtaaattt tatttttata gttttttttt ttttttttaa 2340
tttttaatag gaagtttaag tatggagtat aaaaaatagt tggggggtta ggcgcggtga 2400
tttacgtttg taatttcgat attatgggag gtcgaggtgg attatttgag gttaggagtt 2460
cgaaattagt ttggttaata tggtgaaatt ttgtttttat taaaaatata aaaattagtc 2520
gggcgtggtg atgggcgttt gtaattttag ttatttagga ggttgaggta ggagaatcgt 2580
ttgaatttgg gaggcggagg ttgtagagag tcgagatttt attattgtat tttagtttgg 2640
gtgatagagt aagattttat tttaaaaaaa aaatttgggg tgttaggggt attttacgat 2700
ttagtttttt ttatttttag tttagggttg ttttttgtag gaagttttgg tttatttttt 2760
ttttttagtg tattagtttt agggtttatt gtttattgtt tttagggtaa ttttagaagg 2820
ttaaggtttg agttttgtta gggttgtagg ttgtgttggg gttgggtttg gataaggggt 2880
cgttagtttg gggaggggtt aggagagtag gatgaaggga aagtaaggtg tgatggggac 2940
ggggtataga gggatatagt tttttagaag cggagttagt gagtaattgg tttcggtttt 3000
tggttgtgtt cggtgttttg tcgggttgtt tagttttgaa agcgtttttt atttttattg 3060
gttatcgtag gtttggtttg ttcgttgaga agttttgtgt tttcgttttt tttgggttat 3120
gtggtggtgg tattaatttt tagtagggtt cggtaaggtt atttatagaa ttggttaggg 3180
tttgtgttta tagtttggta ggtatttagg gtattgaggg ggacgttgag ggtgaggttg 3240
ggttcggtaa aggtttcggg gtttagcgtt tttttgtttg ttaggttgtt taggagagtt 3300
cggggtgggt tcgagaggtt cggtttagtt ttagattttt ttgtttttta gggttttgtg 3360
tgtttttaat ttttttcgtt gatgaaggtt ataggttatt tttgggaggt aagatttgtt 3420
ttttatttag aggagatgcg gtggtcgggg ggcggggaag gtgttgtttt tagatagtag 3480
aggttagatg ttcggacgta taggttttaa gagtttgatg ttttattagg aggcgaaagt 3540
attttttttt ttagtaggag tcggttatcg gtagggaagg tggtttagga tattatttta 3600
gtttttttag tggagttgag ggagttacgg gtttttaggt ttagtatcgt tttggtttat 3660
tttttgttgt ttgtgtaggg attattttat atttatttag gttttagtat agttttaatt 3720
ttgaggaggg gtagtgaggt taggagttta atttgtatgt agttttagag tttcgtgggg 3780
gtcgttcggg aggttgtacg gttttaaaag taaatgaggg tggaggattt ttttttggaa 3840
atttttttgc gtcggttatt atttgtcgat attatgtttt ttttatgagt tattatttta 3900
tagatgggga aacgggtata ggaggattcg tttttatcga aggcgttttg ttttgttttt 3960
attttaacgg ggatatttta tagtagtagg atatatagta gttgaaacgt ggaagagttt 4020
aggtattttt gagtaggtta gtatagatta agggatattg ggttgtttgt ttaattaaaa 4080
gggattaagg tattttgcgt aggtacgtgc gtttttcgat ttatgttagg ggaggagtgg 4140
gggtggagtt attttcgttt ggtggtagtt attttcggga tgatgggtgt atattagttt 4200
ttttagatcg ttaggttttt tttgacgttt ttagttagaa aaagagtaga agaggaggtt 4260
tagcgaaatt taagagtttg gcgaggaata ggaagagttt ttaggagggg gaagttcggt 4320
ttttggggta tcgttagtcg tttcgaggtg ttacgttagg ggtttattgg gtttgtttag 4380
gtagtatgga ttgtttagtt gatttgttga ttagtaggat aggaaatagg aaggagagtt 4440
ataaacgtta tttaagttta tagagaggtg gggttaggag ttttcgtttg tttagaaata 4500
gattatatag ggaagagatg ggtttggttt tcgtttcggt tttttacggt ttagagtagg 4560
attgggaggt atatggagat ttaggaatat ttagttttag attcgcgtgt tttggttttt 4620
gaattttttt tgatgtggtt gaaataaggt ttttaaggat tttagaggga cgtcggttga 4680
gtatacgtgt attaaagtag ttataattta taggtagagg cgggagtagt ttaagtgttt 4740
agttatagat gagtagttaa acgtagcgtg gttttttata tattggaaag tgacgtagtc 4800
gtgaatagta gagaggttcg gatttaggtt acggcgcgga tgttttttga ggatagtata 4860
tttcgtgaga taagtcggac gtaaaaggtt acgcggtgtg tgatattatt cgtgtgaaat 4920
gtttaggata ggttaattta gaggtaggaa gggaatgcgt gggtgtcggg attggggagg 4980
gaatggggag tgatggttga tagagacggg gttttttttt ggggtaataa gaatgttttg 5040
gaagttgata gaggtgatag ttgtgtaatt ttgttaatgt attatatgtt attgaattat 5100

atattttaaa aaggtgaatt ttgtcgggtg cggtggttta tatttgtaat tttagtattt 5160
tgggaggtcg aggtaggtag attacgaggt taggagatcg agattatttt ggttaatata 5220
gtgaaatttt atttttatta aaaatataaa aaaattagtc gggtatggtg gtaggcgttt 5280
gtagttttag ttattcggga ggttgaggta ggagaatggt gtgaattcgg gaggtagagt 5340
ttgtagtgag ttaagattac gttattgtat tttagtttgg gtgatagagc gagattttat 5400
tttaaaaaaa aaaaaaaaaa aaaaaaaggt gaattttagg tttagtatta tggtttatat 5460
ttgtaatttt agtattttgg gaggttagag aggattattt aaggttaggg gtttaagatt 5520
agtttgggta atatagtaag tttttgtttt tataaaataa aaaataaaaa ggtgggtcgg 5580
gtatagtgat ttaagtttgt aattttagga ttttgggagg tcgaggcggg cggattataa 5640
ggttaggaga tcgagattat attggttaat acggtgaaat ttcgttttta ttaaaaatat 5700
aaaaataaaa ttagttaggt ttggtggtag gtatttgtag ttttagttta tttgggaggt 5760
tgaagcggag tttgtagtga gttaagatcg tgttatagta ttttagtttg ggggatagag 5820
cgagatttcg ttttaaaaat aaataaataa gtaaataaag gtgaacgtgg cgaagtagtg 5880
gtttatattt gtaattttag tattttggga ggtttaggta ggcggatagt ttgagtttag 5940
gagtttgaga ttagtttgga taatatggcg aaattttatt tttataaaaa attagtcggg 6000
tgtggtggtg tatatttgta gttttagtta ttttgggggt aggaggttta tttgagttta 6060
gagttatgat tgcgttattg tattttagtt tcggcgatat agtgagattt tgtttttaaa 6120
aaaaaaaaaa aaaaaaaaaa aaaaggtgtt aagaaaattg agtttagttt gagtaatacg 6180
gtggcgtttg tgggatatac gggtcgttta gtcgttttat tgatttttcg atagttatag 6240
tagcgtttta gattttgaga gaggaattaa tattttaggt ttgttttagt tggttgggta 6300
tcggaagttt attgtgtgta tagtttagaa tttttagtgg atttattatt ttcgttgttt 6360
tgcggttgtt tagtttttga gttttttttg ggttttagag tttttttttt cgttgtttag 6420
agaggggtat aaatagaggt ttcgaggata tttaggtttt atatggttgg cgtaggggtt 6480
ttagtagatg ttggaatttt ataaaggtcg tttgggatgt aaagatttat aaagtttagg 6540
attgattttt agttcgcgaa aatttttttt attataggtt taagtatttg ttaattatcg 6600
tcgtaattag gtatattgag tgtcgtttat tttttatttt ttggttggat atcgcggggg 6660
cggtgcggat gggagagtta tagttcggat ttgggttcgt tttttacggt agtagtgatt 6720
tttatatttt taaataggta gaaagaaaag atacgaaagt atagtgtttt gtaataggtg 6780
gaagtgatag gaaagggtag ggtcgtggga tatcgttagg ttgtgcgttt acggtagttt 6840
tgggtgtttt tgcggaggtt ttataaggcg ggtagatttt ttgttttcga gtttattttt 6900
attggggtgg aaagggggag ggaggtatgg cggttttttg tttgtatttt tttttttaga 6960
attttattgt gggtttcgta gtagtagtta gtcgtgttgt ttttcgagaa gtacggggag 7020
tcgggttggg tataggcgat tttttttagt ttttttttgt tttattttag gaatttattg 7080
tttattagaa atagtttatg gtattgattt tattttaatt aattgttttt ttgagatata 7140
atttcgtttt ttttttttag gtttgtttag tttggaatgt aatgatgcga tttcggttta 7200
tcgtaatttt cgttttttag gtttaagcga ttttttttgt tttagttttt ttagtagttg 7260
gattataggc gcgcgttatt acgtttggtt aatttttgta tttgtagtag agacggagtt 7320
tcgttatatt ggttaggttg gttttgaatt tttgatttta gatgaagtag ttgggattat 7380
tggtatgtgt tattatattt ggttagtttt tttattttta gtagagagag ggttttatta 7440
tgttggttac gttggtttta aatttttgat tttgtgatta aatcgtttcg gttttttaaa 7500
gtgttgggat tataggcgtg agttattacg ttcggtttta gttttttttt ttttcgagac 7560
ggagttttat ttttgttatt taggttggag tgtaatggta tggttttggt ttatcgtaat 7620
ttttgttttt cgggtttaag tgattttttt gttttagttt ttagagtagt tgggattata 7680
ggcgtgcgtt attatattta gttaattttg tatttttagt agagataggg ttttattatg 7740
ttggttaggt taatttttaa tttcgggtga tttattcgtt ttggtttttt aaagtgttgg 7800
gattataggt atgatcgaat atgtttagtt ttttttttaa tttattgatg ttttttggaa 7860
tttttttatt tttaataaga ttatattttt tgtcggtgag agcgggataa ggtatggatt 7920
ttagggttgt ttttttggga aggaagggaa gagacgggat ttacgtcggt tttatttgtt 7980
ttaatttatt ttttttggtg atcgtttacg ttttttatat taattggagg atcggacgtt 8040
tgaggtttgt ttggaaggtt ttagatattc gttgcgagat ataatttgag cggagtttat 8100
taaatttttt atatataaag gaggtttttt ttttgtagcg ttggtagtaa ggttggttaa 8160
ggttttgggt tttagggttg ggtagtggat attttttttt tttgatagta ttttgtatat 8220
ggtagttttt agttagaata tattagtttt tagaatagga ggattttttg gtagtattta 8280
gggggttgga ggaaatgtag tggtttttga attatagttg taagcggggt agtttttgat 8340
ggtagatagt gattaggaag ggatagggtt tattttttat tttagggggt ttcgtaaggg 8400
taaagtcgtt atatatttag ggttaggatg aaattggacg ttgaatatta gtgagggtgt 8460
attatttatt ttaaggtagt ggggttaagt tgtgggtagt tgcgacgtat agtttagttt 8520
aggttttgtt aatttttttg tttttttgtt ttcgtgtttt ttttcgtttt tttaggtatt 8580
gggttagatt aggtttttgt aatttttttg tttgttttta ttaagatttt taggttttgt 8640
tttatcgttt aggtgtttat ttgagttttt aggatttttt ttattgattg tttaggaaag 8700
tagggggttt atttttatag ttaggttttt attttttagc gtttgttttt ttattgattt 8760
tgtatatata tttgttaaaa tttaagttag atttatttat ttatttattt atttatttat 8820
ttatttattt atttttgaga tagagtttcg ttgtgtcgtt taggttggag cgtaatggtg 8880

cgatttcggg ttatcgtaag tttcgttttt taggttttag taatgttttg ttttagtttt 8940
tcgagtagtt gggattatag gtatacgtta ttatattcgg ttaatttttg tatttttagt 9000
agagatgggg ttttattatg ttggttaggt tggtttcgaa tttttgattt taggcgattt 9060
atttatttta gttttttaaa gtgttgggat tataggcgtg agttatcgcg ttcggttttt 9120
aagcgtgggt tttttattgt tgggagagag attgttattg gtttttttag agggtatttg 9180
ttatttttag tataggtttt gtttagtttt tgattaattt attttataga gattaggtat 9240
aggttttgtt tagtttttga ttaatttatt ttatagagat taggtatagg taattgtttt 9300
attgatttaa ggtattggtt atatttttta ttcgggttta gattgggtat tgagatatta 9360
gaaaggtttc gtggaaatat atttttttta gtcgttttta tttaattttg attgttttag 9420
ttttagagta taaattttag agtttgtgtt tttggttttc gtatgttata gtttgtatgt 9480
tgattaagaa gcgtgttggt cgggcgcggt ggtttacgtt tttaatttta gtattttggg 9540
aggttgaggt aggtggatta cgaggttaga agtttaagat taatttggtt aagatggtga 9600
aatttcgttt ttattaaaaa aaaaaaaaaa aaattagttg ggtattgtgg taggtatttg 9660
taattttagt tattcgggag gttgaggtac gagaattatt tgaatttagg gggcggagat 9720
tgtagtgagt taagattatt ttattgtatt ttagtttgag taataagagt gaaatttcgt 9780
tttaaaaaaa aaaaaaaaaa aaaaaaaaaa ggtcgtacgc gttgatttac gtttgtagta 9840
ttagtatttt gggaggtcga ggcggcggga ttacgaggtt aggagattga gattattttg 9900
gttaatacgg tgaaatttcg tttttattga aaaatataaa aaattggtcg ggcgtggtgg 9960
cgggtgtttg tagttttagt tattcgggag gttgaggtag gagaatggta tgaattcggg 10020
aggcggagtt tgtagtgagt ttagttcgcg ttattgtatt ttagtttggg gaatagagcg 10080
agatttcgtt ttaaaaaaaa aaaaaaaaaa atgtacgtga gttttagggt ttttgagtag 10140
gagagaggtg agagtagata cggtaggagt tttagggttt ttgagtagga ggaaggtgag 10200
agtagatatc gtagtagttt tttttcggat ttttttttaa ggttatggtg ttttgcgtag 10260
ggtgtttttg ttagtttttc gaggtttagt ttagtagata aggttttttt ttggaaatag 10320
gaggtaaagt tttgaaagtt aagaaggttg ggaaaggagt agggtttggg tagggttcgt 10380
taggcggcgg taggtgtagc gtgtttggtt gcgataaggt agggatattt tcggttattt 10440
ttattagttg ggagaggttt aggtcggtta gtgttgtttt aggtaggagt tttttcgcgt 10500
atagatgtag aggcgttcgg gatgtttagc ggtagtttcg tgtttgaaga agttagttgg 10560
aaaagtaagg tttttcggat gtaattcggg agggaggtat agtttttttc gggtagggtt 10620
tttagtttcg ggggttaggg atgtttttag cgttatattt ttggtaaatg aggaaaggtt 10680
acgttttttt tttgttgatt gataagtcgg tgtaaatagt tatttttggt agtttagggt 10740
ttttttttga taggataaat aaattatatt tcgtttggtt tcggtaacgg ttatatattg 10800
cgtagaaaac gtgttggcgg tcgtatataa attttcgggg tttaaataaa tataggttga 10860
ttttattggg cgtttttgtt ttttggatag ttgggggagt taaggggtta gttttagggg 10920
attgggggtc gttttttttt taggagggtt tcgttattag ggtattttaa ttggagttta 10980
agagtttatg taaacgtacg tttcgttgcg ataattggtt ttgtttgagt gaaagttcgt 11040
aacgtaggtt tttaattacg ttttttttgg tacggaggat ataggcgtta gacgggttta 11100
gtttagggtg ttaagaggaa gtaagggagg gagggaggag agaaggaagg aagatagaat 11160
tattagtatc gtgattggtt cggttcggtt tcgattttag taagttatat ttattttttt 11220
acgttggttt tttcgttggg gatttttcgt aatttttttt ttttgaggat tttgacggtt 11280
tttttgtata gcgttttcga gtttagtatt ttttttattt tgtcgtaaga gttttttttt 11340
agtaggtttt ttattaggta tttgtcgatg agtttggttc gtttgcggcg cggttggtag 11400
atgatttcgg tggagtcgat gcggtggatg aacgtgttta tatttatcga tattagttcg 11460
tttttcgtga atatgtttag ttgttgcggg tttattattt ttatgttgga tttagggttt 11520
tggagtttcg tcgttagttt tgagttcgcg ggtgagcggt ttttttttgt gtttcgattt 11580
ttttttttta aaattttata aagaaaaaag aaaaaaaaaa ggtaataaaa attttaaaag 11640
gaagggaaaa attttttttt taatagaaac gattgtttta tacgttagtt tcggttgcga 11700
gtttaagttc ggtgtagggt ttgcggatcg gcgggtatcg gggttcgggg acggcggttt 11760
agacgcgagt tttagtggga ttcgggtttt agaattggga gacggttcgg ttagggttaa 11820
tttcgttttc ggggattttg gggaggtcgg tcggggtttt ttagtgttag gttatttttc 11880
ggggatttta ggtttacgga taagtatgaa tacggtcgag cgtagttttt ttcgggtcgt 11940
ttattttata ggtcgggttc gcgacgtcgt tatttttcgg gatttttata tttggagagt 12000
ttaatttcgt tgggggcgtt ttcggcgacg tttcggtttt atcgtttgtt ttaggagtaa 12060
acgtttcgtc gcggacgatc gagcggattt ttcggggggc gtcgattttc gtcggttcgg 12120
gcgagcgttc ggggggttgg gggcgttcgg gaggtttttc gtcgcggtta atagtttcgt 12180
ttagggttgg tcgttgttcg gcgttcgcga gggttcgtat tgggcgtttt tcgagtcggt 12240
tcgcgatttt taaggtggtc gtcggggttc ggtaggcgtt ttttttttcg taaacgtttg 12300
ttcgagcgcg cgtttcgttt cgagtgtttt tagcgttcgg ttcgcggggc ggcgcggcgg 12360
cgggggtttt acggggtttt cggcggtcga ggcggcgtag gtcgtaggtt tcgggtcggg 12420
gggttttcga aggtgtcggg tttcgggcgg cgcgtttttt tcgtcgcggt cgtcgcgcgt 12480
gtggaggagg cggagcggcg aggggggttt agtttattta aggtcgcggt agtaagtgtc 12540
gttattcgcg tttttttcgt cgtttcgcgt tttattcgcg ggcgtttcgg gtcgtttttt 12600
tttttcgcgt tcgatacgtc gtcgttattt tgtttatttt tttttttttt tttgcgtcgt 12660

cgcggatatc gtacgttcgg tcgtagggta cgttttttcg cgaggacgac gtgtttattc 12720
gttggttgaa tttgttattt tcggtttttt gttcgtttcg tcgtcgttga cgattggagc 12780
gtttggattc ggtcgggcgt tcgagcggcg attggtcggc gtttggtttc gttcgagggg 12840
tggatatagc gcgtttattg gttttcggag acgttcgtta cgggtagggg gcgggaagta 12900
ggtatgggga gacgaggtta cggttgtgat ttcggtgggg ttcgggaagg gggtcgtagg 12960
ttacgggtgt taacggtttt tttgaacggt cgtagggtgg gttgagttcg gtgggcggga 13020
atttgggttt gcggatttgg aaggagcggg gttattttta ttagagtttt tttttcgtat 13080
ttttttttgg ggcgaggtta agggttaaaa agtaatataa agtggttaat tttagttaaa 13140
aatttttttt ttgagttatt tttggattag gggaaagaga aacgtttagg gcggtttttg 13200
ttttatcgga atttatgggg gaagttttag gatagcgttg ggataagttt agcgggggtt 13260
tttcgtgaaa aataagagtt gttaaaatat ggtttgataa ggttgaaaat attgaatttt 13320
tttgtgttag tttcgtgttt tttatttttt tttgtattag cgttaggttg tttagttgga 13380
ataaattatt ttggtataat tttcgttttt tggttttaag cgattttttt gttttagttt 13440
ttttagtagt tgagattata ggcgtgcgtt attacgttcg gttaattttt gtatttttag 13500
tagaaattat aggtgtatgt ttgtaatttt agttatttag aaggttgagg gaggagaatc 13560
gtttgaattc gggaggagga ggttgcggtg agtcgagatc gtgttattgt attttagttt 13620
gggtaataag agcgaaattt tgttttaaaa aaaaaaaaaa gatttttttt agaaaattga 13680
agttgaagga atatttttta atttaaattt attttggtat tttttttttg tattgattgt 13740
tggtttatat tttaattttt tggaatttgg agataatgtt ggataattgg tatcggtagg 13800
ttttacgttt ttttagagaa ttgtaggata tatggaagtg atttaggaaa tatttggtga 13860
ttattataga tagaattttt gaagatggat ttttcgttta aagattattt tttttaagaa 13920
ttttttttag ttattttttt aagtgtttgg acggagatgt gttgtgagtg tgaattatat 13980
acggaatttt tttgtttttt tgatacgtat aattttatta taaaatgtta aaaaatgtag 14040
tattttaaaa aacgtaagat gtattgttga tattaatttt tataagtgtg ttgtgttgtt 14100
atatataata aattttttaa aaattattaa atatttaggg atattaggta gggtgcggtg 14160
gtttatattt gtaattttag tattttggga ggtcgaagta ggcggattac gaggttagga 14220
gtttgaaagt agtttggtta atatggtgaa attttatttt tattaaaaat ataaaaatta 14280
gttgagcgtg gtggtatgtg tttgtaattt tagttattta ggaggttgag gtaggagaat 14340
tgtttgaatt aggattcggg aggcggaggt tgcggtgagt cgagattacg ttattgtatt 14400
ttagtttagg taataagagc gaaattttgt tttaaaaaaa agaaaagaaa agaaaagtat 14460
tttttgtata gagaatttta tgagttatac gaaaatttgt atagttttat attgaagtta 14520
atattgagtt gtattagaat ttatattttt agtaaaataa gtttattgta taggtattat 14580
taaaggatag ttatttattt tttattttta tttttttttt ttttattttt attttttttt 14640
tttttttttt tttttttatt ttttggtttg tttttttttg agtttttttt ttttgtttta 14700
tttttgtttt tagtacgata tgtagtaata ttttttttat attttttttg tagtttaatt 14760
gttttttgtt tatatggttg tttatttttg gttgattgtt tagattatat tttatttagt 14820
ttttttgtag tttttacgat aaataggttt gggtgtttat ttttgatttt tgggcgatgt 14880
ttagagtaaa ataggaagaa ggtagatggt ggttttttag gagtattgcg attttttttt 14940
tttttttttt tatttttttt tatatttcgg aggaatgtaa tttttaattt ttttgttata 15000
gcgagttttg ttattttttg tttttttttt aaattttatt ttttttttgt agacgtggtt 15060
ttttattttt ttaaatattt tttggagaat tttacgaaat tgatagaaga gttcgggtgt 15120
ttttttttgt gttttttttg gtaggtttgc gcgaagaggg tatacgagga tattttgttt 15180
ttcggtttgt tggggtgttt tttatttatg gggatagaat agtatttatt tggtgcgagt 15240
ttttttttag agttttatag agtattcgga tttaatagag atattttttt ttatattgat 15300
tttttttttt tttttttgag acggagtttc gttttgttgt tcgggttgga gtgtaatggt 15360
atgattttgg tttattgtaa tttttgtttt ttagatttaa gtaatttttt tgttttagtt 15420
tttggagtag ttgggattat aggtatatat tattatggtt ggataatttt tggattttta 15480
atagagatag ggttttatta tattgtttag gttggtttcg aatttttaat tttaggtgat 15540
ttatttgttt cggtttttta aagttttggg attataggta tgaggtatta tgtttgtttt 15600
atatattgaa tttttaaagt tagaaaaatt aaaaatgggt ttgttaggta cggtagttta 15660
tatttgtaat tttagtattt tgggaggtcg aggcggatgg attattcgag gttaggagtt 15720
tgagattagt ttggttaacg tggtaaaatt ttgtttttat taaaaatata aaaattagtt 15780
aggcgtggtg gcgggcgttc gtaattttag ttatttggga ggttgaggta ggaggatcgt 15840
ttgaatttag gaggtagagg ttgtagggag ttgagatttt gttattgtat tttattttgg 15900
gtaataatga gatttcgttt cgaaagaaaa aaaaatgggt ttggtatggc ggtttttgtt 15960
tgtaatttta gtattttggg gggttaaggt aggtgattat ttgaggttaa gagtttaaga 16020
ttagtttggg taatatggta aaattttcgt ttttattaaa aatataaaaa ttagttaggt 16080
gtcgtggcgt tcgtttgtaa ttttagttat ttaggaggtt gaggtaagag aatcgtttga 16140
atttgggagg tagaggttgt agtgagttaa gattgtgtta ttgtattgta gtttgggtaa 16200
tatagtgaga ttcggtttta aaaaaaaaaa aaaagggtat aaataaaata ttttattggt 16260
tagatatagt ggtttacgtt tgtaatttta gtattttagg aggttaaggt gggttgatta 16320
cgaggttagg agttcgagat tattttggtt aacgtggtga aatttggttt ttattaaaaa 16380
tataaaaatt agttgggcgt ggtggcgggt atttgtaatt ttagttattc gggaggttga 16440

gataggagaa aggcgtgaat ttaggaggcg gagtttgtag tgaattaaga tcgcgttatc 16500
gtattttagt ttgggagata gagcgagatt tcgttttaaa aaaataaata aataaaaata 16560
aaataaaata ttttattaat aattttaata atgattgtat gttgaaatga tgtaattttg 16620
gatatgttag gttaaagtaa atgtattatt aaaattaatt attttagggt tatgtatagt 16680
ggtttatatt tgtaatttta gaattttggg aggatttttt gagtttagga gtttgaggtt 16740
agtttgggta atatagtaag aatttgtttt aaaaaaaaat aaatatataa ataataggtt 16800
aggtgtagtg gtttacgttt gtaattttag tattttggga gg 16842

<210> 193
<211> 1157
<212> DNA
<213> Artificial Sequence

<220>
<223> chemically treated genomic DNA (Homo sapiens)

<400> 193

ggattttgtt ttttttgaag gtttagggga agagtgtttt ttgttttttt cgaaggtttt 60
agggaagggt ttgttttttt aggtttttgg tggtttgtag gtgtagtttt ttaatttttt 120
tttttaagcg gtttgttgtt tataaggata cgagttatat tggatgaggg gtttattaat 180
tgatggtttt tgtaaagttt ttatttttaa ataaggttat attgtgaggt attgggagtt 240
aggattttaa tatagttttt ttggtggata taatttaatt tttaataacg tttatataat 300
tttaagtagg gtttggtatt ttgtgtgttt tttggagagc ggttgagtta ggttttggta 360
gtgtttaggt tatcggtgat tgtagttttt ggacggtatc gtttattata ggttttggag 420
gttgttttta cggttttttg atagggtttt tgttggtttg ggggtttttg attaggggag 480
cggttttagg aggggagaga ttcgcgtttc gggtttagcg tagtcgtttc gagtaggatc 540
gggattttta ttaagcgggc gtcgttttac gattttcgcg cgtttttagg attattcggg 600
tacgtggtag gtcgtttgta cgttcgcgga ttatttttgt gataggaaaa ggtacgggtt 660
atttggtaaa ttaaggtata gagttttagg cggaagttgg gaaggcgtcg ttcggtttgt 720
atcggtcgaa gggttattcg ggttaggcgt atagggtagc ggcgttgtcg gaggattagg 780
gtcggcgtgt cggcgtttag cgaggatgcg tagattgttt taggttcggc gtcgtcgtat 840
tgggtatgcg tcgattcggt cgggcgggaa tatttcgttt cgttcgggtt tcgttttagt 900
ttcgttttcg cgcgtttcgg tttcgttttc gcgcgttttt ttgttttttt taggttttcg 960
gtttcgtttc gttttagatt tcgttttacg tcgttatttt cgtgtttttc ggtttcgttt 1020
tcgcgtttcg gatatgttgg gatagttcgc gtttttagaa cgttttgcgt ttcgacgttc 1080
gtaggttttc gcggtgcgta tcgtttgcga tttggtgagt gtttgggtcg tttcgttttc 1140
ggaagagtgc ggagttt 1157

<210> 194
<211> 1157
<212> DNA
<213> Artificial Sequence

<220>
<223> chemically treated genomic DNA (Homo sapiens)

<400> 194

gagtttcgta ttttttcggg agcgaggcga tttagatatt tattaagtcg taaacggtgc 60
gtatcgcgag gatttgcggg cgtcgggacg taaagcgttt taggggcgcg ggttgtttta 120
gtatattcgg ggcgcggggg cggggtcgag gggtacgggg atggcggcgt ggggcggggt 180
ttagagcggg gcggagtcga ggatttgaga aaagtaagag agcgcgcggg ggcggggtcg 240
gggcgcgcgg gggcggagtt ggggcggagt tcgggcgggg cggggtgttt tcgttcgatc 300
gggtcggcgt atgtttagtg cggcggcgtc gggtttgagg tagtttgcgt attttcgttg 360
gacgtcggta cgtcggtttt ggtttttcgg tagcgtcgtt gttttgtgcg tttgattcgg 420
atggtttttc ggtcggtata agtcgggcgg cgttttttta gttttcgttt gaggttttgt 480
gttttagttt gttaaatggt tcgtattttt ttttgttata gggatagttc gcgggcgtgt 540
aagcgatttg ttacgtgttc gagtggtttt gaaagcgcgc gggggtcgta ggacggcgtt 600
cgtttagtga gaatttcggt tttgttcggg gcggttacgt tgagttcgga gcgcgagttt 660
tttttttttt ggggtcgttt ttttagttag ggatttttag attagtagag attttgttag 720
ggggtcgtgg gggtagtttt tagggtttgt ggtgggcgat gtcgtttagg ggttgtagtt 780
atcgatggtt tagatattgt tagagtttga tttagtcgtt ttttagagag tatatagggt 840

gttaggtttt gtttggggtt gtgtggacgt tattaggagt tgaattgtgt ttattagaga 900
agttatgttg gagttttaat ttttagtatt ttataatgtg attttatttg gagatgggga 960
ttttatagaa gttattaatt agtgggtttt ttatttagta tgattcgtgt ttttataggt 1020
agtaggtcgt ttggggagga ggattggagg gttgtatttg taagttatta gaagtttaag 1080
aggatagatt tttttttgga gttttcggag ggagtagagg atattttttt ttggagtttt 1140
tagagggagt aggattt 1157

<210> 195
<211> 8093
<212> DNA
<213> Artificial Sequence

<220>
<223> chemically treated genomic DNA (Homo sapiens)

<400> 195

ttatggaagg aaatgagatt agttagcgag ttgatataga ataaggtagt ttaaaataaa 60
tttttttaaa aatacgaaga atagatattg aagggaagag gtgtttgtaa agattaagaa 120
agtatatttg gagatggtat ttttttaaag ttaaagttat taagtgttta agtgttttaa 180
ggagggtaag attattaata aggatttagt atagtagagt aatttgagtg gtaatacggg 240
atattgggaa tataaatttg ttaagaaaat tagtaggaat gagttatagg atagtaattg 300
gtaaggattt aataattttt tttttaatgt acgtatttta attatattta ttgttataat 360
aggattagta ataattatat ttatttaaaa aaaaaaagat tgttatgtag ttatagaatt 420
atttaatata gaaaatagta aaatatattt tttttttttt tttttttttt tttttttttt 480
ataaataaga ttagtttata gtaaattttt tatagttaag ggttaattta aaatttttgg 540
tttatatttt ttttttattt aatgattata tgatgtaaat taattttatt aatattttaa 600
gtaaaatata ttggaatttt ataaaatgta taagatttta atatttaagg aattggggtt 660
agaaagtaga agtggttttt aggtttttta gttttttttt taagtaataa agttttgttg 720
tgaatattta aagttattgg gaaattatcg gtagattttt ttgttttttt ttttcggttt 780
tttattatgt tgttttttta gatatgtaag tttattttat taattaaaat tttagtttga 840
ttatttttga taagtattta atcgatatgt aatttttttt ttgttttaaa tatgtataat 900
aggatagagt ttttaaattt gatttaatta ttaatttttg atttataagt gttatggtga 960
gttaatagaa tttattaatg tttttattgt attttattag ttaaatttag aaggttggaa 1020
ttagtttttt ttatttagta ttttgttagt ttgtttagtt tgtattttta attttgtttt 1080
taatggtaat ttgttttatt ttttgaaata aaataattta tattttggga gggttaattt 1140
tttattgtgt taggttgttt tatgtattgt aggggtgagt gtttttaggt ttaaatgtta 1200
atagaagttt ttagtaaata tgataattaa aaaagtgttt ttatatattt tttagtattt 1260
tttggaatga taggttattg tttttagttg aaagttattg gtataatttt ggtttttaag 1320
tttttatgtt atttatttta attgtttaga tattaatttt atttaaattt ttagttatag 1380
tttggttttt tgaatttgtt ggattagtaa ttatagatta aggtgtttta atagttaaga 1440
taggattttg gaataagagt ttttaaattg tataatattt gagaggattt atgaatataa 1500
attgggtttt gtttataatt agttttatat aatgaatttt aagattgttt ttttatggtg 1560
aatagaagtt tggaaattat tgttttggta taaagtagat tattttagta gaaatataga 1620
attattgtaa tttgtgaata agattgtttt taaatatttt tatttgtgtg ttattttata 1680
tatagaatgt gtacgatagt tttaaatttt agaataaatt tatttttagt atttaataaa 1740
atttgatatt gtattatttt aaaataaagt gtttatttta ggtaggattt tttaaaataa 1800
agtagtaata tttacgtaga taagataaaa aagttaaaat attttatatt ttttaatttt 1860
ggagtgtaat tttttttttt tatcgttttt gatagggtta aatttgtgtt tatagtaaaa 1920
aaaaaaaaaa aaagaattat taattggtaa ttattaagat tttatattta ttatagtttt 1980
ttaataggta agttgataaa atagttttta gttattaaaa aaaaaattta aggaaaattt 2040
ttaataatta gttttatttt taaattgtat gaagtttttt atatttgaaa tttaaaaatg 2100
attttaatga ttttttttat tagtaatgtg ttattattga ggtgggtgat ggggagggaa 2160
gagggaagaa atttgttagt attattttcg aatttagaaa tgtttaaaaa aaagttttaa 2220
atattttgat ggttagataa aatattttta ttgttatgta tgggtttttt ttttggaaat 2280
ttatgaattt gttatgtgag tttttgtaaa ttggtttaaa agtatattta aggagttgat 2340
aatttaagat tatatgaatt agaattttaa tattttatta aaataagagt tgaaattttt 2400
ggtatttatt tttagaatat ttaaaaaata gattgtaaat ttaatttata ttaatattaa 2460
atttttttaa aattaattat attataaaag ataatgattt tgttattaaa ttaagtttta 2520
aaaaaggtgg tatttttatg ttttagtttt atgttgttat ttgagatgaa aaaaaaggta 2580
attgttagaa ttttaattgt gattagtttg gacggttggt atttggtatt tttttataat 2640
ttaaattagt aaatttgaaa atttaaatta atttttgttt ttagaattaa atttttgttg 2700
agttttttaa ttttgtatta taaagtttta tggattagat ttattttttt tttttataag 2760

gttattttaa tcgggtttta ttttagttgt ggtaattcga aatagggttt ggagatgttt 2820
atttgttaat ggttgattta aagtgtgttg gtttcgagtt aaatatgaaa aagttttttt 2880
atagatttgg ttattattaa ataaggatgt tattttatag gtaatttttt tgataattgg 2940
gtaggattta gtagataatt tagtataaaa ttgttttaag tttttgattt tgtatttatt 3000
ttaaagatta gtgtttgttt gaagttttgt tagttttatt tttattgaaa taggtgtata 3060
tttaggttta aagttaaatg gatttgaaaa aagttttaag ttttttttaa tgaatttgag 3120
gtttttaaaa tgttttttaa attttttttg gagacgatag attattattt gatatttatt 3180
aggattaaat atttttttat tttttttatt ttgttgtttt agtttattaa taattttttt 3240
gagggtagga aagtttgtta gatttttttt tttaatatgt ttttgaaata aatttagttt 3300
aatttcgaaa gtataaatat ggttaaaggt agtagtagta aagattttat taattcgtaa 3360
ttttttattg agttttcgaa ggtgttttat aatgtttatt aagaagttaa agttataaag 3420
ttattgtttg tttgagaagt ggattgttgt tgtttttatt gaaattaaga acgtttttat 3480
tttttttttt agagagaata ttagttttaa agtttttttt tttttaagtt aattgtttag 3540
atatcgttta ttaatttttt tattatgttt tgatttagat ttcgttagta aagtttgaaa 3600
tttaggtcgt ttaacgtttt tggttttaat taaaattatt tattcggata tggtatttat 3660
gatttgatta atatttatat tataggagtt taatagtttt aagtgaagaa attttgaata 3720
atgaatgata ttttaatagt tggggtttac ggtttttttt tttatgtatg agacgagttt 3780
tgagttttta ttaattattt ttaaagtatg ggtcgtggtt agtttaatta ttttttgtaa 3840
gttaagtttt gttgtttgta gggattttag gattgtcgat atgagcgtat taatattgaa 3900
atgatgagtt aggttgatta tggttagaag atttttttgt atttttaatt tagggtttat 3960
atcgcggata aagattagga ggtagttttt ataggttata aaagtttggt cgtttaaggt 4020
aagagaatag gttttaaagt ttttggttcg gttaaaaagt tggttgcgta gatttttgtt 4080
aatgtttagg attttttgtt ttgtgatatt tggagataag ttaacgtttt gtaggacgtt 4140
tatatgttcg ggtagtattt tttttagtaa tatttttatg tattggtata taaagttttt 4200
tttattttag tcgcgatttt ttaaggttaa gaggcggtag agttcgaggt ttgtacgagt 4260
agtttttttt ttaggagtga aggaggttac gggtaagtcg ttttgacgta gacgttttat 4320
tagggtcgcg cgttcgtcgt tcgttatata tcgttcgtag tattcgtgtt tagtttcgta 4380
gtggcgtttg acgtcgcgtt cgcgggtagt tacgatgagg cggcgataga ttaggtatag 4440
ggttttatcg tttttcggag gttttattat taaataacgt tgggtttatt cgggtcggaa 4500
aattagagtt tcgtcgattt ttattttgtt ttttttgggc gttatttata ttttgcggga 4560
ggttataaga gtagggttaa cgttagaaag gtcgtaaggg gagaggagga gtttgagaag 4620
cgttaagtat ttttttcgtt ttgcgttaga ttattttagt agaggtatat aagttcggtt 4680
tcggtatttt tgtttttatt ggttggatat ttcgtatttt tcgagttttt aaaaacgaat 4740
taataggaag agcggatagc gatttttaac gcgtaagcgt atattttttt aggtagcggg 4800
tagtagtcgt tttagggagg gacgaagaga tttagtaatt tatagagttg agaaatttga 4860
ttggtattta agttgtttaa ttaatagttg tcgttgaagg gtggggttgg atggcgtaag 4920
ttatagttga aggaagaacg tgagtacgag gtattgaggt gattggttga aggtattttc 4980
gttgagtatt tagacgtttt tttggttttt ttggcgttaa aatgtcgttc gtggtagggg 5040
ttattcggcg gttggacgag atagtggtga atcgtatcgc ggcgggggaa gttatttagc 5100
ggttagttaa tgttattaaa gagatgattg agaattggta cggagggagt cgagtcgggt 5160
ttatttaagg gttacgattt aacgggtcgc gttatttaat ggcgcggata cgtttttttg 5220
ttcgggtaga ggtatgtata gcgtatgttt ataacggcgg aggtcgtcgg gttttttgac 5280
gtgttagtta ggtttttttt tttttcgtag atcgtgtgtt tttttatcgt tttttttcga 5340
gattttttaa gggttgtttg gagtgtaagt ggaggaatat acgtagtgtt gttttaatgg 5400
tatcgttaat taagtaagga agttatttaa tttaaaatta tgtatgtaga atatgcgaag 5460
ttaaaagatg tataaaagtt taagatgggg agaaaaattt ttttttagag ggtattgtgt 5520
tattgttttt ttgtttttta tttattttag aaattatttg tttatattta aaggtataat 5580
ttattttgag ttttttttaa aataaatcgt ttgtagtttt aggataggtt gatgtatttt 5640
gggtttgatt tttgattatt ttattgttaa attagtgatt ttttttagtg tttttttgtt 5700
ttttatttcg gaggacgtat ttcgaagata ttagatttta tgggttattt ttggattttg 5760
aagtttataa ttgtgataaa aaatgtgaag ggaagagatt tgaaatatgt ggaaggaaaa 5820
gtgagtgtag attataaatt tttaaaaaga taagtttaaa atatatttaa acgttatgtt 5880
agattatttt gttaaaatta gttgttagtg acgttcgtac gttaatagaa aaaagaatgt 5940
tttagtttgg agtggtaggt ttttagaggg atttattgtg aaagtataaa ttatttaggg 6000
taatgggatt gagagaatag tgggtagaaa ggattattga aggaaaggaa gagaattgga 6060
aggtagatga aagaaggagt aagaatttgg ggatgttttt ttttttttat ttgtaatagt 6120
agtaatagaa gtaatggtag attggttttt gtttttattg tgttagaatg aattgatagg 6180
ataattgggt ttattattgt attgtgttag aatattgtaa aataaaatta aatatattag 6240
tttggtggtt tgtaattaat tatttaagtg gagattttta tttttttttt attttttttt 6300
tagacggagt tttattttgt tatttaggtt ggagtgtagt ggcgcgattt tagttgattg 6360
taattttttt tttataggtt taagggagat ttttttgttt tagtttttcg agtagttagg 6420
attataggta tgtgttatta tatttggtta attttgtatt tttagtagag atgggatttt 6480
tttatgttgg ttaggttggt gttaaaattt tcgattttag gtgaatcgtt tgttttagtt 6540

ttttaaagtg ttgggattat aggcgtgagt tatcgcgttt tgtagttttt tgtattttta 6600
atagagatag ggttttatta tgttagttag gatggtttcg attttttgat tttaggtgat 6660
ttgttcgttt tggtttttta aagtgttggg attataagta tgagttatcg cgttcggttt 6720
aagtggagat ttttatatgg agtttagtta tatttttttt aatatataag ttgagatgat 6780
taatataatt ttaatatagg ggtttatgag aaatgtttgt aatatttaag taatttattg 6840
tttttttttt ttttttttta agatgaagtt ttattttgtt gtttaggcgg aagtgtagtg 6900
gcgtgatttt ggtttagggt aatttttgtt ttttggtttt aagcgatttt tttgttttag 6960
tttttcgagt agttgggagt ataggcgtgt atgattatat tcggttaatt tttttatttt 7020
tagtagagac ggggtttttt tatgttggtc gggttggttt tgaatttttg attttaggtg 7080
attcgtttat tttagttttt ttaagtgttg ggattatagg tgtgagtttt cgtgtttagt 7140
ttattatttt atttttgaat aaagaattgt ttgtgtgggg aatagataat ttttttttat 7200
gtagtttttg ttagaaaatt tgtttttttt agtagttggt ttgtgtttat aggttatttt 7260
ttgaaagtat aaaaaattta ttgatttttt ttttttgggt tttttttttt ttttgagata 7320
gagttttgtt tttgttgttt aggttggagt gtaatggcgc gattttagtt tatcgtaatt 7380
tttatttttt gggtttaagt gatttttttg ttttagtttt ttgagtagtt gggattatag 7440
gtatgcgtta ttatgtttgg ttaattttgt atttttagta taaatggggt ttttttatgt 7500
tggttaggtt ggttttaaat ttttgatttt aggtgattta ttcgttttgg ttttttaaag 7560
tgttgggatt atgggtgtga gttattgcgt ttggttagaa aatttattga ttttttaaag 7620
atttattaat tttttgtatt attttttttt ttttttttta tcgtaaatat aaaagggaat 7680
agtagagaaa attatttaga attttatttt ttagtgatat tatttagtga tattttatta 7740
gagttattta ggaatttgag gttgaataaa gtttaggtaa aagtaaaatt agttgagaag 7800
agatatttgt taaaagaaat ttatttttaa ttttatttgt tgtttttttt agaggaatag 7860
aaatagtgtt gaatgtttta ttagaaatga tggttgtttt gttcgttttt tttttttttt 7920
ttatataata tgtaaattta tatagtgtat gagtttgtaa gataaaggaa aaatacgtta 7980
atgaggtatt attgtttgta tttggagttt gttattattg tttggtttat attaaaatat 8040
gtatattaga gtagttgtag attgataaat tatttttgt ttgatttgtt agt 8093

<210> 196
<211> 8093
<212> DNA
<213> Artificial Sequence

<220>
<223> chemically treated genomic DNA (Homo sapiens)

<400> 196

attggtaaat taaatagaaa ataatttatt agtttgtaat tattttaatg tatatatttt 60
aatatgagtt aagtaatgat aataaatttt aaatataaat aatagtgttt tattaacgtg 120
tttttttttt gttttatagg tttatatatt gtatgagttt atatattgtg tgagagagag 180
ggaagagacg ggtagagtaa ttattatttt taataggata tttagtatta tttttgtttt 240
tttaggaaag atagtaagtg aagttaaaaa tagatttttt ttggtagatg ttttttttta 300
attaatttta tttttatttg aattttattt agttttaggt ttttaagtga ttttaataaa 360
atgttattaa ataatgttat taaaaaataa aattttgaat gatttttttt attatttttt 420
tttatattta cgatggaggg gaaaaaaaaa agtaatgtag aaagttaata aatttttagg 480
aagttaatga attttttggt taggcgtaat ggtttatatt tataatttta gtattttggg 540
aggttaaggc gggtggatta tttgaggtta ggagtttgag attagtttga ttaatatgga 600
gaaattttat ttgtattaaa aatataaaat tagttaggta tggtgacgta tgtttgtaat 660
tttagttatt taggaggtta aggtaggaga attatttgaa tttaggaggt ggaggttgcg 720
gtgagttgag atcgcgttat tgtattttaa tttgggtaat aagggtaaaa ttttgtttta 780
aaaaaaaaaa aaaatttaaa aaaaagaagt taataaattt tttgtgtttt taaagagtag 840
tttataagta tagattaatt gttagagaaa ataaattttt tagtaggggt tgtatgagaa 900
agagttattt attttttata tagataattt tttatttaga aataagataa taggttgggt 960
acgggggttt atatttgtaa ttttaatatt tggggaggtt gaggtgggcg gattatttga 1020
ggttaggagt ttaagattag ttcggttaat atggagaaat ttcgttttta ttaaaaataa 1080
aaaaattagt cgggtgtggt tatgtacgtt tgtattttta gttattcggg aggttgaggt 1140
aggaagatcg tttgaaatta ggaggtagag gttgttttga gttaagatta cgttattgta 1200
ttttcgtttg ggtaatagag taagatttta ttttaaaaaa aaaagaaaga agataataag 1260
ttatttaaat attatagata ttttttatga gtttttgtat tgaagttgta ttagttattt 1320
taatttatat attaaaaaga gtataattgg attttatata aaaattttta tttgagtcgg 1380
gcgcggtggt ttatgtttgt aattttagta ttttgggagg ttaaagcggg tagattattt 1440
gaggttagga aatcgagatt attttggtta atatggtgaa attttgtttt tattaaaaat 1500
ataaaaaatt gtagggcgcg gtggtttacg tttgtaattt tagtattttg gaaggttgag 1560

gtaggcggtt tatttgagat cgagagtttt gatattagtt tgattaatat ggagaaattt 1620
tatttttatt aaaaatataa aattagttag gtgtggtggt atatgtttat agttttagtt 1680
attcgggagg ttgaggtagg agaatttttt ttgaatttgt gaggaggagg ttgtagttag 1740
ttgagatcgc gttattgtat tttagtttgg gtgataaagt gagatttcgt ttaaaaaaaa 1800
aataaaaaaa aaataaaaat ttttatttaa gtaattaatt ataagttatt aagttagtat 1860
gtttagtttt gttttatagt attttggtat agtataataa taggtttagt tgttttgtta 1920
atttatttta atatagtaga aataaaagtt agtttgttat tgtttttgtt attattatta 1980
taagtgaaaa ggaaaaaata tttttaggtt tttgtttttt tttttattta ttttttaatt 2040
tttttttttt tttttagtgg ttttttttat ttattgtttt tttagtttta ttgttttgaa 2100
tagtttatat ttttataata aattttttta gaaatttatt attttaaatt gaagtatttt 2160
tttttttatt aacgtacgga cgttattaat aattgatttt aataaaataa tttgatataa 2220
cgtttaggtg tattttgggt ttgttttttt ggaagtttat agtttgtatt tatttttttt 2280
tttatatgtt ttaaattttt tttttttata ttttttgtta tagttataag ttttaaaatt 2340
taaggatgat ttataaaatt tggtattttc gaagtgcgtt tttcgaaata aaggatagga 2400
aaatattaag aggggttatt aatttaataa tagggtaatt agaagttaag tttaaagtgt 2460
attagtttgt tttaaaatta taaacgattt gttttgaaag aaatttaaaa tgaattgtgt 2520
ttttggatgt gaatagatga tttttggaat gaatgaaaag taagaaaata gtaatatagt 2580
attttttgaa aaaaggtttt tttttttatt ttaagttttt atatattttt taatttcgta 2640
tgttttgtat atataatttt aaattaagtg gtttttttat ttagttaacg gtattattaa 2700
gataatatta cgtatatttt tttatttata ttttaaataa tttttaaaag gtttcggggg 2760
agagcggtaa agaaatatac ggtttgcgga aaaggagaag gtttgattgg tacgttaggg 2820
aattcggcgg ttttcgtcgt tgtgggtatg cgttgtatat gtttttgttc gggtaaagag 2880
gcgtgttcgc gttattgagt gacgcggttc gttaagtcgt agtttttaag tgagttcggt 2940
tcgatttttt tcgtattagt ttttaattat ttttttgata gtattagttg gtcgttggat 3000
aatttttttc gtcgcgatgc ggtttattat tgtttcgttt agtcgtcgaa taatttttgt 3060
tacgaacgat attttggcgt tagaagagtt aaggaaacgt ttagatgttt aacggaagtg 3120
tttttagtta attattttag tgtttcgtgt ttacgttttt tttttagttg tagtttacgt 3180
tatttagttt tattttttag cggtagttat tgattggata gtttgaatgt tagttaaatt 3240
ttttaatttt gtgggttgtt gggttttttc gttttttttt gaagcggtta ttgttcgtta 3300
tttagaagga tatgcgtttg cgcgttagag atcgttgttc gtttttttta ttggttcgtt 3360
tttaggagtt cggggaatac gaaatattta gttaatagga gtagagatgt cggaatcggg 3420
tttgtgtgtt tttgttgagg tgatttggcg tagagcggag gaggtgtttg gcgtttttta 3480
ggtttttttt ttttttttgc ggttttttta acgttggttt tgtttttgtg gtttttcgta 3540
gaatgtggat gacgtttaaa agaagtaaga tggaagtcga cgaggtttta gtttttcggt 3600
tcgagtggat ttagcgttat ttggtggtgg agttttcgga gggcgatggg gttttgtgtt 3660
tggtttgtcg tcgttttatc gtagttattc gcgaacgcga cgttaggcgt tattacgagg 3720
ttgagtacga atattacgag cggtatgtgg cggacggcga gcgcgcggtt ttggtggagc 3780
gtttgcgtta gggcgatttg ttcgtggttt tttttatttt tgaagagaga gttgttcgtg 3840
taggtttcgg gttttgtcgt tttttggttt tgaagggtcg cggttggggt gagggggatt 3900
ttgtatatta gtgtatggag gtgttgttga gagaggtatt gttcgagtat gtaagcgttt 3960
tgtaaggcgt tgatttattt ttagatatta taaggtagag gattttgagt attgatagga 4020
atttacgtaa ttagtttttt aatcgagtta gggattttaa agtttatttt tttgttttgg 4080
acgattaggt ttttgtggtt tatgagaatt attttttggt ttttattcgc ggtgtaggtt 4140
ttgagttgga ggtgtaagaa gattttttga ttataattaa tttgatttat tattttagtg 4200
ttggtgcgtt tatgtcggta attttagagt ttttgtagat agtagggttt agtttgtaga 4260
gaatggttgg attgattacg atttatattt tgaggatgat tggtgagaat ttaggattcg 4320
ttttatatat gagagaaaag gtcgtaagtt ttaattgttg gaatgttatt tattatttag 4380
gattttttta tttggaattg ttgagttttt atgatgtaga tgttaattag attataaata 4440
ttatattcga atggatagtt ttgattaaga ttagaggcgt taggcgattt gaattttaga 4500
ttttattaac ggaatttgaa ttagagtatg gtgaaagggt taatggacga tgtttgaata 4560
attggtttag gagagggaaa attttaaaat taatattttt tttaagaaaa gaaatggaag 4620
cgtttttggt tttagtaggg gtaataatag tttatttttt agataaataa tggttttgtg 4680
attttggttt tttggtggat attatggaat attttcgaga atttagtgaa gaattacgag 4740
ttagtaaagt ttttgttgtt gttgtttttg attatatttg tattttcgaa gttaagttga 4800
atttatttta aagatatatt gaggaaaaaa atttaataga tttttttgtt tttagagaag 4860
ttgttgatga gttaaaatag taaaataagg aagatgaaaa aatatttgat tttgataggt 4920
attaaatggt gatttgtcgt ttttaaaaag aatttgagag atattttaag gattttaggt 4980
ttattaaaaa ggatttagaa ttttttttaa atttatttaa ttttaaattt gaatatgtat 5040
ttattttagt gagggtggag ttaataaaat tttaggtaaa tattaatttt tggaatgaat 5100
atagaattaa agatttgggg tagttttatg ttggattgtt tgttgaattt tatttaatta 5160
ttaaaggggt tgtttgtaag gtggtatttt tgtttgatag taattaaatt tgtgaaaagg 5220
tttttttata tttgattcga aattaatata ttttgagtta gttattaata gatgagtatt 5280
tttaagtttt gtttcgggtt gttataattg aaatggagtt cggttgggat gattttgtga 5340

gagaaagaaa tgaatttaat ttataaggtt ttgtagtata agattgaaaa atttaataag 5400
aatttaattt taaaagtaaa aattggtttg agtttttaag tttattaatt tggattgtga 5460
gaaagtatta agtattagtc gtttaaattg attataatta aaattttgat agttgttttt 5520
tttttatttt taaatggtag tatgggattg aaatatgaga atgttatttt ttttaaaatt 5580
tagtttagtg ataaagttat tgttttttat gatatagtta attttaaaga gatttagtat 5640
taatgtgagt tgaatttgta gtttgttttt taggtgtttt gaagataaat gttaaaaatt 5700
ttagttttta ttttaatgga gtgttaaaat tttgatttat atagttttaa attattaatt 5760
ttttaaatgt gtttttgaat taatttgtag aagtttatat agtaagttta taagttttta 5820
aaaaggaagt ttatatataa tagtggaggt gttttgttta attattaaaa tgtttgagat 5880
ttttttttaa atatttttga gttcgaaggt aatattgata gatttttttt tttttttttt 5940
tttattattt attttagtga taatatatta ttgatagagg aagttattag aattattttt 6000
aagttttaga tataggagat tttatgtaat ttggagataa gattaattat tgggggtttt 6060
ttttggattt tttttttaat aattgggggt tattttatta gtttgtttat taaaggatta 6120
tggtaagtat agaattttaa tggttgttag ttagtaattt tttttttttt tttttttatt 6180
gtagatataa gtttggtttt attaaaaacg atgaggaaaa aagattgtat tttaggatta 6240
ggaggtgtga gatattttag tttttttgtt ttatttgcgt gggtattgtt gttttatttt 6300
aaaaaatttt gtttaaagta aatattttgt tttaaaatga tatagtatta gattttgtta 6360
gatgttagaa atggatttat tttaaaattt ggaattgtcg tatatatttt atatgtaaga 6420
tagtatataa gtagaaatat ttaaaagtag ttttatttat agattgtagt aattttgtat 6480
ttttattaag ataatttgtt ttgtgttaaa atagtaattt ttaaattttt gtttattatg 6540
aaaaggtaat tttaaagttt attatgtaaa attaattata aataggattt aatttatatt 6600
tatagatttt tttaagtatt atataattta aaaatttttg ttttaaagtt ttgttttaat 6660
tattgaaata ttttaatttg tggttattaa tttagtaaat ttaaggaatt aggttatgat 6720
taagaattta ggtggaattg atgtttgggt aattaaaata aatggtataa gagtttaaaa 6780
attaaagttg tgttagtggt ttttaattag aggtagtaat ttgttatttt agaggatgtt 6840
gagaaatgtg taggggtatt tttttggttg ttatatttat taggggtttt tgttggtatt 6900
taagtttaaa gatatttatt tttgtagtgt atgggatagt ttggtataat gaagaattag 6960
tttttttaaa atgtagatta ttttatttta agggataggg tagattatta ttagaagtaa 7020
aattaaaagt ataagttggg taaattgata gaatattaga taggagagat taattttaat 7080
tttttaaatt tggttagtaa agtgtaataa aggtattgat aagttttgtt agtttattat 7140
agtatttgta aattaggaat taataattga attagattta agggttttgt tttgttatat 7200
atatttaagg tagaaaaaaa gttatatgtc gattaggtat ttattaagaa tggttaagtt 7260
gagattttgg ttaatagagt aagtttatat atttagagaa ataatatagt ggaaaatcga 7320
aaaaaaaaaa tagaaaaatt tatcggtaat tttttaatag ttttgaatat ttatagtaga 7380
gttttattat ttgagagaaa gattggaaga tttgaaagtt atttttgttt tttaatttta 7440
gttttttaaa tattgaaatt ttgtatattt tgtgaaattt tagtatgttt tgtttaaggt 7500
gttaataaaa ttagtttgta ttatgtagtt attgagtgag ggggagatat aagttaagga 7560
ttttaaattg atttttagtt atagagaatt tgttataagt tagttttgtt tgtaaaaaaa 7620
aaaaaaaaaa aagaaaaaga aaaaagtgta ttttattgtt ttttgtatta agtaattttg 7680
taattgtatg gtagtttttt tttttttaaa taaatatagt tgttattggt tttgttgtag 7740
tagtgaatat agttaaaata cgtatattaa aaaaaaaatt attaggtttt tattagttat 7800
tgttttatag tttattttta ttagtttttt tgatagattt gtatttttag tgtttcgtat 7860
tgttatttaa attgttttat tatgttaagt ttttgttaat agttttattt tttttgaaat 7920
atttgaatat ttgatgattt tagttttgag gagatattat ttttaggtgt gtttttttag 7980
tttttgtagg tatttttttt ttttaatatt tgtttttcgt atttttaaaa aaatttgttt 8040
tagattgttt tgttttgtgt tagttcgtta gttgatttta ttttttttta tgg 8093

<210> 197
<211> 6521
<212> DNA
<213> Artificial Sequence

<220>
<223> chemically treated genomic DNA (Homo sapiens)

<400> 197

ataaaattat gtatgggtaa aagatttaat taggtgtagt ttagaaaaac ggattttttt 60
tttttttttg agatagagtt ttgttttgtt atttaagttg gagtgtagtg gtgtaatttc 120
ggttaattgt aattttcgtt tcgtaggttt aagtgatttt tttgttttag tttttcgagt 180
ggttgggatt ataggcgtgt gttattatta tatttgatta atttttgtag ttttagtaga 240
gacgggaggg ttttattatg ttggttaggt tgtttttaaa tttttgattt taggtgattt 300
atttatttta gtattttaaa gtgttgggat tataggcgtg agttattatg tttagattta 360

aatttttttt gttttttttt tttttttggg ggtggggggt agggttttat tttgttgttt 420
aggttggagt gtagaggttt gattttggtt tattgaaatt tttatttttt aggtttaagt 480
aattttttta ttttagtttt tcgagtagtt gggattatag gcgtgtatta ttatatttgg 540
ttaatttttt gtatttttgg taaagacggg gttttgttat gttgtttaag ttgtttttga 600
atttttgggt ttaagcgatt tttttgtttt atttttttaa cgtgttggaa ttgtaggtat 660
gagttattat gtttggaata aagttttatt ttttaagtgg attatttaaa aaaatgtttt 720
atggttaggt acgttatttt tttttagttt gggtaagtga gtcgagattg tgttattgcg 780
gtttggtttg agcgatagag ggagatttta ttttagataa ataaataaat aaaattaaaa 840
taaatttaaa tattttttat atgataaaat tatttttaat ggtaattata aaatgaataa 900
taagggttaa gaaataaatt tagtgaatat tttttttttt tgaattttgt aaatataatt 960
atgttagtat aaaaatagtt atgtattgtt taataatagg gatatgtttt gagaaatgag 1020
ttattaggta attttgttat tgtgtaaata ttatagagtg tatttttata aatttagatg 1080
gtgtagttta ttatttattt aggttatatg gtatagtttg gtgtttttag gttttaatgt 1140
ttatagtatg ttattatatt gaatattgta ggtagttgta agataatggt atttgtgtat 1200
ttattgtaga gatagggttt tttcgtttgt cgtttaggtt ggagtgtagt ggtataatta 1260
tagttttttg tagtttttaa tttttgggtt taagtgattt ttttatttta gttttttaag 1320
tagttaggat tatacgtttg tgttattagg tttgtttatt gtttttgttt gttttttggt 1380
agagatggag ggttttatta tattgtttag attggttttg aatttttggt tttaagtaat 1440
ttttttgttg ttttggtttt taaagtgttg gtattatagg aatgagttaa aagtttaata 1500
tggttgttga ataaaagatt gatttaatta agtacggatt ttgtttttat ggagttgaag 1560
gtcgttagag tagataaaat gagttattat ttttatatga gttagacggt ggataaagtt 1620
attattgagg gtaggtaaag aattgggttg gtttaaagat gttagggatt ttttttagat 1680
tagtggtaga gggttggtta aggagttttt tgaaggaggt ggttttttat ttgagtttta 1740
aagaatagaa gagattagat agggaggata gtaaaatttt tttttttttt tgaaatagtt 1800
tcgttttgtc gtttaggttg gagtgtagtg gcgcgatttt ggtttattat aagttttgtt 1860
ttttgggttt acgttatttt tttgttttag ttttttgagt agttgggatt ataggcggtc 1920
gttattacgt tcggttaatt tttttttttt ttttttttta gtagagacgg ggttttatta 1980
tgttagcgac gatttttgtt tgtttgtttg tttttgagat agagttttat tttgtcgatt 2040
aggttggagt gtagtggcgc gatttcggtt tattgtaatt tttgtttttt aggtttaagt 2100
gatttttttg ttttagtttt ttgagtagtt gagattatag gtgtttgtta ttatgtttag 2160
ttaatttttg tatttttagt agagacgggg ttttattatg ttggttaggt tggtttcgaa 2220
tttttgattt taggtgattt gtatatttcg gttttttaaa gtgttggtat tagaggtatg 2280
agttattgta tttggttagt aaaattattt gtgatatagt agtaagtgta taagtattga 2340
tttattagag ttgaaggtgg ttgaggtatt taagtgggtt tttggggatt ttgttatgtt 2400
agggaatatt tttttagata agggattagg aggtttggag ggtttttaag gaagagttag 2460
ggtagtaggg gtattttgga gttaggggtt atggttatat gttaatagga ttagaaatat 2520
tttaatgtat ttaattaatt tatataatta tattggtggg tgtttattga atattagttt 2580
tagatatgga tttgaaatgg ttttattttt ttttagtttg tttaagttaa tggagtttta 2640
ttagaatgtg agttataaaa tagtagagtt attttttatt taatgttgta tttgtagggt 2700
ttagaatagt atttgaagga ttggcggagg tttaataaat tgttaaaagg gtagattagt 2760
taggtgtgat ggcgtgtgtt tgtagtttta gttatttggg aggttgaggt aggaggatta 2820
tttgatttta ggatttttgg gttgtagtgt agtatattag ttaggtgttt gtattaagtt 2880
ttgtattaat ttggttgttt aaggatggtt gaattgattt aggtggaaat tggagtaggt 2940
taaaatttaa tgttgattag taatagggtc gtatttgtaa atagttatcg ttttttagtt 3000
tgggtaatat agtgagattt tatttttaaa ataaatttta aaaataatta attagaaaaa 3060
aaaattagtt tgtaatttta gtattttggg aggttaaggc gggtagatta tttgaggtta 3120
ggagtttaag ataagtttgg ttaatatggt aaaattttaa tttttattaa aaatataaaa 3180
attagttggg cgtggtggta ggtgtttgta gttttagtta tttaggaggt tgaggtaaga 3240
gaatcgtttg aatttgggag gtggagattg tagtgggtcg agatcgcgtt attttatttt 3300
agtttgggta gtagagtgcg attttgtttt aaaaaaaaaa aaaaagaaag aaaagaaaag 3360
aaaaagaaaa gaaaagaaaa aaattgggag gtttaagttt atttttggtg tttttatatt 3420
tttttgtttt atttttttgt atttagtttt tttttgtaat ttgtgtttta tattagtttt 3480
taagttttta acgtgattta gtatgagaat tggattttgt tatttttttg tttataatat 3540
tttatgtttt ttttttgttt agaatattat tttttttatt gtttttatta atggaattgg 3600
tattttttta aggatatgat taaatttgtt tatttttata ttatttttta aagtagaatt 3660
tatttttttt tttttaatat gatgatattg atagggtttg tttttattta ttagattgtg 3720
agttgtttag ggtaggtagc gttttttgtt tttgtttttg tttttttttt ttgagatagg 3780
gttttgtttt gttatttagg ttagagtgta atggtatagt tttagtttat tgtagtttta 3840
atcgtttcgg tttaaattat tattttattt tagttttttg agtagttggg attataggta 3900
tatgttatta tatttggtta atttttttgt atttttagta gagatagggt ttggttatgt 3960
tgttcgggtt ggtttcgaat ttttggattt aagtaattta tttattttag ttttttaaaa 4020
tgagggatcg tgttttattt atttttatgt ttttagttta tagtttagtg ttggatttat 4080
ggtagtatta aataaatatt tgttgaatgt aatagtaaat agtattttag ggagtaagaa 4140

ttagattaat aaaggtggta aaaggtttgg agaaaaaaat aatagtttaa tttggttaga 4200
gtatgaggga gagtagtagg agataagatg gaaaggtttt ttgggtaagg ttttgaagga 4260
agttggaagt tagaagtata taatgtgtat atcgtggtag gtagtgggga gttaatgaag 4320
gtttttgagt aggagagtaa tgtgttgaaa aataaatata ggttaaattt attagagttt 4380
ttttgatata tatatttgtt ttttatttaa gtttaagttt gttttttata tatttattat 4440
ttaatttatt ttcgggtttt tttagtagtt tgttttattt ttttatttgt tttttggtgg 4500
agttagggat gtatatatga gttgtttttt tttttagtta gaggatatgg gggttttagt 4560
ttttttgttt tttttttttt gtgtttggag ttgggaagta ggttagggtt agttgaggtt 4620
ggttggtaag tagttgggtg gtgttaggga gagtttgtat agtgttaggt ggtgttttgg 4680
gttttaagtt gagtttatgg tttcgataat tttttgtttg tgtatatatt tgttttttat 4740
tttattttta ttttagtttt ggtatggggg agagggtata gggttagata aatttgtgag 4800
attttggttt tatttttgta aaaggcgttt ttgtgagtta gtttgttttt ttttaggttt 4860
gttttttttt tatttagttt tcgtttttaa tgtacgtata gttcgtatat atcgtgtgtt 4920
gggatatttt atagttagtc gtatggtttt tttgtgtttt agtttttggt tttttttgtt 4980
gatttcggtt tttgttttag gttttattgt gtaattgttg ttgttattgt tgtttttggt 5040
gtttgtttat ttttagaggt tgtttcggat gtaggaggat ttttttttgg gaggaggttt 5100
ttttggggaa gatgatttat tgggcgagga ggatttgttt agtgaagagg atttatttag 5160
agaggaggat ttattcggag aggaggattt atttggagag gaggatttat ttggagagga 5220
ggatttattt gaagttaagt ttaaattaga agaagagggt tttttgaagt tagaggattt 5280
atttattgtt gaggtttttg gagattttta agaattttag aataatgttt atagggataa 5340
agaaggtaag tggttattaa tttttaaatt taggttttag gaggtttatg attttttttt 5400
tatattttag tttaggtttt gtttatttag ggaaggaggg gagattgtat tttttataga 5460
agttttttta gaggttttat attaatattt ttatttttat tttcggaggt agaaagggat 5520
agatgtggag agaaaataaa aagggtgtaa aaggagagag gtgagttgga tgagatggga 5580
gagaaggggg aggttggaga agagaaaggg atgagaattg tagatgagag aaaaaatgtg 5640
tagatagagg aaaaaaatag gtggagaagg agagttagag agtttgaggg gaagagaaaa 5700
ggaaagtttg ggaggtgaag tgggtattag agataagtaa gaagagttgg tagaagttat 5760
tttattttag gttataatga ggaaattgag atttaggaag aagggatata gtaggtagag 5820
aaacgtggtt ttttgatttt taagttagga atttggggaa aggggttgga gattatataa 5880
ggtagaggga tgagtgggga gaagaaagaa gggagaaagg aaagatggtg tatttattta 5940
tttgggattt aggattgaag tgtttattta tttttttttt tttttttttt gagataaatt 6000
tttatttttg ttgtttaggt tggagtgtaa tggcgcgatt tcggtttatt gtaattttta 6060
tttttcgggt ttaagtgatt tttttgtttt agtttttagt taagtagttg cgattatagg 6120
tatgcgttat tacgttcggt taatttttgt atttttagta gagacggggt ttcgttatgt 6180
tggttaggtt ggtttcgaat ttttgatttt aggtgattta attattttgg ttttttaaag 6240
tgttgggatt ataggcgtga gttatagcgt ttggtttgaa gtagttattt atttttatag 6300
attttaagat aatgattgta agttggtagg attgttgttt ggtttattta gttgcggtgt 6360
tgagtttggg tgcggttttt tgtgttttgt atttggttcg tttaaggtat ttgttattcg 6420
taatgttttt gtaaggtatt tgcgtttgtg atatcgtttt ggtcgttagg aagggattgg 6480
ggttttaagt ttgagcggtt tatttttttt atttatatag g 6521

<210> 198
<211> 6521
<212> DNA
<213> Artificial Sequence

<220>
<223> chemically treated genomic DNA (Homo sapiens)

<400> 198

tttgtataaa tgaaaaggat gaatcgttta agtttagagt tttaattttt ttttggcgat 60
taaaacgatg ttataaacgt agatgtttta taggagtatt acgggtaata aatgttttaa 120
gcgggttagg tgtaaagtat aggagatcgt atttaaattt aatatcgtag ttgggtgggt 180
taaatagtaa ttttattagt ttgtaattat tgttttaggg tttgtaaaag tgagtggttg 240
ttttaggtta ggcgttgtgg tttacgttta taattttagt attttgggag gttagggtgg 300
ttggattatt tgagattagg agttcgagat tagtttgatt aatatggcga aatttcgttt 360
ttattaaaaa tataaaaatt agtcgggcgt ggtggcgtat gtttgtaatc gtagttattt 420
ggttagaggt tgaggtagga gaattatttg aattcgggag gtggaggttg tagtgagtcg 480
agatcgcgtt attgtatttt agtttgggta ataaaagtga aagtttgttt taaaaaaaaa 540
aaaaaaaaaa gtgagtgggt attttagttt tgagttttaa atgagtgagt atattatttt 600
tttttttttt tttttttttt ttttttattt atttttttgt tttgtatggt ttttaatttt 660
tttttttaaa tttttggttt gggagttaag aagttacgtt tttttatttg ttgtgttttt 720

tttttttagg ttttaatttt tttattgtag tttaagatga gatgattttt attagttttt 780
tttgtttgtt tttggtattt attttatttt ttaagttttt tttttttttt ttttttaaat 840
tttttgattt ttttttttta tttatttttt ttttttgttt gtatattttt ttttttattt 900
gtagttttta tttttttttt ttttttagtt tttttttttt tttttatttt atttagttta 960
tttttttttt tttgtatttt ttttattttt tttttatatt tgtttttttt tattttcgag 1020
agtggggatg gggatattgg tatgggattt ttggaagggt ttttgtgggg agtatagttt 1080
tttttttttt tttgagtgaa tagagtttag gttggggtat gggaggggag ttatgaattt 1140
tttggaattt ggatttggag attgatgatt atttattttt tttgtttttg tgggtattat 1200
tttggggttt ttgaggattt ttaggagttt taatagtagg tagatttttt aattttaggg 1260
agtttttttt ttttgattta ggtttaattt taggtagatt ttttttttta ggtagatttt 1320
tttttttagg tagatttttt ttttcgggtg gatttttttt tttgggtgaa ttttttttat 1380
tgggtagatt tttttcgttt agtgggttat tttttttaga agagtttttt tttaaggggg 1440
aatttttttg tattcggggt aatttttggg gatggatagg tattagaagt agtagtgata 1500
gtagtagttg tatagtgagg tttggagtag gggtcgggat taatagaggg agttaggggt 1560
tggggtatag gggagttatg cggttgattg tggggtgttt tagtatacgg tgtgtacggg 1620
ttgtacgtgt attggaaacg agagttgggt gggggaggag taagtttgga ggggagtagg 1680
ttgatttata gagcgttttt ttgtagagat ggagttaaag ttttataggt ttgtttggtt 1740
ttgtgttttt ttttttatat taaagttagg atgggggtgg agtgaggggt aggtgtgtgt 1800
ataggtagaa ggttatcggg gttatggatt tagtttggaa tttaaggtat tatttggtat 1860
tatgtaggtt ttttttggta ttatttagtt gtttgttagt tagttttagt taattttggt 1920
ttgtttttta gttttaggta tagaagggga aaggtagggg agttggggtt tttatgtttt 1980
ttggttgaga gggaaagtag tttatgtata tatttttgat tttattagga agtaggtaaa 2040
gaggtagggt aggttgttag gggagttcga gggtgagtta agtaatgggt atgtgggaga 2100
taaatttgag tttgaatgaa aagtaagtgt atgtgttaga ggggttttga taggtttaat 2160
ttatatttat tttttaatat attatttttt tgtttaaaag tttttattgg ttttttattg 2220
tttgttacga tatgtatatt gtgtattttt gatttttaat tttttttaaa attttgttta 2280
agagattttt ttattttgtt ttttattatt tttttttata ttttagttaa attaaattat 2340
tatttttttt tttaaatttt ttattatttt tgttaattta gtttttgttt tttgaaatgt 2400
tatttattat tgtatttaat aaatatttat ttagtattat tataggttta gtattgggtt 2460
atggattagg gatatggaaa tgaataagat acggtttttt attttgggag gttgaggtgg 2520
gtggattgtt tgagtttagg agttcgagat tagttcgggt aatatggtta aattttgttt 2580
ttattagaaa tataaaaaaa ttagttaggt gtaatggtat gtgtttgtag ttttagttat 2640
ttaggaggtt gaaatgggat gatggtttga gtcgaggcgg ttgaggttgt agtgagttga 2700
gattgtatta ttgtattttg gtttgggtga tagagtaaga ttttgtttta aaaaagaaaa 2760
ataaaaataa aaataaaaaa cgttatttgt tttgagtagt ttataattta gtgagtgagg 2820
gtaaattttg ttaatattat tatattgagg gaagagagat gaattttgtt ttagaagata 2880
atatagaagt aggtaaattt gattatgttt ttgaagaaat attagtttta ttggtaaaaa 2940
taatagggag aatgatattt tgggtaaagg gaaggtataa aatattatga gtaggagagt 3000
gatagaattt agtttttata ttaggttacg ttgagggttt gagggttagt atggagtata 3060
gattgtaggg gaagattggg tgtaaggaga tggggtagaa gagtgtgagg atattaagaa 3120
tgggtttagg ttttttagtt tttttttttt tttttttttt tttttttttt tttttttttt 3180
tttttttttt tgagatagag tcgtattttg ttatttaggt tggagtgaag tggcgcgatt 3240
tcggtttatt gtaattttta ttttttaggt ttaagcgatt tttttgtttt agttttttgg 3300
gtagttggga ttataggtat ttgttattac gtttagttaa tttttgtatt tttggtagag 3360
attggggttt tgttatgttg gttaggtttg ttttgaattt ttgattttag gtgatttgtt 3420
cgttttggtt ttttaaagtg ttgggattat aggttggttt ttttttttaa ttaattattt 3480
ttaaaattta ttttagagat gggattttat tgtgttgttt aggttggagg gcggtggtta 3540
tttataggtg cgattttgtt attgattagt attgagtttt gatttgtttt aatttttatt 3600
tgggttagtt taattatttt taggtaatta ggttgatgta gaatttagtg tagatatttg 3660
attggtatat tatattatag tttagaaatt ttgggattaa gtgatttttt tgttttagtt 3720
ttttaagtag ttgggattat aggtatacgt tattatattt ggttagtttg tttttttaat 3780
aatttattaa gttttcgtta gttttttagg tgttgtttta ggttttatag atgtagtatt 3840
gaatagagaa tagttttatt gttttgtagt ttatatttta gtaaggtttt attggtttaa 3900
ataaattaaa agagaataag gttattttag gtttatattt agggttaata tttagtaggt 3960
atttattaat atggttgtgt aggttaatta aatatattaa gatattttta attttgttgg 4020
tatatagtta tggtttttag ttttagggta tttttgttat tttagttttt ttttagggat 4080
tttttagatt ttttgatttt ttatttaaag aggtattttt tggtataata gagtttttag 4140
gagtttattt gagtgtttta gttattttta attttgatag gttagtgttt gtgtatttgt 4200
tgttgtgtta taaatagttt tattggttag gtgtagtggt ttatgttttt aatattagta 4260
ttttgggagg tcgaggtgtg tagattattt gaggttagga gttcgagatt agtttggtta 4320
atatggtgaa atttcgtttt tattaaaaat ataaaaatta gttgggtatg gtggtaggta 4380
tttgtaattt tagttattta ggaggttgag gtaggagaat tatttgaatt tgggaggtag 4440
aggttgtagt gagtcgagat cgcgttattg tattttagtt tgatcgatag agtgagattt 4500

tgttttaaaa ataaataaat agataaaaat cgtcgttaat atagtgaaat ttcgttttta 4560
ttgaaaaaaa aaaaaaaaaa aaattagtcg ggcgtggtgg cggtcgtttg tagttttagt 4620
tatttaggag gttgaggtag gagaatggcg tgaatttagg aggtaaagtt tgtagtaagt 4680
taagatcgcg ttattgtatt ttagtttggg cgataaagcg agattgtttt aaaaaaaaaa 4740
aaaaatttta ttgttttttt tgtttaattt tttttgtttt ttgaaattta gatgaaaggt 4800
tatttttttt aggaaatttt ttaattaatt ttttattatt agtttggaaa agatttttgg 4860
tatttttgag ttaatttagt tttttgtttg tttttaataa taattttgtt tatcgtttaa 4920
tttatataag ggtgataatt tattttattt gttttaacgg tttttagttt tataagggta 4980
gggttcgtat ttgattgagt tagtttttta tttaatagtt atattgagtt tttggtttat 5040
ttttgtaata ttagtatttt ggaggttaag gtagtaggag gattgtttga ggttaggagt 5100
ttaaaattag tttgagtaat atagtgagat tttttatttt tattaaaaaa taaataaaaa 5160
tagtagatag gtttgatgat ataagcgtgt agttttagtt atttgggaga ttgaggtggg 5220
aggattattt gagtttagga gttggaggtt gtagggagtt atgattatgt tattgtattt 5280
tagtttgggc gatagacgga gagattttgt ttttatagta gatgtataaa tattattgtt 5340
ttataattgt ttatagtatt tagtatggta atatgttgta agtattgggg tttaggagta 5400
ttaggttata ttatgtagtt tagatgggta ataggttata ttatttaggt ttgtgaaaat 5460
atattttatg atgtttgtat aatgataaaa ttgtttagtg atttattttt tagaatatat 5520
ttttgttgtt aagtaatgta tggttgtttt tatgttgata tgattatatt tataaagttt 5580
aaggaaagaa aatatttatt gagtttattt tttggttttt attatttatt ttataattgt 5640
tattgagaat aattttgtta tataaagaat gtttaaattt gttttaattt tatttattta 5700
tttatttgag atggagtttt tttttgtcgt ttagattaga tcgtagtggt ataatttcgg 5760
tttatttgtt taggttggag agaagtggcg tgtttggtta taaagtattt ttttaaatga 5820
tttatttgaa aaataaaatt ttgttttagg tatggtggtt tatgtttgta attttagtac 5880
gttgggaggg tgaggtaaga ggatcgtttg agtttaggag tttaagaata gtttgggtaa 5940
tatggtaaaa tttcgttttt attaaaaata taaaaaatta gttaggtgtg atggtgtacg 6000
tttgtggttt tagttattcg agaggttgag gtgggaggat tgtttgagtt taggaggtag 6060
aggttttagt gagttaagat taagtttttg tattttagtt tgggtaatag agtgagattt 6120
tgttttttat ttttaaaaaa agaaagaaaa taagaaaaat ttgggtttgg gtatggtggt 6180
ttacgtttgt aattttagta ttttgggatg ttgaggtggg tggattattt gaggttagga 6240
gtttgagagt agtttggtta atatggtgaa attttttcgt ttttattaaa attataaaaa 6300
ttagttaggt gtggtggtgg tatacgtttg taattttagt tattcgggag gttgaggtag 6360
gggaattatt tgaatttgcg aggcgaaggt tgtagttagt cgagattgta ttattgtatt 6420
ttagtttggg tgatagagta agattttgtt ttaaaaaaaa aaaaaaaatt cgtttttttg 6480
aattgtattt gattggattt tttatttatg tataattttg t 6521

<210> 199
<211> 6565
<212> DNA
<213> Artificial Sequence

<220>
<223> chemically treated genomic DNA (Homo sapiens)

<400> 199

cgatttttaa attttataag ggttatcgta gttttttgaa gtagaaaatg tagtttattt 60
tttgttagtt tatgggttat attttgattt aagtattttt acggtgggta tttgcgttta 120
ttttgtagtt ttcgttagag attatgttag aagatggcgg tatataggtt gagtaagagg 180
tggtgaggtt gatcggggtt tatcgattat agaataggtt tttttagagg gatatgaagt 240
atcgttaggt tttttgagtt ttaagttgtg gttcgtagtg ttttggcgag tagttttgtt 300
aatttaattg ttgaggttta gggttaagta tagtggggta tttaatttta gtttgggttt 360
tagttattgt gtgtttagat gcgttaaagt tattttcgta gtttattttg tattaattgg 420
agttttttat aatttaggtg aattttagtt ttattgaggg gaattgattt aaaatatttt 480
ttacgtcggt ttttattgat ttgggttaat cgtgtgatcg cggtggttgg tacgaatttg 540
acgaatttta gggttagtat agtttagtta aattttcgtt tattgttaaa ggtttattat 600
tgttgttttt cgtgggggtg tggttaggtt aagcgttttg agttgcgttg ttgcgtgttt 660
gatatttgtt ttttttgatc gtggtgattt agagggtgat ttatcggggt agggatgttt 720
gtatgtgtaa ttttattaag agttaatagg ttacgattaa atttatttgt ttatggggtg 780
gtatgagttt gtcgaaatat ttttagtgta ttgttttgag gaagtaagtt atataaatcg 840
tatggggtgt ttttggggtt tggttggttc ggggtatggg gttagtagcg gtgtatatgt 900
tgagtaaggt gggtagggag ttgtattgat ggggttagta gaatgtgagt tgagggagga 960
tgatgtgtta gttgaggggt gattgttaaa aatgtatttt tttttttgtt attatgtgaa 1020
gaaggatgcg tttgtttttt tttttattat gattttaaat tttttgtggt ttttttagtt 1080

atgaggaatt gtgagttaat taaatttttt ttttgtataa attgtttagt tttgggtagt 1140
tttttataag aatatgagaa tggattaata taggtttaat tttggttatt ttttaatttt 1200
ttaagttatt ggagttgatt ttttgggtag ttttaatatg attatttttt ttatttttta 1260
gttattaggg tatattaggt tttatgaatt attgatttta atttttgggt tggaggatag 1320
gaaaattttt agtagaggtt aattggagat attcgttttt ttttaggatt tttagaatta 1380
ttttaatatt ttgaaatgtt ttatttttag atattgggta atgaatgtta agtataaaat 1440
attattgggt attgtggtta attaggaata atagagtaga agattatgat ttattttagt 1500
ttattttaaa atttatattt tattgtattt agggttgggt ttagaaaggg gattattatt 1560
taattttagt aagttttaaa aaatagttgt tttgatttta tgtgaaatta taggttattg 1620
attatattgt ataatttata tttgggttta ttgaatgatg gtttaagata atttgtaaat 1680
ttgatattat agtgagttat ttaatgttta atgtaatttt ttaaggtgat gaaattaatt 1740
atttaggtag tagaataagt tattgttatg tagtataaat aattgtttat taatttttta 1800
taattatttt tgtgatatta agtagtttta ttaatagttt tttttatata attttatttt 1860
attatatatt gatttttatt atgagatatg atttttttgt ggtgaattta tgttaaggta 1920
tttatttgag ttttttgttg agaattaaat taattttatt ttttaaaggt cgattttttt 1980
taggaatgga gaatttaggt ttttttattt ataattatta gattttgtta tttataaaat 2040
tataaagaaa gtttaagtat attttgggaa tatatatata tttttatgta ttatatataa 2100
tatgtttttg aataatatcg ttttttttta acgttgatga gaaaaataat taatttttgg 2160
ttagggttat cgtttgtgtg gagtttgtat gtttttttta tgtttgtatg gggttttttt 2220
aggtatttta gttttttttt atattttaaa gatatgtata ttattttttt taaatgtatg 2280
tatagtttat atttatttta gtgtttttta ttagaagtgt ttttggtttt tatttagaag 2340
tttggtgatg tttttgtgat tagtaatatg ttttaggaat ttaatttttg tgtatattta 2400
gtagtttatg gtaaaattgg ttttgtttta tattgtttta tttaaagtta tagtttttaa 2460
gaatttatag atgatattga ggattattta tgtgtataga tatgatggat attttgatat 2520
ttagtggatt ttttttaaaa aattgaattt aaaagtttta ttaaaattta tatttattat 2580
ttattattta aatttaatta tttttttttt tggtattatt taatttatat ttaatattta 2640
gttttaatta tttgtaattg tgtatgatat agtaaagatt ttggggaggt taagatgggt 2700
agattacgag gttaggagat cgagattatt ttggttaata cggtgaaatt ttatttttat 2760
taaaaatata aaaaattagt taggtgtggt ggtaggcgtt tgtagtttta gttatttagg 2820
aggttgaggt agaagaatgg tgtgaattcg ggaggcggag tttgtagtga gcgtagatcg 2880
tattattgta ttttagtttg ggcgatagag taagattttt ttttaaaaaa aaaaaaaaaa 2940
aaagattttg agttaaagtg tttaagttga atgtattttg ttatttataa gataaatcgt 3000
gttaattttt tgtggtttat tttatttata aaatagagat aataatagtt tttgttttta 3060
gggttgttgt gggaattaaa gatttagaat aatgtttagt ttttaattag tgttgttata 3120
attgtttgat ataattgtat tatatttgtg tattttgtag attgatatta aattatattt 3180
ttaaaaatag gtgtttaatg ttttatttaa ttattttaaa atatgtttaa ttatgttttt 3240
attttatttt tataatattt ttataaaaat tttttatata tagcgtttat ttttggttta 3300
gttttttagg aaaataattt tgagagttag ttatttgaat taaagaaata ttaatattat 3360
tagattatat tgggattttt gagattggtt tttattaatt ttttagttac gggtttttgt 3420
tattattttt attagtgatt taagtgttaa atttaaatgt tttgtatttg ttttattaaa 3480
gagatgtagt atttgttttt tttttattgt ttttattttt tttgttatgt ttttttttat 3540
aattataaat atttaggttt tttaggtttt aaacggggta ttttttaatt tttatatttt 3600
tttttttggt tatttttttt tttttaatag tttaatttat ttagattttt acgtttgaaa 3660
ttattttaga tgttttagag gcgttttatt attataatgg tatattattt ttttattttt 3720
tatatgttac gttagttttt aaattgaaaa tttgagcgtt tatttttggt gtattatttt 3780
taaattttag atttttaaaa tttagggtta tgtaatttta aaaaattttt attttttttt 3840
ttttattgtt tttgtttagg ttttattttt tttagtagtt gttttaaagg tttattttgt 3900
ttttttttcg gagtgttaat ttttatcgaa gtttttattt agttgttaat tttgttttat 3960
gtttgataat ttgttcgtgc gttgatatat ataaaatttt taagataaaa attttttaat 4020
aatggtaaat gaattttggg aattgtatat agattatata gatttataat aagagaaaag 4080
gttttagatt aatacggaaa attttttatt taattaatat ttgtattggt aaattttatt 4140
aagtaagatt ttatttaatt ttatattatt ttttattgaa gaggttgtgg ttattttttg 4200
gaaatatttg aatttatttt tataagttag agaaatagcg ttattcgaaa tattattttt 4260
tgggttcgag ttttaaggta tttgataaac ggagcgttgt gggtaggggt gaggtgtttt 4320
ttttagggtt gggattttgt tttgggcgag ggcgtcgtag ggtaaagatt ttatcgggta 4380
gtagaattcg ggtagaaatt agtaattggg tttttcgcgt agtagaaaag gggaatttag 4440
tcggggttta ttttttttgt tagcgtagat cgtaagtttg gttttatttt ttcgtcggga 4500
gtcggtttgg cgcgtttcgt ttaggtattt cgatcgtggg tagtttgcgt tcgtttgggt 4560
tttatcgttt cggttcggta gatatttgag cggtggttag ggtaggtttt cgtttttgtc 4620
gatgtttatg ttttgggata tagcgacgat gtagtttagc gaattaatta tgatagtagc 4680
gggaggattt tcgagttcgt tcgttatagt agaacgcgcg gttaagtttg gcgcgaaatt 4740
gtggtcgttt cgttttttcg tttttatttg tgtaggcgag gtttcgtttt ttcgtttcgg 4800
cgtacgtagg gtcgcggcgt gttcgcgttc gtagacgttt gggaattgcg gtcgcgggtt 4860

cgcgtttttc gtcgggtttt gtcgtcgggt tgttattttt gttttgttat gtttcgtcgg 4920
aagtttgcgt cgggcggttt cgttgttttt agtttagttt ttgcgaggta agcggttttg 4980
agtcgatttt tttagtttac gggaagtttg aaatttattt ttttttttat aggtgtagtc 5040
gattaggtgg attttggcgt tgtagcggtc gtagcgtttt tagcgttcgt tttttcgttt 5100
tagttgtcgt cgtacgtagt aggttttgtt tgggattggg tagggttatc ggggttgggg 5160
gggcggggtt tgtgggtaag gcgggcggag gcgtggattt ttcgttcgat gatagggttg 5220
gaggaggaag ggggcggttg aagaagggga aggtgggaag agtttagtcg gggttataaa 5280
ttgggtgaag cgttgaggtt ttagtatttt cgtttgagga gataggtaaa ggttatgtag 5340
gtttttaatg gtaggtttga gataggaatt taggtttttt gatttttatt ttgatgaggg 5400
ggtttgtggg taaggggtgc gttttttaat ttttattttt tttttttttt tttggtggtc 5460
gaagagtttt attgattttt taaaaaagtg ttggattggg tgattagaag aaagttgttt 5520
ttaggttttt agtttttaag attgtaatag tatggtttaa tatttgaaat tattacggtt 5580
aaaagtagtt atattgtgta gttttttagt gaatattttt tgtagtttag attataaagt 5640
ttgaaatata gtttgggtgg ataaagtttt ttaggatggt atagatgtga ttaattatgg 5700
agtttttttt gttgtcgttt tttaacgtaa taagtacgtt tgtttgtttt taaattagag 5760
atattgttat aggaaaagtt taagggtgat tttttatttg ggttatttgt atagtatttt 5820
ttagattgag gttgtggaag ggtattgaag attggtgttt agttatttat cgtgttttaa 5880
tgtaggtggt agtttgggtg ttttaaaatt ttgttttttt cgtatatatt ttttttttaa 5940
aatgattatt ttttttttgt tttttttgag gacgagttac gtttttatga atttattaaa 6000
atttgtattt tattatttaa agggtagttg gtgaaggatt tatgtatgtt tttggtaagt 6060
tttttgttaa gaaaaaattt ataattttgt tatattttta gtgatgaata taaatttttt 6120
aaataggaaa tatttaaatt gtaatgaata tgttaaaaat gatattgttt ttttattatt 6180
tatttttttt tttttgtgtt tttgttatat aattgtttat tttttagagg tattttattt 6240
agtatatgta agtagggtga tttaagtttt ataattagag aaagttgaag gtttattgaa 6300
ttaagatcgg tagtagtttt ggttttaatt aattttaaat acggtttgag ttgttgaggt 6360
ttttgattat tagattttat tttgtttttt atggtgttaa aaatagttta tttatagggt 6420
tttttagagt taaaagtaga ggattttttg aagagtggat ggtaaggaat tttgttattt 6480
agttgtaaag tttaaattgg tttatatacg ttaggttttt ttagagtaga gataatatat 6540
tattttttaa tttttcgat atagg 6565

<210> 200
<211> 6565
<212> DNA
<213> Artificial Sequence

<220>
<223> chemically treated genomic DNA (Homo sapiens)

<400> 200

tttatatcgg gaaggttaga aaatgatgtg ttatttttat tttgagaagg tttgacgtat 60
gtgaattaat ttaaatttta taattgaatg ataaggtttt ttgttattta ttttttaaag 120
aattttttat ttttagtttt ggaaaatttt atgggtgaat tgtttttaat attataaaag 180
ataaggtaga gtttgatagt taggagtttt agtaatttaa gtcgtgttta gaattaattg 240
aaattaaggt tgttatcggt tttgatttaa tgagttttta gtttttttta attataaaat 300
ttgaattatt ttatttatat atgttaaata aaatattttt aagaaataaa tagttatgta 360
gtaaaggtat agaggggaaa agataggtaa taaaagaata gtattatttt tgatatgttt 420
attgtaattt aagtgttttt tgtttaaaag gtttatgttt attattaaag atgtgataag 480
attatgaatt tttttttggt agaaaatttg ttaaaagtat atataagttt tttattagtt 540
attttttaaa tggtgggtgt aaaattttgg tgaatttatg aaaacgtagt tcgtttttaa 600
aaaaaataga agaggagtaa ttattttaag ggagaaatat atacgaaagg aataagattt 660
tgaagtattt aagttgttat ttatattaaa atacggtagg tggttaaata ttagttttta 720
atgttttttt atagttttag tttgaaaaat attgtgtagg tgatttaagt gaggggttat 780
ttttgggttt tttttgtggt agtatttttg gtttaaaaat aaataaacgt atttattgcg 840
ttgaaggacg gtaataggaa ggattttatg attagttata tttatattat tttaagaaat 900
tttatttatt taaattgtat tttagatttt ataatttaaa ttataaaaag tgtttattgg 960
ggaattgtat aatatgattg tttttaatcg tagtgatttt aaatattgag ttatgttgtt 1020
gtagttttaa aaattggaga tttaagggta gttttttttt agttatttaa tttagtattt 1080
ttttaaaaaa ttagtaaaat ttttcgatta ttaaggaaaa aaaaaaagga tggaggttaa 1140
aagacgtatt ttttgtttat aagttttttt attagaatgg gagttaggag atttgagttt 1200
ttgttttagg tttgttatta aaaatttgta taatttttgt ttattttttt aaacggaagt 1260
attaaaattt tagcgtttta tttaatttgt agtttcggtt gggttttttt tatttttttt 1320
ttttttagtt cgtttttttt ttttttagtt ttattatcgg gcggagggtt tacgttttcg 1380

ttcgttttat ttataagttt cgttttttta gtttcgatgg ttttgtttag ttttagatag 1440
aatttattac gtgcggcggt agttggggcg ggaaggcggg cgttgggggc gttgcggtcg 1500
ttgtagcgtt agggtttatt tggtcggttg tatttgtgga ggaggaggtg gattttaggt 1560
ttttcgtaga ttggaagaat cggtttaaaa tcgtttgttt cgtaggggtt gagttggagg 1620
tagcgaggtc gttcgacgta ggttttcggc gagatatggt agggtaagga tggtagttcg 1680
gcggtagggt tcggcgagga gcgcgaattc gcggtcgtag tttttaggcg tttgcgggcg 1740
cgagtacgtc gcgattttgc gtgcgtcggg gcgggggggc ggggtttcgt ttgtataaat 1800
agggacgagg gggcggggcg gttataattt cgcgttaaat ttgatcgcgc gttttgttgt 1860
aacgagcggg ttcggaggtt ttttcgttgt tgttatggtt ggttcgttaa attgtatcgt 1920
cgttgtgttt tagaatatgg gtatcggtaa gaacggggat ttgttttggt tatcgtttag 1980
gtatttgtcg ggtcggggcg atgggattta aacgggcgta ggttgtttac ggtcggggta 2040
tttgggcggg acgcgttagg tcgattttcg gcgagaggat ggggttagat ttgcggtttg 2100
cgttggtagg aagggtgggt ttcgattgga tttttttttt ttgttgcgcg ggaggtttag 2160
ttgttgattt ttgttcggat tttgttgttc ggtgaggttt ttgttttgcg gcgttttcgt 2220
ttagggtaaa gttttagttt tggagaaaat attttatttt tatttatagc gtttcgtttg 2280
ttaggtgttt tagagttcga gtttaaggga taatgtttcg agtaacgttg tttttttaat 2340
ttgtaggaat gaatttagat atttttagag aatgattata atttttttag tagaaggtaa 2400
tgtgggatta agtagggttt tgtttgatga agtttattag tgtaaatgtt agttaaatgg 2460
aaagtttttc gtgttaattt gggatttttt tttttattat ggatttgtat gatttgtatg 2520
tagtttttaa ggtttattta ttattattaa aaaatttttg ttttagaaat tttatgtatg 2580
ttaacgtacg agtaaattat taggtatggg gtagaattgg taattgggtg gaggtttcgg 2640
tggaggttag tatttcgaaa ggaaaataga gtaggttttt ggaatagttg ttggaagaga 2700
taaggtttga ataagggtag tggagaagag agggtaaaaa ttttttaagg ttatatgatt 2760
ttggattttg gagatttgga atttaaaggt gatgtattag ggatgaacgt ttagattttt 2820
agtttgaaag ttggcgtgat atgtaaagga gtggagaata atgtattatt gtaatgatga 2880
ggcgttttta ggatatttag gataatttta ggcgtgggga tttaggtgaa ttgaattgtt 2940
ggagggaagg gaataattaa ggaaaagaat gtgggggttg agagatgttt cgtttaaaat 3000
ttaagagatt tggatattta tggttgtaag aggaggtatg gtagaggaaa tggaaatagt 3060
aagaaaggag tagatgttgt atttttttaa tgggatagat ataaggtatt tgggtttgat 3120
atttaggtta ttggtagaga tgatgataag agttcgtagt taaagaattg ataaaagtta 3180
gttttaaaaa ttttaatata gtttggtaat gttaatgttt ttttggttta gatagttgat 3240
ttttaaagtt atttttttaa gaaattgaat taaaagtgga cgttatatgt aaaaagtttt 3300
tatagaagtg ttataagagt aggatagaga tataattaaa tatgttttaa ggtaattgag 3360
tggaatatta agtatttatt ttttaaagta tgatttaata ttaatttata aagtatataa 3420
atataatata attgtattag atagttgtga tagtattgat tagaggttga atattatttt 3480
aagtttttaa tttttataat aattttagaa gtaggaatta ttgttatttt tattttatag 3540
ataaagtaaa ttataagggg ttaatacgat ttgttttgtg gatgataaaa tgtatttaat 3600
ttgagtattt tgatttagaa tttttttttt tttttttttt taagagggag ttttgttttg 3660
tcgtttaggt tggagtgtag tggtgcgatt tacgtttatt gtaagtttcg tttttcgggt 3720
ttatattatt tttttgtttt agtttttttga gtagttggga ttatagacgt ttgttattat 3780
atttggttaa ttttttgtat ttttagtaga gatggggttt tatcgtgtta gttaggatgg 3840
tttcgatttt ttgatttcgt gatttgttta ttttggtttt tttagaattt ttattatgtt 3900
atatataatt gtaaatgatt aaaattaaat attggatata aattggataa tgttaaaggg 3960
aaaaatagtt agatttaggt aatgagtgat aagtatgaat tttggtaaga tttttaagtt 4020
tagtttttta aaggagattt attaaatgtt aggatgttta ttatatttat gtatatagat 4080
agtttttaat gttatttgtg agtttttgga aattgtgatt ttaagtgaaa taatgtagag 4140
taaaattaat tttattatag gttattggat gtatataaga gttaagtttt tagggtatat 4200
tgttggttat aaaaatatta ttaaattttt aaatgaaggt taaaaatatt tttaatagaa 4260
aatattgaag taaatatgag ttatatatat atttaagaag aataatgtgt atgtttttgg 4320
gatgtggaag gaaattggaa tatttggaga aattttatgt agatatggga aaagtatgta 4380
gattttatat agacggtggt tttggttagg aattgattat tttttttatt aacgttagaa 4440
gaaaacgatg ttatttaagg atatgttgta tataatatat gaaaatatat gtatattttt 4500
aaaatatatt taggtttttt ttgtgatttt ataggtaata gaatttggtg attatgggta 4560
agaagatttg ggttttttat ttttgagaag aatcgatttt taaagggtag aattaattta 4620
gtttttagta gagaatttaa gtaagtattt taatataaat ttattataag aaaattatgt 4680
tttatagtgg agattagtat atgataaaat gaaattgtat aaaaggagtt attgatgaag 4740
ttgtttaata ttataaaggt gattatagaa aattaataag tagttattta tattatatga 4800
taataattta ttttattatt tgagtaattg attttattat tttgaaaagt tatattaaat 4860
attaaatagt ttattgtggt attagatttg taagttattt tgaattatta tttagtaggt 4920
ttaaatataa attatatagt gtaattaata atttgtagtt ttatataaag ttaaaataat 4980
tattttttgg gatttgttaa aattaagtga tgattttttt tttggattta attttaaatg 5040
taataaaatg tgaattttag gatgggttga aatgggttat aattttttat tttgttattt 5100
ttagttaatt ataatattta ataatatttt gtatttaata tttattattt agtgtttgaa 5160

ggtaaaatat tttaaagtat tgaaatggtt ttagagattt tggagagaag cgggtatttt 5220
tagttagttt ttgttaagaa tttttttgtt ttttaattta ggggttggag ttagtagttt 5280
atgaagtttg atatgttttg gtgattggag agtgggaggg gtaattatat taagattgtt 5340
taggaagtta attttaatgg tttgggaggt tgaggggtgg ttagaattgg atttgtgtta 5400
atttattttt atgtttttat aaagaattgt ttaagattgg gtaatttgta taggaaagag 5460
gtttaattga tttatagttt tttatggttg gggaggttat aggaaattta aaattatggt 5520
ggaaggggaa gtaaacgtat ttttttttat atggtggtag gaaggaaagt gtatttttaa 5580
tagttatttt ttaattaata tattattttt ttttaattta tattttatta attttattaa 5640
tgtaattttt tgtttatttt atttaatata tatatcgttg ttagttttat atttcgaatt 5700
aattaaattt taaagatatt ttatacggtt tatgtagttt atttttttaa agtaatatat 5760
tgaaaatgtt tcgataggtt tatattattt tataaataaa taggtttggt cgtagtttat 5820
tagtttttag taagattata tatgtaagta tttttgtttc ggtgagttat tttttaaatt 5880
attacgatta aaaggaataa gtattaagta cgtagtaacg tagtttaaaa cgtttagttt 5940
agttatattt ttacgggaga tagtagtgat aaatttttag taataaacga aagtttaatt 6000
aagttatatt aattttaggg ttcgttaagt tcgtgttagt tatcgcggtt atacgattaa 6060
tttaagttaa tagaaatcgg cgtaaagagt gttttagatt aatttttttt aataaagtta 6120
aaatttattt gagttgtaaa aaattttagt tgatataaaa taaattacga aagtggtttt 6180
aacgtatttg aatatataat agttaagatt taaattggga ttagatattt tattatgttt 6240
agttttaaat tttaatagtt aaattaataa aattgttcgt tagaatatta cgagttatag 6300
tttaaaattt aaaggatttg gcggtgtttt atattttttt agaggagttt gttttgtaat 6360
cgataaattt cgattaattt tattattttt tgtttagttt atatatcgtt attttttagt 6420
ataatttttg acgaaggttg taaagtaagc gtaagtattt atcgtaaaga tgtttaggtt 6480
aaggtgtagt ttatgagttg gtaagaaatg ggttatattt tttattttag aaaattacga 6540
taatttttat gaaatttaag ggtcg 6565

<210> 201
<211> 2891
<212> DNA
<213> Artificial Sequence

<220>
<223> chemically treated genomic DNA (Homo sapiens)

<220>
<221> unsure
<222> (223, 366)
<223> unknown base

<400> 201

tttttttttt tcgaattgtt tttttttttg gaggtggtgg agggagagaa aagtttattt 60
aaaatgtttt tgggtgaggg attaaggatg agaagaatgt tttttgtttt ttatgtcgtg 120
gaataatata aaataaaaaa tttcgaggga atatatatta tatattaaat atagattatt 180
ttagggagta aataaattat gtgtggggtt gggtaattag ttnaagtcga agcgtaaata 240
aaatgtgaat atacgtttgc gggttatata tagtgtattt ttattagtat ttagaaaaaa 300
ttgtgagtta gtgaattagg aaattaatgt ttggaaggta gttaaatttt aattagttta 360
agattntttt ttttttttaa aaaaaggtac ggaagtaata tttttttttt tttttttgat 420
tagaatcgat gtattttttg tgtatgatcg tatttttaat aataaaaggg gaaagaggat 480
ttggaaagga attaaacgtt cggtttgttc ggggaggaaa gagttaacgg ttttttttat 540
aagggttttt gttgattttt tcggttcggt ttataagttt tttatttgtt ttttttagga 600
agttcggttt cgcggttcgg gtattttttg ttttttttat attttttcgt ttagtatttt 660
tgattttttt taaattcggt agttcgagat tgttgtaaat cggcgttata gggcgtaaag 720
gggatttgtt ttttttgaaa tttggttgag aaattgggaa tttcgtgtgg gaggcgtggg 780
ggtgggacgg tggggtatag attggtagag agtaggtaat ttttttttcg ttttagttta 840
gttttggaat aggtagatat attttagggt taaatagacg tttttcgtac ggggttttac 900
ggaagtttga gtaggcgggg taggagggtc ggtatttgtt gttttggtag taaattgggg 960
gatttagttt gggtggaagg tatttaattt agatagttgt gtatatataa tgtataatat 1020
atgatttttt ttaataaatg taatgggagt ttatttataa cgcgtttttt aagtatacgt 1080
ggtaatgcgt tgttgggtta ttttaattat tttaggtatc gttttttttt ttatgttttt 1140
attatttttt tttatttata ttaatatttt acgttttgaa cgcgcgttta ttaatatttt 1200
tttttttttt attttttttg ggatttttga ttaaagcgcg gttttttttt tagttttagc 1260
gaggcgtttt gtagtttggt acgcgcgtgg cgtggcggtg ggcgcgtagt gcgttttcgg 1320
tgtggagggt agttgtttcg tttgcgatga tttatattta taggataagg atgcggtttg 1380

ttaaatagta ttgttacgga ggagtagtag agaaagggag agggtttgag agggagtaaa 1440
agaaaatggt aggcgcgcgt agttaattta tgcggttttt ttattttgtt tatattttag 1500
agttagagtg ttcggttgtt cggttgagtt ttttttttat ttttttattt ttttttattt 1560
tttttataag cgtttttttc gggtttttaa agtagagggc gtgggggaaa agaaaaaaga 1620
ttttttttcg ttaattttcg tttatcggtt ttttataatg cgagggtttg gacggttgag 1680
gattttcgag ttgtgttgtt cgcggtcgtt atcgtcgggt ttcggtcgtt tttggttttt 1740
tttttgtttc gagaagggta gggtttttta gaggtttggc gggaaaaaga acggagggag 1800
ggatcgcgtt gagtataaaa gtcggttttc ggggttttat ttaattcgtt gtagtaattt 1860
tagcgagagg tagagggagc gagcgggcgg tcggttaggg tggaagagtc gggcgagtag 1920
agttgcgttg tgggcgtttt gggaagggag attcggagcg aatagggggt ttcgtttttg 1980
gtttagtttt ttcgttgatt ttttaattag cggttcgtaa tttttgtcgt atttacgaaa 2040
ttttgtttat agtagcgggc ggatattttg tattggaatt tataatattc gagtaaagac 2100
gcgatttttt cgacgcgggg aggttatttt gtttatttgg ggatattttt tcgtcgttgt 2160
taggattcgt ttttttgaaa ggtttttttt gtagttgttt agacgttgga tttttttcgg 2220
gtggaaaatt aggtaagtat cgaagtttat ttgtttttta atttattttt ttattatttt 2280
aatgttgaga tgagtcgaat gtttaaatag ggtgtttttt tttttatttt tgcgttattg 2340
atattttttt tagagtagtt atggtaattg gggttggggt ggggggtaat ttagaattgg 2400
atcggggtaa agtgatttgt taagatggta gaggagaagg tagagggaaa acgggaatgg 2460
tttttaagat tattttttcg agatttttgt tttatgaata tatttacgtt gattttcggt 2520
cggtcggata tttttgtttt attgtgttaa ttgttttttg cgttttgggg ggttgggggt 2580
tgttttgcgg tgggtagaaa gttttttgta ttttgagttt tttggagtag ggatcgtata 2640
tcgtttgtgt gagttagatc gtttcgtagt cgttgatttg ttttcgtttt cgggagggta 2700
tttaaatttc ggtttatcgt atttttgata gtcggagacg gatattgcgg cgcgtttcgt 2760
tcgtttgttt tcgcggcgat tgtaattcgt tttgattttt ttaagaagtt ggtatttggt 2820
tttttaaaaa ataataatat aatttaaaat ttgggttttt agaggtgtta ggacgtggtg 2880
ttgggtaggc g 2891

<210> 202
<211> 2891
<212> DNA
<213> Artificial Sequence

<220>
<223> chemically treated genomic DNA (Homo sapiens)

<220>
<221> unsure
<222> (2526, 2669)
<223> unknown base

<400> 202

cgtttattta atattacgtt ttaatatttt tagagattta ggttttaaat tgtattattg 60
ttttttaaaa agttaaatgt taatttttta aaaggattag ggcgggttgt aatcgtcgcg 120
gggataggcg ggcgggacgc gtcgtagtgt tcgttttcgg ttgttagaaa tgcggtgagt 180
cgaaatttaa atgttttttc ggagacgggg ataagttagc ggttgcggag cgatttggtt 240
tatataggcg atatgcggtt tttattttaa ggagtttagg atgtaagggg ttttttgttt 300
atcgtaaagt aatttttagt tttttaaaac gtagagagta attaatataa taaagtagga 360
atgttcgatc ggtcgggagt tagcgtgaat atatttataa ggtagaaatt tcgaaagggt 420
agttttaaaa attattttcg tttttttttt gttttttttt ttgttatttt gataagttat 480
tttatttcga tttagttttg gattattttt tattttagtt ttaattatta taattatttt 540
gagaaaagtg ttaatagcgt aggaatggga gaaaagatat tttatttagg tattcgattt 600
attttagtat taaagtgata aaaaaataaa ttaaaaggta agtggatttc ggtgtttatt 660
tggtttttta ttcgaaaaaa atttagcgtt taagtagttg taaggagagt tttttagaga 720
agcgggtttt ggtagcggcg ggaagtgttt tttaaatggg tagaatagtt ttttcgcgtc 780
gggagagtcg cgtttttgtt cgggtgttgt aagttttagt gtaaagtgtt cgttcgttgt 840
tatggataaa gtttcgtgga tgcgataagg gttgcggatc gttggttggg ggattagcgg 900
gagggttggg ttagaggcga agttttttat tcgtttcgga tttttttttt taggacgttt 960
atagcgtagt tttgttcgtt cggttttttt atttagtcgg gtcgttcgtt cgtttttttt 1020
gtttttcgtt ggaattatta tagcgagtta gataaagttt cgaaaatcgg tttttatatt 1080
tagcgcgatt ttttttttcg tttttttttt cgttaagttt ttgagaagtt ttgttttttt 1140
cgaggtagga ggggagttag ggacggtcgg ggttcggcgg tggcggtcgc gagtagtata 1200
gttcgggggt ttttagtcgt ttagattttc gtattataaa gggtcggtgg gcggagatta 1260

gcgagagagg attttttttt ttttttttta cgttttttgt tttgggaatt cgggaggggc 1320
gtttatgggg agggtgggga gggtggggaa ggtggggagg agatttagtc gggtagtcga 1380
gtattttagt tttaggatgt aaatagagta agagagtcgt atgaattaat tacgcgcgtt 1440
tattattttt ttttgttttt ttttaaattt tttttttttt tttgttgttt tttcgtagta 1500
gtattgtttg ataaatcgta tttttgtttt gtgagtataa attatcgtag gcggaatagt 1560
tgttttttat atcgagaacg tattgcgcgt ttatcgttac gttacgcgcg tattaggttg 1620
tagggcgttt cgttaaggtt ggggaaaggg tcgcgttttg attaagagtt ttagggagag 1680
tggaggaaag aagggtatta atgggcgcgc gtttagagcg tgggatgtta gtgtagatag 1740
ggaggaatga tagaggtata aggaggaaaa cgatgtttag aatgattaaa ataatttagt 1800
aacgtattgt tacgtatatt tggagagcgc gttatgaata aatttttatt gtatttgttg 1860
gggggagtta tgtattatgt attatgtatg tatagttatt tggattggat attttttatt 1920
tagattgagt tttttaattt gttgttaaag tagtagatat cggttttttt gtttcgtttg 1980
tttaggtttt cgtggggttt cgtgcgggag gcgtttgttt agttttgaga tgtgtttgtt 2040
tgttttagag ttgggttagg gcgagaggga ggttgtttgt tttttgttag tttgtatttt 2100
atcgttttat ttttacgttt tttatacgga gtttttaatt ttttagttag gttttagaag 2160
agataaattt tttttgcgtt ttgtggcgtc ggtttgtaat agtttcgggt tgtcgggttt 2220
gggagaaatt aaaggtgtta gacgggagaa tatgggaggg gtagggggta ttcgaatcgc 2280
gggatcggat tttttaaaag gggtaagtgg agagtttgtg gatcgagtcg ggggagttag 2340
tagagatttt tgtgaaaaaa atcgttaatt tttttttttt cggataaatc ggacgtttaa 2400
ttttttttta ggtttttttt ttttttttat tattggaaat gcggttatgt ataaaaaatg 2460
tatcgatttt gattaaagaa gaggagagga gtattatttt cgtgtttttt tttggggggg 2520
gggggnagtt ttgagttaat taaaatttgg ttgtttttta ggtattaatt ttttagttta 2580
ttgatttata attttttttg aatattagtg aaagtgtatt gtatgtaatt cgtaaacgtg 2640
tatttatatt ttatttacgt ttcgatttna gttagttgtt tagttttata tatgatttgt 2700
ttgttttttg aaatgattta tatttaatat ataatgtata ttttttcggg attttttatt 2760
ttgtgttatt ttacggtatg aaaaataaaa aatatttttt ttatttttgg ttttttattt 2820
aaaggtattt taagtaaatt tttttttttt tttattattt ttaaaagaga aaataattcg 2880
ggggaaaggg g 2891

<210> 203
<211> 1092
<212> DNA
<213> Artificial Sequence

<220>
<223> chemically treated genomic DNA (Homo sapiens)

<400> 203

aagttttttt tttattattt aagaaggtat ttaggttttt ttgtgttagg ttttaggtaa 60
agtgttggat tatttagtaa ttgggtttag tagtaggatg gttttagatt gaggttttag 120
ggttaaagga ttattttttt ttttagcgtt ggttcggaaa aggtaagttt cgggcgggag 180
cgtacgtcgc gttttcgaag tttggttttt tcgttacgtt tattttttgt ttttatttcg 240
cgttttttta ggtttttttt cggtgaatcg gatgttttgt tagtttttta ttttgcgttt 300
tcggtcgcgg ttcgggtttt tcgtaaagtc gttgttattt cggagggttt agttagcggg 360
ttttcggagg ttggtcgggt aggcgtggtg cgcggtagga gttgggcgcg tacggttatc 420
gcgcgtggag gagatattgt tttgtcgcga tgggggttcg ggcgtttttt ttacgtcgta 480
ggtaagcggg gcggcggttg cggtatttgt ttatcgggag tttttttttt ttttttttgt 540
tgttgttgtt ttgtatttag ttcgggggag gatagaagaa aaaggaggta gaatggattt 600
ttttggtttt tttttgtggt cgggggcggg ttagggtggg tcgcgttgtt taggtagttt 660
tgtcgtgttg ttaggtagtt tggtcgtcgg cgtgggcgat gtcggcgttg gggcgggagt 720
cgcgagggtg ggaggttttg gggcgttttc gggacgtgga gttagtaggg ttttgatttg 780
aaaaacgacg gtaaagcgtg tttttgattg tttttgagta ttttatattt tttagattta 840
gggcgttttt atttttagtt ggaagtttag tttagagtaa tggtgttatt aaaaggggtg 900
tgttggatgt gaaaatattt tttggaagta tttataagtt tgtaggaaat atgttttttt 960
tattttttta ttttgttttt tttattattt attttaagaa gtaatagaat ttgtgtagag 1020
tgtgttttaa gttatattgt atgtttattt ttgtttatgt tgaattcggt gtatatttgt 1080
gagaataagt tt 1092

<210> 204
<211> 1092
<212> DNA
<213> Artificial Sequence

<220>
<223> chemically treated genomic DNA (Homo sapiens)

<400> 204

aagtttattt ttataagtat atatcgagtt taatataaat aaaaataaat atatagtgta 60
atttaaaata tattttgtat aggttttgtt gttttttgaa atgggtaatg aagggagtag 120
agtaagaaaa taaggaaaat atattttttg taggtttata aatattttta aagggtattt 180
ttatatttaa tatatttttt ttagtggtat tattgtttta agttgggttt ttagttggga 240
ataaaggcgt tttgggtttg aaaggtgtga ggtgtttaga agtagttaag aatacgtttt 300
gtcgtcgttt tttaagttag aattttgtta attttacgtt tcggaaacgt tttagggttt 360
tttattttcg cggttttcgt tttagcgtcg gtatcgttta cgtcggcgat taggttgttt 420
agtaatacgg tagggttgtt tgggtaacgc ggtttatttt ggttcgtttt cgattatagg 480
ggaaggttaa ggggatttat tttatttttt tttttttttg ttttttttcg agttggatgt 540
agagtagtag tagtaggaga aggaagggaa agttttcggt gggtaggtat cgtagtcgtc 600
gtttcgtttg tttacggcgt gaaggagcgt ttcgggtttt tatcgcggta gggtagtgtt 660
ttttttacgc gcggtagtcg tgcgcgttta gtttttatcg cgtattacgt ttgttcggtt 720
agttttcggg agttcgttgg ttgggttttt cgggatggta gcggttttgc gagggattcg 780
ggtcgcggtc gaggacgtag agtaggagat tgatagagta ttcggtttat cgggagaaga 840
tttggaaagg cgcgggatgg gggtaggaag tgggcgtggc gagggagtta ggtttcgggg 900
gcgcggcgtg cgttttcgtt cggagtttgt ttttttcgga ttagcgttga ggagaggagt 960
ggttttttgg ttttgggatt ttaatttgag gttattttgt tattgaattt aattattggg 1020
tagtttagta ttttatttgg ggtttagtat agaaagattt gaatgttttt ttggatgatg 1080
aaggggaagt tt 1092

<210> 205
<211> 5203
<212> DNA
<213> Artificial Sequence

<220>
<223> chemically treated genomic DNA (Homo sapiens).

<400> 205

agggaattta acgttttttc ggggtagaag ggggtagcgc gcgcgaggcg gaaggatagt 60
tattttcgtt gtataaattt tttcgcgggt cgcgtcgcgc gtgtaggggt tggacgttat 120
tcggcgttag agtcgggttt gaggagcggg gtttggtcgg gaacgagggt tgttagtttg 180
ggtggtgtta tttgttgttt tattttttat tttttttttt ttcgtgagga attagaggta 240
tcgggtcgcg cgggttgaga atttttaaat ttttttcggt cgtattcggt tgagttaatt 300
cgtagttttt gttagttttt ttgtttttcg ggtagcggtt tttttcgtta gttaggtttg 360
gaagttattt tttgtgagat tgggttagga agtgacgaaa agcgtcgaat tgtttttaaa 420
ttgaaaatat tttttttttt ttttttggag atagcgttga taaatatatg ggatttcggt 480
ttttgatttt tggttgtcgt ttttgttttt ttgtcgttaa taaaattcgt attgagtttt 540
ttagtgtttt gattaatagg tagattaatt tttattgggg ttgggaatag tttgttttaa 600
ttaatgttat ttttaaagtt cgttaggatg cgtttagttt cggttttttg gatgtttgag 660
cgagttagtt atgatatttg tttttttttt ttttttagtt ttaaattttt tagtaaagtt 720
tttagttttc gtttgtttgt ttttaggttt ataacgggat aaatcgtagg atcgggtcgt 780
tggtttttag aataggtagc ggtttagaga ggggatattt agggagaagg gattgaggtg 840
gttttgattt gtttgttttt tttttgaagt tgaatttttg ttttttgggg gtagaggtta 900
tagggaaaag agagatgggg ggatcgttat agggtgatga aatggaaagt aaattttttt 960
aggaatttta tttgtaaaaa tcggagagtg tcgttgattt tagttgtttt atatgttagg 1020
agttttaaat ttaattttta tttggataat agtatggagg gattgggagg taaagttttg 1080
ggagtttgat tcgttgggtt gttaggaatt tttttgttgg gagattttat tggataatgt 1140
tattttaaag gtattttggg ggtgggtagg gagaagatag agagagaaag agagagagag 1200
agagagagag agagagagag agagagatag gttggttttt tagagcgaac ggaagaggat 1260
tgaggattga atttagggag tttttagtat tggttatttg gtaggaggcg aggaagggcg 1320
agggtatcgc ggtttttttt taagtcgagt agtggttata gtttgaaggt gttacggggc 1380
ggttttggga tttttttggt ttttttaggt tttgggttcg gcgaggttgt cgggttagcg 1440
ggaatcggta ggttgttgga gaaaggagag ggataaaggt ttggaagtgg gggaggtatt 1500
agggattggg cggtcgggat gggagttttt gtttttaggg tttattggat tcgtttgtta 1560
gtttcgcgga taagggattt tattttttta gggtttgtaa aaagagagac gtttggggta 1620

ttataaagtg acgtaggtgt ttgttaagat tttttttttt taaaaaatgt tagtttttat 1680
tttttttagc gcgtcggcgt tgcgtttttg tttgatcggg gaaagagaga gtttggaggt 1740
gttagaattg ttagaatttc ggatgggagg tttttggttt tgaagttggg agttggaggt 1800
attttttttt agaagtgggt atttttttgt tttttcgaag gtttgatggg gtgggtatag 1860
gggaatgtta gggatcggtg cgggtgagat gtttgtttta ttttatgttg acgtattaat 1920
attggttaga agttttgggt aggttttggc gagttacggt tttttagcgg gatttttttt 1980
acgtagatag tttcgttatc gggtgggacg atttcggcgg ttttcgcggg aacgtattcg 2040
tcggtttgag aggttttttt tttggttata gggggcggag ggtagggggg aataggggtt 2100
tgtggggagg cggggaaagg agatagaatt taaagcgtaa ttttcgtcgt tttttcgcgt 2160
tttggagggt agtaagtggg tttcgggcgt gttggtagtc gtaattaaaa tggcgatttg 2220
cgagttgttt cggttttgta tatatttttg gtaatcgcgt tcgtatttta gtttcggttt 2280
ttatattttt aggtcgtttc gtttttagag tcggtatttg gtagagggag tgaggttttt 2340
tacgaggtag aatagtattt ttattttttt tgagattagt ttagagagaa cgaggaaatt 2400
ttatttttat tttttttttt ttaaattttg aaaattcggt tttattttgg ggttgaattt 2460
ttttttttaa atattgatat atgggaagta gttaagtttt ttttgttttt tgtttgtgcg 2520
gaggagtttt taggagtttt tttcggagtt ttttttttta tttaatgacg ttagtggtat 2580
ttatttttcg tttttgtttt tttttgattt tttgttaaaa tttttttttcg tagcgagatg 2640
ggggttggag gggtgggaga aggagttgat ttaaagagtt agtgttttgg ggggaaatag 2700
taataagatt acggttgttt tttttcgagg taaagtttaa gaaggtgtta atttattcgg 2760
tcgttaattg agaggttgta aaaatgatat tcgtttttag ttcggataga attatttttt 2820
tttttttcgt tttttcgtaa gtttttcgtt tatgtatagc ggatttgagt tttgagttta 2880
agttttttta atgttcgttt ggaaagtaaa aggagagaga aagagaaagt cgattttcgt 2940
tggtttggga tttagggtcg cggttttatt tgtcggtatt tttcgaaagt cgcgtttttg 3000
cgaaatgaaa tttcgtttag gaggtcgcgg atttggatat tcggcgttat tttttttatt 3060
tttgatttag gttttttttc ggcgttgcga gttttcgggg aagggttaga gtcggtagtt 3120
ttttttagtt cggggagggg agagggttat gcgattttat ttttggttag ggtcggggag 3180
gtttttgttt ttcgggagtt ttgttcgggt tttttggtcg tagggttgtt gggttttagg 3240
taggaacgag agggtgaggt ttatatgtgg ttcggcggtt tagggcggtt tgtagcgttt 3300
ttattgtttc ggttgttagg ggttgcggcg acgcggttag ttagtagcga gtttaggtcg 3360
cgtagatttt attgatgagt tttgattttt agtatttttt ttaagttaag aagagtttag 3420
cgtatttttc ggttgtttta ttttagtttt tttgttttag ttttttagtt ttattttttt 3480
tcgttttgtt ttggggtgtg tatagtagtt taggtttttt tttttttttt cggtttcgtg 3540
gttcgaagtc gtcgagagag ttcgggatag cgtaggatta ggtagtcgtt cgtttttttg 3600
ttattttaat tgtaggtttc gaggggcgtt tttggagtgt attgaggtgt gttttaatcg 3660
tgcggtattt aataaatgga tttttggtgt gtggttagaa gagaaaagtt atttatttat 3720
tttttttttc ggttttttgg taatagttga aggggagttg ttttcgtgga ttgagtagat 3780
ttaggagagg gagtcgtggt gcggagatat acgtattata tatagatgat cggtggtata 3840
tacgatatac gttgatatat cgatatcgtt agtgggatat atatatatat atatatatat 3900
atatatatat atatagagag agagagagaa ttttttttag tattggttat tcgttttttt 3960
atttaggttt ttattttttt ttttttttta ttttttttgg tttttttttt tttcgggcgt 4020
tgcgaaaagt agtcgtattt agttaataaa tggtacgtgg gagaagttgg tgagtgtttg 4080
gtgaggattt tttagggttt tttataagaa ttttttgtat ataaagtaag tggcgtgttt 4140
attcgggttt ttttagttag agttgtgttt ttgtttcgtt gcgtatcgcg gttttcgaaa 4200
ggagaaagga gagaagaaag ggcggggaga gcggggtgga ggatttggat aggttttgga 4260
ggtttgggtt ggggaggttt ttggtttcgt ttagttttcg gttcggtaat tttttttcgg 4320
tttaggtttc gtcgcggttt tcgtagttgg aatggagttg ttaggattta gtgacgtttt 4380
cgtttttttt tttttttttt aaggggttag gtgggttggg gtgcggtcgt cgttgtgttt 4440
tgtgttttgg ggtttcggtt gggatggggt gggggcgggc gggggcgggg cggtaggtta 4500
cgttgttttg gagttggtaa gaaaggatag tatagaaatt tgtatttttc gaggattggg 4560
agtttcgagt ttagtttagg gggagtgggg gcgcgatttt taatttagaa atttttattt 4620
gatcgtttaa gttcgcggta gtagggcggg tcgcgtcgaa tttcggcgtg cgcggagcgg 4680
ggagatgtag gcgagcgtta gagttcgggt tcgggggttt tgcgtcgggg agaggagtcg 4740
ggatttatcg gcggagtcga aaataagtgt atttatattt aaataaacgg attaattgta 4800
ttaggcgggg agagggagta tttaatcggt tggcgcgagg tttcggcgtt gttttgtata 4860
aagtaatatt ttgtgtgaga gcgagcggtg tatttgtatg ttgcggagtg attagtgggt 4920
ttgaaaaggg aatcgtggtt cggttttatt tttcgttttg gtttaggcgt aggaggaagt 4980
gttttgttgg aggatgatga tagaggttag gtttcgttaa tgggttagtg aggagcggtg 5040
gaggcgaggt cgggcgtcgg tatatatata ttaatatatt tgagttatta ttaattagta 5100
taggtgtgtt ggttgtagtt atttttttta tttatatttt ttatttttta ttttttagtc 5160
gttgatagtt tattttattg ttaatttttg tttttttttt agg 5203

<210> 206
<211> 5203

<212> DNA
<213> Artificial Sequence

<220>
<223> chemically treated genomic DNA (Homo sapiens)

<400> 206

tttgggaagg agatagagat tgataataaa atgggttgtt agcggttgga gagtgagaga 60
taaagagtgt gggtgaggga agtggttgta gttagtatat ttatgttgat tggtgatggt 120
ttaagtgtgt taatgtgtgt gtgtcggcgt tcggtttcgt ttttatcgtt ttttattggt 180
ttattagcga agtttgattt ttgttattat tttttagtaa aatatttttt tttgcgtttg 240
aattagagcg ggaaatgagg tcgagttacg gttttttttt taaatttatt aattatttcg 300
taatatgtaa atgtatcgtt cgtttttata taaaatattg ttttatgtaa agtagcgtcg 360
gggtttcgcg ttagtcgatt ggatgttttt ttttttcgtt tggtgtaatt ggttcgtttg 420
tttgaatatg aatatatttg ttttcggttt cgtcggtggg tttcggtttt ttttttcggc 480
gtagggtttt cgagttcggg ttttggcgtt cgtttgtatt ttttcgtttc gcgtacgtcg 540
agattcggcg cggttcgttt tgttgtcgcg aatttgagcg gttaagtgaa ggtttttggg 600
ttgggggtcg cgtttttatt ttttttaagt tggattcggg atttttagtt ttcggagggt 660
gtaagttttt gtgttgtttt tttttgttaa ttttaggata gcgtggtttg tcgtttcgtt 720
ttcgttcgtt tttattttat tttagtcggg gttttaagat atagagtata gcggcggtcg 780
tattttagtt tatttggttt tttggaagaa gaggaaaggg gcgggagcgt tattgggttt 840
tggtagtttt attttagttg cggaggtcgc ggcgaagttt aggtcgagag gaggttgtcg 900
ggtcgagaat taaacgaggt tagaggtttt tttagtttaa gtttttaggg tttgtttaaa 960
ttttttattt cgtttttttc gttttttttt tttttttttt ttttttcgga agtcgcggtg 1020
cgtagcggag tagaggtata gttttggttg gagaggttcg agtaaatacg ttatttattt 1080
tgtgtataga gggtttttgt gaaaagtttt gaagagtttt tattaaatat ttattaattt 1140
tttttacgtg ttatttgttg attaagtgcg gttgtttttc gtagcgttcg agaggagggg 1200
aagttagggg agataagagg ggagggggag tggaagtttg ggtggggggg cggatgatta 1260
atgttgggag ggattttttt tttttttttg tgtgtgtgtg tgtgtgtgtg tgtgtgtgtg 1320
tgtgtgtttt attggcgatg tcggtatgtt agcgtgtgtc gtgtgtgtta tcggttattt 1380
gtgtgtggtg cgtgtgtttt cgtattacga tttttttttt tgggtttgtt tagtttacgg 1440
aggtagtttt tttttagttg ttgttagaaa atcggggagg aaagtaagta aatggttttt 1500
tttttttgat tatatattag aagtttattt gttgaatgtc gtacgattag gatatatttt 1560
agtatatttt aaaggcgttt ttcggagttt gtagttaagg tgataggaga gcgagcggtt 1620
gtttggtttt gcgttgtttc gagtttttttc ggcggtttcg ggttacggag tcggaaggga 1680
gaaggaaggt ttgggttgtt gtatatattt taggataggg cgagggggaa tagggttgaa 1740
gagttgaggt agggaggttg aaatgaagta gtcgaagggt acgttagatt ttttttgatt 1800
tagggaaagt gttgaaagtt agagtttatt aataaaattt gcgcgatttg aattcgttgt 1860
tgattggtcg cgtcgtcgta gtttttggta gtcgggatag tgaggacgtt gtaagtcgtt 1920
ttgggtcgtc gggttatatg tgggttttat tttttcgttt ttgtttggga tttagtagtt 1980
ttgcgattaa ggagttcggg tagggttttc gggaagtaaa ggttttttcg gttttagtta 2040
gaggtggggt cgtataattt tttttttttt tcgggttggg gagggttgtc ggttttaatt 2100
ttttttcggg agttcgtagc gtcgggagga aatttgggtt agaggtgaag gaggtggcgt 2160
cgggtgttta ggttcgcggt tttttgggcg aggttttatt tcgtaggagc gcgattttcg 2220
gaggatgtcg gtaagtgggg tcgcggtttt gagttttaga ttagcgaaag tcggtttttt 2280
tttttttttt ttttttattt tttaagcgag tattaaagaa gtttggattt aagatttaag 2340
ttcgttgtgt atgagcgagg gatttgcggg ggggcggggg ggggggaagt gattttgttc 2400
gaattggagg cgagtattat ttttataatt ttttaattag cgatcgagtg gattagtatt 2460
tttttaagtt ttgtttcgga aagaagtagt cgtaattttg ttgttatttt tttttagggt 2520
attggttttt tgaattagtt ttttttttta tttttttaat ttttatttcg ttgcggagaa 2580
aagttttaat aaaaaattag aaaaggtaag gagcgaggag tgaatgttat tgacgttatt 2640
gggtggggaa gggggtttcg ggaaagattt ttggaaattt tttcgtataa ataaaaaata 2700
aaaaggattt ggttgttttt tatgtgttaa tatttgggga ggggagttta attttagaat 2760
gaggtcgagt ttttaaagtt taaggagaga ggggtgagag tgagattttt tcgttttttt 2820
tggattggtt ttaaggaagg tgggggtgtt gttttatttc gtggggagtt ttattttttt 2880
tgttaagtat cggttttggg ggcgaagcgg tttaggggtg tgggggtcga gattgaggtg 2940
cgggcgcggt tgttagaggt gtgtataaaa tcgagataat tcgtaggtcg ttattttaat 3000
tgcggttatt aatacgttcg aggtttattt gttgtttttt aagacgcggg ggggcggcgg 3060
ggattgcgtt ttaggttttg tttttttttt tcgttttttt ataggttttt gttttttttt 3120
atttttcgtt ttttgtggtt agagggagga ttttttaggt cggcgggtgc gttttcgcgg 3180
gggtcgtcgg ggtcgtttta ttcggtagcg gggttgtttg cgtggaggga gtttcgttgg 3240
gggatcgtgg ttcgttagag tttgtttaga gtttttagtt agtattgatg cgttaatatg 3300

aggtgaggta agtattttat tcgtatcgat ttttggtatt tttttgtgtt tattttatta 3360
ggttttcggg gaggtaaaga agtgtttatt tttgggggga agtgttttta gtttttagtt 3420
ttagaattaa gagtttttta ttcggagttt tgatagtttt gatattttta aatttttttt 3480
tttttcggtt aagtagaggc gtagcgtcgg cgcgttggaa agggtgaaga ttggtatttt 3540
ttggagaagg aaagttttgg tagatatttg cgttattttg tagtgtttta aacgtttttt 3600
tttttgtaga ttttgggaag gtgggatttt ttgttcgcgg agttggtaaa cgggtttagt 3660
gagttttaaa ggtaaaggtt tttatttcgg tcgtttagtt tttggtgttt tttttatttt 3720
taggtttttg tttttttttt ttttttagta atttgtcggt tttcgttaat tcggtagttt 3780
cgtcgagttt agagtttaga aaagttaaga agattttaga gtcgtttcgt ggtattttta 3840
ggttgtggtt attgttcggt ttggggggaa gtcgcggtgt tttcgttttt tttcgttttt 3900
tgttaaatgg ttagtgttag gggttttttg ggtttagttt ttagtttttt ttcgttcgtt 3960
ttgggaggtt agtttgtttt tttttttttt tttttttttt tttttttttt tttttttttt 4020
tttttgtttt ttttttgttt atttttaaaa tatttttaaa ataatattgt ttagtggagt 4080
tttttagtaa agggattttt agtagtttag cgggttagat ttttaaggtt ttgtttttta 4140
gtttttttat gttgttgttt agataggagt taggtttaaa atttttgata tgtaggataa 4200
ttgaaattag cggtattttt cgatttttgt aaatagggtt tttagaaaag tttgtttttt 4260
attttattat tttgtgacga tttttttatt tttttttttt ttgtaatttt tgtttttaag 4320
aggtaagagt ttagttttaa aggaaaagta agtaagttaa ggttatttta gttttttttt 4380
tttggatatt ttttttttaa atcgttgttt attttaaagg ttaacgattc ggttttgcga 4440
tttgtttcgt tgtagatttg ggaataggta ggcgggaatt gggggtttta ttgggggatt 4500
tgaggttggg gagggggagg gagtaaatgt tatggttggt tcgtttaagt atttagggaa 4560
tcgaagttaa gcgtattttg acgggttttt aaaatgatat tgattaggat aagttgtttt 4620
taattttagt aagagttaat ttgtttgtta attaaggtat taaggggttt aatgcgagtt 4680
ttattagcga taggagaata gaggcggtag ttagggatta aaagtcggga ttttatatat 4740
ttgttagcgt tatttttaaa aaaaaaaaaa aaagtatttt taatttgaaa ataattcggc 4800
gtttttcgtt attttttaat ttagttttat agagggtgat ttttaaattt ggttagcggg 4860
gaaaatcgtt gttcggggga tagaggggtt gataggaatt gcgggttggt ttagtcgaat 4920
gcggtcgggg agaatttaag aatttttagt tcgcgcggtt cgatgttttt gattttttac 4980
gagaggaaag ggaatgaaaa atgaagtaat aaatgatatt atttaggttg gtagttttcg 5040
ttttcggtta gatttcgttt tttaggttcg gttttggcgt cgggtggcgt ttagtttttg 5100
tacgcgcggc gcggttcgcg ggaaagtttg tgtagcgaga gtgattgttt tttcgtttcg 5160
cgcgcgttgt ttttttttgt ttcggagggg cgttgggttt ttt 5203

<210> 207
<211> 8085
<212> DNA
<213> Artificial Sequence

<220>
<223> chemically treated genomic DNA (Homo sapiens)

<220>
<221> unsure
<222> (2545)
<223> unknown base

<400> 207

gttttatgat ttaattattt tttaatattt ttttttatta agattattat attggtgatt 60
gggttttgat atatagattt ggggagaata taaatattta tattatagta tttattaatt 120
tatttagtta ttttattgtt aggtatttat ttagaaaaaa tgaaaatata tgtttatata 180
aagatttgta taatattttt atagtagttt tatttataat attaaaaaaa ttggaaataa 240
tttagatatt tatttatagt taaatggata aataaattat agtatataaa taaaaagaat 300
aaaaagaatt aattattgat atatattata atttagttga attttaaaat aattatgttg 360
agtgagagaa attaaatgaa aaaaagatat attatattat atataatttt aagaaataaa 420
atttaattta tttggtattt agatttttat ttttatatta gttttaaata aaaagtttta 480
aatttttttg gataaatggt tgattttacg attggggtag gaaatatata agatgagttt 540
gatgagtttg tagtattttg taatattaga aagtaggaaa gtttatatat atatatatat 600
atatataata tagagagtgg gttaaggagt ataagagtta attgaaaaag tttttagtgt 660
taaaaattgg aaaaatttga gtaataaagt atttatgaat ttatataaag aaaagattga 720
ataaagaagt aaatggggga gaagaagtaa atttttttta tagaatttta aataaattat 780
gtagatattt tattttaaag gagggttagt ataatttttt atttttttat atttttttaa 840
gtgatttata atgatttttt tttaaagagt atagagtatg taaagaggga aaaaagagta 900

attatgtaat ggagaaattt gataaatatt attttagtta ggtgatggag aataatatta 960
atagttataa gttatgttga taatatgtat tttttatatg atgtaataaa tatagtatat 1020
tatttttttg atttttattt cgttaaaatt taaaatttta gtttatttat gaggatatat 1080
taaattttaa tagaagggta ttttgtaata tatttattta gtatttttaa ggattgttaa 1140
ggttattaaa tataaggaaa atttgagaaa tttttatagt taagaggagt ataagatatt 1200
ataataatta aatgtggtgt tttggatgga attttggaaa ggaaaaagga tattaagtga 1260
aaatgaagaa aatttaaata aattatgaat attagttaat aataatttat tagtattagt 1320
ttattaattg taataaatgt attttattaa tttaaaatgt taatagagaa aattgtataa 1380
atgatatatg agtatttttt atattatttg tttaattttt ttgtaaattt aaaatcgttt 1440
tttaaaagta aaatttatta ataaaatatt taaagtaaat agatttaaaa ggtatatgta 1500
aatgtataat ttaatgttta ataaattaga tataaatatt tttaaaaatt taatatttgg 1560
tgatagaaag tagattagtg gtttgttaga aagtggtgat tagtattagg ggaaggggat 1620
gggatagaat agtatgaagg gatttaaaaa atggtatgag gaaatttttg gaggtaaagg 1680
gtatgtttat tattttagtt ttataggttt aataataaat tatataattt aaatatatgt 1740
agtttattat atgataatta tattttgata aagtggtttt tttttttaaa ttatagaaaa 1800
aatattttta tatttaaatt ttttttttta aaaatagttt ttaggaatat ttgaagttag 1860
ttttattata tttgaattta attagtgaat tttgtttatt taaagattta tatatttggg 1920
aagtttttgt gttaaaaatg ttaattattt agtattaaaa tttatggaat aatggtgatt 1980
taggatatgt tggatatttt tattgttgtt tttttatttt aagattttta attttttgga 2040
aataattttt ttagatattt tgataaggga attattataa ttaattaaaa ttaagttaaa 2100
tatagtttgt tagatttaaa atgaggttaa gtgttaattt tatagtagcg atcggaaaat 2160
taaattatat ggtatttgat atttaaaatt atatttttaa gaaggttgtt atttgatgtt 2220
ttgggtttgg gttatagtat ttaattttat aatattgggt aattttgtgt tttagttttt 2280
ttaattatga aattgggata atagtattta ttttgtgaga ttgttgaagg attatagtta 2340
atataggtaa aaggtttata agattatttg gtaatgttgt tttgatttta taaattttat 2400
atattgtgtg gtattagtta ttaggaaaat ataaataaat tagtgagata ttattttata 2460
tatttggggg gttataataa aaaagataga taaaaaatgt tgttaaagat gtgaagaaat 2520
taggattttt atttattgtt ggtgnaaatg taagaagtat aattatttga attatagttt 2580
ggtagttatt ttaaagatta tatttagagt tgttatatga attagtaatt ttatttttat 2640
gtatatattt aggagaaagg aaaatatacg ttgtataaaa atttgtatat gaatgtttat 2700
tagtaggatt ttttatggta gttaaaaggt gtaaataatt taaatgttta ataattgata 2760
gagatggata aataaaatgt ggtttattta tataatgaaa tattatttgt taataaaaag 2820
gagtgaagga gtgaagtatt gatttatgtg gaatatggat gaattttgat ttaaaatagt 2880
taaattatag gtataaaaag tatattagtg gttgttggga gatttaggcg gaaatgtgga 2940
ataattgtta gtgggtattg agattttaga gttatattta tgttataaaa ttaatagtgt 3000
tgatggttgt ataattttga gtatatgaaa aattaatgaa ttgatatttt gagtgagttg 3060
tatgatattg gaattatatt ttaataaagt atggtaattg ttttaagata ggttggaaag 3120
agaaagtttg aaaataataa taatgatatt aataaattag tttatttttt tagttttata 3180
tatttttgtg tttatatttg tttttgtttt atttataatg gtttttttgt agttgttata 3240
ttatattttg ttatttgatg ttcggtgaat attttatatt tgttttttag aatttttttt 3300
attttttttt tatttgttta atttttatat atttaaaatt aattagagta aattatttat 3360
tagaataatt aattttaaat tttagtaatt taatatgata aaggtttgtt ttttatttat 3420
atagtttttt tttagatgat cgaggggttt aggtttttta tttttagtgg tttttttatt 3480
ttttggagtt ttttgtattt tttatatatg gttgagataa attatgagtt attagtatag 3540
ttagattttg aggttttata agaaaatttg taaattattt attttgtttt gaataaggta 3600
tatttaagat gatgttaaaa tatttaatgg ttttgggtta aatatagttt atgattgtgt 3660
atttaaaata tatattgtaa tatttttttt tttttttatt gattttatga atttagcggg 3720
gatttatttt ataagtttaa agataattat tttttagatt aagaatattt agggtaaaaa 3780
gtattgttta atatttttat tgaggatgtt atgatgtagt atattgtata agttggagtt 3840
aaaggaaatt ttttttaaag tgttatttat taaaaattgg aatatatttt ttaagataaa 3900
tcgaagtgtg gtatataata tttaaatttt tattatagat atagaggtgt tattattttt 3960
tatttttaaa tttttttgtt acgttgagga tatttaagag gagtaggata tgttggtcgt 4020
agtaggagaa atttgaaagt atttattttt atggaattta taagggagag aattttttat 4080
tttagtatcg tttttgatat atttattatt ttaaaagata atgtagttaa atgttttttt 4140
ttgtgttaaa tttttataaa attgaaattt taaaatggtg ataaaaattt tatttttgat 4200
agaatttatt tattttttta attagatagg gtataatttt taatttgtaa aataaaacgt 4260
aatatgttta tgaggtttta ttttaaagaa tttgttattg agagtagtat ttagaataac 4320
gggtggaaat gttaatttta gagttttaga ttttatcggt aattggggta gggaggggtt 4380
ttgggcgggg tttttttaga ggaggaggcg ttgttagaaa gttgtttggt tagtttatag 4440
ttgttattaa tcggggtaag ttttgttgta tttgtgcgtg tgggtggtat ttttaatgag 4500
aattagtttt atttgttatt tgagtgaaat ttataattcg aggcggttag tgttttcgta 4560
ttattgggat ttgagatttt cggagatgat tgtcgttcgt agtacggagt tagtagaagt 4620
tcgatttttt ttgggaatgg gttgtatcga gaggttcgat tagttttagg gttttagtga 4680

gggggtagtg gaatttagcg agggattgag agttttatag tatgtacgag tttgatgtta 4740
gagaaaaagt cgggagataa aggagtcgcg tgttattaaa ttgtcgtcgt agtcgtagtt 4800
atttaagtgt cggatttgtg agtattttgc gtttttagtt ttcggataga agttggagaa 4860
tttttttgga gaatttttcg agttaggaga cgagattttt taataattat tatttttttt 4920
tgcgtttttt atttgtcgtt cgttgggata aacgatagtt atagtttttt tgacgatagg 4980
atggaggtta agggtaggag ttgattagcg tcgttttttt tcgttttcga tttaggaggt 5040
ggagattttt tcggtttagt tatatttaat atttattttt tttttttttt gtttttatat 5100
tttcgaaatt tttttttttt tttttttttt tttttttttg gagacggggg aggagaaaag 5160
gggagtttag tcgttatgat tgagttgaag gtaaagggtt ttcgggtttt ttacgtggcg 5220
ggcggttcgt tttttttcga ggtcggattt ttattgttgt gtcgtttagt cgtaggttcg 5280
ttttcgggga gttagatttc ggatattttg tttgaagttt cggttatatt tatttttttg 5340
gacgggttat ttttttttcg gttttgttag ggataggatt ttttcgacga aaagacgtag 5400
gattagtagt cgttgtcgga cgtggagggc gtatatttta gagttgaagt tataaggggt 5460
gttggaggta gtagttttag ttttttagaa aaggatagcg gattgttgga tagtgttttg 5520
gatattttgt tggcgttttt aggtttcggg tagagttaat ttagtttttt cgtttgcgag 5580
gttattagtt tttggtgttt gtttggtttc gaatttttcg aagatttatc ggttgttttc 5640
gttatttagc gggtgttgtt ttcgtttatg agtcggttcg ggtgtaaggt tggagatagt 5700
ttcgggacgg tagttgttta taaagtgttg tttcggggtt tgttattagt tcggtagttg 5760
ttgttttcgg tttttgagag tttttattgg ttcggggttt tagtgaagtc gttttcgtag 5820
gtcgttgcgg tggaggttga ggaggaggat ggttttgagt tcgaggagtt tgcgggttcg 5880
tttttgaagg gtaaatttcg ggttttgggt ggcgcggcgg ttggaggagg agtcgcggtt 5940
gtttcgtcgg ggcggtagta ggaggcgtcg ttttggtttt taaggaagat tttcgttttt 6000
tagcgtttag ggtcgttttg gtggagtagg acgcgtcgat cgttcgggac gttttcgttg 6060
gttattacgg tgatggattt tatttacgtg tttattttgt tttttaatta cgttttattg 6120
gtagttcgta ttcggtagtt gttggaagac gaaagttacg acggcggggt cggggttgtt 6180
agcgtttttg tttcgtcgcg gagtttattt tgtgtttcgt ttatttcggt cgttgtaggc 6240
gattttttcg attgcgcgta ttcgttcgac gtcgagttta aggacgacgc gtattttttt 6300
tatagcgatt tttagtcgtt cgttttaaag ataaaggagg aggaggaagg cgcggaggtt 6360
ttcgcgcgtt tttcgcgttt ttattttgtg gtcggtgtta atttcgtagt tttttcggat 6420
ttttcgttgg ggttatcgtt ttcgttgtcg tcgcgagcga ttttatttag attcggggaa 6480
gcggcggtga cggtcgtatt cgttagtgtt ttagtttcgt ttgcgttttt ttcggggtcg 6540
attttggagt gtattttgta taaagcggag ggcgcgtcgt tttagtaggg ttcgttcgcg 6600
tcgtcgtttt gtaaggcgtc gggcgcgagc ggttgtttgt tttcgcggga cggtttgttt 6660
tttattttcg tttttgtcgt cgtcgtcggg gcggttttcg cgttttattt tgtattcggt 6720
tttaacgggt tttcgtagtt cggttattag gtcgtcgtgt ttaaggaggg tttgtcgtag 6780
gtttattcgt tttattttaa ttatttgagg tgagggttcg ggacggggta cgtttagcgc 6840
gttcgggagt agcggtttcg ttggcggcgg cggtcgttaa tttttagttt tagttttagc 6900
gtatcgttgc gtttttcggg gcggtcggag agggtgggta gcgggatata gtataggggt 6960
agttgttttt tttttttttt tttttttttt ttatttttgg ggatacgaag gtgggcgtag 7020
aatatattat ttttggggcg tgtttttttg aaagttgttt ttttgtttgt tttttaattt 7080
ttcgaatttt ttagatttcg aagtagaatt aatttcgatt taaaacgtgt agcgttatat 7140
taggttcgtt gtagtttagt ggggtagaaa gtgcgcggcg agttgggggt tttatgaaat 7200
gttttttttt tagaagaagg acgtttatta ggagtgtttg ttttggagag gagttaaggt 7260
atcgtttttt cgggaggggt gggatttgag aggtggtcgg ttagaatcga aagtagtatt 7320
attttaggga tttgaatatt ttagtggttt agttttttta agaattttaa gattaaaatt 7380
aagtttacgt gggaaatgtt taaattgtgg atttaaacgt ttgttattgt attgtatcgt 7440
ttttttatta ttgtttgtta tttattataa ttttttttat atataggttt aaaaaatatt 7500
attttgtata ttgaagtaat ggaatgtaaa aaaagaatgt tttgtttgga attttatgtt 7560
gtgaataggt aaaatagtgt tagtgtattg gataatattt taaaatgata aatatatatt 7620
tgtttaagta agtaatgatt atagggttgt gttttaaaaa tttaaaatta aaatattgta 7680
aagtattatc gaatttttaa agttaaatta tatttgtttt gatttagtat atagatagga 7740
aggatataat attttatttg ttaaagatta aattgttttt atataaagag ttttgtagaa 7800
agattttttt ttaatcgatt ttaatttttt aggatataat attatatatt aattattgtt 7860
tttttatatt ggtgttattg atgaatggtt aattatttgt aagtatggtg aatttagtta 7920
cggatagttt attattaagt ttagtttgta tgttttttaa gtgtatatat atagttttgt 7980
ttttaaaatt tttttttatt ttgttaatat tggtttaaga aatttttagt attagatagt 8040
ggtgtattta aaaataaatg gagtattttg ttttgtattt taagg 8085

<210> 208
<211> 8085
<212> DNA
<213> Artificial Sequence

<220>
<223> chemically treated genomic DNA (Homo sapiens)

<220>
<221> unsure
<222> (5541)
<223> unknown base

<400> 208

ttttgaaatg taaaataaag tattttattt atttttaagt gtattattat ttaatattaa 60
aggtttttta aattagtatt aatagggtga aaggagattt taaaaataga attgtatata 120
tatatttgaa agatatgtaa attaaatttg gtaatagatt attcgtaatt ggatttatta 180
tatttgtaaa tgattagtta tttattagta gtattaatat aaaagaataa taattagtgt 240
ataatattat gttttagaaa gttaaagtcg gttaaaagga aattttttta taaaattttt 300
tatatagaaa taatttagtt tttgataaat gaaatgttat gttttttttg tttgtatgtt 360
gggttaaaat aaatatggtt tggttttaaa agttcgatgg tattttgtaa tgttttggtt 420
ttgagttttt aaaatataat tttgtaatta ttgtttattt aagtaagtat atgtttgtta 480
ttttaaagta ttgtttaata tattgatatt gttttgttta tttataatat aagattttaa 540
atagagtatt ttttttttat attttattat tttagtatgt aaagtagtgt tttttaaatt 600
tgtatataaa aaaaattgta gtgaatagta agtaataata agaaaacggt gtaatgtagt 660
gataggcgtt taaatttata gtttaaatat tttttacgtg aatttaattt taattttgag 720
atttttaaga aaattgagtt attgaagtgt ttaaattttt aagatggtgt tgttttcggt 780
tttggtcggt tattttttaa gttttatttt tttcggggga acggtgtttt aatttttttt 840
taagataagt atttttggta aacgtttttt ttttaagaaa gaagtatttt ataaagtttt 900
taattcgtcg cgtatttttt gttttattgg gttatagcgg atttagtgtg acgttgtacg 960
ttttaaatcg ggttggtttt tgtttcggaa tttggaagat tcggaaagtt aaaaaataaa 1020
taaaaaaata gtttttaggg aggtacgttt taaaaatagt atattttgcg tttattttcg 1080
tgtttttaag agtgaggaga ggagggaaga agaagggagg taattgtttt tgtgttgtgt 1140
ttcgttgttt atttttttcg gtcgtttcgg ggagcgtagc ggtgcgttgg ggttggggtt 1200
gagggttggc ggtcgtcgtc gttaacggaa tcgttatttt cggacgcgtt gggcgtgttt 1260
cgtttcgggt ttttatttta ggtagttgag atagggcggg tagatttgcg gtaggttttt 1320
tttgagtacg gcggtttggt agtcgagttg cgggagttcg ttgaggtcga gtgtagggta 1380
gagcgcgggg gtcgtttcgg cggcggcggt agaggcggag gtggagggta ggtcgtttcg 1440
cgggagtagg tagtcgttcg cgttcggcgt tttgtagggc ggcggcgcga acgggttttg 1500
ttggggcggc gcgtttttcg ttttgtatag gatgtatttt agggtcgatt tcgaggagga 1560
cgtagacgag attgaggtat tggcgggtgc ggtcgttatc gtcgtttttt cgggtttgga 1620
tggggtcgtt cgcggcggta gcgggggcgg tggttttaac gggaaattcg ggaaggttgc 1680
ggggttggta tcggttataa ggtaggaacg cggggagcgc gcggaggttt tcgcgttttt 1740
tttttttttt tttattttta gagcgggcgg ttggaagtcg ttatagagag ggtacgcgtc 1800
gtttttgggt tcggcgtcgg gcgggtacgc gtagtcgggg aagtcgttta tagcgatcgg 1860
ggtggacgag gtatagggtg aatttcgcgg cggggtaaag gcgttggtag tttcggtttc 1920
gtcgtcgtaa ttttcgtttt ttagtagttg tcgagtgcgg gttgttaata aggcgtgatt 1980
gagaggtagg ataggtacgt ggatgaaatt tattatcgtg gtggttagcg gggacgtttc 2040
ggacgatcgg cgcgttttgt tttattaggg cgattttggg cgttgagaag cgggaatttt 2100
ttttggggat tagggcgacg ttttttgttg tcgtttcggc gggatagtcg cggttttttt 2160
tttagtcgtc gcgttattta gagttcgagg tttgtttttt agaagcggat tcgtagattt 2220
ttcggattta gagttatttt tttttttaat ttttatcgta gcggtttgcg gagacggttt 2280
tattggggtt tcggattagt gagggttttt agaggtcggg agtagtagtt gtcgggttgg 2340
tgataggttt cggggtagta ttttatgggt agttgtcgtt tcggagttgt ttttaatttt 2400
gtattcggat cggtttatga gcggggataa tattcgttgg gtggcggggg tagtcggtgg 2460
attttcggga agttcggggt taaataggta ttaagagttg gtgatttcgt aggcgggagg 2520
gttgggttgg ttttgttcgg gatttgaggg cgttaataga gtgtttaaga tattgtttag 2580
tagttcgttg tttttttttg ggggattaga attgttgttt ttagtatttt ttgtagtttt 2640
agttttggaa tatgcgtttt ttacgttcga tagcgattgt tggttttgcg ttttttcgtc 2700
ggaggggttt tgtttttggt agggtcgagg gaagagtagt tcgtttaggg agataggtat 2760
ggtcgaaatt ttaggtaagg tgttcgaggt ttggtttttc gggaacggat ttgcggttgg 2820
gcgatatagt agtggggatt cgatttcggg ggagggcggg tcgttcgtta cgtggggagt 2880
tcggggattt tttgttttta gtttagttat gacgattgga tttttttttt tttttttttc 2940
gtttttaggg aggagggaaa agggaaggag gagggggttt cgggaatata ggggtagagg 3000
gaggagaaag tgggtgttga atgtggttgg atcggaggga tttttatttt ttgggtcggg 3060
ggcgggggag ggcggcgttg gttagttttt gtttttggtt tttattttgt cgttagggga 3120
attgtggttg tcgtttgttt tagcgagcgg taagtgggga gcgtaagaaa aagtagtaat 3180

tgttaggaga tttcgttttt taattcgggg agttttttaa gagagttttt taatttttgt 3240
tcgaggattg gagacgtaga gtatttataa gttcggtatt tgagtggttg cggttgcgac 3300
ggtaatttag tgatacgcgg tttttttatt tttcgatttt ttttttggta ttaaattcgt 3360
gtatgttgtg aagtttttag tttttcgttg agttttattg tttttttatt aaaattttgg 3420
ggttagtcgg atttttcggt atagtttatt tttaggaagg gtcggatttt tgttggtttc 3480
gtattgcggg cgatagttat tttcgaagat tttagatttt agtagtgcgg gagtattagt 3540
cgtttcgggt tgtagatttt atttaaatga taagtgaagt tagtttttat tgagaatgtt 3600
atttatacgt ataaatataa taaggtttat ttcgattagt gatagttgtg gattggttag 3660
atagtttttt aataacgttt ttttttttag ggaggtttcg tttaaagttt ttttttattt 3720
taattatcgg taggatttga aattttggag ttggtatttt tattcgttat tttgaatgtt 3780
atttttaata gtaggttttt tgggatggaa ttttataagt atattacgtt ttgttttgta 3840
aattaagaat tatgttttat ttaattggaa aaatgaatag attttattag aagtagaatt 3900
tttgttatta ttttaagatt ttagttttgt aaagatttaa tatagaggaa gatatttggt 3960
tatattattt tttaaaataa taaatgtatt aaggacgata ttaaaataag aaattttttt 4020
ttttatgagt tttataaaag tgaatgtttt taagtttttt ttgttgcgat taatatgttt 4080
tgtttttttt gagtattttt agcgtgataa agaaatttgg gagtgggaga tggtaatatt 4140
tttgtattta tgatggaagt ttggatgttg tgtgttatat ttcgatttgt tttaaggaat 4200
gtgttttaat ttttagtaaa tagtatttta aggaaagttt tttttagttt tagtttatat 4260
agtgtgttat attataatat ttttagtaga gatgttgaat agtatttttt attttaaata 4320
tttttagttt gaaaagtaat tatttttgag tttataaaat ggattttcgt taaatttatg 4380
aagttagtag aaaaagggaa gaatattgta atatatattt tagatatata gttataaatt 4440
gtatttgatt taagattatt gggtatttta atattatttt aaatatattt tgtttaaaat 4500
agagtgaatg atttgtaaat ttttttgtag gattttaagg tttagttgtg ttaatgattt 4560
atagtttatt ttaattatgt ataaagaatg tagaagattt tagaaggtgg gggagttatt 4620
agaggtaagg agtttggatt tttcgattat ttggggaggg gttatatgag tgagaaataa 4680
atttttatta tgttaggtta ttaggatttg gagttggttg ttttagtaaa tagtttattt 4740
tgattaattt tagatatgtg gaagttaggt agatagagga aaggtaaaga gaattttggg 4800
aagtaggtat agaatgttta tcgggtatta aatggtagga tataatatgg taattgtaag 4860
gggattatta tgaatagaat aggagtaagt gtgggtatag aagtatatga gattagagaa 4920
gtaaattaat ttattaatat tattattgtt gtttttaggt tttttttttt tagtttattt 4980
taaaatagtt attatgtttt attgaggtgt aattttagta ttatatagtt tatttaaagt 5040
attagtttat tgatttttta tgtatttaga gttgtgtaat tattagtatt attaattttg 5100
taatatgagt atgattttaa aattttaata tttattagta gttattttat attttcgttt 5160
aaatttttta gtagttatta atatgttttt tgtatttgtg atttggttat tttggattaa 5220
ggtttattta tgttttatat gaattagtat tttatttttt tatttttttt tattgataaa 5280
taatatttta ttgtatggat aaattatatt ttatttattt atttttatta gttgttggat 5340
atttgggttg tttatatttt ttggttatta tgaaaaattt tgttaatgaa tatttatgta 5400
taagtttttg tgtagcgtat attttttttt tttttggata tatgtatagg agtggaattg 5460
ttggtttata tggtaatttt aggtgtggtt tttggagtaa ttgttagatt atggtttaaa 5520
tgattgtatt ttttatattt ntattagtag tgaataaggg ttttagtttt tttatatttt 5580
tgataatatt ttttatttgt tttttttgtt atagtttttt aagtgtgtga agtggtattt 5640
tattgattta tttatatttt tttgatggtt aatgttatat agtgtataga atttgtaaaa 5700
ttaggatagt attgttaggt agttttgtaa gtttttattt tgtgttaatt gtaatttttt 5760
aatagtttta taaaataggt attgttattt taattttata gttgggaaaa ttgaggtata 5820
aggttattta gtattataaa gttaagtatt gtaatttaaa tttaagatat taaatgatag 5880
tttttttagg agtgtgattt taaatgttag atgttatata atttgatttt tcggtcgttg 5940
ttgtaaaatt ggtatttggt tttattttaa atttgataaa ttatatttgg tttgattttg 6000
gttggttata ataatttttt tgttagaatg tttggggagg ttgtttttaa aagattgaaa 6060
attttgaagt gggaaaataa taataaagat gtttagtatg ttttaaatta ttattatttt 6120
ataaattttg atgttgggtg attagtattt ttggtatagg aattttttaa atgtatgaat 6180
ttttgaatga ataaaattta ttgattaaat ttaggtgtga tggggttaat tttaaatatt 6240
tttgaagatt gtttttggag aagaggattt aaatgtggaa gtattttttt tatagtttgg 6300
ggaaaaaaat tattttatta agatatagtt gttatataat aagttgtata tatttaaatt 6360
gtataatttg ttgttagatt tgtgaaatta aaatgataaa tatatttttt gtttttaaaa 6420
gtttttttat gttatttttt aaattttttt atattgtttt gttttatttt ttttttttag 6480
tattaattat tattttttgg taagttatta atttgttttt tgttattaaa tattaggttt 6540
ttaaaaatat ttatatttga tttattaaat attaaattat atatttgtat atgtttttta 6600
ggtttgtttg ttttagatgt tttattaata gattttattt ttgggaaacg attttggatt 6660
tatagaaaaa ttgagtagat aatatagaga atgtttatat attatttgta taattttttt 6720
tgttagtatt ttaaattaat agggtatatt tgttataatt aatgaattaa tattgataaa 6780
ttgttattaa ttaatattta tagtttattt agattttttt tatttttatt taatgttttt 6840
tttttttttt aggattttat ttaggatatt atatttaatt gttatgatgt tttgtgtttt 6900
ttttggttgt gagagttttt tagatttttt ttgtatttga tgattttgat agtttttagg 6960

agtattggtg aggtatattg taagatgttt ttttattggg atttgatgta tttttatgag 7020
tagattaggg ttttgggttt tggcggggtg gaaattaaag aggtaatgta ttgtatttat 7080
tatattatat gaagggtata tattgttaat atgatttatg attgttaatg ttgtttttta 7140
ttatttggtt gaagtagtgt ttgttaggtt tttttattgt atagttattt tttttttttt 7200
ttttgtatat tttgtatttt ttggaaggaa attattatga gttatttaag gaagtgtggg 7260
ggagtaggga gttatgttag tttttttttg gagtggagta tttatataat ttatttggaa 7320
ttttgtgagg aagatttgtt tttttttttt tatttatttt tttatttagt tttttttttg 7380
tatggattta tgaatatttt gttgtttaaa tttttttagt ttttggtatt gggggttttt 7440
ttagttgatt tttgtgtttt ttgatttatt ttttgtattg tgtgtatgtg tgtgtatgtg 7500
tggatttttt tattttttgg tattataaga tgttatagat ttattagatt tattttgtat 7560
attttttgtt ttagtcgtag aattagttat ttatttaagg aagtttgaga ttttttattt 7620
gaaattggta tagaaatgaa gatttgggtg ttaaataaat taggttttat tttttagaat 7680
tgtatataat atagtgtatt ttttttttat ttggtttttt ttatttagta taattatttt 7740
gagatttagt taaattatag tatgtattaa tagttaattt tttttatttt ttttatttat 7800
atattatagt ttgtttattt atttaattgt ggataaatat ttgagttgtt tttaattttt 7860
ttggtattat aaatagagtt attatgaaaa tgttgtataa gtttttgtat gaatatgtgt 7920
ttttattttt tttgggtaaa tatttagtag tgaaatggtt ggataaatta gtaggtgttg 7980
tgatatgagt gtttgtgttt tttttaaatt tgtgtgttaa aatttaatta ttaatgtgat 8040
agttttagta ggaggaggtg ttggggagtg attaggttat ggagt 8085

<210> 209
<211> 7110
<212> DNA
<213> Artificial Sequence

<220>
<223> chemically treated genomic DNA (Homo sapiens)

<400> 209

tgttttttgt gatgttttag aggttagaag gatgttttga aggggaaaat tgtaggagtt 60
aggtagagta gggttttggt ttttttttaa tgttttttta tttagataaa ttcgggttat 120
tttgtagagg ttgtagtgag ttatgattac gttattgtat tttagtttga ggaatagagg 180
gagatttcgt tttaaaaaaa aaaaaaaaaa aaaaaaaaaa aaaaaaaaaa atttcggttt 240
attttatgaa gtatttggta ggtgttttaa tattttttgg tatatgggaa ttattttttt 300
ttattattgg aaggtttaaa aagtaaataa tgtgttttat atggttagag ttagtattaa 360
ggggattaag tagaataatt ggtagttagt gttagtttgg agattaatat agttaaagtt 420
gttttgttat gttatgtttt ttttatttgt attgttttac ggtttaagat aaaatttttg 480
aacgttattt ggatatagtg agtatttggt ttgttttatt gtttttagga gtaattaatt 540
taatttttat ttttttgaga atgatgattt tatatagtat atttttttat taagatgtga 600
aagatgatat tatggtattt gaaatagttt taggagagat ttgggatatg ggaagtttgt 660
agataataat ggaaaatttt tttttagaat atagttattt gtatgattta tagtagcgtt 720
ttttggagaa tgttttaaaa ttatttttat tgaaagaata ataatgtttg ttattaggat 780
aaatgaaata aggggaaaat tttagatttt tggaataatg ggtttatatt taatttaatg 840
attattatgt ttttatattt tgtattattt agaaaatagt agttaaatag atagaaatat 900
agaagattgt ttaaaaatta aagttattga gttagatttt tttttgaaag gttagcgtat 960
gggagatgag aaaggtatta tagagattag agtgtttata taaaagatat tttagtagat 1020
gatttataaa gagtatatta agtatttata ttatttatat taagttgttg gttatttcgt 1080
ttattaaaga aaataagaaa gaaatttttt ttcgatatta ttttggtgta gtaataatag 1140
agttttggaa ttagttgtta gaagcgataa ttaaagttag tttattatgt ttattaatta 1200
atttatatag ttattttgaa gttttatata attgtttttt tgttataagt ttttattttt 1260
ttttatggta ttttggtttt gaatttagat aggtaaagaa ttaagtatgg tcgggcgcgg 1320
tggtttacgt ttgtaatttt agtattttgg gaggtcgtgg cgggtagatt acgaggttag 1380
gagatcgaga ttattttgat taatatggtg aaattttgtt tttattaaat tataaaaaat 1440
tagttcggta tgatggtgtg cgtttgtagt tttagttatt cgggaggttg aggtagggga 1500
attgtttgaa tttgggaggt agaggttgta gtgaatcgag attatgttat tgtattttag 1560
tttgtgagat ttcgttttaa aaaaaaaaaa aaaaaaagaa aaaaaaaaaa agaattgaag 1620
tgtttggaga ttagtgtatt gtaatattta tatagttaag taggtatttt taattgaatt 1680
ttaaaaaagt attatttttg aaattttttt tagtttagat agggttagtt atttgggtag 1740
aaaggaaaag aaaaataaaa aattggaaaa ggatgtatta gtattttttt tagaaataat 1800
ttaatattag gaatagaagg aggattttat ttattaattt ttaataatta tttgatgttg 1860
ttgttgggtt ggttaaatat ttgttaatat tgatgatttt tttatttatt attagagaga 1920
agtagaggga tttgatgttt agagaagtag taaaatttat tttttatttt ttttttttag 1980

tcggatggaa gtatttggag gttggtttta tattttagta ttttagtgtt ggtgatgtag 2040
attatttttg ttttagagta attttgtgtt atggaattta agaggatttt gtcgtattag 2100
gataggaatg tatttttgtt ttatttgttt ttatgaaagt agattttaga gatttttaga 2160
tgttatgaga ttaattgtta tttagggtag gttttagggg agggatagag aagggtagtg 2220
ttaataatag ggattttgga gataagatta gtaatttaag aaataaatgt gagatatttt 2280
attttttttt aatttatttt ttttttaaaa gtttattttt attttttttt tttttattac 2340
gtagtaattg ataattaaat atagttttgg tttttattat gtaatttagt agaatattta 2400
gatattagga attttttatt tttggttatt tatataataa tgaaatttgt agtttacgtt 2460
aggttgtgtg gatagtattt gtatttttgg aaattaattg gaggtttttt ttttattttt 2520
tttcggttgt tttttatttt tttttttatt gtggtttttg ttttttttaa gttgattaga 2580
attatgtata aaattagttt ttaataaata ttaatattga agttttagta ggttgttaat 2640
ttttgtatag ttattttaaa tgttgtatat aagtgtaaag ttttatggag ttttagaggt 2700
ttttaagaat attgtaaata ttttaggttt aattagtagt ttcggagtta ggtaatggtt 2760
tgtggttttt tgcgttaggt tttttgaggt ttttagtttt tagaggttgt ttgtaattat 2820
gatgtgtata tatatagtat ttttttttaa atatagtata aatttttttt agattttgtt 2880
aggtatttat aattattgaa tatatatgta gattgatttt tattttttta aagtttgaaa 2940
aataatttag tgttttaatt taggtttcgt tagatatttt ttgatttaaa ttgtcgtttg 3000
tagttttatt ttttaaggga aatgaaaaaa taataaattt ttagattggt gttgatgttt 3060
atttttttta agcgggtcga ttatttgttt tgtagatttt tgacgggtgg ggtgcggggt 3120
aggagtgcga tttaattttt agtattttcg aattagtttt tttacggtga taggttagtt 3180
taatcggggt tgtaaataga tttgataggt ttgttttggg ttgacggtta ttgattaggt 3240
ttttagatta gataagttat ttggttgagt ttatagtagg tggggcgcgt ttattagttt 3300
aggggtagtg attggacgtt tgttgtaata tcggagaatg tacgttttgg gttgtagtag 3360
gagatatttt taagtataga attaaaaggg tttattttaa gcggtagggt attagcgatg 3420
gagaggttcg agagttttag cgtttagttt ttttttttac gtttgggaag gcgtagaata 3480
ggtcgatgta gagtaaggag tgagttttag gttttagttt tttggtttgt ttttagggta 3540
gtaggcgagg agtggtatta gtttggggat ttttttttcg cgttttgtaa gaatcggcgg 3600
tagttagtag gcggggaggc gggggtacgt gtttggatgt gggtgtttgt gtaattagtt 3660
ttttaagcgt tagtttcgat agcgtttttt cgggaggttg gttcgagttt ttgttttcgt 3720
cgcggcgtag gaagggttgg ggtttcgttg tttgtattag gtaagagtat ttcgagtaaa 3780
ggaagaagac gatttgtttt cggagttatt attggggagt gggaatttgg aaagtttttt 3840
aattagggat atacgtgatt tttttcggaa agtagtttcg attgtggttc gtgtattttt 3900
ttattttttt ttgaattttt ttaggttttt tcgtttcgtt tattcgttgg gttgtagttt 3960
tttatcgttt cgtatttttt atttaattcg gtaattttaa acgtgtacgg ttcggtcggg 4020
gcgcgcggag tttggtttcg ggcgatttat tttgtcgggt ttttacggcg gttaaggggg 4080
ggcggggtta ggtggttttt gagaatcgag tttgatttcg acgtcgcgaa tcgatttgga 4140
gttcgagggg aaagatgttc gatttttttg ggggtatcgg agcgggcgta ggagaggttt 4200
gcggggtgcg ttttatttat agggattttt ttttagttta tttagatagg tgttttttgg 4260
gtttttgaaa tttaacggtt atgtgtttac gtttagtacg ttcggttgag agtttttatt 4320
tttagggtaa acgagtcgag ttatcgggga agcgagaggt ggggcgttgt aagggagtcg 4380
gatgaggtga tatacgttgg cgatataata gtaggttgtt ttttgtgtta agattgatat 4440
tatgaggata tagatttggg ggaaggggga atttttaggt aaaggttgtt atagttaaat 4500
ttttgcgaac gattgtgatt cgatagcggt gtaaaaggaa agagcgaatg tagtttacgt 4560
cgcggaaatt taggggtaga ggtaaggggg gagggtattt tttttgtagg gatcgttttt 4620
gtattttttt ttatattgag tagcgtggtt atttggtttt ttttatttgt gtataggtaa 4680
ttttagattc gagttagtga tattgtttaa cgtatttatt ttagttttta ttattagttt 4740
tttattttcg ttttataata gtttattttg ttttcggttg ggtttttggg tagaggtcga 4800
ggtttagttc gttattttcg tttcgcgttg ttgtaaaagt cgtagtaagt gtagttgtag 4860
gttggcggtt gggaatcggt tcgagtaagt tttaggtagt tatattgggt atgtttagta 4920
gagtttgcgg tttggggatt ttgcgttcgt atttagagtt atcgttttgt ttttttttat 4980
cgttttttgt tttgttttgt ttttttttcg ttcggcgcgg tttcgttcgt ttttcgttcg 5040
tttttcgttt tttttcggtt tttcgaggcg ttcgggtttt cggcgcggcg gcggaggggg 5100
cgggtaggtc ggcgggcggt gatgtggcgg gattttttat gcgttgcggt aggatacgcg 5160
ttcggcgttg ggacgcgatt gcgtttagtt tttttttttc ggaagttgta gttatgatgg 5220
aagtttgaga gttgagtcgt tgtgaggcga ggtcgggttt aggcgaggga gatgagagac 5280
ggcggcggtc gcggttcgga gtttttttta gcgtttgtga gtagtcgcgg gggtagcgtt 5340
ttcggggagt cggtcggttt gcggcggcgg tagcggcggc gtttttcgtt tttttttcgt 5400
tttttttaat cgtgtagttt ttttttcggt tttttttgaa agggaaggtg gaagtcgtgg 5460
gttcgggcgg gagtcggttg aggcgcggcg gcggcggcgg tatttttcgt ttttggagcg 5520
ggggggagaa gcggcggcgg cggcggtcgc ggcggttgta gttttaggga gggggtttga 5580
gtcgtttgtt attattttta gggttgggaa cgtcggagag ttggtttttt tttttttatt 5640
gtttttaata cggcggcggc ggcggcggta tatttaggga ttcgggtcgg ttttaaattt 5700
ttcgttcgtc gtcgtcgtat ttttcgtggt tcgggtttcg gaggtcgtcg gcggaggtag 5760

tcgttcggag gattattcgt ttttttttta tttcgttgtc gtcgttgtta ggtttttggt 5820
tgttgaggag aagtaggttt agtcgttgta attatttagt agtcgtcgta gtagttatta 5880
ttcggttgcg gtttagagtt aagcggcggt agagcgaggg gtattagtta tcgttaagtt 5940
tagagttatt tttattttgt agaagaagtt tcgttattag tagtttttgt tatttttttt 6000
tttttttttt tttagttata ggtttttaga tatgatagtt attattaaag agatcgttag 6060
tagaaataaa aggagatatt aagaggatgg attcgattta gatttgattt gtatttattt 6120
ttgcggttgt ttttttttat ttttttgtta tttttttaga acgtgggagt agacggatgc 6180
gaaaatgttc gtagtttggg tgattataat atttaatttt ggttaggttg ttaggttata 6240
tattttgtgt tttttttttg tgtatttaat ttagggtgtg tttggttaga cggaattttt 6300
gtttggttgt aagtgttaag ttatcgattg tttttttagg ttatttatat ggtttttttt 6360
tgagggttat tgttcgttaa tatagaatat agtatattgt tagtggatta gcgagttcgg 6420
taattcggtt ttttaaatga ataaaaaagt agacgttttt tgaggttgag tatatttcga 6480
ttaaattttg gtttaggttt tagattaagg gtttagatta gaataaaatg aaaattagtg 6540
ttgtacgtac gtatattgta ttagaatttt gtagtgattg ttttagtttt ttgagttgta 6600
ttgatagatt tttttaaaat atgattgatt tgtataattt tagaagtaga attattttta 6660
gtatatatgg tgtatattga gggtaaaaag tagttttgtt aatgtttaaa tttaagttat 6720
ttataatttt gaattgtatg tagaagtttt gtagtttgaa gttaatagtg ttataatata 6780
ttttataagg cgtttttatt agatttttgt tatatttatt tttttttttt tttatggggt 6840
gatgtaggat agtgtttgaa atttgtattt tagtagtatt taatgtttag tgtttttgtt 6900
ataaatatag aatggatatt gagtagtttt tgattttaga tggtaatgtg taggtttaag 6960
ggtattgtgt gtagtaagtg aagattgtag aaataaaatt ttagtttatg tttgaaattt 7020
aagtattgtt gtgatgttag aattgttgtt tatcgttttt aggttttagg ttttttgata 7080
ttttttggta tcgttaattt tattgatttg 7110

<210> 210
<211> 7110
<212> DNA
<213> Artificial Sequence

<220>
<223> chemically treated genomic DNA (Homo sapiens)

<400> 210

taaattagta aaattaacga tattaaaagg tgttagagga tttgaaattt aaaaacggtg 60
agtagtaatt ttggtattat aataatattt aaattttaag tatgaattga agttttattt 120
ttgtaatttt tatttgttat atataatatt tttgaattta tatattatta tttgggatta 180
gaaattattt aatatttatt ttatgtttat gataagagta ttgaatatta aatgttattg 240
aagtgtaaat tttaagtatt attttatatt attttatagg gagagaaaaa ggtaaatata 300
ataaagattt agtaagaacg ttttataagg tatattatgg tattattgat tttaaattat 360
aaaattttta tatatagttt aaagttgtag gtaatttaag tttaaatatt aataaaatta 420
tttttttgttt ttaatgtgta ttatatatat tgaaaatgat tttgttttta aagttatgta 480
aattagttat attttaaaag aatttattaa tgtaatttag gaaattaaaa taattattgt 540
aagattttga tgtaatatgc gtacgtgtaa tattaatttt tattttattt tgatttaaat 600
ttttgattta gggtttaagt taagatttaa tcgaaatatg tttaatttta aaaagcgttt 660
atttttttgt ttatttagga gatcggatta tcgagttcgt taatttatta atagtgtatt 720
gtatttgtta ttaacggata atagttttta ggaagagatt atatagatag tttaagaaag 780
taatcggtgg tttgatattt gtaattaggt aagagtttcg tttagttaaa tatattttag 840
gttgaatata tagaaaggaa atataaaata tatgatttag taatttgatt agggttaaat 900
gttatagtta tttaaattac ggatattttc gtattcgttt atttttacgt tttaagagag 960
tgatagaaag gtaaagagga gtagtcgtag aaatggatat aggttaagtt taagtcgaat 1020
ttattttttt gatatttttt tttgtttttg ttaacgattt ttttgatgat ggttgttatg 1080
tttgggagtt tgtggttgaa gaaaaaggag gagagagatg gtagaagttg ttggtggcgg 1140
ggtttttttt gtaggatgga aatggttttg gatttggcgg tagttgatgt ttttcgtttt 1200
gtcgtcgttt ggttttggat cgtagtcggg taatggttgt tgcggcggtt gttggatggt 1260
tgtagcgatt gggtttgttt ttttttagta gttagaggtt tggtagcggc ggtagcggaa 1320
tggggagaag acgaataatt tttcgaacgg ttgttttcgt cggcggtttt cggagttcgg 1380
gttacggggg gtgcggcggc ggcggacggg aggtttaaaa tcggttcggg tttttggatg 1440
tgtcgtcgtc gtcgtcgtcg tgttggaggt agtagaaggg gagagattaa tttttcggcg 1500
tttttagttt tggaaatggt gataggcgat ttagattttt tttttggagt tgtagtcgtc 1560
gcggtcgtcg tcgtcgtcgt tttttttttt cgttttagga gcgggaggtg tcgtcgtcgt 1620
cgtcgcgttt tagtcggttt tcgttcgagt ttacggtttt tatttttttt tttaggagaa 1680
gtcgaggaag aggttgtacg gttagaaaag acgaagagga ggcgagaaac gtcgtcgttg 1740

tcgtcgtcgt aggtcggtcg gtttttcgag ggcgttgttt tcgcggttgt ttataggcgt 1800
tgagaggggt ttcgggtcgc ggtcgtcgtc gttttttatt tttttcgttt gagttcggtt 1860
tcgttttata gcggtttaat ttttaaattt ttattatggt tgtagttttc gagaggagag 1920
aattgagcgt agtcgcgttt tagcgtcgag cgcgtatttt gtcgtagcgt ataaagagtt 1980
tcgttatatt atcgttcgtc ggtttgttcg tttttttcgt cgtcgcgtcg ggagttcggg 2040
cgtttcggaa gatcgagggg aggcgggagg cgagcgagag gcggacggga tcgcgtcggg 2100
cgaggggagg gtagggtagg gtagggggcg gtaggagggg gtagagcggt agttttgggt 2160
gcgagcgtag agtttttaag tcgtaggttt tattgagtat gtttagtgta gttgtttggg 2220
gtttgttcgg gtcggttttt agtcgttagt ttgtagttgt atttgttgcg gtttttgtag 2280
taacgcgagg cgaggataac gagttaagtt tcggtttttg tttagaaatt tagtcggagg 2340
tagggtaggt tgttgtgggg cgggggtgga ggattgatga tgaaagttga gatgggtgcg 2400
ttgagtagtg ttattgattc gagtttgagg ttatttgtgt ataggtgaaa aggattaggt 2460
gattacgttg tttagtgtag agggaaatgt agggacggtt tttgtaaggg gaatattttt 2520
tttttttgtt tttattttta gattttcgcg gcgtggattg tattcgtttt ttttttttgt 2580
atcgttgtcg gattataatc gttcgtagag atttgattgt aatagttttt gtttagagat 2640
tttttttttt tttaaatttg tgtttttatg gtgttagttt tagtataaag agtaatttgt 2700
tattgtgtcg ttagcgtgta ttattttatt cggttttttt gtagcgtttt atttttcgtt 2760
ttttcggtaa ttcggttcgt ttgttttaaa aatgaaagtt tttagtcgag cgtgttgaac 2820
gtgaatatat agtcgttgaa ttttaagggt ttaaagggta tttatttaaa tgaattgaaa 2880
gaggattttt gtgagtggga cgtatttcgt aggttttttt tgcgttcgtt tcggtgtttt 2940
taagagagtc gagtattttt tttttcgggt tttaggtcgg ttcgcggcgt cggagttaag 3000
ttcggttttt agagattatt tagtttcgtt tttttttggt cgtcgtgaaa attcggtagg 3060
atggatcgtt cggggttagg tttcgcgcgt ttcggtcgga tcgtgtacgt ttggggttat 3120
cgggttgagt ggaaagtacg gaacggtagg aagttgtagt ttagcgagtg ggcggggcga 3180
ggagatttag gagggtttaa aaggaggtgg aaggatatac gggttatagt cggaattatt 3240
tttcgaagga ggttacgtgt gtttttagtt ggggaatttt ttaaattttt attttttagt 3300
gatagtttcg gaggtaagtc gttttttttt tttgttcggg gtgtttttgt ttggtgtagg 3360
tagcggaatt tttaattttt ttgcgtcgcg gcggaaatag gggttcggat tagtttttcg 3420
aaggagcgtt gtcggggttg gcgtttgggg aattggttat ataagtattt atatttaaat 3480
acgtgttttc gttttttcgt ttgttggttg tcgtcgattt ttatagaacg cggggagaga 3540
gtttttaaat tggtgttatt tttcgtttgt tattttaaga gtaagttaaa ggattgagat 3600
ttgagattta ttttttgttt tatatcgatt tattttgcgt ttttttaaac gtgggaaaag 3660
gagttgggcg ttagggtttt cgggtttttt tatcgttgat gttttgtcgt ttagggtgaa 3720
tttttttggt tttgtgtttg agggtatttt ttgttgtagt ttagagcgtg tatttttcga 3780
tgttgtaata aacgtttagt tattattttt gagttggtga gcgcgtttta tttattgtgg 3840
atttagttaa gtgatttatt tggtttgaga atttagttaa tggtcgttag tttagaataa 3900
atttgttaag tttgtttata gtttcgattg ggttgatttg ttatcgtgag agagttgatt 3960
cggaaatgtt gagagttgga tcgtattttt atttcgtatt ttattcgtta aggatttata 4020
aagtaagtag tcgattcgtt taaagagaat gagtattaat attagtttgg gaatttattg 4080
tttttttatt ttttttagga agtagaatta tagacgataa tttgagttaa aaaatattta 4140
acgagattta ggttgaagta ttgagttgtt ttttaaattt tgagagaatg agaattaatt 4200
tgtatgtgtg tttaataatt gtaagtgttt agtaaagttt aaagagggtt tatgttgtat 4260
ttgagaaaga gtattgtata tgtgtatatt atagttgtag gtaatttttg aagattagaa 4320
attttaagag atttagcgta gaaagttata ggttattatt taatttcggg attattaatt 4380
aaatttggga tatttatagt gtttttagag atttttgagg ttttataaaa ttttgtattt 4440
atatatagta tttaagatag ttgtgtagga gttaataatt tgttgaagtt ttagtgttga 4500
tatttattga agattggttt tgtgtatagt tttggttagt ttgggaaagg taaggattat 4560
agtggaaaag gaaatggaaa gtaatcggaa aagagtaaga gaaaggtttt tagttgattt 4620
ttagagatgt agatattgtt tatatagttt agcgtgaatt atagatttta ttattgtata 4680
aataattaaa gataaggagt tttgatgttt taggtgtttt attagattgt atggtaaaaa 4740
ttagagttgt gtttaattat taattattgc gtggtagaaa aagggaaaat gaagatgaat 4800
ttttaaaagg ggagtagatt agggaggggt aaaatgtttt atatttgttt tttagattgt 4860
tgattttgtt tttagagttt ttgttattag tattgttttt ttttgttttt tttttggagt 4920
ttattttaag tgataattag ttttatagta tttgagaatt tttgaaattt gtttttatgg 4980
aagtaaatga aataagggtg tatttttgtt ttggtgcgat agaatttttt tggattttat 5040
ggtatagagt tattttggag tagagataat ttgtattatt agtattaaag tgttagaata 5100
tgagattagt ttttaagtat ttttattcgg ttgaagaaag aaaatgggaa atgggtttta 5160
ttgttttttt aaatattagg tttttttatt tttttttagt ggtgaatgaa aggattatta 5220
atgttggtag atatttggtt agtttaatag taatattagg tggttattag gagttggtga 5280
ataaaatttt ttttttgttt ttagtgttaa gttgtttttg aaagaaatat tgatgtattt 5340
ttttttaatt ttttgttttt tttttttttt ttatttaaat ggttggtttt atttaaatta 5400
aagaaagttt taaaaatagt atttttttaa agtttagttg aaaatatttg tttggttgta 5460
tagatattat aatatattgg tttttaagta ttttagtttt tttttttttt tttttttttt 5520

tttttttttt ttgagacgga gttttatagg ttggagtgta gtggtatgat ttcggtttat 5580
tgtaattttt gttttttagg tttaagtaat ttttttgttt tagtttttcg agtagttggg 5640
attataggcg tatattatta tgtcgggtta attttttgta gtttagtaaa gatagggttt 5700
tattatgttg gttaggatgg tttcgatttt ttgatttcgt gatttgttcg ttacggtttt 5760
ttaaagtgtt gggattatag gcgtgagtta tcgcgttcgg ttatatttag ttttttattt 5820
gtttggattt agaattagaa tattataggg agaagtagaa gtttgtaata aagggataat 5880
tatataaagt tttagggtga ttatgtagat tgattagtaa gtatggtaaa ttagttttaa 5940
ttatcgtttt taatagttga ttttaaaatt ttattgttat tatattaaaa taatgtcgaa 6000
agaaaatttt ttttttattt tttttgataa gcgaaatgat tagtaatttg atgtggatga 6060
tataaatatt tgatgtgttt tttgtaggtt atttgttaag atgttttttg tgtaaatatt 6120
ttgattttta tagtgttttt tttatttttt atacgttgat tttttaaaaa gggatttaat 6180
ttaatagttt taatttttaa ataatttttt gtatttttgt ttgtttaatt attgtttttt 6240
aaataatata gaatataaaa atataataat tattggattg aatgtaaatt tattgtttta 6300
agggtttgag gttttttttt tattttattt attttaataa taaatattat tgttttttta 6360
ataaagataa ttttaagata ttttttaaaa ggcgttattg tgggttatat aagtaattat 6420
attttaaaag agaatttttt attattgttt ataagttttt tatgttttaa attttttttg 6480
aagttatttt agatgttatg gtgttatttt ttatattttg gtagagagat gtgttgtatg 6540
aaattattat ttttagagaa ataagggttg agttggttgt ttttgagaat aatagagtag 6600
attaggtatt tattgtgttt aagtggcgtt tagaaatttt gttttggatc gtggagtaat 6660
ataaataaaa aagatataat atgataagat agttttgatt atgttagttt ttaggttaat 6720
attagttatt aattatttta tttggttttt ttaatattaa ttttagttat atggagtata 6780
ttgtttgttt tttagatttt ttaataatag aaaaaggtaa tttttatata ttagaaaata 6840
ttgggatatt tattaggtgt tttatagagt aggtcgaaat tttttttttt tttttttttt 6900
tttttttttt tttttttgag acggagtttt tttttgtttt ttaggttgga gtgtagtgac 6960
gtgattatgg tttattgtaa tttttgtaga atagttcgaa tttgtttagg tagagggata 7020
ttgagaagga gttaaaattt tgttttgttt aatttttata attttttttt ttaaaatatt 7080
tttttggttt ttaaggtatt ataaaaaata 7110

<210> 211
<211> 6478
<212> DNA
<213> Artificial Sequence

<220>
<223> chemically treated genomic DNA (Homo sapiens)

<400> 211
   tttattttga agggatgttt aagaaatatt gttaaattta aaagtgttgt agagtaatat 60
   gtattataag attatattgg tataatagta ataaatagta tttttttata taattttaaa 120
   aaaatatata gatttatgtt aagtattagt taagatgttt ggtatatggt aggtatttaa 180
   tatttaatga atatatgtag aggattttta ttttttattt tatatttttt ggataagatt 240
   ttgaattttt atgagtatgt attatttaaa tttagattta tagggagtga tggaaaatgt 300
   gaaaataaaa ttttaaaaag gaaaagaaag agtaaagatt tagatttaaa aaaggaaatg 360
   taaataagat gaaatagtta aagataggtt tttttaaaat taatgttgag ataatgttat 420
   tagtagtgat agttagttaa gagatggttc gtagtttatt gtttagtatt gaggtattta 480
   gtaggggttg tatttaattt ttaagttgga attaatcgaa attgttgggt ttgaagagtt 540
   gtttttatga gttatcgacg gtagaaagta tagaagtatt aaagaaaata atttagaaat 600
   gtggaaagag aaaagtatgt ttttgttttg ggagaataat atttgtgtgt aattaagtag 660
   gaagattttg tggaggaaag tttgaaaaag tagagagttg aagagaaaag agttaaatga 720
   aggatacggt ggttaatatt taaagtaaaa agatattttt gaagaagatg tgtttttagg 780
   gtttttaaga agtcgttata tttatgttta gagttatttt gtaagtttat ttggaaagtg 840
   tattggtgtt ttttggtata agatttacga atattaatag ttttgttatg tgagtaggtg 900
   tttatattga agggttataa gtaaagtttt tatgtgttat tattgttaga atttttttcg 960
   ttttttttta atataaaagt agttttgttt gttagtatgg aagttttggg aagagtttta 1020
   tatttgtttc gtgttgttaa tatttttgtt ttggtttttt tgttggaata tataagttat 1080
   ttagtattag ttatttattt ttttggtaga attgtggtaa gaggggatta atagggaaaa 1140
   aaaattaaaa aataaataaa aagttaaaat aatatgaaag agttgggtat gtttttaaaa 1200
   taattagtgt ttattttttt tagttttgtg tttgtgtttg tttgtatttt gtatgtttgt 1260
   ttttgtggtg gggggggata tgtttgcgtt tttgaaaatt tttttattta gatattataa 1320
   ggtattagtt taattttgaa tatagtcggt tgtagtacgg tgtatagata cgattgtttt 1380
   ttttttagaa aattttttag tttaaatatg tttgtagagt atgatttatg gtgttttttt 1440
   ttggattgtt tagtgggtaa ttgttgtacg aaaattagag aaatagatgt agattttgaa 1500
   ttttggttat aaatttttgt tttatgatta tgggttttaa atgtgttgtg ttatattgat 1560
   aaatatatag ttattttgaa ataaaatgga ttttattttt aaagttttgt tttttgtttt 1620
   ttattttttg tttgtttgtt ggttggaaat atatttatta gattattgat taaatataaa 1680
   gaaaatagag atgatatata ttagaatgtt attttatgtt ataataagaa gatatttggg 1740
   aaagtttttt ttaattttgt ggttaatgtt taaaatgggt gttagtagtt tatattttat 1800
   tacggataaa tcggtttatt ttttattttt tattttattt attaattttt atagtgtacg 1860
   gatttgtatt taaagaatta tggtttattt atgtattttt tttttatttt atttaaaatg 1920
   aagaatttgg agatagaatt aagtttttaa gaagtgtaag tatttattga tgaagaaata 1980
   attagtatgt ggttaaatta tgggtttgtt ttataaatta atatttggat taagtaatag 2040
   ttttaattat tgttattagt ataattaatt tatgatgagg aatgattaat tgattagcga 2100
   atattgttat attataagaa atttatatta ttttttttat ggggatttaa atattgatta 2160
   tttattttaa ggtttaagtt tattttttag ttgagaattt taaggtaatt atatatttta 2220
   tatttttttg tataaagaaa gaaaatatcg agtcgtttaa tttttattat atatagagag 2280
   agatatatta atgttgttat ttttgtgtaa tgatatagta tagaggtaag aaattcggaa 2340
   gtagtggttt cgtttaatga ggttgatttt atagtttaag aaatgagaat gaatagaatt 2400
   ttgaggatat ttatttttat ttttattttt ttgggattag tgtattttta ttttttggag 2460
   agatgttaag taagaatgtg tagtagtttt ttttggataa tagatttttg tatatatata 2520
   tttgttagtg atgttatgtt ttttattttt aatttaagtt ttttttgttt tatttttaat 2580
   atagttgttt tttttttttt tgtttaataa ttttttattt atttttattg ttcggtaaaa 2640
   atgaatttag tttttaagga attttaagga ttttaaataa gtatttggag ttataagttt 2700
   tatggattaa gtagaaattg gtatataatg tagtgttttg tgtattttta taattatttt 2760
   aatgatagtg tgttaaatgt tttgtaaatt tttttgtgtt tttttgttta aaatgtgtgt 2820
   tttttttgta gttaaagggt tttttataat aagtttttgt tataaaatga tggttatata 2880
   aaagagagaa gagaggcgta aaaataataa atatagattt agtaaaaatt ttattttttt 2940
   attgttttga attttttaat agtaagaatt gagtttgttg ggttgaaaat tgttttaatg 3000
   tatttttata aaaggatttt tgatttggta agtgtgtgcg atgtatattt tttatgttat 3060
   attataagtg ttatttagta attttattag atagggtagt gttttagtat ggggtggggt 3120
   gttttttgat aggtttttaa aaggttcgga tgttaatgta tattttaata ttatttataa 3180
   aaaggagatt ggagtagtgt ttttttttgt attgggtaag gagatttttt ttttttgttt 3240
   aattatttgt ttgttttgtt ttgtgggaga attataagta aatgttatag aggtagtgga 3300
   gaaaaaaggg tgttttaatt tttttttaga gtttttttta tttgatgtat gttgtatttt 3360
   ttaaataagt tgtgtaaaat ggttgtaggg tagattggtt ttttttttta aagtttttta 3420
   ttcggttggg tttatttgta aatattgtat ttattttttt tagtgtttta ggattggttg 3480
   gaaagatttt tttttttgta ggttgggtta gtgtgagaga tttaaaaaat tatttttttt 3540
   taaaattatt gtattttaat aaaaggattg ggtaggggtt ggaatgagag aaaattggtt 3600
   ttttaaaatg taaaattgtt atatttaaat tagtttataa aatatgcgtt taattatgtg 3660
   gtgggatgtg tgtaggtgta tgatgagaga ggtaattaat atggttattt ggggtgtaag 3720
   gatgtgggaa taggtaagta atttttatat tggattttta ttttagggag ttgggtttta 3780
   gttatagttt tgagttgtgt gattttgggt aggttttatt ttttcggggt tttgttttat 3840
   tagttgaata gtatgaggat gagttatagt taatatttgt tttatgatat ttatttagta 3900
   ttatataagt gttatttttg tttttttagt taatattgac gtgggtagta ttatatgttt 3960
   attttataga tgaggaaatt gaagtttgaa gaagttaaat atttatttta gaatatatag 4020
   ttggtaagtg gtagatttgg aattggaatt tagtttagtt tgatttttta atttatgttt 4080
   ttgattatta tattgttttt ttaagtttag atttgaaatt ttattttttg tgtggttgtg 4140
   tgtttgggat aggggtaatt aatttttgat tattttatat gttgtataga atttggagag 4200
   gattttttaa agtaaatgaa tttcgaaagt tggattgtag agtaaacgag tgtagttaat 4260
   ttagttaggg gtttgtaaga gggagaaaga gaaaaagatt gtggaatgga aagtttttta 4320
   atttaagttt tttttaaggg gtagttattt tttgttttat agtttagggt ttgtatgtgt 4380
   tttttttgga gtggaaaaat atataagtta taaggaattt aatagataga aaggcgtata 4440
   gaggaattta aagtgtgggt tggggggcga ggcggtgggc gggaggcgag cgggcgtagg 4500
   cggaatatcg ttttttaagt taagtcgtcg taaataaaaa ggcgtaaagg gagagaagtt 4560
   ggtgtttaac gtgagttagg agtagcgttt cggttttttt tttgtttatt ttaaaagtat 4620
   tttttgtatt gtttttaagg tgagaaatag gaaagaaaac gtcggtttgt gcgttcgttg 4680
   tttgtttttt tggttgtttg tttttgtagg gttgttggga gtttttaagt tttgtgagaa 4740
   ttttgggagt tggtgatgtt agattagttg ggttatttga aggttagtag ttcgggtagg 4800
   gtttatcgaa agtttattcg tatatattag gtaatttaat tttttatttt gtgtgataga 4860
   agtagtagga agtgagttgt ttagaggtag gagggtttat tttttgttaa aggggggatt 4920
   agaatttttt tatgcgagtt gtttgaggat tgggatgtcg agaacgcgag cgattcgagt 4980
   agggtttgtt tgggtatcgt cggggtagga ttcggaacgt attcggaagg ttttttgtaa 5040
   gtatttattt ggaaggagaa tttgggattt ttttgggaat ttttcgtttc ggttggattg 5100
   gtcgagtaag tttggaaaat ggtaaatgat tatttggatt aattataggt ttttagttgg 5160
   tttgtttgtt ataatttatg attcggggtt gggaaaaaga ttaatagttt acgtgttaaa 5220
   aaaggggtag agtttgatgg agttgggtgg atttttttat gttatttgtt tttatattta 5280
   gaggataagt atttttgtag atatttagtg taagggagat tatgtttgat tgtatggatg 5340
   ttttgttagt gagttttggg taaattttgg atttttatat tgcgagttcg tttttttgta 5400
   tgttttagga gaaagttttt aaagtatgtt ttagtggatt gatttaaatc gaatggtagt 5460
   atcggtatat tgtttaatgt aggtttattt tttttttttt ttttattaag aaaaaaaaaa 5520
   ttgttttttt tgtatgtaat aaagacgttg gaaataaatt gtattggtag taagataaag 5580
   gatttaatga tttaatgttg aaggggtgtg atatgttggt atgtatattg attttttttg 5640
   ttgaaaattt tttgttagat gttttttttt taaataattt ttttcgtttt tatgtttttt 5700
   tttttattaa ttttatagtt ttttgaaaat agtttttaaa atatgtataa aaatgtgggg 5760
   ggtggggagt agagggtgat tataagattt taaaatattt tttaagtagg gtaatttgat 5820
   atattttgta gtttttatta aaatgtatgt agagtggaat ttaattttgt tgttttttgg 5880
   attagttatt agttgtatag gtttttttat tttttgtgta ttttaatagt ttaaagtaat 5940
   taaaatatga tatttttttt gaaaggaatt tagtttaatt aaggtggttg ttgttttcga 6000
   agtttataaa ttattagttt aagttgtcgt ttttaatgtt ttttttgtta aggaaaggta 6060
   atatttttta gtattgattt tgttgttatt tttttaagtt attttgttgg tttaagaagt 6120
   tgttgttttg ttagaaaatt attggtaatg aatatttttt ggattgagtt atttattatt 6180
   tttatatttt gtagtttagt tcggaacgtg gatattagtt aaaggaaagg aaatataagt 6240
   ggggtagttt aatttaaaaa aaaaatattg attattttgt tatttgatgt tgatatagtg 6300
   tcgaggtacg tatttggtta atagtagttt ggttgtaggt ttgagaaatg attaggagtt 6360
   ttagaaaatt ttattttcgg gtggttgtag tgggagtttg taggatgttt cgtcgttttg 6420
   atagtgattt agataaatgg tttagttatt tattaaaatg tttttagtat tttatttg 6478

<210> 212
<211> 6478
<212> DNA
<213> Artificial Sequence

<220>
<223> chemically treated genomic DNA (Homo sapiens)

<400> 212

taaataaagt attaaaaata ttttgataga tagttgagtt atttatttaa attattgtta 60
gggcggcgaa gtattttgta ggtttttatt atagttattc gaaggtggga ttttttggga 120
tttttgatta ttttttaagt ttgtagttag gttattgttg attaggtgcg tatttcggta 180
ttgtgttagt attagataat aaaatagtta atattttttt tttaaattgg gttattttat 240
ttatattttt ttttttttaa ttgatgttta cgtttcgggt tgaattgtaa gatatggaaa 300
taatggatgg tttagtttaa ggggtgttta ttgttagtag ttttttagta aaatagtaat 360
tttttagatt aataaagtga tttggaaagg tggtagtagg gttaatgtta agaaatattg 420
ttttttttta gtaaggaaaa tattggaagc ggtagtttga attggtggtt tatgaatttc 480
gggagtagta gttattttaa ttaaattaaa ttttttttaa ggaaaatgtt atattttaat 540
tattttggat tgttggaata tataaggggt ggggggattt atataattag tagttaattt 600
aaagaatagt aaagttaaat tttattttgt atatatttta gtaaaaattg taaaatgtgt 660
taaattgttt tatttgaaga atgttttaaa gttttgtggt tattttttat tttttatttt 720
ttatattttt atgtatattt taaaaattgt ttttaagaga ttataaggtt ggtaaggaga 780
ggagtatggg ggcgggaggg gttatttggg aagaaaatat ttaataggaa atttttagta 840
aagggaatta atatgtatgt tagtatatta tattttttta gtattaaatt attaaatttt 900
ttgttttgtt attaatgtag tttattttta acgtttttat tgtatgtaag agagataatt 960
tttttttttt tggtagaaga agggaaaaaa ataaatttat attgagtagt gtgtcgatgt 1020
tgttattcgg tttgggttaa tttattgaag tatgttttga gagttttttt ttggagtatg 1080
taggaagacg gattcgtagt gtagaaattt aggatttgtt taggatttat tgatagaata 1140
tttatatagt taaatatgat tttttttgta ttgaatgttt gtaaaagtgt ttatttttta 1200
ggtgtggagg taaatggtat agaaaagttt atttaatttt attaaatttt gttttttttt 1260
tggtacgtag gttgttggtt ttttttttag tttcgaatta tgaattatga tagataagtt 1320
agttaaaagt ttgtaattga tttaaatgat tatttattat tttttaggtt tgttcggtta 1380
atttagtcgg ggcggggggt ttttagaaag attttaagtt ttttttttaa gtaaatgttt 1440
gtaaaaagtt tttcgaatgc gtttcggatt ttatttcgac ggtgtttaga taaattttgt 1500
tcggatcgtt cgcgttttcg gtattttagt ttttaaatag ttcgtatggg ggaattttgg 1560
tttttttttt ggtaaagaat agattttttt gtttttgaat agtttatttt ttattatttt 1620
tgttatatag aatgaaagat tgaattgttt aatatatgcg agtgaatttt cggtgaattt 1680
tattcgggtt gttaattttt aaatgattta attagtttga tattattaat ttttaggatt 1740
tttatagagt ttaaaaattt ttagtagttt tgtaaaagta gatagttaga gaggtaggta 1800
gcgagcgtat aagtcggcgt tttttttttt atttttattt ttaaaaataa tataagaagt 1860
gtttttaaaa tgagtagggg aggagtcggg acgttgtttt tggtttacgt tgagtattaa 1920

tttttttttt tttacgtttt tttatttgcg gcggtttagt ttggaaaacg gtgtttcgtt 1980
tgcgttcgtt cgtttttcgt ttatcgtttc gttttttagt ttatatttta aatttttttg 2040
tgcgtttttt tgtttgttaa attttttata atttatgtat ttttttattt tagaaaaagt 2100
atatgtaagt tttaaattgt agaatagaga atggttattt tttgggaaag gtttgggttg 2160
ggaaattttt tattttatag tttttttttt tttttttttt ttgtaagttt ttggttgaat 2220
tgattgtatt cgtttgtttt gtaatttagt tttcgagatt tatttatttt gaaaaatttt 2280
ttttaggttt tatgtagtat atagagtagt taggaattgg ttatttttgt tttaaatata 2340
tagttatata gaaaatgaga ttttagattt ggatttgaaa aaatagtata gtggttaaga 2400
gtatgggttg gggaattaaa ttgaattaga ttttaatttt aggtttgtta tttattagtt 2460
gtgtgttttg ggataagtat ttaatttttt taggttttag tttttttatt tgtaaaatgg 2520
gtatataata ttatttacgt tagtgttaat tgggaggata gaaataatat ttgtatggtg 2580
ttgggtaaat attatggaat aaatgttagt tgtgatttat ttttatattg tttaattggt 2640
gaaataaaat ttcggggaga tgagatttat ttaaggttat atagtttaga gttgtgatta 2700
gaatttagtt ttttaggatg aaagtttaat gtgaaaatta tttgtttgtt tttatatttt 2760
tgtattttaa atagttatgt tggttgtttt ttttattata tatttatata tattttatta 2820
tataattaaa cgtatatttt gtaaattggt ttaagtatga tagttttata ttttgaagga 2880
ttagtttttt tttattttag tttttgttta atttttttat taaaatatag taattttaag 2940
ggaaaatgat tttttagatt ttttatattg atttaattta taggaagaag agttttttta 3000
gttagtttta aaatattaag aaggatagat gtagtattta taaataaatt tagtcggatg 3060
gagagtttta aaagggagag ttaatttatt ttgtagttat tttgtataat ttgtttaaag 3120
gatgtaatat atattaaata aaggaaattt tggagaggaa ttaaaatatt tttttttttt 3180
tattgttttt gtagtattta tttgtaattt ttttataaaa tagggtaggt aagtggttag 3240
gtagggaggg agagtttttt tgtttaatgt agggaagagt attgttttag tttttttttt 3300
gtggatagtg ttggaatgtg tattggtatt cgggtttttt aaaagtttgt tagggggtat 3360
tttattttat gttgaaatat tgttttgttt agtggagttg ttaagtggta tttgtggtgt 3420
aatatgaaaa gtatgtatcg tatatattta ttaagttaaa gattttttta tgaaggtata 3480
ttaaaataat ttttagttta gtaagtttag tttttgttat tgaaaagttt agggtaataa 3540
aggggtgaga tttttgttga atttgtgttt gttatttttg cgtttttttt ttttttttta 3600
tatggttatt attttgtaat agagatttat tgtaagaaat tttttaatta taaagaaaat 3660
atatatttta agtaagagaa tatagaggaa tttataaaat atttaatata ttgttattaa 3720
ggtagttgta aagatatata aagtattata ttatgtgtta atttttattt ggtttatgag 3780
gtttatggtt ttaaatgttt atttaaggtt tttagagttt tttggaaatt gagtttattt 3840
ttatcggata gtggaggtgg atgaaaagtt gttagataag agaagaggaa atagttatat 3900
tggaagtaga gtaggagaaa tttaagttag agatgagaaa tatggtatta ttgataggta 3960
tgtgtgtata gggatttgtt atttagggga ggttgttgta tatttttgtt taatattttt 4020
ttaaagagta gagatgtatt ggttttaggg aagtgaagat ggggatgggt gtttttaaaa 4080
ttttgtttat ttttattttt taagttatgg aattagtttt attgagcgag attattgttt 4140
tcgaattttt tatttttata ttgtattatt gtataaaaat aataatatta gtgtgttttt 4200
ttttgtgtgt ggtaaaggtt gggcgattcg atgttttttt tttttgtgta agaaaatgta 4260
aaatatataa ttattttgaa gtttttaatt gagaaatgaa tttgggtttt gaaatgaata 4320
attagtattt aaatttttat gaaaggaata gtatgggttt tttataatat gataatattc 4380
gttagttaat taattatttt ttattatgaa ttaattatgt tgatagtaat aattagagtt 4440
attatttaat ttaagtatta atttataaaa tagatttatg atttagttat atgttaatta 4500
tttttttatt agtaaatgtt tatatttttt gaaaatttga ttttgttttt aaatttttta 4560
ttttaagtaa aatgaaggaa agatatataa ataagttata attttttaaa tatagattcg 4620
tgtattataa agattaatga gtagggtaga aagtgaaaaa tagatcgatt tgttcgtaat 4680
gaaatgtaaa ttattgatat ttattttaaa tattagttat agggttggaa gaaatttttt 4740
taaatatttt tttgttatgg tataaaataa tattttgata tgtgttattt ttattttttt 4800
tgtgtttgat taatgattta ataaatgtat ttttaattaa taagtaaata aaaagtgaaa 4860
aataaaaagt aaaattttga gagtaaaatt tattttgttt tagagtaatt gtatatttgt 4920
tagtgtgata tagtatattt gaaatttata attataaaat aaaaatttat agttagggtt 4980
tagggtttat atttgttttt ttggttttcg tatagtaatt atttattgga taatttagaa 5040
agaaatatta tgaattatgt tttgtaaata tgtttgaatt ggagagtttt ttgaagaaag 5100
agtagtcgtg tttgtgtatc gtattgtagt cggttgtgtt taaaattggg ttaatatttt 5160
gtgatgttta aataagaaga tttttagagg cgtagatata ttttttttta ttataggggt 5220
agatatgtaa agtgtaggta gatataggta tagggttggg ggaggtggat attgattgtt 5280
ttaaagatat gtttagtttt tttatgttgt tttggttttt tgtttgtttt ttaatttttt 5340
ttttttgtta attttttttt gttatagttt tgttagaaga ataaatggtt agtattgagt 5400
gatttgtgtg ttttagtagg gaagttaagg tagagatgtt ggtagtacga ggtaagtatg 5460
aggttttttt tagggttttt atattagtag gtagagttgt ttttgtgttg gaagggagcg 5520
ggggaagttt taatagtaat aatatatgga aattttgttt gtggtttttt agtatgaata 5580
tttgtttata taataggatt gttgatgttc gtggatttta tattaaaaga tattaatgta 5640
ttttttaaat gaatttgtaa ggtggttttg ggtatgagta tagcgatttt ttagaaattt 5700

tagaaatata ttttttttaa aaatgttttt ttattttgag tgttggttat cgtgtttttt 5760
atttggtttt ttttttttta gttttttgtt tttttagatt tttttttata aagttttttt 5820
atttggttat atataaatat tgttttttta agataagggt atattttttt ttttttatat 5880
ttttgaatta ttttttttag tgtttttgtg ttttttgtcg tcgatgattt ataaagatag 5940
ttttttaagt ttaatagttt cggttggttt taatttgaaa gttaaatata atttttgttg 6000
aatgttttag tattgagtag tgagttgcga attatttttt agttgattgt tattgttggt 6060
ggtattgttt taatattagt tttggggaga tttatttttg gttattttat tttgtttata 6120
tttttttttt taaatttaga ttttattttt tttttttttt ttttaaaatt ttatttttat 6180
attttttatt attttttata aatttagatt taagtaatat atgtttataa aaatttaaag 6240
ttttgtttaa aagatataaa gtgaaaagta aaagtttttt atatgtattt attaaatgtt 6300
gaatatttat tatgtgttag gtattttaat tggtgtttgg tatagatttg tatatttttt 6360
tgggattgtg tgggggggtg ttgtttgttg ttattatatt agtatggttt tatgatatat 6420
attgttttgt aatattttta aatttagtaa tattttttga atattttttt aaaataaa 6478

<210> 213
<211> 2534
<212> DNA
<213> Artificial Sequence

<220>
<223> chemically treated genomic DNA (Homo sapiens)

<400> 213

aggtaggaaa gtgggatagt cggggagttg gattttattt tttgtgagtt tcgttggtat 60
ttgatggtat gtggtttgga gagggtaggt gatttggcgt ggagggttag agggtaaatt 120
tttaaataag tggtaatagg ttattaattt gaaagggaaa attgtgtagt gatgggaaat 180
gtgtttaata aatttattgg gtgattaatt ataaaggttg ggttggagtt ttagaggttg 240
tttgttaaat attttattaa gcggtatttt gaaagttgtt atttgcgtat tttgggagtt 300
tagaggggat tttgaggggg aatgaggttt ggaggatgga attattttta ggtagattga 360
gaaggagttt ggattttatt tttaaatata gtttggagtt tataggttag aggttttaat 420
gggagaaaag ttaaaggaag agggtgtaga aaggagtttt agggaattgg tggttatgtg 480
attttgagta aattttattt ttttttgaga tttagtgttt ttatttttat ggttttgtgt 540
gtgttataga gatatggtgg ggattaaatt cgatcgtgat atgaaagtgt ttgggaaatt 600
ttatggtttt atttaaatat gagttatttt tatttgaatt aaggggggaa gttatttggt 660
aggattagga attttatttt tttgaatttt tatgggtttt gtcgaggttg aagtagttag 720
gggttaaagt tagtttttag tttttggaag ggtattgtga aagtggattt gatttgagaa 780
gtcgtttttt gatgtgggta gttatgtgat gttagtttcg aataagaggg ggtagtttgg 840
agtttggaaa ggtgttagtg taggtggggt ttacgtttag attttttttg ttgattgttt 900
tgatgattta tttttatatt ttagtttttt tatttttatt gtagagtcgg aaagggtgtg 960
gggaagagag gagagggagg taggttttgg gttttggttt cgttttttgt ttttttttat 1020
ttttttttgg gtttggttat ttagttaaaa ggtaggttaa gagtaggaga gatatagagt 1080
tcggtattgg ttttaggtag tagttagttc gtcgttcgtt tgtgtgtttt tagagttatg 1140
gagagagtta gtttgattta gaaggttaag ttggtagagt aggtcgaacg ttatgaggat 1200
atggtagttt ttatgaaagg cgtcgtggag aagggcgagg agtttttttg cgaagagcga 1260
aatttgtttt tagtagttta taagaacgtg gtgggcggtt agagggttgt ttggagggtg 1320
ttgtttagta ttgagtagaa aagtaacgag gagggttcgg aggagaaggg gttcgaggtg 1380
cgtgagtatc gggagaaggt ggagattgag ttttagggcg tgtgcgatat cgtgttgggt 1440
ttgttggata gttattttat taaggaggtc ggggacgtcg agagtcgggt tttttatttg 1500
aagatgaagg gtgattatta tcgttatttg gtcgaggtgg ttatcggtga cgataagaag 1560
cgtattattg atttagttcg gttagtttat taggaggtta tggatattag taagaaggag 1620
atgtcgttta ttaattttat tcgtttgggt ttggttttga attttttcgt tttttattac 1680
gagatcgtta atagtttcga ggaggttatt tttttggtta agattatttt cgacgaggtt 1740
atggttgatt tgtatatttt tagcgaggat ttttataaag atagtatttt tattatgtag 1800
ttgttgcgag ataatttgat attgtggacg gtcgataacg tcggggaaga ggggggcgag 1860
gttttttagg agttttagag ttgagtgttg ttcgttatcg tttcgttttg ttttttttag 1920
ttttttattt tgtcgagagg attagtatgg ggtgggaggt tttatttttt ttttttaggc 1980
gttgtttttg ttttaaaggg tttcgtggag agggattggt agagttgagg ttatttgggg 2040
ttggggattt tatttttttt gtagttgttg agcgtattta attattggtt atgtttttat 2100
ttttgttttt cgtattcgtt ttttttcgat tttaggatta ggttattttt tttttttttt 2160
tgtttttttt ttgtttttgt tgtttttgat cgtaggaatt gaggagtgtt tcgttttgtg 2220
gttgagaatt ggatagtggt aggggttgga gatgggtgtg tgtgtgtgtg tgtgtgtgtg 2280
tgtgtgcgcg cgcgttagtg taagatcgag attgagggaa agtatgtttg ttgggtgtga 2340

ttatgttttt ttttaataaa gtttttttgt gatatttttt ttgttttttt tttagttttt 2400
ggcgatgggt tgggagtggg attggaattt gatttagaga ttttgatttt ggatttttga 2460
gttagggttt tgaatttttt aggtggttta gtggttcgta cgtaagattt tgagtttagg 2520
tgaggtcggg gttt 2534

<210> 214
<211> 2534
<212> DNA
<213> Artificial Sequence

<220>
<223> chemically treated genomic DNA (Homo sapiens)

<400> 214

ggatttcggt tttatttgga tttaaagttt tgcgtgcggg ttattgagtt atttagggag 60
tttagggttt taatttagag gtttaaagtt agggttttta agttagattt tagttttatt 120
tttagtttat cgttaagaat tggaagagag ataggaggag tgttataggg gaattttatt 180
gagaggaaat atggttatat ttagtagata tgtttttttt tagtttcggt tttgtattgg 240
cgcgcgcgta tatatatata tatatatata tatatatatt tatttttagt ttttgttatt 300
gtttagtttt tagttataag gcgggatatt ttttaatttt tacgattaga ggtagtaggg 360
gtaggaggga ggtaagagga ggggagaagt agtttggttt tggggtcggg aggaagcggg 420
tgcggagagt aggggtgggg gtatgattag tggttaggtg cgtttaatag ttgtaagaag 480
agtgggattt ttagttttag gtggttttag ttttgttagt ttttttttac ggagtttttt 540
ggagtaagaa tagcgtttag gggagaaggg tggggttttt tattttatat tagttttttc 600
ggtagggtgg gggattggag ggggtagggc ggggcggtgg cgggtaatat ttagttttgg 660
ggtttttggg gagtttcgtt tttttttttt tcggcgttgt cggtcgttta tagtgttagg 720
ttgtttcgta gtagttgtat gatgagggtg ttgtttttgt aggagttttc gttgagggtg 780
tgtagattag ttatggtttc gtcgaaagtg gttttggtta gagagatggt tttttcgggg 840
ttgttggcga tttcgtagtg gaagacggaa aagtttaggg ttaggtttag gcggatgggg 900
ttggtggcgg gtattttttt tttgttgatg tttatggttt tttggtaggt tgatcgggtt 960
gagttaatga tgcgtttttt gtcgttatcg gtggttattt cggttaggta gcggtagtag 1020
ttatttttta tttttaggta gaagattcgg ttttcggcgt tttcggtttt tttgatgagg 1080
tggttgttta gtaggtttag tacggtgtcg tatacgtttt ggagtttagt ttttattttt 1140
tttcggtatt tacgtatttc gggttttttt tttttcgagt ttttttcgtt gtttttttgt 1200
ttaatattgg atagtatttt ttaggtagtt ttttggtcgt ttattacgtt tttataggtt 1260
attgagagta ggtttcgttt ttcgtaggag agtttttcgt ttttttttac ggcgtttttt 1320
atgaaggttg ttatgttttt atagcgttcg gtttgttttg ttagtttggt tttttggatt 1380
agattggttt tttttatggt tttggggata tataggcggg cggcgggtta attgttgttt 1440
gggattaatg tcggattttg tgtttttttt gtttttggtt tgttttttgg ttgggtggtt 1500
aggtttagag aagggtgggg aggagtaggg ggcgggatta gggtttaaga tttgtttttt 1560
tttttttttt tttttatatt tttttcggtt ttgtagtaaa ggtaaaaagg ttgggatgtg 1620
ggggtgaatt attagaatag ttagtaggag aaatttgggc gtgggtttta tttgtattgg 1680
tattttttta ggttttaggt tgtttttttt tgttcggggt tggtattata tggttgttta 1740
tattaggaaa cggtttttta aattagattt atttttatag tgttttttta ggggttaagg 1800
attggtttta gtttttggtt gttttagttt cgatagagtt tataaaggtt taggaggatg 1860
gggtttttaa ttttgttagg taattttttt ttttggttta ggtgaggata atttatgttt 1920
aggtagggtt atggagtttt ttaagtattt ttatattacg atcgaattta atttttatta 1980
tgtttttgtg atatatatag gattatagag atgggaatat taagttttag agaggggtga 2040
gatttgttta aagttatata gttattaatt ttttgaaatt tttttttgta tttttttttt 2100
ttagtttttt ttttattgag gtttttgatt tatgagtttt agattgtgtt tggaagtgag 2160
atttaggttt ttttttagtt tatttgaaga tggttttatt ttttaggttt tatttttttt 2220
tagggttttt tttgagtttt tagaatgcgt aggtggtagt ttttagagtg tcgtttaatg 2280
aagtgtttaa taagtagttt ttgaagtttt agtttagttt ttgtaattag ttatttagta 2340
ggtttgttgg atatattttt tattattata taattttttt ttttaagttg gtggtttgtt 2400
gttatttgtt tgaggattta ttttttggtt ttttacgtta ggttatttgt tttttttaag 2460
ttatatgtta ttaggtatta gcggggttta tagagggtgg ggtttagttt ttcggttgtt 2520
ttattttttt gttt 2534

<210> 215
<211> 6558
<212> DNA
<213> Artificial Sequence

<220>
<223> chemically treated genomic DNA (Homo sapiens)

<400> 215

taagattata aattgttttt tattagtggg gaaggtttta aatattatgt atttagtata 60
agtaggtgta attaatcgta acggtttgtt ttgttatttt cgattaatat ataattatta 120
ggttagaatt gtattttata agttttttgc gtttagttat tttgtatata ttaatttttt 180
taagttttgt gttttaaaag aattgtatag tgttgggaag ttttttattt attttaattt 240
ttagggtatc gagttcgaat tcgaattagg tattaggtat gttttattat tataaaagat 300
gtaaaaagat gcgtatagtg gcgtatattt atagttttag ttattgagga ggttaatgtg 360
ggaggattat atgaggttgt agtgagttgt gatggtgtta ttgtatttta gttttggcga 420
tagtgagttt atgttttaaa taagtaagta aataaaaatt aaaaagaatt tagtttatag 480
ggtatttgaa ggtaagagga aaagatgtta gaattagaga tggggagaag atgggttttt 540
ggcgtttgtt gaggttgaga aatgagatag ataggttgag tgtggggtgg agagaggatg 600
ggtagagaga ttgaggttgg tttgaatgga aatgaaatgt tagggttttt agggttatcg 660
gggaataatt ggagttttta ggaaaggttt aacgttgtga ttatttgtgt tcgttatgtt 720
tttttatttt ttattaattg tgtgaatttg gtagattttg agttttagtg tttttttttg 780
tgaagtgggg ttattttatt ttaatttttg ggattgttgt gtgaattaaa tggggtaatg 840
tacggagagt atttgacgta tagcgagtgt tttaaaattt tagtttgtat tttttagtaa 900
aggatatgta tacgtttatt gtgagtgata aatttaggat gatttgaatt taatgtgata 960
acgtgggttt tcgtatgttg gttatgttgt cgggagatat ttatggattt aattagtata 1020
ataggggaaa taaattattt aatgtatttt gttaagatag aatattttag aatttatttt 1080
gtggggcggg gtataataaa gggggttttt ttgttgaaaa cgtttaagtt taggttcgtg 1140
gtattattta attaaggtcg atagttatat agtaagttag aggtaatgtt aggatttaaa 1200
ttaaatttgt ggtttttata atgaggttat tttttttttt tttgaacggt ttggggaaag 1260
ggggtgggtg ggtagaattt ggtagtggtt aattttttat ttttgttttt tggttttttt 1320
ttgtttttat ttttaggttt gtattgattg attgattgag atagggtttt gttttgtcgt 1380
ttaggttgga gtgtagtggt acgattatgg tttattgtag ttttaaattt ttaggtttaa 1440
gtggtttttt cgttttttat ttttcgagta tttatatttt taggttttta aaatggtttt 1500
taggtatttg gttgtcgttt tagatattta tttgggtttt tgggtaggga ttgttcggga 1560
aattttattt atgtgaagta ggtgtgggtg taggaaggtc gtttggaaat gaattagtat 1620
tgttttttgt ttgagtcgta agtagggcgt tagagggttt ggcggataag aaagggagga 1680
tgataggagg tcggtattgt aatgatacgt tttagttatt agagggtacg aagtagttgg 1740
gtaaaatttc gcggggtcgt tggtggaaaa tttttggtat ttggagttcg gagatggggt 1800
ggacggaatg tgaggattta gttttttgag gttgggtcgg ggtagagtta ttgttttgga 1860
tgttcgtagg gtttgtttgt gttttgatta ttttgttttt gtagatagtt ggagaatgtg 1920
agagtgggat tgggatcgga ttttaggggt tatttcgtat aatttttttg ttttgtcgtg 1980
ggggagggag ttgtttaagg ttacgtagta agttagtggt aaatgaatac gattattatt 2040
agttttaggt atatggttat ttgatgggcg tagtcgtagt tttagttttt gagatagaga 2100
tatttgatta aggataggtt tttaggagtt gattttagtg attcgcggtt ttgttgttgt 2160
ttttgttttt ttttttggtt tttttatttg attgattttt tgtttttttc gtttgtttgt 2220
ttgttttcgt ttttgttcgt tggggggttt gtttaatttt tttattgggt tttgggagtc 2280
gtagtttttt gttgttattt tttagggatt tgtagttttt tgaagttttt tttcgattcg 2340
ttgtttcggt tttatttttg ggatttagag gagaggtgat tatttcgtag tatagttagt 2400
ggtgtgattt tacggggtga gaaggatttt tttgttttta agtatttttt tagtgatttt 2460
tgttgttatg tggtagtcgt aagtattggt tggtatttgg tgtgggcgag atttttattt 2520
tatgtagaaa tgagtaagat cggtgagttt attatgtggg ggtgaggttg agagaaaata 2580
agtatatagg tgatttagtt aaaattagaa ttttttaagt atatacgaaa agggtaaaag 2640
gggcgttttg tataggatag aataggtaga tattgaattc ggttgggttt tgggaaggtt 2700
ttttgtagtg gtttttgaag ggggggttgg attttagtag gatagagggt atgggtatgt 2760
gtgggtacgt tttgaataga ggggttagcg taagtcgagg gttttggtta tattagttgt 2820
atgtgtcggt gtgtttaagg gatacgtagt agtaggtcga gtttggagcg ttttattgtt 2880
aggtttttta aaaattttta attttaattt aattttattt tatttttatt ttaagttttg 2940
gtatatatgt gtagaatgtg gtttgttata taggtatata tgtgttatgg tggtttgttg 3000
tatttattaa tttattattt aggttttaag tttcgtatgt attaggtatt agttttaatg 3060
tttttttttt ttttgttttt attttttttt tcgtaattgt ttataggttt tggtatgtgg 3120
tgtttttttt tttgtgttta tatgttttta ttgtttaatt tttatttatg agtgagaata 3180
tatcgtttgg ttttaaggga tagttatggg gatgtattgt agtttttgag tagggaaggt 3240
tttgtggagg tttttagtta aaaggaaaga atggttgtga aaatcgatgt attgcgtttt 3300
tttgtttttt atttttagtg tgaaggtttt ttatttcgag ttttatttga agtaggtttc 3360
gatgggaaga taagtagtat gaggggttta agtattgagg ggagtaaggg atattcggtg 3420

gttgtgttaa ggtgtagaag aggatattgg gggttttaag gtttgatttt atgtatattg 3480
tttaggttgg tttttaagtt atacggtgat cgttaggaag ggattagttt gtttttagtt 3540
tgattttttt gttatgttat tatttaaagt tttttttggt agggtttgtt tggggttttt 3600
gtgttagtgt tttttttgtt aggttgggtt ggggttttta tttattgttt tgggattgtt 3660
gttgttttgg ttttggggga ggagggtgtg tcgttgagtt attgtttggg tatttgggtt 3720
tggaatttcg ggtgagttat ttagggttga ggtagagggg ttgggggagg ggaagaagtt 3780
attcgatagt tggagtaggg aggggagttg gggttatagg aagggcggtg ttttgatgtt 3840
tagacgggtc gggatagata aagggttaag gaggaagggg ttttgggagg gggtagtttt 3900
tttttgggtt gggttttgaa tggttatagt gtttgttttt tttcgggttt ggggaggata 3960
tgtgtgtggg gggtagtgag agagggttgt ggttgagggt tgtgttttag gtttggattt 4020
tggtttggga agttgtttag ttggtgtttt tagttttggg tagggatgta tttttattta 4080
tttatttagt ttttaagttg gagaagagga ggttaaagtt tttttgttta gtttttaatt 4140
attcgggatt tttttatgtt ttttatagat tgtggtttag tttaattgcg gttgtgtgta 4200
gagtaatttt attttttatt gtttttttat ttttttagat atttttttat atagagggat 4260
ttttttaggt atttttaagt atatttagtt attttattat tttattaaga ggtattttgg 4320
tgttggttat taaaagttat tttattttat ttatgttttg aagttagttt tgtttttttt 4380
tttttgatgt tttttagttg ttttttttgg tttttagttt tttaagggtg gttttaggtt 4440
gttttttttt tatatatata ggcgtatgta tgtatacgag tattggatta tgaagtttta 4500
gcgtgtgttt atagtttttt atataggagt gggttgtgat ttataggtat gttatgagaa 4560
tgaggtttgg tattagtttt taggttttag agtaggggtt gttttttttt atttcggttt 4620
aggatgttta gtttttacga tattttttat tttttattgt ggtttgggtg ggtttagggg 4680
ttgtttttga tttggtttag agtttttttt tagttggtgg tggagttggt attttttggg 4740
agggaggggg ttgggaggga atgagtggga atggtaagag gttagggttt ggtgggatta 4800
ggttgaggta ggtttggttt ttttaaaatg ttaagttggg ggttagtggg gtttatatat 4860
aaatttttat tttgggagtt tggttgtttt gttttttttt ttgggtttgt ttttgttatt 4920
tggtttggtg agtatttttg ttttgttgag ggtagggtgg ggaggatttt cgtgggtttt 4980
tgtttttgtt tttatagttt ttttatttta gtttttttgg tgggattaat ttgggttttt 5040
ttgggttttt ttttttggaa gaattttttg tgaagtgttg aagtgttgat tgaagggttt 5100
tttttttttt tttttttttt tgagatggag tttcgttttg tcgtttaggt tggagtatag 5160
tggtgtgatt ttagtttatt gtaaattttt ttttttaggt ttacgttatt tttttgtttt 5220
agtttttcga gtagttggga ttgtaggcgt ttattattat gttcggttaa tttttttgta 5280
tttttagtag agatggggtt ttattatgtt agttaggatg gtttcgattt tttgatttcg 5340
tgatttattt atttcggttt tttaaagtgt tgggattata ggagtaagtt atcgcgttcg 5400
gtcgattgaa gggttttttt ttaggttttt ttgtgaggtt ttagtgtagg ggttgttttg 5460
aggttttttt ttggatattt tagtttaggg gttttttttt gggggtttag gtttaggagt 5520
aggaggtgtg tatgtgggcg ttgttgtaaa aagaattttg agattttttt tttttttttt 5580
tttttttttt gtaaagtttt ggattttagt aggattaagg tgtaagaggt aggggtttta 5640
agattttgtt tgggttatgg ttttaagtag taaagttttg ttttttgttt cggtgaaggt 5700
agggttggta tgatgggttt agggtatgtt ttgtttttgg tatagttttt ttggttttat 5760
tttgaaattt gtttaatttt tttaggttgg tttgagtatt tttagaggtt ttgtcgttga 5820
ggtttgtttt attttgattt taaggtaatg aatattttat atttttaatt ttaattttaa 5880
taggattttt tttggtggag agaatgttaa gttgttttta ttttatttat gtttttgttt 5940
gtttagaggt ttaggggttt ttagggtgag gggagatata ttttttattt tttgggagtt 6000
tttagtttga gagaggaaat atttttgttt aagggagttt ttagttagat ggtagagaga 6060
gatgtttttg gttttaggag tttcgagttt aaggagggaa acgatttttt tagggagttt 6120
tttgttttta ggttgtagtt atggtttttg ttagattgta taggagtttt tatttgttag 6180
tcggtgtatg tggttttgtt ttttagagtt tgcgtagatg ttattaaaat gggattttgg 6240
ttattttgtt attttttttt tggtagatat taaaatgggg agttttgttt ttaggggggt 6300
gttttaagtg ttattagagg aggtttggtg atttttagat ataagggaag ttttagcgtt 6360
tgtttttagg gtgagatgga ggtatcgttt tcggttttag ggaattatag tttgaggggg 6420
agatgtagtt tttgtttttt tatttagaga ggggttttgt gaggtggttt gggggtatag 6480
ggtagaagtg gattttatag gttgagttaa ggttttaaga gttttagtag tgtatttatt 6540
atttggtatt tttgtagt 6558

<210> 216
<211> 6558
<212> DNA
<213> Artificial Sequence

<220>
<223> chemically treated genomic DNA (Homo sapiens)

<400> 216

gttgtagagg tgttaggtga tgggtatatt gttgaggttt ttggggtttt agtttagttt 60
gtaggattta tttttgtttt atgtttttaa gttattttat aaaatttttt tttgaatggg 120
aaggtagggg ttgtattttt tttttagatt gtggtttttt gaggtcggag gcgatatttt 180
tattttattt tgaggtagaa cgttaaagtt tttttgtgt ttgggagtta ttaagttttt 240
tttgatggta tttgggatat ttttttgagg gtagggtttt ttattttagt gtttgttaga 300
agggaaatga tagggtgatt agagttttat tttgatggta tttgcgtagg ttttggggag 360
tagggttata tgtatcggtt ggtagatggg ggtttttgtg tagtttggta ggagttatgg 420
ttatagttta ggagtaggaa gttttttgaa ggagtcgttt ttttttttag attcgggatt 480
tttgaagtta gaggtatttt tttttgttat ttaattggaa gtttttttgg gtaggagtgt 540
tttttttttt agattaggag tttttagagg gtggggaatg tgtttttttt tattttgaag 600
gtttttgagt ttttaggtag ataggggtat ggatagggtg ggggtaattt aatatttttt 660
ttattaggaa ggattttgtt ggaattagaa ttaaagatat gaaatgttta ttgttttggg 720
attaggatgg gatagatttt agcggtaagg tttttgagaa tatttagatt agtttggaaa 780
ggttaggtag gttttagggt gaggttagag gagttatgtt agaggtaggg tatgttttgg 840
gtttattatg ttagttttgt ttttatcgag gtagggggta gagttttgtt gtttggggtt 900
atgatttagg tagagttttg agatttttgt tttttgtatt ttagttttgt tagaatttag 960
gattttgtaa aaaaaaaaaa aaaaaaaaaa aaaaatttta ggattttttt tgtagtaacg 1020
tttatatgta tattttttgt ttttaggttt agatttttaa agaagggttt ttagattgag 1080
atatttaggg gagggtttta gagtaatttt tgtattgaga ttttatagag gaatttggag 1140
aaaaattttt tagtcggtcg ggcgcggtgg tttatttttg taattttagt attttgggag 1200
gtcgagatgg gtggattacg agattaggag atcgagatta ttttggttaa tatggtgaaa 1260
ttttattttt attaaaaata taaaaaaatt agtcgggtat ggtggtgggc gtttgtagtt 1320
ttagttattc gggaggttga ggtaggagaa tggcgtgaat ttgggagggg gagtttgtag 1380
tgagttgaga ttatattatt gtattttagt ttgggcgata gagcgagatt ttattttaaa 1440
aaaaaaaaaa aaaaaaaaaa attttttagt taatatttta gtattttata gagagttttt 1500
ttaaggggga aggtttagag aggtttaggt tgattttatt agggaagttg gaatgagaga 1560
attgtggaga taaagataga gatttacggg gatttttttt attttgtttt tagtaggata 1620
gaggtattta ttagattagg tggtagagat agatttagga aggagagtaa ggtagttagg 1680
tttttagggt gaggatttat atgtgggttt tattggtttt taatttggta ttttaaggaa 1740
attaaatttg ttttaatttg attttattaa atttggtttt tttgttattt ttatttattt 1800
ttttttagtt tttttttttt tagagagtgt tagttttatt attagttggg ggagggtttt 1860
aggttaggtt aagggtagtt tttgagttta tttaggttat agtgggaagt gggaggtgtc 1920
gtggggattg ggtattttgg atcggggtga ggggaggtaa tttttgtttt ggggtttgga 1980
gattggtgtt aggttttatt tttatgatat gtttgtgagt tatagtttat ttttgtgtga 2040
gaggttgtga gtatacgttg agattttatg gtttagtgtt cgtgtatata tatgcgtttg 2100
tgtgtgtgag agagaagtaa tttggggtta tttttaggaa gttgggggtt agaggaggta 2160
gttgggggat attaggagga gaaggatagg attgatttta gggtatggat gaagtggagt 2220
gatttttaat ggttagtatt agaatatttt ttaatgaggt aatgaggtaa ttaagtgtgt 2280
ttagaaatat ttgggaaggt ttttttgtgt aggaaggtgt ttggaaggat ggggaagtag 2340
tgagaaatgg ggttgtttta tatatagtcg tagttgggtt gggttatagt ttgtggggag 2400
tataaggaag tttcgagtag ttaaaggttg aataggaaaa ttttggtttt ttttttttta 2460
gtttgagggt tgggtgggtg ggtaggggta tatttttatt taaggttgaa aatattagtt 2520
ggatagtttt ttaagttaga atttaggttt gaagtatagt ttttagttat agtttttttt 2580
tattgttttt tatatatatg ttttttttag attcggagaa aggtaaatat tgtggttatt 2640
tagagtttag tttaagggag ggttgttttt ttttagggtt tttttttttt tggttttttg 2700
tttatttcgg ttcgtttggg tattagggta tcgttttttt tgtggtttta gttttttttt 2760
ttgttttagt tgtcgagtag tttttttttt ttttttagtt tttttatttt agttttaagt 2820
gatttattcg aggttttagg tttagatgtt taggtagtga tttagcggta tatttttttt 2880
ttttagggtt agggtagtag tagttttaga gtagtaggtg gaagttttag tttagtttgg 2940
tagggaaagt attggtatag agattttaaa taggttttgt taggaggagt tttggataat 3000
gatatggtag gaggattaga ttgagaatag gttggttttt ttttagcggt tatcgtgtga 3060
tttgggggtt agtttgagta gtgtatatga agttaggttt tggggttttt agtgtttttt 3120
tttatatttt ggtatagtta tcgagtgttt tttgtttttt ttagtatttg aattttttat 3180
gttatttgtt tttttatcga ggtttatttt aagtagaatt cggaataaaa attttttata 3240
ttgagggtga gggataaggg agcgtaatgt atcgattttt atagttattt ttttttttta 3300
attaagggtt tttatagggt tttttttgtt tagaaattgt agtgtatttt tatggttgtt 3360
ttttaaagtt aggcggtatg tttttattta taagtgggag ttgaataatg agaatatatg 3420
gatataggga agggaatatt atatattagg gtttgtgggt agttgcgggg agaaggatgg 3480
gggtaagggg agggagagta ttaggattaa tatttgatgt atgcggggtt taaaatttag 3540
atgatgggtt gataggtgta gtaaattatt atggtatatg tatatttatg taataaatta 3600
tattttgtat atgtatgtta gaatttaaag taaaaataaa ataaaattaa attaaaatta 3660
aaaattttta aaaagtttgg tagtgaggcg ttttagattc ggtttgttgt tgcgtgtttt 3720

ttaaatatat cggtatatgt aattagtgtg gttaagattt tcggtttgcg ttgatttttt 3780
tgtttagaac gtgtttatat atgtttatgt tttttatttt gttgaaattt aatttttttt 3840
ttaaaggtta ttgtagggag tttttttagg gtttaatcgg atttagtgtt tatttgtttt 3900
gttttgtata aagcgttttt tttgtttttt tcgtgtgtat ttagagaatt ttgattttga 3960
ttgaattatt tgtgtatttg ttttttttta gttttatttt tatatggtga gtttatcggt 4020
tttatttatt tttgtatgag gtaagggttt cgtttatatt aggtgttaat tagtgtttac 4080
ggttattata tggtaatagg ggttattgga ggagtgttta ggagtaaggg agtttttttt 4140
atttcgtgaa attatattat tgattatgtt acgaaataat tatttttttt ttggatttta 4200
agagtggaat cgagataacg ggtcggaaaa gagttttaga gagttataaa tttttgagga 4260
gtgatagtag gaaattgcgg tttttaggat ttagtgaggg agttgagtaa attttttagc 4320
gggtagagac ggagataggt aggtagacga agaagataaa gagttagtta gatggaaaag 4380
ttagggagaa aaatagagat agtagtagag tcgcgggtta ttagggttag tttttgaagg 4440
tttgttttta attaggtgtt tttgttttag gaattgaggt tgcgattgcg tttattaaat 4500
ggttatatat ttgagattgg tgataatcgt atttatttgt tattaatttg ttgcgtaatt 4560
ttgggtaatt ttttttttta cggtagggta ggagagttgt acggaatggt ttttagagtt 4620
cgattttaat tttattttta tattttttag ttgtttataa aggtagagta gttaagatat 4680
aagtaggttt tgcggatatt taaagtagtg attttgtttc ggtttagttt taggaagttg 4740
ggtttttata tttcgtttat tttattttcg ggttttaggt gttagaaatt ttttattagc 4800
ggtttcgcgg gattttgttt agttgtttcg tgttttttgg tggttaaggc gtgttattgt 4860
agtgtcggtt ttttgttatt tttttttttt tgttcgttag attttttggc gttttgttta 4920
cgatttaaat aggagatagt gttgatttat ttttaagcgg tttttttata tttatatttg 4980
ttttatatag atgaggtttt tcggatagtt tttgtttaga agtttaggtg gatgtttgag 5040
acggtagtta gatatttgga agttatttta aaagtttagg gatatgggta ttcgggagat 5100
aggaggcgga aagattattt gagtttagga gtttgaggtt gtagtgagtt atgatcgtgt 5160
tattgtattt tagtttggac gatagagtaa gattttgttt taattaatta attaatgtaa 5220
gtttagagat aagggtagga gaaaattagg ggatagaagt gagggattgg ttattgttaa 5280
gttttgttta tttatttttt ttttttaggt cgtttagggg aaagagaaat ggttttattg 5340
tgggggttat aggtttagtt taagttttga tattgttttt ggtttattgt gtgattgtcg 5400
attttggttg agtggtgtta cgaatttgag tttaaacgtt tttagtagaa ggattttttt 5460
tattatgttt cgttttataa aataagtttt gaggtatttt gttttagtaa aatgtattaa 5520
ataatttatt ttttttgttg tattaattgg atttataagt gtttttcggt agtatgatta 5580
gtatgcgagg atttacgtta ttatattggg tttaggttat tttggatttg ttatttataa 5640
tgggcgtgtg tatatttttt gttggggggt gtagattgaa attttgaagt attcgttgtg 5700
cgttaggtgt ttttcgtata ttattttatt taatttatat aataatttta ggagttggaa 5760
taagatgatt ttattttata gaggagaata ttgagattta aagtttgtta aatttatata 5820
attagtaagg ggtggggagg tatgacgaat ataggtggtt ataacgttaa atttttttta 5880
gaagttttaa ttatttttcg ataattttga gagttttaat attttatttt tatttagatt 5940
agttttagtt tttttgttta tttttttttt attttatatt tagtttattt gttttatttt 6000
ttaattttag taggcgttag aagtttattt tttttttatt tttgattttg gtattttttt 6060
tttttgtttt taaatgtttt gtggattgga ttttttttaa tttttgttta tttatttatt 6120
tgagatatag atttattgtc gttaaggttg gagtatagtg gtattattat agtttattgt 6180
agttttatgt gattttttta tattaatttt tttagtaatt ggggttatag gtgtgcgtta 6240
ttgtgcgtat ttttttgtat tttttgtggt gatggagtat gtttgatatt tgattcgaat 6300
tcgagttcgg tattttaagg attggaatgg gtaaagaatt ttttagtatt gtatagtttt 6360
tttaaagtat aaagtttggg agaattaata tatgtaggat aattgaacgt aagagatttg 6420
tggaatgtag ttttaatttg ataattgtgt gttaatcggg agtaatagga taggtcgtta 6480
cggttggtta tatttattta tgttagatgt ataatgtttg gagttttttt tattgatgag 6540
aagtaattta tgattttg 6558

<210> 217
<211> 6432
<212> DNA
<213> Artificial Sequence

<220>
<223> chemically treated genomic DNA (Homo sapiens)

<400> 217

tattacgttt agttaatttt cgtattttta gtagagatag ggttttataa tgttggttag 60
gttggtttcg aaattttgat tttatgattt gtttgtttcg gttttttaaa gtgttggaat 120
tataggtata agttagtatg ttcggtttat attttttaat tgtatgtata atttaatttg 180
tagatgattt taaaagttta aattggagtt ttattgtttt ttttattttt agtttttata 240

ataaatttta tgatagtttt tgtttataag aatttttttg tagttttatt atttgtaaat 300
tatttaattt tgttttttcg tgatttttta ttttttatat tttttatatt ttttagttat 360
tgttttaaaa attttggata ataaattaat ttaaattttt ttttaaacgg tatttattta 420
tttttattta gatttatatg gattggttag aggtaggttt gagattggtt gatggaggtt 480
tggtttagat ttaattagga ggttggttta tatttgtttt atatgttttt tttttttttt 540
gtatttgttg gtttttaagt gtttttaatt gaaaataatt agtatattaa agtagtatat 600
tttggagtgg tattttttga aattttttat tggttaggtg gcgaagatta attatttgat 660
taatttgttt tagagtgtag aataagtttg gaattagata ggattaagtt taaatttggg 720
ttttgttttt tattagttat gtgatttttg ttaaatattt aattttttta atttttaaat 780
tttttatttg taaagggaag attagaatag tattgtaagg atatgtttta aaatttaata 840
aggattattt ttggataata gggttaagaa tttttttaaa gtttgttttt aaaaaaaaaa 900
tttaaatttt ttatttataa ttaatacgta ttatttttgt aatttttaaa aagttatttt 960
ttttaaaata tattattgat gttttttgta aatatgtggt ataattgtag gttttattaa 1020
tatttgtgaa gaattgtaaa taagagaatg gattaggaaa attgatattt ttattttttt 1080
ttttattgaa gataagaaat atatattgaa tattgattat ttgtagagta cggagtgtgg 1140
ttttatattg aaaaaggaat attttgtgtt tttaagggtt ttataattat tagggtagaa 1200
ttttaaaaag taaataaata ataataatgt attttatgtt agaatggggt aaatattttt 1260
ttttaaaaaa aggttaaata aacgaaatgt tttttgggtt atttttggga aaggagtgat 1320
tatagtaggt gaagtattat aaaatatttt ttggaggagg tagtattatg gatgatttat 1380
aaagggtgga tgatttttat aaattaatta tagttttagt gtttattggt ttgttttata 1440
ataaaaggaa aattggttaa gagataagag ttgagaatga ggttggttaa tttataagga 1500
gaggaatttt tttgttaatt gttttagttg atagtaattt taatagttgt atgttataaa 1560
gtttagggga gtaggaagtt ttttttttat tataattgag aaatttaacg tttaaataaa 1620
ttataatttg atttagttta attgtgtgtg tttgtgtgtg cgtgtagagt ttaaaaagtt 1680
ggtaaaatag attgttagtt tttaaaattt ttgtttaagt tgatatttat tgttttttaa 1740
tttattaagg gtttaaacgt tatttttttt gttttttaaa ttttgagtcg ggttattatt 1800
tttttgtttt tatttgtaaa tggtttagtt ttttatatgt atatattaat tgtttatgtg 1860
aatatataaa atattatatt aagttaaata tataattatg tgtgaaaatg atgttttgat 1920
taagggtatt agatattttt tttaataaga attttaaaag gaagggtatt atgataattt 1980
tttttttttt tttttttatt tattttttgt ttttgagata gggttttatt ttgtttttta 2040
ggttgaagtg tagtggtata attatggttt attgtagcgt tcgttttttg ggtttaagtg 2100
atttttttat tttagttttt tgagtagttg gtattataga tatatgttaa tatgtttggt 2160
tttttttttt tttttttttt ttttagtaaa gatgaaattt tattatgttg tttaggttgg 2220
tttcgaattt ttgggtttaa gtaatttttt tatttagatt ttttaaagtg ttgggattat 2280
aggtgtggat tattgtattt agtttgatga atttttaaaa ttattaaatt gtaatatgta 2340
tgagtttata tatttattat ttttattatg atgattttta aaaggttagt ttgtatttgg 2400
taatgttgaa ttttgatttt tttttaattt ataggagatt ggagattaaa ttagaaatgt 2460
taagggtaaa taaaattaaa attaatatta atgatgtatt atggtaaaga ttattagtta 2520
aatattttat tatagattta aatatttagt ttttaataat gtaaaatatt tttgtgataa 2580
ttaaggaaaa taaagttttt ggaatttatt ggatatttat ttgttatatt gtgtttttag 2640
gagaaagaaa gtatatgttt gttatttgga ggaatttggg taaatgtagg aaaaaaaaaa 2700
gaagaagaaa gaaatttata tattttttta tttttaagaa aaggaagtag tggtgatatt 2760
attttcggtt aaaatgtagt ttgtttatat tttaaatatt cggtaataat gtttttttta 2820
gagttatgaa gtaattagaa taattaaata taattttttt ttaaattttt attgtttgat 2880
ttatttattt atttaataaa tatttattaa atatcgatta tgtgtttgat gtttagggat 2940
ataatagtaa gtggagggaa agatatataa tatttgtttt taagaaattt ggagttgagt 3000
ggaggataga aatataaatt aaagaatgat ataaataatt ataaagttat agttgttaaa 3060
agaaaagtat atggtgttaa gagaacgtgt aatataagat ttatttatgg aggtgaggga 3120
aagtttgttt attaaagaag ttatgattta atttacgaag attaggagtt ggttgggtga 3180
agaaaaaaag gttagaggaa ggaagtttat attggggaag gttttaagta taaagggtag 3240
gaggattata gaggtatatt tacgaaattt ggagaaggtt tttagtaagt aaggagaagt 3300
taaatgaaag tttacgggag agttggaggt ttgaagatac gtttaaggat ttggttttta 3360
tttttttttt attttaagag tagtgggaag ttattaaatg attttaatta gagggttggt 3420
ataattagtt ttgtattttg aaaagttgaa tttagttttc gtttgagaaa ttgagtgaaa 3480
gagtttagaa cggtcgtggt tgagggtgat tcgtgggaga tttttatata agttatggta 3540
gtggtatggg ttggtggtag aagagggaat agggagaaga tttggaattt aatttttttt 3600
tattgataaa gttattttag ttttggtaag gtaattaatt ggtgggaaag aagatgttta 3660
gtttttttga ttttattgta ttttttgtat ttttaatatg agtattggga agtggtaaaa 3720
tatttagagg tagtttgggt gttaggtgga gtatgagtta aaattttagg atgaagtaaa 3780
tgaatattta gaatgatagg aaagatttgg gagttgggtt tggggagggt ttatttattt 3840
ttattttttg gagattttgg tataaatttt tgtttttgta attttttttt taggtaaagg 3900
aatttattaa atgaattgtt agaagattta ttgattagag ggttgtatag aattatattt 3960
ttgagagtgg gaagtaggtt gattatatag tttattattt aattaggata tatttgaaag 4020

agaaaggggg ttttattaat atttaaatta taaaatatgt atattaggaa tgttttgggt 4080
aaatttggtt gttttagtaa gaaaggaaat ttgaaagttt atattgtttt gtttttatgt 4140
tatttcgttt gtatatgaga gggtaagtat tttttttttt tatttgtatt aagggaataa 4200
aagtataagt atttaggtga tttttaattt atttttaatt ttatagtttt tgttatattt 4260
tatatatttt gaaaattata ttttttatta ttattatttc gtgataggtg attatttata 4320
attatttatt gatttagttt cgggaagagg cggtgtaaaa tgggacgttt tatttaggtg 4380
tttattagaa atgtagaatt tttgtttgtt ttttagattt attgaattag aatttgtatt 4440
tttaaataag atttttaggt gattaatatg tatattaaaa tttgagaaaa atttttagat 4500
ttcgatttaa agaaaaatat tttataattt gatagtgtat gtatatatat atatgtatat 4560
agatataatt gaagtataaa tttaatgaag tagaatttat cgttattatt ttatttggga 4620
aagaaatgtg ttcgcgattt aatagattgg agtatttatt tttggatttt aatttgtaat 4680
ttgaaaacgt atttttaaag tatttaggag taatttgaag aaagttgagg ggaggcggta 4740
gatgttttga tttattaggg aaaacgtgga cgttttttgt tgttattttg tgaattgtgt 4800
gtatttagtt atttttgagt aaatatttgg agcgaggaat ttttgagtgg tgtgggaggg 4860
cggtgagggg tagttgaaag tcggttaaag ttttcggagg ggttggttta ggaaatatga 4920
ttggtagtta cgagagagtt aggggttgga cgtcgaggag agggagaagg ttttcgggcg 4980
gagagaggtt ttgtttagtt gttggcgagg agttttttgt ttttttcgta gcgttgagtt 5040
gaagttgagt gagttattcg cgcgtacgga gcgacgatat tttcgcgcgt gtattcgttc 5100
gggataggag tcggattttt gtgtagtttt tttcggtcgt cgggggtttt ttcgcgtttc 5160
gtcggttttt aggttttttt ttggttggcg agcgggcgtt atatttggtt cgtatatttg 5220
cgttgtcggt tcggcgcggg gttcggagag ggcgcggcgc ggaggcgtag ttaggggttc 5280
gggaaggcgt cgttcgttgc gttgggggtt cggtttatga cgagtagcgg ggtttgttat 5340
gggtcggggg ttgtttaggg gtttgtggtc gttgtatatc gttttgtgga cgcgtatcgt 5400
tagtacgatt ttatcgtacg tttagaagtc gggtgagtgg tttttagttc gggttcggcg 5460
gggcgtcggg ggtttttttg gggttttcgt tttttcgttg cgtttgatag tcgggttcgg 5520
taattcggtt ttcgggcgga aacgaggaaa gtttttttcg cgatatttac gtagttcgat 5580
tttcgtagtt gtagggattg tgagtttttt ttgaaaaaga gaaggaaagt ttagttgtaa 5640
ggggcgcggg gtacgtttgg ttttttttgt gcgagtagga aaggcgttgt gttggtcgcg 5700
ttcgaggcga gtttttattt tcggaaaggg aagtttgaga agttggttat ttgaaggcgg 5760
tcggggagta gcggttcgga gcggtagttt gagttgttaa agtagttagc gtatttgggt 5820
tatttcgttt atggcgatgt cgcgtgttta tttagttttt tttgagttat tcgtttaaaa 5880
ggttagtttt ttagttttta gtttttggag acggttacgt ttttttttag gtcggttttt 5940
tggttcggag tttttggaaa taagttttaa gaaaataatc gatttttaaa agaaagttag 6000
ttggttttat tgacgttttg gtatggatgg atagggagtg gagatgttta ggtgaaatcg 6060
agaatttttt attgaatgtt tattgggtgt tagaggttgt agattttgtt tggaataaga 6120
tagttttgtt tttagggagt tgatggtttt atgtaattat cgtttgttgg aaatcgaagg 6180
gttaaaattt taattaggtt atttatttga tcgttgtttt tgaaggtttt taaggaaaaa 6240
ataaaaataa aaaaatatat atatatatta tatatatata aatatatata taattatata 6300
tataaatata tataatatta atatatatat gtatgtagga atgggggttt ttaattattt 6360
gttatggaaa gtgtaaaatt tttgagattt taaaagttag attttttttt tttggagacg 6420
gagtttcgtt tt 6432

<210> 218
<211> 6432
<212> DNA
<213> Artificial Sequence

<220>
<223> chemically treated genomic DNA (Homo sapiens)

<400> 218

agagcgagat ttcgttttta aaaaaaaaaa atttaatttt tgaagtttta gaggttttat 60
attttttata ataaatagtt aagaattttt atttttgtat gtatatatat attaatatta 120
tatatattta tatatataat tatatatata tttatatata tataatatat atatatattt 180
ttttattttt attttttttt taaaaatttt tagagatagc gattaggtgg gtagtttagt 240
taggatttta atttttcggt ttttaataaa cgataattgt atagaattat taattttttg 300
aagataggat tattttgttt taggtaaaat ttatagtttt tagtatttag taggtattta 360
gtgagggatt ttcggtttta tttgagtatt tttatttttt atttatttat gttaaggcgt 420
tagtggagtt agttaatttt tttttggaaa tcgattattt ttttgaaatt tgtttttaaa 480
aatttcgggt taagaagtcg gtttgaggga aagcgtggtc gtttttagga gttaaggatt 540
gaggagttgg ttttttgaac gggtggttta aaaagagttg ggtgggtacg cggtatcgtt 600
atgggcggag tggtttaggt gcgttggttg ttttggtagt ttaggttgtc gtttcgggtc 660

gttgttttc ggtcgtttt agataattaa tttttaaat ttttttttc gggggtgggg 720
gttcgtttcg aacgcggtta atataacgtt ttttttgttc gtataaaggg gattaaacgt 780
gtttcgcgtt ttttgtaatt gaattttttt tttttttttt aagaaaaatt tataattttt 840
gtagttacgg gagtcgggtt gcgtgagtgt cgcgggggaa atttttttcg ttttcgttcg 900
ggggtcgggt tgtcgggttc gattgttaag cgtagcggag aggcggggat tttaggaaga 960
ttttcggcgt ttcgtcgagt tcgggttggg gattatttat tcgatttttg aacgtgcggt 1020
gggatcgtgt tggcgatacg cgtttatagg acgatgtgta gcggttatag gtttttgagt 1080
agttttcgat ttatggtaga tttcgttgtt cgttatagat cgagttttta gcgtagcgga 1140
cggcgttttt tcggattttt ggttgcgttt tcgcgtcgcg tttttttcgg atttcgcgtc 1200
gggtcggtag cgtagatgtg cgggttagat gtggcgttcg ttcgttagtt aggagggggt 1260
ttggaggtcg gcgaggcgcg gggaggtttt cggcggtcga gggaagttgt ataggagttc 1320
ggtttttgtt tcgagcgggt gtacgcgcgg gggtgtcgtc gtttcgtgcg cgcgagtgat 1380
ttatttaatt ttaatttagc gttgcggggg aaataggaaa tttttcgtta atagttgggt 1440
aggatttttt ttcgttcgag agtttttttt tttttttcga cgtttagttt ttagtttttt 1500
cgtagttgtt aattatgttt tttagattag ttttttcgag agttttggtc gatttttagt 1560
tgttttttat cgttttttta tattatttag gagtttttcg ttttaagtat ttatttaaga 1620
atgattaagt gtatatagtt tataaagtaa taatagaaaa cgtttacgtt ttttttagta 1680
gattagaata tttgtcgttt ttttttagtt ttttttagat tgtttttagg tgttttagag 1740
atgcgttttt aaattgtaag ttgagattta ggagtgaata ttttaattta ttgagtcgcg 1800
agtatatttt ttttttaaat aaaatagtaa cggtaaattt tattttatta aatttgtgtt 1860
ttagttgtgt ttatatgtat gtatgtatgt gtatatattg ttaagttgta aaatgttttt 1920
ttttaggtcg aagtttagag gtttttttta agttttaatg tatatattga ttatttggaa 1980
attttattta aaaatgtaga ttttaattta gtaggtttag gaggtaggta gagattttgt 2040
atttttaatg agtatttgga tagagcgttt tattttgtat cgtttttttt cgggattgag 2100
ttagtgagta attgtaaatg attatttatt acgaagtgat agtggtggga aatgtaattt 2160
ttagaatgta tagagtatag tagaaattgt aaaattaaaa gtgggttggg agttatttga 2220
atgtttgtgt ttttattttt ttaatgtagg tgaagaaaga gaatatttat ttttttatgt 2280
gtaaacgggg taatatggga gtagaatagt ataaattttt aaattttttt ttttgttagg 2340
gtaattagat ttgtttaaga tatttttggt gtatatgttt tgtagtttaa atattaatag 2400
aatttttttt tttttttaga tatgttttga ttggataata aattatgtga ttaatttatt 2460
ttttattttt aaagatatga ttttgtatag ttttttggtt agtagatttt ttagtaattt 2520
atttaatgaa tttttttatt tgagaggaag attgtagagg taagggtttg tgttagggtt 2580
tttagggaat aaaggtaaat agtttttttt taaatttaat ttttaaattt tttttgttat 2640
tttaagtgtt tatttgtttt attttggaat tttaatttat gttttattta gtatttaagt 2700
tgtttttgga tgttttgtta ttttttagta tttatgttga agatgtagaa agtgtagtga 2760
aattaggagg gttaggtatt ttttttttta ttaattaatt gttttgttaa agttggagtg 2820
attttgttaa tggaggaaga ttgagtttta aatttttttt ttattttttt ttttgttatt 2880
agtttatgtt attgttatgg tttgtgtagg agttttttac gagttatttt tagttacggt 2940
cgttttgggt ttttttattt agttttttaa acgagagttg aatttagttt tttaaaatat 3000
aaaattagtt atattaattt tttgattaaa attatttaat ggttttttat tgtttttgag 3060
ataaagagaa gataaaaatt agatttttga acgtgttttt aagtttttaa ttttttcgta 3120
aatttttatt tggttttttt ttgtttattg aaagtttttt ttaaatttcg tgaatatgtt 3180
tttgtaattt ttttattttt tatgtttaga gtttttttta gtgtggattt tttttttttg 3240
attttttttt ttttatttag ttaatttttg gttttcgtgg attgaattat aatttttttg 3300
atgggtaagt ttttttttat ttttatgagt agattttgta ttatacgttt ttttggtatt 3360
atatgttttt tttttaatag ttgtaatttt ataattattt gtgttatttt ttaatttata 3420
tttttatttt ttatttaatt ttaaattttt tgagggtagg tattatgtat tttttttttt 3480
atttattatt gtgtttttaa gtattaaata tataatcgat gtttaataaa tatttgttgg 3540
atgaatgaat gaattaggta ataaagattt agaagaaaat tgtatttgat tgttttagtt 3600
gttttataat tttggagaaa atattgttat cgaatatttg agatataaat agattatatt 3660
ttagtcgaga atgatgttat tattattttt tttttttaag aatggagaag tatgtgggtt 3720
tttttttttt tttttttttt tttttatatt tgtttaagtt tttttagata gtaaatatgt 3780
attttttttt ttttgaaaat ataatgtggt aaataaatgt ttaatagatt ttaaaagttt 3840
tgtttttttt aattattata aagatgtttt atattgttgg aggttagata tttaaattta 3900
tggtggaatg tttaattggt ggtttttgtt atgatatatt attaatgttg attttaattt 3960
tgtttgtttt taatattttt gatttaattt ttagtttttt gtgaaattga gaagaattag 4020
aatttagtat tgttaagtgt aagttagttt tttgaaaatt attataataa aaataataga 4080
tgtgtaaatt tatgtatatt gtaatttgat aattttaaaa atttattagg ttgagtgtag 4140
tggtttatat ttgtaatttt agtattttgg gaggtttaga tgggaggatt atttgagttt 4200
aggagttcga gattagttta agtaatatag tgagatttta tttttattaa aaaaaaaaaa 4260
aaaaaaaaaa aagttaggta tgttggtatg tatttatagt gttaattatt taggaggttg 4320
aggtgggaga attatttgag tttaggagac gaacgttgta gtgagttatg attgtgttat 4380
tgtattttag tttagaagat agaatgagat tttgttttaa aaataaaaaa taaataaaag 4440

aaaaagaaaa aaaaattgtt atgatgtttt tttttttgaa atttttgttg aaagggatgt 4500
ttgatatttt taattaagat attattttta tatatagttg tatatttggt ttagtataat 4560
attttgtgta tttatataag tagttaatat atatatgtaa aaaattgggt tatttgtaaa 4620
tgggaataaa aaaataatag ttcgatttag ggtttaaaaa ataaggaagg taacgtttaa 4680
gtttttaatg agttgaaaaa taataaatat taatttgaat agaaatttta aaagttgata 4740
atttatttta ttagtttttt aaattttgta cgtatatata aatatatata attgaattga 4800
attaaattgt ggtttattta aacgttgggt tttttagttg tgatgagaga agaatttttt 4860
atttttttga gttttgtgat atgtagttgt tgaggttatt gttagttaga gtagttaata 4920
gggagatttt tttttttata aattaattag ttttattttt agtttttatt ttttgattaa 4980
tttttttttt attgtgaagt aaattaataa gtattgagat tgtagttaat ttgtgagggt 5040
tatttatttt ttgtgggtta tttatgatgt tatttttttt aggaaatatt ttgtgatgtt 5100
ttatttattg tgattatttt ttttttaggg ataatttagg gaatatttcg tttatttagt 5160
ttttttttaa aaaaaaatgt ttgttttatt ttaatatgga gtatattatt gttatttatt 5220
tgttttttag aattttattt taatgattgt aaagttttta aaagtataaa atattttttt 5280
tttaatgtag aattatattt cgtgttttat agataattag tatttagtgt gtgttttttg 5340
tttttaatag agagaaaaat aagaatatta gtttttttaa tttatttttt tgtttgtagt 5400
tttttataga tattaataga atttgtaatt atgttatatg tttatagaaa gtattaatag 5460
tatgttttga aaaaaataat tttttgaaaa ttataagggt aatacgtgtt aattataggt 5520
aggaaattta gatttttttt ttaaaggtag atttaaaaag gatttttaat tttattattt 5580
aaagataatt tttgttaaat tttggagtat gtttttgtag tattatttta attttttttt 5640
tataaatgaa gaatttgagg attaaagaga ttaagtattt gataaaggtt atatagttag 5700
taaaagatag agtttagatt tgaatttagt tttgtttagt tttagatttg ttttatattt 5760
tagaataaat tgattaggtg gttgattttc gttatttgat tagtgaagga ttttaggaaa 5820
tgttatttta aaatatgttg ttttggtata ttgattattt ttagttgaag gtatttaaag 5880
attagtaaat gtagggagga ggagagatat gtggaataga tgtggattaa ttttttgatt 5940
gaatttaagt tagattttta ttagttaatt ttaagtttat ttttagttaa tttatatgag 6000
tttgagtaag aataagtaaa tgtcgtttaa ggaaaaattt gggttggttt gttatttaga 6060
atttttgagg taatagttaa gagatataag ggatgtgaag ggtgaggaat tacggaaaag 6120
taaggttaaa taatttgtag gtggtaaaat tataaagggg tttttataga taaaaattat 6180
tatgaggttt gttatggggg ttgaagatga ggaagatagt aggattttaa tttagatttt 6240
taggattatt tataaattaa attgtatata tagttaaaga atatgggtcg ggtatgttgg 6300
tttatgtttg taattttagt attttgggag gtcgaggtag gtagattatg aggttaggat 6360
ttcgagatta gtttgattaa tattgtgaaa ttttgttttt attaaaaata cgaaaattag 6420
ttgagcgtgg tg 6432

<210> 219
<211> 7309
<212> DNA
<213> Artificial Sequence

<220>
<223> chemically treated genomic DNA (Homo sapiens)

<400> 219

atgcgattaa tggtgttaat tgttttttgg attgttttta tagtatagga taagattata 60
ttagttttag taggttttat tgaagaaagt ttatgtgttt tgtagaaata agatttttag 120
tttagtttaa taggagatta attatatgtt tattttattt ttttttaagg tatgatagta 180
tttggttgag atgtttgtgt gtattttttg tgtgaagttg agtatgtaat ttttaaagaa 240
attataaagg agttattcgt ttttaaagga aaaatatatg aattttacgg gtataaggaa 300
gatattaaaa tagggttggg gagaagtttt ttttttttta ttttatagta ttttttatag 360
aaataattga ttgtttgaat atatggttat tgaaatatat atatatatat atatatgtat 420
atgaaaataa gagttggaat atttgatttt gttttttatt ttttattatt ttagataata 480
taggatataa tatggtttta ttgtatatat gtgttttttg gtttaaaaat attgagagta 540
gatttttgtg gaaagaaata tgaaaatttg aggatttatg cgaatgtagt tagaggcgaa 600
tgtagttaga ggggttttta atttgttggt agagttgggg gagagttggg gtaattttat 660
tgttatgaaa ttggttttag atggaggtaa ggaaaggtgt ttattttttg ttggtgttag 720
atatagagag attttgtagt atttcgtatt ttaggtttag aattgtttag tgttgggtag 780
tagttttatt gagggtttgg ttagagttta tggggatggg tggattaggt tgtttgttgt 840
ttggaaaagg ttattttgaa tataatgatt tttatatgga cggtggggtt tgggggaagg 900
tatcgtggtt agggtttgga ggggaagagg gaggaaagta aagaggagtt tttttatgag 960
gtaatagaaa tgatagtatt ttttttaggt tgaattttta aaatttagtt ttgttttgga 1020
agattgggat tgtggtattt tttgtagtgt ttttagatcg ttttaaggtt tttaaattaa 1080

tgatttataa ttatgttgtt tttttagaga gtattttttt ttttggttga tatgtggtta 1140
gtttaggggt ggaggagttt cggtagttac gaatttagac gggatgttga ttgagagttt 1200
tgtggttatg gtagatggga ggggtgagtt ttatttattt aaaggtattc gtattttatc 1260
gatgagtgtg gaattgtgtt ttttcgtttt cgtagtgaga cgatcgatta ggattgtagg 1320
ttgagtaaat gggtgttttt tggtttatag cgttttttag tattagttag cgggatagag 1380
tttcgtgaat tttttaagga gttattattt gtttgtttga atgttggaaa ggggtcggtg 1440
gtgttgatta ttggatttat ttaattaata tttattaaat tcgttgttat taatatttaa 1500
tttttgtgtt ttaggggttt agattttagt aagggaaata gatgggtaaa taattatatt 1560
agagttagtt aagtattgtt aaagaagttg atatagggtg ttgtggggat atatgggagg 1620
ggaattagag gaggtttttc ggaggaggtg atatttaagt tgaggtttga aggatgtgga 1680
agagtttagt aatttttgaa gattaaaaaa atggtgataa aaaagatatt tttttaattt 1740
tttagtgtta cgatttaaga gataaataag taggtgaacg tataattata ttgggtttta 1800
ttataagtta gagtttgttt tttttaggat tttggtatta taattttttt tgaaattttg 1860
ttttcggtat tgatttttgg gggaggtagt aatataggaa tttagtgttc gttgcgaaat 1920
tgatggatgg tttttagttt tgggtacgtt tttatgtgtt aaatttgttt aaatggtagg 1980
atttattttt tttttagaag tatttatttg ttttatagga ttttttatgg aattttggag 2040
tttttgaggc gagagggatt ttggatatta ttgagtttta ttttttattt aataaatata 2100
gaagtggacg tttggatagg taaagtgatt tgattaaggt aggtgtatag ttattttgta 2160
atattgggaa taaattttag gttttttgat tttttgtttt tattttattt tttttttatt 2220
ttttagaaat aaagttttta tgtgtttttt tttatagtga tatgtttgga atgtattagt 2280
tagtaattta ggaaggaaa aaaataaata tataagagat aaatttgtta ggaggataaa 2340
tttgtattgt ttttgattgg tttagagggt gattattatt atggtagaga attatttaat 2400
tagtgtaagt aaaatttttt tgtgggttgg gtattgtata aagatttaaa cgaatttgtt 2460
tatagatttg aaaagtagat acgagatttg tgaatggttg gggtttttaa gtttatagta 2520
taagtatggg ttatatttta tagtttggag gattgagttt tgaaaatggg taagtttttt 2580
tatttttttg aattttattt tttttatatt taaaataagg atgagtagtt tttgaggttt 2640
tttttacgat tttttttttt atagatttta gtattttata atttgatata aagagggtgg 2700
atatgaattt atttttttta gaaaagtttt aggaaagaga atattaggtt attttagtag 2760
gtgtgtagat aggttagata gattttgaaa tttatttagt tttttttaga tgtataattt 2820
tattattgtt tttagttgtt aagagaaagt aggagagttt gtatttttat tttttttttt 2880
tttttttttt tttttggaga cggagtttta ttttattatt taggttagag tgtagtggta 2940
tgattttagt ttattgtaag tttcgttttt taggtttacg ttattttttt gttttagttt 3000
tttaagtaat tgggattata ggcgtttatt attatatttg gttaattttt tgtgttgtta 3060
gtatagacgg ggttttatta tgttagttag gatggtttcg attttttgat ttcgtgattc 3120
gtttattttg gttttttaaa gtgttgggat tataggcgtg agttatcgta tttagtttgt 3180
atttttattt ttattgttag ttttaggttt attttattta gtttattaag tgatgttgaa 3240
taattaattt ttatatatta ttaggtttat ggatattatg atatttagat tgatgggtgt 3300
ttgttgaagg gggtgatttt agtaggagga tttttttacg taaggattta tggagtttgt 3360
tgtttttttt ttttagggtg agaattaaat tgtttttata cggtgggtag aggggaattg 3420
atttaggttt ggaataagag agaatatttt aattgaaaag tttttggaat tcgttgaatt 3480
ttaagatatt gtgtggatta gtttaggata gggagtgaga agaaattaat taaaaggtaa 3540
tttcgttatt ttttagttgg aaaaaagatt agattatatt tgtgttttta taattaagta 3600
gttgttggaa aaaaacgttt tagatgtttt ttatgagaaa attgttgttt gaagtttagt 3660
agaagttatt tatttgatat ttatatttta ggtaaggttt ttcgttggag aaaatatcgg 3720
tattttggat aaaattgaaa tgtgaaaaga aagggaagag agggttttta ttatgtaaga 3780
tgtttattta aagtggattt ggtttggaaa gttttttaaa attttttata tgattgtgga 3840
ataagttatg tggggcgcgg ggataagcga attttttaaa ttttattacg tatgttttta 3900
tttaatttgg atttttagag tggtttttag ggtattttgt ttaggattta gttagttgtt 3960
ggttatattt atgtttttta gttttttgag attttatttg gttttgagag ggttaaaaag 4020
tagtgtggtt aaatatttta ggttttaaag tatttttatt gtggttgggg aagtaataga 4080
attatatttt ataaaataat gaaaatagtg ttagaaaaat atcgagagat agaaatattt 4140
ttacgagtta ggttatagtt agagtgaagg tagggaaggt ttttaaagtt gggtggaggg 4200
gataagttaa aaagatgtgg aaattggttt ttttttttta tggttaaagt gtttaaaggg 4260
gaaaaaggag ttttaaaaat gtttttggaa atattatttt ttacgaattt ttcggttttt 4320
gttgttttaa tgttatttgt ttgagatgta aatagaggag ttttgagaaa gaagttgaat 4380
ttgtattttt ttttgttttt atttgtttta aatttgtggt atttttaata ggatgaagcg 4440
gaagagaaag ggaaagagat aaaagtgtag aaagatggaa gattttagtt gtaaatggtt 4500
atttgtagtt agatggaata gttgttgacg tttagggaaa tgtatgtttt tttttagatg 4560
ggaaggagta gtggaaaggg gtgacgagtt tttggttggt tattaattat tttatttttt 4620
tgtgtcggtt ttttatttgg aaagtgggag tgatatttgt gtttgttttt tttatttata 4680
aagattattg tgagagttat aatacggtga gatatagaat tttgttttta aaaatataaa 4740
gtagaattaa gatgttaata ataaggatag taattgtgtt agttatttgt aattatttat 4800
tatagttagt cgtttaggat tttggatcgt ttttttggtt ttattatagt tttggattag 4860

tttattttta aattttttgt tgaagggtgg agttttgtta gttatgggta gggaattatt 4920
tttttttgtt tttttatttt ttgtttttta aatatgttta gggtttttgt atttgttgtt 4980
ttttttgttt ggtatttttt ttttgtggtt tgttttagag ttgatttttg tttttgttta 5040
ttttttagcg aggatggtat tttagggagt ttttttttta ttatcgtaga gagagtaggt 5100
tttttttagt tatgtttaat ttagaatttt gttttgtttt ttttatagtt ttagtattat 5160
agaaaattat tttgtgtatt tatggatgtt tacgggggta agggttttgt gttgtttaat 5220
ttagtatttt gaatcgtgtt tgttgaatga atatagaatt tcgtttgttt tgggagagta 5280
tagaaaatag ttttttatta tatattatag ttagttgtaa atagtagatg gttttttata 5340
ttttagagag taagaattag agagagagag aaagagagag agtttgggtt tttttttttt 5400
gtgtttgttt tttttagaga aattggaggg gtagtagtta gtattttttc gttggtttta 5460
ttaagtatag ttaaggtttt taggatatgg ttatttttta tttgtggaag cggttttgtt 5520
ggggtgggtg ggtgttagtt ggttttggtt tgggttagag atatttagtg gtttaggtgg 5580
gcgtggggtt agggcgtaga cgagaagggg tacgagggtt tcgtttcgag gatttagcgg 5640
taagtatcgg tttcgggcgc gttttagttt atttattcgc gtgtttacgg cggtattatt 5700
ttttataagg atttgaacga ttcgggggcg gtttcgtttc gttatttttt gttttcggtt 5760
tcgttttttt tttggagggt cgatgaggta atgcggtttt gttattggtt tgagggggcg 5820
ggttttaata gttcgaggcg gggttttcgg gggtttagcg ttatattatt cggtcgttta 5880
ggtagcggcg tagagcgggt agtaggtagg cggcgggcgt ttagacggtt tttttttttt 5940
tttttgtttt tttagttttt gttttttcgt cgggaggtcg ttcgtcgagt tttgcgttag 6000
cgtcgaggta gtttcgttgc gttttatttc gtttcgtcgg gtattcggag ggtagcgcgt 6060
cggaggttaa ggttgtttcg tacggttcgg cgggcgagcg agttcgggtt gtagtagttt 6120
cgtcggcggc gcgtacggta attttggaga ggcgagtagt agtttcggta gcggcggtag 6180
tagcggtaat gattttttgg ttcgggttta tcgtgttttt gggtagttgg agtttggggg 6240
attggggcgt cgaggcgtgt atatgttcgt ttagttattt ttaggacgtt ttttgtaatt 6300
tcgatatcgg taagcgtttt tggtgtttcg ttcgagtttt acgttgtagt taggattgta 6360
gcgttgttta gggaggtagg gcgagtttta tttttttttt ttgttttagg agaggggtag 6420
acggggttgg ggcggagtgg agaaattcga tgtttttggg cgggggcgtt ggtatagttg 6480
agaggggaag atgttttgta gaggaaattt atagtggttg agggagtttt tggtcgtttt 6540
tgttttttta atttaggtcg tgaggttttt atcggttttt ttgatatttg gaaaatgttt 6600
ttatttatta ttaacggagg aagggttaga agagaagggt ggggaaaggg tttttaaaat 6660
ttggaatgtt aattaaagtg ttgggaaatt gaatagttaa aaaaaaaaaa aaaagttgtt 6720
cgttatgtgt acggaaagtt gtaggagaaa tttggagtat ttcgagtttg tattattttt 6780
tgcgtttatg aaatttattt tttttattga gatttaattt agttgtaggt tttttaggta 6840
agcgttaatc gagtttttgg gattgttaag tttgggtgga tttaaggggg aaaatgagga 6900
gaaagttgtg gtattgattt acgttttgga ggtattgtgt ttgagagtgt tgatagttag 6960
tagaaggtgc gaggggttat ttattatata gttgtgaaaa tttataggaa ggttattttt 7020
tttttttttg aatgtgtgtg gggaggggga gaatgggtag tggaaaatag atttgttatt 7080
aaaagaataa atttgttaag ggttatatta cggtgatagt tttagattgg atttagtgtt 7140
ttgagtagag agattttgta aagagatttt ttatttttta gtgtgtatgg agggtttcga 7200
gagtattttg ggaggtttgt ttgggagtgt ttgggatatt tgtgtttgga tggttaaagg 7260
tggagtggta tttgggaaat attttttttt ttttttttag tttataaag 7309

<210> 220
<211> 7309
<212> DNA
<213> Artificial Sequence

<220>
<223> chemically treated genomic DNA (Homo sapiens)

<400> 220
   ttttatgggt tggaaaggag gagagagatg ttttttagat gttattttat ttttagttat 60
   ttaaatataa gtgttttaaa tatttttaga taagtttttt aggatgtttt cggggttttt 120
   tatatatatt aaggagtagg aggttttttt atagggtttt tttatttaag atattaagtt 180
   tagtttggaa ttgttatcgt gatgtgattt ttggtaggtt tgtttttttg gtgataaatt 240
   tgttttttat tatttatttt tttttttttt atatatattt aagggggaaa aaaatagttt 300
   ttttgtgaat ttttatagtt atgtggtgag tgatttttcg tattttttgt tggttattaa 360
   tatttttaaa tatagtgttt ttagggcgtg ggttaatgtt atagtttttt ttttattttt 420
   ttttttaaat ttatttaaat ttggtagttt tagaagttcg gttagcgttt gtttgggaag 480
   tttgtagttg ggttgggttt tagtgaaaag aatgaatttt ataggcgtag gagatgatgt 540
   agattcggag tattttaagt tttttttgta atttttcgtg tatatggcgg gtaatttttt 600
   tttttttttt taattattta attttttagt attttagtta gtattttaag ttttgggaat 660
   ttttttttta tttttttttt ttagtttttt tttcgttagt agtgaatggg gatatttttt 720
   agatgttaaa aggatcggta ggaattttac gatttaagtt aggaaagtaa aggcggttag 780
   gggttttttt agttattgtg agtttttttt gtagggtatt tttttttttt agttatgtta 840
   gcgttttcgt ttaaggatat cgagtttttt tatttcgttt taatttcgtt tgtttttttt 900
   ttggggtaga ggaaaggagt ggggttcgtt ttgttttttt aagtagcgtt gtagttttgg 960
   ttgtagcgtg gggttcgggc ggggtattag gagcgtttat cgatgtcgga gttgtagaag 1020
   gcgttttggg ggtggttggg cgagtatgtg tacgtttcgg cgttttagtt ttttaggttt 1080
   tagttgttta ggagtacgat gagttcgagt taaggggtta ttgtcgttgt tgtcgtcgtt 1140
   gtcggggttg ttgttcgttt ttttaaagtt gtcgtgcgcg tcgtcggcgg ggttgttgta 1200
   gttcgagttc gttcgttcgt cgggtcgtgc ggggtaattt tggttttcgg cgcgttgttt 1260
   ttcgagtgtt cggcgggacg ggatggggcg tagcgaggtt gtttcggcgt tggcgtagga 1320
   ttcggcgggc ggtttttcgg cgaaggagta ggagttggag gagtaagagg aggaggagaa 1380
   gtcgtttgag cgttcgtcgt ttgtttgttg ttcgttttgc gtcgttgttt gggcggtcga 1440
   gtgatatagc gttgggtttt cggggatttc gtttcgggtt gttggggttc gtttttttag 1500
   attaatggta gagtcgtatt attttatcgg ttttttaaaa agggggcggg gtcgggggta 1560
   aggggtaacg gggcggggtc gttttcggat cgtttagatt tttataggga ataatgtcgt 1620
   cgtgggtacg cgagtgggtg ggttggggcg cgttcgggat cggtgtttgt cgttgggttt 1680
   tcggagcgga gttttcgtgt tttttttcgt ttgcgttttg gttttacgtt tatttgggtt 1740
   attgggtgtt tttgatttaa attagaatta attaatattt atttatttta gtaggatcgt 1800
   ttttataggt gaggggtggt tatgttttag agattttgat tgtgtttggt ggaattagcg 1860
   ggggaatgtt aattgttatt tttttagttt ttttggagag agtaggtata gaggagaaag 1920
   atttaaattt tttttttttt tttttttttt tggtttttat tttttgggat atgggaagtt 1980
   atttgttgtt tgtagttggt tatgatatat gatagaagat tgttttttgt gtttttttag 2040
   agtaaacggg gttttgtatt tatttaataa atacggttta ggatgttggg ttaagtaata 2100
   taaagttttt gttttcgtgg atatttatga atgtataggg tgattttttg tgatgttagg 2160
   gttatgaaga aaataaaata gagttttggg ttggatatga ttggggaggg tttgtttttt 2220
   ttgcgatagt aagggaaggg ttttttgaag tgttattttc gttgagaagt ggataaagat 2280
   aaggattagt tttggggtaa gttataggag agaggtatta ggtaggggga atagtaagtg 2340
   taaagatttt gggtatgttt gaaagataga aagtagaaag gtaagaggaa gtggtttttt 2400
   gtttatggtt gatagagttt tattttttag taagggattt gggggtgagt tgatttaaaa 2460
   ttgtagtaaa attaggagaa cgatttagga ttttagacga ttagttataa tagatgattg 2520
   tagataatta atataattat tatttttatt attgatattt tgattttgtt ttgtattttt 2580
   aaaagtagga ttttgtattt tatcgtatta tagtttttat aataattttt gtgggtagga 2640
   aaagtaagta taagtattat ttttattttt tagatgagga atcggtatag aaagatggga 2700
   tgattggtgg ttagttagga attcgttatt tttttttatt gttttttttt atttgaagag 2760
   agatatgtat ttttttgaac gttagtagtt gttttattta attgtaaatg gttatttgta 2820
   gttgggattt tttatttttt tatatttttg tttttttttt tttttttttc gttttatttt 2880
   gttagaaatg ttataagttt ggaataaata gaaataggga gaaatgtaag tttagttttt 2940
   tttttagaat ttttttgttt atattttaga taagtgatat tgggatagta gaggtcgaag 3000
   aattcgtgag agatggtatt tttaagaata tttttgaaat tttttttttt tttttgagta 3060
   ttttagttat aggaaaggga aattagtttt tatatttttt tgatttgttt tttttattta 3120
   gttttaaaaa ttttttttgt ttttatttta attgtggttt aattcgtaga gatgtttttg 3180
   tttttcgatg tttttttagt attgttttta ttgttttatg gggtatgatt ttattgtttt 3240
   tttaattata gtaggaatat tttgaggttt gggatattta gttatattgt tttttagttt 3300
   ttttagaatt aaatagggtt ttaggagatt ggagagtatg ggtgtggtta atagttgatt 3360
   gagttttgag tagagtgttt tggaggttat tttaaggatt taggttgaat gagggtatac 3420
   gtggtggaat ttgagagatt cgtttatttt cgcgttttat atgatttatt ttatagttat 3480
   gtggaaggtt ttagaagatt ttttagatta aatttatttt ggataagtat tttatatgat 3540
   agaggttttt tttttttttt ttttttatat tttagttttg tttaaagtgt cgatattttt 3600
   tttaacggaa ggttttgttt ggaatataag tattaagtag ataatttttg ttgaatttta 3660
   agtagtagtt tttttataga aagtatttga agcgtttttt tttagtagtt atttaattat 3720
   gaaagtataa gtataatttg attttttttt tagttgaaaa gtaacgaaat tattttttgg 3780
   ttaatttttt tttatttttt attttaagtt ggtttatata gtgttttgaa gtttagcgaa 3840
   ttttaagagt tttttagttg ggatgttttt ttttatttta aatttgagtt agtttttttt 3900
   tgtttatcgt gtgaaggtag tttggttttt attttaagga aaagaaatag taaattttat 3960
   gaatttttgc gtaggggagt ttttttgtta gggttatttt ttttagtagg tatttattag 4020
   tttggatgtt atggtgttta tgagtttaat aatatgtaag aattggttat ttaatattat 4080
   ttaataagtt aggtggggtg aatttgaggt taatagtaag aatgaagatg taggttgggt 4140
   gcggtggttt acgtttgtaa ttttagtatt ttgagaggtt aaggtgggcg gattacgagg 4200
   ttaggagatc gagattattt tggttaatat ggtgaaattt cgtttgtatt aataatataa 4260
   aaaattagtt aggtgtggtg gtgggcgttt gtagttttag ttatttggga ggttgaggta 4320
   ggagaatggc gtgaatttgg gaggcggagt ttgtagtgag ttgagattat gttattgtat 4380
   tttagtttag gtgatggagt gagatttcgt ttttaaaaaa aaaaaaaaaa aaaaaaagaa 4440
   tgaagatgta ggtttttttg tttttttttg atagttaaga ataatgatag agttatatat 4500
   ttgggaagaa ttgagtaagt tttaagattt atttggtttg tttatatatt tattaggatg 4560
   atttggtatt tttttttttg gaattttttt aggaaaggtg agtttatatt tatttttttt 4620
   gtattaagtt ataggatgtt agagtttgta ggaagagaag tcgtaaaaag gattttagaa 4680
   attatttatt tttgttttaa atgtgggaaa aataaggttt agagaagtga aggaatttgt 4740
   ttatttttag ggtttagttt tttaagttgt aaggtgtggt ttatgtttgt attgtgggtt 4800
   tggaaatttt agttatttat agatttcgta tttgtttttt agatttgtag atagattcgt 4860
   ttgagttttt gtatagtgtt tagtttatag agaaatttta tttatattga ttaaataatt 4920
   ttttattatg ataataatta ttttttgagt taattagaag taatataggt ttgttttttt 4980
   gataggttta ttttttgtgt gtttattttt tttttttttt aaattattag ttaatgtatt 5040
   ttaaatatat tattataaaa aaaagtatat gaaaattttg tttttgggaa atgaaaagag 5100
   agtaaagtgg aaataaaaaa ttaaaagatt tgagatttgt ttttaatgtt gtagaatagt 5160
   tgtgtatttg ttttggttaa gttattttgt ttgtttaggc gtttattttt gtgtttattg 5220
   gatgaaagat agaatttagt ggtatttagg atttttttcg ttttaaaaat tttaagattt 5280
   tatggggaat tttgtaggat aagtgaatgt ttttgaaggg gagatgggtt ttgttattta 5340
   aataggtttg atatatggga acgtatttaa gattgaagat tatttattag tttcgtaacg 5400
   aatattgagt ttttatattg ttattttttt taagaattaa tgtcgaaaat agaattttag 5460
   agaagattgt gatgttaaag ttttaggaga gataggtttt ggtttatggt ggagtttagt 5520
   gtagttatgc gtttatttgt ttgtttgttt tttggatcgt agtattaaaa agttaggaag 5580
   gtgttttttt tattattatt tttttggttt ttaggagtta ttgaattttt ttatattttt 5640
   tagattttag tttagatgtt atttttttcg ggaaattttt tttgattttt tttttatgta 5700
   tttttataat attttgtatt aatttttttg ataatattta gttgatttta atgtgattat 5760
   ttatttattt gttttttttg ttggagttta agtttttaga atatagggat taagtgttgg 5820
   taataacgag tttaataagt attggttgaa tgggtttaat gattagtatt atcgattttt 5880
   ttttagtatt tagataggta ggtggtggtt ttttggagag tttacggagt tttatttcgt 5940
   tggttggtgt tagggggcgt tataagttag agaatattta tttgtttagt ttgtagtttt 6000
   ggtcggtcgt tttattgcga gggcgggaga gtataatttt atatttatcg gtggggtgcg 6060
   ggtgtttttg agtgggtgaa gtttattttt tttatttgtt atggttataa agtttttagt 6120
   taatatttcg tttgggttcg tggttgtcgg agttttttta tttttgagtt ggttatatgt 6180
   taattaaagg gggagatgtt ttttggggaa gtaatatagt tgtgaattat tggtttgggg 6240
   gttttagggc ggtttgggaa tattgtagag gatgttatag ttttagtttt ttagggtaga 6300
   gttgggtttt ggaaatttaa tttggagaag gtattgttat ttttgttgtt ttatagggaa 6360
   gttttttttt gttttttttt tttttttttt ttagatttta gttacgatgt ttttttttaa 6420
   gttttatcgt ttatgtgaaa gttattatat ttaaaatggt tttttttaag taatagatag 6480
   tttggtttat ttatttttat gagttttggt tagattttta gtggggttgt tgtttagtat 6540
   tgggtaattt tgagtttaag atgcgaggta ttgtaggatt tttttgtgtt tggtattagt 6600
   agggggtaaa tatttttttt tgtttttatt tggagttaat tttatggtag taaaattgtt 6660
   ttagtttttt tttagttttg ttagtaaatt gaagattttt ttggttatat tcgtttttgg 6720
   ttatattcgt atgaattttt aggtttttat attttttttt atagggattt atttttagtg 6780
   tttttaagtt agaaaatata tgtgtgtagt aaagttatgt tgtattttgt gttatttagg 6840
   gtggtgggaa atggaaagta gagttaaata ttttagtttt tgtttttatg tgtatgtatg 6900
   tgtgtgtatg tatgttttaa tgattatata tttaagtagt taattatttt tgtgaagggt 6960
   attatgaaat ggaaggagaa gaattttttt ttaattttgt tttaatgttt tttttatgtt 7020
   cgtggagttt atgtgttttt tttttaaaaa cgagtagttt ttttgtggtt tttttgaagg 7080
   ttgtatgttt agttttatat agagaatata tataagtatt ttagttaggt gttgttatgt 7140
   tttgggaggg aatgggatgg atatgtggtt ggttttttgt tagattgggt tggaggtttt 7200
   gtttttgtaa ggtatatgag ttttttttag tggaatttgt tggaattgat atggttttgt 7260
   tttgtgttgt ggggatagtt taaagagtag ttaatattat tgatcgtat 7309

<210> 221
<211> 15863
<212> DNA
<213> Artificial Sequence

<220>
<223> chemically treated genomic DNA (Homo sapiens)

<400> 221

agttttaatt tcgtaggttt aagcgatttt tttattttag ttttttaagt agttgggatt 60
ataggtttaa tttttttgat agagatagga tttggttgtg ttattaaggt tggttttaaa 120
tttttgggtt taagtatttt tttattttgg ttttttaaag tgttgggatt ataggtatga 180
gttattatat tagttataaa aattttttga atataaattt attggttagg agtagtgttt 240

acgtttgtaa ttttagtatt ttaggaggcg taaatgggtg gattatttga ggtcgggagt 300
ttgagattag tttggttaat ttggcgaaat tttattttta ttttaaatat tttaattagt 360
cgggtgcggt ggtgcgtgtt tgtaatttta gttatttagg aagttgaggt agtagaatcg 420
tttgaatcgg gatatagagg ttgtagtgag gtaagattat attattgttt tttagtttgg 480
gttatagagc gagattttgt tttaaaattt ttttttttta aagaaagtta aaaagaatat 540
aaattgatta tttgtattgt aagatatatt ttttaaaatg ttttaatatt ataattatta 600
atattataat tacgatttcg atgaattatt ttcgagagtt gaaaatttga tagtcgttat 660
tatcgtttta gaaagtagta taaaaaagaa agagtgttga tggaggagga gaaggtttag 720
tagagttttg agtgtttgag tgttttagcg tagggaggtg gagatagtga tttatgttgt 780
taagagatgt agtgaggcga atttagagaa tgatttaaga ggggttgggc gcggggttta 840
tgtttgtaat tttagtattt tgggaggtcg aggtaggagg attacgaggt taggagatcg 900
agattatggt gaaatttcgt ttttattaaa aatataaaaa attagttagg cgtggtggcg 960
ggcgtttgta gttttagtta ttcgggagag gttgaggtag gagaatggcg tgatttcggg 1020
aggtagagtt tgtagtgagt tgagatggcg ttattgtatt ttagttgggt gatagagcga 1080
gattttgttt taaaaaaaaa aaaaaaatat tttttttatg attttttaaa taaattttaa 1140
atgttttatt tataagaatt aatacgacgt ggttgttttt ttttgttttt atttttttgt 1200
tttttttatt tgttttagtt tggttatatt agtattttgg gtttttgttt ttgtgtttgt 1260
ttgtttgttt gtttgtttgt ttgtttgttt tgggatggag ttttattttg ttatttaggt 1320
tggagtgtag tgatatgatt ttggtttatt gtaattttta ttttttgggt ttaaatgatt 1380
tttttgtttt agttttttaa gtagttggga ttataggtat ttattattat atttagttaa 1440
tttttgtatt tttgtagaga tgaggttttg ttatgttggt taggttggtt ttaaattttt 1500
gattttaggt gatttgttta ttttagtttt ttaaagtgtt gggattatag gtgtgagtta 1560
ttgagtttag tttgttttgt tgttttttga gatggagttt tattttgtcg tttaggttgg 1620
agtgtagtgg tataattttg tttgattgta gtttttattt tttgagttta agagattttt 1680
ttgttttagt tatataagta gttgggatta taggcgcgtg ttattatatg cgtttaattt 1740
tggtattttt agtagcgatg gggttttgtt atattggtta ggttggtttc gaatttttgg 1800
ttttaagtga tttgtatatt tcggtttttt gagtagttgg gattataggc gtgagttatt 1860
atgtagggtt tttttggttt tttaacgtat aaagttattt tttagttttg ggtgtttata 1920
gtattattgt tagtttttgg aaagtttttt ttagaagttt ttatatgatt gattttttat 1980
ttttttttag gttttagttt aaatgttttt tttttagaga gttttttttt gattatttta 2040
tgtaatgtgt ttttttttag ttagtttttt tttattttgt gtattttttt tagagtattt 2100
attattattt tggttttgat gtttgttggt ttatatgttt gttgtttgtt ttttatatag 2160
aaagtaaatt agttttttat ttgttttgtt tattatttta ttttagtata gtgattaata 2220
tatattagta gttaattaat aaatagttat aagttaggta cggtgattta tatttgtgat 2280
tttcgtattt tgggaggtta aggtgggcgg attttttgag gttaggaatt tgatattagt 2340
ttgtttaata tggcgaaatt tcgtttttat taaaattata aaaattagtc gagtgtgatg 2400
gcgtgcgttt gtaatattag ttatttgtga ggttgaggta ggagaatcgt ttgaattcgg 2460
gaggtagagg ttgtagtgag tcgagattat attatagtat tttagtttgg gtgatagagt 2520
aagattttgt tttaaaaaaa taagaaatag aaatatatat atgttgttat aagaaattta 2580
ttatttttta gtaaaaaata tagattttta attaaagaaa ggggaaattt ttttttaaat 2640
aattttagaa ttcggggtag aaaacgttat atgtggaaat tcgttttgaa aaatagtttc 2700
gtttgtgaat aggaaatatt tggattatat tttttttatt tttacgaagg atttaaatgt 2760
gtagtattga tgaagaggta gttagtttta gtttttatta tttttgtata ttttatttgt 2820
ttttttttat ggtatagttt tgggggtagg ttataaattc gtaagatgtt attagtatta 2880
ttggttaaga tgtaggaatc gttaggagtt ttagaaatag gggagagtta gaaagttggt 2940
tagatttatg ttttttaagt gtagggttag ggttgagttt gtttttgggg taggtaagtt 3000
tttttgaatt tttgagggaa gtagaagata taaattgtta gataaaatgt aagtttagtt 3060
taaaagggtt acgtgtcgtt ttttttagtt ttggggtatt ttttttttag aaaattggat 3120
tgttttatag tgaaaatttc gggggtggtt agttttttgt ttcgttgtta tttttattat 3180
ttatagtttt ttaagaagtt tttaggttgg gtgttgaatt ttgattagga attattgaga 3240
aatcgaggta gttgggagaa gttgtagttt taagcgttga aaggaagatg ggggataata 3300
aatttgggtc gttaagtaaa gggggtagag gtttggagaa gtgggtttta ggattagagg 3360
atagatcgat tttatatttt atttttttag attttatatt ttattgttat tattatttac 3420
gtgttttttt cgttttttgt agcgggtttt ttagaggtat tttttatggt ttttttagat 3480
tttaattttg gttcgttcgt ttttttttta gaaaggtttt cgtttgtttt ttttgtagga 3540
aggtttgtat ttttagaaag tttttgtttt tcgattcgag gatttaattt attaggggaa 3600
ttaaattttg tttttagggg agtggagaga gaaattgggt ttttttttcg tagtttttgg 3660
gatatagttg agttagttat aggatttggg gataatcggg gcggattttt tttttcggga 3720
ggcggtggta ttagtttaga gttcgtattt ttatttatcg gggaagcgtg gggagaagga 3780
tgggttggag ttgggttttg gtttgaagga tagtagttcg gagttaacgg ttgagttttt 3840
aaagttttta tattgtagag gaagtatagc ggagattagt tttagttagg atggtttcga 3900
agtttttagg gattcgacgt agagttaaag aaatttattt gtgttttttt ttttttttgg 3960
gagtaggtag aagattttcg ggaggagagg cgaatagcgg acgttaattt ttttgaaagt 4020

attgtgtttt ttagtatcgc gggtcgttac gggttttttg ttgtcgcggg atttcggttt 4080
atttttcgat tgggtcgtcg tatttcggat tagatttcgc gggcgattta cggaattcgc 4140
ggagtcggga cgtgaaaggt tagaaggttt ttcgttttta ttaagtttta gggtttttcg 4200
tggttgttgg gagttgtagt ttgaacgttt ttattttggc gagaagcgtt tacgtttttt 4260
ttatcgagtt tcgcggtaat ttttaaagta tttgtatcgt tttttcgtcg tttgtagagg 4320
gcgtagtagg ttttgtattt tttttgtatt ttatttttta ggttttagat ttgttttttt 4380
tatttaaaaa atatttatta tcgagttttt atttgttatt tagtattgat ataggtattt 4440
aggaatataa taatgaataa gatagtagaa aaattttata tttttataag gtttacgttt 4500
ttatgtattg aaagtaatga ataaataaat tttattagag tgataagggt tgtgaaggag 4560
attaaataag atggtgtgat ataaagtatt tgggagaaaa cgttagggtg tgatattacg 4620
gaaagttttt ttaaaaaatg atattttaat tgatgagaag aaaggattta gttgagagta 4680
aacgtaaaag tttttttttt tttatttttt atatttgata taatgtagga tttttttaaa 4740
atgattttta ttaattttgt ttttatagtt ttggtttgta gaatttttta ttttaaaatg 4800
ttagtattta cggtattagg tcggcgagaa ttttgatttt gtattttttt ttttaatttt 4860
attttttttg tttttttcgg taggcggatt atttgttttt atttgttatg gcgattgttt 4920
agttttgtgt taggagtttc gtaggggttg atgggattgg ggtttttttt ttttatgtgt 4980
ttaagattgg cgttaaaagt tttgagtttt ttaaaagttt agagttatcg tttagggagt 5040
aggtagttgt tgggtttcgg ggatattttg cgttcgggtt gggagcgtgt tttttacgac 5100
ggtgatacgt ttttttggat tgggtaagtt tttgattgaa tttgatgagt tttttttgag 5160
ttacgggttt tcggtttcgt gtatttttag ttcgggaaaa tcgttggggt tgggggtggg 5220
gtagtgggga tttagcgagt ttgggggtga gtgggatgga agtttggtta gagggattat 5280
tataggagtt gtattgttgg gagatttggg tgtagatgat ggggatgtta ggattattcg 5340
aatttaaagt tgaacgttta ggtagaggag tggagttttg gggaattttg agtcggttta 5400
aagcgtattt ttttgtatat ttattcggtg ttgggcgtag ggaatttttg aaataaaaga 5460
tgtataaagt attgaggttt gagatttttg gatttcgaaa tattgagaat ttatagttgt 5520
atattttaga gtttatggta ttttagtgaa aattggggtt ttatttcgaa atgattattt 5580
gggggtgatt cggggagttt aagttgttaa ggttttataa ttttcggatt tttgtttttt 5640
ttggagcgat ttttttaggt agttttcggt ttcgttagat ggagaaaatt taattgaagg 5700
ttgttagtcg tggaagtgag aagtgttaaa ttaggggttt gttcgttagg tcgaggagga 5760
tcgtcgtaat ttgagaggtt cggtagtttt gttattgttt ggttttatat ttatattttt 5820
gttttttgta gtagtatttt cggttttttt ttgtcggagt agtttattat ttattcgatg 5880
agaggggagg agagagagag aaaatgtttt ttaggtcggt tttttttatt tggtagaggg 5940
aggttgttat ttttcgtttg tatttttttt tttggattat ttagttatgg tttttgtaaa 6000
ggtaggggta tttgttttga tgtaaatttt aatttttttt tttttttgaa tggtgtgttt 6060
tatttttcgg gtcgtttgta atttaggcgg acgttattat ggcgtagata gggagggaaa 6120
gaagtgtgta gaaggtaagt tcggaggtat ttttaagaat gagtatattt tattttttcg 6180
gagaaaaaaa aaaaagaatg gtacgtttga gaatgaaatt ttgaaagagt gtaatgatgg 6240
gtcgtttgat aatttgtcgg gaaaaataat ttatttgtta tttagttttg ggttaggtta 6300
ttttagtttt agacgtaggt tgaacgtcgt gaagcggaag gggcgggttc gtaggcgttc 6360
gtgtggtttt tcgtgtagtt ttcggttcga gtcggttttt tttggtagga ggcggaattc 6420
gaatttattt ttttcgttgt tttatttttt agttcgcggt tgttttattt cgtagttttt 6480
ttttatgtat ttgtcgcgta tcggttattt tgtgtcgtat ttacgttatt ttttttttaa 6540
atcgaggtgg tatttatata tagcgttagt gtatatagta agtgtatagg aagatgagtt 6600
ttggttttta atcgtttcgt gatgtttatt aagttataga ttttttttat cgttttagaa 6660
acgttttatt acgttttttt ttagtcgatt ttcgatttta tttttatttt gatttttata 6720
attattttgt ttgttggaga attttatata gaatggaatt aggatgggcg ttgtggttta 6780
cgtttgtatt ttggtttacg tttgtatttt gggaggtcga ggcggcggga ttatttgagg 6840
ataggagttt tagattagcg tggttaacgt ggtgaatttt cgtttttatt aaaaaatata 6900
aaaattagtt gggcgtggtg ggtgtttgta atttagttta ttcgggaggg tgaggtagga 6960
gaatcgtttg aattcgggag gtagaggttg tagtgagtta agatcgtgtt attatatttt 7020
agtttgggcg ataagaacga aatttcgttt taaaaaaaag gggggaatta tatattatgt 7080
gtttattttt gtcgggtttt tgttttttaa tgtattgttt gatattcgtt tatgttgtat 7140
atattagtat tttgtttttt tttatttagt atagtttatc gattgtatat tcgttttttt 7200
gatggttttt tgagttgttt tttatttgcg gttatgaaat aaagttgtta taaatatttt 7260
tgtataattt tttttgtgat tatatgtttt cgtgtttttt ggagaaatat ttaggagggg 7320
aattgtggag gaagtaaaaa gtagttgtat tttgaatttt tttagaagtt ttgagttttt 7380
tagagcggtt gtattatttt atattttaat tagtaaggta tgggagttat tatggttgtg 7440
ttatagtttt tcggatatta ggtatgttag tttttttaat gtggtatatt tttgtggttg 7500
taatttatag ttttttattg attaaggatg tttagtattt ttttatgtgt ttattggtta 7560
ttcgtatttt gtttgtaaag tagtttttcg agttttttat ttgttatttt ggtttttttg 7620
tttgttttta ttgtttagtt gtgggattgt tttatatttt ttggatataa gtttttatta 7680
gatttatgag tcgtgaatgt ttttttttga tttgttgcgg gtttatttgt ttgttttata 7740
gagtttatag aattttaaga ggagtggatt aatttttttt atgtttagta tttgttttgt 7800

tttgtttagg atattttttt tttttttttt aattttaggg ttatgaagat ataattttat 7860
attttttttt aggattttta tggtggtaag ttttatagta aggtttttaa gttattaatt 7920
aatttttaaa attaattgtt tatggtgtga ggtgtaggag ttagtttttg gtattttttt 7980
tgtatggaaa tttagttatt ttgtttttat ttgttgaaat aggttttttt tttttattga 8040
atgtttttaa ttttaattat tttatagttg gagtataggg ttattatttt agtgttattt 8100
tttttttttt tttgttaatt tttgagatag ggatttatat tgttgtttag gttagagtat 8160
aatggtataa ttaaggttta ttgtagtttc gaatttttgg gtttaagtag ttttttagta 8220
gttttacgag tagttgggat tattttatta tatttagtta attattttat ttttttgtat 8280
tgataggatt ttattatgtt gtttaggttg gttttaaatt gttggtttta agtttttatt 8340
ttatttcggt tttttaaagt gttgggatta taggtgtgag ttattatgtt tgatttttta 8400
gtgttatttt ttatttattt tttttgtttt ttgttttttt taaacgttgg aggaagaaat 8460
agtatttatt ttatataaat tttttagaaa atagaggaat agattgggcg cggtggttta 8520
tatttgtaat tttagtattt tggtacgttg aggtagggga ttatttgagg tcgggagttc 8580
gagattagtt tggttaatac ggcgaaattt tatttttatt aaaatataaa agtagttagg 8640
cgtgtattat atttgtaatg ttagttattt aggaggttga ggtataagaa ttttttgaat 8700
ttgggaagcg gaggttgtag tgagtcgaga ttgcgttatt gtattttagt ttgggtaata 8760
gagtgagatt ttgttttaga aaaaaaaaga aagaaagaaa aaatagagga atatttttta 8820
atttgttttc gaagttagga taattttggt attaaaatta aataaggata ttataagaaa 8880
agaaaatata gattaatatt tttgttagta tagatatgta atagttaatt aattttagta 8940
aattaaattt ggtaatatag aaaaaaggat aaataggtta gtcgcggtgg tttacgtttg 9000
taattttagt attttgggag gttgaggtag gtagattatt tgaggttagg agtttgagat 9060
tagtttgatt aatatggtga aatttcgttt ttaataaaaa tataaaaatt aggttgggta 9120
cggtggttta cgtttgtaat tttagtattt tgggaggtcg aggtgggtag attacgaggt 9180
taggagttta agattagttt gattaatgtg gtgaaacgtt atttttatta aaaatacgaa 9240
aattagtcgg tgtggtggta tttgtttgta attttagtta tttaggaggt tgaggtagaa 9300
ttgtttgaat tcgggaggta gaggttgtag tgagttaaga tcgtgttatt gtattttagt 9360
ttgggcgata gagtaagatt ttattttaaa aaaaaaaaaa attagttggg tatggtggtg 9420
ggtatttgaa attttagtta ttcgggagtt tgaggtagga gaatcgtttg aatttaggag 9480
gtagaagttg tattgagttg ggattatatt attgtatttt agtttgggta atagagtgag 9540
attttatttt aaaaaaagaa aaagaaaaag gataaatata ttttaattaa ataatgttta 9600
ttttatgatt gtagttgatt taatatttaa aaattggttt ggtgtagtag tttaggtttg 9660
taattttaat attttaggag gttgaggtag gaagattttt tgagtttagg attttaagat 9720
tagtttgggt aatatagtta gattggtttt tattgggggg aaaaaaatta gtttgtgtaa 9780
tttattatat taataaaggg aaatataaaa attttatgat tattttaata gatgtagtaa 9840
aagtagttaa tgatattaat atatatgtat gattataaat taattaattt tttagtaaat 9900
tagggaaagg aaatttaatt agtttgataa tagggcgttt atagtcggag ttttattagt 9960
agtatatata atggtagaaa atttagtgtt gttgggggcg gtggtttacg tttgtaatgt 10020
tagcgttttg ggaggtttag gcgggcggat tacgaggtta ggagatcgag attgttttga 10080
ttagtatgtt gaaatttcgt ttttattaaa aatataaaaa taaaaaatta gtcgggtatg 10140
gtggcgggcg tttatagtgt tagttattcg ggaggttgag gcgagagaat ggcgtgaatt 10200
cgggaggcgg agtttgtaga gtttagatcg tgttattgta ttttagtttg ggtgatagag 10260
tgagatttcg ttttaaaaaa aaaaaaaaaa aaaaaagaaa agaaaattta acgttttttt 10320
ttttaagatt aggaattaga aaaggatttg atttttataa cgttgatatt atattggagg 10380
ttttaattag gtaagaaaaa gaaataatga gggtcgggtg cggtggttta ggtttgtaat 10440
tttagtattt tgggaagtcg agacgggtgg attacgaggt taggagatcg agttattttg 10500
gttaatacgg tgaaattttg tttttattaa atatataaaa aattagtcgg gcgtggtggc 10560
gggcgtttgt agttttagtt attcgggagg ttgaggtagg agaatggcgt gaatttaggg 10620
ggcggagttt gtagtgagtt gagatcgagt tattgtattt tagtttgggc gatagagtaa 10680
gattgtgttt taaaaaaaaa aaaagaaaaa gaaataatga ttagtggttc gatgttttac 10740
gttagtaatt ttagtatttt gggaggtcga ggtgggtaga ttatttgagg tttggagttg 10800
gagattagtt tgataaagat ggtgaaattt cgtttttatt aaaatattaa aaaaatagtt 10860
aggcgttggt cgggtatagt ggtttatgtt tgtaatttta gtattttggg aggtcgaggt 10920
gggtggatta tttgaggtta ggagtttaat attagtttgg ttaatatggt gaaattttat 10980
ttttattaaa aatataaaat tagtcgggcg tagtggcggg cgtttgtaat tttagttatt 11040
tgggaggttt aggtaggaga atcgtttgaa tttgggaggc ggaggttgta gtgagtcgag 11100
attgtattat tgtattttag tttgggtgat aaaagtaaaa atttcgtttt aaaaaaaaaa 11160
gaattagtta ggggtagtgg tgaacgtttg tagttttagt tatttaggag gtagaggtag 11220
gagaattatt tgaatttcgg aggtagaggt tgtagtgagt cgagattgtt ttattgtatt 11280
ttagtttagg cgagaagagt aaaattttat gttaaaaaaa aaaaaaaaaa aggaaagaaa 11340
aaaaataacg attagaaagg aagaaattaa atatatttat agttagtatg attttatata 11400
tattatggtt ttaatggggt taggcgtggt ggtttatgtt gtaattttag tatttttagg 11460
aggttgaggt aggtggtttt tttgggatta gttggttaat atggtgaaat tttaatttta 11520
ataaaaatat aaaaaattag ttaggcgtgg tgagggtatt tttaatttta gttatttagg 11580

aggttgaggt aggagaattg tttggatttg ggaggtagag gttgtagtga gtcgagatcg 11640
cgttattgta ttttagtttg ggtaataaga gtgaaatttc ggtagggtgt ggttttacgt 11700
ttgtaatttt agtatttcgg gaggttgagt taggtcgatt atttgaggtt aggagtttga 11760
gattaattta atatggtgaa atttcgtttt tattaaaaat ataagaatta gttgggtgta 11820
gtggtgggcg tttgtaattt tagttatttg ggaggttgag atagaagaat tgtttgaatt 11880
taggaggtgg aggttgtagt gagttgagat tatgttattg tatattacgt cgggtaatag 11940
agcgagattt cgttttaaaa aaaaaaaaaa gatgaaattt tattttaaaa aaaaaaaaaa 12000
gttttaatgg aaaatttata aaaagttatt aaaattaata aataaatata gtagggttgt 12060
aggttatagg gtaatatagt tattttttta tttgtagggg tttggttttg ggatttttta 12120
tatattaaat ttatagatgt ttaagtttta tatataagac ggaatagtat ttaatttata 12180
tatatttttt tatatagttt aaattattta gattatttat attattttta tataatgaaa 12240
atgttaatgt atatgtaagt atgtatgtaa gtatttgtat tatattgttt agggaattat 12300
tggatagata ggtttttaag attgatatta gtagttattg ttaagatttt ggttaggttt 12360
gtttttgttt ggggttttag ttgattttat tgttttttta tttagttaag ggtatttgta 12420
ttttttttgg ttttttggtt atttggaagg tttagtttag tttggtatat ttgtattttg 12480
gtttattgat gttggtattt ttgggaaggt tttgttttga aaaatacgga gattttagtt 12540
gttattgaag atttgagaga taaagatagg gagatttgtt tgtagatttg tgttttttta 12600
agtgggattg agattttggg ttttttattt taggatagta ttttttggtt tgttgattga 12660
atagattttt gaaggaggtg tagttgtatt ttaggagtgg gggtgggagt agtattattg 12720
attcgtatta ataattatat agtttttttt agaataataa tatagaataa gtgaaataga 12780
ataattgtag aaagagttaa tttttgttga gtttttattg tgtgtttagt atttttttta 12840
attttatatt ttttataata tatagagtat taggtaggcg gggtttgggg gtttacgttt 12900
gtaattttag tattttagga ggttaagggg ggtggattat ttgaggtcgg gagtttaaga 12960
ttagtttgat taatatggtg aaatttcgtt tttattagaa gtataaaatt agttaggtgt 13020
ggtggtatat gtttgtagtt ttagttattt agtaggttga ggtaggagaa ttatttgaat 13080
tcgggaggag gttgtagtaa gcggagatag tgttattgta ttttagtttg ggtaataaga 13140
gttgagattt cgttttaaaa taaaataaaa taaaataaaa taaaataaaa taaaataaaa 13200
aaagaaaaga gtttgttatt aaaggagttg tttggtaggg gatgttttgt tagtgtaaat 13260
aatagaaaag tgggttgggt atagtggttt atgtttgtaa ttttagtatt ttgggaggtt 13320
aaggcgggcg gattatttga agttgggagt ttaagattag tttgattaat atggagaaat 13380
ttcgttttta ttaaaaatat aaaattagtc gggcgtagtg gtcgatgttt gtaattttag 13440
ttattcggga ggttgaggta ggagaatcgt ttgaatttgg gaggtagagg ttgcggtgag 13500
tcgagatcgt attattgtat tttagtttgg acgagagtaa aattttgttt taaaaaaaaa 13560
aaaaaataga aaagtgtaat aaatatttat agtaggtatg ttttttagta aatttgatga 13620
taaatttggt ataaagaaag agagtatttt tgaaaaaaaa aaaaagaaaa agaaagagag 13680
tattttgttt gggtaatata gtgaaatttt gtttttataa aaaaatttaa aaattggtcg 13740
ggtgtagtgg tttatatttg taattttagt attttgggag tcggaggcgg gaggattatt 13800
tgaggttagg agttcgaaat tagtttggtt aatatggtaa aattttattt ttattaaaaa 13860
tataaaaaat taattaggcg tattggtggg cgtttgtaat tttagttatt taggaagttg 13920
aggtaagagg atcgtttgat attgggaggt ggaggttata gtgagtcgag attatattat 13980
tgtattttag tttgggtgat agggcgagat ttcgttttta aaaaaaaaaa gaaaaagaaa 14040
aagattaaaa aattagttag gtaggttttt gtggttttag ttatttggga ggttgaggta 14100
ggagaattat tgagtttagg agtggtaggt tgtagtgagt tatgattgta ttattgtatt 14160
ttagtttggg ttttaaagta agattttgtt ttaaaagaaa aaagaaagaa agaaagaata 14220
tggcgggtta ggtatagtgg tttatatttg taattttagc gttttgagag gtcgaggtag 14280
gtggattata aggttaggag ttttatatta gtttggttaa tatggtgaaa ttttgttttt 14340
attaaaaata taaaaaatta gtaggtaggg tggtaggggt ttgtaatttt agttattcgg 14400
gaggttgagg taggagaatt gtttgaaatt agaaggtaga ggttgtagtg agtttagatt 14460
gtattattgt attttagttt gggcgaaaag agttaaattt tattttaaaa aataaataaa 14520
aaaataaaat aaaaaaaaat atggtagttt ttgaaagttt gtttgggaga aggtgcgatg 14580
atggttgtat aatttcgtgt aagatgttgg tttatatagg ggttgttttt tgtttttttt 14640
cgttttttta attttttata taataggttt gtgtgttatg tatatttatt gagtttaagt 14700
aggtgtaagg tattgtgatt taatattttg gttagtaaga taataagata gattattgtt 14760
ttgtttttag gaagtgtata tgttattaga ggaaatagat aaaataaata aggaaaagta 14820
ttagataatg taagtgttat gagaatgtaa atgaggtgat gtgaattaaa ataggatgat 14880
ttaagtttgt acggaaggtt tttattttta tgtttttggt tagttaagga attattagtt 14940
gattagtaga gaagggtagt tcgtttagtt agagtttttg gggaagaggg agtggttgtt 15000
aagagatgag attaaagaag tcgagacggg tttttcgtga gggggggttg taatgtaggg 15060
ttgaggagtg ttcgaagaga atgggtaggt gagcggtgag atagttgttt ttttagaagt 15120
tttgtagtga aaggaattaa agaaatggag tcgtgtatta ggtggggaag ggtgggggtt 15180
aagggggtgt ttttttttat atagagattg taggttgaga atgattatat ttttgttaat 15240
aggaggtggg agtagggtac ggtagtttat atttgtaatt ttggtatttt aggaggcgga 15300
ggcgggtcga ttatttgaag taaggagttc gagattagtt tggttaatat gtaaagtttt 15360

gtttttatta aaaatataaa aattagttgg gtgtggtggt attcgtttgt aattttagtt 15420
attcgggaga ttgaggtagg agaatggttt gaattcggaa ggtagaggtt gtagtgagtt 15480
gagattatgt tattgtgttt tagtttaggt gatagagaga gattttattt taaaaaaaaa 15540
aaaaaatata ggaagggagt tgggaatagg gtgtatattt aggaagtttt ggggatttag 15600
tggtgggaag gttggaagtt tttttttgat tgtttttttt ttaaagaagt gtatggttgg 15660
tgtggggtgg ggtaggagtg tttgggttgt ggtgaaatat tggaagagag aatgtgaagt 15720
agttattttt tttttgtttt ataggaagtc gagttgtttt agatattggt atggtgttgg 15780
gggagggggt tttttttttg taggtttagg tgatttaggg ttggaagtgt tttatgttgg 15840
attttattt tttttttgt agt 15863

<210> 222
<211> 15863
<212> DNA
<213> Artificial Sequence

<220>
<223> chemically treated genomic DNA (Homo sapiens)

<400> 222

gttgtaagag gaaaagtggg gatttagtat gagatatttt taattttggg ttatttgggt 60
ttgtagagaa ggaatttttt tttttaatat tatgttagtg tttgagatag ttcggttttt 120
tgtggagtag gaaaagaatg gttgttttat attttttttt ttaatgtttt attataattt 180
aagtattttt gttttatttt atattagtta tgtatttttt tgaggaaaag ataattagag 240
agggattttt aatttttta ttattaaatt tttaagattt tttaaatgtg tatttatttt 300
ttaatttttt ttttgtattt tttttttttt ttgagatgga gttttttttt gttatttagg 360
ttggagtata gtggtatgat tttagtttat tgtaattttt atttttcggg tttaagttat 420
ttttttgttt tagtttttcg agtagttggg attataggcg agtattatta tatttagtta 480
atttttgtat ttttagtaga gatagggttt tgtatgttgg ttaggttggt ttcgaatttt 540
ttattttagg tgatcggttc gttttcgttt tttaaagtgt taagattata ggtgtgagtt 600
atcgtgtttt gtttttattt tttgttaata aggatatagt tatttttagt ttgtaatttt 660
tgtatgggga aggatatttt tttggttttt attttttttt atttgatata cggttttatt 720
tttttgattt tttttattgt aaagtttttg gaagaataat tgttttatcg tttatttgtt 780
tatttttttc ggatattttt tagttttgta ttataatttt tttttacgaa gggttcgttt 840
cggttttttt aattttattt tttaataatt attttttttt ttttaaaagt tttagttaga 900
cgggttgttt ttttttgtta attaattggt ggttttttgg ttagttagga atatgggggt 960
aggggttttt cgtgtagatt taagttattt tattttaatt tatattattt tatttgtatt 1020
tttatagtat ttatattgtt tgatattttt ttttgtttat tttatttgtt ttttttaata 1080
gtatatatat tttttaaggg tagggtagtg atttattttg ttgttttgtt gattaaagta 1140
ttagattata atgttttgta tttgtttggg tttaataaat gtgtataata tataagtttg 1200
ttatatgaga ggttaagaga gcgagaaaga gtaaggggta gtttttgtgt ggattagtat 1260
tttgtacgaa gttatgtaat tattatcgta ttttttttta gataagtttt taaaggttgt 1320
tatgtttttt tttgttttgt ttttttgttt gttttttgag atggagtttg gtttttttcg 1380
tttaggttgg agtgtagtgg tgtagtttag gtttattgta atttttgttt tttggtttta 1440
agtaattttt ttgttttagt ttttcgagta gttgggatta taggttttta ttattttgtt 1500
tgttgatttt ttgtattttt agtagagata gggttttatt atgttggtta ggttggtgtg 1560
gaatttttga ttttgtgatt tatttgtttc ggttttttaa agcgttggga ttataggtgt 1620
gagttattgt gtttggttcg ttatgttttt tttttttttt tttttttttt tgaggtaggg 1680
ttttgttttg aagtttaagt tagggtatag tggtgtaatt atggtttatt atagtttgtt 1740
atttttgggt ttagtgattt ttttgtttta gttttttaag tagttgggat tatagaggtt 1800
tgtttggtta attttttagt tttttttttt tttttttttt ttttggagac ggagtttcgt 1860
tttgttattt aggttagagt gtagtggtgt gatttcgatt tattgtaatt tttatttttt 1920
agtattaagc gatttttttg ttttaatttt ttgagtagtt gggattatag gcgtttatta 1980
atgcgtttga ttaatttttt gtatttttag tagagatggg gttttgttat gttggttagg 2040
ttggtttcga atttttgatt ttaggtgatt ttttcgtttt cgatttttaa agtgttggga 2100
ttataggtgt gagttattgt attcggttaa tttttgagtt tttttgtaga ggtagggttt 2160
tattatgttg tttaggtagg atgttttttt tttttttttt tttttttttt ttagggatgt 2220
tttttttttt tatgttaaat ttgttattag atttgttaag aaatatgttt attgtaagtg 2280
tttgttatat ttttttgttt tttttttttt ttgagataga gttttgtttt cgtttaggtt 2340
ggagtgtaat ggtgcgattt cggtttatcg taatttttgt tttttaggtt taagcgattt 2400
ttttgtttta gtttttcgag tagttgggat tataggtatc ggttattgcg ttcggttaat 2460
tttgtatttt tagtagagac ggggtttttt tatattggtt aggttggttt tgaattttta 2520
attttaggtg attcgttcgt tttggttttt taaagtgttg ggattatagg tatgaattat 2580

tgtgtttagt ttattttttt gttgtttgta ttgataaaat atttttattt aaatagtttt 2640
tttaatggta ggtttttttt tttttttatt ttattttatt ttattttatt ttattttatt 2700
ttattttgag acggagtttt agtttttatt gtttaggttg gagtatagtg gtattatttt 2760
cgtttattgt aatttttttt cggatttaaa tgattttttt gttttagttt gttgagtagt 2820
taggattata agtatgtgtt attatatttg gttaattttg tatttttagt agagacgggg 2880
ttttattatg ttagttaggt tggttttgaa ttttcgattt taggtgattt attttttttg 2940
gttttttaaa gtgttgggat tataggcgtg agtttttaag tttcgtttat ttagtatttt 3000
atgtattatg ggaaatgtag agttgaggaa agtgttgggt atatagtaag agtttaataa 3060
aggttagttt tttttgtaat tgttttattt tatttgtttt atattattat tttagagaga 3120
attgtgtgat tgttagtgcg gattagtggt attgttttta tttttatttt taaaatgtaa 3180
ttatattttt ttttaggatt tatttagtta ataggttagg aggtgttgtt ttgaaatggg 3240
gggtttaaag ttttaatttt atttggaggg atataggttt atagataggt ttttttgttt 3300
ttatttttta aatttttagt agtaattaaa attttcgtgt tttttagagt aggatttttt 3360
taggggtatt agtattagtg ggttaggata taaatgtgtt aggttgaatt aggtttttta 3420
aatggttagg gagttaagag aaatgtaggt gtttttggtt gggtgggaag gtaatgagat 3480
taattgagat tttaaatagg ggtaggtttg attagaattt taatagtggt tgttggtatt 3540
agttttgaag gtttatttgt ttagtgattt tttaaataat atagtataag tatttatata 3600
tatatttgta tgtatattag tatttttatt gtatgggggt aatgtaagta atttagataa 3660
tttaaattat atgggaggat atgtgtaggt taaatattat ttcgttttat atatgggatt 3720
tagatatttg tgggtttggt gtgtgaggag ttttagaatt aagtttttat agatagaggg 3780
ataattatat tgttttgtaa tttgtaattt tgttatattt atttattagt tttggtagtt 3840
ttttatggat tttttattag gatttttttt tttttttgag atagagtttt attttttttt 3900
tttttttgag acggaatttc gttttgttgt tcggcgtggt gtgtaatggt atgattttag 3960
tttattgtaa tttttatttt ttgggtttaa gtaatttttt tgttttagtt ttttaagtag 4020
ttgggattat aggcgtttat tattatattt agttaatttt tgtattttta gtagagacgg 4080
ggttttatta tgttaggttg gttttaaatt tttgatttta ggtgatcggt ttggtttagt 4140
ttttcgaagt gttgggatta taggcgtaag attatatttt gtcggagttt tatttttgtt 4200
gtttaggttg gagtgtaata gcgcgatttc ggtttattgt aatttttgtt ttttaggttt 4260
aagtaatttt tttgttttag ttttttgagt agttgggatt agaggtgttt ttattacgtt 4320
tggttgattt tttgtatttt tattagagtt ggggttttat tatgttggtt agttggtttt 4380
agggaagtta tttgttttag ttttttaaaa gtgttaggat tatagtatga gttattacgt 4440
ttggttttat taggattatg gtatgtatag aattatattg gttgtgaatg tgtttgattt 4500
tttttttttt aatcgttatt tttttttttt tttttttttt tttttttttt gatatggaat 4560
tttgtttttt tcgtttaggt tggagtgtaa tgggataatt tcggtttatt gtaatttttg 4620
ttttcggggt ttaagtgatt tttttgtttt tgttttttga gtagttggga ttataggcgt 4680
ttattattat ttttggttaa tttttttttt tttgagacgg agtttttgtt tttgttattt 4740
aggttggagt gtaatggtgt aatttcggtt tattataatt ttcgtttttt aggtttaagc 4800
gatttttttg tttaagtttt ttaagtagtt gggattatag gcgttcgtta ttacgttcgg 4860
ttaattttgt atttttagta gagatggggt tttattatgt tggttaggtt ggtgttgaat 4920
ttttgatttt aggtgattta tttatttcgg ttttttaaag tgttggggtt ataggtatga 4980
gttattgtat tcggttaacg tttggttatt tttttaatat tttaatagag acgaggtttt 5040
attatttttg ttaggttggt ttttaatttt agattttagg tgatttgttt atttcggttt 5100
tttaaagtgt tgggattatt ggcgtgagat atcgggttat taattattat tttttttttt 5160
tttttttttt ttgagatata gttttgtttt gtcgtttagg ttggagtgta gtggttcgat 5220
tttagtttat tgtaagtttc gttttttgag tttacgttat ttttttgttt tagtttttcg 5280
agtagttggg attataggcg ttcgttatta cgttcggtta attttttgta tatttagtag 5340
agatagggtt ttatcgtgtt agttaggatg gttcgatttt ttgatttcgt gatttattcg 5400
tttcggtttt ttaaagtgtt gggattatag gtttgagtta tcgtattcgg tttttattat 5460
tttttttttt tgtttggtta aaatttttag tatggtatta acgttgtgag agttaaattt 5520
ttttttagtt tttgatttta gaggaaaaag cgttgagttt tttttttttt tttttttttt 5580
tttttttttg agacgaagtt ttattttgtt atttaggttg gagtgtagtg gtacgattta 5640
ggttttgtaa gtttcgtttt tcgggtttac gttatttttt cgttttagtt tttcgagtag 5700
ttggtattat aggcgttcgt tattatgttc ggttaatttt ttgtttttgt atttttagta 5760
gagacggggt tttagtatgt tagttaggat agtttcgatt ttttgatttc gtgattcgtt 5820
cgtttaggtt ttttaaagcg ttggtattat aggcgtgagt tatcgttttt agtagtattg 5880
agttttttat tattatgtat gttgttagtg gaatttcgat tgtggacgtt ttgttattaa 5940
attagttaag tttttttttt ttagtttgtt aggaggttgg ttggtttgta attatgtata 6000
tgtgttgata ttattaattg tttttgttat atttgttgaa atgattatag ggtttttatg 6060
tttttttttg ttaatgtggt gaattatata gattgatttt ttttttttta gtaaagatta 6120
gtttgattat gttgtttagg ttggttttga aattttgggt ttaagagatt tttttgtttt 6180
agttttttaa aatgttggga ttataggttt gagttattgt attaggttaa tttttgaatg 6240
ttgaattagt tataattatg agataaatat tatttggtta gaatgtattt attttttttt 6300
tttttttttt tttgagatgg agttttattt tgttgtttag gttggagtgt aatggtgtga 6360

ttttagttta gtgtaatttt tgttttttgg gtttaagcga tttttttgtt ttagattttc 6420
gagtagttgg gattttaggt gtttattatt atgtttagtt aatttttttt ttttttgaga 6480
tgaagttttg ttttgtcgtt taggttggag tgtagtggta cgattttggt ttattgtaat 6540
ttttgttttt cgggtttaag taattttgtt ttagtttttt gagtagttgg gattataggt 6600
aggtgttatt atatcggttg attttcgtat ttttagtaga gatggcgttt tattatattg 6660
gttaggttgg ttttgaattt ttgatttcgt gatttgttta tttcggtttt ttaaagtgtt 6720
gggattatag gcgtgagtta tcgtgtttag tttgattttt gtatttttat tagaaacggg 6780
gttttattat gttggttagg ttggttttaa atttttgatt ttaagtgatt tgtttgtttt 6840
agttttttaa agtgttggga ttataggcgt gagttatcgc gattggttta tttatttttt 6900
tttttatatt attaggtttg gtttgttaaa attggttagt tgttgtatgt ttatgttaat 6960
aggaatattg gtttatattt ttttttttta taatgttttt gtttggtttt ggtattagga 7020
ttattttggt ttcgaaaata agttgggaaa tattttttta tttttttttt tttttttttt 7080
ttttttgaga tagggtttta ttttgttgtt taggttggag tgtagtggcg taatttcggt 7140
ttattgtaat tttcgttttt taggtttaag ggatttttgt gttttagttt tttgagtaat 7200
tggtattata ggtatggtgt acgtttagtt atttttgtat tttagtagag atggggtttc 7260
gtcgtgttgg ttaggttggt ttcgaatttt cgattttaaa tgattttttg ttttagcgta 7320
ttaaagtgtt gagattatag gtatgagtta tcgcgtttag tttgtttttt tgttttttga 7380
agagtttgtg taagatgggt attgtttttt tttttaacgt ttaaagagag tagagaatag 7440
aggagataaa tagaaaatag tattaagagg ttaggtatgg tggtttatat ttgtaatttt 7500
agtattttgg gaggtcgaga tgggatgaaa gtttgaggtt agtagtttga gattagtttg 7560
ggtaatatag tgagattttg ttaatataaa aaaataaaat agttagttgg gtgtggtgga 7620
gtaattttag ttattcgtga ggttgttaga ggattgtttg agtttagggg ttcgaggttg 7680
tagtaagttt tgattgtgtt attgtatttt agtttgggta atagtgtgag tttttgtttt 7740
aaaaattaat aaagaaaaaa agaaaatagt attaaaatgg tagttttata ttttaattgt 7800
aaaataatta aaattaaaag tatttagtag agaaaggaag tttattttaa taagtggaga 7860
tagaataatt ggatttttat ataggaaaga tattagagat tgatttttat attttatatt 7920
ataaataatt aattttaaga attaattaat ggtttaagga ttttattgta aaatttatta 7980
ttataaaggt tttaaaagaa aatgtaagat tatattttta tgattttggg gttaaaaaaa 8040
aaaaaaaaga tgttttaaat aggataaggt aaatattgaa tataaaaaag attaatttat 8100
tttttttaag attttgtaaa ttttgtaaag taaataaata ggttcgtaat agattagaag 8160
aaaatattta cgatttatgg atttgataag gatttgtatt tagaaagtat aaagtagttt 8220
tataattgaa taataaaaat aaataaaaaa attaaaataa taggtaaaag attcgaagag 8280
ttattttata aataaaatac gaatggttaa taggtatatg aaaaaatgtt gaatattttt 8340
agttaataga gaattgtaaa ttataattat aaggatatat tatattagaa agattgatat 8400
atttaatgtt cggaaggttg tggtataatt ataataattt ttatattttg ttagttggag 8460
tgtaaaatgg tataatcgtt ttggaaaatt tagagttttt gaaaaagttt aaaatatagt 8520
tattttttat tttttttata attttttttt taagtatttt tttaagaaat acgaaaatat 8580
atgattataa aaagaattgt ataagaatgt ttatagtagt tttattttat aatcgtaaat 8640
gggaaataat ttaaaaggtt attaaaagga cggatatata atcgatggat tatattaaat 8700
gaaaaggagt aaaatattga tatatataat atgaacgaat gttagatagt atattgaagg 8760
atagaagttc gataaaaatg agtatataat gtatgatttt tttttttttt ttgagacgga 8820
gtttcgtttt tgtcgtttag gttggagtgt agtggtacga ttttggttta ttgtaatttt 8880
tgtttttcgg gtttaagcga tttttttgtt ttattttttc gaatagttgg gattataggt 8940
atttattacg tttagttaat ttttgtattt tttagtagag acggggattt attacgttgg 9000
ttacgttggt ttggaatttt tatttttaag taattcgttc gtttcggttt tttaaagtgt 9060
aggcgtgagt taaagtgtag gcgtgagtta tagcgtttat tttgatttta ttttatatga 9120
agttttttaa taggtaaaat ggttatggag attaaaataa aggtggggtc gggaatcgat 9180
tgggaagaga cgtgatgaaa cgtttttggg acgatgaaaa gggtttgtga tttggtaggt 9240
attacggagc ggttaggggt taaaatttat ttttttgtgt atttgttgtg tgtattggcg 9300
ttgtgtgtaa atgttatttc gatttaggaa aaagatgacg taagtacggt ataaagtggt 9360
cggtacgcgg taggtgtatg ggaagaaatt gcggaatgaa ataatcgcga gttaagagat 9420
ggggtagcgg gagaaatgaa ttcgagtttc gttttttatt aggaagaatc ggttcgggtc 9480
gagggttgta cggaggatta tacggacgtt tgcgggttcg tttttttcgt tttacgacgt 9540
ttagtttgcg tttggaattg gaatggttta gtttaaagtt agataatagg tagattgttt 9600
ttttcgataa attattaaac gatttattat tgtatttttt taaaatttta tttttagacg 9660
tattattttt tttttttttt tttcgggaag atgagatatg tttatttttg aaagtgtttt 9720
cgggtttgtt ttttgtatat tttttttttt ttttgtttac gttatggtag cgttcgttta 9780
ggttgtaggc gattcggggg gtggggtata ttatttaaag aaggggaggg attgaggttt 9840
gtattaaaat aaatattttt gtttttgtaa aggttataat taagtaattt agaaaaagaa 9900
atgtaggcgg agaatagtag tttttttttg ttaagtaaga ggaatcggtt taaaggatat 9960
tttttttttt tttttttttt ttttatcggg tgaatagtga gttgtttcgg taaaaagaaa 10020
tcggaaatgt tgttgtaaga ggtagaaatg taaatgtgga gttaaataat aatagggttg 10080
tcgggttttt tagattgcga cggttttttt cggtttggcg ggtaaatttt tggtttagta 10140

ttttttattt ttacgattga tagtttttaa ttggattttt tttatttagc ggagtcgggg 10200
gttgtttgga aagatcgttt taggaaggat aaaggttcgg aagttgtggg attttagtag 10260
tttgggtttt tcggattatt tttaaatgat tatttcggaa tggagtttta gtttttatta 10320
ggatgttatg ggtttaaaaa tatatagtta tgagttttta atgtttcgag atttaaaagt 10380
tttagatttt aatgttttgt gtatttttta ttttagggat tttttacgtt tagtatcggg 10440
tggatgtgta aagaagtacg ttttaggtcg gtttaaggtt ttttaaagtt ttattttttt 10500
gtttaggcgt ttaattttga gttcggatgg ttttaatatt tttattattt atatttaggt 10560
tttttaataa tgtaattttt atgatgattt ttttagttaa gtttttattt tatttatttt 10620
taaattcgtt aagtttttat tgttttattt ttagttttag cgattttttc gagttgaaaa 10680
tatacggagt cgagagttcg tgatttagag aggatttatt aagtttagtt aggagtttat 10740
ttaatttagg gaagcgtgtt atcgtcgtgg aaagtacgtt tttagttcga acgtaaagtg 10800
ttttcggagt ttagtagtta tttgtttttt ggacggtggt tttagatttt tgagaagttt 10860
aaaattttta gcgttagttt tgagtatatg ggaggggaaa attttaattt tattaatttt 10920
tgcgaggttt ttggtataaa gttggatagt cgttatgata agtaagggta agtaattcgt 10980
ttgtcggagg aagtaaagga aatggagttg gggaggaggg tgtagagtta ggattttcgt 11040
cgatttggtg tcgtagatat taatattttg gggtggaaaa ttttgtaagt tagagttgtg 11100
agggtagaat tggtggaaat tattttggag gaattttgta ttgtgttaaa tatgaagggt 11160
ggaaggaaga aagtttttgc gtttgttttt agttggattt ttttttttta ttagttaaaa 11220
tgttattttt taggaaggtt tttcgtaata ttatatttta acgttttttt ttagatattt 11280
tatattatat tattttattt aatttttttt ataattttta ttattttgat aagatttatt 11340
tgtttattgt ttttagtata tggaaacgta agttttatga ggatatagaa tttttttatt 11400
attttattta ttgttgtatt tttgagtgtt tatattagtg ttgggtagta agtaagagtt 11460
cgataataaa tattttttga atgagggaga taggtttgaa gtttggagaa tgagatgtag 11520
aagaggtgta agatttgttg cgttttttgt aggcggcggg ggggcggtgt aggtgtttta 11580
agaattatcg cgggattcgg tagggggagc gtaggcgttt ttcgttaaga tagaagcgtt 11640
tagattataa tttttagtag ttacgaggag ttttagggtt tgatgggaac gggaaatttt 11700
ttaatttttt acgtttcggt ttcgcgggtt tcgtgggtcg ttcgcgaaat ttgattcggg 11760
atgcggcggt ttaatcggaa ggtggatcga aatttcgcga tagtaagagg ttcgtagcga 11820
ttcgcggtgt taaggaatat agtgttttta aaagaattgg cgttcgttgt tcgttttttt 11880
tttcgggagt tttttgttta tttttagaag aggagggaag tataggtggg tttttttagt 11940
tttgcgtcgg atttttgaga atttcgaagt tattttggtt gaggttaatt ttcgttgtgt 12000
tttttttgta gtatgaagat tttggagatt taatcgttag tttcggattg ttgtttttta 12060
gattaggatt tagttttagt ttattttttt ttttacgttt tttcgatgaa taaaaatgcg 12120
gattttgaat tgatgttatc gtttttcgaa aggggggatt cgtttcggtt gtttttagat 12180
tttgtggttg gtttagttgt gttttaggag ttacgggagg gggatttagt tttttttttt 12240
atttttttgg aaatagagtt tggttttttt agtgagttga gttttcgaat cgaggagtaa 12300
gaattttttg aaaatataag tttttttgta gaagaagtaa acgggagttt ttttgaagaa 12360
gaagcgaacg ggttagagtt ggggtttgga aaagttatgg aagatatttt tggggaattc 12420
gttgtagagg acgagggaga tacgtaagtg gtgatggtag tggagtgtgg agtttgggga 12480
gatgaagtgt gaggtcgatt tgttttttgg ttttgagatt tattttttta ggtttttgtt 12540
tttttgtttt ggcgatttag gtttattgtt ttttattttt tttttagcgt ttggaattat 12600
agtttttttt agttgtttcg attttttagt ggtttttggt tagagtttag tatttaattt 12660
gagaattttt tgaaaggttg taagtggtaa ggataataac ggggtaggga gttgattatt 12720
ttcgagattt ttattgtaaa atagtttagt tttttaggag agggatgttt tagagttggg 12780
agaagcggta cgtagttttt ttagattgag tttatatttt atttagtagt ttgtgttttt 12840
tatttttttt aaggatttag gggggtttat ttattttaga ggtaggttta gttttagttt 12900
tatatttgaa aagtataggt ttggttagtt ttttaatttt tttttgtttt tagggttttt 12960
gacggttttt gtattttgat taatagtgtt gataatattt tgcgaattta taatttgttt 13020
ttagagttgt attatgaggg ggagtaggtg gaatatgtag aaatggtaag gattggggtt 13080
aattgttttt ttattaatgt tgtatattta agtttttcgt ggagatggaa aaagtgtagt 13140
ttaagtgttt tttgtttata aacgggattg ttttttaaga cgagttttta tatgtagcgt 13200
tttttgtttc gaattttgaa gttatttaga gagaggtttt tttttttttt aattaggaat 13260
ttgtgttttt tgttaaaaag tagtgaattt tttgtggtag tatatatata tttttatttt 13320
ttattttttt gagataaggt tttgttttgt tatttaggtt ggagtgttgt ggtgtgattt 13380
cggtttattg taatttttgt ttttcgggtt taagcgattt ttttgtttta gttttataag 13440
tagttggtat tataggcgta cgttattata ttcgattaat ttttatagtt ttagtagaga 13500
cggggtttcg ttatgttggg taggttgata ttaaattttt gattttagag gattcgttta 13560
ttttggtttt ttaaagtgcg gggattatag gtgtgagtta tcgtgtttgg tttatagtta 13620
tttattgatt gattattgat atatgttagt tattgtgttg ggataaagtg gtggataaga 13680
tagatgaagg gttggtttgt tttttgtgtg ggagatagat aataaatatg taaattagta 13740
agtattagga ttaagatgat gataaatgtt ttgaaggaaa tatatagaat gaagagagat 13800
taattagagg aaagtatatt gtataaaatg gttaggaagg agttttttga aaaggaggta 13860
tttgagttaa gatttgaagg aggataaggg attagttatg tgaaggtttt tggaaagagt 13920

tttttaggaa ttggtaatga tattataaat atttagaatt gggaaatagt tttgtgcgtt 13980
aaaagattag aaaggttttg tatggtggtt tacgtttgta atttagttta tttaggaggt 14040
cgaggtgtgt agattatttg aggttaggag ttcgagatta gtttggttaa tatggtaaaa 14100
ttttatcgtt attaaaaata ttaaaattag acgtatgtgg tggtacgcgt ttgtaatttt 14160
agttatttgt gtggttgagg taggagaatt ttttaaattt aggaggtgga ggttgtagtt 14220
agataaaatt gtattattgt attttagttt gggcgataga gtgagatttt attttaaaaa 14280
ataataaaat agattgggtt tagtggttta tatttgtaat tttagtattt tgggaggttg 14340
aggtgggtag attatttgag gttaagagtt tgagattagt ttggttaata tggtaaaatt 14400
ttatttttat aaaaatataa aaattagttg ggtgtgatgg tgggtgtttg taattttagt 14460
tatttgagag gttgaagtag aagaattatt tgaatttagg agatggaggt tgtagtgagt 14520
taagattatg ttattgtatt ttagtttggg tgatagagtg agattttatt ttaaaataaa 14580
taaataaata aataaataaa taaataaata taaaataaaa aatttagaat gttggtatag 14640
ttagattgag gtaagtgagg gggatagagg ggtgaggata gagaagagta gttacgtcgt 14700
gttggttttt atgggtaagg tatttggagt ttatttggga agttatgaga gaggtgtttt 14760
tttttttttt ttgagataga gtttcgtttt gttatttagt tggagtgtag tggcgttatt 14820
ttagtttatt gtaagttttg tttttcggga ttacgttatt tttttgtttt agtttttttc 14880
gagtagttgg gattataggc gttcgttatt acgtttggtt aattttttgt atttttagta 14940
gagacggggt tttattatgg tttcgatttt ttgatttcgt gatttttttg tttcggtttt 15000
ttaaagtgtt gggattataa gtatgagttt cgcgtttagt tttttttggg ttatttttta 15060
aattcgtttt attgtatttt ttagtagtat gagttattgt ttttattttt ttgcgttgga 15120
gtatttaggt atttagggtt ttgttgggtt tttttttttt ttattagtat tttttttttt 15180
ttgtattgtt ttttgagacg gtgataacga ttgttaaatt tttaattttc gagagtggtt 15240
tatcgaggtc gtgattatga tattaatgat tatagtatta ggatatttta aaagatatat 15300
tttgtaatat agatagttaa tttgtgtttt ttttggtttt ttttgggggg ggggggtttt 15360
gagatagagt ttcgttttgt ggtttaggtt ggagagtagt ggtgtgattt tgttttattg 15420
taatttttgt gtttcggttt aagcgatttt gttgttttag ttttttgagt agttgggatt 15480
ataggtacgt attatcgtat tcggttaatt ggggtatttg gagtagagat ggggtttcgt 15540
taagttggtt aggttggttt taaattttcg attttaagtg atttatttat ttgcgttttt 15600
taaagtgttg ggattatagg cgtgagtatt gtttttggtt agtgagtttg tgtttaaaag 15660
atttttgtgg ttggtgtggt ggtttatgtt tataatttta gtattttggg agattaaggt 15720
gggaggatgt ttgagtttag gagtttgaga ttagttttgg taatatagtt agattttgtt 15780
tttattaaaa aagttgggtt tatggtttta gttatttggg aggttgaggt gggaggatcg 15840
tttgagtttg cgaggttgag gtt 15863

<210> 223
<211> 6077
<212> DNA
<213> Artificial Sequence

<220>
<223> chemically treated genomic DNA (Homo sapiens)

<400> 223

aaaatatgag aaagtaagta tttgatattt tttattttaa aagagggggg ttttcggaat 60
gttattgttt tggtttttag tgattgtggt tttttttttt gttagaggtt ttatgtttag 120
attattagtg atatttttag tttttaaaga ttagtttagt ttatataagg gtaatgttga 180
aatatataag ttaggtttta atattgttat attttattgt aaagtataaa agtatgtttt 240
aattcgttat ttaaagtttt aagatatttt taagtttttt tttatttgta tgttaaaaat 300
taaatttaaa tgggaagtag tattgggaga aagttttttt gtgaattaat aattaaaatt 360
ttttgttagt tttataaata tttttggata atttttttaa atttataaag ggtaagaatt 420
gaggttttat agatgataac gaaaatggta aggaatgtat taattttttt atgtagtgtc 480
gaatttagag ttaaaataat agaagattaa aagataggtt tttatttttg tatatataaa 540
gatgtttcgt aaatgtttat ttatttttat taatttattt ttaatacgaa acgggaaatg 600
tttaagtgtt agtattattt ttttaaagat agtttttttt tgtttatatt tgtttattat 660
gtttttattt aagtaaataa ttttaagtaa aaataacgtt tgagtattta tttgttgaat 720
gtatatttta ttatagtttt aagattaaga attgatgttt aaatattaat tgtttcgtta 780
gttttattta ttaaggttaa agtagtttgt gttttatgaa tcgatattaa aaaagaaagt 840
tacgttagtt tttcgtggga gaaaagagag ataggcgggt gacggtagat tggaataagg 900
agttattata gtatttattt ttttatattg tagttttgaa aattgtgatt agttatagtg 960
aattttttat gaaatggaat atttttatta atattttgag gaattatata aattttattg 1020
taagatatga aaattagaaa tatggatttg ttttttttag tttttagggt ttttaagaag 1080
atgattttag taatagatta ttattaagtg ttttagggta ttttgattag agtgtataag 1140

ttggaaaaaa atatttaagt taagtaagta gatgtaattt ttttttttat aatttattga 1200
aatttttata aatggtaaaa ataaaaaata aattttttat ttaattgagg aggggggtat 1260
attgaaaata tattaaggtt gagatgttat ttaaattaat gaggtttttt gttttgtagt 1320
tgatgttatt ttatgtgatt tggaataaaa tttttttgaa gaggtaaaaa acgtaatttg 1380
agagttaaat ttttaaaagt tagatagagt ataggaatgt taggagtggt aaaatttgaa 1440
aaagtattat gatatattag ttagttagta taggtggtgg ataaggaaaa cgaagtttat 1500
tttttacgag gtatgttttt ttttagtggt tttttaatgt taattgtttt ttttttttga 1560
ggatatatat attaggtatt cgggttattt tttttttttg aggatatata ttattaggta 1620
tttaggtata tatattatat gttttgaagt ttaaagttat agttttgtga tttaagtatt 1680
tttttgttat ttatggaacg aagattttaa atttagttta tgaggataat tattttttta 1740
tttggggata gaatattagt atttaaatta tttattcggt atgtggtaga ggagaagaga 1800
attagaggag aagtagagat gataaagtag ttatattatt tattagttta taggtaatag 1860
aattattaat ttgttttttg tgataaagta ataataaaga gtcgatattt tttatatttt 1920
tatttgtgtg tagtcggtat tgtcgatatt tattttttta agggtatttt tttagatttt 1980
ttatttgttt acggtatttt tttgggtttc gtatttgttc gggtatttgt cggggttttg 2040
tatttgttcg gtttttatat ttgtttgggg tatttgtttg ggtttcgtat ttgtttgggg 2100
tatttgtttg ggtttcgtat ttgtttgggg tattttttcg ggttttttat ttgtttaggg 2160
tatttttttg ggtttcgtat ttgtttgggg tatttgttta gatattgtat ttgtttcggg 2220
tatttgttcg ggtttcgtat ttgttcgggt atcgtatttg taggattttt aagttgtttt 2280
tatttacgcg gtcgttttcg gttttgttcg tcggggacgt tggtatcgag gatgttttgt 2340
tcgtggttta ggttttcgtc gtttattagg tatttgtcgt tcggggagaa tttgtagagt 2400
aagttggaga gtttgaatat ttcggagaag tttatggtcg tcgtttgtcg cgggcgttat 2460
tttgcgttcg aaaattcgcg ggatttttgg gcgcgtagta ggttgtaata gtcgacgtcg 2520
gttttcgagg tcggaagtta gaaggcggaa gtgaattgta gtttattagc gtcgtcggtt 2580
ttcgcgcggt attgtggggt ttgtagtttt tgtgtcgtag ggttttaaag gaaacgttta 2640
cgtttttttc gattagggat tttcgattcg agaattttat tttaaaggtc gggaggtttt 2700
tgagtatttt tagttagggt tgttgataaa aatgtagaaa gtatagtaaa atttgaattt 2760
tagatttata ataaatttag ttataagtat gtttttaaat attgtacggg atatgttaat 2820
acggaaaaat tattcgttag tttgaaattt aaatttaatt gagcgatttg tgtgtttgcg 2880
tgtgtgtata tatgtatata tatatattta tatttatatg taaatgtatg tttatatgta 2940
aatatatgtt tatttataaa tatattttta ataagtaata cggtgtttgt cgtatatata 3000
ttatatcgtg tatgtaatgt ataagtattt atttcgtttg tttggggttt tgtttgtttt 3060
tgttgagttc gattttttta tttgtcgttt ggtttttgtt ttacgtttta gtgttattga 3120
gattaaggag agaacgaatt tgtcgttgat tgggtagagc gagcgcgtgg atcgcggtta 3180
tcgttcgttt attattcgcg cgtatttggg ttggtatcgg gcgaagaatc gtgcgggttt 3240
gggatttggg ggtttagagg gagcgagttt ttgcgcgggc gttcggttcg taggtttcgt 3300
aggtttaggg gcgtgtttcg tttttatttt tatttcggat ttcggttttt ttttttagat 3360
agcggttttt tttatttttg gttttcgtag gtcgttagta gttcgcgtta ggtttcgtcg 3420
gcgtttttag ggttttttag atcgcgtaga ttttgatatt ttcgtttggt tttgggtttt 3480
gggagttgag agtcggttag ggttttgttc gtattttcgg gcgtttagtt tcgggtttgt 3540
ttttcgcgga cgttttaatt ttttcggtcg aatggatggt ggtgcgcgcg cgttttattt 3600
cggcggtgtc ggtttttttt gttgttaaaa ttagatttaa atttttgtat gggattcgtt 3660
tttgggtttt tatttcgtgc gtttagtaaa tagtgggtga gttatgaaga tgtgcgagtt 3720
agtcggattt ttttcgttag gcgcggattc gttgcggtta gagaatttag tttgcgttag 3780
ttcggttcgt tcgcgaagtt acgggtttta ttgacgcgat tttttaagac gtgggggtta 3840
ttatgggtag aggatatcgg ttcggagtta gattacgggt tttataagta ttagattata 3900
agtagcgtcg ttattgagag tcgttcggaa ttcgtttagt atgtcgggtt ttttagttag 3960
ggtttggtgt acgtggtcga gggttttgga agtttcgatg gtttaggagg agtaggcggg 4020
cggggcggcg ggtgtcgttg gtcggtagag agtttcggtt tgatttagcg taggtttggt 4080
gcgcgtagag aataatttta agcgtatcga cgttcgcgag ttttttttaa atatcgaacg 4140
ggatttagag ttcgagttta taggcggcgg tcgggggagg gagtagggtg ttggtcgtcg 4200
ttcgggagtg ttcgcgtttt gggtgatttt tggaaggacg tggggtttaa atttcggttg 4260
gggttgggag agtagttttt agaggttttt cgcgggattt tttgtcgggc gggatcgtgg 4320
ttttatagga gaagtgggtg gtaagttttg tttggcggaa agtagtcgtt tttttttttt 4380
tgggtttggg gcggcgtttt ttatttttgt ttttcgtttt ttatttttgt ttttcgtcgg 4440
ttatattttt tgttttttgg attttaagcg tttcgcgcgt cgaggagttt agcgttagtg 4500
gcggcggtta ggagagattc gggtgttagg aaagatgggt cgtttggggg atagtaggga 4560
gttcggggga aacgtaggcg tcgggtatag agtcggtatc ggcgttttta gttttgtcga 4620
agatcgcggt cgggtttggt tcgcgggagg ggttttggcg tcggatttgt ttcggttttg 4680
cgtgggcggt ttcgtcgggt tttgtaggag cgacgcgcgt taaaaggcgg cgggaaggag 4740
gcggggtaga gcgcgttcgg gatttcgatt tggacgcggt tagttggaga ggcggagcgt 4800
cgggaggaga ttttggtttc gtcgcgattc ggtggttcgc gttgtttttt cgcgcgtcgg 4860
gttaaaaagg cgttaacgtt cgcggtcgtt tatttttcgc ggcgtttttt tttttcgcgt 4920

ttatataatt cgtttagggg tcgggtagtt cgttttgttt tttcgttcgc gtattcgttc 4980
ggaggttcgc gcgttcgcga aggggacgta gcgaaatcgg ggttcgcgtt aggttagtcg 5040
ggacggacgt cgatgttcgg ggttgcgacg gttgtaggta ggaggtttag ggtcgggggg 5100
cggttcggtt tcgcgggcgg gggttggagc gtagcgttgg gtaggtattt gggttcgtag 5160
tttcgaagtt gggaggtgag gggagagcga tcggggacga gttgggataa ggcgatatag 5220
gggttttttc ggagttggat cggtttttgg gatttggcgt tcgcgagagg ttggagcggt 5280
tagagtttag tttgcgagga gacgcgggtt ttgtttttag cgtcggtcgt ttttggcgtt 5340
aaagatagtt tcgtaggggt ttcgggaggg tttttttttt gttgtttttt ttttatttcg 5400
ggtttcgagg gtcgttggga gggtaatttc gggaagaggt cggggtgcgg ggcgcgggtg 5460
taggtggaaa tcgttagtaa gttttttttc gttcgcgcgt ttttttcgat ttgtagggtt 5520
gtgttaattt cgaggtttta gtttttttga ggagttaggg ttaggttttt ttttggatag 5580
ggagaaggat ttgggcgggg gttttgattt atggagttgg ttattaagcg gtttcgatgg 5640
tttttcgagg gatagttttt tgtggttttg agtttgtttg tcgagggttt ttggtttgtt 5700
ttcggagcgg ttttaggtag agaaagttcg tgaagaaatg gttcgggtcg gtttggaggg 5760
agatatttta cgttttttta gtttttgggt cgtttttttt tgtagttttt gtttttttcg 5820
gggtttggat ttggggagcg atgattattt ttgtttagtt tgtattttgg tttggacgtt 5880
aggagataag tttatgtagt atgtatacgt ttgttatata aataggggat agatagacga 5940
tttttaatta gtaagggtgt agggaaaagt aatgtatttt aaatttttga ttagaggtta 6000
tttgttttta aagatgttgt tatttgttta tttattgttt ggatatttgg aaatggttta 6060
ggtttattaa tataatg 6077

<210> 224
<211> 6077
<212> DNA
<213> Artificial Sequence

<220>
<223> chemically treated genomic DNA (Homo sapiens)

<400> 224
   tattgtgtta atgagtttga gttattttta aatgtttaga tagtgaataa atagatggta 60
   gtatttttgg aagtaaatga tttttggtta gaagtttggg gtgtattgtt tttttttgta 120
   tttttgttgg ttgaagatcg tttatttgtt ttttgtttat gtagtagacg tgtgtatatt 180
   atatgggttt attttttagc gtttaggtta aaatataagt tgagtaaagg taattatcgt 240
   tttttaagtt taagtttcgg gaaaggtagg ggttgtagga ggaggcggtt taggagttaa 300
   gggggcgtga ggtgtttttt tttaggtcgg ttcgggttat ttttttacgg gtttttttta 360
   tttgggatcg tttcggagat aggttaggag ttttcgatag ataaatttag agttataggg 420
   agttgttttt cgggaaatta tcgaaatcgt ttagtaatta attttatggg ttaaggtttt 480
   cgtttagatt tttttttttg tttagagggg ggtttggttt tggtttttta gggaagttga 540
   ggtttcggga ttggtatagt tttgtaggtc ggagggagcg cgcgggcggg gaggagtttg 600
   ttggcgattt ttatttgtat tcgcgtttcg tatttcggtt ttttttcggg gttatttttt 660
   taacggtttt cggagttcgg ggtggagagg ggatagtagg aggagggttt tttcggaatt 720
   tttgcggggt tgtttttggc gttaaaggcg gtcggcgttg agggtaggat tcgcgttttt 780
   tcgtaggtta gattttggtc gttttagttt ttcgcgagcg ttaagtttta ggggtcgatt 840
   taatttcgag ggagtttttg tgtcgttttg ttttagttcg ttttcgatcg tttttttttt 900
   attttttagt ttcggagttg cgagtttagg tgtttgttta gcgttgcgtt ttagttttcg 960
   ttcgcggagt cgaatcgttt ttcggttttg ggttttttat ttgtagtcgt cgtagtttcg 1020
   ggtatcggcg ttcgtttcgg ttggtttggc gcgggtttcg gtttcgttgc gtttttttcg 1080
   cgggcgcgcg agttttcggg cgggtgcgcg ggcggggagg tagggcgggt tgttcggttt 1140
   ttaggcgggt tatatgggcg cggggagggg aggcgtcgcg gggagtaggc ggtcgcgggc 1200
   gttagcgttt ttttagttcg gcgcgcggga aggtagcgcg ggttatcgag tcgcggcggg 1260
   gttaaggttt tttttcggcg tttcgttttt ttagttggtc gcgtttaagt cggggtttcg 1320
   ggcgcgtttt gtttcgtttt tttttcgtcg ttttttggcg cgcgtcgttt ttgtagagtt 1380
   cggcgaggtc gtttacgtag ggtcgaagta ggttcggcgt tagggttttt ttcgcgggtt 1440
   agattcgatc gcgattttcg gtagagttgg ggacgtcggt gtcgattttg tgttcgacgt 1500
   ttgcgttttt ttcggatttt ttgttgtttt ttagacggtt tatttttttt gatattcggg 1560
   ttttttttgg tcgtcgttat tagcgttggg tttttcggcg cgcggggcgt ttggaattta 1620
   aggggtaggg gatgtggtcg gcggggaata ggggtgaggg gcggggaata ggggtgaggg 1680
   gcgtcgtttt aggtttagga ggaggggaac ggttgttttt cgttaagtag ggtttgttat 1740
   ttattttttt tgtggagtta cggtttcgtt cggtagagga tttcgcggag agtttttggg 1800
   ggttgttttt ttaattttag tcggagtttg ggttttacgt ttttttaggg gttatttagg 1860
   acgcgaatat tttcgggcgg cggttagtat tttgtttttt ttttcggtcg tcgtttgtgg 1920
   gttcgggttt tggatttcgt tcggtgtttg gaaggagttc gcgggcgtcg gtgcgtttgg 1980
   agttgttttt tgcgcgtatt agatttgcgt taggttaggt cgaagttttt tatcggttag 2040
   cgatattcgt cgtttcgttc gtttgttttt tttaggttat cggggttttt agggttttcg 2100
   attacgtata ttaggttttg gttaggggat tcgatatgtt gggcgagttt cgagcggttt 2160
   ttagtggcgg cgttgtttat ggtttgatgt ttatggggtt cgtgatttgg tttcgaatcg 2220
   atgttttttg tttatggtga tttttacgtt ttggaaagtc gcgttagtga agttcgtggt 2280
   ttcgcgagcg agtcgggttg gcgtagattg ggttttttgg tcgtagcggg ttcgcgtttg 2340
   acggggaggg ttcggttgat tcgtatattt ttatggttta tttattgttt gttgggcgta 2400
   cggggtgggg atttaaagac gaattttatg tagaggtttg ggtttagttt tggtaataga 2460
   aaaggtcggt atcgtcggag taggacgcgc gcgtattatt atttattcgg tcggggaggt 2520
   tggggcgttc gcggggagta gattcgaggt tgggcgttcg gaggtacgag taggattttg 2580
   gtcggttttt agtttttaga atttagggtt aggcggggat gttagggttt gcgcgatttg 2640
   gggggtttta gaggcgtcgg cggggtttgg cgcggattat tagcggtttg cgggagttag 2700
   gggtggaggg ggtcgttgtt tagggaagag gatcggggtt cggagtgggg gtgagaacga 2760
   ggtacgtttt tgagtttgcg aaatttgcgg atcgagcgtt cgcgtaggag ttcgtttttt 2820
   ttgggttttt aggttttaga ttcgtacgat tttcgttcgg gtgttagttt agatgcgcgc 2880
   gggtgatgaa cgggcggtgg tcgcgattta cgcgttcgtt ttgtttagtt agcggtaaat 2940
   tcgttttttt tttgatttta gtggtattgg agcgtgaggt aagggttagg cggtaggtag 3000
   aggggtcgga tttagtaaaa gtaaataaaa ttttaagtaa acgaaataaa tatttatata 3060
   ttatatatac gatataatat atgtgcgata gatatcgtat tatttattaa agatatattt 3120
   gtaggtaaat atatatttat atgtaaatat atatttatat ataaatataa atatatatat 3180
   atgtatgtgt atatatacgt agatatatag gtcgtttaat taaatttgaa ttttagatta 3240
   gcgagtaatt ttttcgtatt agtatgtttc gtgtaatatt tgggaatata tttataatta 3300
   gatttgttgt gaatttgaaa tttaaatttt attgtgtttt ttatattttt attagtagtt 3360
   ttagttggag gtgtttaaag gtttttcggt ttttgaggta aggttttcgg gtcggaaatt 3420
   tttggtcgga agaaacgtgg gcgttttttt taaagttttg cggtataaga attataagtt 3480
   ttataatgtc gcgcggaagt cggcggcgtt gataggttgt agtttatttt cgttttttga 3540
   ttttcggttt cggaggtcgg cgtcggttgt tgtagtttgt tgcgcgttta ggggtttcgc 3600
   gggttttcgg gcgtagggtg gcgttcgcgg taggcggcgg ttatgaattt tttcgaggta 3660
   tttaagtttt ttagtttatt ttgtaagttt ttttcggacg gtaagtattt ggtgagcggc 3720
   ggggatttgg gttacgggta ggatattttc ggtgttagcg ttttcggcgg gtagggtcgg 3780
   gggcggtcgc gtgggtggag gtagtttggg gattttgtag gtgcggtgtt cggataggtg 3840
   cggggttcgg gtaggtattc ggggtaggtg tagtgtttga gtaagtgttt tagataggtg 3900
   cgggatttag gaagatattt tagataggtg gagggttcgg ggaggtgttt taagtaggtg 3960
   cggggtttag gtaggtattt taagtaggtg cggggtttag gtaggtattt taagtagata 4020
   tgggggtcgg ataggtgtag gatttcggta ggtgttcggg taggtgcggg atttaggaag 4080
   atatcgtaga taggtggggg gtttggggag gtgtttttgg aaaggtgggt atcggtagtg 4140
   tcgattgtat ataggtgagg gtataggaga tgtcggtttt ttgttgttat tttgttataa 4200
   aaggtaaatt gatgattttg ttgtttgtaa attggtaagt ggtgtggtta ttttgttatt 4260
   tttatttttt ttttaatttt tttttttttt attatatgtc gaataaatga tttaaatatt 4320
   agtattttat ttttaggtaa ggaagtagtt gtttttatgg ttgggatttg aggttttcgt 4380
   tttatgggtg gtagaagggt gtttaaatta tagagttata attttaagtt ttaaaatata 4440
   tagtgtatgt atttggatgt ttagtagtgt atatttttag aagagaggga taattcggat 4500
   gtttagtatg tatgttttta gaagaggggg ataattggta ttggaaagtt attaagaaga 4560
   aatatgtttc gtaaaaagtg ggtttcgttt tttttattta ttatttgtat taattgattg 4620
   atgtattatg atgttttttt aggttttgtt atttttgata tttttgtgtt ttgtttagtt 4680
   tttgagagtt taatttttaa attacgtttt ttattttttt agggaggttt tattttaaat 4740
   tatataaaat agtattagtt ataaaataga aaattttatt agtttaaatg gtattttagt 4800
   tttggtatat ttttaatgtg tttttttttt taattaaatg agaggtttgt tttttgtttt 4860
   tattatttat gaaaatttta gtgaattata aggagaaaga ttatatttgt ttgtttaatt 4920
   tgggtgtttt tttttagttt gtatattttg gttaaaatat tttgaaatat ttggtaatga 4980
   tttgttattg gaattatttt tttgaaagtt ttgaggattg gggagagtag gtttatgttt 5040
   ttagttttta tattttgtag tagagtttat gtggtttttt aaggtgttgg taaaaatatt 5100
   ttattttata aggaatttat tgtaattgat tatagttttt aaagttatag tgtgagaaag 5160
   taagtgttgt ggtagttttt tgttttaatt tgtcgttatt cgtttgtttt tttttttttt 5220
   tacggaaaat taacgtggtt tttttttttg atatcggttt ataaaatata aattattttg 5280
   attttgataa gtaaaattaa cgaagtagtt aatatttaaa tattagtttt tgattttgaa 5340
   attgtagtaa aatatatatt tagtaggtga gtatttaggc gttattttta tttgaaattg 5400
   tttgtttgga tagggatata atgaataaat atggataaga agaaattatt tttaagaaaa 5460
   tagtattaat atttaaatat ttttcgtttc gtgttaggaa taggttaatg gagataaatg 5520
   agtatttgcg aagtattttt atgtgtgtag aagtgaaagt ttgttttttg attttttgtt 5580
   gttttggttt tgagttcggt attgtataag aagattgatg tattttttgt tattttcgtt 5640
   gttatttgtg aagttttaat ttttattttt tataaatttg aggaaattgt ttaaaagtgt 5700
   ttataaaatt agtagaggat tttggttatt agtttataga agagtttttt tttaatgtta 5760
   ttttttattt aagtttagtt tttaatatat agataagaag ggatttaaaa atgttttaaa 5820
   gttttaagta gcgagttaaa atatattttt atattttata atagagtata gtaatattaa 5880
   aatttggttt gtatatttta atattatttt tgtatgaatt gaattgattt ttaggggttg 5940
   gggatgttat tggtgattta gatatgaaat ttttggtagg agagaaggtt atagttattg 6000
   aaggttaggg tagtagtatt tcgaaggttt tttttttttg gagtggagaa tgttaggtgt 6060
   ttgttttttt atatttt 6077

<210> 225
<211> 2501
<212> DNA
<213> Artificial Sequence

<220>
<223> chemically treated genomic DNA (Homo sapiens)

<400> 225

agttgggatt ataggtgtac gttattatgt ttggttaatt tttttatttt ttatagagat 60
aggatttcgt tatgttgttt aggttggttt taatttttga atttaagtga tttatttgtt 120
ttgggttttt aaaatttcgg gattataggc gtatagtgtt aggtttttat atttttatat 180
atttttttgt ttttttagtt gtgaaatgga gataatgata tagttttaat ttacgtggga 240
tgtttagtat agtgtttggt atttgataga tttttttttt tttatttgaa atttattttt 300
gagttgggta cggtggttta tatttgtaat tttagtattt gggaaaatta aggtaggtag 360
attatttgag gtgaggagtt taagattagt ttggtaaata tggtgaaatt tcgtttttat 420
taaaagtata aaaattattt gggtgtggtg gtatatattt gtaattttag ttattttaga 480
ggttgaggta cgataattat ttgaattcgg tagatggagg ttgtagttga gttgagattg 540
agttattgta ttttagtttg ggtgataaag cgagattgtt taaaaaaaaa ttatttttaa 600
tttaatataa aattttttga attttttttt ttttgagttg tttttttatt tgagtttatt 660
ttatattatt gaatagttaa ttatagaatt gtgtattatt ttttttgatt tttagatttt 720
tagtttttta tagtattttg tttttttttg tcgtattagt tatagttgta tttttttttt 780
tttttttttt gagatagagt tttattttgt tatttaggtt gtagtgtagt agtataattt 840
tagtttattg taattttcgt ttttcgggtt taagtaattt ttttgtttta gttttttaag 900
tagttgggat tatatgcgcg tattattacg ttaggttaat ttttgtattt ttagtagaga 960
cgaggtttta ttatgttgtt aggttggttt agaatttttg attttgtgat tagtttattt 1020
tagtttttta aagtattggg attataggta tgagttattg tattcggtta tagttgttat 1080
tttatttgtg tgattgatta tcgtatgacg tttttattga agtgtagtga aggtaggaaa 1140
tatgatcggt ttttataata tttttaatat ttattattat aggtggtata tagtagggtt 1200
ttaataaata tttgtaaaat gaataaagtt aatgagtgtt tatgtttgta gttttaattg 1260
ttttgaagta aatatttgat tttttaattt ttattagagt gttttgtata tagtaggttt 1320
aattattttt ttttttttat taattattta tgttttgtaa gaatttagtt agttgatttt 1380
ttgtatttta taagcgtgat gattgggtgt tttcgtgtga gatgcgttat tttcgaattt 1440
tgttacgacg tcggtatatt gcgcgtttga tatgaagaaa aaaaaaattt agttagttta 1500
ttaggtatag tggttaaggt ttgtaatttt tgtattttga gaggttaagg taggaggatt 1560
atttgaattt aggagttcga gattagttta ggtaatatag cgagatttcg ttttaaataa 1620
taaataaaaa taattagtcg ggtatggtgg tgcgcgttta tagtattaat tattcgggag 1680
gttgaggcga gacgatcgtt tgagttaggg aggttaaggt tgtagtgagt taagttcgcg 1740
ttattgtatt ttagttcggg cgatagagtg agattttgtt ttaaaaaaaa aaaaaatatt 1800
aaattttaga ggggcgaaaa aaaattttat agtggaaata tagtaacgag ttggtttagt 1860
ttcgttttcg ttataacggt ttacggtgtt ggaggatttt tttgcgtacg cgtatagttt 1920
tcggtcggtt attttcgcga gcgcgtttta ttttttatcg agcgcgcgcg aagattacgg 1980
aggtcgattc gggagcgcgt acgtagtttc gtttcgcgtt cgattcgcgg atttcgcggc 2040
gttcggttcg ggtggtttgg atcgcggagg gaatgtttcg gagggcggag aattgggacg 2100
aggtcgaggt aggcgcggag gaggtaggcg tcgaagagta cggttttgaa gaagacggcg 2160
gggaggagtc gggcgtcgag gagttcggtt cggaagagtt cggttcggag gaattgggcg 2220
tcgaggagga gatggaggtc gggcggtcgc ggttcgtgtt gcgttcggtg aattcgcgcg 2280
agttttttta ggttattttt tgtaatcgta gttcgcgcgt cgtgttgttc gtatggttta 2340
atttcgacgg cgagtcgtag ttttatttaa cgttgtcgtt tggtacgggt cgtcgtattt 2400
atagttatcg aggtacgggt tcggcgttta ggttcgattt agtagggacg atagtacggt 2460
ttgaagtttt tttatcgttt cggggtttat tttgtagacg g 2501

<210> 226
<211> 2501

<212> DNA
<213> Artificial Sequence

<220>
<223> chemically treated genomic DNA (Homo sapiens)

<400> 226

tcgtttgtaa aatggatttc ggggcggtag aggggtttta gatcgtgtta tcgtttttgt 60
tgggtcgggt ttaagcgtcg ggttcgtatt tcggtagttg tggatgcggc ggttcgtgtt 120
aggcggtagc gttgggtagg gttgcggttc gtcgtcgaag ttgagttata cgggtagtac 180
gacgcgcgga ttgcgattgt agaagatgat ttgggagggt tcgcgcgagt ttatcgagcg 240
tagtacgggt cgcggtcgtt cggtttttat ttttttttcg gcgtttagtt ttttcgggtc 300
ggattttttc gggtcggatt tttcggcgtt cgattttttt tcgtcgtttt ttttagggtc 360
gtatttttcg acgtttgttt ttttcgcgtt tatttcggtt tcgttttagt ttttcgtttt 420
tcggggtatt tttttcgcga tttagattat tcgggtcgga cgtcgcggga ttcgcgggtc 480
ggacgcgggg cggagttgcg tgcgcgtttt cgagtcgatt ttcgtagttt tcgcgcgcgt 540
tcggtagagg atggaacgcg ttcgcggaaa tagtcggtcg gaggttgtgc gcgtgcgtag 600
aaggattttt tagtatcgta ggtcgttgta acggaggcga ggttaggtta attcgttatt 660
gtatttttat tataaaattt tttttcgttt ttttaaggtt tggtgttttt tttttttttg 720
agatagggtt ttattttgtc gttcgggttg gagtgtagtg gcgcgagttt ggtttattgt 780
agttttgatt tttttggttt aagcgatcgt ttcgttttag tttttcgagt agttggtatt 840
gtaggcgcgt attattatgt tcgattaatt atttttattt gttgtttgaa acggagtttc 900
gttatgttgt ttaggttggt ttcgaatttt tgggtttaag tgattttttt gttttggttt 960
tttaaagtgt agggattata ggttttagtt attgtgtttg gtggattaat tgaatttttt 1020
ttttttttat gttagacgcg taatgtgtcg acgtcgtaat aaggttcgag ggtggcgtat 1080
tttatacggg aatatttaat tattacgttt ataaagtata aaaagttaat taattgaatt 1140
tttataaggt atgggtgatt agtaaagagg gaaaaataat tagatttatt atgtgtagag 1200
tattttggtg gaaattggaa aattagatat ttattttaga gtaattaaaa ttataaatat 1260
aaatatttat taattttgtt tattttataa atatttatta aggttttatt atgtattatt 1320
tatggtgata agtgttgaaa atattgtaaa aatcggttat gttttttgtt tttattgtat 1380
tttaataaaa gcgttatacg gtaattaatt atataaataa aatagtaatt atggtcgggt 1440
gtagtggttt atgtttgtaa ttttagtatt ttgggaggtt gaagtgggtt gattataagg 1500
ttaggagttt tagattagtt tgataatatg gtgaaatttc gtttttatta aaaatataaa 1560
aattagtttg gcgtggtggt gcgcgtatgt aattttagtt atttaggagg ttgaggtagg 1620
agaattgttt gaattcggga ggcggaggtt gtagtgagtt gagattgtgt tattgtattg 1680
tagtttgggt gatagaatga gattttgttt taaaaaaaaa aaaaaaaaaa gtgtaattgt 1740
gattagtacg ataaaaagag atagggtatt atagaaggtt aaagatttgg gggttaagga 1800
agatggtata taattttgtg attaattgtt taatgatgtg ggatggattt aagtggagaa 1860
gtagtttaga aaaagaaggg tttagagagt tttatattag gttaaaaata attttttttt 1920
agatagtttc gttttattat ttaggttgga gtgtagtggt ttagttttag tttagttgta 1980
atttttattt gtcgagttta agtgattatc gtgttttagt ttttggagta gttgggatta 2040
taggtgtgtg ttattatatt taggtaattt ttgtattttt agtagagacg ggttttattt 2100
atgtttgtta ggttggtttt gaatttttta ttttaggtaa tttgtttgtt ttggtttttt 2160
taagtgttgg gattataggt gtgagttatc gtgtttagtt taaaaataaa ttttaagtag 2220
aaagaaaaaa atttattaag tgttagatat tgtgttaagt attttacgtg aattaaggtt 2280
atgttattat ttttatttta tagttaagga aatagaaggg tatataagaa tgtgaaggtt 2340
tagtattata cgtttgtaat ttcgagattt tgggagttta aggtaggtgg attatttgag 2400
tttaggagtt ggaattagtt tgggtaatat agcgaggttt tgtttttata aaaaatgaaa 2460
gaattagtta ggtatagtgg cgtgtatttg tagttttagt t 2501

<210> 227
<211> 2401
<212> DNA
<213> Artificial Sequence

<220>
<223> chemically treated genomic DNA (Homo sapiens)

<400> 227

ttattaaaga aaaaaaagat gatttattag atagttgtta aggtatttat ataaaattat 60
acgatagtag gatagatggt aattaagagg gaatttagga ttcgttatta ttagttggaa 120

atatttgatt tttagtttgt aaatgtgata agttagatta atattagtag gatattgaat 180
atagtttgta gggatgagtt tttgcgttat ttttggatat gtgtttaagt aattagaata 240
atttaaaatg ttattaggtt tttgttgtta tttttataaa tttcgaatag tgagttgggt 300
ttttaaggaa tttgtttttg ttagtttatt ttatgttgta taataaatag gtttaaagtt 360
tttggtagcg tatattaagt atggaggatt ttttatttat tacgatagtt ttttcgttgg 420
agggttgggg ttgtatttta tttttttttt tggaaggggc ggtttttgtt tgaggtttta 480
tttgttttat agtagaggga tgaaacggta ggattttggt tttgacgggt tttatttggt 540
aatggtatat gttaagtggg ttacgtttta ttgtttagag taagttatat ggttacgttt 600
gatattgacg tgcgggaaat gtagaatttt tttgtatttg ggaggtagaa tatttggata 660
atatgtaatt tattataggg ttagttggat gaagaacggt taagggttta ggttttttat 720
ttcgagttag gtcgaattag tcggggtcgt gagattaagt tacggttatt agtgttcgtt 780
tggaattatg ttttgttttt gtcgtgttta tattgtagta gggtttttgt tttttaattt 840
atttgaaatt ggtggtgggt gtggtatata gatgagaaat aggaattttt tacgttgggt 900
tttaatagga gttgcggtgg gaatataaat ggtgtagtta ttttggaaga tagtttggtg 960
gttttttata aaattaaatg tattttttat atatgattcg ttatatatta tttagtaatt 1020
gtattttttg gtatttgttt agatgagtta aaaatttgta tttatataaa aatttgtacg 1080
tggatgttga tagtagtttt atttataatt gtttaaattt ggaagttttt aggaggtgag 1140
tgggtaaata aattgtggtg ttttcggtta gcggaatatt atttagtgtt aaaaagaaat 1200
gagtttaagt tatggaaaga tatgaaagaa ttttaagtat tattaagtta aagaagttaa 1260
tttgaaaagg ttgatgttgt atgagtttat atttatgata ttatagaaaa ggtaaaatta 1320
taaagatagt aaaaggatta gtggttgtag gagttaggga ggagggaggg atgaataggt 1380
ggggtataga ggatttttag ggtagtgaaa ttatttgtta tgatattgta atggtggata 1440
taggatagta agtatttgtt ttttatttcg tagaatgtac ggtattgaga acggattttt 1500
ttgtaatata tggattttta gttataatat tgaatttttt tcggggtttc gtatcgttcg 1560
tagcgttttt taattttcgt tttcgtttcg tttatcgttt tttttttttg ttatagcgcg 1620
ttcgtttcgc ggtcgttaat tagtcgcgcg tttcggtcgc gcgtttcgtt tcgttttcgg 1680
tgggtgtgcg cgcggttaat gggcggtgcg cgggggtcgg gtcgcggcgg ggcggggtag 1740
cggggcggtc gttaatcgtc gtggtgttgt tgaaattgaa aatattatat tatgttaatc 1800
gcggtcgggt tcgcgcgtac gggggtgggg ttcgcgcgta taaagggggc gtaggcgggt 1860
tgggcgtttt ataggttaag tgcgttgtgt tcgaggggtg tcggttaggt ttgagcgagc 1920
gagttagtta gtaggtatcg agggggcgcg gttgtcgttc ggacgagata ggcgaattcg 1980
acgtagaaga gtttattatc ggatagttag gtagtcgtcg cgtttttcgt atacgtagag 2040
tcgggcggcg cggggttttt tttgcgttcg gttttcgttt tttttttttt tttttttttt 2100
tttttttcgt tgtttttttt tttttcgttg ttcgcgtttg cgtagtttcg ggttatgttc 2160
gacgcgtttt ttcgtagtat atttacgatg gagcgttttg tcgttcgtgg gattttttta 2220
gtattagtgc gtattagcgt ttgtcgtagt tttttcgggt cggtggatta cgaggagttg 2280
agtcgcgagt tgtaggttcg tttggtcgag ttgaacgtcg aggattagaa tcgttgggat 2340
tacgattttt agtaggatat gtcgttgcgg ggttttggac gtttgtagtg gatcgaagtg 2400
g 2401

<210> 228
<211> 2401
<212> DNA
<213> Artificial Sequence

<220>
<223> chemically treated genomic DNA (Homo sapiens)

<400> 228
   ttatttcggt ttattgtagg cgtttagggt ttcgtagcgg tatgttttgt tggaagtcgt 60
   aattttagcg gttttggttt tcggcgttta gttcggttag gcgggtttgt agttcgcggt 120
   ttagtttttc gtggtttatc ggttcgaaga ggttgcggta ggcgttggtg cgtattagta 180
   ttgggaaggt tttacgggcg ataagacgtt ttatcgtgga tgtgttgcgg agggacgcgt 240
   cggatatggt tcggggttgc gtaaacgcgg gtagcgagag aggagaggat agcgagaaga 300
   aggggaaagg agaggaggag agggcggagg tcggcgtaag gggagatttc gcgtcgttcg 360
   attttgcgtg tgcgagggac gcggcggtta tttggttgtt cggtggtgga tttttttgcg 420
   tcgggttcgt ttgtttcgtt cggacggtag tcgcgttttt tcgatgtttg ttggttagtt 480
   cgttcgttta ggtttggtcg gtatttttcg agtatagcgt atttggtttg tggaacgttt 540
   agttcgtttg cgtttttttt atacgcgcgg gttttatttt cgtgcgcgcg ggttcggtcg 600
   cgattagtat aatgtagtat ttttagtttt aataatatta cggcgattgg cggtcgtttc 660
   gttgtttcgt ttcgtcgcgg ttcggttttc gcgtatcgtt tattggtcgc gcgtatattt 720
   atcgggggcg gggcggggcg cgcggtcggg gcgcgcggtt gattggcggt cgcggggcga 780
   gcgcgttgta gtaggggaag ggggcggtga gcggggcggg ggcgggagtt aaagggcgtt 840
   gcgggcggta cggaatttcg aaagggattt aatattataa ttgaaagttt atatgttata 900
   ggagggttcg tttttagtgt cgtatatttt acgggataaa ggataaatgt ttattgtttt 960
   gtgtttatta ttatagtatt atataaggta gttttattgt tttaaaaatt ttttgtgttt 1020
   tatttattta tttttttttt ttttttagtt tttgtaatta ttgatttttt tattgttttt 1080
   gtagttttgt tttttttata atgttataga tgtgaattta tatagtatta gtttttttag 1140
   attggttttt ttgatttaat aatatttaag gtttttttat gtttttttat gatttgagtt 1200
   tatttttttt tagtattgaa taatatttcg ttgatcggag gtattatagt ttgtttattt 1260
   atttattttt tgaaggtttt taggtttggg taattatgaa taaagttgtt attaatattt 1320
   acgtgtaagt ttttgtgtgg atgtaagttt ttgatttatt tgggtaaata ttaaggggta 1380
   taattgttgg atggtgtgtg gcggattata tgtgaagggt atatttagtt ttgtaaggag 1440
   ttattaaatt attttttaaa gtggttgtat tatttgtgtt tttatcgtag tttttgttaa 1500
   agtttagcgt ggagagtttt tgttttttat ttgtgtatta tatttattat taattttaga 1560
   tgaattaaaa aatagaaatt ttattataat gtggatacgg taaggataag gtataatttt 1620
   aaacgaatat tgatgatcgt ggtttgattt tacggtttcg gttggttcgg tttggttcgg 1680
   ggtgaagagt ttgggttttt ggtcgttttt tatttagttg gttttgtaat gaattgtatg 1740
   ttgtttaaat atttgttttt ttaaatgtag gaagattttg tatttttcgt acgttaatat 1800
   tagacgtggt tatgtaattt gttttgggta gtgaaacgtg atttatttgg tatgtattat 1860
   tgttaaatgg agttcgttaa agttagggtt ttgtcgtttt atttttttgt tgtgagataa 1920
   atgagatttt agataggggt cgtttttttt aaaagagaaa atggagtata attttagttt 1980
   tttagcgaag ggattgtcgt ggtgagtgag agatttttta tgtttgatgt acgttgttaa 2040
   gagttttggg tttgtttgtt atgtagtata aggtaagttg atagagatag gttttttgaa 2100
   gatttaattt attgttcgag gtttatggga atgatagtaa gggtttggtg gtattttgag 2160
   ttgttttggt tatttggata tatgtttaga aatgacgtag agatttattt ttgtaagttg 2220
   tgtttagtgt tttgttggtg ttggtttagt ttgttatatt tgtaggttgg aggttaggtg 2280
   tttttagttg gtagtgacgg gttttgagtt tttttttggt tattatttat tttgttatcg 2340
   tgtagtttta tgtgagtgtt ttaataattg tttagtaagt tatttttttt ttttttgatg 2400
   a 2401

<210> 229
<211> 1508
<212> DNA
<213> Artificial Sequence

<220>
<223> chemically treated genomic DNA (Homo sapiens)

<400> 229

attttataat tataaatatt tagtgtattt tgtaaatatg gtataatttg ttggtataaa 60
attttattgt tttagaaaat atcggtaata aaattataga aaagttaaag atttttattt 120
tttatttcga agcgtttggg agatatttta gaaacggatg ggaaatgtta aattttgtat 180
gtttgtttat tttttttttt atatcgatta gatgtaaacg agtgttatta aaagtatatt 240
ataggtattt atatagattt tttttataag ggatttataa agtttagatg tgaaatgtat 300
tttaaagggt ttttagtcgt tttttatttt tttttttgtg aaatagggaa gatatatgtg 360
tttttaaggg tagagatgga atttgggtta atgggtcggg gggtgggggg aagttggaag 420
ggattgtttt aggatatggt aggatagtgg attgtttttg tcgttttgtt gtttatatcg 480
gggttttttg taggcgcgtc ggtttttttt ttttttgttg agaagaagtt ttgttaaaat 540
attcgttttt ttcgggaatt ttttcgtggg tttaaagcgg gaaaatgttg ttttaggttt 600
aaaataattt gtttaagttt tttagcgcgg gagaaacgtt tttattcgtt ttttgttttc 660
gggggtgttt ttcgtttttt gttgttagaa ttttggggag tgtttagatt cggggtagta 720
tacgttcggg ttaatcgcga gtagaataaa tttttggtgg gcggttagga agtttttttt 780
tagttatcgt ttttttttag tgtttttttt tttttttata taatataaga tttttttttt 840
ttagtttttt taaagtatag tttaaggaaa tttttttata gtttttattt agttacgggt 900
tagtatgttt gggggtaaat acgtagattc ggaggtaggt attcgtgggg gggcgtcggt 960
tcgggcgtgc ggggaagtgt tcgtttttgt tatttgtttt tttaaatatt tcgattgttg 1020
atttggtttt ttagtttttt ttttatttcg gagtattttt ttggcgttgg tatcgttgaa 1080
gaatgggttt gggcggggag gtgaagagaa gttaggaatg ttttatgttt ttttaatgga 1140
gagggggttt cgggagtttt tgagttagga ggatacggaa aaggggattg gggttttgag 1200
attgggtttg ttgggtttaa gacgcgtttt ttggatgggt ttaggatgtt tttttgtcgc 1260
gggaatttcg cggttcggtt ttgtttgttg ggggttcgaa aaagtggatt gtagggtgga 1320
aggtgttatt tattcgagtt ttggggatag ttttcgggat ttttcgttaa gcgtttaaaa 1380
cggtaggttt ttaagcggcg cgcggtgttt ttgtattttt taaaattttt gaaatatttt 1440

aaaaattttt ttgtattttg gcgtttggta gggtttttta aaaaaagtaa attttttttt 1500
taaatgta 1508

<210> 230
<211> 1508
<212> DNA
<213> Artificial Sequence

<220>
<223> chemically treated genomic DNA (Homo sapiens)

<400> 230

tgtatttggg agggaggttt attttttttg gaaaattttg ttagacgtta agatgtagaa 60
ggatttttga gatgttttaa gaattttgga aagtgtaaaa atatcgcgcg tcgtttgggg 120
atttgtcgtt ttgggcgttt ggcggagagt ttcggggatt gtttttagga ttcgggtaaa 180
taatattttt tattttgtaa tttatttttt cgaattttta gtaggtaggg tcggatcgcg 240
gggttttcgc gataggggag tattttagat ttatttagaa aacgcgtttt gggtttaata 300
gatttaattt taggatttta attttttttt tcgtgttttt ttagtttagg ggttttcgag 360
gttttttttt tattaggaaa atataaaata tttttggttt tttttatttt tttcgtttag 420
gtttattttt tagcggtgtt aacgttagag gagtgtttcg aagtggaaag agggttgggg 480
ggttagatta gtagtcggga tatttggaga ggtagatagt agaagcggat attttttcgt 540
acgttcgagt cggcgttttt ttacggatgt ttattttcga gtttacgtat ttgtttttag 600
atatgttggt tcgtggttgg atgaaaattg tgaggaggtt tttttgggtt gtgttttaag 660
ggaattgagg aaggaagatt ttgtattgta tggggggaaa aaaaggtatt ggaggggggc 720
ggtggttggg agggagtttt ttggtcgttt attaaaggtt tgttttgttc gcggttggtt 780
cggacgtgtg ttgtttcggg tttaggtatt ttttaaggtt ttggtagtag agggcgggga 840
gtattttcgg gggtagagag cgagtaagaa cgtttttttc gcgttggggg gtttggatag 900
attattttaa atttgaggta atattttttc gttttgaatt tacggggggg ttttcggggg 960
aggcggatgt tttggtaggg ttttttttta gtaggggggg agggagtcga cgcgtttgta 1020
gagagtttcg gtatgggtaa taaggcggta gaaataattt attattttgt tatgttttaa 1080
ggtagttttt tttaattttt ttttattttt cggtttattg gtttaagttt tatttttgtt ·1140
tttgaaaata tatgtgtttt ttttgtttta tagagggagg gatgagagac ggttaggaat 1200
tttttagggt atattttata tttaaatttt gtggattttt tatggaagga atttgtgtgg 1260
gtgtttgtgg tgtatttttg gtgatattcg tttatattta gtcggtgtgg aaagaaaaat 1320
aagtaggtat gtagaattta atatttttta ttcgtttttg aaatgttttt taaacgtttc 1380
gaaataagaa atgaagattt ttaatttttt tataatttta ttatcgatat tttttggaat 1440
aatggaattt tatgttaata ggttatgtta tatttgtaaa atatattaaa tgtttataat 1500
tgtagaat 1508

<210> 231
<211> 1984
<212> DNA
<213> Artificial Sequence

<220>
<223> chemically treated genomic DNA (Homo sapiens)

<220>
<221> unsure
<222> (9, 17, 18, 33, 41, 49, 50, 52, 54, 62, 80, 84, 127, 157, 159)
<223> unknown base

<220>
<221> unsure
<222> (175, 207..209, 214, 1181)
<223> unknown base

<400> 231
   agttttagnt attattnnta tttttattaa tgnttttggg nttataggnn gngntttttt 60
   gnttaaatag tagttaatgn aggnatttta aaaggtatat tattttatat ttatttagtg 120
   gttatgntat tatttaattt ttattataat tttgtgngnt tagtataatt tgtgntttta 180
   ttttatttgt gaataaatgg aggtagnnnt ttgngtaatt tataagtaag tggtagattc 240
   gggggttgtg tttagataat gtggttttag attttttgtt tttttttgtt ttttgttgta 300
   ttttgattga gtttagttat ttattttttt aatatatatt aattgttgtt tttatgtttt 360
   aggttttgtg ttagatgtta gaaatatatt tttatttatt ggattttggt atggtttatt 420
   gtttagtgtt ttgttatgtt acgtgttata taatgtattt atttcggtat ttagtttatg 480
   gaaaataatg ttgaaagata ttgtatgtat gtttttataa taaaattatg taatttatgt 540
   tttttttatt gttttggtta ttaggaagtt tttatttaaa ttagagtaaa tatattagaa 600
   tttgggtttg ttattttagt ttgttgaatt tttttttttg gtttagattt tttattttgg 660
   tttataaatt ttattgtata aatgtttttt attgtaaaat attttaaatt ttttttaagg 720
   gaaggttgta tggaaatgat atagtaaggt tttttttgta tttttttaga tttttattag 780
   ggaaggtaga tttagatgtt ttttttattt ttagttttaa agttttttat ttataaaatt 840
   ttttaagtag tgattattgt tgttttgagt atttggaggt agtttagagt ttttgaaagg 900
   taaaatttat atataaaaag aagttttttt cgatttagat tttagttttg gtgttagatg 960
   tatatttgag gagtaggtgg ttagttagat ttttatttga gtagttaata aaatttattg 1020
   ttttttaatg gaattttttt tgtaagttcg aattgatttg ttatttttgt gatttgtgag 1080
   atggtaggga agtattaaat attattatga tttgggttat agtggtggga aaaaaggaaa 1140
   aaagaaaaaa aaaatttatt gttaagtttt gttaggcgta naaagggttg gaattgttgg 1200
   ggttatttta tttgatttat tggaaataga gtggatttta ttaatatttt aataaagaga 1260
   atttttttgt attaggttgg aagtggtcgt tagtttttcg tgtaatttta ttttttggaa 1320
   aagtggaatt agttggtatt gtttagcgtg atttgtgagg ttgagtttta atagtttaaa 1380
   gaagtaaatg ggatgttatt ttcgcggggt tcgtttttcg cgaggtgttt atttcgtatt 1440
   tgttatgtaa aacgagggag cgttaggaag gaattcgttt tgtaaagtta ttggttttgg 1500
   ttattagttt ttatttaatg ttttcgtgat gttgttgttg atttatttgg gaagttggtt 1560
   ggttggcgag gtagagtttt tttttaaagt ttggttttta cggaaaatat gtttagtgta 1620
   gtcgcgtgta tgaatgaaaa cgtcgtcggg cgtttttagt cggataaaat gtagtcgaga 1680
   atttcgttcg ttttgtgcgt tttttgtttt taggtaggga agaggggttg tcgggcgcgt 1740
   tttgcgtttc gtttttgtat tcggatcgtt cggtacgggt agggtgaggg ggtttcgggg 1800
   gggtcggggt tttcggtcgc ggcggcgaag atagatcggg gttcggtagg gaggttattt 1860
   cgagtttaga gattttaggt attttttata tataggtttt tattttggcg tgcgtgcgtg 1920
   tgtgtgtgtg tgtgtgtgtg tgtgtgtgtg tgtgtgtgtg tgtgtacgtt cgttaacggg 1980
   agga 1984

<210> 232
<211> 1984
<212> DNA
<213> Artificial Sequence

<220>
<223> chemically treated genomic DNA (Homo sapiens)

<220>
<221> unsure
<222> (804, 1771, 1776..1778, 1810, 1826, 1828, 1858, 1901, 1905)
<223> unknown base

<220>
<221> unsure
<222> (1923, 1931, 1933, 1935, 1936, 1944, 1952, 1967, 1968, 1976)
<223> unknown base

<400> 232

ttttttcgtt aacgaacgta tatatatata tatatatata tatatatata tatatatata 60
tatatacgta cgtacgttag agtgggagtt tgtgtgtggg gggtgtttag gatttttggg 120
ttcggaatga tttttttatc gagtttcgat ttgttttcgt cgtcgcgatc ggaggtttcg 180
atttttcgaa agttttttta ttttgttcgt gtcgggcgat tcgaatgtag aaacggggcg 240
tagagcgcgt tcggtagttt ttttttttta tttgggatag gagaacgtat agaacgagcg 300
gagttttcgg ttgtattttg ttcgattaga agcgttcggc ggcgttttta tttatgtacg 360
cggttgtatt gagtatattt ttcgtgggag ttaggttttg aggagaggtt ttgtttcgtt 420
agttagttaa ttttttaaat agattagtag tagtattacg aaagtattgg gtagaggttg 480
atgattagga ttaatggttt tataagacgg attttttttt aacgtttttt cgttttgtat 540
ggtagatacg ggtgagtatt ttcgcgagga gcgagtttcg cggaggtggt attttatttg 600
tttttttgga ttgttggggt ttagttttat aaattacgtt gggtaatgtt agttgatttt 660

atttttttag agaatggaat tgtacggggg attggcggtt atttttagtt tagtgtaaaa 720
agattttttt tattaaaatg ttaataagat ttattttatt tttaataaat tagataaaat 780
ggttttagta gttttagttt tttntacgtt tggtaaggtt tggtagtgga tttttttttt 840
tttttttttt ttttttttat tattgtggtt taagttatga tggtgtttgg tgtttttttg 900
ttattttata aattataaag atgatagatt aattcgagtt tgtagaaaaa attttattaa 960
ggggtaatag attttattaa ttgtttaggt gagagtttga ttagttattt attttttaaa 1020
tgtgtatttg gtattaaagt tgggatttgg atcggaagga attttttttt gtatatggat 1080
tttatttttt agaagttttg aattattttt aggtatttag aatagtagta gttattgttt 1140
aagggatttt ataaatgggg ggttttggga ttgagggtaa gagggatatt taggtttgtt 1200
ttttttaata ggaatttaag aaaatgtagg gaagatttta ttgtattatt tttatgtagt 1260
ttttttttag aaagaattta aggtgtttta taataaagga tatttgtgta atagaattta 1320
tgaattaaaa tagaaaattt gggttagaag gaaaagttta gtaaattgaa atgataagtt 1380
taaattttaa tgtatttgtt ttgatttgga taagagtttt ttgatagtta aagtaataaa 1440
agaaatataa gttatatagt tttgttgtag aaatatgtat ataatgtttt ttagtattat 1500
tttttatggg ttaaatatcg gaataaatgt attatgtaat acgtaatata gtaggatatt 1560
gagtaatgga ttatgttaag atttagtgga tggggatgta tttttagtat ttggtatagg 1620
gtttggaata tggagatagt aattagtgtg tgttaaagaa atagatggtt agatttaatt 1680
aagatgtagt agaaggtagg gaggggtaag gagtttggag ttatattgtt taggtatagt 1740
tttcggattt gttatttatt tgtaaattat ntaaannntt gtttttattt gtttatagat 1800
aaaatgaaan tataaattgt attgantnta taaggttgtg gtgaggatta agtgatgnta 1860
taattattga ataaatataa agtgatgtgt tttttaagat ntttntattg gttgttgttt 1920
ggntagaagg ntntnntttg tggntttaag gntattgata aagatgnnag tgatgnttga 1980
agtt 1984

<210> 233
<211> 17897
<212> DNA
<213> Artificial Sequence

<220>
<223> chemically treated genomic DNA (Homo sapiens)

<400> 233

atattgtata attttttatt tatgtttaaa gtaaaggatt tattatatat tttgaatata 60
gtttattttt tatttttatt tggaattgtt ttttttttat ggtttttttt ttttttaatt 120
tttattgttt ataatatgat attataggtt aggtatgttt gtagataatt gggataattt 180
atttttattt aagtaaaaat tttagatggt agttttttta tttgggatta tgaagtaggt 240
aggatttttt tttttttttt aatagagttt aagtagtaag atcgaataag atgggatgaa 300
tttaaagaga aatatttgta tttgtttagt tttttaatta atatttattg aattttttta 360
cggttaggtg tgtaagatgt taggagggat gagataagat gatgatgatg atgatgatga 420
tgatgatgat ggtgatgatg atagtgagga tgagggtgaa tattttaatg agagtttttt 480
atatgttagg gattgtttta ggtattttat atatattaat atatatattt attataatga 540
ttttgtattg ttatgtgtat tttatatatg aggaggtaga ggaaggaaat gaaataattt 600
gtttaagagt atagagtttg taagtggtaa agataagata gaaataattt tttaagagta 660
gagagtggtg atttttgggg atagggaagt ggaaaagtgg ggagataaag gttgttgttt 720
tttttaataa gttttgaaga tttatttgat taggtttaag aaaaatttga taattattat 780
aaagtaaaag gaagaggaga tgaggtgggt ttttgggtat agatgtttgt tttggaaagt 840
ttagggagga aggggtgaga ggaattgggt tgtagtatag gaatggggga tttgggaatt 900
agaggggatt ggttagggtg ggttattgat tgtagtgaat gtttatttgt tgattgggtt 960
gtagtatagg agtgggggt ttggaattaa gaggggattg gtttagggtg ggttattgat 1020
tgtagtgaat atttatttgt tgattagttt gatttagaag aaagggagag atggatgttg 1080
gaggagagag ggggtttcga gggagtttag gattggaggt gggaagatat ttttacggat 1140
atttttttat tttgatagat agaaaagatg atggtttgta gtagtagatt tataaattgt 1200
gattgaaagg tttgatggtt ttttttattc gtttttttta tgtatagaga ggtggagtaa 1260
tttgttttag gtattatagt ttataagtgg tgaagttgga gttgggggtt atagattatc 1320
gggttttttt ttatgaattt ttgttgtatt ttggtttttt ttaatttcga gtatttatga 1380
gttttaagaa agatagttgg gaataggtga tttttatggt tatttttgga atatttggtt 1440
agaatatttt agtataaaat ttaagttgtt gttaagtaga aatttttagt ggatattagt 1500
ttagaaaatt taattttatt ttgtgacgga tttgtagcgc gtgtgtattt attaagtaaa 1560
tttgatttta ttgttgatta gcgagaaaga ggaataatgt aaatagttag tgtaattgta 1620
tttatattta gtttcgtgag tttttgttat tttagttggt tgcggtgggg gatgtggagg 1680
tgtatgtagt ttttaggttt tagtgagggg tttttgtata gggtgggcgt tttttatgtt 1740

ttatgtgtat ttaggtttgt attaaagata gttttttttt tgtagcgtga tatttagtta 1800
agaaaattat tttatttggt gtttagcgga agaaaatatt ttttttaata gggaaggaaa 1860
attggttgtg ttgggttgag ggaggggagt gtggaattta aatggtgttt tttttaggtt 1920
ttttttaagg tgatgtggat tttgatttta gaatgttatt atttggaaaa ttgtgtttta 1980
ttgtttaaat ttttgttttt tttttttgtt tttttttttt tattttattt tttttttttt 2040
gttttttgat ttatttaatg ttaatggttt tttagaaggt taggtttttt atataagttt 2100
agttttttgt taggtaattt tttcggggtt ttaagttgta aatttgggtt gtttttcgta 2160
ttttgttttg aggatttttt taaagagata ggtaagggtt ttttttggta aaatgttttt 2220
ttaattttat gtattagttt tatttgttat tgaagaagtg ggtcgttttt tttgcgtttt 2280
tgaatttgat attttttatt ttgatatttc gtatttttga atttgatatt tttgtatttt 2340
tagtaggtaa aaaattaaag gtgtttattt aataaatcgt cgtaatgggg agtttatggg 2400
aaaatgggta tgtgtaagtt ggttaaagga taataggtat ataggttgtt tgatttggga 2460
gggtttttag atagtaggat ggtttttaga gatgtatttt tgttgttgtt gttgtttatg 2520
attatgtttt tagggtttag aatggtgttt gggtatagta ggtttttaat atagttaggg 2580
aatgaatgag tttattttaa ttacgttgta tggatggaga aattgcgttt tggggttgta 2640
ttatgttatg tatttgtaga aaatgttttt gtttttttag ttttttttta gtggcggagt 2700
ataggattta gtttataaga gtagtttaat tttgttattt gtggaatgat agaataaata 2760
agtttagggg tttagggagg taaggttttt gttttagttt tttaagtagt tggaattata 2820
gttgtatgtt attaagttta tttgattttt tatttttttg tagagatgat gttttattat 2880
gttgtttaga tttttttgga atttttgggt ttaagtaatt tttttatttt tgtttttgaa 2940
gtagttggga ttataggtat ttgttattag gtttggttag tttttatttt ttttgtagag 3000
atgggatatt attatgttgt gaaggttggt tttaaatttt tggatttaag agaggttttt 3060
gtattggttt tttaaagtgt tgggattata ggtgtaagtt attttatttg gatataatat 3120
tttatttgaa ataagaaaaa ttttttttta ttgggaatgt tgtagttgaa ttagtttaaa 3180
tttataaaga atggaagatt gtttagtggt tatttattta tttattaatt ttagatgtta 3240
aattttttag ggtgggggaa tgtgttttta tatttaaggt atttttgaat gtagttaaat 3300
tttgtagttt agttaaattt tatagttaat agttaaattt tgtagtttag ttaaattttt 3360
ttagtagtaa aatttatgat attggaattt tgttttttat attggttatt tgtaatagaa 3420
taatagagtt ttagtattag ataggttttg aaagattatt gaatttaatt tttttttttt 3480
gttagtgaga attttgaagt ttagatttgt tgatttgtga tcgatagtgt taaagatagg 3540
tttgaaatat agatgttttg ggttttagag gtgttgtttg ttttttttta tatttttttt 3600
gttttagaaa attttttttt aaaattggtt ttaataatta gaggggaaag ttatggtttt 3660
tgttttgggg atagtatggg ttggtataga aaagaggttt ataatttagt aggaagtgtt 3720
gtgcgtgcgc gcgtgtgtgt ttgtggaggc gtagggaggg ttatttgagt ttgttttggg 3780
tttggttttg ggtttagtgg taaatttaac gttgggtagg tggtttgagg ataatgacgg 3840
tgttagttgt gagttagatt ttttttattt ataaaattat gtttttttat gtagtgtttt 3900
ttaatttggt tgtacgttag gagttatttg ggtagttttg taaaaatttt aatgtttagt 3960
tggtttgtat tttttgagat tttgattaaa ttgttttgga gtttattttt ttttttttat 4020
tatttattta tttatttatt ttttaatttg gcggtttttt tgggattttt tatatgtagt 4080
aggggttgag aattgttggt ttttttggtt agatgggaat atgtaggtta atagttacgt 4140
ataaatagag tagtagagta ggacggtgtt aaggttggaa agggtgagtt atgaaagttt 4200
aggaaattag aataggaagg ggtgagaatt tttttatttg ggtttgtggg gtaggagaga 4260
agagtttagt gggtaaggtt tagtgagtgt gtttagaatt gggaagatga ggggaagggt 4320
aagcgttggg cggcggtggg taagatggac gagattaaac gggagcgagg attttagcga 4380
gagttttggc gatacggaag gttgagaatc ggagggagtt tgtcgggcga aagcgaaggc 4440
ggttgttggg taggtagtcg aattcggttg gagagtcgag ttggtgagat ttataggaag 4500
tgaagggagt cgtttagggc gtggcgtaac gagattttta gaagaaattt tgaggtagag 4560
atggggggtt ttcgtttata cggagatata aggaagttta tatgtatacg tacgagcgcg 4620
tatatgaata cgtataagta tataaacgtt tttttcgggt agggtatacg cgttcgttgt 4680
ataggtcgag gttttggatt cggaggggat tgtagagtcg atttataatt cgggtttcga 4740
tgtttttttc ggtcgcgttt ttacgattgt ttgcgtaata ggcggcggtt ttagtgggcg 4800
ttcgtcgcgc gttgtcggag tttagtttag ttcggcgcgt tcgggaggag ggagtaggga 4860
gcggggaagg ggtgtgtttc ggttgcgtgt tgggattgcg agggtttggg aggggcgagg 4920
cgcgggggcg gggtcgcggc gtttataaag tcgtttttcg ttcggacgta aataaatttc 4980
gtttggtttt tagttggtgt tgaagttcgt tagtttatta ttcgttttcg gttttcgcgg 5040
ggcgttgggt cgttagtttc ggtatcgttt tttttttttt ttttcgcgtt aggtaagttc 5100
gcgttttttt tttttaggcg cgacgcggag tagaaagggg tcgttcgtgc ggtgtcgttg 5160
gagcgttagg atcgtttttt gtagtttcgc gtattttagg agtaagcgtt tcggagttgt 5220
aagtgcgcgg ttatttcggg gcgcgggcgc gagggggttt tgggtgtaga tatcgttgaa 5280
gtggggggtt taattacgga aagggaggaa aaaaagatag ggttttattt attttttttt 5340
tagttgggga ttggggtttt ggaggtcggg aagggggcga tgagacggtt cgaaaaaata 5400
ttaggatagc gtttttggc gtcgttttcg aattttagtt ggcgtcgttg gtaggggtgc 5460
gcgttggacg tgggtgtaga agggattagg tgagtgtgtg tgtgtgtgtg tgtgtgtgtg 5520

tgtgtgtgtg tttgtgtttg ggaacggaga gattgcgcgc gagtgtgtag atgaggggtt 5580
ggtagaggtg agtggcgttg gtaaggaggt tggagagggg ggcgtgggtg tgtttggttt 5640
gtagattggg tcgggaacgg gtgtggattc gagaggttgg tggcgtttta gtttcgtagt 5700
atatataggg cggtgtgtat gtgtgagtgt aagcgtgtgt gtggggggag ggtttagggt 5760
gtgttatagt gtaatacgcg agtcggagta agtgtttgcg agcgttattg ggtagggtgc 5820
gttttcggag gaattcggga ttggcgtatc ggcggcgtgg gagaagtgat ttgggcgaga 5880
ggggtttcgc ggatatttgg cggttttttg agttggttgg agttaggagt ggcgttattt 5940
tcgttttcgt ttattttttt tcgttttttt tttttttttt gtttttggtg ttggtcgttt 6000
ttgtttttta cgggggaaaa tagttggaat tatttcgacg tttttggggg attcgtcgcg 6060
gtttttgatt tagtttcggg aggagatttc gggttgggtt tggggaatgc ggttggtttt 6120
gggggaaata cgttgttacg tttttgttaa attagtagtt ggagttggga tttagtgggt 6180
cggtttttta tttatttttt ttttatattt ttcgtttgtt gtttttgttg tttttggggg 6240
ttttgaatag tacgtttttg tggtttggaa gtgggagatg tttggtagtg ggtgggggag 6300
gtagggtttt tggttatttt atgtttattt aagaatattt tttttttacg gacgttgggg 6360
gtattttagt ttgtttggtt tttttaagtt attgggaaat atttaatatt tagaagttag 6420
ttagtggtta ggtaggaggg cggtagatat ttttataata tttattagtt gtacgggatt 6480
tttttttaag ttgttttaaa agaatatatt taaatataaa ttttttgttt ttgttgtaga 6540
aatgtagttt taaggcgaat tttttagttt agattttttt tgttgagaaa ttttggcgtt 6600
tttattgagt tttataaatg tgattgaaat aagtaaggta tagagggggt aaattttagc 6660
gttttttttg gaagggttgt tttttggtgg ggaagatata cggttggaga cgtagatcgg 6720
ttttgtgtgg tgtgtgtggc ggtagagtta gttttaggtt ggggtggtat agtgtcgggg 6780
atgaaggaag gtgtaaggga gagagagggt tttggtgtgg atggaaaatt gggtgttttt 6840
ttgatttgtg attttttagt tttgtgggat cgtgtttttt attgtatttg ggtaaggagg 6900
aggatttgtt tgttttgttt tgtaatttgg tttttggttt agcgtttggt aggtgggagg 6960
agttgaaata tgtatatggg atgaagtgga tggttttggg ttttttgttt tatttttttg 7020
tgaattagta ggttgagttt cgggcgaggt aatagattgt gtatgggtgg aggtgttggt 7080
ttttgttttt gagggatttt ggagtgtttg tttgggtttt ttgtgaggaa gtttttttgg 7140
agtaataaag tttgattatt tggaatggaa taaaaaattt atataggatt tatttagttt 7200
aatttttttt tattttatag atggaaaatg aggtataggg atggggaggg atgtgtttag 7260
gtcgaattga gttggagttt agttttgtga gaggtttttg ggtgggattg aaggtagtgg 7320
aaagggtttt tggtaggatt ttgtttgttt gttatggagg ggcgaaagga aattgtattt 7380
ttattgaatt tttggtgagg aaatttttga ggttaaaggg gtagagcgga atgatttttt 7440
ttaagtgtaa attaggtgtt tttgagagat tagtaaagtt agtttgaagg gtaggggttt 7500
agaggttgta gttttttgag ttggtttttg attgaatggg gttgtttttt ttttatgata 7560
gaagatagat tttaattgag ttgagggttt ttttgggtta cgttgaaatt cgttggtttg 7620
aatttgtttt tttatggatt tgaaagaaag atagtattgt gtttttcggg tggtttaaag 7680
ttgtagtttt tttttgggat ttaattttag agaattttat tttagatgtg atttagaggt 7740
tttgttttgg gagagaaagc gtgatgattt agtatttgag atttaagtag ggtttaggtt 7800
tgatttagtt tggaaggaag gttagggagg gatacgatat tttttttttt ttagagtagt 7860
tattgttttt ttttatttgt ttttattttc gtaagaacgt tgtttggatc gtattgtttt 7920
attgaatgga tgaggtaatt gaggttagga gagttttagg atttgtttgt tgttatatgg 7980
tttaggtggg agagttagat tttgaattcg gatagttgga ttttagagtt tttgtttttt 8040
ttttatgata atatgttggg gcgtttgaag aaattaggtt taaatgaaaa ggtagttagg 8100
agttttatgt tttagtttgt ttttttgtaa aatgagagta atagatatga tgttattaga 8160
atgtgtaaat attaatttgt gtaatttgag aatagtattg agtatgtgga aaatagcgtg 8220
taggtgtttg tcgttagtag tattcgtaga agatattttt aaggtatttt tttggtaggt 8280
agagtttggg attttttgtt ttaaatttat ttaaggttgt gaaagtgttt gtaaatgttt 8340
gatttaggta ttgttttttg ttaagtttat gtaggttgta taatagtgtt atgaaaattt 8400
tgtttgaaat gtatattttt aggttcggtt ttttgcgatt ttttttttat taggggatat 8460
gttgttgttt taaagttttt ttggttgggt tgggaatggg gtattttgta ggtagataga 8520
gttgagaatt tttgttttgg aatgataggg gagagaagaa ggaaggtgta gttatatatg 8580
tcgttgtatt tttaggttat tttgtgttta tagtgttagg gaattagtga gtgtttagta 8640
aatagttttt gtttgttata gtgttttgga ggtttaatgt attatattag ttaattgagg 8700
tagaagatat tttttttatt ttttaggtta gaaaattgag gtttaggttg gtgatttttt 8760
tgtttagagt ttagtgagtg gggttttgtt taaggtgagt tttttgagtt ttttttattt 8820
tttaatattg cgtttggagg ttatttatat tttttgggtt tattttttga gaaggagttt 8880
gttttttgga tttattttag gttgttgttt ttgttttttt tgatggttgt gtgatggaag 8940
tatcgcgtgt ttatttgttt tttatttttt aatttggaga tagaggtagg ttagatagcg 9000
gttttggttg agaatttagt ttaggtaggt aggtagaagg gttggggata ttgaggtttt 9060
ataggaaggc ggttgtagag gtagagggtt atgagtaggt gttaatttgg agtcgtgttt 9120
tcggggtggg ggttatgtga aaagaaattt gggttttttc gggtggggcg taagttttag 9180
tattatagaa tgggaatgga ttatataatc ggggttataa gtaggttttt ttgatttaga 9240
tttttgattt ttttatttaa tattgtttgt agatttgttt atgtttattt tgtttgggtt 9300

ttatttttga ttttttgagt ttagcgggtt tttttgtaat tgagttggta gaaagtgagg 9360
ttttttaata gtttgggggt tttgagtgtt ttttttagta gtttaatttt tttttttttt 9420
aaatttgtgg ggagattttt tgtagttata tattttaaat ttgtatagag ttttgtttga 9480
agagaggtgg gtgggggttt aggtttttat tttgtttttt tgtttttatt ttttgtttta 9540
tttttacgga atttaagaag attttaagta atttttttta aatatggatg tgagagggtt 9600
atttaagagg tgggtttatg gtttttttta gttgggaatg tttattattt tttttttttt 9660
ttttttaatt atggaatttt tttttttttt ttagaaaggt ttttttggtt gttttttgtt 9720
ggaggggagg ttgggttggg ttttttttgg gatttgttta tgttttttat gaaatttgag 9780
agtttgtttt tagtaggtgt tgtgttagtg gtggtggaaa ggatattttt attgattaag 9840
gaagtaattt ggattatttt gtatagataa gatcggaggt ttatttcgga gggtggaaat 9900
tgtaagatta ttttggggaa ggattttgtt agcgtggtta gtattttatt tcgggataaa 9960
gggttttgta tgaggaagtt attttttcgt tttttgttaa tattggtttc gtttgtgtga 10020
tttttataaa ttattttaga ttatttagcg tagtttatgt ttgttttgta attttataaa 10080
ataatttttt aaggagatta gtgttttttt ttggattttt agttatttat ttcgtttttt 10140
tagacggtcg ttttttgttt gttaaataaa ttattcgtgt tgttaaaatt ttattgagaa 10200
tttttaggaa gatttcggtt gtattgtaga aaagaggtgg gttttgacga gatttttgat 10260
attttttttt ttttgttgtt gtgtttggga tatttttgtt gatttttttg gtgttgtgtt 10320
taagttgaat atatagtagt ttagttttag ttttagtttt tttggattta tatttaggga 10380
gtgaatgcgg ttttggtttg tttgagaggg agatagtggg gtttattttt ttagtgattt 10440
tttttttatt ttttgaatat taaatattat tgatgttttt tgggttttag gttttattgt 10500
aaagtatagg ggttttcgag gagtttttat tgtggttttg ttttttgata tttgtattgt 10560
ttttttgatt ttgattgttt tgatatagtt ttttaatttt taaattaggg aatttttttg 10620
aaggtttagt gaatttattt atattgtttg ttgagttgat tagttaagaa agatagttcg 10680
tgaagattgt taattttttt atttttttta gggttttgag ttttagaagg gttagaaaat 10740
attaagagag tttatgaatt gttttgtagt ttgattttaa aatttttttg ggtaaagaaa 10800
tgataaagga ttgtatttat agtaaggtag ggttatttag ggtttatttt aaggtggttt 10860
tttttgaggt ttaggtagaa atggtgttgt tttcggtaat tttttgggga agttgttata 10920
gtaaatagtt atagtatatg ttgatttttt tttttttttt aaaaatttgt tttataaaga 10980
gtataatgtg ttggagagaa tattggtttt tatgtggttt taaaattagg tatgttatag 11040
agggtggaat ttgggttagt tgttgttggt gataatcgat gttatttttg ttagtaattt 11100
tcgtttgttt tttttttatt tgttttattg aaggttttta ggggattgtt gttcgttttt 11160
tttcgaggga atgagtgtta tatttgtatt tataataaat tagtgagttt tttatttttt 11220
ttttagtagg gaggaattta gatatgtggt ttttggaagt taagtgggtt tttgtgtttg 11280
tttttgagtt ttaggttatt tatttgtgaa ttgggtataa tatttagttt ttttcggggt 11340
aatttttttg aggagtaagt gaaatagtgt gttgagtgaa tgttttttta aagtattgag 11400
ttggtcgttt taggaaggta ttatggggga gttgaaaata gagaattttt tgggtgttgt 11460
gaagggagaa aagtttaaga tgttggtttt gttaaaagtt taagtttgtt tatgatggtt 11520
tagtggtgat tgtagggtgg aggagaggta tgtgattgtt ttagttgatt tttatacgtt 11580
gaaagtttta tggttacgtg tagtttattt tataacgttg tatttgggtt acgggtggtg 11640
gtgaggattg tggatatatt tagggtaatg tgagattatt gttaatattt tttttaaggt 11700
tgttttaatt ttaaagtagt tagtttgatg tttgttgatt tggggtatat attttaattt 11760
tttgatgttt tagttttttc gtttgttaaa tggtgatatg ttggtatttt ttttttaggg 11820
tttttgtgag gtttaaagga agtatcgggt ttagagttta gttagtgtta tagatattgt 11880
ttttggtgtt gtttttgtgg tgtgtttgtg tagggttata ttgttgtttt tttgtggttg 11940
ttttttttgg tgggcggaat ttggaatttt tagaatagat tatggatgtt tttttgttgg 12000
atattttttt agttttattt tgtgggagtt gtggcggggt ttaggagagt ttgtttaaga 12060
gggtgtgtag gtgggtggta gagttggagt aaggtattag ttttaataga taggagtttg 12120
aggttggtag aagtgggggt tttggtttaa ggtgcggggt taatttggtt agagttgagt 12180
ttggtagggt tttggtaaga ttttgtggtt tttttgtttt ggtgaagtat ttttttgttt 12240
gggtttttat ttgtttgtaa gtttgttgtg gatagcgtga tttttataga cgtatattgt 12300
gtttatttta tagatggaga aattaaggtt tagagaggaa gtgatttgtt tagagttatg 12360
gaataggaaa gtggtagggt ttttatgttt tgatttttag gttagcgttt ttgttgtttt 12420
attgaggttt ttgggaatta taggttttgt gtgttttttt tagtttttta ggggttgttg 12480
aatagttttt aagtggtgtc ggattattga agatgttttt gtttattgtt tttttaggtt 12540
ttagggttac gggttacgtt tttttttttt tcgggtagat tttggttttg gttttgtttg 12600
gtatttgtga tttagttttt ttttgatttt tttgggtttg ttggattgga agggtatttg 12660
tgtagttttt tttgggtttt tattatttat tgatagatag tgttggtgga gagaagggtt 12720
ttagtggtga cgtattgggt ttgataatta gaaattttgg tggtatttgt tgagtatttt 12780
ttatgtgttt tttttttcgt tgggatttta ggattgaaat ttaggttata tttttataga 12840
tgttttggta tttttaaggt aattttgaaa ggaaagtatg ttgatgaatt aagtaattgt 12900
ttgtttaatt ttttatgtta tttttgaatt taagttttat tgttgattat tgggagtagg 12960
agttagtatg tttattgata gataagtaaa ttgaggtaaa gaggttagat agttaggtat 13020
ggtggtatat gtttgtaatt ttagttattt aggaggtcgg agtaggagga ttatttgaat 13080

ttaggagttt gagattagtt tgggtaatat agtaagattt tgttttttta aaaaataaga 13140
tgaataatat atttttaaaa agagattcga taaattatgt aggattatag agttaataaa 13200
tggtgatatt atggtttgta agtttttttt tagtttttgg ttatttttcg tttttttggt 13260
attggttttt tcggaatttt ttttaggtag gtggatggga aaagaagtga agatataggt 13320
tggtagttgg tgttgtgatt tggagttttg tttttggtgt taagttgggt tttagttgaa 13380
ggagatttgt tggaggggtt ttttttgtgt tttttgggga gtaaagatgg ttttggagat 13440
ggagatacgg tttgttagtt ttaagttatt ttaaaggtgg ggttggtggg ttaggagtta 13500
gttttggttt tttcgttatg ttgtgtgtgg ttttgttttg ttattttatt tgttggagga 13560
tttttttgat gttttttagt gggttttaat attttgtgat taggattttg tatgaggtag 13620
agggagggta ggagttggag tttagtaagg attttaagtt tagggatagg ttttatttgt 13680
tatatttgtt aagtcgtgag ggttttaggt gttcgggagg agggagtagg agtggaagta 13740
ttagttcgag tcgttaaatt aggttacgta ttatttgtta ttttttgttg gtagaaggag 13800
attatagata ttagaagata gtagtttttg ttagaattgg gtttttttat gatttttagg 13860
tggtgatttt gaattttaga aaaagtgttt tggggagagt ggagtgaagg aagagtgtga 13920
atattggatt tgattttatt ttagtttttg tgttgtagtt agtagggtga gtttaggata 13980
gttggtagtc gtttgggttt agtttttttt ttcgtgaatg aaggtggtgg ggagtgtacg 14040
tgtgcgtgtg agtgcgtgtg agtataaatg attgtggatg tgagagttta tgaaagtgag 14100
aatgtattag tgtgtaagaa agtgtgagta tgtgagaatg agtgtgtttg tgagaatgta 14160
tgtatgtgtg agtataagtg tgtgaatgtg agaatgtttg agtgtgagaa tgaaagtttg 14220
taagtgtgtt agtgtgaggg aatgtgtgag tgtatttgag agtgtgtgtg tgtgtgtgtg 14280
cgtgtgtgta cgtatgtatt agattaaata agaatgtatt taattgatat tttttggata 14340
ttgttattta ttaggaatta ttaaaattag ttattttaga ggagggtgga tgtcgtatta 14400
ggggatattt ggtaacgttt gtagattttt ttgttggtcg tagcgtgggg tgtatgttgt 14460
gggtatgtgg tgggtagagg ttagggatgt tgttgagttt tttttcgtgt ttagggtagt 14520
ttttaaggta gaggttgttt gtggggttta aatgttagta gtgttgagtt gaggattttt 14580
gtttaggagg tatagacgtt gttttggatg ttgtttagat agtatttgtt ttgtttttta 14640
gggggataga tagatagtgg atatagttta gatgtagttt gatgagtgag tttaagggag 14700
tgtttgtttt ttcgggattg tgagtttcgt gggggtagga attgggtttg tttgtttatg 14760
atttttagat gagttttata gttagtttta ttattatagt tggtgttatt tttgttttga 14820
gttttgtgtt gggtatatag aatttagtgg gtagaaattt ttgaataaat tttgagtttt 14880
ttatttgtt ttcgtgaatt tagatgtttt ttgaggttag gtttggtggc gtgggggttg 14940
ggagtgggta gattgtaggg agttaagttt tggggtggag tggggtttag gttttttttt 15000
ttggtttgag tcgaagtatt ttggtttttt aaattttaaa atttttattt tttaatattt 15060
ttatatgttg aattttttta tatgattttt tttggataaa agtttttgta aaaaaagttt 15120
gaaaagtttt ttggttgagg tattagggat aaggtaaggg ttgtttgatg ggtttaattc 15180
gggagaattg aggtgaggga gtcgtttttt ttttatttta taggttggtt agatggtgga 15240
tcgttagtag ttggttgggt taatttgtag agagtatttt ttgtagtttt tggtttatgc 15300
ggttttagta ttgtaggacg cgattaatat gtaggttatt tcgaggtttg tagtttttat 15360
agttttttta gattagtgta aagttttatt tagtttttcg gtagatttta gagtttagga 15420
tattttttaa atggtttaaa attaatggaa ttatttgggg gataataaat aatgttatta 15480
gatattgtta aatgtttttt gggagagaaa atttttttta gtttagatat atttcgtgat 15540
tatttttagt agttttattt ttggattttt taaaggtttt tagtagtgag ggggttgggt 15600
tagagaaggt tttattagtt tttaaaagta gatgtttaag aatttgatat ttttaggata 15660
tatagcgatt tttttaattt attgttttat gtggtttggg aagttaagtt gtaggaataa 15720
aaaaggtgtc gggtagggta aagaggtttt attagataga ggaaacgggg aaggtggata 15780
tttttgtttt ttttgttttg gagaacgaga gagttggtgg agattgatag atattatgtt 15840
ttaatgtgat tacggttttt ggaagttaaa gagaattgat taaattgggt tgaattatag 15900
tatttttcga gtttttttaa atgtagaatt tgttttttag atattgtatt tttggggttt 15960
agtaatgatt taaaagtgaa ttttattgaa agtatgttgt tgagttatat tgtttattta 16020
tttagagagg gagttgtagt ttgatttttt gtttaggaga gtttttcgtg ttgggagtta 16080
gaggtagaga aaagaataag ttatttgttt gatgaagttt atagatagga ttgtttgaat 16140
ttatttggtg ggatagtgag agttttttga ttttattttg gcgcgttttg ttgagtaggg 16200
tatgttaaga agtttttgtg aaatgagaag tttttttatt tttagaatga ggggtggcgt 16260
tggtagggtt ttgggttttt ggaaggtttt ttaggttatg taggcgaggt gttttatagg 16320
ttagggtttt gggattacga atttgttgaa ttagtaaggt taatagacgc gggtttttta 16380
tgttttttgt ttttaagttg gtagttgttt aggaatgttt ataaatataa aatcgatttt 16440
gtttttagaa tgttttggag tatagatttt aaagtttgta taaagttatt tttgtggggt 16500
tggttattta ttggggaaaa ttatgttggg gttttagatt agagtatttt agtggagagt 16560
aggatttgtt tacggaatgt tatgtgttgg tataagggat ggattcgggg tggattcggg 16620
gtggttcgta tttgtcggtt gatttttaag aggtgtttta ttaggttttt gggagttttt 16680
gggtttttag aggagtttta ggtaggaatt ttagaggttt gtattttttc gattattgta 16740
ttcgaggggt tttgggtagt gcggggttta ggtttttatt ttgaagttgg ttgtgtgagg 16800
gttttggtta ggcgtttttt atttagaaag tttggatttg tgaattgggt gggtggtttg 16860

ggtttttttt ttttgttatt tatttttttt tcgtcggttt tttgttgttg tttgtaagat 16920
ttgattttat tttgtatttt ttatgtttta ttttagggat tgatgggtat ttagtttttt 16980
taatttgatt attgatttag aagaatatat gtttatattt ttggttttta gatttagttt 17040
ttggaggtat gtttaataga tagtattatt tgtttaagtt ttgtatttta gggagttagt 17100
ggtagtattt taagtttttt tgggttgtgt tttagtttgg ttttgtagtt tatatttttg 17160
tatttgtttt gtgttagagt ataggtaatt tttttggttt attttttgtt ttgatttagg 17220
aagtgttttt tgttttggag gttttttttt ttataaaatt gtattgtgcg tttaaatgtt 17280
tgttaaattt tttgatttaa ggtttttttt tgtttttttt tttttttagt aggtagtcga 17340
gagttttgta tcgtggggtg tgtatgtgtg tttacgcgta tgcgtgtgtt tgtgtgtttg 17400
tgcgtttgta ttattaattt tttgttaggg ttaattgtga ggttgaagtt tgtattatgt 17460
ttatacgtgg taagtatcgt gtgagtttgt gttttttttt tattgttata cgggggttgt 17520
ttgattttgg gattagtaat gtaggttttt ttttagaatg tagtgagttg tattttgggt 17580
atggtgggtg ggtttgaatt tggtatgggg atttgtttgt gaggttgatt tatttatttt 17640
attgggaggg tggtgtttgt gagttttagt attttttttt aggaaattta ttgcgttggt 17700
tttaatggga agattgggtt gggtttatgg gttgtgtgta taagggattt ttttattttt 17760
tgtagtgtat tacgagggag gtgaagttgt tgggtagagc gtaggtttgg tttttttgtt 17820
attggttttg gatttgtttt ttttaggaaa ttttttagta ggaagtgttg attttttggt 17880
ttttgttttt ttgtagg 17897

<210> 234
<211> 17897
<212> DNA
<213> Artificial Sequence

<220>
<223> chemically treated genomic DNA (Homo sapiens)

<400> 234

tttgtaagga gataaaggtt aaaaggttaa tattttttgt tgagaggttt tttaaaggaa 60
gtaaatttaa gattaatggt aagaaggtta ggtttgcgtt ttgtttagta gttttatttt 120
tttcgtgatg tattgtaggg gatgaggagg ttttttgtat atatagttta tggatttaat 180
ttagtttttt tattggggtt agcgtagtgg attttttggg agaggatatt gaggtttata 240
gatattattt ttttaatgaa atggatagat tagttttata aataggtttt tatattaagt 300
ttaggtttat ttattatatt taggatgtag tttattgtat tttagaaggg gatttgtatt 360
gttgatttta aagttaggta gttttcgtgt gataatgaaa ggggaatata agtttatacg 420
atgtttatta cgtataggta tagtgtaagt tttagtttta tagttagttt taatagaagg 480
ttggtgatgt agacgtatag atatataggt atacgtatgc gcgtgaatat atatatatat 540
tttacggtgt aggattttcg gttgtttgtt aaaagggaag aggggtagag gaaaatttta 600
agttaaggag tttggtaggt atttgaacgt atagtataat tttgtaagaa aaaagatttt 660
taaggtagaa agtatttttt ggattaaagt aggggatgga ttagaggggt tatttgtgtt 720
ttaatatagg gtaggtgtag aagtgtgggt tgtaaaatta gattgaagta tagtttagga 780
agatttggaa tgttgttatt gattttttgg ggtgtaaggt ttgggtaggt ggtgttattt 840
attaagtatg tttttaagag ttgaatttag aaattagaag tataagtatg tattttttta 900
gattagtagt taagttagaa aaattaaatg tttattaatt tttggggtga agtataaaaa 960
atgtagaata aggttaagtt ttatagatag tagtagagga tcggcggaga agaagtaagt 1020
gataagaggg aagagtttag gttatttatt tagtttatag atttagattt tttgaatgga 1080
aaacgtttga ttaaagtttt tatatagtta gttttagagt gggaatttgg gtttcgtatt 1140
gtttaaggtt tttcggatgt aatggtcgag agaatgtaag tttttgaaat ttttgtttag 1200
ggtttttttg aagatttagg aatttttaag aatttggtga aatatttttt agaggttagt 1260
cggtaagtgc gggttatttc gaatttattt cgggtttatt ttttgtgtta atatatggta 1320
tttcgtgggt agattttgtt ttttattggg gtgttttggt ttggggtttt agtatgattt 1380
tttttagtag ataattagtt ttataagaat ggttttatat agattttgga gtttatgttt 1440
tagaatattt tgaaaataaa gtcggtttta tatttgtgag tatttttggg tagttgttag 1500
tttggaggta ggaggtatga aaggttcgcg tttgttagtt ttgttaattt agtaggttcg 1560
tggttttaag attttggttt gtgggatatt tcgtttatat ggtttaggga gttttttaga 1620
aatttagggt tttgttaacg ttatttttta ttttgggagt gaaaggattt tttattttat 1680
aggagttttt tagtatattt tgtttagtaa agcgcgttag ggtgaagtta gggggttttt 1740
attgttttat taggtgggtt taagtagttt tatttgtgag ttttattaag taggtagttt 1800
gttttttttt ttatttttag tttttagtac gagagatttt tttaggtaga aagttaggtt 1860
gtaatttttt ttttgaataa atgagtaatg tagtttaata gtatgttttt agtaaaattt 1920
atttttaaat tattgttaaa ttttaagagt gtaatgtttg gaaaataaat tttatattta 1980
aaaggattcg agaaatgttg tggtttagtt tagtttggtt agtttttttt ggtttttaga 2040

aatcgtgatt atattaggat atagtgtttg ttagttttta ttagtttttt cgttttttag 2100
aatagaaaag atagaaatgt ttattttttt cgtttttttt atttggtgaa atttttttat 2160
tttattcggt atttttttta tttttatagt ttagtttttt agattatatg aggtaatggg 2220
ttaaaaaaat cgttgtgtgt tttagaaata ttaagttttt gagtatttgt ttttgggagt 2280
tggtggggtt ttttttgatt tagttttttt attgttaaag gtttttggga agtttagaga 2340
taaaattgtt gaggatgatt acggggtata tttaaattag gggggatttt tttttttagg 2400
ggatatttgg taatgtttag tgatattatt tattgttttt taaatgattt tattgatttt 2460
gaattattta ggaagtgttt tagattttga agtttgtcgg gagattggat aaagttttgt 2520
attagtttgg ggggattgtg ggggttgtag gtttcggggt agtttgtatg ttggtcgcgt 2580
tttgtaatat taaggtcgta tgaattaagg attgtagaag gtgttttttg taggttgatt 2640
tagttagttg ttggcggttt attatttggt tagtttgtag ggtgggagga gagcggtttt 2700
tttattttag ttttttcgag ttgggtttat taagtagttt ttattttgtt tttgatgttt 2760
taattaaaaa attttttaaa ttttttttgt aggagttttt atttaaagga aattatgtaa 2820
ggaagtttaa tatataaaaa tgttaaaaaa taaaaatttt aaaatttaaa aagttaaggt 2880
gtttcggttt aagttaggga aaggggtttg aattttattt tattttagga tttggttttt 2940
tataatttgt ttatttttag tttttacgtt attaggttgg tttttaaaga atatttgagt 3000
ttacggaagt aggatggaaa atttaggatt tatttagagg tttttattta ttaggtttta 3060
tgtgtttaat atagagttta gaataaaaat aatattagtt atggtgatga gattggttat 3120
gggatttatt tggggattat gagtaaatag atttagtttt tgtttttacg gagtttatag 3180
tttcgagggg ataggtattt ttttgggttt atttattagg ttgtatttgg gttgtgttta 3240
ttgtttgttt gtttttttgg agggtagagt agatgttgtt tggatagtat ttaggatagc 3300
gtttgtgttt tttggataga gatttttagt ttagtattgt tgatatttgg gttttataag 3360
tagtttttgt tttaggggtt gttttgagta cgggaggagg tttagtagta tttttggttt 3420
ttgtttatta tatgtttata gtatatattt tacgttgcga ttagtaaaag aatttgtaaa 3480
cgttgttaaa tgttttttgg tgcgatattt attttttttt ggaatagttg attttagtaa 3540
tttttagtag gtaatagtat ttagaaaatg ttagttggat gtatttttat ttaatttagt 3600
gtatgcgtgt atatacgtat atatatatat atatattttt aaatgtattt atatattttt 3660
ttatattgat atatttgtaa atttttattt ttatatttag atatttttat atttatatat 3720
ttatatttat atatatatat atttttataa atatatttat ttttatatat ttatattttt 3780
ttatatattg atatattttt atttttatgg atttttatat ttatagttat ttatatttat 3840
acgtatttat acgtatacgt gtatttttta ttatttttat ttacggagga gggggttggg 3900
tttaagcggt tgttagttgt tttgagttta ttttgttggt tgtagtatag gggttggagt 3960
ggagttagat ttaatgttta tatttttttt ttattttatt ttttttaaaa tatttttttt 4020
gggatttaaa gttattattt gagaattatg gaaggattta gttttggtag aagttgttgt 4080
tttttggtgt ttgtgatttt tttttgttag taggaaatgg taggtgatac gtgatttggt 4140
ttggcggttc gagttggtgt ttttattttt gttttttttt ttcgggtatt tgaggttttt 4200
acggtttagt aagtgtggta ggtggagttt gtttttgggt ttgaggtttt tgttggattt 4260
tagtttttgt tttttttttg ttttatgtaa agttttaatt atagaatgtt gggatttatt 4320
agaaagtatt aaagaggttt tttagtaggt ggggtggtaa ggtagggtta tatatagtat 4380
ggcgggggaa ttagggttga tttttagttt attagtttta tttttggagt ggtttgggat 4440
tgataaatcg tgtttttatt tttaaggtta tttttgtttt ttaagaagta tagaagaaat 4500
ttttttagta ggtttttttt agttaaaatt tagtttagta ttaaaaatag ggttttaggt 4560
tatagtatta gttgttagtt tgtattttta tttttttttt tatttatttg tttgggagga 4620
atttcgagaa gattaatgtt agagaagcga aaggtagtta ggagttgggg gaaggtttgt 4680
aggttatggt gttattattt attagttttg tgattttgta taatttatcg aatttttttt 4740
taaaaatgta ttatttattt tgttttttag agagataggg ttttgttatg ttgtttagat 4800
tggttttaaa tttttgggtt taagtgattt ttttatttcg gttttttgag tagttgagat 4860
tataggtata tgttattatg tttggttatt taattttttt gttttagttt gtttgtttgt 4920
taatgggtat gttaattttt gtttttaatg attagtagtg gggtttaaat ttaagaatgg 4980
tatggagaat taagtaaata gttgtttaat ttattaatat attttttttt tagggttgtt 5040
ttggagatgt taggatattt gtgaggatgt ggtttgggtt ttagttttag agttttagcg 5100
aggagaggga tatatggaaa atgtttagta aatgttatta aggtttttga ttattaggtt 5160
tagtgcgtta ttattggggt tttttttttt attagtattg tttgttaatg agtaatgagg 5220
gtttaagagg ggttatataa atattttttt agtttagtag atttaggaga gttagaggag 5280
agttggatta taggtgttag gtaaagttaa ggttaggatt tgttcgagga agaaaagaac 5340
gtgattcgtg attttgggat ttgggagggt agtgggtaga ggtattttta gtaattcgat 5400
attatttgag aattgtttaa tagtttttgg gggattgagg gggatatata gagtttgtga 5460
tttttaaaaa ttttagtggg gtagtaggaa cgttggttta ggaattaggg tatggggatt 5520
ttgttatttt tttattttat gattttgagt aagttatttt ttttttgggt tttggttttt 5580
ttatttgtaa agtaggtata gtgtacgttt atggagatta cgttgtttat aataaattta 5640
taggtaaatg gaaatttagg taggaaggtg ttttattaag ataaaagggt tatagggttt 5700
tattaaggtt ttgttaggtt tagttttggt tagattagtt tcgtattttg gattaaggtt 5760
tttatttttg ttagttttag atttttgttt gttaagatta gtgttttgtt ttagttttgt 5820

tatttatttg tatatttttt tgagtaaatt tttttgggtt tcgttataat ttttataggg 5880
tgaggttgaa ggggtattta gtagagaaat atttatgatt tgttttagaa gttttaggtt 5940
tcgtttatta ggaagagtag ttataggaag gtaataatgt aattttatat agatatatta 6000
taaaaatagt attaggaata gtatttgtgg tattaattgg attttaaatt cggtattttt 6060
tttaagtttt atagaaattt taggagggag gtgttagtat gttattattt aatagacggg 6120
gaaattgagg tattaagagg ttaagatgtg tgttttaagt tagtaagtat taagttggtt 6180
gttttaaggt taaagtaatt ttaaagggaa tgttgataat gattttatat tgttttaaat 6240
atatttatag tttttattat tattcgtggt ttagatataa cgttgtaagg tgggttgtac 6300
gtgattatga ggtttttagc gtgtggaggt tagttgggat agttatatgt ttttttttta 6360
ttttgtagtt attattgagt tattatgagt aggtttgagt ttttggtaaa attagtattt 6420
tgaatttttt tttttttata atatttagaa ggttttttgt ttttagtttt tttatagtat 6480
ttttttaaga cggttaattt aatgttttgg aaaagtattt atttagtata ttgttttatt 6540
tgttttttaa ggaggttgtt tcgggagagg ttgggtatta tatttagttt ataaatgggt 6600
aatttgaggt ttaaaaataa atataaaggt ttatttgatt tttaaaggtt atatgtttag 6660
attttttttt attgagggaa aggtgaaaaa tttattggtt tgttgtgggt gtaggtgtgg 6720
tatttatttt ttcgggggag ggcggataat agttttttgg aggtttttag tgaagtagat 6780
aaagggagag taggcgggaa ttgttgataa gggtagtatc gattgttatt aatagtaatt 6840
ggtttagatt ttattttttg taatatgttt ggttttaagg ttatatgaaa gttagtgttt 6900
tttttagtat attgtgtttt ttatagaata gatttttaaa aggagggaaa aaattaatat 6960
gtgttgtggt tgtttgttgt agtaattttt ttagaaaatt gtcgagaata gtattatttt 7020
tgtttgaatt ttaaggaaaa ttattttgag ataggttttg gatagttttg ttttattgtg 7080
gatataattt tttgttattt ttttgtttaa agagattttg aagttaagtt ataaagtagt 7140
ttatgggttt ttttggtgtt ttttggtttt tttgaaattt aaggttttgg ggagggtgag 7200
gaagttggta gtttttacga attgtttttt ttaattaatt aatttagtaa ataatatggg 7260
tggatttatt ggatttttaa gaaagttttt tagtttagag gttagggaat tgtattagag 7320
taattagaat taaggaaata gtataaatgt tagagaataa aattatagta ggaatttttc 7380
gagggttttt gtattttgta gtggagtttg aaatttagga ggtattagtg atgtttgata 7440
tttaaggaat gaaaaaggga ttattaagaa ggtgaatttt attgtttttt ttttaaataa 7500
gttaaagtcg tatttatttt ttgggtgtgg gtttagaggg gttggagttg aaattgaatt 7560
gttgtgtgtt taatttgagt atagtattag ggaggttagt aagagtgttt taaatataat 7620
agtaggaaga gggaggtgtt aggaatttcg ttagggttta tttttttttt gtaatataat 7680
cgagattttt ttggagattt ttagtggagt tttggtagta cgagtaattt gtttggtaga 7740
tagaagacga tcgtttgagg ggacgaggtg aataattgga agtttaaagg aggatattgg 7800
tttttttaga ggattatttt gtgaaattgt aagataggta tgagttgcgt tggatggttt 7860
gagataattt gtggaaatta tataaacgga gttagtgtta gtagaggacg aggaaatggt 7920
ttttttatat agaatttttt gtttcgagat gggatgttag ttacgttaat aggatttttt 7980
tttagaatgg ttttgtagtt tttatttttc ggaatgggtt ttcgattttg tttatgtaag 8040
atggtttaag ttattttttt aattagtgga gatgtttttt ttattattat taatataata 8100
tttattaagg ataggttttt aggttttatg ggagatatgg ataggtttta ggaaggattt 8160
aatttagttt tttttttagt agaggatagt taaggaggtt tttttggaga aaggaggaga 8220
gttttatgat taaaaaggag gaaggagaga tggtaagtat ttttagttga gagaggttat 8280
agatttattt tttaaatggt ttttttatat ttatatttga agagggttat ttggaatttt 8340
tttaggtttc gtgggggtgg ggtagagggt gaaggtagga gaataaggtg gagatttggg 8400
tttttattta ttttttttta aatagaattt tatgtagatt taaaatgtat agttgtagaa 8460
agttttttta tagatttaaa gaaaggaaag attaaattgt tggggagggt atttaaggtt 8520
tttagattgt taaggagttt tattttttat tagtttagtt gtaagaaaat tcgttggatt 8580
taaaaggtta gagatggagt ttaggtaggt agggtatagg taggtttgta aataatgtta 8640
ggtgggaaag ttaaagattt gggttaaaga gatttgttta tggtttcgat tgtgtaattt 8700
atttttattt tgtagtgttg gggtttacgt tttattcgag ggagtttagg ttttttttta 8760
tatggttttt atttcgagaa tacggtttta ggttggtatt tatttatggt tttttatttt 8820
tgtagtcgtt tttttgtggg gttttagtgt ttttagtttt tttgtttatt tgtttgggtt 8880
aagtttttag ttaagatcgt tatttagttt gtttttattt ttaagttgaa agataaaaag 8940
taagtggata cgcggtattt ttattatata gttattaagg gaggtaggaa taatagtttg 9000
gggtggattt aggaaataag ttttttttta aaaggtgggt ttagagggtg tgggtggttt 9060
ttaagcgtaa tgttggaggg tggaaagggt ttaggaggtt tattttgggt agaattttat 9120
ttattggatt ttggatagaa gagttattag tttgagtttt aattttttga tttgagaggt 9180
ggaaagaatg ttttttattt tagttagttg atgtgatgta ttgaattttt aaggtattat 9240
agtaaataag agttatttat tgagtattta ttaatttttt ggtattgtgg atatagggtg 9300
gtttgaaggt atagcggtat gtatgattat attttttttt tttttttttt tgttatttta 9360
gagtagaggt ttttaatttt gtttgtttgt aggatatttt atttttaatt tagttaaagg 9420
agttttgaaa taatagtata ttttttgatg gagagagaat cgtaggagat cgggtttgga 9480
aatgtgtatt ttagataaga tttttatgat attattatgt aatttatata agtttagtaa 9540
aaagtaatat ttaggttaag tatttgtagg tatttttata gttttgggta ggtttaggat 9600

aaagggtttt agattttgtt tgttaagaag atgttttgag aatgtttttt gcgggtgtta 9660
ttgacggtaa atatttatac gttgtttttt atatgtttag tgttgttttt aggttatata 9720
gattaatatt tatatatttt gatggtatta tgtttgttat ttttatttta tagaaaagta 9780
aattgaggta tggagttttt gattgttttt ttatttgagt ttgatttttt taggcgtttt 9840
aatatgttgt tatggaaaaa aaataaaggt tttgaagttt aattgttcgg gtttaaagtt 9900
tggttttttt atttgagtta tatggtaata ggtaaatttt gaaatttttt tggttttagt 9960
tgttttattt atttaatgga gtaatacgat ttaggtaacg tttttgcgag gatgaaggta 10020
gatggaggga gatagtggtt gttttggagg gagagagatg tcgtgttttt ttttggtttt 10080
ttttttaggt tgagttaaat ttgaattttg tttgagtttt agatgttgag ttattacgtt 10140
ttttttttta ggataaggtt tttgagttat atttggaatg ggattttttg gggttgggtt 10200
ttaaggaaga attgtagttt tgggttattc gaaaaatata atgttatttt ttttttaggt 10260
ttatgaggag gtaagtttag gttagcggat tttaacgtaa tttagggggg tttttagttt 10320
agttgagatt tgttttttgt tatggggggg gggtagtttt atttagttaa aggttaattt 10380
agggggttgt agtttttaag tttttgtttt ttaggttggt tttgttaatt ttttaagaat 10440
atttgattta tatttaaaaa aaattatttc gttttgtttt tttggtttta aaagtttttt 10500
tattagaaat ttaatgaaga tgtaattttt tttcgttttt ttatagtagg taggtaaaat 10560
tttgttagga atttttttta ttgtttttag ttttatttag gagtttttta taaagttgag 10620
ttttagttta gttcggtttg ggtatatttt tttttatttt tgtgttttat tttttatttg 10680
taaaatggaa agaagttgga ttaggtaggt tttgtgtgag ttttttattt tattttaaat 10740
gattaaattt tgttgtttta aaagaatttt tttataagag atttaaatag atattttagg 10800
attttttaaa agtaaaagtt aatattttta tttatgtata gtttattatt tcgttcgaaa 10860
tttaatttgt tggtttatag gagaatgggg tagaagattt agagttattt attttatttt 10920
atgtgtatat tttaattttt tttatttgtt aggcgttggg ttaggggtta ggttataggg 10980
tagggtaggt aaattttttt ttttgtttag atgtagtggg agatacggtt ttataaagtt 11040
aagaagttat aggttagggg aatatttagt tttttattta tattaaaatt tttttttttt 11100
tttatatttt tttttatttt cgatattgtg ttattttagt ttggggttgg ttttgtcgtt 11160
atatatatta tatagaatcg gtttgcgttt ttagtcgtgt gtttttttta ttagaaggta 11220
gtttttttaa gaaaggcgtt ggggtttgtt ttttttgtat tttgtttatt ttaattatat 11280
ttgtgaaatt taataagaac gttagaattt tttaatagga agagtttaga ttgaaaaatt 11340
cgttttgaaa ttatattttt gtagtaagaa tagggggttt gtatttagat atgttttttt 11400
gaggtagttt aggagaaggt ttcgtgtagt tgatgggtat tataaggatg tttatcgttt 11460
ttttgtttgg ttattggttg atttttgaat attagatatt ttttagtagt ttaaagaaat 11520
taaataaatt gaagtgtttt tagcgttcgt gaggaagggg tgtttttgga tgggtataag 11580
ataattaagg gttttgtttt ttttatttat tgttaggtat tttttatttt taagttatag 11640
aggcgtgttg tttaggattt ttagggatag tagggataat aggcggggga tatgggggag 11700
gggtgggtaa ggaatcgatt tattaaattt tagttttagt tattaatttg atagaggcgt 11760
gatagcgtgt tttttttaga attagtcgta ttttttaagt ttagttcgaa attttttttc 11820
gaggttgaat tagagatcgc ggcggatttt ttaggaacgt cgaagtaatt ttagttgttt 11880
tttttcgtgg agggtaggag cggttagtat tagaggtagg ggaggggagg aaagcggggg 11940
agggtgggcg gaggcgaaaa tgacgttatt tttagtttta gttagtttaa ggggtcgtta 12000
aatgttcgcg agattttttt cgtttaggtt atttttttta cgtcgtcgat gcgttaattt 12060
cgggtttttt cgggggcgta ttttgtttag tggcgttcgt agatatttgt ttcggttcgc 12120
gtgttgtatt gtgatatatt ttggattttt tttttatata tacgtttata tttatatatg 12180
tatatcgttt tgtgtgtgtt acgggattga agcgttatta gtttttcgga tttatattcg 12240
ttttcgattt agtttgtaag ttagatatat ttacgttttt ttttttagtt tttttgttag 12300
cgttatttat ttttattaat tttttattta tatattcgcg cgtaattttt tcgtttttag 12360
atatagatat atatatatat atatatatat atatatatat atatttattt agtttttttt 12420
gtatttacgt ttagcgcgta tttttgttag cggcgttagt tgggattcgg agacgacgtt 12480
aggaaacgtt attttggtat tttttcggat cgttttatcg tttttttttc gatttttaga 12540
attttaattt ttagttaggg gggagatgaa tgggattttg tttttttttt tttttttttc 12600
gtaattgaat tttttatttt agcggtgttt gtatttaaag ttttttcgcg ttcgcgtttc 12660
ggggtgatcg cgtatttgta gtttcggggc gtttattttt ggagtacgcg gggttataaa 12720
gggcggtttt ggcgttttag cggtatcgta cgggcggttt ttttttgttt cgcgtcgcgt 12780
ttgggagaag agggcgcggg tttatttaac gcgagggaga aaggaaagga cggtgtcgag 12840
gttggcggtt tagcgtttcg cggaagtcga gggcggatgg tgaattgacg agttttagta 12900
ttagttggga gttaggcgag gtttgtttac gttcgggcgg agggcgattt tataggcgtc 12960
gcggtttcgt tttcgcgttt cgtttttttt agattttcgt agttttagta cgtagtcggg 13020
atatattttt ttttcgtttt ttgttttttt ttttcgggcg cgtcgggttg ggttgggttt 13080
cggtagcgcg cggcgggcgt ttattggagt cgtcgtttgt tgcgtaagta gtcgtagggg 13140
cgcggtcggg aggggtatcg gggttcgggt tgtgggtcgg ttttgtagtt tttttcgagt 13200
ttagggtttc ggtttgtgta gcgggcgcgt gtgttttgtt cggaggaggc gtttgtgtgt 13260
ttgtgcgtgt ttatgtgcgc gttcgtgcgt gtgtatgtgg atttttttgt gttttcgtat 13320
gggcggaggt tttttatttt tgttttagag ttttttttaa gagtttcgtt gcgttacgtt 13380

ttgagcgatt ttttttattt tttgtaggtt ttattagttc ggttttttag tcggattcgg 13440
ttgtttgttt agtagtcgtt ttcgttttcg ttcgataggt ttttttcgat ttttagtttt 13500
tcgtgtcgtt aaagttttcg ttgaaatttt cgttttcgtt taatttcgtt tattttgttt 13560
atcgtcgttt agcgtttatt ttttttttta tttttttagt tttaagtata tttattggat 13620
tttgtttatt gggttttttt tttttgtttt ataagtttag atgagaggat ttttattttt 13680
ttttattttg attttttgaa tttttatgat ttattttttt taattttagt atcgttttgt 13740
tttgttgttt tgtttgtgcg taattattgg tttgtatgtt tttatttggt taaagaaatt 13800
agtaattttt aatttttatt gtatatgaga agttttagag gagtcgttaa attaaaaaat 13860
aaataaatag atagataata aaaagagaga gatggatttt agaatagttt aattagaatt 13920
ttagggggtg taaattaatt gggtattggg gtttttataa agttatttag gtggtttttg 13980
acgtgtagtt aggttgagaa gtattgtatg gaggaatatg attttatgaa taaaggaagt 14040
ttgatttata gttggtatcg ttattgtttt taggttattt gtttagcgtt gaatttgtta 14100
ttgagtttag gattagattt agggtaagtt taggtgattt tttttgcgtt tttatagata 14160
tatacgcgcg tacgtataat attttttgtt gaattgtaaa tttttttttt gtgttaattt 14220
atgttgtttt taaggtaggg gttatggttt ttttttttga ttattagggt tagttttgga 14280
gaagggtttt ttggaataaa agaaatatgg agaggggtaa atagtatttt taggatttag 14340
gatatttgtg ttttaggttt gtttttgata ttatcgatta taaattaata aatttgggtt 14400
ttagaatttt tattggtaaa gggagaggat tagatttaat gattttttag aatttattta 14460
gtgttaaagt tttgttattt tattataaat gattagtgtg aagaatagag ttttagtgtt 14520
atgggttttg ttgttagaag agtttggtta aattataaag tttggttatt agttatgaag 14580
tttggttaaa ttataaagtt tggttatatt taagaatgtt ttgaatgtag gagtatattt 14640
ttttatttta gaaaatttaa tatttagaat taataagtga atgaatgatt attgggtagt 14700
tttttatttt ttatggattt gaattagttt aattgtagta tttttagtga gggaaaattt 14760
tttttatttt aaataaaatg ttgtgtttag gtagagtggt ttatatttgt aattttagta 14820
ttttgggagg ttaatgtaga agtttttttt gagtttagga gtttgagatt agtttttata 14880
atatagtgat gttttatttt tataaaaaag atgaaagtta gttaggtttg gtggtaagta 14940
tttgtagttt tagttatttt agaggtagag gtgggaggat tgtttgagtt taggagtttt 15000
agaaaagttt gagtaatata atgagatatt atttttataa gaaaataaaa aattaggtgg 15060
gtttagtggt atgtagttgt agttttagtt atttgggagg ttaaggtagg agttttgttt 15120
ttttaggttt tgaaatttat ttattttgtt attttataaa tgatagaatt gagttgtttt 15180
tatgagttgg gttttgtatt tcgttattgg gaaaggattg aaaaggtaaa aatatttttt 15240
ataggtgtat agtatggtat agttttaaga cgtagttttt ttatttatat agcgtggttg 15300
agatgaattt atttattttt taattatatt aaggatttat tatgtttagg tattatttta 15360
gattttgggg atatgattat gaataatagt aataataaaa gtgtattttt gggagttatt 15420
ttgttgttta aagatttttt tagattaagt agtttgtgtg tttgttattt tttagttagt 15480
ttatatatat ttattttttt ataggttttt tattgcggcg atttattgag tgagtatttt 15540
tggtttttta tttgttaaag atataagagt gttaagttta aaagtgcgga gtgttaagat 15600
aaggaatgtt aagtttaaag gcgtagggaa ggcggtttat ttttttagta ataaatggaa 15660
ttagtgtatg gaattagaag ggtattttgt taggggaggt ttttgtttgt ttttttgagg 15720
aggtttttag gatagaatgc gaagagtagt ttagatttgt agtttgaaat ttcgaggagg 15780
ttgtttggta ggaggttggg tttgtgtggg aagtttaatt ttttggaaga ttattagtat 15840
tgaataggtt agaagataga ggaaggagag tggagtgaag agaaaagagt agagaaggga 15900
ggtaagaatt tgagtaatgg aatataattt tttaagtgat ggtattttaa aattagagtt 15960
tatattattt tggagaaggt ttgaggaaga tattatttgg attttatatt tttttttttt 16020
agtttaatat agttaatttt ttttttttgt tgaaaaggat gttttttttc gttgagtatt 16080
agatgaagta gtttttttag ttaagtgtta cgttgtagga gaaagattgt ttttagtata 16140
ggtttggatg tatataaagt atagaggacg tttattttat atagaggttt tttattggga 16200
tttgggagtt gtatgtattt ttatattttt tatcgtagtt agttgaggtg gtagaagttt 16260
acggagttga atgtgggtgt aattgtattg attgtttata ttattttttt ttttcgttgg 16320
ttagtagtag aattagattt gtttagtaaa tgtatacgcg ttgtaaattc gttataggat 16380
gagattggat tttttggatt aatgtttatt ggaaattttt atttaataat aatttagatt 16440
ttatattaga atattttaat taaatgtttt aaaaatgatt ataggaatta tttgttttta 16500
attgtttttt ttaaaattta tgggtattcg gggttgggag agattaagat gtagtaggga 16560
tttatggaga agaattcggt ggtttgtggt ttttagtttt aattttatta tttatgagtt 16620
gtagtatttg gggtaaatta ttttattttt ttgtgtatgg aggaaacgag tgagagaaat 16680
tattagattt tttaattata atttatgggt ttattgttgt agattattat tttttttgtt 16740
tgttaaaatg aaaaagtgtt cgtaaaagtg tttttttatt tttagttttg ggttttttcg 16800
ggattttttt ttttttttag tatttatttt tttttttttt ttggattaaa ttaattaata 16860
agtaaatatt tattgtaatt aatgatttat tttaaattaa ttttttttta attttaagtt 16920
ttttattttt gtattgtagt ttaattaata agtaaatatt tattgtaatt aatgatttat 16980
tttaattaat tttttttgat ttttaagttt tttatttttg tattgtagtt tagttttttt 17040
tatttttttt tttttaaatt ttttaaaata agtatttgta tttaaaggtt tattttattt 17100
tttttttttt ttattttgta gtgattatta agtttttttt gaatttggtt aaataagttt 17160

ttaaaatttg ttaggaaaaa taatagtttt tgttttttta tttttttatt tttttatttt 17220
tagaagttat tattttttgt ttttggagaa ttatttttat tttatttttg ttatttgtag 17280
attttatgtt tttgggtaag ttattttatt tttttttttt gtttttttat gtataagata 17340
tatataataa tatagggtta ttgtgataaa tatatgtgtt aatatatgta aagtgtttag 17400
aatagttttt ggtatataga aggtttttat taaagtgttt atttttattt ttattgttat 17460
tattattatt attattatta ttattattat tattattatt ttgttttatt ttttttggta 17520
ttttgtatat ttgatcgtag agaagtttag taaatattgg ttgaagaatt gagtaaatgt 17580
agatgttttt ttttgaattt attttatttt attcggtttt attgtttgga ttttgttagg 17640
agggaaaagg ggattttatt tgttttatga ttttaggtgg agggattgtt atttgggatt 17700
tttatttaag taaaagtaaa ttattttagt tatttgtaag tatgtttggt ttgtaatatt 17760
atgttgtgag taatgaaaat taaaaaaaaa aaaaattatg aaaggaaaat aattttaaat 17820
gagggtgaag ggtaggttat gtttaggatg tgtaataaat tttttatttt gaatataaat 17880
gaagagttgt gtaatgt 17897

<210> 235
<211> 5987
<212> DNA
<213> Artificial Sequence

<220>
<223> chemically treated genomic DNA (Homo sapiens)

<400> 235

tttttaattt gtattatttt ttttatttta ataatttttt ttaatatttt ttataaagta 60
gatttattgg ttatagaatg ttttaatttt tatttgtttg agaaaatttt attttttttt 120
tatttttgaa agataatttt gtagggtata gaattttagg ttgtaggttt ttttttttta 180
aagtgaaata ttttatttta tttttttttt ttttgtatgg tatttgagaa gaagttagat 240
gtaattttta ttattattat ttaaaagatt gtttttgttt tttttttttt tttttttttt 300
tttttttttt ttttgtatat tatatttttt atagttgttt tatatttttt agatattatg 360
ttttggtttt tttttgtgtt tttttttttg atttttagtt ttagaagttt ttatttatat 420
atttgtaatc gtagggattt ttttttttgt tatgtttagt ttattaataa gtttttatag 480
atattgttga tttttgtttt agtgtttttg atttttagta tttttttgat tattttttgg 540
aattgttatt tgtttattta tattattaat ttatttttgt gtgttgtttt attatagtaa 600
ttgtagttgt tttaatttta taggtattgt aattttaata tttttattat atttgatatt 660
gattttgatg tttgttttgt tttattaagt tatgtttttg ttttttagtg tgatttttaa 720
ttttttgttg aaagttaggt atgatgtatt gagtgaaaga aatttaatat attgtaatgt 780
gacgataaga gtttagggga agtgaagtat tttatagttt tatagtaggt ttcggttttt 840
tagtgagttt gtgtttatga acggtgattt ttaataagtg ttttttattt tatttttttt 900
ttgtttttaa gtgggataag attattgggg gggggttaga attgggtatt tttttttttt 960
aatgtagaag ttaaagagag ggttggagtt gggtattttt ttttttttgt atggaaagtt 1020
agaggtagtt aaatttggat attttttttt ttttaattta gttaggttgc gataaaaatt 1080
tcgatagttt aggttttaat attataaaat aatttttttt gagtataggt tttattaaga 1140
atattatatt ttgatggagt tgagggggag ttttttttga tatttattgc gagaatttcg 1200
tagagtttta ggaagtaaaa tttataaaag tgtgggagtt ttttagaatt ttttttttgt 1260
agatttattt gtattgaatt tttagaaatt tattaattat agtttaggtt tttttattta 1320
ggtattggtt tttatggagg tttttgtttg tgtatttttg ttttagtaag ttgttttttt 1380
tgtatggttt gttttttaaa ttttttaagt agggttatga tttgtcgttt tatttttttg 1440
atagttttga gagtgttgat tttttagttt gtttagattt ttatttgttt ttaggatgaa 1500
gtgataattt ttaagttttt ttttgatatg ttagattaga aattgaaagt tttaagtttt 1560
atattttgtg cgtgggtatg tttatatttt gtttgtttta gtgtttttat tttatatttt 1620
tttttttttt tttgtttttt tgtgagattt ttaggtttaa tataaagatt gtgtttaatt 1680
tatttaatta ttttggttta tttgagtatt ataatgaata attataaaaa aaaatgaagt 1740
aaaagaaaaa tttattaaag aattgagata tttgagaaaa agaaaggaga ttagtgtttt 1800
ataaaattta gaaatagatt ttttaagtgt ttttttattg atttatgtga aaggattttt 1860
tttaatttaa taaattatgt gttttcgttt atagttttaa aattttttgt gtagttaaga 1920
atgggtaaat aattaggttt tattaaagga ttaacgtaaa gattttttgt aagtaatatt 1980
tttgttattt aaggaagaga taaattttat ggtataattt tgttaaagta tattaagaat 2040
aattttgata taaagttttt tttttagtta atatgttatg aaagaaagaa gataaggggt 2100
gatttttatt ttttaagtga attattaaat ttattaaaga agaaacgagg gaaatagaaa 2160
gaggattttt gtttgagata atggatttgt atgtatgagt agtagaattt tgtttaaagt 2220
ataaggaagg gaaaaaaaag ttagtttatt tggaattttg gatattaaga gtttttattg 2280
tttatttttt tttaatttat atgaatggtt tattatttta attaataaat attttatttt 2340

tttttaatat atttatgaaa taaatttgaa atgaatagtg taatatgtga atgtttagaa 2400
tattataaaa ttaaatataa aatttgtttg gtaatttttt tagtatttta ggaaaaaagt 2460
tgataaaatt ttaagtagta gaagggggta gtaaaattta atagaaagtt ttggaagatt 2520
tttaagattt tttttatttt ttttttatg tagattattt tttaataaat tttagacgtt 2580
aatagaaatt ttgtataata gatttatata tttgtttaaa atagatatag aaatattgaa 2640
tatatgtaaa tatgagagtt ataagtttta tatgattaaa tttttttttt atggtatata 2700
atagttatag tattttttta tataaaatag gtttagtggt tttaatttag tttggtatat 2760
ttaatatatt tttatgatta gtattttaaa tgtatttatt cgttttattt tattgtttta 2820
gaattgttag ttatttaata aattatgtaa tttttttttt tatgtttaga tttgtatttt 2880
tttttaaaat tttgtttatt tttaaagttt tagatttttt ttgaattttt ttttttgatt 2940
ttttaagtat atggaatttt ttattttatt ttgttatata agtgatagaa tttttattat 3000
gggatagatg gagtttaatt tttttgagtt taaaataatt taaatataat tattttttat 3060
gttttgtttt ttttttattt ttgtatttaa attttttttt agataataga ataattaatg 3120
tttgataagg aagataatga tgatgattat tttaaaatga atttaggatt gtaatgtaaa 3180
attttagtat ttttttatag tatggatttt aatatggttt ttaatttaaa ttaatattag 3240
tagttttaat tataaatttt aaattttagt agatgtaatt tattttttta aaatgaaata 3300
gaagattgaa attattaaat tattaaaaag aaaatgattt acgtttttag ttgaaatttt 3360
atgtaagatt ttatgtaata aataggagtg ttataaatgg aatgatgaaa tatgattaga 3420
ggaggagaaa ggttttttag atgagatgga attttagtta ttcgtgtttt atgaagaatt 3480
agatgtgtat attaagtaaa atagttaaaa aaaaaatttt taagtgagtt ttttatttat 3540
ttttttttta taagattttt ataaattgag gtatttggtg tagttttatt ttaggtttta 3600
tgttgttatt tttttgtaat gttaaggatt taggatataa ttgaattttt tattttttat 3660
tttttttttg gtgtgtgtgt atatatatat gtatatatat atatatatat atatatatat 3720
atatttttta gtattttatt tttatatgtt tttttttgag tattatttat gatagatgtt 3780
aaataaaagt aaagatgaaa ttttaattgt taaaattttt tttttattta attaattttt 3840
tatttaatta tgttttaaaa cgagaataga aaattagttt taataagttt aggtaattga 3900
aaagtaaatg ttatgttgta ttttgattta tggttataat ttataatttt ggaaaagtgg 3960
atagaaaaga taaaagagtg aattttaaaa ttcgaattta ttttattagt attttttatg 4020
aagggttagt aattaaaata atttacgtat tagggagaga aatgttttaa ggtatacgtt 4080
ttggatattt agcgtttttg taaattttgg ttatcgtcgt tttttttgtt tattagaagg 4140
taggaaattt tatattggtg attcgtggag tttatattaa ttatttatag ggtaattgtt 4200
taggattagt attatgagga gaatttattt ttttcgtttt tttttttaag aaataaggag 4260
ggggtgaagg tacggagaat agtatttttt ttgttgaaag taatttagtt ataaagataa 4320
attatagtta tgtatattga aggtagttat tttattttat aaaataagag ttttttaaaa 4380
agttatgtat gtatgtgttg tatatagagt agatatatag tttattaagc gtcgttatta 4440
aaatataaaa tatgttagtt ttttttaatt ttattcgttt tagtttgttt cgacgtgatt 4500
ttttcgattt tttaaagacg tatagattag atacggcggc ggcggcggga gaggggattt 4560
tttgcgtttt cggattttag ggtcgtttag atttttggag aggaagttaa gtgttttttt 4620
gttttttttc ggtattttat ttaaggcgat tagtttagaa ttggttttcg gaagcgttcg 4680
ggtaaagatt gcgaagaaga aaagatattt ggcggaaatt tgtgcgtttg gggcggtgga 4740
attcggggag gagagggagg gattagatag gagagtgggg attatttttt ttgtttttaa 4800
attggggtag ttttttgggt tttcgatttt tttattttcg tgggtaaaaa attttgtttt 4860
tatcgggttt acgtaatttt ttttaaggga gaggagggaa aaatttgtgg ggggtacgaa 4920
aaggcggaaa gaaatagtta tttcgttata tgggtttggt ttttagtttt ataaaaagga 4980
aggttttttc ggttagcgat taattgttat acgatttgta gtgagcgtta ggagtacgtt 5040
taggaatttt ttagtagcgt tttttttagt tttatagtta gacgttttta gatagtaaag 5100
tttattttcg cgtcgcgttt tgttcgtcgt tgcgatgttc gttcgcgttt tgttgttgtg 5160
cgcggttttg gcgtttagtt atataggtga gtatttggcg tcgcgtatcg gggatttcgg 5220
ttttacgtat tcgggtagag ttttcgtttt gattttttgg gtttatttta gtatttcgat 5280
tttttttcga atagagaagt tacgtgattt gggaaagagt ttggatcgtt agagttcgaa 5340
agaatttcgt ggatatttta gtttttttat aagtattgat tattatgagt tagttattta 5400
atcgatttga gatattttta ttttttaaat agggatagat gatattaatt tgtaggttgt 5460
tattatgata agataggatt tgattaatat atgtgaattg tttatatttg gaattttttt 5520
attgagtgga agaagttgtt ttaaatattt tagttagttt ttttttgttt ttaggaaagt 5580
tcggattatg ttttaagata agtaaaatgt tttaaaagta agttgtttta ttttgaattt 5640
ttttttaaat gttgattagt gtattagatt tattttaatt tggaaagtga agtgttattt 5700
atttgaattt tttaaaaatg ttaattttaa tatttaaaga gttaattaag aaaagtttag 5760
taatatgatg tattaagttg aatatgttgt tatttttatt tagaatagaa aattggtatt 5820
tttacgtttt atttatttta aggtaggtta aaaaattgta tttttatgat tatttatata 5880
ttttttgaat ttattattgt aaagttgatt tatagttaaa taattaaatg tttaaattaa 5940
gattaagata ttagagaatg atttatttgt tgttttttaa ttgtagt 5987

<210> 236

<211> 5987
<212> DNA
<213> Artificial Sequence

<220>
<223> chemically treated genomic DNA (Homo sapiens)

<400> 236

gttgtaatta aaggatagta aataaattat tttttagtgt tttaatttta atttaaatat 60
ttaattgttt gattatgaat taattttata ataataaatt taagaaatat ataggtagtt 120
atggaaatat aattttttaa tttgttttag aatggataaa acgtagaaat attaattttt 180
tattttaaat aaggataata gtatatttaa tttggtatat tatgttatta agtttttttt 240
agttaatttt ttagatgtta aaattagtat ttttaagaag tttaaataag tagtatttta 300
ttttttaaat taaaatggat ttagtatatt aattaatatt tagggaaaaa tttaaagtaa 360
aatagtttat ttttaagata ttttgtttat tttaaaatat aattcgggtt tttttgggag 420
taggaaagaa ttgattagaa tatttaaaat aatttttttt atttaataaa aaggttttaa 480
atataaataa tttatatata ttgattagat tttgttttat tataatgata atttgtaaat 540
tagtattatt tatttttatt taggaggtga gagtgtttta gatcggttaa gtaattggtt 600
tataatgatt agtgtttgtg ggaaagttgg aatatttacg gagttttttc gaattttagc 660
ggtttaagtt tttttttaag ttacgtagtt tttttattcg gagagaagtc ggagtattgg 720
gatagattta ggaggttaga gcggaaattt tgttcgggtg cgtggaatcg gagttttcgg 780
tgcgcggcgt taggtattta tttgtatggt tgagcgttag gatcgcgtat agtagtaggg 840
cgcgggcgag tatcgtagcg gcgggtaggg cgcggcgcgg gggtaggttt tgttgtttga 900
gggcgtttgg ttgtggagtt gaaggaggcg ttgttgagga gtttttggac gtgtttttga 960
cgtttattgt aagtcgtatg ataattggtc gttaatcgag agaatttttt tttttataag 1020
attgaaaatt aagtttatgt gacgaaatga ttgttttttt tcgttttttc gtatttttta 1080
taaatttttt tttttttttt ttttaaaaaa attgcgtaag ttcggtgggg gtagggtttt 1140
ttatttacgg aaatgagaaa atcggaaatt taggaagttg ttttaatttg ggagtagagg 1200
gggtagtttt tatttttttg tttgattttt tttttttttt ttcgagtttt atcgttttag 1260
gcgtataggt tttcgttaga tgtttttttt ttttcgtagt ttttgttcga gcgttttcga 1320
gagttagttt tggattgatc gttttggatg ggatatcggg ggagggtaga aggatatttg 1380
gttttttttt taggaatttg agcggttttg aggttcgggg gcgtagggaa tttttttttt 1440
cgtcgtcgtc gtcgtgtttg gtttgtacgt ttttagaggg tcgaggaagt tacgtcggga 1500
tagattgggg cgagtaaggt taagaaaggt tgatatgttt tatgttttag tgacgacgtt 1560
taataggttg tatatttgtt ttatatgtag tatatatata tatagttttt taaaaaattt 1620
ttattttgtg gaatgaaata gttattttta gtgtatatag ttgtaattta tttttgtagt 1680
taagttgttt ttaatagaag aaatattgtt tttcgtattt ttattttttt tttgtttttt 1740
ggaaagagag gcgggaaagg taaatttttt ttataatatt ggttttaagt agttattttg 1800
taaatagtta atgtgagttt tacgggttat taatataaag tttttttgttt tttgatggat 1860
aaaggaagcg gcgatggtta gaatttgtag ggacgttaaa tgtttaaaac gtatgtttta 1920
aggtattttt ttttttgatg cgtggattat tttggttatt agttttttat aggagatatt 1980
ggtaaaataa attcgagttt taaagtttat ttttttgttt tttttgttta tttttttaag 2040
attatgagtt gtgattatgg attaaagtat aatatagtat ttatttttta gttgtttggg 2100
tttattgggg ttaatttttt attttcgttt tggaatatag ttggatgagg aattaattag 2160
atggaaggga gattttgata gttggaattt tatttttgtt tttgtttaat atttattatg 2220
ggtagtgttt agggaggagt atgtgagggt gagatattaa aaaatatata tatgtatatg 2280
tgtgtgtgta tatatatata tatatatata tatatattag aaaagaagtg gaaaatagaa 2340
aatttagtta tgttttaagt ttttagtatt ataggaaaat gatagtataa aatttgaaat 2400
aaaattatat taggtatttt aatttgtaga agttttataa gaaaaaaata aataagagat 2460
ttatttggag gttttttttt taattgtttt gtttagtgta tatatttgat tttttatgag 2520
atacggatga ttaaaatttt attttattta ggaagttttt tttttttttt agttatattt 2580
tattatttta tttatggtat ttttatttat tgtatggaat tttatatgaa attttaatta 2640
agagcgtgga ttattttttt tttgataatt taataatttt aattttttgt tttattttaa 2700
aggagtaggt tgtatttatt gaaatttgaa gtttatagtt agagttatta atgttagttt 2760
gggttagaag ttatgttaga atttatattg tgagagagta ttaaaatttt atattataat 2820
tttgaattta ttttgaagtg attattatta ttgttttttt tattagatat taattgtttt 2880
gttgtttgaa aagagatttg gatataaaag tgagggaaaa atagggtata aggaataatt 2940
atatttagat tattttaaat ttaaaggaat tgaattttat ttattttata gtggaaattt 3000
tgttatttat atagtaggat agagtgaaga gttttatgta tttggaaagt taaaaaaaaa 3060
agtttaagga gaatttggga ttttaaggat gggtagagtt ttagaaagaa gtgtaaattt 3120
gagtataagg aaaaaagtta tataatttat taaataattg ataattttga gataataaaa 3180
taaaacggat aggtatattt gggatgttgg ttatgggagt gtattaaatg tgttaaatta 3240

aattaagatt attaaatttg ttttatatgg aaaagtattg tgattattgt gtattataaa 3300
aaaaaggttt gattatgtaa aatttatagt ttttatgttt gtatatattt aatgtttttg 3360
tgtttatttt aggtaaatat atggatttgt tgtataaaat ttttattagc gtttgaaatt 3420
tgttgggaaa taatttatat gaaaagaaaa taaggaagaa ttttaaaaat tttttagagt 3480
tttttgttga gttttattgt ttttttttgt tgtttgaaat tttgttaatt ttttttttaa 3540
gatgttagga agattgttag atagattttg tgtttaattt tataatgttt taaatattta 3600
tatgttgtat tgtttatttt agatttgttt tatgaatatg ttgaaaagaa atgaaatatt 3660
tattaattga agtgataagt tatttatgtg agttaaaaga aaatgaataa taaagatttt 3720
taatgtttaa aattttaaat aaattaattt tttttttttt tttttgtatt ttgagtaggg 3780
ttttattatt tatatatata gatttattat tttaggtaag ggtttttttt ttattttttt 3840
cgtttttttt ttggtgggtt tagtggttta tttagagagt ggagattatt ttttgttttt 3900
ttttttttat ggtatgttgg ttgaaaaaag agttttgtgt tagggttatt tttagtatat 3960
tttggtaagg ttatgttatg aagtttattt tttttttgag tagtagaaat gttatttata 4020
gaagattttt acgttagttt tttagtaaag tttgattatt tatttatttt tagttatata 4080
agaagttttg aggttataaa cgaaagtata taatttgtta aattaagaaa agttttttta 4140
tataagttaa tgaagaaata tttaaaaaat ttatttttaa gttttataaa atattgattt 4200
tttttttttt ttttaaatat tttaattttt tgatggattt tttttttatt ttattttttt 4260
ttgtgattgt ttattataat atttagatga gttaaagtag ttgaatgagt tgaatatagt 4320
ttttgtattg gatttagaaa ttttataagg ggataaggaa gggaaagaga gtgtgaggtg 4380
ggggtattgg agtaggtagg atgtgaatat atttacgtat agaatatgag gtttaggatt 4440
tttagttttt gatttggtat gttagggagg agtttggaaa ttgttatttt attttaaaaa 4500
taagtaaaaa tttaagtaaa ttgaaaaatt aatattttta gaattgttag agaagtgagg 4560
cgataggtta taattttatt taaaaaattt gaaagataga ttatataaga agaataattt 4620
attggagtag aaatgtatag gtagaaattt ttatgaaaat tagtatttgg gtaggaaaat 4680
ttgaattgta attgatgaat ttttggaggt ttagtgtaga taagtttgta·aaggaaaaat 4740
tttggaagat ttttatattt ttgtgagttt tgttttttgg agttttacga ggttttcgta 4800
gtgaatatta gagaaaattt ttttttagtt ttattagagt atagtgtttt taataaggtt 4860
tatatttaag agaaattatt ttataatatt agagtttaaa ttgtcgggat ttttgtcgta 4920
gtttaattga attagaagaa ggaaaatatt taaatttaat tgtttttagt tttttatata 4980
ggggaagaaa aatatttaat tttagttttt tttttagttt ttatattgga gaagggaaat 5040
atttaatttt agtttttttt tagtgatttt gttttattta aggataggaa aagagtggaa 5100
tgaaaagtat ttgttgaaag ttatcgttta taggtatagg tttattaaaa ggtcgagatt 5160
tgttatagga ttatagaatg ttttattttt tttgaatttt tatcgttata ttataatgta 5220
ttgagttttt tttatttagt atattatgtt tggtttttaa taaaaaatta gaagttatat 5280
taaaagataa aaatatagtt tgataagata gagtaagtat tagaattaat gttaaatatg 5340
gtagagatgt tgaaattata atatttatga aattaaaata attgtaatta ttatgataag 5400
ataatatata agaataggtt ggtaatgtaa gtagatagat ggtaatttta agaaataatt 5460
agagaaatgt tagagattaa aaatattgga atagaagtta ataatgtttg taagggttta 5520
ttagtagatt ggatatggta gaggaaagaa tttttgcgat tgtagatata taaatagaga 5580
tttttaaaat tgagaattaa agaaaaaaat atagaagaaa attaaaatat aatatttaag 5640
aaatatgggg taattataaa aggtgtaata tatagagaag gaaagaaggg aaggagaaga 5700
gagaaaggaa tagaagtaat tttttaagta ataatgataa gaattatatt tgattttttt 5760
ttagatatta tataaagaag aaaagagtgg aatgaaatat tttattttga ggggaaaaaa 5820
tttataattt agaattttgt attttataaa attatttttt aaaagtgaag gagaaataag 5880
gtttttttag ataaatgaaa attgaggtat tttgtggtta gtagatttgt tttgtgagaa 5940
atgttaaaag aagttattga gataagaaaa atgatatagg ttagaaa 5987

<210> 237
<211> 5683
<212> DNA
<213> Artificial Sequence

<220>
<223> chemically treated genomic DNA (Homo sapiens)

<400> 237

atggtaaata tttgaaaaag tttttacgtt gaaatttaaa ttagattata tttaggatgt 60
tttataaggt ggtatttttt attaagatga taaaggataa atattggtgg agttagagag 120
gttgttttta aatatagttt tttttattag taatattgta gtagtttgta tttatcgttt 180
agagtatgaa aaagattttt gttaaagacg gtttttgggg atttattatt attatatgtt 240
aagagtttgt tttttatgtt tttatttttt tattttgtta atttttttta tttttatttt 300
tcggtttagt gtgagatttt attatttttt ttttttattt tttttttttt atatttttgg 360

ttttttagta ggttaatgtt tttgtttttt tattggaatt tatttatttt ggttattttt 420
ttttttatga atttgttgga ttatatttta aagttattta aaagagggat ataaagatat 480
aataaaatcg gaaataacgt ttatttttat tagtttgtag ttaagagtgt gtggaattag 540
ggtggttgta ggtaaagaga tagggggaag ggttgtttta tgttatgggt aggatagtaa 600
atggtgtaaa ttttttggaa agtattttag taatataggt gaagagtttt tataatgtgt 660
ttattttatg atttattttt atttttttta ttttaaagga ataagaagag gtttaattaa 720
agtatgagtg aaaaggttat ttaaatatag ggaaaggggt cgttatgatg tttacggttt 780
taagaaatag aaaataataa tttggtttaa ataataagta tataattaaa gaaatttaga 840
agtaggattt ttttaagagt tgagagattt agtagtttaa aaatgtgatt tgatatacga 900
gtttttttta tgtttttgtt tttttttttt taatattggt tttatattaa atttggtttt 960
ttagtggtta atagtattaa atagtattta tcggtatttt tgttggtatt taaaagagat 1020
ggagaaagtt tgattttata tggttttttt ttaaaagtaa taaaggattt ttttcggaag 1080
tttttagtaa atttcgttag atttgaatta tatgttaatt tttaaattag ttattggtag 1140
ggaagtggga aaaaggggag gaggtgttta tttttaatta atatgtttat ttttgtttat 1200
tgagattaat tttataaaag tattgtttta gttttttgaa gaggagtata atgtttgtga 1260
gttatttatt atatttattt attaagagtt aagggataat tgagtaaaat ggtagagttg 1320
gtaggaaaat ggtgagtagt ttcgtttttt tgtgttataa ttgatttgta tataaagaga 1380
tttggaaatt taaatataag aatttttttt ttttaagttt agggttttgg gtattttaag 1440
tttggttttt gtttgggtat agtggtttac gtttgtaatt ttagtaattt gggaagttaa 1500
ggagggtgga ttatttgagg tgatgagttt gagattagtt tggttaatat ggcgaaattt 1560
taattttatt aaaaaatata aaaattagtt ggatgtagtg gtatacgttt ggaattttag 1620
ttatttagga ggttgaggta ggaaaatcgt ttgaatttgg gaggtaaaag ttgtagtgag 1680
ttgagattgt attattgtat tttagtttgg gagatagagt aagattttat tttatttttt 1740
attaaaaaaa aattttggtt tttatatgtg aaaaaataaa agttaatttt taaaaattaa 1800
ataataaaga gtgttaggtg ataagtttta tgatagagat aatgtgtgtt ttaaaatata 1860
tagtataaaa tagatggaaa atattttatg gagaagggag agtttgaatt ggtgtttgat 1920
tgaaggataa gaaaaatttg ttggtaattt ttttttggtt ttgatttttg atgtagttaa 1980
agtaaagtaa tttagaatta tgatttaggg aatatattta gagagaaatg aatttatgat 2040
gtttttaatt tgaagatgtt atgattttag attattttag aatataatga ttttttattg 2100
ttttatattt gtaagaaatg ttttattatt gaaaatttgt aatatttaaa aaatttttat 2160
ataaaattat agtttatttt gttgttttat gttaaataaa taaaatattt ttttttaatt 2220
atgttgtttt ttttaattat acgtaatttt aaaattagtt gtaattgata gttttttttt 2280
tatatttatt gttaatgtat ttaggttaat tttattttta attagagaaa tttagaaaaa 2340
ggaaatttta cggtgaaatg ttatatgttt tataattttt agtaattaat ttttaaggat 2400
gataatttgg taaaaaagag aattggatta gttttttgaa aggtttatta tttcggttta 2460
tgttaattga ttgttttaaa attatagtgt ttttaggttg tagagatata tttaaaatgg 2520
attattgttt ttatattaaa ggggataggt tttttttttt ttttttttat ttttgttttt 2580
tttaaagttg tatagaggta tatgaggttt ggggaatatt tgtagtatat gttggttggt 2640
aagaatgtgg gtgaattttt gttaaggggc gtagatattt ttttttttat gatttttttt 2700
ttttatttag aataattgaa gtattatgat aagaggaagg gtttgtgttt tgagataagt 2760
agtttttata tttgttagtt ttttttaata agaaagtttt tttttgttat cgtataggtt 2820
atttttttat tggtaaagta tgatgaagtt tggaaattgt tgtttttgtt ttgttgaaag 2880
aagttaagtt ttaagaaatt tgtagaggta tttttataat tttttagtta tggtagtatg 2940
aattttgaag gggtagtgag tgttggtttt tattttgttt gtatagttaa gtttagggga 3000
gataattttt ttatataaga tgtattttgt ttttgtgggt tttttgtagt ttttaagttt 3060
ttatttgatt tgtatataat gatttagtgg gttagaggag tttagagtta tgagcggttt 3120
atttttttaa gaattatttt tgattgttta gtattatgga gtaagtggaa agaagaaaaa 3180
aaaaatttaa ttatttttcg aagagtaaga tgaatgttgt agaaggagaa ggaaggggag 3240
ggagatggat gggtgtcgat tttagaattt ttagatttgt ttggatgaat tattatttat 3300
gatttgcggg ataagaattt gattttattt attaattagt agaaattttt ttttttgttt 3360
tttaatattt gaaatttaat aaatatgtgt tttaggaata tatcggttat tttttagagg 3420
tattttatat atatattgag taattagaaa atattttttt cgtaatatat atatatatat 3480
atatatatat atattatttt gttatataat atttttacgt aagaaaagcg aaattgttgt 3540
ttgatgaatg taaatatttg gttgtttgta gtagtcggga gttttgttag gtttaagtgt 3600
taagatggga ggtgaatttt aggggttttt ttttgttcgt gttgagattt ttatttggtt 3660
aagtttttat ttatgttttg gtttcggagc gagttcgata gcgttggatt atagtagagg 3720
gagcgaggcg gttgacgttt tatttcgaag agatgaatgg aattttagga agttagagtt 3780
atgttggttt gggatagtgg tttggagatt agattttaat gatagaagta ttaggtagcg 3840
gtatttatgg taatgtgtgt atttatagaa atgtaattta tatttcgggt ttaggagtcg 3900
aaaaatgaaa agaacgttta gggaggaaaa agggaaatat aataataggt agagagtaat 3960
ttattatttt atgggtttgt tttgtaaata gttgaagatt ttggagttag atggttttgt 4020
aaatttttta aataggagtt acgttaagaa gtacgagtgg gtataaaaat tgttttttaa 4080
gatataattt taatttggtt tgtggaaatt ggatacgagt aagttttttt aaaattcgag 4140

tagaaagtag ttgttttttt cgggtttttt gatgagaata cgtatatcgt ttttaagcgg 4200
tcggtcgagg gagcgtcgcg gtagcgggag aggcgttttt gtgggttttt tggtagtcgc 4260
ggtaggaaag ggttcgaagg tagcgaaggc gaacgcggcg tattaatttg tcggtttcgt 4320
cgacgtcgcg tttatttttt ttcggggcgg gcgtggggtt agtttaggat aggcgttcgg 4380
gggacgcgtg tttttatttt acggggacgg tggaggagag ttagcgaggg ttcgaggggt 4440
aggtatttta acgaatggtt tttttggtgt tttttgcgtt tcgtcggttt attttttttt 4500
ttataaaacg ggtttagttt ttagtattta tttttcgtta ttaattaggt atttcgggag 4560
attagttcgt tcgaaagttt ttgcgttatt tcgcgggttt ttttaggtgg tttttttagt 4620
ttcgtttttt ttcgggatgt ttgttgatta tttcgagttc gcgtggcgta agagtacgag 4680
cgtcgagttc gtgcgcgtta aggttgcgtg ggcgggtatc gatttttttg agaagtttta 4740
gtgtttttaa gtttcgattt tcgttttttt tattttttag ttggaaagtt gcgcgttagg 4800
tagcgggggg cggagagagg agtttagatt ggtttttatt tttcgttttt tgttcggtcg 4860
tcgtttattg gtcggaggaa tttttaggaa tgcgagcgtt tttttaaaag cgcgcggttt 4920
tttcgttttg ttagtcgttg cgttcgagtt ggtttgcgag tttagggttt ttgtcgtttt 4980
ttaggagtaa tttttatttc ggacgtatag gtatttttcg cgttttttta gttttcgtcg 5040
ttttcgttat cgttttcggt cgtcgcgttt cggtatatat aggtaagtcg ttttcggcgg 5100
tcgtcgagga ttaaagttgt tcgggatatt tatttggagc gttgaggttt tagttttttt 5160
ggtggatttc ggaatttata tttttttcgt tcgtttattt tagttcgttt tttttagtcg 5220
ttggaggatt ttttagggta aggttttaga gttatttttt ttagttttga ggtttataaa 5280
ttaatttatt aggatatttt aagatttttt tattttttga agtttttttt aagtttttgt 5340
attagtattt tagggaagag tttgtatttt ttttgttttt tttgtaattt taaattatag 5400
tttttgattt tgtttatttt cgatttttta aaagttttta aattgttggt tttgcgtttt 5460
ttatatttta aaattagtat tttttttttt tatttttttt tttttttttt tttttttttt 5520
ttatgttttt tggaaagtaa ttttgttgtt ttttatttta gtaaacgcgg ggttaaagag 5580
ggatggtttc ggttttgttt ttatgtttag tttcgttttt gcgtatcgtt tttttttgtt 5640
tagttgattt ttgattttcg gtttttgtgt tttttgttat agg 5683

<210> 238
<211> 5683
<212> DNA
<213> Artificial Sequence

<220>
<223> chemically treated genomic DNA (Homo sapiens)

<400> 238

tttgtagtag gaagtataag agtcgagggt tagagattag ttaggtaggg agggacggtg 60
cgtagaaacg gggttgggta tggggatagg gtcgggatta tttttttttg atttcgcgtt 120
tgttgaaatg aaggataata ggattatttt ttagagagta tgagagagag agagagagag 180
agagagagag gtggaggaaa gagatgttgg ttttaaagtg tggggggcgt aagattaata 240
atttgggggt ttttgggaag tcgaaggtga gtaagattag gaattgtagt ttggggttgt 300
agggagggta ggggaagtat agattttttt ttggagtgtt gatataaagg tttaaggagg 360
attttagaga gtaaggaaat tttggggtgt tttgatgagt tggtttgtga attttaaggt 420
tggagaggat ggttttggag ttttgttttg aagagttttt tagcggttga gaggagcggg 480
ttggggtagg cgagcgggga gagtgtaggt ttcgggtttt attagaggga ttgaagtttt 540
agcgttttag gtggatgttt cggtagtttt tggttttcgg cggtcgtcgg gggcgattta 600
tttgtgtgta tcggagcgcg gcggtcggga gcggtggcga gggcggcgag ggttggaggg 660
gcgcggggaa tgtttgtgcg ttcggagtag aggttgtttt tggagagcga taggagtttt 720
gaattcgtag gttagttcgg gcgtagcggt tggtaaggcg gaggagtcgc gcgtttttaa 780
aggggcgttc gtatttttgg ggattttttc ggttaatggg cggcggtcgg gtaggaagcg 840
ggaggtgggg gttagtttgg gttttttttt tcgtttttcg ttgtttggcg cgtaattttt 900
tagttagaaa gtgaaggggg cgggggtcgg ggtttgggag tattagaatt ttttagaaaa 960
gtcggtgttc gtttacgtag ttttggcgcg tacgggttcg gcgttcgtat ttttgcgtta 1020
cgcgggttcg gggtgattag taagtatttc gaaaagggac ggggttgggg agattattta 1080
ggagggttcg cggggtggcg taggggtttt cgggcgagtt gatttttcgg aatgtttggt 1140
tgatggcgag aggtggatat tagagattgg gttcgttttg taaaaagaaa aatgggtcga 1200
cggggcgtag gggatattaa gagagttatt cgttaaagta tttgtttttc gggttttcgt 1260
tgattttttt ttatcgtttt cgtggggtga ggatacgcgt ttttcgagcg tttgttttga 1320
gttggtttta cgttcgtttc ggagggaggt gagcgcggcg tcggcggggt cggtaggttg 1380
gtgcgtcgcg ttcgttttcg ttgttttcgg gttttttttt gtcgcggttg ttagggggtt 1440
tatagagacg tttttttcgt tgtcgcggcg ttttttcggt cggtcgtttg ggggcggtgt 1500
gcgtattttt attaaggggt tcggggagga tagttgtttt ttattcgaat tttaaggaaa 1560

tttattcgta tttagttttt ataagttaaa ttgaaattgt gttttgaaaa atagtttttg 1620
tgtttattcg tgttttttaa cgtgattttt gtttggagaa tttgtagaat tatttggttt 1680
tagagttttt agttatttat agagtagatt tatagagtaa taaattattt tttgtttatt 1740
attgtatttt tttttttttt ttttaaacgt tttttttatt tttcggtttt tgaattcgag 1800
gtgtgggtta tatttttgtg ggtgtatata ttgttatgag tgtcgttgtt tagtgttttt 1860
gttattgaag tttggttttt aagttattgt tttaagttag tatgatttta gttttttgga 1920
attttattta ttttttcggg atgggacgtt agtcgtttcg ttttttttgt tgtgatttag 1980
cgttatcggg ttcgtttcga ggttagggta taggtagaag tttgattaaa taaggatttt 2040
agtacgggta gggggaaatt tttggggttt attttttatt ttagtattta aatttggtag 2100
gattttcgat tgttgtaaat agttaagtgt ttatatttat taaatagtag tttcgttttt 2160
tttgcgtggg agtgttgtat gataagatgg tgtgtgtgtg tgtgtgtgtg tgtgtgtatt 2220
acggaaagag tgttttttag ttatttaata tgtatataaa atgtttttaa aagatgatcg 2280
gtatgttttt aaggtatatg tttgttggat tttagatgtt gggaggtaga aagaaaagtt 2340
tttattggtt gatgaataaa attagatttt tgtttcgtaa attataggta atgatttatt 2400
taagtagatt tgaagatttt ggaatcggta tttatttatt tttttttttt tttttttttt 2460
tttatagtat ttattttgtt tttcgaaaag taattgggtt tttttttttt tttttttatt 2520
tgttttatga tattggatag ttagaaatag tttttggagg gatgggtcgt ttatggtttt 2580
gggttttttt ggtttattgg attattgtgt ataaattaga tgggggtttg agggttgtaa 2640
gaggtttata aaagtagaat gtattttgta tgaaaagatt atttttttta ggtttagttg 2700
tgtagataga gtagaaatta gtatttattg tttttttagg atttatgttg ttatggttga 2760
gggattgtag gaatgttttt gtagattttt tggaatttgg ttttttttaa taaaataagg 2820
ataatagttt ttaaatttta ttatgtttta ttaataagaa ggtgatttat acggtggtag 2880
gaaagagttt ttttattaga gaaagttgat aggtatagga gttgtttatt ttaaatataa 2940
agtttttttt tttattatga tattttagtt gttttgggtg gaaaggaaag gttatagaag 3000
agggagtgtt tgcgtttttt aataggaatt tatttatatt tttgttagtt aatatgtgtt 3060
gtaaatattt tttaaatttt atatattttt gtgtagtttt gagagaagta gggatgggaa 3120
aagggaaaag aaaatttatt ttttttaatg tgagggtaat agtttatttt gagtgtgttt 3180
ttataatttg gaagtattgt gattttaagg taattaatta atatgggtcg aagtgatgga 3240
ttttttaggg agttggttta gttttttttt ttgttaagtt gttatttttg aggattagtt 3300
attgaaaatt gtaaggtata tggtatttta tcgtaggatt tttttttttt aagttttttt 3360
aattggaggt gggattgatt taaatatatt ggtagtaagt gtgaggaaag ggttgttagt 3420
tatagttagt tttaaaattg cgtgtaatta aggagagtaa tataattgag aaaaaatatt 3480
ttgtttattt agtatagaat agtaaggtgg gttgtgattt tgtgtaggaa ttttttaagt 3540
gttgtaaatt tttagtagtg gaatattttt tataagtgtg aagtaatgaa aaattattgt 3600
attttagaat agtttaaaat tatagtattt ttaaattgaa gatattataa atttattttt 3660
ttttgaatat gttttttaga ttataatttt aagttgtttt attttaattg tattagaggt 3720
tagagttagg aggaagttat taatagattt tttttgtttt ttaattaaat attaatttaa 3780
gttttttttt ttttatgagg tgttttttat ttgttttatg ttgtgtgttt taaagtatat 3840
attattttta ttataaaatt tattatttaa tattttttat tatttagttt ttaaaaatta 3900
gtttttattt ttttatatgt aaagattaga attttttttt ggtggggggt gggatggagt 3960
tttgttttgt tttttaggtt ggagtgtagt ggtataattt tagtttattg taatttttgt 4020
tttttagatt taagcgattt ttttgtttta gttttttgag tagttgggat tttaggcgtg 4080
tgttattata tttaattaat ttttgtattt tttagtagag ttggggtttc gttatgttgg 4140
ttaggttggt tttaaattta ttattttagg tgatttattt tttttggttt tttaaattgt 4200
tgagattata ggcgtgagtt attgtgttta ggtaaggatt agatttgaag tgtttaaagt 4260
tttgggtttg ggaagagaga gtttttatgt ttaaattttt aaattttttt gtgtataagt 4320
taattatggt atagaaggac gggattattt attatttttt tattagtttt attattttat 4380
ttagttattt tttagttttt ggtgggtaaa tgtggtgagt gatttataaa tattgtattt 4440
ttttttaaag aattgaagta atatttttat ggaattgatt ttagtgggta ggggtaggta 4500
tattggttaa gggtaaatat tttttttttt tttttttatt tttttgttag tgattgattt 4560
aggaattggt atgtgattta aatttgacga ggtttgttga aggttttcgg gaaaagtttt 4620
ttgttgtttt taaggaagag ttatgtgaaa ttaggttttt tttatttttt ttggatatta 4680
ataagaatgt cgataagtgt tgtttggtgt tattggttat tggggagtta agtttagtgt 4740
gaaattagtg ttggaggagg aagaataaaa gtatagaaag aattcgtatg ttaggttata 4800
tttttgagtt gttaaatttt ttaattttta agggaatttt atttttagat ttttttggtt 4860
atgtgtttat tatttaagtt aagttgttgt tttttatttt ttagaatcgt agatattata 4920
acggtttttt tttttgtgtt tggatggttt ttttatttat gttttgatta aatttttttt 4980
tattttttta gaataagaga agtaaggata ggttatagga taaatatatt gtaaaggttt 5040
tttatttata ttattaagat gttttttaga aaatttgtat tatttattgt tttgtttatg 5100
gtatgaggta gttttttttt ttattttttt gtttgtagtt attttagttt tatatatttt 5160
taattgtagg ttggtaaggg tgagcgttat tttcgatttt attgtgtttt tgtatttttt 5220
ttttgggtaa ttttgagatg tagtttagta ggtttataaa gaaaagaata attaggatgg 5280
atggatttta gtgaaggggt aggggtattg atttgttagg aaattaaggg tatggaggaa 5340

agaagatggg aaaaggagat ggtgaggttt tatattgggt cgaaagatgg aaatggggaa 5400
gattagtaag gtaggaaggt gaggatatgg agaataggtt tttaatatgt aataatgata 5460
aatttttaaa agtcgttttt ggtaaaagtt ttttttatat tttggacgat aggtgtaggt 5520
tattgtagtg ttgttgatgg ggaggattgt atttgggagt agtttttttg gttttattaa 5580
tgtttatttt ttattatttt agtaaaaaat gttattttat ggggtatttt aaatatggtt 5640
tggtttgggt tttagcgtgg aagttttttt aagtgtttgt tat 5683

<210> 239
<211> 7833
<212> DNA
<213> Artificial Sequence

<220>
<223> chemically treated genomic DNA (Homo sapiens)

<400> 239

gtttttggtg agatatgtgt tttataagtt ttaatggaga aaaatgtaag tattttattt 60
tttgaaattt ggttatttga gtaatgagaa aatagttatt ttttttagga tagtggtttt 120
taattatggt tatgtgtttt tttaggaaaa ttttaaaaat atatatatat taatgttttt 180
gtgttatttt tagggatttt aagtttttga atacgaattt tgtattagta ttttttaatt 240
atttaggtga ttgtgatgtg aaattatgat tgagttttat tgttttaaga tgaaataaat 300
tttttttagt attgaaatta taaatttaaa ttattaaaat taattaaggg tatgggaatt 360
aataaggtat agggaagttt ttatattata aaattatttt tttaaattat agtttattgt 420
ttatatgtta tttgttattg tagaaaaggg tgaaaaaata gtaaatttaa ttatttttag 480
tttgaaaaat tatttagaaa tgaagatgac gattttgaaa tattgttaat attatttgat 540
ttataaataa tgttttaata tatttattat atattgatag atattttttt atatgaatat 600
tatatattaa aattaaggta ataatgtatt tagaatattt tatttatatt tatgtatttt 660
aagtaggtta gaaattaaga tatgagttat taagtatgag atgttaaggt gtggggttag 720
aaattatatt gtattttatt attaataatt aatatatatt ttaatattat atatatttaa 780
ttttaatttg tatattttta attattttta attatgtgta taaatataag tatatatatt 840
tttatgtatt tatttattta tatttttatt tatttattta tataggggat ttttttaaat 900
ttattattat taaattatat atttttattt taatttttag aataagttta ggaggtaggt 960
attgttatta tttatatttt ataaatgagg aaattgttta tagttataaa gttattgtgt 1020
tagatatatt agaagtttaa tatatatttg gtgaatatat gtataaaaat agagagatag 1080
atatgtataa tagtttattt ttatattgag taaaagtttt taatttgttt tagaaatttt 1140
tttgtgaaaa ttgagtaaaa atcgaggtat ttttttattt gttatatagg tataggtggt 1200
attttatttt tttaataagg atgaatattg aaatgtggat tttaaggttt aattttagat 1260
tttttgaatt tttgatagtg ggatttggaa tttgtttatt gttttaaagt tttttaagga 1320
atttatatga ttaattaggt ttagaaatta ttggatttta ttgtcgaagt ttgagaatta 1380
aagtttgggt tttattgcgg ttttatagaa agggtaaatg aagtattatg gatagaattg 1440
atacgttttt agttagtttt tttttttaga agttaatagg tagtaatata gtagaaatta 1500
gtgatttatg ttttgtgttt tgaagttagg tagaatttta tagagtttta gtagtgttat 1560
tgacgagatt tgttttttgg ggtaagttgt ttgatgtttt taaagttata ttttttttat 1620
ataaaatgag ataatatttt ttgttttata ggggtgtttt aaagattaaa taaaaataat 1680
atgttttatt ttatatggta taatgtttga tatttaagaa gtaaaggata tattttattt 1740
ttattgaagt aattagaaag tatgaaatta tgaaggagat aagagttttg attggtagtg 1800
tattttattt ttttaggttt atttatttat tttaaattat ttttgttgga gaataatttt 1860
taagtttttt atttaagttg tgagtaattt tatattttat aatgatgttt tttttatgag 1920
aaaaaaaaat gtttttaagt tttttggaga aaatatattt gtattatttt tattgaaaaa 1980
tttaataatt ggattttgtt tttttgtatt aattttagag tgtatatgtt ataaataaag 2040
tgttttagtt taagaagatt gaaagtaaat atggtatagt attttaaaat aagaattttg 2100
taaatatatg gtatgattgt gttatattat tagtaattat atgatacgta atgtaaagta 2160
tagtttatag atttaaattt aattttaata agtaaattga ttttgttttg ttggggaaaa 2220
gttaaagtat taatttaatt gttaatgtag ttttgtttat ttttttggta tttagtgata 2280
agtttaaata atgtatatat ttttatttat atatttagta atataatttt ttgtttaatg 2340
agtgatgttt ttttgttatt tggtggtgtt tgttagtttt agaatttgtt ttttggtggt 2400
attataatat taagtataga gtaagtgtaa taaaattgta gtatttttat tgaaaaggtt 2460
ttgttttaaa ttgtttaata atttaaagga tttttgtgga agtaatcgta tttgttaatt 2520
agttataatt agtaattaat ttttttggag ttttaattta tttttggtaa aacgttttag 2580
gaagagtata tattattaga aagtatgtta aaaatttatt tagtagaaaa tttaaaaata 2640
gttttttttt gttaagaggt tttttaaaat tttatttata tagttaaatt ttgaaatttt 2700
agtaggtttt gttttattat tataattatt gtataaatat ttttaaggat tttgttttta 2760

gttttaagta tgatttattt ttataagttt gattagttat tatattagtt ttgttatgga 2820
aaatgatatg tttttatttt ttgttgtaga gttgttaaat tttgatttat atttatgttg 2880
ttttttttgt tgaaagtttg tagcgaaaga aatttttaat tttttgtttt gtaatattag 2940
ttggtagttt tatttaatgg gtattttgtt tttttaaaga atttagttgt tttgtttaga 3000
agtcgatttt ttgatgtttt taacgtttgg tttaattgat ttgttttaat ggagttttcg 3060
tcggtgagga gcgagatgtt atcgattaga atgttgggat ttgttgttta attgttagga 3120
gtgagagata ttgagattta gaaatttttg gaggtgggag gggagaggga tagtttcgga 3180
cggaggcgga gatgtaagat aaagggatgg attttatata ggaaaaaaaa aaagatttcg 3240
ttgaggtatt gaggtgttgt acgattatat tttttaaagg agaagttaaa aagtaaggaa 3300
gtgggaggag gttggaggtt aaagtattta aaaggattat tcgggtataa tttgtttttt 3360
tgttggtgtt tgtaaaggat agatagtttc gtttttaaag tatatgaatg ttttttttaa 3420
gtgattggga atggatatta attgtttgtt aaatgttatt aaatgttttt ttaaatttag 3480
gggatataga aagaggggta taaaaggaga atttaaatag aaaaagggag gattcggagg 3540
tttttgaaag cggggggaga agaaggagga gggataatag agaggaatag agaaggagag 3600
cggagagaag ataaataaaa ataaaaatag gaattattga ataattatat attaaaaaga 3660
aagttttttt ttatggggta tttaaaatat tgagattgta atagtgattt cggttatgga 3720
agaaagatgt ttttttttat ttttgttttc gaaagttttt ggtttcgtta ttggcgatta 3780
aaattttatt aggttaaaga gtgtgtttaa ttgtttgaag aatgtagtag acggaaggcg 3840
ggtttcgtta tgtcgtttgt ttttttcgtt ggagagaatg aaagaaacgc gtagagttag 3900
agatttttgt cgagttagat tttttttcgt cgttttaggt tatcggttat tcggtaaaga 3960
ttcgagtaag gaacgtaggg ttattgtttg ggttaataaa tggagttcgt tttttttttt 4020
tcggacgtcg ttgttcggtc gatgttttcg gtaatttatt cgcggcgtat gtagaggagt 4080
tttttttttt tttttagatt atttgtttcg attaatttga ttttttaaat atatttgatc 4140
gtatttttta ggtggatata ttaataggtt acgggttgga gaggagcggg tgatgaggag 4200
agggatttaa atttgcgaac gtttgggttg ggtcggagtt gcggggggtt tgggaggaga 4260
gaggggagaa gagagaagga aggagagcgt ttgtcgggat ggttgagttg tttcggcgag 4320
tagttttggg gttgtacgtt tttgtgggag atgttgttgt tgtttttagg tcggtaagag 4380
cggttttaat attatcgttt tttatttttt tttttgtaaa tttttagaga aacgtttttg 4440
gttttttcgt cgcgatattt ttagtttgta tttttttata gtttaggcgg cgcgttttcg 4500
tacgttggag cgtcggtcgt tagtaggacg ttttttttcg cgtcgattcg tttttttttg 4560
ttttgttgtt gttgtttttt tgatattttc gtttttatta tttttagttc ggagagacgt 4620
tatttagtcg cggttcgtat tcgcggttcg gggttacgcg cggaagaggg gcgttagttc 4680
ggatttcgtt ttcggtaggg ggcgttttgg agcggagagt gaggcgaatg gtatatgagt 4740
gtgcgggtag tttattttga agttcgagtt ttttatttga gttatgtttc gtttagtttt 4800
attcgggtta gcgtttggcg agcgagttta tttgtggttt tcgcggtcgt tttttttttg 4860
tatttttgta tttattcgtc gatttttttt tttcgggatt tgtattttgt tttattaatt 4920
agagttcgat tgtttttttt tacgtgattt cgggcgggtt gaggatttgt tgttttttaa 4980
acgttagagg gatgcgggcg gtagagttcg agaggcggtt gtcgggttgc ggggcgtttt 5040
gatttttttt ttattttgtt ttttcgggtt ttattcgttt gtttttggat tttcgttttt 5100
ttttgttttt cggtttttta gagttttttt tttatggtag tagtttttcg cgttttcggc 5160
gtagtttttt agcggacgat tttttcgttt cggggttgag tttagttttt ggatgttgtt 5220
gaaattttcg agattatgcg cgggtttggt tgttgttttt tcgtcgggtg ttattgttat 5280
cgtcgtcgtt tttgttgtcg tcgttcgcgg gatgtttagt agttcgttgt tcggttttcg 5340
cgattttgtg tttttcggaa gtcgtttgtt gttgtagagt tgtacgaatt agttatggtg 5400
ttgtgggagt tttcgcggta gtgtagtagt tggatatttt gcgagggttt ttgttggttg 5460
ttgttgttgt tcgttatgtt atttatcgta gttcgttcgg tgaagttcgt tgtttttttt 5520
atttttttaa gtgattgtta aacgtttatc ggttggaatt gttttggtaa gtttagaatt 5580
ttcgttttcg attttttaat ttcgtagaag aatacgcgta tttagtatag attagtttat 5640
tttagcgcgt ttttttagtt ttttattttt tattgtttta gatttttaat attatttatt 5700
tttatttaga gaaataaggg gaattgttgt aggttcgggg gtgaggggtg gttttgggat 5760
gggtagaaag tgtaggtgta gtaggaaatt tttgtatgtt tgcgtttata ttggagttgc 5820
gaggattttg agaaatatta aacgggatgg ttttttgggt ttattgtttt gaaagagtat 5880
taattttagg ggaaatattg aaatagaagt tttgttatta ttaaagaaaa aagttttatt 5940
aggatgagga agaaataatt ttatgagaaa gaatgagcga gaaagtaata aattaaatgg 6000
tgattgtagg ggaatcgttg atttttggta aaggtgttat gaggtcgtat tggtttttcg 6060
ttgaagatta ggttatatag attttagagg agttgggttt taatagaatt tttttttttt 6120
tttttttttt tttttttttt tttttttttt tttttttatt tatttatttt tttttttttt 6180
ttattttttt tttttttagg cggtaaaaga tattggtttt gtagtttaga tatgtttttt 6240
tttttgtttt tttaagtttt aaggtagtat aggggagttg agaaaaagaa tattttgcgg 6300
gttttttagg tcggagtggg tatgattgag gttggttagg ttttatgtag gcgagtcgag 6360
ggcggaatcg attttagtgg gcgttgattt ttttattttt ggataggttt ttgtggagtg 6420
ggttaggtat tttttttgtt cgttcgggtt tttttagatt ttgacggcga acgtttggta 6480
ggtttcgttt tgttgaagtt ttttaattaa atagggttag aggatgggag ttgttgtatt 6540

tttagttggt atagtattcg gtttgatagt ttgtagtata gggtgtatgt aattttttat 6600
tttttgtgaa tataattttg ttgtagttaa atttggtttt gaataaagtg ttttttaaag 6660
atgtatataa gttgaagtgt atgtaatttt agagaggagg gaatgattaa ttgtaattta 6720
gggtgaaagt ttgtatagtt tttagttatt attgatgtaa atgttaaaag gaaaattatt 6780
atgtattatt ttaatttatt ttttataaag ataagttgag atatgtaatt ttattagatt 6840
tgggttaata gattgttttt ttttttggta gtttttaaat ttggtatttt aataaaattt 6900
aatatgtttt tataattttt tgatttatgc gtatatgtgt gttgtttttg aaagaataag 6960
ttttattttg ttattgttta attatttttt agatgtttta ttatggtaat aattatgagt 7020
ttgtaaaaat aatttttgga aatgttgatg gttttgtagt ttaatataga ttggtttgtt 7080
ttatttttag tttttgtatt gttttaggaa ataattaatt taaatgtgaa gttgatattt 7140
gtaattaaga aattatatat ttattagata ttttaaaggg gattgtataa attaaagaga 7200
ataaattggt tttgtagata ggttgttaag aatttggtat ttcgttttta tttttgttaa 7260
tttagaggtg attaattttt atttgagtta aatagattat tatagaaaat attgtgtttg 7320
tttattttta ttattgaggt tttgtttttt ttttgtttgg atatatttta aataaggggt 7380
tgttttagtc gttgaagtaa aagaataatt aaagatgggg aaatggtaaa agggtattta 7440
gagattatta ttagtttttt tttaaaatgt ggagttttgt ggttataaat attgtttatt 7500
taatgagtaa aaaataaaaa taaaaaaaaa ataggaagta aatgttaagt ttttatttat 7560
tattgttagt attaacgtaa gttttaaaaa atagtattat tagaaaagga tattaaagga 7620
gaattgatta gaaaagaatt gtggaaaatg gaaacgaata ttgattattt aattagattt 7680
tgaggttatt agtagatagt gattttgtag tatagttata gttgttggat ttaaaattta 7740
ggataagtat tttaaagttt taaagtagtg tttttttttg ttaaaaattt gtaagatgtt 7800
ttaatgattg gagtgttttt tttgaatttg agg 7833

<210> 240
<211> 7833
<212> DNA
<213> Artificial Sequence

<220>
<223> chemically treated genomic DNA (Homo sapiens)

<400> 240

ttttaaattt aaagagaata ttttagttat taaaatattt tatagatttt taataaaaaa 60
aagtattatt ttgaagtttt aaaatatttg ttttaaattt taaatttaat aattatagtt 120
gtattgtaag gttattgttt attgataatt ttaaaattta gttaagtgat taatattcgt 180
ttttattttt tataattttt ttttagttaa ttttttttta gtattttttt ttgatagtgt 240
tattttttaa agtttgcgtt aatattgata gtggtgaatg aaagtttaat atttgttttt 300
tgtttttttt ttatttttat tttttgttta ttaggtggat aatatttatg attataaaat 360
tttatatttt ggaaaagagt tagtgatgat ttttgaatat ttttttatta tttttttatt 420
tttaattgtt tttttgtttt aacgattgaa ataatttttt atttgaaatg tatttagata 480
aagaggaaat aaagttttaa taataaagat aaataggtat agtgtttttt gtgatggttt 540
gtttggttta aatgaagatt gattattttt aagttaatag gggtggaagc ggggtgttaa 600
gtttttgata atttatttgt aaaattagtt tatttttttt agtttatgta gtttttttta 660
aaatatttgg taaatatgta attttttgat tgtaaatgtt aattttatat ttaagttagt 720
tattttttaa aataatgtaa gggttaggaa tgaagtaaat tagtttgtgt tggattataa 780
agttattaat atttttaaaa attgtttttg taggtttata attattatta taataaagta 840
tttaaaaagt gattaggtaa tagtaaagtg aaatttattt ttttaaaaat aatatatatg 900
tacgtatgaa ttaagaagtt atagaaatat gttgagtttt attaaaatgt taaatttaga 960
aattgttaaa aaagagaata atttattgat ttaaatttaa tagggttgta tattttaatt 1020
tgtttttgta aaggataaat tagaatgatg tataataatt ttttttttgg tatttatatt 1080
agtaataatt aggaattata taggttttta ttttgagtta tagttggtta tttttttttt 1140
tttaaagtta tatatatttt agtttatata tatttttgaa agatatttta tttagagtta 1200
gatttaatta tagtaaaatt atatttatag aagatgaaaa attatatata ttttatatta 1260
taggttgtta aatcgaatgt tatgttagtt aggagtgtag taatttttat tttttggttt 1320
tatttaatta ggaagtttta gtagagcgaa gtttgttaag cgttcgtcgt tagaatttga 1380
aggaattcga gcgagtaaga agagtgtttg atttatttta tagaagtttg tttagaaatg 1440
gaggagttag cgtttattga agtcggtttc gttttcggtt cgtttatatg gagtttgatt 1500
agttttagtt atgtttattt cggtttggga gattcgtaaa gtgttttttt ttttaatttt 1560
tttgtattat tttgaagttt agggaagtaa agagaggggt atatttggat tgtaaaatta 1620
atgttttttg tcgtttagga gagaagggaa tgagagagag agagagatag atagatagag 1680
agagagagag agagagagag agagagagag agagagagag agaaatttta ttgaatttta 1740
gtttttttag aatttgtgtg atttggtttt taacgggaga ttagtgcgat tttatggtat 1800

ttttgttagg aattagcgat ttttttgtag ttattatttg atttattgtt ttttcgttta 1860
ttttttttta taaagttatt ttttttttat tttagtaaga tttttttttt taatgatgat 1920
aaagtttttg ttttagtgtt ttttttagga ttggtgtttt tttaaaatag tgaatttaga 1980
aaattatttc gtttaatatt ttttaaaatt ttcgtagttt taatgtaagc gtaagtatgt 2040
aaaggttttt tgttatattt gtattttttg tttattttag aattattttt tattttcggg 2100
tttgtaatag ttttttttgt ttttttggat agaggtgggt ggtattaggg gtttagggta 2160
gtaggaggtg aggggttgag gaggcgcgtt agggtaggtt ggtttgtgtt ggatacgcgt 2220
gtttttttgc ggagttaaag ggtcggggac gggggttttg gatttattag agtaatttta 2280
gtcggtgggc gtttggtagt tatttaagga ggtagggaaa gtagcgagtt ttatcgggcg 2340
ggttacgatg agtagtatga cgggtagtag tagtagttag taaaagtttt cgtaaagtgt 2400
ttagttgttg tattgtcgcg gggattttta tagtattatg attagttcgt gtaattttgt 2460
agtagtaaac ggttttcgag gaatatagga tcgcgggggt cgggtagcgg gttattgagt 2520
atttcgcgga cggcggtagt agaggcggcg gcggtggtag tggtattcgg cggggaagta 2580
gtagttaaat tcgcgtatga tttcgagagt tttagtaata tttagggatt gggtttagtt 2640
tcggagcgag agggtcgttc gttgagaagt tgcgtcggag acgcgggaag ttgttgttat 2700
aaggagggag ttttgggaag tcggaggata ggaggagacg ggagtttagg ggtagacgag 2760
tggagttcga ggaggtaggg tggagggaga gttaaggcgt ttcgtagttc ggtagtcgtt 2820
tttcgagttt tgtcgttcgt atttttttgg cgtttgggaa gtagtaggtt tttagttcgt 2880
tcggggttac gtgggaagag gtagtcgggt tttgattggt ggagtaggat gtaggtttcg 2940
ggagggaggg gtcgacgagt aggtgtaagg atgtaaggag gaggcggtcg cggaagttat 3000
agatgggttc gttcgttagg cgttggttcg agtggggtta ggcggggtat ggtttaaatg 3060
agaagttcgg gttttagggt gggttattcg tatatttata tattattcgt tttatttttc 3120
gttttaggac gttttttatc gaaggcgggg ttcggattag cgtttttttt tcgcgcgtga 3180
tttcgggtcg cgagtgcggg tcgcggttgg gtggcgtttt ttcgagttgg agatggtggg 3240
ggcggaggtg ttagaggagt agtagtagta gggtagagag gggcgagtcg gcgcgggaga 3300
gggcgttttg ttggcgatcg gcgttttagc gtgcgggagc gcgtcgttta ggttgtaggg 3360
ggatgtaggt tgggaatgtc gcggcggaga ggttagggac gtttttttag ggatttatag 3420
gaaagagggt gagaggcgat ggtgttagaa tcgtttttgt cgatttggaa gtaatagtag 3480
tattttttat aagagcgtgt aattttaagg ttgttcgtcg aggtagttta gttatttcgg 3540
taggcgtttt tttttttttt tttttttttt tttttttttt ttaggttttt cgtagtttcg 3600
atttagttta agcgttcgta ggtttgaatt ttttttttta ttattcgttt ttttttagtt 3660
cgtagtttat tagtgtgttt atttgggagg tgcggttaga tgtgtttgga aggttagatt 3720
ggtcgggata agtggtttga gagaaagaga aaggtttttt tgtatacgtc gcgggtgggt 3780
tgtcgggagt atcggtcggg tagcggcgtt cgggaagggg agagcgggtt ttatttgttg 3840
gtttaggtag tgattttgcg ttttttattc gggtttttgt cggatggtcg gtgatttggg 3900
gcgacgagag aaggtttaat tcggtaggag tttttggttt tgcgcgtttt ttttattttt 3960
tttagcggga agggtaaacg gtatagcggg attcgttttt cgtttgttgt attttttagg 4020
tagttagata tattttttag tttaatggaa ttttagtcgt tagtaacggg attaagagtt 4080
ttcggggata agggtggaga ggaatatttt ttttttatga tcggggttat tattgtagtt 4140
ttagtgtttt ggatgtttta tagggaagag tttttttttt ggtgtgtgat tatttagtga 4200
tttttgtttt tgtttttgtt tatttttttt tcgttttttt tttttatttt tttttgttat 4260
tttttttttt tttttttttt tcgtttttaa aagttttcgg attttttttt tttttattta 4320
aatttttttt ttgtgttttt ttttttgtgt tttttgaatt taggagagta tttgataata 4380
tttaataggt aattagtgtt tatttttaat tatttaaaag aggtatttat atattttgaa 4440
aacgggatta tttatttttt gtagatatta gtagaaaaat aaattgtatt cgagtaattt 4500
ttttaagtat tttaattttt aattttttt tattttttg tttttaatt tttttttga 4560
gagatgtgat cgtgtagtat tttagtgttt taacgaaatt tttttttttt ttttgtgtga 4620
aatttatttt tttattttat attttcgttt tcgttcgaga ttgttttttt ttttttttat 4680
ttttaaagat ttttgaattt tagtgttttt tatttttggt aattaagtag tagattttag 4740
tattttagtc ggtggtattt cgttttttat cgacgaagat tttattaaaa tagattaatt 4800
agattagacg ttggaggtat tagaaaatcg gtttttagat agagtagtta aattttttaa 4860
ggaaatagaa tatttattag atagagttgt taattaatat tgtaaaataa ggaattagaa 4920
atttttttcg ttataggttt ttagtagaga aggtaatata aatatagatt aagatttaat 4980
aattttatag tagagaatga gaatatgtta ttttttatag taaggttggt gtggtaatta 5040
attaggttta tgaaaataag ttatgtttga aattaaaggt aaagttttta aaagtgttta 5100
tgtagtaatt atgataatga aataggattt gttaggattt tagagtttgg ttatgtaagt 5160
agaattttag agaatttttt agtagaggaa aattgttttt gaattttttg ttaagtaaat 5220
ttttggtata ttttttaata atatatgttt tttttaagac gttttgttaa aagtaagtta 5280
aaattttaaa ggagttaatt attggttgta attggttaat aaatgcggtt gtttttatag 5340
aggtttttta aattattaaa tagtttgaag taaagttttt ttaatgggaa tgttgtaatt 5400
ttgttgtatt tattttgtat ttagtgttat agtgttatta agaaataaat tttgaaattg 5460
gtaagtatta ttaagtggta gaagaatatt atttattgag tagagaattg tattattgaa 5520
tatgtaaata aaaatatata tattatttag atttgttatt aggtattaaa gaagtagata 5580

agattgtatt agtaattgga ttagtgtttt aatttttttt tagtaaggta aaattagttt 5640
atttattaga attaaattta agtttatgaa ttgtattttg tattgcgtat tatatgattg 5700
ttagtaatat gatataatta tattatgtat ttgtaaaatt tttattttaa aatattatat 5760
tatatttatt tttaattttt ttgagttaga atattttatt tgtggtatat atattttaga 5820
attgatgtag aggagtagag tttagttgtt agatttttta gtagaaatag tgtagatata 5880
ttttttttag aaaatttaag aatatttttt ttttttatgg aaagaatatt attataaagt 5940
gtgagattat ttatagttta agtagggggt ttgggagtta ttttttaata agaatagttt 6000
aagataaata aatgaatttg ggaaaataag atatattgtt aattagaatt tttatttttt 6060
ttatgatttt atattttttg attgttttaa taaaggtaag atgtattttt tgttttttag 6120
gtgttaggta ttgtgttatg taggatagaa tatgttattt ttatttaatt tttaaaatat 6180
ttttatgaga taaagaatat tattttattt tatataaaag gaatatggtt ttgaaagtat 6240
taggtaattt gttttaagaa ataaatttcg ttagtgatat tgttgggatt ttgtgaaatt 6300
ttgtttgatt ttagagtata agatataagt tattaatttt tgttgtattg ttgtttgtta 6360
gtttttgaga ggggaaatta attgggaacg tattagtttt gtttatgata ttttatttgt 6420
tttttttgtg gagtcgtagt aaggtttaaa ttttaatttt taaatttcgg taataagatt 6480
tagtgatttt tgaatttggt tgattatatg aattttttga gaaattttga aataatagat 6540
aaattttaag ttttattatt agggatttag aaaatttgga gttgggtttt gggatttata 6600
ttttaatatt tatttttgtt ggagaagtaa ggtattattt atatttatat gataaatgaa 6660
aggatatttc gatttttgtt tagtttttat agagaggttt ttgagatagg ttaaaagttt 6720
ttatttagtg taaagatgag ttgttgtata tgtttgtttt tttgttttta tgtatatgtt 6780
tattaaatat gtattaagtt tttaatatgt ttgatatagt aattttgtga ttgtagataa 6840
tttttttatt tgtaaaatgt gagtaataat aatatttgtt ttttgggttt gttttaaaga 6900
ttaaaataaa aatgtatggt ttaatggtag tggatttggg ggattttttt atataaataa 6960
gtgaatggag gtatgaataa ataaatatat aaagatgtgt gtatttatat ttatatatat 7020
aattaaaaat agttaaagat gtataaatta aagttaaatg tatgtgatat tgaagtatat 7080
gttgattatt gataatgaag tatagtataa tttttaattt tatattttaa tattttatat 7140
ttaataattt atattttaat ttttagttta tttaagatat atagatatag atagaatgtt 7200
ttaaatgtat tattgtttta gttttaatgt ataatattta tatgaaaaag tatttattag 7260
tgtgtagtaa atgtattaga atattattta taggttaaat gatattgata atgttttaga 7320
gtcgttattt ttatttttgg ataatttttt aaattgagag taattaaatt tgttattttt 7380
ttattttttt ttataatggt aaataatata taaataatga gttgtgattt aaagaaataa 7440
ttttataatg taaaagtttt tttatgtttt attgattttt atgtttttaa ttaattttgg 7500
tagtttaagt ttgtgatttt agtgttgagg aaagtttatt ttattttaga gtagtggggt 7560
ttagttatga ttttatatta taattatttg gataattaaa gaatattgat gtagagttcg 7620
tatttaaaga tttggaattt ttagaagtga tatagaagta ttggtatata tatattttta 7680
aagttttttt ggagaaatat atagttatga ttgagaatta ttgttttggg gaaagtgatt 7740
atttttttat tatttaaata gttaagtttt aggaggtaaa atatttatat ttttttttat 7800
taaaatttgt aaaatatata ttttattaaa gat 7833

<210> 241
<211> 2501
<212> DNA
<213> Artificial Sequence

<220>
<223> chemically treated genomic DNA (Homo sapiens)

<400> 241

gtaaattttt atattttgag gagagatagg gttttattat gttggttagg ttggttttaa 60
atttttgatt ttaggtgatt tattcgtttt ggttttttaa agtgttggga ttataggcgt 120
gagttattaa gtttgacggg tagtaattta ttttttatat aatttttttt tttttttttt 180
ggtagagacg aggttttgtt atgttgttta ggttggttta aaattatcgg gtttaagtga 240
tttgtttatt cgggtttttt aaagtgtagg gattataagt atgaattatt atgtttggtt 300
gtaatttttt atatatttcg gatattaatt ttttattaga taatatagtt tgtaaatttt 360
tttttttttt atttgtaggt tgtttttttt attgttggtt atttttttttg ttgtatagtt 420
atttttaagt ttgttgtaaa tttatttgtt tattttttgt tttgttgttt gtgttttgtt 480
gttatattta agaaattatt gttaaattta ttgttatgaa gttttttttt atgttttttt 540
ttaggagtat tatagtttta ggttttatgt ttaattttta ataagttttg tgtttttgta 600
tatggtgtaa ggtaagggtt taattttatt tttttgtatg tggttatata gtttttttag 660
tattatttgt taaagatata attttttttt tatgttttgg tgttttaaaa aaaaaaaaaa 720
ttttggttgg ttacggtggt ttaggtttat aattttagta ttttgggagg ttgaggtaag 780
tggattgttt gaggttagga gttttagatt agtttggtta atatggtgaa attttgtttt 840

tattatcgaa gatataaaaa ttagttaggc gtggtggagt acgtttgtaa ttttagttta 900
ttagggaggt tgaggtatga gaatcgtttg aatttgggag gtagaggttg tagtgagtta 960
agattttatt attgtacgtt agtcggggtg atagagtgag gtagggtttt attttgtcgt 1020
ttaggtagga gtttagtggt ttaattatgg tttattgtag tttatattgt tagggtttaa 1080
gttatttttt tattttagtt ttttaagtag ttaggattat aggtgtgtgt tattatgttt 1140
agtaaatttt tgtatttttt gtagagatgg ggtattttta tgttgtttag gttggttttg 1200
aatttttaat tttaagcgat ttttttattt gggtttttta aagtattggg attataggcg 1260
tgagttattg cgtttgatat ggtgtttttt aatttatttt tattttttat ttttattttt 1320
ttgagataag gttttgtttt gttttttagg ttggaatgta gtggtataat tatggtttat 1380
tgtaattttt gttttttcgg tttaagtgat ttttttgttt taatttttgg agtagtttgg 1440
attatgggta tatgttataa cgattagtta atttttgttt tttttttttt tttttttttt 1500
tttttttttt tttttttttt ttgagatgta gtttttttat gttatttagg ttggtttaaa 1560
atttttgggt ttaagcgatt tttttatttt ggttttttaa agtgttggga tgataggcgt 1620
gagttacgtg gtgtttaaaa aaggtaataa aaaatttttt atatattggg tatagaagtg 1680
gtatgggttt ttatatattg tgagattttt ggtattagtt ataaattttg tgtatattta 1740
agatttttta gagtaggtgt aattatttcg ttttatagat gaggatatag aggttgagtc 1800
gtagtgattt atttaaggtc gtatagttag taaatagatg gaggttggat tggaattgag 1860
gattttattt aagggttttt ataaattttt gggggttttt cgttgtttta tttttattat 1920
atttgaaaga atgaatgaat gaatgtttcg ggtatcgtgt ttatttttta gtaaatcgtg 1980
gagtttggac gagtttattg tttcgcgtgg ggggggtgtg tgttcgtttt gcgtatgcgt 2040
gttttttggg tatggtcggt ttcgttttat ttttttgtat agggtatcgt tttttttcgt 2100
ttggtatatt tttttttttt ttacgttcgg attggggtgg tagacgtcgt tttcgtttat 2160
cgtttttttt tatcggtttt cgcgcgaaaa gtcggggcgt ttgcgttgtc gtcgtcgcgt 2220
ttgttgaagt tttcgagatg tcggcgcgta tcgttttagt tcgggtgttt atattggtcg 2280
tttcggttat ttcgttgttc gacgatgttc gtaggcggta ggtattatgg gggggaatac 2340
ggatttaggg ggataggtag ggcgttgggt ggggggtcgt tttttttcgg ggtggtcggt 2400
ggtcgttgtt gatagacggg cgcgtatgcg tggggtggtg cggcgcgtag cggtagttgg 2460
cgcgggtagg gtggtatttt cggtcgcgcg tgttcgggtt g 2501

<210> 242
<211> 2501
<212> DNA
<213> Artificial Sequence

<220>
<223> chemically treated genomic DNA (Homo sapiens)

<400> 242
   tagttcgggt acgcgcgatc ggaagtgtta ttttgttcgc gttaattgtc gttgcgcgtc 60
   gtattatttt acgtatgcgc gttcgtttgt tagtagcggt tatcggttat ttcgagggga 120
   agcgattttt tatttagcgt tttgtttgtt tttttgagtt cgtgtttttt tttatggtat 180
   ttatcgtttg cggatatcgt cgggtagcga gatggtcggg acggttagtg tgggtattcg 240
   ggttggggcg gtacgcgtcg gtatttcgga ggttttagta gacgcggcgg cggtagcgta 300
   ggcgtttcgg tttttcgcgc ggaaatcgat ggggaggggc gatgagcgga ggcggcgttt 360
   attattttag ttcgggcgtg ggggaggagg ggatgtatta aacggagaga ggcgatattt 420
   tgtgtagaag gatggaacgg agtcggttat gtttagggaa tacgtatgcg taaggcgggt 480
   atatattttt tttacgcgga gtagtgggtt cgtttaagtt ttacggtttg ttgggaggtg 540
   ggtacggtgt tcgaggtatt tatttattta tttttttagg tgtgatgggg ataaagtagc 600
   gagaagtttt taagggtttg tgagagtttt tgagtaaagt ttttagtttt aatttaattt 660
   ttatttattt gttggttata cgatttaggg tgggttatta cggtttagtt tttgtgtttt 720
   tatttgtaaa acggggtaat tgtatttatt ttagaaaatt ttgagtatat atagaatttg 780
   tagttagtat taagaatttt atagtgtata gaggtttatg ttatttttat atttagtgtg 840
   tgggggtttt tttgttgttt tttttaagta ttacgtggtt tacgtttgtt attttagtat 900
   tttgggaggt tagggtggga ggatcgtttg agtttaggag ttttagatta gtttaggtaa 960
   tatagagaaa ttgtatttta aaaaaaaaaa aaaagaaaga aagaaagaaa gaaaaaaaga 1020
   aaaataaaaa ttagttagtc gttgtggtat gtgtttatag tttaaattat tttagaggtt 1080
   gaggtaggaa gattatttga atcggagagg tagaggttgt agtgagttat gattgtatta 1140
   ttatatttta gtttgggaga tagagtaaga ttttgtttta aaaaaataaa aataaaaagt 1200
   aagaataaat taagaagtat tatgttaggc gtagtggttt acgtttgtaa ttttagtgtt 1260
   ttgagaggtt taggtgggag gatcgtttga ggttaggagt ttaagattag tttgagtaat 1320
   atagggatat tttattttta taaaaaatat aaagatttgt tgggtatggt gatatatatt 1380
   tgtaatttta gttatttggg aggttgaggt gggaggatgg tttgaatttt ggtagtgtag 1440
   gttgtaatga gttatgattg ggttattgga tttttgtttg ggcgataggg taagattttg 1500
   ttttattttg ttatttcggt tggcgtgtag tggtgagatt ttggtttatt gtaatttttg 1560
   ttttttaggt ttaagcgatt tttatgtttt agttttttta gtaggttggg attataggcg 1620
   tattttatta cgtttggtta atttttgtat tttcggtggt agagataggg ttttattatg 1680
   ttggttaggt tagtttggga tttttagttt taagtagttt atttgtttta gttttttaaa 1740
   gtgttgggat tataggttta agttatcgta attagttaga attttttttt tttttaaagt 1800
   attagaatat gggggaaaga ttgtgttttt aataaatggt gttgagaaaa ttgtataatt 1860
   atatataaaa gaatgaagtt ggatttttat tttatattat atataaaaat ataaaattta 1920
   ttaaagatta aatataagat ttgaaattat agtgttttta gaagaaaata tggggaaaag 1980
   ttttatgata atggatttgg taatgatttt ttggatatga tagtaaaata taggtaataa 2040
   gataaaagat agataaatgg gtttgtaata aatttaagga tagttatata gtaaaagaaa 2100
   tgattaatag tgaaaaggta atttatagaa tggggaaaaa aaaagtttgt aaattgtatt 2160
   atttggtaaa gggttaatat tcggaatata tgaaaaatta tagttaggta tggtggttta 2220
   tgtttgtgat ttttgtattt tgggaggttc gggtaggtag attatttgag ttcggtagtt 2280
   ttagattagt ttgagtaata tggtaaaatt tcgtttttat taaaaaaaga agaaaaaaat 2340
   tatataaaaa ataaattatt attcgttagg tttggtggtt tacgtttgta attttagtat 2400
   tttgagaggt taaggcgggt ggattatttg aggttaagag tttgagatta gtttggttaa 2460
   tatggtgaaa ttttgttttt ttttaaaata taaaaatttg t 2501

<210> 243
<211> 4138
<212> DNA
<213> Artificial Sequence

<220>
<223> chemically treated genomic DNA (Homo sapiens)

<400> 243

gaatttcgga ttcggattta tgttttcgtt taaattttat gttaaattgt aaattttaat 60
gttggaggtg gggttttgtg agaagtgatt ggataatgcg ggtggatttt ttgttttgat 120
gttgtttttg tgatagagat tttatatgat ttggttgttt aaaagtgtgt agtatttttt 180
tttttttttt tttttttttt tgtttatgtt ttgttatgta agacgttttt cgtttttttt 240
ttatcgttta gaatgattgt aagttttttg aggttttttt aggagtagaa gttattatgt 300
tttttgtata attgtagaat gatgagcgaa ttaaattttt tttttttata aattatttag 360
ttttaggtat ttttttatag taatgcgagg atagattaat ataatttttt atttttagat 420
tttcgtatac gtttagtttt agatattatt gtttttggga gtatgtatag cgtagttttt 480
tgtcgataaa agtaaagtta taaaggtgat aaaaatttgt atttggggat atttgattgt 540
gaaagaggat ggatagtata tttgtagtta tagagattgg ggtttatcga gttgaaattt 600
ggtagtattt tggtataata tgtgtatgat tcgtgtttaa tgtttagaga ttagtgttga 660
gtaaaatagt ttggtttggg gtcgttgttg tttttatttt ttttttgttt attagagggc 720
ggtagagttt ttttttttta ttttggagtt tttttagggg ttgttgattt ttttagtcgg 780
gtttatagtt tagtagggtt tatttttatt cgggttattt cggtttacgt ttttttcgtt 840
tttcgagttt tttatacgga ttttgttagt ttttttttgt agtttatcgg tcgtttattt 900
gaggtttgtc ggtcgtttat ttgaggtttg tcggttgttt tttgtaggta gtttttgttt 960
tttatatttt tttttttttc gggtttagtt gaaagggcgt tttttagggt agttttttgt 1020
gatttttagt atagtttagt tttttatagg tttcgacgtt ttttatgttg ttattttata 1080
gttttgttat tattattaat tttttagttt tatgaagttt attgagcgtt tgtttttcgg 1140
ttataggaaa attttgtgat agggattacg tttgtaatgt tttttgtgga attttaaggt 1200
ttagtttagt gtttgatacg gaatagatgt tttataaata ttggttaaat gtgtgggaga 1260
tttttaaaaa tagtatatta ttttcgtgtg gtttttagta gttagagttt gttttatgtg 1320
gatatagggg tattggtatt agtatgggag gaggttagta agtgttcgcg gttgttttag 1380
gaatgaggtt ttaattttta gagttttaga agggaggata gaggtttgta gggaatagat 1440
ttttcggttt gattttgtag tttaatttag agtttagggt tagtttatat tacgtcgatt 1500
ttggttagta tttttagggt agttttagat aaggtcggag gttttttttt ttgtttttta 1560
gggggtgata ttgtatatag atattattta ggaaacggat ttttttggat aggaatttgg 1620
ttttgttaag gaagtggagg tggagtttgg tttttatttt ttgttttaat agattttttt 1680
gatttttttt atatatttgt tttgtttttt tttgggtttt atgaggattt tgttttgtta 1740
ggggtttttg tgtaatttta gatttttttt tggtattatt atggggaagg tggggtgatt 1800
ataggatagt tagtttcgta gagatagaga ttatttagga tagttaggga gaatatggat 1860
aggttttgag tcgtagtttt agttaataga tacggagagg gagggttttt ttggagtttt 1920
ttttaaggat agtagagttt agagttattt attttttttt attatagttt ttttttttta 1980
ggatatataa gatatttttt tttttatatg taggatttgg ggatttttga gatttttggg 2040

tttgggtttt tatttttggg ttagtggcgg ggttggtggt attggagata gagggttggt 2100
ttttttttag ttattattta gtgagttttt ttttagtttt tagagttatt tttgttattt 2160
ttttgttggg tattatttta tttttttaga gttttggaga gtatggggag attcgggatt 2220
ttgttgggtt tttttgttat aaaggaaaat aatttttttg gtgtgataga tttaaggata 2280
gaatatagta gaggttagta ttggggaaga taggttgttt ttttagggga tgggggtgga 2340
tttattttgt cgaaaagatt tgtttgagga attgaaaata gaagggaaaa aagaggaggg 2400
ataaaagagg tagaaatgag aggggagggg atagaggata tttgaataaa gattatattt 2460
tgatttacgt gatgttgaga agtatttttg ttttaggaag agatttaggg tagagggagg 2520
aaggatagta gattagatag ttatagtagt tttgataaaa cgtttttgga atttaagttt 2580
ttttttatag aggaggatag agtagatagt agagattatg gagttttttt cggttttttt 2640
ttatagatgg tgtatttttt ggtagaggtt tttgtttata ggtgaaggga ggataatttg 2700
ggagagggtg ggaggaggga gtatagagat tggttggggt tttttgggta ggatagggtt 2760
gtgagacgga tagaggtttt tgttggagtt tgaataggga agaggatatt agagagggat 2820
aggagttata ttagaaaaat taaattgaat tggaattgga aaggggtagg aaaattttaa 2880
gagttttatt tttttagtta attgttattg gttattacgt ttttaaaaat tataataatt 2940
gtattatatg atattttaaa taaaaatata ataattgtat tagatgatat tttaaataaa 3000
aatataatta gggtatgaaa tattgttttt attcgtttat cgcggatatt ggaaaataag 3060
ttttaggttg tggagggttt tgggaatttt tatgaattta tttataggaa tttgtagttt 3120
gttttaggta ttggggtgta attaagatta tataaatttt tgtttttatg aagtttatgt 3180
ttttatgggg aggaagatag atatataaag agatttagaa tgtgaggtta ggtgttgata 3240
agagttttgg aggaatagag tagggaaagg ttagaaaagg aagatttagg gtttttagag 3300
gaggtgttag ggaagggatt ttttaagaat gttttgatgt gagtaggatt tgaaggtaat 3360
ggggagggag tcgtgaagat ttttggaaaa gtagatttta tatagggaaa tgttaaggtc 3420
ggaggtgtta aggaaatagg agatatattg ttgattttga tttagtagga tatatatata 3480
tatatatata tatttattta ttttagggtt gggggatgaa gagatttgtt taggatttag 3540
gattttattt ttttatttta atgtataggt tttaatattg atcgatgttt tttgtttttt 3600
tttagtttta tttttaattt tttggaattc gtttattatt gttaagttta ttattgaatt 3660
tacgtcgttt aatgtcgtag aggggaagga ggtgttttta tttgtttata atttgtttta 3720
gtattttttt ggttatagtt ggtataaagg tgaaagagtg gatggtaatc gttaaattat 3780
aggatatgta ataggaattt aataagttat tttagggttc gtatatagtg gtcgagagat 3840
aatatatttt aatgtatttt tgttgattta gaatattatt tagaatgata taggatttta 3900
tattttatac gttataaagt tagattttgt gaatgaagaa gtaattggtt agtttcgggt 3960
atatcgtgag tgattttttt atgatttttg ggtgttgggg gttagtttta ttttttatat 4020
ataggattat taggtttggg ttgtgttgtg gttttttttg tattacgtat tatgttaggg 4080
tttgggtatt tagtgtagga tatatataga agagataaat tttaatagat tagaattt 4138

<210> 244
<211> 4138
<212> DNA
<213> Artificial Sequence

<220>
<223> chemically treated genomic DNA (Homo sapiens)

<400> 244

gaattttgat ttgttgaagt ttgttttttt tgtgtgtatt ttgtattaaa tgtttaaatt 60
ttaatatggt gcgtaatgta gagggggtta tagtatagtt taggtttgat aattttgtgt 120
gtgggaagta gaattgattt ttaatattta gaggttatgg gggaattatt tacggtatat 180
tcggaattgg ttagttgttt ttttatttat aagatttgat tttatgacgt gtagggtgta 240
gaattttgtg ttattttgga tgatgttttg gattagtagg gatgtattgg ggtatattat 300
ttttcgatta ttgtatgcgg gttttggggt agtttgttga gtttttatta tatattttat 360
aatttgacgg ttgttattta tttttttatt tttgtattag ttgtagttaa aaagatgttg 420
gggtagattg tggataagta gaagtatttt tttttttttt gcgatattga acggcgtgga 480
tttaatagtg agtttggtag tggtgggcgg gttttagaag gttagaagtg aggttaggag 540
agagtagaga gtatcggtta atattgggat ttatgtatta gggtggaaaa atggggtttt 600
gggttttgag taggtttttt tattttttag ttttggagtg agtgagtgtg tgtgtgtgtg 660
tgtgtgtgtt ttattaggtt aaggttagta gtgtgttttt tattttttta gtattttcga 720
ttttggtatt tttttgtgtg gaatttgttt ttttaggggt ttttacggtt ttttttttat 780
tgtttttagg ttttgtttat attagggtat ttttgggaga tttttttttt gatatttttt 840
ttagagattt tgggtttttt ttttttgatt tttttttgtt ttattttttt agggtttttg 900
ttaatatttg attttatatt ttagattttt ttgtatgttt gttttttttt ttatgagagt 960
atgagtttta tgagggtagg gatttgtgtg attttggttg tattttagtg tttgggatag 1020

gttgtagatt tttgtggatg agtttatgag ggtttttagg gttttttata gtttggggtt 1080
tattttttaa tgttcgcggt aggcggatga ggatagtgtt ttatgttttg gttatgtttt 1140
tatttaaagt gttatttgat gtagttatta tgtttttatt taaagtgtta tgtgatgtag 1200
ttattatgat ttttaaaaac gtagtggtta gtgataatta attaggaaaa tagaattttt 1260
gaggtttttt tgtttttttt taattttagt ttaatttgat ttttttggtg tgatttttgt 1320
ttttttttga tgtttttttt tttatttagg ttttaatagg agtttttgtt cgttttatag 1380
ttttgtttta tttaggagat tttagttagt ttttgtgttt ttttttttta ttttttttta 1440
ggttgttttt tttttatttg tgagtaggag tttttgttag gggatgtatt atttgtgggg 1500
aggggtcgag ggagatttta tggtttttgt tgtttgtttt gttttttttt gtggagaaga 1560
gtttgagttt taggaacgtt ttgttaaggt tgttgtgatt gtttggtttg ttgttttttt 1620
tttttttgtt ttgagttttt ttttagggta ggagtatttt ttagtattac gtgggttagg 1680
gtgtggtttt tatttaggtg ttttttgttt tttttttttt tatttttgtt ttttttgttt 1740
tttttttttt ttttttttta tttttagttt tttagataaa ttttttcggt aaggtggatt 1800
tatttttatt ttttgggagg ataatttgtt ttttttagtg ttgatttttg ttgtgttttg 1860
tttttgggtt tgttatatta gggggattat ttttttttgt gatagagaaa tttagtaggg 1920
tttcgggttt ttttatgttt tttagggttt tgggaaggtg ggatgatgtt taataggaag 1980
gtgatagagg tggttttggg ggttagaaaa aggtttattg ggtggtggtt ggggagggat 2040
tagttttttg tttttagtat tattaatttc gttattgatt tagggatgga gatttaggtt 2100
tagaggtttt aggagttttt agattttgta tgtagagggg gaggtgtttt gtgtgttttg 2160
gaaagagagg attgtggtgg agggaggtgg gtgattttgg gttttgttgt ttttggggaa 2220
ggttttaggg ggattttttt ttttcgtgtt tgttggttga agttgcggtt tagggtttgt 2280
ttatgttttt tttgattgtt ttgggtggtt tttgtttttg cgaggttgat tgttttgtga 2340
ttattttatt ttttttatgg tggtattagg agggagtttg gagttgtata gggatttttg 2400
gtagaatagg gtttttatag gatttagaaa ggaatagagt aggtatgtgg gagagattag 2460
aagggtttgt tggagtaagg gatggaaatt aggttttatt tttatttttt tagtaaagtt 2520
aggtttttgt ttaggggaat tcgttttttg agtgatgttt gtgtgtaatg ttattttttg 2580
gagggtaaag aaggagattt tcggttttgt ttggaattgt tttagggatg ttgattaggg 2640
tcgacgtggt gtgagttgat tttgaatttt ggattaggtt gtagggttag gtcggaggat 2700
ttattttttg taggtttttg tttttttttt tgaagttttg ggggttgagg ttttattttt 2760
ggggtagtcg cgggtatttg ttggtttttt tttatgttgg tgttagtgtt tttgtgttta 2820
tatggaatag attttgattg ttgggggtta tacggaggtg atatgttgtt tttagagatt 2880
ttttatatat ttaattagta tttatggagt atttgtttcg tgttaggtat tgggttgggt 2940
tttgggattt tatagagagt attatagacg tggtttttgt tatagagttt ttttgtaatc 3000
gggagatagg cgtttagtga attttatggg attgaggagt taatggtaat gatagggttg 3060
tgaggtgata gtataggggg cgtcggagtt tgtgagagat tgagttgtat tggagattat 3120
agggagttgt tttgggagac gtttttttag ttgagttcgg ggaaggaggg ggtgtagggg 3180
ataggagttg tttgtagagg gtagtcgata ggttttaagt gggcggtcga taagttttag 3240
gtgggcggtc gataggttgt agggaggagt tgatagagtt cgtgtgagga gttcggaggg 3300
cgaggaggac gtgggtcgag gtgattcggg tgagggtgga ttttgttagg ttgtgggttc 3360
ggttgaggag gttagtagtt tttggggagg ttttagggtg ggagggagga attttgtcgt 3420
tttttggtgg ataggaggga agtggggata gtagcggttt tagattaggt tgttttattt 3480
aatattgatt tttagatatt gaatacgggt tatgtatatg ttatgttaaa gtgttattag 3540
gttttagttc ggtgagtttt agtttttgtg gttataagtg tattgtttat ttttttttat 3600
aattagatgt ttttaaatgt agatttttgt tatttttgtg attttgtttt tgtcggtagg 3660
aggttgcgtt gtgtatgttt ttaggggtag tgatgtttgg ggttaagcgt gtgcggggat 3720
ttgggagtag aagattgtat tagtttgttt tcgtattgtt ataaagaaat atttgagatt 3780
gggtaattta taaagaaaag aggtttaatt cgtttattat tttgtagttg tataggaagt 3840
atagtggttt ttgtttttgg ggaggtttta gaaaatttat aattattttg gacggtgaag 3900
gggaaacgag gaacgtttta tatggtagag tatgagtaag agagagagag agagagggga 3960
gaggtgttat atatttttaa ataattagat tatgtgagat ttttattata gaaatagtat 4020
taaagtagaa aatttattcg tattatttaa ttatttttta taaggtttta tttttaatat 4080
tggggtttat aatttgatat gagatttggg cggggatata gattcggatt cggaattt 4138

<210> 245
<211> 11222
<212> DNA
<213> Artificial Sequence

<220>
<223> chemically treated genomic DNA (Homo sapiens)

<400> 245
   ggtttggcgt tggtttagga gggtagattt tgttagggag gtataagtta agttgggggt 60
   ggggtgggag ggtttgttgg taggagggag gtattgttta gattggtttt taaaggttgg 120
   tttttagaag gaggaggaag agagtagtga agatcgtggg gagttttggt tttgaggagt 180
   tagagttttt tttttgttta tatttttgat tttttttggt ggtgttttaa gtttagattt 240
   attaaaggga attgttagtt gtttaagggt tagtaaattt ttaggttatt tagattggat 300
   tttttgatta taaaaattgt gggttaggtg tatagttgtt tgaggggttt aaatttgtgt 360
   aggtgggtgt tggaattttt tttgttatag cgtttttttt gtgtgtatgt gattagaggt 420
   gagagtggtt ggagatcgaa gatggtagag ttcgtttttt tttattagat tgaggttttc 480
   gatttgtttt gtttggggag ggtgtttagt ggaagttttg ggatgtttag tttggataag 540
   aaaagttttg ggtgagtttg tttttttttt tgtgaggtag atttagttgg gtttttatat 600
   ttttaaggag taggcggagt tgtcgtaagg aagtgtacgt gatatggagg tattttttgg 660
   ttcgatataa aaaaggattt ttaaagtttt aggtagtgag tttattattt ttaggagtat 720
   ttaagtagat gttagatgat gatttgtagg gaaaattatg tttagtggga aattggataa 780
   aatatcgttg aaggtttttt ttagttataa gatattatgg ttttataatt ttagattttt 840
   ttgtttttag ttttttataa gtttggaatt tagaatttaa gttttttcgg tgttgaaaag 900
   ttgaagattt tgatatttta gaattttaat tttgggattt taagatttta gaattgggag 960
   ttttgaggat gattttataa gttttagttt tggatttaaa ggattttgtg atatgaaagg 1020
   gaaaaagagg aggtttgatt tggtgggatt tagttttgtt ttattgggta ttagattaat 1080
   gtgttttgta ttcgtgcgtg tgtgtgcgtg tgtgtagtat gagcgtgtaa ggttgtagaa 1140
   agttttgtat ataaaggtag tttggttttt ttattatgtg tttgtagttt ttttgttttg 1200
   gttataatga aggttgttat agggataata atagttttta aatagagttg gtgattttag 1260
   gtagttatag taattgtgag gtaacgttgt tttgttgtag agatgtgtga gtataaatag 1320
   atgtcggtgt ttttttatta gtttaaatgg taaaatttaa aaggtttatt aattttaaag 1380
   ttataggttt ttatgagtat gttagttaat gggggcgttt gttaagcggg tgatagtttt 1440
   atattaaata ttttagtatt agtaatgggt tgatgagatt ttaaatgttt aggttatttt 1500
   tagataattt aggaggttag agacgggaga agagttggga ttcgggtagt aattagtttt 1560
   gttttgtttc gtatttttag gggtgttttg atattttcgg gagggtatag gttagagagt 1620
   atttgaaaat cgtatattgc gttttaattt ttaggagttt tagatttgtt ttttggtttt 1680
   ttttagcgta ttaggtaggg ggttttaggt tagatgtggt atagtttaat gttatttttt 1740
   tttttttgtt tttttttttt atattttgga tttattttta gcgagtgatt agtagtaagg 1800
   gttaaattgt tgagagtttg agatcggggt agattttttt agagttgttt ttttttttga 1860
   ggttgggttt ggtatagatt ggggttttag taatgggttt ttaaatgaat gaatgaataa 1920
   aggaataaat agatgattga ttgattgaat gaatgaatga gatttttttt ggtattttta 1980
   tggttttttt tttttttttt gagatggagg ttcgttttgt tgttaggttg gagtatagcg 2040
   atttcggttt attgtaattt ttgtttttcg ggtttaagtg atttttttgt tttagttttt 2100
   taagtagttg ggattatagg ttcgtgttat tatgtttagt taatttttgt atttttagta 2160
   gagatagggt ttgaatatgt tagttaggat agtttcgatt ttttgatttt gtgatttatt 2220
   tattttagtt ttttaaattg ttgggattat aggcgtgagt tattacgttc ggttttttat 2280
   ggtattttag ttatttattt tgttattgtt ttttttattg ttaaagagat gggtttttgg 2340
   aggtcggttg gtttagtatt tggggttgat ttttggaatg gttattaggg aaggggcgtt 2400
   agaggtgtag tcggtgtttt tgattttttt ggtcgttagg agttagtcga tttagtatat 2460
   aggatttatt gaatggagat ataaggtttt tttagggagt ggcggtttat ggtaatttta 2520
   gaatggttat tagttaggtt ttagagattt atatagaggg cgttttgatt taaagttgta 2580
   ttggggaatt ttaagtttgg ggattttttg aatttaattt tttttttagt tatatttttt 2640
   attatttgtt taatttttat taaatatttt tgtttatatt ttgaagttgg gatttgattg 2700
   gtagagttag tagatgttga ttatttagat ggagttttga tattggtttt tttagtttgg 2760
   ttgtgattgg tagtaggttt gcgggagaat tgtgtgtttt agaatatgat tggttatatt 2820
   tgtattttag taagattggg gtagggtagt tatttttaaa aagttgtgta ggtggggtag 2880
   ttattattga taaatttagt gtagatttag gatggtttaa atattggttt attttttttg 2940
   aattttattt tttatagttg taaagcgggg tggtgttcgt tattttatag aggtgttgta 3000
   aagattagat gtaattttgt taagtagtta ttttgtaaat tgtatagttt tatgtagatg 3060
   gaaggaaggg tttgtgttta ttttgattat agtattaaat aaattgtatt gtatttttat 3120
   tttttttagt tattttttta aaaagatttt gagttttttg aatataggaa ggtgttttat 3180
   ttgattttgt tatttttagt atgtagtagt gtttgatata tagtaggtgt tttattattg 3240
   tgagagggat ggatggatgg gtggagttat agatggatag aaggatagat ggagggatgg 3300
   gtggatgatg gatggataga tggatggagg ggggatgatg aatggaggga taatgagtgg 3360
   atgaatgagg gaatgggtgg atggatggat ggagggatgg aggaatagat agatagatgg 3420
   agggatgggt gggtgatgga tggatagatg gatggaggga gggatgatga atggagggat 3480
   aatgaatgga tgaatgaggg gatgggtgga tggatgaatg gagggatgat gggtggatga 3540
   atgaattgag ggatggatgg atgaatatat ggatggatgg atagatggat agatggagga 3600
   attggtggat tttggatgga tgggtggatg gatagatgaa tgaatgtttg gatagataaa 3660
   gagatgatgg atagatgaat agatgaatta agggatgtcg gatagatgga gggattgata 3720
   gatgttggat ggatgggtgg tggatggata gatgagtgaa tgtatggata gataaagaga 3780
   tgatggatgg atgaattaag ggatgataga tggatggatg gatgagtaat tggatggata 3840
   agtggataaa tggatagatg gttgaatatt tgaatggatt gaaggaggat gtatggatgt 3900
   aagataaggt taattatttt ttattttttt tttttgtaaa attatttatt tatttattta 3960
   ataaatattt atttagttta aatttggtat aaagtattat gtgaggttta agagatacgt 4020
   gggttaataa aatagagttt ttgttttttt gaaaattgta aagaaagggg cgtggttttt 4080
   tgagtttaaa ttttaatttt gttagcgatt agttgtatat tagtgatgtt tttttatttt 4140
   tttttaatta aatagggata atgttagtat ttattatatt gggaggtttt gcggggatta 4200
   aatgagttat taaatgttaa gtgtttggga tagggtttgg tatttagtaa agttttttgt 4260
   gagtgttggt tgttattatt ttaatggaga agatggtatg aaaattagga aataggatgt 4320
   tttttgggaa gtaatgtaat aggaatttat ataaagaaag gaaaggagga agtaattagt 4380
   ggtgttttaa aggagtatgt taagaaaaat tttttagagg gaaatttttg agtagggtta 4440
   tgaaaatagg agttttttaa gagattgtgg atttgtttgg gattatttgg ttataagtat 4500
   aaaattattc ggtttttttt tgttattttt ggttgggtga ggggtttttg gtaaaggggt 4560
   agaaggtgcg tgagaggttg cgaatggtta ggattgtttt ggggttagtc ggggtatttg 4620
   gtggttaagt ttagaaatat gataggtttt tttgggaggg ttgatcgtag ggagcgttgg 4680
   gttttaggtt gttggcgtcg gtttttgtgg tgtttttttt gtcggttatg agagtttaga 4740
   tagtgtttaa tttttttttt ttttttttat acgtataatt attttatttt ttgtggtttg 4800
   agttgttttg tttcgttata atggtatttg ttttaaaata gttttttatg tgagggttag 4860
   agaaaggaaa agattagatt ttttttggat gagagagaga aagtgaagga gggtagggga 4920
   gggggatagc gagttattga gcgatttttg ttaagtattt tagaaggtat aaaaacgttt 4980
   ttgggattag gtagttttaa attttagttg ttggggttag gatatttagt gagtttatat 5040
   ttgttttttt tgtttttttt agattgcgtt atggggttta gcgacgggga atggtagttg 5100
   gtgttgaacg tttgggggaa ggtggaggtt gatattttag gttatgggta ggaagttttt 5160
   attaggtaaa aggaagagat tttattgttt ttgttattta tattttaaga ttaagggtgt 5220
   ttagttgtaa ggtggaaagt ttgtacgtgg ggtaggttag ttggttgtat tagttaaggg 5280
   tgttagaacg gttatttgtt ttttttttgt ttttaagtgt tagggattgg atttaggaga 5340
   gggaaaggag ttattttagg ttgatgttag tagttggagg aagtatgaga attaaattta 5400
   ggatgtttag agtttattag gaagaatttt agaattatag atagttagag ttaataaggg 5460
   ttttgagaga ttttgtatag ttattttttt tataggatga ggataaaaag ggattgagaa 5520
   ggggaggata tttttagagt tatagtttat taaatgtttt taaagtgtta aggttaagat 5580
   atgtttttta aggggagata gatttggttt tagatttggt tttgttattg agttattggg 5640
   tgatttttgg gaaagtattt aatttttcgg agttttaatt tttttttttg tatagtgagg 5700
   ggatatttta atatttatat tttagaggag atgtgagaat taaataaaat aatgtatgta 5760
   agaggtttgg tatggttttt ggtatatatt gagttttaga aatgttagta gttattattg 5820
   atgaagttta ggttagggat tttttaaagt attgtaatta gagaatagaa gatagaggtt 5880
   tattagtgat tttcgatgtt gagtatgttt ttagtttgag aggtttgaat gatgtggttt 5940
   gtaagtatat tttgtttttt attataaggg attttagaat atattaaaga aaataaaatt 6000
   ttgaggtttg taaatagagg gtggttgtgg tttgtatata gaagtttatt ttttcgttgt 6060
   tttttatttt aaaggtgata tatttttttt ttggtttttt tttttattat tttgagttgg 6120
   tttttttaga agtttaatag gttaagaatt aacgtttttg ttaacgggag gaaggaagtg 6180
   ggcgtcgggg ttgttataag tgaatggatt gtttttggta ttttaatttg tagattaatt 6240
   tttttttatt tgtgagttag agttaatttt ttttttttat ttcgtcgttt tttgggtttt 6300
   ttttgggttt tatttatttt tagttttcgt ttatttcggg agtatggttg gttttggaag 6360
   tttcgttatt taaaatttga gtcgttggtg attcgtgtta gaaatatttg aggaagaaga 6420
   gttgagtttt ttgttgattg taatggtatc gtagtgtgtt tatgtttttt tttttttgtt 6480
   tttgttattt ggttgtaata ggttttgttg gattgtataa agtgtttttt cgattaatat 6540
   atatatataa attgatatta atttttgggt tatagatatg ttatgtgatt ttggataagt 6600
   ttttttattt ttgggtttta tttttttgtt ggttgagcga agggattgtt ttttatggtt 6660
   ttcgagattt cgttttacgt ttatgtttta gaattttttg agtttttgat gtatttttgt 6720
   ttttggcgag gaggtaggat agttaggcgt ggaggtagga gattagagtt tgtagagtag 6780
   aggttttttt aaggtgattt gggagttgtt agatttaagg tagtgatttg tacgttttga 6840
   aagagattta gggtgttgta ttattaggga ggattacgga ggtttgtcgg gggtcgggag 6900
   ttagggataa gatataatgt ttagggttgt tggtttttga aaggttttag aggtttggtg 6960
   ttttgttacg ttttaggttg tgggatatgt agagattgtg ttagttggtt ttgttttttt 7020
   agtatttggg gtatagggag gatggttttt ttgggggtag ggtggaggag gtttatgttt 7080
   aggattgtaa atgttattcg tgagagttgg tattttgatt ttgttttaga tattgatttt 7140
   ggatttttta attgattttg aaaggttatg tttttaattt tttgattgta aaagagggta 7200
   aattttatga agtttagttt gtaaaatatt gaggttttta ttgtacgtat ataaatataa 7260
   aattttagaa tatagaagtt ggaaggttat ttggagattt tggttttagg agtggggttt 7320
   cggaagggag ggggttgttg agtgggtttt atatttttgt tttttttttt tttatttaaa 7380
   ttttttttgt tttatttatt ttggttgttt tggggagcgg ggggaataaa ggattttaag 7440
   gttataaaat gtttgaaaat tattagtggt ggttattatt ttttattata gggaggaatt 7500
   gggtttgaga gtttttatcg gttaatggta gttataggta ggattaaaaa tttttgggtg 7560
   atggtgatga taatgataat gataatgata atggtagaga tgatgaggag gagaaagata 7620
   gtaattattt atttagtata tattgattgt tgggttttgt ttaagtgttt tatttattga 7680
   agtttgataa taattttatg aggtagatat gattatgatt attatattta atagagaggt 7740
   taagtaattt acgttaagtt atatagtaag taagaaagga gttggaattt aaatttaggt 7800
   agtttggatt tagaattttt gttttttttt tttttttttg agatggagtt ttgttgggat 7860
   tataggtgta tgttattatg tttggttaat tttgtatttt ttagtagaga ttaggtttcg 7920
   ttatgttggt taggttggtt tcgaattttt gattttaggt gatttgttcg ttttggtttt 7980
   ttaaagtgtt gggattataa gttaatgagt tcggtttaga atttttgttt ttgattatta 8040
   tgttatattg ttttatgatt ttgttatacg taagacgggt tgttatttac gttttggttt 8100
   ttttatttta gtagagttag aggggtgttt atggatttga taatgtgttt tatgttttat 8160
   gtgagtgttt aattgggttt tagtttagag aatagtagga aaatggtttt tttttttttt 8220
   gatttggatt gaagatttgt ttgatgaatt aggtttattg atagtagggt tgaagttgga 8280
   aatataggtt agcggggttt tttgaaatat tagagtaaac gtttttcgtt tatttttata 8340
   ggttgagggg tttttatttg tttatttttc ggattaggta aatttaggag aatggttttt 8400
   tgtggttgtt tggggggttt ttagttgtaa ggtttgggtt tataggaggt aggtagttgt 8460
   gtggtgtcgg aatgattttg cggggagata aattagttcg attttgtttt gttgtttaaa 8520
   gtaaatatgg ggtggggtgg ggtggtttag gtttataatt ttagtatttt gggaggtagg 8580
   atcggggaat cgtttgagtt taggagttcg agattagttt gagttatata gtgagatttt 8640
   ttttttttat aaaaaataaa taaaattagt tgggcgtggt ggtgtgtgtt tgtagtttta 8700
   gttacgcggg aggttgaggt aggaggatta tttgggttta ggagattgag gttttagtga 8760
   gttaagatta tattattgta ttttagtttg ggtaatagag taatatcgtg ttttaaaaaa 8820
   atattaataa agtaaatatg agtttttttt ttggttgttt ggtgttgagt ggaaagaaag 8880
   gtattggcgg gtttagattt ttttggttta tatatggatg agtaatgatt tagagaggtt 8940
   aagtgatttt aatattatag agtatattgg cgttagaatt gggatcggaa tttagttttc 9000
   gtaattgtta gttttatgtt ttttttttta gatattagag ggtaggggta ggttttaaaa 9060
   tgtttgagat ttttagttgt tttgtggttt aaatttttgg ttttgttttg ttttagatag 9120
   tttgttttta gcgttttgat ttttgattat cgtttgtgta tgttaggttt tgtatttgtt 9180
   aggcgggagt ttaaggatta ttgtcgttag ataataatag ttaggagttt agggattttt 9240
   ggatgaaagg tgttagagat aagtcgttag tgacgtacga ggatttggat aaggttttgg 9300
   agcgaggtgg taatgttggg tacggcggta gattatgttt aatatggggg aagggagtgt 9360
   atgtggttta ggtttgtgcg tgtttttgtg tgtgtgtgta ggtgagtatg gcgtgtgttg 9420
   ggatatattt atgtgtattt ttgtgtgagt gtgtaggttt atatgtgagt gtgtatatat 9480
   atttgtgaga gtttgtatgt atgtatacgt gtgaggtgtt gatatatgtt tatgtaggta 9540
   ttagtttgtt tgtaaatatt tttgtgtaat aataataaag ttcgaatagt tattaagagt 9600
   atagatatga agttagattg tttgggttaa atttttagtt agttagtttt tcggtgtttt 9660
   tgtttttttt tttgtaaaat ggcgtttatt ttattaggat tttaaaatta gttaatatgg 9720
   ttgggcgttt gtaattttag tattttggga ggttgaggta ggcggattat aaggttagga 9780
   gatcgagatt attttgttta atatggagaa ttttgagaat tgtttaaatt taggaggtgg 9840
   aggttgcggt gagtagagat tgtattattg tattttagtt tgggtaatag agttagattt 9900
   tattttaaaa aagaaaaata aaaaataaaa aaattatgaa tttattttta ggttgaggag 9960
   tttagtattt tggttgtgaa atattttttt tataaaattt tgggatggag attacgggga 10020
   ttaggtgttt ttttgtgata atttttgggt atggtggttt aggcgtaaat ttggagtgtg 10080
   gttataatat atattgtgta tttttataag gatgttatag agtttgggta ttataaaaga 10140
   ggagtttttt aaggaattga aattattaga taggagagag agttttgggt agatagggtt 10200
   gttcgtgtta aatattttag ttgtggtata agggaaaggg tgggagttat gaaattgttt 10260
   tattttgggt ttaggtttgg gttttgtcgt tagttagtta agtgattttg gttatttatt 10320
   tttgtggttt tttatgagta aaaggcggaa atttattttt atttagaggg taggtttgat 10380
   tttttttaat tagtatttat ttgtttatag taggaaggat tgaggtttaa agttggaggt 10440
   gggtaggaag gattgaggtt taaagttgga ggtgggtagg aaggattaag gtttaaagtt 10500
   ggaggtgggt aggaaggatc gaggtttaaa gttggaggtg gttgtttaga gttttagtag 10560
   aggtttttgg ggtattttat tgagtgtttg gtaggagtgg gtgtttgttt tagggttggg 10620
   ttgagttgtt tttattagga tttttcgtta tttgtatagt gaggggattg ggaggtttag 10680
   agagttatag tttgggttta aaataagtaa gaggtttttg agtgtgagga ttgttttgga 10740
   gtggaatggt ttttataggt aggagtgagt tttttgtagt tagaggtatt taagtagttg 10800
   aaggataatt tttgggtagg aagttgtaga gatggtcgta gcgtggatta gaattgttgt 10860
   tttggttatt tagattttat tttagtttgg tttttttgga tagtattttt gtaatagtga 10920
   gttggtgatt ttacgtttta gaatttcggt ttttatattt gtaaaatggg aattatatga 10980
   tatttattat gtgttagata ttttgttggt atatagtata tattatttta tttaattttt 11040
   taagtaggga taagttattt ttatttttta tatgaggaag ttgaggtata gagaggtgaa 11100
   gtgaatggtt taaggttata tagttgggaa gatagggagt taaatttgaa ttttagtttg 11160
   gttgttttta gattttatat cgtatttttt atgtcgattt tagttttttt tgtgtttata 11220
   gg 11222

<210> 246
<211> 11222
<212> DNA
<213> Artificial Sequence

<220>
<223> chemically treated genomic DNA (Homo sapiens)

<400> 246
   tttgtgggta tagggaaggt tggagtcggt atgggaggtg cggtgtgagg tttgggggta 60
   gttagattag agtttaagtt tagttttttg tttttttagt tgtgtgattt tgggttattt 120
   attttatttt tttgtgtttt agttttttta tataagggat ggggataatt tgtttttatt 180
   tgaaggatta agtgagatag tgtgtgttat gtattaatag ggtgtttggt atatagtgag 240
   tgttatataa tttttatttt atagatgtgg aaatcgaggt tttgaggcgt aaagttatta 300
   gtttattatt gtaggggtgt tgtttagaga agttaggttg gaatgaggtt tgagtgatta 360
   aaatagtagt tttagtttac gttgcgatta tttttgtagt tttttgttta gggattgttt 420
   tttagttgtt taaatatttt tagttatagg aggtttattt ttatttgtga gggttatttt 480
   attttagagt aatttttata tttagaaatt ttttgtttgt tttgagttta agttgtgatt 540
   ttttgaattt tttagttttt ttattgtgta gatgacgaag ggttttggtg ggagtaattt 600
   aatttagttt tgagataggt atttattttt gttaggtatt tagtgaggtg ttttagaggt 660
   ttttgttggg attttgagta gttattttta gttttagatt tcggtttttt ttgtttattt 720
   ttaattttag atttggtttt tttttgttta tttttagttt tagattttag ttttttttgt 780
   ttatttttag ttttagattt tagttttttt tgttgtgagt aggtgggtgt tggttaaagg 840
   gagttagatt tgttttttgg gtaggagtga gttttcgttt tttatttatg gaagattata 900
   gagataagtg gttaaggtta tttggttagt tagcggtaga gtttagattt aaatttaaaa 960
   tggaatagtt ttataatttt tatttttttt tttgtgttat agttgaaatg tttggtacgg 1020
   gtaattttgt ttgtttaggg tttttttttt tgtttaatgg ttttagtttt ttgaaaagtt 1080
   ttttttttat gatatttagg ttttgtgata tttttgtaaa agtatatagt atgtattgtg 1140
   gttatatttt agtttgcgtt ttgagttatt atgtttagaa gttgttataa ggaagtattt 1200
   gattttcgta gtttttattt tagggtttta tgaggagggt attttataat taggatgttg 1260
   agttttttaa tttgaaaatg agtttatggt ttttttgttt tttgtttttt ttttttgaga 1320
   tggaatttgg ttttgttgtt taggttggag tgtagtggtg taatttttgt ttatcgtaat 1380
   ttttattttt tgggtttgag taatttttag aattttttat gttaggtagg atggtttcga 1440
   ttttttgatt ttgtgattcg tttgttttag ttttttaaag tgttgggatt ataggcgttt 1500
   agttatatta attgatttta aaattttgat ggggtagacg ttattttgta gaggaagaaa 1560
   tagaggtatc gaaagattaa ttagttgggg atttaattta ggtagtttgg ttttatgttt 1620
   atatttttga tgattattcg gattttgtta ttgttatata gagatattta taggtaaatt 1680
   gatatttata tggatatgta ttaatatttt atacgtgtat atgtatgtag gtttttatag 1740
   atgtgtgtgt atatttatat atgggtttgt atatttatat agaggtatat ataaatatat 1800
   tttagtatac gttatattta tttgtatata tatatagaga tacgtataag tttgaattat 1860
   atatattttt tttttttatg ttgggtatgg tttgtcgtcg tgtttaatat tgttatttcg 1920
   ttttaggatt ttatttaaat tttcgtgcgt tattgacggt ttgtttttga tattttttat 1980
   ttaagggttt ttgagttttt gattattatt atttggcgat aatgattttt gaattttcgt 2040
   ttgatagatg taagatttgg tatgtatagg cgatggttag aggttaggac gttgaaagta 2100
   gattgtttga ggtagagtag ggttaagaat ttgggttata aggtagttgg gggttttaga 2160
   tattttgggg tttgtttttg ttttttgata tttaggaaag agaatatggg attgatagtt 2220
   acggaagttg agtttcggtt ttagttttgg cgttaatgtg ttttgtgatg ttgaagttat 2280
   ttagtttttt tggattattg tttatttata tgtaaattaa aagggtttag attcgttaat 2340
   gttttttttt ttatttaata ttagatagtt agggagagag tttatattta ttttattaat 2400
   atttttttga gatacggtat tgttttgttg tttaggttgg agtgtagtgg tgtgattttg 2460
   gtttattgga gttttaattt tttgggttta agtgattttt ttattttagt ttttcgcgta 2520
   gttgggatta taggtatata ttattacgtt tagttaattt tgtttatttt ttgtagagaa 2580
   ggggggtttt attatgtggt ttaggttggt ttcgaatttt tggatttaag cgatttttcg 2640
   attttgtttt ttaaagtgtt gggattatag gtttgagtta ttttatttta ttttatattt 2700
   attttaaata gtagagtaga atcgggttaa tttgtttttt cgtaagatta tttcggtatt 2760
   atatagttgt ttgttttttg tgggtttagg ttttgtagtt aggagttttt taggtagtta 2820
   tagaaggtta tttttttgga tttgtttgat tcggaaaatg aatagatgaa agttttttag 2880
   tttgtggaaa tggacggagg gcgtttgttt tagtatttta gaaagtttcg ttggtttgtg 2940
   tttttagttt taattttgtt gttagtgggt ttggtttatt agatagattt ttagtttaag 3000
   ttagagagaa gggaggttat ttttttgttg ttttttgaat tgaggtttaa ttggatattt 3060
   atatggaata tggagtatat tgttagattt atgaatattt ttttggtttt attagaataa 3120
   aagaattaga gcgtgggtgg tagttcgttt tgcgtgtggt aaggttatgg ggtagtatag 3180
   tatagtggtt aagagtaagg attttgggtc gggtttattg gtttgtaatt ttagtatttt 3240
   gggaggttaa ggcgggtaga ttatttgagg ttaggagttc gagattagtt tggttaatat 3300
   ggcgaaattt agtttttatt aaaaatataa aaattagtta ggtatggtgg tatgtatttg 3360
   taattttagt aagattttat tttaaaaaaa aaaaaaagga gtaaggattt tggatttaag 3420
   ttgtttgaat ttgaatttta gttttttttt tatttgttgt gtgatttggc gtaagttatt 3480
   taattttttt gttaaatgtg gtgattataa ttatatttat tttataagat tgttgttagg 3540
   ttttagtgag tgaaatattt aaatagagtt tagtaattag tatgtgttga ataaatggtt 3600
   gttgtttttt ttttttttat tatttttatt attattatta ttattattat tattattatt 3660
   attatttagg aatttttggt tttgtttgtg gttgttatta atcggtggag atttttaggt 3720
   ttagtttttt tttgtaatga gaagtagtga ttattattgg tgatttttaa atattttatg 3780
   attttgaaat tttttgtttt tttcgttttt taaaatagtt aaagtaaata aaataaaaaa 3840
   ggtttgagta aagaaaggaa aaataggggt gtggagttta tttagtagtt tttttttttt 3900
   cgaagtttta tttttggagt tagaattttt aagtgatttt ttagtttttg tgttttagaa 3960
   ttttatgttt gtgtacgtgt agtgaaggtt ttagtgtttt atagattggg ttttatggaa 4020
   tttgtttttt tttataatta gaagattgga gatataattt tttaaggtta gttagaaaat 4080
   ttagagttag tatttagaat aaagttaaga tgttaatttt tacgggtggt atttgtagtt 4140
   ttgggtatga gtttttttta ttttgttttt agggaggtta ttttttttgt attttaaata 4200
   ttgagagggt agaattagtt ggtatagttt ttgtatgttt tataatttaa gacgtgatag 4260
   ggtattaggt ttttgggatt ttttagggat tagtaatttt aggtattatg ttttattttt 4320
   ggttttcggt tttcggtaaa ttttcgtgat tttttttgat gatgtaatat tttgagtttt 4380
   ttttaagacg tataagttat tgttttaggt ttggtagttt ttaaattatt ttggaaagat 4440
   ttttgttttg taaattttgg ttttttattt ttacgtttga ttgttttgtt ttttcgttaa 4500
   aggtaaaagt gtattaagga tttagagaat tttagggtat gagcgtggga cgggatttcg 4560
   gagattatgg aagataattt tttcgtttaa ttagtagaga aataaggttt agaggtggag 4620
   agatttgttt aaggttatat ggtatatttg tggtttagga gttgatgtta atttgtgtgt 4680
   gtgtgttggt cgggggagta ttttgtgtaa tttaataggg tttgttgtag ttagatgata 4740
   gggataggag aagaaagata taaatatatt acgatgttat tatagttaat agaaaattta 4800
   attttttttt tttaagtgtt tttagtacgg attattaacg gtttaaattt tagataacgg 4860
   agtttttagg attagttatg ttttcgaggt agacggaagt tggaaatggg taaagtttaa 4920
   gggagattta aaggacgacg gagtggaaga ggaaaattgg ttttggttta taaataagga 4980
   gaaattaatt tgtaaattaa agtgttaaag ataatttatt tatttgtggt aatttcggcg 5040
   tttatttttt ttttttcgtt ggtagaaacg ttgattttta atttattaga tttttgaggg 5100
   aattagttta gaatggtgag ggaaggggtt aagagaagag tgtattattt ttgggataga 5160
   aggtaacgag gagatgagtt tttatgtata aattatagtt attttttatt tataaatttt 5220
   agaattttgt tttttttggt gtattttgga attttttgtg gtagaaggta ggatatattt 5280
   gtagattata ttatttagat tttttaaatt agagatatat ttaatatcga aggttattaa 5340
   tgagttttta ttttttgttt tttaattgta atgttttgaa aggtttttag tttgggtttt 5400
   attagtaata gttattaata tttttaggat ttagtatatg ttaggaatta tgttaggttt 5460
   tttgtatgta ttattttatt taatttttat atttttttta ggatatagat attaggatat 5520
   ttttttattg tataggagag gaaattgagg tttcgagagg ttgagtattt ttttaaaggt 5580
   tatttagtgg tttagtggta gagttaagtt tagaattaga tttgtttttt tttgaagagt 5640
   atgttttaat tttgatattt taagagtatt taatgagttg tgattttgga aatgtttttt 5700
   tttttttagt ttttttttgt ttttattttg taagagaggt ggttgtataa aattttttag 5760
   gatttttgtt aattttgatt gtttataatt ttaaaatttt ttttggtgga ttttgagtat 5820
   tttaggtttg atttttatgt tttttttagt tgttgatatt agtttgaaat ggtttttttt 5880
   ttttttttga gtttaatttt tgatatttaa aagtaaagaa aaagtaagtg atcgttttaa 5940
   tatttttaat taatgtagtt aattgattta ttttacgtgt aaatttttta ttttgtagtt 6000
   gaatattttt gattttaggg tgtgggtggt aggggtaatg gaattttttt tttttatttg 6060
   atgaggattt tttgtttatg gtttgggatg ttagttttta ttttttttta gacgtttagt 6120
   attaattgtt atttttcgtc gttgagtttt atggcgtagt ttgaagaaga taaaaagagt 6180
   aagtatgggt ttattgggtg ttttggtttt aatagttggg gtttgaggtt gtttggtttt 6240
   aagggcgttt ttatattttt tgggatgttt gataaagatc gtttaatggt tcgttgtttt 6300
   ttttttttgt ttttttttat tttttttttt ttatttaggg agggtttaat tttttttttt 6360
   ttttagtttt tatatgggaa gttattttag ggtaggtgtt attgtggcga ggtaggatag 6420
   tttaggttat agggggtggg gtggttgtgc gtgtggaaag aaggggagga ggttgggtat 6480
   tgtttggatt tttatagtcg atagaaaggg tattatagaa gtcgacgtta gtagtttgaa 6540
   atttaacgtt ttttgcggtt agttttttta agaggatttg ttatgttttt aagtttggtt 6600
   attaggtgtt tcggttggtt ttaggatagt tttggttatt cgtaattttt tacgtatttt 6660
   ttgttttttt gttagagatt ttttatttag ttagaagtga tagaaaggaa tcggatggtt 6720
   ttgtgtttat agttaggtgg ttttaggtaa gtttataatt ttttagagaa tttttgtttt 6780
   tatgattttg tttaaaggtt tttttttgaa aagttttttt tgatatattt ttttgagata 6840
   ttattaattg tttttttttt tttttttttt gtgtaagttt ttgttgtatt gttttttaaa 6900
   gggtatttta ttttttggtt tttatgttat tttttttatt aggataatag tagttagtat 6960
   ttataagaga ttttgttggg tgttaggttt tgttttaaat atttggtatt tggtaattta 7020
   tttaattttc gtaagatttt ttaatgtgat aggtattgat attattttta tttaattgag 7080
   agaaagtagg gaaatattat tgatgtataa ttagtcgttg gtagagttgg gatttgaatt 7140
   taggaagtta cgtttttttt tttgtagttt ttaggagggt aggagttttg ttttattaat 7200
   ttacgtattt tttgggtttt atatggtgtt ttgtgttaag tttgaattga ataaatgttt 7260
   attgagtaaa tgggtggatg gttttgtaaa gaaagagagt ggaggatgat tagttttatt 7320
   ttatatttat gtattttttt ttaatttatt taggtattta attatttatt tatttattta 7380
   tttgtttatt tagttattta tttatttatt tatttgttat tttttaattt atttatttat 7440
   tatttttttg tttatttatg tatttattta tttatttatt tattatttat ttatttaata 7500
   tttattaatt tttttattta ttcgatattt tttaatttat ttatttattt atttattatt 7560
   tttttgttta tttaggtatt tatttattta tttatttatt tatttattta aaatttatta 7620
   gtttttttat ttatttattt atttatttat ttatgtgttt atttatttat tttttaattt 7680
   atttatttat ttattatttt tttatttatt tatttattta tttttttatt tatttattta 7740
   ttattttttt atttattatt ttttttttta tttatttatt tatttattat ttatttattt 7800
   ttttatttat ttatttgttt ttttattttt ttatttattt atttatttat ttttttattt 7860
   atttatttat tattttttta tttattattt tttttttatt tatttattta tttattattt 7920
   atttattttt ttatttattt ttttatttat ttgtaatttt atttatttat ttattttttt 7980
   tatagtgata gagtatttat tgtgtgttag atattgttat atgttgagga taataaaatt 8040
   aaataaaata tttttttgtg tttaaggaat ttagagtttt tttagggaga taattaagag 8100
   gaatgaaaat atagtatagt ttgtttagtg ttatgattaa ggtaggtata ggtttttttt 8160
   tttatttgta taagattata tagtttataa agtggttgtt tggtaagatt atatttaatt 8220
   tttataatat ttttgtgagg tagcgagtat tatttcgttt tatagttgtg ggagatgaga 8280
   tttagaaagg ataagttagt gtttgggtta ttttgggttt gtattggatt tgttagtaat 8340
   gattgtttta tttatatagt tttttgaaga taattgtttt gttttaattt tgttgaggtg 8400
   taggtgtagt tagttatgtt ttgggatata tagttttttc gtaaatttgt tgttagttat 8460
   agttaagttg agaaagttaa tgttagggtt ttatttgaat agttagtatt tattagtttt 8520
   gttagttaga ttttagtttt agaatgtgaa tagagatgtt tggtaagaat tggataaata 8580
   ataggaaatg tagttagaaa aagagttaaa tttaaagaat ttttaaattt ggagtttttt 8640
   agtgtaattt tgggttagaa cgttttttgt gtgggttttt aaagtttggt tggtgattat 8700
   tttaggattg ttatgagtcg ttattttttg gaggagtttt gtgtttttat ttagtgagtt 8760
   ttgtgtattg agtcggttga tttttagcga ttaaaagggt tagaggtatc ggttgtattt 8820
   ttggcgtttt ttttttagtg attattttag aagttaattt taaatgttag gttagtcgat 8880
   ttttaaaggt ttattttttt agtagtggaa gaagtagtaa taaaataagt agttaaaatg 8940
   ttatgggggg tcgggcgtgg tggtttacgt ttgtaatttt agtaatttgg gaggttgagg 9000
   tgggtggatt ataaggttaa gagatcgaga ttattttggt taatatgttt aagttttgtt 9060
   tttattaaaa atataaaaat tagttgggta tggtggtacg aatttgtagt tttagttatt 9120
   tgggaggttg aggtaggaga attatttgaa ttcgggaggt agaggttgta gtgagtcgag 9180
   atcgttgtat tttagtttgg taatagagcg agtttttatt ttaaaaagaa aaaaagaagt 9240
   tatggggatg ttaaagggga ttttatttat ttatttagtt agttagttat ttgtttgttt 9300
   ttttgtttat ttatttattt gaaagtttat tattgaggtt ttagtttgtg ttaggtttag 9360
   ttttagaagg aaagataatt ttgagagggt ttgtttcggt tttagatttt tagtagtttg 9420
   atttttattg ttggttattc gttggaggtg agtttagggt gtgagagagg agggtagaaa 9480
   gaaggagatg gtattgagtt gtgttatatt tgatttgggg ttttttgttt ggtgcgttgg 9540
   ggaaggttaa gaggtagatt tgagattttt ggagattgag acgtaatgtg cggtttttaa 9600
   atgttttttg gtttgtgttt tttcgagagt gttagaatat ttttgagggt gcggggtagg 9660
   gtagggttga ttattattcg aattttagtt tttttttcgt ttttggtttt ttgagttgtt 9720
   tggggatggt ttgagtattt aaaattttat taatttatta ttgatgttga aatgtttaat 9780
   atgaagttat tattcgtttg gtaagcgttt ttattaattg gtatgtttat ggaaatttgt 9840
   agttttgaag ttgatggatt ttttaagttt tattatttaa gttaatggga aggtatcgat 9900
   atttgtttgt atttatatat ttttataata gagtagcgtt attttatagt tgttatggtt 9960
   atttggagtt attagttttg tttgggggtt attattattt ttgtgatagt ttttattatg 10020
   gttagggtag agaagttgta gatatatgat aggaaagtta ggttgttttt gtgtgtaaaa 10080
   ttttttatag ttttatacgt ttatgttata tatacgtata tatacgtacg aatgtagggt 10140
   atattagttt gatatttagt ggggtaaaat tggattttat taagttagat tttttttttt 10200
   ttttttttat attataggat tttttgaatt taaaattgag atttgtagaa ttatttttaa 10260
   ggtttttaat tttggaattt tgagatttta gaattaaaat tttagagtgt taaagttttt 10320
   aattttttag tatcggagaa atttgggttt tagattttag atttatagag agttagaagt 10380
   agagaaattt agaattatag ggttatagta ttttgtgatt aaaagaggtt tttaacggta 10440
   ttttgtttaa ttttttatta aatatagttt tttttataga ttattatttg gtatttgttt 10500
   gaatattttt gggagtggtg agtttattat ttaaagtttt aaaaattttt ttttatgtcg 10560
   aattaaaaga tgtttttatg ttacgtgtat ttttttgcgg tagtttcgtt tgttttttgg 10620
   ggatatagag gtttagttga gtttgtttta taagagaagg agtaggttta tttaagattt 10680
   tttttgttta gattgaatat tttaggattt ttattgagta ttttttttag gtaaggtaag 10740
   tcgggggttt tagtttggtg gagaaagacg ggttttgtta ttttcggttt ttagttattt 10800
   ttatttttgg ttatatgtat ataaagagaa cgttatagta aaggggattt tagtatttat 10860
   ttgtataggt ttgagttttt taggtagttg tgtatttggt ttatagtttt tatggttaga 10920
   aaatttaatt taggtgattt aggaatttgt tagtttttgg atagttgata attttttttg 10980
   gtaaatttgg gtttaagata ttattaaggg agattaaaga tgtggtaaga aggagaattt 11040
   tggtttttta aagttaggat tttttacgat ttttattatt tttttttttt tttttttggg 11100
   aattagtttt tggaggttag tttgggtagt gttttttttt tgttagtagg tttttttatt 11160
   ttatttttag tttagtttgt gtttttttgg tagagtttat ttttttggat tagcgttagg 11220
   tt 11222

<210> 247
<211> 5952
<212> DNA
<213> Artificial Sequence

<220>
<223> chemically treated genomic DNA (Homo sapiens)

<400> 247

atatttttga tttttatatg agtatatgtt cgagtagttt ggattatagg tgtgtagtat 60
tatatttagt taatttttaa atttttgtgt agcgatgaag ttttatttta ttgtttaggt 120
tggttttaaa tttttgggtt cgagtaattt ttttatatag gttttttaaa gttttgggat 180
tataggtatg agttatagta ttcgagtaag atgatttttt aagataggtg aagatgttag 240
tgtttagaat tttattttta ggggtacgta ggtatgaaag gttatattta agtgagtgtt 300
atgttagttt ttttagaaat ttagttgttt ttttttaaat ttagtttaat aaaaggaggt 360
ttggagaatt aatgtatata agttagaatt atagaatgta tagtatgatt attaggatgg 420
ataatatgtg gtttgaataa attttttatt tattatattt agttttttta ggttaatgga 480
atttagatta agtatatttt tgaagttagt attgtgtgac gaataggggt ttggtggaag 540
tgatgttaat agggttttag gttagtttta gagttttttt tttttttttt tttcgttttt 600
tgtttttttt ttgtttgttt tttgtttttt ttttttagat agtttttttt ttgttgttta 660
ggttggagtg taatggtgtg atttcggttt attgtaattt tcgtttttta ggtttaagta 720
atttttttgt tttagttttt cgagtagttg ggattatagg cgttcgttat tatagttggt 780
taatttttgt atttttagta gagatagggg ttttaggtta ggttggtttt gaatttttga 840
ttttaggtga ttcgtttatt tgggtttttt aaagtgttgg gattataggt attagttatt 900
gtatttggtt taagaaatat ttttaagtat gtttgatttt ggtaaatgtt tttgattttt 960
tttgtaggaa aaaattttag agatcgaatt tttgtgttaa agaataagta aaattttaag 1020
gtttttgata tatattttta agtttttatt agaaaaatta tattaatgta tatttttatt 1080
tgtaatgtat ttaagtgttt atttttttga atttttttta atatattagg tgttaatatg 1140
atttttatta atttggtagg ttaaaatttt gtttttattg aatttatatt tttttgttta 1200
ttaatgtggt taataaattt tacgtttatg aagattagga ttggatagtg gaggttagat 1260
atttttggtt ttttgtttag atgaataaat tttttatttt ttttaattta tttaagttga 1320
gttttttgtt attgaaattt aaattatatt aattaatata attttttgag gtatattgaa 1380
gttttaaaat ttgtatggtt atttattatg ttttatgggt ttttttttta tgtttggaag 1440
tttataggaa gattttataa atatttatta aaatattttt ttatttttta ttgggttaag 1500
atttgaattt gaatttttta tttagaattt agtgaataat tattaggaaa agtttttttt 1560
tttttttttg agatggaggt ttgttttgtt atttaggttg gagtatagtg gcgtaatttt 1620
ggtttattgt aatttttgtt tttcgggttt aagtaatttt tttgcgttag tttttttagt 1680
atttggaatt ataggtgtgt attattacgt ttagttaatt tttgtatttt tagtagagac 1740
ggggttttat tatgttggtt tggttggttt cgaatttttg attttagatg atttatttat 1800
tttagttttt taaagttttg ggattatagg tatgagttat tgcgtttatt tagaaaagtt 1860
tatagttttt attttttttt aatagtttaa ttaattattt gattaaaatt tattttattt 1920
ttttttttat tgatttaata gtatttttat tagatgttag agtagttatt taaaatgtaa 1980
attggattat attaaagatt tgttgttgat tatatttttt tgtattttta gagagtttat 2040
aaagttttgt aagagtttat tttagttttt ttgtaatttt attttgtatt atttttgttt 2100
tagatatatt agtttgagtt ttatattaaa tttgtttgaa tatattttaa gtatgttgat 2160
ttttataatt atgttatttt tttagtttag aatgtttttt tttaattttt taagtagttg 2220
ttttttttga taagatgtta tttttttaga gaggtttttt ttgattatag ttataggtat 2280
gtttatgttt ttattttagt ttaatataaa atttacgtaa ttgtatattt ttttattatt 2340
tgatattttt aggaggatat tatttttgta atagagtaga aataaataga ttgatgtata 2400
tggtggattt tttatggata gagtttgtta tttaaaatgt ttttaaattt attatatgta 2460
atttggtatt aaattaaaat gttattatgt aagcgaatgt ttttaaagtt taaaagtttt 2520
tttaaaataa aatagattcg ggcgcggtgg tttatgtttg taattttagt attttgggag 2580

gttaaggtag gtagattata tgaggttagg agtttaagat taatttggtt aatatggtaa 2640
aatttcgttt ttattaaaaa tataaaaatt agttaggtgt gatggcgtat gtttgtaatt 2700
ttagttattt aggaggttga ggtatgagaa ttgtttgaat ttaggaggcg gagattgcgg 2760
ttagttaaga ttatattatt gtattttagt ttgggtgata gagtgagatt ttgttttaga 2820
aaaaaaaaaa agaaaaagaa aaagaaaaaa aaaaagaaat tttaagtgat aaagtattgt 2880
gtaatagttt gaggttgggt atagtggttt atgtttgtaa ttttatattt tgggagatcg 2940
attgggaaga ttgtttgagt ttaggagttg gagattagtt agagcgatat agtgggattt 3000
tttttttata aaaatattta aaaattagtt aggtatagtg gtgtattttt gtagttttag 3060
ttatttggga ggtggtaaga ggatttttcg agtttagaag ttggaggtgg tagtgagtag 3120
ttattttgtt attgtatttt agtttgggtg ttaaagggag attttttttt ttagaaaata 3180
ataaaaaaaa aagtttgata gtttttttgt ttgtgttata taaaataatg gtatatatta 3240
taattgaagg tattttataa tagaaatgtt tttttttgtt tttttttaag gttgatttag 3300
tgttttaaga ggttgatata gaagggtaaa gttaagtttt tataaaattt agggaagaga 3360
ttgtgaaatt tattttagaa ttttgtatgg agttatagtt gaattaggaa aaaaaaaaaa 3420
aaaaaaaaaa ggaaagaaag gtattttaga gataaagaat agattaagga gaggagatat 3480
ttgaagttat aatgattgag aatatgttag atttgaaatg aatttttagt ttttttaata 3540
agataaataa aattaatttt atatttagat atatggtaga aaaattaaag aattttgttg 3600
attaaggtat taaaagtaat taaagagaag tggtgtgaag aaagtaagag agaaataata 3660
aattttgttt attttgtaat aattgaaaat ttttggttgg gcgtggtggt ttacgtttgt 3720
aattttagta ttttgagagg tcgaggtagg tggattattt taggttaggt gtttaagatt 3780
agtttggtta atatggtgaa atttcgtttt tattaaaaaa aaataataat aataatataa 3840
aaattagtcg ggggtggtgg taggtatttg taattttaga tattcgggag gttgaggtag 3900
gagatttatt tgaatttggg aggcggaggt tgtaatgagt tgagatcgcg ttattgtatt 3960
ttagtttgga tgatagagta ggattttatt ttaaaaagaa aggaaaagaa aagaaaaaat 4020
attaaatgtg tacgtttttt gatttagttg tattatttta aggagttgat attattaaaa 4080
ttgtttaagt gtttaaaggt gtttgtagtt aaataatagg agattgataa attatgttat 4140
atatatgtga tgttatgttt taaagaggta ttgatatgat aaaagatgta cgtggtataa 4200
aattaaatgt attttattaa gtattttttt aagtgtttac ggaatgagtg tatttttgaa 4260
aaaaaaaaag tgtattcgaa tttttaaaaa agttttaaaa gttttatata atgaacgatt 4320
gagtgattat aagagttggc gggggaatgt taagaggatg atagggagtt aagtttaata 4380
gaataattta ttttttattt ttgtgatatt tacgagcgta ttaattttgt aattgaaaaa 4440
taaagtgtat atttgtagta gttgtatttt ttttaggttg taaggaggtt ttttttttcg 4500
gtaggtttga tttgtatttt atttttattt tcgtggttgg aaattttta tttacgtagt 4560
gggaggttga ggagttatta taaagttggg gtttgacgag tcgggatcgg gattcgattt 4620
ttatatatgt tcggatttgt tttgcggtcg ggtttaggag ttaaagaggc ggggagattt 4680
gcgcgacgtt gtttcgtttt gcgttcgttt tttttaatgt atgttttagg gggcgggttt 4740
cgcggggagt atggatacga ttggttttaa agtttttttc gtaaggtcgt gggttggata 4800
gcgtggtgac gtcgtaacgc ggcgtagggt gagagcgcgc gtttgcggac gcggcggtat 4860
taaacggttg taggcgtagt agagtggtcg ttgttttttt aggttttagt cggtcgtcgc 4920
gacgttcgtt cgttcgtttt gaggtttttg aagtcgaaat tagttagatt tttttttttt 4980
tcgtttgttt gtagcggcgt tgttgttatt tcgttattat gttcgaggcg cgtttggttt 5040
agggttttat ttttaagaaa ggtgttggag gtatttaagg attttattaa cgaggtttgt 5100
tgggatatta gttttagcgg tgtaaatttg tagagtatgg attcgtttta cgtttttttg 5160
gtgtagttta ttttgcggtt tgagggtttc gatatttatc gttgcgatcg taatttggtt 5220
atgggcgtga attttattag gtgagtttcg cggtttcggg aagtcggttt cggttcgttt 5280
gtattttcgg tgtttggcgg gagcgttttc gagtttagtt tttattggtt ggcgtgggtt 5340
attcgcgttt ttttattggt ttgttacgta gtggggtggg gtttagttga gcgcgcggtt 5400
cggaaaagtt cgcgttggtt gttgcgcgaa tttgtttttt cgcgttaaag ttataaagcg 5460
ggtggtggcg ggaaaattaa gggttttttc gtagtgttag gaatattgtt ttagggtttt 5520
ttgtttatta aattttgttg gttttgaatg gacgttttag ttgtggtttt tttgtttttg 5580
agacggtttc ggtgtgttgt tcgggttggt ttttaatttt tgggtttaag cgattttttc 5640
ggtttagtcg cggttgattt taaatgtttt ataatgtttt tgcgagaaat gtggtagttt 5700
gttattttat ttagtggtag gagattgttt ttatttagaa gggatattgt tggtggtatt 5760
ttagtataaa tattgttaga tgcgttttaa aacgtttgta ttaataatgg tattttttag 5820
tagttcgttt attttttatt agttttgaga cggtttgatg ggtgagagtg gtaatttttt 5880
ttaatcgcgt tcgaaatata gttttttagt agacggcgtt gattttaaag tatgtgtttt 5940
ttgtttttta gt 5952

<210> 248
<211> 5952
<212> DNA
<213> Artificial Sequence

<220>
<223> chemically treated genomic DNA (Homo sapiens)

<400> 248

attagaagat aggagatata tgttttaaaa ttaacgtcgt ttgttgaagg gttgtatttc 60
gaacgcggtt agaaggggtt attattttta tttattaggt cgttttagaa ttggtggagg 120
gtaagcggat tgttggagga tgttattatt aatgtagacg ttttgggacg tatttggtag 180
tatttatatt aaaatattat tagtagtgtt ttttttggat agaaataatt ttttgttatt 240
aagtaggatg ataggttgtt atatttttcg taagggtatt ataaagtatt taaagttaat 300
cgcgattgag tcgggaggat cgtttgagtt tagaagttgg agattagttc gggtaatata 360
tcgagatcgt tttagaaata agaaagttat agttaaagcg tttatttaag gttaatagga 420
tttagtgagt aaagagtttt ggaatagtgt tttggtatt gcggaaaaat tttgattttt 480
ttcgttatta ttcgttttgt gattttggcg cgaaaaagta ggttcgcgta gtagttagcg 540
cgggtttttt cgaatcgcgc gtttagttgg gttttatttt attgcgtggt aggttaatga 600
gaaggcgcgg atggtttacg ttagttaatg agggttaggt tcgaaagcgt tttcgttaag 660
tatcggaggt gtaggcgggt cggggtcggt ttttcggggt cgcgaggttt atttggtgag 720
gtttacgttt atggttaggt tgcggtcgta gcggtaggtg tcgaagtttt tagatcgtag 780
ggtgagttgt attaaagaga cgtgggacga gtttatgttt tgtaggttta tatcgttgga 840
gttaatattt tagtaggttt cgttgatgag gtttttgagt gtttttaata ttttttttga 900
ggatggagtt ttggattagg cgcgtttcga atatggtggc ggagtggtaa taacgtcgtt 960
ataggtaggc gggaaggagg aaagtttagt tggtttcggt tttaggagtt ttagagcgag 1020
cgggcgaacg tcgcgacgat cggttgagat ttagaaagat aacgattatt ttgttacgtt 1080
tgtaatcgtt taatgtcgtc gcgttcgtaa gcgcgcgttt ttattttgcg tcgcgttgcg 1140
acgttattac gttgtttagt ttacggtttt gcggggaaga ttttagggtt aatcgtgttt 1200
atgtttttcg cgaggttcgt tttttagagt atatattgga ggaagcgggc gtagggcggg 1260
gtagcgtcgc gtaggttttt tcgttttttt gatttttgaa ttcggtcgta gaataagttc 1320
gggtatatgt ggagatcggg tttcggtttc ggttcgttaa gttttagttt tatggtggtt 1380
ttttagtttt ttattacgtg ggtagaaagt ttttagttac gaaagtgaaa gtgaaatgta 1440
aattaagttt atcgggagga aaagtttttt tgtagtttga agagagtata gttgttgtaa 1500
atatgtattt tattttttaa ttatagaatt gatgcgttcg taggtgttat aagataaaga 1560
ggtgaattgt tttgttaaat ttagtttttt attatttttt taatattttt tcgttagttt 1620
ttataattat ttaatcgttt attgtataaa gtttttaaag tttttttaaa agttcgaata 1680
tatttttttt tttttaaaaa tgtatttatt tcgtaaatat ttggaaaagt atttaataaa 1740
gtatatttaa ttttatgtta cgtatatttt ttattatatt agtatttttt taaaatatag 1800
tattatatgt atataatata atttattaat tttttgttgt ttaattataa atatttttga 1860
gtatttaggt aattttggtg atattaattt tttgaagtaa tatagttgag ttaaagagcg 1920
tatatattta atattttttt tttttttttt tttttttttt gagatggagt tttgttttgt 1980
tatttaggtt ggagtatagt ggcgcgattt tagtttattg taattttcgt tttttaggtt 2040
taagtgagtt ttttgtttta gtttttcgag tatttgggat tataggtgtt tattattatt 2100
ttcggttaat ttttgtatta ttattattat ttttttttag tagagacggg gttttattat 2160
gttggttagg ttggttttga atatttgatt tgaggtgatt tatttgtttc ggttttttaa 2220
agtgttggga ttataggcgt gagttattac gtttagttag aaatttttaa ttgttatagg 2280
atggatagga tttgttgttt tttttttgtt ttttttatat tatttttttt tagttatttt 2340
taatattttg gttagtagaa ttttttagtt tttttattat gtatttaggt atggagttag 2400
ttttatttgt tttgttgagg gaattgaaga tttattttaa gtttgatata tttttaatta 2460
ttatggtttt aaatattttt tttttttagt ttatttttta tttttggaat gttttttttt 2520
tttttttttt tttttttttt tttttttagt ttagttgtga ttttatataa ggttttaaga 2580
tgagttttat agtttttttt ttggattttg tggagattta attttatttt tttgtattag 2640
ttttttaaag tattgggtta gttttaaaag ggaataggaa agggtatttt tattataaga 2700
tgtttttaat tgtaatatgt attattattt tatgtagtat aagtaagaaa attgttaaat 2760
tttttttttt attatttttt agagagggga gttttttttt gatatttagg ttggagtgta 2820
gtggtaagat gattatttat tgttattttt aatttttagg ttcgagagat ttttttgtta 2880
ttttttaaat agttgggatt ataggggtgt attattgtat ttggttaatt tttaaatgtt 2940
tttgtagaga gaaggtttta ttatgtcgtt ttggttggtt ttttaatttt gggtttaagt 3000
aattttttta atcggttttt taaagtgtga gattataggt atgagttatt gtgtttagtt 3060
ttaaattgtt atataatgtt ttattattta gaattttttt tttttttttt tttttttttt 3120
tttttttttt tttttgagat agagttttat tttattattt aggttggagt gtagtggtgt 3180
gattttggtt aatcgtaatt ttcgtttttt gggtttaagt aatttttatg ttttagtttt 3240
ttgaatagtt aggattatag gtatgcgtta ttatatttgg ttaatttttg tatttttagt 3300
agagacgggg ttttgttatg ttggttaggt tggttttgaa tttttgattt tatgtgattt 3360
gtttgttttg gttttttaaa gtgttgggat tataggtatg agttatcgcg ttcgggttta 3420
ttttgtttta aaagaatttt tagattttaa aaatattcgt ttatataata atattttggt 3480

ttaatgttaa attatatgta atagatttga agatatttta aatgataaat tttatttatg 3540
ggggatttat tatgtatatt aatttgttta tttttgtttt attatagaag tgatgttttt 3600
ttaaaagtgt taaatgataa agaggtgtat agttacgtaa attttatgtt aaattgaaat 3660
gaggatataa atatgtttat gattgtggtt agagaaggtt tttttgagga ggtagtattt 3720
tattagagaa gatagttgtt tggagagttg gaaaaaagta ttttaggttg agagaatagt 3780
atgattataa aggttagtat atttggaata tatttagata agtttgatgt agaatttaga 3840
ttgatgtgtt tgaagtaaga gtggtgtaag gtgaggttgt agagaggttg gggtgagttt 3900
ttgtaggatt ttgtaggttt tttaaaagtg taagggaatg tggttaatag tagatttttg 3960
atgtgattta atttatattt taagtggtta ttttaatatt tagtaaaggt attattggat 4020
tagtgggaga aagaatgaag taaattttga ttagataatt ggttaaatta ttagaaaaga 4080
ataaaagttg tagatttttt tggatgggcg tagtggttta tgtttgtaat tttagaattt 4140
tgggaggttg aggtaggtgg attatttgag gttaggagtt cgagattagt taggttaata 4200
tggtgaaatt tcgtttttat taaaaatata aaaattagtt gggcgtggtg gtgtatattt 4260
gtaattttag atattaggga ggttgacgta ggagaattgt ttgaattcgg gaggtagagg 4320
ttgtagtgaa ttaagattgc gttattgtat tttagtttgg gtgatagagt aaatttttat 4380
tttaaaaaaa aaaaaaaaga ttttttttaa taattattta ttaaatttta gataaagaat 4440
ttaaatttaa attttgattt aataagaaat ggaaaaatat tttggtaaat atttatgaga 4500
tttttttatg ggtttttaaa tataagaaaa aaatttataa aatataatga ataattatat 4560
aagttttaag attttagtat gttttaaagg attatattgg ttagtatagt ttaggtttta 4620
gtgatagaga atttaatttg aatggattaa gagaaataga aaatttattt atttaaataa 4680
aaagttagag gtatttggtt tttattgttt agttttagtt tttataaacg tgaagtttgt 4740
tagttatatt aataagtaaa gaaatgtaaa tttaatggag atagaatttt aatttattaa 4800
attaataaaa attatgttaa tatttggtgt gttaggaagg atttaaggaa gtaggtattt 4860
aaatatattg taagtgggag tatatattgg tataattttt ttggtggaaa tttagaaatg 4920
tgtattaaaa gttttagaat tttatttatt ttttgatata gaaattcgat ttttagggtt 4980
tttttttata gaagaaatta ggggtattta ttaagattaa gtatgtttaa gaatgttttt 5040
tgagttaggt gtagtggttg atgtttgtaa ttttagtatt ttgggaggtt taggtgggcg 5100
gattatttga ggttaagaat ttaaaattag tttgatttaa aatttttgtt tttattaaaa 5160
atataaaaat tagttagttg tggtggcggg cgtttgtagt tttagttatt cgggaggttg 5220
agataggaga attgtttgaa tttgggaggc ggaggttgta gtgagtcgag attatattat 5280
tgtattttag tttgagtaat aagagggaaa ttgtttaaaa aaaaaaaata aaaaataaat 5340
aaaaagaaaa taaaaaacga aaagagagag agagaagggt tttaaagttg gtttaaagtt 5400
ttattagtat tatttttatt agatttttgt tcgttatata gtattgattt taggaatata 5460
tttgatttga gttttattaa tttgagaggg ttgagtgtaa tggatgagaa gtttgtttag 5520
attatatgtt atttatttta gtaattatat tgtgtatttt ataattttga tttatgtgta 5580
ttgatttttt aagttttttt ttattaggtt ggatttgaaa aggagtagtt gagtttttag 5640
aaggattgat atgatattta tttagatatg attttttata tttacgtgtt tttgaaggtg 5700
aagttttaga tattaatatt tttatttgtt ttagaagatt attttgttcg agtgttgtgg 5760
tttatgtttg taattttaga attttaggag gtttatgtgg gaggattgtt cgagtttagg 5820
agtttaagat tagtttaagt aataaagtga gattttatcg ttatataaaa atttaaaagt 5880
tagttgggtg tggtgttgta tatttgtagt ttaagttatt cgggtatata tttatataga 5940
aattaggaat at 5952

<210> 249
<211> 6664
<212> DNA
<213> Artificial Sequence

<220>
<223> chemically treated genomic DNA (Homo sapiens)

<400> 249

tattataagg gtattaattt tattaatgag ggaaaagttt tcgaggttta ataattattt 60
ttcgaaggtt ttatttttta atattattat tttggggttt aagttttaat atatgtattt 120
aggaggggta tatattttta aattatagta attcgtaagt gatatatatt ttaggtttta 180
tggaatttat atttattttt ggttatattt agttacgtgg atgttttata gtattttatt 240
attaatatgt ttaaagtgat gttttatgtt ttatagttgt tttttagtat ttttttagaa 300
tagtaggatt tttagtttgt tgtaaagtta tatatttaag ttatttttga tgattttttt 360
tttttttttt aatttagttt tagtttttaa aaatatttta ttgttatttt ttttttttga 420
aattgatatt tttttaagtt tagtatttgt tgggattgtt gtagagtttt ttattgtttg 480
ttggttttga tgttgtatta ttagtttttt ttatgatttt ttttttttaa tttttattta 540
tgattatgtt attgttttgt ttatattttt ttgtttaagt ttaacgttta atttaaaatt 600

tttagtatga ttttgaatat tatttttaag gtatagatat tatttaatta tttagtttaa 660
tgtttatgat ttttgttgag tatttagatt ttgtttatgt ttagcgtatt gaattaattt 720
tagtttataa atttgtattg tttttttttg ttaatggatt aagtttttta tttttatttg 780
tttttttggt aaattttttt tattttttaa gatttagttt agttaatttt gttatgaagt 840
tttgtttgat gtttttttta ttatgttttg tatatagtag tttttattat attgtattgt 900
aaatatttgt attttattta ggtggaagat ttatagttat ttatttgtta tttttgattt 960
ttattagttt taaggttgat atatgaattt ttttttttta tattatgttt agtttttaaa 1020
attttaatga gagattaagg tgggttgata tgtatgtatt taaagtataa ttattaatag 1080
gtttaaagtg attttttttt ttttttgtgg ggcgagaatg aagatagggg gtatggaggt 1140
tgtatatgat aaatttttat tatttgtatt ttatattttg gattattttt taaaatttat 1200
agaaaaatta tattattttt ataattagaa aaatatttaa ttttgttata gtaacgatag 1260
tttagtattt ttatttgtgg tgaaaagatt tgaatttata tattttgtat tttttgttat 1320
tttttttttt gaattaatat gaaaaaggat aaattttttt tttttgtata tatttttcgg 1380
taatttttaa attttttttt tttttttata tatttgtttg tatagagata gggttttatt 1440
atgttgttaa gttggtttta aatttttggt tttaagggat tttttatttt ggttttttaa 1500
agtgttggga ttataggtgt gagttattgt gtttggttaa tttttattat ttttatttag 1560
tagtaatgga ggttttggta agattgattt atttatttat ttatttattc gtttattttt 1620
gggttagggt agtatattta cgtggtttaa tatttaaaag atataaaatt ttttttttta 1680
tttttgtatt aggttatttt tgtattttat agagggtata tttttattag ttttttttat 1740
ttttttttcg aagtgtattt tttttttttt tttttttttt ttagaaggag tttcgtttta 1800
ttattaggtt ggagtgtagt ggcgttattt cggtttattg taatattcgt ttttagggtt 1860
taagcgattt ttttgattta gtttttcgag tagttgggat tataggtatg tgttattacg 1920
tttagttaat ttttgtattt ttagtagaga tgggttttta ttacgttggt taggatggtt 1980
tcgatttttt gataacgtga tttattcgtt ttggtttttt agagtgttga gattataggc 2040
gtgagatatc gcgttcggtt cggaggtgta tttttaagta aatgttttta tatttttttt 2100
atttaaaaaa aatatttgtt ttgatttggg ttgttataat aaaatgttat agattacgtg 2160
ttttaaataa taaaatttta tttttattta gttttggagg tttggtgtgt aagtttagag 2220
ggcgggtaat attaggtttt ggtaagggtt gattttttta ttgataggtg tttttttatt 2280
ttaattttat atgagagtaa ttaagttttt tagcgttttt ttttataaga gtattaattt 2340
tattacgagg gttttatttt tatgttttta tttaaattta attatttttt aaagttttat 2400
ttttaaatat tattttagtg gggtgaggtt tttaacgtat gaattagcgg ggtgggggga 2460
taataaatat ttagattata gaaatacgta aatataagaa ataaaattaa aaagatttat 2520
gtgtaatatt aattgatttt tttttttttt tttaaaatgg agtttattaa aatataaata 2580
tgaagttttt tagagtatat attttttttt gttttttttt gtagtttttt tttttttaat 2640
attattttta gggtagtttt tttaaaatat ggttttaagg ttgtgtgtat tttttttgtt 2700
tagatttttt ggaatagtgt ttagcgttta ttaggatttt gattgtaaat tttttttttt 2760
gattttaata ttataggatg tagtattttt tatattttat tatggggggt attttgaatt 2820
atttgggttg attttttggt ttttgtgttt tatttatata attttttttg tatggaaggt 2880
ttattttaga attttttttt tagtttttgt ttaaatgtta tttttgttag tattagtagt 2940
tttttttttt gggtttttat attgttttgt aatataagtt ataaggtaag tattagaaga 3000
gaatttttaa ttattatttt gtttttttat ttttaatatt tttagcggta attgtatttt 3060
atttattttt gtgtttatga agtatagtgt tgtattttgg agatattata tagaagttgg 3120
tttaaagggt aataaattgt atttgttaag atgatagtat ggatttataa taatttttga 3180
aattttataa agaaagatga ttgagttaaa ttgatatatt tagtattata gttgtggaag 3240
tttgtggttt tttttatgtt aaaggtaaaa tggatataat gaattagttg ttatttttag 3300
atgatataga agttgaattt tttttaagtt ttttaaagag aaagatatat taaaatttat 3360
taaaattagt attataaatt gtatagaata gtttggtttt ttagagtatt tttttggggt 3420
agtgagtttt tatttaaaaa atgaagtttg gattttaaat ataagtttta aaagtgatta 3480
ggttttagta ggagttttta aatttttatt tatattatga tttttaaatg ttttgttttt 3540
gttgtttagg aataaaaatt gttaaggttg agttagatgt aatgtataat attagatgat 3600
agatgtagtt tattgtgtta aaaggtgtta aatatttaag tgtatgtgat atattttata 3660
ttataagggt aaggaagttt tattttatat tttgatttgg gttttttaat atatatttgt 3720
agtggatgtg gtaattagtt tttgttttat gttttattag gattaatgta attagaaatt 3780
tttaaaatgg atttttatta tgtttagttt tgttttattg ttttatttgt ttattttaaa 3840
aagattttag aatggtgttt aggttgggtt ggtggtttac gtttgtaatt ttagtatttt 3900
gggaggttaa ggtaggagga ttgttggagt ttaggagttg gagattagtt agggtaatat 3960
aaggagatat tgttttcgta aataaataga aaaaaaaaaa gtaagaaaga aagagagaaa 4020
gaaggaaaga ataagaaaaa ggaaaaaaaa attaattgta gtaagtgtag aaattttttt 4080
taaggatatg atggatggta atgaagatag agtttttgat attggattat ttaaagtata 4140
atttaagttt tttttttttt ttttatagaa ttgtgaattt ttgtgtttta attatattta 4200
agttaggcgt ggtggcgagt gtttgtagtc gtagttgcgt tggaggttga ggtcgattgt 4260
ttgagtttag gatttggagg ttagtatgcg taatataatg agatttagtt tttaaatgta 4320
tgtttttttt tatatattta aaattttgat gtgaaaatat tttaaaattt aatatatttt 4380

aaatgttttt aattgtataa taaataaaat gtaaataata aaataattta atattaaatt 4440
taaaaatgag gtagaaataa agtatagcga tataaataat aaattttttt ttatattttt 4500
gaggcggttt tttgagtttt ttattttttt tttaaggtta ttgaaatgtg ttttttggag 4560
ttagttcgta aattacgtat ttagaaaaat ataattatat atttttaatt ttaagtatta 4620
gaagtgaaag taatggaatt tcgatgtaaa tataatatta tttttttgat gagttatttt 4680
gagtataata aatttgaatt gtgttaatgt tgggagaaaa aatttaaaag aagaacggag 4740
cgaatagtag tttttttgttt cgttgattag aaatagtagg acgatatttt ttcgattgga 4800
ggagagcgtt tgcgttcgta tttagttggc gttcgttttt ttgttttttt tttagtcgtt 4860
tttttttttt tttttcgcgt tttagttatt cgggaaggtt tgtttagcgt agttgggttt 4920
tgattggttg ttttgaaagt ttacggttta ttcgattggt gaattcgggg ttttttagcg 4980
cggtgagttt gaaattgttc gtatttggtt ttaaagttgg tttttggaaa ttgagcggag 5040
agcgacgcgg ttgttgtagt tgtcgttgcg gtcgtcgcgg aataataagt cgggtatagt 5100
ggttggggtt agggtcgtgt ttaggggacg gtcgagggtt tcggagggcg agtattgagg 5160
aacggggttt tttaagaagg tcggattgga ggttagggat ttgcgcgggg ttcggttggg 5220
tttcgggggg cggtgggtag ggttttcgtt tgggtttggt cgtgtgagtt atattgggtt 5280
ggttttagag ggaggttatg ggagtttagt ttggcggggt taggcggcgt gggggtgggg 5340
gttcggggtt gtagttatga gtcgaggtcg gttgttttta ggaattgttt gaagaggttt 5400
tcgggttttg attgaagtaa aattcgtttt tttacgttga gcgtttgcgg agtagtaggt 5460
ttttttttgg gttcggtttt tgcgcgtttt gttatttcgt ttcgggggtg ggaaagtggg 5520
atcgtatttg ggttaggtat aggcgcgggt ttttagagtt aggtcgtttt gagaaatcga 5580
ttttggaatt cgtgggtttt tttgggtttg ttttgtttac ggtttcggtt tatttggagt 5640
tgttaagaat agttagttta ggtatttaat ttttatgagt agtgtagtgt agtttttttt 5700
atttaaaaag atatattttt gttttttttt ggattgattt ttttttgaag atgaatgtga 5760
gaaatagaat ttaaaggttt attttgagtg tgttttataa atgattttat tattagtgat 5820
gggtgttaga aaaatttagg taattatgtt tggagttttg acgttttatt ggtaaatttt 5880
agaggaagtt ttgaattttt ggttgttagt tttttttttt aatttattta ttgtgattta 5940
agtatatcgt ttttttgttt agtttttgat attgtttagg ttataattag aattttattt 6000
gtggaattgt attttttatt tttttgtttt acggtgttta agtgatattt tttatttttg 6060
gaaaaagtat tttgaaagtt ttgagtatgt ttgtttgtga ttttagtaag tagaggaaaa 6120
agattaaaat tgttacgtgg gtaattgtaa atatttaaaa tgtttttgtc gttttaaata 6180
aaattttttt aatgaatagt ttgaatatat tttagttttg tttttataat tttaaaaaat 6240
aatgttttga ataaaattag gagtattata aagcggtata aatttgttga aatatttggg 6300
tataattttg gtatacgtag gtattatttt aaagtgttat aaaaagggaa tataattttg 6360
gtttttgttt ggaaagaata gataagttga tatttgaatt tgagggttat tttgattgga 6420
agaagttaaa tattatatta gtatagaata tatacgtttt taatgtttta ggagttaaga 6480
aggatggaaa gttattgtag gagtttttat ttttgtaaat attgtttgaa tgtaattaat 6540
atttatggaa tatttatgtt gtagtaggta ttgtggaaaa tgttttatat atagtgtttt 6600
attaatgttt aaaattattc gagttagtgg tatgtaatta agttagtttt ttttttattt 6660
tagg 6664

<210> 250
<211> 6664
<212> DNA
<213> Artificial Sequence

<220>
<223> chemically treated genomic DNA (Homo sapiens)

<400> 250
   tttaaagtga aggaaaaatt gatttaatta tatgttatta attcgagtag ttttaagtat 60
   taatgaaata ttatatgtaa agtatttttt atagtgttta ttgtagtata agtattttat 120
   aaatattaat tatatttaaa taatatttat aagggtaagg atttttataa taatttttta 180
   ttttttttga tttttgagat attggaaacg tgtatatttt atattagtgt agtatttagt 240
   tttttttaat taaaataatt tttaaattta ggtattaatt tgtttatttt ttttaaataa 300
   aaattagaat tatgtttttt ttttgtgata ttttaaagta gtatttgcgt gtgttaaagt 360
   tatatttaaa tattttaata aatttatatc gttttatgat atttttaatt ttatttaaaa 420
   tattattttt taaagttatg aggatagaat taaaatgtgt ttagattgtt tattaggaaa 480
   attttgttta aaacgataaa aatattttaa gtgtttatag ttgtttacgt gataatttta 540
   attttttttt tttgtttgtt gaggttatag ataaatatgt ttaagatttt taaggtattt 600
   tttttaagag taggaaatat tatttaagta tcgtaaaata aaagaatgaa aagtgtaatt 660
   ttatagataa aattttaatt ataatttaaa taatgttaag gattaagtag aaaaacggta 720
   tatttgaatt ataatgaatg ggttaaaaaa aaaagttgat aattagaagt ttaagatttt 780
   ttttgaagtt tgttaatgaa acgttagaat tttaggtata attatttaga tttttttaat 840
   atttattatt gatgataaaa ttatttataa agtatattta gaatagattt ttaagtttta 900
   ttttttatat ttatttttaa agaaaaatta atttagaaag aagtagagat gtattttttt 960
   aaatgaaaaa aattgtatta tattgtttat gagagttaag tgtttgggtt gattgttttt 1020
   aatagtttta ggtgggtcgg ggtcgtgggt agagtaagtt taagaaggtt tacgagtttt 1080
   aggatcggtt ttttagggcg gtttggtttt agggattcgc gtttgtattt ggtttaggtg 1140
   cggttttatt tttttatttt cgaggcggga tggtagggcg cgtaggggtc gggtttagga 1200
   aagagtttgt tatttcgtag gcgtttagcg tgggggagcg gattttgttt taattaagat 1260
   tcgaaagttt ttttaggtag tttttgagaa tagtcgattt cgatttatga ttgtagtttc 1320
   gaatttttat ttttacgtcg tttaatttcg ttaggttggg tttttatagt ttttttttaa 1380
   agttagttta gtgtggttta tacggttaga tttaggcgaa ggttttgttt atcgtttttc 1440
   gaggtttagt cgggtttcgc gtagattttt gatttttagt tcggtttttt tagaggattt 1500
   cgttttttaa tattcgtttt tcgaggtttt cggtcgtttt ttagatacga ttttgatttt 1560
   agttattgta ttcggtttat tatttcgcgg cggtcgtagc ggtagttata ataatcgcgt 1620
   cgtttttcgt ttaattttta agagttagtt ttgaagttaa gtgcgagtag ttttaaattt 1680
   atcgcgttaa agggtttcgg atttattaat cgggtagttc gtagattttt aaagtagtta 1740
   attagagttt agttacgttg ggtaggtttt ttcgggtggt tagagcgcga aagaaagagg 1800
   aaagggcggt tagagaaaaa gtaggagggc gggcgttaat tgagtgcgag cgtaagcgtt 1860
   tttttttagt cgggagagtg tcgttttatt gtttttagtt agcggagtag gaagttattg 1920
   ttcgtttcgt ttttttttta aatttttttt tttagtattg gtatagttta aatttattat 1980
   atttaaaata gtttattaaa aaagtgatat tgtgtttata tcgagatttt attattttta 2040
   tttttaatat ttagggttag gagtgtatag ttatgttttt ttaaatgcgt gatttgcggg 2100
   ttggttttaa ggagtatatt ttagtgattt taagaaggaa atggaaaatt taaaagatcg 2160
   ttttaaaaat gtaaaggaaa atttattatt tatatcgttg tgttttgttt ttattttatt 2220
   tttgaattta atattaaatt attttattat ttatattttg tttattatat aattaaaaat 2280
   atttgaaatg tattaaattt taaaatattt ttatattaga attttaaata tatagagaga 2340
   ggtatgtatt tagagattgg gttttattat gttgcgtatg ttggttttta aattttgggt 2400
   ttaagtaatc ggttttagtt tttagcgtag ttgcgattat aggtattcgt tattacgttt 2460
   ggtttaaata taattaaaat ataaaaattt ataattttat gaggggagag aaagaggttt 2520
   aaattatgtt ttaaataatt tagtgttaaa aatttgtttt ttattgttat ttattatatt 2580
   tttgagagag atttttgtat ttattgtagt taattttttt tttttttttt ttgttttttt 2640
   tttttttttt tttttttttt ttattttttt tttttttgtt tgtttgcgga gataatgttt 2700
   ttttatgttg ttttagttgg tttttaattt ttaggtttta gtaatttttt tgttttggtt 2760
   ttttaaagtg ttgagattat aggcgtgagt tattagttta atttaaatat tattttaaaa 2820
   tttttttaaa ataaataaat agaatagtaa aatagagtta gatataataa aaatttattt 2880
   taaggatttt tggttgtatt gattttaatg ggatatagaa taaagattga ttattatatt 2940
   tattataagt atgtattaga agatttaaat taggatatgg aatggaattt ttttattttt 3000
   gtgatataga gtatattata tgtatttaag tgtttaatat tttttaatat aatgagttgt 3060
   atttattatt tggtgttata tattgtattt ggtttaattt tgataatttt tgtttttagg 3120
   taatagaggt agagtatttg ggagttatgg tgtaggtagg aatttaagaa tttttgttgg 3180
   agtttaatta tttttaaaat ttatgtttaa aatttaaatt ttatttttta aatgaaaatt 3240
   tattgtttta gagaagtgtt ttagggaatt aggttgtttt gtgtagttta tggtattggt 3300
   tttaatgaat tttaatatgt tttttttttt gagaggttta agaggaattt aatttttata 3360
   ttatttaaaa atggtaatta atttattata tttattttat ttttaatata aagaagatta 3420
   taagttttta tagttgtgat gttaggtgta ttaatttaat ttaattattt ttttttataa 3480
   agttttagag gttattgtaa atttatgtta ttattttggt aaatatagtt tattattttt 3540
   tgggttaatt tttatgtagt atttttaaag tgtaatattg tgttttatag atataaaaat 3600
   gaataagata tagttatcgt tggaggtgtt gagggtaggg gagtaggatg ataattggga 3660
   attttttttt ggtatttatt ttgtaattta tgttataggg taatgtgaga atttagagga 3720
   ggaaattgtt aatgttagta gaagtgatat ttgaataaag gttggggaaa ggatttggga 3780
   gtgggttttt tatatagagg aaattgtata agtagagtat agaggttaga aagttaattt 3840
   aggtaattta gggtgttttt tatggtagaa tgtagggagt attgtatttt gtgatgttaa 3900
   agttagagaa gggagtttat agttagaatt ttgatgagcg ttgaatattg ttttaaagga 3960
   tttgagtagg agaggtatat atagttttgg aattatattt taggaaaatt attttagagg 4020
   tggtattaaa aaaaaaaaaa ttgtagagga gagtagggga aggtatgtgt tttgaaaaat 4080
   tttatatttg tattttggta aattttattt tggaaggaaa agaaaagatt agttggtatt 4140
   gtatataagt ttttttggtt ttgtttttta tatttacgta tttttatggt ttgaatgttt 4200
   gttgtttttt tatttcgtta atttatacgt tgaaggtttt attttattgg gatggtgttt 4260
   ggagatgagg ttttgggagg taattaggtt tagatgagga tatgagggtg gggttttcgt 4320
   gatggaatta gtgtttttat aagaagagac gttgaagagt ttggttgttt ttatgtgagg 4380
   ttagaatgag aaggtatttg ttagtgagga agttagtttt tgttagaatt tgatgttgtt 4440
   cgttttttga atttgtatat taagttttta gaattgagtg aaaataaaat tttgttgttt 4500
   aaggtacgta gtttatggta ttttgttatg gtagtttagg ttaagataag tgtttttttt 4560
   agataagaaa aatgtgagaa tatttgtttg aaaatatatt ttcgggtcgg gcgcggtgtt 4620
   ttacgtttat aattttagta ttttgggagg ttaaggcggg tggattacgt tgttaggaga 4680
   tcgagattat tttggttaac gtggtgaaaa tttattttta ttaaaaatat aaaaattagt 4740
   tgggcgtggt ggtatatgtt tgtaatttta gttattcggg aggttgagtt aggagaatcg 4800
   tttgaatttt ggaggcgaat gttgtagtga gtcgagatgg cgttattgta ttttagttta 4860
   gtgatagagc gagatttttt ttaaaaaaaa aagaaaagaa aagaaaatat atttcgggaa 4920
   ggaggtggaa gaaattaata aaaatgtgtt ttttgtggaa tataagagtg atttggtata 4980
   gaggtggaaa ggagagtttt gtattttttg aatattgagt tacgtgaatg tattatttta 5040
   gtttaaaaat gaacgaataa ataaatgaat gaatgaatta gttttgttaa ggtttttatt 5100
   gttattggat aaaaatggta aaaattggtt aggtatagtg gtttatattt gtaattttag 5160
   tattttggga ggttaaggtg gaggattttt tgaggttagg agtttgaggt tagtttggta 5220
   atatagtaag attttgtttt tatatagata aatatataaa aagaaaaaaa aaatttaaaa 5280
   gttgtcggaa aatgtatgta aaagaagaag atttattttt ttttatgtta atttagaaag 5340
   gaaagtgata gaaaatataa gatatataga tttaggtttt tttattataa gtgaaaatat 5400
   tgaattatcg ttgttatgat agagttgggt gtttttttga ttataaaagt aatgtgattt 5460
   ttttgtaaat tttaagaaat aatttaaagt gtaaaatata gataataaaa gtttgttata 5520
   tataattttt atgtttttta tttttatttt cgttttataa aaagagaaag agattatttt 5580
   aaatttgttg gtagttgtat tttaaatgta tatatgttaa tttattttaa ttttttattg 5640
   gaattttaga aattgggtat ggtgtaggga gaagagattt atatgttaat tttgggatta 5700
   atgaggatta aggataatag gtgagtagtt gtggattttt tatttaggtg gggtgtaaat 5760
   gtttataatg taatatggtg aagattgttg tgtgtagaat atagtggaga gggtattagg 5820
   taaggtttta tagtaaagtt gattgaattg agttttaagg aataaaaaga gtttgttaag 5880
   aagataaatg aaggtggaga gtttgattta ttgataagaa agaataatgt aagtttgtag 5940
   attgaaatta atttagtacg ttgaatataa atagaatttg ggtgtttagt aagggttata 6000
   aatattaggt taggtagtta aataatattt atattttgaa ggtaatgttt aaggttatgt 6060
   taaaagtttt ggattagacg ttggatttga ataaaaaaat gtgaataagg tagtgatatg 6120
   gttataagtg ggagttaggg gggaaaagtt atgaagaggg ttggtgatgt agtattagag 6180
   ttagtagata atggagggtt ttgtaatagt tttagtaggt gttgaatttg gggagatgtt 6240
   agttttaaga ggaagaaatg ataatgagat atttttagga gttgggattg ggttaaaaaa 6300
   gggagaagga gttattaggg atgatttggg tgtgtggttt tgtaataggt tgagggtttt 6360
   gttgttttag agagatgtta aggggtagtt atggggtatg gagtattatt ttgggtatat 6420
   tgatagtgaa gtattgtggg atatttacgt ggttggatat ggttaagggt aagtgtaggt 6480
   tttatagggt ttgaagtgta tattatttgc ggattgttat aatttgaagg tatgtgtttt 6540
   ttttaaatgt atatgttgga atttaaattt taaggtgatg gtattaagag gtagggtttt 6600
   cgggaggtga ttattaaatt tcgagggttt tttttttatt aatgggatta atgtttttat 6660
   aata 6664

<210> 251
<211> 6815
<212> DNA
<213> Artificial Sequence

<220>
<223> chemically treated genomic DNA (Homo sapiens)

<400> 251

ttgtttgcga attaaaaatt tagagttttt ttgtgaatta tgttgttaag aattgtagat 60
ttaagtttta agattgtacg aatttaaatt ttgattttat ggttttagtt ggtatgaaga 120
tataaagata attttttagt aaaaatagtt ttgaaagatc gggcgtgatg gtttatgttt 180
ataattttag tattttggga ggttgaggcg ggtggattat ttgtggttag gagtttagga 240
ttagtttggt taatatggtg aaattttgtt ttaattaaat atataaaaaa attagttggg 300
tgtggtggcg gatatttgta attttagtta tttagggagg ttgaggtagg agaatcgttt 360
gaatttagga ggtagaggtt gtagtgagtc gagattatat tagtgtattt tagtttgggt 420
gataaaagtg aaattttgtt ttaaaaaaaa aaaaaaatgg tagaatgtta tatggaaagt 480
taatttttag agatagtaat ttagatattt ataatattat attgtttttt taagtattta 540
tattaataaa ttaattaata tttgttgatt taatatggtt agggatagtg ttgggttttt 600
ataattttta agtaatttta gttagatttt gtttttaagg agtttatgag tagtatgagg 660
agataaaaaa tagataaata attttaattt aggaagaatt tgataaatgt atgataatta 720
ttattagaat ttagaggaga ggaaattttt tttgaataga gattaaagga aggaataatg 780
aatgtggtag tatttgggtt ggatttatta taagaggtat gttttttttt gataagttaa 840
gaaagtgggg atttaaggta gaagaaaaga aaagttttga gtttttggag tattaaaatt 900
ttgtttggtt gaagtatagg ttatagtcgg aggagatgga ttaggaagga tcgttatggg 960

tagagaggaa gttttgaatt tatgggggaa gttatgattt gggttaaaga tagagttggg 1020
ttatgttaag atggattttg aagtagtaaa aaaaatttta agaaagtaaa taggttgtag 1080
ggtttaggat gaagttcggg aggaaagggt agagtggttt ttaggaggtc gttaggatag 1140
gtaaggtaat gggttttaaa gggagtggtc gaaatgtaat ggaaaaagag agattgtaaa 1200
gttagaaggt ttaggaattg ttttttgatt aggtgtggaa ggtaagggaa aattagtttt 1260
cgaagaagat agtgagattt taatttgggt ggttggagag atagtgatgt tgggtataga 1320
tacggggaag ttgagaggaa tattatgttt gagaatggtg atttatattt gaataagttt 1380
gtaatgttta gtagatcgtt ggaaaagtgg ggttggagat atatttaacg gaggagttag 1440
attaattttt attttttttt atttgagaga gttagtaagt tacggttgga acgtgtgtgt 1500
ttagtaggag agggtaggga gggaagttaa gagagttggg agttcgagtg aagtttttgt 1560
taaaggtaga agaggaaagt cggcgtagta tagtatattt ttttatttat gtttattaag 1620
tttagggata aggtttatta agatgagttt ggaagagaat gttggagaga aagtggttaa 1680
gaaaattgtt tttattgaat tttttgggtt aattttgatt gtaagttttt gaataattaa 1740
agtttgtgag gagatagtta atttttttat tttttttatg ttaatagtga ataattgtag 1800
attttttttt tttttttttt tttttttttt gttttttttt tttttttttt tgaatatttt 1860
tgtttttttt tgggattggt ttagagtatg ggtggttatt gttgatttat aggaggtatt 1920
attgttatta ataaagggta atagtttttt tttttaatat ttatttatat ttagtattta 1980
tttttaatat tgattatgga gagagttttt ttgtgtttaa atattgtaat attgggggtt 2040
ttttaaagta taaaaatata tatttgtatg atggtattat taatattttt atggtttttt 2100
attttttttt tgtattggtt ttaagagtta tttataaatt ttttagtaat tgtatagtgt 2160
tttagggtta gagatcggtt atttttggta ttgtgattag agttatttaa tatttaaggt 2220
ggtgattaat gtttggtaat aaagttttta ttgggtgtta tgtgttttgg gattttgagc 2280
gtgggtattt taggagtatt ttagtattgc gtgttagtat tatggtcgag agaatagttg 2340
agaaagtggt taagaggtgg atttatgtga acgttattgg gaaatgagag atttcgtttt 2400
taattacggt tagtgtaatt cgaaagttta aaattagttt aaaataaagg tatttatttt 2460
tattttatgt ttatatttta ggtttttaat aatacgtatt ttttatatgt ttatagaaag 2520
tagttaattg agttatttat ggaaaggttt gtgggtttgg ttaacgaagt ggaggagtat 2580
tatattttag ttggaaatat atttttagaa tgttaaaata tttattttaa agtttggttt 2640
tttggtgtaa tcggaggtat ggtaatgttt ttgtttagag attgggggtt agggttagta 2700
aggtatttga tttatatgta ttttagaagg tttttattgt taaattatat tttttcggaa 2760
aaattattta tgttttattt ttgtaaattg atatttatat attttgatt ggtattttat 2820
tttagtcgta agattatgat ttatagtaag tttgtttttt tttttgtttg gggtggtagt 2880
agaaagtata gggtattttt tagtttttaa gggtaggggt aaaggggttg gggttttttt 2940
tttttagtat agtttttttt ggttgtgtta tattgttttt tgtgagtaga tagtaagttt 3000
ttttttattt tttattgtta tttatttagc gttgtgtagt agtttagttg cgtgtttgtc 3060
gggaggggtt gttaagtgtt ttgtttattg gttgtttttc gaatttttgt tattttacgt 3120
ataaatatat ttatatattt tttttgttta gtttatatat tgagttattc gtatatgcga 3180
gtatattttt tttttttttt ttattttttc ggtttttgat ttttataagt ttatggaata 3240
tttttggaaa gacgtttttg atttagtagg gtaggtttgt tttgattttt ttttttgtag 3300
ttttagtatt ttgagaaagt aatttatttt tttggttagt gtttgtattt tagtagggag 3360
atgaggattg ttgtttttta tgggggtatg tgtgtgtttt tttttttttt taggatttgt 3420
aggatttttt gtgttatttg tatataattt ggtaggttta tattttttaa gagttttatg 3480
aagtgttttt tgtatgtgtt ttaaaaaggt atttgaaaat tgaaagtgtg atttatggaa 3540
attaaattat ttgtaaaaaa ttgttttgga aagtaatgat tgttggttat aaagggaaat 3600
atttgcgatg tatttaatgt gtttttaatt ttttatttgt tgataattta tagttattaa 3660
tgttaaattc gattttggtt ttagttatat ttgtatattg tttaataatg gtttattttt 3720
gtaagaatta gataaaatgt atatttgata taaaatagtt aaaaatgtaa tttttagtaa 3780
tagtaagttt ggtatttaga tagattatga atatttcgtt agatattttg ttgggtgttt 3840
gggatagtaa ttaaaataaa gtattgatag ttgtattaga gtttattagg ttgtagtaaa 3900
ggaagtttat ttaaaagtat aaattattta agattataga cgtatgatat attttattta 3960
ttttttgttt ttttaatatg tatatatata tatatatata tatatatata tatatatgtg 4020
tgtgtgtatg tgcgtgtgta tgtttaattt ttaatttagt taaaaatttt tttttatttg 4080
ttttttattt ggatatttga ttttgtatat tttagtttaa gtgaatcgag aagatcgagt 4140
tgtaggatta aaggatagat atgtagaaat gtattttaaa aatttgttag ttggattaga 4200
tcgataatgt aatataattg ttaaagtttt ggttcgtgat ttgaggttat gtttggtatg 4260
aaaaggttat attttatatt tagttttttg aagttttggt tgtataatta atttgtggaa 4320
ggtatgaata tttatgtgcg ttttaattaa aggttttttt gaattatttt ttatatgaga 4380
atttttaatg ggattaagta tagtattgtg gtttaatata aatatataag ttaggttgag 4440
agaattttag aaggttgtgg aagggtttat ttattttggg agtattttgt agaggaagaa 4500
attgaggttt tggtaggttg tatttttttg atggtaaaat gtagtttttt ttatatgtat 4560
attttgaatt ttcgtttttt tttttttaga tgttttttgt tagttttttt agttgttaaa 4620
tatagttgtt tgtggttggt tgcgtatgta atcgtatatt ttattttatt tgttttattt 4680
cggttatagt gtagtttttt ttagggttat tttatgtata tattacgtat ttttagttaa 4740

cgaggagggg gaattaaata gaaagagaga taaatagaga tatatcggag tttggtacgg 4800
ggtatataag gtagtatatt agagaaagtc ggtttttgga ttcgtttttc gcgtttattt 4860
taagtttagt tttttttggg ttatttttag tagattttcg tgcgttttcg ttttttggtc 4920
gtgaaattta gtttttattt agtagcgacg ataagtaaag taaagtttag ggaagttgtt 4980
ttttgggatc gttttaaatc gagttgtgtt tggagtgatg tttaagttaa tgttagggta 5040
aggtaatagt ttttggtcgt tttttagtat ttttgtaatg tatatgagtt cgggagatta 5100
gtatttaaag ttggaggttc gggagtttag gagttggcgg agggcgttcg ttttgggatt 5160
gtatttgttt tcgtcgggtc gttcggtttt atcggattcg taggttttcg gggtagggtc 5220
ggggttagag ttcgcgtgtc ggcgggatat gcgttgcgtc gtttttaatt tcgggttgtg 5280
tttttttttt aggtggttcg tcggtttttg agttttttgt tttgcgggga tacggtttgt 5340
attttgttcg cggttacgga ttatgattat gattttttat attaaagtat ttgggatggt 5400
tttattgtat tagatttaag ggaacgagtt ggagtttttg aatcgttcgt agtttaagat 5460
ttttttggag cggtttttgg gcgaggtgta tttggatagt agtaagttcg tcgtgtataa 5520
ttatttcgag ggcgtcgttt acgagtttaa cgtcgcggtc gtcgttaacg cgtaggttta 5580
cggttagatc ggtttttttt acggtttcgg gtttgaggtt gcggcgttcg gttttaacgg 5640
tttggggggt ttttttttat ttaatagcgt gttttcgagt tcgttgatgt tattgtattc 5700
gtcgtcgtag ttgtcgtttt ttttgtagtt ttacggttag taggtgtttt attatttgga 5760
gaacgagttt agcggttata cggtgcgcga ggtcggttcg tcggtatttt ataggtattc 5820
gcgttcgcgt cgttcgtcgg ggtggtcgtc gcgttcggta ggagggaggg agggagggag 5880
ggagaaggga gagtttaggg agttgcggga gtcgcgggac gcgcgattcg agggtgcgcg 5940
tagggagttc ggggcgcgcg gtttagttcg ggggttttgc gtgtagttcg cgttgcgttt 6000
agagttaagt tttttcgtcg ggtagttgaa aaaaacgtat tttttattta tttatcgttc 6060
gtgcgagagg tagattcgaa agttcgggtt ttttaataaa atatacgttg gaaaattaga 6120
taaagtagta gttatttgtg ggggaaaata tttttaggta aataaatacg gggcgttttg 6180
agttatttgg gaaggtttcg tttttggtat ttaaagttgg gggtgtttgg agttagtaga 6240
gtttagtaga gttttattta tttttttaat gtttttgttt aatgtgtttt ttaaattttt 6300
ttttatttag attatttgat tggaaatatg ttagttatga tgatgatttt ttgggaagcg 6360
atttttgtta ttcgtttttt tttttttttt attttacgtt ttggggtttt agagagcgat 6420
tgggagttga atgggtttga tttcggagtt agttggttga gttcgcgttg gagcggattg 6480
ttggtatgtg atttttgata gtcggaaatt tgtaggtgtt tcgcgagttt aaaataagtt 6540
atatggaagt ataagtgttt aaaaataatt ttttgttagt ttagtgataa gtttgtttta 6600
ttcggggaga atgtttcgga gtggcgtgcg ggttagttag ggtttgcgtt tcgtagttat 6660
tgtggaagga gcgcggtcgg tttaggatat aggagattat tttgtgattt taatggcgaa 6720
ggttgtgtgt ttttatttta attttttttt ttataagaat tgtttttttt tttttttttt 6780
ttttttttat ttttttttgt ttagtttttt ttttt 6815

<210> 252
<211> 6815
<212> DNA
<213> Artificial Sequence

<220>
<223> chemically treated genomic DNA (Homo sapiens)

<400> 252

aaaaggagaa attgggtaag agaaaatggg agggagagga gagggagaaa gaataatttt 60
tgtagggaaa aaaattaaaa tgaggatata taattttcgt tattgaagtt ataaagtggt 120
tttttgtgtt ttggatcggt cgcgtttttt ttatagtggt tgcgaggcgt agattttggt 180
tgattcgtac gttatttcgg ggtatttttt tcgggtggga taggtttgtt attgggttgg 240
taggagatta tttttaagta tttgtgtttt tatatggttt gttttaaatt cgcgggatat 300
ttataaattt tcggttgtta gaagttatat gttagtaatt cgttttagcg cggatttagt 360
tagttaattt cgaaattaga tttatttaat ttttaatcgt tttttaaagt tttaggacgt 420
ggggtgggga ggaggggaaa gcgggtgata ggaatcgttt tttagaaagt tattattata 480
gttgatatat ttttaattaa atagtttaga tgaaaggaaa tttggggagt atattaaata 540
aaaatattaa aaggataaat aaaattttgt tgagttttgt taattttaaa tatttttaat 600
tttaaatgtt aagagcgaga tttttttaag tgatttaaag cgtttcgtgt ttatttgttt 660
ggaggtgttt ttttttataa ataattgttg ttttgtttgg ttttttaacg tgtgttttgt 720
taggaagttc gggttttcgg gtttgttttt cgtacggacg gtaagtgggt ggagagtacg 780
ttttttttag ttgttcggcg agagaatttg attttgaacg tagcgcgggt tgtacgtaga 840
attttcgggt tgggtcgcgc gtttcgggtt ttttgcgcgt attttcgggt cgcgcgtttc 900
gcggttttcg tagtttttta ggtttttttt tttttttttt tttttttttt ttttttgtcg 960
ggcgcggcgg ttatttcgac gggcggcgcg ggcgcgggta tttgtagaat gtcggcgggt 1020

cggtttcgcg tatcgtgtag tcgttgggtt cgttttttag gtagtagggt atttgttggt 1080
cgtggggttg taggaaaggc gatagttgcg gcggcgggtg tagtagtatt agcgggttcg 1140
gagatacgtt gttgagtggg gggaaatttt ttaggtcgtt ggagtcgaac gtcgtagttt 1200
tagattcggg gtcgtagggg aggtcggttt gatcgtagat ttgcgcgttg gcggcggtcg 1260
cggcgttgaa ttcgtaggcg gcgttttcgg ggtagttgta tacggcgggt ttgttgttgt 1320
ttaggtatat ttcgtttagg ggtcgtttta gggggatttt gagttgcgga cggtttaggg 1380
gttttagttc gtttttttgg atttgatgta gtagggttat tttagatgtt ttggtgtgga 1440
gggttatggt tatggttcgt ggtcgcgggt agggtgtaga tcgtgttttc gtagggtaga 1500
aggtttagaa atcggcgggt tatttggaaa aagagtatag ttcgaggtta gaggcgacgt 1560
agcgtatgtt tcgtcgatac gcgagttttg gtttcggttt tgtttcggga gtttgcgggt 1620
tcggtgaagt cgggcgattc gacgggagta agtgtagttt taggacgaac gtttttcgtt 1680
agtttttggg ttttcgggtt tttaatttta agtattggtt tttcgagttt atatgtatta 1740
taaaggtgtt ggaggacggt tagggattgt tgttttgttt tgatattggt ttaaatatta 1800
ttttaggtat aattcgattt ggagcgattt taaagagtag tttttttgaa ttttatttta 1860
tttgtcgtcg ttgttggata gaggttgagt tttacggtta gggggcgggg gcgtacgagg 1920
atttgttaaa ggtggtttag ggaagattgg gtttaaaata aacgcgaaag acggatttag 1980
gggtcggttt tttttaatgt gttgttttat gtgtttcgtg ttagatttcg atatattttt 2040
gtttgttttt ttttttgttt gatttttttt tttcgttggt tagaaatacg tagtgtgtat 2100
ataggatgat tttggggagg attatattgt aatcgagata gggtagatag aatggggtgt 2160
gcggttgtat acgtagttag ttatagatag ttatatttag tagttggggg aattgatagg 2220
gggtatttga ggggaagggg gcggagattt agggtatata tataggaaga gttgtatttt 2280
gttattagga gaatgtaatt tgttaggatt ttagtttttt tttttgtaaa atgtttttaa 2340
agtagataga tttttttata attttttgag atttttttag tttgatttgt gtgtttatgt 2400
tggattatag tattgtattt ggttttatta ggaattttta tgtgaaggat gatttagaaa 2460
aatttttggt tagggcgtat atgggtgttt atgtttttta taggttggtt atgtaattaa 2520
aattttagaa aattgaatat aaaatgtgat ttttttatat taaatataat tttaggttac 2580
gaattaaagt tttggtaatt atgttatatt gtcggtttgg tttagttaat agatttttaa 2640
aatgtatttt tgtatgttta ttttttagtt ttataattcg attttttcgg tttatttggg 2700
ttaggatatg tagaattaaa tatttagatg aaaaataaat agaaaaaagt ttttaattga 2760
attaaaagtt aaatatgtat acgtatatat atatatatat atatgtgtat atatatatat 2820
atatatatat atatatatat taaggagata aaaaataggt gaagtatatt atgcgtttat 2880
aattttggat agtttatatt tttgaataaa ttttttttgt tgtagtttaa tagattttga 2940
tataattatt aatattttgt tttaattgtt attttaaata tttaatagag tatttgacga 3000
agtgtttatg gtttatttaa atgttaagtt tattgttatt aagagttata tttttgatta 3060
ttttatatta agtatatatt ttatttaatt ttttataaaa tagattattg ttggataata 3120
tgtaaatgta gttgaagtta aaatcgagtt tagtattaat gattatagat tgttagtaaa 3180
taaagggtta aaaatatatt aggtgtatcg tagatatttt tttttatggt tagtaattat 3240
tattttttaa agtaattttt tatagatgat ttaattttta taaattatat ttttaatttt 3300
taaatgtttt tttaaaatat atgtaaaaag tattttatag ggtttttaaa aaatgtgaat 3360
ttgttaaatt atatgtaaat ggtataaaga attttataag ttttgaaaga aaaaggagat 3420
atatatatat ttttatggag aatagtaatt tttatttttt tgttaggata tagatattag 3480
ttagaaaggt aagttgtttt tttaaaatgt taaagttata gagagagaaa ttaaaataag 3540
tttattttgt tggattaaga acgttttttt agaaatgttt tatgggtttg tagaagttaa 3600
gggtcgagag agtgagaagg aaggaaggaa tgtgttcgta tgtgcgagtg gtttagtgtg 3660
tgaattaggt agagagagtg tgtggatgtg tttgtgcgtg gaatggtagg gattcgggaa 3720
gtagttagta ggtagggtat ttggtagttt ttttcggtag atacgtagtt gggttattgt 3780
atagcgttgg atgaatggta gtggggagtg aggggagatt tgttgtttgt ttatagggag 3840
tagtgtggta tagttagaga aagttgtatt ggggaggaga aattttagtt tttttgtttt 3900
tatttttgga ggttggaaag tattttatgt tttttgttgt tattttaagt aagaggaaaa 3960
ataggtttgt tgtgaattat agttttacgg ttaaaataga atgttagtta aaagtgtatg 4020
gatattaagt ttataaaata ggatatgggt ggttttttcg aaagaatata atttaataat 4080
aaaagttttt tgggatatat gtggattaaa tgttttattg gttttagttt ttagtttttg 4140
aatagaggta ttgttatgtt ttcgattgta ttaggaaatt agattttgga ataaatgttt 4200
tggtatttta gggatgtgtt tttagttgaa atgtaatatt tttttatttc gttaattaaa 4260
tttataaatt tttttatgaa tagtttagtt gattgttttt tgtaaatatg tgaaaaatac 4320
gtattattaa aagtttagga tatgaatata agataaaggt agatattttt gttttaaatt 4380
gattttaggt tttcgagttg tattgatcgt gattgggaac gaggtttttt attttttagt 4440
ggcgtttata tggatttatt ttttgattat ttttttaatt attttttcgg ttatagtatt 4500
aatacgtaat attgaggtgt ttttagagtg tttacgttta gggttttagg atatatgata 4560
tttaatggag gttttgttgt tagatattag ttattatttt ggatattaaa tgattttaat 4620
tataatgtta ggagtggtcg gtttttggtt ttgggatatt atgtagttat tgagagattt 4680
atgagtggtt tttgagatta gtataaaaaa gaaatagaaa gttataaaaa tgttaatgat 4740
gttattatgt aaatatatgt ttttgtgttt tgaaagattt ttagtattgt agtgtttgag 4800

tataggagag ttttttttat agttagtatt gaaaataaat attggatata aataaatatt 4860
gaaaagaaag attgttattt tttgttggtg atagtggtgt tttttgtagg ttaataatgg 4920
ttatttatgt tttagattag ttttagaaaa aagtaagagt atttagggag ggaggagaga 4980
ggaatagggg aaaggagaag gaaaggaaag gggatttgta attgtttatt attgatatag 5040
gaagaataag aaggttagtt gtttttttat aggttttgat tgtttagaga tttataatta 5100
aagttagttt aagaagttta gtaaaggtag tttttttaat tatttttttt ttagtatttt 5160
tttttaaatt tattttggtg agttttgttt ttgggtttgg tgagtatggg tgggaaagta 5220
tattgtgtta cgtcgatttt ttttttttgt ttttggtaaa aattttattc gggtttttag 5280
tttttttggt tttttttttt attttttttt gttggatata tacgttttag tcgtgattta 5340
ttggtttttt taggtgaaga agggtaaaga ttgatttggt tttttcgttg aatgtgtttt 5400
tagttttatt tttttagcgg tttgttgggt attgtaggtt tgtttaaata tgagttatta 5460
tttttaaata tggtgttttt tttaattttt tcgtgtttgt gtttagtatt attgtttttt 5520
tagttattta gattaaaatt ttattgtttt tttcgagggt tgattttttt ttgtttttta 5580
tatttaatta agaggtaatt tttaagtttt ttagttttat aatttttttt tttttattgt 5640
atttcggtta ttttttttga gatttattat tttatttgtt ttaacggttt tttaaggatt 5700
attttgtttt tttttttcgg attttatttt aaattttgta atttgtttat ttttttggat 5760
ttttttttat tgttttagga tttattttga tatgatttag ttttgttttt aatttaaatt 5820
atagtttttt ttatgagttt agggtttttt ttttgtttat aacgattttt tttagtttat 5880
ttttttcgat tgtaatttat gttttagtta agtaaaattt taatatttta agaatttaag 5940
gttttttttt ttttttattt taaattttta tttttttggt ttgttagggg aaagtatgtt 6000
ttttgtaata ggtttagttt aaatgttatt atatttatta tttttttttt tagtttttat 6060
ttaaagaaaa tttttttttt tttgaatttt agtagtagtt attatgtatt tattaaattt 6120
tttttgaatt agagttattt atttgttttt tattttttta tgttgtttat aagttttttg 6180
aggataaggt ttggttagaa ttatttggga attatgagaa tttagtattg tttttggtta 6240
tgttaagtta ataaatgttg gttaatttat tgatatagat atttaaaagg ataatgtaat 6300
attataggtg tttggattat tatttttaaa agttaatttt ttatgtgata ttttattatt 6360
tttttttttt tttgagatag agttttattt ttgttattta ggttggagta tattggtgtg 6420
atttcggttt attgtaattt ttgttttttg ggtttaagcg atttttttgt tttagttttt 6480
ttaagtagtt gggattatag gtgttcgtta ttatatttag ttaatttttt tgtatgttta 6540
gttgagatag gattttatta tgttggttag gttggttttg aatttttgat tataggtgat 6600
ttattcgttt taatttttta aagtgttggg attataggta tgagttatta cgttcggttt 6660
tttaaaattg tttttgttag aagattattt ttgtattttt atgttaattg aggttataaa 6720
gttaaaattt aaattcgtat aattttgagg tttaagtttg taatttttgg tagtatgatt 6780
tataagagag ttttgaattt ttagttcgta agtag 6815

<210> 253
<211> 5527
<212> DNA
<213> Artificial Sequence

<220>
<223> chemically treated genomic DNA (Homo sapiens)

<400> 253

ggttttttgt tgttgttaaa ttaataattt tagtttttgg gtttagttat ttgttgttgt 60
taaaaatgtt gttgagagtg atggagaggt gttttagttt ttattttata gattagaaaa 120
ttaaggttta gagaaattaa gtagttttgg agataggagt tatggttttt ttatttgagt 180
tattattttg aaatattaag gataagttaa ttttattaat agttttaggt ttgtgagaaa 240
tgggtttggt ttattttgaa gattgtaaat aaataaaaaa atagattgtt ttattattga 300
tgagaaatat gagttatttt gttaggaatt gaaagtatag ttgtttataa aaaagttatg 360
tcggggttta gtttttttag gtttttgtgt gtgaaaaatt atttagttaa agtttttttt 420
ttattttttt ttgtgtttag taggatgttg aaatttttgg aaaagataat ggaaattagg 480
ggtaggaaga gaatagtgtg gggtgttaaa tagattttag taattttttt gtttacggtt 540
atttttgcgt agatatttcg attttttatg cgtaggtgga gtagcgtttt ttagtttttt 600
ttagaggttt tttttttgttt gtttttttagt tatatcgttt gagattgata acgttttata 660
gtaagacgga aatttttttt tatagtatta ttttgcgatt ttgtagttat aaagttgaga 720
tttttgaacg tggtattttt ttgttttttt attgttttac gtgtatttgt gttatttttt 780
ttgttttatc gtaaatatat ttttaaaatt tttatttttt ttatttagaa atagaattat 840
aagcggatgg ttaaataatt taaataaatg gtttatgggg aaaagtgaat tttatattgt 900
tttttaaatt tttagtgttt tttatggaga taattattgt ttttaatgtt ttgaatattt 960
ttttagagat attttatttt tatataagta tatgtgtatt tgtgtttttt ttttattttt 1020
tttttataaa tattagtaga ttgttttgta ttttggttag tttatttaat atattttgga 1080

gttaggtgcg gtggtttatg tttgtaattt tagtattttg ggaggtcgag gtgggcggat 1140
tatttgaggt cgggagttcg agattagttt gattaacgtg gagaaatttc gtttttatta 1200
aaaatataaa attagttagg cgtggtggta tatgtttgta attttagtta ttagggaggt 1260
tgaggtagga gaatcgtttg aatttgggag gcggaggttg cggtgagtcg agatcgtgtt 1320
attgtatttt agtttgggta atagaatata tagatttgtt tgattttttt tatgattgtt 1380
atagttttat tgaatgtatg gattattagt gttttagtta gtattttagt gatgggtatt 1440
tagggattat aaattttata agggtagaaa taatatttta tttatttttt gtttttgata 1500
tggtttggat gtgtgttttt gtttaaatta tacgttaagg taatttttta tgttagaggt 1560
ggggtttggt ggaggtgatt ggattatggg gatggatttt ttatgaatgg gttagtatta 1620
tttttttggt gttgtttttg tgatagagtt tttatgagat ttggttgttt aaatgtgtgt 1680
ggtatttttt tatttttttg gttaagtttt tgttatgtaa gatgtttgtt tttgttttgt 1740
tttttgttat gaataaaagt tttttgaggt ttttttagaa gtagatgtta ttatgttttt 1800
tgtatagttt gtggaattgt gagttaatta agtttttttt ttttatgaat tatttagttt 1860
taggtatttt tttgtaatag tgtgaggatg agttaatata gttttttata ttgtaattta 1920
aggtaatgtt ttgtataaag ggatgaatta gattgtttag taattaaaat ataaatataa 1980
attataagta tatttattta tgaattgggg gatagttttt atttgatttt gagttttgat 2040
atttgttgtt ttgttttgtt aaaaatgtgt tagtttttat ttagtttttt attagttata 2100
ttattaatta atatgtataa tatgtataat ttgttaggtt aggaggaaat ttatggatat 2160
aagaaatgtt tttttttttt aggaaatgat aatttgtttt gggagagaag gtaggtattt 2220
tttggtaaaa gtgatataat aaaataatta aaaaaagatg gatttttttt ttttttttga 2280
gatggagtta cgttttgtta tttaggttgg agtgtagtgg cgcgatttcg gtttattgta 2340
atattcgttt tttaggttta agcgattttt ttgttttagg ttttcgagta gttgggatta 2400
taggtattta ttattacgtt cggttaatat tttgtatttt tagtagagac ggggttttat 2460
tgtgttagtt aggatggttt cgattttttg attttgtgat tcgtttgttt cggtttttta 2520
aagtgttggg attataaata tgagttattg tattaggcga aaagatggat tttttaaaaa 2580
aggtgttagg ataattaggt ttttatttgt aagaaagtaa agttagaagt ttattttaga 2640
ttatgtatat tagtataaaa atgttaaatg gtttaaagat aaaatgttaa aataagttta 2700
gaaaggtatt aaaagaaaat atagaaaata tagtattagt aaaaattttt ttatataaga 2760
tgtaaaagtt agaagttata aaggaaaagg ttgataaatt agaatagata aaaatgtaaa 2820
atttttgtat aatataagat ataaagaatg aatttaaaga tatataatga aggttaggaa 2880
gatgaagatg ttttggcgat gagtggtggt tatatattag tattaatgta tttaatgtta 2940
ttgaattgta tatttaaaat gatgaaaatg gtaattttta tgttatgtgt attttattat 3000
ataaaaaaag atatgaaatg aatcggggaa aatatttgtt atataattaa gaatgaaggt 3060
ttttaataaa atttgtaaaa ttatatatat ttttaggaat gaattaataa ataattttta 3120
tgaattagaa aaaagtgata atttaattaa aaaatgaata agggatataa gtaatgtgtt 3180
ttatagaaaa aataaaaatt gataatgaag ttatgaaaaa atgtttagtt tttttagtaa 3240
ttgtataaaa taaattaaaa taatgagata ttatttttaa gattagtaaa tgttaaagaa 3300
aaataataat tggtgagggt gtggggaagt gggtatttat atttatgttt ggaaatataa 3360
attggtgtaa ttttatatagg agagtaattt tataatattt tttaaagatt tatatgtata 3420
attttatggt agagaaattt attttttttt taggattttt ttttttttaa aaatagtgat 3480
gtggatgaaa aatatatgtt tattattgta tagggtataa tagttgaaaa ttggaaacga 3540
taatatttat atttttttta gtaggggaat ggttaaataa attttataag ttatttggta 3600
gatattaggt atgagttaaa agttagggtt tagttagaga tggaaagtat attagtaatt 3660
tgaaagggaa aatgtaatat gaagaattat taattagtaa aagaaggtta attgttaaag 3720
gtataagagt atttaagttg tttgtagtta gtaggtttcg gttggtgagt aggaagttgt 3780
tcgttgggag gttgttaaag ttttttgaag gtgagtatta ttggttttat aagttgttgg 3840
tagttatggc gttaagagta ggaagagaag tattagaatt cggaagagaa atttagtttt 3900
ttgttaggtt ttgtatcgtt tttttggcgt tttttattga taaagttagt aaaattgtgt 3960
cgttagtaaa ggagattttt ttatgggatg tagtttggtg ttattaaaga gaatagagtg 4020
gatttggagt ttagatgtaa tataatgatt gatattggta tagtatattt attttgtttt 4080
tgtaaataaa atggtatatg tgatgttttt ttttgttttt ttgtatataa aataatattt 4140
gtttttattt attatgtatt tatgttttta ttttgtatgt taggagttaa gtattttgta 4200
tgtattaatt tattttgttt ttataataat ttttatatgt aggaattatt atagttattt 4260
tatgaatgag tcgaggaagg tattgagacg ttaagtaatt tgtttaaggt tacgtagtta 4320
gtaagtggta gagtaagaat tattatggtt ttataagttt aggaaaaagt ttgaaagaat 4380
taaaatgtta atagcgggga ttttaaggaa gtattgaaga ggttatggga gaagttttta 4440
ttttgttaaa aaattagttt tttaaataaa taaatatagt gaggtttttt tagaagtaga 4500
tgttattatg ttttttgtat agtttgtgga attgtgagtt agttaaattt ttttttttta 4560
taaattattt agttttaggt atttttttat aatagtgtta ggatgagttg atatagtttt 4620
ttatattgta atttaaggta atgttttgta taaagggatg agttagattg tttagtaatt 4680
aaaacgtaaa tataaattat aagtatattt atttatgaat tggggggttg ttttgtgtgt 4740
atagataagg tatatttttt aaaaaaatta tttttttaag aagaaaataa attagttaat 4800
aaacgataat ttatagtgtt aggaagtgag aaataagtgt gtgataaacg gtggagaatg 4860

ggagtatttt tcgtagtggg cgggaggaga cgaggagggc gtttttgggg gagtggtagt 4920
ggttggagta aaggtttgga ggaggtaagt tatgtgtttt gagtttttgg tttttgtttt 4980
attttgtgtt tgagttggtt tgaaggttgg ttgtttagat tgagtttttt gtttgtttgt 5040
atttcgttaa tagttttaga agaaggtgat tggtggttgt ttgaggaata ttagtgggta 5100
agagaattag tatttttgga gtatttgttg tttgtgagat taagtattat gtatattgtt 5160
ttatttattt tttatagtaa ttttattaag tagttttttt ttacgtgaaa agatggaggt 5220
tgggtggagt aaaaggaggt atttagagtt tttagtaagt gagaggtaga gttgggattt 5280
gaatttagat ttgtttgata ttgaagttta ggttggtttt attttttcgg attgtttttt 5340
agggagtaga agatagatat tttattttag ttggttgttt ttagaagttt gattttgttg 5400
gtttaaaacg attttagttt tttgtttaga ggttgcgggt tgcgggttaa gatattagta 5460
gaaggagggt ttagttagag aggttgatat gggtttttat ttatcgttat tttttttttt 5520
ttttagg 5527

<210> 254
<211> 5527
<212> DNA
<213> Artificial Sequence

<220>
<223> chemically treated genomic DNA (Homo sapiens)

<400> 254

tttggaagga aggagagatg acggtgagta gaagtttatg ttagtttttt tggttgggtt 60
ttttttttat tgatgttttg attcgtagtt cgtagttttt gggtaaggaa ttaaagtcgt 120
tttgagttag tagggttaga tttttagaag tagttagtta aggtaaaata tttgtttttt 180
attttttgga aagtagttcg gagaggtgga attagtttag attttagtat taggtagatt 240
tggatttaaa ttttagtttt gttttttatt tgttgaggat tttaaatatt tttttttgtt 300
ttatttagtt tttatttttt tacgtggaaa agaattgttt ggtaaggttg ttgtggggag 360
tgaataaagt aatatatata gtgtttaatt ttatagatag tagatgtttt agaaatgtta 420
atttttttgt ttattggtat tttttaggta gttattagtt attttttttt gaagttgttg 480
gcggggtata ggtaggtagg aagtttagtt tgaataatta gtttttagat tagtttagat 540
ataaggtgaa atagaaatta aaaatttaga gtatatgatt tatttttttt aaatttttgt 600
tttaattatt gttatttttt agggaacgtt tttttcgttt tttttcgttt attgcggaga 660
gtgtttttat tttttatcgt ttattatata tttgtttttt attttttggt attgtgagtt 720
gtcgtttatt aattggttta tttttttttt ggaaaaataa tttttttaaa aatatatttt 780
tatttatgta tataaagtag ttttttaatt tatgaatgga tatgtttgtg gtttgtattt 840
gcgttttaat tattaagtaa tttggtttat ttttttgtgt aaagtattgt tttaggttat 900
agtgtaggaa attgtattag tttattttag tattgttata aagaaatatt taagattgga 960
taatttataa agaaaagagg tttaattggt ttatagtttt ataggttgta taggaagtat 1020
agtgatattt gtttttgggg aagttttatt gtatttattt atttgaagga ttgatttttt 1080
aataaagtga aaattttttt tatggttttt ttaatgtttt tttgaggttt tcgttgttaa 1140
tattttgatt tttttagatt tttttttagg tttataaagt tatagtaatt tttgttttgt 1200
tatttattag ttgcgtgatt ttgggtaagt tatttaacgt tttagtgttt ttttcggttt 1260
atttataaag tagttataat gattttgtta tgtgaaggtt attatgagaa taaaatgagt 1320
taatatatgt aaaatattta gtttttggta tgtagagtaa agatataaat atataataag 1380
tagaaataaa tattgtttta tatatagaga gataaagaga gatattatat atattatttt 1440
gtttataaaa gtaggtaagt tatattgtgt tagtattaat tattgtgttg tatttgagtt 1500
ttaagtttat tttgtttttt ttggtgatat taagttatat tttataaaaa gatttttttt 1560
gttagcggta taattttatt ggttttgtta gtagagggcg ttagagggac ggtgtaaggt 1620
ttagtagagg attggatttt tttttcgggt tttggtgttt ttttttttgt ttttaacgtt 1680
atgattgtta gtagtttgta gaattagtgg tgtttatttt tagggaattt tggtagtttt 1740
ttagcgggta gttttttgtt tattagtcgg ggtttgttga ttgtagatag tttgagtgtt 1800
tttgtatttt tagtagttag ttttttttta ttagttaata attttttata ttatattttt 1860
ttttttaaat tattggtgtg ttttttattt ttaattggat tttaattttt agtttatgtt 1920
tggtatttat tagatggttt gtaaaattta tttaattatt tttttattga agggaatgtg 1980
agtattatcg tttttagttt ttagttgtta tattttgtgt agtagtgaat atgtgttttt 2040
tatttatatt attgtttttg aaggaaaaaa aattttggaa gaggaataaa tttttttgtt 2100
atagggttgt gtatgtaagt ttttggaaaa tgttgtgaga ttgttttttt tataaggttg 2160
tattaattta tatttttaaa tataggtgta agtgtttatt tttttatatt tttattaatt 2220
attatttttt tttaatattt attaatttta ggggtaatgt tttattgttt tagtttgttt 2280
tgtgtaatta ttaaggagat tgaatatttt tttataattt tattgttaat ttttattttt 2340
tttgtgaaat atattgttta tattttttat ttatttttta gttggattgt tatttttttt 2400

taatttatag aaattatttg ttgatttatt tttaaaagtg tgtatagttt tatagatttt 2460
attaagggtt tttattttta gttatgtagt aaatattttt ttcgatttat tttatgtttt 2520
tttttatgtg atgaaatata tataatataa aaattattat ttttattatt ttaagtgtat 2580
agtttagtaa tattaagtat attaatattg atgtgtaatt attatttatc gttaaaatat 2640
ttttattttt ttaattttta ttgtatgttt ttaaatttat tttttgtgtt ttatattata 2700
taaaagtttt atatttttat ttattttaat ttattaattt ttttttttat gatttttggt 2760
ttttatattt tgtgtaggga aatttttgtt ggtgttgtat tttttatatt tttttttaat 2820
atttttttag atttgtttta atattttatt tttaagttat ttgatatttt tgtgttggta 2880
tgtatggttt gaggtgggtt ttttaattta tttttttgta ggtggggatt tgattatttt 2940
aatatttttt ttaaaaaatt tattttttcg tttggtgtag tggtttatgt ttgtaatttt 3000
agtattttgg gaggtcgaag taggcggatt ataaggttag gagatcgaga ttattttggt 3060
taatatagtg aaatttcgtt tttattaaaa atataaaata ttagtcgggc gtggtggtgg 3120
gtgtttgtag ttttagttat tcgggagttt gagataggag aatcgtttga atttgggagg 3180
cggatgttgt agtgagtcga gatcgcgtta ttgtatttta gtttgggtga tagagcgtga 3240
ttttatttta aaaaagaaaa aaagatttat ttttttttga ttattttatt atgttatttt 3300
tgttaaaaag tatttgtttt tttttttaaa gtagattatt attttttaga agggagggat 3360
attttttgta tttataagtt tttttttgat ttggtagatt gtatatgtta tatatgttgg 3420
ttggtgatat gattgatagg aagttgaatg agaattagta tatttttaat aaagtaaagt 3480
agtaggtatt agaatttaga attaaataaa agttgttttt taatttatga atggatatgt 3540
ttgtggtttg tatttgtgtt ttaattatta agtaatttgg tttatttttt tgtgtaaagt 3600
attgttttag gttatagtgt aggaaattgt attagtttat ttttatattg ttataaagaa 3660
atatttaaga ttggataatt tataaggaaa agaggtttaa ttggtttata gttttatagg 3720
ttgtatagga agtatagtgg tatttgtttt tggggaagtt ttagagagtt tttatttatg 3780
gtagaaggta aagtaagggt aagtatttta tatggtagga atttgattaa gagagtgggg 3840
aggtgttata tatatttaaa taattagatt ttatgagagt tttattataa gaatagtatt 3900
aaagggatgg tgttaattta tttatgaagg atttattttt atgatttaat tatttttatt 3960
aggttttatt tttgatatgg gggattattt tgacgtgtga tttgggtagg gatatatatt 4020
taaattatat tagaaataga ggatgggtaa gatattgttt ttgtttttat gaagtttgtg 4080
atttttaaat gtttattatt aaggtgttag ttggagtatt gatgatttat atatttaatg 4140
gagttgtagt agttatgaaa aggattaagt agatttatat gttttgttgt ttaggttgga 4200
gtgtaatggt acgatttcgg tttatcgtaa ttttcgtttt ttaggtttaa gcgatttttt 4260
tgttttagtt tttttagtag ttgagattat aggtatgtgt tattacgttt ggttaatttt 4320
gtatttttag tagagacggg gtttttttac gttggttagg ttggtttcga attttcgatt 4380
ttaggtgatt cgtttatttc ggttttttaa agtgttggga ttataggtat gagttatcgt 4440
atttggtttt aagatatgtt aaatgagtta attaaggtgt aaaatagttt gttagtgttt 4500
gtggggggaa aatgagggaa ggatataaat atatatatgt ttatataaga ataaaatatt 4560
tttgaaagaa tatttaaaat attagaaata ataattattt ttataaaagg tattgaaagt 4620
ttgggggata gtgtgaaatt tatttttttt tatggattat ttgtttaaat tgtttaatta 4680
ttcgtttgta gttttatttt taaataaaga aaatgggaat tttagaagta tgtttacgat 4740
gaaataagaa agatgatata ggtatacgtg aaatagtaag ggaatagaag agtgttacgt 4800
ttagaagttt taattttgtg gttgtagaat cgtaaggtgg tgttgtaaag ggaggttttc 4860
gttttgttgt agggcgttgt taattttagg cgatgtggtt ggaaggtagg tagaaggaag 4920
tttttggaaa gaattaggaa acgttgtttt atttgcgtat ggggggtcga ggtgtttgcg 4980
tagagatggt cgtgggtagg gaggttgttg agatttgttt agtattttat attgtttttt 5040
ttttgttttt gatttttatt gtttttttta gaaattttag tattttattg agtataaaga 5100
gaaataaaag gggaattttg attgagtggt tttttatata taaggatttg gagaggttgg 5160
atttcggtat agttttttta tgggtagttg tatttttaat ttttggtaga atgatttata 5220
ttttttatta gtaatggagt agtttatttt tttatttgtt tataattttt aagataaatt 5280
aagtttattt tttataggtt taaaattgtt gataaagttg gtttgttttt gatgttttaa 5340
gatgataatt taagtggaga ggttatggtt tttattttta agattattta gtttttttgg 5400
gttttgattt tttgatttgt aaaatgggaa ttaaaatatt tttttattat ttttagtaat 5460
atttttagta gtagtaggtg gttaaattta agggttggag ttgttaattt ggtagtagta 5520
agaagtt 5527

<210> 255
<211> 10224
<212> DNA
<213> Artificial Sequence

<220>
<223> chemically treated genomic DNA (Homo sapiens)

<400> 255

agtttttata gttttagtat cgtttttttt attttatttc gattgcgttt ttgttgttta 60
tttttgttta tttattttaa ttttagtgtt ttttttagtt tttgggtttt aggtgatata 120
ttagttagtg ggatatgggt ttttataggt atttttagtt taatttagtt tttttttttt 180
ttttggtttt ttggttagta tttgtattat attggttttt attggatatt tttgtagttt 240
cgggtttttt ttatagatat attgttggat tagttgttta atttggttta tttgtagagt 300
tttttggttt atttgatttt aattgaaatt tagtttttta agaaggattt ggtgttggaa 360
taggtaggta ttggaaagtt agttgtttag gattattgtt ttattttatt agtatttttt 420
tgttattttt tatttttttt ttttagattt tagaaatttg ggagcggttg gagcgagaaa 480
atagaggtaa gtggtaggta attgttaagt attagtttta gtatgtgttt agttttttag 540
agtaggattt gcggttgtag gcgcgaaggt aaggtttgtg gaaatggtag ggagggtgga 600
ggggatgtag gaggtatgga tgtgggtggg gtgtttttat tttttagggt tagttagatt 660
tttttgattt ttttttaggt gggttgagat ttataggttg gatgtgttag aggtagtggt 720
tttagagcgg tttcgttgtg tttattgtag tgtagaggtt tttaagcgtt gtttacgatg 780
ttagaatgag tggtattgtt gtaggtgagg gtattttaga attttggatt tttaagtttt 840
atttttatat tttttatatg tattgttatt tttaatattt atttgtttgt agggagtgtt 900
aagttaagta ttgggaaaag tatggaaaga tttgtgtttt ggtagtttag ggtgatagag 960
ttaaatgagg gttgtagttg ttgagggtcg attatttatg ttaagggaat ttatttagaa 1020
tgtatttttg aattttaaga ttacggttta gtttttgtcg gagttttagt tttcgtagtg 1080
gagagtagag cgggcggtaa agttgttgat cgattttttt tttttttatt ttaagtgaag 1140
gttcgagatt ttttgtttta tttagtgggt aggttaagtg tgttgtttta gtaaatcgga 1200
ttaggagggt tagggtcgga tgtggggatt tttttttttt agtatagtaa agttggtttt 1260
tagaaatacg ggtattttcg cgtggtgttt tgcggtcgtc gtcgttgtgg tcgttcgggg 1320
tggggtgtga ggaggggacg aaggagggaa ggaagggtaa ggcggggggg gttttgcgag 1380
agcgcgttta gtttcgtttt cgggttttat agtttttgta tttaggtttt tattgcgcgg 1440
ttttttttag ttttttttcg tcgtttagtt tggattttgg gggaggcgtt gaagtcgggg 1500
ttcgttttgt ggtttcgttc ggttcgcgtt tgttagcgtt taaagttagc gaagtacggg 1560
tttaatcggg ttatgtcggg ggagtttgag tttattgagt tgcgggagtt ggtattcgtt 1620
gggcgcgttg ggaagggtcg tattcggttg gagcgtgtta acgcgttgcg tatcgcgcgg 1680
ggtatcgcgt gtaattttat acggtagttg gtttttggtc gtggttatcg tttttagttc 1740
gcggggttcg ttacgtatac gtggtgcgat ttttgtggcg attttatttg gggcgtcgtg 1800
cgtaaaggtt tgtagtgcgc gcgtgagtag tggtttcgcg cgtttacgag agcggaaggg 1860
gtagttaagg ggtagcgtag tcgtcgcggg ttaagtcgcg gtagaggggg tcggcgggga 1920
tagttttcga ggattaggtt cgttattttc gttttatcgt tgaagagtgc gcgaaaatgg 1980
tttatttttt gtcgtatttt attcgtattt gggttataga tgagtagagg tggttgttta 2040
tatgtaaaaa tacgttgatt ttaagttttt tatttttaaa atgttttggt tttttttgag 2100
aaagggtttg tgtttattgt tttcggagtt tattttttta ggtttgtttt tttttaaata 2160
tttatgattt tttttagaat ttttagggtg aagggaaatt attatttatg ggagggagtt 2220
tggaaaaatt tagaattttt ggtgggtttt ttgtaagtag gagttttgtt gagtttttat 2280
ttagtaaata tttttttttg atttagtgaa ttagatgtta aaatatgtac gtagttatat 2340
atttagtagt ttttttgtat ttttgggaat cgttagtaag taaaggttgt ttttttttgg 2400
gtagatatta gttggaatta ttaggggtgt ttttatagtt tttttcgtta gtttggattt 2460
tatcgtagat ttgttgaatt aattgttggg agtggatttt aggtattagt aaattttaaa 2520
aattttttaa attattgtaa tatggagttt gggttgagta ttattgtttt ggtttattta 2580
ggaatttgtg gatggatagt gttttaggtt tgtgtgtgta tggagatttt tttattcggt 2640
ataagaggat attataaatt tagttggggg gagtataaag ttgtgataga atgtaaagaa 2700
tgaataaggg gtcgagcgcg gtggtttatg tttgtaattt tagtatttcg gaaggcggag 2760
gcgggtggat tatttgaggt taggagttta agattagttt ggttaatatg gtgaaatttt 2820
atgtttatta aaaaataaaa aaaaatgagt taggcgtagt ggcgggtgtt tgtaatttta 2880
gttattcggg aggttgaggt gggagaattg tttgaatata ggaggcggag gttgtagtga 2940
gtcgagatcg tgttattgtt ttttagtttt ggcgatagag tgagattttg ttttaaaaaa 3000
aaaaaaaaaa aaaaaaagaa taaggttggg atattgtagc gtttttaaag agaaataaag 3060
tagttatgga gataagaagt aggatgattt gggtatgttt attagaggta gagataaggg 3120
agaaattaaa gataagtttg ggtttttgtt tttagtaatt gggagtttag tggttatttt 3180
tgttgtaaag aggaagttgg gtaagtgtag tagtgaggtt gaagaaaagg gaattaaatt 3240
ttggttatgt ttatttgaaa cgttttttag atattttagt gaaggtattg gtacggagga 3300
tttagtttga gggtttaggt tagtgtttta gtcgtggatt tggggtagat gaatgtagat 3360
agattaggtt agtgattagg attgagttta gattttatcg tgagatatgg aagttgagtt 3420
agaatttgta aaggagttga gtaggagttg tagggggtag gaggaaaatt gggagagtgt 3480
agtttttggg agttaaaggg agtaagtttt aaatgatgtt gagggggtga gaatggagaa 3540
tggaatattg gattttattt ggtagtatat agatcgttga ggattttgtt tcgggtagtt 3600
ttttggagga agaggtaagt ttggttggag tgggtagagg ggagagtgaa ggcgaaggat 3660
tagagtgtat agagattagt gttttggttt gaggggagta gagataggtg ataattatag 3720

ggtagacgta ggttaaaggt gtttagtttt ttttttaagt aaatgggtag atgtatttta 3780
tatacgtttt tagtgaaggg tcgggtgcgg tggtttaagt ttgtagtttt agtattttgg 3840
aaggtcgagg cgggtggatt atttgagatt aggagtttga gattagtttg gttaatatgg 3900
tgaaatttcg tttttattaa aaatataaaa attagttggg tatggtggcg ggcgtttgta 3960
attttaggta tttaggaggt tgaggtagaa gaatcgtttg aatttaggag gcggaggttg 4020
cggtgagtcg aaatcgcgtt attgtatttt agtttgggtg ataaaagtaa gacgtagttt 4080
tttgttgttg tttttttaat tgttaatgag gaaaggggaa gttttgtgtt aggcgataga 4140
gatttaattg ttgagtaggt ttttttgttt gtggtttttc ggtcggtttt tagacgttta 4200
ggtggttaat attagagttc gcgtagtagt gtgaggtaat ttattgagat aggtcgggtt 4260
tgcggagttt ggcgagtagc ggtttttttt ttggggtttt tttttaattt tcgggatatt 4320
ttttcgattt ggagtttttt cgttttatcg ttaggttttt ttgtagattg taagtttatt 4380
tgttattatc gttgtcgcgc gttcgtttgt ttggattgtt gcgggtttcg ggatttgggt 4440
tgggaattcg cggtggagcg ggatacgaac gtggtgagcg cggggtcgag ggcgtatggg 4500
aagggcgagg atgggtaggt tatagtgtag gtattttcga gggttgtttg ggtgtcgcgc 4560
gtaaggagcg ttttaattgt cgatttttcg gcggtataga gaggttaatt ttgcgcgggg 4620
gttgggaggg gagtttggat tgtcggtttc gtaagtattt tattcgttgt aagcggattc 4680
gggtttaggt tgatttaggt ttcgcgtacg cgtatttttc gtattttttc gttttcgttt 4740
tcggttagag gttatttttg tgcgtttgtt cggacgttgg tattcgtttt cgttttttgt 4800
ggtaggtggg gtttgtgagt ggagtttcgg agcgatgagg ttatttttgg gggcgaagcg 4860
tgcgtgtttt cgtttcggcg tttttgtttt aatgagataa gagttagatt tcggcgattt 4920
acgttttagt tttaacggtt gcggcgcggt tttggttcgg gcgtacgcgt atattgatac 4980
gcgtatacgt acgtacgcga tcggggcggt ggttggcggt tacggacgcg taggattggg 5040
ggacgggcgg gtacggttat gggcgaggcg gaggcgtttt ttttcgaaat gatttggagt 5100
agtacgacga gtagtggtta ttgtagttaa gaggattcgg attcggagtt cgagtagtat 5160
tttatcgcgc gaatttcgtt agttcgtagg tcgcgtcggg attaggtggg agttaggggg 5220
tgtcggcggg cgggagggga agcggtcgtt ggagtttcgt ttttttcggt tcgttgtcgc 5280
gttttgggtc ggtgggtagt tttatttttt tggttacgtg gtttttcgcg ggttttggtc 5340
ggggatttgt tcgcggaatc gtgcgtaaga tttcgatttt atcgtttaga tgttgggtgt 5400
cggggttttt ttggtttttg ttatagatag gttgaatacg gaaaaagtag ttgtatggtt 5460
tgtggtagat ttgagtcggg tattatttag ttatgattaa agtcgatcga gtagtttgga 5520
ttagtatttc gattttcgcg ttcgaatgtt tttgtttttt ttttggggag attaggggag 5580
gatgtggaga gggaagagtt ttcgttagga attgagaagt atgtttagga aaatttgaga 5640
ggtagagaga gattttgttt ttttatttgt atttttgtat ggagttagtt gagtttttat 5700
tttttttttg ttttggtttg ttattagttg ttggaatgtg gaagattttg tttttttttt 5760
ttagggtgga tttggagaaa gatttgggaa tagataggaa agaagttttg ttttggatta 5820
taagtattta ggagtatttt atttatagga agggggaaag ttagattata aaatgtttaa 5880
agaggtggaa aaagagattt aggttattaa tttaggattg taaggtgttt cggaattttt 5940
taggtatttt tattatcgga gaattgtgtg ttagatgtta ttggtgtgat tattaggttt 6000
agagaattag gtttaggtat taggaaaaag aaatagggat tgtgaagttt agtatgtttg 6060
gtagaaatgg ggcggaaatt tttatttaag taaagaaagt ggagttgtga gtgatgtttt 6120
agataaaatt ttataaaatt ttttataaaa tgggtggtgt ttagtacgtt aaaattttag 6180
tttagagttt gggtgtaagg gttgagttga gtgtagattt ttgggtttgt ttttatgtta 6240
gttagttttg agttattttt tattgtggaa aggtgggaaa attataagat attaattaat 6300
tgaaaaggag ggttagttac ggaggtgtat atttgtaatt ttagttattt gggagggtga 6360
ggtagaagga ttatttgaat ttgggaggta gaggttgtag tgagttaaga tcgtgttatt 6420
gtattttagt ttgagtgata gagtgagatt ttgttttaaa aatagaaaag gaagttaagt 6480
acggtggttt atatttttaa tgttaatgtt ttgggaggtt aaggtaggtg gattatttgt 6540
aattaggaat tcgaggttag tttggttaat atggtgaaat tttattttta ttaaatatat 6600
aaaaattagt cgggtatggt ggtgtgtgat tgtagtttta gttatttggg agattgaatt 6660
attttaatcg ggaggtaaag gttgtagtga gttaagatcg tgttattgta ttttaatttg 6720
ggtgataggg tgaggttttg ttttaaaaaa aagaaagaag gttgggtttg gtgatttatg 6780
tttgtaattt tagtattttg ggaggttaag gtaggtagat tatttgaggt taagagttcg 6840
agatttgtta ggttaatata gtaaaatttc gtttgtattg aaaatataaa aaaattattt 6900
ggttatggtg gtgtgtgttt gtaattttag ttattgggga ggttgaggta ggagtattat 6960
ttgaatttag aagatagagg ttgtagtgag tcgagattgg gttattgtat tttagtttgg 7020
atgagagagt aagattttgt ttttaaaaaa aaaaaaaaaa aaaagaaaga ataggaggtt 7080
gagaagtttt aagttatatg ttaaaaaaaa agaaaaaaat attagtttta ggttaggtgt 7140
agtggtttat atttttaatt ttagtatttt ggaaagtcga ggtgggtgga ttatgaggtt 7200
aggagtttaa gattagtttg gttaaaatgg tgaaatttcg tttcgattaa aaatataaaa 7260
aattagttag ttgtggtggt aggtatttgt aattttagtt atttgggagg ttgaagtaga 7320
gaattgtttg aatttaggag gtagagattg taatgagtta agatcgtatt attgtatttt 7380
agtttggaaa atagagcgag attttgtttt aaaaaaaaaa ttattagttt ttatggatag 7440
tggtagagtg gagggtgggt ttttatggtg tagaaggaaa atttatggtt tttgttgtgt 7500

attcgattgg gatggttgtt gaaatttttt tttagtaggt agttttggaa atagaaaaag 7560
aaattttttt ttttttagaa ttttggaagg gttgtgtagt gtttttaatt taagtttgtt 7620
ttttgagtga agatagggag gtttattatt agaagggaag gggttggaaa tgaggttatt 7680
gtattttagt ttagggtttt tgggttattt aggaagggaa gaaggagtaa gtttttttat 7740
tgttaggtag gagtttagag ttattataag aataagttag tattattttt gtgttttttt 7800
tgttttgtaa ataaaatgat tttttttttt gttttggtat tagagtttgt ttggtatttt 7860
ttttgttttt agtatttttt ttatttgggt attttttttc gttggtgtat tgaataaata 7920
tatttattgt tttatttata gtttttagtt tttatttttt agggtttata ttatttgttt 7980
ttattaattc gataaggttg tttattgttt ttagtaaggt ttgtattggg gtttttattt 8040
tagtgttttt ttttatttag gagatttttg gatatttggg gaagaaaatg agtttaaatt 8100
tttatttttt tttttttatt ttttttttgt aaggttttgg ttttagtttt tagttttata 8160
tttttgttgg ttgtagaata gtagcgggtt ttgggtaagg agtattttgt taaaacgttt 8220
tattttgttt ttttatttgt tttttttatt tgtttttatt agatggttta agtgtttaag 8280
gggattttag ggcggagtta gggagaattt tggttttttt gggttaggta taagattatt 8340
ttataggaaa ttttgtggga atttttttgg gataaagtat tggttagcgt tgagtttagt 8400
tgtgtttgtg atattcgtat tttaattagg gtttatttga cgttaatagg aagtaaggtt 8460
gatgtagtgg ggttaaggga gtttgggaga agaaagtcgg tttagagttt tggttgtttt 8520
gttttatatt ttattttttc ggtaagaatt tagtttttag atgaggtggg gagtgagtgg 8580
tcgagttaaa aatttttggg tcgggtacga tggtttacgt ttgtaatttt agtattttgg 8640
gaggtgaagg taggcggatt atttgaggtt aggagtttaa gattaatttg gttaatgtgg 8700
tgaaatttta tttttattaa aaatataaaa attagtcggg tgttgttgtg gtacgcgttt 8760
gtagttttag ttattcggga gtttgaggta ggagaatcgt ttgaatttag gaggtagaat 8820
ttgtagtgag ttaagattta gttattgtat tatagtttgg gcgatagagt gaggtttcgt 8880
tttaaaaaaa aaaaaaattt ttgggttaaa tttttagata gtataggtag gtgtagaaat 8940
ttattaggaa gttgtttgtg tatttttggt agattggagt ttggtttaaa gttgtttttt 9000
atgtagtttg ggttaaggtt aaatattatg ttatagtgat tttttttatt atgtgtgaga 9060
tatggagaat tggttttaag tattattttg tttattggtg gttggattat tgatgtgtat 9120
tattttttat tttttttatt ttgtagtggg ttatggtttc gtgtcggggt agaggagaaa 9180
aatgggttgt tttttttagg ataaattttt attttaattt aattagggtg ttgtgattag 9240
aatgtgtaat tgaggtgtga ttttattgat tttttttttt tttgagatcg agtttcgttt 9300
ttgttgttta ggttggagtg cgatggtacg attttagttt attgtaattt ttatttttcg 9360
agtttgagta atttttttgt tttagttttt taagtagttg ggattatagg tatgtgttat 9420
tacgtttggt taattttgta tttttagtag agacggggtt tttttatgtt ggttaggttg 9480
gttttaaatt tttgatttta ggtgatttat tcgtttcggt tttttaaagt gttagaatta 9540
taggcgtgag ttaacgtgtt tagtttgttt ttgttttttg tgttttgaag tagggtttta 9600
tttagttttt taggttggag tgtagtgata cgataatagt ttattgtagt tgtaattttt 9660
cgggtttaaa cgattttttt attttagttt tttgaatagt tgggattata ggtatattat 9720
tatatttggt taattttttt tttttttttt ttagtagaga tgaggttttg ttatgttgtt 9780
taagttggtt ttaaattttt gaggattaag tgattttttt attttagttt tttaaaatgt 9840
tgggattgta gatgtgagtt attatattta gtttgatttt attttaaatg agagtttttt 9900
tttagagttt tttagttgtt tttggttttt ggttatgtgt ttttagttgt ttttgttttt 9960
gtggtatttt taaggttata tttagtgttg aggttttagg taggtagtag agagaagtta 10020
aatgattttg tttttttttt atttatttag agtatgtaaa attaggagta gtggtgggtt 10080
tagggtgggt attagttatg tatatgtata ttagggatag ggggttaaag gtagttagtt 10140
tttaaagatt gttttagagg ttatttttta gagaagtttt gggtttttta agggttttgt 10200
gtttatgttg gtttattttg tagg 10224

<210> 256
<211> 10224
<212> DNA
<213> Artificial Sequence

<220>
<223> chemically treated genomic DNA (Homo sapiens)

<400> 256

tttgtaagat gggttagtat ggatataggg tttttgagga atttagggtt tttttgaaaa 60
atggtttttg gggtagtttt tggaaattga ttgtttttgg ttttttgttt ttgatgtata 120
tatatatagt tggtgtttat tttgaattta ttattgtttt tggttttgta tgttttgggt 180
ggataaggga aagatagaat tatttggttt ttttttgttg tttgtttagg gttttagtat 240
tgaatgtagt tttaaggata ttatagaagt aggggtaatt gaaggtatat ggttaggggt 300
taggaatagt tgaggatttt tgaagaggga tttttattta aagtaaaatt aggttgggtg 360

tggtggttta tatttgtaat tttagtattt tgggaggtta aggtaggagg attatttgat 420
ttttaggagt ttgagattag tttgggtaat atagtaagat tttattttta ttaaaaaaag 480
aaaaaaaaaa attagttagg tgtggtggtg tgtttgtagt tttaattgtt taggaggttg 540
aggtgggagg atcgtttgag ttcgggagat tgtagttata gtaagttatt atcgtgttat 600
tgtattttag tttggggaat tgagtgagat tttgttttaa aatataaaaa ataaaaatag 660
gttgggtacg ttggtttacg tttgtaattt tagtattttg ggaggtcgag gcgggtggat 720
tatttgaggt taggagtttg agattagttt gattaatatg gagaaatttc gtttttatta 780
aaaatataaa attagttagg cgtggtggta tatgtttgta attttagtta tttaggaggt 840
tgaggtagga gaattgttta aattcgggag gtggaggttg tagtgaattg agatcgtgtt 900
atcgtatttt agtttgggta ataagagcga aattcggttt taaaaaaaaa aaaaattagt 960
aaaattatat tttaattgta tattttgatt atagtatttt agttgagttg gagtgagggt 1020
ttgttttgga gaaggtagtt tatttttttt ttttgtttcg gtacggggtt atgatttatt 1080
gtagggtgag aggagtggag agtggtgtat attagtagtt tagttattag tggatagagt 1140
agtatttgga gttagttttt tatgttttat atatagtgag aaaaattatt gtgatatgat 1200
gtttaatttt gatttaagtt gtataaaagg tagttttagg ttaggtttta atttgttaga 1260
ggtatatagg tagttttttg gtgggttttt gtatttgttt gtgttgtttg gagatttggt 1320
ttaaagattt tttttttttt tgagacgaag ttttattttg tcgtttaggt tgtagtgtag 1380
tggttggatt ttggtttatt gtaagttttg ttttttgggt ttaagcgatt tttttgtttt 1440
agattttcga gtagttggga ttataggcgc gtgttataat aatattcggt taatttttgt 1500
atttttagta gagatgggat tttattatat tggttaggtt ggttttgaat ttttgatttt 1560
aagtgattcg tttgttttta ttttttaaag tgttgggatt ataggcgtga attatcgtat 1620
tcgatttaga gatttttaat tcgattattt atttttattt ttatttaggg attggatttt 1680
tgtcggaagg gtggagtgtg ggatagggta gttagggttt tgaatcgatt tttttttttt 1740
agattttttt ggttttattg tattagtttt attttttgtt gacgttagat aggttttagt 1800
tagaatgcga gtgttataga tatagttaag tttagcgttg attaatattt tgttttagaa 1860
gaatttttat aaggtttttt gtagaatgat tttgtgttta gtttaggaga gttagggttt 1920
tttttgattt cgttttggag tttttttaag tatttaaatt atttgatggg gataaatgga 1980
gaggatagat gagggagtag ggtggagcgt tttagtagaa tgttttttat ttagaattcg 2040
ttgttatttt gtagttagta aggatgtggg gttaagaatt aaggttaggg ttttatagga 2100
aaaaggtaaa gggggagggg tggaatttta agtttatttt tttttttaag tatttaaagg 2160
ttttttggat ggagaagagt attggagtaa aaattttagt ataaatttta ttggggatag 2220
tgggtaattt tgtcgggtta gtaaaaataa atggtgtggg ttttggaaaa tgagggttgg 2280
aggttgtgaa taaagtagtg gatgtgtttg tttagtatat taacgggaag aagtatttag 2340
atgggaggag tattaggggt aggagaaatg ttagatagat tttagtgtta gggtaagaag 2400
gaagattatt ttgtttgtag aatagggagg gtatagggat ggtgttaatt tgtttttgtg 2460
atggttttga gtttttattt aataatgaga aagtttgttt tttttttttt ttttggatga 2520
tttaggagtt ttgggttggg atgtagtgat tttattttta gttttttttt ttttggtgat 2580
gaattttttt atttttattt agaaaataga tttggattag aggtattgta tagttttttt 2640
aggattttaa aggaggaaga gttttttttt ttgtttttaa agttgtttgt tggaagagga 2700
ttttaatagt tattttagtc ggatgtatag taggattatg gaattttttt tttgtattat 2760
agggatttat tttttatttt attattgttt ataaaaattg atggtttttt ttttgagata 2820
gagtttcgtt ttgtttttta ggttggagtg tagtggtgcg attttggttt attgtaattt 2880
ttgttttttg ggtttaagta attttttgtt ttagtttttt aagtagttgg gattataggt 2940
gtttgttatt ataattggtt aattttttgt atttttagtc gagacggggt tttattattt 3000
tggttaggtt ggttttgaat ttttgatttt atgatttatt tatttcggtt ttttaaagtg 3060
ttgggattaa aggtgtgagt tattgtattt ggtttaaaat tgatgttttt tttttttttt 3120
ttaatatata atttgggatt ttttagtttt ttattttttt tttttttttt tttttttttt 3180
tgagatagag ttttgttttt ttatttaggt tggaatgtag tggtttagtt tcgatttatt 3240
gtaatttttg ttttttgggt ttaagtgata tttttgtttt agttttttta gtagttggga 3300
ttataggtat atattattat ggttagataa tttttttgta tttttagtat agacggggtt 3360
ttgttatgtt ggtttggtag gtttcgaatt tttggtttta agtgatttgt ttgttttggt 3420
tttttaaaat gttgagatta taggtatgag ttattaagtt tagttttttt tttttttttt 3480
gagatagagt tttattttgt tatttaggtt ggagtgtagt ggtacgattt tggtttattg 3540
taatttttgt ttttcggttg aagtgattta gttttttaag tagttgggat tatagttata 3600
tattattatg ttcggttaat ttttgtatgt ttagtagaga tagggtttta ttatgttggt 3660
taggttgatt tcgaattttt gattgtaaat gatttatttg ttttggtttt ttaaagtatt 3720
ggtattagag gtgtgagtta tcgtatttgg tttttttttt tatttttgag atagagtttt 3780
attttgttat ttaggttgga gtgtagtggt acgattttgg tttattgtaa tttttgtttt 3840
ttaggtttaa gtgatttttt tgttttattt ttttaagtag ttgggattat aggtgtgtat 3900
tttcgtggtt agtttttttt tttaattggt tagtgttttg tggttttttt atttttttat 3960
agtggaaaat ggtttaggat tgattgatat gaagataagt ttaggggttt atatttaatt 4020
taatttttgt atttaagttt tgggttaaga ttttggcgtg ttgagtatta tttattttgt 4080
aaggaatttt gtaaaatttt atttgaagta ttatttataa ttttattttt tttatttaaa 4140

taaggatttt cgttttattt ttgttaggta tattgagttt tatagttttt gttttttttt 4200
tttggtgttt aggtttggtt ttttgagttt ggtggttata ttaatggtat ttggtatata 4260
gtttttcgat aatggggata tttaggaggt ttcgagatat tttatagttt tgggttagta 4320
atttggattt tttttttat tttttaggt attttataat ttagtttttt tttttttgt 4380
gggtaaagtg tttttgaatg tttatggttt aaaataagat ttttttttta tttattttta 4440
aatttttttt tagatttatt ttagaggaag ggaatagaat tttttatatt ttagtagttg 4500
gtgataggtt agaataggga agaggtgagg gtttagttgg ttttatatag gagtgtagat 4560
ggaggagtag gatttttttt tgttttttaa gtttttttaa atatattttt taatttttgg 4620
cgaggatttt ttttttttta tatttttttt tagttttttt aaggagggag taggagtatt 4680
cgaacgcgga aatcgaggtg ttagtttaaa ttgttcggtc ggttttagtt atagttggat 4740
aatgttcggt ttaggtttat tataagttat atagttgttt ttttcgtgtt taatttgttt 4800
gtgatagaaa ttaagggggt ttcggtattt agtatttagg cggtggaatc ggggttttac 4860
gtacggtttc gcgggtaggt tttcggttag gattcgcggg gagttacgta gttaggaggg 4920
tggggttgtt tatcgattta ggacgcggta acggatcggg gagggcggag ttttagcgat 4980
cgtttttttt ttcgttcgtc ggtatttttt ggttttattt tggtttcggc gcggtttgcg 5040
agttagcgag gttcgcgcgg tgaagtattg ttcgagtttc gagttcgagt ttttttggtt 5100
gtagtagtta ttgttcgtcg tgttgtttta ggttatttcg aaagaaggcg ttttcgtttc 5160
gtttatagtc gtattcgttc gttttttagt tttgcgcgtt cgtagtcgtt aattatcgtt 5220
tcggtcgcgt gcgtgcgtgt acgcgtgtta gtgtgcgcgt gcgttcgggt tagagtcgcg 5280
tcgtaatcgt taagattgaa acgtagatcg tcgggattta gtttttgttt tattggggta 5340
ggaacgtcgg ggcggggata cgtacgtttc gtttttagga atgattttat cgtttcggag 5400
ttttatttat agattttatt tattataggg aacgggggcg ggtgttagcg ttcgggtaag 5460
cgtataagag tggtttttgg tcggaggcga gggcgggaag gtgcgggaag tgcgcgtgcg 5520
cggagtttgg gttagtttgg gttcgggttc gtttgtagcg ggtggagtat ttgcggagtc 5580
ggtaatttag gttttttttt tagttttcgc gtagaattag tttttttgtg tcgtcgggaa 5640
atcggtaatt agaacgtttt ttgcgcgcgg tatttaggta gttttcgaga atgtttgtat 5700
tgtggtttgt ttattttcgt tttttttata cgttttcggt ttcgcgttta ttacgttcgt 5760
gtttcgtttt atcgcgggtt tttagtttag gtttcggggt tcgtaatagt ttaggtagac 5820
gagcgcgcgg tagcggtagt ggtaggtgaa tttgtaattt gtagagaggt ttggcggtga 5880
ggcggaggag ttttaggtcg gggaaatgtt tcggagattg aagggaagtt ttagggagag 5940
ggtcgttgtt cgttaggttt cgtaggttcg atttatttta gtgggttatt ttatattgtt 6000
acgcggattt taatgttggt tatttgggcg tttggaaatc ggtcggaagg ttataggtag 6060
agaggtttgt ttaatagttg gatttttatc gtttagtata gaattttttt tttttttatt 6120
ggtaattaaa aaaataataa taaaaaattg cgttttgttt ttgttattta ggttggagtg 6180
taatggcgcg atttcggttt atcgtaattt tcgttttttg ggtttaagcg atttttttgt 6240
tttagttttt tgagtattta ggattatagg cgttcgttat tatgtttagt taatttttgt 6300
atttttagta gagacggggt tttattatgt tagttaggtt ggttttaaat ttttgatttt 6360
aggtgattta ttcgtttcgg ttttttaaag tgttgggatt ataggtttga gttatcgtat 6420
tcggtttttt attgggaacg tatatggaat atatttgttt atttatttga aggaaaaatt 6480
aaatattttt aatttacgtt tgttttgtgg ttgttatttg tttttatttt ttttagatta 6540
agatattggt ttttatatat tttaattttt cgttttattt ttttttttta tttattttag 6600
ttaggtttgt tttttttttt aggaaattgt tcgggatagg gtttttagcg atttgtgtat 6660
tattaaatgg aatttagtgt tttatttttt atttttattt ttttagtatt atttgaagtt 6720
tgtttttttt gatttttagg ggttatattt ttttagtttt ttttttattt tttgtagttt 6780
ttgtttagtt tttttgtaga ttttgattta atttttatat tttacgatga agtttgggtt 6840
tagttttgat tattggtttg gtttgtttat atttatttgt tttagattta cggttgaaat 6900
attgatttaa atttttagat tagatttttc gtgttagtat ttttattagg atgtttaaaa 6960
gacgttttaa gtgaatatgg ttaaaattta attttttttt ttttagtttt attgttatat 7020
ttgtttagtt ttttttttgt agtaaaaatg gttattaggt ttttagttat tggagataaa 7080
agtttaaatt tatttttgat tttttttttg tttttatttt tgataaatat gtttaaatta 7140
ttttgttttt tatttttatg gttattttat ttttttttga gaacgttgta atgttttagt 7200
tttgtttttt tttttttttt tttttttttt gagatagagt tttattttgt cgttaaggtt 7260
ggagggtagt ggtacgattt cggtttattg taattttcgt tttttgtgtt taagtaattt 7320
ttttatttta gtttttcgag tagttgggat tataggtatt cgttattacg tttggtttat 7380
ttttttttat tttttagtag atatgaggtt ttattatgtt ggttaggttg gttttgaatt 7440
tttgatttta ggtgatttat tcgttttcgt ttttcgaagt gttgggatta taggtatgag 7500
ttatcgcgtt cggttttttg tttatttttt gtattttgtt ataattttgt gtttttttta 7560
gttgaatttg tgatgttttt ttgtatcgga tgagagggtt tttatgtata tatagatttg 7620
ggatattatt tatttataag tttttaaata ggttagagta gtgatgttta atttagattt 7680
tatgttataa taatttgggg agtttttaaa atttattgat gtttagggtt tatttttagt 7740
agttgattta ataggtttgc ggtgggattt aggttagcgg ggaggattgt aaaagtattt 7800
ttggtgattt tagttggtgt ttatttaggg gagagtaatt tttgtttgtt ggcgattttt 7860
aggggtgtag aaggattgtt gggtgtgtgg ttgcgtgtat attttagtat ttgatttatt 7920

gggttagaaa agggtgtttg ttaaataaag atttaataaa atttttgttt gtagggggtt 7980
tattaaaggt tttaaatttt tttaggtttt tttttatagg tggtaatttt tttttatttt 8040
aaaggttttg gagggggtta tgagtgtttg agaagaggta agtttgggaa gatggatttc 8100
gaggatagta ggtataaatt tttttttaag aagggttaag gtattttaaa gataagaaat 8160
ttaaaattag cgtattttta tatataagta gttatttttg tttatttgtg gtttagatac 8220
gagtggagtg cgataaggga taaattattt tcgcgtattt tttagcgatg gggcgaaagt 8280
aacggattta gttttcggga gttgttttcg tcgatttttt ttgtcgcgat ttgattcgcg 8340
gcgattgcgt tgttttttgg ttgttttttt cgttttcgta ggcgcgcggg gttattattt 8400
acgcgcgtat tgtaggtttt tgcgtacgac gttttagatg aagtcgttat agaggtcgta 8460
ttacgtgtgc gtggcgggtt tcgcgggttg gaagcggtgg ttacggttag ggattagttg 8520
tcgtgtgggg ttgtacgcgg tgtttcgcgc gatgcgtagc gcgttggtac gttttagtcg 8580
ggtgcggttt tttttagcgc gtttagcggg tgttagtttt cgtagtttaa tgagtttagg 8640
ttttttcgat atggttcggt tgggttcgtg tttcgttggt tttgggcgtt agtaagcgcg 8700
ggtcgggcgg ggttataggg cgggtttcga ttttagcgtt ttttttagga tttagattgg 8760
gcggcgggaa ggagttgagg agagtcgcgt aatggaaatt tgggtgtagg gattgtgggg 8820
ttcgaaggcg gggttgggcg cgttttcgta gagttttttt cgttttgttt tttttttttt 8880
ttttcgtttt ttttttatat tttatttcgg acggttataa cgacggcgat cgtaaagtat 8940
tacgcggaga tattcgtgtt tttggaggtt agttttattg tgttagagga agagggtttt 9000
tatattcggt tttggttttt ttggttcggt ttgttgaagt aatatatttg gtttatttat 9060
tgggtggggt aggaagtttc gagtttttat ttggggtgag gaggagggag atcggttagt 9120
agttttatcg ttcgttttgt tttttattgc ggagattggg gtttcggtag aggttggatc 9180
gtgattttga ggtttagggg tgtattttgg gtggattttt ttggtatggg tggtcggttt 9240
ttagtaattg tagtttttat ttggttttgt tattttgggt tgttaggata taagtttttt 9300
tatgtttttt ttagtgtttg atttggtatt ttttgtaggt aggtgggtat tgaggatggt 9360
aatgtatgtg ggggatgtgg gagtagggtt tagaggttta aggttttagg atatttttat 9420
ttgtagtaat attatttatt ttggtatcgt gagtagcgtt tagaagtttt tgtattgtag 9480
taagtatagc ggggtcgttt tggagttatt gtttttagta tatttagttt gtaggtttta 9540
gtttatttgg gggaaagtta ggaaggtttg attggttttg gaaggtgggg gtattttatt 9600
tatatttatg ttttttgtat tttttttatt ttttttgtta tttttatagg ttttattttc 9660
gcgtttgtag tcgtaggttt tgttttgagg ggttgaatat atgttggagt tggtgtttgg 9720
taattgtttg ttatttgttt ttgttttttc gttttagtcg tttttagatt tttgggattt 9780
aggagagaga agtggagagt ggtaggaagg tgttggtaaa gtgggatagt ggttttgagt 9840
agttaatttt ttagtgttta tttgttttaa tattaggttt tttttaggag gttgggtttt 9900
agttagggtt agatgggtta ggaaattttg taagtgggtt aggttgggta gttggtttag 9960
tagtgtgttt gtgaggaagg ttcgaagttg taagggtgtt tagtggaggt tagtgtgatg 10020
taggtgttgg ttagggggtt aaggaaaagg aaggggttgg gttgggttga gaatgtttgt 10080
gggggtttat gttttattag ttggtgtgtt atttgaggtt taaggattga aaagagtatt 10140
ggggttgagg tgggtgggta aagataaata gtagggacgt agtcggggta ggatggggag 10200
ggcggtattg gggttgtggg ggtt 10224

<210> 257
<211> 11787
<212> DNA
<213> Artificial Sequence

<220>
<223> chemically treated genomic DNA (Homo sapiens)

<400> 257
   tgtgaaatat tagttatgaa gaaattttga taataatatt ttagtgtaga attgtatatt 60
   agtaatagta tgtttggatt tttttagata tgtggtatta atatatgttt ggaattttaa 120
   gtgttagatt aagaagaaaa atttaagata taaagtggtg ttttaataat ttgagtttta 180
   ttatttttaa tgttaagaaa ttattatata tataaatttt atgtttgtaa ttttagattt 240
   attgttgtat aggatttttg gtaattataa gttagttgta ttaagtaaat tttgatttat 300
   ttggttggtg gttttgtgaa cgataattat ttttaggcgg gtttaatgga aggtcggaga 360
   aggtattgtt tgataagtta tattttgtta gttttagaat tttgagtgaa tgttgttgtt 420
   ttgttttgtt tgtttttata ttgtaaattg gttatgagtt tatttattat ttttttttgt 480
   tgtgaatttt tagtaatata ttttattaaa agattttttt tgataaagta gatttttatg 540
   agttgaaata tttattgatt ttaatgttta tttttagata gttttgtata ggaattttta 600
   tgtttaagga tagttgattt tggtaaattg gattttttaa aaattagatt atagaatttt 660
   tgtttgtaat ttttttattt aaggaatatt tagataattt ggtttggaga gaagattatt 720
   aaattttttt ttatataata ggtttttttt attaggtttg taagggataa atagtgaata 780
   tttatttgta attatgtaat tgttttgtag agtaagtgta gttataagtg gtaagaaatg 840
   aatgagtatg gttgtgtttt aattataatt attatggatg gatatatttg tattttttat 900
   gttagatgga attttgtttt gttgtttagg ttggagtgta gtgacgttat tttgatttat 960
   tgtaattttc gttttttagg tttaagtagt tttgttttag ttttttaagt agttgggatt 1020
   ataggcgtac ggttaatttt tgtattttta ttagagacgg ggttttatta tattggttag 1080
   gttggtttta aatttttgat ttcgtgattt gtttgtatta attttttaaa gtgttgggat 1140
   tgtaggcgtg agttatcgtg tttgatttag gattatattt aaatgattta taaatgtaaa 1200
   aattattttt atattataga tttgatttaa ggattgtagt ttgttgtgtt tttgtttttt 1260
   gaatgggacg tggtggtata gtagttggga ttatagttat atattattac gtttatttaa 1320
   ttttgtattt ttagtagaga tggggtatta ttatgtttat taggttgatt ttgaattttt 1380
   gattttaggt gatttattcg ttttaatttt ttaaagtgtt gggattatag gcgtgagtta 1440
   ttgtgttcgg tttgtatttt tatttttaaa ttttagtatt tttgaataat aacgggtaga 1500
   gaaatattcg tagtagttgt ttataataga gtgaaataaa aagtttgagg aattgttaat 1560
   agaaaaatgg gtggtcgtgg ggagtatcgg gatttgtatt tgtatataat ttagggtagt 1620
   ttttaatgag tttatgagtt atttagggat tttattaaaa tgagattagt tttgagtggg 1680
   gtttttttta tagatttttg ttattatatt ttgaagagta agggtttagt tgattttaag 1740
   taggtgtgta tttttttgga tagtgtttgt agaaaaatag aattatattt atggatttta 1800
   tgattatatt gttagttttt tggttaaggg gttgagttag gtatttattg tagatagaaa 1860
   taaagtagat gtatgtttat tagtgagtat tgagatattt agtatggtta tttatgaatt 1920
   gaagtttggt ttttttttat ttattaattg attatttttt attttaagta agtattttat 1980
   attttgtata ttagtatatt tttggatttc gtttttttat atggtttgat attttgaata 2040
   ttagattatt taagagtgag atttaattta tttttaaatt gtttttaaaa ttagggaaat 2100
   aaaagagaat gaaattgtgg taattttttt tttttttagt tattagatgt atgttattta 2160
   tggtgggttt tggttttttt aaagataatt ggaagatttt attggattga tagagagaaa 2220
   ttgcgtaatt ttattttagt atgttaagat gaagaaacgg ggggatttgt agataggtta 2280
   ggagatatgg taagtaatat ttttgatata tttttataaa ttattttagc gtttgtatta 2340
   ttttatggtt ttatggattt ttagggtggt atttcgagtg ttgttttgaa agtagttttt 2400
   ttttttattt attgtagagt gtatgaagga tatagaataa ttttatagaa attttttgtc 2460
   gtttgttgtt aataaaatta gtatatggta aaggttaggt atttataatt tgaagttaaa 2520
   gaagttttta ttttgtgatt tgtatataaa ttttttttta aataggtgga ttttttttat 2580
   attttatttg gaattgttgg attgttattt ttgggagaag aatagattaa atttgttaat 2640
   tttgtttttt gtatgtttga agaagtgttt tagagagtga atgtttagtt tgagttagtg 2700
   agttagattt attgaattga aagttgtata gtatagtttt gttattttaa tatttttgta 2760
   tttgaagtgt ttatcgaatt taaaagtgat tattgttaat attttgtata ttgtgttaaa 2820
   ttaattttaa taaattatat aattatatat attgtaaaat aaagatcggt attttatttt 2880
   ttgtttttta ttttgaagtt ttgttatttt gattatagtt ttttgtgtat atttttgtgt 2940
   ttgttatgtt taataattga gttaatataa aataatttta ggtttttatt tgattttttt 3000
   tttatgtata ttttttattt gttttatagt aagttgatgt aaattggttt gttaataaga 3060
   atgttaattg atgaaagtgg atagaattta tatatgaatt aaatgatgta taaaataaat 3120
   ggttgttgaa atttggaaat gattgatagg tttgaatatg gttattttga attggttata 3180
   gatttaggtt aaatggtaat ttattatttt aaaatggggt tgtgattcgg attgtttttt 3240
   tttttttttt tttttttttt ttttttagta ttttatatat tagaattatt taaagaagga 3300
   gttgttttta aataaggaaa tattaacggg taagttgatg gttattatag tatttttttg 3360
   tatttgtttt ttttttaaaa agttaaaatg ggtataatta ttttttttta tttttttgtg 3420
   atagaatttt tgttgtttag gttggagcgt agtagtacga ttttagttta attgtaattt 3480
   tcgtttgtcg ggtttaagta atttagtcgt agtttttcga gtagttggga ttatagtcgt 3540
   atattattat gttcggttaa tttttatgtt attttagtat agatagggtt ttattatgtt 3600
   gaggagttgg agattagttt gattttgtga tttatttatt tcggtttttt aaagtgttgg 3660
   gattataggc gtgagttatt acgtttggtt taaaatgagt ataattattt taggggttta 3720
   tagttttggt gaaagtgagg tattttttat ttttaaattt tattttttag agatagggtt 3780
   tttttttttt ttttaggttg gggtatagta gtgtaattat agtttattgt aattttaaat 3840
   ttttgggttt aagtagtttt tttattttag ttttttgagt attagggttt ataggtattt 3900
   gttattatgt ttattatgtt tagttgattt ttttaatttg gggataggta ttgttttttt 3960
   taggttggtt ttcggaggtg aagtaatttt ttggttttag ttttttaaag tattgggatt 4020
   atagggagga gttatcgtgt tcgatttgaa aataaggttt tttgatggaa attgagattt 4080
   tttttaagta tgtttggaat gttttgtgta ataattattt taagaataga gatgttttta 4140
   aggattgttg ttattgtatg tgggtaaatc gttgtgtata attggtttat tgagatattt 4200
   aatagtggta ggattttagt agtggtgtaa attaatgttt tttaatagtt tttgtatggt 4260
   agtatttaga taatttcgtt atgtttaatt tatggatttt tggtaagttg gtattattgg 4320
   ttttatttaa ggaatttttt aaaggttatt attgtaaagt tggagtttga atttttagtg 4380
   tgttattaat tgatttatat gttagaaatt atagtattag aaattggtag atggggaatt 4440
   gttaattgag agattttatt tagttagttt ttttttgttt tttattttat tatgtatata 4500
   gttatgtgta tattattttt tgataatata attatttttt ttatatttgg ttataagatt 4560
   aattttaggt gggttatgtt gtgtgagtat tatacgtttt tattgtgtta aaggtattgg 4620
   gtgtattgtg ttgggtggtt tttatattta aagataagtt agtgtgtttg gacgtgtggg 4680
   aaatgtatgt aatatttgta ataagggaga ttgggtgttg tgagaataat ggggttaata 4740
   aatcgggtgg ttattgatgg gttttgtaga gttttggtta gtttgatatt tgtaggttga 4800
   ggatgtttta gtgaggttta tgtggaaagt gatgggtttg ggaatggttg gaagtagggt 4860
   tatagatttg aagtacgtga atttatgtgg aaagttgagt ttgtttgcgt ttgcgtagtg 4920
   tagtttagtg cgtcggcgcg tagttttttc gttcgtttta gcggcgtagt ttttgtagtt 4980
   gggttattgg aggggtcgtt tagaaatttt atatttttcg ggttagggaa ggtttgggag 5040
   gatgttgagg tttgagattt tattaatttc gttttttgtt tcggcggttt tttcgtcgtc 5100
   gggagaaatt cgtttatttt agggttttcg ttataatttc ggattttagg agattttata 5160
   gagtcgtttt tcggtttagg tggtttttgt atttatttgt tttggtacgt taggagttgc 5220
   gggcgatcgg agtagtagta gtagtagttt tttttgtttc gcgttaaatt ttcggttagt 5280
   ttaaggtaag gagtgattgg gtggaggagt ttttttgagg ttagaggtcg gtagtttatg 5340
   gaggttcggg ggtacgtggg tggtggttat tatagtgaag ttgtgatttg gttggggcgt 5400
   gagatttgag tgttttggga atttggtgag tttgggtgat tagaagtttt gattttttaa 5460
   attggtttta ttgtaagatt ttgattgtgg atggtttagt tttgagaaag gttgaagtat 5520
   tttatataac gttttttata gtgtttgata aatgatagtt tgtgcgtgag tttgttgata 5580
   gaagttgtga gttgattttt ggttgttgaa tttaattttt taaattttgt taaggtggga 5640
   taaaatagat taaattgaat attttattta atttagggaa aataatatgt ttagtattaa 5700
   aagagtgggt tgggcggtgg ttgacgtttg taattttagt attttgggaa attagggtgg 5760
   gcggattatt tgaggtcggg agttttatat tagtttggtt aatatggtga atttttattt 5820
   tttatttttt atttttatta aaagtataaa aattatttgg gtgtggtggt acgtgattgt 5880
   ggttttagtt atttaggagg ttgaggtggg aggatggcga gttcgggagg tcgaggttgt 5940
   cgtgagttga gattgcgtta ttgtatttta gtttgggtga tatagtgaga ttttgattta 6000
   aaataaataa ataaaaaaga tgtatgtttt ttaaattatt tattattttg ttagttttta 6060
   tttatgataa agtattatta ttttttatat acgattgtaa tataatggta aagtaataaa 6120
   tattaaattt aatggttatt ttttagttta ttttatgtga tttttttgtt gtatttaatg 6180
   tttttaattt ttttattttt agaaatagag tttcgttatg ttgtttatgt tggttttaaa 6240
   tttttggttt taagtgtttt tgtttcggtt tgtttaagta ttgggattat aggtatgagt 6300
   tattgttgtt tggtttatgt ttaatgtttt tgattggttt ttttttaaat ttttttaatt 6360
   tggttttttt gattttattt gtttttgttt ttttgttatt ttttagagaa ttgttgtgtt 6420
   tatttttgtt tttaaattat gggtatgtta ttaaagtttt tttttttagg ttatttttat 6480
   ttagatgtga ttatttttat agtttagatt ttatttatat tttagattta ttttagattg 6540
   ttttttgaag tttttattga tggattaatg gatattttag gttgattata tgattaatta 6600
   aatttttttt tgtatttttt tttttttttt gagatagagt tttgttttgt tgtttaggtt 6660
   ggagtgtagt ggtatggtta tgatttattg tagttttaaa tttttgggtt taagcgattt 6720
   ttttatttta gtttttcgag tagttgggat taaggtatgt gtttttatag atggttaatt 6780
   taaaaaattt ttttgtagag atagtgtttt tttacgttgt ttagtttggt ttcgaatttt 6840
   taggtttaag cgattttttt attttagttt tttaaaatgt tgggattata ggtgtgagtt 6900
   attgtattta gtttttaaat ttttaagtgt ttttttattt ttgtagttta tttattgtat 6960
   tattagagtt atgtggaaaa tgattttatt atgttttttg ttatttaaat tagtagattt 7020
   gtatttgtag gtaaaagatt aaatttttta gaaggatagt taaagtaatt gggatcggtt 7080
   tttaattttt atagatttat tttttattat ttaatattta ttttgttttt taataaaatt 7140
   gagttatttg taattttttt aaagtaatat gaatgtttat attgttagtt taattttttt 7200
   ttttattaga tgtttggtta tttatttatt tattagtaat gataatattt attaatttat 7260
   atgtatttat tttgttttgg ttttttatta ggtagacggt atttttgatt tttataataa 7320
   ttttgtagag tagatttttt ttttgttttt gttttataga tagaattaag gtttaaatag 7380
   gcgaataaag gtttaaatta tgttagtagt tggggataat aatttgtgtt tttaatgaat 7440
   ttaaggttta gtagagaaga tagatttaaa aattaataaa tatatattta agaaataatt 7500
   gtatttgagg tatttataat ttttgaggta ggtataatta aagtatgggg ataaaagtgg 7560
   gaaaagatat tttatagagt aagtgtattt tgaattggtt tttaagaata agtaggaaga 7620
   agaatgttag ttaaagggaa atagttggag cgtatttttg agaaaaatac gtgtaaaggt 7680
   atagaagaat tatgtaaaat tgttaataat tttgtttggt tagagtagga gagtgattta 7740
   ggtagataaa tttgaatgta gatatagttg tgagaattag atttgggaga taaatttaat 7800
   ttagagattt tttatattta atattaggtt tttggttttg gtgattggat agatggtagt 7860
   agtatttttt gattttggga attttgtagg taaagtaaat tttagaggga agatgatgag 7920
   ttttgttatg gatatgttga gtgtgaaaag tttgtgggat atttatatat ttatattaag 7980
   ataattgaat ataggaaatt agagttaaga agagagaaat ttgagggaaa tatggatttg 8040
   aaagttatta gtatgttggg attatagtag agaatatgaa gattatatag taaaatagag 8100
   gttaaggaag taggaggaga attaagataa gtgaatgagt tttaagaaag ggtatatagt 8160
   ttaaaagtag tttgagaata agaaatagtt ttttttattt ttagttttcg tatttttagt 8220
   ttttggtaat tatttttttt tttagttttt taaagtttag aaatgttaat ggagtatatt 8280
   ttatgttggt tttatatacg tttttatgat agtattttta tattttgttt atatttttgt 8340
   aaatattttt tttattaaat tattttagtt tgagtatatt attgattttt gttggaattt 8400
   atataatgaa gttatatttt attttatggt gagtatgggg agatatatag gaattgtaag 8460
   aaagagagat attgattaga tttgtttttt agaaagatta ttggtgatga ttcgaagaat 8520
   agattagaga ggtgtaatat tggaagtaag gagaagaaga agtaggaaat ttgtataagg 8580
   attaaagaag atataggttt aaaatgggtt gggaggagta gagagggatt taaattttag 8640
   tgatgttaaa aaaaaagaat tttagtattt agttattgag tgttagggtt gatgaaggaa 8700
   gaattgagaa tgttttttaa gtttatgatt tgagtgataa gggataagtt ggtataatta 8760
   attatattgg taataaagaa ttaattttga gggaaagata gagtttattt ttggatattt 8820
   agaatgttgg gaagatattt aaaggtttat gtttaagaga tagatatagt gatttggagt 8880
   tatattgttt gatagaatat tttgtgataa tagaaacgtt ttatatttat gttgttcgat 8940
   gtggtaatta gtagtcgtat gtggtttttg agtatttgaa aatgttgtta gtgtgattga 9000
   ggaatagaat ttaaattgta tttaattttg atttagttaa atatttatat atgttgtttt 9060
   attgaataat gtaggtatag agttgagaac ggtttttgta tttttgaagg attaaagaat 9120
   taagaatatt ttgtgatatt aaaattatat gaaatttaga atttaatatg tagaaataaa 9180
   gttattttta attgaaatat agttattttt atttatttat gtattattta tgattgtttt 9240
   tttatgatag tgttagagtt gagtaattgt gatagtgatt atatggttta taaaagttta 9300
   aaatatttgt tatttggttt tttatttaaa agtttatggg ttgggtatga tggtttatgt 9360
   ttataatttt agtattttgg gaggttgagg tgggtagatt atttgaggtt aggagtttat 9420
   gattagtttg gttaatatga taaaattttg tttttattaa aaatataaaa aaaaaatagt 9480
   taggtgtggt gggtatgttt ataatgttag ttgtttggga ggttgaggta ggaggattgt 9540
   ttgaatttgg gaggtagagg ttgtagtgag ttgagattgc gttattgtat tttagtttgg 9600
   gtaataagag tgaaatttta ttttaaaaaa aaaaaaaaaa agtttatgtt gaggtatggt 9660
   ggtttatgtt tgtaatttta gtgttttggg aggttgaagt gggaggattg tttgaggtta 9720
   ggtgtttgag attagtttgg gtaatgtagt aagattttgt ttttataaaa aaaaaattag 9780
   ttgtgtgtgg tgaggtgtat ttatagttta tagttattta agaggttgaa gtaggaggtt 9840
   atttgagttt atgagtttga ggttgtagtg agttgtgatt atattattgt attttagttt 9900
   gggcgataaa gagagatttt gtttttataa gatagtaaaa tagattgggt atggtggttt 9960
   tcgattataa ttttagtatt ttgggaggtt gagatgggaa gattatttga gtttaggagt 10020
   ttgagattag tttgggtaat atggtaaaat tttattttta taaaaatata aaaaattagt 10080
   ttggtatggt ggtatgagtt tatagtttta gttattcggg agggtgaggt aggaggattg 10140
   tttgagttta ggagtttaag gttgtattta gttaagatta tattattgaa ttttagttta 10200
   agttatagaa cgagagtttt atatatagat aaaaaggaaa aattaaaagt ttagtaattt 10260
   ttggtttata gtttaaggag tcgttatagt tatatacgaa ggtttgagag ttgttagaaa 10320
   atagatgtta atagaaataa agaggagaga tgagatgttt ggagatgtat aatgttgatt 10380
   tgtgagggaa agtttaaaag tatgaagttt tggaggttaa tagttaagaa gtagaggaaa 10440
   aaggagatgg taaaggaaat taaaagtggt ttaaagagaa tttaagatag agagttgtgg 10500
   aaattaaaga gaataggtga atagagttta aatattttaa aattttttta gaatttttat 10560
   gtgttaagag gttatgttag gttgtttatg tatattattt aaaaatttta tgataatttt 10620
   gtaaaatagg taatagttta tattagtaga taagtattta aataaggaat tgaagttaat 10680
   tgagtttaaa atgttatttg tataggggag agaaatttaa tgaagaaatt aatagaagaa 10740
   ggttaaaaga ggtttaataa ttattatata aatgaaatat atgttttagt ttgtattttg 10800
   ttgtttttaa aagaatattt gaaattgggt gattattaag aaagaaaata tattttttgg 10860
   aagtttggag gttgggaagt ttaaggttaa aggaggtata tttagtgagg gtttttgtgt 10920
   tggtggagtt tttgtagagt tttgtagtgg tatagggtat tatatggaga gggagttgag 10980
   tatggtagtt taggtttttt attttttttt tataaaatta ttagttttat tttcgtgata 11040
   atttattaat ttattaaatt atgaatggat taatttattt atgaaggtag agtttttatg 11100
   ttttattatt ttttaaaggt tttatttttg ttttgttata ttttattggg gattaaattt 11160
   taatgtaagt ttcggagggg atagatattt atattttagt aatatatttg gtatatttga 11220
   agattaaagg gtaatttatg attttagtta gagtagttgt agtgaatttg tagaggtaaa 11280
   agttatatta gtatattttt gtatatttat taataatgtt attaattttg ttataatttg 11340
   tttattttta aaaattattt ttatatgaaa ttttatagaa agtgaatata gtggttgggt 11400
   gcggtgattt aggtttttaa ttttagtatt ttgggaggtc gaggtaggta gattatgagg 11460
   ttaggaattc gagattagtt tagttaatat ggtgaaattt tatttttatt aaaaatataa 11520
   aaatttatta ggcgtggtgg cgggtgtttg taattttagt tattcgggag gttgaggtag 11580
   gaggattatt tgaatttggg aggtggacgt tgtagtgagt cgagattatg ttattacgtt 11640
   ttagtttggg cgataagagt aagattttgt ttttaaaaag aaaaaaagaa aaaaaaggaa 11700
   agtgaatatg gtataaatta taatgattaa tatattattg tataatttaa atttttaaga 11760
   attgattgtt aattttttaa atttagg 11787

<210> 258
<211> 11787
<212> DNA
<213> Artificial Sequence

<220>
<223> chemically treated genomic DNA (Homo sapiens)

<400> 258

tttaaatttg aaaaattaat agttaatttt taaagatttg aattatgtaa tgatgtatta 60
gttattataa tttatattat gtttattttt tttttttttt tttttttttt ttttgagata 120
gagttttgtt tttgtcgttt aggttggagc gtagtggtat gatttcggtt tattgtaacg 180
tttatttttt aagtttaagt gatttttttg ttttagtttt tcgagtagtt gggattatag 240
gtattcgtta ttacgtttgg tgaatttttg tatttttagt agagatgggg ttttattatg 300
ttggttgggt tggtttcgaa ttttgatttt tatgatttgt ttgtttcggt tttttaaagt 360
gttgggatta gaggtttgag ttatcgtatt taattattat gtttattttt tataaaattt 420
tatgtgaaaa tgatttttga aaataaatag attataataa aattaatgat attattgata 480
aatatataaa aatatgttaa tatagttttt gtttttataa gtttattgta attattttgg 540
ttaaaattat aaattatttt ttaattttta aatatattag atgtgttgtt aaggtatgaa 600
tatttgtttt tttcgaaatt tatattgaaa tttaattttt agtgaagtgt ggtaggatag 660
aggtggggtt tttaagaggt gatggggtat gagggttttg tttttatgaa tgaattaatt 720
tatttatgat ttaataggtt aatgggttat tacgagagta ggattggtgg ttttataaga 780
agaggatgag agatttgagt tattatgttt agtttttttt ttatgtgatg ttttgtatta 840
ttataggatt ttatagagat tttattagta taaagatttt tattaaatgt gttttttttg 900
attttggatt ttttagtttt tagattttta agaaatatgt tttttttttt gataattatt 960
tagttttaag tattttttta aaagtaatag aatatagatt aagatatatg ttttatttgt 1020
ataataatta ttagattttt tttaattttt ttttgttaat ttttttattg aatttttttt 1080
ttttatgtaa atggtatttt agatttaatt ggttttagtt ttttatttag gtatttattt 1140
attgatgtaa gttattattt attttatagg attgttataa aatttttaaa taatgtatat 1200
aaataattta gtatagtttt ttggtatata gaagttttaa aaaaatttta aaatgtttgg 1260
gttttgttta tttatttttt ttgattttta taatttttta ttttgaattt tttttaaatt 1320
atttttagtt ttttttgtta tttttttttt tttttgtttt ttaattgttg gtttttaggg 1380
ttttatattt ttaagttttt ttttataaat taatattatg tatttttagg tattttattt 1440
tttttttttg tttttattaa tatttatttt ttgataattt ttaagttttc gtatatagtt 1500
gtaacgattt tttaaattgt aaattagggg ttgttaaatt tttaattttt tttttttgtt 1560
tgtatgtgag attttcgttt tgtagtttag gttggaattt agtggtgtga ttttggttaa 1620
atgtagtttt gaatttttgg gtttaagtaa tttttttatt ttattttttc gagtagttgg 1680
gattataggt ttatgttatt atgttaggtt aattttttgt atttttgtag agatggggtt 1740
ttgttatgtt gtttaggttg gttttaaatt tttgggttta agtgattttt ttattttagt 1800
tttttaaaat gttgggatta taatcgggag ttattatgtt tagtttattt tattgtttta 1860
taaagataag gttttttttt gtcgtttaga ttggagtgta gtagtgtgat tatagtttat 1920
tgtagtttta agtttatggg tttaagtaat tttttgtttt agttttttga gtaattatgg 1980
attataggtg tattttatta tatatagtta attttttttt tatagagata gggttttgtt 2040
atattgttta ggttgatttt aaatatttgg ttttaagtaa tttttttatt ttagtttttt 2100
aaagtattga gattataagt ataagttatt atgttttagt ataaattttt tttttttttt 2160
tttgagatgg agttttattt ttgttgttta ggttagagtg taatggcgta attttagttt 2220
attgtagttt ttgtttttta ggtttaagta atttttttgt tttagttttt taagtagttg 2280
gtattatagg tatgtttatt atatttggtt attttttttt tgtattttta atagagatag 2340
ggttttgtta tgttggttag attggttatg aatttttggt tttaggtgat ttgtttattt 2400
tagtttttta aagtgttggg attataggta tgagttatta tgtttagttt ataaattttt 2460
aaataaagga ttagatggta aatattttag gtttttgtgg gttatataat tattgttata 2520
attatttagt tttgatatta ttatagaaaa gtagttatag ataatatata aataaatgag 2580
aatggttatg ttttaattaa aaatggtttt atttttatat attgaatttt aaattttata 2640
taattttaat attataaaat atttttgatt ttttaatttt ttaaaagtgt aaaaatcgtt 2700
tttagtttta tatttgtatt gtttaataaa gtagtatatg tgagtattta attaaattaa 2760
aattaaatat aatttaaatt ttgtttttta gttatattag taatattttt aaatgtttaa 2820
aagttatata cggttattag ttattatatc ggatagtata agtatgaaac gtttttatta 2880
ttatagaata ttttattaga taatgtagtt ttaggttatt atatttattt tttgggtata 2940
aatttttagg tattttttta gtattttaaa tgtttaaaag tgaattttat ttttttttta 3000
aaattaattt tttattatta gtatggttaa ttgtattagt ttgttttttg ttatttaagt 3060
tataagttta ggagatattt ttaatttttt ttttattaat tttaatattt agtgattgag 3120
tgttagaatt tttttttttt aatattatta aaatttaaat ttttttttat tttttttagt 3180
ttattttaag tttgtatttt ttttggtttt tgtgtaagtt ttttattttt tttttttttt 3240
gtttttagta ttatattttt ttaatttatt tttcgaatta ttattaataa tttttttgaa 3300
aagtaaattt gattagtatt tttttttttt ataattttta tgtgtttttt tatatttatt 3360
atagggtaaa atataatttt attatatggg ttttagtagg aattaatgat atatttaaat 3420

tgggataatt tgataaagag aatatttata aaggtgtgga tagggtgtag gggtgttatt 3480
ataaagacgt gtgtagaatt agtataaaat gtgttttatt agtattttta ggttttaggg 3540
agttaaggaa gagagtggtt gttagaaatt ggagatgcgg aaattggaga tggagagggt 3600
tgttttttgt ttttagatta tttttagatt atatattttt ttttgaaatt tatttatttg 3660
ttttaatttt ttttttattt ttttgatttt tattttgtta tatagttttt atattttttg 3720
ttgtggtttt aatatattgg taatttttaa atttatattt tttttaagtt tttttttttt 3780
tagttttagt tttttatatt taattatttt aatataaata tatggatatt ttataggttt 3840
tttatattta gtatgtttat aatagagttt attatttttt ttttaaaatt tgttttgttt 3900
ataaaatttt tagaattaga aaatattatt attatttatt tagttattaa aattagagat 3960
ttggtgttga atatgggggg tttttgggtt aaatttgttt tttagattta atttttataa 4020
ttatatttat atttaagttt atttatttag attatttttt tgttttagtt aagtaaaatt 4080
attagtagtt ttatatgatt tttttgtgtt tttgtacgtg ttttttttaa gaatgcgttt 4140
tagttgtttt tttttggttg gtattttttt ttttatttat ttttgaagat tagtttaagg 4200
tgtatttatt ttgtgaaatg ttttttttta tttttgtttt tatattttaa ttatatttat 4260
ttttaaaatt ataaatattt taaatatagt tattttttaa atgtgtattt gttgattttt 4320
aagtttgttt tttttattga attttaagtt tattgagagt atagattatt atttttagtt 4380
gttaatataa tttgaatttt tgttcgttta tttgggtttt ggttttattt gtaaaatagg 4440
aataaaaaaa aaatttattt tgtaaaatta ttgtgaggat taaagatatc gtttgtttag 4500
taagaagtta gaataaagta ggtgtatata aattagtgaa tgttattatt attgataaat 4560
aagtgggtga ttaagtattt gatgaagaga gggattagat tgataatgtg aatatttatg 4620
ttgttttaaa ggaattgtaa gtagtttagt tttgttagaa agtagggtaa atattaaatg 4680
atgagggata aatttgtaaa gattagaaat cgattttaat tgttttggtt atttttttag 4740
agagtttgat tttttatttg tagatatagg tttattgatt taagtggtaa gggatatgat 4800
aagattattt tttatataat tttggtggta tagtaagtaa gttataagga tggaagaata 4860
tttaagagtt tgagggttag gtgtagtggt ttatatttgt aattttagta ttttgggagg 4920
ttgaggtagg aggatcgttt gagtttagaa gttcgagatt aggttgggta acgtaggaaa 4980
atattgtttt tataaaaaaa ttttttaaat tagttatttg taggggtata tgttttggtt 5040
ttagttattc gggaggttga ggtgggagga tcgtttgagt ttaggaattt gaggttgtag 5100
tgagttatga ttatgttatt gtattttagt ttgggtaata gagtaagatt ttgttttaaa 5160
aaaaaaaaaa aagtgtagag aggaatttaa ttaattatat agttagtttg aggtatttat 5220
tagtttatta ataaaaattt tagaagatag tttgaagtaa gtttggagtg tagatgagat 5280
ttgagttgtg gagatagtta tatttaggta gaagtggttt aggaaaaaaa gttttaatga 5340
tatgtttata atttgggaat aaaggtgggt atagtaattt tttgggaggt agtagaaaaa 5400
taggggtaag tggaattaag gaagttaaat taaaagagtt taagagaaga ttagttaaga 5460
atattaaata taggttaggt agtagtggtt tatgtttgta attttaatgt ttgggtagat 5520
cgaggtagga gtatttgaga ttaggagttt gagattagta tgggtagtat agcgagattt 5580
tgtttttaaa aataaagaaa ttaagaatat taaatatagt agaaaggtta tatgagatga 5640
attgaaaaat gattattgga tttggtattt gttattttat tattgtgttg taatcgtgtg 5700
tgaaaaatgg tgatgttttg ttatgaatag agattaataa aataataaat gatttgaagg 5760
atatgtattt tttttgtttg tttgttttga gttagggttt tattgtgtta tttaggttga 5820
aatgtagtgg cgtaatttta gtttacggta gtttcgattt ttcggattcg ttattttttt 5880
attttaattt tttgagtagt tgggattata gttacgtgtt attatattta gataattttt 5940
gtatttttag tagagatagg ggatgggggt gggaggttta ttatgttggt taggttggtg 6000
tggaattttc ggttttaagt gattcgttta ttttggtttt ttaaagtgtt gggattatag 6060
gcgttagtta tcgtttagtt tattttttta atattaaata tattattttt tttgaattag 6120
atgaaatgtt taatttaatt tattttattt tattttagta ggatttggag aattaaattt 6180
agtagttaaa gattagttta taatttttat tagtaaattt acgtataaat tgttatttgt 6240
tagatattgt gagggacgtt atatgggata ttttagtttt ttttagaatt gagttattta 6300
tagttaaggt tttgtaatag aattaatttg aaaagttaag gtttttaatt atttagattt 6360
attaaatttt tagggtattt agattttacg ttttaattaa attataattt tattgtggta 6420
attattattt acgtgttttc gagtttttat gaattgtcgg tttttagttt taaggagatt 6480
tttttattta attatttttt gttttgagtt ggtcgggagt ttggcgcggg gtagggaagg 6540
ttgttgttgt tgttgtttcg gtcgttcgta gtttttgacg tgttaggata ggtggatgta 6600
gagattattt ggatcgaagg gcggttttgt ggagtttttt ggagttcgaa attgtagcgg 6660
ggattttgag gtgagcgggt ttttttcgac gacggggaaa tcgtcggggt agaaggcgag 6720
gttgatgaga ttttaggttt tagtattttt ttaaattttt tttgattcga gaagtatgag 6780
gtttttgagc gattttttta gtagtttaat tatagaagtt gcgtcgttga aacgggcggg 6840
agaattgcgc gtcgacgtat taagttgtat tgcgtaggcg taggtaggtt tagtttttta 6900
tatggattta cgtattttag gtttatgatt ttgtttttag ttatttttaa atttattatt 6960
ttttatatga attttattgg aatattttta gtttgtaggt attagattga ttagaatttt 7020
gtaaggttta ttaatgatta ttcgatttgt tagttttatt atttttatag tatttaattt 7080
tttttattgt aaatgttata tgtatttttt atacgtttaa gtatattagt ttatttttaa 7140
atgtagaaat tatttaatat agtatattta gtgtttttga tataatgaga acgtgtaatg 7200

tttatatagt ataatttatt taaggttgat tttgtgatta aatataggaa agatagttat 7260
attattaaaa gatagtatgt atatggttgt gtgtatagta gagtaggaaa tagggaaaga 7320
ttgattgggt aagatttttt agttaataat tttttattta ttaattttta gtattgtggt 7380
ttttaatata taaattagtt aatggtatat tagaagttta aattttagtt ttgtagtggt 7440
gatttttaga aaatttttta aataaggtta atagtattaa tttattagag atttatggat 7500
taaatatgac gaaattattt ggatattgtt atatagaagt tattagaaga tattaattta 7560
tattattatt gaaattttgt tattgttgag tattttaata aattagttat gtatagcggt 7620
ttatttatat gtagtgataa tagtttttaa gaatattttt gtttttggaa tgattattgt 7680
atagggtatt ttaaatatat ttgagaaaaa ttttaatttt tattaaaaaa ttttattttt 7740
agatcgggta cgatggtttt tttttgtaat tttagtattt tgggagattg aggttagagg 7800
attattttat tttcggagat tagtttgggg aagatagtgt ttgtttttaa aattaagaaa 7860
ttagttgggt atagtgggta tggtggtagg tatttgtaga ttttggtatt taggaggttg 7920
aggtggaagg attgtttgag tttaggagtt taaggttata gtgagttgtg attgtattat 7980
tatgttttag tttggggaag aaaggaagat tttgttttta aaaaataaaa tttaaaaata 8040
gagaatattt tatttttatt aaaattgtaa atttttagag tgattgtatt tattttaggt 8100
taggcgtggt ggtttacgtt tgtaatttta gtattttggg aggtcgaggt gggtggatta 8160
taaggttagg ttggttttta gttttttagt atggtgaaat tttgtttgta ttaaagtaat 8220
ataaaaatta gtcgggtatg gtggtgtgcg gttgtagttt tagttattcg agaggttgcg 8280
gttgaattgt ttaaattcgg tagacggagg ttgtagttaa gttgagatcg tgttgttgcg 8340
ttttagtttg ggtaatagag attttgttat aaaaaaataa aaaagagtga ttgtatttat 8400
tttagttttt taagaaaaag ataggtgtaa aaaaatattg tggtgattat taatttattc 8460
gttagtgttt ttttatttga agataatttt ttttttgggt aattttgata tataaaatgt 8520
taaaaaaaaa aaaaaaaaaa aaaaaaaaaa ggatagttcg aattataatt ttattttaag 8580
atggtagatt gttatttaat ttaagtttgt gattaattta gaataattat atttaaattt 8640
attaattatt tttaaatttt aatagttatt tattttgtgt attatttaat ttatgtatgg 8700
gttttattta tttttattag ttaatatttt tgttgataaa ttaatttata ttaatttgtt 8760
atagaataga tgaaaggtat atatggggga aaaattaagt agaaatttag agttatttta 8820
tgttagttta gttattgaat ataatagata tagaagtata tataaagaat tgtagttaga 8880
ataataggat tttagaataa aaagtagaaa ataaaatatc ggtttttatt ttataatatg 8940
tataattata taatttatta aaattaattt aatataatat ataaaatatt aatagtagtt 9000
atttttagat tcgataagta ttttaaatat agaaatgtta aaatggtaaa attatattat 9060
gtaattttta atttaatgaa tttagtttat tagtttaggt tgaatattta ttttttgaag 9120
tattttttta ggtatgtaaa agataggatt aataggttta atttgttttt tttttaaaag 9180
tgataattta gtaattttaa ataaggtatg aaaaggattt atttatttaa aaaagaattt 9240
atatatagat tataaaatga aaattttttt aattttaaat tatgaatatt tgatttttat 9300
tatatattaa ttttgttagt aataagcgat aagaaatttt tgtaagattg ttttatattt 9360
tttatatatt ttataataaa tagaaaaaaa aattgttttt aaagtaatat tcggaatgtt 9420
attttggaaa tttatggaat tatgaagtaa tgtaaacgtt gaaataattt atagaaatat 9480
gttagaaata ttatttgtta tattttttgg tttgtttgta aattttttcg tttttttatt 9540
ttgatatgtt aaaatgaaat tacgtagttt tttttattta atttaatgaa gtttttttaat 9600
tatttttagg gaagttagga tttattatga gtagtatata tttggtaatt aggagggaaa 9660
aaagttgtta taattttatt tttttttatt tttttaattt tgagaataat ttaaaagtgg 9720
gttaagtttt atttttaggt aatttgatgt ttaaagtgtt aaattatgtg gaaggacgaa 9780
atttagggat atattgatat gtagggtatg aaatatttgt ttagaatgaa agataattaa 9840
ttaatgggta agaagagatt aaattttaat ttataggtaa ttatgttaaa tattttagta 9900
tttattgata agtatgtatt tgttttgttt ttatttgtaa tgaatgttta atttagtttt 9960
ttaattaaga agttagtagt ataattatga aatttatggg tatagttttg tttttttata 10020
agtattgttt aggggggtat atatttgttt aaaattaatt aaatttttgt tttttaaaat 10080
atggtgatag aagtttataa agaaaatttt atttaaggtt gattttattt taatgagatt 10140
tttgagtaat ttatagattt attagaagtt gttttagatt gtgtgtaagt gtaggtttcg 10200
gtgtttttta cggttattta tttttttgtt gatagttttt taggtttttt attttatttt 10260
attgtagata gttattgcgg gtgttttttt attcgttgtt atttagaaat attaaggttt 10320
aaaaataaaa atataggtcg agtatagtgg tttacgtttg taattttagt attttgggag 10380
gttgaggcgg gtagattatt tgaggttagg agtttaagat tagtttgatg aatatggtga 10440
tattttattt ttattaaaaa tataaaattg gatgggcgtg gtggtgtatg attgtaattt 10500
tagttattgt attattacgt tttatttaga gggtaggggt atagtaagtt ataatttttg 10560
agttaaattt atagtgtagg aatagttttt atatttataa attatttaaa tatagttttg 10620
ggttaggtac ggtggtttac gtttataatt ttagtatttt gggaggttga tgtaggtaga 10680
ttacgaggtt aggagtttga gattagtttg gttaatatgg tgaaatttcg tttttaataa 10740
aaatataaaa attagtcgtg cgtttgtagt tttagttatt tgggaggttg aggtagaatt 10800
gtttgaattt gggaggcgga ggttgtagtg aattaagatg gcgttattgt attttagttt 10860
gggtaataga gtaagatttt atttgatata ggaaatataa gtatatttat ttataataat 10920
tgtaattgga atatagttat atttatttat tttttattat ttatggttgt atttgtttta 10980

taggataatt atatagttgt aaatgagtat ttattatttg ttttttatag atttgataag 11040
agagatttat tatgtggagg aaaatttaat aatttttttt ttaaattaaa ttatttgggt 11100
attttttaaa taagaagatt gtaaataaaa attttgtagt ttaattttta aaaaatttaa 11160
tttgttaaaa ttaattgttt ttaaatatgg agatttttgt ataagattgt ttggaaatgg 11220
atattaggat taatgagtat tttaatttat gggggtttat tttgttaagg ggagtttttt 11280
agtaggatat attattgaaa atttataata gggaaaggta ataaataagt ttatggttaa 11340
tttataatat agaaataggt aagataaagt agtagtattt atttaaggtt ttaaagttag 11400
tagaatatgg tttattaaat aatatttttt tcggtttttt attgagttcg tttgagggta 11460
attgtcgttt ataaagttat tagttaagta aattagaatt tatttgatgt aattgatttg 11520
taattgttag aaattttgtg taataataga tttaaaatta tagatataaa gtttatgtgt 11580
ataataattt tttgatatta aaagtgataa aatttaaatt attaaaatat tattttatat 11640
tttaagtttt tttttttgat ttgatattta aaattttaaa tatgtattaa tattatatat 11700
ttgaaaaaat ttaagtatat tattattagt atgtaatttt gtattgaaat attattgtta 11760
aaattttttt atggttagta ttttata 11787

<210> 259
<211> 2455
<212> DNA
<213> Artificial Sequence

<220>
<223> chemically treated genomic DNA (Homo sapiens)

<400> 259

ggagtgtaag aatatagaat taaaatagag tttgaaattt aaagaaaggg agagatttgg 60
gggaggagtg gggtggagtg acgtgatgtg ttgttggaaa ttagtagttg gtggtttttt 120
tttgtgtttt tttttttgtg ggtttttttt tgtttgtggg agggtttttt tttttttttt 180
cgatagaaag gttatttttg gtgttcgttt ttttgaattg taatattttg taagggtatg 240
attttatgtt tttgtgtggg tgtgaatttt tttatggtga tttttaaaat ttgtatatag 300
gatttttttt tattgagggg aggggattaa aataatttta ttttttaggg tttttttttg 360
ttttaattgg tttgtgttta agagaagttt taggtaaatg gggttagttt gaagattaaa 420
taggtttggt agtttttttc ggtttttttt ttttttttta tagttttata gttatagttg 480
ttttgatatt aatattgatt ttggttggtt ggtatgatta tttatagggt atcgtgtttt 540
aatttattag gtgataggta acgttgtttt tttttggaat tatttagtag agttagggtt 600
gtatttttaa attttgtaat agaggttttt ttttatttta tttttttgtt tttgtatttt 660
ttttatttta gtagtttttt tttttttttt tgggtttttt tttttatttt tttttttttt 720
tgggtttggt aaattagttt ttaatttttt tatattttag attggaaggt gggttttttt 780
ttgatatttt ttagagttgt tattaatttt ttttaagttt ttatagtttt attgtttaat 840
agatttgtta ggggtaatta ttaatttagt ttttaatttt gtttttttat tttttttgtt 900
ggaggggatt ttttaattat tggttagtat agttaggtta ttttattttt attatttttt 960
ttaatttttt gggttggggg gttggggagg aattttttta ttttagggta ttaggaataa 1020
agttggggag gatggtgtat ttaaagggat tatatatata tatatatata tttttttttt 1080
ttttttttta taattttatt ttcgtaatat atatatatat atatatatat atatatatat 1140
atatatatat agacgtataa ataagtttta tggagtagtg attttattat gtttatcgtt 1200
ttgagtttaa tttttggttt aaaataggta ttaaatagtt gtcgaatgta tgaatgatag 1260
atattttttt gtttttaggg gtgtgtagaa gtgcgaaggg gtatgggtat gttttagtag 1320
gggtgtgagt gttttgatta gaattatttt tttttgttag aatttgatgt aattcgaatg 1380
tttttatttt tgtttgaagg gtttaaataa taaattaggt tttgtcgtgt tattatgggg 1440
gtggttatat tttgtattta ggaaataggt acggtagggt tgagatagaa gttttgtttg 1500
ttttcgttta tttatttgaa atatcgttta tttaggtttt attttgtttg ttaggtattg 1560
ttttaagttt tgtataaata ttaatttaga gggtataaat attaatttaa gagttgttgt 1620
aggaaaaaaa ataagcgttt ttggtttttt aagtttggtt ttttttttaa aattttcgta 1680
acggttttaa atttttttta gggattggga ttacggattt tggttcgatt ttttcgcggg 1740
ttttttattg cgttaattaa attttagaaa tagtgagtgt tagaggttcg gttgttaagt 1800
aacggtagag ggcgggaagt ttgaacgttt tggattcgtt tcgaaggtaa ataggttaat 1860
tagcgtttag attttttagt tacggtagtt cgtttttttt tttcgttttg tcggattttt 1920
aggttggatt cgggtttttt taattaatag cggttaggag ggcggggcgc gtgcgcgcgt 1980
atttcgttta cgcgtcggcg cgtttttttt gtaggttcgt ggcggtcggt tagcggggcg 2040
tttttttatt tgtagcgatt taggttattg aaaaggcggg aaaacgttgc gatggcggta 2100
gttgggggag gaggaagata agcgcgtgag gttggggttt tggcgcgtgg ttggtagagg 2160
tagagatata agacgtgtac gattcgtttt ataggttttt tagatttttt ttttttaaag 2220
ggtaattttc gcgtgatagg aatgagggtg gggcgcgtgg agttttttat aatttgtatt 2280

ttagttaaat attcgagaat ttattttttg tgtttatagt tttttacgtt ttttagtttt 2340
gtttcgtagt tttgtagtag aagtatttag tgttttgtat tttgcgcgtt attttatttc 2400
ggtttttttt gtgaatcgtt gttttcgaat cgtttttatt ttttgtattc gtaga 2455

<210> 260
<211> 2455
<212> DNA
<213> Artificial Sequence

<220>
<223> chemically treated genomic DNA (Homo sapiens)

<400> 260

tttgcggatg taaaaagtga gggcggttcg gaagtaacga tttatagagg aggtcggggt 60
agggtggcgc gtagaatgta aagtattaag tatttttgtt atagggttgc ggggtagggt 120
tgaggggcgt ggagagttgt ggatatagga ggtgaatttt cgggtattta attaaagtat 180
agattgtggg aaattttacg cgttttattt ttatttttgt tacgcggagg ttattttttg 240
gaaggaaggg gtttgagggt tttgtggggc gagtcgtgta cgttttatgt ttttgttttt 300
gttaattacg cgttaggatt ttagttttac gcgtttattt tttttttttt ttagttgtcg 360
ttatcgtagc gttttttcgt ttttttagta atttgagtcg ttataggtgg gagaacgttt 420
cgttgatcga tcgttacgag tttgtaaaag gagcgcgtcg gcgcgtgagc gaggtgcgcg 480
cgtacgcgtt tcgttttttt agtcgttgtt gattggagag gttcggattt agtttagaga 540
ttcgataggg cgaggaggag aagcggattg tcgtagttga agagtttgga cgttgattgg 600
tttatttgtt ttcggggcgg gtttagaacg tttaaatttt tcgttttttg tcgttgttta 660
gtagtcgggt ttttagtatt tattgttttt gggatttgat tggcgtagtg ggaagttcgc 720
gagaaggtcg gattagggtt cgtagtttta gtttttggaa ggggtttggg atcgttgcgg 780
gggttttgag gggaggttta aatttaaaga gttagaggcg tttatttttt tttttgtaat 840
aatttttaag ttaatatttg tattttttga gttaatattt atatagagtt tagaatagtg 900
tttggtaaat aaagtaagat ttaaatgagc ggtgttttaa ataaataagc ggaaataagt 960
aggatttttg ttttagtttt atcgtgtttg ttttttgggt atagggtatg attattttta 1020
taatggtacg atagggttta atttattatt taaatttttt aagtagaggt ggaagtattc 1080
gaattatatt aaattttggt agagagaagt agttttgatt agaatattta tatttttatt 1140
gggatatgtt tatatttttt cgtattttta tatatttttg aagatagaga ggtatttatt 1200
atttatgtat tcggtaatta tttagtgttt attttgggtt aggggttgga tttaaagcgg 1260
tgaatataat gaagttattg ttttataaag tttatttgtg cgtttgtgtg tgtgtgtgtg 1320
tgtgtgtgtg tgtgtgtgtg tgtgtgtgtt gcgggggtgg ggttatgagg gagaaagaaa 1380
aaaaatatat atatatatat atataatttt tttaaatgta ttattttttt tagttttgtt 1440
tttagtattt tgagatgggg agattttttt ttagtttttt aatttaagaa gttaggaaag 1500
atggtggggg tgagatgatt tagttgtgtt aattagtaat tggaaaattt tttttagtaa 1560
gaaaggtggg ggaatagagt taagggttgg gttaatggtt atttttggta aatttgttga 1620
gtaatggagt tatagaaatt tggaggaggt tggtgatagt tttggggagt gttagggagg 1680
gatttatttt ttaatttggg gtgtgaagag attagggatt agtttattaa gtttaggaag 1740
gggagggagt ggaaagagaa gtttagagag ggaaagagga gttattgaga taggagaaat 1800
gtagggatag ggaggtgaga tggagggaaa tttttgttgt aggatttggg ggtatagttt 1860
tggttttgtt ggatggtttt aggagagggt agcgttgttt gttatttggt aaattaaggt 1920
acgatatttt gtgggtagtt atgttagtta gttaaagtta atattgatat taaggtagtt 1980
gtagttataa agttgtagga gagaaaagag aggtcgggag aggttgttaa atttgtttga 2040
tttttaagtt ggttttattt atttaaggtt ttttttggat atagattagt tggaatagga 2100
gaggattttg agaagtagag ttgttttgat tttttttttt taatgggaag gggttttgtg 2160
tgtagatttt gagggttatt atgagggaat ttatatttat atagaggtat ggaattatat 2220
ttttatagga tgttatagtt taagggaacg aatattaaag atagtttttt tgtcgggagg 2280
agagaaaaag gtttttttat aagtaggaga aaatttatag aagaggaagt ataagaggaa 2340
attattaatt gttggttttt agtagtatat tacgttattt tattttattt tttttttaag 2400
tttttttttt tttttaagtt ttaagttttg ttttagtttt gtgtttttgt atttt 2455

<210> 261
<211> 2932
<212> DNA
<213> Artificial Sequence

<220>
<223> chemically treated genomic DNA (Homo sapiens)

<400> 261

gttaagaata aaatatatta agataaggaa aatttgtagt taagaataga aaaaaattat 60
ggttttgaag tatgagttat ttaaagaaag tggaaatatt tttagattat gtagtaaaaa 120
ataaagtgat tttttttttt taaatttatg taataaattg ataggtaata tgtgaaagtt 180
atagaatgta gattagagga tataataaat ttattttttt tatgtttata agaagtaaga 240
aaagttttga tgtgagttag tattgtttta taattttgaa ttgtgtagat tgtatgtatt 300
tttttttagt ttgaagtaaa tagtggatag gaaaaaatat taaatgttgg tagtaaatat 360
ggaaggaaat tataattaat gtaatatgtt aaaatatgtt atgtttattt tattaatttg 420
aattaaaatg taagaattta aaatgttttg gaaaaatatg ggtattgatt tgatgtttga 480
agttttaaaa tattatatat tttgaaatag tatttgtatt ttgaaatatt tgtttttata 540
tattttttaa aatttttttt tttttttatt ttatttatta ttaaataaag gatgaataga 600
tgtaatttag aaattgttaa gtatgttgaa gaaagattat tgtagaaaaa ttttttttag 660
tttttttaaa ggtgttagga agtagaaagg tgatatagaa ttggagaggt tggagttttt 720
gtattaattg tattaaatgt gaattttgag aaaatttttt ttaattatgt tttgtagtta 780
tatggatatg aagatttatg tgaattttga aagatgtgtt tatataagtt gaaatgtttt 840
taatgattta gttgatgtgt gtttttttat ttgttttttt tagagaggtg taatggaagt 900
tagaatattt tttttggaaa tttaatttgt tttgtagttt tttgaggaat tagtatttag 960
ttaatttggg ttgggagtag ttatttgtgg tgaggttgat tggttgggta ggaatagtgt 1020
tggggtgtgg gttgagtata gttgttttgt tttttttgtt ataggaagtt tgagtttatt 1080
tgagtagtgg tttttttaag tttaaagaag tagaggttgt tgtttgtttt ttttaggttt 1140
ttttattaaa gttggagtat ttttttttaa aattttatgt tttggtggtt gttttaagga 1200
gtgtgaggta ggggtatgta aagttgggag ttattatggg atagttttta agtgttaggt 1260
ttttagattt tttgaatttg gtttttatgg gagaagggtt ttttgaggtg tggatagtgt 1320
gaagtttttt ggtaagttta tggggattaa gtggggttag atttagattt aggagttttt 1380
ggagtagtgt ttaaattgta gtggtattgg attatgttgt ttggagtgtg tatagtttgt 1440
gtggtgtggg gatttgtttt ttgagtttgt gggtggtggg tgggaggaag tattgtttgt 1500
ggtgattgga attgggaggg agaattgtat tggtggtggg taaagtttag aatgtgttgt 1560
tagattttta attttgtttt tgtggagatg ttggagattt tgtgtatagg aaagtttttg 1620
tagtgtttat tgtggttaga gtagttgggg tattaatggt gggtgttttt ttttattgtt 1680
ttttggtttt gatgggggat tagaggttag ttttattttt agtgtgtttg aggtttatgt 1740
atttggttaa tgagttgtgg ttttttttta ggttgggatg gattttgaag gggattgtaa 1800
tggaggagta aagaagaaga atttttttaa attgaataat aaaaggtaat tagtttgttt 1860
tatttttata gtttatatag ttgtgagatt tgagtaattt atttttagtt tttagttttg 1920
aaataaatga tatgttgttg tttttaatta tttttaagaa atgtaagtta gtttttggaa 1980
ttaatatttt tgtttagagt agaagtttgt tggttgagtg gagtatagta tatgtatttt 2040
ttttgttttt tttgtttttt tttttaatga tatataatat tttatatatt tatgaaatgg 2100
ggtatatgga agtgtttttt atatgtttgg aatgtgtaat gattaagttt gggtatttga 2160
aggatatatt attttaggta tattttattt ttatgtgttg ataatatttt aagtttttta 2220
gttattttga aatatataat atattgttaa ttgtagttat ttttgtttgt tattgaatat 2280
tggaatttat ttgttttatt taattgtttt tagttattta ttaatttttt tttattttat 2340
tttttttattt tttttggttt tttttttttta gttttggtgt gttttttttt tagttttttt 2400
gttttagata ggtggatgtt tatatgtgtt tttgttttat gaatttttgt tttttaagtg 2460
gtgttggttg tttatatgtg agttatatgt tgttggtgat ttgttttgtg gtttaggttt 2520
ttgtttttgg taaatggtta tgtaaatatt gtgtttgtgg tttggttgat gagatagaag 2580
gttaaaagta tatttaggtt gttaattggt aataaatatt tgtatataat attggtaatg 2640
taattatata gggaaaataa ttatttaaag taaattttga ttatggtgtt ttgtttttat 2700
agaatattta aaattttatt aaatagattt attgttagta gtaaattgta aaatagatta 2760
gtaagtttaa taatattaga aattgtaatg taaattataa gataaattag ttaaatatat 2820
taatattata agaaattaag ttttttagtg taagagaaaa aatataaatg tggaaattaa 2880
atatattttt aaaaataatg ttaagtttga attagaaatt ttaatatgaa tt 2932

<210> 262
<211> 2932
<212> DNA
<213> Artificial Sequence

<220>
<223> chemically treated genomic DNA (Homo sapiens)

<400> 262

aatttatatt gaaattttta atttaaattt aatattattt ttaaaaatgt atttgatttt 60
tatatttgta tttttttttt tatattgaaa agtttggttt tttataatat taatgtgttt 120
agttaattta ttttataatt tatattatag tttttaatat tattaaattt attagtttat 180
tttataattt attattaata atgaatttat ttagtgaagt tttaaatatt ttataaaggt 240
agagtattat gattaaaatt tattttaaat aattattttt tttatatgat tatattatta 300
atattatata tagatattta ttgttagtta ataatttaaa tgtatttttg attttttgtt 360
ttattagtta ggttataaat gtgatgttta tatagttatt tattggaagt aagagtttgg 420
attatagagt agattattag tagtatatgg tttatgtgtg ggtgattaat attatttgaa 480
aagtagaagt ttataagata gaaatatata tgagtatttg tttgtttggg gtaggaaagt 540
tgagaaagag gtatattaag attaagggaa agaggttggg aagggtaaaa aggtgaaatg 600
aaaagaggtt ggtgaatgat taagaatggt tggataggat aaataagttt taatgtttga 660
tagtagatga gggtgattat agttagtaat atattgtata ttttaaagta gttagaagat 720
ttaaaatgtt attaatatat agaaatgaaa tatatttaag gtgatgtatt ttttaaatat 780
ttggatttga ttattatata ttttgggtat gtaaaaaatg tttttatgta ttttatttta 840
taaatatgta aaatattatg tattattaaa agaaagaata aaaaagatag ggaaaatgta 900
tatgttgtgt tttatttagt taataaattt ttgttttaag tagggatatt gattttaaag 960
gttagtttgt gttttttaaa aataattaaa aataataata tgttatttat tttagagttg 1020
gaggttagaa ataaattatt taaattttgt aattatgtaa attatgaaaa tgaaataagt 1080
tagttatttt ttattgttta gtttaaaaaa gttttttttt tttgtttttt tattgtggtt 1140
ttttttaaga tttattttga tttgaagaga aattgtagtt tattagttaa atgtatgagt 1200
tttaggtgtg ttggaggtga gattaatttt tagttttttg ttgaagttag agagtagtaa 1260
gagggagtgt ttgttgttga tgttttagtt gttttggttg tgatgggtat tgtaggggtt 1320
tttttgtgtg tggggttttt agtattttta tgaaggtaga gttgggggtt tggtagtgtg 1380
ttttggattt tgtttgttgt tagtgtgatt tttttttttg gttttagttg ttgtggatga 1440
tgtttttttt tatttattgt ttgtgggttt agagagtagg tttttgtatt gtgtgggttg 1500
tgtgtgtttt gggtaatatg gtttagtgtt attatggttt gggtgttgtt ttaggagttt 1560
ttgagtttag atttaatttt atttggtttt tatggatttg ttagaggatt ttatattatt 1620
tatgttttaa gaagtttttt ttttgtgaag attaagttta ggaaatttga aagtttgata 1680
tttgggaatt gttttatagt agtttttagt tttgtgtgtt tttattttgt gttttttgga 1740
atggttatta agatgtgaaa ttttggaaga agatattttg attttagtgg aaagatttaa 1800
aggaaatgaa tagtggtttt tgtttttttg agtttggaag agttgttatt tgaatgagtt 1860
taggtttttt gtggtaaaga gagtgaagtg gttgtgttta gtttatgttt tggtgttgtt 1920
tttgtttagt taattagttt tattatagat gattgttttt ggtttggatt gattgaatgt 1980
tgattttttg agaaattgtg aaataggttg aatttttagg aggaatgttt tggtttttgt 2040
tgtatttttt tagaaagggt aagtagagaa atgtgtatta gttgaattat tggggatatt 2100
ttaatttatg tagatatgtt ttttaaagtt tatataagtt tttatattta tataattata 2160
ggatgtagtt aagggaaatt tttttgggat ttgtatttaa tatagttaat ataaaaattt 2220
tgattttttt aattttgtat tatttttttg ttttttaata tttttgaaaa ggttaggaga 2280
aatttttttg tagtggtttt tttttagtat gtttgatagt ttttgagtta tatttgttta 2340
ttttttattt gatgataaat ggaatgaaag aaaaaggaag ttttaaaaaa tatatagaga 2400
taggtatttt aaggtataaa tgttatttta aagtgtgtaa tattttaaaa ttttagatgt 2460
tagattaatg tttgtgtttt tttagggtat tttaaatttt tatattttaa tttaaattag 2520
taaaataaat atagtatgtt ttagtatatt atattagttg taattttttt ttatatttat 2580
tgttaatatt taatattttt ttttgtttat tatttatttt aaattgagga aaagtatgtg 2640
taatttgtat aatttaaaat tgtaaagtaa tgttaattta tattagagtt ttttttattt 2700
tttatgaata tagaggaaat aggtttgttg tattttttag tttatatttt atgattttta 2760
tatattattt attagtttat tgtataagtt tagaagaaga aaattatttt gttttttatt 2820
gtatagtttg aggatgtttt tatttttttt aaataattta tattttaaag ttataatttt 2880
tttttatttt tgattataaa ttttttttat tttgatatat tttgtttttg gt 2932

<210> 263
<211> 7432
<212> DNA
<213> Artificial Sequence

<220>
<223> chemically treated genomic DNA (Homo sapiens)

<400> 263

atttgaagtt agagttattt agaggagaaa gagttggaat tgagaattta aggaatgttt 60
ggaagtgatt gggtttgagt ttatttagga agaaatagag gttggagata tgagattgtg 120
ttgttatttt tttttattaa tttttgggtt ttattgaggt tttattataa gtttggtttt 180

gtagtttagt gattaggaga aattagatat aagataataa taatagtaat gatttttttt 240
ttttttttga gatggagttt tgtttttttg tttaggttgg attgtagtgg tgtgattttg 300
gtttaatgta agttttattt tttaggttta tgttattttt ttgttttagt tttttgagta 360
gttaggatta taggtgtttg ttattatgtt tggttaattt tttatatttt tagtagagat 420
ggggttttat tgtgttagtt aagatggttt taattttttg attttgtgat ttgtttgttt 480
tggtttttta aagtgttggg gttataggta tgagttattg tgtttggtta atagtaatga 540
tttttgagta tttatattgt tagtttttat ggtaagagtt ttttttattt tttgttttgt 600
tttgttttaa gatagagttt tgttttgtta tttaggttgg agtgtagtgg tgtgattgtg 660
gtttattgta gtttttattt tttgggttta agttattttt ttgttttagt tttttaagta 720
gttgggatta taggtatgta ttattatttt tggttaattt ttgtattttt agtagggata 780
gggtttttta ttatgttggt taggttggtt ttaaattttt ggttttatat gatttgttta 840
ttttggtttt ttaaagtgtt gggattatag gtgtgagtta ttgtgttttt ttttgtattt 900
gtttaagtaa tatattgaaa tatgtattgt gttttttatt ttatggagga ggaaattgag 960
gttagtaaat gaggttgtta tgggaggtgg agataggatt tgaatttgtt ttagtgtagg 1020
aggtttaaga gtttttgttt ttttttaggg tattgtgtgg gagggtgaga aggagggagg 1080
tttatagagg tatgattttt gattgttatt gttatttggg ttttgttttt tgaagttttt 1140
gttaagtggg gaggtggttg ggggagggtt ttgttttgtg tagttttttt ttttttggtt 1200
tgtagagttg agtatagagg gatagaggta tggaattttt agaaatgttt ttttttagaa 1260
ataggtttta ggttttgttt gttttgtgtt tttgtgtgtt ggttttggtt tgtattgggg 1320
gtgagaagaa gtgggtggag ggatgtgggg tttatatttg gtgggtgtga gtgtggttgt 1380
gtgttttgtg gttttgtagt tatgtgagat atgagtatgg agtgtgtgtg ttttatggtg 1440
tgtgtatgta tgtgtgtgtt ggggagtgtg tgtgttggtg gttgagagtg aagtgtgaat 1500
gttatattgg tataaattgg gattatttgt gtgtgtgtat gtgtgtgtgt ggaagtggga 1560
gtatgtagtt gtggtaaaaa agtgtatgtt tgtgtgtata tgtgtatttg tgtgtatttg 1620
tttttttgtg gggtatgtgt gtgtaaaata tttgagtgtg tggatatgtg tgagggggtg 1680
agtgtgtgtt ggtgtgtatg tttgtgtttt gtatatgtat tttttttttt ttttttgaga 1740
tggagtttta ttttgttatt taggttggag tgtagtggta gtagtggtgt gattttggtt 1800
tattgtatta tttgtttatt tgttttaagg gatttttttg ttttagtttt tagagtattt 1860
gggattatag atatatgtta ttatgtttgg ttattttttt tttttgagat ggagttttgt 1920
tttgttattt aggttggagt gtagtggtgt gattttggtt tattgtaatg tttgtttttt 1980
gggtttatgt tatttttttg ttttagtttt ttgagtagtt gggattatag gagtttatta 2040
ttatgtttgg ttaatttttt gtatttttat tagagatggg gttttgttgt gttagttagg 2100
atggttttta tattttgatt ttgtgatttg tttgttttgg ttttttaaag tgttaggatt 2160
ataggtgtga gttattgtgt ttggttaatg tttggttaat ttttttatat ttttggtaga 2220
gatagggttt tgttatgttg tttaggttgg ttttgaaatt ttgattttag gtgatttgtt 2280
tgttttggtt ttttaaagtg ttgggattat aggtatgagt tattatgttt ggttatgtat 2340
tttatgttaa aatggattta tattttagat tagtattatt tagtagaaat ttaaattttt 2400
aatatagggt taggtatggt ggtttatgtt tgtaatttta gtatttttgg aggttgaggt 2460
gggtggattg taaggttagg agatttgaga ttattttggt taatagatgg gtaaaaattt 2520
atttttatta aaaatataaa aaattagtta tgtatggtgg tatgtgtttg tagttttagt 2580
tatttgggag gttgaggttg gagaattatt tgaatttggg aggtagaggt tgtagtgagt 2640
tgagattgtg ttattgtatt ttaatttggg tgatagagtg agattttgtt tgaaaaaaaa 2700
aaaaaattta atatgtatgt agataatgtg taaggtatta ttttatgtgt attgtatgta 2760
gtaattttta atttttatga taattttgag gtagatatta ttattttgtt ttataaaagg 2820
agaaatagtt ttggggagat aggataagtt attggttaag gtatatagtt agttatatgt 2880
ggtttttgtg tgatggttgg tttttgtagg tgaggttttg tttagatgtg tgggtagaag 2940
gtttggtttg gaaagaggaa ttgatagtaa ggttaagtta atgtttgttt ttgggggtag 3000
aaagttattt tttgtttttt ttttattgtt tatagaggta gtttagatag ggtgggggtt 3060
ataggagaat gaagggagaa gggggtagtt tttgggtagt aaaattaggt ggtgaaggga 3120
ggtattagag gatggtaatt agagaggtta ttagagggga attataggta gatagggtga 3180
taggagggat tattgatata aggtgaagag atggtttagt tggatggggt ggtttatatt 3240
tgtaatttta gtattttggg agtttgaggt gggtggatta tttgaggtta ggagtttgag 3300
gttttaatat ggtaaaattt tatttttttt aaaaatataa aaattagttg ggtatgatgg 3360
tagatgtttg taatttttgt tatttgggag gttgaggtag gaaaattgtt tgaatttagg 3420
aggtggaggt tgtaatgaga tgagattatg atattgtatt ttattttggg taatagagta 3480
agagattgat tttgttttat aaaaaaaaag aaaaaagaaa aaaaaaaaga gatggttgat 3540
ggttaaagag gggttagtgg ttaggggata tataagggta aaggtaggag gtaagaggat 3600
tggtaggggg ttgtttttgg gttattggga gtgatatagg atgagtatgg agggaaaggg 3660
agaaggggat tttagggttt tagtttattt aagttgtttt ttggttttat ttagtatgtt 3720
agttagaggt ttaggaagga attttgagtt ttttattaaa gttaaagatg agttgttgga 3780
gtttggtgag gggtaggatg aaggagttgt tggagatagt ggtgaatagg attagagatg 3840
ggtggtaatg gttttggtga gggtgtgttg ggttgggtgg tgggagggga tttttgggtt 3900
tgagggagga ggggttgggg tttggatttt tgagttttag ggaggaggaa agggtgggag 3960

tggggttgtg atttttaggt ttgggaggag tggagggtta gagttgagag taggaatttt 4020
taggttatag agaggagtgg ataaatgggg tagagaatat ttggggagag ttggggtttt 4080
tattgtgatg tttttttttt tgtaggagtt tgagtatttt ttggggtttt atgtagattt 4140
attatgatga ttatttgagg gatttgggtt tgtttattaa ggtttggttt tttgaattta 4200
aagatagttt tttgaagaag atttatagtt tgtgttttag gtttgtttgt ggggataagg 4260
attagggtta aaatgtttat aaaagttagg tgtggttgtg gtgggtgttt gtagttttag 4320
ttatttagga ggttgaggta ggatgatggt ttgagtttag gagtttgaga ttagtttggg 4380
taatatagtg agattttttg ggggtaaaat aaaaagaaaa aaaaaagttt atattttttt 4440
aataaataaa gttttatttg tgtttttgtt tggatttttt tttagtgtgg ttagaaaaaa 4500
atttatttta ttgtttttta ggaattaatg agtagaagag gtgatatttg atggggaagg 4560
aagagtaggg aggttgggaa gggtattaag gaataatatt ttattgtggg tttgtggaga 4620
atgggggatt ttaaggtgtg ttagttttag gagggtgagg gtaggagtgt gggtggagtt 4680
agtaggtttt tatgatggtt tttattgaga gttttgtttt tgttttttat aagttttgat 4740
tttattttta gtgggtattt agtattttta atttttttat agtttttaat ttagtttttg 4800
ttggaggtga atttttagag tgagggtttt ttgttatttg agagaaggtt ttttgtgatg 4860
tgattttggg ggatgttatt gttttttttg tttttattta ttttttttgt agttttgttg 4920
gttaggattt tggtttagat aaaggatggg ggttgtggtt gtggagtgga agtgggtttt 4980
aattattata aatttttttt gtgtttgttt ggagttggtg aggatagttt gttagagttt 5040
ggtaagaaag ggatttaggg tgtggggata ggggggtgtt agtagggaga gggtaaagat 5100
tgataaagta ggaattttaa gaggtataat attagaagtt tgtgttggaa ttatgattgt 5160
gtgtgtgtgt gtgtgtgtgt gtgtgtgtgt gtgtgtgtgt gagagagaga gagagggaga 5220
tggagttttg ttatgtagtt taggttagat ttaaattttt gggtttaagt aatttttttg 5280
ttttagtttt tttagtagtt gggattatag gtgtattatt atattttata aattatagaa 5340
tttagaattg tagattattt gagtttttgt ttagagttag ggtggttgag gtggggagga 5400
ttttttgagt ttaggagttt gaggatgtag tgagttgtga ttttgttatt gtgttttagt 5460
ttgggtgata gagaaatttt atttttaaaa aagagaaaga aaaagggata ggtataatgg 5520
tttatgtttg taattttagt attttgggag gttgaggtgg gtggattatt tgaggttagg 5580
agtttgatat tagttttatt agtatggtga aattgtattt atattaaaaa tataaaaatt 5640
ggttgggtgt ggtagtatat gtttgtaatt ttagttattt tagaggttga gataggagaa 5700
ttgtttgaat ttaggaagtg gaggttgtag tgagtttaga ttgtgttatt gtattttagt 5760
ttgggtgata gagtaagatt tagttttggt ggaaaaaaag aatgaaaaaa tttaaaaaat 5820
taaaaaagaa ttgtaggttg ggtgtggtgg tttatatttg taatttaaat gttttgggag 5880
gttaaggtaa atggattatt tgatgttagg agttggagat tagtttggtt aatatggtga 5940
aattttgttt ttattaaaaa tataaaaatt agataggtat ggtggtgtat gtttgtattt 6000
ttagttattt aggaggttga ggtaggagaa ttgtttgaat ttggaagata gaggttgtgg 6060
tgagttaaaa ttgtgttatt gtattttagt ttgggtgaga gaataagatt ttgttttgga 6120
aaaaaaaaaa gaattgtaga ttatttgttt gtgttttttt tttggggatt agattttatt 6180
ttttttttgt tgtatttttt tattttttat gtgtggatga tgatattgtg ttttgtgtat 6240
gttttttgtt attaaaagtg ttttttttat agagtaggtg agaatttagt gaggagatgt 6300
agggatatga ggtttgattt agggtagagt tttaaggtaa tatatttgat ttattgtagg 6360
tttttaatgg tgtttgtaga gtattttttt gtattgattt agttttagta aagggtagag 6420
gttttgttgt gttttttgtt gggtttagaa ttgtttaggt gtttaagaaa gttttttagg 6480
ttgggtttag tggtttatat ttgtattttt agtattttgg ggaggttgag atgggaggat 6540
tgtttgagtt taggagtttt agattagttt gggtaataaa ataagttttt tatttttata 6600
aaagaataaa aattagtagt tgggtatggt ggtttatgtt tgtaatttta gtattttggg 6660
aggttaaggt aggtaaatta tttgaggtta ggagtttaag attagtttgg ttaatatggt 6720
gaaattttat ttttattaaa aatataaaaa ttaggtgggg tatagtggtt taagtttgta 6780
attttagtat tttgggaggt taaggtgggt ggattatgag gttagaagtt tgagattagt 6840
ttggttagta tggtgaaatt ttatttttat taaaaatata aaatattagt tgggtatggt 6900
ggtaggtgtt tgtgatttta gttttttggg aggttgaggt agaagaattg ttagaatttt 6960
ggaggtagag gttgtagtga gttgagaata tgttattgta ttttagtttg ggtgatagag 7020
tgagatttta ttttaaaaaa tatgaaaata aaaattagtt gggtggtggt gggtgtttgt 7080
aattttagtt attggggagg ttgaggtagg agaattgttt gaatttggga ggtgggggtt 7140
gtagtgagtt aagattgtat tattgtattt tagtttgggt aatagagtga gattttattt 7200
taaaaaagaa aaaaataata attaaaatgt taaaattagg agtagaatta tagaatgttg 7260
gaaagtgagg tttaagaagg gggttgtgtt taagtttatg tatgggaaat ttgattggga 7320
tattgagttt atatagagta ggattttagt tttttttatt agagtggggt gtgattatag 7380
gaatagttgt ttttagttag tttgttatat gatatttttt taatgtttta gg 7432

<210> 264
<211> 7432
<212> DNA
<213> Artificial Sequence

<220>
<223> chemically treated genomic DNA (Homo sapiens)

<400> 264

tttggaatat tgagggggtg ttatgtggta ggttgattgg aggtggttgt ttttgtggtt 60
atgttttatt ttggtggggg ggattgagat tttgttttgt gtgagtttgg tgttttagtt 120
aagtttttta tgtatggatt tggatatagt tttttttttg ggttttattt tttaatattt 180
tgtgatttta tttttgattt taatatttta attattattt tttttttttt tgagatggaa 240
ttttattttg ttgtttaggt tggagtgtag tggtgtaatt ttggtttatt gtaattttta 300
ttttttaggt ttaagtaatt tttttgtttt agttttttta gtagttggga ttataggtat 360
ttgttattat ttggttgatt tttgtttttg tattttttga gatggagttt tgttttgtta 420
tttaggttgg agtgtagtgg tgtgtttttg gtttattgta atttttgttt ttagggtttt 480
agtaattttt ttgttttagt tttttaagga gttggaatta taagtatttg ttattatgtt 540
tggttaatat tttgtatttt tagtagagat ggggttttat tatgttggtt aggttggttt 600
tgaatttttg attttgtgat ttgtttattt tggtttttta aagtgttagg attataggtt 660
tgagttattg tgttttattt gatttttgta tttttagtag agatggggtt ttattatgtt 720
ggttaggttg gttttgaatt tttaatttta agtgatttgt ttgttttggt tttttaaagt 780
gttggaatta taggtatgag ttattatgtt tagttgttaa tttttatttt tttgtagaga 840
tgggagattt gttttgttgt ttaggttggt ttggaatttt tgggtttaag tgattttttt 900
attttggttt ttttagggtg ttgggagtat aggtgtgagt tattgagttt agtttagaag 960
gtttttttga gtatttaaat agttttgggt ttagtaggga atatagtaaa gtttttgttt 1020
tttgttaagg ttgagttagt gtagggaggt gttttgtaga tattattaag gatttatagt 1080
agattaaatg tgttatttta gggttttgtt ttaagttaga ttttatgttt ttgtattttt 1140
ttattgagtt tttatttgtt ttatgagaga ggtatttttg gtgatgaaga atatgtatag 1200
ggtataatat tattatttat atgtaggaga tgaggaagta tggtagaaag agggtgagat 1260
ttgatttttg gagaaagaat ataagtaaat ggtttataat tttttttttt tttttaagat 1320
aaggttttgt ttttttgttt aggttggagt gtgatggtgt aattttggtt tattgtaatt 1380
tttgtttttt gggtttgagt gatttttttg ttttagtttt ttgagtaatt ggaaatataa 1440
gtatgtatta ttatgtttgt ttaatttttg tatttttagt agagatgggg ttttattatg 1500
ttggttaggt tggtttttaa tttttgatat taagtgattt gtttgttttg gttttttaaa 1560
gtgtttggat tataagtgta agttattatg tttagtttat agtttttttt tagtttttta 1620
aattttttta tttttttttt ttgttaagat tgagttttgt tttgttattt aggttagagt 1680
atagtggtat gatttgggtt tattgtaatt tttgtttttt gggtttaagt aatttttttg 1740
ttttagtttt tggaatagtt gggattatag gtatatgtta ttatatttgg ttaatttttg 1800
tatttttagt atagatgtgg ttttattatg ttggtaaggt tggtgttgaa tttttgattt 1860
taagtgattt atttgttttg gttttttaga gtgttgggat tataggtatg agttattgta 1920
tttatttttt tttttttttt ttttttagaa atggggtttt tttgttattt aggttggaat 1980
atggtggtaa gattatagtt tattgtattt ttaaattttt gggtttaagg gatttttttt 2040
atttagttta ttttaatttt aagtagaagt ttaaatagtt tatagtttta aattttgtga 2100
tttgtggagt gtggtggtgt atttgtagtt ttagttattg gggaggttga ggtaggagga 2160
ttgtttgagt ttaggagttt gagtttagtt tgggttatat agtgagattt tatttttttt 2220
tttttttttt ttatatatat atatatatat atatatatat atatatatat atatagttat 2280
ggttttaata tgggttttta atattgtgtt ttttaaaatt tttgttttat tgatttttgt 2340
tttttttttg ttgatgtttt tttgtttttg tattttgagt ttttttttta ttagattttg 2400
gtaggttgtt tttattagtt ttggatgggt atagagagga tttatagtgg ttgagattta 2460
tttttgtttt atagttataa tttttatttt ttgtttaggt taaagttttg gttaatagaa 2520
ttgtggaggg ggtgggtggg gatagaaagg gtaatgatgt tttttaaggt tatgttatag 2580
ggaatttttt tttaagtgat agggaatttt tattttgaga atttgttttt gatagaggtt 2640
gggttggggg ttgtggaggg gttggaggtg ttgggtgttt attgggaatg gagttagagt 2700
ttgtaggaga taagggtgaa gtttttagtg agggttatta tggggatttg ttgattttat 2760
ttatgttttt gtttttattt ttttgaaatt gatatgtttt gaagtttttt attttttgta 2820
agtttataat agggtgttat tttttgatat tttttttgat ttttttattt tttttttttt 2880
attaggtgtt atttttttta tttattgatt tttgggaggt agtggggtgg gttttttttt 2940
ggttatattg gggaaggatt tagataggga tataggtgag attttattta ttggagaagt 3000
atgaattttt tttttttttt ttgttttatt tttaagagat tttgttgtgt tgtttaggtt 3060
ggttttgaat ttttgggttt aagttattat tttattttag ttttttgagt agttgggatt 3120
ataggtattt gttataatta tatttggttt ttatgaatat ttttaatttg gtttttgttt 3180
ttatagataa gtttggggta taggttgtgg gtttttttta agaggttgtt tttggatttg 3240
aggaattagg ttttggtgag tggatttagg ttttttaggt ggttgttata gtaggtttgt 3300
atgaagtttt ggaaggtgtt tgggttttta taggagagag gatattatag tggaggtttt 3360
agtttttttt aggtgttttt tgttttattt atttgttttt ttttgtgatt taggagtttt 3420

tgtttttagt tttaattttt tatttttttt agatttaggg gttatagttt tatttttatt 3480
tttttttttt ttttgagatt taggggttta ggttttagtt tttttttttt tagatttagg 3540
agtttttttt tattatttag tttagtatat ttttattaga attattgtta tttgtttttg 3600
gttttgttta ttattgtttt tagtagtttt tttattttgt tttttattag gttttagtga 3660
tttattttta gttttggtgg ggggtttagg gttttttttt gggtttttgg ttggtatgtt 3720
aggtggaatt agagggtaat ttgggtaggt tgggatttta gaattttttt tttttttttt 3780
ttttatgttt attttgtgtt gtttttggtg gtttaggggt agttttttgt tagttttttt 3840
gttttttgtt tttattttta tgtgtttttt gattgttaat ttttttttaa ttattagtta 3900
tttttttttt tttttttttt tttttttttt ttatgagata gagttagttt tttgttttgt 3960
tgtttagggt ggagtgtagt gttatgattt tgttttattg taatttttat tttttggatt 4020
taggtaattt ttttgtttta gttttttgag tagtagggat tataggtatt tgttattatg 4080
tttggttaat ttttgtattt ttagaagaga tggggttttg ttatgttggg gttttgaatt 4140
tttgatttta agtgatttat ttattttggg tttttaaaat gttgggatta tagatgtgag 4200
ttattttgtt tggttgggtt atttttttat tttgtgttag tagttttttt tgttattttg 4260
tttgtttgtg gttttttttt aatggttttt ttaattgtta ttttttgatg ttttttttta 4320
ttatttgatt ttgttgttta ggaattattt tttttttttt ttgttttttt gtgattttta 4380
ttttgtttga gttatttttg tggatagtgg agggagagta ggaggtgatt ttttgttttt 4440
aggggtagat attggtttag ttttgttgtt agtttttttt tttgggttag attttttatt 4500
tatgtatttg gataaagttt tgtttataga gattagttgt tatgtggggg ttatatgtag 4560
ttagttgtgt gttttggttg gtgatttatt ttgttttttt aggattgttt ttttttttgt 4620
agaatggggt aataatattt attttagggt tattgtgaga attaagagtt attatatatg 4680
atgtatatgg aatggtgttt tgtatattgt ttatatatgt gttaaatttt tttttttttt 4740
tagatggagt tttgttttgt tatttaggtt ggaatgtagt ggtgtgattt tggtttattg 4800
taatttttgt tttttgggtt taagtgattt tttggtttta gttttttgag tagttgggat 4860
tataggtgta tgttattatg tatggttaat tttttgtatt tttagtagag atgggttttt 4920
atttatttgt tagttaggat gattttaaat tttttgattt tgtgatttat ttgttttggt 4980
ttttgaaagt gttgggatta taggtatgag ttattgtgtt tggttttgta ttaaaaattt 5040
aaatttttat tggataatgt tgatttagaa tatgatttta ttttaatata aagtatatgg 5100
ttgggtgtgg tggtttatgt ttgtaatttt agtattttgg gaggttaagg tgggtggatt 5160
atttgaggtt aggattttaa gattagtttg ggtaatatgg taaaattttg tttttattaa 5220
aaatataaaa aaattagtta ggtattggtt aggtgtagtg gtttatgttt ataattttag 5280
tattttggaa ggttgaggta ggtggattat gaggttagga tatggagatt attttggtta 5340
atatggtgaa attttgtttt tagtaaaaat ataaaaaatt agttaggtgt ggtggtgggt 5400
ttttgtagtt ttagttattt gggaggttga ggtaggagaa tggtgtgaat ttgggaggtg 5460
gatgttgtag tgagttaaga ttatgttatt gtattttagt ttgggtaata gagtgagatt 5520
ttgttttaaa aaaaaaaaat agttaggtat ggtggtgtgt gtttgtagtt ttaaatattt 5580
tgaagattga ggtaggagaa ttttttgaaa tgggtaggtg gatgatgtag tgagttaaga 5640
ttgtattatt gttattattg tattttagtt tgggtgatag agtgagattt tgttttaaaa 5700
aaaaaaaaaa aaatgtatat gtaaaatata gatgtatata ttagtatata tttatttttt 5760
tatatatgtt tatatattta aatattttgt atatatatat tttatagaga gataggtgta 5820
tataaatata tatatgtata tagatatgta tttttttatt atgattgtat atttttattt 5880
ttatgtatgt atgtgtatat atatagatga ttttagtttg tattaatgtg atatttatat 5940
tttattttta gttattgata tatatatttt ttgatatgta tatgtatatg tatgttatga 6000
aatgtatata ttttgtattt atgttttatg tggttataga gttataggat atatagttat 6060
atttatattt attaggtgtg ggttttatat ttttttattt attttttttt atttttaatg 6120
taggttagga ttagtatgta gaggtatagg gtaggtaggg tttggagttt gtttttgagg 6180
agggatattt ttgggggttt tgtgtttttg tttttttgtg tttaattttg tgggttgggg 6240
gaggggaggt tgtatagagt agggtttttt tttggttatt tttttgtttg gtagagattt 6300
tagagagtag ggtttaggtg atagtggtaa ttagaggtta tgtttttgtg ggtttttttt 6360
tttttatttt ttttatatag tgttttgaga gaagatagag gtttttgagt tttttgtatt 6420
gaggtaggtt taaattttgt ttttattttt tatgatagtt ttatttgttg gttttagttt 6480
ttttttttat aaagtgggag gtatagtata tattttagta tattatttga ataaatataa 6540
agaaaggtgt agtggtttat gtttgtaatt ttagtatttt gggaggttga ggtgggtaga 6600
ttatatgagg ttaggagttt gagattaatt tggttaatat ggtgaaaaat tttgttttta 6660
ttaaaaatat aaaaattagt tagaagtagt gatgtgtgtt tgtaatttta gttatttggg 6720
aggttgaggt aggagaatgg tttgaatttg ggaagtgaag gttgtagtga gttgtgatta 6780
tattattgta ttttagtttg ggtgatagag taagattttg ttttgaaata aaataaaata 6840
aaaaatgaag aaagttttta ttgtggagat tggtaatata ggtgtttaaa gattattgtt 6900
gttggttagg tgtggtggtt tatgtttgta attttaatat tttgggaggt tgaggtaggt 6960
ggattatgag gttaggagat tgagattatt ttggttaata tggtgaaatt ttatttttat 7020
taaaaatatg aaaaattagt taggtgtggt ggtaggtgtt tgtagtttta gttatttggg 7080
aggttgaggt aggagaatgg tgtgaatttg ggaggtggag tttgtattga gttgagattg 7140
tgttattgta gtttagtttg ggtgaaagag taagattttg ttttagaaaa aaaaaaaaga 7200

ttattgttgt tattattatt ttgtgtttaa ttttttttag ttattgggtt gtagggttag 7260
gtttgtggta gggttttaat agggtttaag ggttgatgag aggaaatagt aatatagttt 7320
tatgttttta gtttttgttt ttttttaggt gggtttgagt ttgattattt ttaagtattt 7380
tttgagtttt taattttaat tttttttttt ttgggtgatt ttggttttag at 7432

<210> 265
<211> 12423
<212> DNA
<213> Artificial Sequence

<220>
<223> chemically treated genomic DNA (Homo sapiens)

<400> 265

tttatttgag ttttttttgt tgtagagaaa gtttttttgt gattttattt ttttttttaa 60
atgttgggaa atgtttttga gttttgtttt tgatatatat attttttttg ggttgaggga 120
tggggattgg gatatatgtt ttgggaaggg taggtgtaga gtgatggggt tgatggggtg 180
ggggaagtag ggggaggagt tgtttttttg aggtttttgg gaattggttg tgttaggtat 240
ttttagtttt ttagttaagt aatatagaga gttaaaagag gttattagag ataagaaggt 300
ttttatgggg ttattgtttg tgggggatgt tagtagtggg gaggagtgaa gtaggaagga 360
attgggagat tggatggaat tgtttttttt atatggttat atatagttat atatggttat 420
tgggattaga gttagttttg tttgggtagt taggttttta gtgtaagtta ttagtttatt 480
tggaggggat tagtgttttg gttaataagg ttagttggtt tttttttagt tggagtttat 540
ttaattttgt taggaagtta gagagttgga taagtggagt ttagtttggg gaggtggaag 600
agagggagga tggagtatgg tgaagtatag gtggtttttt tggtagtttt agttttggtt 660
ttggaatagt ttgggtagtg tgttaatttt ttttgttatt gttgttttat tgatttttat 720
gaatgagttg tgaggtagtt atttttagtt ttttatggat aaatatttga ggtttagaga 780
gggtaagaga tttgtttgtg attttttagt atttttgttt ttttttgggg ttttgagggt 840
ataataaaga tttttaaggt tttttttttt tgtaggaggt ttaggtgtag ttgtggggga 900
gggtgttttt ggtgtttttt gtttttgtag ttagtttgtt ttttatttgg tagttttttt 960
tttttttttg ggatgagatg tgtatgtgat gatgggattt tgtaaatggg ttgggaagaa 1020
ggagttattt ttttaaaggg ttagtttttt taggagtgtt tgaattttta aagggggtag 1080
ggttttatat aaattttatt tagaagtagg attataagag tttttgtagt attttttttt 1140
ttttgttggt tttttttgag tgtgtatttt gttttattag tggaatattt gtgggttagg 1200
gttattggag atagggttat attgtttggg tggttattta agtaatttta ttattttgag 1260
tatttttttt ttttatttga aaggtggggg gttatttatt tagtttttag ggttgttaag 1320
gtgattaaat gggataatgg ttgtattatt tttgagtaaa gggtattata gatgggaaga 1380
ttattttagt tttgtaggga gttagatggg ttgtgaggga ggggtaaagg tagggttgga 1440
gggggttgga ggggttggtt gagtagggta tatatggggg gggttttttt ttaaatttag 1500
aaaaattttt agttaggttt agtgtttttg ttgagaaatt gaggtttaaa gaaaggaatt 1560
ttttgttttt ttgttttttt ttttttgttt gaggttattt atttatttaa taattttgtg 1620
tttgatatta gataaggttt tagtgagtaa aatgaatata gtttttttgg gaatatagag 1680
tttagatagg gagatgatta ttttattatt gtaaatataa atatataatt ttagtttggg 1740
ttaagtgttg tgaagtttta gatttgtgtt tggtttggtg ttgtatttta agtattttgg 1800
tatttaagtg gtattgaaat atttgttgga aagaaggtta ttttgaggag gtgatgttag 1860
agttgaggtt tggagggttg gagttaaata aagatggttt taggtttttt ttgttttttt 1920
tgtaggtttt gtttttgttt tttttttggt atttaggtta agggtaagta tttttgattg 1980
gttattagtt ttaatatgtt gagtataggg attagatttt gttttgtgtg gttggttttt 2040
tttaggtttt gaatttaggg aagtgtatgt ggattttaat ttatttagat ggtgtgtttg 2100
tgtttgtttt ttttttgggt atgttttaga atagagttta gtttagagtt taggggtggt 2160
tgggatgatg gatgagggtg tggggtttgt tgattattag gggatttgat tattgggttg 2220
ggttagtttt tttgttttta tttttttttt tgtaaagaag ggtgttgggg tgtttttatt 2280
ttttgtgtta gttttggggt ttttattttg gtgggaaggg taggttgatt tggtagattg 2340
tggttttttg ggagggaaga aggtgttaga tgtgtggagt aattataaat agtttttttt 2400
ttttagaaga tggtatgggg tttaagattt aggtgttgta tatttagaat gagagtagag 2460
atttttgtta ggaaaaaaag gtatttaggg gatttgttta ttagtatgaa atgtaaatga 2520
gtttgtttgg ttttatttat ataattttgt gtatggattt ggtgaaaggg taattaggga 2580
gatgatggtg tagtagttat tttgttattt tttttatttt ttttttttat tggttggggt 2640
gggaattgag atgattttaa ttttttgtgg tggtgggagg tgtgtggggg ttgtgtgggt 2700
ggtgtagttt taggagagtg aataatgttt aggggtgatg gttttagtaa agtgaggggt 2760
ggtgatggag gttatttgat ttattttgtt gtttttttgt agtggtgtaa gtgttttaaa 2820
atttttggag agggaattga ttgatttatt aggttttgtt gtgtttattt tttgtagatg 2880

ttggggaagt gttttttggt gtttaatttt tgttgttttt ttttttattg gtgtttagta 2940
tatttgtggt gttttgtttt tgggtattta tagttttttg gagtggtgtt ggtggtagga 3000
gagagttaga gttgattgag ttttagaatt tggggaggta ggtggggtaa gggttggggt 3060
ttgtgtgtgt gtgtgtatgt gtggaaagta taaatttatg agggtttgtt gttggtaggg 3120
ttaaggtgag tgagtgaagg tggtagattt gtgaagtttt agttttgtgg atattttttt 3180
tttaagttat atagggattt aagagaaatt gaggtatgaa gttggggggt ggtggtggtg 3240
gggatttgga aggttgagtt gataaagttt agttttgtgt ttttgttttt ttttatatat 3300
ttgagatttt tataggtttt tttttagggg tttttgtttt gttttgtagt tttttttggg 3360
gatttgggtt ttgtggttta gtgtgatttg tttggggtta tgtgtttaag tatgaagttt 3420
ttgtgtggag tttatgtttt ttaaaaggtt tttaggggta aagggatgaa gtttaggagt 3480
gttaatgtga ggttaggttt agtgggttag ggttatgagg ttagggttga agttttagaa 3540
ttgatttggg agtatgatta ataagtttaa gagatttgtg gaggttatgt ttagattatg 3600
tttattttag ttttttttga gttttgtttg tttttttaag attgggtttt tttagtttga 3660
agttttattt ttaggatatt tagttttgtt tttttttttt tttagttttg tagttaagtt 3720
taattttagg gtgggagatt aattaggtgg agatttggga ttttgttttg ggattttttg 3780
gtgataattt ttatttttgt gttgtttgtt tttttttttt tttaggtttg tttagagttg 3840
agttttgttt tttggttgtt gtttaaatta ggggttgtgg ataaaggatg tttggggttt 3900
gtggttttat gttaggattt tgtgttgaat attttgattt tttgggtttt ttttaaggga 3960
gttttaaaga ttttaatggt taataggaaa gtgggtttgg tttgggtagt agtttgattg 4020
gttttagttt ttgggagtgg atttaggggt aaggggaggg gagaggggtg gttttgggtt 4080
ttggggtggg aattggattt tagttgtggt tttttttttg tgtttttgtt tgtattgtta 4140
tggtggatgg ttaatgggtg tgtggtttgg ggttggtggt gtttggtgat tggttgtggg 4200
gttgtttggg ggtggggttg aggtttgaag ttgaagtgag ggatttagtt gtggtgtgtg 4260
tggggttttt tgttgttgtt tttgtttgtt tgttttgtgt tttggttgga ggaggaggtt 4320
gtggtgttgg tgatagttat ggtagtggta gttattgtgg tggttgtggt ggtggtattt 4380
ttgtttttat ttttgtttgg gattgttttt tattgttttt ttgttttttt tggatagtga 4440
gtttgtggtg gggtggggga aggagttgtt tttattttttt ttaagtttat ttttaaagag 4500
attttttttt tttttttttg ttgtttggtg tggagttggg atgatgttga ttttttagat 4560
ttggttttag ttgtgttgtg ggaagagggg gtgttttttt ttggattttt gttttttgtt 4620
gttgtttttt tttttgtttg tttttttggg gtggttttgt tgaaggtagt gttgaatttg 4680
gtaattggag tttgggtgtg aagtgaagaa gttggaataa agtgaggggg agttggttgg 4740
ttggtttggg aagttttagg ggtgtagggg aagtgggatt tgtgttgggt ggggtttttt 4800
tgtgttttgg tgttttgtgg gtagtatgtt tttgtgggta gggggagttg ggttgaattg 4860
gtttttttgg gggtttagtt tgtgttttag agtttattag atgtgttttt gttggggttt 4920
ttgggttgtg tgaggatatt ttttttgagg ggtgttgttt gttttttttt ggattgtttg 4980
gggttttggt tggttagagg atggatgagg aggaggatgg agtgggtgtt gaggagttgg 5040
gatagttttg gagttttatg tggtttaatg atttgttggg ggttgggggt ggttggggtt 5100
ggggttagta gaaaaggatt tgggtagtgt ggattttgag gtggaggggt tgttgtattt 5160
ggtgttggtt ttggtggttt ttttttattt gagttaggat agttgtttgt ggagttggtg 5220
tttttgtatg gtttgtaatt tatatttttt ggggtatgat ggttaggtgt ggggttagaa 5280
gggggatggg ttgtattgtt gggggttggg ggggaagaag atttattgtt aggtgagttg 5340
aagtgagttt ggagggtagg tggatggggg gggggttgtt agggagggga gggggtatta 5400
gagtgggagt ggagatgtga gtaggttttg ttggtaattt gggtttattt tatttgtgta 5460
tatatatttt agtttttttg ggttgggggg gtggggattt aggttaggag aagagagttg 5520
tgttttgttg gtttgtagtt ggatgttttt tagatgtggt taggggaggg ttgttatttt 5580
ttagatgtgg gaagttttgg gagtttggga gttgtatttt gtttgtgttg gttagtgagt 5640
tgggtttggt ttttgagttt ggtggggggt gggtgggggt ggtggggagg aagttgtggg 5700
gatggaggag gggggattgt aattttttgg gttttttttt tttttttgtt ttaaagtttg 5760
tggtggattt tagggtttga ttttttaatt tggtttttga ggttgaggtt tttattgatt 5820
tagaaggaga tgttgggggg tggggattag gttttggttt attttatttt ttattttgtg 5880
ggttggaggt ataataagga gattttggtg gtggttgatg ttaggggtgt agaatgagaa 5940
taagatgtgg tggaggggag ttgtttgttt ttggagttgg gagtggagtt tttttttgtt 6000
agagtttagt gttgtgggtg ttttttattt gatttttatt tgatgtaggg tgtttaagat 6060
tgggttgggg agttttatgt tggatttttt ttgtggaggg aaagattgtg ttaagagttt 6120
ttttagtaag tgggggatgg ggttaagttg gtttaggtta tttttttttt tttataaata 6180
aagagtgttt ttgtagatta ggtatggggg attgtaggtt tgtgaaagtt ttgaggggat 6240
agtaatattg ttagtgtgtt tattaaggag gatttgggtt tattatttta gtgtagattt 6300
tgtagatagg tttggggaat gtaagtttta tattttaggg agatggattt agtattgttt 6360
tttgtttgtt taggttggag tggtagggga gattttagtt tagttagatt ttagtttaaa 6420
gtgttatttg atagatgttt ggttttaagt tttttggtgg gttttgttat tggggaaagg 6480
aaaggtggag gggaggtgtt gttgttattt taggtttgga tagttttttt gggttttttt 6540
tttttagttt agaggttttt atagatttag ggtgggagat tttaggtttt ttttgtagtt 6600
tttgtgagtt tagtagggat tttgttttgg ggaagggggg tggaaggttg gtgttgtttt 6660

tattttttgt agttttgttg tttttagtgt gtaaagtagg gatgagtttg tggttttttt 6720
attttatttt tttatatttt gtgggttttt aggaagtttt ttttattata tttttttttt 6780
atttattgtt tttttttttt ttttttttga gttgtagaaa gagaggtgta ggttttagag 6840
gtttgtgttt ttgttgaata ggtataattt tgaggtttga gtttttagtt ttaggttttt 6900
tttttttagg tagggatgga gatggagaat tgatatggtt ggaaaggatg ggttttgagt 6960
aatttttttt tttgttttta attatttatt ttttttatga atgggaaaaa gtattaattt 7020
ttatgttttt ttttttaagg gttttagggg aggtagagag agtttttttt tttttttttt 7080
ttgaaaatag atttttaggt tagtattttt ttttgtttgt tttattttta ggtgttagtg 7140
gagggtttgg aggtagattt ttttattgag tggtttggat gggggtaagt gttgaggttt 7200
ggtatggggg aggagaggtg ggaggatagt atttattttg ggagttgggg agtagttttt 7260
tttaggggag tggtagttgg gtgtgtggta ttagtattat taggtttgga gaagtttggg 7320
ttggttgagg atgggttgag aataattttt tggtagggga ttgtttaagt tgtgtttttt 7380
attattgtta ttttatttaa gtattaagag atatttgtat ttttgttgtt agaggggttt 7440
tgaattgttt ttagggtttt tttttttttt tttgtggtat ttgtgatttt ttataggttt 7500
taggagggtt tttgtatgtt tttgagttgg agggatataa ggatattttt ttttattttt 7560
aattaattga agttagttag gttttttttg gtatattttg ttttttggtt tttttttttt 7620
aagggattag gttttttaga gaggagattt agatttaggt aggaagtgtg tgtgtgtgtg 7680
tgtgtgtgtg tgtgtgtgtg tgtgtgtgtg tgtgttgtaa gggaggatag taaaaggttt 7740
agaagtgtaa gttttggtgt ggagagtagg ggtattggta gatagttatt tttgttttag 7800
attagagggg agtatgttgg tttttttagt atgtgttttt gtaagtgtga gggagtgtgt 7860
tttttttttg ggaggagttt tttttgtatt tttttttttgg ttgttgtgtt atatttggaa 7920
gtttgtgtag gagttttttt aagtttagag tttagtatag ttttttttta tttggaaatt 7980
ataggttggt atttgggatg ttgagagagg gggagtggga ggggggttag agggggaaga 8040
ggggagtttg ttaggagttg ggggaggaga ggggggggag gtttgaatgg taggagtttt 8100
aatggttttt agaagtgagt gtgagtgagt gattgtgtgt tattttgtgt gtttgagtgt 8160
gtttttttag aggggtgggg gtataggagt ttttggagtt ttttttgtta tattgtttta 8220
atgttttaag tttgtttatt ttttttattt agggagaatt ttttattttt gtttttagag 8280
gaaatggtgg ggggagtgtt ttttttgttt ttttattaat tttttttttt aatttaattt 8340
ttaaatttta agtttattag aggagatttt ggggagaagg gttggggttg gagaattgaa 8400
tgggattgag ggggggttag attatttttt ggtatgtttt ttgttttagg tttatttttt 8460
ttttgggtta tatatatatt tttttttatt tgttttgttt tattgttttg ttgggggatt 8520
ttttattatt tgtagtgtta gttggagtta tttttgggat tatttttatt atatagtttt 8580
ttttattttt gtttgttttt gtttagtatt tattttgttt tgtttgtttt agttaggatg 8640
gtgatagata taattgttta tatgtagata tgtgtatgtg tgtgtatata tatatgtata 8700
tatatatata gagttataga tgtttgaatt agatttttgg tttatttttt tttttaaaga 8760
ttttagtggg ttgttttttt tgtgtttttg tgttttatgt ttttgttttt gggtggagtt 8820
ggttgttttt ttgtgagttt tagtttttaa tttagttgga agtttttttt tttttatatt 8880
tatgtattga agtgtggtga ggggggaatt atagggaaat aggtttgtgg ggtgaggggg 8940
gggggttttg ttgggggaat ttttttttga gttttggagt agggtggagt ttttatattt 9000
tatgttattt taggggttgt tgaatttgat agatattagg agtttttttg tgggatgggt 9060
gaggtaagga tttatttttt tattttttta tttattagaa ggttaggatt tttgggttta 9120
ggtttatttt ttttttaatg ttttttagtt ttatattttt aggtttttag gtgggaatgg 9180
ggttgttgga gggtttgttt gtttttttag tttatgttta gagtttttgt gtttttagag 9240
gtgttttagt tatttttttt ttttttttat tttttttatt tttgtggttg ttaggttaag 9300
taagggtttt atttagataa agttttatat agaggattta tttgtggaga taatatgttt 9360
tagttttttt ttttaggagg gaggaaagga ggagggatgg aagtggggtt tttttttttg 9420
ttgttttttt tttttttgtt tttgatggtg ggttgtgggg agaagaaaga tttttttttt 9480
ttttattttt tttttgggat ttattttagt ttggatagtt attatagtaa tagtagatat 9540
gtgattgtat aggtttttta tagttattat ggtaattagt gttttttgtt tttttggtta 9600
ttatggggat ttgttatatg gttttttttt aatttgataa tttgtgggtt tttttgattt 9660
tttttttttg tataaggata agataatagt tttagttagg ggagattatg tgagaggggt 9720
ttttttaatt attatgataa taaatttata ttatttggtg atatattttt ttgttgttat 9780
ggtaatagaa gaatattata tagtgatata ttgttatgtt tttaaggaaa taagttttat 9840
tttgagaagg gtgaaagatt ttttttttta ttaattattt ttttatttag agtgttttaa 9900
attgttgtgt gttttggttt tttttttgtt tgtttatgtt tatgtgttgt ggaaggagag 9960
ttgtataggg tgggtaggat gttatttgga agtaggttga attgttaagg gatttttaag 10020
aaggggttga tatgattaag agttgtgtat gaggttattt gggggtaggt tttgtttgta 10080
agaggttttt agtttgagtt tgggtaaggt ggggatagaa agaggggtaa tttttttgtg 10140
aggttatttt tgtatttttt tttagttata agtagtttat ttttgttaag ggaaataagg 10200
taggaagatt tgaatgggga tatatatttg gtttttaggt agggagattt attgattggt 10260
tgatttgttt atttatttat ttagtttttt taggtattga ttatttgttt tgtgttaggt 10320
attatagtaa gttttgtagg ggatttaaag atgtattaga atgtttttgt ttgttaggag 10380
ttttagttta gtaggggagg ttgataagtg tatagattat taatatggtg gaattttgtt 10440

attagagggg tttaagtaga tgttttggga gttttttttt tttagagatt tattgttttt 10500
atatagagat atttatttat tggggttttt tttagatttt tttttttttg tagaggagag 10560
gaggggattt gtatggggaa agtagattag tttttgaggg taattgtgtt tttttttttt 10620
taaatgatgg tttttttttt gttttttttt ttttagggtg attttggttt gaattttatt 10680
ttggggagag atttattgta ttttggtttt atttagaaaa ataattttat ggaaatagag 10740
tattttttat tttttttttg ttatttgttt atttttaaaa atttagaatt ttttttttaa 10800
atttttttat ttttgtgagg gggtgggggt tagggaaatt gtagtgtatt aggatttttg 10860
gggtattatg gataggtggt gagtaggtat gtgagtttag gggagaaggg tttgggtggt 10920
ggagaagaga taggggaggg ttgttagggg tttgagggat ttatgggtta tgggggaggg 10980
tatgtggttt gggagttaga atagagatgg gttatggttg ggattagttg ggaggaaggt 11040
ttataggttt ttgaatattt ttgttggttt ttttatgttt ggttaaagtt tttttatttt 11100
agaagtaaga ttttatttgt tttgggtttt atagagtttg gtttggtggg ttatgttagg 11160
agttgttata atattttttt tatttttatt ttttaggggg tttttggttt ttttttttta 11220
attttttgtt ttttgatttt ttttagtttt tttatttttt tttatttttt ttatattttt 11280
tttaaagaaa ataggatatt tggttattta attgtgagtt tagtgtttgg aattttttaa 11340
ggtttggagg ttgttttgta tatttttttg ttgatgggtt ggaaatgagt tattttatag 11400
aatttattgt atggaaagat ttagggttta gaaagaattt tttttttttg tttgttgttt 11460
tggttggttt gggttggaag ttataagtag gtgtgatggt ggtaatggtt ttttggggga 11520
gggggtagat attgtttatt ttgtatggtt tgggagttat gtttagtatg gtgtttgata 11580
tataatgggt ttttaatatt taaatgtgtg aatatgttta gtggttattt gagggtgttg 11640
agaggttggg aggttggtgg tggaagaggg agttttgggt tttggtgttg tagtaggggg 11700
gattgtagtt gtggatgttt gttttatatt tagtgttagg gtggttatga taggtagttt 11760
tgtttgtttg agtagttatg gtgtgtagag gaaattatta tatattagga gtttggaaat 11820
tattgtttta ttttgttttt ttgggtgatt tttagtatat gattttggta tttttgggtt 11880
ttaatttttt tgagttttgg ggaagaaaat gatttatttt agatatttta tagggttgtt 11940
gtgagtatta attgtggtat tgtgtgaaag tgtttggtaa attgtaaggt aggtataatt 12000
tgattttagg tttgtataat atatttatat atagtatttt atttgttttt tatatttatt 12060
ttgtgaggta gggagaatag gatttagatt ttgtagtaaa tttatggtgt ttggtatttt 12120
tataggtatg attgatttat tttttaaatg gtttgtgtaa atagttaggg tatttgtggt 12180
ttgttttttt ttatagatga ggaagttggg tttagagagg tgagatgatt aaggtgggat 12240
tttttttttt tgatgaggag tttggggttg ggggttggtt tgtgtgtatg ttagggtttt 12300
ggtattatat ttgtttagtt ttagatggag ttaggaggta aatgaggagg aggtgttagg 12360
gtggggttgg ggggttggtt ttagttttag ttttggttag ttgttttttt gattgttagt 12420
att 12423

<210> 266
<211> 12423
<212> DNA
<213> Artificial Sequence

<220>
<223> chemically treated genomic DNA (Homo sapiens)

<400> 266

ggtattggta gttaaggaaa tagttggtta aggttggagt tgaggttggt tttttgattt 60
tgttttaata tttttttttt gtttattttt tggttttatt tgaggttaag taggtgtggt 120
gttagggttt tgatatatat gtagattagt ttttagtttt agatttttta ttagagaagg 180
ggagttttat tttggttatt ttattttttt ggatttagtt tttttatttg taaaggaaga 240
taagttataa atattttgat tatttgtata agttatttgg ggaataaatt gattatgttt 300
gtggaaatgt tagatattat gagtttgttg tagaatttga attttatttt ttttatttta 360
tagggtagat gtgagaagta aatgagatat tgtatgtgaa tgtgttatat aaatttaaag 420
ttggattatg tttgttttat agtttattaa gtatttttat ataatgttat agttgatgtt 480
tataatagtt ttgtgaaata tttggggtag attatttttt tttttagaat ttgaggaagt 540
tgaggtttag aaatgttgaa gttatgtgtt gaaggttatt tgagaaggta ggatgagatg 600
gtggttttta agtttttgat atatgatagt tttttttgta tattatagtt gtttaggtag 660
gtagaattgt ttgttatgat tgttttggta ttaggtgtaa ggtagatgtt tatagttgta 720
gttttttttg ttatagtatt agagtttggg gttttttttt ttattattag ttttttaatt 780
ttttaatatt tttaaatgat tattaggtat atttatatat ttaaatattg aggatttatt 840
atgtgttagg tattatgttg ggtatagttt ttaagttgtg tagagtggat aatgtttatt 900
ttttttttta agaggttatt gttgttatta tatttgtttg tggtttttag tttagattag 960
ttaaaatagt aagtaggaga gaggggtttt ttttaaattt tgggtttttt tatatagtgg 1020
attttatgga gtgatttatt tttagtttat tagtaggaga atgtataaaa tggtttttgg 1080

attttagaag gttttaggta ttggatttat ggttaaatga ttaagtattt tatttttttt 1140
aaggaaaata tgagaagggt ggggaagagt aaagaggtta gaaaagatta gagagtagga 1200
gattggggaa aggagattag agattttttg gggaatgggg atgggggagg tattgtggta 1260
gtttttggta tgatttatta agttagattt tgtagggttt agagtaggtg gaattttgtt 1320
tttggaatga gaaggttttg gttaggtatg aagaggttag taggaatgtt taggaattta 1380
tggatttttt ttttagttgg ttttagttat agtttatttt tgttttgatt tttaagttat 1440
atgttttttt ttatagttta tgaatttttt aaattttgga tagttttttt ttgttttttt 1500
tttattattt aggttttttt tttttagatt tatatgtttg tttgttattt gtttgtggta 1560
ttttgagagt tttaatgtat tgtagttttt ttaattttta tttttttata agaataagag 1620
ggtttaagaa aaaagtttta ggttttgggg ggtaggtaga tgatagggag gaaataaaaa 1680
gtgttttgtt tttatggaat tgtttttttg agtagagttg gagtgtagtg gatttttttt 1740
tagaatgggg tttagttagg ggttattttg gaaaaagggg gataggaagg gagttattat 1800
ttggaaaggg aaagatatag ttatttttaa gaattggttt gtttttttta tgtaggtttt 1860
tttttttttt ttgtagggaa agggggtttt ggaaggagtt ttagtgagtg aatgtttttg 1920
tgtggaggta gtagattttt gaggaagagg ggtttttagg atatttgttt ggattttttt 1980
aatggtagag ttttattgtg ttagtaattt gtgtatttgt tgattttttt tgttgagttg 2040
gggtttttga taggtagggg tattttgata tgtttttaga ttttttatgg agtttgttgt 2100
agtgtttggt atggagtaga tgattaatat ttgggaagat tgaatgaatg aatgaatgaa 2160
ttaattaatt aataagtttt tttgtttaga agttagatgt gtgtttttat ttaggttttt 2220
ttgttttgtt ttttttgata gaggtgaatt gtttataatt gggggaaggt atagaagtga 2280
ttttatagag gagttgtttt tttttttgtt tttattttat ttaggtttag attggaggtt 2340
ttttataggt aaagtttgtt tttgaataat tttatgtata gtttttgatt atgttagttt 2400
ttttttaagg gttttttggt aatttagttt gtttttaagt aatattttgt ttattttgta 2460
tagttttttt tttatgatat gtaagtataa atggataggg aagagattaa ggtatatgat 2520
aatttgggat attttgaata gaggaataat taatgggggg gggggttttt tatttttttt 2580
agagtagggt ttgttttttt gggaatatgg tagtatgtta ttgtgtgatg tttttttgtt 2640
gttataatga taggaggatg tgttattagg tggtgtggat ttgttgttat ggtaattaaa 2700
aagatttttt ttatatgatt ttttttgatt gaggttgttg ttttgttttt atgtagggga 2760
ggagaattag ggagatttat aggttattag gttgggggaa aattatgtga tagattttta 2820
tggtaattaa gggaatgggg gatattggtt gttatagtga ttatgagaga tttgtgtagt 2880
tatatgttta ttgttgttat ggtgattgtt tgggttggag tggattttaa agagggggta 2940
ggagagggga ggattttttt tttttttata atttattatt aaaagtagag aaaaaggaaa 3000
tggtagagag aggaatttta tttttatttt tttttttttt tttttttttg ggagagagaa 3060
ttagagtatg ttatttttat aaatgagttt tttgtgtggg gtttatttta gatagagttt 3120
ttgtttagtt tggtagttgt aagggtgagg aaagtaggga gaggaggggg gtggttggga 3180
tatttttagg ggtatagggg ttttgggtat gggttgagaa ggtaagtagg ttttttagtg 3240
gttttatttt tatttaggag tttggaaatg tgagattaag gaatgttgag gagaaaatgg 3300
gtttggattt aggagttttg gttttttagt aaatgaggag gtagggggat aggtttttgt 3360
tttatttatt ttatagagag gtttttggtg tttattggat ttggtaattt ttagggtaat 3420
atgggatgta ggggttttgt tttgttttaa agtttaaagg gggatttttt tagtagaatt 3480
tttttttttt attttataag tttatttttt tgtggttttt tttttattat gttttagtgt 3540
atgggtgtag ggggagagag gtttttagtt gaattgaaaa ttgaagttta tagaaaggta 3600
gttggtttta tttagagata gagatatgag atatagagat ataagagaga tggtttgttg 3660
gaatttttgg aaggggaaat aggttagaaa tttgatttaa atgtttgtga ttttgtgtgt 3720
gtgtgtgtgt gtgtgtgtgt gtgtgtgtgt gtatgtttgt gtgtgagtag ttgtgtttgt 3780
tgttattttg gttgaggtag gtagggtggg gtgagtgttg ggtgggagta ggtgagggtg 3840
gggggggttg tgtggtgggg gtaattttag gaatggtttt aattggtatt atgggtgatg 3900
gggagttttt tagtagagtg gtggagtaga gtgggtggga aggaatgtgt gtgtgattta 3960
gaaggaaagt gggtttgggg tagaaagtat gttaaggggt gatttggttt ttttttaatt 4020
ttatttaatt ttttagtttt agtttttttt tttagaattt tttttggtga gtttagggtt 4080
tggaaattaa attggaggag ggaattgatg aagagatagg aagaatattt tttttattat 4140
tttttttgag gatagaaatg gagaattttt tttaggtaag agaaataggt agatttgggg 4200
tattgggata gtgtggtaag gaaggtttta gaaatttttg tgtttttatt tttttagagg 4260
gatatattta gatatataga gtgatgtata gttatttatt tatatttatt tttggaagtt 4320
attagggttt ttgttgttta ggtttttttt tttttttttt ttttgatttt tggtaggttt 4380
tttttttttt tttttggttt tttttttatt tttttttttt tggtatttta aatgttagtt 4440
tgtgattttt aaatgagaaa aagttgtgtt gggttttgag tttggagaaa tttttatata 4500
aatttttaga tgtgatataa tagttagaga gggaatgtgg aggaattttt ttttgagagg 4560
ggagtgtgtt tttttatatt tgtaggaata tatattggaa aggttagtat gttttttttt 4620
gatttgaggt aggggtgatt gtttgttagt atttttgttt tttatattaa agtttgtatt 4680
tttgagtttt ttgttgtttt tttttgtaat atatatatat atatatatat atatatatat 4740
atatatatat atatatattt tttatttggg tttagatttt ttttttagga aatttggttt 4800
tttaaaaagg agaggttaag aagtagaatg tattaagaaa agtttaatta gttttagtta 4860

gttgggggtg gaggggagtg tttttatgtt tttttggttt agaggtgtat agggattttt 4920
ttgaagtttg tggggagtta tagatattat aaagagaaag agggaggttt tggaggtagt 4980
ttagagtttt tttggtagta aaaatatagg tgttttttgg tatttgggta ggatggtagt 5040
ggtgaggggt atagtttagg tagttttttg ttaaaaaatt gtttttaatt tatttttagt 5100
taatttaggt ttttttagat ttagtaatgt tgatgttata tatttaatta ttattttttt 5160
gggggaagtt gttttttaat ttttaaggtg agtgttgttt ttttattttt ttttttttat 5220
gttaggtttt aatatttgtt tttgtttaga ttatttagtg aaggagtttg tttttagatt 5280
ttttattagt atttggagat agggtaggtg agaagagatg ttagtttaga ggtttgtttt 5340
tagggagagg ggagagaaga gtttttttta ttttttttgg gatttttgag aaggaggata 5400
tagaggttga tgtttttttt tatttgtgga aggggtaaat gattaggggt agagaggaga 5460
gttgtttgga atttattttt tttagttata ttagtttttt atttttattt ttgtttggga 5520
gaaaggggtt tggggttggg ggtttaaatt ttaagattgt gtttatttag taagaatata 5580
gatttttgag gtttgtattt ttttttttgt agtttagaga gaaaggaaga aggggtagtg 5640
gatgagaaga ggatgtgatg aggggagttt tttaaagatt tataaggtgt agaggatggg 5700
ggtggggggg ttataagttt atttttgttt tgtgtattag aggtagtaga gttgtagaga 5760
gtgagggtgg tattaatttt ttattttttt tttttaaagt agagtttttg ttaggtttat 5820
aggggttata agggagattt ggggtttttt attttgggtt tataagaatt tttgagttgg 5880
aaggaaggag tttagagaag ttgtttagat ttgagatggt agtaatattt tttttttatt 5940
tttttttttt ttggtggtag gatttgttag ggagtttggg gttaggtgtt tgttaagtgg 6000
tgttttgagt tagggtttga ttgggttggg gtttttttta ttattttagt ttgggtagat 6060
agggagtagt gttaaattta tttttttgga atgtggggtt tgtatttttt aaatttattt 6120
gtggagttta tgttggggtg gtggatttga gtttttttta gtgaatatat tgatagtatt 6180
attgtttttt taagattttt gtgggtttgt agttttttat atttggtttg taggagtatt 6240
ttttatttgt gaaaggggag gagtaatttg gattagttta atttttattt ttatttgtta 6300
aaaaagtttt tgatatagtt ttttttttta taagaaaaat ttagtataaa attttttaat 6360
ttaattttaa gtattttgta ttgaatggag attgggtagg aggtatttgt ggtattgggt 6420
tttagtggaa aggggtttta tttttagttt tgggggtaga tagttttttt ttattatatt 6480
ttgtttttat tttgtgtttt tgatattagt tgttgttgga atttttttgt tgtgttttta 6540
atttgtgagg tggggggtga ggtgggttag gatttggttt ttgtttttta atattttttt 6600
ttaggttagt aaaaatttta gttttaagga ttaggttggg ggattagatt ttagaatttg 6660
ttgtaaattt tggggtgggg gaaggaggga aatttaggag attgtggttt tttttttttt 6720
gtttttgtag tttttttttt attgttttta tttatttttt attaaatttg gaaattaaat 6780
ttagtttgtt aattggtgtg ggtagagtat agtttttagg tttttgaagt tttttatatt 6840
tggaggatga tgattttttt ttgattatat ttggagggtg tttagttgtg agttagtggg 6900
gtatagtttt ttttttttgg tttggatttt tattttttta atttaggaag attgaggtgt 6960
gtgtatgtag gtggggtaag tttgggttat tgatgagatt tgtttgtgtt tttgttttta 7020
ttttggtgtt tttttttttt tttggtagtt ttttttttat ttgtttattt tttgggttta 7080
ttttgattta tttggtggtg ggtttttttt ttttttgatt tttggtggtg tggtttgttt 7140
tttttttgat tttgtgtttg gttgttgtgt tttgaaggat atgggttata gattgtgtgg 7200
agatattagt tttgtgggtg gttgttttgg tttaagtaga agaaaattat tggggttagt 7260
gttgggtatg gtagtttttt tgttttggag tttgtgttgt ttgggttttt ttttgttgat 7320
tttggttttg gttgtttttg gtttttgata ggttgttgag ttgtatgaag ttttggggtt 7380
gttttgattt tttggtgttt gttttatttt tttttttgtt tattttttgg ttagttgggg 7440
ttttgggtga tttggagagg ggtaagtggt gttttttaga ggaggtgttt ttatgtaatt 7500
tgggggtttt ggtgggggta tatttggtgg gttttagggt gtaagttgag tttttgggag 7560
ggttagttta gtttagtttt ttttgtttgt aggggtatgt tgtttgtggg gtgttggggt 7620
gtagggaaat tttgtttggt gtgagttttg ttttttttgt gtttttgggg ttttttgggt 7680
tagttggttg gttttttttt attttgtttt ggtttttttg ttttgtgttt aggttttggt 7740
tgttggattt ggtgttattt ttggtgaagt tgttttgaga gggtgaatga aaaggggggt 7800
agtggtggag ggtgggggtt tggggagggg tgtttttttt ttttgtggtg tagttggagt 7860
tggatttaag gggttagtat tgttttggtt ttgtgttagg tggtggggga ggggaggagg 7920
gattttttta ggggtgggtt tggagggggt gggggtggtt tttttttttg ttttgttgtg 7980
ggtttattgt ttgggagggg tggggagata gtggggggta gttttgggtg ggagtggagg 8040
tggagatgtt gttgttgtag ttgttgtggt ggttgttgtt gttgtagttg ttgttggtgt 8100
tatagttttt ttttttggtt gagatgtgga gtgagtgagt gaaagtggtg gtgaggagtt 8160
ttgtgtatat tatagttgga ttttttattt taattttaag ttttggtttt gtttttagat 8220
agttttgtag ttaattgttg gatgttgttg gttttgagtt gtgtgtttat tggttgtttg 8280
ttgtggtagt gtgggtggga gtgtagggag agaattatag ttggaatttg atttttattt 8340
taaaatttag gattgttttt tttttttttt tttgtttttg ggtttgtttt tgaaggttgg 8400
agttaattag attgttgttt agattgaatt tattttttta ttggttattg gggtttttgg 8460
gatttttttg gagggagttt gaagaattag agtatttggt gtggggtttt ggtgtagggt 8520
tgtaggtttt aggtattttt tgtttatgat ttttgatttg ggtagtagtt ggaggatgga 8580
gtttagtttt gggtgggttt ggggaagaaa ggagataggt ggtatggggg tgagggttgt 8640

tattaggggg ttttagagtg aaattttagg tttttatttg gttaattttt tattttggag 8700
ttgagtttgg ttgtgggatt ggggaaggga aggagtggag ttggatgttt tgggggtggg 8760
gttttaggtt ggaggggttt agttttggag aaatgggtgg agtttgagaa aggttagggt 8820
aggtatggtt tgggtgtggt ttttataaat tttttgggtt tattggttat atttttgagt 8880
taattttgag attttgattt tgattttgta attttaattt gttggatttg attttatatt 8940
gatgtttttg ggttttattt ttttgttttt agggattttt tgaagggtgt ggattttatg 9000
taggggtttt gtgtttgaat atgtggtttt aggtaggttg tgttggattg tagagtttgg 9060
atttttagga ggggttgtgg agtagagtga ggatttttaa gaaggaattt gtgggggttt 9120
tgagtgtgtg ggagggggtg gaagtataga gttgggtttt attgatttga tttttaaagt 9180
ttttattatt gttatttttt aattttgtgt tttagttttt tttaagtttt tatgtggttt 9240
agggaaggag tgtttgtgga attggaattt tgtgggtttg ttgtttttat ttatttattt 9300
taattttatt ggtagtgggt ttttgtgggt ttgtgttttt tatgtgtgta tatgtatgtg 9360
tagattttgg tttttgtttt gtttattttt ttgagttttg gggtttagtt ggttttagtt 9420
tttttttgtt gttggtgtta ttttagaaag ttgtgggtat ttgggagtgg agtgttgtgg 9480
gtgtgttggg tgttggtgga ggggggaata gtgaggatta gatgttggga agtatttttt 9540
taatatttgt gagaggtaga tatggtggaa tttagtgggt tagttagttt tttttttgga 9600
gattttgggg tgtttgtgtt attgtggaga ggtggtggga tgggttggat gatttttatt 9660
attatttttt attttgttga ggttattatt tttgggtgtt gtttattttt ttaaggttgt 9720
attatttatg tagtttttgt gtattttttg ttattgtaga gggttggggt tgttttagtt 9780
tttgttttgg ttgatggggg gaggggatgg gggaagtggt agagtggttg ttgtgttgtt 9840
gtttttttgg ttgttttttt gttgaattta tgtatagagt tgtgtaaatg aggttgggtg 9900
ggtttatttg tattttatgt taatgagtag attttttaag tgtttttttt ttttggtggg 9960
agtttttgtt tttattttga gtatgtggtg tttgagtttt gaattttgtg ttgttttttg 10020
ggaaaggggg gttatttatg gttgttttgt gtgtttgata tttttttttt ttttgagagg 10080
ttgtagtttg ttgggttggt ttgttttttt tgttaggata gaggttttgg ggttggtgtg 10140
agaagtaggg atgtttaata tttttttttt gtagagggga aaatgaggat agagaggttg 10200
gtttagttta atgattaggt tttttggtgg ttagtaggtt ttatattttt atttattatt 10260
ttggttattt ttgggttttg gattgaattt tgttttagaa tatgtttgaa aggagggtag 10320
gtataaatgt attatttaga taaattgaga tttatatgta tttttttggg tttaggattt 10380
aggggagatt ggttatgtaa gataggattt gatttttgta tttggtatgt tggggttgat 10440
ggttagttgg agatgtttat ttttagtttg agtgttagag aggaggtaga ggtagaattt 10500
atagggaagg taaagaagat ttgaaattat ttttatttaa ttttagtttt ttaagtttta 10560
gttttgatat tattttttta gagtggtttt ttttttagta agtattttaa tgttatttaa 10620
gtgttaaggt gtttaggata tagtattgag ttagatataa gtttggaatt ttatagtatt 10680
tagtttaggt tgggattata tgtttgtgtt tgtagtaatg aaatgattat ttttttattt 10740
aggttttgtg tttttagagg ggttatgttt attttgttta ttagggtttt gtttagtgtt 10800
gggtataaag ttgttaggtg aatgaatgat tttaggtagg gaaagggaaa taaagggata 10860
agaaattttt ttttttgggt tttagttttt taataaagat attaagttta attaagagtt 10920
tttttgggtt tgaggaagaa ttttttttat atatattttg tttagttagt ttttttagtt 10980
ttttttagtt ttgtttttat ttttttttta tagtttattt ggttttttgt ggagttaaga 11040
taattttttt atttgtgata ttttttgttt agggatgata taattattat tttatttagt 11100
tattttaata gttttgagag ttgggtggat ggttttttat tttttagata aggaaggggg 11160
gtgtttagaa tgatgaagtt atttaagtga ttgtttagat ggtatggttt tgtttttagt 11220
gattttggtt tgtaggtgtt ttgttggtag aatagggtgt atatttaaga gggattagta 11280
aaggggaagg gatgttgtag gaatttttat aattttgttt ttgagtgaga tttatgtggg 11340
attttgtttt ttttagaaat ttgagtattt ttgggagaat taatttttta aggaggtaat 11400
tttttttttt taatttattt atagaatttt attattgtgt gtatatttta ttttaggagg 11460
gagagaggag ttgttgagta gaaagtagat tggttgtagg gatagaagat attaagggta 11520
ttttttttta tagttgtgtt tggatttttt gtgagaagag gaagttttag gggtttttat 11580
tgtattttta agattttaga gagaaataga agtgttgagg ggttgtaggt aggtttttta 11640
ttttttttgg gttttgaata tttatttata ggaggttgaa ggtaattgtt ttgtaattta 11700
tttatgaggg ttaatgggat gatagtggta agaggggttg gtatattgtt tagattgttt 11760
taaagttagg gttggggttg ttaaaaaggt tatttgtgtt ttattatgtt ttattttttt 11820
ttttttttat ttttttaggt tgggttttat ttgtttagtt ttttgatttt ttggtaaggt 11880
tgagtaggtt ttagttagga aggggttagt tgattttgtt ggttggagta ttaatttttt 11940
ttagatgggt tggtggtttg tattgagggt ttggttgttt agatagagtt gattttggtt 12000
ttagtgatta tgtgtggtta tgtgtgattg tgtgaaggga gtaattttat ttagtttttt 12060
ggtttttttt tgttttattt ttttttattg ttgatatttt ttataagtag tggttttatg 12120
aaggtttttt tgtttttagt agtttttttt ggttttttgt gttgtttagt tggagggttg 12180
aggatgtttg gtatagttga tttttaaagg ttttagaggg atagtttttt tttttgtttt 12240
ttttattttg ttagttttat tattttgtat ttgttttttt taaagtatgt gttttagttt 12300
ttatttttta atttaaggaa gatgtatgtg ttaaggatag gatttaggag tattttttaa 12360
tatttagggg aaaggatgag gttataggaa ggtttttttt gtagtaagaa ggatttagat 12420

aga                                                                12423

<210> 267
<211> 3720
<212> DNA
<213> Artificial Sequence

<220>
<223> chemically treated genomic DNA (Homo sapiens)

<400> 267

tgtagttgtt tattaaaatt ttatatatat tttggtatat aattatattt gttggaattt      60
attttataga tatatttata tttatatagt taaggatgtt gattttaata atattggtat     120
aagtaaaaag ttggaaattg tttatatgtt tatggatagg ggataaattg attaatttaa     180
ggtttttttt tataatggag tattatatag ttttgaaaaa gaatgataag atttttatgt     240
taggatatgg tataattttt aagttatttt aagtgaaaaa ataataataa taataatatt     300
gtagaataat gtgtatggtt ggatatatat aaattttttt gaaagaatat ttaagaaatt     360
taaatgtggt tattttagga gttaggaatg aggtaggtgg agaggaggga tttttatttt     420
ttaatttaga atatattttg ttgtgttagt tgattttttt tttattgtta ataagtatta     480
tagttttttt taatttaaat tatttttttt agtataaatt taaaaaaaaa aaatttaaaa     540
tggaaaaata tataggtgtg agtaaaagaa atgaatggta ataatttttg tggtttattg     600
agtgtgtttg gatatttatg tgtttttagt ttttttttat aggaaatgta taggtgagaa     660
attgatgtta agggggattg agtgttaagt tagttagtgg tagagggtag atttaaattt     720
aatatggttt ttttttgttg ttttttggtt tttgttttta ggtgggaagt gtatttattg     780
gatggttggt ttggtgaggt gtagtgtttt agattggtgt atttgtggtt ttagtgtttt     840
atgtttgggg agtgtgtgtg tatgtagtgg tgtgagtttg gtggtggtgg tgataataat     900
aatgttatag tttgagtttt tttttttgga gtttttggta tatattttgt gtttatgttt     960
gggtatttat gttatggtgg tagggttggg gttttttaaa atggtagtgg tttggggagt    1020
tggaagtttg gagttagtgt tgttgtagtt atataagtgg gggggttgtg ggttggggga    1080
gtttggtagt gttttggaga ggtgaggagt tgttgtttga ggttggtgtg ggtgagtgag    1140
ggtgttgtgg ttttttgatt ttttttttag aggtgagtgt ttgaagttag gagtttggtg    1200
tttataggtt tgtgtgttgt ggggaatttt tattgttagt ttttttgtta tttgtgtgtt    1260
tttaagttta gtgggtgagg ttttgggtgt tttatagttg gtgttgtgtt atgttttatt    1320
ttagtgagtt ttttgaattt gatgtgtttt ttgttaagtt tattgaaggt tgttgtgttg    1380
aatttagtgt tgaattgttt tggttgtttg ttagggatgt ttttgtggtt attggttatt    1440
ttggaggtaa ggagggtgag taggggtgtt tagatgatga tggtgtagtg tgggggtgta    1500
ttatatttga gaattaggag ggattgggat attggagtta tgagaatagg ggtgatggga    1560
agtttaggag ttttggggtg tgtattttta tttttattta tattgggttg ttagtgtttt    1620
tgtaggaatt tgtgtggtta ttggagtgtt tttttgtttg tgtatgtgtg ttttgtgtgt    1680
gtatgttttt atgtgttttt tgggatgtat gtgggttttt gttgaattag aatgtgtaaa    1740
aggtattttg tgtatatttg tgggtattaa gagttttgta ggtaggggtt gtggatttag    1800
gtgtattttt tgatgtgttt agagttgatt tttgtttttt ttttagtagg tgattttttg    1860
agttgggttt tgaatagttg tggtgtaagt ggggatttag ttgatttttg ttttttggag    1920
gggtttgtgt ttatattttt ttttggtttt agttatagtg gtagtttttt tatttaggta    1980
gtgtttgaat atttgtatga tttggaggta ttttttaatt ttatgttggg tgttttaggt    2040
ttggtatttt tttttggttt agaggtagta gtgtttagat ttttgttgga tgtttttttt    2100
tttgtggggt ttgatgtttt gttgttgggt ttgttggatt tttatttttt ggatttgggt    2160
gttgtttttt ttttagaggt gttttgggag gttttgtttt gtgtgggtgt ttttttgtag    2220
tgtttgtatg agttttagtt ttttttgttt gatgttaagt ttggtttttg gtggtttttt    2280
gttttgttag tgttggatgt tgtttttgtt tttaagggtt tttatgtgtt ttgggagttg    2340
ttttttgtgg gggttttagg gaattgtggg ttatagggag attattaggt tgttttggag    2400
gtttgttttt ttgtaatagg gattaagatt gaggatttgt tgtttattag ttgttttgtg    2460
gaattgttgg ttgttttagt taatagattt tattttagtg gggtttatga tgtttttttg    2520
ttggttttgg gtgatttagg ggagggggtt gagggttttt ttgggttttt gatttttttt    2580
agtggggagg gagggagtag tggtgatggt ggagagtttt tggttagtat gtagttttag    2640
ttttttttgt tgggtttttg tagtgttgtt gtggtggatt tttttaaatt tttggtggtg    2700
gatatttttg gaagtagtgg tgtggttgta ttatttgtgt tgttgttgtt gtttattttt    2760
tttttttagg ttaaggtgtg atgtaagggg tgttgtggtg gtaaatgtag tatgtgttgt    2820
ttttgtttgt ggttgtatgt taaggttttt gtttgtttgg tggagagttg tgtgtggagt    2880
tttgtgtgtt ttgatgagtt taattgttat ttgtgtattt atatgggtta taaatttttt    2940
tagtgttgta tttgtttttg taattttagt tgtagtgatt attttattag ttatgtgtgt    3000
atttatattg gtgagaagtt ttttgtttgt gatgtgtgtg gttgttgttt tgtgtgtagt    3060

gatgagaaga aatggtatag taaggtgtat tttaagtaga aggtgtgtgt tgaggagtgg 3120
tttaagggtt ttggttttta tttgttgggt tttttttttg tttttttttg agtaagagat 3180
gggtttatgg gttggggtgt tgttgtttgg tgtgtatgag ttttgggttg tttttttttt 3240
tgtttttttt ttaatttttt tttgtatgtt tgagggttgg tttttgtttt gtttttagtt 3300
tttttgaagt gtttgttgta tatgttttat ttagtattag tttgttggat agtttttgtg 3360
gtttaggtgt tgttattttt gttagttgtg ttttggggga agttttttga gattatttag 3420
tgaataggta ttattttggg atttaagata gttttttgta attgtatatg ttttatgttt 3480
ttttttataa tttttagaga taggttgggg taggttaagg tggttttgtg tgggattttg 3540
tatagtagtg ttttatttag tagttgattg gatgtaatgt tttgttttgg gttttttttt 3600
tttttgttgt tgttaatttt tgtaaagtag atgttatttt taagtagttg ataaaattgt 3660
ttatttttgt aattaaaatt attgtgttaa aagtttattg aatttgttat gtaagttttt 3720

<210> 268
<211> 3720
<212> DNA
<213> Artificial Sequence

<220>
<223> chemically treated genomic DNA (Homo sapiens)

<400> 268

aggagtttat atggtagatt tagtaagttt ttagtataat aattttaatt gtaaaaataa 60
atagttttgt taattgtttg agagtagtgt ttgttttata aaaattaata ataataaaag 120
gaaaaaaaat ttaaagtaaa atgttatatt taattggttg ttggataaga tattgttgta 180
taaagttttg tgtgagattg ttttggtttg ttttagtttg tttttggggg ttatagagga 240
aggtgtgggg tgtgtgtagt tataaaagat tgttttgaat tttagggtag tgtttattta 300
ttgggtggtt ttagaagatt ttttttaaag tgtggttgat aagggtgata gtgtttggat 360
tgtgggaatt gtttggtgag ttggtgttga atagggtgtg tgtggtgggt gttttaagga 420
aattggaagt gggatggagg ttggtttttg ggtgtgtgag gaggagttgg aagaagagtg 480
gggaggggaa tggtttggaa tttgtgtgtg ttgaatggtg gtgttttaat ttataaattt 540
attttttgtt tagagagaag tgaaggagag gtttagtgag taaaagttga ggtttttgag 600
ttgttttttg gtgtgtgttt tttgtttgag gtgtattttg ttgtgttgtt tttttttatt 660
gttgtgtgtg aagtggtggt tgtatatgtt gtaagtaaaa ggttttttgt tggtgtgggt 720
gtgtatgtgg ttggtgaggt ggttgttgtg gttgaagttg tggaggtaga tgtggtattg 780
gaagggtttg tggtttgtgt ggatgtgtag gtggtgattg agtttgttgg agtgtgtaaa 840
gttttgtata taatttttta ttgggtaagt gaaggttttg gtgtgtggtt gtgggtagaa 900
gtagtgtgtg ttgtatttgt tgttgtggtg ttttttgtgt tgtgttttgg tttgggggaa 960
aggggtgggt ggtggtggtg gtatgggtgg tgtagttatg ttattgtttt tagggatgtt 1020
tgttattaga ggtttaggga agtttgttgt ggtggtgttg tgaaggttta gtggggaaag 1080
ttgaggttgt gtattggtta gaaatttttt gttgttgttg ttattttttt tttttttatt 1140
aggaggggtt aggagtttag ggaggttttt agtttttttt tttaagttat ttggggttag 1200
tgggaaagtg ttataggttt tgttgggata gagtttgttg gttgggatgg ttggtagttt 1260
tgtagggtag ttgatggata gtaagttttt aattttggtt tttattatgg gaaaatgagt 1320
ttttggggtg gtttggtagt ttttttgtga tttatagttt tttggggttt ttatagaaag 1380
tagtttttag ggtgtgtagg gatttttgaa ggtagagata gtgtttagtg ttggtgaggt 1440
gggaggttgt tggaggttgg gtttgatgtt gggtggggag agttgaggtt tatataggta 1500
ttgtgagggg gtatttgtgt aaggtgaggt tttttagaat gtttttggga aaggggtagt 1560
gtttagattt ggggagtaaa ggtttggtgg atttggtagt aaggtattgg attttgtagg 1620
aaaaggggta tttagtgggg atttggatgt tgttgttttt ggattgggga agggtgttag 1680
gtttaagatg tttgatatga ggttgaagag tgtttttggg ttgtgtgggt gtttgggtat 1740
tgtttgaatg aagaagttat tgttgtagtt gaggttggga gggggtgtgg gtgtaggttt 1800
ttttaggaag taggagttgg ttaagttttt atttgtgttg tagttgttta aagtttagtt 1860
taagaagttg tttgttgagg agggagtagg aattagtttt gggtatatta aagggtgtat 1920
ttgagtttat agtttttatt tataaaattt ttggtgttta tggatatata taaagtgttt 1980
tttgtatatt ttgatttagt aggggtttgt atatgtttta aggagtatat agaaatatgt 2040
atatgtaaaa tatatgtgta taggtaaaag ggtgttttga taattgtata ggtttttgtg 2100
gaggtgttgg tggtttagtg tgggtgggaa tgggggtgtg tattttagga tttttaagtt 2160
ttttattgtt tttattttta tagttttaat gttttagttt tttttggttt ttaggtatgg 2220
tgtgtttttg tgttgtgttg ttgttgtttg agtatttttg tttgtttttt ttatttttag 2280
ggtagttggt ggttgtggga gtgtttttgg taggtagttg ggtaatttta gtgttgggtt 2340
tggtgtaata gtttttagtg gatttgatga ggagtgtgtt gggtttggaa aatttgttaa 2400
ggtggagtat ggtgtggtgt tggttgtggg gtgtttgggg ttttgtttgt tgggtttggg 2460

ggtgtgtggg tggtggggag gttggtggta ggggtttttt gtagtgtata gatttatggg 2520
tgttgggttt ttggttttgg gtatttattt ttgggaaagg agttggggag ttgtggtgtt 2580
tttgtttgtt tgtattggtt ttgggtggtg gttttttgtt tttttaaagt gttgttgggt 2640
ttttttagtt tatagttttt ttatttatat agttgtggtg gtgttggttt tgggtttttg 2700
attttttggg ttattgttat tttgggaggt tttggttttg ttgttgtgat gtaaatgttt 2760
aaatatggat ataggatgtg tgttggggat tttgaaaagg aaagtttgag ttgtgatgtt 2820
gttgttgttg ttgttgttgt taggtttgtg ttgttgtgtg tgtgtgtgtt ttttaggtgt 2880
ggagtgttgg ggttgtggat gtgttagttt ggggtgttgt gttttattgg gttgattgtt 2940
tggtagatgt gttttttatt tggaggtaga ggttgagggg tagtagggga ggattgtgtt 3000
gggtttgaat ttgttttttg ttattaatta gtttgatatt tagttttttt taatgttagt 3060
tttttatttg tgtatttttt gtggagagaa attgagagta tataaatgtt taggtgtatt 3120
tagtaaatta tagagattat tgttatttat tttttttgtt tatgtttgtg tgttttttta 3180
ttttaagttt ttttttttta aatttgtgtt aggaaaaata atttaaatta aaaaggattg 3240
taatatttgt taatagtagg aggaaagtta attagtataa tagggtatat tttaaattga 3300
aaagtaaaag tttttttttt ttatttattt tatttttagt ttttgaggta gttatattta 3360
agttttttgg gtattttttt agaagaattt atgtatattt aattatatat attgttttgt 3420
aatattatta ttattattat ttttttattt gaaataattt agagattgtg ttatgttttg 3480
atataaagat tttgttattt ttttttaagg ttgtatagta ttttattgta agggaggatt 3540
ttgagttagt tgatttattt tttatttatg agtatgtagg tagtttttaa ttttttattt 3600
atattaatgt tgttggagtt aatatttttg attatataaa tgtgaatgtg tttgtgagat 3660
aaattttaat gagtgtaatt gtgtgttaaa gtatatgtaa aattttgata gataattgta 3720

<210> 269
<211> 8759
<212> DNA
<213> Artificial Sequence

<220>
<223> chemically treated genomic DNA (Homo sapiens)

<400> 269

tagtttggaa gagggtagtt aggggagaag ttagggttgg agttatgaga aaggataaga 60
tgagatgatg gttttaatat tgaggataga aagaatattg agatgagaaa gtagtttatt 120
ataagtattt atgtaaagga aatagtagat gtttataaat tagtagaggt aataattttg 180
aaagtttatt tataagtttt tttttttatt tttaatttag tttaaatgta attatttaaa 240
ttgtttttta tttttttttt tggattatga atgagttttt taaatgtagg gtttatagtg 300
ttttatttat tagtgaattt taagtgttta gtatagagtt tggtaaatag taaatgttta 360
ataaatattt gtttagtgta tgaattggag tgatttttta ttttgtttat aagttgaaaa 420
aaggtttatt atatatttaa atatgttgaa attatagggt atttggtaat tttttaaaat 480
taaaattttt agtttggaaa tagaatttta attttgtgaa aataaaattt atttatttat 540
ttaaaaaata tttattaaat aatgattatg ttttatatta ggttgagttt taaggattta 600
atgatgaata aaaattaata tgatttttgt ttttaggaaa tatatagttt agtgaggaaa 660
atagattgtg agaagttttt taataaatat tgggtgttat taaaatatat taaaaggtga 720
gtgggtgagg gatttgagtt agtttaggtg gtttaggaag tttttttgga tgtgttgata 780
tgtataggta ttaattagat aaatagagag aaggatgaat taatattgta ggtagaggga 840
atagaatatg taaaggtagg aaggattatg gagttgttgg aggatttgaa taaaggttag 900
tgtaagtgga ttttagaaaa taggaggaaa ggtgtatgag atgagattag agaggtagat 960
tatgtggggt atggttaatg ttttggattt ttttattaag agtaatgggg agatagtgat 1020
aggatttaaa tggggaaata atatgattag attaaatttt ttaaaaaatt ttttatgtaa 1080
atatatattg agagttaatt attgataaag atttaaaggt aataaagtgg agagagaata 1140
gtatttttaa aaaatggtgt taaaataata ggatatttat attaaaagtt gggtatttgt 1200
ttataaaatt taatttaaaa tggattatag atttaaatgt aaaattgaaa gttatataat 1260
ttttggaaga aaatatagat gggaatttgt gtgattttga gtttgaaaat gatttattat 1320
atttgatatt ataatttgta agttaatata atttataagt gaataaagtg atgaattgga 1380
ttttattaga atttaaaatg tttgtgtttt aaaagatatt ggtatgataa tgaagataaa 1440
ttatagataa gatattgttg aattatattt ttgataaagg aattgtgttt agaatatata 1500
attttaaaat ttttataata aattataagt agtttaatta aaaaaaaaaa aagagaaaaa 1560
atttatagtt tttattaaag aaagtatata attgtaaaat aagtatatga aaaatgtttt 1620
gtatttttat ttatgggaga aataaaaatt aaatgggaaa gatattttta attagaatat 1680
taaaattaaa aagattgatt atattaagta ttggtgaagt ggaaatgtaa aatgatataa 1740
ttatttagga agatgatttg gaagtttttt ataaaagtag gtgtatattt attttgtgat 1800
ttatttattt tatggttaag tatttatttg agagaaatga aagaatatat ttatataaag 1860

atgtttatat aaatatttat agtagtttta tttgtagtag ttttaaattg aaaagaattt 1920
aaatgtttat taaaagtgaa tggataaata aagtgtggta tagtaatgta atagaatatt 1980
atttagtaat aaagaagaat gagttagtga tatatataat agtttaaatg tatattaaag 2040
gtattgtgtt tagtgaaaga tgtaagtaaa aaaaaaaaag agtatatgtt gtatagtttt 2100
attgatataa aattttggaa agtgaaaaat agtttatatt gatagaaagt agattattgg 2160
ttgtttgagg aggaggagta taggagaggt ggagggaaaa tgtataaagt ggtataataa 2220
aaatttttgg aattatagat atatttatta ttttgattga gtgatgattt tatgagtgta 2280
tgtgtgtgtt aaaaatgatt aatttatgta attttaaata tgtgtagttt attgtatata 2340
ttaattatat tttagtatgg ttattaaaaa gaaatttttt ggttgtataa tgtagaattg 2400
attttaggaa agagtggagg gaggatgatt atttatagtg ttttaggtgg aagagaatgg 2460
tgttttttgg aagtgaatta ggttggtaat aatagagatg aaataaatgg gtagatgtgt 2520
gagatattta ggaaataaaa tttgatggtt attatttttt aaaggttagt ttattttggt 2580
tttttagagt aaagagttag ggaagatttt attaataaat tttttttgaa gttgtagagg 2640
aagtttatag tagaaattta tttttaattt gattaatttg agagaatatt tttggtttta 2700
tttgattatt aaaaaattgt aaagaatagg aggagaaaga agaagaaagt tggtataaat 2760
agtgaattta tataatatta attaataatt gttttttgtt tttaaaagta atgggaagaa 2820
aatgagattt gagttggaag attagagttt aaaatttaaa taaagtatat ggttttaata 2880
tgtttatagt agttaatttt ttttgataat gatataattg ttgatagtat tatttttaaa 2940
aataaaaata atatagtaat tttttagatt tgtagaatgt ttttttgttt ataagtttgt 3000
tttatatata ttatgttttt taaatgatat attagttttt tgagggtaat ttatattggt 3060
aatagttttt agatgtggaa attgtgaaga taatgttggt gatgtggaag taatataaat 3120
tttggagttt tttagattta ggtttgaatg ttagattgtt ttttatttag agtaatttta 3180
gagtattatt ttttatttta attttttttt aggttttttt gtgtttatgt gtttttttta 3240
tttttgttta ttgtttattt agtgattttt gtattttttt ttattgttag tgtgtagata 3300
tatagttttt ttggttttga gatttatgtt aattttattt tattattttg ttagtttatt 3360
taatttttat tgagtaatgt tagttgaaag ttgtggtggg attaaatgtt gtaatgagta 3420
tttaaatgag gttgaagtat ttatgtattt tatttatata tggtgaggta tatttaagga 3480
aggttgtagt tattaaaatt ttaggaaata atttttattt tttttaggtg aaagggtttt 3540
taggtttttg tgttttggaa ggtttattta tagttatttt ttaaatgata atgtgattga 3600
tgagtttaga gtttagttta aatagtaatg gattggaaga ttagtttagg ttttattaat 3660
gtggaatata gaataaatta tgtttttgtt ttagtttgtt tatttgtgaa atagagttta 3720
ttatatttag ttttttttgt ttttaggttt gagttatttt ttttggttaa ggtaagtaaa 3780
tgtttatgat gtttggttgt gtataagata aagttataat aaagttataa tttatttttt 3840
ttttgtagaa gattgtaaaa agtaaaagag atttaggtaa aaattttgga atgatttttg 3900
gaatagagag tttttttaga attagaagtt aaaggaattt aaaatatagg gaggtttagg 3960
gtttttattg atataaagga aagatgtttt ttttataggt ttatgtttat attttttttt 4020
tttttttatt tttatttgta ttttttattt tatataggt ttatgggatt tttttttataa 4080
aagagtagtt gtagtaattt atattatttt ttatgtttgg ttgtttatta agaggtgaaa 4140
agtagtttta tataggtttt atttttggat agttttagtt gtaaagttta aaatatgtga 4200
aggtaatttg gaaaagtaag tggttgtata taaagtaaat gtttatagag ttttggataa 4260
aattgagtgt ttatgtgtat atggtaagtg tttttagtgt ttgtgtgttt atttgtttgt 4320
ttgggtgatt ttgtttttga gagtttggat gagaaatgta tggttaaagg taattttaga 4380
taggaagaaa ggtagagaag agggtagaaa tgatttttga tttttggggt tgagggtttt 4440
tagagtaaat ggtataatgt tatgaggttt gatttatttt tatgatggaa tttaaggttt 4500
tagtaagtat ttgttggttt ggttatggtt tgttttttag tttgtaggag atttttttat 4560
ttttttattt gtgtgttttt attagttttg aaaagaattt ttggtagtta ggagtaggta 4620
tttttattgt tttttttttt ttttttttgt ttttattttg ttggtttttt agattgggtt 4680
ttggaattaa atttggtgag tgttggtttt taggaaattt ggagttttgg tgtttaaatt 4740
ttggtttagg aaagtaggag ttatttagga agtaggggtt ttttagggtt agagttagtt 4800
ttttttgttt ttgtttatgt tgtgttagta tttgtttttt taaagttatt aggtaggtgt 4860
tagtgtgtgg tgaggggagg ggagaaaagg aaaggggagg ggagggaaaa ggaggtggga 4920
aggtaaggag gttggtttgg tgggggtggg atttgatttg taaattgttg tatttgtttt 4980
ttatttttta gtgttttttt tgagattttg gggagttagt ttgttgggag agtgggatgg 5040
tttggagtaa gtttagaggt agaggaggtg atagagggaa aaagggttga gttagttgtt 5100
ttagtgttgt ataggagttg aagggatgta ttatgttagt tttagtttgg ttttagtgat 5160
agttaatgtt ttttgtagtg tggtggtttt gaagttgttg tttggagttg tttttttttt 5220
tttggtgaag tttttaaaag ttgttaaaga tttggaggaa gtaaggaaag tgtttggtag 5280
gattgatggt tgtttttgtt tttttttttt ttattttgtt tttttttatt ttgttttttt 5340
tttttttttt gttttttttt ttgtagttgt tttagttggt tatttttagt taattttttt 5400
tattattttt ttttttattt gtttttttgt ttttgttggt ttagtgttgt tagtttgagt 5460
ttgtagagag gtaatttttt ttggttgtga gtgggtgagt tagttgtata ttgtaaagaa 5520
ggtttttagg agttaggtga ttggggagtg gttttagtat tgtagttatg atttgtttgg 5580
ttaggttgta tgtggagaga attttttgtt tttttttatt ttttttttat ttttttttgt 5640

tttttttatt ttgagtgtgg agttagagat taaaagatga aaaggtagtt aggtttttag 5700
tagttaaaaa ataaaataaa taaaaataaa aaagttgaaa taaaagaaaa agataataat 5760
ttagttttta tttgtattta ttttagtgga tattgaattt ggaaggtgga ggattttgtt 5820
tttttttttt aagatttggg tattttttga atttattttt taagtattaa gagatagatt 5880
gtgagtttag tagggtagat tttgtttatt gtgtgttttt ttttgtatga gattttgagg 5940
ttgttagagt gttttttgtg tggttgtttt tgtaagtttt tttttttgga gttttttgta 6000
ggtgggtagt tagttgtagt gattattgta ttattatagt ttgttgaatt tttttgagta 6060
agagaagggg aggtggggta agggaagtag gtggaagatt tagttaagtt taaggatgga 6120
agtgtagtta gggttgggaa gggtttattt ttggttgttg tttaagattt attgaggagt 6180
tttttagaat ttgttttaga gtgtgtgtga agtgatttag aatttgggtt ttaggtattt 6240
agaggttgtg agtgtagtat tttttggtgt tagtttgttg ttgttgtagt agtagtagta 6300
gtagtagtag tagtagtagt agtagtagta gtagtagtag tagtagtagt agtaagagat 6360
tagttttagg tagtagtagt agtagtaggg tgaggatggt tttttttaag tttattgtag 6420
aggttttata ggttatttgg ttttggatga ggaatagtaa tttttatagt tgtagttggt 6480
tttggagtgt tattttgaga gaggttgtgt tttagagttt ggagttgttg tggttgttag 6540
taaggggttg ttgtagtagt tgttagtatt tttggatgag gatgatttag ttgttttatt 6600
tatgttgttt ttgttgggtt ttattttttt tggtttaagt agttgttttg ttgattttaa 6660
agatattttg agtgaggtta gtattatgta atttttttag taatagtagt aggaagtagt 6720
atttgaaggt agtagtagtg ggagagtgag ggaggttttg ggggttttta ttttttttaa 6780
ggataattat ttagggggta ttttgattat ttttgataat gttaaggagt tgtgtaaggt 6840
agtgttggtg tttatgggtt tgggtgtgga ggtgttggag tatttgagtt taggggaata 6900
gttttggggg gattgtatgt atgttttatt tttgggagtt ttatttgttg tgtgttttat 6960
tttttgtgtt ttattggttg aatgtaaagg ttttttgtta gatgatagtg taggtaagag 7020
tattgaagat attgttgagt attttttttt taagggaggt tatattaaag ggttagaagg 7080
tgagagttta ggttgttttg gtagtgttgt agtagggagt tttgggatat ttgaattgtt 7140
gtttattttg tttttttata agtttggagt attggatgag gtagttgtgt attagagttg 7200
tgattattat aattttttat tggttttggt tggattgttg ttttttttgt tgttttttta 7260
tttttatgtt tgtattaagt tggagaattt gttggattat ggtagtgttt gggtggttgt 7320
ggtggtgtag tgttgttatg gggatttggt gagtttgtat ggtgtgggtg tagtgggatt 7380
tggttttggg ttatttttag ttgttgtttt tttattttgg tatatttttt ttatagttga 7440
agaaggttag ttgtatggat tgtgtggtgg tggtgggggt ggtggtggtg gtggtggtgg 7500
tggtggtggt ggtggtggtg gtggtggtgg tggtgaggtg ggagttgtag ttttttatgg 7560
ttatatttgg tttttttagg ggttggtggg ttaggaaagt gattttattg tatttgatgt 7620
gtggtatttt ggtggtatgg tgagtagagt gttttatttt agttttattt gtgttaaaag 7680
tgaaatgggt ttttggatgg atagttattt tggattttat ggggatatgt ggtaagtttt 7740
tttttttaga aatgttgttt tttggtttag ggtagagtta ttttgtgttt tggggtattt 7800
agtggttttt atttgtgtga atatttagat tgtttttggg agagtttagt agggtaaatt 7860
tagagttttt ttgtggattt ttggtttgtt agaggtttaa tttgagtttt tttaattttt 7920
gttgtgtgtt ttgggtgttg atttttgttt ttttagattt tttaattttt ttaattgttt 7980
taaattttta ttattttttg gtatttgagg ttttaaatag aaattttatt gtatgggtta 8040
tttttagaga taaagttttt aagtttttta tttttttttt ttttttgttt ttaaagtttg 8100
agaattttaa taggaatttg ggtaattttt tttttttagg tttgttagga ttttattttt 8160
agtttgtgta gattagagtt aaaaagattg gtttaatagt tttttagtgg gtatttttta 8220
gagaggtaaa gtgaaatttt tggttaggga aagaaagtgg tttttgggtg ttgaggtttg 8280
ttgtgtgaaa gggtgaattt tttttttttg aagtaattgg ggatttgttt tagggttgga 8340
ggttagtaga gataatttaa attgttatgt ttagagtagg tagaggggta atttttttgg 8400
taaagatttt ataggatttg tatttatagt tttttaatgt tggttgatta tgttgaaagt 8460
agttgtttgg gttggttttt ttttgtaaag tgtttatttt ttttgtggat tataatagat 8520
ttatagtttt ttattttagg tttgtattag atttataaag aggagaatat ttttttaatg 8580
tataatttaa ttaggtttga ttttgattta taaaattgtt ggaaaatatt tttttgtaaa 8640
gtattttttg ttattttagt gtgttttaaa atttttattg gggagggtgg agtgaggttt 8700
tttattatat ttttttattt ttaggatatg tttgtatttt agaatatgtg tagttagtt 8759

<210> 270
<211> 8759
<212> DNA
<213> Artificial Sequence

<220>
<223> chemically treated genomic DNA (Homo sapiens)

<400> 270

agttaattgt atatatttta aaatgtaaat atgttttaaa aataaaggaa tataataaaa 60
aatttatatt tatttttttt agtggagatt ttggagtata ttgaaatagt aggaaatgtt 120
ttataaaaaa atgtttttta atagttttgt aagttagagt taagtttaat taaattgtat 180
attaaaagaa tatttttttt tttatagatt tgatgtaaat ttgaagtagg gggttgtgga 240
tttgttataa tttatagaga aaataaatat tttataagag aaaattgatt taagtaatta 300
tttttaatat agttaattaa tgttgagaaa ttgtgagtgt aaattttgtg aagtttttat 360
taagaaagtt gtttttttgt ttattttaaa tatgatggtt tggattattt ttattgattt 420
ttagttttgg agtaagtttt tagttgtttt aggagaaaga agtttatttt tttatatagt 480
agattttagt atttagagat tatttttttt ttttaattga gaattttatt ttattttttt 540
ggaggatatt tgttgagaag ttattgggtt ggtttttttg attttaattt gtgtaggttg 600
aaagtgaaat tttaatagat ttgaagagga gaaattgttt aaatttttgt tgaggttttt 660
agattttgag gataggagga aaggaagagt ggagggtttg ggagttttat ttttgaaggt 720
ggtttgtgta ataggatttt tgtttgggat tttgagtatt aggaggtagt gagaatttgg 780
ggtggttggg ggagttgaag aatttgggag ggtaggaatt agtatttagg gtatgtagta 840
gaaattagga gagtttaggt taaatttttg gtaggttggg agtttatggg agagttttag 900
gtttattttg ttgagttttt ttaggggtaa tttgagtgtt tgtgtaggta ggagttgtta 960
gatattttag aatatagagt gattttgttt tgggttgaaa ggtgatattt ttggaaggaa 1020
aaatttattg tatgtttttg taaggtttgg agtagttatt tatttagggg tttattttgt 1080
ttttgatata agtgggattg ggatagggta ttttgtttat tatgttgtta gggtattata 1140
tattaggtgt ggtgaagttg tttttttggt ttgttagttt ttgagggggt tgagtgtagt 1200
tgtagggggt tatagttttt gttttgttgt tgttgttgtt gttgttgttg ttgttgttgt 1260
tgttgttgtt gttgttatta tttttattat tattatatgg tttatataat tggttttttt 1320
tggttgtgaa gagagtgtgt taggatgagg aagtggtggt tgagggtgat ttagaattgg 1380
gttttgttgt atttgtgtta tgtaggtttg ttaggttttt atagtggtat tgtgttgttg 1440
tagttgttta ggtgttgttg tagtttagtg ggttttttag tttgatgtga gtgtggggat 1500
ggggaggtgg tggagggggt ggtggtttgg ttagagttag tggaaagttg tagtagttgt 1560
gattttggta tgtagttgtt ttgtttagtg ttttggattt gtagagagat agggtagatg 1620
gtagtttaag tgttttggag ttttttgttg tagtgttgtt agagtagttt aggtttttgt 1680
tttttagttt tttggtgtaa ttttttttga aaggggaata tttagtagta tttttagtgt 1740
ttttgtttgt gttgttgttt agtagagaat ttttgtattt ggttaatggg gtataaggag 1800
tgggatgtat agtgggtgga atttttaaaa gtggggtgta tatgtaattt ttttgaagtt 1860
gtttttttgg atttagatgt tttaatgttt ttatatttag gtttatggat attgatattg 1920
ttttatataa ttttttggtg ttgttagaaa tggttgaagt gttttttaag taattgtttt 1980
tggaggaagt gggagttttt gaggtttttt ttgttttttt gttgttgttg tttttggata 2040
ttgttttttg ttgttgttgt tgaaggagtt gtatggtgtt ggttttgttt aggatgtttt 2100
taaggttagt ggagtagttg tttaagttgg ggaaagtggg gtttagtagg gataatgtgg 2160
atggggtagt tgagttattt ttgtttggag gtgttggtag ttgttgtggt agttttttgt 2220
tggtggttat ggtggtttta ggttttggga tgtaattttt tttggggtgg tattttaggg 2280
ttgattgtgg ttgtgaaggt tgttgttttt tatttaggat taggtagttt gtggggtttt 2340
tatgatgggt ttggggagaa ttatttttat tttgttgttg ttgttgttgt ttggggttag 2400
ttttttgttg ttgttgttgt tgttgttgtt gttgttgttg ttgttgttgt tgttgttgtt 2460
gttgttgttg ttgtagtagt agtaaattgg tgttgggagg tgttgtgttt gtggtttttg 2520
ggtgtttggg gtttgggttt tggattattt tgtgtatgtt ttggaataga ttttggaaag 2580
ttttttggta ggttttggat ggtggttgag ggtagatttt ttttagtttt aattgtattt 2640
ttatttttga gtttggttga attttttatt tatttttttt attttgtttt tttttttttt 2700
gtttagaaga gtttaatagg ttgtgatgat gtggtagttg ttgtagttag ttgtttattt 2760
gtgggaagtt ttagagaagg aaatttgtgg gagtaattat gtaaaaagtg ttttgatagt 2820
tttaaagttt tgtgtagaag aagatatatg gtggataaga tttgttttgt taggtttata 2880
gtttgttttt taatatttga agggtagatt taaaagatgt ttagatttta aaagaaaaaa 2940
ataaaatttt ttatttttta aatttagtgt ttattgaagt aggtgtaaat aagaattgag 3000
ttattatttt ttttttttat tttggttttt ttgtttttgt ttgttttgtt ttttggttat 3060
tgaagatttg attgtttttt tattttttga tttttggttt tgtatttggg gtggggaagg 3120
taggaggagg tggagagaga gtgggggaaa atagagggtt ttttttgtgt gtagtttaat 3180
taggtgggtt gtggttgtag tgttgaagtt gttttttagt tgtttggttt ttaagagttt 3240
tttttgtaat gtgtagttag tttgtttgtt tgtagttaaa gggagttatt tttttgtaaa 3300
tttgggttgg tagtgttggg ttgatggggg tgggggggtg ggtggggaga agggtggtga 3360
ggggggttgg ttgagagtag ttgattgagg tagttgtggg agagaagatg ggggaggggg 3420
ggaaggtagg gtggggggag gtggggtgga gaggaggagg ataaaggtag ttgttagttt 3480
tattaggtat tttttttgtt ttttttgagt ttttagtagt ttttaaaaat tttattgaag 3540
aggaaagggt agttttgggt ggtggttttg aagttgttgt gttgtaagag gtgttggttg 3600
ttgttggagt tgggttgggg ttggtgtggt gtgttttttt ggtttttgta tagtattgga 3660
gtggttagtt tggttttttt tttttttgtt gttttttttg tttttgggtt tgttttggat 3720
tgttttgttt ttttagtaag ttggtttttt gggattttgg agggggtgtt gggaggtgga 3780

gagtaaatgt aatagtttgt gagttgggtt ttgtttttat tgggttggtt tttttgtttt 3840
tttatttttt tttttttttt tttttttttt tttttttttt ttttttttat tgtgtgttaa 3900
tgtttgttta gtggttttgg agaaataagt gttggtgtag tgtgggtgag ggtaggagag 3960
gttagtttta gttttggagg attttgtttt tttgaatagt ttttgttttt ttaaattaag 4020
gtttaggtgt tagggtttta gattttttgg aggttagtat ttattaaatt tggttttaaa 4080
gtttaattta aaaaattaat agggtggagg tgagggaggg aggaaaagga tgataggaat 4140
atttgttttt ggttgttagg ggtttttttt aggattgata agagtgtgta gatgggagag 4200
tgggagagtt ttttataaat tgaggagtaa gttatgatta agttagtaga tatttgttga 4260
aattttagat tttgttatag ggatagattg ggttttgtgg tattgtgtta tttgttttag 4320
gaatttttag ttttaagaat tagaggttat ttttattttt tttttgtttt ttttttttgt 4380
ttggaattgt ttttaattat gtatttttta tttagatttt taaaggtaaa attatttaga 4440
taagtaggta aatatataaa tattaaaaat atttgttatg tatatatagg tgtttaattt 4500
tgtttagagt tttgtaaatg tttgttttgt atgtagttgt ttgttttttt aagttgtttt 4560
tgtatatttt aaattttata attggaatta tttaaggata gaatttatat agggttgttt 4620
tttgtttttt gatggatagt taggtgtaga ggatgatgtg ggttattgta attgtttttt 4680
tgtggaggag gttttataag ttttgtgtaa aggtggagat gtaagtggga atggaaagag 4740
agagaaaatg taaatgtaaa tttataagga aaatattttt tttttatgtt agtagagatt 4800
ttgggttttt ttatgtttta aatttttttg attttgattt ttggggaggt tttttgtttt 4860
aaaagttatt ttgagatttt tgtttgggtt ttttttgttt tttgtagttt tttatagaga 4920
aaagatgggt tgtagttttg ttgtagtttt attttgtgta tagttaaata ttataggtat 4980
ttatttattt tgattaaaga aggtaattta aatttaaagg taaggaagat tggatatgat 5040
aggttttatt ttatagatga ataggttgaa ataaggatat aatttgtttt atgttttata 5100
ttagtaaaat ttaaattagt tttttagttt attgttattt gagttagatt ttagatttat 5160
taattgtatt gttatttggg aaatggttat aaatgaattt tttagaatat aaaggtttga 5220
agattttttt atttgaggag gtgaaaaatt attttttgag attttaatgg ttatagtttt 5280
ttttgaatat attttattat atgtaagtag aatgtgtaga tattttaatt ttatttgaat 5340
atttattgta atatttaatt ttattataat ttttaattag tattgtttaa taggaattga 5400
gtgggttggt aggatggtag aatggaatta atataggttt tagagttagg agaattatgt 5460
gtttatatat taatagtgaa ggaaggtgta aaaattattg aatgaataat ggataggagt 5520
gaagaggata tatagatata aagaggtttg aaaaaaaatt aaggtgagaa ataatgtttt 5580
gaagttattt tgaataaaaa gtagtttgat atttaaattt gggtttgaaa gattttaaag 5640
tttatgttgt ttttatatta ttaatattgt ttttatagtt tttatatttg aaaattgttg 5700
ttaatataag ttatttttag aaggttagtg tgttatttaa aagatataat gtgtatagaa 5760
taaatttgta aataggaaag tattttataa atttgaagga ttattatgtt atttttattt 5820
ttaaagatgg tgttgttaat aattatatta ttattaggaa aggttaatta ttataagtat 5880
attagggtta tatattttat ttggattttg aattttgatt ttttagttta aattttattt 5940
tttttttatt gtttttaaga ataagagata attattgatt aatattatat aagtttattg 6000
tttgtattag tttttttttt tttttttttt ttgttttttg tagtttttta gtaattaaat 6060
ggaattagag gtgttttttt agattagtta ggttgagagt aagtttttgt tataagtttt 6120
ttttgtaatt ttaagaggga tttattaata aagttttttt tagttttttg ttttggaaag 6180
ttaggatgag ttgatttttg gaaaatggtg attattgggt tttatttttt aagtatttta 6240
tatatttgtt tatttatttt atttttgttg ttgttaattt agtttatttt tagaaggtat 6300
tgtttttttt tatttggagt attgtaaatg gttatttttt ttttattttt ttttagaatt 6360
agttttgtat tgtgtagtta gagggttttt ttttaatagt tgtattgagg tataattgat 6420
atatataata aattgtatat atttaaagtt gtataaattg attatttttg atatatgtgt 6480
gtatttatga aattattatt taattaagat agtgaatata tttatgattt taaaagtttt 6540
tattgtgtta ttttgtatat ttttttttta ttttttttat attttttttt tttaggtaat 6600
taatgatttg ttttttgtta gtatagattg ttttttattt tttagagttt tatgttaatg 6660
gaattatata gtatgtattt tttttttttt ttatttgtat tttttattga gtataatgtt 6720
tttgatgtat atttaagttg ttatgtatat tattagttta ttttttttta ttgttaagta 6780
gtattttatt gtattgttgt attatgtttt gtttatttat ttatttttga tggatatttg 6840
ggtttttttt agtttggggt tattataaat aaaattgtta taaatatttg tataaatatt 6900
tttgtatgga tgtatttttt tatttttttt aggtaaatat ttagttatgg aatgggtgag 6960
ttatagggta ggtatatatt tatttttgta agaaattttt aaattatttt tttaagtgat 7020
tgtattattt tatattttta ttttattaat atttggtatg gttagttttt ttaattttag 7080
tattttaatt agaggtgttt tttttattta atttttattt tttttatgaa taaagatgta 7140
gagtattttt tatgtgttta ttttataatt gtatattttt tttgatgaag attgtaaatt 7200
tttttttttt tttttttttt aattgggtta tttgtaattt attatggggg ttttagagtt 7260
atgtattttg agtataattt ttttattaga aatatgattt aataatattt tatttgtagt 7320
ttgtttttat tattatatta gtgttttttg aagtataaat attttaaatt ttgatgaagt 7380
ttagtttatt atttgtttta tttatgaatt atgttaattt atggattatg gtgttagata 7440
taataaatta tttttaaatt taagattata tagattttta tttgtgtttt tttttagaag 7500
ttgtatagtt tttagtttta tatttaggtt tgtgatttat tttgaattaa gttttgtaga 7560

tagatattta atttttaata tagatgtttt attgttttgg tattattttt tgaaaatatt 7620
attttttttt tattttgttg tttttgaatt tttgttaata attaattttt aatatatatt 7680
tgtatagagg gttttttaga aagtttaatt tggttatatt atttttttgt ttaagttttg 7740
ttattgtttt tttattgttt ttaatagaaa agtttaaaat attaattata ttttatatga 7800
tttgtttttt tgattttatt ttatatattt ttttttttgt tttttgagat ttatttatat 7860
tggtttttat ttaggttttt taatgatttt ataatttttt ttgtttttgt atattttgtt 7920
ttttttattt gtaatgttgg tttatttttt tttttattta tttagttaat gtttatgtat 7980
attagtatat ttaggaagat tttttgaatt atttaggtta gtttaagttt tttatttatt 8040
tattttttaa tatattttaa tagtatttag tatttgttgg aggatttttt ataatttgtt 8100
ttttttattg aattgtatgt tttttaagag taggaattat gttggttttt gtttattatt 8160
gaatttttag gatttagttt ggtgtgggat atggttattg tttaataaat attttttgaa 8220
taaatgaatg gattttattt ttatagaatt aaaattttgt ttttaaattg ggagttttgg 8280
ttttgggggg ttgttaaatg ttttgtgatt ttagtatatt taggtatgta ataaattttt 8340
ttttaattta tgagtaaagt agagaattat tttaatttat gtattgaatg aatatttgtt 8400
aagtatttat tatttgttag gttttgtgtt aggtatttgg aatttattga tgaataggat 8460
attgtggatt ttgtatttaa ggagtttatt tatgatttag gaaggagagt gaagaataat 8520
ttaaataatt atatttgaat tggattggag atgaaaagag gggtttatga ataaattttt 8580
agagttgttg tttttgttga tttgtggata tttgttattt tttttgtata gatgtttata 8640
atggattatt tttttatttt aatatttttt ttgtttttaa tgttgaagtt attattttat 8700
tttatttttt tttatagttt tagttttaat tttttttttg gttgtttttt tttaggtta 8759

<210> 271
<211> 8404
<212> DNA
<213> Artificial Sequence

<220>
<223> chemically treated genomic DNA (Homo sapiens)

<400> 271

aggaagttaa aaattttgaa aaaaggttag aggaattgtt atttaaaata agtagtttag 60
agaagaatat aaatgatttg atggagttga aaaatatagt ataagaattt tgtgaagtat 120
atataagtat taatagttaa agtagaagaa aggatattag agattgaaga ttatttttat 180
gaaataaagt gtgaagataa gattagagga aaaagaatga aaagaaatga ataaagtttt 240
taagaaatag gggattatgt gaaaagatta aatttatgtt tgattgtttt tgtatttgaa 300
agtgataggg agaattgaat taagttggaa aatatttttt aggatattat ttaggagaat 360
ttttttaatt tagtatgata ggttaatatt ttaatttagg aaatatagag aatattataa 420
agatattttt taagaagagt aattttaaga tatataattg ttagatttat taaggttgaa 480
atgaagttaa aaatgttaaa agtagttaga gagaaagttg ggttatttat aaagggaagt 540
ttattagatt aatagtggat ttttttgtaa aaattttata ggttataaaa gatgggggtt 600
aatatttaat atttttaaag aaaggaattt ttaatttaga attttgtatt tagttaaatt 660
aagttttata agtgaatgat aaataaaatt ttttatagat aagtaaatgt tgagagattt 720
tgttattatt aggtttgtat tataagagtt tttgaaggaa gtattaaata tggaaaggaa 780
aaattggttt tagttattgt aaaaatatat taaattgtaa agattattga tattatgaag 840
aaattaatgg gtattaatta atgggtaaat taattggtta gtattataat gataggatta 900
gatttatata taataatatt aattttaaaa gtaaatgggt taagtgttat aattaaaaga 960
tatagattgg taagttggat aaagagttaa gatttattag tgtgttgtat ttagtagatt 1020
tattttatat gtaaagatat gtgttttaaa taagggaatg gaggaagatt tattatagta 1080
atggaaagta aaaaaaaaaa aaagtagggg atgtaatttt agtttttgat aaaatagatt 1140
ttaaattaat gtagattaga aaaagataag aagggtatta tatgatggta tagggattaa 1200
tgtaatatga agagttattt attttaagta tgtatgtatt taatatagga gttattagat 1260
ttataaagtg agtttttgga gaggaagaaa gagatttaga tttttatgta ataatagtgg 1320
gagattttaa tattttgttg ttaatatttg atagattaat gagatagagt aattaataag 1380
gataattagg attttaagtt agttttgtat tatttagatt aaatagatat atttaatata 1440
tatatatata tatatatata tatatatatt ttttgagatg gggttttgtt tttgttgtat 1500
aggttgaagt gtagtggtat gattttagtt tattgtaatt tttgtttttt gggtttaagt 1560
gatttttttg ttttagtttt tagagtagtt gggattatag gtatttgtta ttatatttag 1620
ttaattttgt atttttggta gagatgtggt tttattatgt ttgttagttt ggttttaaat 1680
ttttgatttt aggtgattta tttgttttgg ttttttaaat tgttgggatt ataggtgtga 1740
gttattgtat ttggtttaga ttttgttgaa attttaatat ttttttggta atgtaataga 1800
aaattaaaga ttttgatggt gaatttaatt gtttttgagt tgtgtggatg aaaatatatt 1860
ttattatttt tttttttaat ttttaagttt ttaggtatat gtgtaggatg tgtaggtttg 1920

ttatataggt aaatgtgtgt tatggtggtg tgttgtatag attaatttat tattatggta 1980
ttaagtttag tatttattag tttttttgat gttttttttt tttttatttt tttttgttat 2040
aggttttagt gtgtgttgtt tttttttttg ggtttatttt atggtatgta tgtgtgtatg 2100
tgtgtgtgtg tgtgtgtgtg tgtgtgtgtg tgtgtattta tttatgtgta tatattatga 2160
tatatatatt atattttttt taaattatta ttttaggttt agggatatat gtgtaagttt 2220
gttatatagg taaattgtgt gtttttgggg tttggtgtgt atagattttt ttattattta 2280
ggtaataagt gtagtatttg ataggtagtt ttttgatttt ttgttttttt atttatattt 2340
tttaagtaat tttttagtgt gtgttgtttt tttttttatg tttatgtgtt tttattgttt 2400
agtttttatt tataagtgag aatatgtagt gtttggtttt ttgtttttgt gttagtttgt 2460
tgaggataaa ggtttttggt tttatttatg tttttgtaaa ggatatgatt ttattttttt 2520
ttatggttgt aggaaaatat attttatata ttagtgaata tagttaaatt aatttatggt 2580
taagatatat atttttttgt aaattgagaa atttttttat tataattgta atatggtaat 2640
aaataaattt agattatata aatgtaattt agatgttgat aaattggttt taaataaagt 2700
aagtgatatt tatgtatgaa gagagtttat tttagttttt agaatttttt tttatttttt 2760
tgttattttt atttttagta agtaagtatt tattgtgtgt gtaatattgt gttaggtatt 2820
aaggtaatga tgatatttaa gatttggttt ttattttgaa gataagtata ggagaaatat 2880
tattagttat gatgtttttt gttgtaaata agtgaaaatt ttagtttagt ttaggttagt 2940
tttagggttg gttaaattag aggtttattt ataatattga ggatttagat ttttttgaaa 3000
ttttttttgt attaatatta gtatttttat tttttttgta gggtggtttg ttttatgtta 3060
taatggttgt agtaattaga agtgttatat ttttagtaat atttaatatt attattatta 3120
tttaataata tttagaattg tgttattttt gttttaattt attaggtttt tagtagatta 3180
ttttttattt tttaaatgtt aaaattgtat tttgagtttt tgtttaaatt aatttggggt 3240
gaggtgaatg gaagtagtat tttaattgta tttatttttt gtagttggat ttgggtttgg 3300
gttatttttt gatatttgtt tataagaaag atttttgaag ttaggtagga atggttgttg 3360
agtaggttag tgtttgttaa atttttatat atttttatat atgttataat atgtagataa 3420
aaagataatt ttttatataa tttgttggga taaatttagg aggtttatag tatgatttgt 3480
ttgaaggttt tttttgtttt agattttgtt tagttgtttt aggatgaggg gatttatatt 3540
atagtataat tgtattttat ttatagtata aattattttt ttttttttta gttgatgagt 3600
ttagatgggt aataatagtg tttgttaaag agaaaaaaaa atgtatttaa attagataat 3660
ttattggtat aaatattgag atatagaagt gataatagtt ttaagttaat gtttgatggt 3720
ggtagtttta gtaagttttt ttttgatgtt tttgtgtttt tgtatatgtt ttttttttgt 3780
tattgttttt tttattaaat ataatataat ggataagtgg aattaaatag aattgagttt 3840
aaattttttg ttatttattg gttttggtat tggataaatt aatttttttg agtttgtttt 3900
tttatttgta atgtagatta gttaatatta tttattggaa agtgttgttt tttagttaat 3960
gtatgtaagg tttaaaattt agatgatggg ttgataggtg tagtaaattt ttatggtata 4020
tgtatgttta tgtaataaat ttatatgttt tgtatttgta ttttggaatt taaagtaaaa 4080
aaaaaaaaaa aaaaaaaaag aaagaaagaa aaagaaaaaa aaggttgttt ttggggatta 4140
aataagataa ttatgtaagg tggttagtat agtttttggt atatagtaaa tgttaggttt 4200
gtttgataga tttttattta gtagttattg tttttttgaa aatttttttt agatgttttt 4260
atggtgtttt ttgtttttat attattataa ttttttatta tattattatt tgtttatttt 4320
ttatagtttt tttttttttt ttttttaatt agtgatttta aaaggttagt aagtgtttaa 4380
tattttttat tttttaagtg attggtaaag ttttgtattt attagtgttt taagtttgtt 4440
tttgttttgg ttgattttgt aaaatggatt gggtgggatg agaggtgggg ggtgttgtta 4500
aggagggaga gtggtgtttt tgttgagggt gtattagtta gatatttttt gtggggtttg 4560
agagtttttt ttattagatt ttgggatagg gatgaggttt atagttattt attagatttt 4620
ttgtatagtt ttgttaagtg tattttggtt tggagggggt ggttttgggt agaagtatgt 4680
ttgggttgtg ttggatgttt tttggaaggt gtttggattt atgttaggtt ttttagttta 4740
attttttatg atttagtgtt ttagtttgtg tattgattag tgttttagtt ttttatagat 4800
gttggtgggt ttgggagttt tgtggatgtg atgttgtggg tggaagtgat gtttttttgt 4860
ggttggatgt ggtgtttagt tgttgggtgg gggagggtgt gtttggtttt ttttagggga 4920
tgttgaaatt atttttgtaa tgggagttgg gagaggatgg ggtgtgtttt gatgtgtgtg 4980
tgtgttgttt tttttggtgt ttttttatag tttgttggtt tttgttgtgg aaaggtgtta 5040
tgttgtttaa aattttttga aaaatggttg ttattgttgt tgttgttgtt gtggaatttt 5100
tggtattgtt gttgttgttt ttttttgagg aggatttaga gtaggatagt ggtttggagg 5160
atttgttttt tgttaggtga gtgagtagag ttgttgttgt tttatgtggg aagggtgttt 5220
tgggtgtgtg tagggtgggt gtaaggtggt ttggtgggga tttgtttttg ttaggggttg 5280
ggttttggtg ggaggaggtg ttttttttgt tttttgttat ggtggagtgt ttgtagaatg 5340
gtgataggat tttgggtttt tgggtgaggg gttttggttt taatttgata ggtgttgggt 5400
gggtggggtt agggttttga gtgaagtgat aggtgtagtt tttttttgtg aggtttggag 5460
gtagagggtt gttgtgagtg tttattagat gtaaaaaatg aaaaataaaa atataaaaat 5520
ggtgtttgtg ggagagtttt ttattggaga attggagtat tttggtggtt gtttgatttt 5580
ttgttttggt ttatgtgatg taatagttgg gaagtatttt tttttgggtg tgtattgtat 5640
ttgaagttta tttgtgggag aggttgatta gaaagttttg gataagaagt gtagggtttt 5700

gagtgtttat tgtttatagg atatttggtt taggagtttt gtggtgtttt tttagaataa 5760
taatgtattg aggttttggg gatttaaagt tatttgtagt gattgatgga gtgtaatttt 5820
ttagaggaat tgaaatatgg gtaaaatttt tatgagatat ttattagaag tgtttgaaag 5880
tttttaaatt tttttttttt ttgtttgatg aatttttttt gtgtgttagt tggttttggt 5940
ttgttttatt atttttttta ttttgttttt tgattttgaa ttagtaagga ttttggtgtt 6000
tttttttttt gtttttgttt ttggtataaa attagtggtt ttgtgtgtaa atggaaattt 6060
ttgttttttt ttattaagtg gaatttaaat ttaagtaagt ttatatgaat gtattagatt 6120
ttgaagggaa gtatttattg tattataata ttttattttt tttgattttt ttattttatg 6180
gttaaatagt tatgattgaa agagtgtaat tgttattatt gttagtattg gttttatttt 6240
gagataagtt tattgtagag ggaatgggat ttgtttttgt tttttattat tttttttttt 6300
atttttgttt attattaaat ttttttattt ttatttattt ttttttattt ggtatatatt 6360
aataatttat ggtagaaaag attgaagtgg gatttaggaa atggtttttg gaaggttatt 6420
aaaaatttat atatttaaat ggattgtttt ataggttagt taaaaattat atttgttaaa 6480
aaattaaaat aataataata aaaaattaaa aattatgttt ggggtatgtt ttgggaaaag 6540
atatggtttt agtttttgat taaattttga tgtatttaat ttttgtaaat tttttttggg 6600
aaaatgtagt aatggttatt ttaaagaatt ttatgttatg tatatagttt tggaagtata 6660
ttttaatgta gggtaattag gaatattgat gttgtggtta ttgtttggaa aaaagaggat 6720
tgtttttttt attttttaat ttattttata tattttaagt aatggaatta taaaaaattt 6780
ttttataagt taaaattata taagatatag tttgtttttg attatttata tttataaagg 6840
gttttattaa tatagatagt tgaattttat aaattaatta gtaagggtag aattattgtt 6900
gtgtagtttg atattgagta ttttgtttgg agatggaatt aaggtttagt ttaattgtat 6960
ttttagtgat agattttatt tttgatttga gagtttagat ttttattttg ttgttttatt 7020
tttggtttaa gtaatgtagt tggaagggaa attgttatta ggaatagtat attatattta 7080
ttgttgaaat aattaaaggt taattatgtt ttgagttggg tgtgatggtg tgtatttgta 7140
gtttatgtta tttaggaggt tgaggtagga ggattatatg agtttgggag ttggaggtta 7200
gtttgggtaa taatgtaaga ttttagtttt taaaaaaata aaaaagttga ttaggtatgt 7260
tggtttatgt tgtaatgtta gtattttggg aggttaagat gggaagattg tttgaggtta 7320
ggagtttgag aggagtttgg gtaataaaat aagattttgt ttttaaaata aattttaaaa 7380
aattagttag tggttgtggt atatttgtag gtttatttat ttaggaggtt gaggtgggag 7440
gatttttgag tttaggagtt tgagattgta gtgagttatg attttattat tgtattttag 7500
tttggatgat agagtgaaat ttttatttaa aataagaaaa aaaaataaaa attaattatg 7560
ttttgaagga ggaattggta gtttagaata gtggttaagg gtatggatta tagagttaga 7620
ttgttgggtt tatatagtgg tttatttttt attggttgag attttaggta agttttttaa 7680
tttttttaat ttgatagata tttgtaaatg tttattttta tagatgagaa tatttattta 7740
aaagggttga tgttgggatt tagtgaaatt atatgtttaa ggtatttttt ataggattat 7800
agtaaatatt taaaaattat taattatttt ttatattatt tttattagta gataatatat 7860
agaaattaga tgaatgaaaa tgaattagtt aataagaatt tattaaatgt ttgtttttgt 7920
taaggggaaa ggataaaaat aagtagttgt attatgtaag atgtgttagt aggtagagag 7980
atatatgaaa tattgataag agaagattat agtttaataa agttattggt gttgaattgt 8040
ataatattat ttatgggttt ttattttttt gttgttattt ttattttatt tttttaaata 8100
gttttagtta ttatatttat ttttttttat agaagtgttt tgttgttttg aagtatattt 8160
gatttattat gtaagtaaat attttttatt gtgtggtatt tttattttgg aatgattatg 8220
aaaaagataa ttatatgttt aaatttgaag tgtaatgttt ttttaagata aaataagatt 8280
ttaaagtatt taataatgtt tttttttata gtagtgttat gtgtaaatta ttgaaataag 8340
tatgtattga attaatttga tttataagta tatgttaatt atatgattat ttttatttgg 8400
tagg 8404

<210> 272
<211> 8404
<212> DNA
<213> Artificial Sequence

<220>
<223> chemically treated genomic DNA (Homo sapiens)

<400> 272

tttattaaat gaaaataatt atataattgg tatatattta taaattaaat tgatttagta 60
tatatttgtt ttaatagttt gtatataata ttattgtgaa aaagaatatt attaaatatt 120
ttaagatttt attttatttt agaaaaatat tatattttaa atttaaatat atgattattt 180
tttttatggt tattttaaaa taaggatatt atatagtgaa aaatatttgt ttgtatggta 240
agttaaatat attttaaagt agtaaaatat ttttgtgaag gaaagtaaat gtaatagtta 300
aagttgtttg ggaaaatagg ataaagataa taataaaaag ataaaaattt atagataata 360

ttatataatt taatgttagt gattttgtta aattgtagtt tttttttatt aatgttttat 420
atattttttt atttattagt atattttata tgatgtgatt gtttattttt gttttttttt 480
tttaataaag ataagtattt aataaatttt tgttggttaa tttattttta tttatttaat 540
ttttatgtgt tatttattaa taagaatagt atgaagaata attaataatt tttgagtatt 600
tattatgatt ttgtagaaag tattttaaat atatggtttt attgaatttt gatattagtt 660
tttttagata agtattttta tttgtaaaag tagatattta tagatgtttg ttaaattgga 720
gaagttaaag aatttatttg aggttttagt tggtaagagg taaattatta tgtgagttta 780
atagtttaat tttataattt atgtttttga ttattatttt agattgttaa tttttttttt 840
aaaatataat tagtttttgt tttttttttt tgttttgaaa tagggtttta ttttgttatt 900
taggttgggg tgtagtggtg aaattatggt ttattgtagt tttaaatttt tgggtttaag 960
gattttttta ttttagtttt ttgagtagat gagtttatag gtgtgttata gttattggtt 1020
aattttttaa aatttgtttt agagataggg ttttgttttg ttgtttaggt tttttttaaa 1080
tttttggttt taagtaattt ttttattttg gttttttaaa gtgttggtat tatagtgtga 1140
gttaatatgt ttggttagtt tttttgtttt tttaaagatt ggggttttgt gttgttgttt 1200
aggttggttt ttaattttta agtttatgtg atttttttgt tttagttttt tgagtagtat 1260
ggattatagg tatgtgttat tatatttagt ttgaaatatg attaattttt gattatttta 1320
ataatgaatg tggtatgttg tttttagtga tagttttttt tttagttata ttatttaggt 1380
tagagataag gtaatagaaa taagatttaa atttttaaat taggaatgga atttgttatt 1440
ggaggtgtag ttggattgag ttttgatttt atttttaggt aagatgttta gtattagatt 1500
gtatggtagt ggttttattt ttgttggtta atttatagag tttaattgtt tatgttaatg 1560
aaattttttg tgggtatgga taattaagaa tagattgtat tttgtatggt tttgatttat 1620
aagagaattt tttatagttt tattatttaa aatatataaa atgagttaaa aaatgaaaga 1680
aatagttttt ttttttttaa gtagtggtta taatgttaat atttttggtt attttatatt 1740
agagtatgtt tttaaagttg tgtgtataat atggaatttt ttagagtagt tattattgta 1800
tttttttaag ggaaatttgt aagaattgga tatattagaa tttaattaga aattaaagtt 1860
atgttttttt ttagaatatg ttttagatgt ggtttttaat tttttgttgt tgttattttg 1920
gttttttaat gaatatggtt tttaattgat ttataagata gtttatttaa atatataaat 1980
ttttaatagt tttttagggg ttatttttta aattttattt taattttttt tgttgtgaat 2040
tgttagtgta tattgagtgg aaagaaatgg gtgagggtga aaggatttgg tgatagataa 2100
gaatgggaga gaaaatagtg aaaaatagaa ataagtttta ttttttttgt aatgaatttg 2160
ttttaaagta gaattagtgt tgataataat gataattata tttttttaat tatagttatt 2220
taattataaa gtagaaaaat taagaaaagt aagatgttgt aatataataa atattttttt 2280
ttaagattta gtgtatttgt atggatttgt ttaaatttag attttattta ataagggaaa 2340
atgaaaattt ttatttgtgt atagaattat taattttgtg ttaaaaataa aggtaaaaag 2400
aggggatatt aagatttttg ttggtttaaa gttaaaaggt aaaatggaag aaataatgag 2460
ataagttgaa gttgattaat atgtaagaag agtttattaa ataggaaaaa aaaaagttta 2520
gaaattttta agtatttttg gtaaatgttt tatgaaagtt ttgtttatgt tttagttttt 2580
ttaaagagtt atgttttatt aattattata gatggttttg agtttttaag gttttgatgt 2640
attattgttt taaggaaatg ttgtaaagtt tttgagttga gtattttgtg ggtaatggat 2700
atttaggatt ttgtgttttt tgtttaaggt tttttggttg gtttttttta taaatggatt 2760
ttagatgtag tgtatgtttg gaagaaaata ttttttaatt gttgtattgt gtgaattaaa 2820
atagaaagtt agatgattat tggagtattt taattttttg gtgaaaaatt tttttataga 2880
tattattttt gtattttttat tttttatttt ttgtgtttga tggatgtttg taatgatttt 2940
ttgtttttga gttttataag agggaattgt atttgttatt ttgtttagga ttttagtttt 3000
atttgtttga tatttgttaa gttgaagttg agattttttg tttaagaatt tagaattttg 3060
ttattatttt gtagatgttt tgttgtggtg gggggtaggg agggtgtttt tttttgttgg 3120
gatttggttt ttggtgagga tgggtttttg ttgagttgtt ttgtgtttgt tttatgtata 3180
tttggggtgt tttttttgtg tgaggtgatg gtggttttgt ttgtttattt gatgagaggt 3240
aggttttttg ggttgttgtt ttgttttggg ttttttttag gagggggtgg tggtggtggt 3300
gttgggggtt ttgtggtggt agtggtggtg gtggtggttg ttttttgggg ggttttgggt 3360
ggtatgatgt ttttttgtgg tgggagttag tgagttgtgg aggagtgttg gggaggatga 3420
tgtgtgtgta tgttggggta tgttttgttt ttttttgatt tttgttataa aaataatttt 3480
aatgtttttt gagaaaaatt ggatgtgttt ttttttgttt ggtaattgag tgttgtgttt 3540
aattgtggga aaatgttatt tttgtttgtg gtgttatgtt tgtgaggttt ttgggtttgt 3600
tggtgtttgt ggggaattgg ggtgttggtt ggtgtgtggg ttgggatgtt aagttatgag 3660
gaattaaatt gggaaatttg gtgtgggttt aggtgttttt taggaggtat ttggtgtggt 3720
ttggatgtgt ttttatttag aattattttt tttaggttgg ggtgtattta atggggttat 3780
ataaagagtt tggtgggtga ttgtgggttt tatttttatt ttggggtttg atagggaaga 3840
tttttgggtt ttgtagggaa tatttggtta gtgtattttt ggtgggagtg ttattttttt 3900
tttttggtgg tgttttttat tttttatttt gtttaatttg ttttgtaaag ttggttaaaa 3960
taaaaataaa tttggagtgt tgataggtgt ggaattttat tagttattta gaagatagaa 4020
aatgttagat atttgttggt tttttgggat tattggttag ggaaaggaag ggagggagtt 4080
gtggggaatg aatggatagt agtgtaataa aggattgtag tggtgtaaga atgggaagta 4140

ttgtggaaat gtttgaggaa gatttttagg ggagtagtag ttgttgaata gaagtttgtt 4200
aggtaggttt gatatttatt atgtattagg aattgtgttg gttattttat ataattgttt 4260
tatttaattt ttagaaatag tttttttttt tttttttttt tttttttttt tttttttttt 4320
ttttttttat tttaagtttt gggatataag tgtagaatgt gtaggtttgt tatataggta 4380
tatgtatgtt atggaggttt gttgtattta ttaatttgtt atttaggttt taagttttgt 4440
atgtattagt taagaaataa tgttttttaa tgggtagtat tagttagttt atgttgtaga 4500
tgaggaaata ggtttagagg agttaatttg tttaatatta gggttgatgg gtagtagaga 4560
atttgaattt aattttattt gattttattt gtttattata ttatgtttga tgaagaaaat 4620
agtgatagag aaggagtatg tgtaaaggta taaagatatt agaaaagagt ttgttgggat 4680
tattattatt aaatattggt ttaaagttgt tattattttt gtgttttggt atttgtatta 4740
atagattatt tagtttgaat atattttttt ttttttttgg tagatattgt tattgtttat 4800
ttgaatttgt taattgagaa ggaaagagat ggtttatgtt gtgaataaaa tatagttgtg 4860
ttgtgatgta aattttttta ttttggagta gttgagtaag gtttaaggta ggaagaattt 4920
ttaagtaggt tatgttgtaa gttttttgag tttattttag taagttgtat aagggattat 4980
ttttttattt gtatattatg atatatgtga ggatgtgtaa aggtttggta agtattggtt 5040
tatttaatag ttatttttat ttaattttag aaattttttt tgtgggtaaa tgttaggaga 5100
tagtttaggt ttaggtttag ttataaagaa tgaatataat taaagtgtta tttttattta 5160
ttttatttta gattagttta ggtaagagtt taggatgtag ttttgatatt tgaaagataa 5220
ggagtagttt gttgggagtt tgataaattg aaatagggat ggtatagttt taaatattat 5280
taaataatag taataatatt aagtattatt gaggatgtga tatttttgat tgttgtagtt 5340
attgtaatgt gaggtaagtt attttataga gaaggtagaa atgttgatat tggtgtagga 5400
ggaattttaa aagaatttgg atttttagtg ttataaatga gtttttgatt tggttagttt 5460
tggagttgat ttaagttgag ttgaggtttt tatttgtttg taatagaaag tattatgatt 5520
gataatgttt tttttgtgtt tatttttagg atagagatta aattttgggt attattattg 5580
ttttagtatt tagtatagtg ttatatatat agtaaatgtt tgtttattaa aagtagggat 5640
ggtaggaaag tgaaggaaag ttttaggaat tgaagtaaat tttttttata tgtagatatt 5700
atttgtttta tttaaaatta gtttattaat atttagatta tatttatgta atttagattt 5760
atttattatt atattataat tatgataaag agatttttta atttataaaa gaatatatgt 5820
tttgattatg gattaattta attatgttta ttagtatgta aaatatattt ttttgtagtt 5880
ataaaaagga atgagattat gtttttgtta ggaatatgga tggagttaga agtttttatt 5940
tttagtaaat taatgtagga atagaaaatt aaatattgta tgtttttatt tataagtggg 6000
agttaaataa tgagaatata tggatatgaa gaggggaata atatatattg ggggattatt 6060
tgagggtgta gggtgagagg atagaagatt aaaaaattat ttattaggta ttatatttgt 6120
tatttgggtg gtgaaaaaat ttgtatatat taaattttag agatatatag tttatttata 6180
taataaattt gtatatgtat ttttaaattt aaaataatag tttgaaaaaa gtgtggtgta 6240
tatattataa tatatatata taaatagata tatatatata tatatatata tatatatata 6300
tatatatata tatatatata ttataaaata gatttaggaa ggggaataat atatattggg 6360
gtttgtagtg gggaagggtg gggagaggga gagtattagg agagttaatg gatgttgggt 6420
ttaatattat ggtgatgggt tgatttgtgt agtatattat tatagtatat atttatttgt 6480
gtaataaatt tgtatatttt atatatatat ttgggagttt aaaagttgaa ggaaaaaata 6540
ataaaatata tttttattta tataatttag aagtaattga gtttattatt aaaattttta 6600
gttttttgtt gtattattaa agaagtatta gaattttggt agagtttaga ttaggtatgg 6660
tggtttatgt ttgtaatttt agtagtttgg gaggttaagg tgggtggatt atttgaggtt 6720
aggagtttga gattaggttg gtaaatatgg tgaaattata tttttattaa aaatatgaaa 6780
ttagttgagt gtggtggtag gtgtttgtaa ttttagttat tttggaggtt gaggtaggag 6840
aattatttga atttaggagg tagaggttgt agtgagttga gattatgtta ttgtatttta 6900
gtttatgtga taagagtaag attttatttt aaaaaatata tatatatata tatatatata 6960
tatatatatt gaatatgttt atttagtttg aatggtgtaa aattgatttg aagttttgat 7020
tatttttgtt aattattttg ttttattgat ttgttaaata ttgatagtgg ggtgttaaag 7080
ttttttatta ttattgtgtg ggagtttaag tttttttttt tttttttaag gatttgtttt 7140
atgaatttgg tagtttttgt attgggtgta tatatattta ggataggtag ttttttatgt 7200
tgtattgatt tttatattat tatgtaatgt tttttttgtt tttttttgat ttatgttggt 7260
ttaaagtttg ttttattaga gattaggatt gtattttttg tttttttttt tttttgtttt 7320
ttattgttgt ggtaaatttt tttttatttt tttatttaga gtatgtgttt ttgtatgtga 7380
gatgagtttg ttgaatatag tatattgatg ggttttgatt ttttatttaa tttgttagtt 7440
tgtgtttttt aattgtggta tttagtttgt ttatttttaa ggttaatatt gttatatgtg 7500
aatttgattt tgttattatg atgttagtta gttagtttgt ttattagttg atgtttatta 7560
gtttttttat agtgttaatg gtttttataa tttggtatgt ttttgtagtg gttgggatta 7620
attttttttt tttatattta gtgttttttt taggagtttt tgtaatgtag gtttggtggt 7680
gataaaattt tttaatattt gtttatttgt gaaggatttt atttattatt tatttataaa 7740
gtttagtttg gttggatata aaattttggg ttgaaaattt ttttttttaa gaatgttgaa 7800
tattggtttt tattttttat ggtttgtagg gtttttgtag agagatttat tattagtttg 7860
atgggttttt ttttgtaggt aatttgattt tttttttggt tgtttttaat atttttggtt 7920

ttattttaat tttggtgaat ttgatgatta tgtgttttgg ggttgttttt tttgaggagt 7980
atttttgtgg tgttttttgt attttttgaa ttggaatgtt ggtttgttgt gttaggttgg 8040
ggaaattttt ttggataata ttttgaagag tgttttttaa tttggtttaa ttttttttgt 8100
tatttttagg tatagggata attaaatata ggtttggttt ttttatatag ttttttattt 8160
tttggaggtt ttgtttgttt ttttttattt tttttttttt aattttgttt ttatatttta 8220
ttttataaag gtgattttta atttttgata tttttttttt tgttttggtt gttgatattt 8280
gtgtatgttt tatgaagttt ttgtgttgtg ttttttagtt ttattaggtt atttatgttt 8340
ttttttaaat tgtttatttt aggtagtaat ttttttaatt tttttttaag gtttttagtt 8400
tttt 8404

<210> 273
<211> 5312
<212> DNA
<213> Artificial Sequence

<220>
<223> chemically treated genomic DNA (Homo sapiens)

<400> 273

gttggttttg ggggttgatg attttttggg taggtgatgt gttttgttta ggttttattt 60
tttgtgtttg taaaatgggg tggataatgt agtttaaggt agagttgtgt taaggttttt 120
tgttgttgtt gggttttggt ggtttgattg ggtttggggt ttgagtgtgt ttttgggttt 180
gggggggttg ttgtgatgga tttgttggta gtgttttagg attgtttggt ggagtagttg 240
ttgttgttgt ggatttaggt ttagaggtgg tttaaggtgt gtgtgtggag agggtggaat 300
gtgggtttgt gattttgtgg gttttgggat ttggtatggg gtttttgttt ttgtgtgtta 360
tggtttgtta gtgtttaggt ttagtttttt tttttgtagt ggttttgtag gtttgtggtt 420
ttatggtggg gtgatgtgtt ttgtttaggt tttattttat gtgattttaa aatgtggtgg 480
agaatgtggt tgtgggattt ttttaggggt tttaagggtt tgtgggggtt gggtgtttgg 540
gtgtgtgggt gggggaggtt tggtgttgtg gtttataaat aggtgttgtt ttgttattgt 600
tgttgttgtt gtttttgtgg ttggatttgg gttaggtggg gttttttgtt ttttggtatt 660
ttttggaggg gtgagtgttg aggtttttag tttttttggt ttatggaggg ttttgagtgt 720
gttttagggg ttttaatttt gttgtttttt gtttgaggag ggaggatatt gtttgtgttt 780
gttgtggttt gatatttttt tatggaataa atatagtgtt ggtttttgta gagttttttt 840
tgggattggg gttaggggtg tttttttttg ttattatttt gtttgttttt ttatttttag 900
gtttttgttt atgggttttt tttttatttt tgttaatgaa atgttttatt ttgggaattg 960
tattttttgt tgttttttta tattgtattt ggtaagagat agggtattgg gtttttggag 1020
aaatttttgt gtttttattt tgttttattt tgtttttagg atattgataa gtagtatgtg 1080
ggttttgtta tattgtttaa ttaggtgtat tgtaagttgg tgaagaaagg ttttgatttt 1140
atatttatgg tggttggtga gtgggttagg ttttttgggg gagtggttgg ggttattggt 1200
tagttaagtt ttgttgttgg agttttagat ttaatgtagt tttttttttg tatttgtgtg 1260
taggtgagtt aggtttgggg aagtttatat tggtttatag tttttttttg atagatttgt 1320
ataaggattg gaagttgttt agtgttgagg gtgagtggtt tttaggaggt tttggtattg 1380
atttttagtt tttttttatg ggattttttt aaggattttt ttttaggttt agtttttatt 1440
gttttgtttt tgtggtgtgg gttttttggg ggtagggtta aggtattaag atggatgagg 1500
atgaggtttt tggttgttaa gggtgagggg ttgagggttg gagaggtttt tttagtggtt 1560
ttttttttgt agagtgtatt agttagatgg tagagatttt aaaatatatg gtggatattg 1620
aggagaaggg agttaagttg aagtttatta ttgtggatat gttgggattt ggggatgttg 1680
ttaataatat tgagtggtga gtgaggtttg ttgagaaagg ttttgtttag gtggttatag 1740
tatttgaggt ttggttttat tttttttttg tttatagttg gaagtttatt attgattatg 1800
tggattagta gtttgagtag tatttttgtg atgagagtgg ttttaattga aagaatattt 1860
aagataattg agtgtattgt tgtttatatt ttattttttt ttttgggtat gggtgtgtgg 1920
ttgttttggg gttaggtttg ggagtgtagt ttttataata tggttttttg gttgtgttta 1980
tgtttatttt tggttgtttt tggtaggttg tggttagtgg atgtgggttt tatgaaggta 2040
ttgtatgaga aggttaatat tgtgtttttt attgttaaag ttgattgttt tgtttttagt 2100
gagatttgga agttgaagga gtgggtgagt ttgttgttgt ataggggttt ggttagggtt 2160
ttggggttga gagtattagg gggatttgtt tggttttaaa tttgatggtt tttgttttat 2220
tatagatttg ggaggagatt gataagtttg ggatttatgt atattagttt tttgagtgtg 2280
atttggatga ggatgaggat tttaagtagt aggattggga attgaaggtg aatatgtaga 2340
ttggtggggt aggggggatg gagttggtga ggggtagaat tagagggttt tgtttttttt 2400
atattgagtt tgttggagga gggttaggtt agtttagttg ggtgtaagag ttatttgttt 2460
taggagtaga ggatttgtat tttttttatt tagggttaga aggtaaaggt tattatgttt 2520
ttgtttggtt ggggtgggtt atgtgggttt tgtgtggtgt ttggtaggta tggagtattt 2580

gtttagattt agaggtgtag tagtgggtta ggtttttaat tttaattttt tttttatggg 2640
ttattagagg aaggggttgt tttagtggta tattttggtt tttagatttt tgagttttag 2700
ggttggtggt tttgggtgtt ttggtagtta gagtgatgat ggagattatg ttaggggtag 2760
gtggttatta gggtaggggt attagtatgg ggagatatag gtttagtttt ggggagggaa 2820
ggggtagttg gagggggtgg tttttgatga gggaggagat gaggagggag tatgtttggt 2880
tgggtatagt ttggaaaggt tttggaatgt gagttatatg ggttttggtt ttagttaggg 2940
gtagagaggt tagaaagggg taatgttagg atttttttga ggagagatag ttgatggtga 3000
tgttggaggg gtgttttggg agttatatgt ttgtttttta ttagtaattg tgtaattatg 3060
tttattgggt gtgagttttt tttttttttt tttttaggag agtgtgtttt ttgttgttat 3120
aggtagtaat atggtggtgg aggttaaggg gtagtgggtt tggggttgat tgtatttttg 3180
ggggattgtg gagggtgagt agagttttgg ggtattaggt ttggtggggg aaggttgttt 3240
tgggttggtg ttagtttatt atttattttt attttgtagt ggagaattag gtgtattgtg 3300
attttgtgaa gttgtgtaat atgtttattt gtatgtatat gtatgatttt aaggatgtga 3360
tgtgtgatgt gtattatgag aattattgtg tgtattgtat ttagtagatg attaggtgtg 3420
tgttttagtt gtgagttaga ttttgttttt ttggttttgt ttatgttttt ataattgagg 3480
gttggttttg ttagtttatt tagatttgaa ttttgtatta ttttttaagt tttttttttt 3540
ttttagagtt tggttttttt agaattaagt ttagggttgt gagggttttg gagggtaggg 3600
ttttagtagt ggtggggatg ggttaggtat tgttagttta tgttgagttt tttggtggtg 3660
ttgttttgtt tatttttttt tttgttttgt gtagtaaatt gatttaggat agttgtatgg 3720
agagttttat tttgattttg ttgttgttta ttttggatgt tgagattgag aagtttatta 3780
ggatgaagga tgaggaagta tgtggggtgg tgggggtggt ggaggtgggt gttagggatg 3840
tttttttgtg gtgttgttta tttgttgggt tgtttttgtt tgtagttgag gtgtatgtag 3900
gagatgttgt agaggatgaa gtagtagatg taggattagt gatgtttgtt gtggatatat 3960
tgtttgtttt tgggatgttt ttgtattttt ggatattaga ttggattgtt tttgatttgg 4020
agatgtgggg ttatagtttt tagttgattt taatttattt ttagtattat ttttttttag 4080
gttattgtgt ttgtttttga ggggtttgga ttgtagtttt tgtttagttg gttttttttg 4140
attttggggg attaggagtg aagttgggtg ggattttaga gatttgtttt ttttgttttt 4200
tttttgtttt tggatggtta tagtatttaa attgtaggtt ttgttttggt aggtaggtaa 4260
agttaggtag aagaggattt ttaggatttt gggtttgttt tttgttttag tgttgtagaa 4320
tggatttggg agtttttttt tgtttgtttt tgtgggttat ttagtgagtg ttgagatttt 4380
attttttgtt gaggtgggtt gagttttttt ttatttttag atgtttagtg ggtagggttg 4440
ggttgaatta aatgggagtt ttttagatat aaggaggtta gaggttgtaa ggagtggggt 4500
tgtgattgtt tatatttttt ttttatagtt tggtttgatt tggagggttt ttggggtatt 4560
gggtggtgag ttattttttg gtaatttttg gtgttgtttt ttgtttttgt ttgaggtttt 4620
ttttttttag tattgttgtg gtgtgttggg attttgagtt taggtttttt gatgttttta 4680
tttgtatgat ttttttttgt tatatgatgt tttgtttttt tttgttgtga atgttgtgtt 4740
ttgttttggt gataggagaa taatgttggt gaatgttgta gtgggtgttt gagtgttttg 4800
tgtgtttttg agagtgggtg ggagtggaag tttgagtggt ttgtggtttt tggtgatagt 4860
gtgttatttg ttgttgtagt gtgtgtttgt gtggtttttg ggttattttt ggttaggttg 4920
tagtattgtg gttggtgtgg gtgtggtagg ggtggggtgg ttttattgtt tggttttttt 4980
gtttatgttt tttgttgtag agtaagttgg gttgttgttt ttttgttatg ggttttggtg 5040
agtttggagt ttggttgggt ttttggttgt tttttaggtt gatttgggtt gagaggagtt 5100
ttggttgttt ggttgtagtt gggagagatt tgggttagat ttagagggga tttttagttg 5160
gtgtgtgggg tggttagggt ggagaggttg gtgggttatt gggatgttgg gtattagggg 5220
ttggatggag ttgggttggt agtttgggta tttagagatg ttgtttaggt gtttgttgat 5280
tgtttggttt attgtggtgt tttttttgta gg 5312

<210> 274
<211> 5312
<212> DNA
<213> Artificial Sequence

<220>
<223> chemically treated genomic DNA (Homo sapiens)

<400> 274
   tttgtaaggg aagtgttgta gtaagttgag tggttggtgg gtatttgggt gatatttttg 60
   agtatttaga ttgttggttt ggttttattt agtttttgat gtttggtgtt ttggtagttt 120
   gttagttttt ttattttgat tattttgtat gttggttgga agtttttttt aagtttgatt 180
   taagtttttt ttagttgtag ttaaatgatt agagtttttt ttaatttggg ttggtttagg 240
   gagtagttgg gggtttgatt ggattttaga tttattggag tttatggtga gaagatggta 300
   gtttggttta ttttgtagtg ggaggtgtag gtgggagagt tgagtgataa ggttgttttg 360
   tttttgttat gtttgtattg attatagtgt tgtggtttgg ttagaaatag tttagaggtt 420
   gtgtggatgt gtgttgtagt ggtagatagt atattattgt tggaggttgt aggttgttta 480
   ggtttttgtt tttatttgtt tttaggggta tatggagtat ttggatattt gttgtgatgt 540
   ttattaatat tgtttttttg ttattaggat aggatgtggt atttataatg gaagaaaatg 600
   gagtattgtg tggtgggaag ggattatgtg ggtgggaata ttgggaggtt taggtttagg 660
   attttggtat attatagtgg tgttggggag aagaggtttt gagtgagggt aggggatggt 720
   attgagagtt gttaggaggt ggtttattgt ttagtgtttt gggggttttt taggttgggt 780
   tgggttgtgg agaaggggtg taagtggtta tgattttgtt ttttgtagtt tttggttttt 840
   ttatgtttgg agggttttta tttgatttag tttaattttg tttgttaggt gtttggggat 900
   aagggaagat ttggtttgtt ttgatagaaa atggggtttt agtatttgtt gggtgatttg 960
   tgggagtagg taaaggaggg tttttaagtt tgttttgtag tattggggta gggaatagat 1020
   ttaggatttt gggaattttt ttttgtttag ttttgtttgt ttgttagagt agggtttgtg 1080
   gtttgggtgt tgtgattgtt tgggggtggg ggaagggtaa gggaggtgga tttttgaagt 1140
   tttgtttaat tttgtttttg attttttaag gttagagagg gttagttggg tgggggttat 1200
   ggtttaggtt ttttggaaat agatataatg atttgggagg gggtggtgtt gagaataaat 1260
   tagggttagt tgggggttgt ggttttgtgt ttttgggttg ggaatagttt ggtttggtgt 1320
   ttaggggtgt gagggtgttt tggagatgga tggtgtgttt gtggtgagtg ttattggttt 1380
   tgtatttgtt gttttatttt ttgtagtatt ttttgtatgt gttttagttg tgggtggaga 1440
   taatttggtg ggtgagtagt attgtggagg agtatttttg atgtttgttt ttgttgtttt 1500
   tgttgtttta tatatttttt tattttttat tttgataagt tttttagttt tggtgtttgg 1560
   ggtgggtagt ggtaggattg ggatggggtt ttttatgtgg ttgttttggg ttagtttgtt 1620
   gtgtggggtg ggggggagga tgggtggggt ggtgttattg ggaggtttag tgtgggttgg 1680
   tgatgtttgg tttatttttg ttattgttga ggttttgttt tttggagttt ttatagtttt 1740
   ggatttggtt ttagggagat taggttttgg gggagggggg gggtttaggg aatggtgtaa 1800
   agtttaagtt tgggtgggtt gataggattg gtttttagtt atggagatgt gggtggggtt 1860
   agaggggtga ggtttggttt gtggttgggg tgtgtatttg gttatttgtt ggatgtagtg 1920
   tgtgtggtag tttttgtagt gtatgttgta tgttatgttt ttgaggttgt gtatatgtgt 1980
   gtggatgagt atgttgtgta gttttatgaa gttgtaatgt gtttggtttt ttattgtggg 2040
   gtgggggtgg gtagtgggtt ggtgttggtt taggatagtt tttttttatt agatttggta 2100
   ttttaagatt ttatttattt tttatgattt tttaggggta tagttggttt tggatttgtt 2160
   gttttttggt ttttattatt gtgttgttgt ttataatggt gaagggtgtg tttttttggg 2220
   ggaaggaggg agaaaggttt atatttggtg agtgtaattg tatggttatt gatgggaaat 2280
   gggtatgtaa tttttggggt atttttttag tattattatt aattattttt ttttaaagag 2340
   attttggtgt tgtttttttt tggttttttt gtttttggtt ggagttagag tttatgtggt 2400
   ttatatttta gggttttttt aggttatgtt taattaggta tgtttttttt ttattttttt 2460
   ttttattaag aattattttt tttagttgtt tttttttttt ttagggttga gtttatgttt 2520
   ttttatgttg atgtttttgt tttggtggtt atttgttttt ggtatggttt ttattattat 2580
   tttagttgtt ggggtattta aagttattaa ttttggagtt tagaaatttg ggagttaggg 2640
   tgtgttattg gggtagtttt tttttttggt gatttatggg aagagggttg gggttggggg 2700
   tttggtttat tgttgtattt ttggatttgg gtaagtgttt tatatttgtt aagtattata 2760
   tggggtttat atgatttatt ttagttaggt aaagatatgg tgatttttat tttttagttt 2820
   tggatgaagg gggtataggt tttttatttt tagaataaat gatttttgta tttaattggg 2880
   ttgatttggt ttttttttag taggtttaat gtgaaggaga taaagttttt tggttttgtt 2940
   ttttattagt tttatttttt ttgttttatt agtttgtatg tttattttta gtttttggtt 3000
   ttgttgtttg aagtttttat ttttgtttga gttatattta gggaattggt atatatggat 3060
   tttaaatttg ttaatttttt tttggatttg tggtggggta aggattatta gatttgaggt 3120
   tagataagtt tttttggtat ttttagtttt agggttttgg ttaggttttt gtgtgatggt 3180
   aggtttattt gtttttttag tttttggatt ttattgggga taagatagtt agttttggtg 3240
   atgagaggta tgatgttgat ttttttatgt aatgttttta tgaaatttat atttattggt 3300
   tgtagtttgt tgagagtagt taagggtggg tataggtata gttagggggt tatattgtag 3360
   gggttgtatt tttgagtttg gttttaggat agttatatat ttatgtttga agggggagat 3420
   gaagtatagg tagtagtgta tttggttgtt ttggatgttt ttttggttga ggttgttttt 3480
   attatggaag tattgtttaa attgttggtt tatatagttg gtgatgggtt tttagttgtg 3540
   ggtaggaggg aggtgaggtt aggttttgag tgttgtggtt gtttaggtaa ggtttttttt 3600
   agtaggtttt atttattatt tggtgttgtt gatagtgttt ttgaattttg gtgtgtttat 3660
   gatggtgagt tttagtttga tttttttttt tttaatgttt attgtgtgtt ttagaatttt 3720
   tattgtttgg ttgatgtgtt ttatggggaa ggggttattg gaagggtttt tttaattttt 3780
   agttttttat ttttggtagt taggattttt gtttttattt attttggtgt tttggtttta 3840
   tttttagggg atttatattg tgagaataga atagtgggag ttggatttga aagggagttt 3900
   ttggagaggt tttatggaag gggattgggg attagtgtta gggttttttg ggggttattt 3960
   atttttagta ttgagtagtt tttggttttt gtataagttt gttaggaaga ggttgtggat 4020
   tagtgtggat ttttttaggt ttgatttatt tgtatatagg tatagagaag gagttgtgtt 4080
   aggtttgggg ttttaatgat agagtttggt tggttagtga ttttagttat ttttttgagg 4140
   agtttggttt atttattagt tattatgagt gtgaagttaa agtttttttt tattgatttg 4200
   tggtgtattt ggttgggtag tgtggtgaag tttatgtatt gtttgttaat gttttgggga 4260
   taggatggaa tagggtaggg atataaaggt ttttttagaa gtttaatatt ttgtttttta 4320
   ttaaatgtgg tatgaggaga tagtagaggg tatggttttt agggtggggt attttgttgg 4380
   taaaaataag gggagggttt gtgggtaaaa gtttggagat aagaaggtag gtgaggtggt 4440
   ggtaggagaa ggtatttttg gttttaattt tagggagggt tttgtaggga ttgatattgt 4500
   atttattttg tggggagatg ttaggttgta gtaggtatag gtgatgtttt ttttttttgg 4560
   gtagagggta gtaggattgg gatttttggg atatatttaa ggttttttat aagttggaga 4620
   agttgggggt tttgatattt atttttttgg agagtgttag ggggtgggag gttttatttg 4680
   gtttggattt agttgtgggg gtggtggtag tggtggtggt ggagtagtgt ttatttatag 4740
   attgtggtgt tgagtttttt ttgtttgtgt gtttaggtgt ttagtttttg tagatttttg 4800
   gggtttttgg aggggtttta tggttgtatt ttttgttgtg ttttggagtt gtgtgaggtg 4860
   aggtttggat agggtatgtt gttttattgt ggggttgtgg atttgtgagg ttgttgtgga 4920
   gggaagggtt gagtttaggt gttggtgggt tgtggtgtgt ggggatgggg attttatgtt 4980
   gagttttgga gtttgtgggg ttatagattt atgttttgtt ttttttatat atgtattttg 5040
   agttgttttt gggtttgggt ttgtggtgat agtagttgtt ttattaggtg gttttggggt 5100
   gttgttagtg agtttattgt ggtgattttt ttaggtttgg gggtatgttt ggattttagg 5160
   tttggttagg ttgttagagt ttggtgatag tgagggattt tggtgtggtt ttgttttagg 5220
   ttgtgttgtt tattttgttt tgtggatgtg ggaagtgagg tttggatagg gtatgttgtt 5280
   tatttggggg attgttgatt tttgaggttg at 5312

<210> 275
<211> 3025
<212> DNA
<213> Artificial Sequence

<220>
<223> chemically treated genomic DNA (Homo sapiens)

<400> 275
   tttttatgat ttgattttta tagttgtttt atttatgggt tttttataaa ttaggggtat 60
   agaggagtat tgaaagttag tttagaggtg agtgtgtgta gttagtgttt tttgtggata 120
   tagtagttgg gtgttggaga ggtttggaaa gggtgtgttg gagagttaag tgttagttgt 180
   ttagggtttg ttggttgttt tttttttttt tgtttggtag gggatttagt gtgtatgtta 240
   gtgtggaggg gtgggttggt tggttagttt tgggtttttt ggttattttg gggatttttt 300
   ttaagttttg tttttgagtg tttttattgg ttttggattt tttttttttt agttgtttgt 360
   ttgggttttg gggtgtttag gttattatgg ataaatagtt tagggtgttt ggttgagaag 420
   ttaggggtga ggaagttttg gggtgttgtt gttgtttttt ttaattataa attaggttgg 480
   ataggagagg gaggggtggg ggatagtggg tggggattta gattgttagt attttgttat 540
   ttatagttgg ggtttttgag tggtagaaag ttttggttat tttttgttgt ttgggttggg 600
   tgaaagttag gattgtgttg tgttattgtt aggatatgga gttattgttg ttattgtttt 660
   gtgatgtaga tttgatggtt tttgatggtt ttttttgttt ttttgttata atggatgatt 720
   tttatgatga tttgtgtttt gattttttgg atttgtgttt ttttgaagat ttggatttgt 780
   gtttgatgta tgtgggtgtg tttttgaaat ttgaagagta tttgtatttt tttgtggtgg 840
   tgtatttggt tttgggtgta tgtgaggatg agtatgtgtg tgtgtttagt gggtattatt 900
   aggtgggttg ttgtttattg tgggtttgta aggtgtgtaa gtgtaagatt attaatgttg 960
   attgttgtaa ggttgttatt atgtgtgagt ggtgttgttt gagtaaagta aatgaggttt 1020
   ttgagatatt taagtgttgt atgttgagta atttaaatta gtggttgttt aaggtggaga 1080
   ttttgtgtaa tgttatttgt tatattgagg gtttgtaggt tttgttgtgt gattaggatg 1140
   ttgtgttttt tggtgttgta gttgtttttt atgtgttggg tttgttgttt ttgggttgtg 1200
   gtggtgagta ttatagtggt gattttgatg tgtttagttt gtgttttaat tgttttgatg 1260
   gtatggtaag gttgggattt taggaagtga ggaagttagg gtggtgtttg ggatattagg 1320
   gatgtgtttt tgagggtggg gagttggttt tgtgggaggt ttgggttagg attttttttg 1380
   agagagagga tttttttgtt ttgggtagtt gttattgggg tagtttgttt tggaagtgtg 1440
   tgggtaagtg tttgagttgt tttattgggg gtgttattag aatattgtag tgtgaatgtg 1500
   aagatttttt tttttattta tttttatttt taaaatgtaa atttgtgttt tttggtgatt 1560
   gtttgttttt ggtttggttt tgtatgttgt agattttatt ttttatttat ttgtaattat 1620
   ttttttaatt aggataggtt tgggtttgga attagagttt taggttagag ttagggaggg 1680
   ggtggttata ggaattggtg tttgggtttt gagttgtttt gtgggtttga tttagttgtt 1740
   tttgttgttt gtagatggat tatagtggtt ttttgagtgg tgtttggtgg tggaattgtt 1800
   atgaaggtgt ttattataat gaggtgttta gtggtgggta ttttgggttt ttttttgttt 1860
   gttttttttt ttttatggag ttgttttggt ttttatttag gatgttttta tttttattta 1920
   tatatgttta tgttttggga agtggtgtag gagatgaaat attaagtaag tagttttttg 1980
   tttttttgat tgttttggat tttaattaaa gtttttagtt tttaatttgt tttaaagtat 2040
   tgggtttggg ggtgggaggt ttgttgtggt tttatttttg tttattaatt gagttttttt 2100
   tgtgtagaat ttaggtttgg gaagagtgtg gtggtgttga gtttagattg tttgtttagt 2160
   attgtggagt gtatttttat tgagagtttt gtggtgtttg tttttttgtt ggtggatgtg 2220
   ttttttgagt tgtttttgtg taggtaagag gttgttgttt ttagtgaggg agagagtagt 2280
   ggtgatttta tttagttatt ggatgttgtt ttgtagtgtt ttgtgggtgt gaattttaat 2340
   ttgatatatt aggtgttttg aggggatggt ggttgtttat ttgtttgagg gatggtgttt 2400
   ttagggtttt ttgtgtttaa aagattgaat ttaaatgttt tttttttaat agtgttttaa 2460
   aagtgatttt ttttgaggta ggagaggtgg gagaattgaa gtttttgttt ttgttttata 2520
   gggtaaggat atagtgtggt tttttttatg tagtattttt tttggagatt tattgtgatg 2580
   gttgttttgt gttttttggt gggttagagt tgaattttga ggggttaggt ttagtttttt 2640
   tgtgtttttt tttatggggg tgagattttt gtagatttaa ttttgttttg ggatgtattg 2700
   gttatttggg ggggtgtgag atttagtgta ttttggtttt aaatgtagta ggtgtaattg 2760
   taatttattt ttaatttgtt ttttggttta ggattatttt ttgtaatatt tttgtaattt 2820
   atttttgtaa ataagagttg ttttgttaga gtaggagttt ttggggttgt atttattttt 2880
   gaggtatggt gtgtggtgtt atagggaatt tgtatgttta tattgtaggt gggtgagttg 2940
   tgggtgtttg tttaggtgat taaaataaag gtgttaattt atattgttgt ggttttggtt 3000
   ttttttggat atgggtgtgg gattt 3025

<210> 276
<211> 3025
<212> DNA
<213> Artificial Sequence

<220>
<223> chemically treated genomic DNA (Homo sapiens)

<400> 276

ggattttata tttatgttta gggaaagttg gagttatggt ggtataaatt agtgttttta 60
ttttgattat ttgagtgagt gtttgtggtt tgtttgtttg tggtataaat gtataaattt 120
tttgtagtat tatatattat gttttagaga taaatatagt tttaggggtt tttgttttgg 180
taaagtaatt tttatttata ggaatagatt ataaaagtat tataaaaagt ggttttgaat 240
tgggaaatgg gttgggggtg ggttatggtt atatttgtta tatttgggat tggagtgtat 300
tgggttttat gtttttttaa ataattggtg tattttgggg tagggttagg tttgtgaggg 360
ttttattttt atgggggagg gtgtgagaaa gttgaattta gttttttaag gtttagtttt 420
ggtttattga ggaatatgga gtggttattg taatgggttt ttgagaaggg tgttgtgtgg 480
aaaaaattgt gttgtgtttt tgttttgtgg ggtgggggtg gaaattttag tttttttgtt 540
ttttttattt taagagaagt tgtttttaaa gtgttgttgg gaggggggta tttaagttta 600
attttttggg tgtgagggat tttaggggta ttattttttg ggtgggtggg tggttattat 660
ttttttagag tatttggtat attgggttgg ggtttgtatt tgtagggtat tgtggggtgg 720
tgtttggtga ttgggtgggg ttgttgttgt tttttttttt gttgggggtg gtagtttttt 780
gtttgtgtgg aggtgattta gaaggtatgt ttgttagtag gagggtgggt gttgtagggt 840
ttttggtgga gatgtgtttt atgatgttgg ataggtagtt taggtttgat attgttgtat 900
tttttttggg tttgggtttt gtgtggggag ggtttggtta gtaagtaggg gtggggttgt 960
gataagtttt ttatttttgg gtttagtatt ttgagataga ttggaaattg aggattttag 1020
ttagagtttg ggataattga aaagataggg agttatttgt ttagtatttt attttttgta 1080
ttatttttta ggatataggt gtgtgtgagt gggggtggga gtgttttaga tagaggttag 1140
gatagtttta tgaaggagga ggagtgagta gggagaggtt tggaatattt attgttgggt 1200
gttttgttgt agtaggtgtt tttgtagtag ttttgttgtt gggtgttgtt tggggggttg 1260
ttgtagttta tttgtaaata gtaagggtga ttgagttagg tttgtgggat ggtttgaggt 1320
ttgaatatta atttttgtag ttgttttttt tttaatttta gtttaaggtt ttagttttgg 1380
gtttagattt gttttggttg ggggggtaat tatgggtggg taggagatga ggtttgtaat 1440
atgtagggtt aaattaaggg tggatagtta ttaaggggtg taaatttata ttttggaagt 1500
agggataagt agagaaaaag atttttatgt ttgtgttgta gtgttttaat agtgttttta 1560
atggggtagt ttgaatgttt gtttgtatat ttttaaaata ggttatttta gtgatagttg 1620
tttaggataa gggggttttt ttttttggga aggattttgg tttaaatttt ttgtaaggtt 1680
agttttttgt ttttggaaat gtgtttttga tattttgagt gttgttttaa tttttttatt 1740
ttttggggtt ttggttttat tatgttgttg gagtagttgg agtgtgggtt ggatgtgttg 1800
gagttgttgt tgtagtgttt gttgttgtgg tttgggggta gtgggtttgg tgtatagaag 1860
gtggttgtgg tgttaggggg tgtggtgttt tggttgtgta gtagagtttg taggtttttg 1920
atatagtgga tggtgttgtg taggattttt attttgggta attgttggtt tggattgttt 1980

gatgtgtagt gtttgagtgt tttaaaggtt ttatttattt tgtttaggtg gtgttgtttg 2040
tgtatggtgg tggttttgtg gtggttggtg ttggtggttt tgtgtttgta tgttttgtag 2100
gtttatagta ggtagtggtt tgtttggtgg tgtttgttgg gtgtgtgtat atgtttgttt 2160
ttatgtgtgt ttggggttgg gtgtattgtt gtggggaagt gtgagtgttt tttgggtttt 2220
aggagtgtgt ttatgtgtat taggtgtggg tttaggtttt tgaagaagtg taggtttggg 2280
gagttgaaat atgggttgtt atagaagttg tttgttgtgg taaaggagta gagagagttg 2340
ttgggggttg ttaggtttat gttgtggagt ggtggtgata gtagttttat attttggtgg 2400
tggtgtggta tggttttggt ttttgtttaa tttaagtggt agagagtggt tggaattttt 2460
tgttgtttgg gagttttggt tgtagatagt aaagtgttgg tagtttgaat ttttatttat 2520
tgttttttat tttttttttt tttgtttggt ttgatttgtg gttaaggaaa gtggtggtag 2580
tgttttaggg tttttttatt tttagttttt tggttaggtg ttttgggtta tttatttgta 2640
gtagtttaaa tgttttggag tttaggtggg tagttggggg agggggagtt tgaggttaat 2700
aggaatattt gggggtgggg tttggggggg gtttttgggg tggttgaggg gtttgagatt 2760
ggttagttag tttgtttttt tatattggtg tgtgtgttag gttttttatt gggtagggag 2820
ggagggagtg attggtaagt tttaggtggt tgatatttgg ttttttggta tgtttttttt 2880
aaattttttt aatatttgat tgttgtattt gtgggaaatg ttggttgtgt gtgtttattt 2940
ttgagttgat ttttaatatt tttttgtatt tttgatttgt gggggattta tgggtagagt 3000
ggttgtagaa attaggttgt gagag 3025

<210> 277
<211> 11913
<212> DNA
<213> Artificial Sequence

<220>
<223> chemically treated genomic DNA (Homo sapiens)

<400> 277
   taaagtaggg tgttattaaa gaaagaagga agaaattggt taaatgaatt tagaatataa 60
   ataaaattat tgtataaata aaatttttgt ttatgattaa gttggaatgg tggttaaaaa 120
   agaaaaggga aaaaaaattt ttagatgtag gtagattttt ataattaagt tattttttaa 180
   aggggaagta aatgtgatag aatatttagt aagtaaattt attgaggaat attttgtttt 240
   tatataatta aatttaatat tgaggtaaaa ttgataatta ttaaattgag ggtagttggg 300
   agagggaaag gggatgagat aaaatttagt ttataaaata ttttattatt ttttataggt 360
   taaaaaatat aaaagaaata tttggaaaga attgtttaag atggaaagat tttaagattg 420
   tgtgattttt tttttttttt tttttttttt ttttttttta taggtgtaga ataatttttt 480
   tttttttttt tgtggttttt ttttttttta tttttttttt ttgttgtttg taaagttgaa 540
   tttggggtta ggagttttaa ttgtaatttt agattggaat tttagatggt gtgtttagga 600
   ttggaagtaa tttttagttt attaatttta tgggtttatg tatttgtttt ggttatggtt 660
   tggtttttat ttttattgtg gtattaatgt agaataaatt gagatatatt ttttatataa 720
   ttagttttag aatttaaaaa gatttatggt ataataatag aggtagtgtt gtggaatttt 780
   tttattgagg atttttgggg agtttgggga ttattttgtg gtggtattag aatagatgag 840
   taattttagt ggttttaggt attgtgaatt gggggagagg gggaaatttt gtattgtatt 900
   tggtagtatt taatggtttt gggtttttag atagggttag tgaggagagt taggatagta 960
   agtggggatt ggttaaaaaa aattgattta attttgaaat gattagaaga agttgggagt 1020
   agttattata aagttttttt tttgtttttt ttttttttta attgaaggaa tgatgttgaa 1080
   atttttattg ttagtgtggg gttgggaatt attttaaata tttgttttgt agttttgagt 1140
   gggtagattt tgaagagttg gagttggtgg ggaggggagg aatagttgtt gggttgtttt 1200
   gtgtgtgggg tttggggtgt gggttttggg gagaggtgtt agttgaaagt ttgttttttt 1260
   tttgtgatag gtagtagtga ggttgagtta ttttttatta tggtttgtgg gttttgtgtg 1320
   tagtgtggtt gtttttgttt ttgattgatt gtagtgtttt ggggtgttta tttagttgtt 1380
   tgtggttgtt gggtgatttt tggagggtgg tttaggtgtg tgtattggtt tttggggttg 1440
   tggttgtagt agtttagttt tgtgttttgt tagatgtagt ggatagtgtt ggggtgaaag 1500
   taagggttga gaaattttta ttgttttgtt tttatttagt tttgattttt ttatattgta 1560
   ataattatgt atttttgagg ttgggatgtt tgtgttttgt tagtgtatta atgttatatg 1620
   gttttttagg ggttgttttt gtttttgtat tttattagtt ttgaagaggt agaggggtgg 1680
   tgggtaaaat ttttaattgg atgttgagag atttgggttt tagtttggtg ttgttattaa 1740
   tttgtttttt gtttttaaat agtagtagta ataaaaattg taaagttgat tgagttaaag 1800
   gtttatattg tttttttatt taatttttag aataatttta tgaggtgggt gttagtattt 1860
   tattattttg tttgattagt aaggaaattg aggtttagag agattgtgga atttgtttag 1920
   ggttatatag ttgttggtta tttggtagag ttgaggtttt attttggggt tattaatttt 1980
   ttgtttttta ttgttgtgtt ttatagattg tttttttttt ggttgtttgg gttttagttt 2040
   ttttttttgt aaaatgagaa gtttaatttg tagttttttt tgttaattgg tggtttagat 2100
   taggaatttt tgttatagat tttggtgttg ataggggtaa gaaggtgggt gaaagaaggg 2160
   ggtgtttagt tttttatagt ttgttttttt ttgggatttt gtgaagggtg ggttttgtga 2220
   gttaaaggag gtataggaga gtgtttattg tttgtggtgg gtgaaggtgt tatttgtttt 2280
   tgtgttaggt tgtgagtttt ggtgtttagt ttagggtgtt aaggttagtg tagttggtat 2340
   gttttttttg gaaaatttta ggttttttgt agagaatgtt atttataata aagaagagga 2400
   tagagaggta tggagtgttt tgtgattgta ggagtatgtt agttttttag tgttggttta 2460
   gtgttgtttg ggtttttggg tatgtggatt tgttggggtt gtatggagat tttttgttgg 2520
   gtttttgggg ttttttgatt gtggtttttt agtttagtat tttttttttg gttttgtagg 2580
   ttgtagggaa ttttttttat ttttttagtt ggagaagttt aagttgggtg agggggtatt 2640
   ttggggtttg tattggtgtt tttttttttt ttgtttttat aaggattttg agaaaataaa 2700
   tggtagagga gagaggagtt ttatatttgt ttggtttttt ttttttttta tttattttta 2760
   tattttttat tttggggtaa aaatttattt ttgaaaaatg ttggtagaga tttatgtgtt 2820
   tttgttttat ttgggtttta taaatataat gatttatatt taagttagtt tttttttaga 2880
   taattttttt tttttttgtt aaaagtgtta aggatggtaa aagaagaaat aattttaatt 2940
   tttttgtttg gaaatgaaag tttttggttt tttataaagg gttttttgtt ttttatagtt 3000
   gagttttagt taagaaaaat gatttttaag tagaaataat aggtggggag aaggaaggga 3060
   gatataggga tttggggggt ttttagggta attggtagtg aattttgttt tgagagtttt 3120
   ttttttattt aaaaaattaa atgtgtgagt tttgtgaaag gtttagggat agattgtgtg 3180
   ggagaggatt gagtagagag tgtgggggta gtgttttgta gaattttttt ttttttaata 3240
   ataattttaa aagtttttga gtggagatga tgtaaagtta agtagtaaag gtggtttggg 3300
   aggtaagtgg agggtttaag tgttgtattt ttatttttag ggttttttgt gtttatggga 3360
   tgtgtatttt taagaagtgt gttttttgag taagttttgg gtttgtatat attttgggtt 3420
   tgtagttaga atttaatggt gataatgttt atgtaatgta agttaaaaat taaagtgtaa 3480
   aaaattatta tgttatttat tgaaatgtta ttttttgtta aattgtaatt attttgtttt 3540
   atataagttt ggttggaaag tttgtagttt tagtttgggt tagttaggta taggaggttg 3600
   gattgtaatt ggttgttttt tttttgttgt gtttggttgt tttatgttgt gttgttgttg 3660
   ttgttttttg gtgtttttgg gattttatat gtatttttga aatatgtttt gttttggttt 3720
   ttggtttttt tttttgttta ggggttgttt tttaatagat attgattttt ttagaagatt 3780
   taaaaattaa attaaaatat tttttatttg ttttaaatat ttgttttggg gtgtgggggt 3840
   tgttaaatag agattagatg aagggagtta gatttagtga agttttttga gtttttaaag 3900
   atttgaatat taatttgtgt ttgtgggttg atggaggttt tttttatttt attttttggt 3960
   ttttttaatt ggtttttgtt ttttggttaa ttattgagta attagaatgg tatttttgat 4020
   tagggttata ggtagtgttt ggtggagtgg ttttaggagt tatttgtttt ttgttgggtt 4080
   ttgtatttaa attttttttt ttattttttt tttttaaatt gggtgttagg atgttttggt 4140
   tggaatatat gtaggttttg ggtgtttgtt taagggtttt tttttttttt aaattagttg 4200
   ttgttttttt ggtttaattg tagaagaatt agatattttt tattggaaag ggaaattaag 4260
   tgttgttgat tttaatttta ggtaggtggt aattgttttt tgtttggtgt aaattttatt 4320
   aagtaaataa ttattagttg attgaaatat gtttggttta taattggtgt aatttttggt 4380
   tatttaattg agggatgttt gtttttagtt ttgatttttg gaatttataa agggttattt 4440
   ttttttttag tgattttaag attatggtta tttttttatt tgatagtttt agaagtaaga 4500
   gttagattta agggtgtaaa gtaagggtat atgttttttt gaagtttgat tgagtttttt 4560
   ttgtgttttt ttgaagtttt tgtttttttg gagtttattt gttttttttt ttaaattatt 4620
   tttttagatt aataatttta tttttatttt tattgtattt gattttgttt ggatttattg 4680
   tttatttgaa tggatttttt agtgagatga ggtttttata ttggtgaagg ttaagaaggg 4740
   gaggtggggg gagggttgtg ttatattggt tagttgagag tgtgtgttgg gttgaagagg 4800
   agggtgtttt tgagagggat gtttttttgg atttgttttt attttagttg tgagggtgtt 4860
   tttaaggagt agtgtgtgtt gtttggttgg gtttgggttg ttgagtgaat ggagtggttg 4920
   agttttttgg tttttttttt tttttgtgtt gttggttttt tttatttgag ttttgggaag 4980
   ttgagggtag ttaggtagtt ggggtaagga gtttaaggta gtgtttatat ttgggggttt 5040
   tttgtaattt gattgtttgt ttgttttttt attttttgtt ttttttttta tttatttatt 5100
   tatttattta tttatttaga gttgggatgg tagtttaggt gtttgggttt tgttgttttt 5160
   ttgttgtgat tttggatttt tttttgttgt aggatttggt ttttatgtgt gttttggagt 5220
   tggtgtttta gtatatgttt tgttttgggt ttgggtgttt atagtagtta gagtagtagg 5280
   gagtttggga tttgggtggt atttgggtta agttaggtgt tgttgaggtt agtgttgaat 5340
   gtttttaggg ttggaggagt tgtggggtgt ttgggtttga gttgtagtaa atgggttttg 5400
   atgtgtggga tttgaatgtg ttgttgtttg ttgttttttt tttgggtggt ggtggtggtt 5460
   gtgttttgtt tgtgagtggt gtggtgtagt gggtgttggt gttggatttt gtgtttttgg 5520
   gtgttttggt ttatgggttg ttgggtggtt ttgtgttgtt attggttttg ttgttatttt 5580
   tgttgttgtt gttttatttt tttattaaat aggagttgag ttggggtggt gtggagttgt 5640
   atgaggagta gtgtttgagt gtttttattg tttatttttt tggttagttt attggtatag 5700
   ttggagtttg ttgttatggg ttttttggtt tttttttgtt tagttaggtg ttatttggtt 5760
   aggttaggat gttttttaat gtgttttatt tgtttagttg ttttgagagt tagtttgtta 5820
   tttgtaatta gggtaagtag gttggggagt gttttttatg tgtggggtag tggtgttagg 5880
   gattttttgt tttaggatat tttttttttt tatttttttt gattgtagtt tttatttagt 5940
   tgttttttaa gggttgtgag gatagtggaa gtggtggttg gggaggaggt tggagagtgg 6000
   gagtgtatgt aggtattggt ttttgatatt ttttaaagtt aggtagagtt aggagtttga 6060
   ttgtttgtaa gagttgggag ggtgtttggg gttttttaga gtttgagaga ttttgggatt 6120
   gttagttttt atgggagtgg aattaggaga ttggtgtagt gtttggtttt gttttgggga 6180
   gagttttgtt aattaaggaa atgttaaatt gttattaagt gtagtgtttt ttagatgttt 6240
   ggattttgga tttggagtgg gattagtgtg ggggtgagtg tttgtgttgt gttgtttttg 6300
   tagtgttaaa atgtttagga gtatttttag tttttataat atggtgttgg tatatattta 6360
   ggtgtatatt aagtattgta gagaattaga atgagtatat gtatttttag taattaaatg 6420
   gttttttgga atagtgttgt gtttgtggat agtttttgtg tatggtaata tagaagtgga 6480
   tttttattgg gttttgttta ggtggtggtt atgtaggttt tggggatgga agagaatttg 6540
   atttatttga gtttaagtta tgtgggatta ggttttattg taggtagtgg aaggaggtat 6600
   gagttttggt ttttttttta gtgtatttgg ggtgtttttt tattttgtaa atgtggggtt 6660
   tagttgggat agtatttttt taagatttgg ggttagaata aggatagggt tatttgttgt 6720
   gtttagttag gatataagga gggaatttgg tatatgataa gtattaggtt ggttttttat 6780
   tttgtgttga tgtttgttta tttttttttt gtttgggtgg tgtggatttg aatttgtgga 6840
   gtttttgtag tttgtttttt ttatttattt ttagtggatt tttatgttat tgagaattta 6900
   agttagtttg gtttgatttg gtaatttagt aaatgtttta ttaatataat ggtggatgtt 6960
   ttttttggag taaaattatt tgagggttta gggattggga tgtttttgga atgtgtggga 7020
   agtagagtgt attttttgta ggaggtattt ttgtttaagg tttgggagaa gggttttgtt 7080
   tttgttgttt gtttttattt gtgtttttta aatgtgtgtt tttaggttgt ttttatttta 7140
   gtttaattag gagtatttgg tttttgtgaa gttttggaga aattgaaatt gttttttttt 7200
   ttgggagaat ttgggattat tgttttgtgg aggaattgta agtagattta agggttgttt 7260
   ttattttatg tttttagtta gtgaaattag tgttttgtat ttaaggggtg ttgggataat 7320
   ttattggttg ttgaggtttg tgttttttga ttagattgtg ttatttttgt ttagtaggtt 7380
   ggtgttattt ttggggagtt tttaggtttt agtgtttttg tttataagag gatagttgag 7440
   atgggtaatt tggagattgg gtttggtttg aagttatatt ttatagatgt agaagtgtta 7500
   tttattattt ttttggggtt ttaaatatgg aatttggtaa tttatttaaa tatttgtgat 7560
   tgagaaataa tgaatttgag agtattgttt gtatgtgtgt tatttggagg tggttgagaa 7620
   attattgaat ttatgttttt ggggtataaa atattattat gttattattt ttttagtatt 7680
   tttaggggat ttttgaaatt tgggttttgt tgttttgttt gggttttttg tttagttttt 7740
   tttagggtag gtttggtgtt tgggatttta ttatatgttg tggggggtgg gattgtgggt 7800
   tttgtagtta gtaggtatta tatttttgga aaatatttgg atttttttgt aatatatagg 7860
   tgtggttgtt atggagaaaa agttaaaagg ggattaggat tagtaggtga atgtattttt 7920
   tttttttatt ttggagaaaa agtttaatgg agagagggtt ttagatttaa gttatatagt 7980
   ttggaagtat tatttttagg tttaagtttt gttttgtagt gaaaatagat gggtttattt 8040
   tatttagttt ttgaggttta ttgggtagtt tggatgtgag aagtgttttg gttgttattt 8100
   tggagtgggt aagggtggtt tttgagattt tgggtttatt ttttggattt ttagggtttt 8160
   tgttgttttt ttaggaatgt agggtttggt ttaagatttg ggttgtgaga ggtaagggta 8220
   ttaattttta aaaatgtgga aatggttatt agagtgttaa ggttaaattt taggggagag 8280
   gaattaaggt tgtttttgga tttttagggt agtttagtta tggtgtatag gtaggggtaa 8340
   ttgttggtat ttatttataa ttgtttaaaa ttttataagt atgtttatat tttaaatttt 8400
   taaatattat attttgtatt ttaattttgt aaatattaat ttagtaaata ttaattgttt 8460
   atttggttag gtattgggtt ttgaatatta attaagataa tggttttgag aatttttgta 8520
   agaagtttat tagttaattt tggagaggtt ttttgttttg gatgaggaaa tttaggtttt 8580
   atgagattag gtggtagagg tggatttgga atttagatta tgttagggtg tttttatttt 8640
   ttttttaggg attttggttt tattgttgag atggattttt tttggtttgt gggaggggta 8700
   ttaaattttt agtttatgtg tgggggttga tgggtttgtg agggtagatg tggggtgagg 8760
   atttgagtag ttttagatta gatgtaaggt ttaaggagga ggggaaaaaa gtttgtttgg 8820
   gttggatgtg gtggtttgag gtggttggat tatttgaggt taggagtttg agattagttt 8880
   ggttaatatg gtgaaatttt gtttttattg aaaatataaa aaaaaaaatt ggttgggttt 8940
   ggtgatgggt gtttataatt ttagttattt gggaggttga ggtataagaa ttgtttgaat 9000
   ttgggaggtt gaggttgaag tgagttgaga ttgtgttatt gtattgtagt ttgggtgata 9060
   gagtgaaatt ttgttttgga aaaaaaaaaa aaaaaaagag gttgtttgtt agtttattga 9120
   ttttgtagtt ttgtaggttt gtagaatttt aaggtatgat taggtttggg attattaggt 9180
   tattttaggg ttaggtgtgg aggtttgtga tgtttgaggg ttaaaggttt ttttttttgg 9240
   ttatatagga gagggtattt tgttggggga taggtttttt ttaattatag tgtagttgta 9300
   gttattgttt ttagtttttt gagtattttt ttggttgagt ttgttgttta ttttattttt 9360
   tagtttttat ggtttggtta ggttaggtgg tttttggttt ttttgtagtt gttggtgggg 9420
   gaggagggta tagagtgtgt tttgtttttt agtgtatggt taaggttgtg tttttttttt 9480
   gggttgggat taattgtggg aaaggtgtta ttttggttat ttgatttttt ttttttaagg 9540
   tttgtataag gagtttttga atatgttgtt ttttttagtt ggtagtttta ttttttttgg 9600
   tttttgtttt tgtatttttt gtgttaggtt aagtgttggg tttagggata gtgtgtttta 9660
   attaaatttg tgtttgataa gtttttaatt aaaagagatt tttttttgtt ttgtaggtgt 9720
   ttttattatt gttttttttg gggggatttg aaggagaggg tttgaggttg gtttttgttt 9780
   gttgaggttt gtgtgtttgg tttgggagga ggtttaggag ggtttgtggg ttatgggtat 9840
   ttttgggttt gagttttggg gtgtggatgg atgttttgag agtgggtgtt gtgatttggg 9900
   atttatggtt tttgttgggt atggatagtt gtggagtagg gttttgagga ttgtgtagtg 9960
   ttttgggttt ttgtttattt ttgggtttag gaatggagaa gggtttgttt agagaaaatt 10020
   tttttgtagt ttttattttg tttttttttt tttttttttt tttttttttt tttttttttt 10080
   tttttttttt tttttttttg tgtttgtttt tttttttttt tttttttttt tagtgttttt 10140
   ttagaaattt ggtttttatt ttgggtaata gtgggtgttt tgagttaaat tttgtttgta 10200
   agtttattta taggttttaa agaaggttag gttttaggtt ttaaaaggaa gtttgtggaa 10260
   gtttaaatgt gtttgtttat gtaggagagt taatggggtt gggggttatt tttgtttatt 10320
   tagtaggaga gaaatggagg tttttgaggt tatatgataa aagttagtgg ttttttaaat 10380
   ttttagtgtg tggtgttgag tagtgtgtga gatttaagtt tggatttgta ttatttggga 10440
   gttaggattt gttttttttt tttttttttt gtattttgtt atattatttt ttaggtttat 10500
   gtttttgttg ttttagagaa gttttttttt tttttttttt tttttttttt tttttgggtg 10560
   gggggtggtg gtgtgtgtgt atgagtgttt gtgtgtgttt tgaaaatgga ggtaatgatg 10620
   ttttgggttt tgtttattgg ttgagttttg tttaagtttt tggaggtatt ttggttaaat 10680
   agtgtaagag tgggagaatg ggtgttgttt gatttaattt atttttgaga attttgtgaa 10740
   gtttggagat ttagggtagt tttgggttag gaggatttga ggttgttggg gtggttttgt 10800
   taggatttta taggagtagt tttttttagt tattgtgttt ggattttagg ttatagattt 10860
   tggaatttgt agaggaatga gattttattt ttgggttttg gggttaatga tgtattttta 10920
   gttgaaggat tttttttttt agaggtagta atagtttttt agaagtttgt ggggtataag 10980
   tttaaagtaa tttaataata aaaattataa aagattatgt tttttaattg aggttttatg 11040
   tttttttttt tattttgggg ggggggggga atgttaggat ttttttaggg ggaaattgag 11100
   gtaaaggatt gaagtattat tatggaggga aagggagata tagtgttagg tatattttaa 11160
   aggaaattgg ttgtttagtt tggggagtga ggaagttggt ttttatagta gtggttaggg 11220
   ttggattttt gggtgtaagg tgggttttat aggggggagg aggaggagag tgtagggttg 11280
   ttatttgaag gtttaattgg aaggggtttt tgtttttttt tttattagtt tttttttggg 11340
   tgttatggtg ttaggttggg tagttttaga tagataatat tatttggtaa ttattttttt 11400
   tatttttaag attaggttat ttaattggtt ttgagtaatt aatgaaagtt ggtgtgtggg 11460
   tttttgattt atagtttgga gttaatgtgg ggttagttta gtagatttat tttgtttgtt 11520
   tgtgttgttt ttgtggaaga gttgtagttt atttttgggg ggtgaataat tttttttgtt 11580
   gattaggtaa tgagttttat atttaatttg ttaggggttt gttgtagaag tggtagagat 11640
   tgtagaagat tgtggtataa ataaattggg aaaaagtgag gtgggggttt tagttagaaa 11700
   aggatggaga agtttgtttt aaggttatat ttagggattt gggttgtttt tttgttgaaa 11760
   gttttggagg tttgtggggt tggatttttt tttgttgggg gtttgaatgt ggggtttagt 11820
   gtatattttt tttgtttgtg gttggtttag atttattgtt ttgttttgtg agagtattgt 11880
   tgatattgtg tttttttttg ttgtttttgt agg 11913

<210> 278
<211> 11913
<212> DNA
<213> Artificial Sequence

<220>
<223> chemically treated genomic DNA (Homo sapiens)

<400> 278

tttgtgggag tggtggagag aagtatagtg ttagtggtgt ttttgtaaga tggggtagtg 60
ggtttgaatt agttatgggt ggggggggtg tgtgttgaat tttgtatttg gatttttagt 120
ggaggagaat ttagttttat aagtttttag gatttttggt aggaagatgg tttaagtttt 180
tggatgtgat tttgggatag gtttttttat ttttttttga ttaaaatttt tattttgttt 240
ttttttaatt tatttatgtt gtagtttttt atggtttttg ttgtttttgt agtgaatttt 300
tggtaaatta agtataaaat ttgttgtttg attgatagag aaggttgttt attttttaaa 360
aataaattgt agtttttttg taggagtagt gtgggtaagt gaaatgaatt tgttgggtta 420
attttgtgtt gattttgagt tgtaaattag gggtttgtgt attaattttt attaattatt 480
tggagttagt taaatggttt ggttttaaaa atgggaagag taattattga gtaatgttat 540
ttgtttgggg ttatttgatt tgatattatg atatttaagg gaaagttggt gagggggaaa 600
ataaggtttt tttttagttg ggtttttaga tggtgatttt gtgttttttt tttttttttt 660
tttatgggat ttattttgtg tttaggggtt tgattttgat tattgttgta ggagttaatt 720
tttttatttt ttaggttgga taattaattt tttttagagt gtgtttgata ttgtattttt 780

tttttttttt atggtagtgt tttggttttt tgttttagtt tttttttagg gaaattttag 840
tatttttttt tttttttaaa gtgagaaaaa gagtgtggag ttttagttgg aaaatatagt 900
tttttataat ttttgttatt aaattgtttt aaatttatat tttataagtt tttggaaagt 960
tgttgttgtt ttttgggaaa aggatttttt ggttaaagat atgttattgg ttttggaatt 1020
taaggatggg gttttatttt tttataagtt ttggagtttg tggtttgaag tttagatata 1080
atggttaagg agagttgttt ttatgggatt ttggtagggt tattttagta gttttagatt 1140
tttttggttt aggattgttt tgggttttta aattttataa aatttttagg agtaaattgg 1200
gttaaatagt atttattttt ttatttttgt attatttgat taaagtgttt ttaaagattt 1260
aagtaggatt tagttgatga gtaggattta gggtattgtt atttttattt ttgaaatata 1320
tatggatgtt tatgtatata tattattatt ttttgtttag aaaaaaaaaa aaagaaaaag 1380
aaaaaaaaaa agtttttttg gggtaataga ggtatgagtt tggagggtgg tgtggtagag 1440
tgtaagggag ggaaggaggg ggtaaatttt aatttttgga tagtataggt ttaggtttaa 1500
gttttatata ttgtttagta ttatatattg gggatttgag aagttattag tttttattat 1560
atgattttga gggtttttat tttttttttg ttaagtgggt aaagataatt tttggttttg 1620
ttagtttttt tgtatgggtg agtatgtttg ggtttttata gatttttttt tgaaatttga 1680
ggtttggttt tttttgaggt ttatagatga gtttataaat gaaatttggt ttagggtatt 1740
tattattgtt tggagtgaag gttgaatttt tgaaaggata ttaaaaagag agagagagaa 1800
agagagatag atatagagag agagagagag agggagggag agagagagag agagagagag 1860
agagagagag agatgaaata gaagttatga agaagttttt tttagatgaa ttttttttta 1920
tttttgagtt taggagtagg tagggattta gggtattgta taatttttag agttttgttt 1980
tgtaattgtt tgtgtttggt gagggttgtg ggttttgagt tgtggtattt atttttgaga 2040
tgtttgtttg tattttagaa tttgggttta aggatgtttg tggtttgtga gtttttttag 2100
gttttttttt agattggata tgtagatttt ggtgggtaaa gattggtttt aaattttttt 2160
ttttaggttt ttttgggagg ggtagtggtg aaagtgtttg tggagtggag ggaggttttt 2220
tttaattaga agtttattgg atatgggttt gattagagtg tgttgttttt gggtttagtg 2280
tttggtttgg tgtggagagt gtgggggtag gggttagggg aggtaaagtt gttggttgga 2340
aggagtagtg tgtttaaagg ttttttgtat aggttttgga gggagagaat tgagtggtta 2400
aggtagtgtt ttttttatgg ttagttttgg tttgggaggg gagtgtaatt ttgattgtgt 2460
gttaagaggt gagatgtgtt ttgtgttttt tttttttgtt ggtggttgta gagaagttgg 2520
gaattgtttg gtttggttgg gttgtgagag ttgaggggtg aaatggatag taggtttagt 2580
taggaaggtg tttagaaggt tgaaaatgat gattgtagtt gtattgtgat tggggaaaat 2640
ttgtttttta ataaaatatt tttttttgtg tgattggaag agggagtttt tggtttttgg 2700
gtgttgtagg tttttgtatt tgattttagg gtggtttgat gattttaggt ttaattatgt 2760
tttagggttt tgtgagtttg tggggttgtg ggattagtgg attagtaggt aatttttttt 2820
tttttttttt ttttttgaga tggagttttg ttttgttgtt taggttgtag tgtagtggtg 2880
tgattttagt ttattttaat tttagttttt tgggtttaaa tgatttttgt gttttagttt 2940
tttgagtagt tgggattata ggtgtttgtt attaagtttg gttaattttt ttttttgtat 3000
ttttagtaga gatagggttt tattgtgttg gttaggttgg ttttaaattt ttgattttag 3060
gtgatttaat tgttttgagt tattgtgttt ggtttaagta agtttttttt tttttttttt 3120
taagttttgt atttagtttg gggttattta agtttttatt ttatatttgt ttttataaat 3180
ttgttaattt ttatatatgg attaagagtt tggtgttttt tttgtaggtt agaagaaatt 3240
tattttaatg gtggggttaa gatttttgga aaaggagtag aaatatttta gtatggtttg 3300
ggtttaaatt ttatttttgt tatttaattt tatagaattt ggattttttt atttaaaata 3360
aggggttttt ttaggattag ttggtgagtt ttttgtaaag gtttttgagg ttattgtttt 3420
aattaatatt tagaatttag tgtttgatta agtaggtgat tgatgtttgt taaattaatg 3480
tttataagat taggatgtaa gatataatat ttaagaattt aaaatatgaa tatgtttgtg 3540
aagttttggg tggttgtgag tgggtattaa taattgtttt tgtttgtgta ttatggttag 3600
attgttttag ggatttagag atggttttga tttttttttt ttggggtttg gttttggtgt 3660
tttgatggtt atttttatat ttttgagagt tgatgttttt gttttttata gtttaagttt 3720
tgggttaggt tttgtatttt tggggaagta gtaggaattt tggaaattta aagaataaat 3780
ttagaatttt gagggttatt tttgtttatt ttaggatagt agttggagtg ttttttatat 3840
ttaagttgtt taatgagttt taagggttgg gtaagatgga tttatttgtt tttattgtaa 3900
gataaaattt aaatttggag atggtgtttt taggttatat gatttgaatt tagggttttt 3960
tttttattgg gttttttttt tagggtggag aagaaggata tatttattta ttagttttgg 4020
ttttttttta attttttttt tatggtagtt atgtttgtat attatagaag aatttagata 4080
ttttttagaa gtgtaatgtt tgttggttgt aaaatttata gttttatttt ttatgatatg 4140
tgataagatt ttaggtatta gatttgtttt gaaaagggtt ggataaggga tttaaatgaa 4200
gtgatagaat ttaggtttta aaaatttttt agaagtatta aaaagataat ggtgtagtag 4260
tattttgtgt tttaggggta tggatttgat ggttttttaa ttgtttttaa atagtatata 4320
tgtagatagt gtttttggat ttattgtttt ttagttatag atgtttagat gggttgttga 4380
gttttatatt taaagtttta agagggtggt gggtagtgtt tttgtattta tggagtataa 4440
ttttaagttg gatttaattt ttaggttgtt tattttagtt gtttttttat agatggggat 4500
attgagattt agaaattttt taaaagtaat attagtttgt taaataaagg tggtgtgatt 4560

tgattaaaga atataagttt tagtgattag taagttgttt taatgttttt tgagtataaa 4620
atattaattt tattaattaa aagtatagag tggaggtaat ttttgggttt gtttgtggtt 4680
tttttatagg atagtgattt tagatttttt tgaagaaaag ggtggttttg atttttttaa 4740
ggttttgtgg gggttgggtg tttttggtta ggttaaggta ggagtggttt gaagatgtgt 4800
gtttagaagg tgtgggtgaa ggtgggtaat aagggtagag tttttttttt gagttttggg 4860
tgaaggtatt ttttgtaaaa gatatatttt gttttttatg tattttaaaa atattttggt 4920
ttttaggttt ttgagtaatt ttgttttagg aaaagtattt gttattgtat tagtaaagtg 4980
tttattaaat tattgaatta aattgaattg gtttaggttt ttaatagtgt ggaaatttat 5040
tgaaaataaa tgaagagggt aaattatagg ggttttgtag gtttgggttt gtgttgttta 5100
ggtgaaagag aggtgggtgg gtattggtgt gggatgagaa attaatttga tatttattgt 5160
gtgttgagtt ttttttttgt attttgatta agtatagtga ataattttgt ttttgtttta 5220
attttaggtt ttgaagaaat attgttttag ttgagttttg tgtttataag atgaagaggt 5280
gttttagatg tgttgaaaga aaggttaaag tttgtgtttt tttttattgt ttgtggtaga 5340
atttggtttt gtatagtttg gatttggata agttaagttt ttttttattt ttagaatttg 5400
tgtggttgtt gtttgagtga agtttagtga agatttattt ttgtattatt atatgtggga 5460
gttatttatg gatatgatgt tgttttagaa agttatttag ttattggagg tgtatgtgtt 5520
tattttagtt ttttatgata tttaatgtgt atttaagtgt gtgttaatat tgtattataa 5580
gggttggagg tgtttttgga tattttagtg ttgtggaggt agtatagtat aggtgtttat 5640
ttttgtgttg gttttatttt gaatttagag tttagatgtt tgaaaagtgt tatgtttggt 5700
gataatttgg tgttttttta attaataaaa ttttttttaa ggtagagtta gatattgtgt 5760
tggttttttg gttttatttt tgtggggatt agtagttttg gaattttttg gattttaagg 5820
ggttttagat gtttttttgg tttttgtgaa tagttaggtt tttagttttg tttggttttg 5880
gaggatgttg ggggttagtg tttgtgtgta tttttatttt ttggtttttt ttttagttgt 5940
tgtttttgtt atttttatgg tttttgggaa gtagttgggt aagagttgtg gttaaaaggg 6000
gtaggagagg ggggtgtttt agagtggaga gtttttggtg ttattgtttt gtgtgtaggg 6060
ggtgtttttt ggtttattta ttttgattgt gaatagtggg ttggtttttg aggtagttgg 6120
gtaggtaggg tgtgttagga aatattttgg tttggttgga tgatgtttgg ttgggtggag 6180
gaggattgaa gggtttgtag tgataggttt tggttgtgtt agtgaattgg ttggaaaagt 6240
ggatagtgaa ggtgtttagg tattgttttt tgtgtggttt tgtgttgttt tagtttggtt 6300
tttgtttgat gaaggagtga ggtggtggtg gtgggggtgg tggtggagtt ggtggtggtg 6360
tggggttgtt taatgatttg taagttgaag tgtttggggg tgtaaagttt agtattggtg 6420
tttattgtgt tgtgttgttt ataggtaggg tatagttgtt gttgttattt agggagggga 6480
tggtgggtag tagtgtgttt aggttttgta tgttggagtt tatttgttgt ggtttagatt 6540
tggatgtttt gtggtttttt tggttttgga gatgtttagt gttggttttg gtggtgttta 6600
atttggttta gatgttgttt gggttttgga ttttttgttg ttttggttgt tgtaggtatt 6660
taggtttgga gtggagtgtg tgttgagatg ttggttttgg gatatatgtg gaagttgggt 6720
tttgtagtaa gaggaagttt aggattgtgg tgaggagatg gtggggtttg ggtgtttggg 6780
ttgttgtttt ggttttgggt gggtgggtgg gtgaatgagt aggtgggagg gagggtggga 6840
agtgggggag tggataggtg gttgggttgt ggagagtttt tgggtgtggg tgttgttttg 6900
aattttttat tttagttgtt tggttgtttt tagtttttta aagtttaaat aagaggggtt 6960
ggtggtgtgg ggaagaggag gagttaggag gtttggttgt tttatttatt tagtagttta 7020
agtttggtta ggtagtgtgt gttgtttttt gggggtgttt ttgtagttgg ggtgagggtg 7080
ggtttgaggg agtgtttttt ttggagatat tttttttttt aatttaatat gtgtttttag 7140
ttggttggtg tggtataatt ttttttttat tttttttttt tggtttttgt tagtgtggga 7200
gttttgtttt attggagagt ttgtttaggt aagtagtgag tttgggtagg gttgaatgtg 7260
gtgggagtag agatggggtt gttaatttaa aagagtggtt tggagggagg ggtaggtagg 7320
ttttaagagg gtgggaattt taggaaagtg tagaaagaat ttagttaagt tttaaagaag 7380
tgtatatttt tgttttgtat ttttgagttt ggtttttgtt tttagaattg ttgggtaggg 7440
gagtggttat gattttgggg ttattgggga aagaggtggt tttttgtggg ttttagaggt 7500
tgggattgaa agtgaatgtt ttttagttgg gtggttggga gttgtattag ttataagttg 7560
ggtgtatttt gattggttag tagttgttta tttggtgagg tttgtgttag gtggaaggtg 7620
gttgttgttt atttaaaatt ggagttagta gtatttagtt ttttttttta gtgaggaata 7680
tttaattttt ttatggttaa gttgggaaaa tagtggttag tttaggaggg aagaaaattt 7740
ttgggtaaat gtttaaagtt tgtgtatatt ttggttggag tattttggtg tttagtttgg 7800
ggaggagggg tgaaggggag ggtttggata tgaagtttgg tagggagtgg gtaatttttg 7860
gagttatttt gttgagtatt gtttgtggtt ttggttgagg atattatttt ggttgtttag 7920
tgattgatta ggaggtggaa gttagttaag gggattaagg agtggagtag ggagaatttt 7980
tattggttta tgggtgtgag ttagtgtttg aatttttggg agtttgaaga gttttgttaa 8040
atttgatttt ttttgtttag tttttgtttg gtagtttttg tgttttagag taggtgtttg 8100
gggtgggtag ggggtgtttt ggtttggttt ttggattttt taaaggagtt agtatttatt 8160
gggaaataat ttttaggtaa ggagaagaat tgggggttgg agtggagtgt gttttagagg 8220
tgtgtataaa attttagggg tgttaggagg tagtagtgat ggtgtagtgt gggatggtta 8280
ggtgtgatgg gagggaagta attggttgta gtttggtttt ttgtatttgg ttggtttggg 8340

ttggggttgt aagtttttta gttaaattta tgtgaagtaa agtagttgta gtttgataaa 8400
gaatggtgtt ttaataaatg atatagtaat tttttatgtt ttggttttta gtttgtattg 8460
tataaatgtt gttgttatta aattttggtt gtggatttga agtgtgtgtg ggtttaggat 8520
ttatttgaag ggtgtatttt ttgggaatgt gtattttgta ggtgtaggag attttgaggg 8580
taaagatgtg gtatttgagt tttttgtttg ttttttaggt tatttttgtt gtttgatttt 8640
gtgttgtttt tatttagaag tttttaaaat tattattaag aaaagaaaga ttttataaga 8700
tattgttttt atgttttttg tttagttttt ttttatatga tttattttta aattttttgt 8760
ggggtttgtg tgtttggttt tttgagtgga gaaaggattt ttgagataaa atttattgtt 8820
agttgtttta agaatttttt agatttttgt attttttttt tttttttttg tttattattt 8880
ttatttggaa gttgtttttt ttaattagga tttaattgtg aggggtgagg agttttttat 8940
gaaaagttgg ggatttttat ttttaaatga aaagattgag attgtttttt tttttattat 9000
ttttggtatt tttagtgggg ggagaggagg ttatttgaag ggaggttggt ttgaatgtga 9060
gttgttgtgt ttgtgaaatt taggtaaggt aaagatatgt aaatttttgt taatattttt 9120
taaaaataag tttttgtttt ggggtgaggg atgtaggggt ggataggagg aaaggagagt 9180
taagtaaatg tagaattttt tttttttttg ttatttattt ttttagaatt tttgtggggg 9240
tggggagggg aagagtattg gtgtgaattt tggggtgttt ttttgtttga tttggatttt 9300
tttgattaaa gaggtgggag gagttttttg tagtttgtgg ggttaagaag aaagtgttaa 9360
gttgaggagt tgtagttgga ggattttagg gatttgatag gaagtttttg tatgatttta 9420
atggatttat atgtttggaa gtttaggtga tattaagtta gtgttgggga attgatgtat 9480
ttttgtagtt gtagggtgtt ttatgttttt ttgttttttt ttttgttgtg ggtaatgttt 9540
tttgtgggag atttgaggtt ttttaagggg gatatgttag ttatattggt tttggtgttt 9600
tgagttaagt attaggattt atagtttagt atgaaggtag gtagtatttt tatttgttgt 9660
ggatgatagg tgtttttttg tatttttttt agtttgtgag gtttgttttt tgtgaggttt 9720
tagagaaaag taggttgtgg aaaattgggt gttttttttt tttatttatt tttttatttt 9780
tgttagtgtt gagatttgta gtagaggttt ttggtttgaa ttattgattg gtaaagaaag 9840
ttgtagatta aattttttgt tttatagaga aggaaattga ggtttagata gttgaaggag 9900
aggtagttta tggagtgtag tggtaaagag taaggggttg gtggttttag aatggaattt 9960
tagttttgtt aggtaattaa tagttgtgtg attttagatg agttttgtag tttttttgag 10020
ttttagtttt tttattggtt aaatgggata atgggatatt agtgtttatt ttatagagtt 10080
gttttgagga ttagatagga gagtagtgtg ggtttttgat ttaattaatt ttatagtttt 10140
tgttattatt attgtttaag gataggaaat aagttagtgg taatgttgag ttgaaatttg 10200
ggttttttaa tgtttagttg gaggttttat ttattatttt tttatttttt tagagttaat 10260
gggatgtaga ggtgaggata gtttttggaa aattatgtgg tgttgatata ttggtaaagt 10320
gtaggtattt tggttttgaa ggtgtataat tattgtaata tgaaaaaatt gaggttgggt 10380
aggagtaggg tagtgaggat tttttaattt ttatttttat tttggtgttg tttattgtat 10440
ttgatagagt gtggagttgg gttgttatag ttgtaatttt agggattagt gtgtatgttt 10500
gggttgtttt ttgggagtta tttagtggtt gtggataatt gaataaatat tttaaagtgt 10560
tgtggttggt taagggtggg gatagttata ttgtgtgtgg ggtttgtagg ttgtaataaa 10620
gagtggtttg attttgttgt tgtttgttgt gggagaggaa taagtttttg attagtgttt 10680
ttttttgggg tttgtgtttt gagttttatg tgtaagatag tttaatagtt gttttttttt 10740
tttttgttga ttttaatttt ttggaatttg tttatttggg gttgtagggt aagtgtttag 10800
gatggttttt agttttgtgt tggtggtgaa aattttaatg ttattttttt aattaaaaaa 10860
aggggggggt aagggagggg ttttgtgata attattttta gttttttttg attattttaa 10920
aattaagttg atttttttta attagttttt atttattgtt ttaatttttt ttgttgattt 10980
tatttgggag tttggaattg ttaggtattg ttgaatgtgg tgtaaaattt tttttttttt 11040
tttagtttgt agtgtttgga gttgttgggg ttatttgttt gttttgatgt tattgtgaga 11100
tggtttttga gttttttgag agtttttagt gaaaggattt tgtggtattg tttttattat 11160
tatattgtaa atttttttaa attttggaat taattatata gaggatatgt tttaatttgt 11220
tttgtattaa tgttatagtg gggatggagg ttaggttgtg gttagagtag atatgtaggt 11280
ttatgaaatt gatgaattga gagttgtttt tagttttgag tgtattattt ggaattttag 11340
tttgaggtta taattagaat ttttgatttt agatttaatt ttgtaaataa taggggaaaa 11400
aaatggggaa aagaaaaatt atgagagagg gaaaaaaaat tattttgtat ttgtaaaaaa 11460
aaaaaaaaaa aaaaaaaaaa aaaaaaagtt atataatttt aaaatttttt tgttttagat 11520
aatttttttt aagtattttt tttgtgtttt ttaatttata aaagataatg gaatgtttta 11580
tagattggat tttattttat tttttttttt ttttttaatt gtttttagtt taatagttgt 11640
tagttttatt ttagtgttga atttaattat ataggaataa aatatttttt agtaaatttg 11700
tttgttaaat attttgttat atttattttt tttttgagaa atggtttaat tatagaaatt 11760
tatttgtatt taggaatttt tttttttttt ttttttttga ttgttatttt agtttaattg 11820
taagtagaga ttttatttgt gtagtaattt tatttgtgtt ttaaatttgt ttagttaatt 11880
tttttttttt ttttttagta atattttgtt tta 11913

<210> 279
<211> 4316

<212> DNA
<213> Artificial Sequence

<220>
<223> chemically treated genomic DNA (Homo sapiens)

<400> 279
   ttgtagttaa taaaaaaaaa aaaagaaaga aagaaattgg tttttgtttt attttatatg 60
   tttaggtata atttttttag agaagaagag gaggggggtg gggaggagta ggaggaggag 120
   gaaagaagga ggagaaggag aaggagaaga agaggaagag gaagaggaag aagaagaaga 180
   agaagaagag gaagaggaag aggaagaaga agaagaagaa gaagaagaag aagaagaaga 240
   agaagaagaa gaagaagaag aagaagaaga ggaagaagag gaagaagaag aaattgtttt 300
   tagattttta tttttaggat aagtttattg tttggtatta agttttttgg ggtgaatttt 360
   tttttaaaag agtttgggga gtttaggtat ggaatgggag gtagaaagtt taattaaggg 420
   attgggattt tggaatgaat aatgaaggga gatggattgg gtttatgttg aaggtttttt 480
   tttggttttt tagtttttgg gtgaagattt agggagatat tgagatatat tttgtatagg 540
   agggggaggg ggagggggag ggtaaagttt tagggtttta ggagtggttt ttaagggttt 600
   aggttttgag gtggtgtttg gggttggaag gtttagtatt gagaattttt tattttttta 660
   gagttttttt ttttttttta atttgtgtta ggttttttat tttggatatt tataatgtgg 720
   ttttattttt tatttttatt gggtgttgtg tttttagaga agttaattag tgttgttgta 780
   gtttttaggt tttaaagttt tatgtatttt gtgggattta tatttttttt agatgtggag 840
   gttggggtta tggtgttttg aagttttttt ttgttgtttt taggggtttt ggttttgatt 900
   gatatttggg tgggtgagtg tggggtttag agagaaatgg tttttgtggg gaggagtgag 960
   gggtttgttt ggtgggggtg taggatttag ggagttgtgt ttggaggagg gtttggtggg 1020
   ttttattttt tttttgtttt taggttttta ttttttgagg tattttagta ttgttgtgtt 1080
   gtggtttggt tgtggggagt tttgttatat tgttgtggag tatgtagatg atatgtaatt 1140
   tttgtggttt gatagtgatg ttgtgatttt gaggatggag ttgtgggagt tgtgggtgga 1200
   gtaagagggg ttgtagtatt gggagtggat tatagggtat gttaaggtta atgtatagat 1260
   tgattgagtg gttttgagga atttgttttg ttgttataat tagagtgagg ttggtgagtg 1320
   aatttggttg gggtgtaggg ttatgattat tttttatttg ttatggattg tttgggtttt 1380
   tttgagtttt tggatttgaa atttattttg aggtagtgga tttgtttaga ttttttattt 1440
   gggagagttt taggtgtttt tattgaggtt tatttttagt ttaggttaaa atttttgtgg 1500
   gttgggtggg gagggggtgg ggttagttgg gtggggttga ttgtggggat tggttagggt 1560
   tttatatttt ttagggaatg aatggttgtg atatggggtt tgatggatgt tttttttgtg 1620
   ggtattatta gtatgtgtat gatggtaagg attatatttt tttgaatgag gatttgtgtt 1680
   tttggattgt ggtggatatt gtggtttaga ttatttagtg tttttatgag gtagaggaat 1740
   atgtagagga gtttaggatt tatttggagg gtgagtgttt ggagttgttt tgtagatatt 1800
   tggagaatgg gaaggagatg ttatagtgtg taggtattag gggttatggg tgtttttttt 1860
   attttttgta gattttttgg gatggttttg tataaggttg ggaggaaagt gggtttaatg 1920
   ttaggatatt gttttttttt tagttttgag taggaagaat ttttttggtt tttgagattt 1980
   ggtattagag agtgattgtg agagtttgtt ttgttttttg ggataattaa gggatgaaat 2040
   ttttgaggga atggagggaa gatagttttt ggaatattga tttgtggttt tttttgagtt 2100
   ttttaatagt tttgggtttt gtgatttttt ttttaagttt tgttttttgt tttatattta 2160
   atgtgtttgg ggttttgatt ttagtttttt ggtttttatt taggttaggg ttagaagttt 2220
   ttgtttttta tttagagatt tgaatttttt aaggaatagg agattttttt aggtgtttgt 2280
   gtttaggttg gtgtttgggt tttgtgtttt tttttttatt ttaggtgttt tgtttatttt 2340
   taggttggtt atatgggtgt tgttggggtt ttttatgagg agtgtaaagt gtttgaattt 2400
   tttgattttt tttagatttt ttaaaggtat atgttgttta ttattttatt tttgattatg 2460
   aggttatttt gaggtgttgg gttttgggtt tttattttgt ggagattatg ttgatttggt 2520
   agtgggatgg ggaggaatag atttaggata tagagtttgt ggagattagg tttgtagggg 2580
   atggaatttt ttagaagtgg gttgttgtgg tggtgttttt tggagaggaa tagagatata 2640
   tatgttatgt gtagtatgag gggttgtttt agttttttat tttgagatgg ggtaaggagg 2700
   gagatgggta aagaggggaa tgaggggtta tgtttttttt tagggaaagt aggagttttt 2760
   ttggagtttt ttagtagggt tagggttgag gtttggagat tagggttttt tatttttttt 2820
   ttttttttta gagtagtttt tttagtttat tatttttatt gtgggtattg ttgttggttt 2880
   tgttgttttt ggagttgtgg ttattggagt tgtggttgtt gttgtgatgt ggaggaagaa 2940
   gagtttaggt aggaaggggt gaggagtgga gtttgagttt ttttgtttta ttgggggttg 3000
   taagttttaa gtagaagtgt gttttgtttt attattggga agtattattt atatttatgg 3060
   gtttatttag tttgggtttt gtgtgttagt atttatttat ttgtaaagtt tttgtgaaaa 3120
   tgaaggatag attttttatt ttgatgatta tggtggtgat gggatttgat tttagtagtt 3180
   ataaattata ggggaaggtt tttgttgatg atagatttta ggagggtagt tggtttagga 3240
   tttatatttg ttttttttat attttttgat tttgttttgg atttatagtt atattttttt 3300
   ggaaattttt ttgggattaa agattagggg tttgttttag gattttatgg ttttgttttt 3360
   tttttggttt tttataggat attttttttt tatagataga aatagaggga gttattttta 3420
   ggttgtaggt aagatgaagg aggttgattt ttgagattgt tgggatattg tggttaggag 3480
   tttatgaggg agtttattta ttttatagtt tttttagtta tatttgtggg ttttgattag 3540
   gttttgtttt tgttttattt taattattga tagtgtttag ggttttgggg tgttttttat 3600
   agttaataaa ggtgatattt tagggtaggg gttttgatgt gagtggggtg ttggggggga 3660
   atagagggga tttagttgtg ttattgggtt tttttgattt ggatgttttg agtatgaaat 3720
   gggttattta gagtgttatt ttttattgtg attgatatga atttgtttat gaatattttt 3780
   tttatagtgt gagatagttt ttttgtgtgg gattgagaag taagatatta atgtagtaga 3840
   attgtatttg tgttttatga atatatataa attttaaaaa taaagaataa aaatatattt 3900
   ttttatagat ataggtagat atgtttttat agtatgtatg taaatgtgtg tgtgtgtgtg 3960
   tgtgtgtgaa gagaaagagt gaatagagag attaagattt ttttaatggt gaaaagatat 4020
   atatatattt ggaattagtt agtttgattt agtttaggtg attttaattt tggtggtaat 4080
   aattaaagta ttgtagttag gagttagttg aatatatgtt tttttttttt tattagattg 4140
   aattagagtg ttgattttgg ttatattaat gttataaatt tttttatagt ttgtttgatt 4200
   tggtgtttgt tggttttaat atttatagtg aatataagta ggttgttgtt ttttattttt 4260
   tttatagttt atttagtggt tagtggaaat ttgatgataa tatggtggtt aagttt 4316

<210> 280
<211> 4316
<212> DNA
<213> Artificial Sequence

<220>
<223> chemically treated genomic DNA (Homo sapiens)

<400> 280

aagtttgatt attatgttat tattaagttt ttgttgatta ttgagtaggt tgtgaagaag 60
atagaaaata atagtttatt tgtgtttatt gtggatgtta aagttaataa gtattagatt 120
aaataggttg tgaagaagtt tgtgatattg atgtggttaa agttaatatt ttgatttagt 180
ttgatggaga gaggaaggta tatgtttgat tggtttttga ttatgatgtt ttggttgttg 240
ttattaaaat tgggattatt taaattgagt taagttggtt aattttaaat atatgtatat 300
ttttttatta ttaaaagaat tttaattttt ttatttattt ttttttttta tatatatata 360
tatatatata tatttatgtg tatgttataa aaatatattt atttgtattt ataaaaagat 420
atatttttat tttttatttt taaaatttat gtatgtttgt gaggtataag tgtaattttg 480
ttatattgat attttgtttt ttagttttat ataaggaagt tgttttatat tatagagaaa 540
atatttatga ataaatttgt attagttata gtgagaggta atattttaaa tagtttattt 600
tatgtttaag atatttaagt taaagaaatt taatagtata gttgagtttt ttttgttttt 660
ttttaatatt ttatttatat tagggttttt gttttggagt gttattttta ttagttgtga 720
gagatatttt agagttttgg gtattgttag tgattggggt agaataaaaa taggatttgg 780
ttagagttta tagatgtggt tagaggaatt gtggggtggg tgagtttttt tataggtttt 840
tgattataat attttaataa ttttagggat tagttttttt tattttattt gtagtttgag 900
agtagttttt tttgttttta tttatgggaa gaaaatgttt tgtgagaggt tagaaaggag 960
gtagggttat aaggttttag agtaaatttt tagtttttga ttttagagaa gtttttagaa 1020
aagtgtaatt gtagatttag ggtaggatta agaaatatga agaaagtaga tgtgggtttt 1080
ggattaattg tttttttgag gtttgttatt agtaggattt tttttttgtg atttgtgatt 1140
gttgggatta ggtttattta ttattataat tattgaagtg aagaatttgt tttttatttt 1200
tataggagtt ttataaatga gtaggtgttg gtatataggg tttaggttgg gtagatttat 1260
gagtgtggat ggtgtttttt agtaatgagg tagggtatat ttttatttgg ggtttgtaat 1320
ttttagtggg ataagaaaat ttagatttta tttttttattt tttttttattt gagtttttt 1380
ttttttatat tatagtagtg attatagttt tagtgattat agttttaagg ataataaggt 1440
tagtaatgat gtttatgatg gggatggtgg gttggggaga ttgttttggg aaaggaaggg 1500
aaggtgaggg gttttgattt ttaggtttta gttttgattt tgttgaagag ttttagaagg 1560
gtttttgttt tttttgagaa aagatatgat tttttgtttt tttttttatt tatttttttt 1620
tttattttat tttaggatga ggggttgggg tagttttttg tgttgtatat ggtatgtgta 1680
tttttgtttt tttttagaag gtattattat agtggtttat ttttggaagg ttttattttt 1740
tgtaggtttg gtttttataa gttttgtgtt ttgggtttgt ttttttttat tttgttgtta 1800
ggttagtgtg atttttgtag ggtagaagtt tagggtttag tattttaggg tggttttatg 1860
gttagagatg gggtggtggg taatgtgtgt ttttggagga tttgagaaga gttagaaaat 1920
ttaggtattt tgtatttttt atgggaaatt ttagtagtat ttatgtgatt aatttgagaa 1980
tggataggat atttggggtg gggaagggag tatagaattt agatattagt ttggatatag 2040
atatttggga aaatttttta ttttttggaa agtttgagtt tttgagtggg gagtagggat 2100

ttttggtttt gatttaagtg gaggttgagg gattggaatt agagttttaa atatattgag 2160
tgtgaggtag agaataaaat ttgagagaaa agttatgggg tttaaggttg ttggagggtt 2220
taaaggggat tgtggattgg tattttaggg attgtttttt ttttattttt ttagagattt 2280
tattttttaa ttgttttaga gagtagggtg gatttttata gttatttttt ggtattggat 2340
tttgaaagtt aggaagattt tttttattta ggattagagg gagggtgata ttttagtatt 2400
gggtttattt tttttttaat tttgtgtgag gttattttaa gagatttata ggagataggg 2460
aaggtgttta tggtttttgg tatttgtgtg ttgtagtgtt ttttttttat tttttaagta 2520
tttgtggagt aattttaggt atttgttttt taggtaggtt ttgaattttt ttgtatattt 2580
ttttgtttta tagaagtgtt gggtgatttg agttatggtg tttgttgtgg tttaggagtg 2640
taggtttttg tttagggaga tgtaattttt gttgttgtat gtgtgttggt gatatttgtg 2700
gaggaggtgt ttgttgggtt ttatgttgta gttatttatt ttttggaggg tgtgagattt 2760
tagttggttt ttgtagttag ttttgtttag ttagttttgt tttttttttg tttaatttgt 2820
ggggattttg gtttaaattg aaaatgaatt ttggtaaagg tgtttgggat ttttttgggt 2880
ggagggtttg ggtgggtttg ttgttttggg gtagattttg gatttggaga tttgggggga 2940
tttgggtggt ttgtggtgga tggggggtgg ttgtgatttg tgtttttggt tgggtttatt 3000
tattagtttt gttttggttg tagtggtgga gtaggttttt tagggttatt tggttagttt 3060
gtgtgttggt tttggtgtat tttgtggttt atttttaata ttgtggtttt ttttgtttta 3120
tttatggttt ttgtggtttt atttttggaa ttgtggtgtt gttgttgaat tgtaggaatt 3180
gtgtgttgtt tatgtatttt atggtgatgt agtggggttt tttgtggttg ggttgtgata 3240
tagtggtgtt gaaatatttt aaggagtggg agtttggggg tgaggagggg gtgagatttg 3300
ttagattttt ttttgggtgt ggttttttga gttttgtgtt tttattgggt gggtttttta 3360
ttttttttta tagaggttgt ttttttttgg attttgtatt tatttgttta agtattggtt 3420
agggttaggg tttttgagag tagtaggagg aggttttggg gtgttatgat tttaattttt 3480
gtgtttggga aaaatatgag ttttgtgggg tgtgtgggat tttagaattt gggaattgtg 3540
gtgatattga ttggtttttt tagaaatgtg atgtttaatg ggagtgagaa atggggttgt 3600
gttatgagta tttaggatga aggatttgat atgggttggg agagaaagag aaattttggg 3660
gagatgggga atttttaata ttgagttttt taattttaga tattgttttg gggtttgagt 3720
ttttgagagt tatttttggg gttttgggat tttgtttttt tttttttttt tttttttttg 3780
tgtagggtgt gttttaatgt ttttttgagt ttttgtttgg gggttgagaa attagggaga 3840
aatttttggt atggatttag tttatttttt tttattattt attttgaaat tttagttttt 3900
tgattgaatt ttttgttttt tattttatat ttggattttt tggatttttt tggaagaaaa 3960
tttattttaa ggagtttggt gttagataat gaatttgttt tgagaatgaa ggtttagaga 4020
tagttttttt tttttttttt tttttttttt tttttttttt tttttttttt tttttttttt 4080
tttttttttt tttttttttt tttttttttt tttttttttt tttttttttt tttttttttt 4140
tttttttttt tttttttttt tttttttttt tttttttttt tttttttttt tttttttttt 4200
tttttttgtt tttttttgtt tttttttttt ttttttttgg aaaagttgta tttgagtata 4260
tgaaatagga tagagattag tttttttttt tttttttttt tttttattag ttgtag 4316

<210> 281
<211> 14147
<212> DNA
<213> Artificial Sequence

<220>
<223> chemically treated genomic DNA (Homo sapiens)

<400> 281

gggggtattg agtaatatat ttagattaat aatattttat aagtgtgagt attattgatt 60
taaatttttt tgttggttgt gttttgtgtt tatgtttgtt ttatatttat tttttttaga 120
gttgtttttt ttagagtttt agtaattttt atttttttaa atttttttag ttatgggaag 180
gattagtagg tgttagtgga tttaaaagaa ttttagattt gttgagattt ttgatttttg 240
gtgtttagat taattagtaa atagtttaat taaatggttt gtttttatgg ttaatataat 300
tttgaattgg tggttattta gattaagttt attttgtttt tgagatggaa ttttgttttt 360
gttgtttagg ttggagtgta gtggtgtgat tttggtttat tgtaattttt gttttttggg 420
tttaagggat ttttttgttt tagtttttta agtagttggg attataggta tttattatta 480
tttttgatta atttttgtat ttttagtaga gatagggttt tgttatgttg gttaggttgg 540
ttttaaattt tgattttaga tgatttgttt attttagttt ttttaagtgt tgggattgta 600
ggtgtgagtt attgtgttta gttaaagttt agtttgtata agaaggtttt ttttgttatg 660
ttataggatt tttgagttta ttaaatagat tttaatgttt tgagatttta tgtatttttt 720
taaaatgaag tatttaaatt gttagtgttt gtttttttgt ttggtttttt tgatgtattg 780
atttggtttg ttagtgtgat gaaaaatatg gttgagtttt attgttttat tgtaattagt 840
aggattgttt agaagtttaa tttttttaag agatgaagat ttgagtttat gtagttggtt 900

gttttttaaa aatatttggt tatttgggat atattttgaa ttaatttttt ggttatttta 960
tgtgattaaa agtgttagtt ttttttgttt ttagtatatt tttttgtttg ggaagtggaa 1020
gggatgaaat ttttatattt ttttttgatg atatagaaag gagtatatat ttatatttta 1080
tgaatagata tgtaattata gaagaaatag tttaatggtt gggtgtggtg gtttatattt 1140
gtaattttag tattttggga ggttgaggtg ggtggattat gaggttagga gtttaagatt 1200
agtttgttta agatagtgaa attttgtttt tattaaaaat ataaaaaatt agttgggaat 1260
ggtggtaggt gtttgtaatt ttagttattt aggaggttga ggtaggagaa ttgtttgaat 1320
ttggagggtg gaggttgtag tgagttgaga ttatgttatt gtattttagt ttgggtgata 1380
tagtgagatt ttattttaaa aaaaaaaaaa aaagtataat tatatgatta tttatattgt 1440
tttatatatt aaattagtag taatttgttt tttatatagt aaataaaaat aaattagtag 1500
taataaatat attttttttg tgttatagtt agtatttggt gaattttgta tttttttttt 1560
ttttaaagtg ttgaatttat aggtttttat aaggtaagtt tatattatta attaattttg 1620
tttttttttt tgatgttaaa attgtttttt aggagttgat taggtttatt tttttgtttt 1680
ttaagtatta tttttagaat atttttgaaa gtatttttgt tttttggtaa taatatttat 1740
attttattta ttttttgttt ttaaatatat atttagttaa ttttaggttt tttttaatgg 1800
agtagttatt tatttattta atatatgttg ggtgtttttt ttgtgttata tattgttttg 1860
gtttttgggg atagagtagg aaataaaaaa aaataaaatt tgtttttata tagttgatat 1920
tttagttagg ggagataaat aataaataaa tgaatgatgt agtatgttag atgttgatag 1980
tgttatggag aaagtatatt atggagatta gaagtattat ttgtaatgga atagttatga 2040
ttttaagaag aatggttgat tggatgtgaa gtgtgagaga gaggagttta ggtttttggt 2100
tgagtaattg gagggatgga gttgttatta tttgagatgg agaagttagg ggaggagtag 2160
atttggaggg gaaggttagg aatttagttt ggaatttgtt aagtatgagg tatttattaa 2220
atattgaagt ggatttttag aagatgttat taaaattagt taagtttagg agagagtttt 2280
gggttagaaa taaaaatgtg ggagatattg gtatatatat gggtttatat tatttttttt 2340
tttaatttaa tattttattt tattgttttt tgttttttta atagtattta gttttattta 2400
ttttttaaat tgatttttat attgatagta taataaaatt tatatttttt attatattaa 2460
gattttgtgt gatttaattt ttttttattt tttggatttt gttttaggtt attttttttt 2520
tttatttaat ttttaatatt ttgattttat tgttttttaa atagtttttt gttgtttttt 2580
gttttttttg tggaaaaggt ttttttattg tttaattatt aggtattttt tgattttttt 2640
ttatttttta tttttgagaa aggttttttt tgatttttta tattaattag gatttttttg 2700
ggtgagattt gatttaaatt ggtttagaga aaaggagtta attgattggt ttatgtaatt 2760
taattttttt tttttgttgt aatttttttt tggtttttat tttatttaag gttaaagtta 2820
gggtttttat tatggttttg taggttttat atagtttgtt ttttttatta tttttttgat 2880
tttatttttt atattttttt tttatttatt tttttttagt tagagtggtt tttttgttgt 2940
attttaaata tgttaggtat atttttattt tagtgttttt gtgtttgttg ttttttgtgt 3000
gtggaatata ttttttatat agttatatta tttaaaattt attgttttta agtttttatt 3060
taaatgtttt tttgtgaagt tatttttgag ttttttttaa aattgtagtt ttttattttt 3120
gtatttttta ttgtttttta tttttaattt ttgttattgt agttagaatt atttgatata 3180
tagtttttat tttattggtt ttttgttatg ttttttttta taagaatttg aagtttatta 3240
ggttgggagt tttgtttgtt gtgtttgttg ttatttttta gtgtttaaaa ttgtttggta 3300
tatagtaggt atttaataat ttttgaatta gtgaaaatta gatggtggtt tggtattttt 3360
atataggaat gagttaggtg gaaattattt aggatataag tagattttga agtgataagg 3420
aagggttatt ataattatgt ggggtttatt ttgttttttt ttgttttttt tttttaggtt 3480
tagtaatagt tttattaagg attttatgaa tattaaagtt tatatttata tgttgttaga 3540
ggtgagagta gtttatttta ttattagatt ttgtgtttag ggtggtgatt tgaagaagga 3600
agagagtgaa agaagggaag gattattttt ttttttaaat tggagttaag ggagggaggt 3660
tagagtaagt ttgggggtgt aatttagatt tagtttttgt ttaatttttt ttgttttttt 3720
ttttaggggt ttagagaatt ataaggtttg tagaattttt tagagaagtt ttattattga 3780
tttttttttt ttatttttag atattaaaga gtttgaatgt ttttgagtta tatggttttt 3840
tttttttttt ataaattttg ttagttgtta tgggggtttg tttttagggt ataaagttat 3900
tgagagattt agagatttag tttttttgtg gaatttttaa aatgttttag tagttttgtt 3960
tttagtttgt tgtttggtag tatttgttag ttgattttat gtattgtttt tttttttgtt 4020
attttggtaa tttgggtttt ttattttatt ttttttttta tttttttttt tagatgttat 4080
tttttgaatt gttatagaga atgggttaat ttatggtgga ggtttgttta tttttataat 4140
aattaaaggg ttttaaggtt tatgttttta tatatttaat tattttttag gtattttaaa 4200
ttatatttaa attttaattt agttttattt tgttttagtt tttgtttgta agtgttttag 4260
tttttaatgg gtatagattt ggattttgtt ttttttagtt gggtttttat tttaggttga 4320
gtttagttat tgttaattaa tgtagagtta ggtttttttt ttttttaatt ttggttgtag 4380
tttaattttg ttttttgaaa gtattttgtt tttgttattt aatgtttttt tgtgtgtttg 4440
atagttattt tgttttttta tttttgttgt gttttaaatt tgtttttatt tagttttatg 4500
gttttgttta attgtagagt tgttttgttt gtttatgtgg agtttagttt gatttatttt 4560
tgttggttgt tatgtttatt tttatataag ttttgttttt gttgagtagt atggtgtgtg 4620
atattgtttt tttggtgttt tggtaggggt ttagaagttt tttgtttgtt aattagagaa 4680

aaatagggtt atttattttt gtttttgggg gttgtttttt attaatttta tgtaagttaa 4740
ttagtgtgag gtagattttt gtttttttga tataggtttt gagttttttt agttgtttat 4800
agtttgttag tttttgagtt aattggtgtg gagtattgtg aaggttgaat gtgttttttt 4860
gggattttag gggttgtgag tgttttatta ttttttttta tgttaattat ggtatagttt 4920
tgtagggaag ggtttgtttt ttaatttttt gaggttttgt ggttgattaa tagtgttttg 4980
gttaattagt gagtggtggg atattgggtt tttttttttt gggtttatgt gagttgtagg 5040
gaaatgtagg ggtggttttt aggtgttgtt gttgttattg ttattattat ttttattgtt 5100
gttttggaat ttaggtttgg ggggtggtgg ggttgtgtat ggagtttttg ttttttggag 5160
ttgttaatat tgttaatgtt attgttatgt tatatatagg tgagttttgg gtttggaggg 5220
tggagggttt agtttgtgat tttatgtatt ttttttgttt ttgtgtagag gatgtggttt 5280
ggttggtggt ttgttggtgt ttgatttttt gttgttatta ttatggttgg tggatttgtg 5340
tgtggtattg tttaagtttt ttgtttttta tagtggattt gattgtggtt taattttttt 5400
tttttgttgt ataatggatt ggtttgtgtg tttatgtttt tttttatgtt aatttagggt 5460
ggatttgttt ataggttttt tttttatttt tatttttatt tgttttttat ttatttttat 5520
ttatagggag ttagtttgtg atagtgttta gtttttttaa tagtaggtgt atttgagtgt 5580
ttggattttt tttagtttta atttttagga gatttgtttg tggttttatt tattttattt 5640
tttttgtata ttgtagtgaa ttaatttgtg gtgttgtatt tttttgtatt tttttttaat 5700
agtgagttta tttgagtttg tatttttgtt ttttttagtt ttttaatgta ttagtttatg 5760
gtttttttta aaattatttt ttttttttat tattattatt attattttat agtggattag 5820
tttttatgtt tggatttatg tttttttagt tattagttta ttgttttgtt tttttttttt 5880
tgtagattgt tgttttagtg tttggggttt tttttttttt aatagtgggt tagtttagtg 5940
ttttatattt ttttattttt agtagtttag tttatagttt tttttttttt tgttttgaat 6000
tagtttgtgt gatggttttt tttttttttt attgtggtgt aggagtttgt aaggtttttt 6060
gttttttttt tttttagatg atgagtttta ttattttttt ttttgtagtg gatttgttta 6120
ttggtttttt ttttttatat ttttgtatgt ttgtatagtt ttttgtatat ttttttgtga 6180
gtttagtgtt ttgtaattgg gggaagattg gttgtgtatt tttaattttt tttttttttt 6240
tttttttttt ttagaagaga atgtgttgat tttttttttt ttgtgatgga gtattgtatg 6300
gttttttttg ttttttattt tttaatagtg ggttagtttg gttatatttg taattataaa 6360
gatgttttgt tttattataa taagtgaata tattagtgtt agtgatgata gtaatgttaa 6420
taggagttag tgtttattga gtatttgttg tatattaggt agagtgtttt atatgaatga 6480
gtttttttaa tttttattat aattttatga gataggtagt gagggaattg ggttataggg 6540
aggttaaatt ttttatttaa aggtggttta ggaagtattt aggatatagg ataatttttg 6600
aggttttttt tttagatttt attttatttt atgtgaggtt ttagttttta aaaattaaat 6660
tagtttttat ttttaagttt ttgtttttgt tgtttttttt gtttgtagag tagtttttat 6720
tttttatttt tttttagtat ttatttattt tttaatttta gtagttgtgt gattttagat 6780
gagtgattta attatttttt ttttgtgttt ttttttttat ttgtgaaatg ggggattaga 6840
attagaattt tttttagtgt aaataaatat taattgtgaa ttaatggtaa aataagtaat 6900
ttttttagta ttattgttta tattttttta tttttttatt gtgttttaat tatattggtt 6960
ttattatata gttttttaaa tatagtaggt atgggttagt tttagggttt ttgtattggt 7020
tgttttttgt gttttgaata ttatttttag atttttgtat ggtttttttt agtattttgt 7080
ttaaatatta atttttaaag gatgtttatt atattgtggt gtgtggaatg tagtataaaa 7140
agatatatgt atgaaagatg tttttaagaa tgttagttta tatatgagta aggttttttt 7200
ttttttttaa aaggatgtgt ttataagatt gaggtatgta atatgttttg taaaggtata 7260
ggtataaaag ttgtttttaa gagtgttagt tgtatatgtg aataaggatt gtgttgttgt 7320
attgaaagaa tgtgtttata aaattgtggt gtgtagttgg gtataggggt gtgtatttat 7380
agttttagtt atttgggagg ttgaggtggg aggatagttt gagtttagga gttttaggtt 7440
agtttgggta atatagtgag attttttttt ttgaaaaaaa aaaatttaaa aaataattat 7500
tgtggtgtgt gtaatatatt tttttaaagg tataggtgta aaagatgttt ttaagagtga 7560
tttgattttg ttgtatttgg gtagtgttat tttattaaag gttgattgtt atattatggt 7620
atttagaata tattatataa aggtatatgt ataaaaaaga agtttttaat aatgttattt 7680
gtaaaaataa aaaaagttaa tttttttttg attatttgaa gttgtatatt tagtagggtt 7740
taagttaggt ttttgatttg ttttgttgtt ttttattatt gtttgatata ttgtgtgggt 7800
tatttgtttg ttgtttgttt ttttttatta gaatgtgagt tttatgagga tttagttttt 7860
gtttgtttat ttattgttat gtttttagta attgaaaatg tattaggtaa atagtagatg 7920
tttaataaga atttatttta tgaataaatg ggaaaaagat aatttgagat attagtgtag 7980
gggttatatg aggaagaaga agggatatga gagttaatta gttgggattg tttttgtaat 8040
taatttttat tgagtttttt gtgagtatta ggtatttttt taggtgttgg aatatggtaa 8100
ggaatgaaat agggtgaaat atttgtgttt gtggagttga tatttattaa aagttgggga 8160
ggttgggtat ggtggttaat attagtaatt ttagtatttt gggaggttga ggtgggtgga 8220
ttgtttgagt ttaggagttt aagattagtt tgggtattat ggtgaaattt tatttttatt 8280
aaaatataaa aaaattagtt gggtgtggtg gtgtgtgttt gtaattttag ttatttggga 8340
ggttgaggta ggagaattgt ttgaatttag gaggtggagg ttgtagtgag ttaagattgt 8400
attatgtatt ttagtatggg tgatagagtg agattttgtt ttaaaaaaaa aaaaaaaaaa 8460

atttaagttg ggagatagaa ttatgtgaat aaaataaata agtaaaatta gtataataga 8520
taaatgttga gaagaaataa tgaaataggg taggtaggaa ttgagtattg ggggtggttg 8580
aaattttagg tatagggaag gtgatttttg aattagggtt ttatggtaga gatggagaga 8640
gttttaatgt ttatgggaag agtatattag gtagaaggaa gtgttagttt agaggttttg 8700
aggtgggatg gtgtttgtta tgtttaagga atattaaggt ggttagtgtg gttggagtag 8760
agtgtgggga agggtagaag gtgaggttat ataggtgatg ggagttagat agtgtaggtg 8820
atatatagga taggattgta ggggtagggg gagaaggatg atatattagt taatagttaa 8880
tatttgtgga gtatttgtta tatattagag gtattttttt gtttaaatat taatttttaa 8940
aggatgttta ttatatggtg gtgtttggaa tgtattataa aaagatatat gtgttaaaga 9000
tatttttaag aatgttagtt tatatatgaa taaggatttt tttttttttt aaaaggatat 9060
gtttataaaa ttgaggtatg taatatgttt tatggaggta taggtataaa agttgttttt 9120
aagagtgtta gttgtatatg tgaataagga ttgtgttgtt gtattgaaag aatgtgttta 9180
taaaattgtg gtgtatagtt gggtatagag gttgaggtgg gaggattatt tgagtttagg 9240
agttttaggt tagtttgtgt aatatagtga gatttttgtt tttgaaaaaa aatttaaaaa 9300
ataattattg tggtgtgtat aatatatttt tttaaaggta taggtataaa agatgttttt 9360
aagagtgatt tgattttttg gtatttgggt agtgttattt tagtaaatat ttattgttaa 9420
atattatggt gtttggaata tattatataa aggtatatgt ataaaaaaga agtttttaat 9480
aatgttattt gtaaaaataa aaaaaaaagt taatttttta agttttttat agaagtattt 9540
tatagaattg atatagttaa tttttataga agttttggag ggtaggtgta tttttgagaa 9600
tatttgattt tgagagtaaa atatttttat tttatagatg aggaaataag taaagagagg 9660
ttgagtaatt tgtttagggt aattttgagt tagaatagtt agaatagggg tagaggaata 9720
taaggttgaa gatttagaaa gaagaataga tttgtaaggg taggtaggtt atttggtatt 9780
tttgattatg tagaggaaga atgaattttt tttggaatgt gatgattgtg taattgagtt 9840
tatgaagtga gggaagagtt ttttttgtgg ggagagtagt ttgtgtttat ttttagaggt 9900
tggaattgtt ttgggaatgg tagatagttt agggtggttg ttttagagtt tttgtatatg 9960
ttgtttgttt tgtttgaagg ttttttttta tagagggtag tttggtttgt tatttttttt 10020
tttttaggtt aggtgttatt tttttgggga agaatttttt gtatattttg tgtaaaaata 10080
gtattttgt tatttttag gatttattt tttttgttt tttttatgg agaattggtg 10140
gtgtaaggtg attgtttatt agagttaaat tatttaggtt ttgaattttg gttttgttaa 10200
tttttagttg tgtgatttgg ggtaggttat ttttttgtat ttttattgtt ttatgtggaa 10260
aatgggggaa atgataattt ttatttttta atggtggttt gaggatttaa tgagttaaat 10320
gatttaaaga gtgtgtagtt tatggtaagg attgtatgtt tggtttgtat ttttaaatta 10380
ttaaaatgta tttatagtat atttgtagat gtttgatagg tttttgaaat tgattatttt 10440
taaaattggt gtgatttttt ttaaaattta tttttttgta aggtttttat tttagttgat 10500
tataatgttg tttttgtggg tgattaggtt ataaatttag atttattttg gatttttttt 10560
ttattttttt tgggtatgtt atttgttagt aagttttatt ttgtgtttag gtttttttta 10620
gattttagtt attttttgtt agttttattg ttattattat tatttattgt agtggttttt 10680
ttagttttaa tagtttgttt tttttataat tgttaggggg attttgttga aattttagtt 10740
atagtttgtt atttttttgt ttataatttt tttgtggttt ttgttttatt taatattttg 10800
atagtatttg aaaaagtttg gtttagtttg gtttttgtta ttttgttttt tttttttgtt 10860
gttttttttt ttttttttga atttatgtta gttttttttt tttatgttat ttgttaggta 10920
ttttttagtt ttgggttttt tatattggtt gttgttttta tttggtttgt tgtttttaga 10980
tatttatgag tttagttttt ttattttttt ttggtttttg tttagttgtt ttttttttgg 11040
tgattatttt tttttgattt tttatgtaaa agtaatagtt tttttttgtt tgattttttt 11100
tttataattt ttattattat ttgatgtatt tttgtatatg tttgtttttt tttttatttg 11160
ttttttttgt taagatgtga gtttttttgg agtagggatt gttgtttgtt gtgtttttga 11220
ttgtatttgt agtatttggt ttatagtatg tatttattaa atgtttgtta agtgaaggag 11280
atggaaagaa gtaaatatat ttaagtaagg tagattatta gaatttttat gaggggttaa 11340
gtgggagaaa taataaaata taaagttttg tgtgttgttg aagttggaag tttattgtta 11400
ttttttattt agttgatagg ttttgttgta gttgaaagaa gaattggtaa aattgtttgt 11460
ttttgttatt aggagttatt ttgaattttt tggatttttg aaataagttt gtttgtaaaa 11520
taaatttagt gaggtttttg gtgataggat tttttaattg gatattttat ggttttgtga 11580
tttgtataat tgaataggaa gtttatgttt atttttagtt tgtttttagg aaagaaagta 11640
gtaatggggt gatagggagg attttgtttt ttgaaggata tgttgagagg gaatagttgg 11700
tgtgaggggt tgtttagttt aatggtgaaa tgtttagtta attggaattt gattgtttgg 11760
ttttaaatgt tggttttgtt atatgtggtt tgtggaaggt atgtagatat ttgtgttttt 11820
gttgttttat ttataagtta atgataggaa gattatttat tttttagagg tatgattagg 11880
gataaataag ttaatgttta ttaaaaaaaa aaataatagt ataggtgtat atgtttttat 11940
ttaaaatttt tggggttaga tgtgttttag aatttttatt tttttatatt ttagaaaggt 12000
aatatgatgt atatatgatg ttatataata ttttttagta agttttgtaa ttggttgtgt 12060
ttatattttt gtagtaaaat gtatgaatat ttgtaataag tgagatttaa taaagattag 12120
aaagggtttt gttttatttt aaattaggtt ttgttattaa aagagttaaa agaagttttt 12180
tgtttttaga gtattttgga tgttggaatg gtagataagg gattatgaat aaattagaaa 12240

ttaatattta gagtttatta tatattttag gtatataata tttaatttgt ataatatttt 12300
tgtgaattag gtattgttat ttttatttta ttaatgggta tattattatt tttgttttat 12360
atatgagaaa attgaggtat aaagaggtta aagaatatta gttaagtgtg gtgttttatg 12420
tttgtaattt tagtatttta ggaggttagg gagagaggat tgtttgagtt taggagttta 12480
agaatagttt gggtaatata gtgagatttt attttttaaa aaaagaaaaa gaatatgttt 12540
agggttatat agttaataag tggtagaatt aggatttaaa tattggtaat ttggttttag 12600
tgtttgggta gttgtttagt aggttatatt gtttttttgt ttagaagagt gtttgatgtg 12660
tattaagttt ttaatagatg ttaattattg ttgttattaa gaaagagaat atttagggtt 12720
gagtatggta ttttatattt gtaattttag tattttggga agttaaggtg ggaggtgggt 12780
ggattatttg aggttaggag tttaagatta gtttggttaa tatggtgaaa ttatgttttt 12840
attaaaaata taaaaattag ttgggtgtgg ttgtgggtat ttgtaatttt agttatgtgg 12900
gaggttgagg tatgagaatt gtttgaattt gggaggtaga ggttgtagtg agttaagatt 12960
gtgttattat attgtgtttt agtttgggta atagagagag attttatttt aaaaaaaaag 13020
aaaaaaaaaa aaagaatgag aatatttagg gatagtaata ggatagagag attaggtatt 13080
tatttagttt taggatggtt ttataaagga aaggattttt gggttgggat ttgaaagata 13140
agaaagattg ttaggtgaaa gaaggaagtt ttatgtggag tgagtagttt ttgtaagggt 13200
ttggtaaaaa aaaaattttt tgaaaaattt gatattgata tggtagaaga tatagataat 13260
atttaaatga ggaggatgga attagttttt tatttttgtt gtaataaata attataaatt 13320
tagttgttta aaataatata tttgttattt tatagttttg taggttagaa gttttatatt 13380
ggttttagtt gaaattaagg tattggtagg gttgtgtttt ttttggaggt tttaggggaa 13440
aatttatttt ttgtttattt aagttgttgg tagaatttaa ttttttgaga ttgtaggatt 13500
gaagtttttg tttttttatt gtttgttagt tgatagttat ttttagtttt aagaggttgt 13560
ttatagtttt tgattttgag tttttttttt ttatttttaa agttaataat ggtaggttga 13620
gtttttttta tattttgaat tttttttgtt ttttttgtta ttatattttt gagttatttt 13680
ttttttttta ttatttgttt tttagagttt atatgattag attgaatttt attaaataat 13740
ttagaaataa tttttttgtt ttagggtttg taattttaat tttatttgta aagttttttt 13800
ttgttatgta gtgtaaataa tatataggtt ttgaggatta ggatgtggag tagggttggt 13860
attatttggt ttattatagt ttgttttttg atttttatag atttatattt gttttatatt 13920
taaaatatat ttatattatt ttaagatatt tatgagttgt attttattat agggagtatt 13980
ttaagtaaga ttggggagtt agaagttagt ttgggaaggt ttagggaatg atggtagggt 14040
ttgaagatag agatggagaa aagatgataa aaaaaggaat tttttttttg ttgatttttg 14100
ttttttttat gtttatagtt ttttaattta gttgtttgtt ttttagg 14147

<210> 282
<211> 14147
<212> DNA
<213> Artificial Sequence

<220>
<223> chemically treated genomic DNA (Homo sapiens)

<400> 282

tttggagagt aaatagttga gttgagaggt tgtggatata ggaggggtag aggttagtgg 60
gaggaggatt tttttttttg ttattttttt tttatttttg tttttaaatt ttattattat 120
tttttaaatt tttttagatt aatttttagt tttttagttt tgtttgggat gttttttgtg 180
atgggatata atttatgggt attttaggat ggtgtgaatg tattttgaat gtgaaataaa 240
tgtgaatttg tgggggttag agggtagatt gtggtaggtt aaatgatgtt aattttgttt 300
tgtattttaa tttttggaat ttgtatgtta tttatattat atagtagaaa ggaattttgt 360
agatgaaatt aaggttataa attttaagat agggagatta tttttggatt atttgatgaa 420
atttaattta attatatgag ttttgaaaag tagatgatgg agaaagaaag gtagtttaga 480
gatgtgatga tagaagaggt aggggagatt taaaatgtga gagggattta atttgttatt 540
gttggttttg aagatggagg aaggaggttt agaattaagg attgtgggta gttttttaaa 600
gttgggaatg gttgttagtt gatagatagt aaggaaatag ggattttagt tttataattt 660
taaggaattg gattttgtta ataatttgaa tgagtaggaa atggattttt ttttagagtt 720
tttagaagga atgtaatttt gttaatattt tgattttagt tgagattagt gtgggatttt 780
tgatttatag aattgtaaga taataaatgt gttgttttaa gtagttaaat ttgtgattat 840
ttgttatagt aagaatagaa aattagtttt atttttttta tttgaatatt atttgtattt 900
tttattatat taatgttaag ttttttagga ggtttttttt ttgttagatt tttgtaaagg 960
ttgtttattt tatgtggggt tttttttttt tgtttagtag ttttttttat tttttaagtt 1020
ttagtttaaa ggtttttttt tttatgaagt tgttttaaaa ttgaatgaat gtttgatttt 1080
tttgttttgt tattgttttt gaatgttttt gttttttttt tttttttttt tttttttgag 1140
atggagtttt tttttgttgt ttaggttgga gtgtagtata gtggtataat tttgatttat 1200

tgtaattttt gttttttagg tttaagtgat ttttatgttt tagttttttg tatagttggg 1260
attataggta tttatgatta tgtttggtta atttttgtat ttttagtaga gatatggttt 1320
tattatgttg gttagattgg ttttgaattt ttgattttaa gtgatttatt tattttttat 1380
tttggttttt taaagtgtta ggattatagg tgtgagatat tgtgtttggt tttgaatatt 1440
tttttttttg atgatagtaa taattaatat ttattgagag tttaatatat attaggtatt 1500
tttttaagta gagaggtaat atggtttgtt ggataattgt ttaggtatta gggttagatt 1560
gttagtgttt gaattttggt tttgttattt attagttgtg tgattttggg tatatttttt 1620
tttttttttt aagaaatggg gttttattat gttgtttagg ttgtttttga atttttgggt 1680
ttaagtaatt tttttttttt ggttttttaa agtgttagga ttataggtat gaggtattat 1740
gtttggttga tattttttaa tttttttgta ttttaatttt tttatgtgta aaatagggat 1800
aatgatatgt ttattagtaa aatggggata ataatattta gtttatagag atattgtata 1860
gattaaatat tatatgtttg aggtgtatga taagttttaa atattagttt ttgatttgtt 1920
tatagttttt tatttgttat tttaatattt aaaatgtttt aagggtagaa agtttttttt 1980
aatttttttg gtggtaaaat ttgatttgaa atgaaataag gttttttttg gtttttattg 2040
aattttattt attgtgagtg tttatatatt ttgttgtaga aatatgaata tagttgatta 2100
tagagtttat taaggggtgt tatgtaatat tgtatatgta ttatgttatt tttttgaaat 2160
gtgaagaaat ggaaatttg aaatatattt ggtttagggg gttttgggta agggtatgta 2220
tatttgtatt gttatttttt tttttaatgg gtattgattt atttattttt gattatgttt 2280
ttgagaagta ggtagttttt ttgttattaa tttataaatg aggtagtgga ggtataaatg 2340
tttatgtatt ttttataggt tatatgtggt aaagttagta tttggagtta ggtagttagg 2400
ttttagttga ttaagtattt tattattagg ttaaatggtt ttttatattg attatttttt 2460
tttagtatgt tttttaaaga ataggatttt ttttgttatt ttattattgt tttttttttt 2520
gagagtaaat tggaaataaa tatgagtttt ttgtttaatt atataagtta taaagttatg 2580
aagtgtttga ttagagagtt ttgttattag agattttatt gaatttgttt tgtaggtagg 2640
tttgttttag aagtttaaag agtttagagt gatttttggt ggtaaagata ggtaatttta 2700
ttagtttttt ttttagttgt aatagaattt attagttgag tgaaaggtga tggtgggttt 2760
ttagttttaa tagtatatag ggttttgtat tttgttattt tttttatttg attttttata 2820
agagttttag taatttgttt tgtttagata tgtttatttt tttttatttt ttttatttag 2880
taagtatttg gtgagtgtat attgtgggtt aggtgttgtg gatatagttg agaatgtaat 2940
agataataat ttttgtttta gaggagttta tattttagta ggaaaggtag atgagaaaaa 3000
aggtaaatat gtataagagt gtgttagatg gtggtaaggg ttatgggaaa aaaattaagt 3060
aggggagagt tgttattttt atatggagag ttaggggaga gtagttgtta aggaggggat 3120
aattgggtag agattaaaag gaggtgaagg aattgagttt gtgagtattt gagagtagta 3180
agttaggtgg aagtggtagt tggtgtgaag ggtttaaggt tggagagtgt ttggtaggtg 3240
atatggggag aggaggttga tatgggtttg ggggagagga gggaggtagt gggagaggag 3300
aatagagtga tgggggttag attgagttag gtttttttgg gtgttgttag gatgttgagt 3360
gagataggag ttataagagg gttgtgaata gagggatgat aggttgtgat tgagatttta 3420
ataggatttt tttggtggtt gtgagaagaa tagattgttg gggttgaaga agttattgtg 3480
gtagatgatg gtggtagtag tggggttggt gagaagtagt tggggtttgg agaaagtttg 3540
aatatagaat aggatttgtt gatagatagt atgtttgggg gaggtgagag aagggtttag 3600
ggtgagttta ggtttgtggt ttaattattt ataaagatag tattgtggtt agttgaggtg 3660
ggagttttgt aggagagtaa gttttgaggg gggttatatt agttttggag atggttagtt 3720
ttagaggttt gttagatatt tatgggtatg ttatgaatat attttaataa tttaaaaatg 3780
taagttaggt atatgatttt tattgtgggt tgtatatttt ttgaattatt taatttattg 3840
aatttttaaa ttattgttaa agggtgggga ttattatttt ttttattttt tatatgaggt 3900
aatggaggta tagagaagta atttgtttta agttatatag ttggaagttg gtagagttgg 3960
aatttaaaat ttaagtggtt tggttttagt gagtaattgt tttatattat tagtttttta 4020
tgaagagaaa tagagaaggg taagatttta gagagtgata ggaatgttgt ttttatatag 4080
ggtgtataag gagttttttt ttaaggaagt gatatttggt ttgaaggagg ggaggtagta 4140
agttaggttg ttttttgtgg gggagggttt ttaggtggag tgaatggtgt gtataaaggt 4200
tttgaggtag ttattttgaa ttatttattg tttttgagat agttttaatt tttgagggta 4260
agtatgggtt gttttttttg tagggagagt ttttttttta ttttatggat ttaattatat 4320
aattattgta ttttaggaag aatttatttt ttttttgtgt gattaaaggt gttaaataat 4380
ttgtttgttt ttgtaaattt gttttttttt ttgagttttt agttttatgt ttttttgttt 4440
ttgttttggt tattttggtt taaggttatt ttgagtaagt tatttagttt ttttttgttt 4500
gtttttttat ttgtaaaatg gggatatttt atttttaaag ttaaatattt ttaaaagtat 4560
atttattttt taaggttttt gtgaggatta attgtgttaa ttttgtaaaa tatttttgta 4620
aaagatttga ggggttgatt tttttttttg tttttataag tagtattgtt aggaattttt 4680
tttttatata tgtgtttttg tatagtatat tttaagtatt atagtgttta ataatgaata 4740
tttattgaaa taatattatt taggtgttag aaaattaaat tatttttaga aatatttttt 4800
atgtttgtgt ttttaggaga gtgtattatg tatattatag tggttatttt ttgggttttt 4860
ttttggagat ggggatttta ttatgttgta taggttggtt tggaattttt gggtttaagt 4920
gattttttta ttttagtttt tgtgtttagt tatatattat agttttgtga atatattttt 4980

ttaatgtaat aatatagttt ttatttatat atataattag tatttttaga aatagttttt 5040
atatttgtgt ttttatagag tatattatat attttagttt tgtgaatata tttttttgaa 5100
aaggaggaaa atttttattt atatataaat tggtattttt agaaatattt ttgatatatg 5160
tatttttttg tagtgtattt taggtattat tgtgtggtaa atattttttg ggggttggta 5220
tttgaataga aaagtgtttt tgatgtgtgg tagatatttt atagatatta gttattagtt 5280
agtgtgttat tttttttttt ttgtttttgt aattttattt tatgtattat ttgtattatt 5340
tggtttttat tatttgtgtg attttatttt ttattttttt ttgtattttg ttttagttat 5400
attggttgtt ttgatgtttt ttgaatatgg taggtattat tttattttag ggtttttgaa 5460
ttggtatttt tttttgtttg gtatgttttt tttgtagata ttaaggtttt ttttgttttt 5520
attgtgaggt tttaatttaa gagttatttt ttttatgttt aagattttaa ttatttttaa 5580
tatttgattt ttatttgttt tattttatta ttttttttta gtatttgttt attatgttaa 5640
ttttatttat ttattttgtt tatatagttt gtttttttag tttgggtttt tttttttttt 5700
tttttgagat agagttttgt tttgttattt atgttggagt gtatggtgta attttggttt 5760
attgtaattt ttgttttttg ggtttaagta atttttttgt tttagttttt taagtaattg 5820
ggattatagg tgtatgttgt tatatttagt taattttttt gtattttagt agagatgggg 5880
ttttattgtg atgtttaggt tggttttgaa tttttgagtt taggtaattt atttatttta 5940
gttttttaaa gtgttaggat tattggtgtt agttattgtg tttagttttt ttaattttta 6000
ataaatgtta gttttataag tataaatatt ttgttttgtt ttgttttttg ttatatttta 6060
gtatttagaa gagtgtttgg tatttataag gggtttagta aaggttggtt gtaggaatag 6120
ttttaattaa ttagttttta tatttttttt ttttttttat gtgattttta tattggtgtt 6180
ttaaattatt ttttttttat ttatttatgg gataaatttt tattgagtat ttgttgttta 6240
tttggtatat ttttagttgt tggagatata gtggtgaatg agtagataaa aattgagttt 6300
ttatgggatt tatattttaa tgaggagaga tagataataa ataagtaatt tatatagtgt 6360
gttagatagt gataaagggt aatgggataa attagggatt tggtttgagt tttgttgggt 6420
gtgtagtttt agatggttag ggaggggttg attttttttg tttttataag tagtattgtt 6480
aggaattttt tttttatata tgtgtttttg tatagtatat tttaagtatt atggtgtgat 6540
aattaatttt taatgaaata atattattta ggtataataa aattaaatta tttttagaaa 6600
tattttttat gtttgtgttt ttaagagagt gtattatata tattatagtg gttgtttttt 6660
gggttttttt tttttagaaa aagggatttt gttatattgt ttaggttggt ttggaatttt 6720
tgggtttaag ttattttttt attttagttt tttgagtagt tgggattata agtgtatatt 6780
tttgtgttta gttatatatt atagttttgt gaatatattt ttttaatgta ataatatagt 6840
ttttatttat atatataatt agtattttta gaaatagttt ttatatttat gtttttatag 6900
agtatattat gtattttagt tttgtgaata tatttttttg aaaaggagga aaatttttat 6960
ttatatataa attggtattt ttagaaatat tttttatata tgtatttttt tatattgtat 7020
tttatatatt atagtgtggt aaatattttt tgggggttgg tatttgaata gagtgttgaa 7080
aggaattatg tagagatttg ggggtggtgt ttaaggtata aagaatagtt aatgtaaagg 7140
ttttgaggtt ggtttatgtt tgttatgttt gaggagttgt gtggtggaat tagtgtggtt 7200
ggagtatagt aagggggtgg gaaggtatag gtggtaatgt tagaaaggtt atttgtttta 7260
ttattaattt atagttaata tttatttata ttggaaggga ttttagtttt gattttttat 7320
tttataaatg aagaaaaagg tatagagagg aagtggttaa attatttatt taaggttata 7380
tagttgttgg gattgggggg tgggtgggtg ttgagagaag gtgaggaatg aagattgttt 7440
tgtaggtaga gggaatagta agaataaaag tttggaggtg agaattaatt tggtttttaa 7500
ggattgaggt tttatgtggg gtgggatgag atttaagagg ggagttttaa aaattatttt 7560
gtgttttgag tattttttgg gttatttttg agtaaggaat ttaatttttt tatggtttaa 7620
tttttttatt atttatttta tggggttgtg gtgaggatta aaggaattta tttatatgaa 7680
atattttgtt tgatatatgg taggtgttta ataaatgtta gtttttattg gtattattat 7740
tattattggt gttaatgtat ttatttattg tagtgaggta gaatgttttt gtggttatga 7800
gtgtgattag gttgatttat tattaagagg tggaggatag ggaaggttgt atagtgtttt 7860
attataagga aaagaggatt agtatgtttt tttttgggaa gggaaaaaaa aagagaagga 7920
aattgaaagt atatggttga ttttttttta attataaagt attggattta tgggagggta 7980
tgtgggggat tgtgtaagta tataggggtg tgggggagga aggttaatga atagatttat 8040
tgtaaaggga ggggtgatga ggtttattgt ttgaggggaa ggggagtaga aaattttata 8100
gatttttgta ttatagtgag aggagagaaa agttattata tagattgatt taaagtagag 8160
agggaaaagg ttatggattg agttattgaa aatagggaga tgtaaagtat taaattaatt 8220
tattattaaa aaggagagaa ttttaagtgt tagagtgata gtttatgaaa aagaggagat 8280
agggtaatgg attgatagtt aaagggatgt gaatttaaat atgaagattg atttattata 8340
aaatggtggt ggtggtggtg gggggggggg gtggttttga aaaaagttat ggattgatgt 8400
attgggggat tggaggaggt aggaatataa atttaggtag atttattgtt aaagagggat 8460
gtggaggagt gtggtgttat ggattgattt gttatgatgt ataggaaggg tggaataaat 8520
aaggttatgg atgggttttt tgaaagttga ggttaggaga aatttaaata tttagatata 8580
tttattatta agggggttga atattgttat aggttagttt tttataagtg ggggtgggtg 8640
gggggtgggt gggggtaggg gtagggggaa agtttatgga tgagtttatt ttaagttggt 8700
gtggggaaaa atataagtat gtagattgat ttattatata atgggagggg gaggttaggt 8760

tatggttaga tttattataa gggatgaagg gtttgagtaa tgttatatgt aggtttatta 8820
gttgtggtgg tggtggtggg gagttgaata ttagtaggtt attggttaag ttatattttt 8880
tgtgtggggg tgggaaggat atgtggggtt atggattggg ttttttattt tttaggttta 8940
gagtttattt gtgtgtagtg tggtggtggt gttggtaatg ttggtagttt tggggggtgg 9000
gggttttata tgtggtttta ttgtttttta ggtttgggtt ttgaggtggt ggtggaggtg 9060
gtggtggtgg tggtggtggt agtatttaga agttgttttt gtgttttttt atagtttatg 9120
tgggtttgag gaggaggagt ttaatgtttt gttgtttgtt gattggttaa agtgttatta 9180
attggttgta aggttttgag ggttgggggg atgggttttt ttttatagag ttgtgttgtg 9240
attggtgtaa ggggaaatga tggaatgttt atggtttttg gagttttgag gaggtgtgtt 9300
tggtttttat ggtgttttat gttgattggt ttaaggattg atggattgtg agtaattgaa 9360
aaggtttggg gtttgtgttg gggggtgggg ggtttgtttt atattgattg gtttatatgg 9420
gattgatgga agatagtttt taaggatggg ggtgggtggt tttgtttttt tttgattggt 9480
ggatgaggga tttttagatt tttgttaaaa tattaagggg gtggtgttgt atgttatgtt 9540
gtttagtgga agtagggttt gtatagaaat gggtgtagta gttggtggga gtgggttgga 9600
ttgggttttg tgtaggtggg tagggtagtt ttatggttag ataagattat agagttgggt 9660
aaagatgaat ttagaatata gtggaggtag gagggtagga tggttgttag gtatatgaaa 9720
gagtattgag tggtagaaat gaaatgtttt tagagggtag ggttgaattg tggttagagt 9780
taaaaagggg aggaatttgg ttttgtattg attggtggtg gttggattta atttagaata 9840
ggggtttgat tagggagagt gggatttagg tttgtgttta ttggaagttg gaatatttat 9900
aagtaggggt taagataagg tagggttgag ttagggtttg ggtgtaattt ggggtatttg 9960
gggaatgatt gggtgtgtga aagtgtaaat tttagggttt tttgattgtt gtgggaatga 10020
ataaattttt attatgaatt ggtttatttt ttatggtaat ttaaagggtg gtgtttagga 10080
aaaggaatgg aagaaagggt gaggtggggg gtttaggttg ttaagatgat agaagagagg 10140
gtagtgtata aggttaattg gtaaatattg ttaggtaata ggttggaggt aggattgttg 10200
gggtattttg gaggttttat agagagattg gatttttggg tttttttagta atttgtgttt 10260
ttaggaatag gtttttgtgg taattagtag ggtttatggg gaaaaagaag ggttatgtga 10320
tttaaaggta tttaggtttt ttaatgtttg aggatggggg gaagaagtta atggtgaggt 10380
ttttttggga aattttgtag gttttgtagt tttttaagtt tttgaaaaag aggatagaag 10440
agattgaata aagattgggt ttgggttatg tttttaggtt tgttttgatt tttttttttt 10500
gattttagtt tagagggaaa gatggttttt tttttttttt gttttttttt ttttttaggt 10560
tattatttta aatatagagt ttggtagtgg ggtgagttgt ttttattttt agtaatatgt 10620
ggatatgggt tttgatattt atggagtttt tggtgaggtt gttgttgagt ttagagaaaa 10680
gaaataagaa agggtaaaat gggttttata tgattatgat gatttttttt tattatttta 10740
agatttattt atattttgga tgatttttat ttggtttatt tttatgtgga aatgttaagt 10800
tattatttgg tttttattga tttaaagatt attaaatgtt tattgtatgt taggtaattt 10860
taggtattgg gagatagtag taaatataat aaataaaatt tttggtttga tggattttaa 10920
atttttgtaa agaaagatat aataaaaaat taatggaata aaagttatgt gttagatggt 10980
tttaattata ataataaaaa ttaaaaatga gagataataa ggagtatagg agtggggggt 11040
tgtaatttta aagagggttt agggatggtt ttatggagaa gtatttgagt aaagatttaa 11100
agatagtgaa ttttgagtag tgtggttata tggaggatgt gttttatata taaggggtag 11160
taaatataaa ggtattgagg tgggagtgtg tttggtatgt ttgaggtgta gtaaagaggt 11220
tattttggtt ggagaagaat aagtgaaggg ggaatgtaag aggtgaggtt agagaggtaa 11280
tgagggagat aaattgtgtg aagtttataa ggttatggta aggattttgg ttttgatttt 11340
aagtgagatg ggagttaagg gaaggttgta gtagaggaag gaggttgaat tatatgaatt 11400
agttagttga tttttttttt ttaagttagt ttggattaga ttttgtttaa aagaatttta 11460
attgatataa agggttaagg aaggtttttt ttaagaatga agaatgagaa ggaattgaag 11520
aatgtttaat ggttaagtag taggaaggtt ttttttataa aaaggataag aggtaataaa 11580
gagttgtttg agaagtagtg aggttagagt gttagaaatt gagtggggag ggaaagtggt 11640
ttaagataag gtttaagaga taggaagagg ttagattata tagggtttta atataataag 11700
gaatgtggat tttattgtgt tgttaatgta ggagttagtt tgaggggtaa atgaaattaa 11760
atattgttgg aaaagtagag agtaataaga taggatgtta aattggaaaa aaaagtagta 11820
tgaatttatg tatatattaa tattttttat atttttattt ttagtttaga attttttttt 11880
gaatttgatt agttttaatg atatttttta aaaatttatt ttagtgttta ataagtgttt 11940
tatatttaat aggttttaga ttaaattttt gatttttttt tttaaattta tttttttttt 12000
aattttttta ttttagataa tggtaatttt atttttttag ttgtttagtt aaaaatttgg 12060
gttttttttt tttatatttt atatttagtt agttattttt tttaaaatta tgattgtttt 12120
attgtaggta atgtttttga tttttatgat atgttttttt tatagtattg ttaatatttg 12180
atatattata ttatttattt gtttattgtt tgtttttttt gattagaatg ttagttgtat 12240
gagggtagat tttgtttttt tttgtttttt gttttatttt tagggattag aatagtgtgt 12300
ggtatagagg aggtatttaa tgtatgttga atgaataagt gattatttta ttaaaaggaa 12360
tttgaggttg gttgagtgta tgtttaggga taggaaataa atgaagtatg ggtgttgttg 12420
ttagagagta agaatatttt taagaatatt ttgggggtaa tgtttaaaga atagaagaat 12480
gaatttgatt agtttttaag ggataatttt aatattaaaa aagagaataa aattagttaa 12540

tggtgtaagt ttattttatg aaaatttatg agtttaatat tttaaaaggg agaaaaatat 12600
agagtttatt aaatgttagt tgtaatataa aagggatatg tttattattg ttgatttatt 12660
tttatttgtt atgtgagaag taaattatta ttgatttagt atgtaaggta gtgtgaatag 12720
ttatatggtt atgttttttt tttttttttt gagatggagt tttattgtgt tgtttaggtt 12780
ggagtgtagt ggtgtgattt tggtttattg taatttttat tttttgagtt taagtgattt 12840
ttttgtttta gttttttaag tagttgggat tataggtgtt tgttattgtt tttggttaat 12900
tttttgtatt tttagtagag atggggtttt attattttgg ataggttggt tttgaatttt 12960
tgattttgtg atttatttat tttggttttt taaagtgttg ggattatagg tgtgagttat 13020
tgtgtttggt tgttaggttg ttttttttat aattatatgt ttatttatga gatgtggatg 13080
tgtatttttt tttgtgttat taggaaagaa tatagagatt ttattttttt tattttttaa 13140
ataaagaaat atattaagaa taggaagaat tgatattttt gattatataa aatgattaga 13200
aagttagttt aaagtatatt ttaagtaatt aaatattttt aaaggatagt tagttatata 13260
gatttaaatt tttatttttt agaaaaatta aatttttaga tagttttatt aattataata 13320
aagtagtaag atttaattat attttttatt atattaatag attaggttag tatattaaaa 13380
agattaggta agggggtagg tattgataat ttaaatattt tattttgggg aagtatgtgg 13440
agttttaaaa tattaaaatt tatttgatag gtttaggaat tttgtgatat aataaggaaa 13500
gtttttttat ataaattaga ttttagttgg gtgtggtagt ttatatttgt aattttagta 13560
tttagggagg ttgaggtggg tagattattt gaggttggag tttgagatta gtttggttaa 13620
tatggtaaaa ttttgttttt attaaaaata taaaaattag ttgggagtgg tggtgggtgt 13680
ttgtagtttt agttatttgg gaggttgagg taggagaatt ttttaagttt aggaggtaga 13740
ggttgtagtg agttaagatt gtattattgt attttagttt gggtgataag agtaaaattt 13800
tgttttaaaa ataaagtgga tttggtttaa atgattatta gtttagaatt gtgttgatta 13860
tgaaagtagg ttgtttaatt aaattatttg ttaattggtt tgaatattaa gaattagaaa 13920
ttttagtagg tttgaggttt ttttgggttt attagtattt gttagttttt tttatggttg 13980
gaaaggtttg aaaagataaa aattattgaa attttaaggg aggtaatttt ggaaaaaatg 14040
aatgtagaat agatataaat ataaaatata attaataaag aagtttaaat taatggtatt 14100
tatatttgtg agatgttgtt aatttaagtg tattgtttag tgtttttt 14147

<210> 283
<211> 3120
<212> DNA
<213> Artificial Sequence

<220>
<223> chemically treated genomic DNA (Homo sapiens)

<400> 283

aattttaagt ttaggttggg tatagtggtt tatgtatgta attttagtat ttggagggat 60
tgaggtgggt ggattataag gttaggagat tgagattatt ttggttagta tggtgaaatt 120
ttgtttttat taaaaatata aaatttagtt gggtatgatg gtatgtgttt gtagttttag 180
ttatttggga ggttgaggta ggagaattgt ttgaatttgg gaggtggagg ttgtagtgag 240
tggagattgt attattgtat ttgtttggtg atagagtaag attttatttt aaaaaataaa 300
taaagaaaat ttgaagtata gtattttttt aaattttaaa tagataatag aaattggttt 360
tttttatttt aaattagaat ttaagtttaa ttttatatat ttttgatagt ttggattttg 420
ttttttaatt ttataaaatt gggaatttaa gtattatttg gtttgattta aatgtaatgt 480
agaatttgta ttaaaatatt atattaaagt tttagatttg tagtagttaa tagtattttt 540
atgtatgtgt tagggattgt tttaaatatt ttatatatat taattttttt attttgtatt 600
tttgtttttg ttttatatag taggaaattg aaatattgag aggttaagta attaaagtta 660
tagagttaga gtgataggag taaagtttta atttaggtaa tttagatttt tagagttttg 720
atttttatta ttaagttgtt agtatagttt ttttggtaat tatttttaat ttaaatataa 780
tttgagtgat ttatttaata agttattatt ttgataattt agtgatttgt aatgtaaaat 840
tatttattgt aatttattta atattattgt ttttttgtgt tgtaaaaatt atagtaattg 900
agatgtaatt tattattttt ttttttattt ttggtatttt gtgttaattt ttttgttttg 960
tggatttttt ttgatttttt attatgtgtg ttaattgtta ttaatttttt tgtttgttgg 1020
ggattggggt tgtgagggta tatttttgag gggtatgggg ttagggttag gtaggttgtg 1080
tggttgggtg gggttttgtg ttttattgtg gagtgtgggt tgggaagtgg agagagaagt 1140
agttgtgtaa tttgttggat gtggattagg gtgtttttta tttttgttgg gagtttgttg 1200
attggttggg tgtgggtgta tgtgattgat atgtggttgt attggtgtag tttgttaggg 1260
tgttattgga gatagaatgg aggtgttgtt ggatttggaa atggggtagg tgttggagtt 1320
attatggtta ggtttgttgt ggggggaggg gggaaggtgg tttttttttg tattgtttta 1380
aattgatggt ttttttttgg tatagggttt attgtagtat gttaaatgag gaggtagggg 1440
tgttgttttt ttgtttttta ttgtagtatt ggagatggat tttttgtatt ttggatttag 1500

ggtttttgat agaagaggaa gaagggggag gggtagaagt gttaagggga gtttgttgag 1560
aaaagttgtt tttgaagtta gaaggggttt ttgtttttat aatgttattt gatagagtgg 1620
aataatagta tttaaggaaa tgggtagagg ataataaaga atggagtata tttatggtga 1680
ggagtaaaag ttttatttta ttgaaaggtt tttttttttt tttggtgata aggatatatg 1740
tattggtggt taaaagagag aggagataaa attgttgtag atggttgatg tgaatttagt 1800
ggaaagagtt attggggatg agagaaagag gaggaggtag gtattgtaga gtgtgagtgg 1860
tggtgttggt tggtgaaata ttggttatta gtagtgtgtt tgtttttgta aaatatttaa 1920
gtaaattttt tgtgaatagg gtggtaaata gatattagtg tttttgttag ttataaaatg 1980
tagtggtagt ggttttttgt ggatgattgt agtagtgttt tttttttttt tgttaggttg 2040
aaaagataat tgtagaggaa taagaaattt tgtagtaaat gttggggtag aagtttattt 2100
ataagaagtt atagtttata aatgtagttt gaatagtaga aaaaaaatta ttgtttttta 2160
aagtaggaat aatgttaggt tatgaatgtt ttgttattgg aatgtattgg atattttgat 2220
tttatattat gaaagtgatg tttaaatttt ttgatttaat ataaatttta tatgaaattt 2280
taataaatta tgtatgaaat agtggatttt tttttttgtt agtgaagttt ttatgttatt 2340
aattaggtta tttaagagta aattatttta taatgtaaat tattttgtta aaaattatgg 2400
tgaataaatt tttgtaggtt taatatttaa gatttatagt taagtaattt tatatttttt 2460
tttggttgtt tttagataat attgaataaa tatttaagat attaatttag tgtgtaaata 2520
ttttaaatta aagtaatatg gttttttttt tagatgtatt tttgtttagt gagtatttat 2580
gagatatatt tttgatataa agtgtttttt attgagtttt tttttttttt atttaaatgt 2640
aaaagtttat ttttatgtat atttatagta gttagtttgt tatatttttt tattttttga 2700
ataaggatgg tagtggtttt gtaaagtttt ttagggttag tgatagtgtt ttttaaataa 2760
tgtttatgta atagaaagtt ttaagatgat ttattatatt gttttagtaa tatttttaag 2820
ataatgtgga attgggttgg tttagataaa taagattata taagtttttt attgataaat 2880
ttaaatagtt ttttaaaaaa ttatttttgt tttaagaatt atttgattat ggatattagg 2940
gttaatagta tttaggagtt gggtgtggtg gtttatgttt gtaatataag ttaaatagga 3000
ggttgaggga agttgaggta ggaggattat ttgagttttg gtgtttaaga ttagtttggg 3060
taattaagta agattttatt ttaaaaaaaa gtatttagag tttttttttt aaaatttgat 3120

<210> 284
<211> 3120
<212> DNA
<213> Artificial Sequence

<220>
<223> chemically treated genomic DNA (Homo sapiens)

<400> 284

attagatttt aaaaaagaga ttttaagtat ttttttttga gatagggttt tgtttggttg 60
tttaggttgg ttttgaatgt taaggtttaa gtgatttttt tgttttagtt ttttttagtt 120
ttttgtttag tttgtattat aggtatgggt tattatgttt ggtttttaag tattattaat 180
tttgatattt atgattaagt aatttttaaa gtagaaataa ttttttaaag aattatttaa 240
atttattagt gaaaaattta tgtagtttta tttatttaag ttagtttaat tttgtattat 300
tttgaaaata ttattgaaat aatataatag gttattttaa gattttttat tatataaata 360
ttatttaaga gatattgtta ttgattttgg agggttttat aaagttattg ttatttttgt 420
ttagggagtg gggaggtgtg gtaggttggt tattataagt atgtataaag ataggttttt 480
atatttaggt aaaggaaaaa aaagtttaat gaaaaatatt ttatattaag agtatatttt 540
atgagtattt attaaataga aatatattta gaagaaaagt tatgttgttt taatttaaaa 600
tatttgtata ttgaattaat attttaaata tttatttagt gttatttaaa agtaattaaa 660
gagaaatata aaattattta attataggtt ttaaatatta ggtttataaa aatttgttta 720
ttataatttt tgataaagta atttatgttg taaagtggtt tgtttttgaa tgatttaatt 780
aatggtataa aagttttatt aataaaagaa aagatttatt gttttatata taatttatta 840
agattttgta taggatttat gttaaattaa agaatttgag tattattttt gtgatgtaga 900
attaaagtgt ttaatatatt ttaatgataa aatatttata gtttgatatt atttttattt 960
taaaaaatag taattttttt tttgttgttt aggttgtatt tataaattat ggttttttgt 1020
aaatgggttt ttattttagt atttgttgta aggtttttta tttttttgta gttgtttttt 1080
tggtttaata gagggagaaa aagtattgtt gtagttgttt gtaaaaagtt attattattg 1140
tattttgtaa ttaataaaga tattggtatt tgtttgttat tttgtttata gggagtttat 1200
ttagatgttt tataaaagta ggtatattat tggtgattag tattttatta attaatatta 1260
ttatttatgt tttgtagtat ttgttttttt tttttttttt tatttttagt agtttttttt 1320
attagattta tattagttat ttgtagtggt tttgtttttt tttttttttg gttattaatg 1380
tatgtgtttt tgttgttagg gaggaaaaga agttttttaa tggggtagag tttttgtttt 1440
ttgttatgaa tatgttttat tttttgttgt tttttatttg tttttttaga tattgttatt 1500

ttattttgtt aaatggtatt ataaaaataa aaattttttt tggttttaaa aatagttttt 1560
tttagtagat tttttttaat atttttattt tttttttttt tttttttttt gttaaaaatt 1620
ttggatttga agtataggaa atttattttt agtgttgtag tggggggtgg ggggatgatg 1680
tttttgtttt tttgtttggt atgttgtagt ggattttgtg ttaaggaaag gttattggtt 1740
taagatagtg tgagggaaaa ttattttttt tttttttttt gtagtaagtt tggttatggt 1800
ggttttagta tttattttat ttttgagttt ggtagtattt ttattttgtt tttagtgata 1860
ttttggtggg ttgtattaat atagttatat gttggttatg tgtgtttata tttagttaat 1920
tggtgggttt ttgatgggaa tggggagtgt tttggtttgt atttagtgga ttatatagtt 1980
gttttttttt ttgttttttg atttgtattt tgtagtgggg tatagggttt tgtttaattg 2040
tatagtttgt ttagttttag ttttgtattt tttgggggta tgtttttgtg gttttagttt 2100
ttagtaagta aggaagttga tggtagttga tatgtatagt aaagagttgg gggaggtttg 2160
tagggtagaa ggattagtat aagatgttgg aggtgggagg gagagtaata aattatattt 2220
tgattgttat gatttttgta gtatagagaa ataataatat taaatgaatt ataatgaata 2280
attttatatt ataagttatt gagttgttag agtgatgatt tgttagatag attatttgaa 2340
ttatatttga attaaaaata gttattagaa aagttatgtt agtagtttag tagtggagat 2400
tagaattttg gaagtttggg ttgtttgagt tgaagtttta tttttgttat tttagttttg 2460
taattttagt tatttaattt tttagtgttt taattttttg ttgtataaaa tgggaataga 2520
agtatagaat agaggagtta atatatatga agtatttaga gtagtttttg atatatatat 2580
agaagtgttg ttagttatta taaatttgag gttttaatgt agtattttaa tgtaaatttt 2640
atattgtatt taaattgaat taggtgatgt ttaaattttt aattttatga ggttgaagga 2700
taaaatttaa attgttaaga atatataaag ttaaatttaa attttggttt aaatgggggg 2760
aaattagttt ttattattta tttaaaattt aaaaggatat tatattttaa gttttttttg 2820
tttgtttttt gagatagagt tttgttttgt tattaggtga gtgtagtggt gtaatttttg 2880
tttattgtaa tttttatttt ttgggtttaa gtgatttttt tgttttagtt ttttaagtag 2940
ttgggattat aggtatgtgt tattatgttt agttaagttt tgtattttta gtagagatgg 3000
ggttttatta tgttggttag gatggtttta attttttgat tttgtgattt gtttattttg 3060
gtttttttaa gtattgggat tatatgtgtg agttattgtg tttggtttaa atttaaagtt 3120

<210> 285
<211> 2501
<212> DNA
<213> Artificial Sequence

<220>
<223> chemically treated genomic DNA (Homo sapiens)

<400> 285

ttatatggtt agaaagggtt taggtggttt ttggtgtttt tttagttttt gtttttatgg 60
tgtatttttt tgttagttgt ttttttttgt attttgttgt ttgtttggtt tggttgtgtg 120
tggttttttg ggagttttta ttttgttgtg gattttagtg attattaagt ttgtatgtgg 180
tttgttgtag gtttgagtag aaggttttgt gtatatttat tgtgttgtgg ttgtgtggga 240
ggtttgtgtt gtttgtgtta tttattggtt gggttttgtg ggtgtagtgg agtgggtggg 300
tggttggttt ggatgtgttt tttttggttg tggttttgtg tgttatgtgt ttttggtggg 360
atgtgttatt tttgggtttg ttgtggtgtt tttaatttgg gttagggagt ggggtggagg 420
gggtggttgg gtggtttaga ggagggtttt tttttttttt tttttttgaa tgaattgtgt 480
gatgttatgt ataggaaatt ggttgggttg tgtagagaat gaagtaagag gataggtatt 540
atagttttgt ttttgttttt ttttttttgt gtttgttttt ttgtgttgtt tgtttgtttg 600
tttgttgtgt ttttgtttgt tgttttttgt ggttttgttg tgttgttgtt gttgttgttg 660
ttagtagaag gtgttggggt tttgggtttt ttttgttggt ggttgttagt gtttggagtg 720
ggttgtgtgg tgggagtttt gggaggtggt tgtagttagt gttgttgtgt aggattagga 780
ggaggagaaa gggtgtgtag tttggaggtg gggtgtgttg gtggggtgta gtggaagagg 840
gggtttaggg gggagaattt tgtagtagtt atttttttta ggaaaagagg gaaaaaataa 900
aatttttttt tattattttt ttttttttat tttttgttgt attatatata gtgtgggttt 960
ttagtgtttg gtattggtgg gttaggtgtg ttttgttttt atttatttag tagttttttg 1020
gaaaatgtat ttgttgtttg gagtttaatt agaagaggat ttttgttttt gtttttggtt 1080
tttttattgt tttttttttt ttgttttttt tttggggagg tgtgaagtgg ttttgtggat 1140
agagatttat gtttgtgttt gtgtgtgtgt gtgtgtgtgt aaattgttga gaaggggaaa 1200
atattatagg atttttgtga atattggatt gaaaattgta atttatttgt tgttgttgtt 1260
gttttttttt ttttttgagt ttttgagatt tttggttggg atttttgtgg attgatattt 1320
ttgtgaagta gaagtttggg aattgatttg gaaatttttt taatttttat tttttttttt 1380
tgtgattttt gatttattgg gaagttttaa attagttata attggagagt gttgaagatt 1440
gatgggattg ttgttttatg tatttgtttt ggttttataa aaaggaaatt tgatagagga 1500

ttatgttgta tttaaaaaat ataagtaagt tttttgtata ggaaattggt ttaatgtaat 1560
ttttaatgga aatttttgag attttttatt taaagtgtat ttgagtaaat ttaattttta 1620
ggtagtttaa tatatttttt ttagttgtgt tatttgtagt gtgtatgttt tgtttttatt 1680
tagtgtgtat agggaaatgt atttgatttt ttatttatta gtttgttttt tttttaattt 1740
tttagtatta tagaggaagt agattgatat taataatatt tattaataat aatgtgtttt 1800
atgaaataaa gatttgaaag gaattggaat aaaaattttt tgtattttat gttaaggggg 1860
aaatattaga attaagtgtt ttgtgtgatt gaagatattt ttttgtttaa gaatgtaaag 1920
tatatttaat aaaatagttg gattataatt tttttttttt ttttgggggt tgtggggtgg 1980
gagttggggt gagaggtgtt gttggttttt gttgtttttt ttttgggaag gatggtgtat 2040
gttgggagaa tagggtatga taattgggag atagtgatga agtatattta ttataagttg 2100
ttgtagaggg gttatgagtg ggatgtggga gatgtgggtg ttgtgttttt gggggttgtt 2160
tttgtattgg gtattttttt tttttagttt gggtatatgt tttatttagt tgtattttgg 2220
gatttggttg ttaggatttt gttgttgtag attttggttg tttttggtgt tgttgtgggg 2280
tttgtgttta gtttggtgtt atttgtggtt tatttgattt tttgttaggt tggtgatgat 2340
tttttttgtt gttattgttg tgattttgtt gagatgttta gttagttgta tttgatgttt 2400
tttattgtgt ggggatgttt tgttatggtg gtggaggagt tttttaggga tggggtgaat 2460
tgggggagga ttgtggtttt ttttgagttt ggtggggtta t 2501

<210> 286
<211> 2501
<212> DNA
<213> Artificial Sequence

<220>
<223> chemically treated genomic DNA (Homo sapiens)

<400> 286

atgattttat tgaatttaaa gaaggttata attttttttt agtttatttt gtttttgaag 60
agttttttta ttattgtggt aaagtgtttt tgtgtggtga agggtgttag gtgtagttgg 120
ttggatattt tggtgaagtt gtggtggtag tggtgggaga agttgttgtt ggtttggtgg 180
agggttaggt ggattatagg tggtattggg ttgagtgtag gttttgtggt ggtgttgggg 240
gtagttgggg tttgtagtgg tgaggttttg gtgattgggt tttgggatgt ggttggatgg 300
ggtgtgtgtt tgggttggga ggagaagatg tttggtgtgg gggtggtttt tgggggtgtg 360
gtgtttatat tttttgtatt ttatttgtag tttttttgtg atagtttata atggatgtat 420
tttattatta ttttttggtt attgtatttt gtttttttag tgtgtgttat tttttttaga 480
ggaaaagtaa tgggggttaa tggtattttt tgttttagtt tttattttat ggtttttaga 540
gaaagaagag gagttataat ttagttattt tattggatgt gttttgtatt tttggatgag 600
ggggtgtttt taattatgtg gaatatttga ttttggtgtt ttttttttgg tatgagatgt 660
aggaaatttt tattttaatt ttttttggat ttttatttta tgaggtatgt tattattagt 720
aagtattgtt aatattagtt tatttttttt gtgatgttga aaggttaaag aaaaaataaa 780
ttaataagta aaaaattagg tgtgtttttt tgtatatatt gagtgaaagt agggtatata 840
tattataagt aatatggtta aaaagaatgt attaagttgt ttggaaatta aatttatttg 900
aatgtatttt aagtaaaaaa ttttaaaggt ttttattgaa agttatatta aattaatttt 960
ttgtgtagag aatttatttg tattttttaa gtatagtatg attttttgtt aagttttttt 1020
tttgtaaaat taaaataaat gtataaggta atgattttat taatttttag tattttttag 1080
ttatagttga tttgaaattt tttaatgaat taggagttgt ggggagaggg agtaaaaatt 1140
aggaggattt ttagattgat ttttagattt ttgttttata gaaatgttaa tttgtaggaa 1200
ttttaattgg agattttaag agtttgagaa aaaaaaaagg tagtggtggt ggtagatgaa 1260
ttataatttt tagtttggta tttgtagaag ttttgtgatg tttttttttt tttggtaatt 1320
tatatgtgtg tatatatgtg tgggtatagg tatgaatttt tatttatggg attgttttat 1380
gtttttttag gagagagata ggggagaggg gatgatgaag gagttgggga tggaggtagg 1440
aatttttttt tgattaaatt ttgaatagta aatgtatttt ttgaaaagtt gttggataaa 1500
tgaaggtagg atgtgtttgg tttgttggtg ttgagtgtta gaagtttgtg ttgtgtgtgg 1560
tgtggtgagg ggtggggaga aggaggtggt gggggagggt tttatttttt tttttttttt 1620
ttaaaaagga tgattgttat gaagtttttt tttttggatt tttttttttg ttgtatttta 1680
ttggtgtatt ttgtttttgg gttgtgtatt tttttttttt tttttggttt tgtgtggtgg 1740
tgttggttat ggttgttttt tggagttttt gttgtgtagt ttgttttgag tgttgatggt 1800
tgttggtagg gagggtttgg agttttggta ttttttgttg gtagtggtgg tggtggtagt 1860
gtggtggggt tatggagagt ggtgggtggg agtgtggtgg gtgggtgggt aggtggtgtg 1920
gaggggtggg tgtgggagga agggggtggg agtggggttg tggtgtttgt ttttttattt 1980
tattttttgt atagtttgat tggttttttg tgtgtaatgt tatatggttt atttaaaaaa 2040
agaagaaaga aagagttttt ttttgagtta tttgattgtt ttttttgttt tgttttttgg 2100

tttgggttaa aggtgttgtg gtaggtttgg gagtggtgtg ttttgttggg ggtatatggt 2160
gtgtggggtt gtggttgggg agggtgtgtt tgggttggtt atttgtttgt tttgttgtgt 2220
ttgtggggtt tggttagtgg gtggtgtggg tggtataggt tttttgtgtg gttgtggtgt 2280
ggtgggtgtg tgtggggttt tttgtttagg tttgtggtag gttgtgtgtg gatttggtgg 2340
ttgttggggt ttgtgatggg gtgggggttt ttggggaatt gtatgtggtt gggttgggtg 2400
gatgatggag tgtggagggg ggtggttggt gggagggtgt gttatgaaaa taagggttgg 2460
aaaagtgttg ggaattgttt ggattttttt tggttgtgtg a 2501

<210> 287
<211> 6104
<212> DNA
<213> Artificial Sequence

<220>
<223> chemically treated genomic DNA (Homo sapiens)

<400> 287

tagttttgaa gtaggtagat atgtttatat ttttttttga gttagtgttt ggatttaggg 60
gattagattt tttatttgat atggtagaga gataagttat gttttaggtt tttaaaagga 120
gtttggaaaa tataattaat ttaagttaaa tatttttatt atttttttgt atatataata 180
aagataaaaa tatttatttt attaaattgt tgagagtaag aaagataata tatatatagt 240
gtttggtata tggatggaat ttagttgatg gtagttataa tatttataat attttttttt 300
tgttttaatt taatatgatt tatatgattt tttatattta ttgtatatgt aatatttttt 360
agataatttt atatttaggt tattagtaaa atattgatat tttttttagt atttatatag 420
atgttttttt tttaaagttt ttagtaaaat ttttattggg gaattttagt tgttttttat 480
tttttttttt ttttattttt atttttatat taaaggtttt tatttttaag gtatataata 540
tgatattttg atatatgtat attttgtgaa atgattgtaa taattaagtt aattaatata 600
ttattatttt atatagtttt ttgtgtgtgt gtgtgtgtgt gtgtgtttag agtttttgag 660
atttattttt tttgtaaatt ttaagtatat gttaattata gttattaggt tgtatattag 720
gtttttagaa tttatttatt ttataattga aaggttgtat tttttaatta gtattttttt 780
tttattttta attgtagttt taaataatta ttattttaat ttttgttatt gagtttgatt 840
tgttttttga gattttatat ataagtgaga ttatgtatta tgtgtttttt tgtgttttat 900
ttattttgtg tgttataata ttttttaggt ttagttatgt tggtataaat gataatattt 960
tttttttttg aataatattg ttgattaata ttttattgtg tgtgtgtgat aaatatatat 1020
tatatttttt ttaataattt atttattaat ggatatttag gttgtttttg tattttggtt 1080
attgtgaatt ttgttgtttt tttggtagtt atttttgtat tttttttttt agaagtagtt 1140
ttattgtagt ttaaaagtgt tttaggaaaa tgatttagaa gtaattattt tttttggtat 1200
gttttttagt aaatatgatg atggaaagtt ttgatttttg ttttttttta aatagtgtaa 1260
ttgattttta gttgggattt tttttagtta atttgatagt tttatgtttt tattaggttg 1320
aagaaaatgt atgtgttgtg ggttgttgta ttattgttgt tggtggtgtt gttgttattg 1380
ttgagatttt gtggttgagt ttatattata aaggaattgt agagtagtaa taaatttaat 1440
ttgagttttg aatttaagtt tttaggtgtg atttttttaa ttatgtggaa atttatgtaa 1500
gaatttttaa ttagatttta tgtttatttt tattttttaa gtaaagggta taggaatttg 1560
gtttaaatga atgggaataa atattatagt tagattttat tggagtgttg taaaaatgtt 1620
tttttttaaa attagattgt tttaaaattg atttggtttt ttagattttt aagttaggaa 1680
gtaagatagt aaaaattaag ggttaaatag ttaagtgatt atttttttaa aattagtatt 1740
ttttttggtg atttttgtta gattatgttt tgtttagtat tgaaatttag agaagtttta 1800
ggaatagtgg gatgaaattt aagtgatttg tttatgggaa gttttatttt ttttttgttt 1860
gtttagtaaa tttgggatgg gtttagagaa tgattttgta ttttgtatta gaagatttta 1920
gtttttagag agaattttag atgaaatttt tatttttttg ttttattttt gaaaattttt 1980
atttgggaat tgatttgttg tgataaataa aatgggagga gtttggttga atagtttgtg 2040
gattttggaa agtaaagtga aatgttgttt taattaggtt tgaatttttt gtaagttatt 2100
ttgtgaaatt ggtgaatttg ttagaatgaa tttttattta tttaattttg gtggattgtt 2160
tattagggtg tttggtatgg tgttaattgt tggagaaaat gatttttaaa taaaaatgaa 2220
aaaaattttt ggtttttagt aaggagttag gtaattttta taaatttaag gagtgtttga 2280
gaggtgagtt aatgattgtt ttttttgttt taaagagata gatttttata gttagttttg 2340
aaattgtttg gggttatatt agtgtttttt ggatgtagat tttggttagt gttttatggt 2400
attagttttt gatttgtagt tgttaaagat ttgatagtgg ggagtatgtg tttattttga 2460
aggttgaaga tatatattat agttaggagt agaattttga ttttttaaag tgatatggtt 2520
ataaaatttg tgtttattgg tttttatttt gtggttattg tggtatgggt gaaagttatg 2580
aatttttttt ttaggaaaat atatattata gatatatata tttataaatg gttttaggag 2640
gtttttatgt tagaaatttt tggttttgga aaaaaattat ttttgttgaa gtatgtggta 2700

gttgagtgta aatatttttt tgttatagtt ttagtggtag aggtttaagt attatgtttt 2760
atttagtgtt tgtattgtgg tttttgtaat tttttaagta gttttgtatt gaaaatttag 2820
gttggagatt tgagtttaaa ttgttgaaaa gttttaaggt tttggatagg gttttagtag 2880
gggttatttt tttttaggat ttggggttta ttgtgtttaa gatttgttat ttaagttttg 2940
ttttgtgtgt atgttgtttg ttttatgttt tgtgttgata tgtgttggtg tttttttggg 3000
ttagttttag ttttgggttt tgtggttgtt ttgttggtga ttttgtggag agtgagtttt 3060
agatatttag tttttagttt tgagttgtta tttttttgtt gtagttaaga aggaggagat 3120
taattaaggg tattttagaa gttaggtgtt tttgttgttt tttttgagta tggaggttat 3180
taatttttta gggggaagag atgtagtgtg aggtaggggt gttgtgttaa gaaattttga 3240
tgtttttggg gattgaggat aaaggtgtgg atatgatttt ggggtatttg gagttttgtg 3300
atttgtgtta tggatggtat atttaggggt taatttttgt tttgttttaa agaattttaa 3360
gttagagttt ttgtttttgt ttatagtttt gggatgttgt tgttgtgttt attgtatagg 3420
tagtgtttgg attggttgta gtagattgtg tgttgtgtgt tttattggga gatggtggag 3480
atgttgaaaa gttttttttt tgttattttg gatgttgtgg gtggtaagtg ttttagtttt 3540
tatttttgtt gagttgaatg tttaggtata gtggaattga aatttggttt tgtgggatgt 3600
gagagttgtt gaggttatgt gtaattgggt gtgatggagg gtgtttgttt gtgatgtgtg 3660
taggtttgat gtaagtaggt tattgttgtg tgagtgtgtg gatgtgattg tttgagagat 3720
ttggaggtag gtttgggata tgtttgagtg aatattttag gatatttttt tggttagtat 3780
ttgtttttta gtgtttgtga tttagagtgg gtatatgttg ggagatagta atgggtttgg 3840
gtgtgtgtaa atgagtgtga ttggaagtga gtgtgagttt gatttaggta gggattatat 3900
agtattgtta tatttgtttg ttttttagta gaggattgaa gtgtgggggt gggggtatgg 3960
ggttggaata gaatgttttt gggatatttt ggtaaatagt agttggaagt aaaggggtag 4020
ttgtgtaaat ggtttaggta ggtgatggat ggtagggtag gaagggggag gtttagaggt 4080
ttggatggag gtttttgtat ttgtattttg taatttattt tttaggttta gtaggttatt 4140
ggtttttttt ttatatatgt aatggatttg aagagtattt tgggatagtt tggggagatg 4200
gagatttgga aagtatttat ggagatttta tagaattttt tgtgtggatt aggaaatttt 4260
tgtagatttt tgtttatttg aggtttaggt gttgggttgt tttttataat atttttttaa 4320
gatgagattg tggtttttat tttaaagatg agtatattga gtttttgtga agttatttgt 4380
ttatgattat ataattagga attgggttaa ttgtaattga atttttgttt aataaagttt 4440
ttgtttttag ttttgttttt tgttttttat gagttttggt tttttgtggg taatattagt 4500
tattggagtt agattttttg ggtttagaat ttatttttag gggtattaat ttttaaaatt 4560
ttatttgggt aggggtttgg gattagagtt ggaagagttt ttataatttt ggattttttt 4620
tgttaaattg tgaaattagg ggtggagtgg ggtgagggtt taaaattagg ttggattgag 4680
aggtgaaatt tattatgatg ttaaattgtt tttaaatttt tgtttatttt aagggtgtta 4740
tttgttggtg tttttattat tttttattat ttttattaat gattttaatg taaatataag 4800
tgggatggtt ttgttggatt ttttaggttt tggaagtatg agggtgatgt aatttagggg 4860
taaaggattt ttttgtttat tggttgttgt gtattggtgg aatttttttg atttatagtg 4920
gtgggaataa gagtagttgt tggtgttggg aggtattaga gatattgttt agtttaagtg 4980
ttgttgttgt ttttataggg ttttgttgga tgttgttgtt gttgttgtta ttgtttttga 5040
tttaagttat tttttgttag gtgagttttg agattttggt ttaggtatat gttttttttt 5100
tttttttttt ttatttgtta tttttttatt tttttttttt tttttttttt ttttttgtta 5160
gtttttttat tagatagttt ttgttagttt gtgatttata ttaggtttgt gttttaggtt 5220
ggattggata gttttaattt tttggtttgt tttttttgtt tggttgtgga ttttagtttt 5280
atttttttgt attgtatata ggtttaggtt agttttggga tatttaggtt ttttagggat 5340
tgtgtataga gtttgaggta agagaaattt tttgtagatg gtgtgattag ggatggtgtt 5400
tggagtttag tagttttagg gaaattggtt tagaatttgg aatagagtgg atgggtggta 5460
aataggtatg atgattgagg gataagtagt tttaaattgt aagttttagt tataggtttt 5520
gggagtaaag agagaaagtt ttgggtttta atttaagagt aaaatttggt ttttgtaggg 5580
gattgaggtg ttagtaggtg gagtttaggt tgagtattaa aattgatttt tttatttttt 5640
agattttaat tgtagagata atttttgtgg atagatttta ttttttttat taaggtgtat 5700
gtaaaagttt tagaagagga ggatagtttt gggttttttt atgggattag tttttgtgat 5760
aggggtttgg gagtatgttt tttatagaga tttggttaat aggtttatat gtatttgtgt 5820
ggttttgggg agaagggtag gattgagatt ttttttattt agtttttttt ttaggattgt 5880
ttttttagtt ttattagatt gtgtatgtta taggtttatt gaatttttta gaagtttatt 5940
gtgttgatga ggaaataaaa aagaagagaa atataatgat tagtttgaag ttataaagtt 6000
agagtgttag aagtaagata gaaaattagg ataggagtta aggtttgtgg gtttttagaa 6060
ttttgaattg gtgtaggtgg tttattttat tttttttttg taga 6104

<210> 288
<211> 6104
<212> DNA
<213> Artificial Sequence

<220>
<223> chemically treated genomic DNA (Homo sapiens)

<400> 288

tttgtaaggg aagaatgaga taaattattt gtgttagttt gaagttttag gagtttatag 60
attttggttt ttattttggt tttttgtttt gtttttaatg ttttggtttt gtggttttag 120
attggttatt atattttttt ttttttttgt ttttttatta gtatagtgga tttttgggag 180
gtttagtggg tttgtagtat gtatagtttg atagagttgg aggagtgatt ttagagggag 240
agttgagtgg gggaggtttt agttttattt ttttttttaa agttatatag atatatataa 300
atttgttagt taggtttttg tggaggatat gtttttaggt ttttgttata gaagttgatt 360
ttataaggaa atttagagtt gttttttttt tttggggttt ttgtatatat tttggtggga 420
ggggtgggat ttatttatag agattatttt tgtaattgga atttgagaaa taaaagaatt 480
aattttagtg tttagtttaa gttttgtttg ttggtatttt agttttttgt aggaattaag 540
ttttattttt aaattgggat ttaagatttt ttttttttgt ttttagagtt tgtgattggg 600
gtttgtagtt tagggttgtt tgttttttag ttgttgtgtt tatttgttat ttatttgttt 660
tgttttaggt tttgaattaa tttttttggg attgttgggt tttagatgtt gtttttgatt 720
gtattgtttg tggaaagttt tttttgtttt aggttttgtg tgtggttttt ggggagtttg 780
agtgttttga gattggttta agtttgtgtg tagtgtgaga gagtaagatt ggagtttgta 840
gttgagtagg aagagtgagt tgggggattg agattgtttg atttaattta gggtatgagt 900
ttggtataaa ttgtggatta atagagatta tttgatgaag agattaatgg agagagaggg 960
agaggaggaa agggagtgag aaaatgatga atggagggag agggagggag agatatatat 1020
ttgagttagg attttggggt ttatttggtg ggaggtggtt tggattagag gtggtggtag 1080
tggtggtggt ggtgtttagt agagttttgt ggaagtggtg gtgatatttg ggttgggtag 1140
tgtttttgat gttttttagt gttagtgatt gtttttattt ttgttgttgt gggttgggaa 1200
agttttgtta gtgtatagta attaatgggt ggaggggttt tttgtttttg ggttgtgtta 1260
tttttatgtt tttagaattt ggaggattta gtaggattgt tttatttgta tttgtattga 1320
ggttattgat ggaaatggtg ggggatggtg ggggtattaa taagtaatgt ttttaaagtg 1380
agtgggaatt tgagggtagt ttgatgttat ggtgaatttt attttttagt ttggtttggt 1440
tttgaatttt tgttttattt tatttttggt tttatgattt ggtggaaagg gtttaggatt 1500
gtagggattt ttttaatttt gattttagat ttttgtttaa gtgagatttt aaaggttaat 1560
gtttttaagg gtgggttttg ggtttaagaa atttgatttt agtagttggt attatttata 1620
gagggttagg gtttgtggga aataagagat ggagttggga gtaagaattt tgttagatag 1680
gagtttaatt atagttggtt taatttttgg ttgtgtgatt atgggtaagt aattttatag 1740
aggtttagtg tatttatttt tgaaatgggg attataattt tattttgaag gaatattgtg 1800
agaaatagtt tagtgtttgg gttttagata ggtagggatt tataagagtt ttttggtttg 1860
tataggggat tttgtaagat ttttatggat attttttgaa tttttatttt tttggattgt 1920
tttggggtat tttttagatt tattatatgt gtgaggaggg ggttgatgat ttgttgggtt 1980
tgaggggtga gttgtaaggt atagatgtgg aagtttttat ttagattttt ggattttttt 2040
ttttttattt tgttatttat tatttgtttg agttgtttgt atagttgttt ttttgttttt 2100
ggttgttgtt tgttaagatg ttttagagat attttatttt ggttttgtat ttttattttt 2160
gtattttagt tttttattaa agagtaggta ggtgtgatag tgttgtgtgg tttttgttta 2220
gattaagttt atatttgttt ttggttatat ttatttatat atatttaaat ttattattgt 2280
tttttaatat gtgtttattt tgaattatag atattaaaaa atagatattg gttagaagag 2340
tattttgagg tgtttattta aatgtgtttt aagtttgttt ttgagttttt tgggtggttg 2400
tatttatata tttgtatgat gatgatttgt ttgtattaaa tttgtatata ttatgaatag 2460
gtgtttttta ttatatttaa ttatgtgtga ttttaatagt ttttatattt tgtagaattg 2520
ggttttagtt ttattgtgtt tgagtgttta gtttagtaga ggtagggatt aaggtgtttg 2580
ttgtttatag tgtttagagt ggtaagaaag aagtttttta gtgtttttat tattttttgg 2640
tggaatgtgt agtgtgtgat ttgttgtagt tggtttgggt gttgtttgtg tggtgagtgt 2700
agtagtggta ttttggggtt gtgggtggag gtaaggattt tagtttgagg ttttttgagg 2760
tagagtagaa attagttttt aggtgtgttg tttgtggtgt gagttatgga attttaggta 2820
ttttggggtt gtgtttgtat ttttgttttt agtttttaga agtgttgaaa ttttttagta 2880
tgatattttt gttttgtgtt atattttttt tttttagggg gttggtggtt tttgtgttta 2940
aaggaggtag tgggaatgtt taatttttaa gatgttttta attgattttt ttttttttaa 3000
ttatagtgag ggaataataa tttggggttg gggattgggt atttaagatt tgttttttgt 3060
agggttgttg gtagggtggt tataggattt gggattggaa ttggtttggg aggatattgg 3120
tgtgtgttag tgtagagtgt gaaataggta atgtgtgtgt agagtagggt ttggatggtg 3180
gattttgggt ataatgagtt ttggattttg aaggaagata atttttgttg agattttgtt 3240
tggagttttg gagtttttta gtagtttagg tttggatttt tggtttgggt ttttggtgta 3300
gagttgtttg aagagttgtg aaggttgtag tgtgggtatt gagtggggtg tagtgtttgg 3360
gtttttgtta ttggggttgt agtagaaggg tatttgtgtt tagttattat atattttagt 3420
ggaaatgatt tttttttaag attagaggtt tttaatataa gaattttttg aaattgtttg 3480

taagtgtgtg tgtttgtgat gtgtgttttt ttaaagagag agtttatagt ttttgtttgt 3540
gttatggtga ttgtaaaatg aaagttagta gatgtagatt ttgtggttat gttattttga 3600
ggggttggaa ttttattttt gattgtggtg tgtgttttta gtttttagaa tagatatgta 3660
ttttttatta ttaaattttt ggtagttata aattaaaggt tggtgttgtg gggtgttaat 3720
tagagtttgt gtttagaggg tgttagtgta gttttgagta gttttaagat tgattgtaaa 3780
agtttgtttt tttaggatag gagggatggt tattaattta ttttttaagt attttttgag 3840
tttgtaggaa ttgtttggtt ttttgttggg ggttgaaagt tttttttgtt tttgtttgaa 3900
aattattttt tttagtaatt agtattatgt taggtatttt agtagatgat ttgttaaagt 3960
taaatgaatg aaaatttatt ttggtaaatt tattagtttt ataaggtagt ttgtagaaaa 4020
tttggattta gttagagtgg tattttgttt tgttttttga aatttataaa ttatttagtt 4080
agattttttt tattttattt gttatagtaa gttggttttt aggtagaagt ttttaggaat 4140
gaggtaggga agtaaggatt ttgtttaggg ttttttttaa agattgagat tttttggtgt 4200
aaaatgtgaa gttatttttt gagtttattt tggatttatt gagtgagtga aggaagggta 4260
gagttttttg tgagtggatt atttagattt tattttgtta tttttggaat ttttttgagt 4320
tttagtatta aatagagtat ggtttaatgg aagttgttaa ggaaaatgtt gattttaaaa 4380
aaatagttgt ttgattattt gatttttgat ttttattgtt ttatttttta atttaaaaat 4440
ttggaaatta gggttagttt tgaaataatt tgattttgga aaaaaatatt tttataatat 4500
tttaataaag tttaattgta atgtttgttt ttatttgttt agattagatt tttgtatttt 4560
ttgtttaaga aataaaaata aatatgaagt ttggttgaaa atttttatgt gaatttttat 4620
gtgattaaaa aagttatatt tgaaggtttg agtttagagt ttaagttaaa tttattattg 4680
ttttataatt tttttataat atggatttaa ttataaaatt ttagtagtag taatagtatt 4740
attagtaata gtaatataat agtttataat atatatattt tttttagttt ggtagaaatg 4800
tggagttatt aaattaattg gaaaaaattt taattgaaaa ttgattgtat tgtttaaaga 4860
agggtagaaa ttaaagtttt ttattattgt gtttattgaa gaatatgtta agagaagtag 4920
ttgtttttga attatttttt taagatattt ttagattgta gtaaagttgt ttttggggaa 4980
gggagtatag aaatggttgt taaagagata gtgagattta taataattaa gatatagaaa 5040
tagtttaagt gtttattgat gaatgaatta ttaaagaaaa tgtggtgtgt gtttattata 5100
tatatataat ggaatattag ttagtagtat tatttagaag aaggaaatat tgttatttgt 5160
gttaatatgg ttgaatttgg ggaatattat gatatataaa ataagtgagg tatagaaaga 5220
tatgtaatat atgattttat ttatatgtag aattttaaaa aataagttga atttagtaat 5280
aaaggttaga atggtgatta tttggggttg tggttggggg taaaagagaa atattagtta 5340
aaggatataa ttttttagtt ataagatgag taagttttgg agatttaata tatagtttgg 5400
tgattatagt taatatatgt ttgaaatttg taaagagagt agattttaag agttttaagt 5460
gtatatatat atatatatat ataggaaatt atgtgaggta atgatatgtt aattaatttg 5520
attgttgtaa ttattttata aagtatatat gtattaaaat attatattgt atattttaaa 5580
aataaaaatt tttaatatgg aaataaaagt gaaaaaaaaa aagagtgaga gatagttgaa 5640
attttttagt gggaattttg ttgaagattt tagaagaggg gtatttgtat aaatattgaa 5700
gaggatatta atgttttatt aataatttag atatagaatt atttaaaaga tgttgtatgt 5760
gtagtaagta tgaaaagtta tataaattat attaaattga aataaagaaa agatattata 5820
aatattataa ttattattaa ttaagtttta tttgtgtgtt aggtattgtg tgtatattat 5880
tttttttatt tttaatagtt taataagata gatattttta tttttgttgt atatgtaagg 5940
gaatagtaag agtatttggt ttgaattaat tatatttttt aggttttttt tgaggatttg 6000
aaatatagtt tatttttttg ttatgttagg taagaggttt ggttttttgg atttaggtat 6060
tgatttagag gaagatatgg gtatgtttgt ttgttttagg gtta 6104

<210> 289
<211> 5976
<212> DNA
<213> Artificial Sequence

<220>
<223> chemically treated genomic DNA (Homo sapiens)

<400> 289

agaggggtat tttttatttt aatttgaatt ttatgattgt attttttgtt aggagatgaa 60
agggataaga ttgtttgaag gattaatttt tagtttttag gtaggttagt ggttaaaata 120
tttaggaagt gaaagtttgt tttttgttat aaggaaattt ggagtttgtg attttattat 180
gtggggtttg ttatttgtta ggtgataggg ttttttattt atttgtggtg atttgaaggg 240
taaggttttt ggttaaatat tttttttttt tagtgatgtt tgaaatttta ttgttagtgt 300
tggattttag taagatttta gagaaagttt aatttttgga gtttgggttt tttttagttg 360
agatgttagg aaagtaatgg gttttattat ttgtatggat aggtttgtgt atagttttag 420
gggatttagg gattttatta gagtttattt atagatatta ggttaaaagt tttggatagt 480

atttataatt tttttgaaag aagaaaagtt tttttttaga gtttttttta aagtgtaaat 540
ggttttggga ggtttgaaat gatgagttgg ttggtatgaa agaatttttt ttagtagatg 600
taagagataa tttgaggagt tttagtggtg ggggtggggg aaggagagtt tgggggaggg 660
gaggttttag gtagtttata atttttttta gaattggaaa gggaatgtat tgggttaaga 720
gggttaatta tttatttttg tagagtgttt ataagaattt tatgaattaa gtgttattaa 780
tatttttatt ttatagatga ggaaattaag atggtggaga taaaatgatt tatgtttttt 840
aattaatggt agagttagaa gtgaatttag gttggagtga agtggtttta gtgtttaaat 900
gattttttta ttttagtttt tagagtagtt gggattatag gtatgtattg ttatgtttag 960
ttaatttaaa ataatatttg ttggggagat ggggttttat tatgttgttt agattggttt 1020
taaatttttg ggtttaagta atttttttat tttttaatta gttttttaaa gtgttgtgat 1080
tatagttgtg agttattagg tttgtttagg tatttttgtt tgatataaga gaaaatagag 1140
ggtaggtata gtggtttata tttgtaattt tagtattttg ggaggttgag gtaggtggat 1200
tatttgaggt taggagtttg agattagttt ggttaatatg gtgaaatttt gtttttatta 1260
aaatataaaa attagttaga tatggtgatg tgtgtttgta attatagtta tttaggaggt 1320
tgaggtataa gaattttttg aatttgggag gtagagagtg tagtgagttg agattgtgtt 1380
attgtatttt agttggggta atagagtaag attttgttat taaataaata aataggaaat 1440
agagaagtaa tttagtgtgt tgtgaaagtt atatagtatt tgttttttat aaggagtttt 1500
tttgtgtttt tgttttattt tggatatttg atttattgtt tttggagttt ggagaaggat 1560
ttgttgagaa gtttgaagaa tgtattaggt ttgatttgat tttgttgttt ttaattgttt 1620
tttaaaattt tttttattga attattagag tgtaatggaa gagttgatga gagttgttgt 1680
agagttatga ggttaaggtt tgttgggtag aagaaagtaa agttttaaag gtttttaggg 1740
tttagtgatg gtataaaata tgtattttta tataattgag aggaaaaaaa taagtagata 1800
gtatagtttt atgggggaaa tgtttttttt tgtattttta ttattttgtt taaggtatga 1860
ttttagtggg aaaggaaata ttggagtttt tttttttgga gttttttggt gggttaagag 1920
tagagggagt taaaatatga tatttaaaag atttgattgg aaatttatta aaattgaggg 1980
gataggttgt tggggggggt ggtgttgggt tgtttagaaa ttgtttgtat ttgagaaatt 2040
atttgatgga tttagaggta tttttagttt tgggtgaaat attttaaata tggttttttt 2100
taggaggaag tgaaataggg gttgggtttt ttgaaagagt aatttttatt ttaaatattt 2160
attgagtagt tgttatgatg agttaagaat tttgtttagt gattgttttt gtattagttt 2220
atgttaattt atttatataa aggagaatat tgattttttt aaggttatag ggttagtgag 2280
tggttgattt tatttaatgt tgtgtagttt taattatgtt tgaaggataa agggaaagga 2340
ggtgtttttg ttggggtaga taagttattt agatgtggtt ggttataggg agttagatag 2400
ggaagttgtt taatttaata tttgtttgtt tattttgttt ttgttttttg atggaggatg 2460
gtgggtgttg gagggttatt ggtttaggat ttgagatttt tggtttttat ttattttatt 2520
tttgatttta aaattatttt attggggata gaattttttg gagaaggtga tggtggttat 2580
ggtgtggttg tggtattaaa agttttattt tgggggttag aagattttgg gttttggttt 2640
ttggttttga tattgatttg tttatgtgat attgaataag ttatttgtaa attataggtt 2700
tgggtgaatt ttaaaggttt tttttaggtt aatattaaaa aatagattga aaattgtagt 2760
tttagttgga agattgggat tatatgagat gaagttgatt gttagaagaa aggttttgga 2820
attagagagg ttggttttag ttttgtgtta ttttttattg gttttaattt ttttaagttt 2880
tagttgttta tttattatat gggaataata atatagtttt tgggttgttt tgaaatgagt 2940
ttaaatgagt tggttagtgg ttgggtatgg tggtttatgt ttgtaatttt agtattttgg 3000
gaggttgagg taggtggatt atttgaggat aggagtttaa gattagtttg gttaatatga 3060
tgaaattttg tttttattaa aaatataaaa attagttagt tgtggtggtg tatgtttgta 3120
attttagtta tttaggaggt tgatagagga gaattgtttg aatttaagag gtgaaggttg 3180
tagtgagtta agaatatatt attgtatttt agtttgggtg atagagtagg attttgtttt 3240
aaaaaaaaaa gagttggtta gtgttaaatg tttagtatag agattggtat agtaattttt 3300
aatgtttagt atttattgtt attatttttt tttttttttt ttttttgaga tagagttttg 3360
ttttgttgtt taggttggag tatagtggtg tgattttggt ttattgtaag ttttattttt 3420
taggtttatg ttattttttt gttttagttt tttgagtagt tgggattata ggtgtttatt 3480
attatgtttg gttaattttt tgtattttta gtagagatgg ggttttattg tgttagttag 3540
gatggtttta attttttgat tttgtgattt gtttgttttg gttttttaaa gtgttgggat 3600
tataggtgtg agttattatg tttggtttta ttgttattat ttttattttt tatttttgta 3660
tagagtattt atggtttaag aaatatttgt tattttaatt gtatgggagt tttataatag 3720
tatagggaga tatttttgat tattattttt attaggaggg tggagaaatt gaggttttgg 3780
gaggtggttt tgatttaggg aattaatttg ttgatttatt aatttatgaa gttttatagt 3840
taaaaaagat tagattaaaa aatgagaatt tagtaaaggg gttgaggtag gaggattgtt 3900
tgagtttaga aatttgagat tagttttggt aatatagtga gatttttttt ttagaaaaat 3960
tttttaaaaa attaggttgt ttgaggtaga gtgtagtggt ttatgtttgt aatttaatat 4020
tttaggaggt tgaagagggt ggattatttg aggttaggag ttttagatta gtttggttaa 4080
tatggtgaaa ttttgtttgt attaaaaata taaaattagt tggtgtggtg gtatatgttt 4140
gtagttttag ttatttaata ggttgagata ggagagtttt ttgaatttgg taggtggagg 4200
ttgtagtgag ttgagattgt gttattgtat tttagtttgg gtaagataga gtgagatttt 4260

gttttaaaaa atataaataa aataaataaa taaaaaatta ggttgttagt ttagtggttt 4320
atggtttata tttgaaattt tagtattttg ggaggttaag gtaggaggat tgttttagtt 4380
taggagtttg agattaggtt gggtaatata gggagatata gtgtttttat tgtttttgtt 4440
tgttttgatt tgttttttta taaaaaggta aaagaaaaaa aaattagttt ggtgtggtgg 4500
tgtgtatttg tatttttagt tattagagag gttggggtta gaggattgtt tgagtttagg 4560
agtttgaggt tgtagtgagt tgtgattgta ttattgtatt ttagtttggg tgaaagagtg 4620
agattttatt tttaaaatga ataaataaaa aattttaaaa aataaaagaa tttagttaag 4680
tgtaaaagtt tttttgattt ttaggtttta gtgagttatt ggtggggttg ggatttgaat 4740
ttagtggaat tagaattgtg taggttttat aatttattta gattttagta attttaggtt 4800
agagggttat tgtgtttatg tgaggttggg tgggtgggtt gttagttttg ttttggggag 4860
gggtttgtgt tgttgattgg ttgtggttgg taggtgaatt tttagttaat tagtggtatg 4920
gggggtggtg tttttggggt ttatttggtt gtagttatgt attttttttt agtggtgttg 4980
gaattgtaaa gtatttgtga gtttgtggaa gttagtttag attttagttt gttttagttt 5040
ggtttgattt gattgtattt ggtgtttgtt tttgtttggt gtttttggtt agttatgggt 5100
ttttggagtt gtagtttttt ggtgttgttg ttgttgttgt aggtattttg gattttttga 5160
tttgtgaggg atgtatttgg gttgtaagtt ttgtgtttta gttttgtgtt tttttttttt 5220
ttgttgttat tgtttttttt tttttaagaa agtttgggtt ttgaggagtg gagtggtttg 5280
gaagttttgt gtgttttgga ttttttagtg atgggagtgg ggggtgggtg gtgaggggtg 5340
agtgtggttt ttttgttttt tttagtgtag attgaggtgg gggtgtttgg ttgtggagtt 5400
tgtggggtgg gtttgtgtgg gtggtggggg tgtgaagtgg ggtgtagggg gtggggtgtg 5460
gagaaggggt gttttggtgt aagttgaggg ggagttagga gttgtgggga tgatttttga 5520
gggaaggaga ggggtatttg tagaaataaa ggtatttgtt atgttaagaa aggttgtaaa 5580
taggagtgag ggttttgggg ataagaaagt gaggttggag gaggtgggag tgttttttgt 5640
tttgaggagt ggtgtatttt tggtttaagg aaagtggggt attggagaat aaagatattt 5700
ttaataaaat gagaaaggag attgaaaggg aatggtgggt taggttttga gggggtgatt 5760
tggtggtttt tttttgggag tttttggggg tttggtggtt gtaggttttg gggtggggga 5820
gggtgatgtt gttgtttgtt tgttttgggg ttgtgggttg gggttttttt ttaattttga 5880
tgttgggagt gagggagggg tggtgttgtt ggttttggtg agtaggaggg aattttttga 5940
gttatttggt tttatttatt ttttttttat tttagg 5976

<210> 290
<211> 5976
<212> DNA
<213> Artificial Sequence

<220>
<223> chemically treated genomic DNA (Homo sapiens)

<400> 290
   tttggggtgg gggaaaggta gatggaattg ggtgatttgg agggtttttt tttgtttatt 60
   gaaattaata gtgttgtttt ttttttgttt ttggtgttgg gattggggga ggattttagt 120
   ttgtagtttt gggatgggtg ggtagtgatg ttattttttt ttattttgaa atttatggtt 180
   gttgagtttt taggaatttt tgggaggggg ttgttgagtt atttttttaa gatttagttt 240
   attgtttttt tttagttttt ttttttattt tattggagat gtttttattt tttagtattt 300
   tatttttttt agattgggaa tgtattattt tttagagtga ggggtgtttt tatttttttt 360
   gattttattt ttttattttt gggattttta tttttattta tgattttttt tggtatggta 420
   ggtgttttta tttttatgga tgtttttttt tttttttgga agattgtttt tatgattttt 480
   ggtttttttt tgatttgtat tagggtattt tttttttata ttttattttt tatattttgt 540
   tttatgtttt tattgtttgt gtgagtttat tttgtggatt ttgtggttag atgtttttgt 600
   tttggtttgt gttggagggg gtaggaaagt tgtgtttgtt ttttattatt tattttttat 660
   ttttattatt ggggggtttg gagtgtgtga ggtttttagg ttgttttgtt ttttaggatt 720
   tgaatttttt tggaagaagg gaagtggtga tgatgggaga ggaaggggtg tagggttggg 780
   gtgtggagtt tgtggtttga atgtgttttt tgtaagttag gggatttggg gtatttgtag 840
   tagtagtagt agtgttgaga ggttgtggtt ttaagggttt atggttggtt ggggatgttg 900
   agtgagggta ggtgttgggt gtggttgggt tgggttgggt tggagtgggt tggagtttga 960
   attgattttt gtaagtttat aggtgttttg tagttttgat gttattgaga gggggtgtgt 1020
   ggttgtagtt aggtgagttt tggaggtatt gttttttgta ttgttgattg gttgagggtt 1080
   tatttgttgg ttatagttaa ttagtagtgt ggattttttt ttagggtgga gttgatggtt 1140
   tgtttatttg gttttgtata gatgtggtga ttttttagtt tggagttgtt agggtttagg 1200
   tgggttatgg gatttgtatg gttttgattt tattgggttt gaattttagt tttgttggtg 1260
   gtttattaag atttgggatt agaaagggtt tttatatttg gttgagtttt tttgtttttt 1320
   gggatttttt gtttgtttgt tttggagatg gggttttatt tttttattta agttggagtg 1380
   tagtggtgtg attatagttt attgtagttt tgaatttttg ggtttaagtg gttttttggt 1440
   tttagttttt ttagtagttg ggagtatagg tgtatattat tatgttaggt taattttttt 1500
   tttttttgtt tttttgtaga gagataagtt ggggtggata ggggtagtgg gggtgttgtg 1560
   tttttttgta ttgtttagtt tggttttgaa tttttgggtt gaagtgattt ttttgttttg 1620
   gttttttaaa gtgttaggat tttaggtgtg agttatgagt tattgagtta gtagtttaat 1680
   tttttgtttg tttgttttgt ttgtattttt tgagatggag ttttgttttg ttttgtttag 1740
   gttggagtgt agtggtatga ttttggttta ttgtaatttt tgtttgttgg gtttaagaga 1800
   tttttttgtt ttagtttatt gagtagttgg gattataggt gtgtgttatt atattggtta 1860
   attttgtatt tttagtatag atggggtttt attatgttgg ttaggttggt ttggaatttt 1920
   tgattttagg tgatttattt tttttagttt tttgaagtgt tggattatag gtgtgagtta 1980
   ttgtattttg ttttgagtgg tttaattttt taaaaaattt ttttagagag gggattttat 2040
   tatgttgttg aggttgattt taaatttttg aatttaggtg atttttttgt tttagttttt 2100
   ttattgagtt tttatttttt aatttagttt tttttaattg tagagtttta tgggttagtg 2160
   agttagtaaa ttgatttttt aggttaggat tattttttaa agttttagtt tttttatttt 2220
   tttaatggga ataatgatta gaaatgtttt tttatgttgt tgtgggattt ttatataatt 2280
   aaaatgataa atgttttttg agttataaat gttttgtata gaagtgaggg gtaaaaatag 2340
   taataatagg gttaggtatg gtggtttatg tttgtaattt tagtattttg ggaggttgag 2400
   gtgggtagat tatgaggtta ggagattgag attattttgg ttaatgtagt gaaattttgt 2460
   ttttattaaa aatataaaaa attagttagg tgtggtggtg ggtgtttgta gttttagtta 2520
   tttgggaggt tgaggtagga gaatggtatg aatttgggag gtggagtttg tagtgagttg 2580
   agattgtgtt attgtatttt agtttgggtg atagagtaag attttgtttt aaaaaaaaaa 2640
   aaaaaaaaaa aatagtaata ataggtgttg gatattgaag attattgtgt tagtttttgt 2700
   gttaagtatt tgatattgat tagttttttt ttttttgaga tggagttttg ttttgttgtt 2760
   taggttggag tgtaatggtg tgtttttggt ttattgtaat ttttgttttt tgggtttaag 2820
   tggttttttt ttattagttt tttgagtagt tgggattata ggtatgtgtt attatgattg 2880
   gttaattttt gtatttttag tagagatagg gttttattat gttggttagg ttggttttga 2940
   atttttgttt ttagatgatt tatttgtttt agttttttaa agtgttggga ttataggtat 3000
   gagttattgt gtttagttat tgattagttt atttaagttt attttaaaat aatttaagag 3060
   ttgtattatt atttttatat gatagatggg taattgaagt ttagagagat tgaggttaat 3120
   gagaggtggt gtaggattgg aattaatttt tttgatttta gggttttttt tttaataatt 3180
   agttttattt tatgtggttt tagtttttta gttggagttg tagtttttag tttgtttttt 3240
   gatgttgatt tggaagagat ttttagagtt tatttaaatt tatgatttgt aaataatttg 3300
   tttaatgtta tataggtagg ttaatgttaa agttaaagat taaaatttag gattttttga 3360
   tttttagagt ggggttttta atgttatagt tatattatgg ttattattat tttttttaga 3420
   gaattttgtt tttagtgaaa taattttagg attaaaaata gaatagatag gggttaaagg 3480
   ttttaggttt tgggttagtg gttttttagt atttgttgtt ttttattaaa agataaaaat 3540
   agaatagata ggtaaatgtt aggttagata atttttttgt ttgatttttt gtggttagtt 3600
   atgtttgggt ggtttgttta ttttgataag ggtatttttt ttttttttat tttttaagta 3660
   tggttagaat tatatagtat taagtggagt tagttattta ttagttttgt gattttgggg 3720
   aagttagtgt ttttttttgt ataagtgaat tggtatgagt taatataaaa ataattgtta 3780
   agtagagttt ttggtttatt atagtaattg tttaataaat atttggagta ggggttattt 3840
   ttttaaaagg tttagttttt attttgtttt tttttgggga aagttatgtt tggaatattt 3900
   tatttagagt taaaggtgtt tttgagttta ttaagtaatt ttttaaatgt agatgatttt 3960
   tgggtagttt agtattgttt tttttaataa tttgtttttt taattttaat gagtttttaa 4020
   ttaggttttt tggatgttgt attttggttt tttttgtttt taatttatta ggaggtttag 4080
   agggagaagg ttttggtatt ttttttttta ttaggattgt gttttgaata agataatgga 4140
   aatatagaaa gaggtgtttt ttttatgggg ttgtattgtt tgtttatttt ttttttttta 4200
   gttatgtaaa aatatatatt ttatattatt gttgggtttt gggagttttt agggttttat 4260
   ttttttttat ttagtaagtt ttggttttgt aattttgtag tgatttttat tgattttttt 4320
   attatatttt gataatttaa taaaaaggat tttagaaaat aattaggggt agtaaagtta 4380
   ggttaggttt gatgtatttt ttagattttt tggtaagttt ttttttaagt tttaaagata 4440
   gtagattagg tgtttagggt gaggtgggaa tatagagggg ttttttgtgg aagataggtg 4500
   ttgtgtgatt tttgtaatat attgaattgt tttttgtttt tttatttatt tatttagtga 4560
   taaggttttg ttttgttgtt ttggttggag tgtaatggtg tgattttggt ttattgtatt 4620
   ttttgttttt taggtttaag agatttttgt gttttagttt tttgagtagt tgtgattata 4680
   gatatatgtt attatgtttg gttaattttt gtattttagt agagatggga ttttgttatg 4740
   ttggttaggt tggttttaaa tttttgattt taagtgattt atttatttta gttttttaaa 4800
   gtgttgggat tataggtgtg agttattgtg tttgtttttt gttttttttt gtattaaata 4860
   gggatattta agtaggtttg gtggtttata attgtaattg tagtattttg ggaggttagt 4920
   tgggaggtga gaagattatt tgagtttagg agtttgagat tagtttgggt aatatggtga 4980
   aattttattt ttttaataaa tattattttg aattagttgg atatggtagt gtatgtttgt 5040
   agttttagtt attttggagg ttgaggtagg aggattattt gagtattgga gttattttat 5100
   tttagtttgg atttattttt ggttttgtta ttagttgaga agtataagtt attttatttt 5160
   tattattttg gtttttttat ttgtaaaatg ggaatgttga tagtatttaa tttataaggt 5220
   ttttgtaaat attttgtaaa ggtgagtgat tagttttttt ggtttaatat attttttttt 5280
   tagttttagg aaaaattgtg agttgtttgg agtttttttt tttttaggtt tttttttttt 5340
   tatttttgtt attaaagttt tttaggttat tttttgtatt tattggagaa aattttttta 5400
   tgttggttag tttattattt tgaatttttt agggttattt gtattttaaa gagggtttta 5460
   gagaaagatt tttttttttt taaaggagtt gtaaatgttg tttagggttt ttaatttggt 5520
   gtttgtggat gggttttggt ggggtttttg aattttttga gattgtatat aaatttgttt 5580
   gtgtaggtga taaggtttat tgttttttta atattttaat tggggaaaat ttaaatttta 5640
   ggagttgagt tttttttgag attttgttga gatttagtgt taatagtgga attttaggta 5700
   ttattgggga aaagaaatgt ttggttagag gttttgtttt ttagattatt gtagatagat 5760
   aagagatttt gttatttggt agataataga ttttgtatga tgggattata gattttaggt 5820
   ttttttatgg tagaggatag atttttattt tttgggtgtt ttggttattg gtttgtttgg 5880
   aggttaaaga ttaatttttt aggtagtttt gtttttttta ttttttggta aggggtgtag 5940
   ttatagggtt tagattgggg tgggaggtgt tttttt 5976

<210> 291
<211> 2501
<212> DNA
<213> Artificial Sequence

<220>
<223> chemically treated genomic DNA (Homo sapiens)

<400> 291

tagaagtttt tttttagagt gtgtttgggt atatatttaa gtgtatggtt gtaaattttt 60
ttttttaaag tattgaatag tattagatat ttagtaggta tttaagaaat attgaatgtt 120
gtggtggtgg tgagttagaa gttataaaaa aaattttttt ttaaaaataa taataaaaag 180
aattatttta ttgtgaagtt tagtattata aaaatttaaa taatttatta taagttttta 240
ttaaaaaaaa tttttttttt aaagtaaata gatagataat gtttagttta tttgaaatgt 300
ttgaaagtag aggggtttta aggtagtggg agaaggtgtt tgttttttgt tggatatttg 360
ataattagtt ttttggatgg tttggatgta taggagtgaa ggtgtagata gtagtggggt 420
ttagagtggg gttttgaggt tgtgttgtgg tttttttggg gtttagttat aattttggtt 480
tgattttagg gtgaggtagg ttaagggggt ttgttattgt gtttttttat ttttatttgg 540
gtttttattt ttatagtaga ggagaaagaa gtttgttttt tttgaggtta gttgtgttag 600
aggaagaaga ttgggtatgt ttgggtagag atttttagat tttgagtagt ttgagatgtt 660
agtaattgta gttgttttaa gtttgggttt tgttttttag tgggattttt gtttagatga 720
ataatttatt ttttgtaatt ttttaaaagt aaaattgtaa atgttttagg tatagaaagg 780
aggtaaaggt gaagtttagg ggaggttagg ggtgtgaggt agatgggagt ggatagatat 840
attatttatt tttgtgtttg ttagaagaat tagtagatat ttttagaatt gttttttatt 900
tatgttattt ttataaatta tttgtaaatg agggttattt ggtatttttg ttattttgga 960
gttatagaaa taaaggatga taagtagaga gttttgggta ggaggtaaaa gttttgtgtt 1020
ttaattatag ttattttttt gttgtatgat ttgagttagg ttattagatt tttttgagtt 1080
ttagtttttt tagtagtgta tatgggttat gtggggagta tttaggagat agataattta 1140
tttgttaaat tttttttttt ttggttaata aagttgttgt aattataggg attttttttg 1200
tttaggtgag tgtagggtgt agggagattg gtttaatgtt taattttttt gtttttttgg 1260
agattaggtt gttttttttt ggtagttttt ttaatttttt tttttttgga agtatgtgat 1320
aattaataat tttgtatatt taagtttagt ggattttaat ttttttattt gtgaaataaa 1380
tgggattgaa aaattatttt ggttttaaga tgttttgttg gggtgtttag gtgttttagg 1440
tgtttttggg agaggtgatt tagtgaggga ttagtgggaa tagaggtgat attgtggggt 1500
ttttttggaa attgtagaga ggtgtattgt ttttataatt tatgaatttt tatgtattaa 1560
tgttattttt ttgatttttt tagttgtatt gggtaaattt ttgtttgtta gagtgggtta 1620
gtggtgagtt agaaaggggg tttattttaa tagtgttgtg tttttttgga gagtgttaat 1680
ttatttttta agtaaaaaaa gttagatttg tggtttattt tgtggggaaa tgtgtttagt 1740
gtattaatgt aggtgaggga ttgggggagg agggaagtgt ttttttgtag tatgtgaggt 1800
tttgggattg gttggtttgt tggaatttgg ttaggtttag ttggtttggt gttgggtagt 1860
taggagtttg ggttttgggg agggtggttt tgggtggtgt ggtgggttga gtgtgggttt 1920
tgtttttttg aggtgggttt gggtggggtg gttgtatatt agggttgtgt tgagttgtgt 1980
tagttgaggt gtgagtagtt gttgaagtta gttttttgtg gagttggagt tgggtgtgga 2040
tttgttgagg tattgaggta tttagaggag gtgagagagt ggtggtagat aataggggat 2100
tttgggttgg tggtttagag ttgagttaag tgtgtttgtg tgtgtttttg tgtgtttgtg 2160
aggatgtgtg tttgtgggtg tgtgttgtgt ttataggtgt ttttgtggta ggtgaatgat 2220
gggtgtgggt tggtgtgtgt ttggtttgtg tatatggtgt ttttaagtgt gtgggtgatg 2280

agagttggga tgtgttggag attttggggt ggagagtggg attataagta taggaatttt 2340
tggttatgtt ttttgttttt ggaaatttag ttggggtgag ggagggtgtg gatgggattg 2400
ttttgggagt ttgtttttgg ttgtggttgg ttttaggttt taggtgtagt ttgtttgtgg 2460
tgtggggatg aagtttgtgt ttttggaggg gtttaggaag g 2501

<210> 292
<211> 2501
<212> DNA
<213> Artificial Sequence

<220>
<223> chemically treated genomic DNA (Homo sapiens)

<400> 292

tttttttggg tttttttagg gatatggatt ttatttttat gttgtgagta aattgtgttt 60
ggggtttgga gttaattgta gttaaaggtg agtttttaga atggttttgt ttatgttttt 120
ttttgtttta gttgggtttt taggggtggg gagtgtgatt agggattttt gtatttgtaa 180
ttttgttttt tgttttgggg tttttggtat attttgattt ttgttatttg tgtatttaga 240
gatattgtgt gtgtaagttg agtgtgtatt gatttatgtt tgttatttat ttgttgtaga 300
aatatttgtg aatgtagtat atatttgtga atatgtattt ttgtggatat gtagggatat 360
atgtgggtat gtttggtttg gttttgggtt gttggtttgg ggttttttgt tgtttgttgt 420
tgttttttta ttttttttga gtgttttggt gttttggtga atttgtgttt agttttggtt 480
ttataaggaa ttgattttgg tagttgttta tattttagtt ggtgtagttt agtgtggttt 540
tgatatataa ttgttttgtt tgggtttgtt ttaaggaggt gggatttgtg tttggtttat 600
tgtgttgttt gggattgttt tttttggggt ttaggttttt ggttgtttag tgttgagtta 660
gttgagtttg gttgagtttt agtaggttag ttggttttgg aattttgtgt gttgtaggag 720
ggtatttttt ttttttttta gttttttgtt tgtgttggtg tgttggatat atttttttat 780
gaagtgagtt ataaatttgg ttttttttat ttggagaatg agttggtatt ttttaggagg 840
atatagtatt gttagaatga gttttttttt tggtttattg ttgatttatt ttggtaggta 900
aggatttatt taatgtagtt gaaaagatta ggaggatgat attaatatat aaaaatttat 960
aaattataaa aatgatgtat ttttttgtaa tttttagaaa agttttataa tattattttt 1020
atttttattg attttttatt aggttatttt ttttagaagt atttggagta tttagatatt 1080
ttaataaagt attttgaggt tagaatgatt ttttagtttt gtttatttta tagatgagga 1140
aattgaggtt tattgaattt aagtatataa agttgttgat tgttatatgt ttttgggaag 1200
gagggaattg gagagattat taaaaaaggg taatttgatt tttagggaaa tagaagaatt 1260
ggatattgaa ttaatttttt tatattttat atttatttga atagaagaaa tttttgtggt 1320
tgtagtagtt ttgttggtta ggaaggggag gatttgatga gtgagttgtt tgttttttga 1380
atatttttta tatagtttgt atatattgtt ggggaaattg gggtttagag aagtttggtg 1440
atttaattta gattatgtag taaagaaatg attatagttg gaatatagga tttttgtttt 1500
ttgtttgggg ttttttgttt gttatttttt atttttgtgg ttttaaaatg ataaaaatgt 1560
taaataattt ttatttgtag atggtttatg gagatgatat aaataaagga taattttgga 1620
agtgtttatt ggtttttttg atagatatag aaatgagtga tgtgtttatt tgtttttatt 1680
tattttatat ttttgatttt ttttggattt tatttttgtt ttttttttgt gtttgaaata 1740
tttgtagttt tgtttttaaa aaattgtaga ggatggattg tttatttgaa tagaaatttt 1800
attaaaaaat agaatttagg tttggagtag ttataattat tgatatttta ggttgtttag 1860
agtttggaaa tttttgttta gatatgttta gttttttttt tttaatgtag ttgattttgg 1920
ggaggatagg tttttttttt ttttgttgtg gggatgggag tttaggtagg ggtgggagga 1980
tatagtagta gatttttttg gtttgttttg ttttggagtt aggttaggat tgtggttaaa 2040
ttttagaaag gttatggtat agttttagga ttttatttta agttttattg ttgtttgtat 2100
ttttgttttt atatatttaa attatttaaa gggttggttg ttaaatgttt agtagaggat 2160
aggtattttt ttttattgtt ttgaagtttt tttgttttta ggtattttaa atagattaga 2220
tattgtttgt ttgtttattt tggggagaaa atttttttta ataaaggttt gtaatgaatt 2280
atttaaattt ttgtggtatt gagttttata atgaaataat tttttttgtt gttgtttttg 2340
ggaaagaatt ttttttataa tttttagttt attattatta tgatatttaa tattttttaa 2400
gtatttatta agtgtttagt attatttagt gttttaaaaa aaaaagtttg taattatgta 2460
tttgaatgtg tatttagata tattttaagg gaggattttt g 2501

<210> 293
<211> 3486
<212> DNA
<213> Artificial Sequence

<220>
<223> chemically treated genomic DNA (Homo sapiens)

<400> 293

aattaaagag gatgagattt tttgtttgat tttaggtgaa tttttttttg ttagttaggt 60
aggaagtttt gatttgaaaa ttagttttgg gtattgtttt ttttattgtt ttttgggtat 120
taatttttgt tttttaattt tagttgaatt agtggatggt gatattatag gtttaagata 180
gttgtattta aatattagtg attataggtt atattaagga aatatttgta ggtaatttag 240
ggtttgggaa ggagttattt ttagttattt gtaagatagt aggatttgta gattatagta 300
taattaaatt tagataaaat tttgaattag tgagaattat taggaaggaa aggaatagaa 360
aatgaattaa tttgagtgtt aggagatttg tatttagttt tgattttggt attaattgaa 420
tgtatgtttt tggataggaa atttttttga gttttgattt tttttgtggt aaaaaggaga 480
gggttaaatt atagggtttt gagggttttt tttagttgtt atattttgga gtgtatgaga 540
tgaggtaggt atataaagtg gataagatgt ggttaagaaa ataagttata tattaagttt 600
atttgtagta taggtgttta agaaaatttt gttgttgtgt aatattagaa tggaaggttg 660
gtttttagta aaatgtatta attttggttt aaattaagat gatgggtatt gggtatgggg 720
gtggggaggt agttgaagat ttattgagtt ttgttttagg gtagttttgt ttattgtttt 780
attttatttt ttattatggt gtttaagttt atattgagag agaaattttt agttgtaaaa 840
gggagaagag aaatgttgga atattagtat tggatgttag gatatggttg tggtgtttta 900
aaaattattt tattatttgg agtttgattt tgaggggagt atttttattt tttagttttt 960
tgaaagtatt tattagtatt tgaatattgt tttgagtttg ttggagtagt gaaatttggt 1020
gagagagaag ggtggaggaa ggaaggagtt gttgtatttg gtggttggat ttaggtagag 1080
gaaattgtta taattttggg aaagaataga aaagtagaaa gggatgagtt tttatatgta 1140
gttaatgttt atggttttaa ttgtgtttgg gaaggaagat tttgggttag gggtgtattt 1200
ttgtttttta aaattaaatg tgtttgagat agttataaag tttattaagg gatttgagag 1260
attagagttt ttttgttttt ttttttaatt ttgagttttt tttttatttt tattgaggga 1320
gagtttgagt ttatgataag tgttgtgttt atttttggtt aatttttaaa agaaagatgt 1380
ttgttttggt ttttttttta ggtttttagt ttttttaggg atggtagaaa tttttgggtt 1440
aaggttgagt gaattattgt ttattgtttt tattagtttt tagtaaaggt atgttggtgg 1500
gggggtgttt agttttttta gtaaatgttt tgtggttttt tttgtagatt atgaggtggg 1560
ggttgttggg gagggttgag ttgggggtag tttgttattt tggtttttag tgagttgttg 1620
gtgattttgg tggtttttgg gtttaggttt tggtttttgg ggtgaggagt gggagggagg 1680
ttggggttta ggtgttgtgg tgaatttgtt aatgtagtgt tgggttttga attttaggtt 1740
ttgttttagg tttttggttg tttggttagt ttgtttgttt taattttaat ttttttgagg 1800
ttagttagag taggtttgtt ggtagtagta tttttttagt agttatgtga ttagttaatt 1860
ttttggtggt gtttggggag gtggtgtgtt tgggaatgag gggaggtggt ggaattgtgt 1920
tggggttatt ttaaggttgt gtttgttagt tttggtgggg tggtttttgt tgttgtaatt 1980
aatggatttt tttttttgtt taaatagatt tgttgtgtta attatttttt tttttgttag 2040
tttttttttt attgttatat tgggttatta aaaaaagggg gtttgttttt ttggggtgtt 2100
tttttttttt tttttgtttt ttgtttgttt atggttttgt gattttgatg ttggtaaggt 2160
ttggagagtg gttgggtttg tgggatttgt gggtttgtat ttgtttagat ttggatgggt 2220
tttgttattt tttttgtttg tttggttttt tttttttttt gtttttttgt ttgttagttt 2280
atttgattag tggagatttg gtggttgggt tggggttttt ttgtagtttt tgtgtgtttt 2340
tagagtttgg gttgtggttt gttggggttt gtgttttttg gttttgaggg tagttgttgg 2400
gtttttgaga ggggtttggg ttgtgtaggg gtgttttgtt ttgtttggtt ttgttttttt 2460
gagagtgtga gagaggtggt tgtgtagatt tgggagaaag atgttaaatg tgtgagtgtt 2520
taatgggagt tttagtttgg agtggatgga tgttaggtag gtggagtatt ttaagttttt 2580
ggtttgtagg aatttttttg gtttggtgga ttatgaagag ttaatttggg atttggagaa 2640
gtattgtaga gatatggaag aggtgagtta gtgtaagtgg aattttgatt tttagaatta 2700
taaattttta gagggtaagt atgagtggta agaggtggag aagggtagtt tgtttgagtt 2760
ttattataga tttttgtggt tttttaaagg tgtttgtaag gtgttggtgt aggagagtta 2820
ggatgttagt gggagttgtt tggtggtgtt tttaattggg gttttggtta attttgagga 2880
tatgtatttg gtggatttaa agattgattt gttggatagt tagatggggt tagtggagta 2940
atgtgtagga ataaggaagt gatttgtaat tgatggtaat gatttttttt taattataga 3000
atgtgtttgg ggttttgttt tgtttgttgg agggtgttaa ttttagtttg ttttttggtg 3060
tattttgatt tagttttggg agagttaatt ttattggttt taggtgttta gtgttatttg 3120
gtttattgtt tgtttgtttg tgatttttaa gttagaaatt ggagatggta agatttgata 3180
atttttttaa tttaatatat tgtggttttt tttattagta atttttaggt atgtgataaa 3240
gttgggatgt ttattaatgg tttgtttttt ggttagggaa agagttttgg ggtggagaat 3300
gtattttttg ttttttgaaa ataattttat tttgtgtttt taaaagttat tggggatgat 3360
ggatttagga ttgtgggtgg aggtagtggg ttttttattt tttgattatg gggttaattt 3420
ttgttagtta ttgttttttt taataaagat tgtgtgtttt ttttaaaaat tttttttgtg 3480

tttaga                                                              3486

<210> 294
<211> 3486
<212> DNA
<213> Artificial Sequence

<220>
<223> chemically treated genomic DNA (Homo sapiens)

<400> 294

tttaagtgta ggggaaattt ttaaaaagaa tatataattt ttattagaaa aaataatggt      60
tggtagaagt tggttttata gttaggggat gaaaaattta ttatttttat ttataatttt     120
ggatttgtta tttttagtgg tttttaaggg tataaaatga ggttgttttt aaaaaataga     180
aagtgtattt tttgttttag agtttttttt ttagttagga ggtggattgt tgataaatat     240
tttaatttgg ttatatattt aggagttgtt agtgagaggg attgtgatgt attaagttag     300
ggaaattatt ggattttatt atttttagtt tttgatttaa aagttataaa tagataagta     360
gtgggttagg tagtattgaa tatttaagat taataaagtt agttttttta aagttaaatt     420
agaatatgtt gaaaagtaag ttaaggttaa tattttttag taggtaaagt ggggttttaa     480
atatatttta tggttgggaa agggttatta ttgttggttg taggttgttt ttttattttt     540
gtgtattgtt ttgttaattt tgtttggttg tttgatggat tagtttttgg gtttattaaa     600
tgtgtgtttt tagagttagt tggagtttta attaaaggtg ttgttgggtg gtttttgttg     660
atattttggt ttttttgtgt tggtattttg taggtatttt tggggggttg tgggggtttg     720
tagtagaatt tgggtaagtt gttttttttt attttttgtt atttgtattt gttttttagg     780
ggtttgtgat tttgaaaatt gaaattttat ttgtgttggt ttgttttttt tatgtttttg     840
tagtgttttt ttaagttttg ggttaatttt ttgtggttta ttgggttgaa gaggtttttg     900
taggttgagg gtttggggtg ttttgtttgt ttggtgttta tttgttttag gttagggttt     960
ttgttagata tttgtatgtt tgatattttt tttttgggtt tgtatgattg ttttttttgt    1020
atttttaaaa aaataaaatt gaataaaata aagtgttttt atgtagtttg aatttttttt    1080
ggaagtttag tgattgtttt tggagttaaa agatatagat tttgatgagt tatggtttga    1140
gttttaggag tgtgtagggg ttgtggggaa gttttggttt ggttgttgag tttttgttga    1200
ttaaatggat tggtgagtgg gagggtggag aggagagggg attaggtaag tggagagggt    1260
ggtaaagttt gtttgagttt gggtgggtgt aagtttgtgg gttttgtgaa tttagttgtt    1320
ttttaaattt tgttggtgtt ggagttgtag agttgtgagt aagtggggat aggggagggg    1380
gagaaaaata ttttgaaaag atgagttttt tttttttagt ggtttaatat ggtggtggaa    1440
gggaggttga tgaagaagaa aatgattgat atggtgagtt tatttaaata gaggaggaga    1500
tttattggtt gtggtggtgg gagttgtttt gttgaggttg gtgagtgtgg ttttaaggtg    1560
gttttggtgt ggttttgtta tttttttttg tttttgagtg tgttgttttt ttgagtgttg    1620
ttgggagatt ggttggttgt gtgattgttg gaggggtatt gttgttaata aatttgtttt    1680
ggttggtttt ggagaaatta aaattaagat aaataaatta gttaaatggt tgggaatttg    1740
gggtggggtt tgaggtttgg ggtttggtgt tgtgttggtg ggtttgttgt ggtgtttaag    1800
ttttgatttt ttttttgttt tttgtttggg aagttgggat ttggattaga ggattgtgaa    1860
ggttgttggt agtttgttag gagttggggt ggtgagttgt ttttagtttg gtttttttta    1920
gtggttttta ttttgtggtt tgtgggggag gttgtggagt gtttgttggg ggggttgggt    1980
gtttttttgt tggtgtgttt ttgttggggg ttggtggagg tagtgggtaa tggtttgttt    2040
agttttaatt tagaagtttt tgttattttt ggggaggttg ggggtttagg gaagaagtta    2100
aagtgaatgt ttttttttta gaaattagtt aggagtagat gtggtattta ttatgaattt    2160
aagttttttt ttaatgaaaa taagaaagga atttaagatt aaaaaaaaaa aataaaaaat    2220
tttagttttt taagtttttt aataaatttt gtagttgttt tagatatgtt tagttttgaa    2280
aaatgagggt atatttttgg tttaggattt ttttttttaa gtatagttaa ggttatggat    2340
attggttgtg tgtgggaatt tgtttttttt tatttttttg tttttttttg ggattgtagt    2400
agtttttttt atttgagttt agttgttaaa tataatagtt tttttttttt tttatttttt    2460
ttttttatta gattttattg ttttaataaa tttagaataa tatttagatg ttagtgaatg    2520
tttttagagg gttgaagggt gaaaatattt tttttggggt taaattttag atgatgaaat    2580
gatttttaaa atattataat tatgttttaa tgtttgatat tagtatttta gtgttttttt    2640
tttttttttt gtagttggaa attttttttt tagtgtgggt ttgagtattg tggtggaagg    2700
taaagtagga tgatgagtag ggttgttttg agataaagtt tagtggattt ttaattgttt    2760
ttttattttt atgtttggta tttattattt tggtttgagt taaagttaat gtattttatt    2820
ggaaattaat tttttgtttt aatattatat agtagtaaag tttttttaag tatttatgtt    2880
atagatgagt ttgatgtgta gtttgttttt ttagttatat tttgtttatt ttgtgtgttt    2940
attttatttt atatgtttta gaatgtgata gttggaaggg atttttggga ttttgtggtt    3000
taattttttt ttttttatta tggaggaaat tgagatttag agaggttttt tgtttaggga    3060

tatgtatttg gttggtattg gagttaggat tagatgtaag ttttttaata tttaggttgg 3120
tttatttttt gttttttttt tttttaatgg tttttattgg tttagggttt tgtttgagtt 3180
tgattgtgtt gtagtttgta ggttttgttg ttttataagt gattgaaaat ggtttttttt 3240
taggttttgg gttgtttgta gatgtttttt tgatgtggtt tgtggttatt gatatttaaa 3300
tgtagttgtt ttgagtttgt ggtattatta tttattagtt taattaaaat tgaaggatag 3360
gggttgatat ttagagaata gtaaaggggg tagtgtttag aattagtttt taagttagga 3420
ttttttattt aattgataga aaagaattta tttaggatta agtaagaggt tttatttttt 3480
ttagtt 3486

<210> 295
<211> 8666
<212> DNA
<213> Artificial Sequence

<220>
<223> chemically treated genomic DNA (Homo sapiens)

<400> 295

ttgtagggtt ttttttagtt taaagaaagt aattaggtgt ttttattgtg ttatgatttt 60
tatttttagt tgggtttttt agtttggttt taaagaataa aataaaaggt ttttttgttt 120
gagttttatt taatttattt tttttattta taagaggtat tttaaatttt tatgattttt 180
aaatttttta attgattatt ttatttttat tttgtaaata agttaatttt tttatttata 240
gaaggaagtg ttttaatttt ttttttgttt tgattttttt ttttttttaa atttatgtat 300
tttttgtgat aaaatttata attattgttg tattttgagt tttatttttt tattattttt 360
gagggatttt aagtttttaa aaatatttta ttttgtttgt gtatttaatt ttttttttat 420
ttttttttaa tttttttttt tttttatttg gtatttgttt ttttaggtat atgtgtgttt 480
aggttttttt tattttttat ttgttttatt ttatggtata gggttttgaa ttattataat 540
taagttatga aagagtagtt aatgtagtgt ttttattttt ttgttatttt attattttag 600
tttttttttt ggttttttga ggagtttttt ataggttggt ttttaggaat aagtttaaat 660
gaattatttt tagttttttt aaatttttat gtttttgtat atttttttat ttttgtttag 720
aatatttttt tttttttttt ttatttttaa atttttatat tattttttaa gattgggatt 780
agtttttagt atttggaagt ttttgtttat tagagataaa tgagaatgag tttggttatt 840
tttttatttt tttgtattat tttgtgtttt attttgtttt tttaagagta ttatatgttt 900
tatttaattt ttaaataatt gtttgaggta agtatagtta ttattttaat tatgtaaatg 960
agaaaataga ggtttagata tgttgagtaa ttttgataaa agttaaagaa ttaataagtg 1020
gaatagttga ggtttgaatt ttggtagttt gattgtagag atattatgtt tgatttattt 1080
ttttttgttt ttattttatg tttgttttta gtttttgagt ttgttgaatg aatgaatagg 1140
tggtagtttt tttttgttat aagattgatt agaattaaga taggtttaaa ttttatgtgt 1200
agaattttta aaattgtaaa ggtagtgtaa atttaaaaaa agaatggtat ttttaggaaa 1260
gaggaaaagt aagtgtgaga ataataataa taataattaa taaattttag taaatttagt 1320
aaatgtagta aatttttata ttaaaagttt ttggatatat attattatat tttatggtta 1380
tatgaaatat attataattt tattttgtat ataggaaatt tgagattggt ataaggagta 1440
tagagattta ggattttata tttttatttt tttaggattt tgtattttag gttgatatgt 1500
atgagtaaat tgggagtata atgggttttt taatagaaaa attaggaaag tttttttatt 1560
attattaatt atttatataa tattttttta attttattat tatttatatt ttaagtttta 1620
gagtatatgt gtataatgtg taggtttgtt atatatgtat atatgtgtta tgttggtgtg 1680
ttgtatttat taatttatta tttagtatta ggtatatttt ttaatgttat tttttttttt 1740
ttttttttat ataagattta taatggataa tggattttaa tttttagagt aaaatggttt 1800
tatttaagga tgttataatt tttttagagt tttattgtaa gatatgagat atatatattt 1860
aaaatttgtt tttggtattt ttaaagtagt taatttttat atttgtttat aatgtattta 1920
aatgttgttt ttatatggtt gtatttttta ttttttttat taatagttat atatattttt 1980
tataagagtt gaaagagttt ttgatgtagg aatttatggt agagttttag agaaattttt 2040
gaatttattg aaagttttat ttagaaatat atgtgtaagt gaatatattt tttttaaaaa 2100
aaattattat ttattttttt ttttgagaag aaggtattta ttttaataga tttttgaagg 2160
agtttatttt ttttattttt ttatttttat taagaattat tgtaggttgg gtatgatggt 2220
ttatgtttgt aattttagta ttttgggagg ttaaggtggg tggattattt gaggttagga 2280
gtttgagata agtttagtta atatagtgaa attttgtttt tattaataat ataaaaatta 2340
gttgggtatg gtagtatgtg tttgtaattt tagttatttg ggaggttgag gtaggagaat 2400
tgtttgaatt tgggaggtgg aggttgtagt gaattgagag agattgtgtg gtgttatttt 2460
attttagttt gggtaataga gtgaaatttt attttaaaaa aatatataaa ataaataaat 2520
aaaaagaaag aattattgta ttagtgatgg aaatgtgttt tttttttttt attttggtaa 2580
ttattttttt ttttttttat tataaaatat tttaaattaa attaaaataa ttttatttat 2640

tgatagtttg aatttttttt attattgtta tatagttaat tgagaggtat tttgaggaaa 2700
atataaatgg tatagtaatg tattgtagat tttaataata tatttgatat tttaaattgt 2760
ttttattggt tttattttaa aaattatatg ttttaaaatt aagtagatat taaaagtata 2820
agatatattg ggtttataag gtttaagtta attagggatt gaaatataat ttttaaatag 2880
agttggatta tttagtaggt agattaagta tgtgtttaag gtattagtaa agtttgagta 2940
atttattttt taaaatgtag tatatgtttt tgataagttt aaaaagtagt agttatagga 3000
aaaattagaa tttttatttt tttgtgtttg ttatattttt tagtgttgtt taattttttt 3060
ttgtaagtga gggtggtgga gggtgtttat aattttttta gggagtaagt tttttttggt 3120
tttttttttt tttttttttt tttttttttt tgagattaag ttttgttttt gttttttagg 3180
ttggagtgta atggtgtgat tttggtttat tgtaattttt gttttttttt gggtttaagt 3240
gattttttta tattagtttt tgagtagttg ggattatagg tatgtgttat taagttttgt 3300
taattttgta ttttttagta gagatagggt tttgttatgt tggttaggtt tgttttgaat 3360
ttttggtttt aggtgatttg tttgttttgg ttttttagaa tgttgggatt atagatgtga 3420
gttattgtat ttggattttt tttttatgta atagtgataa ttttatttaa agtatttttt 3480
tttttttttg agttggagtt ttattttgtt atttaggttg gagggtggtg gtgtgatttt 3540
ggtttattgt aatttttgtt ttttgggttt aagtgatttt tttgttttag ttttttgagt 3600
agttggaatt atatatgtgt gttattatgg ttagttaatt tttgtatttt tagtagagat 3660
ggggtgttat tattttggtt aagttggttt tgaatttttg attttaggtg atttgtttgt 3720
tttggttttt taaagtgttg ggattatagg tgtgagttat tgtgttttgt tttaaagtat 3780
ttttttttta tgttttaaaa taagattgta agttagtttt taaagtggat aatttaagag 3840
ttaataggta ttagtttagg atgtgtggta ttgtttttaa ggtttatatg tattaatata 3900
ttatttaaat ttataataat ttttataaag tagggggtat ttatattttt tttttttttt 3960
ataattatga aaaatgtaag gtatttttag taggaaagag aaatgtgaga agtgtgaagg 4020
agataggata gtatttgaag ttggtttttg gattattgtg taattttgtt tttagaatat 4080
tgagtatttt ttttggttta ggaattatga ttttgagaat ggagtttgtt tttttaatga 4140
tttttttttt atttttttat ttgtttatag gtagaatttt tttttgtttg tattaaataa 4200
attttatttt tttagagttt gtttttatat taggtaatgt atatgtttga gaaatttttg 4260
ttttagatag ttgttttata tgtaggaggg gaaggggagg ggaaggagag agtagtttga 4320
ttttttaaaa ggaatttttt gaattagggt ttttgattta gtgaattttg tgtttttgaa 4380
aattaagggt tgagggggta gggggatatt ttttagttgt ataggtgatt ttgatttttg 4440
gtggggtttt tataattagg aaagaatagt tttgtttttt tttatgatta aaagaagaag 4500
ttatattttt tttatgatat taaatatttt gatttaattt ggtagttagg aaggttgtat 4560
tgtggaggaa ggaaatgggg tgggggtgga ttttttttta atagagtgaa tgtatttaaa 4620
tatgtttttg ttggtaggtg ggggagtgtg gttgggagta gggaggttgg agggtggtgt 4680
ggggggtagg tggggaggag tttagttttt tttttttgtt aatgttggtt ttggtgaggg 4740
ttgtttttgg ttggtgtttt tgggggagat ttaatttggg gtgattttag gggtgttata 4800
tttgttaagt gtttggagtt aatagtattt tttttgagta tttgtttatg gtgttttttt 4860
gtttggaaag atattgtggt ttttttagag gatttgaggg atagggttgg agggggtttt 4920
tttgttagta ttggaggaag aaagaggagg ggttggttgg ttattagagg gtggggtgga 4980
ttgtgtgtgt ttggtggttg tggagagggg gagagtaggt agtgggtggt ggggagtagt 5040
atggagttgg tggtggggag tagtatggag tttttggttg attggttggt tatggttgtg 5100
gtttggggtt gggtagagga ggtgtgggtg ttgttggagg tgggggtgtt gtttaatgta 5160
ttgaatagtt atggttggag gttgatttag gtgggtagag ggtttgtagt gggagtaggg 5220
gatggtgggt gattttggag gatgaagttt gtaggggaat tggaattagg tagtgttttg 5280
attttttgga aaaaggggag gttttttggg gagtttttag aaggggtttg taattataga 5340
tttttttttg gtgatgtttt gggggtttgg gaagttaagg aagaggaatg aggagttatg 5400
tgtgtataga ttttttgaat gttgagaaga tttgaagggg ggaatatatt tgtattagat 5460
ggaagtatgt tttttattag atataaaatt tatgaatgtt tgggataaaa agggagtttt 5520
aaagaaatgt aagatgtgtt gggattattt agtttttaat ttatagatat ttggatggag 5580
tttatttttt ttattaggag ggattattag tggaaatttg tggtgtatgt tggaataaat 5640
attgaatata aattttgatt gaaattattt agaagtggtt gggtgtggtg ttttatgttt 5700
tgtaattttt ttattttggg agattaaggt ggggggaatt atttgaggtt gggagtttga 5760
gattagtttg gttaataggt gaaattttgt ttttattaaa aatataaaaa gtagttgggg 5820
gtggtggtag gtgtttgtaa ttttagttat ttgggaggtt gaggtaggag aattgtttga 5880
atttgggagg ttgaggttgt agtgaatagt gagatggagt tattttattt tagtttgggt 5940
gatagagtga gattttgttg aaagaaagaa agagagaaag agagagagaa aaattattta 6000
gaagtaatta tatattgtgt ttatttttaa ttgagtaggg taaataaata tatgtttgtt 6060
gtaggaattt aggaaataat gagttatatt tatgtgatta ttttagaggt aatatgtagt 6120
tattattttg ggaatatttg ttaatatttt tgttttttta ttatttttag tttatttgat 6180
atagtttatt tgtgataaga gtttttaatt ttttattttt gaatagaggt gttttttttt 6240
tttttatttt tgttttgtga gggagttagg ggaggattta aaagtaatta atatatgggt 6300
aatttagtat ttttaaaatt ttgttaatag tttgaatttg ggagtttggt tttgtagttt 6360
tataatattt tagaagagat tttatttgtt taaaaataaa aaggaaaaag aaaagtggat 6420

agttttgata atttttaatg gagaagggag aagaatatgt agaaaagggg aaatgatgtt 6480
ggtttagaat tttaattata ttggtgttta atataggaat atttatttat ataatatttt 6540
aaagtattaa atttatatta gtatattatt aaatggatat attattaaat gggtttaagt 6600
attttatata ttttaattta attgatttat tttttttttg ttttggattt ttattatgat 6660
ttaaatattt atatatgggt tattttttag attttttata ttatgaaata taagaaaaat 6720
ttttaaggtt agttttatga ttaagatgaa ggattttatt gaatatataa aataataaat 6780
atattgtaat attttgtttt tttttttgta gttgtaattt ggtttgttta tatttttttt 6840
ttgttttttt gaaaattgag ttagttttat ttttttagga taggatttaa taattataat 6900
ataatttagt ataatttttt gatttaggta aattatgtaa tttgtgttta gtatgaaatg 6960
tatttaaaaa taagtaattt ttttttaata ttattatttt taaattaata taataaataa 7020
tagttatttt aaaataaatt gtttattttt attatgtagt atttaaattt taaggttgtt 7080
atgattgtag atagtatttt aaaatttttt tttggaaatg gttttgtttt taagatgatt 7140
taggaattaa agaggtgatt attttttgtt taatgaattt ttaaattata aatttgggaa 7200
gtgttttagt tttttattgt tgttgttata aattattata aatgtgttag ttaaaataaa 7260
tataaaatta ttattttata gttttagaga ttagaagtta aaaatgggtt tataaggttt 7320
tatttttttt ggaaatttta aggggtaatt tgtttttttg ttttttttag tttttagtga 7380
ttattaaatt ttttggttta tggtttttgt attttttttg tggtttgtgt ttttattttt 7440
gtattttttt tttgattgtg attttttaat aaaaatattt ggggttatgt tgggtttatt 7500
ttgaaaattt tggataattt tttttaagat tattaattaa attatatttg taaagttttt 7560
tttgttatat aagttaatgt attaaaagtt tttgaggatt aggatataga tattgggggt 7620
gggggggtat tatttagttt attataggaa ggaattttag ggttaattaa attagttttt 7680
ttattttata tttgaagaaa ttgaagtttt ggaattggag agtattatgt taaatgaaat 7740
aagttaaata tagaaagata aatattatat gtttttattt atttgtgaaa tataaaataa 7800
ttatattttt agtagtaaag agtagaatgg tggttattag agttgggggg tgggaggaat 7860
ggggagatgg taattaagat ataaagtttt agttaagatg ggaggaataa gtttgattgt 7920
tttttttgag atgtgtttta tagtatgatg aatatagtta aatagtaaat tttaaatgtt 7980
tttatttgat aaaaatgtta aatatttgag atgatggata ggttatttag tttgatttaa 8040
taatttttta ttgtgtttaa agattataat tttatattgt attatataaa tatatataat 8100
tgtattattt taatatataa ttttaaaatt aatataatga aaaagaaatt gaagtttaat 8160
atttttagaa gttaagtgta atttaaaagt tttgtgagaa tttgttttaa taaataaata 8220
agtttttttt ttttaataat tattatattt tgtgtttgga tatatagtag tgaataaaaa 8280
aaaaaaaaaa aaaaaaaatt tttaggttta atataatttt aggaagaaat tttagtagtt 8340
gtattttagg ggaaatatag gaagttagtt tggagtaaaa gttagtttgt ttttgttttt 8400
ttgttatttt gtttgtgttt tatagtgttt tttgtttgtg atgatagttt tgtagaagtt 8460
tggaggatat aatggaattt attgtgtatt gaagaatgga tagagaattt aagaaggaaa 8520
ttggaaattg gaagtaaatg taggggtaat tagatatttg gggtttgtgt gggggtttgt 8580
ttggtggtga gggggtttta tataagtttt ttttttgtta tgttggtttt tattttggtt 8640
ttgattattt tgtttttttt ggtagg 8666

<210> 296
<211> 8666
<212> DNA
<213> Artificial Sequence

<220>
<223> chemically treated genomic DNA (Homo sapiens)

<400> 296

tttgttagag agaatagaat ggttagagtt agggtggggg ttggtatgat ggaaaggaag 60
tttgtgtaga gtttttttat tgttaagtag atttttatat aagttttagg tgtttaatta 120
tttttatatt tgtttttagt ttttaatttt ttttttgagt tttttattta ttttttagta 180
tataatgaat tttattatat tttttgaatt tttgtggagt tgttgttata ggtagagagt 240
attgtgaggt atgggtaaaa tagtaaaggg gtagggatag attgattttt attttaggtt 300
aattttttgt attttttttg agatataatt attgaaattt ttttttgaaa ttatgttagg 360
tttggagatt tttttttttt tttttttttt tgtttattgt tgtatattta agtgtagaat 420
gtggtaattg ttaaaaagag aaaatttgtt tgtttgttaa aataaatttt tataaaattt 480
ttaagttata tttagttttt gggaatgttg aattttaatt ttttttttat tatattagtt 540
ttaaaattat atattgggat agtatagttg tatatattta tgtggtataa tatgaagtta 600
tgatttttga atataatggg gaattattaa gttaagttaa gtaatttatt tattatttta 660
aatatttgat atttttgtta aatgagagta tttgggattt attatttagt tatatttatt 720
atgttatgaa atatatttta aaaaaaataa ttaaatttat tttttttatt ttaattgagg 780
ttttatattt tgattattat ttttttattt tttttatttt ttagttttag taattattat 840

tttatttttt attgttaaga atgtaattgt tttatatttt atagataagt gagaatatgt 900
gatatttgtt tttttgtgtt tggtttattt tatttagtat aatgtttttt aattttaaaa 960
ttttaatttt tttaagtata aaataagaag gttagtttaa ttaattttaa aatttttttt 1020
tgtggtaggt tgaataatgt ttttttattt ttaatgttta tgttttaatt ttttaaaatt 1080
tttaatatat taatttatgt ggtaaaagag gttttgtaga tgtgatttaa ttaatggttt 1140
tgagggagat tatttagaat ttttagggtg ggtttaatat aattttaagt gtttttatta 1200
gagggttata gttagagaga agatataaga atggaagtat aggttataga gaaaatatag 1260
agattatgag ttaaggaatt tgatggttat tagaagttgg aaaagataag gaaatagatt 1320
gttttttaga gtttttaaaa ggaatgaaat tttgtggatt tatttttgat ttttgatttt 1380
tagaattgta aaataataat tttgtgtttg ttttagttaa tatatttgtg ataatttgta 1440
atagtagtag taggaaatta aaatattttt taggtttatg atttgagagt ttattaaata 1500
agagatggtt atttttttgg tttttaaatt attttggaaa taaagttatt tttagagagg 1560
aattttaaaa tattgtttgt agttatagta attttaaaat ttgagtgttg tatggtggaa 1620
gtagataatt tattttagga taattgttat ttgttatatt agtttgagga tggtggtgtt 1680
aaagaggagt tatttatttt taggtatatt ttatattaaa tataaattgt ataatttgtt 1740
taaattaagg aattatatta aattatatta tggttattaa attttgtttt gagaaagtga 1800
aattgattta gtttttaaag agataaagag aaagtataag taaattaaat tgtagttata 1860
aaaagaaaga taaaatgttg tagtatattt attgttttgt gtatttaatg aagttttttg 1920
ttttggttat aaaattagtt ttaaaggttt tttttatatt ttatagtatg aaaaatttaa 1980
aaagtaattt atatgtaaat atttaaatta tgatagaaat ttaaagtaaa aagaaaatga 2040
attaattgaa ttaaaatgtg taggatgttt aaatttattt gataatatat ttatttgata 2100
atatattaat atgaatttag tattttaaaa tgttatataa ataaatgttt ttatattaaa 2160
tattaatgta gttaggattt taagttaata ttattttttt ttttttatat gttttttttt 2220
tttttttatt aaaaattgtt aaaattattt attttttttt tttttttttg tttttaaata 2280
aataaggttt tttttaagat attgtaggat tataaagtta aatttttggg tttaagttgt 2340
tggtaaaatt ttagagatgt taagttattt atgtattaat tatttttaaa ttttttttta 2400
atttttttat aaaataggag tagggagagg agaaatattt ttgtttaaaa atgaggaatt 2460
gaaaattttt attataaata aattatatta agtaagttaa agatagtaaa agagtaaaaa 2520
tgttagtaga tatttttaaa atggtaatta tatattattt ttggaatgat tatatgaatg 2580
tggtttatta ttttttaagt ttttatagta aatatatatt tatttgtttt atttagttaa 2640
aaataaatat aatatgtagt tgtttttgaa taattttttt tttttttttt tttttttttt 2700
tttttttgat aaagttttat tttgttattt aggttggagt gaagtggttt tattttgttg 2760
tttattataa ttttagtttt ttgggtttaa gtgatttttt tgttttaatt ttttgagtag 2820
ttgggattat aggtgtttgt tattattttt ggttattttt tgtattttta gtagaggtga 2880
ggttttattt gttggttagg ttggttttga atttttgatt ttaggtgatt ttttttgttt 2940
tgatttttta aagtgaaggg attataaggt gtgaggtatt gtgtttggtt gtttttgaat 3000
aattttgatt aaaatttata tttgatattt attttaatat atattataga tttttattga 3060
taattttttt tagtaagaaa gataagtttt atttaggtat ttgtgaattg gaggttaagt 3120
agttttagta tattttatat ttttttaaga tttttttttt attttaaatg tttgtaaatt 3180
ttgtatttga taaagagtat atttttattt aatataaata tgtttttttt tttagatttt 3240
tttagtattt gagagatttg tatgtgtgtg gttttttatt tttttttttt ggttttttaa 3300
gtttttaggg tgttgttagg aggaggtttg tgattataaa ttttttttga aaatttttta 3360
ggaagttttt tttttttttg gagaattgaa gtgttatttg attttaattt ttttgtaaat 3420
tttgtttttt agagttgttt gttatttttt gtttttgttg tagatttttt atttatttgg 3480
attggttttt gattgtaatt atttggtgtg ttgggtagtg tttttgtttt tagtagtgtt 3540
tgtatttttt ttatttgatt ttgggttgtg gttgtggtta gttagttagt tgaaggtttt 3600
atgttgtttt ttgttgttgg ttttatgttg ttttttgttg tttgttgttt gttttttttt 3660
tttttgtagt tgttgagtgt atgtggtttg ttttattttt tggtgattag ttagtttttt 3720
tttttttttt ttttggtgtt ggtggaagag ttttttttga ttttgttttt taaatttttt 3780
ggagggattg tggtattttt ttaggtaagg ggatgttgtg agtgagtgtt tggaggaggt 3840
gttattaatt ttgagtattt agtgaatgtg gtatttttga agttgtttta ggttgggttt 3900
tttttggggg tattagttgg aagtagtttt tgttagagtt agtgttggta aggaaggagg 3960
attgggtttt tttttatttg ttttttatat tgttttttgg tttttttgtt tttagttgtg 4020
tttttttgtt tgttagtaaa ggtgtgtttg agtgtgttta ttttgttaaa aagaaatttg 4080
tttttgtttt gttttttttt tttgtgatat aattttttta attgttaaat tgaattgggg 4140
tgtttggtgt tatagggaaa gtatggtttt tttttttaat tataagaaaa agtaaaatta 4200
ttttttttta gttgtgagag ttttattgag aattgaaatt atttgtatga ttagaaagtg 4260
tttttttatt tttttaattt ttgattttta ggagtgtggg gtttattaag ttagaaattt 4320
tagtttaaag gatttttttt ggagagttgg attgtttttt tttttttttt tttttttttt 4380
tttgtgtgta aaatggttgt ttggggtaag ggttttttag atgtgtatat tgtttggtat 4440
aagagtagat tttgaaaaga tgaggtttat ttaatatgga tgggggagaa ttttgtttgt 4500
aggtagatag gaaaatgggg agggagttat tggaaggatg gattttattt ttaaagttat 4560
aatttttaga ttagaaaaag tgtttagtgt tttagaagta gagttgtata gtgatttaaa 4620

gattagtttt aaatattgtt ttgttttttt tatatttttt atattttttt tttttattga 4680
aaatattttg tattttttgt aattataaag ggggaaggga atatgagtgt tttttgtttt 4740
ataggggttg ttgtgagttt aaatgatgta ttaatatata taagttttaa gaatagtgtt 4800
atatatttta agttaatatt tgttagtttt tgaattattt gttttgagga ttggtttgta 4860
attttgtttt gaggtataga aagaaaatgt tttggagtag gatgtggtgg tttatatttg 4920
taattttagt attttgggaa gttgaggtgg gtagattatt tgaggttagg agtttgaggt 4980
tagtttggtt aaaatggtga tattttgttt ttattaaaaa tataaaaatt agttggttat 5040
ggtggtgtat gtgtgtaatt ttagttattt aggaggttga ggtaggagaa ttgtttgaat 5100
ttgggaggta gaggttgtag taagttgaga ttgtgttatt attttttagt ttgggtgata 5160
gaatgagatt ttgatttaaa aaaaaaaaaa aatgttttgg atagaattat tattattata 5220
taaaaggaaa gtttggatgt ggtggtttat gtttataatt ttagtatttt gggaggttga 5280
gataggtgga ttatttgagg ttaggagttt gagataagtt tgattaatat ggtgaaattt 5340
tgtttttatt aaaaaatata aaattagtgg ggtttggtgg tgtatgtttg taattttagt 5400
tatttggagg ttgatgtagg agaattgttt gaatttagga gaaggtggag gttgtagtga 5460
gttgagattg tgttattgta ttttagtttg ggagataaga gtgaaatttg gttttaagaa 5520
aaaaagaaag aaagaaagaa agaaagatta agaagaattt attttttgaa aagattatgg 5580
gtatttttta ttatttttat ttataaagaa aagttaaata gtattaaaga gtataataag 5640
tgtaaggagg taaaagtttt aatttttttt gtgattatta ttttttaagt ttattaaaaa 5700
tatgtattat gttttaaaaa atggattgtt tagattttgt tgatgtttta agtatatgtt 5760
taatttgttt attggataat ttagttttgt ttaaaagtta tattttaatt tttggttgat 5820
ttaaattttg tagatttagt atattttgta tttttagtgt ttgtttgatt ttaaaatatg 5880
tagtttttaa aatgaagtta atgaaaataa tttgggatgt taagtatgtt attaaaattt 5940
ataatgtatt attgtattat ttatattttt tttggggtat tttttaatta gttgtgtagt 6000
aatgataggg aaaatttaaa ttattgataa ataaaattat tttagtttag tttaagatat 6060
tttatgatgg aggaggaaga aagtggttgt taggatggga gggagggaat atatttttat 6120
tattaatata atggtttttt ttttttgttt gtttgttttg tgtgtttttt tgagatggag 6180
ttttgttttg ttgtttaggt tggagtgaaa tggtattgta tgattttttt tggtttattg 6240
taatttttgt tttttgggtt tgagtaattt ttttgtttta gttttttgag tagttgggat 6300
tataggtata tgttgttata tttagttaat ttttgtatta ttagtagaga tggggtttta 6360
ttatgttggt taggtttgtt ttgaattttt gattttaggt gatttattta ttttagtttt 6420
ttaaagtgtt gggattatag gtatgagtta ttgtgtttgg tttatagtgg tttttaatgg 6480
gggtgggaga gtgggaagag taggtttttt taagagtttg ttgaaataaa tatttttttt 6540
ttaaaaaaga aagtaggtaa tgattttttt taaaaaagat gtgtttattt gtatatgtat 6600
ttttagataa aatttttagt gaatttaggg atttttttga aattttatta tggattttta 6660
tattaagaat ttttttagtt tttgtgaaaa atatatatag ttattggtga agaagatggg 6720
agatgtaatt atataaaaat aatatttgga tgtattataa ataggtgtaa aagttgattg 6780
ttttggaaat attaagaata agttttagat atgtatattt tatattttgt agtagagttt 6840
tggaaggatt atggtatttt tgggtggggt tattttgttt tagaaattga agtttattat 6900
ttattataaa ttttatgtag gggggagggg ggagggatag tattaggaga tatatttaat 6960
gttaaatgat gagttgatgg gtgtagtata ttaatatggt atatgtatat atatgtaata 7020
aatttgtata ttgtgtatat gtattttaaa atttaaagta taaataataa taaaattaaa 7080
aaaatattat gtaaataatt aataatagtg ggaaaatttt tttagttttt ttgttaaaga 7140
gtttattata tttttagttt atttatatat attgatttga ggtgtaaaat tttagaagaa 7200
tgaaaatata aagttttgga tttttgtgtt ttttatgtta gttttagatt ttttatgtgt 7260
aaaatgggat tataatatat tttatgtggt tataaaatat gataatgtat gtttaaaagt 7320
ttttaatgta aaaatttatt atatttatta aatttattaa agtttgttgg ttattgttat 7380
tattattttt atatttattt tttttttttt ttgagaatgt tatttttttt ttagatttgt 7440
attgtttttg tagttttgaa aattttatat gtgaaattta aatttgtttt aattttgatt 7500
agttttataa taaaaaaaga ttattatttg tttatttatt taataagttt agaaattaag 7560
ggtagatatg gggtgggggt agaggggagt aggttaaata tagtattttt atagttagat 7620
tgttagggtt taaattttaa ttgttttatt tattggtttt ttaattttta ttaaagttat 7680
ttaatatgtt tgggtttttg tttttttatt tgtatgatta ggataataat tgtatttgtt 7740
ttaaatagtt gtttagggat taaatgaagt atgtaatgtt tttagaagag taaaataaag 7800
tatagaatga tataagaaaa tgaaaaggtg attaaattta tttttatttg tttttagtag 7860
gtaaaggttt ttggatgttg agagttgatt ttagttttga agaatgatgt gagagtttaa 7920
agatggaaaa gaaggagaaa gatattttag gtagaggtaa gaggatgtgt aaaggtatag 7980
aagtttagga aaattgaaag taatttattt agatttattt ttgaaaatta gtttgtgaag 8040
gattttttag agagttaaaa gaaaaattag gataatggga tggtaaggaa gtggggatat 8100
tgtgttgatt atttttttat agtttggttg tgatagttta aggttttatg ttatgaagtg 8160
aggtagatgg aaggtggagg agatttgagt atatatatat ttggaagggt aagtgttaag 8220
tgaagagaag ggaaagttag aaaagaataa aagaaaagtt aagtatatag gtaagatagg 8280
atatttttga ggatttgagg ttttttaaaa ataatgaaga aatagaattt aaagtatagt 8340
ggtggttata gattttgtta taagagatat atagatttgg gagggagaaa aggttagaat 8400

ggggaggaag ttgaggtatt tttttttatg aatgaggagg ttagtttgtt tgtagagtga 8460
aaataagatg gttggttagg aagtttgaga attgtaaagg tttggaatat tttttatgag 8520
tagagaggat gggttgagta agatttagat agagaagttt tttgttttgt tttttaaaat 8580
tagattggag aatttagttg agggtggaga ttatgatata gtagggatat ttgattattt 8640
tttttaaatt aaagaaagtt ttgtag 8666

<210> 297
<211> 2501
<212> DNA
<213> Artificial Sequence

<220>
<223> chemically treated genomic DNA (Homo sapiens)

<400> 297

gaaaattagg ttgagtaggt tggtttttta aagaaattta ttgagttttt ataatgtttt 60
ttttatttga tgggagaaaa ggggtttggg aagattaagt gtgaagtttt agaattttgt 120
ttggagttgg ttaggattat ttaggtttga gtataagtaa agtgttgttt gtggtgttag 180
taagtgtgaa tgtttttggt aatgtttttt ttatttaaag ttgtttttta tttagttaat 240
ttgttttgtg aagtttatat atttgttttg tgtaagattt ttagttattt tatttagtta 300
ttggtttttt ttttattatt attttttatg ttttttttaa agaaaaatat tattttttat 360
ttttttatta attttaaata gtttttattt ttttgatata tttattattt aagaaaatgg 420
tttaagaatt gggttagatt attttgtttt aatttttttg ataagtttta gagaaattta 480
aaggttaaaa taagagtata agagtaaaag gtagagaaat taagaaattg aagattagga 540
aatgggggtt ggatgggaaa gaaaaagaaa ttgttattaa tgttatttgg tttttttttt 600
tgtttaggtg gattttagtt ttgttgaggt tttgttagta gatttttagt tttaattagt 660
atttttatgg tggtggtatt tttattgttt ttaaaagaaa gagttttttt taattttata 720
gggatttggg ggatgaggag ttagagttaa ggtattttaa aaaaaatatg tgaagatttt 780
tattttgtaa tatataagta attgtttttt tgttaagatt ttgttttttt tagtattttg 840
aattaaaatg attttgtaaa taaaaattgt ttatttttag gagaggttta tttatgtagt 900
tttttattaa agtttttagg taataaattt ataatttgtg gttttttttt tatttaatgt 960
agtatttgtt gaaatgtttt aaatatttta agtaataaat gttgatttaa atttatttag 1020
gaagattagg aaggggaaaa aaagtatttg gtatttaaat ttttagaaga gaatttaatg 1080
ataggtttag tttgtttaat gataagttta gtattatatt ttttttttat gatgttttat 1140
tattattgta taaatttttt ttattattta tgataaataa aaataagata tttgataaag 1200
tgggtttaaa taggtaagag tgtaaataaa gatttattgt ataaatatga tgaaattggg 1260
attttagatt tttaaagtat aatttttttt tgttttatgt gtgttaggtt gttattttta 1320
attttgaatt agtttttttt ttttttaagg gttgtattta taatgtaaaa atggaaagaa 1380
ttaaaaagta tatgtaaaat atgatttttg ggattttttt ttatttttat ggttgattaa 1440
tttaaataga aagatatatt taagagaaaa ttgttaagtt tgatataagt tatgaaattt 1500
gtgaagttta agtattgttt ggggatgaat ttaatttgta tgataggtgt agagttgttg 1560
tttttagata ttttaagaaa tatggagtta ttttgaatga tttttttttg gttataaggg 1620
agttattaat gtttttatag tgaaattaat tggttggttg aaggaataga aattttttgt 1680
tttgtttatt ggggattagg agttgagggt agtggtgaat atttttaaaa tattagtttt 1740
ggttttattg gatatttagt gagtagtgta gttagtattt ttggtggttt tttggtttag 1800
tttttggtgt atgtgtttta gtatttttgg gtaggttttt ttgtttttgt gatgtgttgg 1860
tttgggtttg gttttttggt gattatagtg gatagggggt ggagtttaag ggggtgggga 1920
gatgttggtt ttttggttta gttgaaaatg gaattttttg ttggttggtt ttttattttg 1980
ttagagtgag gtggggtagt gaggattttg tgatgtgttt gtattttgtg gttagagtgg 2040
ttttgagttt ggttgtgttt gtgttaggtg ttttttttta gaagtaattt aggtgtgttt 2100
gttggttttt gagtgttagg aaaagtttgg agttaatgat tggttgtttg gttattgtat 2160
ggggttttaa gttgtagaag gatgatggga gggtaatgaa gttgagttta ggttttttag 2220
gaaggagaga gtgtgttgga gtagtgtggg aaagaaggga agagtgttgt taagtttatg 2280
gttaatggtg gattatttgg gttgttgtgt gtttgggggt tgtggaatgt gtgaggagaa 2340
taagggtatg tttagtgggg gtggtagtga tgagggtttg gttagtgttg tggtgtgggg 2400
attagtggag aaggtgtgat agtttttgga agttggtgtg gattttaatg gagttaattg 2460
ttttgggagg tgtgtgattt aggtagttgg ggttttaggg t 2501

<210> 298
<211> 2501
<212> DNA
<213> Artificial Sequence

<220>
<223> chemically treated genomic DNA (Homo sapiens)

<400> 298
   gttttggggt tttagttatt tggattgtgt gttttttgaa atggttgatt ttgttgggat 60
   ttgtgttggt ttttaggagt tgttgtattt tttttattag tttttgtgtt gtggtgttgg 120
   ttagattttt attgttgttg tttttattgg gtatgttttt gttttttttg tgtattttgt 180
   agtttttaga tgtgtagtgg tttggataat ttattgttgg ttgtaaattt aatgatattt 240
   tttttttttt ttttatgttg ttttggtgta tttttttttt tttaggagat ttgggtttag 300
   ttttattatt tttttgttgt ttttttgtgg tttggggttt tgtgtagtgg ttgagtggtt 360
   ggttgttagt tttgggtttt ttttggtgtt taagaattag tgggtgtgtt tggattgttt 420
   ttgggaaaaa gtgtttagtg tggatgtagt tgagtttaaa gttgttttgg ttgtagggtg 480
   tggatgtgtt gtggagtttt tattgttttg ttttgttttg gtagagtggg gagttagttg 540
   gtaaagaatt ttgtttttag ttgggttaag gggttggtgt ttttttattt ttttaggttt 600
   tgttttttgt ttgttgtgat tgttgggagg ttaggtttgg gttgatgtgt tatgagggtg 660
   gggaagtttg tttaaagatg ttaggatgta tgtgttagag attgggttag ggagttgtta 720
   ggaatgttgg ttgtattgtt tgttggatgt ttagtaaagt taaggttaat attttgggaa 780
   tgtttattat tgtttttagt ttttaatttt tagtaggtgg agtagaggat ttttgttttt 840
   ttagttagtt agttggtttt attgtggaga tgttggtggt ttttttgtga ttgagagaaa 900
   gttatttaaa ataattttgt gttttttaag atgtttgaaa gtgatagttt tgtatttgtt 960
   atataagtta aatttatttt taggtagtat ttgggtttta taagttttat aatttgtatt 1020
   aaatttagta attttttttt ggatgtgttt ttttgtttga attagttaat tataaaaata 1080
   gagaaaaatt ttgagaatta tgttttgtgt gtgtttttta atttttttta tttttgtatt 1140
   atggatataa tttttaaaag agaaaaaaat tagtttgaga ttgagagtgg taatttggta 1200
   tatataagat aaaaaaaaat tatattttaa gaatttgaga ttttagtttt attatatttg 1260
   tatagtaaat ttttgtttgt atttttattt atttaaattt attttgttag gtattttatt 1320
   tttatttatt atgagtaata aaggaaattt atgtagtaat aatgaaatat tataagagag 1380
   gggtgtggtg ttgggtttgt tattaaatag gttgaatttg ttattaaatt tttttttgaa 1440
   gatttaaatg ttaagtgttt tttttttttt ttttaatttt tttaggtgag tttgaattaa 1500
   tatttattat ttaaaatatt taaaatattt tagtggatgt tatattggat aggaagagaa 1560
   ttgtaagtta tggatttgtt gtttaaaaat tttggtgagg aattgtataa gtggattttt 1620
   tttaaaagtg aatagttttt gtttatagaa ttattttggt ttggagtgtt gaggaagata 1680
   aagttttaat aggagggtaa ttgtttgtgt attgtaaaat gagagttttt atatgttttt 1740
   tttaggatat tttagttttg atttttattt ttttaaattt ttgtagaatt aaaaaaagtt 1800
   ttttttttta aaggtagtgg aagtgttatt attatggaag tgttggttag ggttgaaaat 1860
   ttattgatag agttttaata gagttgaaat ttatttggat agggaaggga attgggtagt 1920
   attaataata attttttttt tttttttatt taatttttat tttttagttt ttagtttttt 1980
   aattttttta ttttttattt ttatgttttt gttttgattt ttgagttttt ttgaaattta 2040
   ttagaaaagt taggataaga tagtttgatt taatttttga gttatttttt taggtagtaa 2100
   atatgttaga aaaatgaaag ttgtttggag ttgataagga aatggaagat aatgtttttt 2160
   tttgaggggg atataaagaa tggtgatagg gaaagaatta atgattaagt aaaatgattg 2220
   agaattttgt atgaggtaga tgtgtgagtt ttgtgaagta agttgattga atgaaaaata 2280
   attttgggta gggaaaatgt tgttgggggt atttgtgttt attagtatta tggatagtgt 2340
   tttgtttata tttaggtttg ggtggtttta attagtttta gatagaattt taaagtttta 2400
   tatttgattt tttaaagttt tttttttttt gttaaataga aaggatatta tggaagttta 2460
   atgaattttt ttgaaaaatt aatttgttta atttggtttt t 2501

<210> 299
<211> 2501
<212> DNA
<213> Artificial Sequence

<220>
<223> chemically treated genomic DNA (Homo sapiens)

<400> 299

gtgttttagt ttttgaattt atgttgtttg ttattatatt gtattgtttg tggatgattt 60
atttgttttt gtagttttat ttgaagaatt tttttttttt tttttttgag gtttgtttta 120
aatattattt tttttgtgaa gattgtttgg tgttttttag gagagagtgt gatttttttt 180
tttaggaatg gtagtatttt aaatatatat attttgtagt atattttatt ttgtatggta 240

atggtttgtt tgtgagtttt attttgtatt agagggtgag ggttttgaag atagtgttag 300
gttttattta ttttgatatt ttataatatt tattatatgt ttgatgaatg aatgtatagg 360
gggatggttg ggtgtatttt ttttaatttt ttaatttttt gaaataaata ataaaatttt 420
attttttttt agaagttttg gtatggtttt ttttattttg ttatagttat tgataattat 480
ttttattttt tgtgtaattt attaggtaag aagtttatgt agatttattt ttagtagttt 540
atatgataaa taaatattgt gtttgatttt taaatattaa attatagtat attatagata 600
gatatagagt tattatttaa agtatgatat tttaatttta aaaggttttt tttgaagaat 660
attataaatt tttttttttt ttaagatttg ttgggtagga aagatgggag aaaatgaatt 720
aatgtttata tagaaaggag gataatgggg gtaaaaataa tagatgaatg tatgggtgga 780
tgagagaatg gataaaatga taggtggata tgttgatttt ggatagatgg gaaatgagtg 840
gatatattaa taaatagata tgtgggtgga tgggtggaga agaggatggt ggatggttgt 900
ggttttatga agagatgtga aaaaggaagt gtggaatgat ggatgagaag ttgtatggga 960
agatgaatag aagaataggt ggttgaataa attaaaaggt gtgtggttgg atgaatgaat 1020
gagtgggatg atagatggat ttaagtggtt agtggatgga taggaggatg gatggatgtg 1080
agagttttag aaggatataa ggaaagatgg gtggatagat ggatgggtgg atggaaggat 1140
atttaggagg atgaatgagt atgtgtgtgg agagaggtgt ttatttatat tggtttgaat 1200
atatgggtta gttgagttaa atgttagttt tatgataggt tattagtagt tttttttgag 1260
ttgttttgtt aagaagttaa aatttattta agttatgtgg atttgttatt gaggggaaaa 1320
agaatgagtt tttttttttt ttatttggaa gatttattaa ttttttattt tttatttttt 1380
attgtgggta tggaggtatt gtgttattta gggtaagatt ttgttttttt tttagttttt 1440
tttttaggat atttaatatt ttgtgaaatt tagagatttt gttttagttg gatttagaga 1500
aatttagtgg gaaaggagag gttaaaggtt gaatttaatg gtgtaaggtt ttatggtttg 1560
gttatttttt gttttgatgt tgtggggtta gtgggagaag aaagttagtg tgtttttggg 1620
tgtaggggtt agtggggttt ggaggtatag gtattttgtg atattttagg ttttttgatt 1680
tatgtttttg gtagttttga ttatttatag ttttagtaga gtatggggtg gggtagaggg 1740
ggtttgtttg ggagggttgt tattttttaa aatttttgtg ggttgtttag ttatagtttt 1800
ttttgtttgg gtgtgttttt tgtttgtttt ttttttttgt tttaggttat tgtttttaat 1860
tttgaataaa aattgtagtt aatttttgag gtagttttat tgtttagtgg attttagttt 1920
ttgttaggtt tggtttgtta tttttgtttt gttttttgtt ggtttttgtt ttgtgtttag 1980
ggatttttta gttttttttg tttgtgtttt ttgtttgttt tggatattat ggataagttt 2040
tggtggtatg tagtttgggg atttgttttt gtgttgttga gtttggtgta gattggtgag 2100
tgtttgttgt agtttgggta gtaagatggg tgtggggtgt ttagtgtgga tttggtggta 2160
gtttttttgg ttgagttggt tttgggggat tggagttaag tgagttgttt gtgaagtgta 2220
ttgggttttg gaaagtaggg ttgggatttg tgttaaattg ttggagaatg tgtttgtgga 2280
agtattattt ggttgaaaga aaaagagaaa gagaagaaag tttgttgggt aggttgttgg 2340
tgtgtagttt tgggtgaggt tgttagagtt gtagtatatg gtagaaagta attgtttttt 2400
tggatgtgta tagttgttgt ttggattaat aggtttttgt gtttaagggt ttgttaagtt 2460
ttattgggtt gtgtttaggt agggtagagt tgggtggggt a 2501

<210> 300
<211> 2501
<212> DNA
<213> Artificial Sequence

<220>
<223> chemically treated genomic DNA (Homo sapiens)

<400> 300

tgttttgttt agttttgttt tgtttagata tagtttggtg gggtttggtg agtttttggg 60
tataggagtt tgttagttta gataatgatt gtgtgtattt gggagaatgg ttattttttg 120
ttatgtgttg tagttttagt gattttgttt aaaattgtgt gttggtagtt tgtttaataa 180
attttttttt tttttttttt tttttttaat taaatggtgt ttttatagat atatttttta 240
atggtttagt gtaaatttta gttttgtttt ttggagttta atgtattttg tagatagttt 300
atttgatttt agttttttag ggttgattta gttggagggg ttgttgttgg gtttgtgttg 360
agtattttgt atttattttg ttgtttaggt tgtggtgggt atttattgat ttgtgttagg 420
tttagtggta tgaggtagag tttttaggtt gtgtgttatt aaaatttgtt tatggtgttt 480
ggagtgaatg gagggtgtgg gtgaaaggag ttggaggatt tttgggtgtg gggtaggggt 540
tggtggagga tgggatgagg atggtggatt gaatttggta gaggttgggg tttgttgggt 600
aatgaggttg ttttggaagt tggttgtagt ttttatttga ggttgaaaat agtgatttaa 660
gatggaggga gggagtgagt gaaggatata tttaagtaag gggggttgtg attaagtagt 720
ttgtagaggt tttaagaagt agtagttttt ttgggtgggt ttttttgttt ttgttttgtg 780
ttttgttgag gttgtaaata attggggttg ttagggatgt gggttgggga atttggagtg 840

ttgtggggtg tttgtgtttt tgagttttat tggtttttgt gtttagagat gtattggttt 900
tttttttttg ttggttttgt ggtgttagga tagaggatga ttgaattgta aaattttgta 960
ttattgggtt tagtttttgg tttttttttt tttgttaaat ttttttgaat ttggttggag 1020
taagattttt gggttttata ggatgttgga tattttggga gaggagttgg agagagggtg 1080
aggttttgtt ttgggtggtg tagtgttttt gtgtttatag tggggagtga ggggtgggga 1140
gttggtgaat tttttaagtg gaaagaggga gagtttattt tttttttttt taataataag 1200
tttatatggt ttgaatgaat tttagttttt tggtagaata gtttagggaa agttattgat 1260
ggtttgttat agggttggta tttggtttag ttaatttatg tgtttaagtt agtgtgaatg 1320
ggtatttttt tttatatata tgtttattta tttttttaaa tattttttta tttgtttatt 1380
tatttattta tttatttttt tttatgtttt tttggggttt ttatatttat ttattttttt 1440
gtttatttat taattattta ggtttattta ttattttatt tatttattta tttaattata 1500
tattttttaa tttatttaat tatttatttt tttatttatt tttttatata attttttatt 1560
tattatttta tatttttttt tttatatttt tttataaaat tataattatt tattattttt 1620
ttttttattt atttatttat atatttgttt attgatatat ttatttattt tttatttgtt 1680
taagattaat atatttattt attattttat ttattttttt atttatttat atgtttattt 1740
attatttttg tttttattat tttttttttt gtgtaaatat taatttattt ttttttattt 1800
tttttattta gtagatttta aagagaggaa gagtttgtaa tattttttag gggaagtttt 1860
ttgagattga aatattatat tttgaatgat aattttatat ttatttataa tatattatgg 1920
tttaatgttt ggaaattaaa tgtagtattt atttgttatg tgaattatta aaagtaaatt 1980
tgtatggatt ttttgtttgg tgagttatat agaaaatgaa agtgattatt agtggttgtg 2040
gtaagatgaa aggaattatg ttaggatttt tgagggaagg taagattttg ttatttattt 2100
taagaaattg aaaagttgag agaaatgtat ttagttattt ttttatgtat ttatttatta 2160
gatatgtaat gagtgttgtg gggtattgag gtaaataaga tttggtgtta tttttagagt 2220
ttttattttt tggtatggaa taaaatttat aagtaggtta ttattatgta aggtgaaata 2280
tgttgtaaaa tgtgtgtgtt tggggtgtta ttatttttag agaagggagt tatatttttt 2340
tttggagaat attaggtagt ttttataggg gaagtgatat ttgaggtagg ttttaaagga 2400
aaaggaggag gaatttttta gatgaaatta tagggataag taagttattt ataggtagta 2460
tagtgtagtg atgaatagta tggatttagg agttagaata t 2501

<210> 301
<211> 16579
<212> DNA
<213> Artificial Sequence

<220>
<223> chemically treated genomic DNA (Homo sapiens)

<400> 301

tgttttttaa aatgatgttt agttgaatta gttaatggag ataaaaatat ttatatataa 60
ttttttttta agtttattag ttattttata gattagttat aattttggtt attttaatga 120
gttaattttt ttttagagat attataaatt ttttaaagat ggaaaataat tgttattttt 180
ttgaataatg tttattattt aaaataatgt atataaaagt tgtttaataa atttttgttg 240
aattgagaga aaatataaaa aattgaaagt agtttgtttg taataagtgt ttgttaagtt 300
ttatagattt tttttttttt ttggaattat tgtaataaat atttgatttt tgatttgtag 360
gattaaaatg tgatttttta tattttaata ggagtaagta ttagttttgt aatttttttt 420
gttaatagaa taaaaatgga atgtatgttt gttgttttta tatgttggtt gatatttatg 480
ttgaatattt tttttataat gtagaggttt ttttgttttt tgttttgtgt tttatttttg 540
tttattatag agatattaat gatgttatgt gtttttttgt attattagtg tagtagtttt 600
tattatgttt tttgttttgg ttttattgta gtttaattaa ataaatggaa atgggtagtt 660
attgaaaata aatagtaatt gtaaaagttt tgttttttaa gaaatttttt gtattgtatt 720
ttgttttgtt ttattgattt taagattttt attgtttttt tgtgtttaga aattagattt 780
tttagtttgt tttgttttag ttagataaat gttttataaa aatgatttat attttttaga 840
ttgtggggag agaagaaaaa gattttgaga aagtgattaa ggagggagta agagtgatat 900
aatttttgat ttaatttagt tggattgtgt tttttatgtt tttatttttt tttttataaa 960
aaagatagtg tttttgatgt tttgttttag gtagtttttt agggtgagtg tagatgtgtt 1020
gagttatttg gttgttttga ggattggttt agagagtttt tttttttatt aatgaagtta 1080
tttttttttg gtagtttggt tttttttatt attttatgtt ggaatttggt tttggtttgt 1140
atagttttgt tgtgatgtag atggtttttt agttaagttt tagagaaatt tgtttaatta 1200
tgaataggat atttatagta taggagaaaa aaaaatttat taaaataaat attttattgt 1260
aatattttga tttgggggtt ggagagtaaa aatttaatat ttgatgtttg ggaaaaattt 1320
aatgttttat ggttttttaa ggaaatttgt ttgtgtttaa tttattagag taatttatat 1380
attttgggtt aaagtataat gagattgtta ttattatttt gttttttgta agattttttt 1440

tttaagaata aaaagaaggt aataaagatt ttatattttt gtttttttta gaaagttaaa 1500
gagaatttaa agtgttggat gaaatgtttg tttttaattt gtagtgtttt atttaaaaaa 1560
aaaattatga tataatttta gaggataggt tttttttttt tagggttttt gaaagttaaa 1620
agtatatttt taatttgtag tattattgta ttatttagtg aggataattt gtttagttgt 1680
gtttaggatt agttagatta aggagttgaa attaagtttt ttttgaattt gaatgggatt 1740
ggaattttgt tatttttaga gtagtaatag atgatgttat gtttaagaat attttatttg 1800
attaataaaa aaggagaagt ttgaaatgta attttaaaga atttagaagg gaggtttaga 1860
tgaagataat aaaatttttt ggagatatat ttgttattta tatttttttt ttttaagtta 1920
ttggtttgtt gtttttgagt ttagttaaat ttttaaagat aagattaaag taaggaagta 1980
aggtatatag tttttggttt atggtttttt ttagatataa gtattgtgaa tgaagatttt 2040
gttttattta tatttgggat tttggagttt ttataaagaa tatttttagt ataaggatgt 2100
gatttaaagg gggatttata gaataatatt ttaattattg tttgatttat atttgagtgt 2160
aatggataaa taaatataat tttagttata aaatttgaaa aatatatatt tttttattta 2220
aaaatttttg atagttttgt taggtattgg agaattagaa ataaatgttt ttttattttt 2280
ttttttttga tgaaggaaag ttattaaaaa tagttatata gaaataataa tataattgta 2340
gattttattt taaatatagt tggtatgttt tttttttttt tttttattta ttttttattt 2400
tagatagtta gaggggaaag tttgttttag agatagaaag aagatattta tggggtataa 2460
ttttatttta gttatttgat agtttgaaga agtatttaat tttttgaata ataatgttat 2520
aagttaaaga agagaaaaga taggtagttt aaagtttttt aatattgtgt atattttaat 2580
tttttaagtt agttttgaga aagtgttttt gttttgtaga agtatttggt taagtgttgt 2640
gaagtttatt ggtattaaaa gaaattaaaa attttggagg attagagaat taaatttggt 2700
aatataaatg aaaatatttt agaatgtaaa aagtatttaa tttattattt ttaattatgt 2760
tttattatat atagatggta ttatttatag tgaatgttaa ttattaatat ttttttaaaa 2820
atgggtatta atttgttatt agttgtttgt ttttgattta gttttttttt gttgaaaggt 2880
tttatttaat tgtattataa aggataaaaa gggtaaaaga taataagttt tttagattgg 2940
tagatttgtt atttataaaa tggttttggg taggttattt gaattttttg aaatttaatt 3000
agatttattt aaatttaaga atgagaatat aaatttatat tttgaagtgt tttatagaaa 3060
ggtttatttt aatgtttgga gtatatattt taatgaatat ttattttatt ttattttttt 3120
ttatttttga attaagtaat tttgaattta aagttgttat gattagtatt gaaaagatta 3180
ttggattatt aattgtgtga ttttgggata gtaatttttt gtattttagt ttgtttatat 3240
gttatatatg aaggttgaag tttgattttg ttttgtgatt attattttaa atatttgatg 3300
aaattaaatt ttagtgtttg gaatggtagt ataataaatt tattaagaat aaataattta 3360
ttgtaaaaat atattgattt ttaaatgatg taattgatag ttatattatt gtagagggtt 3420
gataaataat aaaagaaatg aaagatgtat atggtgagaa ttgaaattat tttgataagt 3480
tttttatttg tttattattt aaaattaatg attatgttga atgtttataa attataaaat 3540
ataaaagaaa ttttataaat gtgtatgtat aggagtttaa gttattaaaa gttttaaagt 3600
ataagtttaa attaaattaa ttaaagaagt tgagaggaaa aattggtttt tatttttaat 3660
tattattgtt ttgaggtttt atgtttaata taatttttta agtagaggtt ttagagagaa 3720
gagttgtgag gatattttta tatttgtgta gaaggaaaag tttgttattt attttagtat 3780
ttttagtgtt atattgatgt gtattttgga tttattttgt ttttattgta taaatttata 3840
tttgatttta aagaaaagga aaatttaaag tttttttttt ttaaggggat agaaattttt 3900
tgtgttaatt gtttgatttt tttttttgta aggttttatt ggaaattttt tgtaatataa 3960
tgtaggggat ttttttatgt gttgatgttg tttatatagt ggggtgggtt tgattgaaga 4020
aaaaaaattg tatatatgta tgaaagatta tggttttatt tttggaaagt atgaaaggtg 4080
attgatattt ttaagaagtt tttgttattt aggaaaatta ttaaatattt tatttagaga 4140
tatttggaaa gattgaagga aaggaagaat gaagaaagta gaatttagat ttatgtgggg 4200
agagatttgt ggtagaggaa aagtattttt tttgaatttg ataagggatt tgtttggggg 4260
aattttttgt ttagtttttt attattaggg ttttttgaag ttgggttttt tattgggtag 4320
ttttttggga gtgtagtggg gaatttttat attttttttt taggtttttg aaggattttg 4380
tttttttagt gtttttttta ggttggtagg agttttgagt ttgatatttt tttttgatgg 4440
gataggtaag ttttgtgggt gtgtaaatat gttgtaatta agttttttgt tgattttata 4500
gttttgtgtg tttttgagaa gaagtgattg tatttaattg tttattgttg gtttgttttt 4560
taagagtttg gggggttttt ttttttaatt tagaattagt tgtatggggg gtggggaagt 4620
gggggtgggg aaggagtggg agggtagtgg tttttgtgag tagagtgatg ttattgagtg 4680
agtttttgaa tggggagtgt tgttgttttt aagttgattg gtattttttg ttaggaagaa 4740
atgttaagag gtgggagtgt ttggggaggg aggtaggtgg tttttattgt aggtgtgggg 4800
agttgttttt ttgttttttt gtttgttttt taagtttgga tttttaggag tggttgaagt 4860
tgtggagtgt ttttggagtt tgtgaatgaa tttttttttt tttttttttt tttttttttg 4920
ttgagttttt tttttggttt tgatggtata gtgatataat gatgatgggt gttataattt 4980
gtatttgaat ttgtaggtga gttgttttga gtttttttgg ggaagaattt taggtgtgtg 5040
gatgtaatag ttgagaatat taggtgttgt ggataggagt tgggattaag atttttggtt 5100
agttttgtat ttttttgtat tttttagtat tgttttgtat tttttgtatt tttttttggg 5160
ttattatgtt ttttatgtga tttgtttggg taatgttgaa tttagttgtg tagtgttgta 5220

gtgaattttt tttttaaatt gtaataagtt gttttttaag gtaattatgt tttttttgtt 5280
ttttttttaa aaaataaaaa taaaaaattt atagaaaaaa atttgtgagt ttagaaaaaa 5340
gaagtaattg gtagaaggtt ttaattaagg taaagagttg taaggtgaag ttaagaaaat 5400
gtaggtattt aaaaaatgta ggtaattttt ataaaggttt ttggggagag gtatatagag 5460
ggattttggt gttgaaaaag atttagataa aagaaattta gggtggggtg ggggtaaaat 5520
gattaatgga attgggggaa gggagggaat aaattgtaaa gaaattatag aaaagtggtg 5580
ggtttttgag ttggagagaa gagagggatt tttggtattt tgattttttt gttgttgttg 5640
tttttaatat gtttgaggta aaagtttgaa tggggattat taagatttgt tatagataag 5700
tttttgaagt tgttttggtg taggttattt ggttttttag tttttggtgt gtggttagtg 5760
tttggtgttt ttggaaagtt atttttgggt agtttttgat agtgtgattt ggtgtttaag 5820
tagtttggga ttttgtgtgg atttgatttt tttagattgt aggtagtttg ggaggaggtt 5880
tggttggggg aggtgtagga tttttgttgt gtttttttga tgatttgggg attgttatgg 5940
tttttttttg gtgtttttgg gtttttttgt tttgtatgtg gtgtgaaggg gttagtaggg 6000
aaggagtaga ggatgggggg tggggttgtt ggagttttgt ggaggtttgg gaggtttttg 6060
ggtgggaaaa gtttgttttg aattggtagg gatgtgtaat aatttttttt attttgaaga 6120
gtgaaatagg gtttgttgtt tttattttaa taagtaaatt ggtatttaga gtgtattgta 6180
gataaaggtg gtttggggat ttgaattagg ggtttttggg ttagtttttt tgtttagatt 6240
ataggagttt tttgtttttt tatatatttt ttattttttt ttagtttttt gttttagttg 6300
agtaatttta ttggtaggtt tagtggtagt ggtgtgtgtg tgtgttggga gttttggggg 6360
atgtttttgg ttggagtgtt ttattgtttt gtagaggtgt gggtgttgta gggtgatatt 6420
tttggtgtgt gtagatttag ggtatttggg ttgttttggt ttgtggtttg tgttattggg 6480
tgtggagtgg tttgggtgtt aggtagagga gagtggggta gaaaaatagt gttatagttt 6540
aaattatttg ttttttaatt taattgtgtt ttggtaagtt ttgtatgttt ttgggagatg 6600
ttgtgggaag ggggagaaat ttatatggtg tttggagagt tgttttgttg tgtgtatatt 6660
tgtggtagag tttttttggg ttgtgttttt tatttttgtg tttttttttg tttttatttt 6720
ttttgttttt tttttttttt tttagttttt gttttttttt tttagttttt gtgttttttt 6780
ttttatagtt gataaatgaa tggttagtgt gaaatttttg ttttttttgt gttttaaggt 6840
agtagggagg gaggagtgag ggaggtggtg tgtttttttg gatttgtttt tagtttagtt 6900
tataagattt gtagaattta gatgtttagg aattgggagt tttgtggtgg gtgtgggtgt 6960
tttttgatgg agaagttttg tataggtgga gaaaaataag ttttttagat taagtgtgta 7020
ttttttataa ttttgtgttt aaggatggaa ggttttagtt tttttttaag ttatttattt 7080
tgtattttat aatttgtggt gtatatttag gagtttggag gattttgaaa aaaggttttg 7140
gtttgtgtaa agtgtagaga tgtttttttg tgagttggtt ggaatgtatg tggtgttttg 7200
ggtttagttt gttgtttagt ggatgaatgt atatggttgg ggtggagtta ggttttggag 7260
gtttagatgt gttattttgg gtgtttttaa gaaagataat atatatattg tgtttttaaa 7320
atttatgatt atttgaattt aatggtaggg ttttgtttta ggatattgta aaagaagggt 7380
ttttaattta aaatttaaat ttttttaatt ttagggtggg tgttggatgg gaaagtggag 7440
agaaggtggg tagtgggagg aaaaaagaaa gggaaggaag ggaagtggaa ggaaagagga 7500
ttgtggaggg gaggaaaagg agaaaggaat ggagatgggg tagatggtat ttaaatagtt 7560
aggaatggat tggaagggtt tgttttgggg tttgggtttg aattttgaga tttgttttag 7620
gatatttttt tttttttttt agtggtaata aatgtgattg tagaggtgtg ttgggatgga 7680
agggtgggaa agaattagat aagtggaggt ggtttagttt tgtttaaagg ggttgggttt 7740
ggggtttgtt tttatttgtt tattttgtgt taagggagga tttgtaatgg gtgttttttg 7800
tagtggttgg gttagttagt tgttatgtgg gttggtgttt tggtttttag ttgagatggg 7860
tgtgattttt tgggtttaga gattgtagta tagttgtata ggtggttggt gtagttgagg 7920
tttttattta ttgaggttaa tgggggtggg gtggggaagg tagggtttag tttaattgag 7980
agagttgagt ggttttgttt ttgggatttg gggttggttg tttttggagt tgatttgagt 8040
tttatttaga tagtgttgaa tatttttatt aaaaggttat ttaagtttta ttttatttta 8100
ttttattttt gtttgttttt tgggagtggt aggaggggta ttgtgttttt gttttgaggt 8160
aggggagagg ttaaaattta ttttaagtaa attttttgtt agaaaaatga gattttatat 8220
tgtattttat ttgttgattt tttaaatata tttaggttat tttaaagttt atttttagtt 8280
aattaataga ttaattttgt tttagattgt tataaagagt ttgtttggaa tggtaaatat 8340
tgttatttga taagattaat agaattttat gggggtaaat atggaagggt tgtgagtgta 8400
attgaggttt ttgtgtttta atgaggaatt ttagtgggga tttggtgggg attggaagtt 8460
tagtatttat ttagggagtg attgagttgg aaatttggag aagtttggtg tagatgagat 8520
ggtgtttttt agagttgtat tttaagtttt tttattttgt tgtatattgg aagaattgga 8580
ttttaaagat tttttttatt attttagatt ttgtagttaa tagttatgtt tttttttttt 8640
tgtttgttga aagttgtgat ttaattgttt taagtatttt ttgataattt tgttaaattt 8700
attttgaaaa tatttatata aagttttaaa ataggatgtt gtgagtgtaa tataaaaata 8760
ggaaggtatt aatggaagta ataattagtg ttttgagata gaagtggttt ttagtttgta 8820
atttggaaat gttaagagta gtatattagg gagaaggttt ttaagagttt gattttttgt 8880
tttgagtttt gagtaaagaa agaaatatgt tagaattgta ttgtttgttt gtagtaatat 8940
tgtaaagggt tttaatggat tttaatgtga ttgagtaatt atttttttta gtattaagtt 9000

tatagttttt aatttttttg tgagaagatt aggtaggaag tgggatgaga tttaagataa 9060
ttatttagga tttatgttag aatttaaagt tttgattttt ttggtagata aaaatagagt 9120
tggttttaaa ttgtattttt tgtttttaaa aaaatgaggt ttgaaaggaa tttttttaga 9180
tttatatagg tttatagatg taagtttttt tttttttttt gtgtttgtaa attggaggga 9240
gatgtttttg atattttaga atatttgtat ttatatattt gtaaattttg ttaggaaagg 9300
atttaaatag ttaaattaaa ggtgattatg attaatgtta aagatattat ttattgtttt 9360
tttttttgtt gtattattag gtttaaatat tttggagtta atgattggtt atttaattga 9420
gtaatagagt ttttgagagt gtaaagatta attaattttg ttttttaaag tttggtaatt 9480
aagttttttt tttttggtga gatttaaata tttttgttga tgttatttat ggataaatat 9540
tggtaaatat tgtatttaaa atgggatatt gttttgtgat ggagtagaat tttttaagat 9600
atatggtttt tttgaaagaa agaattaatg attgtagtaa ggtatttttt tattatttat 9660
gtagagaaat tagttaggat tgataaatat ttattgttgg gagttatttg ttttggtttg 9720
agaaaatatg ttgattgtta tataggtttg tattttatta gaaaatataa agtatgtatt 9780
ttttttgatt tggaattaga aaagggggat tagatatgga tgttttggtt aagttttatt 9840
ttaattagtg aatttttgtt agtttttttt tgtagtttag atttggtaaa ttatatatat 9900
ataaagtttg tgtgtgtgtg tggggtgtgt gtgtgtgtgt atgtatatag ggtttttaaa 9960
tattgttaag tttttattat tgaatgtgtt agaggtggtt gtattttaga ggttagtata 10020
aagaattttt gtttaatgtg ttagttttgt tttttgaaat atatttttgg gtagatttat 10080
taattataaa aggatataaa tgtatatatt ttttatattt gtatatattt tattttattt 10140
ttttaaggat tttaagaggt gtttatgttt ataaaatgag attatatatt ttataataaa 10200
tatatttgat aaattataat ttttttatta ttaaattatt gatttgatat tatttgaggt 10260
atagtggaat gagtattaga tttggaatta gaaagtttga gttggattta ttagttgaga 10320
aaattaagta aagatattat ttttggattt ttagtgttat aatttattaa atgaatatgt 10380
agttataaag attaaaagag taagtatttt gaaaaattat ataaaaaagg gatgataata 10440
tattatgttt tgggatttat attgatttaa ttttataatt ttaataataa ttgtattatt 10500
taagttatgt agtttttatt gaatttataa aataaattag agaggggtat attgttttaa 10560
aaattgttta gttttattaa ttagaagata gattatagaa attgatttta atgaagttgg 10620
atatttagtg gaaaggaaga attatttaat aaatggtttt aggataattg atgggtttat 10680
gtaaaataag aattagtttt tatttgattt tttattttaa aagaaatata agatgaatta 10740
aatatgaaat gtagggaata attttttata aaatgataaa agaaaattat gggattaaaa 10800
aaaataagtt aggaatagag aaatttgttt taagtaaaat ataaaattta gtgttatttt 10860
aggaaatatt gataggtttg attaagtaaa aattatataa aagtaaataa tatatttgga 10920
aaatatttat gatatttgag agataaaaag ttaataattt ttgatatttt atggattttt 10980
atgaataatt ttgaaaagtt tatatttatt aaaatatgag taaaaattat gtataggtag 11040
tggtagaaag agatatagag ttttaaaatt atagaaaaaa ttgtttagtt ttatttgtaa 11100
ttaaataaat agtaattaaa atattaatga tatgttatat tattttttta ttaattagag 11160
gagtaataaa aaaaaataat tttttttttt tttttttttt ttgagatgga gtttgttatt 11220
taggttggag tgtagtggta tgattttagt ttattgtaat ttttgttttt taggtttaag 11280
taattttttt gtgttagttt ttttagtagt tgggattatg ggtatttatt attatgtttg 11340
gttaattttt gtgtttttag tagagtgggg ttttattatg ttggttatgg tagttgattt 11400
tgaatttttg attttaagtg atttatttat tttagttttt taaagtgttg ggattatagg 11460
tatgagttat tgtgtttggt aaaaatgaaa ttaaaaagtt tgatgtaagt ttgtgttggt 11520
taggttgtgg agaaattggg ttttttatat attgttgtat gaaagtaaat aggttttttt 11580
attttaaaaa ataatttggt aattattttt ttttgatttt ttattaaaag tttttaaaag 11640
atgtatatat tttttgattt agtaatttta ttttttgata ttattatatg gatggatatt 11700
ggtattaata ttatttaaga taaatggata aggatgtttt ttgtagtatt gtttttaaga 11760
gtaaatattt ggaaatagtg taataggttt attataggga attatttaaa taaaagttgt 11820
tgtatttatt taatggaaaa ttatgtagtt gttaaaaata atatggaaaa attgtatttg 11880
ttgatttgga atatttttta aaatatattg tattttaagt gaaaaaattg atagataaaa 11940
ttgagtatat attatatatt taattatgta aaatgggtgt atatttattt ttttaataaa 12000
tatttattga gtgttttagg tattgtggat ataggagtga ataaaatatt aaagattgtt 12060
ttgttggaat ttttattatg gtaggtagag aaaggtaatt tttaattata tatgtaaaat 12120
ggtgatatgt gttaggatga aaatagagta tgggaaaggg aatagagtat agtatgtttt 12180
aggtagagta atgagaaagg agtttattag gtagagaata gaggagtgtt aaggggtgat 12240
aatgtgaaaa agtttagtta agtttaggtt taggaattta tatgatgtta ttatttgtat 12300
gaagtaaatt tatgaaattt tgtatttaaa ttatgtgatt tttttttgtt gtataatgat 12360
tttttttgtg agaaagaatt agtgagtaat atgatatgtg atgtttatta tatttatttt 12420
gtagttgaag tattttagaa agttggttgt ttagggtatt taattagtag aatggtggat 12480
aaaattattg tgtttttgtt tttaagttat tttttgttag ttgagttttt ttaaaattaa 12540
gggagaatat taaatattta tttaatagaa ttttggtttt tttttggagg ttgtttattt 12600
ttggtgtgtt ttttattttt tttgtagata ttttttatgg ataggagttt tattgatttt 12660
gttttttatg ttttttaggt aattaaatag ttttagtatt ttgtatttgg attgtttaat 12720
gatttttttt tttttttatt gattttattg gagtagaagg ttaaatatag gtatttattt 12780

ttatattttt taggtttttt tatgtagata gagttgttat tattagtaga tggtgtttat 12840
aagagagata tggaaaagtg gaagaatgga aatagggatt tggagtggtg ttaaaaataa 12900
agaaagaggt ttttgaaagt tttttattta atatgattaa atatagaaga taaaagatgt 12960
aatttaatga ttaaggatag ggatatagtt ttgagaaaat tatagaagtg gaatgaatgt 13020
tttggatttt aaagaagagg gaatggaaat gtagttttga ggtagttaaa gttaataaag 13080
ttttttgata tatttattgt agaaataaat gattattttt taatttaagg tttgtaaatt 13140
taggtgatta taggggtgag gtatggagtg gaaatgggta agagattaag tgggggtagt 13200
aatggttttg agtatataga tttaagggga gagattgttg tttggttaat gtttatgatt 13260
agttagttga aatatagatt tagtgttgtt agattttttg attttttgaa agaagttaga 13320
gattttatat ttagtgtaaa tagtttaatt tttaaattga gtaatttatt taaatatatt 13380
tatgtaattt atttaaatgt atttagagta tgtttgattt tttttttgta tttttattta 13440
gtttaggggg taattttaag gaaagtgatg tgtatatttg ttgtatttat gtagatggag 13500
gattattttt ttttttttaa taaaagatat ttttataagg attgagtgtg gtataaagag 13560
tttgattttt agtatttaaa ttgagtagga aatttaaata tagaagattt ttttttgggt 13620
tatatttttg gtttttttaa atgagatttg attttttaga tattgataaa tattatatta 13680
ttgggttatg gtaaattttt aagtggattg atttaaaagt gtttaaaagt attttaggtg 13740
ttaaggaatt gttggtatgg agttttaaag gtgtttttgt ataagaaata gaggtgttga 13800
ttatgtattt gatttataaa tattttttaa ggatatattt tgaatttggt aatgaagaaa 13860
tattgtggga attattaagt tgaataaagt attagttttg ttttttagaa gtttattgtt 13920
ttatagagga gattagatat gtttatagat aatttgaagt atatagtaat aaattatagg 13980
atgatttatt ttaagtataa agtgtatatt gttattatag tggtaattta ggttataatt 14040
tttaaatgta agagttttaa atattatttt ttttattttt agtgatatga aattatgtta 14100
gaatatttgt agaaagtttg aattagtaat tttaataata aatattgagt attgatatat 14160
tgtatattat tgagatttat tttatatata ttattttatt aatttttatg attattttgg 14220
taagtattaa tttttataat taagtagtga tagaaaatga ggtttagaga gatatagtat 14280
tttatttaaa gttatagaat ataagtagag aatgtagaat ttgtattttg ggttatttga 14340
tagtggagtt tagattttta aaattaggtt tataaagatt ttttggaaaa aaagaaaaat 14400
aaaaaataga gaataaattt ttttgaagaa aagtaggaaa ttattattta taagtataat 14460
taaagagaga tttttaattt tttttttttt tttttttttg agatggagtt ttgttttgtt 14520
gtttaggtgt aattttagtt tattgtaatt tttatttttt gggtttatgt tataaagaaa 14580
gtttttattt gattggtttg atttgattgt ttattagaat ataggttttt tagtagggtt 14640
aataagttat aatagtttta gtgatataag ataaatataa attaataata aggttaattt 14700
agaattatta atatatgttt tttattgtga attattagtt ggtttataga tggaaaatat 14760
aagatatata atgtattagt ttaaaaaatg ttttttagag agtgtttaga attatagaat 14820
ttaaaaattt tgttttgaaa aggtttaatt tttattttaa aatatataaa gaaattaaat 14880
gtaaaaaaat taagagaaaa ggttttaatt aattaaatgt tgtataattt taagttttat 14940
ttatgtttaa gattagtttt gtaatttatg attaatttta gtattagttt gtttttaatt 15000
taggtttgtt aaataatata gaattttttt tttaatgaag ttttttaggg ttttagttga 15060
aaaatatata ttagttagga tgtttttaat tgtaaggaat aaaaagttat gattttattg 15120
ggtttaaata ataatgatat tttataattt atataaaatg aagtttaaag gtatggttta 15180
ttttaggttt aatttttaag gatttatgtt ttgtttttgt tgattttttt ttagttttat 15240
ttttagaggt attttattta tttatggatg taaaattttt ataatagttt tagtagaaag 15300
attttagtat gataatgttt agggaagaaa attgattatt tttttttttg tttttttttt 15360
aggatgaatt tatttttttt agggattttt tattggagga agagattttt ttatatttta 15420
ttggttaaaa ttatattata tattgtttta aatttaatta ttggaaaagg gaatggtatt 15480
tttataattg atttaggata ttaggattat tttttgggtt ggggtttgat ttaggatttt 15540
ttaaagtatg tgattgaggg taggtattag aatggaatta gagttgtgtt agaaaggaga 15600
aatgtatggt tattatagaa tagttttaaa tgttattgag gagggtataa ttgtaaaaat 15660
taaataattt tttgttattt ttttgaagtt ggtattttga tttttagatg gttttttaat 15720
ataggttttt ttttttttta ttattatagt tgttttgaaa tttgagttgg aagggaatat 15780
tttgagattt agattgttaa atgttttttt taaagttatg taataaatta aatggtaaag 15840
ttagggtagt tttttgattt agtatagggt attttttttt atttttattt tttgagattt 15900
tagaattgtt ggtattgttt taaaatttat ggtaagaatt ggttattttt gtaattaata 15960
tttttttata atatatttgt tttgtttgtt tagttagtta gaaattatat ggagtttgtg 16020
ttttaaaaag tttgttgaag tttttatttt ttgttttggt attatgtgta tgaattatta 16080
attggttttt ttttatttta tatttgatga agatgttttt tttttaatat ttttttttat 16140
tgttttttat ttttttttgt tttatttatg attagttgtt tgtttttaaa tagattttgt 16200
ggttatttat ttttttttgt gttagttttt atttattagt tatttgaatt gtggttttta 16260
ttgtttttta tatagtttat ttatttgtgg ttgttatata tatttatatt gttatatgtt 16320
tttaaattgt attttggaat gattttggta atggttgtat tggatgagat ttaaattaat 16380
aattaaagta ttgagatagt ttttgttatt ataagtttat ttttgttttt atagtttaag 16440
aggagttatt ttttttattt ttattattta atgtttaata ttatttttat tatatataat 16500
gtataaaaag tatgtgattt atgatttatt ttaaatttga atgtttgtga tttattttgt 16560

gtttttttt attttagg                                                16579

<210> 302
<211> 16579
<212> DNA
<213> Artificial Sequence

<220>
<223> chemically treated genomic DNA (Homo sapiens)

<400> 302

tttagaaatg aagaaaaata taaagtaaat tataagtatt taggtttaaa gtgggttata      60
aattatatgt tttttgtgta ttgtatgtaa taagggtagt attaagtatt aagtaataag     120
agtagaagaa gtagtttttt ttggattata aaaataggag taaatttgta atagtagaag     180
ttattttaat gttttaatta ttagtttgaa ttttatttaa tgtagttatt attaaaatta     240
ttttagaata tagtttggga atatataata atgtggatgt gtgtgataat tataagtgag     300
taggttgtat gaagaataat agagattata gtttaagtaa ttagtggata ggagttggta     360
taaaaagaaa tgggtgatta taaagtttat ttagaaatag atagttgatt ataaatagag     420
taagagaaga tggggagtaa tagagaaaga tgttgaaaga aaggtatttt tattaagtgt     480
aaggtgagaa ggaattaatt ggtggtttat gtatataata ttaaaataga gaataaggat     540
tttggtaggt tttttaaagt atagatttta tgtagttttt agttggttaa gtaaataaaa     600
taaatgtatt ataaggaggt gttaattata aaagtgatta gtttttgtta tgaattttaa     660
agtagtatta atagttttaa agttttaaga gtaaagaatg aaagaaaata ttttgtattg     720
agttaagaaa ttgttttggt tttgttattt aatttattgt atgattttgg aaaagatatt     780
taataatttg ggttttagaa tgtttttttt tagtttaaat tttaaggtag ttataatgat     840
gaggaggaga agaatttgtg ttggggaatt atttagaaat taaagtgtta attttaaaag     900
aatggtaaaa gattatttgg tttttatagt tgtgtttttt ttggtagtat ttagaattat     960
tttataatag ttatgtgttt ttttttttta atataatttt ggttttattt tagtgtttat    1020
ttttagttat atattttgga gaattttggg ttaaatttta gtttaaggga tgattttgat    1080
attttaagtt aattatagag gtattatttt tttttttaat gattggattt ggaatagtat    1140
ataatatagt tttggttagt gagatgtgag gggatttttt tttttagtga agggtttttg    1200
ggaaagatag atttattttg agaagagaat aaaggaagag gtggttagtt tttttttttg    1260
gatattatta tgttggaatt tttttgttgg aattgttgta gggattttgt atttatgagt    1320
aagtgagata tttttggaaa tgaagttgag aaagaattag taaaggtaag gtgtaagttt    1380
ttaaggatta gatttagagt ggattatgtt tttgaatttt attttatatg agattataaa    1440
tgttattatt gtttaaattt aataaaattg tgattttttg ttttttatag ttgaaagtat    1500
tttaattggt atatattttt tagttgggat tttgaaaaat tttattggaa aagaaatttt    1560
atattattta gtaaatttag attgaaaata agttaatgtt gaaattagtt ataaattata    1620
aaattgattt tgaatataaa tagggtttgg agttgtatag tatttaatta attaagattt    1680
ttttttttaa tttttttgta tttgattttt ttatatattt tgaaataaga attagatttt    1740
tttaaaataa aatttttaaa ttttataatt ttagatattt tttgaagagt attttttaag    1800
ttaatatatt atatatttta tattttttat ttatagatta attgataatt tataatgaaa    1860
aatatatgtt gataatttta aattaatttt attattagtt tgtatttatt ttatattatt    1920
agaattgtta tgatttgtta attttattgg aaagtttatg ttttagtaga taattaggtt    1980
ggattaatta aatagaaatt ttttttatgg tatgaatttg ggaggtggag gttgtagtga    2040
gttgagattg tgtttgggtg atagagtgag attttgtttt aaaaaaaaaa aaaaaaaaga    2100
aattaaagat ttttttttaa ttatatttat aaataataat tttttatttt tttttaggaa    2160
aatttgtttt ttatttttta tttttttttt tttttaggaa atttttataa gtttaatttt    2220
aaaagtttgg gttttattgt taaatagttt agaatgtaag ttttgtattt tttatttata    2280
ttttgtgatt ttgggtaaaa tattatattt ttttaaattt tattttttgt tattatttag    2340
ttgtgaaaat tagtatttat tgaagtgatt ataggaattg ataaaataat gtatataaaa    2400
taaattttag tgatatataa tatattagta tttaatattt attattaaaa ttattaattt    2460
agattttttg tagatgtttt aatatagttt tatattatta agagtaaaaa gaatagtatt    2520
tggggttttt atatttgaaa attataattt aggttattat tatagtagta atatatattt    2580
tatgtttgga ataaattatt ttataattta ttgttatgta ttttaaatta tttgtaaata    2640
tgtttaattt tttttgtaga atagtaggtt tttggaaggt agagttagta ttttatttaa    2700
tttaataatt tttataatat ttttttattg ttaggtttag ggtatatttt tgaggaatgt    2760
ttgtagatta aatatatgat taatgttttt attttttatg tagaaatatt tttgaaattt    2820
tgtgttaata attttttaat atttgaaata tttttgaata tttttgaatt agtttatttg    2880
aagatttatt atgatttaat aatatgatgt ttgttaatat ttagagagtt aaattttatt    2940
taaaaaaatt aaaggtgtga tttaggaaag gattttttgt atttgagttt tttatttaat    3000
ttaaatatta aaaattgaat tttttgtgtt atatttaatt tttataaaag tgttttttgt    3060

tggggagagg agaatggttt tttatttata taggtataat aagtatatgt attatttttt 3120
ttaagattat ttttaagatt aggtaagaat ataaggagga gattagatat gttttaaata 3180
tatttgaata agttgtataa atatgtttga ataagttatt taatttaaaa attagattgt 3240
ttatattaaa tataaaattt ttaatttttt ttaaaaaatt agaaggttta gtaatattaa 3300
gtttatattt tagttggttg attatgagta ttaattaagt agtagttttt ttttttaagt 3360
ttgtgtgttt aaggttatta ttgtttttat ttagtttttt atttattttt attttatgtt 3420
ttgtttttgt agttatttaa gtttgtaaat tttgaattaa aagatgatta tttattttta 3480
tagtaaatat attagagggt tttattgatt ttagttgttt tagaattgta tttttatttt 3540
tttttttttg gagtttaaga tgtttatttt atttttgtaa tttttttaag gttatgtttt 3600
tatttttggt tattaagtta tattttttgt tttttatgtt tgattatatt aaatggaaag 3660
tttttagaag tttttttttt tgtttttagt attattttaa atttttattt ttattttttt 3720
atttttttat gtttttttta taagtattgt ttattagtga taataatttt gtttgtatgg 3780
aaagatttga gagatatgag agtgaatgtt tatatttagt tttttatttt agtgaagtta 3840
atgagaaggg aagaaagtta ttgaataatt taggtatagg gtattgagat tgtttaattg 3900
tttgggagat ataaaagata aggttaatgg aatttttatt tatgagaaat gtttgtaggg 3960
aggataaggg atatattagg gatgggtagt ttttgagaga aagttaagat tttattaaat 4020
agatatttag tatttttttt tgattttggg gaagtttagt tgataagggg tgatttaagg 4080
atagagatat ggtggttttg tttattattt tgttaattag gtattttaga taattaattt 4140
tttaggatat tttagttata aagtggatgt aatgagtatt atatattata ttgtttattg 4200
gttttttttt atagaaagaa ttattgtata gtagagagaa attatgtgat ttaggtgtga 4260
aattttatga atttatttta tataagtaat agtattatgt gaatttttga atttggattt 4320
ggttaagttt ttttatattg ttgtttttta gtattttttt gttttttgtt tggtgaattt 4380
tttttttatt attttattta agatatattg tattttattt ttttttttat gttttatttt 4440
tattttagta tatattatta ttttgtatat gtgattggga attgtttttt tttatttatt 4500
atagtaaaag ttttaataga gtagtttttg atgttttgtt tatttttgta tttataatgt 4560
ttagaatatt tagtaaatat ttgttaaaag aatgaatata tatttatttt atataattag 4620
atgtataata tatatttagt tttatttatt aattttttta tttagaatat aatatatttt 4680
ggagaatgtt ttaaattagt aaatataatt tttttatgtt atttttgata gttgtatagt 4740
tttttattag gtggatatag taatttttat ttaaatggtt ttttatgatg agtttgttat 4800
attgttttta gatatttgtt tttagaagta atgttgtaaa gaatattttt gtttatttgt 4860
tttgggtgat attggtatta atatttattt atataataat gttgagaagt ggaattgttg 4920
aattaaagag tatatatatt ttttaaaaat ttttgataaa gaattaaaaa aaagtgattg 4980
ttaaattatt ttttgaagta gaggaattta tttattttta tataatagta tatgagagat 5040
ttagtttttt tataatttgg ttaatataaa tttgtattaa attttttaat tttatttttg 5100
ttaggtatag tggtttatgt ttgtaatttt agtattttgg gaggttgagg tgggtgaatt 5160
atttgaggtt aggagtttaa gattaattgt tatggttaat atggtgaaat tttattttat 5220
taaaaatatg aaaattagtt aagtatggtg gtgggtgttt ataattttag ttattaggga 5280
ggttgatata ggagaattgt ttgaatttgg aaggtggagg ttgtagtgag ttgagattgt 5340
gttattgtat tttaatttga gtaatagatt ttattttaaa aaaaaaaaaa aagaaaaaaa 5400
ttattttttt ttgttatttt tttgattggt gaaaaaatgg tgtagtatat tattggtgtt 5460
ttaattatta tttatttaat tatagatgag gttgagtgat tttttttata attttaaaat 5520
tttatgtttt tttttgttat tatttgtata taatttttgt ttatattttg atagatgtgg 5580
attttttaaa gttatttata aaagtttatg gaatattaag agttattagt tttttgtttt 5640
ttaaatgttg tagatgtttt ttaaatatat tatttgtttt tgtgtaattt ttatttagtt 5700
agatttatta gtatttttta gaatggtatt gggttttatg ttttgtttag aataggtttt 5760
tttattttta atttattttt tttaatttta tgattttttt ttattatttt gtaaaaagtt 5820
attttttata ttttatattt gatttatttt atattttttt taggatgaga aattaggtag 5880
agattgattt ttattttgta tggatttatt aattgttttg aaattattta ttgaataatt 5940
tttttttttt attgaatgtt taattttatt ggagttaatt tttgtaattt gttttttgat 6000
taatgaaatt aaataatttt taaaataata tgtttttttt tgatttattt tatagattta 6060
gtagaaatta tatggtttgg gtaatgtagt tattattaaa attatagaat tagattaata 6120
tgaattttaa gatataatat attattattt tttttttgta tagtttttta aagtatttat 6180
ttttttgatt tttataattg tatatttatt taatagatta tgatattaaa ggtttaaaag 6240
taatattttt gtttggtttt tttagttagt gagtttaatt taaatttttt gattttaagt 6300
ttagtgttta ttttattata ttttaagtaa tattaaatta ataatttaat aataaaaaga 6360
ttataattta ttaaatatgt ttattgtaaa atatgtaatt ttattttata gatataaata 6420
ttttttaaaa tttttagaag aatgaggtag gatgtatata gatgtggaaa gtatatatat 6480
ttgtattttt ttatagttgg taaatttatt tagaggtgtg ttttaggaaa tagaattgat 6540
atattaaata aagatttttt gtattgattt ttgaaatata attattttta atatatttaa 6600
tggtaagaat ttagtagtgt ttaaaaattt tgtatatata tatatatata tgtattttat 6660
atatatatat agattttata tatatgtaat ttattaagtt taggttataa gaggaaattg 6720
gtaaaaattt attaattgaa atggagtttg gttaagatat ttatatttaa tttttttttt 6780
ttgattttaa attagggaga gtatatgttt tgtgtttttt ggtaaaatat agatttatgt 6840

aataattagt atattttttt agattagagt aagtaatttt taataatgag tgtttgttag 6900
ttttaattgg tttttttatg tgggtggtga aaaaatattt tattatagtt attaattttt 6960
ttttttaaaa gaattatgtg ttttgagaag ttttgtttta ttatagggta atattttatt 7020
ttaaatatag tgtttattga tgtttgttta tgaatgatgt taataaaaat gtttaaattt 7080
tattaggaaa ggaggtttta gttgttagat tttgggagat agagttagtt gatttttgtg 7140
tttttaaagg ttttgttatt taattaagtg attaattatt aattttaaag tatttaagtt 7200
taataatata ataaaagaga gaataatggg tagtattttt aatattaatt atggttattt 7260
ttggtttagt tgtttaggtt tttttttagt agaatttata agtgtataga tataggtatt 7320
ttggaatatt aaaggtattt ttttttagtt tgtaaatata gggaaaaaga agaaatttat 7380
atttgtaaat ttatgtaaat ttagggggat tttttttaaa ttttgttttt ttgaaagtag 7440
gaaatataat ttaaaattaa ttttgttttt atttattaaa agaattaaaa ttttgaattt 7500
tgatataaat tttaaatgat tgttttgaat tttattttgt tttttattta atttttttat 7560
aagaagatta aggattgtgg atttggtgtt ggaggaagta attgtttaat tatattggaa 7620
tttattggaa ttttttataa tattattgtg ggtagataat ataattttaa tatatttttt 7680
tttttattta gagtttagaa taagaagtta gatttttgga gatttttttt ttgatgtatt 7740
gtttttaata tttttaagtt ataaattggg aattgttttt attttagaat attaattgtt 7800
gtttttattg gtattttttt atttttatat tatatttata atattttatt ttaggatttt 7860
atgtgaatat ttttaggatg ggtttggtga aattgttaag aagtgtttaa aataattagg 7920
ttatagtttt tagtagataa aaagaaaaag atgtggttgt tagttataga atttgaggtg 7980
atggaaaaga tttttaaaat ttagtttttt taatatgtag taaaatggga ggatttaaaa 8040
tgtagtttta agaagtattg ttttgtttgt attaagtttt tttaggtttt tagtttagtt 8100
atttttaaa taggtgttga gttttagtt tttgttaggt ttttgttaag gtttttattt 8160
gaggtatagg aattttagtt atatttgtag tttttttatg tttgttttta tgaaatttta 8220
ttagttttgt tgaatggtag tatttgttat tttaggtgaa ttttttatga tagtttggaa 8280
tgaaattaat ttgttaattg attaagaatg ggttttagag tgatttgaat gtatttaaaa 8340
agttaataaa tggaatatgg tataaagttt tatttttttg ataaaaagtt tgtttggagt 8400
aaattttaat tttttttttg ttttaaaata aaagtatgat gtttttttta ttatttttaa 8460
gaagtaagta aaaataaaat aaaataaaat aaaatttaaa tagtttttta ataagagtgt 8520
ttagtgttgt ttggatggaa tttgggttag ttttagaagt agttggtttt gaattttggg 8580
ggtgaggtta tttggttttt ttggttaagt tgggttttgt tttttttgtt ttgtttttat 8640
tggttttggt gggtgggggt tttggttgtg ttggttgttt gtgtggttgt attgtagttt 8700
ttgagtttag gaagttatat ttgttttggt tgggagttga agtgttaatt tatgtggtgg 8760
ttggttaatt tagttgttgt ggggagtgtt tgttgtgggt ttttttttgg tgtgaggtga 8820
gtgagtgggg gtgggtttta gatttagttt ttttgggtgg ggttggatta tttttgtttg 8880
tttagttttt ttttattttt ttattttggt atgtttttgt agttatattt gttgttgttg 8940
ggaaagggaa aagagtattt taaaatgagt tttgaaattt agatttaaat tttgaaatga 9000
atttttttgg tttgtttttg gttgtttgaa tgttatttgt tttatttttg tttttttttt 9060
tttttttttt tttttatggt tttttttttt tttatttttt tttttttttt tttttttttt 9120
tttttattgt ttgttttttt tttatttttt tgtttgatgt ttgttttgaa gttgaggaag 9180
tttaagtttt ggattaggag tttttttttt gtaatatttt ggggtggggt tttattgttg 9240
ggtttaagtg gttatgggtt ttgagagtat ggtatgtgta ttattttttt taaagatgtt 9300
tagggtggtg tgtttagatt tttgaaattt ggttttattt tggttatgtg tgtttgttta 9360
ttaagtggtg aattggattt agggtgttgt gtgtgtttta gttgatttgt gagaaggtat 9420
ttttgtattt tgtatagatt aagatttttt tttagggttt tttaaatttt tagatgtatg 9480
ttgtagattg tggagtgtaa aatgaatgat ttaagggaaa attagggttt tttatttttg 9540
aatataaggt tgtgaaagat gtatgtttgg tttggggggt ttgttttttt ttgtttgtgt 9600
gggatttttt tattaggggg tgtttatatt tgttgtaaaa tttttaattt ttagatattt 9660
aggttttgta agttttgtga gttgaattgg gggtagattt gaaggagtgt attgtttttt 9720
ttgttttttt ttttttgttg ttttggagta tgggaaaggt agggatttta tattagttat 9780
ttatttgtta gttgtggggg aggggatgta gaagttagag agagagaata gaggttggga 9840
gaggggaagg gaggtagaga agatggagat agagggagat ataaagatga ggaatgtgat 9900
ttagagagat tttgttgtgg gtgtgtgtgt ggtaggataa ttttttaggt gttgtgtaga 9960
tttttttttt tttttgtagt attttttagg gatatgtagg atttgttgag atgtagttgg 10020
attggagagt aaatagttta ggttatgata ttattttttt attttgtttt tttttgttta 10080
atatttagat tgttttatgt ttagtgatat agattgtggg ttagaataat ttggatgttt 10140
taggtttgta tgtattaggg atgttgtttt atagtgttta tgtttttgta ggatggtggg 10200
gtgttttagt tgaggatgtt ttttggggtt tttggtatat gtgtgtgttg ttgttgttag 10260
atttgttagt aaagttgttt aattagagtg gagaattagg agagggtggg gaatatgtaa 10320
ggaaatggag gatttttgta gtttgggtga gaggattgat ttagaggttt ttgatttggg 10380
tttttgagtt atttttgttt gtagtgtgtt ttgggtgttg gtttgtttgt tgagatggga 10440
gtgatagatt ttattttatt ttttagggtg agaaaaattg ttgtatattt ttgttgattt 10500
agaataggtt ttttttgttt aggggttttt tagatttttg tggggtttta ataattttat 10560
tttttatttt ttgttttttt tttgttggtt tttttgtatt gtgtgtagga taaaagaatt 10620

taggggtgtt gggagagaat tgtgatagtt tttaagttgt taaagagata tggtggggat 10680
tttgtatttt ttttggttgg attttttttt agattgtttg tggtttgaag gggttaggtt 10740
tgtgtaaggt tttaggttgt ttgggtgttg ggttgtattg ttaggagttg tttgggagtg 10800
gttttttagg aatattaggt attgattata tattaggggt tgggaaatta ggtggtttgt 10860
attaaggtgg ttttggggat ttgtttgtgg taagttttgg tagtttttat ttaaattttt 10920
gttttgagtg tgttaagaat aataataata aaaaaattaa agtgttaaag gttttttttt 10980
tttttttagt ttaagaattt attatttttt tatgattttt ttataattta tttttttttt 11040
ttttttaatt ttgttagtta ttttattttt attttatttt gggttttttt tgtttgaatt 11100
ttttttaata ttaaggtttt tttgtatgtt tttttttaaa agtttttatg aaagttattt 11160
gtatttttta agtgtttata tttttttaat tttgttttat agttttttgt tttaattaaa 11220
gttttttatt aattgttttt tttttttaag tttgtgggtt tttttttata agttttttgt 11280
ttttgttttt taagggggga ataaaagaaa tgtgattatt ttggaaggtg gtttattgta 11340
gtttgggggg aaaatttatt gtagtgttgt gtgattgggt ttggtgttgt ttaggtgggt 11400
tatataggaa gtgtggtggt ttggggaagg atgtggaggg tgtgggatgg tgttggaaga 11460
tgtgggagga tgtggggttg gttgaagatt ttggttttag tttttgttta taatatttaa 11520
tgtttttggt tgttgtgttt gtatgtttgg agtttttttt tagaaaggtt tggggtagtt 11580
tgtttgtaag tttaaatgtg ggttgtgata tttattatta ttatattatt gtattgttag 11640
agttgaggag gagatttagt gagaagaagg aggagggaga ggaggagggt ttatttatag 11700
gttttaaaag tgttttgtag ttttagttat ttttaagagt ttaggtttgg aaagtaggtg 11760
gaggggtgga aaggtagttt tttgtgtttg tggtagggat tgtttgtttt tttttttagg 11820
tatttttatt ttttggtgtt tttttttgat aagaagtatt aattggtttg gggatagtag 11880
tgttttttat ttagggattt atttagtaat attgttttgt ttgtggaaat tattgttttt 11940
ttattttttt tttattttta tttttttgtt ttttgtgtag ttagttttgg gttaggggaa 12000
aggagttttt aggtttttag ggggtaggtt agtaatagat aattgagtat gattattttt 12060
ttttgggagt atataaaatt gtaaaattag taaagaattt ggttatagtg tgtttatgtg 12120
tttatagagt ttgtttgttt tattaaaggg aagtgttagg tttaaggttt ttgttaattt 12180
gaaagagata ttgagaaaat gagatttttt ggggatttag agggaaagtg taagaatttt 12240
ttattgtatt tttagggaat tgtttaatgg ggagtttggt tttaaaagat tttggtaata 12300
aaaggttgga taggaaattt ttttaggtaa attttttgtt ggatttaaag agaatatttt 12360
ttttttgtta taaatttttt tttatataag tttagatttt gttttttttg tttttttttt 12420
tttttagttt ttttaagtat ttttgagtag aatatttgat aatttttttg agtaataggg 12480
atttttttgga agtattaatt atttttttatg tttttttggaa ataagattat aatttttttat 12540
gtgtatatgt gatttttttt ttttagttag gtttatttta ttgtgtaaat agtattaata 12600
tatggaagag ttttttgtat tgtgttataa aagattttta ataggatttt atagagaaaa 12660
gggttaaata gttgatataa aggatttttg tttttttaga aaagagggat tttggatttt 12720
tttttttttt gaagttaagt atgagtttat ataataggaa taaaataaat ttaaggtgta 12780
tattagtata atattaggga tattagaatg gatggtaaat tttttttttt atataaatat 12840
gaaagtattt ttataatttt tttttttgaa gtttttattt agaaaattat attaaatata 12900
ggattttaaa atagtagtga ttaaagatga aagttaattt ttttttttaa tttttttgat 12960
tagtttggtt taaatttatg ttttaaaatt tttagtaatt tagatttttg tatatgtgta 13020
tttataagat tttttttata ttttgtaatt tgtaggtatt tagtatggtt attgatttta 13080
agtgataaat aggtagaaga tttgttagga taattttagt ttttattatg tgtatttttt 13140
attttttttg ttatttatta gttttttgta gtaatataat tgttagttat attatttgga 13200
aattaatgtg tttttgtagt gaattattta tttttagtaa atttattgta ttattatttt 13260
aaatattgaa atttgatttt attagatgtt tagaatgata gttatagagt agaattagat 13320
tttaattttt atgtataata tgtaaataaa ttaaggtgta gaaagttatt gttttaaagt 13380
tatataatta atagtttagt ggttttttta gtgttaatta tagtaatttt agatttaaga 13440
ttgtttgatt taggaatgga gagaaataaa ataaaatgaa tgtttattga aatatatatt 13500
ttagatatta agatagattt ttttgtgaaa tattttaaga tgtggatttg tatttttatt 13560
tttaaatttg agtaaattta gttgagtttt aaagagttta agtaatttgt ttaaggttat 13620
tttgtaagtg atagatttgt tagtttgaaa agtttattat tttttatttt tttgtttttt 13680
tatgatatag ttaagtaaaa ttttttaata aaggagaatt agattagaaa taagtaatta 13740
atagtagatt aatgtttatt tttagggaaa tgttaatagt taatatttat tataaataat 13800
gttatttgta tataatggaa tatgattgaa agtgatgggt tagatatttt ttatattttg 13860
agatgttttt atttatgttg ttaaatttga ttttttaatt ttttaaaatt tttagttttt 13920
tttaatgtta atagatttta taatatttga ttaaatattt ttataggata aaggtatttt 13980
tttagggttg atttaaggaa ttagaatatg tataatgttg aaaagtttta aattgtttgt 14040
tttttttttt ttttgatttg taatattatt atttagaaaa ttaaatattt ttttaggtta 14100
ttagataatt aaaatgaaat tatattttat ggatattttt tttttatttt tagggtagat 14160
tttttttttt agttgtttaa aatggagaat ggataaaaaa gaaaaaggaa aatatattag 14220
ttatatttag aatagagttt atagttgtat tattattttt atgtaattgt ttttaatgat 14280
ttttttttat taagagaaag aaaatgagag agtatttatt tttgattttt taatgtttga 14340
taaaattatt agaagttttt gaatgggaaa atatatattt tttaggtttt ataattaaaa 14400

ttgtatttat ttgtttatta tatttaagta tgaattaaat agtaattaaa atattatttt 14460
gtgagttttt ttttaaatta tatttttgta ttggggatat tttttgtgaa aattttaaga 14520
ttttaggtat aaataaagta agatttttat ttataatgtt tgtgtttaaa agaaattata 14580
agttagaagt tatgtgtttt gtttttttgt tttgattttg tttttgggaa tttggttgag 14640
tttaggggta gtaagttaat gatttggaga ggaaaggtgt agatggtaga tgtgttttta 14700
gggaatttta ttatttttat ttaaattttt ttttgaggtt ttttaggatt atattttaga 14760
tttttttttt tttattgatt aggtgaagtg tttttagata tggtattatt tattgttgtt 14820
ttagggatga taagatttta gttttattta aatttagaaa gagtttagtt ttaatttttt 14880
gatttgattg gttttaggta taattaagta aattattttt attggatgat atagtaatgt 14940
tatagattaa aaatgtattt ttaattttta aagattttgg aaaaaggaag tttatttttt 15000
aagattgtgt tataattttt tttttaaata aaatattgta aattagggat agatatttta 15060
tttagtattt tgaatttttt ttggtttttt aggaaaggta gaaatataag gtttttattg 15120
tttttttttt atttttaaaa aagaaatttt gtaaaaggta aagtgatggt ggtaatttta 15180
ttgtgtttta atttgaagta tataaattgt tttgataaat taaatataga taaatttttt 15240
tagaaagtta taaaatatta gatttttttt agatattaga tattgagttt ttgtttttta 15300
atttttaaat taaaatgtta tagtaaaata tttgttttaa taaatttttt ttttttttgt 15360
gttgtaaatg ttttgtttat ggttgagtag atttttttag aatttaattg aaagattatt 15420
tatattatga tagaattatg taagttaaag ttaagtttta atatggagtg atgggagagg 15480
ttgggttgtt gagaggagat gattttatta gtgagaagag aggttttttg agttggtttt 15540
taaggtagtt aaatggttta gtatatttgt atttattttg ggaggttgtt tggagtagag 15600
tattaaaaat gttatttttt ttataaagaa aaagatgaag gtataaagaa tatagtttaa 15660
ttaaattgaa ttaaagattg tattattttt attttttttt tagttatttt tttaaagttt 15720
tttttttttt tttttatagt ttgaaaaata taggttattt ttataaagta tttgtttgat 15780
tgaagtaaga taaattgaag agtttggttt ttgaatataa ggaagtggta gagattttag 15840
aattaatgag gtagggtgga atataatgta gaaggttttt taagaggtaa aatttttgta 15900
gttgttgttt atttttagtg attatttgtt tttatttatt taattagatt atagtgggat 15960
tgagataagg agtataatga aagttgttgt attgatggtg taaaaagata tatggtatta 16020
ttaatgtttt tgtggtgagt aaaggtagaa tgtaagatag aaaataagga aatttttgtg 16080
ttgtaagaaa agtgtttagt atgggtatta attggtatgt agaagtagta agtatatatt 16140
ttatttttat tttattgata aaaagaattg taggattaat gtttgttttt attaaaatat 16200
gagaaattat attttaattt tataaattaa aggttaaata tttgttgtaa taattttaaa 16260
aaaaagagaa gatttgtaag atttagtaaa tatttattat aaatagattg tttttaattt 16320
tttatatttt tttttaattt aataaaagtt tattgagtag tttttatgtg tattgtttta 16380
gatagtaggt attatttaaa aaagtaataa ttatttttta tttttaagga gtttataatg 16440
tttttagaaa gaaattaatt tattggagta gttaaagtta taattgattt ataaaatgat 16500
tagtgaattt agaggaaaat tatatataaa tatttttgtt tttattggtt ggtttagtta 16560
gatattattt taaaaaata 16579

<210> 303
<211> 6090
<212> DNA
<213> Artificial Sequence

<220>
<223> chemically treated genomic DNA (Homo sapiens)

<400> 303

tagagatgga atgtttaaat taggataatt tagggaggtg tgggagaggt gtagggggat 60
tataagggat ggagtaggaa tttaaggtag taatagtaga gttggtatta attttaggtt 120
taaaaagata aaggagagag agtggttatt aaaatttaga aagagaaaat ttggtaaaag 180
atgggatttg gtgaggagta gtaatttgtg tttggttgtg gattgtggta attttgtagg 240
gaggagagta gaggaatgat tattttggtt ttattttttt tttttttttt ttattttatg 300
ttaggtgttt tattggttaa atttattagt aattagagga taaggaatgt attaaaataa 360
ttttatggtt tagagagtag attagatgta gagtgataaa tggattatat ttagtataat 420
tgtaggttta tttgttgatt tgtttttttt attagattga gttatttgag gatagagaat 480
gtttttttta tatttgtatt tttaatattt ggtggatatt agatatttaa gaaatgttta 540
tggatgggat tagagaagat ggtaaattgg gtattagatg gaggtttgtg ttttttgagt 600
ttgttgtatg ttttttggtg aatttaaggt aaaatgagaa atagaatgag ttttgtattt 660
ttgtttggta aaagaaaata tgatttggtg agaagaaatg aatattttta gtatttagtg 720
ttgggaggaa ttggaattag ttatgtgatt gttttattta gaggttttta attgtgtaat 780
ggatagaatt tttaatggtg aaattataat gtagaaatat tttttttttt tttttttttt 840
ttttttttga gatggagttt tgttttgtta tttaggttgg agtgtagtgg tattattttg 900

gtttattgta agttttgttt tttgggttta ttttattttt ttgttttagt tttttaagta 960
gttgggatta taggtatttg ttattatatt tggttaattt tttgtatttt tagtagaaat 1020
ggggttttat tgtgttagtt aggatgtttt tgattttttg attttgtgat ttgtttgttt 1080
tggtttttta aagtgggaaa tatttttaaa aatattagtt tttgataaaa ttaaagttat 1140
gttataaaaa agtaattaaa aggtggaaat gagataatat ggtttatttt gagttgagaa 1200
tattttgtta gaaaatgatt gttttagatt ttaaatttga ttttttatat aaatttttag 1260
tgtaattttt tttgtattat tttttttttt attagttaaa agatggaaag gataaattga 1320
ggaatttatg ttggaggagt atggatttat tagatggtag aaatttgggt ttttgaatga 1380
ttattgtgga gttgattttt gtttatgttt tgtaaatttt tgtgttgaga gtaagtaata 1440
tatttttaat gtgttaaatt tttgggattt gttgggtttt atagtaatgg ttattttatt 1500
ttaatatgat tgtaaaataa tgttttaaaa tttatagttg ggtttgttta aaatattatt 1560
gttattatta ttataattta tagtttatat tataattatt tttatagaag ttttttttaa 1620
ttggtatttt ataatttgtg gttttttata ttgttgtttt ttatgtgtga gatgtttttt 1680
tgagtttttt ttgttaggga tatgatagaa aataaaatat ttaatatttt ggtgtatttt 1740
ataatgtata aagttttgtt ttatgtatta tttttttaga tttttttaat aaatttaagg 1800
aatatgtaag ggtttataat tatttttatt ttataggtga tgaaattgag atgtttatat 1860
gtaaagtgga aataattatt tatttattta tttatttatt aaataaaaat tttgttaatt 1920
tatttaaaga ttaattgaga gaattatttt atttgtttgg aatttggtat gtgttaagag 1980
aaaagttttt ttaatttttt tttttaagaa gataatttat tgttatataa taaattattt 2040
tgaaataatg atttaaaata ttaaaagtta ttgattttgt ttatagaatt tgaattggaa 2100
gatataaata attgggagtg agggtggggt tggatagttg gtttttttgg gtattttttg 2160
ttttttttgt ggttttgtta tatgttttta tatggtagtt ttagggtaat tatgatagtt 2220
gaaggtttta gagtgggtgg agggggagag ggagggaggg aaagagagag agagagagag 2280
agagagaaag agaagtaagt aggtagaagt tttaatgttt tatgatttaa ttttggaaat 2340
aaattatatt gtgttattgt tattattttt ttttagaagt aagtagatag ggttggtgaa 2400
tatttattga gaggggaatt agattttatt ttttgattgg gggagtgtta aagaatttgt 2460
gaatatgttt ttaaatgatt atattttttt tttttaaaat gtttgtttaa attggtgtat 2520
ataaaaagtt tgggggttag tgatttattt aaggttatta agttgttaaa tggttttatt 2580
aatattagat ttaattttgt gattttaaat tttatgtttt tttttattat tttagataat 2640
ttttggtgaa tattattgtt tattagggta gttaagatat tttagttgtt aaatagattt 2700
agttttttta attgttttgg ataatatatt ttttattttg tagaaaataa atattattta 2760
tatggtagtt atttataaat attttttaag tttattttaa atatttgtgt tttaaatttt 2820
tttttttaat agatgtattt atttgtattt ttttagatga attatttttt ttttgttgtt 2880
gttgttattt ttgattgtat tttttatttt ttaagatttt tttgtattat taagtttatt 2940
gtttaagtta atattatttt ttaatttggg gatttttttt tgatttttgt gtgttttttt 3000
tgtgggttat taatgtaatg ttatttaaga ttaggatttg gttgggtgta gtggtttatg 3060
tttgtaattt tagtattgtg ggaggttgag ttgggaggat tatttgaggt taggagttta 3120
agattagttt gattaatatg gtgaaattat gtttttatta aaaatataaa attagttggg 3180
tatagttata tgtttgtaat tttagttatt gggtagtttg aggtaggaga attgtttgaa 3240
tttgggaggt ggaggtggag tttgtagtga gttaagattt tataattgta ttttagtttg 3300
ggttataaga gtgaaaattt gttttaaaaa aaaaaaaaag attaggattt gatataaggt 3360
ttgaggggta tttttttgtt ttgttttgtt ttgtttttaa gaggttaaaa tttttatagt 3420
tgaaaatttt tgttgaatta tagagatttg aattaattta gtttagaaag tttggggatt 3480
tgaataatgg tatggattgg aaattttttt atttgttagt ttttattatt ttaggtagta 3540
aaatagattt ttttttagga gttttttatt gtttggggtt ttttagtagt gggatgtggg 3600
gaattaattt ttttttgttt ttatttaaat attaggtggg agtaaggggt gggaagtaga 3660
gaaagtggat agaggttttt agtggatatg ggatttttgt gtagattagt atagttttta 3720
gaaattttat gtaagtaata taggtattgt tatatttttt agtggttatt ttttaaaaag 3780
taaataggtg aggttgggtg tggttgttat gtttgtaatt ttagtatttt gggaggttta 3840
ggtgggtgga ttatgaggtt aagagatgga gattattttg gttgatatgg tgaaattttg 3900
tttttattaa aaatataaaa attagttggg tatggtgatg tgtgattgta gttttagtta 3960
ttggggaggt tgaggtagga gaattatttg aattttggag gtggaggttg ttatgtttta 4020
ttatatttta gtttggtgat agagtgagat tttgttttaa aaaaaagaaa gtaaataggt 4080
gaaattaatt ttaataatat attttgttta atttaatgta tttaaaatat tattatttga 4140
aagtgtaatg aatataaaaa tatttatgag atatttttta tttttatatt tatattgttt 4200
tggattttaa tgtgtatttt atatttatag tataattaat ttgggattag ttatatttta 4260
gtttaataat agttaatagt atatgggata gtgtaaataa attttgtgtt tttgttgttt 4320
ttttgggttt tggagatttt aatttttttt ttagattgta aatttttttg tttttaagtt 4380
ttggttttaa tattagtttg gtagaggaat ttagtttaat gaggtatgtt ttttttttgt 4440
tattttttat tttattaatt tgttttgtgg taaatgtagg attgattttt taaaattatt 4500
ttattaatta gtttatatat ttattattta tttgttttat tagaatgtag gtttttggaa 4560
ggtagggatt taaaaaaatt tgttttgttt tatgtgattt ttttatatta agtattgtgt 4620
ttggtataag ttgggatttt agtatatatt ttgggatgga agaattgtgt ttttttagaa 4680

tttagttaga gggtagttta gtaatgtgtt ataggtgggg tgtttgtgtt ttgggtggat 4740
gtattggttt tttggttggt gtgggtgtgg ggtgagtggg tgtgtgtggg gtgtgtgtgg 4800
tagagtgtgt tagtgagttt ggagtgtgga gttgggagga gtagtgagtg ttgtgtagaa 4860
tttgtagtgt tggtttggta gggtagtttg gaggtgggtg ggttgtgttg ttagtttgtt 4920
tgtagggttt ttattggttg tttgttggtt gttttttgtt taaaaggtgg taaggagttg 4980
agaggttgtt ttggagtgtg aggaggatag ttggattgag ttaatgttgg ggattttgtt 5040
ttttttgtgg aggggatttg gtaatttgta gtggtagggt ttggggttgg tgtttgggag 5100
ggatttgtgt tttttattta ttttttagtt gtgtttttgt tgtgttttgg ttagtttttg 5160
gtgttggtgt ttatggttgg tttttgatag tgttttggag ggattggggg agtttttagg 5220
tgtttgggtg agtagttagg tgtggttttt tggttttttt gatttttggt gttagttttt 5280
gtttttttag ttagggtgtg gtggggtttg tttgggtagt gttttgagta attgggaagg 5340
ttaaggtgga gggaaatttg gttttgggga gaagtgtgat tgtagttggg aggttttttt 5400
agttttgtgg gttgggtgag aataggtggt gttggtttga ttaggtgttt tgtgttgggg 5460
tgtgaggatt tggagtgaat tgttgtgttt tggtgggttg tttttttttt ttttttgttt 5520
ttttgggtgg ttgtatgttg ggttggttgg gtaatggaga gggagttgtt aggaatgtgg 5580
ttttggggat tgttttgttt ggggaagggg agagggtggt tatggtgtta ggagaggtgt 5640
gggagttgag aggtggtgtg ggggtgttat tgttgttgta ggttggagag agattgtttt 5700
tagtgaggtg tgtattgttt gggtgagggt tttatttttt tgtggtgttt ttggaggtgg 5760
gaaagttggg tgggtatgtg tgtagagaaa ggggaggtgg ggaggttagt tatttttgga 5820
gttggttttg attttaatag attgtttagt gtttggggat gttgattttg gggtgttgtg 5880
gtgtttggtt ttatgtgtgt gtggggttga ggggttgggg gtgtttttgg ttgtttagtt 5940
ttaataaagg gtgttttttt ttattttgtg aggaggggta gttttggaga tttggttttt 6000
agtgagtggg gttttagtgt tggggaggtt tatttttttt tggggttgtt attttattat 6060
tattattgtt tttttttttt ttttaaaagg 6090

<210> 304
<211> 6090
<212> DNA
<213> Artificial Sequence

<220>
<223> chemically treated genomic DNA (Homo sapiens)

<400> 304

ttttttgaag aaaaaaaaaa ggtaataata atagtaaaat ggtaatttta aaaggaagta 60
gatttttttg gtgttaagat tttgtttgtt gaagattggg tttttggagt tgtttttttt 120
tgtggggtgg agaggagtat tttttgttaa agttgggtgg ttagggatgt ttttgatttt 180
ttagttttgt gtgtgtgtgg ggttgggtat tatggtattt tgaggttggt gtttttaaat 240
gttgggtggt ttgttgggat tagaattggt tttggaagtg attggttttt ttgttttttt 300
tttttttgta tatatgttta tttagttttt ttatttttag ggatgttgtg gaagaatgaa 360
gtttttgttt agatggtatg tgttttattg ggagtaattt ttttttagtt tgtggtaatg 420
gtggtatttt tgtgttattt tttggttttt gtgttttttt taatattgtg gttatttttt 480
tttttttttt gagtgaggta gtttttagag ttatattttt ggtgattttt tttttgttat 540
ttggttgatt tggtgtgtgg ttgtttgggg gagtaaggga gggaagggag tggtttattg 600
aggtgtagta gtttgtttta gatttttgtg ttttgatata aagtgtttgg ttgggttggt 660
gttatttgtt tttatttggt ttgtggggtt ggggaagttt tttggttgtg attgtatttt 720
tttttgaagt taagtttttt tttgttttgg tttttttagt tgtttgaggt attgtttgga 780
taaattttat tgtgttttgg ttgggaaagt aaaagttggt gttgggggtt ggggggattg 840
gggagttgtg tttggttatt tatttgggtg tttgggagtt tttttggttt ttttggagtg 900
ttgttggagg ttgattatag gtgttagtgt tggagattag ttggggtgtg gtgggaatat 960
agttagggag tgagtggggg gtgtagattt tttttaggtg ttggttttag attttgttgt 1020
tgtgagttgt tgagtttttt ttgtggaggg aataaagttt ttggtgttgg tttggtttgg 1080
ttgttttttt tatattttga agtagttttt tggttttttg ttgttttttg ggtggagggt 1140
ggttggtagg tggttaatgg ggattttgta agtgggttgg tggtgtggtt tatttatttt 1200
tgagttgttt tgttaggttg gtgttgtggg ttttgtgtgg tgtttgttgt tttttttagt 1260
tttgtgtttt gggtttgttg gtgtgtttta ttgtgtatat tttgtatata tttatttgtt 1320
ttatatttat gttggttggg gagttagtgt gtttgtttgg aatgtgggtg ttttatttgt 1380
gatatattgt taagttgttt tttgattggg ttttagggaa atatggtttt tttgttttga 1440
gatgtgtatt gggattttag tttgtgttgg gtatggtgtt tggtatggga aaattatata 1500
gaataaaata gattttttta aatttttgtt ttttggaaat ttgtattttg gtgggataga 1560
tagataataa atatgtaagt taattaataa ggtaattttg gaggattagt tttatgttta 1620
ttatgaaata ggttaatgga atagagaatg gtaggaaggg agtgtatttt attagattgg 1680

gtttttttgt tggattggtg ttggagttga ggtttgaagg taagaaggtt tgtaatttga 1740
agaaagggtt gaggtttttg agatttaaag aagtaataga gatgtagagt ttatttgtgt 1800
tattttatat gttattggtt attgttgagt tgaaatgtag ttagttttaa attaattgtg 1860
ttgtaagtgt aaaatatata ttagagttta agatagtatg gatatgagaa tgaaaaatat 1920
tttatgaata tttttatatt tattatattt ttaaatgata gtattttgga tatgttgggt 1980
taaataaaat atattattaa aattaatttt atttgtttat tttttttttt ttgagatgga 2040
gttttatttt gttgttaggt tggagtgtag tggagtgtgg taatttttat ttttagggtt 2100
taagtgattt ttttgttttg gtttttttag tagttaggat tatagttgtg tgttattatg 2160
tttagttaat ttttgtattt ttagtagaga tggggtttta ttgtgttgat taggatggtt 2220
tttatttttt gattttgtga tttgtttgtt tgggtttttt aaagtgttgg gattataggt 2280
gtgatgattg tgtttggttt tatttgttta ttttttaaaa ggtggttatt agaaaatata 2340
atagtattta tgttgtttgt atgagatttt tgaggattgt gttggtttat ataaagattt 2400
tatatttatt ggagattttt atttattttt tttatttttt attttttgtt tttatttaat 2460
gtttgggtgg agatgaagaa gggttgattt tttatatttt attgttggag aattttaaat 2520
ggtgaaaagt ttttaaaggg aaatttattt tattgtttag aatgatgaaa attgatagat 2580
gaagagattt ttgatttata ttgttgttta aatttttagg ttttttaaat tgagttggtt 2640
taaatttttg tggtttaata gaaattttta attgtgaaga ttttggtttt ttgaaaataa 2700
ggtaaaataa aataaaagaa tattttttag gttttatgtt aaattttagt tttttttttt 2760
tttttgagat gggtttttgt ttttgtggtt tagattggag tgtagttgtg aaattttggt 2820
ttattgtaaa ttttgttttt gttttttggg tttaagtgat ttttttgttt taggttattt 2880
agtagttggg attataggta tgtgattatg tttggttaat tttgtatttt tagtagagat 2940
gtggttttat tatgttggtt aggttggttt tgaatttttg attttaggtg atttttttgg 3000
tttggttttt tatagtgttg ggattatagg tgtgagttat tgtgtttagt taaattttag 3060
ttttaaataa tattgtatta atgatttata ggaagggtat ataggaatta aggggaagtt 3120
tttaaattgg gagatggtat tagtttaagt agtgggtttg ataatataaa agggttttga 3180
gaggtgagaa atatagttaa aaataataat aataataaaa aaagagtgat ttatttggga 3240
aaatgtaaat gaatatattt gttgggagaa aaaatttaaa atatagatgt ttgggataaa 3300
tttggaaggt gtttatggat gattgttata tgggtaatat ttattttttg taaaataaaa 3360
aatatattgt ttgaggtaat taaaaggatt gaatttattt ggtagttaga atgttttaat 3420
tattttaatg gataatgatg tttattggag attatttgga gtagtgaaaa agagtatgaa 3480
atttggagtt ataaaattag atttagtatt ggtggaatta tttaatagtt taataatttt 3540
ggatgagtta ttaatttttg agttttttgt gtatattaat ttagataagt attttagaaa 3600
ggggaaatgt ggttatttaa aaatatattt ataaattttt tgatattttt ttaattaaga 3660
ggtggagttt aatttttttt ttaataaata tttattaatt ttatttgttt gtttttaaga 3720
aaaggtagtg gtagtggtat agtgtaattt atttttaagg ttaggttata aggtattaga 3780
gtttttgttt gtttgttttt tttttttttt tttttttttt tttttttttt tttttttttt 3840
tttttttttt ttatttattt taaagttttt agttgttatg attattttag ggttgttatg 3900
tagagatatg tgataaaatt atagaaagaa tgagagatgt ttagagaagt tagttgttta 3960
gttttatttt tatttttagt tgtttatatt ttttagttta agttttgtga gtaaaattaa 4020
tgatttttaa tgttttaagt tattatttta gagtggtttg ttatgtagta atagattatt 4080
tttttaaaaa gaaaaattaa gaaaattttt tttttaatat atattaggtt ttagatagat 4140
gaaataattt ttttaattaa tttttaaata aattagtggg gtttttattt aataaataaa 4200
taaataaata agtggttatt tttattttat atgtaaatgt tttagtttta ttatttgtga 4260
aatgaagatg attatagatt tttatatatt ttttgggttt gttgggagaa tttaaagaga 4320
taatgtatga aataggattt tgtatattat aaaatatatt agaatgttgg atattttatt 4380
ttttattatg tttttagtaa aaaggattta gagaggtatt ttatatatag aaggtaataa 4440
tataagaagt tataaattat gagatgttag ttgagaagga tttttataaa aataattatg 4500
atatggatta tggattataa taatgatggt gataatattt taaataaatt tgattgtaga 4560
ttttagaata ttattttata gttgtattag ggtaaaataa ttattgttat aagatttaat 4620
aaattttaga ggtttaatat attaaaaatg tgttatttgt ttttagtata aaggtttgta 4680
gggtataagt agagattagt tttatagtag ttatttagaa atttaggttt ttgttattta 4740
atgaatttat gtttttttag tgtaagtttt ttagtttatt ttttttattt tttggttgat 4800
gagagaagag ataatatagg gaggattgta ttgaaggttt atgtaggagg ttagatttga 4860
agtttgagat aattattttt tggtaagatg tttttagttt aaagtggatt atattatttt 4920
atttttattt tttggttgtt tttttgtaat atggttttgg ttttattaag ggttaatatt 4980
tttaagaatg ttttttattt tgggaggttg aggtgggtag attatgaggt taggagatta 5040
agagtatttt agttaatatg gtgaaatttt atttttatta aaaatataaa aaattagtta 5100
ggtgtggtgg taggtgtttg tagttttagt tatttgggag gttgaggtag gagaatgggg 5160
tgaatttggg aggtggagtt tgtagtgagt tgagatggtg ttattgtatt ttagtttggg 5220
tgatagagta agattttgtt ttaaaaaaaa aaaaaaaaaa aaaaaaaaga atgtttttgt 5280
attgtggttt tattattaaa gattttgttt gttatgtagt tgagagtttt tgatagaggt 5340
agttatatga ttgattttag ttttttttag tattgagtgt taaaagtatt tatttttttt 5400
tattaaatta tatttttttt tattagataa aagtatagag tttattttgt tttttatttt 5460

gttttggatt tgttagaaag tatgtggtaa gtttagaaag tataaatttt tatttaatgt 5520
ttaatttatt attttttttg attttgttta taagtatttt ttgagtattt agtgtttatt 5580
aaatgttagg gatatagata taagagaaat attttttgtt tttaggtaat ttagtttgat 5640
gagggagatg gattaataaa tagatttgtg attgtgttaa atataattta tttgttattt 5700
tatatttaat ttattttttg agttgtaaga ttattttaat gtattttttg ttttttggtt 5760
gttggtaggt ttagttaatg gggtatttga tatgagatga aggagaagga aggagagtga 5820
agttaagata gttatttttt tgtttttttt tttgtaagat tgttatagtt tatagttaaa 5880
tataggttgt tgttttttat taggttttgt tttttattgg gttttttttt tttgggtttt 5940
ggtaattatt tttttttttt tgtttttttg agtttagggt tggtgttagt tttgttatta 6000
ttattttggg tttttgtttt attttttatg gtttttttgt atttttttta tatttttttg 6060
aattgtttta atttgagtat tttatttttg 6090

<210> 305
<211> 6274
<212> DNA
<213> Artificial Sequence

<220>
<223> chemically treated genomic DNA (Homo sapiens)

<400> 305

ggattatttg agtttgggaa gttgaggttg tagtgagtta aggttatgtt attgtatttt 60
ggattgggta atagagttag attttgtttt aaaaaaaaga aaaattttat ggttttgttt 120
atattattta tttgatttta tatgttgttt atttttttta ttaaaatttt tagtttatta 180
attatagttt ttttataatt aatattttga tgtgataatg ttttagttta attattgtgg 240
ttataataga atgttataaa ttgggtgatt tataaataaa agaagttgat ttaggttgat 300
ttagaggttg gggagtttaa gagtttggtg ttagtatttg atgagtgttt ttttgtttta 360
ttataatatg ggagagggta ttatgtgtga agagagttta tttttataat atagttattt 420
ttataagaat taatttattg ttatgagagt tatgtgaatt tatttatgag gatagtgggt 480
taagttttta atatatggat tttttgggga tatatttaaa ttatagtagt tagttgtaat 540
gtttgtgtta tgttttattt tggttttgat gtttgtgtag tttttttaaa ttgtgttttt 600
gttttttagt gtgttttgta atgtggaaat gatatattgg gtaagaggag ttgtagtaaa 660
gaggttttta gtgatgtagt gataagttgt ggggagaggg agtgttgtat agttttgttg 720
tatgttatag ttttttagtg agtttgtgtt tttggattgt gaattttatg tttgtttttt 780
agttttttta gttttttaga tggtatagaa ttgttggagg ggggtggagt tgtatatttt 840
ttttgttttg ggtaggttat tttttgataa aatattaggt taggtttttg gtgaaataat 900
tttttttgag ggtagatttt ttattaataa tagaatgttt taatttattt taaaatggtt 960
tttttttttt ttttattgtt agaagtataa tgagattttt tttttaatat ttgtggtaag 1020
gatttagtag agttttagga ggtaatattt ttaagtgttt tatattattt tgtattatga 1080
ttgggttttg ttggagtttt taatttgtag aattgtttat attgagtttt ttgtaatttt 1140
ttaattatag ggtaaatttt tttagttggt attgggtttt tggaggtttt tgtttgttgg 1200
tttttttttt tggaggttgt gtttttgtgt ttgtttgttt ttttaatttg gggggtagtg 1260
gtttgtttaa tgattttaat tttttgaaag agttaagaag aggtgttaat tttttggttt 1320
gtttagtttt ttatttgttg ttagaatgga gtgttaatag tgttttttat agtgatatgt 1380
aatttttaat tttagagatg atgaagtata ttaatgataa aggagaaatg ttttagtagt 1440
tttttgttag tatattattt ttttgaaaag ttgttttttt tatattttgt aagagatggg 1500
ttttaattta gagtttaagg taaatgattt tttttaagga gaaggaataa atagttttag 1560
aaattatgaa agtttgtttt taggagtgtt tttgaatttt agtaggggtt atatttattt 1620
tgtagaaata ggtgaggtat gtttttggta taaaatttta atggtataga aggataaatt 1680
gaaaaataag ttttttttta aatttttgat tttgagttat ttagtttttt tttttagagg 1740
taaagttgtt attagatttt tgtatttttt taatatatat ttttagaaga gttgttaagt 1800
gaatatatgt ttaagtgaaa atttatttta gaagtgtatt tttttaagga attttagggt 1860
tggaaggaat ttgtgttagt ttttaattta taatttttat tagtatttat tgtttttgta 1920
taaaaatttt ttttaaaaaa gtttttttta ttttgatata gtttatttta tttttattta 1980
gttttaataa tttataaaat atatttttga aagtttaaaa tttgttatta tgtttttttt 2040
ttttaattaa tttttatttt gatttttaag ttagagaaaa taggtggtta aatgtttttt 2100
gtttaagatg gaatttagaa tatttgaaga ttttagattt ttattttgtt aaataatgtg 2160
tttttttttt ttttttatag agtatttggt tttaggaaat ttagagttat attttttata 2220
gataagatta aatttttatt taatatattt agaaattttt tttaagagga taattattta 2280
ttagaggaaa aaagtttttt atgtatagtt ggtaaaggga tggaattatt tgtgttatta 2340
aaattgatag atgtttatga gatttattaa gggaaatatt agagttttag tatatatttg 2400
ttaatatagt atatatgaag gttttattta taattttttt ggttaagtag aaattttggt 2460

attatttatt ttaataaatt tttaagatat tatgaaatag aattttaggt tttgatatta 2520
ttatttgttt taggttttga agtgttgttg gataagaggg gtaaaatatg gttttgtttt 2580
ggatttaaag ttggtttttt atattagtaa gtatattttg gtattttggt tatttttttt 2640
ggttatagta ttatattgtt ataaaaggta gatataagta ttaattagtt tataggttat 2700
tagataagtt tagtttgatt aatgtttaat atagtaattg ggttattatt gttatttttg 2760
tggtggtggt atatatattt agttttgttt tgggttatgg tttaggatta tttttttatag 2820
aggttgtgag ttagagtttt aattgtgtag ggttttaatt atgttaggtt atttattttt 2880
tttaagagga ttttattagt gtttgtttgg ttatatagtt ttttatttat taagtaatat 2940
agttattagt atattttagg tattagttaa ggattataaa attaatgtga atgttagttt 3000
ttgtattaaa agtttaaagg agaaatttaa attttatata gatgttttat gaagatgttt 3060
agtaaattta tttttttttg ttttttggaa tttattttag tattgtgtta tgtgtgtgtt 3120
tagtaatttt ttataaaaag ttttgttttt tgtgatgtta ttagattata ttgaagaata 3180
tataagttgt attatgaagg ttgttgtttt atatagtttt aatgtagtga gaattgatgt 3240
ttttatatgt tgtttttttg ggtatttaaa gaaatttttg tatagtttta taaattagtt 3300
gtagtttaaa ttgatttgtg ttgtgatttg tatatatagg ttatattttt ttgatagaaa 3360
atatagttta aaattaaatt tgtagttttt gttaagtgaa tgtataggat tttattgtat 3420
ttaggttttt tattgtaaga tttatttttg tttttatttt atgttttatt gttgtgtttt 3480
ttatttgttt ttttttagtt ttgttttttg tgtttttatg gattgttttt agtttaggtg 3540
tgtaggaagt atatatatgt ttgtagagtt tttatggttt ttgtatttag ggtatgattt 3600
taatgtatag tggttgtatt gatttgttaa aataaaggaa tagattattt tttttaattt 3660
atagggaagt tttaggttgt gtgggtagtg agtagatttg tgtttgtttg tgtttgtttt 3720
ggtgaaaaat tttattgttt aggttgtagg gtgtgagatt taggtataaa tattttgttg 3780
gatgaggagg aaagatgtaa ggttgttttt ttttagagat agtaaagggt aggtttgtag 3840
ttttatttat tttaggattg tgatttttga tagagttgag agattagggt tgttgaatta 3900
ggtttgaagg ttttagtgaa ttttgtgaag agaggagggg tttggttgta atatggattt 3960
agaggatatt tttattgtag gagaaggaat agtggggatg gggtggattt gttaaaggaa 4020
tatagtttaa gtttttgtag tttaaaaaag tttagttttt tttggttaaa gtttttgtga 4080
gtttttttgg tatttttttt gtgggagtta taggggtagt gggatattta gtttttttaa 4140
aagtattttt atggttgttt gtgttaagtt tttattttaa gagttttatt tttgtgaagt 4200
aatgtgtttt atatattggt tttaaagtat ttatggttgg ttgtaataaa tattaaggag 4260
gtttgttttt gtatttggag ttgggtgttt ttattttaga tgattttgag ggtgtttgat 4320
aagatttgaa ggatttttgg attttagagt attattttgg atgtttggta tttttgttgt 4380
gtgggtatgg tgattttttt agttgttagg ttagtttttg atttttgaga gggttttgta 4440
gtgttgtagg ggaggtgggg atttgaataa aggagtagtt tttttgttgg tgttattatt 4500
tgatgttggt tttaaggttt ggttagtttg tttaaagttg gtataagttt gttttgtaaa 4560
ataaaagaag ggaaaggggg aaggggattt tggtatagat ttggtttgat ttggatatag 4620
gttgggtttg taagtttgtg gggattgggt ttagaggggt agtgttggga atgttttttt 4680
tggaaattaa ttttttaggg tatttgtttt ttttttatgt gttgttttat ttttgttgga 4740
gattaggttt tgtgggggtt attgttgttt attgtttttg gtggttgttg gttttgggtt 4800
tttgttgttg gttttttttt tttttttttg tatttttttt tttttttgtt ttttttgatt 4860
ttttttttgt tgtttggttt tttttttttt tgttttgttt ttttgtgttt tggtttgtgt 4920
gagttagatg tttgggtagt ttttggtgta gtgtggttgt agtagttttt gttttttgta 4980
tggtgtgagt gtttgatgtg gttgaggtgg ttggagtttt gagttagttt tggtggttgt 5040
tgttgtttag attggatgat aggttatttt gttggtgttt gtttgagttt ttgttttgtt 5100
gttaatgtta taattattgt gtatggtttt ttgattttgt ttagtattga ttgggagagt 5160
tggagtgagt tttttgggga gtagtgatgt gattttttgg gatggttggg gtagtgtttt 5220
tggtgttgtt ggttgtgttt tgtttggtga gttgggtttt ggaggaaaag aaaggtaagg 5280
gtgtgttttg ttggtttttg tgttgttttt ggattgtgtt ttggattttg tagtttgttt 5340
aattgtgtat tggtgtattg gtttggtgtt tgtgtttttg tttgtttttt tttgtttttt 5400
tgagattagt tgtgttgttg attgggattg tgggaggaat gggatgtttt gttttttggt 5460
tgggagagtt tggggtgggt ggaggaggag atgtgtggga tattgggttg taggttaggt 5520
ggggaatggt tgttgggatt tttggtgttt tgaattgttt ttaatttttt ttttttattt 5580
tttttgttta gttgtgtagg attggtgtta gtgggtgaaa gttgggtgtt ggtgggtgtt 5640
tggggttggg gttttgtatg tgtgttttgt gttgtttttt tagggtgtga tggggttttg 5700
gtgtgtattt gaggggtggg tgttgtttat ttgttgagat tgtattgttt agggaagttg 5760
aggaaggaat ttaaaaatat agtttttttt tggattttgt gggataggtg gttttttgag 5820
aggatttttt tgtttttgtt ttttgtgtag gttttaaatt gaagttggtg tttgttagtt 5880
tggttttggt ttttttttag gtttttgtga tttttgtttt ttagtgtgga gttgtagttt 5940
tgatttggga gttgggagaa tttgtttatt attatttgtg gtttttgggg aggggtggtg 6000
ttggtggtgg ttagtttttt tgttggtaaa aggtaggtgg ggtttgattt gttttttggg 6060
tgtagatttt ggttgtttgt tttgtttggt gtgtttttgt tttgtttatt taagagtgtt 6120
ttttattttt tggggatttt agtttttttt tgggtgtttg tgttgaaagt tttaggtttt 6180
tttttgatgg ttgttttgtg gagatgtttg ggtttgtttt atttgtagtt ttttggttgt 6240

gtttgggttt tgtggtggag tgggatgtgg ttgt 6274

<210> 306
<211> 6274
<212> DNA
<213> Artificial Sequence

<220>
<223> chemically treated genomic DNA (Homo sapiens)

<400> 306

atagttgtgt tttgttttat tgtgaagttt aggtgtgatt gaagggttgt aggtggagta 60
gatttggatg tttttgtgag gtggttattg aaggagagtt tggggttttt ggtgtgggtg 120
tttaggaggg agttggggtt tttgggaggt gggggtattt tttggatagg taagatgagg 180
gtgtattggg tgaggtgagt ggttggggtt tgtgtttaag gggtgggttg gattttattt 240
gttttttgtt aatgaggaaa ttaattgttg ttagtattat ttttttttgg gagttgtagg 300
tggtggtaga tgagtttttt tagtttttag gttgaggttg tgattttata ttggggaatg 360
aggattgtgg ggatttggag aggggttggg gttaggttgg tgggtgttgg ttttagtttg 420
agatttgtgt ggagggtgga ggtggggagg tttttttaga aagttgtttg ttttgtgggg 480
tttgagggga ggttgtattt ttgggttttt tttttagttt ttttaaatag tgtagttttg 540
gtgggtgggt agtgtttgtt ttttgggtgt gtgttaggat tttgttgtgt tttgggaaga 600
tagtgtgggg tgtatgtgtg ggattttggt tttaggtgtt tattagtatt tggtttttgt 660
ttattgatgt tgattttgtg tagttgggtg gggaaagtga gggaagaaag ttgggagtgg 720
tttggggtgt tggaggtttt ggtggttgtt ttttgtttgg tttgtagttt ggtgttttat 780
gtgttttttt ttttgtttgt tttagatttt tttggttgaa gaatgaaatg ttttgttttt 840
tttgtggttt tggttggtgg tgtagttgat tttaaggaaa taggaaagga tgggtggggg 900
tgtgggtgtt gagttggtgt gttggtgtgt ggttgggtgg gttgtggggt ttggggtgtg 960
atttgggggt ggtgtgggag ttggtgagat atgtttttat tttttttttt ttttagagtt 1020
tgatttgttg ggtagagtgt agttagtagt gttaggagtg ttgttttggt tgttttggag 1080
ggttgtattg ttgttttttg aagagtttgt tttggttttt ttgattaata ttggatggag 1140
ttagggggtt gtgtgtggtg gttgtggtgt tggtggtgag gtggggattt gggtggatgt 1200
tgatgaggtg gtttgttgtt tggtttgggt ggtggtggtt gttggggtta gtttgggatt 1260
ttggttgttt tggttgtgtt gggtgtttat attgtgtggg gggtggaggt tgttgtggtt 1320
gtgttgtgtt gggggttgtt tggatgttta gtttgtgtgg gttgaggtgt ggggaggtag 1380
ggtgggagga ggagggatta ggtggtggag gagggattgg gaggagtaga ggaggaggag 1440
aatgtgagga ggagggagga gaattagtag tggggattta aggttagtgg ttgttggagg 1500
tggtggatag tggtggtttt tgtgggattt agtttttggt gggagtgggg tggtgtatgg 1560
gaggggagtg ggtgttttga ggagttaatt tttgagaggg gtgtttttag tattgttttt 1620
ttggatttgg tttttgtgga tttgtaggtt tagtttatgt ttaggttgag ttaaatttgt 1680
gttagggttt tttttttttt tttttttttt ttgttttata aagtaaattt gtattagttt 1740
tagatagatt ggttgagttt tagagttagt gttggataat ggtattgatg gggaaattgt 1800
ttttttattt gggtttttat tttttttgta gtattatggg atttttttgg ggattagagg 1860
ttggtttggt agttgaggag gttgttgtgt ttgtgtggta ggggtgttag gtgtttgagg 1920
tggtgtttta aagtttgagg gttttttaga ttttgttaag tatttttgaa attatttgaa 1980
atgagggtat ttaattttgg gtgtagagat aggttttttt aatgtttatt gtaattagtt 2040
atgggtattt tgaagttaat gtgtgaagta tattattttg taaaagtgaa gtttttggaa 2100
taaaggtttg atataaatag ttgtggaggt gtttttagag aggttaagtg ttttattgtt 2160
tttgtagttt ttgtaggaga aatgttaggg aaatttgtgg aagttttggt taaaagaaat 2220
tgagtttttt tgggttgtag gaatttgagt tatatttttt tggtaagttt attttatttt 2280
tattgttttt ttttttgtag taaaaatgtt ttttaggttt atgttatagt tagatttttt 2340
ttttttttat gagatttatt aggattttta ggtttggttt aataattttg attttttggt 2400
tttgttaaaa attataattt tgaagtaagt gaagttatag atttgttttt tgttgttttt 2460
gaaggggagt aattttatat tttttttttt tatttagtaa aatgtttgtg tttgggtttt 2520
gtattttgta gtttgaatgg tggggttttt tattagggta agtgtagata gatataggtt 2580
tgtttattgt ttgtataatt tggaattttt ttgtgaatta ggagaagtaa tttgtttttt 2640
tgttttaata aattagtata gttattgtgt gttgaagtta tgttttgaat gtagaggtta 2700
tgaaggtttt gtaggtatgt gtgtgttttt tgtatatttg ggttgagagt agtttgtaga 2760
aatatagaaa ataaaattag agaaaggtaa atgggaagta taatagtgga atataaaatg 2820
aagataggag tgagttttat aataaaagat ttgaatataa taaagttttg tgtatttatt 2880
taataagggt tgtaaatttg gttttaaatt atattttttg ttaagggaat gtgatttgtg 2940
tgtataagtt ataatataaa ttaatttaag ttataattga tttgtaagat tgtataggaa 3000
tttttttgag tgtttaaaaa gatagtatgt aagaatatta gtttttatta tgttaggatt 3060

atatgagata atagttttta tagtatggtt tgtatatttt ttaatgtgat ttgatagtat 3120
tataagaaat agagtttttt gtaaagaatt attagatata tatatagtat aatattggga 3180
tggattttag ggaatagaga agaatgggtt tgttgaatat ttttatggga tatttatgta 3240
aagtttgagt tttttttttg agtttttgat ataaaagttg atatttatgt tgattttgta 3300
gtttttggtt agtatttgga gtgtgttgat aattgtgtta tttggtaagt aaaaaattgt 3360
atggttgagt aggtattaat gaagtttttt taagagagat aagtagtttg gtatagttag 3420
ggttttgtat agttagggtt ttagtttata gtttttgtgg agggtggttt tagattatgg 3480
tttggataga ggttgggtgt gtgtgttatt attataggaa tgataatagt ggtttagttg 3540
ttgtgttaag tattggttag attaagttta tttgataatt tgtgagttgg ttaatatttg 3600
tatttgtttt ttatagtaat gtgatgttgt ggttgggaga agtgattagg atattaaggt 3660
atatttgtta gtatgagagg ttaattttga atttaaggta gagttgtgtt ttattttttt 3720
tgtttagtaa tgttttaaaa tttgagataa atggtgatgt taggatttaa aattttattt 3780
tataatgttt tggaaatttg ttagggtgag taatattaaa atttttgttt ggttaaaaaa 3840
attatagata aagtttttat gtatgttata ttagtaagta tgtattaaga ttttagtatt 3900
ttttttaata aattttataa gtgtttgtta attttaataa tatagatggt tttatttttt 3960
tgttagttgt atatgagaaa tttttttttt ttgatgagtg gttatttttt tagaagaagt 4020
ttttgagtat gttgaataag agtttagttt tgtttataag gaatgtggtt ttgaattttt 4080
taaaattaaa tgttttgtga aaggggagga gaaatatgtt atttggtagg gtgaagattt 4140
gaggttttta aatattttaa gttttatttt aggtaaaagg tatttgatta tttgtttttt 4200
ttggtttaga ggttaaagta aagattgatt aggaaaaaaa aatatagtgg tagattttag 4260
atttttaaaa atatgtttta taagttattg gagttaagta aaagtagaat aagttatgtt 4320
agagtggaaa gggttttttt gagaaagatt tttgtgtaag aataataagt attaatggag 4380
gttgtgaatt aaggattgat ataggttttt tttaatttta aagtttttta agaaaatgta 4440
tttttaaaat aggtttttat ttaaatatgt gtttatttga tagttttttt aaggatatat 4500
gttaaggaga tataagaatt tggtaatagt tttgttttta gggaggagaa ttaggtagtt 4560
tggggttagg ggtttagagg gagatttgtt ttttaatttg tttttttgta ttattgggat 4620
tttgtattag gagtatgttt tatttatttt tgtaaggtaa gtgtggtttt tgttgaggtt 4680
tagggatatt tttgggggta ggtttttatg gtttttgaga ttgtttattt tttttttttg 4740
aaggaagtta tttgttttga gttttgagtt gagatttatt ttttgtagaa tgtggaagaa 4800
gtagtttttt taaggggtaa tgtgttgata gaaaattgtt gaagtatttt ttttttgtta 4860
ttagtatgtt ttattatttt tagagttgag ggttatatgt tattatagga ggtattattg 4920
gtgttttatt ttagtaataa gtagaaagtt gagtaaattg aaaaattaat attttttttt 4980
agttttttta gagaattgag gttattgggt aaattattgt tttttaaatt ggagagatag 5040
gtaaatatag aagtataatt tttagaggaa gaaattagta gatagaaatt tttaaggatt 5100
tagtgttggt tgggaaagtt tgttttgtaa ttgagaaatt gtgggaggtt tagtgtgggt 5160
agttttgtaa attaagaatt ttagtagagt ttagttatgg tgtagggtag tatgagatat 5220
ttgagagtgt tattttttgg agttttgtta ggtttttatt atgaatatta gggggaaaat 5280
tttattatgt ttttggtagt ggaaggagaa gaggaattat tttgaaatag gttggaatat 5340
tttattatta atagaaggtt tgtttttagg agaaattatt ttattagagg tttaatttgg 5400
tgttttatta gagggtgatt tatttagagt aagggaaata tataatttta ttttttttta 5460
atagttttgt attatttaag gggttgggga agttgagaag taagtatgaa gtttatagtt 5520
tagggatata ggtttattgg aagattgtga tatgtggtag gattgtgtaa tatttttttt 5580
ttttatagtt tgttattatg ttattagaag tttttttatt atagtttttt ttatttagta 5640
tattattttt atattgtaag gtatattaaa aggtaaagat gtagtttgaa gagattgtat 5700
aagtattaga attagaatga gatatgatat gaatgttata attaattgtt gtggtttgaa 5760
tgtgtttttg aaaagtttat atattggaaa tttaatttgt tgtttttatg aatgaattta 5820
tatggttttt atggtggtgg gttaattttt gtgggagtgg ttatgttata agagtaagtt 5880
ttttttatat gtgatgtttt tttttatgtt atgatgaagt aagaagatat ttattagatg 5940
ttagtattaa gtttttggat tttttagttt ttaaattagt ttaaattagt tttttttgtt 6000
tataaattat ttagtttgtg gtattttgtt ataattatag taattggatt aagatattat 6060
tatattggag tattaattat aaaaaaatta tgattaatag gttaggagtt ttaatggaaa 6120
aaataggtaa tatgtaagat tagatggata atgtaagtag agttatggaa tttttttttt 6180
ttttgagata gggtttggtt ttgttgttta atttagagtg tagtggtgtg attttggttt 6240
attgtaattt taatttttta ggtttaggtg attt 6274

<210> 307
<211> 3002
<212> DNA
<213> Artificial Sequence

<220>
<223> chemically treated genomic DNA (Homo sapiens)

<400> 307

atatttaaat tgtatatttg gtttgtaata tattatatat ttaaaagaat tattattttt 60
ttgaatatta ttaatattaa taatagtatt atagtgatta agagtggatt taaataaagg 120
ttggtttgaa atttaggttt gttatttatt agtaagtttt taaaaatttt gagtttttag 180
tttttttata tgtgaaatgg agaaaataaa tttgtaaaat taattaaatt tgtttattta 240
tatttttttt tttagtgtat attaattggt attttttgtt aggttagaat gtgtttttaa 300
ttatgtttta aatttgtttt gtgttaattt tattgttaga atttttttta ttttgagaat 360
tagaaaagga aatattatgt ttggtaatgt ttatattttt taaaataaat ttgtaggaaa 420
gaatatttag taagtgatga gagtagtaat gattgttttt attattttaa atttataata 480
ttattttttt tagagttttt taagtattgt agataatttt ttatttatta aaaaataaat 540
tgtaattata agtatttagg gttgatattg tttttgaatt agatagtgtt tatattagtt 600
gtataagatt aatttaaagt agaggatgaa attttttttt tgaatttttt ttagaatgta 660
atttagtgaa tatattaaaa ttaaattttt tttgaatggg agtaattttt atggattaat 720
ttgtaatttt ttagattata tttaaggtaa tgtagaggtt gttgtattat aggttttgtg 780
attagagatt attggatttg tttttggaaa agttatttat gatttttttgg gttttggttt 840
ttttattttt tatattggtt taataatgat tattgtagag ttgttatgga agttaaatga 900
atttatgtat tataagtgat taatataatg attgatattt agtgatttat taataaatta 960
aaatatttat tattaatatg atagagaagg tgttgttaaa atagataata ggtttttgga 1020
agaggtgatt aaatggatgt aaaatttatg gattgtttat tttgtttatt tttgttgtgt 1080
tttttggttg tggtatatat atgtgtgggt ataaaattgt aaattttatg tagttgtgta 1140
gtgtatgtgt agaaggttta gatatgaaat gttattttag taatgtgttt agagaagttt 1200
tgatgttgtt ttggaagtaa gttgttgttg tttgattttt gggtgtttgg gatggatgtt 1260
tatatttgta tttagtagta ttggaagggg tttaggtttt ttgtagtata gtttattttt 1320
agattgttta gttttttata atatatattt ttatggaaaa gggtattttt ttttgttaga 1380
aaaagtgttt tagtttggtt tgggttggtt tttattttat gttgttgtaa gtaggtgaag 1440
ttttttttgt tttttttttt ggggtaagtg gaaaggagtt tggtaggggg tttgtagtgg 1500
tttgtatagg ggaattgggt agtgagagag ttttaggtaa ttttgggggt tgttttatag 1560
aagtaggtgg ggattgatag tggttttttg gtttagggag gagagtgtgg ttgtgggttt 1620
ttttttttag tttggaggtt gtagttgttt gagttggttt gggtgggggt ggggtggggg 1680
tggtgtggag ggtatggaga ttatggtggt gttatttggg atatttaggg ttttgaggtt 1740
ttgggtggtt tttatgtgag attgtaaatt atgataatag gtagttattt gaggttaaat 1800
aaaaatggag tgggtttttt gtgtgttgtt gttttttgtg tttttggtgg ttttttttga 1860
ggtttttggt ggttttatga gtttgtagta gttggtggtg atgttgtttt tgttttattt 1920
ttttgtgtaa gtgtgaggtt gttggtagtg tggtgtatgt tttggttgtt tttggttttt 1980
gtgtaaaatt tttattttgt ttatgtgaag ttgttgttgt tttagagagg gggaaagagt 2040
tgtgggaaaa gttggggagt gatgattgtg gtggttgggt gtgttttttt attttttttt 2100
tttttttttt tttttttgtt gtagtttgga gttttggttt tttttttttt tttttttttt 2160
ttggagttgg tttttttttt tgttttgttt tttttttgtt tgtgtatgtt atttgttgtg 2220
gggtggttga aggggatgtt ttgtttttat tagaggtata gtgtgaaggg gaaattttga 2280
tattggaagg aatgagaata aatatttaat tatggatgta ttgaattgtg gttgggatag 2340
atattttggg aatttgaggt ggattgggtg atgaggtgag tgattttttt ttttaatttt 2400
tgttttaggg tttttggggg agtttgagtt gagagaattt ttaaattttt tgggaaagtg 2460
tgtgaggttt tgttggggat gttgagtgtt gggtattgag gatgtgtagt tggatggtgt 2520
gtgggtgttt gtgtttttgg ggggtgtttg gaggttgggt gttttatgtt tgagggtttg 2580
ggttgtttgg attgtagtgg tgttttttgt tttagaagat gtttttaagt tttaagggtt 2640
ttttttgagt ttgtttgttt tttttggggt tggtgtggag tttgtgtgta atggagttta 2700
tttagtagtt tagtgtgtgg tttttatttg tattttgttt ttatttggta gaggtgtgag 2760
tattggggtt ttttttatat tttttttatg atgtgtatta ttttttgatg attttttaga 2820
tggtttaggt gtgaggatgt tgatttagag ttttttggag ggttataggt gtttgggttt 2880
ttttggtgtt gggtgtgtgt gtattttaaa ggtttgtgtt ttaattttta ggtattgatt 2940
gggtttttta attgtggtga ttttatttta atagttttta tgtggtgtgg attgaatgtt 3000
-- 3002

<210> 308
<211> 3002
<212> DNA
<213> Artificial Sequence

<220>
<223> chemically treated genomic DNA (Homo sapiens)

<400> 308

--gatattta gtttatgtta tataaaaatt attaaagtgg gattgttgta gttgaaaagt 60
ttgattagtg tttggagatt agaatgtgag tttttaaagt atatatgtat ttggtattgg 120
gaaagtttag gtgtttgtga ttttttgaag gattttgggt tagtattttt gtgtttggat 180
tatttagggg gttattagaa agtaatatat gttataagaa agatgtgggg gagattttga 240
tgtttgtgtt tttgttaggt ggaggtgggg tgtaggtaga agttgtgtgt tggattgttg 300
gatgaatttt gttatgtgta ggttttgtgt tgattttgga agggataggt aggtttggaa 360
gggatttttg gggtttgggg atgtttttta gggtagagag tattgttgtg gtttgagtgg 420
tttgggtttt taggtgtggg gtatttggtt tttaagtgtt ttttggggat gtaggtgttt 480
atgtattgtt tagttgtgtg tttttagtat ttagtgtttg gtgtttttgg tggagttttg 540
tgtatttttt tggaaagttt gggggttttt ttaatttagg tttttttggg agttttgggg 600
tgggggttgg aagaaggggt tatttatttt gttgtttggt ttgttttggg tttttgaagt 660
gtttgtttta gttgtggttt agtgtgtttg taattaagta tttatttttg ttttttttag 720
tgttgaagtt ttttttttgt gttgtgtttt tggtgaaaat aggatatttt ttttggttat 780
tttataataa atagtgtata taagtggggg agaagtgggg tggagggaga agttggtttt 840
gagggggagg aggaaaggag aggagttaaa attttggatt gtgatagggg ggaaaggaga 900
agaaaagaaa atgagagagt gtgtttagtt gttgtagttg ttattttttg gttttttttg 960
tagttttttt ttttttttta aggtagtgat aattttatgt ggataggatg gaagttttgt 1020
gtggaagttg ggaatggttg gagtgtgtgt tgtgttgttg gtagttttgt atttgtgtag 1080
ggaggtgggg tgggggtgat gttgttattg gttattgtgg gtttgtgagg ttgttggggg 1140
ttttggggga ggttgttagg gatgtggggg gtggtggtgt gtgggggatt tattttgttt 1200
ttatttgatt ttgggtgatt gtttattgtt atggtttgtg attttgtgtg gggattgttt 1260
agggttttgg ggttttggat gttttgggtg gtgttgttgt aatttttgtg ttttttgtgt 1320
tgtttttatt ttgtttttat ttgggttgat ttgagtggtt gtagttttta ggttgagggg 1380
agggatttgt gattgtgttt ttttttttgg gttggagagt tattgttgat ttttatttgt 1440
ttttgtgggg tagtttttgg aattgtttgg aatttttttg ttatttagtt tttttgtgta 1500
ggttattgtg ggttttttgt tggatttttt tttatttatt ttaagggagg agatagaagg 1560
gattttgttt atttgtaata atgtgaaata aaaattaatt tagattagat tggggtgttt 1620
tttttgatgg gaggaaatat ttttttttgt ggagatatat gttatgaagg attaagtggt 1680
ttggggatag gttgtgttgt gaagggtttg ggtttttttt agtgttgttg ggtgtaggta 1740
taggtatttg ttttagatgt ttaaaggtta ggtagtaatg atttattttt aaggtggtgt 1800
tagagttttt ttaggtatat tgttgaaatg atattttgtg tttaagtttt ttgtgtatgt 1860
attatgtgat tatataggat ttatgatttt atatttatat gtgtgtatgt tataattagg 1920
ggatatagta aaggtagatg gaataaataa tttataaatt ttgtatttat ttaattattt 1980
tttttaaaaa tttattgttt attttagtgg tatttttttt gttatattga taataaatgt 2040
tttaatttat tgataaatta ttgagtgtta attattgtat tggttattta tggtatatga 2100
gtttatttaa tttttatgat aattttatgg tggttattat taagttggta taaaagatga 2160
ggaaattaaa gtttaaagaa ttatgagtga tttttttaaa gataaattta gtggttttta 2220
attataaagt ttatgatata ataattttta tattatttta ggtgtggttt aagagattat 2280
agattaattt gtagaaatta tttttattta aagaaagttt agttttaata tatttattaa 2340
gttatgtttt gaaaaaggtt tagaaaaaag attttatttt ttattttagg ttggttttat 2400
gtaattgata tgagtattgt ttaatttaaa agtagtatta attttgaata tttatggtta 2460
taatttattt tttaatgagt ggggaattat ttataatgtt taagaggttt tagaaaaggt 2520
ggtgttgtaa atttaaaatg ataaaggtag ttgttgttgt ttttattatt tattgggtgt 2580
ttttttttgt agatttattt tggagggtgt aggtattgtt aggtataatg tttttttttt 2640
tggtttttaa ggtagaaagg gttttggtag tggggttggt ataaagtgga tttggagtat 2700
ggttgagagt atattttggt ttaatgagga atgttagtta atatatattg gagagaaaaa 2760
tatgaatgga tagatttaat taattttgta gatttatttt ttttatttta tatgtgagaa 2820
aattaagggt ttagaatttt tagaagtttg ttaataaatg gtagatttga gttttaagtt 2880
agtttttatt taagtttatt tttagttatt gtgatgttat tattagtatt aatggtattt 2940
agaaaaatag taattttttt aaatatgtaa tatattataa gttaaatatg taatttaaat 3000
gt 3002

<210> 309
<211> 5649
<212> DNA
<213> Artificial Sequence

<220>
<223> chemically treated genomic DNA (Homo sapiens)

<400> 309

tggtggttta tatttgtaat tttagtattt tgggaggttg aggtaggtgg attataaggt 60
taggagattg agattatttt ggttaatata gtgaaatttt gttttattta aaaatataaa 120
aaattagttg ggtgtggtgg tgggtgtttg tagttttagt tattttggag gttgaggaag 180
gagaatggtg tgaatttggg aggtggagtt tgtagtgagt taagattgtg ttattgtatt 240
ttagtttggg tgatagagtt agattttatt ttaaaaaaaa aaaaagaaaa aagaaaatat 300
tttaagtttt gaggattata tagtttttgt tgtaattatt tatttgggtt attgtagtat 360
aaaaataaat atagataata tatatatgag tgagtatggt tggggtttaa taaaatttta 420
tttatataag taggtgatga attatggttt atagtgtttt aatttttgat ttagatggtg 480
aattattatg agtttttgtt gttttttttg gtagaaatat gggagaaatt atagagagaa 540
atagagattt taggaaaata gtaaaattaa ataaataaag tgagatgttg ttttttttgg 600
aggttgtagg ttgtgttgaa tttgtgtgtg tttgggtgta tagggatgtg agtagatgtg 660
tgtggagatg tgtgtgtggg tggagattga gtgtggttgg aggtggttgt ggtaggatgt 720
tagaaggaag gagtttgtat ttagtttttt tattagtatt ttttggagtt gttgagggga 780
tgttattgtt gaatattgat tttggtgtag gttaaattga agttagtttt tgttattttt 840
tgttgggtga ggtattgggg agaatagtgg gattgaatgg ggtgggtttt ttttttttta 900
tatttttttt ggaaatattt agtattagta tattagaata tggggagtta ttttttttag 960
tgttgttttt taaggtttgg aaggggaaat tttgatttta agttttgttt gaagagtgat 1020
agtaaagggg agaggttggt tgtggtttgg tggtgtttat tttttttttt tgttttgaaa 1080
ttataggttg gtatattatt tgagagaatg attagtgtta ttaagggaag ggtataagtt 1140
tattggtttt aggggagata ttttagggga taggaatatt ttttgatgtt gaatgagtgt 1200
agtatataga tggagaatat atataagaga aattatgagg atatgagaga aggtattggt 1260
aataggaagt tataaaaagt aataaaaaga aagagtagat ataaaaataa taattgaaga 1320
aagttaataa tagatgggat gaattatatg aggtatgtga tttatggatg aagaggtgag 1380
tgatgagatt agttggggaa ttttttttta ggaaagataa attagaagaa atataaatga 1440
tattgaagtg gttaggtgta gtggtttatg tttgtaattt tagtattttg ggagtttgag 1500
gtaggtggat tatttgaggt taggagtttg agattagttt ggttaatatg atgaaatttt 1560
gttttattta aaaatataaa aattagttag gtgtggtggt aggtatttgt gattttagtt 1620
atttaggagg ttgaggtagg agaattattt gaattaggaa gtagagattg tagtgagttg 1680
agatggtgtt agtgtatttt agtttgggta gtagagtgat agtttgttta aaaaaaaaaa 1740
aaggtagtaa agtgatagga ggatagatta gaagggttat tttttagata gtgtaaggaa 1800
gaaagaagaa gaagaagagg aggaggagga ggaagaggaa gaagatggag aagagggtat 1860
tttagaagaa ataatggaga tatatttttt aaaattgaag aaagatgatt aattttagat 1920
tgaaggttaa gaggtaaatt agagagatag tttggtaagt taggaagagt attaaagtta 1980
gataattaga gtgaaattaa agaaaattta aggtgataaa aatttaaaaa gtttttttgt 2040
gattttagtt tattggtgaa aatttaaaaa aaaaaaaaaa aaaaaaagga tagtttgggt 2100
aatatggtga aattttattt ttataaaaaa tataaaaatt agttgggtgt ggtggtatat 2160
gtttgtagtt ttagttattt gagaggttaa gtaaggtagg aggattattt ggggttaaga 2220
gttttagatt agttgtggta atatatatgt tgtgggaagt taggtatttt gaatggaggg 2280
attggttgaa gttatggtag aagaatataa attgtgaaga ttttatggat atttattatt 2340
tttttaatta atatttttgt gattttttat gtttgttttt aattttttaa ttttgttatt 2400
tttgtaaatt gaggatgtat gttgttttag gattttgtga tgattgtgtt aattgtataa 2460
attgtttgta gaggataatt gttgtgtgtt tgagtaatgt gaaatttggg tattttaaga 2520
ataggataat agtaattttt agggaataag ggagataatt ttaaagtttg gttgtttgtg 2580
ggttgggtag gatagagtta tatttttttt attattgtaa ataggtaaga gaaatattgt 2640
tgaatttttt ttttagtaag gaatattaat aattaatagt tttgagaaaa gaatgtattt 2700
ttagggttgg gttattgaaa tggttttttt gagagtgttt gtttttattt agaagtagaa 2760
agggatgaaa tatgttttag ttttttgtag tatttttaga tttgatagga ttaggaaatt 2820
ttagtttggt aaattttagt tagattggtt ttttgttttt gaattttgat agtgtgtgtt 2880
agtgggattt ggaagtttat tagtgatttt agttttgttg tgattttttg ttttgtgatt 2940
ttttgttgtt tttgaagtat gtgatttttg tgatttatat tttgtttgtg tatttttttt 3000
tttttgaaaa ttgttaataa aatttgttgg gttttatggt ttagggggta ttatggaatt 3060
tgtggatatg tgatgttttt tttggatatt tggttttaaa atgttttttt tttgtatttt 3120
ttttttttat tttttaggtt agttgatatt tagggaaaat aggaaaggat ttatgtgaaa 3180
tattgggggt tgaatttttt ttgataataa gttttttaaa aaagattttt aaatgaaatt 3240
tttttgtttt ttaattttta tttttgtttt gggggtatat gtgaaggttt gttatataag 3300
taaatttgtg tttttggtgt ttgttgtata gaatatttta ttatttaggt attatgttga 3360
gtatttaata gttttttttt ttgttttttt tttttttttt attttgtatt ttggagttaa 3420
ttatagtgtt tgttgttttt ttgtttgtgt tataagtttt tattatttag tttttattta 3480
taagtgagaa tatttagtat ttggattttt gtttttgtat tagtttgtta aggataatag 3540
tttttagttt tatttatgtt ttataaaaga tatgatttag ttttttttaa tggttgtatt 3600
aaatgaagtt ttaaagatat aatataaata ttaatttttt ttttattata aaaatttttt 3660
gttgaatttg attatattta aattaatgag ttttgtttta tgaaagattt tttggataaa 3720
tttgatagtt gatggaatag gagaagttgt ttgttatgtt taaagttaat aagagattaa 3780

tatttagaat aaatggagat ttgtaaatta atagaaagta ggtagtaaag ttaaagaaaa 3840
tagtttaagg tatagttatt aaaaggaatg tgattatgtt ttttgtaggg atatgggtgg 3900
agttggaagt tgttagtttt agtaaattta tataggaata gaaaattagt gagattgtat 3960
ggttttattt ataagtggga gttgaataat gagaatatat ggttatatgg tggtgattaa 4020
tatatattgg tgtttgttga gtggggtgtt ggggagggag agtattagga agaatagtta 4080
agggatattg ggtttaatat ttgggtgatg ggatgatttg tatagtaaat tattatggtg 4140
tatatattta tgtaataaat ttgtatattt tttatatgta ttttagaatt ttaaataaaa 4200
gttggatggt taggtgtggt ggtttatgtt tgtaatttta gtatttttgg gaagttgagg 4260
tgtgtagatt atttaaggtt aggagtttga gattagtttg gttaatatgg tgaaattttg 4320
tttttattaa aaatataaaa attagttaga tgtggtatgt tatttataat tttatttatt 4380
tgggaggttg aagtagaatt gtttgaattt gagagtggag gttgtagtga gttgttgaga 4440
ttgtgttatt gtattttagt ttgggttata gtgtgagatt atgttataaa ataaaataaa 4500
ataatataaa ataaaataaa ataaaataaa ataaaataaa ataaaataaa ataaaataaa 4560
aaaataaaat aaaataaaat aaaataaaat aaagtaattt tttttttttt aagtggtttt 4620
tatttttttt ttttgttttg tgaagtgggt gtgtaagttt tgggattgta gtggttttag 4680
ggaatttttt tttgtgatgt tttggtgtgt tagtttgttg tgtatatttt gttgtggttt 4740
tttttttgtt gtttgtttat tttttaggtt ttgttgggat ttgggaaaga gggaaaggtt 4800
tttttggtta gttgtgtggt gattttgggg attttagggt gtttttttgt ggttgatgtt 4860
tggggtgtag tggttgttgg ggttggggtt ggtgggagtt tgtgggattt tttagaagag 4920
tggttggtgt tgtgatttag tattggggtg gagtggggtg ggattatttt tataaggttt 4980
ggaggttgtg aggttttgtt ggagttttgt tgttgtagtt tttgttatta gtgagtatgt 5040
gtggtttgtg tttttgggga tggggtttag agtttttagt atggggttaa tttgtagtat 5100
taggtttggg tttttggtag gttttttgtt tattttgaga tttgggatgg gggtttaggg 5160
gatttaggat gtttttagtg ttgttagtgg tttttagggg gtttggagtg ttttggggag 5220
ggatgggatt ttgggggtgg ggaggggggg tagattgtgt ttattgtgtt ttggtatttt 5280
tttttgggtt ttagtaaatt tttttttgtt tgttgtagtg ttgttttata ttgtggttta 5340
ttttttagtt tgaggtagga gtatgtgttt ggtagggaag ggaggtaggg gttggggttg 5400
tagtttatag ttttttgttt atttggagag atttgaattt ttttattttt ttgttgtgtg 5460
gtttttattt tgggtttttt ttttgttttt tgtttttttt gttatgtttg ttttttgttt 5520
tagtgttgtg tgaaattttt ggaggaattt gtttttttgt tttttttttg tatttttgat 5580
ttttttttgg gttgttgtga ggtggagttg gtttggtttt tatattttgt attttttttt 5640
ttttgtagt 5649

<210> 310
<211> 5649
<212> DNA
<213> Artificial Sequence

<220>
<223> chemically treated genomic DNA (Homo sapiens)

<400> 310

gttgtgggga gggagaagta tgagatgtgg ggattgggtt gattttgttt tgtagtaatt 60
tggggagggg ttaggagtgt agggagggaa tagggaaata ggtttttttg aagattttat 120
ataatattgg ggtggggagt aggtatggtg ggagaggtgg ggaataggaa ggaggtttgg 180
ggtaaaagtt atatgatgga gggataaggg ggtttggatt tttttgggtg ggtgaggggt 240
tgtgggttgt agttttagtt tttgtttttt ttttttgtta gatatatgtt tttattttga 300
attgggaaat agattatggt gtagggtggt attgtagtga ataaagaaaa gtttgttgga 360
gtttggggga ggatgttaag gtgtggtgag tgtagtttgt tttttttttt tgtttttggg 420
gttttatttt tttttgaggt gttttgggtt ttttgaaagt tgttaatggt attggggatg 480
ttttgggttt tttaggtttt tgttttgggt tttgaggtgg gtgaggagtt tgttgggagt 540
ttgggtttga tgttgtgggt tggttttatg ttgggagttt tgagttttat ttttggggat 600
gtgggttgtg tgtatttatt ggtggtgaag attgtggtgg tgaaatttta gtgaagtttt 660
gtggtttttg agttttataa gggtggtttt gttttgtttt gttttagtgt tgagttatgg 720
tgttggttgt ttttttggag ggttttgtgg atttttgttg gttttagttt tggtggttgt 780
tgtattttgg gtgttggttg tagaggggtg ttttggagtt tttggagttg ttgtgtagtt 840
ggttggggaa gttttttttt tttttttagg ttttagtggg gtttagggag taaatagata 900
gtaggaagag gattgtagtg aagtgtgtgt agtgaattgg tgtgttggga tattgtgggg 960
ggaaattttt taagattgtt gtgattttgg agtttgtata tttgttttat agggtagggg 1020
agaggggtgg aggttgttta gaggaaagga aattgtttta ttttatttta ttttatttta 1080
ttttattttt ttattttatt ttattttatt ttattttatt ttattttatt ttattttatt 1140
ttgtgttatt ttattttatt ttatgatgta gttttatgtt gtggtttagg ttggagtgta 1200

gtggtgtgat tttggtggtt tattgtaatt tttgtttttg ggtttaagta attttgtttt 1260
agttttttga gtaggtggaa ttataggtgg tgtgttatat ttggttgatt tttgtatttt 1320
tagtagagat ggggttttat tatgttggtt gggttggttt tgaatttttg attttaggtg 1380
atttgtatgt tttggttttt taagagtgtt gggattatag gtgtgagtta ttatgtttgg 1440
ttgtttaatt tttatttgaa gttttggggt atatgtagag gatgtgtagg tttgttatat 1500
aggtgtgtgt gttatgatgg tttgttgtat agattatttt attatttagg tattaagttt 1560
agtatttttt agttattttt tttggtattt ttttttttta gtattttgtt taataggtat 1620
tagtgtgtgt tgattgttgt tatgtgatta tgtgttttta ttgtttagtt tttatttata 1680
agtgagatta tgtggttttg ttggtttttt gtttttgtgt gagtttgttg aggttaatgg 1740
tttttagttt tatttatgtt tttgtaaagg atatgattat gtttttttta gtggttgtgt 1800
tttaggttat tttttttggt tttgttgttt attttttgtt gatttgtaga tttttattta 1860
ttttagatat tgattttttg ttggttttag atatgataga tagttttttt tattttatta 1920
attgttaagt ttgtttaagg agttttttat gaaataaaat ttgttaattt aagtgtaatt 1980
aaatttagta agggattttt gtggtgggga agaggttggt gtttatgttg tatttttaaa 2040
attttattta atgtagttat taaaaagaat tagattatgt tttttgtggg atatggatgg 2100
agttagaggt tattattttt agtaaattaa tgtaggaata gaaatttaaa tattggatgt 2160
ttttatttgt aagtgggagt taaatgatga gaatttataa tataaataag gaaataatag 2220
atattgtggt tgattttagg gtgtaggatg ggaggaagga gaggagtaga aaagagaatt 2280
attgggtatt tggtataata tttgggtgat gaaatatttt gtataataaa tattgaagat 2340
atgagtttat ttatgtaata aatttttata tgtattttta aaatagaaat aaaagttaaa 2400
aaataaagaa attttattta aaagtttttt ttaagagatt tattattggg ggaaatttag 2460
tttttaatat tttatgtggg ttttttttta ttttttttaa gtgttggttg gtttgagaaa 2520
taaagggaaa gagtataaaa gagagatatt ttaaagttgg gtgtttaggg gagatattat 2580
atgtttgtag gttttgtgat gttttttgag ttgtaaaatt agtgagtttt tattagtaat 2640
ttttaaagag gagggagtgt atgaataggg tgtgggttat agagattata tattttaagg 2700
gtgataaaag attatagggt agaaggttat ggtgaaatta gaattattaa tgaattttta 2760
ggttttattg gtatgtattg ttagggttta agagtagaga attggtttga ttagaatttg 2820
ttaggttgga attttttaat tttattaagt ttgggggtgt tgtaggagat tagggtgtgt 2880
tttatttttt tttatttttg aataaaggta ggtattttta gggaggttat tttagtggtt 2940
tggttttggg aatgtatttt ttttttaggg ttgttaatta ttaatatttt ttattgggga 3000
aagaatttag tgatattttt tttatttgtt tgtggtaata agagaagtat ggttttgttt 3060
tgtttagttt ataggtagtt agattttaag gttatttttt ttgttttttg aaaattgttg 3120
ttattttgtt tttaagatgt ttagatttta tattgtttaa atatataata attgtttttt 3180
ataaataatt tgtgtagtta atgtagttat tatagggttt taaggtgata tatattttta 3240
gtttatgaag atgataggat taagagatta aagataggta taggaaatta taagagtatt 3300
gattggggaa gtgataaatg tttatgaaat ttttataatt tatgtttttt tgttatggtt 3360
ttagttagtt tttttgtttg gggtgtttga ttttttataa tatgtatgtt gttgtagttg 3420
gtttggaatt tttggtttta agtgattttt ttgttttatt tagttttttg aatagttggg 3480
attataggta tgtgttatta tatttagtta atttttgtat tttttgtaga gatggggttt 3540
tattatgttg tttaggttgt tttttttttt tttttttttt ttttaaattt ttattaatgg 3600
attgaggtta taaggaagtt ttttaaattt ttgttatttt gggttttttt taattttatt 3660
ttaattgttt agttttggtg ttttttttgg tttgttaagt tgtttttttg gtttgttttt 3720
tggtttttgg tttgaggttg gttatttttt tttagttttg agaaatgtat ttttattgtt 3780
ttttttgaga tgtttttttt tttgtttttt tttttttttt tttttttttt tttttttttt 3840
tttttttttt tttttgtatt atttggaaaa tggttttttt aatttatttt tttattattt 3900
tattgttttt tttttttttt agataggttg ttattttatt gtttaggttg gagtgtattg 3960
gtattatttt agtttattgt aatttttgtt ttttggttta agtgattttt ttgttttagt 4020
tttttgagta gttgggatta taggtgtttg ttattatatt tggttaattt ttgtattttt 4080
agtagagata gggttttatt atgttggtta ggttggtttt aaatttttga ttttaagtga 4140
tttatttgtt ttgggttttt aaagtgttgg gattataggt gtgagttatt gtgtttggtt 4200
attttgatgt tatttgtgtt ttttttggtt tgtttttttt gggagagagt tttttagttg 4260
attttattat ttattttttt atttatgagt tgtatatttt gtatgattta ttttatttgt 4320
tgttggtttt ttttgattat tgtttttata tttatttttt ttttttgttg ttttttgtaa 4380
ttttttattg ttagtgtttt tttttatgtt tttatgattt tttttgtgtg tattttttgt 4440
ttgtgtgttg tatttattta gtgttagggg atgtttttgt tttttgagat gttttttttg 4500
gaattagtga gtttgtgttt tttttttggt ggtattggtt atttttttgg gtagtatgtt 4560
agtttgtggt tttaaagtaa aggaaggaga taagtattat taaattatag ttaatttttt 4620
ttttttgtta ttgtttttta aatagagttt gaggttagag tttttttttt tgaattttgg 4680
gaagtagtgt tggggaaggt ggttttttat gttttaatgt gttggtgttg ggtgttttta 4740
ggggaagtgt gggagggaga aagtttgttt tatttagttt tattgttttt tttagtgttt 4800
tatttagtag agggtagtag aggttaattt tggtttagtt tgtattaggg ttagtgttta 4860
gtagtgatat ttttttaata attttagaga gtattaataa aggaattgaa tgtaaatttt 4920
ttttttttgg tattttatta tagttatttt tagttatgtt tagtttttat ttatatatat 4980

atttttatgt gtatttattt atatttttgt atatttagat atatataggt ttaatatagt 5040
ttgtagtttt taggaggggt gatgttttat tttatttgtt taattttatt gtttttttga 5100
aatttttgtt tttttttgtg gtttttttta tatttttatt ggagagggta atgaaagttt 5160
atgatagttt attatttaaa ttaaggattg ggatattatg gattgtggtt tattatttgt 5220
ttgtgtaaat aaagttttat tgggttttag ttatgtttat ttatgtgtgt gttatttatg 5280
tttgtttttg tgttatagtg gtttaaatga gtgattgtaa tagagattat gtggttttta 5340
aagtttaaga tatttttttt tttttttttt tttttttgag atggagtttg gttttgttgt 5400
ttaggttgga gtgtagtggt atgattttgg tttattgtaa gttttatttt ttgggtttat 5460
gttatttttt ttttttagtt tttagagtag ttgggattat aggtgtttgt tattatattt 5520
ggttaatttt ttgtattttt agtagagatg gggttttatt gtgttagtta ggatggtttt 5580
gattttttga ttttgtgatt tgtttgtttt agttttttaa agtgttggga ttataggtgt 5640
gagttattg 5649

<210> 311
<211> 12781
<212> DNA
<213> Artificial Sequence

<220>
<223> chemically treated genomic DNA (Homo sapiens)

<400> 311

tattatgttg ttagttggtt ttgaattttt ggttttaagt tatttttttg ttttggtttt 60
ttaaagtgtt gggattgtaa gtgtgagtta ttgtttttgg ttagttggtg atttatttgt 120
ataattgttt aatttattga atatgtatgg tgtgttaagt attgggttga gggttttata 180
atgttttttt atttaatgtt tagtataatt tatgaagaag gtagtgttaa tattattttt 240
gttttataga tgtagaaatt gaggtatagg ttaaataatt tgtttaataa gtttgtgtag 300
tttagtaagt agtttagttg ggatgtgaat ataaattttg aatatagagt ttttaattag 360
taggttagag gtttttaaat atttagtata tttattattt tagtattttt ttgttatatt 420
ttttaggtta aaataatttt attgattgtt atgtaattta ttaagtgtag ttatttgaaa 480
aaaaaatata tataaaagaa aaatattttt atttagtttt taaaataatt tatatatagt 540
tatggaatgt atgtgttggt tgggtattgt agattaaaga tagtttaggg ttgggtatgg 600
tggtttaggt ttgtaatttt agtattttgg gaggttaagt agagagaagg gtttgagttt 660
aagagttgga gattggtttg ggtaatatag taagatttag tttttataga aaataaaatt 720
attagggtgt gaggatgtat atttgtagtt ttagttgttt gggaggttga ggttggagga 780
ttagtttggg taatagagtg agattttgtt ttagaaaaaa aaaaaaaaaa agatagattt 840
ttatttagat atgtatgtag gagtttatag aaaaaaaaag tgagtttagg aggttgttat 900
ttggtattta tataattttt ttttttttga attttgaaat ttattttata tatattattt 960
aataggggaa gaggagggag aaaaagtttt ttatgggaag aataaatagg tttttggggg 1020
aataaaaggg agataagaat gtttgttttt gtaggtgtaa gtggtttttg tttttttttt 1080
tggttattaa aattttttta gagaggagat ttatggtagt tttattttta gaaatttttg 1140
ttgttagttg tataagggaa gttttgaaat ggtatttttt tgtatttgtt ggtttttaaa 1200
tgtttttagt tttaagtaat atttatgtta attttggggg tttgagtgtt tttatataat 1260
atatgtgttt ttttgttttt taatgaagtt tgtgggaggt atttaattgt agtttttaaa 1320
atttggttta taggtattat tgtttttatt aaagatgagg aaataagttt agaaaagtat 1380
taattgtttt gagttatttg tttggttagt taggattaga gtttagtttt ttatttaatt 1440
taaagtttag gtttttaatt ttttataatt ggtgtatttt ttttgaattt ttattttttt 1500
tttgtaaaag aataatattg gttgggtgta gtggtttata tttataattt tagtattttg 1560
ggaggtagag atgggtggat tatgaggtta ggagtttgag attagtttgg ttaatatggt 1620
gaaattttat ttttattaaa aatataaaat ttagttgggt gtgttggtgt ttgtaatttt 1680
agttatttag gagattgagg taggagaatt gttttaattt gggaggtgga ggttgtggtg 1740
agttaagatt gtgttattgt attttagttt tggtgataga gtaagatttt atttttaaaa 1800
aaataaataa taataataaa aagagaataa tgttatattt agttgaatta aaatgaatta 1860
aatattaata tttgtatttt aaaaatggtt tagtttggtt gggtgtagtg gtttttgttt 1920
gtaattttaa tattttggga ggttgaggta ggaggattat ttgaggttag gagtttgaga 1980
ttagtttggt taatatggtg aaattttgtt tttattaaaa atataaaaat tagttgggta 2040
gtagtagtgt gtgttggtaa ttttagttat ttaggaggtt gaggaaggag aattgtttga 2100
gtttgggagg tgaaagttgt ggtgagttga gattgtatta ttgtatatta gtttgggaga 2160
tagagtaaga ttttgtttta aaataaaata attaaaaaat taaaaaggtt tagtttgggt 2220
aatatagtga aattttgttt ttatagaaaa tttttaaaat attagtaggg tattgggtat 2280
agtggtttat atttgtaatt ttagtatttt gggaggttga ggtaggtggg ttatttgtgg 2340
ttaggagttt gggattaggt aggttaatat ggtgaaattg tgtttttatt aaaaaataaa 2400

aattagttgg gtatggtggt aggtattagt aattttagta tttaggaggt tgaggtatga 2460
gaattgtttg aatttgtaaa gtaggggttg tagtgaatta agatggtgtt attgtatttt 2520
agtttgggtg atagaataag attttttaat taaaaaaaaa aaaaattagt tgggtatggt 2580
gttgtgggtt tgtggtttta ggtatttagg aggttgaggt gagaggatta tttaagtttg 2640
ggaggttgag gttatagtaa gttaagatta tgttattata ttttagtttt tgtgatagag 2700
ttagattttg ttttaaaaaa attttaaaaa gggtaaattt tggtaatttt atatagttta 2760
atttagtata aggtggttgt aataattaaa tgagataaaa tggtgttaaa ttggaagtat 2820
tatagtattt ttgttaataa tatagggttt tagaattagt ttttttgagt ttaaatttag 2880
gttttattat tttttagtta tgtagttatg ggtaagttat ttgatttaat tgtgttttag 2940
ttttatttat gatatggaga tataggataa ttagtttttt atgtgtaatt ttgaaattta 3000
aaaagttttg taaattaaaa gtttgggggt aaatttattt ggtagtaaat tttgatttga 3060
ggttatttat agtttatatt ttgtattttt tttatttagt atgaataagt atgtaagttt 3120
tattgtatgt ttgattttag tatgtttttt tagatttttt gggggtgttt atgtatgttg 3180
gtgggggaaa gagattaatt tttaaatatt attttaaata gttggtttta ttgtgtttgt 3240
ttgggtagta tatatattaa aattggaatg atttttgtat agggatgaaa tgtaaatttg 3300
tgaagtatat tgtatttttt ttagtatata ttatttttgg taatattttt tttttttttt 3360
tgagagggag ttttgttttg ttgtttaggt tggagtgtag aggtgtgatt ttggtttatt 3420
gtaagttttg ttttttgggt ttatattatt tttttatttt agttttttta gtagttggga 3480
ttataggtgt gtgttattat gttaggttaa ttttttgtat ttttagtaga ggtggggttt 3540
tattgtgtta gttaggatgg ttttgatttt ttgattttgt gatttgttta tttttgtttt 3600
ttttttgaag tgttgagtgt tgggattata ggtgtgagtt attgtgtttg gtttttgttt 3660
ttttttttta gattgatttt attatttgtt tagtaaagga gaattttttg gtagaatagt 3720
ttttaagaat aaggtaaata attaatttta tataggtttt tattaatgta tagttgttga 3780
ttgtgattgg tttttggtaa tttggatttt ataatttgga taaggggata aataattgtt 3840
tgttttttat tatttttttt gaatttgaat agaatttttt tatttttata gttttttagt 3900
tttttttttt ttttttttga gatggagttt tgtttttgtt gtttaggttg gagtgtagtg 3960
gtgtgatttt ggtttattgt aaatgttgtt ttttaggttt aagttatttt tttgttttag 4020
ttttttaagt agttgggatt ataggtgtat gttattatgt ttggttaatt tttggatttt 4080
tagtagagat gggggtttta ttatgttgat taggttggtt tttaatgttt gattttaggt 4140
gatttgtttg ttttggtatt ttaaagtgtt gggattatag gtgtgagtta ttgtgtttgg 4200
ttttttatag ttttttagtt ttttaaaatt tgaaaaattt tgtaaagata tatttgggtt 4260
aaagggttta gataatggat tgtggttttt aagtatttat gttataggtt attgagagga 4320
ttgatttagt tattagatgt aaaatgttgg gattagtgtt tggtaaagga aaattttgta 4380
tagttgtagg tttttattat atataatagt attgttaatg aatgttatta taatatttat 4440
ttagtgttta ttaagtgttt attttatata aattttgaga atataatgtg aaggtgaatt 4500
gttgattaaa gtttggtttt ttttgttttg tttttttgtg aaaatgtttt aatggtagga 4560
ggttatgtga gtgttggatg tgtttttttt tgtgagtttt tttttgaggt tttttgggtt 4620
tttttggtta tttttgtttg ggtggtgtgt gtttttgttg tttttgtttt tgttttagag 4680
aggtagggtt ttttgagttt gttagttttg tggttgtaat taattttggg ttggagtgtt 4740
ttggtttggt tagttttgtg gtgtgaggga aggggagttt agtagttttt tgtgtggggt 4800
ttaggtgttg gtggtagggt gggtttttta ttgttagtgt gttagttttg tttttattta 4860
ttagtgtgtt agttttgttt ttttttatgt tgttgtgtgt ttgggggtgg ggtttggtgt 4920
gtattgtttg tgtatggtga ggtgtttgtt gattggttat tggggtttgg gtttttttgg 4980
tggagtgtgt ttttttttag attttttgtt agtaggagtt gtgtggtaga tgtggtgttt 5040
ttaggagttt tgtttgatag aatggttggg ttttgttggt tgttggtatg ttgtgataga 5100
gtattttgta tagttttttt tttaagtttt tggtgttgag tgatgttaat aaggttttgg 5160
ttagggtttt atgtgaaggt ggttgtgttg ttgttgtttt tggggttttt ttttttttag 5220
gtggggatgt ggtttggagt gaggttgggt ttgggtttag gtttttggtg tgttttgtgt 5280
tattgtttaa ggtgaagaat tttaatggag ggttgtggag attggtagtg tttgttgttt 5340
ttattaggta gtggggtggg ggtggggttg aaggtggggg tatagtggtg gggtgtttgg 5400
aatttggtga ggggaggttt gtataggggg ttgggggggt gtatggtttg gttttgggtt 5460
tggaggaggt ggggttgtag agttggtttg aatgagtgta ggggttgagt ttggagtatt 5520
tgggggtgta gtttgtaaat agggttggag gagagaggtt gtgtaggaag agggttgtag 5580
gggagatgtg gagagtttgg gttttttgga gggaggagat gtgttttgtt aggtgggggt 5640
gttgggttaa ggaaggggtg atgtgtgtag ttttgggtgg ggagggggtt tgggaggtgg 5700
gagtattggg ggtggggtga gaaggggaag gtgtttggtt tatttggtgg gtggggtggg 5760
gggtggggtg gtgggaagga ggaatgtttg tgggaggttg aatggggaga gtttggtggt 5820
gtggggtgtg aaaggaggtt ttttggttgg tgtggagata gtgagttggg gttttagtag 5880
ttgattgagg tagatattta gaggtagtta agagttgtgg ttgttgagat gttttgggga 5940
tggtgggttt ttggtttagg tgaggggttg ttgagggggt gggttatgag ttgtgagtgt 6000
gggggggtgt gttattatgg ggattgtggg gtttaattgg gtggggtggg gttgtgggga 6060
gttgaagtgt tgggatttgg ttgggttttt tggtaggtag gttgtatgtg tattgagatg 6120
aagatagaat attttgatgt atgtggaaat ttgtgttgag tggaaaatat tttattttat 6180

gaaatagtgt aatttttatg gggtattatt gggtttttag aggttttaat tgggtgttgg 6240
ataaagatgt gtggatgtga gtgattttgg ggaagtttgt tgggagttgt ttttatttta 6300
tgggtagtta agtgtttttt tttttttttt ttttttgaga gagagttttg tttaagttta 6360
ggttggagtg taatggtgtg attttagttt attgtaattt ttgtgttttg gttttaagtg 6420
attttttagt tttagttttt tgagtagttg ggattatagg aatgtgtttt tatattttgt 6480
taattttgta tttttagtag agatggggtt tttttatgtt ggttaggtta gttttggaat 6540
ttttgatttt aggtgattta tttgttttgg ttttaaagtg ttgggattat aggtgttagt 6600
tattgtgttt ggtttgttta gggtttttta tttgggtagt tggtgatatt ttgaaaagtt 6660
tgtttttgtt gtttgttaga tatatatata tgtattttga gatagagttt tgtttttttg 6720
tttaggttag agtgtagtgg tgtgtttttg gtttattata atttttgttt ttggattaag 6780
agattatttt gttttagttt tttaagtagt tgggattata ggtgtgtttt attatgtttg 6840
gttaattttt gtatttttag tagagatggg ttttattatg ttggttaggt tggtattgaa 6900
tttttgattt tttgattggt ttgtattggt ttattaaagt gttgggatta taggtatgaa 6960
ttattgagtt tggttgtttg ttagatatta aatataaagt ttaatggttg ttatttgaat 7020
aaatgaagaa ataaaggttt agaaaaaata atttatttaa gataagagtt agtttgtgtt 7080
ttttgtttgg ttttgttttg aaatggagtt ttgttttgtt gtttaggttg gagtgttgtg 7140
gtgttttttt ggtttattgt aatttttgtt tgttgggttt aagtgatttt tttgttttag 7200
ttttttgagt agttgggatt atgggtgtgt ttattgtggt ttggttgatt ttttattatg 7260
gagttttatt atgttggtta ggttggtttt gaaattgttg attttaagtg gtttatttat 7320
tttagttttt taaagtgttg ggattatagg tgtgagttat tgtgtttggt tgttagttag 7380
tggttttgag taatggattt taaatttatt taaatttagt ttaaagtgtt ttaaaggaat 7440
tttgagattt ttttaagtgt aattatagtg ttatttttgt ttaagtgatt tttttttgta 7500
gtttaaatat atatagttgt gtattagtag aatatgtttg tggggaatag agttagtatt 7560
tgtaataata aatttttggt tagaaaagta tgatgtattg tttatttgag tatgaattga 7620
ttgttgaatt taaattaaat gggtgtattt attgtaagga tgtattttat atttatattg 7680
aatagtgtat gttatttgtt ttttgaggtt gagtttaaga gatttgtaaa aataaaatgt 7740
atatatttta ttttttgtta tggagaggat tttagggtat ttaagaaaga tgggtatttg 7800
atattaggtt tttaaagaaa ttgtagtgtt tagattattt tgttgagtat agtattaggt 7860
attatggatt ttggatataa ttatgaatag gataaagtaa agaggtaatt gtagatttta 7920
agtggaaagg ggatggagag gatgtgggtt aggttaggtt tttagttttg taaattgaaa 7980
ttagaaggat aaataagttt agatagatta tagtgagagt gggaagttgg tttaggaaga 8040
ggaaggtttg taaattgtgg gtaggatgaa aggaggagga gggagtattg gagaagttaa 8100
gtagagattt aattatgaat agtttgatga gttatagaga tatttggatt tattttatga 8160
attatttaag ttttaaaata tgttgagtgt attttttttt tttttgagat ggaattttat 8220
ttttgttgtt taagttggag tgtagtggtg tggttttagt ttattgtaat ttttgttttt 8280
tgggttttag tgattttttt gttttatttt tttaagtatt tggtattata ggtgtttgtt 8340
attatgttta gttaattttt gtgtttatag tagagatggg ttttattatg ttggttaggt 8400
tagttgtgaa tttttgattt taggtgattt atttatttta gttttttaaa gtgttgggat 8460
tataggtgtg aattattgta tttggttgtg agttattgtg tttgtttgag tattttttaa 8520
tgtttgttat ttagattttt ttttgtgttg aagtgtttgt tttttgttgt tttttttttt 8580
ttttttagtt ttttgttatt tgtgtgtgat ataaatgttt ttttttataa tgagtttttt 8640
tatttagttt atggttttgt tgttgttgtt gaataataga ggttttattt tgttgtttag 8700
gttggtgttg aatttttgtt tttaagtgat ttttttattt tagtttttta atttgttggg 8760
attataagtg tgagttatta tatttagttt tatggtattt tttgatgaat aaattattga 8820
ttataatgtg gaatttgttt ttttattttt tttgtggtta gtgtttttat aggttttatt 8880
taagaaattt atagggaggt tgggtgtagt ggtttatgtt tgtaaattta atattttggg 8940
aggttaaggt aggttaatat ggttagattt tgttttttta aaaaataaga aaattagtta 9000
ggtatggtgg tgtgtgtttg tagttttagt tttttgggag attgagatgg gaggattgtt 9060
tgagtttagg aggttgaggt tgtagtaagt taagagatta tgttatgtat tttagtttgg 9120
gtggtagagt tagattttgt ttgttaaaaa ataaaataaa agttggtgaa aatgttgatt 9180
atatatttta ggaataatta gtaattgata ttaaaattat gggttaaaga gaaagtaaaa 9240
ataatgtgat tttaaattag aatttaaaag atttgtttag tgtatgttta gataaagtta 9300
ttatttatta tattaaagtt ttaatattta ttttgtgagt tgttagtttt tttttttaat 9360
attttttttt attgttttaa aaaattttaa gaatattgga tatttaagat ttatttaaag 9420
ttttaaaagt atttgtaaaa ttttaaaatt ataatttaag gtgtttttgg agggtaggag 9480
taatggtggt aggtagtgtt ttgttttgtt gtttaggttg aagtatagtg gtagattttg 9540
gtttattgta tttttgattt atttggttta agtgattttt ttattttagt tttttgagta 9600
gttgggattt taagtgtata ttattttatg tttggttaat ttttaaattt ttttgtagaa 9660
ataaggtttt attgtgtagt ttagatggtt ttgaattttt gggtttttaa gagatttttt 9720
taaagtgttg ggattatagg tgtgagttat tatatttggt ttattttggt atttttgaaa 9780
attgtaggat tattatggat aaaattttaa aattattttt aaagaattta ggtttatata 9840
taaaaaaaat ttggtttgtt agtagtgagt gaagaaaaat tttgagaaat gtttaaaatt 9900
tttagttttg ttatataata tattttatta tttgtttaat tatttttttt gatttagaaa 9960

ttagtttttt gggtttagga tgtttagtgg tatttttttt ttttaagttt tttagtattg 10020
gaggaattgt atattagtaa aatttttagt tttagtattt tatagtttat tgttattttt 10080
ttttttttat tttttttttt tttttttttt tttttttttt ttttttgaga tggagttttg 10140
ttttgttatt taggttggag tgtagtggta tgattttggt ttattgtaat ttttgttttt 10200
taggtttaaa tgattttttt gttttagttt tttgagtagt tgggattata gatgtttgtt 10260
attatgttta gttaattttt atttttttag tagagatggg gttttattat gttggttagg 10320
ttagttttga atttttgatt ttgtgattaa tttgttttgg ttttttaaag tgttgggatt 10380
ataggtgtga gttattgtgt ttagtttttt tttttttttt tttttttttt tttttttttt 10440
tgagatggag ttttgttttg ttatttaggt tggagtgtag tggtatgatt tgggtttatt 10500
gtaattttta ttttttgggt ttaagggagt tttttgtttt agttttttga gtagttggga 10560
ttataggtgt ttgttattat gtttagttaa tttttgtatt tttttagtag agatggggtt 10620
ttgttatgtt ggttaggttg gttttgaatt tttgatttta ggtgatttgt ttgttttgtt 10680
tttttaaaat gttgggatta taggagtgag ttattgtgtt tggtttagta tattgttatt 10740
tttttttttt tttttttttt tttttttttt tttttgtttt tttttttttt tttttttttt 10800
tgtgagatgg agtttgttgt ttaggttgga atgtagtggt gtttttttgg tttattgtaa 10860
tttttgttgt ttgggtttaa gtgatttttt tgttttaggt ttttaagtag ttgggattat 10920
aggtttttgt tattgtattt ggttaatttt tgtatttttg gtagagatgg ggttttatta 10980
ttttggttag gttggttttg aatttttgat tttgtgattt atttgttttg gttttttaaa 11040
gtgttgggat tatagatttg agttattgta tttggtttat attgttattt tttaatagta 11100
gagaaattat gtgttagatt ttgtaagtgt aatggtatat aaaggataaa atgatgttga 11160
aaaataaaat tttttgttta aatgtttatg tttttaatat tttattttag aaaggaattt 11220
attttaaaat tgataatggt tggatttagt tttttatata aatttttttt ttttagtgag 11280
gatgtatatt tattttgtaa ataaatggaa gatattattt ttttaattgt ttgtttagaa 11340
atgaaataat ttttttttaa tgatttttta aagtatgaga ttttatatat tatttaaaat 11400
aatttatatt gtattttata tatgataaag ttttaaggta atagtttttt tttaagatta 11460
aagttatagt tttttttttg tatttgaggg ggattggttg taggattttt ttgtgaatat 11520
ttaaattttt ggatgtttaa gttttttatg agatgtagta tttgtatata atttatatat 11580
attttttttt gtattttatt tttagattat gtataatatt taatagaatg taaatgtttt 11640
gaaattagtt gtttagttgt attttaaatt ttgtattttt tttttttttt ttttgagata 11700
gagttttgtt ttgttgttta ggttggagta tagtatagtg attatagttt attgtatttt 11760
taatttttta ggtttaagtt gttttgtttt ggttttttta agtgttggga ttataggtgt 11820
gagttattat atttggttat tgttttttat tggttttaaa tttttgattt aaaattttta 11880
atttaggttg gttgaatttg gattggaatt taaggatatg tttgttgagt atattgtatt 11940
tattttggaa tataattaga atgtttaatt tgtatgttaa aaatatttta tttggttagg 12000
tgtggtggtt tatgtttgta attttagtat tttgagaggt taaggtgggt gaattatttg 12060
aggttaggag tttaagatga gtttggttaa tatggtaaaa ttttgatttt ataaaaaaaa 12120
ggtaaaaata agttaggtgt gatggtgtgt gtttgtagtt ttggttattt aggaggttga 12180
gatataagaa ttgtttgaat tggggaggta tgttatgttt ttagttgttg atttgagttt 12240
ttttgtagtt tgtaggggga gggtaataga gtattaggta gtttttggaa tatataggag 12300
tgtaattgga aaaagatttt aagtaagttt aatgaattag ataatttatt taattagtaa 12360
attatgtaat tagtatgttt tataataata ttgtgagtta gattttgttt ttgatatgta 12420
tatattatat tgtataggtg ttattaattt ggagagtata tatagtgagt ttatgttttt 12480
tttttgttat tagtattata ttaaaatttt gttatggttt ttaaattttg attttgagaa 12540
agttttttat tttaataata taattatatt tgtgtttgtt tttatagtta aatatgtatt 12600
atgatattag tttgtatata ttttttaaat gatttgaata tttgatttta aaaattattt 12660
tagaattttt gtgttttaat attaatgtta gaagatttgg aattgtttat ttgtaggtaa 12720
ttgtttttaa ggaaatttga ttaaatatta attaagttat gtattttttt ttggtaatag 12780
t 12781

<210> 312
<211> 12781
<212> DNA
<213> Artificial Sequence

<220>
<223> chemically treated genomic DNA (Homo sapiens)

<400> 312

attgttgtta aaaggaaata tataatttag ttaatatttg gttaagtttt tttaaaggta 60
gttatttata aataaataat tttaagtttt ttggtattaa tattgaagta tagaaatttt 120
agaataattt ttaaagttag atatttaagt tatttaaaaa atgtatgtaa gttgatatta 180
taatgtatat ttaattatga agataaatat aaatatagtt atgttattag ggtgagaaat 240

ttttttagaa ttaaagttta aaaattatgg tagaattttg gtataatgtt gatggtagga 300
aaaaggtatg gatttattgt atatgttttt taaattagta gtatttatat aatatggtat 360
gtatatatta gaaataggat ttaatttata atattattat aaagtatatt gattatataa 420
tttattaatt aggtaaatta tttaatttat tagatttgtt tggaattttt ttttagttat 480
atttttatgt attttaaaag ttatttaata ttttattatt ttttttttat aaattatgga 540
aaaatttaag ttaataattg agggtgtaat gtgttttttt ggtttaagtg atttttgtgt 600
tttagttttt tgaatagtta agattatagg tatatgttat tatatttggt ttatttttat 660
tttttttttg tagagttagg gttttgttat gttggttagg tttgttttaa atttttgatt 720
ttaaatgatt tatttgtttt ggttttttaa agtgttggga ttataggtgt gagttattgt 780
gtttggttaa ataaagtatt tttaatatat aagttaagta ttttagttgt attttaaagt 840
aaatatagta tgtttaataa atatattttt gggttttagt ttagatttaa ttaatttaga 900
ttaaaaattt taaattaaaa atttaaaatt aataaaaaat agtgattagg tatgatggtt 960
tatatttgta attttaatat ttggggaggt tgaggtagga tagtttgagt ttgggaggtt 1020
aaaggtgtag tgagttgtga ttattgtatt gtattttagt ttgggtaata gagtaagatt 1080
ttgttttaaa aaaaaaggaa aaaaaatata aaatttaaaa tatagttgaa taattaattt 1140
taaagtattt atattttatt aggtattgta tgtaatttag agataaagta taggggaaga 1200
tgtgtatagg ttatatgtaa atattatatt ttataaggga tttggatatt taaggatttg 1260
ggtatttata ggggggtttt gtaattaatt ttttttagat ataaagggag gattgtaatt 1320
ttagttttag aaaagggttg ttattttaga attttgttat gtgtaaaata tagtataaat 1380
tgttttaaat gatgtatgag gttttatgtt ttaaaagatt attagaaaag aattatttta 1440
tttttaagta agtaattaaa aaaataatgt tttttatttg tttataggat aaatgtgtat 1500
ttttattagg aaaaaaaagt ttgtgtgaaa agttggattt aattattatt agttttgaaa 1560
taaatttttt tttgaaataa aatattagaa atataagtat ttaaataaaa aattttgttt 1620
tttaatatta ttttattttt tatatattat tatatttata gaatttgata tataattttt 1680
ttgttgttaa gaaatagtag tatgggttgg gtgtggtggt ttaggtttgt aattttagta 1740
ttttgggagg ttaaggtagg tggattatga ggttaggagt ttaagattag tttggttaag 1800
atggtgaaat tttgttttta ttaaaaatat aaaaattagt taagtgtagt ggtagaggtt 1860
tataatttta gttatttggg agtttgaagt aggagaatta tttgaatttg ggtagtagag 1920
gttgtagtga gttaagaaaa tgttattgta ttttagtttg gatgatagat tttgttttat 1980
aaaaaaaaaa aaaaaaaaaa aaaaataaaa aaaaaaaaaa ggaagaagaa agaaagaaag 2040
aaatagtagt atgttgggtt gggtgtagtg gtttattttt ataattttag tattttggga 2100
ggatgaggta ggtagattat ttgaggttag gagtttaaga ttagtttggt taatatggtg 2160
aaattttatt tttattaaaa aaatataaaa attagttggg tatggtggta ggtgtttgta 2220
attttagtta tttgggaggt tgaagtagga gatttttttg aatttgggag gtggaggttg 2280
tagtgagttt agattatgtt attatatttt agtttgggta atagagtaag attttgtttt 2340
aaaaaaaaaa aaaaaaagaa aaagaaaaag aaaaaggttg ggtatggtgg tttatgtttg 2400
taattttagt attttgaaag gttaaggtgg gttgattatg aggttaggag tttgagattg 2460
gtttggttaa tatggtgaaa ttttattttt attaaaaaaa taaaaattag ttgggtatgg 2520
tggtgggtat ttgtaatttt agttatttgg gaggttgaag taggagaatt atttgaattt 2580
ggaaggtaga ggttgtagta agttgagatt gtgttattgt attttagttt gggtgatagg 2640
gtgagatttt attttaaaaa aaaaaaaaaa aaaaaaaaaa agaaagaaag aatgaaagaa 2700
aagaaatagt agtaagttat aaaatgttaa gattaaaaat tttattaata tgtagttttt 2760
ttaatgttaa aaagtttgaa gaaaaagagt attattaaat attttggatt taggaaattg 2820
gtttttgagt taaaaaagat aattaaatag gtagtaaaat gtattgtgta ataaaattaa 2880
aaattttaaa tattttttaa aatttttttt tatttattgt taataaatta aatttttttt 2940
gtgtgtaaat ttgaattttt taaaggtaat tttaaaattt tatttgtggt aattttgtgg 3000
tttttaagaa tgttaaaata ggttaggtgt ggtggtttat atttatgatt ttagtatttt 3060
gggaggattt tttaagagtt taggaatttg agattatttg ggttatatag tgagattttg 3120
tttttataaa aaaatttaaa aattagttag gtatggggtg gtgtgtattt ggggttttag 3180
ttatttagaa agttgaggtg ggaggattat ttgagttgaa taggttgagg gtgtagtgaa 3240
ttgagatttg ttattgtatt ttagtttggg taataaagta aaatattgtt tgttattatt 3300
gtttttgttt tttaaaaata ttttaaatta tgattttagg attttgtaag tgtttttaaa 3360
gttttaagtg agttttaaat gtttggtatt tttggggttt tttaagatgg tggggaaaaa 3420
tgttaagagg aaaagttgat agtttataaa ataaatgtta aaattttgat ataataagta 3480
atggttttgt ttaaatatat gttaaataga ttttttgaat tttggtttaa aattatatta 3540
tttttgtttt ttttttagtt tataattttg atgttaatta ttagttgttt ttaaaatata 3600
taattaatat ttttattaat ttttatttta ttttttggta gatagggttt gattttgtta 3660
tttaggttgg agtgtatggt atgatttttt ggtttattgt agttttaatt ttttggattt 3720
aagtgatttt tttattttag ttttttaaga agttgggatt ataggtatat gttattatgt 3780
ttggttaatt tttttatttt ttggagagat agggtttagt tatgttggtt tgttttggtt 3840
ttttaaagtg ttgggtttat aggtatgagt tattgtattt agtttttttg tggatttttt 3900
aaataaaatt tatagaaata ttaattatag agaaaataaa aggataaatt ttatattata 3960
attaataatt tgtttattga aagatgttat aaggttgggt gtggtggttt atatttgtaa 4020

ttttaatagg ttgggaggtt gaagtgggag gattgtttga gagtaagagt ttaatattag 4080
tttgggtaat aaagtgagat ttttattatt taataataat aataaagtta taaattaaat 4140
gaaagaattt attgtgaaag aagatattta tattatatat aaatgataaa gaattaggaa 4200
aaaaaaaaga aatagtaaaa gataaatatt ttagtataag aagaaattta aatgataaat 4260
attaaaagat gtttggatag atatggtggt ttatggttag gtgtggtggt ttatgtttgt 4320
aattttaata ttttgggagg ttgaggtggg tggattattt gaggttagga gtttatgatt 4380
ggtttgatta atatggtgaa atttgttttt attataaata taaaaattag ttgggtgtgg 4440
tggtaggtat ttgtaatatt aggtatttga gaggatgagg taggagaatt gttggaattt 4500
aggaggtgga ggttgtagtg agttgagatt atattattgt atttagtttt gggtaataag 4560
agtgaaattt tgttttaaaa aaaaaaaaaa tatgtttaat atgttttaag gtttaaatga 4620
tttatggagt aagtttgaat gtttttgtag tttattagat tgtttatgat tggatttttg 4680
tttaattttt ttaatgtttt tttttttttt ttttatttta tttataattt gtagattttt 4740
ttttttttga attagttttt tatttttatt ataatttgtt taagtttatt tgtttttttg 4800
gttttggttt atagagttga gagtttagtt tgatttgtat tttttttgtt ttttttttat 4860
ttggagttta taattgtttt tttgttttgt tttgtttatg gttgtattta ggatttataa 4920
tgtttaatgt tgtgtttggt agagtgattt agatattgta gtttttttag aaatttagta 4980
ttaaatgttt attttttttg aatattttgg aatttttttt ataatgggag gtgaaatgta 5040
tatgttttat ttttataagt tttttaaatt taattttaag aaataaataa tatatgttat 5100
ttaatatgaa tgtaaagtat atttttataa taaatatatt tgtttggttt ggatttaata 5160
attaatttat gtttaagtaa ataatatgtt atgttttttt aattaggagt ttgttattgt 5220
ggatgttggt tttgtttttt ataagtatat tttattaatg tataattata tatatttaaa 5280
ttgtgggaaa gggttgttta aataagggta atattataat tatatttaga aaaattttag 5340
aattttttta ggatatttta aattggattt aaatggattt gaaatttatt atttaaaatt 5400
attaattagt ggttgggtat ggtggtttat atttgtaatt ttagtatttt gggaggttga 5460
agtgggtgga ttatttgagg ttagtagttt taagattagt ttggttaata tggtgaaatt 5520
ttatggtgaa aaattagttg gattgtggtg ggtatatttg taattttagt tatttgggaa 5580
gttgaggtag gagaattatt tgaatttggt gggtagaggt tgtagtgagt tgagaaagtg 5640
ttatagtatt ttagtttggg tgatagagtg agattttatt ttaaaataaa attaaataaa 5700
aaatatgaat tggtttttat tttgaatgag ttattttttt tgagttttta tttttttgtt 5760
tgtttaaata gtgattatta aattttgtgt ttagtatttg ataaatggtt gggtttggtg 5820
gtttatgttt gtaattttag tattttggta ggttaatgtg ggttgattaa gaggttagga 5880
gtttgatatt agtttggtta atatagtgaa atttgttttt attaaaaata taaaaattag 5940
ttaggtgtgg tggggtgtat ttgtagtttt agttatttgg gaggttgagg taggataatt 6000
ttttgattta gagatagagg ttgtggtgag ttaagaatgt gttattgtat tttagtttgg 6060
ataaaagagt aagattttgt tttaaaatat atatatatgt atttggtaaa tagtaaaagt 6120
aagtttttta aaatgttgtt agttgtttgg ataaaaagtt ttaaatagat tgggtgtggt 6180
ggttagtgtt tgtaatttta gtattttgag gttgaggtgg gtggattatt tgaggttggg 6240
aattttgaga ttagtttgat taatatggag aaattttgtt tttattaaaa atataaaatt 6300
ggtggggtgt gggggtgtat ttttgtaatt ttgattattt gggaggttga agttggggaa 6360
ttgtttgaag ttgggatgtg gagattgtgg tgagttgaga ttgtgttatt gtattttagt 6420
ttggatttga gtgaaatttt tttttaaaaa ggaaaaaaaa aaaaaagtat ttaattgttt 6480
ataaagtgag gataattttt gataggtttt tttggagtta tttatgttta tatatttttg 6540
tttagtgttt gattaaggtt tttaaaagtt tagtggtgtt ttataaaaat tatattattt 6600
tataaaataa aatatttttt atttgatatg aatttttata tatgttaaaa tattttattt 6660
ttgttttggt gtatgtgtag tttatttatt gaaggattta gttggatttt agtattttgg 6720
ttttttatgg ttttgttttg tttaattagg ttttgtggtt tttatggtga tatatttttt 6780
tgtgtttgta gtttgtggtt tatttttttg gtggtttttt atttaggttg aaggtttatt 6840
gtttttaagg tgttttggtg attgtggttt ttaattattt ttaagtgttt attttgattg 6900
attattggag ttttaattta ttatttttgt gttggttgag gaattttttt ttgtatttta 6960
tattattgga ttttttttgt ttggtttttt gtaggtattt tttttttttg ttattttgtt 7020
ttttgttttg tttattaagt gggttgggtg tttttttttt tttgttttat ttttagtgtt 7080
tttatttttt aggttttttt tttatttgga gttgtgtgtg ttgttttttt tttagtttag 7140
tatttttatt tggtgggatg tgtttttttt ttttaaaagg tttgaatttt ttgtgttttt 7200
tttgtagttt tttttttgta tagttttttt tttttgattt tgtttataag ttatattttt 7260
aaatgtttta gatttgattt ttgtatttat ttaagttaat tttgtggttt tgtttttttt 7320
gagtttaggg ttaggttgtg tattttttta attttttgtg tgagtttttt tttgttgggt 7380
tttaagtgtt ttgttgttat gtttttgttt ttgattttat ttttattttg ttatttggtg 7440
ggggtagtag gtgttattga tttttgtagt tttttgttga ggttttttgt tttgggtggt 7500
gatgtggagt gtgttaaggg tttgggttta ggtttagttt tgttttaggt tgtatttttg 7560
tttggggaag gagaggtttt gggggtagtg gtggtatggt tgtttttgtg tgaggttttg 7620
gttgaggttt tgttggtatt atttagtgtt ggagatttgg ggaagaagtt gtatagggtg 7680
ttttgttgtg atatattgat agttggtaag gtttaattgt tttgttggat ggagttttta 7740
aaagtattgt atttattgtg tggtttttgt tggtgggaaa tttgggggga ggtgtgtttt 7800

gttggaggaa tttgggtttt agtggttaat taataggtgt tttgttgtgt gtgggtggtg 7860
tgtgttaggt tttgtttttg ggtgtgtggt gatgtgggga agggtggggt tggtatattg 7920
gtgggtaggg gtggggttgg tatgttggtg gtgaggggtt tgttttgttg ttgatgtttg 7980
ggttttgtgt ggggaattgt tgagtttttt tttttttatg ttgtggggtt ggttgggttg 8040
gaatattttg atttggggtt agttgtggtt gtggggttag taggtttgga aagttttgtt 8100
tttttggagt ggaagtggga atagtggggg tgtgtattgt ttgggtgggg ataattgggg 8160
ggatttgaaa ggttttggaa aggggtttgt ggggagaaat gtgtttagtg tttgtgtgat 8220
tttttgttgt tagagtattt ttgtaaggag atggagtgaa agggattaaa ttttagttag 8280
tagtttattt ttatgttgta tttttaagat ttgtatagag taggtatttg gtaaatgtta 8340
aatgaatatt gtaatagtat ttgttagtga tgttgttgtg tatggtgaaa gtttgtagtt 8400
gtataaagtt tttttttgtt aggtattgat tttagtattt tatatttggt aattaaattg 8460
atttttttaa taatttgtga tgtaagtatt taagggttat agtttgttat ttgagttttt 8520
tgatttaggt gtgtttttat aggatttttt aaattttaga aagttaagag attatgagag 8580
gttgggtgta gtggtttatg tttgtaattt tagtattttg ggatgttgag gtgggtggat 8640
tatttgaggt taggtgttga agattagttt agttaatatg gtgaaatttt tgtttttatt 8700
aaaaatttaa aaattagttg ggtgtggtgg tatgtgtttg taattttagt tatttgggag 8760
gttgaggtag gagaataatt tgaatttggg aggtagtgtt tgtagtgagt taaggttgtg 8820
ttattgtatt ttagtttggg tgataagagt gaaattttgt tttaaaaaaa aaagaaagaa 8880
agttaagagg ttatgagaat aaaaaggttt tatttaaatt taagaaagat agtggaagat 8940
agataattgt ttgttttttt atttagattg tgaaatttag attgttggaa attagttata 9000
attaatagtt gtatattggt aaaaatttat gtaaaattag ttgtttgttt tgtttttgga 9060
gattgttttg ttagaaggtt tttttttgtt aggtaagtaa taaaattaat ttaaaaaaaa 9120
aaggtggggg ttgggtgtag tggtttatgt ttgtagtttt agtatttggt attttggggg 9180
gggggtggag gtgggtggat tatgaggtta ggagattgag attattttgg ttaatatagt 9240
gaaattttgt ttttattaaa aatataaaaa attagtttgg tgtggtagta tatgtttgta 9300
gttttagtta ttggggaggt tgaggtagga gaatggtgtg aatttgggag gtggagtttg 9360
tagtgagttg agattgtgtt tttgtatttt agtttgggtg atagagtaag attttttttt 9420
aaaaaaaaaa aaagaatatt gttaaaggtg gtatgtgtta agaaaaatat agtatgtttt 9480
atgaatttgt attttatttt tgtgtagggg ttattttaat tttggtatat gtgttattta 9540
agtaagtata gtgaaattaa ttgtttgaga taatatttga gagttggttt ttttttttta 9600
ttggtatatg taaatatttt tagagagttt gggggaatat gttgaaatta aatatgtagt 9660
aaaatttata tgtttattta tattaagtag aaagaatata gaatatagat tataaatagt 9720
tttaggttaa aatttgttat taaatgagtt tatttttaaa tttttggttt atagagtttt 9780
ttggatttta gaattgtatg taagaggttg gttattttgt atttttatat tatggatgaa 9840
gttgaggtat agttgggtta agtaatttgt ttatgattgt atagttagga agtggtggag 9900
tttggattta aatttaagga agttggtttt ggagttttat gttgttaata gaaatattat 9960
aatattttta atttaatatt attttatttt atttagttat tataattatt ttatattagg 10020
ttgaattatg tgaaattgtt aaaatttgtt ttttttaaaa tttttttgag atagggtttg 10080
gttttgttat agaggttgga gtatagtggt gtgattttgg tttattgtag ttttaatttt 10140
ttaggtttaa gtaatttttt tattttagtt ttttgagtat ttgggattat aggtttgtaa 10200
tattatgttt agttaatttt tttttttttt aattgaggag ttttattttg ttatttaggt 10260
tggagtatag tgatgttatt ttggtttatt gtaatttttg ttttgtgggt ttaggtaatt 10320
tttatgtttt agttttttga gtgttaggat tattggtgtt tattattatg tttagttaat 10380
ttttgttttt tagtagagat atggttttat tatgttggtt tgtttgattt taaatttttg 10440
attataagtg atttgtttgt ttttagtttt taaagtgttg ggattatagg tatgagttat 10500
tgtgtttggt gttttgttag tattttaaaa attttttgta gagatgaagt tttattatgt 10560
tgtttaagtt ggattttttt ggttttttgg ttgttttgtt ttgagatagg gttttatttt 10620
gttttttaga ttggtgtgta gtagtgtagt tttagtttat tataattttt attttttaag 10680
tttaagtaat tttttttttt tagttttttg aatagttggg attattggta tgtgttatta 10740
ttgtttagtt aatttttgta tttttagtag agataggatt ttattatgtt ggttaggttg 10800
gttttaaatt tttgatttta aatgattttt ttgttttggt tttttaaaat gttgggatta 10860
taggtgggag ttattgtgtt tagttaagtt ggattgtttt tgaagtataa atattaatat 10920
ttaatttatt ttggtttaat tgaatataat gttatttttt ttttgttgtt gttgtttgtt 10980
tttttgggga tggagttttg ttttgttatt gaggttggag tgtaatggta tgattttggt 11040
ttattataat ttttgttttt taggttaagg taattttttt gttttagttt tttgagtagt 11100
tggggttata ggtattaata tatttagtta agttttgtat ttttagtaga gatggggttt 11160
tattatgttg gttaggttgg ttttaaattt ttgattttgt gatttgttta tttttgtttt 11220
ttaaagtgtt gggattatag atgtgagtta ttgtgtttgg ttgatgttat ttttttatag 11280
ggaggaaatg gaggtttaga ggggatgtgt tagttgtaag aggttaagag tttgggtttt 11340
gggttaaatg gagaattgag ttttgatttt agttagttaa gtagataatt tggagtaatt 11400
aatatttttt tgaatttgtt tttttgtttt tgataaggat agtagtattt atgggttaaa 11460
ttttggaggt tatagttaga tgttttttat aaattttatt aaaagataag agaatatatg 11520
tattatgtgg ggatatttag atttttagaa ttggtatgaa tgttatttgg aattgaagat 11580

atttgagagt taatagatgt agaaagatgt tgttttaaag ttttttttat gtgattaata 11640
gtagaaattt ttgggagtga agttgttgta aatttttttt ttaggggagt tttagtggtt 11700
agaaaaaaag ataaagatta tttgtatttg taaaaataaa tatttttatt ttttttttgt 11760
ttttttagaa atttatttgt tttttttatg gaaggttttt tttttttttt ttttttttat 11820
taaatggtat atataaagta gattttgaga tttaagaaaa aaaaagttgt ataaatgtta 11880
aataatagtt ttttggattt attttttttt tttgtgaatt tttgtatgta tatttgaata 11940
aaagtttgtt tttttttttt tttttttttg agatagggtt ttattttgtt gtttaggttg 12000
attttttagt tttagttttt taagtagtta agattgtagg tgtgtgtttt tatattttga 12060
taattttatt ttttgtagag attgggtttt gttatgttgt ttaggttggt ttttaatttt 12120
tgggtttaag tttttttttt tatttggttt tttaaagtgt taggattata ggtttgagtt 12180
attgtgtttg gttttaaatt gtttttagtt tgtagtgttt aattaatata tatattttat 12240
gattatatat gggttatttt gaaaattgaa taaaaatatt ttttttttat atgtattttt 12300
tttttaaatg gttatattta gtaaattgta taataattaa tggagttgtt ttggtttagg 12360
agatgtggta ggaaagtatt aaggtagtaa atatattagg tgtttgggaa tttttggttt 12420
attggttaag agttttgtat ttaaagtttg tgtttatatt ttagttgagt tgtttattaa 12480
attgtatgag tttgttgggt aagttattta gtttgtgttt tagtttttat atttgtaaaa 12540
tagggatgat attaatatta ttttttttat gggttgtgtt aagtattgag tgaaagggta 12600
ttatgaagtt tttagtttag tgtttggtat gttatatgta tttaataaat tggatagtta 12660
tataaataaa ttattaattg gttagggata gtggtttata tttataattt taatattttg 12720
ggaggttaag atagaaggat gatttgaggt taggagttta agattagtta gtaatatagt 12780
g 12781

<210> 313
<211> 1631
<212> DNA
<213> Artificial Sequence

<220>
<223> chemically treated genomic DNA (Homo sapiens)

<400> 313

ggatttagaa tatggggtgg atttaggtag tggtgggaat taggtggatt tgtttggatt 60
gaagtttttg ttaggtattg aagttgtgaa gttgtttgtg tagtaaaggg agtttttggg 120
gggtgtttga gattttggtg ttgtttgggg gttgttggta gttgtgtttg ttgttttttg 180
tttggtgatt tagttggtat ttttggtggg gttatggggg ttagaggata gtgtgggggg 240
tggtgagtgt ggttgttaag ttttttaagg ttttggaggg ataggatttg ggtgtatttt 300
attggttgtt atggtaatgt agtgttttag ttttttgtgt tttgtggtgg agggaggtgt 360
agttaattgg aagtggtgga gtttttgggg attttttttt ttttgtttta tgttgtgtgt 420
gggtgggggt taggaggtgt ggggtggagg tggagtgtgg gttgtggaga tttggtgttt 480
gtaggaggtt tggtgtgtgg tggggttgta tggggttatt tttggagtag tattgtgagt 540
agtggtgtga tagtgtgggg tttttttttt tttagaagag atgttattta tataaatttg 600
atttttatgt ggggtgtggg attttgtggg gttttagttg taggtgtttt tgttgttttt 660
tttgggtggg tgggttggag aagaagttta tgtgggattt tttaaaatgg tgtatagggg 720
gattttaggg ttttatggtt atttttttgg attttttgga ggtgttgttt agttattttt 780
agttgttttg atgatttgtt gtgggttttt ttaggagatt tgaaagttta gggtgggttg 840
tgtgggtaat atttttgttt aggtttttgg agggttttat gtggttgttt ttggagtatt 900
ttagagtgtt agggttgtgg gaaggggtgg gtgtgttttt ggaaatatga gtgtttgtta 960
gtattagtgt ttggataggg ttggggataa attagtttat gttgtgtata ttgaatttat 1020
gtgtttgagt gagggtgggt gtgttgggga gtgtgtttaa gtggatagtg ttgtatagta 1080
atgtttatgg ggagttttag gagagtttgg ttatttttgt gtaggataat ttttttttta 1140
ttatttgagt tttgtgtttg tgggtaggat ttttttgaat tggggttgtg tgtttagaaa 1200
tatgtatatg ggagtgttta gtttaaagtt ttagggtttt tgggatttgt gtgtttgggg 1260
ttggggtttt aggtgggtat aggtgggagg gtgaatttgt gggtagggtg ggtttttttt 1320
tgtgggttta gtatttgtgt gtttttagtt taggtgtgtg ggaggaaggt agtggtttgt 1380
tgtgtagagt tttgtgtgtt tgtgaggtgg tgttatagtt gtagtagtgt ttgttggttt 1440
gggttgtttt agataagagt gtgtggaaag tgtggtgggg ttgagatgtg attaggatgt 1500
ggggaggatg gattagtagg atagattgat tgggggtttg gtgggtggag ggtagtgtag 1560
ttatgttttt tttggatttg ttgttagttg ggtttggggt ttttgatatg ttttttaggt 1620
gggtttttga g 1631

<210> 314
<211> 1631

<212> DNA
<213> Artificial Sequence

<220>
<223> chemically treated genomic DNA (Homo sapiens)

<400> 314

tttgaggatt tatttggggg gtatgttgaa agttttgggt ttggttgatg gtggatttag 60
gggggatgtg gttgtgttgt tttttgtttg ttgggttttt ggttggtttg ttttgttggt 120
ttgttttttt tgtgttttgg ttgtgtttta gttttgttgt gttttttgta tatttttatt 180
tggagtggtt tgggttggtg ggtgttgttg tggttatggt gttattttgt gggtgtgtag 240
ggttttgtgt ggtaggttgt tgtttttttt ttgtgtattt gagttggagg tgtatagatg 300
ttgagtttgt ggggaggggt ttattttatt tgtaggtttg tttttttatt tgtatttatt 360
tgggattttg attttggata tgtgagtttt agaaattttg gggttttgag ttgagtgttt 420
ttatgtatgt atttttaggt atatagtttt agtttggaaa agttttgttt gtgagtatgg 480
gatttgggtg gtgggggaga ggttattttg tgtaggagta gttgagtttt tttggaattt 540
tttgtagata ttattgtatg gtattgttta tttggatgta ttttttaata tatttatttt 600
tatttaggtg tatggatttg gtgtgtgtgg tataaattga tttgttttta gttttattta 660
ggtattgata ttaataggtg tttatgtttt tagaagtata tttgtttttt tttgtagttt 720
tagtgttttg ggatatttta ggggtagtta tgtagggttt tttgggaatt tgggtagagg 780
tgttatttat atagtttgtt ttgaattttt gggtttttta aaggaattta tgatgagtta 840
ttagggtgat tggaagtggt tggatagtgt ttttgggggg tttaaagaag taattatggg 900
gttttagaat ttttttgtat gttgttttga agaattttgt gtggattttt tttttaattt 960
atttgtttga aggaaataat aggggtgttt gtggttggga ttttgtaagg ttttgtgttt 1020
tatgtgaagg ttaggtttgt gtgggtgatg ttttttttgg gagaaagggg attttgtatt 1080
gttgtgttgt tgtttgtagt attattttgg aagtggtttt gtgtagtttt gttgtgtatt 1140
aagttttttg tgaatgttgg atttttgtgg tttatgtttt gtttttgttt tgtatttttt 1200
gatttttgtt tatatatggt gtggagtggg gggggggggg tttttgagaa ttttgttgtt 1260
tttgattggt tgtgtttttt tttattgtgg agtgtgaggg gttggggtgt tgtgttatta 1320
tggtaattgg tgaggtgtgt ttagattttg tttttttgag gttttggaga gtttagtaat 1380
tgtgtttgtt gttttttgtg ttgttttttg gtttttatgg ttttgttaga ggtattggtt 1440
gagttattgg gtgaggggta gtgggtgtgg ttattagtgg tttttgagtg atattaaggt 1500
tttgggtgtt ttttggaggt tttttttgtt gtgtaggtga ttttatggtt ttggtgtttg 1560
gtggaggttt tggtttaaat gagtttgttt gatttttgtt gttgtttgag tttgttttat 1620
gttttggatt t 1631

<210> 315
<211> 9254
<212> DNA
<213> Artificial Sequence

<220>
<223> chemically treated genomic DNA (Homo sapiens)

<400> 315

tgggggaaat tgattttatg atttaattat ttttatttga ttttattttt gatatgtgag 60
gattattata atttaaggtg agatttgagt ggggatataa ttaaattata tgagtggata 120
tgtaatatga gtgagaaata attttgtgat tgtaatttag taagattttt ggatggttaa 180
tatagtataa tttagagtat atggaggtgt gtatttttag ttttttgatt tttgggtatt 240
tagtaggatt gtattttttt gtatttttta agtaaggtat ggttatgtga ttaattttag 300
ttaataatat gtaaatataa taaaaagtgt tatttttagg agggagtttt taaagttaat 360
gtgagtttat tatgtttttt tttttttgtt aaattgatgt gatgattgtt gaagtatgta 420
ttaagatgaa atttttttta gattaggttt ttaaatgagt atattaagta gagttttgtg 480
aagttgaata tgtagtatga gtaaaatata aaatttattt ttaagttatt gaaattttag 540
gattgtttgt tatggtagta taatttaaat tattttgatt tatataaaaa ttggttttta 600
gatatgggtt agtgtaataa taataataag tataataata ataaaattaa ataattattt 660
agagggtgat aattaagaat attgttatta gaagttgaag ggatggtgat ttgtttggta 720
aatatattat ttgtgataat gtagaaggta gattatgaat ttgatgaatt aaagaaaagg 780
attgtaaaat agagagtgag tagtgtgtgt tggtggttgt tggatgtatt ttgaaagtta 840
ttataagtta tttaaagatg aatttagaaa aatttggttt gtttgtaatt aggaatgaaa 900
tggaatagag aaaatttata aatttaggga tttatattgt tagaagaggt aattgttttt 960

tagttttgat tggtaaaata taaaattaag aaatattttt agtaagaaag gatttggaag 1020
aaattaagtt ttgaagtaag gattaagtta taaagtatga atttattatt aaaattttag 1080
gataggaggt tgaggaggat ggattatttg aggttgggag tttgagataa gtttgattaa 1140
tatggagaaa ttttgttgtt tttaaaaata taaaattagt tgggtgtggt ggtgtatgtt 1200
tgtaatttta gttatttagg aggttgaggt aggagaattg tttgaatttg ggaggtagag 1260
gttgtagtga gttgagattg tgttattgta ttttaattag ggtaataaga gtgaaatttt 1320
gttttagaaa aaaaaaaaaa aggattaatt aagattttat ttagtatata tttttaattg 1380
gataaaatgg ttttgggaaa aggaagaaag gtttaatttt tttattaaaa tttaataagt 1440
ttaaagtgtt taaaattaag tgaagagtaa gagagtgaga tggtaaaaga gagatagagg 1500
agttaatagg taggagagta ggaaagtaaa gagatatggt aaattaaaga agtatgtttg 1560
gaaaagaatt ttgtggttat tgttatatgg aatattttat attagataga taagaagttt 1620
atttatatat atttaagatt taatgttatt ttgtattttt ttaattttta aaagtttatt 1680
taaaagtata ttttttaatg tttgttttag ttgtggaaaa ggaagatttt ttgagggtaa 1740
atttaggagt tatggaaaat aatgggtagg gtagtttggt gttgtgattg gatgggtttt 1800
taaggttgtt ttttttgaat aaagaatgag ggaattttat taggggaagg agagtagatt 1860
aaatatttgg tgaatagaaa gggagataga gtttttagtg ttatttttta aatatttttg 1920
gagagttttt tgagatattt ggtaggatta tagtgattta aagaggtatg gttatgtgat 1980
tgttttggtt aatgatgtat gggtagaagt gatgtgtgtt ataagtggat gttttgatag 2040
ttatgatgtt ttgagttttt tttttatagt taggatagtt gtgaaaaggg gtattaatat 2100
gaggtttttt ttagtttgag ttttttagtg attataatga gtagagttat tttgttaatt 2160
tataatggtt atgtagtaga aatgattaat ttttattgga ataagttatt gatatgttag 2220
ggttgtttgt tagggtagta taatattgtt ttttttgata gaattgggag tgagagataa 2280
ggttggagag agaaggagtt ggattgtgaa aagttttttt tttaaggtat aggaattgga 2340
atttttttga ttgtaaggaa aagttaatga aggtttttaa gtaggggtgt taatatgaat 2400
agatttgtta tttttttgga tgaaatatgt tattttttaa tataaggaga agattagttt 2460
attaaattat atgaggtaga gaggaatata tagaatttta ggtaaaagat atatatttat 2520
gtaaagtttt aagttttgaa atttttaaat ataagagttt tgaatggagt tatagttaga 2580
ggattaggta agaaaatttt ttttatgtta taagtgttat tttttttgta gataattaat 2640
attttataag aaagtttgtt agagtagaga aaatataaag gaatgtaata tagatttgag 2700
gttaattttt agttgtttgt gtgattttaa taaagatatt taattttttt gggtttattt 2760
ttttatatgt taaatttgga taataatata tgttttttaa gatagttgtg ggagaaaaat 2820
agtgtattta aattgtttag gagaatgttt ggtagataat aagtgtttaa tatatgaaat 2880
tgatttttat tattgttatt aaaaaatatt tagaatatta attttttgaa tataatagaa 2940
aatgatttag ggtaatagat agaggagaat gttttttttt tgaggaagta attggatttt 3000
gttattattg tatatttatt tattttattt tttttttagt ttagtgtttg gtttatagta 3060
ggtttttagt tattttttgt agattagtga aagttaggtg agtgtttgga gtgaagaaaa 3120
gttggtaggt tttttattga tattagttgt tgttggtttt tgaatattta aagttgtaaa 3180
tattttaggg ttgggggtaa tgaatttaag gttgaatttt aagtttagaa gtagtgaagt 3240
ttgaatttag aatttaggaa tttaaattgt tgtaggttta attttaagtt ttagttttag 3300
atagaggttt gggaaagatt tgatttttaa tttggttttt tttttttttt tttttttgat 3360
gttttttata ggaaagtata tttggttttg ttaaattgta tttttatatt ttggtatttt 3420
atttaggttt tgtgaggatg gaaattgtga ggtaggggag ggaagtgggt tgtttggtta 3480
ttattttttt agtgttgtag gtgattttgt tatattaatt tttggtagtt tagtgaggtg 3540
gatggtattg gttttatttg tagatgaggg aaatgaagtt tggaggagtt ttgtgatttg 3600
tttgttttat attgtggtag tttggttatg aaagaattag gatttttgat tttgggattt 3660
tttttattat atatttttgt ttttaagggg tggggggtgg ggggagaata aaataattgt 3720
ggaaaaggaa ttatttatat gtgtttagtg ttttttagag gttatttagg atatgtgttt 3780
attatttggt tgggagtgta gagatttgaa gtttaggttt tattttgggt tttgagtatt 3840
attgtgtgat tttatgtgag tgtttgttgt tttttgggtt tgtttttttg gaagtaattt 3900
ggtgtggatg gagtgtgtgt gtgtgtgtgt gtgtgtgtta tgttgtgtgt gttgtgttaa 3960
gtgtgtgtgt gttgtttggg ggtgggaggg ggagtagatt aatattgggg ttttttgagt 4020
tttggattgt ttatatgttt gtttttattt ggattttgtt atttattaat tgtgtttatt 4080
ataaaaattg tttttttgag gtaaagttgt gaatttttgt gttttttttt ttatggtttt 4140
ggaggatgaa gtggggtgta atggagattt agttgagtgt ttagttttgg gtaatataaa 4200
ttttttggtt tttatgagta gttagatgat tttgtggatt gttgtttttg aatttgattg 4260
agatgtaaat tttggagtgt ttttaatgtg gggggttgat ttttgggaga ttgtttttaa 4320
atttaagttt ttttaggttt gtttttattt gggattttat agagttattt taagggtggt 4380
atttttgttt ttttggaagt gtgggggggt ggtagtgtat ttggattttt agtttttagt 4440
tttgtgtggt ggtggttgtt ggtggatgat tttgggtttt ataagtgggg aaataataat 4500
tattttttgt ttgtattttt ggtttaaaat aattggttag gttttttggt tttttgggtt 4560
tttgtatttt ttgtattttt gtttgtagtt gtgaatttga gttttttttt attagaggtt 4620
gtgagtgttt gtttgtttgt ggtagttatt gttattagat agttaaattt taagatgtta 4680
gtttataatg tattgggtgg gttgggaaaa atggtttggg gaggggattg gggaagagag 4740

ggttgagagg tgtgtggtag gggggagggt aggagaaaga agggtttgat tgtaggaggg 4800
tagtggagta ttattttatt ttgtgagttt ttgtgggttt agagaagaat tttttagggt 4860
ggagttttta tggtgatggg tgggtttgtt tttttgagag tgatgtgagt taatgggaag 4920
gttttggggt gatattatgg gttattttta ggggttgatt ggtagtagat aagtgttgag 4980
tttgggttgg ataagggttt agagttgtat tgagtgtggt tgaagtagtg aggtgggagt 5040
ggaggtgtgt ggagttaggt agatagatag atatagttag ttagttaggt tggtagtata 5100
gtttgaattg taaattttat ttttttttta tttttttttt aattgtttag agttagtgtt 5160
tgtggttttt gggttggtgt tttgggagtg tttagagagt ttggttttta gttgtttttg 5220
ggaggagagt tttgttgttt aggtgttgtt gatagtggtg gaaagtagtg gtatttatgt 5280
gtttgttggg ggaagttggt gagtggttgt agtagtaaag aatttttttg gttgggagga 5340
ttggagataa gtggtagagt tttggagtta atttttgtaa gttttttttg tgttttaggt 5400
ttttttatgg tggtaaagat tagaaggtgg tggagagtta tgtaagagaa gaaggatgtg 5460
tgtttagttt tgtttgtatt ggttgttgaa tttgggtgag tgtgagttgt ggttgttggg 5520
tgtttttttt ttttagtagt ggaggagggg ataagttgtt ggagtttggg tggttaagat 5580
ttgttgttgg ttggttattg tagggtttgt attgatttgt tttgtgggga gagttgttgt 5640
tttgggaagt gagtttgttt gtggattttg aggaattgtt gtgtatgaag agtgtttagt 5700
gagtgattgt gattttttaa agttgggtag tgtgtgtgag ttgataagta agagtgtggg 5760
aggtatttta attaattttg tgttttttgg agtgagttgg tgaggagggt gtagtgggga 5820
tgatagttag tgggtgtgtg tgtttttaga gaaatttttt ttgttaaagg ttttgggggg 5880
tgtgggtgtt ttttgtttgt tatagttttg ttgtggtttt gaaatttgtg tgtgtagttt 5940
aaattaattt tatgtgaagt gatggattgt tttatgattg taaagatgga aatgattttt 6000
tatgatgatg tttttaatgt tttgtttttt ttgtttgaga gtggatttta tggttatagt 6060
aattttaaga ttttgaaata gagtatgatt ttgaatttgg ttgatttagt ggggagtttg 6120
aagttgtatt tttgtgttaa gaatttggat ttttttattt tgtttgatgt ggggttgttt 6180
aagttggtgt tgtttgagtt ggagtgtttg ataatttagt ttagtaatgg gtatattatt 6240
attatgttga tttttattta gtttttgtgt tttaagaatg tgatagatga gtaggagggt 6300
tttgttgagg gttttgtgtg tgttttggtt gaattgtata gttagaatat gttgtttagt 6360
gttatgttgg tggtgtagtt ggttaatggg gtaggtatgg tggtttttgt ggtagttttg 6420
gtggtagggg gtagtggtag tggtggtttt agtgttagtt tgtatagtga gttgttggtt 6480
tatgtaaatt ttagtaattt taatttaggt gtgttgagta gtggtggtgg ggtgtttttt 6540
tatggtgtgg ttggtttggt tttttttgtg taattttagt agtagtagta gttgttgtat 6600
tatttgtttt agtagatgtt tgtgtagtat ttgtggttgt aggttttgaa ggaggagttt 6660
tagatagtgt ttgagatgtt tggtgagata ttgtttttgt tttttattga tatggagttt 6720
taggagtgga ttaaggtgga gaggaagtgt atgaggaatt gtattgttgt ttttaagtgt 6780
tgaaaaagga agttggagag aattgtttgg ttggaggaaa aagtgaaaat tttgaaagtt 6840
tagaatttgg agttggtgtt tatggttaat atgtttaggg aataggtggt atagtttaaa 6900
tagaaagtta tgaattatgt taatagtggg tgttaattta tgttaatgta gtagttgtaa 6960
atattttgaa gagagattgt tgggggttga ggggtaatga agaaaaaaaa taatatagag 7020
agatagattt gagaatttga taagttgtga tggagagaaa aaagaagtgt ttgagaatta 7080
aagttaaggg tatttaagtt ggattgggtt gtgttttgat ggtgttttta gtgtgtatga 7140
gtgggaagga tttggtgtgt ttttttttgg tgtggagtta gggagtggtt gtttgtgggt 7200
tgttttgttt tgtggatggg ttgtttttgt gtgaatggaa tgttggattt tttgttaata 7260
ttgattaaga attgtatgga tttaatattt gattttattt agtattaaag gggggagggg 7320
gagggggtta taaattgtaa tagagattgt agattgtttt tgtagtattt tttaagaata 7380
taaagtgggg ggagggttgg ggaggggtgg taggagggag gtttgtgaga gtgaggttga 7440
gtttatagat gaattttttt tggtttgttt ttgttaattg tgtatgtata tatatatatt 7500
ttttaatttg atgaaagttg attattgtta ataaatagtt ttatgttttt gtaagttatt 7560
ttttgtttgt ttgtttgggt attttgttta gtgttgtttg taaataagag atttggagta 7620
ttttgagttt attatttgta ataaagtata taattttttt atgttttgtt tttgaaaatt 7680
ttagaaagga tatttaagaa aatataataa attattggaa agtatttttt taattttttt 7740
tttgtattat ttgtagatat tagttattta ggtggagttg aaagagttaa gaatgttgat 7800
taaaattatt tttagtgttt tttattatta agtagtaaaa attgtttttt attagatttt 7860
agaaataaat gtatttgatg tatttgatgt tatggttagg ttatattttt ttttttttag 7920
ttatttatat ggaattgttt attaaaggat agtgtgatgt tttaggaggt tggaggaagg 7980
ggggttgtag tggagaggga tagtttattg agaagttaaa tattttaaag tttggattgt 8040
attaagtggt atgtgttgtg attatttata atgttagtag aaattttata ataggtgttt 8100
atttttaaag taggaattgg tggtagattt tataaaagat gtattttttt aatttggaat 8160
tttttttttg ataattttta gataaaaaga tggtttttgt ttatgaatat ttataatagt 8220
atttttgtta taataaatgt atttaaatat taataataga ttttgaattg ttttttttta 8280
ttattttttt ttttttaagt tatatattga agtttttatt tttagttgtt gaggttaata 8340
ttgttgtaat ttgaagtgat ttgaaaatta agaagtaaag aatttaggaa gagttaaatt 8400
agaagttgtg atttgaatga aatagttttt taattttatt aatttttttt tttttttttt 8460
atttttattt ttttttttat tattttattt ttaaataaat ttttatgttt atggaaatta 8520

atagtttagt attttgggtt tgtttgtagt ttatttttta atttgttttt aaaataatta 8580
attttgttta aatatagaat aagttttaaa agatagtaat ttaatattat ggaaaagatt 8640
tataagagaa aatatgaatt agtattaatt taatgattat atgtgtaaat taaattttag 8700
ttttggggaa atgggaaatt tgaattttat ttatagatat aatttaggag attaataagg 8760
tagtgtttaa taagggaata attttttgtt tggttgtttt ttatattaag agttagtgtg 8820
attattaggt agtaaaagta atttgatatt taggaatatt ttataaatta ttaattgagt 8880
tagggttgtt tgtttattag tagtgtttta gtatttagta ggtttgggag tgttaatatt 8940
ttgtttgtgg ttgtaaattt aaatatataa agagtttttt tttaggagta tatttaggag 9000
tggaagagat tggataggtt tgttagtttt ggattttttg taataatatt ttttgaataa 9060
agggttattg ggagtagagt ttagtttgtt aaattgaatt tgttgatgtt agtaatttgg 9120
aaggtgagat gattttgatt atatgtagaa atagtagagg gataataaag tattttgggg 9180
tggggttttg tggaagttaa ataagagtta attagaaaaa ataagtaggt tggggaatta 9240
attatttgta aagg 9254

<210> 316
<211> 9254
<212> DNA
<213> Artificial Sequence

<220>
<223> chemically treated genomic DNA (Homo sapiens)

<400> 316

tttttgtagg taattaattt tttagtttgt ttattttttt tagttggttt ttgtttagtt 60
tttatagagt tttattttaa aatgttttat tgtttttttg ttatttttat atatggttaa 120
aattatttta ttttttgggt tgttgatatt ggtaaattta gtttggtaaa ttgggttttg 180
tttttagtaa ttttttgttt aagaagtgtt attataaagg atttgaagtt aataagtttg 240
tttaattttt tttattttta gatatatttt taaggagaaa ttttttgtgt atttggattt 300
atagttatag gtaggatatt agtattttta gatttattgg gtattagaat attgttaatg 360
agtaaatagt tttgatttaa ttagtggttt gtgaaatatt tttggatatt aaattgtttt 420
tgttgtttga taattatatt ggtttttggt atgaaaagta attaagtaaa aaattgtttt 480
tttgttaagt attgttttgt tgatttttta agttatattt gtaggtagaa tttaagtttt 540
ttattttttt agagttgagg tttgatttat gtatgtaatt attgagttag tattagttta 600
tgtttttttt tgtgaatttt ttttatagta ttagattatt attttttgag atttatttta 660
tatttaaata aggttgatta ttttagaaat aaattaagaa ataagttata gatagattta 720
aaatgttaaa ttgttagttt ttataggtat aggggtttat ttgggggtga ggtagtagag 780
agagagatag agatggagaa agagggaaag aattaatgaa attgggagat tgttttattt 840
agattataat ttttagtttg atttttttta aattttttgt tttttaattt ttaaattatt 900
ttagattgta gtaatgttaa ttttagtaat taaaaataaa aattttagta tataatttgg 960
gaagaaaaaa gtagtaaagg gaagtaattt aagatttgtt attggtattt gaatatattt 1020
attgtgataa gaatgttgtt ataaatattt ataagtaaag gttatttttt tatttaggaa 1080
ttgttaaaga gaagatttta aattggaagg atatattttt tgtaaaattt gttattaatt 1140
tttgttttga gaataagtat ttattgtaaa atttttatta atattataaa tggttatagt 1200
atatgttatt tgatataatt taaattttga aatgtttgat tttttagtgg gttgtttttt 1260
tttattgtaa tttttttttt ttttagtttt tgaaatattg tattattttt tggtaagtaa 1320
ttttatatag atagttgggg gaggaggagt ataatttgat tatagtatta ggtatattag 1380
gtatatttat ttttaaagtt taatagagaa tagtttttat tgtttaatag taagaagtat 1440
tgagagtgat tttaattgat atttttaatt tttttaattt tatttagata gttagtattt 1500
atagatgatg tagaaaagag gttaggggag tattttttaa tagtttattg tattttttta 1560
aatatttttt ttggaatttt tagaaataaa atataaaaaa attatatatt ttattataaa 1620
tggtaaattt agagtgtttt aaatttttta tttataaata atattgggta ggatatttaa 1680
ataaataaat aagaaataat ttataaaggt atgaagttgt ttattgatag taattagttt 1740
ttattaaatt aaaaaatata tatatgtata tatatagtta atgaaggtag gttagaaaga 1800
gtttatttgt aggtttagtt ttgtttttat aaattttttt tttgttgttt ttttttaatt 1860
ttttttttgt tttgtgtttt taaggagtat tatagaagta atttatagtt tttattgtag 1920
tttgtaattt tttttttttt ttttttttaa tattgaatga gattgaatgt taggtttatg 1980
tagtttttgg ttaatgttaa tgaaaagttt aatgttttgt ttgtgtgggg atagtttgtt 2040
tgtaaagtgg ggtagtttgt aggtggttgt tttttggttt tatgttaagg gagggtgtgt 2100
taagtttttt ttatttgtgt atattggggg tgttgttagg atgtaattta gtttaatttg 2160
gatattttttg gtttagtttt ttggatattt tttttttttt tttgttgtaa tttgttaagt 2220
ttttaagttt gtttttttgt gttatttttt ttttttgttg ttttttagtt tttgatggtt 2280
tttttttaaa atgtttgtaa ttgttgtgtt agtatgagtt ggtatttatt gttaatgtgg 2340

tttatgattt tttgtttaag ttgtgttatt tgttttttga gtatgttggt tgtggatgtt 2400
agttttgagt tttgagtttt taaggttttt attttttttt ttagttgggt gatttttttt 2460
agtttttttt tttggtattt ggaggtagtg atgtggtttt ttatgtgttt tttttttgtt 2520
ttgatttgtt tttgggattt tatgttgatg ggggataggg gtggtgtttt gttgggtatt 2580
ttgggtattg tttgaggttt tttttttagg gtttgtagtt gtgggtgttg tatgggtatt 2640
tgttggggta ggtggtgtgg tggttgttgt tgttgttggg gttgtgtggg aaaggttagg 2700
ttggttgtgt tgtaggaggg tgttttgttg ttgttgttta gtgtgtttgg gttgaagttg 2760
ttgaggtttg tgtagattgg tggtttgttg tgtaggttgg tgttgaagtt gttgttgttg 2820
ttgttttttg ttattgaggt tattgtggga gttattatgt ttgttttgtt gattggttgt 2880
gttgttgatg tgatgttggg tagtgtgttt tggttgtgta gtttggttag ggtgtgtatg 2940
aagtttttgg tgaagttttt ttgtttattt gttatgtttt tggggtatag gaattgggtg 3000
ggggttggtg tggtggtgat gtgtttgttg ttggattgga ttattaggtg ttttagtttg 3060
ggtgatgtta gtttgagtag ttttatgttg ggtgaggtga ggaggtttga gtttttggtg 3120
tggaggtgtg gttttaggtt ttttattggg ttggttaggt ttagggttat gttttgtttt 3180
aggattttgg ggttattgta gttataaggt ttgtttttgg atgggaggaa tgaggtgttg 3240
agggtattgt tatagaaggt tgtttttatt tttgtagtta tagaatagtt tgttatttta 3300
tgtgaggtta gtttgggttg tgtgtataag ttttggggtt gtaatagggt tgtggtaagt 3360
gggggatatt tgtgtttttt ggagtttttg atagggaaag tttttttaag agtgtatgta 3420
tttgttggtt gttgtttttg ttgtgttttt tttattagtt tgttttaggg agtgtagggt 3480
taattaagat gttttttgta tttttatttg ttgatttgtg tgtgttattt ggttttgaaa 3540
agttgtggtt atttattgag tgttttttgt gtgtagtggt tttttggagt ttgtaggtga 3600
atttattttt tagagtagtg gttttttttg tggagtggat tagtgtggat tttgtagtgg 3660
ttggttggtg gtgggttttg gttgtttgga ttttgatgat ttgttttttt ttttgttgtt 3720
agggggaggg ggtgtttggt agttgtggtt tgtgtttgtt taagtttaat aattggtgtg 3780
agtgaagttg agtgtatgtt tttttttttt tgtgtggttt tttgttgttt tttggttttt 3840
attgttgtgg agaagtttaa gatgtaggaa aggtttgtaa aagttggttt tgggattttg 3900
ttatttgttt ttggtttttt tagttgggaa agttttttgt tgttgtagtt gtttgttgat 3960
tttttttggt gggtgtgtgg gtattgttgt tttttgttgt tgttaatagt gtttgggtag 4020
tagggttttt tttttggggg tggttggaga ttaggttttt tggatatttt tgaaatatta 4080
gtttgggagt tataggtgtt agttttgggt agttagagag aaggtgaaaa gaaaataaga 4140
tttgtagttt ggattatatt gttgatttgg ttggttggtt gtgtttgttt gtttgtttga 4200
ttttgtgtat ttttattttt gttttgttgt tttagttata tttagtgtaa ttttgagttt 4260
ttatttagtt tgagtttaat atttatttgt tattagttaa tttttaaaaa tagtttatga 4320
tgttatttta aggttttttt attggtttgt gttgttttta ggggggtggg tttgtttgtt 4380
attatggaga ttttatttta gaagattttt ttttgggttt gtggaggttt atgggatgag 4440
gtaatgtttt gttgtttttt tatagttggg tttttttttt ttttattttt ttttttgttg 4500
tatgtttttt ggtttttttt tttttggttt tttttttggg ttgttttttt tggtttgttt 4560
ggtgtattgt gggttgatgt tttggggttt gattgtttag tgatggtggt tgttgtgagt 4620
gggtggatgt ttgtaatttt tgatggaggg gggtttaagt ttatggttgt ggatggggat 4680
gtgggggatg tagggatttg ggaggttagg gaatttggtt agttgttttg ggttaggggt 4740
gtgggtgagg ggtggttgtt gtttttttat ttgtggggtt tgaagttatt tattggtggt 4800
tgttattgtg tggggttgga ggttgagaat ttaagtatgt tgttattttt ttgtattttt 4860
gggggggtgg gaatattgtt tttaaggtgg ttttgtgaag ttttaggtgg gggtgagttt 4920
aaggggattt aagtttaggg gtggtttttt gagagttggt tttttatgtt gagaatattt 4980
tgaagtttgt attttggtta agtttaggag tgatggtttg tggggttgtt tggttgtttg 5040
tagaagttga gagatttgta ttgtttgaaa ttggatgttt agttgagttt ttgttgtatt 5100
ttattttatt ttttgggatt gtgggggaaa gggtgtgggg gtttatagtt ttgttttgga 5160
ggggtagttt ttatagtggg tgtaattggt gggtaatagg gtttagatgg gaataagtgt 5220
gtaggtgatt tgaaatttgg ggaattttgg tgttagttta tttttttttt tgtttttgga 5280
taatatgtat atgtttaata taatatgtat aatataatgt gtgtgtgtgt gtatatatat 5340
attttatttg tgttgagttg tttttgggaa aataagttta gaaggtggtg gatatttgta 5400
taaagttatg tagtagtatt tggagtttgg ggtagaattt ggattttaaa tttttgtatt 5460
tttggttggg tggtgggtgt atattttggg tagtttttag gaagtgttag atatatgtaa 5520
gtggtttttt ttttgtggtt attttatttt tttttgtttt tttatttttt agggatgaaa 5580
gtgtgtggtg gaaagaattt tgaagttggg aattttggtt tttttgtggt taggttatta 5640
tagtgtgaag taagtaaatt atggaatttt tttgagtttt atttttttta tttgtaggtg 5700
gggttggtgt tgtttatttt attgggttgt taggagttag tgtgataggg ttgtttgtag 5760
tattagggag gtggtggtta aatagtttgt tttttttttt tgttttgtag tttttatttt 5820
tgtagagttt ggataggatg ttaggatata agagtgtgat ttggtaggat taggtgtatt 5880
tttttgtgag aagtattgag gggaggaggg gagggggaat tgggttagaa gttagatttt 5940
ttttaagttt ttgtttaaaa ttggggtttg aagttggatt tgtagtagtt taagttttta 6000
ggttttaaat ttagattttg ttgtttttga atttggaatt tagttttggg tttattgttt 6060
ttagttttaa ggtatttgtg gttttgagtg tttagaaatt aatagtagtt ggtattagtg 6120

ggaaatttgt taattttttt ttattttggg tatttattta gtttttattg atttgtagag 6180
ggtaattgaa agtttattgt gagttaggta ttgagttggg gaggggtggg ggtaggtagg 6240
tatatagtga tgataagatt tagttgtttt tttaaggaga gaatattttt ttttgtttgt 6300
tgttttgaat tattttttgt tgtatttagg gagttggtgt tttaaatatt ttttaataat 6360
agtaataaaa attagtttta tgtattaaat atttattatt tgttaggtat ttttttaagt 6420
aatttaaata tgttgttttt tttttataat tgttttgaga gatatatatt attatttaga 6480
tttaatatgt gaggaaatag gtttagagag gttaagtatt tttgttgagg ttatataggt 6540
agttggaaat tgattttagg tttgtgttat atttttttgt atttttttta ttttggtaaa 6600
tttttttgtg gagtattagt tatttgtaga ggaaatagtg tttgtggtat agaagaaatt 6660
tttttgtttg attttttgat tgtaattttg tttagagttt ttgtatttag aaattttaag 6720
atttaggatt ttgtataaat atatattttt tgtttaaggt tttgtgtgtt tttttttgtt 6780
ttatgtggtt tagtaggttg attttttttt tatgttgaga aatagtatat tttatttaaa 6840
aaaataataa atttgtttat gttaatgttt ttgtttggaa gtttttattg gttttttttt 6900
ataattagga gagttttgat ttttatgttt tggagaaggg gttttttata atttaatttt 6960
tttttttttt agtttatttt tttattttta gttttgttag gaaaggtagt gttatgttgt 7020
tttaataaat aattttaata tattagtggt ttattttagt gaaagttggt tatttttgtt 7080
atatgattgt tgtaggttgg tagggtagtt ttgtttattg tagttattaa agggtttagg 7140
ttgaaggagg ttttgtgttg atattttttt ttatagttat tttgattgtg gggaaggaat 7200
ttggaatatt atggttgtta gagtatttat ttatggtata tattattttt gtttatatat 7260
tattggttaa agtagttata tggttatgtt tttttagatt attataattt tattaggtgt 7320
tttaaagggt tttttaggaa tatttgggga ataatattaa agattttgtt tttttttttg 7380
tttattaaat gtttggttta tttttttttt tttggtggaa tttttttatt ttttatttaa 7440
gggaaataat tttaaagatt tatttagtta tagtattaag ttgttttgtt tattgttttt 7500
tatgattttt ggatttgttt ttggagagtt tttttttttt atagttaaag taggtattgg 7560
aaaatatgtt tttaaataag tttttaaaag ttggggagat gtagagtgat attaagtttt 7620
gaatatgtgt aggtaggttt tttatttatt tgatatgagg tgttttatgt gataatagtt 7680
ataaagtttt tttttagata tatttttttg gtttgttata tttttttgtt tttttgtttt 7740
tttgtttgtt gatttttttg tttttttttt gttgttttat ttttttgttt tttatttgat 7800
tttgagtatt ttgggtttat tgggttttga tgggagagtt agattttttt tttttttttt 7860
agagttattt tgtttagttg aaagtatata ttgggtaaga ttttaattga tttttttttt 7920
tttttttttg agatggagtt ttatttttgt tgttttggtt ggagtgtaat ggtatagttt 7980
tggtttattg taatttttat tttttgggtt taagtgattt ttttgtttta gttttttgag 8040
tagttgggat tataggtatg tgttattatg tttggttaat tttgtatttt taggagtgat 8100
aggatttttt tatattggtt aggtttgttt tgaatttttg attttaggtg atttgttttt 8160
tttggttttt tattttgagg ttttaatagt aggtttatat tttgtgattt gatttttatt 8220
ttaaagttta gtttttttta ggtttttttt tgttaaaagt attttttgat tttatatttt 8280
attagttggg attgggaaat aattgttttt tttaatgatg taaatttttg gatttatagg 8340
ttttttttat tttattttat ttttgattgt aaataggtta aatttttttg agtttatttt 8400
tgaatagttt gtagtaattt ttagaatgta tttaatagtt attaatatat attatttatt 8460
ttttgtttta taattttttt ttttagttta ttaggtttat gatttgtttt ttatattatt 8520
ataggtgata tgtttattaa atagattatt atttttttag tttttgatag tagtgttttt 8580
agttattgtt ttttaaatga ttgtttagtt ttattgttgt tatgtttatt gttgttatta 8640
tattaattta tatttaggag ttaattttta tatgagttag gatggtttag gttatgttgt 8700
tataataaat aattttaaaa ttttagtggt ttaaaaataa attttatatt ttgtttatat 8760
tatatgttta atttatgggg gttttgttta atgtgtttat ttagaaattt agtttgaaga 8820
aggttttatt ttgatatatg ttttaataat tattgtatta gtttggtagg ggaaagaaag 8880
tatggtgaat ttatattggt tttaaaagtt tttttttagg aatgatattt tttattgtat 8940
ttatatatta ttggttaaaa ttagttatat ggttatgttt tatttaaagg gtgtaggaaa 9000
gtgtaatttt attaggtatt tggaagttaa agagttggaa atgtatattt ttatatattt 9060
taggttatgt tgtgttagtt atttaaaaat tttattgggt tataattata gaattgtttt 9120
ttatttatat tgtatgttta tttatatggt ttggttgtgt ttttatttaa attttatttt 9180
gaattgtaat aatttttata tgttaaaggt ggggttaggt ggagataatt gaattatggg 9240
gttagttttt ttta 9254

<210> 317
<211> 8244
<212> DNA
<213> Artificial Sequence

<220>
<223> chemically treated genomic DNA (Homo sapiens)

<400> 317

ttttaaatat atttattatt ttgatgggtt ataataattt ttttggatgt taatttttgg 60
ggatgttaaa atatattagt tttggtgggt ttttttagat ttatattatg tggtttgggg 120
ttttagatgt gttttttttt aggttttgga tagttttata tatttttttt aggttttata 180
gatttatagg ttattaattt gggtttttta attttaaaga tgaagttttt ggtttgtggt 240
ggttgtgttg attaatttgt ttggtttttt aggatgtgga tagtgttttt tttatattta 300
ggtattgttt ttaaaataag tggaattttt ttgtataatt ttgaatatta attttagggt 360
tttttaagtt tattgttttt tgtatatgtt tgttttattt tgtgtttttt tattttattt 420
ttgtgttttt ttttgtttat tttagtatgg gatatttgtt gttgggtttt gtaaatttat 480
ttatattaat ttgggttttt taaattttat atgttgaatt ttaagttttt tatgatattt 540
agtagggttg gaaggttgga atttttagag tatgaaaatt gttttttata ttttttttta 600
aatttggtta ttatatttag agtatgttga attttttttt gaattataaa tgatttgttt 660
tttgattgtt tgagttttat aaaatggagg gattttttta gtgatttttt aaagttattg 720
agaatgttgt gggtgtgtga gttataaagt tttggggtat taatgtttat ggttttatat 780
gttgtttggt tatgaattag gttttttaag atgtagatag tgttatttag gtttttggag 840
tatattgagt agttattagg aggtgtttaa gtgtgattta ggatttttta ggttttttta 900
aattatatag ggttttttgg gtttttttgg gttatattaa gtataaaagg atttttgggg 960
tagagtttat ttttattttt gttatgttag gtgttgttaa tggttttagt ttttaaatat 1020
tttgagtatt ttttttgtat tataatatat tgattattaa ataatttttt gggttttagg 1080
agttttataa taagaatttt gtttttttaa aatttaggta ttggtttgaa attttaatgg 1140
ttaggattat attggatttt ttttttggtt ttttatattt ggatgttttg gatgttgttt 1200
tttaggtttt ttaggaatat ttagtattgt ttttggatta taggatagta ttgatttttt 1260
gggttttaaa taatttattg agttatttta aagttaagta atattttttt taaatatatt 1320
gtatattttt tattataaaa tttgaaaatt tttaagtttt aagatttagg aattttgaat 1380
tttttttttt taaaatgtat atatggattt ttaagttttt taaggatttt gagtaatttt 1440
tttagatttt tagatttaaa aatgattttt ttggagtttt taaattgtgg tattgttttt 1500
tagtttttta aagtaaattg agattttttt ttttttataa aataattgag gttttttttt 1560
tttttaatat tgatttatga gttttttgat tttatggttt ttgttttggg tttttttata 1620
tttagaaaat gttttatgga tttttaaagt atattaaaaa atgtttttgg gttttaagaa 1680
attttaggta tggaaaaatt tgtgatttat aagtttttta gttattaatt aggtttttag 1740
aaatttagat attaaatttt tattgggtaa tttttatgtg ttttaaaaat ttgaaatgtg 1800
ttttattggg gtttttttaa atgtagatga tatttaggaa aatatatagt ttttttttta 1860
tttattaaaa ataagttaat ggaaattatt taaaaattag tatagaaaaa taatattgat 1920
tttatatttt tttatttttt attttgtttt ttttaaatgt attgattata ttttaggttt 1980
ttttaaaatt tagatagtgt ttttttttat ttttgaaggg tgttaaaatt tttttttgaa 2040
gttatagtaa ttatgaaggt tatttttttt ttggttgttg ttagtgttta ggttattaat 2100
ttatattttt aagatgtgaa aattaatttt agtttttttt taatatatta agaatgtgtt 2160
tggattttta aaatgtgttt tttgttttta tttgttaatt ttatgtaata tggttttatg 2220
gtaaaatttt taattttata tggagaattt tttttaatta ttttttttta taaattggtt 2280
ttttaagtta gttggttttt atttgagatt ttttttttta tgtttttaat tgtatggagt 2340
aatttttttt attttttaaa tttgtaattt atttttttgg agttttgagt ttagttatta 2400
attatggttt ttatattaat ggagtttttg gggttttttt ttttaggata tttatttgtt 2460
aagtttgtaa ggtagaaaga attttgtttt gtgtttttta aaatttgggt attgtttttg 2520
gtttgttggt tatttattgt agttttttta attttgtgta tagtgggtat tggttttggg 2580
tttttttgtt ttttatgaag tttttagagt aatttggatt tgggaaattt ttttttagtg 2640
ttgtttaaat atatttgggt tggttgtgtg tttttaggat gtggataggg agggtttttt 2700
tgtgtttagg aaagtgattg ggtattgttt ttagtttttt ttaaatttgg gtattgtttt 2760
tgggtttttt aatggattgg gtgttgtttt tggatattga ggattggttt tggggttttg 2820
tttattttta gtagtgttta ttgtttgtta tagagtgttt gattgtttgt tgtttgagtt 2880
tttggtgtgt ttgtggatgt agtttttagt tttgtggtga gttttttgtt gtgttgattt 2940
tttttgtttt tgtgtttttg attggttttt ggtttgtatt tgttgttgtt ttgttggtgt 3000
tggtgtttgt ttttggttgt tgttggggag gttggtgtgg ggtgtgggat atggttgtgg 3060
atttgtggtt ggtggttgtg tttgtttttg ttgggtgttt tgaaatttgt gttatttttg 3120
tttgtttatt gtaaagattt tatttgtggg gaaagtggtt ggagggtttt aaagtggggt 3180
gggtaggggg ttggggtgag ggatgtggag gagaggtgtt tttgttgggt ggtaaagtgt 3240
ttttagtttg tgggtttagg attttgttgg gaggtgtgtg tggagtgtgg gtgaagtgat 3300
tgatggtgga gtgagggggg tgaggggggt taggggggtg tgagattttg ttggtggttt 3360
tttttttttg gttaggttta ggtggtgggg ggttggtggg gtgtgggatt ttgtgtgtgg 3420
tttttgattt ggtaaaaatt atagtgtttt tttatattgt ttaaattttt taggagatgg 3480
tttttttaga tttttaaatt attgtggtgg tttttgagat tgaatttgtg tttatgtaag 3540
tttaatgtat tgaggatggg gtaattatta tttagatatt ttgggtgggt tgtaaaggtg 3600
agttatttag atgtattttg gtgagtttgg ttatgtaggt aggatttgag ttgtgttttt 3660
tgttttgatt tttttttttt tggtggttgg agtttttgta ggttttaagt ttggtttaga 3720

tttggtttgg tgtagttggt tttttgtgtg ttttgtattt ggtgggggtt ttggggtttt 3780
ggtgtggtat tggggggtgt ttgggatttg gttgaggttt taagtgttgt gtggttggtt 3840
ttttttgttg gggtaggtgg tggttgtgtg ttttgtttga gtttaggggt ttttttagtt 3900
gtaataatta gtaaggattt tttgatttag ttttaagtta tttgtatttg tatttagatg 3960
gggtgtattt gtagtgtagt tttttggtgg ggtgttggga gtttgtttgt ttttgtttgt 4020
ttggagattt tagtttatga gtataggttg tttgggtatt ttagaaattt gggatggggt 4080
ttttgaattt tttagaatgg gtatttagta tggttttggt gttttgtggt tttttgtttt 4140
ttatttagtt ttgtttttgt gtatttttta gtttttgtga ttgttgtttt tttttttggg 4200
gttttagggt ttttagtttg tattttttgt tttgtttttg gtttgggttg tgggggtggg 4260
ttgggggtgg ggtgagggtt ttgtgggtgt ttattggtgt gggtgtgagg ttagtgaggt 4320
ttgtgtgggt tttgggttgt gggttggtgt gattataaga gttgggtgtg ggtgtttgta 4380
gtttgtttgt tttttggtgg agttgtgtga ggtttggttg gttttggttt tttttttttg 4440
gttgtttttg ttttttggtt tatgtttgtt tgtgttttgt ttattagtgt ttttattggg 4500
taaggtggtt ttgtgttgat gttgtttgtt gttttgttgt tgatttttgt tttgggtgtt 4560
gtttgtgggg ttgtgttttg ttgggtttgt ggattttttg ttgttttttt tttatttatt 4620
ttaatttttt ttatttttgt tttgtttgag gaggtggttt tttttgtagg tagtttggtt 4680
tgtaggttgt tggtgttgtt attttttttg tgtttttttt ttagtttttt ttttggtgtg 4740
tagttttggg ttgttttttt ttttgtgttg tgttttggag tggttttggt gttgttattg 4800
tttgtttttt ttttgaggtt tgggtttgtg atggtagagg gttttgttgg tttaaattga 4860
gttgggtgtt tgtggtttgg gtgtagtttt tattttgttt tttagttatt gttttttttg 4920
gttttttgat gtggtgtttt tttttttgtt tttttgtgtt ttttgtgttt tttttggtgt 4980
ttggttttgg tttttgtttt tttttttgta attttttttt tgtttttttt tgtttttttt 5040
agtttttagt ttttgatttt tttttttttt tatgtttgtt tttttgtttt tgttgaatta 5100
aagtggatta attatatgtt ttttgttttt ttttgtgttg tttttttttg ttgtgtgttt 5160
gtttgttttt tgttgttttt tttttttttt gttttttttt tggttttttt tttttatgtt 5220
tattttgttt tttttattat ttttgttttt ttttattttt gatataatag ttgattttat 5280
tttttgatat tttttttttt ttgaaaagta taatatttgg tttgttttag tttgaagata 5340
gtttgttttt tttggataat tagatgaatt tttttttttt ttttaaaaaa aagttatttt 5400
tttgttttgt tttgttgtat atttggtttt tgtgatttgg ttagagtggt gttggtagag 5460
gagtgtttgg taggagggtt aatgtttgtt gtttggtttg tgatatgtag tagggaggtg 5520
ggtggtagtg ttgttggttt ttaggtaagt ggtgtgtgtg ggttgggttg gggttggggt 5580
tggggtggtg tgggtttgtg ttggatgttt ggtttttttt ttgtttgttt ttggtttggg 5640
gtttgtgggg tttggtgggg tggttgagtt tgggggggag gaggaggagg aggaggagga 5700
tggatggttg tgggttttgt tttttgtgtg gagttttgtg tttattttgg tggtggagtt 5760
gggggtgggg tgggggtgtt gggaagggtt gagggaggtg tgaggtgttt gtaggggtga 5820
ttttttggtt ggtttgtggg tgtagggggt gttgttttat atgtgtgatt tgtgttttgt 5880
gggttttggt ttttggggtg ttgggtaatg aggaggggtg tggagttgta gaagtttatt 5940
ttggtatgtt gatgtggtgt tagtgagatt gtgagaggaa gatgggggtg ggtggggtta 6000
ggatggagag gggttgagtt ggtaggagtt atggtagatg ttatatttgt gatatttttt 6060
ttatattttt tttggttttg tttgtaatat ttttaaatag gagttttggg agagggggag 6120
aggggttgtt ggtttgaggt taagaagggt agagtttttg atttggagag aggttgtggt 6180
ttttgtttag tgggtagtgt ggaagttttt atataaggag gtgggaagga gatttttttt 6240
ttttttattg ttttgtgtag agatgagttg ggggtgtagg atgggagttt atggtatttt 6300
gttatgggat ggtttagggt ttttggttgg gggtgtagga gagaagagat tggttgggag 6360
gagggagagg gtgggagtaa aggtgtgggg gagtggttag tagggagagg ggtggggggt 6420
agggtggagt ttgggttggg aggagttggt ttatatataa aagttgaggt attgattagt 6480
ttgtaaattg gatattagtt ttttttgtga aagagatttt tagttttttt tttttttttt 6540
tttttttttt ttttttgttt tagtgagttt tttgttgagt tgtaggtaat tagggttgtg 6600
gagtgaggat ttttgttgtt attttatttt agtttgaggt agggtagtag ggggtatggt 6660
ttatgtttgg gttttgggtt ttgtagttgt tagtttgttg ttttttggat agtatttttt 6720
tttttttttt ttttttgttt ttgtttttat ttggtttttg tttttttatt tgtttttttt 6780
ttttttgttt tttttttttg gttttttttt tgtttagttt agggtttttt ttgggttttt 6840
atttgttttg tattgttgta tgttttttgt tttgtttttt gttggttttt tgatttggat 6900
ttttaagttt agagtggtgg ggtttgttgt ggaagtgtgg tgagggttag agtggttagt 6960
tggtggagtg tgtttttaga atttggaagg gggtggtaga gggggtggtg agaggattgg 7020
ttagggttta gttaaggaga tgattaagga ggtttttaga tttttggtgt agttgtttat 7080
tagtttttag agagggtatg taaagttgtg tttttgtttt attgtttgtt tagttgttta 7140
tataatttat tgtattaaaa atttttttgg gtttgtggag tgaaggttgg ggttgtttgt 7200
ttggagggtt ttattttttg taggggtagg gttaatttgt tgtggtggtt tttggttttt 7260
tatttttgag tgggttaatt tggttttgtg gttttgtagt ttgtggaggg ggtgtgtttt 7320
aagattggtt ttttttttta gattgtagtt tggggaattt ggtgttggat tttttaggtg 7380
gtttgagttg gtttttttag tttttatggg gagagtttgg tagtgtaaat agggagatgt 7440
tttggaggtt tttggtttta ttggtttgat ttgaggtttg gaaaggaggt tttgggtgtg 7500

tgtgtgtgtg tttgggggta tttaaggtag attggagttg gagaattggg tgattgggaa 7560
aataaggttt ttagagtatg ggtggtgtgg ttgtgttaat tattggagtt tttgatttag 7620
gtttggttta gttgtagatt ggaaaggtgg aaaagttagg gggaggggtg gggttggttt 7680
agtaggattg gtttgttgtt ttgagggtga tggttttttt ggattttttt tgtttagttg 7740
ggggttgtgg ggaggaaggg attggttttt ttggatgtat atgttttgta ggggtggggt 7800
tgtttgttat tttggttggt gttggaggtt tgagaagtgg tgatgtgatg ttgggttggt 7860
tttgttttta tggtgttata ggatggaggt taggttgggt gtttagtttg ggtttttgta 7920
gttgtggatg ttgttgagtt tttgtaataa tgattgtgga gatggttatt ttttgtgtaa 7980
aattattagt gttttttgta aatggaagga attgggtttt ttttgtgtgt ttttggatgt 8040
tttattttgt atatggtttt gtgtttttat tttggtatta tgatttgtgt gttatttttg 8100
taataaaaat aatgtttata ggaaagttgt gtttttaatt tttaattgaa tttgtaggtt 8160
ggtaaatttg gtttgggagg ggtatttttg gtttggggtt tggtttggtt gttttgttta 8220
tgttattttt tttttgtttt taga 8244

<210> 318
<211> 8244
<212> DNA
<213> Artificial Sequence

<220>
<223> chemically treated genomic DNA (Homo sapiens)

<400> 318

tttgggggtg ggagagaagt ggtgtgagtg gggtagttag gttaagtttt aggttagagg 60
tgtttttttt aaattaaatt tgttaattta taaatttagt tgaaaattga aagtatggtt 120
tttttataaa tattattttt attataaaag taatatatag gttataatgt tgaggtgagg 180
gtgtagggtt atgtgtagaa tgaagtgttt agaagtatat agaaaaggtt tagttttttt 240
tatttgtaag aagtattagt aattttatat gaggggtgat tatttttatg gttattattg 300
taggagttta gtggtattta tagttgtagg ggtttaggtt ggatattgga tttggttttt 360
gttttatgat attatggggg tagggttagt ttagtgttat attgttattt tttaggtttt 420
tagtgttagt taagatggta gatagtttta tttttataga gtatgtgtat ttaggaggat 480
tagttttttt ttttttataa tttttagttg agtagggggg gtttaggagg attattgttt 540
ttaaagtagt aggttagttt tgttgggtta gttttgtttt tttttttggt ttttttattt 600
ttttagtttg tagttgagtt aggtttgggt taaggatttt aatggttaat ataattatgt 660
tatttatgtt ttagaaattt tgttttttta gttatttagt tttttaattt tagtttgttt 720
tgggtatttt taaatatata tatatatgtt tagagttttt tttttaggtt ttaaattgaa 780
ttagtaaggt taggggtttt tagaatattt ttttatttgt gttattaaat tttttttgtg 840
ggagttggag agattagttt aggttatttg ggaagtttga tattaggttt tttagattat 900
aatttggaag gggaggttag ttttaggata tatttttttt ataggttgta gggttatagg 960
gttgggttaa tttatttggg ggtgggaggt tgggagttat tatagtaagt tggttttgtt 1020
tttgtagaag gtggaatttt ttaggtgggt agttttagtt tttgttttgt agatttaggg 1080
gagtttttga tgtaataaat tatgtgagtg attgagtagg tagtgggata gaagtataat 1140
tttgtatgtt ttttttaaag attagtgggt agttgtgttg aggatttgaa agtttttttg 1200
gttatttttt tgattggatt ttggttagtt tttttattgt tttttttgtt attttttttt 1260
aaattttaag agtatatttt gttagttggt tattttagtt tttgttgtgt ttttgtaata 1320
agttttatta ttttgaattt ggaggtttgg gttaggggat tggtagaaag taggatagga 1380
agtatgtagt ggtgtggagt aggtgagggt ttaggaaaag ttttgagttg ggtaaggagg 1440
gggttgaagg gaaggaatag aaagggaagg agtaggtgag ggagtagaga ttaggtgggg 1500
gtaggggtag aggaaaagag gggagggggg tgttgtttga gaggtagtgg gttggtggtt 1560
gtagggtttg aggtttaggt gtgggttgtg ttttttgttg ttttgtttta ggttggagtg 1620
ggatggtagt gggggttttt attttatagt tttggttatt tatagtttag tagaaggttt 1680
gttggggtag gaggaggagg aggaagagga ggaggaggaa gttggaagtt ttttttatag 1740
gagaagttaa tgtttagttt gtaggttggt tagtgtttta gtttttatgt gtgagttgat 1800
tttttttagt ttgggtttta ttttattttt tatttttttt tttgttgatt atttttttgt 1860
gtttttgttt ttgttttttt ttttttttta gttagttttt ttttttttgt atttttaatt 1920
gggagttttg gattattttg tagtgaagtg ttatgggttt ttattttgta tttttggttt 1980
atttttgtat agggtagtga aggggggggg ggtttttttt ttattttttt gtatggaaat 2040
ttttatgttg tttattgggt aggggttgtg gttttttttt gggttgaagg ttttgttttt 2100
tttagtttta gattagtagt tttttttttt tttttttggg atttttgttt ggaaatgttg 2160
tggatagagt tagagggggt gtgggggaga tgttgtgaat gtggtgtttg ttatgatttt 2220
tgttaatttg gttttttttt attttggttt tgtttatttt tgtttttttt ttgtggtttt 2280
gttggtattg tgttaatata ttagggtggg tttttgtggt tttgtgtttt tttttgttat 2340

ttagtatttt ggaggttagg gtttgtaagg tatgaattat atatgtgagg tggtattttt 2400
tgtatttata gattgattgg gaagttgttt ttgtagatat tttatatttt ttttggtttt 2460
ttttgatgtt tttattttat ttttagtttt gttgttaggg tgagtgtggg gttttgtgta 2520
gggaatggga tttgtagttg tttgtttttt tttttttttt tttttttttt tttgggttta 2580
gttgttttgt tgagttttgt aggttttggg ttgggagtgg gtggaggaag ggttgggtgt 2640
ttggtgtaag tttgtgttgt tttagttttg gttttggttt ggtttgtata tgttgtttat 2700
ttggaagttg gtgatgttgt tgtttatttt tttgttgtgt gttgtaaatt gaatagtggg 2760
tgttggtttt tttgttggat atttttttgt tagtgttgtt ttggttgagt tgtgggggtt 2820
gaatgtgtga tggggtagag tggggggatg gttttttttt gggggggggg ggagaatttg 2880
tttgattgtt tagggaggat gggttgtttt tgggttgggg tgggttagat gttgtatttt 2940
ttggggggga aaaggtattg ggaaatgagg ttagttgttg tattaaggat agaggggggt 3000
agagatagtg ggagagatag agtgaatgtg aaaggggggg gttgaagaga gggtgagggg 3060
gagagaggat agtgagaggt gggtaggtgt atagtgggag agaatagtat ggagagaaat 3120
agaaagtgtg taattaattt attttggttt ggtgaaggtg agagggtggg tgtgaggggg 3180
ggagggagtt ggaggttagg agttgggggg gatgggaggg agtgaaggga aggttgtggg 3240
agaaagagtg ggggttgggg ttagatgtta agaggggtgt ggggagtata gagaagtgga 3300
gggaagggtg ttatgttgag gggttggggg aggtggtgat tggggggtgg agtggaggtt 3360
gtatttggat tgtgggtgtt tagtttggtt tgggttgatg gagttttttg ttgttgtgag 3420
tttgggtttt gggaggggga taggtggtgg tggtattggg gttgttttgg gatgtagtgt 3480
ggaaagggga gtggtttgag gttgtgtgtt ggggggaggg ttggaggggg agtgtggggg 3540
gggtgataat gttggtggtt tgtgagttgg attgtttgtg gggggaattg tttttttggg 3600
tgaagtgggg atggggggag ttgaggtaga tgaagaggag gtggtgggga atttgtaggt 3660
ttggtggagt gtgattttgt ggatggtgtt tgggatggga gttagtagtg aggtagtggg 3720
tggtgttgat gtggggttgt tttgtttgat ggaggtgttg gtgggtagag tgtgggtagg 3780
tgtgggttag gaggtggggg tggttggaag gggggggttg gggttggttg ggttttatgt 3840
agttttgttg gagagtaggt gaattgtggg tgtttatgtt tggtttttat agttgtgtta 3900
gtttgtggtt tagggtttgt gtgggttttg ttggttttgt gtttgtgtta atgggtgttt 3960
gtggagtttt tgttttgttt ttggtttgtt tttgtaattt gagttaagag tggggtgggg 4020
ggtgtgggtt gggggttttg gggttttggg ggggggggtg gtggttgtag gggttggggg 4080
gtgtgtgggg gtgaggttgg gtggggggta gggagttgta gagtgttaag gttatgttga 4140
atgtttgttt tagagaattt aggggtttta ttttgggttt ttggggtgtt tgggtggttt 4200
gtgtttgtga gttggggttt ttgggtaggt aggggtaggt gggtttttag tgttttatta 4260
ggaggttgta ttgtgggtgt gttttgtttg agtgtagatg taggtggttt ggggttgagt 4320
tggggggttt ttgttggttg ttgtggttga gggggttttt gggtttgggt ggggtgtgta 4380
gttgttattt gttttagtag aaggagttgg ttatgtggtg tttggagttt tagttagatt 4440
ttgagtgttt tttggtgttg tgttggagtt ttggagtttt tgttaggtgt gggatgtgtg 4500
gaaggttggt tgtgttgggt tgaatttggg ttaggtttgg agtttatgga ggttttggtt 4560
gttagaggag agaggattag ggtgggaaat atagtttaaa ttttatttgt atggttgagt 4620
ttattggggt gtgtttaagt agtttgtttt tgtggtttat ttaaaatatt tggataatgg 4680
ttattttgtt tttagtgtgt tggatttgta tagatgtgag tttggttttg ggggttatta 4740
tgataatttg ggggtttggg gaaattattt tttggagagt ttgaatgatg taagaaagta 4800
ttgtgatttt tattaagtta aaaattatgt ataaaatttt gtattttgtt agttttttgt 4860
tgtttaagtt tagttaaggg gaaggggttg ttggtggaat tttgtgtttt tttggttttt 4920
tttgtttttt ttgttttgtt attaattatt ttgtttgtgt tttgtgtgtg ttttttggtg 4980
gagttttagg tttgtgggtt agaggtattt tattgtttgg tgggagtgtt ttttttttgt 5040
gttttttgtt ttagtttttt gtttgtttta ttttgggatt ttttagttgt tttttttatg 5100
aatgagattt ttgtaatgag taaatgaaag tggtgtggat tttggggtgt ttagtaggag 5160
tgagtgtagt tgttagttgt aagtttgtag ttgtgttttg tgttttatgt tggttttttt 5220
ggtggtagtt ggagatgagt gttagtattg gtgggatagt agtagatgtg ggttgagagt 5280
tggttagggg tgtagaggtg gaggggattg gtgtggtggg gagtttattg tgaagttgga 5340
ggttgtgttt gtgggtgtat taggagttta ggtagtgagt gattgaatgt tttgtggtag 5400
gtggtggatg ttgttgaagg tgagtgagat tttggggtta gtttttagtg tttgggagta 5460
atgtttagtt tgttggaaga tttggggata atgtttaaat ttgggggaga ttgggggtaa 5520
tgtttggttg tttttttgga tatggggaag ttttttttgt ttatgttttg agggtgtgtg 5580
gttgatttaa gtgtgtttgg gtaatgttag agagaaattt tttaaatttg agttgttttg 5640
gggattttgt aggagataga gaggtttgaa attagtgttt gttgtgtgtg gggttgggga 5700
agttgtagtg ggtggttggt gagttgggaa taatgtttaa attttgggga atgtaaggta 5760
gagttttttt tgttttgtgg gtttagtgaa tgggtgtttt ggaggagggg atttggggga 5820
ttttgttaat gtgggaattg tgattaatga ttaaatttga gattttagaa aaatagatta 5880
taggtttggg ggatgagaga agttgtttta tgtaattggg aatatagaga aagaggtttt 5940
aaataggggt tagttagttt gggggattaa tttgtgagga ggggtagtta aagaaaattt 6000
tttatatgaa attgggaatt ttgttgtaga gttatattat gtaaaattgg tagatgaaag 6060
taaggaatat attttgggga tttaaatata tttttggtgt gttaggggga agttgagatt 6120

aatttttata ttttaagaat ataagttagt ggtttggatg ttggtagtag ttggggaaaa 6180
aataattttt ataattattg tggttttagg gaaaggtttt aatatttttt agagatggaa 6240
gaaagtattg tttagatttt gggggagttt ggagtgtgat tagtatattt ggggaaagta 6300
aaataaaaaa taaaaaaata taaaattagt attatttttt tatgttaatt tttaaatgat 6360
ttttattagt ttatttttgg taaataagaa aaagattata tatttttttg gatgttgttt 6420
gtatttgggg gagttttagt gaaatatatt ttaagttttt gggatatatg gaaattattt 6480
aatggagatt tgatatttaa atttttggaa atttagttag tagttaggaa atttataggt 6540
tgtaggtttt tttatgtttg ggattttttg ggatttaggg atatttttta gtgtgttttg 6600
ggggtttatg gaatattttt taaatgtagg gggatttagg gtagggatta taaagttagg 6660
agatttataa attagtatta aaaaaaaaaa aaattttaat tgttttgtga agggaaaaaa 6720
attttaattt gttttggaag gttgggagat aatattgtaa tttgggggtt ttaggaaaat 6780
tgtttttgaa tttaaagatt tagggaagtt gtttagagtt tttggaggat ttgggggttt 6840
atatgtatat tttaaagaaa ggggatttag aatttttagg ttttaggatt taggaatttt 6900
tagattttgt gatagggagt gtgtagtgtg tttgaaggaa atattgttta attttgagat 6960
gatttagtgg gttgtttgga gtttaagaaa ttagtgttgt tttgtgattt gagggtaatg 7020
ttgaatgttt ttagagggtt tggagggtag tgtttagaat atttaagtat gggggattag 7080
gaaaagggtt tagtgtgatt ttggttgttg gggttttaga ttaatgtttg aattttagaa 7140
aagtaagatt tttgttatga agtttttggg atttagagaa ttgtttaata gttaatatgt 7200
tgtgatgtag agaaagtgtt tggaatgttt gggaattggg gttgttagta atatttggta 7260
taatagaaat aaaagtaggt tttgtttaag gggttttttt gtgtttggtg tagtttagag 7320
ggatttggga gattttgtgt gatttggggg gatttggaga attttgggtt atatttgagt 7380
attttttggt aattgtttag tgtgttttag ggatttgggt ggtattgttt atattttaag 7440
ggatttgatt tgtggttagg tagtatatag agttatagat gttaatgttt taaagttttg 7500
tgatttatat atttataata tttttaatag ttttggggaa ttattgggga aattttttta 7560
ttttatgaaa tttagataat tagagggtag gttgtttatg atttagaaaa ggatttaata 7620
tgttttgggt gtaatgattg aatttggggg aaggtatggg aagtaatttt tatattttga 7680
gggttttaat tttttagttt tgttgagtgt tatgagggat ttgggattta atgtgtaaag 7740
tttagggaat ttaggttagt gtgaatagat ttgtgggatt taatagtaaa tgttttatgt 7800
tgaggtgaat gggaaggggt gtgggggtgg ggtggggggg tgtggaatgg ggtggatgtg 7860
tgtgggaaat agtaagttta ggagattttg gagttaatat ttaaaattat gtgggaaagt 7920
tttatttgtt ttggaaataa tgtttaaatg tggaaaaggt attgtttata ttttggggag 7980
ttaggtaaat tggttagtgt gattattatg gattaagggt tttgttttta aaattaggga 8040
atttgggtta gtggtttgta aatttgtggg atttggaggg ggtgtataaa attgtttaga 8100
gtttgggaga aagtatattt ggggttttag gttatatagt gtaagtttgg aaaagtttgt 8160
tgggattgat gtgttttgat gtttttggaa attaatgttt aagaaaatta ttgtgatttg 8220
ttgaaatggt gaatatattt agaa 8244

<210> 319
<211> 16842
<212> DNA
<213> Artificial Sequence

<220>
<223> chemically treated genomic DNA (Homo sapiens)

<400> 319

ttttttaaag tgttgggatt ataggtgtga gttattgtat ttggtttgtt atttatgtat 60
ttattttttt ttgagataag tttttgttat gttgtttagg ttggttttaa atttttaggt 120
ttaagagatt tttttaaagt tttgggatta taggtgtgag ttattgtgta taattttgaa 180
gtggttaatt ttaataatat atttatttta gtttaatata tttaaaatta tattatttta 240
atatgtaatt attattaaaa ttattaatga gatattttat tttattttta tttatttatt 300
tttttgagat ggagttttgt tttgtttttt aggttggagt gtggtggtgt gattttggtt 360
tattgtaagt tttgtttttt gggtttatgt tttttttttg ttttagtttt ttgagtagtt 420
gggattatag gtgtttgtta ttatgtttag ttaatttttg tatttttagt agagattagg 480
ttttattatg ttggttagga tggttttgaa tttttgattt tgtgattagt ttattttggt 540
tttttaaagt gttgggatta taggtgtgag ttattgtgtt tggttagtga gatattttat 600
ttgtgttttt tttttttttt tttgagattg agttttattg tgttgtttag gttgtagtgt 660
agtggtataa ttttggttta ttgtaatttt tgttttttag gtttaagtga tttttttatt 720
ttagtttttt gagtagttgg gattataggt gggtgttatg atatttggtt agtttttgta 780
tttttagtag agatgggggt tttattatgt tgtttaggtt ggttttgaat tttgattttt 840
aggtgattat ttgttttggt tttttaaagt gttgggatta taggtaaaag ttgttatatt 900
aggtttattt tttttttttt tgagatggag ttttattgtt gtttaaggtg gagtgtaatg 960

gtaagatttt agttttttgt aatttttgtt ttttgggttt aagtgatttt tttgttttag 1020
ttttttaagt agttgagatt atgggtgttt gttattatgt ttggttaatt tttgtatttt 1080
tagtagagat agggttttat tatgttggtt agattggttt taaatttttg attttgggtg 1140
atttatttgt tttggttttt taaagtgttg ggattatagg tgtgaattat tgtgtttggt 1200
aggtttattt ttaatttttt taattttgga aatttagtgt gtagggtagg tatggtgttt 1260
tatgtttgta attttagaat tttgggaggt tgaggtaggt ggattatttg aggttaggag 1320
tttgggatta gtttgaataa tatggtgaaa ttttgttttt attgaaaatt taaaaattat 1380
ttagttatgg tggtgtatgt ttgtagtttt agttatttta gaggttgaga taggagaatt 1440
gtttgaattt gggaagtaga ggttgtagtg aattaagatt atgttattgt atttagtttt 1500
gggtaataga gtgagatttt gttttaaaaa aaaaaaaaga gagttagtgt ggagggaagt 1560
gtttttgttg ggtttgggtg ttttgtggga ttttgaggaa aagtttgtat taggtagatg 1620
ttgttttgtt tttatgggta aaggatattt taataaattg aggagtaaaa tgtttttgta 1680
tgtttttttt gtgtagattt gttaggaaga gtataagaag aaatatttgg atttttttgt 1740
taattttgtg gaatttttta agaagtgttt ggagagatgg aagattatgt ttgtaaagga 1800
gaagtgaagt ttgaagagaa ggtaaaaagt gataaagttt gttgtgatag ggagattaaa 1860
aattatattt ttttgaaatg taagaaaggg taagaaagga aagaaaaagg attgtaatgt 1920
ttttagaagg ttattatttg tttttttttt gttttgtttt gaatattgtt taaagattaa 1980
aagtggatat ttaggtttat ttgttgtgga aattgtaaag aaattgggtg aaatgtggtt 2040
tgggtagtta gttaaagata aataattata tgagtagaaa gtagttaagt tataggagag 2100
atatgaaaag ggtattgttg tatattgtgt taagggtaaa agtgaagtag gaaagaaggg 2160
tttaaagaag aataaattag aagatgagga ggaggaggag gagaaagaag atgaagatga 2220
ggaggaagag ggtgaagatg aagaataaat ggttattttt taatgatgtt tgtgtagtgg 2280
gtttgttttg ttaagaatgt gaattttagt atagtttagt attagtttta gtataaaatt 2340
gtataaattt ttgtatagtt tataagattt tttgtataga aaatattttt tttttttttt 2400
tttttttttg agatagagtt ttgtttttgt tgtttaggtt ggagtgtaat ggtgtgattt 2460
tggtttattg taatttttgt tttttgggtt ttggtttaag tagttttttt gttttagttt 2520
tttgagtagt tgggattata ggtatatgtt attatgttta gttaattttt gtatttttag 2580
tagagatggg gttttattat gttggttagg ttgtttttaa atttttgatt ttgtgatttg 2640
tttgttttgg ttttttaaaa tattgggatt ataggtgtga gttattgtat tttgtttaat 2700
gtttttaaat atttaatggt ttttaaaaaa tttattgtgt atggtagtat agtatatttg 2760
taggaattag tattaatagt atattttgtg ttttttaaga tgttgtattt tttaatattt 2820
tgtaataaaa ttatgtgtat taaaaaaata aagaaatttt gtgtgtagtt tatatttata 2880
gtatattttt gtttaggtat ttgagagaat gattaggagg ggtttttgga ggaggtggtt 2940
tttgaatgga gaatttattt ttaaggattt tgtttgtaat ggttattaag tattttttga 3000
gttattttta tgtgttttgt agttttttga aggggtgtgg gatttattga tgttaattat 3060
ttagtattat ttttagattt taagaagttg gggtgtgagt tagtaattag tatagaaaag 3120
agatattaaa ataagtttga gttggggagt gtttttttaa ttttagtttt ttggaagaga 3180
tttttttttt ttttttgaga tagagttttg tttttattgt ttaagttgga gtgtagtggt 3240
atgattttgg tttattgtaa tttttttttt ttgggtttaa gtgatttttt ttttttagtt 3300
ttttgagtag ttgggattat agatatgtat ttgtaatttt tattaaaaat ataaaaatta 3360
gttgggtgtg gtggtgtatg tttgtaattt tagttattgg ggaggttgag gtaggagaat 3420
tgtttgaaat taggaggtgg agattgtatt aagatagttt gttttagtta aataatttgg 3480
tgttagtgta ggaaaaggtg gaaggtatgg ggttagtata ggagggttta atatttttaa 3540
ttttattaag ttatattttg gtaatttttg ttttttatga gaagtttttg ttgggtttgt 3600
tttagtgttg ttttgaggtt ttttttatga gttttgatag ggtagaggtt gttttgagtg 3660
tttttttttt ttttggttta agagtggttt aaaagaagga tttttgattg gaattggtta 3720
ttttgtgtta ttttttgatt tttgattttg ttttaaaggg ggatgtgggg gaggggtttt 3780
ggtaggggtg gttttgtttt ttttaggttt gtaagtttag gtttttgttt attgggttta 3840
gtttattttg tggttgttta gggaggttgt tggtatttgt gatttatgat tttttttttg 3900
agttttattg aggttatagt tgtggttttg tttttttatg tttgtttttt gttttttgtt 3960
tgtgatgggt gtttttgagg attaatgagt gtgttgtatt tattttttgg gtggggttaa 4020
gtgttgatta attgttgttt gggtgtttgg ttgggtttaa atgttttaat tgttagtggt 4080
ggtggggtgg gtagagggtt ggggatggta ggtttaatta atgggtgggt atgttgtttt 4140
tgtgaggagg tgtgttttgt ggttgggtgt gtggtgtttg tggtggtgta gggaggggga 4200
gggaggtaaa taagatggtg gtggtgtgtt gggtgtggaa gggggaggtg gtttggggtg 4260
tttgtgagtg aggtgtgggg tggtgaaggg agtgtgggtg gtggtatttg ttgttgtggt 4320
tttggatggg ttgggttttt tttgttgttt tgtttttttt atatgtgtgg tggttgtggt 4380
gagggggatg tgttgtttgg ggtttggtat ttttgggaat tttttggttt ggagtttgtg 4440
gtttgtgttg ttttggttgt tgggagtttt gtggagtttt tgttgttgtg ttgttttgtg 4500
gattggatgt tgagggtatt tggggtgggg tgtgtgtttg ggtagatgtt tgtggggagg 4560
ggggtgtttg ttgggttttg gtgattattt tgggggttgt gggttggttt ggggggtgtt 4620
tagtgtgggt ttttgtgggt gttgggtagt gattagtttt gagtggagtt gttggttgtg 4680
gtgggaggtt ttttggatgt ttttagtttt ttgaatgttt gtttgggttg gtgggagttg 4740

gtgttttttg ggaggtttgt ttggttgttt gtggtggagt gtttgttttt gggataggtg 4800
gtgggattgg ggtgttgttg gagatgtttt tagtgaagtt gggtttttta ggtgtggggg 4860
ttttgggggg tagtgatgtt gtggatttgg tttgtgggat gggtggtttg gagaagattg 4920
tgtttggttg tgtttatatt tgtttgtggg tttgaggttt ttggaggatg atttagtatt 4980
gaaaagtttt ggttggtttt tttagggttt ttgaggatga agttgatttt gattgggttg 5040
ttttttagtt ttgaggtttg ggttttattg gaatttgtgt ttgagttgtt gttttggatt 5100
tttggtgttt gttggtttgt agatttgtta ttgggtttgg atttgtagtt gggattgatg 5160
tgtagaataa ttgtttttgt tggaagaagg gttttttttt ttttttgggg gtttttgttg 5220
tttttttttt tttttttttt tttgtaaaat tttggagaag ggaagttgga atataaggaa 5280
ggattgttta tttgtggatt tagggttggt ggtgggattt taggattttg ggtttagtat 5340
ggaggtggtg gatttgtagt agttgggtat gtttatggag ggtgagttga tgttggtggg 5400
tatggatatg tttatttatt gtattgattt tattgaggtt atttattagt tgtgttgtaa 5460
gtgggttaag tttattggta agtatttgat gggggatttg ttgggggaag gtttttatgg 5520
taaggtgaag gaggtgttgg atttggagat gttgtgtagg agggttgtta agatttttaa 5580
gaagaagaag ttgtgaagga tttttaatgg ggaggttaat gtgaagaagt aagtatggtt 5640
tgttggggtt ggggttgggt tgggttagtt atggtgttga tggttttgtt tttttttttt 5700
tttttttttt tttttttttt attttttttt aatatttgga gttggatttg tttggtgttt 5760
gtgttttttg tgttagggag agtgtggttg ggggtttgtg ttatggattt ttatttaggt 5820
aaggttagtt gttgtagtgg ggtgtgtgtt tgtatgggtt tttggatttt agttaaaatg 5880
ttttggtagt gaaatttttt tgagaaggga gtggttttta attttttaag attagttttt 5940
tggttttttt agttgtttaa ggagtagagg tgtttagtgg aattagtttg tgtttgtttg 6000
ggttttgaga gtttgtgtgt ggttgttaat atgttttttg tgtagtgtgt ggttgttatt 6060
ggggttaggt gaaatgtgat ttgtttattt tgttagaggg gaattttggg ttgttaaaaa 6120
taattgtttg tattggttta ttagttagta ggagggaaat gtagtttttt tttatttgtt 6180
agggatgtga tgttggaagt attttggttt tttgggggttg gaagttttgt ttgagggggga 6240
ttgtgttttt tttttgaatt gtatttggaa gattttattt ttttaattga tttttttagg 6300
tatggggttg ttgttgggta ttttggatgt ttttgtattt gtgtgtggag aagtttttat 6360
ttagggtagt attggttggt ttgagttttt tttagttggt gggggtggtt gggggtgttt 6420
ttgttttatt gtagttagat atgttgtatt tgttgttgtt tggtgggttt tgtttaggtt 6480
ttgttttttt tttagttttt ttaattttta aaattttgtt ttttgttttt aagaaaggat 6540
tttatttgtt gggttaggtt ttgagggatt ggtaaggata ttttgtgtag ggtattatga 6600
ttttggaagg aggtttgggg aggggttgtt gtggtgtttg tttttatttt ttttttgttt 6660
aggggttttg gggtttttgt tgtgtttgtt tttatttttt ttttgtttag gggttttggg 6720
gtttatgtat attttttttt tttttttttt gagatggagt tttgttttgt tttttaggtt 6780
ggagtgtagt ggtgtgaatt gggtttattg taagttttgt tttttgggtt tatgttattt 6840
ttttgtttta gttttttgag tagttgggat tataggtatt tgttattatg tttggttaat 6900
tttttgtatt ttttagtaga gatggggttt tattgtgtta gttaggatgg ttttaatttt 6960
ttgattttgt gatttgtttg ttttggtttt ttaaagtgtt ggtattatag gtgtgagtta 7020
atgtgtgtgg tttttttttt tttttttttt tttttttttg agatggagtt ttatttttgt 7080
tgtttaggtt ggagtgtagt ggagtggttt tggtttattg taatttttgt tttttgggtt 7140
taagtgattt ttgtgtttta gttttttgag tagttgggat tataggtgtt tgttatagtg 7200
tttagttaat tttttttttt tttttttagt agagatgggg ttttattatt ttggttaggt 7260
tggttttgaa tttttgattt tgtgatttat ttgttttagt tttttaaagt gttgggatta 7320
aaggtgtgag ttattgtgtt tggttagtat gttttttaat tagtatgtag gttgtgatat 7380
gtggaaatta ggggtatagg ttttgaagtt tgtattttgg agttggagta gttagggtta 7440
aatgggagtg gttgggaagg gtgtgttttt atgaggtttt tttgatgttt tagtgtttag 7500
tttggatttg ggtagaaggt gtggttagtg ttttgagtta gtggagtagt tgtttgtgtt 7560
tggtttttgt gagatgggtt ggttaggggt taggtagagt ttgtgtttgg tttttgtgag 7620
atgggttggt taggggttag gtagagtttg tgttgggggt agtaggtgtt ttttaaaggg 7680
gttagtgata gttttttttt tagtagtgag aaatttatgt ttaggggttg ggtgtggtgg 7740
tttatgtttg taattttagt attttgggag gttgaggtgg gtggattgtt tgaggttagg 7800
agtttgagat tagtttggtt aatatggtga aattttattt ttattaaaaa tataaaagtt 7860
agttgagtgt ggtggtgtgt gtttgtaatt ttagttattt gggaggttga ggtagaatat 7920
tgttggagtt tgggaggtgg agtttgtggt gatttgagat tgtattattg tgttttagtt 7980
tgggtgatat agtgagattt tgttttaaaa ataaataaat aaataaataa ataaataaat 8040
aaataaataa atttggtttg agttttgata aatgtatatg tagggttaat gggaagatag 8100
atgttggagg gtggaagttt gattgtgaga gtgagttttt tgtttttttg aatagttagt 8160
ggaggaggtt ttgggagttt aggtgagtat ttgaatggta gggtagagtt tgagggtttt 8220
ggtggagata ggtaagaagg ttgtaggaat ttagtttggt ttagtgtttg ggagaatggg 8280
agggaatatg gaggtaggga ggtagagggg ttggtaggat ttggattgga ttgtgtgttg 8340
tagttattta tagtttgatt ttattgtttt gaagtgagtg atgtattttt attagtgttt 8400
agtgtttaat tttattttgg ttttgagtgt gtaatggttt tgtttttgtg ggtttttttg 8460
ggatgagggg tgagttttgt ttttttttgg ttattgtttg ttattaggag ttgttttgtt 8520

tatagttgtg atttaggagt tattgtattt tttttagttt tttgggtgtt gttagggggt 8580
ttttttattt tgggagttgg tgtgttttga ttggaggttg ttatgtgtag agtgttgtta 8640
aggagaaggg gtgtttattg tttagttttg aggtttaagg ttttggttag ttttgttgtt 8700
agtgttgtag ggaaaaagtt ttttttgtgt gtgggaagtt taataaattt tgtttagatt 8760
gtgttttgta gtgagtgttt ggaatttttt agataagttt taggtgtttg gttttttagt 8820
tggtgtggaa agtgtgggtg attattaagg ggggtgggtt ggggtagatg gagttggtgt 8880
gagtttgttt tttttttttt tttttagaa aggtagtttt ggagtttatg ttttgttttg 8940
tttttattgg taaaaagtat aattttatta gaaataggaa agttttaaaa agtattaatg 9000
agttaaaaag agggttgggt atgtttggtt atgtttgtaa ttttagtatt ttgggaggtt 9060
aaggtgggtg gattatttga ggttgggagt tagtttgatt aatatggtga aattttgttt 9120
ttattaaaaa tataaaatta gttgagtgtg gtggtgtatg tttgtaattt tagttatttt 9180
ggaggttgag gtaggagaat tatttgaatt tgggaagtag aggttgtggt gagttaagat 9240
tatgttattg tattttagtt tgggtaataa gagtgaaatt ttgttttgaa aaaaaaaagg 9300
gttggggttg ggtgtggtgg tttatgtttg taattttagt attttgggag gttgaggtgg 9360
tttgattata aggttagaag tttgagatta gtgtggttaa tatggtgaaa tttttttttt 9420
attaaaaata gaaaaattag ttaggtgtgg tggtatatgt taataatttt agttatttta 9480
tttgaggtta ggagtttaag attagtttgg ttaatatggt gaaattttgt ttttattata 9540
aatataaaaa ttagttaggt gtggtggtgt gtgtttgtag tttagttatt ggggaagttg 9600
aagtaaggag aattgtttga atttgggagg tggaggttgt ggtgagttga gattgtatta 9660
ttgtatttta ggttgggtaa gtttaggaga gaagagtgaa attgtgtttt aaaaaaataa 9720
ttaattaaaa taaaattagt gttataggtt gttttggtag gtagtgggtt ttttagggtg 9780
gagtagggaa gggttggaaa gggttgtttg tgtttagttt gattttttgt gttttttggg 9840
ggatagtatg gttagttgtt gttgtggagt ttatagtggg gttttggggg gggggggtgta 9900
ggtagaaggt tgttatgttt tttttttttt ttttatttta gtgagagtgg gtttgaaggt 9960
agggagtttg tttgttttgt ggagtttttg tagaggtatt tagggttgtt gtgagtgtat 10020
agtttggtgg tgttttatgg ttttgttttt ttttgttatt tttatttgtt gtaaaatatt 10080
gtgtttttgt attttttttt tttatttgtt tggaaatgtg aaaattattg ttgttgtagg 10140
aaatgggttt agatttgggt tgtggttttt ttatttgtat tgtttttgtg gtgtttagtt 10200
aggaggtggg aggtgggtgg tatttagtgt gtttggttgt ggtgatgatt ggtagatgtt 10260
tggatttatg gtaaagagga tttttgtgag ttgggagtta gttttggatt ttgtaggttt 10320
ttatatttta ggtggttttt atggggtttt agtatttgtt gaggtttttg tgttagttat 10380
gtgaggtttg ggtgtttttg aggtttttgt ttgtgttttt ttttgggtag tgggagggag 10440
agttttgaga tttagaggga gtttaggggt tgggtagttg tagaatagtg ggggtaatgg 10500
gtttattggg ggttttgggt tgtgtatata gtgggttttt ggtgtttagt tagttggggt 10560
aggtttggaa tgttggtttt ttttttagga tttggggtgt tgttgtagtt gttggggaat 10620
tagtgaggtg gttggatggt ttgtatattt tataaatgtt attgtattgt ttaaattgga 10680
tttaattttt ttaatatttt tttttttttt tttttttttt tttttggaga tagggtttta 10740
ttgtattgtt gaggttggag tgtagtggtg taattatagt tttgggttta ggtgattttt 10800
ttgtttttaa ggtagttggg attgtagatg tgtattatta tatttgatta gttttttgta 10860
gagatggggt tttgttatgt tgtttaggtt ggttttaaat ttttagattt aagttatttg 10920
tttgtttggg ttttttagag tgttgggatt ataggtatga gttattgttt tgttatgttt 10980
atttttattt atttatttat ttatttttga gatggagttt tgttttgttt tttaggttgg 11040
agtgttgtgg tatgattttg gtttattgta agttttgttt tagtttttta agtaagttgg 11100
aattataggt gtttgttatt aagtttggtt aattttgttt ttgtattttt agtagagatg 11160
gggttttatt gtgttagtta gtatggtttt gattttttga ttttgtgatt tgtttgtttt 11220
ggttttttaa agttttggga ttataggttt gagttattgt gtttggttta tttttttatt 11280
ttttatttta tagagataag ggtttgttgt gttgtttagg ttggttttga atttttgatt 11340
ttaagtaatt ttttttggtt ttttaaagtg ttgggattat aggtgtaagt tatagtattg 11400
ggtttaaagt ttattttttt tttttttttt tttttttttg agatggagtt ttgttttgtt 11460
atttaggttg gagtgtagtg gtgtgatttt ggtttattgt aagttttgtt ttttgggttt 11520
atattatttt tttgttttag ttttttgaat agttgggatt ataggtgttt gttattatgt 11580
ttggttaatt tttttgtatt tttagtagag atggggtttt attgtgttag ttaggatggt 11640
tttgattttt tgattttgtg atttgtttgt tttggttttt taaagtgttg gggttatagg 11700
tgtgagttat tgtatttggt aaagtttatt tttttaaagt gtgtaattta gtggtatata 11760
gtatattgat agagttgtat aattattatt tttattagtt tttagaatat ttttgttatt 11820
ttagaaggaa attttgtttt tgttagttat tattttttat ttttttttta gttttggtat 11880
ttatgtattt tttttttgtt tttggattgg tttgttttga atattttata tgagtggtgt 11940
tatatattgt gtggtttttt gtgtttggtt tgttttatgg agtgtgttgt ttttaagggg 12000
tatttgtgtt gtggtttggg tttgaatttt tttgttgttt atggttgtgt tattttttag 12060
tgtgtggagg gttatgttgt gtttgattgt ttatttgtgg ttggatattt gggttgtttt 12120
tgtttttgtt tgtgaattgt ggttgttttg gtgtatgtgt gtttagttgg tgttttttta 12180
gaatttttga gggttttgtt ttagttatat taggagggat ttagggatta gggtatgtgg 12240
gtttggagtt gggtgttttt gggtttttat gtgtttttta gttttgtttt gagttgtggg 12300

aggttggggt gagggttagg tttatttttt ttttgtatga tttgttttta ggtagatgga 12360
ggtttttagt tttatttttt tgtgagtttg agtggtgttt atgatttttt tttttatttt 12420
ttgttttgtt aattaataag ttagttaaat agtttatgtt gtttgggtaa gtttagtagg 12480
tttttaatgt ggtattttgg agtggttggt gatgttttaa gagttgggtt tttttttttt 12540
ggaggttttt tttgtttttt gttaagtttt taggttttgt tgggtttttt tttttgtttt 12600
ttttttggtt ggagatgtta gaggaagttt ggtgatttag agaagttgat gtgtatttat 12660
tattttggag gtgattgtta ttaggtgggg gtggttttat ttttattttt tttttggtat 12720
aaattggggg atgtatgtgt ttgtgtagaa tgttttggtt ttttttaatt aaatagataa 12780
tttagtgttt tttggtttat gttgatttgt ttaggagtgt ttgggttttt ttatgtttta 12840
gttgttgtgt gttttgttgt tgtgaagtgt ttttgttgag gtggaggtag ggtagggtgt 12900
ttttggtgag ggtgagtttt tttgtgtttg tttttttatt tgtagaatga tggtttatgg 12960
aggagatatg gtgttggtag gtggtggttg gtgtagggag gtttttagga gggagttttt 13020
tatttttatt tgtttttagg gttgtgtagt tttttgagtg gtttttatga ggttttgagg 13080
ttgtatgtag gttgagtttt tggttttatt gttttttttt agggttagga ttgtgttgga 13140
gtttgggtgg gtgtggggtg gtttttgtat aggtagtagg aggtgggtta gagtggtgtt 13200
ggatttgaag gtttgtggtt tttttagttt tattgaggag gttggaatag tgttttgggt 13260
tatttttttt gttggtagtt ggtttttgtt gagggagggg gtgtttttgt tttttggtgg 13320
agtattaggt ttttggggtt tgtgtgtttg agtatttggt ttttgttgtt tgaaaatagt 13380
attttttttg ttttttggtt attgtatttt ttttgaatag aaggtaagtt ttgtttttta 13440
ggaatggttt gtgattttta ttagtggggg aggttagggg tatatagagt tttggggaat 13500
aaggggattt ggggttgagt tgggtttttt gggtttattt tgagtttttt tgagtagttt 13560
ggtaggtagg aaggtgttgg gttttggggt ttttgttgag tttagtttta tttttagtgt 13620
tttttttaat gttttgggtg tttgttaggt tgtggatata ggttttggtt agttttataa 13680
ataattttgt tgggttttgt tgggaattgg tgttattatt atatagttta gagagaatgg 13740
aaatataggg ttttttagtg agtagattag gtttgtggtg attagtggaa gtgggggatg 13800
tttttagggt tgggtagttt gatagggtat tgggtatagt taggggttgg aattagttgt 13860
ttattggttt tgtttttggg ggattgtgtt tttttgtgtt ttgtttttat tatattttgt 13920
ttttttttta ttttgttttt ttagtttttt tttagattgg tggttttttg tttaggttta 13980
gttttagtat agtttgtagt tttggtagag tttaggtttt ggttttttga ggttgttttg 14040
agggtagtga gtaatgagtt ttgggattgg tgtattggga gggaaaggtg agttaaggtt 14100
ttttgtaaga ggtagttttg agttgggggt ggggaaggtt gggttgtggg atatttttga 14160
tattttaagt tttttttttg agatggagtt ttgttttgtt atttaggttg gagtgtagtg 14220
gtgagatttt gattttttgt aatttttgtt ttttaggttt aagtgatttt tttgttttag 14280
ttttttgagt agttgggatt ataggtgttt attattatgt ttggttaatt tttgtatttt 14340
tagtagagat aaggttttat tatgttggtt aggttggttt tgaatttttg attttaggtg 14400
atttattttg gttttttata gtgttgggat tataggtgtg agttattgtg tttggttttt 14460
tagttgtttt ttatgtttta tgtttgggtt ttttgttaag aattaaaaaa gaaaaaaaaa 14520
attatggaga tggaatttat atattgtata atttatttgt ttatagtgta taatttagtg 14580
gtattaagta tatttatatg gttgtgtaat tattattttt aattttagaa tgtttggtta 14640
ttttaaaagt aagttttatt tttattgtgt tattttttat tttttttatt agtttttggt 14700
tattttgaat ttattttttg gttttggatt ggtttgtttt ggatattgta tagaaatggg 14760
attttgttgg ttgggtgtgg tgatttatga gagtaagaga tttagattat tttggttaat 14820
atggtgaaat tttattttta ttaaaaatat aaaaattagt tgggtgtggt tgtgggtatt 14880
tgtagtttta gtgatttagg ggttgagtta ggagaaattg tttgaatttg ggaggtagag 14940
gttgtagtga gttgagatta tgttattgta ttttggtttg gtgatagagt gagattttgt 15000
ttttaaaaaa aaaaaaataa gaaatgggat tttgttattt tagtgttttt tgaggttgag 15060
gtgggtagat gatttgagtt tagtagttta aatgagatta gtttgggtag tatggtgaaa 15120
ttgtttttat taaaatataa aaattagttg ggtatggtag tatatgtttg tagttttagt 15180
tatttgtggg gttgagatgg gaggattgtt tgagtttggg aggtgtattt tagtttgggt 15240
gatagggaaa ttttgtttta aaaaaaaaaa aaaaagtgta ttatatattg tgtgtttttt 15300
agtgttttat tgtgatattt ttaaggtgta gttaggttgt ggtttgagtt agagttttat 15360
tggtttttgt ggttgagttt tgttgagtgt gtggatgggt tgtgttgatt tttgtttgtg 15420
gaagggtttt tgggtggttt tatattttgg ttgttgtgtg ttttgttgtt gtgagtattt 15480
tgtgttttgt ttttgtgtgg tgtgtattgt tttttagggt gtgtatttgg gagtggagtt 15540
gttggtttat agggtaatag atttttggag aagttgtaga tgtttttatg tttatttatt 15600
gggtgtgagg attttgtttt ttgttatttt tgtttttgtt taattttttt ttttatgaga 15660
tagggtttta ttttgttgtt taggttggag tgtagtagta tgattatagg taattatagg 15720
tttaagtgat ttttttattt gagtttttttg agtagttagg atttgaggta tgtattttta 15780
tatttggtta atttttgtat ttttagtaga gatagggttt tgttgtgttg tttaggttgg 15840
ttttgaattt ttgagtttag gtgatttatt tgttttggtt ttttatagtg ttgggagaaa 15900
tgaagtgttt gtgatagggt ggtagttgta gagggggttg tgttttttgg tttgttgtgt 15960
tttatttttg tgataggtag gtgggtgtgg ttaattgggt ggtagttgta gagggggtag 16020
tgataggtgg gtgggtgtgg taggatttta taatgttttt ttttgtggtg tttttttttt 16080

tgtttgtttt tttttatggg ttattttttt agtttatttg tggtttattt tttaaggagt 16140
gggatttttg gagttgggag tttttggttg tgtgggattg tattgtgtga tggagttatt 16200
attagggttt taatttattt tggtttttgt tatagtttgg gttttatttg tgttattatt 16260
gtagtagttt ggtttttagg aagtaaaatt gttattgagt tataaagttt aattaagagt 16320
gttttttggg aggttgaggt gggtggatta taaggttagg agattgagat tattttggtt 16380
aatatggtga aattttgttt gtattaaaat ataaaaaaat tagttggttg tggtgatatg 16440
tgtttgtagt tttagttatt tgggaggttg aggtagggaa attatttgaa tttgggaggt 16500
agaggttgta gtgagttgag attgggttat tgtgttttag tttagggata gagtaagatt 16560
ttgttttaga aaaaaaaaaa agtgttttaa ttgtgttttt taaagttttt gttggttgat 16620
gatagattag agggtgttgt gtttttattt tttattgttt tgagtttgga tgtgtggttt 16680
ttgtagggtt tttttttata gtattgtgaa tttatagttt ttttttaggg aagggaggag 16740
gtatgttatt tttataggga gatggggagg ttgattttag ggatttaggt tattattttg 16800
atgttgggtt ggttgatata tttttgtttt ttttgtttta gg 16842

<210> 320
<211> 16842
<212> DNA
<213> Artificial Sequence

<220>
<223> chemically treated genomic DNA (Homo sapiens)

<400> 320

tttgggatag agaggatagg ggtgtattag ttgatttaat gttaggatga tggtttggat 60
ttttggagtt ggttttttta tttttttgtg gaagtggtgt attttttttt ttttttagag 120
agaagttgtg agtttatagt gttgtgggaa agggttttgt aggggttatg tgtttaggtt 180
tagggtggta gggggtggga gtatagtgtt ttttagtttg ttattggttg gtgagggttt 240
tggaggatat agttaggata tttttttttt tttttgagat agagttttgt tttgttttta 300
ggttggagta tggtggtttg attttagttt attgtaattt ttgtttttta ggtttaagtg 360
atttttttgt tttagttttt tgagtagttg ggattatagg tatgtgttat tatagttggt 420
taattttttt gtattttagt atagataggg ttttattatg ttggttagga tggttttgat 480
tttttgattt tgtgatttgt ttgttttggt tttttaaagg atatttttaa ttgagttttg 540
taatttagta atagttttgt tttttagaaa ttagattatt gtaatggtag tatagatgga 600
gtttagattg tggtaggagt tagggtgagt tggagttttg gtgatggttt tgttatatag 660
tatagtttta tgtagttaag ggtttttaat tttgagggtt ttattttttg gagggtgggt 720
tgtaggtgag ttggaaggat ggtttatagg aaggggtagg tgaagaagga gatgttatag 780
aaagagatat tgtggggttt tgttatattt atttgtttgt tattgttttt tttgtagttg 840
ttgtttagtt ggttatgttt atttgtttgt tataagggtg gggtataata ggttagagag 900
tatagttttt tttgtagttg ttgttttgtt gtagatattt tatttttttt agtattatag 960
gaggttgagg tgggtggatt atttgagttt aggagtttga gattagtttg agtaatatgg 1020
tgaaattttg tttttattaa aaatataaaa attagttagg tgtggaggta tgtgttttaa 1080
gttttagtta tttgggaggt ttaggtggga ggattatttg agtttgtagt tatttgtgat 1140
tgtgttattg tattttagtt tggataatag aataagattt tgttttataa aagaaaaaat 1200
taaataaaaa taaaaatgat gaggagtaag gtttttatat ttagtgggtg ggtgtggaag 1260
tgtttgtagt ttttttaaag gtttgttatt ttgtgagtta gtagttttat ttttaggtat 1320
atattttgag gaatagtata tgttatgtag aaatggagta tgagatgttt atagtagtag 1380
gatgtatagt agttgagatg tggaattatt taggggtttt tttatagatg aggattagta 1440
tagtttattt atgtatttaa taagatttag ttataaaaat tagtgaggtt ttgatttgag 1500
ttatagttta gttgtatttt gaggatgtta tagtgagata ttaaaggata tatagtgtgt 1560
gatgtatttt tttttttttt tttgagatag ggtttttttg ttgtttaggt tggagtgtat 1620
tttttaggtt taagtgattt ttttatttta gttttgtaag tagttgggat tatagatgtg 1680
tgttattatg tttagttaat tttgtatttt tagtagagat agttttattg tgttgtttag 1740
gttggttttg tttgaattat taggtttaag ttatttgttt attttggttt tggaaaatgt 1800
tgggatgata ggattttatt ttttgttttt ttttttttgg agatggagtt ttattttgtt 1860
gttaggttag agtgtaatgg tatgatttta gtttattgta atttttgttt tttgggttta 1920
agtgattttt tttgatttag tttttgagtt gttgggatta taggtgtttg taattatgtt 1980
tagttaattt ttgtattttt agtagagatg gggttttatt atgttggtta ggatggtttg 2040
gatttttttgt ttttgtgagt tattgtgttt ggttgatagg attttatttt tatgtaatat 2100
ttaggataag ttaatttaga gttaggaagt ggatttagag tggttaggag ttggtggagg 2160
ggatggagag tgatataatg ggaatggggt ttgtttttgg ggtgattgaa tgttttggaa 2220
ttagaagtag tggttgtata gttgtgtgaa tgtatttaat gttattgaat tgtatattgt 2280
gaatgaatga gttgtatagt atgtaaattt tatttttata gttttttttt ttttttttaa 2340

tttttaatag gaagtttaag tatggagtat aaaaaatagt tggggggtta ggtgtggtga 2400
tttatgtttg taattttgat attatgggag gttgaggtgg attatttgag gttaggagtt 2460
tgaaattagt ttggttaata tggtgaaatt ttgtttttat taaaaatata aaaattagtt 2520
gggtgtggtg atgggtgttt gtaattttag ttatttagga ggttgaggta ggagaattgt 2580
ttgaatttgg gaggtggagg ttgtagagag ttgagatttt attattgtat tttagtttgg 2640
gtgatagagt aagattttat tttaaaaaaa aaatttgggg tgttaggggt attttatgat 2700
ttagtttttt ttatttttag tttagggttg ttttttgtag gaagttttgg tttatttttt 2760
ttttttagtg tattagtttt agggtttatt gtttattgtt tttagggtaa ttttagaagg 2820
ttaaggtttg agttttgtta gggttgtagg ttgtgttggg gttgggtttg gataaggggt 2880
tgttagtttg ggagggggtt aggagagtag gatgaaggga aagtaaggtg tgatggggat 2940
ggggtataga gggatatagt tttttagaag tggagttagt gagtaattgg ttttggtttt 3000
tggttgtgtt tggtgttttg ttgggttgtt tagttttgaa agtgtttttt atttttattg 3060
gttattgtag gtttggtttg tttgttgaga agttttgtgt ttttgttttt tttgggttat 3120
gtggtggtgg tattaatttt tagtagggtt tggtaaggtt atttatagaa ttggttaggg 3180
tttgtgttta tagtttggta ggtatttagg gtattgaggg ggatgttgag ggtgaggttg 3240
ggtttggtaa aggttttggg gtttagtgtt tttttgtttg ttaggttgtt taggagagtt 3300
tggggtgggt ttgagaggtt tggtttagtt ttagattttt ttgtttttta gggttttgtg 3360
tgtttttaat tttttttgtt gatgaaggtt ataggttatt tttgggaggt aagatttgtt 3420
ttttatttag aggagatgtg gtggttgggg ggtggggaag gtgttgtttt tagatagtag 3480
aggttagatg tttggatgta taggttttaa gagtttgatg ttttattagg aggtgaaagt 3540
attttttttt ttagtaggag ttggttattg gtagggaagg tggtttagga tattatttta 3600
gtttttttag tggagttgag ggagttatgg gtttttaggt ttagtattgt tttggtttat 3660
tttttgttgt ttgtgtaggg attattttat atttatttag gttttagtat agttttaatt 3720
ttgaggaggg gtagtgaggt taggagttta atttgtatgt agttttagag ttttgtgggg 3780
gttgtttggg aggttgtatg gttttaaaag taaatgaggg tggaggattt ttttttggaa 3840
atttttttgt gttggttatt atttgttgat attatgtttt ttttatgagt tattatttta 3900
tagatgggga aatgggtata ggaggatttg tttttattga aggtgttttg ttttgttttt 3960
attttaatgg ggatatttta tagtagtagg atatatagta gttgaaatgt ggaagagttt 4020
aggtattttt gagtaggtta gtatagatta agggatattg ggttgtttgt ttaattaaaa 4080
gggattaagg tattttgtgt aggtatgtgt gttttttgat ttatgttagg ggaggagtgg 4140
gggtggagtt atttttgttt ggtggtagtt atttttggga tgatgggtgt atattagttt 4200
ttttagattg ttaggttttt tttgatgttt ttagttagaa aaagagtaga agaggaggtt 4260
tagtgaaatt taagagtttg gtgaggaata ggaagagttt ttaggagggg gaagtttggt 4320
ttttggggta ttgttagttg ttttgaggtg ttatgttagg ggtttattgg gtttgtttag 4380
gtagtatgga ttgtttagtt gatttgttga ttagtaggat aggaaatagg aaggagagtt 4440
ataaatgtta tttaagttta tagagaggtg gggttaggag tttttgtttg tttagaaata 4500
gattatatag ggaagagatg ggtttggttt ttgttttggt tttttatggt ttagagtagg 4560
attgggaggt atatggagat ttaggaatat ttagttttag atttgtgtgt tttggttttt 4620
gaattttttt tgatgtggtt gaaataaggt ttttaaggat tttagaggga tgttggttga 4680
gtatatgtgt attaaagtag ttataattta taggtagagg tgggagtagt ttaagtgttt 4740
agttatagat gagtagttaa atgtagtgtg gttttttata tattggaaag tgatgtagtt 4800
gtgaatagta gagaggtttg gatttaggtt atggtgtgga tgttttttga ggatagtata 4860
ttttgtgaga taagttggat gtaaaaggtt atgtggtgtg tgatattatt tgtgtgaaat 4920
gtttaggata ggttaattta gaggtaggaa gggaatgtgt gggtgttggg attggggagg 4980
gaatggggag tgatggttga tagagatggg gttttttttt ggggtaataa gaatgttttg 5040
gaagttgata gaggtgatag ttgtgtaatt ttgttaatgt attatatgtt attgaattat 5100
atattttaaa aaggtgaatt ttgttgggtg tggtggttta tatttgtaat tttagtattt 5160
tgggaggttg aggtaggtag attatgaggt taggagattg agattatttt ggttaatata 5220
gtgaaatttt atttttatta aaaatataaa aaaattagtt gggtatggtg gtaggtgttt 5280
gtagttttag ttatttggga ggttgaggta ggagaatggt gtgaatttgg gaggtagagt 5340
ttgtagtgag ttaagattat gttattgtat tttagtttgg gtgatagagt gagattttat 5400
tttaaaaaaa aaaaaaaaaa aaaaaaaggt gaattttagg tttagtatta tggtttatat 5460
ttgtaatttt agtattttgg gaggttagag aggattattt aaggttaggg gtttaagatt 5520
agtttgggta atatagtaag tttttgtttt tataaaataa aaaataaaaa ggtgggttgg 5580
gtatagtgat ttaagtttgt aattttagga ttttgggagg ttgaggtggg tggattataa 5640
ggttaggaga ttgagattat attggttaat atggtgaaat tttgttttta ttaaaaatat 5700
aaaaataaaa ttagttaggt ttggtggtag gtatttgtag ttttagttta tttgggaggt 5760
tgaagtggag tttgtagtga gttaagattg tgttatagta ttttagtttg ggggatagag 5820
tgagattttg ttttaaaaat aaataaataa gtaaataaag gtgaatgtgg tgaagtagtg 5880
gtttatattt gtaattttag tattttggga ggtttaggta ggtggatagt ttgagtttag 5940
gagtttgaga ttagtttgga taatatggtg aaattttatt tttataaaaa attagttggg 6000
tgtggtggtg tatatttgta gttttagtta ttttgggggt aggagggtta tttgagttta 6060
gagttatgat tgtgttattg tattttagtt ttggtgatat agtgagattt tgtttttaaa 6120

aaaaaaaaaa aaaaaaaaaa aaaaggtgtt aagaaaattg agtttagttt gagtaatatg 6180
gtggtgtttg tgggatatat gggttgttta gttgttttat tgattttttg atagttatag 6240
tagtgtttta gattttgaga gaggaattaa tattttaggt ttgttttagt tggttgggta 6300
ttggaagttt attgtgtgta tagtttagaa tttttagtgg atttattatt tttgttgttt 6360
tgtggttgtt tagtttttga gttttttttg ggttttagag tttttttttt tgttgtttag 6420
agaggggtat aaatagaggt tttgaggata tttaggtttt atatggttgg tgtaggggtt 6480
ttagtagatg ttggaatttt ataaaggttg tttgggatgt aaagatttat aaagtttagg 6540
attgattttt agtttgtgaa aatttttttt attataggtt taagtatttg ttaattattg 6600
ttgtaattag gtatattgag tgttgtttat tttttatttt ttggttggat attgtggggg 6660
tggtgtggat gggagagtta tagtttggat ttgggtttgt tttttatggt agtagtgatt 6720
tttatatttt taaataggta gaaagaaaag atatgaaagt atagtgtttt gtaataggtg 6780
gaagtgatag gaaagggtag ggttgtggga tattgttagg ttgtgtgttt atggtagttt 6840
tgggtgtttt tgtggaggtt ttataaggtg ggtagatttt ttgtttttga gtttattttt 6900
attggggtgg aaagggggag ggaggtatgg tggttttttg tttgtatttt tttttttaga 6960
attttattgt gggttttgta gtagtagtta gttgtgttgt tttttgagaa gtatggggag 7020
ttgggttggg tataggtgat tttttttagt ttttttttgt tttattttag gaatttattg 7080
tttattagaa atagtttatg gtattgattt tattttaatt aattgttttt ttgagatata 7140
attttgtttt ttttttttag gtttgtttag tttggaatgt aatgatgtga ttttggttta 7200
ttgtaatttt tgttttttag gtttaagtga ttttttttgt tttagttttt ttagtagttg 7260
gattataggt gtgtgttatt atgtttggtt aatttttgta tttgtagtag agatggagtt 7320
ttgttatatt ggttaggttg gttttgaatt tttgatttta gatgaagtag ttgggattat 7380
tggtatgtgt tattatattt ggttagtttt tttattttta gtagagagag ggttttatta 7440
tgttggttat gttggtttta aatttttgat tttgtgatta aattgttttg gttttttaaa 7500
gtgttgggat tataggtgtg agttattatg tttggtttta gttttttttt tttttgagat 7560
ggagttttat ttttgttatt taggttggag tgtaatggta tggttttggt ttattgtaat 7620
ttttgttttt tgggtttaag tgattttttt gttttagttt ttagagtagt tgggattata 7680
ggtgtgtgtt attatattta gttaattttg tatttttagt agagataggg ttttattatg 7740
ttggttaggt taatttttaa ttttgggtga tttatttgtt ttggtttttt aaagtgttgg 7800
gattataggt atgattgaat atgtttagtt ttttttttaa tttattgatg ttttttggaa 7860
tttttttatt tttaataaga ttatattttt tgttggtgag agtgggataa ggtatggatt 7920
ttagggttgt ttttttggga aggaagggaa gagatgggat ttatgttggt tttatttgtt 7980
ttaatttatt ttttttggtg attgtttatg ttttttatat taattggagg attggatgtt 8040
tgaggtttgt ttggaaggtt ttagatattt gttgtgagat ataatttgag tggagtttat 8100
taaatttttt atatataaag gaggtttttt ttttgtagtg ttggtagtaa ggttggttaa 8160
ggttttgggt tttagggttg ggtagtggat attttttttt tttgatagta ttttgtatat 8220
ggtagttttt agttagaata tattagtttt tagaatagga ggattttttg gtagtattta 8280
gggggttgga ggaaatgtag tggtttttga attatagttg taagtggggt agtttttgat 8340
ggtagatagt gattaggaag ggatagggtt tattttttat tttagggggt tttgtaaggg 8400
taaagttgtt atatatttag ggttaggatg aaattggatg ttgaatatta gtgagggtgt 8460
attatttatt ttaaggtagt ggggttaagt tgtgggtagt tgtgatgtat agtttagttt 8520
aggttttgtt aatttttttg tttttttgtt tttgtgtttt tttttgtttt tttaggtatt 8580
gggttagatt aggtttttgt aatttttttg tttgttttta ttaagatttt taggttttgt 8640
tttattgttt aggtgtttat ttgagttttt aggatttttt ttattgattg tttaggaaag 8700
tagggggttt atttttatag ttaggttttt attttttagt gtttgttttt ttattgattt 8760
tgtatatata tttgttaaaa tttaagttag atttatttat ttatttattt atttatttat 8820
ttatttattt atttttgaga tagagttttg ttgtgttgtt taggttggag tgtaatggtg 8880
tgattttggg ttattgtaag ttttgttttt taggttttag taatgttttg ttttagtttt 8940
ttgagtagtt gggattatag gtatatgtta ttatatttgg ttaatttttg tatttttagt 9000
agagatgggg ttttattatg ttggttaggt tggttttgaa tttttgattt taggtgattt 9060
atttatttta gttttttaaa gtgttgggat tataggtgtg agttattgtg tttggttttt 9120
aagtgtgggt tttttattgt tgggagagag attgttattg gtttttttag agggtatttg 9180
ttatttttag tataggtttt gtttagtttt tgattaattt attttataga gattaggtat 9240
aggttttgtt tagtttttga ttaatttatt ttatagagat taggtatagg taattgtttt 9300
attgatttaa ggtattggtt atatttttta tttgggttta gattgggtat tgagatatta 9360
gaaaggtttt gtggaaatat atttttttta gttgttttta tttaattttg attgttttag 9420
ttttagagta taaattttag agtttgtgtt tttggttttt gtatgttata gtttgtatgt 9480
tgattaagaa gtgtgttggt tgggtgtggt ggtttatgtt tttaatttta gtattttggg 9540
aggttgaggt aggtggatta tgaggttaga agtttaagat taatttggtt aagatggtga 9600
aattttgttt ttattaaaaa aaaaaaaaaa aaattagttg ggtattgtgg taggtatttg 9660
taattttagt tatttgggag gttgaggtat gagaattatt tgaatttagg gggtggagat 9720
tgtagtgagt taagattatt ttattgtatt ttagtttgag taataagagt gaaattttgt 9780
tttaaaaaaa aaaaaaaaaa aaaaaaaaaa ggttgtatgt gttgatttat gtttgtagta 9840
ttagtatttt gggaggttga ggtgggtgga ttatgaggtt aggagattga gattattttg 9900

gttaatatgg tgaaattttg tttttattga aaaatataaa aaattggttg ggtgtggtgg 9960
tgggtgtttg tagttttagt tatttgggag gttgaggtag gagaatggta tgaatttggg 10020
aggtggagtt tgtagtgagt ttagtttgtg ttattgtatt ttagtttggg gaatagagtg 10080
agattttgtt ttaaaaaaaa aaaaaaaaaa atgtatgtga gttttagggt ttttgagtag 10140
gagagaggtg agagtagata tggtaggagt tttagggttt ttgagtagga ggaaggtgag 10200
agtagatatt gtagtagttt ttttttggat ttttttttaa ggttatggtg ttttgtgtag 10260
ggtgtttttg ttagtttttt gaggtttagt ttagtagata aggttttttt ttggaaatag 10320
gaggtaaagt tttgaaagtt aagaaggttg ggaaaggagt agggtttggg tagggtttgt 10380
taggtggtgg taggtgtagt gtgtttggtt gtgataaggt agggatattt ttggttattt 10440
ttattagttg ggagaggttt aggttggtta gtgttgtttt aggtaggagt ttttttgtgt 10500
atagatgtag aggtgtttgg gatgtttagt ggtagttttg tgtttgaaga agttagttgg 10560
aaaagtaagg ttttttggat gtaatttggg agggaggtat agtttttttt gggtagggtt 10620
tttagttttg ggggttaggg atgtttttag tgttatattt ttggtaaatg aggaaaggtt 10680
atgttttttt tttgttgatt gataagttgg tgtaaatagt tatttttggt agtttagggt 10740
ttttttttga taggataaat aaattatatt ttgtttggtt ttggtaatgg ttatatattg 10800
tgtagaaaat gtgttggtgg ttgtatataa atttttgggg tttaaataaa tataggttga 10860
ttttattggg tgtttttgtt ttttggatag ttgggggagt taaggggtta gttttagggg 10920
attgggggtt gttttttttt taggagggtt ttgttattag ggtattttaa ttggagttta 10980
agagtttatg taaatgtatg ttttgttgtg ataattggtt ttgtttgagt gaaagtttgt 11040
aatgtaggtt tttaattatg ttttttttgg tatggaggat ataggtgtta gatgggttta 11100
gtttagggtg ttaagaggaa gtaagggagg gagggaggag agaaggaagg aagatagaat 11160
tattagtatt gtgattggtt tggtttggtt ttgattttag taagttatat ttattttttt 11220
atgttggttt ttttgttggg gattttttgt aatttttttt ttttgaggat tttgatggtt 11280
tttttgtata gtgtttttga gtttagtatt ttttttattt tgttgtaaga gttttttttt 11340
agtaggtttt ttattaggta tttgttgatg agtttggttt gtttgtggtg tggttggtag 11400
atgattttgg tggagttgat gtggtggatg aatgtgttta tatttattga tattagtttg 11460
ttttttgtga atatgtttag ttgttgtggg tttattattt ttatgttgga tttagggttt 11520
tggagttttg ttgttagttt tgagtttgtg ggtgagtggt ttttttttgt gttttgattt 11580
ttttttttta aaattttata aagaaaaaag aaaaaaaaaa ggtaataaaa attttaaaag 11640
gaagggaaaa attttttttt taatagaaat gattgtttta tatgttagtt ttggttgtga 11700
gtttaagttt ggtgtagggt ttgtggattg gtgggtattg ggggtttggg atggtggttt 11760
agatgtgagt tttagtggga tttgggtttt agaattggga gatggtttgg ttagggttaa 11820
ttttgttttt ggggattttg gggaggttgg ttggggtttt ttagtgttag gttatttttt 11880
ggggatttta ggtttatgga taagtatgaa tatggttgag tgtagttttt tttgggttgt 11940
ttattttata ggttgggttt gtgatgttgt tattttttgg gatttttata tttggagagt 12000
ttaattttgt tgggggtgtt tttggtgatg ttttggtttt attgtttgtt ttaggagtaa 12060
atgttttgtt gtggatgatt gagtggattt tttggggggt gttgattttt gttggtttgg 12120
gtgagtgttt ggggggttgg gggtgtttgg gaggtttttt gttgtggtta atagttttgt 12180
ttagggttgg ttgttgtttg gtgtttgtga gggtttgtat tgggtgtttt ttgagttggt 12240
ttgtgatttt taaggtggtt gttggggttt ggtaggtgtt tttttttttg taaatgtttg 12300
tttgagtgtg tgttttgttt tgagtgtttt tagtgtttgg tttgtggggt ggtgtggtgg 12360
tgggggtttt atggggtttt tggtggttga ggtggtgtag gttgtaggtt ttgggttggg 12420
gggtttttga aggtgttggg ttttgggtgg tgtgtttttt ttgttgtggt tgttgtgtgt 12480
gtggaggagg tggagtggtg aggggggttt agtttattta aggttgtggt agtaagtgtt 12540
gttatttgtg ttttttttgt tgttttgtgt tttatttgtg ggtgttttgg gttgtttttt 12600
ttttttgtgt ttgatatgtt gttgttattt tgtttatttt tttttttttt tttgtgttgt 12660
tgtggatatt gtatgtttgg ttgtagggta tgtttttttg tgaggatgat gtgtttattt 12720
gttggttgaa tttgttattt ttggtttttt gtttgttttg ttgttgttga tgattggagt 12780
gtttggattt ggttgggtgt ttgagtggtg attggttggt gtttggtttt gtttgagggg 12840
tggatatagt gtgtttattg gtttttggag atgtttgtta tgggtagggg gtgggaagta 12900
ggtatgggga gatgaggtta tggttgtgat tttggtgggg tttgggaagg gggttgtagg 12960
ttatgggtgt taatggtttt tttgaatggt tgtagggtgg gttgagtttg gtgggtggga 13020
atttgggttt gtggatttgg aaggagtggg gttattttta ttagagtttt ttttttgtat 13080
ttttttttgg ggtgaggtta agggttaaaa agtaatataa agtggttaat tttagttaaa 13140
aatttttttt ttgagttatt tttggattag gggaaagaga aatgtttagg gtggtttttg 13200
ttttattgga atttatgggg gaagttttag gatagtgttg ggataagttt agtgggggtt 13260
ttttgtgaaa aataagagtt gttaaaatat ggtttgataa ggttgaaaat attgaatttt 13320
tttgtgttag ttttgtgttt tttatttttt tttgtattag tgttaggttg tttagttgga 13380
ataaattatt ttggtataat ttttgttttt tggttttaag tgattttttt gttttagttt 13440
ttttagtagt tgagattata ggtgtgtgtt attatgtttg gttaattttt gtatttttag 13500
tagaaattat aggtgtatgt ttgtaatttt agttatttag aaggttgagg gaggagaatt 13560
gtttgaattt gggaggagga ggttgtggtg agttgagatt gtgttattgt attttagttt 13620
gggtaataag agtgaaattt tgttttaaaa aaaaaaaaaa gatttttttt agaaaattga 13680

agttgaagga atatttttta atttaaattt attttggtat tttttttttg tattgattgt 13740
tggtttatat tttaattttt tggaatttgg agataatgtt ggataattgg tattggtagg 13800
ttttatgttt ttttagagaa ttgtaggata tatggaagtg atttaggaaa tatttggtga 13860
ttattataga tagaattttt gaagatggat tttttgttta aagattattt tttttaagaa 13920
ttttttttag ttattttttt aagtgtttgg atggagatgt gttgtgagtg tgaattatat 13980
atggaatttt tttgtttttt tgatatgtat aattttatta taaaatgtta aaaaatgtag 14040
tattttaaaa aatgtaagat gtattgttga tattaatttt tataagtgtg ttgtgttgtt 14100
atatataata aattttttaa aaattattaa atatttaggg atattaggta gggtgtggtg 14160
gtttatattt gtaattttag tattttggga ggttgaagta ggtggattat gaggttagga 14220
gtttgaaagt agtttggtta atatggtgaa attttatttt tattaaaaat ataaaaatta 14280
gttgagtgtg gtggtatgtg tttgtaattt tagttattta ggaggttgag gtaggagaat 14340
tgtttgaatt aggatttggg aggtggaggt tgtggtgagt tgagattatg ttattgtatt 14400
ttagtttagg taataagagt gaaattttgt tttaaaaaaa agaaaagaaa agaaaagtat 14460
tttttgtata gagaatttta tgagttatat gaaaatttgt atagttttat attgaagtta 14520
atattgagtt gtattagaat ttatattttt agtaaaataa gtttattgta taggtattat 14580
taaaggatag ttatttattt tttattttta tttttttttt ttttattttt attttttttt 14640
tttttttttt tttttttatt ttttggtttg tttttttttg agtttttttt ttttgtttta 14700
tttttgtttt tagtatgata tgtagtaata ttttttttat attttttttg tagtttaatt 14760
gttttttgtt tatatggttg tttatttttg gttgattgtt tagattatat tttatttagt 14820
ttttttgtag tttttatgat aaataggttt gggtgtttat ttttgatttt tgggtgatgt 14880
ttagagtaaa ataggaagaa ggtagatggt ggttttttag gagtattgtg attttttttt 14940
tttttttttt tatttttttt tatatttgg aggaatgtaa tttttaattt ttttgttata 15000
gtgagttttg ttattttttg tttttttttt aaattttatt ttttttttgt agatgtggtt 15060
ttttattttt ttaaatattt tttggagaat tttatgaaat tgatagaaga gtttgggtgt 15120
ttttttttgt gttttttttg gtaggtttgt gtgaagaggg tatatgagga tattttgttt 15180
tttggtttgt tggggtgttt tttatttatg gggatagaat agtatttatt tggtgtgagt 15240
ttttttttag agttttatag agtatttgga tttaatagag atattttttt ttatattgat 15300
tttttttttt tttttttgag atggagtttt gttttgttgt ttgggttgga gtgtaatggt 15360
atgattttgg tttattgtaa tttttgtttt ttagatttaa gtaatttttt tgttttagtt 15420
tttggagtag ttgggattat aggtatatat tattatggtt ggataatttt tggattttta 15480
atagagatag ggttttatta tattgtttag gttggttttg aatttttaat tttaggtgat 15540
ttatttgttt tggtttttta aagttttggg attataggta tgaggtatta tgtttgtttt 15600
atatattgaa tttttaaagt tagaaaaatt aaaaatgggt ttgttaggta tggtagttta 15660
tatttgtaat tttagtattt tgggaggttg aggtggatgg attatttgag gttaggagtt 15720
tgagattagt ttggttaatg tggtaaaatt ttgtttttat taaaaatata aaaattagtt 15780
aggtgtggtg gtgggtgttt gtaattttag ttatttggga ggttgaggta ggaggattgt 15840
ttgaatttag gaggtagagg ttgtagggag ttgagatttt gttattgtat tttattttgg 15900
gtaataatga gatttgtttt tgaaagaaaa aaaaatgggt ttggtatggt ggtttttgtt 15960
tgtaatttta gtattttggg gggttaaggt aggtgattat ttgaggttaa gagtttaaga 16020
ttagtttggg taatatggta aaatttttgt ttttattaaa aatataaaaa ttagttaggt 16080
gttgtggtgt ttgtttgtaa ttttagttat ttaggaggtt gaggtaagag aattgtttga 16140
atttgggagg tagaggttgt agtgagttaa gattgtgtta ttgtattgta gtttgggtaa 16200
tatagtgaga tttggtttta aaaaaaaaaa aaaagggtat aaataaaata ttttattggt 16260
tagatatagt ggtttatgtt tgtaatttta gtattttagg aggttaaggt gggttgatta 16320
tgaggttagg agtttgagat tattttggtt aatgtggtga aatttggttt ttattaaaaa 16380
tataaaaatt agttgggtgt ggtggtgggt atttgtaatt ttagttattt gggaggttga 16440
gataggagaa aggtgtgaat ttaggaggtg gagtttgtag tgaattaaga ttgtgttatt 16500
gtattttagt ttgggagata gagtgagatt ttgttttaaa aaaataaata aataaaaata 16560
aaataaaata ttttattaat aattttaata atgattgtat gttgaaatga tgtaattttg 16620
gatatgttag gttaaagtaa atgtattatt aaaattaatt attttagggt tatgtatagt 16680
ggtttatatt tgtaatttta gaattttggg aggatttttt gagtttagga gtttgaggtt 16740
agtttgggta atatagtaag aatttgtttt aaaaaaaaat aaatatataa ataataggtt 16800
aggtgtagtg gtttatgttt gtaattttag tattttggga gg 16842

<210> 321
<211> 1157
<212> DNA
<213> Artificial Sequence

<220>
<223> chemically treated genomic DNA (Homo sapiens)

<400> 321

ggattttgtt ttttttgaag gttttaggga agagtgtttt ttgttttttt tgaaggtttt 60
agggaagggt ttgttttttt aggtttttgg tggtttgtag gtgtagtttt ttaatttttt 120
tttttaagtg gtttgttgtt tataaggata tgagttatat tggatgaggg gtttattaat 180
tgatggtttt tgtaaagttt ttatttttaa ataaggttat attgtgaggt attgggagtt 240
aggattttaa tatagttttt ttggtggata taatttaatt tttaataatg tttatataat 300
tttaagtagg gtttggtatt ttgtgtgttt tttggagagt ggttgagtta ggttttggta 360
gtgtttaggt tattggtgat tgtagttttt ggatggtatt gtttattata ggttttggag 420
gttgttttta tggttttttg atagggtttt tgttggtttg ggggtttttg attaggggag 480
tggttttagg aggggagaga tttgtgtttt gggtttagtg tagttgtttt gagtaggatt 540
gggattttta ttaagtgggt gttgttttat gatttttgtg tgtttttagg attatttggg 600
tatgtggtag gttgtttgta tgtttgtgga ttatttttgt gataggaaaa ggtatgggtt 660
atttggtaaa ttaaggtata gagttttagg tggaagttgg gaaggtgttg tttggtttgt 720
attggttgaa gggttatttg ggttaggtgt atagggtagt ggtgttgttg gaggattagg 780
gttggtgtgt tggtgtttag tgaggatgtg tagattgttt taggtttggt gttgttgtat 840
tgggtatgtg ttgatttggt tgggtgggaa tattttgttt tgtttgggtt ttgttttagt 900
tttgtttttg tgtgttttgg ttttgttttt gtgtgttttt ttgttttttt taggtttttg 960
gttttgtttt gttttagatt ttgttttatg ttgttatttt tgtgtttttt ggttttgttt 1020
ttgtgttttg gatatgttgg gatagtttgt gtttttagaa tgttttgtgt tttgatgttt 1080
gtaggttttt gtggtgtgta ttgtttgtga tttggtgagt gtttgggttg ttttgttttt 1140
ggaagagtgt ggagttt 1157

<210> 322
<211> 1157
<212> DNA
<213> Artificial Sequence

<220>
<223> chemically treated genomic DNA (Homo sapiens)

<400> 322

gagttttgta tttttttggg agtgaggtga tttagatatt tattaagttg taaatggtgt 60
gtattgtgag gatttgtggg tgttgggatg taaagtgttt taggggtgtg ggttgtttta 120
gtatatttgg ggtgtggggg tggggttgag gggtatgggg atggtggtgt ggggtggggt 180
ttagagtggg gtggagttga ggatttgaga aaagtaagag agtgtgtggg ggtggggttg 240
gggtgtgtgg gggtggagtt ggggtggagt ttgggtgggg tggggtgttt ttgtttgatt 300
gggttggtgt atgtttagtg tggtggtgtt gggtttgagg tagtttgtgt atttttgttg 360
gatgttggta tgttggtttt ggttttttgg tagtgttgtt gttttgtgtg tttgatttgg 420
atggtttttt ggttggtata agttgggtgg tgttttttta gtttttgttt gaggttttgt 480
gttttagttt gttaaatggt ttgtattttt ttttgttata gggatagttt gtgggtgtgt 540
aagtgatttg ttatgtgttt gagtggtttt gaaagtgtgt gggggttgta ggatggtgtt 600
tgtttagtga gaattttggt tttgtttggg gtggttatgt tgagtttgga gtgtgagttt 660
tttttttttt ggggttgttt ttttagttag ggatttttag attagtagag attttgttag 720
ggggttgtgg gggtagtttt tagggtttgt ggtgggtgat gttgtttagg ggttgtagtt 780
attgatggtt tagatattgt tagagtttga tttagttgtt ttttagagag tatatagggt 840
gttaggtttt gtttggggtt gtgtggatgt tattaggagt tgaattgtgt ttattagaga 900
agttatgttg gagttttaat ttttagtatt ttataatgtg attttatttg gagatgggga 960
ttttatagaa gttattaatt agtgggtttt ttatttagta tgatttgtgt ttttataggt 1020
agtaggttgt ttggggagga ggattggagg gttgtatttg taagttatta gaagtttaag 1080
aggatagatt tttttttgga gtttttggag ggagtagagg atattttttt ttggagtttt 1140
tagagggagt aggattt 1157

<210> 323
<211> 8093
<212> DNA
<213> Artificial Sequence

<220>
<223> chemically treated genomic DNA (Homo sapiens)

<400> 323

ttatggaagg aaatgagatt agttagtgag ttgatataga ataaggtagt ttaaaataaa 60
tttttttaaa aatatgaaga atagatattg aagggaagag gtgtttgtaa agattaagaa 120
agtatatttg gagatggtat ttttttaaag ttaaagttat taagtgttta agtgttttaa 180
ggagggtaag attattaata aggatttagt atagtagagt aatttgagtg gtaatatggg 240
atattgggaa tataaatttg ttaagaaaat tagtaggaat gagttatagg atagtaattg 300
gtaaggattt aataattttt tttttaatgt atgtatttta attatattta ttgttataat 360
aggattagta ataattatat ttatttaaaa aaaaaaagat tgttatgtag ttatagaatt 420
atttaatata gaaaatagta aaatatattt tttttttttt tttttttttt tttttttttt 480
ataaataaga ttagtttata gtaaattttt tatagttaag ggttaattta aaatttttgg 540
tttatatttt ttttttattt aatgattata tgatgtaaat taattttatt aatattttaa 600
gtaaaatata ttggaatttt ataaaatgta taagatttta atatttaagg aattggggtt 660
agaaagtaga agtggttttt aggtttttta gttttttttt taagtaataa agttttgttg 720
tgaatattta aagttattgg gaaattattg gtagattttt ttgttttttt tttttggttt 780
tttattatgt tgttttttta gatatgtaag tttattttat taattaaaat tttagtttga 840
ttatttttga taagtattta attgatatgt aatttttttt ttgttttaaa tatgtataat 900
aggatagagt ttttaaattt gatttaatta ttaatttttg atttataagt gttatggtga 960
gttaatagaa tttattaatg tttttattgt attttattag ttaaatttag aaggttggaa 1020
ttagtttttt ttatttagta ttttgttagt ttgtttagtt tgtattttta attttgtttt 1080
taatggtaat ttgttttatt ttttgaaata aaataattta tattttggga gggttaattt 1140
tttattgtgt taggttgttt tatgtattgt aggggtgagt gtttttaggt ttaaatgtta 1200
atagaagttt ttagtaaata tgataattaa aaaagtgttt ttatatattt tttagtattt 1260
tttggaatga taggttattg tttttagttg aaagttattg gtataatttt ggtttttaag 1320
tttttatgtt atttatttta attgtttaga tattaatttt atttaaattt ttagttatag 1380
tttggttttt tgaatttgtt ggattagtaa ttatagatta aggtgtttta atagttaaga 1440
taggattttg gaataagagt ttttaaattg tataatattt gagaggattt atgaatataa 1500
attgggtttt gtttataatt agttttatat aatgaatttt aagattgttt ttttatggtg 1560
aatagaagtt tggaaattat tgttttggta taaagtagat tattttagta gaaatataga 1620
attattgtaa tttgtgaata agattgtttt taaatatttt tatttgtgtg ttattttata 1680
tatagaatgt gtatgatagt tttaaatttt agaataaatt tatttttagt atttaataaa 1740
atttgatatt gtattatttt aaaataaagt gtttatttta ggtaggattt tttaaaataa 1800
agtagtaata tttatgtaga taagataaaa aagttaaaat attttatatt ttttaatttt 1860
ggagtgtaat tttttttttt tattgttttt gatagggtta aatttgtgtt tatagtaaaa 1920
aaaaaaaaaa aaagaattat taattggtaa ttattaagat tttatattta ttatagtttt 1980
ttaataggta agttgataaa atagttttta gttattaaaa aaaaaattta aggaaaattt 2040
ttaataatta gttttatttt taaattgtat gaagtttttt atatttgaaa tttaaaaatg 2100
attttaatga ttttttttat tagtaatgtg ttattattga ggtgggtgat ggggagggaa 2160
gagggaagaa atttgttagt attatttttg aatttagaaa tgtttaaaaa aaagttttaa 2220
atattttgat ggttagataa aatattttta ttgttatgta tgggtttttt ttttggaaat 2280
ttatgaattt gttatgtgag tttttgtaaa ttggtttaaa agtatattta aggagttgat 2340
aatttaagat tatatgaatt agaattttaa tattttatta aaataagagt tgaaattttt 2400
ggtatttatt tttagaatat ttaaaaaata gattgtaaat ttaatttata ttaatattaa 2460
atttttttaa aattaattat attataaaag ataatgattt tgttattaaa ttaagtttta 2520
aaaaaggtgg tatttttatg ttttagtttt atgttgttat ttgagatgaa aaaaaaggta 2580
attgttagaa ttttaattgt gattagtttg gatggttggt atttggtatt tttttataat 2640
ttaaattagt aaatttgaaa atttaaatta atttttgttt ttagaattaa atttttgttg 2700
agttttttaa ttttgtatta taaagtttta tggattagat ttattttttt tttttataag 2760
gttattttaa ttgggtttta ttttagttgt ggtaatttga aatagggttt ggagatgttt 2820
atttgttaat ggttgattta aagtgtgttg gttttgagtt aaatatgaaa aagttttttt 2880
atagatttgg ttattattaa ataaggatgt tattttatag gtaatttttt tgataattgg 2940
gtaggattta gtagataatt tagtataaaa ttgttttaag tttttgattt tgtatttatt 3000
ttaaagatta gtgtttgttt gaagttttgt tagttttatt tttattgaaa taggtgtata 3060
tttaggttta aagttaaatg gatttgaaaa aagttttaag ttttttttaa tgaatttgag 3120
gtttttaaaa tgttttttaa attttttttg gagatgatag attattattt gatatttatt 3180
aggattaaat atttttttat tttttttatt ttgttgtttt agtttattaa taattttttt 3240
gagggtagga aagtttgtta gatttttttt tttaatatgt ttttgaaata aatttagttt 3300
aattttgaaa gtataaatat ggttaaaggt agtagtagta aagattttat taatttgtaa 3360
ttttttattg agtttttgaa ggtgttttat aatgtttatt aagaagttaa agttataaag 3420
ttattgtttg tttgagaagt ggattgttgt tgtttttatt gaaattaaga atgtttttat 3480
tttttttttt agagagaata ttagttttaa agtttttttt tttttaagtt aattgtttag 3540
atattgttta ttaatttttt tattatgttt tgatttagat tttgttagta aagtttgaaa 3600
tttaggttgt ttaatgtttt tggttttaat taaaattatt tatttggata tggtatttat 3660
gatttgatta atatttatat tataggagtt taatagtttt aagtgaagaa attttgaata 3720
atgaatgata ttttaatagt tggggtttat ggtttttttt tttatgtatg agatgagttt 3780

tgagttttta ttaattattt ttaaagtatg ggttgtggtt agtttaatta ttttttgtaa 3840
gttaagtttt gttgtttgta gggattttag gattgttgat atgagtgtat taatattgaa 3900
atgatgagtt aggttgatta tggttagaag atttttttgt atttttaatt tagggtttat 3960
attgtggata aagattagga ggtagttttt ataggttata aaagtttggt tgtttaaggt 4020
aagagaatag gttttaaagt ttttggtttg gttaaaaagt tggttgtgta gatttttgtt 4080
aatgtttagg attttttgtt ttgtgatatt tggagataag ttaatgtttt gtaggatgtt 4140
tatatgtttg ggtagtattt tttttagtaa tatttttatg tattggtata taaagttttt 4200
tttattttag ttgtgatttt ttaaggttaa gaggtggtag agtttgaggt ttgtatgagt 4260
agtttttttt ttaggagtga aggaggttat gggtaagttg ttttgatgta gatgttttat 4320
tagggttgtg tgtttgttgt ttgttatata ttgtttgtag tatttgtgtt tagttttgta 4380
gtggtgtttg atgttgtgtt tgtgggtagt tatgatgagg tggtgataga ttaggtatag 4440
ggttttattg ttttttggag gttttattat taaataatgt tgggtttatt tgggttggaa 4500
aattagagtt ttgttgattt ttattttgtt ttttttgggt gttatttata ttttgtggga 4560
ggttataaga gtagggttaa tgttagaaag gttgtaaggg gagaggagga gtttgagaag 4620
tgttaagtat tttttttgtt ttgtgttaga ttattttagt agaggtatat aagtttggtt 4680
ttggtatttt tgtttttatt ggttggatat tttgtatttt ttgagttttt aaaaatgaat 4740
taataggaag agtggatagt gatttttaat gtgtaagtgt atattttttt aggtagtggg 4800
tagtagttgt tttagggagg gatgaagaga tttagtaatt tatagagttg agaaatttga 4860
ttggtattta agttgtttaa ttaatagttg ttgttgaagg gtggggttgg atggtgtaag 4920
ttatagttga aggaagaatg tgagtatgag gtattgaggt gattggttga aggtattttt 4980
gttgagtatt tagatgtttt tttggttttt ttggtgttaa aatgttgttt gtggtagggg 5040
ttatttggtg gttggatgag atagtggtga attgtattgt ggtgggggaa gttatttagt 5100
ggttagttaa tgttattaaa gagatgattg agaattggta tggagggagt tgagttgggt 5160
ttatttaagg gttatgattt aatgggttgt gttatttaat ggtgtggata tgtttttttg 5220
tttgggtaga ggtatgtata gtgtatgttt ataatggtgg aggttgttgg gttttttgat 5280
gtgttagtta ggtttttttt ttttttgtag attgtgtgtt tttttattgt ttttttttga 5340
gattttttaa gggttgtttg gagtgtaagt ggaggaatat atgtagtgtt gttttaatgg 5400
tattgttaat taagtaagga agttatttaa tttaaaatta tgtatgtaga atatgtgaag 5460
ttaaaagatg tataaaagtt taagatgggg agaaaaattt ttttttagag ggtattgtgt 5520
tattgttttt ttgtttttta tttattttag aaattatttg tttatattta aaggtataat 5580
ttattttgag ttttttttaa aataaattgt ttgtagtttt aggataggtt gatgtatttt 5640
gggtttgatt tttgattatt ttattgttaa attagtgatt ttttttagtg tttttttgtt 5700
ttttattttg gaggatgtat tttgaagata ttagatttta tgggttattt ttggattttg 5760
aagtttataa ttgtgataaa aaatgtgaag ggaagagatt tgaaatatgt ggaaggaaaa 5820
gtgagtgtag attataaatt tttaaaaaga taagtttaaa atatatttaa atgttatgtt 5880
agattatttt gttaaaatta gttgttagtg atgtttgtat gttaatagaa aaaagaatgt 5940
tttagtttgg agtggtaggt ttttagaggg atttattgtg aaagtataaa ttatttaggg 6000
taatggattt gagagaatag tgggtagaaa ggattattga aggaaaggaa gagaattgga 6060
aggtagatga aagaaggagt aagaatttgg ggatgttttt ttttttttat ttgtaatagt 6120
agtaatagaa gtaatggtag attggttttt gtttttattg tgttagaatg aattgatagg 6180
ataattgggt ttattattgt attgtgttag aatattgtaa aataaaatta aatatattag 6240
tttggtggtt tgtaattaat tatttaagtg gagattttta tttttttttt attttttttt 6300
tagatggagt tttattttgt tatttaggtt ggagtgtagt ggtgtgattt tagttgattg 6360
taattttttt tttataggtt taagggagat ttttttgttt tagttttttg agtagttagg 6420
attataggta tgtgttatta tatttggtta attttgtatt tttagtagag atgggatttt 6480
tttatgttgg ttaggttggt gttaaaattt ttgattttag gtgaattgtt tgttttagtt 6540
ttttaaagtg ttgggattat aggtgtgagt tattgtgttt tgtagttttt tgtattttta 6600
atagagatag ggttttatta tgttagttag gatggttttg attttttgat tttaggtgat 6660
ttgtttgttt tggtttttta aagtgttggg attataagta tgagttattg tgtttggttt 6720
aagtggagat ttttatatgg agtttagtta tatttttttt aatatataag ttgagatgat 6780
taatataatt ttaatatagg ggtttatgag aaatgtttgt aatatttaag taatttattg 6840
tttttttttt ttttttttta agatgaagtt ttattttgtt gtttaggtgg aagtgtagtg 6900
gtgtgatttt ggtttagggt aatttttgtt ttttggtttt aagtgatttt tttgttttag 6960
ttttttgagt agttgggagt ataggtgtgt atgattatat ttggttaatt tttttatttt 7020
tagtagagat ggggtttttt tatgttggtt gggttggttt tgaatttttg attttaggtg 7080
atttgtttat tttagttttt ttaagtgttg ggattatagg tgtgagtttt tgtgtttagt 7140
ttattatttt atttttgaat aaagaattgt ttgtgtgggg aatagataat ttttttttat 7200
gtagtttttg ttagaaaatt tgtttttttt agtagttggt ttgtgtttat aggttatttt 7260
ttgaaagtat aaaaaattta ttgatttttt ttttttgggt tttttttttt ttttgagata 7320
gagttttgtt tttgttgttt aggttggagt gtaatggtgt gattttagtt tattgtaatt 7380
tttatttttt gggtttaagt gatttttttg ttttagtttt ttgagtagtt gggattatag 7440
gtatgtgtta ttatgtttgg ttaattttgt atttttagta taaatggggt ttttttatgt 7500
tggttaggtt ggttttaaat ttttgatttt aggtgattta tttgttttgg ttttttaaag 7560

tgttgggatt atgggtgtga gttattgtgt ttggttagaa aatttattga ttttttaaag 7620
atttattaat tttttgtatt attttttttt ttttttttta ttgtaaatat aaaagggaat 7680
agtagagaaa attatttaga attttatttt ttagtgatat tatttagtga tattttatta 7740
gagttattta ggaatttgag gttgaataaa gtttaggtaa aagtaaaatt agttgagaag 7800
agatatttgt taaaagaaat ttatttttaa ttttatttgt tgtttttttt agaggaatag 7860
aaatagtgtt gaatgtttta ttagaaatga tggttgtttt gtttgttttt tttttttttt 7920
ttatataata tgtaaattta tatagtgtat gagtttgtaa gataaaggaa aaatatgtta 7980
atgaggtatt attgtttgta tttggagttt gttattattg tttggtttat attaaaatat 8040
gtatattaga gtagttgtag attgataaat tattttttgt ttgatttgtt agt 8093

<210> 324
<211> 8093
<212> DNA
<213> Artificial Sequence

<220>
<223> chemically treated genomic DNA (Homo sapiens)

<400> 324

attggtaaat taaatagaaa ataatttatt agtttgtaat tattttaatg tatatatttt 60
aatatgagtt aagtaatgat aataaatttt aaatataaat aatagtgttt tattaatgtg 120
tttttttttt gttttatagg tttatatatt gtatgagttt atatattgtg tgagagagag 180
ggaagagatg ggtagagtaa ttattatttt taataggata tttagtatta tttttgtttt 240
tttaggaaag atagtaagtg aagttaaaaa tagatttttt ttggtagatg ttttttttta 300
attaatttta tttttatttg aattttattt agttttaggt ttttaagtga ttttaataaa 360
atgttattaa ataatgttat taaaaaataa aattttgaat gatttttttt attatttttt 420
tttatattta tgatggaggg gaaaaaaaaa agtaatgtag aaagttaata aatttttagg 480
aagttaatga attttttggt taggtgtaat ggtttatatt tataatttta gtattttggg 540
aggttaaggt gggtggatta tttgaggtta ggagtttgag attagtttga ttaatatgga 600
gaaattttat ttgtattaaa aatataaaat tagttaggta tggtgatgta tgtttgtaat 660
tttagttatt taggaggtta aggtaggaga attatttgaa tttaggaggt ggaggttgtg 720
gtgagttgag attgtgttat tgtattttaa tttgggtaat aagggtaaaa ttttgtttta 780
aaaaaaaaaa aaaatttaaa aaaaagaagt taataaattt tttgtgtttt taaagagtag 840
tttataagta tagattaatt gttagagaaa ataaattttt tagtaggggt tgtatgagaa 900
agagttattt attttttata tagataattt tttatttaga aataagataa taggttgggt 960
atgggggttt atatttgtaa ttttaatatt tggggaggtt gaggtgggtg gattatttga 1020
ggttaggagt ttaagattag tttggttaat atggagaaat tttgttttta ttaaaaataa 1080
aaaaattagt tgggtgtggt tatgtatgtt tgtattttta gttatttggg aggttgaggt 1140
aggaagattg tttgaaatta ggaggtagag gttgttttga gttaagatta tgttattgta 1200
tttttgtttg ggtaatagag taagatttta ttttaaaaaa aaaagaaaga agataataag 1260
ttatttaaat attatagata ttttttatga gtttttgtat tgaagttgta ttagttattt 1320
taatttatat attaaaaaga gtataattgg attttatata aaaattttta tttgagttgg 1380
gtgtggtggt ttatgtttgt aattttagta ttttgggagg ttaaagtggg tagattattt 1440
gaggttagga aattgagatt attttggtta atatggtgaa attttgtttt tattaaaaat 1500
ataaaaaatt gtagggtgtg gtggtttatg tttgtaattt tagtattttg gaaggttgag 1560
gtaggtggtt tatttgagat tgagagtttt gatattagtt tgattaatat ggagaaattt 1620
tatttttatt aaaaatataa aattagttag gtgtggtggt atatgtttat agttttagtt 1680
atttgggagg ttgaggtagg agaatttttt ttgaatttgt gaggaggagg ttgtagttag 1740
ttgagattgt gttattgtat tttagtttgg gtgataaagt gagattttgt ttaaaaaaaa 1800
aataaaaaaa aaataaaaat ttttatttaa gtaattaatt ataagttatt aagttagtat 1860
gtttagtttt gttttatagt attttggtat agtataataa taggtttagt tgttttgtta 1920
atttatttta atatagtaga aataaaagtt agtttgttat tgtttttgtt attattatta 1980
taagtgaaaa ggaaaaaata tttttaggtt tttgtttttt tttttattta ttttttaatt 2040
tttttttttt tttttagtgg ttttttttat ttattgtttt tttagtttta ttgttttgaa 2100
tagtttatat ttttataata aattttttta gaaatttatt attttaaatt gaagtatttt 2160
tttttttatt aatgtatgga tgttattaat aattgatttt aataaaataa tttgatataa 2220
tgtttaggtg tattttgggt ttgttttttt ggaagtttat agtttgtatt tatttttttt 2280
tttatatgtt ttaaattttt tttttttata tttttgttta tagttataag ttttaaaatt 2340
taaggatgat ttataaaatt tggtattttt gaagtgtgtt ttttgaaata aaggatagga 2400
aaatattaag aggggttatt aatttaataa tagggtaatt agaagttaag tttaaagtgt 2460
attagtttgt tttaaaatta taaatgattt gttttgaaag aaatttaaaa tgaattgtgt 2520
ttttggatgt gaatagatga tttttggaat gaatgaaaag taagaaaata gtaatatagt 2580

attttttgaa aaaaggtttt tttttttatt ttaagttttt atatattttt taattttgta 2640
tgttttgtat atataatttt aaattaagtg gtttttttat ttagttaatg gtattattaa 2700
gataatatta tgtatatttt tttatttata ttttaaataa tttttaaaag gttttggggg 2760
agagtggtaa agaaatatat ggtttgtgga aaaggagaag gtttgattgg tatgttaggg 2820
aatttggtgg tttttgttgt tgtgggtatg tgttgtatat gtttttgttt gggtaaagag 2880
gtgtgtttgt gttattgagt gatgtggttt gttaagttgt agtttttaag tgagtttggt 2940
ttgatttttt ttgtattagt ttttaattat ttttttgata gtattagttg gttgttggat 3000
aatttttttt gttgtgatgt ggtttattat tgttttgttt agttgttgaa taatttttgt 3060
tatgaatgat attttggtgt tagaagagtt aaggaaatgt ttagatgttt aatggaagtg 3120
tttttagtta attattttag tgttttgtgt ttatgttttt tttttagttg tagtttatgt 3180
tatttagttt tattttttag tggtagttat tgattggata gtttgaatgt tagttaaatt 3240
ttttaatttt gtgggttgtt gggttttttt gttttttttt gaagtggtta ttgtttgtta 3300
tttagaagga tatgtgtttg tgtgttagag attgttgttt gtttttttta ttggtttgtt 3360
tttaggagtt tggggaatat gaaatattta gttaatagga gtagagatgt tggaattggg 3420
tttgtgtgtt tttgttgagg tgatttggtg tagagtggag gaggtgtttg gtgtttttta 3480
ggtttttttt ttttttttgt ggttttttta atgttggttt tgtttttgtg gttttttgta 3540
gaatgtggat gatgtttaaa agaagtaaga tggaagttga tgaggtttta gttttttggt 3600
ttgagtggat ttagtgttat ttggtggtgg agtttttgga gggtgatggg gttttgtgtt 3660
tggtttgttg ttgttttatt gtagttattt gtgaatgtga tgttaggtgt tattatgagg 3720
ttgagtatga atattatgag tggtatgtgg tggatggtga gtgtgtggtt ttggtggagt 3780
gtttgtgtta gggtgatttg tttgtggttt tttttatttt tgaagagaga gttgtttgtg 3840
taggttttgg gttttgttgt tttttggttt tgaagggttg tggttggggt gagggggatt 3900
ttgtatatta gtgtatggag gtgttgttga gagaggtatt gtttgagtat gtaagtgttt 3960
tgtaaggtgt tgatttattt ttagatatta taaggtagag gattttgagt attgatagga 4020
atttatgtaa ttagtttttt aattgagtta gggattttaa agtttatttt tttgttttgg 4080
atgattaggt ttttgtggtt tatgagaatt attttttggt ttttatttgt ggtgtaggtt 4140
ttgagttgga ggtgtaagaa gattttttga ttataattaa tttgatttat tattttagtg 4200
ttggtgtgtt tatgttggta attttagagt ttttgtagat agtagggttt agtttgtaga 4260
gaatggttgg attgattatg atttatattt tgaggatgat tggtgagaat ttaggatttg 4320
ttttatatat gagagaaaag gttgtaagtt ttaattgttg gaatgttatt tattatttag 4380
gattttttta tttggaattg ttgagttttt atgatgtaga tgttaattag attataaata 4440
ttatatttga atggatagtt ttgattaaga ttagaggtgt taggtgattt gaattttaga 4500
ttttattaat ggaatttgaa ttagagtatg gtgaaagggt taatggatga tgtttgaata 4560
attggtttag gagagggaaa attttaaaat taatattttt tttaagaaaa gaaatggaag 4620
tgtttttggt tttagtaggg gtaataatag tttatttttt agataaataa tggttttgtg 4680
attttggttt tttggtggat attatggaat atttttgaga atttagtgaa gaattatgag 4740
ttagtaaagt ttttgttgtt gttgtttttg attatatttg tatttttgaa gttaagttga 4800
atttatttta aagatatatt gaggaaaaaa atttaataga tttttttgtt tttagagaag 4860
ttgttgatga gttaaaatag taaaataagg aagatgaaaa aatatttgat tttgataggt 4920
attaaatggt gatttgttgt ttttaaaaag aatttgagag atattttaag gattttaggt 4980
ttattaaaaa ggatttagaa ttttttttaa atttatttaa ttttaaattt gaatatgtat 5040
ttattttagt gagggtggag ttaataaaat tttaggtaaa tattaatttt tggaatgaat 5100
atagaattaa agatttgggg tagttttatg ttggattgtt tgttgaattt tatttaatta 5160
ttaaaggggt tgtttgtaag gtggtatttt tgtttgatag taattaaatt tgtgaaaagg 5220
tttttttata tttgatttga aattaatata ttttgagtta gttattaata gatgagtatt 5280
tttaagtttt gttttgggtt gttataattg aaatggagtt tggttgggat gattttgtga 5340
gagaaagaaa tgaatttaat ttataaggtt ttgtagtata agattgaaaa atttaataag 5400
aatttaattt taaaagtaaa aattggtttg agtttttaag tttattaatt tggattgtga 5460
gaaagtatta agtattagtt gtttaaattg attataatta aaattttgat agttgttttt 5520
ttttttattt taaatggtag tatgggattg aaatatgaga atgttatttt ttttaaaatt 5580
tagtttagtg ataaagttat tgttttttat gatatagtta attttaaaga gatttagtat 5640
taatgtgagt tgaatttgta gtttgttttt taggtgtttt gaagataaat gttaaaaatt 5700
ttagttttta ttttaatgga gtgttaaaat tttgatttat atagttttaa attattaatt 5760
ttttaaatgt gtttttgaat taatttgtag aagtttatat agtaagttta taagttttta 5820
aaaaggaagt ttatatataa tagtggaggt gttttgttta attattaaaa tgtttgagat 5880
ttttttttaa atatttttga gtttgaaggt aatattgata gatttttttt tttttttttt 5940
tttattattt attttagtga taatatatta ttgatagagg aagttattag aattattttt 6000
aagttttaga tataggagat tttatgtaat ttggagataa gattaattat tgggggtttt 6060
ttttggattt tttttttaat aattgggggt tattttatta gtttgtttat taaaggatta 6120
tggtaagtat agaattttaa tggttgttag ttagtaattt tttttttttt tttttttatt 6180
gtagatataa gtttggtttt attaaaaatg atgaggaaaa aagattgtat tttaggatta 6240
ggaggtgtga gatattttag tttttttgtt ttatttgtgt gggtattgtt gttttatttt 6300
aaaaaatttt gtttaaagta aatattttgt tttaaaatga tatagtatta gattttgtta 6360

gatgttagaa atggatttat tttaaaattt ggaattgttg tatatatttt atatgtaaga 6420
tagtatataa gtagaaatat ttaaaagtag ttttatttat agattgtagt aattttgtat 6480
ttttattaag ataatttgtt ttgtgttaaa atagtaattt ttaaattttt gtttattatg 6540
aaaaggtaat tttaaagttt attatgtaaa attaattata aataggattt aatttatatt 6600
tatagatttt tttaagtatt atataattta aaaatttttg ttttaaagtt ttgttttaat 6660
tattgaaata ttttaatttg tggttattaa tttagtaaat ttaaggaatt aggttatgat 6720
taagaattta ggtggaattg atgtttgggt aattaaaata aatggtataa gagtttaaaa 6780
attaaagttg tgttagtggt ttttaattag aggtagtaat ttgttatttt agaggatgtt 6840
gagaaatgtg taggggtatt tttttggttg ttatatttat taggggtttt tgttggtatt 6900
taagtttaaa gatatttatt tttgtagtgt atgggatagt ttggtataat gaagaattag 6960
tttttttaaa atgtagatta ttttatttta agggataggg tagattatta ttagaagtaa 7020
aattaaaagt ataagttggg taaattgata gaatattaga taggagagat taattttaat 7080
tttttaaatt tggttagtaa agtgtaataa aggtattgat aagttttgtt agtttattat 7140
agtatttgta aattaggaat taataattga attagattta agggttttgt tttgttatat 7200
atatttaagg tagaaaaaaa gttatatgtt gattaggtat ttattaagaa tggttaagtt 7260
gagattttgg ttaatagagt aagtttatat atttagagaa ataatatagt ggaaaattga 7320
aaaaaaaaaa tagaaaaatt tattggtaat tttttaatag ttttgaatat ttatagtaga 7380
gttttattat ttgagagaaa gattggaaga tttgaaagtt atttttgttt tttaatttta 7440
gttttttaaa tattgaaatt ttgtatattt tgtgaaattt tagtatgttt tgtttaaggt 7500
gttaataaaa ttagtttgta ttatgtagtt attgagtgag ggggagatat aagttaagga 7560
ttttaaattg atttttagtt atagagaatt tgttataagt tagttttgtt tgtaaaaaaa 7620
aaaaaaaaaa aagaaaaaga aaaaagtgta ttttattgtt ttttgtatta agtaattttg 7680
taattgtatg gtagtttttt tttttttaaa taaatatagt tgttattggt tttgttgtag 7740
tagtgaatat agttaaaata tgtatattaa aaaaaaaatt attaggtttt tattagttat 7800
tgttttatag tttattttta ttagtttttt tgatagattt gtatttttag tgttttgtat 7860
tgttatttaa attgttttat tatgttaagt ttttgttaat agttttattt tttttgaaat 7920
atttgaatat ttgatgattt tagttttgag gagatattat ttttaggtgt gtttttttag 7980
tttttgtagg tatttttttt ttttaatatt tgttttttgt atttttaaaa aaatttgttt 8040
tagattgttt tgttttgtgt tagtttgtta gttgatttta ttttttttta tgg 8093

<210> 325
<211> 6521
<212> DNA
<213> Artificial Sequence

<220>
<223> chemically treated genomic DNA (Homo sapiens)

<400> 325

ataaaattat gtatgggtaa aagatttaat taggtgtagt ttagaaaaat ggattttttt 60
tttttttttg agatagagtt ttgttttgtt atttaagttg gagtgtagtg gtgtaatttt 120
ggttaattgt aatttttgtt ttgtaggttt aagtgatttt tttgttttag ttttttgagt 180
ggttgggatt ataggtgtgt gttattatta tatttgatta atttttgtag ttttagtaga 240
gatgggaggg ttttattatg ttggttaggt tgtttttaaa tttttgattt taggtgattt 300
atttatttta gtattttaaa gtgttgggat tataggtgtg agttattatg tttagattta 360
aatttttttt gttttttttt tttttttggg ggtggggggt agggttttat tttgttgttt 420
aggttggagt gtagaggttt gattttggtt tattgaaatt tttatttttt aggtttaagt 480
aattttttta ttttagtttt ttgagtagtt gggattatag gtgtgtatta ttatatttgg 540
ttaatttttt gtatttttgg taaagatggg gttttgttat gttgtttaag ttgtttttga 600
atttttgggt ttaagtgatt tttttgtttt atttttttaa tgtgttggaa ttgtaggtat 660
gagttattat gtttggaata aagttttatt ttttaagtgg attatttaaa aaaatgtttt 720
atggttaggt atgttatttt tttttagttt gggtaagtga gttgagattg tgttattgtg 780
gtttggtttg agtgatagag ggagatttta ttttagataa ataaataaat aaaattaaaa 840
taaatttaaa tattttttat atgataaaat tatttttaat ggtaattata aaatgaataa 900
taagggttaa gaaataaatt tagtgaatat tttttttttt tgaattttgt aaatataatt 960
atgttagtat aaaaatagtt atgtattgtt taataatagg gatatgtttt gagaaatgag 1020
ttattaggta attttgttat tgtgtaaata ttatagagtg tatttttata aatttagatg 1080
gtgtagttta ttatttattt aggttatatg gtatagtttg gtgtttttag gttttaatgt 1140
ttatagtatg ttattatatt gaatattgta ggtagttgta agataatggt atttgtgtat 1200
ttattgtaga gatagggttt ttttgtttgt tgtttaggtt ggagtgtagt ggtataatta 1260
tagttttttg tagtttttaa tttttgggtt taagtgattt ttttatttta gttttttaag 1320
tagttaggat tatatgtttg tgttattagg tttgtttatt gtttttgttt gttttttggt 1380

agagatggag ggttttatta tattgtttag attggttttg aatttttggt tttaagtaat 1440
ttttttgttg ttttggtttt taaagtgttg gtattatagg aatgagttaa aagtttaata 1500
tggttgttga ataaaagatt gatttaatta agtatggatt ttgtttttat ggagttgaag 1560
gttgttagag tagataaaat gagttattat ttttatatga gttagatggt ggataaagtt 1620
attattgagg gtaggtaaag aattgggttg gtttaaagat gttagggatt ttttttagat 1680
tagtggtaga gggttggtta aggagttttt tgaaggaggt ggttttttat ttgagtttta 1740
aagaatagaa gagattagat agggaggata gtaaaatttt tttttttttt tgaaatagtt 1800
ttgttttgtt gtttaggttg gagtgtagtg gtgtgatttt ggtttattat aagttttgtt 1860
ttttgggttt atgttatttt tttgttttag ttttttgagt agttgggatt ataggtggtt 1920
gttattatgt ttggttaatt tttttttttt ttttttttta gtagagatgg ggttttatta 1980
tgttagtgat gatttttgtt tgtttgtttg tttttgagat agagttttat tttgttgatt 2040
aggttggagt gtagtggtgt gattttggtt tattgtaatt tttgtttttt aggtttaagt 2100
gatttttttg ttttagtttt ttgagtagtt gagattatag gtgtttgtta ttatgtttag 2160
ttaatttttg tatttttagt agagatgggg ttttattatg ttggttaggt tggttttgaa 2220
tttttgattt taggtgattt gtatattttg gttttttaaa gtgttggtat tagaggtatg 2280
agttattgta tttggttagt aaaattattt gtgatatagt agtaagtgta taagtattga 2340
tttattagag ttgaaggtgg ttgaggtatt taagtgggtt tttggggatt ttgttatgtt 2400
agggaatatt tttttagata agggattagg aggtttggag ggtttttaag gaagagttag 2460
ggtagtaggg gtattttgga gttaggggtt atggttatat gttaatagga ttagaaatat 2520
tttaatgtat ttaattaatt tatataatta tattggtggg tgtttattga atattagttt 2580
tagatatgga tttgaaatgg ttttattttt ttttagtttg tttaagttaa tggagtttta 2640
ttagaatgtg agttataaaa tagtagagtt attttttatt taatgttgta tttgtagggt 2700
ttagaatagt atttgaagga ttggtggagg tttaataaat tgttaaaagg gtagattagt 2760
taggtgtgat ggtgtgtgtt tgtagtttta gttatttggg aggttgaggt aggaggatta 2820
tttgatttta ggatttttgg gttgtagtgt agtatattag ttaggtgttt gtattaagtt 2880
ttgtattaat ttggttgttt aaggatggtt gaattgattt aggtggaaat tggagtaggt 2940
taaaatttaa tgttgattag taatagggtt gtatttgtaa atagttattg ttttttagtt 3000
tgggtaatat agtgagattt tatttttaaa ataaatttta aaaataatta attagaaaaa 3060
aaaattagtt tgtaatttta gtattttggg aggttaaggt gggtagatta tttgaggtta 3120
ggagtttaag ataagtttgg ttaatatggt aaaattttaa tttttattaa aaatataaaa 3180
attagttggg tgtggtggta ggtgtttgta gttttagtta tttaggaggt tgaggtaaga 3240
gaattgtttg aatttgggag gtggagattg tagtgggttg agattgtgtt attttatttt 3300
agtttgggta gtagagtgtg attttgtttt aaaaaaaaaa aaaaagaaag aaaagaaaag 3360
aaaaagaaaa gaaaagaaaa aaattgggag gtttaagttt atttttggtg tttttatatt 3420
tttttgtttt atttttttgt atttagtttt tttttgtaat ttgtgtttta tattagtttt 3480
taagttttta atgtgattta gtatgagaat tggattttgt tatttttttg tttataatat 3540
tttatgtttt ttttttgttt agaatattat tttttttatt gtttttatta atggaattgg 3600
tattttttta aggatatgat taaatttgtt tatttttata ttatttttta aagtagaatt 3660
tatttttttt tttttaatat gatgatattg atagggtttg tttttattta ttagattgtg 3720
agttgtttag ggtaggtagt gttttttgtt tttgtttttg tttttttttt ttgagatagg 3780
gttttgtttt gttatttagg ttagagtgta atggtatagt tttagtttat tgtagtttta 3840
attgttttgg tttaaattat tattttattt tagttttttg agtagttggg attataggta 3900
tatgttatta tatttggtta atttttttgt atttttagta gagatagggt ttggttatgt 3960
tgtttgggtt ggttttgaat ttttggattt aagtaattta tttattttag ttttttaaaa 4020
tgagggattg tgttttattt atttttatgt ttttagttta tagtttagtg ttggatttat 4080
ggtagtatta aataaatatt tgttgaatgt aatagtaaat agtattttag ggagtaagaa 4140
ttagattaat aaaggtggta aaaggtttgg agaaaaaaat aatagtttaa tttggttaga 4200
gtatgaggga gagtagtagg agataagatg gaaaggtttt ttgggtaagg ttttgaagga 4260
agttggaagt tagaagtata taatgtgtat attgtggtag gtagtgggga gttaatgaag 4320
gtttttgagt aggagagtaa tgtgttgaaa aataaatata ggttaaattt attagagttt 4380
ttttgatata tatatttgtt ttttatttaa gtttaagttt gttttttata tatttattat 4440
ttaatttatt tttgggtttt tttagtagtt tgttttattt ttttatttgt tttttggtgg 4500
agttagggat gtatatatga gttgtttttt tttttagtta gaggatatgg gggttttagt 4560
ttttttgttt tttttttttt gtgtttggag ttgggaagta ggttagggtt agttgaggtt 4620
ggttggtaag tagttgggtg gtgttaggga gagtttgtat agtgttaggt ggtgttttgg 4680
gttttaagtt gagtttatgg ttttgataat tttttgtttg tgtatatatt tgttttttat 4740
tttattttta ttttagtttt ggtatggggg agagggtata gggttagata aatttgtgag 4800
attttggttt tatttttgta aaagggtgtt ttgtgagtta gtttgttttt ttttaggttt 4860
gttttttttt tatttagttt ttgtttttaa tgtatgtata gtttgtatat attgtgtgtt 4920
gggatatttt atagttagtt gtatggtttt tttgtgtttt agtttttggt tttttttgtt 4980
gattttggtt tttgttttag gttttattgt gtaattgttg ttgttattgt tgtttttggt 5040
gtttgtttat ttttagaggt tgttttggat gtaggaggat ttttttttgg gaggaggttt 5100
ttttggggaa gatgatttat tgggtgagga ggatttgttt agtgaagagg atttatttag 5160

agaggaggat ttatttggag aggaggattt atttggagag gaggatttat ttggagagga 5220
ggatttattt gaagttaagt ttaaattaga agaagagggt tttttgaagt tagaggattt 5280
atttattgtt gaggtttttg gagattttta agaattttag aataatgttt atagggataa 5340
agaaggtaag tggttattaa tttttaaatt taggttttag gaggtttatg attttttttt 5400
tatattttag tttaggtttt gtttatttag ggaaggaggg gagattgtat tttttataga 5460
agttttttta gaggttttat attaatattt ttatttttat ttttggaggt agaaagggat 5520
agatgtggag agaaaataaa aagggtgtaa aaggagagag gtgagttgga tgagatggga 5580
gagaaggggg aggttggaga agagaaaggg atgagaattg tagatgagag aaaaaatgtg 5640
tagatagagg aaaaaaatag gtggagaagg agagttagag agtttgaggg gaagagaaaa 5700
ggaaagtttg ggaggtgaag tgggtattag agataagtaa gaagagttgg tagaagttat 5760
tttattttag gttataatga ggaaattgag atttaggaag aagggatata gtaggtagag 5820
aaatgtggtt ttttgatttt taagttagga atttggggaa aggggttgga gattatataa 5880
ggtagaggga tgagtgggga gaagaaagaa gggagaaagg aaagatggtg tatttattta 5940
tttgggattt aggattgaag tgtttattta tttttttttt tttttttttt gagataaatt 6000
tttatttttg ttgtttaggt tggagtgtaa tggtgtgatt ttggtttatt gtaattttta 6060
ttttttgggt ttaagtgatt tttttgtttt agtttttagt taagtagttg tgattatagg 6120
tatgtgttat tatgtttggt taatttttgt atttttagta gagatggggt tttgttatgt 6180
tggttaggtt ggttttgaat ttttgatttt aggtgattta attattttgg ttttttaaag 6240
tgttgggatt ataggtgtga gttatagtgt ttggtttgaa gtagttattt atttttatag 6300
attttaagat aatgattgta agttggtagg attgttgttt ggtttattta gttgtggtgt 6360
tgagtttggg tgtggttttt tgtgttttgt atttggtttg tttaaggtat ttgttatttg 6420
taatgttttt gtaaggtatt tgtgtttgtg atattgtttt ggttgttagg aagggattgg 6480
ggttttaagt ttgagtggtt tatttttttt atttatatag g 6521

<210> 326
<211> 6521
<212> DNA
<213> Artificial Sequence

<220>
<223> chemically treated genomic DNA (Homo sapiens)

<400> 326

tttgtataaa tgaaaaggat gaattgttta agtttagagt tttaattttt ttttggtgat 60
taaaatgatg ttataaatgt agatgtttta taggagtatt atgggtaata aatgttttaa 120
gtgggttagg tgtaaagtat aggagattgt atttaaattt aatattgtag ttgggtgggt 180
taaatagtaa ttttattagt ttgtaattat tgttttaggg tttgtaaaag tgagtggttg 240
ttttaggtta ggtgttgtgg tttatgttta taattttagt attttgggag gttagggtgg 300
ttggattatt tgagattagg agtttgagat tagtttgatt aatatggtga aattttgttt 360
ttattaaaaa tataaaaatt agttgggtgt ggtggtgtat gtttgtaatt gtagttattt 420
ggttagaggt tgaggtagga gaattatttg aatttgggag gtggaggttg tagtgagttg 480
agattgtgtt attgtatttt agtttgggta ataaaagtga aagtttgttt taaaaaaaaa 540
aaaaaaaaaa gtgagtgggt attttagttt tgagttttaa atgagtgagt atattatttt 600
tttttttttt tttttttttt ttttttattt atttttttgt tttgtatggt ttttaatttt 660
tttttttaaa tttttggttt gggagttaag aagttatgtt tttttatttg ttgtgttttt 720
tttttttagg ttttaatttt tttattgtag tttaagatga gatgattttt attagttttt 780
tttgtttgtt tttggtattt attttatttt ttaagttttt tttttttttt ttttttaaat 840
tttttgattt ttttttttta tttatttttt ttttttgttt gtatattttt ttttttattt 900
gtagttttta tttttttttt ttttttagtt tttttttttt tttttatttt atttagttta 960
tttttttttt tttgtatttt ttttattttt tttttatatt tgtttttttt tatttttgag 1020
agtggggatg gggatattgg tatgggattt ttggaagggt ttttgtgggg agtatagttt 1080
tttttttttt tttgagtgaa tagagtttag gttggggtat gggaggggag ttatgaattt 1140
tttggaattt ggatttggag attgatgatt atttattttt tttgtttttg tgggtattat 1200
tttggggttt ttgaggattt ttaggagttt taatagtagg tagatttttt aattttaggg 1260
agtttttttt ttttgattta ggtttaattt taggtagatt ttttttttta ggtagatttt 1320
tttttttagg tagatttttt tttttgggtg gatttttttt tttgggtgaa ttttttttat 1380
tgggtagatt ttttttgttt agtgggttat tttttttaga agagtttttt tttaaggggg 1440
aatttttttg tatttggggt aatttttggg gatggatagg tattagaagt agtagtgata 1500
gtagtagttg tatagtgagg tttggagtag gggttgggat taatagaggg agttaggggt 1560
tggggtatag gggagttatg tggttgattg tggggtgttt tagtatatgg tgtgtatggg 1620
ttgtatgtgt attggaaatg agagttgggt gggggaggag taagtttgga ggggagtagg 1680
ttgatttata gagtgttttt ttgtagagat ggagttaaag ttttataggt ttgtttggtt 1740

ttgtgttttt ttttttatat taaagttagg atgggggtgg agtgaggggt aggtgtgtgt 1800
ataggtagaa ggttattggg gttatggatt tagtttggaa tttaaggtat tatttggtat 1860
tatgtaggtt ttttttggta ttatttagtt gtttgttagt tagttttagt taattttggt 1920
ttgtttttta gttttaggta tagaagggga aaggtagggg agttggggtt tttatgtttt 1980
ttggttgaga gggaaagtag tttatgtata tatttttgat tttattagga agtaggtaaa 2040
gaggtagggt aggttgttag gggagtttga gggtgagtta agtaatgggt atgtgggaga 2100
taaatttgag tttgaatgaa aagtaagtgt atgtgttaga ggggttttga taggtttaat 2160
ttatatttat tttttaatat attatttttt tgtttaaaag tttttattgg ttttttattg 2220
tttgttatga tatgtatatt gtgtattttt gatttttaat tttttttaaa atttgtttta 2280
agagattttt ttattttgtt ttttattatt tttttttata ttttagttaa attaaattat 2340
tatttttttt tttaaatttt ttattatttt tgttaattta gtttttgttt tttgaaatgt 2400
tatttattat tgtatttaat aaatatttat ttagtattat tataggttta gtattgggtt 2460
atggattagg gatatggaaa tgaataagat atggtttttt attttgggag gttgaggtgg 2520
gtggattgtt tgagtttagg agtttgagat tagtttgggt aatatggtta aattttgttt 2580
ttattagaaa tataaaaaaa ttagttaggt gtaatggtat gtgtttgtag ttttagttat 2640
ttaggaggtt gaaatgggat gatggtttga gttgaggtgg ttgaggttgt agtgagttga 2700
gattgtatta ttgtattttg gtttgggtga tagagtaaga ttttgtttta aaaaagaaaa 2760
ataaaaataa aaataaaaaa tgttatttgt tttgagtagt ttataattta gtgagtgagg 2820
gtaaattttg ttaatattat tatattgagg gaagagagat gaattttgtt ttagaagata 2880
atatagaagt aggtaaattt gattatgttt ttgaagaaat attagtttta ttggtaaaaa 2940
taatagggag aatgatattt tgggtaaagg gaaggtataa aatattatga gtaggagagt 3000
gatagaattt agtttttata ttaggttatg ttgagggttt gagggttagt atggagtata 3060
gattgtaggg gaagattggg tgtaaggaga tggggtagaa gagtgtgagg atattaagaa 3120
tgggtttagg ttttttagtt tttttttttt tttttttttt tttttttttt tttttttttt 3180
tttttttttt tgagatagag ttgtattttg ttatttaggt tggagtgaag tggtgtgatt 3240
ttggtttatt gtaattttta ttttttaggt ttaagtgatt tttttgtttt agttttttgg 3300
gtagttggga ttataggtat ttgttattat gtttagttaa tttttgtatt tttggtagag 3360
attggggttt tgttatgttg gttaggtttg ttttgaattt ttgattttag gtgatttgtt 3420
tgttttggtt ttttaaagtg ttgggattat aggttggttt ttttttttaa ttaattattt 3480
ttaaaattta ttttagagat gggattttat tgtgttgttt aggttggagg gtggtggtta 3540
tttataggtg tgattttgtt attgattagt attgagtttt gatttgtttt aatttttatt 3600
tgggttagtt taattatttt taggtaatta ggttgatgta gaatttagtg tagatatttg 3660
attggtatat tatattatag tttagaaatt ttgggattaa gtgatttttt tgttttagtt 3720
ttttaagtag ttgggattat aggtatatgt tattatattt ggttagtttg tttttttaat 3780
aatttattaa gtttttgtta gttttttagg tgttgtttta ggttttatag atgtagtatt 3840
gaatagagaa tagttttatt gttttgtagt ttatatttta gtaaggtttt attggtttaa 3900
ataaattaaa agagaataag gttattttag gtttatattt agggttaata tttagtaggt 3960
atttattaat atggttgtgt aggttaatta aatatattaa gatattttta attttgttgg 4020
tatatagtta tggtttttag ttttagggta tttttgttat tttagttttt ttttagggat 4080
tttttagatt ttttgatttt ttatttaaag aggtattttt tggtataata gagtttttag 4140
gagtttattt gagtgtttta gttattttta attttgatag gttagtgttt gtgtatttgt 4200
tgttgtgtta taaatagttt tattggttag gtgtagtggt ttatgttttt aatattagta 4260
ttttgggagg ttgaggtgtg tagattattt gaggttagga gtttgagatt agtttggtta 4320
atatggtgaa attttgtttt tattaaaaat ataaaaatta gttgggtatg gtggtaggta 4380
tttgtaattt tagttattta ggaggttgag gtaggagaat tatttgaatt tgggaggtag 4440
aggttgtagt gagttgagat tgtgttattg tattttagtt tgattgatag agtgagattt 4500
tgttttaaaa ataaataaat agataaaaat tgttgttaat atagtgaaat tttgttttta 4560
ttgaaaaaaa aaaaaaaaaa aaattagttg ggtgtggtgg tggttgtttg tagttttagt 4620
tatttaggag gttgaggtag gagaatggtg tgaatttagg aggtaaagtt tgtagtaagt 4680
taagattgtg ttattgtatt ttagtttggg tgataaagtg agattgtttt aaaaaaaaaa 4740
aaaaatttta ttgttttttt tgtttaattt tttttgtttt ttgaaattta gatgaaaggt 4800
tatttttttt aggaaatttt ttaattaatt ttttattatt agtttggaaa agatttttgg 4860
tatttttgag ttaatttagt tttttgtttg tttttaataa taattttgtt tattgtttaa 4920
tttatataag ggtgataatt tattttattt gttttaatgg tttttagttt tataagggta 4980
gggtttgtat ttgattgagt tagtttttta tttaatagtt atattgagtt tttggtttat 5040
ttttgtaata ttagtatttt ggaggttaag gtagtaggag gattgtttga ggttaggagt 5100
ttaaaattag tttgagtaat atagtgagat tttttatttt tattaaaaaa taaataaaaa 5160
tagtagatag gtttgatgat ataagtgtgt agttttagtt atttgggaga ttgaggtggg 5220
aggattattt gagtttagga gttggaggtt gtagggagtt atgattatgt tattgtattt 5280
tagtttgggt gatagatgga gagattttgt ttttatagta gatgtataaa tattattgtt 5340
ttataattgt ttatagtatt tagtatggta atatgttgta agtattgggg tttaggagta 5400
ttaggttata ttatgtagtt tagatgggta ataggttata ttatttaggt ttgtgaaaat 5460
atattttatg atgtttgtat aatgataaaa ttgtttagtg atttattttt tagaatatat 5520

ttttgttgtt aagtaatgta tggttgtttt tatgttgata tgattatatt tataaagttt 5580
aaggaaagaa aatatttatt gagtttattt tttggttttt attatttatt ttataattgt 5640
tattgagaat aattttgtta tataaagaat gtttaaattt gttttaattt tatttattta 5700
tttatttgag atggagtttt tttttgttgt ttagattaga ttgtagtggt ataattttgg 5760
tttatttgtt taggttggag agaagtggtg tgtttggtta taaagtattt ttttaaatga 5820
tttatttgaa aaataaaatt ttgttttagg tatggtggtt tatgtttgta attttagtat 5880
gttgggaggg tgaggtaaga ggattgtttg agtttaggag tttaagaata gtttgggtaa 5940
tatggtaaaa ttttgttttt attaaaaata taaaaaatta gttaggtgtg atggtgtatg 6000
tttgtggttt tagttatttg agaggttgag gtgggaggat tgtttgagtt taggaggtag 6060
aggttttagt gagttaagat taagtttttg tattttagtt tgggtaatag agtgagattt 6120
tgttttttat ttttaaaaaa agaaagaaaa taagaaaaat ttgggtttgg gtatggtggt 6180
ttatgtttgt aattttagta ttttgggatg ttgaggtggg tggattattt gaggttagga 6240
gtttgagagt agtttggtta atatggtgaa atttttttgt ttttattaaa attataaaaa 6300
ttagttaggt gtggtggtgg tatatgtttg taattttagt tatttgggag gttgaggtag 6360
gggaattatt tgaatttgtg aggtgaaggt tgtagttagt tgagattgta ttattgtatt 6420
ttagtttggg tgatagagta agattttgtt ttaaaaaaaa aaaaaaaatt tgtttttttg 6480
aattgtattt gattggattt tttatttatg tataattttg t 6521

<210> 327 .
<211> 6565
<212> DNA
<213> Artificial Sequence

<220>
<223> chemically treated genomic DNA (Homo sapiens)

<400> 327

tgatttttaa attttataag ggttattgta gttttttgaa gtagaaaatg tagtttattt 60
tttgttagtt tatgggttat attttgattt aagtattttt atggtgggta tttgtgttta 120
ttttgtagtt tttgttagag attatgttag aagatggtgg tatataggtt gagtaagagg 180
tggtgaggtt gattggggtt tattgattat agaataggtt tttttagagg gatatgaagt 240
attgttaggt tttttgagtt ttaagttgtg gtttgtagtg ttttggtgag tagttttgtt 300
aatttaattg ttgaggttta gggttaagta tagtggggta tttaatttta gtttgggttt 360
tagttattgt gtgtttagat gtgttaaagt tatttttgta gtttattttg tattaattgg 420
agttttttat aatttaggtg aattttagtt ttattgaggg gaattgattt aaaatatttt 480
ttatgttggt ttttattgat ttgggttaat tgtgtgattg tggtggttgg tatgaatttg 540
atgaatttta gggttagtat agtttagtta aatttttgtt tattgttaaa ggtttattat 600
tgttgttttt tgtgggggtg tggttaggtt aagtgttttg agttgtgttg ttgtgtgttt 660
gatatttgtt ttttttgatt gtggtgattt agagggtgat ttattggggt agggatgttt 720
gtatgtgtaa ttttattaag agttaatagg ttatgattaa atttatttgt ttatggggtg 780
gtatgagttt gttgaaatat ttttagtgta ttgttttgag gaagtaagtt atataaattg 840
tatggggtgt ttttggggtt tggttggttt ggggtatggg gttagtagtg gtgtatatgt 900
tgagtaaggt gggtagggag ttgtattgat ggggttagta gaatgtgagt tgagggagga 960
tgatgtgtta gttgaggggt gattgttaaa aatgtatttt tttttttgtt attatgtgaa 1020
gaaggatgtg tttgtttttt tttttattat gattttaaat tttttgtggt ttttttagtt 1080
atgaggaatt gtgagttaat taaatttttt ttttgtataa attgtttagt tttgggtagt 1140
tttttataag aatatgagaa tggattaata taggtttaat tttggttatt ttttaatttt 1200
ttaagttatt ggagttgatt ttttgggtag ttttaatatg attatttttt ttatttttta 1260
gttattaggg tatattaggt tttatgaatt attgatttta atttttgggt tggaggatag 1320
gaaaattttt agtagaggtt aattggagat atttgttttt ttttaggatt tttagaatta 1380
ttttaatatt ttgaaatgtt ttatttttag atattgggta atgaatgtta agtataaaat 1440
attattgggt attgtggtta attaggaata atagagtaga agattatgat ttattttagt 1500
ttattttaaa atttatattt tattgtattt agggttgggt ttagaaaggg gattattatt 1560
taattttagt aagttttaaa aaatagttgt tttgatttta tgtgaaatta taggttattg 1620
attatattgt ataatttata tttgggttta ttgaatgatg gtttaagata atttgtaaat 1680
ttgatattat agtgagttat ttaatgttta atgtaatttt ttaaggtgat gaaattaatt 1740
atttaggtag tagaataagt tattgttatg tagtataaat aattgtttat taatttttta 1800
taattatttt tgtgatatta agtagtttta ttaatagttt tttttatata attttatttt 1860
attatatatt gatttttatt atgagatatg atttttttgt ggtgaattta tgttaaggta 1920
tttatttgag ttttttgttg agaattaaat taattttatt ttttaaaggt tgattttttt 1980
taggaatgga gaatttaggt ttttttattt ataattatta gattttgtta tttataaaat 2040
tataaagaaa gtttaagtat attttgggaa tatatatata tttttatgta ttatatataa 2100

tatgtttttg aataatattg ttttttttta atgttgatga gaaaaataat taatttttgg 2160
ttagggttat tgtttgtgtg gagtttgtat gtttttttta tgtttgtatg gggttttttt 2220
aggtatttta gttttttttt atattttaaa gatatgtata ttattttttt taaatgtatg 2280
tatagtttat atttatttta gtgtttttta ttagaagtgt ttttggtttt tatttagaag 2340
tttggtgatg tttttgtgat tagtaatatg ttttaggaat ttaatttttg tgtatattta 2400
gtagtttatg gtaaaattgg ttttgtttta tattgtttta tttaaagtta tagtttttaa 2460
gaatttatag atgatattga ggattattta tgtgtataga tatgatggat attttgatat 2520
ttagtggatt ttttttaaaa aattgaattt aaaagtttta ttaaaattta tatttattat 2580
ttattattta aatttaatta tttttttttt tggtattatt taatttatat ttaatattta 2640
gttttaatta tttgtaattg tgtatgatat agtaaagatt ttggggaggt taagatgggt 2700
agattatgag gttaggagat tgagattatt ttggttaata tggtgaaatt ttatttttat 2760
taaaaatata aaaaattagt taggtgtggt ggtaggtgtt tgtagtttta gttatttagg 2820
aggttgaggt agaagaatgg tgtgaatttg ggaggtggag tttgtagtga gtgtagattg 2880
tattattgta ttttagtttg ggtgatagag taagattttt ttttaaaaaa aaaaaaaaaa 2940
aaagattttg agttaaagtg tttaagttga atgtattttg ttatttataa gataaattgt 3000
gttaattttt tgtggtttat tttatttata aaatagagat aataatagtt tttgttttta 3060
gggttgttgt gggaattaaa gatttagaat aatgtttagt ttttaattag tgttgttata 3120
attgtttgat ataattgtat tatatttgtg tattttgtag attgatatta aattatattt 3180
ttaaaaatag gtgtttaatg ttttatttaa ttattttaaa atatgtttaa ttatgttttt 3240
attttatttt tataatattt ttataaaaat tttttatata tagtgtttat ttttggttta 3300
gttttttagg aaaataattt tgagagttag ttatttgaat taaagaaata ttaatattat 3360
tagattatat tgggattttt gagattggtt tttattaatt ttttagttat gggtttttgt 3420
tattattttt attagtgatt taagtgttaa atttaaatgt tttgtatttg ttttattaaa 3480
gagatgtagt atttgttttt tttttattgt ttttattttt tttgttatgt ttttttttat 3540
aattataaat atttaggttt tttaggtttt aaatggggta ttttttaatt tttatatttt 3600
tttttttggt tatttttttt tttttaatag tttaatttat ttagattttt atgtttgaaa 3660
ttattttaga tgttttagag gtgttttatt attataatgg tatattattt tttatttttt 3720
tatatgttat gttagttttt aaattgaaaa tttgagtgtt tatttttggt gtattatttt 3780
taaattttag atttttaaaa tttagggtta tgtaatttta aaaaattttt attttttttt 3840
ttttattgtt tttgtttagg ttttattttt tttagtagtt gttttaaagg tttattttgt 3900
tttttttttg gagtgttaat ttttattgaa gtttttattt agttgttaat tttgttttat 3960
gtttgataat ttgtttgtgt gttgatatat ataaaatttt taagataaaa attttttaat 4020
aatggtaaat gaattttggg aattgtatat agattatata gatttataat aagagaaaag 4080
gttttagatt aatatggaaa attttttatt taattaatat ttgtattggt aaattttatt 4140
aagtaagatt ttatttaatt ttatattatt ttttattgaa gaggttgtgg ttattttttg 4200
gaaatatttg aatttatttt tataagttag agaaatagtg ttatttgaaa tattattttt 4260
tgggtttgag ttttaaggta tttgataaat ggagtgttgt gggtaggggt gaggtgtttt 4320
ttttagggtt gggattttgt tttgggtgag ggtgttgtag ggtaaagatt ttattgggta 4380
gtagaatttg ggtagaaatt agtaattggg ttttttgtgt agtagaaaag gggaatttag 4440
ttggggttta ttttttttgt tagtgtagat tgtaagtttg gttttatttt tttgttggga 4500
gttggtttgg tgtgttttgt ttaggtattt tgattgtggg tagtttgtgt ttgtttgggt 4560
tttattgttt tggtttggta gatatttgag tggtggttag ggtaggtttt tgtttttgtt 4620
gatgtttatg ttttgggata tagtgatgat gtagtttagt gaattaatta tgatagtagt 4680
gggaggattt ttgagtttgt ttgttatagt agaatgtgtg gttaagtttg gtgtgaaatt 4740
gtggttgttt tgtttttttg tttttatttg tgtaggtgag gttttgtttt tttgttttgg 4800
tgtatgtagg gttgtggtgt gtttgtgttt gtagatgttt gggaattgtg gttgtgggtt 4860
tgtgtttttt gttgggtttt gttgttgggt tgttattttt gttttgttat gttttgttgg 4920
aagtttgtgt tgggtggttt tgttgttttt agtttagttt ttgtgaggta agtggttttg 4980
agttgatttt tttagtttat gggaagtttg aaatttattt ttttttttat aggtgtagtt 5040
gattaggtgg attttggtgt tgtagtggtt gtagtgtttt tagtgtttgt ttttttgttt 5100
tagttgttgt tgtatgtagt aggttttgtt tggattgggg tagggttatt ggggttgggg 5160
gggtggggtt tgtgggtaag gtgggtggag gtgtggattt tttgtttgat gatagggttg 5220
gaggaggaag gggtgggttg aagaagggga aggtgggaag agtttagttg gggttataaa 5280
ttgggtgaag tgttgaggtt ttagtatttt tgtttgagga gataggtaaa ggttatgtag 5340
gtttttaatg gtaggtttga gataggaatt taggtttttt gatttttatt ttgatgaggg 5400
ggtttgtggg taaggggtgt gttttttaat ttttattttt tttttttttt tttggtggtt 5460
gaagagtttt attgattttt taaaaaagtg ttggattggg tgattagaag aaagttgttt 5520
ttaggttttt agtttttaag attgtaatag tatggtttaa tatttgaaat tattatggtt 5580
aaaagtagtt atattgtgta gttttttagt gaatattttt tgtagtttag attataaagt 5640
ttgaaatata gtttgggtgg ataaagtttt ttaggatggt atagatgtga ttaattatgg 5700
agtttttttt gttgttgttt tttaatgtaa taagtatgtt tgtttgtttt taaattagag 5760
atattgttat aggaaaagtt taagggtgat tttttatttg ggttatttgt atagtatttt 5820
ttagattgag gttgtggaag ggtattgaag attggtgttt agttatttat tgtgttttaa 5880

tgtaggtggt agtttgggtg ttttaaaatt ttgttttttt tgtatatatt ttttttttaa 5940
aatgattatt ttttttttgt tttttttgag gatgagttat gtttttatga atttattaaa 6000
attttgtatt tattatttaa agggtagttg gtgaaggatt tatgtatgtt tttggtaagt 6060
tttttgttaa gaaaaaattt ataattttgt tatattttta gtgatgaata taaatttttt 6120
aaataggaaa tatttaaatt gtaatgaata tgttaaaaat gatattgttt ttttattatt 6180
tatttttttt tttttgtgtt tttgttatat aattgtttat tttttagagg tattttattt 6240
agtatatgta agtagggtga tttaagtttt ataattagag aaagttgaag gtttattgaa 6300
ttaagattgg tagtagtttt ggttttaatt aattttaaat atggtttgag ttgttgaggt 6360
ttttgattat tagattttat tttgtttttt atggtgttaa aaatagttta tttatagggt 6420
tttttagagt taaaagtaga ggattttttg aagagtggat ggtaaggaat tttgttattt 6480
agttgtaaag tttaaattgg tttatatatg ttaggttttt ttagagtaga gataatatat 6540
tattttttaa tttttttgat atagg 6565

<210> 328
<211> 6565
<212> DNA
<213> Artificial Sequence

<220>
<223> chemically treated genomic DNA (Homo sapiens)

<400> 328

tttatattgg gaaggttaga aaatgatgtg ttatttttat tttgagaagg tttgatgtat 60
gtgaattaat ttaaatttta taattgaatg ataaggtttt ttgttattta ttttttaaag 120
aattttttat ttttagtttt ggaaaatttt atgggtgaat tgtttttaat attataaaag 180
ataaggtaga gtttgatagt taggagtttt agtaatttaa gttgtgttta gaattaattg 240
aaattaaggt tgttattggt tttgatttaa tgagttttta gtttttttta attataaaat 300
ttgaattatt ttatttatat atgttaaata aaatattttt aagaaataaa tagttatgta 360
gtaaaggtat agaggggaaa agataggtaa taaaagaata gtattatttt tgatatgttt 420
attgtaattt aagtgttttt tgtttaaaag gtttatgttt attattaaag atgtgataag 480
attatgaatt tttttttggt agaaaatttg ttaaaagtat atataagttt tttattagtt 540
attttttaaa tggtgggtgt aaaattttgg tgaatttatg aaaatgtagt ttgtttttaa 600
aaaaaataga agaggagtaa ttattttaag ggagaaatat atatgaaagg aataagattt 660
tgaagtattt aagttgttat ttatattaaa atatggtagg tggttaaata ttagttttta 720
atgttttttt atagttttag tttgaaaaat attgtgtagg tgatttaagt gaggggttat 780
ttttgggttt tttttgtggt agtatttttg gtttaaaaat aaataaatgt atttattgtg 840
ttgaaggatg gtaataggaa ggattttatg attagttata tttatattat tttaagaaat 900
tttatttatt taaattgtat tttagatttt ataatttaaa ttataaaaag tgtttattgg 960
ggaattgtat aatatgattg tttttaattg tagtgatttt aaatattgag ttatgttgtt 1020
gtagttttaa aaattggaga tttaagggta gttttttttt agttatttaa tttagtattt 1080
ttttaaaaaa ttagtaaaat tttttgatta ttaaggaaaa aaaaaaagga tggaggttaa 1140
aagatgtatt ttttgtttat aagttttttt attagaatgg gagttaggag atttgagttt 1200
ttgttttagg tttgttatta aaaatttgta taatttttgt ttattttttt aaatggaagt 1260
attaaaattt tagtgtttta tttaatttgt agttttggtt gggttttttt tatttttttt 1320
ttttttagtt tgtttttttt ttttttagtt ttattattgg gtggagggtt tatgtttttg 1380
tttgttttat ttataagttt tgttttttta gttttgatgg ttttgtttag ttttagatag 1440
aatttattat gtgtggtggt agttggggtg ggaaggtggg tgttgggggt gttgtggttg 1500
ttgtagtgtt agggtttatt tggttggttg tatttgtgga ggaggaggtg gattttaggt 1560
tttttgtaga ttggaagaat tggtttaaaa ttgtttgttt tgtaggggtt gagttggagg 1620
tagtgaggtt gtttgatgta ggtttttggt gagatatggt agggtaagga tggtagtttg 1680
gtggtagggt ttggtgagga gtgtgaattt gtggttgtag tttttaggtg tttgtgggtg 1740
tgagtatgtt gtgattttgt gtgtgttggg gtgggggggt ggggttttgt ttgtataaat 1800
agggatgagg gggtggggtg gttataattt tgtgttaaat ttgattgtgt gttttgttgt 1860
aatgagtggg tttggaggtt tttttgttgt tgttatggtt ggtttgttaa attgtattgt 1920
tgttgtgttt tagaatatgg gtattggtaa gaatggggat ttgttttggt tattgtttag 1980
gtatttgttg ggttggggtg atgggattta aatgggtgta ggttgtttat ggttggggta 2040
tttgggtggg atgtgttagg ttgatttttg gtgagaggat ggggttagat ttgtggtttg 2100
tgttggtagg aagggtgggt tttgattgga tttttttttt ttgttgtgtg ggaggtttag 2160
ttgttgattt ttgtttggat tttgttgttt ggtgaggttt ttgttttgtg gtgtttttgt 2220
ttagggtaaa gttttagttt tggagaaaat attttatttt tatttatagt gttttgtttg 2280
ttaggtgttt tagagtttga gtttaaggga taatgttttg agtaatgttg tttttttaat 2340
ttgtaggaat gaatttagat atttttagag aatgattata atttttttag tagaaggtaa 2400

tgtgggatta agtagggttt tgtttgatga agtttattag tgtaaatgtt agttaaatgg 2460
aaagtttttt gtgttaattt gggatttttt tttttattat ggatttgtat gatttgtatg 2520
tagtttttaa ggtttattta ttattattaa aaaatttttg ttttagaaat tttatgtatg 2580
ttaatgtatg agtaaattat taggtatggg gtagaattgg taattgggtg gaggttttgg 2640
tggaggttag tattttgaaa ggaaaataga gtaggttttt ggaatagttg ttggaagaga 2700
taaggtttga ataagggtag tggagaagag agggtaaaaa ttttttaagg ttatatgatt 2760
ttggattttg gagatttgga atttaaaggt gatgtattag ggatgaatgt ttagattttt 2820
agtttgaaag ttggtgtgat atgtaaagga gtggagaata atgtattatt gtaatgatga 2880
ggtgttttta ggatatttag gataatttta ggtgtgggga tttaggtgaa ttgaattgtt 2940
ggagggaagg gaataattaa ggaaaagaat gtgggggttg agagatgttt tgtttaaaat 3000
ttaagagatt tggatattta tggttgtaag aggaggtatg gtagaggaaa tggaaatagt 3060
aagaaaggag tagatgttgt atttttttaa tgggatagat ataaggtatt tgggtttgat 3120
atttaggtta ttggtagaga tgatgataag agtttgtagt taaagaattg ataaaagtta 3180
gttttaaaaa ttttaatata gtttggtaat gttaatgttt ttttggttta gatagttgat 3240
ttttaaagtt atttttttaa gaaattgaat taaaagtgga tgttatatgt aaaaagtttt 3300
tatagaagtg ttataagagt aggatagaga tataattaaa tatgttttaa ggtaattgag 3360
tggaatatta agtatttatt tttaaaagta tgatttaata ttaatttata aagtatataa 3420
atataatata attgtattag atagttgtga tagtattgat tagaggttga atattatttt 3480
aagtttttaa tttttataat aattttagaa gtaggaatta ttgttatttt tattttatag 3540
ataaagtaaa ttataagggg ttaatatgat ttgttttgtg gatgataaaa tgtatttaat 3600
ttgagtattt tgatttagaa tttttttttt tttttttttt taagagggag ttttgttttg 3660
ttgtttaggt tggagtgtag tggtgtgatt tatgtttatt gtaagttttg ttttttgggt 3720
ttatattatt tttttgtttt agttttttga gtagttggga ttatagatgt ttgttattat 3780
atttggttaa ttttttgtat ttttagtaga gatggggttt tattgtgtta gttaggatgg 3840
ttttgatttt ttgattttgt gatttgttta ttttggtttt tttagaattt ttattatgtt 3900
atatataatt gtaaatgatt aaaattaaat attggatata aattggataa tgttaaaggg 3960
aaaaatagtt agatttaggt aatgagtgat aagtatgaat tttggtaaga tttttaagtt 4020
tagtttttta aaggagattt attaaatgtt aggatgttta ttatatttat gtatatagat 4080
agtttttaat gttatttgtg agtttttgga aattgtgatt ttaagtgaaa taatgtagag 4140
taaaattaat tttattatag gttattggat gtatataaga gttaagtttt tagggtatat 4200
tgttggttat aaaaatatta ttaaattttt aaatgaaggt taaaaatatt tttaatagaa 4260
aatattgaag taaatatgag ttatatatat atttaagaag aataatgtgt atgtttttgg 4320
gatgtggaag gaaattggaa tatttggaga aattttatgt agatatggga aaagtatgta 4380
gattttatat agatggtggt tttggttagg aattgattat tttttttatt aatgttagaa 4440
gaaaatgatg ttatttaagg atatgttgta tataatatat gaaaatatat gtatattttt 4500
aaaatatatt taggtttttt ttgtgatttt ataggtaata gaatttggtg attatgggta 4560
agaagatttg ggttttttat ttttgagaag aattgatttt taaagggtag aattaattta 4620
gtttttagta gagaatttaa gtaagtattt taatataaat ttattataag aaaattatgt 4680
tttatagtgg agattagtat atgataaaat gaaattgtat aaaaggagtt attgatgaag 4740
ttgtttaata ttataaaggt gattatagaa aattaataag tagttattta tattatatga 4800
taataattta ttttattatt tgagtaattg attttattat tttgaaaagt tatattaaat 4860
attaaatagt ttattgtggt attagatttg taagttattt tgaattatta tttagtaggt 4920
ttaaatataa attatatagt gtaattaata atttgtagtt ttatataaag ttaaaataat 4980
tattttttgg gatttgttaa aattaagtga tgattttttt tttggattta attttaaatg 5040
taataaaatg tgaattttag gatgggttga aatgggttat aattttttat tttgttattt 5100
ttagttaatt ataatattta ataatatttt gtatttaata tttattattt agtgtttgaa 5160
ggtaaaatat tttaaagtat tgaaatggtt ttagagattt tggagagaag tgggtatttt 5220
tagttagttt ttgttaagaa tttttttgtt ttttaattta ggggttggag ttagtagttt 5280
atgaagtttg atatgttttg gtgattggag agtgggaggg gtaattatat taagattgtt 5340
taggaagtta attttaatgg tttgggaggt tgaggggtgg ttagaattgg atttgtgtta 5400
atttattttt atgtttttat aaagaattgt ttaagattgg gtaatttgta taggaaagag 5460
gtttaattga tttatagttt tttatggttg gggaggttat aggaaattta aaattatggt 5520
ggaaggggaa gtaaatgtat ttttttttat atggtggtag gaaggaaagt gtatttttaa 5580
tagttatttt ttaattaata tattattttt ttttaattta tattttatta attttattaa 5640
tgtaattttt tgtttatttt atttaatata tatattgttg ttagttttat attttgaatt 5700
aattaaattt taaagatatt ttatatggtt tatgtagttt atttttttaa agtaatatat 5760
tgaaaatgtt ttgataggtt tatattattt tataaataaa taggtttggt tgtagtttat 5820
tagtttttag taagattata tatgtaagta tttttgtttt ggtgagttat tttttaaatt 5880
attatgatta aaaggaataa gtattaagta tgtagtaatg tagtttaaaa tgtttagttt 5940
agttatattt ttatgggaga tagtagtgat aaatttttag taataaatga aagtttaatt 6000
aagttatatt aattttaggg tttgttaagt ttgtgttagt tattgtggtt atatgattaa 6060
tttaagttaa tagaaattgg tgtaaagagt gttttagatt aatttttttt aataaagtta 6120
aaatttattt gagttgtaaa aaattttagt tgatataaaa taaattatga aagtggtttt 6180

aatgtatttg aatatataat agttaagatt taaattggga ttagatattt tattatgttt 6240
agttttaaat tttaatagtt aaattaataa aattgtttgt tagaatatta tgagttatag 6300
tttaaaattt aaaggatttg gtggtgtttt atattttttt agaggagttt gttttgtaat 6360
tgataaattt tgattaattt tattattttt tgtttagttt atatattgtt attttttagt 6420
ataatttttg atgaaggttg taaagtaagt gtaagtattt attgtaaaga tgtttaggtt 6480
aaggtgtagt ttatgagttg gtaagaaatg ggttatattt tttattttag aaaattatga 6540
taatttttat gaaatttaag ggttg 6565

<210> 329
<211> 2891
<212> DNA
<213> Artificial Sequence

<220>
<223> chemically treated genomic DNA (Homo sapiens)

<220>
<221> unsure
<222> (223, 366)
<223> unknown base

<400> 329

tttttttttt ttgaattgtt tttttttttg gaggtggtgg agggagagaa aagtttattt 60
aaaatgtttt tgggtgaggg attaaggatg agaagaatgt tttttgtttt ttatgttgtg 120
gaataatata aaataaaaaa ttttgaggga atatatatta tatattaaat atagattatt 180
ttagggagta aataaattat gtgtggggtt gggtaattag ttnaagttga agtgtaaata 240
aaatgtgaat atatgtttgt gggttatata tagtgtattt ttattagtat ttagaaaaaa 300
ttgtgagtta gtgaattagg aaattaatgt ttggaaggta gttaaatttt aattagttta 360
agattntttt ttttttttaa aaaaaggtat ggaagtaata tttttttttt tttttttgat 420
tagaattgat gtattttttg tgtatgattg tatttttaat aataaaaggg gaaagaggat 480
ttggaaagga attaaatgtt tggtttgttt ggggaggaaa gagttaatgg ttttttttat 540
aagggttttt gttgattttt ttggtttggt ttataagttt tttatttgtt ttttttagga 600
agtttggttt tgtggtttgg gtattttttg ttttttttat atttttttgt ttagtatttt 660
tgattttttt taaatttggt agtttgagat tgttgtaaat tggtgttata gggtgtaaag 720
gggatttgtt ttttttgaaa tttggttgag aaattgggaa ttttgtgtgg gaggtgtggg 780
ggtgggatgg tggggtatag attggtagag agtaggtaat tttttttttg ttttagttta 840
gttttggaat aggtagatat attttagggt taaatagatg ttttttgtat ggggttttat 900
ggaagtttga gtaggtgggg taggagggtt ggtatttgtt gttttggtag taaattgggg 960
gatttagttt gggtggaagg tatttaattt agatagttgt gtatatataa tgtataatat 1020
atgatttttt ttaataaatg taatgggagt ttatttataa tgtgtttttt aagtatatgt 1080
ggtaatgtgt tgttgggtta ttttaattat tttaggtatt gttttttttt ttatgttttt 1140
attatttttt tttatttata ttaatatttt atgttttgaa tgtgtgttta ttaatatttt 1200
tttttttttt attttttttg ggatttttga ttaaagtgtg gttttttttt tagttttagt 1260
gaggtgtttt gtagtttggt atgtgtgtgg tgtggtggtg ggtgtgtagt gtgtttttgg 1320
tgtggagggt agttgttttg tttgtgatga tttatattta taggataagg atgtggtttg 1380
ttaaatagta ttgttatgga ggagtagtag agaaagggag agggtttgag agggagtaaa 1440
agaaaatggt aggtgtgtgt agttaattta tgtggttttt ttattttgtt tatattttag 1500
agttagagtg tttggttgtt tggttgagtt ttttttttat tttttttatt ttttttattt 1560
tttttataag tgtttttttt gggtttttaa agtagagggt gtgggggaaa agaaaaaaga 1620
tttttttttg ttaatttttg tttattggtt ttttataatg tgagggtttg gatggttgag 1680
gatttttgag ttgtgttgtt tgtggttgtt attgttgggt tttggttgtt tttggttttt 1740
tttttgtttt gagaagggta gggtttttta gaggtttggt gggaaaaaga atggagggag 1800
ggattgtgtt gagtataaaa gttggttttt ggggttttat ttaatttgtt gtagtaattt 1860
tagtgagagg tagagggagt gagtgggtgg ttggttaggg tggaagagtt gggtgagtag 1920
agttgtgttg tgggtgtttt gggaagggag atttggagtg aatagggggt tttgtttttg 1980
gtttagtttt tttgttgatt ttttaattag tggtttgtaa tttttgttgt atttatgaaa 2040
ttttgtttat agtagtgggt ggatattttg tattggaatt tataatattt gagtaaagat 2100
gtgatttttt tgatgtgggg aggttatttt gtttatttgg ggatattttt ttgttgttgt 2160
taggatttgt ttttttgaaa ggtttttttt gtagttgttt agatgttgga ttttttttgg 2220
gtggaaaatt aggtaagtat tgaagtttat ttgtttttta atttattttt ttattatttt 2280
aatgttgaga tgagttgaat gtttaaatag ggtgtttttt tttttatttt tgtgttattg 2340
atattttttt tagagtagtt atggtaattg gggttggggt ggggggtaat ttagaattgg 2400

attggggtaa agtgatttgt taagatggta gaggagaagg tagagggaaa atgggaatgg 2460
tttttaagat tatttttttg agatttttgt tttatgaata tatttatgtt gatttttggt 2520
tggttggata tttttgtttt attgtgttaa ttgttttttg tgttttgggg ggttgggggt 2580
tgttttgtgg tgggtagaaa gttttttgta ttttgagttt tttggagtag ggattgtata 2640
ttgtttgtgt gagttagatt gttttgtagt tgttgatttg tttttgtttt tgggagggta 2700
tttaaatttt ggtttattgt atttttgata gttggagatg gatattgtgg tgtgttttgt 2760
ttgtttgttt ttgtggtgat tgtaatttgt tttgattttt ttaagaagtt ggtatttggt 2820
tttttaaaaa ataataatat aatttaaaat ttgggttttt agaggtgtta ggatgtggtg 2880
ttgggtaggt g 2891

<210> 330
<211> 2891
<212> DNA
<213> Artificial Sequence

<220>
<223> chemically treated genomic DNA (Homo sapiens)

<220>
<221> unsure
<222> (2526, 2669)
<223> unknown base

<400> 330

tgtttattta atattatgtt ttaatatttt tagagattta ggttttaaat tgtattattg 60
ttttttaaaa agttaaatgt taatttttta aaaggattag ggtgggttgt aattgttgtg 120
gggataggtg ggtgggatgt gttgtagtgt ttgtttttgg ttgttagaaa tgtggtgagt 180
tgaaatttaa atgttttttt ggagatgggg ataagttagt ggttgtggag tgatttggtt 240
tatataggtg atatgtggtt tttattttaa ggagtttagg atgtaagggg ttttttgttt 300
attgtaaagt aatttttagt tttttaaaat gtagagagta attaatataa taaagtagga 360
atgtttgatt ggttgggagt tagtgtgaat atatttataa ggtagaaatt ttgaaagggt 420
agttttaaaa attatttttg tttttttttt gttttttttt ttgttatttt gataagttat 480
tttattttga tttagttttg gattattttt tattttagtt ttaattatta taattatttt 540
gagaaaagtg ttaatagtgt aggaatggga gaaaagatat tttatttagg tatttgattt 600
attttagtat taaagtgata aaaaaataaa ttaaaaggta agtggatttt ggtgtttatt 660
tggtttttta tttgaaaaaa atttagtgtt taagtagttg taaggagagt tttttagaga 720
agtgggtttt ggtagtggtg gggaagtgtt tttaaatggg tagaatagtt tttttgtgtt 780
gggagagttg tgtttttgtt tgggtgttgt aagttttagt gtaaagtgtt tgtttgttgt 840
tatggataaa gttttgtgga tgtgataagg gttgtggatt gttggttggg ggattagtgg 900
gagggttggg ttagaggtga agttttttat ttgttttgga tttttttttt taggatgttt 960
atagtgtagt tttgtttgtt tggttttttt attttagttg gttgtttgtt tgtttttttt 1020
gttttttgtt ggaattatta tagtgagtta gataaagttt tgaaaattgg tttttatatt 1080
tagtgtgatt tttttttttg tttttttttt tgttaagttt ttgagaagtt ttgttttttt 1140
tgaggtagga ggggagttag ggatggttgg ggtttggtgg tggtggttgt gagtagtata 1200
gtttgggggt ttttagttgt ttagattttt gtattataaa gggttggtgg gtggagatta 1260
gtgagagagg attttttttt ttttttttta tgttttttgt tttgggaatt tgggaggggt 1320
gtttatgggg agggtgggga gggtggggaa ggtggggagg agatttagtt gggtagttga 1380
gtattttagt tttaggatgt aaatagagta agagagttgt atgaattaat tatgtgtgtt 1440
tattattttt ttttgttttt ttttaaattt tttttttttt tttgttgttt ttttgtagta 1500
gtattgtttg ataaattgta tttttgtttt gtgagtataa attattgtag gtggaatagt 1560
tgttttttat attgagaatg tattgtgtgt ttattgttat gttatgtgtg tattaggttg 1620
tagggtgttt tgttaaggtt ggggaaaggg ttgtgttttg attaagagtt ttagggagag 1680
tggaggaaag aagggtatta atgggtgtgt gtttagagtg tgggatgtta gtgtagatag 1740
ggaggaatga tagaggtata aggaggaaaa tgatgtttag aatgattaaa ataatttagt 1800
aatgtattgt tatgtatatt tggagagtgt gttatgaata aatttttatt gtatttgttg 1860
gggggagtta tgtattatgt attatgtatg tatagttatt tggattggat attttttatt 1920
tagattgagt tttttaattt gttgttaaag tagtagatat tggttttttt gttttgtttg 1980
tttaggtttt tgtggggttt tgtgtgggag gtgtttgttt agttttgaga tgtgtttgtt 2040
tgttttagag ttgggttagg gtgagaggga ggttgtttgt tttttgttag tttgtatttt 2100
attgttttat ttttatgttt tttatatgga gtttttaatt ttttagttag gttttagaag 2160
agataaattt tttttgtgtt ttgtggtgtt ggtttgtaat agttttgggt tgttgggttt 2220
gggagaaatt aaaggtgtta gatgggagaa tatgggaggg gtagggggta tttgaattgt 2280

gggattggat tttttaaaag gggtaagtgg agagtttgtg gattgagttg ggggagttag 2340
tagagatttt tgtgaaaaaa attgttaatt tttttttttt tggataaatt ggatgtttaa 2400
ttttttttta ggtttttttt ttttttttat tattggaaat gtggttatgt ataaaaaatg 2460
tattgatttt gattaaagaa gaggagagga gtattatttt tgtgtttttt tttggggggg 2520
gggggnagtt ttgagttaat taaaatttgg ttgtttttta ggtattaatt ttttagttta 2580
ttgatttata attttttttg aatattagtg aaagtgtatt gtatgtaatt tgtaaatgtg 2640
tatttatatt ttatttatgt tttgatttna gttagttgtt tagttttata tatgatttgt 2700
ttgttttttg aaatgattta tatttaatat ataatgtata tttttttggg attttttatt 2760
ttgtgttatt ttatggtatg aaaaataaaa aatatttttt ttatttttgg ttttttattt 2820
aaaggtattt taagtaaatt tttttttttt tttattattt ttaaaagaga aaataatttg 2880
ggggaaaggg g 2891

<210> 331
<211> 1092
<212> DNA
<213> Artificial Sequence

<220>
<223> chemically treated genomic DNA (Homo sapiens)

<400> 331

aagttttttt tttattattt aagaaggtat ttaggttttt ttgtgttagg ttttaggtaa 60
agtgttggat tatttagtaa ttgggtttag tagtaggatg gttttagatt gaggttttag 120
ggttaaagga ttattttttt ttttagtgtt ggtttgggaa aggtaagttt tgggtgggag 180
tgtatgttgt gtttttgaag tttggttttt ttgttatgtt tattttttgt ttttattttg 240
tgttttttta ggtttttttt tggtgaattg gatgttttgt tagtttttta ttttgtgttt 300
ttggttgtgg tttgggtttt ttgtaaagtt gttgttattt tggagggttt agttagtggg 360
tttttggagg ttggttgggt aggtgtggtg tgtggtagga gttgggtgtg tatggttatt 420
gtgtgtggag gagatattgt tttgttgtga tgggggtttg gggtgttttt ttatgttgta 480
ggtaagtggg gtggtggttg tggtatttgt ttattgggag tttttttttt ttttttttgt 540
tgttgttgtt ttgtatttag tttgggggag gatagaagaa aaaggaggta gaatggattt 600
ttttggtttt tttttgtggt tgggggtggg ttagggtggg ttgtgttgtt taggtagttt 660
tgttgtgttg ttaggtagtt tggttgttgg tgtgggtgat gttggtgttg gggtgggagt 720
tgtgagggtg ggaggttttg ggggtgtttt gggatgtgga gttagtaggg ttttgatttg 780
aaaaatgatg gtaaagtgtg tttttgattg tttttgagta ttttatattt tttagattta 840
gggtgttttt atttttagtt ggaagtttag tttagagtaa tggtgttatt aaaaggggtg 900
tgttggatgt gaaaatattt tttggaagta tttataagtt tgtaggaaat atgttttttt 960
tattttttta ttttgttttt tttattattt attttaagaa gtaatagaat ttgtgtagag 1020
tgtgttttaa gttatattgt atgtttattt ttgtttatgt tgaatttggt gtatatttgt 1080
gagaataagt tt 1092

<210> 332
<211> 1092
<212> DNA
<213> Artificial Sequence

<220>
<223> chemically treated genomic DNA (Homo sapiens)

<400> 332

aagtttattt ttataagtat atattgagtt taatataaat aaaaataaat atatagtgta 60
atttaaaata tattttgtat aggttttgtt gttttttgaa atgggtaatg aagggagtag 120
agtaagaaaa taaggaaaat atattttttg taggtttata aatattttta aagggtattt 180
ttatatttaa tatatttttt ttagtggtat tattgtttta agttgggttt ttagttggga 240
ataaaggtgt tttgggtttg aaaggtgtga ggtgtttaga agtagttaag aatatgtttt 300
gttgttgttt tttaagttag aattttgtta attttatgtt ttggaaatgt tttagggttt 360
tttatttttg tggtttttgt tttagtgttg gtattgttta tgttggtgat taggttgttt 420
agtaatatgg tagggttgtt tgggtaatgt ggtttatttt ggtttgtttt tgattatagg 480
ggaaggttaa ggggatttat tttatttttt tttttttttg tttttttttg agttggatgt 540
agagtagtag tagtaggaga aggaagggaa agtttttggt gggtaggtat tgtagttgtt 600
gttttgtttg tttatggtgt gaaggagtgt tttgggtttt tattgtggta gggtagtgtt 660

ttttttatgt gtggtagttg tgtgtgttta gtttttattg tgtattatgt ttgtttggtt 720
agtttttggg agtttgttgg ttgggttttt tgggatggta gtggttttgt gagggatttg 780
ggttgtggtt gaggatgtag agtaggagat tgatagagta tttggtttat tgggagaaga 840
tttggaaagg tgtgggatgg gggtaggaag tgggtgtggt gagggagtta ggttttgggg 900
gtgtggtgtg tgtttttgtt tggagtttgt tttttttgga ttagtgttga ggagaggagt 960
ggttttttgg ttttgggatt ttaatttgag gttattttgt tattgaattt aattattggg 1020
tagtttagta ttttatttgg ggtttagtat agaaagattt gaatgttttt ttggatgatg 1080
aaggggaagt tt 1092

<210> 333
<211> 5203
<212> DNA
<213> Artificial Sequence

<220>
<223> chemically treated genomic DNA (Homo sapiens)

<400> 333

agggaattta atgttttttt ggggtagaag ggggtagtgt gtgtgaggtg gaaggatagt 60
tatttttgtt gtataaattt ttttgtgggt tgtgttgtgt gtgtaggggt tggatgttat 120
ttggtgttag agttgggttt gaggagtggg gtttggttgg gaatgagggt tgttagtttg 180
ggtggtgtta tttgttgttt tattttttat tttttttttt tttgtgagga attagaggta 240
ttgggttgtg tgggttgaga atttttaaat tttttttggt tgtatttggt tgagttaatt 300
tgtagttttt gttagttttt ttgttttttg ggtagtggtt ttttttgtta gttaggtttg 360
gaagttattt tttgtgagat tgggttagga agtgatgaaa agtgttgaat tgtttttaaa 420
ttgaaaatat tttttttttt ttttttggag atagtgttga taaatatatg ggattttggt 480
ttttgatttt tggttgttgt ttttgttttt ttgttgttaa taaaatttgt attgagtttt 540
ttagtgtttt gattaatagg tagattaatt tttattgggg ttgggaatag tttgttttaa 600
ttaatgttat tttaaaagtt tgttaggatg tgtttagttt tggttttttg gatgtttgag 660
tgagttagtt atgatatttg tttttttttt ttttttagtt ttaaattttt tagtaaagtt 720
tttagttttt gtttgtttgt ttttaggttt ataatgggat aaattgtagg attgggttgt 780
tggtttttag aataggtagt ggtttagaga ggggatattt agggagaagg gattgaggtg 840
gttttgattt gtttgttttt tttttgaagt tgaatttttg ttttttgggg gtagaggtta 900
tagggaaaag agagatgggg ggattgttat agggtgatga aatggaaagt aaattttttt 960
aggaatttta tttgtaaaaa ttggagagtg ttgttgattt tagttgtttt atatgttagg 1020
agttttaaat ttaattttta tttggataat agtatggagg gattgggagg taaagttttg 1080
ggagtttgat ttgttgggtt gttaggaatt tttttgttgg gagattttat tggataatgt 1140
tattttaaag gtattttggg ggtgggtagg gagaagatag agagagaaag agagagagag 1200
agagagagag agagagagag agagagatag gttggttttt tagagtgaat ggaagaggat 1260
tgaggattga atttagggag tttttagtat tggttatttg gtaggaggtg aggaagggtg 1320
agggtattgt ggtttttttt taagttgagt agtggttata gtttgaaggt gttatggggt 1380
ggtttgggga tttttttggt ttttttaggt tttgggtttg gtgaggttgt tgggttagtg 1440
ggaattggta ggttgttgga gaaaggagag ggataaaggt ttggaagtgg gggaggtatt 1500
agggattggg tggttgggat gggagttttt gttttagggg tttattggat ttgtttgtta 1560
gttttgtgga taagggattt tattttttta gggtttgtaa aaagagagat gtttggggta 1620
ttataaagtg atgtaggtgt ttgttaagat tttttttttt taaaaaatgt tagtttttat 1680
tttttttagt gtgttggtgt tgtgtttttg tttgattggg gaaagagaga gtttggaggt 1740
gttagaattg ttagaatttt ggatgggagg tttttggttt tgaagttggg agttggaggt 1800
attttttttt agaagtgggt atttttttgt ttttttgaag gtttgatggg gtgggtatag 1860
gggaatgtta gggattggtg tgggtgagat gtttgtttta ttttatgttg atgtattaat 1920
attggttaga agttttgggt aggttttggt gagttatggt tttttagtgg gatttttttt 1980
atgtagatag ttttgttatt gggtgggatg attttggtgg tttttgtggg aatgtatttg 2040
ttggtttgag aggttttttt tttggttata gggggtggag ggtagggggg aataggggtt 2100
tgtggggagg tggggaaagg agatagaatt taaagtgtaa tttttgttgt ttttttgtgt 2160
tttggagggt agtaagtggg ttttgggtgt gttggtagtt gtaattaaaa tggtgatttg 2220
tgagttgttt tggttttgta tatatttttg gtaattgtgt ttgtatttta gttttggttt 2280
ttatattttt aggttgtttt gtttttagag ttggtatttg gtagagggag tgaggttttt 2340
tatgaggtag aatagtattt ttattttttt tgagattagt ttagagagaa tgaggaaatt 2400
ttatttttat tttttttttt ttaaattttg aaaatttggt tttattttgg ggttgaattt 2460
ttttttttaa atattgatat atgggaagta gttaagtttt ttttgttttt tgtttgtgtg 2520
gaggagtttt taggagtttt ttttggagtt ttttttttta tttaatgatg ttagtggtat 2580
ttattttttg ttttttgttt tttttgattt tttgttaaaa tttttttttg tagtgagatg 2640

ggggttggag gggtgggaga aggagttgat ttaaagagtt agtgttttgg ggggaaatag 2700
taataagatt atggttgttt ttttttgagg taaagtttaa gaaggtgtta atttatttgg 2760
ttgttaattg agaggttgta aaaatgatat ttgtttttag tttggataga attatttttt 2820
ttttttttgt ttttttgtaa gttttttgtt tatgtatagt ggatttgagt tttgagttta 2880
agttttttta atgtttgttt ggaaagtaaa aggagagaga aagagaaagt tgatttttgt 2940
tggtttggga tttagggttg tggttttatt tgttggtatt ttttgaaagt tgtgtttttg 3000
tgaaatgaaa ttttgtttag gaggttgtgg atttggatat ttggtgttat tttttttatt 3060
tttgatttag gttttttttt ggtgttgtga gtttttgggg aagggttaga gttggtagtt 3120
ttttttagtt tggggagggg agagggttat gtgattttat ttttggttag ggttggggag 3180
gtttttgttt tttgggagtt ttgtttgggt tttttggttg tagggttgtt gggttttagg 3240
taggaatgag agggtgaggt ttatatgtgg tttggtggtt tagggtggtt tgtagtgttt 3300
ttattgtttt ggttgttagg ggttgtggtg atgtggttag ttagtagtga gtttaggttg 3360
tgtagatttt attgatgagt tttgattttt agtatttttt ttaagttaag aagagtttag 3420
tgtatttttt ggttgtttta ttttagtttt tttgttttag ttttttagtt ttattttttt 3480
ttgttttgtt ttggggtgtg tatagtagtt taggtttttt tttttttttt tggttttgtg 3540
gtttgaagtt gttgagagag tttgggatag tgtaggatta ggtagttgtt tgtttttttg 3600
ttattttaat tgtaggtttt gaggggtgtt tttggagtgt attgaggtgt gttttaattg 3660
tgtggtattt aataaatgga tttttggtgt gtggttagaa gagaaaagtt atttatttat 3720
tttttttttt ggttttttgg taatagttga aggggagttg tttttgtgga ttgagtagat 3780
ttaggagagg gagttgtggt gtggagatat atgtattata tatagatgat tggtggtata 3840
tatgatatat gttgatatat tgatattgtt agtgggatat atatatatat atatatatat 3900
atatatatat atatagagag agagagagaa ttttttttag tattggttat ttgttttttt 3960
atttaggttt ttattttttt ttttttttta ttttttttgg tttttttttt ttttgggtgt 4020
tgtgaaaagt agttgtattt agttaataaa tggtatgtgg gagaagttgg tgagtgtttg 4080
gtgaggattt tttagggttt tttataagaa ttttttgtat ataaagtaag tggtgtgttt 4140
atttgggttt ttttagttag agttgtgttt ttgttttgtt gtgtattgtg gtttttgaaa 4200
ggagaaagga gagaagaaag ggtggggaga gtggggtgga ggatttggat aggttttgga 4260
ggtttgggtt ggggaggttt ttggttttgt ttagtttttg gtttggtaat ttttttttgg 4320
tttaggtttt gttgtggttt ttgtagttgg aatggagttg ttaggattta gtgatgtttt 4380
tgtttttttt tttttttttt aaggggttag gtgggttggg gtgtggttgt tgttgtgttt 4440
tgtgttttgg ggttttggtt gggatggggt gggggtgggt gggggtgggg tggtaggtta 4500
tgttgttttg gagttggtaa gaaaggatag tatagaaatt tgtatttttt gaggattggg 4560
agttttgagt ttagtttagg gggagtgggg gtgtgatttt taatttagaa atttttattt 4620
gattgtttaa gtttgtggta gtagggtggg ttgtgttgaa ttttggtgtg tgtggagtgg 4680
ggagatgtag gtgagtgtta gagtttgggt ttgggggttt tgtgttgggg agaggagttg 4740
ggatttattg gtggagttga aaataagtgt atttatattt aaataaatgg attaattgta 4800
ttaggtgggg agagggagta tttaattggt tggtgtgagg ttttggtgtt gttttgtata 4860
aagtaatatt ttgtgtgaga gtgagtggtg tatttgtatg ttgtggagtg attagtgggt 4920
ttgaaaaggg aattgtggtt tggttttatt ttttgttttg gtttaggtgt aggaggaagt 4980
gttttgttgg aggatgatga tagaggttag gttttgttaa tgggttagtg aggagtggtg 5040
gaggtgaggt tgggtgttgg tatatatata ttaatatatt tgagttatta ttaattagta 5100
taggtgtgtt ggttgtagtt atttttttta tttatatttt ttatttttta ttttttagtt 5160
gttgatagtt tattttattg ttaatttttg tttttttttt agg 5203

<210> 334
<211> 5203
<212> DNA
<213> Artificial Sequence

<220>
<223> chemically treated genomic DNA (Homo sapiens)

<400> 334

tttgggaagg agatagagat tgataataaa atgggttgtt agtggttgga gagtgagaga 60
taaagagtgt gggtgaggga agtggttgta gttagtatat ttatgttgat tggtgatggt 120
ttaagtgtgt taatgtgtgt gtgttggtgt ttggttttgt ttttattgtt ttttattggt 180
ttattagtga agtttgattt ttgttattat tttttagtaa aatatttttt tttgtgtttg 240
aattagagtg ggaaatgagg ttgagttatg gttttttttt taaatttatt aattattttg 300
taatatgtaa atgtattgtt tgtttttata taaaatattg ttttatgtaa agtagtgttg 360
gggttttgtg ttagttgatt ggatgttttt tttttttgtt tggtgtaatt ggtttgtttg 420
tttgaatatg aatatatttg tttttggttt tgttggtggg ttttggtttt tttttttggt 480
gtagggtttt tgagtttggg ttttggtgtt tgtttgtatt tttttgtttt gtgtatgttg 540

agatttggtg tggtttgttt tgttgttgtg aatttgagtg gttaagtgaa ggtttttggg 600
ttgggggttg tgtttttatt ttttttaagt tggatttggg atttttagtt tttggagggt 660
gtaagttttt gtgttgtttt tttttgttaa ttttaggata gtgtggtttg ttgttttgtt 720
tttgtttgtt tttattttat tttagttggg gttttaagat atagagtata gtggtggttg 780
tattttagtt tatttggttt tttggaagaa gaggaaaggg gtgggagtgt tattgggttt 840
tggtagtttt attttagttg tggaggttgt ggtgaagttt aggttgagag gaggttgttg 900
ggttgagaat taaatgaggt tagaggtttt tttagtttaa gtttttaggg tttgtttaaa 960
ttttttattt tgtttttttt gttttttttt tttttttttt tttttttgga agttgtggtg 1020
tgtagtggag tagaggtata gttttggttg gagaggtttg agtaaatatg ttatttattt 1080
tgtgtataga gggtttttgt gaaaagtttt gaagagtttt tattaaatat ttattaattt 1140
tttttatgtg ttatttgttg attaagtgtg gttgtttttt gtagtgtttg agaggagggg 1200
aagttagggg agataagagg ggagggggag tggaagtttg ggtggggggg tggatgatta 1260
atgttgggag ggattttttt tttttttttg tgtgtgtgtg tgtgtgtgtg tgtgtgtgtg 1320
tgtgtgtttt attggtgatg ttggtatgtt agtgtgtgtt gtgtgtgtta ttggttattt 1380
gtgtgtggtg tgtgtgtttt tgtattatga tttttttttt tgggtttgtt tagtttatgg 1440
aggtagtttt tttttagttg ttgttagaaa attggggagg aaagtaagta aatggttttt 1500
tttttttgat tatatattag aagtttattt gttgaatgtt gtatgattag gatatatttt 1560
agtatatttt aaaggtgttt tttggagttt gtagttaagg tgataggaga gtgagtggtt 1620
gtttggtttt gtgttgtttt gagttttttt ggtggttttg ggttatggag ttgggaagga 1680
gaaggaaggt ttgggttgtt gtatatattt taggataggg tgagggggaa tagggttgaa 1740
gagttgaggt agggaggttg aaatgaagta gttgaagggt atgttagatt ttttttgatt 1800
tagggaaagt gttgaaagtt agagtttatt aataaaattt gtgtgatttg aatttgttgt 1860
tgattggttg tgttgttgta gtttttggta gttgggatag tgaggatgtt gtaagttgtt 1920
ttgggttgtt gggttatatg tgggttttat ttttttgttt ttgtttggga tttagtagtt 1980
ttgtgattaa ggagtttggg tagggttttt gggaagtaaa ggtttttttg gttttagtta 2040
gaggtggggt tgtataattt tttttttttt ttgggttggg gagggttgtt ggttttaatt 2100
tttttttggg agtttgtagt gttgggagga aatttgggtt agaggtgaag gaggtggtgt 2160
tgggtgttta ggtttgtggt tttttgggtg aggttttatt ttgtaggagt gtgatttttg 2220
gaggatgttg gtaagtgggg ttgtggtttt gagttttaga ttagtgaaag ttggtttttt 2280
tttttttttt tttttatttt tttaagtgag tattaaagaa gtttggattt aagatttaag 2340
tttgttgtgt atgagtgagg gatttgtggg ggggtggggg ggggggaagt gattttgttt 2400
gaattggagg tgagtattat ttttataatt ttttaattag tgattgagtg gattagtatt 2460
tttttaagtt ttgttttgga aagaagtagt tgtaattttg ttgttatttt tttttagggt 2520
attggttttt tgaattagtt ttttttttta tttttttaat ttttattttg ttgtggagaa 2580
aagttttaat aaaaaattag aaaaggtaag gagtgaggag tgaatgttat tgatgttatt 2640
gggtggggaa gggggttttg ggaaagattt ttggaaattt ttttgtataa ataaaaaata 2700
aaaaggattt ggttgttttt tatgtgttaa tatttgggga ggggagttta attttagaat 2760
gaggttgagt ttttaaagtt taaggagaga ggggtgagag tgagattttt ttgttttttt 2820
tggattggtt ttaaggaagg tgggggtgtt gttttatttt gtggggagtt ttattttttt 2880
tgttaagtat tggttttggg ggtgaagtgg tttaggggtg tgggggttga gattgaggtg 2940
tgggtgtggt tgttagaggt gtgtataaaa ttgagataat ttgtaggttg ttattttaat 3000
tgtggttatt aatatgtttg aggtttattt gttgtttttt aagatgtggg ggggtggtgg 3060
ggattgtgtt ttaggttttg tttttttttt ttgttttttt ataggttttt gttttttttt 3120
attttttgtt ttttgtggtt agagggagga ttttttaggt tggtgggtgt gtttttgtgg 3180
gggttgttgg ggttgtttta tttggtagtg gggttgtttg tgtggaggga gttttgttgg 3240
gggattgtgg tttgttagag tttgtttaga gtttttagtt agtattgatg tgttaatatg 3300
aggtgaggta agtattttat ttgtattgat ttttggtatt tttttgtgtt tattttatta 3360
ggtttttggg gaggtaaaga agtgtttatt tttgggggga agtgttttta gtttttagtt 3420
ttagaattaa gagtttttta tttggagttt tgatagtttt gatattttta aatttttttt 3480
ttttttggtt aagtagaggt gtagtgttgg tgtgttggaa agggtgaaga ttggtatttt 3540
ttggagaagg aaagttttgg tagatatttg tgttattttg tagtgtttta aatgtttttt 3600
tttttgtaga ttttgggaag gtgggatttt ttgtttgtgg agttggtaaa tgggtttagt 3660
gagttttaaa ggtaaaggtt tttattttgg ttgtttagtt tttggtgttt tttttatttt 3720
taggtttttg tttttttttt ttttttagta atttgttggt ttttgttaat ttggtagttt 3780
tgttgagttt agagtttaga aaagttaaga agattttaga gttgttttgt ggtattttta 3840
ggttgtggtt attgtttggt ttggggggaa gttgtggtgt ttttgttttt ttttgttttt 3900
tgttaaatgg ttagtgttag gggttttttg ggtttagttt ttagtttttt tttgtttgtt 3960
ttgggaggtt agtttgtttt tttttttttt tttttttttt tttttttttt tttttttttt 4020
tttttgtttt ttttttgttt atttttaaaa tatttttaaa ataatattgt ttagtggagt 4080
tttttagtaa agggattttt agtagtttag tgggttagat ttttaaggtt ttgtttttta 4140
gtttttttat gttgttgttt agataggagt taggtttaaa atttttgata tgtaggataa 4200
ttgaaattag tggtattttt tgatttttgt aaatagggtt tttagaaaag tttgtttttt 4260
attttattat tttgtgatga tttttttatt tttttttttt ttgtaatttt tgtttttaag 4320

aggtaagagt ttagttttaa aggaaaagta agtaagttaa ggttatttta gttttttttt 4380
tttggatatt ttttttttaa attgttgttt attttaaagg ttaatgattt ggttttgtga 4440
tttgttttgt tgtagatttg ggaataggta ggtgggaatt gggggtttta ttgggggatt 4500
tgaggttggg gagggggagg gagtaaatgt tatggttggt ttgtttaagt atttagggaa 4560
ttgaagttaa gtgtattttg atggtttttt aaaatgatat tgattaggat aagttgtttt 4620
taattttagt aagagttaat ttgtttgtta attaaggtat taaggggttt aatgtgagtt 4680
ttattagtga taggagaata gaggtggtag ttagggatta aaagttggga ttttatatat 4740
ttgttagtgt tatttttaaa aaaaaaaaaa aaagtatttt taatttgaaa ataatttggt 4800
gttttttgtt attttttaat ttagttttat agagggtgat ttttaaattt ggttagtggg 4860
gaaaattgtt gtttggggga tagaggggtt gataggaatt gtgggttggt ttagttgaat 4920
gtggttgggg agaatttaag aatttttagt ttgtgtggtt tgatgttttt gattttttat 4980
gagaggaaag ggaatgaaaa atgaagtaat aaatgatatt atttaggttg gtagtttttg 5040
tttttggtta gattttgttt tttaggtttg gttttggtgt tgggtggtgt ttagtttttg 5100
tatgtgtggt gtggtttgtg ggaaagtttg tgtagtgaga gtgattgttt ttttgttttg 5160
tgtgtgttgt ttttttttgt tttggagggg tgttgggttt ttt 5203

<210> 335
<211> 8085
<212> DNA
<213> Artificial Sequence

<220>
<223> chemically treated genomic DNA (Homo sapiens)

<220>
<221> unsure
<222> (2545)
<223> unknown base

<400> 335

gttttatgat ttaattattt tttaatattt ttttttatta agattattat attggtgatt 60
gggttttgat atatagattt ggggagaata taaatattta tattatagta tttattaatt 120
tatttagtta ttttattgtt aggtatttat ttagaaaaaa tgaaaatata tgtttatata 180
aagatttgta taatattttt atagtagttt tatttataat attaaaaaaa ttggaaataa 240
tttagatatt tatttatagt taaatggata aataaattat agtatataaa taaaaagaat 300
aaaaagaatt aattattgat atatattata atttagttga attttaaaat aattatgttg 360
agtgagagaa attaaatgaa aaaaagatat attatattat atataatttt aagaaataaa 420
atttaattta tttggtattt agatttttat ttttatatta gttttaaata aaaagtttta 480
aatttttttg gataaatggt tgattttatg attggggtag gaaatatata agatgagttt 540
gatgagtttg tagtattttg taatattaga aagtaggaaa gtttatatat atatatatat 600
atatataata tagagagtgg gttaaggagt ataagagtta attgaaaaag tttttagtgt 660
taaaaattgg aaaaatttga gtaataaagt atttatgaat ttatataaag aaaagattga 720
ataaagaagt aaatggggga gaagaagtaa atttttttta tagaatttta aataaattat 780
gtagatattt tattttaaag gagggttagt ataatttttt atttttttat atttttttaa 840
gtgatttata atgatttttt tttaaagagt atagagtatg taaagaggga aaaaagagta 900
attatgtaat ggagaaattt gataaatatt attttagtta ggtgatggag aataatatta 960
atagttataa gttatgttga taatatgtat tttttatatg atgtaataaa tatagtatat 1020
tatttttttg atttttattt tgttaaaatt taaaatttta gtttatttat gaggatatat 1080
taaattttaa tagaagggta ttttgtaata tattttatta gtatttttaa ggattgttaa 1140
ggttattaaa tataaggaaa atttgagaaa tttttatagt taagaggagt ataagatatt 1200
ataataatta aatgtggtgt tttggatgga attttggaaa ggaaaaagga tattaagtga 1260
aaatgaagaa aatttaaata aattatgaat attagttaat aataatttat tagtattagt 1320
ttattaattg taataaatgt attttattaa tttaaaatgt taatagagaa aattgtataa 1380
atgatatatg agtatttttt atattatttg tttaattttt ttgtaaattt aaaattgttt 1440
tttaaaagta aaatttatta ataaaatatt taaagtaaat agatttaaaa ggtatatgta 1500
aatgtataat ttaatgttta ataaattaga tataaatatt tttaaaaatt taatatttgg 1560
tgatagaaag tagattagtg gtttgttaga aagtggtgat tagtattagg ggaaggggat 1620
gggatagaat agtatgaagg gatttaaaaa atggtatgag gaaatttttg gaggtaaagg 1680
gtatgtttat tattttagtt ttataggttt aataataaat tatataattt aaatatatgt 1740
agtttattat atgataatta tattttgata aagtggtttt tttttttaaa ttatagaaaa 1800
aatattttta tatttaaatt ttttttttta aaaatagttt ttaggaatat ttgaagttag 1860
ttttattata tttgaattta attagtgaat tttgtttatt taaagattta tatatttggg 1920

aagtttttgt gttaaaaatg ttaattattt agtattaaaa tttatggaat aatggtgatt 1980
taggatatgt tggatatttt tattgttgtt tttttatttt aagattttta attttttgga 2040
aataattttt ttagatattt tgataaggga attattataa ttaattaaaa ttaagttaaa 2100
tatagtttgt tagatttaaa atgaggttaa gtgttaattt tatagtagtg attggaaaat 2160
taaattatat ggtatttgat atttaaaatt atatttttaa gaaggttgtt atttgatgtt 2220
ttgggtttgg gttatagtat ttaattttat aatattgggt aattttgtgt tttagttttt 2280
ttaattatga aattgggata atagtattta ttttgtgaga ttgttgaagg attatagtta 2340
atataggtaa aaggtttata agattatttg gtaatgttgt tttgatttta taaattttat 2400
atattgtgtg gtattagtta ttaggaaaat ataaataaat tagtgagata ttattttata 2460
tatttggggg gttataataa aaaagataga taaaaaatgt tgttaaagat gtgaagaaat 2520
taggattttt atttattgtt ggtgnaaatg taagaagtat aattatttga attatagttt 2580
ggtagttatt ttaaagatta tatttagagt tgttatatga attagtaatt ttatttttat 2640
gtatatattt aggagaaagg aaaatatatg ttgtataaaa atttgtatat gaatgtttat 2700
tagtaggatt ttttatggta gttaaaaggt gtaaataatt taaatgttta ataattgata 2760
gagatggata aataaaatgt ggtttattta tataatgaaa tattatttgt taataaaaag 2820
gagtgaagga gtgaagtatt gatttatgtg gaatatggat gaattttgat ttaaaatagt 2880
taaattatag gtataaaaag tatattagtg gttgttggga gatttaggtg gaaatgtgga 2940
ataattgtta gtgggtattg agattttaga gttatattta tgttataaaa ttaatagtgt 3000
tgatggttgt ataattttga gtatatgaaa aattaatgaa ttgatatttt gagtgagttg 3060
tatgatattg gaattatatt ttaataaagt atggtaattg ttttaagata ggttggaaag 3120
agaaagtttg aaaataataa taatgatatt aataaattag tttatttttt tagttttata 3180
tatttttgtg tttatatttg tttttgtttt atttataatg gtttttttgt agttgttata 3240
ttatattttg ttatttgatg tttggtgaat attttatatt tgttttttag aatttttttt 3300
attttttttt tatttgttta atttttatat atttaaaatt aattagagta aattatttat 3360
tagaataatt aattttaaat tttagtaatt taatatgata aaggtttgtt ttttatttat 3420
atagtttttt tttagatgat tgaggggttt aggtttttta tttttagtgg tttttttatt 3480
ttttggagtt ttttgtattt tttatatatg gttgagataa attatgagtt attagtatag 3540
ttagattttg aggttttata agaaaatttg taaattattt attttgtttt gaataaggta 3600
tatttaagat gatgttaaaa tatttaatgg ttttgggtta aatatagttt atgattgtgt 3660
atttaaaata tatattgtaa tatttttttt tttttttatt gattttatga atttagtggg 3720
gatttatttt ataagtttaa agataattat tttttagatt aagaatattt agggtaaaaa 3780
gtattgttta atatttttat tgaggatgtt atgatgtagt atattgtata agttggagtt 3840
aaaggaaatt ttttttaaag tgttatttat taaaaattgg aatatatttt ttaagataaa 3900
ttgaagtgtg gtatataata tttaaatttt tattatagat atagaggtgt tattattttt 3960
tatttttaaa tttttttgtt atgttgagga tatttaagag gagtaggata tgttggttgt 4020
agtaggagaa atttgaaagt atttattttt atggaattta taagggagag aattttttat 4080
tttagtattg tttttgatat atttattatt ttaaaagata atgtagttaa atgttttttt 4140
ttgtgttaaa tttttataaa attgaaattt taaaatggtg ataaaaattt tatttttgat 4200
agaatttatt tattttttta attagatagg gtataatttt taatttgtaa aataaaatgt 4260
aatatgttta tgaggtttta ttttaaagaa tttgttattg agagtagtat ttagaataat 4320
gggtggaaat gttaatttta gagttttaga ttttattggt aattggggta gggaggggtt 4380
ttgggtgggg tttttttaga ggaggaggtg ttgttagaaa gttgtttggt tagtttatag 4440
ttgttattaa ttggggtaag ttttgttgta tttgtgtgtg tgggtggtat ttttaatgag 4500
aattagtttt atttgttatt tgagtgaaat ttataatttg aggtggttag tgtttttgta 4560
ttattgggat ttgagatttt tggagatgat tgttgtttgt agtatggagt tagtagaagt 4620
ttgatttttt ttgggaatgg gttgtattga gaggtttgat tagttttagg gttttagtga 4680
gggggtagtg gaatttagtg agggattgag agttttatag tatgtatgag tttgatgtta 4740
gagaaaaagt tgggagataa aggagttgtg tgttattaaa ttgttgttgt agttgtagtt 4800
atttaagtgt tggatttgtg agtattttgt gtttttagtt tttggataga agttggagaa 4860
tttttttgga gaattttttg agttaggaga tgagattttt taataattat tatttttttt 4920
tgtgtttttt atttgttgtt tgttgggata aatgatagtt atagtttttt tgatgatagg 4980
atggaggtta agggtaggag ttgattagtg ttgttttttt ttgtttttga tttaggaggt 5040
ggagattttt ttggtttagt tatatttaat atttattttt tttttttttt gtttttatat 5100
ttttgaaatt tttttttttt tttttttttt tttttttttg gagatggggg aggagaaaag 5160
gggagtttag ttgttatgat tgagttgaag gtaaagggtt tttgggtttt ttatgtggtg 5220
ggtggtttgt ttttttttga ggttggattt ttattgttgt gttgtttagt tgtaggtttg 5280
tttttgggga gttagatttt ggatattttg tttgaagttt tggttatatt tatttttttg 5340
gatgggttat tttttttttg gttttgttag ggataggatt tttttgatga aaagatgtag 5400
gattagtagt tgttgttgga tgtggagggt gtatatttta gagttgaagt tataaggggt 5460
gttggaggta gtagttttag ttttttagaa aaggatagtg gattgttgga tagtgttttg 5520
gatattttgt tggtgttttt aggttttggg tagagttaat ttagtttttt tgtttgtgag 5580
gttattagtt tttggtgttt gtttggtttt gaattttttg aagatttatt ggttgttttt 5640
gttatttagt gggtgttgtt tttgtttatg agttggtttg ggtgtaaggt tggagatagt 5700

tttgggatgg tagttgttta taaagtgttg ttttggggtt tgttattagt ttggtagttg 5760
ttgtttttgg tttttgagag tttttattgg tttggggttt tagtgaagtt gtttttgtag 5820
gttgttgtgg tggaggttga ggaggaggat ggttttgagt ttgaggagtt tgtgggtttg 5880
tttttgaagg gtaaattttg ggttttgggt ggtgtggtgg ttggaggagg agttgtggtt 5940
gttttgttgg ggtggtagta ggaggtgttg ttttggtttt taaggaagat ttttgttttt 6000
tagtgtttag ggttgttttg gtggagtagg atgtgttgat tgtttgggat gtttttgttg 6060
gttattatgg tgatggattt tatttatgtg tttattttgt tttttaatta tgttttattg 6120
gtagtttgta tttggtagtt gttggaagat gaaagttatg atggtggggt tggggttgtt 6180
agtgtttttg ttttgttgtg gagtttattt tgtgttttgt ttattttggt tgttgtaggt 6240
gatttttttg attgtgtgta tttgtttgat gttgagttta aggatgatgt gtattttttt 6300
tatagtgatt tttagttgtt tgttttaaag ataaaggagg aggaggaagg tgtggaggtt 6360
tttgtgtgtt ttttgtgttt ttattttgtg gttggtgtta attttgtagt ttttttggat 6420
tttttgttgg ggttattgtt tttgttgttg ttgtgagtga ttttatttag atttggggaa 6480
gtggtggtga tggttgtatt tgttagtgtt ttagttttgt ttgtgttttt tttggggttg 6540
attttggagt gtattttgta taaagtggag ggtgtgttgt tttagtaggg tttgtttgtg 6600
ttgttgtttt gtaaggtgtt gggtgtgagt ggttgtttgt ttttgtggga tggtttgttt 6660
tttatttttg tttttgttgt tgttgttggg gtggtttttg tgttttattt tgtatttggt 6720
tttaatgggt ttttgtagtt tggttattag gttgttgtgt ttaaggaggg tttgttgtag 6780
gtttatttgt tttattttaa ttatttgagg tgagggtttg ggatggggta tgtttagtgt 6840
gtttgggagt agtggttttg ttggtggtgg tggttgttaa tttttagttt tagttttagt 6900
gtattgttgt gttttttggg gtggttggag agggtgggta gtgggatata gtataggggt 6960
agttgttttt tttttttttt tttttttttt ttatttttgg ggatatgaag gtgggtgtag 7020
aatatattat ttttggggtg tgtttttttg aaagttgttt ttttgtttgt tttttaattt 7080
tttgaatttt ttagattttg aagtagaatt aattttgatt taaaatgtgt agtgttatat 7140
taggtttgtt gtagtttagt ggggtagaaa gtgtgtggtg agttgggggt tttatgaaat 7200
gttttttttt tagaagaagg atgtttatta ggagtgtttg ttttggagag gagttaaggt 7260
attgtttttt tgggaggggt gggatttgag aggtggttgg ttagaattga aagtagtatt 7320
attttaggga tttgaatatt ttagtggttt agttttttta agaattttaa gattaaaatt 7380
aagtttatgt gggaaatgtt taaattgtgg atttaaatgt ttgttattgt attgtattgt 7440
ttttttatta ttgtttgtta tttattataa ttttttttat atataggttt aaaaaatatt 7500
attttgtata ttgaagtaat ggaatgtaaa aaaagaatgt tttgtttgga attttatgtt 7560
gtgaataggt aaaatagtgt tagtgtattg gataatattt taaaatgata aatatatatt 7620
tgtttaagta agtaatgatt atagggttgt gttttaaaaa tttaaaatta aaatattgta 7680
aagtattatt gaatttttaa agttaaatta tatttgtttt gatttagtat atagatagga 7740
aggatataat attttatttg ttaaagatta aattgttttt atataaagag ttttgtagaa 7800
agattttttt ttaattgatt ttaatttttt aggatataat attatatatt aattattgtt 7860
tttttatatt ggtgttattg atgaatggtt aattatttgt aagtatggtg aatttagtta 7920
tggatagttt attattaagt ttagtttgta tgttttttaa gtgtatatat atagttttgt 7980
ttttaaaatt tttttttatt ttgttaatat tggtttaaga aatttttagt attagatagt 8040
ggtgtattta aaaataaatg gagtattttg ttttgtattt taagg 8085

<210> 336
<211> 8085
<212> DNA
<213> Artificial Sequence

<220>
<223> chemically treated genomic DNA (Homo sapiens)

<220>
<221> unsure
<222> (5541)
<223> unknown base

<400> 336

ttttgaaatg taaaataaag tattttattt atttttaagt gtattattat ttaatattaa 60
aggtttttta aattagtatt aatagggtga aaggagattt taaaaataga attgtatata 120
tatatttgaa agatatgtaa attaaatttg gtaatagatt atttgtaatt ggatttatta 180
tatttgtaaa tgattagtta tttattagta gtattaatat aaaagaataa taattagtgt 240
ataatattat gttttagaaa gttaaagttg gttaaaagga aattttttta taaaattttt 300
tatatagaaa taatttagtt tttgataaat gaaatgttat gttttttttg tttgtatgtt 360
gggttaaaat aaatatggtt tggttttaaa agtttgatgg tattttgtaa tgttttggtt 420

ttgagttttt aaaatataat tttgtaatta ttgtttattt aagtaagtat atgtttgtta 480
ttttaaagta ttgtttaata tattgatatt gttttgttta tttataatat aagattttaa 540
atagagtatt ttttttttat attttattat tttagtatgt aaagtagtgt tttttaaatt 600
tgtatataaa aaaaattgta gtgaatagta agtaataata agaaaatggt gtaatgtagt 660
gataggtgtt taaatttata gtttaaatat tttttatgtg aatttaattt taattttgag 720
atttttaaga aaattgagtt attgaagtgt ttaaattttt aagatggtgt tgtttttggt 780
tttggttggt tattttttaa gttttatttt ttttggggga atggtgtttt aatttttttt 840
taagataagt atttttggta aatgtttttt ttttaagaaa gaagtatttt ataaagtttt 900
taatttgttg tgtatttttt gttttattgg gttatagtgg atttagtgtg atgttgtatg 960
ttttaaattg gggttggttt tgttttggaa tttggaagat ttggaaagtt aaaaaataaa 1020
taaaaaaata gtttttaggg aggtatgttt taaaaatagt atattttgtg tttatttttg 1080
tgtttttaag agtgaggaga ggagggaaga agaagggagg taattgtttt tgtgttgtgt 1140
tttgttgttt attttttttg gttgttttgg ggagtgtagt ggtgtgttgg ggttggggtt 1200
gagggttggt ggttgttgtt gttaatggaa ttgttatttt tggatgtgtt gggtgtgttt 1260
tgttttgggt ttttatttta ggtagttgag atagggtggg tagatttgtg gtaggttttt 1320
tttgagtatg gtggtttggt agttgagttg tgggagtttg ttgaggttga gtgtagggta 1380
gagtgtgggg gttgttttgg tggtggtggt agaggtggag gtggagggta ggttgttttg 1440
tgggagtagg tagttgtttg tgtttggtgt tttgtagggt ggtggtgtga atgggttttg 1500
ttggggtggt gtgttttttg ttttgtatag gatgtatttt agggttgatt ttgaggagga 1560
tgtagatgag attgaggtat tggtgggtgt ggttgttatt gttgtttttt tgggtttgga 1620
tggggttgtt tgtggtggta gtgggggtgg tggttttaat gggaaatttg ggaaggttgt 1680
ggggttggta ttggttataa ggtaggaatg tggggagtgt gtggaggttt ttgtgttttt 1740
tttttttttt tttattttta gagtgggtgg ttggaagttg ttatagagag ggtatgtgtt 1800
gtttttgggt ttggtgttgg gtgggtatgt gtagttgggg aagttgttta tagtgattgg 1860
ggtggatgag gtatagggtg aattttgtgg tggggtaaag gtgttggtag ttttggtttt 1920
gttgttgtaa tttttgtttt ttagtagttg ttgagtgtgg gttgttaata aggtgtgatt 1980
gagaggtagg ataggtatgt ggatgaaatt tattattgtg gtggttagtg gggatgtttt 2040
ggatgattgg tgtgttttgt tttattaggg tgattttggg tgttgagaag tgggaatttt 2100
ttttggggat tagggtgatg ttttttgttg ttgttttggt gggatagttg tggttttttt 2160
tttagttgtt gtgttattta gagtttgagg tttgtttttt agaagtggat ttgtagattt 2220
tttggattta gagttatttt tttttttaat ttttattgta gtggtttgtg gagatggttt 2280
tattggggtt ttggattagt gagggttttt agaggttggg agtagtagtt gttgggttgg 2340
tgataggttt tggggtagta ttttatgggt agttgttgtt ttggagttgt ttttaatttt 2400
gtatttggat tggtttatga gtggggataa tatttgttgg gtggtggggg tagttggtgg 2460
atttttggga agtttggggt taaataggta ttaagagttg gtgattttgt aggtgggagg 2520
gttgggttgg ttttgtttgg gatttgaggg tgttaataga gtgtttaaga tattgtttag 2580
tagtttgttg tttttttttg ggggattaga attgttgttt ttagtatttt ttgtagtttt 2640
agttttggaa tatgtgtttt ttatgtttga tagtgattgt tggttttgtg tttttttgtt 2700
ggaggggttt tgtttttggt agggttgagg gaagagtagt ttgtttaggg agataggtat 2760
ggttgaaatt ttaggtaagg tgtttgaggt ttggtttttt gggaatggat ttgtggttgg 2820
gtgatatagt agtggggatt tgattttggg ggagggtggg ttgtttgtta tgtggggagt 2880
ttggggattt tttgttttta gtttagttat gatgattgga tttttttttt tttttttttt 2940
gtttttaggg aggagggaaa agggaaggag gagggggttt tgggaatata ggggtagagg 3000
gaggagaaag tgggtgttga atgtggttgg attggaggga tttttatttt ttgggttggg 3060
ggtgggggag ggtggtgttg gttagttttt gtttttggtt tttattttgt tgttagggga 3120
attgtggttg ttgtttgttt tagtgagtgg taagtgggga gtgtaagaaa aagtagtaat 3180
tgttaggaga ttttgttttt taatttgggg agttttttaa gagagttttt taatttttgt 3240
ttgaggattg gagatgtaga gtatttataa gtttggtatt tgagtggttg tggttgtgat 3300
ggtaatttag tgatatgtgg tttttttatt ttttgatttt ttttttggta ttaaatttgt 3360
gtatgttgtg aagtttttag ttttttgttg agttttattg tttttttatt aaaatttggg 3420
ggttagttgg attttttggt atagtttatt tttaggaagg gttggatttt tgttggtttt 3480
gtattgtggg tgatagttat ttttgaagat tttagatttt agtagtgtgg gagtattagt 3540
tgttttgggt tgtagatttt atttaaatga taagtgaagt tagtttttat tgagaatgtt 3600
atttatatgt ataaatataa taaggtttat tttgattagt gatagttgtg gattggttag 3660
atagtttttt aataatgttt ttttttttag ggaggttttg tttaaagttt ttttttattt 3720
taattattgg taggatttga aattttggag ttggtatttt tatttgttat tttgaatgtt 3780
atttttaata gtaggttttt tgggatggaa ttttataagt atattatgtt ttgttttgta 3840
aattaagaat tatgttttat ttaattggaa aaatgaatag attttattag aagtagaatt 3900
tttgttatta ttttaagatt ttagttttgt aaagatttaa tatagaggaa gatatttggt 3960
tatattattt tttaaaataa taaatgtatt aaggatgata ttaaaataag aaattttttt 4020
ttttatgagt tttataaaag tgaatgtttt taagtttttt ttgttgtgat taatatgttt 4080
tgtttttttt gagtattttt agtgtgataa agaaatttgg gagtgggaga tggtaatatt 4140
tttgtattta tgatggaagt ttggatgttg tgtgttatat tttgatttgt tttaaggaat 4200

gtgttttaat ttttagtaaa tagtatttta aggaaagttt tttttagttt tagtttatat 4260
agtgtgttat attataatat ttttagtaga gatgttgaat agtatttttt attttaaata 4320
tttttagttt gaaaagtaat tatttttgag tttataaaat ggatttttgt taaatttatg 4380
aagttagtag aaaaagggaa gaatattgta atatatattt tagatatata gttataaatt 4440
gtatttgatt taagattatt gggtatttta atattatttt aaatatattt tgtttaaaat 4500
agagtgaatg atttgtaaat ttttttgtag gattttaagg tttagttgtg ttaatgattt 4560
atagtttatt ttaattatgt ataaagaatg tagaagattt tagaaggtgg gggagttatt 4620
agaggtaagg agtttggatt ttttgattat ttggggaggg gttatatgag tgagaaataa 4680
atttttatta tgttaggtta ttaggatttg gagttggttg ttttagtaaa tagtttattt 4740
tgattaattt tagatatgtg gaagttaggt agatagagga aaggtaaaga gaattttggg 4800
aagtaggtat agaatgttta ttgggtatta aatggtagga tataatatgg taattgtaag 4860
gggattatta tgaatagaat aggagtaagt gtgggtatag aagtatatga gattagagaa 4920
gtaaattaat ttattaatat tattattgtt gtttttaggt tttttttttt tagtttattt 4980
taaaatagtt attatgtttt attgaggtgt aattttagta ttatatagtt tatttaaagt 5040
attagtttat tgatttttta tgtatttaga gttgtgtaat tattagtatt attaattttg 5100
taatatgagt atgattttaa aattttaata tttattagta gttattttat atttttgttt 5160
aaatttttta gtagttatta atatgttttt tgtatttgtg atttggttat tttggattaa 5220
ggtttattta tgttttatat gaattagtat tttatttttt tatttttttt tattgataaa 5280
taatatttta ttgtatggat aaattatatt ttatttattt atttttatta gttgttggat 5340
atttgggttg tttatatttt ttggttatta tgaaaaattt tgttaatgaa tatttatgta 5400
taagtttttg tgtagtgtat attttttttt tttttggata tatgtatagg agtggaattg 5460
ttggtttata tggtaatttt aggtgtggtt tttggagtaa ttgttagatt atggtttaaa 5520
tgattgtatt ttttatattt ntattagtag tgaataaggg ttttagtttt tttatatttt 5580
tgataatatt ttttatttgt tttttttgtt atagtttttt aagtgtgtga agtggtattt 5640
tattgattta tttatatttt tttgatggtt aatgttatat agtgtataga atttgtaaaa 5700
ttaggatagt attgttaggt agttttgtaa gttttttatt tgtgttaatt gtaatttttt 5760
aatagtttta taaaataggt attgttattt taattttata gttgggaaaa ttgaggtata 5820
aggttattta gtattataaa gttaagtatt gtaatttaaa tttaagatat taaatgatag 5880
tttttttagg agtgtgattt taaatgttag atgttatata atttgatttt ttggttgttg 5940
ttgtaaaatt ggtatttggt tttattttaa atttgataaa ttatatttgg tttgattttg 6000
gttggttata ataatttttt tgttagaatg tttggggagg ttgtttttaa aagattgaaa 6060
attttgaagt gggaaaataa taataaagat gtttagtatg ttttaaatta ttattatttt 6120
ataaattttg atgttgggtg attagtattt ttggtatagg aattttttaa atgtatgaat 6180
ttttgaatga ataaaattta ttgattaaat ttaggtgtga tggggttaat tttaaatatt 6240
tttgaagatt gtttttggag aagaggattt aaatgtggaa gtattttttt tatagtttgg 6300
ggaaaaaaat tattttatta agatatagtt gttatataat aagttgtata tatttaaatt 6360
gtataatttg ttgttagatt tgtgaaatta aaatgataaa tatatttttt gtttttaaaa 6420
gtttttttat gttatttttt aaattttttt atattgtttt gttttatttt ttttttttag 6480
tattaattat tattttttgg taagttatta atttgttttt tgttattaaa tattaggttt 6540
ttaaaaatat ttatatttga tttattaaat attaaattat atatttgtat atgtttttta 6600
ggtttgtttg ttttagatgt tttattaata gatttatttt ttgggaaatg attttggatt 6660
tatagaaaaa ttgagtagat aatatagaga atgtttatat attatttgta taattttttt 6720
tgttagtatt ttaaattaat agggtatatt tgttataatt aatgaattaa tattgataaa 6780
ttgttattaa ttaatattta tagtttattt agattttttt tatttttatt taatgttttt 6840
tttttttttt aggattttat ttaggatatt atatttaatt gttatgatgt tttgtgtttt 6900
ttttggttgt gagagttttt tagatttttt ttgtatttga tgattttgat agtttttagg 6960
agtattggtg aggtatattg taagatgttt ttttattggg atttgatgta tttttatgag 7020
tagattaggg ttttgggttt tggtggggtg gaaattaaag aggtaatgta ttgtatttat 7080
tatattatat gaagggtata tattgttaat atgatttatg attgttaatg ttgtttttta 7140
ttatttggtt gaagtagtgt ttgttaggtt tttttattgt atagttattt tttttttttt 7200
ttttgtatat tttgtatttt ttggaaggaa attattatga gttatttaag gaagtgtggg 7260
ggagtaggga gttatgttag tttttttttg gagtggagta tttatataat ttatttggaa 7320
ttttgtgagg aagatttgtt tttttttttt tatttatttt tttatttagt tttttttttg 7380
tatggattta tgaatatttt gttgtttaaa tttttttagt ttttggtatt gggggttttt 7440
ttagttgatt tttgtgtttt ttgatttatt ttttgtattg tgtgtatgtg tgtgtatgtg 7500
tggatttttt tattttttgg tattataaga tgttatagat ttattagatt tattttgtat 7560
attttttgtt ttagttgtag aattagttat ttatttaagg aagtttgaga ttttttattt 7620
gaaattggta tagaaatgaa gatttgggtg ttaaataaat taggttttat tttttagaat 7680
tgtatataat atagtgtatt ttttttttat ttggtttttt ttatttagta taattatttt 7740
gagatttagt taaattatag tatgtattaa tagttaattt tttttatttt ttttatttat 7800
atattatagt ttgtttattt atttaattgt ggataaatat ttgagttgtt tttaattttt 7860
ttggtattat aaatagagtt attatgaaaa tgttgtataa gtttttgtat gaatatgtgt 7920
ttttattttt tttgggtaaa tatttagtag tgaaatggtt ggataaatta gtaggtgttg 7980

tgatatgagt gtttgtgttt tttttaaatt tgtgtgttaa aatttaatta ttaatgtgat 8040
agttttagta ggaggaggtg ttggggagtg attaggttat ggagt 8085

<210> 337
<211> 7110
<212> DNA
<213> Artificial Sequence

<220>
<223> chemically treated genomic DNA (Homo sapiens)

<400> 337

tgttttttgt gatgttttag aggttagaag gatgttttga aggggaaaat tgtaggagtt 60
aggtagagta gggttttggt ttttttttaa tgttttttta tttagataaa tttgggttat 120
tttgtagagg ttgtagtgag ttatgattat gttattgtat tttagtttga ggaatagagg 180
gagattttgt tttaaaaaaa aaaaaaaaaa aaaaaaaaaa aaaaaaaaaa attttggttt 240
attttatgaa gtatttggta ggtgttttaa tattttttgg tatatgggaa ttattttttt 300
ttattattgg aaggtttaaa aagtaaataa tgtgttttat atggttagag ttagtattaa 360
ggggattaag tagaataatt ggtagttagt gttagtttgg agattaatat agttaaagtt 420
gttttgttat gttatgtttt ttttatttgt attgttttat ggtttaagat aaaatttttg 480
aatgttattt ggatatagtg agtatttggt ttgttttatt gtttttagga gtaattaatt 540
taatttttat ttttttgaga atgatgattt tatatagtat atttttttat taagatgtga 600
aagatgatat tatggtattt gaaatagttt taggagagat ttgggatatg ggaagtttgt 660
agataataat ggaaaatttt tttttagaat atagttattt gtatgattta tagtagtgtt 720
ttttggagaa tgttttaaaa ttatttttat tgaaagaata ataatgtttg ttattaggat 780
aaatgaaata aggggaaaat tttagatttt tggaataatg ggtttatatt taatttaatg 840
attattatgt ttttatattt tgtattattt agaaaatagt agttaaatag atagaaatat 900
agaagattgt ttaaaaatta aagttattga gttagatttt tttttgaaag gttagtgtat 960
gggagatgag aaaggtatta tagagattag agtgtttata taaaagatat tttagtagat 1020
gatttataaa gagtatatta agtatttata ttatttatat taagttgttg gttattttgt 1080
ttattaaaga aaataagaaa gaaatttttt tttgatatta ttttggtgta gtaataatag 1140
agttttggaa ttagttgtta gaagtgataa ttaaagttag tttattatgt ttattaatta 1200
atttatatag ttattttgaa gttttatata attgtttttt tgttataagt ttttattttt 1260
ttttatggta ttttggtttt gaatttagat aggtaaagaa ttaagtatgg ttgggtgtgg 1320
tggtttatgt ttgtaatttt agtattttgg gaggttgtgg tgggtagatt atgaggttag 1380
gagattgaga ttattttgat taatatggtg aaattttgtt tttattaaat tataaaaaat 1440
tagtttggta tgatggtgtg tgtttgtagt tttagttatt tgggaggttg aggtagggga 1500
attgtttgaa tttgggaggt agaggttgta gtgaattgag attatgttat tgtattttag 1560
tttgtgagat tttgttttaa aaaaaaaaaa aaaaaaagaa aaaaaaaaaa agaattgaag 1620
tgtttggaga ttagtgtatt gtaatattta tatagttaag taggtatttt taattgaatt 1680
ttaaaaaagt attatttttg aaattttttt tagtttagat agggttagtt atttgggtag 1740
aaaggaaaag aaaaataaaa aattggaaaa ggatgtatta gtattttttt tagaaataat 1800
ttaatattag gaatagaagg aggattttat ttattaattt ttaataatta tttgatgttg 1860
ttgttgggtt ggttaaatat ttgttaatat tgatgatttt tttatttatt attagagaga 1920
agtagaggga tttgatgttt agagaagtag taaaatttat tttttatttt ttttttttag 1980
ttggatggaa gtatttggag gttggtttta tattttagta ttttagtgtt ggtgatgtag 2040
attattttttg ttttagagta atttgtgttt atggaattta agaggatttt gttgtattag 2100
gataggaatg tatttttgtt ttatttgttt ttatgaaagt agattttaga gatttttaga 2160
tgttatgaga ttaattgtta tttagggtag gttttagggg agggatagag aagggtagtg 2220
ttaataatag ggattttgga gataagatta gtaatttaag aaataaatgt gagatatttt 2280
attttttttt aatttatttt ttttttaaaa gtttattttt attttttttt tttttattat 2340
gtagtaattg ataattaaat atagttttgg tttttattat gtaatttagt agaatattta 2400
gatattagga attttttatt tttggttatt tatataataa tgaaatttgt agtttatgtt 2460
aggttgtgtg gatagtattt gtatttttgg aaattaattg gaggtttttt ttttattttt 2520
ttttggttgt tttttatttt tttttttatt gtggtttttg ttttttttaa gttgattaga 2580
attatgtata aaattagttt ttaataaata ttaatattga agttttagta ggttgttaat 2640
ttttgtatag ttattttaaa tgttgtatat aagtgtaaag ttttatggag ttttagaggt 2700
ttttaagaat attgtaaata ttttaggttt aattagtagt tttggagtta ggtaatggtt 2760
tgtggttttt tgtgttaggt tttttgaggt ttttagtttt tagaggttgt ttgtaattat 2820
gatgtgtata tatatagtat ttttttttaa atatagtata aatttttttt agattttgtt 2880
aggtatttat aattattgaa tatatatgta gattgatttt tattttttta aagtttgaaa 2940
aataatttag tgttttaatt taggttttgt tagatatttt ttgatttaaa ttgttgtttg 3000

tagttttatt ttttaaggga aatgaaaaaa taataaattt ttagattggt gttgatgttt 3060
atttttttta agtgggttga ttatttgttt tgtagatttt tgatgggtgg ggtgtggggt 3120
aggagtgtga tttaattttt agtatttttg aattagtttt tttatggtga taggttagtt 3180
taattggggt tgtaaataga tttgataggt ttgttttggg ttgatggtta ttgattaggt 3240
ttttagatta gataagttat ttggttgagt ttatagtagg tggggtgtgt ttattagttt 3300
aggggtagtg attggatgtt tgttgtaata ttggagaatg tatgttttgg gttgtagtag 3360
gagatatttt taagtataga attaaaaggg tttattttaa gtggtagggt attagtgatg 3420
gagaggtttg agagttttag tgtttagttt ttttttttat gtttgggaag gtgtagaata 3480
ggttgatgta gagtaaggag tgagttttag gtttagtttt tttggtttgt ttttagggta 3540
gtaggtgagg agtggtatta gtttggggat tttttttttg tgttttgtaa gaattggtgg 3600
tagttagtag gtggggaggt gggggtatgt gtttggatgt gggtgtttgt gtaattagtt 3660
ttttaagtgt tagttttgat agtgtttttt tgggaggttg gtttgagttt ttgtttttgt 3720
tgtggtgtag gaagggttgg ggttttgttg tttgtattag gtaagagtat tttgagtaaa 3780
ggaagaagat gatttgtttt tggagttatt attggggagt gggaatttgg aaagtttttt 3840
aattagggat atatgtgatt ttttttggaa agtagttttg attgtggttt gtgtattttt 3900
ttattttttt ttgaattttt ttaggttttt ttgttttgtt tatttgttgg gttgtagttt 3960
tttattgttt tgtatttttt atttaatttg gtaattttaa atgtgtatgg tttggttggg 4020
gtgtgtggag tttggttttg ggtgatttat tttgttgggt ttttatggtg gttaaggggg 4080
ggtggggtta ggtggttttt gagaattgag tttgattttg atgttgtgaa ttgatttgga 4140
gtttgagggg aaagatgttt gatttttttg ggggtattgg agtgggtgta ggagaggttt 4200
gtggggtgtg ttttatttat aggatttttt ttttagttta tttagatagg tgtttttttgg 4260
gtttttgaaa tttaatggtt atgtgtttat gtttagtatg tttggttgag agtttttatt 4320
tttagggtaa atgagttgag ttattgggga agtgagaggt ggggtgttgt aagggagttg 4380
gatgaggtga tatatgttgg tgatataata gtaggttgtt ttttgtgtta agattgatat 4440
tatgaggata tagatttggg ggaaggggga atttttaggt aaaggttgtt atagttaaat 4500
ttttgtgaat gattgtgatt tgatagtggt gtaaaaggaa agagtgaatg tagtttatgt 4560
tgtggaaatt taggggtaga ggtaaggggg gagggtattt tttttgtagg gattgttttt 4620
gtattttttt ttatattgag tagtgtggtt atttggtttt ttttatttgt gtataggtaa 4680
ttttagattt gagttagtga tattgtttaa tgtatttatt ttagttttta ttattagttt 4740
tttatttttg ttttataata gtttattttg tttttggttg ggtttttggg tagaggttga 4800
ggtttagttt gttatttttg ttttgtgttg ttgtaaaagt tgtagtaagt gtagttgtag 4860
gttggtggtt gggaattggt ttgagtaagt tttaggtagt tatattgggt atgtttagta 4920
gagtttgtgg tttggggatt ttgtgtttgt atttagagtt attgttttgt ttttttttat 4980
tgttttttgt tttgttttgt tttttttttg tttggtgtgg ttttgtttgt tttttgtttg 5040
ttttttgttt ttttttggtt ttttgaggtg tttgggtttt tggtgtggtg gtggaggggg 5100
tgggtaggtt ggtgggtggt gatgtggtgg gattttttat gtgttgtggt aggatatgtg 5160
tttggtgttg ggatgtgatt gtgtttagtt tttttttttt ggaagttgta gttatgatgg 5220
aagtttgaga gttgagttgt tgtgaggtga ggttgggttt aggtgaggga gatgagagat 5280
ggtggtggtt gtggtttgga gtttttttta gtgtttgtga gtagttgtgg gggtagtgtt 5340
tttggggagt tggttggttt gtggtggtgg tagtggtggt gttttttgtt ttttttttgt 5400
tttttttaat tgtgtagttt tttttttggt tttttttgaa agggaaggtg gaagttgtgg 5460
gtttgggtgg gagttggttg aggtgtggtg gtggtggtgg tattttttgt ttttggagtg 5520
ggggggagaa gtggtggtgg tggtggttgt ggtggttgta gttttaggga gggggtttga 5580
gttgtttgtt attattttta gggttgggaa tgttggagag ttggtttttt tttttttatt 5640
gtttttaata tggtggtggt ggtggtggta tatttaggga tttgggttgg ttttaaattt 5700
tttgtttgtt gttgttgtat tttttgtggt ttgggttttg gaggttgttg gtggaggtag 5760
ttgtttggag gattatttgt ttttttttta ttttgttgtt gttgttgtta ggtttttggt 5820
tgttgaggag aagtaggttt agttgttgta attatttagt agttgttgta gtagttatta 5880
tttggttgtg gtttagagtt aagtggtggt agagtgaggg gtattagtta ttgttaagtt 5940
tagagttatt tttattttgt agaagaagtt ttgttattag tagtttttgt tatttttttt 6000
tttttttttt tttagttata ggtttttaga tatgatagtt attattaaag agattgttag 6060
tagaaataaa aggagatatt aagaggatgg atttgattta gatttgattt gtatttattt 6120
ttgtggttgt ttttttttat tttttgttta ttttttttaga atgtgggagt agatggatgt 6180
gaaaatgttt gtagtttggg tgattataat atttaatttt ggttaggttg ttaggttata 6240
tattttgtgt tttttttttg tgtatttaat ttagggtgtg tttggttaga tggaattttt 6300
gtttggttgt aagtgttaag ttattgattg tttttttagg ttatttatat ggtttttttt 6360
tgagggttat tgtttgttaa tatagaatat agtatattgt tagtggatta gtgagtttgg 6420
taatttggtt ttttaaatga ataaaaaagt agatgttttt tgaggttgag tatattttga 6480
ttaaattttg gtttaggttt tagattaagg gtttagatta gaataaaatg aaaattagtg 6540
ttgtatgtat gtatattgta ttagaatttt gtagtgattg ttttagtttt ttgagttgta 6600
ttgatagatt tttttaaaat atgattgatt tgtataattt tagaagtaga attattttta 6660
gtatatatgg tgtatattga gggtaaaaag tagttttgtt aatgtttaaa tttaagttat 6720
ttataatttt gaattgtatg tagaagtttt gtagtttgaa gttaatagtg ttataatata 6780

ttttataagg tgtttttatt agatttttgt tatatttatt tttttttttt tttatggggt 6840
gatgtaggat agtgtttgaa atttgtattt tagtagtatt taatgtttag tgtttttgtt 6900
ataaatatag aatggatatt gagtagtttt tgattttaga tggtaatgtg taggtttaag 6960
ggtattgtgt gtagtaagtg aagattgtag aaataaaatt ttagtttatg tttgaaattt 7020
aagtattgtt gtgatgttag aattgttgtt tattgttttt aggttttagg ttttttgata 7080
ttttttggta ttgttaattt tattgatttg 7110

<210> 338
<211> 7110
<212> DNA
<213> Artificial Sequence

<220>
<223> chemically treated genomic DNA (Homo sapiens)

<400> 338

taaattagta aaattaatga tattaaaagg tgttagagga tttgaaattt aaaaatggtg 60
agtagtaatt ttggtattat aataatattt aaattttaag tatgaattga agttttattt 120
ttgtaatttt tatttgttat atataatatt tttgaattta tatattatta tttgggatta 180
gaaattattt aatatttatt ttatgtttat gataagagta ttgaatatta aatgttattg 240
aagtgtaaat tttaagtatt attttatatt attttatagg gagagaaaaa ggtaaatata 300
ataaagattt agtaagaatg ttttataagg tatattatgg tattattgat tttaaattat 360
aaaattttta tatatagttt aaagttgtag gtaatttaag tttaaatatt aataaaatta 420
ttttttgttt ttaatgtgta ttatatatat tgaaaatgat tttgttttta aagttatgta 480
aattagttat attttaaaag aatttattaa tgtaatttag gaaattaaaa taattattgt 540
aagattttga tgtaatatgt gtatgtgtaa tattaatttt tattttattt tgatttaaat 600
ttttgattta gggtttaagt taagatttaa ttgaaatatg tttaatttta aaaagtgttt 660
atttttttgt ttatttagga gattggatta ttgagtttgt taatttatta atagtgtatt 720
gtattttgta ttaatggata atagttttta ggaagagatt atatagatag tttaagaaag 780
taattggtgg tttgatattt gtaattaggt aagagttttg tttagttaaa tatattttag 840
gttgaatata tagaaaggaa atataaaata tatgatttag taatttgatt agggttaaat 900
gttatagtta tttaaattat ggatattttt gtatttgttt atttttatgt tttaagagag 960
tgatagaaag gtaaagagga gtagttgtag aaatggatat aggttaagtt taagttgaat 1020
ttattttttt gatatttttt tttgtttttg ttaatgattt ttttgatgat ggttgttatg 1080
tttgggagtt tgtggttgaa gaaaaaggag gagagagatg gtagaagttg ttggtggtgg 1140
ggtttttttt gtaggatgga aatggttttg gatttggtgg tagttgatgt tttttgtttt 1200
gttgttgttt ggttttggat tgtagttggg taatggttgt tgtggtggtt gttggatggt 1260
tgtagtgatt gggtttgttt ttttttagta gttagaggtt tggtagtggt ggtagtggaa 1320
tggggagaag atgaataatt ttttgaatgg ttgtttttgt tggtggtttt tggagtttgg 1380
gttatggggg gtgtggtggt ggtggatggg aggtttaaaa ttggtttggg tttttggatg 1440
tgttgttgtt gttgttgttg tgttggaggt agtagaaggg gagagattaa ttttttggtg 1500
tttttagttt tggaaatggt gataggtgat ttagattttt tttttggagt tgtagttgtt 1560
gtggttgttg ttgttgttgt tttttttttt tgttttagga gtgggaggtg ttgttgttgt 1620
tgttgtgttt tagttggttt ttgtttgagt ttatggtttt tatttttttt tttaggagaa 1680
gttgaggaag aggttgtatg gttagaaaag atgaagagga ggtgagaaat gttgttgttg 1740
ttgttgttgt aggttggttg gttttttgag ggtgttgttt ttgtggttgt ttataggtgt 1800
tgagaggggt tttgggttgt ggttgttgtt gttttttatt ttttttgttt gagtttggtt 1860
ttgttttata gtggtttaat ttttaaattt ttattatggt tgtagttttt gagaggagag 1920
aattgagtgt agttgtgttt tagtgttgag tgtgtatttt gttgtagtgt ataaagagtt 1980
ttgttatatt attgtttgtt ggtttgtttg ttttttttgt tgttgtgttg ggagtttggg 2040
tgttttggaa gattgagggg aggtgggagg tgagtgagag gtggatggga ttgtgttggg 2100
tgaggggagg gtagggtagg gtagggggtg gtaggagggg gtagagtggt agttttgggt 2160
gtgagtgtag agtttttaag ttgtaggttt tattgagtat gtttagtgta gttgtttggg 2220
gtttgtttgg gttggttttt agttgttagt ttgtagttgt atttgttgtg gtttttgtag 2280
taatgtgagg tgaggataat gagttaagtt ttggtttttg tttagaaatt tagttggagg 2340
tagggtaggt tgttgtgggg tgggggtgga ggattgatga tgaaagttga gatgggtgtg 2400
ttgagtagtg ttattgattt gagtttgagg ttatttgtgt ataggtgaaa aggattaggt 2460
gattatgttg tttagtgtag agggaaatgt agggatggtt tttgtaaggg gaatattttt 2520
tttttttgtt tttattttta gatttttgtg gtgtggattg tatttgtttt ttttttttgt 2580
attgttgttg gattataatt gtttgtagag atttgattgt aatagttttt gtttagagat 2640
tttttttttt tttaaatttg tgtttttatg gtgttagttt tagtataaag agtaatttgt 2700
tattgtgttg ttagtgtgta ttattttatt tggttttttt gtagtgtttt attttttgtt 2760

tttttggtaa tttggtttgt ttgttttaaa aatgaaagtt tttagttgag tgtgttgaat 2820
gtgaatatat agttgttgaa ttttaagggt ttaaagggta tttatttaaa tgaattgaaa 2880
gaggattttt gtgagtggga tgtattttgt aggttttttt tgtgtttgtt ttggtgtttt 2940
taagagagtt gagtattttt ttttttgggt tttaggttgg tttgtggtgt tggagttaag 3000
tttggttttt agagattatt tagttttgtt tttttttggt tgttgtgaaa atttggtagg 3060
atggattgtt tggggttagg ttttgtgtgt tttggttgga ttgtgtatgt ttggggttat 3120
tgggttgagt ggaaagtatg gaatggtagg aagttgtagt ttagtgagtg ggtggggtga 3180
ggagatttag gagggtttaa aaggaggtgg aaggatatat gggttatagt tggaattatt 3240
ttttgaagga ggttatgtgt gtttttagtt ggggaatttt ttaaattttt attttttagt 3300
gatagttttg gaggtaagtt gttttttttt tttgtttggg gtgtttttgt ttggtgtagg 3360
tagtggaatt ttaatttttt ttgtgttgtg gtggaaatag gggtttggat tagttttttg 3420
aaggagtgtt gttggggttg gtgtttgggg aattggttat ataagtattt atatttaaat 3480
atgtgttttt gtttttttgt ttgttggttg ttgttgattt ttatagaatg tggggagaga 3540
gtttttaaat tggtgttatt ttttgtttgt tattttaaga gtaagttaaa ggattgagat 3600
ttgagattta ttttttgttt tatattgatt tattttgtgt ttttttaaat gtgggaaaag 3660
gagttgggtg ttagggtttt tgggtttttt tattgttgat gttttgttgt ttagggtgaa 3720
tttttttggt tttgtgtttg agggtatttt ttgttgtagt ttagagtgtg tattttttga 3780
tgttgtaata aatgtttagt tattattttt gagttggtga gtgtgtttta tttattgtgg 3840
atttagttaa gtgatttatt tggtttgaga atttagttaa tggttgttag tttagaataa 3900
atttgttaag tttgtttata gttttgattg ggttgatttg ttattgtgag agagttgatt 3960
tggaaatgtt gagagttgga ttgtattttt attttgtatt ttatttgtta aggatttata 4020
aagtaagtag ttgatttgtt taaagagaat gagtattaat attagtttgg gaatttattg 4080
tttttttatt ttttttagga agtagaatta tagatgataa tttgagttaa aaaatattta 4140
atgagattta ggttgaagta ttgagttgtt ttttaaattt tgagagaatg agaattaatt 4200
tgtatgtgtg tttaataatt gtaagtgttt agtaaagttt aaagagggtt tatgttgtat 4260
ttgagaaaga gtattgtata tgtgtatatt atagttgtag gtaatttttg aagattagaa 4320
attttaagag atttagtgta gaaagttata ggttattatt taattttggg attattaatt 4380
aaatttggga tatttatagt gtttttagag atttttgagg ttttataaaa ttttgtattt 4440
atatatagta tttaagatag ttgtgtagga gttaataatt tgttgaagtt ttagtgttga 4500
tatttattga agattggttt tgtgtatagt tttggttagt ttgggaaagg taaggattat 4560
agtggaaaag gaaatggaaa gtaattggaa aagagtaaga gaaaggtttt tagttgattt 4620
ttagagatgt agatattgtt tatatagttt agtgtgaatt atagatttta ttattgtata 4680
aataattaaa gataaggagt ttttgatgtt taggtgtttt attagattgt atggtaaaaa 4740
ttagagttgt gtttaattat taattattgt gtggtagaaa aagggaaaat gaagatgaat 4800
ttttaaaagg ggagtagatt agggaggggt aaaatgtttt atatttgttt tttagattgt 4860
tgattttgtt tttagagttt ttgttattag tattgttttt ttttgttttt tttttggagt 4920
ttattttaag tgataattag ttttatagta tttgagaatt tttgaaattt gtttttatgg 4980
aagtaaatga aataagggtg tatttttgtt ttggtgtgat agaatttttt tggattttat 5040
ggtatagagt tattttggag tagagataat ttgtattatt agtattaaag tgttagaata 5100
tgagattagt ttttaagtat ttttatttgg ttgaagaaag aaaatgggaa atgggtttta 5160
ttgttttttt aaatattagg tttttttatt tttttttagt ggtgaatgaa aggattatta 5220
atgttggtag atatttggtt agtttaatag taatattagg tggttattag gagttggtga 5280
ataaaatttt ttttttgttt ttagtgttaa gttgtttttg aaagaaatat tgatgtattt 5340
ttttttaatt ttttgttttt tttttttttt ttatttaaat ggttggtttt atttaaatta 5400
aagaaagttt taaaaatagt atttttttaa agtttagttg aaaatatttg tttggttgta 5460
tagatattat aatatattgg tttttaagta ttttagtttt tttttttttt tttttttttt 5520
tttttttttt ttgagatgga gttttatagg ttggagtgta gtggtatgat tttggtttat 5580
tgtaattttt gttttttagg tttaagtaat ttttttgttt tagttttttg agtagttggg 5640
attataggtg tatattatta tgttgggtta attttttgta gtttagtaaa gatagggttt 5700
tattatgttg gttaggatgg ttttgatttt ttgattttgt gatttgtttg ttatggtttt 5760
ttaaagtgtt gggattatag gtgtgagtta ttgtgtttgg ttatatttag ttttttattt 5820
gtttggattt agaattagaa tattataggg agaagtagaa gtttgtaata aagggataat 5880
tatataaagt tttagggtga ttatgtagat tgattagtaa gtatggtaaa ttagttttaa 5940
ttattgtttt taatagttga ttttaaaatt ttattgttat tatattaaaa taatgttgaa 6000
agaaaatttt ttttttattt tttttgataa gtgaaatgat tagtaatttg atgtggatga 6060
tataaatatt tgatgtgttt tttgtaggtt atttgttaag atgttttttg tgtaaatatt 6120
ttgattttta tagtgttttt tttatttttt atatgttgat tttttaaaaa gggatttaat 6180
ttaatagttt taatttttaa ataatttttt gtatttttgt ttgtttaatt attgtttttt 6240
aaataatata gaatataaaa atataataat tattggattg aatgtaaatt tattgtttta 6300
agggtttgag gttttttttt tattttattt attttaataa taaatattat tgttttttta 6360
ataaagataa ttttaagata ttttttaaaa ggtgttattg tgggttatat aagtaattat 6420
attttaaaag agaatttttt attattgttt ataagttttt tatgttttaa attttttttg 6480
aagttatttt agatgttatg gtgttatttt ttatattttg gtagagagat gtgttgtatg 6540

aaattattat ttttagagaa ataagggttg agttggttgt ttttgagaat aatagagtag 6600
attaggtatt tattgtgttt aagtggtgtt tagaaatttt gttttggatt gtggagtaat 6660
ataaataaaa aagatataat atgataagat agttttgatt atgttagttt ttaggttaat 6720
attagttatt aattatttta tttggttttt ttaatattaa ttttagttat atggagtata 6780
ttgtttgttt tttagatttt ttaataatag aaaaaggtaa tttttatata ttagaaaata 6840
ttgggatatt tattaggtgt tttatagagt aggttgaaat tttttttttt tttttttttt 6900
tttttttttt tttttttgag atggagtttt tttttgtttt ttaggttgga gtgtagtgat 6960
gtgattatgg tttattgtaa tttttgtaga atagtttgaa tttgtttagg tagagggata 7020
ttgagaagga gttaaaattt tgttttgttt aatttttata attttttttt ttaaaatatt 7080
tttttggttt ttaaggtatt ataaaaaata 7110

<210> 339
<211> 6478
<212> DNA
<213> Artificial Sequence

<220>
<223> chemically treated genomic DNA (Homo sapiens)

<400> 339

tttattttga agggatgttt aagaaatatt gttaaattta aaagtgttgt agagtaatat 60
gtattataag attatattgg tataatagta ataaatagta tttttttata taattttaaa 120
aaaatatata gatttatgtt aagtattagt taagatgttt ggtatatggt aggtatttaa 180
tatttaatga atatatgtag aggattttta ttttttattt tatatttttt ggataagatt 240
ttgaattttt atgagtatgt attatttaaa tttagattta tagggagtga tggaaaatgt 300
gaaaataaaa ttttaaaaag gaaaagaaag agtaaagatt tagatttaaa aaaggaaatg 360
taaataagat gaaatagtta aagataggtt tttttaaaat taatgttgag ataatgttat 420
tagtagtgat agttagttaa gagatggttt gtagtttatt gtttagtatt gaggtattta 480
gtaggggttg tatttaattt ttaagttgga attaattgaa attgttgggt ttgaagagtt 540
gtttttatga gttattgatg gtagaaagta tagaagtatt aaagaaaata atttagaaat 600
gtggaaagag aaaagtatgt ttttgttttg ggagaataat atttgtgtgt aattaagtag 660
gaagattttg tggaggaaag tttgaaaaag tagagagttg aagagaaaag agttaaatga 720
aggatatggt ggttaatatt taaagtaaaa agatattttt gaagaagatg tgtttttagg 780
gtttttaaga agttgttata tttatgttta gagttatttt gtaagtttat ttggaaagtg 840
tattggtgtt ttttggtata agatttatga atattaatag ttttgttatg tgagtaggtg 900
tttatattga agggttataa gtaaagtttt tatgtgttat tattgttaga attttttttg 960
ttttttttta atataaaagt agttttgttt gttagtatgg aagttttggg aagagtttta 1020
tatttgtttt gtgttgttaa tatttttgtt ttggtttttt tgttggaata tataagttat 1080
ttagtattag ttatttattt ttttggtaga attgtggtaa gaggggatta atagggaaaa 1140
aaaattaaaa aataaataaa aagttaaaat aatatgaaag agttgggtat gtttttaaaa 1200
taattagtgt ttattttttt tagttttgtg tttgtgtttg tttgtatttt gtatgtttgt 1260
ttttgtggtg gggggggata tgtttgtgtt tttgaaaatt tttttattta gatattataa 1320
ggtattagtt taattttgaa tatagttggt tgtagtatgg tgtatagata tgattgtttt 1380
ttttttagaa aattttttag tttaaatatg tttgtagagt atgatttatg gtgttttttt 1440
ttggattgtt tagtgggtaa ttgttgtatg aaaattagag aaatagatgt agattttgaa 1500
ttttggttat aaatttttgt tttatgatta tgggttttaa atgtgttgtg ttatattgat 1560
aaatatatag ttattttgaa ataaaatgga ttttattttt aaagttttgt tttttgtttt 1620
ttattttttg tttgtttgtt ggttggaaat atatttatta gattattgat taaatataaa 1680
gaaaatagag atgatatata ttagaatgtt attttatgtt ataataagaa gatatttggg 1740
aaagtttttt ttaattttgt ggttaatgtt taaaatgggt gttagtagtt tatattttat 1800
tatggataaa ttggtttatt ttttattttt tattttattt attaattttt atagtgtatg 1860
gatttgtatt taaagaatta tggtttattt atgtattttt tttttatttt atttaaaatg 1920
aagaatttgg agatagaatt aagtttttaa gaagtgtaag tatttattga tgaagaaata 1980
attagtatgt ggttaaatta tgggtttgtt ttataaatta atatttggat taagtaatag 2040
ttttaattat tgttattagt ataattaatt tatgatgagg aatgattaat tgattagtga 2100
atattgttat attataagaa atttatatta ttttttttat ggggatttaa atattgatta 2160
tttattttaa ggtttaagtt tattttttag ttgagaattt taaggtaatt atatatttta 2220
tatttttttg tataaagaaa gaaaatattg agttgtttaa tttttattat atatagagag 2280
agatatatta atgttgttat ttttgtgtaa tgatatagta tagaggtaag aaatttggaa 2340
gtagtggttt tgtttaatga ggttgatttt atagtttaag aaatgagaat gaatagaatt 2400
ttgaggatat ttatttttat ttttattttt ttgggattag tgtattttta ttttttggag 2460
agatgttaag taagaatgtg tagtagtttt ttttggataa tagatttttg tatatatata 2520

tttgttagtg atgttatgtt ttttattttt aatttaagtt ttttttgttt tatttttaat 2580
atagttgttt tttttttttt tgtttaataa ttttttattt atttttattg tttggtaaaa 2640
atgaatttag tttttaagga attttaagga ttttaaataa gtatttggag ttataagttt 2700
tatggattaa gtagaaattg gtatataatg tagtgttttg tgtattttta taattatttt 2760
aatgatagtg tgttaaatgt tttgtaaatt tttttgtgtt tttttgttta aaatgtgtgt 2820
tttttttgta gttaaagggt tttttataat aagtttttgt tataaaatga tggttatata 2880
aaagagagaa gagaggtgta aaaataataa atatagattt agtaaaaatt ttattttttt 2940
attgttttga attttttaat agtaagaatt gagtttgttg ggttgaaaat tgttttaatg 3000
tatttttata aaaggatttt tgatttggta agtgtgtgtg atgtatattt tttatgttat 3060
attataagtg ttatttagta attttattag atagggtagt gttttagtat ggggtggggt 3120
gttttttgat aggtttttaa aaggtttgga tgttaatgta tattttaata ttatttataa 3180
aaaggagatt ggagtagtgt ttttttttgt attgggtaag gagatttttt ttttttgttt 3240
aattatttgt ttgttttgtt ttgtgggaga attataagta aatgttatag aggtagtgga 3300
gaaaaaaggg tgttttaatt tttttttaga gtttttttta tttgatgtat gttgtatttt 3360
ttaaataagt tgtgtaaaat ggttgtaggg tagattggtt ttttttttta aagtttttta 3420
tttggttggg tttatttgta aatattgtat ttattttttt tagtgtttta ggattggttg 3480
gaaagatttt tttttttgta ggttgggtta gtgtgagaga tttaaaaaat tatttttttt 3540
taaaattatt gtattttaat aaaaggattg ggtaggggtt ggaatgagag aaaattggtt 3600
ttttaaaatg taaaattgtt atatttaaat tagtttataa aatatgtgtt taattatgtg 3660
gtgggatgtg tgtaggtgta tgatgagaga ggtaattaat atggttattt ggggtgtaag 3720
gatgtgggaa taggtaagta atttttatat tggattttta ttttagggag ttgggtttta 3780
gttatagttt tgagttgtgt gattttgggt aggttttatt tttttggggt tttgttttat 3840
tagttgaata gtatgaggat gagttatagt taatatttgt tttatgatat ttatttagta 3900
ttatataagt gttatttttg tttttttagt taatattgat gtgggtagta ttatatgttt 3960
attttataga tgaggaaatt gaagtttgaa gaagttaaat atttatttta gaatatatag 4020
ttggtaagtg gtagatttgg aattggaatt tagtttagtt tgatttttta atttatgttt 4080
ttgattatta tattgttttt ttaagtttag atttgaaatt ttattttttg tgtggttgtg 4140
tgtttgggat aggggtaatt aatttttgat tattttatat gttgtataga atttggagag 4200
gattttttaa agtaaatgaa ttttgaaagt tggattgtag agtaaatgag tgtagttaat 4260
ttagttaggg gtttgtaaga gggagaaaga gaaaaagatt gtggaatgga aagtttttta 4320
atttaagttt tttttaaggg gtagttattt tttgttttat agtttagggt ttgtatgtgt 4380
tttttttgga gtggaaaaat atataagtta taaggaattt aatagataga aaggtgtata 4440
gaggaattta aagtgtgggt tgggggtgga ggtggtgggt gggaggtgag tgggtgtagg 4500
tggaatattg ttttttaagt taagttgttg taaataaaaa ggtgtaaagg gagagaagtt 4560
ggtgtttaat gtgagttagg agtagtgttt tggttttttt tttgtttatt ttaaaagtat 4620
tttttgtatt gtttttaagg tgagaaatag gaaagaaaat gttggtttgt gtgtttgttg 4680
tttgtttttt tggttgtttg tttttgtagg gttgttggga gtttttaagt tttgtgagaa 4740
ttttgggagt tggtgatgtt agattagttg ggttatttga aggttagtag tttgggtagg 4800
gtttattgaa agtttatttg tatatattag gtaatttaat tttttatttt gtgtgataga 4860
agtagtagga agtgagttgt ttagaggtag gagggtttat tttttgttaa aggggggatt 4920
agaatttttt tatgtgagtt gtttgaggat tgggatgttg agaatgtgag tgatttgagt 4980
agggtttgtt tgggtattgt tggggtagga tttggaatgt atttggaagg ttttttgtaa 5040
gtatttattt ggaaggagaa tttgggattt ttttgggaat tttttgtttt ggttggattg 5100
gttgagtaag tttggaaaat ggtaaatgat tatttggatt aattataggt ttttagttgg 5160
tttgtttgtt ataatttatg atttggggtt gggaaaaaga ttaatagttt atgtgttaaa 5220
aaaggggtag agtttgatgg agttgggtgg atttttttat gttatttgtt tttatattta 5280
gaggataagt atttttgtag atatttagtg taagggagat tatgtttgat tgtatggatg 5340
ttttgttagt gagttttggg taaattttgg atttttatat tgtgagtttg tttttttgta 5400
tgttttagga gaaagttttt aaagtatgtt ttagtggatt gatttaaatt gaatggtagt 5460
attggtatat tgtttaatgt aggtttattt tttttttttt ttttattaag aaaaaaaaaa 5520
ttgttttttt tgtatgtaat aaagatgttg gaaataaatt gtattggtag taagataaag 5580
gatttaatga tttaatgttg aaggggtgtg atatgttggt atgtatattg attttttttg 5640
ttgaaaattt tttgttagat gttttttttt taaataattt tttttgtttt tatgtttttt 5700
tttttattaa ttttatagtt ttttgaaaat agtttttaaa atatgtataa aaatgtgggg 5760
ggtggggagt agagggtgat tataagattt taaaatattt tttaagtagg gtaatttgat 5820
atattttgta gtttttatta aaatgtatgt agagtggaat ttaattttgt tgttttttgg 5880
attagttatt agttgtatag gtttttttat tttttgtgta ttttaatagt ttaaagtaat 5940
taaaatatga tatttttttt gaaaggaatt tagtttaatt aaggtggttg ttgtttttga 6000
agtttataaa ttattagttt aagttgttgt ttttaatgtt ttttttgtta aggaaaggta 6060
atatttttta gtattgattt tgttgttatt tttttaagtt attttgttgg tttaagaagt 6120
tgttgttttg ttagaaaatt attggtaatg aatatttttt ggattgagtt atttattatt 6180
tttatatttt gtagtttagt ttggaatgtg gatattagtt aaaggaaagg aaatataagt 6240
ggggtagttt aatttaaaaa aaaaatattg attattttgt tatttgatgt tgatatagtg 6300

ttgaggtatg tatttggtta atagtagttt ggttgtaggt ttgagaaatg attaggagtt 6360
ttagaaaatt ttatttttgg gtggttgtag tgggagtttg taggatgttt tgttgttttg 6420
atagtgattt agataaatgg tttagttatt tattaaaatg tttttagtat tttatttg 6478

<210> 340
<211> 6478
<212> DNA
<213> Artificial Sequence

<220>
<223> chemically treated genomic DNA (Homo sapiens)

<400> 340

taaataaagt attaaaaata ttttgataga tagttgagtt atttatttaa attattgtta 60
gggtggtgaa gtattttgta ggtttttatt atagttattt gaaggtggga ttttttggga 120
tttttgatta ttttttaagt ttgtagttag gttattgttg attaggtgtg tattttggta 180
ttgtgttagt attagataat aaaatagtta atattttttt tttaaattgg gttattttat 240
ttatattttt ttttttttaa ttgatgttta tgttttgggt tgaattgtaa gatatggaaa 300
taatggatgg tttagtttaa ggggtgttta ttgttagtag ttttttagta aaatagtaat 360
tttttagatt aataaagtga tttggaaagg tggtagtagg gttaatgtta agaaatattg 420
ttttttttta gtaaggaaaa tattggaagt ggtagtttga attggtggtt tatgaatttt 480
gggagtagta gttattttaa ttaaattaaa ttttttttaa ggaaaatgtt atattttaat 540
tattttggat tgttggaata tataaggggt ggggggattt atataattag tagttaattt 600
aaagaatagt aaagttaaat tttattttgt atatatttta gtaaaaattg taaaatgtgt 660
taaattgttt tatttgaaga atgttttaaa gttttgtggt tattttttat tttttatttt 720
ttatattttt atgtatattt taaaaattgt ttttaagaga ttataaggtt ggtaaggaga 780
ggagtatggg ggtgggaggg gttatttggg aagaaaatat ttaataggaa atttttagta 840
aagggaatta atatgtatgt tagtatatta tattttttta gtattaaatt attaaatttt 900
ttgttttgtt attaatgtag tttattttta atgtttttat tgtatgtaag agagataatt 960
tttttttttt tggtagaaga agggaaaaaa ataaatttat attgagtagt gtgttgatgt 1020
tgttatttgg tttgggttaa tttattgaag tatgttttga gagttttttt ttggagtatg 1080
taggaagatg gatttgtagt gtagaaattt aggatttgtt taggatttat tgatagaata 1140
tttatatagt taaatatgat tttttttgta ttgaatgttt gtaaaagtgt ttatttttta 1200
ggtgtggagg taaatggtat agaaaagttt atttaatttt attaaatttt gttttttttt 1260
tggtatgtag gttgttggtt ttttttttag ttttgaatta tgaattatga tagataagtt 1320
agttaaaagt ttgtaattga tttaaatgat tatttattat tttttaggtt tgtttggtta 1380
atttagttgg ggtggggggt ttttagaaag attttaagtt ttttttttaa gtaaatgttt 1440
gtaaaaagtt ttttgaatgt gttttggatt ttattttgat ggtgtttaga taaattttgt 1500
ttggattgtt tgtgtttttg gtattttagt ttttaaatag tttgtatggg ggaattttgg 1560
tttttttttt ggtaaagaat agattttttt gtttttgaat agtttatttt ttattatttt 1620
tgttatatag aatgaaagat tgaattgttt aatatatgtg agtgaatttt tggtgaattt 1680
tatttgggtt gttaattttt aaatgattta attagtttga tattattaat ttttaggatt 1740
tttatagagt ttaaaaattt ttagtagttt tgtaaaagta gatagttaga gaggtaggta 1800
gtgagtgtat aagttggtgt tttttttttt atttttattt ttaaaaataa tataagaagt 1860
gtttttaaaa tgagtagggg aggagttggg atgttgtttt tggtttatgt tgagtattaa 1920
tttttttttt tttatgtttt tttatttgtg gtggtttagt ttggaaaatg gtgttttgtt 1980
tgtgtttgtt tgttttttgt ttattgtttt gttttttagt ttatatttta aatttttttg 2040
tgtgtttttt tgtttgttaa attttttata atttatgtat ttttttattt tagaaaaagt 2100
atatgtaagt tttaaattgt agaatagaga atggttattt tttgggaaag gtttgggttg 2160
ggaaattttt tattttatag tttttttttt tttttttttt ttgtaagttt ttggttgaat 2220
tgattgtatt tgtttgtttt gtaatttagt ttttgagatt tatttatttt gaaaaatttt 2280
ttttaggttt tatgtagtat atagagtagt taggaattgg ttatttttgt tttaaatata 2340
tagttatata gaaaatgaga ttttagattt ggatttgaaa aaatagtata gtggttaaga 2400
gtatgggttg gggaattaaa ttgaattaga ttttaatttt aggtttgtta tttattagtt 2460
gtgtgttttg ggataagtat ttaatttttt taggttttag tttttttatt tgtaaaatgg 2520
gtatataata ttatttatgt tagtgttaat tgggaggata gaaataatat ttgtatggtg 2580
ttgggtaaat attatggaat aaatgttagt tgtgatttat ttttatattg tttaattggt 2640
gaaataaaat tttggggaga tgagatttat ttaaggttat atagtttaga gttgtgatta 2700
gaatttagtt ttttaggatg aaagtttaat gtgaaaatta tttgtttgtt tttatatttt 2760
tgtattttaa atagttatgt tggttgtttt ttttattata tatttatata tattttatta 2820
tataattaaa tgtatatttt gtaaattggt ttaagtatga tagttttata ttttgaagga 2880
ttagtttttt tttattttag tttttgttta atttttttat taaaatatag taattttaag 2940

ggaaaatgat tttttagatt ttttatattg atttaattta taggaagaag agttttttta 3000
gttagtttta aaatattaag aaggatagat gtagtattta taaataaatt tagttggatg 3060
gagagtttta aaagggagag ttaatttatt ttgtagttat tttgtataat ttgtttaaag 3120
gatgtaatat atattaaata aaggaaattt tggagaggaa ttaaaatatt tttttttttt 3180
tattgttttt gtagtattta tttgtaattt ttttataaaa tagggtaggt aagtggttag 3240
gtagggaggg agagtttttt tgtttaatgt agggaagagt attgttttag tttttttttt 3300
gtggatagtg ttggaatgtg tattggtatt tgggtttttt aaaagtttgt tagggggtat 3360
tttattttat gttgaaatat tgttttgttt agtggagttg ttaagtggta tttgtggtgt 3420
aatatgaaaa gtatgtattg tatatattta ttaagttaaa gattttttta tgaaggtata 3480
ttaaaataat ttttagttta gtaagtttag tttttgttat tgaaaagttt agggtaataa 3540
aggggtgaga tttttgttga atttgtgttt gttatttttg tgtttttttt ttttttttta 3600
tatggttatt attttgtaat agagatttat tgtaagaaat tttttaatta taaagaaaat 3660
atatatttta agtaagagaa tatagaggaa tttataaaat atttaatata ttgttattaa 3720
ggtagttgta aagatatata aagtattata ttatgtgtta atttttattt ggtttatgag 3780
gtttatggtt ttaaatgttt atttaaggtt tttagagttt tttggaaatt gagtttattt 3840
ttattggata gtggaggtgg atgaaaagtt gttagataag agaagaggaa atagttatat 3900
tggaagtaga gtaggagaaa tttaagttag agatgagaaa tatggtatta ttgataggta 3960
tgtgtgtata gggatttgtt atttagggga ggttgttgta tatttttgtt taatattttt 4020
ttaaagagta gagatgtatt ggttttaggg aagtgaagat ggggatgggt gtttttaaaa 4080
ttttgtttat ttttattttt taagttatgg aattagtttt attgagtgag attattgttt 4140
ttgaattttt tatttttata ttgtattatt gtataaaaat aataatatta gtgtgttttt 4200
ttttgtgtgt ggtaaaggtt gggtgatttg atgttttttt tttttgtgta agaaaatgta 4260
aaatatataa ttattttgaa gtttttaatt gagaaatgaa tttgggtttt gaaatgaata 4320
attagtattt aaatttttat gaaaggaata gtatgggttt tttataatat gataatattt 4380
gttagttaat taattatttt ttattatgaa ttaattatgt tgatagtaat aattagagtt 4440
attatttaat ttaagtatta atttataaaa tagatttatg atttagttat atgttaatta 4500
tttttttatt agtaaatgtt tatatttttt gaaaatttga ttttgttttt aaatttttta 4560
ttttaagtaa aatgaaggaa agatatataa ataagttata attttttaaa tatagatttg 4620
tgtattataa agattaatga gtagggtaga aagtgaaaaa tagattgatt tgtttgtaat 4680
gaaatgtaaa ttattgatat ttattttaaa tattagttat agggttggaa gaaatttttt 4740
taaatatttt tttgttatgg tataaaataa tattttgata tgtgttattt ttattttttt 4800
tgtgtttgat taatgattta ataaatgtat ttttaattaa taagtaaata aaaagtgaaa 4860
aataaaaagt aaaattttga gagtaaaatt tattttgttt tagagtaatt gtatatttgt 4920
tagtgtgata tagtatattt gaaatttata attataaaat aaaaatttat agttagggtt 4980
tagggtttat atttgttttt ttggtttttg tatagtaatt atttattgga taatttagaa 5040
agaaatatta tgaattatgt tttgtaaata tgtttgaatt ggagagtttt ttgaagaaag 5100
agtagttgtg tttgtgtatt gtattgtagt tggttgtgtt taaaattggg ttaatatttt 5160
gtgatgttta aataagaaga tttttagagg tgtagatata ttttttttta ttataggggt 5220
agatatgtaa agtgtaggta gatataggta tagggttggg ggaggtggat attgattgtt 5280
ttaaagatat gtttagtttt tttatgttgt tttggttttt tgtttgtttt ttaatttttt 5340
tttttgttta attttttttt gttatagttt tgttagaaga ataaatggtt agtattgagt 5400
gatttgtgtg ttttagtagg gaagttaagg tagagatgtt ggtagtatga ggtaagtatg 5460
aggttttttt tagggttttt atattagtag gtagagttgt ttttgtgttg gaagggagtg 5520
ggggaagttt taatagtaat aatatatgga aattttgttt gtggtttttt agtatgaata 5580
tttgtttata taataggatt gttgatgttt gtggatttta tattaaaaga tattaatgta 5640
ttttttaaat gaatttgtaa ggtggttttg ggtatgagta tagtgatttt ttagaaattt 5700
tagaaatata ttttttttaa aaatgttttt ttattttgag tgttggttat tgtgtttttt 5760
atttggtttt tttttttta gtttttttgtt tttttagatt ttttttttata aagtttttttt 5820
atttggttat atataaatat tgttttttta agataagggt atattttttt ttttttatat 5880
ttttgaatta ttttttttag tgtttttgtg ttttttgttg ttgatgattt ataaagatag 5940
ttttttaagt ttaatagttt tggttggttt taatttgaaa gttaaatata atttttgttg 6000
aatgttttag tattgagtag tgagttgtga attatttttt agttgattgt tattgttggt 6060
ggtattgttt taatattagt tttggggaga tttatttttg gttattttat tttgtttata 6120
tttttttttt taaatttaga tttttatttt tttttttttt ttttaaaatt ttatttttat 6180
attttttatt attttttata aatttagatt taagtaatat atgtttataa aaatttaaag 6240
ttttgtttaa aagatataaa gtgaaaagta aaagtttttt atatgtattt attaaatgtt 6300
gaatatttat tatgtgttag gtattttaat tggtgtttgg tatagatttg tatatttttt 6360
tgggattgtg tgggggggtg ttgtttgttg ttattatatt agtatggttt tatgatatat 6420
attgttttgt aatattttta aatttagtaa tattttttga atattttttt aaaataaa 6478

<210> 341
<211> 2534
<212> DNA

<213> Artificial Sequence

<220>
<223> chemically treated genomic DNA (Homo sapiens)

<400> 341
   aggtaggaaa gtgggatagt tggggagttg gattttattt tttgtgagtt ttgttggtat 60
   ttgatggtat gtggtttgga gagggtaggt gatttggtgt ggagggttag agggtaaatt 120
   tttaaataag tggtaatagg ttattaattt gaaaggaaaa attgtgtagt gatgggaaat 180
   gtgtttaata aatttattgg gtgattaatt ataaaggttg ggttggagtt ttagaggttg 240
   tttgttaaat attttattaa gtggtatttt gaaagttgtt atttgtgtat tttgggagtt 300
   tagaggggat tttgaggggg aatgaggttt ggaggatgga attattttta ggtagattga 360
   gaaggagttt ggattttatt tttaaatata gtttggagtt tataggttag aggttttaat 420
   gggagaaaag ttaaaggaag agggtgtaga aaggagtttt agggaattgg tggttatgtg 480
   attttgagta aattttattt ttttttgaga tttagtgttt ttatttttat ggttttgtgt 540
   gtgttataga gatatggtgg ggattaaatt tgattgtgat atgaaagtgt ttgggaaatt 600
   ttatggtttt atttaaatat gagttatttt tatttgaatt aaggggggaa gttatttggt 660
   aggattagga attttatttt tttgaatttt tatgggtttt gttgaggttg aagtagttag 720
   gggttaaagt tagtttttag tttttggaag ggtattgtga aagtggattt gatttgagaa 780
   gttgtttttt gatgtgggta gttatgtgat gttagttttg aataagaggg ggtagtttgg 840
   agtttggaaa ggtgttagtg taggtggggt ttatgtttag attttttttg ttgattgttt 900
   tgatgattta tttttatatt ttagtttttt tatttttatt gtagagttgg aaagggtgtg 960
   gggaagagag gagagggagg taggttttgg gttttggttt tgttttttgt ttttttttat 1020
   ttttttttgg gtttggttat ttagttaaaa ggtaggttaa gagtaggaga gatatagagt 1080
   ttggtattgg ttttaggtag tagttagttt gttgtttgtt tgtgtgtttt tagagttatg 1140
   gagagagtta gtttgattta gaaggttaag ttggtagagt aggttgaatg ttatgaggat 1200
   atggtagttt ttatgaaagg tgttgtggag aagggtgagg agtttttttg tgaagagtga 1260
   aatttgtttt tagtagttta taagaatgtg gtgggtggtt agagggttgt ttggagggtg 1320
   ttgtttagta ttgagtagaa aagtaatgag gagggtttgg aggagaaggg gtttgaggtg 1380
   tgtgagtatt gggagaaggt ggagattgag ttttagggtg tgtgtgatat tgtgttgggt 1440
   ttgttggata gttattttat taaggaggtt ggggatgttg agagttgggt tttttatttg 1500
   aagatgaagg gtgattatta ttgttatttg gttgaggtgg ttattggtga tgataagaag 1560
   tgtattattg atttagtttg gttagtttat taggaggtta tggatattag taagaaggag 1620
   atgttgttta ttaattttat ttgtttgggt ttggttttga atttttttgt tttttattat 1680
   gagattgtta atagttttga ggaggttatt tttttggtta agattatttt tgatgaggtt 1740
   atggttgatt tgtatatttt tagtgaggat ttttataaag atagtatttt tattatgtag 1800
   ttgttgtgag ataatttgat attgtggatg gttgataatg ttggggaaga ggggggtgag 1860
   gttttttagg agttttagag ttgagtgttg tttgttattg ttttgttttg ttttttttag 1920
   ttttttattt tgttgagagg attagtatgg ggtgggaggt tttatttttt ttttttaggt 1980
   gttgtttttg ttttaaaggg ttttgtggag agggattggt agagttgagg ttatttgggg 2040
   ttggggattt tatttttttt gtagttgttg agtgtattta attattggtt atgtttttat 2100
   ttttgttttt tgtatttgtt tttttttgat tttaggatta ggttattttt tttttttttt 2160
   tgtttttttt ttgtttttgt tgtttttgat tgtaggaatt gaggagtgtt ttgttttgtg 2220
   gttgagaatt ggatagtggt aggggttgga gatgggtgtg tgtgtgtgtg tgtgtgtgtg 2280
   tgtgtgtgtg tgtgttagtg taagattgag attgagggaa agtatgtttg ttgggtgtga 2340
   ttatgttttt ttttaataaa gtttttttgt gatatttttt ttgttttttt tttagttttt 2400
   ggtgatgggt tgggagtggg attggaattt gatttagaga ttttgatttt ggatttttga 2460
   gttagggttt tgaatttttt aggtggttta gtggtttgta tgtaagattt tgagtttagg 2520
   tgaggttggg gttt 2534

<210> 342
<211> 2534
<212> DNA
<213> Artificial Sequence

<220>
<223> chemically treated genomic DNA (Homo sapiens)

<400> 342
   ggattttggt tttatttgga tttaaagttt tgtgtgtggg ttattgagtt atttagggag 60
   tttagggttt taatttagag gtttaaagtt agggttttta agttagattt tagttttatt 120
   tttagtttat tgttaagaat tggaagagag ataggaggag tgttataggg gaattttatt 180
   gagaggaaat atggttatat ttagtagata tgtttttttt tagttttggt tttgtattgg 240
   tgtgtgtgta tatatatata tatatatata tatatatatt tatttttagt ttttgttatt 300
   gtttagtttt tagttataag gtgggatatt ttttaatttt tatgattaga ggtagtaggg 360
   gtaggaggga ggtaagagga ggggagaagt agtttggttt tggggttggg aggaagtggg 420
   tgtggagagt aggggtgggg gtatgattag tggttaggtg tgtttaatag ttgtaagaag 480
   agtgggattt ttagttttag gtggttttag ttttgttagt ttttttttat ggagtttttt 540
   ggagtaagaa tagtgtttag gggagaaggg tggggttttt tattttatat tagttttttt 600
   ggtagggtgg gggattggag ggggtagggt ggggtggtgg tgggtaatat ttagttttgg 660
   ggtttttggg gagttttgtt tttttttttt ttggtgttgt tggttgttta tagtgttagg 720
   ttgttttgta gtagttgtat gatgagggtg ttgtttttgt aggagttttt gttgagggtg 780
   tgtagattag ttatggtttt gttgaaagtg gttttggtta gagagatggt tttttggggg 840
   ttgttggtga ttttgtagtg gaagatggaa aagtttaggg ttaggtttag gtggatgggg 900
   ttggtgggtg gtattttttt tttgttgatg tttatggttt tttggtaggt tgattgggtt 960
   gagttaatga tgtgtttttt gttgttattg gtggttattt tggttaggta gtggtagtag 1020
   ttatttttta tttttaggta gaagatttgg tttttggtgt ttttggtttt tttgatgagg 1080
   tggttgttta gtaggtttag tatggtgttg tatatgtttt ggagtttagt ttttattttt 1140
   ttttggtatt tatgtatttt gggttttttt ttttttgagt tttttttgtt gtttttttgt 1200
   ttaatattgg atagtatttt ttaggtagtt ttttggttgt ttattatgtt tttataggtt 1260
   attgagagta ggttttgttt tttgtaggag agttttttgt ttttttttat ggtgtttttt 1320
   atgaaggttg ttatgttttt atagtgtttg gtttgttttg ttagtttggt tttttggatt 1380
   agattggttt tttttatggt tttggggata tataggtggg tggtgggtta attgttgttt 1440
   gggattaatg ttggattttg tgtttttttt gtttttggtt tgttttttgg ttgggtggtt 1500
   aggtttagag aagggtgggg aggagtaggg ggtgggatta gggtttaaga tttgtttttt 1560
   tttttttttt tttttatatt ttttttggtt ttgtagtaaa ggtaaaaagg ttgggatgtg 1620
   ggggtgaatt attagaatag ttagtaggag aaatttgggt gtgggtttta tttgtattgg 1680
   tattttttta ggttttaggt tgtttttttt tgtttggggt tggtattata tggttgttta 1740
   tattaggaaa tggtttttta aattagattt atttttatag tgttttttta ggggttaagg 1800
   attggtttta gtttttggtt gttttagttt tgatagagtt tataaaggtt taggaggatg 1860
   gggtttttaa ttttgttagg taattttttt ttttggttta ggtgaggata atttatgttt 1920
   aggtagggtt atggagtttt ttaagtattt ttatattatg attgaattta atttttatta 1980
   tgtttttgtg atatatatag gattatagag atgggaatat taagttttag agaggggtga 2040
   gatttgttta aagttatata gttattaatt ttttgaaatt tttttttgta tttttttttt 2100
   ttagtttttt ttttattgag gtttttgatt tatgagtttt agattgtgtt tggaagtgag 2160
   atttaggttt ttttttagtt tatttgaaga tggttttatt ttttaggttt tatttttttt 2220
   tagggttttt tttgagtttt tagaatgtgt aggtggtagt ttttagagtg ttgtttaatg 2280
   aagtgtttaa taagtagttt ttgaagtttt agtttagttt ttgtaattag ttatttagta 2340
   ggtttgttgg atatattttt tattattata taattttttt ttttaagttg gtggtttgtt 2400
   gttatttgtt tgaggattta ttttttggtt ttttatgtta ggttatttgt tttttttaag 2460
   ttatatgtta ttaggtatta gtggggttta tagagggtgg ggtttagttt tttggttgtt 2520
   ttattttttt gttt 2534

<210> 343
<211> 6558
<212> DNA
<213> Artificial Sequence

<220>
<223> chemically treated genomic DNA (Homo sapiens)

<400> 343

taagattata aattgttttt tattagtggg gaaggtttta aatattatgt atttagtata 60
agtaggtgta attaattgta atggtttgtt ttgttatttt tgattaatat ataattatta 120
ggttagaatt gtattttata agttttttgt gtttagttat tttgtatata ttaatttttt 180
taagttttgt gttttaaaag aattgtatag tgttgggaag ttttttattt attttaattt 240
ttagggtatt gagtttgaat ttgaattagg tattaggtat gttttattat tataaaagat 300
gtaaaaagat gtgtatagtg gtgtatattt atagttttag ttattgagga ggttaatgtg 360
ggaggattat atgaggttgt agtgagttgt gatggtgtta ttgtatttta gttttggtga 420
tagtgagttt atgttttaaa taagtaagta aataaaaatt aaaaagaatt tagtttatag 480
ggtatttgaa ggtaagagga aaagatgtta gaattagaga tggggagaag atgggttttt 540
ggtgtttgtt gaggttgaga aatgagatag ataggttgag tgtggggtgg agagaggatg 600
ggtagagaga ttgaggttgg tttgaatgga aatgaaatgt tagggttttt agggttattg 660

gggaataatt ggagttttta ggaaaggttt aatgttgtga ttatttgtgt ttgttatgtt 720
tttttatttt ttattaattg tgtgaatttg gtagattttg agttttagtg tttttttttg 780
tgaagtgggg ttattttatt ttaatttttg ggattgttgt gtgaattaaa tggggtaatg 840
tatggagagt atttgatgta tagtgagtgt tttaaaattt tagtttgtat tttttagtaa 900
aggatatgta tatgtttatt gtgagtgata aatttaggat gatttgaatt taatgtgata 960
atgtgggttt ttgtatgttg gttatgttgt tgggagatat ttatggattt aattagtata 1020
ataggggaaa taaattattt aatgtatttt gttaagatag aatattttag aatttatttt 1080
gtggggtggg gtataataaa gggggttttt ttgttgaaaa tgtttaagtt taggtttgtg 1140
gtattattta attaaggttg atagttatat agtaagttag aggtaatgtt aggatttaaa 1200
ttaaatttgt ggtttttata atgaggttat tttttttttt tttgaatggt ttggggaaag 1260
ggggtgggtg ggtagaattt ggtagtggtt aattttttat ttttgttttt tggttttttt 1320
ttgtttttat ttttaggttt gtattgattg attgattgag atagggtttt gttttgttgt 1380
ttaggttgga gtgtagtggt atgattatgg tttattgtag ttttaaattt ttaggtttaa 1440
gtggtttttt tgttttttat tttttgagta tttatatttt taggttttta aaatggtttt 1500
taggtatttg gttgttgttt tagatattta tttgggtttt tgggtaggga ttgtttggga 1560
aattttattt atgtgaagta ggtgtgggtg taggaaggtt gtttggaaat gaattagtat 1620
tgttttttgt ttgagttgta agtagggtgt tagagggttt ggtggataag aaagggagga 1680
tgataggagg ttggtattgt aatgatatgt tttagttatt agagggtatg aagtagttgg 1740
gtaaaatttt gtggggttgt tggtggaaaa tttttggtat ttggagtttg gagatggggt 1800
ggatggaatg tgaggattta gttttttgag gttgggttgg ggtagagtta ttgttttgga 1860
tgtttgtagg gtttgtttgt gttttgatta ttttgttttt gtagatagtt ggagaatgtg 1920
agagtgggat tgggattgga ttttaggggt tattttgtat aatttttttg ttttgttgtg 1980
ggggagggag ttgtttaagg ttatgtagta agttagtggt aaatgaatat gattattatt 2040
agttttaggt atatggttat ttgatgggtg tagttgtagt tttagttttt gagatagaga 2100
tatttgatta aggataggtt tttaggagtt gattttagtg atttgtggtt ttgttgttgt 2160
ttttgttttt ttttttggtt tttttatttg attgattttt tgtttttttt gtttgtttgt 2220
ttgtttttgt ttttgtttgt tggggggttt gtttaatttt tttattgggt tttgggagtt 2280
gtagtttttt gttgttattt tttagggatt tgtagttttt tgaagttttt ttttgatttg 2340
ttgttttggt ttttattttg ggatttagag gagaggtgat tattttgtag tatagttagt 2400
ggtgtgattt tatggggtga gaaggatttt tttgttttta agtatttttt tagtgatttt 2460
tgttgttatg tggtagttgt aagtattggt tggtatttgg tgtgggtgag atttttattt 2520
tatgtagaaa tgagtaagat tggtgagttt attatgtggg ggtgaggttg agagaaaata 2580
agtatatagg tgatttagtt aaaattagaa ttttttaagt atatatgaaa agggtaaaag 2640
gggtgttttg tataggatag aataggtaga tattgaattt ggttgggttt tgggaaggtt 2700
ttttgtagtg gtttttgaag ggggggttgg attttagtag gatagagggt atgggtatgt 2760
gtgggtatgt tttgaataga ggggttagtg taagttgagg gttttggtta tattagttgt 2820
atgtgttggt gtgtttaagg gatatgtagt agtaggttga gtttggagtg ttttattgtt 2880
aggtttttta aaaattttta attttaattt aattttattt tatttttatt ttaagttttg 2940
gtatatatgt gtagaatgtg gtttgttata taggtatata tgtgttatgg tggtttgttg 3000
tatttattaa tttattattt aggttttaag ttttgtatgt attaggtatt agttttaatg 3060
tttttttttt ttttgttttt attttttttt ttgtaattgt ttataggttt tggtatgtgg 3120
tgtttttttt tttgtgttta tatgttttta ttgtttaatt tttatttatg agtgagaata 3180
tattgtttgg ttttaaggga tagttatggg gatgtattgt agtttttgag tagggaaggt 3240
tttgtggagg tttttagtta aaaggaaaga atggttgtga aaattgatgt attgtgtttt 3300
tttgtttttt atttttagtg tgaagggttt ttattttgag ttttatttga agtaggtttt 3360
gatgggaaga taagtagtat gaggggttta agtattgagg ggagtaaggg atatttggtg 3420
gttgtgttaa ggtgtagaag aggatattgg gggttttaag gtttgatttt atgtatattg 3480
tttaggttgg tttttaagtt atatggtgat tgttaggaag ggattagttt gtttttagtt 3540
tgattttttt gttatgttat tatttaaagt tttttttggt agggtttgtt tggggttttt 3600
gtgttagtgt tttttttgtt aggttgggtt ggggttttta tttattgttt tgggattgtt 3660
gttgttttgg ttttggggga ggagggtgtg ttgttgagtt attgtttggg tatttgggtt 3720
tggaattttg ggtgagttat ttagggttga ggtagagggg ttgggggagg ggaagaagtt 3780
atttgatagt tggagtaggg aggggagttg gggttatagg aagggtggtg ttttgatgtt 3840
tagatgggtt gggatagata aagggttaag gaggaagggg ttttgggagg gggtagtttt 3900
tttttgggtt gggttttgaa tggttatagt gtttgttttt ttttgggttt ggggaggata 3960
tgtgtgtggg gggtagtgag agagggttgt ggttgagggt tgtgttttag gtttggattt 4020
tggtttggga agttgtttag ttggtgtttt tagttttggg tagggatgta tttttattta 4080
tttatttagt ttttaagttg gagaagagga ggttaaagtt tttttgttta gtttttaatt 4140
atttgggatt tttttatgtt ttttatagat tgtggtttag tttaattgtg gttgtgtgta 4200
gagtaatttt attttttatt gtttttttat ttttttagat atttttttat atagagggat 4260
ttttttaggt atttttaagt atatttagtt attttattat tttattaaga ggtattttgg 4320
tgttggttat taaaagttat tttattttat ttatgttttg aagttagttt tgtttttttt 4380
tttttgatgt tttttagttg ttttttttgg tttttagttt tttaagggtg gttttaggtt 4440

gttttttttt tatatatata ggtgtatgta tgtatatgag tattggatta tgaagtttta 4500
gtgtgtgttt atagtttttt atataggagt gggttgtgat ttataggtat gttatgagaa 4560
tgaggtttgg tattagtttt taggttttag agtaggggtt gttttttttt attttggttt 4620
aggatgttta gtttttatga tattttttat tttttattgt ggtttgggtg ggtttagggg 4680
ttgtttttga tttggtttag agtttttttt tagttggtgg tggagttggt attttttggg 4740
agggaggggg ttgggaggga atgagtggga atggtaagag gttagggttt ggtgggatta 4800
ggttgaggta ggtttggttt ttttaaaatg ttaagttggg ggttagtggg gtttatatat 4860
aaatttttat tttgggagtt tggttgtttt gttttttttt ttgggtttgt ttttgttatt 4920
tggtttggtg agtatttttg ttttgttgag ggtagggtgg ggaggatttt tgtgggtttt 4980
tgtttttgtt tttatagttt ttttatttta gtttttttgg tgggattaat ttgggttttt 5040
ttgggttttt ttttttggaa gaattttttg tgaagtgttg aagtgttgat tgaagggttt 5100
tttttttttt tttttttttt tgagatggag ttttgttttg ttgtttaggt tggagtatag 5160
tggtgtgatt ttagtttatt gtaaattttt ttttttaggt ttatgttatt tttttgtttt 5220
agttttttga gtagttggga ttgtaggtgt ttattattat gtttggttaa tttttttgta 5280
tttttagtag agatggggtt ttattatgtt agttaggatg gttttgattt tttgattttg 5340
tgatttattt attttggttt tttaaagtgt tgggattata ggagtaagtt attgtgtttg 5400
gttgattgaa gggttttttt ttaggttttt ttgtgaggtt ttagtgtagg ggttgttttg 5460
aggttttttt ttggatattt tagtttaggg gttttttttt gggggtttag gtttaggagt 5520
aggaggtgtg tatgtgggtg ttgttgtaaa aagaattttg agattttttt tttttttttt 5580
tttttttttt gtaaagtttt ggattttagt aggattaagg tgtaagaggt aggggtttta 5640
agattttgtt tgggttatgg ttttaagtag taaagttttg ttttttgttt tggtgaaggt 5700
agggttggta tgatgggttt agggtatgtt ttgtttttgg tatagttttt ttggttttat 5760
tttgaaattt gtttaatttt tttaggttgg tttgagtatt tttagaggtt ttgttgttga 5820
ggtttgtttt attttgattt taaggtaatg aatattttat atttttaatt ttaattttaa 5880
taggattttt tttggtggag agaatgttaa gttgttttta ttttatttat gtttttgttt 5940
gtttagaggt ttaggggttt ttagggtgag gggagatata ttttttattt tttgggagtt 6000
tttagtttga gagaggaaat atttttgttt aagggagttt ttagttagat ggtagagaga 6060
gatgtttttg gttttaggag ttttgagttt aaggagggaa atgatttttt tagggagttt 6120
tttgttttta ggttgtagtt atggtttttg ttagattgta taggagtttt tatttgttag 6180
ttggtgtatg tggttttgtt ttttagagtt tgtgtagatg ttattaaaat gggattttgg 6240
ttattttgtt attttttttt tggtagatat taaaatgggg agttttgttt ttaggggggt 6300
gttttaagtg ttattagagg aggtttggtg atttttagat ataagggaag ttttagtgtt 6360
tgtttttagg gtgagatgga ggtattgttt ttggttttag ggaattatag tttgaggggg 6420
agatgtagtt tttgtttttt tatttagaga ggggttttgt gaggtggttt gggggtatag 6480
ggtagaagtg gattttatag gttgagttaa ggttttaaga gttttagtag tgtatttatt 6540
atttggtatt tttgtagt 6558

<210> 344
<211> 6558
<212> DNA
<213> Artificial Sequence

<220>
<223> chemically treated genomic DNA (Homo sapiens)

<400> 344

gttgtagagg tgttaggtga tgggtatatt gttgaggttt ttggggtttt agtttagttt 60
gtaggattta tttttgtttt atgtttttaa gttattttat aaaatttttt tttgaatggg 120
aaggtagggg ttgtattttt tttttagatt gtggtttttt gaggttggag gtgatatttt 180
tattttattt tgagggtaga tgttaaagtt ttttttgtgt ttgggagtta ttaagttttt 240
tttgatggta tttgggatat ttttttgagg gtagggtttt ttattttagt gtttgttaga 300
agggaaatga tagggtgatt agagttttat tttgatggta tttgtgtagg ttttggggag 360
tagggttata tgtattggtt ggtagatggg ggtttttgtg tagtttggta ggagttatgg 420
ttatagttta ggagtaggaa gttttttgaa ggagttgttt ttttttttag atttgggatt 480
tttgaagtta gaggtatttt tttttgttat ttaattggaa gtttttttgg gtaggagtgt 540
tttttttttt agattaggag tttttagagg gtggggaatg tgtttttttt tattttgaag 600
gtttttgagt ttttaggtag ataggggtat ggatagggtg ggggtaattt aatatttttt 660
ttattaggaa ggattttgtt ggaattagaa ttaaagatat gaaatgttta ttgttttggg 720
attaggatgg gatagatttt agtggtaagg tttttgagaa tatttagatt agtttggaaa 780
ggttaggtag gttttagggt gaggttagag gagttatgtt agaggtaggg tatgttttgg 840
gtttattatg ttagttttgt ttttattgag gtagggggta gagttttgtt gtttggggtt 900
atgatttagg tagagttttg agatttttgt tttttgtatt ttagttttgt tagaatttag 960

gattttgtaa aaaaaaaaaa aaaaaaaaaa aaaaatttta ggattttttt tgtagtaatg 1020
tttatatgta tattttttgt ttttaggttt agatttttaa agaaggtttt ttagattgag 1080
atatttaggg gagggtttta gagtaatttt tgtattgaga ttttatagag gaatttggag 1140
aaaaattttt tagttggttg ggtgtggtgg tttatttttg taattttagt attttgggag 1200
gttgagatgg gtggattatg agattaggag attgagatta ttttggttaa tatggtgaaa 1260
ttttattttt attaaaaata taaaaaaatt agttgggtat ggtggtgggt gtttgtagtt 1320
ttagttattt gggaggttga ggtaggagaa tggtgtgaat ttgggagggg gagtttgtag 1380
tgagttgaga ttatattatt gtattttagt ttgggtgata gagtgagatt ttattttaaa 1440
aaaaaaaaaa aaaaaaaaaa attttttagt taatatttta gtattttata gagagttttt 1500
ttaaggggga aggtttagag aggtttaggt tgattttatt agggaagttg gaatgagaga 1560
attgtggaga taaagataga gatttatggg gatttttttt attttgtttt tagtaggata 1620
gaggtattta ttagattagg tggtagagat agatttagga aggagagtaa ggtagttagg 1680
tttttagggt gaggatttat atgtgggttt tattggtttt taatttggta ttttaaggaa 1740
attaaatttg ttttaatttg attttattaa attttggttt tttgttattt ttatttattt 1800
ttttttagtt tttttttttt tagagagtgt tagttttatt attagttggg ggagggtttt 1860
aggttaggtt aagggtagtt tttgagttta tttaggttat agtgggaagt gggaggtgtt 1920
gtggggattg ggtattttgg attggggtga ggggaggtaa tttttgtttt ggggtttgga 1980
gattggtgtt aggttttatt tttatgatat gtttgtgagt tatagtttat ttttgtgtga 2040
gaggttgtga gtatatgttg agattttatg gtttagtgtt tgtgtatata tatgtgtttg 2100
tgtgtgtgag agagaagtaa tttggggtta tttttaggaa gttgggggtt agaggaggta 2160
gttgggggat attaggagga gaaggatagg attgatttta gggtatggat gaagtggagt 2220
gatttttaat ggttagtatt agaatatttt ttaatgaggt aatgaggtaa ttaagtgtgt 2280
ttagaaatat ttgggaaggt ttttttgtgt aggaaggtgt ttggaaggat ggggaagtag 2340
tgagaaatgg ggttgtttta tatatagttg tagttgggtt gggttatagt ttgtggggag 2400
tataaggaag ttttgagtag ttaaaggttg aataggaaaa ttttggtttt ttttttttta 2460
gtttgagggt tgggtgggtg ggtaggggta tatttttatt taaggttgaa aatattagtt 2520
ggatagtttt ttaagttaga atttaggttt gaagtatagt ttttagttat agtttttttt 2580
tattgttttt tatatatatg ttttttttag atttggagaa aggtaaatat tgtggttatt 2640
tagagtttag tttaagggag ggttgttttt ttttagggtt tttttttttt tggttttttg 2700
tttattttgg tttgtttggg tattagggta ttgttttttt tgtggtttta gttttttttt 2760
ttgttttagt tgttgagtag tttttttttt ttttttagtt tttttatttt agttttaagt 2820
gatttatttg aggttttagg tttagatgtt taggtagtga tttagtggta tatttttttt 2880
ttttagggtt agggtagtag tagttttaga gtagtaggtg gaagttttag tttagtttgg 2940
tagggaaagt attggtatag agattttaaa taggttttgt taggaggagt tttggataat 3000
gatatggtag gaggattaga ttgagaatag gttggttttt ttttagtggt tattgtgtga 3060
tttgggggtt agtttgagta gtgtatatga agttaggttt tggggttttt agtgtttttt 3120
tttatatttt ggtatagtta ttgagtgttt tttgtttttt ttagtatttg aattttttat 3180
gttatttgtt tttttattga ggtttatttt aagtagaatt tggaataaaa attttttata 3240
ttgagggtga gggataaggg agtgtaatgt attgattttt atagttattt ttttttttta 3300
attaagggtt tttatagggt tttttttgtt tagaaattgt agtgtatttt tatggttgtt 3360
ttttaaagtt aggtggtatg tttttattta taagtgggag ttgaataatg agaatatatg 3420
gatataggga agggaatatt atatattagg gtttgtgggt agttgtgggg agaaggatgg 3480
gggtaagggg agggagagta ttaggattaa tatttgatgt atgtggggtt taaaatttag 3540
atgatgggtt gataggtgta gtaaattatt atggtatatg tatatttatg taataaatta 3600
tattttgtat atgtatgtta gaatttaaag taaaaataaa ataaaattaa attaaaatta 3660
aaaattttta aaaagtttgg tagtgaggtg ttttagattt ggtttgttgt tgtgtgtttt 3720
ttaaatatat tggtatatgt aattagtgtg gttaagattt ttggtttgtg ttgatttttt 3780
tgtttagaat gtgtttatat atgtttatgt tttttatttt gttgaaattt aatttttttt 3840
ttaaaggtta ttgtagggag tttttttagg gtttaattgg atttagtgtt tatttgtttt 3900
gttttgtata aagtgttttt tttgtttttt ttgtgtgtat ttagagaatt ttgattttga 3960
ttgaattatt tgtgtatttg ttttttttta gttttatttt tatatggtga gtttattggt 4020
tttatttatt tttgtatgag gtaagggttt tgtttatatt aggtgttaat tagtgtttat 4080
ggttattata tggtaatagg ggttattgga ggagtgttta ggagtaaggg agtttttttt 4140
attttgtgaa attatattat tgattatgtt atgaaataat tatttttttt ttggatttta 4200
agagtggaat tgagataatg ggttggaaaa gagttttaga gagttataaa tttttgagga 4260
gtgatagtag gaaattgtgg tttttaggat ttagtgaggg agttgagtaa attttttagt 4320
gggtagagat ggagataggt aggtagatga agaagataaa gagttagtta gatggaaaag 4380
ttagggagaa aaatagagat agtagtagag ttgtgggtta ttagggttag tttttgaagg 4440
tttgttttta attaggtgtt tttgttttag gaattgaggt tgtgattgtg tttattaaat 4500
ggttatatat ttgagattgg tgataattgt atttatttgt tattaatttg ttgtgtaatt 4560
ttgggtaatt ttttttttta tggtagggta ggagagttgt atggaatggt ttttagagtt 4620
tgattttaat tttattttta tattttttag ttgtttataa aggtagagta gttaagatat 4680
aagtaggttt tgtggatatt taaagtagtg attttgtttt ggtttagttt taggaagttg 4740

ggtttttata ttttgtttat tttatttttg ggttttaggt gttagaaatt ttttattagt 4800
ggttttgtgg gattttgttt agttgttttg tgttttttgg tggttaaggt gtgttattgt 4860
agtgttggtt ttttgttatt tttttttttt tgtttgttag attttttggt gttttgttta 4920
tgatttaaat aggagatagt gttgatttat ttttaagtgg tttttttata tttatatttg 4980
ttttatatag atgaggtttt ttggatagtt tttgtttaga agtttaggtg gatgtttgag 5040
atggtagtta gatatttgga agttatttta aaagtttagg gatatgggta tttgggagat 5100
aggaggtgga aagattattt gagtttagga gtttgaggtt gtagtgagtt atgattgtgt 5160
tattgtattt tagtttggat gatagagtaa gattttgttt taattaatta attaatgtaa 5220
gtttagagat aagggtagga gaaaattagg ggatagaagt gagggattgg ttattgttaa 5280
gttttgttta tttatttttt ttttttaggt tgtttagggg aaagagaaat ggttttattg 5340
tgggggttat aggtttagtt taagttttga tattgttttt ggtttattgt gtgattgttg 5400
attttggttg agtggtgtta tgaatttgag tttaaatgtt tttagtagaa ggattttttt 5460
tattatgttt tgttttataa aataagtttt gaggtatttt gttttagtaa aatgtattaa 5520
ataatttatt ttttttgttg tattaattgg atttataagt gttttttggt agtatgatta 5580
gtatgtgagg atttatgtta ttatattggg tttaggttat tttggatttg ttatttataa 5640
tgggtgtgtg tatatttttt gttggggggt gtagattgaa attttgaagt atttgttgtg 5700
tgttaggtgt tttttgtata ttattttatt taatttatat aataatttta ggagttggaa 5760
taagatgatt ttattttata gaggagaata ttgagattta aagtttgtta aatttatata 5820
attagtaagg ggtggggagg tatgatgaat ataggtggtt ataatgttaa atttttttta 5880
gaagttttaa ttattttttg ataattttga gagttttaat attttatttt tatttagatt 5940
agttttagtt tttttgttta tttttttttt attttatatt tagtttattt gttttatttt 6000
ttaattttag taggtgttag aagtttattt tttttttatt tttgattttg gtattttttt 6060
tttttgtttt taaatgtttt gtggattgga ttttttttaa tttttgttta tttatttatt 6120
tgagatatag atttattgtt gttaaggttg gagtatagtg gtattattat agtttattgt 6180
agttttatgt gattttttta tattaatttt tttagtaatt ggggttatag gtgtgtgtta 6240
ttgtgtgtat ttttttgtat tttttgtggt gatggagtat gtttgatatt tgatttgaat 6300
ttgagtttgg tattttaagg attggaatgg gtaaagaatt ttttagtatt gtatagtttt 6360
tttaaagtat aaagtttggg agaattaata tatgtaggat aattgaatgt aagagatttg 6420
tggaatgtag ttttaatttg ataattgtgt gttaattggg agtaatagga taggttgtta 6480
tggttggtta tatttattta tgttagatgt ataatgtttg gagttttttt tattgatgag 6540
aagtaattta tgattttg 6558

<210> 345
<211> 6432
<212> DNA
<213> Artificial Sequence

<220>
<223> chemically treated genomic DNA (Homo sapiens)

<400> 345

tattatgttt agttaatttt tgtattttta gtagagatag ggttttataa tgttggttag 60
gttggttttg aaattttgat tttatgattt gtttgttttg gttttttaaa gtgttggaat 120
tataggtata agttagtatg tttggtttat attttttaat tgtatgtata atttaatttg 180
tagatgattt taaaagttta aattggagtt ttattgtttt ttttattttt agtttttata 240
ataaatttta tgatagtttt tgtttataag aatttttttg tagttttatt atttgtaaat 300
tatttaattt tgtttttttg tgatttttta ttttttatat tttttatatt ttttagttat 360
tgttttaaaa attttggata ataaattaat ttaaattttt ttttaaatgg tatttattta 420
tttttattta gatttatatg gattggttag aggtaggttt gagattggtt gatggaggtt 480
tggtttagat ttaattagga ggttggttta tatttgtttt atatgttttt tttttttttt 540
gtatttgttg gtttttaagt gtttttaatt gaaaataatt agtatattaa agtagtatat 600
tttggagtgg tattttttga aattttttat tggttaggtg gtgaagatta attatttgat 660
taatttgttt tagagtgtag aataagtttg gaattagata ggattaagtt taaatttggg 720
ttttgttttt tattagttat gtgatttttg ttaaatattt aattttttta atttttaaat 780
tttttatttg taaagggaag attagaatag tattgtaagg atatgtttta aaatttaata 840
aggattattt ttggataata gggttaagaa tttttttaaa gtttgttttt aaaaaaaaaa 900
tttaaatttt ttatttataa ttaatatgta ttatttttgt aatttttaaa aagttatttt 960
ttttaaaata tattattgat gttttttgta aatatgtggt ataattgtag gttttattaa 1020
tatttgtgaa gaattgtaaa taagagaatg gattaggaaa attgatattt ttattttttt 1080
ttttattgaa gataagaaat atatattgaa tattgattat ttgtagagta tggagtgtgg 1140
ttttatattg aaaaaggaat attttgtgtt tttaagggtt ttataattat tagggtagaa 1200
ttttaaaaag taaataaata ataataatgt attttatgtt agaatggggt aaatattttt 1260

ttttaaaaaa aggttaaata aatgaaatgt tttttgggtt atttttggga aaggagtgat 1320
tatagtaggt gaagtattat aaaatatttt ttggaggagg tagtattatg gatgatttat 1380
aaagggtgga tgatttttat aaattaatta tagttttagt gtttattggt ttgttttata 1440
ataaaaggaa aattggttaa gagataagag ttgagaatga ggttggttaa tttataagga 1500
gaggaatttt tttgttaatt gttttagttg atagtaattt taatagttgt atgttataaa 1560
gtttagggga gtaggaagtt ttttttttat tataattgag aaatttaatg tttaaataaa 1620
ttataatttg atttagttta attgtgtgtg tttgtgtgtg tgtgtagagt ttaaaaagtt 1680
ggtaaaatag attgttagtt tttaaaattt ttgtttaagt tgatatttat tgttttttaa 1740
tttattaagg gtttaaatgt tatttttttt gttttttaaa ttttgagttg ggttattatt 1800
tttttgtttt tatttgtaaa tggtttagtt ttttatatgt atatattaat tgtttatgtg 1860
aatatataaa atattatatt aagttaaata tataattatg tgtgaaaatg atgttttgat 1920
taagggtatt agatattttt tttaataaga attttaaaag gaagggtatt atgataattt 1980
tttttttttt tttttttatt tattttttgt ttttgagata gggttttatt ttgtttttta 2040
ggttgaagtg tagtggtata attatggttt attgtagtgt ttgttttttg ggtttaagtg 2100
atttttttat tttagttttt tgagtagttg gtattataga tatatgttaa tatgtttggt 2160
tttttttttt tttttttttt ttttagtaaa gatgaaattt tattatgttg tttaggttgg 2220
ttttgaattt ttgggtttaa gtaatttttt tatttagatt ttttaaagtg ttgggattat 2280
aggtgtggat tattgtattt agtttgatga atttttaaaa ttattaaatt gtaatatgta 2340
tgagtttata tatttattat ttttattatg atgattttta aaaggttagt ttgtatttgg 2400
taatgttgaa ttttgatttt ttttaatttt ataggagatt ggagattaaa ttagaaatgt 2460
taagggtaaa taaaattaaa attaatatta atgatgtatt atggtaaaga ttattagtta 2520
aatattttat tatagattta aatatttagt ttttaataat gtaaaatatt tttgtgataa 2580
ttaaggaaaa taaagttttt ggaatttatt ggatatttat ttgttatatt gtgtttttag 2640
gagaaagaaa gtatatgttt gttatttgga ggaatttggg taaatgtagg aaaaaaaaaa 2700
gaagaagaaa gaaatttata tattttttta tttttaagaa aaggaagtag tggtgatatt 2760
atttttggtt aaaatgtagt ttgtttatat tttaaatatt tggtaataat gtttttttta 2820
gagttatgaa gtaattagaa taattaaata taattttttt ttaaattttt attgtttgat 2880
ttatttattt atttaataaa tatttattaa atattgatta tgtgtttgat gtttagggat 2940
ataatagtaa gtggagggaa agatatataa tatttgtttt taagaaattt ggagttgagt 3000
ggaggataga aatataaatt aaagaatgat ataaataatt ataaagttat agttgttaaa 3060
agaaaagtat atggtgttaa gagaatgtgt aatataagat ttatttatgg aggtgaggga 3120
aagtttgttt attaaagaag ttatgattta atttatgaag attaggagtt ggttgggtga 3180
agaaaaaaag gttagaggaa ggaagtttat attggggaag gttttaagta taaagggtag 3240
gaggattata gaggtatatt tatgaaattt ggagaaggtt tttagtaagt aaggagaagt 3300
taaatgaaag tttatgggag agttggaggt ttgaagatat gtttaaggat ttggttttta 3360
tttttttttt attttaagag tagtgggaag ttattaaatg attttaatta gagggttggt 3420
ataattagtt ttgtattttg aaaagttgaa tttagttttt gtttgagaaa ttgagtgaaa 3480
gagtttagaa tggttgtggt tgagggtgat ttgtgggaga tttttatata agttatggta 3540
gtggtatggg ttggtggtag aagagggaat agggagaaga tttggaattt aatttttttt 3600
tattgataaa gttattttag ttttggtaag gtaattaatt ggtgggaaag aagatgttta 3660
gtttttttga ttttattgta ttttttgtat ttttaatatg agtattggga agtggtaaaa 3720
tatttagagg tagtttgggt gttaggtgga gtatgagtta aaattttagg atgaagtaaa 3780
tgaatattta gaatgatagg aaagatttgg gagttgggtt tgggggaggg ttatttattt 3840
ttattttttg gagattttgg tataaatttt tgtttttgta attttttttt taggtaaagg 3900
aatttattaa atgaattgtt agaagattta ttgattagag ggttgtatag aattatattt 3960
ttgagagtgg gaagtaggtt gattatatag tttattattt aattaggata tatttgaaag 4020
agaaaggggg ttttattaat atttaaatta taaaatatgt atattaggaa tgttttgggt 4080
aaatttggtt gttttagtaa gaaaggaaat ttgaaagttt atattgtttt gtttttatgt 4140
tattttgttt gtatatgaga gggtaagtat tttttttttt tatttgtatt aagggaataa 4200
aagtataagt atttaggtga tttttaattt atttttaatt ttatagtttt tgttatattt 4260
tatatatttt gaaaattata ttttttatta ttattatttt gtgataggtg attatttata 4320
attatttatt gatttagttt tgggaagagg tggtgtaaaa tgggatgttt tatttaggtg 4380
tttattagaa atgtagaatt tttgtttgtt ttttagattt attgaattag aatttgtatt 4440
tttaaataag atttttaggt gattaatatg tatattaaaa tttgagaaaa atttttagat 4500
tttgatttaa agaaaaatat tttataattt gatagtgtat gtatatatat atatgtatat 4560
agatataatt gaagtataaa tttaatgaag tagaatttat tgttattatt ttatttggga 4620
aagaaatgtg tttgtgattt aatagattgg agtatttatt tttggatttt aatttgtaat 4680
ttgaaaatgt atttttaaag tatttaggag taatttgaag aaagttgagg ggaggtggta 4740
gatgttttga tttattaggg aaaatgtgga tgttttttgt tgttattttg tgaattgtgt 4800
gtatttagtt atttttgagt aaatatttgg agtgaggaat ttttgagtgg tgtgggaggg 4860
tggtgagggg tagttgaaag ttggttaaag tttttggagg ggttggttta ggaaatatga 4920
ttggtagtta tgagagagtt aggggttgga tgttgaggag agggagaagg tttttgggtg 4980
gagagaggtt ttgtttagtt gttggtgagg agttttttgt tttttttgta gtgttgagtt 5040

gaagttgagt gagttatttg tgtgtatgga gtgatgatat ttttgtgtgt gtatttgttt 5100
gggataggag ttggattttt gtgtagtttt ttttggttgt tgggggtttt tttgtgtttt 5160
gttggttttt aggttttttt ttggttggtg agtgggtgtt atatttggtt tgtatatttg 5220
tgttgttggt ttggtgtggg gtttggagag ggtgtggtgt ggaggtgtag ttaggggttt 5280
gggaaggtgt tgtttgttgt gttgggggtt tggtttatga tgagtagtgg ggtttgttat 5340
gggttggggg ttgtttaggg gtttgtggtt gttgtatatt gttttgtgga tgtgtattgt 5400
tagtatgatt ttattgtatg tttagaagtt gggtgagtgg tttttagttt gggtttggtg 5460
gggtgttggg ggtttttttg gggtttttgt ttttttgttg tgtttgatag ttgggtttgg 5520
taatttggtt tttgggtgga aatgaggaaa gttttttttg tgatatttat gtagtttgat 5580
ttttgtagtt gtagggattg tgagtttttt ttgaaaaaga gaaggaaagt ttagttgtaa 5640
ggggtgtggg gtatgtttgg ttttttttgt gtgagtagga aaggtgttgt gttggttgtg 5700
tttgaggtga gttttatttt ttggaaaggg aagtttgaga agttggttat ttgaaggtgg 5760
ttggggagta gtggtttgga gtggtagttt gagttgttaa agtagttagt gtatttgggt 5820
tattttgttt atggtgatgt tgtgtgttta tttagttttt tttgagttat ttgtttaaaa 5880
ggttagtttt ttagttttta gtttttggag atggttatgt ttttttttag gttggttttt 5940
tggtttggag tttttggaaa taagttttaa gaaaataatt gatttttaaa agaaagttag 6000
ttggttttat tgatgttttg gtatggatgg atagggagtg gagatgttta ggtgaaattg 6060
agaatttttt attgaatgtt tattgggtgt tagaggttgt agattttgtt tggaataaga 6120
tagttttgtt tttagggagt tgatggtttt atgtaattat tgtttgttgg aaattgaagg 6180
gttaaaattt taattaggtt atttatttga ttgttgtttt tgaaggtttt taaggaaaaa 6240
ataaaaataa aaaaatatat atatatatta tatatatata aatatatata taattatata 6300
tataaatata tataatatta atatatatat gtatgtagga atgggggttt ttaattattt 6360
gttatggaaa gtgtaaaatt tttgagattt taaaagttag attttttttt tttggagatg 6420
gagttttgtt tt 6432

<210> 346
<211> 6432
<212> DNA
<213> Artificial Sequence

<220>
<223> chemically treated genomic DNA (Homo sapiens)

<400> 346

agagtgagat tttgttttta aaaaaaaaaa atttaatttt tgaagtttta gaggttttat 60
attttttata ataaatagtt aagaattttt atttttgtat gtatatatat attaatatta 120
tatatattta tatatataat tatatatata tttatatata tataatatat atatatattt 180
ttttattttt attttttttt taaaaatttt tagagatagt gattaggtgg gtagtttagt 240
taggatttta attttttggt ttttaataaa tgataattgt atagaattat taattttttg 300
aagataggat tattttgttt taggtaaaat ttatagtttt tagtatttag taggtattta 360
gtgagggatt tttggtttta tttgagtatt tttatttttt atttatttat gttaaggtgt 420
tagtggagtt agttaatttt tttttggaaa ttgattattt ttttgaaatt tgtttttaaa 480
aattttgggt taagaagttg gtttgaggga aagtgtggtt gtttttagga gttaaggatt 540
gaggagttgg ttttttgaat gggtggttta aaaagagttg ggtgggtatg tggtattgtt 600
atgggtggag tggtttaggt gtgttggttg ttttggtagt ttaggttgtt gttttgggtt 660
gttgtttttt ggttgttttt agataattaa ttttttaaat tttttttttt gggggtgggg 720
gtttgttttg aatgtggtta atataatgtt tttttgtttt gtataaaggg gattaaatgt 780
gttttgtgtt ttttgtaatt gaattttttt tttttttttt aagaaaaatt tataattttt 840
gtagttatgg gagttgggtt gtgtgagtgt tgtgggggaa attttttttg tttttgtttg 900
ggggttgggt tgttgggttt gattgttaag tgtagtggag aggtggggat tttaggaaga 960
tttttggtgt tttgttgagt ttgggttggg gattatttat ttgatttttg aatgtgtggt 1020
gggattgtgt tggtgatatg tgtttatagg atgatgtgta gtggttatag gtttttgagt 1080
agtttttgat ttatggtaga ttttgttgtt tgttatagat tgagttttta gtgtagtgga 1140
tggtgttttt ttggattttt ggttgtgttt ttgtgttgtg ttttttttgg attttgtgtt 1200
gggttggtag tgtagatgtg tgggttagat gtggtgtttg tttgttagtt aggagggggt 1260
ttggaggttg gtgaggtgtg gggaggtttt tggtggttga gggaagttgt ataggagttt 1320
ggtttttgtt ttgagtgggt gtatgtgtgg gggtgttgtt gttttgtgtg tgtgagtgat 1380
ttatttaatt ttaatttagt gttgtggggg aaataggaaa ttttttgtta atagttgggt 1440
aggatttttt tttgtttgag agtttttttt ttttttttga tgtttagttt ttagtttttt 1500
tgtagttgtt aattatgttt tttagattag tttttttgag agttttggtt gatttttagt 1560
tgttttttat tgttttttta tattatttag gagttttttg ttttaagtat ttatttaaga 1620
atgattaagt gtatatagtt tataaagtaa taatagaaaa tgtttatgtt ttttttagta 1680

gattagaata tttgttgttt ttttttagtt ttttttagat tgtttttagg tgttttagag 1740
atgtgttttt aaattgtaag ttgagattta ggagtgaata ttttaattta ttgagttgtg 1800
agtatatttt ttttttaaat aaaatagtaa tggtaaattt tattttatta aatttgtgtt 1860
ttagttgtgt ttatatgtat gtatgtatgt gtatatattg ttaagttgta aaatgttttt 1920
ttttaggttg aagtttagag gtttttttta agttttaatg tatatattga ttatttggaa 1980
attttattta aaaatgtaga ttttaattta gtaggtttag gaggtaggta gagattttgt 2040
atttttaatg agtatttgga tagagtgttt tattttgtat tgtttttttt tgggattgag 2100
ttagtgagta attgtaaatg attatttatt atgaagtgat agtggtggga aatgtaattt 2160
ttagaatgta tagagtatag tagaaattgt aaaattaaaa gtgggttggg agttatttga 2220
atgtttgtgt ttttattttt ttaatgtagg tgaagaaaga gaatatttat ttttttatgt 2280
gtaaatgggg taatatggga gtagaatagt ataaattttt aaattttttt ttttgttagg 2340
gtaattagat ttgtttaaga tatttttggt gtatatgttt tgtagtttaa atattaatag 2400
aatttttttt tttttttaga tatgttttga ttggataata aattatgtga ttaatttatt 2460
ttttattttt aaagatatga ttttgtatag ttttttggtt agtagatttt ttagtaattt 2520
atttaatgaa tttttttatt tgagaggaag attgtagagg taagggtttg tgttagggtt 2580
tttagggaat aaaggtaaat agtttttttt taaatttaat ttttaaattt tttttgttat 2640
tttaagtgtt tatttgtttt attttggaat tttaatttat gttttattta gtatttaagt 2700
tgtttttgga tgttttgtta ttttttagta tttatgttga agatgtagaa agtgtagtga 2760
aattaggagg gttaggtatt ttttttttta ttaattaatt gttttgttaa agttggagtg 2820
attttgttaa tggaggaaga ttgagtttta aatttttttt ttattttttt ttttgttatt 2880
agtttatgtt attgttatgg tttgtgtagg agttttttat gagttatttt tagttatggt 2940
tgttttgggt ttttttattt agttttttaa atgagagttg aatttagttt tttaaaatat 3000
aaaattagtt atattaattt tttgattaaa attatttaat ggttttttat tgtttttgag 3060
ataaagagaa gataaaaatt agatttttga atgtgttttt aagtttttaa tttttttgta 3120
aatttttatt tggttttttt ttgtttattg aaagtttttt ttaaattttg tgaatatgtt 3180
tttgtaattt ttttattttt tatgtttaga gtttttttta gtgtggattt tttttttttg 3240
attttttttt ttttatttag ttaatttttg gtttttgtgg attgaattat aatttttttg 3300
atgggtaagt ttttttttat ttttatgagt agattttgta ttatatgttt ttttggtatt 3360
atatgttttt tttttaatag ttgtaatttt ataattattt gtgttatttt ttaatttata 3420
tttttatttt ttatttaatt ttaaattttt tgagggtagg tattatgtat tttttttttt 3480
atttattatt gtgtttttaa gtattaaata tataattgat gtttaataaa tatttgttgg 3540
atgaatgaat gaattaggta ataaagattt agaagaaaat tgtatttgat tgttttagtt 3600
gttttataat tttggagaaa atattgttat tgaatatttg agatataaat agattatatt 3660
ttagttgaga atgatgttat tattattttt tttttttaag aatggagaag tatgtgggtt 3720
tttttttttt tttttttttt tttttatatt tgtttaagtt tttttagata gtaaatatgt 3780
attttttttt ttttgaaaat ataatgtggt aaataaatgt ttaatagatt ttaaaagttt 3840
tgtttttttt aattattata aagatgtttt atattgttgg aggttagata tttaaattta 3900
tggtggaatg tttaattggt ggtttttgtt atgatatatt attaatgttg attttaattt 3960
tgtttgtttt taatattttt gatttaattt ttagtttttt gtgaaattga gaagaattag 4020
aatttagtat tgttaagtgt aagttagttt tttgaaaatt attataataa aaataataga 4080
tgtgtaaatt tatgtatatt gtaatttgat aattttaaaa atttattagg ttgagtgtag 4140
tggtttatat ttgtaatttt agtattttgg gaggtttaga tgggaggatt atttgagttt 4200
aggagtttga gattagttta agtaatatag tgagatttta tttttattaa aaaaaaaaaa 4260
aaaaaaaaaa aagttaggta tgttggtatg tatttatagt gttaattatt taggaggttg 4320
aggtgggaga attatttgag tttaggagat gaatgttgta gtgagttatg attgtgttat 4380
tgtattttag tttagaagat agaatgagat tttgttttaa aaataaaaaa taaataaaag 4440
aaaaagaaaa aaaaattgtt atgatgtttt tttttttgaa atttttgttg aaagggatgt 4500
ttgatatttt taattaagat attattttta tatatagttg tatatttggt ttagtataat 4560
attttgtgta tttatataag tagttaatat atatatgtaa aaaattgggt tatttgtaaa 4620
tgggaataaa aaaataatag tttgatttag ggtttaaaaa ataaggaagg taatgtttaa 4680
gtttttaatg agttgaaaaa taataaatat taatttgaat agaaatttta aaagttgata 4740
atttatttta ttagtttttt aaattttgta tgtatatata aatatatata attgaattga 4800
attaaattgt ggtttattta aatgttgggt tttttagttg tgatgagaga agaatttttt 4860
atttttttga gttttgtgat atgtagttgt tgaggttatt gttagttaga gtagttaata 4920
gggagatttt tttttttata aattaattag ttttattttt agtttttatt ttttgattaa 4980
tttttttttt attgtgaagt aaattaataa gtattgagat tgtagttaat ttgtgagggt 5040
tatttatttt ttgtgggtta tttatgatgt tatttttttt aggaaatatt ttgtgatgtt 5100
ttatttattg tgattatttt ttttttaggg ataatttagg gaatattttg tttatttagt 5160
ttttttttaa aaaaaaatgt ttgttttatt ttaatatgga gtatattatt gttatttatt 5220
tgttttttag aattttattt taatgattgt aaagttttta aaagtataaa atattttttt 5280
tttaatgtag aattatattt tgtgttttat agataattag tatttagtgt gtgttttttg 5340
tttttaatag agagaaaaat aagaatatta gtttttttaa tttatttttt tgtttgtagt 5400
tttttataga tattaataga atttgtaatt atgttatatg tttatagaaa gtattaatag 5460

tatgttttga aaaaaataat tttttgaaaa ttataagggt aatatgtgtt aattataggt 5520
aggaaattta gatttttttt ttaaaggtag attttaaaag gatttttaat tttattattt 5580
aaagataatt tttgttaaat tttggagtat gtttttgtag tattatttta attttttttt 5640
tataaatgaa gaatttgagg attaaagaga ttaagtattt gataaaggtt atatagttag 5700
taaaagatag agtttagatt tgaatttagt tttgtttagt tttagatttg ttttatattt 5760
tagaataaat tgattaggtg gttgattttt gttatttgat tagtgaagga ttttaggaaa 5820
tgttatttta aaatatgttg ttttggtata ttgattattt ttagttgaag gtatttaaag 5880
attagtaaat gtagggagga ggagagatat gtggaataga tgtggattaa ttttttgatt 5940
gaatttaagt tagattttta ttagttaatt ttaagtttat ttttagttaa tttatatgag 6000
tttgagtaag aataagtaaa tgttgtttaa ggaaaaattt gggttggttt gttatttaga 6060
atttttgagg taatagttaa gagatataag ggatgtgaag ggtgaggaat tatggaaaag 6120
taaggttaaa taatttgtag gtggtaaaat tataaagggg tttttataga taaaaattat 6180
tatgaggttt gttatggggg ttgaagatga ggaagatagt aggattttaa tttagatttt 6240
taggattatt tataaattaa attgtatata tagttaaaga atatgggttg ggtatgttgg 6300
tttatgtttg taattttagt attttgggag gttgaggtag gtagattatg aggttaggat 6360
tttgagatta gtttgattaa tattgtgaaa ttttgttttt attaaaaata tgaaaattag 6420
ttgagtgtgg tg 6432

<210> 347
<211> 7309
<212> DNA
<213> Artificial Sequence

<220>
<223> chemically treated genomic DNA (Homo sapiens)

<400> 347

atgtgattaa tggtgttaat tgttttttgg attgttttta tagtatagga taagattata 60
ttagttttag taggttttat tgaagaaagt ttatgtgttt tgtagaaata agatttttag 120
tttagtttaa taggagatta attatatgtt tattttattt ttttttaagg tatgatagta 180
tttggttgag atgtttgtgt gtattttttg tgtgaagttg agtatgtaat ttttaaagaa 240
attataaagg agttatttgt ttttaaagga aaaatatatg aattttatgg gtataaggaa 300
gatattaaaa tagggttggg gagaagtttt ttttttttta ttttatagta ttttttatag 360
aaataattga ttgtttgaat atatggttat tgaaatatat atatatatat atatatgtat 420
atgaaaataa gagttggaat atttgatttt gttttttatt ttttattatt ttagataata 480
taggatataa tatggtttta ttgtatatat gtgttttttg gtttaaaaat attgagagta 540
gatttttgtg gaaagaaata tgaaaatttg aggatttatg tgaatgtagt tagaggtgaa 600
tgtagttaga ggggttttta atttgttggt agagttgggg gagagttggg gtaattttat 660
tgttatgaaa ttggttttag atggaggtaa ggaaaggtgt ttattttttg ttggtgttag 720
atatagagag attttgtagt atttgtattt ttaggtttag aattgtttag tgttgggtag 780
tagttttatt gagggtttgg ttagagttta tggggatggg tggattaggt tgtttgttgt 840
ttggaaaagg ttattttgaa tataatgatt tttatatgga tggtggggtt tgggggaagg 900
tattgtggtt agggtttgga ggggaagagg gaggaaagta aagaggagtt tttttatgag 960
gtaatagaaa tgatagtatt ttttttaggt tgaattttta aaatttagtt ttgttttgga 1020
agattgggat tgtggtattt tttgtagtgt ttttagattg ttttaaggtt tttaaattaa 1080
tgatttataa ttatgttgtt tttttagaga gtattttttt ttttggttga tatgtggtta 1140
gtttaggggt ggaggagttt tggtagttat gaatttagat gggatgttga ttgagagttt 1200
tgtggttatg gtagatggga ggggtgagtt ttatttattt aaaggtattt gtattttatt 1260
gatgagtgtg gaattgtgtt tttttgtttt tgtagtgaga tgattgatta ggattgtagg 1320
ttgagtaaat gggtgttttt tggtttatag tgttttttag tattagttag tgggatagag 1380
ttttgtgaat tttttaagga gttattattt gtttgtttga atgttggaaa ggggttggtg 1440
gtgttgatta ttggatttat ttaattaata tttattaaat ttgttgttat taatatttaa 1500
tttttgtgtt ttaggggttt agattttagt aagggaaata gatgggtaaa taattatatt 1560
agagttagtt aagtattgtt aaagaagttg atatagggtg ttgtggggat atatgggagg 1620
ggaattagag gaggtttttt ggaggaggtg atatttaagt tgaggtttga aggatgtgga 1680
agagtttagt aatttttgaa gattaaaaaa atggtgataa aaaagatatt tttttaattt 1740
tttagtgtta tgatttaaga gataaataag taggtgaatg tataattata ttgggtttta 1800
ttataagtta gagtttgttt tttttaggat tttggtatta taattttttt tgaaattttg 1860
tttttggtat tgatttttgg gggaggtagt aatataggaa tttagtgttt gttgtgaaat 1920
tgatggatgg tttttagttt tgggtatgtt tttatgtgtt aaatttgttt aaatggtagg 1980
atttattttt tttttagaag tatttatttg ttttatagga ttttttatgg aattttggag 2040
tttttgaggt gagagggatt ttggatatta ttgagtttta ttttttattt aataaatata 2100

gaagtggatg tttggatagg taaagtgatt tgattaaggt aggtgtatag ttattttgta 2160
atattgggaa taaattttag gttttttgat tttttgtttt tattttattt tttttttatt 2220
ttttagaaat aaagttttta tgtgtttttt tttatagtga tatgtttgga atgtattagt 2280
tagtaattta ggaagggaaa aaaataaata tataagagat aaatttgtta ggaggataaa 2340
tttgtattgt ttttgattgg tttagagggt gattattatt atggtagaga attatttaat 2400
tagtgtaagt aaaatttttt tgtgggttgg gtattgtata aagatttaaa tgaatttgtt 2460
tatagatttg aaaagtagat atgagatttg tgaatggttg gggtttttaa gtttatagta 2520
taagtatggg ttatatttta tagtttggag gattgagttt tgaaaatggg taagtttttt 2580
tatttttttg aattttattt tttttatatt taaaataagg atgagtagtt tttgaggttt 2640
tttttatgat tttttttttt atagatttta gtattttata atttgatata aagagggtgg 2700
atatgaattt atttttttta gaaaagtttt aggaaagaga atattaggtt attttagtag 2760
gtgtgtagat aggttagata gattttgaaa tttatttagt tttttttaga tgtataattt 2820
tattattgtt tttagttgtt aagagaaagt aggagagttt gtatttttat tttttttttt 2880
tttttttttt tttttggaga tggagtttta ttttattatt taggttagag tgtagtggta 2940
tgattttagt ttattgtaag ttttgttttt taggtttatg ttattttttt gttttagttt 3000
tttaagtaat tgggattata ggtgtttatt attatatttg gttaattttt tgtgttgtta 3060
gtatagatgg ggttttatta tgttagttag gatggttttg attttttgat tttgtgattt 3120
gtttattttg gttttttaaa gtgttgggat tataggtgtg agttattgta tttagtttgt 3180
atttttattt ttattgttag ttttaggttt attttattta gtttattaag tgatgttgaa 3240
taattaattt ttatatatta ttaggtttat ggatattatg atatttagat tgatgggtgt 3300
ttgttgaagg gggtgatttt agtaggagga tttttttatg taaggattta tggagtttgt 3360
tgtttttttt ttttagggtg agaattaaat tgtttttata tggtgggtag aggggaattg 3420
atttaggttt ggaataagag agaatatttt aattgaaaag tttttggaat ttgttgaatt 3480
ttaagatatt gtgtggatta gtttaggata gggagtgaga agaaattaat taaaaggtaa 3540
ttttgttatt ttttagttgg aaaaaagatt agattatatt tgtgttttta taattaagta 3600
gttgttggaa aaaaatgttt tagatgtttt ttatgagaaa attgttgttt gaagtttagt 3660
agaagttatt tatttgatat tttatattta ggtaaggttt tttgttggag aaaatattgg 3720
tattttggat aaaattgaaa tgtgaaaaga aagggaagag agggttttta ttatgtaaga 3780
tgtttattta aagtggattt ggtttggaaa gttttttaaa attttttata tgattgtgga 3840
ataagttatg tggggtgtgg ggataagtga attttttaaa ttttattatg tatgttttta 3900
tttaatttgg atttttagag tggtttttag ggtattttgt ttaggattta gttagttgtt 3960
ggttatattt atgtttttta gttttttgag attttatttg gttttgagag ggttaaaaag 4020
tagtgtggtt aaatatttta ggttttaaag tatttttatt gtggttgggg aagtaataga 4080
attatatttt ataaaataat gaaaatagtg ttagaaaaat attgagagat agaaatattt 4140
ttatgagtta ggttatagtt agagtgaagg tagggaaggt ttttaaagtt gggtggaggg 4200
gataagttaa aaagatgtgg aaattggttt ttttttttta tggttaaagt gtttaaaggg 4260
gaaaaaggag ttttaaaaat gtttttggaa atattatttt ttatgaattt tttggttttt 4320
gttgttttaa tgttatttgt ttgagatgta aatagaggag ttttgagaaa gaagttgaat 4380
ttgtattttt ttttgttttt atttgtttta aatttgtggt atttttaata ggatgaagtg 4440
gaagagaaag ggaaagagat aaaagtgtag aaagatggaa gattttagtt gtaaatggtt 4500
atttgtagtt agatggaata gttgttgatg tttagggaaa tgtatgtttt tttttagatg 4560
ggaaggagta gtggaaaggg gtgatgagtt tttggttggt tattaattat tttatttttt 4620
tgtgttggtt ttttatttgg aaagtgggag tgatatttgt gtttgttttt tttatttata 4680
aagattattg tgagagttat aatatggtga gatatagaat tttgttttta aaaatataaa 4740
gtagaattaa gatgttaata ataaggatag taattgtgtt agttatttgt aattatttat 4800
tatagttagt tgtttaggat tttggattgt ttttttggtt ttattatagt tttggattag 4860
tttattttta aattttttgt tgaagggtgg agttttgtta gttatgggta gggaattatt 4920
tttttttgtt tttttatttt ttgtttttta aatatgttta gggtttttgt atttgttgtt 4980
ttttttgttt ggtatttttt ttttgtggtt tgttttagag ttgatttttg tttttgttta 5040
ttttttagtg aggatggtat tttagggagt ttttttttta ttattgtaga gagagtaggt 5100
tttttttagt tatgtttaat ttagaatttt gttttgtttt ttttatagtt ttagtattat 5160
agaaaattat tttgtgtatt tatggatgtt tatgggggta agggttttgt gttgtttaat 5220
ttagtatttt gaattgtgtt tgttgaatga atatagaatt ttgtttgttt tgggagagta 5280
tagaaaatag ttttttatta tatattatag ttagttgtaa atagtagatg gttttttata 5340
ttttagagag taagaattag agagagagag aaagagagag agtttgggtt tttttttttt 5400
gtgtttgttt tttttagaga aattggaggg gtagtagtta gtattttttt gttggtttta 5460
ttaagtatag ttaaggtttt taggatatgg ttatttttta tttgtggaag tggttttgtt 5520
ggggtgggtg ggtgttagtt ggttttggtt tgggttagag atatttagtg gtttaggtgg 5580
gtgtggggtt agggtgtaga tgagaagggg tatgagggtt ttgttttgag gatttagtgg 5640
taagtattgg ttttgggtgt gttttagttt atttatttgt gtgtttatgg tggtattatt 5700
ttttataagg atttgaatga tttgggggtg gttttgtttt gttatttttt gtttttggtt 5760
ttgttttttt tttggagggt tgatgaggta atgtggtttt gttattggtt tgagggggtg 5820
ggttttaata gtttgaggtg gggtttttgg gggtttagtg ttatattatt tggttgttta 5880

ggtagtggtg tagagtgggt agtaggtagg tggtgggtgt ttagatggtt tttttttttt 5940
tttttgtttt tttagttttt gtttttttgt tgggaggttg tttgttgagt tttgtgttag 6000
tgttgaggta gttttgttgt gttttatttt gttttgttgg gtatttggag ggtagtgtgt 6060
tggaggttaa ggttgttttg tatggtttgg tgggtgagtg agtttgggtt gtagtagttt 6120
tgttggtggt gtgtatggta attttggaga ggtgagtagt agttttggta gtggtggtag 6180
tagtggtaat gattttttgg tttgggttta ttgtgttttt gggtagttgg agtttggggg 6240
attggggtgt tgaggtgtgt atatgtttgt ttagttattt ttaggatgtt ttttgtaatt 6300
ttgatattgg taagtgtttt tggtgttttg tttgagtttt atgttgtagt taggattgta 6360
gtgttgttta gggaggtagg gtgagtttta tttttttttt ttgttttagg agaggggtag 6420
atggggttgg ggtggagtgg agaaatttga tgtttttggg tgggggtgtt ggtatagttg 6480
agaggggaag atgttttgta gaggaaattt atagtggttg agggagtttt tggttgtttt 6540
tgttttttta atttaggttg tgaggttttt attggttttt ttgatatttg gaaaatgttt 6600
ttatttatta ttaatggagg aagggttaga agagaagggt ggggaaaggg tttttaaaat 6660
ttggaatgtt aattaaagtg ttgggaaatt gaatagttaa aaaaaaaaaa aaaagttgtt 6720
tgttatgtgt atggaaagtt gtaggagaaa tttggagtat tttgagtttg tattattttt 6780
tgtgtttatg aaatttattt tttttattga gatttaattt agttgtaggt tttttaggta 6840
agtgttaatt gagtttttgg gattgttaag tttgggtgga tttaaggggg aaaatgagga 6900
gaaagttgtg gtattgattt atgttttgga ggtattgtgt ttgagagtgt tgatagttag 6960
tagaaggtgt gaggggttat ttattatata gttgtgaaaa tttataggaa ggttattttt 7020
tttttttttg aatgtgtgtg gggaggggga gaatgggtag tggaaaatag atttgttatt 7080
aaaagaataa atttgttaag ggttatatta tggtgatagt tttagattgg atttagtgtt 7140
ttgagtagag agattttgta aagagatttt ttatttttta gtgtgtatgg agggttttga 7200
gagtattttg ggaggtttgt ttgggagtgt ttgggatatt tgtgtttgga tggttaaagg 7260
tggagtggta tttgggaaat attttttttt ttttttttag tttataaag 7309

<210> 348
<211> 7309
<212> DNA
<213> Artificial Sequence

<220>
<223> chemically treated genomic DNA (Homo sapiens)

<400> 348

ttttatgggt tggaaaggag gagagagatg ttttttagat gttattttat ttttagttat 60
ttaaatataa gtgttttaaa tatttttaga taagtttttt aggatgtttt tggggttttt 120
tatatatatt aaggagtagg aggttttttt atagggtttt tttatttaag atattaagtt 180
tagtttggaa ttgttattgt gatgtgattt ttggtaggtt tgtttttttg gtgataaatt 240
tgttttttat tatttatttt tttttttttt atatatattt aagggggaaa aaaatagttt 300
ttttgtgaat ttttatagtt atgtggtgag tgattttttg tattttttgt tggttattaa 360
tatttttaaa tatagtgttt ttagggtgtg ggttaatgtt atagtttttt ttttattttt 420
ttttttaaat ttatttaaat ttggtagttt tagaagtttg gttagtgttt gtttgggaag 480
tttgtagttg ggttgggttt tagtgaaaag aatgaatttt ataggtgtag gagatgatgt 540
agatttggag tattttaagt tttttttgta attttttgtg tatatggtgg gtaatttttt 600
tttttttttt taattattta attttttagt attttagtta gtattttaag ttttgggaat 660
ttttttttta tttttttttt ttagtttttt ttttgttagt agtgaatggg gatatttttt 720
agatgttaaa aggattggta ggaattttat gatttaagtt aggaaagtaa aggtggttag 780
gggttttttt agttattgtg agtttttttt gtagggtatt tttttttttt agttatgtta 840
gtgtttttgt ttaaggatat tgagtttttt tattttgttt taattttgtt tgtttttttt 900
ttggggtaga ggaaaggagt ggggtttgtt ttgttttttt aagtagtgtt gtagttttgg 960
ttgtagtgtg gggtttgggt ggggtattag gagtgtttat tgatgttgga gttgtagaag 1020
gtgttttggg ggtggttggg tgagtatgtg tatgttttgg tgttttagtt ttttaggttt 1080
tagttgttta ggagtatgat gagtttgagt taaggggtta ttgttgttgt tgttgttgtt 1140
gttggggttg ttgtttgttt ttttaaagtt gttgtgtgtg ttgttggtgg ggttgttgta 1200
gtttgagttt gtttgtttgt tgggttgtgt ggggtaattt tggtttttgg tgtgttgttt 1260
tttgagtgtt tggtgggatg ggatggggtg tagtgaggtt gttttggtgt tggtgtagga 1320
tttggtgggt ggttttttgg tgaaggagta ggagttggag gagtaagagg aggaggagaa 1380
gttgtttgag tgtttgttgt ttgtttgttg tttgttttgt gttgttgttt gggtggttga 1440
gtgatatagt gttgggtttt tggggatttt gttttgggtt gttggggttt gtttttttag 1500
attaatggta gagttgtatt attttattgg ttttttaaaa agggggtggg gttgggggta 1560
aggggtaatg gggtggggtt gtttttggat tgtttagatt tttataggga ataatgttgt 1620
tgtgggtatg tgagtgggtg ggttggggtg tgtttgggat tggtgtttgt tgttgggttt 1680

ttggagtgga gtttttgtgt ttttttttgt ttgtgttttg gttttatgtt tatttgggtt 1740
attgggtgtt tttgatttaa attagaatta attaatattt atttatttta gtaggattgt 1800
ttttataggt gaggggtggt tatgttttag agattttgat tgtgtttggt ggaattagtg 1860
ggggaatgtt aattgttatt tttttagttt ttttggagag agtaggtata gaggagaaag 1920
atttaaattt tttttttttt tttttttttt tggtttttat tttttgggat atgggaagtt 1980
atttgttgtt tgtagttggt tatgatatat gatagaagat tgttttttgt gtttttttag 2040
agtaaatggg gttttgtatt tatttaataa atatggttta ggatgttggg ttaagtaata 2100
taaagttttt gtttttgtgg atatttatga atgtataggg tgattttttg tgatgttagg 2160
gttatgaaga aaataaaata gagttttggg ttggatatga ttggggaggg tttgtttttt 2220
ttgtgatagt aagggaaggg ttttttgaag tgttattttt gttgagaagt ggataaagat 2280
aaggattagt tttggggtaa gttataggag agaggtatta ggtaggggga atagtaagtg 2340
taaagatttt gggtatgttt gaaagataga aagtagaaag gtaagaggaa gtggtttttt 2400
gtttatggtt gatagagttt tattttttag taaggatttt ggggtgagt tgatttaaaa 2460
ttgtagtaaa attaggagaa tgatttagga ttttagatga ttagttataa tagatgattg 2520
tagataatta atataattat tatttttatt attgatattt tgattttgtt ttgtattttt 2580
aaaagtagga ttttgtattt tattgtatta tagtttttat aataattttt gtgggtagga 2640
aaagtaagta taagtattat ttttattttt tagatgagga attggtatag aaagatggga 2700
tgattggtgg ttagttagga atttgttatt tttttttatt gttttttttt atttgaagag 2760
agatatgtat ttttttgaat gttagtagtt gttttattta attgtaaatg gttatttgta 2820
gttgggattt ttttattttt tatattttttg tttttttttt tttttttttt gttttatttt 2880
gttagaaatg ttataagttt ggaataaata gaaataggga gaaatgtaag tttagttttt 2940
tttttagaat ttttttgttt atattttaga taagtgatat tgggatagta gaggttgaag 3000
aatttgtgag agatggtatt tttaagaata tttttgaaat tttttttttt tttttgagta 3060
ttttagttat aggaaaggga aattagtttt tatatttttt tgatttgttt tttttattta 3120
gttttaaaaa ttttttttgt ttttatttta attgtggttt aatttgtaga gatgtttttg 3180
ttttttgatg tttttttagt attgttttta ttgttttatg gggtatgatt ttattgtttt 3240
tttaattata gtaggaatat tttgaggttt gggatattta gttatattgt tttttagttt 3300
ttttagaatt aaatagggtt ttaggagatt ggagagtatg ggtgtggtta atagttgatt 3360
gagttttgag tagagtgttt tggaggttat tttaaggatt taggttgaat gagggtatat 3420
gtggtggaat ttgagagatt tgtttatttt tgtgttttat atgatttatt ttatagttat 3480
gtggaaggtt ttagaagatt ttttagatta aatttatttt ggataagtat tttatatgat 3540
agaggttttt tttttttttt ttttttatat tttagttttg tttaaagtgt tgatattttt 3600
tttaatggaa ggttttgttt ggaatataag tattaagtag ataatttttg ttgaatttta 3660
agtagtagtt tttttataga aagtatttga agtgtttttt tttagtagtt atttaattat 3720
gaaagtataa gtataatttg attttttttt tagttgaaaa gtaatgaaat tattttttgg 3780
ttaatttttt tttatttttt attttaagtt ggtttatata gtgttttgaa gtttagtgaa 3840
ttttaagagt tttttagttg ggatgttttt ttttatttta aatttgagtt agtttttttt 3900
tgtttattgt gtgaaggtag tttggttttt attttaagga aaagaaatag taaattttat 3960
gaatttttgt gtaggggagt ttttttgtta gggttatttt ttttagtagg tatttattag 4020
tttggatgtt atggtgttta tgagtttaat aatatgtaag aattggttat ttaatattat 4080
ttaataagtt aggtggggtg aatttgaggt taatagtaag aatgaagatg taggttgggt 4140
gtggtggttt atgtttgtaa ttttagtatt ttgagaggtt aaggtgggtg gattatgagg 4200
ttaggagatt gagattattt tggttaatat ggtgaaattt tgtttgtatt aataatataa 4260
aaaattagtt aggtgtggtg gtgggtgttt gtagttttag ttatttggga ggttgaggta 4320
ggagaatggt gtgaatttgg gaggtggagt ttgtagtgag ttgagattat gttattgtat 4380
tttagtttag gtgatggagt gagattttgt ttttaaaaaa aaaaaaaaaa aaaaaaagaa 4440
tgaagatgta ggtttttttg tttttttttg atagttaaga ataatgatag agttatatat 4500
ttgggaagaa ttgagtaagt tttaagattt atttggtttg tttatatatt tattaggatg 4560
atttggtatt tttttttttg gaattttttt aggaaaggtg agtttatatt tatttttttt 4620
gtattaagtt ataggatgtt agagtttgta ggaagagaag ttgtaaaaag gattttagaa 4680
attatttatt tttgttttaa atgtgggaaa aataaggttt agagaagtga aggaatttgt 4740
ttatttttag ggtttagttt tttaagttgt aaggtgtggt ttatgtttgt attgtgggtt 4800
tggaaatttt agttatttat agattttgta tttgtttttt agatttgtag atagatttgt 4860
ttgagttttt gtatagtgtt tagtttatag agaaatttta tttatattga ttaaataatt 4920
ttttattatg ataataatta ttttttgagt taattagaag taatataggt ttgttttttt 4980
gataggttta ttttttgtgt gtttattttt tttttttttt aaattattag ttaatgtatt 5040
ttaaatatat tattataaaa aaaagtatat gaaaattttg tttttgggaa atgaaaagag 5100
agtaaagtgg aaataaaaaa ttaaaagatt tgagatttgt ttttaatgtt gtagaatagt 5160
tgtgtatttg ttttggttaa gttattttgt ttgtttaggt gtttattttt gtgtttattg 5220
gatgaaagat agaatttagt ggtatttagg attttttttg ttttaaaaat tttaagattt 5280
tatggggaat tttgtaggat aagtgaatgt ttttgaaggg gagatgggtt ttgttattta 5340
aataggtttg atatatggga atgtatttaa gattgaagat tatttattag ttttgtaatg 5400
aatattgagt ttttatattg ttattttttt taagaattaa tgttgaaaat agaattttag 5460

agaagattgt gatgttaaag ttttaggaga gataggtttt ggtttatggt ggagtttagt 5520
gtagttatgt gtttatttgt ttgtttgttt tttggattgt agtattaaaa agttaggaag 5580
gtgttttttt tattattatt tttttggttt ttaggagtta ttgaattttt ttatattttt 5640
tagattttag tttagatgtt attttttttg ggaaattttt tttgattttt tttttatgta 5700
tttttataat attttgtatt aatttttttg ataatattta gttgatttta atgtgattat 5760
ttatttattt gttttttttg ttggagttta agtttttaga atatagggat taagtgttgg 5820
taataatgag tttaataagt attggttgaa tgggtttaat gattagtatt attgattttt 5880
ttttagtatt tagataggta ggtggtggtt ttttggagag tttatggagt tttattttgt 5940
tggttggtgt tagggggtgt tataagttag agaatattta tttgtttagt ttgtagtttt 6000
ggttggttgt tttattgtga gggtgggaga gtataatttt atatttattg gtggggtgtg 6060
ggtgtttttg agtgggtgaa gtttattttt tttatttgtt atggttataa agtttttagt 6120
taatattttg tttgggtttg tggttgttgg agttttttta tttttgagtt ggttatatgt 6180
taattaaagg gggagatgtt ttttggggaa gtaatatagt tgtgaattat tggtttgggg 6240
gttttagggt ggtttgggaa tattgtagag gatgttatag ttttagtttt ttagggtaga 6300
gttgggtttt ggaaatttaa tttggagaag gtattgttat ttttgttgtt ttatagggaa 6360
gttttttttt gttttttttt tttttttttt ttagatttta gttatgatgt ttttttttaa 6420
gttttattgt ttatgtgaaa gttattatat ttaaaatggt tttttttaag taatagatag 6480
tttggtttat ttatttttat gagttttggt tagattttta gtggggttgt tgtttagtat 6540
tgggtaattt tgagtttaag atgtgaggta ttgtaggatt tttttgtgtt tggtattagt 6600
agggggtaaa tatttttttt tgtttttatt tggagttaat tttatggtag taaaattgtt 6660
ttagtttttt ttttagtttg ttagtaaatt gaagattttt ttggttatat ttgtttttgg 6720
ttatatttgt atgaattttt aggtttttat attttttttt atagggattt atttttagtg 6780
tttttaagtt agaaaatata tgtgtgtagt aaagttatgt tgtattttgt gttatttagg 6840
gtggtgggaa atggaaagta gagttaaata ttttagtttt tgtttttatg tgtatgtatg 6900
tgtgtgtatg tatgttttaa tgattatata tttaagtagt taattatttt tgtgaagggt 6960
attatgaaat ggaaggagaa gaattttttt ttaattttgt tttaatgttt tttttatgtt 7020
tgtggagttt atgtgttttt tttttaaaaa tgagtagttt ttttgtggtt tttttgaagg 7080
ttgtatgttt agttttatat agagaatata tataagtatt ttagttaggt gttgttatgt 7140
tttgggaggg aatgggatgg atatgtggtt ggttttttgt tagattgggt tggaggtttt 7200
gtttttgtaa ggtatatgag ttttttttag tggaatttgt tggaattgat atggttttgt 7260
tttgtgttgt ggggatagtt taaagagtag ttaatattat tgattgtat 7309

<210> 349
<211> 15863
<212> DNA
<213> Artificial Sequence

<220>
<223> chemically treated genomic DNA (Homo sapiens)

<400> 349
   agttttaatt ttgtaggttt aagtgatttt tttattttag ttttttaagt agttgggatt 60
   ataggtttaa tttttttgat agagatagga tttggttgtg ttattaaggt tggttttaaa 120
   tttttgggtt taagtatttt tttattttgg ttttttaaag tgttgggatt ataggtatga 180
   gttattatat tagttataaa aattttttga atataaattt attggttagg agtagtgttt 240
   atgtttgtaa ttttagtatt ttaggaggtg taaatgggtg gattatttga ggttgggagt 300
   ttgagattag tttggttaat ttggtgaaat tttattttta ttttaaatat tttaattagt 360
   tgggtgtggt ggtgtgtgtt tgtaatttta gttatttagg aagttgaggt agtagaattg 420
   tttgaattgg gatatagagg ttgtagtgag gtaagattat attattgttt tttagtttgg 480
   gttatagagt gagattttgt tttaaaattt ttttttttta aagaaagtta aaaagaatat 540
   aaattgatta tttgtattgt aagatatatt ttttaaaatg ttttaatatt ataattatta 600
   atattataat tatgattttg atgaattatt tttgagagtt gaaaatttga tagttgttat 660
   tattgtttta gaaagtagta taaaaaagaa agagtgttga tggaggagga gaaggtttag 720
   tagagttttg agtgtttgag tgttttagtg tagggaggtg gagatagtga tttatgttgt 780
   taagagatgt agtgaggtga atttagagaa tgatttaaga ggggttgggt gtggggttta 840
   tgtttgtaat tttagtattt tgggaggttg aggtaggagg attatgaggt taggagattg 900
   agattatggt gaaattttgt ttttattaaa aatataaaaa attagttagg tgtggtggtg 960
   ggtgtttgta gttttagtta tttgggagag gttgaggtag gagaatggtg tgattttggg 1020
   aggtagagtt tgtagtgagt tgagatggtg ttattgtatt ttagttgggt gatagagtga 1080
   gattttgttt taaaaaaaaa aaaaaaatat tttttttatg attttttaaa taaattttaa 1140
   atgttttatt tataagaatt aatatgatgt ggttgttttt ttttgttttt atttttttgt 1200
   tttttttatt tgttttagtt tggttatatt agtattttgg gttttttgtt ttgtgtttgt 1260
   ttgtttgttt gtttgtttgt ttgtttgttt tgggatggag ttttattttg ttatttaggt 1320
   tggagtgtag tgatatgatt ttggtttatt gtaattttta ttttttgggt ttaaatgatt 1380
   tttttgtttt agttttttaa gtagttggga ttataggtat ttattattat atttagttaa 1440
   tttttgtatt tttgtagaga tgaggttttg ttatgttggt taggttggtt ttaaattttt 1500
   gattttaggt gatttgttta ttttagtttt ttaaagtgtt gggattatag gtgtgagtta 1560
   ttgagtttag tttgttttgt tgttttttga gatggagttt tattttgttg tttaggttgg 1620
   agtgtagtgg tataattttg tttgattgta gtttttattt tttgagttta agagattttt 1680
   ttgttttagt tatataagta gttgggatta taggtgtgtg ttattatatg tgtttaattt 1740
   tggtattttt agtagtgatg gggttttgtt atattggtta ggttggtttt gaatttttgg 1800
   ttttaagtga tttgtatatt ttggtttttt gagtagttgg gattataggt gtgagttatt 1860
   atgtagggtt tttttggttt tttaatgtat aaagttattt tttagttttg ggtgtttata 1920
   gtattattgt tagtttttgg aaagtttttt ttagaagttt ttatatgatt gattttttat 1980
   ttttttttag gttttagttt aaatgttttt tttttagaga gttttttttt gattatttta 2040
   tgtaatgtgt ttttttttag ttagtttttt tttattttgt gtattttttt tagagtattt 2100
   attattattt tggttttgat gtttgttggt ttatatgttt gttgtttgtt ttttatatag 2160
   aaagtaaatt agttttttat ttgttttgtt tattatttta ttttagtata gtgattaata 2220
   tatattagta gttaattaat aaatagttat aagttaggta tggtgattta tatttgtgat 2280
   ttttgtattt tgggaggtta aggtgggtgg attttttgag gttaggaatt tgatattagt 2340
   ttgtttaata tggtgaaatt ttgtttttat taaaattata aaaattagtt gagtgtgatg 2400
   gtgtgtgttt gtaatattag ttatttgtga ggttgaggta ggagaattgt ttgaatttgg 2460
   gaggtagagg ttgtagtgag ttgagattat attatagtat tttagtttgg gtgatagagt 2520
   aagattttgt tttaaaaaaa taagaaatag aaatatatat atgttgttat aagaaattta 2580
   ttatttttta gtaaaaaata tagattttta attaaagaaa ggggaaattt ttttttaaat 2640
   aattttagaa tttggggtag aaaatgttat atgtggaaat ttgttttgaa aaatagtttt 2700
   gtttgtgaat aggaaatatt tggattatat tttttttatt tttatgaagg atttaaatgt 2760
   gtagtattga tgaagaggta gttagtttta gtttttatta tttttgtata ttttatttgt 2820
   ttttttttat ggtatagttt tgggggtagg ttataaattt gtaagatgtt attagtatta 2880
   ttggttaaga tgtaggaatt gttaggagtt ttagaaatag gggagagtta gaaagttggt 2940
   tagatttatg ttttttaagt gtagggttag ggttgagttt gtttttgggg taggtaagtt 3000
   tttttgaatt tttgagggaa gtagaagata taaattgtta gataaaatgt aagtttagtt 3060
   taaaagggtt atgtgttgtt ttttttagtt ttggggtatt ttttttttag aaaattggat 3120
   tgttttatag tgaaaatttt gggggtggtt agttttttgt tttgttgtta tttttattat 3180
   ttatagtttt ttaagaagtt tttaggttgg gtgttgaatt ttgattagga attattgaga 3240
   aattgaggta gttgggagaa gttgtagttt taagtgttga aaggaagatg ggggataata 3300
   aatttgggtt gttaagtaaa gggggtagag gtttggagaa gtgggtttta ggattagagg 3360
   atagattgat tttatatttt atttttttag attttatatt ttattgttat tattatttat 3420
   gtgttttttt tgttttttgt agtgggtttt ttagaggtat tttttatggt ttttttagat 3480
   tttaattttg gtttgtttgt ttttttttta gaaaggtttt tgtttgtttt ttttgtagga 3540
   aggtttgtat ttttagaaag tttttgtttt ttgatttgag gatttaattt attaggggaa 3600
   ttaaattttg tttttagggg agtggagaga gaaattgggt tttttttttg tagtttttgg 3660
   gatatagttg agttagttat aggatttggg gataattggg gtggattttt tttttgggga 3720
   ggtggtggta ttagtttaga gtttgtattt ttatttattg gggaagtgtg gggagaagga 3780
   tgggttggag ttgggttttg gtttgaagga tagtagtttg gagttaatgg ttgagttttt 3840
   aaagttttta tattgtagag gaagtatagt ggagattagt tttagttagg atggttttga 3900
   agtttttagg gatttgatgt agagttaaag aaatttattt gtgttttttt ttttttttgg 3960
   gagtaggtag aagatttttg ggaggagagg tgaatagtgg atgttaattt ttttgaaagt 4020
   attgtgtttt ttagtattgt gggttgttat gggttttttg ttgttgtggg attttggttt 4080
   attttttgat tgggttgttg tattttggat tagattttgt gggtgattta tggaatttgt 4140
   ggagttggga tgtgaaaggt tagaaggttt tttgttttta ttaagtttta gggttttttg 4200
   tggttgttgg gagttgtagt ttgaatgttt ttattttggt gagaagtgtt tatgtttttt 4260
   ttattgagtt ttgtggtaat ttttaaagta tttgtattgt ttttttgttg tttgtagagg 4320
   gtgtagtagg ttttgtattt tttttgtatt ttatttttta ggttttagat ttgttttttt 4380
   tatttaaaaa atatttatta ttgagttttt atttgttatt tagtattgat ataggtattt 4440
   aggaatataa taatgaataa gatagtagaa aaattttata tttttataag gtttatgttt 4500
   ttatgtattg aaagtaatga ataaataaat tttattagag tgataagggt tgtgaaggag 4560
   attaaataag atggtgtgat ataaagtatt tgggagaaaa tgttagggtg tgatattatg 4620
   gaaagttttt ttaaaaaatg atattttaat tgatgagaag aaaggattta gttgagagta 4680
   aatgtaaaag tttttttttt tttatttttt atatttgata taatgtagga tttttttaaa 4740
   atgattttta ttaattttgt ttttatagtt ttggtttgta gaatttttta ttttaaaatg 4800
   ttagtattta tggtattagg ttggtgagaa ttttgatttt gtattttttt ttttaatttt 4860
   attttttttg ttttttttgg taggtggatt atttgttttt atttgttatg gtgattgttt 4920
   agttttgtgt taggagtttt gtaggggttg atgggattgg ggtttttttt ttttatgtgt 4980
   ttaagattgg tgttaaaagt tttgagtttt ttaaaagttt agagttattg tttagggagt 5040
   aggtagttgt tgggttttgg ggatattttg tgtttgggtt gggagtgtgt tttttatgat 5100
   ggtgatatgt ttttttggat tgggtaagtt tttgattgaa tttgatgagt tttttttgag 5160
   ttatgggttt ttggttttgt gtatttttag tttgggaaaa ttgttggggt tgggggtggg 5220
   gtagtgggga tttagtgagt ttgggggtga gtgggatgga agtttggtta gagggattat 5280
   tataggagtt gtattgttgg gagatttggg tgtagatgat ggggatgtta ggattatttg 5340
   aatttaaagt tgaatgttta ggtagaggag tggagttttg gggaattttg agttggttta 5400
   aagtgtattt ttttgtatat ttatttggtg ttgggtgtag ggaatttttg aaataaaaga 5460
   tgtataaagt attgaggttt gagatttttg gattttgaaa tattgagaat ttatagttgt 5520
   atattttaga gtttatggta ttttagtgaa aattggggtt ttattttgaa atgattattt 5580
   gggggtgatt tggggagttt aagttgttaa ggttttataa tttttggatt tttgtttttt 5640
   ttggagtgat ttttttaggt agtttttggt tttgttagat ggagaaaatt taattgaagg 5700
   ttgttagttg tggaagtgag aagtgttaaa ttaggggttt gtttgttagg ttgaggagga 5760
   ttgttgtaat ttgagaggtt tggtagtttt gttattgttt ggttttatat ttatattttt 5820
   gttttttgta gtagtatttt tggttttttt ttgttggagt agtttattat ttatttgatg 5880
   agaggggagg agagagagag aaaatgtttt ttaggttggt tttttttatt tggtagaggg 5940
   aggttgttat tttttgtttg tatttttttt tttggattat ttagttatgg tttttgtaaa 6000
   ggtaggggta tttgttttga tgtaaatttt aatttttttt tttttttgaa tggtgtgttt 6060
   tattttttgg gttgtttgta atttaggtgg atgttattat ggtgtagata gggagggaaa 6120
   gaagtgtgta gaaggtaagt ttggaggtat ttttaagaat gagtatattt tatttttttg 6180
   gagaaaaaaa aaaaagaatg gtatgtttga gaatgaaatt ttgaaagagt gtaatgatgg 6240
   gttgtttgat aatttgttgg gaaaaataat ttatttgtta tttagttttg ggttaggtta 6300
   ttttagtttt agatgtaggt tgaatgttgt gaagtggaag gggtgggttt gtaggtgttt 6360
   gtgtggtttt ttgtgtagtt tttggtttga gttggttttt tttggtagga ggtggaattt 6420
   gaatttattt tttttgttgt tttatttttt agtttgtggt tgttttattt tgtagttttt 6480
   ttttatgtat ttgttgtgta ttggttattt tgtgttgtat ttatgttatt ttttttttaa 6540
   attgaggtgg tatttatata tagtgttagt gtatatagta agtgtatagg aagatgagtt 6600
   ttggttttta attgttttgt gatgtttatt aagttataga ttttttttat tgttttagaa 6660
   atgttttatt atgttttttt ttagttgatt tttgatttta tttttatttt gatttttata 6720
   attattttgt ttgttggaga attttatata gaatggaatt aggatgggtg ttgtggttta 6780
   tgtttgtatt ttggtttatg tttgtatttt gggaggttga ggtgggtgga ttatttgagg 6840
   ataggagttt tagattagtg tggttaatgt ggtgaatttt tgtttttatt aaaaaatata 6900
   aaaattagtt gggtgtggtg ggtgtttgta attttagtta tttgggaggg tgaggtagga 6960
   gaattgtttg aatttgggag gtagaggttg tagtgagtta agattgtgtt attatatttt 7020
   agtttgggtg ataagaatga aattttgttt taaaaaaaag gggggaatta tatattatgt 7080
   gtttattttt gttgggtttt tgttttttaa tgtattgttt gatatttgtt tatgttgtat 7140
   atattagtat tttgtttttt tttatttagt atagtttatt gattgtatat ttgttttttt 7200
   gatggttttt tgagttgttt tttatttgtg gttatgaaat aaagttgtta taaatatttt 7260
   tgtataattt tttttgtgat tatatgtttt tgtgtttttt ggagaaatat ttaggagggg 7320
   aattgtggag gaagtaaaaa gtagttgtat tttgaatttt tttagaagtt ttgagttttt 7380
   tagagtggtt gtattatttt atattttaat tagtaaggta tgggagttat tatggttgtg 7440
   ttatagtttt ttggatatta ggtatgttag tttttttaat gtggtatatt tttgtggttg 7500
   taatttatag ttttttattg attaaggatg tttagtattt ttttatgtgt ttattggtta 7560
   tttgtatttt gtttgtaaag tagttttttg agttttttat ttgttatttt ggtttttttg 7620
   tttgttttta ttgtttagtt gtgggattgt tttatatttt ttggatataa gttttattta 7680
   gatttatgag ttgtgaatgt ttttttttga tttgttgtgg gtttatttgt ttgttttata 7740
   gagtttatag aattttaaga ggagtggatt aatttttttt atgtttagta tttgttttgt 7800
   tttgtttagg atattttttt tttttttttt aattttaggg ttatgaagat ataattttat 7860
   attttttttt aggattttta tggtggtaag ttttatagta aggtttttaa gttattaatt 7920
   aatttttaaa attaattgtt tatggtgtga ggtgtaggag ttagtttttg gtattttttt 7980
   tgtatggaaa tttagttatt ttgtttttat ttgttgaaat aggttttttt tttttattga 8040
   atgtttttaa ttttaattat tttatagttg gagtataggg ttattatttt agtgttattt 8100
   tttttttttt tttgttaatt tttgagatag ggatttatat tgttgtttag gttagagtat 8160
   aatggtataa ttaaggttta ttgtagtttt gaatttttgg gtttaagtag ttttttagta 8220
   gttttatgag tagttgggat tattttatta tatttagtta attattttat ttttttgtat 8280
   tgataggatt ttattatgtt gtttaggttg gttttaaatt gttggtttta agtttttatt 8340
   ttattttggt tttttaaagt gttgggatta taggtgtgag ttattatgtt tgatttttta 8400
   gtgttatttt ttatttattt tttttgtttt ttgttttttt taaatgttgg aggaagaaat 8460
   agtatttatt ttatataaat tttttagaaa atagaggaat agattgggtg tggtggttta 8520
   tatttgtaat tttagtattt tggtatgttg aggtagggga ttatttgagg ttgggagttt 8580
   gagattagtt tggttaatat ggtgaaattt tatttttatt aaaatataaa agtagttagg 8640
   tgtgtattat atttgtaatg ttagttattt aggaggttga ggtataagaa ttttttgaat 8700
   ttgggaagtg gaggttgtag tgagttgaga ttgtgttatt gtattttagt ttgggtaata 8760
   gagtgagatt ttgttttaga aaaaaaaaga aagaaagaaa aaatagagga atatttttta 8820
   atttgttttt gaagttagga taattttggt attaaaatta aataaggata ttataagaaa 8880
   agaaaatata gattaatatt tttgttagta tagatatgta atagttaatt aattttagta 8940
   aattaaattt ggtaatatag aaaaaaggat aaataggtta gttgtggtgg tttatgtttg 9000
   taattttagt attttgggag gttgaggtag gtagattatt tgaggttagg agtttgagat 9060
   tagtttgatt aatatggtga aattttgttt ttaataaaaa tataaaaatt aggttgggta 9120
   tggtggttta tgtttgtaat tttagtattt tgggaggttg aggtgggtag attatgaggt 9180
   taggagttta agattagttt gattaatgtg gtgaaatgtt atttttatta aaaatatgaa 9240
   aattagttgg tgtggtggta tttgtttgta attttagtta tttaggaggt tgaggtagaa 9300
   ttgtttgaat ttgggaggta gaggttgtag tgagttaaga ttgtgttatt gtattttagt 9360
   ttgggtgata gagtaagatt ttattttaaa aaaaaaaaaa attagttggg tatggtggtg 9420
   ggtatttgaa attttagtta tttgggagtt tgaggtagga gaattgtttg aatttaggag 9480
   gtagaagttg tattgagttg ggattatatt attgtatttt agtttgggta atagagtgag 9540
   attttatttt aaaaaaagaa aaagaaaaag gataaatata ttttaattaa ataatgttta 9600
   ttttatgatt gtagttgatt taatatttaa aaattggttt ggtgtagtag tttaggtttg 9660
   taattttaat attttaggag gttgaggtag gaagattttt tgagtttagg attttaagat 9720
   tagtttgggt aatatagtta gattggtttt tattgggggg aaaaaaatta gtttgtgtaa 9780
   tttattatat taataaaggg aaatataaaa attttatgat tattttaata gatgtagtaa 9840
   aagtagttaa tgatattaat atatatgtat gattataaat taattaattt tttagtaaat 9900
   tagggaaagg aaatttaatt agtttgataa tagggtgttt atagttggag ttttattagt 9960
   agtatatata atggtagaaa atttagtgtt gttgggggtg gtggtttatg tttgtaatgt 10020
   tagtgttttg ggaggtttag gtgggtggat tatgaggtta ggagattgag attgttttga 10080
   ttagtatgtt gaaattttgt ttttattaaa aatataaaaa taaaaaatta gttgggtatg 10140
   gtggtgggtg tttatagtgt tagttatttg ggaggttgag gtgagagaat ggtgtgaatt 10200
   tgggaggtgg agtttgtaga gtttagattg tgttattgta ttttagtttg ggtgatagag 10260
   tgagattttg ttttaaaaaa aaaaaaaaaa aaaaaagaaa agaaaattta atgttttttt 10320
   ttttaagatt aggaattaga aaaggatttg atttttataa tgttgatatt atattggagg 10380
   ttttaattag gtaagaaaaa gaaataatga gggttgggtg tggtggttta ggtttgtaat 10440
   tttagtattt tgggaagttg agatgggtgg attatgaggt taggagattg agttattttg 10500
   gttaatatgg tgaaattttg tttttattaa atatataaaa aattagttgg gtgtggtggt 10560
   gggtgtttgt agttttagtt atttgggagg ttgaggtagg agaatggtgt gaatttaggg 10620
   ggtggagttt gtagtgagtt gagattgagt tattgtattt tagtttgggt gatagagtaa 10680
   gattgtgttt taaaaaaaaa aaaagaaaaa gaaataatga ttagtggttt gatgttttat 10740
   gttagtaatt ttagtatttt gggaggttga ggtgggtaga ttatttgagg tttggagttg 10800
   gagattagtt tgataaagat ggtgaaattt tgtttttatt aaaatattaa aaaaatagtt 10860
   aggtgttggt tgggtatagt ggtttatgtt tgtaatttta gtattttggg aggttgaggt 10920
   gggtggatta tttgaggtta ggagtttaat attagtttgg ttaatatggt gaaattttat 10980
   ttttattaaa aatataaaat tagttgggtg tagtggtggg tgtttgtaat tttagttatt 11040
   tgggaggttt aggtaggaga attgtttgaa tttgggaggt ggaggttgta gtgagttgag 11100
   attgtattat tgtattttag tttgggtgat aaaagtaaaa attttgtttt aaaaaaaaaa 11160
   gaattagtta ggggtagtgg tgaatgtttg tagttttagt tatttaggag gtagaggtag 11220
   gagaattatt tgaattttgg aggtagaggt tgtagtgagt tgagattgtt ttattgtatt 11280
   ttagtttagg tgagaagagt aaaattttat gttaaaaaaa aaaaaaaaaa aggaaagaaa 11340
   aaaaataatg attagaaagg aagaaattaa atatatttat agttagtatg attttatata 11400
   tattatggtt ttaatggggt taggtgtggt ggtttatgtt gtaattttag tatttttagg 11460
   aggttgaggt aggtggtttt tttgggatta gttggttaat atggtgaaat tttaatttta 11520
   ataaaaatat aaaaaattag ttaggtgtgg tgagggtatt tttaatttta gttatttagg 11580
   aggttgaggt aggagaattg tttggatttg ggaggtagag gttgtagtga gttgagattg 11640
   tgttattgta ttttagtttg ggtaataaga gtgaaatttt ggtagggtgt ggttttatgt 11700
   ttgtaatttt agtattttgg gaggttgagt taggttgatt atttgaggtt aggagtttga 11760
   gattaattta atatggtgaa attttgtttt tattaaaaat ataagaatta gttgggtgta 11820
   gtggtgggtg tttgtaattt tagttatttg ggaggttgag atagaagaat tgtttgaatt 11880
   taggaggtgg aggttgtagt gagttgagat tatgttattg tatattatgt tgggtaatag 11940
   agtgagattt tgttttaaaa aaaaaaaaaa gatgaaattt tattttaaaa aaaaaaaaaa 12000
   gttttaatgg aaaatttata aaaagttatt aaaattaata aataaatata gtagggttgt 12060
   aggttatagg gtaatatagt tattttttta tttgtagggg tttggttttg ggatttttta 12120
   tatattaaat ttatagatgt ttaagtttta tatataagat ggaatagtat ttaatttata 12180
   tatatttttt tatatagttt aaattattta gattatttat attattttta tataatgaaa 12240
   atgttaatgt atatgtaagt atgtatgtaa gtatttgtat tatattgttt agggaattat 12300
   tggatagata ggtttttaag attgatatta gtagttattg ttaagatttt ggttaggttt 12360
   gtttttgttt ggggttttag ttgattttat tgttttttta tttagttaag ggtatttgta 12420
   ttttttttgg ttttttggtt atttggaagg tttagtttag tttggtatat ttgtattttg 12480
   gtttattgat gttggtattt ttgggaaggt tttgttttga aaaatatgga gattttagtt 12540
   gttattgaag atttgagaga taaagatagg gagatttgtt tgtagatttg tgttttttta 12600
   agtgggattg agattttggg ttttttattt taggatagta ttttttggtt tgttgattga 12660
   atagattttt gaaggaggtg tagttgtatt ttaggagtgg gggtgggagt agtattattg 12720
   atttgtatta ataattatat agtttttttt agaataataa tatagaataa gtgaaataga 12780
   ataattgtag aaagagttaa tttttgttga gtttttattg tgtgtttagt atttttttta 12840
   atttatattt ttttataata tatagagtat taggtaggtg gggtttgggg gtttatgttt 12900
   gtaattttag tattttagga ggttaagggg ggtggattat ttgaggttgg gagtttaaga 12960
   ttagtttgat taatatggtg aaattttgtt tttattagaa gtataaaatt agttaggtgt 13020
   ggtggtatat gtttgtagtt ttagttattt agtaggttga ggtaggagaa ttatttgaat 13080
   ttgggaggag gttgtagtaa gtggagatag tgttattgta ttttagtttg ggtaataaga 13140
   gttgagattt tgttttaaaa taaaataaaa taaaataaaa taaaataaaa taaaataaaa 13200
   aaagaaaaga gtttgttatt aaaggagttg tttggtaggg gatgttttgt tagtgtaaat 13260
   aatagaaaag tgggttgggt atagtggttt atgtttgtaa ttttagtatt ttgggaggtt 13320
   aaggtgggtg gattatttga agttgggagt ttaagattag tttgattaat atggagaaat 13380
   tttgttttta ttaaaaatat aaaattagtt gggtgtagtg gttgatgttt gtaattttag 13440
   ttatttggga ggttgaggta ggagaattgt ttgaatttgg gaggtagagg ttgtggtgag 13500
   ttgagattgt attattgtat tttagtttgg atgagagtaa aattttgttt taaaaaaaaa 13560
   aaaaaataga aaagtgtaat aaatatttat agtaggtatg ttttttagta aatttgatga 13620
   taaatttggt ataaagaaag agagtatttt tgaaaaaaaa aaaaagaaaa agaaagagag 13680
   tattttgttt gggtaatata gtgaaatttt gtttttataa aaaaatttaa aaattggttg 13740
   ggtgtagtgg tttatatttg taattttagt attttgggag ttggaggtgg gaggattatt 13800
   tgaggttagg agtttgaaat tagtttggtt aatatggtaa aattttattt ttattaaaaa 13860
   tataaaaaat taattaggtg tattggtggg tgtttgtaat tttagttatt taggaagttg 13920
   aggtaagagg attgtttgat attgggaggt ggaggttata gtgagttgag attatattat 13980
   tgtattttag tttgggtgat agggtgagat tttgttttta aaaaaaaaaa gaaaaagaaa 14040
   aagattaaaa aattagttag gtaggttttt gtggttttag ttatttggga ggttgaggta 14100
   ggagaattat tgagtttagg agtggtaggt tgtagtgagt tatgattgta ttattgtatt 14160
   ttagtttggg ttttaaagta agattttgtt ttaaaagaaa aaagaaagaa agaaagaata 14220
   tggtgggtta ggtatagtgg tttatatttg taattttagt gttttgagag gttgaggtag 14280
   gtggattata aggttaggag ttttatatta gtttggttaa tatggtgaaa ttttgttttt 14340
   attaaaaata taaaaaatta gtaggtaggg tggtaggggt ttgtaatttt agttatttgg 14400
   gaggttgagg taggagaatt gtttgaaatt agaaggtaga ggttgtagtg agtttagatt 14460
   gtattattgt attttagttt gggtgaaaag agttaaattt tattttaaaa aataaataaa 14520
   aaaataaaat aaaaaaaaat atggtagttt ttgaaagttt gtttgggaga aggtgtgatg 14580
   atggttgtat aattttgtgt aagatgttgg tttatatagg ggttgttttt tgtttttttt 14640
   tgttttttta attttttata taataggttt gtgtgttatg tatatttatt gagtttaagt 14700
   aggtgtaagg tattgtgatt taatattttg gttagtaaga taataagata gattattgtt 14760
   ttgtttttag gaagtgtata tgttattaga ggaaatagat aaaataaata aggaaaagta 14820
   ttagataatg taagtgttat gagaatgtaa atgaggtgat gtgaattaaa ataggatgat 14880
   ttaagtttgt atggaaggtt tttattttta tgtttttggt tagttaagga attattagtt 14940
   gattagtaga gaagggtagt ttgtttagtt agagtttttg gggaagaggg agtggttgtt 15000
   aagagatgag attaaagaag ttgagatggg ttttttgtga gggggggttg taatgtaggg 15060
   ttgaggagtg tttgaagaga atgggtaggt gagtggtgag atagttgttt ttttagaagt 15120
   tttgtagtga aaggaattaa agaaatggag ttgtgtatta ggtggggaag ggtgggggtt 15180
   aagggggtgt ttttttttat atagagattg taggttgaga atgattatat ttttgttaat 15240
   aggaggtggg agtagggtat ggtagtttat atttgtaatt ttggtatttt aggaggtgga 15300
   ggtgggttga ttatttgaag taaggagttt gagattagtt tggttaatat gtaaagtttt 15360
   gtttttatta aaaatataaa aattagttgg gtgtggtggt atttgtttgt aattttagtt 15420
   atttgggaga ttgaggtagg agaatggttt gaatttggaa ggtagaggtt gtagtgagtt 15480
   gagattatgt tattgtgttt tagtttaggt gatagagaga gattttattt taaaaaaaaa 15540
   aaaaaatata ggaagggagt tgggaatagg gtgtatattt aggaagtttt ggggatttag 15600
   tggtgggaag gttggaagtt tttttttgat tgtttttttt ttaaagaagt gtatggttgg 15660
   tgtggggtgg ggtaggagtg tttgggttgt ggtgaaatat tggaagagag aatgtgaagt 15720
   agttattttt tttttgtttt ataggaagtt gagttgtttt agatattggt atggtgttgg 15780
   gggagggggt tttttttttg taggtttagg tgatttaggg ttggaagtgt tttatgttgg 15840
   atttttattt ttttttttgt agt 15863

<210> 350
<211> 15863
<212> DNA
<213> Artificial Sequence

<220>
<223> chemically treated genomic DNA (Homo sapiens)

<400> 350

gttgtaagag gaaaagtggg gatttagtat gagatatttt taattttggg ttatttgggt 60
ttgtagagaa ggaatttttt tttttaatat tatgttagtg tttgagatag tttggttttt 120
tgtggagtag gaaaagaatg gttgttttat attttttttt ttaatgtttt attataattt 180
aagtattttt gttttatttt atattagtta tgtatttttt tgaggaaaag ataattagag 240
agggattttt aattttttta ttattaaatt tttaagattt tttaaatgtg tattttattt 300
ttaatttttt ttttgtattt tttttttttt ttgagatgga gttttttttt gttatttagg 360
ttggagtata gtggtatgat tttagtttat tgtaattttt atttttgggg tttaagttat 420
ttttttgttt tagttttttg agtagttggg attataggtg agtattatta tatttagtta 480
atttttgtat ttttagtaga gatagggttt tgtatgttgg ttaggttggt tttgaatttt 540
ttattttagg tgattggttt gttttgtttt tttaaagtgt taagattata ggtgtgagtt 600
attgtgtttt gttttatttt tttgttaata aggatatagt tatttttagt ttgtaatttt 660
tgtatgggga aggatatttt tttggttttt attttttttt atttgatata tggttttatt 720
tttttgattt tttttattgt aaagtttttg gaagaataat tgttttattg tttatttgtt 780
tatttttttt ggatattttt tagttttgta ttataatttt tttttatgaa gggtttgttt 840
tggttttttt aattttattt tttaataatt attttttttt ttttaaaagt tttagttaga 900
tgggttgttt ttttttgtta attaattggt ggttttttgg ttagttagga atatgggggt 960
aggggttttt tgtgtagatt taagttattt tattttaatt tatattattt tatttgtatt 1020
tttatagtat ttatattgtt tgatattttt ttttgtttat tttatttgtt ttttttaata 1080
gtatatatat tttttaaggg tagggtagtg atttattttg ttgttttgtt gattaaagta 1140
ttagattata atgttttgta tttgtttggg tttaataaat gtgtataata tataagtttg 1200
ttatatgaga ggttaagaga gtgagaaaga gtaaggggta gtttttgtgt ggattagtat 1260
tttgtatgaa gttatgtaat tattattgta ttttttttta gataagtttt taaaggttgt 1320
tatgtttttt tttgttttgt ttttttgttt gttttttgag atggagtttg gttttttttg 1380
tttaggttgg agtgtagtgg tgtagtttag gtttattgta atttttgttt tttggtttta 1440
agtaattttt ttgttttagt tttttgagta gttgggatta taggttttta ttattttgtt 1500
tgttgatttt ttgtattttt agtagagata gggttttatt atgttggtta ggttggtgtg 1560
gaatttttga ttttgtgatt tatttgtttt ggttttttaa agtgttggga ttataggtgt 1620
gagttattgt gtttggtttg ttatgttttt tttttttttt tttttttttt tgaggtaggg 1680
ttttgttttg aagtttaagt tagggtatag tggtgtaatt atggtttatt atagtttgtt 1740
atttttgggt ttagtgattt ttttgtttta gttttttaag tagttgggat tatagaggtt 1800
tgtttggtta attttttagt tttttttttt tttttttttt ttttggagat ggagttttgt 1860
tttgttattt aggttagagt gtagtggtgt gattttgatt tattgtaatt tttatttttt 1920
agtattaagt gatttttttg ttttaatttt ttgagtagtt gggattatag gtgtttatta 1980
atgtgtttga ttaatttttt gtatttttag tagagatggg gttttgttat gttggttagg 2040
ttggttttga atttttgatt ttaggtgatt tttttgtttt tgatttttaa agtgttggga 2100
ttataggtgt gagttattgt atttggttaa tttttgagtt tttttgtaga ggtagggttt 2160
tattatgttg tttaggtagg atgttttttt tttttttttt tttttttttt ttagggatgt 2220
tttttttttt tatgttaaat ttgttattag atttgttaag aaatatgttt attgtaagtg 2280
tttgttatat ttttttgttt tttttttttt ttgagataga gttttgtttt tgtttaggtt 2340
ggagtgtaat ggtgtgattt tggtttattg taatttttgt tttttaggtt taagtgattt 2400
ttttgtttta gttttttgag tagttgggat tataggtatt ggttattgtg tttggttaat 2460
tttgtatttt tagtagagat ggggtttttt tatattggtt aggttggttt tgaattttta 2520
attttaggtg atttgtttgt tttggttttt taaagtgttg ggattatagg tatgaattat 2580
tgtgtttagt ttattttttt gttgtttgta ttgataaaat attttttatt aaatagtttt 2640
tttaatggta ggtttttttt tttttttatt ttattttatt ttattttatt ttattttatt 2700
ttattttgag atggagtttt agtttttatt gtttaggttg gagtatagtg gtattatttt 2760
tgtttattgt aatttttttt tggatttaaa tgattttttt gttttagttt gttgagtagt 2820
taggattata agtatgtgtt attatatttg gttaattttg tatttttagt agagatgggg 2880
ttttattatg ttagttaggt tggttttgaa tttttgattt taggtgattt attttttttg 2940
gttttttaaa gtgttgggat tataggtgtg agtttttaag ttttgtttat ttagtatttt 3000
atgtattatg ggaaatgtag agttgaggaa agtgttgggt atatagtaag agtttaataa 3060
aggttagttt tttttgtaat tgttttattt tatttgtttt atattattat tttagagaga 3120
attgtgtgat tgttagtgtg gattagtggt attgttttta tttttatttt taaaatgtaa 3180
ttatattttt tttagggatt tatttagtta ataggttagg aggtgttgtt ttgaaatggg 3240
gggtttaaag ttttaatttt atttggaggg atataggttt atagataggt ttttttgttt 3300
ttatttttta aatttttagt agtaattaaa atttttgtgt tttttagagt aggatttttt 3360
taggggtatt agtattagtg ggttaggata taaatgtgtt aggttgaatt aggtttttta 3420
aatggttagg gagttaagag aaatgtaggt gtttttggtt gggtgggaag gtaatgagat 3480
taattgagat tttaaatagg ggtaggtttg attagaattt taatagtggt tgttggtatt 3540
agttttgaag gtttatttgt ttagtgattt tttaaataat atagtataag tatttatata 3600

tatatttgta tgtatattag tatttttatt gtatgggggt aatgtaagta atttagataa 3660
tttaaattat atgggaggat atgtgtaggt taaatattat tttgttttat atatgggatt 3720
tagatatttg tgggtttggt gtgtgaggag ttttagaatt aagtttttat agatagaggg 3780
ataattatat tgttttgtaa tttgtaattt tgttatattt atttattagt tttggtagtt 3840
ttttatggat tttttattag gatttttttt tttttttgag atagagtttt attttttttt 3900
tttttttgag atggaatttt gttttgttgt ttggtgtggt gtgtaatggt atgattttag 3960
tttattgtaa tttttatttt ttgggtttaa gtaatttttt tgttttagtt ttttaagtag 4020
ttgggattat aggtgtttat tattatattt agttaatttt tgtattttta gtagagatgg 4080
ggttttatta tgttaggttg gttttaaatt tttgatttta ggtgattggt ttggtttagt 4140
tttttgaagt gttgggatta taggtgtaag attatatttt gttggagttt tatttttgtt 4200
gtttaggttg gagtgtaata gtgtgatttt ggtttattgt aatttttgtt ttttaggttt 4260
aagtaatttt tttgttttag ttttttgagt agttgggatt agaggtgttt ttattatgtt 4320
tggttgattt tttgtatttt tattagagtt ggggttttat tatgttggtt agttggtttt 4380
agggaagtta tttgttttag ttttttaaaa gtgttaggat tatagtatga gttattatgt 4440
ttggttttat taggattatg gtatgtatag aattatattg gttgtgaatg tgtttgattt 4500
tttttttttt aattgttatt tttttttttt tttttttttt tttttttttt gatatggaat 4560
tttgtttttt ttgtttaggt tggagtgtaa tgggataatt ttggtttatt gtaatttttg 4620
tttttggggt ttaagtgatt tttttgtttt tgttttttga gtagttggga ttataggtgt 4680
ttattattat ttttggttaa tttttttttt tttgagatgg agtttttgtt tttgttattt 4740
aggttggagt gtaatggtgt aattttggtt tattataatt tttgtttttt aggtttaagt 4800
gatttttttg tttaagtttt ttaagtagtt gggattatag gtgtttgtta ttatgtttgg 4860
ttaattttgt atttttagta gagatggggt tttattatgt tggttaggtt ggtgttgaat 4920
ttttgatttt aggtgattta tttattttgg ttttttaaag tgttggggtt ataggtatga 4980
gttattgtat ttggttaatg tttggttatt tttttaatat tttaatagag atgaggtttt 5040
attatttttg ttaggttggt ttttaatttt agattttagg tgatttgttt attttggttt 5100
tttaaagtgt tgggattatt ggtgtgagat attgggttat taattattat tttttttttt 5160
tttttttttt ttgagatata gttttgtttt gttgtttagg ttggagtgta gtggtttgat 5220
tttagtttat tgtaagtttt gttttttgag tttatgttat ttttttgttt tagttttttg 5280
agtagttggg attataggtg tttgttatta tgtttggtta attttttgta tatttagtag 5340
agatagggtt ttattgtgtt agttaggatg gtttgatttt ttgattttgt gatttatttg 5400
ttttggtttt ttaaagtgtt gggattatag gtttgagtta ttgtatttgg tttttattat 5460
tttttttttt tgtttggtta aaatttttag tatggtatta atgttgtgag agttaaattt 5520
ttttttagtt tttgatttta gaggaaaaag tgttgagttt tttttttttt tttttttttt 5580
tttttttttg agatgaagtt ttattttgtt atttaggttg gagtgtagtg gtatgattta 5640
ggttttgtaa gttttgtttt ttgggtttat gttatttttt tgttttagtt ttttgagtag 5700
ttggtattat aggtgtttgt tattatgttt ggttaatttt ttgtttttgt atttttagta 5760
gagatggggt tttagtatgt tagttaggat agttttgatt ttttgatttt gtgatttgtt 5820
tgtttaggtt ttttaaagtg ttggtattat aggtgtgagt tattgttttt agtagtattg 5880
agttttttat tattatgtat gttgttagtg gaattttgat tgtggatgtt ttgttattaa 5940
attagttaag tttttttttt ttagtttgtt aggaggttgg ttggtttgta attatgtata 6000
tgtgttgata ttattaattg tttttgttat atttgttgaa atgattatag ggtttttatg 6060
tttttttttg ttaatgtggt gaattatata gattgatttt ttttttttta gtaaagatta 6120
gtttgattat gttgtttagg ttggttttga aattttgggt ttaagagatt tttttgtttt 6180
agttttttaa aatgttggga ttataggttt gagttattgt attaggttaa tttttgaatg 6240
ttgaattagt tataattatg agataaatat tatttggtta gaatgtattt attttttttt 6300
tttttttttt tttgagatgg agttttattt tgttgtttag gttggagtgt aatggtgtga 6360
ttttagttta gtgtaatttt tgttttttgg gtttaagtga tttttttgtt ttagattttt 6420
gagtagttgg gattttaggt gtttattatt atgtttagtt aatttttttt ttttttgaga 6480
tgaagttttg ttttgttgtt taggttggag tgtagtggta tgattttggt ttattgtaat 6540
ttttgttttt tgggtttaag taattttgtt ttagtttttt gagtagttgg gattataggt 6600
aggtgttatt atattggttg atttttgtat ttttagtaga gatggtgttt tattatattg 6660
gttaggttgg ttttgaattt ttgattttgt gatttgttta ttttggtttt ttaaagtgtt 6720
gggattatag gtgtgagtta ttgtgtttag tttgattttt gtatttttat tagaaatggg 6780
gttttattat gttggttagg ttggttttaa atttttgatt ttaagtgatt tgtttgtttt 6840
agttttttaa agtgttggga ttataggtgt gagttattgt gattggttta tttatttttt 6900
tttttatatt attaggtttg gtttgttaaa attggttagt tgttgtatgt ttatgttaat 6960
aggaatattg gtttatattt ttttttttta taatgttttt gtttggtttt ggtattagga 7020
ttattttggt tttgaaaata agttgggaaa tattttttta tttttttttt tttttttttt 7080
ttttttgaga tagggtttta ttttgttgtt taggttggag tgtagtggtg taattttggt 7140
ttattgtaat ttttgttttt taggtttaag ggatttttgt gttttagttt tttgagtaat 7200
tggtattata ggtatggtgt atgtttagtt atttttgtat tttagtagag atggggtttt 7260
gttgtgttgg ttaggttggt tttgaatttt tgattttaaa tgattttttg ttttagtgta 7320
ttaaagtgtt gagattatag gtatgagtta ttgtgtttag tttgtttttt tgttttttga 7380

agagtttgtg taagatgggt attgtttttt tttttaatgt ttaaagagag tagagaatag 7440
aggagataaa tagaaaatag tattaagagg ttaggtatgg tggtttatat ttgtaatttt 7500
agtattttgg gaggttgaga tgggatgaaa gtttgaggtt agtagtttga gattagtttg 7560
ggtaatatag tgagattttg ttaatataaa aaaataaaat agttagttgg gtgtggtgga 7620
gtaattttag ttatttgtga ggttgttaga ggattgtttg agtttagggg tttgaggttg 7680
tagtaagttt tgattgtgtt attgtatttt agtttgggta atagtgtgag tttttgtttt 7740
aaaaattaat aaagaaaaaa agaaaatagt attaaaatgg tagttttata ttttaattgt 7800
aaaataatta aaattaaaag tatttagtag agaaaggaag tttattttaa taagtggaga 7860
tagaataatt ggatttttat ataggaaaga tattagagat tgatttttat attttatatt 7920
ataaataatt aattttaaga attaattaat ggtttaagga ttttattgta aaatttatta 7980
ttataaaggt tttaaaagaa aatgtaagat tatattttta tgattttggg gttaaaaaaa 8040
aaaaaaaaga tgttttaaat aggataaggt aaatattgaa tataaaaaag attaatttat 8100
tttttttaag attttgtaaa ttttgtaaag taaataaata ggtttgtaat agattagaag 8160
aaaatattta tgatttatgg atttgataag gatttgtatt tagaaagtat aaagtagttt 8220
tataattgaa taataaaaat aaataaaaaa attaaaataa taggtaaaag atttgaagag 8280
ttattttata aataaaatat gaatggttaa taggtatatg aaaaaatgtt gaatattttt 8340
agttaataga gaattgtaaa ttataattat aaggatatat tatattagaa agattgatat 8400
atttaatgtt tggaaggttg tggtataatt ataataattt ttatattttg ttagttggag 8460
tgtaaaatgg tataattgtt ttggaaaatt tagagttttt gaaaaagttt aaaatatagt 8520
tattttttat tttttttata attttttttt taagtatttt tttaagaaat atgaaaatat 8580
atgattataa aaagaattgt ataagaatgt ttatagtagt tttattttat aattgtaaat 8640
gggaaataat ttaaaaggtt attaaaagga tggatatata attgatggat tatattaaat 8700
gaaaaggagt aaaatattga tatatataat atgaatgaat gttagatagt atattgaagg 8760
atagaagttt gataaaaatg agtatataat gtatgatttt tttttttttt ttgagatgga 8820
gttttgtttt tgttgtttag gttggagtgt agtggtatga ttttggttta ttgtaatttt 8880
tgttttttgg gtttaagtga tttttttgtt ttattttttt gaatagttgg gattataggt 8940
atttattatg tttagttaat ttttgtattt tttagtagag atggggattt attatgttgg 9000
ttatgttggt ttggaatttt tatttttaag taatttgttt gttttggttt tttaaagtgt 9060
aggtgtgagt taaagtgtag gtgtgagtta tagtgtttat tttgatttta ttttatatga 9120
agttttttaa taggtaaaat ggttatggag attaaaataa aggtggggtt gggaattgat 9180
tgggaagaga tgtgatgaaa tgtttttggg atgatgaaaa gggtttgtga tttggtaggt 9240
attatggagt ggttaggggt taaaatttat ttttttgtgt atttgttgtg tgtattggtg 9300
ttgtgtgtaa atgttatttt gatttaggaa aaagatgatg taagtatggt ataaagtggt 9360
tggtatgtgg taggtgtatg ggaagaaatt gtggaatgaa ataattgtga gttaagagat 9420
ggggtagtgg gagaaatgaa tttgagtttt gttttttatt aggaagaatt ggtttgggtt 9480
gagggttgta tggaggatta tatggatgtt tgtgggtttg ttttttttgt tttatgatgt 9540
ttagtttgtg tttggaattg gaatggttta gtttaaagtt agataatagg tagattgttt 9600
tttttgataa attattaaat gatttattat tgtatttttt taaaatttta tttttagatg 9660
tattattttt tttttttttt ttttgggaag atgagatatg tttatttttg aaagtgtttt 9720
tgggtttgtt ttttgtatat tttttttttt ttttgtttat gttatggtag tgtttgttta 9780
ggttgtaggt gatttggggg gtggggtata ttatttaaag aaggggaggg attgaggttt 9840
gtattaaaat aaatattttt gtttttgtaa aggttataat taagtaattt agaaaaagaa 9900
atgtaggtgg agaatagtag tttttttttg ttaagtaaga ggaattggtt taaaggatat 9960
tttttttttt tttttttttt ttttattggg tgaatagtga gttgttttgg taaaaagaaa 10020
ttggaaatgt tgttgtaaga ggtagaaatg taaatgtgga gttaaataat aatagggttg 10080
ttgggttttt tagattgtga tggttttttt tggtttggtg ggtaaatttt tggtttagta 10140
ttttttattt ttatgattga tagtttttaa ttggattttt tttatttagt ggagttgggg 10200
gttgtttgga aagattgttt taggaaggat aaaggtttgg aagttgtggg attttagtag 10260
tttgggtttt ttggattatt tttaaatgat tattttggaa tggagtttta gtttttatta 10320
ggatgttatg ggttttaaaa tatatagtta tgagttttta atgttttgag atttaaaagt 10380
tttagatttt aatgttttgt gtatttttta ttttagggat tttttatgtt tagtattggg 10440
tggatgtgta aagaagtatg ttttaggttg gtttaaggtt ttttaaagtt ttattttttt 10500
gtttaggtgt ttaattttga gtttggatgg ttttaatatt tttattattt atatttaggt 10560
tttttaataa tgtaattttt atgatgattt ttttagttaa gtttttattt tatttatttt 10620
taaatttgtt aagtttttat tgttttattt ttagttttag tgattttttt gagttgaaaa 10680
tatatggagt tgagagtttg tgatttagag aggatttatt aagtttagtt aggagtttat 10740
ttaatttagg gaagtgtgtt attgttgtgg aaagtatgtt tttagtttga atgtaaagtg 10800
tttttggagt ttagtagtta tttgtttttt ggatggtggt tttagatttt tgagaagttt 10860
aaaattttta gtgttagttt tgagtatatg ggaggggaaa attttaattt tattaatttt 10920
tgtgaggttt ttggtataaa gttggatagt tgttatgata agtaagggta agtaatttgt 10980
ttgttggagg aagtaaagga aatggagttg ggaggagggg tgtagagtta ggatttttgt 11040
tgatttggtg ttgtagatat taatattttg ggtggaaaaa ttttgtaagt tagagttgtg 11100
agggtagaat tggtggaaat tattttggag gaattttgta ttgtgttaaa tatgaagggt 11160

ggaaggaaga aagtttttgt gtttgttttt agttggattt ttttttttta ttagttaaaa 11220
tgttattttt taggaaggtt ttttgtaata ttatatttta atgttttttt ttagatattt 11280
tatattatat tattttattt aatttttttt ataattttta ttattttgat aagatttatt 11340
tgtttattgt ttttagtata tggaaatgta agttttatga ggatatagaa tttttttatt 11400
attttattta ttgttgtatt tttgagtgtt tatattagtg ttgggtagta agtaagagtt 11460
tgataataaa tattttttga atgagggaga taggtttgaa gtttggagaa tgagatgtag 11520
aagaggtgta agatttgttg tgttttttgt aggtggtggg ggggtggtgt aggtgtttta 11580
agaattattg tgggatttgg tagggggagt gtaggtgttt tttgttaaga tagaagtgtt 11640
tagattataa tttttagtag ttatgaggag ttttagggtt tgatgggaat gggaaatttt 11700
ttaatttttt atgttttggt tttgtgggtt ttgtgggttg tttgtgaaat ttgatttggg 11760
atgtggtggt ttaattggaa ggtggattga aattttgtga tagtaagagg tttgtagtga 11820
tttgtggtgt taaggaatat agtgttttta aaagaattgg tgtttgttgt ttgttttttt 11880
ttttgggagt tttttgttta tttttagaag aggagggaag tataggtggg tttttttagt 11940
tttgtgttgg atttttgaga attttgaagt tattttggtt gaggttaatt tttgttgtgt 12000
tttttttgta gtatgaagat tttggagatt taattgttag ttttggattg ttgtttttta 12060
gattaggatt tagttttagt ttattttttt ttttatgttt ttttgatgaa taaaaatgtg 12120
gattttgaat tgatgttatt gttttttgaa aggggggatt tgttttggtt gtttttagat 12180
tttgtggttg gtttagttgt gttttaggag ttatgggagg gggatttagt tttttttttt 12240
atttttttgg aaatagagtt tggttttttt agtgagttga gtttttgaat tgaggagtaa 12300
gaattttttg aaaatataag tttttttgta gaagaagtaa atgggagttt ttttgaagaa 12360
gaagtgaatg ggttagagtt ggggtttgga aaagttatgg aagatatttt tggggaattt 12420
gttgtagagg atgagggaga tatgtaagtg gtgatggtag tggagtgtgg agtttgggga 12480
gatgaagtgt gaggttgatt tgttttttgg ttttgagatt tattttttta ggtttttgtt 12540
ttttttgttt ggtgatttag gtttattgtt ttttattttt tttttagtgt ttggaattat 12600
agtttttttt agttgttttg attttttagt ggtttttggt tagagtttag tatttaattt 12660
gagaattttt tgaaaggttg taagtggtaa ggataataat ggggtaggga gttgattatt 12720
tttgagattt ttattgtaaa atagtttagt tttttaggag agggatgttt tagagttggg 12780
agaagtggta tgtagttttt ttagattgag tttatatttt atttagtagt ttgtgttttt 12840
tatttttttt aaggatttag gggggtttat ttattttaga ggtaggttta gttttagttt 12900
tatatttgaa aagtataggt ttggttagtt ttttaatttt tttttgtttt tagggttttt 12960
gatggttttt gtattttgat taatagtgtt gataatattt tgtgaattta taatttgttt 13020
ttagagttgt attatgaggg ggagtaggtg gaatatgtag aaatggtaag gattggggtt 13080
aattgttttt ttattaatgt tgtatattta agttttttgt ggagatggaa aaagtgtagt 13140
ttaagtgttt tttgtttata aatgggattg ttttttaaga tgagttttta tatgtagtgt 13200
tttttgtttt gaattttgaa gttatttaga gagaggtttt tttttttttt aattaggaat 13260
ttgtgttttt tgttaaaaag tagtgaattt tttgtggtag tatatatata tttttatttt 13320
ttattttttt gagataaggt tttgttttgt tatttaggtt ggagtgttgt ggtgtgattt 13380
tggtttattg taatttttgt tttttgggtt taagtgattt ttttgtttta gttttataag 13440
tagttggtat tataggtgta tgttattata tttgattaat ttttatagtt ttagtagaga 13500
tggggttttg ttatgttggg taggttgata ttaaattttt gattttagag gatttgttta 13560
ttttggtttt ttaaagtgtg gggattatag gtgtgagtta ttgtgtttgg tttatagtta 13620
tttattgatt gattattgat atatgttagt tattgtgttg ggataaagtg gtggataaga 13680
tagatgaagg gttggtttgt tttttgtgtg ggagatagat aataaatatg taaattagta 13740
agtattagga ttaagatgat gataaatgtt ttgaaggaaa tatatagaat gaagagagat 13800
taattagagg aaagtatatt gtataaaatg gttaggaagg agttttttga aaaggaggta 13860
tttgagttaa gatttgaagg aggataaggg attagttatg tgaaggtttt tggaaagagt 13920
tttttaggaa ttggtaatga tattataaat atttagaatt gggaaatagt tttgtgtgtt 13980
aaaagattag aaaggttttg tatggtggtt tatgtttgta attttagtta tttaggaggt 14040
tgaggtgtgt agattatttg aggttaggag tttgagatta gtttggttaa tatggtaaaa 14100
ttttattgtt attaaaaata ttaaaattag atgtatgtgg tggtatgtgt ttgtaatttt 14160
agttatttgt gtggttgagg taggagaatt ttttaaattt aggaggtgga ggttgtagtt 14220
agataaaatt gtattattgt attttagttt gggtgataga gtgagatttt attttaaaaa 14280
ataataaaat agattgggtt tagtggttta tatttgtaat tttagtattt tgggaggttg 14340
aggtgggtag attatttgag gttaagagtt tgagattagt ttggttaata tggtaaaatt 14400
ttatttttat aaaaatataa aaattagttg ggtgtgatgg tgggtgtttg taattttagt 14460
tatttgagag gttgaagtag aagaattatt tgaatttagg agatggaggt tgtagtgagt 14520
taagattatg ttattgtatt ttagtttggg tgatagagtg agattttatt ttaaaataaa 14580
taaataaata aataaataaa taaataaata taaaataaaa aatttagaat gttggtatag 14640
ttagattgag gtaagtgagg gggatagagg ggtgaggata gagaagagta gttatgttgt 14700
gttggttttt atgggtaagg tatttggagt ttatttggga agttatgaga gaggtgtttt 14760
tttttttttt ttgagataga gttttgtttt gttatttagt tggagtgtag tggtgttatt 14820
ttagtttatt gtaagttttg ttttttggga ttatgttatt tttttgtttt agtttttttt 14880
gagtagttgg gattataggt gtttgttatt atgtttggtt aattttttgt atttttagta 14940

gagatggggt tttattatgg ttttgatttt ttgattttgt gatttttttg ttttggtttt 15000
ttaaagtgtt gggattataa gtatgagttt tgtgtttagt tttttttggg ttatttttta 15060
aatttgtttt attgtatttt ttagtagtat gagttattgt ttttattttt ttgtgttgga 15120
gtatttaggt atttagggtt ttgttgggtt tttttttttt ttattagtat tttttttttt 15180
ttgtattgtt ttttgagatg gtgataatga ttgttaaatt tttaattttt gagagtggtt 15240
tattgaggtt gtgattatga tattaatgat tatagtatta ggatatttta aaagatatat 15300
tttgtaatat agatagttaa tttgtgtttt ttttggtttt ttttgggggg ggggggtttt 15360
gagatagagt tttgttttgt ggtttaggtt ggagagtagt ggtgtgattt tgttttattg 15420
taatttttgt gttttggttt aagtgatttt gttgttttag ttttttgagt agttgggatt 15480
ataggtatgt attattgtat ttggttaatt ggggtatttg gagtagagat ggggttttgt 15540
taagttggtt aggttggttt taaatttttg attttaagtg atttatttat ttgtgttttt 15600
taaagtgttg ggattatagg tgtgagtatt gtttttggtt agtgagtttg tgtttaaaag 15660
atttttgtgg ttggtgtggt ggtttatgtt tataatttta gtattttggg agattaaggt 15720
gggaggatgt ttgagtttag gagtttgaga ttagttttgg taatatagtt agattttgtt 15780
tttattaaaa aagttgggtt tatggtttta gttatttggg aggttgaggt gggaggattg 15840
tttgagtttg tgaggttgag gtt 15863

<210> 351
<211> 6077
<212> DNA
<213> Artificial Sequence

<220>
<223> chemically treated genomic DNA (Homo sapiens)

<400> 351

aaaatatgag aaagtaagta tttgatattt tttattttaa aagagggggg tttttggaat 60
gttattgttt tggtttttag tgattgtggt tttttttttt gttagaggtt ttatgtttag 120
attattagtg atatttttag tttttaaaga ttagtttagt ttatataagg gtaatgttga 180
aatatataag ttaggtttta atattgttat attttattgt aaagtataaa agtatgtttt 240
aatttgttat ttaaagtttt aagatatttt taagtttttt tttatttgta tgttaaaaat 300
taaatttaaa tgggaagtag tattgggaga aagttttttt gtgaattaat aattaaaatt 360
ttttgttagt tttataaata tttttggata atttttttaa atttataaag ggtaagaatt 420
gaggttttat agatgataat gaaaatggta aggaatgtat taattttttt atgtagtgtt 480
gaatttagag ttaaaataat agaagattaa aagataggtt tttatttttg tatatataaa 540
gatgttttgt aaatgtttat ttatttttat taatttattt ttaatatgaa atgggaaatg 600
tttaagtgtt agtattattt ttttaaagat agtttttttt tgtttatatt tgtttattat 660
gtttttattt aagtaaataa ttttaagtaa aaataatgtt tgagtattta tttgttgaat 720
gtatatttta ttatagtttt aagattaaga attgatgttt aaatattaat tgttttgtta 780
gttttattta ttaaggttaa agtagtttgt gttttatgaa ttgatattaa aaaagaaagt 840
tatgttagtt ttttgtggga gaaaagagag ataggtgggt gatggtagat tggaataagg 900
agttattata gtatttattt ttttatattg tagttttgaa aattgtgatt agttatagtg 960
aattttttat gaaatggaat atttttatta atattttgag gaattatata aattttattg 1020
taagatatga aaattagaaa tatggatttg ttttttttag tttttagggt ttttaagaag 1080
atgattttag taatagatta ttattaagtg ttttagggta ttttgattag agtgtataag 1140
ttggaaaaaa atatttaagt taagtaagta gatgtaattt ttttttttat aatttattga 1200
aatttttata aatggtaaaa ataaaaaata aattttttat ttaattgagg aggggggtat 1260
attgaaaata tattaaggtt gagatgttat ttaaattaat gaggtttttt gttttgtagt 1320
tgatgttatt ttatgtgatt tggaataaaa tttttttgaa gaggtaaaaa atgtaatttg 1380
agagttaaat ttttaaaagt tagatagagt ataggaatgt taggagtggt aaaatttgaa 1440
aaagtattat gatatattag ttagttagta taggtggtgg ataaggaaaa tgaagtttat 1500
tttttatgag gtatgttttt ttttagtggt tttttaatgt taattgtttt ttttttttga 1560
ggatatatat attaggtatt tgggttattt tttttttttg aggatatata ttattaggta 1620
tttaggtata tatattatat gttttgaagt ttaaagttat agttttgtga tttaagtatt 1680
tttttgttat ttatggaatg aagattttaa attttagtta tgaggataat tattttttta 1740
tttggggata gaatattagt atttaaatta tttatttggt atgtggtaga ggagaagaga 1800
attagaggag aagtagagat gataaagtag ttatattatt tattagttta taggtaatag 1860
aattattaat ttgttttttg tgataaagta ataataaaga gttgatattt tttatatttt 1920
tatttgtgtg tagttggtat tgttgatatt tattttttta agggtatttt tttagatttt 1980
ttatttgttt atggtatttt tttgggtttt gtatttgttt gggtatttgt tggggttttg 2040
tatttgtttg gtttttatat ttgtttgggg tatttgtttg ggttttgtat ttgtttgggg 2100
tatttgtttg ggttttgtat ttgtttgggg tatttttttg ggttttttat ttgtttaggg 2160

tatttttttg ggttttgtat ttgtttgggg tatttgttta gatattgtat ttgttttggg 2220
tatttgtttg ggttttgtat ttgtttgggt attgtatttg taggattttt aagttgtttt 2280
tatttatgtg gttgtttttg gttttgtttg ttggggatgt tggtattgag gatgttttgt 2340
ttgtggttta ggtttttgtt gtttattagg tatttgttgt ttggggagaa tttgtagagt 2400
aagttggaga gtttgaatat tttggagaag tttatggttg ttgtttgttg tgggtgttat 2460
tttgtgtttg aaaatttgtg ggatttttgg gtgtgtagta ggttgtaata gttgatgttg 2520
gtttttgagg ttggaagtta gaaggtggaa gtgaattgta gtttattagt gttgttggtt 2580
tttgtgtggt attgtggggt ttgtagtttt tgtgttgtag ggttttaaag gaaatgttta 2640
tgtttttttt gattagggat ttttgatttg agaattttat tttaaaggtt gggaggtttt 2700
tgagtatttt tagttagggt tgttgataaa aatgtagaaa gtatagtaaa atttgaattt 2760
tagatttata ataaatttag ttataagtat gtttttaaat attgtatggg atatgttaat 2820
atggaaaaat tatttgttag tttgaaattt aaatttaatt gagtgatttg tgtgtttgtg 2880
tgtgtgtata tatgtatata tatatattta tatttatatg taaatgtatg tttatatgta 2940
aatatatgtt tatttataaa tatattttta ataagtaata tggtgtttgt tgtatatata 3000
ttatattgtg tatgtaatgt ataagtattt attttgtttg tttggggttt tgtttgtttt 3060
tgttgagttt gattttttta tttgttgttt ggtttttgtt ttatgtttta gtgttattga 3120
gattaaggag agaatgaatt tgttgttgat tgggtagagt gagtgtgtgg attgtggtta 3180
ttgtttgttt attatttgtg tgtatttggg ttggtattgg gtgaagaatt gtgtgggttt 3240
gggatttggg ggtttagagg gagtgagttt ttgtgtgggt gtttggtttg taggttttgt 3300
aggtttaggg gtgtgttttg tttttatttt tattttggat tttggttttt ttttttagat 3360
agtggttttt tttatttttg gtttttgtag gttgttagta gtttgtgtta ggttttgttg 3420
gtgtttttag ggttttttag attgtgtaga ttttgatatt tttgtttggt tttgggtttt 3480
gggagttgag agttggttag ggttttgttt gtatttttgg gtgtttagtt ttgggtttgt 3540
tttttgtgga tgttttaatt tttttggttg aatggatggt ggtgtgtgtg tgttttattt 3600
tggtggtgtt ggtttttttt gttgttaaaa ttagatttaa atttttgtat gggatttgtt 3660
tttgggtttt tattttgtgt gtttagtaaa tagtgggtga gttatgaaga tgtgtgagtt 3720
agttggattt tttttgttag gtgtggattt gttgtggtta gagaatttag tttgtgttag 3780
tttggtttgt ttgtgaagtt atgggtttta ttgatgtgat tttttaagat gtgggggtta 3840
ttatgggtag aggatattgg tttggagtta gattatgggt tttataagta ttagattata 3900
agtagtgttg ttattgagag ttgtttggaa tttgtttagt atgttgggtt ttttagttag 3960
ggtttggtgt atgtggttga gggttttgga agttttgatg gtttaggagg agtaggtggg 4020
tggggtggtg ggtgttgttg gttggtagag agttttggtt tgatttagtg taggtttggt 4080
gtgtgtagag aataatttta agtgtattga tgtttgtgag ttttttttaa atattgaatg 4140
ggatttagag tttgagttta taggtggtgg ttgggggagg gagtagggtg ttggttgttg 4200
tttgggagtg tttgtgtttt gggtgatttt tggaaggatg tggggtttaa attttggttg 4260
gggttgggag agtagttttt agaggttttt tgtgggattt tttgttgggt gggattgtgg 4320
ttttatagga gaagtgggtg gtaagttttg tttggtggaa agtagttgtt tttttttttt 4380
tgggtttggg gtggtgtttt ttatttttgt tttttgtttt ttatttttgt tttttgttgg 4440
ttatattttt tgttttttgg attttaagtg ttttgtgtgt tgaggagttt agtgttagtg 4500
gtggtggtta ggagagattt gggtgttagg aaagatgggt tgtttggggg atagtaggga 4560
gtttggggga aatgtaggtg ttgggtatag agttggtatt ggtgttttta gttttgttga 4620
agattgtggt tgggtttggt ttgtgggagg ggttttggtg ttggatttgt tttggttttg 4680
tgtgggtggt tttgttgggt tttgtaggag tgatgtgtgt taaaaggtgg tgggaaggag 4740
gtggggtaga gtgtgtttgg gatttgattt tggatgtggt tagttggaga ggtggagtgt 4800
tgggaggaga ttttggtttt gttgtgattt ggtggtttgt gttgtttttt tgtgtgttgg 4860
gttaaaaagg tgttaatgtt tgtggttgtt tattttttgt ggtgtttttt ttttttgtgt 4920
ttatataatt tgtttagggg ttgggtagtt tgttttgttt ttttgtttgt gtatttgttt 4980
ggaggtttgt gtgtttgtga aggggatgta gtgaaattgg ggtttgtgtt aggttagttg 5040
ggatggatgt tgatgtttgg ggttgtgatg gttgtaggta ggaggtttag ggttgggggg 5100
tggtttggtt ttgtgggtgg gggttggagt gtagtgttgg gtaggtattt gggtttgtag 5160
ttttgaagtt gggaggtgag gggagagtga ttggggatga gttgggataa ggtgatatag 5220
gggttttttt ggagttggat tggtttttgg gatttggtgt ttgtgagagg ttggagtggt 5280
tagagtttag tttgtgagga gatgtgggtt ttgtttttag tgttggttgt ttttggtgtt 5340
aaagatagtt ttgtaggggt tttgggaggg tttttttttt gttgtttttt ttttattttg 5400
ggttttgagg gttgttggga gggtaatttt gggaagaggt tggggtgtgg ggtgtgggtg 5460
taggtggaaa ttgttagtaa gttttttttt gtttgtgtgt tttttttgat ttgtagggtt 5520
gtgttaattt tgaggtttta gtttttttga ggagttaggg ttaggttttt ttttggatag 5580
ggagaaggat ttgggtgggg gttttgattt atggagttgg ttattaagtg gttttgatgg 5640
ttttttgagg gatagttttt tgtggttttg agtttgtttg ttgagggttt ttggtttgtt 5700
tttggagtgg ttttaggtag agaaagtttg tgaagaaatg gtttgggttg gtttggaggg 5760
agatatttta tgttttttta gtttttgggt tgtttttttt tgtagttttt gttttttttg 5820
gggtttggat ttggggagtg atgattattt ttgtttagtt tgtattttgg tttggatgtt 5880
aggagataag tttatgtagt atgtatatgt ttgttatata aataggggat agatagatga 5940

tttttaatta gtaagggtgt agggaaaagt aatgtatttt aaatttttga ttagaggtta 6000
tttgttttta aagatgttgt tatttgttta tttattgttt ggatatttgg aaatggttta 6060
ggtttattaa tataatg 6077

<210> 352
<211> 6077
<212> DNA
<213> Artificial Sequence

<220>
<223> chemically treated genomic DNA (Homo sapiens)

<400> 352

tattgtgtta atgagtttga gttattttta aatgtttaga tagtgaataa atagatggta 60
gtatttttgg aagtaaatga tttttggtta gaagtttggg gtgtattgtt tttttttgta 120
tttttgttgg ttgaagattg tttatttgtt ttttgtttat gtagtagatg tgtgtatatt 180
atatgggttt attttttagt gtttaggtta aaatataagt tgagtaaagg taattattgt 240
tttttaagtt taagttttgg gaaaggtagg ggttgtagga ggaggtggtt taggagttaa 300
gggggtgtga ggtgtttttt tttaggttgg tttgggttat ttttttatgg gtttttttta 360
tttgggattg ttttggagat aggttaggag tttttgatag ataaatttag agttataggg 420
agttgttttt tgggaaatta ttgaaattgt ttagtaatta attttatggg ttaaggtttt 480
tgtttagatt tttttttttg tttagagggg ggtttggttt tggtttttta gggaagttga 540
ggttttggga ttggtatagt tttgtaggtt ggagggagtg tgtgggtggg gaggagtttg 600
ttggtgattt ttatttgtat ttgtgttttg tattttggtt tttttttggg gttatttttt 660
taatggtttt tggagtttgg ggtggagagg ggatagtagg aggagggttt ttttggaatt 720
tttgtggggt tgtttttggt gttaaaggtg gttggtgttg agggtaggat ttgtgttttt 780
ttgtaggtta gattttggtt gttttagttt tttgtgagtg ttaagtttta ggggttgatt 840
taattttgag ggagtttttg tgttgttttg ttttagtttg tttttgattg tttttttttt 900
attttttagt tttggagttg tgagtttagg tgtttgttta gtgttgtgtt ttagtttttg 960
tttgtggagt tgaattgttt tttggttttg ggttttttat ttgtagttgt tgtagttttg 1020
ggtattggtg tttgttttgg ttggtttggt gtgggttttg gttttgttgt gttttttttg 1080
tgggtgtgtg agtttttggg tgggtgtgtg ggtggggagg tagggtgggt tgtttggttt 1140
ttaggtgggt tatatgggtg tggggagggg aggtgttgtg gggagtaggt ggttgtgggt 1200
gttagtgttt ttttagtttg gtgtgtggga aggtagtgtg ggttattgag ttgtggtggg 1260
gttaaggttt ttttttggtg ttttgttttt ttagttggtt gtgtttaagt tggggttttg 1320
ggtgtgtttt gttttgtttt ttttttgttg ttttttggtg tgtgttgttt ttgtagagtt 1380
tggtgaggtt gtttatgtag ggttgaagta ggtttggtgt tagggttttt tttgtgggtt 1440
agatttgatt gtgatttttg gtagagttgg ggatgttggt gttgattttg tgtttgatgt 1500
ttgtgttttt tttggatttt ttgttgtttt ttagatggtt tatttttttt gatatttggg 1560
ttttttttgg ttgttgttat tagtgttggg ttttttggtg tgtggggtgt ttggaattta 1620
aggggtaggg gatgtggttg gtggggaata ggggtgaggg gtggggaata ggggtgaggg 1680
gtgttgtttt aggtttagga ggaggggaat ggttgttttt tgttaagtag ggtttgttat 1740
ttattttttt tgtggagtta tggttttgtt tggtagagga ttttgtggag agtttttggg 1800
ggttgttttt ttaattttag ttggagtttg ggttttatgt ttttttaggg gttatttagg 1860
atgtgaatat ttttgggtgg tggttagtat tttgtttttt tttttggttg ttgtttgtgg 1920
gtttgggttt tggattttgt ttggtgtttg gaaggagttt gtgggtgttg gtgtgtttgg 1980
agttgttttt tgtgtgtatt agatttgtgt taggttaggt tgaagttttt tattggttag 2040
tgatatttgt tgttttgttt gtttgttttt tttaggttat tggggttttt agggtttttg 2100
attatgtata ttaggttttg gttaggggat ttgatatgtt gggtgagttt tgagtggttt 2160
ttagtggtgg tgttgtttat ggtttgatgt ttatggggtt tgtgatttgg ttttgaattg 2220
atgttttttg tttatggtga tttttatgtt ttggaaagtt gtgttagtga agtttgtggt 2280
tttgtgagtg agttgggttg gtgtagattg ggttttttgg ttgtagtggg tttgtgtttg 2340
atggggaggg tttggttgat ttgtatattt ttatggttta tttattgttt gttgggtgta 2400
tggggtgggg atttaaagat gaattttatg tagaggtttg ggtttagttt tggtaataga 2460
aaaggttggt attgttggag taggatgtgt gtgtattatt atttatttgg ttggggaggt 2520
tggggtgttt gtggggagta gatttgaggt tgggtgtttg gaggtatgag taggattttg 2580
gttggttttt agtttttaga atttagggtt aggtggggat gttagggttt gtgtgatttg 2640
gggggtttta gaggtgttgg tggggtttgg tgtggattat tagtggtttg tgggagttag 2700
gggtggaggg ggttgttgtt tagggaagag gattggggtt tggagtgggg gtgagaatga 2760
ggtatgtttt tgagtttgtg aaatttgtgg attgagtgtt tgtgtaggag tttgtttttt 2820
ttgggttttt aggttttaga tttgtatgat tttttgtttg gtgttagttt agatgtgtgt 2880
gggtgatgaa tgggtggtgg ttgtgattta tgtgtttgtt ttgtttagtt agtggtaaat 2940

ttgttttttt tttgatttta gtggtattgg agtgtgaggt aagggttagg tggtaggtag 3000
aggggttgga tttagtaaaa gtaaataaaa ttttaagtaa atgaaataaa tatttatata 3060
ttatatatat gatataatat atgtgtgata gatattgtat tatttattaa agatatattt 3120
gtaggtaaat atatatttat atgtaaatat atatttatat ataaatataa atatatatat 3180
atgtatgtgt atatatatgt agatatatag gttgtttaat taaatttgaa ttttagatta 3240
gtgagtaatt tttttgtatt agtatgtttt gtgtaatatt tgggaatata tttataatta 3300
gatttgttgt gaatttgaaa tttaaatttt attgtgtttt ttatattttt attagtagtt 3360
ttagttggag gtgtttaaag gttttttggt ttttgaggta aggtttttgg gttggaaatt 3420
tttggttgga agaaatgtgg gtgttttttt taaagttttg tggtataaga attataagtt 3480
ttataatgtt gtgtggaagt tggtggtgtt gataggttgt agtttatttt tgttttttga 3540
tttttggttt tggaggttgg tgttggttgt tgtagtttgt tgtgtgttta ggggttttgt 3600
gggtttttgg gtgtagggtg gtgtttgtgg taggtggtgg ttatgaattt ttttgaggta 3660
tttaagtttt ttagtttatt ttgtaagttt tttttggatg gtaagtattt ggtgagtggt 3720
ggggatttgg gttatgggta ggatattttt ggtgttagtg tttttggtgg gtagggttgg 3780
gggtggttgt gtgggtggag gtagtttggg gattttgtag gtgtggtgtt tggataggtg 3840
tggggtttgg gtaggtattt ggggtaggtg tagtgtttga gtaagtgttt tagataggtg 3900
tgggatttag gaagatattt tagataggtg gagggtttgg ggaggtgttt taagtaggtg 3960
tggggtttag gtaggtattt taagtaggtg tggggtttag gtaggtattt taagtagata 4020
tgggggttgg ataggtgtag gattttggta ggtgtttggg taggtgtggg atttaggaag 4080
atattgtaga taggtggggg gtttggggag gtgtttttgg aaaggtgggt attggtagtg 4140
ttgattgtat ataggtgagg gtataggaga tgttggtttt ttgttgttat tttgttataa 4200
aaggtaaatt gatgattttg ttgtttgtaa attggtaagt ggtgtggtta ttttgttatt 4260
tttatttttt ttttaatttt tttttttttt attatatgtt gaataaatga tttaaatatt 4320
agtattttat ttttaggtaa ggaagtagtt gtttttatgg ttgggatttg aggtttttgt 4380
tttatgggtg gtagaagggt gtttaaatta tagagttata attttaagtt ttaaaatata 4440
tagtgtatgt atttggatgt ttagtagtgt atatttttag aagagaggga taatttggat 4500
gtttagtatg tatgttttta gaagaggggg ataattggta ttggaaagtt attaagaaga 4560
aatatgtttt gtaaaaagtg ggttttgttt tttttattta ttatttgtat taattgattg 4620
atgtattatg atgttttttt aggttttgtt atttttgata tttttgtgtt ttgtttagtt 4680
tttgagagtt taatttttaa attatgtttt ttattttttt agggaggttt tattttaaat 4740
tatataaaat agtattagtt ataaaataga aaattttatt agtttaaatg gtattttagt 4800
tttggtatat ttttaatgtg tttttttttt taattaaatg agaggtttgt tttttgtttt 4860
tattatttat gaaaatttta gtgaattata aggagaaaga ttatatttgt ttgtttaatt 4920
tgggtgtttt tttttagttt gtatattttg gttaaaatat tttgaaatat ttggtaatga 4980
tttgttattg gaattatttt tttgaaagtt ttgaggattg gggagagtag gtttatgttt 5040
ttagttttta tattttgtag tagagtttat gtggtttttt aaggtgttgg taaaaatatt 5100
ttattttata aggaatttat tgtaattgat tatagttttt aaagttatag tgtgagaaag 5160
taagtgttgt ggtagttttt tgttttaatt tgttgttatt tgtttgtttt tttttttttt 5220
tatggaaaat taatgtggtt tttttttttg atattggttt ataaaatata aattattttg 5280
attttgataa gtaaaattaa tgaagtagtt aatatttaaa tattagtttt tgattttgaa 5340
attgtagtaa aatatatatt tagtaggtga gtatttaggt gttattttta tttgaaattg 5400
tttgtttgga tagggatata atgaataaat atggataaga agaaattatt tttaagaaaa 5460
tagtattaat atttaaatat tttttgtttt gtgttaggaa taggttaatg gagataaatg 5520
agtatttgtg aagtattttt atgtgtgtag aagtgaaagt ttgttttttg attttttgtt 5580
gttttggttt tgagtttggt attgtataag aagattgatg tattttttgt tatttttgtt 5640
gttatttgtg aagttttaat ttttattttt tataaatttg aggaaattgt ttaaaagtgt 5700
ttataaaatt agtagaggat tttggttatt agtttataga agagtttttt tttaatgtta 5760
ttttttattt aagtttagtt tttaatatat agataagaag ggatttaaaa atgttttaaa 5820
gttttaagta gtgagttaaa atatattttt atattttata atagagtata gtaatattaa 5880
aatttggttt gtatatttta atattatttt tgtatgaatt gaattgattt ttaggggttg 5940
gggatgttat tggtgattta gatatgaaat ttttggtagg agagaaggtt atagttattg 6000
aaggttaggg tagtagtatt ttgaaggttt tttttttttg gagtggagaa tgttaggtgt 6060
ttgttttttt atatttt 6077

<210> 353
<211> 2501
<212> DNA
<213> Artificial Sequence

<220>
<223> chemically treated genomic DNA (Homo sapiens)

<400> 353

agttgggatt ataggtgtat gttattatgt ttggttaatt tttttatttt ttatagagat 60
aggattttgt tatgttgttt aggttggttt taatttttga atttaagtga tttatttgtt 120
ttgggttttt aaaattttgg gattataggt gtatagtgtt aggtttttat atttttatat 180
atttttttgt ttttttagtt gtgaaatgga gataatgata tagttttaat ttatgtggga 240
tgtttagtat agtgtttggt atttgataga tttttttttt tttatttgaa atttattttt 300
gagttgggta tggtggttta tatttgtaat tttagtattt gggaaaatta aggtaggtag 360
attatttgag gtgaggagtt taagattagt ttggtaaata tggtgaaatt ttgtttttat 420
taaaagtata aaaattattt gggtgtggtg gtatatattt gtaattttag ttattttaga 480
ggttgaggta tgataattat ttgaatttgg tagatggagg ttgtagttga gttgagattg 540
agttattgta ttttagtttg ggtgataaag tgagattgtt taaaaaaaaa ttatttttaa 600
tttaatataa aattttttga attttttttt ttttgagttg tttttttatt tgagtttatt 660
ttatattatt gaatagttaa ttatagaatt gtgtattatt ttttttgatt tttagatttt 720
tagtttttta tagtattttg tttttttttg ttgtattagt tatagttgta tttttttttt 780
tttttttttt gagatagagt tttattttgt tatttaggtt gtagtgtagt agtataattt 840
tagtttattg taatttttgt tttttgggtt taagtaattt ttttgtttta gttttttaag 900
tagttgggat tatatgtgtg tattattatg ttaggttaat ttttgtattt ttagtagaga 960
tgaggtttta ttatgttgtt aggttggttt agaatttttg attttgtgat tagtttattt 1020
tagtttttta aagtattggg attataggta tgagttattg tatttggtta tagttgttat 1080
tttatttgtg tgattgatta ttgtatgatg tttttattga agtgtagtga aggtaggaaa 1140
tatgattggt ttttataata tttttaatat ttattattat aggtggtata tagtagggtt 1200
ttaataaata tttgtaaaat gaataaagtt aatgagtgtt tatgtttgta gttttaattg 1260
ttttgaagta aatatttgat tttttaattt ttattagagt gttttgtata tagtaggttt 1320
aattattttt ttttttttat taattattta tgttttgtaa gaatttagtt agttgatttt 1380
ttgtatttta taagtgtgat gattgggtgt ttttgtgtga gatgtgttat ttttgaattt 1440
tgttatgatg ttggtatatt gtgtgtttga tatgaagaaa aaaaaaattt agttagttta 1500
ttaggtatag tggttaaggt ttgtaatttt tgtattttga gaggttaagg taggaggatt 1560
atttgaattt aggagtttga gattagttta ggtaatatag tgagattttg ttttaaataa 1620
taaataaaaa taattagttg ggtatggtgg tgtgtgttta tagtattaat tatttgggag 1680
gttgaggtga gatgattgtt tgagttaggg aggttaaggt tgtagtgagt taagtttgtg 1740
ttattgtatt ttagtttggg tgatagagtg agattttgtt ttaaaaaaaa aaaaaatatt 1800
aaattttaga ggggtgaaaa aaaattttat agtggaaata tagtaatgag ttggtttagt 1860
tttgtttttg ttataatggt ttatggtgtt ggaggatttt tttgtgtatg tgtatagttt 1920
ttggttggtt atttttgtga gtgtgtttta ttttttattg agtgtgtgtg aagattatgg 1980
aggttgattt gggagtgtgt atgtagtttt gttttgtgtt tgatttgtgg attttgtggt 2040
gtttggtttg ggtggtttgg attgtggagg gaatgttttg gagggtggag aattgggatg 2100
aggttgaggt aggtgtggag gaggtaggtg ttgaagagta tggttttgaa gaagatggtg 2160
gggaggagtt gggtgttgag gagtttggtt tggaagagtt tggtttggag gaattgggtg 2220
ttgaggagga gatggaggtt gggtggttgt ggtttgtgtt gtgtttggtg aatttgtgtg 2280
agttttttta ggttattttt tgtaattgta gtttgtgtgt tgtgttgttt gtatggttta 2340
attttgatgg tgagttgtag ttttatttaa tgttgttgtt tggtatgggt tgttgtattt 2400
atagttattg aggtatgggt ttggtgttta ggtttgattt agtagggatg atagtatggt 2460
ttgaagtttt tttattgttt tggggtttat tttgtagatg g 2501

<210> 354
<211> 2501
<212> DNA
<213> Artificial Sequence

<220>
<223> chemically treated genomic DNA (Homo sapiens)

<400> 354

ttgtttgtaa aatggatttt ggggtggtag aggggtttta gattgtgtta ttgtttttgt 60
tgggttgggt ttaagtgttg ggtttgtatt ttggtagttg tggatgtggt ggtttgtgtt 120
aggtggtagt gttgggtagg gttgtggttt gttgttgaag ttgagttata tgggtagtat 180
gatgtgtgga ttgtgattgt agaagatgat ttgggagggt ttgtgtgagt ttattgagtg 240
tagtatgggt tgtggttgtt tggtttttat tttttttttg gtgtttagtt tttttgggtt 300
ggattttttt gggttggatt ttttggtgtt tgattttttt ttgttgtttt ttttagggtt 360
gtattttttg atgtttgttt tttttgtgtt tattttggtt ttgttttagt tttttgtttt 420
ttggggtatt ttttttgtga tttagattat ttgggttgga tgttgtggga tttgtgggtt 480
ggatgtgggg tggagttgtg tgtgtgtttt tgagttgatt tttgtagttt ttgtgtgtgt 540

ttggtagagg atggaatgtg tttgtggaaa tagttggttg gaggttgtgt gtgtgtgtag 600
aaggattttt tagtattgta ggttgttgta atggaggtga ggttaggtta atttgttatt 660
gtatttttat tataaaattt tttttgtttt ttttaaggtt tggtgttttt tttttttttg 720
agataggttt ttattttgtt gtttgggttg gagtgtagtg gtgtgagttt ggtttattgt 780
agttttgatt tttttggttt aagtgattgt tttgttttag ttttttgagt agttggtatt 840
gtaggtgtgt attattatgt ttgattaatt atttttattt gttgtttgaa atggagtttt 900
gttatgttgt ttaggttggt tttgaatttt tgggtttaag tgattttttt gttttggttt 960
tttaaagtgt agggattata ggttttagtt attgtgtttg gtggattaat tgaatttttt 1020
ttttttttat gttagatgtg taatgtgttg atgttgtaat aaggtttgag ggtggtgtat 1080
tttatatggg aatatttaat tattatgttt ataaagtata aaaagttaat taattgaatt 1140
tttataaggt atgggtgatt agtaaagagg gaaaaataat tagatttatt atgtgtagag 1200
tattttggtg gaaattggaa aattagatat ttattttaga gtaattaaaa ttataaatat 1260
aaatatttat taattttgtt tattttataa atatttatta aggttttatt atgtattatt 1320
tatggtgata agtgttgaaa atattgtaaa aattggttat gttttttgtt tttattgtat 1380
tttaataaaa gtgttatatg gtaattaatt atataaataa aatagtaatt atggttgggt 1440
gtagtggttt atgtttgtaa ttttagtatt ttgggaggtt gaagtgggtt gattataagg 1500
ttaggagttt tagattagtt tgataatatg gtgaaatttt gtttttatta aaaatataaa 1560
aattagtttg gtgtggtggt gtgtgtatgt aattttagtt atttaggagg ttgaggtagg 1620
agaattgttt gaatttggga ggtggaggtt gtagtgagtt gagattgtgt tattgtattg 1680
tagtttgggt gatagaatga gattttgttt taaaaaaaaa aaaaaaaaaa gtgtaattgt 1740
gattagtatg ataaaaagag ataggtattt atagaaggtt aaagatttgg gggttaagga 1800
agatggtata taattttgtg attaattgtt taatgatgtg ggatggattt aagtggagaa 1860
gtagtttaga aaaagaaggg tttagagagt tttatattag gttaaaaata attttttttt 1920
agatagtttt gttttattat ttaggttgga gtgtagtggt ttagttttag tttagttgta 1980
atttttattt gttgagttta agtgattatt gtgttttagt ttttggagta gttgggatta 2040
taggtgtgtg ttattatatt taggtaattt ttgtattttt agtagagatg gggttttatt 2100
atgtttgtta ggttggtttt gaatttttta ttttaggtaa tttgtttgtt ttggtttttt 2160
taagtgttgg gattataggt gtgagttatt gtgtttagtt taaaaataaa ttttaagtag 2220
aaagaaaaaa atttattaag tgttagatat tgtgttaagt attttatgtg aattaaggtt 2280
atgttattat ttttatttta tagttaagga aatagaaggg tatataagaa tgtgaaggtt 2340
tagtattata tgtttgtaat tttgagattt tgggagttta aggtaggtgg attatttgag 2400
tttaggagtt ggaattagtt tgggtaatat agtgaggttt tgtttttata aaaaatgaaa 2460
gaattagtta ggtatagtgg tgtgtatttg tagttttagt t 2501

<210> 355
<211> 2401
<212> DNA
<213> Artificial Sequence

<220>
<223> chemically treated genomic DNA (Homo sapiens)

<400> 355

ttattaaaga aaaaaaagat gatttattag atagttgtta aggtatttat ataaaattat 60
atgatagtag gatagatggt aattaagagg gaatttagga tttgttatta ttagttggaa 120
atatttgatt tttagtttgt aaatgtgata agttagatta atattagtag gatattgaat 180
atagtttgta gggatgagtt tttgtgttat ttttggatat gtgtttaagt aattagaata 240
atttaaaatg ttattaggtt tttgttgtta tttttataaa ttttgaatag tgagttgggt 300
ttttaaggaa tttgtttttg ttagtttatt ttatgttgta taataaatag gtttaaagtt 360
tttggtagtg tatattaagt atggaggatt ttttatttat tatgatagtt tttttgttgg 420
agggttgggg ttgtatttta tttttttttt tggaaggggt ggtttttgtt tgaggtttta 480
tttgttttat agtagaggga tgaaatggta ggattttggt tttgatgggt tttatttggt 540
aatggtatat gttaagtggg ttatgtttta ttgtttagag taagttatat ggttatgttt 600
gatattgatg tgtgggaaat gtagaatttt tttgtatttg gggaggtaga tatttggata 660
atatgtaatt tattataggg ttagttggat gaagaatggt taagggttta ggttttttat 720
tttgagttag gttgaattag ttggggttgt gagattaagt tatggttatt agtgtttgtt 780
tggaattatg ttttgttttt gttgtgttta tattgtagta gggtttttgt tttttaattt 840
atttgaaatt ggtggtgggt gtggtatata gatgagaaat aggaattttt tatgttgggt 900
tttaatagga gttgtggtgg gaatataaat ggtgtagtta ttttggaaga tagtttggtg 960
gttttttata aaattaaatg tattttttat atatgatttg ttatatatta tttagtaatt 1020
gtattttttg gtatttgttt agatgagtta aaaatttgta tttatataaa aatttgtatg 1080
tggatgttga tagtagtttt atttataatt gtttaaattt ggaagttttt aggaggtgag 1140

tgggtaaata aattgtggtg tttttggtta gtggaatatt atttagtgtt aaaaagaaat 1200
gagtttaagt tatggaaaga tatgaaagaa ttttaagtat tattaagtta aagaagttaa 1260
tttgaaaagg ttgatgttgt atgagtttat atttatgata ttatagaaaa ggtaaaatta 1320
taaagatagt aaaaggatta gtggttgtag gagttaggga ggagggaggg atgaataggt 1380
ggggtataga ggatttttag ggtagtgaaa ttattttgta tgatattgta atggtggata 1440
taggatagta agtatttgtt ttttattttg tagaatgtat ggtattgaga atggattttt 1500
ttgtaatata tggattttta gttataatat tgaatttttt ttggggtttt gtattgtttg 1560
tagtgttttt taatttttgt ttttgttttg tttattgttt tttttttttg ttatagtgtg 1620
tttgttttgt ggttgttaat tagttgtgtg ttttggttgt gtgttttgtt ttgtttttgg 1680
tgggtgtgtg tgtggttaat gggtggtgtg tgggggttgg gttgtggtgg ggtggggtag 1740
tggggtggtt gttaattgtt gtggtgttgt tgaaattgaa aatattatat tatgttaatt 1800
gtggttgggt ttgtgtgtat gggggtgggg tttgtgtgta taaagggggt gtaggtgggt 1860
tgggtgtttt ataggttaag tgtgttgtgt ttgaggggtg ttggttaggt ttgagtgagt 1920
gagttagtta gtaggtattg agggggtgtg gttgttgttt ggatgagata ggtgaatttg 1980
atgtagaaga gtttattatt ggatagttag gtagttgttg tgttttttgt atatgtagag 2040
ttgggtggtg tggggttttt tttgtgtttg gtttttgttt tttttttttt tttttttttt 2100
ttttttttgt tgtttttttt ttttttgttg tttgtgtttg tgtagttttg ggttatgttt 2160
gatgtgtttt tttgtagtat atttatgatg gagtgttttg ttgtttgtgg gattttttta 2220
gtattagtgt gtattagtgt ttgttgtagt ttttttgggt tggtggatta tgaggagttg 2280
agttgtgagt tgtaggtttg tttggttgag ttgaatgttg aggattagaa ttgttgggat 2340
tatgattttt agtaggatat gttgttgtgg ggttttggat gtttgtagtg gattgaagtg 2400
g 2401

<210> 356
<211> 2401
<212> DNA
<213> Artificial Sequence

<220>
<223> chemically treated genomic DNA (Homo sapiens)

<400> 356

ttattttggt ttattgtagg tgtttagggt tttgtagtgg tatgttttgt tggaagttgt 60
aattttagtg gttttggttt ttggtgttta gtttggttag gtgggtttgt agtttgtggt 120
ttagtttttt gtggtttatt ggtttgaaga ggttgtggta ggtgttggtg tgtattagta 180
ttgggaaggt tttatgggtg ataagatgtt ttattgtgga tgtgttgtgg agggatgtgt 240
tggatatggt ttggggttgt gtaaatgtgg gtagtgagag aggagaggat agtgagaaga 300
aggggaaagg agaggaggag agggtggagg ttgggtgtaa gggagatttt gtgttgtttg 360
attttgtgtg tgtgagggat gtggtggtta tttggttgtt tggtggtgga tttttttgtg 420
ttgggtttgt ttgttttgtt tggatggtag ttgtgttttt ttgatgtttg ttggttagtt 480
tgtttgttta ggtttggttg gtattttttg agtatagtgt atttggtttg tggaatgttt 540
agtttgtttg tgtttttttt atatgtgtgg gttttatttt tgtgtgtgtg ggtttggttg 600
tgattagtat aatgtagtat ttttagtttt aataatatta tggtgattgg tggttgtttt 660
gttgttttgt tttgttgtgg tttggttttt gtgtattgtt tattggttgt gtgtatattt 720
attgggggtg gggtggggtg tgtggttggg gtgtgtggtt gattggtggt tgtggggtga 780
gtgtgttgta gtaggggaag ggggtggtga gtggggtggg ggtgggagtt aaagggtgtt 840
gtgggtggta tggaattttg aaagggattt aatattataa ttgaaagttt atatgttata 900
ggagggtttg tttttagtgt tgtatatttt atgggataaa ggataaatgt ttattgtttt 960
gtgtttatta ttatagtatt atataaggta gttttattgt tttaaaaatt ttttgtgttt 1020
tatttattta tttttttttt ttttttagtt tttgtaatta ttgatttttt tattgttttt 1080
gtagttttgt tttttttata atgttataga tgtgaattta tatagtatta gtttttttag 1140
attggttttt ttgatttaat aatatttaag gtttttttat gtttttttat gatttgagtt 1200
tatttttttt tagtattgaa taatattttg ttgattggag gtattatagt ttgtttattt 1260
atttattttt tgaaggtttt taggtttggg taattatgaa taaagttgtt attaatattt 1320
atgtgtaagt ttttgtgtgg atgtaagttt ttgatttatt tgggtaaata ttaaggggta 1380
taattgttgg atggtgtgtg gtggattata tgtgaagggt atatttagtt ttgtaaggag 1440
ttattaaatt attttttaaa gtggttgtat tatttgtgtt tttattgtag tttttgttaa 1500
agtttagtgt ggagagtttt tgttttttat ttgtgtatta tatttattat taattttaga 1560
tgaattaaaa aatagaaatt ttattataat gtggatatgg taaggataag gtataatttt 1620
aaatgaatat tgatgattgt ggtttgattt tatggttttg gttggtttgg tttggtttgg 1680
ggtgaagagt ttgggttttt ggttgttttt tatttagttg gttttgtaat gaattgtatg 1740
ttgtttaaat atttgttttt ttaaatgtag gaagattttg tattttttgt atgttaatat 1800

tagatgtggt tatgtaattt gttttgggta gtgaaatgtg atttatttgg tatgtattat 1860
tgttaaatgg agtttgttaa agttagggtt ttgttgtttt atttttttgt tgtgagataa 1920
atgagatttt agataggggt tgtttttttt aaaagagaaa atggagtata attttagttt 1980
tttagtgaag ggattgttgt ggtgagtgag agatttttta tgtttgatgt atgttgttaa 2040
gagttttggg tttgtttgtt atgtagtata aggtaagttg atagagatag gttttttgaa 2100
gatttaattt attgtttgag gtttatggga atgatagtaa gggtttggtg gtattttgag 2160
ttgttttggt tatttggata tatgtttaga aatgatgtag agatttattt ttgtaagttg 2220
tgtttagtgt tttgttggtg ttggtttagt ttgttatatt tgtaggttgg aggttaggtg 2280
tttttagttg gtagtgatgg gttttgagtt tttttttggt tattatttat tttgttattg 2340
tgtagtttta tgtgagtgtt ttaataattg tttagtaagt tatttttttt ttttttgatg 2400
a 2401

<210> 357
<211> 1508
<212> DNA
<213> Artificial Sequence

<220>
<223> chemically treated genomic DNA (Homo sapiens)

<400> 357

attttataat tataaatatt tagtgtattt tgtaaatatg gtataatttg ttggtataaa 60
attttattgt tttagaaaat attggtaata aaattataga aaagttaaag atttttattt 120
tttattttga agtgtttggg agatatttta gaaatggatg ggaaatgtta aattttgtat 180
gtttgtttat tttttttttt atattgatta gatgtaaatg agtgttatta aaagtatatt 240
ataggtattt atatagattt tttttataag ggatttataa agtttagatg tgaaatgtat 300
tttaaagggt ttttagttgt tttttatttt tttttttgtg aaatagggaa gatatatgtg 360
tttttaaggg tagagatgga atttgggtta atgggttggg gggtgggggg aagttggaag 420
ggattgtttt aggatatggt aggatagtgg attgtttttg ttgttttgtt gtttatattg 480
gggttttttg taggtgtgtt ggtttttttt ttttttgttg agaagaagtt ttgttaaaat 540
atttgttttt tttgggaatt tttttgtggg tttaaagtgg gaaaatgttg ttttaggttt 600
aaaataattt gtttaagttt tttagtgtgg gagaaatgtt tttatttgtt ttttgttttt 660
gggggtgttt tttgtttttt gttgttagaa ttttggggag tgtttagatt tggggtagta 720
tatgtttggg ttaattgtga gtagaataaa tttttggtgg gtggttagga agtttttttt 780
tagttattgt ttttttttag tgtttttttt tttttttata taatataaga tttttttttt 840
ttagtttttt taaagtatag tttaaggaaa tttttttata gtttttattt agttatgggt 900
tagtatgttt gggggtaaat atgtagattt ggaggtaggt atttgtgggg gggtgttggt 960
ttgggtgtgt ggggaagtgt ttgtttttgt tatttgtttt tttaaatatt ttgattgttg 1020
atttggtttt ttagtttttt ttttattttg gagtattttt ttggtgttgg tattgttgaa 1080
gaatgggttt gggtggggag gtgaagagaa gttaggaatg ttttatgttt ttttaatgga 1140
gagggggttt tgggagtttt tgagttagga ggatatggaa aaggggattg gggttttgag 1200
attgggtttg ttgggtttaa gatgtgtttt ttggatgggt ttaggatgtt tttttgttgt 1260
gggaattttg tggtttggtt ttgtttgttg ggggtttgaa aaagtggatt gtagggtgga 1320
aggtgttatt tatttgagtt ttggggatag tttttgggat tttttgttaa gtgtttaaaa 1380
tggtaggttt ttaagtggtg tgtggtgttt ttgtattttt taaaattttt gaaatatttt 1440
aaaaattttt ttgtattttg gtgtttggta gggtttttta aaaaaagtaa attttttttt 1500
taaatgta 1508

<210> 358
<211> 1508
<212> DNA
<213> Artificial Sequence

<220>
<223> chemically treated genomic DNA (Homo sapiens)

<400> 358
   tgtatttggg agggaggttt attttttttg gaaaattttg ttagatgtta agatgtagaa 60
   ggatttttga gatgttttaa gaattttgga aagtgtaaaa atattgtgtg ttgtttgggg 120
   atttgttgtt ttgggtgttt ggtggagagt tttggggatt gtttttagga tttgggtaaa 180
   taatattttt tattttgtaa tttatttttt tgaattttta gtaggtaggg ttggattgtg 240
   gggtttttgt gataggggag tattttagat ttatttagaa aatgtgtttt gggtttaata 300
   gatttaattt taggatttta attttttttt ttgtgttttt ttagtttagg ggtttttgag 360
   gttttttttt tattaggaaa atataaaata tttttggttt ttttttattt ttttgtttag 420
   gtttattttt tagtggtgtt aatgttagag gagtgttttg aagtggaaag agggttgggg 480
   ggttagatta gtagttggga tatttggaga ggtagatagt agaagtggat atttttttgt 540
   atgtttgagt tggtgttttt ttatggatgt ttatttttga gtttatgtat ttgtttttag 600
   atatgttggt ttgtggttgg atgaaaattg tgaggaggtt tttttgggtt gtgttttaag 660
   ggaattgagg aaggaagatt ttgtattgta tggggggaaa aaaaggtatt ggaggggggt 720
   ggtggttggg agggagtttt ttggttgttt attaaaggtt tgttttgttt gtggttggtt 780
   tggatgtgtg ttgttttggg tttaggtatt ttttaaggtt ttggtagtag agggtgggga 840
   gtatttttgg gggtagagag tgagtaagaa tgtttttttt gtgttggggg gtttggatag 900
   attattttaa atttgaggta atattttttt gttttgaatt tatggggggg tttttggggg 960
   aggtggatgt tttggtaggg ttttttttta gtaggggggg agggagttga tgtgtttgta 1020
   gagagttttg gtatgggtaa taaggtggta gaaataattt attattttgt tatgttttaa 1080
   ggtagttttt tttaattttt ttttattttt tggtttattg gtttaagttt tatttttgtt 1140
   tttgaaaata tatgtgtttt ttttgtttta tagagggagg gatgagagat ggttaggaat 1200
   tttttagggt atattttata tttaaatttt gtggattttt tatggaagga atttgtgtgg 1260
   gtgtttgtgg tgtatttttg gtgatatttg tttatattta gttggtgtgg aaagaaaaat 1320
   aagtaggtat gtagaattta atatttttta tttgtttttg aaatgttttt taaatgtttt 1380
   gaaataagaa atgaagattt ttaatttttt tataatttta ttattgatat tttttggaat 1440
   aatggaattt tatgttaata ggttatgtta tatttgtaaa atatattaaa tgtttataat 1500
   tgtagaat 1508

<210> 359
<211> 1984
<212> DNA
<213> Artificial Sequence

<220>
<223> chemically treated genomic DNA (Homo sapiens)

<220>
<221> unsure
<222> (9, 17, 18, 33, 41, 49, 50, 52, 54, 62, 80, 84, 127, 157, 159)
<223> unknown base

<220>
<221> unsure
<222> (175, 207..209, 214, 1181)
<223> unknown base

<400> 359
   agttttagnt attattnnta tttttattaa tgnttttggg nttataggnn gngntttttt 60
   gnttaaatag tagttaatgn aggnatttta aaaggtatat tattttatat ttatttagtg 120
   gttatgntat tatttaattt ttattataat tttgtgngnt tagtataatt tgtgntttta 180
   ttttatttgt gaataaatgg aggtagnnnt ttgngtaatt tataagtaag tggtagattt 240
   gggggttgtg tttagataat gtggttttag attttttgtt tttttttgtt ttttgttgta 300
   ttttgattga gtttagttat ttattttttt aatatatatt aattgttgtt tttatgtttt 360
   aggttttgtg ttagatgtta gaaatatatt tttatttatt ggattttggt atggtttatt 420
   gtttagtgtt ttgttatgtt atgtgttata taatgtattt attttggtat ttagtttatg 480
   gaaaataatg ttgaaagata ttgtatgtat gtttttataa taaaattatg taatttatgt 540
   tttttttatt gttttggtta ttaggaagtt tttatttaaa ttagagtaaa tatattagaa 600
   tttgggtttg ttattttagt ttgttgaatt tttttttttg gtttagattt tttattttgg 660
   tttataaatt ttattgtata aatgtttttt attgtaaaat attttaaatt ttttttaagg 720
   gaaggttgta tggaaatgat atagtaaggt tttttttgta tttttttaga tttttattag 780
   ggaaggtaga tttagatgtt ttttttattt ttagttttaa agttttttat ttataaaatt 840
   ttttaagtag tgattattgt tgttttgagt atttggaggt agtttagagt ttttgaaagg 900
   taaaatttat atataaaaag aagttttttt tgatttagat tttagttttg gtgttagatg 960
   tatatttgag gagtaggtgg ttagttagat ttttatttga gtagttaata aaatttattg 1020
   ttttttaatg gaattttttt tgtaagtttg aattgatttg ttatttttgt gatttgtgag 1080
   atggtaggga agtattaaat attattatga tttgggttat agtggtggga aaaaaggaaa 1140
   aaagaaaaaa aaaatttatt gttaagtttt gttaggtgta naaagggttg gaattgttgg 1200
   ggttatttta tttgatttat tggaaataga gtggatttta ttaatatttt aataaagaga 1260
   atttttttgt attaggttgg aagtggttgt tagttttttg tgtaatttta ttttttggaa 1320
   aagtggaatt agttggtatt gtttagtgtg atttgtgagg ttgagtttta atagtttaaa 1380
   gaagtaaatg ggatgttatt tttgtggggt ttgttttttg tgaggtgttt attttgtatt 1440
   tgttatgtaa aatgagggag tgttaggaag gaatttgttt tgtaaagtta ttggttttgg 1500
   ttattagttt ttatttaatg tttttgtgat gttgttgttg atttatttgg gaagttggtt 1560
   ggttggtgag gtagagtttt tttttaaagt ttggttttta tggaaaatat gtttagtgta 1620
   gttgtgtgta tgaatgaaaa tgttgttggg tgtttttagt tggataaaat gtagttgaga 1680
   attttgtttg ttttgtgtgt ttttttgttt taggtaggga agaggggttg ttgggtgtgt 1740
   tttgtgtttt gtttttgtat ttggattgtt tggtatgggt agggtgaggg ggtttttggg 1800
   gggttggggt ttttggttgt ggtggtgaag atagattggg gtttggtagg gaggttattt 1860
   tgagtttaga gattttaggt attttttata tataggtttt tattttggtg tgtgtgtgtg 1920
   tgtgtgtgtg tgtgtgtgtg tgtgtgtgtg tgtgtgtgtg tgtgtatgtt tgttaatggg 1980
   agga 1984

<210> 360
<211> 1984
<212> DNA
<213> Artificial Sequence

<220>
<223> chemically treated genomic DNA (Homo sapiens)

<220>
<221> unsure
<222> (804, 1771, 1776..1778, 1810, 1826, 1828, 1858, 1901, 1905)
<223> unknown base

<220>
<221> unsure
<222> (1923, 1931, 1933, 1935, 1936, 1944, 1952, 1967, 1968, 1976)
<223> unknown base

<400> 360

tttttttgtt aatgaatgta tatatatata tatatatata tatatatata tatatatata 60
tatatatgta tgtatgttag agtgggagtt tgtgtgtggg gggtgtttag gatttttggg 120
tttggaatga tttttttatt gagttttgat ttgtttttgt tgttgtgatt ggaggttttg 180
attttttgaa agttttttta ttttgtttgt gttgggtgat ttgaatgtag aaatggggtg 240
tagagtgtgt ttggtagttt ttttttttta tttgggatag gagaatgtat agaatgagtg 300
gagtttttgg ttgtattttg tttgattaga agtgtttggt ggtgttttta tttatgtatg 360
tggttgtatt gagtatattt tttgtgggag ttaggttttg aggagaggtt ttgttttgtt 420
agttagttaa ttttttaaat agattagtag tagtattatg aaagtattgg gtagaggttg 480
atgattagga ttaatggttt tataagatgg attttttttt aatgtttttt tgttttgtat 540
ggtagatatg gggtgagtat tttgtgagga gtgagttttg tggaggtggt attttatttg 600
tttttttgga ttgttggggt ttagttttat aaattatgtt gggtaatgtt agttgatttt 660
atttttttag agaatggaat tgtatggggg attggtggtt atttttagtt tagtgtaaaa 720
agattttttt tattaaaatg ttaataagat ttattttatt tttaataaat tagataaaat 780
ggttttagta gttttagttt tttntatgtt tggtaaggtt tggtagtgga tttttttttt 840
tttttttttt ttttttttat tattgtggtt taagttatga tggtgtttgg tgtttttttg 900
ttattttata aattataaag atgatagatt aatttgagtt tgtagaaaaa attttattaa 960
ggggtaatag attttattaa ttgtttaggt gagagtttga ttagttattt attttttaaa 1020
tgtgtatttg gtattaaagt tgggatttgg attggaagga attttttttt gtatatggat 1080
tttatttttt agaagttttg aattattttt aggtatttag aatagtagta gttattgttt 1140
aagggatttt ataaatgggg ggttttggga ttgagggtaa gagggatatt taggtttgtt 1200
ttttttaata ggaatttaag aaaatgtagg gaagatttta ttgtattatt tttatgtagt 1260
ttttttttag aaagaattta aggtgtttta taataaagga tatttgtgta atagaattta 1320
tgaattaaaa tagaaaattt gggttagaag gaaaagttta gtaaattgaa atgataagtt 1380
taaattttaa tgtatttgtt ttgatttgga taagagtttt ttgatagtta aagtaataaa 1440
agaaatataa gttatatagt tttgttgtag aaatatgtat ataatgtttt ttagtattat 1500
tttttatggg ttaaatattg gaataaatgt attatgtaat atgtaatata gtaggatatt 1560
gagtaatgga ttatgttaag atttagtgga tggggatgta tttttagtat ttggtatagg 1620
gtttggaata tggagatagt aattagtgtg tgttaaagaa atagatggtt agatttaatt 1680

aagatgtagt agaaggtagg gaggggtaag gagtttggag ttatattgtt taggtatagt 1740
ttttggattt gttatttatt tgtaaattat ntaaannntt gtttttattt gtttatagat 1800
aaaatgaaan tataaattgt attgantnta taaggttgtg gtgaggatta agtgatgnta 1860
taattattga ataaatataa agtgatgtgt tttttaagat ntttntattg gttgttgttt 1920
ggntagaagg ntntnntttg tggntttaag gntattgata aagatgnnag tgatgnttga 1980
agtt 1984

<210> 361
<211> 17897
<212> DNA
<213> Artificial Sequence

<220>
<223> chemically treated genomic DNA (Homo sapiens)

<400> 361
   atattgtata atttttattt tatgtttaaa gtaaaggatt tattatatat tttgaatata 60
   gtttattttt tatttttatt tggaattgtt ttttttttat ggtttttttt ttttttaatt 120
   tttattgttt ataatatgat attataggtt aggtatgttt gtagataatt gggataattt 180
   atttttattt aagtaaaaat tttagatggt agttttttta tttgggatta tgaagtaggt 240
   aggatttttt tttttttttt aatagagttt aagtagtaag attgaataag atgggatgaa 300
   tttaaagaga aatatttgta tttgtttagt tttttaatta atatttattg aattttttta 360
   tggttaggtg tgtaagatgt taggagggat gagataagat gatgatgatg atgatgatga 420
   tgatgatgat ggtgatgatg atagtgagga tgagggtgaa tattttaatg agagtttttt 480
   atatgttagg gattgtttta ggtattttat atatattaat atatatattt attataatga 540
   ttttgtattg ttatgtgtat tttatatatg aggaggtaga ggaaggaaat gaaataattt 600
   gtttaagagt atagagtttg taagtggtaa agataagata gaaataattt tttaagagta 660
   gagagtggtg atttttgggg atagggaagt ggaaaagtgg ggagataaag gttgttgttt 720
   tttttaataa gttttgaaga tttatttgat taggtttaag aaaaatttga taattattat 780
   aaagtaaaag gaagaggaga tgaggtgggt ttttgggtat agatgtttgt tttggaaagt 840
   ttagggagga aggggtgaga ggaattgggt tgtagtatag gaatggggga tttgggaatt 900
   agaggggatt ggttagggtg ggttattgat tgtagtgaat gtttatttgt tgattgggtt 960
   gtagtatagg agtggggggt ttggaattaa gaggggattg gtttagggtg ggttattgat 1020
   tgtagtgaat atttatttgt tgattagttt gatttagaag aaagggagag atggatgttg 1080
   gaggagagag gggttttgga gggagtttag gattggaggt gggaagatat ttttatggat 1140
   atttttttat tttgatagat agaaaagatg atggtttgta gtagtagatt tataaattgt 1200
   gattgaaagg tttgatggtt ttttttattt gtttttttta tgtatagaga ggtggagtaa 1260
   tttgttttag gtattatagt ttataagtgg tgaagttgga gttgggggtt atagattatt 1320
   gggttttttt ttatgaattt ttgttgtatt ttggtttttt ttaattttga gtatttatga 1380
   gttttaagaa agatagttgg gaataggtga tttttatggt tatttttgga atatttggtt 1440
   agaatatttt agtataaaat ttaagttgtt gttaagtaga aatttttagt ggatattagt 1500
   ttagaaaatt taattttatt ttgtgatgga tttgtagtgt gtgtgtattt attaagtaaa 1560
   tttgatttta ttgttgatta gtgagaaaga ggaataatgt aaatagttag tgtaattgta 1620
   tttatattta gttttgtgag tttttgttat tttagttggt tgtggtgggg gatgtggagg 1680
   tgtatgtagt ttttaggttt tagtgagggg tttttgtata gggtgggtgt tttttatgtt 1740
   ttatgtgtat ttaggtttgt attaaagata gttttttttt tgtagtgtga tatttagtta 1800
   agaaaattat tttatttggt gtttagtgga agaaaatatt ttttttaata gggaaggaaa 1860
   attggttgtg ttgggttgag ggaggggagt gtggaattta aatggtgttt tttttaggtt 1920
   ttttttaagg tgatgtggat tttgatttta gaatgttatt atttggaaaa ttgtgtttta 1980
   ttgtttaaat ttttgttttt tttttttgtt tttttttttt tattttattt tttttttttt 2040
   gttttttgat ttatttaatg ttaatggttt tttagaaggt taggtttttt atataagttt 2100
   agttttttgt taggtaattt ttttggggtt ttaagttgta aatttgggtt gttttttgta 2160
   ttttgttttg aggatttttt taaagagata ggtaagggtt ttttttggta aaatgttttt 2220
   ttaattttat gtattagttt tatttgttat tgaagaagtg ggttgttttt tttgtgtttt 2280
   tgaatttgat attttttatt ttgatatttt gtatttttga atttgatatt tttgtatttt 2340
   tagtaggtaa aaaattaaag gtgtttattt aataaattgt tgtaatgggg agtttatggg 2400
   aaaatgggta tgtgtaagtt ggttaaagga taataggtat ataggttgtt tgatttggga 2460
   gggtttttag atagtaggat ggtttttaga gatgtatttt tgttgttgtt gttgtttatg 2520
   attatgtttt tagggtttag aatggtgttt gggtatagta ggtttttaat atagttaggg 2580
   aatgaatgag tttattttaa ttatgttgta tggatggaga aattgtgttt tggggttgta 2640
   ttatgttatg tatttgtaga aaatgttttt gtttttttag ttttttttta gtggtggagt 2700
   ataggattta gtttataaga gtagtttaat tttgttattt gtggaatgat agaataaata 2760
   agttttaggg tttagggagg taaggttttt gttttagttt tttaagtagt tggaattata 2820
   gttgtatgtt attaagttta tttgattttt tatttttttg tagagatgat gttttattat 2880
   gttgtttaga tttttttgga atttttgggt ttaagtaatt tttttatttt tgtttttgaa 2940
   gtagttggga ttataggtat ttgttattag gtttggttag tttttatttt ttttgtagag 3000
   atgggatatt attatgttgt gaaggttggt tttaaatttt tggatttaag agaggttttt 3060
   gtattggttt tttaaagtgt tgggattata ggtgtaagtt attttatttg gatataatat 3120
   tttatttgaa ataagaaaaa ttttttttta ttgggaatgt tgtagttgaa ttagtttaaa 3180
   tttataaaga atggaagatt gtttagtggt tatttattta tttattaatt ttagatgtta 3240
   aattttttag ggtgggggaa tgtgttttta tatttaaggt atttttgaat gtagttaaat 3300
   tttgtagttt agttaaattt tatagttaat agttaaattt tgtagtttag ttaaattttt 3360
   ttagtagtaa aatttatgat attggaattt tgttttttat attggttatt tgtaatagaa 3420
   taatagagtt ttagtattag ataggttttg aaagattatt gaatttaatt tttttttttt 3480
   gttagtgaga attttgaagt ttagatttgt tgatttgtga ttgatagtgt taaagatagg 3540
   tttgaaatat agatgttttg ggttttagag gtgttgtttg ttttttttta tatttttttt 3600
   gttttagaaa attttttttt aaaattggtt ttaataatta gaggggaaag ttatggtttt 3660
   tgttttgggg atagtatggg ttggtataga aaagaggttt ataatttagt aggaagtgtt 3720
   gtgtgtgtgt gtgtgtgtgt ttgtggaggt gtagggaggg ttatttgagt ttgttttggg 3780
   tttggttttg ggtttagtgg taaatttaat gttgggtagg tggtttgagg ataatgatgg 3840
   tgttagttgt gagttagatt ttttttattt ataaaattat gtttttttat gtagtgtttt 3900
   ttaatttggt tgtatgttag gagttatttg ggtagttttg taaaaatttt aatgtttagt 3960
   tggtttgtat tttttgagat tttgattaaa ttgttttgga gtttattttt ttttttttat 4020
   tatttattta tttatttatt ttttaatttg gtggtttttt tgggattttt tatatgtagt 4080
   aggggttgag aattgttggt ttttttggtt agatgggaat atgtaggtta atagttatgt 4140
   ataaatagag tagtagagta ggatggtgtt aaggttggaa agggtgagtt atgaaagttt 4200
   aggaaattag aataggaagg ggtgagaatt tttttatttg ggtttgtggg gtaggagaga 4260
   agagtttagt gggtaaggtt tagtgagtgt gtttagaatt gggaagatga ggggaagggt 4320
   aagtgttggg tggtggtggg taagatggat gagattaaat gggagtgagg attttagtga 4380
   gagttttggt gatatggaag gttgagaatt ggagggagtt tgttgggtga aagtgaaggt 4440
   ggttgttggg taggtagttg aatttggttg gagagttgag ttggtgagat ttataggaag 4500
   tgaagggagt tgtttagggt gtggtgtaat gagattttta gaagaaattt tgaggtagag 4560
   atggggggtt tttgtttata tggagatata aggaagttta tatgtatatg tatgagtgtg 4620
   tatatgaata tgtataagta tataaatgtt ttttttgggt agggtatatg tgtttgttgt 4680
   ataggttgag gttttggatt tggaggggat tgtagagttg atttataatt tgggttttga 4740
   tgtttttttt ggttgtgttt ttatgattgt ttgtgtaata ggtggtggtt ttagtgggtg 4800
   tttgttgtgt gttgttggag tttagtttag tttggtgtgt ttgggaggag ggagtaggga 4860
   gtggggaagg ggtgtgtttt ggttgtgtgt tgggattgtg agggtttggg aggggtgagg 4920
   tgtgggggtg gggttgtggt gtttataaag ttgttttttg tttggatgta aataaatttt 4980
   gtttggtttt tagttggtgt tgaagtttgt tagtttatta tttgtttttg gtttttgtgg 5040
   ggtgttgggt tgttagtttt ggtattgttt tttttttttt tttttgtgtt aggtaagttt 5100
   gtgttttttt tttttaggtg tgatgtggag tagaaagggg ttgtttgtgt ggtgttgttg 5160
   gagtgttagg attgtttttt gtagttttgt gtattttagg agtaagtgtt ttggagttgt 5220
   aagtgtgtgg ttattttggg gtgtgggtgt gagggggttt tgggtgtaga tattgttgaa 5280
   gtggggggtt taattatgga aagggaggaa aaaaagatag ggttttattt attttttttt 5340
   tagttgggga ttggggtttt ggaggttggg aagggggtga tgagatggtt tgaaaaaata 5400
   ttaggatagt gttttttggt gttgtttttg aattttagtt ggtgttgttg gtaggggtgt 5460
   gtgttggatg tgggtgtaga aggattaggg tgagtgtgtg tgtgtgtgtg tgtgtgtgtg 5520
   tgtgtgtgtg tttgtgtttg ggaatggaga gattgtgtgt gagtgtgtag atgaggggtt 5580
   ggtagaggtg agtggtgttg gtaaggaggt tggagagggg ggtgtgggtg tgtttggttt 5640
   gtagattggg ttgggaatgg gtgtggattt gagaggttgg tggtgtttta gttttgtagt 5700
   atatataggg tggtgtgtat gtgtgagtgt aagtgtgtgt gtggggggag ggtttagggt 5760
   gtgttatagt gtaatatgtg agttggagta agtgtttgtg agtgttattg ggtagggtgt 5820
   gtttttggag gaatttggga ttggtgtatt ggtggtgtgg gagaagtgat ttgggtgaga 5880
   ggggttttgt ggatatttgg tggttttttg agttggttgg agttaggagt ggtgttattt 5940
   ttgtttttgt ttattttttt ttgttttttt tttttttttt gtttttggtg ttggttgttt 6000
   ttgtttttta tgggggaaaa tagttggaat tattttgatg tttttggggg atttgttgtg 6060
   gtttttgatt tagttttggg aggagatttt gggttgggtt tggggaatgt ggttggtttt 6120
   gggggaaata tgttgttatg tttttgttaa attagtagtt ggagttggga tttagtgggt 6180
   tggtttttta tttatttttt ttttatattt tttgtttgtt gtttttgttg tttttggggg 6240
   ttttgaatag tatgtttttg tggtttggaa gtgggagatg tttggtagtg ggtgggggag 6300
   gtagggtttt tggttatttt atgtttattt aagaatattt tttttttatg gatgttgggg 6360
   gtattttagt ttgtttggtt tttttaagtt attgggaaat atttaatatt tagaagttag 6420
   ttagtggtta ggtaggaggg tggtagatat ttttataata tttattagtt gtatgggatt 6480
   tttttttaag ttgttttaaa agaatatatt taaatataaa ttttttgttt ttgttgtaga 6540
   aatgtagttt taaggtgaat tttttagttt agattttttt tgttgagaaa ttttggtgtt 6600
   tttattgagt tttataaatg tgattgaaat aagtaaggta tagagggggt aaattttagt 6660
   gttttttttg gaagggttgt tttttggtgg ggaagatata tggttggaga tgtagattgg 6720
   ttttgtgtgg tgtgtgtggt ggtagagtta gttttaggtt ggggtggtat agtgttgggg 6780
   atgaaggaag gtgtaaggga gagagagggt tttggtgtgg atggaaaatt gggtgttttt 6840
   ttgatttgtg attttttagt tttgtgggat tgtgtttttt attgtatttg ggtaaggagg 6900
   aggatttgtt tgttttgttt tgtaatttgg tttttggttt agtgtttggt aggtgggagg 6960
   agttgaaata tgtatatggg atgaagtgga tggttttggg ttttttgttt tatttttttg 7020
   tgaattagta ggttgagttt tgggtgaggt aatagattgt gtatgggtgg aggtgttggt 7080
   ttttgttttt gagggatttt ggagtgtttg tttgggtttt ttgtgaggaa gtttttttgg 7140
   agtaataaag tttgattatt tggaatggaa taaaaaattt atataggatt tatttagttt 7200
   aatttttttt tattttatag atggaaaatg aggtataggg atggggaggg atgtgtttag 7260
   gttgaattga gttggagttt agttttgtga gaggtttttg ggtgggattg aaggtagtgg 7320
   aaagggtttt tggtaggatt ttgtttgttt gttatggagg ggtgaaagga aattgtattt 7380
   ttattgaatt tttggtgagg aaatttttga ggttaaaggg gtagagtgga atgatttttt 7440
   ttaagtgtaa attaggtgtt tttgagagat tagtaaagtt agtttgaagg gtaggggttt 7500
   agaggttgta gttttttgag ttggtttttg attgaatggg gttgtttttt ttttatgata 7560
   gaagatagat tttaattgag ttgagggttt ttttgggtta tgttgaaatt tgttggtttg 7620
   aatttgtttt tttatggatt tgaaagaaag atagtattgt gttttttggg tggtttaaag 7680
   ttgtagtttt tttttgggat ttaattttag agaattttat tttagatgtg atttagaggt 7740
   tttgttttgg gagagaaagt gtgatgattt agtatttgag atttaagtag ggtttaggtt 7800
   tgatttagtt tggaaggaag gttagggagg gatatgatat tttttttttt ttagagtagt 7860
   tattgttttt ttttatttgt ttttattttt gtaagaatgt tgtttggatt gtattgtttt 7920
   attgaatgga tgaggtaatt gaggttagga gagttttagg atttgtttgt tgttatatgg 7980
   tttaggtggg agagttagat tttgaatttg gatagttgga ttttagagtt tttgtttttt 8040
   ttttatgata atatgttggg gtgtttgaag aaattaggtt taaatgaaaa ggtagttagg 8100
   agttttatgt tttagtttgt ttttttgtaa aatgagagta atagatatga tgttattaga 8160
   atgtgtaaat attaatttgt gtaatttgag aatagtattg agtatgtgga aaatagtgtg 8220
   taggtgtttg ttgttagtag tatttgtaga agatattttt aaggtatttt tttggtaggt 8280
   agagtttggg attttttgtt ttaaatttat ttaaggttgt gaaagtgttt gtaaatgttt 8340
   gatttaggta ttgttttttg ttaagtttat gtaggttgta taatagtgtt atgaaaattt 8400
   tgtttgaaat gtatattttt aggtttggtt ttttgtgatt ttttttttat taggggatat 8460
   gttgttgttt taaagttttt ttggttgggt tgggaatggg gtattttgta ggtagataga 8520
   gttgagaatt tttgttttgg aatgataggg gagagaagaa ggaaggtgta gttatatatg 8580
   ttgttgtatt tttaggttat tttgtgttta tagtgttagg gaattagtga gtgtttagta 8640
   aatagttttt gtttgttata gtgttttgga ggtttaatgt attatattag ttaattgagg 8700
   tagaagatat tttttttatt ttttaggtta gaaaattgag gtttaggttg gtgatttttt 8760
   tgtttagagt ttagtgagtg gggttttgtt taaggtgagt tttttgagtt ttttttattt 8820
   tttaatattg tgtttggagg ttatttatat tttttgggtt tattttttga gaaggagttt 8880
   gttttttgga tttattttag gttgttgttt ttgttttttt tgatggttgt gtgatggaag 8940
   tattgtgtgt ttatttgttt tttatttttt aatttggaga tagaggtagg ttagatagtg 9000
   gttttggttg agaatttagt ttaggtaggt aggtagaagg gttggggata ttgaggtttt 9060
   ataggaaggt ggttgtagag gtagagggtt atgagtaggt gttaatttgg agttgtgttt 9120
   ttggggtggg ggttatgtga aaagaaattt gggttttttt gggtggggtg taagttttag 9180
   tattatagaa tgggaatgga ttatataatt ggggttataa gtaggttttt ttgatttaga 9240
   tttttgattt ttttatttaa tattgtttgt agatttgttt atgttttatt tgtttgggtt 9300
   ttatttttga ttttttgagt ttagtgggtt tttttgtaat tgagttggta gaaagtgagg 9360
   ttttttaata gtttgggggt tttgagtgtt ttttttagta gtttaatttt tttttttttt 9420
   aaatttgtgg ggagattttt tgtagttata tattttaaat ttgtatagag ttttgtttga 9480
   agagaggtgg gtgggggttt aggtttttat tttgtttttt tgtttttatt ttttgtttta 9540
   tttttatgga atttaagaag attttaagta atttttttta aatatggatg tgagagggtt 9600
   atttaagagg tgggtttatg gtttttttta gttgggaatg tttattattt tttttttttt 9660
   ttttttaatt atggaatttt tttttttttt ttagaaaggt ttttttggtt gttttttgtt 9720
   ggaggggagg ttgggttggg ttttttttgg gatttgttta tgttttttat gaaatttgag 9780
   agtttgtttt tagtaggtgt tgtgttagtg gtggtggaaa ggatattttt attgattaag 9840
   gaagtaattt ggattatttt gtatagataa gattggaggt ttattttgga gggtggaaat 9900
   tgtaagatta ttttggggaa ggattttgtt agtgtggtta gtattttatt ttgggataaa 9960
   gggttttgta tgaggaagtt atttttttgt tttttgttaa tattggtttt gtttgtgtga 10020
   tttttataaa ttattttaga ttatttagtg tagtttatgt ttgttttgta attttataaa 10080
   ataatttttt aaggagatta gtgttttttt ttggattttt agttatttat tttgtttttt 10140
   tagatggttg ttttttgttt gttaaataaa ttatttgtgt tgttaaaatt ttattgagaa 10200
   tttttaggaa gattttggtt gtattgtaga aaagaggtgg gttttgatga gatttttgat 10260
   attttttttt ttttgttgtt gtgtttggga tatttttgtt gatttttttg gtgttgtgtt 10320
   taagttgaat atatagtagt ttagttttag ttttagtttt tttggattta tatttaggga 10380
   gtgaatgtgg ttttggtttg tttgagaggg agatagtggg gtttattttt ttagtgattt 10440
   tttttttatt ttttgaatat taaatattat tgatgttttt tgggttttag gttttattgt 10500
   aaagtatagg ggtttttgag gagtttttat tgtggttttg ttttttgata tttgtattgt 10560
   ttttttgatt ttgattgttt tgatatagtt ttttaatttt taaattaggg aatttttttg 10620
   aaggtttagt gaatttattt atattgtttg ttgagttgat tagttaagaa agatagtttg 10680
   tgaagattgt taattttttt atttttttta gggttttgag ttttagaagg gttagaaaat 10740
   attaagagag tttatgaatt gttttgtagt ttgattttaa aatttttttg ggtaaagaaa 10800
   tgataaagga ttgtatttat agtaaggtag ggttatttag ggtttatttt aaggtggttt 10860
   tttttgaggt ttaggtagaa atggtgttgt ttttggtaat tttttgggga agttgttata 10920
   gtaaatagtt atagtatatg ttgatttttt tttttttttt aaaaatttgt tttataaaga 10980
   gtataatgtg ttggagagaa tattggtttt tatgtggttt taaaattagg tatgttatag 11040
   agggtggaat ttgggttagt tgttgttggt gataattgat gttatttttg ttagtaattt 11100
   ttgtttgttt tttttttatt tgttttattg aaggttttta ggggattgtt gtttgttttt 11160
   ttttgaggga atgagtgtta tatttgtatt tataataaat tagtgagttt tttatttttt 11220
   ttttagtagg gaggaattta gatatgtggt ttttggaagt taagtgggtt tttgtgtttg 11280
   tttttgagtt ttaggttatt tatttgtgaa ttgggtataa tatttagttt tttttggggt 11340
   aatttttttg aggagtaagt gaaatagtgt gttgagtgaa tgttttttta aagtattgag 11400
   ttggttgttt taggaaggta ttatggggga gttgaaaata gagaattttt tgggtgttgt 11460
   gaagggagaa aagtttaaga tgttggtttt gttaaaagtt taagtttgtt tatgatggtt 11520
   tagtggtgat tgtagggtgg aggagaggta tgtgattgtt ttagttgatt tttatatgtt 11580
   gaaagtttta tggttatgtg tagtttattt tataatgttg tatttgggtt atgggtggtg 11640
   gtgaggattg tggatatatt tagggtaatg tgagattatt gttaatattt tttttaaggt 11700
   tgttttaatt ttaaagtagt tagtttgatg tttgttgatt tggggtatat attttaattt 11760
   tttgatgttt tagttttttt gtttgttaaa tggtgatatg ttggtatttt ttttttaggg 11820
   tttttgtgag gtttaaagga agtattgggt ttagagttta gttagtgtta tagatattgt 11880
   ttttggtgtt gtttttgtgg tgtgtttgtg tagggttata ttgttgtttt tttgtggttg 11940
   ttttttttgg tgggtggaat ttggaatttt tagaatagat tatggatgtt tttttgttgg 12000
   atattttttt agttttattt tgtgggagtt gtggtggggt ttaggagagt ttgtttaaga 12060
   gggtgtgtag gtgggtggta gagttggagt aaggtattag ttttaataga taggagtttg 12120
   aggttggtag aagtgggggt tttggtttaa ggtgtggggt taatttggtt agagttgagt 12180
   ttggtagggt tttggtaaga ttttgtggtt tttttgtttt ggtgaagtat ttttttgttt 12240
   gggtttttat ttgtttgtaa gtttgttgtg gatagtgtga tttttataga tgtatattgt 12300
   gtttatttta tagatggaga aattaaggtt tagagaggaa gtgatttgtt tagagttatg 12360
   gaataggaaa gtggtagggt ttttatgttt tgatttttag gttagtgttt ttgttgtttt 12420
   attgaggttt ttgggaatta taggttttgt gtgttttttt tagtttttta ggggttgttg 12480
   aatagttttt aagtggtgtt ggattattga agatgttttt gtttattgtt tttttaggtt 12540
   ttagggttat gggttatgtt tttttttttt ttgggtagat tttggttttg gttttgtttg 12600
   gtatttgtga tttagttttt ttttgatttt tttgggtttg ttggattgga agggtatttg 12660
   tgtagttttt tttgggtttt tattatttat tgatagatag tgttggtgga gagaagggtt 12720
   ttagtggtga tgtattgggt ttgataatta gaaattttgg tggtatttgt tgagtatttt 12780
   ttatgtgttt ttttttttgt tgggatttta ggattgaaat ttaggttata tttttataga 12840
   tgttttggta tttttaaggt aattttgaaa ggaaagtatg ttgatgaatt aagtaattgt 12900
   ttgtttaatt ttttatgtta tttttgaatt taagttttat tgttgattat tgggagtagg 12960
   agttagtatg tttattgata gataagtaaa ttgaggtaaa gaggttagat agttaggtat 13020
   ggtggtatat gtttgtaatt ttagttattt aggaggttgg agtaggagga ttatttgaat 13080
   ttaggagttt gagattagtt tgggtaatat agtaagattt tgttttttta aaaaataaga 13140
   tgaataatat atttttaaaa agagatttga taaattatgt aggattatag agttaataaa 13200
   tggtgatatt atggtttgta agtttttttt tagtttttgg ttattttttg tttttttggt 13260
   attggttttt ttggaatttt ttttaggtag gtggatggga aaagaagtga agatataggt 13320
   tggtagttgg tgttgtgatt tggagttttg tttttggtgt taagttgggt tttagttgaa 13380
   ggagatttgt tggaggggtt ttttttgtgt tttttgggga gtaaagatgg ttttggagat 13440
   ggagatatgg tttgttagtt ttaagttatt ttaaaggtgg ggttggtggg ttaggagtta 13500
   gttttggttt ttttgttatg ttgtgtgtgg ttttgttttg ttattttatt tgttggagga 13560
   tttttttgat gttttttagt gggttttaat attttgtgat taggattttg tatgaggtag 13620
   agggagggta ggagttggag tttagtaagg attttaagtt tagggatagg ttttatttgt 13680
   tatatttgtt aagttgtgag ggttttaggt gtttgggagg agggagtagg agtggaagta 13740
   ttagtttgag ttgttaaatt aggttatgta ttatttgtta ttttttgttg gtagaaggag 13800
   attatagata ttagaagata gtagtttttg ttagaattgg gtttttttat gatttttagg 13860
   tggtgatttt gaattttaga aaaagtgttt tggggagagt ggagtgaagg aagagtgtga 13920
   atattggatt tgattttatt ttagtttttg tgttgtagtt agtagggtga gtttaggata 13980
   gttggtagtt gtttgggttt agtttttttt tttgtgaatg aaggtggtgg ggagtgtatg 14040
   tgtgtgtgtg agtgtgtgtg agtataaatg attgtggatg tgagagttta tgaaagtgag 14100
   aatgtattag tgtgtaagaa agtgtgagta tgtgagaatg agtgtgtttg tgagaatgta 14160
   tgtatgtgtg agtataagtg tgtgaatgtg agaatgtttg agtgtgagaa tgaaagtttg 14220
   taagtgtgtt agtgtgaggg aatgtgtgag tgtatttgag agtgtgtgtg tgtgtgtgtg 14280
   tgtgtgtgta tgtatgtatt agattaaata agaatgtatt taattgatat tttttggata 14340
   ttgttattta ttaggaatta ttaaaattag ttattttaga ggagggtgga tgttgtatta 14400
   ggggatattt ggtaatgttt gtagattttt ttgttggttg tagtgtgggg tgtatgttgt 14460
   gggtatgtgg tgggtagagg ttagggatgt tgttgagttt ttttttgtgt ttagggtagt 14520
   ttttaaggta gaggttgttt gtggggttta aatgttagta gtgttgagtt gaggattttt 14580
   gtttaggagg tatagatgtt gttttggatg ttgtttagat agtatttgtt ttgtttttta 14640
   gggggataga tagatagtgg atatagttta gatgtagttt gatgagtgag tttaagggag 14700
   tgtttgtttt tttgggattg tgagttttgt gggggtagga attgggtttg tttgtttatg 14760
   atttttagat gagttttata gttagtttta ttattatagt tggtgttatt tttgttttga 14820
   gttttgtgtt gggtatatag aatttagtgg gtagaaattt ttgaataaat tttgagtttt 14880
   ttattttgtt tttgtgaatt tagatgtttt ttgagggtta gtttggtggt gtgggggttg 14940
   ggagtgggta gattgtaggg agttaagttt tggggtggag tggggtttag gttttttttt 15000
   ttggtttgag ttgaagtatt ttggtttttt aaattttaaa atttttattt tttaatattt 15060
   ttatatgttg aattttttta tatgattttt tttggataaa agtttttgta aaaaaagttt 15120
   gaaaagtttt ttggttgagg tattagggat aaggtaaggg ttgtttgatg ggtttaattt 15180
   gggagaattg aggtgaggga gttgtttttt ttttatttta taggttggtt agatggtgga 15240
   ttgttagtag ttggttgggt taatttgtag agagtatttt ttgtagtttt tggtttatgt 15300
   ggttttagta ttgtaggatg tgattaatat gtaggttatt ttgaggtttg tagtttttat 15360
   agttttttta gattagtgta aagttttatt tagttttttg gtagatttta gagtttagga 15420
   tattttttaa atggtttaaa attaatggaa ttatttgggg gataataaat aatgttatta 15480
   gatattgtta aatgtttttt gggagagaaa atttttttta gtttagatat attttgtgat 15540
   tatttttagt agttttattt ttggattttt taaaggtttt tagtagtgag ggggttgggt 15600
   tagagaaggt tttattagtt tttaaaagta gatgtttaag aatttgatat ttttaggata 15660
   tatagtgatt tttttaattt attgttttat gtggtttggg aagttaagtt gtaggaataa 15720
   aaaaggtgtt gggtagggta aagaggtttt attagataga ggaaatgggg aaggtggata 15780
   tttttgtttt ttttgttttg gagaatgaga gagttggtgg agattgatag atattatgtt 15840
   ttaatgtgat tatggttttt ggaagttaaa gagaattgat taaattgggt tgaattatag 15900
   tattttttga gtttttttaa atgtagaatt tgttttttag atattgtatt tttggggttt 15960
   agtaatgatt taaaagtgaa ttttattgaa agtatgttgt tgagttatat tgtttattta 16020
   tttagagagg gagttgtagt ttgatttttt gtttaggaga gttttttgtg ttgggagtta 16080
   gaggtagaga aaagaataag ttatttgttt gatgaagttt atagatagga ttgtttgaat 16140
   ttatttggtg ggatagtgag agttttttga ttttattttg gtgtgttttg ttgagtaggg 16200
   tatgttaaga agtttttgtg aaatgagaag tttttttatt tttagaatga ggggtggtgt 16260
   tggtagggtt ttgggttttt ggaaggtttt ttaggttatg taggtgaggt gttttatagg 16320
   ttagggtttt gggattatga atttgttgaa ttagtaaggt taatagatgt gggtttttta 16380
   tgttttttgt ttttaagttg gtagttgttt aggaatgttt ataaatataa aattgatttt 16440
   gtttttagaa tgttttggag tatagatttt aaagtttgta taaagttatt tttgtggggt 16500
   tggttattta ttggggaaaa ttatgttggg gttttagatt agagtatttt agtggagagt 16560
   aggatttgtt tatggaatgt tatgtgttgg tataagggat ggatttgggg tggatttggg 16620
   gtggtttgta tttgttggtt gatttttaag aggtgtttta ttaggttttt gggagttttt 16680
   gggtttttag aggagtttta ggtaggaatt ttagaggttt gtattttttt gattattgta 16740
   tttgaggggt tttgggtagt gtggggttta ggtttttatt ttgaagttgg ttgtgtgagg 16800
   gttttggtta ggtgtttttt atttagaaag tttggatttg tgaattgggt gggtggtttg 16860
   ggtttttttt ttttgttatt tatttttttt ttgttggttt tttgttgttg tttgtaagat 16920
   ttgattttat tttgtatttt ttatgtttta ttttagggat tgatgggtat ttagtttttt 16980
   taatttgatt attgatttag aagaatatat gtttatattt ttggttttta gatttagttt 17040
   ttggaggtat gtttaataga tagtattatt tgtttaagtt ttgtatttta gggagttagt 17100
   ggtagtattt taagtttttt tgggttgtgt tttagtttgg ttttgtagtt tatatttttg 17160
   tatttgtttt gtgttagagt ataggtaatt tttttggttt attttttgtt ttgatttagg 17220
   aagtgttttt tgttttggag gttttttttt ttataaaatt gtattgtgtg tttaaatgtt 17280
   tgttaaattt tttgatttaa ggtttttttt tgtttttttt tttttttagt aggtagttga 17340
   gagttttgta ttgtggggtg tgtatgtgtg tttatgtgta tgtgtgtgtt tgtgtgtttg 17400
   tgtgtttgta ttattaattt tttgttaggg ttaattgtga ggttgaagtt tgtattatgt 17460
   ttatatgtgg taagtattgt gtgagtttgt gttttttttt tattgttata tgggggttgt 17520
   ttgattttgg gattagtaat gtaggttttt ttttagaatg tagtgagttg tattttgggt 17580
   atggtgggtg ggtttgaatt tggtatgggg atttgtttgt gaggttgatt tatttatttt 17640
   attgggaggg tggtgtttgt gagttttagt attttttttt aggaaattta ttgtgttggt 17700
   tttaatggga agattgggtt gggtttatgg gttgtgtgta taagggattt ttttattttt 17760
   tgtagtgtat tatgagggag gtgaagttgt tgggtagagt gtaggtttgg tttttttgtt 17820
   attggttttg gatttgtttt ttttaggaaa ttttttagta ggaagtgttg attttttggt 17880
   ttttgttttt ttgtagg 17897

<210> 362
<211> 17897
<212> DNA
<213> Artificial Sequence

<220>
<223> chemically treated genomic DNA (Homo sapiens)

<400> 362

tttgtaagga gataaaggtt aaaaggttaa tattttttgt tgagaggttt tttaaaggaa 60
gtaaatttaa gattaatggt aagaaggtta ggtttgtgtt ttgtttagta gttttatttt 120
ttttgtgatg tattgtaggg gatgaggagg ttttttgtat atatagttta tggatttaat 180
ttagtttttt tattggggtt agtgtagtgg attttttggg agaggatatt gaggtttata 240
gatattattt ttttaatgaa atggatagat tagttttata aataggtttt tatattaagt 300
ttaggtttat ttattatatt taggatgtag tttattgtat tttagaaggg gatttgtatt 360
gttgatttta aagttaggta gtttttgtgt gataatgaaa ggggaatata agtttatatg 420
atgtttatta tgtataggta tagtgtaagt tttagtttta tagttagttt taatagaagg 480
ttggtgatgt agatgtatag atatataggt atatgtatgt gtgtgaatat atatatatat 540
tttatggtgt aggatttttg gttgtttgtt aaaagggaag aggggtagag gaaaatttta 600
agttaaggag tttggtaggt atttgaatgt atagtataat tttgtaagaa aaaagatttt 660
taaggtagaa agtatttttt ggattaaagt aggggatgga ttagaggggt tatttgtgtt 720
ttaatatagg gtaggtgtag aagtgtgggt tgtaaaatta gattgaagta tagtttagga 780
agatttggaa tgttgttatt gattttttgg ggtgtaaggt ttgggtaggt ggtgttattt 840
attaagtatg tttttaagag ttgaatttag aaattagaag tataagtatg tattttttta 900
gattagtagt taagttagaa aaattaaatg tttattaatt tttggggtga agtataaaaa 960
atgtagaata aggttaagtt ttatagatag tagtagagga ttggtggaga agaagtaagt 1020
gataagaggg aagagtttag gttatttatt tagtttatag atttagattt tttgaatgga 1080
aaatgtttga ttaaagtttt tatatagtta gttttagagt gggaatttgg gttttgtatt 1140
gtttaaggtt ttttggatgt aatggttgag agaatgtaag tttttgaaat ttttgtttag 1200
ggtttttttg aagatttagg aatttttaag aatttggtga aatatttttt agaggttagt 1260
tggtaagtgt gggttatttt gaatttattt tgggtttatt ttttgtgtta atatatggta 1320
ttttgtgggt agattttgtt ttttattggg gtgttttggt ttggggtttt agtatgattt 1380
tttttagtag ataattagtt ttataagaat ggttttatat agattttgga gtttatgttt 1440
tagaatattt tgaaaataaa gttggtttta tatttgtgag tatttttggg tagttgttag 1500
tttggaggta ggaggtatga aaggtttgtg tttgttagtt ttgttaattt agtaggtttg 1560
tggttttaag attttggttt gtgggatatt ttgtttatat ggtttaggga gttttttaga 1620
aatttagggt tttgttaatg ttatttttta ttttgggagt gaaaggattt tttattttat 1680
aggagttttt tagtatattt tgtttagtaa agtgtgttag ggtgaagtta gggggttttt 1740
attgttttat taggtgggtt taagtagttt tatttgtgag ttttattaag taggtagttt 1800
gttttttttt ttatttttag tttttagtat gagagatttt tttaggtaga aagttaggtt 1860
gtaatttttt ttttgaataa atgagtaatg tagtttaata gtatgttttt agtaaaattt 1920
atttttaaat tattgttaaa ttttaagagt gtaatgtttg gaaaataaat tttatattta 1980
aaaggatttg agaaatgttg tggtttagtt tagtttggtt agtttttttt ggtttttaga 2040
aattgtgatt atattaggat atagtgtttg ttagttttta ttagtttttt tgttttttag 2100
aatagaaaag atagaaatgt ttattttttt tgtttttttt atttggtgaa atttttttat 2160
tttatttggt atttttttta tttttatagt ttagtttttt agattatatg aggtaatggg 2220
ttaaaaaaat tgttgtgtgt tttagaaata ttaagttttt gagtatttgt ttttgggagt 2280
tggtggggtt ttttttgatt tagttttttt attgttaaag gtttttggga agtttagaga 2340
taaaattgtt gaggatgatt atggggtata tttaaattag gggggatttt tttttttagg 2400
ggatatttgg taatgtttag tgatattatt tattgttttt taaatgattt tattgatttt 2460
gaattattta ggaagtgttt tagattttga agtttgttgg gagattggat aaagttttgt 2520
attagtttgg ggggattgtg ggggttgtag gttttggggt agtttgtatg ttggttgtgt 2580
tttgtaatat taaggttgta tgaattaagg attgtagaag gtgttttttg taggttgatt 2640
tagttagttg ttggtggttt attatttggt tagtttgtag ggtgggagga gagtggtttt 2700
tttattttag tttttttgag ttgggtttat taagtagttt ttattttgtt tttgatgttt 2760
taattaaaaa attttttaaa ttttttttgt aggagttttt atttaaagga aattatgtaa 2820
ggaagtttaa tatataaaaa tgttaaaaaa taaaaatttt aaaatttaaa aagttaaggt 2880
gttttggttt aagttaggga aaggggtttg aattttattt tattttagga tttggttttt 2940
tataatttgt ttatttttag tttttatgtt attaggttgg tttttaaaga atatttgagt 3000
ttatggaagt aggatggaaa atttaggatt tatttagagg tttttattta ttaggtttta 3060

tgtgtttaat atagagttta gaataaaaat aatattagtt atggtgatga gattggttat 3120
gggatttatt tggggattat gagtaaatag atttagtttt tgtttttatg gagtttatag 3180
ttttgagggg ataggtattt ttttgggttt atttattagg ttgtatttgg gttgtgttta 3240
ttgtttgttt gtttttttgg agggtagagt agatgttgtt tggatagtat ttaggatagt 3300
gtttgtgttt tttggataga gatttttagt ttagtattgt tgatatttgg gttttataag 3360
tagtttttgt tttaggggtt gttttgagta tgggaggagg tttagtagta tttttggttt 3420
ttgtttatta tatgtttata gtatatattt tatgttgtga ttagtaaaag aatttgtaaa 3480
tgttgttaaa tgttttttgg tgtgatattt attttttttt ggaatagttg attttagtaa 3540
tttttagtag gtaatagtat ttagaaaatg ttagttggat gtatttttat ttaatttagt 3600
gtatgtgtgt atatatgtat atatatatat atatattttt aaatgtattt atatattttt 3660
ttatattgat atatttgtaa attttatttt ttatatttag atatttttat atttatatat 3720
ttatatttat atatatatat atttttataa atatatttat ttttatatat ttatattttt 3780
ttatatattg atatattttt atttttatgg atttttatat ttatagttat ttatatttat 3840
atgtatttat atgtatatgt gtatttttta ttatttttat ttatggagga gggggttggg 3900
tttaagtggt tgttagttgt tttgagttta ttttgttggt tgtagtatag gggttggagt 3960
ggagttagat ttaatgttta tatttttttt ttattttatt ttttttaaaa tatttttttt 4020
gggatttaaa gttattattt gagaattatg gaaggattta gttttggtag aagttgttgt 4080
tttttggtgt ttgtgatttt tttttgttag taggaaatgg taggtgatat gtgatttggt 4140
ttggtggttt gagttggtgt ttttattttt gttttttttt tttgggtatt tgaggttttt 4200
atggtttagt aagtgtggta ggtggagttt gtttttgggt ttgaggtttt tgttggattt 4260
tagtttttgt tttttttttg ttttatgtaa agttttaatt atagaatgtt gggatttatt 4320
agaaagtatt aaagaggttt tttagtaggt ggggtggtaa ggtagggtta tatatagtat 4380
ggtgggggaa ttagggttga tttttagttt attagtttta tttttggagt ggtttgggat 4440
tgataaattg tgtttttatt tttaaggtta tttttgtttt ttaagaagta tagaagaaat 4500
ttttttagta ggtttttttt agttaaaatt tagtttagta ttaaaaatag ggttttaggt 4560
tatagtatta gttgttagtt tgtattttta tttttttttt tatttatttg tttgggagga 4620
attttgagaa gattaatgtt agagaagtga aaggtagtta ggagttgggg gaaggtttgt 4680
aggttatggt gttattattt attagttttg tgattttgta taatttattg aatttttttt 4740
taaaaatgta ttatttattt tgttttttag agagataggg ttttgttatg ttgtttagat 4800
tggttttaaa tttttgggtt taagtgattt ttttattttg gttttttgag tagttgagat 4860
tataggtata tgttattatg tttggttatt taattttttt gttttagttt gtttgtttgt 4920
taatgggtat gttaattttt gtttttaatg attagtagtg gggtttaaat ttaagaatgg 4980
tatggagaat taagtaaata gttgtttaat ttattaatat attttttttt tagggttgtt 5040
ttggagatgt taggatattt gtgaggatgt ggtttgggtt ttagttttag agttttagtg 5100
aggagaggga tatatggaaa atgtttagta aatgttatta aggtttttga ttattaggtt 5160
tagtgtgtta ttattggggt tttttttttt attagtattg tttgttaatg agtaatgagg 5220
gtttaagagg ggttatataa atattttttt agtttagtag atttaggaga gttagaggag 5280
agttggatta taggtgttag gtaaagttaa ggttaggatt tgtttgagga agaaaagaat 5340
gtgatttgtg attttgggat ttgggagggt agtgggtaga ggtattttta gtaatttgat 5400
attatttgag aattgtttaa tagtttttgg gggattgagg gggatatata gagtttgtga 5460
tttttaaaaa ttttagtggg gtagtaggaa tgttggttta ggaattaggg tatggggatt 5520
ttgttatttt tttattttat gattttgagt aagttatttt ttttttgggt tttggttttt 5580
ttatttgtaa agtaggtata gtgtatgttt atggagatta tgttgtttat aataaattta 5640
taggtaaatg gaaatttagg taggaaggtg ttttattaag ataaaagggt tatagggttt 5700
tattaaggtt ttgttaggtt tagttttggt tagattagtt ttgtattttg gattaaggtt 5760
tttatttttg ttagttttag atttttgttt gttaagatta gtgttttgtt ttagttttgt 5820
tatttatttg tatatttttt tgagtaaatt tttttgggtt ttgttataat ttttataggg 5880
tgaggttgaa ggggtattta gtagagaaat atttatgatt tgttttagaa gttttaggtt 5940
ttgtttatta ggaagagtag ttataggaag gtaataatgt aattttatat agatatatta 6000
taaaaatagt attaggaata gtatttgtgg tattaattgg attttaaatt tggtattttt 6060
tttaagtttt atagaaattt taggagggag gtgttagtat gttattattt aatagatggg 6120
gaaattgagg tattaagagg ttaagatgtg tgttttaagt tagtaagtat taagttggtt 6180
gttttaaggt taaagtaatt tttaaaggaa tgttgataat gattttatat tgttttaaat 6240
atatttatag tttttattat tatttgtggt ttagatataa tgttgtaagg tgggttgtat 6300
gtgattatga ggtttttagt gtgtggaggt tagttgggat agttatatgt ttttttttta 6360
ttttgtagtt attattgagt tattatgagt aggtttgagt ttttggtaaa attagtattt 6420
tgaatttttt tttttttata atatttagaa ggttttttgt ttttagtttt tttatagtat 6480
ttttttaaga tggttaattt aatgttttgg aaaagtattt atttagtata ttgttttatt 6540
tgttttttaa ggaggttgtt ttgggagagg ttgggtatta tatttagttt ataaatgggt 6600
aatttgaggt ttaaaaataa atataaaggt ttatttgatt tttaaaggtt atatgtttag 6660
attttttttt attgagggaa aggtgaaaaa tttattggtt tgttgtgggt gtaggtgtgg 6720
tatttatttt tttgggggag ggtggataat agttttttgg aggtttttag tgaagtagat 6780
aaagggagag taggtgggaa ttgttgataa gggtagtatt gattgttatt aatagtaatt 6840

ggtttagatt ttattttttg taatatgttt ggttttaagg ttatatgaaa gttagtgttt 6900
tttttagtat attgtgtttt ttatagaata gatttttaaa aggagggaaa aaattaatat 6960
gtgttgtggt tgtttgttgt agtaattttt ttagaaaatt gttgagaata gtattatttt 7020
tgtttgaatt ttaaggaaaa ttattttgag ataggttttg gatagttttg ttttattgtg 7080
gatataattt tttgttattt ttttgtttaa agagattttg aagttaagtt ataaagtagt 7140
ttatgggttt ttttggtgtt ttttggtttt tttgaaattt aaggttttgg ggagggtgag 7200
gaagttggta gtttttatga attgtttttt ttaattaatt aatttagtaa ataatatggg 7260
tggatttatt ggatttttaa gaaagttttt tagtttagag gttagggaat tgtattagag 7320
taattagaat taaggaaata gtataaatgt tagagaataa aattatagta ggaatttttt 7380
gagggttttt gtattttgta gtggagtttg aaatttagga ggtattagtg atgtttgata 7440
tttaaggaat gaaaaaggga ttattaagaa ggtgaatttt attgtttttt ttttaaataa 7500
gttaaagttg tatttatttt ttgggtgtgg gtttagaggg gttggagttg aaattgaatt 7560
gttgtgtgtt taatttgagt atagtattag ggaggttagt aagagtgttt taaatataat 7620
agtaggaaga gggaggtgtt aggaattttg ttagggttta tttttttttt gtaatataat 7680
tgagattttt ttggagattt ttagtggagt tttggtagta tgagtaattt gtttggtaga 7740
tagaagatga ttgtttgagg ggatgaggtg aataattgga agtttaaagg aggatattgg 7800
tttttttaga ggattatttt gtgaaattgt aagataggta tgagttgtgt tggatggttt 7860
gagataattt gtggaaatta tataaatgga gttagtgtta gtagaggatg aggaaatggt 7920
ttttttatat agaatttttt gttttgagat gggatgttag ttatgttaat aggatttttt 7980
tttagaatgg ttttgtagtt tttatttttt ggaatgggtt tttgattttg tttatgtaag 8040
atggtttaag ttattttttt aattagtgga gatgtttttt ttattattat taatataata 8100
tttattaagg ataggttttt aggttttatg ggagatatgg ataggtttta ggaaggattt 8160
aatttagttt tttttttagt agaggatagt taaggaggtt tttttggaga aaggaggaga 8220
gttttatgat taaaaaggag gaaggagaga tggtaagtat ttttagttga gagaggttat 8280
agatttattt tttaaatggt ttttttatat ttatatttga agagggttat ttggaatttt 8340
tttaggtttt gtgggggtgg ggtagagggt gaaggtagga gaataaggtg gagatttggg 8400
tttttattta ttttttttta aatagaattt tatgtagatt taaaatgtat agttgtagaa 8460
agttttttta tagatttaaa gaaaggaaag attaaattgt tggggagggt atttaaggtt 8520
tttagattgt taaggagttt tattttttat tagtttagtt gtaagaaaat ttgttggatt 8580
taaaaggtta gagatggagt ttaggtaggt agggtatagg taggtttgta aataatgtta 8640
ggtgggaaag ttaaagattt gggttaaaga gatttgttta tggttttgat tgtgtaattt 8700
atttttattt tgtagtgttg gggtttatgt tttatttgag ggagtttagg ttttttttta 8760
tatggttttt attttgagaa tatggtttta ggttggtatt tatttatggt tttttatttt 8820
tgtagttgtt tttttgtggg gttttagtgt ttttagtttt tttgtttatt tgtttgggtt 8880
aagtttttag ttaagattgt tatttagttt gtttttattt ttaagttgaa agataaaaag 8940
taagtggata tgtggtattt ttattatata gttattaagg gaggtaggaa taatagtttg 9000
gggtggattt aggaaataag ttttttttta aaaggtgggt ttagagggtg tgggtggttt 9060
ttaagtgtaa tgttggaggg tggaaagggt ttaggaggtt tattttgggt agaattttat 9120
ttattggatt ttggatagaa gagttattag tttgagtttt aattttttga tttgagaggt 9180
ggaaagaatg ttttttattt tagttagttg atgtgatgta ttgaattttt aaggtattat 9240
agtaaataag agttatttat tgagtattta ttaatttttt ggtattgtgg atatagggtg 9300
gtttgaaggt atagtggtat gtatgattat attttttttt tttttttttt tgttatttta 9360
gagtagaggt ttttaatttt gtttgtttgt aggatatttt atttttaatt tagttaaagg 9420
agttttgaaa taatagtata ttttttgatg gagagagaat tgtaggagat tgggtttgga 9480
aatgtgtatt ttagataaga tttttatgat attattatgt aatttatata agtttagtaa 9540
aaagtaatat ttaggttaag tatttgtagg tatttttata gttttgggta ggtttaggat 9600
aaagggtttt agattttgtt tgttaagaag atgttttgag aatgtttttt gtgggtgtta 9660
ttgatggtaa atatttatat gttgtttttt atatgtttag tgttgttttt aggttatata 9720
gattaatatt tatatatttt gatggtatta tgtttgttat ttttatttta tagaaaagta 9780
aattgaggta tggagttttt gattgttttt ttatttgagt ttgatttttt taggtgtttt 9840
aatatgttgt tatggaaaaa aaataaaggt tttgaagttt aattgtttgg gtttaaagtt 9900
tggttttttt atttgagtta tatggtaata ggtaaatttt gaaatttttt tggttttagt 9960
tgttttattt atttaatgga gtaatatgat ttaggtaatg tttttgtgag gatgaaggta 10020
gatggaggga gatagtggtt gttttggagg gagagagatg ttgtgttttt ttttggtttt 10080
ttttttaggt tgagttaaat ttgaattttg tttgagtttt agatgttgag ttattatgtt 10140
ttttttttta ggataaggtt tttgagttat atttggaatg ggattttttg gggttgggtt 10200
ttaaggaaga attgtagttt tgggttattt gaaaaatata atgttatttt ttttttaggt 10260
ttatgaggag gtaagtttag gttagtggat tttaatgtaa tttagggggg tttttagttt 10320
agttgagatt tgttttttgt tatggggggg gggtagtttt atttagttaa aggttaattt 10380
agggggttgt agtttttaag tttttgtttt ttaggttggt tttgttaatt ttttaagaat 10440
atttgattta tatttaaaaa aaattatttt gttttgtttt tttggtttta aaagtttttt 10500
tattagaaat ttaatgaaga tgtaattttt ttttgttttt ttatagtagg taggtaaaat 10560
tttgttagga atttttttta ttgtttttag ttttatttag gagtttttta taaagttgag 10620

ttttagttta gtttggtttg ggtatatttt tttttatttt tgtgttttat tttttatttg 10680
taaaatggaa agaagttgga ttaggtaggt tttgtgtgag ttttttattt tattttaaat 10740
gattaaattt tgttgtttta aaagaatttt tttataagag atttaaatag atattttagg 10800
attttttaaa agtaaaagtt aatattttta tttatgtata gtttattatt ttgtttgaaa 10860
tttaatttgt tggtttatag gagaatgggg tagaagattt agagttattt attttatttt 10920
atgtgtatat tttaattttt tttatttgtt aggtgttggg ttaggggtta ggttataggg 10980
tagggtaggt aaattttttt ttttgtttag atgtagtggg agatatggtt ttataaagtt 11040
aagaagttat aggttagggg aatatttagt tttttattta tattaaaatt tttttttttt 11100
tttatatttt tttttatttt tgatattgtg ttattttagt ttgggttggt tttgttgtt 11160
atatatatta tatagaattg gtttgtgttt ttagttgtgt gtttttttta ttagaaggta 11220
gtttttttaa gaaaggtgtt ggggtttgtt ttttttgtat tttgtttatt ttaattatat 11280
ttgtgaaatt taataagaat gttagaattt tttaatagga agagtttaga ttgaaaaatt 11340
tgttttgaaa ttatattttt gtagtaagaa tagggggttt gtatttagat atgttttttt 11400
gaggtagttt aggagaaggt tttgtgtagt tgatgggtat tataaggatg tttattgttt 11460
ttttgtttgg ttattggttg atttttgaat attagatatt ttttagtagt ttaaagaaat 11520
taaataaatt gaagtgtttt tagtgtttgt gaggaagggg tgtttttgga tgggtataag 11580
ataattaagg gttttgtttt ttttatttat tgttaggtat tttttatttt taagttatag 11640
aggtgtgttg tttaggattt ttagggatag tagggataat aggtggggga tatgggggag 11700
gggtgggtaa ggaattgatt tattaaattt tagttttagt tattaatttg atagaggtgt 11760
gatagtgtgt tttttttaga attagttgta ttttttaagt ttagtttgaa attttttttt 11820
gaggttgaat tagagattgt ggtggatttt ttaggaatgt tgaagtaatt ttagttgttt 11880
ttttttgtgg agggtaggag tggttagtat tagaggtagg ggaggggagg aaagtggggg 11940
agggtgggtg gaggtgaaaa tgatgttatt tttagtttta gttagtttaa ggggttgtta 12000
aatgtttgtg agattttttt tgtttaggtt atttttttta tgttgttgat gtgttaattt 12060
tgggtttttt tgggggtgta ttttgtttag tggtgtttgt agatatttgt tttggtttgt 12120
gtgttgtatt gtgatatatt ttggattttt tttttatata tatgtttata tttatatatg 12180
tatattgttt tgtgtgtgtt atgggattga agtgttatta gttttttgga tttatatttg 12240
tttttgattt agtttgtaag ttagatatat ttatgttttt ttttttagtt tttttgttag 12300
tgttatttat ttttattaat tttttattta tatatttgtg tgtaattttt ttgtttttag 12360
atatagatat atatatatat atatatatat atatatatat atatttattt agtttttttt 12420
gtatttatgt ttagtgtgta tttttgttag tggtgttagt tgggatttgg agatgatgtt 12480
aggaaatgtt attttggtat ttttttggat tgttttattg tttttttttt gatttttaga 12540
attttaattt ttagttaggg gggagatgaa tgggattttg tttttttttt tttttttttt 12600
gtaattgaat tttttatttt agtggtgttt gtatttaaag tttttttgtg tttgtgtttt 12660
ggggtgattg tgtatttgta gttttggggt gtttattttt ggagtatgtg gggttataaa 12720
gggtggtttt ggtgttttag tggtattgta tgggtggttt ttttttgttt tgtgttgtgt 12780
ttgggagaag agggtgtggg tttatttaat gtgagggaga aaggaaagga tggtgttgag 12840
gttggtggtt tagtgttttg tggaagttga gggtggatgg tgaattgatg agttttagta 12900
ttagttggga gttaggtgag gtttgtttat gtttgggtgg agggtgattt tataggtgtt 12960
gtggttttgt ttttgtgttt tgtttttttt agatttttgt agttttagta tgtagttggg 13020
atatattttt tttttgtttt ttgttttttt tttttgggtg tgttgggttg ggttgggttt 13080
tggtagtgtg tggtgggtgt ttattggagt tgttgtttgt tgtgtaagta gttgtagggg 13140
tgtggttggg aggggtattg gggtttgggt tgtgggttgg ttttgtagtt ttttttgagt 13200
ttagggtttt ggtttgtgta gtgggtgtgt gtgttttgtt tggaggaggt gtttgtgtgt 13260
ttgtgtgtgt ttatgtgtgt gtttgtgtgt gtgtatgtgg atttttttgt gtttttgtat 13320
gggtggaggt tttttatttt tgttttagag ttttttttaa gagttttgtt gtgttatgtt 13380
ttgagtgatt ttttttattt tttgtaggtt ttattagttt ggttttttag ttggatttgg 13440
ttgtttgttt agtagttgtt tttgtttttg tttgataggt tttttttgat ttttagtttt 13500
ttgtgttgtt aaagtttttg ttgaaatttt tgtttttgtt taattttgtt tattttgttt 13560
attgttgttt agtgtttatt ttttttttta tttttttagt tttaagtata tttattggat 13620
tttgtttatt gggttttttt tttttgtttt ataagtttag atgagaggat ttttattttt 13680
ttttattttg attttttgaa tttttatgat ttattttttt taattttagt attgttttgt 13740
tttgttgttt tgtttgtgtg taattattgg tttgtatgtt tttatttggt taaagaaatt 13800
agtaattttt aatttttatt gtatatgaga agttttagag gagttgttaa attaaaaaat 13860
aaataaatag atagataata aaaagagaga gatggatttt agaatagttt aattagaatt 13920
ttagggggtg taaattaatt gggtattggg gtttttataa agttatttag gtggtttttg 13980
atgtgtagtt aggttgagaa gtattgtatg gaggaatatg attttatgaa taaaggaagt 14040
ttgatttata gttggtattg ttattgtttt taggttattt gtttagtgtt gaatttgtta 14100
ttgagtttag gattagattt agggtaagtt taggtgattt tttttgtgtt tttatagata 14160
tatatgtgtg tatgtataat attttttgtt gaattgtaaa tttttttttt gtgttaattt 14220
atgttgtttt taaggtaggg gttatggttt ttttttttga ttattagggt tagttttgga 14280
gaagggtttt ttggaataaa agaaatatgg agaggggtaa atagtatttt taggatttag 14340
gatatttgtg ttttaggttt gtttttgata ttattgatta taaattaata aatttgggtt 14400

ttagaatttt tattggtaaa gggagaggat tagatttaat gattttttag aatttattta 14460
gtgttaaagt tttgttattt tattataaat gattagtgtg aagaatagag ttttagtgtt 14520
atgggttttg ttgttagaag agtttggtta aattataaag tttggttatt agttatgaag 14580
tttggttaaa ttataaagtt tggttatatt taagaatgtt ttgaatgtag gagtatattt 14640
ttttatttta gaaaatttaa tatttagaat taataagtga atgaatgatt attgggtagt 14700
tttttatttt ttatggattt gaattagttt aattgtagta tttttagtga gggaaaattt 14760
tttttatttt aaataaaatg ttgtgtttag gtagagtggt ttatatttgt aattttagta 14820
ttttgggagg ttaatgtaga agtttttttt gagtttagga gtttgagatt agtttttata 14880
atatagtgat gttttatttt tataaaaaag atgaaagtta gttaggtttg gtggtaagta 14940
tttgtagttt tagttatttt agaggtagag gtgggaggat tgtttgagtt taggagtttt 15000
agaaaagttt gagtaatata atgagatatt atttttataa gaaaataaaa aattaggtgg 15060
gtttagtggt atgtagttgt agttttagtt atttgggagg ttaaggtagg agttttgttt 15120
ttttaggttt tgaaatttat ttattttgtt attttataaa tgatagaatt gagttgtttt 15180
tatgagttgg gttttgtatt ttgttattgg gaaaggattg aaaaggtaaa aatatttttt 15240
ataggtgtat agtatggtat agttttaaga tgtagttttt ttatttatat agtgtggttg 15300
agatgaattt atttattttt taattatatt aaggatttat tatgtttagg tattatttta 15360
gattttgggg atatgattat gaataatagt aataataaaa gtgtattttt gggagttatt 15420
ttgttgttta aagatttttt tagattaagt agtttgtgtg tttgttattt tttagttagt 15480
ttatatatat ttattttttt ataggttttt tattgtggtg atttattgag tgagtatttt 15540
tggtttttta tttgttaaag atataagagt gttaagttta aaagtgtgga gtgttaagat 15600
aaggaatgtt aagtttaaag gtgtagggaa ggtggtttat tttttagtta ataaatggaa 15660
ttagtgtatg gaattagaag ggtattttgt taggggaggt ttttgtttgt ttttttgagg 15720
aggtttttag gatagaatgt gaagagtagt ttagatttgt agtttgaaat tttgaggagg 15780
ttgtttggta ggaggttggg tttgtgtggg aagtttaatt ttttggaaga ttattagtat 15840
tgaataggtt agaagataga ggaaggagag tggagtgaag agaaaagagt agagaaggga 15900
ggtaagaatt tgagtaatgg aatataattt tttaagtgat ggtattttaa aattagagtt 15960
tatattattt tggagaaggt ttgaggaaga tattatttgg attttatatt tttttttttt 16020
agtttaatat agttaatttt ttttttttgt tgaaaaggat gttttttttt gttgagtatt 16080
agatgaagta gtttttttag ttaagtgtta tgttgtagga gaaagattgt ttttagtata 16140
ggtttggatg tatataaagt atagaggatg tttattttat atagaggttt tttattggga 16200
tttgggagtt gtatgtattt ttatattttt tattgtagtt agttgaggtg gtagaagttt 16260
atggagttga atgtgggtgt aattgtattg attgtttata ttattttttt tttttgttgg 16320
ttagtagtag aattagattt gtttagtaaa tgtatatgtg ttgtaaattt gttataggat 16380
gagattggat tttttggatt aatgtttatt ggaaattttt atttaataat aatttagatt 16440
ttatattaga atattttaat taaatgtttt aaaaatgatt ataggaatta tttgttttta 16500
attgtttttt ttaaaattta tgggtatttg gggttgggag agattaagat gtagtaggga 16560
tttatggaga agaatttggt ggtttgtggt ttttagtttt aattttatta tttatgagtt 16620
gtagtatttg gggtaaatta ttttattttt ttgtgtatgg aggaaatgag tgagagaaat 16680
tattagattt tttaattata atttatgggt ttattgttgt agattattat tttttttgtt 16740
tgttaaaatg aaaaagtgtt tgtaaaagtg tttttttatt tttagttttg ggtttttttg 16800
ggattttttt ttttttttag tatttatttt tttttttttt ttggattaaa ttaattaata 16860
agtaaatatt tattgtaatt aatgatttat tttaaattaa ttttttttta attttaagtt 16920
ttttattttt gtattgtagt ttaattaata agtaaatatt tattgtaatt aatgatttat 16980
tttaattaat tttttttgat ttttaagttt tttatttttg tattgtagtt tagttttttt 17040
tatttttttt tttttaaatt ttttaaaata agtatttgta tttaaaggtt tattttattt 17100
tttttttttt ttattttgta gtgattatta agtttttttt gaatttggtt aaataagttt 17160
ttaaaatttg ttaggaaaaa taatagtttt tgttttttta tttttttatt tttttatttt 17220
tagaagttat tattttttgt ttttggagaa ttatttttat tttatttttg ttatttgtag 17280
attttatgtt tttgggtaag ttattttatt tttttttttt gtttttttat gtataagata 17340
tatataataa tatagggtta ttgtgataaa tatatgtgtt aatatatgta aagtgtttag 17400
aatagttttt ggtatataga aggtttttat taaagtgttt atttttattt ttattgttat 17460
tattattatt attattatta ttattattat tattattatt ttgttttatt ttttttggta 17520
ttttgtatat ttgattgtag agaagtttag taaatattgg ttgaagaatt gagtaaatgt 17580
agatgttttt ttttgaattt attttatttt atttggtttt attgtttgga ttttgttagg 17640
agggaaaagg ggattttatt tgttttatga ttttaggtgg agggattgtt atttgggatt 17700
tttatttaag taaaagtaaa ttattttagt tatttgtaag tatgtttggt ttgtaatatt 17760
atgttgtgag taatgaaaat taaaaaaaaa aaaaattatg aaaggaaaat aattttaaat 17820
gagggtgaag ggtaggttat gtttaggatg tgtaataaat tttttatttt gaatataaat 17880
gaagagttgt gtaatgt 17897

<210> 363
<211> 5987
<212> DNA

<213> Artificial Sequence

<220>
<223> chemically treated genomic DNA (Homo sapiens)

<400> 363

tttttaattt gtattatttt ttttatttta ataatttttt ttaatatttt ttataaagta 60
gatttattgg ttatagaatg ttttaatttt tatttgtttg agaaaatttt attttttttt 120
tatttttgaa agataatttt gtagggtata gaattttagg ttgtaggttt ttttttttta 180
aagtgaaata ttttatttta tttttttttt ttttgtatgg tatttgagaa gaagttagat 240
gtaattttta ttattattat ttaaaagatt gttttgtttt tttttttttt tttttttttt 300
tttttttttt ttttgtatat tatatttttt atagttgttt tatatttttt agatattatg 360
ttttggtttt tttttgtgtt tttttttttg atttttagtt ttagaagttt ttatttatat 420
atttgtaatt gtagggattt ttttttttgt tatgtttagt ttattaataa gtttttatag 480
atattgttga tttttgtttt agtgtttttg atttttagta tttttttgat tattttttgg 540
aattgttatt tgtttattta tattattaat ttatttttgt gtgttgtttt attatagtaa 600
ttgtagttgt tttaatttta taggtattgt aattttaata tttttattat atttgatatt 660
gattttgatg tttgttttgt tttattaagt tatgtttttg ttttttagtg tgatttttaa 720
ttttttgttg aaagttaggt atgatgtatt gagtgaaaga aatttaatat attgtaatgt 780
gatgataaga gtttagggga agtgaagtat tttatagttt tatagtaggt tttggttttt 840
tagtgagttt gtgtttatga atggtgattt ttaataagtg ttttttattt tatttttttt 900
ttgtttttaa gtgggataag attattgggg gggggttaga attgggtatt tttttttttt 960
aatgtagaag ttaaagagag ggttggagtt gggtattttt ttttttttgt atggaaagtt 1020
agaggtagtt aaatttggat attttttttt ttttaattta gttaggttgt gataaaaatt 1080
ttgatagttt aggttttaat attataaaat aatttttttt gagtataggt tttattaaga 1140
atattatatt ttgatggagt tgagggggag ttttttttga tatttattgt gagaattttg 1200
tagagtttta ggaagtaaaa tttataaaag tgtgggagtt ttttagaatt ttttttttgt 1260
agatttattt gtattgaatt tttagaaatt tattaattat agtttaggtt tttttattta 1320
ggtattggtt tttatggagg tttttgtttg tgtatttttg ttttagtaag ttgttttttt 1380
tgtatggttt gttttttaaa ttttttaagt agggttatga tttgttgttt tatttttttg 1440
atagttttga gagtgttgat tttttagttt gtttagattt ttatttgttt ttaggatgaa 1500
gtgataattt ttaagttttt ttttgatatg ttagattaga aattgaaagt tttaagtttt 1560
atattttgtg tgtgggtatg tttatatttt gtttgtttta gtgtttttat tttatatttt 1620
tttttttttt tttgtttttt tgtgagattt ttaggtttaa tataaagatt gtgtttaatt 1680
tatttaatta ttttggttta tttgagtatt ataatgaata attataaaaa aaaatgaagt 1740
aaaagaaaaa tttattaaag aattgagata tttgagaaaa agaaaggaga ttagtgtttt 1800
ataaaattta gaaatagatt ttttaagtgt ttttttattg atttatgtga aaggattttt 1860
tttaatttaa taaattatgt gtttttgttt atagttttaa aattttttgt gtagttaaga 1920
atgggtaaat aattaggttt tattaaagga ttaatgtaaa gattttttgt aagtaatatt 1980
tttgttattt aaggaagaga taaattttat ggtataattt tgttaaagta tattaagaat 2040
aattttgata taaagttttt tttttagtta atatgttatg aaagaaagaa gataaggggt 2100
gatttttatt ttttaagtga attattaaat ttattaaaga agaaatgagg gaaatagaaa 2160
gaggattttt gtttgagata atggatttgt atgtatgagt agtagaattt tgtttaaagt 2220
ataaggaagg gaaaaaaaag ttagtttatt tggaattttg gatattaaga gtttttattg 2280
tttatttttt tttaatttat atgaatggtt tattatttta attaataaat attttatttt 2340
tttttaatat atttatgaaa taaatttgaa atgaatagtg taatatgtga atgtttagaa 2400
tattataaaa ttaaatataa aatttgtttg gtaatttttt tagtatttta ggaaaaaagt 2460
tgataaaatt ttaagtagta gaagggggta gtaaaattta atagaaagtt ttggaagatt 2520
tttaagattt ttttttattt tttttttatg tagattattt tttaataaat tttagatgtt 2580
aatagaaatt ttgtataata gatttatata tttgtttaaa atagatatag aaatattgaa 2640
tatatgtaaa tatgagagtt ataagtttta tatgattaaa tttttttttt atggtatata 2700
atagttatag tattttttta tataaaatag gtttagtggt tttaatttag tttggtatat 2760
ttaatatatt tttatgatta gtattttaaa tgtatttatt tgttttattt tattgtttta 2820
gaattgttag ttatttaata aattatgtaa tttttttttt tatgtttaga tttgtatttt 2880
tttttaaaat tttgtttatt tttaaagttt tagatttttt ttgaattttt ttttttgatt 2940
ttttaagtat atggaatttt ttattttatt ttgttatata agtgatagaa tttttattat 3000
gggatagatg gagtttaatt tttttgagtt taaaataatt taaatataat tattttttat 3060
gttttgtttt ttttttattt ttgtatttaa attttttttt agataataga ataattaatg 3120
tttgataagg aagataatga tgatgattat tttaaaatga atttaggatt gtaatgtaaa 3180
attttagtat ttttttatag tatggatttt aatatggttt ttaatttaaa ttaatattag 3240
tagttttaat tataaatttt aaattttagt agatgtaatt tattttttta aaatgaaata 3300
gaagattgaa attattaaat tattaaaaag aaaatgattt atgtttttag ttgaaatttt 3360

atgtaagatt ttatgtaata aataggagtg ttataaatgg aatgatgaaa tatgattaga 3420
ggaggagaaa ggttttttag atgagatgga attttagtta tttgtgtttt atgaagaatt 3480
agatgtgtat attaagtaaa atagttaaaa aaaaaatttt taagtgagtt ttttatttat 3540
ttttttttta taagattttt ataaattgag gtatttggtg tagttttatt ttaggtttta 3600
tgttgttatt tttttgtaat gttaaggatt taggatataa ttgaattttt tattttttat 3660
tttttttttg gtgtgtgtgt atatatatat gtatatatat atatatatat atatatatat 3720
atatttttta gtattttatt tttatatgtt tttttttgag tattatttat gatagatgtt 3780
aaataaaagt aaagatgaaa ttttaattgt taaaattttt tttttattta attaattttt 3840
tatttaatta tgttttaaaa tgagaataga aaattagttt taataagttt aggtaattga 3900
aaagtaaatg ttatgttgta ttttgattta tggttataat ttataatttt ggaaaagtgg 3960
atagaaaaga taaaagagtg aattttaaaa tttgaattta ttttattagt attttttatg 4020
aagggttagt aattaaaata atttatgtat tagggagaga aatgttttaa ggtatatgtt 4080
ttggatattt agtgtttttg taaattttgg ttattgttgt tttttttgtt tattagaagg 4140
taggaaattt tatattggtg atttgtggag tttatattaa ttatttatag ggtaattgtt 4200
taggattagt attatgagga gaatttattt tttttgtttt tttttttaag aaataaggag 4260
ggggtgaagg tatggagaat agtatttttt ttgttgaaag taatttagtt ataaagataa 4320
attatagtta tgtatattga aggtagttat tttattttat aaaataagag ttttttaaaa 4380
agttatgtat gtatgtgttg tatatagagt agatatatag tttattaagt gttgttatta 4440
aaatataaaa tatgttagtt ttttttaatt ttatttgttt tagtttgttt tgatgtgatt 4500
tttttgattt tttaaagatg tatagattag atatggtggt ggtggtggga gaggggattt 4560
tttgtgtttt tggattttag ggttgtttag atttttggag aggaagttaa gtgttttttt 4620
gttttttttt ggtattttat ttaaggtgat tagtttagaa ttggtttttg gaagtgtttg 4680
ggtaaagatt gtgaagaaga aaagatattt ggtggaaatt tgtgtgtttg gggtggtgga 4740
atttggggag gagagggagg gattagatag gagagtgggg attatttttt ttgtttttaa 4800
attggggtag ttttttgggt ttttgatttt tttatttttg tgggtaaaaa attttgtttt 4860
tattgggttt atgtaatttt tttaagggga gaggagggaa aaatttgtgg ggggtatgaa 4920
aaggtggaaa gaaatagtta ttttgttata tgggtttggt ttttagtttt ataaaaagga 4980
aggttttttt ggttagtgat taattgttat atgatttgta gtgagtgtta ggagtatgtt 5040
taggaatttt ttagtagtgt tttttttagt tttatagtta gatgttttta gatagtaaag 5100
tttatttttg tgttgtgttt tgtttgttgt tgtgatgttt gtttgtgttt tgttgttgtg 5160
tgtggttttg gtgtttagtt atataggtga gtatttggtg ttgtgtattg gggattttgg 5220
ttttatgtat ttgggtagag tttttgtttt gattttttgg gtttatttta gtattttgat 5280
ttttttttga atagagaagt tatgtgattt gggaaagagt ttggattgtt agagtttgaa 5340
agaattttgt ggatatttta gtttttttat aagtattgat tattatgagt tagttattta 5400
attgatttga gatattttta ttttttaaat agggatagat gatattaatt tgtaggttgt 5460
tattatgata agataggatt tgattaatat atgtgaattg tttatatttg gaattttttt 5520
attgagtgga agaagttgtt ttaaatattt tagttagttt ttttttgttt ttaggaaagt 5580
ttggattatg ttttaagata agtaaaatgt tttaaaagta agttgtttta ttttgaattt 5640
ttttttaaat gttgattagt gtattagatt tattttaatt tggaaagtga agtgttattt 5700
atttgaattt tttaaaaatg ttaattttaa tatttaaaga gttaattaag aaaagtttag 5760
taatatgatg tattaagttg aatatgttgt tatttttatt tagaatagaa aattggtatt 5820
tttatgtttt atttatttta aggtaggtta aaaaattgta tttttatgat tatttatata 5880
ttttttgaat ttattattgt aaagttgatt tatagttaaa taattaaatg tttaaattaa 5940
gattaagata ttagagaatg atttatttgt tgttttttaa ttgtagt 5987

<210> 364
<211> 5987
<212> DNA
<213> Artificial Sequence

<220>
<223> chemically treated genomic DNA (Homo sapiens)

<400> 364

gttgtaatta aaggatagta aataaattat tttttagtgt tttaatttta atttaaatat 60
ttaattgttt gattatgaat taattttata ataataaatt taagaaatat ataggtagtt 120
atggaaatat aattttttaa tttgttttag aatggataaa atgtagaaat attaattttt 180
tattttaaat aaggataata gtatatttaa tttggtatat tatgttatta agtttttttt 240
agttaatttt ttagatgtta aaattagtat ttttaagaag tttaaataag tagtatttta 300
ttttttaaat taaaatggat ttagtatatt aattaatatt tagggaaaaa tttaaagtaa 360
aatagtttat ttttaagata ttttgtttat tttaaaatat aatttgggtt tttttgggag 420
taggaaagaa ttgattagaa tatttaaaat aatttttttt atttaataaa aaggttttaa 480

atataaataa tttatatata ttgattagat tttgttttat tataatgata atttgtaaat 540
tagtattatt tatttttatt taggaggtga gagtgtttta gattggttaa gtaattggtt 600
tataatgatt agtgtttgtg ggaaagttgg aatatttatg gagttttttt gaattttagt 660
ggtttaagtt tttttttaag ttatgtagtt tttttatttg gagagaagtt ggagtattgg 720
gatagattta ggaggttaga gtggaaattt tgtttgggtg tgtggaattg gagtttttgg 780
tgtgtggtgt taggtattta tttgtatggt tgagtgttag gattgtgtat agtagtaggg 840
tgtgggtgag tattgtagtg gtgggtaggg tgtggtgtgg gggtaggttt tgttgtttga 900
gggtgtttgg ttgtggagtt gaaggaggtg ttgttgagga gtttttggat gtgtttttga 960
tgtttattgt aagttgtatg ataattggtt gttaattgag agaatttttt tttttataag 1020
attgaaaatt aagtttatgt gatgaaatga ttgttttttt ttgttttttt gtatttttta 1080
taaatttttt tttttttttt ttttaaaaaa attgtgtaag tttggtgggg gtagggtttt 1140
ttatttatgg aaatgagaaa attggaaatt taggaagttg ttttaatttg ggagtagagg 1200
gggtagtttt tatttttttg tttgattttt tttttttttt tttgagtttt attgttttag 1260
gtgtataggt tttgttagaa tgtttttttt tttttgtagt ttttgtttga gtgtttttga 1320
gagttagttt tggattgatt gttttggatg ggatattggg ggagggtaga aggatatttg 1380
gttttttttt taggaatttg agtggttttg aggtttgggg gtgtagggaa tttttttttt 1440
tgttgttgtt gttgtgtttg gtttgtatgt ttttagaggg ttgaggaagt tatgttggga 1500
tagattgggg tgagtaaggt taagaaaggt tgatatgttt tatgttttag tgatgatgtt 1560
taataggttg tatatttgtt ttatatgtag tatatatata tatagttttt taaaaaattt 1620
ttattttgtg gaatgaaata gttattttta gtgtatatag ttgtaattta tttttgtagt 1680
taagttgttt ttaatagaag aaatattgtt ttttgtattt ttattttttt tttgtttttt 1740
ggaaagagag gtgggaaagg taaatttttt ttataatatt ggttttaagt agttattttg 1800
taaatagtta atgtgagttt tatgggttat taatataaag ttttttgttt tttgatggat 1860
aaaggaagtg gtgatggtta gaatttgtag ggatgttaaa tgtttaaaat gtatgtttta 1920
aggtattttt ttttttgatg tgtggattat tttggttatt agttttttat aggagatatt 1980
ggtaaaataa atttgagttt taaagtttat ttttttgttt tttttgttta tttttttaag 2040
attatgagtt gtgattatgg attaaagtat aatatagtat ttatttttta gttgtttggg 2100
tttattgggg ttaatttttt atttttgttt tggaatatag ttggatgagg aattaattag 2160
atggaaggga gattttgata gttggaattt tatttttgtt tttgtttaat atttattatg 2220
ggtagtgttt agggaggagt atgtgagggt gagatattaa aaaatatata tatgtatatg 2280
tgtgtgtgta tatatatata tatatatata tatatattag aaaagaagtg gaaaatagaa 2340
aatttagtta tgttttaagt ttttagtatt ataggaaaat gatagtataa aatttgaaat 2400
aaaattatat taggtatttt aatttgtaga agttttataa gaaaaaaata aataagagat 2460
ttatttggag gttttttttt taattgtttt gtttagtgta tatatttgat tttttatgag 2520
atatggatga ttaaaatttt attttattta ggaagttttt tttttttttt agttatattt 2580
tattatttta tttatggtat ttttatttat tgtatggaat tttatatgaa attttaatta 2640
agagtgtgga ttattttttt tttgataatt taataatttt aattttttgt tttattttaa 2700
aggagtaggt tgtatttatt gaaatttgaa gtttatagtt agagttatta atgttagttt 2760
gggttagaag ttatgttaga atttatattg tgagagagta ttaaaatttt atattataat 2820
tttgaattta ttttgaagtg attattatta ttgttttttt tattagatat taattgtttt 2880
gttgtttgaa aagagatttg gatataaaag tgagggaaaa atagggtata aggaataatt 2940
atatttagat tattttaaat ttaaaggaat tgaattttat ttattttata gtggaaattt 3000
tgttatttat atagtaggat agagtgaaga gttttatgta tttggaaagt taaaaaaaaa 3060
agtttaagga gaatttggga ttttaaggat gggtagagtt ttagaaagaa gtgtaaattt 3120
gagtataagg aaaaaagtta tataatttat taaataattg ataattttga gataataaaa 3180
taaaatggat aggtatattt gggatgttgg ttatgggagt gtattaaatg tgttaaatta 3240
aattaagatt attaaatttg ttttatatgg aaaagtattg tgattattgt gtattataaa 3300
aaaaaggttt gattatgtaa aatttatagt ttttatgttt gtatatattt aatgtttttg 3360
tgtttatttt aggtaaatat atggatttgt tgtataaaat ttttattagt gtttgaaatt 3420
tgttgggaaa taatttatat gaaaagaaaa taaggaagaa ttttaaaaat tttttagagt 3480
tttttgttga gttttattgt ttttttttgt tgtttgaaat tttgttaatt ttttttttaa 3540
gatgttagga agattgttag atagattttg tgtttaattt tataatgttt taaatattta 3600
tatgttgtat tgtttatttt agatttgttt tatgaatatg ttgaaaagaa atgaaatatt 3660
tattaattga agtgataagt tatttatgtg agttaaaaga aaatgaataa taaagatttt 3720
taatgtttaa aattttaaat aaattaattt tttttttttt tttttgtatt ttgagtaggg 3780
ttttattatt tatatatata gatttattat tttaggtaag ggtttttttt ttattttttt 3840
tgtttttttt ttggtgggtt tagtggttta tttagagagt ggagattatt ttttgttttt 3900
ttttttttat ggtatgttgg ttgaaaaaag agttttgtgt tagggttatt tttagtatat 3960
tttggtaagg ttatgttatg aagtttattt tttttttgag tagtagaaat gttatttata 4020
gaagattttt atgttagttt tttagtaaag tttgattatt tatttatttt tagttatata 4080
agaagttttg aggttataaa tgaaagtata taatttgtta aattaagaaa agttttttta 4140
tataagttaa tgaagaaata tttaaaaaat ttatttttaa gttttataaa atattgattt 4200
tttttttttt ttttaaatat tttaattttt tgatggattt tttttttatt ttattttttt 4260

ttgtgattgt ttattataat atttagatga gttaaagtag ttgaatgagt tgaatatagt 4320
ttttgtattg gatttagaaa ttttataagg ggataaggaa gggaaagaga gtgtgaggtg 4380
ggggtattgg agtaggtagg atgtgaatat atttatgtat agaatatgag gtttaggatt 4440
tttagttttt gatttggtat gttagggagg agtttggaaa ttgttatttt attttaaaaa 4500
taagtaaaaa tttaagtaaa ttgaaaaatt aatattttta gaattgttag agaagtgagg 4560
tgataggtta taattttatt taaaaaattt gaaagataga ttatataaga agaataattt 4620
attggagtag aaatgtatag gtagaaattt ttatgaaaat tagtatttgg gtaggaaaat 4680
ttgaattgta attgatgaat ttttggaggt ttagtgtaga taagtttgta aaggaaaaat 4740
tttggaagat ttttatattt ttgtgagttt tgttttttgg agttttatga ggtttttgta 4800
gtgaatatta gagaaaattt ttttttagtt ttattagagt atagtgtttt taataaggtt 4860
tatatttaag agaaattatt ttataatatt agagtttaaa ttgttgggat ttttgttgta 4920
gtttaattga attagaagaa ggaaaatatt taaatttaat tgtttttagt tttttatata 4980
ggggaagaaa aatatttaat tttagttttt tttttagttt ttatattgga gaagggaaat 5040
atttaatttt agtttttttt tagtgatttt gttttattta aggataggaa aagagtggaa 5100
tgaaaagtat ttgttgaaag ttattgttta taggtatagg tttattaaaa ggttgagatt 5160
tgttatagga ttatagaatg ttttattttt tttgaatttt tattgttata ttataatgta 5220
ttgagttttt tttatttagt atattatgtt tggtttttaa taaaaaatta gaagttatat 5280
taaaagataa aaatatagtt tgataagata gagtaagtat tagaattaat gttaaatatg 5340
gtagagatgt tgaaattata atatttatga aattaaaata attgtaatta ttatgataag 5400
ataatatata agaataggtt ggtaatgtaa gtagatagat ggtaatttta agaaataatt 5460
agagaaatgt tagagattaa aaatattgga atagaagtta ataatgtttg taagggttta 5520
ttagtagatt ggatatggta gaggaaagaa tttttgtgat tgtagatata taaatagaga 5580
tttttaaaat tgagaattaa agaaaaaaat atagaagaaa attaaaatat aatatttaag 5640
aaatatgggg taattataaa aggtgtaata tatagagaag gaaagaaggg aaggagaaga 5700
gagaaaggaa tagaagtaat tttttaagta ataatgataa gaattatatt tgattttttt 5760
ttagatatta tataaagaag aaaagagtgg aatgaaatat tttattttga ggggaaaaaa 5820
tttataattt agaattttgt attttataaa attatttttt aaaagtgaag gagaaataag 5880
gtttttttag ataaatgaaa attgaggtat tttgtggtta gtagatttgt tttgtgagaa 5940
atgttaaaag aagttattga gataagaaaa atgatatagg ttagaaa 5987

<210> 365
<211> 5683
<212> DNA
<213> Artificial Sequence

<220>
<223> chemically treated genomic DNA (Homo sapiens)

<400> 365
   atggtaaata tttgaaaaag tttttatgtt gaaatttaaa ttagattata tttaggatgt 60
   tttataaggt ggtatttttt attaagatga taaaggataa atattggtgg agttagagag 120
   gttgttttta aatatagttt tttttattag taatattgta gtagtttgta tttattgttt 180
   agagtatgaa aaagattttt gttaaagatg gtttttgggg atttattatt attatatgtt 240
   aagagtttgt tttttatgtt tttatttttt tattttgtta atttttttta tttttatttt 300
   ttggtttagt gtgagatttt attatttttt ttttttattt tttttttttt atatttttgg 360
   ttttttagta ggttaatgtt tttgtttttt tattggaatt tatttatttt ggttattttt 420
   ttttttatga atttgttgga ttatatttta aagttattta aaagagggat ataaagatat 480
   aataaaattg gaaataatgt ttatttttat tagtttgtag ttaagagtgt gtggaattag 540
   ggtggttgta ggtaaagaga tagggggaag ggttgtttta tgttatgggt aggatagtaa 600
   atggtgtaaa ttttttggaa agtattttag taatataggt gaagagtttt tataatgtgt 660
   ttattttatg atttattttt atttttttta ttttaaagga ataagaagag gtttaattaa 720
   agtatgagtg aaaaggttat ttaaatatag ggaaaggggt tgttatgatg tttatggttt 780
   taagaaatag aaaataataa tttggtttaa ataataagta tataattaaa gaaatttaga 840
   agtaggattt ttttaagagt tgagagattt agtagtttaa aaatgtgatt tgatatatga 900
   gtttttttta tgtttttgtt tttttttttt taatattggt tttatattaa atttggtttt 960
   ttagtggtta atagtattaa atagtattta ttggtatttt tgttggtatt taaaagagat 1020
   ggagaaagtt tgattttata tggttttttt ttaaaagtaa taaaggattt tttttggaag 1080
   tttttagtaa attttgttag atttgaatta tatgttaatt tttaaattag ttattggtag 1140
   ggaagtggga aaaaggggag gaggtgttta tttttaatta atatgtttat ttttgtttat 1200
   tgagattaat tttataaaag tattgtttta gttttttgaa gaggagtata atgtttgtga 1260
   gttatttatt atatttattt attaagagtt aagggataat tgagtaaaat ggtagagttg 1320
   gtaggaaaat ggtgagtagt tttgtttttt tgtgttataa ttgatttgta tataaagaga 1380
   tttggaaatt taaatataag aatttttttt ttttaagttt agggttttgg gtattttaag 1440
   tttggttttt gtttgggtat agtggtttat gtttgtaatt ttagtaattt gggaagttaa 1500
   ggagggtgga ttatttgagg tgatgagttt gagattagtt tggttaatat ggtgaaattt 1560
   taattttatt aaaaaatata aaaattagtt ggatgtagtg gtatatgttt ggaattttag 1620
   ttatttagga ggttgaggta ggaaaattgt ttgaatttgg gaggtaaaag ttgtagtgag 1680
   ttgagattgt attattgtat tttagtttgg gagatagagt aagattttat tttatttttt 1740
   attaaaaaaa aattttggtt tttatatgtg aaaaaataaa agttaatttt taaaaattaa 1800
   ataataaaga gtgttaggtg ataagtttta tgatagagat aatgtgtgtt ttaaaatata 1860
   tagtataaaa tagatggaaa atattttatg gagaagggag agtttgaatt ggtgtttgat 1920
   tgaaggataa gaaaaatttg ttggtaattt tttttggttt ttgattttgg atgtagttaa 1980
   agtaaagtaa tttagaatta tgatttaggg aatatattta gagagaaatg aatttatgat 2040
   gtttttaatt tgaagatgtt atgattttag attattttag aatataatga ttttttattg 2100
   ttttatattt gtaagaaatg ttttattatt gaaaatttgt aatatttaaa aaatttttat 2160
   ataaaattat agtttatttt gttgttttat gttaaataaa taaaatattt ttttttaatt 2220
   atgttgtttt ttttaattat atgtaatttt aaaattagtt gtaattgata gttttttttt 2280
   tatatttatt gttaatgtat ttaggttaat tttattttta attagagaaa tttagaaaaa 2340
   ggaaatttta tggtgaaatg ttatatgttt tataattttt agtaattaat ttttaaggat 2400
   gataatttgg taaaaaagag aattggatta gttttttgaa aggtttatta ttttggttta 2460
   tgttaattga ttgttttaaa attatagtgt ttttaggttg tagagatata tttaaaatgg 2520
   attattgttt ttatattaaa ggggataggt tttttttttt ttttttttat ttttgttttt 2580
   tttaaagttg tatagaggta tatgaggttt ggggaatatt tgtagtatat gttggttggt 2640
   aagaatgtgg gtgaattttt gttaaggggt gtagatattt ttttttttat gatttttttt 2700
   ttttatttag aataattgaa gtattatgat aagaggaagg gttttgtgtt tgagataagt 2760
   agtttttata tttgttagtt ttttttaata agaaagtttt tttttgttat tgtataggtt 2820
   atttttttat tggtaaagta tgatgaagtt tggaaattgt tgtttttgtt ttgttgaaag 2880
   aagttaagtt ttaagaaatt tgtagaggta tttttataat tttttagtta tggtagtatg 2940
   aattttgaag gggtagtgag tgttggtttt tattttgttt gtatagttaa gtttagggga 3000
   gataattttt ttatataaga tgtattttgt ttttgtgggt tttttgtagt ttttaagttt 3060
   ttatttgatt tgtatataat gatttagtgg gttagaggag tttagagtta tgagtggttt 3120
   atttttttaa gaattatttt tgattgttta gtattatgga gtaagtggaa agaagaaaaa 3180
   aaaaatttaa ttattttttg aagagtaaga tgaatgttgt agaaggagaa ggaaggggag 3240
   ggagatggat gggtgttgat tttagaattt ttagatttgt ttggatgaat tattatttat 3300
   gatttgtggg ataagaattt gattttattt attaattagt agaaattttt ttttttgttt 3360
   tttaatattt gaaatttaat aaatatgtgt tttaggaata tattggttat tttttagagg 3420
   tattttatat atatattgag taattagaaa atattttttt tgtaatatat atatatatat 3480
   atatatatat atattatttt gttatataat atttttatgt aagaaaagtg aaattgttgt 3540
   ttgatgaatg taaatatttg gttgtttgta gtagttggga gttttgttag gtttaagtgt 3600
   taagatggga ggtgaatttt aggggttttt ttttgtttgt gttgagattt ttatttggtt 3660
   aagtttttat ttatgttttg gttttggagt gagtttgata gtgttggatt atagtagagg 3720
   gagtgaggtg gttgatgttt tattttgaag agatgaatgg aattttagga agttagagtt 3780
   atgttggttt gggatagtgg tttggagatt agattttaat gatagaagta ttaggtagtg 3840
   gtatttatgg taatgtgtgt atttatagaa atgtaattta tattttgggt ttaggagttg 3900
   aaaaatgaaa agaatgttta gggaggaaaa agggaaatat aataataggt agagagtaat 3960
   ttattatttt atgggtttgt tttgtaaata gttgaagatt ttggagttag atggttttgt 4020
   aaatttttta aataggagtt atgttaagaa gtatgagtgg gtataaaaat tgttttttaa 4080
   gatataattt taatttggtt tgtggaaatt ggatatgagt aagttttttt aaaatttgag 4140
   tagaaagtag ttgttttttt tgggtttttt gatgagaata tgtatattgt ttttaagtgg 4200
   ttggttgagg gagtgttgtg gtagtgggag aggtgttttt gtgggttttt tggtagttgt 4260
   ggtaggaaag ggtttgaagg tagtgaaggt gaatgtggtg tattaatttg ttggttttgt 4320
   tgatgttgtg tttatttttt tttggggtgg gtgtggggtt agtttaggat aggtgtttgg 4380
   gggatgtgtg tttttatttt atggggatgg tggaggagag ttagtgaggg tttgaggggt 4440
   aggtatttta atgaatggtt tttttggtgt tttttgtgtt ttgttggttt attttttttt 4500
   ttataaaatg ggtttagttt ttagtattta ttttttgtta ttaattaggt attttgggag 4560
   attagtttgt ttgaaagttt ttgtgttatt ttgtgggttt ttttaggtgg tttttttagt 4620
   tttgtttttt tttgggatgt ttgttgatta ttttgagttt gtgtggtgta agagtatgag 4680
   tgttgagttt gtgtgtgtta aggttgtgtg ggtgggtatt gatttttttg agaagtttta 4740
   gtgtttttaa gttttgattt ttgttttttt tattttttag ttggaaagtt gtgtgttagg 4800
   tagtgggggg tggagagagg agtttagatt ggtttttatt ttttgttttt tgtttggttg 4860
   ttgtttattg gttggaggaa tttttaggaa tgtgagtgtt tttttaaaag tgtgtggttt 4920
   ttttgttttg ttagttgttg tgtttgagtt ggtttgtgag tttagggttt ttgttgtttt 4980
   ttaggagtaa tttttatttt ggatgtatag gtattttttg tgttttttta gtttttgttg 5040
   tttttgttat tgtttttggt tgttgtgttt tggtatatat aggtaagttg tttttggtgg 5100
   ttgttgagga ttaaagttgt ttgggatatt tatttggagt gttgaggttt tagttttttt 5160
   ggtggatttt ggaatttata ttttttttgt ttgtttattt tagtttgttt tttttagttg 5220
   ttggaggatt ttttagggta aggttttaga gttatttttt ttagttttga ggtttataaa 5280
   ttaatttatt aggatatttt aagatttttt tattttttga agtttttttt aagtttttgt 5340
   attagtattt tagggaagag tttgtatttt ttttgttttt tttgtaattt taaattatag 5400
   tttttgattt tgtttatttt tgatttttta aaagttttta aattgttggt tttgtgtttt 5460
   ttatatttta aaattagtat tttttttttt tatttttttt tttttttttt tttttttttt 5520
   ttatgttttt tggaaagtaa ttttgttgtt ttttatttta gtaaatgtgg ggttaaagag 5580
   ggatggtttt ggttttgttt ttatgtttag ttttgttttt gtgtattgtt tttttttgtt 5640
   tagttgattt ttgatttttg gtttttgtgt tttttgttat agg 5683

<210> 366
<211> 5683
<212> DNA
<213> Artificial Sequence

<220>
<223> chemically treated genomic DNA (Homo sapiens)

<400> 366
   tttgtagtag gaagtataag agttgagggt tagagattag ttaggtaggg agggatggtg 60
   tgtagaaatg gggttgggta tggggatagg gttgggatta tttttttttg attttgtgtt 120
   tgttgaaatg aaggataata ggattatttt ttagagagta tgagagagag agagagagag 180
   agagagagag gtggaggaaa gagatgttgg ttttaaagtg tggggggtgt aagattaata 240
   atttgggggt ttttgggaag ttgaaggtga gtaagattag gaattgtagt ttggggttgt 300
   agggagggta ggggaagtat agattttttt ttggagtgtt gatataaagg tttaaggagg 360
   attttagaga gtaaggaaat tttggggtgt tttgatgagt tggtttgtga attttaaggt 420
   tggagaggat ggttttggag ttttgttttg aagagttttt tagtggttga gaggagtggg 480
   ttggggtagg tgagtgggga gagtgtaggt tttggggttt attagaggga ttgaagtttt 540
   agtgttttag gtggatgttt tgggtagttt tggtttttgg tggttgttgg gggtgattta 600
   tttgtgtgta ttggagtgtg gtggttggga gtggtggtga gggtggtgag ggttggaggg 660
   gtgtggggaa tgtttgtgtg tttggagtag aggttgtttt tggagagtga taggagtttt 720
   gaatttgtag gttagtttgg gtgtagtggt tggtaaggtg gaggagttgt gtgtttttaa 780
   aggggtgttt gtattttggg ggattttttt ggttaatggg tggtggttgg gtaggaagtg 840
   ggaggtgggg gttagtttgg gttttttttt ttgttttttg ttgtttggtg tgtaattttt 900
   tagttagaaa gtgaaggggg tgggggttgg ggtttgggag tattagaatt ttttagaaaa 960
   gttggtgttt gtttatgtag ttttggtgtg tatgggtttg gtgtttgtat ttttgtgtta 1020
   tgtgggtttg gggtgattag taagtatttt gaaaagggat ggggttgggg agattattta 1080
   ggagggtttg tggggtggtg taggggtttt tgggtgagtt gattttttgg aatgtttggt 1140
   tgatggtgag aggtggatat tagagattgg gtttgttttg taaaaagaaa aatgggttga 1200
   tggggtgtag gggatattaa gagagttatt tgttaaagta tttgtttttt gggtttttgt 1260
   tgattttttt ttattgtttt tgtggggtga ggatatgtgt tttttgagtg tttgttttga 1320
   gttggtttta tgtttgtttt ggagggaggt gagtgtggtg ttggtggggt tggtaggttg 1380
   gtgtgttgtg tttgtttttg ttgtttttgg gttttttttt gttgtggttg ttagggggtt 1440
   tatagagatg ttttttttgt tgttgtggtg tttttttggt tggttgtttg ggggtggtgt 1500
   gtgtattttt attaaggggt ttggggagga tagttgtttt ttatttgaat tttaaggaaa 1560
   tttatttgta tttagttttt ataagttaaa ttgaaattgt gttttgaaaa atagtttttg 1620
   tgtttatttg tgttttttaa tgtgattttt gtttggagaa tttgtagaat tatttggttt 1680
   tagagttttt agttatttat agagtagatt tatagagtaa taaattattt tttgtttatt 1740
   attgtatttt tttttttttt ttttaaatgt tttttttatt ttttggtttt tgaatttgag 1800
   gtgtgggtta tatttttgtg ggtgtatata ttgttatgag tgttgttgtt tagtgttttt 1860
   gttattgaag tttggttttt aagttattgt tttaagttag tatgatttta gttttttgga 1920
   attttattta tttttttggg atgggatgtt agttgttttg ttttttttgt tgtgatttag 1980
   tgttattggg tttgttttga ggttagggta taggtagaag tttgattaaa taaggatttt 2040
   agtatgggta gggggaaatt tttggggttt attttttatt ttagtattta aatttggtag 2100
   gatttttgat tgttgtaaat agttaagtgt ttatatttat taaatagtag ttttgttttt 2160
   tttgtgtggg agtgttgtat gataagatgg tgtgtgtgtg tgtgtgtgtg tgtgtgtatt 2220
   atggaaagag tgttttttag ttatttaata tgtatataaa atgtttttaa aagatgattg 2280
   gtatgttttt aaggtatatg tttgttggat tttagatgtt gggaggtaga aagaaaagtt 2340
   tttattggtt gatgaataaa attagatttt tgttttgtaa attataggta atgatttatt 2400
   taagtagatt tgaagatttt ggaattggta tttatttatt tttttttttt tttttttttt 2460
   tttatagtat ttattttgtt ttttgaaaag taattgggtt tttttttttt tttttttatt 2520
   tgttttatga tattggatag ttagaaatag tttttggagg gatgggttgt ttatggtttt 2580
   gggttttttt ggtttattgg attattgtgt ataaattaga tgggggtttg agggttgtaa 2640
   gaggtttata aaagtagaat gtattttgta tgaaaagatt atttttttta ggtttagttg 2700
   tgtagataga gtagaaatta gtatttattg tttttttagg atttatgttg ttatggttga 2760
   gggattgtag gaatgttttt gtagattttt tggaatttgg ttttttttaa taaaataagg 2820
   ataatagttt ttaaatttta ttatgtttta ttaataagaa ggtgatttat atggtggtag 2880
   gaaagagttt ttttattaga gaaagttgat aggtatagga gttgtttatt ttaaatataa 2940
   agtttttttt tttattatga tattttagtt gttttgggtg gaaaggaaag gttatagaag 3000
   agggagtgtt tgtgtttttt aataggaatt tatttatatt tttgttagtt aatatgtgtt 3060
   gtaaatattt tttaaatttt atatattttt gtgtagtttt gagagaagta gggatgggaa 3120
   aagggaaaag aaaatttatt ttttttaatg tgagggtaat agtttatttt gagtgtgttt 3180
   ttataatttg gaagtattgt gattttaagg taattaatta atatgggttg aagtgatgga 3240
   ttttttaggg agttggttta gttttttttt ttgttaagtt gttatttttg aggattagtt 3300
   attgaaaatt gtaaggtata tggtatttta ttgtaggatt tttttttttt aagttttttt 3360
   aattggaggt gggattgatt taaatatatt ggtagtaagt gtgaggaaag ggttgttagt 3420
   tatagttagt tttaaaattg tgtgtaatta aggagagtaa tataattgag aaaaaatatt 3480
   ttgtttattt agtatagaat agtaaggtgg gttgtgattt tgtgtaggaa ttttttaagt 3540
   gttgtaaatt tttagtagtg gaatattttt tataagtgtg aagtaatgaa aaattattgt 3600
   attttagaat agtttaaaat tatagtattt ttaaattgaa gatattataa atttattttt 3660
   ttttgaatat gttttttaga ttataatttt aagttgtttt attttaattg tattagaggt 3720
   tagagttagg aggaagttat taatagattt tttttgtttt ttaattaaat attaatttaa 3780
   gttttttttt ttttatgagg tgttttttat ttgttttatg ttgtgtgttt taaagtatat 3840
   attattttta ttataaaatt tattatttaa tattttttat tatttagttt ttaaaaatta 3900
   gtttttattt ttttatatgt aaagattaga attttttttt ggtggggggt gggatggagt 3960
   tttgttttgt tttttaggtt ggagtgtagt ggtataattt tagtttattg taatttttgt 4020
   tttttagatt taagtgattt ttttgtttta gttttttgag tagttgggat tttaggtgtg 4080
   tgttattata tttaattaat ttttgtattt tttagtagag ttggggtttt gttatgttgg 4140
   ttaggttggt tttaaattta ttattttagg tgatttattt tttttggttt tttaaattgt 4200
   tgagattata ggtgtgagtt attgtgttta ggtaaggatt agatttgaag tgtttaaagt 4260
   tttgggtttg ggaagagaga gtttttatgt ttaaattttt aaattttttt gtgtataagt 4320
   taattatggt atagaaggat gggattattt attatttttt tattagtttt attattttat 4380
   ttagttattt tttagttttt ggtgggtaaa tgtggtgagt gatttataaa tattgtattt 4440
   ttttttaaag aattgaagta atatttttat ggaattgatt ttagtgggta ggggtaggta 4500
   tattggttaa gggtaaatat tttttttttt tttttttatt tttttgttag tgattgattt 4560
   aggaattggt atgtgattta aatttgatga ggtttgttga aggtttttgg gaaaagtttt 4620
   ttgttgtttt taaggaagag ttatgtgaaa ttaggttttt tttatttttt ttggatatta 4680
   ataagaatgt tgataagtgt tgtttggtgt tattggttat tggggagtta agtttagtgt 4740
   gaaattagtg ttggaggagg aagaataaaa gtatagaaag aatttgtatg ttaggttata 4800
   tttttgagtt gttaaatttt ttaattttta agggaatttt atttttagat ttttttggtt 4860
   atgtgtttat tatttaagtt aagttgttgt tttttatttt ttagaattgt agatattata 4920
   atggtttttt tttttgtgtt tggatggttt ttttatttat gttttgatta aatttttttt 4980
   tattttttta gaataagaga agtaaggata ggttatagga taaatatatt gtaaaggttt 5040
   tttatttata ttattaagat gttttttaga aaatttgtat tatttattgt tttgtttatg 5100
   gtatgaggta gttttttttt ttattttttt gtttgtagtt attttagttt tatatatttt 5160
   taattgtagg ttggtaaggg tgagtgttat ttttgatttt attgtgtttt tgtatttttt 5220
   ttttgggtaa ttttgagatg tagtttagta ggtttataaa gaaaagaata attaggatgg 5280
   atggatttta gtgaaggggt aggggtattg atttgttagg aaattaaggg tatggaggaa 5340
   agaagatggg aaaaggagat ggtgaggttt tatattgggt tgaaagatgg aaatggggaa 5400
   gattagtaag gtaggaaggt gaggatatgg agaataggtt tttaatatgt aataatgata 5460
   aatttttaaa agttgttttt ggtaaaagtt ttttttatat tttggatgat aggtgtaggt 5520
   tattgtagtg ttgttgatgg ggaggattgt atttgggagt agtttttttg gttttattaa 5580
   tgtttatttt ttattatttt agtaaaaaat gttattttat ggggtatttt aaatatggtt 5640
   tggtttgggt tttagtgtgg aagttttttt aagtgtttgt tat 5683

<210> 367
<211> 7833
<212> DNA
<213> Artificial Sequence

<220>
<223> chemically treated genomic DNA (Homo sapiens)

<400> 367

gtttttggtg agatatgtgt tttataagtt ttaatggaga aaaatgtaag tattttattt 60
tttgaaattt ggttatttga gtaatgagaa aatagttatt ttttttagga tagtggtttt 120
taattatggt tatgtgtttt tttaggaaaa ttttaaaaat atatatatat taatgttttt 180
gtgttatttt tagggatttt aagtttttga atatgaattt tgtattagta ttttttaatt 240
atttaggtga ttgtgatgtg aaattatgat tgagttttat tgttttaaga tgaaataaat 300
tttttttagt attgaaatta taaatttaaa ttattaaaat taattaaggg tatgggaatt 360
aataaggtat agggaagttt ttatattata aaattatttt tttaaattat agtttattgt 420
ttatatgtta tttgttattg tagaaaaggg tgaaaaaata gtaaatttaa ttatttttag 480
tttgaaaaat tatttagaaa tgaagatgat gattttgaaa tattgttaat attatttgat 540
ttataaataa tgttttaata tatttattat atattgatag atattttttt atatgaatat 600
tatatattaa aattaaggta ataatgtatt tagaatattt tatttatatt tatgtatttt 660
aagtaggtta gaaattaaga tatgagttat taagtatgag atgttaaggt gtggggttag 720
aaattatatt gtattttatt attaataatt aatatatatt ttaatattat atatatttaa 780
ttttaatttg tatattttta attattttta attatgtgta taaatataag tatatatatt 840
tttatgtatt tatttattta tatttttatt tatttattta tataggggat ttttttaaat 900
ttattattat taaattatat atttttattt taatttttag aataagttta ggaggtaggt 960
attgttatta tttatatttt ataaatgagg aaattgttta tagttataaa gttattgtgt 1020
tagatatatt agaagtttaa tatatatttg gtgaatatat gtataaaaat agagagatag 1080
atatgtataa tagtttattt ttatattgag taaaagtttt taatttgttt tagaaatttt 1140
tttgtgaaaa ttgagtaaaa attgaggtat ttttttattt gttatatagg tataggtggt 1200
attttatttt tttaataagg atgaatattg aaatgtggat tttaaggttt aattttagat 1260
tttttgaatt tttgatagtg ggatttggaa tttgtttatt gttttaaagt tttttaagga 1320
atttatatga ttaattaggt ttagaaatta ttggatttta ttgttgaagt ttgagaatta 1380
aagtttgggt tttattgtgg ttttatagaa agggtaaatg aagtattatg gatagaattg 1440
atatgttttt agttagtttt tttttttaga agttaatagg tagtaatata gtagaaatta 1500
gtgatttatg ttttgtgttt tgaagttagg tagaatttta tagagtttta gtagtgttat 1560
tgatgagatt tgttttttgg ggtaagttgt ttgatgtttt taaagttata ttttttttat 1620
ataaaatgag ataatatttt ttgttttata ggggtgtttt aaagattaaa taaaaataat 1680
atgttttatt ttatatggta taatgtttga tatttaagaa gtaaaggata tattttattt 1740
ttattgaagt aattagaaag tatgaaatta tgaaggagat aagagttttg attggtagtg 1800
tattttattt ttttaggttt atttatttat tttaaattat ttttgttgga gaataatttt 1860
taagtttttt atttaagttg tgagtaattt tatattttat aatgatgttt tttttatgag 1920
aaaaaaaaat gtttttaagt tttttggaga aaatatattt gtattatttt tattgaaaaa 1980
tttaataatt ggattttgtt tttttgtatt aattttagag tgtatatgtt ataaataaag 2040
tgttttagtt taagaagatt gaaagtaaat atggtatagt attttaaaat aagaattttg 2100
taaatatatg gtatgattgt gttatattat tagtaattat atgatatgta atgtaaagta 2160
tagtttatag atttaaattt aattttaata agtaaattga ttttgttttg ttggggaaaa 2220
gttaaagtat taatttaatt gttaatgtag ttttgtttat ttttttggta tttagtgata 2280
agtttaaata atgtatatat ttttatttat atatttagta atataatttt ttgtttaatg 2340
agtgatgttt ttttgttatt tggtggtgtt tgttagtttt agaatttgtt ttttggtggt 2400
attataatat taagtataga gtaagtgtaa taaaattgta gtatttttat tgaaaaggtt 2460
ttgttttaaa ttgtttaata atttaaagga tttttgtgga agtaattgta tttgttaatt 2520
agttataatt agtaattaat ttttttggag ttttaattta tttttggtaa aatgttttag 2580
gaagagtata tattattaga aagtatgtta aaaatttatt tagtagaaaa tttaaaaata 2640
gttttttttt gttaagaggt tttttaaaat tttatttata tagttaaatt ttgaaatttt 2700
agtaggtttt gttttattat tataattatt gtataaatat ttttaaggat tttgttttta 2760
gttttaagta tgatttattt ttataagttt gattagttat tatattagtt ttgttatgga 2820
aaatgatatg tttttatttt ttgttgtaga gttgttaaat tttgatttat atttatgttg 2880
ttttttttgt tgaaagtttg tagtgaaaga aatttttaat tttttgtttt gtaatattag 2940
ttggtagttt tatttaatgg gtattttgtt tttttaaaga atttagttgt tttgtttaga 3000
agttgatttt ttgatgtttt taatgtttgg tttaattgat ttgttttaat ggagtttttg 3060
ttggtgagga gtgagatgtt attgattaga atgttgggat ttgttgttta attgttagga 3120
gtgagagata ttgagattta gaaatttttg gaggtgggag gggagaggga tagttttgga 3180
tggaggtgga gatgtaagat aaagggatgg attttatata ggaaaaaaaa aaagattttg 3240
ttgaggtatt gaggtgttgt atgattatat tttttaaagg agaagttaaa aagtaaggaa 3300
gtgggaggag gttggaggtt aaagtattta aaaggattat ttgggtataa tttgtttttt 3360
tgttggtgtt tgtaaaggat agatagtttt gtttttaaag tatatgaatg ttttttttaa 3420
gtgattggga atggatatta attgtttgtt aaatgttatt aaatgttttt ttaaatttag 3480
gggatataga aagaggggta taaaaggaga atttaaatag aaaaagggag gatttggagg 3540
tttttgaaag tggggggaga agaaggagga gggataatag agaggaatag agaaggagag 3600
tggagagaag ataaataaaa ataaaaatag gaattattga ataattatat attaaaaaga 3660
aagttttttt ttatggggta tttaaaatat tgagattgta atagtgattt tggttatgga 3720
agaaagatgt ttttttttat ttttgttttt gaaagttttt ggttttgtta ttggtgatta 3780

aaattttatt aggttaaaga gtgtgtttaa ttgtttgaag aatgtagtag atggaaggtg 3840
ggttttgtta tgttgtttgt tttttttgtt ggagagaatg aaagaaatgt gtagagttag 3900
agatttttgt tgagttagat ttttttttgt tgttttaggt tattggttat ttggtaaaga 3960
tttgagtaag gaatgtaggg ttattgtttg ggttaataaa tggagtttgt tttttttttt 4020
ttggatgttg ttgtttggtt gatgtttttg gtaatttatt tgtggtgtat gtagaggagt 4080
tttttttttt tttttagatt atttgttttg attaatttga ttttttaaat atatttgatt 4140
gtatttttta ggtggatata ttaataggtt atgggttgga gaggagtggg tgatgaggag 4200
agggatttaa atttgtgaat gtttgggttg ggttggagtt gtggggggtt tgggaggaga 4260
gaggggagaa gagagaagga aggagagtgt ttgttgggat ggttgagttg ttttggtgag 4320
tagttttggg gttgtatgtt tttgtgggag atgttgttgt tgtttttagg ttggtaagag 4380
tggttttaat attattgttt tttatttttt tttttgtaaa tttttagaga aatgtttttg 4440
gtttttttgt tgtgatattt ttagtttgta tttttttata gtttaggtgg tgtgtttttg 4500
tatgttggag tgttggttgt tagtaggatg tttttttttg tgttgatttg tttttttttg 4560
ttttgttgtt gttgtttttt tgatattttt gtttttatta tttttagttt ggagagatgt 4620
tatttagttg tggtttgtat ttgtggtttg gggttatgtg tggaagaggg gtgttagttt 4680
ggattttgtt tttggtaggg ggtgttttgg agtggagagt gaggtgaatg gtatatgagt 4740
gtgtgggtag tttattttga agtttgagtt ttttatttga gttatgtttt gtttagtttt 4800
atttgggtta gtgtttggtg agtgagttta tttgtggttt ttgtggttgt tttttttttg 4860
tatttttgta tttatttgtt gatttttttt ttttgggatt tgtattttgt tttattaatt 4920
agagtttgat tgtttttttt tatgtgattt tgggtgggtt gaggatttgt tgttttttaa 4980
atgttagagg gatgtgggtg gtagagtttg agaggtggtt gttgggttgt ggggtgtttt 5040
gatttttttt ttattttgtt tttttgggtt ttatttgttt gtttttggat ttttgttttt 5100
ttttgttttt tggtttttta gagttttttt tttatggtag tagttttttg tgtttttggt 5160
gtagtttttt agtggatgat ttttttgttt tggggttgag tttagttttt ggatgttgtt 5220
gaaatttttg agattatgtg tgggtttggt tgttgttttt ttgttgggtg ttattgttat 5280
tgttgttgtt tttgttgttg ttgtttgtgg gatgtttagt agtttgttgt ttggtttttg 5340
tgattttgtg ttttttggaa gttgtttgtt gttgtagagt tgtatgaatt agttatggtg 5400
ttgtgggagt ttttgtggta gtgtagtagt tggatatttt gtgagggttt ttgttggttg 5460
ttgttgttgt ttgttatgtt atttattgta gtttgtttgg tgaagtttgt tgtttttttt 5520
atttttttaa gtgattgtta aatgtttatt ggttggaatt gttttggtaa gtttagaatt 5580
tttgtttttg attttttaat tttgtagaag aatatgtgta tttagtatag attagtttat 5640
tttagtgtgt ttttttagtt ttttattttt tattgtttta gatttttaat attatttatt 5700
tttatttaga gaaataaggg gaattgttgt aggtttgggg gtgaggggtg gttttgggat 5760
gggtagaaag tgtaggtgta gtaggaaatt tttgtatgtt tgtgtttata ttggagttgt 5820
gaggattttg agaaatatta aatgggatgg ttttttgggt ttattgtttt gaaagagtat 5880
taattttagg ggaaatattg aaatagaagt tttgttatta ttaaagaaaa aagttttatt 5940
aggatgagga agaaataatt ttatgagaaa gaatgagtga gaaagtaata aattaaatgg 6000
tgattgtagg ggaattgttg atttttggta aaggtgttat gaggttgtat tggttttttg 6060
ttgaagatta ggttatatag attttagagg agttgggttt taatagaatt tttttttttt 6120
tttttttttt tttttttttt tttttttttt tttttttatt tatttatttt tttttttttt 6180
ttattttttt tttttttagg tggtaaaaga tattggtttt gtagtttaga tatgtttttt 6240
tttttgtttt tttaagtttt aaggtagtat aggggagttg agaaaaagaa tattttgtgg 6300
gttttttagg ttggagtggg tatgattgag gttggttagg ttttatgtag gtgagttgag 6360
ggtggaattg attttagtgg gtgttgattt ttttattttt ggataggttt ttgtggagtg 6420
ggttaggtat tttttttgtt tgtttgggtt tttttagatt ttgatggtga atgtttggta 6480
ggttttgttt tgttgaagtt ttttaattaa atagggttag aggatgggag ttgttgtatt 6540
tttagttggt atagtatttg gtttgatagt ttgtagtata gggtgtatgt aattttttat 6600
tttttgtgaa tataattttg ttgtagttaa atttggtttt gaataaagtg ttttttaaag 6660
atgtatataa gttgaagtgt atgtaatttt agagaggagg gaatgattaa ttgtaattta 6720
gggtgaaagt ttgtatagtt tttagttatt attgatgtaa atgttaaaag gaaaattatt 6780
atgtattatt ttaatttatt ttttataaag ataagttgag atatgtaatt ttattagatt 6840
tgggttaata gattgttttt ttttttggta gtttttaaat ttggtatttt aataaaattt 6900
aatatgtttt tataattttt tgatttatgt gtatatgtgt gttgtttttg aaagaataag 6960
ttttattttg ttattgttta attatttttt agatgtttta ttatggtaat aattatgagt 7020
ttgtaaaaat aatttttgga aatgttgatg gttttgtagt ttaatataga ttggtttgtt 7080
ttatttttag tttttgtatt gttttaggaa ataattaatt taaatgtgaa gttgatattt 7140
gtaattaaga aattatatat ttattagata ttttaaaggg gattgtataa attaaagaga 7200
ataaattggt tttgtagata ggttgttaag aatttggtat tttgttttta tttttgttaa 7260
tttagaggtg attaattttt atttgagtta aatagattat tatagaaaat attgtgtttg 7320
tttattttta ttattgaggt tttgtttttt ttttgtttgg atatatttta aataaggggt 7380
tgttttagtt gttgaagtaa aagaataatt aaagatgggg aaatggtaaa agggtattta 7440
gagattatta ttagtttttt tttaaaatgt ggagttttgt ggttataaat attgtttatt 7500
taatgagtaa aaaataaaaa taaaaaaaaa ataggaagta aatgttaagt ttttatttat 7560

tattgttagt attaatgtaa gttttaaaaa atagtattat tagaaaagga tattaaagga 7620
gaattgatta gaaaagaatt gtggaaaatg gaaatgaata ttgattattt aattagattt 7680
tgaggttatt agtagatagt gattttgtag tatagttata gttgttggat ttaaaattta 7740
ggataagtat tttaaagttt taaagtagtg tttttttttg ttaaaaattt gtaagatgtt 7800
ttaatgattg gagtgttttt tttgaatttg agg 7833

<210> 368
<211> 7833
<212> DNA
<213> Artificial Sequence

<220>
<223> chemically treated genomic DNA (Homo sapiens)

<400> 368

ttttaaattt aaagagaata ttttagttat taaaatattt tatagatttt taataaaaaa 60
aagtattatt ttgaagtttt aaaatatttg ttttaaattt taaatttaat aattatagtt 120
gtattgtaag gttattgttt attgataatt ttaaaattta gttaagtgat taatatttgt 180
ttttattttt tataattttt ttttagttaa ttttttttta gtattttttt ttgatagtgt 240
tattttttaa agtttgtgtt aatattgata gtggtgaatg aaagtttaat atttgttttt 300
tgtttttttt ttatttttat tttttgttta ttaggtggat aatatttatg attataaaat 360
tttatatttt ggaaaagagt tagtgatgat ttttgaatat ttttttatta tttttttatt 420
tttaattgtt tttttgtttt aatgattgaa ataatttttt atttgaaatg tatttagata 480
aagaggaaat aaagttttaa taataaagat aaataggtat agtgtttttt gtgatggttt 540
gtttggttta aatgaagatt gattattttt aagttaatag gggtggaagt ggggtgttaa 600
gtttttgata atttatttgt aaaattagtt tatttttttt agtttatgta gtttttttta 660
aaatatttgg taaatatgta attttttgat tgtaaatgtt aattttatat ttaagttagt 720
tattttttaa aataatgtaa gggttaggaa tgaagtaaat tagtttgtgt tggattataa 780
agttattaat atttttaaaa attgtttttg taggtttata attattatta taataaagta 840
tttaaaaagt gattaggtaa tagtaaagtg aaatttattt ttttaaaaat aatatatatg 900
tatgtatgaa ttaagaagtt atagaaatat gttgagtttt attaaaatgt taaatttaga 960
aattgttaaa aaagagaata atttattgat ttaaatttaa tagggttgta tattttaatt 1020
tgtttttgta aaggataaat tagaatgatg tataataatt ttttttttgg tatttatatt 1080
agtaataatt aggaattata taggttttta ttttgagtta tagttggtta tttttttttt 1140
tttaaagtta tatatatttt agtttatata tatttttgaa agatatttta tttagagtta 1200
gatttaatta tagtaaaatt atatttatag aagatgaaaa attatatata ttttatatta 1260
taggttgtta aattgaatgt tatgttagtt aggagtgtag taatttttat tttttggttt 1320
tatttaatta ggaagtttta gtagagtgaa gtttgttaag tgtttgttgt tagaatttga 1380
aggaatttga gtgagtaaga agagtgtttg atttatttta tagaagtttg tttagaaatg 1440
gaggagttag tgtttattga agttggtttt gtttttggtt tgtttatatg gagtttgatt 1500
agttttagtt atgtttattt tggtttggga gatttgtaaa gtgttttttt ttttaatttt 1560
tttgtattat tttgaagttt agggaagtaa agagaggggt atatttggat tgtaaaatta 1620
atgttttttg ttgtttagga gagaagggaa tgagagagag agagagatag atagatagag 1680
agagagagag agagagagag agagagagag agagagagag agaaatttta ttgaaattta 1740
gtttttttag aatttgtgtg atttggtttt taatgggaga ttagtgtgat tttatggtat 1800
ttttgttagg aattagtgat ttttttgtag ttattatttg atttattgtt tttttgttta 1860
ttttttttta taaagttatt ttttttttat tttagtaaga tttttttttt taatgatgat 1920
aaagtttttg ttttagtgtt ttttttagga ttggtgtttt tttaaaatag tgaatttaga 1980
aaattatttt gtttaatatt ttttaaaatt tttgtagttt taatgtaagt gtaagtatgt 2040
aaaggttttt tgttatattt gtattttttg tttattttag aattattttt tatttttggg 2100
tttgtaatag ttttttttgt ttttttggat agaggtgggt ggtattaggg gtttagggta 2160
gtaggaggtg aggggttgag gaggtgtgtt agggtaggtt ggtttgtgtt ggatatgtgt 2220
gtttttttgt ggagttaaag ggttggggat gggggttttg gatttattag agtaatttta 2280
gttggtgggt gtttggtagt tatttaagga ggtagggaaa gtagtgagtt ttattgggtg 2340
ggttatgatg agtagtatga tgggtagtag tagtagttag taaaagtttt tgtaaagtgt 2400
ttagttgttg tattgttgtg gggattttta tagtattatg attagtttgt gtaattttgt 2460
agtagtaaat ggtttttgag gaatatagga ttgtgggggt tgggtagtgg gttattgagt 2520
attttgtgga tggtggtagt agaggtggtg gtggtggtag tggtatttgg tggggaagta 2580
gtagttaaat ttgtgtatga ttttgagagt tttagtaata tttagggatt gggtttagtt 2640
ttggagtgag agggttgttt gttgagaagt tgtgttggag atgtgggaag ttgttgttat 2700
aaggagggag ttttgggaag ttggaggata ggaggagatg ggagtttagg ggtagatgag 2760
tggagtttga ggaggtaggg tggagggaga gttaaggtgt tttgtagttt ggtagttgtt 2820

ttttgagttt tgttgtttgt atttttttgg tgtttgggaa gtagtaggtt tttagtttgt 2880
ttggggttat gtgggaagag gtagttgggt tttgattggt ggagtaggat gtaggttttg 2940
ggagggaggg gttgatgagt aggtgtaagg atgtaaggag gaggtggttg tggaagttat 3000
agatgggttt gtttgttagg tgttggtttg agtggggtta ggtggggtat ggtttaaatg 3060
agaagtttgg gttttagggt gggttatttg tatatttata tattatttgt tttatttttt 3120
gttttaggat gttttttatt gaaggtgggg tttggattag tgtttttttt ttgtgtgtga 3180
ttttgggttg tgagtgtggg ttgtggttgg gtggtgtttt tttgagttgg agatggtggg 3240
ggtggaggtg ttagaggagt agtagtagta gggtagagag gggtgagttg gtgtgggaga 3300
gggtgttttg ttggtgattg gtgttttagt gtgtgggagt gtgttgttta ggttgtaggg 3360
ggatgtaggt tgggaatgtt gtggtggaga ggttagggat gtttttttag ggatttatag 3420
gaaagagggt gagaggtgat ggtgttagaa ttgtttttgt tgatttggaa gtaatagtag 3480
tattttttat aagagtgtgt aattttaagg ttgtttgttg aggtagttta gttattttgg 3540
taggtgtttt tttttttttt tttttttttt tttttttttt ttaggttttt tgtagttttg 3600
atttagttta agtgtttgta ggtttgaatt ttttttttta ttatttgttt ttttttagtt 3660
tgtagtttat tagtgtgttt atttgggagg tgtggttaga tgtgtttgga aggttagatt 3720
ggttgggata agtggtttga gagaaagaga aaggtttttt tgtatatgtt gtgggtgggt 3780
tgttgggagt attggttggg tagtggtgtt tgggaagggg agagtgggtt ttatttgttg 3840
gtttaggtag tgattttgtg ttttttattt gggtttttgt tggatggttg gtgatttggg 3900
gtgatgagag aaggtttaat ttggtaggag tttttggttt tgtgtgtttt ttttattttt 3960
tttagtggga agggtaaatg gtatagtggg atttgttttt tgtttgttgt attttttagg 4020
tagttagata tattttttag tttaatggaa ttttagttgt tagtaatggg attaagagtt 4080
tttggggata agggtggaga ggaatatttt ttttttatga ttggggttat tattgtagtt 4140
ttagtgtttt ggatgtttta tagggaagag tttttttttt ggtgtgtgat tatttagtga 4200
tttttgtttt tgtttttgtt tatttttttt ttgttttttt ttttttattt tttttgttat 4260
tttttttttt tttttttttt ttgtttttaa aagtttttgg attttttttt tttttattta 4320
aatttttttt ttgtgttttt ttttttgtgt tttttgaatt taggagagta tttgataata 4380
tttaataggt aattagtgtt tatttttaat tatttaaaag aggtatttat atattttgaa 4440
aatgggatta tttatttttt gtagatatta gtagaaaaat aaattgtatt tgagtaattt 4500
ttttaagtat tttaattttt aatttttttt tatttttttg ttttttaatt ttttttttga 4560
gagatgtgat tgtgtagtat tttagtgttt taatgaaatt tttttttttt ttttgtgtga 4620
aatttatttt tttatttat attttttgttt ttgtttgaga ttgttttttt tttttttttat 4680
ttttaaagat ttttgaattt tagtgttttt tatttttggt aattaagtag tagattttag 4740
tattttagtt ggtggtattt tgttttttat tgatgaagat tttattaaaa tagattaatt 4800
agattagatg ttggaggtat tagaaaattg gtttttagat agagtagtta aattttttaa 4860
ggaaatagaa tatttattag atagagttgt taattaatat tgtaaaataa ggaattagaa 4920
attttttttg ttataggttt ttagtagaga aggtaatata aatatagatt aagatttaat 4980
aattttatag tagagaatga gaatatgtta ttttttatag taaggttggt gtggtaatta 5040
attaggttta tgaaaataag ttatgtttga aattaaaggt aaagttttta aaagtgttta 5100
tgtagtaatt atgataatga aataggattt gttaggattt tagagtttgg ttatgtaagt 5160
agaattttag agaatttttt agtagaggaa aattgttttt gaattttttg ttaagtaaat 5220
ttttggtata ttttttaata atatatgttt tttttaagat gttttgttaa aagtaagtta 5280
aaattttaaa ggagttaatt attggttgta attggttaat aaatgtggtt gtttttatag 5340
aggtttttta aattattaaa tagtttgaag taaagttttt ttaatgggaa tgttgtaatt 5400
ttgttgtatt tattttgtat ttagtgttat agtgttatta agaaataaat tttgaaattg 5460
gtaagtatta ttaagtggta gaagaatatt atttattgag tagagaattg tattattgaa 5520
tatgtaaata aaaatatata tattatttag atttgttatt aggtattaaa gaagtagata 5580
agattgtatt agtaattgga ttagtgtttt aatttttttt tagtaaggta aaattagttt 5640
atttattaga attaaattta agtttatgaa ttgtattttg tattgtgtat tatatgattg 5700
ttagtaatat gatataatta tattatgtat ttgtaaaatt tttattttaa aatattatat 5760
tatatttatt tttaattttt ttgagttaga atattttatt tgtggtatat atattttaga 5820
attgatgtag aggagtagag tttagttgtt agatttttta gtagaaatag tgtagatata 5880
ttttttttag aaaatttaag aatatttttt ttttttatgg aaagaatatt attataaagt 5940
gtgagattat ttatagttta agtagggggt ttgggagtta ttttttaata agaatagttt 6000
aagataaata aatgaatttg ggaaaataag atatattgtt aattagaatt tttatttttt 6060
ttatgatttt atattttttg attgttttaa taaaggtaag atgtattttt tgttttttag 6120
gtgttaggta ttgtgttatg taggatagaa tatgttattt ttatttaatt tttaaaatat 6180
ttttatgaga taaagaatat tattttattt tatataaaag gaatatggtt ttgaaagtat 6240
taggtaattt gttttaagaa ataaattttg ttagtgatat tgttgggatt ttgtgaaatt 6300
ttgtttgatt ttagagtata agatataagt tattaatttt tgttgtattg ttgtttgtta 6360
gtttttgaga ggggaaatta attgggaatg tattagtttt gtttatgata ttttatttgt 6420
tttttttgtg gagttgtagt aaggtttaaa ttttaatttt taaattttgg taataagatt 6480
tagtgatttt tgaatttggt tgattatatg aattttttga gaaattttga aataatagat 6540
aaattttaag ttttattatt agggatttag aaaatttgga gttgggtttt gggatttata 6600

ttttaatatt tatttttgtt ggagaagtaa ggtattattt atatttatat gataaatgaa 6660
aggatatttt gatttttgtt tagtttttat agagaggttt ttgagatagg ttaaaagttt 6720
ttatttagtg taaagatgag ttgttgtata tgtttgtttt tttgttttta tgtatatgtt 6780
tattaaatat gtattaagtt tttaatatgt ttgatatagt aattttgtga ttgtagataa 6840
tttttttatt tgtaaaatgt gagtaataat aatatttgtt ttttgggttt gttttaaaga 6900
ttaaaataaa aatgtatggt ttaatggtag tggatttggg gggatttttt atataaataa 6960
gtgaatggag gtatgaataa ataaatatat aaagatgtgt gtatttatat ttatatatat 7020
aattaaaaat agttaaagat gtataaatta aagttaaatg tatgtgatat tgaagtatat 7080
gttgattatt gataatgaag tatagtataa tttttaattt tatattttaa tattttatat 7140
ttaataattt atattttaat ttttagttta tttaagatat atagatatag atagaatgtt 7200
ttaaatgtat tattgtttta gttttaatgt ataatattta tatgaaaaag tatttattag 7260
tgtgtagtaa atgtattaga atattattta taggttaaat gatattgata atgttttaga 7320
gttgttattt ttatttttgg ataatttttt aaattgagag taattaaatt tgttattttt 7380
ttattttttt ttataatggt aaataatata taaataatga gttgtgattt aaagaaataa 7440
ttttataatg taaaagtttt tttatgtttt attgattttt atgtttttaa ttaattttgg 7500
tagtttaagt ttgtgatttt agtgttgagg aaagtttatt ttattttaga gtagtggggt 7560
ttagttatga ttttatatta taattatttg gataattaaa gaatattgat gtagagtttg 7620
tatttaaaga tttggaattt ttagaagtga tatagaagta ttggtatata tatattttta 7680
aagttttttt ggagaaatat atagttatga ttgagaatta ttgttttggg gaaagtgatt 7740
atttttttat tatttaaata gttaagtttt aggaggtaaa atatttatat ttttttttat 7800
taaaatttgt aaaatatata ttttattaaa gat 7833

<210> 369
<211> 2501
<212> DNA
<213> Artificial Sequence

<220>
<223> chemically treated genomic DNA (Homo sapiens)

<400> 369
   gtaaattttt atattttgag gagagatagg gttttattat gttggttagg ttggttttaa 60
   atttttgatt ttaggtgatt tatttgtttt ggttttttaa agtgttggga ttataggtgt 120
   gagttattaa gtttgatggg tagtaattta ttttttatat aatttttttt tttttttttt 180
   ggtagagatg aggttttgtt atgttgttta ggttggttta aaattattgg gtttaagtga 240
   tttgtttatt tgggtttttt aaagtgtagg gattataagt atgaattatt atgtttggtt 300
   gtaatttttt atatattttg gatattaatt ttttattaga taatatagtt tgtaaatttt 360
   tttttttttt attttgtagg ttgttttttt attgttggtt attttttttg ttgtatagtt 420
   atttttaagt ttgttgtaaa tttatttgtt tattttttgt tttgttgttt gtgttttgtt 480
   gttatattta agaaattatt gttaaattta ttgttatgaa gttttttttt atgttttttt 540
   ttaggagtat tatagtttta ggttttatgt ttaattttta ataagttttg tgtttttgta 600
   tatggtgtaa ggtaagggtt taattttatt tttttgtatg tggttatata gtttttttag 660
   tattatttgt taaagatata attttttttt tatgttttgg tgttttaaaa aaaaaaaaaa 720
   ttttggttgg ttatggtggt ttaggtttat aattttagta ttttgggagg ttgaggtaag 780
   tggattgttt gaggttagga gttttagatt agtttggtta atatggtgaa atttgttttt 840
   tattattgaa gatataaaaa ttagttaggt gtggtggagt atgtttgtaa ttttagttta 900
   ttagggaggt tgaggtatga gaattgtttg aatttgggag gtagaggttg tagtgagtta 960
   agattttatt attgtatgtt agttggggtg atagagtgag gtagggtttt attttgttgt 1020
   ttaggtagga gtttagtggt ttaattatgg tttattgtag tttatattgt tagggtttaa 1080
   gttatttttt tattttagtt ttttaagtag ttaggattat aggtgtgtgt tattatgttt 1140
   agtaaatttt tgtatttttt gtagagatgg ggtattttta tgttgtttag gttggttttg 1200
   aatttttaat tttaagtgat ttttttattt gggtttttta aagtattggg attataggtg 1260
   tgagttattg tgtttgatat ggtgtttttt aatttatttt tattttttat ttttattttt 1320
   ttgagataag gttttgtttt gttttttagg ttggaatgta gtggtataat tatggtttat 1380
   tgtaattttt gtttttttgg tttaagtgat ttttttgttt taatttttgg agtagtttgg 1440
   attatgggta tatgttataa tgattagtta atttttgttt tttttttttt tttttttttt 1500
   tttttttttt tttttttttt ttgagatgta gtttttttat gttatttagg ttggtttaaa 1560
   atttttgggt ttaagtgatt tttttatttt ggttttttaa agtgttggga tgataggtgt 1620
   gagttatgtg gtgtttaaaa aaggtaataa aaaatttttt atatattggg tatagaagtg 1680
   gtatgggttt ttatatattg tgagattttt ggtattagtt ataaattttg tgtatattta 1740
   agatttttta gagtaggtgt aattattttg ttttatagat gaggatatag aggttgagtt 1800
   gtagtgattt atttaaggtt gtatagttag taaatagatg gaggttggat tggaattgag 1860
   gattttattt aagggttttt ataaattttt gggggttttt tgttgtttta tttttattat 1920
   atttgaaaga atgaatgaat gaatgttttg ggtattgtgt ttatttttta gtaaattgtg 1980
   gagtttggat gagtttattg ttttgtgtgg ggggggtgtg tgtttgtttt gtgtatgtgt 2040
   gttttttggg tatggttggt tttgttttat ttttttgtat agggtattgt ttttttttgt 2100
   ttggtatatt tttttttttt ttatgtttgg attggggtgg tagatgttgt ttttgtttat 2160
   tgtttttttt tattggtttt tgtgtgaaaa gttggggtgt ttgtgttgtt gttgttgtgt 2220
   ttgttgaagt ttttgagatg ttggtgtgta ttgttttagt ttgggtgttt atattggttg 2280
   ttttggttat tttgttgttt gatgatgttt gtaggtggta ggtattatgg gggggaatat 2340
   ggatttaggg ggataggtag ggtgttgggt ggggggttgt ttttttttgg ggtggttggt 2400
   ggttgttgtt gatagatggg tgtgtatgtg tggggtggtg tggtgtgtag tggtagttgg 2460
   tgtgggtagg gtggtatttt tggttgtgtg tgtttgggtt g 2501

<210> 370
<211> 2501
<212> DNA
<213> Artificial Sequence

<220>
<223> chemically treated genomic DNA (Homo sapiens)

<400> 370

tagtttgggt atgtgtgatt ggaagtgtta ttttgtttgt gttaattgtt gttgtgtgtt 60
gtattatttt atgtatgtgt gtttgtttgt tagtagtggt tattggttat tttgagggga 120
agtgattttt tatttagtgt tttgtttgtt tttttgagtt tgtgtttttt tttatggtat 180
ttattgtttg tggatattgt tgggtagtga gatggttggg atggttagtg tgggtatttg 240
ggttggggtg gtatgtgttg gtattttgga ggttttagta gatgtggtgg tggtagtgta 300
ggtgttttgg ttttttgtgt ggaaattgat ggggaggggt gatgagtgga ggtggtgttt 360
attattttag tttgggtgtg ggggaggagg ggatgtatta aatggagaga ggtgatattt 420
tgtgtagaag gatggaatgg agttggttat gtttagggaa tatgtatgtg taaggtgggt 480
atatattttt tttatgtgga gtagtgggtt tgtttaagtt ttatggtttg ttgggaggtg 540
ggtatggtgt ttgaggtatt tatttattta tttttttagg tgtgatgggg ataaagtagt 600
gagaagtttt taagggtttg tgagagtttt tgagtaaagt ttttagtttt aatttaattt 660
ttatttattt gttggttata tgattttagg tgggttatta tggtttagtt tttgtgtttt 720
tatttgtaaa atggggtaat tgtatttatt ttagaaaatt ttgagtatat atagaatttg 780
tagttagtat taagaatttt atagtgtata gaggtttatg ttatttttat atttagtgtg 840
tggggggttt tttgttgttt tttttaagta ttatgtggtt tatgtttgtt attttagtat 900
tttgggaggt tagggtggga ggattgtttg agtttaggag ttttagatta gtttaggtaa 960
tatagagaaa ttgtatttta aaaaaaaaaa aaaagaaaga aagaaagaaa gaaaaaaaga 1020
aaaataaaaa ttagttagtt gttgtggtat gtgtttatag tttaaattat tttagaggtt 1080
gaggtaggaa gattatttga attggagagg tagaggttgt agtgagttat gattgtatta 1140
ttatatttta gtttgggaga tagagtaaga ttttgtttta aaaaaataaa aataaaaagt 1200
aagaataaat taagaagtat tatgttaggt gtagtggttt atgtttgtaa ttttagtgtt 1260
ttgagaggtt taggtgggag gattgtttga ggttaggagt ttaagattag tttgagtaat 1320
atagggatat tttattttta taaaaaatat aaagatttgt tgggtatggt gatatatatt 1380
tgtaatttta gttatttggg aggttgaggt gggaggatgg tttgaatttt ggtagtgtag 1440
gttgtaatga gttatgattg ggttattgga tttttgtttg ggtgataggg taagattttg 1500
ttttattttg ttattttggt tggtgtgtag tggtgagatt ttggtttatt gtaatttttg 1560
ttttttaggt ttaagtgatt tttatgtttt agttttttta gtaggttggg attataggtg 1620
tattttatta tgtttggtta atttttgtat ttttggtggt agagataggg ttttattatg 1680
ttggttaggt tagtttggga tttttagttt taagtagttt atttgtttta gttttttaaa 1740
gtgttgggat tataggttta agttattgta attagttaga attttttttt tttttaaagt 1800
attagaatat gggggaaaga ttgtgttttt aataaatggt gttgagaaaa ttgtataatt 1860
atatataaaa gaatgaagtt ggatttttat tttatattat atataaaaat ataaaattta 1920
ttaaagatta aatataagat ttgaaattat agtgttttta gaagaaaata tggggaaaag 1980
ttttatgata atggatttgg taatgatttt ttggatatga tagtaaaata taggtaataa 2040
gataaaagat agataaatgg gtttgtaata aatttaagga tagttatata gtaaaagaaa 2100
tgattaatag tgaaaaggta atttatagaa tggggaaaaa aaaagtttgt aaattgtatt 2160
atttggtaaa gggttaatat ttggaatata tgaaaaatta tagttaggta tggtggttta 2220
tgtttgtgat ttttgtattt tgggaggttt gggtaggtag attatttgag tttggtagtt 2280
ttagattagt ttgagtaata tggtaaaatt ttgtttttat taaaaaaaga agaaaaaaat 2340
tatataaaaa ataaattatt atttgttagg tttggtggtt tatgtttgta attttagtat 2400
tttgagaggt taaggtgggt ggattatttg aggttaagag tttgagatta gtttggttaa 2460

tatggtgaaa ttttgttttt ttttaaaata taaaaatttg t 2501

<210> 371
<211> 4138
<212> DNA
<213> Artificial Sequence

<220>
<223> chemically treated genomic DNA (Homo sapiens)

<400> 371

gaattttgga tttggattta tgtttttgtt taaattttat gttaaattgt aaattttaat 60
gttggaggtg gggttttgtg agaagtgatt ggataatgtg ggtggatttt ttgttttgat 120
gttgtttttg tgatagagat tttatatgat ttggttgttt aaaagtgtgt agtatttttt 180
tttttttttt tttttttttt tgtttatgtt ttgttatgta agatgttttt tgtttttttt 240
ttattgttta gaatgattgt aagttttttg aggttttttt aggagtagaa gttattatgt 300
tttttgtata attgtagaat gatgagtgaa ttaaattttt tttttttata aattatttag 360
ttttaggtat ttttttatag taatgtgagg atagattaat ataatttttt atttttagat 420
ttttgtatat gtttagtttt agatattatt gtttttggga gtatgtatag tgtagttttt 480
tgttgataaa agtaaagtta taaaggtgat aaaaatttgt atttggggat atttgattgt 540
gaaagaggat ggatagtata tttgtagtta tagagattgg ggtttattga gttgaaattt 600
ggtagtattt tggtataata tgtgtatgat ttgtgtttaa tgtttagaga ttagtgttga 660
gtaaaatagt ttggtttggg gttgttgttg tttttatttt ttttttgttt attagagggt 720
ggtagagttt ttttttttta ttttggagtt tttttagggg ttgttgattt ttttagttgg 780
gtttatagtt tagtagggtt tatttttatt tgggttattt tggtttatgt tttttttgtt 840
ttttgagttt tttatatgga ttttgttagt ttttttttgt agtttattgg ttgtttattt 900
gaggtttgtt ggttgtttat ttgaggtttg ttggttgttt tttgtaggta gtttttgttt 960
tttatatttt tttttttttt gggtttagtt gaaagggtgt tttttagggt agttttttgt 1020
gatttttagt atagtttagt tttttatagg ttttgatgtt ttttatgttg ttattttata 1080
gttttgttat tattattaat tttttagttt tatgaagttt attgagtgtt tgttttttgg 1140
ttataggaaa attttgtgat agggattatg tttgtaatgt tttttgtgga attttaaggt 1200
ttagtttagt gtttgatatg gaatagatgt tttataaata ttggttaaat gtgtgggaga 1260
tttttaaaaa tagtatatta tttttgtgtg gtttttagta gttagagttt gttttatgtg 1320
gatatagggg tattggtatt agtatgggag gaggttagta agtgtttgtg gttgttttag 1380
gaatgaggtt ttaattttta gagttttaga agggaggata gaggtttgta gggaatagat 1440
tttttggttt gattttgtag tttaatttag agtttagggt tagtttatat tatgttgatt 1500
ttggttagta tttttagggt agttttagat aaggttggag gttttttttt ttgtttttta 1560
gggggtgata ttgtatatag atattattta ggaaatggat ttttttggat aggaatttgg 1620
ttttgttaag gaagtggagg tggagtttgg tttttatttt ttgttttaat agattttttt 1680
gatttttttt atatatttgt tttgtttttt tttgggtttt atgaggattt tgttttgtta 1740
ggggtttttg tgtaatttta gatttttttt tggtattatt atggggaagg tggggtgatt 1800
ataggatagt tagttttgta gagatagaga ttatttagga tagttaggga gaatatggat 1860
aggttttgag ttgtagtttt agttaataga tatggagagg gagggttttt ttggagtttt 1920
ttttaaggat agtagagttt agagttattt attttttttt attatagttt ttttttttta 1980
ggatatataa gatatttttt tttttatatg taggatttgg ggatttttga gatttttggg 2040
tttgggtttt tatttttggg ttagtggtgg ggttggtggt attggagata gagggttggt 2100
ttttttttag ttattattta gtgagttttt ttttagtttt tagagttatt tttgttattt 2160
ttttgttggg tattatttta tttttttaga gttttggaga gtatggggag atttgggatt 2220
ttgttgggtt tttttgttat aaaggaaaat aatttttttg gtgtgataga tttaaggata 2280
gaatatagta gaggttagta ttggggaaga taggttgttt ttttagggga tgggggtgga 2340
tttattttgt tgaaaagatt tgtttgagga attgaaaata gaagggaaaa aagaggaggg 2400
ataaaagagg tagaaatgag aggggagggg atagaggata tttgaataaa gattatattt 2460
tgatttatgt gatgttgaga agtatttttg ttttaggaag agatttaggg tagagggagg 2520
aaggatagta gattagatag ttatagtagt tttgataaaa tgtttttgga atttaagttt 2580
ttttttatag aggaggatag agtagatagt agagattatg gagttttttt tggttttttt 2640
ttatagatgg tgtatttttt ggtagaggtt tttgtttata ggtgaaggga ggataatttg 2700
ggagagggtg ggaggaggga gtatagagat tggttggggt tttttgggta ggatagggtt 2760
gtgagatgga tagaggtttt tgttggagtt tgaataggga agaggatatt agagagggat 2820
aggagttata ttagaaaaat taaattgaat tggaattgga aaggggtagg aaaattttaa 2880
gagttttatt tttttagtta attgttattg gttattatgt ttttaaaaat tataataatt 2940
gtattatatg atattttaaa taaaaatata ataattgtat tagatgatat tttaaataaa 3000
aatataatta gggtatgaaa tattgttttt atttgtttat tgtggatatt ggaaaataag 3060

ttttaggttg tggagggttt tgggaatttt tatgaattta tttataggaa tttgtagttt 3120
gttttaggta ttggggtgta attaagatta tataaatttt tgtttttatg aagtttatgt 3180
ttttatgggg aggaagatag atatataaag agatttagaa tgtgaggtta ggtgttgata 3240
agagttttgg aggaatagag tagggaaagg ttagaaaagg aagatttagg gtttttagag 3300
gaggtgttag ggaagggatt ttttaagaat gttttgatgt gagtaggatt tgaaggtaat 3360
ggggagggag ttgtgaagat ttttggaaaa gtagatttta tataggaaaa tgttaaggtt 3420
ggaggtgtta aggaaatagg agatatattg ttgattttga tttagtagga tatatatata 3480
tatatatata tatttattta ttttagggtt gggggatgaa gagatttgtt taggatttag 3540
gattttattt ttttatttta atgtataggt tttaatattg attgatgttt tttgtttttt 3600
tttagtttta tttttaattt tttggaattt gtttattatt gttaagttta ttattgaatt 3660
tatgttgttt aatgttgtag aggggaagga ggtgttttta tttgtttata atttgtttta 3720
gtattttttt ggttatagtt ggtataaagg tgaaagagtg gatggtaatt gttaaattat 3780
aggatatgta ataggaattt aataagttat tttagggttt gtatatagtg gttgagagat 3840
aatatatttt aatgtatttt tgttgattta gaatattatt tagaatgata taggatttta 3900
tattttatat gttataaagt tagattttgt gaatgaagaa gtaattggtt agttttgggt 3960
atattgtgag tgattttttt atgatttttg ggtgttgggg gttagtttta ttttttatat 4020
ataggattat taggtttggg ttgtgttgtg gttttttttg tattatgtat tatgttaggg 4080
tttgggtatt tagtgtagga tatatataga agagataaat tttaatagat tagaattt 4138

<210> 372
<211> 4138
<212> DNA
<213> Artificial Sequence

<220>
<223> chemically treated genomic DNA (Homo sapiens)

<400> 372

gaattttgat ttgttgaagt ttgttttttt tgtgtgtatt ttgtattaaa tgtttaaatt 60
ttaatatggt gtgtaatgta gagggggtta tagtatagtt taggtttgat aattttgtgt 120
gtgggaagta gaattgattt ttaatattta gaggttatgg gggaattatt tatggtatat 180
ttggaattgg ttagttgttt ttttatttat aagatttgat tttatgatgt gtagggtgta 240
gaattttgtg ttattttgga tgatgttttg gattagtagg gatgtattgg ggtatattat 300
tttttgatta ttgtatgtgg gttttggggt agtttgttga gtttttatta tatattttat 360
aatttgatgg ttgttattta tttttttatt tttgtattag ttgtagttaa aaagatgttg 420
gggtagattg tggataagta gaagtatttt tttttttttt gtgatattga atggtgtgga 480
tttaatagtg agtttggtag tggtgggtgg gttttagaag gttagaagtg aggttaggag 540
agagtagaga gtattggtta atattgggat ttatgtatta gggtggaaaa atggggtttt 600
gggttttgag taggtttttt tattttttag ttttggagtg agtgagtgtg tgtgtgtgtg 660
tgtgtgtgtt ttattaggtt aaggttagta gtgtgttttt tattttttta gtatttttga 720
ttttggtatt tttttgtgtg gaatttgttt ttttaggggt ttttatggtt ttttttttat 780
tgtttttagg ttttgtttat attagggtat ttttgggaga tttttttttt gatatttttt 840
ttagagattt tgggtttttt ttttttgatt tttttttgtt ttattttttt agggtttttg 900
ttaatatttg attttatatt ttagattttt ttgtatgttt gttttttttt ttatgagagt 960
atgagtttta tgagggtagg gatttgtgtg attttggttg tattttagtg tttgggatag 1020
gttgtagatt tttgtggatg agtttatgag ggtttttagg gttttttata gtttggggtt 1080
tattttttaa tgtttgtggt aggtggatga ggatagtgtt ttatgttttg gttatgtttt 1140
tatttaaagt gttatttgat gtagttatta tgtttttatt taaagtgtta tgtgatgtag 1200
ttattatgat ttttaaaaat gtagtggtta gtgataatta attaggaaaa tagaattttt 1260
gaggtttttt tgtttttttt taattttagt ttaatttgat ttttttggtg tgatttttgt 1320
ttttttttga tgtttttttt tttatttagg ttttaatagg agtttttgtt tgttttatag 1380
ttttgtttta tttaggagat tttagttagt ttttgtgttt ttttttttta ttttttttta 1440
ggttgttttt tttttatttg tgagtaggag tttttgttag gggatgtatt atttgtgggg 1500
aggggttgag ggagatttta tggtttttgt tgtttgtttt gttttttttt gtggagaaga 1560
gtttgagttt taggaatgtt ttgttaaggt tgttgtgatt gtttggtttg ttgttttttt 1620
tttttttgtt ttgagttttt ttttagggta ggagtatttt ttagtattat gtgggttagg 1680
gtgtggtttt tatttaggtg ttttttgttt tttttttttt tatttttgtt ttttttgttt 1740
tttttttttt ttttttttta tttttagttt tttagataaa tttttttggt aaggtggatt 1800
tatttttatt ttttgggagg ataatttgtt ttttttagtg ttgatttttg ttgtgttttg 1860
tttttgggtt tgttatatta gggggattat ttttttttgt gatagagaaa tttagtaggg 1920
ttttgggttt ttttatgttt tttagggttt tgggaaggtg ggatgatgtt taataggaag 1980
gtgatagagg tggttttggg ggttagaaaa aggtttattg ggtggtggtt ggggagggat 2040

tagttttttg tttttagtat tattaatttt gttattgatt tagggatgga gatttaggtt 2100
tagaggtttt aggagttttt agattttgta tgtagagggg gaggtgtttt gtgtgttttg 2160
gaaagagagg attgtggtgg agggaggtgg gtgattttgg gttttgttgt ttttggggaa 2220
ggttttaggg ggattttttt tttttgtgtt tgttggttga agttgtggtt tagggtttgt 2280
ttatgttttt tttgattgtt ttgggtggtt tttgtttttg tgaggttgat tgttttgtga 2340
ttattttatt ttttttatgg tggtattagg agggagtttg gagttgtata gggatttttg 2400
gtagaatagg gtttttatag gatttagaaa ggaatagagt aggtatgtgg gagagattag 2460
aagggtttgt tggagtaagg gatggaaatt aggttttatt tttatttttt tagtaaagtt 2520
aggtttttgt ttaggggaat ttgttttttg agtgatgttt gtgtgtaatg ttattttttg 2580
gagggtaaag aaggagattt ttggttttgt ttggaattgt tttagggatg ttgattaggg 2640
ttgatgtggt gtgagttgat tttgaatttt ggattaggtt gtagggttag gttggaggat 2700
ttattttttg taggtttttg tttttttttt tgaagttttg ggggttgagg ttttattttt 2760
ggggtagttg tgggtatttg ttggtttttt tttatgttgg tgttagtgtt tttgtgttta 2820
tatggaatag attttgattg ttgggggtta tatggaggtg atatgttgtt tttagagatt 2880
ttttatatat ttaattagta tttatggagt atttgttttg tgttaggtat tgggttgggt 2940
tttgggattt tatagagagt attatagatg tggtttttgt tatagagttt ttttgtaatt 3000
gggagatagg tgtttagtga attttatggg attgaggagt taatggtaat gatagggttg 3060
tgaggtgata gtataggggg tgttggagtt tgtgagagat tgagttgtat tggagattat 3120
agggagttgt tttgggagat gtttttttag ttgagtttgg ggaaggaggg ggtgtagggg 3180
ataggagttg tttgtagagg gtagttgata ggttttaagt gggtggttga taagttttag 3240
gtgggtggtt gataggttgt agggaggagt tgatagagtt tgtgtgagga gtttggaggg 3300
tgaggaggat gtgggttgag gtgatttggg tgagggtgga ttttgttagg ttgtgggttt 3360
ggttgaggag gttagtagtt tttggggagg ttttagggtg ggagggagga attttgttgt 3420
tttttggtgg ataggaggga agtggggata gtagtggttt tagattaggt tgttttattt 3480
aatattgatt tttagatatt gaatatgggt tatgtatatg ttatgttaaa gtgttattag 3540
gttttagttt ggtgagtttt agtttttgtg gttataagtg tattgtttat ttttttttat 3600
aattagatgt ttttaaatgt agatttttgt tatttttgtg attttgtttt tgttggtagg 3660
aggttgtgtt gtgtatgttt ttaggggtag tgatgtttgg ggttaagtgt gtgtggggat 3720
ttgggagtag aagattgtat tagtttgttt ttgtattgtt ataaagaaat atttgagatt 3780
gggtaattta taaagaaaag aggtttaatt tgtttattat tttgtagttg tataggaagt 3840
atagtggttt ttgtttttgg ggaggtttta gaaaatttat aattattttg gatggtgaag 3900
gggaaatgag gaatgtttta tatggtagag tatgagtaag agagagagag agagagggga 3960
gaggtgttat atatttttaa ataattagat tatgtgagat ttttattata gaaatagtat 4020
taaagtagaa aatttatttg tattatttaa ttatttttta taaggtttta tttttaatat 4080
tggggtttat aatttgatat gagatttggg tggggatata gatttggatt tggaattt 4138

<210> 373
<211> 11222
<212> DNA
<213> Artificial Sequence

<220>
<223> chemically treated genomic DNA (Homo sapiens)

<400> 373

ggtttggtgt tggtttagga gggtagattt tgttagggag gtataagtta agttgggggt 60
ggggtgggag ggtttgttgg taggagggag gtattgttta gattggtttt taaaggttgg 120
tttttagaag gaggaggaag agagtagtga agattgtggg gagttttggt tttgaggagt 180
tagagttttt ttttttgtta tatttttgat tttttttggt ggtgttttaa gtttagattt 240
attaaaggga attgttagtt gtttaagggt tagtaaattt ttaggttatt tagattggat 300
tttttgatta taaaaattgt gggttaggtg tatagttgtt tgaggggttt aaatttgtgt 360
aggtgggtgt tggaattttt tttgttatag tgtttttttt gtgtgtatgt gattagaggt 420
gagagtggtt ggagattgaa gatggtagag tttgtttttt tttattagat tgaggttttt 480
gatttgtttt gtttggggag ggtgtttagt ggaagttttg ggatgtttag tttggataag 540
aaaagttttg ggtgagtttg tttttttttt tgtgaggtag atttagttgg gtttttatat 600
ttttaaggag taggtggagt tgttgtaagg aagtgtatgt gatatggagg tattttttgg 660
tttgatataa aaaaggattt ttaaagtttt aggtagtgag tttattattt ttaggagtat 720
ttaagtagat gttagatgat gatttgtagg gaaaattatg tttagtggga aattggataa 780
aatattgttg aaggtttttt ttagttataa gatattatgg ttttataatt ttagattttt 840
ttgtttttag ttttttataa gtttggaatt tagaatttaa gtttttttgg tgttgaaaag 900
ttgaagattt tgatatttta gaattttaat tttgggattt taagatttta gaattgggag 960
ttttgaggat gattttataa gttttagttt tggatttaaa ggattttgtg atatgaaagg 1020

gaaaaagagg aggtttgatt tggtgggatt tagttttgtt ttattgggta ttagattaat 1080
gtgttttgta tttgtgtgtg tgtgtgtgtg tgtgtagtat gagtgtgtaa ggttgtagaa 1140
agttttgtat ataaaggtag tttggttttt ttattatgtg tttgtagttt ttttgttttg 1200
gttataatga aggttgttat agggataata atagttttta aatagagttg gtgattttag 1260
gtagttatag taattgtgag gtaatgttgt tttgttgtag agatgtgtga gtataaatag 1320
atgttggtgt ttttttatta gtttaaatgg taaaatttaa aaggtttatt aattttaaag 1380
ttataggttt ttatgagtat gttagttaat gggggtgttt gttaagtggg tgatagtttt 1440
atattaaata ttttagtatt agtaatgggt tgatgagatt ttaaatgttt aggttatttt 1500
tagataattt aggaggttag agatgggaga agagttggga tttgggtagt aattagtttt 1560
gttttgtttt gtatttttag gggtgttttg atatttttgg gagggtatag gttagagagt 1620
atttgaaaat tgtatattgt gttttaattt ttaggagttt tagatttgtt ttttggtttt 1680
ttttagtgta ttaggtaggg ggttttaggt tagatgtggt atagtttaat gttatttttt 1740
tttttttgtt tttttttttt atattttgga tttattttta gtgagtgatt agtagtaagg 1800
gttaaattgt tgagagtttg agattggggt agattttttt agagttgttt ttttttttga 1860
ggttgggttt ggtatagatt ggggttttag taatgggttt ttaaatgaat gaatgaataa 1920
aggaataaat agatgattga ttgattgaat gaatgaatga gatttttttt ggtattttta 1980
tggttttttt tttttttttt gagatggagg tttgttttgt tgttaggttg gagtatagtg 2040
attttggttt attgtaattt ttgttttttg ggtttaagtg atttttttgt tttagttttt 2100
taagtagttg ggattatagg tttgtgttat tatgtttagt taatttttgt atttttagta 2160
gagatagggt ttgaatatgt tagttaggat agttttgatt ttttgatttt gtgatttatt 2220
tattttagtt ttttaaattg ttgggattat aggtgtgagt tattatgttt ggttttttat 2280
ggtattttag ttatttattt tgttattgtt ttttttattg ttaaagagat gggtttttgg 2340
aggttggttg gtttagtatt tggggttgat ttttggaatg gttattaggg aaggggtgtt 2400
agaggtgtag ttggtgtttt tgattttttt ggttgttagg agttagttga tttagtatat 2460
aggatttatt gaatggagat ataaggtttt tttagggagt ggtggtttat ggtaatttta 2520
gaatggttat tagttaggtt ttagagattt atatagaggg tgttttgatt taaagttgta 2580
ttggggaatt ttaagtttgg ggattttttg aatttaattt tttttttagt tatatttttt 2640
attatttgtt taatttttat taaatatttt tgtttatatt ttgaagttgg gatttgattg 2700
gtagagttag tagatgttga ttatttagat ggagttttga tattggtttt tttagtttgg 2760
ttgtgattgg tagtaggttt gtgggagaat tgtgtgtttt agaatatgat tggttatatt 2820
tgtattttag taagattggg gtagggtagt tatttttaaa aagttgtgta ggtggggtag 2880
ttattattga taaatttagt gtagatttag gatggtttaa atattggttt attttttttg 2940
aattttattt tttatagttg taaagtgggg tggtgtttgt tattttatag aggtgttgta 3000
aagattagat gtaattttgt taagtagtta ttttgtaaat tgtatagttt tatgtagatg 3060
gaaggaaggg tttgtgttta ttttgattat agtattaaat aaattgtatt gtatttttat 3120
tttttttagt tattttttta aaaagatttt gagttttttg aatataggaa ggtgttttat 3180
ttgattttgt tatttttagt atgtagtagt gtttgatata tagtaggtgt tttattattg 3240
tgagagggat ggatggatgg gtggagttat agatggatag aaggatagat ggagggatgg 3300
gtggatgatg gatggataga tggatggagg ggggatgatg aatggaggga taatgagtgg 3360
atgaatgagg gaatgggtgg atggatggat ggagggatgg aggaatagat agatagatgg 3420
agggatgggt gggtgatgga tggatagatg gatggaggga gggatgatga atggagggat 3480
aatgaatgga tgaatgaggg gatgggtgga tggatgaatg gagggatgat gggtggatga 3540
atgaattgag ggatggatgg atgaatatat ggatggatgg atagatggat agatggagga 3600
attggtggat tttggatgga tgggtggatg gatagatgaa tgaatgtttg gatagataaa 3660
gagatgatgg atagatgaat agatgaatta agggatgttg gatagatgga gggattgata 3720
gatgttggat ggatgggtgg tggatggata gatgagtgaa tgtatggata gataaagaga 3780
tgatggatgg atgaattaag ggatgataga tggatggatg gatgagtaat tggatggata 3840
agtggataaa tggatagatg gttgaatatt tgaatggatt gaaggaggat gtatggatgt 3900
aagataaggt taattatttt ttattttttt tttttgtaaa attatttatt tatttattta 3960
ataaatattt atttagttta aatttggtat aaagtattat gtgaggttta agagatatgt 4020
gggttaataa aatagagttt ttgttttttt gaaaattgta aagaaagggg tgtggttttt 4080
tgagtttaaa ttttaatttt gttagtgatt agttgtatat tagtgatgtt tttttatttt 4140
tttttaatta aatagggata atgttagtat ttattatatt gggaggtttt gtggggatta 4200
aatgagttat taaatgttaa gtgtttggga tagggtttgg tatttagtaa agttttttgt 4260
gagtgttggt tgttattatt ttaatggaga agatggtatg aaaattagga aataggatgt 4320
tttttgggaa gtaatgtaat aggaatttat ataaagaaag gaaaggagga agtaattagt 4380
ggtgttttaa aggagtatgt taagaaaaat tttttagagg gaaatttttg agtagggtta 4440
tgaaaatagg agttttttaa gagattgtgg atttgtttgg gattatttgg ttataagtat 4500
aaaattattt ggtttttttt tgttattttt ggttgggtga ggggtttttg gtaaaggggt 4560
agaaggtgtg tgagaggttg tgaatggtta ggattgtttt ggggttagtt ggggtatttg 4620
gtggttaagt ttagaaatat gataggtttt tttgggaggg ttgattgtag ggagtgttgg 4680
gttttaggtt gttggtgttg gtttttgtgg tgtttttttt gttggttatg agagtttaga 4740
tagtgtttaa tttttttttt ttttttttat atgtataatt attttatttt ttgtggtttg 4800

agttgttttg ttttgttata atggtatttg ttttaaaata gttttttatg tgagggttag 4860
agaaaggaaa agattagatt ttttttggat gagagagaga aagtgaagga gggtagggga 4920
gggggatagt gagttattga gtgatttttg ttaagtattt tagaaggtat aaaaatgttt 4980
ttgggattag gtagttttaa attttagttg ttggggttag gatatttagt gagtttatat 5040
ttgttttttt tgtttttttt agattgtgtt atggggttta gtgatgggga atggtagttg 5100
gtgttgaatg tttgggggaa ggtggaggtt gatattttag gttatgggta ggaagttttt 5160
attaggtaaa aggaagagat tttattgttt ttgttattta tattttaaga ttaagggtgt 5220
ttagttgtaa ggtggaaagt ttgtatgtgg ggtaggttag ttggttgtat tagttaaggg 5280
tgttagaatg gttatttgtt ttttttttgt ttttaagtgt tagggattgg atttaggaga 5340
gggaaaggag ttattttagg ttgatgttag tagttggagg aagtatgaga attaaattta 5400
ggatgtttag agtttattag gaagaatttt agaattatag atagttagag ttaataaggg 5460
ttttgagaga ttttgtatag ttattttttt tataggatga ggataaaaag ggattgagaa 5520
ggggaggata tttttagagt tatagtttat taaatgtttt taaagtgtta aggttaagat 5580
atgtttttta aggggagata gatttggttt tagatttggt tttgttattg agttattggg 5640
tgatttttgg gaaagtattt aattttttgg agttttaatt tttttttttg tatagtgagg 5700
ggatatttta atatttatat tttagaggag atgtgagaat taaataaaat aatgtatgta 5760
agaggtttgg tatggttttt ggtatatatt gagttttaga aatgttagta gttattattg 5820
atgaagttta ggttagggat tttttaaagt attgtaatta gagaatagaa gatagaggtt 5880
tattagtgat ttttgatgtt gagtatgttt ttagtttgag aggtttgaat gatgtggttt 5940
gtaagtatat tttgtttttt attataaggg attttagaat atattaaaga aaataaaatt 6000
ttgaggtttg taaatagagg gtggttgtgg tttgtatata gaagtttatt tttttgttgt 6060
tttttatttt aaaggtgata tatttttttt ttggtttttt tttttattat tttgagttgg 6120
tttttttaga agtttaatag gttaagaatt aatgtttttg ttaatgggag gaaggaagtg 6180
ggtgttgggg ttgttataag tgaatggatt gtttttggta ttttaatttg tagattaatt 6240
tttttttatt tgtgagttag agttaatttt ttttttttat tttgttgttt tttgggtttt 6300
ttttgggttt tatttatttt tagtttttgt ttattttggg agtatggttg gttttggaag 6360
ttttgttatt taaaatttga gttgttggtg atttgtgtta gaaatatttg aggaagaaga 6420
gttgagtttt ttgttgattg taatggtatt gtagtgtgtt tatgtttttt tttttttgtt 6480
tttgttattt ggttgtaata ggttttgttg gattgtataa agtgtttttt tgattaatat 6540
atatatataa attgatatta atttttgggt tatagatatg ttatgtgatt ttggataagt 6600
tttttttattt ttgggtttta tttttttgtt ggttgagtga aggattgttt ttttatggtt 6660
tttgagattt tgttttatgt ttatgtttta gaattttttg agtttttgat gtatttttgt 6720
ttttggtgag gaggtaggat agttaggtgt ggaggtagga gattagagtt tgtagagtag 6780
aggttttttt aaggtgattt gggagttgtt agatttaagg tagtgatttg tatgttttga 6840
aagagattta gggtgttgta ttattaggga ggattatgga ggtttgttgg gggttgggag 6900
ttagggataa gatataatgt ttagggttgt tggtttttga aaggttttag aggtttggtg 6960
ttttgttatg ttttaggttg tgggatatgt agagattgtg ttagttggtt ttgttttttt 7020
agtatttggg gtatagggag gatggttttt ttgggggtag ggtggaggag gtttatgttt 7080
aggattgtaa atgttatttg tgagagttgg tattttgatt ttgttttaga tattgatttt 7140
ggatttttta attgattttg aaaggttatg tttttaattt tttgattgta aaagagggta 7200
aattttatga agtttagttt gtaaaatatt gaggttttta ttgtatgtat ataaatataa 7260
aattttagaa tatagaagtt ggaaggttat ttggagattt tggttttagg agtggggttt 7320
tggaagggag ggggttgttg agtgggtttt atatttttgt tttttttttt tttatttaaa 7380
ttttttttgt tttatttatt ttggttgttt tggggagtgg ggggaataaa ggattttaag 7440
gttataaaat gtttgaaaat tattagtggt ggttattatt ttttattata gggaggaatt 7500
gggtttgaga gtttttattg gttaatggta gttataggta ggattaaaaa tttttgggtg 7560
atggtgatga taatgataat gataatgata atggtagaga tgatgaggag gagaaagata 7620
gtaattattt atttagtata tattgattgt tgggttttgt ttaagtgttt tatttattga 7680
agtttgataa taattttatg aggtagatat gattatgatt attatattta atagagaggt 7740
taagtaattt atgttaagtt atatagtaag taagaaagga gttggaattt aaatttaggt 7800
agtttggatt tagaattttt gttttttttt tttttttttg agatggagtt ttgttgggat 7860
tataggtgta tgttattatg tttggttaat ttttgtattt ttagtagaga ttaggttttg 7920
ttatgttggt taggttggtt ttgaattttt gattttaggt gatttgtttg ttttggtttt 7980
ttaaagtgtt gggattataa gttaatgagt ttggtttaga atttttgttt ttgattatta 8040
tgttatattg ttttatgatt ttgttatatg taagatgggt tgttatttat gttttggttt 8100
ttttatttta gtagagttag aggggtgttt atggatttga taatgtgttt tatgttttat 8160
gtgagtgttt aattgggttt tagtttagag aatagtagga aaatggtttt tttttttttt 8220
gatttggatt gaagatttgt ttgatgaatt aggtttattg atagtagggt tgaagttgga 8280
aatataggtt agtggggttt tttgaaatat tagagtaaat gttttttgtt tatttttata 8340
ggttgagggg tttttatttg tttatttttt ggattaggta aatttaggag aatggttttt 8400
tgtggttgtt tggggggttt ttagttgtaa ggtttgggtt tataggaggt aggtagttgt 8460
gtggtgttgg aatgattttg tggggagata aattagtttg attttgtttt gttgtttaaa 8520
gtaaatatgg ggtggggtgg ggtggtttag gtttataatt ttagtatttt gggaggtagg 8580

attggggaat tgtttgagtt taggagtttg agattagttt gagttatata gtgagatttt 8640
ttttttttat aaaaaataaa taaaattagt tgggtgtggt ggtgtgtgtt tgtagtttta 8700
gttatgtggg aggttgaggt aggaggatta tttgggttta ggagattgag gttttagtga 8760
gttaagatta tattattgta ttttagtttg ggtaatagag taatattgtg ttttaaaaaa 8820
atattaataa agtaaatatg agtttttttt ttggttgttt ggtgttgagt ggaaagaaag 8880
gtattggtgg gtttagattt ttttggttta tatatggatg agtaatgatt tagagaggtt 8940
aagtgatttt aatattatag agtatattgg tgttagaatt gggattggaa tttagttttt 9000
gtaattgtta gttttatgtt ttttttttta gatattagag ggtaggggta ggttttaaaa 9060
tgtttgagat ttttagttgt tttgtggttt aaatttttgg ttttgttttg ttttagatag 9120
tttgttttta gtgttttgat ttttgattat tgtttgtgta tgttaggttt tgtatttgtt 9180
aggtgggagt ttaaggatta ttgttgttag ataataatag ttaggagttt aggatttttt 9240
ggatgaaagg tgttagagat aagttgttag tgatgtatga ggatttggat aaggttttgg 9300
agtgaggtgg taatgttggg tatggtggta gattatgttt aatatggggg aagggagtgt 9360
atgtggttta ggtttgtgtg tgtttttgtg tgtgtgtgta ggtgagtatg gtgtgtgttg 9420
ggatatattt atgtgtattt ttgtgtgagt gtgtaggttt atatgtgagt gtgtatatat 9480
atttgtgaga gtttgtatgt atgtatatgt gtgaggtgtt gatatatgtt tatgtaggta 9540
ttagtttgtt tgtaaatatt tttgtgtaat aataataaag tttgaatagt tattaagagt 9600
atagatatga agttagattg tttgggttaa atttttagtt agttagtttt ttggtgtttt 9660
tgtttttttt tttgtaaaat ggtgtttatt ttattaggat tttaaaatta gttaatatgg 9720
ttgggtgttt gtaattttag tattttggga ggttgaggta ggtggattat aaggttagga 9780
gattgagatt attttgttta atatggagaa ttttgagaat tgtttaaatt taggaggtgg 9840
aggttgtggt gagtagagat tgtattattg tattttagtt tgggtaatag agttagattt 9900
tattttaaaa aagaaaaata aaaaataaaa aaattatgaa tttattttta ggttgaggag 9960
tttagtattt tggttgtgaa atattttttt tataaaattt tgggatggag attatgggga 10020
ttaggtgttt ttttgtgata atttttgggt atggtggttt agggtgtaaa ttggagtgtg 10080
gttataatat atattgtgta tttttataag gatgttatag agtttgggta ttataaaaga 10140
ggagtttttt aaggaattga aattattaga taggagagag agttttgggt agatagggtt 10200
gtttgtgtta aatattttag ttgtggtata agggaaaggg tgggagttat gaaattgttt 10260
tattttgggt ttaggtttgg gttttgttgt tagttagtta agtgattttg gttatttatt 10320
tttgtggttt tttatgagta aaaggtggaa atttattttt atttagaggg taggtttgat 10380
tttttttaat tagtatttat ttgtttatag taggaaggat tgaggtttaa agttggaggt 10440
gggtaggaag gattgaggtt taaagttgga ggtgggtagg aaggattaag gtttaaagtt 10500
ggaggtgggt aggaaggatt gaggtttaaa gttggaggtg gttgtttaga gttttagtag 10560
aggtttttgg ggtattttat tgagtgtttg gtaggagtgg gtgtttgttt tagggttggg 10620
ttgagttgtt tttattagga ttttttgtta tttgtatagt gaggggattg ggaggtttag 10680
agagttatag tttgggttta aaataagtaa gaggtttttg agtgtgagga ttgttttgga 10740
gtggaatggt ttttataggt aggagtgagt tttttgtagt tagaggtatt taagtagttg 10800
aaggataatt tttgggtagg aagttgtaga gatggttgta gtgtggatta gaattgttgt 10860
tttggttatt tagattttat tttagtttgg tttttttgga tagtattttt gtaatagtga 10920
gttggtgatt ttatgtttta gaattttggt ttttatattt gtaaaatggg aattatatga 10980
tatttattat gtgttagata ttttgttggt atatagtata tattatttta tttaattttt 11040
taagtaggga taagttattt ttatttttta tatgaggaag ttgaggtata gagaggtgaa 11100
gtgaatggtt taaggttata tagttgggaa gatagggagt taaatttgaa ttttagtttg 11160
gttgttttta gattttatat tgtatttttt atgttgattt tagttttttt tgtgtttata 11220
gg 11222

<210> 374
<211> 11222
<212> DNA
<213> Artificial Sequence

<220>
<223> chemically treated genomic DNA (Homo sapiens)

<400> 374

tttgtgggta tagggaaggt tggagttggt atgggaggtg tggtgtgagg tttgggggta 60
gttagattag agtttaagtt tagttttttg tttttttagt tgtgtgattt tgggttattt 120
attttatttt tttgtgtttt agttttttta tataagggat ggggataatt tgtttttatt 180
tgaaggatta agtgagatag tgtgtgttat gtattaatag ggtgtttggt atatagtgag 240
tgttatataa tttttatttt atagatgtgg aaattgaggt tttgaggtgt aaagttatta 300
gtttattatt gtaggggtgt tgtttagaga agttaggttg gaatgaggtt tgagtgatta 360
aaatagtagt tttagtttat gttgtgatta tttttgtagt tttttgttta gggattgttt 420

tttagttgtt taaatatttt tagttatagg aggtttattt ttatttgtga gggttatttt 480
attttagagt aatttttata tttagaaatt ttttgtttgt tttgagttta agttgtgatt 540
ttttgaattt tttagttttt ttattgtgta gatgatgaag ggttttggtg ggagtaattt 600
aatttagttt tgagataggt atttattttt gttaggtatt tagtgaggtg ttttagaggt 660
ttttgttggg atttgagtta gttattttta gtttagattt ttggtttttt ttgtttattt 720
ttaattttag attttggttt tttttgttta tttttagttt tagattttag ttttttttgt 780
ttatttttag ttttagattt tagttttttt tgttgtgagt aggtgggtgt tggttaaagg 840
gagttagatt tgttttttgg gtaggagtga gtttttgttt tttatttatg gaagattata 900
gagataagtg gttaaggtta tttggttagt tagtggtaga gtttagattt aaatttaaaa 960
tggaatagtt ttataatttt tatttttttt tttgtgttat agttgaaatg tttggtatgg 1020
gtaattttgt ttgtttaggg tttttttttt tgtttaatgg ttttagtttt ttgaaaagtt 1080
ttttttttat gatatttagg ttttgtgata tttttgtaaa agtatatagt atgtattgtg 1140
gttatatttt agtttgtgtt ttgagttatt atgtttagaa gttgttataa ggaagtattt 1200
gatttttgta gtttttattt tagggtttta tgaggagggt attttataat taggatgttg 1260
agttttttaa tttgaaaatg agtttatggt ttttttgttt tttgtttttt ttttttgaga 1320
tggaatttgg ttttgttgtt taggttggag tgtagtggtg taatttttgt ttattgtaat 1380
ttttattttt tgggtttgag taatttttag aattttttat gttaggtagg atggttttga 1440
ttttttgatt ttgtgatttg tttgttttag ttttttaaag tgttgggatt ataggtgttt 1500
agttatatta attgatttta aaattttgat ggggtagatg ttattttgta gaggaagaaa 1560
tagaggtatt gaaagattaa ttagttgggg atttaattta ggtagtttgg ttttatgttt 1620
atatttttga tgattatttg gattttgtta ttgttatata gagatattta taggtaaatt 1680
gatatttata tggatatgta ttaatatttt atatgtgtat atgtatgtag gtttttatag 1740
atgtgtgtgt atatttatat atgggtttgt atatttatat agaggtatat ataaatatat 1800
tttagtatat gttatattta tttgtatata tatatagaga tatgtataag tttgaattat 1860
atatattttt tttttttatg ttgggtatgg tttgttgttg tgtttaatat tgttattttg 1920
ttttaggatt ttatttaaat ttttgtgtgt tattgatggt ttgtttttga tattttttat 1980
ttaagggttt ttgagttttt gattattatt atttggtgat aatgattttt gaatttttgt 2040
ttgatagatg taagatttgg tatgtatagg tgatggttag aggttaggat gttgaaagta 2100
gattgtttga ggtagagtag ggttaagaat ttgggttata aggtagttgg gggttttaga 2160
tattttgggg tttgtttttg ttttttgata tttagggaag agaatatggg attgatagtt 2220
atggaagttg agtttggttt ttagttttgg tgttaatgtg ttttgtgatg ttgaagttat 2280
ttagtttttt tggattattg tttatttata tgtaaattaa aagggtttag atttgttaat 2340
gttttttttt ttatttaata ttagatagtt agggagagag tttatattta ttttattaat 2400
atttttttga gatatggtat tgttttgttg tttaggttgg agtgtagtgg tgtgattttg 2460
gtttattgga gttttaattt tttgggttta agtgattttt ttattttagt tttttgtgta 2520
gttgggatta taggtatata ttattatgtt tagttaattt tgtttatttt ttgtagagaa 2580
ggggggtttt attatgtggt ttaggttggt tttgaatttt tggatttaag tgattttttg 2640
attttgtttt ttaaagtgtt gggattatag gtttgagtta ttttatttta ttttatattt 2700
attttaaata gtagagtaga attgggttaa tttgtttttt tgtaagatta ttttggtatt 2760
atatagttgt ttgttttttg tgggtttagg ttttgtagtt aggagttttt taggtagtta 2820
tagaaggtta tttttttgga tttgtttgat ttggaaaatg aatagatgaa agttttttag 2880
tttgtggaaa tggatggagg gtgtttgttt tagtatttta gaaagttttg ttggtttgtg 2940
tttttagttt taattttgtt gttagtgggt ttggtttatt agatagattt ttagtttaag 3000
ttagagagaa gggaggttat ttttttgttg ttttttgaat tgaggtttaa ttggatattt 3060
atatggaata tggagtatat tgttagattt atgaatattt ttttggtttt attagaataa 3120
aagaattaga gtgtgggtgg tagtttgttt tgtgtgtggt aaggttatgg ggtagtatag 3180
tatagtggtt aagagtaagg attttgggtt gggtttattg gtttgtaatt ttagtatttt 3240
gggaggttaa ggtgggtaga ttatttgagg ttaggagttt gagattagtt tggttaatat 3300
ggtgaaattt agtttttatt aaaaatataa aaattagtta ggtatggtgg tatgtatttg 3360
taattttagt aagattttat tttaaaaaaa aaaaaaagga gtaaggattt tggatttaag 3420
ttgtttgaat ttgaatttta gttttttttt tatttgttgt gtgatttggt gtaagttatt 3480
taattttttt gttaaatgtg gtgattataa ttatatttat tttataagat tgttgttagg 3540
ttttagtgag tgaaatattt aaatagagtt tagtaattag tatgtgttga ataaatggtt 3600
gttgtttttt ttttttttat tatttttatt attattatta ttattattat tattattatt 3660
attatttagg aatttttggt tttgtttgtg gttgttatta attggtggag atttttaggt 3720
ttagtttttt tttgtaatga gaagtagtga ttattattgg tgatttttaa atattttatg 3780
attttgaaat tttttgtttt ttttgttttt taaaatagtt aaagtaaata aaataaaaaa 3840
ggtttgagta aagaaaggaa aaataggggt gtggagttta tttagtagtt tttttttttt 3900
tgaagtttta tttttggagt tagaattttt aagtgatttt ttagtttttg tgttttagaa 3960
ttttatgttt gtgtatgtgt agtgaaggtt ttagtgtttt atagattggg ttttatggaa 4020
tttgtttttt tttataatta gaagattgga gatataattt tttaaggtta gttagaaaat 4080
ttagagttag tatttagaat aaagttaaga tgttaatttt tatgggtggt atttgtagtt 4140
ttgggtatga gtttttttta ttttgttttt agggaggtta ttttttttgt attttaaata 4200

ttgagagggt agaattagtt ggtatagttt ttgtatgttt tataatttaa gatgtgatag 4260
ggtattaggt ttttgggatt ttttagggat tagtaatttt aggtattatg ttttattttt 4320
ggtttttggt ttttggtaaa tttttgtgat tttttttgat gatgtaatat tttgagtttt 4380
ttttaagatg tataagttat tgttttaggt ttggtagttt ttaaattatt ttggaaagat 4440
ttttgttttg taaattttgg ttttttattt ttatgtttga ttgttttgtt tttttgttaa 4500
aggtaaaagt gtattaagga tttagagaat tttagggtat gagtgtggga tgggattttg 4560
gagattatgg aagataattt ttttgtttaa ttagtagaga aataaggttt agaggtggag 4620
agatttgttt aaggttatat ggtatatttg tggtttagga gttgatgtta atttgtgtgt 4680
gtgtgttggt tgggggagta ttttgtgtaa tttaataggg tttgttgtag ttagatgata 4740
gggataggag aagaaagata taaatatatt atgatgttat tatagttaat agaaaattta 4800
attttttttt tttaagtgtt tttagtatgg attattaatg gtttaaattt tagataatgg 4860
agtttttagg attagttatg tttttgaggt agatggaagt tggaaatggg taaagtttaa 4920
gggagattta aaggatgatg gagtggaaga ggaaaattgg ttttggttta taaataagga 4980
gaaattaatt tgtaaattaa agtgttaaag ataatttatt tatttgtggt aattttggtg 5040
tttatttttt tttttttgtt ggtagaaatg ttgattttta atttattaga tttttgaggg 5100
aattagttta gaatggtgag ggaaggggtt aagagaagag tgtattattt ttgggataga 5160
aggtaatgag gagatgagtt tttatgtata aattatagtt attttttatt tataaatttt 5220
agaattttgt tttttttggt gtattttgga attttttgtg gtagaaggta ggatatattt 5280
gtagattata ttatttagat tttttaaatt agagatatat ttaatattga aggttattaa 5340
tgagttttta ttttttgttt tttaattgta atgttttgaa aggtttttag tttgggtttt 5400
attagtaata gttattaata tttttaggat ttagtatatg ttaggaatta tgttaggttt 5460
tttgtatgta ttattttatt taatttttat atttttttta ggatatagat attaggatat 5520
ttttttattg tataggagag gaaattgagg ttttgagagg ttgagtattt ttttaaaggt 5580
tatttagtgg tttagtggta gagttaagtt tagaattaga tttgtttttt tttgaagagt 5640
atgttttaat tttgatattt taagagtatt taatgagttg tgattttgga aatgtttttt 5700
tttttttagt ttttttttgt ttttattttg taagagaggt ggttgtataa aattttttag 5760
gatttttgtt aattttgatt gtttataatt tttaaatttt ttttggtgga ttttgagtat 5820
tttaggtttg atttttatgt tttttttagt tgttgatatt agtttgaaat ggtttttttt 5880
ttttttttga gtttaatttt tgatatttaa aagtaaagaa aaagtaagtg attgttttaa 5940
tatttttaat taatgtagtt aattgattta ttttatgtgt aaatttttta ttttgtagtt 6000
gaatattttt gattttaggg tgtgggtggt aggggtaatg gaattttttt tttttatttg 6060
atgaggattt tttgtttatg gtttgggatg ttagttttta ttttttttta gatgtttagt 6120
attaattgtt attttttgtt gttgagtttt atggtgtagt ttgaagaaga taaaaagagt 6180
aagtatgggt ttattgggtg ttttggtttt aatagttggg gtttgaggtt gtttggtttt 6240
aagggtgttt ttatattttt tgggatgttt gataaagatt gtttaatggt ttgttgtttt 6300
ttttttttgt ttttttttat tttttttttt ttatttaggg agggtttaat tttttttttt 6360
ttttagtttt tatatgggaa gttattttag ggtaggtgtt attgtggtga ggtaggatag 6420
tttaggttat agggggtggg gtggttgtgt gtgtggaaag aaggggagga ggttgggtat 6480
tgtttggatt tttatagttg atagaaaggg tattatagaa gttgatgtta gtagtttgaa 6540
atttaatgtt ttttgtggtt agttttttta agaggatttg ttatgttttt aagtttggtt 6600
attaggtgtt ttggttggtt ttaggatagt tttggttatt tgtaattttt tatgtatttt 6660
ttgttttttt gttagagatt ttttatttag ttagaagtga tagaaaggaa ttggatggtt 6720
ttgtgtttat agttaggtgg ttttaggtaa gtttataatt ttttagagaa tttttgtttt 6780
tatgattttg tttaaaggtt tttttttgaa aagttttttt tgatatattt ttttgagata 6840
ttattaattg tttttttttt tttttttttt gtgtaagttt ttgttgtatt gttttttaaa 6900
gggtatttta ttttttggtt tttatgttat tttttttatt aggataatag tagttagtat 6960
ttataagaga ttttgttggg tgttaggttt tgttttaaat atttggtatt tggtaattta 7020
tttaattttt gtaagatttt ttaatgtgat aggtattgat attattttta tttaattgag 7080
agaaagtagg gaaatattat tgatgtataa ttagttgttg gtagagttgg gatttgaatt 7140
taggaagtta tgtttttttt tttgtagttt ttaggagggt aggagttttg ttttattaat 7200
ttatgtattt tttgggtttt atatggtgtt ttgtgttaag tttgaattga ataaatgttt 7260
attgagtaaa tgggtggatg gttttgtaaa gaaagagagt ggaggatgat tagttttatt 7320
ttatatttat gtattttttt ttaatttatt taggtattta attatttatt tatttattta 7380
tttgtttatt tagttattta tttatttatt tatttgttat tttttaattt atttatttat 7440
tatttttttg tttatttatg tatttattta tttatttatt tattatttat ttatttaata 7500
tttattaatt tttttattta tttgatattt tttaatttat ttatttattt atttattatt 7560
tttttgttta tttaggtatt tatttattta tttatttatt tatttattta aaatttatta 7620
gtttttttat ttatttattt atttatttat ttatgtgttt atttatttat tttttaattt 7680
atttatttat ttattatttt tttatttatt tatttattta tttttttatt tatttattta 7740
ttattttttt atttattatt ttttttttta tttatttatt tatttattat ttatttattt 7800
ttttatttat ttatttgttt ttttattttt ttatttattt atttatttat ttttttattt 7860
atttatttat tattttttta tttattattt ttttttttatt tatttattta tttattattt 7920
atttattttt ttatttattt ttttatttat ttgtaatttt atttatttat ttattttttt 7980

tatagtgata gagtatttat tgtgtgttag atattgttat atgttgagga taataaaatt 8040
aaataaaata tttttttgtg tttaaggaat ttagagtttt tttagggaga taattaagag 8100
gaatgaaaat atagtatagt ttgtttagtg ttatgattaa ggtaggtata ggtttttttt 8160
tttatttgta taagattata tagtttataa agtggttgtt tggtaagatt atatttaatt 8220
tttataatat ttttgtgagg tagtgagtat tattttgttt tatagttgtg ggagatgaga 8280
tttagaaagg ataagttagt gtttgggtta ttttgggttt gtattggatt tgttagtaat 8340
gattgtttta tttatatagt tttttgaaga taattgtttt gttttaattt tgttgaggtg 8400
taggtgtagt tagttatgtt ttgggatata tagttttttt gtaaatttgt tgttagttat 8460
agttaagttg agaaagttaa tgttagggtt ttatttgaat agttagtatt tattagtttt 8520
gttagttaga ttttagtttt agaatgtgaa tagagatgtt tggtaagaat tggataaata 8580
ataggaaatg tagttagaaa aagagttaaa tttaaagaat ttttaaattt ggagtttttt 8640
agtgtaattt tgggttagaa tgttttttgt gtgggttttt aaagtttggt tggtgattat 8700
tttaggattg ttatgagttg ttattttttg gaggagtttt gtgtttttat ttagtgagtt 8760
ttgtgtattg agttggttga tttttagtga ttaaaagggt tagaggtatt ggttgtattt 8820
ttggtgtttt ttttttagtg attattttag aagttaattt taaatgttag gttagttgat 8880
ttttaaaggt ttattttttt agtagtggaa gaagtagtaa taaaataagt agttaaaatg 8940
ttatgggggg ttgggtgtgg tggtttatgt ttgtaatttt agtaatttgg gaggttgagg 9000
tgggtggatt ataaggttaa gagattgaga ttattttggt taatatgttt aagttttgtt 9060
tttattaaaa atataaaaat tagttgggta tggtggtatg aatttgtagt tttagttatt 9120
tgggaggttg aggtaggaga attatttgaa tttgggaggt agaggttgta gtgagttgag 9180
attgttgtat tttagtttgg taatagagtg agtttttatt ttaaaaagaa aaaaagaagt 9240
tatggggatg ttaaagggga ttttatttat ttatttagtt agttagttat ttgtttgttt 9300
ttttgtttat ttatttattt gaaagtttat tattgaggtt ttagtttgtg ttaggtttag 9360
ttttagaagg aaagataatt ttgagagggt ttgttttggt tttagatttt tagtagtttg 9420
atttttattg ttggttattt gttggaggtg agtttagggt gtgagagagg agggtagaaa 9480
gaaggagatg gtattgagtt gtgttatatt tgatttgggg ttttttgttt ggtgtgttgg 9540
ggaaggttaa gaggtagatt tgagattttt ggagattgag atgtaatgtg tggtttttaa 9600
atgttttttg gtttgtgttt ttttgagagt gttagaatat ttttgagggt gtggggtagg 9660
gtagggttga ttattatttg aattttagtt ttttttttgt ttttggtttt ttgagttgtt 9720
tggggatggt ttgagtattt aaaattttat taatttatta ttgatgttga aatgtttaat 9780
atgaagttat tatttgtttg gtaagtgttt ttattaattg gtatgtttat ggaaatttgt 9840
agttttgaag ttgatggatt ttttaagttt tattatttaa gttaatggga aggtattgat 9900
atttgtttgt atttatatat ttttataata gagtagtgtt attttatagt tgttatggtt 9960
atttggagtt attagttttg tttgggggtt attattattt ttgtgatagt ttttattatg 10020
gttagggtag agaagttgta gatatatgat aggaaagtta ggttgttttt gtgtgtaaaa 10080
ttttttatag ttttatatgt ttatgttata tatatgtata tatatgtatg aatgtagggt 10140
atattagttt gatatttagt ggggtaaaat tggattttat taagttagat tttttttttt 10200
ttttttttat attataggat tttttgaatt taaaattgag atttgtagaa ttatttttaa 10260
ggtttttaat tttggaattt tgagatttta gaattaaaat tttagagtgt taaagttttt 10320
aattttttag tattggagaa atttgggttt tagattttag atttatagag agttagaagt 10380
agagaaattt agaattatag ggttatagta ttttgtgatt aaaagaggtt tttaatggta 10440
ttttgtttaa ttttttatta aatatagttt tttttataga ttattatttg gtatttgttt 10500
gaatattttt gggagtggtg agtttattat ttaaagtttt aaaaattttt ttttatgttg 10560
aattaaaaga tgtttttatg ttatgtgtat ttttttgtgg tagttttgtt tgttttttgg 10620
ggatatagag gtttagttga gtttgtttta taagagaagg agtaggttta tttaagattt 10680
tttttgttta gattgaatat tttaggattt ttattgagta ttttttttag gtaaggtaag 10740
ttgggggttt tagtttggtg gagaaagatg ggttttgtta tttttggttt ttagttattt 10800
ttatttttgg ttatatgtat ataaagagaa tgttatagta aaggggattt tagtatttat 10860
ttgtataggt ttgagttttt taggtagttg tgtatttggt ttatagtttt tatggttaga 10920
aaatttaatt taggtgattt aggaatttgt tagtttttgg atagttgata attttttttg 10980
gtaaatttgg gtttaagata ttattaaggg agattaaaga tgtggtaaga aggagaattt 11040
tggtttttta aagttaggat tttttatgat ttttattatt tttttttttt tttttttggg 11100
aattagtttt tggaggttag tttgggtagt gttttttttt tgttagtagg tttttttatt 11160
ttatttttag tttagtttgt gtttttttgg tagagtttat ttttttggat tagtgttagg 11220
tt 11222

<210> 375
<211> 5952
<212> DNA
<213> Artificial Sequence

<220>
<223> chemically treated genomic DNA (Homo sapiens)

<400> 375

atatttttga tttttatatg agtatatgtt tgagtagttt ggattatagg tgtgtagtat 60
tatatttagt taatttttaa atttttgtgt agtgatgaag ttttatttta ttgtttaggt 120
tggttttaaa tttttgggtt tgagtaattt ttttatatag gttttttaaa gttttgggat 180
tataggtatg agttatagta tttgagtaag atgatttttt aagataggtg aagatgttag 240
tgtttagaat tttattttta ggggtatgta ggtatgaaag gttatattta agtgagtgtt 300
atgttagttt ttttagaaat ttagttgttt ttttttaaat ttagtttaat aaaaggaggt 360
ttggagaatt aatgtatata agttagaatt atagaatgta tagtatgatt attaggatgg 420
ataatatgtg gtttgaataa attttttatt tattatattt agttttttta ggttaatgga 480
atttagatta agtatatttt tgaagttagt attgtgtgat gaataggggt ttggtggaag 540
tgatgttaat agggttttag gttagtttta gagttttttt tttttttttt ttttgttttt 600
tgtttttttt ttgtttgttt tttgtttttt ttttttagat agtttttttt ttgttgttta 660
ggttggagtg taatggtgtg attttggttt attgtaattt ttgtttttta ggtttaagta 720
atttttttgt tttagttttt tgagtagttg ggattatagg tgtttgttat tatagttggt 780
taatttttgt atttttagta gagatagggg ttttaggtta ggttggtttt gaatttttga 840
ttttaggtga tttgtttatt tgggtttttt aaagtgttgg gattataggt attagttatt 900
gtatttggtt taagaaatat ttttaagtat gtttgatttt ggtaaatgtt tttgattttt 960
tttgtaggaa aaaattttag agattgaatt tttgtgttaa agaataagta aaattttaag 1020
gtttttgata tatattttta agtttttatt agaaaaatta tattaatgta tatttttatt 1080
tgtaatgtat ttaagtgttt atttttttga atttttttta atatattagg tgttaatatg 1140
atttttatta atttggtagg ttaaaatttt gtttttattg aatttatatt tttttgttta 1200
ttaatgtggt taataaattt tatgtttatg aagattagga ttggatagtg gaggttagat 1260
atttttggtt ttttgtttag atgaataaat tttttatttt ttttaattta tttaagttga 1320
gttttttgtt attgaaattt aaattatatt aattaatata attttttgag gtatattgaa 1380
gttttaaaat ttgtatggtt atttattatg ttttatgggt ttttttttta tgtttggaag 1440
tttataggaa gattttataa atatttatta aaatattttt ttatttttta ttgggttaag 1500
atttgaattt gaatttttta tttagaattt agtgaataat tattaggaaa agtttttttt 1560
tttttttttg agatggaggt ttgttttgtt atttaggttg gagtatagtg gtgtaatttt 1620
ggtttattgt aatttttgtt ttttgggttt aagtaatttt tttgtgttag tttttttagt 1680
atttggaatt ataggtgtgt attattatgt ttagttaatt tttgtatttt tagtagagat 1740
ggggttttat tatgttggtt tggttggttt tgaatttttg attttagatg atttatttat 1800
tttagttttt taaagttttg ggattatagg tatgagttat tgtgtttatt tagaaaagtt 1860
tatagttttt attttttttt aatagtttaa ttaattattt gattaaaatt tattttattt 1920
ttttttttat tgatttaata gtatttttat tagatgttag agtagttatt taaaatgtaa 1980
attggattat attaaagatt tgttgttgat tatatttttt tgtattttta gagagtttat 2040
aaagttttgt aagagtttat tttagttttt ttgtaatttt attttgtatt atttttgttt 2100
tagatatatt agtttgagtt ttatattaaa tttgtttgaa tatattttaa gtatgttgat 2160
ttttataatt atgttatttt tttagtttag aatgtttttt tttaattttt taagtagttg 2220
ttttttttga taagatgtta tttttttaga gaggtttttt ttgattatag ttataggtat 2280
gtttatgttt ttattttagt ttaatataaa atttatgtaa ttgtatattt ttttattatt 2340
tgatattttt aggaggatat tatttttgta atagagtaga aataaataga ttgatgtata 2400
tggtggattt tttatggata gagtttgtta tttaaaatgt ttttaaattt attatatgta 2460
atttggtatt aaattaaaat gttattatgt aagtgaatgt ttttaaagtt taaaagtttt 2520
tttaaaataa aatagatttg ggtgtggtgg tttatgtttg taattttagt attttgggag 2580
gttaaggtag gtagattata tgaggttagg agtttaagat taatttggtt aatatggtaa 2640
aattttgttt ttattaaaaa tataaaaatt agttaggtgt gatggtgtat gtttgtaatt 2700
ttagttattt aggaggttga ggtatgagaa ttgtttgaat ttaggaggtg gagattgtgg 2760
ttagttaaga ttatattatt gtattttagt ttgggtgata gagtgagatt ttgttttaga 2820
aaaaaaaaaa agaaaaagaa aaagaaaaaa aaaaagaaat tttaagtgat aaagtattgt 2880
gtaatagttt gaggttgggt atagtggttt atgtttgtaa ttttatattt tgggagattg 2940
attgggaaga ttgtttgagt ttaggagttg gagattagtt agagtgatat agtgggattt 3000
tttttttata aaaatattta aaaattagtt aggtatagtg gtgtattttt gtagttttag 3060
ttatttggga ggtggtaaga ggattttttg agtttagaag ttggaggtgg tagtgagtag 3120
ttattttgtt attgtatttt agtttgggtg ttaaagggag attttttttt ttagaaaata 3180
ataaaaaaaa aagtttgata gtttttttgt ttgtgttata taaaataatg gtatatatta 3240
taattgaagg tattttataa tagaaatgtt tttttttgtt tttttttaag gttgatttag 3300
tgttttaaga ggttgatata gaagggtaaa gttaagtttt tataaaattt agggaagaga 3360
ttgtgaaatt tattttagaa ttttgtatgg agttatagtt gaattaggaa aaaaaaaaaa 3420
aaaaaaaaaa ggaaagaaag gtattttaga gataaagaat agattaagga gaggagatat 3480
ttgaagttat aatgattgag aatatgttag atttgaaatg aatttttagt ttttttaata 3540
agataaataa aattaatttt atatttagat atatggtaga aaaattaaag aattttgttg 3600

attaaggtat taaaagtaat taaagagaag tggtgtgaag aaagtaagag agaaataata 3660
aattttgttt attttgtaat aattgaaaat ttttggttgg gtgtggtggt ttatgtttgt 3720
aattttagta ttttgagagg ttgaggtagg tggattattt taggttaggt gtttaagatt 3780
agtttggtta atatggtgaa attttgtttt tattaaaaaa aaataataat aataatataa 3840
aaattagttg ggggtggtgg taggtatttg taattttaga tatttgggag gttgaggtag 3900
gagatttatt tgaatttggg aggtggaggt tgtaatgagt tgagattgtg ttattgtatt 3960
ttagtttgga tgatagagta ggattttatt ttaaaaagaa aggaaaagaa aagaaaaaat 4020
attaaatgtg tatgtttttt gatttagttg tattatttta aggagttgat attattaaaa 4080
ttgtttaagt gtttaaaggt gtttgtagtt aaataatagg agattgataa attatgttat 4140
atatatgtga tgttatgttt taaagaggta ttgatatgat aaaagatgta tgtggtataa 4200
aattaaatgt attttattaa gtattttttt aagtgtttat ggaatgagtg tatttttgaa 4260
aaaaaaaaag tgtatttgaa tttttaaaaa agtttaaaaa gttttatata atgaatgatt 4320
gagtgattat aagagttggt gggggaatgt taagaggatg atagggagtt aagtttaata 4380
gaataattta tttttttatt ttgtgatatt tatgagtgta ttaattttgt aattgaaaaa 4440
taaagtgtat atttgtagta gttgtatttt ttttaggttg taaggaggtt tttttttttg 4500
gtaggtttga tttgtatttt atttttattt ttgtggttgg aaatttttta tttatgtagt 4560
gggaggttga ggagttatta taaagttggg gtttgatgag ttgggattgg gatttgattt 4620
ttatatatgt ttggatttgt tttgtggttg ggtttaggag ttaaagaggt ggggagattt 4680
gtgtgatgtt gttttgtttt gtgtttgttt tttttaatgt atgttttagg gggtgggttt 4740
tgtggggagt atggatatga ttggttttaa agtttttttt gtaaggttgt gggttggata 4800
gtgtggtgat gttgtaatgt ggtgtagggt gagagtgtgt gtttgtggat gtggtggtat 4860
taaatggttg taggtgtagt agagtggttg ttgttttttt aggttttagt tggttgttgt 4920
gatgtttgtt tgtttgtttt gaggtttttg aagttgaaat tagttagatt tttttttttt 4980
ttgtttgttt gtagtggtgt tgttgttatt ttgttattat gtttgaggtg tgtttggttt 5040
agggttttat ttttaagaaa ggtgttggag gtatttaagg attttattaa tgaggtttgt 5100
tgggatatta gttttagtgg tgtaaatttg tagagtatgg atttgtttta tgtttttttg 5160
gtgtagttta ttttgtggtt tgagggtttt gatatttatt gttgtgattg taatttggtt 5220
atgggtgtga attttattag gtgagttttg tggttttggg aagttggttt tggtttgttt 5280
gtatttttgg tgtttggtgg gagtgttttt gagtttagtt tttattggtt ggtgtgggtt 5340
atttgtgttt ttttattggt ttgttatgta gtggggtggg gtttagttga gtgtgtggtt 5400
tggaaaagtt tgtgttggtt gttgtgtgaa tttgtttttt tgtgttaaag ttataaagtg 5460
ggtggtggtg ggaaaattaa gggttttttt gtagtgttag gaatattgtt ttagggtttt 5520
ttgtttatta aattttgttg gttttgaatg gatgttttag ttgtggtttt tttgtttttg 5580
agatggtttt ggtgtgttgt ttgggttggt ttttaatttt tgggtttaag tgattttttt 5640
ggtttagttg tggttgattt taaatgtttt ataatgtttt tgtgagaaat gtggtagttt 5700
gttattttat ttagtggtag gagattgttt ttatttagaa gggatattgt tggtggtatt 5760
ttagtataaa tattgttaga tgtgttttaa aatgtttgta ttaataatgg tattttttag 5820
tagtttgttt attttttatt agttttgaga tggtttgatg ggtgagagtg gtaatttttt 5880
ttaattgtgt ttgaaatata gttttttagt agatggtgtt gattttaaag tatgtgtttt 5940
ttgtttttta gt 5952

<210> 376
<211> 5952
<212> DNA
<213> Artificial Sequence

<220>
<223> chemically treated genomic DNA (Homo sapiens)

<400> 376

attagaagat aggagatata tgttttaaaa ttaatgttgt ttgttgaagg gttgtatttt 60
gaatgtggtt agaaggggtt attattttta tttattaggt tgttttagaa ttggtggagg 120
gtaagtggat tgttggagga tgttattatt aatgtagatg ttttgggatg tatttggtag 180
tatttatatt aaaatattat tagtagtgtt ttttttggat agaaataatt ttttgttatt 240
aagtaggatg ataggttgtt atattttttg taagggtatt ataaagtatt taaagttaat 300
tgtgattgag ttgggaggat tgtttgagtt tagaagttgg agattagttt gggtaatata 360
ttgagattgt tttagaaata agaaagttat agttaaagtg tttatttaag gttaatagga 420
tttagtgagt aaagagtttt ggaatagtgt ttttggtatt gtggaaaaat ttttgatttt 480
tttgttatta tttgttttgt gattttggtg tgaaaaagta ggtttgtgta gtagttagtg 540
tgggtttttt tgaattgtgt gtttagttgg gttttatttt attgtgtggt aggttaatga 600
gaaggtgtgg atggtttatg ttagttaatg agggttaggt ttgaaagtgt ttttgttaag 660
tattggaggt gtaggtgggt tggggttggt tttttggggt tgtgaggttt atttggtgag 720

gtttatgttt atggttaggt tgtggttgta gtggtaggtg ttgaagtttt tagattgtag 780
ggtgagttgt attaaagaga tgtgggatga gtttatgttt tgtaggttta tattgttgga 840
gttaatattt tagtaggttt tgttgatgag gtttttgagt gtttttaata ttttttttga 900
ggatggagtt ttggattagg tgtgttttga atatggtggt ggagtggtaa taatgttgtt 960
ataggtaggt gggaaggagg aaagtttagt tggttttggt tttaggagtt ttagagtgag 1020
tgggtgaatg ttgtgatgat tggttgagat ttagaaagat aatgattatt ttgttatgtt 1080
tgtaattgtt taatgttgtt gtgtttgtaa gtgtgtgttt ttattttgtg ttgtgttgtg 1140
atgttattat gttgtttagt ttatggtttt gtggggaaga ttttagggtt aattgtgttt 1200
atgttttttg tgaggtttgt tttttagagt atatattgga ggaagtgggt gtagggtggg 1260
gtagtgttgt gtaggttttt ttgttttttt gatttttgaa tttggttgta gaataagttt 1320
gggtatatgt ggagattggg ttttggtttt ggtttgttaa gttttagttt tatggtggtt 1380
ttttagtttt ttattatgtg ggtagaaagt ttttagttat gaaagtgaaa gtgaaatgta 1440
aattaagttt attgggagga aaagtttttt tgtagtttga agagagtata gttgttgtaa 1500
atatgtattt tattttttaa ttatagaatt gatgtgtttg taggtgttat aagataaaga 1560
ggtgaattgt tttgttaaat ttagtttttt attatttttt taatattttt ttgttagttt 1620
ttataattat ttaattgttt attgtataaa gtttttaaag tttttttaaa agtttgaata 1680
tatttttttt tttttaaaaa tgtatttatt ttgtaaatat ttggaaaagt atttaataaa 1740
gtatatttaa ttttatgtta tgtatatttt ttattatatt agtatttttt taaaatatag 1800
tattatatgt atataatata atttattaat tttttgttgt ttaattataa atatttttga 1860
gtatttaggt aattttggtg atattaattt tttgaagtaa tatagttgag ttaaagagtg 1920
tatatattta atattttttt tttttttttt tttttttttt gagatggagt tttgttttgt 1980
tatttaggtt ggagtatagt ggtgtgattt tagtttattg taatttttgt tttttaggtt 2040
taagtgagtt ttttgtttta gttttttgag tatttgggat tataggtgtt tattattatt 2100
tttggttaat ttttgtatta ttattattat ttttttttag tagagatggg gttttattat 2160
gttggttagg ttggttttga atatttgatt tgaggtgatt tatttgtttt ggttttttaa 2220
agtgttggga ttataggtgt gagttattat gtttagttag aaatttttaa ttgttatagg 2280
atggatagga tttgttgttt tttttttgtt ttttttatat tatttttttt tagttatttt 2340
taatattttg gttagtagaa ttttttagtt tttttattat gtatttaggt atggagttag 2400
ttttatttgt tttgttgagg gaattgaaga tttattttaa gtttgatata tttttaatta 2460
ttatggtttt aaatattttt tttttttagt ttatttttta tttttggaat gttttttttt 2520
tttttttttt tttttttttt tttttttagt ttagttgtga ttttatataa ggttttaaga 2580
tgagttttat agtttttttt ttggattttg tggagattta attttatttt tttgtattag 2640
ttttttaaag tattgggtta gttttaaaag ggaataggaa agggtatttt tattataaga 2700
tgtttttaat tgtaatatgt attattattt tatgtagtat aagtaagaaa attgttaaat 2760
tttttttttt attatttttt agagagggga gttttttttt gatatttagg ttggagtgta 2820
gtggtaagat gattatttat tgttattttt aatttttagg tttgagagat ttttttgtta 2880
ttttttaaat agttgggatt ataggggtgt attattgtat ttggttaatt tttaaatgtt 2940
tttgtagaga gaaggtttta ttatgttgtt ttggttggtt tttaattttt gggtttaagt 3000
aattttttta attggttttt taaagtgtga gattataggt atgagttatt gtgtttagtt 3060
ttaaattgtt atataatgtt ttattattta gaattttttt tttttttttt tttttttttt 3120
tttttttttt tttttgagat agagttttat tttattattt aggttggagt gtagtggtgt 3180
gattttggtt aattgtaatt tttgtttttt gggtttaagt aatttttatg ttttagtttt 3240
ttgaatagtt aggattatag gtatgtgtta ttatatttgg ttaatttttg tatttttagt 3300
agagatgggg ttttgttatg ttggttaggt tggttttgaa tttttgattt tatgtgattt 3360
gtttgttttg gttttttaaa gtgttgggat tataggtatg agttattgtg tttgggttta 3420
ttttgtttta aaagaatttt tagattttaa aaatatttgt ttatataata atattttggt 3480
ttaatgttaa attatatgta atagatttga agatatttta aatgataaat tttatttatg 3540
ggggatttat tatgtatatt aatttgttta tttttgtttt attatagaag tgatgttttt 3600
ttaaaagtgt taaatgataa agaggtgtat agttatgtaa attttatgtt aaattgaaat 3660
gaggatataa atatgtttat gattgtggtt agagaaggtt tttttgagga ggtagtattt 3720
tattagagaa gatagttgtt tggagagttg gaaaaaagta ttttaggttg agagaatagt 3780
atgattataa aggttagtat atttggaata tatttagata agtttgatgt agaatttaga 3840
ttgatgtgtt tgaagtaaga gtggtgtaag gtgaggttgt agagaggttg gggtgagttt 3900
ttgtaggatt ttgtaggttt tttaaaagtg taagggaatg tggttaatag tagatttttg 3960
atgtgattta atttatattt taagtggtta ttttaatatt tagtaaaggt attattggat 4020
tagtgggaga aagaatgaag taaattttga ttagataatt ggttaaatta ttagaaaaga 4080
ataaaagttg tagatttttt tggatgggtg tagtggttta tgtttgtaat tttagaattt 4140
tgggaggttg aggtaggtgg attatttgag gttaggagtt tgagattagt taggttaata 4200
tggtgaaatt ttgtttttat taaaaatata aaaattagtt gggtgtggtg gtgtatattt 4260
gtaattttag atattaggga ggttgatgta ggagaattgt ttgaatttgg gaggtagagg 4320
ttgtagtgaa ttaagattgt gttattgtat tttagtttgg gtgatagagt aaatttttat 4380
tttaaaaaaa aaaaaaaaga ttttttttaa taattattta ttaaatttta gataaagaat 4440
ttaaatttaa attttgattt aataagaaat ggaaaaatat tttggtaaat atttatgaga 4500

tttttttatg ggtttttaaa tataagaaaa aaatttataa aatataatga ataattatat 4560
aagttttaag attttagtat gttttaaagg attatattgg ttagtatagt ttaggtttta 4620
gtgatagaga atttaatttg aatggattaa gagaaataga aaatttattt atttaaataa 4680
aaagttagag gtatttggtt tttattgttt agttttagtt tttataaatg tgaagtttgt 4740
tagttatatt aataagtaaa gaaatgtaaa tttaatggag atagaatttt aatttattaa 4800
attaataaaa attatgttaa tatttggtgt gttaggaagg atttaaggaa gtaggtattt 4860
aaatatattg taagtgggag tatatattgg tataattttt ttggtggaaa tttagaaatg 4920
tgtattaaaa gttttagaat tttatttatt ttttgatata gaaatttgat ttttagggtt 4980
tttttttata gaagaaatta ggggtattta ttaagattaa gtatgtttaa gaatgttttt 5040
tgagttaggt gtagtggttg atgtttgtaa ttttagtatt ttgggaggtt taggtgggtg 5100
gattatttga ggttaagaat ttaaaattag tttgatttaa aatttttgtt tttattaaaa 5160
atataaaaat tagttagttg tggtggtggg tgtttgtagt tttagttatt tgggaggttg 5220
agataggaga attgtttgaa tttgggaggt ggaggttgta gtgagttgag attatattat 5280
tgtattttag tttgagtaat aagagggaaa ttgtttaaaa aaaaaaaata aaaaataaat 5340
aaaaagaaaa taaaaaatga aaagagagag agagaagggt tttaaagttg gtttaaagtt 5400
ttattagtat tatttttatt agatttttgt ttgttatata gtattgattt taggaatata 5460
tttgatttga gttttattaa tttgagaggg ttgagtgtaa tggatgagaa gtttgtttag 5520
attatatgtt atttatttta gtaattatat tgtgtatttt ataattttga tttatgtgta 5580
ttgatttttt aagttttttt ttattaggtt ggatttgaaa aggagtagtt gagtttttag 5640
aaggattgat atgatattta tttagatatg attttttata tttatgtgtt tttgaaggtg 5700
aagttttaga tattaatatt tttatttgtt ttagaagatt attttgtttg agtgttgtgg 5760
tttatgtttg taattttaga attttaggag gtttatgtgg gaggattgtt tgagtttagg 5820
agtttaagat tagtttaagt aataaagtga gattttattg ttatataaaa atttaaaagt 5880
tagttgggtg tggtgttgta tatttgtagt ttaagttatt tgggtatata tttatataga 5940
aattaggaat at 5952

<210> 377
<211> 6664
<212> DNA
<213> Artificial Sequence

<220>
<223> chemically treated genomic DNA (Homo sapiens)

<400> 377

tattataagg gtattaattt tattaatgag ggaaaagttt ttgaggttta ataattattt 60
tttgaaggtt ttatttttta atattattat tttggggttt aagttttaat atatgtattt 120
aggaggggta tatattttta aattatagta atttgtaagt gatatatatt ttaggtttta 180
tggaatttat atttattttt ggttatattt agttatgtgg atgttttata gtattttatt 240
attaatatgt ttaaagtgat gttttatgtt ttatagttgt tttttagtat ttttttagaa 300
tagtaggatt tttagtttgt tgtaaagtta tatatttaag ttatttttga tgattttttt 360
tttttttttt aatttagttt tagtttttaa aaatatttta ttgttatttt ttttttttga 420
aattgatatt tttttaagtt tagtatttgt tgggattgtt gtagagtttt ttattgtttg 480
ttggttttga tgttgtatta ttagtttttt ttatgatttt ttttttttaa tttttattta 540
tgattatgtt attgttttgt ttatattttt ttgtttaagt ttaatgttta atttaaaatt 600
tttagtatga ttttgaatat tatttttaag gtatagatat tatttaatta tttagtttaa 660
tgtttatgat ttttgttgag tatttagatt ttgtttatgt ttagtgtatt gaattaattt 720
tagtttataa atttgtattg tttttttttg ttaatggatt aagtttttta tttttatttg 780
tttttttggt aaattttttt tattttttaa gatttagttt agttaatttt gttatgaagt 840
tttgtttgat gtttttttta ttatgttttg tatatagtag tttttattat attgtattgt 900
aaatatttgt atttatttta ggtggaagat ttatagttat ttatttgtta tttttgattt 960
ttattagttt taaggttgat atatgaattt ttttttttta tattatgttt agtttttaaa 1020
attttaatga gagattaagg tgggttgata tgtatgtatt taaagtataa ttattaatag 1080
gtttaaagtg attttttttt ttttttgtgg ggtgagaatg aagatagggg gtatggaggt 1140
tgtatatgat aaatttttat tatttgtatt ttatattttg gattattttt taaaatttat 1200
agaaaaatta tattattttt ataattagaa aaatatttaa ttttgttata gtaatgatag 1260
tttagtattt ttatttgtgg tgaaaagatt tgaatttata tattttgtat tttttgttat 1320
tttttttttt gaattaatat gaaaaaggat aaattttttt tttttgtata tattttttgg 1380
taatttttaa attttttttt tttttttata tatttgtttg tatagagata gggttttatt 1440
atgttgttaa gttggtttta aatttttggt tttaagggat tttttatttt ggttttttaa 1500
agtgttggga ttataggtgt gagttattgt gtttggttaa tttttattat ttttatttag 1560
tagtaatgga ggttttggta agattgattt atttatttat ttatttattt gtttattttt 1620

gggttagggt agtatattta tgtggtttaa tatttaaaag atataaaatt ttttttttta 1680
tttttgtatt aggttatttt tgtattttat agagggtata tttttattag ttttttttat 1740
tttttttttg aagtgtattt tttttttttt tttttttttt ttagaaggag ttttgtttta 1800
ttattaggtt ggagtgtagt ggtgttattt tggtttattg taatatttgt ttttagggtt 1860
taagtgattt ttttgattta gttttttgag tagttgggat tataggtatg tgttattatg 1920
tttagttaat ttttgtattt ttagtagaga tgggttttta ttatgttggt taggatggtt 1980
ttgatttttt gataatgtga tttatttgtt ttggtttttt agagtgttga gattataggt 2040
gtgagatatt gtgtttggtt tggaggtgta tttttaagta aatgttttta tatttttttt 2100
atttaaaaaa aatatttgtt ttgatttggg ttgttataat aaaatgttat agattatgtg 2160
ttttaaataa taaaatttta tttttattta gttttggagg tttggtgtgt aagtttagag 2220
ggtgggtaat attaggtttt ggtaagggtt gattttttta ttgataggtg tttttttatt 2280
ttaattttat atgagagtaa ttaagttttt tagtgttttt ttttataaga gtattaattt 2340
tattatgagg gttttatttt tatgttttta tttaaattta attatttttt aaagttttat 2400
ttttaaatat tattttagtg gggtgaggtt tttaatgtat gaattagtgg ggtgggggga 2460
taataaatat ttagattata gaaatatgta aatataagaa ataaaattaa aaagatttat 2520
gtgtaatatt aattgatttt tttttttttt tttaaaatgg agtttattaa aatataaata 2580
tgaagttttt tagagtatat attttttttt gttttttttt gtagtttttt tttttttaat 2640
attattttta gggtagtttt tttaaaatat ggttttaagg ttgtgtgtat tttttttgtt 2700
tagatttttt ggaatagtgt ttagtgttta ttaggatttt gattgtaaat tttttttttt 2760
gattttaata ttataggatg tagtattttt tatattttat tatggggggt attttgaatt 2820
atttgggttg attttttggt ttttgtgttt tatttatata attttttttg tatggaaggt 2880
ttattttaga attttttttt tagtttttgt ttaaatgtta tttttgttag tattagtagt 2940
tttttttttt gggtttttat attgttttgt aatataagtt ataaggtaag tattagaaga 3000
gaatttttaa ttattatttt gtttttttat ttttaatatt tttagtggta attgtatttt 3060
atttattttt gtgtttatga agtatagtgt tgtattttgg agatattata tagaagttgg 3120
tttaaagggt aataaattgt atttgttaag atgatagtat ggatttataa taatttttga 3180
aattttataa agaaagatga ttgagttaaa ttgatatatt tagtattata gttgtggaag 3240
tttgtggttt tttttatgtt aaaggtaaaa tggatataat gaattagttg ttatttttag 3300
atgatataga agttgaattt tttttaagtt ttttaaagag aaagatatat taaaatttat 3360
taaaattagt attataaatt gtatagaata gtttggtttt ttagagtatt tttttggggt 3420
agtgagtttt tatttaaaaa atgaagtttg gattttaaat ataagtttta aaagtgatta 3480
ggttttagta ggagttttta aatttttatt tatattatga tttttaaatg ttttgttttt 3540
gttgtttagg aataaaaatt gttaaggttg agttagatgt aatgtataat attagatgat 3600
agatgtagtt tattgtgtta aaaggtgtta aatatttaag tgtatgtgat atattttata 3660
ttataagggt aaggaagttt tattttatat tttgatttgg gttttttaat atatatttgt 3720
agtggatgtg gtaattagtt tttgttttat gttttattag gattaatgta attagaaatt 3780
tttaaaatgg atttttatta tgtttagttt tgttttattg ttttatttgt ttattttaaa 3840
aagattttag aatggtgttt aggttgggtt ggtggtttat gtttgtaatt ttagtatttt 3900
gggaggttaa ggtaggagga ttgttggagt ttaggagttg gagattagtt agggtaatat 3960
aaggagatat tgtttttgta aataaataga aaaaaaaaaa gtaagaaaga aagagagaaa 4020
gaaggaaaga ataagaaaaa ggaaaaaaaa attaattgta gtaagtgtag aaattttttt 4080
taaggatatg atggatggta atgaagatag agtttttgat attggattat ttaaagtata 4140
atttaagttt tttttttttt ttttatagaa ttgtgaattt ttgtgtttta attatattta 4200
agttaggtgt ggtggtgagt gtttgtagtt gtagttgtgt tggaggttga ggttgattgt 4260
ttgagtttag gatttggagg ttagtatgtg taatataatg agatttagtt tttaaatgta 4320
tgtttttttt tatatattta aaattttgat gtgaaaatat tttaaaattt aatatatttt 4380
aaatgttttt aattgtataa taaataaaat gtaaataata aaataattta atattaaatt 4440
taaaaatgag gtagaaataa agtatagtga tataaataat aaattttttt ttatattttt 4500
gaggtggttt tttgagtttt ttattttttt tttaaggtta ttgaaatgtg ttttttggag 4560
ttagtttgta aattatgtat ttagaaaaat ataattatat atttttaatt ttaagtatta 4620
gaagtgaaag taatggaatt ttgatgtaaa tataatatta tttttttgat gagttatttt 4680
gagtataata aatttgaatt gtgttaatgt tgggagaaaa aatttaaaag aagaatggag 4740
tgaatagtag ttttttgttt tgttgattag aaatagtagg atgatatttt tttgattgga 4800
ggagagtgtt tgtgtttgta tttagttggt gtttgttttt ttgttttttt tttagttgtt 4860
tttttttttt ttttttgtgt tttagttatt tgggaaggtt tgtttagtgt agttgggttt 4920
tgattggttg ttttgaaagt ttatgggtta tttgattggt gaatttgggg ttttttagtg 4980
tggtgagttt gaaattgttt gtatttggtt ttaaagttgg tttttggaaa ttgagtggag 5040
agtgatgtgg ttgttgtagt tgttgttgtg gttgttgtgg aataataagt tgggtatagt 5100
ggttggggtt agggttgtgt ttaggggatg gttgagggtt ttggagggtg agtattgagg 5160
aatggggttt tttaagaagg ttggattgga ggttagggat ttgtgtgggg tttggttggg 5220
ttttgggggg tggtgggtag ggtttttgtt tgggtttggt tgtgtgagtt atattgggtt 5280
ggttttagag ggaggttatg ggagtttagt ttggtggggt taggtggtgt gggggtgggg 5340
gtttggggtt gtagttatga gttgaggttg gttgttttta ggaattgttt gaagaggttt 5400

ttgggttttg attgaagtaa aatttgtttt tttatgttga gtgtttgtgg agtagtaggt 5460
ttttttttgg gtttggtttt tgtgtgtttt gttattttgt tttgggggtg ggaaagtggg 5520
attgtatttg ggttaggtat aggtgtgggt ttttagagtt aggttgtttt gagaaattga 5580
ttttggaatt tgtgggtttt tttgggtttg ttttgtttat ggttttggtt tatttggagt 5640
tgttaagaat agttagttta ggtatttaat ttttatgagt agtgtagtgt agtttttttt 5700
atttaaaaag atatattttt gttttttttt ggattgattt ttttttgaag atgaatgtga 5760
gaaatagaat ttaaaggttt attttgagtg tgttttataa atgattttat tattagtgat 5820
gggtgttaga aaaatttagg taattatgtt tggagttttg atgttttatt ggtaaatttt 5880
agaggaagtt ttgaattttt ggttgttagt tttttttttt aatttattta ttgtgattta 5940
agtatattgt ttttttgttt agtttttgat attgtttagg ttataattag aattttattt 6000
gtggaattgt attttttatt tttttgtttt atggtgttta agtgatattt tttatttttg 6060
gaaaaagtat tttgaaagtt ttgagtatgt ttgtttgtga ttttagtaag tagaggaaaa 6120
agattaaaat tgttatgtgg gtaattgtaa atatttaaaa tgtttttgtt gttttaaata 6180
aaattttttt aatgaatagt ttgaatatat tttagttttg tttttataat tttaaaaaat 6240
aatgttttga ataaaattag gagtattata aagtggtata aatttgttga aatatttggg 6300
tataattttg gtatatgtag gtattatttt aaagtgttat aaaaagggaa tataattttg 6360
gtttttgttt ggaaagaata gataagttga tatttgaatt tgagggttat tttgattgga 6420
agaagttaaa tattatatta gtatagaata tatatgtttt taatgtttta ggagttaaga 6480
aggatggaaa gttattgtag gagtttttat ttttgtaaat attgtttgaa tgtaattaat 6540
atttatggaa tatttatgtt gtagtaggta ttgtggaaaa tgttttatat atagtgtttt 6600
attaatgttt aaaattattt gagttagtgg tatgtaatta agttagtttt ttttttattt 6660
tagg 6664

<210> 378
<211> 6664
<212> DNA
<213> Artificial Sequence

<220>
<223> chemically treated genomic DNA (Homo sapiens)

<400> 378

tttaaagtga aggaaaaatt gatttaatta tatgttatta atttgagtag ttttaagtat 60
taatgaaata ttatatgtaa agtatttttt atagtgttta ttgtagtata agtattttat 120
aaatattaat tatatttaaa taatatttat aagggtaagg atttttataa taatttttta 180
ttttttttga tttttgagat attggaaatg tgtatatttt atattagtgt agtatttagt 240
tttttttaat taaaataatt tttaaattta ggtattaatt tgtttatttt ttttaaataa 300
aaattagaat tatgtttttt ttttgtgata ttttaaagta gtatttgtgt gtgttaaagt 360
tatatttaaa tattttaata aatttatatt gttttatgat atttttaatt ttatttaaaa 420
tattattttt taaagttatg aggatagaat taaaatgtgt ttagattgtt tattaggaaa 480
attttgttta aaatgataaa aatattttaa gtgtttatag ttgtttatgt gataatttta 540
attttttttt tttgtttgtt gaggttatag ataaatatgt ttaagatttt taaggtattt 600
tttttaagag taggaaatat tatttaagta ttgtaaaata aaagaatgaa aagtgtaatt 660
ttatagataa aattttaatt ataatttaaa taatgttaag gattaagtag aaaaatggta 720
tatttgaatt ataatgaatg ggttaaaaaa aaaagttgat aattagaagt ttaagatttt 780
ttttgaagtt tgttaatgaa atgttagaat tttaggtata attatttaga tttttttaat 840
atttattatt gatgataaaa ttatttataa agtatattta gaatagattt ttaagtttta 900
ttttttatat ttatttttaa agaaaaatta atttagaaag aagtagagat gtattttttt 960
aaatgaaaaa aattgtatta tattgtttat gagagttaag tgtttgggtt gattgttttt 1020
aatagtttta ggtgggttgg ggttgtgggt agagtaagtt taagaaggtt tatgagtttt 1080
aggattggtt ttttagggtg gtttggtttt agggatttgt gtttgtattt ggtttaggtg 1140
tggttttatt tttttatttt tgaggtggga tggtagggtg tgtaggggtt gggtttagga 1200
aagagtttgt tattttgtag gtgtttagtg tgggggagtg gattttgttt taattaagat 1260
ttgaaagttt ttttaggtag tttttgagaa tagttgattt tgatttatga ttgtagtttt 1320
gaatttttat ttttatgttg tttaattttg ttaggttggg tttttatagt ttttttttaa 1380
agttagttta gtgtggttta tatggttaga tttaggtgaa ggttttgttt attgtttttt 1440
gaggtttagt tgggttttgt gtagattttt gatttttagt ttggtttttt tagaggattt 1500
tgttttttaa tatttgtttt ttgaggtttt tggttgtttt ttagatatga ttttgatttt 1560
agttattgta tttggtttat tattttgtgg tggttgtagt ggtagttata ataattgtgt 1620
tgttttttgt ttaattttta agagttagtt ttgaagttaa gtgtgagtag ttttaaattt 1680
attgtgttaa agggttttgg atttattaat tgggtagttt gtagattttt aaagtagtta 1740
attagagttt agttatgttg ggtaggtttt tttgggtggt tagagtgtga aagaaagagg 1800

aaagggtggt tagagaaaaa gtaggagggt gggtgttaat tgagtgtgag tgtaagtgtt 1860
tttttttagt tgggagagtg ttgttttatt gtttttagtt agtggagtag gaagttattg 1920
tttgttttgt ttttttttta aatttttttt tttagtattg gtatagttta aatttattat 1980
atttaaaata gtttattaaa aaagtgatat tgtgtttata ttgagatttt attattttta 2040
tttttaatat ttagggttag gagtgtatag ttatgttttt ttaaatgtgt gatttgtggg 2100
ttggttttaa ggagtatatt ttagtgattt taagaaggaa atggaaaatt taaaagattg 2160
ttttaaaaat gtaaaggaaa atttattatt tatattgttg tgttttgttt ttattttatt 2220
tttgaattta atattaaatt attttattat ttatattttg tttattatat aattaaaaat 2280
atttgaaatg tattaaattt taaaatattt ttatattaga attttaaata tatagagaga 2340
ggtatgtatt tagagattgg gttttattat gttgtgtatg ttggttttta aattttgggt 2400
ttaagtaatt ggttttagtt tttagtgtag ttgtgattat aggtatttgt tattatgttt 2460
ggtttaaata taattaaaat ataaaaattt ataattttat gaggggagag aaagaggttt 2520
aaattatgtt ttaaataatt tagtgttaaa aattttgttt ttattgttat ttattatatt 2580
tttgagagag atttttgtat ttattgtagt taattttttt tttttttttt ttgttttttt 2640
tttttttttt tttttttttt ttattttttt tttttttgtt tgtttgtgga gataatgttt 2700
ttttatgttg ttttagttgg tttttaattt ttaggtttta gtaatttttt tgttttggtt 2760
ttttaaagtg ttgagattat aggtgtgagt tattagttta atttaaatat tattttaaaa 2820
tttttttaaa ataaataaat agaatagtaa aatagagtta gatataataa aaatttattt 2880
taaggatttt tggttgtatt gattttaatg ggatatagaa taaagattga ttattatatt 2940
tattataagt atgtattaga agatttaaat taggatatgg aatggaattt ttttattttt 3000
gtgatataga gtatattata tgtatttaag tgtttaatat tttttaatat aatgagttgt 3060
atttattatt tggtgttata tattgtattt ggtttaattt tgataatttt tgtttttagg 3120
taatagaggt agagtatttg ggagttatgg tgtaggtagg aatttaagaa tttttgttgg 3180
agtttaatta tttttaaaat ttatgtttaa aatttaaatt ttatttttta aatgaaaatt 3240
tattgtttta gagaagtgtt ttagggaatt aggttgtttt gtgtagttta tggtattggt 3300
tttaatgaat tttaatatgt tttttttttt gagaggttta agaggaattt aatttttata 3360
ttatttaaaa atggtaatta atttattata tttattttat ttttaatata aagaagatta 3420
taagttttta tagttgtgat gttaggtgta ttaatttaat ttaattattt ttttttataa 3480
agttttagag gttattgtaa atttatgtta ttattttggt aaatatagtt tattattttt 3540
tgggttaatt tttatgtagt atttttaaag tgtaatattg tgttttatag atataaaaat 3600
gaataagata tagttattgt tggaggtgtt gagggtaggg gagtaggatg ataattggga 3660
attttttttt ggtatttatt ttgtaattta tgttataggg taatgtgaga atttagagga 3720
ggaaattgtt aatgttagta gaagtgatat ttgaataaag gttggggaaa ggattttgga 3780
gtgggttttt tatatagagg aaattgtata agtagagtat agaggttaga aagttaattt 3840
aggtaattta gggtgttttt tatggtagaa tgtagggagt attgtatttt gtgatgttaa 3900
agttagagaa gggagtttat agttagaatt ttgatgagtg ttgaatattg ttttaaagga 3960
tttgagtagg agaggtatat atagttttgg aattatattt taggaaaatt atttagaggg 4020
tggtattaaa aaaaaaaaaa ttgtagagga gagtagggga aggtatgtgt tttgaaaaat 4080
tttatatttg tattttggta aattttattt tggaaggaaa agaaaagatt agttggtatt 4140
gtatataagt ttttttggtt ttgtttttta tatttatgta tttttatggt ttgaatgttt 4200
gttgtttttt tattttgtta atttatatgt tgaaggtttt attttattgg gatggtgttt 4260
ggagatgagg ttttgggagg taattaggtt tagatgagga tatgagggtg gggtttttgt 4320
gatggaatta gtgtttttat aagaagagat gttgaagagt ttggttgttt ttatgtgagg 4380
ttagaatgag aaggtatttg ttagtgagga agttagtttt tgttagaatt tgatgttgtt 4440
tgttttttga atttgtatat taagttttta gaattgagtg aaaataaaat tttgttgttt 4500
aaggtatgta gtttatggta ttttgttatg gtagtttagg ttaagataag tgtttttttt 4560
agataagaaa aatgtgagaa tatttgtttg aaaatatatt tttgggttgg gtgtggtgtt 4620
ttatgtttat aattttagta ttttgggagg ttaaggtggg tggattatgt tgttaggaga 4680
ttgagattat tttggttaat gtggtgaaaa tttattttta ttaaaaatat aaaaattagt 4740
tgggtgtggt ggtatatgtt tgtaatttta gttatttggg aggttgagtt aggagaattg 4800
tttgaatttt ggaggtgaat gttgtagtga gttgagatgg tgttattgta ttttagttta 4860
gtgatagagt gagatttttt ttaaaaaaaa aagaaaagaa aagaaaatat attttgggaa 4920
ggaggtggaa gaaattaata aaaatgtgtt ttttgtggaa tataagagtg atttggtata 4980
gaggtggaaa ggagagtttt gtattttttg aatattgagt tatgtgaatg tattatttta 5040
gtttaaaaat gaatgaataa ataaatgaat gaatgaatta gttttgttaa ggtttttatt 5100
gttattggat aaaaatggta aaaattggtt aggtatagtg gtttatattt gtaattttag 5160
tattttggga ggttaaggtg gaggattttt tgaggttagg agtttgaggt tagtttggta 5220
atatagtaag attttgtttt tatatagata aatatataaa aagaaaaaaa aaatttaaaa 5280
gttgttggaa aatgtatgta aaagaagaag atttattttt ttttatgtta atttagaaag 5340
gaaagtgata gaaaatataa gatatataga tttaggtttt tttattataa gtgaaaatat 5400
tgaattattg ttgttatgat agagttgggt gtttttttga ttataaaagt aatgtgattt 5460
ttttgtaaat tttaagaaat aatttaaagt gtaaaatata gataataaaa gtttgttata 5520
tataattttt atgtttttta tttttatttt tgttttataa aaagagaaag agattatttt 5580

aaatttgttg gtagttgtat tttaaatgta tatatgttaa tttattttaa ttttttattg 5640
gaattttaga aattgggtat ggtgtaggga gaagagattt atatgttaat tttgggatta 5700
atgaggatta aggataatag gtgagtagtt gtggattttt tatttaggtg gggtgtaaat 5760
gtttataatg taatatggtg aagattgttg tgtgtagaat atagtggaga gggtattagg 5820
taaggtttta tagtaaagtt gattgaattg agttttaagg aataaaaaga gtttgttaag 5880
aagataaatg aaggtggaga gtttgattta ttgataagaa agaataatgt aagtttgtag 5940
attgaaatta atttagtatg ttgaatataa atagaatttg ggtgtttagt aagggttata 6000
aatattaggt taggtagtta aataatattt atattttgaa ggtaatgttt aaggttatgt 6060
taaaagtttt ggattagatg ttggatttga ataaaaaaat gtgaataagg tagtgatatg 6120
gttataagtg ggagttaggg gggaaaagtt atgaagaggg ttggtgatgt agtattagag 6180
ttagtagata atggagggtt ttgtaatagt tttagtaggt gttgaatttg gggagatgtt 6240
agttttaaga ggaagaaatg ataatgagat atttttagga gttgggattg ggttaaaaaa 6300
gggagaagga gttattaggg atgatttggg tgtgtggttt tgtaataggt tgagggtttt 6360
gttgttttag agagatgtta aggggtagtt atggggtatg gagtattatt ttgggtatat 6420
tgatagtgaa gtattgtggg atatttatgt ggttggatat ggttaagggt aagtgtaggt 6480
tttatagggt ttgaagtgta tattatttgt ggattgttat aatttgaagg tatgtgtttt 6540
ttttaaatgt atatgttgga atttaaattt taaggtgatg gtattaagag gtagggtttt 6600
tgggaggtga ttattaaatt ttgagggttt tttttttatt aatgggatta atgtttttat 6660
aata 6664

<210> 379
<211> 6815
<212> DNA
<213> Artificial Sequence

<220>
<223> chemically treated genomic DNA (Homo sapiens)

<400> 379
   ttgtttgtga attaaaaatt tagagttttt ttgtgaatta tgttgttaag aattgtagat 60
   ttaagtttta agattgtatg aatttaaatt ttgattttat ggttttagtt ggtatgaaga 120
   tataaagata attttttagt aaaaatagtt ttgaaagatt gggtgtgatg gtttatgttt 180
   ataattttag tattttggga ggttgaggtg ggtggattat ttgtggttag gagtttagga 240
   ttagtttggt taatatggtg aaattttgtt ttaattaaat atataaaaaa attagttggg 300
   tgtggtggtg gatatttgta attttagtta tttagggagg ttgaggtagg agaattgttt 360
   gaatttagga ggtagaggtt gtagtgagtt gagattatat tagtgtattt tagtttgggt 420
   gataaaagtg aaattttgtt ttaaaaaaaa aaaaaaatgg tagaatgtta tatggaaagt 480
   taatttttag agatagtaat ttagatattt ataatattat attgtttttt taagtattta 540
   tattaataaa ttaattaata tttgttgatt taatatggtt agggatagtg ttgggttttt 600
   ataattttta agtaatttta gttagatttt gtttttaagg agtttatgag tagtatgagg 660
   agataaaaaa tagataaata attttaattt aggaagaatt tgataaatgt atgataatta 720
   ttattagaat ttagaggaga ggaaattttt tttgaataga gattaaagga aggaataatg 780
   aatgtggtag tatttgggtt ggatttatta taagaggtat gttttttttt gataagttaa 840
   gaaagtgggg atttaaggta gaagaaaaga aaagttttga gtttttggag tattaaaatt 900
   ttgtttggtt gaagtatagg ttatagttgg aggagatgga ttaggaagga ttgttatggg 960
   tagagaggaa gttttgaatt tatgggggaa gttatgattt gggttaaaga tagagttggg 1020
   ttatgttaag atggattttg aagtagtaaa aaaaaattta agaaagtaaa taggttgtag 1080
   ggtttaggat gaagtttggg aggaaagggt agagtggttt ttaggaggtt gttaggatag 1140
   gtaaggtaat gggttttaaa gggagtggtt gaaatgtaat ggaaaaagag agattgtaaa 1200
   gttagaaggt ttaggaattg ttttttgatt aggtgtggaa ggtaagggaa aattagtttt 1260
   tgaagaagat agtgagattt taatttgggt ggttggagag atagtgatgt tgggtataga 1320
   tatggggaag ttgagaggaa tattatgttt gagaatggtg atttatattt gaataagttt 1380
   gtaatgttta gtagattgtt ggaaaagtgg ggttggagat atatttaatg gaggagttag 1440
   attaattttt attttttttt atttgagaga gttagtaagt tatggttgga atgtgtgtgt 1500
   ttagtaggag agggtaggga gggaagttaa gagagttggg agtttgagtg aagtttttgt 1560
   taaaggtaga agaggaaagt tggtgtagta tagtatattt ttttatttat gtttattaag 1620
   tttagggata aggtttatta agatgagttt ggaagagaat gttggagaga aagtggttaa 1680
   gaaaattgtt tttattgaat tttttgggtt aattttgatt gtaagttttt gaataattaa 1740
   agtttgtgag gagatagtta atttttttat tttttttatg ttaatagtga ataattgtag 1800
   attttttttt tttttttttt tttttttttt gttttttttt tttttttttt tgaatatttt 1860
   tgtttttttt tgggattggt ttagagtatg ggtggttatt gttgatttat aggaggtatt 1920
   attgttatta ataaagggta atagtttttt tttttaatat ttatttatat ttagtattta 1980
   tttttaatat tgattatgga gagagttttt ttgtgtttaa atattgtaat attgggggtt 2040
   ttttaaagta taaaaatata tatttgtatg atggtattat taatattttt atggtttttt 2100
   attttttttt tgtattggtt ttaagagtta tttataaatt ttttagtaat tgtatagtgt 2160
   tttagggtta gagattggtt atttttggta ttgtgattag agttatttaa tatttaaggt 2220
   ggtgattaat gtttggtaat aaagttttta ttgggtgtta tgtgttttgg gattttgagt 2280
   gtgggtattt taggagtatt ttagtattgt gtgttagtat tatggttgag agaatagttg 2340
   agaaagtggt taagaggtgg atttatgtga atgttattgg gaaatgagag attttgtttt 2400
   taattatggt tagtgtaatt tgaaagttta aaattagttt aaaataaagg tatttatttt 2460
   tattttatgt ttatatttta ggtttttaat aatatgtatt ttttatatgt ttatagaaag 2520
   tagttaattg agttatttat ggaaaggttt gtgggtttgg ttaatgaagt ggaggagtat 2580
   tatattttag ttggaaatat atttttagaa tgttaaaata tttattttaa agtttggttt 2640
   tttggtgtaa ttggaggtat ggtaatgttt ttgtttagag attgggggtt agggttagta 2700
   aggtatttga tttatatgta ttttagaagg tttttattgt taaattatat ttttttggaa 2760
   aaattattta tgttttattt tgtaaatttg atatttatat atttttgatt ggtattttat 2820
   tttagttgta agattatgat ttatagtaag tttgtttttt tttttgtttg gggtggtagt 2880
   agaaagtata gggtattttt tagtttttaa gggtaggggt aaaggggttg gggttttttt 2940
   tttttagtat agtttttttt ggttgtgtta tattgttttt tgtgagtaga tagtaagttt 3000
   ttttttattt tttattgtta tttatttagt gttgtgtagt agtttagttg tgtgtttgtt 3060
   gggaggggtt gttaagtgtt ttgtttattg gttgtttttt gaatttttgt tattttatgt 3120
   ataaatatat ttatatattt tttttgttta gtttatatat tgagttattt gtatatgtga 3180
   gtatattttt tttttttttt ttattttttt ggtttttgat ttttataagt ttatggaata 3240
   tttttggaaa gatgtttttg atttagtagg gtaggtttgt tttgattttt ttttttgtag 3300
   ttttagtatt ttgagaaagt aatttatttt tttggttagt gtttgtattt tagtagggag 3360
   atgaggattg ttgtttttta tgggggtatg tgtgtgtttt tttttttttt taggatttgt 3420
   aggatttttt gtgttatttg tatataattt ggtaggttta tattttttaa gagttttatg 3480
   aagtgttttt tgtatgtgtt ttaaaaaggt atttgaaaat tgaaagtgtg atttatggaa 3540
   attaaattat ttgtaaaaaa ttgttttgga aagtaatgat tgttggttat aaagggaaat 3600
   atttgtgatg tatttaatgt gtttttaatt ttttatttgt tgataattta tagttattaa 3660
   tgttaaattt gattttggtt ttagttatat ttgtatattg tttaataatg gtttattttt 3720
   gtaagaatta gataaaatgt atatttgata taaaatagtt aaaaatgtaa tttttagtaa 3780
   tagtaagttt ggtatttaga tagattatga atattttgtt agatattttg ttgggtgttt 3840
   gggatagtaa ttaaaataaa gtattgatag ttgtattaga gtttattagg ttgtagtaaa 3900
   ggaagtttat ttaaaagtat aaattattta agattataga tgtatgatat attttattta 3960
   ttttttgttt ttttaatatg tatatatata tatatatata tatatatata tatatatgtg 4020
   tgtgtgtatg tgtgtgtgta tgtttaattt ttaatttagt taaaaatttt tttttatttg 4080
   ttttttattt ggatatttga ttttgtatat tttagtttaa gtgaattgag aagattgagt 4140
   tgtaggatta aaggatagat atgtagaaat gtattttaaa aatttgttag ttggattaga 4200
   ttgataatgt aatataattg ttaaagtttt ggtttgtgat ttgaggttat gtttggtatg 4260
   aaaaggttat attttatatt tagttttttg aagttttggt tgtataatta atttgtggaa 4320
   ggtatgaata tttatgtgtg ttttaattaa aggttttttt gaattatttt ttatatgaga 4380
   atttttaatg ggattaagta tagtattgtg gtttaatata aatatataag ttaggttgag 4440
   agaattttag aaggttgtgg aagggtttat ttattttggg agtattttgt agaggaagaa 4500
   attgaggttt tggtaggttg tatttttttg atggtaaaat gtagtttttt ttatatgtat 4560
   attttgaatt tttgtttttt tttttttaga tgttttttgt tagttttttt agttgttaaa 4620
   tatagttgtt tgtggttggt tgtgtatgta attgtatatt ttattttatt tgttttattt 4680
   tggttatagt gtagtttttt ttagggttat tttatgtata tattatgtat ttttagttaa 4740
   tgaggagggg gaattaaata gaaagagaga taaatagaga tatattggag tttggtatgg 4800
   ggtatataag gtagtatatt agagaaagtt ggtttttgga tttgtttttt gtgtttattt 4860
   taagtttagt tttttttggg ttatttttag tagatttttg tgtgtttttg ttttttggtt 4920
   gtgaaattta gtttttattt agtagtgatg ataagtaaag taaagtttag ggaagttgtt 4980
   ttttgggatt gttttaaatt gagttgtgtt tggagtgatg tttaagttaa tgttagggta 5040
   aggtaatagt ttttggttgt tttttagtat ttttgtaatg tatatgagtt tgggagatta 5100
   gtatttaaag ttggaggttt gggagtttag gagttggtgg agggtgtttg ttttgggatt 5160
   gtatttgttt ttgttgggtt gtttggtttt attggatttg taggtttttg gggtagggtt 5220
   ggggttagag tttgtgtgtt ggtgggatat gtgttgtgtt gtttttaatt ttgggttgtg 5280
   tttttttttt aggtggtttg ttggtttttg agttttttgt tttgtgggga tatggtttgt 5340
   attttgtttg tggttatgga ttatgattat gattttttat attaaagtat ttgggatggt 5400
   tttattgtat tagatttaag ggaatgagtt ggagtttttg aattgtttgt agtttaagat 5460
   ttttttggag tggtttttgg gtgaggtgta tttggatagt agtaagtttg ttgtgtataa 5520
   ttattttgag ggtgttgttt atgagtttaa tgttgtggtt gttgttaatg tgtaggttta 5580
   tggttagatt ggtttttttt atggttttgg gtttgaggtt gtggtgtttg gttttaatgg 5640
   tttggggggt ttttttttat ttaatagtgt gtttttgagt ttgttgatgt tattgtattt 5700
   gttgttgtag ttgttgtttt ttttgtagtt ttatggttag taggtgtttt attatttgga 5760
   gaatgagttt agtggttata tggtgtgtga ggttggtttg ttggtatttt ataggtattt 5820
   gtgtttgtgt tgtttgttgg ggtggttgtt gtgtttggta ggagggaggg agggagggag 5880
   ggagaaggga gagtttaggg agttgtggga gttgtgggat gtgtgatttg agggtgtgtg 5940
   tagggagttt ggggtgtgtg gtttagtttg ggggttttgt gtgtagtttg tgttgtgttt 6000
   agagttaagt ttttttgttg ggtagttgaa aaaaatgtat tttttattta tttattgttt 6060
   gtgtgagagg tagatttgaa agtttgggtt ttttaataaa atatatgttg gaaaattaga 6120
   taaagtagta gttatttgtg ggggaaaata tttttaggta aataaatatg gggtgttttg 6180
   agttatttgg gaaggttttg tttttggtat ttaaagttgg gggtgtttgg agttagtaga 6240
   gtttagtaga gttttattta tttttttaat gtttttgttt aatgtgtttt ttaaattttt 6300
   ttttatttag attatttgat tggaaatatg ttagttatga tgatgatttt ttgggaagtg 6360
   atttttgtta tttgtttttt tttttttttt attttatgtt ttggggtttt agagagtgat 6420
   tgggagttga atgggtttga ttttggagtt agttggttga gtttgtgttg gagtggattg 6480
   ttggtatgtg atttttgata gttggaaatt tgtaggtgtt ttgtgagttt aaaataagtt 6540
   atatggaagt ataagtgttt aaaaataatt ttttgttagt ttagtgataa gtttgtttta 6600
   tttggggaga atgttttgga gtggtgtgtg ggttagttag ggtttgtgtt ttgtagttat 6660
   tgtggaagga gtgtggttgg tttaggatat aggagattat tttgtgattt taatggtgaa 6720
   ggttgtgtgt ttttatttta attttttttt ttataagaat tgtttttttt tttttttttt 6780
   ttttttttat ttttttttgt ttagtttttt ttttt 6815

<210> 380
<211> 6815
<212> DNA
<213> Artificial Sequence

<220>
<223> chemically treated genomic DNA (Homo sapiens)

<400> 380

aaaaggagaa attgggtaag agaaaatggg agggagagga gagggagaaa gaataatttt 60
tgtagggaaa aaaattaaaa tgaggatata taatttttgt tattgaagtt ataaagtggt 120
tttttgtgtt ttggattggt tgtgtttttt ttatagtggt tgtgaggtgt agattttggt 180
tgatttgtat gttattttgg ggtatttttt ttgggtggga taggtttgtt attgggttgg 240
taggagatta tttttaagta tttgtgtttt tatatggttt gttttaaatt tgtgggatat 300
ttataaattt ttggttgtta gaagttatat gttagtaatt tgttttagtg tggatttagt 360
tagttaattt tgaaattaga tttatttaat ttttaattgt tttttaaagt tttaggatgt 420
ggggtgggga ggaggggaaa gtgggtgata ggaattgttt tttagaaagt tattattata 480
gttgatatat ttttaattaa atagtttaga tgaaaggaaa tttggggagt atattaaata 540
aaaatattaa aaggataaat aaaattttgt tgagttttgt taattttaaa tatttttaat 600
tttaaatgtt aagagtgaga tttttttaag tgatttaaag tgttttgtgt ttatttgttt 660
ggaggtgttt ttttttataa ataattgttg ttttgtttgg ttttttaatg tgtgttttgt 720
taggaagttt gggtttttgg gtttgttttt tgtatggatg gtaagtgggt ggagagtatg 780
ttttttttag ttgtttggtg agagaatttg attttgaatg tagtgtgggt tgtatgtaga 840
atttttgggt tgggttgtgt gttttgggtt ttttgtgtgt atttttgggt tgtgtgtttt 900
gtggtttttg tagtttttta ggtttttttt tttttttttt tttttttttt ttttttgttg 960
ggtgtggtgg ttattttgat gggtggtgtg ggtgtgggta tttgtagaat gttggtgggt 1020
tggttttgtg tattgtgtag ttgttgggtt tgttttttag gtagtagggt atttgttggt 1080
tgtggggttg taggaaaggt gatagttgtg gtggtgggtg tagtagtatt agtgggtttg 1140
gagatatgtt gttgagtggg gggaaatttt ttaggttgtt ggagttgaat gttgtagttt 1200
tagatttggg gttgtagggg aggttggttt gattgtagat ttgtgtgttg gtggtggttg 1260
tggtgttgaa tttgtaggtg gtgtttttgg ggtagttgta tatggtgggt ttgttgttgt 1320
ttaggtatat tttgtttagg ggttgtttta gggggatttt gagttgtgga tggtttaggg 1380
gttttagttt gtttttttgg atttgatgta gtagggttat tttagatgtt ttggtgtgga 1440
gggttatggt tatggtttgt ggttgtgggt agggtgtaga ttgtgttttt gtagggtaga 1500
aggtttagaa attggtgggt tatttggaaa aagagtatag tttgaggtta gaggtgatgt 1560
agtgtatgtt ttgttgatat gtgagttttg gttttggttt tgttttggga gtttgtgggt 1620
ttggtgaagt tgggtgattt gatgggagta agtgtagttt taggatgaat gttttttgtt 1680
agtttttggg tttttgggtt tttaatttta agtattggtt ttttgagttt atatgtatta 1740
taaaggtgtt ggaggatggt tagggattgt tgttttgttt tgatattggt ttaaatatta 1800
ttttaggtat aatttgattt ggagtgattt taaagagtag tttttttgaa ttttatttta 1860
tttgttgttg ttgttggata gaggttgagt tttatggtta gggggtgggg gtgtatgagg 1920
atttgttaaa ggtggtttag ggaagattgg gtttaaaata aatgtgaaag atggatttag 1980
gggttggttt tttttaatgt gttgttttat gtgttttgtg ttagattttg atatattttt 2040

gtttgttttt ttttttgttt gatttttttt ttttgttggt tagaaatatg tagtgtgtat 2100
ataggatgat tttggggagg attatattgt aattgagata gggtagatag aatggggtgt 2160
gtggttgtat atgtagttag ttatagatag ttatatttag tagttggggg aattgatagg 2220
gggtatttga ggggaagggg gtggagattt agggtatata tataggaaga gttgtatttt 2280
gttattagga gaatgtaatt tgttaggatt ttagtttttt tttttgtaaa atgtttttaa 2340
agtagataga tttttttata attttttgag atttttttag tttgatttgt gtgtttatgt 2400
tggattatag tattgtattt ggttttatta ggaattttta tgtgaaggat gatttagaaa 2460
aatttttggt tagggtgtat atgggtgttt atgtttttta taggttggtt atgtaattaa 2520
aattttagaa aattgaatat aaaatgtgat tttttatat taaatataat tttaggttat 2580
gaattaaagt tttggtaatt atgttatatt gttggtttgg tttagttaat agatttttaa 2640
aatgtatttt tgtatgttta ttttttagtt ttataatttg atttttttgg tttatttggg 2700
ttaggatatg tagaattaaa tatttagatg aaaaataaat agaaaaaagt ttttaattga 2760
attaaaagtt aaatatgtat atgtatatat atatatatat atatgtgtat atatatatat 2820
atatatatat atatatatat taaggagata aaaaataggt gaagtatatt atgtgtttat 2880
aattttggat agtttatatt tttgaataaa ttttttttgt tgtagtttaa tagattttga 2940
tataattatt aatattttgt tttaattgtt attttaaata tttaatagag tatttgatga 3000
agtgtttatg gtttatttaa atgttaagtt tattgttatt aagagttata tttttgatta 3060
ttttatatta agtatatatt ttatttaatt tttataaaaa tagattattg ttggataata 3120
tgtaaatgta gttgaagtta aaattgagtt tagtattaat gattatagat tgttagtaaa 3180
taaagggtta aaaatatatt aggtgtattg tagatatttt tttttatggt tagtaattat 3240
tattttttaa agtaattttt tatagatgat ttaattttta taaattatat ttttaatttt 3300
taaatgtttt tttaaaatat atgtaaaaag tattttatag ggtttttaaa aaatgtgaat 3360
ttgttaaatt atatgtaaat ggtataaaga attttataag ttttgaaaga aaaaggagat 3420
atatatatat ttttatggag aatagtaatt tttatttttt tgttaggata tagatattag 3480
ttagaaaggt aagttgtttt tttaaaatgt taaagttata gagagagaaa ttaaaataag 3540
tttattttgt tggattaaga atgttttttt agaaatgttt tatgggtttg tagaagttaa 3600
gggttgagag agtgagaagg aaggaaggaa tgtgtttgta tgtgtgagtg gtttagtgtg 3660
tgaattaggt agagagagtg tgtggatgtg tttgtgtgtg gaatggtagg gatttgggaa 3720
gtagttagta ggtagggtat ttggtagttt tttttggtag atatgtagtt gggttattgt 3780
atagtgttgg atgaatggta gtggggagtg aggggagatt tgttgtttgt ttatagggag 3840
tagtgtggta tagttagaga aagttgtatt ggggaggaga aattttagtt tttttgtttt 3900
tatttttgga ggttggaaag tattttatgt tttttgttgt tattttaagt aagaggaaaa 3960
ataggtttgt tgtgaattat agttttatgg ttaaaataga atgttagtta aaagtgtatg 4020
gatattaagt ttataaaata ggatatgggt ggtttttttg aaagaatata atttaataat 4080
aaaagttttt tgggatatat gtggattaaa tgttttattg gttttagttt ttagtttttg 4140
aatagaggta ttgttatgtt tttgattgta ttaggaaatt agattttgga ataaatgttt 4200
tggtatttta gggatgtgtt tttagttgaa atgtaatatt tttttatttt gttaattaaa 4260
tttataaatt tttttatgaa tagtttagtt gattgttttt tgtaaatatg tgaaaaatat 4320
gtattattaa aagtttagga tatgaatata agataaaggt agatattttt gttttaaatt 4380
gattttaggt ttttgagttg tattgattgt gattgggaat gaggtttttt attttttagt 4440
ggtgtttata tggatttatt ttttgattat ttttttaatt atttttttgg ttatagtatt 4500
aatatgtaat attgaggtgt ttttagagtg tttatgttta gggttttagg atatatgata 4560
tttaatggag gttttgttgt tagatattag ttattatttt ggatattaaa tgattttaat 4620
tataatgtta ggagtggttg gtttttggtt ttgggatatt atgtagttat tgagagattt 4680
atgagtggtt tttgagatta gtataaaaaa gaaatagaaa gttataaaaa tgttaatgat 4740
gttattatgt aaatatatgt ttttgtgttt tgaaagattt ttagtattgt agtgtttgag 4800
tataggagag ttttttttat agttagtatt gaaaataaat attggatata aataaatatt 4860
gaaaagaaag attgttattt tttgttggtg atagtggtgt tttttgtagg ttaataatgg 4920
ttatttatgt tttagattag ttttagaaaa aagtaagagt atttagggag ggaggagaga 4980
ggaatagggg aaaggagaag gaaaggaaag gggatttgta attgtttatt attgatatag 5040
gaagaataag aaggttagtt gtttttttat aggttttgat tgtttagaga tttataatta 5100
aagttagttt aagaagttta gtaaaggtag tttttttaat tatttttttt ttagtatttt 5160
tttttaaatt tattttggtg agttttgttt ttgggtttgg tgagtatggg tgggaaagta 5220
tattgtgtta tgttgatttt ttttttttgt ttttggtaaa aattttattt gggtttttag 5280
tttttttggt tttttttttt attttttttt gttggatata tatgttttag ttgtgattta 5340
ttggtttttt taggtgaaga agggtaaaga ttgatttggt ttttttgttg aatgtgtttt 5400
tagttttatt tttttagtgg tttgttgggt attgtaggtt tgtttaaata tgagttatta 5460
tttttaaata tggtgttttt tttaattttt ttgtgtttgt gtttagtatt attgtttttt 5520
tagttattta gattaaaatt ttattgtttt ttttgagggt tgattttttt ttgtttttta 5580
tatttaatta agaggtaatt tttaagtttt ttagttttat aatttttttt tttttattgt 5640
attttggtta ttttttttga gatttattat tttatttgtt ttaatggttt tttaaggatt 5700
atttgtttt tttttttttgg atttatttt aaatttgta atttgttat tttttggat 5760
ttttttttat tgttttagga tttattttga tatgatttag ttttgttttt aatttaaatt 5820

atagtttttt ttatgagttt agggtttttt ttttgtttat aatgattttt tttagtttat 5880
tttttttgat tgtaatttat gtttagttta agtaaaattt taatatttta agaatttaag 5940
gttttttttt ttttttattt taaattttta tttttttggt ttgttagggg aaagtatgtt 6000
ttttgtaata ggtttagttt aaatgttatt atatttatta tttttttttt tagtttttat 6060
ttaaagaaaa tttttttttt tttgaatttt agtagtagtt attatgtatt tattaaattt 6120
tttttgaatt agagttattt atttgttttt tattttttta tgttgtttat aagttttttg 6180
aggataaggt ttggttagaa ttatttggga attatgagaa tttagtattg tttttggtta 6240
tgttaagtta ataaatgttg gttaatttat tgatatagat atttaaaagg ataatgtaat 6300
attataggtg tttggattat tatttttaaa agttaatttt ttatgtgata ttttattatt 6360
tttttttttt tttgagatag agttttattt ttgttattta ggttggagta tattggtgtg 6420
attttggttt attgtaattt ttgttttttg ggtttaagtg atttttttgt tttagttttt 6480
ttaagtagtt gggattatag gtgtttgtta ttatatttag ttaatttttt tgtatgttta 6540
gttgagatag gattttatta tgttggttag gttggttttg aatttttgat tataggtgat 6600
ttatttgttt taatttttta aagtgttggg attataggta tgagttatta tgtttggttt 6660
tttaaaattg tttttgttag aagattattt ttgtattttt atgttaattg aggttataaa 6720
gttaaaattt aaatttgtat aattttgagg tttaagtttg taatttttgg tagtatgatt 6780
tataagagag ttttgaattt ttagtttgta agtag 6815

<210> 381
<211> 5527
<212> DNA
<213> Artificial Sequence

<220>
<223> chemically treated genomic DNA (Homo sapiens)

<400> 381

ggttttttgt tgttgttaaa ttaataattt tagtttttgg gtttagttat ttgttgttgt 60
taaaaatgtt gttgagagtg atggagaggt gttttagttt ttattttata gattagaaaa 120
ttaaggttta gagaaattaa gtagttttgg agataggagt tatggttttt ttatttgagt 180
tattattttg aaatattaag gataagttaa ttttattaat agttttaggt ttgtgagaaa 240
tgggtttggt ttattttgaa gattgtaaat aaataaaaaa atagattgtt ttattattga 300
tgagaaatat gagttatttt gttaggaatt gaaagtatag ttgtttataa aaaagttatg 360
ttggggttta gtttttttag gtttttgtgt gtgaaaaatt atttagttaa agtttttttt 420
ttattttttt ttgtgtttag taggatgttg aaatttttgg aaaagataat ggaaattagg 480
ggtaggaaga gaatagtgtg gggtgttaaa tagattttag taattttttt gtttatggtt 540
atttttgtgt agatattttg attttttatg tgtaggtgga gtagtgtttt ttagtttttt 600
ttagaggttt ttttttgttt gttttttagt tatattgttt gagattgata atgttttata 660
gtaagatgga aatttttttt tatagtatta ttttgtgatt ttgtagttat aaagttgaga 720
tttttgaatg tggtattttt ttgttttttt attgttttat gtgtatttgt gttatttttt 780
ttgttttatt gtaaatatat ttttaaaatt tttatttttt ttatttagaa atagaattat 840
aagtggatgg ttaaataatt taaataaatg gtttatgggg aaaagtgaat tttatattgt 900
tttttaaatt tttagtgttt tttatggaga taattattgt ttttaatgtt ttgaatattt 960
ttttagagat attttatttt tatataagta tatgtgtatt tgtgtttttt ttttattttt 1020
tttttataaa tattagtaga ttgttttgta ttttggttag tttatttaat atattttgga 1080
gttaggtgtg gtggtttatg tttgtaattt tagtattttg ggaggttgag gtgggtggat 1140
tatttgaggt tgggagtttg agattagttt gattaatgtg gagaaatttt gtttttatta 1200
aaaatataaa attagttagg tgtggtggta tatgtttgta attttagtta ttagggaggt 1260
tgaggtagga gaattgtttg aatttgggag gtggaggttg tggtgagttg agattgtgtt 1320
attgtatttt agtttgggta atagaatata tagatttgtt tgattttttt tatgattgtt 1380
atagttttat tgaatgtatg gattattagt gttttagtta gtattttagt gatgggtatt 1440
tagggattat aaattttata agggtagaaa taatatttta tttatttttt gtttttgata 1500
tggtttggat gtgtgttttt gtttaaatta tatgttaagg taatttttta tgttagaggt 1560
ggggtttggt ggaggtgatt ggattatggg gatggatttt ttatgaatgg gttagtatta 1620
tttttttggt gttgtttttg tgatagagtt tttatgagat ttggttgttt aaatgtgtgt 1680
ggtatttttt tatttttttg gttaagtttt tgttatgtaa gatgtttgtt tttgttttgt 1740
tttttgttat gaataaaagt tttttgaggt ttttttagaa gtagatgtta ttatgttttt 1800
tgtatagttt gtggaattgt gagttaatta agtttttttt ttttatgaat tatttagttt 1860
taggtatttt tttgtaatag tgtgaggatg agttaatata gttttttata ttgtaattta 1920
aggtaatgtt ttgtataaag ggatgaatta gattgtttag taattaaaat ataaatataa 1980
attataagta tatttattta tgaattgggg gatagttttt atttgatttt gagttttgat 2040
atttgttgtt ttgttttgtt aaaaatgtgt tagtttttat ttagtttttt attagttata 2100

ttattaatta atatgtataa tatgtataat ttgttaggtt aggaggaaat ttatggatat 2160
aagaaatgtt tttttttttt aggaaatgat aatttgtttt gggagagaag gtaggtattt 2220
tttggtaaaa gtgatataat aaaataatta aaaaaagatg gatttttttt ttttttttga 2280
gatggagtta tgttttgtta tttaggttgg agtgtagtgg tgtgattttg gtttattgta 2340
atatttgttt tttaggttta agtgattttt ttgttttagg tttttgagta gttgggatta 2400
taggtattta ttattatgtt tggttaatat tttgtatttt tagtagagat ggggttttat 2460
tgtgttagtt aggatggttt tgattttttg attttgtgat ttgtttgttt tggtttttta 2520
aagtgttggg attataaata tgagttattg tattaggtga aaagatggat tttttaaaaa 2580
aggtgttagg ataattaggt ttttatttgt aagaaagtaa agttagaagt ttattttaga 2640
ttatgtatat tagtataaaa atgttaaatg gtttaaagat aaaatgttaa aataagttta 2700
gaaaggtatt aaaagaaaat atagaaaata tagtattagt aaaaattttt ttatataaga 2760
tgtaaaagtt agaagttata aaggaaaagg ttgataaatt agaatagata aaaatgtaaa 2820
atttttgtat aatataagat ataaagaatg aatttaaaga tatataatga aggttaggaa 2880
gatgaagatg ttttggtgat gagtggtggt tatatattag tattaatgta tttaatgtta 2940
ttgaattgta tatttaaaat gatgaaaatg gtaattttta tgttatgtgt attttattat 3000
ataaaaaaag atatgaaatg aattggggaa aatatttgtt atataattaa gaatgaaggt 3060
ttttaataaa atttgtaaaa ttatatatat ttttaggaat gaattaataa ataattttta 3120
tgaattagaa aaaagtgata atttaattaa aaaatgaata agggatataa gtaatgtgtt 3180
ttatagaaaa aataaaaatt gataatgaag ttatgaaaaa atgtttagtt tttttagtaa 3240
ttgtataaaa taaattaaaa taatgagata ttatttttaa gattagtaaa tgttaaagaa 3300
aaataataat tggtgagggt gtggggaagt gggtatttat atttatgttt ggaaatataa 3360
attggtgtaa ttttataggg agagtaattt tataatattt tttaaagatt tatatgtata 3420
attttatggt agagaaattt attttttttt taggattttt ttttttttaa aaatagtgat 3480
gtggatgaaa aatatatgtt tattattgta tagggtataa tagttgaaaa ttggaaatga 3540
taatatttat atttttttta gtaggggaat ggttaaataa attttataag ttatttggta 3600
gatattaggt atgagttaaa agttagggtt tagttagaga tggaaagtat attagtaatt 3660
tgaaagggaa aatgtaatat gaagaattat taattagtaa aagaaggtta attgttaaag 3720
gtataagagt atttaagttg tttgtagtta gtaggttttg gttggtgagt aggaagttgt 3780
ttgttgggag gttgttaaag ttttttgaag gtgagtatta ttggttttat aagttgttgg 3840
tagttatggt gttaagagta ggaagagaag tattagaatt tggaagagaa atttagtttt 3900
ttgttaggtt ttgtattgtt tttttggtgt tttttattga taaagttagt aaaattgtgt 3960
tgttagtaaa ggagattttt ttatgggatg tagtttggtg ttattaaaga gaatagagtg 4020
gatttggagt ttagatgtaa tataatgatt gatattggta tagtatattt attttgtttt 4080
tgtaaataaa atggtatatg tgatgttttt ttttgttttt ttgtatataa aataatattt 4140
gtttttattt attatgtatt tatgttttta ttttgtatgt taggagttaa gtattttgta 4200
tgtattaatt tattttgttt ttataataat ttttatatgt aggaattatt atagttattt 4260
tatgaatgag ttgaggaagg tattgagatg ttaagtaatt tgtttaaggt tatgtagtta 4320
gtaagtggta gagtaagaat tattatggtt ttataagttt aggaaaaagt ttgaaagaat 4380
taaaatgtta atagtgggga ttttaaggaa gtattgaaga ggttatggga gaagttttta 4440
ttttgttaaa aaattagttt tttaaataaa taaatatagt gaggtttttt tagaagtaga 4500
tgttattatg ttttttgtat agtttgtgga attgtgagtt agttaaattt ttttttttta 4560
taaattattt agttttaggt atttttttat aatagtgtta ggatgagttg atatagtttt 4620
ttatattgta atttaaggta atgttttgta taaagggatg agttagattg tttagtaatt 4680
aaaatgtaaa tataaattat aagtatattt atttatgaat tggggggttg ttttgtgtgt 4740
atagataagg tatatttttt aaaaaaatta tttttttaag aagaaaataa attagttaat 4800
aaatgataat ttatagtgtt aggaagtgag aaataagtgt gtgataaatg gtggagaatg 4860
ggagtatttt ttgtagtggg tgggaggaga tgaggagggt gtttttgggg gagtggtagt 4920
ggttggagta aaggtttgga ggaggtaagt tatgtgtttt gagtttttgg tttttgtttt 4980
attttgtgtt tgagttggtt tgaaggttgg ttgtttagat tgagtttttt gtttgtttgt 5040
attttgttaa tagttttaga agaaggtgat tggtggttgt ttgaggaata ttagtgggta 5100
agagaattag tatttttgga gtatttgttg tttgtgagat taagtattat gtatattgtt 5160
ttatttattt tttatagtaa ttttattaag tagttttttt ttatgtgaaa agatggaggt 5220
tgggtggagt aaaaggaggt atttagagtt tttagtaagt gagaggtaga gttgggattt 5280
gaatttagat ttgtttgata ttgaagttta ggttggtttt atttttttgg attgtttttt 5340
agggagtaga agatagatat tttattttag ttggttgttt ttagaagttt gattttgttg 5400
gtttaaaatg attttagttt tttgtttaga ggttgtgggt tgtgggttaa gatattagta 5460
gaaggagggt ttagttagag aggttgatat gggtttttat ttattgttat tttttttttt 5520
ttttagg 5527

<210> 382
<211> 5527
<212> DNA
<213> Artificial Sequence

<220>
<223> chemically treated genomic DNA (Homo sapiens)

<400> 382

tttggaagga aggagagatg atggtgagta gaagtttatg ttagtttttt tggttgggtt 60
ttttttttat tgatgttttg atttgtagtt tgtagttttt gggtaaggaa ttaaagttgt 120
tttgagttag tagggttaga tttttagaag tagttagtta aggtaaaata tttgtttttt 180
attttttgga aagtagtttg gagaggtgga attagtttag attttagtat taggtagatt 240
tggatttaaa ttttagtttt gtttttattt tgttgaggat tttaaatatt tttttttgtt 300
ttatttagtt tttatttttt tatgtggaaa agaattgttt ggtaaggttg ttgtggggag 360
tgaataaagt aatatatata gtgtttaatt ttatagatag tagatgtttt agaaatgtta 420
atttttttgt ttattggtat tttttaggta gttattagtt attttttttt gaagttgttg 480
gtggggtata ggtaggtagg aagtttagtt tgaataatta gtttttagat tagtttagat 540
ataaggtgaa atagaaatta aaaatttaga gtatatgatt tatttttttt aaatttttgt 600
tttaattatt gttatttttt agggaatgtt ttttttgttt ttttttgttt attgtggaga 660
gtgtttttat tttttattgt ttattatata tttgtttttt attttttggt attgtgagtt 720
gttgtttatt aattggttta tttttttttt ggaaaaataa tttttttaaa aatatatttt 780
tatttatgta tataaagtag ttttttaatt tatgaatgga tatgtttgtg gtttgtattt 840
gtgttttaat tattaagtaa tttggtttat ttttttgtgt aaagtattgt tttaggttat 900
agtgtaggaa attgtattag tttattttag tattgttata aagaaatatt taagattgga 960
taatttataa agaaaagagg tttaattggt ttatagtttt ataggttgta taggaagtat 1020
agtgatattt gtttttgggg aagttttatt gtatttattt atttgaagga ttgatttttt 1080
aataaagtga aaattttttt tatggttttt ttaatgtttt tttgaggttt ttgttgttaa 1140
tattttgatt tttttagatt tttttttagg tttataaagt tatagtaatt tttgttttgt 1200
tatttattag ttgtgtgatt ttgggtaagt tatttaatgt tttagtgttt tttttggttt 1260
atttataaag tagttataat gatttttgta tgtgaaggtt attatgagaa taaaatgagt 1320
taatatatgt aaaatattta gtttttggta tgtagagtaa agatataaat atataataag 1380
tagaaataaa tattgtttta tatatagaga gataaagaga gatattatat atattatttt 1440
gtttataaaa gtagggtaag tatattgtgt tagtattaat tattgtgttg tatttgagtt 1500
ttaagtttat tttgtttttt ttggtgatat taagttatat tttataaaaa gatttttttt 1560
gttagtggta taattttatt ggttttgtta gtagagggtg ttagagggat ggtgtaaggt 1620
ttagtagagg attggatttt ttttttgggt tttggtgttt ttttttttgt ttttaatgtt 1680
atgattgtta gtagtttgta gaattagtgg tgtttatttt tagggaattt tggtagtttt 1740
ttagtgggta gttttttgtt tattagttgg ggtttgttga ttgtagatag tttgagtgtt 1800
tttgtatttt tagtagttag ttttttttta ttagttaata attttttata ttatattttt 1860
ttttttaaat tattggtgtg ttttttattt ttaattggat tttaattttt agtttatgtt 1920
tggtatttat tagatggttt gtaaaattta tttaattatt tttttattga agggaatgtg 1980
agtattattg tttttagttt ttagttgtta tattttgtgt agtagtgaat atgtgttttt 2040
tatttatatt attgtttttg aaggaaaaaa aattttggaa gaggaataaa tttttttgtt 2100
atagggttgt gtatgtaagt ttttggaaaa tgttgtgaga ttgttttttt tataaggttg 2160
tattaattta tatttttaaa tataggtgta agtgtttatt tttttatatt tttattaatt 2220
attatttttt tttaatattt attaatttta ggggtaatgt tttattgttt tagtttgttt 2280
tgtgtaatta ttaaggagat tgaatatttt tttataattt tattgttaat ttttattttt 2340
tttgtgaaat atattgttta tattttttat ttatttttta gttggattgt tatttttttt 2400
taatttatag aaattatttg ttgatttatt tttaaaagtg tgtatagttt tatagatttt 2460
attaagggtt tttattttta gttatgtagt aaatattttt tttgatttat tttatgtttt 2520
tttttatgtg atgaaatata tataatataa aaattattat ttttattatt ttaagtgtat 2580
agtttagtaa tattaagtat attaatattg atgtgtaatt attatttatt gttaaaatat 2640
ttttattttt ttaattttta ttgtatgttt ttaaatttat tttttgtgtt ttatattata 2700
taaaagtttt atatttttat ttattttaat ttattaattt ttttttttat gatttttggt 2760
ttttatattt tgtgtaggga aatttttgtt ggtgttgtat tttttatatt tttttttaat 2820
atttttttag atttgtttta atattttatt tttaagttat ttgatatttt tgtgttggta 2880
tgtatggttt gaggtgggtt ttttaattta tttttttgta ggtggggatt tgattatttt 2940
aatatttttt ttaaaaaatt tatttttttg tttggtgtag tggtttatgt ttgtaatttt 3000
agtattttgg gaggttgaag taggtggatt ataaggttag gagattgaga ttattttggt 3060
taatatagtg aaattttgtt tttattaaaa atataaaata ttagttgggt gtggtggtgg 3120
gtgtttgtag ttttagttat ttgggagttt gagataggag aattgtttga atttgggagg 3180
tggatgttgt agtgagttga gattgtgtta ttgtatttta gtttgggtga tagagtgtga 3240
ttttatttta aaaaagaaaa aaagatttat ttttttttga ttattttatt atgttatttt 3300
tgttaaaaag tatttgtttt tttttttaaa gtagattatt attttttaga agggagggat 3360
attttttgta tttataagtt tttttttgat ttggtagatt gtatatgtta tatatgttgg 3420

ttggtgatat gattgatagg aagttgaatg agaattagta tatttttaat aaagtaaagt 3480
agtaggtatt agaatttaga attaaataaa agttgttttt taatttatga atggatatgt 3540
ttgtggtttg tatttgtgtt ttaattatta agtaatttgg tttatttttt tgtgtaaagt 3600
attgttttag gttatagtgt aggaaattgt attagtttat ttttatattg ttataaagaa 3660
atatttaaga ttggataatt tataaggaaa agaggtttaa ttggtttata gttttatagg 3720
ttgtatagga agtatagtgg tatttgtttt tggggaagtt ttagagagtt tttatttatg 3780
gtagaaggta aagtaagggt aagtatttta tatggtagga atttgattaa gagagtgggg 3840
aggtgttata tatatttaaa taattagatt ttatgagagt tttattataa gaatagtatt 3900
aaagggatgg tgttaattta tttatgaagg atttattttt atgatttaat tatttttatt 3960
aggttttatt tttgatatgg gggattattt tgatgtgtga tttgggtagg gatatatatt 4020
taaattatat tagaaataga ggatgggtaa gatattgttt ttgtttttat gaagtttgtg 4080
atttttaaat gtttattatt aaggtgttag ttggagtatt gatgatttat atatttaatg 4140
gagttgtagt agttatgaaa aggattaagt agatttatat gttttgttgt ttaggttgga 4200
gtgtaatggt atgatttggg tttattgtaa tttttgtttt ttaggtttaa gtgatttttt 4260
tgttttagtt tttttagtag ttgagattat aggtatgtgt tattatgttt ggttaatttt 4320
gtatttttag tagagatggg gtttttttat gttggttagg ttggttttga atttttgatt 4380
ttaggtgatt tgtttatttt ggttttttaa agtgttggga ttataggtat gagttattgt 4440
atttggtttt aagatatgtt aaatgagtta attaaggtgt aaaatagttt gttagtgttt 4500
gtggggggaa aatgagggaa ggatataaat atatatatgt ttatataaga ataaaatatt 4560
tttgaaagaa tatttaaaat attagaaata ataattattt ttataaaagg tattgaaagt 4620
ttgggggata gtgtgaaatt tatttttttt tatggattat ttgtttaaat tgtttaatta 4680
tttgtttgta gttttatttt taaataaaga aaatgggaat tttagaagta tgtttatgat 4740
gaaataagaa agatgatata ggtatatgtg aaatagtaag ggaatagaag agtgttatgt 4800
ttagaagttt taattttgtg gttgtagaat tgtaaggtgg tgttgtaaag ggaggttttt 4860
gttttgttgt agggtgttgt taattttagg tgatgtggtt ggaaggtagg tagaaggaag 4920
tttttggaaa gaattaggaa atgttgtttt atttgtgtat ggggggttga ggtgtttgtg 4980
tagagatggt tgtgggtagg gaggttgttg agatttgttt agtattttat attgtttttt 5040
ttttgttttt gatttttatt gtttttttta gaaattttag tattttattg agtataaaga 5100
gaaataaaag gggaattttg attgagtggt tttttatata taaggatttg gagaggttgg 5160
attttggtat agttttttta tgggtagttg tatttttaat ttttggtaga atgatttata 5220
ttttttatta gtaatggagt agtttatttt tttatttgtt tataattttt aagataaatt 5280
aagtttattt tttataggtt taaaattgtt gataaagttg gtttgttttt gatgttttaa 5340
gatgataatt taagtggaga ggttatggtt tttattttta agattattta gtttttttgg 5400
gttttgattt tttgatttgt aaaatgggaa ttaaaatatt tttttattat ttttagtaat 5460
atttttagta gtagtaggtg gttaaattta agggttggag ttgttaattt ggtagtagta 5520
agaagtt 5527

<210> 383
<211> 10224
<212> DNA
<213> Artificial Sequence

<220>
<223> chemically treated genomic DNA (Homo sapiens)

<400> 383

agtttttata gttttagtat tgtttttttt attttatttt gattgtgttt ttgttgttta 60
tttttgttta tttattttaa ttttagtgtt ttttttagtt tttgggtttt aggtgatata 120
ttagttagtg ggatatgggt ttttataggt atttttagtt taatttagtt tttttttttt 180
ttttggtttt ttggttagta tttgtattat attggttttt attggatatt tttgtagttt 240
tgggtttttt ttatagatat attgttggat tagttgttta atttggttta tttgtagagt 300
tttttggttt atttgatttt aattgaaatt tagtttttta agaaggattt ggtgttggaa 360
taggtaggta ttggaaagtt agttgtttag gattattgtt ttattttatt agtatttttt 420
tgttattttt tatttttttt ttttagattt tagaaatttg ggagtggttg gagtgagaaa 480
atagaggtaa gtggtaggta attgttaagt attagtttta gtatgtgttt agttttttag 540
agtaggattt gtggttgtag gtgtgaaggt aaggtttgtg gaaatggtag ggagggtgga 600
ggggatgtag gaggtatgga tgtgggtggg gtgtttttat tttttagggt tagttagatt 660
tttttgattt ttttttaggt gggttgagat ttataggttg gatgtgttag aggtagtggt 720
tttagagtgg ttttgttgtg tttattgtag tgtagaggtt tttaagtgtt gtttatgatg 780
ttagaatgag tggtattgtt gtaggtgagg gtattttaga attttggatt tttaagtttt 840
atttttatat tttttatatg tattgttatt tttaatattt atttgtttgt agggagtgtt 900
aagttaagta ttgggaaaag tatggaaaga tttgtgtttt ggtagtttag ggtgatagag 960

ttaaatgagg gttgtagttg ttgagggttg attatttatg ttaagggaat ttatttagaa 1020
tgtattttg aatttaaga ttatggttta gttttgttg gagtttagt ttttgtagtg 1080
gagagtagag tgggtggtaa agttgttgat tgattttttt tttttttatt ttaagtgaag 1140
gtttgagatt ttttgtttta tttagtgggt aggttaagtg tgttgtttta gtaaattgga 1200
ttaggagggt tagggttgga tgtggggatt tttttttttt agtatagtaa agttggtttt 1260
tagaaatatg ggtatttttg tgtggtgttt tgtggttgtt gttgttgtgg ttgtttgggg 1320
tggggtgtga ggaggggatg aaggagggaa ggaagggtaa ggtggggggg gttttgtgag 1380
agtgtgttta gttttgtttt tgggttttat agtttttgta tttaggtttt tattgtgtgg 1440
ttttttttag tttttttttg ttgtttagtt tggattttgg gggaggtgtt gaagttgggg 1500
tttgttttgt ggttttgttt ggtttgtgtt tgttagtgtt taaagttagt gaagtatggg 1560
tttaattggg ttatgttggg ggagtttgag tttattgagt tgtgggagtt ggtatttgtt 1620
gggtgtgttg ggaagggttg tatttggttg gagtgtgtta atgtgttgtg tattgtgtgg 1680
ggtattgtgt gtaattttat atggtagttg gtttttggtt gtggttattg tttttagttt 1740
gtggggtttg ttatgtatat gtggtgtgat ttttgtggtg attttatttg gggtgttgtg 1800
tgtaaaggtt tgtagtgtgt gtgtgagtag tggttttgtg tgtttatgag agtggaaggg 1860
gtagttaagg ggtagtgtag ttgttgtggg ttaagttgtg gtagaggggg ttggtgggga 1920
tagtttttga ggattaggtt tgttattttt gttttattgt tgaagagtgt gtgaaaatgg 1980
tttatttttt gttgtatttt atttgtattt gggttataga tgagtagagg tggttgttta 2040
tatgtaaaaa tatgttgatt ttaagttttt tatttttaaa atgttttggt tttttttgag 2100
aaagggtttg tgtttattgt ttttggagtt tattttttta ggtttgtttt tttttaaata 2160
tttatgattt tttttagaat ttttagggtg aagggaaatt attatttatg ggagggagtt 2220
tggaaaaatt tagaattttt ggtgggtttt ttgtaagtag gagttttgtt gagtttttat 2280
ttagtaaata tttttttttg atttagtgaa ttagatgtta aaatatgtat gtagttatat 2340
atttagtagt ttttttgtat ttttgggaat tgttagtaag taaaggttgt ttttttttgg 2400
gtagatatta gttggaatta ttaggggtgt ttttatagtt ttttttgtta gtttggattt 2460
tattgtagat ttgttgaatt aattgttggg agtggatttt aggtattagt aaattttaaa 2520
aattttttaa attattgtaa tatggagttt gggttgagta ttattgtttt ggtttattta 2580
ggaatttgtg gatggatagt gttttaggtt tgtgtgtgta tggagatttt tttatttggt 2640
ataagaggat attataaatt tagttggggg gagtataaag ttgtgataga atgtaaagaa 2700
tgaataaggg gttgagtgtg gtggtttatg tttgtaattt tagtattttg gaaggtggag 2760
gtgggtggat tatttgaggt taggagttta agattagttt ggttaatatg gtgaaatttt 2820
atgtttatta aaaaataaaa aaaaatgagt taggtgtagt ggtgggtgtt tgtaatttta 2880
gttatttggg aggttgaggt gggagaattg tttgaatata ggaggtggag gttgtagtga 2940
gttgagattg tgttattgtt ttttagtttt ggtgatagag tgagattttg ttttaaaaaa 3000
aaaaaaaaaa aaaaaaagaa taaggttggg atattgtagt gtttttaaag agaaataaag 3060
tagttatgga gataagaagt aggatgattt gggtatgttt attagaggta gagataaggg 3120
agaaattaaa gataagtttg ggtttttgtt tttagtaatt gggagtttag tggttatttt 3180
tgttgtaaag aggaagttgg gtaagtgtag tagtgaggtt gaagaaaagg gaattaaatt 3240
ttggttatgt ttatttgaaa tgttttttag atattttagt gaaggtattg gtatggagga 3300
tttagtttga gggtttaggt tagtgtttta gttgtggatt tggggtagat gaatgtagat 3360
agattaggtt agtgattagg attgagttta gattttattg tgagatatgg aagttgagtt 3420
agaatttgta aaggagttga gtaggagttg tagggggtag gaggaaaatt gggagagtgt 3480
agtttttggg agttaaaggg agtaagtttt aaatgatgtt gagggggtga gaatggagaa 3540
tggaatattg gattttattt ggtagtatat agattgttga ggattttgtt ttgggtagtt 3600
ttttggagga agaggtaagt ttggttggag tgggtagagg ggagagtgaa ggtgaaggat 3660
tagagtgtat agagattagt gttttggttt gaggggagta gagataggtg ataattatag 3720
ggtagatgta ggttaaaggt gtttagtttt ttttttaagt aaatgggtag atgtatttta 3780
tatatgtttt tagtgaaggg ttgggtgtgg tggtttaagt ttgtagtttt agtattttgg 3840
aaggttgagg tgggtggatt atttgagatt aggagtttga gattagtttg gttaatatgg 3900
tgaaattttg tttttattaa aaatataaaa attagttggg tatggtggtg ggtgtttgta 3960
attttaggta tttaggaggt tgaggtagaa gaattgtttg aatttaggag gtggaggttg 4020
tggtgagttg aaattgtgtt attgtatttt agtttgggtg ataaaagtaa gatgtagttt 4080
tttgttgttg tttttttaat tgttaatgag gaaaggggaa gttttgtgtt aggtgataga 4140
gatttaattg ttgagtaggt ttttttgttt gtggtttttt ggttggtttt tagatgttta 4200
ggtggttaat attagagttt gtgtagtagt gtgaggtaat ttattgagat aggttgggtt 4260
tgtggagttt ggtgagtagt ggtttttttt ttggggtttt tttttaattt ttgggatatt 4320
tttttgattt ggagtttttt tgttttattg ttaggttttt ttgtagattg taagtttatt 4380
tgttattatt gttgttgtgt gtttgtttgt ttggattgtt gtgggttttg ggatttgggt 4440
tgggaatttg tggtggagtg ggatatgaat gtggtgagtg tggggttgag ggtgtatggg 4500
aagggtgagg atgggtaggt tatagtgtag gtatttttga gggttgtttg ggtgttgtgt 4560
gtaaggagtg ttttaattgt tgattttttg gtggtataga gaggttaatt ttgtgtgggg 4620
gttgggaggg gagtttggat tgttggtttt gtaagtattt tatttgttgt aagtggattt 4680
gggtttaggt tgatttaggt tttgtgtatg tgtatttttt gtattttttt gtttttgttt 4740

ttggttagag gttattttg tgtgtttgtt tggatgttgg tatttgtttt tgttttttgt 4800
ggtaggtggg gtttgtgagt ggagttttgg agtgatgagg ttattttgg gggtgaagtg 4860
tgtgtgtttt tgttttggtg tttttgtttt aatgagataa gagttagatt ttggtgattt 4920
atgttttagt tttaatggtt gtggtgtggt tttggtttgg gtgtatgtgt atattgatat 4980
gtgtatatgt atgtatgtga ttggggtggt ggttggtggt tatggatgtg taggattggg 5040
ggatgggtgg gtatggttat gggtgaggtg gaggtgtttt tttttgaaat gatttggagt 5100
agtatgatga gtagtggtta ttgtagttaa gaggatttgg atttggagtt tgagtagtat 5160
tttattgtgt gaattttgtt agtttgtagg ttgtgttggg attaggtggg agttaggggg 5220
tgttggtggg tgggaggga agtggttgtt ggagttttgt tttttttggt ttgttgttgt 5280
gttttgggtt ggtgggtagt tttatttttt tggttatgtg gtttttgtg ggttttggtt 5340
ggggatttgt ttgtggaatt gtgtgtaaga ttttgatttt attgtttaga tgttgggtgt 5400
tggggttttt ttggtttttg ttatagatag gttgaatatg gaaaaagtag ttgtatggtt 5460
tgtggtagat ttgagttggg tattatttag ttatgattaa agttgattga gtagtttgga 5520
ttagtatttt gatttttgtg tttgaatgtt tttgtttttt ttttggggag attaggggag 5580
gatgtggaga gggaagagtt tttgttagga attgagaagt atgtttagga aaatttgaga 5640
ggtagagaga gattttgttt ttttatttgt atttttgtat ggagttagtt gagtttttat 5700
tttttttttg ttttggtttg ttattagttg ttggaatgtg gaagattttg tttttttttt 5760
ttagggtgga tttggagaaa gatttgggaa tagataggaa agaagttttg ttttggatta 5820
taagtattta ggagtatttt atttatagga aggggaaag ttagattata aaatgtttaa 5880
agaggtggaa aaagagattt aggttattaa tttaggattg taaggtgttt tggaattttt 5940
taggtatttt tattattgga gaattgtgtg ttagatgtta ttggtgtgat tattaggttt 6000
agagaattag gtttaggtat taggaaaaag aaatagggat tgtgaagttt agtatgtttg 6060
gtagaaatgg ggtggaaatt tttatttaag taaagaaagt ggagttgtga gtgatgtttt 6120
agataaaatt ttataaaatt ttttataaaa tgggtggtgt ttagtatgtt aaaattttag 6180
tttagagttt gggtgtaagg gttgagttga gtgtagattt ttgggtttgt ttttatgtta 6240
gttagttttg agttattttt tattgtggaa aggtgggaaa attataagat attaattaat 6300
tgaaaaggag ggttagttat ggaggtgtat atttgtaatt ttagttattt gggagggtga 6360
ggtagaagga ttatttgaat ttgggaggta gaggttgtag tgagttaaga ttgtgttatt 6420
gtattttagt ttgagtgata gagtgagatt ttgttttaaa aatagaaaag gaagttaagt 6480
atggtggttt atatttttaa tgttaatgtt ttgggaggtt aaggtaggtg gattatttgt 6540
aattaggaat ttgaggttag tttggttaat atggtgaaat tttattttta ttaaatatat 6600
aaaaattagt tgggtatggt ggtgtgtgat tgtagtttta gttatttggg agattgaatt 6660
attttaattg ggaggtaaag gttgtagtga gttaagattg tgttattgta ttttaatttg 6720
ggtgataggg tgaggttttg ttttaaaaaa aagaaagaag gttgggtttg gtgatttatg 6780
tttgtaattt tagtattttg ggaggttaag gtaggtagat tatttgaggt taagagtttg 6840
agatttgtta ggttaatata gtaaaatttt gtttgtattg aaaatataaa aaaattattt 6900
ggttatggtg gtgtgtgttt gtaattttag ttattgggga ggttgaggta ggagtattat 6960
ttgaatttag aagatagagg ttgtagtgag ttgagattgg gttattgtat tttagtttgg 7020
atgagagagt aagattttgt tttaaaaaaa aaaaaaaaaa aaaagaaaga ataggaggtt 7080
gagaagtttt aagttatatg ttaaaaaaaa agaaaaaaat attagtttta ggttaggtgt 7140
agtggtttat atttttaatt ttagtatttt ggaaagttga ggtgggtgga ttatgaggtt 7200
aggagtttaa gattagtttg gttaaaatgg tgaaattttg ttttgattaa aaatataaaa 7260
aattagttag ttgtggtggt aggtatttgt aattttagtt atttgggagg ttgaagtaga 7320
gaattgtttg aatttaggag gtagagattg taatgagtta agattgtatt attgtatttt 7380
agtttggaaa atagagtgag attttgtttt aaaaaaaaaa ttattagttt ttatggatag 7440
tggtagagtg gagggtgggt ttttatggtg tagaaggaaa atttatggtt tttgttgtgt 7500
atttgattgg gatggttgtt gaaatttttt tttagtaggt agttttggaa atagaaaaag 7560
aaattttttt tttttagaa ttttggaagg gttgtgtagt gtttttaatt taagtttgtt 7620
ttttgagtga agatagggag gtttattatt agaaggaaag gggttggaaa tgaggttatt 7680
gtattttagt ttagggtttt tgggttattt aggaagggaa gaaggagtaa gtttttttat 7740
tgttaggtag gagtttagag ttattataag aataagttag tattattttt gtgttttttt 7800
tgttttgtaa ataaaatgat tttttttttt gttttggtat tagagtttgt ttggtatttt 7860
ttttgttttt agtatttttt ttatttgggt attttttttt gttggtgtat tgaataaata 7920
tatttattgt tttatttata gtttttagtt tttatttttt agggtttata ttatttgttt 7980
ttattaattt gataaggttg tttattgttt ttagtaaggt ttgtattggg gtttttattt 8040
tagtgttttt ttttatttag gagattttg gatatttggg gaagaaaatg agtttaaatt 8100
tttatttttt tttttttatt tttttttgt aaggttttgg ttttagtttt tagttttata 8160
tttttgttgg ttgtagaata gtagtgggtt ttgggtaagg agtattttgt taaaatgttt 8220
tattttgttt ttttatttgt tttttttatt tgtttttatt agatggttta agtgtttaag 8280
gggattttag ggtggagtta gggagaattt tggttttttt gggttaggta taagattatt 8340
ttataggaaa ttttgtggga atttttttgg gataaagtat tggttagtgt tgagtttagt 8400
tgtgtttgtg atatttgtat tttaattagg gtttatttga tgttaatagg aagtaaggtt 8460
gatgtagtgg ggttaaggga gtttgggaga agaaagttgg tttagagttt tggttgtttt 8520

gttttatatt ttattttttt ggtaagaatt tagtttttag atgaggtggg gagtgagtgg 8580
ttgagttaaa aattttgggg ttgggtatga tggtttatgt ttgtaatttt agtattttgg 8640
gaggtgaagg taggtggatt atttgaggtt aggagtttaa gattaatttg gttaatgtgg 8700
tgaaatttta tttttattaa aaatataaaa attagttggg tgttgttgtg gtatgtgttt 8760
gtagttttag ttatttggga gtttgaggta ggagaattgt ttgaatttag gaggtagaat 8820
ttgtagtgag ttaagattta gttattgtat tatagtttgg gtgatagagt gaggttttgt 8880
tttaaaaaaa aaaaaaattt ttgggttaaa tttttagata gtataggtag gtgtagaaat 8940
ttattaggaa gttgtttgtg tatttttggt agattggagt ttggtttaaa gttgtttttt 9000
atgtagtttg ggttaaggtt aaatattatg ttatagtgat tttttttatt atgtgtgaga 9060
tatggagaat tggttttaag tattattttg tttattggtg gttggattat tgatgtgtat 9120
tattttttat tttttttatt ttgtagtggg ttatggtttt gtgttggggt agaggagaaa 9180
aatgggttgt tttttttagg ataaattttt attttaattt aattagggtg ttgtgattag 9240
aatgtgtaat tgaggtgtga ttttattgat tttttttttt tttgagattg agtttgtttt 9300
ttgttgttta ggttggagtg tgatggtatg attttagttt attgtaattt ttattttttg 9360
agtttgagta atttttttgt tttagttttt taagtagttg ggattatagg tatgtgttat 9420
tatgtttggt taattttgta tttttagtag agatggggtt tttttatgtt ggttaggttg 9480
gttttaaatt tttgatttta ggtgatttat ttgttttggt tttttaaagt gttagaatta 9540
taggtgtgag ttaatgtgtt tagtttgttt ttgttttttg tgttttgaag tagggtttta 9600
tttagttttt taggttggag tgtagtgata tgataatagt ttattgtagt tgtaattttt 9660
tgggtttaaa tgattttttt attttagttt tttgaatagt tgggattata ggtatattat 9720
tatatttggt taattttttt tttttttttt ttagtagaga tgaggttttg ttatgttgtt 9780
taagttggtt ttaaattttt gaggattaag tgattttttt attttagttt tttaaaatgt 9840
tgggattgta gatgtgagtt attatattta gtttgatttt attttaaatg agagtttttt 9900
tttagagttt tttagttgtt tttggttttt ggttatgtgt ttttagttgt ttttgttttt 9960
gtggtatttt taaggttata tttagtgttg aggttttagg taggtagtag agagaagtta 10020
aatgattttg tttttttttt atttatttag agtatgtaaa attaggagta gtggtgggtt 10080
tagggtgggt attagttatg tatatgtata ttagggatag ggggttaaag gtagttagtt 10140
tttaaagatt gttttagagg ttatttttta gagaagtttt gggtttttta agggttttgt 10200
gtttatgttg gtttattttg tagg 10224

<210> 384
<211> 10224
<212> DNA
<213> Artificial Sequence

<220>
<223> chemically treated genomic DNA (Homo sapiens)

<400> 384

tttgtaagat gggttagtat ggatataggg tttttgagga atttagggtt tttttgaaaa 60
atggtttttg gggtagtttt tggaaattga ttgtttttgg ttttttgttt ttgatgtata 120
tatatatagt tggtgtttat tttgaattta ttattgtttt tggttttgta tgttttgggt 180
ggataaggga aagatagaat tatttggttt ttttttgttg tttgtttagg gttttagtat 240
tgaatgtagt tttaaggata ttatagaagt aggggtaatt gaaggtatat ggttaggggt 300
taggaatagt tgagggattt tgaagaggga tttttattta aagtaaaatt aggttgggtg 360
tggtggttta tatttgtaat tttagtattt tgggaggtta aggtaggagg attatttgat 420
ttttaggagt ttgagattag tttgggtaat atagtaagat tttattttta ttaaaaaaag 480
aaaaaaaaaa attagttagg tgtggtggtg tgtttgtagt tttaattgtt taggaggttg 540
aggtgggagg attgtttgag tttgggagat tgtagttata gtaagttatt attgtgttat 600
tgtattttag tttggggaat tgagtgagat tttgttttaa aatataaaaa ataaaaatag 660
gttgggtatg ttggtttatg tttgtaattt tagtattttg ggaggttgag gtgggtggat 720
tatttgaggt taggagtttg agattagttt gattaatatg gagaaatttt gtttttatta 780
aaaatataaa attagttagg tgtggtggta tatgtttgta attttagtta tttaggaggt 840
tgaggtagga gaattgttta aatttgggag gtggaggttg tagtgaattg agattgtgtt 900
attgtatttt agtttgggta ataagagtga aatttggttt taaaaaaaaa aaaaattagt 960
aaaattatat tttaattgta tattttgatt atagtatttt agttgagttg gagtgagggt 1020
ttgttttgga gaaggtagtt tatttttttt ttttgttttg gtatggggtt atgatttatt 1080
gtagggtgag gaggtggag agtggtgtat attagtagtt tagttattag tggatagagt 1140
agtatttgga gttagttttt tatgttttat atatagtgag aaaaattatt gtgatatgat 1200
gtttaatttt gatttaagtt gtataaaagg tagttttagg ttaggtttta atttgttaga 1260
ggtatatagg tagttttttg gtgggttttt gtatttgttt gtgttgtttg gagatttggt 1320
ttaaagattt tttttttttt tgagatgaag ttttattttg ttgtttaggt tgtagtgtag 1380

tggttggatt ttggtttatt gtaagttttg ttttttgggt ttaagtgatt tttttgtttt 1440
agatttttga gtagttggga ttataggtgt gtgttataat aatatttggt taatttttgt 1500
atttttagta gagatgggat tttattatat tggttaggtt ggttttgaat ttttgatttt 1560
aagtgatttg tttgttttta ttttttaaag tgttgggatt ataggtgtga attattgtat 1620
ttgatttaga gatttttaat ttgattattt atttttattt ttatttaggg attggatttt 1680
tgttggaagg gtggagtgtg ggatagggta gttagggttt tgaattgatt tttttttttt 1740
agattttttt ggttttattg tattagtttt attttttgtt gatgttagat aggttttagt 1800
tagaatgtga gtgttataga tatagttaag tttagtgttg attaatattt tgttttagaa 1860
gaatttttat aaggtttttt gtagaatgat tttgtgttta gtttaggaga gttagggttt 1920
tttttgattt tgttttggag tttttttaag tatttaaatt atttgatggg gataaatgga 1980
gaggatagat gagggagtag ggtggagtgt tttagtagaa tgttttttat ttagaatttg 2040
ttgttatttt gtagttagta aggatgtggg gttaagaatt aaggttaggg ttttatagga 2100
aaaaggtaaa gggggagggg tggaatttta agtttatttt tttttttaag tatttaaagg 2160
ttttttggat ggagaagagt attggagtaa aaattttagt ataaatttta ttggggatag 2220
tgggtaattt tgttgggtta gtaaaaataa atggtgtggg ttttggaaaa tgagggttgg 2280
aggttgtgaa taaagtagtg gatgtgtttg tttagtatat taatgggaag aagtatttag 2340
atgggaggag tattaggggt aggagaaatg ttagatagat tttagtgtta gggtaagaag 2400
gaagattatt ttgtttgtag aatagggagg gtatagggat ggtgttaatt tgtttttgtg 2460
atggttttga gtttttattt aataatgaga aagtttgttt tttttttttt ttttggatga 2520
tttaggagtt ttgggttggg atgtagtgat tttattttta gttttttttt ttttggtgat 2580
gaattttttt atttttattt agaaaataga tttggattag aggtattgta tagttttttt 2640
aggattttaa aggaggaaga gttttttttt ttgtttttaa agttgtttgt tggaagagga 2700
ttttaatagt tattttagtt ggatgtatag taggattatg gaattttttt tttgtattat 2760
agggatttat tttttatttt attattgttt ataaaaattg atggtttttt ttttgagata 2820
gagttttgtt ttgtttttta ggttggagtg tagtggtgtg attttggttt attgtaattt 2880
ttgttttttg ggtttaagta attttttgtt ttagtttttt aagtagttgg gattataggt 2940
gtttgttatt ataattggtt aattttttgt atttttagtt gagatggggt tttattattt 3000
tggttaggtt ggttttgaat ttttgatttt atgatttatt tattttggtt ttttaaagtg 3060
ttgggattaa aggtgtgagt tattgtattt ggtttaaaat tgatgttttt tttttttttt 3120
ttaatatata atttgggatt ttttagtttt ttattttttt tttttttttt tttttttttt 3180
tgagatagag ttttgttttt ttatttaggt tggaatgtag tggtttagtt ttgatttatt 3240
gtaatttttg ttttttgggt ttaagtgata tttttgtttt agttttttta gtagttggga 3300
ttataggtat atattattat ggttagataa tttttttgta tttttagtat agatggggtt 3360
ttgttatgtt ggtttggtag gttttgaatt tttggtttta agtgatttgt ttgttttggt 3420
tttttaaaat gttgagatta taggtatgag ttattaagtt tagttttttt tttttttttt 3480
gagatagagt tttattttgt tatttaggtt ggagtgtagt ggtatgattt tggtttattg 3540
taatttttgt tttttggttg aagtgattta gttttttaag tagttgggat tatagttata 3600
tattattatg tttggttaat ttttgtatgt ttagtagaga tagggtttta ttatgttggt 3660
taggttgatt ttgaattttt gattgtaaat gatttatttg ttttggtttt ttaaagtatt 3720
ggtattagag gtgtgagtta ttgtatttgg tttttttttt tatttttgag atagagtttt 3780
attttgttat ttaggttgga gtgtagtggt atgattttgg tttattgtaa tttttgtttt 3840
ttaggtttaa gtgatttttt tgttttattt ttttaagtag ttgggattat aggtgtgtat 3900
ttttgtggtt agtttttttt tttaattggt tagtgttttg tggttttttt atttttttat 3960
agtggaaaat ggtttaggat tgattgatat gaagataagt ttaggggttt atatttaatt 4020
taatttttgt atttaagttt tgggttaaga ttttggtgtg ttgagtatta tttattttgt 4080
aaggaatttt gtaaaatttt atttgaagta ttatttataa ttttattttt tttatttaaa 4140
taaggatttt tgttttattt ttgttaggta tattgagttt tatagttttt gttttttttt 4200
tttggtgttt aggtttggtt ttttgagttt ggtggttata ttaatggtat ttggtatata 4260
gttttttgat aatggggata tttaggaggt tttgagatat tttatagttt tgggttagta 4320
atttggattt ttttttttat ttttttaggt attttataat ttagtttttt ttttttttgt 4380
gggtaaagtg tttttgaatg tttatggttt aaaataagat ttttttttta tttattttta 4440
aatttttttt tagatttatt ttagaggaag ggaatagaat tttttatatt ttagtagttg 4500
gtgataggtt agaataggga agaggtgagg gtttagttgg ttttatatag gagtgtagat 4560
ggaggagtag gatttttttt tgttttttaa gtttttttaa atatattttt taatttttgg 4620
tgaggatttt ttttttttta tatttttttt tagttttttt aaggagggag taggagtatt 4680
tgaatgtgga aattgaggtg ttagtttaaa ttgtttggtt ggttttagtt atagttggat 4740
aatgtttggt ttaggtttat tataagttat atagttgttt tttttgtgtt taatttgttt 4800
gtgatagaaa ttaagggggt tttggtattt agtatttagg tggtggaatt ggggttttat 4860
gtatggtttt gtgggtaggt ttttggttag gatttgtggg gagttatgta gttaggaggg 4920
tggggttgtt tattgattta ggatgtggta atggattggg gagggtggag ttttagtgat 4980
tgtttttttt tttgtttgtt ggtatttttt ggtttttatt tggttttggt gtggtttgtg 5040
agttagtgag gtttgtgtgg tgaagtattg tttgagtttt gagtttgagt ttttttggtt 5100
gtagtagtta ttgtttgttg tgttgtttta ggttattttg aaagaaggtg tttttgtttt 5160

gtttatagtt gtatttgttt gttttttagt tttgtgtgtt tgtagttgtt aattattgtt 5220
ttggttgtgt gtgtgtgtgt atgtgtgtta gtgtgtgtgt gtgtttgggt tagagttgtg 5280
ttgtaattgt taagattgaa atgtagattg ttgggattta gtttttgttt tattggggta 5340
ggaatgttgg ggtggggata tgtatgtttt gtttttagga atgattttat tgttttggag 5400
ttttatttat agattttatt tattataggg aatgggggtg ggtgttagtg tttgggtaag 5460
tgtataagag tggtttttgg ttggaggtga gggtgggaag gtgtgggaag tgtgtgtgtg 5520
tggagtttgg gttagtttgg gtttgggttt gtttgtagtg ggtggagtat ttgtggagtt 5580
ggtaatttag gttttttttt tagtttttgt gtagaattag tttttttgtg ttgttgggaa 5640
attggtaatt agaatgtttt ttgtgtgtgg tatttaggta gtttttgaga atgtttgtat 5700
tgtggtttgt ttatttttgt tttttttata tgtttttggt tttgtgttta ttatgtttgt 5760
gttttgtttt attgtgggtt tttagtttag gttttggggt ttgtaatagt ttaggtagat 5820
gagtgtgtgg tagtggtagt ggtaggtgaa tttgtaattt gtagagaggt ttggtggtga 5880
ggtggaggag ttttaggttg gggaaatgtt ttggagattg aagggaagtt ttagggagag 5940
ggttgttgtt tgttaggttt tgtaggtttg atttatttta gtgggttatt ttatattgtt 6000
atgtggattt taatgttggt tatttgggtg tttggaaatt ggttggaagg ttataggtag 6060
agaggtttgt ttaatagttg gatttttatt gtttagtata gaattttttt tttttttatt 6120
ggtaattaaa aaaataataa taaaaaattg tgttttgttt ttgttattta ggttggagtg 6180
taatggtgtg attttggttt attgtaattt ttgtttttttg ggtttaagtg atttttttgt 6240
tttagttttt tgagtattta ggattatagg tgtttgttat tatgtttagt taatttttgt 6300
atttttagta gagatggggt tttattatgt tagttaggtt ggttttaaat ttttgatttt 6360
aggtgattta tttgttttgg ttttttaaag tgttgggatt ataggtttga gttattgtat 6420
ttggtttttt attgggaatg tatatggaat atatttgttt atttatttga aggaaaaatt 6480
aaatattttt aatttatgtt tgttttgtgg ttgttatttg tttttatttt ttttagatta 6540
agatattggt ttttatatat tttaattttt tgtttttatt ttttttttta tttattttag 6600
ttaggtttgt tttttttttt aggaaattgt ttgggatagg gtttttagtg atttgtgtat 6660
tattaaatgg aatttagtgt tttatttttt atttttattt ttttagtatt atttgaagtt 6720
tgtttttttt gatttttagg ggttatattt ttttagtttt ttttttattt tttgtagttt 6780
ttgtttagtt tttttgtaga ttttgattta atttttatat tttatgatga agtttgggtt 6840
tagttttgat tattggtttg gtttgtttat atttatttgt tttagattta tggttgaaat 6900
attgatttaa atttttagat tagatttttt gtgttagtat ttttattagg atgtttaaaa 6960
gatgttttaa gtgaatatgg ttaaaattta attttttttt ttttagtttt attgttatat 7020
ttgtttagtt ttttttttgt agtaaaaatg gttattaggt ttttagttat tggagataaa 7080
agtttaaatt tatttttgat tttttttttg tttttatttt tgataaatat gtttaaatta 7140
ttttgttttt tatttttatg gttattttat ttttttttga gaatgttgta atgttttagt 7200
tttgtttttt tttttttttt tttttttttt gagatagagt tttattttgt tgttaaggtt 7260
ggagggtagt ggtatgattt tggtttattg taatttttgt tttttgtgtt taagtaattt 7320
ttttatttta gttttttgag tagttgggat tataggtatt tgttattatg tttggtttat 7380
ttttttttat tttttagtag atatgaggtt ttattatgtt ggttaggttg gttttgaatt 7440
tttgatttta ggtgatttat ttgtttttgt tttttgaagt gttgggatta taggtatgag 7500
ttattgtgtt tggttttttg tttatttttt gtattttgtt ataattttgt gtttttttta 7560
gttgaatttg tgatgttttt ttgtattgga tgagagggtt tttatgtata tatagatttg 7620
ggatattatt tatttataag tttttaaata ggttagagta gtgatgttta atttagattt 7680
tatgttataa taatttgggg agtttttaaa atttattgat gtttagggtt tatttttagt 7740
agttgattta ataggtttgt ggtgggattt aggttagtgg ggaggattgt aaaagtattt 7800
ttggtgattt tagttggtgt ttatttaggg gagagtaatt tttgtttgtt ggtgattttt 7860
aggggtgtag aaggattgtt gggtgtgtgg ttgtgtgtat attttagtat ttgatttatt 7920
gggttagaaa agggtgtttg ttaaataaag atttaataaa atttttgttt gtaggggggtt 7980
tattaaaggt tttaaatttt tttaggtttt tttttatagg tggtaatttt tttttatttt 8040
aaaggttttg gagggggtta tgagtgtttg agaagaggta agtttgggaa gatggatttt 8100
gaggatagta ggtataaatt tttttttaag aagggttaag gtattttaaa gataagaaat 8160
ttaaaattag tgtattttta tatataagta gttatttttg tttatttgtg gtttagatat 8220
gagtggagtg tgataaggga taaattattt ttgtgtattt tttagtgatg gggtgaaagt 8280
aatggattta gtttttggga gttgtttttg ttgatttttt ttgttgtgat ttgatttgtg 8340
gtgattgtgt tgttttttgg ttgttttttt tgtttttgta ggtgtgtggg gttattattt 8400
atgtgtgtat tgtaggtttt tgtgtatgat gttttagatg aagttgttat agaggttgta 8460
ttatgtgtgt gtggtgggtt ttgtgggttg gaagtggtgg ttatggttag ggattagttg 8520
ttgtgtgggg ttgtatgtgg tgttttgtgt gatgtgtagt gtgttggtat gttttagttg 8580
ggtgtggttt tttttagtgt gtttagtggg tgttagtttt tgtagtttaa tgagtttagg 8640
tttttttgat atggtttggt tgggtttgtg ttttgttggt tttgggtgtt agtaagtgtg 8700
ggttgggtgg ggttataggg tgggttttga ttttagtgtt ttttttagga tttagattgg 8760
gtggtgggaa ggagttgagg agagttgtgt aatggaaatt tgggtgtagg gattgtgggg 8820
tttgaaggtg gggttgggtg tgtttttgta gagttttttt tgttttgttt tttttttttt 8880
tttttgtttt ttttttatat tttattttgg atggttataa tgatggtgat tgtaaagtat 8940

tatgtggaga tatttgtgtt tttggaggtt agttttattg tgttagagga agagggtttt 9000
tatatttggt tttggttttt ttggtttggt ttgttgaagt aatatatttg gtttatttat 9060
tgggtggggt aggaagtttt gagtttttat ttggggtgag gaggagggag attggttagt 9120
agttttattg tttgttttgt tttttattgt ggagattggg gttttggtag aggttggatt 9180
gtgattttga ggtttagggg tgtattttgg gtggattttt ttggtatggg tggttggttt 9240
ttagtaattg tagtttttat ttggttttgt tattttgggt tgttaggata taagtttttt 9300
tatgtttttt ttagtgtttg atttggtatt ttttgtaggt aggtgggtat tgaggatggt 9360
aatgtatgtg ggggatgtgg gagtagggtt tagaggttta aggttttagg atatttttat 9420
ttgtagtaat attatttatt ttggtattgt gagtagtgtt tagaagtttt tgtattgtag 9480
taagtatagt ggggttgttt tggagttatt gtttttagta tatttagttt gtaggtttta 9540
gtttatttgg gggaaagtta ggaaggtttg attggttttg gaaggtgggg gtattttatt 9600
tatatttatg ttttttgtat tttttttatt ttttttgtta tttttatagg ttttattttt 9660
gtgtttgtag ttgtaggttt tgttttgagg ggttgaatat atgttggagt tggtgtttgg 9720
taattgtttg ttatttgttt ttgttttttt gttttagttg tttttagatt tttgggattt 9780
aggagagaga agtggagagt ggtaggaagg tgttggtaaa gtgggatagt ggttttgagt 9840
agttaatttt ttagtgttta tttgttttaa tattaggttt tttttaggag gttgggtttt 9900
agttagggtt agatgggtta ggaaattttg taagtgggtt aggttgggta gttggtttag 9960
tagtgtgttt gtgaggaagg tttgaagttg taagggtgtt tagtggaggt tagtgtgatg 10020
taggtgttgg ttagggggtt aaggaaaagg aaggggttgg gttgggttga gaatgtttgt 10080
gggggtttat gttttattag ttggtgtgtt atttgaggtt taaggattga aaagagtatt 10140
ggggttgagg tgggtgggta aagataaata gtagggatgt agttggggta ggatggggag 10200
ggtggtattg gggttgtggg ggtt 10224

<210> 385
<211> 11787
<212> DNA
<213> Artificial Sequence

<220>
<223> chemically treated genomic DNA (Homo sapiens)

<400> 385

tgtgaaatat tagttatgaa gaaattttga taataatatt ttagtgtaga attgtatatt 60
agtaatagta tgtttggatt tttttagata tgtggtatta atatatgttt ggaattttaa 120
gtgttagatt aagaagaaaa atttaagata taaagtggtg ttttaataat ttgagtttta 180
ttatttttaa tgttaagaaa ttattatata tataaatttt atgtttgtaa ttttagattt 240
attgttgtat aggatttttg gtaattataa gttagttgta ttaagtaaat tttgatttat 300
ttggttggtg gttttgtgaa tgataattat ttttaggtgg gtttaatgga aggttggaga 360
aggtattgtt tgataagtta tattttgtta gttttagaat tttgagtgaa tgttgttgtt 420
ttgttttgtt tgtttttata ttgtaaattg gttatgagtt tatttattat ttttttttgt 480
tgtgaatttt tagtaatata ttttattaaa agattttttt tgataaagta gatttttatg 540
agttgaaata tttattgatt ttaatgttta tttttagata gttttgtata ggaattttta 600
tgtttaagga tagttgattt tggtaaattg gattttttaa aaattagatt atagaatttt 660
tgtttgtaat ttttttattt aaggaatatt tagataattt ggtttggaga gaagattatt 720
aaattttttt ttatataata ggtttttttt attaggtttg taagggataa atagtgaata 780
tttatttgta attatgtaat tgttttgtag agtaagtgta gttataagtg gtaagaaatg 840
aatgagtatg gttgtgtttt aattataatt attatggatg gatatatttg tattttttat 900
gttagatgga attttgtttt gttgtttagg ttggagtgta gtgatgttat tttgatttat 960
tgtaattttt gttttttagg tttaagtagt tttgttttag ttttttaagt agttgggatt 1020
ataggtgtat ggttaatttt tgtattttta ttagagatgg ggttttatta tattggttag 1080
gttggtttta aatttttgat tttgtgattt gtttgtatta attttttaaa gtgttgggat 1140
tgtaggtgtg agttattgtg tttgatttag gattatattt aaatgattta taaatgtaaa 1200
aattattttt atattataga tttgatttaa ggattgtagt ttgttgtgtt tttgtttttt 1260
gaatgggatg tggtggtata gtagttggga ttatagttat atattattat gtttatttaa 1320
ttttgtattt ttagtagaga tggggtatta ttatgtttat taggttgatt ttgaattttt 1380
gattttaggt gatttatttg ttttaatttt ttaaagtgtt gggattatag gtgtgagtta 1440
ttgtgtttgg tttgtatttt tatttttaaa ttttagtatt tttgaataat aatgggtaga 1500
gaaatatttg tagtagttgt ttataataga gtgaaataaa aagtttgagg aattgttaat 1560
agaaaaatgg gtggttgtgg ggagtattgg gatttgtatt tgtatataat ttagggtagt 1620
ttttaatgag tttatgagtt atttagggat tttattaaaa tgagattagt tttgagtggg 1680
gtttttttta tagatttttg ttattatatt ttgaagagta agggtttagt tgattttaag 1740
taggtgtgta tttttttgga tagtgtttgt agaaaaatag aattatattt atggatttta 1800

tgattatatt gttagttttt tggttaaggg gttgagttag gtatttattg tagatagaaa 1860
taaagtagat gtatgtttat tagtgagtat tgagatattt agtatggtta tttatgaatt 1920
gaagtttggt ttttttttat ttattaattg attatttttt attttaagta agtattttat 1980
attttgtata ttagtatatt tttggatttt gtttttttat atggtttgat attttgaata 2040
ttagattatt taagagtgag atttaattta tttttaaatt gtttttaaaa ttagggaaat 2100
aaaagagaat gaaattgtgg taattttttt tttttttagt tattagatgt atgttattta 2160
tggtgggttt tggttttttt aaagataatt ggaagatttt attggattga tagagagaaa 2220
ttgtgtaatt ttattttagt atgttaagat gaagaaatgg ggggatttgt agataggtta 2280
ggagatatgg taagtaatat ttttgatata tttttataaa ttattttagt gtttgtatta 2340
ttttatggtt ttatggattt ttagggtggt attttgagtg ttgttttgaa agtagttttt 2400
ttttttattt attgtagagt gtatgaagga tatagaataa ttttatagaa attttttgtt 2460
gtttgttgtt aataaaatta gtatatggta aaggttaggt atttataatt tgaagttaaa 2520
gaagttttta ttttgtgatt tgtatataaa ttttttttta aataggtgga ttttttttat 2580
attttatttg gaattgttgg attgttattt ttgggagaag aatagattaa atttgttaat 2640
tttgtttttt gtatgtttga agaagtgttt tagagagtga atgtttagtt tgagttagtg 2700
agttagattt attgaattga aagttgtata gtatagtttt gttattttaa tatttttgta 2760
tttgaagtgt ttattgaatt taaaagtgat tattgttaat attttgtata ttgtgttaaa 2820
ttaattttaa taaattatat aattatatat attgtaaaat aaagattggt attttatttt 2880
ttgtttttta ttttgaagtt ttgttatttt gattatagtt ttttgtgtat atttttgtgt 2940
ttgttatgtt taataattga gttaatataa aataatttta ggttttattt tgattttttt 3000
tttatgtata ttttttattt gttttatagt aagttgatgt aaattggttt gttaataaga 3060
atgttaattg atgaaagtgg atagaattta tatatgaatt aaatgatgta taaaataaat 3120
ggttgttgaa atttggaaat gattgatagg tttgaatatg gttattttga attggttata 3180
gatttaggtt aaatggtaat ttattatttt aaaatggggt tgtgatttgg attgtttttt 3240
tttttttttt tttttttttt ttttttagta ttttatatat tagaattatt taaagaagga 3300
gttgttttta aataaggaaa tattaatggg taagttgatg gttattatag tatttttttg 3360
tatttgtttt ttttttaaaa agttaaaatg ggtataatta ttttttttta tttttttgtg 3420
atagaatttt tgttgtttag gttggagtgt agtagtatga ttttagttta attgtaattt 3480
ttgtttgttg ggtttaagta atttagttgt agttttttga gtagttggga ttatagttgt 3540
atattattat gtttggttaa tttttatgtt attttagtat agatagggtt ttattatgtt 3600
gaggagttgg agattagttt gattttgtga tttatttatt ttggtttttt aaagtgttgg 3660
gattataggt gtgagttatt atgtttggtt taaaatgagt ataattattt taggggttta 3720
tagttttggt gaaagtgagg tattttttat ttttaaattt tattttttag agatagggtt 3780
tttttttttt ttttaggttg gggtatagta gtgtaattat agtttattgt aattttaaat 3840
ttttgggttt aagtagtttt tttattttag ttttttgagt attagggttt ataggtattt 3900
gttattatgt ttattatgtt tagttgattt ttttaatttg gggataggta ttgttttttt 3960
taggttggtt tttggaggtg aagtaatttt ttggttttag ttttttaaag tattgggatt 4020
atagggagga gttattgtgt ttgatttgaa aataaggttt tttgatggaa attgagattt 4080
tttttaagta tgtttggaat gttttgtgta ataattattt taagaataga gatgttttta 4140
aggattgttg ttattgtatg tgggtaaatt gttgtgtata attggtttat tgagatattt 4200
aatagtggta ggattttagt agtggtgtaa attaatgttt tttaatagtt tttgtatggt 4260
agtatttaga taattttgtt atgtttaatt tatggatttt tggtaagttg gtattattgg 4320
ttttatttaa ggaatttttt aaaggttatt attgtaaagt tggagtttga atttttagtg 4380
tgttattaat tgatttatat gttagaaatt atagtattag aaattggtag atggggaatt 4440
gttaattgag agattttatt tagttagttt ttttttgttt tttattttat tatgtatata 4500
gttatgtgta tattattttt tgataatata attatttttt ttatatttgg ttataagatt 4560
aattttaggt gggttatgtt gtgtgagtat tatatgtttt tattgtgtta aaggtattgg 4620
gtgtattgtg ttgggtggtt tttatattta aagataagtt agtgtgtttg gatgtgtggg 4680
aaatgtatgt aatatttgta ataagggaga ttgggtgttg tgagaataat ggggttaata 4740
aattgggtgg ttattgatgg gttttgtaga gttttggtta gtttgatatt tgtaggttga 4800
ggatgtttta gtgaggttta tgtggaaagt gatgggtttg ggaatggttg gaagtagggt 4860
tatagatttg aagtatgtga atttatgtgg aaagttgagt ttgtttgtgt ttgtgtagtg 4920
tagtttagtg tgttggtgtg tagttttttt gtttgtttta gtggtgtagt ttttgtagtt 4980
gggttattgg aggggttgtt tagaaatttt atattttttg ggttagggaa ggtttgggag 5040
gatgttgagg tttgagattt tattaatttt gttttttgtt ttggtggttt ttttgttgtt 5100
gggagaaatt tgtttatttt agggtttttg ttataatttt ggattttagg agattttata 5160
gagttgtttt ttggtttagg tggtttttgt atttatttgt tttggtatgt taggagttgt 5220
gggtgattgg agtagtagta gtagtagttt tttttgtttt gtgttaaatt tttggttagt 5280
ttaaggtaag gagtgattgg gtggaggagt ttttttgagg ttagaggttg gtagtttatg 5340
gaggtttggg ggtatgtggg tggtggttat tatagtgaag ttgtgatttg gttggggtgt 5400
gagatttgag tgttttggga atttggtgag tttgggtgat tagaagtttt gattttttaa 5460
attggtttta ttgtaagatt ttgattgtgg atggtttagt tttgagaaag gttgaagtat 5520
tttatataat gttttttata gtgtttgata aatgatagtt tgtgtgtgag tttgttgata 5580

gaagttgtga gttgattttt ggttgttgaa tttaattttt taaattttgt taaggtggga 5640
taaaatagat taaattgaat atttatttta atttagggaa aataatatgt ttagtattaa 5700
aagagtgggt tgggtggtgg ttgatgtttg taattttagt attttgggaa attagggtgg 5760
gtggattatt tgaggttggg agttttatat tagtttggtt aatatggtga attttttatt 5820
tttatttttt atttttatta aaagtataaa aattatttgg gtgtggtggt atgtgattgt 5880
ggttttagtt atttaggagg ttgaggtggg aggatggtga gtttgggagg ttgaggttgt 5940
tgtgagttga gattgtgtta ttgtatttta gtttgggtga tatagtgaga ttttgattta 6000
aaataaataa ataaaaaaga tgtatgtttt ttaaattatt tattattttg ttagttttta 6060
tttatgataa agtattatta ttttttatat atgattgtaa tataatggta aagtaataaa 6120
tattaaattt aatggttatt ttttagttta ttttatgtga tttttttgtt gtatttaatg 6180
tttttaattt ttttattttt agaaatagag ttttgttatg ttgtttatgt tggttttaaa 6240
tttttggttt taagtgtttt tgttttggtt tgtttaagta ttgggattat aggtatgagt 6300
tattgttgtt tggtttatgt ttaatgtttt tgattggttt ttttttaaat ttttttaatt 6360
tggtttttttt gatttatttt gttttgtttt ttttgttatt ttttagagaa ttgttgtgtt 6420
tatttttgtt tttaaattat gggtatgtta ttaaagtttt tttttttagg ttatttttat 6480
ttagatgtga ttatttttat agtttagatt ttatttatat tttagattta ttttagattg 6540
ttttttgaag tttttattga tggattaatg gatattttag gttgattata tgattaatta 6600
aatttttttt tgtatttttt tttttttttt gagatagagt tttgttttgt tgtttaggtt 6660
ggagtgtagt ggtatggtta tgatttattg tagttttaaa tttttgggtt taagtgattt 6720
ttttatttta gttttttgag tagttgggat taaggtatgt gtttttatag atggttaatt 6780
taaaaaattt ttttgtagag atagtgtttt tttatgttgt ttagtttggt tttgaatttt 6840
taggtttaag tgattttttt attttagttt tttaaaatgt tgggattata ggtgtgagtt 6900
attgtattta gtttttaaat ttttaagtgt ttttttattt ttgtagttta tttattgtat 6960
tattagagtt atgtggaaaa tgattttatt atgttttttg ttatttaaat tagtagattt 7020
gtatttgtag gtaaaagatt aaatttttta gaaggatagt taaagtaatt gggattggtt 7080
tttaattttt atagatttat tttttattat ttaatattta ttttgttttt taataaaatt 7140
gagttatttg taattttttt aaagtaatat gaatgtttat attgttagtt taattttttt 7200
ttttattaga tgtttggtta tttatttatt tattagtaat gataatattt attaatttat 7260
atgtatttat tttgttttgg ttttttatta ggtagatggt atttttgatt tttataataa 7320
ttttgtagag tagatttttt ttttgttttt gttttataga tagaattaag gtttaaatag 7380
gtgaataaag gtttaaatta tgttagtagt tggggataat aatttgtgtt tttaatgaat 7440
ttaaggttta gtagagaaga tagatttaaa aattaataaa tatatattta agaaataatt 7500
gtatttgagg tatttataat ttttgaggta ggtataatta aagtatgggg ataaaagtgg 7560
gaaaagatat tttatagagt aagtgtattt tgaattggtt tttaagaata agtaggaaga 7620
agaatgttag ttaaagggaa atagttggag tgtatttttg agaaaaatat gtgtaaaggt 7680
atagaagaat tatgtaaaat tgttaataat tttgtttggt tagagtagga gagtgattta 7740
ggtagataaa tttgaatgta gatatagttg tgagaattag atttgggaga taaatttaat 7800
ttagagattt tttatattta atattaggtt tttggttttg gtgattggat agatggtagt 7860
agtatttttt gattttggga attttgtagg taaagtaaat tttagaggga agatgatgag 7920
ttttgttatg gatatgttga gtgtgaaaag tttgtgggat atttatatat ttatattaag 7980
ataattgaat ataggaaatt agagttaaga agagagaaat ttgagggaaa tatggatttg 8040
aaagttatta gtatgttggg attatagtag agaatatgaa gattatatag taaaatagag 8100
gttaaggaag taggaggaga attaagataa gtgaatgagt tttaagaaag ggtatatagt 8160
ttaaaagtag tttgagaata agaaatagtt ttttttattt ttagtttttg tatttttagt 8220
ttttggtaat tatttttttt tttagttttt taaagtttag aaatgttaat ggagtatatt 8280
ttatgttggt tttatatatg tttttatgat agtattttta tattttgttt atatttttgt 8340
aaatattttt tttattaaat tattttagtt tgagtatatt attgattttt gttggaattt 8400
atataatgaa gttatatttt attttatggt gagtatgggg agatatatag gaattgtaag 8460
aaagagagat attgattaga tttgtttttt agaaagatta ttggtgatga tttgaagaat 8520
agattagaga ggtgtaatat tggaagtaag gagaagaaga agtaggaaat ttgtataagg 8580
attaaagaag atataggttt aaaatgggtt gggaggagta gagagggatt taaattttag 8640
tgatgttaaa aaaaaagaat tttagtattt agttattgag tgttagggtt gatgaaggaa 8700
gaattgagaa tgttttttaa gtttatgatt tgagtgataa gggataagtt ggtataatta 8760
attatattgg taataaagaa ttaattttga gggaaagata gagtttattt ttggatattt 8820
agaatgttgg gaagatattt aaaggtttat gtttaagaga tagatatagt gatttggagt 8880
tatattgttt gatagaatat tttgtgataa tagaaatgtt ttatatttat gttgtttgat 8940
gtggtaatta gtagttgtat gtggtttttg agtatttgaa aatgttgtta gtgtgattga 9000
ggaatagaat ttaaattgta tttaattttg atttagttaa atatttatat atgttgtttt 9060
attgaataat gtaggtatag agttgagaat ggtttttgta tttttgaagg attaaagaat 9120
taagaatatt ttgtgatatt aaaattatat gaaatttaga atttaatatg tagaaataaa 9180
gttattttta attgaaatat agttattttt atttatttat gtattattta tgattgtttt 9240
tttatgatag tgttagagtt gagtaattgt gatagtgatt atatggttta taaaagttta 9300
aaatatttgt tatttggttt tttatttaaa agtttatggg ttgggtatga tggtttatgt 9360

ttataatttt agtattttgg gaggttgagg tgggtagatt atttgaggtt aggagtttat 9420
gattagtttg gttaatatga taaaattttg tttttattaa aaatataaaa aaaaaatagt 9480
taggtgtggt gggtatgttt ataatgttag ttgtttggga ggttgaggta ggaggattgt 9540
ttgaatttgg gaggtagagg ttgtagtgag ttgagattgt gttattgtat tttagtttgg 9600
gtaataagag tgaaatttta ttttaaaaaa aaaaaaaaaa agtttatgtt gaggtatggt 9660
ggtttatgtt tgtaatttta gtgttttggg aggttgaagt gggaggattg tttgaggtta 9720
ggtgtttgag attagtttgg gtaatgtagt aagattttgt ttttataaaa aaaaaattag 9780
ttgtgtgtgg tgaggtgtat ttatagttta tagttattta agaggttgaa gtaggaggtt 9840
atttgagttt atgagtttga ggttgtagtg agttgtgatt atattattgt attttagttt 9900
gggtgataaa gagagatttt gtttttataa gatagtaaaa tagattgggt atggtggttt 9960
ttgattataa ttttagtatt ttgggaggtt gagatgggaa gattatttga gtttaggagt 10020
ttgagattag tttgggtaat atggtaaaat tttattttta taaaaatata aaaaattagt 10080
ttggtatggt ggtatgagtt tatagtttta gttatttggg agggtgaggt aggaggattg 10140
tttgagttta ggagtttaag gttgtattta gttaagatta tattattgaa ttttagttta 10200
agttatagaa tgagagtttt atatatagat aaaaaggaaa aattaaaagt ttagtaattt 10260
ttggtttata gtttaaggag ttgttatagt tatatatgaa ggtttgagag ttgttagaaa 10320
atagatgtta atagaaataa agaggagaga tgagatgttt ggagatgtat aatgttgatt 10380
tgtgagggaa agtttaaaag tatgaagttt tggaggttaa tagttaagaa gtagaggaaa 10440
aaggagatgg taaaggaaat taaaagtggt ttaaagagaa tttaagatag agagttgtgg 10500
aaattaaaga gaataggtga atagagttta aatattttaa aattttttta gaatttttat 10560
gtgttaagag gttatgttag gttgtttatg tatattattt aaaaatttta tgataatttt 10620
gtaaaatagg taatagttta tattagtaga taagtattta aataaggaat tgaagttaat 10680
tgagtttaaa atgttatttg tataggggag agaaatttaa tgaagaaatt aatagaagaa 10740
ggttaaaaga ggtttaataa ttattatata aatgaaatat atgttttagt ttgtattttg 10800
ttgtttttaa aagaatattt gaaattgggt gattattaag aaagaaaata tattttttgg 10860
aagtttggag gttgggaagt ttaaggttaa aggaggtata tttagtgagg gtttttgtgt 10920
tggtggagtt tttgtagagt tttgtagtgg tatagggtat tatatggaga gggagttgag 10980
tatggtagtt taggtttttt attttttttt tataaaatta ttagttttat ttttgtgata 11040
atttattaat ttattaaatt atgaatggat taatttattt atgaaggtag agtttttatg 11100
ttttattatt ttttaaaggt tttatttttg ttttgttata ttttattggg gattaaattt 11160
taatgtaagt tttggagggg atagatattt atattttagt aatatatttg gtatatttga 11220
agattaaagg gtaatttatg atttagttta gagtagttgt agtgaatttg tagaggtaaa 11280
agttatatta gtatattttt gtatatttat taataatgtt attaattttg ttataatttg 11340
tttattttta aaaattattt ttatatgaaa ttttatagaa agtgaatata gtggttgggt 11400
gtggtgattt aggtttttaa ttttagtatt ttgggaggtt gaggtaggta gattatgagg 11460
ttaggaattt gagattagtt tagttaatat ggtgaaattt tatttttatt aaaaatataa 11520
aaatttatta ggtgtggtgg tgggtgtttg taattttagt tatttgggag gttgaggtag 11580
gaggattatt tgaatttggg aggtggatgt tgtagtgagt tgagattatg ttattatgtt 11640
ttagtttggg tgataagagt aagattttgt tttaaaaaag aaaaaaagaa aaaaaaggaa 11700
agtgaatatg gtataaatta taatgattaa tatattattg tataatttaa atttttaaga 11760
attgattgtt aatttttttaa atttagg 11787

<210> 386
<211> 11787
<212> DNA
<213> Artificial Sequence

<220>
<223> chemically treated genomic DNA (Homo sapiens)

<400> 386

tttaaatttg aaaaattaat agttaatttt taaagatttg aattatgtaa tgatgtatta 60
gttattataa tttatattat gtttattttt tttttttttt tttttttttt ttttgagata 120
gagttttgtt tttgttgttt aggttggagt gtagtggtat gattttggtt tattgtaatg 180
tttatttttt aagtttaagt gatttttttg ttttagtttt ttgagtagtt gggattatag 240
gtatttgtta ttatgtttgg tgaatttttg tatttttagt agagatgggg ttttattatg 300
ttggttgggt tggttttgaa tttttgattt tatgatttgt ttgttttggt tttttaaagt 360
gttgggatta gaggtttgag ttattgtatt taattattat gtttattttt tataaaattt 420
tatgtgaaaa tgatttttga aaataaatag attataataa aattaatgat attattgata 480
aatatataaa aatatgttaa tatagttttt gtttttataa gtttattgta attattttgg 540
ttaaaattat aaattatttt ttaattttta aatatattag atgtgttgtt aaggtatgaa 600
tatttgtttt ttttgaaatt tatattgaaa tttaattttt agtgaagtgt ggtaggatag 660

aggtggggtt tttaagaggt gatggggtat gagggttttg tttttatgaa tgaattaatt 720
tatttatgat ttaataggtt aatgggttat tatgagagta ggattggtgg ttttataaga 780
agaggatgag agatttgagt tattatgttt agtttttttt ttatgtgatg ttttgtatta 840
ttataggatt ttatagagat tttattagta taaagatttt tattaaatgt gttttttttg 900
attttggatt ttttagtttt tagattttta agaaatatgt tttttttttt gataattatt 960
tagttttaag tattttttta aaagtaatag aatatagatt aagatatatg ttttatttgt 1020
ataataatta ttagattttt tttaattttt ttttgttaat ttttttattg aatttttttt 1080
ttttatgtaa atggtatttt agatttaatt ggttttagtt ttttatttag gtatttattt 1140
attgatgtaa gttattattt attttatagg attgttataa aatttttaaa taatgtatat 1200
aaataattta gtatagtttt ttggtatata gaagttttaa aaaaatttta aaatgtttgg 1260
gttttgttta tttatttttt ttgattttta taatttttta ttttgaattt tttttaaatt 1320
atttttagtt ttttttgtta tttttttttt tttttgtttt ttaattgttg gtttttaggg 1380
ttttatattt ttaagttttt ttttataaat taatattatg tatttttagg tattttattt 1440
tttttttttg tttttattaa tatttatttt ttgataattt ttaagttttt gtatatagtt 1500
gtaatgattt tttaaattgt aaattagggg ttgttaaatt tttaattttt tttttttgtt 1560
tgtatgtgag atttttgttt tgtagtttag gttggaattt agtggtgtga ttttggttaa 1620
atgtagtttt gaatttttgg gtttaagtaa tttttttatt ttattttttt gagtagttgg 1680
gattataggt ttatgttatt atgttaggtt aattttttgt atttttgtag agatggggtt 1740
ttgttatgtt gtttaggttg gttttaaatt tttgggttta agtgattttt ttattttagt 1800
tttttaaaat gttgggatta taattgggag ttattatgtt tagtttattt tattgtttta 1860
taaagataag gttttttttt gttgtttaga ttggagtgta gtagtgtgat tatagtttat 1920
tgtagtttta agtttatggg tttaagtaat tttttgtttt agttttttga gtaattatgg 1980
attataggtg tattttatta tatatagtta attttttttt tatagagata gggttttgtt 2040
atattgttta ggttgatttt aaatatttgg ttttaagtaa tttttttatt ttagtttttt 2100
aaagtattga gattataagt ataagttatt atgttttagt ataaattttt tttttttttt 2160
tttgagatgg agttttattt ttgttgttta ggttagagtg taatggtgta attttagttt 2220
attgtagttt ttgtttttta ggtttaagta atttttttgt tttagttttt taagtagttg 2280
gtattatagg tatgtttatt atatttggtt attttttttt tgtattttta atagagatag 2340
ggttttgtta tgttggttag attggttatg aatttttggt tttaggtgat ttgtttattt 2400
tagtttttta aagtgttggg attataggta tgagttatta tgtttagttt ataaattttt 2460
aaataaagga ttagatggta aatattttag gtttttgtgg gttatataat tattgttata 2520
attatttagt tttgatatta ttatagaaaa gtagttatag ataatatata aataaatgag 2580
aatggttatg ttttaattaa aaatggtttt atttttatat attgaatttt aaattttata 2640
taattttaat attataaaat atttttgatt ttttaatttt ttaaaagtgt aaaaattgtt 2700
tttagtttta tatttgtatt gtttaataaa gtagtatatg tgagtattta attaaattaa 2760
aattaaatat aatttaaatt ttgtttttta gttatattag taatattttt aaatgtttaa 2820
aagttatata tggttattag ttattatatt ggatagtata agtatgaaat gtttttatta 2880
ttatagaata ttttattaga taatgtagtt ttaggttatt atatttattt tttgggtata 2940
aatttttagg tattttttta gtattttaaa tgtttaaaag tgaattttat ttttttttta 3000
aaattaattt tttattatta gtatggttaa ttgtattagt ttgttttttg ttatttaagt 3060
tataagttta ggagatattt ttaatttttt ttttattaat tttaatattt agtgattgag 3120
tgttagaatt tttttttttt aatattatta aaatttaaat ttttttttat tttttttagt 3180
ttattttaag tttgtatttt ttttggtttt tgtgtaagtt ttttattttt tttttttttt 3240
gtttttagta ttatattttt ttaatttatt ttttgaatta ttattaataa tttttttgaa 3300
aagtaaattt gattagtatt tttttttttt ataattttta tgtgtttttt tatatttatt 3360
atagggtaaa atataatttt attatatggg ttttagtagg aattaatgat atatttaaat 3420
tgggataatt tgataaagag aatatttata aaggtgtgga tagggtgtag gggtgttatt 3480
ataaagatgt gtgtagaatt agtataaaat gtgttttatt agtattttta ggttttaggg 3540
agttaaggaa gagagtggtt gttagaaatt ggagatgtgg aaattggaga tggagagggt 3600
tgttttttgt ttttagatta tttttagatt atatattttt ttttgaaatt tatttatttg 3660
ttttaatttt tttttattt ttttgatttt tatttgttta tatagtttt atattttttg 3720
ttgtggtttt aatatattgg taatttttaa atttatattt tttttaagtt tttttttttt 3780
tagttttagt tttttatatt taattatttt aatataaata tatggatatt ttataggttt 3840
tttatattta gtatgtttat aatagagttt attatttttt ttttaaaatt tgttttgttt 3900
ataaaatttt tagaattaga aaatattatt attatttatt tagttattaa aattagagat 3960
ttggtgttga atatgggggg tttttgggtt aaatttgttt tttagattta atttttataa 4020
ttatatttat atttaagttt atttatttag attatttttt tgttttagtt aagtaaaatt 4080
attagtagtt ttatatgatt tttttgtgtt tttgtatgtg ttttttttaa gaatgtgttt 4140
tagttgtttt tttttggttg gtattttttt ttttatttat ttttgaagat tagtttaagg 4200
tgtatttatt ttgtgaaatg ttttttttta tttttgtttt tatattttaa ttatatttat 4260
tttaaaaatt ataaatattt taaatatagt tattttttaa atgtgtattt gttgattttt 4320
aagtttgttt tttttattga attttaagtt tattgagagt atagattatt atttttagtt 4380
gttaatataa tttgaatttt tgtttgttta tttgggtttt ggttttattt gtaaaatagg 4440

aataaaaaaa aaatttattt tgtaaaatta ttgtgaggat taaagatatt gtttgtttag 4500
taagaagtta gaataaagta ggtgtatata aattagtgaa tgttattatt attgataaat 4560
aagtgggtga ttaagtattt gatgaagaga gggattagat tgataatgtg aatatttatg 4620
ttgttttaaa ggaattgtaa gtagtttagt tttgttagaa agtagggtaa atattaaatg 4680
atgagggata aatttgtaaa gattagaaat tgattttaat tgttttggtt atttttttag 4740
agagtttgat tttttatttg tagatatagg tttattgatt taagtggtaa gggatatgat 4800
aagattattt tttatataat tttggtggta tagtaagtaa gttataagga tggaagaata 4860
tttaagagtt tgagggttag gtgtagtggt ttatatttgt aattttagta ttttgggagg 4920
ttgaggtagg aggattgttt gagtttagaa gtttgagatt aggttgggta atgtaggaaa 4980
atattgtttt tataaaaaaa ttttttaaat tagttatttg taggggtata tgttttggtt 5040
ttagttattt gggaggttga ggtgggagga ttgtttgagt ttaggaattt gaggttgtag 5100
tgagttatga ttatgttatt gtattttagt ttgggtaata gagtaagatt ttgttttaaa 5160
aaaaaaaaaa aagtgtagag aggaatttaa ttaattatat agttagtttg aggtatttat 5220
tagtttatta ataaaaattt tagaagatag tttgaagtaa gtttggagtg tagatgagat 5280
ttgagttgtg gagatagtta tatttaggta gaagtggttt aggaaaaaaa gttttaatga 5340
tatgtttata atttgggaat aaaggtgggt atagtaattt tttgggaggt agtagaaaaa 5400
taggggtaag tggaattaag gaagttaaat taaaagagtt taagagaaga ttagttaaga 5460
atattaaata taggttaggt agtagtggtt tatgtttgta attttaatgt ttgggtagat 5520
tgaggtagga gtatttgaga ttaggagttt gagattagta tgggtagtat agtgagattt 5580
tgtttttaaa aataaagaaa ttaagaatat taaatatagt agaaaggtta tatgagatga 5640
attgaaaaat gattattgga tttggtattt gttattttat tattgtgttg taattgtgtg 5700
tgaaaaatgg tgatgttttg ttatgaatag agattaataa aataataaat gatttgaagg 5760
atatgtattt tttttgtttg tttgttttga gttagggttt tattgtgtta tttaggttga 5820
aatgtagtgg tgtaatttta gtttatggta gttttgattt tttggatttg ttattttttt 5880
attttaattt tttgagtagt tgggattata gttatgtgtt attatattta gataattttt 5940
gtatttttag tagagatagg ggatgggggt gggaggttta ttatgttggt taggttggtg 6000
tggaattttt ggttttaagt gatttgttta ttttggtttt ttaaagtgtt gggattatag 6060
gtgttagtta ttgtttagtt tattttttta atattaaata tattattttt tttgaattag 6120
atgaaatgtt taatttaatt tattttattt tattttagta ggatttggag aattaaattt 6180
agtagttaaa gattagttta taatttttat tagtaaattt atgtataaat tgttatttgt 6240
tagatattgt gagggatgtt atatgggata ttttagtttt ttttagaatt gagttattta 6300
tagttaaggt tttgtaatag aattaatttg aaaagttaag gtttttaatt atttagattt 6360
attaaatttt tagggtattt agattttatg ttttaattaa attataattt tattgtggta 6420
attattattt atgtgttttt gagtttttat gaattgttgg tttttagttt taaggagatt 6480
tttttattta attatttttt gttttgagtt ggttgggagt ttggtgtggg gtagggaagg 6540
ttgttgttgt tgttgttttg gttgtttgta gtttttgatg tgttaggata ggtggatgta 6600
gagattattt ggattgaagg gtggttttgt ggagtttttt ggagtttgaa attgtagtgg 6660
ggattttgag gtgagtgggt tttttttgat gatggggaaa ttgttggggt agaaggtgag 6720
gttgatgaga ttttaggttt tagtattttt ttaaattttt tttgatttga gaagtatgag 6780
gtttttgagt gattttttta gtagtttaat tatagaagtt gtgttgttga aatgggtggg 6840
agaattgtgt gttgatgtat taagttgtat tgtgtaggtg taggtaggtt tagtttttta 6900
tatggattta tgtattttag gtttatgatt ttgtttttag ttatttttaa atttattatt 6960
ttttatatga attttattgg aatattttta gtttgtaggt attagattga ttagaatttt 7020
gtaaggttta ttaatgatta tttgatttgt tagttttatt atttttatag tatttaattt 7080
tttttattgt aaatgttata tgtatttttt atatgtttaa gtatattagt ttatttttaa 7140
atgtagaaat tatttaatat agtatattta gtgtttttga tataatgaga atgtgtaatg 7200
tttatatagt ataatttatt taaggttgat tttgtgatta aatataggaa agatagttat 7260
attattaaaa gatagtatgt atatggttgt gtgtatagta gagtaggaaa tagggaaaga 7320
ttgattgggt aagatttttt agttaataat tttttattta ttaattttta gtattgtggt 7380
ttttaatata taaattagtt aatggtatat tagaagttta aattttagtt ttgtagtggt 7440
gatttttaga aaatttttta aataaggtta atagtattaa tttattagag atttatggat 7500
taaatatgat gaaattattt ggatattgtt atatagaagt tattagaaga tattaattta 7560
tattattatt gaaattttgt tattgttgag tattttaata aattagttat gtatagtggt 7620
ttatttatat gtagtgataa tagtttttaa gaatattttt gtttttggaa tgattattgt 7680
atagggtatt ttaaatatat ttgagaaaaa ttttaatttt tattaaaaaa ttttattttt 7740
agattgggta tgatggtttt tttttgtaat tttagtattt tgggagattg aggttagagg 7800
attattttat ttttggagat tagtttgggg aagatagtgt ttgtttttaa aattaagaaa 7860
ttagttgggt atagtgggta tggtggtagg tatttgtaga ttttggtatt taggaggttg 7920
aggtggaagg attgtttgag tttaggagtt taaggttata gtgagttgtg attgtattat 7980
tatgttttag tttggggaag aaaggaagat tttgttttta aaaaataaaa tttaaaaata 8040
gagaatattt tatttttatt aaaattgtaa atttttagag tgattgtatt tattttaggt 8100
taggtgtggt ggtttatgtt tgtaatttta gtattttggg aggttgaggt gggtggatta 8160
taaggttagg ttggttttta gttttttagt atggtgaaat tttgtttgta ttaaagtaat 8220

ataaaaatta gttgggtatg gtggtgtgtg gttgtagttt tagttatttg agaggttgtg 8280
gttgaattgt ttaaatttgg tagatggagg ttgtagttaa gttgagattg tgttgttgtg 8340
ttttagtttg ggtaatagag attttgttat aaaaaaataa aaaagagtga ttgtatttat 8400
tttagttttt taagaaaaag ataggtgtaa aaaaatattg tggtgattat taatttattt 8460
gttagtgttt ttttatttga agataatttt ttttttgggt aattttgata tataaaatgt 8520
taaaaaaaaa aaaaaaaaaa aaaaaaaaaa ggatagtttg aattataatt ttattttaag 8580
atggtagatt gttatttaat ttaagtttgt gattaattta gaataattat atttaaattt 8640
attaattatt tttaaatttt aatagttatt tattttgtgt attatttaat ttatgtatgg 8700
gttttattta tttttattag ttaatatttt tgttgataaa ttaatttata ttaatttgtt 8760
atagaataga tgaaaggtat atatggggga aaaattaagt agaaatttag agttatttta 8820
tgttagttta gttattgaat ataatagata tagaagtata tataaagaat tgtagttaga 8880
ataataggat tttagaataa aaagtagaaa ataaaatatt ggtttttatt ttataatatg 8940
tataattata taatttatta aaattaattt aatataatat ataaaatatt aatagtagtt 9000
atttttagat ttgataagta ttttaaatat agaaatgtta aaatggtaaa attatattat 9060
gtaattttta atttaatgaa tttagtttat tagtttaggt tgaatattta ttttttgaag 9120
tattttttta ggtatgtaaa agataggatt aataggttta atttgttttt tttttaaaag 9180
tgataattta gtaattttaa ataaggtatg aaaaggattt atttatttaa aaaagaattt 9240
atatatagat tataaaatga aaattttttt aattttaaat tatgaatatt tgatttttat 9300
tatatattaa ttttgttagt aataagtgat aagaaatttt tgtaagattg ttttatattt 9360
tttatatatt ttataataaa tagaaaaaaa aattgttttt aaagtaatat ttggaatgtt 9420
attttggaaa tttatggaat tatgaagtaa tgtaaatgtt gaaataattt atagaaatat 9480
gttagaaata ttatttgtta tattttttgg tttgtttgta aatttttttg tttttttatt 9540
ttgatatgtt aaaatgaaat tatgtagttt tttttattta atttaatgaa gtttttttaat 9600
tatttttagg gaagttagga tttattatga gtagtatata tttggtaatt aggagggaaa 9660
aaagttgtta taattttatt tttttttatt tttttaattt tgagaataat ttaaaagtgg 9720
gttaagtttt atttttaggt aatttgatgt ttaaagtgtt aaattatgtg gaaggatgaa 9780
atttagggat atattgatat gtagggtatg aaatatttgt ttagaatgaa agataattaa 9840
ttaatgggta agaagagatt aaattttaat ttataggtaa ttatgttaaa tattttagta 9900
tttattgata agtatgtatt tgttttgttt ttatttgtaa tgaatgttta atttagtttt 9960
ttaattaaga agttagtagt ataattatga aatttatggg tatagttttg tttttttata 10020
agtattgttt aggggggtat atatttgttt aaaattaatt aaatttttgt tttttaaaat 10080
atggtgatag aagtttataa agaaaatttt atttaaggtt gattttattt taatgagatt 10140
tttgagtaat ttatagattt attagaagtt gttttagatt gtgtgtaagt gtaggttttg 10200
gtgtttttta tggttattta tttttttgtt gatagttttt taggtttttt attttatttt 10260
attgtagata gttattgtgg gtgttttttt atttgttgtt atttagaaat attaaggttt 10320
aaaaataaaa atataggttg agtatagtgg tttatgtttg taattttagt attttgggag 10380
gttgaggtgg gtagattatt tgaggttagg agtttaagat tagtttgatg aatatggtga 10440
tattttattt ttattaaaaa tataaaattg gatgggtgtg gtggtgtatg attgtaattt 10500
tagttattgt attattatgt tttatttaga gggtaggggt atagtaagtt ataatttttg 10560
agttaaattt atagtgtagg aatagttttt atatttataa attatttaaa tatagttttg 10620
ggttaggtat ggtggtttat gtttataatt ttagtatttt gggaggttga tgtaggtaga 10680
ttatgaggtt aggagtttga gattagtttg gttaatatgg tgaaattttg tttttaataa 10740
aaatataaaa attagttgtg tgtttgtagt tttagttatt tgggaggttg aggtagaatt 10800
gtttgaattt gggaggtgga ggttgtagtg aattaagatg gtgttattgt attttagttt 10860
gggtaataga gtaagatttt atttgatata ggaaatataa gtatatttat ttataataat 10920
tgtaattgga atatagttat atttatttat tttttattat ttatggttgt atttgtttta 10980
taggataatt atatagttgt aaatgagtat ttattatttg ttttttatag atttgataag 11040
agagatttat tatgtggagg aaaatttaat aatttttttt ttaaattaaa ttatttgggt 11100
attttttaaa taagaagatt gtaaataaaa attttgtagt ttaattttta aaaaatttaa 11160
tttgttaaaa ttaattgttt ttaaatatgg agatttttgt ataagattgt ttggaaatgg 11220
atattaggat taatgagtat tttaatttat gggggtttat tttgttaagg ggagtttttt 11280
agtaggatat attattgaaa atttataata gggaaaggta ataaataagt ttatggttaa 11340
tttataatat agaaataggt aagataaagt agtagtattt atttaaggtt ttaaagttag 11400
tagaatatgg tttattaaat aatatttttt ttggtttttt attgagtttg tttgagggta 11460
attgttgttt ataaagttat tagttaagta aattagaatt tatttgatgt aattgatttg 11520
taattgttag aaattttgtg taataataga tttaaaatta tagatataaa gtttatgtgt 11580
ataataattt tttgatatta aaagtgataa aatttaaatt attaaaatat tattttatat 11640
tttaagtttt tttttttgat ttgatattta aaattttaaa tatgtattaa tattatatat 11700
ttgaaaaaat ttaagtatat tattattagt atgtaatttt gtattgaaat attattgtta 11760
aaattttttt atggttagta ttttata 11787

<210> 387
<211> 2455

<212> DNA
<213> Artificial Sequence

<220>
<223> chemically treated genomic DNA (Homo sapiens)

<400> 387

ggagtgtaag aatatagaat taaaatagag tttgaaattt aaagaaaggg agagatttgg 60
gggaggagtg gggtggagtg atgtgatgtg ttgttggaaa ttagtagttg gtggtttttt 120
tttgtgtttt tttttttgtg ggtttttttt tgtttgtggg agggtttttt tttttttttt 180
tgatagaaag gttatttttg gtgtttgttt ttttgaattg taatattttg taagggtatg 240
attttatgtt tttgtgtggg tgtgaatttt tttatggtga tttttaaaat ttgtatatag 300
gatttttttt tattgagggg aggggattaa aataatttta ttttttaggg tttttttttg 360
ttttaattgg tttgtgttta agagaagttt taggtaaatg gggttagttt gaagattaaa 420
taggtttggt agtttttttt ggtttttttt ttttttttta tagttttata gttatagttg 480
ttttgatatt aatattgatt ttggttggtt ggtatgatta tttatagggt attgtgtttt 540
aatttattag gtgataggta atgttgtttt tttttggaat tatttagtag agttagggtt 600
gtatttttaa attttgtaat agaggttttt ttttatttta tttttttgtt tttgtatttt 660
ttttatttta gtagtttttt tttttttttt tgggtttttt tttttatttt tttttttttt 720
tgggtttggt aaattagttt ttaatttttt tatattttag attggaaggt gggttttttt 780
ttgatatttt ttagagttgt tattaatttt ttttaagttt ttatagtttt attgtttaat 840
agatttgtta ggggtaatta ttaatttagt ttttaatttt gtttttttat tttttttgtt 900
ggaggggatt ttttaattat tggttagtat agttaggtta ttttattttt attatttttt 960
ttaatttttt gggttggggg gttggggagg aattttttta ttttagggta ttaggaataa 1020
agttggggag gatggtgtat tttaaaggat tatatatata tatatatata tttttttttt 1080
ttttttttta taattttatt tttgtaatat atatatatat atatatatat atatatatat 1140
atatatatat agatgtataa ataagtttta tggagtagtg attttattat gtttattgtt 1200
ttgagtttaa tttttggttt aaaataggta ttaaatagtt gttgaatgta tgaatgatag 1260
atattttttt gtttttaggg gtgtgtagaa gtgtgaaggg gtatgggtat gttttagtag 1320
gggtgtgagt gttttgatta gaattatttt tttttgttag aatttgatgt aatttgaatg 1380
tttttatttt tgtttgaagg gtttaaataa taaattaggt tttgttgtgt tattatgggg 1440
gtggttatat tttgtattta ggaaataggt atggtagggt tgagatagaa gttttgtttg 1500
tttttgttta tttatttgaa atattgttta tttaggtttt attttgtttg ttaggtattg 1560
ttttaagttt tgtataaata ttaatttaga gggtataaat attaatttaa gagttgttgt 1620
aggaaaaaaa ataagtgttt ttggtttttt aagtttggtt ttttttttaa aatttttgta 1680
atggttttaa atttttttta gggattggga ttatggattt tggtttgatt tttttgtggg 1740
ttttttattg tgttaattaa attttagaaa tagtgagtgt tagaggtttg gttgttaagt 1800
aatggtagag ggtgggaagt ttgaatgttt tggatttgtt ttgaaggtaa ataggttaat 1860
tagtgtttag attttttagt tatggtagtt tgtttttttt ttttgttttg ttggattttt 1920
aggttggatt tgggtttttt taattaatag tggttaggag ggtggggtgt gtgtgtgtgt 1980
attttgttta tgtgttggtg tgtttttttt gtaggtttgt ggtggttggt tagtggggtg 2040
tttttttatt tgtagtgatt taggttattg aaaaggtggg aaaatgttgt gatggtggta 2100
gttgggggag gaggaagata agtgtgtgag gttggggttt tggtgtgtgg ttggtagagg 2160
tagagatata agatgtgtat gatttgtttt atagggtttt tagatttttt ttttttaaag 2220
ggtaattttt gtgtgatagg aatgagggtg gggtgtgtgg agttttttat aatttgtatt 2280
ttagttaaat atttgagaat ttattttttg tgtttatagt tttttatgtt ttttagtttt 2340
gttttgtagt tttgtagtag aagtatttag tgttttgtat tttgtgtgtt attttatttt 2400
ggtttttttt gtgaattgtt gtttttgaat tgtttttatt ttttgtattt gtaga 2455

<210> 388
<211> 2455
<212> DNA
<213> Artificial Sequence

<220>
<223> chemically treated genomic DNA (Homo sapiens)

<400> 388

tttgtggatg taaaaagtga gggtggtttg gaagtaatga tttatagagg aggttggggt 60
agggtggtgt gtagaatgta aagtattaag tatttttgtt atagggttgt ggggtagggt 120
tgaggggtgt ggagagttgt ggatatagga ggtgaatttt tgggtattta attaaagtat 180

agattgtggg aaattttatg tgttttattt ttatttttgt tatgtggagg ttattttttg 240
gaaggaaggg gtttgagggt tttgtggggt gagttgtgta tgttttatgt ttttgttttt 300
gttaattatg tgttaggatt ttagttttat gtgtttattt tttttttttt ttagttgttg 360
ttattgtagt gtttttttgt ttttttagta atttgagttg ttataggtgg gagaatgttt 420
tgttgattga ttgttatgag tttgtaaaag gagtgtgttg gtgtgtgagt gaggtgtgtg 480
tgtatgtgtt ttgttttttt agttgttgtt gattggagag gtttggattt agtttagaga 540
tttgataggg tgaggaggag aagtggattg ttgtagttga agagtttgga tgttgattgg 600
tttatttgtt tttggggtgg gtttagaatg tttaaatttt ttgttttttg ttgttgttta 660
gtagttgggt ttttagtatt tattgttttt gggatttgat tggtgtagtg ggaagtttgt 720
gagaaggttg gattagggtt tgtagtttta gtttttggaa ggggtttggg attgttgtgg 780
gggttttgag gggaggttta aatttaaaga gttagaggtg tttatttttt tttttgtaat 840
aatttttaag ttaatatttg tattttttga gttaatattt atatagagtt tagaatagtg 900
tttggtaaat aaagtaagat ttaaatgagt ggtgttttaa ataaataagt ggaaataagt 960
aggatttttg ttttagtttt attgtgtttg ttttttgggt atagggtatg attattttta 1020
taatggtatg atagggttta atttattatt taaatttttt aagtagaggt ggaagtattt 1080
gaattatatt aaattttggt agagagaagt agttttgatt agaatattta tatttttatt 1140
gggatatgtt tatatttttt tgtattttta tatatttttg aagatagaga ggtatttatt 1200
atttatgtat ttggtaatta tttagtgttt attttgggtt aggggttgga tttaaagtgg 1260
tgaatataat gaagttattg ttttataaag tttatttgtg tgtttgtgtg tgtgtgtgtg 1320
tgtgtgtgtg tgtgtgtgtg tgtgtgtgtt gtgggggtgg ggttatgagg gagaaagaaa 1380
aaaaatatat atatatatat atataatttt tttaaatgta ttattttttt tagttttgtt 1440
tttagtattt tgagatgggg agattttttt ttagtttttt aatttaagaa gttaggaaag 1500
atggtggggg tgagatgatt tagttgtgtt aattagtaat tggaaaattt tttttagtaa 1560
gaaaggtggg ggaatagagt taagggttgg gttaatggtt atttttggta aatttgttga 1620
gtaatggagt tatagaaatt tggaggaggt tggtgatagt tttggggagt gttagggagg 1680
gatttatttt ttaatttggg gtgtgaagag attagggatt agtttattaa gtttaggaag 1740
gggagggagt ggaaagagaa gtttagagag ggaaagagga gttattgaga taggagaaat 1800
gtagggatag ggaggtgaga tggagggaaa tttttgttgt aggatttggg ggtatagttt 1860
tggttttgtt ggatggtttt aggagagggt agtgttgttt gttatttggt aaattaaggt 1920
atgatatttt gtgggtagtt atgttagtta gttaaagtta atattgatat taaggtagtt 1980
gtagttataa agttgtagga gagaaaagag aggttgggag aggttgttaa atttgtttga 2040
tttttaagtt ggttttattt atttaaggtt ttttttggat atagattagt tggaatagga 2100
gaggattttg agaagtagag ttgttttgat tttttttttt taatgggaag gggttttgtg 2160
tgtagatttt gagggttatt atgagggaat ttatatttat atagaggtat ggaattatat 2220
ttttatagga tgttatagtt taagggaatg aatattaaag atagtttttt tgttgggagg 2280
agagaaaaag gtttttttat aagtaggaga aaatttatag aagaggaagt ataagaggaa 2340
attattaatt gttggttttt agtagtatat tatgttattt tattttattt tttttttaag 2400
tttttttttt tttttaagtt ttaagttttg ttttagtttt gtgtttttgt atttt 2455

<210> 389
<211> 28
<212> DNA
<213> Artificial Sequence

<220>
<223> Detection primer for MDR1

<400> 389

taagtatgtt gaagaaagat tattgtag 28

<210> 390
<211> 28
<212> DNA
<213> Artificial Sequence

<220>
<223> Detection primer for MDR1

<400> 390

taaaaactat cccataataa ctcccaac 28

<210> 391
<211> 20
<212> DNA
<213> Artificial Sequence

<220>
<223> Detection primer for APOC2

<400> 391

atgagtagaa gaggtgatat                                                    20

<210> 392
<211> 20
<212> DNA
<213> Artificial Sequence

<220>
<223> Detection primer for APOC2

<400> 392

ccctaaatcc ctttcttacc                                                    20

<210> 393
<211> 23
<212> DNA
<213> Artificial Sequence

<220>
<223> Detection primer for CACNA1G

<400> 393

gggatttaag agaaattgag gta                                                23

<210> 394
<211> 20
<212> DNA
<213> Artificial Sequence

<220>
<223> Detection primer for CACNA1G

<400> 394

aaaccccaaa catcctttat                                                    20

<210> 395
<211> 20
<212> DNA
<213> Artificial Sequence

<220>
<223> Detection primer for EGR4

<400> 395

agggggattg agtgttaagt                                                    20

<210> 396
<211> 20
<212> DNA

<213> Artificial Sequence

<220>
<223> Detection primer for EGR4

<400> 396

cccaaacata aacacaaaat                                                    20

<210> 397
<211> 20
<212> DNA
<213> Artificial Sequence

<220>
<223> Detection primer for AR

<400> 397

gtagtagtag tagtaagaga                                                    20

<210> 398
<211> 20
<212> DNA
<213> Artificial Sequence

<220>
<223> Detection primer for AR

<400> 398
   accccctaaa taattatcct                                                    20

<210> 399
<211> 23
<212> DNA
<213> Artificial Sequence

<220>
<223> Detection primer for RB1

<400> 399

tttaagtttg tttttgtttt ggt                                                23

<210> 400
<211> 22
<212> DNA
<213> Artificial Sequence

<220>
<223> Detection primer for RB1

<400> 400

tcctactcta aatcctcctc aa                                                 22

<210> 401
<211> 22
<212> DNA
<213> Artificial Sequence

<220>

<223> Detection primer for GPIb beta

<400> 401

ggtgatagga gaataatgtt gg                                                22

<210> 402
<211> 20
<212> DNA
<213> Artificial Sequence

<220>
<223> Detection primer for GPIb beta

<400> 402

tctcccaact acaaccaaac                                                   20

<210> 403
<211> 24
<212> DNA
<213> Artificial Sequence

<220>
<223> Detection primer for MYOD1

<400> 403

attaggggta tagaggagta ttga                                              24

<210> 404
<211> 22
<212> DNA
<213> Artificial Sequence

<220>
<223> Detection primer for MYOD1

<400> 404

cttacaaacc cacaataaac aa                                                22

<210> 405
<211> 20
<212> DNA
<213> Artificial Sequence

<220>
<223> Detection primer for WT1

<400> 405

aaagggaaat taagtgttgt                                                   20

<210> 406
<211> 19
<212> DNA
<213> Artificial Sequence

<220>
<223> Detection primer for WT1

<400> 406

taactaccct caacttccc                                                    19

<210> 407
<211> 22
<212> DNA
<213> Artificial Sequence

<220>
<223> Detection primer for HLA-F

<400> 407

ttgttgtttt taggggtttt gg                                                 22

<210> 408
<211> 22
<212> DNA
<213> Artificial Sequence

<220>
<223> Detection primer for HLA-F

<400> 408

tccttcccat tctccaaata tc                                                 22

<210> 409
<211> 24
<212> DNA
<213> Artificial Sequence

<220>
<223> Detection primer for ELK1

<400> 409

aagtgtttta gtttttaatg ggta                                               24

<210> 410
<211> 20
<212> DNA
<213> Artificial Sequence

<220>
<223> Detection primer for ELK1

<400> 410

caaacccaaa actcacctat                                                    20

<210> 411
<211> 22
<212> DNA
<213> Artificial Sequence

<220>
<223> Detection primer for APC

<400> 411

tcaactacca tcaacttcct ta                                                 22

<210> 412
<211> 25
<212> DNA
<213> Artificial Sequence

<220>
<223> Detection primer for APC

<400> 412

aatttatttt tagtgttgta gtggg                                          25

<210> 413
<211> 22
<212> DNA
<213> Artificial Sequence

<220>
<223> Detection primer for BCL2

<400> 413

gtattttatg ttaaggggga aa                                             22

<210> 414
<211> 18
<212> DNA
<213> Artificial Sequence

<220>
<223> Detection primer for BCL2

<400> 414

aaaaaccaca atcctccc                                                  18

<210> 415
<211> 20
<212> DNA
<213> Artificial Sequence

<220>
<223> Detection primer for CALCA

<400> 415

gttttggaag tatgagggtg                                                20

<210> 416
<211> 21
<212> DNA
<213> Artificial Sequence

<220>
<223> Detection primer for CALCA

<400> 416

ccaaattcta aaccaatttc c                                              21

<210> 417
<211> 19
<212> DNA

<213> Artificial Sequence

<220>
<223> Detection primer for CDH1

<400> 417

gaggttgggg ttagaggat                                                    19

<210> 418
<211> 25
<212> DNA
<213> Artificial Sequence

<220>
<223> Detection primer for CDH1

<400> 418

caaactcaca aatactttac aattc                                             25

<210> 419
<211> 21
<212> DNA
<213> Artificial Sequence

<220>
<223> Detection primer for CDKN1A

<400> 419

ggattagtgg gaatagaggt g                                                 21

<210> 420
<211> 21
<212> DNA
<213> Artificial Sequence

<220>
<223> Detection primer for CDKN1A

<400> 420

aaacccaaac tcctaactac c                                                 21

<210> 421
<211> 19
<212> DNA
<213> Artificial Sequence

<220>
<223> Detection primer for CDKN1B (p27 Kip1)

<400> 421

gtggggaggt agttgaaga                                                    19

<210> 422
<211> 20
<212> DNA
<213> Artificial Sequence

<220>

<223> Detection primer for CDKN1B (p27 Kip1)

<400> 422

atacacccct aacccaaaat                                                    20

<210> 423
<211> 20
<212> DNA
<213> Artificial Sequence

<220>
<223> Detection primer for CDKN2a

<400> 423

ttgaaaatta agggttgagg                                                    20

<210> 424
<211> 20
<212> DNA
<213> Artificial Sequence

<220>
<223> Detection primer for CDKN2a

<400> 424

caccctctaa taaccaacca                                                    20

<210> 425
<211> 20
<212> DNA
<213> Artificial Sequence

<220>
<223> Detection primer for CDKN2a

<400> 425

ggggttggtt ggttattaga                                                    20

<210> 426
<211> 20
<212> DNA
<213> Artificial Sequence

<220>
<223> Detection primer for CDKN2a

<400> 426

aaccctctac ccacctaaat                                                    20

<210> 427
<211> 22
<212> DNA
<213> Artificial Sequence

<220>
<223> Detection primer for CDKN2B

<400> 427

ggttggttga aggaatagaa at                                                22

<210> 428
<211> 22
<212> DNA
<213> Artificial Sequence

<220>
<223> Detection primer for CDKN2B

<400> 428

cccactaaac atacccttat tc                                                22

<210> 429
<211> 21
<212> DNA
<213> Artificial Sequence

<220>
<223> Detection primer for CD44

<400> 429

gaaaggagag gttaaaggtt g                                                 21

<210> 430
<211> 21
<212> DNA
<213> Artificial Sequence

<220>
<223> Detection primer for CD44

<400> 430

aactcactta actccaatcc c                                                 21

<210> 431
<211> 22
<212> DNA
<213> Artificial Sequence

<220>
<223> Detection primer for CSPG2

<400> 431

ggataggagt tgggattaag at                                                22

<210> 432
<211> 22
<212> DNA
<213> Artificial Sequence

<220>
<223> Detection primer for CSPG2

<400> 432

aaatcttttt caacaccaaa at                                                22

<210> 433
<211> 22
<212> DNA
<213> Artificial Sequence

<220>
<223> Detection primer for DAPK1

<400> 433

aaccctttct tcaaattaca aa                                                22

<210> 434
<211> 21
<212> DNA
<213> Artificial Sequence

<220>
<223> Detection primer for DAPK1

<400> 434

tgattgggtt ttagggaaat a                                                 21

<210> 435
<211> 22
<212> DNA
<213> Artificial Sequence

<220>
<223> Detection primer for EGFR

<400> 435

gggtttggtt gtaatatgga tt                                                22

<210> 436
<211> 19
<212> DNA
<213> Artificial Sequence

<220>
<223> Detection primer for EGFR

<400> 436

cccaacacta cccctctaa                                                    19

<210> 437
<211> 21
<212> DNA
<213> Artificial Sequence

<220>
<223> Detection primer for ERBB2

<400> 437

ggaagaggtg attaaatgga t                                                 21

<210> 438
<211> 19
<212> DNA

<213> Artificial Sequence

<220>
<223> Detection primer for ERBB2

<400> 438

cccaaaaatc aaacaacaa                                                    19

<210> 439
<211> 19
<212> DNA
<213> Artificial Sequence

<220>
<223> Detection primer for GSTP1

<400> 439

atttgggaaa gagggaaag                                                    19

<210> 440
<211> 21
<212> DNA
<213> Artificial Sequence

<220>
<223> Detection primer for GSTP1

<400> 440

taaaaactct aaaccccatc c                                                 21

<210> 441
<211> 19
<212> DNA
<213> Artificial Sequence

<220>
<223> Detection primer for GTBP/MSH6

<400> 441

ccctacccac caatatacc                                                    19

<210> 442
<211> 21
<212> DNA
<213> Artificial Sequence

<220>
<223> Detection primer for GTBP/MSH6

<400> 442

agatttgggg aagaagttgt a                                                 21

<210> 443
<211> 20
<212> DNA
<213> Artificial Sequence

<220>

<223> Detection primer for HIC-1

<400> 443

tgggttggag aagaagttta                                                    20

<210> 444
<211> 21
<212> DNA
<213> Artificial Sequence

<220>
<223> Detection primer for HIC-1

<400> 444

tcatatttcc aaaaacacac c                                                  21

<210> 445
<211> 23
<212> DNA
<213> Artificial Sequence

<220>
<223> Detection primer for HRAS

<400> 445

cttattccca tctaaaccct att                                                23

<210> 446
<211> 22
<212> DNA
<213> Artificial Sequence

<220>
<223> Detection primer for HRAS

<400> 446

gtggttttgt gaagttttag gt                                                 22

<210> 447
<211> 20
<212> DNA
<213> Artificial Sequence

<220>
<223> Detection primer for IGF2

<400> 447

cccttcccct taactaaact                                                    20

<210> 448
<211> 19
<212> DNA
<213> Artificial Sequence

<220>
<223> Detection primer for IGF2

<400> 448

aatttgggtt aggtttgga 19

<210> 449
<211> 22
<212> DNA
<213> Artificial Sequence

<220>
<223> Detection primer for LKB1

<400> 449

taaaagaagg atttttgatt gg 22

<210> 450
<211> 21
<212> DNA
<213> Artificial Sequence

<220>
<223> Detection primer for LKB1

<400> 450

catcttattt acctccctcc c 21

<210> 451
<211> 22
<212> DNA
<213> Artificial Sequence

<220>
<223> Detection primer for MGMT

<400> 451

aaggttttag ggaagagtgt tt 22

<210> 452
<211> 23
<212> DNA
<213> Artificial Sequence

<220>
<223> Detection primer for MGMT

<400> 452

accttttcct atcacaaaaa taa 23

<210> 453
<211> 20
<212> DNA
<213> Artificial Sequence

<220>
<223> Detection primer for MLH1

<400> 453

taaggggaga ggaggagttt 20

<210> 454
<211> 22
<212> DNA
<213> Artificial Sequence

<220>
<223> Detection primer for MLH1

<400> 454

accaattctc aatcatctct tt                                                22

<210> 455
<211> 22
<212> DNA
<213> Artificial Sequence

<220>
<223> Detection primer for MNCA9

<400> 455

gggaagtagg ttagggttag tt                                                22

<210> 456
<211> 22
<212> DNA
<213> Artificial Sequence

<220>
<223> Detection primer for MNCA9

<400> 456

aaatcctcct ctccaaataa at                                                22

<210> 457
<211> 18
<212> DNA
<213> Artificial Sequence

<220>
<223> Detection primer for MSH3

<400> 457

tgtttgggat tgggtagg                                                     18

<210> 458
<211> 23
<212> DNA
<213> Artificial Sequence

<220>
<223> Detection primer for MSH3

<400> 458

cataaccttt acctatctcc tca                                               23

<210> 459
<211> 22
<212> DNA

<213> Artificial Sequence

<220>
<223> Detection primer for MYC

<400> 459

agagggagta aaagaaaatg gt                                                22

<210> 460
<211> 22
<212> DNA
<213> Artificial Sequence

<220>
<223> Detection primer for MYC

<400> 460

ccaaataaac aaaataacct cc                                                22

<210> 461
<211> 22
<212> DNA
<213> Artificial Sequence

<220>
<223> Detection primer for N33

<400> 461

ttttagattg aggttttagg gt                                                22

<210> 462
<211> 22
<212> DNA
<213> Artificial Sequence

<220>
<223> Detection primer for N33

<400> 462

atccattcta cctccttttt ct                                                22

<210> 463
<211> 18
<212> DNA
<213> Artificial Sequence

<220>
<223> Detection primer for PAX6

<400> 463

ggaggggaga gggttatg                                                     18

<210> 464
<211> 22
<212> DNA
<213> Artificial Sequence

<220>

<223> Detection primer for PAX6

<400> 464

tactatacac accccaaaac aa                                                22

<210> 465
<211> 19
<212> DNA
<213> Artificial Sequence

<220>
<223> Detection primer for PGR

<400> 465

ttttgggaat gggttgtat                                                    19

<210> 466
<211> 21
<212> DNA
<213> Artificial Sequence

<220>
<223> Detection primer for PGR

<400> 466

ctacccttaa cctccatcct a                                                 21

<210> 467
<211> 26
<212> DNA
<213> Artificial Sequence

<220>
<223> Detection primer for PTEN

<400> 467

ttttaggtag ttatattggg tatgtt                                            26

<210> 468
<211> 21
<212> DNA
<213> Artificial Sequence

<220>
<223> Detection primer for PTEN

<400> 468

tcaactctca aacttccatc a                                                 21

<210> 469
<211> 22
<212> DNA
<213> Artificial Sequence

<220>
<223> Detection primer for RARB

<400> 469

ttgttgggag tttttaagtt tt                                                22

<210> 470
<211> 22
<212> DNA
<213> Artificial Sequence

<220>
<223> Detection primer for RARB

<400> 470

caaattctcc ttccaaataa at                                                22

<210> 471
<211> 20
<212> DNA
<213> Artificial Sequence

<220>
<223> Detection primer for SFN

<400> 471

gaagagagga gagggaggta                                                   20

<210> 472
<211> 19
<212> DNA
<213> Artificial Sequence

<220>
<223> Detection primer for SFN

<400> 472

ctatccaaca aacccaaca                                                    19

<210> 473
<211> 22
<212> DNA
<213> Artificial Sequence

<220>
<223> Detection primer for S100A2

<400> 473

gtttttaagt tggagaagag ga                                                22

<210> 474
<211> 22
<212> DNA
<213> Artificial Sequence

<220>
<223> Detection primer for S100A2

<400> 474

acctataaat cacaacccac tc                                                22

<210> 475
<211> 22
<212> DNA
<213> Artificial Sequence

<220>
<223> Detection primer for TGFBR2

<400> 475

gtaatttgaa gaaagttgag gg                                                22

<210> 476
<211> 22
<212> DNA
<213> Artificial Sequence

<220>
<223> Detection primer for TGFBR2

<400> 476

ccaacaacta aacaaaacct ct                                                22

<210> 477
<211> 22
<212> DNA
<213> Artificial Sequence

<220>
<223> Detection primer for TIMP3

<400> 477

tgagaaaatt gttgtttgaa gt                                                22

<210> 478
<211> 22
<212> DNA
<213> Artificial Sequence

<220>
<223> Detection primer for TIMP3

<400> 478

caaaataccc taaaaaccac tc                                                22

<210> 479
<211> 20
<212> DNA
<213> Artificial Sequence

<220>
<223> Detection primer for TP53

<400> 479

ggagttgtat tgttgggaga                                                   20

<210> 480
<211> 21
<212> DNA

<213> Artificial Sequence

<220>
<223> Detection primer for TP53

<400> 480

taaaacccca attttcacta a                                                21

<210> 481
<211> 24
<212> DNA
<213> Artificial Sequence

<220>
<223> Detection primer for TP73

<400> 481

agtaaatagt gggtgagtta tgaa                                             24

<210> 482
<211> 25
<212> DNA
<213> Artificial Sequence

<220>
<223> Detection primer for TP73

<400> 482

gaaaaacctc taaaaactac tctcc                                            25

<210> 483
<211> 26
<212> DNA
<213> Artificial Sequence

<220>
<223> Detection primer for VHL

<400> 483

tgtaaaatga ataaagttaa tgagtg                                           26

<210> 484
<211> 23
<212> DNA
<213> Artificial Sequence

<220>
<223> Detection primer for VHL

<400> 484

tcctaaattc aaataatcct cct                                              23

<210> 485
<211> 22
<212> DNA
<213> Artificial Sequence

<220>

<223> Detection primer for CDKN1C

<400> 485

ggggaggtag atatttggat aa                                                22

<210> 486
<211> 23
<212> DNA
<213> Artificial Sequence

<220>
<223> Detection primer for CDKN1C

<400> 486

aactacacca tttatattcc cac                                               23

<210> 487
<211> 22
<212> DNA
<213> Artificial Sequence

<220>
<223> Detection primer for CAV1

<400> 487

gttagtatgt ttgggggtaa at                                                22

<210> 488
<211> 22
<212> DNA
<213> Artificial Sequence

<220>
<223> Detection primer for CAV1

<400> 488

ataaataaca ccttccaccc ta                                                22

<210> 489
<211> 22
<212> DNA
<213> Artificial Sequence

<220>
<223> Detection primer for CDH13

<400> 489

tttgtattag gttggaagtg gt                                                22

<210> 490
<211> 22
<212> DNA
<213> Artificial Sequence

<220>
<223> Detection primer for CDH13

<400> 490

cccaaataaa tcaacaacaa ca                                                22

<210> 491
<211> 22
<212> DNA
<213> Artificial Sequence

<220>
<223> Detection primer for DRG1

<400> 491

ggttttgggt ttagtggtaa at                                                22

<210> 492
<211> 22
<212> DNA
<213> Artificial Sequence

<220>
<223> Detection primer for DRG1

<400> 492

aactttcata actcaccctt tc                                                22

<210> 493
<211> 22
<212> DNA
<213> Artificial Sequence

<220>
<223> Detection primer for PTGS2

<400> 493

gatttttgga gaggaagtta ag                                                22

<210> 494
<211> 22
<212> DNA
<213> Artificial Sequence

<220>
<223> Detection primer for PTGS2

<400> 494

aaaactaaaa accaaaccca ta                                                22

<210> 495
<211> 20
<212> DNA
<213> Artificial Sequence

<220>
<223> Detection primer for THBS1

<400> 495

tggggttagt ttaggatagg                                                   20

<210> 496
<211> 25
<212> DNA
<213> Artificial Sequence

<220>
<223> Detection primer for THBS1

<400> 496

cttaaaaaca ctaaaacttc tcaaa 25

<210> 497
<211> 21
<212> DNA
<213> Artificial Sequence

<220>
<223> Detection primer for TPEF (=TMEFF2; =HPP1)

<400> 497

ttgtttgggt taataaatgg a 21

<210> 498
<211> 20
<212> DNA
<213> Artificial Sequence

<220>
<223> Detection primer for TPEF (=TMEFF2; =HPP1)

<400> 498

cttctctctt ctcccctctc 20

<210> 499
<211> 18
<212> DNA
<213> Artificial Sequence

<220>
<223> Detection primer for DNMT1

<400> 499

tccccatcac acctaaaa 18

<210> 500
<211> 18
<212> DNA
<213> Artificial Sequence

<220>
<223> Detection primer for DNMT1

<400> 500

gggaggaggg gatgtatt 18

<210> 501
<211> 22
<212> DNA

<213> Artificial Sequence

<220>
<223> Detection primer for CEA

<400> 501

tatgggagga ggttagtaag tg                                                22

<210> 502
<211> 22
<212> DNA
<213> Artificial Sequence

<220>
<223> Detection primer for CEA

<400> 502

ccccaaatcc tacatataaa aa                                                22

<210> 503
<211> 21
<212> DNA
<213> Artificial Sequence

<220>
<223> Detection primer for MB

<400> 503

gtttttggta aaggggtaga a                                                 21

<210> 504
<211> 22
<212> DNA
<213> Artificial Sequence

<220>
<223> Detection primer for MB

<400> 504

cctaaaatat caacctccac ct                                                22

<210> 505
<211> 23
<212> DNA
<213> Artificial Sequence

<220>
<223> Detection primer for PCNA

<400> 505

tttttaggtt gtaaggaggt ttt                                               23

<210> 506
<211> 22
<212> DNA
<213> Artificial Sequence

<220>

<223> Detection primer for PCNA

<400> 506

taaatacctc caacaccttt ct                                                22

<210> 507
<211> 25
<212> DNA
<213> Artificial Sequence

<220>
<223> Detection primer for CDC2

<400> 507

attagaagtg aaagtaatgg aattt                                             25

<210> 508
<211> 19
<212> DNA
<213> Artificial Sequence

<220>
<223> Detection primer for CDC2

<400> 508

tcaatttcca aaaaccaac                                                    19

<210> 509
<211> 22
<212> DNA
<213> Artificial Sequence

<220>
<223> Detection primer for ESR1

<400> 509
   agggggaatt aaatagaaag ag                                                 22

<210> 510
<211> 22
<212> DNA
<213> Artificial Sequence

<220>
<223> Detection primer for ESR1

<400> 510

caataaaacc atcccaaata ct                                                22

<210> 511
<211> 22
<212> DNA
<213> Artificial Sequence

<220>
<223> Detection primer for CASP8

<400> 511

agtggatttg gagtttagat gt                                                22

<210> 512
<211> 22
<212> DNA
<213> Artificial Sequence

<220>
<223> Detection primer for CASP8

<400> 512

aacaaaataa aaacttctcc ca                                                22

<210> 513
<211> 24
<212> DNA
<213> Artificial Sequence

<220>
<223> Detection primer for RASSF1

<400> 513

acctctctac aaattacaaa ttca                                              24

<210> 514
<211> 20
<212> DNA
<213> Artificial Sequence

<220>
<223> Detection primer for RASSF1

<400> 514

agtttgggtt agtttgggtt                                                   20

<210> 515
<211> 21
<212> DNA
<213> Artificial Sequence

<220>
<223> Detection primer for MSH4

<400> 515

aggatgttga ggtttgagat t                                                 21

<210> 516
<211> 21
<212> DNA
<213> Artificial Sequence

<220>
<223> Detection primer for MSH4

<400> 516

cactataata accaccaccc a                                                 21

<210> 517
<211> 22
<212> DNA
<213> Artificial Sequence

<220>
<223> Detection primer for MSH5

<400> 517

tattaggaat aaagttgggg ag                                                22

<210> 518
<211> 22
<212> DNA
<213> Artificial Sequence

<220>
<223> Detection primer for MSH5

<400> 518

aacccttcaa acaaaaataa aa                                                22

<210> 519
<211> 18
<212> DNA
<213> Artificial Sequence

<220>
<223> Detection oligonucleotide for MDR1

<400> 519

ttggtggtcg ttttaagg                                                     18

<210> 520
<211> 18
<212> DNA
<213> Artificial Sequence

<220>
<223> Detection oligonucleotide for MDR1

<400> 520

ttggtggttg ttttaagg                                                     18

<210> 521
<211> 18
<212> DNA
<213> Artificial Sequence

<220>
<223> Detection oligonucleotide for MDR1

<400> 521

ttgaaagacg tgtttata                                                     18

<210> 522
<211> 18
<212> DNA

<213> Artificial Sequence

<220>
<223> Detection oligonucleotide for MDR1

<400> 522

ttgaaagatg tgtttata 18

<210> 523
<211> 18
<212> DNA
<213> Artificial Sequence

<220>
<223> Detection oligonucleotide for MDR1

<400> 523

aggtgtaacg gaagttag 18

<210> 524
<211> 18
<212> DNA
<213> Artificial Sequence

<220>
<223> Detection oligonucleotide for MDR1

<400> 524

aggtgtaatg gaagttag 18

<210> 525
<211> 18
<212> DNA
<213> Artificial Sequence

<220>
<223> Detection oligonucleotide for MDR1

<400> 525

tagtttttcg aggaatta 18

<210> 526
<211> 18
<212> DNA
<213> Artificial Sequence

<220>
<223> Detection oligonucleotide for MDR1

<400> 526

tagtttttg aggaatta 18

<210> 527
<211> 18
<212> DNA
<213> Artificial Sequence

<220>

<223> Detection oligonucleotide for APOC2

<400> 527

gagagtttcg tttttgtt                                                    18

<210> 528
<211> 18
<212> DNA
<213> Artificial Sequence

<220>
<223> Detection oligonucleotide for APOC2

<400> 528

gagagttttg tttttgtt                                                    18

<210> 529
<211> 18
<212> DNA
<213> Artificial Sequence

<220>
<223> Detection oligonucleotide for APOC2

<400> 529

ttgggggacg ttattgtt                                                    18

<210> 530
<211> 18
<212> DNA
<213> Artificial Sequence

<220>
<223> Detection oligonucleotide for APOC2

<400> 530

ttgggggatg ttattgtt                                                    18

<210> 531
<211> 18
<212> DNA
<213> Artificial Sequence

<220>
<223> Detection oligonucleotide for APOC2

<400> 531

tgtgttcgtt cggagttg                                                    18

<210> 532
<211> 18
<212> DNA
<213> Artificial Sequence

<220>
<223> Detection oligonucleotide for APOC2

<400> 532

tgtgtttgtt tggagttg 18

<210> 533
<211> 18
<212> DNA
<213> Artificial Sequence

<220>
<223> Detection oligonucleotide for APOC2

<400> 533

tgggtttgcg gagaatgg 18

<210> 534
<211> 18
<212> DNA
<213> Artificial Sequence

<220>
<223> Detection oligonucleotide for APOC2

<400> 534

tgggtttgtg gagaatgg 18

<210> 535
<211> 18
<212> DNA
<213> Artificial Sequence

<220>
<223> Detection oligonucleotide for CACNA1G

<400> 535

tttaggagcg ttaatgtg 18

<210> 536
<211> 18
<212> DNA
<213> Artificial Sequence

<220>
<223> Detection oligonucleotide for CACNA1G

<400> 536

tttaggagtg ttaatgtg 18

<210> 537
<211> 18
<212> DNA
<213> Artificial Sequence

<220>
<223> Detection oligonucleotide for CACNA1G

<400> 537

tagggttacg aggttagg 18

<210> 538
<211> 18
<212> DNA
<213> Artificial Sequence

<220>
<223> Detection oligonucleotide for CACNA1G

<400> 538

tagggttatg aggttagg                                                    18

<210> 539
<211> 18
<212> DNA
<213> Artificial Sequence

<220>
<223> Detection oligonucleotide for CACNA1G

<400> 539

ggaggttacg tttagatt                                                    18

<210> 540
<211> 18
<212> DNA
<213> Artificial Sequence

<220>
<223> Detection oligonucleotide for CACNA1G

<400> 540

ggaggttatg tttagatt                                                    18

<210> 541
<211> 18
<212> DNA
<213> Artificial Sequence

<220>
<223> Detection oligonucleotide for CACNA1G

<400> 541

ttaggggtcg tggataaa                                                    18

<210> 542
<211> 18
<212> DNA
<213> Artificial Sequence

<220>
<223> Detection oligonucleotide for CACNA1G

<400> 542

ttaggggttg tggataaa                                                    18

<210> 543
<211> 18
<212> DNA

<213> Artificial Sequence

<220>
<223> Detection oligonucleotide for EGR4

<400> 543

ggtgggaagc gtatttat 18

<210> 544
<211> 18
<212> DNA
<213> Artificial Sequence

<220>
<223> Detection oligonucleotide for EGR4

<400> 544

ggtgggaagt gtatttat 18

<210> 545
<211> 18
<212> DNA
<213> Artificial Sequence

<220>
<223> Detection oligonucleotide for EGR4

<400> 545

ttatagttcg agtttttt 18

<210> 546
<211> 18
<212> DNA
<213> Artificial Sequence

<220>
<223> Detection oligonucleotide for EGR4

<400> 546

ttatagtttg agtttttt 18

<210> 547
<211> 18
<212> DNA
<213> Artificial Sequence

<220>
<223> Detection oligonucleotide for AR

<400> 547

tgttatttcg agagaggt 18

<210> 548
<211> 18
<212> DNA
<213> Artificial Sequence

<220>

<223> Detection oligonucleotide for AR

<400> 548

tgttattttg agagaggt                                                    18

<210> 549
<211> 18
<212> DNA
<213> Artificial Sequence

<220>
<223> Detection oligonucleotide for AR

<400> 549

attttgagcg aggttagt                                                    18

<210> 550
<211> 18
<212> DNA
<213> Artificial Sequence

<220>
<223> Detection oligonucleotide for AR

<400> 550

attttgagtg aggttagt                                                    18

<210> 551
<211> 18
<212> DNA
<213> Artificial Sequence

<220>
<223> Detection oligonucleotide for AR

<400> 551

gtagtattcg aaggtagt                                                    18

<210> 552
<211> 18
<212> DNA
<213> Artificial Sequence

<220>
<223> Detection oligonucleotide for AR

<400> 552

gtagtatttg aaggtagt                                                    18

<210> 553
<211> 18
<212> DNA
<213> Artificial Sequence

<220>
<223> Detection oligonucleotide for RB1

<400> 553

ttagatttcg ggataggg 18

<210> 554
<211> 18
<212> DNA
<213> Artificial Sequence

<220>
<223> Detection oligonucleotide for RB1

<400> 554

ttagattttg ggataggg 18

<210> 555
<211> 18
<212> DNA
<213> Artificial Sequence

<220>
<223> Detection oligonucleotide for RB1

<400> 555

tatagtttcg ttaagtgt 18

<210> 556
<211> 18
<212> DNA
<213> Artificial Sequence

<220>
<223> Detection oligonucleotide for RB1

<400> 556

tatagttttg ttaagtgt 18

<210> 557
<211> 18
<212> DNA
<213> Artificial Sequence

<220>
<223> Detection oligonucleotide for RB1

<400> 557

gtgtatttcg gtttggag 18

<210> 558
<211> 18
<212> DNA
<213> Artificial Sequence

<220>
<223> Detection oligonucleotide for RB1

<400> 558

gtgtattttg gtttggag 18

<210> 559
<211> 18
<212> DNA
<213> Artificial Sequence

<220>
<223> Detection oligonucleotide for RB1

<400> 559

tggatttacg ttaggttt                                                    18

<210> 560
<211> 18
<212> DNA
<213> Artificial Sequence

<220>
<223> Detection oligonucleotide for RB1

<400> 560

tggatttatg ttaggttt                                                    18

<210> 561
<211> 18
<212> DNA
<213> Artificial Sequence

<220>
<223> Detection oligonucleotide for GPIb beta

<400> 561

tgttatttgt cgttgtag                                                    18

<210> 562
<211> 18
<212> DNA
<213> Artificial Sequence

<220>
<223> Detection oligonucleotide for GPIb beta

<400> 562

tgttatttgt tgttgtag                                                    18

<210> 563
<211> 18
<212> DNA
<213> Artificial Sequence

<220>
<223> Detection oligonucleotide for GPIb beta

<400> 563

gtgggagcgg aagtttga                                                    18

<210> 564
<211> 18
<212> DNA

<213> Artificial Sequence

<220>
<223> Detection oligonucleotide for GPIb beta

<400> 564

gtgggagtgg aagtttga                                                    18

<210> 565
<211> 18
<212> DNA
<213> Artificial Sequence

<220>
<223> Detection oligonucleotide for GPIb beta

<400> 565

tagagtaagt cgggttgt                                                    18

<210> 566
<211> 19
<212> DNA
<213> Artificial Sequence

<220>
<223> Detection oligonucleotide for GPIb beta

<400> 566

tagagtaagt cgggttgtt                                                   19

<210> 567
<211> 18
<212> DNA
<213> Artificial Sequence

<220>
<223> Detection oligonucleotide for GPIb beta

<400> 567

ggttaggtcg tagtattg                                                    18

<210> 568
<211> 18
<212> DNA
<213> Artificial Sequence

<220>
<223> Detection oligonucleotide for GPIb beta

<400> 568

ggttaggttg tagtattg                                                    18

<210> 569
<211> 18
<212> DNA
<213> Artificial Sequence

<220>

<223> Detection oligonucleotide for MYOD1

<400> 569

atagtagtcg ggtgttgg 18

<210> 570
<211> 18
<212> DNA
<213> Artificial Sequence

<220>
<223> Detection oligonucleotide for MYOD1

<400> 570

atagtagttg ggtgttgg 18

<210> 571
<211> 18
<212> DNA
<213> Artificial Sequence

<220>
<223> Detection oligonucleotide for MYOD1

<400> 571

tagttgttcg tttgggtt 18

<210> 572
<211> 18
<212> DNA
<213> Artificial Sequence

<220>
<223> Detection oligonucleotide for MYOD1

<400> 572

tagttgtttg tttgggtt 18

<210> 573
<211> 18
<212> DNA
<213> Artificial Sequence

<220>
<223> Detection oligonucleotide for WT1

<400> 573

tttagtttcg atttttgg 18

<210> 574
<211> 18
<212> DNA
<213> Artificial Sequence

<220>
<223> Detection oligonucleotide for WT1

<400> 574

tttagttttg atttttgg                                                    18

<210> 575
<211> 18
<212> DNA
<213> Artificial Sequence

<220>
<223> Detection oligonucleotide for HLA-F

<400> 575

atagggtacg ttaaggtt                                                    18

<210> 576
<211> 18
<212> DNA
<213> Artificial Sequence

<220>
<223> Detection oligonucleotide for HLA-F

<400> 576

atagggtatg ttaaggtt                                                    18

<210> 577
<211> 18
<212> DNA
<213> Artificial Sequence

<220>
<223> Detection oligonucleotide for HLA-F

<400> 577

gagagaaacg gtttttgt                                                    18

<210> 578
<211> 18
<212> DNA
<213> Artificial Sequence

<220>
<223> Detection oligonucleotide for HLA-F

<400> 578

gagagaaatg gtttttgt                                                    18

<210> 579
<211> 18
<212> DNA
<213> Artificial Sequence

<220>
<223> Detection oligonucleotide for ELK1

<400> 579

tgtttaatcg tagagttg                                                    18

<210> 580
<211> 18
<212> DNA
<213> Artificial Sequence

<220>
<223> Detection oligonucleotide for ELK1

<400> 580

tgtttaattg tagagttg                                                    18

<210> 581
<211> 18
<212> DNA
<213> Artificial Sequence

<220>
<223> Detection oligonucleotide for ELK1

<400> 581

tttgttttcg ttgagtag                                                    18

<210> 582
<211> 18
<212> DNA
<213> Artificial Sequence

<220>
<223> Detection oligonucleotide for ELK1

<400> 582

tttgtttttg ttgagtag                                                    18

<210> 583
<211> 18
<212> DNA
<213> Artificial Sequence

<220>
<223> Detection oligonucleotide for ELK1

<400> 583

gaagggttcg ttttttaa                                                    18

<210> 584
<211> 18
<212> DNA
<213> Artificial Sequence

<220>
<223> Detection oligonucleotide for ELK1

<400> 584

gaagggtttg ttttttaa                                                    18

<210> 585
<211> 18
<212> DNA

<213> Artificial Sequence

<220>
<223> Detection oligonucleotide for ELK1

<400> 585

attaatagcg ttttggtt                                                    18

<210> 586
<211> 18
<212> DNA
<213> Artificial Sequence

<220>
<223> Detection oligonucleotide for ELK1

<400> 586

attaatagtg ttttggtt                                                    18

<210> 587
<211> 18
<212> DNA
<213> Artificial Sequence

<220>
<223> Detection oligonucleotide for APC

<400> 587

tatttagcgg attatata                                                    18

<210> 588
<211> 18
<212> DNA
<213> Artificial Sequence

<220>
<223> Detection oligonucleotide for APC

<400> 588

tatttagtgg attatata                                                    18

<210> 589
<211> 18
<212> DNA
<213> Artificial Sequence

<220>
<223> Detection oligonucleotide for APC

<400> 589

aaggttatcg gtttaaga                                                    18

<210> 590
<211> 18
<212> DNA
<213> Artificial Sequence

<220>

<223> Detection oligonucleotide for APC

<400> 590

aaggttattg gtttaaga                                                    18

<210> 591
<211> 18
<212> DNA
<213> Artificial Sequence

<220>
<223> Detection oligonucleotide for APC

<400> 591

gggggacgac gtttttgt                                                    18

<210> 592
<211> 18
<212> DNA
<213> Artificial Sequence

<220>
<223> Detection oligonucleotide for APC

<400> 592

gggggatgat gtttttgt                                                    18

<210> 593
<211> 18
<212> DNA
<213> Artificial Sequence

<220>
<223> Detection oligonucleotide for BCL2

<400> 593

agtgtttcgc gtgattga                                                    18

<210> 594
<211> 18
<212> DNA
<213> Artificial Sequence

<220>
<223> Detection oligonucleotide for BCL2

<400> 594

agtgttttgt gtgattga                                                    18

<210> 595
<211> 18
<212> DNA
<213> Artificial Sequence

<220>
<223> Detection oligonucleotide for BCL2

<400> 595

taagttgtcg tagagggg 18

<210> 596
<211> 18
<212> DNA
<213> Artificial Sequence

<220>
<223> Detection oligonucleotide for BCL2

<400> 596

taagttgttg tagagggg 18

<210> 597
<211> 18
<212> DNA
<213> Artificial Sequence

<220>
<223> Detection oligonucleotide for BCL2

<400> 597

ggatttcgtc gttgtaga 18

<210> 598
<211> 18
<212> DNA
<213> Artificial Sequence

<220>
<223> Detection oligonucleotide for BCL2

<400> 598

ggattttgtt gttgtaga 18

<210> 599
<211> 18
<212> DNA
<213> Artificial Sequence

<220>
<223> Detection oligonucleotide for CALCA

<400> 599

gagggtgacg taatttag 18

<210> 600
<211> 18
<212> DNA
<213> Artificial Sequence

<220>
<223> Detection oligonucleotide for CALCA

<400> 600

gagggtgatg taatttag 18

<210> 601
<211> 18
<212> DNA
<213> Artificial Sequence

<220>
<223> Detection oligonucleotide for CALCA

<400> 601

tgtattggcg gaattttt                                                    18

<210> 602
<211> 18
<212> DNA
<213> Artificial Sequence

<220>
<223> Detection oligonucleotide for CALCA

<400> 602

tgtattggtg gaattttt                                                    18

<210> 603
<211> 18
<212> DNA
<213> Artificial Sequence

<220>
<223> Detection oligonucleotide for CALCA

<400> 603

gttagtttcg ggatattt                                                    18

<210> 604
<211> 18
<212> DNA
<213> Artificial Sequence

<220>
<223> Detection oligonucleotide for CALCA

<400> 604

gttagttttg ggatattt                                                    18

<210> 605
<211> 18
<212> DNA
<213> Artificial Sequence

<220>
<223> Detection oligonucleotide for CDH1

<400> 605

tagaggatcg tttgagtt                                                    18

<210> 606
<211> 18
<212> DNA

<213> Artificial Sequence

<220>
<223> Detection oligonucleotide for CDH1

<400> 606

tagaggattg tttgagtt                                                    18

<210> 607
<211> 18
<212> DNA
<213> Artificial Sequence

<220>
<223> Detection oligonucleotide for CDH1

<400> 607

gttgtgatcg tattattg                                                    18

<210> 608
<211> 18
<212> DNA
<213> Artificial Sequence

<220>
<223> Detection oligonucleotide for CDH1

<400> 608

gttgtgattg tattattg                                                    18

<210> 609
<211> 18
<212> DNA
<213> Artificial Sequence

<220>
<223> Detection oligonucleotide for CDH1

<400> 609

ttgggattcg aatttagt                                                    18

<210> 610
<211> 18
<212> DNA
<213> Artificial Sequence

<220>
<223> Detection oligonucleotide for CDH1

<400> 610

ttgggatttg aatttagt                                                    18

<210> 611
<211> 18
<212> DNA
<213> Artificial Sequence

<220>

<223> Detection oligonucleotide for CDKN1A

<400> 611

aggtgtatcg tttttata                                                    18

<210> 612
<211> 18
<212> DNA
<213> Artificial Sequence

<220>
<223> Detection oligonucleotide for CDKN1A

<400> 612

aggtgtattg tttttata                                                    18

<210> 613
<211> 18
<212> DNA
<213> Artificial Sequence

<220>
<223> Detection oligonucleotide for CDKN1A

<400> 613

tgggttagcg gtgagtta                                                    18

<210> 614
<211> 18
<212> DNA
<213> Artificial Sequence

<220>
<223> Detection oligonucleotide for CDKN1A

<400> 614

tgggttagtg gtgagtta                                                    18

<210> 615
<211> 18
<212> DNA
<213> Artificial Sequence

<220>
<223> Detection oligonucleotide for CDKN1A

<400> 615

gtttatttcg tggggaaa                                                    18

<210> 616
<211> 18
<212> DNA
<213> Artificial Sequence

<220>
<223> Detection oligonucleotide for CDKN1A

<400> 616

gtttattttg tggggaaa                                                    18

<210> 617
<211> 18
<212> DNA
<213> Artificial Sequence

<220>
<223> Detection oligonucleotide for CDKN1A

<400> 617

ttggaattcg gttaggtt                                                    18

<210> 618
<211> 18
<212> DNA
<213> Artificial Sequence

<220>
<223> Detection oligonucleotide for CDKN1A

<400> 618

ttggaatttg gttaggtt                                                    18

<210> 619
<211> 18
<212> DNA
<213> Artificial Sequence

<220>
<223> Detection oligonucleotide for CDKN1B (p27 Kip1)

<400> 619

aagagaaacg ttggaata                                                    18

<210> 620
<211> 18
<212> DNA
<213> Artificial Sequence

<220>
<223> Detection oligonucleotide for CDKN1B (p27 Kip1)

<400> 620

aagagaaatg ttggaata                                                    18

<210> 621
<211> 18
<212> DNA
<213> Artificial Sequence

<220>
<223> Detection oligonucleotide for CDKN1B (p27 Kip1)

<400> 621

tttgatttcg aggggagt                                                    18

<210> 622
<211> 18
<212> DNA
<213> Artificial Sequence

<220>
<223> Detection oligonucleotide for CDKN1B (p27 Kip1)

<400> 622

tttgattttg aggggagt                                                    18

<210> 623
<211> 18
<212> DNA
<213> Artificial Sequence

<220>
<223> Detection oligonucleotide for CDKN1B (p27 Kip1)

<400> 623

gtatttggcg gttggatt                                                    18

<210> 624
<211> 18
<212> DNA
<213> Artificial Sequence

<220>
<223> Detection oligonucleotide for CDKN1B (p27 Kip1)

<400> 624

gtatttggtg gttggatt                                                    18

<210> 625
<211> 18
<212> DNA
<213> Artificial Sequence

<220>
<223> Detection oligonucleotide for CDKN1B (p27 Kip1)

<400> 625

tataatttcg ggaaagaa                                                    18

<210> 626
<211> 18
<212> DNA
<213> Artificial Sequence

<220>
<223> Detection oligonucleotide for CDKN1B (p27 Kip1)

<400> 626

tataattttg ggaaagaa                                                    18

<210> 627
<211> 18
<212> DNA

<213> Artificial Sequence

<220>
<223> Detection oligonucleotide for CDKN2a

<400> 627

agagtgaacg tatttaaa                                                    18

<210> 628
<211> 18
<212> DNA
<213> Artificial Sequence

<220>
<223> Detection oligonucleotide for CDKN2a

<400> 628

agagtgaatg tatttaaa                                                    18

<210> 629
<211> 18
<212> DNA
<213> Artificial Sequence

<220>
<223> Detection oligonucleotide for CDKN2a

<400> 629

gatagggtcg gagggggt                                                    18

<210> 630
<211> 18
<212> DNA
<213> Artificial Sequence

<220>
<223> Detection oligonucleotide for CDKN2a

<400> 630

gatagggttg gagggggt                                                    18

<210> 631
<211> 18
<212> DNA
<213> Artificial Sequence

<220>
<223> Detection oligonucleotide for CDKN2B

<400> 631

atatttagcg agtagtgt                                                    18

<210> 632
<211> 18
<212> DNA
<213> Artificial Sequence

<220>

<223> Detection oligonucleotide for CDKN2B

<400> 632

atatttagtg agtagtgt                                                    18

<210> 633
<211> 18
<212> DNA
<213> Artificial Sequence

<220>
<223> Detection oligonucleotide for CDKN2B

<400> 633

ataggggcg gagtttaa                                                     18

<210> 634
<211> 18
<212> DNA
<213> Artificial Sequence

<220>
<223> Detection oligonucleotide for CDKN2B

<400> 634

ataggggtg gagtttaa                                                     18

<210> 635
<211> 18
<212> DNA
<213> Artificial Sequence

<220>
<223> Detection oligonucleotide for CDKN2B

<400> 635

ttattgtacg gggtttta                                                    18

<210> 636
<211> 18
<212> DNA
<213> Artificial Sequence

<220>
<223> Detection oligonucleotide for CDKN2B

<400> 636

ttattgtatg gggtttta                                                    18

<210> 637
<211> 18
<212> DNA
<213> Artificial Sequence

<220>
<223> Detection oligonucleotide for CDKN2B

<400> 637

ttttaagtcg tagaagga 18

<210> 638
<211> 18
<212> DNA
<213> Artificial Sequence

<220>
<223> Detection oligonucleotide for CDKN2B

<400> 638

ttttaagttg tagaagga 18

<210> 639
<211> 18
<212> DNA
<213> Artificial Sequence

<220>
<223> Detection oligonucleotide for CD44

<400> 639

ggtagtttcg attattta 18

<210> 640
<211> 18
<212> DNA
<213> Artificial Sequence

<220>
<223> Detection oligonucleotide for CD44

<400> 640

ggtagttttg attattta 18

<210> 641
<211> 18
<212> DNA
<213> Artificial Sequence

<220>
<223> Detection oligonucleotide for CD44

<400> 641

tggtggtacg tagtttgg 18

<210> 642
<211> 18
<212> DNA
<213> Artificial Sequence

<220>
<223> Detection oligonucleotide for CD44

<400> 642

tggtggtatg tagtttgg 18

<210> 643
<211> 18
<212> DNA
<213> Artificial Sequence

<220>
<223> Detection oligonucleotide for CSPG2

<400> 643

atgtgattcg tttgggta                                                    18

<210> 644
<211> 18
<212> DNA
<213> Artificial Sequence

<220>
<223> Detection oligonucleotide for CSPG2

<400> 644

atgtgatttg tttgggta                                                    18

<210> 645
<211> 18
<212> DNA
<213> Artificial Sequence

<220>
<223> Detection oligonucleotide for DAPK1

<400> 645

gttggagtcg aggtttga                                                    18

<210> 646
<211> 18
<212> DNA
<213> Artificial Sequence

<220>
<223> Detection oligonucleotide for DAPK1

<400> 646

gttggagttg aggtttga                                                    18

<210> 647
<211> 18
<212> DNA
<213> Artificial Sequence

<220>
<223> Detection oligonucleotide for DAPK1

<400> 647
   ttttttgtcg gattggtg                                                     18

<210> 648
<211> 18
<212> DNA

<213> Artificial Sequence

<220>
<223> Detection oligonucleotide for DAPK1

<400> 648

tttttgttg gattggtg                                                    18

<210> 649
<211> 18
<212> DNA
<213> Artificial Sequence

<220>
<223> Detection oligonucleotide for EGFR

<400> 649

gatgatttcg agggtgtt                                                   18

<210> 650
<211> 18
<212> DNA
<213> Artificial Sequence

<220>
<223> Detection oligonucleotide for EGFR

<400> 650

gatgattttg agggtgtt                                                   18

<210> 651
<211> 18
<212> DNA
<213> Artificial Sequence

<220>
<223> Detection oligonucleotide for EGFR

<400> 651

gagggtttcg tagtgttg                                                   18

<210> 652
<211> 18
<212> DNA
<213> Artificial Sequence

<220>
<223> Detection oligonucleotide for EGFR

<400> 652

gagggttttg tagtgttg                                                   18

<210> 653
<211> 18
<212> DNA
<213> Artificial Sequence

<220>

<223> Detection oligonucleotide for EGFR

<400> 653

tggggattcg aataaagg                                                    18

<210> 654
<211> 18
<212> DNA
<213> Artificial Sequence

<220>
<223> Detection oligonucleotide for EGFR

<400> 654

tggggatttg aataaagg                                                    18

<210> 655
<211> 18
<212> DNA
<213> Artificial Sequence

<220>
<223> Detection oligonucleotide for ERBB2

<400> 655

tatatatacg tgtgggta                                                    18

<210> 656
<211> 18
<212> DNA
<213> Artificial Sequence

<220>
<223> Detection oligonucleotide for ERBB2

<400> 656

tatatatatg tgtgggta                                                    18

<210> 657
<211> 18
<212> DNA
<213> Artificial Sequence

<220>
<223> Detection oligonucleotide for ERBB2

<400> 657

tttagatacg aaatgtta                                                    18

<210> 658
<211> 18
<212> DNA
<213> Artificial Sequence

<220>
<223> Detection oligonucleotide for ERBB2

<400> 658

tttagatatg aaatgtta 18

<210> 659
<211> 18
<212> DNA
<213> Artificial Sequence

<220>
<223> Detection oligonucleotide for GSTP1

<400> 659

ggtttttcg gttagttg 18

<210> 660
<211> 18
<212> DNA
<213> Artificial Sequence

<220>
<223> Detection oligonucleotide for GSTP1

<400> 660

ggtttttttg gttagttg 18

<210> 661
<211> 18
<212> DNA
<213> Artificial Sequence

<220>
<223> Detection oligonucleotide for GSTP1

<400> 661

gtagttttcg ttattagt 18

<210> 662
<211> 18
<212> DNA
<213> Artificial Sequence

<220>
<223> Detection oligonucleotide for GSTP1

<400> 662.

gtagttttg ttattagt 18

<210> 663
<211> 18
<212> DNA
<213> Artificial Sequence

<220>
<223> Detection oligonucleotide for GTBP/MSH6

<400> 663

ttttgtcgga cggagttt 18

<210> 664
<211> 18
<212> DNA
<213> Artificial Sequence

<220>
<223> Detection oligonucleotide for GTBP/MSH6

<400> 664

ttttgttgga tggagttt                                                    18

<210> 665
<211> 18
<212> DNA
<213> Artificial Sequence

<220>
<223> Detection oligonucleotide for GTBP/MSH6

<400> 665

aaggtttaat cgttttgt                                                    18

<210> 666
<211> 18
<212> DNA
<213> Artificial Sequence

<220>
<223> Detection oligonucleotide for GTBP/MSH6

<400> 666

aaggtttaat tgttttgt                                                    18

<210> 667
<211> 18
<212> DNA
<213> Artificial Sequence

<220>
<223> Detection oligonucleotide for HIC-1

<400> 667

ttaaaacggc gtataggg                                                    18

<210> 668
<211> 18
<212> DNA
<213> Artificial Sequence

<220>
<223> Detection oligonucleotide for HIC-1

<400> 668

ttaaaatggt gtataggg                                                    18

<210> 669
<211> 18
<212> DNA

<213> Artificial Sequence

<220>
<223> Detection oligonucleotide for HIC-1

<400> 669

aggagattcg aaagttta                                                    18

<210> 670
<211> 18
<212> DNA
<213> Artificial Sequence

<220>
<223> Detection oligonucleotide for HIC-1

<400> 670

aggagatttg aaagttta                                                    18

<210> 671
<211> 18
<212> DNA
<213> Artificial Sequence

<220>
<223> Detection oligonucleotide for HRAS

<400> 671

atagtgggcg taattggt                                                    18

<210> 672
<211> 18
<212> DNA
<213> Artificial Sequence

<220>
<223> Detection oligonucleotide for HRAS

<400> 672

atagtgggtg taattggt                                                    18

<210> 673
<211> 18
<212> DNA
<213> Artificial Sequence

<220>
<223> Detection oligonucleotide for HRAS

<400> 673

aaattggacg tttagttg                                                    18

<210> 674
<211> 18
<212> DNA
<213> Artificial Sequence

<220>

<223> Detection oligonucleotide for HRAS

<400> 674

aaattggatg tttagttg                                                    18

<210> 675
<211> 18
<212> DNA
<213> Artificial Sequence

<220>
<223> Detection oligonucleotide for HRAS

<400> 675

tagaagtcga gagatttg                                                    18

<210> 676
<211> 18
<212> DNA
<213> Artificial Sequence

<220>
<223> Detection oligonucleotide for HRAS

<400> 676

tagaagttga gagatttg                                                    18

<210> 677
<211> 18
<212> DNA
<213> Artificial Sequence

<220>
<223> Detection oligonucleotide for HRAS

<400> 677

gaatatttcg aagtttgt                                                    18

<210> 678
<211> 18
<212> DNA
<213> Artificial Sequence

<220>
<223> Detection oligonucleotide for HRAS

<400> 678

gaatattttg aagtttgt                                                    18

<210> 679
<211> 18
<212> DNA
<213> Artificial Sequence

<220>
<223> Detection oligonucleotide for IGF2

<400> 679

ggttattacg ataatttg                                                    18

<210> 680
<211> 18
<212> DNA
<213> Artificial Sequence

<220>
<223> Detection oligonucleotide for IGF2

<400> 680

ggttattatg ataatttg                                                    18

<210> 681
<211> 18
<212> DNA
<213> Artificial Sequence

<220>
<223> Detection oligonucleotide for IGF2

<400> 681

tggagtttac ggaggttt                                                    18

<210> 682
<211> 18
<212> DNA
<213> Artificial Sequence

<220>
<223> Detection oligonucleotide for IGF2

<400> 682

tggagtttat ggaggttt                                                    18

<210> 683
<211> 18
<212> DNA
<213> Artificial Sequence

<220>
<223> Detection oligonucleotide for LKB1

<400> 683

ttaattaacg ggtgggta                                                    18

<210> 684
<211> 18
<212> DNA
<213> Artificial Sequence

<220>
<223> Detection oligonucleotide for LKB1

<400> 684

ttaattaatg ggtgggta                                                    18

<210> 685
<211> 18
<212> DNA
<213> Artificial Sequence

<220>
<223> Detection oligonucleotide for MGMT

<400> 685

taaggatacg agttatat                                                    18

<210> 686
<211> 18
<212> DNA
<213> Artificial Sequence

<220>
<223> Detection oligonucleotide for MGMT

<400> 686

taaggatatg agttatat                                                    18

<210> 687
<211> 18
<212> DNA
<213> Artificial Sequence

<220>
<223> Detection oligonucleotide for MGMT

<400> 687

ttggagagcg gttgagtt                                                    18

<210> 688
<211> 18
<212> DNA
<213> Artificial Sequence

<220>
<223> Detection oligonucleotide for MGMT

<400> 688

ttggagagtg gttgagtt                                                    18

<210> 689
<211> 18
<212> DNA
<213> Artificial Sequence

<220>
<223> Detection oligonucleotide for MGMT

<400> 689

taggttatcg gtgattgt                                                    18

<210> 690
<211> 18
<212> DNA

<213> Artificial Sequence

<220>
<223> Detection oligonucleotide for MGMT

<400> 690

taggttattg gtgattgt                                                    18

<210> 691
<211> 18
<212> DNA
<213> Artificial Sequence

<220>
<223> Detection oligonucleotide for MGMT

<400> 691

taggggagcg gttttagg                                                    18

<210> 692
<211> 18
<212> DNA
<213> Artificial Sequence

<220>
<223> Detection oligonucleotide for MGMT

<400> 692

taggggagtg gttttagg                                                    18

<210> 693
<211> 18
<212> DNA
<213> Artificial Sequence

<220>
<223> Detection oligonucleotide for MLH1

<400> 693

ttgagaagcg ttaagtat                                                    18

<210> 694
<211> 18
<212> DNA
<213> Artificial Sequence

<220>
<223> Detection oligonucleotide for MLH1

<400> 694

ttgagaagtg ttaagtat                                                    18

<210> 695
<211> 18
<212> DNA
<213> Artificial Sequence

<220>

<223> Detection oligonucleotide for MLH1

<400> 695

gtagtagtcg ttttaggg                                                    18

<210> 696
<211> 18
<212> DNA
<213> Artificial Sequence

<220>
<223> Detection oligonucleotide for MLH1

<400> 696

gtagtagttg ttttaggg                                                    18

<210> 697
<211> 18
<212> DNA
<213> Artificial Sequence

<220>
<223> Detection oligonucleotide for MLH1

<400> 697

atagttgtcg ttgaaggg                                                    18

<210> 698
<211> 18
<212> DNA
<213> Artificial Sequence

<220>
<223> Detection oligonucleotide for MLH1

<400> 698

atagttgttg ttgaaggg                                                    18

<210> 699
<211> 18
<212> DNA
<213> Artificial Sequence

<220>
<223> Detection oligonucleotide for MLH1

<400> 699

ttggatggcg taagttat                                                    18

<210> 700
<211> 18
<212> DNA
<213> Artificial Sequence

<220>
<223> Detection oligonucleotide for MLH1

<400> 700

ttggatggtg taagttat 18

<210> 701
<211> 18
<212> DNA
<213> Artificial Sequence

<220>
<223> Detection oligonucleotide for MNCA9

<400> 701

taaaagggcg ttttgtga 18

<210> 702
<211> 18
<212> DNA
<213> Artificial Sequence

<220>
<223> Detection oligonucleotide for MNCA9

<400> 702

taaaagggtg ttttgtga 18

<210> 703
<211> 18
<212> DNA
<213> Artificial Sequence

<220>
<223> Detection oligonucleotide for MNCA9

<400> 703

tagttagtcg tatggttt 18

<210> 704
<211> 18
<212> DNA
<213> Artificial Sequence

<220>
<223> Detection oligonucleotide for MNCA9

<400> 704

tagttagttg tatggttt 18

<210> 705
<211> 18
<212> DNA
<213> Artificial Sequence

<220>
<223> Detection oligonucleotide for MNCA9

<400> 705

gatttattcg gagaggag 18

<210> 706
<211> 18
<212> DNA
<213> Artificial Sequence

<220>
<223> Detection oligonucleotide for MNCA9

<400> 706

gatttatttg gagaggag                                                    18

<210> 707
<211> 18
<212> DNA
<213> Artificial Sequence

<220>
<223> Detection oligonucleotide for MSH3

<400> 707

atttttcgtt cgatgata                                                    18

<210> 708
<211> 18
<212> DNA
<213> Artificial Sequence

<220>
<223> Detection oligonucleotide for MSH3

<400> 708
   attttttgtt tgatgata                                                     18

<210> 709
<211> 18
<212> DNA
<213> Artificial Sequence

<220>
<223> Detection oligonucleotide for MSH3

<400> 709

agtttagtcg gggttata                                                    18

<210> 710
<211> 18
<212> DNA
<213> Artificial Sequence

<220>
<223> Detection oligonucleotide for MSH3

<400> 710

agtttagttg gggttata                                                    18

<210> 711
<211> 18
<212> DNA

<213> Artificial Sequence

<220>
<223> Detection oligonucleotide for MSH3

<400> 711

gggtgaagcg ttgaggtt                                                    18

<210> 712
<211> 18
<212> DNA
<213> Artificial Sequence

<220>
<223> Detection oligonucleotide for MSH3

<400> 712

gggtgaagtg ttgaggtt                                                    18

<210> 713
<211> 18
<212> DNA
<213> Artificial Sequence

<220>
<223> Detection oligonucleotide for MYC

<400> 713

ttagagtgtt cggttgtt                                                    18

<210> 714
<211> 18
<212> DNA
<213> Artificial Sequence

<220>
<223> Detection oligonucleotide for MYC

<400> 714

ttagagtgtt tggttgtt                                                    18

<210> 715
<211> 18
<212> DNA
<213> Artificial Sequence

<220>
<223> Detection oligonucleotide for MYC

<400> 715

aggattttcg agttgtgt                                                    18

<210> 716
<211> 18
<212> DNA
<213> Artificial Sequence

<220>

<223> Detection oligonucleotide for MYC

<400> 716

aggattttg agttgtgt                                                    18

<210> 717
<211> 18
<212> DNA
<213> Artificial Sequence

<220>
<223> Detection oligonucleotide for MYC

<400> 717

aattttagcg agaggtag                                                   18

<210> 718
<211> 18
<212> DNA
<213> Artificial Sequence

<220>
<223> Detection oligonucleotide for MYC

<400> 718

aattttagtg agaggtag                                                   18

<210> 719
<211> 18
<212> DNA
<213> Artificial Sequence

<220>
<223> Detection oligonucleotide for PAX6

<400> 719

tattgtttcg gttgttag                                                   18

<210> 720
<211> 18
<212> DNA
<213> Artificial Sequence

<220>
<223> Detection oligonucleotide for PAX6

<400> 720

tattgttttg gttgttag                                                   18

<210> 721
<211> 18
<212> DNA
<213> Artificial Sequence

<220>
<223> Detection oligonucleotide for PAX6

<400> 721

gttagtagcg agtttagg                                                    18

<210> 722
<211> 18
<212> DNA
<213> Artificial Sequence

<220>
<223> Detection oligonucleotide for PAX6

<400> 722

gttagtagtg agtttagg                                                    18

<210> 723
<211> 18
<212> DNA
<213> Artificial Sequence

<220>
<223> Detection oligonucleotide for PAX6

<400> 723

agagtttagc gtattttt                                                    18

<210> 724
<211> 18
<212> DNA
<213> Artificial Sequence

<220>
<223> Detection oligonucleotide for PAX6

<400> 724

agagtttagt gtattttt                                                    18

<210> 725
<211> 18
<212> DNA
<213> Artificial Sequence

<220>
<223> Detection oligonucleotide for PGR

<400> 725

gaatttagcg agggattg                                                    18

<210> 726
<211> 18
<212> DNA
<213> Artificial Sequence

<220>
<223> Detection oligonucleotide for PGR

<400> 726

gaatttagtg agggattg                                                    18

<210> 727
<211> 18
<212> DNA
<213> Artificial Sequence

<220>
<223> Detection oligonucleotide for PGR

<400> 727

agtatgtacg agtttgat                                                    18

<210> 728
<211> 18
<212> DNA
<213> Artificial Sequence

<220>
<223> Detection oligonucleotide for PGR

<400> 728

agtatgtatg agtttgat                                                    18

<210> 729
<211> 18
<212> DNA
<213> Artificial Sequence

<220>
<223> Detection oligonucleotide for PGR

<400> 729

gggataaacg atagttat                                                    18

<210> 730
<211> 18
<212> DNA
<213> Artificial Sequence

<220>
<223> Detection oligonucleotide for PGR

<400> 730

gggataaatg atagttat                                                    18

<210> 731
<211> 18
<212> DNA
<213> Artificial Sequence

<220>
<223> Detection oligonucleotide for PTEN

<400> 731

agagtttgcg gtttgggg                                                    18

<210> 732
<211> 19
<212> DNA

<213> Artificial Sequence

<220>
<223> Detection oligonucleotide for PTEN

<400> 732

agagtttgtg gtttggggt                                                    19

<210> 733
<211> 18
<212> DNA
<213> Artificial Sequence

<220>
<223> Detection oligonucleotide for RARB

<400> 733

gggtttatcg aaagttta                                                     18

<210> 734
<211> 18
<212> DNA
<213> Artificial Sequence

<220>
<223> Detection oligonucleotide for RARB

<400> 734

gggtttattg aaagttta                                                     18

<210> 735
<211> 18
<212> DNA
<213> Artificial Sequence

<220>
<223> Detection oligonucleotide for RARB

<400> 735

agtttattcg tatatatt                                                     18

<210> 736
<211> 18
<212> DNA
<213> Artificial Sequence

<220>
<223> Detection oligonucleotide for RARB

<400> 736

agtttatttg tatatatt                                                     18

<210> 737
<211> 18
<212> DNA
<213> Artificial Sequence

<220>

<223> Detection oligonucleotide for RARB

<400> 737

tttttatgcg agttgttt                                                    18

<210> 738
<211> 18
<212> DNA
<213> Artificial Sequence

<220>
<223> Detection oligonucleotide for RARB

<400> 738

tttttatgtg agttgttt                                                    18

<210> 739
<211> 18
<212> DNA
<213> Artificial Sequence

<220>
<223> Detection oligonucleotide for SFN

<400> 739

atagagttcg gtattggt                                                    18

<210> 740
<211> 18
<212> DNA
<213> Artificial Sequence

<220>
<223> Detection oligonucleotide for SFN

<400> 740

atagagtttg gtattggt                                                    18

<210> 741
<211> 18
<212> DNA
<213> Artificial Sequence

<220>
<223> Detection oligonucleotide for SFN

<400> 741

gtaggtcgaa cgttatga                                                    18

<210> 742
<211> 18
<212> DNA
<213> Artificial Sequence

<220>
<223> Detection oligonucleotide for SFN

<400> 742

gtaggttgaa tgttatga 18

<210> 743
<211> 18
<212> DNA
<213> Artificial Sequence

<220>
<223> Detection oligonucleotide for SFN

<400> 743

aaaagtaacg aggagggt 18

<210> 744
<211> 18
<212> DNA
<213> Artificial Sequence

<220>
<223> Detection oligonucleotide for SFN

<400> 744

aaaagtaatg aggagggt 18

<210> 745
<211> 18
<212> DNA
<213> Artificial Sequence

<220>
<223> Detection oligonucleotide for S100A2

<400> 745

tttaattgcg gttgtgtg 18

<210> 746
<211> 18
<212> DNA
<213> Artificial Sequence

<220>
<223> Detection oligonucleotide for S100A2

<400> 746

tttaattgtg gttgtgtg 18

<210> 747
<211> 18
<212> DNA
<213> Artificial Sequence

<220>
<223> Detection oligonucleotide for S100A2

<400> 747

tatataggcg tatgtatg 18

<210> 748
<211> 18
<212> DNA
<213> Artificial Sequence

<220>
<223> Detection oligonucleotide for S100A2

<400> 748

tatataggtg tatgtatg                                                    18

<210> 749
<211> 18
<212> DNA
<213> Artificial Sequence

<220>
<223> Detection oligonucleotide for S100A2

<400> 749

agttttagcg tgtgttta                                                    18

<210> 750
<211> 18
<212> DNA
<213> Artificial Sequence

<220>
<223> Detection oligonucleotide for S100A2

<400> 750

agttttagtg tgtgttta                                                    18

<210> 751
<211> 18
<212> DNA
<213> Artificial Sequence

<220>
<223> Detection oligonucleotide for TGFBR2

<400> 751

atttggagcg aggaattt                                                    18

<210> 752
<211> 18
<212> DNA
<213> Artificial Sequence

<220>
<223> Detection oligonucleotide for TGFBR2

<400> 752

atttggagtg aggaattt                                                    18

<210> 753
<211> 18
<212> DNA

<213> Artificial Sequence

<220>
<223> Detection oligonucleotide for TGFBR2

<400> 753

ttgaaagtcg gttaaagt 18

<210> 754
<211> 18
<212> DNA
<213> Artificial Sequence

<220>
<223> Detection oligonucleotide for TGFBR2

<400> 754

ttgaaagttg gttaaagt 18

<210> 755
<211> 18
<212> DNA
<213> Artificial Sequence

<220>
<223> Detection oligonucleotide for TGFBR2

<400> 755

ggtagttacg agagagtt 18

<210> 756
<211> 18
<212> DNA
<213> Artificial Sequence

<220>
<223> Detection oligonucleotide for TGFBR2

<400> 756

ggtagttatg agagagtt 18

<210> 757
<211> 18
<212> DNA
<213> Artificial Sequence

<220>
<223> Detection oligonucleotide for TIMP3

<400> 757

aggtttttcg ttggagaa 18

<210> 758
<211> 18
<212> DNA
<213> Artificial Sequence

<220>

<223> Detection oligonucleotide for TIMP3

<400> 758

aggtttttg ttggagaa                                                    18

<210> 759
<211> 18
<212> DNA
<213> Artificial Sequence

<220>
<223> Detection oligonucleotide for TIMP3

<400> 759

gaaaatatcg gtattttg                                                   18

<210> 760
<211> 18
<212> DNA
<213> Artificial Sequence

<220>
<223> Detection oligonucleotide for TIMP3

<400> 760

gaaaatattg gtattttg                                                   18

<210> 761
<211> 18
<212> DNA
<213> Artificial Sequence

<220>
<223> Detection oligonucleotide for TIMP3

<400> 761

gggataagcg aatttttt                                                   18

<210> 762
<211> 18
<212> DNA
<213> Artificial Sequence

<220>
<223> Detection oligonucleotide for TIMP3

<400> 762

gggataagtg aatttttt                                                   18

<210> 763
<211> 18
<212> DNA
<213> Artificial Sequence

<220>
<223> Detection oligonucleotide for TIMP3

<400> 763

ttttattacg tatgtttt 18

<210> 764
<211> 18
<212> DNA
<213> Artificial Sequence

<220>
<223> Detection oligonucleotide for TIMP3

<400> 764

ttttattatg tatgtttt 18

<210> 765
<211> 18
<212> DNA
<213> Artificial Sequence

<220>
<223> Detection oligonucleotide for TP53

<400> 765

aagttgaacg tttaggta 18

<210> 766
<211> 18
<212> DNA
<213> Artificial Sequence

<220>
<223> Detection oligonucleotide for TP53

<400> 766

aagttgaatg tttaggta 18

<210> 767
<211> 18
<212> DNA
<213> Artificial Sequence

<220>
<223> Detection oligonucleotide for TP53

<400> 767

ttttgagtcg gtttaaag 18

<210> 768
<211> 18
<212> DNA
<213> Artificial Sequence

<220>
<223> Detection oligonucleotide for TP53

<400> 768

ttttgagttg gtttaaag 18

<210> 769
<211> 18
<212> DNA
<213> Artificial Sequence

<220>
<223> Detection oligonucleotide for TP53

<400> 769

tatttattcg gtgttggg                                                    18

<210> 770
<211> 18
<212> DNA
<213> Artificial Sequence

<220>
<223> Detection oligonucleotide for TP53

<400> 770

tatttatttg gtgttggg                                                    18

<210> 771
<211> 18
<212> DNA
<213> Artificial Sequence

<220>
<223> Detection oligonucleotide for TP53

<400> 771

ttggatttcg aaatattg                                                    18

<210> 772
<211> 18
<212> DNA
<213> Artificial Sequence

<220>
<223> Detection oligonucleotide for TP53

<400> 772

ttggattttg aaatattg                                                    18

<210> 773
<211> 18
<212> DNA
<213> Artificial Sequence

<220>
<223> Detection oligonucleotide for TP73

<400> 773

tgatttagcg taggtttg                                                    18

<210> 774
<211> 18
<212> DNA

<213> Artificial Sequence

<220>
<223> Detection oligonucleotide for TP73

<400> 774

tgatttagtg taggtttg                                                    18

<210> 775
<211> 18
<212> DNA
<213> Artificial Sequence

<220>
<223> Detection oligonucleotide for TP73

<400> 775

ttagagttcg agtttata                                                    18

<210> 776
<211> 18
<212> DNA
<213> Artificial Sequence

<220>
<223> Detection oligonucleotide for TP73

<400> 776

ttagagtttg agtttata                                                    18

<210> 777
<211> 18
<212> DNA
<213> Artificial Sequence

<220>
<223> Detection oligonucleotide for TP73

<400> 777

ggaagtttcg atggttta                                                    18

<210> 778
<211> 18
<212> DNA
<213> Artificial Sequence

<220>
<223> Detection oligonucleotide for TP73

<400> 778

ggaagttttg atggttta                                                    18

<210> 779
<211> 18
<212> DNA
<213> Artificial Sequence

<220>

<223> Detection oligonucleotide for VHL

<400> 779

tttataagcg tgatgatt                                                    18

<210> 780
<211> 18
<212> DNA
<213> Artificial Sequence

<220>
<223> Detection oligonucleotide for VHL

<400> 780

tttataagtg tgatgatt                                                    18

<210> 781
<211> 18
<212> DNA
<213> Artificial Sequence

<220>
<223> Detection oligonucleotide for VHL

<400> 781

ggtgttttcg tgtgagat                                                    18

<210> 782
<211> 18
<212> DNA
<213> Artificial Sequence

<220>
<223> Detection oligonucleotide for VHL

<400> 782
   ggtgttttgg tgtgagat                                                     18

<210> 783
<211> 18
<212> DNA
<213> Artificial Sequence

<220>
<223> Detection oligonucleotide for VHL

<400> 783

tgtgagatgc gttatttt                                                    18

<210> 784
<211> 18
<212> DNA
<213> Artificial Sequence

<220>
<223> Detection oligonucleotide for VHL

<400> 784

tgtgagatgt gttatttt                                                    18

<210> 785
<211> 18
<212> DNA
<213> Artificial Sequence

<220>
<223> Detection oligonucleotide for VHL

<400> 785

tatattgcgc gtttgata                                                    18

<210> 786
<211> 18
<212> DNA
<213> Artificial Sequence

<220>
<223> Detection oligonucleotide for VHL

<400> 786

tatattgtgt gtttgata                                                    18

<210> 787
<211> 18
<212> DNA
<213> Artificial Sequence

<220>
<223> Detection oligonucleotide for CDKN1C

<400> 787

atgaagaacg gttaaggg                                                    18

<210> 788
<211> 18
<212> DNA
<213> Artificial Sequence

<220>
<223> Detection oligonucleotide for CDKN1C

<400> 788

atgaagaatg gttaaggg                                                    18

<210> 789
<211> 18
<212> DNA
<213> Artificial Sequence

<220>
<223> Detection oligonucleotide for CDKN1C

<400> 789

ttttatttcg agttaggt                                                    18

<210> 790
<211> 18
<212> DNA
<213> Artificial Sequence

<220>
<223> Detection oligonucleotide for CDKN1C

<400> 790

ttttattttg agttaggt                                                    18

<210> 791
<211> 18
<212> DNA
<213> Artificial Sequence

<220>
<223> Detection oligonucleotide for CDKN1C

<400> 791

ttaagttacg gttattag                                                    18

<210> 792
<211> 18
<212> DNA
<213> Artificial Sequence

<220>
<223> Detection oligonucleotide for CDKN1C

<400> 792

ttaagttatg gttattag                                                    18

<210> 793
<211> 18
<212> DNA
<213> Artificial Sequence

<220>
<223> Detection oligonucleotide for CDKN1C

<400> 793

ttagtgttcg tttggaat                                                    18

<210> 794
<211> 18
<212> DNA
<213> Artificial Sequence

<220>
<223> Detection oligonucleotide for CDKN1C

<400> 794

ttagtgtttg tttggaat                                                    18

<210> 795
<211> 18
<212> DNA

<213> Artificial Sequence

<220>
<223> Detection oligonucleotide for CAV1

<400> 795

ttggtatcgt tgaagaat                                                    18

<210> 796
<211> 18
<212> DNA
<213> Artificial Sequence

<220>
<223> Detection oligonucleotide for CAV1

<400> 796

ttggtattgt tgaagaat                                                    18

<210> 797
<211> 18
<212> DNA
<213> Artificial Sequence

<220>
<223> Detection oligonucleotide for CAV1

<400> 797

tgggggttcg aaaaagtg                                                    18

<210> 798
<211> 18
<212> DNA
<213> Artificial Sequence

<220>
<223> Detection oligonucleotide for CAV1

<400> 798

tgggggtttg aaaaagtg                                                    18

<210> 799
<211> 18
<212> DNA
<213> Artificial Sequence

<220>
<223> Detection oligonucleotide for CAV1

<400> 799

gaagtgttcg tttttgtt                                                    18

<210> 800
<211> 18
<212> DNA
<213> Artificial Sequence

<220>

<223> Detection oligonucleotide for CAV1

<400> 800

gaagtgtttg tttttgtt                                                    18

<210> 801
<211> 18
<212> DNA
<213> Artificial Sequence

<220>
<223> Detection oligonucleotide for CDH13

<400> 801

gaagtggtcg ttagtttt                                                    18

<210> 802
<211> 19
<212> DNA
<213> Artificial Sequence

<220>
<223> Detection oligonucleotide for CDH13

<400> 802

gaagtggttg ttagttttt                                                   19

<210> 803
<211> 18
<212> DNA
<213> Artificial Sequence

<220>
<223> Detection oligonucleotide for CDH13

<400> 803

ttgtttagcg tgatttgt                                                    18

<210> 804
<211> 18
<212> DNA
<213> Artificial Sequence

<220>
<223> Detection oligonucleotide for CDH13

<400> 804

ttgtttagtg tgatttgt                                                    18

<210> 805
<211> 18
<212> DNA
<213> Artificial Sequence

<220>
<223> Detection oligonucleotide for DRG1

<400> 805

gagtaggacg gtgttaag 18

<210> 806
<211> 18
<212> DNA
<213> Artificial Sequence

<220>
<223> Detection oligonucleotide for DRG1

<400> 806

gagtaggatg gtgttaag 18

<210> 807
<211> 18
<212> DNA
<213> Artificial Sequence

<220>
<223> Detection oligonucleotide for DRG1

<400> 807

aaatttaacg ttgggtag 18

<210> 808
<211> 18
<212> DNA
<213> Artificial Sequence

<220>
<223> Detection oligonucleotide for DRG1

<400> 808

aaatttaatg ttgggtag 18

<210> 809
<211> 18
<212> DNA
<213> Artificial Sequence

<220>
<223> Detection oligonucleotide for DRG1

<400> 809

gataatgacg gtgttagt 18

<210> 810
<211> 18
<212> DNA
<213> Artificial Sequence

<220>
<223> Detection oligonucleotide for DRG1

<400> 810

gataatgatg gtgttagt 18

<210> 811
<211> 18
<212> DNA
<213> Artificial Sequence

<220>
<223> Detection oligonucleotide for DRG1

<400> 811

tggttgtacg ttaggagt                                                    18

<210> 812
<211> 18
<212> DNA
<213> Artificial Sequence

<220>
<223> Detection oligonucleotide for DRG1

<400> 812

tggttgtatg ttaggagt                                                    18

<210> 813
<211> 18
<212> DNA
<213> Artificial Sequence

<220>
<223> Detection oligonucleotide for PTGS2

<400> 813

atttaaggcg attagttt                                                    18

<210> 814
<211> 18
<212> DNA
<213> Artificial Sequence

<220>
<223> Detection oligonucleotide for PTGS2

<400> 814

atttaaggtg attagttt                                                    18

<210> 815
<211> 18
<212> DNA
<213> Artificial Sequence

<220>
<223> Detection oligonucleotide for THBS1

<400> 815

ggagagttag cgagggtt                                                    18

<210> 816
<211> 18
<212> DNA

<213> Artificial Sequence

<220>
<223> Detection oligonucleotide for THBS1

<400> 816

ggagagttag tgagggtt 18

<210> 817
<211> 18
<212> DNA
<213> Artificial Sequence

<220>
<223> Detection oligonucleotide for THBS1

<400> 817

tattttaacg aatggttt 18

<210> 818
<211> 18
<212> DNA
<213> Artificial Sequence

<220>
<223> Detection oligonucleotide for THBS1

<400> 818

tattttaatg aatggttt 18

<210> 819
<211> 18
<212> DNA
<213> Artificial Sequence

<220>
<223> Detection oligonucleotide for THBS1

<400> 819

ttataaaacg ggtttagt 18

<210> 820
<211> 18
<212> DNA
<213> Artificial Sequence

<220>
<223> Detection oligonucleotide for THBS1

<400> 820

ttataaaatg ggtttagt 18

<210> 821
<211> 18
<212> DNA
<213> Artificial Sequence

<220>

<223> Detection oligonucleotide for THBS1

<400> 821

aggtatttcg ggagatta                                                    18

<210> 822
<211> 18
<212> DNA
<213> Artificial Sequence

<220>
<223> Detection oligonucleotide for THBS1

<400> 822

aggtattttg ggagatta                                                    18

<210> 823
<211> 18
<212> DNA
<213> Artificial Sequence

<220>
<223> Detection oligonucleotide for DNMT1

<400> 823

agtgggttcg tttaagtt                                                    18

<210> 824
<211> 18
<212> DNA
<213> Artificial Sequence

<220>
<223> Detection oligonucleotide for DNMT1

<400> 824

agtgggtttg tttaagtt                                                    18

<210> 825
<211> 18
<212> DNA
<213> Artificial Sequence

<220>
<223> Detection oligonucleotide for DNMT1

<400> 825

ttttttacgc ggagtagt                                                    18

<210> 826
<211> 18
<212> DNA
<213> Artificial Sequence

<220>
<223> Detection oligonucleotide for DNMT1

<400> 826

tttttatgt ggagtagt                                                    18

<210> 827
<211> 18
<212> DNA
<213> Artificial Sequence

<220>
<223> Detection oligonucleotide for DNMT1

<400> 827

gagagaggcg atattttg                                                    18

<210> 828
<211> 18
<212> DNA
<213> Artificial Sequence

<220>
<223> Detection oligonucleotide for DNMT1

<400> 828

gagagaggtg atattttg                                                    18

<210> 829
<211> 18
<212> DNA
<213> Artificial Sequence

<220>
<223> Detection oligonucleotide for DNMT1

<400> 829

gtattaaacg gagagagg                                                    18

<210> 830
<211> 18
<212> DNA
<213> Artificial Sequence

<220>
<223> Detection oligonucleotide for DNMT1

<400> 830

gtattaaatg gagagagg                                                    18

<210> 831
<211> 18
<212> DNA
<213> Artificial Sequence

<220>
<223> Detection oligonucleotide for CEA

<400> 831

ttttgagtcg tagtttag                                                    18

<210> 832
<211> 18
<212> DNA
<213> Artificial Sequence

<220>
<223> Detection oligonucleotide for CEA

<400> 832

ttttgagttg tagtttag                                                    18

<210> 833
<211> 18
<212> DNA
<213> Artificial Sequence

<220>
<223> Detection oligonucleotide for CEA

<400> 833

aatagatacg gagaggga                                                    18

<210> 834
<211> 18
<212> DNA
<213> Artificial Sequence

<220>
<223> Detection oligonucleotide for CEA

<400> 834

aatagatatg gagaggga                                                    18

<210> 835
<211> 18
<212> DNA
<213> Artificial Sequence

<220>
<223> Detection oligonucleotide for MB

<400> 835

agaaggtgcg tgagaggt                                                    18

<210> 836
<211> 18
<212> DNA
<213> Artificial Sequence

<220>
<223> Detection oligonucleotide for MB

<400> 836

agaaggtgtg tgagaggt                                                    18

<210> 837
<211> 18
<212> DNA

<213> Artificial Sequence

<220>
<223> Detection oligonucleotide for MB

<400> 837

gggttagtcg gggtattt 18

<210> 838
<211> 18
<212> DNA
<213> Artificial Sequence

<220>
<223> Detection oligonucleotide for MB

<400> 838

gggttagttg gggtattt 18

<210> 839
<211> 18
<212> DNA
<213> Artificial Sequence

<220>
<223> Detection oligonucleotide for MB

<400> 839

ggggatagcg agttattg 18

<210> 840
<211> 18
<212> DNA
<213> Artificial Sequence

<220>
<223> Detection oligonucleotide for MB

<400> 840

ggggatagtg agttattg 18

<210> 841
<211> 18
<212> DNA
<213> Artificial Sequence

<220>
<223> Detection oligonucleotide for MB

<400> 841

ttagattgcg ttatgggg 18

<210> 842
<211> 18
<212> DNA
<213> Artificial Sequence

<220>

<223> Detection oligonucleotide for MB

<400> 842

ttagattgtg ttatgggg                                                    18

<210> 843
<211> 18
<212> DNA
<213> Artificial Sequence

<220>
<223> Detection oligonucleotide for PCNA

<400> 843

tatggatacg attggttt                                                    18

<210> 844
<211> 18
<212> DNA
<213> Artificial Sequence

<220>
<223> Detection oligonucleotide for PCNA

<400> 844

tatggatatg attggttt                                                    18

<210> 845
<211> 18
<212> DNA
<213> Artificial Sequence

<220>
<223> Detection oligonucleotide for PCNA

<400> 845

gtattaaacg gttgtagg                                                    18

<210> 846
<211> 18
<212> DNA
<213> Artificial Sequence

<220>
<223> Detection oligonucleotide for PCNA

<400> 846

gtattaaatg gttgtagg                                                    18

<210> 847
<211> 18
<212> DNA
<213> Artificial Sequence

<220>
<223> Detection oligonucleotide for PCNA

<400> 847

tttgaagtcg aaattagt                                                    18

<210> 848
<211> 18
<212> DNA
<213> Artificial Sequence

<220>
<223> Detection oligonucleotide for PCNA

<400> 848

tttgaagttg aaattagt                                                    18

<210> 849
<211> 18
<212> DNA
<213> Artificial Sequence

<220>
<223> Detection oligonucleotide for CDC2

<400> 849

tggaatttcg atgtaaat                                                    18

<210> 850
<211> 18
<212> DNA
<213> Artificial Sequence

<220>
<223> Detection oligonucleotide for CDC2

<400> 850

tggaattttg atgtaaat                                                    18

<210> 851
<211> 18
<212> DNA
<213> Artificial Sequence

<220>
<223> Detection oligonucleotide for CDC2

<400> 851

tagtaggacg atattttt                                                    18

<210> 852
<211> 18
<212> DNA
<213> Artificial Sequence

<220>
<223> Detection oligonucleotide for CDC2

<400> 852

tagtaggatg atattttt                                                    18

<210> 853
<211> 18
<212> DNA
<213> Artificial Sequence

<220>
<223> Detection oligonucleotide for CDC2

<400> 853

tagttattcg ggaaggtt                                                    18

<210> 854
<211> 18
<212> DNA
<213> Artificial Sequence

<220>
<223> Detection oligonucleotide for CDC2

<400> 854

tagttatttg ggaaggtt                                                    18

<210> 855
<211> 18
<212> DNA
<213> Artificial Sequence

<220>
<223> Detection oligonucleotide for CDC2

<400> 855

aaattgttcg tatttggt                                                    18

<210> 856
<211> 18
<212> DNA
<213> Artificial Sequence

<220>
<223> Detection oligonucleotide for CDC2

<400> 856

aaattgtttg tatttggt                                                    18

<210> 857
<211> 18
<212> DNA
<213> Artificial Sequence

<220>
<223> Detection oligonucleotide for ESR1

<400> 857

agatatatcg gagtttgg                                                    18

<210> 858
<211> 18
<212> DNA

<213> Artificial Sequence

<220>
<223> Detection oligonucleotide for ESR1

<400> 858

agatatattg gagtttgg                                                    18

<210> 859
<211> 18
<212> DNA
<213> Artificial Sequence

<220>
<223> Detection oligonucleotide for ESR1

<400> 859

gtttggtacg gggtatat                                                    18

<210> 860
<211> 18
<212> DNA
<213> Artificial Sequence

<220>
<223> Detection oligonucleotide for ESR1

<400> 860

gtttggtatg gggtatat                                                    18

<210> 861
<211> 18
<212> DNA
<213> Artificial Sequence

<220>
<223> Detection oligonucleotide for ESR1

<400> 861

ttttaaatcg agttgtgt                                                    18

<210> 862
<211> 18
<212> DNA
<213> Artificial Sequence

<220>
<223> Detection oligonucleotide for ESR1

<400> 862

ttttaaattg agttgtgt                                                    18

<210> 863
<211> 18
<212> DNA
<213> Artificial Sequence

<220>

<223> Detection oligonucleotide for ESR1

<400> 863

tatgagttcg ggagatta                                                    18

<210> 864
<211> 18
<212> DNA
<213> Artificial Sequence

<220>
<223> Detection oligonucleotide for ESR1

<400> 864

tatgagtttg ggagatta                                                    18

<210> 865
<211> 18
<212> DNA
<213> Artificial Sequence

<220>
<223> Detection oligonucleotide for CASP8

<400> 865

gaatgagtcg aggaaggt                                                    18

<210> 866
<211> 18
<212> DNA
<213> Artificial Sequence

<220>
<223> Detection oligonucleotide for CASP8

<400> 866

gaatgagttg aggaaggt                                                    18

<210> 867
<211> 18
<212> DNA
<213> Artificial Sequence

<220>
<223> Detection oligonucleotide for CASP8

<400> 867

tattgagacg ttaagtaa                                                    18

<210> 868
<211> 18
<212> DNA
<213> Artificial Sequence

<220>
<223> Detection oligonucleotide for CASP8

<400> 868

tattgagatg ttaagtaa 18

<210> 869
<211> 18
<212> DNA
<213> Artificial Sequence

<220>
<223> Detection oligonucleotide for CASP8

<400> 869

taaggttacg tagttagt 18

<210> 870
<211> 18
<212> DNA
<213> Artificial Sequence

<220>
<223> Detection oligonucleotide for CASP8

<400> 870

taaggttatg tagttagt 18

<210> 871
<211> 18
<212> DNA
<213> Artificial Sequence

<220>
<223> Detection oligonucleotide for CASP8

<400> 871

gttaatagcg gggatttt 18

<210> 872
<211> 18
<212> DNA
<213> Artificial Sequence

<220>
<223> Detection oligonucleotide for CASP8

<400> 872

gttaatagtg gggatttt 18

<210> 873
<211> 18
<212> DNA
<213> Artificial Sequence

<220>
<223> Detection oligonucleotide for RASSF1

<400> 873

gtagttttcg agaatgtt 18

<210> 874
<211> 18
<212> DNA
<213> Artificial Sequence

<220>
<223> Detection oligonucleotide for RASSF1

<400> 874

gtagtttttg agaatgtt                                                    18

<210> 875
<211> 18
<212> DNA
<213> Artificial Sequence

<220>
<223> Detection oligonucleotide for RASSF1

<400> 875

taattagaac gttttttg                                                    18

<210> 876
<211> 18
<212> DNA
<213> Artificial Sequence

<220>
<223> Detection oligonucleotide for RASSF1

<400> 876

taattagaat gttttttg                                                    18

<210> 877
<211> 18
<212> DNA
<213> Artificial Sequence

<220>
<223> Detection oligonucleotide for RASSF1

<400> 877

tagttttcgc gtagaatt                                                    18

<210> 878
<211> 18
<212> DNA
<213> Artificial Sequence

<220>
<223> Detection oligonucleotide for RASSF1

<400> 878

tagtttttgt gtagaatt                                                    18

<210> 879
<211> 18
<212> DNA

<213> Artificial Sequence

<220>
<223> Detection oligonucleotide for MSH4

<400> 879

ttgtttcggc ggtttttt                                                    18

<210> 880
<211> 18
<212> DNA
<213> Artificial Sequence

<220>
<223> Detection oligonucleotide for MSH4

<400> 880

ttgttttggt ggtttttt                                                    18

<210> 881
<211> 18
<212> DNA
<213> Artificial Sequence

<220>
<223> Detection oligonucleotide for MSH4

<400> 881

ttttggtacg ttaggagt                                                    18

<210> 882
<211> 18
<212> DNA
<213> Artificial Sequence

<220>
<223> Detection oligonucleotide for MSH4

<400> 882

ttttggtatg ttaggagt                                                    18

<210> 883
<211> 18
<212> DNA
<213> Artificial Sequence

<220>
<223> Detection oligonucleotide for MSH4

<400> 883

taaattttcg gttagttt                                                    18

<210> 884
<211> 18
<212> DNA
<213> Artificial Sequence

<220>

<223> Detection oligonucleotide for MSH4

<400> 884

taaatttttg gttagttt                                                    18

<210> 885
<211> 18
<212> DNA
<213> Artificial Sequence

<220>
<223> Detection oligonucleotide for MSH4

<400> 885

ttagaggtcg gtagttta                                                    18

<210> 886
<211> 18
<212> DNA
<213> Artificial Sequence

<220>
<223> Detection oligonucleotide for MSH4

<400> 886

ttagaggttg gtagttta                                                    18

<210> 887
<211> 18
<212> DNA
<213> Artificial Sequence

<220>
<223> Detection oligonucleotide for MSH5

<400> 887

atgtttatcg ttttgagt                                                    18

<210> 888
<211> 18
<212> DNA
<213> Artificial Sequence

<220>
<223> Detection oligonucleotide for MSH5

<400> 888

atgtttattg ttttgagt                                                    18

<210> 889
<211> 18
<212> DNA
<213> Artificial Sequence

<220>
<223> Detection oligonucleotide for MSH5

<400> 889

atagttgtcg aatgtatg                                                    18

<210> 890
<211> 18
<212> DNA
<213> Artificial Sequence

<220>
<223> Detection oligonucleotide for MSH5

<400> 890

atagttgttg aatgtatg                                                    18

<210> 891
<211> 18
<212> DNA
<213> Artificial Sequence

<220>
<223> Detection oligonucleotide for MSH5

<400> 891

tagaagtgcg aaggggta                                                    18

<210> 892
<211> 18
<212> DNA
<213> Artificial Sequence

<220>
<223> Detection oligonucleotide for MSH5

<400> 892

tagaagtgtg aaggggta                                                    18

<210> 893
<211> 18
<212> DNA
<213> Artificial Sequence

<220>
<223> Detection oligonucleotide for MSH5

<400> 893

atgtaattcg aatgtttt                                                    18

<210> 894
<211> 18
<212> DNA
<213> Artificial Sequence

<220>
<223> Detection oligonucleotide for MSH5

<400> 894

atgtaatttg aatgtttt                                                    18

<210> 895
<211> 18
<212> DNA
<213> Artificial Sequence

<220>
<223> Detection oligonucleotide for CDH13

<400> 895

aatgttttcg tgatgttg                                                    18

<210> 896
<211> 18
<212> DNA
<213> Artificial Sequence

<220>
<223> Detection oligonucleotide for CDH13

<400> 896

aatgttttg tgatgttg                                                     18

<210> 897
<211> 18
<212> DNA
<213> Artificial Sequence

<220>
<223> Detection oligonucleotide for CD44

<400> 897

ttgtttagcg gattttag                                                    18

<210> 898
<211> 18
<212> DNA
<213> Artificial Sequence

<220>
<223> Detection oligonucleotide for CD44

<400> 898

ttgtttagtg gattttag                                                    18

<210> 899
<211> 18
<212> DNA
<213> Artificial Sequence

<220>
<223> Detection oligonucleotide for TPEF (=TMEFF2; =HPP1)

<400> 899

aatttgcgaa cgtttggg                                                    18

<210> 900
<211> 18
<212> DNA

<213> Artificial Sequence

<220>
<223> Detection oligonucleotide for TPEF (=TMEFF2; =HPP1)

<400> 900

aatttgtgaa tgtttggg 18

<210> 901
<211> 18
<212> DNA
<213> Artificial Sequence

<220>
<223> Detection oligonucleotide for CSPG2

<400> 901

gggtaacgtc gaatttag 18

<210> 902
<211> 18
<212> DNA
<213> Artificial Sequence

<220>
<223> Detection oligonucleotide for CSPG2

<400> 902

gggtaatgtt gaatttag 18

<210> 903
<211> 18
<212> DNA
<213> Artificial Sequence

<220>
<223> Detection oligonucleotide for CDH13

<400> 903

aaggaattcg ttttgtaa 18

<210> 904
<211> 18
<212> DNA
<213> Artificial Sequence

<220>
<223> Detection oligonucleotide for CDH13

<400> 904

aaggaatttg ttttgtaa 18

<210> 905
<211> 18
<212> DNA
<213> Artificial Sequence

<220>

<223> Detection oligonucleotide for GSTP1

<400> 905

ggagttcgcg ggattttt                                                    18

<210> 906
<211> 18
<212> DNA
<213> Artificial Sequence

<220>
<223> Detection oligonucleotide for GSTP1

<400> 906

ggagtttgtg ggattttt                                                    18

<210> 907
<211> 18
<212> DNA
<213> Artificial Sequence

<220>
<223> Detection oligonucleotide for TGFBR2

<400> 907

aaagttttcg gaggggtt                                                    18

<210> 908
<211> 18
<212> DNA
<213> Artificial Sequence

<220>
<223> Detection oligonucleotide for TGFBR2

<400> 908
   aaagttttgg gaggggtt                                                     18

<210> 909
<211> 18
<212> DNA
<213> Artificial Sequence

<220>
<223> Detection oligonucleotide for N33

<400> 909

tgttatttcg gagggttt                                                    18

<210> 910
<211> 18
<212> DNA
<213> Artificial Sequence

<220>
<223> Detection oligonucleotide for N33

<400> 910

tgttattttg gagggttt                                                    18

<210> 911
<211> 18
<212> DNA
<213> Artificial Sequence

<220>
<223> Detection oligonucleotide for CAV1

<400> 911

tagattcgga ggtaggta                                                    18

<210> 912
<211> 18
<212> DNA
<213> Artificial Sequence

<220>
<223> Detection oligonucleotide for CAV1

<400> 912

tagatttgga ggtaggta                                                    18

<210> 913
<211> 18
<212> DNA
<213> Artificial Sequence

<220>
<223> Detection oligonucleotide for PTGS2

<400> 913

tttattttcg tgggtaaa                                                    18

<210> 914
<211> 18
<212> DNA
<213> Artificial Sequence

<220>
<223> Detection oligonucleotide for PTGS2

<400> 914

tttattttg tgggtaaa                                                     18

<210> 915
<211> 18
<212> DNA
<213> Artificial Sequence

<220>
<223> Detection oligonucleotide for TP73

<400> 915

aagttacggg ttttattg                                                    18

<210> 916
<211> 18
<212> DNA
<213> Artificial Sequence

<220>
<223> Detection oligonucleotide for TP73

<400> 916

aagttatggg ttttattg                                                    18

<210> 917
<211> 18
<212> DNA
<213> Artificial Sequence

<220>
<223> Detection oligonucleotide for TPEF (=TMEFF2; =HPP1)

<400> 917

ataggttacg ggttggag                                                    18

<210> 918
<211> 18
<212> DNA
<213> Artificial Sequence

<220>
<223> Detection oligonucleotide for TPEF (=TMEFF2; =HPP1)

<400> 918

ataggttatg ggttggag                                                    18

<210> 919
<211> 18
<212> DNA
<213> Artificial Sequence

<220>
<223> Detection oligonucleotide for CD44

<400> 919

gtggggttcg gaggtata                                                    18

<210> 920
<211> 18
<212> DNA
<213> Artificial Sequence

<220>
<223> Detection oligonucleotide for CD44

<400> 920

gtggggtttg gaggtata                                                    18

<210> 921
<211> 18
<212> DNA

<213> Artificial Sequence

<220>
<223> Detection oligonucleotide for ERBB2

<400> 921

aagtaagtcg ttgttgtt                                                    18

<210> 922
<211> 18
<212> DNA
<213> Artificial Sequence

<220>
<223> Detection oligonucleotide for ERBB2

<400> 922

aagtaagttg ttgttgtt                                                    18

<210> 923
<211> 18
<212> DNA
<213> Artificial Sequence

<220>
<223> Detection oligonucleotide for ERBB2

<400> 923

agtgtatgcg tagaaggt                                                    18

<210> 924
<211> 18
<212> DNA
<213> Artificial Sequence

<220>
<223> Detection oligonucleotide for ERBB2

<400> 924

agtgtatgtg tagaaggt                                                    18

<210> 925
<211> 18
<212> DNA
<213> Artificial Sequence

<220>
<223> Detection oligonucleotide for GTBP/MSH6

<400> 925

tttgttggcg ggaaattt                                                    18

<210> 926
<211> 18
<212> DNA
<213> Artificial Sequence

<220>

<223> Detection oligonucleotide for GTBP/MSH6

<400> 926

tttgttggtg ggaaattt                                                    18

<210> 927
<211> 18
<212> DNA
<213> Artificial Sequence

<220>
<223> Detection oligonucleotide for EGR4

<400> 927

ggagttttcg gtatatat                                                    18

<210> 928
<211> 18
<212> DNA
<213> Artificial Sequence

<220>
<223> Detection oligonucleotide for EGR4

<400> 928

ggagtttttg gtatatat                                                    18

<210> 929
<211> 18
<212> DNA
<213> Artificial Sequence

<220>
<223> Detection oligonucleotide for CDH1

<400> 929

tagtggcgtc ggaattgt                                                    18

<210> 930
<211> 18
<212> DNA
<213> Artificial Sequence

<220>
<223> Detection oligonucleotide for CDH1

<400> 930

tagtggtgtt ggaattgt                                                    18

<210> 931
<211> 18
<212> DNA
<213> Artificial Sequence

<220>
<223> Detection oligonucleotide for EGFR

<400> 931

atttggttcg atttggat                                                    18

<210> 932
<211> 18
<212> DNA
<213> Artificial Sequence

<220>
<223> Detection oligonucleotide for EGFR

<400> 932

atttggtttg atttggat                                                    18

<210> 933
<211> 18
<212> DNA
<213> Artificial Sequence

<220>
<223> Detection oligonucleotide for LKB1

<400> 933

agggaggtcg ttggtatt                                                    18

<210> 934
<211> 18
<212> DNA
<213> Artificial Sequence

<220>
<223> Detection oligonucleotide for LKB1

<400> 934

agggaggttg ttggtatt                                                    18

<210> 935
<211> 18
<212> DNA
<213> Artificial Sequence

<220>
<223> Detection oligonucleotide for DAPK1

<400> 935

ttgtttttcg gaaatttg                                                    18

<210> 936
<211> 18
<212> DNA
<213> Artificial Sequence

<220>
<223> Detection oligonucleotide for DAPK1

<400> 936

ttgtttttg gaaatttg                                                     18

<210> 937
<211> 18
<212> DNA
<213> Artificial Sequence

<220>
<223> Detection oligonucleotide for IGF2

<400> 937

gattagggcg ggaaatat                                                    18

<210> 938
<211> 18
<212> DNA
<213> Artificial Sequence

<220>
<223> Detection oligonucleotide for IGF2

<400> 938

gattagggtg ggaaatat                                                    18

<210> 939
<211> 18
<212> DNA
<213> Artificial Sequence

<220>
<223> Detection oligonucleotide for HLA-F

<400> 939

tatttgggcg ggtgagtg                                                    18

<210> 940
<211> 18
<212> DNA
<213> Artificial Sequence

<220>
<223> Detection oligonucleotide for HLA-F

<400> 940

tatttgggtg ggtgagtg                                                    18

<210> 941
<211> 18
<212> DNA
<213> Artificial Sequence

<220>
<223> Detection oligonucleotide for IGF2

<400> 941

ttgtatggtc gagtttat                                                    18

<210> 942
<211> 18
<212> DNA

<213> Artificial Sequence

<220>
<223> Detection oligonucleotide for IGF2

<400> 942

ttgtatggtt gagtttat 18

<210> 943
<211> 18
<212> DNA
<213> Artificial Sequence

<220>
<223> Detection oligonucleotide for N33

<400> 943

gtttagttag cgggtttt 18

<210> 944
<211> 18
<212> DNA
<213> Artificial Sequence

<220>
<223> Detection oligonucleotide for N33

<400> 944

gtttagttag tgggtttt 18

<210> 945
<211> 18
<212> DNA
<213> Artificial Sequence

<220>
<223> Detection oligonucleotide for CSPG2

<400> 945

aaaaattcgc gagtttag 18

<210> 946
<211> 18
<212> DNA
<213> Artificial Sequence

<220>
<223> Detection oligonucleotide for CSPG2

<400> 946

aaaaatttgt gagtttag 18

<210> 947
<211> 18
<212> DNA
<213> Artificial Sequence

<220>

<223> Detection oligonucleotide for PTEN

<400> 947

tgatgtggcg ggattttt                                                    18

<210> 948
<211> 18
<212> DNA
<213> Artificial Sequence

<220>
<223> Detection oligonucleotide for PTEN

<400> 948

tgatgtggtg ggattttt                                                    18

<210> 949
<211> 18
<212> DNA
<213> Artificial Sequence

<220>
<223> Detection oligonucleotide for MLH1

<400> 949

ttaggtagcg ggtagtag                                                    18

<210> 950
<211> 18
<212> DNA
<213> Artificial Sequence

<220>
<223> Detection oligonucleotide for MLH1

<400> 950

ttaggtagtg ggtagtag                                                    18

<210> 951
<211> 18
<212> DNA
<213> Artificial Sequence

<220>
<223> Detection oligonucleotide for GTBP/MSH6

<400> 951

gaggaattcg ggttttag                                                    18

<210> 952
<211> 18
<212> DNA
<213> Artificial Sequence

<220>
<223> Detection oligonucleotide for GTBP/MSH6

<400> 952

gaggaatttg ggttttag                                                    18

<210> 953
<211> 18
<212> DNA
<213> Artificial Sequence

<220>
<223> Detection oligonucleotide for CALCA

<400> 953

attaggttcg tgttttag                                                    18

<210> 954
<211> 18
<212> DNA
<213> Artificial Sequence

<220>
<223> Detection oligonucleotide for CALCA

<400> 954

attaggtttg tgttttag                                                    18

<210> 955
<211> 18
<212> DNA
<213> Artificial Sequence

<220>
<223> Detection oligonucleotide for DAPK1

<400> 955

gaagggagcg tattttat                                                    18

<210> 956
<211> 18
<212> DNA
<213> Artificial Sequence

<220>
<223> Detection oligonucleotide for DAPK1

<400> 956

gaagggagtg tattttat                                                    18

<210> 957
<211> 18
<212> DNA
<213> Artificial Sequence

<220>
<223> Detection oligonucleotide for PTEN

<400> 957

agagttatcg ttttgttt                                                    18

<210> 958
<211> 18
<212> DNA
<213> Artificial Sequence

<220>
<223> Detection oligonucleotide for PTEN

<400> 958

agagttattg ttttgttt                                                    18

<210> 959
<211> 18
<212> DNA
<213> Artificial Sequence

<220>
<223> Detection oligonucleotide for WT1

<400> 959

tgttatatcg gttagttg                                                    18

<210> 960
<211> 18
<212> DNA
<213> Artificial Sequence

<220>
<223> Detection oligonucleotide for WT1

<400> 960

tgttatattg gttagttg                                                    18

<210> 961
<211> 18
<212> DNA
<213> Artificial Sequence

<220>
<223> Detection oligonucleotide for EGFR

<400> 961

tttgtattcg gagttggg                                                    18

<210> 962
<211> 18
<212> DNA
<213> Artificial Sequence

<220>
<223> Detection oligonucleotide for EGFR

<400> 962

tttgtatttg gagttggg                                                    18

<210> 963
<211> 18
<212> DNA

<213> Artificial Sequence

<220>
<223> Detection oligonucleotide for CSPG2

<400> 963

aagattttcg gttagttt                                                    18

<210> 964
<211> 18
<212> DNA
<213> Artificial Sequence

<220>
<223> Detection oligonucleotide for CSPG2

<400> 964

aagattttg gttagttt                                                     18

<210> 965
<211> 18
<212> DNA
<213> Artificial Sequence

<220>
<223> Detection oligonucleotide for LKB1

<400> 965

tttaggttcg taagttta                                                    18

<210> 966
<211> 18
<212> DNA
<213> Artificial Sequence

<220>
<223> Detection oligonucleotide for LKB1

<400> 966

tttaggtttg taagttta                                                    18

<210> 967
<211> 18
<212> DNA
<213> Artificial Sequence

<220>
<223> Detection oligonucleotide for WT1

<400> 967

tatattggcg aaggttaa                                                    18

<210> 968
<211> 18
<212> DNA
<213> Artificial Sequence

<220>

<223> Detection oligonucleotide for WT1

<400> 968

tatattggtg aaggttaa                                                    18

<210> 969
<211> 18
<212> DNA
<213> Artificial Sequence

<220>
<223> Detection oligonucleotide for ESR1

<400> 969

tggaggttcg ggagttta                                                    18

<210> 970
<211> 18
<212> DNA
<213> Artificial Sequence

<220>
<223> Detection oligonucleotide for ESR1

<400> 970

tggaggtttg ggagttta                                                    18

<210> 971
<211> 18
<212> DNA
<213> Artificial Sequence

<220>
<223> Detection oligonucleotide for APC

<400> 971

tttaatcgta tagtttgt                                                    18

<210> 972
<211> 18
<212> DNA
<213> Artificial Sequence

<220>
<223> Detection oligonucleotide for APC

<400> 972

tttaattgta tagtttgt                                                    18

<210> 973
<211> 18
<212> DNA
<213> Artificial Sequence

<220>
<223> Detection oligonucleotide for IGF2

<400> 973

agtttgaacg atgtaaga                                                    18

<210> 974
<211> 18
<212> DNA
<213> Artificial Sequence

<220>
<223> Detection oligonucleotide for IGF2

<400> 974

agtttgaatg atgtaaga                                                    18

<210> 975
<211> 18
<212> DNA
<213> Artificial Sequence

<220>
<223> Detection oligonucleotide for MYOD1

<400> 975

aattaggtcg gataggag                                                    18

<210> 976
<211> 18
<212> DNA
<213> Artificial Sequence

<220>
<223> Detection oligonucleotide for MYOD1

<400> 976

aattaggttg gataggag                                                    18

<210> 977
<211> 18
<212> DNA
<213> Artificial Sequence

<220>
<223> Detection oligonucleotide for N33

<400> 977

ttggttcggg aaaggtaa                                                    18

<210> 978
<211> 18
<212> DNA
<213> Artificial Sequence

<220>
<223> Detection oligonucleotide for N33

<400> 978

ttggtttggg aaaggtaa                                                    18

<210> 979
<211> 18
<212> DNA
<213> Artificial Sequence

<220>
<223> Detection oligonucleotide for TPEF (=TMEFF2; =HPP1)

<400> 979

atttgtttcg attaattt                                                    18

<210> 980
<211> 18
<212> DNA
<213> Artificial Sequence

<220>
<223> Detection oligonucleotide for TPEF (=TMEFF2; =HPP1)

<400> 980

atttgttttg attaattt                                                    18

<210> 981
<211> 18
<212> DNA
<213> Artificial Sequence

<220>
<223> Detection oligonucleotide for CDKN2a

<400> 981

aatagttacg gtcggagg                                                    18

<210> 982
<211> 18
<212> DNA
<213> Artificial Sequence

<220>
<223> Detection oligonucleotide for CDKN2a

<400> 982

aatagttatg gttggagg                                                    18

<210> 983
<211> 18
<212> DNA
<213> Artificial Sequence

<220>
<223> Detection oligonucleotide for CDH1

<400> 983

agggttatcg cgtttatg                                                    18

<210> 984
<211> 18
<212> DNA

<213> Artificial Sequence

<220>
<223> Detection oligonucleotide for CDH1

<400> 984

agggttattg tgtttatg                                                    18

<210> 985
<211> 18
<212> DNA
<213> Artificial Sequence

<220>
<223> Detection oligonucleotide for APC

<400> 985

tattttggcg ggttgtat                                                    18

<210> 986
<211> 18
<212> DNA
<213> Artificial Sequence

<220>
<223> Detection oligonucleotide for APC

<400> 986

tattttggtg ggttgtat                                                    18

<210> 987
<211> 18
<212> DNA
<213> Artificial Sequence

<220>
<223> Detection oligonucleotide for PTEN

<400> 987

attttgcgtt cgtattta                                                    18

<210> 988
<211> 18
<212> DNA
<213> Artificial Sequence

<220>
<223> Detection oligonucleotide for PTEN

<400> 988

attttgtgtt tgtattta                                                    18

<210> 989
<211> 18
<212> DNA
<213> Artificial Sequence

<220>

<223> Detection oligonucleotide for HLA-F

<400> 989

agttgtttcg tagatatt                                                    18

<210> 990
<211> 18
<212> DNA
<213> Artificial Sequence

<220>
<223> Detection oligonucleotide for HLA-F

<400> 990

agttgttttg tagatatt                                                    18

<210> 991
<211> 18
<212> DNA
<213> Artificial Sequence

<220>
<223> Detection oligonucleotide for RARB

<400> 991

tagtagttcg ggtagggt                                                    18

<210> 992
<211> 18
<212> DNA
<213> Artificial Sequence

<220>
<223> Detection oligonucleotide for RARB

<400> 992

tagtagtttg ggtagggt                                                    18

<210> 993
<211> 18
<212> DNA
<213> Artificial Sequence

<220>
<223> Detection oligonucleotide for N33

<400> 993

atttagttcg ggggagga                                                    18

<210> 994
<211> 18
<212> DNA
<213> Artificial Sequence

<220>
<223> Detection oligonucleotide for N33

<400> 994

atttagtttg ggggagga                                                    18

<210> 995
<211> 18
<212> DNA
<213> Artificial Sequence

<220>
<223> Detection oligonucleotide for RASSF1

<400> 995

tttgtagcgg gtggagta                                                    18

<210> 996
<211> 18
<212> DNA
<213> Artificial Sequence

<220>
<223> Detection oligonucleotide for RASSF1

<400> 996

tttgtagtgg gtggagta                                                    18

<210> 997
<211> 18
<212> DNA
<213> Artificial Sequence

<220>
<223> Detection oligonucleotide for CDKN2a

<400> 997

ggagttttcg gttgattg                                                    18

<210> 998
<211> 18
<212> DNA
<213> Artificial Sequence

<220>
<223> Detection oligonucleotide for CDKN2a

<400> 998
   ggagtttttg gttgattg                                                     18

<210> 999
<211> 18
<212> DNA
<213> Artificial Sequence

<220>
<223> Detection oligonucleotide for S100A2

<400> 999

tatgtatacg agtattgg                                                    18

<210> 1000
<211> 18
<212> DNA
<213> Artificial Sequence

<220>
<223> Detection oligonucleotide for S100A2

<400> 1000

tatgtatatg agtattgg                                                    18

<210> 1001
<211> 18
<212> DNA
<213> Artificial Sequence

<220>
<223> Detection oligonucleotide for MGMT

<400> 1001

agtaggatcg ggattttt                                                    18

<210> 1002
<211> 18
<212> DNA
<213> Artificial Sequence

<220>
<223> Detection oligonucleotide for MGMT

<400> 1002

agtaggattg ggattttt                                                    18

<210> 1003
<211> 18
<212> DNA
<213> Artificial Sequence

<220>
<223> Detection oligonucleotide for CEA

<400> 1003

aagtgttcgc ggttgttt                                                    18

<210> 1004
<211> 18
<212> DNA
<213> Artificial Sequence

<220>
<223> Detection oligonucleotide for CEA

<400> 1004

aagtgtttgt ggttgttt                                                    18

<210> 1005
<211> 18
<212> DNA

<213> Artificial Sequence

<220>
<223> Detection oligonucleotide for GPIb beta

<400> 1005

ggagttcggt cgggtttt                                                    18

<210> 1006
<211> 18
<212> DNA
<213> Artificial Sequence

<220>
<223> Detection oligonucleotide for GPIb beta

<400> 1006

ggagtttggt tgggtttt                                                    18

<210> 1007
<211> 18
<212> DNA
<213> Artificial Sequence

<220>
<223> Detection oligonucleotide for PTGS2

<400> 1007

agttatttcg ttatatgg                                                    18

<210> 1008
<211> 18
<212> DNA
<213> Artificial Sequence

<220>
<223> Detection oligonucleotide for PTGS2

<400> 1008

agttattttg ttatatgg                                                    18

<210> 1009
<211> 18
<212> DNA
<213> Artificial Sequence

<220>
<223> Detection oligonucleotide for MYOD1

<400> 1009

gtgttagtcg tttagggt                                                    18

<210> 1010
<211> 18
<212> DNA
<213> Artificial Sequence

<220>

<223> Detection oligonucleotide for MYOD1

<400> 1010

gtgttagttg tttagggt                                                    18

<210> 1011
<211> 18
<212> DNA
<213> Artificial Sequence

<220>
<223> Detection oligonucleotide for PGR

<400> 1011

ttaagtgtcg gatttgtg                                                    18

<210> 1012
<211> 18
<212> DNA
<213> Artificial Sequence

<220>
<223> Detection oligonucleotide for PGR

<400> 1012

ttaagtgttg gatttgtg                                                    18

<210> 1013
<211> 18
<212> DNA
<213> Artificial Sequence

<220>
<223> Detection oligonucleotide for PCNA

<400> 1013

taaagaggcg gggagatt                                                    18

<210> 1014
<211> 18
<212> DNA
<213> Artificial Sequence

<220>
<223> Detection oligonucleotide for PCNA

<400> 1014

taaagaggtg gggagatt                                                    18

<210> 1015
<211> 18
<212> DNA
<213> Artificial Sequence

<220>
<223> Detection oligonucleotide for MSH3

<400> 1015

agtattttcg tttgagga                                                    18

<210> 1016
<211> 18
<212> DNA
<213> Artificial Sequence

<220>
<223> Detection oligonucleotide for MSH3

<400> 1016

agtattttg tttgagga                                                     18

<210> 1017
<211> 18
<212> DNA
<213> Artificial Sequence

<220>
<223> Detection oligonucleotide for WT1

<400> 1017

tagtgagacg aggttttt                                                    18

<210> 1018
<211> 18
<212> DNA
<213> Artificial Sequence

<220>
<223> Detection oligonucleotide for WT1

<400> 1018

tagtgagatg aggttttt                                                    18

<210> 1019
<211> 18
<212> DNA
<213> Artificial Sequence

<220>
<223> Detection oligonucleotide for MYC

<400> 1019

ttataatgcg agggtttg                                                    18

<210> 1020
<211> 18
<212> DNA
<213> Artificial Sequence

<220>
<223> Detection oligonucleotide for MYC

<400> 1020

ttataatgtg agggtttg                                                    18

<210> 1021
<211> 18
<212> DNA
<213> Artificial Sequence

<220>
<223> Detection oligonucleotide for HIC-1

<400> 1021

ttttagagcg ttagggtt                                                    18

<210> 1022
<211> 18
<212> DNA
<213> Artificial Sequence

<220>
<223> Detection oligonucleotide for HIC-1

<400> 1022

ttttagagtg ttagggtt                                                    18

<210> 1023
<211> 18
<212> DNA
<213> Artificial Sequence

<220>
<223> Detection oligonucleotide for PTGS2

<400> 1023

atatttggcg gaaatttg                                                    18

<210> 1024
<211> 18
<212> DNA
<213> Artificial Sequence

<220>
<223> Detection oligonucleotide for PTGS2

<400> 1024

atatttggtg gaaatttg                                                    18

<210> 1025
<211> 18
<212> DNA
<213> Artificial Sequence

<220>
<223> Detection oligonucleotide for BCL2

<400> 1025

ttttgttacg gtggtgga                                                    18

<210> 1026
<211> 18
<212> DNA

<213> Artificial Sequence

<220>
<223> Detection oligonucleotide for BCL2

<400> 1026

ttttgttatg gtggtgga                                                    18

<210> 1027
<211> 18
<212> DNA
<213> Artificial Sequence

<220>
<223> Detection oligonucleotide for AR

<400> 1027

agaggttgcg ttttagag                                                    18

<210> 1028
<211> 18
<212> DNA
<213> Artificial Sequence

<220>
<223> Detection oligonucleotide for AR

<400> 1028

agaggttgtg ttttagag                                                    18

<210> 1029
<211> 18
<212> DNA
<213> Artificial Sequence

<220>
<223> Detection oligonucleotide for CDKN2a

<400> 1029

gttgttttcg gttggtgt                                                    18

<210> 1030
<211> 18
<212> DNA
<213> Artificial Sequence

<220>
<223> Detection oligonucleotide for CDKN2a

<400> 1030

gttgttttg gttggtgt                                                     18